# EUROPEAN PATENT APPLICATION

(11) **EP 1 396 543 A2**
(43) Date of publication of application: **10.03.2004**
(21) Application number: 03025638.2
(22) Date of filing: 07.07.2000
(51) Int. Cl.: C12N 15/12, C12N 15/10, C12N 15/85, C12N 5/10, C07K 14/47, C07K 16/18, C12Q 1/68

(54) **Primers for synthesizing full length cDNA clones and their use**

(30) Priority: 08.07.1999 JP 19448699; 11.01.2000 JP 2000118774; 02.05.2000 JP 2000183865
(62) Divisional of application: 00114089.6
(71) Applicant: Research Association for Biotechnology, Tokyo 105-0003 (JP)
(72) Inventor: Ota, Toshio, Fujisawa-shi, Kanagawa 251-0042 (JP); Nishikawa, Tetsuo, Tokyo 173-0013 (JP); Isogai, Takao, Ibaraki 300-0303 (JP); Hayashi, Koji, Ichihara-shi, Chiba 299-0125 (JP); Ishii, Shizuko, Kisarazu-shi, Chiba 292-0812 (JP); Kawai, Yuri, Kisarazu-shi, Chiba 292-0812 (JP); Wakamatsu, Ai, Kisarazu-shi, Chiba 292-0014 (JP); Sugiyama, Tomoyasu, Kisarazu-shi, Chiba 292-0045 (JP); Nagai, Keiichi, Higashiyamato-shi, Tokyo 207-0022 (JP); Kojima, Shinichi, Kisara-shi, Chiba 292-0052 (JP); Otsuki, Tetsuji, Kisarazu-shi, Chiba 292-0055 (JP); Koga, Hisashi, Kisarazu-shi, Chiba 292-0055 (JP)
(74) Representative: VOSSIUS & PARTNER

(57) **Abstract**

Primers for synthesizing full length cDNAs and their use are provided. 830 cDNA encoding a human protein has been isolated and nucleotide sequences of 5'-, and 3'-ends of the cDNA have been determined. Furthermore, primers for synthesizing the full length cDNA have been provided to clarify the function of the protein encoded by the cDNA. The full length cDNA of the present invention containing the translation start site provides information useful for analyzing the functions of the protein.

SEQUENCE LISTING

<110> Research Association for Biotechnology

<120> Primers for Synthesis of Full Length cDNAs and there uses.

<130> E2086 EP/1 S3

<140>
<141>

<150> JP 1999-194486
<151> 1999-07-08

<150> JP 2000-118774
<151> 2000-01-11

<150> JP 2000-183765
<151> 2000-05-02

<160> 4484

<170> PatentIn Ver. 2.0

<210> 1
<211> 913
<212> DNA
<213> Homo sapiens

<400> 1
atacccatga ccaacctcct actcctcatt gtacccattc taatcgcaat ggcattccta 60
atgcttaccg aacgaaaaat tctaggctat atacaactac gcaaaggccc caacgttgta 120
ggcccctacg ggctactaca acccttcgct gacgccataa aactcttcac caaagagccc 180
ctaaaacccg ccacatctac catcaccctc tacatcatcg ccccgacctt agctctcacc 240
atcgctcttc tactatgaac ccccctcccc atacccaacc ccctggtcaa cctcaaccta 300
ggcctcctac ttattctagc cacctctagc ctagccgttt actcaatcct ctgatcaggg 360
tgagcatcaa actcaaacta cgccctgatc ggcgcactgc gagcagtagc ccaaacaatc 420
tcatatgaag tcaccctagc catcattcta ctatcaacat tactaataag tggctccttt 480
aacctctcca cccttatcac aacacaagaa cacctctgat tactcctgcc atcatgaccc 540
ttggccataa tatgatttat ctccacacta gcagagacca accgaacccc cttcgacctt 600
gccgaagggg agtccgaact agtctcaggc ttcaacatcg aatacgccgc angccccttc 660
gccctattct tcatagccga atacacaaac attattataa taaacacctt caccactaca 720
atcttctang aacaacatat gacgcactct ccctgaactc tacacaacat attttggnca 780
ccaagaacct tacttctaac ctcctggtct tatgaattcg aacaggatta cccccggant 840
ncggtacgac caactcatac acctcctatg gaaaaacttt cttacactna ccctacatta 900
cttatatgac atg 913

<210> 2
<211> 749
<212> DNA
<213> Homo sapiens

<400> 2
acatcagggt cccgttctac gtgaggggcc ccaacgtgga agccggctgt ctgaatcccc 60
acatcgtcct caacattgac ctggccccca ccatcctgga cattgcaggc ctggacatac 120
ctgcggatat ggacgggaaa tccatcctca agctgctgga cacggagcgg ccggtgaatc 180
ggtttcactt gaaaaagaag atgagggtct ggcgggactc cttcttggtg gagagaggca 240
agctgctaca caagagagac aatgacaagg tggacgccca ggaggagaac tttctgccca 300
agtaccagcg tgtgaaggac ctgtgtcagc gtgctgagta ccagacggcg tgtgagcagc 360
tgggacagaa gtggcagtgt gtggaggacg ccacggggaa gctgaagctg cataagtgca 420
agggccccat gcggctgggc ggcagcagag ccctctccaa cctcgtgccc aagtactacg 480
ggcagggcag cgaggcctgc acctgtgaca gcggggacta caagctcagc ctggccggac 540
gccggaaaaa actcttcaag aagaagtaca aggccagcta tgtccgcagt cgctccatcc 600

<220>
<223> Description of Artificial Sequence: an artificially synthesized primer
sequence

<400> 4444
tgttgacgct gatttaggca                                                    20


<210> 4445
<211> 20
<212> DNA
<213> Artificial Sequence

<220>
<223> Description of Artificial Sequence: an artificially synthesized primer
sequence

<400> 4445
taaacaagac ccagcaccat                                                    20


<210> 4446
<211> 20
<212> DNA
<213> Artificial Sequence

<220>
<223> Description of Artificial Sequence: an artificially synthesized primer
sequence

<400> 4446
taaacaagac ccagcaccat                                                    20


<210> 4447
<211> 20
<212> DNA
<213> Artificial Sequence

<220>
<223> Description of Artificial Sequence: an artificially synthesized primer
sequence

<400> 4447
caaggaggta atagggagat                                                    20


<210> 4448
<211> 20
<212> DNA
<213> Artificial Sequence

<220>
<223> Description of Artificial Sequence: an artificially synthesized primer
sequence

<400> 4448
cgagacaggg tcttggtttg                                                    20


<210> 4449
<211> 20

<212> DNA
<213> Artificial Sequence

<220>
<223> Description of Artificial Sequence: an artificially synthesized primer
sequence

<400> 4449
tttccttcgg ctggtatggc                                                    20


<210> 4450
<211> 20
<212> DNA
<213> Artificial Sequence

<220>
<223> Description of Artificial Sequence: an artificially synthesized primer
sequence

<400> 4450
tcctacgttg tggtgcagtg                                                    20


<210> 4451
<211> 20
<212> DNA
<213> Artificial Sequence

<220>
<223> Description of Artificial Sequence: an artificially synthesized primer
sequence

<400> 4451
agcacccctt attggaagat                                                    20


<210> 4452
<211> 20
<212> DNA
<213> Artificial Sequence

<220>
<223> Description of Artificial Sequence: an artificially synthesized primer
sequence

<400> 4452
gatggttact cctctttggt                                                    20


<210> 4453
<211> 20
<212> DNA
<213> Artificial Sequence

<220>
<223> Description of Artificial Sequence: an artificially synthesized primer
sequence

<400> 4453
tgctgatgcc tctgttccct                                                    20

<210> 4454
<211> 20
<212> DNA
<213> Artificial Sequence

<220>
<223> Description of Artificial Sequence: an artificially synthesized primer
sequence

<400> 4454
gaaacaacca agcaccccat                                                    20


<210> 4455
<211> 20
<212> DNA
<213> Artificial Sequence

<220>
<223> Description of Artificial Sequence: an artificially synthesized primer
sequence

<400> 4455
ataagggcag ctgggaagtg                                                    20


<210> 4456
<211> 20
<212> DNA
<213> Artificial Sequence

<220>
<223> Description of Artificial Sequence: an artificially synthesized primer
sequence

<400> 4456
tgttagtagc aggaggaagc                                                    20


<210> 4457
<211> 20
<212> DNA
<213> Artificial Sequence

<220>
<223> Description of Artificial Sequence: an artificially synthesized primer
sequence

<400> 4457
   acagagttca gtccccttta                                                     20


<210> 4458
<211> 20
<212> DNA
<213> Artificial Sequence

<220>
<223> Description of Artificial Sequence: an artificially synthesized primer
sequence

<400> 4458
gttagaggac actggcatca                                                    20

<210> 4459
<211> 20
<212> DNA
<213> Artificial Sequence

<220>
<223> Description of Artificial Sequence: an artificially synthesized primer
sequence

<400> 4459
actgacttga ggaataagcc                                                    20


<210> 4460
<211> 20
<212> DNA
<213> Artificial Sequence

<220>
<223> Description of Artificial Sequence: an artificially synthesized primer
sequence

<400> 4460
agagtgagag catgggaggt                                                    20


<210> 4461
<211> 20
<212> DNA
<213> Artificial Sequence

<220>
<223> Description of Artificial Sequence: an artificially synthesized primer
sequence

<400> 4461
ggcacagaca ccatccttga                                                    20


<210> 4462
<211> 20
<212> DNA
<213> Artificial Sequence

<220>
<223> Description of Artificial Sequence: an artificially synthesized primer
sequence

<400> 4462
acaaatgagc ctgaaaagtc                                                    20


<210> 4463
<211> 20
<212> DNA
<213> Artificial Sequence

<220>
<223> Description of Artificial Sequence: an artificially synthesized primer
sequence

<400> 4463
tggtcctcac tgccttcaaa                                                    20


<210> 4464
<211> 20
<212> DNA
<213> Artificial Sequence

<220>
<223> Description of Artificial Sequence: an artificially synthesized primer
sequence

<400> 4464
agtgccctca aagctccagt                                                    20


<210> 4465
<211> 20
<212> DNA
<213> Artificial Sequence

<220>
<223> Description of Artificial Sequence: an artificially synthesized primer
sequence

<400> 4465
acgaaagcac tcaaatgtca                                                    20


<210> 4466
<211> 20
<212> DNA
<213> Artificial Sequence

<220>
<223> Description of Artificial Sequence: an artificially synthesized primer
sequence

<400> 4466
gaatgaaatg tggttgagca                                                    20


<210> 4467
<211> 20
<212> DNA
<213> Artificial Sequence

<220>
<223> Description of Artificial Sequence: an artificially synthesized primer
sequence

<400> 4467
aatgattcgg ggcagcagga                                                    20


<210> 4468
<211> 20
<212> DNA
<213> Artificial Sequence

<220>
<223> Description of Artificial Sequence: an artificially synthesized primer

sequence

<400> 4468
gagagagaca cacacagaaa                                                    20


<210> 4469
<211> 20
<212> DNA
<213> Artificial Sequence

<220>
<223> Description of Artificial Sequence: an artificially synthesized primer
sequence

<400> 4469
agttttatac cagcattggc                                                    20


<210> 4470
<211> 20
<212> DNA
<213> Artificial Sequence

<220>
<223> Description of Artificial Sequence: an artificially synthesized primer
sequence

<400> 4470
ggtgtagaag taaaatggga                                                    20


<210> 4471
<211> 20
<212> DNA
<213> Artificial Sequence

<220>
<223> Description of Artificial Sequence: an artificially synthesized primer
sequence

<400> 4471
gattgtgttc tcttactcct                                                    20


<210> 4472
<211> 20
<212> DNA
<213> Artificial Sequence

<220>
<223> Description of Artificial Sequence: an artificially synthesized primer
sequence

<400> 4472
gctccacctg acgttcttta                                                    20


<210> 4473
<211> 20
<212> DNA
<213> Artificial Sequence

<220>
<223> Description of Artificial Sequence: an artificially synthesized primer
sequence

<400> 4473
gtctcccata tcgctgtctt                                                    20


<210> 4474
<211> 20
<212> DNA
<213> Artificial Sequence

<220>
<223> Description of Artificial Sequence: an artificially synthesized primer
sequence

<400> 4474
cactttctaa taacccttgg                                                    20


<210> 4475
<211> 20
<212> DNA
<213> Artificial Sequence

<220>
<223> Description of Artificial Sequence: an artificially synthesized primer
sequence

<400> 4475
ttgggattgg aaaccgcatt                                                    20


<210> 4476
<211> 20
<212> DNA
<213> Artificial Sequence

<220>
<223> Description of Artificial Sequence: an artificially synthesized primer
sequence

<400> 4476
agaaaccact gaggcccaag                                                    20


<210> 4477
<211> 20
<212> DNA
<213> Artificial Sequence

<220>
<223> Description of Artificial Sequence: an artificially synthesized primer
sequence

<400> 4477
cagaagcaga agcaggagca                                                    20


<210> 4478
<211> 20
<212> DNA

<213> Artificial Sequence

<220>
<223> Description of Artificial Sequence: an artificially synthesized primer
sequence

<400> 4478
atttactgcg atttctcctg                                                    20


<210> 4479
<211> 20
<212> DNA
<213> Artificial Sequence

<220>
<223> Description of Artificial Sequence: an artificially synthesized primer
sequence

<400> 4479
gagtttcttt gctagttcca                                                    20


<210> 4480
<211> 20
<212> DNA
<213> Artificial Sequence

<220>
<223> Description of Artificial Sequence: an artificially synthesized primer
sequence

<400> 4480
atatttgtgt ttgccttggg                                                    20


<210> 4481
<211> 20
<212> DNA
<213> Artificial Sequence

<220>
<223> Description of Artificial Sequence: an artificially synthesized primer
sequence

<400> 4481
ggatggctgg gtcaaatggt                                                    20


<210> 4482
<211> 20
<212> DNA
<213> Artificial Sequence

<220>
<223> Description of Artificial Sequence: an artificially synthesized primer
sequence

<400> 4482
aatgatggct agggtgactt                                                    20


<210> 4483

<211> 20
<212> DNA
<213> Artificial Sequence

<220>
<223> Description of Artificial Sequence: an artificially synthesized primer
sequence

<400> 4483
tcttcccaca ttcgttacac                                                    20


<210> 4484
<211> 20
<212> DNA
<213> Artificial Sequence

<220>
<223> Description of Artificial Sequence: an artificially synthesized primer
sequence

<400> 4484
agggagtaga tgttggtaaa                                                    20

1


46

## Description

### FIELD OF THE INVENTION

The present invention relates to a polynucleotide encoding a novel protein, a protein encoded by the polynucleotide, and new uses of these.

### BACKGROUND OF THE INVENTION

Currently, the sequencing projects, the determination and analysis of the genomic DNA of various living organisms have been in progress all over the world. The whole genomic sequences of more than 10 species of prokaryotes, a lower eukaryote, yeast, and a multicellular eukaryote, C. elegans are already determined. As to human genome, which is supposed to be composed of three thousand million base pairs, the world wide cooperative projects have been under way to analyze it, and the whole structure is predicted to be determined by the years 2002-2003. The aim of the determination of genomic sequence is to reveal the functions of all genes and their regulation and to understand living organisms as a network of interactions between genes, proteins, cells or individuals through deducing the information in a genome, which is a blueprint of the highly complicated living organisms. To understand living organisms by utilizing the genomic information from various species is not only important as an academic subject, but also socially significant from the viewpoint of industrial application.

However, determination of genomic sequences itself cannnot identify the functions of all genes. For example, as for yeast, only the function of approximately half of the 6000 genes, which is predicted based on the genomic sequence, was able to be deduced. As for human, the number of the genes is predicted to be approximately one hundred thousand. Therefore, it is desirable to establish "a high throughput analysis system of the gene functions" which allows us to identify rapidly and efficiently the functions of vast amounts of the genes obtained by the genomic sequencing.

Many genes in the eukaryotic genome are split by introns into multiple exons. Thus, it is difficult to predict correctly the structure of encoded protein solely based on genomic information. In contrast, cDNA, which is produced from mRNA that lacks introns, encodes a protein as a single continuous amino acid sequence and allows us to identify the primary structure of the protein easily. In human cDNA research, to date, more than one million ESTs (Expression Sequence Tags) are publicly available, and the ESTs presumably cover not less than 80% of all human genes.

The information of ESTs is utilized for analyzing the structure of human genome, or for predicting the exon-regions of genomic sequences or their expression profile. However, many human ESTs have been derived from proximal regions to the 3'-end of cDNA, and information around the 5'-end of mRNA is extremely little. Among these human cDNAs, the number of the corresponding mRNAs whose encoding protein sequences are deduced is approximately 7000, and further, the number of full-length therein is only 5500. Thus, even including cDNA registered as EST, the percentage of human cDNA obtained so far is estimated to be 10-15% of all the genes.

It is possible to identify the transcription start site of mRNA on the genomic sequence based on the 5'-end sequence of a full-length cDNA, and to analyze factors involved in the stability of mRNA that is contained in the cDNA, or in its regulation of expression at the translation stage. Also, since a full-length cDNA contains ATG, the translation start site, in the 5'-region, it can be translated into a protein in a correct frame. Therefore, it is possible to produce a large amount of the protein encoded by the cDNA or to analyze biological activity of the expressed protein by utilizing an appropriate expression system. Thus, analysis of a full-length cDNA provides valuable information which complements the information from genome sequencing. Also, full-length cDNA clones that can be expressed are extremely valuable in empirical analysis of gene function and in industrial application.

In particular, human secretory proteins or membrane proteins are would be useful by itself as a medicine like tissue plasminogen activator (TPA), or as a target of medicines like membrane receptors. In addition, genes for signal transduction-associated proteins (protein kinases, etc.), glycoprotein-associated proteins, transcription-associated proteins, and disease-associated proteins form a gene group rich in genes whose relationships to human diseases have been elucidated.

Therefore, it has great significance to isolate novel full-length cDNA clones of human, only few of which has been isolated. Especially, isolation of a novel cDNA clone encoding a secretory protein or membrane protein is desired since the protein itself would be useful as a medicine, and also the clones potentially include a gene associated with diseases. In addition, genes encoding proteins that are associated with signal transduction, glycoprotein, transcription, or diseases are expected to be useful as target molecules for therapy, or as medicines themselves. These genes form a gene group predicted to be strongly associated with diseases. Thus, identification of the full-length cDNA clones encoding those proteins has great significance.

### SUMMARY OF THE INVENTION

An objective of the present invention is to provide a primer that enables synthesizing polynucleotide from human, the resulting polynucleotide or its clone, and a protein encoded by the polynucleotide.

The inventors have developed a method for efficiently cloning a human full-length cDNA that is predicted by the ATGpr etc. to be a full-length cDNA clone, from a full-length-enriched cDNA library that is synthesized by the oligo-capping method. Then, the inventors determined the nucleotide sequence of the obtained cDNA clones from both 5'- and 3'- ends. By utilizing the sequences, the inventors selected clones that were expected to contain a signal by the PSORT (Nakai K. and Kanehisa M. (1992) Genomics 14: 897-911), and obtained clones that contain a cDNA encoding a secretory protein or membrane protein. Moreover, the inventors specifically selected full-length cDNA clones that encode secretory or membrane proteins, signal transduction-associated proteins, glycoprotein-associated proteins, transcription-associated proteins, or disease-associated proteins from clones homologous to the clones in the SwissProt (http://www.ebi.ac.uk/ebi_docsSwissProt_db/swisshome.html) according to the keywords of SwissProt.

The full-length cDNA clones of the present invention have high fullness ratio since these were obtained by the combination of (1) construction of a full-length-enriched cDNA library that is synthesized by the oligo-capping method, and (2) a system in which fullness ratio is evaluated from the nucleotide sequence of the 5'-end (in this system, clones are selected based on the estimation by the ATGpr, following the removal of sequences judged not to be full-length when compared with ESTs). However, the primers of the present invention enable obtaining full-length cDNA easily without any special methods mentioned above.

Homology analysis in which the analysis is carried out against a non-full-length cDNA fragment to postulate the function of a protein encoded by said fragment, is being commonly performed. However, since such analysis is based on the information of the fragment, it is not clear as to whether this fragment corresponds to a part that is functionally important in the protein. In other words, the reliability of the homology analysis based on the information of a fragment is doubtful, as information relating to the structure of the whole protein is not available. However, the homology analysis of the present invention is conducted based on the information of a full-length cDNA comprising the whole coding region of the cDNA, and therefore, the homology of various portions of the protein can be analyzed. Hence, the reliability of the homology analysis has been dramatically improved in the present invention.

The inventors completed the invention by finding that it is possible to synthesize a novel full-length cDNA by using the combination of a primer that is designed based on the nucleotide sequence of the 5'-ends of the selected full-length cDNA clones and any of an oligo-dT primer or a 3'-primer that is designed based on the nucleotide sequence of the 3'-ends of the selected clones.

Thus, the present invention relates to primers described below, a method for synthesizing a polynucleotide using the primers, and polynucleotides obtained by the method.

First, the present invention relates to
(1) use of an oligonucleotide as a primer for synthesizing the polynucleotide comprising the nucleotide sequence set forth in any one of SEQ ID NOs: 1-829 and 2545, or the complementary strand thereof, wherein said oligonucleotide is complementary to said polynucleotide or the complementary strand thereof and comprises at least 15 nucleotides;
(2) a primer set for synthesizing polynucleotides, the primer set comprising an oligo-dT primer and an oligonucleotide complementary to the complementary strand of the polynucleotide comprising the nucleotide sequence set forth in any one of SEQ ID NOs: 1-829 and 2545, wherein said oligonucleotide comprises at least 15 nucleotides; and
(3) A primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence / 3'-end nucleotide sequence is selected from the combinations of 5'-end nucleotide sequence / 3'-end nucleotide sequence set forth in the SEQ ID NOs in Table 1.

Table 1 shows names of clones obtained in the examples described later, comprising the polynucleotide of the present invention (830 clones), names of nucleotide sequences at the 5'-end and 3'-end of the full-length cDNA, and their corresponding SEQ ID NOs. A blank indicates that the of the 3'-end sequence corresponding to the 5'-end sequence has not been determined the same clone.

The SEQ ID NO of a 5'-end sequence is shown on the right side of the name of the 5'-end sequence, and the SEQ ID NO of a 3'-end sequence is shown on the right side of the name of the 3'-end sequence.

**Table 1**

| Correspondence between names of clone and the sequence name, and the SEQ ID. | | | | |
|---|---|---|---|---|
| Name of clone | Name of 5'-sequence | SEQ ID | Name of 3'-sequence | SEQ ID |
| BNGH41000020 | F-BNGH41000020 | 1 | | |
| BNGH41000087 | F-BNGH41000087 | 2 | | |
| BNGH41000091 | F-BNGH41000091 | 3 | | |
| HEMBA1000006 | F-HEMBA1000006 | 4 | R-HEMBA1000006 | 830 |
| HEMBA1000121 | F-HEMBA1000121 | 5 | R-HEMBA1000121 | 831 |
| HEMBA1000128 | F-HEMBA1000128 | 6 | R-HEMBA1000128 | 832 |
| HEMBA1000275 | F-HEMBA1000275 | 7 | R-HEMBA1000275 | 833 |
| HEMBA1000300 | F-HEMBA1000300 | 8 | R-HEMBA1000300 | 834 |
| HEMBA1000349 | F-HEMBA1000349 | 9 | R-nnnnnnnnnnnn | 835 |
| HEMBA1000443 | F-HEMBA1000443 | 10 | | |
| HEMBA1000462 | F-HEMBA1000462 | 11 | R-HEMBA1000462 | 836 |
| HEMBA1000477 | F-HEMBA1000477 | 12 | R-HEMBA1000477 | 837 |
| HEMBA1000590 | F-HEMBA1000590 | 13 | R-HEMBA1000590 | 838 |
| HEMBA1000634 | F-HEMBA1000634 | 14 | R-HEMBA1000634 | 839 |
| HEMBA1000671 | F-HEMBA1000671 | 15 | R-HEMBA1000671 | 840 |
| HEMBA1000713 | F-HEMBA1000713 | 16 | R-HEMBA1000713 | 841 |
| HEMBA1000732 | F-HEMBA1000732 | 17 | R-HEMBA1000732 | 842 |
| HEMBA1000745 | F-HEMBA1000745 | 18 | R-nnnnnnnnnnnn | 843 |
| HEMBA1000835 | F-HEMBA1000835 | 19 | | |
| HEMBA1000875 | F-HEMBA1000875 | 20 | R-HEMBA1000875 | 844 |
| HEMBA1000907 | F-HEMBA1000907 | 21 | | |
| HEMBA1000940 | F-HEMBA1000940 | 22 | R-HEMBA1000940 | 845 |
| HEMBA1000962 | F-HEMBA1000962 | 23 | R-HEMBA1000962 | 846 |
| HEMBA1001184 | F-HEMBA1001184 | 24 | R-HEMBA1001184 | 847 |
| HEMBA1001221 | F-HEMBA1001221 | 25 | R-HEMBA1001221 | 848 |
| HEMBA1001228 | F-HEMBA1001228 | 26 | R-HEMBA1001228 | 849 |
| HEMBA1001272 | F-HEMBA1001272 | 27 | R-HEMBA1001272 | 850 |
| HEMBA1001296 | F-HEMBA1001296 | 28 | R-HEMBA1001296 | 851 |
| HEMBA1001297 | F-HEMBA1001297 | 29 | R-HEMBA1001297 | 852 |
| HEMBA1001390 | F-HEMBA1001390 | 30 | R-HEMBA1001390 | 853 |
| HEMBA1001563 | F-HEMBA1001563 | 31 | R-HEMBA1001563 | 854 |
| HEMBA1001621 | F-HEMBA1001621 | 32 | R-HEMBA1001621 | 855 |
| HEMBA1001878 | F-HEMBA1001878 | 33 | R-HEMBA1001878 | 856 |
| HEMBA1001886 | F-HEMBA1001886 | 34 | R-HEMBA1001886 | 857 |
| HEMBA1002048 | F-HEMBA1002048 | 35 | R-HEMBA1002048 | 858 |
| HEMBA1002131 | F-HEMBA1002131 | 36 | R-HEMBA1002131 | 859 |
| HEMBA1002163 | F-HEMBA1002163 | 37 | R-HEMBA1002163 | 860 |
| HEMBA1002164 | F-HEMBA1002164 | 38 | | |
| HEMBA1002167 | F-HEMBA1002167 | 39 | R-HEMBA1002167 | 861 |
| HEMBA1002178 | F-HEMBA1002178 | 40 | R-HEMBA1002178 | 862 |
| HEMBA1002195 | F-HEMBA1002195 | 41 | R-HEMBA1002195 | 863 |
| HEMBA1002227 | F-HEMBA1002227 | 42 | R-HEMBA1002227 | 864 |
| HEMBA1002239 | F-HEMBA1002239 | 43 | | |
| HEMBA1002316 | F-HEMBA1002316 | 44 | R-HEMBA1002316 | 865 |
| HEMBA1002420 | F-HEMBA1002420 | 45 | R-HEMBA1002420 | 866 |
| HEMBA1002421 | F-HEMBA1002421 | 46 | R-HEMBA1002421 | 867 |
| HEMBA1002524 | F-HEMBA1002524 | 47 | R-HEMBA1002524 | 868 |
| HEMBA1002551 | F-HEMBA1002551 | 48 | R-HEMBA1002551 | 869 |
| HEMBA1002767 | F-HEMBA1002767 | 49 | R-HEMBA1002767 | 870 |
| HEMBA1002985 | F-HEMBA1002985 | 50 | R-HEMBA1002985 | 871 |
| HEMBA1002992 | F-HEMBA1002992 | 51 | | |
| HEMBA1003047 | F-HEMBA1003047 | 52 | R-HEMBA1003047 | 872 |
| HEMBA1003072 | F-HEMBA1003072 | 53 | R-HEMBA1003072 | 873 |
| HEMBA1003101 | F-HEMBA1003101 | 54 | R-HEMBA1003101 | 874 |
| HEMBA1003120 | F-HEMBA1003120 | 55 | R-HEMBA1003120 | 875 |
| HEMBA1003230 | F-HEMBA1003230 | 56 | R-HEMBA1003230 | 876 |
| HEMBA1003294 | F-HEMBA1003294 | 57 | R-HEMBA1003294 | 877 |
| HEMBA1003315 | F-HEMBA1003315 | 58 | R-HEMBA1003315 | 878 |
| HEMBA1003392 | F-HEMBA1003392 | 59 | R-HEMBA1003392 | 879 |
| HEMBA1003399 | F-HEMBA1003399 | 60 | R-HEMBA1003399 | 880 |
| HEMBA1003487 | F-HEMBA1003487 | 61 | R-HEMBA1003487 | 881 |
| HEMBA1003497 | F-HEMBA1003497 | 62 | R-HEMBA1003497 | 882 |
| HEMBA1003530 | F-HEMBA1003530 | 63 | R-HEMBA1003530 | 883 |
| HEMBA1003602 | F-HEMBA1003602 | 64 | R-HEMBA1003602 | 884 |
| HEMBA1003732 | F-HEMBA1003732 | 65 | R-HEMBA1003732 | 885 |
| HEMBA1003945 | F-HEMBA1003945 | 66 | R-HEMBA1003945 | 886 |
| HEMBA1004007 | F-HEMBA1004007 | 67 | R-HEMBA1004007 | 887 |
| HEMBA1004067 | F-HEMBA1004067 | 68 | | |
| HEMBA1004085 | F-HEMBA1004085 | 69 | R-HEMBA1004085 | 888 |
| HEMBA1004110 | F-HEMBA1004110 | 70 | R-nnnnnnnnnnnn | 889 |
| HEMBA1004250 | F-HEMBA1004250 | 71 | R-HEMBA1004250 | 890 |
| HEMBA1004391 | F-HEMBA1004391 | 72 | R-HEMBA1004391 | 891 |
| HEMBA1004444 | F-HEMBA1004444 | 73 | R-HEMBA1004444 | 892 |
| HEMBA1004454 | F-HEMBA1004454 | 74 | R-HEMBA1004454 | 893 |
| HEMBA1004505 | F-HEMBA1004505 | 75 | R-HEMBA1004505 | 894 |
| HEMBA1004785 | F-HEMBA1004785 | 76 | R-HEMBA1004785 | 895 |
| HEMBA1004797 | F-HEMBA1004797 | 77 | R-HEMBA1004797 | 896 |
| HEMBA1004952 | F-HEMBA1004952 | 78 | R-HEMBA1004952 | 897 |
| HEMBA1004971 | F-HEMBA1004971 | 79 | R-HEMBA1004971 | 898 |
| HEMBA1004982 | F-HEMBA1004982 | 80 | R-HEMBA1004982 | 899 |
| HEMBA1005070 | F-HEMBA1005070 | 81 | R-HEMBA1005070 | 900 |
| HEMBA1005084 | F-HEMBA1005084 | 82 | R-HEMBA1005084 | 901 |
| HEMBA1005145 | F-HEMBA1005145 | 83 | R-HEMBA1005145 | 902 |
| HEMBA1005230 | F-HEMBA1005230 | 84 | R-HEMBA1005230 | 903 |
| HEMBA1005246 | F-HEMBA1005246 | 85 | R-HEMBA1005246 | 904 |
| HEMBA1005267 | F-HEMBA1005267 | 86 | R-HEMBA1005267 | 905 |
| HEMBA1005337 | F-HEMBA1005337 | 87 | R-HEMBA1005337 | 906 |
| HEMBA1005430 | F-HEMBA1005430 | 88 | R-HEMBA1005430 | 907 |
| HEMBA1005449 | F-HEMBA1005449 | 89 | R-HEMBA1005449 | 908 |
| HEMBA1005489 | F-HEMBA1005489 | 90 | R-HEMBA1005489 | 909 |
| HEMBA1005522 | F-HEMBA1005522 | 91 | R-HEMBA1005522 | 910 |
| HEMBA1005545 | F-HEMBA1005545 | 92 | R-HEMBA1005545 | 911 |
| HEMBA1005698 | F-HEMBA1005698 | 93 | R-HEMBA1005698 | 912 |
| HEMBA1005913 | F-HEMBA1005913 | 94 | R-HEMBA1005913 | 913 |
| HEMBA1005929 | F-HEMBA1005929 | 95 | R-HEMBA1005929 | 914 |
| HEMBA1005945 | F-HEMBA1005945 | 96 | R-HEMBA1005945 | 915 |
| HEMBA1006016 | F-HEMBA1006016 | 97 | R-HEMBA1006016 | 916 |
| HEMBA1006171 | F-HEMBA1006171 | 98 | R-HEMBA1006171 | 917 |
| HEMBA1006276 | F-HEMBA1006276 | 99 | R-HEMBA1006276 | 918 |
| HEMBA1006299 | F-HEMBA1006299 | 100 | R-HEMBA1006299 | 919 |
| HEMBA1006311 | F-HEMBA1006311 | 101 | R-HEMBA1006311 | 920 |
| HEMBA1006335 | F-HEMBA1006335 | 102 | R-HEMBA1006335 | 921 |
| HEMBA1006357 | F-HEMBA1006357 | 103 | R-HEMBA1006357 | 922 |
| HEMBA1006430 | F-HEMBA1006430 | 104 | R-HEMBA1006430 | 923 |
| HEMBA1006482 | F-HEMBA1006482 | 105 | R-HEMBA1006482 | 924 |
| HEMBA1006517 | F-HEMBA1006517 | 106 | R-HEMBA1006517 | 925 |
| HEMBA1006544 | F-HEMBA1006544 | 107 | R-HEMBA1006544 | 926 |
| HEMBA1006572 | F-HEMBA1006572 | 108 | R-HEMBA1006572 | 927 |
| HEMBA1006658 | F-HEMBA1006658 | 109 | R-HEMBA1006658 | 928 |
| HEMBA1006707 | F-HEMBA1006707 | 110 | R-HEMBA1006707 | 929 |
| HEMBA1006724 | F-HEMBA1006724 | 111 | R-HEMBA1006724 | 930 |
| HEMBA1006749 | F-HEMBA1006749 | 112 | R-HEMBA1006749 | 931 |
| HEMBA1006770 | F-HEMBA1006770 | 113 | R-HEMBA1006770 | 932 |
| HEMBA1006902 | F-HEMBA1006902 | 114 | R-HEMBA1006902 | 933 |
| HEMBA1006912 | F-HEMBA1006912 | 115 | R-HEMBA1006912 | 934 |
| HEMBA1006916 | F-HEMBA1006916 | 116 | R-HEMBA1006916 | 935 |
| HEMBA1006960 | F-HEMBA1006960 | 117 | R-HEMBA1006960 | 936 |
| HEMBA1007013 | F-HEMBA1007013 | 118 | R-HEMBA1007013 | 937 |
| HEMBA1007057 | F-HEMBA1007057 | 119 | R-HEMBA1007057 | 938 |
| HEMBA1007063 | F-HEMBA1007063 | 120 | R-HEMBA1007063 | 939 |
| HEMBA1007226 | F-HEMBA1007226 | 121 | | |
| HEMBA1007241 | F-HEMBA1007241 | 122 | R-HEMBA1007241 | 940 |
| HEMBA1007291 | F-HEMBA1007291 | 123 | R-HEMBA1007291 | 941 |
| HEMBA1007332 | F-HEMBA1007332 | 124 | R-HEMBA1007332 | 942 |
| HEMBB1000106 | F-HEMBB1000106 | 125 | R-HEMBB1000106 | 943 |
| HEMBB1000276 | F-HEMBB1000276 | 126 | R-HEMBB1000276 | 944 |
| HEMBB1000309 | F-HEMBB1000309 | 127 | R-HEMBB1000309 | 945 |
| HEMBB1000407 | F-HEMBB1000407 | 128 | R-HEMBB1000407 | 946 |
| HEMBB1000447 | F-HEMBB1000447 | 129 | R-HEMBB1000447 | 947 |
| HEMBB1000542 | F-HEMBB1000542 | 130 | R-HEMBB1000542 | 948 |
| HEMBB1000567 | F-HEMBB1000567 | 131 | R-HEMBB1000567 | 949 |
| HEMBB1000642 | F-HEMBB1000642 | 132 | R-HEMBB1000642 | 950 |
| HEMBB1000668 | F-HEMBB1000668 | 133 | R-HEMBB1000668 | 951 |
| HEMBB1000679 | F-HEMBB1000679 | 134 | R-HEMBB1000679 | 952 |
| HEMBB1000881 | F-HEMBB1000881 | 135 | R-HEMBB1000881 | 953 |
| HEMBB1000905 | F-HEMBB1000905 | 136 | R-HEMBB1000905 | 954 |
| HEMBB1001026 | F-HEMBB1001026 | 137 | R-HEMBB1001026 | 955 |
| HEMBB1001048 | F-HEMBB1001048 | 138 | R-HEMBB1001048 | 956 |
| HEMBB1001200 | F-HEMBB1001200 | 139 | R-HEMBB1001200 | 957 |
| HEMBB1001407 | F-HEMBB1001407 | 140 | R-HEMBB1001407 | 958 |
| HEMBB1001530 | F-HEMBB1001530 | 141 | R-HEMBB1001530 | 959 |
| HEMBB1001547 | F-HEMBB1001547 | 142 | R-HEMBB1001547 | 960 |
| HEMBB1001573 | F-HEMBB1001573 | 143 | R-HEMBB1001573 | 961 |
| HEMBB1001847 | F-HEMBB1001847 | 144 | R-HEMBB1001847 | 962 |
| HEMBB1001959 | F-HEMBB1001959 | 145 | R-HEMBB1001959 | 963 |
| HEMBB1001978 | F-HEMBB1001978 | 146 | R-HEMBB1001978 | 964 |
| HEMBB1002039 | F-HEMBB1002039 | 147 | R-HEMBB1002039 | 965 |
| HEMBB1002041 | F-HEMBB1002041 | 148 | R-HEMBB1002041 | 966 |
| HEMBB1002051 | F-HEMBB1002051 | 149 | R-HEMBB1002051 | 967 |
| HEMBB1002120 | F-HEMBB1002120 | 150 | R-HEMBB1002120 | 968 |
| HEMBB1002162 | F-HEMBB1002162 | 151 | R-HEMBB1002162 | 969 |
| HEMBB1002228 | F-HEMBB1002228 | 152 | R-HEMBB1002228 | 970 |
| HEMBB1002245 | F-HEMBB1002245 | 153 | R-HEMBB1002245 | 971 |
| HEMBB1002302 | F-HEMBB1002302 | 154 | R-HEMBB1002302 | 972 |
| HEMBB1002427 | F-HEMBB1002427 | 155 | R-HEMBB1002427 | 973 |
| HEMBB1002465 | F-HEMBB1002465 | 156 | R-HEMBB1002465 | 974 |
| HEMBB1002661 | F-HEMBB1002661 | 157 | R-HEMBB1002661 | 975 |
| HEMBB1002663 | F-HEMBB1002663 | 158 | R-HEMBB1002663 | 976 |
| HEMBB1002693 | F-HEMBB1002693 | 159 | R-HEMBB1002693 | 977 |
| MAMMA1000046 | F-MAMMA1000046 | 160 | R-MAMMA1000046 | 978 |
| MAMMA1000102 | F-MAMMA1000102 | 161 | R-MAMMA1000102 | 979 |
| MAMMA1000106 | F-MAMMA1000106 | 162 | R-MAMMA1000106 | 980 |
| MAMMA1000118 | F-MAMMA1000118 | 163 | R-MAMMA1000118 | 981 |
| MAMMA1000141 | F-MAMMA1000141 | 164 | R-MAMMA1000141 | 982 |
| MAMMA1000204 | F-MAMMA1000204 | 165 | R-MAMMA1000204 | 983 |
| MAMMA1000226 | F-MAMMA1000226 | 166 | R-MAMMA1000226 | 984 |
| MAMMA1000403 | F-MAMMA1000403 | 167 | R-MAMMA1000403 | 985 |
| MAMMA1000449 | F-MAMMA1000449 | 168 | R-MAMMA1000449 | 986 |
| MAMMA1000457 | F-MAMMA1000457 | 169 | R-MAMMA1000457 | 987 |
| MAMMA1000473 | F-MAMMA1000473 | 170 | R-MAMMA1000473 | 988 |
| MAMMA1000496 | F-MAMMA1000496 | 171 | R-MAMMA1000496 | 989 |
| MAMMA1000528 | F-MAMMA1000528 | 172 | R-MAMMA1000528 | 990 |
| MAMMA1000591 | F-MAMMA1000591 | 173 | R-MAMMA1000591 | 991 |
| MAMMA1000614 | F-MAMMA1000614 | 174 | R-MAMMA1000614 | 992 |
| MAMMA1000652 | F-MAMMA1000652 | 175 | R-MAMMA1000652 | 993 |
| MAMMA1000681 | F-MAMMA1000681 | 176 | R-MAMMA1000681 | 994 |
| MAMMA1000706 | F-MAMMA1000706 | 177 | R-MAMMA1000706 | 995 |
| MAMMA1000788 | F-MAMMA1000788 | 178 | R-MAMMA1000788 | 996 |
| MAMMA1000810 | F-MAMMA1000810 | 179 | R-MAMMA1000810 | 997 |
| MAMMA1000814 | F-MAMMA1000814 | 180 | R-MAMMA1000814 | 998 |
| MAMMA1000881 | F-MAMMA1000881 | 181 | R-MAMMA1000881 | 999 |
| MAMMA1000986 | F-MAMMA1000986 | 182 | R-MAMMA1000986 | 1000 |
| MAMMA1000994 | F-MAMMA1000994 | 183 | R-MAMMA1000994 | 1001 |
| MAMMA1001043 | F-MAMMA1001043 | 184 | R-MAMMA1001043 | 1002 |
| MAMMA1001066 | F-MAMMA1001066 | 185 | R-MAMMA1001066 | 1003 |
| MAMMA1001094 | F-MAMMA1001094 | 186 | R-MAMMA1001094 | 1004 |
| MAMMA1001141 | F-MAMMA1001141 | 187 | R-MAMMA1001141 | 1005 |
| MAMMA1001150 | F-MAMMA1001150 | 188 | R-MAMMA1001150 | 1006 |
| MAMMA1001237 | F-MAMMA1001237 | 189 | R-MAMMA1001237 | 1007 |
| MAMMA1001284 | F-MAMMA1001284 | 190 | R-MAMMA1001284 | 1008 |
| MAMMA1001310 | F-MAMMA1001310 | 191 | R-MAMMA1001310 | 1009 |
| MAMMA1001344 | F-MAMMA1001344 | 192 | | |
| MAMMA1001418 | F-MAMMA1001418 | 193 | R-MAMMA1001418 | 1010 |
| MAMMA1001532 | F-MAMMA1001532 | 194 | R-MAMMA1001532 | 1011 |
| MAMMA1001609 | F-MAMMA1001609 | 195 | R-MAMMA1001609 | 1012 |
| MAMMA1001615 | F-MAMMA1001615 | 196 | R-MAMMA1001615 | 1013 |
| MAMMA1001623 | F-MAMMA1001623 | 197 | R-MAMMA1001623 | 1014 |
| MAMMA1001634 | F-MAMMA1001634 | 198 | R-MAMMA1001634 | 1015 |
| MAMMA1001893 | F-MAMMA1001893 | 199 | R-MAMMA1001893 | 1016 |
| MAMMA1001901 | F-MAMMA1001901 | 200 | R-MAMMA1001901 | 1017 |
| MAMMA1001957 | F-MAMMA1001957 | 201 | R-MAMMA1001957 | 1018 |
| MAMMA1001978 | F-MAMMA1001978 | 202 | R-MAMMA1001978 | 1019 |
| MAMMA1002070 | F-MAMMA1002070 | 203 | R-MAMMA1002070 | 1020 |
| MAMMA1002080 | F-MAMMA1002080 | 204 | R-MAMMA1002080 | 1021 |
| MAMMA1002087 | F-MAMMA1002087 | 205 | R-MAMMA1002087 | 1022 |
| MAMMA1002091 | F-MAMMA1002091 | 206 | | |
| MAMMA1002095 | F-MAMMA1002095 | 207 | R-MAMMA1002095 | 1023 |
| MAMMA1002128 | F-MAMMA1002128 | 208 | R-MAMMA1002128 | 1024 |
| MAMMA1002142 | F-MAMMA1002142 | 209 | R-MAMMA1002142 | 1025 |
| MAMMA1002165 | F-MAMMA1002165 | 210 | R-MAMMA1002165 | 1026 |
| MAMMA1002205 | F-MAMMA1002205 | 211 | R-MAMMA1002205 | 1027 |
| MAMMA1002224 | F-MAMMA1002224 | 212 | R-MAMMA1002224 | 1028 |
| MAMMA1002234 | F-MAMMA1002234 | 213 | R-MAMMA1002234 | 1029 |
| MAMMA1002586 | F-MAMMA1002586 | 214 | R-MAMMA1002586 | 1030 |
| MAMMA1002633 | F-MAMMA1002633 | 215 | R-MAMMA1002633 | 1031 |
| MAMMA1003126 | F-MAMMA1003126 | 216 | R-MAMMA1003126 | 1032 |
| NT2RM1000407 | F-NT2RM1000407 | 217 | | |
| NT2RM1000462 | F-NT2RM1000462 | 218 | | |
| NT2RM1000542 | F-NT2RM1000542 | 219 | | |
| NT2RM1000580 | F-NT2RM1000580 | 220 | | |
| NT2RM1000789 | F-NT2RM1000789 | 221 | | |
| NT2RM1000855 | F-NT2RM1000855 | 222 | | |
| NT2RM1000858 | F-NT2RM1000858 | 223 | | |
| NT2RM1000899 | F-NT2RM1000899 | 224 | | |
| NT2RM2000241 | F-NT2RM2000241 | 225 | | |
| NT2RM2000306 | F-NT2RM2000306 | 226 | | |
| NT2RM2000410 | F-NT2RM2000410 | 227 | | |
| NT2RM2000423 | F-NT2RM2000423 | 228 | | |
| NT2RM2000497 | F-NT2RM2000497 | 229 | | |
| NT2RM2000514 | F-NT2RM2000514 | 230 | | |
| NT2RM2000565 | F-NT2RM2000565 | 231 | | |
| NT2RM2000582 | F-NT2RM2000582 | 232 | | |
| NT2RM2000589 | F-NT2RM2000589 | 233 | | |
| NT2RM2000622 | F-NT2RM2000622 | 234 | | |
| NT2RM2000632 | F-NT2RM2000632 | 235 | | |
| NT2RM2000773 | F-NT2RM2000773 | 236 | | |
| NT2RM2001126 | F-NT2RM2001126 | 237 | | |
| NT2RM2001558 | F-NT2RM2001558 | 238 | | |
| NT2RM2001626 | F-NT2RM2001626 | 239 | | |
| NT2RM2001643 | F-NT2RM2001643 | 240 | | |
| NT2RM2001738 | F-NT2RM2001738 | 241 | | |
| NT2RM2001767 | F-NT2RM2001767 | 242 | | |
| NT2RM2001792 | F-NT2RM2001792 | 243 | | |
| NT2RM2001818 | F-NT2RM2001818 | 244 | | |
| NT2RM2001902 | F-NT2RM2001902 | 245 | | |
| NT2RM2001939 | F-NT2RM2001939 | 246 | | |
| NT2RM2001941 | F-NT2RM2001941 | 247 | | |
| NT2RM4000100 | F-NT2RM4000100 | 248 | R-NT2RM4000100 | 1033 |
| NT2RM4000115 | F-NT2RM4000115 | 249 | R-NT2RM4000115 | 1034 |
| NT2RM4000198 | F-NT2RM4000198 | 250 | R-NT2RM4000198 | 1035 |
| NT2RM4000284 | F-NT2RM4000284 | 251 | R-NT2RM4000284 | 1036 |
| NT2RM4000295 | F-NT2RM4000295 | 252 | R-NT2RM4000295 | 1037 |
| NT2RM4000326 | F-NT2RM4000326 | 253 | R-NT2RM4000326 | 1038 |
| NT2RM4000417 | F-NT2RM4000417 | 254 | R-NT2RM4000417 | 1039 |
| NT2RM4000444 | F-NT2RM4000444 | 255 | R-NT2RM4000444 | 1040 |
| NT2RM4000587 | F-NT2RM4000587 | 256 | R-NT2RM4000587 | 1041 |
| NT2RM4000593 | F-NT2RM4000593 | 257 | R-NT2RM4000593 | 1042 |
| NT2RM4000648 | F-NT2RM4000648 | 258 | R-NT2RM4000648 | 1043 |
| NT2RM4000761 | F-NT2RM4000761 | 259 | R-NT2RM4000761 | 1044 |
| NT2RM4000965 | F-NT2RM4000965 | 260 | R-NT2RM4000965 | 1045 |
| NT2RM4000997 | F-NT2RM4000997 | 261 | R-NT2RM4000997 | 1046 |
| NT2RM4001321 | F-NT2RM4001321 | 262 | R-NT2RM4001321 | 1047 |
| NT2RM4001325 | F-NT2RM4001325 | 263 | R-NT2RM4001325 | 1048 |
| NT2RM4001377 | F-NT2RM4001377 | 264 | R-NT2RM4001377 | 1049 |
| NT2RM4001735 | F-NT2RM4001735 | 265 | R-NT2RM4001735 | 1050 |
| NT2RM4001768 | F-NT2RM4001768 | 266 | R-NT2RM4001768 | 1051 |
| NT2RM4001843 | F-NT2RM4001843 | 267 | R-NT2RM4001843 | 1052 |
| NT2RM4002352 | F-NT2RM4002352 | 268 | R-NT2RM4002352 | 1053 |
| NT2RP1000002 | F-NT2RP1000002 | 269 | | |
| NT2RP1000050 | F-NT2RP1000050 | 270 | | |
| NT2RP1000181 | F-NT2RP1000181 | 271 | | |
| NT2RP1000239 | F-NT2RP1000239 | 272 | | |
| NT2RP1000261 | F-NT2RP1000261 | 273 | | |
| NT2RP1000271 | F-NT2RP1000271 | 274 | | |
| NT2RP1000300 | F-NT2RP1000300 | 275 | | |
| NT2RP1000325 | F-NT2RP1000325 | 276 | | |
| NT2RP1000448 | F-NT2RP1000448 | 277 | | |
| NT2RP1000465 | F-NT2RP1000465 | 278 | | |
| NT2RP1000468 | F-NT2RP1000468 | 279 | | |
| NT2RP1000551 | F-NT2RP1000551 | 280 | | |
| NT2RP1000579 | F-NT2RP1000579 | 281 | | |
| NT2RP1000613 | F-NT2RP1000613 | 282 | | |
| NT2RP1000679 | F-NT2RP1000679 | 283 | | |
| NT2RP1000740 | F-NT2RP1000740 | 284 | | |
| NT2RP1000903 | F-NT2RP1000903 | 285 | | |
| NT2RP1000981 | F-NT2RP1000981 | 286 | | |
| NT2RP1001004 | F-NT2RP1001004 | 287 | | |
| NT2RP1001020 | F-NT2RP1001020 | 288 | | |
| NT2RP1001031 | F-NT2RP1001031 | 289 | | |
| NT2RP1001563 | F-NT2RP1001563 | 290 | | |
| NT2RP2000092 | F-NT2RP2000092 | 291 | R-NT2RP2000092 | 1054 |
| NT2RP2000178 | F-NT2RP2000178 | 292 | R-NT2RP2000178 | 1055 |
| NT2RP2000240 | F-NT2RP2000240 | 293 | R-NT2RP2000240 | 1056 |
| NT2RP2000394 | F-NT2RP2000394 | 294 | R-NT2RP2000394 | 1057 |
| NT2RP2000447 | F-NT2RP2000447 | 295 | R-NT2RP2000447 | 1058 |
| NT2RP2000479 | F-NT2RP2000479 | 296 | R-NT2RP2000479 | 1059 |
| NT2RP2000514 | F-NT2RP2000514 | 297 | R-NT2RP2000514 | 1060 |
| NT2RP2000533 | F-NT2RP2000533 | 298 | R-NT2RP2000533 | 1061 |
| NT2RP2000610 | F-NT2RP2000610 | 299 | | |
| NT2RP2000616 | F-NT2RP2000616 | 300 | R-NT2RP2000616 | 1062 |
| NT2RP2000649 | F-NT2RP2000649 | 301 | R-NT2RP2000649 | 1063 |
| NT2RP2000663 | F-NT2RP2000663 | 302 | R-NT2RP2000663 | 1064 |
| NT2RP2000694 | F-NT2RP2000694 | 303 | | |
| NT2RP2000712 | F-NT2RP2000712 | 304 | R-NT2RP2000712 | 1065 |
| NT2RP2000739 | F-NT2RP2000739 | 305 | R-NT2RP2000739 | 1066 |
| NT2RP2000818 | F-NT2RP2000818 | 306 | R-NT2RP2000818 | 1067 |
| NT2RP2000903 | F-NT2RP2000903 | 307 | R-NT2RP2000903 | 1068 |
| NT2RP2001200 | F-NT2RP2001200 | 308 | R-NT2RP2001200 | 1069 |
| NT2RP2001223 | F-NT2RP2001223 | 309 | R-NT2RP2001223 | 1070 |
| NT2RP2001276 | F-NT2RP2001276 | 310 | R-NT2RP2001276 | 1071 |
| NT2RP2001388 | F-NT2RP2001388 | 311 | R-NT2RP2001388 | 1072 |
| NT2RP2001469 | F-NT2RP2001469 | 312 | R-NT2RP2001469 | 1073 |
| NT2RP2001480 | F-NT2RP2001480 | 313 | R-NT2RP2001480 | 1074 |
| NT2RP2001495 | F-NT2RP2001495 | 314 | R-NT2RP2001495 | 1075 |
| NT2RP2001514 | F-NT2RP2001514 | 315 | R-NT2RP2001514 | 1076 |
| NT2RP2001529 | F-NT2RP2001529 | 316 | | |
| NT2RP2001538 | F-NT2RP2001538 | 317 | R-NT2RP2001538 | 1077 |
| NT2RP2001562 | F-NT2RP2001562 | 318 | R-NT2RP2001562 | 1078 |
| NT2RP2001662 | F-NT2RP2001662 | 319 | R-NT2RP2001662 | 1079 |
| NT2RP2001755 | F-NT2RP2001755 | 320 | R-NT2RP2001755 | 1080 |
| NT2RP2001769 | F-NT2RP2001769 | 321 | R-NT2RP2001769 | 1081 |
| NT2RP2001817 | F-NT2RP2001817 | 322 | R-NT2RP2001817 | 1082 |
| NT2RP2001878 | F-NT2RP2001878 | 323 | R-NT2RP2001878 | 1083 |
| NT2RP2001903 | F-NT2RP2001903 | 324 | R-NT2RP2001903 | 1084 |
| NT2RP2001915 | F-NT2RP2001915 | 325 | R-NT2RP2001915 | 1085 |
| NT2RP2001921 | F-NT2RP2001921 | 326 | R-NT2RP2001921 | 1086 |
| NT2RP2001948 | F-NT2RP2001948 | 327 | R-NT2RP2001948 | 1087 |
| NT2RP2001956 | F-NT2RP2001956 | 328 | R-NT2RP2001956 | 1088 |
| NT2RP2002015 | F-NT2RP2002015 | 329 | R-NT2RP2002015 | 1089 |
| NT2RP2002063 | F-NT2RP2002063 | 330 | R-NT2RP2002063 | 1090 |
| NT2RP2002188 | F-NT2RP2002188 | 331 | R-NT2RP2002188 | 1091 |
| NT2RP2002232 | F-NT2RP2002232 | 332 | R-NT2RP2002232 | 1092 |
| NT2RP2002304 | F-NT2RP2002304 | 333 | R-nnnnnnnnnnnn | 1093 |
| NT2RP2002409 | F-NT2RP2002409 | 334 | R-NT2RP2002409 | 1094 |
| NT2RP2002510 | F-NT2RP2002510 | 335 | R-NT2RP2002510 | 1095 |
| NT2RP2002527 | F-NT2RP2002527 | 336 | R-NT2RP2002527 | 1096 |
| NT2RP2002533 | F-NT2RP2002533 | 337 | R-NT2RP2002533 | 1097 |
| NT2RP2002564 | F-NT2RP2002564 | 338 | R-NT2RP2002564 | 1098 |
| NT2RP2002674 | F-NT2RP2002674 | 339 | R-NT2RP2002674 | 1099 |
| NT2RP2002721 | F-NT2RP2002721 | 340 | R-NT2RP2002721 | 1100 |
| NT2RP2002824 | F-NT2RP2002824 | 341 | R-NT2RP2002824 | 1101 |
| NT2RP2002942 | F-NT2RP2002942 | 342 | R-NT2RP2002942 | 1102 |
| NT2RP2002974 | F-NT2RP2002974 | 343 | R-NT2RP2002974 | 1103 |
| NT2RP2002976 | F-NT2RP2002976 | 344 | R-NT2RP2002976 | 1104 |
| NT2RP2003042 | F-NT2RP2003042 | 345 | R-NT2RP2003042 | 1105 |
| NT2RP2003138 | F-NT2RP2003138 | 346 | | |
| NT2RP2003179 | F-NT2RP2003179 | 347 | R-NT2RP2003179 | 1106 |
| NT2RP2003210 | F-NT2RP2003210 | 348 | R-NT2RP2003210 | 1107 |
| NT2RP2003302 | F-NT2RP2003302 | 349 | R-NT2RP2003302 | 1108 |
| NT2RP2003369 | F-NT2RP2003369 | 350 | R-NT2RP2003369 | 1109 |
| NT2RP2003383 | F-NT2RP2003383 | 351 | R-NT2RP2003383 | 1110 |
| NT2RP2003390 | F-NT2RP2003390 | 352 | R-NT2RP2003390 | 1111 |
| NT2RP2003469 | F-NT2RP2003469 | 353 | R-NT2RP2003469 | 1112 |
| NT2RP2003545 | F-NT2RP2003545 | 354 | R-NT2RP2003545 | 1113 |
| NT2RP2003593 | F-NT2RP2003593 | 355 | R-NT2RP2003593 | 1114 |
| NT2RP2003599 | F-NT2RP2003599 | 356 | R-NT2RP2003599 | 1115 |
| NT2RP2003655 | F-NT2RP2003655 | 357 | R-NT2RP2003655 | 1116 |
| NT2RP2003664 | F-NT2RP2003664 | 358 | R-NT2RP2003664 | 1117 |
| NT2RP2003931 | F-NT2RP2003931 | 359 | R-NT2RP2003931 | 1118 |
| NT2RP2003940 | F-NT2RP2003940 | 360 | R-NT2RP2003940 | 1119 |
| NT2RP2003950 | F-NT2RP2003950 | 361 | R-NT2RP2003950 | 1120 |
| NT2RP2004069 | F-NT2RP2004069 | 362 | R-NT2RP2004069 | 1121 |
| NT2RP2004108 | F-NT2RP2004108 | 363 | R-NT2RP2004108 | 1122 |
| NT2RP2004141 | F-NT2RP2004141 | 364 | R-NT2RP2004141 | 1123 |
| NT2RP2004179 | F-NT2RP2004179 | 365 | R-NT2RP2004179 | 1124 |
| NT2RP2004205 | F-NT2RP2004205 | 366 | R-NT2RP2004205 | 1125 |
| NT2RP2004447 | F-NT2RP2004447 | 367 | R-NT2RP2004447 | 1126 |
| NT2RP2004495 | F-NT2RP2004495 | 368 | R-NT2RP2004495 | 1127 |
| NT2RP2004524 | F-NT2RP2004524 | 369 | R-NT2RP2004524 | 1128 |
| NT2RP2004556 | F-NT2RP2004556 | 370 | R-NT2RP2004556 | 1129 |
| NT2RP2004606 | F-NT2RP2004606 | 371 | R-NT2RP2004606 | 1130 |
| NT2RP2004648 | F-NT2RP2004648 | 372 | R-NT2RP2004648 | 1131 |
| NT2RP2004670 | F-NT2RP2004670 | 373 | R-NT2RP2004670 | 1132 |
| NT2RP2004794 | F-NT2RP2004794 | 374 | R-NT2RP2004794 | 1133 |
| NT2RP2004837 | F-NT2RP2004837 | 375 | R-NT2RP2004837 | 1134 |
| NT2RP2004847 | F-NT2RP2004847 | 376 | R-NT2RP2004847 | 1135 |
| NT2RP2005027 | F-NT2RP2005027 | 377 | R-NT2RP2005027 | 1136 |
| NT2RP2005069 | F-NT2RP2005069 | 378 | R-NT2RP2005069 | 1137 |
| NT2RP2005163 | F-NT2RP2005163 | 379 | R-NT2RP2005163 | 1138 |
| NT2RP2005181 | F-NT2RP2005181 | 380 | R-NT2RP2005181 | 1139 |
| NT2RP2005247 | F-NT2RP2005247 | 381 | R-NT2RP2005247 | 1140 |
| NT2RP2005378 | F-NT2RP2005378 | 382 | R-NT2RP2005378 | 1141 |
| NT2RP2005391 | F-NT2RP2005391 | 383 | R-NT2RP2005391 | 1142 |
| NT2RP2005425 | F-NT2RP2005425 | 384 | R-NT2RP2005425 | 1143 |
| NT2RP2005463 | F-NT2RP2005463 | 385 | R-NT2RP2005463 | 1144 |
| NT2RP2005514 | F-NT2RP2005514 | 386 | R-NT2RP2005514 | 1145 |
| NT2RP2005535 | F-NT2RP2005535 | 387 | R-NT2RP2005535 | 1146 |
| NT2RP2005541 | F-NT2RP2005541 | 388 | R-NT2RP2005541 | 1147 |
| NT2RP2005597 | F-NT2RP2005597 | 389 | R-NT2RP2005597 | 1148 |
| NT2RP2005632 | F-NT2RP2005632 | 390 | R-nnnnnnnnnnnn | 1149 |
| NT2RP2005666 | F-NT2RP2005666 | 391 | R-NT2RP2005666 | 1150 |
| NT2RP2005774 | F-NT2RP2005774 | 392 | R-NT2RP2005774 | 1151 |
| NT2RP2005878 | F-NT2RP2005878 | 393 | R-NT2RP2005878 | 1152 |
| NT2RP2005883 | F-NT2RP2005883 | 394 | R-NT2RP2005883 | 1153 |
| NT2RP2005887 | F-NT2RP2005887 | 395 | R-NT2RP2005887 | 1154 |
| NT2RP2005941 | F-NT2RP2005941 | 396 | R-nnnnnnnnnnnn | 1155 |
| NT2RP2005994 | F-NT2RP2005994 | 397 | R-NT2RP2005994 | 1156 |
| NT2RP2006004 | F-NT2RP2006004 | 398 | R-NT2RP2006004 | 1157 |
| NT2RP2006042 | F-NT2RP2006042 | 399 | R-NT2RP2006042 | 1158 |
| NT2RP2006092 | F-NT2RP2006092 | 400 | R-NT2RP2006092 | 1159 |
| NT2RP2006099 | F-NT2RP2006099 | 401 | R-NT2RP2006099 | 1160 |
| NT2RP2006134 | F-NT2RP2006134 | 402 | R-NT2RP2006134 | 1161 |
| NT2RP2006269 | F-NT2RP2006269 | 403 | R-NT2RP2006269 | 1162 |
| NT2RP2006512 | F-NT2RP2006512 | 404 | R-NT2RP2006512 | 1163 |
| NT2RP3000011 | F-NT2RP3000011 | 405 | R-NT2RP3000011 | 1164 |
| NT2RP3000022 | F-NT2RP3000022 | 406 | R-NT2RP3000022 | 1165 |
| NT2RP3000059 | F-NT2RP3000059 | 407 | R-NT2RP3000059 | 1166 |
| NT2RP3000063 | F-NT2RP3000063 | 408 | R-NT2RP3000063 | 1167 |
| NT2RP3000125 | F-NT2RP3000125 | 409 | R-nnnnnnnnnnnn | 1168 |
| NT2RP3000148 | F-NT2RP3000148 | 410 | R-NT2RP3000148 | 1169 |
| NT2RP3000169 | F-NT2RP3000169 | 411 | R-NT2RP3000169 | 1170 |
| NT2RP3000171 | F-NT2RP3000171 | 412 | R-NT2RP3000171 | 1171 |
| NT2RP3000172 | F-NT2RP3000172 | 413 | R-NT2RP3000172 | 1172 |
| NT2RP3000201 | F-NT2RP3000201 | 414 | R-NT2RP3000201 | 1173 |
| NT2RP3000232 | F-NT2RP3000232 | 415 | R-NT2RP3000232 | 1174 |
| NT2RP3000304 | F-NT2RP3000304 | 416 | R-NT2RP3000304 | 1175 |
| NT2RP3000378 | F-NT2RP3000378 | 417 | R-NT2RP3000378 | 1176 |
| NT2RP3000427 | F-NT2RP3000427 | 418 | | |
| NT2RP3000436 | F-NT2RP3000436 | 419 | R-NT2RP3000436 | 1177 |
| NT2RP3000444 | F-NT2RP3000444 | 420 | R-NT2RP3000444 | 1178 |
| NT2RP3000460 | F-NT2RP3000460 | 421 | R-NT2RP3000460 | 1179 |
| NT2RP3000481 | F-NT2RP3000481 | 422 | R-NT2RP3000481 | 1180 |
| NT2RP3000616 | F-NT2RP3000616 | 423 | R-NT2RP3000616 | 1181 |
| NT2RP3000645 | F-NT2RP3000645 | 424 | R-NT2RP3000645 | 1182 |
| NT2RP3000652 | F-NT2RP3000652 | 425 | R-NT2RP3000652 | 1183 |
| NT2RP3000676 | F-NT2RP3000676 | 426 | R-NT2RP3000676 | 1184 |
| NT2RP3000677 | F-NT2RP3000677 | 427 | R-NT2RP3000677 | 1185 |
| NT2RP3000721 | F-NT2RP3000721 | 428 | R-NT2RP3000721 | 1186 |
| NT2RP3000789 | F-NT2RP3000789 | 429 | R-NT2RP3000789 | 1187 |
| NT2RP3000818 | F-NT2RP3000818 | 430 | R-NT2RP3000818 | 1188 |
| NT2RP3000820 | F-NT2RP3000820 | 431 | R-NT2RP3000820 | 1189 |
| NT2RP3000838 | F-NT2RP3000838 | 432 | R-NT2RP3000838 | 1190 |
| NT2RP3000871 | F-NT2RP3000871 | 433 | R-NT2RP3000871 | 1191 |
| NT2RP3000907 | F-NT2RP3000907 | 434 | R-NT2RP3000907 | 1192 |
| NT2RP3000921 | F-NT2RP3000921 | 435 | R-NT2RP3000921 | 1193 |
| NT2RP3001012 | F-NT2RP3001012 | 436 | R-NT2RP3001012 | 1194 |
| NT2RP3001044 | F-NT2RP3001044 | 437 | R-NT2RP3001044 | 1195 |
| NT2RP3001061 | F-NT2RP3001061 | 438 | R-NT2RP3001061 | 1196 |
| NT2RP3001159 | F-NT2RP3001159 | 439 | R-NT2RP3001159 | 1197 |
| NT2RP3001170 | F-NT2RP3001170 | 440 | R-NT2RP3001170 | 1198 |
| NT2RP3001195 | F-NT2RP3001195 | 441 | R-NT2RP3001195 | 1199 |
| NT2RP3001240 | F-NT2RP3001240 | 442 | R-NT2RP3001240 | 1200 |
| NT2RP3001271 | F-NT2RP3001271 | 443 | R-NT2RP3001271 | 1201 |
| NT2RP3001322 | F-NT2RP3001322 | 444 | R-NT2RP3001322 | 1202 |
| NT2RP3001388 | F-NT2RP3001388 | 445 | | |
| NT2RP3001542 | F-NT2RP3001542 | 446 | R-NT2RP3001542 | 1203 |
| NT2RP3001560 | F-NT2RP3001560 | 447 | R-NT2RP3001560 | 1204 |
| NT2RP3001592 | F-NT2RP3001592 | 448 | R-NT2RP3001592 | 1205 |
| NT2RP3001650 | F-NT2RP3001650 | 449 | | |
| NT2RP3001685 | F-NT2RP3001685 | 450 | R-NT2RP3001685 | 1206 |
| NT2RP3001738 | F-NT2RP3001738 | 451 | R-NT2RP3001738 | 1207 |
| NT2RP3001754 | F-NT2RP3001754 | 452 | R-NT2RP3001754 | 1208 |
| NT2RP3001858 | F-NT2RP3001858 | 453 | R-NT2RP3001858 | 1209 |
| NT2RP3001976 | F-NT2RP3001976 | 454 | R-NT2RP3001976 | 1210 |
| NT2RP3002015 | F-NT2RP3002015 | 455 | R-NT2RP3002015 | 1211 |
| NT2RP3002160 | F-NT2RP3002160 | 456 | R-NT2RP3002160 | 1212 |
| NT2RP3002281 | F-NT2RP3002281 | 457 | R-NT2RP3002281 | 1213 |
| NT2RP3002286 | F-NT2RP3002286 | 458 | R-NT2RP3002286 | 1214 |
| NT2RP3002311 | F-NT2RP3002311 | 459 | R-NT2RP3002311 | 1215 |
| NT2RP3002324 | F-NT2RP3002324 | 460 | R-NT2RP3002324 | 1216 |
| NT2RP3002342 | F-NT2RP3002342 | 461 | R-NT2RP3002342 | 1217 |
| NT2RP3002353 | F-NT2RP3002353 | 462 | R-NT2RP3002353 | 1218 |
| NT2RP3002409 | F-NT2RP3002409 | 463 | R-NT2RP3002409 | 1219 |
| NT2RP3002411 | F-NT2RP3002411 | 464 | R-NT2RP3002411 | 1220 |
| NT2RP3002448 | F-NT2RP3002448 | 465 | R-NT2RP3002448 | 1221 |
| NT2RP3002571 | F-NT2RP3002571 | 466 | R-NT2RP3002571 | 1222 |
| NT2RP3002664 | F-NT2RP3002664 | 467 | R-NT2RP3002664 | 1223 |
| NT2RP3002721 | F-NT2RP3002721 | 468 | R-NT2RP3002721 | 1224 |
| NT2RP3002737 | F-NT2RP3002737 | 469 | R-NT2RP3002737 | 1225 |
| NT2RP3002738 | F-NT2RP3002738 | 470 | R-NT2RP3002738 | 1226 |
| NT2RP3002790 | F-NT2RP3002790 | 471 | R-NT2RP3002790 | 1227 |
| NT2RP3002836 | F-NT2RP3002836 | 472 | R-NT2RP3002836 | 1228 |
| NT2RP3002887 | F-NT2RP3002887 | 473 | R-NT2RP3002887 | 1229 |
| NT2RP3002900 | F-NT2RP3002900 | 474 | R-NT2RP3002900 | 1230 |
| NT2RP3002958 | F-NT2RP3002958 | 475 | R-NT2RP3002958 | 1231 |
| NT2RP3002983 | F-NT2RP3002983 | 476 | R-NT2RP3002983 | 1232 |
| NT2RP3003000 | F-NT2RP3003000 | 477 | R-NT2RP3003000 | 1233 |
| NT2RP3003076 | F-NT2RP3003076 | 478 | R-NT2RP3003076 | 1234 |
| NT2RP3003354 | F-NT2RP3003354 | 479 | R-NT2RP3003354 | 1235 |
| NT2RP3003448 | F-NT2RP3003448 | 480 | R-NT2RP3003448 | 1236 |
| NT2RP3003469 | F-NT2RP3003469 | 481 | R-NT2RP3003469 | 1237 |
| NT2RP3003473 | F-NT2RP3003473 | 482 | R-NT2RP3003473 | 1238 |
| NT2RP3003527 | F-NT2RP3003527 | 483 | R-NT2RP3003527 | 1239 |
| NT2RP3003532 | F-NT2RP3003532 | 484 | R-NT2RP3003532 | 1240 |
| NT2RP3003535 | F-NT2RP3003535 | 485 | R-nnnnnnnnnnnn | 1241 |
| NT2RP3003559 | F-NT2RP3003559 | 486 | R-NT2RP3003559 | 1242 |
| NT2RP3003614 | F-NT2RP3003614 | 487 | R-NT2RP3003614 | 1243 |
| NT2RP3003729 | F-NT2RP3003729 | 488 | R-NT2RP3003729 | 1244 |
| NT2RP3003849 | F-NT2RP3003849 | 489 | R-NT2RP3003849 | 1245 |
| NT2RP3003874 | F-NT2RP3003874 | 490 | R-NT2RP3003874 | 1246 |
| NT2RP3003939 | F-NT2RP3003939 | 491 | | |
| NT2RP3003963 | F-NT2RP3003963 | 492 | R-NT2RP3003963 | 1247 |
| NT2RP3004000 | F-NT2RP3004000 | 493 | R-NT2RP3004000 | 1248 |
| NT2RP3004025 | F-NT2RP3004025 | 494 | R-NT2RP3004025 | 1249 |
| NT2RP3004067 | F-NT2RP3004067 | 495 | | |
| NT2RP3004075 | F-NT2RP3004075 | 496 | R-NT2RP3004075 | 1250 |
| NT2RP3004083 | F-NT2RP3004083 | 497 | R-NT2RP3004083 | 1251 |
| NT2RP3004090 | F-NT2RP3004090 | 498 | R-NT2RP3004090 | 1252 |
| NT2RP3004119 | F-NT2RP3004119 | 499 | R-NT2RP3004119 | 1253 |
| NT2RP3004130 | F-NT2RP3004130 | 500 | R-NT2RP3004130 | 1254 |
| NT2RP3004133 | F-NT2RP3004133 | 501 | R-NT2RP3004133 | 1255 |
| NT2RP3004202 | F-NT2RP3004202 | 502 | R-NT2RP3004202 | 1256 |
| NT2RP3004294 | F-NT2RP3004294 | 503 | R-NT2RP3004294 | 1257 |
| NT2RP3004309 | F-NT2RP3004309 | 504 | R-NT2RP3004309 | 1258 |
| NT2RP3004321 | F-NT2RP3004321 | 505 | R-NT2RP3004321 | 1259 |
| NT2RP3004345 | F-NT2RP3004345 | 506 | R-NT2RP3004345 | 1260 |
| NT2RP3004355 | F-NT2RP3004355 | 507 | R-NT2RP3004355 | 1261 |
| NT2RP3004374 | F-NT2RP3004374 | 508 | R-NT2RP3004374 | 1262 |
| NT2RP3004406 | F-NT2RP3004406 | 509 | R-NT2RP3004406 | 1263 |
| NT2RP3004481 | F-NT2RP3004481 | 510 | R-NT2RP3004481 | 1264 |
| NT2RP3004552 | F-NT2RP3004552 | 511 | R-NT2RP3004552 | 1265 |
| NT2RP3004557 | F-NT2RP3004557 | 512 | | |
| NT2RP3004625 | F-NT2RP3004625 | 513 | R-NT2RP3004625 | 1266 |
| NT2RP3004640 | F-NT2RP3004640 | 514 | R-NT2RP3004640 | 1267 |
| NT2RP3004647 | F-NT2RP3004647 | 515 | R-NT2RP3004647 | 1268 |
| NT2RP4000108 | F-NT2RP4000108 | 516 | R-NT2RP4000108 | 1269 |
| NT2RP4000634 | F-NT2RP4000634 | 517 | R-NT2RP4000634 | 1270 |
| NT2RP4000962 | F-NT2RP4000962 | 518 | R-NT2RP4000962 | 1271 |
| NT2RP4001001 | F-NT2RP4001001 | 519 | R-NT2RP4001001 | 1272 |
| NT2RP4001009 | F-NT2RP4001009 | 520 | R-NT2RP4001009 | 1273 |
| NT2RP4001467 | F-NT2RP4001467 | 521 | R-NT2RP4001467 | 1274 |
| NT2RP4001877 | F-NT2RP4001877 | 522 | R-NT2RP4001877 | 1275 |
| NT2RP4001879 | F-NT2RP4001879 | 523 | R-NT2RP4001879 | 1276 |
| NT2RP4002187 | F-NT2RP4002187 | 524 | R-NT2RP4002187 | 1277 |
| NT2RP4002451 | F-NT2RP4002451 | 525 | R-NT2RP4002451 | 1278 |
| NT2RP4002715 | F-NT2RP4002715 | 526 | R-NT2RP4002715 | 1279 |
| NT2RP4002750 | F-NT2RP4002750 | 527 | R-NT2RP4002750 | 1280 |
| OVARC1000003 | F-OVARC1000003 | 528 | R-OVARC1000003 | 1281 |
| OVARC1000090 | F-OVARC1000090 | 529 | R-OVARC1000090 | 1282 |
| OVARC1000105 | F-OVARC1000105 | 530 | R-OVARC1000105 | 1283 |
| OVARC1000137 | F-OVARC1000137 | 531 | R-OVARC1000137 | 1284 |
| OVARC1000208 | F-OVARC1000208 | 532 | R-OVARC1000208 | 1285 |
| OVARC1000255 | F-OVARC1000255 | 533 | R-OVARC1000255 | 1286 |
| OVARC1000275 | F-OVARC1000275 | 534 | R-OVARC1000275 | 1287 |
| OVARC1000298 | F-OVARC1000298 | 535 | R-OVARC1000298 | 1288 |
| OVARC1000307 | F-OVARC1000307 | 536 | R-OVARC1000307 | 1289 |
| OVARC1000313 | F-OVARC1000313 | 537 | R-OVARC1000313 | 1290 |
| OVARC1000331 | F-OVARC1000331 | 538 | R-OVARC1000331 | 1291 |
| OVARC1000410 | F-OVARC1000410 | 539 | R-OVARC1000410 | 1292 |
| OVARC1000439 | F-OVARC1000439 | 540 | R-OVARC1000439 | 1293 |
| OVARC1000467 | F-OVARC1000467 | 541 | R-OVARC1000467 | 1294 |
| OVARC1000529 | F-OVARC1000529 | 542 | R-OVARC1000529 | 1295 |
| OVARC1000553 | F-OVARC1000553 | 543 | R-OVARC1000553 | 1296 |
| OVARC1000775 | F-OVARC1000775 | 544 | R-OVARC1000775 | 1297 |
| OVARC1000811 | F-OVARC1000811 | 545 | R-OVARC1000811 | 1298 |
| OVARC1000853 | F-OVARC1000853 | 546 | R-OVARC1000853 | 1299 |
| OVARC1000873 | F-OVARC1000873 | 547 | R-OVARC1000873 | 1300 |
| OVARC1000916 | F-OVARC1000916 | 548 | R-OVARC1000916 | 1301 |
| OVARC1000956 | F-OVARC1000956 | 549 | R-OVARC1000956 | 1302 |
| OVARC1000995 | F-OVARC1000995 | 550 | R-OVARC1000995 | 1303 |
| OVARC1001030 | F-OVARC1001030 | 551 | R-OVARC1001030 | 1304 |
| OVARC1001049 | F-OVARC1001049 | 552 | R-OVARC1001049 | 1305 |
| OVARC1001086 | F-OVARC1001086 | 553 | R-OVARC1001086 | 1306 |
| OVARC1001132 | F-OVARC1001132 | 554 | R-OVARC1001132 | 1307 |
| OVARC1001163 | F-OVARC1001163 | 555 | R-OVARC1001163 | 1308 |
| OVARC1001222 | F-OVARC1001222 | 556 | R-OVARC1001222 | 1309 |
| OVARC1001260 | F-OVARC1001260 | 557 | R-OVARC1001260 | 1310 |
| OVARC1001336 | F-OVARC1001336 | 558 | R-OVARC1001336 | 1311 |
| OVARC1001338 | F-OVARC1001338 | 559 | R-OVARC1001338 | 1312 |
| OVARC1001569 | F-OVARC1001569 | 560 | R-OVARC1001569 | 1313 |
| OVARC1001570 | F-OVARC1001570 | 561 | R-OVARC1001570 | 1314 |
| OVARC1001596 | F-OVARC1001596 | 562 | R-OVARC1001596 | 1315 |
| OVARC1001607 | F-OVARC1001607 | 563 | R-OVARC1001607 | 1316 |
| OVARC1001725 | F-OVARC1001725 | 564 | R-OVARC1001725 | 1317 |
| OVARC1001727 | F-OVARC1001727 | 565 | R-OVARC1001727 | 1318 |
| OVARC1001807 | F-OVARC1001807 | 566 | R-OVARC1001807 | 1319 |
| OVARC1001833 | F-OVARC1001833 | 567 | R-OVARC1001833 | 1320 |
| OVARC1001952 | F-OVARC1001952 | 568 | | |
| OVARC1001991 | F-OVARC1001991 | 569 | R-OVARC1001991 | 1321 |
| OVARC1002058 | F-OVARC1002058 | 570 | R-OVARC1002058 | 1322 |
| OVARC1002178 | F-OVARC1002178 | 571 | R-OVARC1002178 | 1323 |
| PLACE1000033 | F-PLACE1000033 | 572 | R-PLACE1000033 | 1324 |
| PLACE1000231 | F-PLACE1000231 | 573 | R-PLACE1000231 | 1325 |
| PLACE1000258 | F-PLACE1000258 | 574 | R-PLACE1000258 | 1326 |
| PLACE1000442 | F-PLACE1000442 | 575 | R-PLACE1000442 | 1327 |
| PLACE1000560 | F-PLACE1000560 | 576 | R-PLACE1000560 | 1328 |
| PLACE1000740 | F-PLACE1000740 | 577 | R-PLACE1000740 | 1329 |
| PLACE1000907 | F-PLACE1000907 | 578 | | |
| PLACE1000912 | F-PLACE1000912 | 579 | R-PLACE1000912 | 1330 |
| PLACE1000914 | F-PLACE1000914 | 580 | R-PLACE1000914 | 1331 |
| PLACE1000927 | F-PLACE1000927 | 581 | R-PLACE1000927 | 1332 |
| PLACE1000986 | F-PLACE1000986 | 582 | R-PLACE1000986 | 1333 |
| PLACE1001016 | F-PLACE1001016 | 583 | R-PLACE1001016 | 1334 |
| PLACE1001100 | F-PLACE1001100 | 584 | R-PLACE1001100 | 1335 |
| PLACE1001114 | F-PLACE1001114 | 585 | R-PLACE1001114 | 1336 |
| PLACE1001123 | F-PLACE1001123 | 586 | R-PLACE1001123 | 1337 |
| PLACE1001183 | F-PLACE1001183 | 587 | R-PLACE1001183 | 1338 |
| PLACE1001229 | F-PLACE1001229 | 588 | R-PLACE1001229 | 1339 |
| PLACE1001231 | F-PLACE1001231 | 589 | R-PLACE1001231 | 1340 |
| PLACE1001340 | F-PLACE1001340 | 590 | R-PLACE1001340 | 1341 |
| PLACE1001401 | F-PLACE1001401 | 591 | R-PLACE1001401 | 1342 |
| PLACE1001407 | F-PLACE1001407 | 592 | R-PLACE1001407 | 1343 |
| PLACE1001464 | F-PLACE1001464 | 593 | R-PLACE1001464 | 1344 |
| PLACE1001500 | F-PLACE1001500 | 594 | R-PLACE1001500 | 1345 |
| PLACE1001516 | F-PLACE1001516 | 595 | R-PLACE1001516 | 1346 |
| PLACE1001536 | F-PLACE1001536 | 596 | R-PLACE1001536 | 1347 |
| PLACE1001564 | F-PLACE1001564 | 597 | R-PLACE1001564 | 1348 |
| PLACE1001655 | F-PLACE1001655 | 598 | R-PLACE1001655 | 1349 |
| PLACE1001788 | F-PLACE1001788 | 599 | R-PLACE1001788 | 1350 |
| PLACE1001795 | F-PLACE1001795 | 600 | R-PLACE1001795 | 1351 |
| PLACE1001836 | F-PLACE1001836 | 601 | R-PLACE1001836 | 1352 |
| PLACE1001918 | F-PLACE1001918 | 602 | R-PLACE1001918 | 1353 |
| PLACE1001949 | F-PLACE1001949 | 603 | R-PLACE1001949 | 1354 |
| PLACE1002080 | F-PLACE1002080 | 604 | R-PLACE1002080 | 1355 |
| PLACE1002095 | F-PLACE1002095 | 605 | R-PLACE1002095 | 1356 |
| PLACE1002153 | F-PLACE1002153 | 606 | R-PLACE1002153 | 1357 |
| PLACE1002329 | F-PLACE1002329 | 607 | R-PLACE1002329 | 1358 |
| PLACE1002355 | F-PLACE1002355 | 608 | R-PLACE1002355 | 1359 |
| PLACE1002374 | F-PLACE1002374 | 609 | R-PLACE1002374 | 1360 |
| PLACE1002518 | F-PLACE1002518 | 610 | R-PLACE1002518 | 1361 |
| PLACE1002547 | F-PLACE1002547 | 611 | R-PLACE1002547 | 1362 |
| PLACE1002726 | F-PLACE1002726 | 612 | R-PLACE1002726 | 1363 |
| PLACE1002905 | F-PLACE1002905 | 613 | R-PLACE1002905 | 1364 |
| PLACE1002911 | F-PLACE1002911 | 614 | R-PLACE1002911 | 1365 |
| PLACE1002967 | F-PLACE1002967 | 615 | R-PLACE1002967 | 1366 |
| PLACE1003135 | F-PLACE1003135 | 616 | R-PLACE1003135 | 1367 |
| PLACE1003163 | F-PLACE1003163 | 617 | R-PLACE1003163 | 1368 |
| PLACE1003407 | F-PLACE1003407 | 618 | R-PLACE1003407 | 1369 |
| PLACE1003428 | F-PLACE1003428 | 619 | R-PLACE1003428 | 1370 |
| PLACE1003438 | F-PLACE1003438 | 620 | R-PLACE1003438 | 1371 |
| PLACE1003460 | F-PLACE1003460 | 621 | R-PLACE1003460 | 1372 |
| PLACE1003529 | F-PLACE1003529 | 622 | R-nnnnnnnnnnnn | 1373 |
| PLACE1003573 | F-PLACE1003573 | 623 | R-PLACE1003573 | 1374 |
| PLACE1003598 | F-PLACE1003598 | 624 | R-PLACE1003598 | 1375 |
| PLACE1003644 | F-PLACE1003644 | 625 | R-PLACE1003644 | 1376 |
| PLACE1003737 | F-PLACE1003737 | 626 | R-PLACE1003737 | 1377 |
| PLACE1003772 | F-PLACE1003772 | 627 | R-PLACE1003772 | 1378 |
| PLACE1003839 | F-PLACE1003839 | 628 | R-PLACE1003839 | 1379 |
| PLACE1003845 | F-PLACE1003845 | 629 | R-PLACE1003845 | 1380 |
| PLACE1003852 | F-PLACE1003852 | 630 | R-PLACE1003852 | 1381 |
| PLACE1004028 | F-PLACE1004028 | 631 | R-PLACE1004028 | 1382 |
| PLACE1004078 | F-PLACE1004078 | 632 | R-PLACE1004078 | 1383 |
| PLACE1004166 | F-PLACE1004166 | 633 | R-PLACE1004166 | 1384 |
| PLACE1004168 | F-PLACE1004168 | 634 | R-nnnnnnnnnnnn | 1385 |
| PLACE1004199 | F-PLACE1004199 | 635 | R-PLACE1004199 | 1386 |
| PLACE1004279 | F-PLACE1004279 | 636 | R-PLACE1004279 | 1387 |
| PLACE1004282 | F-PLACE1004282 | 637 | R-PLACE1004282 | 1388 |
| PLACE1004305 | F-PLACE1004305 | 638 | R-PLACE1004305 | 1389 |
| PLACE1004441 | F-PLACE1004441 | 639 | R-PLACE1004441 | 1390 |
| PLACE1004450 | F-PLACE1004450 | 640 | R-PLACE1004450 | 1391 |
| PLACE1004482 | F-PLACE1004482 | 641 | R-PLACE1004482 | 1392 |
| PLACE1004492 | F-PLACE1004492 | 642 | R-PLACE1004492 | 1393 |
| PLACE1004519 | F-PLACE1004519 | 643 | R-PLACE1004519 | 1394 |
| PLACE1004520 | F-PLACE1004520 | 644 | R-PLACE1004520 | 1395 |
| PLACE1004630 | F-PLACE1004630 | 645 | R-PLACE1004630 | 1396 |
| PLACE1004637 | F-PLACE1004637 | 646 | R-PLACE1004637 | 1397 |
| PLACE1004648 | F-PLACE1004648 | 647 | R-PLACE1004648 | 1398 |
| PLACE1004816 | F-PLACE1004816 | 648 | R-PLACE1004816 | 1399 |
| PLACE1004887 | F-PLACE1004887 | 649 | R-PLACE1004887 | 1400 |
| PLACE1005003 | F-PLACE1005003 | 650 | R-PLACE1005003 | 1401 |
| PLACE1005005 | F-PLACE1005005 | 651 | R-PLACE1005005 | 1402 |
| PLACE1005031 | F-PLACE1005031 | 652 | R-PLACE1005031 | 1403 |
| PLACE1005239 | F-PLACE1005239 | 653 | R-PLACE1005239 | 1404 |
| PLACE1005250 | F-PLACE1005250 | 654 | R-PLACE1005250 | 1405 |
| PLACE1005383 | F-PLACE1005383 | 655 | R-PLACE1005383 | 1406 |
| PLACE1005410 | F-PLACE1005410 | 656 | R-PLACE1005410 | 1407 |
| PLACE1005426 | F-PLACE1005426 | 657 | R-PLACE1005426 | 1408 |
| PLACE1005519 | F-PLACE1005519 | 658 | R-PLACE1005519 | 1409 |
| PLACE1005539 | F-PLACE1005539 | 659 | R-PLACE1005539 | 1410 |
| PLACE1005544 | F-PLACE1005544 | 660 | R-PLACE1005544 | 1411 |
| PLACE1005569 | F-PLACE1005569 | 661 | R-PLACE1005569 | 1412 |
| PLACE1005601 | F-PLACE1005601 | 662 | R-PLACE1005601 | 1413 |
| PLACE1005660 | F-PLACE1005660 | 663 | R-PLACE1005660 | 1414 |
| PLACE1005669 | F-PLACE1005669 | 664 | R-PLACE1005669 | 1415 |
| PLACE1005682 | F-PLACE1005682 | 665 | R-PLACE1005682 | 1416 |
| PLACE1005725 | F-PLACE1005725 | 666 | R-PLACE1005725 | 1417 |
| PLACE1005736 | F-PLACE1005736 | 667 | R-PLACE1005736 | 1418 |
| PLACE1005745 | F-PLACE1005745 | 668 | R-PLACE1005745 | 1419 |
| PLACE1005768 | F-PLACE1005768 | 669 | R-PLACE1005768 | 1420 |
| PLACE1005815 | F-PLACE1005815 | 670 | R-PLACE1005815 | 1421 |
| PLACE1005878 | F-PLACE1005878 | 671 | R-PLACE1005878 | 1422 |
| PLACE1005927 | F-PLACE1005927 | 672 | R-PLACE1005927 | 1423 |
| PLACE1006071 | F-PLACE1006071 | 673 | R-PLACE1006071 | 1424 |
| PLACE1006073 | F-PLACE1006073 | 674 | R-PLACE1006073 | 1425 |
| PLACE1006079 | F-PLACE1006079 | 675 | R-PLACE1006079 | 1426 |
| PLACE1006093 | F-PLACE1006093 | 676 | R-PLACE1006093 | 1427 |
| PLACE1006208 | F-PLACE1006208 | 677 | R-nnnnnnnnnnnn | 1428 |
| PLACE1006219 | F-PLACE1006219 | 678 | R-PLACE1006219 | 1429 |
| PLACE1006277 | F-PLACE1006277 | 679 | R-PLACE1006277 | 1430 |
| PLACE1006290 | F-PLACE1006290 | 680 | R-PLACE1006290 | 1431 |
| PLACE1006443 | F-PLACE1006443 | 681 | R-PLACE1006443 | 1432 |
| PLACE1006515 | F-PLACE1006515 | 682 | R-PLACE1006515 | 1433 |
| PLACE1006716 | F-PLACE1006716 | 683 | R-PLACE1006716 | 1434 |
| PLACE1006786 | F-PLACE1006786 | 684 | R-PLACE1006786 | 1435 |
| PLACE1006809 | F-PLACE1006809 | 685 | R-PLACE1006809 | 1436 |
| PLACE1006959 | F-PLACE1006959 | 686 | R-PLACE1006959 | 1437 |
| PLACE1007028 | F-PLACE1007028 | 687 | R-PLACE1007028 | 1438 |
| PLACE1007040 | F-PLACE1007040 | 688 | R-PLACE1007040 | 1439 |
| PLACE1007077 | F-PLACE1007077 | 689 | R-PLACE1007077 | 1440 |
| PLACE1007081 | F-PLACE1007081 | 690 | R-PLACE1007081 | 1441 |
| PLACE1007096 | F-PLACE1007096 | 691 | R-PLACE1007096 | 1442 |
| PLACE1007296 | F-PLACE1007296 | 692 | R-PLACE1007296 | 1443 |
| PLACE1007591 | F-PLACE1007591 | 693 | R-PLACE1007591 | 1444 |
| PLACE1007626 | F-PLACE1007626 | 694 | R-PLACE1007626 | 1445 |
| PLACE1007702 | F-PLACE1007702 | 695 | R-PLACE1007702 | 1446 |
| PLACE1007845 | F-PLACE1007845 | 696 | R-PLACE1007845 | 1447 |
| PLACE1007881 | F-PLACE1007881 | 697 | R-PLACE1007881 | 1448 |
| PLACE1007971 | F-PLACE1007971 | 698 | R-PLACE1007971 | 1449 |
| PLACE1008282 | F-PLACE1008282 | 699 | R-PLACE1008282 | 1450 |
| PLACE1008297 | F-PLACE1008297 | 700 | R-PLACE1008297 | 1451 |
| PLACE1008359 | F-PLACE1008359 | 701 | R-PLACE1008359 | 1452 |
| PLACE1008469 | F-PLACE1008469 | 702 | R-PLACE1008469 | 1453 |
| PLACE1008549 | F-PLACE1008549 | 703 | R-PLACE1008549 | 1454 |
| PLACE1008657 | F-PLACE1008657 | 704 | R-PLACE1008657 | 1455 |
| PLACE1008716 | F-PLACE1008716 | 705 | R-PLACE1008716 | 1456 |
| PLACE1008744 | F-PLACE1008744 | 706 | R-PLACE1008744 | 1457 |
| PLACE1008984 | F-PLACE1008984 | 707 | R-PLACE1008984 | 1458 |
| PLACE1008985 | F-PLACE1008985 | 708 | R-PLACE1008985 | 1459 |
| PLACE1009067 | F-PLACE1009067 | 709 | R-PLACE1009067 | 1460 |
| PLACE1009196 | F-PLACE1009196 | 710 | R-PLACE1009196 | 1461 |
| PLACE1009279 | F-PLACE1009279 | 711 | R-PLACE1009279 | 1462 |
| PLACE1009527 | F-PLACE1009527 | 712 | R-PLACE1009527 | 1463 |
| PLACE1009546 | F-PLACE1009546 | 713 | R-PLACE1009546 | 1464 |
| PLACE1009600 | F-PLACE1009600 | 714 | R-PLACE1009600 | 1465 |
| PLACE1009735 | F-PLACE1009735 | 715 | R-PLACE1009735 | 1466 |
| PLACE1009982 | F-PLACE1009982 | 716 | R-nnnnnnnnnnnn | 1467 |
| PLACE1010011 | F-PLACE1010011 | 717 | R-PLACE1010011 | 1468 |
| PLACE1010078 | F-PLACE1010078 | 718 | R-PLACE1010078 | 1469 |
| PLACE1010081 | F-PLACE1010081 | 719 | R-PLACE1010081 | 1470 |
| PLACE1010251 | F-PLACE1010251 | 720 | R-PLACE1010251 | 1471 |
| PLACE1010445 | F-PLACE1010445 | 721 | R-PLACE1010445 | 1472 |
| PLACE1010713 | F-PLACE1010713 | 722 | R-PLACE1010713 | 1473 |
| PLACE1010784 | F-PLACE1010784 | 723 | R-PLACE1010784 | 1474 |
| PLACE1010827 | F-PLACE1010827 | 724 | R-PLACE1010827 | 1475 |
| PLACE1010968 | F-PLACE1010968 | 725 | R-PLACE1010968 | 1476 |
| PLACE1011045 | F-PLACE1011045 | 726 | R-PLACE1011045 | 1477 |
| PLACE1011116 | F-PLACE1011116 | 727 | R-PLACE1011116 | 1478 |
| PLACE1011181 | F-PLACE1011181 | 728 | | |
| PLACE1011236 | F-PLACE1011236 | 729 | R-PLACE1011236 | 1479 |
| PLACE1011364 | F-PLACE1011364 | 730 | R-PLACE1011364 | 1480 |
| PLACE1011407 | F-PLACE1011407 | 731 | R-PLACE1011407 | 1481 |
| PLACE1011516 | F-PLACE1011516 | 732 | R-PLACE1011516 | 1482 |
| PLACE1011708 | F-PLACE1011708 | 733 | R-PLACE1011708 | 1483 |
| PLACE1011824 | F-PLACE1011824 | 734 | R-PLACE1011824 | 1484 |
| PLACE1011978 | F-PLACE1011978 | 735 | R-PLACE1011978 | 1485 |
| PLACE2000118 | F-PLACE2000118 | 736 | R-PLACE2000118 | 1486 |
| PLACE2000219 | F-PLACE2000219 | 737 | R-PLACE2000219 | 1487 |
| PLACE3000181 | F-PLACE3000181 | 738 | R-PLACE3000181 | 1488 |
| PLACE3000213 | F-PLACE3000213 | 739 | R-PLACE3000213 | 1489 |
| PLACE4000354 | F-PLACE4000354 | 740 | R-PLACE4000354 | 1490 |
| PLACE4000455 | F-PLACE4000455 | 741 | R-PLACE4000455 | 1491 |
| SKNMC1000004 | F-SKNMC1000004 | 742 | | |
| SKNMC1000014 | F-SKNMC1000014 | 743 | | |
| SKNMC1000082 | F-SKNMC1000082 | 744 | | |
| THYRO1000036 | F-THYRO1000036 | 745 | R-THYRO1000036 | 1492 |
| THYRO1000061 | F-THYRO1000061 | 746 | R-THYRO1000061 | 1493 |
| THYRO1000099 | F-THYRO1000099 | 747 | R-THYRO1000099 | 1494 |
| THYRO1000196 | F-THYRO1000196 | 748 | R-THYRO1000196 | 1495 |
| THYRO1000400 | F-THYRO1000400 | 749 | R-THYRO1000400 | 1496 |
| THYRO1000580 | F-THYRO1000580 | 750 | R-THYRO1000580 | 1497 |
| THYRO1000584 | F-THYRO1000584 | 751 | R-THYRO1000584 | 1498 |
| THYRO1000678 | F-THYRO1000678 | 752 | R-THYRO1000678 | 1499 |
| THYRO1000776 | F-THYRO1000776 | 753 | R-THYRO1000776 | 1500 |
| THYRO1000795 | F-THYRO1000795 | 754 | R-THYRO1000795 | 1501 |
| THYRO1000846 | F-THYRO1000846 | 755 | R-THYRO1000846 | 1502 |
| THYRO1000866 | F-THYRO1000866 | 756 | R-THYRO1000866 | 1503 |
| THYRO1000956 | F-THYRO1000956 | 757 | R-THYRO1000956 | 1504 |
| THYRO1000964 | F-THYRO1000964 | 758 | R-THYRO1000964 | 1505 |
| THYRO1000999 | F-THYRO1000999 | 759 | R-THYRO1000999 | 1506 |
| THYRO1001063 | F-THYRO1001063 | 760 | R-THYRO1001063 | 1507 |
| THYRO1001071 | F-THYRO1001071 | 761 | R-THYRO1001071 | 1508 |
| THYRO1001102 | F-THYRO1001102 | 762 | R-THYRO1001102 | 1509 |
| THYRO1001113 | F-THYRO1001113 | 763 | R-THYRO1001113 | 1510 |
| THYRO1001128 | F-THYRO1001128 | 764 | R-THYRO1001128 | 1511 |
| THYRO1001205 | F-THYRO1001205 | 765 | R-THYRO1001205 | 1512 |
| THYRO1001237 | F-THYRO1001237 | 766 | R-THYRO1001237 | 1513 |
| THYRO1001242 | F-THYRO1001242 | 767 | R-THYRO1001242 | 1514 |
| THYRO1001266 | F-THYRO1001266 | 768 | R-THYRO1001266 | 1515 |
| THYRO1001327 | F-THYRO1001327 | 769 | R-THYRO1001327 | 1516 |
| THYRO1001456 | F-THYRO1001456 | 770 | R-THYRO1001456 | 1517 |
| THYRO1001457 | F-THYRO1001457 | 771 | R-THYRO1001457 | 1518 |
| THYRO1001471 | F-THYRO1001471 | 772 | R-THYRO1001471 | 1519 |
| THYRO1001478 | F-THYRO1001478 | 773 | R-THYRO1001478 | 1520 |
| THYRO1001495 | F-THYRO1001495 | 774 | R-THYRO1001495 | 1521 |
| THYRO1001523 | F-THYRO1001523 | 775 | R-THYRO1001523 | 1522 |
| THYRO1001529 | F-THYRO1001529 | 776 | R-THYRO1001529 | 1523 |
| THYRO1001593 | F-THYRO1001593 | 777 | R-THYRO1001593 | 1524 |
| THYRO1001608 | F-THYRO1001608 | 778 | R-THYRO1001608 | 1525 |
| THYRO1001641 | F-THYRO1001641 | 779 | R-THYRO1001641 | 1526 |
| THYRO1001700 | F-THYRO1001700 | 780 | R-THYRO1001700 | 1527 |
| THYRO1001702 | F-THYRO1001702 | 781 | R-THYRO1001702 | 1528 |
| THYRO1001725 | F-THYRO1001725 | 782 | R-THYRO1001725 | 1529 |
| THYRO1001770 | F-THYRO1001770 | 783 | R-THYRO1001770 | 1530 |
| THYRO1001803 | F-THYRO1001803 | 784 | R-THYRO1001803 | 1531 |
| Y79AA1000030 | F-Y79AA1000030 | 785 | R-Y79AA1000030 | 1532 |
| Y79AA1000127 | F-Y79AA1000127 | 786 | R-Y79AA1000127 | 1533 |
| Y79AA1000207 | F-Y79AA1000207 | 787 | R-Y79AA1000207 | 1534 |
| Y79AA1000226 | F-Y79AA1000226 | 788 | R-Y79AA1000226 | 1535 |
| Y79AA1000270 | F-Y79AA1000270 | 789 | R-Y79AA1000270 | 1536 |
| Y79AA1000426 | F-Y79AA1000426 | 790 | R-Y79AA1000426 | 1537 |
| Y79AA1000521 | F-Y79AA1000521 | 791 | R-Y79AA1000521 | 1538 |
| Y79AA1000750 | F-Y79AA1000750 | 792 | R-Y79AA1000750 | 1539 |
| Y79AA1000776 | F-Y79AA1000776 | 793 | R-Y79AA1000776 | 1540 |
| Y79AA1000777 | F-Y79AA1000777 | 794 | R-Y79AA1000777 | 1541 |
| Y79AA1000876 | F-Y79AA1000876 | 795 | R-Y79AA1000876 | 1542 |
| Y79AA1000888 | F-Y79AA1000888 | 796 | | |
| Y79AA1000959 | F-Y79AA1000959 | 797 | R-Y79AA1000959 | 1543 |
| Y79AA1000967 | F-Y79AA1000967 | 798 | R-Y79AA1000967 | 1544 |
| Y79AA1001013 | F-Y79AA1001013 | 799 | R-Y79AA1001013 | 1545 |
| Y79AA1001056 | F-Y79AA1001056 | 800 | R-Y79AA1001056 | 1546 |
| Y79AA1001062 | F-Y79AA1001062 | 801 | R-Y79AA1001062 | 1547 |
| Y79AA1001090 | F-Y79AA1001090 | 802 | R-Y79AA1001090 | 1548 |
| Y79AA1001212 | F-Y79AA1001212 | 803 | R-Y79AA1001212 | 1549 |
| Y79AA1001264 | F-Y79AA1001264 | 804 | R-Y79AA1001264 | 1550 |
| Y79AA1001272 | F-Y79AA1001272 | 805 | R-Y79AA1001272 | 1551 |
| Y79AA1001328 | F-Y79AA1001328 | 806 | R-Y79AA1001328 | 1552 |
| Y79AA1001426 | F-Y79AA1001426 | 807 | R-Y79AA1001426 | 1553 |
| Y79AA1001427 | F-Y79AA1001427 | 808 | | |
| Y79AA1001430 | F-Y79AA1001430 | 809 | R-Y79AA1001430 | 1554 |
| Y79AA1001523 | F-Y79AA1001523 | 810 | R-Y79AA1001523 | 1555 |
| Y79AA1001530 | F-Y79AA1001530 | 811 | R-Y79AA1001530 | 1556 |
| Y79AA1001592 | F-Y79AA1001592 | 812 | R-Y79AA1001592 | 1557 |
| Y79AA1001727 | F-Y79AA1001727 | 813 | R-Y79AA1001727 | 1558 |
| Y79AA1001787 | F-Y79AA1001787 | 814 | R-Y79AA1001787 | 1559 |
| Y79AA1001793 | F-Y79AA1001793 | 815 | | |
| Y79AA1001795 | F-Y79AA1001795 | 816 | R-Y79AA1001795 | 1560 |
| Y79AA1001799 | F-Y79AA1001799 | 817 | R-Y79AA1001799 | 1561 |
| Y79AA1001803 | F-Y79AA1001803 | 818 | R-Y79AA1001803 | 1562 |
| Y79AA1001863 | F-Y79AA1001863 | 819 | R-Y79AA1001863 | 1563 |
| Y79AA1002022 | F-Y79AA1002022 | 820 | R-Y79AA1002022 | 1564 |
| Y79AA1002058 | F-Y79AA1002058 | 821 | | |
| Y79AA1002121 | F-Y79AA1002121 | 822 | R-nnnnnnnnnnnn | 1565 |
| Y79AA1002129 | F-Y79AA1002129 | 823 | R-nnnnnnnnnnnn | 1566 |
| Y79AA1002213 | F-Y79AA1002213 | 824 | R-Y79AA1002213 | 1567 |
| Y79AA1002334 | F-Y79AA1002334 | 825 | R-Y79AA1002334 | 1568 |
| Y79AA1002373 | F-Y79AA1002373 | 826 | R-Y79AA1002373 | 1569 |
| Y79AA1002376 | F-Y79AA1002376 | 827 | R-Y79AA1002376 | 1570 |
| Y79AA1002378 | F-Y79AA1002378 | 828 | R-Y79AA1002378 | 1571 |
| Y79AA1002381 | F-Y79AA1002381 | 829 | R-Y79AA1002381 | 1572 |
| NT2RP2006580 | F-NT2RP2006580 | 2545 | R-NT2RP2006580 | 2546 |

The sequence name starting from "F" means the name of 5'-end sequence, and the sequence name starting from "R" means the name of 3'-end sequence. A blank indicates that the 3'-end sequence corresponding to the 5'-end sequence has not been determined in the clone.

Furthermore, the present invention relates to the use of the above primers, as described below.
(4) A polynucleotide which can be synthesized with the primer set of (2) or (3).
(5) A polynucleotide comprising a coding region in the polynucleotide of (4).
(6) A substantially pure protein encoded by polynucleotide of (4).
(7) A partial peptide of the protein of (6).

In addition, the present invention comprises a polynucleotide described below and a protein encoded by the polynucleotide.
(8) An isolated polynucleotide selected from the group consisting of
   (a) a polynucleotide comprising a coding region of the nucleotide sequence set forth in any one of the SEQ ID NOs in Table 370;
   (b) a polynucleotide comprising a nucleotide sequence encoding a protein comprising the amino acid sequence set forth in any one of the SEQ ID NOs in Table 370;
   (c) a polynucleotide comprising a nucleotide sequence encoding a protein comprising an amino acid sequence selected from the amino acid sequences set forth in the SEQ ID NOs in Table 370, in which one or more amino acids are substituted, deleted, inserted, and/or added, wherein said protein is functionally equivalent to the protein comprising said amino acid sequence selected from the amino acid sequences set forth in the SEQ ID NOs in Table 370;
   (d) a polynucleotide that hybridizes with a polynucleotide comprising a nucleotide sequence selected from the nucleotide sequences set forth in the SEQ ID NOs in Table 370, and that comprises a nucleotide sequence encoding a protein functionally equivalent to the protein encoded by the nucleotide sequence selected from the nucleotide sequences set forth in the SEQ ID NOs in Table 370;
   (e) a polynucleotide comprising a nucleotide sequence encoding a partial amino acid sequence of a protein encoded by the polynucleotide of (a) to (d);
   (f) a polynucleotide comprising a nucleotide sequence with at least 70% identity to the nucleotide sequence set forth in any one of the SEQ ID NOs in Table 370.
(9). A substantially pure protein encoded by the polynucleotide of (8).
(10) An antibody against the protein or peptide of any one of (6), (7), and (9).
(11) A vector comprising the polynucleotide of (5) or (8).
(12) A transformant carrying the polynucleotide of (5) or (8), or the vector of (11).
(13) A transformant expressively carrying the polynucleotide of (5) or (8), or the vector of (11).
(14) A method for producing the protein or peptide of any one of (6), (7), and (9), comprising culturing the transformant of (13) and recovering the expression product.
(15) An oligonucleotide comprising the nucleotide sequence set forth in any one of the SEQ ID NOs in Table 370 or the nucleotide sequence complementary to the complementary strand thereof, wherein said oligonucleotide comprises 15 nucleotides or more.
(16) Use of the oligonucleotide of (15) as a primer for synthesizing a polynucleotide.
(17) Use of the oligonucleotide of (15) as a probe for detecting a gene.
(18) An antisense polynucleotide against the polynucleotide of (8), or the portion thereof.
(19) A method for synthesizing a polynucleotide, the method comprising:
   a) synthesizing a complementary strand using a cDNA library as a template, and using the primer set of (2) or (3), or the primer of (16); and
   b) recovering the synthesized product.
(20) The method of (19), wherein the cDNA library is obtainable by oligo-capping method.
(21) The method of (19), wherein the complementary strand is obtainable by PCR.
(22) A method for detecting the polynucleotide of (8), the method comprising:
   a) incubating a target polynucleotide with the oligonucleotide of (15) under the conditions where hybridization occurs, and
   b) detecting the hybridization of the target polynucleotide with the oligonucleotide of (15).
(23) A database of polynucleotides and/or proteins, the database comprising information on at least one sequence selected from the nucleotide sequences set forth in the SEQ ID NOs in Table 370 and/or the amino acid sequences set forth in the SEQ ID NOs in Table 370, or a medium on which the database is stored.

Any patents, patent applications, and publications cited herein are incorporated by reference.

### BRIEF DESCRIPTION OF THE DRAWINGS

Figure 1 shows the restriction maps of vectors pME18SFL3 and pUC19FL3.
Figure 2 shows the reproducibility of gene expression analysis. The ordinate and the abscissa show the intensities of gene expression obtained in experiments different from each other.
Figure 3 shows the detection limit in gene expression analysis. The intensity of expression is shown in the ordinate, and the concentration (µg/ml) of the probe used is shown in the abscissa.

### DETAILED DESCRIPTION OF THE INVENTION

Herein, "polynucleotide" is defined as a molecule in which multiple nucleotides are polymerized. There are no limitations in the number of the polymerized nucleotides. In case that the polymer contains relatively low number of nucleotides, it is also described as an "oligonucleotide". The polynucleotide or the oligonucleotide of the present invention can be a natural or chemically synthesized product. Alternatively, it can be synthesized using a template DNA by an enzymatic reaction such as PCR.

All the cDNA provided by the invention are full-length cDNA. Herein, a "full-length cDNA" is defined as a cDNA which contains both ATG codon (the translation start site) and the stop codon. Accordingly, the untranslated regions, which are originally found in the upstream or downstream of the protein coding region in natural mRNA, may or may not be contained.

An "isolated polynucleotide" is a polynucleotide the structure of which is not identical to that of any naturally occurring nucleic acid or to that of any fragment of a naturally occurring genomic nucleic acid spanning more than three separate genes. The term therefore covers, for example,
(a) a DNA which has the sequence of part of a naturally occurring genomic DNA molecule but is not flanked by both of the coding sequences that flank that part of the molecule in the genome of the organism in which it naturally occurs;
(b) a nucleic acid incorporated into a vector or into the genomic DNA of a prokaryote or eukaryote in a manner such that the resulting molecule is not identical to any naturally occurring vector or genomic DNA;
(c) a separate molecule such as a cDNA, a genomic fragment, a fragment produced by polymerase chain reaction (PCR), or a restriction fragment; and
(d) a recombinant nucleotide sequence that is part of a hybrid gene, i.e., a gene encoding a fusion protein. Specifically excluded from this definition are nucleic acids present in mixtures of different (i) DNA molecules, (ii) transfected cells, or (iii) cell clones: e.g., as these occur in a DNA library such as a cDNA or genomic DNA library.

The term "substantially pure" as used herein in reference to a given polypeptide means that the protein or polypeptide is substantially free from other biological macromolecules. The substantially pure protein or polypeptide is at least 75% (e.g., at least 80, 85, 95, or 99%) pure by dry weight. Purity can be measured by any appropriate standard method, for example, by column chromatography, polyacrylamide gel electrophoresis, or HPLC analysis.

All the clones of the present invention (830 clones) are novel and covering full-length, and also predicted to encode any of the following functional protein:
secretory proteins,
membrane proteins,
proteins associated to signal transduction (signal transduction-associated proteins; e.g. protein kinases, etc.),
proteins associated to a glycoprotein (glycoprotein-associated proteins),
proteins associated with transcription (transcription-associated proteins),
proteins associated with diseases (disease-associated proteins),
or, enzymes and/or metabolism-associated proteins, cell division- and/or cell proliferation-associated proteins, cytoskeleton-associated proteins, nuclear proteins, DNA- and/or RNA-binding proteins, ATP- and/or GTP-binding proteins, protein synthesis- and/or protein transport-associated proteins, and cellular defense-associated proteins.

Furthermore, all the cDNA clones of the present invention can be characterized as follows:
(1) a cDNA that is obtained by the oligo-capping method, which provides cDNA with high fullness ratio. The cDNA was selected by the score in the ATGpr (described as ATGpr1, as well), which is a program for prediction of the fullness of the 5'-end of cDNA based on the features of the 5'-end sequence. In addition, the PSORT, which is a program for prediction of the existence of the signal sequence selected, cDNA that contains a signal sequence in the 5'-end, or transmembrane region in the protein coding region. Furthermore, the homology search with the 5'-end sequences confirmed that, the selected clones were not identical to any of the known human mRNA (namely novel);
   or,
(2) a cDNA that is obtained by the oligo-capping method, which provides cDNA with high fullness ratio. The cDNA was selected by the score in the ATGpr, which is a program for prediction of the fullness of the 5'-end based on the features of the 5'-end sequence. Furthermore, the a cDNA that has relative homology with an amino acid sequence of a protein with known functions was selected by the BLAST search (Altschul S.F., Gish W., Miller W., Myers E.W., and Lipman D.J. (1990) J. Mol. Biol. 215: 403-410 ; Gish W., and States D.J. (1993) Nature Genet. 3: 266-272) on the SwissProt database using the 5'-end sequence. In addition, the homology search using the 5'-end sequence confirmed that the selected clones were not identical to any of the known human mRNA (namely novel).

All clones are obtainable as a full-length clone by such a method as PCR (Current Protocols in Molecular Biology, Ausubel et al. edit, (1987) John Wiley & Sons, Section 6.1-6.4) using both the 5'- and 3'-end sequences, or using the 5'-end sequence and an oligo-dT primer that corresponds to the polyA sequence.

Specifically, PCR can be performed using an oligonucleotide that has 15 nucleotides longer, and specifically hybridizes with the complementary strand of the polynucleotide that contains the nucleotide sequence selected from the 5'-end sequences shown in Table 1 (SEQ ID NO: 1-829, and SEQ ID NO: 2545), and an oligo-dT primer as a 5'-, and 3'-primer, respectively. The length of the primers is usually 15-100 bp, and favorably between 15-35 bp. In case of LA PCR, which is described below, the primer length of 25-35 bp may provide a good result.

A method to design a primer that enables a specific amplification based on the given nucleotide sequence is known to those skilled in the art (Current Protocols in Molecular Biology, Ausubel et al. edit, (1987) John Wiley & Sons, Section 6.1-6.4). In designing a primer based on the 5'-end sequence, the primer is designed so as that, in principle, the amplification products will include the translation start site. Accordingly, in case that a given 5'-end nucleotide sequence is the 5'- untranslated region (5'UTR), any part of the sequence can be used as a 5'-primer as far as the specificity toward the target cDNA is insured. The translation start site can be predicted using a known method such as the ATGpr as described below.

When synthesizing a polynucleotide, the target nucleotide sequence to be amplified can extend to several thousand bp in some cDNA. However, it is possible to amplify such a long nucleotides by using such as LA PCR (Long and Accurate PCR). It is advantageous to use LA PCR when synthesizing long DNA. In LA PCR, in which a special DNA polymerase having 3' →5' exonuclease activity is used, misincorporated nucleotides can be removed. Accordingly, accurate synthesis of the complementary strand can be achieved even with a long nucleotide sequence. By using LA PCR, it is reported that amplification of a nucleotide with 20 kb longer can be achieved under desirable condition (Takeshi Hayashi (1996) Jikken-Igaku Bessatsu, "Advanced Technologies in PCR" Youdo-sha).

A template DNA for synthesizing the cDNA of the present invention can be obtained by using cDNA libraries that are prepared by various methods. The full-length cDNA clones obtained here are those with high fullness ratio, which were obtained using a combination of (1) a method to prepare a full-length-enriched cDNA library using the oligo-capping method, and (2) an estimation system for fullness using the 5'-end sequence (selection based on the estimation by the ATGpr after removing clones that are non-full-length compared to the ESTs). However, it is possible to easily obtain a full-length cDNA by using the primers that are provided by the present invention, not by the above described specialized method.

The problem with the cDNA libraries prepared by the known methods or commercially available is that mRNA contained in the libraries has very low fullness ratio. Thus, it is difficult to screen full-length cDNA clone directly from the library using ordinary cloning methods. The present invention has revealed a primer that is capable of synthesizing a full-length cDNA. If provided with primers, it is possible to synthesize a target full-length cDNA by using enzymatic reactions such as PCR. In particular, a full-length-enriched cDNA library, synthesized by methods such as oligo-capping, is desirable to synthesize a full-length cDNA with more reliability.

Once the nucleotide sequences of the full-length cDNAs obtained in the present invention is determined, it is possible to predict the functions of the proteins encoded by the cDNA clones, for example, by searching the databases such as GenBank (http://www.ncbi.nhn.nih.gov/web/GenBank/), Swiss-Prot (http://www.ebi.ac.uk/ebi_docsSwiss-Prot_db/swisshome.html), UniGene (http://www.ncbi.nlm.nih.gov/UniGene) for homologies of the cDNAs, or by searching the amino acid sequences deduced from the full-length nucleotide sequences for signal sequence by using software such as PSORT (K. Nakai & M. Kanehisa, Genomics, 14: 897-991 (1992), for transmembrane region by using software such as SOSUI (T. Hirokawa et al., Bioinformatics, 14:378-379 (1998); Mitsui Knowledge Industry Co., Ltd.) or for motif by using software such as Pfam (http://www.sanger.ac.uk/Software/Pfam/index.shtml) or PROSITE (http://www.expasy.ch/prosite). As a matter of course, the functions are often predictable by using partial sequence information (preferably 300 nucleotides or more) instead of the full-length nucleotide sequences. However, the result of the prediction obtained by using partial sequence information does not always agree with the result obtained by using full-length nucleotide sequence, and thus it is needless to say that the prediction of function is preferably performed based on the full-length nucleotide sequences.

Homology search using each of GenBank, Swiss-Prot and UniGene was performed for the 826 clones whose full-length nucleotide sequences had been determined (HEMBA1005337, NT2RM1000407, NT2RM2001767, and NT2RP3003939 are not full-length). The amino acid sequences deduced from the full-length nucleotide sequences were searched for functional domains by using analytical software programs, PSORT, SOSUI and Pfam. Based on the results, proteins encoded by the cDNA clones were grouped into some categories and their functions were predicted.

The following 437 clones were categorized into secretory and/or membrane proteins. The clones categorized into secretory and/or membrane proteins are those which matched the full-length sequences of Swiss-Prot database with the keywords "growth factor", "cytokine", "hormone", "signal", "transmembrane", "membrane", "extracellular matrix", "receptor", "G-protein coupled receptor", "ionic channel", "voltage-gated channel", "calcium channel", "cell adhesion", "collagen" or "connective tissue"; those which matched the data, suggesting that the proteins are secretory and/or membrane proteins; or those which matched with the full-length sequences of GenBank or UniGene database similar description; and, further, those predicted to have an N-terminal signal sequence or a transmembrane region as a result of domain search for the amino acid sequences deduced from the full-length nucleotide sequences. BNGH41000020, BNGH41000087, BNGH41000091, HEMBA1000121, HEMBA1000128, HEMBA1000349, HEMBA1000477, HEMBA1000590, HEMBA1000713, HEMBA1000732, HEMBA1000745, HEMBA1000835, HEMBA1000940, HEMBA1000962, HEMBA1001221, HEMBA1001228, HEMBA1001621, HEMBA1002131, HEMBA1002163, HEMBA1002167, HEMBA1002178, HEMBA1002195, HEMBA1002227, HEMBA1002420, HEMBA1002421, HEMBA1002767, HEMBA1003047, HEMBA1003101, HEMBA1003230, HEMBA1003392, HEMBA1003530, HEMBA1003602, HEMBA1003732, HEMBA1003945, HEMBA1004110, HEMBA1004250, HEMBA1004391, HEMBA1004444, HEMBA1004454, HEMBA1004505, HEMBA1004797, HEMBA1004982, HEMBA1005070, HEMBA1005449, HEMBA1005522, HEMBA1005545, HEMBA1005698, HEMBA1005945, HEMBA1006171, HEMBA1006299, HEMBA1006311, HEMBA1006335, HEMBA1006357, HEMBA1006430, HEMBA1006482, HEMBA1006707, HEMBA1006724, HEMBA1006749, HEMBA1006902, HEMBA1006960, HEMBA1007241, HEMBB1000407, HEMBB1000447, HEMBB1000567, HEMBB1000679, HEMBB1000881, HEMBB1001026, HEMBB1001048, HEMBB1001407, HEMBB1001530, HEMBB1001573, HEMBB1001847, HEMBB1001978, HEMBB1002041, HEMBB1002162, HEMBB1002245, HEMBB1002427, HEMBB1002693, MAMMA1000102, MAMMA1000106, MAMMA1000118, MAMMA1000141, MAMMA1000204, MAMMA1000226, MAMMA1000457, MAMMA1000473, MAMMA1000496, MAMMA1000591, MAMMA1000681, MAMMA1000810, MAMMA1000986, MAMMA1000994, MAMMA1001043, MAMMA1001141, MAMMA1001237, MAMMA1001344, MAMMA1001418, MAMMA1001893, MAMMA1001957, MAMMA1001978, MAMMA1002070, MAMMA1002091, MAMMA1002095, MAMMA1002165, MAMMA1002234, MAMMA1002586, MAMMA1002633, MAMMA1003126, NT2RM1000462, NT2RM1000542, NT2RM1000580, NT2RM1000855, NT2RM1000858, NT2RM1000899, NT2RM2000241, NT2RM2000410, NT2RM2000423, NT2RM2000565, NT2RM2001626, NT2RM2001792, NT2RM2001939, NT2RM2001941, NT2RM4000198, NT2RM4000284, NT2RM4000417, NT2RM4000444, NT2RM4000587, NT2RM4000593, NT2RM4000648, NT2RM4000761, NT2RM4000997, NT2RM4001325, NT2RM4001735, NT2RM4001768, NT2RM4001843, NT2RM4002352, NT2RP1000050, NT2RP1000181, NT2RP1000261, NT2RP1000300, NT2RP1000325, NT2RP1000448, NT2RP1000551, NT2RP1000613, NT2RP1000981, NT2RP1001004, NT2RP1001563, NT2RP2000479, NT2RP2000533, NT2RP2000616, NT2RP2000649, NT2RP2000663, NT2RP2000694, NT2RP2000818, NT2RP2000903, NT2RP2001200, NT2RP2001276, NT2RP2001480, NT2RP2001495, NT2RP2001514, NT2RP2001755, NT2RP2001915, NT2RP2001956, NT2RP2002063, NT2RP2002188, NT2RP2002232, NT2RP2002527, NT2RP2002533, NT2RP2002721, NT2RP2002824, NT2RP2002942, NT2RP2002976, NT2RP2003042, NT2RP2003210, NT2RP2003383, NT2RP2003390, NT2RP2003469, NT2RP2003593, NT2RP2003655, NT2RP2003664, NT2RP2003950, NT2RP2004179, NT2RP2004205, NT2RP2004495, NT2RP2004524, NT2RP2004556, NT2RP2004606, NT2RP2004648, NT2RP2004794, NT2RP2005027, NT2RP2005163, NT2RP2005181, NT2RP2005378, NT2RP2005463, NT2RP2005541, NT2RP2005597, NT2RP2005666, NT2RP2005883, NT2RP2005994, NT2RP2006004, NT2RP2006042, NT2RP2006269, NT2RP2006512, NT2RP2006580, NT2RP3000169, NT2RP3000171, NT2RP3000304, NT2RP3000436, NT2RP3000460, NT2RP3000616, NT2RP3000676, NT2RP3000721, NT2RP3000818, NT2RP3000907, NT2RP3000921, NT2RP3001012, NT2RP3001159, NT2RP3001195, NT2RP3001240, NT2RP3001271, NT2RP3001322, NT2RP3001388, NT2RP3001560, NT2RP3001592, NT2RP3001650, NT2RP3001738, NT2RP3001858, NT2RP3002015, NT2RP3002160, NT2RP3002311, NT2RP3002342, NT2RP3002411, NT2RP3002737, NT2RP3002790, NT2RP3002836, NT2RP3002900, NT2RP3002958, NT2RP3003000, NT2RP3003076, NT2RP3003354, NT2RP3003532, NT2RP3003535, NT2RP3003614, NT2RP3004025, NT2RP3004075, NT2RP3004083, NT2RP3004130, NT2RP3004133, NT2RP3004309, NT2RP3004345, NT2RP3004406, NT2RP3004481, NT2RP3004552, NT2RP3004625, NT2RP3004647, NT2RP4001001, NT2RP4001009, NT2RP4001467, NT2RP4001879, NT2RP4002187, NT2RP4002451, NT2RP4002750, OVARC1000003, OVARC1000105, OVARC1000298, OVARC1000307, OVARC1000313, OVARC1000410, OVARC1000439, OVARC1000553, OVARC1000811, OVARC1000873, OVARC1000956, OVARC1001030, OVARC1001163, OVARC1001336, OVARC1001570, OVARC1001607, OVARC1001725, OVARC1001991, PLACE1000033, PLACE1000231, PLACE1000560, PLACE1000740, PLACE1000912, PLACE1000914, PLACE1000927, PLACE1001016, PLACE1001123, PLACE1001183, PLACE1001231, PLACE1001340, PLACE1001401, PLACE1001407, PLACE1001464, PLACE1001516, PLACE1001536, PLACE1001564, PLACE1001655, PLACE1001795, PLACE1001836, PLACE1001918, PLACE1001949, PLACE1002080, PLACE1002095, PLACE1002355, PLACE1002374, PLACE1002518, PLACE1002547, PLACE1002726, PLACE1002905, PLACE1002911, PLACE1002967, PLACE1003407, PLACE1003573, PLACE1003737, PLACE1003772, PLACE1003839, PLACE1003845, PLACE1003852, PLACE1004279, PLACE1004282, PLACE1004441, PLACE1004450, PLACE1004482, PLACE1004520, PLACE1004630, PLACE1004637, PLACE1004648, PLACE1004816, PLACE1005003, PLACE1005005, PLACE1005031, PLACE1005383, PLACE1005410, PLACE1005426, PLACE1005544, PLACE1005569, PLACE1005660, PLACE1005725, PLACE1005745, PLACE1005878, PLACE1005927, PLACE1006071, PLACE1006093, PLACE1006208, PLACE1006277, PLACE1006290, PLACE1006443, PLACE1006716, PLACE1006809, PLACE1006959, PLACE1007081, PLACE1007096, PLACE1007296, PLACE1007626, PLACE1007845, PLACE1007881, PLACE1008359, PLACE1008469, PLACE1008716, PLACE1008744, PLACE1008985, PLACE1009067, PLACE1009196, PLACE1009279, PLACE1009527, PLACE1009546, PLACE1009600, PLACE1009982, PLACE1010011, PLACE1010078, PLACE1010251, PLACE1010445, PLACE1010713, PLACE1010784, PLACE1010827, PLACE1010968, PLACE1011116, PLACE1011181, PLACE1011236, PLACE1011516, PLACE1011708, PLACE3000181, PLACE3000213, PLACE4000354, SKNMC1000004, SKNMC1000014, SKNMC1000082, THYRO1000036, THYRO1000099, THYRO1000196, THYRO1000400, THYRO1000584, THYRO1000678, THYRO1000776, THYRO1000795, THYRO1000956, THYRO1001102, THYRO1001113, THYRO1001205, THYRO1001237, THYRO1001242, THYRO1001266, THYRO1001327, THYRO1001456, THYRO1001478, THYRO1001523, THYRO1001529, THYRO1001641, THYRO1001702, THYRO1001725, Y79AA1000207, Y79AA1000226, Y79AA1000270, Y79AA1000426, Y79AA1000521, Y79AA1000876, Y79AA1000888, Y79AA1000959, Y79AA1001013, Y79AA1001212, Y79AA1001264, Y79AA1001328, Y79AA1001426, Y79AA1001427, Y79AA1001430, Y79AA1001727, Y79AA1001787, Y79AA1001795, Y79AA1001799, Y79AA1001803, Y79AA1002022, Y79AA1002058, Y79AA1002129, Y79AA1002213, Y79AA1002373,

The following 146 clones were categorized into glycoprotein-associated proteins. The clones categorized into glycoprotein-associated proteins are those which matched the full-length sequences of Swiss-Prot database with the keyword "glycoprotein"; those which matched the data suggesting that the proteins are glycoprotein; or those which matched the full-length sequences of GenBank or UniGene database with similar description. BNGH41000087, BNGH41000091, HEMBA1000349, HEMBA1000590, HEMBA1000745, HEMBA1000835, HEMBA1001221, HEMBA1001228, HEMBA1001621, HEMBA1002131, HEMBA1002178, HEMBA1002421, HEMBA1002767, HEMBA1003230, HEMBA1003392, HEMBA1004250, HEMBA1004391, HEMBA1004444, HEMBA1004505, HEMBA1005449, HEMBA1005522, HEMBA1005545, HEMBA1006707, HEMBA1006749, HEMBA1006902, HEMBB1000679, HEMBB1000881, HEMBB1001048, HEMBB1002120, HEMBB1002245, HEMBB1002427, MAMMA1000102, MAMMA1000591, MAMMA1000681, MAMMA1001043, MAMMA1001237, MAMMA1002070, MAMMA1002586, MAMMA1003126, NT2RM1000462, NT2RM1000580, NT2RM2001792, NT2RM2001818, NT2RM2001939, NT2RM2001941, NT2RM4000198, NT2RM4000284, NT2RM4000417, NT2RM4000648, NT2RM4000997, NT2RM4001325, NT2RM4002352, NT2RP1000613, NT2RP1000981, NT2RP1001004, NT2RP2000616, NT2RP2000694, NT2RP2000903, NT2RP2001480, NT2RP2001755, NT2RP2002533, NT2RP2003042, NT2RP2003210, NT2RP2004205, NT2RP2004606, NT2RP2005027, NT2RP2005181, NT2RP2005541, NT2RP2005597, NT2RP2005883, NT2RP2006004, NT2RP2006042, NT2RP2006269, NT2RP3000304, NT2RP3000616, NT2RP3000921, NT2RP3001650, NT2RP3002160, NT2RP3002737, NT2RP3002958, NT2RP3003000, NT2RP3003532, NT2RP3004130, NT2RP3004133, NT2RP3004481, NT2RP3004552, NT2RP3004640, NT2RP4000108, NT2RP4001467, NT2RP4002750, OVARC1000003, OVARC1000553, OVARC1000811, OVARC1000873, OVARC1001336, OVARC1001607, OVARC1001991, PLACE1000033, PLACE1000740, PLACE1001016,
PLACE1001123, PLACE1001231, PLACE1001464, PLACE1001655, PLACE1001836, PLACE1002355, PLACE1002374, PLACE1002905, PLACE1002911, PLACE1003573, PLACE1003737, PLACE1003772, PLACE1003839, PLACE1004282, PLACE1004441, PLACE1004450, PLACE1004520, PLACE1004648, PLACE1005003, PLACE1005426, PLACE1006071, PLACE1006073, PLACE1006290, PLACE1007081, PLACE1007845, PLACE1008716, PLACE1008744, PLACE1008985, PLACE1010251, PLACE1010784, PLACE1010968, PLACE1011116, PLACE3000181, PLACE3000213, PLACE4000354, THYRO1000036, THYRO1000196, THYRO1000584, THYRO1000956, THYRO1001266, Y79AA1000270, Y79AA1000426, Y79AA1001727, Y79AA1001795, Y79AA1002022, Y79AA1002213,

The following 57 clones were categorized into signal transduction-associated proteins. The clones categorized into signal transduction-associated proteins are those which matched the full-length sequences of Swiss-Prot database with the keywords "serine/threonine-protein kinase", "tyrosine-protein kinase" or "SH3 domain"; those which matched the data suggesting that the proteins are signal transduction-associated proteins (for example, "ADP-ribosylation factor"); or those which matched the full-length sequences of GenBank or UniGene database with similar description; and, further, those which was similarly predicted to be signal transduction-associated proteins based on the matching data of Pfam.
HEMBA1000006, HEMBA1002195, HEMBA1002227, HEMBA1002551, HEMBA1005084, HEMBA1005929, HEMBA1006658, HEMBA1006916, MAMMA1000881, MAMMA1001150, MAMMA1001310, MAMMA1002142, NT2RM2001902, NT2RP1001020, NT2RP1001031, NT2RP2001469, NT2RP2001529, NT2RP2001769, NT2RP2003179, NT2RP2003545, NT2RP2004670, NT2RP3000011, NT2RP3000022, NT2RP3000172, NT2RP3000201, NT2RP3000820, NT2RP3003527, NT2RP3003849, NT2RP3003874, NT2RP3004067, NT2RP4000634, NT2RP4000962, OVARC1000255, OVARC1000529, OVARC1000916, OVARC1001338, OVARC1001569, PLACE1002329, PLACE1003135, PLACE1003598, PLACE1005519, PLACE1006208, PLACE1008282, PLACE1008297, PLACE1010081, PLACE1011364, PLACE1011824, THYRO1001457, THYRO1001593, THYRO1001700, THYRO1001770, Y79AA1000777, Y79AA1000967, Y79AA1002376, Y79AA1002381, HEMBB1000668, NT2RM4001377

The following 81 clones were categorized into transcription-associated proteins. The clones categorized into transcription-associated proteins are those which keywords "transcription regulation", "zinc finger" or "homeobox" matched the full-length sequences of Swiss-Prot database; those which the matched the data suggesting that the proteins were transcription-associated proteins; or those which matched the full-length sequences of GenBank or UniGene database with similar description; and, further, those which was similarly predicted to be transcription-associated proteins based on the matching data of Pfam.
HEMBA1000462, HEMBA1000671, HEMBA1001297, HEMBA1001390, HEMBA1001886, HEMBA1002048, HEMBA1003120, HEMBA1003497, HEMBA1004785, HEMBA1005230, HEMBA1005246, HEMBA1006276, HEMBA1006572, HEMBA1007226, HEMBB1000106, HEMBB1000905, HEMBB1001959, HEMBB1002051, HEMBB1002661, MAMMA1001094, MAMMA1001532, MAMMA1001615, NT2RM1000789, NT2RM2000632, NT2RM2000773, NT2RM4000326, NT2RP1000271, NT2RP1000468, NT2RP2000092, NT2RP2000610, NT2RP2000712, NT2RP2000739, NT2RP2001538, NT2RP2001662, NT2RP2001817, NT2RP2001948, NT2RP2002564, NT2RP2002974, NT2RP2003138, NT2RP2003302, NT2RP2003940, NT2RP2004108, NT2RP2004847, NT2RP2005247, NT2RP2005391, NT2RP2005535, NT2RP2005774, NT2RP2005941, NT2RP2006092, NT2RP3000148, NT2RP3000232, NT2RP3000378, NT2RP3000652, NT2RP3001976, NT2RP3004090, NT2RP3004119, NT2RP3004294, OVARC1001049, OVARC1001086, OVARC1001132, OVARC1001807, PLACE1000258, PLACE1000442, PLACE1000907, PLACE1003529, PLACE1004166, PLACE1004168, PLACE1004887, PLACE1005250, PLACE1005682, PLACE1006079, PLACE1008549, PLACE1011407, PLACE1011978, THYRO1000580, Y79AA1000030, Y79AA1001090, Y79AA1001523, Y79AA1002334, Y79AA1002378, HEMBB1002302,

The following 85 clones were categorized into disease-associated proteins. The clones categorized into disease-associated proteins are those which matched the full-length sequences of Swiss-Prot database with the keywords "disease mutation" or "syndrome"; those which matched the data suggesting that the proteins are disease-associated proteins; or those which matched the full-length sequences of Swiss-Prot database and GenBank or UniGene database where the matched sequences are those of genes or proteins which had been deposited in the database of Online Mendelian Inheritance in Man (OMIM) (http://www.ncbi.nlm.nih.gov/Omim/), which is a database of human genes and diseases.
BNGH41000020, HEMBA1000349, HEMBA1000590, HEMBA1000671, HEMBA1000835, HEMBA1001184, HEMBA1001228, HEMBA1001886, HEMBA1003120, HEMBA1004250, HEMBA1005246, HEMBA1005267, HEMBA1006707, HEMBA1006749, HEMBA1006902, HEMBA1006916, HEMBA1007013, HEMBB1002120, MAMMA1000204, MAMMA1002080, NT2RM2000632, NT2RM2001126, NT2RM2001558, NT2RP1000271, NT2RP1000465, NT2RP1000579, NT2RP2000447, NT2RP2000514, NT2RP2000739, NT2RP2001223, NT2RP2001529, NT2RP2001562, NT2RP2002674, NT2RP2003369, NT2RP2004108, NT2RP2004205, NT2RP2005535, NT2RP2005941, NT2RP2006004, NT2RP3000059, NT2RP3000125, NT2RP3000201, NT2RP3000232, NT2RP3000616, NT2RP3000677, NT2RP3000838, NT2RP3000921, NT2RP3001542, NT2RP3002286, NT2RP3002721, NT2RP3002737, NT2RP3002738, NT2RP3004481, OVARC1000208, OVARC1000275, OVARC1000331, OVARC1000410, OVARC1001086, OVARC1001132, OVARC1001607, OVARC1001725, OVARC1001952, PLACE-1000258, PLACE1000442, PLACE1000907, PLACE1001100, PLACE1001500, PLACE1002905, PLACE1002967, PLACE1003407, PLACE1003428, PLACE1005005, PLACE1005239, PLACE1005815, PLACE1007028, PLACE1008716, PLACE1011407, PLACE1011978, PLACE2000118, THYRO1000580, THYRO1000866, THYRO1001071, THYRO1001478, Y79AA1001062, Y79AA1001530,

It is unclear, by the analyses so far, whether or not the remaining 212 clones encode proteins belonging to any of the categories of secretory and/or membrane proteins, glycoprotein-associated proteins, signal transduction-associated proteins, transcription-associated proteins or disease-associated proteins. Nonetheless, it is still possible for these clones to encode secretory and/or membrane proteins, glycoprotein-associated proteins, signal transduction-associated proteins, transcription-associated proteins, or disease-associated proteins. On the other hand, some of these clones can be presumed to have functions other than those as secretory and/or membrane proteins, glycoprotein-associated proteins, signal transduction-associated proteins, transcription-associated proteins and disease-associated proteins.

Among the 212 clones, the following clones presumably belong to the categories of enzymes and/or metabolism-associated proteins, cell division- and/or cell proliferation-associated proteins, cytoskeleton-associated proteins, nuclear proteins, DNA- and/or RNA-binding proteins, ATP- and/or GTP-binding proteins, protein synthesis- and/or protein transport-associated proteins, or cellular defense-associated proteins, although it is unclear whether or not the clones belong to any of the categories of secretory and/or transmembrane proteins, glycoprotein-associated proteins, signal transduction-associated proteins, transcription-associated proteins, and disease-associated proteins.

The following 10 clones presumably belong to the category of enzymes and/or metabolism-associated proteins. The clones herein defined as clones presumably belonging to the category of enzymes and/or metabolism-associated proteins matched data containing keywords such as "metabolism", "oxidoreductase" and "E.C. No. (Enzyme commission number)".
HEMBA1003315, HEMBB1002465, MAMMA1000614, NT2RP2000178, NT2RP2001388, NT2RP2001903, NT2RP2002304, NT2RP2005878, NT2RP3001685, PLACE1006219

The following 4 clones presumably belong to the category of cell division- and/or cell proliferation-associated proteins. The cDNA clones were herein defined as clones presumably belonging to the category of cell division- and/or cell proliferation-associated proteins matched data containing keywords such as "cell division", "cell cycle", "mitosis", "chromosomal protein", "cell growth" and "apoptosis".
MAMMA1000403, NT2RM2000497, NT2RP2000394, Y79AA1002121

The following 6 clones presumably belong to the category of cytoskeleton-associated proteins. The cDNA clones were herein defined as clones presumably belonging to the category of cytoskeleton-associated proteins matched data containing keywords such as "structural protein", "cytoskeleton", "actin-binding" and "microtubules".
MAMMA1001609, NT2RM2000589, NT2RP3000063, PLACE1004078, PLACE1004492, PLACE1008657

The following 7 clones presumably belong to the category of nuclear proteins. The cDNA clones were herein defined as clones presumably belonging to the category of nuclear proteins matched data containing keywords such as "nuclear protein".
HEMBA1001878, HEMBA1002992, MAMMA1000614, NT2RM4000965, NT2RM2001738, NT2RP2001388, Y79AA1002121

The following 5 clones presumably belong to the category of DNA- and/or RNA-binding proteins. The cDNA clones were herein defined as clones presumably belonging to the category of DNA- and/or RNA-binding proteins matched data containing keywords such as "DNA-binding" and "RNA-binding".
HEMBA1003072, HEMBA1006770, HEMBA1007332, NT2RM2000497, Y79AA1002121

The following 7 clones presumably belong to the category of ATP- and/or GTP-binding proteins. The cDNA clones were herein defined as clones presumably belonging to the category of ATP- and/or GTP-binding proteins matched data containing keywords such as "ATP-binding" and "GTP-binding".
HEMBA1002316, MAMMA1001609, NT2RM2000306, NT2RM2000497, NT2RP2000178, NT2RP3003729, PLACE1004305

The following 7 clones presumably belong to the category of protein synthesis- and/or protein transport-associated proteins. The cDNA clones were herein defined as clones presumably belonging to the category of protein synthesis-associated and/or protein transport-associated proteins matched data containing keywords such as "translation regulation", "protein biosynthesis", "amino-acid biosynthesis", "ribosomal protein", "protein transport" and "signal recognition particle".
NT2RM4000965, NT2RP2005069, NT2RP3000481, NT2RP3000789, NT2RP4001877, OVARC1001833, OVARC1002058,

The following 1 clone presumably belongs to the category of cellular defense-associated proteins. The cDNA clones were herein defined as clones presumably belonging to the category of cellular defense-associated proteins matched data containing keywords such as "heat shock", "DNA repair" and "DNA damage".
PLACE1005539

Although it is unclear whether or not 26 out of 174 clones other than the above-mentioned clones belong to any of the above-described categories, these clones are predicted to have some functions, based on the homology search using their full-length sequences thereof. The clone names and the gene definitions found in the result of homology search are shown below, separated with a double-slash mark, //.
HEMBA1000634//Homo sapiens T-cell activation protein (PGR1) gene, complete cds.
HEMBA1002524//Human MHC Class I region proline rich protein mRNA, complete cds.
HEMBA1003399//MVP1 PROTEIN.
HEMBA1005489//Mus musculus semaphorin cytoplasmic domain-associated protein 3A (Semcap3) mRNA, complete cds.
HEMBB1000542//Mus musculus bromodoma in-conta in ing prote in BP75 mRNA, complete cds.
MAMMA1000788//Bos taurus P14 (p14) mRNA, complete cds.
MAMMA1002128//ABC1 PROTEIN HOMOLOG PRECURSOR.
NT2RM2000514//Homo sapiens F-box protein Fbx21 (FBX21) mRNA, complete cds.
NT2RM2000622//Mus musculus F-box protein FBL10 mRNA, partial cds.
NT2RM4000100//Homo sapiens Leman coiled-coil protein (LCCP) mRNA, complete cds.
NT2RP2005425//Homo sapiens mRNA for AKAP450 protein.
NT2RP3001170//Mus musculus act ivity-dependent neuroprotect ive prote in (Adnp) mRNA, complete cds.
NT2RP3002571//Bos taurus mRNA for lyncein.
NT2RP3004557//Human Ki nuclear autoantigen mRNA, complete cds.
OVARC1001596//Homo sapiens Arf-like 2 binding protein BART1 mRNA, complete cds.
PLACE1002153//Homo sapiens TACC2 protein (TACC2) mRNA, partial cds.
PLACE1003163//Homo sapiens DBI-related protein mRNA, complete cds.
PLACE1005736//Human mRNA for BAS-GRIP protein.
PLACE1007702//Mus musculus TRA1 mRNA, complete cds.
PLACE1011045//Homo sapiens E1-like protein mRNA, complete cds.
THYRO1000061//Mus musculus mRNA for UBE-1c1, UBE-1c2, UBE-1c3, complete cds.
THYRO1000964//Drosophila melanogaster Pelle associated protein Pellino (Pli) mRNA, complete cds.
Y79AA1000776//Mus musculus mRNA for GSG1, complete cds.
Y79AA1001056//Homo sapiens MAID protein mRNA, complete cds.
Y79AA1001272//Homo sapiens retinoic acid repressible protein (RARG-1) mRNA, complete cds.
Y79AA1001793//Mus musculus mRNA for GSG1, complete cds.

So far, useful information for presuming the functions are unavailable for the remaining 148 clones, whose names are listed below.
HEMBA1000275, HEMBA1000300, HEMBA1000443, HEMBA1000875, HEMBA1000907, HEMBA1001272, HEMBA1001296, HEMBA1001563, HEMBA1002164, HEMBA1002239, HEMBA1002985, HEMBA1003294, HEMBA1003487, HEMBA1004007, HEMBA1004067, HEMBA1004085, HEMBA1004952, HEMBA1004971, HEMBA1005145, HEMBA1005430, HEMBA1005913, HEMBA1006016, HEMBA1006517, HEMBA1006544, HEMBA1006912, HEMBA1007057, HEMBA1007063, HEMBA1007291, HEMBB1000276, HEMBB1000309, HEMBB1000642, HEMBB1001200, HEMBB1001547, HEMBB1002039, HEMBB1002228, HEMBB1002663, MAMMA1000046, MAMMA1000449, MAMMA1000528, MAMMA1000652, MAMMA1000706, MAMMA1000814, MAMMA1001066, MAMMA1001284, MAMMA1001623, MAMMA1001634, MAMMA1001901, MAMMA1002087, MAMMA1002205, MAMMA1002224, NT2RM2000582, NT2RM2001643, NT2RM4000115, NT2RM4000295, NT2RM4001321, NT2RP1000002, NT2RP1000239, NT2RP1000679, NT2RP1000740, NT2RP1000903, NT2RP2000240, NT2RP2001878, NT2RP2001921, NT2RP2002015, NT2RP2002409, NT2RP2002510, NT2RP2003599, NT2RP2003931, NT2RP2004069, NT2RP2004141, NT2RP2004447, NT2RP2004837, NT2RP2005514, NT2RP2005632, NT2RP2005887, NT2RP2006099, NT2RP2006134, NT2RP3000427, NT2RP3000444, NT2RP3000645, NT2RP3000871, NT2RP3001044, NT2RP3001061, NT2RP3001754, NT2RP3002281, NT2RP3002324, NT2RP3002353, NT2RP3002409, NT2RP3002448, NT2RP3002664, NT2RP3002887, NT2RP3002983, NT2RP3003448, NT2RP3003469, NT2RP3003473, NT2RP3003559, NT2RP3003963, NT2RP3004000, NT2RP3004202, NT2RP3004321,
NT2RP3004355, NT2RP3004374, NT2RP4002715, OVARC1000090, OVARC1000137, OVARC1000467, OVARC1000775, OVARC1000853, OVARC1000995, OVARC1001222, OVARC1001260, OVARC1001727, OVARC1002178, PLACE1000986, PLACE1001114, PLACE1001229, PLACE1001788, PLACE1003438, PLACE1003460, PLACE1003644, PLACE1004028, PLACE1004199, PLACE1004519, PLACE1005601, PLACE1005669, PLACE1005768, PLACE1006515, PLACE1006786, PLACE1007040, PLACE1007077, PLACE1007591, PLACE1007971, PLACE1008984, PLACE1009735, PLACE2000219, PLACE4000455, THYRO1000846, THYRO1000999, THYRO1001063, THYRO1001128, THYRO1001471, THYRO1001495, THYRO1001608, THYRO1001803, Y79AA1000127, Y79AA1000750, Y79AA1001592, Y79AA1001863,

In the 437 clones categorized into secretory and/or membrane proteins by using their full-length sequences, 410 clones were also predicted to encode proteins having functions of secretory and/or membrane proteins by using their partial nucleotide sequences. In the 146 clones categorized into glycoprotein-associated proteins by using their full-length sequences, 124 clones were also predicted to encode proteins having functions of glycoprotein-associated proteins by using their partial nucleotide sequences. In the 57 clones categorized into signal transduction-associated proteins by using their full-length sequences, 46 clones were also predicted to encode proteins having functions of signal transduction-associated proteins by using their partial nucleotide sequences. In the 81 clones categorized into transcription-associated proteins by using their full-length sequences, 57 clones were also predicted to encode proteins having functions of transcription-associated proteins by using their partial nucleotide sequences. In the 85 clones categorized into disease-associated proteins by using their full-length sequences, 6 clones were also predicted to encode proteins having functions of disease-associated proteins by using their partial nucleotide sequences. The number of clones, which were predicted to encode disease-associated proteins based on the full-length nucleotide sequences, is much greater than that predicted based on the partial sequences. The reason is that the full-length sequences were categorized by using the data found in the OMIM database into the category of disease-associated proteins.

In some cases, the predicted functions based on the partial sequences are different from those based on the full-length sequences. The reason is that a protein does not always belong solely to a single category of the above-described functional categories, and therefore, it is possible for the protein to belong to both of the predicted functional categories. Besides, additional functions can be found for the clones classified into these functional categories by further analyses.

The following list shows the cDNA clones predicted and selected on the basis of the partial sequences (5' sequences) as cDNAs encoding secretory and/or membrane proteins, glycoprotein-associated proteins, signal transduction-associated proteins, transcription-associated proteins, or disease-associated proteins.

The clones that are selected by the score in the ATGpr and by the PSORT for the existence of a signal sequence can be expected to encode a secretory or membrane protein since they are predicted to possess the secretion signal or a transmembrane region. The clones that are selected by the score in the ATGpr and by the PSORT for the existence of a signal sequence are listed below (254 clones).
HEMBA1000300 HEMBA1000713 HEMBA1000907
HEMBA1000962 HEMBA1001272 HEMBA1001297
HEMBA1002164 HEMBA1002239 HEMBA1002420
HEMBA1002421 HEMBA1003101 HEMBA1003294
HEMBA1003399 HEMBA1003602 HEMBA1003732
HEMBA1004110 HEMBA1004797 HEMBA1005430
HEMBA1006016 HEMBA1006171 HEMBA1006311
HEMBA1006335 HEMBA1006357 HEMBA1006572
HEMBA1006658 HEMBA1006707 HEMBA1006902
HEMBA1006960 HEMBA1007013 HEMBB1000276
HEMBB1000447 HEMBB1000567 HEMBB1000642
HEMBB1000905 HEMBB1001200 HEMBB1001407
HEMBB1001530 HEMBB1001547 HEMBB1001978
HEMBB1002162 HEMBB1002228 HEMBB1002245
HEMBB1002427 HEMBB1002465 HEMBB1002663
HEMBB1002693 MAMMA1000046 MAMMA1000102
MAMMA1000118 MAMMA1000141 MAMMA1000449
MAMMA1000457 MAMMA1000591 MAMMA1000652
MAMMA1000681 MAMMA1000986 MAMMA1000994
MAMMA1001043 MAMMA1001141 MAMMA1001284
MAMMA1001310 MAMMA1001344 MAMMA1001893
MAMMA1001901 MAMMA1001957 MAMMA1002070
MAMMA1002087 MAMMA1002165 MAMMA1002205
MAMMA1002224 MAMMA1002633 NT2RM2000241
NT2RM2000306 NT2RM2000410 NT2RM2000514
NT2RM2001643 NT2RM2001941 NT2RM4000115
NT2RM4000997 NT2RM4001321 NT2RM4001325
NT2RM4001768 NT2RP1000050 NT2RP1000448
NT2RP1000903 NT2RP1001563 NT2RP2000479
NT2RP2001495 NT2RP2001915 NT2RP2001948
NT2RP2002015 NT2RP2002063 NT2RP2002304
NT2RP2002674 NT2RP2002721 NT2RP2003383
NT2RP2003469 NT2RP2003593 NT2RP2003599
NT2RP2003655 NT2RP2003664 NT2RP2004179
NT2RP2004447 NT2RP2004495 NT2RP2004524
NT2RP2004556 NT2RP2004837 NT2RP2005027
NT2RP2005463 NT2RP2005514 NT2RP2005887
NT2RP2006042 NT2RP2006269 NT2RP3000169
NT2RP3000460 NT2RP3000481 NT2RP3000645
NT2RP3000789 NT2RP3000818 NT2RP3001012
NT2RP3001044 NT2RP3001195 NT2RP3001560
NT2RP3001685 NT2RP3001858 NT2RP3002160
NT2RP3002281 NT2RP3002721 NT2RP3002836
NT2RP3002958 NT2RP3003076 NT2RP3003354
NT2RP3003469 NT2RP3003535 NT2RP3003559
NT2RP3003963 NT2RP3004000 NT2RP3004083
NT2RP3004133 NT2RP3004309 NT2RP3004321
NT2RP3004355 NT2RP3004374 NT2RP4001001
NT2RP4001879 NT2RP4002451 NT2RP4002715
OVARC1000208 OVARC1000298 OVARC1000439
OVARC1000775 OVARC1000811 OVARC1000853
OVARC1001222 OVARC1001727 OVARC1001807
OVARC1001833 PLACE1000231 PLACE1000560
PLACE1000740 PLACE1000912 PLACE1000914
PLACE1000927 PLACE1000986 PLACE1001100
PLACE1001183 PLACE1001229 PLACE1001407
PLACE1001536 PLACE1001788 PLACE1002080
PLACE1002095 PLACE1002374 PLACE1002518
PLACE1003407 PLACE1003428 PLACE1003460
PLACE1003839 PLACE1003845 PLACE1004028
PLACE1004199 PLACE1004282 PLACE1004305
PLACE1004482 PLACE1004637 PLACE1005005
PLACE1005250 PLACE1005383 PLACE1005410
PLACE1005544 PLACE1005569 PLACE1005601
PLACE1005660 PLACE1005669 PLACE1005725
PLACE1005768 PLACE1005927 PLACE1006079
PLACE1006093 PLACE1006219 PLACE1006277
PLACE1006443 PLACE1006786 PLACE1006809
PLACE1007040 PLACE1007096 PLACE1007296
PLACE1007626 PLACE1007971 PLACE1008469
PLACE1008984 PLACE1008985 PLACE1009067
PLACE1009196 PLACE1009527 PLACE1009982
PLACE1010251 PLACE1011236 PLACE2000219
PLACE4000455 SKNMC1000004 SKNMC1000014
THYRO1000036 THYRO1000099 THYRO1000196
THYRO1000795 THYRO1000999 THYRO1001237
THYRO1001327 THYRO1001478 THYRO1001495
THYRO1001523 THYRO1001702 THYRO1001725
Y79AA1000226 Y79AA1000270 Y79AA1000426
Y79AA1000521 Y79AA1000776 Y79AA1000959
Y79AA1001013 Y79AA1001056 Y79AA1001264
Y79AA1001328 Y79AA1001427 Y79AA1001430
Y79AA1001530 Y79AA1001592 Y79AA1001793
Y79AA1001795 Y79AA1001803 Y79AA1001863
Y79AA1002022 Y79AA1002373

In the example mentioned below, the 254 clones as described above were categorized into three groups according to their maximal value in the ATGpr and the result in the PSORT, which are shown in Table 7-10, 11, 12 (246 clones), and Table 13, 14, 15 (8 clones). In the tables, the name of clone, indicate the name of the clone that was selected by the ATGpr and the PSORT; the name of sequence indicates the name of the 5'-end sequence of the clone on the left; the maximal ATGpr score indicates the maximal ATGpr1 score of the 5'-end sequence shown on the left; and signal indicates the presence of the signal sequence according to the prediction by the PSORT. In addition, the representative sequence is the sequence that has the longest sequence among the cluster in which the 5'-end sequence on the left was included. The maximal ATGpr score and signal on the right indicate the maximal ATGpr1 score of the representative sequence, and the presence of a signal sequence in the representative sequence according to the prediction by the PSORT, respectively. The 170 clones shown in Table 7-10, having the maximal score in the ATGpr1 higher than 0.5, and predicted to possess a signal sequence by the PSORT, are very likely to be full-length and encode a secretory or membrane protein. The 35 clones in Table 11, which have the maximal score in the ATGpr1 0.3 or higher and less than 0.5, and predicted to have a signal sequence, are also as well. And, the 41 clones in Table 12, having the maximal score in the ATGpr1 0 or higher and less than 0.3, and predicted to have a signal sequence, are likely to be full-length and encode a secretory or membrane protein.

The 8 clones in Table 13 (4 clones), Table 14 (2 clones), and Table 15 (2 clones) have the maximal score in the ATGpr1 0.5 or higher, 0.3 or higher and less than 0.5, and 0 or higher and less than 0.3, respectively, and are predicted to have no signal sequence by the PSORT. However, these clones contain a region that is recognized by the PSORT to be a signal sequence within the representative sequence composing the same cluster. Thus, the clones were judged as a full-length clone which encodes a membrane protein, especially.

The clones selected by the score in the ATGpr and by the keywords in the top hit data in the SwissProt are likely to encode a secretory or membrane protein, or proteins with functions associated to signal transduction, glycoprotein, transcription, and diseases according to the respective keywords. These 659 clones are shown below. Here, top hit data is defined to be data of known amino acid sequence which is identified to be the most homologous sequence in homology search using the SwissProt.
BNGH41000020 BNGH41000087 BNGH41000091
HEMBA1000006 HEMBA1000121 HEMBA1000128
HEMBA1000275 HEMBA1000349 HEMBA1000443
HEMBA1000462 HEMBA1000477 HEMBA1000590
HEMBA1000634 HEMBA1000671 HEMBA1000732
HEMBA1000745 HEMBA1000835 HEMBA1000875
HEMBA1000907 HEMBA1000940 HEMBA1001184
HEMBA1001221 HEMBA1001228 HEMBA1001296
HEMBA1001390 HEMBA1001563 HEMBA1001621
HEMBA1001878 HEMBA1001886 HEMBA1002048
HEMBA1002131 HEMBA1002163 HEMBA1002164
HEMBA1002167 HEMBA1002178 HEMBA1002195
HEMBA1002227 HEMBA1002316 HEMBA1002421
HEMBA1002524 HEMBA1002551 HEMBA1002767
HEMBA1002985 HEMBA1002992 HEMBA1003047
HEMBA1003072 HEMBA1003101 HEMBA1003120
HEMBA1003230 HEMBA1003315 HEMBA1003392
HEMBA1003487 HEMBA1003497 HEMBA1003530
HEMBA1003945 HEMBA1004007 HEMBA1004067
HEMBA1004085 HEMBA1004250 HEMBA1004391
HEMBA1004444 HEMBA1004454 HEMBA1004505
HEMBA1004785 HEMBA1004797 HEMBA1004952
HEMBA1004971 HEMBA1004982 HEMBA1005070
HEMBA1005084 HEMBA1005145 HEMBA1005230
HEMBA1005246 HEMBA1005267 HEMBA1005337
HEMBA1005449 HEMBA1005489 HEMBA1005522
HEMBA1005545 HEMBA1005698 HEMBA1005913
HEMBA1005929 HEMBA1005945 HEMBA1006276
HEMBA1006299 HEMBA1006335 HEMBA1006430
HEMBA1006482 HEMBA1006517 HEMBA1006544
HEMBA1006572 HEMBA1006707 HEMBA1006724
HEMBA1006749 HEMBA1006770 HEMBA1006902
HEMBA1006912 HEMBA1006916 HEMBA1007013
HEMBA1007057 HEMBA1007063 HEMBA1007226
HEMBA1007241 HEMBA1007291 HEMBA1007332
HEMBB1000106 HEMBB1000309 HEMBB1000407
HEMBB1000447 HEMBB1000542 HEMBB1000567
HEMBB1000668 HEMBB1000679 HEMBB1000881
HEMBB1001026 HEMBB1001048 HEMBB1001200
HEMBB1001573 HEMBB1001847 HEMBB1001959
HEMBB1002039 HEMBB1002041 HEMBB1002051
HEMBB1002120 HEMBB1002302 HEMBB1002427
HEMBB1002661 MAMMA1000106 MAMMA1000204
MAMMA1000226 MAMMA1000403 MAMMA1000473
MAMMA1000496 MAMMA1000528 MAMMA1000591
MAMMA1000614 MAMMA1000681 MAMMA1000706
MAMMA1000788 MAMMA1000810 MAMMA1000814
MAMMA1000881 MAMMA1001043 MAMMA1001066
MAMMA1001094 MAMMA1001150 MAMMA1001237
MAMMA1001418 MAMMA1001532 MAMMA1001609
MAMMA1001615 MAMMA1001623 MAMMA1001634
MAMMA1001893 MAMMA1001957 MAMMA1001978
MAMMA1002070 MAMMA1002080 MAMMA1002091
MAMMA1002095 MAMMA1002128 MAMMA1002142
MAMMA1002165 MAMMA1002234 MAMMA1002586
MAMMA1002633 MAMMA1003126 NT2RM1000407
NT2RM1000462 NT2RM1000542 NT2RM1000580
NT2RM1000789 NT2RM1000855 NT2RM1000858
NT2RM1000899 NT2RM2000410 NT2RM2000423
NT2RM2000497 NT2RM2000565 NT2RM2000582
NT2RM2000589 NT2RM2000622 NT2RM2000632
NT2RM2000773 NT2RM2001126 NT2RM2001558
NT2RM2001626 NT2RM2001738 NT2RM2001767
NT2RM2001792 NT2RM2001818 NT2RM2001902
NT2RM2001939 NT2RM2001941 NT2RM4000100
NT2RM4000198 NT2RM4000284 NT2RM4000295
NT2RM4000326 NT2RM4000417 NT2RM4000444
NT2RM4000587 NT2RM4000593 NT2RM4000648
NT2RM4000761 NT2RM4000965 NT2RM4001377
NT2RM4001735 NT2RM4001843 NT2RM4002352
NT2RP1000002 NT2RP1000050 NT2RP1000181
NT2RP1000239 NT2RP1000261 NT2RP1000271
NT2RP1000300 NT2RP1000325 NT2RP1000465
NT2RP1000468 NT2RP1000551 NT2RP1000579
NT2RP1000613 NT2RP1000679 NT2RP1000740
NT2RP1000981 NT2RP1001004 NT2RP1001020
NT2RP1001031 NT2RP2000092 NT2RP2000178
NT2RP2000240 NT2RP2000394 NT2RP2000447
NT2RP2000514 NT2RP2000533 NT2RP2000610
NT2RP2000616 NT2RP2000649 NT2RP2000663
NT2RP2000694 NT2RP2000712 NT2RP2000739
NT2RP2000818 NT2RP2000903 NT2RP2001200
NT2RP2001223 NT2RP2001276 NT2RP2001388
NT2RP2001469 NT2RP2001480 NT2RP2001495
NT2RP2001514 NT2RP2001529 NT2RP2001538
NT2RP2001562 NT2RP2001662 NT2RP2001755
NT2RP2001769 NT2RP2001817 NT2RP2001878
NT2RP2001903 NT2RP2001921 NT2RP2001948
NT2RP2001956 NT2RP2002063 NT2RP2002188
NT2RP2002232 NT2RP2002304 NT2RP2002409
NT2RP2002510 NT2RP2002527 NT2RP2002533
NT2RP2002564 NT2RP2002824 NT2RP2002942
NT2RP2002974 NT2RP2002976 NT2RP2003042
NT2RP2003138 NT2RP2003179 NT2RP2003210
NT2RP2003302 NT2RP2003369 NT2RP2003390
NT2RP2003469 NT2RP2003545 NT2RP2003593
NT2RP2003655 NT2RP2003664 NT2RP2003931
NT2RP2003940 NT2RP2003950 NT2RP2004069
NT2RP2004108 NT2RP2004141 NT2RP2004205
NT2RP2004447 NT2RP2004606 NT2RP2004648
NT2RP2004670 NT2RP2004794 NT2RP2004847
NT2RP2005069 NT2RP2005163 NT2RP2005181
NT2RP2005247 NT2RP2005378 NT2RP2005391
NT2RP2005425 NT2RP2005535 NT2RP2005541
NT2RP2005597 NT2RP2005632 NT2RP2005666
NT2RP2005774 NT2RP2005878 NT2RP2005883
NT2RP2005941 NT2RP2005994 NT2RP2006004
NT2RP2006042 NT2RP2006092 NT2RP2006099
NT2RP2006134 NT2RP2006269 NT2RP2006512
NT2RP3000011 NT2RP3000022 NT2RP3000059
NT2RP3000063 NT2RP3000125 NT2RP3000148
NT2RP3000171 NT2RP3000172 NT2RP3000201
NT2RP3000232 NT2RP3000304 NT2RP3000378
NT2RP3000427 NT2RP3000436 NT2RP3000444
NT2RP3000481 NT2RP3000616 NT2RP3000645
NT2RP3000652 NT2RP3000676 NT2RP3000677
NT2RP3000721 NT2RP3000820 NT2RP3000838
NT2RP3000871 NT2RP3000907 NT2RP3000921
NT2RP3001012 NT2RP3001061 NT2RP3001159
NT2RP3001170 NT2RP3001195 NT2RP3001240
NT2RP3001271 NT2RP3001322 NT2RP3001388
NT2RP3001542 NT2RP3001560 NT2RP3001592
NT2RP3001650 NT2RP3001738 NT2RP3001754
NT2RP3001976 NT2RP3002015 NT2RP3002160
NT2RP3002286 NT2RP3002311 NT2RP3002324
NT2RP3002342 NT2RP3002353 NT2RP3002409
NT2RP3002411 NT2RP3002448 NT2RP3002571
NT2RP3002664 NT2RP3002737 NT2RP3002738
NT2RP3002790 NT2RP3002836 NT2RP3002887
NT2RP3002900 NT2RP3002958 NT2RP3002983
NT2RP3003000 NT2RP3003076 NT2RP3003354
NT2RP3003448 NT2RP3003473 NT2RP3003527
NT2RP3003532 NT2RP3003614 NT2RP3003729
NT2RP3003849 NT2RP3003874 NT2RP3003939
NT2RP3004025 NT2RP3004067 NT2RP3004075
NT2RP3004090 NT2RP3004119 NT2RP3004130
NT2RP3004133 NT2RP3004202 NT2RP3004294
NT2RP3004309 NT2RP3004345 NT2RP3004406
NT2RP3004481 NT2RP3004552 NT2RP3004557
NT2RP3004625 NT2RP3004640 NT2RP3004647
NT2RP4000108 NT2RP4000634 NT2RP4000962
NT2RP4001009 NT2RP4001467 NT2RP4001877
NT2RP4001879 NT2RP4002187 NT2RP4002451
NT2RP4002750 OVARC1000003 OVARC1000090
OVARC1000105 OVARC1000137 OVARC1000255
OVARC1000275 OVARC1000307 OVARC1000313
OVARC1000331 OVARC1000410 OVARC1000439
OVARC1000467 OVARC1000529 OVARC1000553
OVARC1000873 OVARC1000916 OVARC1000956
OVARC1000995 OVARC1001030 OVARC1001049
OVARC1001086 OVARC1001132 OVARC1001163
OVARC1001222 OVARC1001260 OVARC1001336
OVARC1001338 OVARC1001569 OVARC1001570
OVARC1001596 OVARC1001607 OVARC1001725
OVARC1001952 OVARC1001991 OVARC1002058
OVARC1002178 PLACE1000033 PLACE1000258
PLACE1000442 PLACE1000740 PLACE1000907
PLACE1001016 PLACE1001114 PLACE1001123
PLACE1001231 PLACE1001340 PLACE1001401
PLACE1001407 PLACE1001464 PLACE1001500
PLACE1001516 PLACE1001564 PLACE1001655
PLACE1001795 PLACE1001836 PLACE1001918
PLACE1001949 PLACE1002080 PLACE1002095
PLACE1002153 PLACE1002329 PLACE1002355
PLACE1002374 PLACE1002547 PLACE1002726
PLACE1002905 PLACE1002911 PLACE1002967
PLACE1003135 PLACE1003163 PLACE1003428
PLACE1003438 PLACE1003460 PLACE1003529
PLACE1003573 PLACE1003598 PLACE1003644
PLACE1003737 PLACE1003772 PLACE1003852
PLACE1004078 PLACE1004166 PLACE1004168
PLACE1004279 PLACE1004441 PLACE1004450
PLACE1004482 PLACE1004492 PLACE1004519
PLACE1004520 PLACE1004630 PLACE1004648
PLACE1004816 PLACE1004887 PLACE1005003
PLACE1005031 PLACE1005239 PLACE1005383
PLACE1005426 PLACE1005519 PLACE1005539
PLACE1005544 PLACE1005569 PLACE1005682
PLACE1005736 PLACE1005745 PLACE1005815
PLACE1005878 PLACE1005927 PLACE1006071
PLACE1006073 PLACE1006208 PLACE1006277
PLACE1006290 PLACE1006443 PLACE1006515
PLACE1006716 PLACE1006959 PLACE1007028
PLACE1007077 PLACE1007081 PLACE1007096
PLACE1007296 PLACE1007591 PLACE1007702
PLACE1007845 PLACE1007881 PLACE1008282
PLACE1008297 PLACE1008359 PLACE1008469
PLACE1008549 PLACE1008657 PLACE1008716
PLACE1008744 PLACE1008984 PLACE1008985
PLACE1009279 PLACE1009527 PLACE1009546
PLACE1009600 PLACE1009735 PLACE1010011
PLACE1010078 PLACE1010081 PLACE1010251
PLACE1010445 PLACE1010713 PLACE1010784
PLACE1010827 PLACE1010968 PLACE1011045
PLACE1011116 PLACE1011181 PLACE1011236
PLACE1011364 PLACE1011407 PLACE1011516
PLACE1011708 PLACE1011824 PLACE1011978
PLACE2000118 PLACE3000181 PLACE3000213
PLACE4000354 SKNMC1000014 SKNMC1000082
THYRO1000061 THYRO1000196 THYRO1000400
THYRO1000580 THYRO1000584 THYRO1000678
THYRO1000776 THYRO1000795 THYRO1000846
THYRO1000866 THYRO1000956 THYRO1000964
THYRO1001063 THYRO1001071 THYRO1001102
THYRO1001113 THYRO1001128 THYRO1001205
THYRO1001242 THYRO1001266 THYRO1001456
THYRO1001457 THYRO1001471 THYRO1001478
THYRO1001529 THYRO1001593 THYRO1001608
THYRO1001641 THYRO1001700 THYRO1001702
THYRO1001770 THYRO1001803 Y79AA1000030
Y79AA1000127 Y79AA1000207 Y79AA1000270
Y79AA1000426 Y79AA1000750 Y79AA1000777
Y79AA1000876 Y79AA1000888 Y79AA1000967
Y79AA1001062 Y79AA1001090 Y79AA1001212
Y79AA1001272 Y79AA1001426 Y79AA1001523
Y79AA1001727 Y79AA1001787 Y79AA1001799
Y79AA1001803 Y79AA1001863 Y79AA1002058
Y79AA1002121 Y79AA1002129 Y79AA1002213
Y79AA1002334 Y79AA1002376 Y79AA1002378
Y79AA1002381 NT2RP2006580

Among the clones, the following 83 clones are identical to the clones selected by the score in the ATGpr and the prediction by the PSORT for the existence of a signal sequence.
HEMBA1000907 NT2RM2000410 PLACE1000740
HEMBA1002164 NT2RM2001941 PLACE1001407
HEMBA1002421 NT2RP1000050 PLACE1002080
HEMBA1003101 NT2RP2001495 PLACE1002095
HEMBA1004797 NT2RP2001948 PLACE1002374
HEMBA1006335 NT2RP2002063 PLACE1003428
HEMBA1006572 NT2RP2002304 PLACE1003460
HEMBA1006707 NT2RP2003469 PLACE1004482
HEMBA1006902 NT2RP2003593 PLACE1005383
HEMBA1007013 NT2RP2003655 PLACE1005544
HEMBB1000447 NT2RP2003664 PLACE1005569
HEMBB1000567 NT2RP2004447 PLACE1005927
HEMBB1001200 NT2RP2006042 PLACE1006277
HEMBB1002427 NT2RP2006269 PLACE1006443
MAMMA1000591 NT2RP3000481 PLACE1007096
MAMMA1000681 NT2RP3000645 PLACE1007296
MAMMA1001043 NT2RP3001012 PLACE1008469
MAMMA1001893 NT2RP3001195 PLACE1008984
MAMMA1001957 NT2RP3001560 PLACE1008985
MAMMA1002070 NT2RP3002160 PLACE1009527
MAMMA1002165 NT2RP3002836 PLACE1010251
MAMMA1002633 NT2RP3002958 PLACE1011236
NT2RP3003076 SKNMC1000014
NT2RP3003354 THYRO1000196
NT2RP3004133 THYRO1000795
NT2RP3004309 THYRO1001478
NT2RP4001879 THYRO1001702
NT2RP4002451 Y79AA1000270
OVARC1000439 Y79AA1000426
OVARC1001222 Y79AA1001803
Y79AA1001863

The 446 clones in Table 16, 17, 18, 19, and 20, and NT2RP2006580 are predicted to encode a secretory or membrane protein. Among them, 77 clones were identical to the clones selected by the score in the ATGpr and the prediction by the PSORT for the existence of a signal sequence (overlapping with any of the 254 clones listed in Table 7-15). Besides, many clones were turned out to be identical to the clones selected as a protein associated with a glycoprotein. Also, there were clones identical to those selected as a protein associated with a disease.

The 243 clones in Table 21 are predicted to encode a glycoprotein. Among them, 53 clones were identical to those selected by the score in the ATGpr and the prediction by the PSORT for the existence of a signal sequence. And, many clones were turned out to be identical to the clones selected as a secretory or membrane protein. Moreover, there were clones identical to those selected as a protein associated with a disease.

The 51 clones in Table 22 are predicted to encode a protein associated to signal transduction.

The 130 clones in Table 23 are predicted to encode a protein associated to transcription.

The 17 clones in Table 24 are predicted to encode a protein associated with diseases.

In these clones, 532 clones have the maximal ATGpr1 score of 0.5 or higher (Table 25). 60 clones have the maximal ATGpr1 score of 0.3 or higher and less than 0.5 (Table 26 and NT2RP2006580). And 67 clones were with the maximal ATGpr1 score of 0 or higher and less than 0.3 (Table 27).

532 clones shown in Table 25, each having the maximal score in the ATGpr1 0.5 or higher, are very likely to be full-length and encode a secretory or membrane protein, or proteins associated to signal transduction, glycoprotein, transcription, or diseases. 59 clones in Table 26 and NT2RP2006580, which have the maximal score in the ATGpr1 0.3 or higher and less than 0.5, are likely to be full-length and encode a secretory or membrane protein, or proteins associated to signal transduction, glycoprotein, transcription, or diseases. 67 clones in Table 27, having the maximal score in the ATGpr1 0 or higher and less than 0.3, are still likely to be full-length and encode a secretory or membrane protein, or proteins associated to signal transduction, glycoprotein, transcription, or diseases.

This is the method for selecting the cDNA clones predicted to encode secretory and/or transmembrane proteins, glycoprotein-associated proteins, signal transduction-associated proteins, transcription-associated proteins, or disease-associated proteins on the basis of the partial sequences (5' sequences).

The polynucleotide of the present invention encodes an amino acid sequence of a functional protein such as a secretory or membrane protein, or a protein associated to signal transduction, glycoprotein, transcription, or diseases. Since the protein has the complete amino acid sequence, it is possible to analyze its biological activity by expressing the protein as a recombinant protein using an appropriate expression system, or by raising and using an antibody which specifically recognizes it.

It is possible to analyze the biological activity of a secretory protein or a membrane protein, or proteins associated to signal transduction, glycoprotein, or transcription, based on the methods in "Gene Transcription" (Hames B.D., and Higgins S.J. edit, (1993)), "Glycobiology" (Fukuda M., and Kobata A. edit, (1993)), "Growth Factors" (McKay I., and Leigh I. edit, (1993)), "Extracellular Matrix" (Haralson M.A., and Hassell J.R. edit, (1995), "Transcription Factors" (Latchman D.S. edit, (1993)), "Signal Transduction" (Milligans G. edit, (1992)), featured in "The Practical Approach Series" (IRL PRESS), or "Signal Transduction Protocols" (Kendall D.A., and Hill S.J. edit, (1995), "Glycoprotein Analysis in Biomedicine" (Hounsell E.F. edit, (1993)), featured in "Method in Molecular Biology" (Humana Press).

As to a protein associated with a disease, it is possible to perform a functional analysis as described above, but also possible to analyze correlation between the expression or the activity of the protein and a certain disease by using a specific antibody that recognizes the protein. Alternatively, it is possible to utilize the database Online Mendelian Inheritance in Man (OMIM) (http://www.ncbi.nlm.nih.gov/Omim/), which is a database of human genes and diseases, to analyze the protein. New information is constantly being deposited in the OMIM database. Therefore, it is possible for one skilled in the art to find a new relationship between a particular disease and a gene of the present invention in the updated database.

Proteins associated with diseases are useful in drug development as they can be utilized as a diagnostic marker, a drug that regulates the level of their expression and activities, or a target of gene therapy. Also, as for a secretory protein, membrane protein, or proteins associated with signal transduction, glycoprotein, or transcription, search of the OMIM with the keywords mentioned below revealed that the proteins are associated with many diseases. Also, relationship between a proteins associated to signal transduction or transcription and diseases is reported in "Transcription Factor Research-1999" (Fujii, Tamura, Morohashi, Kageyama, and Satake edit, (1999) Jikken-Igaku Zoukan, Vol.17, No.3), and "Gene Medicine" ((1999) Vol.3, No.2). Thus, not only a protein associated with diseases, but also a secretory protein, membrane protein, or protein associated with signal transduction, glycoprotein, or transcription is involved in diseases, suggesting these proteins also are very important as a target in medical industry.

Keywords used in the search of the OMIM
(1) secretion protein
(2) membrane protein
(3) channel
(4) extracellular matrix
(5) receptor
(6) glycoprotein
(7) protein kinase
(8) calmodulin kinase
(9) transcription factor

Shown in the search result are only the accession numbers in the OMIM. Using the number, data showing the relationship between a disease and a gene or protein can be seen. The OMIM data has been renewed everyday.
1) Secretion protein
   268 entries found, searching for "secretion protein"
   104760, 176860, 160900, 107400, 118910, 139320, 603850, 147572, 176880, 600946, 603215, 157147, 600174, 151675, 170280, 179512, 179513, 138120, 179509, 246700, 179510, 600626, 179511, 600998, 109270, 601489, 154545, 179490, 185860, 603216, 122559, 601746, 147290, 602672, 146770, 603062, 179508, 131230, 601591, 602421, 139250, 167805, 167770, 600041, 600564, 118825, 601146, 300090, 600753, 601652, 600759, 600768, 602434, 182590, 603166, 308230, 602534, 603489, 107470, 150390, 104610, 173120, 158106, 143890, 306900, 308700, 134797, 137350, 227500, 176300, 107730, 600760, 138079, 120180, 120160, 120150, 124092, 138160, 101000, 227600, 600509, 601199, 142410, 104311, 193400, 201910, 107300, 122560, 272800, 217000, 590050, 147670, 133170, 176730, 300300, 134370, 274600, 120140, 162151, 158070, 152790, 120120, 106100, 300200, 192340, 190160, 138040, 147470, 147620, 173350, 147380, 152200, 152760, 157145, 153450, 264080, 113811, 600937, 600840, 188545, 202110, 600514, 186590, 603372, 136435, 137241, 252800, 214500, 207750, 138850, 139191, 142640, 138130, 189907, 603692, 600633, 603355, 107270, 600377, 147892, 232200, 600281, 232800, 602358, 137035, 601771, 601769, 253200, 601933, 118444, 600270, 120700, 600945, 603732, 147660, 600761, 172400, 600823, 600877, 130080, 171060, 107740, 307800, 602843, 130660, 152780, 124020, 601124, 601340, 601604, 601610, 171050, 312060, 232700, 300159, 142703, 600734, 125255, 168450, 123812, 188540, 147940, 188450, 600839, 182452, 188400, 182280, 176760, 263200, 600264, 188826, 252650, 601185, 162641, 137216, 601398, 601538, 118888, 118445, 601745, 190180, 601922, 182098, 602008, 147440, 602384, 600031, 109160, 602663, 151670, 602682, 602730, 602779, 146880, 603061, 142704, 603140, 106150, 600732, 153620, 603318, 139392, 600042, 102200, 603493, 182100, 264300, 603795, 184600
2) Membrane protein
   1017 entries found, searching for "membrane protein"
   130500, 305360, 153330, 173610, 170995, 109270, 170993, 309060, 120920, 602333, 133740, 133710, 602690, 133730, 159430, 600897, 133090, 601178, 602413, 602003, 109280, 603237, 602173, 107776, 602334, 125305, 602335, 182879, 154045, 309845, 600594, 603718, 603241, 603214, 603657, 603177, 600182, 601476, 602879, 136950, 600723, 601114, 185880, 185881, 300096, 602257, 160900, 177070, 603062, 603344, 602977, 310200, 600959, 300100, 186945, 600039, 600267, 128240, 182900, 601097, 136430, 600946, 602534, 601047, 143450, 603141, 603700, 600579, 256540, 159440, 602414, 600403, 602048, 188860, 137290, 158343, 184756, 602910, 603179, 600279, 108733, 107770, 173335, 602625, 154050, 219800, 603850, 601028, 600447, 104225, 186946, 601767, 603143, 121015, 603215, 227400, 603735, 600179, 602421, 180721,
   176801, 176860, 600753, 603142, 176790, 600266, 601239, 115501, 143890, 121014, 121011, 125950, 603534, 304040, 601134, 600754, 601510, 601595, 190315, 300172, 602216, 602261, 602262, 602461, 131560, 179514, 179512, 176981, 142461, 139310, 312080, 176640, 128239, 185470, 310300, 601403, 601757, 273800, 151460, 176943, 104311, 168468, 120130, 602887, 600164, 601531, 601832, 104775, 600040, 603583, 176894, 602631, 166945, 182180, 120620, 141180, 601014, 139150, 182860, 177061, 600174, 180069, 191275, 104760, 601693, 300017, 603518, 601009, 134651, 601107, 603868, 600168, 136425, 603531, 603291, 600917, 603216, 102720, 300118, 179590, 135630, 602285, 107450, 602296, 303630, 176878, 120090, 600322, 138160, 601212, 603293, 131230, 112205, 600763, 600718, 300187, 170715, 601966, 300051, 602474,
   120070, 600691, 600855, 182309, 602101, 602857, 194355, 162230, 600874, 113730, 155550, 602701, 306400, 601789, 231200, 107271, 175100, 182870, 305100, 301000, 601313, 157147, 147670, 139200, 603593, 157655, 600934, 155970, 602049, 155960, 155760, 118990, 135620, 308230, 602694, 162060, 300023, 160993, 153619, 153432, 120131, 603823, 603167, 601023, 600816, 165040, 601681, 166490, 300112, 120190, 300145, 163970, 600772, 602926, 602933, 602202, 400015, 151510, 600759, 602672, 602654, 603821, 116952, 151430, 602632, 155975, 602217, 150370, 600752, 601179, 600932, 603048, 603234, 601805, 603822, 603869, 601717, 601181, 313440, 139130, 107777, 109190, 603452, 191163, 191164, 602370, 176877, 103195, 600523, 191328, 601275, 204200, 602426, 603820, 600551, 600695, 600552, 600553, 602306, 601523,
   602507, 602299, 600583, 114070, 600632, 603498, 185430, 600587, 235200, 173470, 603199, 601633, 602500, 208900, 180297, 156225, 516020, 190195, 141900, 102680, 193300, 101000, 193400, 300011, 107400, 257220, 107741, 180380, 203200, 111700, 600024, 304800, 600065, 110750, 179605, 113705, 601638, 222900, 120120, 602509, 602469, 600930, 601383, 176261, 602574, 602997, 311770, 131550, 603616, 308700, 603372, 256100, 224100, 276903, 305900, 516000, 131195, 314555, 601567, 603866, 306900, 103390, 186720, 173850, 601050, 602505, 186590, 246530, 602689, 194380, 300041, 162643, 152790, 120150, 600682, 600106, 272750, 188040, 602382, 601497, 113811, 182138, 212138, 601309, 109690, 114760, 176805, 601253, 123900, 602581, 189980, 191190, 110700, 600163, 137167, 600580, 601610, 190000, 123825, 603491, 600135, 186591, 173910, 138140, 107266, 120950, 601081, 603690, 244400, 312700, 171060, 601199, 601758, 170500, 277900, 601997, 314850, 601880, 603009, 120220, 603126, 164920, 602934, 164730, 163890, 603434, 107269, 602909, 600877, 256550, 164761, 602872, 120110, 126150, 158070, 266200, 223360, 250800, 269920, 252650, 603355, 154582, 138190, 300035, 602640, 227650, 158120, 153700, 182380, 155740, 204500, 603401, 601975, 300135, 136350, 602924, 300167, 185050, 176100, 300189, 151525, 300200, 165180, 230800, 602158, 602676, 603411, 193245, 120325, 601848, 192500, 603102, 147795, 245900, 137060, 147557, 120650, 602377, 307800, 120930, 308100, 142800, 191092, 232300, 173510, 602225, 180470, 190930, 186357, 134638, 600544, 601373, 600509, 600359, 603784, 600395, 600653, 603754, 601597, 601066,
   600185, 601295, 600978, 205400, 603274, 600418, 600839, 516050, 601691, 601007, 600650, 600308, 603261, 601193, 600004, 600017, 516040, 253800, 276901, 600019, 257200, 108780, 300037, 300104, 300126, 255125, 203300, 300191, 426000, 302060, 304700, 201475, 252010, 193210, 311030, 306250, 248600, 191740, 108360, 131244, 600423, 232200, 191305, 231680, 103320, 190180, 600493, 111200, 226200, 312600, 600170, 111680, 186910, 203100, 600536, 600238, 186830, 186760, 186745, 186711, 106180, 112203, 103180, 182530, 182160, 600644, 307030, 192321, 600667, 125647, 179080, 114207, 114860, 176000, 116930, 600748, 173515, 173325, 600377, 171760, 171050, 118425, 170260, 191315, 600798, 600821, 600823, 600444, 600840, 159465, 600857, 158380, 600867, 154360, 152427, 150330, 110900, 147840, 147360, 147280,
   146880, 312610, 120940, 142871, 142790, 600937, 142600, 134390, 111250, 600979, 600997, 142460, 186845, 134635, 601017, 139191, 139090, 138850, 601040, 138720, 122561, 131100, 123610, 217070, 100500, 603377, 602354, 603302, 603207, 603086, 602188, 602095, 603867, 603842, 603798, 602602, 601194, 602607, 603713, 603681, 601252, 603648, 603646, 603644, 601282, 601284, 603667, 603712, 603594, 601872, 603425, 601843, 603263, 603208, 601411, 603201, 603189, 601463, 603164, 603152, 603087, 602874, 601492, 602893, 602057, 602859, 602746, 603879, 603510, 602458, 603380, 601581, 603765, 603283, 601599, 601733, 601852, 602316, 601615, 601617, 602184, 602894, 603005, 603030, 603861, 602835, 602136, 600153, 600074, 600046, 600023, 601625, 516006, 600018, 600016, 516002, 601590, 313475, 313470, 600244,
   600528, 601611, 600282, 600327, 601568, 600368, 601730, 601535, 601745, 601929, 300169, 300150, 300132, 601533, 600385, 600464, 600424, 600429, 601756, 601488, 516005, 251100, 516004, 600918, 516003, 602192, 516001, 240500, 600465, 602241, 602243, 230200, 601485, 601478, 601416, 602297, 601459, 601839, 602314, 193065, 193001, 191306, 600504, 601020, 191191, 602372, 190181, 600534, 188380, 186854, 186360, 600530, 185250, 182331, 600535, 182305, 601296, 600582, 600732, 600734, 600742, 600782, 176802, 176266, 600769, 601883, 600864, 601901, 176260, 173490, 600910, 601905, 171890, 600916, 601987, 602679, 162651, 161555, 160994, 602714, 602715, 602724, 602736, 300007, 602783, 275630, 602836, 270200, 602871, 159460, 602876, 154540, 153900, 602890, 601153, 602190, 602905, 153634, 153337, 602914,
   152310, 151690, 151625, 602935, 602974, 150325, 602992, 150320, 250790, 603006, 603007, 603008, 150292, 233690, 603046, 150210, 603061, 147940, 603063, 221770, 223100, 603097, 147880, 603118, 147730, 146928, 146630, 142622, 603149, 603150, 603151, 600923, 138981, 138590, 138330, 216950, 603192, 138297, 603202, 601002, 602343, 138230, 136131, 603217, 603220, 134660, 131390, 131235, 603242, 603243, 130130, 602345, 126455, 601123, 126064, 125240, 602359, 603312, 602380, 603318, 123890, 123836, 603356, 603361, 603366, 123830, 179610, 188060, 123620, 120980, 186355, 118510, 114835, 114217, 113810, 603499, 182310, 111740, 109610, 603548, 603564, 108740, 603598, 603613, 107273, 603626, 602518, 179410, 603647, 602515, 603652, 106195, 602573, 178990, 105210, 104615, 167055, 603717, 104614, 603728, 104210, 603749, 603750, 103850, 602608, 603787, 603788, 603796, 173445, 103220, 102910, 102681, 102670, 102642, 603833, 173391, 102576, 102575, 171833, 102573, 101800, 603875, 601108
3) Channel
   272 entries found, searching for "channel"
   176266, 600724, 170500, 182390, 123825, 114208, 114205, 601784, 114206, 600937, 114204, 603415, 600053, 114209, 114207, 600760, 118425, 601011, 192500, 176261, 600761, 176260, 600359, 600228, 600877, 602235, 300008, 182389, 182391, 601328, 601534, 600504, 602323, 601958, 602780, 602781, 601327, 601012, 600734, 603208, 182392, 603220, 603219, 603888, 600054, 602232, 601745, 603537, 602604, 603796, 302910, 602866, 601013, 602905, 602906, 600163, 152427, 180901, 600702, 600308, 602754, 107776, 602024, 314555, 601949, 600235, 602023, 176263, 600681, 176265, 193245, 603305, 176258, 602983, 601219, 601141, 176267, 602343, 602726, 138253, 176262, 600003, 600397, 602872, 138249, 600843, 600935, 600580, 600845, 602158, 602106, 176264, 300110, 176257, 602717, 603493, 176268, 600932, 602727, 138254,
   603652, 300138, 602420, 600570, 600150, 603583, 602345, 603749, 601142, 176256, 600846, 138252, 602982, 603787, 602836, 603788, 602566, 603651, 602421, 100690, 107777, 100725, 100710, 600509, 603061, 154275, 304040, 154276, 180902, 121014, 602368, 139311, 601383, 108745, 601313, 601042, 600131, 186360, 600109, 600229, 600170, 603319, 601485, 118503, 180903, 602076, 124030, 601059, 601212, 601218, 147450, 600855, 600919, 601154, 601157, 171060, 600968, 182139, 131230, 121015, 600421, 113730, 249210, 310500, 600637, 125950, 118800, 156490, 602974, 104610, 121011, 602522, 118504, 300041, 160900, 601382, 602103, 600465, 602014, 600442, 601109, 602481, 277900, 254210, 138247, 164920, 170280, 171050, 128100, 173910, 600884, 123885, 602887, 600232, 180297, 137192, 600304, 138251, 603053, 300103, 603152, 603199, 118511, 118508, 138079, 600983, 182307, 603324, 305990, 603418, 114080, 232200, 600046, 600040, 602403, 603750, 603785, 104210, 600019, 600300, 182860, 603852, 603853, 603855, 516060
4) Extracellular matrix
   167 entries found, searching for "extracellular matrix"
   603479, 602201, 601418, 601548, 154870, 115437, 602285, 602262, 602261, 134797, 600754, 120361, 116935, 602263, 603320, 601807, 603321, 185250, 185261, 253700, 128239, 120324, 193300, 276901, 308700, 600514, 600261, 602109, 120140, 120150, 147557, 193400, 600536, 188826, 120180, 118661, 120320, 152200, 135821, 112260, 230740, 602090, 155760, 192975, 190182, 602108, 601463, 186745, 600900, 600985, 600758, 602369, 179590, 601211, 600065, 602178, 600262, 182888, 182889, 151510, 182120, 150325, 190181, 150370, 186355, 193065, 165070, 154705, 147559, 146650, 146640, 153619, 175100, 187380, 231050, 188060, 135820, 156790, 130660, 301870, 128240, 600076, 600119, 193210, 600215, 600245, 121010, 150240, 600309, 600491, 222600, 120328, 600564, 600596, 600616, 600700, 600742, 120325, 138297, 600930,
   156225, 601028, 601050, 601105, 253800, 601284, 601313, 120280, 310200, 601492, 120250, 601587, 601636, 601652, 601692, 601728, 120220, 601915, 602048, 155120, 310300, 120210, 120165, 120120, 118940, 116930, 602264, 116806, 602366, 120470, 602415, 602428, 602453, 602505, 602574, 603005, 603196, 603221, 603319, 107269, 216550, 103320, 603489, 603551, 603767, 603799, 603842
5) Receptor (including membrane proteins, and also including transcription factors, since nuclear proteins were not excluded in the search)
   1606 entries found, searching for "receptor"
   600408, 176943, 107770, 601531, 143890, 313700, 162643, 202200, 147670, 191306, 182131, 192321, 138249, 190120, 600264, 162321, 603613, 603614, 602164, 138251, 182134, 138040, 152790, 602393, 133430, 601769, 304800, 147280, 168468, 147730, 155555, 191190, 109760, 122561, 136537, 602034, 147138, 187930, 312861, 107450, 138981, 182135, 603612, 191191, 600933, 601267, 600168, 120650, 182133, 601919, 108962, 162323, 102576, 600022, 603366, 600052, 136430, 601835, 600825, 600979, 102981, 192974, 602779, 601828, 603499, 601972, 191315, 602601, 603611, 602943, 601300, 191316, 108960, 601299, 147810, 602909, 601834, 108961, 602392, 602910, 603372, 106165, 601199, 180901, 131244, 107470, 136435, 146740, 164920, 139191, 138971, 109690, 100690, 138850, 118504, 147781, 146928, 601373, 126449,
   104225, 100725, 164342, 104220, 118503, 136538, 137241, 601663, 601805, 306250, 600377, 600337, 600509, 600983, 126110, 118507, 602548, 162010, 601268, 147671, 601284, 113503, 109190, 603826, 154540, 147569, 600956, 138245, 139139, 155541, 137192, 118445, 104250, 300119, 118511, 601159, 100710, 305660, 104210, 114610, 138253, 146929, 602778, 135630, 147140, 146790, 147045, 601998, 158378, 131243, 600041, 118508, 137142, 113995, 138247, 182137, 147811, 182132, 138248, 600414, 600563, 137160, 138032, 601496, 138252, 138254, 602600, 273800, 146760, 179590, 180903, 109635, 300038, 138244, 182139, 600849, 600644, 601296, 602282, 179611, 143090, 100720, 602076, 600380, 170998, 600845, 118505, 118510, 190160, 104260, 137164, 137140, 600456, 147880, 137190, 601256, 601956, 100730, 601194, 602717,
   173880, 600042, 180902, 300093, 602423, 602469, 603028, 601437, 146933, 179610, 173510, 600843, 305915, 137143, 603361, 603031, 137141, 136539, 162332, 118509, 155540, 600233, 601109, 601743, 600409, 147139, 131235, 603506, 603455, 151445, 602408, 601681, 603540, 602454, 602490, 600214, 600798, 600232, 602729, 600730, 601122, 120577, 160994, 603248, 602621, 601305, 176946, 118502, 176888, 602167, 138246, 158050, 601470, 603453, 603065, 137163, 137166, 600846, 118495, 138243, 192975, 138060, 602757, 191311, 603652, 120340, 600315, 136425, 118493, 600731, 601502, 601503, 601751, 603651, 602345, 118496, 601598, 602343, 118494, 186880, 134934, 601007, 600946, 186930, 186970, 133171, 308380, 120620, 126452, 190010, 126450, 186810, 180220, 173410, 188070, 131550, 109691, 147679, 180240, 173490,
   300034, 264080, 114131, 146661, 147370, 147545, 136352, 190182, 186780, 126453, 110700, 147851, 308385, 126451, 128239, 600018, 601487, 601040, 305990, 601604, 190181, 188545, 162641, 600445, 231200, 136350, 601586, 164770, 600073, 602376, 138491, 118946, 176761, 176806, 601770, 180245, 164870, 602691, 600939, 109610, 192977, 159530, 602997, 164860, 300007, 146710, 601757, 150370, 118444, 600239, 138033, 601167, 108360, 162642, 173850, 603881, 180246, 193210, 173470, 601469, 134935, 182452, 138492, 135620, 601523, 601790, 600221, 109630, 602451, 147265, 602626, 602001, 602069, 601642, 600848, 102776, 180190, 182098, 176886, 602250, 182454, 600222, 600821, 180247, 602730, 134637, 167055, 165196, 165195, 173391, 601426, 600527, 600167, 153618, 601330, 601970, 600785, 600004, 601043, 109135,
   109636, 601613, 600804, 601529, 102581, 600936, 126150, 182451, 300107, 190151, 602746, 165070, 170650, 600421, 600997, 602648, 603243, 300035, 305670, 182455, 162651, 109535, 601320, 120920, 603411, 603691, 601535, 102775, 601166, 180381, 176804, 602188, 600051, 601993, 601773, 603755, 600241, 601050, 603659, 600253, 300130, 137026, 602368, 154570, 176945, 602005, 120980, 602583, 102777, 600524, 602848, 173393, 601711, 104221, 176884, 600441, 108361, 600665, 600446, 600799, 600742, 104219, 603819, 600867, 602351, 601978, 600578, 602566, 600551, 601898, 151443, 602642, 300182, 191164, 602356, 603071, 602190, 601522, 182889, 142703, 601576, 100735, 176882, 602969, 182453, 601895, 602886, 602927, 602697, 601070, 120830, 601108, 176891, 176802, 600024, 600552, 601908, 176981, 603510, 151460,
   602237, 602545, 162341, 603748, 161565, 600894, 186711, 137161, 601839, 601592, 603507, 602885, 430000, 104222, 601116, 602643, 601896, 601404, 601381, 600180, 600870, 123889, 600206, 600242, 602992, 601380, 602950, 602174, 603159, 602042, 602297, 602836, 602355, 602304, 603822, 147267, 603030, 603821, 602043, 603820, 603317, 601965, 601524, 601974, 603823, 602728, 601741, 137570, 603500, 602592, 153622, 601204, 186990, 601115, 600283, 182180, 600600, 600066, 600282, 176944, 600926, 600895, 300188, 602657, 602756, 600925, 182888, 603808, 603029, 602702, 602464, 603751, 425000, 601910, 151020, 600553, 603195, 602646, 601909, 603692, 136950, 600543, 164320, 300086, 602933, 602993, 137162, 603281, 153243, 161555, 600011, 600144, 600869, 600493, 602346, 600342, 602336, 113955, 602893, 601577,
   603749, 602892, 603810, 602853, 600579, 601044, 603809, 186690, 600752, 603304, 600240, 603232, 600868, 602894, 602337, 603143, 109545, 602891, 602890, 602716, 603142, 602510, 603144, 603141, 300090, 602550, 603145, 107741, 107730, 164761, 208900, 107269, 114208, 235200, 158120, 147440, 300200, 161561, 186830, 151520, 131240, 139250, 601667, 153240, 218030, 147680, 186740, 600044, 107940, 153390, 102582, 600978, 601218, 142409, 601156, 601884, 601789, 125950, 171833, 602221, 139320, 147760, 190000, 184757, 601512, 165180, 166490, 168470, 601366, 176000, 191160, 102578, 103320, 112260, 192240, 109091, 603233, 134370, 254210, 173610, 125480, 600957, 118945, 120470, 136351, 600725, 176797, 602887, 173511, 123910, 601459, 252500, 104615, 601528, 600345, 601890, 151390, 602170, 186790, 186960,
   600542, 186973, 600423, 600058, 176730, 186945, 104760, 601309, 601500, 300091, 113705, 214500, 602731, 600726, 176885, 164875, 139330, 104640, 147840, 600065, 131242, 164160, 600565, 601211, 151530, 603599, 180860, 190040, 147720, 222100, 223900, 600555, 600994, 186770, 120420, 602004, 601099, 603776, 601212, 132890, 600007, 158105, 603886, 603884, 176830, 162200, 602458, 601603, 107910, 193400, 133100, 160900, 114855, 601498, 601511, 116831, 131390, 107773, 137800, 155750, 147795, 264600, 114207, 244400, 187040, 126455, 164040, 147683, 263200, 188230, 184756, 147574, 179615, 176960, 176947, 147561, 131320, 107265, 147470, 264700, 173335, 124010, 147050, 132810, 230350, 276000, 114130, 155730, 134638, 254150, 122560, 186940, 208150, 188040, 188400, 100600, 601564, 603875, 603849, 601497,
   603254, 603180, 602952, 602775, 602584, 602570, 601045, 600632, 602559, 601883, 600618, 601848, 601766, 601753, 600554, 600937, 600694, 311360, 302910, 600173, 300139, 300065, 274000, 305100, 300116, 300022, 163800, 187410, 187020, 603818, 105850, 107941, 307800, 601897, 212895, 603598, 603584, 603583, 603454, 603412, 601872, 114204, 266300, 114841, 116897, 152690, 603261, 603130, 603109, 603061, 603055, 603043, 601771, 154275, 600910, 187011, 188060, 600039, 600464, 186355, 121360, 122559, 123000, 253800, 602901, 125255, 131100, 600837, 602772, 600835, 131530, 602695, 137580, 602623, 139130, 249210, 142408, 602457, 600179, 142445, 154276, 245480, 142800, 600998, 602354, 601698, 602272, 601053, 602265, 231550, 229300, 147780, 601985, 147796, 226200, 600276, 147830, 150200, 601937, 189906,
   601922, 600341, 100650, 601662, 189965, 159440, 190020, 601054, 600764, 600410, 153440, 205400, 601055, 203800, 600576, 600749, 190090, 601767, 600475, 190080, 154550, 184745, 601721, 601712, 600594, 600739, 600738, 180069, 601020, 601679, 162662, 601266, 601676, 601203, 172400, 601463, 600685, 601599, 164009, 600611, 601570, 600626, 600201, 600514, 191100, 195002, 600567, 600633, 191030, 190700, 600014, 303700, 308240, 308230, 191170, 269700, 191092, 192130, 600319, 238600, 195000, 600125, 600577, 261680, 600566, 266100, 309900, 266600, 306900, 600536, 194070, 201910, 188860, 188595, 257220, 600038, 202110, 261000, 600363, 600850, 306100, 602229, 603883, 102645, 186921, 186910, 603785, 186852, 104311, 107266, 107270, 603746, 603732, 109270, 602060, 602041, 148180, 151385, 151625, 123830,
   182530, 123842, 124030, 134660, 153370, 134850, 601023, 182280, 134797, 182125, 134390, 601047, 135820, 601804, 153420, 601801, 139312, 139392, 181031, 601622, 180700, 147570, 603157, 162150, 601589, 121013, 151440, 602919, 151510, 123840, 123890, 131210, 179730, 602663, 179020, 602630, 601231, 601252, 137295, 138190, 138550, 277700, 601295, 139311, 153455, 176311, 176310, 601313, 133170, 141800, 142995, 602358, 143100, 145505, 300079, 168461, 145981, 158070, 106700, 107400, 107720, 601485, 103950, 108330, 165360, 300300, 156490, 603492, 602018, 118485, 164951, 164730, 164720, 147620, 603406, 164343, 119530, 161560, 142910, 602414, 601933, 120290, 164013, 164012, 600667, 601534, 601513, 601520, 164015, 164340, 173515, 164975, 302020, 160782, 601501, 165230, 302060, 165120, 601492, 601483, 167040, 167415, 600714, 192968, 600696, 601458, 601447, 173325, 168820, 176820, 601413, 190198, 600489, 173445, 601754, 600481, 300155, 600453, 154230, 173430, 154030, 173360, 181590, 176876, 176970, 178600, 164005, 182160, 274500, 179616, 275120, 151990, 300147, 601863, 211750, 275355, 151675, 190170, 179838, 600750, 180200, 180231, 151430, 600753, 601582, 150240, 601584, 601153, 224500, 601154, 601969, 180440, 600596, 600281, 600262, 227400, 601997, 147684, 147678, 600761, 147581, 602014, 602024, 147573, 601150, 159555, 147559, 600945, 191163, 602065, 147558, 601641, 147450, 602096, 602113, 600583, 602154, 600556, 159350, 147245, 186855, 192020, 191510, 146631, 146630, 180950, 601693, 146255, 602260, 238300, 600231, 602293, 194540, 602299, 145680, 602311, 145260, 600230, 602350, 239100,
   600508, 143055, 601723, 143010, 155960, 602377, 602382, 275350, 600220, 602396, 304050, 600424, 602421, 142461, 602430, 602431, 142460, 246530, 273300, 249100, 602461, 602465, 141850, 601788, 602498, 140210, 181460, 600422, 300127, 600164, 600765, 602582, 138800, 300101, 138720, 138420, 602609, 600157, 138079, 138030, 600156, 601017, 137240, 602671, 602680, 602682, 136530, 182090, 600807, 601826, 602724, 153245, 182331, 210250, 600151, 602738, 131340, 131241, 300008, 131195, 600119, 602809, 602839, 602840, 128240, 126850, 126065, 252650, 253200, 188380, 600810, 600396, 123841, 182810, 123835, 600053, 217030, 602921, 602931, 602932, 601900, 600050, 185430, 185520, 121800, 254770, 259700, 222300, 120940, 120700, 120436, 120360, 603033, 600295, 603035, 262600, 603046, 600009, 263400, 120150,
   263520, 603126, 590085, 185605, 118960, 118940, 118455, 603149, 603177, 300019, 603181, 118425, 603207, 426000, 116955, 116940, 116930, 300088, 603264, 603272, 603274, 116899, 603302, 313650, 266265, 603331, 114835, 603356, 114830, 312760, 603378, 602061, 310400, 603414, 603437, 113730, 310200, 113710, 147557, 113000, 112205, 111400, 111200, 110750, 603518, 603526, 602136, 603576, 309845, 603594, 308310, 108728, 108355, 600250, 307200, 603628, 107777, 602165, 603653, 600899, 107273, 107271, 603695, 603705, 603717, 186590, 186720, 186760, 186820, 187700, 600916, 147183, 105590, 104610, 146770, 306400, 602227, 103390, 103280, 103070, 102670, 301000, 603851, 600874, 600871, 102200, 158380, 603889
6) Glycoprotein
   438 entries found, searching for "glycoprotein"
   231200, 273800, 138700, 138600, 173510, 173515, 192974, 109770, 138610, 603578, 195002, 182889, 601275, 602977, 300051, 173470, 171050, 138680, 142640, 194460, 176390, 138720, 600119, 185430, 138670, 180297, 171060, 600073, 173335, 128239, 118850, 602616, 110700, 164345, 159465, 159460, 191845, 173511, 600564, 159440, 256550, 153330, 168840, 600738, 151460, 138470, 190197, 603586, 601325, 601193, 162820, 158120, 155970, 190920, 603201, 603594, 601411, 155735, 186730, 601028, 158030, 176391, 115501, 137290, 601103, 600315, 176870, 601525, 601418, 137061, 137207, 137060, 176397, 176393, 176394, 182888, 160995, 176398, 600718, 176396, 176392, 176399, 176401, 176395, 125645, 601774, 104175, 195000, 601785, 193400, 600065, 111200, 253700, 152200, 154550, 310200, 188230, 256540, 120980, 130660,
   107269, 158070, 156790, 131550, 135630, 138297, 155760, 231050, 603381, 143890, 176801, 118860, 103600, 193210, 186820, 114890, 188060, 118910, 107271, 153340, 313470, 600074, 603062, 109270, 602724, 603356, 120324, 192340, 266265, 110900, 306400, 601327, 118457, 151250, 136836, 253000, 104640, 190010, 190000, 135620, 135600, 601852, 134797, 134370, 111250, 156225, 600596, 187430, 164870, 151510, 186720, 601962, 600900, 151530, 180990, 180290, 182120, 603759, 179605, 158381, 158373, 158343, 155740, 153420, 151675, 142800, 176300, 153240, 140100, 134820, 134660, 134390, 125240, 120520, 112205, 111740, 106100, 104225, 103000, 139090, 176860, 102720, 176895, 180621, 602421, 602257, 252600, 252650, 180620, 227600, 228960, 208900, 182145, 208400, 602643, 602593, 602461, 602171, 182900, 601269,
   600270, 600033, 602894, 247100, 186910, 600239, 230500, 217800, 600867, 253220, 601581, 305600, 162060, 158378, 188450, 186880, 186990, 188040, 154582, 164022, 188540, 153634, 189965, 193190, 165095, 165220, 158375, 152790, 158374, 151627, 182205, 155750, 191316, 151523, 151020, 165330, 166490, 192225, 224050, 224100, 185490, 192240, 173445, 167770, 185050, 153619, 230800, 147740, 147730, 147670, 153618, 236200, 193300, 249000, 176385, 153440, 147557, 152310, 253270, 184900, 182331, 146760, 146661, 272300, 272800, 142930, 169615, 171640, 182520, 182310, 300027, 147780, 305370, 151525, 142858, 142840, 308230, 308385, 309060, 142445, 142290, 216950, 139130, 600026, 600044, 600046, 138971, 138960, 138140, 600133, 600182, 600215, 173350, 173360, 135820, 600403, 600410, 600491, 134934, 147563,
   600535, 133700, 600587, 133170, 131390, 600616, 600713, 131195, 600722, 130160, 600742, 600751, 600764, 600769, 600798, 180540, 600978, 129010, 601040, 601050, 125505, 125490, 124010, 601194, 601211, 601253, 147380, 120930, 601313, 120830, 120620, 601365, 601368, 120220, 601456, 601484, 117700, 147045, 601652, 274500, 116930, 116880, 601788, 116805, 114250, 113810, 601880, 601914, 601932, 111700, 602046, 602053, 602108, 602243, 176820, 178630, 178640, 111680, 300017, 109535, 142860, 602692, 109530, 602746, 308840, 107740, 107400, 603104, 603125, 603126, 603130, 107273, 603197, 107266, 603241, 603243, 104614, 312080, 104610, 603492, 103390, 102670, 102480, 100735, 603665, 603756, 100730, 603777
7) Protein kinase (a member of signal transduction)
   729 entries found, searching for "protein kinase"
   600289, 160900, 601032, 176948, 600899, 176947, 176894, 601939, 602980, 601314, 601955, 601335, 600447, 602130, 603606, 601158, 602006, 602549, 602678, 176872, 603014, 602520, 186945, 601254, 602315, 601263, 177061, 176873, 600497, 603607, 602449, 602740, 602898, 602742, 300083, 602899, 114080, 603412, 601184, 602739, 176910, 600050, 176949, 602741, 602743, 600341, 601951, 601212, 600038, 156490, 600855, 600758, 603455, 156491, 603424, 300075, 180381, 151520, 600168, 600085, 601890, 602896, 602123, 600221, 602904, 602687, 176945, 602897, 600222, 602098, 601685, 176944, 109635, 601795, 601679, 603722, 602521, 601436, 191164, 602004, 176942, 601684, 300101, 601299, 601434, 602615, 601435, 176960, 153390, 603166, 179090, 300300, 176871, 602216, 176912, 176893, 603453, 300185, 177060, 176943,
   176981, 603441, 176970, 116899, 603442, 311550, 603495, 169190, 600448, 602399, 176946, 176977, 188830, 602350, 601591, 601782, 137026, 400008, 300094, 600058, 600831, 176892, 601639, 602919, 176911, 176975, 300172, 603072, 600583, 177015, 176982, 176980, 603248, 176941, 602757, 600870, 603440, 148750, 601959, 600869, 602448, 600408, 306000, 266200, 602539, 602425, 600160, 310200, 208900, 600997, 186973, 305360, 179611, 307030, 603262, 601050, 602758, 147795, 311800, 600664, 600441, 603251, 600527, 313650, 123831, 191315, 602958, 123832, 602989, 602614, 123828, 136350, 601284, 602940, 601329, 600173, 171834, 124095, 603464, 603584, 116953, 300035, 600600, 109135, 601232, 601988, 603850, 603384, 603184, 602990, 125855, 601047, 603460, 603258, 602748, 603723, 164870, 603368, 139310, 601818,
   602913, 603005, 602221, 173570, 147670, 602610, 600524, 113508, 251170, 142370, 142408, 191306, 147796, 118423, 600286, 601074, 600765, 600922, 179610, 602747, 601953, 601132, 600136, 601288, 601289, 602297, 191316, 601535, 602925, 125450, 602839, 602168, 602461, 105590, 114085, 600714, 154235, 603261, 109270, 191305, 602625, 602366, 602749, 600927, 300134, 603496, 136352, 176790, 600073, 601523, 115441, 191170, 114210, 116900, 188250, 102576, 603764, 603497, 603304, 601934, 602255, 600004, 600526, 601589, 600856, 601269, 601465, 601207, 601935, 600863, 602394, 601983, 602048, 300200, 179050, 164761, 189980, 602626, 137025, 602694, 602731, 125305, 123810, 300139, 601792, 275350, 171833, 193525, 601540, 162200, 601826, 116898, 601108, 601528, 600954, 602609, 602838, 603167, 601522, 600505,
   600431, 600267, 188555, 600140, 308240, 600137, 600011, 151410, 601530, 134934, 116952, 300142, 191311, 601603, 603889, 601728, 165160, 165070, 136351, 601836, 601839, 133090, 601595, 602745, 602516, 154950, 603601, 602052, 154045, 603583, 600695, 602933, 602756, 601014, 602474, 602887, 172270, 601366, 180220, 602189, 114105, 600963, 603369, 601296, 603289, 602265, 602337, 600066, 600456, 602387, 600917, 139270, 603271, 188345, 601231, 115440, 115442, 600778, 164920, 175100, 310300, 601767, 603168, 603140, 603113, 116940, 162060, 603048, 603275, 601146, 600434, 602336, 602310, 602308, 147522, 603311, 227645, 114110, 601114, 300022, 164785, 167414, 123280, 600007, 600560, 229300, 151430, 176740, 602426, 109636, 602775, 108355, 601999, 601524, 190151, 202500, 601592, 600723, 102750, 600753,
   603618, 603814, 147521, 602514, 603015, 602525, 602524, 172471, 602647, 602590, 600864, 172470, 602538, 602527, 602526, 143890, 164730, 232300, 131550, 116951, 601713, 154550, 603434, 138160, 601702, 126335, 272800, 601497, 602038, 107940, 191190, 273800, 193300, 120150, 603068, 603259, 102582, 601099, 253700, 308000, 602338, 602689, 185605, 164040, 600119, 152690, 601496, 602188, 123900, 602860, 164960, 600618, 601045, 300189, 164860, 602505, 230800, 141850, 147545, 110300, 600051, 138600, 233700, 109700, 238600, 109690, 253800, 311770, 156225, 603719, 191030, 309000, 601893, 190030, 189972, 601247, 190120, 600179, 258501, 190040, 300121, 180380, 314850, 600438, 306400, 179715, 190450, 600636, 180901, 173335, 601290, 154500, 131195, 115460, 603078, 603775, 603760, 158900, 602354, 102680,
   122560, 602382, 103320, 603597, 603510, 232600, 133170, 603353, 603345, 603318, 603309, 230400, 139250, 603296, 603272, 114240, 147557, 232400, 139259, 602122, 230200, 164014, 248600, 601703, 194364, 150000, 602102, 601683, 158070, 602170, 602007, 601306, 248610, 601387, 164770, 232800, 164760, 601902, 601253, 173470, 602930, 603691, 165360, 123835, 603544, 147265, 107777, 602377, 600605, 602869, 601121, 602388, 301300, 590060, 601059, 168461, 142600, 314555, 142410, 170290, 602498, 190090, 311030, 142409, 190181, 600966, 308100, 600936, 171900, 190182, 172405, 102600, 603754, 600281, 172430, 138249, 300039, 603154, 602642, 300041, 602659, 192240, 603473, 136537, 603134, 182280, 603084, 125660, 189990, 133430, 603755, 603302, 600737, 300144, 305400, 188545, 600652, 189940, 600633, 603009,
   181031, 182160, 148500, 602384, 600650, 602190, 171150, 173490, 164765, 147780, 600910, 601272, 147880, 125255, 120090, 173390, 602668, 600483, 602995, 602996, 180860, 142445, 116949, 602681, 603027, 182125, 600327, 600799, 164757, 230450, 602730, 600782, 164880, 232700, 116935, 602303, 603203, 164710, 600244, 600206, 263200, 300034, 601487, 600980, 600956, 601985, 600884, 601956, 191041, 601568, 601586, 155960, 602288, 176761, 113900, 600581, 516050, 190070, 164940, 600544, 107941, 312861, 602373, 123836, 106165, 103850, 274270, 601922, 602355, 147420, 311010, 163800, 600536, 602018, 307200, 603619, 603652, 193001, 193245, 603729, 603752, 118888, 603796, 305600, 603852, 603853, 603886
8) Calmodulin kinase (a member of signal transduction)
   35 entries found, searching for "calmodulin binding"
   300172, 114180, 302020, 139312, 602584, 602293, 600310, 603379, 114106, 602350, 114105, 114213, 138249, 153430, 177061, 182520, 102680, 600489, 601478, 306000, 600490, 600922, 601302, 601568, 168890, 164795, 163730, 602698, 602699, 602987, 102681, 603384
9) Transcription factor
   717 entries found, searching for "transcription factor"
   305371, 189963, 164177, 600297, 600298, 163260, 189907, 173110, 600733, 189903, 189972, 600438, 600281, 189962, 157670, 602272, 600609, 189968, 189971, 189889, 107580, 189908, 601748, 601542, 601632, 600610, 189969, 600480, 601760, 176310, 603022, 600519, 601750, 123803, 600520, 600494, 164176, 602460, 603246, 113725, 107773, 189904, 602228, 147141, 132890, 189964, 164175, 142410, 313650, 164011, 164014, 164005, 601714, 600492, 164012, 601878, 600673, 600635, 191523, 602191, 600490, 602149, 189967, 140580, 164343, 603256, 600727, 600729, 600728, 140581, 600571, 601511, 102582, 189906, 600660, 124097, 314310, 600860, 600662, 184756, 600777, 600013, 600172, 600489, 601425, 600786, 600502, 602406, 600743, 189965, 603257, 602751, 602542, 603148, 603107, 603789, 602318, 156845, 123811, 600425,
   600540, 600695, 601622, 147574, 601601, 602407, 602150, 601538, 600663, 603306, 165170, 187040, 602444, 189902, 189973, 600659, 600661, 601010, 600788, 602617, 601602, 602053, 601742, 300039, 602438, 602976, 600744, 602543, 602479, 600481, 600473, 603739, 600426, 603738, 189901, 603677, 600427, 603255, 600607, 600379, 189909, 601679, 600787, 602160, 601043, 601397, 601044, 600366, 300025, 602575, 602669, 601804, 601801, 176312, 176311, 600746, 602480, 602944, 600967, 600912, 306700, 306900, 193400, 601206, 480000, 191160, 601861, 164008, 600475, 600773, 600772, 600774, 142409, 156490, 600589, 601490, 151385, 600599, 184757, 602955, 234000, 603433, 603349, 603198, 602294, 600390, 603628, 147620, 600211, 601787, 601863, 147470, 603795, 603734, 152760, 104155, 128990, 601729, 600197, 147370,
   173490, 603423, 600822, 188595, 603243, 600573, 601689, 142765, 603181, 600879, 603731, 600288, 602295, 121360, 164874, 300019, 162095, 602355, 603258, 126090, 159540, 300070, 600555, 600664, 601874, 153245, 191191, 601126, 601512, 146733, 131550, 142385, 601796, 603406, 602959, 601734, 601732, 139191, 139139, 600633, 138971, 600006, 603170, 601488, 147576, 147680, 601498, 602630, 602643, 603364, 600914, 154040, 602746, 128992, 143089, 160900, 600140, 134934, 133510, 176860, 190180, 601150, 601175, 170993, 601361, 122560, 602778, 308230, 602903, 309550, 601788, 602946, 159970, 124092, 180200, 173410, 602356, 603015, 600779, 603111, 187930, 602614, 600951, 603200, 602369, 164770, 147569, 603300, 603301, 159980, 134638, 603431, 147730, 603366, 603348, 600556, 602136, 164160, 310200, 152390,
   601241, 116897, 137295, 600576, 194070, 601487, 600698, 164810, 601769, 141900, 602225, 275350, 131100, 179755, 600075, 162200, 165160, 116806, 600899, 123810, 133450, 216400, 278700, 190080, 164730, 191170, 193300, 600618, 600999, 601090, 106150, 601843, 133530, 110700, 602550, 138040, 133430, 300133, 163731, 602302, 126337, 309548, 180245, 126110, 602291, 109565, 107400, 314670, 601444, 143100, 104760, 106180, 601953, 600584, 125852, 602419, 600401, 142200, 107680, 167414, 600020, 188400, 208900, 175100, 602700, 601828, 139320, 602777, 600185, 602681, 603023, 314997, 602848, 600284, 102578, 114290, 165095, 137070, 602991, 602421, 600005, 602996, 314995, 152200, 151900, 112260, 129010, 600892, 273800, 176760, 602341, 490000, 136533, 400003, 601007, 602229, 603620, 602218, 602116, 602020,
   142000, 601955, 126340, 120150, 193067, 182452, 142461, 194558, 180660, 600756, 160745, 107741, 106210, 157640, 186770, 146738, 603759, 213700, 147880, 152391, 277900, 151626, 107730, 600711, 600246, 107470, 600237, 223100, 107748, 600065, 600349, 426000, 194500, 154030, 227650, 600247, 314980, 109560, 305370, 600800, 301000, 277700, 600838, 312173, 600439, 600440, 191315, 601595, 190450, 190070, 190020, 162360, 131320, 133540, 600993, 601993, 159530, 601902, 602868, 181590, 601724, 602260, 601093, 187270, 164761, 602102, 603245, 136950, 106100, 601182, 167410, 601897, 602896, 170100, 602506, 104150, 176730, 601600, 187011, 102600, 180380, 162080, 603450, 142967, 602301, 126375, 603372, 603355, 164720, 603250, 167409, 167415, 602897, 601565, 185250, 182138, 601851, 600749, 601575, 194548,
   154500, 601365, 194541, 601621, 601623, 601531, 600790, 194355, 123830, 123812, 154540, 601415, 143055, 601386, 194550, 186930, 131290, 601320, 601620, 601754, 601313, 184430, 182900, 182500, 600725, 147870, 154365, 116953, 601297, 601296, 601265, 600796, 120436, 601644, 601930, 601643, 230200, 601645, 601972, 600861, 602009, 601172, 601158, 601646, 180630, 600821, 118440, 601656, 601647, 150200, 601125, 601671, 141850, 116899, 600697, 109270, 202110, 150570, 601108, 191339, 601063, 109691, 180240, 203100, 151430, 179710, 111000, 176797, 238600, 104311, 240300, 125255, 600423, 158070, 602439, 600324, 112261, 243305, 602474, 174762, 600613, 602539, 138890, 138720, 114550, 173865, 602582, 602584, 173510, 600250, 602627, 173325, 602635, 246530, 172425, 600193, 602691, 600188, 170998, 152790,
   168468, 256540, 225250, 600848, 143400, 168461, 262600, 168360, 601912, 602951, 600017, 230000, 266600, 602981, 272800, 109150, 102200, 603025, 603026, 603109, 167050, 603127, 603128, 165240, 230400, 313700, 164975, 164875, 602017, 115500, 235800, 164873, 602110, 164785, 164772, 312865, 603296, 600542, 164740, 602125, 309801, 602148, 300007, 306955, 603368, 116940, 602181, 603416, 126650, 163920, 300024, 603437, 602209, 603576, 603607, 305435, 600944, 180410, 303630, 159557, 301870, 132810, 100790, 603849, 603862, 603881,

There are several methods for analyzing the expression levels of genes associated with diseases. Differences in gene expression levels between diseased and normal tissues are studied by the analytical methods, for example, Northern hybridization and differential display. Other examples include a method with high-density cDNA filter, a method with DNA microarray and methods with PCR amplification (Experimental Medicine, Vol.17, No. 8, 980-1056 (1999); Cell Engineering (additional volume) DNA Microarray and Advanced PCR Methods, Muramatsu & Naba (eds.), Shujunsya). The levels of gene expression between diseased tissues and normal tissues can be studied by any of these analytical methods. When explicit difference in expression level is observed for a gene, it can be concluded that the gene is closely associated with a disease or disorder. Instead of diseased tissues, cultured cells can be used for the assessment. Similarly, when gene expression is explicitly different between normal cells and cells reproducing disease-associated specific features, it can be concluded that the gene is closely associated with a disease or disorder. When the expression levels of genes are evidently varied during major cellular events (such as differentiation and apoptosis), the genes are involved in the cellular events and accordingly are candidates for disease- and/or disorder-associated genes. Further, genes exhibiting tissue-specific expression are genes playing important parts in the tissue functions and, therefore, can be candidates for genes associated with diseases and/or disorders affecting the tissues.

For example, non-enzymic protein glycation reaction is believed to be a cause for a variety of chronic diabetic complications. Accordingly, in endothelial cells, genes, of which expression levels are elevated or decreased in a glycated protein-dependent manner, are associated with diabetic complications caused by glycated proteins (Diabetes 1996, 45 (Suppl. 3), S67-S72; Diabetes 1997, 46 (Suppl. 2), S19-S25). The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center, http://www.rheuma-net.or.jp/). Recent studies have also revealed that tissue necrosis factor (TNF)-α participates in the onset (Current opinion in immunology 1999, 11, 657-662). When the expression of a gene exhibits responsiveness to the action of TNF on synovial cells, the gene is considered to be involved in rheumatoid arthritis. Many genes acting at the downstream of TNF-α and IL-1β among inflammation-associated cytokines have been previously identified. The respective stimulations are transduced through independent pathways of signaling cascade. There exists another signaling cascade for both stimulations, wherein NF- κB is a common transducing molecule shared by the two stimulations (J. Leukoc. Biol., 1994, 56(5): 542-547). It has also been revealed that many inflammation-associated genes, including IL-2, IL-6 and G-CSF, are varied in the expression levels in response to the signal through the common pathway (Trends Genet. 1999, 15(6): 229-235). It is assumed that genes of which expression levels are varied in response to the stimulation of TNF-α or IL-1β also participate in inflammation. Genes associated with neural differentiation can be candidates for causative genes for neurological diseases as well as candidates for genes usable for treating the diseases.

Clones exhibiting differences in the expression levels thereof can be selected by using gene expression analysis. The selection comprises, for example; analyzing cDNA clones by using high-density cDNA filter; and statistically treating the multiple signal values (signal values of radioisotope in the radiolabeled probes or values obtained by measuring fluorescence intensities emitted from the fluorescent labels) for the respective clones by two-sample t-test, where the signal values are determined by multiple experiments of hybridization. The clones of interest are selectable based on the statistically significant differences in the signal distribution at p<0.05. However, selectable clones with significant difference in the expression levels thereof may be changed depending on the partial modification of statistical treatment. For example, the clones may be selected by conducting statistical treatment with two-sample t-test at p<0.01; or genes exhibiting more explicit differences in the expression levels thereof can be selected by performing statistical treatment with a pre-determined cut-off value for the significant signal difference. An alternative method is that the expression levels are simply compared with each other, and then, the clones of interest are selected based on the ratio of the expression levels thereof.

Clones exhibiting differences in the expression levels can also be selected by comparing the expression levels by PCR analysis, for example, by using the method of determining the band intensities representing the amounts of PCR products with ethidium bromide staining; the method of determining the radioisotopic signal values or fluorescence intensities of the PCR products when radio-labeled or fluorescence-labeled primers; or the method of determining the values of radioisotope signals or fluorescence intensities of the probes hybridized to the PCR products when radio-labeled or fluorescence-labeled probes, respectively, are used in the hybridization. If the expression level ratios obtained in multiple PCR experiments are constantly at least 2-fold, such a clone can be judged to exhibit the difference in the expression level. When the ratios are several-fold or not less than 10-fold, the clone can be selected as a gene exhibiting the explicit difference in the expression level.

A survey of genes of which expression levels are varied specifically to the glycated protein in the endothelial cells revealed three genes with elevated expression levels, NT2RP2001538, NT2RP4001001 and Y79AA1000967. These clones are genes associated with diabetes.

A survey of genes of which expression levels are varied in response to TNF α (Tumor Necrosis Factor-alpha) in the primary cell culture of synovial tissue detected the following clones with elevated expression levels in the presence of TNFα :
BNGH41000020, HEMBA1000349, HEMBA1000634, HEMBA1000671, HEMBA1000835, HEMBA1000962, HEMBA1002178, HEMBA1002195, HEMBA1002239, HEMBA1002420, HEMBA1002524, HEMBA1002992, HEMBA1003315, HEMBA1003392, HEMBA1003487, HEMBA1003602, HEMBA1004067, HEMBA1004797, HEMBA1005337, HEMBA1005489, HEMBA1006916, HEMBB1000668, HEMBB1000905, HEMBB1001547, HEMBB1001573, HEMBB1002041, HEMBB1002663, MAMMA1000652, MAMMA1000810, MAMMA1001634, MAMMA1002091, MAMMA1002234, NT2RM2000306, NT2RM4000417, NT2RP1000002, NT2RP1000181, NT2RP1000740, NT2RP2000694, NT2RP2001921, NT2RP2002527, NT2RP2004495, NT2RP2004606, NT2RP2005163, NT2RP2005463, NT2RP2006134, NT2RP3000171, NT2RP3000652, NT2RP3001195, NT2RP3001976, NT2RP3003473, NT2RP3003874, NT2RP3004090, NT2RP3004294, NT2RP3004557, NT2RP3004647, NT2RP4000108, NT2RP4001001, NT2RP4001877, OVARC1000090, OVARC1000105, OVARC1000275, OVARC1000439, OVARC1001607, PLACE1000740, PLACE1000927, PLACE1001016, PLACE1001100, PLACE1001464, PLACE1001500, PLACE1001918, PLACE1002095, PLACE1002547, PLACE1003644, PLACE1004519, PLACE1005031, PLACE1005410, PLACE1005736, PLACE1006219, PLACE1006809, PLACE1008716, PLACE1010081, THYRO1001770, Y79AA1000127, Y79AA1000207, Y79AA1000270, Y79AA1000876, Y79AA1001013, Y79AA1001264, Y79AA1001272, Y79AA1001328, Y79AA1001430, Y79AA1001530, Y79AA1001799

Clones with decreased expression levels in the presence of TNFα are NT2RM4000326, NT2RP1000300, NT2RP2000514, NT2RP2001755, NT2RP2006042, NT2RP3000481, NT2RP3002790. These clones are candidates for rheumatoid arthritis-associated genes.

A survey of genes of which expression levels are varied in response to TNFα (Tumor Necrosis Factor-alpha) or IL-1β (Interleukin-1 beta) in a human T cell strain, Jurkat cell, revealed the following clones with elevated expression levels in the presence of TNF α: MAMMA1000141, MAMMA1000788, MAMMA1001237, MAMMA1002070,NT2RM2000582, NT2RM2002109, NT2RP1000679, NT2RP2003664, NT2RP2004108, NT2RP2005597, NT2RP3001592, NT2RP3002738, NT2RP3004133, NT2RP3004294, NT2RP3004321, NT2RP3004557, PLACE1002547, PLACE1003573, PLACE1004305, PLACE1008744, PLACE1010011, PLACE1010713, PLACE1011181, Y79AA1000776, Y79AA1002129

The survey also revealed a clone of which expression level was decreased in the presence of TNFα. The clone is PLACE1002070. The same survey further revealed the clones of which expression levels were elevated in the presence of IL-1β. The clones are MAMMA1000614, MAMMA1001237, NT2RM2000514 and NT2RP3001159. These clones are genes associated with inflammation.

A survey of genes of which expression levels are varied in response to the stimulation for inducing cell differentiation (stimulation using retinoic acid (RA) or using RA/inhibitor (inhibitor for cell division)) in cultured cells of neural strain, NT2, revealed the following clones with elevated expression levels in the presence of RA:
HEMBA1000121, HEMBA1000275, HEMBA1000300, HEMBA1000634, HEMBA1000671, HEMBA1000875, HEMBA1001184, HEMBA1001390, HEMBA1001886, HEMBA1002163, HEMBA1002227, HEMBA1002420, HEMBA1002421, HEMBA1003072, HEMBA1003120, HEMBA1003294, HEMBA1003497, HEMBA1004007, HEMBA1004110, HEMBA1004391, HEMBA1004444, HEMBA1005230, HEMBA1005246, HEMBA1005267, HEMBA1005489, HEMBA1005913, HEMBA1006299, HEMBA1006357, HEMBA1006517, HEMBA1006544, HEMBA1006658, HEMBA1006749, HEMBA1007063, HEMBA1007241, HEMBB1000447, HEMBB1000542, HEMBB1000567, HEMBB1000642, HEMBB1000668, HEMBB1001026, HEMBB1001847, HEMBB1002051, HEMBB1002120, HEMBB1002228, HEMBB1002693, MAMMA1000106, MAMMA1000141, MAMMA1000473, MAMMA1000528, MAMMA1000810, MAMMA1000881, MAMMA1001634, MAMMA1001957, MAMMA1002205, MAMMA1002224, NT2RM2000423, NT2RM2000497, NT2RM2000582, NT2RM2001126, NT2RM2001902, NT2RM4000198, NT2RM4000284, NT2RM4000593, NT2RM4001321, NT2RP1000002, NT2RP1000050, NT2RP1000181, NT2RP1000261, NT2RP1000465, NT2RP1000468, NT2RP1000579, NT2RP1000679, NT2RP2000092, NT2RP2000479, NT2RP2000610, NT2RP2000663, NT2RP2000694, NT2RP2000903, NT2RP2001388, NT2RP2001538, NT2RP2001878, NT2RP2002015, NT2RP2002304, NT2RP2002721, NT2RP2002824, NT2RP2002942, NT2RP2002974, NT2RP2002976, NT2RP2003179, NT2RP2003302, NT2RP2003383, NT2RP2003469, NT2RP2003664, NT2RP2003940, NT2RP2004069, NT2RP2004108, NT2RP2004524, NT2RP2004556, NT2RP2004670, NT2RP2005069, NT2RP2005247, NT2RP2005425, NT2RP2005463, NT2RP2005514, NT2RP2005535, NT2RP2005541, NT2RP2005774, NT2RP2005878, NT2RP2005883, NT2RP2005887, NT2RP2006099, NT2RP2006134, NT2RP3000011, NT2RP3000125, NT2RP3000171, NT2RP3000232, NT2RP3000460, NT2RP3000481, NT2RP3000652, NT2RP3000677, NT2RP3000818, NT2RP3000820, NT2RP3001044, NT2RP3001061, NT2RP3001170, NT2RP3001240, NT2RP3001322, NT2RP3001388, NT2RP3001542, NT2RP3001592, NT2RP3001976, NT2RP3002790, NT2RP3002900, NT2RP3002983, NT2RP3003000, NT2RP3003354, NT2RP3003532, NT2RP3003729, NT2RP3003874, NT2RP3003939, NT2RP3004025, NT2RP3004083, NT2RP3004090, NT2RP3004130, NT2RP3004202, NT2RP3004294, NT2RP3004640, NT2RP4000108, NT2RP4000634, NT2RP4002451, NT2RP4002715, OVARC1000090, OVARC1000208, OVARC1000275, OVARC1000553, OVARC1000775, OVARC1000853, OVARC1000873, OVARC1000916, OVARC1000995, OVARC1001030, OVARC1001049, OVARC1001132, OVARC1001596, OVARC1002178, PLACE1000258, PLACE1000442, PLACE1000927, PLACE1000986, PLACE1001100, PLACE1001123, PLACE1001795, PLACE1002518, PLACE1002547, PLACE1002967, PLACE1003407, PLACE1003428, PLACE1003644, PLACE1003839, PLACE1004078, PLACE1004441, PLACE1004450, PLACE1005669, PLACE1005682, PLACE1005736, PLACE1005768, PLACE1005815, PLACE1006073, PLACE1006208, PLACE1007296, PLACE1007626, PLACE1008282, PLACE1008984, PLACE1008985, PLACE1010445, PLACE1011708, PLACE1011978, PLACE4000455, SKNMC1000004, THYRO1000036, THYRO1000580, THYRO1000776, THYRO1000999, THYRO1001063, THYRO1001128, THYRO1001205, THYRO1001327, THYRO1001523, THYRO1001725, THYRO1001770, Y79AA1000207, Y79AA1000226, Y79AA1000270, Y79AA1001056, Y79AA1001062, Y79AA1001090, Y79AA1001727, Y79AA1002213, Y79AA1002381

The survey also revealed the clones of which expression levels were decreased in the presence of RA. The clones are BNGH41000020, HEMBA1005070, NT2RP2005027, NT2RP3003473 and Y79AA1002376.

The same survey further revealed the following clones with elevated expression levels in the presence of RA/inhibitor:
HEMBA1000128, HEMBA1000875, HEMBA1001390, HEMBA1002163, HEMBA1002227, HEMBA1002421, HEMBA1004391, HEMBA1004454, HEMBA1004785, HEMBA1005913, HEMBA1006171, HEMBA1006299, HEMBA1006335, HEMBA1006544, HEMBA1007241, HEMBB1000447, HEMBB1000668, MAMMA1000994, MAMMA1001344, NT2RM2000582, NT2RP1001004, NT2RP2000663, NT2RP2000694, NT2RP2000903, NT2RP2001388, NT2RP2002674, NT2RP2002974, NT2RP2003383, NT2RP2004069, NT2RP2004606, NT2RP2004837, NT2RP2005069, NT2RP2005425, NT2RP2005463, NT2RP2005541, NT2RP2005883, NT2RP2005887, NT2RP3000460, NT2RP3000838, NT2RP3001044, NT2RP3001240, NT2RP3001388, NT2RP3002721, NT2RP3002738, NT2RP3003469, NT2RP3004083, NT2RP3004130, NT2RP3004202, NT2RP3004294, NT2RP3004640, NT2RP4000108, NT2RP4002451, NT2RP4002715, OVARC1000275, OVARC1000467, OVARC1000553, OVARC1000853, OVARC1000873, OVARC1000916, OVARC1000995, OVARC1001030, OVARC1001222, OVARC1001596, OVARC1002058, OVARC1002178, PLACE1000927, PLACE1001123, PLACE1001407, PLACE1001464, PLACE1001564, PLACE1001795, PLACE1002547, PLACE1003407, PLACE1003644, PLACE1003845, PLACE1004441, PLACE1004482, PLACE1005410, PLACE1005601, PLACE1005725, PLACE1005736, PLACE1006093, PLACE1006219, PLACE1006290, PLACE1006716, PLACE1007296, PLACE1007626, PLACE1008359, PLACE1010968, PLACE1011364, PLACE1011824, THYRO1000678, THYRO1000776, THYRO1000999, THYRO1001113, THYRO1001237, THYRO1001523, Y79AA1000226, Y79AA1000888, Y79AA1001430

The same survey further revealed the following clones with elevated expression levels in the presence of RA/inhibitor:
HEMBA1000349, HEMBA1001297, HEMBA1001878, HEMBA1005070, HEMBA1006482, HEMBB1001959, NT2RM2001939, NT2RP1000981, NT2RP2001469, NT2RP3003473, OVARC1001132, PLACE1001655, Y79AA1000127, Y79AA1002381. These clones are associated with neural differentiation and, therefore, are candidates for genes associated with neurological diseases.

Based on the functional analyses using a secretory protein, membrane protein, or proteins associated with signal transduction, glycoprotein, transcription, or diseases, it is possible to develop a medicine.

In case of a membrane protein, it is most likely to be a protein that functions as a receptor or ligand on the cell surface. Therefore, it is possible to reveal a new relationship between a ligand and receptor by screening the membrane protein of the invention based on the binding activity with the known ligand or receptor. Screening can be performed according to the known methods.

For example, a ligand against the protein of the invention can be screened in the following manner. Namely, a ligand that binds to a specific protein can be screened by a method comprising the steps of: (a) contacting a test sample with the protein of the invention or a partial peptide thereof, or cells expressing these, and (b) selecting a test sample that binds to said protein, said partial peptide, or said cells.

On the other hand, for example, screening using cells expressing the protein of the present invention that is a receptor protein can also be performed as follows. It is possible to screen receptors that is capable of binding to a specific protein by using procedures (a) attaching the sample cells to the protein of the invention or its partial peptide, and (b) selecting cells that can bind to the said protein or its partial peptide.

In a following screening as an example, first the protein of the invention is expressed, and the recombinant protein is purified. Next, the purified protein is labeled, binding assay is performed using a various cell lines or primary cultured cells, and cells that are expressing a receptor are selected (Growth and differentiation factors and their receptors, Shin-Seikagaku Jikken Kouza Vol.7 (1991) Honjyo, Arai, Taniguchi, and Muramatsu edit, p203-236, Tokyo-Kagaku-Doujin). A protein of the invention can be labeled with RI such as ¹²⁵I, and enzyme (alkaline phosphatase etc.). Alternatively, a protein of the invention may be used without labeling and then detected by using a labeled antibody against the protein. The cells that are selected by the above screening methods, which express a receptor of the protein of the invention, can be used for the further screening of an agonists or antagonists of the said receptor.

Once the ligand binding to the protein of the invention, the receptor of the protein of the invention or the cells expressing the receptor are obtained by screening, it is possible to screen a compound that binds to the ligand and receptor. Also it is possible to screen a compound that can inhibit both bindings (agonists or antagonists of the receptor, for example) by utilizing the binding activities.

When the protein of the invention is a receptor, the screening method comprises the steps of (a) contacting the protein of the invention or cells expressing the protein of the invention with the ligand, in the presence of a test sample, (b) detecting the binding activity between said protein or cells expressing said protein and the ligand, and (c) selecting a compound that reduces said binding activity when compared to the activity in the absence of the test sample. Furthermore, when the protein of the invention is a ligand, the screening method comprises the steps of (a) contacting the protein of the invention with its receptor or cells expressing the receptor in the presence of samples, (b) detecting the binding activity between the protein and its receptor or the cells expressing the receptor, and (c) selecting a compound that can potentially reduce the binding activity compared to the activity in the absence of the sample.

Samples to screen include cell extracts, expressed products from a gene library, synthesized low molecular compound, synthesized peptide, and natural compounds, for example, but are not construed to be listed here. A compound that is isolated by the above screening using a binding activity of the protein of the invention can also be used as a sample.

A compound isolated by the screening may be a candidate to be an agonist or an antagonist of the receptor of the protein. By utilizing an assay that monitors a change in the intracellular signaling such as phosphorylation which results from reduction of the binding between the protein and its receptor, it is possible to identify whether the obtained compound is an agonist or antagonist of the receptor. Also, the compound may be a candidate of a molecule that can inhibit the interaction between the protein and its associated proteins (including a receptor) in vivo. Such compounds can be used for developing drugs for precaution or cures of a disease with which the protein is associated.

Secretory proteins may regulate cellular conditions such as growth and differentiation. It is possible to find out a novel factor that regulates cellular conditions by adding the secretory protein of the invention to a certain kind of cell, and performing a screening by utilizing the cellular changes in growth or differentiation, or activation of a particular gene.

The screening can be performed, for example, as follows. First, the protein of the invention is expressed and purified in a recombinant form. Then, the purified protein is microinjectedadded into a various kind of cell lines or primary cultured cells, and the change in the cell growth and differentiation is monitored. The induction of a particular gene that is known to be involved in a certain cellular change is detected with the amounts of mRNA and protein. Alternatively, the amount of an intracellular molecule (low molecular compounds, etc.) that is changed by the function of a gene product (protein) that is known to be functioning in a certain cellular change is used for the detection.

Once the screening reveals that the protein of the invention can regulate cellular conditions or the functions, it is possible to apply the protein as a pharmaceutical and diagnostic medicine for associated diseases by itself or by altering a part of it into an appropriate composition.

As is above described for membrane proteins, the secretory protein provided by the invention may be used to explore a novel ligand-receptor interaction using a screening based on the binding activity to a known ligand or receptor. A similar method can be used to identify an agonist or antagonist. The resulting compounds obtained by the methods can be a candidate of a compound that can inhibit the interaction between the protein of the invention and an interacting molecule (including a receptor). The compounds may be able to use as a preventive, therapeutic, and diagnostic medicine for the diseases, in which the protein may play a certain role.

Proteins associated with signal transduction or transcription may be a factor that affects a certain protein or gene in response to intracellular/extracellular stimuli. It is possible to find out a novel factor that can affect a protein or gene by expressing the protein provided by the invention in a certain types of cells, and performing a screening utilizing the activation of a certain intracellular protein or gene.

The screening may be performed as follows. First, a transformed cell expressing the protein is obtained. Then, the transformed cell line and the untransformed original cell are compared for the changes in the expression of a certain gene by detecting the amount of its mRNA or protein. Alternatively, the amount of an intracellular molecule (low molecular compounds), which is changed by the function of a gene product (protein) that is known to function in a certain cellular change, may be used for the detection. Furthermore, the change of the expression of a certain gene can be detected by introducing a fusion gene that comprises a regulatory region of the gene and a marker gene (luciferase, beta-galactosidase, etc.) into a cell, expressing the protein provided by the invention into the cell, and estimating the activity of a marker gene product (protein).

If the protein or gene of the invention is associated with diseases, it is possible to screen a gene or compound that can regulate its expression and/or activity either directly or indirectly by utilizing the protein of the present invention.

For example, the protein of the invention is expressed and the recombinant protein is purified. Then, the protein and gene whose expression was affected in the presence of the protein of the invention is also purified, and the binding activity between the two proteins or genes is examined. The examination may be performed with pretreatment with a compound that is candidate of an inhibitor. In an alternative method, a transcription regulatory region locating in the 5'-upstream of the gene encoding the protein of the invention that is capable of regulating the expression of other genes is obtained, and fused with a marker gene. The fusion is introduced into a cell, and the cell is added with compounds to explore a regulatory factor of the expression of the said gene.

The compound obtained by the screening can be used for developing pharmaceutical and diagnostic medicines for the diseases with which the protein of the present invention is associated. Similarly, if the regulatory factor obtained by the screening is a protein, the protein itself can be used as a pharmaceutical, and if there is a compound that affects the original expression level and/or activity of the protein, it also can be used for the same purpose.

If the protein of the invention has an enzymatic activity, regardless of whether it is a secretory protein, membrane protein, or proteins associated with signal transduction, glycoprotein, transcription, or diseases, a screening may be performed by adding a compound to the protein of the invention under the suitable condition and monitoring the change of the compound. The enzymatic activity may also be utilized to screen for a compound that can inhibit the activity of the protein.

In a screening given as an example, the protein of the invention is expressed and the recombinant protein is purified. Then, compounds are contacted with the purified protein, and the amount of the compound and the reaction products is examined. Alternatively, compounds that are candidates of an inhibitor are pretreated, then a compound (substrate) that can react with the purified protein is added, and the amount of the substrate and the reaction products is examined.

The compounds obtained in the screening may be used as a medicine for diseases with which the protein of the invention is associated. Also they can be applied for tests that examine whether the protein of the invention functions normally *in vivo*.

Whether the secretory or membrane protein of the present invention is a novel protein associated with diseases or not is determined in another method than described above, by obtaining a specific antibody against the protein of the invention, and examining the relationship between the expression or activity of the protein and a certain disease. In an alternative way, it may be analyzed referred to the methods in "Molecular Diagnosis of Genetic Diseases" (Elles R. edit, (1996) in the series of "Method in Molecular Biology" (Humana Press).

Disease-associated proteins are a target of the above described screenings and very useful for developing a drug that is capable of regulating the expression and activity of the protein. Also, they are useful in medicinal industry as a diagnostic marker of the related disease and as a target for gene therapy.

Compounds isolated as mentioned above can be administered patients as it is, or after formulated into a pharmaceutical composition according to the known methods. For example, a pharmaceutically acceptable carrier or vehicle, specifically sterilized water, saline, plant oil, emulsifier, or suspending agent can be mixed with the compounds appropriately. The pharmaceutical compositions can be administered to patients by a method known to those skilled in the art, such as intraarterial intravenous, or subcutaneous injections. The dosage may vary depending on the weight or age of a patient, or the method of administration, but those skilled in the art can choose an appropriate dosage properly. If the compound is encoded by DNA, the DNA can be cloned into a vector for gene therapy, and used for gene therapy. The dosage of the DNA and the method of its administration may vary depending on the weight or age of a patient, or the symptoms, but those skilled in the art can choose properly.

The protein encoded by the polynucleotide of the invention can be prepared as a recombinant protein or as a natural protein. For example, the recombinant protein can be prepared by inserting the polynucleotide encoding the protein of the invention into a vector, introducing the vector into an appropriate host cell and purifying the protein expressed within the transformed host cell, as described below. In contrast, the natural protein can be prepared, for example, by utilizing an affinity column to which an antibody against the protein of the invention (Current Protocols in Molecular Biology (1987) Ausubel et al. edit, John Wily & Sons, Section 16.1-16.19) is attached. The antibody used for the affinity purification may be either a polyclonal antibody, or a monoclonal antibody. Alternatively, in vitro translation (See, for example, "On the fidelity of mRNA translation in the nuclease-treated rabbit reticulocyte lysate system." Dasso M.C., and Jackson R.J. (1989) Nucleic Acids Res. 17: 3129-3144) may be used for preparing the protein of the invention.

Proteins functionally equivalent to the proteins of the present invention can be prepared based on the activities, which were clarified in the above-mentioned manner, of the proteins of the present invention. Using the biological activity possessed by the protein of the invention as an index, it is possible to verify whether or not a particular protein is functionally equivalent to the protein of the invention by examining whether or not the protein has said activity.

Proteins functionally equivalent to the proteins of the present invention can be prepared by those skilled in the art, for example, by using a method for introducing mutations into an amino acid sequence of a protein (for example, site-directed mutagenesis (Current Protocols in Molecular Biology, edit, Ausubel et al., (1987) John Wiley & Sons, Section 8.1-8.5). Besides, such proteins can be generated by spontaneous mutations. The present invention comprises the proteins having one or more amino acid substitutions, deletions, insertions and/or additions in the amino acid sequences of the proteins of the present invention (Table 370), as far as the proteins have the equivalent functions to those of the proteins identified in the present Examples described later.

There are no limitations in the number and sites of amino acid mutations, as far as the proteins maintain the functions thereof. The number of mutations is typically 30% or less, or 20% or less, or 10% or less, preferably within 5% or less, or 3% or less of the total amino acids, more preferably within 2% or less or 1% or less of the total amino acids. From the viewpoint of maintaining the protein function, it is preferable that a substituted amino has a similar property to that of the original amino acid. For example, Ala, Val, Leu, Ile, Pro, Met, Phe and Trp are assumed to have similar properties to one another because they are all classified into a group of non-polar amino acids. Similarly, substitution can be performed among non-charged amino acid such as Gly, Ser, Thr, Cys, Tyr, Asn, and Gln, acidic amino acids such as Asp and Glu, and basic amino acids such as Lys, Arg, and His.

In addition, proteins functionally equivalent to the proteins of the present invention can be isolated by using techniques of hybridization or gene amplification known to those skilled in the art. Specifically, using the hybridization technique (Current Protocols in Molecular Biology, edit, Ausubel et al., (1987) John Wiley & Sons, Section 6.3-6.4)), those skilled in the art can usually isolate a DNA highly homologous to the DNA encoding the protein identified in the present Example based on the identified nucleotide sequence (Table 370) or a portion thereof and obtain the functionally equivalent protein from the isolated DNA. The present invention includes proteins encoded by the DNAs hybridizing with the DNAs encoding the proteins identified in the present Example, as far as the proteins are functionally equivalent to the proteins identified in the present Example. Organisms from which the functionally equivalent proteins are isolated are illustrated by vertebrates such as human, mouse, rat, rabbit, pig and bovine, but are not limited to these animals.

Washing conditions of hybridization for the isolation of DNAs encoding the functionally equivalent proteins are usually "1×SSC, 0.1 % SDS, 37°C"; more stringent conditions are "0.5×SSC, 0.1% SDS, 42°C"; and still more stringent conditions are "0.1×SSC, 0.1% SDS, 65°C". Alternatively, the following conditions can be given as hybridization conditions of the present invention. Namely, conditions in which the hybridization is done at "6×SSC, 40% Formamide, 25°C", and the washing at "1×SSC, 55°C" can be given. More preferable conditions are those in which the hybridization is done at "6×SSC, 40% Formamide, 37°C", and the washing at "0.2 × SSC, 55°C". Even more preferable are those in which the hybridization is done a1 "6×SSC, 50% Formamide, 37°C", and the washing at "0.1×SSC, 62°C". The more stringent the conditions of hybridization are, the more frequently the DNAs highly homologous to the probe sequence are isolated. Therefore, it is preferable to conduct hybridization under stringent conditions. Examples of stringent conditions in the present invention are, washing conditions of "0.5×SSC, 0.1% SDS, 42°C", or alternatively, hybridization conditions of "6×SSC, 40% Formamide, 37°C", and the washing at "0.2×SSC, 55°C". However, the above-mentioned combinations of SSC, SDS and temperature conditions are indicated just as examples. Those skilled, in the art can select the hybridization conditions with similar stringency to those mentioned above by properly combining the above-mentioned or other factors (for example, probe concentration, probe length and duration of hybridization reaction) that determines the stringency of hybridization.

The amino acid sequences of proteins isolated by using the hybridization techniques usually exhibit high homology to those of the proteins of the present invention, which are shown in Table 370. The present invention encompasses a polynucleotide comprising a nucleotide sequence that has a high identity to the nucleotide sequence of claim 8 (a). Furthermore, the present invention encompasses a peptide, or protein comprising an amino acid sequence that has a high identity to the amino acid sequence encoded by the polynucleotide of claim 8 (b). The term "high identity" indicates sequence identity of at least 40% or more; preferably 60% or more; and more preferably 70% or more. Alternatively, more preferable is identity of 90% or more, or 93% or more, or 95% or more, furthermore, 97% or more, or 99% or more. The identity can be determined by using the BLAST search algorithm.

With the gene amplification technique (PCR) (Current Protocols in Molecular Biology, edit, Ausubel et al., (1987) John Wiley & Sons, Section 6.3-6.4)) using primers designed based on the nucleotide sequence (Table 370) or a portion thereof identified in the present Example, it is possible to isolate a DNA fragment highly homologous to the nucleotide sequence or a portion thereof and to obtain functionally equivalent protein to a particular protein identified in the present Example based on the isolated DNA fragment.

The "percent identity" of two amino acid sequences or of two nucleic acids is determined using the algorithm of Karlin and Altschul (Proc. NatL Acad. Sei. USA 87:2264-2268, 1990), modified as in Karlin and Altschul (Proc. Natl. Acad. Sei. USA 90:5873-5877, 1993), Such an algorithm is incorporated into the BLASTN and BLASTX programs of Altschul et al. (J. Mol. Biol.215:403-410, 1990). BLAST nucleotide searches arc performed with the BLASTN program, score = 100, wordlength = 12. BLAST protein searches are performed with the BLASTX program, score = 50, wordlength = 3. When gaps exist between two sequences, Gapped BLAST is utilized as described in Altschul et al. (Nucleic Acids Res.25:3389-3402,1997). When utilizing BLAST and Gapped BLAST programs, the default parameters of the respective programs (e.g., BLASTX and BLASTN) are used. See http://www.ncbi.nlm.nih.gov.

The present invention also includes a partial peptide of the proteins of the invention. The partial peptide comprises a protein generated as a result that a signal peptide has been removed from a secretory protein. If the protein of the present invention has an activity as a receptor or a ligand, the partial peptide may function as a competitive inhibitor of the protein and may bind to the receptor (or ligand). In addition, the present invention comprises an antigen peptide for raising antibodies. For the peptides to be specific for the protein of the invention, the peptides comprise at least 7 amino acids, preferably 8 amino acids or more, more preferably 9 amino acids or more, and even more preferably 10 amino acids or more. The peptide can be used for preparing antibodies against the protein of the invention, or competitive inhibitors of them, and also screening for a receptor that binds to the protein of the invention. The partial peptides of the invention can be produced, for example, by genetic engineering methods, known methods for synthesizing peptides, or digesting the protein of the invention with an appropriate peptidase.

The present invention also relates to a vector into which the DNA of the invention is inserted. The vector of the invention is not limited as long as it contains the inserted DNA stably. For example, if E. coli is used as a host, vectors such as pBluescript vector (Stratagene) are preferable as a cloning vector. To produce the protein of the invention, expression vectors are especially useful. Any expression vector can be used as far as it is capable of expressing the protein in vitro, in E. coli, in cultured cells, or in vivo. For example, pBEST vector (Promega) is preferable for in vitro expression, pET vector (Invitrogen) for E, coli, pME18S-FL3 vector (GenBank Accession No. AB009864) for cultured cells, and pME18S vector (Mol. Cell, Biol. (1988) 8: 466-472) for in vivo expression. To insert the DNA of the invention, ligation utilizing restriction sites can be performed according to the standard method (Current Protocols in Molecular Biology (1987) Ausubei et al. edit, John Wily & Sons, Section 11.4-11.11).

The present invention also relates to a transformant carrying the vector of the invention. Any cell can be used as a host into which the vector of the invention is inserted, and various kinds of host cells can be used depending on the purposes. For strong expression of the protein in eukaryotic cells, COS cells or CHO cells can be used, for example.

Introduction of the vector into host cells can be performed, for example, by calcium phosphate precipitation method, electroporation method (Current Protocols in Molecular Biology (1987) Ausubel et al. edit, John Wily & Sons, Section 9.1-9.9), lipofectamine method (GIBCO-BRL), or microinjection method, etc.

The primer of the present invention can be used for synthesizing full-length cDNA, and also for the detection and/or diagnosis of the abnormality of the protein of the invention encoded by the full-length cDNA. For example, by utilizing polymerase chain reaction (genomic DNA-PCR, or RT-PCR) using the primer of the invention, DNA encoding the protein of the invention can be amplified. It is also possible to obtain the regulatory region of expression in the 5'-upstream by using PCR or hybridization since the transcription start site within the genomic sequence can be easily specified based on the 5'-end sequence of the full-length cDNA. The obtained genomic region can be used for detection and/or diagnosis of the abnormality of the sequence by RFLP analysis, SSCP, or direct sequencing.

Furthermore, the "polynucleotide having a length of at least 15 nucleotides, comprising a nucleotide sequence that is complementary to a polynucleotide comprising the nucleotide sequence set forth in any one of SEQ ID NOs in Table 370, or its complementary strand" includes an antisense polynucleotide for suppressing the expression of the protein of the invention. To exert the antisense effect, the antisense polynucleotide has a length of at least 15 bp or more, for example, 50 bp or more, preferably 100 bp or more, and more preferably 500 bp or more, and has a length of usually 3000 bp or less and preferably 2000 bp or less. The antisense DNA can be used in the gene therapy of the diseases that are caused by the abnormality of the protein of the invention (abnormal function or abnormal expression). Said antisense DNA can be prepared, for example, by the phosphorothioate method ("Physicochemical properties of phosphorothioate oligodeoxynucleotides." Stein (1988) Nucleic Acids Res. 16: 3209-3221) based on the nucleotide sequence of the DNA encoding the protein (for example, the DNA set forth in any one of SEQ ID NOs in Table 370).

The polynucleotide or antisense DNA of the present invention can be used in gene therapy, for example, by administrating it into a patient by the in vivo or ex vivo method with virus vectors such as retrovirus vectors, adenovirus vectors, and adeno-associated virus vectors, or non-virus vectors such as liposome.

The present invention also relates to antibodies that bind to the protein of the invention. There are no limitations in the form of the antibodies of the invention. They include polyclonal antibodies, monoclonal antibodies, or their portions that can bind to the protein of the invention. They also include antibodies of all classes. Furthermore, special antibodies such as humanized antibodies are also included.

The polyclonal antibody of the invention can be obtained according to the standard method by synthesizing an oligopeptide corresponding to the amino acid sequence and immunizing rabbits with the peptide (Current Protocols in Molecular Biology (1987) Ausubel et al. edit, John Wily & Sons, Section 11.12-11.13). The monoclonal antibody of the invention can be obtained according to the standard method by purifying the protein expressed in E. coli, immunizing mice with the protein, and producing a hybridoma cell by fusing the spleen cells and myeloma cells (Current Protocols in Molecular Biology (1987) Ausubel et al. edit, John Wily & Sons, Section 11.4-11.11).

The antibody binding to the protein of the present invention can be used for purification of the protein of the invention, and also for detection and/or diagnosis of the abnormalities of the expression and structure of the protein. Specifically, proteins can be extracted, for example, from tissues, blood, or cells, and the protein of the invention is detected by Western blotting, immunoprecipitation, or ELISA, etc. for the above purpose.

Furthermore, the antibody binding to the protein of the present invention can be utilized for treating the diseases that associates with the protein of the invention. If the antibodies are used for treating patients, human antibodies or humanized antibodies are preferable in terms of their low antigenicity. The human antibodies can be prepared by immunizing a mouse whose immune system is replaced with that of human ("Functional transplant of megabase human immunoglobulin loci recapitulates human antibody response in mice" Mendez M.J. et al. (1997) Nat. Genet. 15: 146-156). The humanized antibodies can be prepared by recombination of the hypervariable region of a monoclonal antibody (Methods in Enzymology (1991) 203: 99-121).

The present invention further relates to databases comprising at least a sequence of polynucleotide and/or protein, or a medium recorded in such databases, selected from the sequence data of the nucleotide and/or the amino acids indicated in Table 370. The term "database" means a set of accumulated information as machine-searchable and readable information of nucleotide sequence. The databases of the present invention comprise at least one of the novel nucleotide sequences of polynucleotide provided by the present invention. The databases of the present invention can consist of only the sequence data of the polynucleotide provided by the present invention or can comprise other information on nucleotide sequences of known full-length cDNAs or ESTs. The databases of the present invention can be comprised of not only the information on the nucleotide sequences but also the information on the gene functions revealed by the present invention. Additional information such as names of DNA clones carrying the full-length cDNAs can be recorded or linked together with the sequence data in the databases.

The database of the present invention is useful for gaining complete gene sequence information from partial sequence information of a gene of interest. The database of the present invention comprises nucleotide sequence information of full-length cDNAs. Consequently, by comparing the information in this database with the nucleotide sequence of a partial gene fragment yielded by differential display method or subtraction method, the information on the full-length nucleotide sequence of interest can be gained from the sequence of the partial fragment as a starting clue.

The sequence information of the full-length cDNAs constituting the database of the present invention contains not only the information on the complete sequences but also extra information on expression frequency of the genes as well as homology of the genes to known genes and known proteins. Thus the extra information facilitates rapid functional analyses of partial gene fragments. Further, the information on human genes is accumulated in the database of the present invention, and therefore, the database is useful for isolating a human homologue of a gene originating from other species. The human homologue can be isolated based on the nucleotide sequence of the gene from the original species.

At present, information on a wide variety of gene fragments can be obtained by differential display method and subtraction method. In general, these gene fragments are utilized as tools for isolating the full-length sequences thereof. When the gene fragment corresponds to an already-known gene, the full-length sequence is easily obtained by comparing the partial sequence with the information in known databases. However, when there exists no information corresponding to the partial sequence of interest in the known databases, cDNA cloning should be carried out for the full-length cDNA. It is often difficult to obtain the full-length nucleotide sequence using the partial sequence information as an initial clue. If the full-length of the gene is not available, the amino acid sequence of the protein encoded by the gene remains unidentified. Thus the database of the present invention can contribute to the identification of full-length cDNAs corresponding to gene fragments, which cannot be revealed by using databases of known genes.

The invention is illustrated more specifically with reference to the following examples, but is not to be construed as being limited thereto.

### EXAMPLE 1

Construction of a cDNA library by the oligo-capping method.
The NT-2 neuron progenitor cells (Stratagene), a teratocarcinoma cell line from human embryo testis, which can differentiate into neurons by treatment with retinoic acid were used. The NT-2 cells were cultured according to the manufacturer's instructions as follows.
(1)NT-2 cells were cultured without induction by retinoic acid treatment ((NT2RM1, NT2RM2, NT2RM4)).
(2) After cultured, NT-2 cells were induced by adding retinoic acid, and then were cultured for 48 hours (NT2RP1).
(3) After cultured, NT-2 cells were induced by adding retinoic acid, and then were cultured for 2 weeks (NT2RP2, NT2RP3, NT2RP4).

Also, the human brain neuroglioma cell line H4 (ATCC HTG-148) (BNGH41), human neuroblastoma cell line SK-N-MC (ATCC HTB-10) (SKNMC1), and human retinoblastoma cell line Y79 (ATCC HTB-18) (Y79AA1) were cultured according to the culture conditions described in the ATCC catalogue. The cells were harvested separately, and mRNA was extracted from each cell by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.

Similarly, human placenta (PLACE1, PLACE2, PLACE3), human ovary cancer tissue (OVARC1), tissues from human embryo at 10 weeks, which is enriched with head (HEMBA1), or body (HEMBB1), human mammary gland (MAMMA1), human thyroid gland (THYRO1) were used to extract mRNA by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.

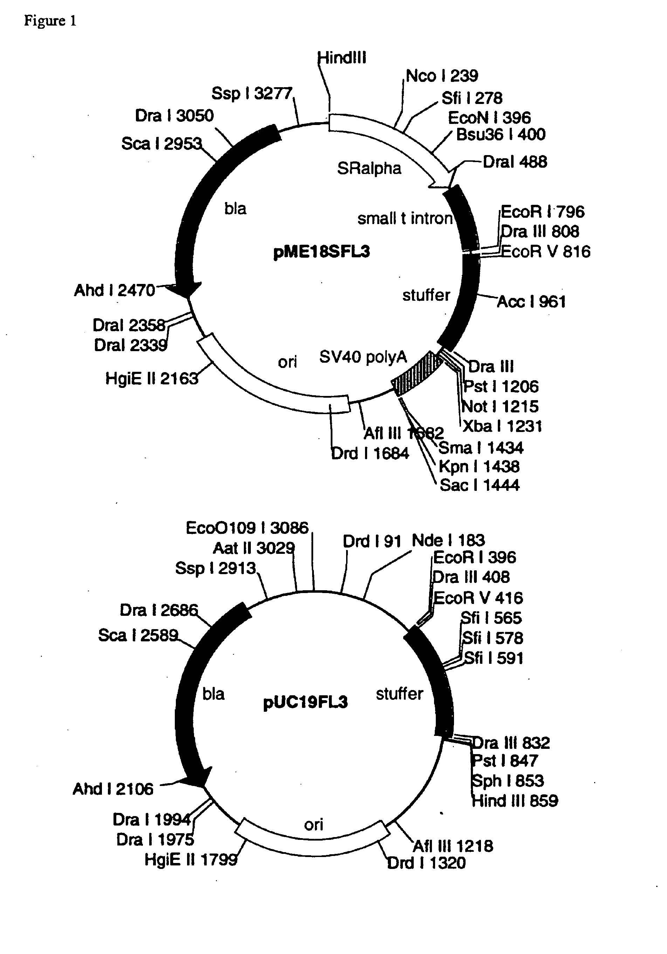

Each poly(A)⁺RNA was used to construct a cDNA library by the oligo-capping method (Maruyama M. and Sugano S. (1994) Gene 138: 171-174). Using the Oligo-cap linker (SEQ ID NO: 2541) and the Oligo-dT primer (SEQ ID NO: 2542), bacterial alkaline phosphatase (BAP) treatment, tobacco acid phosphatase (TAP) treatment, RNA ligation, the first strand cDNA synthesis, and removal of RNA were performed as described in the reference (Suzuki and Kanno (1996) Protein Nucleic acid and Enzyme. 41: 197-201; Suzuki Y. et al. (1997) Gene 200: 149-156). Next, 5'- and 3'-PCR primers (SEQ ID NO: 2543, and 2544, respectively) were used for performing PCR to convert the cDNA into double stranded cDNA, which was then digested with SfiI. Then, the DraIII-cleaved pUC19FL3 vector (Figure 1; for NT2RM1, and NT2RP1), or the DraIII-cleaved pME18SFL3 (Figure 1) (GenBank AB009864, expression vector; for NT2RM2, NT2RM4, NT2RP2, NT2RP3, NT2RP4, BNGH41, SKNMC1, Y79AA1, PLACE1, PLACE2, PLACE3,OVARC1, HEMBA1, HEMBB1, MAMMA1, and THYRO1) was used for cloning the cDNA in an unidirectional manner, and cDNA libraries were obtained. Then, the nucleotide sequence of the 5'- and 3'- ends of the cDNA clones was analyzed with a DNA sequencer (ABI PRISM 377, PE Biosystems) after sequencing reactions were performed with the DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, from by PE Biosystems) according to the instructions. The data were compiled into a database.

The full-length-enriched cDNA libraries except those for NT2RM1 and NT2RP1 were constructed using eukaryotic expression vector pME18SFL3. The vector contains SRα promoter and SV40 small t intron in the upstream of the cloning site, and SV40 polyA added signal sequence site in the downstream. As the cloning site of pME18SFL3 has asymmetrical Dram sites, and the ends of cDNA fragments contain SfiI sites complementary to the DraIII sites, the cloned cDNA fragments can be inserted into the downstream of the SRα promoter unidirectionally. Therefore, clones containing full-length cDNA can be expressed transiently by introducing the obtained plasmid directly into COS cells. Thus, the clones can be analyzed very easily in terms of the proteins that are the gene products of the clones, or in terms of the biological

Herein, the cDNA libraries and the name of each clone are related as shown in Table 2. Therein, "xxxxxx" represents the clone number of six digits. Thus, the sequences are named by the library name, the clone number plus F- for the 5'-end, or R- for the 3'-end.

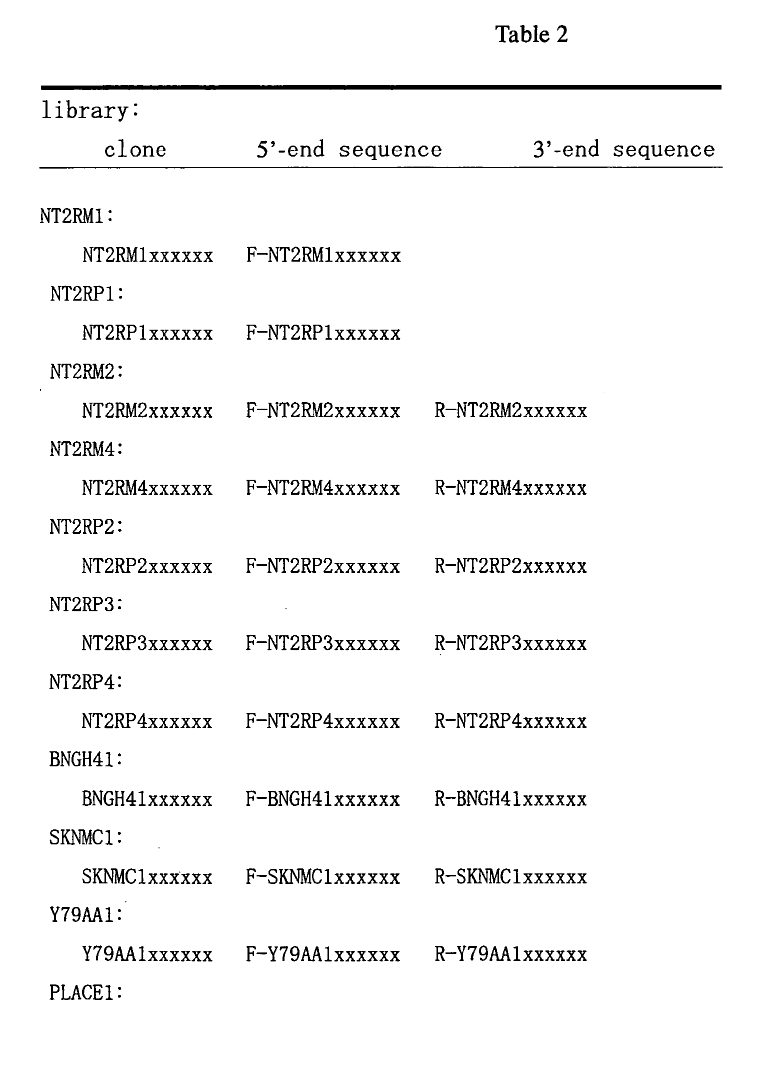

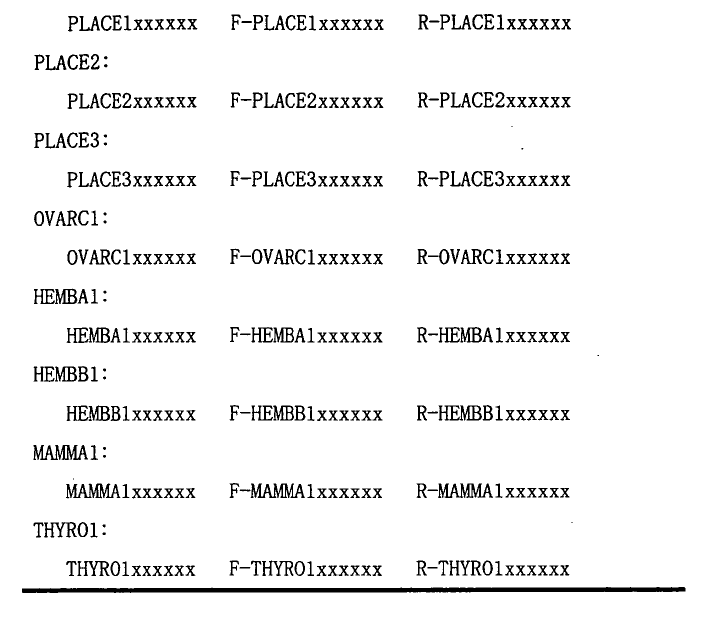

### EXAMPLE 2

Estimation of the fullness ratio of the 5'-ends of the clones contained in the cDNA libraries constructed by the oligo-capping method.

The fullness ratio at the 5'-end sequences of the 59,823 clones in the human cDNA libraries constructed by the oligo-capping method was determined as follows. Of all the clones whose 5'-end sequences were found in those of known human mRNA in the public database, a clone was judged to be "full-length", if it had a longer 5'-end sequence than that of the known human mRNA, or, even though the 5'-end sequence was shorter, if it contained the translation initiation codon. A clone which did not contain the translation initiation codon was judged to be "non-full-length". The fullness ratio ((the number of full-length clones)/(the number of full-length and non-full-length clones)) at the 5'-end of the cDNA clones from each library was determined by comparing with the known human mRNA. As a result, the fullness ratio of the 5'-ends was 63.5%. It suggests that the human cDNA clones obtained by the described method have complete 5'-ends with high probability.

### EXAMPLE 3

### Assessment of the fullness ratio of the 5'-end of the cDNA by the ATGpr and the ESTiMateFL.

The ATGpr, developed by Salamov A.A., Nishikawa T., and Swindells M.B. in the Helix Research Institute, is a program for prediction of the translation start codon based on the characteristics of the sequences in the vicinity of the ATG codon. The results are shown with expectations that an ATG is a true start codon (0.05-0.94). When this program is applied to general cDNAs without considering whether or not the ATG codons in the cDNAs are the true initiation codons of the cDNAs, both the sensitivity and the specificity of the results are estimated at 66%. Here, the sensitivity means the ratio of the number of codons judged to be initiation codons by the program to the total number of true initiation codons, and the specificity means the ratio of the number of true initiation codons to the number of codons judged to be initiation codons by the program. In contrast, when the program was applied to the 5'-end sequences of the clones from the cDNA library that was obtained by the oligo-capping method and that had 65% fullness ratio, the sensitivity and specificity of evaluation of a full-length clone (clone containing the N-terminal end of ORF) were improved to 82-83% by selecting only clones having the ATGpr1 score 0.6 or higher.

Furthermore, the program was used to assess the fullness of 18,959 clones in the human cDNA libraries obtained here, which have 5'-ends matched to a known human mRNA. Briefly, the maximal ATGpr1 score of the clones was determined, and then their 5'-end sequence was compared with the known human mRNA to estimate whether the clone is full-length or not. The result was summarized in Table 3. Based on the knowledge that known mRNAs, in general, are highly expressed in the cell, the expression levels of genes having a low number in the EST hit, which represent mRNAs whose expression levels are relatively low were examined, and the result is shown in Table 4.

In the table, the number of full-length clones indicate that of clones containing the N-terminal end of ORF, and so does the number of non-full-length clones that of clones without the N-terminal end of ORF. The fullness ratio represents (the number of full-length clones)/(the number of full-length clones plus the number of non-full-length clones).

| | | | |
|---|---|---|---|
| The maximal ATGpr1 score and the fullness ratio of the 5'-end sequences of clones obtained from human cDNA libraries constructed by the oligo-capping method; clones having a matched 5'-end with that of a known human mRNA. | | | |

| maximal ATGpr1 score | number of (full-length clones plus non-full-length clones) | number of full-length clones | fullness ratio |
|---|---|---|---|
| >=0.70 | 11,193 | 9,346 | 83.5% |
| >=0.50 | 13,369 | 10,549 | 78.9% |
| >=0.30 | 15,489 | 11,340 | 73.2% |
| >=0.15 | 17,394 | 11,811 | 67.9% |
| >=0.00 | 18,959 | 12,046 | 63.5% |

| | | | |
|---|---|---|---|
| The maximal ATGpr1 score and the fullness ratio of the 5'-end sequences of the clones obtained from human cDNA libraries constructed by the oligo-capping method; clones having 5 EST hits or less among the clones having a matched 5'-end with that of a known human mRNA. | | | |

| maximal ATGpr1 score | number of (full-length clones plus non-full-length clones) | number of full-length clones | fullness ratio |
|---|---|---|---|
| >=0.70 | 2,801 | 1,934 | 69.0% |
| >=0. 50 | 3, 683 | 2, 393 | 65.0% |
| >=0.30 | 4,683 | 2,707 | 57.8% |
| >=0.15 | 5,559 | 2,890 | 52.0% |
| >=0.00 | 6, 113 | 3,013 | 49.8% |

The ESTiMateFL, developed by Nishikawa and Ota in the Helix Research Institute, is a method for the selection of a clone with high fullness ratio by comparing with the 5'-end or 3'-end sequences of ESTs in the public database.

By the method, a cDNA clone is judged presumably not to be full-length if there exist any ESTs which have longer 5'-end or 3'-end sequences than the clone. The method is systematized for high throughput analysis. A clone is judged to be full-length if the clone has a longer 5'-end sequence than ESTs in the public database. Even if a clone has a shorter 5'-end, the clone is judged to be full-length if the difference in length is within 50 bases, and otherwise judged not to be full-length, for convenience. In case of the 5'-end sequence of the clones which matches a known mRNA, about 80% of the sequences that were judged to be full-length by comparing with ESTs was judged to be full-length by estimating the 5'-end sequence, as well; about 80% of the sequences that were judged to be not full-length by comparing with ESTs was judged to be not full-length by estimating the 5'-end sequence, as well. The accuracy of the prediction by comparing cDNA clones with ESTs is improved with increasing number of ESTs to be compared. However, when only a limited number of ESTs are available, the reliability becomes low. Thus, the method is effective in excluding clones with high probability of being non-full-length, from the cDNA clones that is synthesized by the oligo-capping method and that have the 5'-end sequences with about 60 % fullness ratio. In particular, the ESTiMateFL is efficiently used to estimate the fullness ratio at the 3'-end sequence of cDNA of a human unknown mRNA which has a significant number of ESTs in the public database.

The 18,959 clones isolated from human cDNA libraries constructed by the oligo-capping method, which have the 5'-end sequence that matches a known human mRNA, were estimated by using the ATGpr and ESTiMateFL. Briefly, the 5'-end sequence that matches a known human mRNA of the respective clone was analyzed to obtain the maximal ATGpr1 score, and compared with the ORF of the known human mRNA that matches it to determine whether the clone is full-length or not. Then, the 5'-end sequence of the respective clone was analyzed by the ESTiMateFL to judge whether the clone is full-length or not. Specifically, the 5'-end sequences that match a known human mRNA of the 18,959 clones constructed by the oligo-capping method were compared with those of ESTs by the ESTiMateFL and the clones other than those that are not full-length were selected. Then, the selected clones were used to analyze the relationship between the ATGpr and the fullness ratio. The result was summarized in Table 5. Also, among the selected, the clones in which the number of the EST hit is not more than 5 were selected and analyzed. The result was summarized in Table 6, which represents the result of the analysis of mRNA with relatively low abundance.

In the Tables, the number of full-length clones, the number of non-full-length clones, and the fullness ratio indicate the number of the clones that contain the N-terminus of the ORF, the number of the clones that do not contain the N-terminus of the ORF, and (the number of full-length clones)/(the number of full-length clones) plus (the number of non-full-length clones), respectively.

| | | | |
|---|---|---|---|
| The maximal ATGpr1 score and the fullness ratio of the 5'-end sequence in the clones isolated from human cDNA libraries constructed by the oligo-capping method, which have the 5'-end sequence that matches a known human mRNA, and also other than those being not full-length according to the comparison with ESTs. | | | |

| maximal ATGpr1 score | number of (full-length clones plus non-full-length clones) | number of full-length clones | fullness ratio |
|---|---|---|---|
| >=0. 70 | 9,068 | 8,349 | 92.1% |
| >=0. 50 | 10,345 | 9,318 | 90.1% |
| >=0.30 | 11,425 | 9,964 | 87.2% |
| >=0. 15 | 12,254 | 10, 335 | 84.3% |
| >=0.00 | 12, 785 | 10,484 | 82.0% |

| | | | |
|---|---|---|---|
| The maximal ATGpr1 score and the fullness ratio of the 5'-end sequence in the clones isolated from human cDNA libraries constructed by the oligo-capping method, which have the 5'-end sequence that matches a known human mRNA, and also other than those being not full-length according to the comparison with ESTs, in which the number of the EST hit is not more than 5. | | | |

| maximal ATGpr1 score | number of (full-length clones plus non-full-length clones) | number of full-length clones | fullness ratio |
|---|---|---|---|
| >=0. 70 | 1,959 | 1, 510 | 77. 1% |
| >=0. 50 | 2,469 | 1, 821 | 73.8% |
| >=0. 30 | 2,975 | 2,046 | 68.8% |
| >=0. 15 | 3, 368 | 2, 164 | 64. 3% |
| >=0. 00 | 3,661 | 2,226 | 60. 8% |

According to the above results, it was found that, in case of using clones isolated from human cDNA libraries constructed by the oligo-capping method, the fullness ratio of the clones that have low score in the ATGpr can be improved by assessing their 5'-end sequence using the combination of the ATGpr and the ESTiMateFL. Therefore, the method was applied to select a cDNA clone with high fullness ratio.

### EXAMPLE 4

### Clustering of the 5'-end and 3'-end sequences of cDNA clones.

The 5'-end and 3'-end sequences of cDNA clones were obtained, and clustered separately. Briefly, data of the single pass sequencing of the determined 5'-end and 3'-end of cDNA clones was subjected to the BLAST search between the sequence data of all the clones synthesized in Example 1, and clones that are supposed to be originating from the same gene were clustered into a group. For the 5'-end sequence, those having the consensus sequence of 95% identity 300 base pairs or more are clustered into the same group. For the 3'-end sequence, those having the consensus sequence of 90% identity 200 base pairs or more are clustered into the same group. Among the clusters of the 5'-end and 3'-end sequences, the sequence having the longest lead was chosen as the representative sequence of the cluster (group).

### EXAMPLE 5

Characterization of the representative sequences and the sequences of clones Data of the 5'-end sequences of the representative sequences and clones was characterized by the following methods:
(1) judging whether it is identical to the sequence of mRNA or ESTs from human by the BLAST search of the GenBank or SwissProt, and examining whether it is full-length by comparing with the sequences of known mRNA and ESTs from human.
(2) determining the ATGpr1 score using all the initiation codons contained within the 5'-end sequence by the ATGpr which predict fullness ratio.
(3) predicting the existence of the signal sequence using all the initiation codons contained within the 5'-end sequence by the PSORT which predict signal.
   and,
(4) only with the 5'-end sequences of the representative sequences of the clusters, examining the keywords in the top hit data of the homology search of the SwissProt.

Data of the characterized representative sequences and clones was used for the final selection of the clones.

### EXAMPLE 6

### Identity to the human mRNA and human EST, and comparison of the 5'-end length.

The clones and the representative sequences of the clusters were judged to be identical to any human mRNA, if their 5'-end sequence has a region of 200 nucleotides or longer with 94% or more identity to the mRNA. The clones and the representative sequences of the clusters were judged to be identical to any human EST, if their 5'-end sequence has a region of 200 nucleotides or longer with 90% or more identity to the EST.

The clones and the representative sequences of the clusters were judged to be full-length in comparison with human mRNA, if their 5'-end sequence is longer than those of the mRNA, or it contains the translation initiation site. The clones and the representative sequences of the clusters were judged to be full-length in comparison with human EST in the database, if their 5'-end sequence is longer than those of the EST, or even though it is shorter, the difference in length between the two sequences is 50 nucleotides or less, for convenience. Otherwise, the clones and the representative sequences of the clusters were judged to be not full-length.

### EXAMPLE 7

### Prediction of the fullness ratio by the ATGpr.

The score in the ATGpr1 is the expectation to be full-length based on calculations, and the higher score reflects the higher fullness ratio as shown in Example 3. Further, the maximal ATGpr1 score represents the score obtained with all the initiation codons contained in the 5'-end sequence of the clones and the representative sequences, and are used for the characterization.

### EXAMPLE 8

### Prediction of the existence of a signal sequence by the PSORT.

Prediction of the existence of a signal sequence by the PSORT was performed on all of the amino acid sequences predicted from all the initiation codons in the 5'-end sequence of the clones and the representative sequences of the clusters. By analyzing the presence or absence of the sequence which is predicted to be a signal sequence, which is characteristics of the N-terminus of many secretory proteins, cDNA clones encoding a secretory protein or membrane protein were selected.

### EXAMPLE 9

### Prediction of the protein function by the BLAST search.

The 5'-end sequence of the representative sequences of the cluster was analyzed by the BLAST homology search of the SwissProt. The obtained top hit data was classified into those identical to the 5'-end representative sequence (identity was 90% or higher), those not identical to the 5'-end representative sequence (identity was 60% or lower, and compared sequence was not more than 25 nucleotides), and those similar to the 5'-end representative sequence (the rest of the data).

All the keywords in the SwissProt data corresponding to the top hit data were selected, and the 5'-end representative sequences were classified by the keywords relating with functions.

The keywords relating with a secretory protein or membrane protein are the followings:
growth factor,
cytokine,
hormone,
receptor,
G-protein coupled receptor,
ionic channel,
voltage-gated channel,
calcium channel,
extracellular matrix,
transmembrane, and
signal.
The keywords relating to glycoprotein is glycoprotein.
The keywords relating to signal transduction are the followings:
serine/threonine-protein kinase,
tyrosine-protein kinase, and
calmodulin-binding.

The keywords relating to transcription are the followings:
transcription regulation and activator,
transcription regulation and repressor, and
nuclear protein and repressor.

The keywords relating to diseases are disease mutation, and syndrome.

Many keywords overlapped in the respective group (receptor and transmembrane, for example), and some keywords overlapped in different groups (secretory or membrane, and diseases, etc.).

### EXAMPLE 10

### Selection of clones by characterization.

From the data obtained by the above characterization, clones encoding a novel secretory protein or membrane protein, or proteins with other predicted functions were selected by the combination of the ATGpr1 score and the prediction of the signal sequence by the PSORT, or according to the top hit data in the homology search of the SwissProt.

In selecting the clones, the 5'-end sequences that are identical to a human mRNA were ignored, whereas those that are identical to a human mRNA in part but obviously not identical in the other part were included. Because there were clones selected that are identical to a human mRNA in part but obviously not identical in the other part.

Also, if the finally selected clones were found to be not full-length compared with the sequences of human mRNA and ESTs, these clones were discarded.

### EXAMPLE 11

A method for selection of clones by the combination of the ATGpr1 score and the prediction of the signal sequence by the PSORT (a method for selection of secretory proteins and membrane proteins that are novel and full-length).

The sequences of clones and the representative sequences of their clusters were used to obtain the maximal ATGpr1 score and predict the presence of the signal sequence. First, clones were selected based on the representative sequences of the clusters. The correspondence between the name and SEQ ID of the representative sequences used for selection (Table 368), and the correspondence between the name and SEQ ID of the introns (including the representative sequences of the 5'-end and 3'-end, and ESTs) used for selection of clones from the representative sequences of the groups (Table 369) were shown in the last part of the present specification. Therein, HRIFA and HRIRA indicate the representative sequence of the 5'-end group, and that of the 3'-end group, respectively.

In the clusters in which a single clone is contained (the sequence of the 5'-end clone = the representative sequence of the 5'-end), selected were the clones that were judged to be full-length in comparison with human mRNA and ESTs, having the maximal ATGpr1 score 0.5 or higher, and predicted to contain the signal sequence, in principle. However, in the following cases, a clone having a longer 5'-end was selected: the maximal ATGpr1 score was less than 0.5, the sequence of the 5'-end was not full-length, the clone was obviously shorter although the clone was not classified into the same cluster according to the BLAST search of the other clones, or the 5'-end sequence corresponding to the 3'-end of the other clones in the same cluster in which the 3'-end sequence of the clone was contained was found to be longer by assembling. Furthermore, if there were multiple full-length clones in the same cluster and it was not successful to determine by assembling which has the longer 5'-end, all the clones were selected. For assembling, the Sequencher™ (Hitachi Soft Engineering ) was used. As a result, the signal sequence predicted to be present in the representative sequence was not present in some of the selected clones. In some cases, the ATGpr1 score became smaller than 0.5 or 0.3. The fullness ratio in these clones was low, yet still it is possible that the clones are full-length. The clones in which the signal sequence predicted to be present in the representative sequence was not present after selection were likely to be without the signal sequence, but still it is possible that the clones encode a membrane protein.

In the clusters comprising multiple clones, in which the representative sequence of the 5'-end was predicted to contain the signal sequence, selected were the clones having the longest 5'-end sequence among the clones which were judged to be full-length compared with human mRNA and ESTs, having the maximal ATGpr1 score for the 5'-end sequence 0.5 or higher, and predicted to contain the signal sequence. However, in the following cases, a clone having a longer 5'-end was selected: the maximal ATGpr1 score was less than 0.5, the sequence of the 5'-end was not full-length, the clone was obviously shorter although the clone was not classified into the same cluster according to the BLAST search of the other clones, or the 5'-end sequence corresponding to the 3'-end of the other clones in the same cluster in which the 3'-end sequence of the clone was contained was found to be longer. Furthermore, if there were multiple full-length clones in the same cluster and it was not successful to determine by assembling which has the longer 5'-end, all the clones were selected. As a result, the signal sequence predicted to be present in the representative sequence was not present in some of the selected clones. In some cases, the ATGpr1 score became smaller than 0.5 or 0.3. The fullness ratio in these clones was low, yet still it is possible that the clones are full-length. The clones in which the signal sequence predicted to be present in the representative sequence was not present after selection were likely to be without the signal sequence at the 5'-end, but still it is possible that the clones encode a membrane protein.

Next, in the clusters comprising multiple clones, in which the representative sequence of the 5'-end was predicted to have no signal sequence, selected were the clones which were judged to be full-length compared with human mRNA and ESTs, having the maximal ATGpr1 score for the 5'-end sequence 0.5 or higher, and predicted to contain the signal sequence.

The number of the clones selected by the combination of the ATGpr1 score and the prediction of a signal sequence by the PSORT were 254. The number of the clones having the maximal ATGpr1 score 0.5 or higher, and predicted to contain a signal sequence were 170 (Table 7-10). Among the clones, 164 clones were found to have the representative sequence of the original cluster that fulfills the same conditions. On the other hand, 5 clones were selected from the representative sequences of the 5'-end of the clusters which was predicted to contain a signal sequence while the maximal ATGpr1 score was lower than 0.5. A clone was selected from the representative sequence of the 5'-end of the cluster which was predicted to have no signal sequence.

The clones that have the maximal ATGpr1 score 0.3 or higher and less than 0.5 and predicted to contain the signal sequence were 35 clones (Table 11), in which 8 clones were found to have the representative sequence of the original cluster that fulfills the same conditions. Twenty-seven clones were selected from the representative sequences of the clusters which have the maximal ATGpr1 score 0.5 or higher and were predicted to have no signal sequence.

The clones that have the maximal ATGpr1 score less than 0.3 and were predicted to contain a signal sequence were 41 clones (Table 12). The clones that have the maximal ATGpr1 score 0.5 or higher and were predicted to have no signal sequence were 4 clones (Table 13). The clones that have the maximal ATGpr1 score 0.3 or higher and less than 0.5 and were predicted to have no signal sequence were 2 clones (Table 14). The clones that have the maximal ATGpr1 score less than 0.3 and were predicted to contain a signal sequence were 2 clones (Table 15). The representative sequences of the original clusters of all the clones had the maximal ATGpr1 score 0.3 or higher, and were predicted to contain a signal sequence.

The fullness ratio of the clones having the maximal ATGpr1 score 0.5 or higher, 0.3 or higher, and 0 or higher is expected to be as shown in Table 3, 4, 5, and 6.

| | | | | | | |
|---|---|---|---|---|---|---|
| The 170 clones in which the selected clones have the maximal ATGpr1 score 0.5 or higjer, and were predicted to contain a signal sequence by the PSORT | | | | | | |

| name of clone | name of sequence | maximal | signal | name of representative | maximal | signal |
|---|---|---|---|---|---|---|
| | | ATGpr1 score | | sequence | ATGpr1 score | |
| HEMBA1000713 | F-HEMBA1000713 | 0.67 | Yes | HRIFA017729a | 0.57 | Yes |
| HEMBA1000962 | F-HEMBA1000962 | 0.69 | Yes | HRIFA000899a | 0.69 | Yes |
| HEMBA1001272 | F-HEMBA1001272 | 0.94 | Yes | HRIFA001179a | 0.94 | Yes |
| HEMBA1001297 | F-HEMBA1001297 | 0.89 | Yes | HRIFA001201 a | 0.89 | Yes |
| HEMBA1002420 | F-HEMBA1002420 | 0.6 | Yes | HRIFA002195a | 0.6 | Yes |
| HEMBA1003101 | F-HEMBA1003101 | 0.67 | Yes | HRIFA002787a | 0.94 | Yes |
| HEMBA1003399 | F-HEMBA1003399 | 0.94 | Yes | HRIFA002985a | 0.94 | Yes |
| HEMBA1003732 | F-HEMBA1003732 | 0.86 | Yes | HRIFA003169a | 0.86 | Yes |
| HEMBA1004110 | F-HEMBA1004110 | 0.59 | Yes | HRIFA003379a | 0.59 | Yes |
| HEMBA1005430 | F-HEMBA1005430 | 0.69 | Yes | HRIFA020661 a | 0.69 | Yes |
| HEMBA1006016 | F-HEMBA1006016 | 0.6 | Yes | HRIFA020466a | 0.6 | Yes |
| HEMBA1006171 | F-HEMBA1006171 | 0.62 | Yes | HRIFA021399a | 0.62 | Yes |
| HEMBA1006311 | F-HEMBA1006311 | 0.94 | Yes | HRIFA021594a | 0.94 | Yes |
| HEMBA1006335 | F-HEMBA1006335 | 0.83 | Yes | HRIFA012069a | 0.94 | Yes |
| HEMBA1006357 | F-HEMBA1006357 | 0.67 | Yes | HRIFA021448a | 0.67 | Yes |
| HEMBA1006658 | F-HEMBA1006658 | 0.66 | Yes | HRIFA021323a | 0.66 | Yes |
| HEMBA1006707 | F-HEMBA1006707 | 0.66 | Yes | HRIFA021499a | 0.94 | Yes |
| HEMBA1006902 | F-HEMBA1006902 | 0.66 | Yes | HRIFA021754a | 0.94 | Yes |
| HEMBA1006960 | F-HEMBA1006960 | 0.94 | Yes | HRIFA021886a | 0.94 | Yes |
| HEMBB1000276 | F-HEMBB1000276 | 0.94 | Yes | HRIFA029577a | 0.94 | Yes |
| HEMBB1000642 | F-HEMBB1000642 | 0.94 | Yes | HRIFA029779a | 0.94 | Yes |
| HEMBB1000905 | F-HEMBB1000905 | 0.94 | Yes | HRIFA009764a | 0.91 | Yes |
| HEMBB1001200 | F-HEMBB1001200 | 0.83 | Yes | HRIFA030839a | 0.81 | Yes |
| HEMBB1001407 | F-HEMBB1001407 | 0.87 | Yes | HR1FA030981a | 0.87 | Yes |
| HEMBB1001530 | F-HEMBB1001530 | 0.6 | Yes | HRIFA031062a | 0.6 | Yes |
| HEMBB1001547 | F-HEMBB1001547 | 0.87 | Yes | HRIFA031075a | 0.87 | Yes |
| HEMBB1001978 | F-HEMBB1001978 | 0.7 | Yes | HRIFA031350a | 0.7 | Yes |
| HEMBB1002162 | F-HEMBB1002162 | 0.91 | Yes | HRIFA031472a | 0.91 | Yes |
| HEMBB1002228 | F-HEMBB1002228 | 0.53 | Yes | HRIFA031510a | 0.53 | Yes |
| HEMBB1002245 | F-HEMBB1002245 | 0.94 | Yes | HRIFA032984a | 0.94 | Yes |
| HEMBB1002427 | F-HEMBB1002427 | 0.57 | Yes | HRIFA005760a | 0.94 | Yes |
| HEMBB1002465 | F-HEMBB1002465 | 0.72 | Yes | HRIFA031672a | 0.72 | Yes |
| HEMBB1002693 | F-HEMBB1002693 | 0.64 | Yes | HRIFA031895a | 0.64 | Yes |
| MAMMA1000046 | F-MAMMA1000046 | 0.7 | Yes | HRIFA024841a | 0.7 | Yes |
| MAMMA1000102 | F-MAMMA1000102 | 0.79 | Yes | HR1FA026151a | 0.79 | Yes |
| MAMMA1000118 | F-MAMMA1000118 | 0.81 | Yes | HRIFA026153a | 0.81 | Yes |
| MAMMA1000141 | F-MAMMA1000141 | 0.8 | Yes | HRIFA024554a | 0.8 | Yes |
| MAMMA1000449 | F-MAMMA1000449 | 0.94 | Yes | HRIFA026203a | 0.94 | Yes |
| MAMMA1000457 | F-MAMMA1000457 | 0.78 | Yes | HRIFA026210a | 0.78 | Yes |
| MAMMA1000652 | F-MAMMA1000652 | 0.94 | Yes | HRIFA026346a | 0.94 | Yes |
| MAMMA1000994 | F-MAMMA1000994 | 0.84 | Yes | HRIFA026735a | 0.84 | Yes |
| MAMMA1001141 | F-MAMMA1001141 | 0.89 | Yes | HRIFA027265a | 0.89 | Yes |
| MAMMA1001310 | F-MAMMA1001310 | 0.74 | Yes | HRIFA026899a | 0.74 | Yes |
| MAMMA1001344 | F-MAMMA1001344 | 0.71 | Yes | HRIFA026918a | 0.71 | Yes |
| MAMMA1002070 | F-MAMMA1002070 | 0.6 | Yes | HRIFA028371a | 0.82 | Yes |
| MAMMA1002087 | F-MAMMA1002087 | 0.68 | Yes | HRIFA027619a | 0.68 | Yes |
| MAMMA1002165 | F-MAMMA1002165 | 0.57 | Yes | HRIFA027673a | 0.34 | Yes |
| MAMMA1002205 | F-MAMMA1002205 | 0.74 | Yes | HRIFA027701a | 0.74 | Yes |
| MAMMA1002633 | F-MAMMA1002633 | 0.53 | Yes | HRIFA030461a | 0.94 | Yes |
| NT2RM2000241 | F-NT2RM2000241 | 0.94 | Yes | HRIFA020965a | 0.94 | Yes |
| NT2RM2000514 | F-NT2RM2000514 | 0.51 | Yes | HRIFA022106a | 0.51 | Yes |
| NT2RM2001643 | F-NT2RM2001643 | 0.69 | Yes | HRIFA028926a | 0.69 | Yes |
| NT2RM4000115 | F-NT2RM4000115 | 0.56 | Yes | HRIFA025792a | 0.53 | Yes |
| NT2RM4000997 | F-NT2RM4000997 | 0.94 | Yes | HRIFA029274a | 0.94 | Yes |

| | | | | | | |
|---|---|---|---|---|---|---|
| The 170 clones in which the selected clones have the maximal ATGpr1 score 0.5 or higher, and were predicted to contain the signal sequence by the PSORT | | | | | | |

| name of clone | name of sequence | maximal | signal | name of representative | maximal | signal |
|---|---|---|---|---|---|---|
| | | ATGpr1 score | | sequence | ATGpr1 score | |
| NT2RM4001321 | F-NT2RM4001321 | 0.74 | Yes | HRIFA024533a | 0.74 | Yes |
| NT2RM4001325 | F-NT2RM4001325 | 0.94 | Yes | HRIFA033349a | 0.94 | Yes |
| NT2RM4001768 | F-NT2RM4001768 | 0.73 | Yes | HRIFA013668a | 0.5 | Yes |
| NT2RP1000448 | F-NT2RP1000448 | 0.62 | Yes | HRIFA005356a | 0.62 | Yes |
| NT2RP1001563 | F-NT2RP1001563 | 0.52 | Yes | HRIFA006018a | 0.52 | Yes |
| NT2RP2001915 | F-NT2RP2001915 | 0.94 | Yes | HRIFA007541a | 0.94 | Yes |
| NT2RP2002015 | F-NT2RP2002015 | 0.94 | Yes | HRIFA007619a | 0.94 | Yes |
| NT2RP2002063 | F-NT2RP2002063 | 0.87 | Yes | HRIFA007659a | 0.94 | Yes |
| NT2RP2002304 | F-NT2RP2002304 | 0.87 | Yes | HRIFA007829a | 0.94 | Yes |
| NT2RP2002674 | F-NT2RP2002674 | 0.8 | Yes | HRIFA008099a | 0.8 | Yes |
| NT2RP2002721 | F-NT2RP2002721 | 0.56 | Yes | HRIFA008131a | 0.56 | Yes |
| NT2RP2003383 | F-NT2RP2003383 | 0.67 | Yes | HRIFA008606a | 0.67 | Yes |
| NT2RP2003593 | F-NT2RP2003593 | 0.73 | Yes | HRIFA008252a | 0.94 | Yes |
| NT2RP2003599 | F-NT2RP2003599 | 0.58 | Yes | HRIFA008753a | 0.58 | Yes |
| NT2RP2003655 | F-NT2RP2003655 | 0.78 | Yes | HRIFA008784a | 0.83 | Yes |
| NT2RP2004179 | F-NT2RP2004179 | 0.83 | Yes | HRIFA008827a | 0.83 | Yes |
| NT2RP2004495 | F-NT2RP2004495 | 0.58 | Yes | HRIFA009372a | 0.58 | Yes |
| NT2RP2004524 | F-NT2RP2004524 | 0.73 | Yes | HRIFA009392a | 0.82 | Yes |
| NT2RP2004556 | F-NT2RP2004556 | 0.81 | Yes | HRIFA009414a | 0.81 | Yes |
| NT2RP2004837 | F-NT2RP2004837 | 0.94 | Yes | HRIFA006216a | 0.93 | Yes |
| NT2RP2005027 | F-NT2RP2005027 | 0.92 | Yes | HRIFA004145a | 0.93 | Yes |
| NT2RP2005463 | F-NT2RP2005463 | 0.93 | Yes | HRIFA010034a | 0.42 | No |
| NT2RP2005514 | F-NT2RP2005514 | 0.58 | Yes | HRIFA010070a | 0.58 | Yes |
| NT2RP2005887 | F-NT2RP2005887 | 0.94 | Yes | HRIFA010322a | 0.94 | Yes |
| NT2RP2006269 | F-NT2RP2006269 | 0.78 | Yes | HRIFA025913a | 0.57 | Yes |
| NT2RP3000169 | F-NT2RP3000169 | 0.94 | Yes | HRIFA022262a | 0.94 | Yes |
| NT2RP3000460 | F-NT2RP3000460 | 0.61 | Yes | HRIFA022794a | 0.61 | Yes |
| NT2RP3000789 | F-NT2RP3000789 | 0.62 | Yes | HRIFA023605a | 0.62 | Yes |
| NT2RP3000818 | F-NT2RP3000818 | 0.52 | Yes | HRIFA023619a | 0.52 | Yes |
| NT2RP3001012 | F-NT2RP3001012 | 0.67 | Yes | HRIFA023129a | 0.22 | Yes |
| NT2RP3001044 | F-NT2RP3001044 | 0.93 | Yes | HRIFA007026a | 0.73 | Yes |
| NT2RP3001560 | F-NT2RP3001560 | 0.58 | Yes | HRIFA030599a | 0.92 | Yes |
| NT2RP3001685 | F-NT2RP3001685 | 0.5 | Yes | HRIFA023521a | 0.5 | Yes |
| NT2RP3001858 | F-NT2RP3001858 | 0.94 | Yes | HRIFA026490a | 0.94 | Yes |
| NT2RP3002160 | F-NT2RP3002160 | 0.61 | Yes | HRIFA005760a | 0.94 | Yes |
| NT2RP3002836 | F-NT2RP3002836 | 0.68 | Yes | HRIFA024392a | 0.72 | Yes |
| NT2RP3002958 | F-NT2RP3002958 | 0.54 | Yes | HRIFA017670a | 0.91 | Yes |
| NT2RP3003535 | F-NT2RP3003535 | 0.94 | Yes | HRIFA025498a | 0.94 | Yes |
| NT2RP3004000 | F-NT2RP3004000 | 0.93 | Yes | HRIFA025276a | 0.93 | Yes |
| NT2RP3004321 | F-NT2RP3004321 | 0.81 | Yes | HRIFA025786a | 0.81 | Yes |
| NT2RP3004355 | F-NT2RP3004355 | 0.6 | Yes | HRIFA025360a | 0.6 | Yes |
| NT2RP3004374 | F-NT2RP3004374 | 0.58 | Yes | HRIFA024533a | 0.74 | Yes |
| NT2RP4001001 | F-NT2RP4001001 | 0.53 | Yes | HRIFA009214a | 0.5 | Yes |
| NT2RP4002715 | F-NT2RP4002715 | 0.94 | Yes | HRIFA024921a | 0.53 | Yes |
| OVARC1000298 | F-OVARC1000298 | 0.61 | Yes | HRIFA004852a | 0.59 | Yes |
| OVARC1000775 | F-OVARC1000775 | 0.7 | Yes | HRIFA011347a | 0.7 | Yes |
| OVARC1000811 | F-OVARC1000811 | 0.52 | Yes | HRIFA000974a | 0.39 | Yes |
| OVARC1000853 | F-OVARC1000853 | 0.94 | Yes | HRIFA011403a | 0.94 | Yes |
| OVARC1001222 | F-OVARC1001222 | 0.79 | Yes | HRIFA022714a | 0.67 | Yes |
| OVARC1001807 | F-OVARC1001807 | 0.52 | Yes | HRIFA021069a | 0.52 | Yes |
| OVARC1001833 | F-OVARC1001833 | 0.9 | Yes | HRIFA021136a | 0.9 | Yes |
| PLACE1000231 | F-PLACE1000231 | 0.52 | Yes | HRIFA011802a | 0.52 | Yes |
| PLACE1000560 | F-PLACE1000560 | 0.88 | Yes | HRIFA012022a | 0.88 | Yes |
| PLACE1000740 | F-PLACE1000740 | 0.57 | Yes | HRIFA012151a | 0.57 | Yes |

| | | | | | | |
|---|---|---|---|---|---|---|
| The 170 clones in which the selected clones have the maximal ATGpr1 score 0.5 or higher, and were predicted to contain the signal sequence by the PSORT | | | | | | |

| name of clone | name of sequence | maximal | signal | name of representative | maximal | signal |
|---|---|---|---|---|---|---|
| | | ATGpr1 score | | sequence | ATGpr1 score | |
| PLACE1000912 | F-PLACE1000912 | 0.9 | Yes | HRIFA012282a | 0.9 | Yes |
| PLACE1000914 | F-PLACE1000914 | 0.94 | Yes | HRIFA012283a | 0.94 | Yes |
| PLACE1000927 | F-PLACE1000927 | 0.71 | Yes | HRIFA012290a | 0.71 | Yes |
| PLACE1000986 | F-PLACE1000986 | 0.51 | Yes | HRIFA012333a | 0.51 | Yes |
| PLACE1001100 | F-PLACE1001100 | 0.76 | Yes | HRIFA012417a | 0.76 | Yes |
| PLACE1001183 | F-PLACE1001183 | 0.69 | Yes | HRIFA012480a | 0.69 | Yes |
| PLACE1001229 | F-PLACE1001229 | 0.65 | Yes | HRIFA012513a | 0.65 | Yes |
| PLACE1001407 | F-PLACE1001407 | 0.83 | Yes | HRIFA012069a | 0.94 | Yes |
| PLACE1001788 | F-PLACE1001788 | 0.6 | Yes | HRIFA012881a | 0.6 | Yes |
| PLACE1002374 | F-PLACE1002374 | 0.68 | Yes | HRIFA013265a | 0.92 | Yes |
| PLACE1002518 | F-PLACE1002518 | 0.94 | Yes | HRIFA018849a | 0.78 | Yes |
| PLACE1003839 | F-PLACE1003839 | 0.67 | Yes | HRIFA014178a | 0.6 | Yes |
| PLACE1003845 | F-PLACE1003845 | 0.92 | Yes | HRIFA019185a | 0.92 | Yes |
| PLACE1004199 | F-PLACE1004199 | 0.94 | Yes | HRIFA014417a | 0.94 | Yes |
| PLACE1004282 | F-PLACE1004282 | 0.94 | Yes | HRIFA014467a | 0.94 | Yes |
| PLACE1004305 | F-PLACE1004305 | 0.87 | Yes | HRIFA014482a | 0.87 | Yes |
| PLACE1004637 | F-PLACE1004637 | 0.89 | Yes | HRIFA014692a | 0.89 | Yes |
| PLACE1005005 | F-PLACE1005005 | 0.55 | Yes | HRIFA014953a | 0.55 | Yes |
| PLACE1005250 | F-PLACE1005250 | 0.52 | Yes | HRIFA015129a | 0.52 | Yes |
| PLACE1005410 | F-PLACE1005410 | 0.61 | Yes | HRIFA015236a | 0.61 | Yes |
| PLACE1005725 | F-PLACE1005725 | 0.92 | Yes | HRIFA015443a | 0.92 | Yes |
| PLACE1005768 | F-PLACE1005768 | 0.62 | Yes | HRIFA015471a | 0.62 | Yes |
| PLACE1005927 | F-PLACE1005927 | 0.66 | Yes | HRIFA015568a | 0.94 | Yes |
| PLACE1006079 | F-PLACE1006079 | 0.56 | Yes | HRIFA015671a | 0.56 | Yes |
| PLACE1006093 | F-PLACE1006093 | 0.59 | Yes | HRIFA015682a | 0.59 | Yes |
| PLACE1006219 | F-PLACE1006219 | 0.94 | Yes | HRIFA015764a | 0.93 | Yes |
| PLACE1006809 | F-PLACE1006809 | 0.66 | Yes | HRIFA016129a | 0.66 | Yes |
| PLACE1007040 | F-PLACE1007040 | 0.87 | Yes | HRIFA013288a | 0.87 | Yes |
| PLACE1007096 | F-PLACE1007096 | 0.59 | Yes | HRIFA012167a | 0.82 | Yes |
| PLACE1007626 | F-PLACE1007626 | 0.67 | Yes | HRIFA016623a | 0.67 | Yes |
| PLACE1007971 | F-PLACE1007971 | 0.74 | Yes | HRIFA016838a | 0.74 | Yes |
| PLACE1008985 | F-PLACE1008985 | 0.65 | Yes | HRIFA017457a | 0.48 | Yes |
| PLACE1009067 | F-PLACE1009067 | 0.59 | Yes | HRIFA017509a | 0.59 | Yes |
| PLACE1009196 | F-PLACE1009196 | 0.73 | Yes | HRIFA017594a | 0.73 | Yes |
| PLACE1009527 | F-PLACE1009527 | 0.58 | Yes | HRIFA017791a | 0.87 | Yes |
| PLACE1009982 | F-PLACE1009982 | 0.94 | Yes | HRIFA018075a | 0.94 | Yes |
| PLACE1011236 | F-PLACE1011236 | 0.52 | Yes | HRIFA018827a | 0.66 | Yes |
| PLACE2000219 | F-PLACE2000219 | 0.73 | Yes | HRIFA034010a | 0.73 | Yes |
| SKNMC1000004 | F-SKNMC1000004 | 0.94 | Yes | HRIFA030097a | 0.94 | Yes |
| THYRO1000036 | F-THYRO1000036 | 0.83 | Yes | HRIFA027754a | 0.83 | Yes |
| THYRO1000099 | F-THYRO1000099 | 0.94 | Yes | HRIFA027803a | 0.94 | Yes |
| THYRO1001237 | F-THYRO1001237 | 0.94 | Yes | HRIFA030248a | 0.94 | Yes |
| THYRO1001327 | F-THYRO1001327 | 0.93 | Yes | HRIFA025125a | 0.94 | Yes |
| THYRO1001495 | F-THYRO1001495 | 0.89 | Yes | HRIFA030394a | 0.89 | Yes |
| THYRO1001523 | F-THYRO1001523 | 0.71 | Yes | HRIFA030408a | 0.71 | Yes |
| THYRO1001725 | F-THYRO1001725 | 0.94 | Yes | HRIFA029107a | 0.94 | Yes |
| Y79AA1000226 | F-Y79AA1000226 | 0.94 | Yes | HRIFA027874a | 0.94 | Yes |
| Y79AA1000521 | F-Y79AA1000521 | 0.92 | Yes | HRIFA027961a | 0.92 | Yes |
| Y79AA1000776 | F-Y79AA1000776 | 0.78 | Yes | HRIFA028401a | 0.78 | Yes |
| Y79AA1000959 | F-Y79AA1000959 | 0.9 | Yes | HRIFA028465a | 0.9 | Yes |
| Y79AA1001013 | F-Y79AA1001013 | 0.94 | Yes | HRIFA011193a | 0.94 | Yes |
| Y79AA1001264 | F-Y79AA1001264 | 0.94 | Yes | HRIFA028573a | 0.94 | Yes |
| Y79AA1001328 | F-Y79AA1001328 | 0.91 | Yes | HRIFA028592a | 0.91 | Yes |
| Y79AA1001427 | F-Y79AA1001427 | 0.65 | Yes | HRIFA028652a | 0.65 | Yes |

| | | | | | | |
|---|---|---|---|---|---|---|
| The 170 clones in which the selected clones have the maximal ATGpr1 score 0.5 or higher, and were predicted to contain the signal sequence by the PSORT | | | | | | |

| name of clone | name of sequence | maximal | signal | name of representative | maximal | signal |
|---|---|---|---|---|---|---|
| | | ATGpr1 score | | sequence | ATGpr1 score | |
| Y79AA1001430 | F-Y79AA1001430 | 0.94 | Yes | HRIFA028654a | 0.94 | Yes |
| Y79AA1001530 | F-Y79AA1001530 | 0.94 | Yes | HRIFA010206a | 0.94 | Yes |
| Y79AA1001592 | F-Y79AA1001592 | 0.94 | Yes | HRIFA028708a | 0.94 | Yes |
| Y79AA1001793 | F-Y79AA1001793 | 0.89 | Yes | HRIFA032066a | 0.89 | Yes |
| Y79AA1001795 | F-Y79AA1001795 | 0.59 | Yes | HRIFA032067a | 0.59 | Yes |
| Y79AA1001863 | F-Y79AA1001863 | 0.56 | Yes | HRIFA032097a | 0.15 | Yes |
| Y79AA1002022 | F-Y79AA1002022 | 0.94 | Yes | HRIFA033718a | 0.94 | Yes |
| Y79AA1002373 | F-Y79AA1002373 | 0.79 | Yes | HRIFA032271a | 0.79 | Yes |

| | | | | | | |
|---|---|---|---|---|---|---|
| The 35 clones in which the selected clones have the maximal ATGpr1 score 0.3 or higher and less than 0.5, and were predicted to contain the signal sequence by the PSORT | | | | | | |

| name of clone | name of sequence | maximal | signal | representative | maximal | signal |
|---|---|---|---|---|---|---|
| | | ATGpr1 score | | sequence | ATGpr1 score | |
| HEMBA1000907 | F-HEMBA1000907 | 0.39 | Yes | HRIFA000845a | 0.94 | Yes |
| HEMBA1003602 | F-HEMBA1003602 | 0.43 | Yes | HRIFA020109a | 0.43 | Yes |
| HEMBA1004797 | F-HEMBA1004797 | 0.45 | Yes | HRIFA020883a | 0.66 | Yes |
| HEMBB1000447 | F-HEMBB1000447 | 0.31 | Yes | HRIFA001558a | 0.76 | Yes |
| MAMMA1000591 | F-MAMMA1000591 | 0.34 | Yes | HRIFA026303a | 0.77 | Yes |
| MAMMA1000681 | F-MAMMA1000681 | 0.35 | Yes | HRIFA026364a | 0.94 | Yes |
| MAMMA1000986 | F-MAMMA1000986 | 0.37 | Yes | HRIFA021611a | 0.37 | Yes |
| MAMMA1001893 | F-MAMMA1001893 | 0.44 | Yes | HRIFA027485a | 0.9 | Yes |
| MAMMA1001957 | F-MAMMA1001957 | 0.48 | Yes | HRIFA027536a | 0.94 | Yes |
| NT2RM2001941 | F-NT2RM2001941 | 0.44 | Yes | HRIFA032011a | 0.94 | Yes |
| NT2RP1000050 | F-NT2RP1000050 | 0.47 | Yes | HRIFA005102a | 0.54 | Yes |
| NT2RP1000903 | F-NT2RP1000903 | 0.38 | Yes | HRIFA005650a | 0.38 | Yes |
| NT2RP2003469 | F-NT2RP2003469 | 0.33 | Yes | HRIFA008661a | 0.9 | Yes |
| NT2RP2003664 | F-NT2RP2003664 | 0.36 | Yes | HRIFA008790a | 0.89 | Yes |
| NT2RP2004447 | F-NT2RP2004447 | 0.36 | Yes | HRIFA009339a | 0.93 | Yes |
| NT2RP2006042 | F-NT2RP2006042 | 0.37 | Yes | HRIFA010425a | 0.69 | Yes |
| NT2RP3001195 | F-NT2RP3001195 | 0.44 | Yes | HRIFA023227a | 0.94 | Yes |
| NT2RP3003354 | F-NT2RP3003354 | 0.3 | Yes | HRIFA008212a | 0.51 | Yes |
| NT2RP3003469 | F-NT2RP3003469 | 0.3 | Yes | HRIFA025143a | 0.3 | Yes |
| NT2RP3003963 | F-NT2RP3003963 | 0.44 | Yes | HRIFA008949a | 0.62 | Yes |
| NT2RP3004133 | F-NT2RP3004133 | 0.35 | Yes | HRIFA025706a | 0.94 | Yes |
| NT2RP3004309 | F-NT2RP3004309 | 0.4 | Yes | HRIFA025778a | 0.92 | Yes |
| OVARC1000208 | F-OVARC1000208 | 0.4 | Yes | HRIFA010942a | 0.4 | Yes |
| PLACE1001536 | F-PLACE1001536 | 0.33 | Yes | HRIFA012761a | 0.31 | Yes |
| PLACE1003407 | F-PLACE1003407 | 0.48 | Yes | HRIFA013899a | 0.48 | Yes |
| PLACE1003428 | F-PLACE1003428 | 0.37 | Yes | HRIFA013911a | 0.61 | Yes |
| PLACE1003460 | F-PLACE1003460 | 0.38 | Yes | HRIFA013932a | 0.94 | Yes |
| PLACE1005569 | F-PLACE1005569 | 0.32 | Yes | HRIFA015351a | 0.68 | Yes |
| PLACE1006277 | F-PLACE1006277 | 0.32 | Yes | HRIFA015802a | 0.65 | Yes |
| PLACE1010251 | F-PLACE1010251 | 0.32 | Yes | HRIFA018238a | 0.62 | Yes |
| THYRO1000196 | F-THYRO1000196 | 0.44 | Yes | HRIFA029050a | 0.71 | Yes |
| THYRO1000795 | F-THYRO1000795 | 0.33 | Yes | HRIFA029327a | 0.92 | Yes |
| THYRO1000999 | F-THYRO1000999 | 0.4 | Yes | HRIFA030203a | 0.4 | Yes |
| THYRO1001478 | F-THYRO1001478 | 0.47 | Yes | HRIFA030385a | 0.89 | Yes |
| Y79AA1000426 | F-Y79AA1000426 | 0.47 | Yes | HRIFA027940a | 0.92 | Yes |

| | | | | | | |
|---|---|---|---|---|---|---|
| 41 clones from which selected clones have the maximal ATGpr1 score 0 or higher and less than 0.3, and predicted to be containing the signal sequence by the PSORT | | | | | | |

| name of clone | name of sequence | maximal | signal | representative | maximal | signal |
|---|---|---|---|---|---|---|
| | | ATGpr1 score | | Sequence | ATGpr1 score | |
| HEMBA1000300 | F-HEMBA1000300 | 0.13 | Yes | HRIFA000284a | 0.13 | Yes |
| HEMBA1002164 | F-HEMBA1002164 | 0.11 | Yes | HRIFA001972a | 0.74 | Yes |
| HEMBA1002239 | F-HEMBA1002239 | 0.17 | Yes | HRIFA002037a | 0.17 | Yes |
| HEMBA1002421 | F-HEMBA1002421 | 0.22 | Yes | HRIFA005392a | 0.9 | Yes |
| HEMBA1003294 | F-HEMBA1003294 | 0.15 | Yes | HRIFA020163a | 0.15 | Yes |
| HEMBA1006572 | F-HEMBA1006572 | 0.06 | Yes | HRIFA021543a | 0.62 | Yes |
| HEMBA1007013 | F-HEMBA1007013 | 0.19 | Yes | HRIFA021906a | 0.82 | Yes |
| HEMBB1000567 | F-HEMB81000567 | 0.09 | Yes | HRIFA029730a | 0.15 | Yes |
| HEMBB1002663 | F-HEMBB1002663 | 0.29 | Yes | HRIFA031871a | 0.29 | Yes |
| MAMMA1001043 | F-MAMMA1001043 | 0.17 | Yes | HRIFA026764a | 0.81 | Yes |
| MAMMA1001284 | F-MAMMA1001284 | 0.26 | Yes | HRIFA026889a | 0.26 | Yes |
| MAMMA1001901 | F-MAMMA1001901 | 0.17 | Yes | HRIFA027493a | 0.17 | Yes |
| MAMMA1002224 | F-MAMMA1002224 | 0.13 | Yes | HRIFA027717a | 0.13 | Yes |
| NT2RM2000306 | F-NT2RM2000306 | 0.25 | Yes | HRIFA021985a | 0.25 | Yes |
| NT2RM2000410 | F-NT2RM2000410 | 0.22 | Yes | HRIFA022055a | 0.82 | Yes |
| NT2RP2000479 | F-NT2RP2000479 | 0.24 | Yes | HRIFA000822a | 0.12 | Yes |
| NT2RP2001495 | F-NT2RP2001495 | 0.19 | Yes | HRIFA007228a | 0.78 | Yes |
| NT2RP2001948 | F-NT2RP2001948 | 0.29 | Yes | HRIFA007565a | 0.89 | Yes |
| NT2RP3000645 | F-NT2RP3000645 | 0.2 | Yes | HRIFA022890a | 0.91 | Yes |
| NT2RP3003076 | F-NT2RP3003076 | 0.23 | Yes | HRIFA024978a | 0.65 | Yes |
| NT2RP4001879 | F-NT2RP4001879 | 0.26 | Yes | HRIFA017818a | 0.79 | Yes |
| NT2RP4002451 | F-NT2RP4002451 | 0.11 | Yes | HRIFA018447a | 0.34 | Yes |
| OVARC1000439 | F-OVARC1000439 | 0.15 | Yes | HRIFA011105a | 0.21 | Yes |
| OVARC1001727 | F-OVARC1001727 | 0.22 | Yes | HRIFA019960a | 0.22 | Yes |
| PLACE1002080 | F-PLACE1002080 | 0.17 | Yes | HRIFA013092a | 0.76 | Yes |
| PLACE1002095 | F-PLACE1002095 | 0.23 | Yes | HRIFA013103a | 0.61 | Yes |
| PLACE1004028 | F-PLACE1004028 | 0.12 | Yes | HRIFA014303a | 0.12 | Yes |
| PLACE1004482 | F-PLACE1004482 | 0.13 | Yes | HRIFA014590a | 0.57 | Yes |
| PLACE1005383 | F-PLACE1005383 | 0.11 | Yes | HRIFA015219a | 0.52 | Yes |
| PLACE1005544 | F-PLACE1005544 | 0.08 | Yes | HRIFA009852a | 0.41 | Yes |
| PLACE1005660 | F-PLACE1005660 | 0.2 | Yes | HRIFA015409a | 0.2 | Yes |
| PLACE1006443 | F-PLACE1006443 | 0.27 | Yes | HRIFA015902a | 0.89 | Yes |
| PLACE1007296 | F-PLACE1007296 | 0.22 | Yes | HRIFA016430a | 0.27 | Yes |
| PLACE1008469 | F-PLACE1008469 | 0.27 | Yes | HRIFA017146a | 0.94 | Yes |
| PLACE1008984 | F-PLACE1008984 | 0.11 | Yes | HRIFA017456a | 0.74 | Yes |
| PLACE4000455 | F-PLACE4000455 | 0.23 | Yes | HRIFA012333a | 0.51 | Yes |
| SKNMC1000014 | F-SKNMC1000014 | 0.15 | Yes | HRIFA030106a | 0.76 | Yes |
| THYRO1001702 | F-THYRO1001702 | 0.14 | Yes | HRIFA030511a | 0.8 | Yes |
| Y79AA1000270 | F-Y79AA1000270 | 0.21 | Yes | HRIFA005644a | 0.63 | Yes |
| Y79AA1001056 | F-Y79AA1001056 | 0.27 | Yes | HRIFA028497a | 0.27 | Yes |
| Y79AA1001803 | F-Y79AA1001803 | 0.08 | Yes | HRIFA032073a | 0.68 | Yes |

| | | | | | | |
|---|---|---|---|---|---|---|
| Four clones from which selected clones have the maximal ATGpr1 score 0.5 or higher, and predicted to be lacking the signal sequence by the PSORT | | | | | | |

| name of clone | name of sequence | maximal | signal | name of representative | maximal | signal |
|---|---|---|---|---|---|---|
| | | ATGpr1 score | | sequence | ATGpr1 score | |
| NT2RP3002281 | F-NT2RP3002281 | 0.81 | No | HRIFA012999a | 0.61 | Yes |
| NT2RP3002721 | F-NT2RP3002721 | 0.94 | No | HRIFA023305a | 0.57 | Yes |
| NT2RP3004083 | F-NT2RP3004083 | 0.94 | No | HRIFA008387a | 0.76 | Yes |
| PLACE1005669 | F-PLACE1005669 | 0.94 | No | HRIFA012513a | 0.65 | Yes |

| | | | | | | |
|---|---|---|---|---|---|---|
| Two clones from which selected clones have the maximal ATGpr1 score 0.3 or higher and less than 0.5 and predicted to have no signal sequence by the PSORT | | | | | | |

| name of clone | name of sequence | maximal | signal | representative | maximal | signal |
|---|---|---|---|---|---|---|
| | | ATGpr1 score | | sequence | ATGpr1 score | |
| NT2RP3000481 | F-NT2RP3000481 | 0.47 | No | HRIFA028614a | 0.93 | Yes |
| NT2RP3003559 | F-NT2RP3003559 | 0.48 | No | HRIFA025514a | 0.45 | Yes |

| | | | | | | |
|---|---|---|---|---|---|---|
| Two clones from which selected clones have the maximal ATGpr1 score 0 or higher and less than 0.3, and predicted to have no signal sequence by the PSORT | | | | | | |

| name of clone | name of sequence | maximal | signal | representative | maximal | signal |
|---|---|---|---|---|---|---|
| | | ATGpr1 score | | sequence | ATGpr1 score | |
| PLACE1005601 | F-PLACE1005601 | 0.12 | No | HRIFA010593a | 0.64 | Yes |
| PLACE1006786 | F-PLACE1006786 | 0.22 | No | HRIFA012333a | 0.51 | Yes |

### EXAMPLE 12

A method for the selection of clones based on the top hit data in the homology search against the SwissProt (a method for the selection of a novel full-length protein that is predicted to have a function based on the top hit data).

The representative sequences of the clusters were discarded in which the 5'-end sequence is identical (90% or more matching), or not similar (the compared part contains a sequence of 25 nucleotides or shorter and the similarity is lower than 60%) to the top hit data in the SwissProt. Then, the remaining representative sequences which has similarity to the representative sequences of the 5'-ends were classified by a group of the above keywords (some representative sequences belong to a group by multiple keywords), and then clones were selected from the clusters. The names and the corresponding SEQ IDs of the representative sequences, and also the names of the introns (including the representative sequence of the 5'-end or the 3'-end, or ESTs) used for selecting the clones from the representative sequences and the corresponding SEQ IDs are shown in the last part of the present specification (Table 368 and 369, respectively). HRIFA indicates the representative sequence of the 5'-end group, and HRIRA indicates the representative sequence of the 3'-end group.

In principle, from the clusters containing only a single clone (the 5'-end sequence is the representative sequence of the cluster), the clone was selected. However, in the following cases, the clone containing a longer 5'-end was selected: where the maximal ATGpr1 score was less than 0.5, the 5'-end sequence of the clone to be selected was not complete, or the 5'-end of the clone was found to be obviously short nevertheless the clone should not be included in the same cluster based on the BLAST analysis between the other clones, or further, the 5'-end sequence of the said clone, which corresponds to the 3'-ends of the other clones belonging to the same cluster in which the 3'-end of the said clone was included, was turn out to be longer than those of the other clones by assembling them. When there were two clones in the same cluster, judged to be full-length, and it was difficult to determine which clone has the longer 5'-end even by assembling them, all the clones were selected. As a result, the ATGpr1 score in some clones became less than 0.5 or less than 0.3. The fullness ratio of these clones became lower, but there is still a possibility that the clones are full-length.

In the case in which multiple clones were contained in a cluster, selected was the clone having the longest 5'-end in the clones judged to be full-length compared to the human mRNA or human EST. However, in the following cases, the clone containing a longer 5'-end was selected: where the maximal ATGpr1 score was less than 0.5, the 5'-end sequence of the clone to be selected was not complete, or the 5'-end of the clone was found to be obviously short nevertheless the clone should not be included in the same cluster based on the BLAST analysis between the other clones, or further, the 5'-end sequence of the said clone, which corresponds to the 3'-ends of the other clones belonging to the same cluster in which the 3'-end of the said clone was included, was turn out to be longer than those of the other clones by assembling them. When there were two clones in the same cluster, judged to be full-length, and it was difficult to determine which clone has the longer 5'-end even by assembling them, all the clones were selected. As a result, the ATGpr1 score in some clones became less than 0.5 or less than 0.3. These clones can still be full-length.

Based on the top hit data in the SwissProt homology search, 658 clones were selected. Among them, 446 clones were selected by the keywords, secretion or membrane. Using the keyword, glycoprotein, 243 clones were selected. 51 clones were selected by the keywords for signal transduction. With the keywords for transcription, 130 clones were selected. 17 clones were selected by the keywords for disease.

Among the 446 clones selected by the keywords, secretion or membrane, 77 clones were overlapped with those selected by combining the ATGpr1 score and prediction by the PSORT for the existence of a signal sequence. Also, many clones were overlapped with those selected by the keyword, glycoprotein. Moreover, some clones were overlapped with the clones selected by the keywords for diseases.

Among the 243 clones selected by the keyword, glycoprotein, 53 clones were overlapped with those selected by combining the ATGpr1 score and prediction by the PSORT for the existence of a signal sequence. Also, many clones were overlapped with those selected by the keywords, secretion or membrane. Moreover, some clones were overlapped with the clones selected by the keywords in diseases.

Among the clones selected by the top hit data in the homology search on the SwissProt, 532 clones were having the maximal ATGpr1 score 0.5 or higher. 59 clones were having the maximal score 0.3 or higher and less than 0.5. 67 clones were with the maximal score less than 0.3.

When the maximal ATGpr1 score is 0.5 or higher, 0.3 or higher, no less than 0, the expected fullness ratio is as shown in Table 3, 4, 5, and 6, respectively.

| | |
|---|---|
| The representative sequences of the most homologous sequences in the SwissProt with the keyword(s) "growth factor", "cytokine", or "hormone", and the selected clones. | |

| name of clone | name of representative sequence |
|---|---|
| HEMBA1001563 | HRIFA001439a |
| HEMBA1003047 | HRIFA002743a |
| HEMBA1005070 | HRIFA020144a |
| HEMBA1006724 | HRIFA021620a |
| HEMBA1006916 | HRIFA021855a |
| MAMMA1001066 | HRIFA027355a |
| MAMMA1001634 | HRIFA027187a |
| MAMMA1002165 | HRIFA027673a |
| NT2RM4000326 | HRIFA032530a |
| NT2RM4001377 | HRIFA005300a |
| NT2RP2000447 | HRIFA006448a |
| NT2RP2000663 | HRIFA006609a |
| NT2RP2000903 | HRIFA006798a |
| NT2RP2002974 | HRIFA027860a |
| NT2RP2003369 | HRIFA008596a |
| NT2RP2004141 | HRIFA009123a |
| NT2RP2005941 | HRIFA010361a |
| NT2RP2006099 | HRIFA010466a |
| NT2RP3000645 | HRIFA022890a |
| NT2RP3000838 | HRIFA005300a |
| NT2RP4002451 | HRIFA018447a |
| OVARC1000275 | HRIFA010988a |
| OVARC1001030 | HRIFA021061a |
| PLACE1004492 | HRIFA014598a |
| PLACE1009279 | HRIFA017643a |
| THYRO1001071 | HRIFA029440a |
| Y79AA1000207 | HRIFA027867a |
| Y79AA1000426 | HRIFA027940a |

| | |
|---|---|
| The representative sequences of the most homologous sequences in the SwissProt with the keyword(s) "receptor", "G-protein coupled receptor", "ionic channel", "voltage-gated channel", or "calcium channel", and the selected clones. | |

| name of clone | name of representative sequence |
|---|---|
| BNGH41000091 | HRIFA029511a |
| HEMBA1001621 | HRIFA001489a |
| HEMBA1003392 | HRIFA002980a |
| HEMBA1005545 | HRIFA020272a |
| HEMBA1007291 | HRIFA022462a |
| HEMBA1007332 | HRIFA022493a |
| MAMMA1000681 | HRIFA026364a |
| MAMMA1000706 | HRIFA026382a |
| MAMMA1001978 | HRIFA027549a |
| NT2RM2001939 | HRIFA032009a |
| NT2RM2001941 | HRIFA032011a |
| NT2RM4002352 | HRIFA001337a |
| NT2RP2002510 | HRIFA007985a |
| NT2RP2002533 | HRIFA008000a |
| NT2RP2005181 | HRIFA005409a |
| NT2RP3000304 | HRIFA022616a |
| NT2RP3001542 | HRIFA028501a |
| NT2RP3002409 | HRIFA024197a |
| NT2RP3002836 | HRIFA024392a |
| NT2RP3003000 | HRIFA024767a |
| NT2RP3004552 | HRIFA025904a |
| NT2RP4001877 | HRIFA032433a |
| NT2RP4002750 | HRIFA028157a |
| OVARC1000090 | HRIFA010859a |
| OVARC1000956 | HRIFA011484a |
| OVARC1001991 | HRIFA019498a |
| PLACE1001016 | HRIFA012354a |
| PLACE1001340 | HRIFA012584a |
| PLACE1001401 | HRIFA012625a |
| PLACE1001564 | HRIFA012737a |
| PLACE1001655 | HRIFA012795a |
| PLACE1002547 | HRIFA013376a |
| PLACE1002967 | HRIFA013620a |
| PLACE1003573 | HRIFA014006a |
| PLACE1003852 | HRIFA014185a |
| PLACE1004441 | HRIFA014561a |
| PLACE1005031 | HRIFA014967a |
| PLACE1005878 | HRIFA015536a |
| PLACE1007296 | HRIFA016430a |
| PLACE1008469 | HRIFA017146a |
| PLACE1010784 | HRIFA031126a |
| PLACE1010968 | HRIFA018666a |
| THYRO1000956 | HRIFA029393a |
| Y79AA1001062 | HRIFA023434a |

| | |
|---|---|
| The representative sequences of the most homologous sequenses in the SwissProt with the keyword(s) "extracellular matrix", and the selected clones. | |

| name of clone | name of representative sequence |
|---|---|
| HEMBA1000006 | HRIFA027327a |
| HEMBA1000275 | HRIFA000264a |
| HEMBA1000835 | HRIFA000776a |
| HEMBA1000907 | HRIFA000845a |
| HEMBA1002164 | HRIFA001972a |
| HEMBA1003101 | HRIFA002787a |
| HEMBA1003230 | HRIFA002891a |
| MAMMA1000403 | HRIFA026465a |
| MAMMA1001615 | HRIFA022865a |
| MAMMA1001893 | HRIFA027485a |
| MAMMA1002128 | HRIFA027644a |
| NT2RM4000284 | HRIFA032506a |
| NT2RM4000295 | HRIFA032511a |
| NT2RM4000587 | HRIFA032696a |
| NT2RP2000616 | HRIFA006572a |
| NT2RP2000694 | HRIFA006633a |
| NT2RP2001562 | HRIFA010799a |
| NT2RP2001948 | HRIFA007565a |
| NT2RP2002409 | HRIFA007909a |
| NT2RP2004447 | HRIFA009339a |
| NT2RP2004847 | HRIFA005944a |
| NT2RP2006004 | HRIFA002766a |
| NT2RP3000059 | HRIFA022203a |
| NT2RP3000616 | HRIFA022875a |
| NT2RP3000871 | HRIFA023048a |
| NT2RP3000921 | HRIFA023069a |
| NT2RP3002015 | HRIFA015995a |
| NT2RP3002448 | HRIFA024218a |
| NT2RP3002983 | HRIFA024473a |
| NT2RP3003729 | HRIFA025488a |
| NT2RP3004067 | HRIFA027327a |
| OVARC1001049 | HRIFA022702a |
| OVARC1001222 | HRIFA022714a |
| OVARC1002058 | HRIFA022737a |
| PLACE1001114 | HRIFA012427a |
| PLACE1002329 | HRIFA013235a |
| PLACE1004816 | HRIFA014819a |
| PLACE1005383 | HRIFA015219a |
| PLACE1005569 | HRIFA015351a |
| PLACE1006073 | HRIFA003402a |
| PLACE1006277 | HRIFA015802a |
| PLACE1008984 | HRIFA017456a |
| THYRO1001102 | HRIFA008174a |
| THYRO1001471 | HRIFA030381a |
| THYRO1001478 | HRIFA030385a |
| Y79AA1000888 | HRIFA028440a |

| | |
|---|---|
| The representative sequences of the most homologous sequences in the SwissProt with the keyword(s) "transmembrane", and the selected clones (except overlapped with Table 16, 17, and 18) | |

| name of clone | name of representative sequence |
|---|---|
| BNGH41000020 | HRIFA030662a |
| HEMBA1000121 | HRIFA000116a |
| HEMBA1000349 | HRIFA000327a |
| HEMBA1000477 | HRIFA000446a |
| HEMBA1000940 | HRIFA002384a |
| HEMBA1002163 | HRIFA001971a |
| HEMBA1002421 | HRIFA005392a |
| HEMBA1002767 | HRIFA002503a |
| HEMBA1003945 | HRIFA009220a |
| HEMBA1004250 | HRIFA003504a |
| HEMBA1004391 | HRIFA020693a |
| HEMBA1004444 | HRIFA029285a |
| HEMBA1004454 | HRIFA003592a |
| HEMBA1004505 | HRIFA003635a |
| HEMBA1004797 | HRIFA020883a |
| HEMBA1004982 | HRIFA003892a |
| HEMBA1005489 | HRIFA024543a |
| HEMBA1005698 | HRIFA020453a |
| HEMBA1005945 | HRIFA020349a |
| HEMBA1006299 | HRIFA025771a |
| HEMBA1006430 | HRIFA021213a |
| HEMBA1006482 | HRIFA022328a |
| HEMBA1007241 | HRIFA022423a |
| HEMBB1000679 | HRIFA029802a |
| HEMBB1001200 | HRIFA030839a |
| HEMBB1001573 | HRIFA031091a |
| HEMBB1002427 | HRIFA005760a |
| MAMMA1000204 | HRIFA025966a |
| MAMMA1000473 | HRIFA018870a |
| MAMMA1000496 | HRIFA026242a |
| MAMMA1000788 | HRIFA018287a |
| MAMMA1000814 | HRIFA026618a |
| MAMMA1001237 | HRIFA026860a |
| MAMMA1001418 | HRIFA027045a |
| MAMMA1002091 | HRIFA027622a |
| MAMMA1002095 | HRIFA027625a |
| MAMMA1002586 | HRIFA027012a |
| NT2RM1000580 | HRIFA004523a |
| NT2RM1000855 | HRIFA004696a |
| NT2RM1000858 | HRIFA004714a |
| NT2RM1000899 | HRIFA004745a |
| NT2RM2000565 | HRIFA022139a |
| NT2RM2000582 | HRIFA021787a |
| NT2RM2001126 | HRIFA024088a |
| NT2RM4000198 | HRIFA032453a |
| NT2RM4000417 | HRIFA032587a |
| NT2RM4000444 | HRIFA032605a |
| NT2RM4000593 | HRIFA023489a |
| NT2RM4000761 | HRIFA023923a |
| NT2RM4000965 | HRIFA030103a |
| NT2RM4001735 | HRIFA002063a |
| NT2RP1000181 | HRIFA005184a |
| NT2RP1000261 | HRIFA005231a |
| NT2RP1000300 | HRIFA005255a |
| NT2RP1000325 | HRIFA005271a |
| NT2RP1000551 | HRIFA005420a |
| NT2RP1000981 | HRIFA005702a |
| NT2RP2000533 | HRIFA006510a |
| NT2RP2000649 | HRIFA006596a |
| NT2RP2000818 | HRIFA006730a |
| NT2RP2001200 | HRIFA007013a |
| NT2RP2001495 | HRIFA007228a |
| NT2RP2001514 | HRIFA007243a |
| NT2RP2001956 | HRIFA007571a |
| NT2RP2002063 | HRIFA007659a |
| NT2RP2002232 | HRIFA009783a |
| NT2RP2002527 | HRIFA023767a |
| NT2RP2002942 | HRIFA008284a |
| NT2RP2002976 | HRIFA008314a |
| NT2RP2003210 | HRIFA008483a |
| NT2RP2003390 | HRIFA008611a |
| NT2RP2003469 | HRIFA008661a |
| NT2RP2003655 | HRIFA008784a |
| NT2RP2003664 | HRIFA008790a |
| NT2RP2004205 | HRIFA009171a |
| NT2RP2004794 | HRIFA009578a |
| NT2RP2005425 | HRIFA010005a |
| NT2RP2005597 | HRIFA010130a |
| NT2RP2005632 | HRIFA010152a |
| NT2RP2005994 | HRIFA010394a |
| NT2RP2006269 | HRIFA025913a |
| NT2RP2006512 | HRIFA024937a |
| NT2RP3000125 | HRIFA022234a |
| NT2RP3000171 | HRIFA007722a |
| NT2RP3000676 | HRIFA026576a |
| NT2RP3000907 | HRIFA025046a |
| NT2RP3001061 | HRIFA023154a |
| NT2RP3001170 | HRIFA010078a |
| NT2RP3001195 | HRIFA023227a |
| NT2RP3001240 | HRIFA023257a |
| NT2RP3001322 | HRIFA023304a |
| NT2RP3001388 | HRIFA006926a |
| NT2RP3001560 | HRIFA030599a |
| NT2RP3001738 | HRIFA025766a |
| NT2RP3002160 | HRIFA005760a |
| NT2RP3002324 | HRIFA024884a |
| NT2RP3002342 | HRIFA006586a |
| NT2RP3002571 | HRIFA024255a |
| NT2RP3002900 | HRIFA024423a |
| NT2RP3002958 | HRIFA017670a |
| NT2RP3003532 | HRIFA013477a |
| NT2RP3003939 | HRIFA025636a |
| NT2RP3004130 | HRIFA025703a |
| NT2RP3004133 | HRIFA025706a |
| NT2RP3004294 | HRIFA025771a |
| NT2RP3004406 | HRIFA025800a |
| NT2RP3004481 | HRIFA026089a |
| NT2RP3004625 | HRIFA026564a |
| NT2RP3004647 | HRIFA026576a |
| NT2RP4001009 | HRIFA022227a |
| NT2RP4001879 | HRIFA017818a |
| OVARC1000003 | HRIFA010790a |
| OVARC1000105 | HRIFA007493a |
| OVARC1000137 | HRIFA010891a |
| OVARC1000307 | HRIFA002919a |
| OVARC1000331 | HRIFA004162a |
| OVARC1000553 | HRIFA011197a |
| OVARC1000873 | HRIFA032478a |
| OVARC1001163 | HRIFA032079a |
| OVARC1001260 | HRIFA019867a |
| OVARC1001336 | HRIFA019867a |
| OVARC1001607 | HRIFA022729a |
| PLACE1000740 | HRIFA012151a |
| PLACE1001123 | HRIFA012436a |
| PLACE1001231 | HRIFA012515a |
| PLACE1001836 | HRIFA012914a |
| PLACE1001949 | HRIFA012990a |
| PLACE1002095 | HRIFA013103a |
| PLACE1002905 | HRIFA013586a |
| PLACE1002911 | HRIFA013589a |
| PLACE1003163 | HRIFA013744a |
| PLACE1003644 | HRIFA014056a |
| PLACE1003737 | HRIFA014111a |
| PLACE1004279 | HRIFA014465a |
| PLACE1004450 | HRIFA014568a |
| PLACE1004482 | HRIFA014590a |
| PLACE1004630 | HRIFA014688a |
| PLACE1005544 | HRIFA009852a |
| PLACE1005745 | HRIFA017855a |
| PLACE1005927 | HRIFA015568a |
| PLACE1006290 | HRIFA015811a |
| PLACE1007096 | HRIFA012167a |
| PLACE1007845 | HRIFA016758a |
| PLACE1007881 | HRIFA016240a |
| PLACE1008359 | HRIFA015547a |
| PLACE1008716 | HRIFA017295a |
| PLACE1008985 | HRIFA017457a |
| PLACE1009600 | HRIFA017836a |
| PLACE1010011 | HRIFA018092a |
| PLACE1010078 | HRIFA018131a |
| PLACE1010251 | HRIFA018238a |
| PLACE1010445 | HRIFA010736a |
| PLACE1010827 | HRIFA018580a |
| PLACE1011045 | HRIFA014500a |
| PLACE1011181 | HRIFA018794a |
| PLACE1011236 | HRIFA018827a |
| PLACE1011516 | HRIFA018993a |
| PLACE3000181 | HRIFA004112a |
| SKNMC1000082 | HRIFA030147a |
| THYRO1000196 | HRIFA029050a |
| THYRO1000400 | HRIFA000564a |
| THYRO1000584 | HRIFA029209a |
| THYRO1000678 | HRIFA029256a |
| THYRO1000776 | HRIFA029317a |
| THYRO1000795 | HRIFA029327a |
| THYRO1000866 | HRIFA027714a |
| THYRO1001113 | HRIFA029460a |
| THYRO1001128 | HRIFA029467a |
| THYRO1001242 | HRIFA032360a |
| THYRO1001266 | HRIFA030264a |
| THYRO1001456 | HRIFA030370a |
| THYRO1001529 | HRIFA030411a |
| THYRO1001702 | HRIFA030511a |
| Y79AA1000127 | HRIFA026121a |
| Y79AA1000270 | HRIFA005644a |
| Y79AA1001426 | HRIFA028651a |
| Y79AA1001787 | HRIFA028790a |
| Y79AA1001799 | HRIFA032070a |
| Y79AA1002213 | HRIFA032224a |

| | |
|---|---|
| The representative sequences of the most homologous sequences in the SwissProt with the keyword(s) "signal", and the selected clones (except overlapped with Table 16, 17, 18 and 19) | |

| name of clone | name of representative sequence |
|---|---|
| BNGH41000087 | HRIFA029508a |
| HEMBA1000128 | HRIFA000123a |
| HEMBA1000443 | HRIFA000415a |
| HEMBA1000590 | HRIFA000553a |
| HEMBA1000634 | HRIFA004780a |
| HEMBA1000745 | HRIFA000695a |
| HEMBA1001221 | HRIFA001132a |
| HEMBA1001228 | HRIFA001138a |
| HEMBA1001390 | HRIFA000071a |
| HEMBA1002131 | HRIFA001942a |
| HEMBA1002167 | HRIFA001975a |
| HEMBA1002178 | HRIFA001984a |
| HEMBA1002524 | HRIFA002284a |
| HEMBA1002992 | HRIFA002694a |
| HEMBA1003072 | HRIFA002762a |
| HEMBA1003315 | HRIFA000016a |
| HEMBA1003487 | HRIFA003055a |
| HEMBA1003530 | HRIFA003093a |
| HEMBA1005145 | HRIFA003946a |
| HEMBA1005337 | HRIFA019651a |
| HEMBA1005449 | HRIFA020707a |
| HEMBA1005522 | HRIFA021398a |
| HEMBA1006335 | HRIFA012069a |
| HEMBA1006572 | HRIFA021543a |
| HEMBA1006707 | HRIFA021499a |
| HEMBA1006749 | HRIFA021637a |
| HEMBA1006902 | HRIFA021754a |
| HEMBA1007013 | HRIFA021906a |
| HEMBA1007057 | HRIFA022985a |
| HEMBB1000447 | HRIFA001558a |
| HEMBB1000567 | HRIFA029730a |
| HEMBB1000881 | HRIFA029932a |
| HEMBB1001026 | HRIFA030025a |
| HEMBB1001048 | HRIFA030045a |
| HEMBB1001847 | HRIFA031249a |
| MAMMA1000106 | HRIFA024482a |
| MAMMA1000226 | HRIFA025978a |
| MAMMA1000591 | HRIFA026303a |
| MAMMA1001043 | HRIFA026764a |
| MAMMA1001957 | HRIFA027536a |
| MAMMA1002080 | HRIFA016963a |
| MAMMA1002234 | HRIFA027722a |
| MAMMA1002633 | HRIFA030461a |
| MAMMA1003126 | HRIFA029263a |
| NT2RM1000462 | HRIFA004426a |
| NT2RM1000542 | HRIFA004490a |
| NT2RM2000410 | HRIFA022055a |
| NT2RM2000423 | HRIFA022065a |
| NT2RM2000622 | HRIFA022156a |
| NT2RM2000773 | HRIFA023894a |
| NT2RM2001626 | HRIFA028911a |
| NT2RM2001818 | HRIFA031935a |
| NT2RM4000648 | HRIFA032730a |
| NT2RM4001843 | HRIFA024718a |
| NT2RP1000050 | HRIFA005102a |
| NT2RP1001004 | HRIFA005720a |
| NT2RP2000394 | HRIFA003640a |
| NT2RP2000514 | HRIFA006494a |
| NT2RP2001480 | HRIFA007219a |
| NT2RP2001755 | HRIFA007424a |
| NT2RP2001878 | HRIFA007512a |
| NT2RP2002188 | HRIFA007745a |
| NT2RP2002564 | HRIFA007244a |
| NT2RP2002824 | HRIFA008200a |
| NT2RP2003042 | HRIFA008362a |
| NT2RP2003593 | HRIFA008252a |
| NT2RP2003931 | HRIFA008976a |
| NT2RP2004606 | HRIFA009451a |
| NT2RP2004648 | HRIFA009482a |
| NT2RP2005163 | HRIFA009825a |
| NT2RP2005247 | HRIFA009881a |
| NT2RP2005378 | HRIFA004919a |
| NT2RP2005541 | HRIFA010090a |
| NT2RP2005883 | HRIFA010319a |
| NT2RP2006042 | HRIFA010425a |
| NT2RP3000063 | HRIFA022528a |
| NT2RP3000436 | HRIFA022776a |
| NT2RP3000444 | HRIFA022782a |
| NT2RP3000481 | HRIFA028614a |
| NT2RP3000721 | HRIFA009825a |
| NT2RP3001012 | HRIFA023129a |
| NT2RP3001159 | HRIFA023212a |
| NT2RP3001592 | HRIFA023464a |
| NT2RP3001754 | HRIFA007728a |
| NT2RP3002311 | HRIFA024718a |
| NT2RP3002738 | HRIFA020748a |
| NT2RP3002790 | HRIFA026519a |
| NT2RP3002887 | HRIFA029278a |
| NT2RP3003354 | HRIFA008212a |
| NT2RP3003448 | HRIFA025479a |
| NT2RP3003473 | HRIFA001413a |
| NT2RP3003614 | HRIFA032642a |
| NT2RP3004075 | HRIFA010301a |
| NT2RP3004090 | HRIFA027329a |
| NT2RP3004202 | HRIFA025327a |
| NT2RP3004309 | HRIFA025778a |
| NT2RP3004345 | HRIFA025353a |
| NT2RP3004557 | HRIFA025907a |
| NT2RP4001467 | HRIFA013276a |
| OVARC1000313 | HRIFA011016a |
| OVARC1000410 | HRIFA022691a |
| OVARC1000439 | HRIFA011105a |
| OVARC1001086 | HRIFA011580a |
| OVARC1001569 | HRIFA022728a |
| OVARC1001570 | HRIFA019412a |
| PLACE1001407 | HRIFA012069a |
| PLACE1001464 | HRIFA013276a |
| PLACE1001516 | HRIFA012702a |
| PLACE1001795 | HRIFA012885a |
| PLACE1001918 | HRIFA012969a |
| PLACE1002080 | HRIFA013092a |
| PLACE1002153 | HRIFA013135a |
| PLACE1002355 | HRIFA013254a |
| PLACE1002374 | HRIFA013265a |
| PLACE1002726 | HRIFA018688a |
| PLACE1003428 | HRIFA013911a |
| PLACE1003460 | HRIFA013932a |
| PLACE1003772 | HRIFA014133a |
| PLACE1004078 | HRIFA014336a |
| PLACE1004520 | HRIFA014621a |
| PLACE1004648 | HRIFA014702a |
| PLACE1004887 | HRIFA014868a |
| PLACE1005426 | HRIFA015246a |
| PLACE1006071 | HRIFA016639a |
| PLACE1006443 | HRIFA015902a |
| PLACE1006716 | HRIFA016070a |
| PLACE1006959 | HRIFA016214a |
| PLACE1007077 | HRIFA016639a |
| PLACE1007081 | HRIFA016290a |
| PLACE1007702 | HRIFA016669a |
| PLACE1008657 | HRIFA017257a |
| PLACE1008744 | HRIFA017312a |
| PLACE1009546 | HRIFA017801a |
| PLACE1011116 | HRIFA018754a |
| PLACE1011708 | HRIFA019105a |
| PLACE2000118 | HRIFA024994a |
| PLACE3000213 | HRIFA015486a |
| PLACE4000354 | HRIFA015486a |
| THYRO1000061 | HRIFA013279a |
| THYRO1000846 | HRIFA029349a |
| THYRO1001063 | HRIFA029434a |
| THYRO1001608 | HRIFA030456a |
| THYRO1001803 | HRIFA030566a |
| Y79AA1000876 | HRIFA030629a |
| Y79AA1001090 | HRIFA028511a |
| Y79AA1001272 | HRIFA028576a |
| Y79AA1001727 | HRIFA006642a |
| Y79AA1001803 | HRIFA032073a |
| Y79AA1002376 | HRIFA032820a |

| | |
|---|---|
| The representative sequences of the most homologous sequences in the SwissProt with the keyword(s) "glycoprotein", and the selected clones | |

| name of clone | name of representative sequence |
|---|---|
| BNGH41000087 | HRIFA029508a |
| BNGH41000091 | HRIFA029511a |
| HEMBA1000275 | HRIFA000264a |
| HEMBA1000349 | HRIFA000327a |
| HEMBA1000590 | HRIFA000553a |
| HEMBA1000634 | HRIFA004780a |
| HEMBA1000835 | HRIFA000776a |
| HEMBA1000907 | HRIFA000845a |
| HEMBA1001221 | HRIFA001132a |
| HEMBA1001228 | HRIFA001138a |
| HEMBA1001621 | HRIFA001489a |
| HEMBA1002131 | HRIFA001942a |
| HEMBA1002164 | HRIFA001972a |
| HEMBA1002167 | HRIFA001975a |
| HEMBA1002178 | HRIFA001984a |
| HEMBA1002316 | HRIFA002102a |
| HEMBA1002421 | HRIFA005392a |
| HEMBA1002767 | HRIFA002503a |
| HEMBA1003047 | HRIFA002743a |
| HEMBA1003101 | HRIFA002787a |
| HEMBA1003230 | HRIFA002891a |
| HEMBA1003392 | HRIFA002980a |
| HEMBA1004250 | HRIFA003504a |
| HEMBA1004391 | HRIFA020693a |
| HEMBA1004444 | HRIFA029285a |
| HEMBA1004454 | HRIFA003592a |
| HEMBA1004505 | HRIFA003635a |
| HEMBA1005449 | HRIFA020707a |
| HEMBA1005489 | HRIFA024543a |
| HEMBA1005522 | HRIFA021398a |
| HEMBA1005545 | HRIFA020272a |
| HEMBA1006335 | HRIFA012069a |
| HEMBA1006572 | HRIFA021543a |
| HEMBA1006707 | HRIFA021499a |
| HEMBA1006724 | HRIFA021620a |
| HEMBA1006749 | HRIFA021637a |
| HEMBA1006902 | HRIFA021754a |
| HEMBA1007057 | HRIFA022985a |
| HEMBA1007332 | HRIFA022493a |
| HEMBB1000447 | HRIFA001558a |
| HEMBB1000567 | HRIFA029730a |
| HEMBB1000679 | HRIFA029802a |
| HEMBB1000881 | HRIFA029932a |
| HEMBB1001048 | HRIFA030045a |
| HEMBB1002427 | HRIFA005760a |
| MAMMA1000106 | HRIFA024482a |
| MAMMA1000403 | HRIFA026465a |
| MAMMA1000591 | HRIFA026303a |
| MAMMA1000681 | HRIFA026364a |
| MAMMA1000706 | HRIFA026382a |
| MAMMA1001043 | HRIFA026764a |
| MAMMA1001237 | HRIFA026860a |
| MAMMA1001615 | HRIFA022865a |
| MAMMA1001893 | HRIFA027485a |
| MAMMA1001978 | HRIFA027549a |
| MAMMA1002070 | HRIFA028371a |
| MAMMA1002091 | HRIFA027622a |
| MAMMA1002128 | HRIFA027644a |
| MAMMA1002586 | HRIFA027012a |
| MAMMA1003126 | HRIFA029263a |
| NT2RM1000462 | HRIFA004426a |
| NT2RM1000542 | HRIFA004490a |
| NT2RM1000580 | HRIFA004523a |
| NT2RM2000423 | HRIFA022065a |
| NT2RM2001626 | HRIFA028911a |
| NT2RM2001792 | HRIFA029002a |
| NT2RM2001818 | HRIFA031935a |
| NT2RM2001939 | HRIFA032009a |
| NT2RM2001941 | HRIFA032011a |
| NT2RM4000198 | HRIFA032453a |
| NT2RM4000284 | HRIFA032506a |
| NT2RM4000417 | HRIFA032587a |
| NT2RM4000587 | HRIFA032696a |
| NT2RM4000648 | HRIFA032730a |
| NT2RM4001843 | HRIFA024718a |
| NT2RM4002352 | HRIFA001337a |
| NT2RP1000002 | HRIFA005072a |
| NT2RP1000050 | HRIFA005102a |
| NT2RP1000613 | HRIFA005462a |
| NT2RP1000981 | HRIFA005702a |
| NT2RP1001004 | HRIFA005720a |
| NT2RP2000394 | HRIFA003640a |
| NT2RP2000514 | HRIFA006494a |
| NT2RP2000616 | HRIFA006572a |
| NT2RP2001480 | HRIFA007219a |
| NT2RP2001562 | HRIFA010799a |
| NT2RP2001755 | HRIFA007424a |
| NT2RP2001878 | HRIFA007512a |
| NT2RP2002188 | HRIFA007745a |
| NT2RP2002304 | HRIFA007829a |
| NT2RP2002409 | HRIFA007909a |
| NT2RP2002510 | HRIFA007985a |
| NT2RP2002533 | HRIFA008000a |
| NT2RP2002564 | HRIFA007244a |
| NT2RP2002942 | HRIFA008284a |
| NT2RP2003042 | HRIFA008362a |
| NT2RP2003469 | HRIFA008661a |
| NT2RP2003931 | HRIFA008976a |
| NT2RP2004205 | HRIFA009171a |
| NT2RP2004447 | HRIFA009339a |
| NT2RP2004606 | HRIFA009451a |
| NT2RP2004648 | HRIFA009482a |
| NT2RP2004847 | HRIFA005944a |
| NT2RP2005181 | HRIFA005409a |
| NT2RP2005247 | HRIFA009881a |
| NT2RP2005541 | HRIFA010090a |
| NT2RP2005597 | HRIFA010130a |
| NT2RP2005632 | HRIFA010152a |
| NT2RP2005883 | HRIFA010319a |
| NT2RP2006004 | HRIFA002766a |
| NT2RP2006042 | HRIFA010425a |
| NT2RP2006269 | HRIFA025913a |
| NT2RP3000059 | HRIFA022203a |
| NT2RP3000063 | HRIFA022528a |
| NT2RP3000125 | HRIFA022234a |
| NT2RP3000304 | HRIFA022616a |
| NT2RP3000481 | HRIFA028614a |
| NT2RP3000616 | HRIFA022875a |
| NT2RP3000871 | HRIFA023048a |
| NT2RP3000921 | HRIFA023069a |
| NT2RP3001012 | HRIFA023129a |
| NT2RP3001061 | HRIFA023154a |
| NT2RP3001159 | HRIFA023212a |
| NT2RP3001542 | HRIFA028501a |
| NT2RP3001560 | HRIFA030599a |
| NT2RP3001754 | HRIFA007728a |
| NT2RP3002015 | HRIFA015995a |
| NT2RP3002160 | HRIFA005760a |
| NT2RP3002311 | HRIFA024718a |
| NT2RP3002448 | HRIFA024218a |
| NT2RP3002738 | HRIFA020748a |
| NT2RP3002836 | HRIFA024392a |
| NT2RP3002958 | HRIFA017670a |
| NT2RP3002983 | HRIFA024473a |
| NT2RP3003000 | HRIFA024767a |
| NT2RP3003076 | HRIFA024978a |
| NT2RP3003354 | HRIFA008212a |
| NT2RP3003532 | HRIFA013477a |
| NT2RP3003729 | HRIFA025488a |
| NT2RP3004130 | HRIFA025703a |
| NT2RP3004133 | HRIFA025706a |
| NT2RP3004309 | HRIFA025778a |
| NT2RP3004481 | HRIFA026089a |
| NT2RP3004552 | HRIFA025904a |
| NT2RP3004625 | HRIFA026564a |
| NT2RP3004640 | HRIFA030250a |
| NT2RP4000108 | HRIFA001341a |
| NT2RP4001467 | HRIFA013276a |
| NT2RP4001877 | HRIFA032433a |
| NT2RP4002750 | HRIFA028157a |
| OVARC1000003 | HRIFA010790a |
| OVARC1000090 | HRIFA010859a |
| OVARC1000313 | HRIFA011016a |
| OVARC1000467 | HRIFA011128a |
| OVARC1000553 | HRIFA011197a |
| OVARC1000873 | HRIFA032478a |
| OVARC1000956 | HRIFA011484a |
| OVARC1001049 | HRIFA022702a |
| OVARC1001086 | HRIFA011580a |
| OVARC1001260 | HRIFA019867a |
| OVARC1001336 | HRIFA019867a |
| OVARC1001569 | HRIFA022728a |
| OVARC1001570 | HRIFA019412a |
| OVARC1001607 | HRIFA022729a |
| OVARC1001991 | HRIFA019498a |
| OVARC1002058 | HRIFA022737a |
| PLACE1000740 | HRIFA012151a |
| PLACE1001016 | HRIFA012354a |
| PLACE1001114 | HRIFA012427a |
| PLACE1001123 | HRIFA012436a |
| PLACE1001231 | HRIFA012515a |
| PLACE1001407 | HRIFA012069a |
| PLACE1001464 | HRIFA013276a |
| PLACE1001516 | HRIFA012702a |
| PLACE1001564 | HRIFA012737a |
| PLACE1001655 | HRIFA012795a |
| PLACE1001836 | HRIFA012914a |
| PLACE1002095 | HRIFA013103a |
| PLACE1002329 | HRIFA013235a |
| PLACE1002355 | HRIFA013254a |
| PLACE1002374 | HRIFA013265a |
| PLACE1002905 | HRIFA013586a |
| PLACE1002911 | HRIFA013589a |
| PLACE1003163 | HRIFA013744a |
| PLACE1003428 | HRIFA013911a |
| PLACE1003438 | HRIFA013919a |
| PLACE1003573 | HRIFA014006a |
| PLACE1003737 | HRIFA014111a |
| PLACE1003852 | HRIFA014185a |
| PLACE1004441 | HRIFA014561a |
| PLACE1004450 | HRIFA014568a |
| PLACE1004520 | HRIFA014621a |
| PLACE1004630 | HRIFA014688a |
| PLACE1004816 | HRIFA014819a |
| PLACE1005003 | HRIFA014951a |
| PLACE1005383 | HRIFA015219a |
| PLACE1005426 | HRIFA015246a |
| PLACE1005539 | HRIFA029425a |
| PLACE1005544 | HRIFA009852a |
| PLACE1005569 | HRIFA015351a |
| PLACE1006071 | HRIFA016639a |
| PLACE1006073 | HRIFA003402a |
| PLACE1006277 | HRIFA015802a |
| PLACE1006290 | HRIFA015811a |
| PLACE1006443 | HRIFA015902a |
| PLACE1006716 | HRIFA016070a |
| PLACE1007077 | HRIFA016639a |
| PLACE1007081 | HRIFA016290a |
| PLACE1007845 | HRIFA016758a |
| PLACE1008469 | HRIFA017146a |
| PLACE1008716 | HRIFA017295a |
| PLACE1008744 | HRIFA017312a |
| PLACE1008984 | HRIFA017456a |
| PLACE1008985 | HRIFA017457a |
| PLACE1009527 | HRIFA017791a |
| PLACE1010251 | HRIFA018238a |
| PLACE1010784 | HRIFA031126a |
| PLACE1010968 | HRIFA018666a |
| PLACE1011116 | HRIFA018754a |
| PLACE1011708 | HRIFA019105a |
| PLACE2000118 | HRIFA024994a |
| PLACE3000181 | HRIFA004112a |
| PLACE3000213 | HRIFA015486a |
| PLACE4000354 | HRIFA015486a |
| SKNMC1000014 | HRIFA030106a |
| THYRO1000196 | HRIFA029050a |
| THYRO1000584 | HRIFA029209a |
| THYRO1000956 | HRIFA029393a |
| THYRO1001102 | HRIFA008174a |
| THYRO1001128 | HRIFA029467a |
| THYRO1001266 | HRIFA030264a |
| THYRO1001803 | HRIFA030566a |
| Y79AA1000127 | HRIFA026121a |
| Y79AA1000207 | HRIFA027867a |
| Y79AA1000270 | HRIFA005644a |
| Y79AA1000426 | HRIFA027940a |
| Y79AA1000888 | HRIFA028440a |
| Y79AA1001062 | HRIFA023434a |
| Y79AA1001272 | HRIFA028576a |
| Y79AA1001426 | HRIFA028651a |
| Y79AA1001523 | HRIFA030642a |
| Y79AA1001727 | HRIFA006642a |
| Y79AA1001863 | HRIFA032097a |

| | |
|---|---|
| The representative sequences of the most homologous sequences in the SwissProt with the keyword(s) "serine/threonine-protein kinase", "tyrosine-protein kinase", or "calmodulin-binding", and the selected clones | |

| name of clone | name of representative sequence |
|---|---|
| HEMBA1001878 | HRIFA001712a |
| HEMBA1002195 | HRIFA017703a |
| HEMBA1002227 | HRIFA019136a |
| HEMBA1002551 | HRIFA002309a |
| HEMBA1005084 | HRIFA020184a |
| HEMBA1005913 | HRIFA029866a |
| HEMBA1005929 | HRIFA020335a |
| HEMBB1000668 | HRIFA029792a |
| MAMMA1000881 | HRIFA026659a |
| MAMMA1001150 | HRIFA026813a |
| MAMMA1002142 | HRIFA027656a |
| NT2RM2000589 | HRIFA021794a |
| NT2RM2001902 | HRIFA031986a |
| NT2RP1001020 | HRIFA005728a |
| NT2RP1001031 | HRIFA005732a |
| NT2RP2001469 | HRIFA028061a |
| NT2RP2001529 | HRIFA007256a |
| NT2RP2001769 | HRIFA007435a |
| NT2RP2003179 | HRIFA008459a |
| NT2RP2003545 | HRIFA008717a |
| NT2RP2004670 | HRIFA028468a |
| NT2RP3000011 | HRIFA022177a |
| NT2RP3000022 | HRIFA022182a |
| NT2RP3000172 | HRIFA022265a |
| NT2RP3000201 | HRIFA022546a |
| NT2RP3000820 | HRIFA018262a |
| NT2RP3003527 | HRIFA025492a |
| NT2RP3003849 | HRIFA025250a |
| NT2RP3003874 | HRIFA025261a |
| NT2RP4000634 | HRIFA029866a |
| NT2RP4000962 | HRIFA027681a |
| OVARC1000255 | HRIFA010975a |
| OVARC1000529 | HRIFA011179a |
| OVARC1000916 | HRIFA011449a |
| OVARC1001338 | HRIFA019869a |
| PLACE1003135 | HRIFA013726a |
| PLACE1005519 | HRIFA015070a |
| PLACE1005736 | HRIFA015453a |
| PLACE1008282 | HRIFA016654a |
| PLACE1008297 | HRIFA017031a |
| PLACE1010081 | HRIFA018134a |
| PLACE1011364 | HRIFA018904a |
| PLACE1011824 | HRIFA019175a |
| THYRO1001205 | HRIFA030237a |
| THYRO1001457 | HRIFA030371a |
| THYRO1001593 | HRIFA030448a |
| THYRO1001700 | HRIFA030509a |
| THYRO1001770 | HRIFA030545a |
| Y79AA1000777 | HRIFA028402a |
| Y79AA1000967 | HRIFA028468a |
| Y79AA1002381 | HRIFA032275a |

| | |
|---|---|
| The representative sequences of the most homologous sequences in the SwissProt with the keyword(s) "transcription regulation" and "activator", "transcription regulation" and "repressor", or "nuclear protein" and "repressor", and the selected clones | |

| name of clone | name of representative sequence |
|---|---|
| HEMBA1000462 | HRIFA000432a |
| HEMBA1000671 | HRIFA000631a |
| HEMBA1000875 | HRIFA000814a |
| HEMBA1001184 | HRIFA001099a |
| HEMBA1001296 | HRIFA001200a |
| HEMBA1001886 | HRIFA001720a |
| HEMBA1002048 | HRIFA001866a |
| HEMBA1002985 | HRIFA002689a |
| HEMBA1003120 | HRIFA002805a |
| HEMBA1003497 | HRIFA003063a |
| HEMBA1004007 | HRIFA021040a |
| HEMBA1004067 | HRIFA003340a |
| HEMBA1004085 | HRIFA003357a |
| HEMBA1004785 | HRIFA020862a |
| HEMBA1004952 | HRIFA019532a |
| HEMBA1004971 | HRIFA003883a |
| HEMBA1005230 | HRIFA004006a |
| HEMBA1005246 | HRIFA019490a |
| HEMBA1005267 | HRIFA004034a |
| HEMBA1006276 | HRIFA021224a |
| HEMBA1006517 | HRIFA021445a |
| HEMBA1006544 | HRIFA021494a |
| HEMBA1006770 | HRIFA021651a |
| HEMBA1006912 | HRIFA022335a |
| HEMBA1007063 | HRIFA022348a |
| HEMBA1007226 | HRIFA022411a |
| HEMBB1000106 | HRIFA028262a |
| HEMBB1000309 | HRIFA029602a |
| HEMBB1000407 | HRIFA029649a |
| HEMBB1000542 | HRIFA029715a |
| HEMBB1001959 | HRIFA031336a |
| HEMBB1002039 | HRIFA031395a |
| HEMBB1002041 | HRIFA031397a |
| HEMBB1002051 | HRIFA026351a |
| HEMBB1002120 | HRIFA031438a |
| HEMBB1002302 | HRIFA009136a |
| HEMBB1002661 | HRIFA031869a |
| MAMMA1000528 | HRIFA026265a |
| MAMMA1000614 | HRIFA026316a |
| MAMMA1000810 | HRIFA026615a |
| MAMMA1001094 | HRIFA026789a |
| MAMMA1001532 | HRIFA027125a |
| MAMMA1001609 | HRIFA027173a |
| NT2RM1000407 | HRIFA004401a |
| NT2RM1000789 | HRIFA004663a |
| NT2RM2001558 | HRIFA028804a |
| NT2RM2001738 | HRIFA028867a |
| NT2RM2001767 | HRIFA028983a |
| NT2RM4000100 | HRIFA032389a |
| NT2RP1000239 | HRIFA005214a |
| NT2RP1000271 | HRIFA005240a |
| NT2RP1000465 | HRIFA005369a |
| NT2RP1000468 | HRIFA005372a |
| NT2RP1000679 | HRIFA005500a |
| NT2RP1000740 | HRIFA005540a |
| NT2RP2000092 | HRIFA006183a |
| NT2RP2000178 | HRIFA006250a |
| NT2RP2000240 | HRIFA006298a |
| NT2RP2000610 | HRIFA006566a |
| NT2RP2000712 | HRIFA006649a |
| NT2RP2000739 | HRIFA006667a |
| NT2RP2001223 | HRIFA007032a |
| NT2RP2001276 | HRIFA007068a |
| NT2RP2001388 | HRIFA007152a |
| NT2RP2001538 | HRIFA007262a |
| NT2RP2001662 | HRIFA007352a |
| NT2RP2001817 | HRIFA007463a |
| NT2RP2001921 | HRIFA007547a |
| NT2RP2003138 | HRIFA008426a |
| NT2RP2003302 | HRIFA008547a |
| NT2RP2003940 | HRIFA008981a |
| NT2RP2003950 | HRIFA008989a |
| NT2RP2004069 | HRIFA009071a |
| NT2RP2004108 | HRIFA009101a |
| NT2RP2005069 | HRIFA009762a |
| NT2RP2005391 | HRIFA009983a |
| NT2RP2005535 | HRIFA010085a |
| NT2RP2005666 | HRIFA010176a |
| NT2RP2005774 | HRIFA015063a |
| NT2RP2006092 | HRIFA010460a |
| NT2RP2006134 | HRIFA010490a |
| NT2RP3000148 | HRIFA022249a |
| NT2RP3000232 | HRIFA022564a |
| NT2RP3000378 | HRIFA022671a |
| NT2RP3000427 | HRIFA025033a |
| NT2RP3000652 | HRIFA022895a |
| NT2RP3000677 | HRIFA023007a |
| NT2RP3001271 | HRIFA023634a |
| NT2RP3001650 | HRIFA026923a |
| NT2RP3001976 | HRIFA026496a |
| NT2RP3002286 | HRIFA024185a |
| NT2RP3002353 | HRIFA024893a |
| NT2RP3002664 | HRIFA024305a |
| NT2RP3004025 | HRIFA025290a |
| NT2RP3004119 | HRIFA025695a |
| OVARC1000995 | HRIFA011512a |
| OVARC1001132 | HRIFA022707a |
| OVARC1001596 | HRIFA019437a |
| OVARC1001725 | HRIFA019958a |
| OVARC1001952 | HRIFA019466a |
| OVARC1002178 | HRIFA021007a |
| PLACE1000258 | HRIFA011820a |
| PLACE1000442 | HRIFA011947a |
| PLACE1000907 | HRIFA012278a |
| PLACE1003529 | HRIFA013980a |
| PLACE1003598 | HRIFA014024a |
| PLACE1004166 | HRIFA014396a |
| PLACE1004168 | HRIFA014397a |
| PLACE1004519 | HRIFA014620a |
| PLACE1005239 | HRIFA015122a |
| PLACE1005682 | HRIFA015423a |
| PLACE1006208 | HRIFA015756a |
| PLACE1006515 | HRIFA015947a |
| PLACE1007028 | HRIFA016255a |
| PLACE1007591 | HRIFA016599a |
| PLACE1008549 | HRIFA017190a |
| PLACE1009735 | HRIFA017921a |
| PLACE1011407 | HRIFA018931a |
| PLACE1011978 | HRIFA019262a |
| THYRO1000580 | HRIFA029208a |
| THYRO1000964 | HRIFA029398a |
| THYRO1001641 | HRIFA030472a |
| Y79AA1000030 | HRIFA025936a |
| Y79AA1000750 | HRIFA028187a |
| Y79AA1001212 | HRIFA005296a |
| Y79AA1002058 | HRIFA032161a |
| Y79AA1002121 | HRIFA032186a |
| Y79AA1002129 | HRIFA011926a |
| Y79AA1002334 | HRIFA032257a |
| Y79AA1002378 | HRIFA032274a |

| | |
|---|---|
| The representative sequences of the most homologous sequences in the SwissProt with the keyword(s) "disease mutation", or "syndrome", and the selected clones | |

| name of clone | name of representative sequence |
|---|---|
| HEMBA1000732 | HRIFA000683a |
| HEMBA1000835 | HRIFA000776a |
| HEMBA1004391 | HRIFA020693a |
| MAMMA1001623 | HRIFA027179a |
| NT2RM2000497 | HRIFA021781a |
| NT2RM2000632 | HRIFA022166a |
| NT2RP1000579 | HRIFA005438a |
| NT2RP2001903 | HRIFA007532a |
| NT2RP2005878 | HRIFA008186a |
| NT2RP3002411 | HRIFA005781a |
| NT2RP3002737 | HRIFA024132a |
| NT2RP4002187 | HRIFA030342a |
| PLACE1000033 | HRIFA011659a |
| PLACE1001500 | HRIFA012692a |
| PLACE1005815 | HRIFA015506a |
| PLACE1008657 | HRIFA017257a |
| PLACE1010713 | HRIFA024504a |

| | | |
|---|---|---|
| The clones selected by the keyword(s) of the top hit data in the SwissProt, and having the maximal score in the ATGpr1 0.5 or higher. | | |

| name of clone | name of sequence | maximal ATGpr1 score |
|---|---|---|
| BNGH41000020 | F-BNGH41000020 | 0.94 |
| BNGH41000087 | F-BNGH41000087 | 0.57 |
| BNGH41000091 | F-BNGH41000091 | 0.81 |
| HEMBA1000121 | F-HEMBA1000121 | 0.94 |
| HEMBA1000128 | F-HEMBA1000128 | 0.81 |
| HEMBA1000275 | F-HEMBA1000275 | 0.83 |
| HEMBA1000349 | F-HEMBA1000349 | 0.52 |
| HEMBA1000443 | F-HEMBA1000443 | 0.56 |
| HEMBA1000462 | F-HEMBA1000462 | 0.75 |
| HEMBA1000477 | F-HEMBA1000477 | 0.55 |
| HEMBA1000590 | F-HEMBA1000590 | 0.75 |
| HEMBA1000634 | F-HEMBA1000634 | 0.94 |
| HEMBA1000671 | F-HEMBA1000671 | 0.65 |
| HEMBA1000745 | F-HEMBA1000745 | 0.78 |
| HEMBA1000835 | F-HEMBA1000835 | 0.64 |
| HEMBA1000907 | F-HEMBA1000907 | 0.94 |
| HEMBA1000940 | F-HEMBA1000940 | 0.86 |
| HEMBA1001184 | F-HEMBA1001184 | 0.54 |
| HEMBA1001221 | F-HEMBA1001221 | 0.89 |
| HEMBA1001228 | F-HEMBA1001228 | 0.94 |
| HEMBA1001390 | F-HEMBA1001390 | 0.90 |
| HEMBA1001621 | F-HEMBA1001621 | 0.94 |
| HEMBA1001878 | F-HEMBA1001878 | 0.92 |
| HEMBA1002048 | F-HEMBA1002048 | 0.71 |
| HEMBA1002131 | F-HEMBA1002131 | 0.94 |
| HEMBA1002164 | F-HEMBA1002164 | 0.74 |
| HEMBA1002167 | F-HEMBA1002167 | 0.94 |
| HEMBA1002178 | F-HEMBA1002178 | 0.91 |
| HEMBA1002316 | F-HEMBA1002316 | 0.73 |
| HEMBA1002421 | F-HEMBA1002421 | 0.90 |
| HEMBA1002524 | F-HEMBA1002524 | 0.80 |
| HEMBA1002551 | F-HEMBA1002551 | 0.60 |
| HEMBA1002767 | F-HEMBA1002767 | 0.94 |
| HEMBA1002985 | F-HEMBA1002985 | 0.90 |
| HEMBA1002992 | F-HEMBA1002992 | 0.76 |
| HEMBA1003047 | F-HEMBA1003047 | 0.70 |
| HEMBA1003072 | F-HEMBA1003072 | 0.94 |
| HEMBA1003101 | F-HEMBA1003101 | 0.94 |
| HEMBA1003230 | F-HEMBA1003230 | 0.77 |
| HEMBA1003315 | F-HEMBA1003315 | 0.76 |
| HEMBA1003392 | F-HEMBA1003392 | 0.94 |
| HEMBA1003487 | F-HEMBA1003487 | 0.90 |
| HEMBA1003497 | F-HEMBA1003497 | 0.61 |
| HEMBA1003530 | F-HEMBA1003530 | 0.94 |
| HEMBA1003945 | F-HEMBA1003945 | 0.94 |
| HEMBA1004085 | F-HEMBA1004085 | 0.59 |
| HEMBA1004250 | F-HEMBA1004250 | 0.83 |
| HEMBA1004391 | F-HEMBA1004391 | 0.94 |
| HEMBA1004444 | F-HEMBA1004444 | 0.94 |
| HEMBA1004454 | F-HEMBA1004454 | 0.50 |
| HEMBA1004505 | F-HEMBA1004505 | 0.94 |
| HEMBA1004785 | F-HEMBA1004785 | 0.89 |
| HEMBA1004797 | F-HEMBA1004797 | 0.66 |
| HEMBA1004982 | F-HEMBA1004982 | 0.55 |
| HEMBA1005070 | F-HEMBA1005070 | 0.94 |
| HEMBA1005084 | F-HEMBA1005084 | 0.89 |
| HEMBA1005145 | F-HEMBA1005145 | 0.85 |
| HEMBA1005337 | F-HEMBA1005337 | 0.62 |
| HEMBA1005449 | F-HEMBA1005449 | 0.91 |
| HEMBA1005489 | F-HEMBA1005489 | 0.70 |
| HEMBA1005522 | F-HEMBA1005522 | 0.73 |
| HEMBA1005545 | F-HEMBA1005545 | 0.94 |
| HEMBA1005698 | F-HEMBA1005698 | 0.68 |
| HEMBA1005929 | F-HEMBA1005929 | 0.72 |
| HEMBA1005945 | F-HEMBA1005945 | 0.80 |
| HEMBA1006276 | F-HEMBA1006276 | 0.50 |
| HEMBA1006299 | F-HEMBA1006299 | 0.94 |
| HEMBA1006335 | F-HEMBA1006335 | 0.94 |
| HEMBA1006430 | F-HEMBA1006430 | 0.93 |
| HEMBA1006482 | F-HEMBA1006482 | 0.59 |
| HEMBA1006517 | F-HEMBA1006517 | 0.68 |
| HEMBA1006544 | F-HEMBA1006544 | 0.94 |
| HEMBA1006572 | F-HEMBA1006572 | 0.62 |
| HEMBA1006707 | F-HEMBA1006707 | 0.94 |
| HEMBA1006724 | F-HEMBA1006724 | 0.80 |
| HEMBA1006749 | F-HEMBA1006749 | 0.94 |
| HEMBA1006902 | F-HEMBA1006902 | 0.94 |
| HEMBA1006916 | F-HEMBA1006916 | 0.80 |
| HEMBA1007013 | F-HEMBA1007013 | 0.82 |
| HEMBA1007057 | F-HEMBA1007057 | 0.94 |
| HEMBA1007226 | F-HEMBA1007226 | 0.50 |
| HEMBA1007241 | F-HEMBA1007241 | 0.94 |
| HEMBB1000106 | F-HEMBB1000106 | 0.94 |
| HEMBB1000447 | F-HEMBB1000447 | 0.73 |
| HEMBB1000668 | F-HEMBB1000668 | 0.50 |
| HEMBB1000679 | F-HEMBB1000679 | 0.91 |
| HEMBB1000881 | F-HEMBB1000881 | 0.77 |
| HEMBB1001026 | F-HEMBB1001026 | 0.94 |
| HEMBB1001048 | F-HEMBB1001048 | 0.88 |
| HEMBB1001200 | F-HEMBB1001200 | 0.81 |
| HEMBB1001573 | F-HEMBB1001573 | 0.80 |
| HEMBB1001847 | F-HEMBB1001847 | 0.81 |
| HEMBB1001959 | F-HEMBB1001959 | 0.94 |
| HEMBB1002041 | F-HEMBB1002041 | 0.79 |
| HEMBB1002051 | F-HEMBB1002051 | 0.60 |
| HEMBB1002302 | F-HEMBB1002302 | 0.89 |
| HEMBB1002427 | F-HEMBB1002427 | 0.94 |
| HEMBB1002661 | F-HEMBB1002661 | 0.94 |
| MAMMA1000106 | F-MAMMA1000106 | 0.78 |
| MAMMA1000204 | F-MAMMA1000204 | 0.94 |
| MAMMA1000226 | F-MAMMA1000226 | 0.94 |
| MAMMA1000403 | F-MAMMA1000403 | 0.59 |
| MAMMA1000473 | F-MAMMA1000473 | 0.86 |
| MAMMA1000496 | F-MAMMA1000496 | 0.70 |
| MAMMA1000591 | F-MAMMA1000591 | 0.77 |
| MAMMA1000681 | F-MAMMA1000681 | 0.94 |
| MAMMA1000788 | F-MAMMA1000788 | 0.83 |
| MAMMA1000814 | F-MAMMA1000814 | 0.94 |
| MAMMA1000881 | F-MAMMA1000881 | 0.51 |
| MAMMA1001043 | F-MAMMA1001043 | 0.81 |
| MAMMA1001094 | F-MAMMA1001094 | 0.80 |
| MAMMA1001150 | F-MAMMA1001150 | 0.91 |
| MAMMA1001237 | F-MAMMA1001237 | 0.52 |
| MAMMA1001532 | F-MAMMA1001532 | 0.52 |
| MAMMA1001615 | F-MAMMA1001615 | 0.94 |
| MAMMA1001634 | F-MAMMA1001634 | 0.94 |
| MAMMA1001893 | F-MAMMA1001893 | 0.90 |
| MAMMA1001957 | F-MAMMA1001957 | 0.94 |
| MAMMA1002070 | F-MAMMA1002070 | 0.82 |
| MAMMA1002080 | F-MAMMA1002080 | 0.72 |
| MAMMA1002091 | F-MAMMA1002091 | 0.70 |
| MAMMA1002095 | F-MAMMA1002095 | 0.85 |
| MAMMA1002128 | F-MAMMA1002128 | 0.79 |
| MAMMA1002234 | F-MAMMA1002234 | 0.94 |
| MAMMA1002586 | F-MAMMA1002586 | 0.67 |
| MAMMA1002633 | F-MAMMA1002633 | 0.94 |
| MAMMA1003126 | F-MAMMA1003126 | 0.94 |
| NT2RM1000407 | F-NT2RM1000407 | 0.94 |
| NT2RM1000462 | F-NT2RM1000462 | 0.94 |
| NT2RM1000542 | F-NT2RM1000542 | 0.94 |
| NT2RM1000580 | F-NT2RM1000580 | 0.94 |
| NT2RM1000789 | F-NT2RM1000789 | 0.82 |
| NT2RM1000855 | F-NT2RM1000855 | 0.94 |
| NT2RM1000858 | F-NT2RM1000858 | 0.50 |
| NT2RM1000899 | F-NT2RM1000899 | 0.78 |
| NT2RM2000410 | F-NT2RM2000410 | 0.82 |
| NT2RM2000423 | F-NT2RM2000423 | 0.66 |
| NT2RM2000565 | F-NT2RM2000565 | 0.56 |
| NT2RM2000582 | F-NT2RM2000582 | 0.78 |
| NT2RM2000589 | F-NT2RM2000589 | 0.86 |
| NT2RM2000622 | F-NT2RM2000622 | 0.94 |
| NT2RM2000632 | F-NT2RM2000632 | 0.73 |
| NT2RM2000773 | F-NT2RM2000773 | 0.64 |
| NT2RM2001126 | F-NT2RM2001126 | 0.87 |
| NT2RM2001558 | F-NT2RM2001558 | 0.94 |
| NT2RM2001626 | F-NT2RM2001626 | 0.68 |
| NT2RM2001738 | F-NT2RM2001738 | 0.94 |
| NT2RM2001767 | F-NT2RM2001767 | 0.82 |
| NT2RM2001818 | F-NT2RM2001818 | 0.70 |
| NT2RM2001902 | F-NT2RM2001902 | 0.69 |
| NT2RM2001939 | F-NT2RM2001939 | 0.55 |
| NT2RM2001941 | F-NT2RM2001941 | 0.94 |
| NT2RM4000100 | F-NT2RM4000100 | 0.74 |
| NT2RM4000198 | F-NT2RM4000198 | 0.85 |
| NT2RM4000284 | F-NT2RM4000284 | 0.89 |
| NT2RM4000295 | F-NT2RM4000295 | 0.74 |
| NT2RM4000417 | F-NT2RM4000417 | 0.82 |
| NT2RM4000444 | F-NT2RM4000444 | 0.94 |
| NT2RM4000587 | F-NT2RM4000587 | 0.94 |
| NT2RM4000648 | F-NT2RM4000648 | 0.88 |
| NT2RM4000965 | F-NT2RM4000965 | 0.60 |
| NT2RM4001377 | F-NT2RM4001377 | 0.82 |
| NT2RM4001735 | F-NT2RM4001735 | 0.94 |
| NT2RM4002352 | F-NT2RM4002352 | 0.83 |
| NT2RP1000002 | F-NT2RP1000002 | 0.76 |
| NT2RP1000050 | F-NT2RP1000050 | 0.54 |
| NT2RP1000181 | F-NT2RP1000181 | 0.73 |
| NT2RP1000239 | F-NT2RP1000239 | 0.94 |
| NT2RP1000261 | F-NT2RP1000261 | 0.57 |
| NT2RP1000300 | F-NT2RP1000300 | 0.94 |
| NT2RP1000465 | F-NT2RP1000465 | 0.94 |
| NT2RP1000468 | F-NT2RP1000468 | 0.94 |
| NT2RP1000551 | F-NT2RP1000551 | 0.94 |
| NT2RP1000579 | F-NT2RP1000579 | 0.94 |
| NT2RP1000613 | F-NT2RP1000613 | 0.90 |
| NT2RP1000679 | F-NT2RP1000679 | 0.68 |
| NT2RP1000740 | F-NT2RP1000740 | 0.80 |
| NT2RP1000981 | F-NT2RP1000981 | 0.94 |
| NT2RP1001004 | F-NT2RP1001004 | 0.74 |
| NT2RP1001020 | F-NT2RP1001020 | 0.70 |
| NT2RP1001031 | F-NT2RP1001031 | 0.59 |
| NT2RP2000092 | F-NT2RP2000092 | 0.88 |
| NT2RP2000394 | F-NT2RP2000394 | 0.94 |
| NT2RP2000447 | F-NT2RP2000447 | 0.50 |
| NT2RP2000514 | F-NT2RP2000514 | 0.82 |
| NT2RP2000533 | F-NT2RP2000533 | 0.94 |
| NT2RP2000610 | F-NT2RP2000610 | 0.94 |
| NT2RP2000616 | F-NT2RP2000616 | 0.80 |
| NT2RP2000649 | F-NT2RP2000649 | 0.53 |
| NT2RP2000663 | F-NT2RP2000663 | 0.88 |
| NT2RP2000694 | F-NT2RP2000694 | 0.74 |
| NT2RP2000818 | F-NT2RP2000818 | 0.54 |
| NT2RP2000903 | F-NT2RP2000903 | 0.59 |
| NT2RP2001200 | F-NT2RP2001200 | 0.88 |
| NT2RP2001223 | F-NT2RP2001223 | 0.92 |
| NT2RP2001276 | F-NT2RP2001276 | 0.59 |
| NT2RP2001480 | F-NT2RP2001480 | 0.85 |
| NT2RP2001495 | F-NT2RP2001495 | 0.78 |
| NT2RP2001514 | F-NT2RP2001514 | 0.78 |
| NT2RP2001529 | F-NT2RP2001529 | 0.94 |
| NT2RP2001538 | F-NT2RP2001538 | 0.89 |
| NT2RP2001662 | F-NT2RP2001662 | 0.94 |
| NT2RP2001755 | F-NT2RP2001755 | 0.94 |
| NT2RP2001769 | F-NT2RP2001769 | 0.66 |
| NT2RP2001878 | F-NT2RP2001878 | 0.68 |
| NT2RP2001921 | F-NT2RP2001921 | 0.61 |
| NT2RP2001948 | F-NT2RP2001948 | 0.89 |
| NT2RP2001956 | F-NT2RP2001956 | 0.74 |
| NT2RP2002063 | F-NT2RP2002063 | 0.94 |
| NT2RP2002188 | F-NT2RP2002188 | 0.78 |
| NT2RP2002232 | F-NT2RP2002232 | 0.90 |
| NT2RP2002304 | F-NT2RP2002304 | 0.94 |
| NT2RP2002409 | F-NT2RP2002409 | 0.94 |
| NT2RP2002527 | F-NT2RP2002527 | 0.58 |
| NT2RP2002533 | F-NT2RP2002533 | 0.87 |
| NT2RP2002564 | F-NT2RP2002564 | 0.94 |
| NT2RP2002942 | F-NT2RP2002942 | 0.66 |
| NT2RP2002976 | F-NT2RP2002976 | 0.94 |
| NT2RP2003042 | F-NT2RP2003042 | 0.93 |
| NT2RP2003179 | F-NT2RP2003179 | 0.94 |
| NT2RP2003210 | F-NT2RP2003210 | 0.61 |
| NT2RP2003302 | F-NT2RP2003302 | 0.79 |
| NT2RP2003369 | F-NT2RP2003369 | 0.93 |
| NT2RP2003390 | F-NT2RP2003390 | 0.79 |
| NT2RP2003469 | F-NT2RP2003469 | 0.90 |
| NT2RP2003545 | F-NT2RP2003545 | 0.55 |
| NT2RP2003593 | F-NT2RP2003593 | 0.94 |
| NT2RP2003655 | F-NT2RP2003655 | 0.83 |
| NT2RP2003664 | F-NT2RP2003664 | 0.89 |
| NT2RP2004069 | F-NT2RP2004069 | 0.76 |
| NT2RP2004108 | F-NT2RP2004108 | 0.91 |
| NT2RP2004141 | F-NT2RP2004141 | 0.53 |
| NT2RP2004447 | F-NT2RP2004447 | 0.93 |
| NT2RP2004606 | F-NT2RP2004606 | 0.94 |
| NT2RP2004648 | F-NT2RP2004648 | 0.94 |
| NT2RP2004670 | F-NT2RP2004670 | 0.94 |
| NT2RP2004794 | F-NT2RP2004794 | 0.65 |
| NT2RP2004847 | F-NT2RP2004847 | 0.94 |
| NT2RP2005069 | F-NT2RP2005069 | 0.89 |
| NT2RP2005163 | F-NT2RP2005163 | 0.79 |
| NT2RP2005181 | F-NT2RP2005181 | 0.87 |
| NT2RP2005247 | F-NT2RP2005247 | 0.77 |
| NT2RP2005425 | F-NT2RP2005425 | 0.77 |
| NT2RP2005535 | F-NT2RP2005535 | 0.51 |
| NT2RP2005597 | F-NT2RP2005597 | 0.74 |
| NT2RP2005632 | F-NT2RP2005632 | 0.87 |
| NT2RP2005666 | F-NT2RP2005666 | 0.77 |
| NT2RP2005774 | F-NT2RP2005774 | 0.87 |
| NT2RP2005878 | F-NT2RP2005878 | 0.70 |
| NT2RP2005883 | F-NT2RP2005883 | 0.94 |
| NT2RP2005941 | F-NT2RP2005941 | 0.81 |
| NT2RP2005994 | F-NT2RP2005994 | 0.62 |
| NT2RP2006004 | F-NT2RP2006004 | 0.61 |
| NT2RP2006042 | F-NT2RP2006042 | 0.69 |
| NT2RP2006099 | F-NT2RP2006099 | 0.65 |
| NT2RP2006512 | F-NT2RP2006512 | 0.94 |
| NT2RP3000011 | F-NT2RP3000011 | 0.93 |
| NT2RP3000022 | F-NT2RP3000022 | 0.55 |
| NT2RP3000059 | F-NT2RP3000059 | 0.74 |
| NT2RP3000063 | F-NT2RP3000063 | 0.78 |
| NT2RP3000171 | F-NT2RP3000171 | 0.72 |
| NT2RP3000172 | F-NT2RP3000172 | 0.93 |
| NT2RP3000201 | F-NT2RP3000201 | 0.50 |
| NT2RP3000232 | F-NT2RP3000232 | 0.61 |
| NT2RP3000304 | F-NT2RP3000304 | 0.94 |
| NT2RP3000378 | F-NT2RP3000378 | 0.56 |
| NT2RP3000436 | F-NT2RP3000436 | 0.65 |
| NT2RP3000444 | F-NT2RP3000444 | 0.94 |
| NT2RP3000616 | F-NT2RP3000616 | 0.77 |
| NT2RP3000645 | F-NT2RP3000645 | 0.91 |
| NT2RP3000676 | F-NT2RP3000676 | 0.94 |
| NT2RP3000721 | F-NT2RP3000721 | 0.82 |
| NT2RP3000838 | F-NT2RP3000838 | 0.94 |
| NT2RP3000871 | F-NT2RP3000871 | 0.94 |
| NT2RP3000907 | F-NT2RP3000907 | 0.59 |
| NT2RP3000921 | F-NT2RP3000921 | 0.64 |
| NT2RP3001061 | F-NT2RP3001061 | 0.72 |
| NT2RP3001159 | F-NT2RP3001159 | 0.94 |
| NT2RP3001170 | F-NT2RP3001170 | 0.70 |
| NT2RP3001195 | F-NT2RP3001195 | 0.94 |
| NT2RP3001240 | F-NT2RP3001240 | 0.83 |
| NT2RP3001322 | F-NT2RP3001322 | 0.62 |
| NT2RP3001388 | F-NT2RP3001388 | 0.94 |
| NT2RP3001560 | F-NT2RP3001560 | 0.94 |
| NT2RP3001592 | F-NT2RP3001592 | 0.74 |
| NT2RP3001650 | F-NT2RP3001650 | 0.73 |
| NT2RP3001738 | F-NT2RP3001738 | 0.94 |
| NT2RP3002015 | F-NT2RP3002015 | 0.93 |
| NT2RP3002160 | F-NT2RP3002160 | 0.65 |
| NT2RP3002286 | F-NT2RP3002286 | 0.82 |
| NT2RP3002311 | F-NT2RP3002311 | 0.94 |
| NT2RP3002324 | F-NT2RP3002324 | 0.92 |
| NT2RP3002342 | F-NT2RP3002342 | 0.94 |
| NT2RP3002353 | F-NT2RP3002353 | 0.76 |
| NT2RP3002411 | F-NT2RP3002411 | 0.74 |
| NT2RP3002448 | F-NT2RP3002448 | 0.87 |
| NT2RP3002571 | F-NT2RP3002571 | 0.61 |
| NT2RP3002664 | F-NT2RP3002664 | 0.82 |
| NT2RP3002738 | F-NT2RP3002738 | 0.81 |
| NT2RP3002790 | F-NT2RP3002790 | 0.94 |
| NT2RP3002836 | F-NT2RP3002836 | 0.72 |
| NT2RP3002887 | F-NT2RP3002887 | 0.94 |
| NT2RP3002900 | F-NT2RP3002900 | 0.88 |
| NT2RP3002958 | F-NT2RP3002958 | 0.91 |
| NT2RP3002983 | F-NT2RP3002983 | 0.92 |
| NT2RP3003000 | F-NT2RP3003000 | 0.80 |
| NT2RP3003076 | F-NT2RP3003076 | 0.65 |
| NT2RP3003354 | F-NT2RP3003354 | 0.66 |
| NT2RP3003448 | F-NT2RP3003448 | 0.61 |
| NT2RP3003473 | F-NT2RP3003473 | 0.94 |
| NT2RP3003527 | F-NT2RP3003527 | 0.94 |
| NT2RP3003532 | F-NT2RP3003532 | 0.93 |
| NT2RP3003614 | F-NT2RP3003614 | 0.81 |
| NT2RP3003729 | F-NT2RP3003729 | 0.90 |
| NT2RP3003849 | F-NT2RP3003849 | 0.93 |
| NT2RP3003939 | F-NT2RP3003939 | 0.67 |
| NT2RP3004025 | F-NT2RP3004025 | 0.83 |
| NT2RP3004067 | F-NT2RP3004067 | 0.74 |
| NT2RP3004075 | F-NT2RP3004075 | 0.77 |
| NT2RP3004090 | F-NT2RP3004090 | 0.94 |
| NT2RP3004119 | F-NT2RP3004119 | 0.57 |
| NT2RP3004130 | F-NT2RP3004130 | 0.66 |
| NT2RP3004133 | F-NT2RP3004133 | 0.94 |
| NT2RP3004202 | F-NT2RP3004202 | 0.53 |
| NT2RP3004294 | F-NT2RP3004294 | 0.71 |
| NT2RP3004309 | F-NT2RP3004309 | 0.92 |
| NT2RP3004345 | F-NT2RP3004345 | 0.73 |
| NT2RP3004406 | F-NT2RP3004406 | 0.62 |
| NT2RP3004481 | F-NT2RP3004481 | 0.76 |
| NT2RP3004552 | F-NT2RP3004552 | 0.78 |
| NT2RP3004557 | F-NT2RP3004557 | 0.78 |
| NT2RP3004625 | F-NT2RP3004625 | 0.87 |
| NT2RP3004640 | F-NT2RP3004640 | 0.82 |
| NT2RP3004647 | F-NT2RP3004647 | 0.94 |
| NT2RP4000108 | F-NT2RP4000108 | 0.94 |
| NT2RP4000634 | F-NT2RP4000634 | 0.69 |
| NT2RP4000962 | F-NT2RP4000962 | 0.67 |
| NT2RP4001009 | F-NT2RP4001009 | 0.56 |
| NT2RP4001467 | F-NT2RP4001467 | 0.94 |
| NT2RP4001877 | F-NT2RP4001877 | 0.94 |
| NT2RP4001879 | F-NT2RP4001879 | 0.82 |
| NT2RP4002187 | F-NT2RP4002187 | 0.7 |
| NT2RP4002451 | F-NT2RP4002451 | 0.53 |
| NT2RP4002750 | F-NT2RP4002750 | 0.67 |
| OVARC1000003 | F-OVARC1000003 | 0.62 |
| OVARC1000105 | F-OVARC1000105 | 0.62 |
| OVARC1000137 | F-OVARC1000137 | 0.72 |
| OVARC1000255 | F-OVARC1000255 | 0.78 |
| OVARC1000307 | F-OVARC1000307 | 0.94 |
| OVARC1000313 | F-OVARC1000313 | 0.94 |
| OVARC1000331 | F-OVARC1000331 | 0.87 |
| OVARC1000410 | F-OVARC1000410 | 0.79 |
| OVARC1000467 | F-OVARC1000467 | 0.83 |
| OVARC1000529 | F-OVARC1000529 | 0.94 |
| OVARC1000553 | F-OVARC1000553 | 0.94 |
| OVARC1000873 | F-OVARC1000873 | 0.88 |
| OVARC1000916 | F-OVARC1000916 | 0.94 |
| OVARC1000956 | F-OVARC1000956 | 0.92 |
| OVARC1001030 | F-OVARC1001030 | 0.94 |
| OVARC1001049 | F-OVARC1001049 | 0.94 |
| OVARC1001086 | F-OVARC1001086 | 0.73 |
| OVARC1001132 | F-OVARC1001132 | 0.94 |
| OVARC1001163 | F-OVARC1001163 | 0.75 |
| OVARC1001222 | F-OVARC1001222 | 0.67 |
| OVARC1001336 | F-OVARC1001336 | 0.92 |
| OVARC1001338 | F-OVARC1001338 | 0.89 |
| OVARC1001570 | F-OVARC1001570 | 0.94 |
| OVARC1001607 | F-OVARC1001607 | 0.86 |
| OVARC1001725 | F-OVARC1001725 | 0.81 |
| OVARC1001952 | F-OVARC1001952 | 0.66 |
| OVARC1001991 | F-OVARC1001991 | 0.94 |
| OVARC1002058 | F-OVARC1002058 | 0.79 |
| PLACE1000442 | F-PLACE1000442 | 0.93 |
| PLACE1000740 | F-PLACE1000740 | 0.57 |
| PLACE1001016 | F-PLACE1001016 | 0.92 |
| PLACE1001114 | F-PLACE1001114 | 0.94 |
| PLACE1001123 | F-PLACE1001123 | 0.89 |
| PLACE1001231 | F-PLACE1001231 | 0.81 |
| PLACE1001340 | F-PLACE1001340 | 0.77 |
| PLACE1001401 | F-PLACE1001401 | 0.87 |
| PLACE1001407 | F-PLACE1001407 | 0.94 |
| PLACE1001464 | F-PLACE1001464 | 0.91 |
| PLACE1001500 | F-PLACE1001500 | 0.77 |
| PLACE1001516 | F-PLACE1001516 | 0.89 |
| PLACE1001564 | F-PLACE1001564 | 0.52 |
| PLACE1001655 | F-PLACE1001655 | 0.56 |
| PLACE1001795 | F-PLACE1001795 | 0.91 |
| PLACE1001836 | F-PLACE1001836 | 0.81 |
| PLACE1001918 | F-PLACE1001918 | 0.76 |
| PLACE1001949 | F-PLACE1001949 | 0.94 |
| PLACE1002080 | F-PLACE1002080 | 0.76 |
| PLACE1002095 | F-PLACE1002095 | 0.61 |
| PLACE1002153 | F-PLACE1002153 | 0.94 |
| PLACE1002329 | F-PLACE1002329 | 0.94 |
| PLACE1002355 | F-PLACE1002355 | 0.57 |
| PLACE1002374 | F-PLACE1002374 | 0.92 |
| PLACE1002547 | F-PLACE1002547 | 0.87 |
| PLACE1002726 | F-PLACE1002726 | 0.83 |
| PLACE1002905 | F-PLACE1002905 | 0.94 |
| PLACE1002911 | F-PLACE1002911 | 0.73 |
| PLACE1003135 | F-PLACE1003135 | 0.69 |
| PLACE1003163 | F-PLACE1003163 | 0.61 |
| PLACE1003428 | F-PLACE1003428 | 0.61 |
| PLACE1003438 | F-PLACE1003438 | 0.93 |
| PLACE1003460 | F-PLACE1003460 | 0.94 |
| PLACE1003573 | F-PLACE1003573 | 0.78 |
| PLACE1003598 | F-PLACE1003598 | 0.57 |
| PLACE1003644 | F-PLACE1003644 | 0.93 |
| PLACE1003737 | F-PLACE1003737 | 0.88 |
| PLACE1003772 | F-PLACE1003772 | 0.80 |
| PLACE1003852 | F-PLACE1003852 | 0.82 |
| PLACE1004078 | F-PLACE1004078 | 0.94 |
| PLACE1004166 | F-PLACE1004166 | 0.92 |
| PLACE1004168 | F-PLACE1004168 | 0.58 |
| PLACE1004279 | F-PLACE1004279 | 0.64 |
| PLACE1004441 | F-PLACE1004441 | 0.90 |
| PLACE1004450 | F-PLACE1004450 | 0.85 |
| PLACE1004482 | F-PLACE1004482 | 0.57 |
| PLACE1004492 | F-PLACE1004492 | 0.85 |
| PLACE1004519 | F-PLACE1004519 | 0.74 |
| PLACE1004520 | F-PLACE1004520 | 0.94 |
| PLACE1004630 | F-PLACE1004630 | 0.76 |
| PLACE1004648 | F-PLACE1004648 | 0.59 |
| PLACE1004816 | F-PLACE1004816 | 0.94 |
| PLACE1004887 | F-PLACE1004887 | 0.94 |
| PLACE1005003 | F-PLACE1005003 | 0.80 |
| PLACE1005031 | F-PLACE1005031 | 0.88 |
| PLACE1005239 | F-PLACE1005239 | 0.94 |
| PLACE1005383 | F-PLACE1005383 | 0.52 |
| PLACE1005426 | F-PLACE1005426 | 0.52 |
| PLACE1005519 | F-PLACE1005519 | 0.69 |
| PLACE1005544 | F-PLACE1005544 | 0.59 |
| PLACE1005569 | F-PLACE1005569 | 0.68 |
| PLACE1005682 | F-PLACE1005682 | 0.64 |
| PLACE1005736 | F-PLACE1005736 | 0.67 |
| PLACE1005815 | F-PLACE1005815 | 0.94 |
| PLACE1005878 | F-PLACE1005878 | 0.92 |
| PLACE1005927 | F-PLACE1005927 | 0.94 |
| PLACE1006073 | F-PLACE1006073 | 0.94 |
| PLACE1006208 | F-PLACE1006208 | 0.75 |
| PLACE1006277 | F-PLACE1006277 | 0.65 |
| PLACE1006290 | F-PLACE1006290 | 0.94 |
| PLACE1006443 | F-PLACE1006443 | 0.89 |
| PLACE1006716 | F-PLACE1006716 | 0.63 |
| PLACE1006959 | F-PLACE1006959 | 0.89 |
| PLACE1007028 | F-PLACE1007028 | 0.94 |
| PLACE1007081 | F-PLACE1007081 | 0.77 |
| PLACE1007096 | F-PLACE1007096 | 0.84 |
| PLACE1007702 | F-PLACE1007702 | 0.51 |
| PLACE1008282 | F-PLACE1008282 | 0.70 |
| PLACE1008297 | F-PLACE1008297 | 0.64 |
| PLACE1008469 | F-PLACE1008469 | 0.94 |
| PLACE1008549 | F-PLACE1008549 | 0.52 |
| PLACE1008657 | F-PLACE1008657 | 0.94 |
| PLACE1008716 | F-PLACE1008716 | 0.79 |
| PLACE1008744 | F-PLACE1008744 | 0.94 |
| PLACE1008984 | F-PLACE1008984 | 0.74 |
| PLACE1009279 | F-PLACE1009279 | 0.60 |
| PLACE1009527 | F-PLACE1009527 | 0.87 |
| PLACE1009546 | F-PLACE1009546 | 0.80 |
| PLACE1009600 | F-PLACE1009600 | 0.76 |
| PLACE1010011 | F-PLACE1010011 | 0.94 |
| PLACE1010078 | F-PLACE1010078 | 0.86 |
| PLACE1010081 | F-PLACE1010081 | 0.92 |
| PLACE1010251 | F-PLACE1010251 | 0.62 |
| PLACE1010445 | F-PLACE1010445 | 0.94 |
| PLACE1010713 | F-PLACE1010713 | 0.62 |
| PLACE1010827 | F-PLACE1010827 | 0.88 |
| PLACE1010968 | F-PLACE1010968 | 0.51 |
| PLACE1011045 | F-PLACE1011045 | 0.64 |
| PLACE1011116 | F-PLACE1011116 | 0.69 |
| PLACE1011181 | F-PLACE1011181 | 0.78 |
| PLACE1011236 | F-PLACE1011236 | 0.66 |
| PLACE1011364 | F-PLACE1011364 | 0.94 |
| PLACE1011407 | F-PLACE1011407 | 0.64 |
| PLACE1011516 | F-PLACE1011516 | 0.83 |
| PLACE1011708 | F-PLACE1011708 | 0.94 |
| PLACE1011824 | F-PLACE1011824 | 0.94 |
| PLACE3000181 | F-PLACE3000181 | 0.74 |
| SKNMC1000014 | F-SKNMC1000014 | 0.76 |
| SKNMC1000082 | F-SKNMC1000082 | 0.74 |
| THYRO1000196 | F-THYRO1000196 | 0.71 |
| THYRO1000400 | F-THYRO1000400 | 0.73 |
| THYRO1000584 | F-THYRO1000584 | 0.73 |
| THYRO1000678 | F-THYRO1000678 | 0.86 |
| THYRO1000776 | F-THYRO1000776 | 0.91 |
| THYRO1000795 | F-THYRO1000795 | 0.92 |
| THYRO1000846 | F-THYRO1000846 | 0.78 |
| THYRO1000866 | F-THYRO1000866 | 0.56 |
| THYRO1000956 | F-THYRO1000956 | 0.94 |
| THYRO1000964 | F-THYRO1000964 | 0.80 |
| THYRO1001063 | F-THYRO1001063 | 0.87 |
| THYRO1001071 | F-THYRO1001071 | 0.94 |
| THYRO1001102 | F-THYRO1001102 | 0.90 |
| THYRO1001113 | F-THYRO1001113 | 0.74 |
| THYRO1001128 | F-THYRO1001128 | 0.79 |
| THYRO1001205 | F-THYRO1001205 | 0.94 |
| THYRO1001242 | F-THYRO1001242 | 0.94 |
| THYRO1001266 | F-THYRO1001266 | 0.94 |
| THYRO1001456 | F-THYRO1001456 | 0.69 |
| THYRO1001457 | F-THYRO1001457 | 0.88 |
| THYRO1001471 | F-THYRO1001471 | 0.91 |
| THYRO1001478 | F-THYRO1001478 | 0.89 |
| THYRO1001529 | F-THYRO1001529 | 0.55 |
| THYRO1001593 | F-THYRO1001593 | 0.94 |
| THYRO1001608 | F-THYRO1001608 | 0.94 |
| THYRO1001641 | F-THYRO1001641 | 0.94 |
| THYRO1001700 | F-THYRO1001700 | 0.76 |
| THYRO1001702 | F-THYRO1001702 | 0.80 |
| THYRO1001770 | F-THYRO1001770 | 0.73 |
| THYRO1001803 | F-THYRO1001803 | 0.94 |
| Y79AA1000030 | F-Y79AA1000030 | 0.88 |
| Y79AA1000270 | F-Y79AA1000270 | 0.63 |
| Y79AA1000426 | F-Y79AA1000426 | 0.92 |
| Y79AA1000750 | F-Y79AA1000750 | 0.94 |
| Y79AA1000777 | F-Y79AA1000777 | 0.94 |
| Y79AA1000876 | F-Y79AA1000876 | 0.94 |
| Y79AA1000888 | F-Y79AA1000888 | 0.85 |
| Y79AA1000967 | F-Y79AA1000967 | 0.92 |
| Y79AA1001090 | F-Y79AA1001090 | 0.74 |
| Y79AA1001212 | F-Y79AA1001212 | 0.93 |
| Y79AA1001426 | F-Y79AA1001426 | 0.82 |
| Y79AA1001523 | F-Y79AA1001523 | 0.94 |
| Y79AA1001727 | F-Y79AA1001727 | 0.94 |
| Y79AA1001787 | F-Y79AA1001787 | 0.75 |
| Y79AA1001799 | F-Y79AA1001799 | 0.60 |
| Y79AA1001803 | F-Y79AA1001803 | 0.68 |
| Y79AA1002058 | F-Y79AA1002058 | 0.89 |
| Y79AA1002121 | F-Y79AA1002121 | 0.66 |
| Y79AA1002213 | F-Y79AA1002213 | 0.86 |
| Y79AA1002378 | F-Y79AA1002378 | 0.82 |
| Y79AA1002381 | F-Y79AA1002381 | 0.89 |

| | | |
|---|---|---|
| The clones selected by the keyword(s) of the top hit data in the SwissProt, and having the maximal score in the ATGpr1 0.3 or higher and less than 0.5. | | |

| name of clone | name of sequence | maximal ATGpr1 score |
|---|---|---|
| HEMBA1000732 | F-HEMBA1000732 | 0.32 |
| HEMBA1001886 | F-HEMBA1001886 | 0.46 |
| HEMBA1002163 | F-HEMBA1002163 | 0.44 |
| HEMBA1002195 | F-HEMBA1002195 | 0.41 |
| HEMBA1003120 | F-HEMBA1003120 | 0.44 |
| HEMBA1004007 | F-HEMBA1004007 | 0.31 |
| HEMBA1004067 | F-HEMBA1004067 | 0.49 |
| HEMBA1005267 | F-HEMBA1005267 | 0.37 |
| HEMBA1006770 | F-HEMBA1006770 | 0.40 |
| HEMBB1000407 | F-HEMBB1000407 | 0.41 |
| HEMBB1000542 | F-HEMBB1000542 | 0.47 |
| HEMBB1002120 | F-HEMBB1002120 | 0.33 |
| MAMMA1000810 | F-MAMMA1000810 | 0.43 |
| MAMMA1001609 | F-MAMMA1001609 | 0.32 |
| MAMMA1001978 | F-MAMMA1001978 | 0.32 |
| MAMMA1002142 | F-MAMMA1002142 | 0.34 |
| MAMMA1002165 | F-MAMMA1002165 | 0.34 |
| NT2RM2001792 | F-NT2RM2001792 | 0.37 |
| NT2RM4001843 | F-NT2RM4001843 | 0.44 |
| NT2RP1000271 | F-NT2RP1000271 | 0.41 |
| NT2RP2000739 | F-NT2RP2000739 | 0.39 |
| NT2RP2001388 | F-NT2RP2001388 | 0.47 |
| NT2RP2001562 | F-NT2RP2001562 | 0.41 |
| NT2RP2001903 | F-NT2RP2001903 | 0.46 |
| NT2RP2003138 | F-NT2RP2003138 | 0.41 |
| NT2RP2003931 | F-NT2RP2003931 | 0.44 |
| NT2RP2004205 | F-NT2RP2004205 | 0.37 |
| NT2RP2005378 | F-NT2RP2005378 | 0.43 |
| NT2RP2005541 | F-NT2RP2005541 | 0.31 |
| NT2RP2006092 | F-NT2RP2006092 | 0.47 |
| NT2RP2006269 | F-NT2RP2006269 | 0.41 |
| NT2RP3000148 | F-NT2RP3000148 | 0.44 |
| NT2RP3000427 | F-NT2RP3000427 | 0.38 |
| NT2RP3000820 | F-NT2RP3000820 | 0.44 |
| NT2RP3001754 | F-NT2RP3001754 | 0.30 |
| NT2RP3001976 | F-NT2RP3001976 | 0.46 |
| NT2RP3002409 | F-NT2RP3002409 | 0.31 |
| NT2RP3002737 | F-NT2RP3002737 | 0.33 |
| NT2RP3003874 | F-NT2RP3003874 | 0.34 |
| OVARC1000275 | F-OVARC1000275 | 0.44 |
| OVARC1000995 | F-OVARC1000995 | 0.34 |
| OVARC1001569 | F-OVARC1001569 | 0.30 |
| OVARC1001596 | F-OVARC1001596 | 0.36 |
| PLACE1000907 | F-PLACE1000907 | 0.34 |
| PLACE1002967 | F-PLACE1002967 | 0.38 |
| PLACE1003529 | F-PLACE1003529 | 0.31 |
| PLACE1006071 | F-PLACE1006071 | 0.38 |
| PLACE1006515 | F-PLACE1006515 | 0.48 |
| PLACE1007881 | F-PLACE1007881 | 0.43 |
| PLACE1008359 | F-PLACE1008359 | 0.47 |
| PLACE1008985 | F-PLACE1008985 | 0.48 |
| PLACE1009735 | F-PLACE1009735 | 0.37 |
| PLACE1010784 | F-PLACE1010784 | 0.44 |
| PLACE1011978 | F-PLACE1011978 | 0.39 |
| THYRO1000061 | F-THYRO1000061 | 0.38 |
| THYRO1000580 | F-THYRO1000580 | 0.48 |
| Y79AA1000127 | F-Y79AA1000127 | 0.32 |
| Y79AA1001272 | F-Y79AA1001272 | 0.47 |
| Y79AA1002129 | F-Y79AA1002129 | 0.36 |

| | | |
|---|---|---|
| The clones selected by the keyword(s) of the top hit data in the SwissProt, and having the maximal score in the ATGpr1 0 or higher and less than 0.3. | | |

| name of clone | name of sequence | maximal ATGpr1 score |
|---|---|---|
| HEMBA1000006 | F-HEMBA1000006 | 0.14 |
| HEMBA1000875 | F-HEMBA1000875 | 0.12 |
| HEMBA1001296 | F-HEMBA1001296 | 0.08 |
| HEMBA1001563 | F-HEMBA1001563 | 0.17 |
| HEMBA1002227 | F-HEMBA1002227 | 0.05 |
| HEMBA1004952 | F-HEMBA1004952 | 0.05 |
| HEMBA1004971 | F-HEMBA1004971 | 0.24 |
| HEMBA1005230 | F-HEMBA1005230 | 0.14 |
| HEMBA1005246 | F-HEMBA1005246 | 0.17 |
| HEMBA1005913 | F-HEMBA1005913 | 0.12 |
| HEMBA1006912 | F-HEMBA1006912 | 0.11 |
| HEMBA1007063 | F-HEMBA1007063 | 0.14 |
| HEMBA1007291 | F-HEMBA1007291 | 0.14 |
| HEMBA1007332 | F-HEMBA1007332 | 0.23 |
| HEMBB1000309 | F-HEMBB1000309 | 0.15 |
| HEMBB1000567 | F-HEMBB1000567 | 0.15 |
| HEMBB1002039 | F-HEMBB1002039 | 0.15 |
| MAMMA1000528 | F-MAMMA1000528 | 0.11 |
| MAMMA1000614 | F-MAMMA1000614 | 0.23 |
| MAMMA1000706 | F-MAMMA1000706 | 0.26 |
| MAMMA1001066 | F-MAMMA1001066 | 0.28 |
| MAMMA1001418 | F-MAMMA1001418 | 0.12 |
| MAMMA1001623 | F-MAMMA1001623 | 0.23 |
| NT2RM2000497 | F-NT2RM2000497 | 0.17 |
| NT2RM4000326 | F-NT2RM4000326 | 0.23 |
| NT2RM4000593 | F-NT2RM4000593 | 0.16 |
| NT2RM4000761 | F-NT2RM4000761 | 0.13 |
| NT2RP1000325 | F-NT2RP1000325 | 0.29 |
| NT2RP2000178 | F-NT2RP2000178 | 0.28 |
| NT2RP2000240 | F-NT2RP2000240 | 0.16 |
| NT2RP2000712 | F-NT2RP2000712 | 0.15 |
| NT2RP2001469 | F-NT2RP2001469 | 0.28 |
| NT2RP2001817 | F-NT2RP2001817 | 0.15 |
| NT2RP2002510 | F-NT2RP2002510 | 0.26 |
| NT2RP2002824 | F-NT2RP2002824 | 0.14 |
| NT2RP2002974 | F-NT2RP2002974 | 0.05 |
| NT2RP2003940 | F-NT2RP2003940 | 0.25 |
| NT2RP2003950 | F-NT2RP2003950 | 0.07 |
| NT2RP2005391 | F-NT2RP2005391 | 0.19 |
| NT2RP2006134 | F-NT2RP2006134 | 0.10 |
| NT2RP3000125 | F-NT2RP3000125 | 0.26 |
| NT2RP3000481 | F-NT2RP3000481 | 0.11 |
| NT2RP3000652 | F-NT2RP3000652 | 0.24 |
| NT2RP3000677 | F-NT2RP3000677 | 0.11 |
| NT2RP3001012 | F-NT2RP3001012 | 0.22 |
| NT2RP3001271 | F-NT2RP3001271 | 0.29 |
| NT2RP3001542 | F-NT2RP3001542 | 0.28 |
| OVARC1000090 | F-OVARC1000090 | 0.21 |
| OVARC1000439 | F-OVARC1000439 | 0.21 |
| OVARC1001260 | F-OVARC1001260 | 0.10 |
| OVARC1002178 | F-OVARC1002178 | 0.12 |
| PLACE1000033 | F-PLACE1000033 | 0.20 |
| PLACE1000258 | F-PLACE1000258 | 0.21 |
| PLACE1005539 | F-PLACE1005539 | 0.27 |
| PLACE1005745 | F-PLACE1005745 | 0.29 |
| PLACE1007077 | F-PLACE1007077 | 0.21 |
| PLACE1007296 | F-PLACE1007296 | 0.27 |
| PLACE1007591 | F-PLACE1007591 | 0.20 |
| PLACE1007845 | F-PLACE1007845 | 0.21 |
| PLACE2000118 | F-PLACE2000118 | 0.23 |
| PLACE3000213 | F-PLACE3000213 | 0.21 |
| PLACE4000354 | F-PLACE4000354 | 0.20 |
| Y79AA1000207 | F-Y79AA1000207 | 0.21 |
| Y79AA1001062 | F-Y79AA1001062 | 0.28 |
| Y79AA1001863 | F-Y79AA1001863 | 0.15 |
| Y79AA1002334 | F-Y79AA1002334 | 0.23 |
| Y79AA1002376 | F-Y79AA1002376 | 0.29 |

### EXAMPLE 13

### Selection of cDNA clone NT2RP2006580

Clone NT2RP2006580 as well as clone HEMBA1000121 was selected from the representative sequences belonging to HRIFA000116a cluster of the most homologous sequence in the SwissProt with the keywords "transmembrane". Although each of the clones, HEMBA1000121 and NT2RP2006580, was assembled with other clones for 5' extension, any other clones did not extend the clones toward the 5' direction. Accordingly, it is possible that both clones are full-length cDNA clones. The maximal ATGpr1 score of F-NT2RP2006580 is 0.37, and therefore, the fullness ratio is low. However, it is still possible for the sequence to cover the full-length.

Thus, the total number of selected clones is 830. Based on the top matching data resulted from Swiss-Prot homology search, 659 clones were selected. From them, 447 clones were selected by the keywords of "secretion" and "membrane". Among the clones selected based on the top matching data, 60 clones exhibited the maximal ATGpr1 score of 0.3 or higher and less than 0.5.

The sequences of F-NT2RP2006580 and R-NT2RP2006580 are shown in SEQ ID NO: 2545 and SEQ ID NO: 2546, respectively.

### EXAMPLE 14

### Full-length sequence analysis and homology search

Full-length sequence was determined for each selected cDNA clones. The nucleotide sequence determination was performed mainly by the dye-terminator method using custom synthesized DNA primers according to the primer walking procedure (custom synthesized DNA primers were used for sequencing; sequencing reaction was performed with DNA sequencing reagent supplied by PE Biosystems according to the supplier's manual; and the samples were analyzed in an automatic sequencer made by the same supplier). Sequence determination of some clones was carried out in the same manner but using a Licor DNA sequencer. Overlapping partial nucleotide sequences, which were obtained by the above-described method, were assembled together to determine a full-length nucleotide sequence. Amino acid sequences were then deduced from the determined full-length nucleotide sequences. However, amino acid sequence is not shown for a clone of which coding region was hard to be deduced or of which amino acid sequence has less than 100 amino acid residues. SEQ ID NOs corresponding to the respective clones are indicated in Table 370.

GenBank, Swiss-Prot and UniGene were searched for the determined nucleotide sequences by BLAST analysis. Matching data of cDNA clone which exhibits higher homology and of which functions are easily predicted based on the nucleotide sequences and the deduced amino acid sequences are selected from the BLAST analysis matching data with P value of 10⁻⁴ or less. The matching data selected are listed herein. However, there are some clones that did not match the criteria for judgment and such matching data of BLAST analysis are not shown herein. The results of homology search indicated in the last part of this specification are as follows.
Homology search result 1: data obtained by the homology search of Swiss-Prot database for representative sequences of the 5'-end cluster
Homology search result 2: homology of representative sequences of the 5'-end cluster to the data in Swiss-Prot database; the P value is 10⁻¹⁰ or less
Homology search result 3: homology of representative sequences of the 5'-end cluster to the data in Swiss-Prot database; the P value is higher than 10⁻¹⁰ and 10⁻⁴ or less
Homology search result 4: homology of representative sequences of the 5'-end cluster to the data in Swiss-Prot database; the P value is higher than 10⁻⁴ and 1 or less
Homology search result 5: data obtained by the homology search of Swiss-Prot database for 5'-end sequences of cDNA clone
Homology search result 6: data obtained by the homology search of GenBank database (http://www.ncbi.nlm.nih.gov/web/GenBank/) except for EST and STS sequence data for 5'-end sequences of cDNA clone
Homology search result 7: data obtained by the homology search of GenBank database (http://www.ncbi.nlm.nih.gov/web/GenBank/) except for EST and STS sequence data for 3'-end sequences of cDNA clone
Homology search result 8: data obtained by the homology search of Human UniGene database (http://www.ncbi.nlm.nih.gov/Unigene) for 5'-end sequences of cDNA clone
Homology search result 9: data obtained by the homology search of Human UniGene database (http://www.ncbi.nlm.nih.gov/Unigene) for 3'-end sequences of cDNA clone
Homology search result 10: result obtained by the homology search for full-length nucleotide sequences and deduced amino acid sequences
The P value indicates similarity between two sequences as a score by considering the probability that the two sequences are accidentally similar. In general, as the value is lower, the similarity is higher. In general, as the value is lower, the homology is higher (Altschul, S.F., Gish, W., Miller, W., Myers, E.W. & Lipman, D.L. (1990) "Basic local alignment search tool." J. Mol. Biol. 215:403-410; Gish, W. & States, D.J. (1993) " Identification of protein coding regions by database similarity search." Nature Genet. 3:266-272).

### Example 15. Gene expression analysis with hybridization using high density DNA filter

Nylon membrane for DNA spotting was prepared according to the following procedure. E. coli was cultured in each well of a 96-well plate (in a LB medium at 37°C for 16 hours). A sample of each culture was suspended in 10 µl of sterile water in a well of a 96-well plate. The plate was heated at 100°C for 10 minutes. Then, the boiled samples were analyzed by PCR. PCR was performed in a 20 µl solution by using TaKaRa PCR Amplification Kit (Takara) according to the supplier's protocol. Primers used for the amplification of an insert cDNA in a plasmid were a pair of sequencing primers, ME761FW (5' tacggaagtgttacttctgc 3'/SEQ ID NO: 3591) and ME1250RV (5' tgtgggaggttttttctcta 3'/ SEQ ID NO: 3592), or a pair of primers, M13M4 (5' gttttcccagtcacgac 3' / SEQ ID NO: 3593) and M13RV (5' caggaaacagctatgac 3' / SEQ ID NO: 3594). PCR was performed using a thermal cycler, GeneAmp System 9600 (PE Biosystems) at 95°C for 5 minutes; at 95°C for 10 seconds and at 68°C for 1 minute for 10 cycles; at 98°C for 20 seconds and at 60°C for 3 minutes for 20 cycles; and at 72°C for 10 minutes. After the PCR, the 20 µl reaction solution was loaded onto a 1% agarose gel and fractionated by electrophoresis. DNA on the gel was stained with ethidium bromide to confirm the amplification of cDNA. When cDNAs were not amplified by PCR, plasmids containing the corresponding insert cDNAs were prepared by the alkali-extraction method (J. Sambrook, E.F., Fritsh, & T. Maniatis, "Molecular Cloning, A laboratory manual/ 2nd edition, Cold Spring Harbor Laboratory Press, 1989).

Preparation of DNA array was carried out by the following procedure. A sample of a DNA solution was added in each well of a 384-well plate. DNA was spotted onto a nylon membrane (Boehringer) by using a 384-pin tool of Biomek 2000 Laboratory Automation System (Beckman-Coulter). Specifically, the 384-well plate containing the DNA was placed under the 384-pin tool. The independent 384 needles were simultaneously dipped into the DNA solution for DNA deposition. The needles were gently pressed onto a nylon membrane and the DNA deposited at the tips of needles was spotted onto the membrane. Denaturation of the spotted DNA and immobilization of the DNA on the nylon membrane were carried out according to standard methods (J. Sambrook, E.F., Fritsh, & T. Maniatis, "Molecular Cloning, A laboratory manual/ 2nd edition, Cold Spring Harbor Laboratory Press, 1989).

A probe for hybridization was radioisotope-labeled first strand cDNA. Synthesis of the first strand cDNA was performed by using Thermoscript⁽™⁾ RT-PCR System (GIBCO). Specifically, the first strand cDNA was synthesized by using 1.5 µg of mRNAs from various human tissues (Clontech), 1 µl of 50 µM Oligo(dT)20 and 50 µCi [α ³³P]dATP according to an attached protocol. Purification of a probe was carried out by using ProbeQuant⁽™⁾ G-50 micro column (Amersham-Pharmacia Biotech) according to an attached protocol. In the next step, 2 units of E. coli RNase H were added to the reaction mixture. The mixture was incubated at room temperature for 10 minutes, and then, 100µg of human COT-1 DNA (GIBCO) was added thereto. The mixture was incubated at 97°C for 10 minutes and then was allowed to stand on ice to give hybridization probe.

Hybridization of the radioisotope-labeled probe to the DNA array was performed according to standard methods (J. Sambrook, E.F., Fritsh, & T. Maniatis, Molecular Cloning, A laboratory manual/ 2nd edition, Cold Spring Harbor Laboratory Press, 1989). The membrane was washed as follows: the nylon membrane was washed 3 times by incubating it in Washing solution 1 (2×SSC, 1% SDS) at room temperature (about 26°C) for 20 minutes; then the membrane was washed 3 times by incubating it in Washing solution 2 (0.1 ×SSC, 1% SDS) at 65°C for 20 minutes.

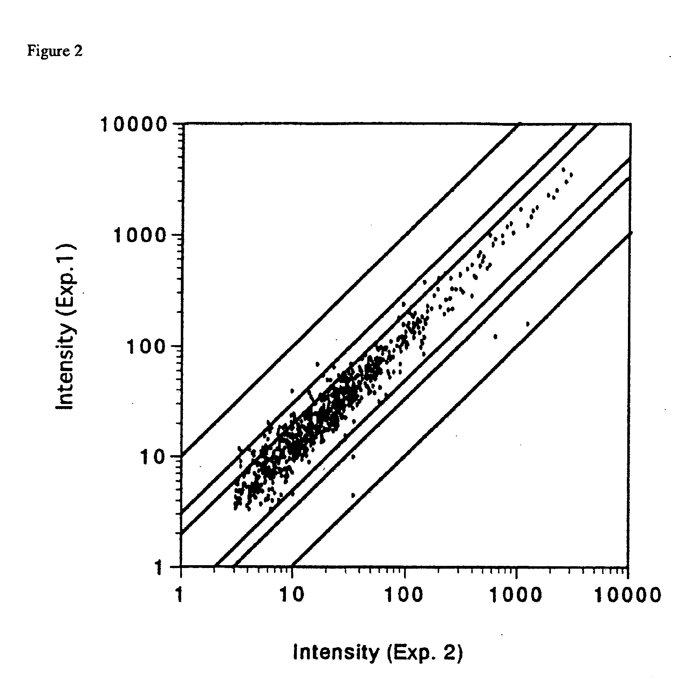

Autoradiography was performed by using an image plate for BAS2000 (Fuji Photo Film Co., Ltd.). Specifically, the nylon membrane with probe hybridized thereon was wrapped with a piece of Saran Wrap and brought into tight contact with the image plate on the light-sensitive surface. The membrane with the image plate was placed in an imaging cassette for radioisotope and allowed to stand in dark place for 4 hours. The radioactivity recorded on the image plate was analyzed by using BAS2000 (Fuji Photo Film Co., Ltd.). The activity was subjected to electronic conversion and recorded as an image file of autoradiogram. The signal intensity of each DNA spot was analyzed by using Visage High Density Grid Analysis Systems (Genomic Solutions Inc.). The signal intensity was converted into numerical data. The data were taken in duplicate. The reproducibility was assessed by comparing the signal intensities of the corresponding spots on the duplicated DNA filters that were hybridized to a single DNA probe (Figure 2). In 95% of entire spots, the ratio between the corresponding spots falls within a range of 2 or less, and the correlation coefficient is r=1.97. Thus, the reproducibility is satisfactory.

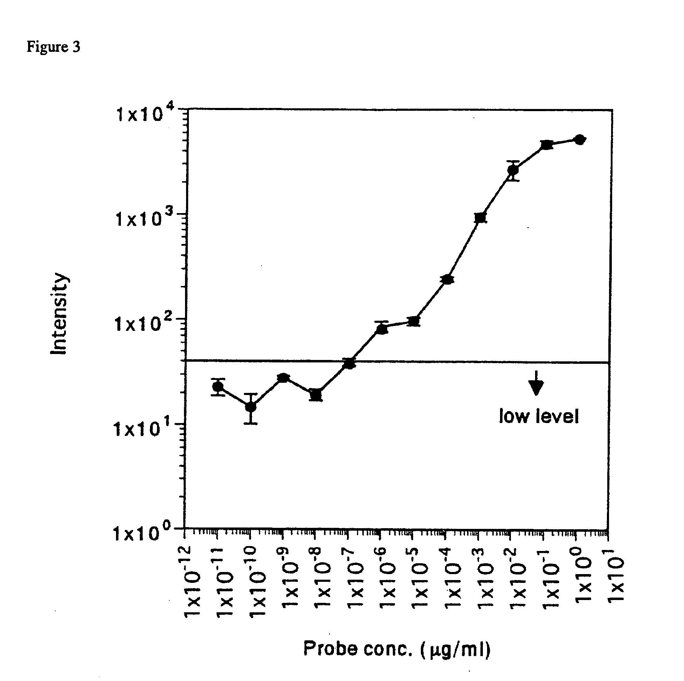

The detection sensitivity in gene expression analysis was estimated by examining increases in the signal intensity of probe concentration-dependent spot in hybridization using a probe complementary to the DNA spotted on the nylon membrane. DNA used was PLACE1008092 (the same as DNA deposited in GenBank under an Accession No. AF107253). The DNA array with DNA of PLACE1008092 was prepared according to the above-mentioned method. The probe used was prepared as follows: mRNA was synthesized in vitro from the clone, PLACE1008092. By using this mRNA as a template, radioisotope-labeled first strand cDNA was synthesized in the same manner as described above, and the cDNA was used as the probe. In order to synthesize mRNA from PLACE1008092 in vitro, a plasmid in which the 5' end of the cDNA PLACE1008092 was ligated to the T7 promoter of pBluescript SK(-) was constructed. Specifically, the PLACE1008092 insert was cut out from pME18SFL3 carrying the cDNA at a DraIII site thereof by XhoI digestion. The resulting PLACE1008092 fragment was ligated to XhoI-predigested pBluescript SK(-) by using DNA ligation kit ver.2 (Takara). The in vitro mRNA synthesis from PLACE1008092 inserted into pBluescript SK(-) was carried out by using Ampliscribe⁽™⁾ T7 high yield transcription kit (Epicentre technologies). Hybridization and the analysis of signal intensity of each DNA spot were performed by the same methods as described above. When the probe concentration is 1×10⁷ µg/ml or less, there was no increase of signal intensity proportional to the probe concentration. Therefore, it was assumed to be difficult to compare the signals with one another in this concentration range. Thus, the spots with the intensity of 40 or less were uniformly taken as low level signals (Figure 3). Within a concentration of the probe ranging from 1×10⁷ µg/ml to 0.1 µg/ml, the signal was found to increase in a probe concentration-dependent manner. The detection limit represented as the ratio of the expression level of test mRNA to that of total mRNA in a sample was 1:100,000.

Tables 28-184 (also containing clones without description in Examples) show the expression of each cDNA in human normal tissues (heart, lung, pituitary gland, thymus, brain, kidney, liver and spleen). The expression levels are indicated with numerical values of 0-10,000. Genes that were expressed in at least a single tissue are indicated below by the corresponding clone names:
clone: BNGH41000020, BNGH41000087, BNGH41000091, HEMBA1000121, HEMBA1000275, HEMBA1000300, HEMBA1000443, HEMBA1000462, HEMBA1000477, HEMBA1000634, HEMBA1000713, HEMBA1000835, HEMBA1000875, HEMBA1000940, HEMBA1000962, HEMBA1001228, HEMBA1001296, HEMBA1001390, HEMBA1001563, HEMBA1001621, HEMBA1002048, HEMBA1002131, HEMBA1002163, HEMBA1002164, HEMBA1002167, HEMBA1002178, HEMBA1002195, HEMBA1002227, HEMBA1002239, HEMBA1002316, HEMBA1002421, HEMBA1002524, HEMBA1002551, HEMBA1002767, HEMBA1002985, HEMBA1002992, HEMBA1003047, HEMBA1003072, HEMBA1003101, HEMBA1003120, HEMBA1003230, HEMBA1003294, HEMBA1003315, HEMBA1003392, HEMBA1003399, HEMBA1003487, HEMBA1003530, HEMBA1003945, HEMBA1004007, HEMBA1004067, HEMBA1004085, HEMBA1004110, HEMBA1004391, HEMBA1004444, HEMBA1004454, HEMBA1004505, HEMBA1004797, HEMBA1004952, HEMBA1005070, HEMBA1005084, HEMBA1005145, HEMBA1005230, HEMBA1005246, HEMBA1005337, HEMBA1005430, HEMBA1005449, HEMBA1005489, HEMBA1005545, HEMBA1005698, HEMBA1005929, HEMBA1005945, HEMBA1006016, HEMBA1006171, HEMBA1006276, HEMBA1006311, HEMBA1006335, HEMBA1006357, HEMBA1006430, HEMBA1006482, HEMBA1006517, HEMBA1006544, HEMBA1006658, HEMBA1006707, HEMBA1006749, HEMBA1006770, HEMBA1006902, HEMBA1006912, HEMBA1006916, HEMBA1006960, HEMBA1007013, HEMBA1007057, HEMBA1007063, HEMBA1007291, HEMBA1007332, HEMBB1000106, HEMBB1000309, HEMBB1000447, HEMBB1000542,
   HEMBB1000567, HEMBB1000642, HEMBB1000905, HEMBB1001026, HEMBB1001048, HEMBB1001407, HEMBB1001530, HEMBB1001573, HEMBB1001847, HEMBB1001959, HEMBB1001978, HEMBB1002039, HEMBB1002041, HEMBB1002051, HEMBB1002162, HEMBB1002228, HEMBB1002302, HEMBB1002427, HEMBB1002465, HEMBB1002661, HEMBB1002663, HEMBB1002693, MAMMA1000046, MAMMA1000102, MAMMA1000106, MAMMA1000118, MAMMA1000204, MAMMA1000226, MAMMA1000403, MAMMA1000449, MAMMA1000457, MAMMA1000473, MAMMA1000528, MAMMA1000591, MAMMA1000614, MAMMA1000652, MAMMA1000681, MAMMA1000706, MAMMA1000788, MAMMA1000810, MAMMA1000814, MAMMA1000881, MAMMA1000986, MAMMA1000994, MAMMA1001043, MAMMA1001066, MAMMA1001094, MAMMA1001141, MAMMA1001150, MAMMA1001284, MAMMA1001310, MAMMA1001344, MAMMA1001418, MAMMA1001532, MAMMA1001609, MAMMA1001615, MAMMA1001634, MAMMA1001893, MAMMA1001901, MAMMA1001957, MAMMA1002070, MAMMA1002091, MAMMA1002095, MAMMA1002128, MAMMA1002142, MAMMA1002165, MAMMA1002205, MAMMA1002224, MAMMA1002586, MAMMA1003126, NT2RM1000407, NT2RM1000462, NT2RM1000542, NT2RM1000789, NT2RM1000855, NT2RM1000858, NT2RM2000241, NT2RM2000306, NT2RM2000410, NT2RM2000423, NT2RM2000497, NT2RM2000514, NT2RM2000565, NT2RM2000582, NT2RM2000589, NT2RM2000622, NT2RM2000773, NT2RM2001126, NT2RM2001626, NT2RM2001792, NT2RM2001941, NT2RM4000198, NT2RM4000295, NT2RM4000444, NT2RM4000593, NT2RM4000761, NT2RM4000965, NT2RM4000997, NT2RM4001321, NT2RM4001325,
   NT2RM4001377, NT2RM4001735, NT2RM4001768, NT2RM4001843, NT2RP1000002, NT2RP1000181, NT2RP1000271, NT2RP1000300, NT2RP1000325, NT2RP1000465, NT2RP1000468, NT2RP1000740, NT2RP1000903, NT2RP1000981, NT2RP2000092, NT2RP2000178, NT2RP2000240, NT2RP2000447, NT2RP2000479, NT2RP2000533, NT2RP2000610, NT2RP2000616, NT2RP2000694, NT2RP2000739, NT2RP2001200, NT2RP2001223, NT2RP2001388, NT2RP2001469, NT2RP2001480, NT2RP2001514, NT2RP2001529, NT2RP2001538, NT2RP2001562, NT2RP2001662, NT2RP2001878, NT2RP2001903, NT2RP2001921, NT2RP2001956, NT2RP2002015, NT2RP2002063, NT2RP2002188, NT2RP2002232, NT2RP2002409, NT2RP2002510, NT2RP2002527, NT2RP2002533, NT2RP2002564, NT2RP2002721, NT2RP2002824, NT2RP2002942, NT2RP2002974, NT2RP2003138, NT2RP2003179, NT2RP2003210, NT2RP2003302, NT2RP2003369, NT2RP2003383, NT2RP2003390, NT2RP2003469, NT2RP2003593, NT2RP2003599, NT2RP2003655, NT2RP2003940, NT2RP2003950, NT2RP2004069, NT2RP2004108, NT2RP2004141, NT2RP2004179, NT2RP2004205, NT2RP2004447, NT2RP2004524, NT2RP2004556, NT2RP2004606, NT2RP2004648, NT2RP2004794, NT2RP2004837, NT2RP2004847, NT2RP2005027, NT2RP2005069, NT2RP2005163, NT2RP2005181, NT2RP2005247, NT2RP2005378, NT2RP2005391, NT2RP2005425, NT2RP2005463, NT2RP2005535, NT2RP2005541, NT2RP2005597, NT2RP2005632, NT2RP2005666, NT2RP2005774, NT2RP2005878, NT2RP2005887, NT2RP2005941, NT2RP2006004, NT2RP2006042, NT2RP2006092,
   NT2RP2006099, NT2RP2006269, NT2RP3000011, NT2RP3000022, NT2RP3000059, NT2RP3000063, NT2RP3000125, NT2RP3000148, NT2RP3000171, NT2RP3000172, NT2RP3000201, NT2RP3000232, NT2RP3000304, NT2RP3000378, NT2RP3000436, NT2RP3000460, NT2RP3000645, NT2RP3000652, NT2RP3000676, NT2RP3000677, NT2RP3000721, NT2RP3000789, NT2RP3000818, NT2RP3000820, NT2RP3000838, NT2RP3000907, NT2RP3000921, NT2RP3001044, NT2RP3001159, NT2RP3001170, NT2RP3001195, NT2RP3001271, NT2RP3001388, NT2RP3001560, NT2RP3001592, NT2RP3001685, NT2RP3001738, NT2RP3001754, NT2RP3001858, NT2RP3001976, NT2RP3002015, NT2RP3002160, NT2RP3002281, NT2RP3002311, NT2RP3002324, NT2RP3002353, NT2RP3002409, NT2RP3002411, NT2RP3002721, NT2RP3002737, NT2RP3002738, NT2RP3002836, NT2RP3002900, NT2RP3002958, NT2RP3003000, NT2RP3003076, NT2RP3003354, NT2RP3003448, NT2RP3003469, NT2RP3003473, NT2RP3003532, NT2RP3003614, NT2RP3003729, NT2RP3003849, NT2RP3003874, NT2RP3003939, NT2RP3003963, NT2RP3004025, NT2RP3004067, NT2RP3004083, NT2RP3004090, NT2RP3004119, NT2RP3004130, NT2RP3004133, NT2RP3004202, NT2RP3004294, NT2RP3004309, NT2RP3004321, NT2RP3004355, NT2RP3004374, NT2RP3004406, NT2RP3004481, NT2RP3004552, NT2RP3004557, NT2RP3004625, NT2RP3004640, NT2RP3004647, NT2RP4000108, NT2RP4000634, NT2RP4001877, NT2RP4001879, NT2RP4002187, NT2RP4002715, NT2RP4002750, OVARC1000090, OVARC1000105, OVARC1000137, OVARC1000208,
   OVARC1000255, OVARC1000313, OVARC1000331, OVARC1000410, OVARC1000439, OVARC1000467, OVARC1000529, OVARC1000553, OVARC1000775, OVARC1000853, OVARC1000873, OVARC1000916, OVARC1000956, OVARC1000995, OVARC1001030, OVARC1001049, OVARC1001086, OVARC1001163, OVARC1001260, OVARC1001336, OVARC1001569, OVARC1001570, OVARC1001596, OVARC1001807, OVARC1001833, OVARC1001991, PLACE1000231, PLACE1000258, PLACE1000442, PLACE1000560, PLACE1000912, PLACE1000927, PLACE1001016, PLACE1001100, PLACE1001114, PLACE1001183, PLACE1001229, PLACE1001340, PLACE1001407, PLACE1001500, PLACE1001516, PLACE1001655, PLACE1001836, PLACE1001918, PLACE1002080, PLACE1002095, PLACE1002153, PLACE1002329, PLACE1002374, PLACE1002518, PLACE1002547, PLACE1002726, PLACE1002905, PLACE1002911, PLACE1002967, PLACE1003163, PLACE1003407, PLACE1003428, PLACE1003438, PLACE1003460, PLACE1003529, PLACE1003598, PLACE1003644, PLACE1003772, PLACE1003839, PLACE1003845, PLACE1003852, PLACE1004078, PLACE1004166, PLACE1004168, PLACE1004199, PLACE1004279, PLACE1004282, PLACE1004305, PLACE1004441, PLACE1004482, PLACE1004492, PLACE1004520, PLACE1004630, PLACE1004637, PLACE1004648, PLACE1004816, PLACE1004887, PLACE1005005, PLACE1005031, PLACE1005383, PLACE1005410, PLACE1005426, PLACE1005539, PLACE1005544, PLACE1005569, PLACE1005725, PLACE1005736, PLACE1005768, PLACE1005815, PLACE1005878, PLACE1005927, PLACE1006071,
   PLACE1006073, PLACE1006079, PLACE1006277, PLACE1006443, PLACE1006716, PLACE1006809, PLACE1007077, PLACE1007096, PLACE1007626, PLACE1007702, PLACE1008469, PLACE1008985, PLACE1009067, PLACE1009527, PLACE1009982, PLACE1010078, PLACE1010251, PLACE1010445, PLACE1011045, PLACE1011116, PLACE1011181, PLACE1011236, PLACE1011364, PLACE1011516, PLACE1011708, PLACE1011978, PLACE2000118, PLACE2000219, PLACE3000181, PLACE4000354, PLACE4000455, SKNMC1000014, THYRO1000061, THYRO1000099, THYRO1000584, THYRO1000795, THYRO1000866, THYRO1000999, THYRO1001063, THYRO1001113, THYRO1001128, THYRO1001205, THYRO1001237, THYRO1001242, THYRO1001456, THYRO1001457, THYRO1001478, THYRO1001495, THYRO1001523, THYRO1001529, THYRO1001593, THYRO1001608, THYRO1001700, THYRO1001702, THYRO1001725, THYRO1001770, THYRO1001803, Y79AA1000127, Y79AA1000207, Y79AA1000226, Y79AA1000270, Y79AA1000426, Y79AA1000521, Y79AA1000776, Y79AA1000777, Y79AA1000888, Y79AA1000967, Y79AA1001013, Y79AA1001090, Y79AA1001272, Y79AA1001328, Y79AA1001426, Y79AA1001427, Y79AA1001430, Y79AA1001523, Y79AA1001530, Y79AA1001592, Y79AA1001727, Y79AA1001787, Y79AA1001793, Y79AA1001799, Y79AA1001803, Y79AA1001863, Y79AA1002022, Y79AA1002213, Y79AA1002373, Y79AA1002376, Y79AA1002381.

Genes that were expressed in all the tissues tested are indicated below by the corresponding clone names: clone: BNGH41000020, HEMBA1000300, HEMBA1001390, HEMBA1002239, HEMBA1002316, HEMBA1004007, HEMBA1004067, HEMBA1005145, HEMBA1005230, HEMBA1005929, HEMBA1006357, HEMBA1006482, HEMBB1000567, HEMBB1001847, HEMBB1001978, MAMMA1000614, MAMMA1000652, MAMMA1000810, MAMMA1000814, MAMMA1001066, MAMMA1001094, MAMMA1001284, MAMMA1001310, MAMMA1001634, MAMMA1002165, MAMMA1002205, MAMMA1002224, NT2RM1000462, NT2RM1000855, NT2RM1000858, NT2RM2000423, NT2RM4000761, NT2RM4000997, NT2RP1000271, NT2RP1000325, NT2RP1000465, NT2RP2001538, NT2RP2001662, NT2RP2001903, NT2RP2002015, NT2RP2002188, NT2RP2002409, NT2RP2002510, NT2RP2002533, NT2RP2004556, NT2RP2004794, NT2RP2004847, NT2RP2005069, NT2RP2005163, NT2RP2005535, NT2RP2006269, NT2RP3000171, NT2RP3000645, NT2RP3000838, NT2RP3001271, NT2RP3001754, NT2RP3003076, NT2RP3003354, NT2RP3003614, NT2RP3004640, NT2RP3004647, OVARC1000090, OVARC1000208, OVARC1000553, OVARC1000995, OVARC1001030, OVARC1001049, PLAGE1000231, PLACE1000258, PLACE1001516, PLACE1002080, PLACE1002911, PLACE1003598, PLACE1004648, PLACE1006443, PLACE1008469, PLACE1011708, PLACE2000118, THYRO1001128, THYRO1001205, THYRO1001242, THYRO1001803, Y79AA1000207, Y79AA1001013, Y79AA1001272, Y79AA1001328, Y79AA1001793, Y79AA1001863, Y79AA1002022, Y79AA1002376.

Genes that were expressed at low levels in any of the tissues tested are indicated below by the corresponding clone names: clone: HEMBA1000006, HEMBA1000128, HEMBA1000349, HEMBA1000590, HEMBA1000671, HEMBA1000732, HEMBA1000745, HEMBA1000907, HEMBA1001184, HEMBA1001221, HEMBA1001272, HEMBA1001297, HEMBA1001878, HEMBA1001886, HEMBA1002420, HEMBA1003497, HEMBA1003602, HEMBA1003732, HEMBA1004250, HEMBA1004785, HEMBA1004971, HEMBA1004982. HEMBA1005267, HEMBA1005522, HEMBA1005913, HEMBA1006299, HEMBA1006572, HEMBA1006724, HEMBA1007241, HEMBB1000276, HEMBB1000407, HEMBB1000668, HEMBB1000679, HEMBB1000881, HEMBB1001200, HEMBB1001547, HEMBB1002120, HEMBB1002245, MAMMA1000141, MAMMA1000496, MAMMA1001237, MAMMA1001623, MAMMA1001978, MAMMA1002080, MAMMA1002087, MAMMA1002234, MAMMA1002633, NT2RM1000580, NT2RM1000899, NT2RM2000632, NT2RM2001643, NT2RM2001818, NT2RM2001902, NT2RM2001939, NT2RM4000100, NT2RM4000115, NT2RM4000284, NT2RM4000326, NT2RM4000417, NT2RM4000587, NT2RM4000648, NT2RM4002352, NT2RP1000050, NT2RP1000239, NT2RP1000261, NT2RP1000448, NT2RP1000551, NT2RP1000579, NT2RP1000613, NT2RP1000679, NT2RP1001004, NT2RP1001020, NT2RP1001031, NT2RP1001563, NT2RP2000394, NT2RP2000514, NT2RP2000649, NT2RP2000663, NT2RP2000712, NT2RP2000818, NT2RP2000903, NT2RP2001276, NT2RP2001495, NT2RP2001755, NT2RP2001769, NT2RP2001817, NT2RP2001915, NT2RP2001948, NT2RP2002304, NT2RP2002674, NT2RP2002976, NT2RP2003042, NT2RP2003545, NT2RP2003664, NT2RP2003931, NT2RP2004495, NT2RP2004670, NT2RP2005514, NT2RP2005883, NT2RP2005994,
NT2RP2006134, NT2RP2006512, NT2RP3000169, NT2RP3000444, NT2RP3000481, NT2RP3000616, NT2RP3000871, NT2RP3001012, NT2RP3001061, NT2RP3001240, NT2RP3001322, NT2RP3001542, NT2RP3002286, NT2RP3002342, NT2RP3002448, NT2RP3002571, NT2RP3002664, NT2RP3002790, NT2RP3002887, NT2RP3002983, NT2RP3003527, NT2RP3003535, NT2RP3003559, NT2RP3004000, NT2RP3004075, NT2RP3004345, NT2RP4000962, NT2RP4001001, NT2RP4001009, NT2RP4001467, NT2RP4002451, OVARC1000003, OVARC1000275, OVARC1000298, OVARC1000307, OVARC1000811, OVARC1001132, OVARC1001222, OVARC1001338, OVARC1001607, OVARC1001725, OVARC1001727, OVARC1002058, OVARC1002178, PLACE1000033, PLACE1000740, PLACE1000914, PLACE1000986, PLACE1001123, PLACE1001231, PLACE1001401, PLACE1001464, PLACE1001536, PLACE1001564, PLACE1001788, PLACE1001795, PLACE1001949, PLACE1002355, PLACE1003135, PLACE1003573, PLACE1003737, PLACE1004028, PLACE1004450, PLACE1004519, PLACE1005003, PLACE1005239, PLACE1005250, PLACE1005519, PLACE1005601, PLACE1005660, PLACE1005669, PLACE1005682, PLACE1005745, PLACE1006093, PLACE1006208, PLACE1006219, PLACE1006290, PLACE1006515, PLACE1006786, PLACE1006959, PLACE1007028, PLACE1007040, PLACE1007081, PLACE1007296, PLACE1007591, PLACE1007845, PLACE1007881, PLACE1007971, PLACE1008282, PLACE1008297, PLACE1008359, PLACE1008549, PLACE1008657, PLACE1008716, PLACE1008744, PLACE1008984, PLACE1009196,
PLACE1009279, PLACE1009546, PLACE1009600, PLACE1009735, PLACE1010011, PLACE1010081, PLACE1010713, PLACE1010784, PLACE1010827, PLACE1010968, PLACE1011407, PLACE1011824, PLACE3000213, SKNMC1000004, SKNMC1000082, THYRO1000036, THYRO1000196, THYRO1000400, THYRO1000580, THYRO1000678, THYRO1000776, THYRO1000846, THYRO1000956, THYRO1001071, THYRO1001102, THYRO1001266, THYRO1001327, THYRO1001471, Y79AA1000876, Y79AA1000959, Y79AA1001056, Y79AA1001062, Y79AA1001264, Y79AA1001795.

Genes exhibiting characteristic features in the expression thereof were selected by statistical analysis of these data. Two examples are shown below to describe the selection of genes of which expression is varied greatly among tissues. The β-actin gene is used frequently as a control in gene expression analysis. Genes of which expression is varied greatly among tissues as compared that of the β-actin gene were determined as follows. Specifically, sum of squared deviation was calculated in the signal intensity of β-actin observed in each tissue, which was divided by 7 degrees of freedom to determine a variance Sₐ². Next, sum of squared deviation was calculated in the signal intensity of a compared gene in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names:
clone: BNGH41000020, NT2RM4000761, Y79AA1002376.

Gene of OVARC1000037{heterogeneous nuclear ribonucleoprotein (hnRNP)} which expression is varied little. Genes of which expression is varied greatly among tissues as compared that of the OVARC1000037 gene were determined as follows. Specifically, sum of squared deviation was calculated in the signal intensity of β-actin observed in each tissue, which was divided by 7 degrees of freedom to determine a variance Sₐ². Next, sum of squared deviation was calculated in the signal intensity of a gene to be compared observed in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names:
clone: BNGH41000020, HEMBA1000300, OVARC1001030, NT2RM4000761, PLACE1000231, HEMBA1002316, NT2RP1000325, NT2RP1000271, PLACE1004648, HEMBA1005145, HEMBA1005929, NT2RP2002510, NT2RP2001538, NT2RP2002409, NT2RP2002188, NT2RP2001903, NT2RP2002533, NT2RP2002015, NT2RP2006269, NT2RP2004837, NT2RP2004205, NT2RP2005378, HEMBA1006357, HEMBB1000567, NT2RP2003940, NT2RP2004794, HEMBA1006912, NT2RP2004556, NT2RP2005163, NT2RP3000838, NT2RP3001271, PLACE2000118, NT2RP3000645, NT2RP3003076, HEMBB1002693, MAMMA1000046, NT2RP3003354, THYRO1001205, MAMMA1000614, MAMMA1000652, MAMMA1000810, THYRO1001242, MAMMA1001066, MAMMA1002224, MAMMA1001634, MAMMA1001094, MAMMA1002205, NT2RM1000855, NT2RM1000858, Y79AA1002376, NT2RM2000423.
Thus, characteristic features in the expression of a gene are illustrated by comparing and statistically analyzing the expression of many genes.

### Analysis of disease-associated genes

Non-enzymic protein glycation reaction is believed to be a cause of a variety of chronic diabetic complications. Accordingly, genes of which expression is elevated or decreased in a glycated protein-specific manner in the endothelial cells are associated with diabetic complications caused by glycated proteins. Vascular endothelial cells are affected with glycated proteins present in blood. Reaction products of non-enzymic protein glycation include amadori compound (glycated protein) as a mildly glycated protein and advanced glycation endproduct as a heavily glycated protein. Hence, a survey was carried out for genes of which expression levels are varied depending on the presence of these glycated proteins in endothelial cells. The mRNAs were extracted from endothelial cells that were cultured in the presence or absence of glycated protein. The mRNAs were converted into radiolabeled first strand cDNAs for preparing probes. The probes were hybridized to the above-mentioned DNA array. Signal of each DNA spot was detected by BAS2000 and analyzed by ArrayGauge (Fuji Photo Film Co., Ltd.).

Advanced glycation endproduct of bovine serum albumin was prepared as follows: bovine serum albumin (BSA; Sigma) was incubated in a phosphate buffer solution containing 50 mM glucose at 37°C for 8 weeks; and the resulting brownish BSA was dialyzed against a phosphate buffer solution.

Human normal pulmonary arterial endothelial cells (Cell Applications) were cultured in an Endothelial Cell Growth Medium (Cell Applications). The culture dish (Farcon) with the cells were incubated in a CO₂ incubator (37°C, 5% CO₂, in a humid atmosphere). When the cells were grown to be confluent in the dish, 250 µg/ml of bovine serum albumin (sigma), glycated bovine serum albumin (Sigma) or advanced glycation endproduct of bovine serum albumin was added thereto and the cells were incubated for 33 hours. The mRNA was extracted from the cells by using a FastTack™ 2.0 kit (Invitrogen). The labeling of hybridization probe was carried out by using the mRNA according to the same procedure as described above.

Table 185 shows the expression level of each cDNA in human pulmonary arterial endothelial cells cultured in a medium containing bovine serum albumin (sigma), glycated bovine serum albumin (Sigma) or advanced glycation endproduct of bovine serum albumin. Genes of which expression was detected in the endothelial cell are as follows:
BNGH41000020, BNGH41000087, HEMBA1000275, HEMBA1000300, HEMBA1000477, HEMBA1000634, HEMBA1000671, HEMBA1000713, HEMBA1000745, HEMBA1000835, HEMBA1000875, HEMBA1000940, HEMBA1001390, HEMBA1002131, HEMBA1002163, HEMBA1002164, HEMBA1002195, HEMBA1002227, HEMBA1002239, HEMBA1002420, HEMBA1002767, HEMBA1002992, HEMBA1003047, HEMBA1003120, HEMBA1003294, HEMBA1003315, HEMBA1003602, HEMBA1003945, HEMBA1004007, HEMBA1004067, HEMBA1004971, HEMBA1005145, HEMBA1005267, HEMBA1005337, HEMBA1005698, HEMBA1005929, HEMBA1005945, HEMBA1006171, HEMBA1006299, HEMBA1006335, HEMBA1006357, HEMBA1006430, HEMBA1006482, HEMBA1006658, HEMBA1006724, HEMBA1006770, HEMBA1006912, HEMBA1006960, HEMBA1007063, HEMBB1000447, HEMBB1000642, HEMBB1000905, HEMBB1001026, HEMBB1001048, HEMBB1001573, HEMBB1001847, HEMBB1001978, HEMBB1002041, HEMBB1002427, HEMBB1002663, HEMBB1002693, MAMMA1000102, MAMMA1000106, MAMMA1000204, MAMMA1000403, MAMMA1000449, MAMMA1000614, MAMMA1000652, MAMMA1000810, MAMMA1000814, MAMMA1000881, MAMMA1000986, MAMMA1001066, MAMMA1001237, MAMMA1001284, MAMMA1001344, MAMMA1001615, MAMMA1001634, MAMMA1001893, MAMMA1001901, MAMMA1001957, MAMMA1002087, MAMMA1002095, MAMMA1002165, MAMMA1002205, MAMMA1002224, MAMMA1002633, MAMMA1003126, NT2RM1000462, NT2RM1000580, NT2RM1000789, NT2RM1000855, NT2RM1000858,
NT2RM2000241, NT2RM2000306, NT2RM2000410, NT2RM2000423, NT2RM2000582, NT2RM2000589, NT2RM2000622, NT2RM2000773, NT2RM4000100, NT2RM4000198, NT2RM4000284, NT2RM4000444, NT2RM4000587, NT2RM4000593, NT2RM4000761, NT2RM4000997, NT2RM4001321, NT2RM4001325, NT2RM4001377, NT2RM4001735, NT2RM4001768, NT2RM4001843, NT2RP1000002, NT2RP1000181, NT2RP1000271, NT2RP1000300, NT2RP1000325, NT2RP1000465, NT2RP1000740, NT2RP1000981, NT2RP2000092, NT2RP2000240, NT2RP2000479, NT2RP2000533, NT2RP2000610, NT2RP2000616, NT2RP2000649, NT2RP2000663, NT2RP2000712, NT2RP2000903, NT2RP2001276, NT2RP2001388, NT2RP2001480, NT2RP2001495, NT2RP2001529, NT2RP2001538, NT2RP2001662, NT2RP2001878, NT2RP2001903, NT2RP2001948, NT2RP2001956, NT2RP2002015, NT2RP2002188, NT2RP2002232, NT2RP2002409, NT2RP2002510, NT2RP2002527, NT2RP2002533, NT2RP2002564, NT2RP2002721, NT2RP2002824, NT2RP2002942, NT2RP2002976, NT2RP2003138, NT2RP2003210, NT2RP2003390, NT2RP2003593, NT2RP2003599, NT2RP2003664, NT2RP2003931, NT2RP2003940, NT2RP2004069, NT2RP2004108, NT2RP2004179, NT2RP2004205, NT2RP2004495, NT2RP2004524, NT2RP2004556, NT2RP2004606, NT2RP2004648, NT2RP2004794, NT2RP2004837, NT2RP2004847, NT2RP2005027, NT2RP2005069, NT2RP2005163, NT2RP2005247, NT2RP2005378, NT2RP2005425, NT2RP2005535, NT2RP2005541, NT2RP2005632, NT2RP2005774,
NT2RP2005878, NT2RP2006099, NT2RP2006134, NT2RP2006269, NT2RP2006512, NT2RP3000011, NT2RP3000171, NT2RP3000201, NT2RP3000232, NT2RP3000436, NT2RP3000460, NT2RP3000645, NT2RP3000652, NT2RP3000676, NT2RP3000721, NT2RP3000818, NT2RP3000820, NT2RP3000838, NT2RP3000907, NT2RP3001159, NT2RP3001195, NT2RP3001240, NT2RP3001271, NT2RP3001388, NT2RP3001592, NT2RP3001738, NT2RP3001754, NT2RP3002015, NT2RP3002324, NT2RP3002342, NT2RP3002353, NT2RP3002409, NT2RP3002448, NT2RP3002721, NT2RP3002737, NT2RP3002738, NT2RP3002836, NT2RP3002900, NT2RP3003076, NT2RP3003354, NT2RP3003448, NT2RP3003473, NT2RP3003532, NT2RP3003614, NT2RP3003939, NT2RP3003963, NT2RP3004025, NT2RP3004067, NT2RP3004075, NT2RP3004083, NT2RP3004090, NT2RP3004119, NT2RP3004130, NT2RP3004133, NT2RP3004294, NT2RP3004309, NT2RP3004345, NT2RP3004374, NT2RP3004557, NT2RP3004625, NT2RP3004640, NT2RP3004647, NT2RP4000108, NT2RP4000634, NT2RP4001001, NT2RP4001009, NT2RP4001467, NT2RP4001877, NT2RP4001879, NT2RP4002187, NT2RP4002451, NT2RP4002715, OVARC1000003, OVARC1000090, OVARC1000105, OVARC1000137, OVARC1000208, OVARC1000298, OVARC1000313, OVARC1000331, OVARC1000410, OVARC1000439, OVARC1000553, OVARC1000775, OVARC1000853, OVARC1000873, OVARC1000916, OVARC1000956, OVARC1000995, OVARC1001030, OVARC1001049, OVARC1001086, OVARC1001132,
OVARC1001222, OVARC1001260, OVARC1001336, OVARC1001569, OVARC1001570, OVARC1001596, OVARC1001607, OVARC1001807, OVARC1001991, PLACE1000231, PLACE1000258, PLACE1000442, PLACE1000740, PLACE1000927, PLACE1001016, PLACE1001100, PLACE1001114, PLACE1001123, PLACE1001229, PLACE1001340, PLACE1001407, PLACE1001464, PLACE1001788, PLACE1001795, PLACE1001918, PLACE1002080, PLACE1002095, PLACE1002329, PLACE1002374, PLACE1002518, PLACE1002547, PLACE1002726, PLACE1002905, PLACE1002911, PLACE1002967, PLACE1003163, PLACE1003407, PLACE1003460, PLACE1003573, PLACE1003598, PLACE1003644, PLACE1003772, PLACE1003839, PLACE1003845, PLACE1004078, PLACE1004166, PLACE1004168, PLACE1004199, PLACE1004279, PLACE1004282, PLACE1004441, PLACE1004482, PLACE1004492, PLACE1004637, PLACE1004887, PLACE1005003, PLACE1005005, PLACE1005031, PLACE1005250, PLACE1005410, PLACE1005519, PLACE1005544, PLACE1005660, PLACE1005669, PLACE1005725, PLACE1005736, PLACE1005745, PLACE1005768, PLACE1005815, PLACE1006073, PLACE1006208, PLACE1006219, PLACE1006290, PLACE1006443, PLACE1006809, PLACE1006959, PLACE1007028, PLACE1007296, PLACE1007626, PLACE1007702, PLACE1007845, PLACE1008282, PLACE1008469, PLACE1008657, PLACE1009196, PLACE1009600, PLACE1009735, PLACE1010081, PLACE1010251, PLACE1010713, PLACE1011116, PLACE1011181,
PLACE1011236, PLACE1011516, PLACE1011708, PLACE1011824, PLACE1011978, PLACE2000118, PLACE3000181, SKNMC1000004, SKNMC1000014, THYRO1000584, THYRO1000866, THYRO1001113, THYRO1001128, THYRO1001205, THYRO1001242, THYRO1001495, THYRO1001523, THYRO1001529, THYRO1001593, THYRO1001608, THYRO1001702, THYRO1001725, THYRO1001770, THYRO1001803, Y79AA1000127, Y79AA1000207, Y79AA1000226, Y79AA1000270, Y79AA1000426, Y79AA1000777, Y79AA1000876, Y79AA1000888, Y79AA1000959, Y79AA1001013, Y79AA1001056, Y79AA1001090, Y79AA1001264, Y79AA1001272, Y79AA1001328, Y79AA1001427, Y79AA1001430, Y79AA1001530, Y79AA1001592, Y79AA1001727, Y79AA1001793, Y79AA1001799, Y79AA1001863, Y79AA1002022, Y79AA1002213, Y79AA1002373, Y79AA1002376, Y79AA1002381.

Signal ratios of EC_AGE_BSA to EC_BSA and of EC_glycated_BSA to EC_BSA were calculated for each gene. Genes with high signal ratios were selected. In the case of calculating the ratio of signal value of 40 or less to that of more than 40, such signal values were, for convenience, taken as 40 instead of the real values. When the ratio EC_AGE_BSA/EC_BSA is 2 or more, expression of the genes exhibiting such ratio is expected to be elevated due to advanced glycation endproduct of bovine serum albumin. The higher the value is, the higher the gene expression level is. When the ratio EC_AGE_BSA/EC_BSA ranges from 0.5 to 2, expression of the genes exhibiting such ratio is expected to be unaffected due to advanced glycation endproduct of bovine serum albumin. When the ratio EC_AGE_BSA/EC_BSA is less than 0.5, expression of the genes exhibiting such ratio value is expected to be decreased due to advanced glycation endproduct of bovine serum albumin. The lower the value is, the lower the gene expression level is.

Clone with EC_AGE_BSA/EC_BSA ratio of 2 or higher are as follows: NT2RP2001538, NT2RP4001001 and Y79AA1000967.

These cDNAs are associated with diabetes.

### Analysis of genes associated with neural cell differentiation

Genes involved in neural cell differentiation are useful for treating neurological diseases. It is possible that genes with varying expression levels in response to induction of cellular differentiation in neural cells are associated with neurological diseases.

A survey was performed for genes of which expression levels are varied in response to induction of differentiation (stimulation by retinoic acid (RA)) in cultured cells of a neural strain, NT2.

The NT2 cells were treated basically according to supplier's instruction manual. "Undifferentiated NT2 cells" means NT2 cells successively cultured in an Opti-MEM I (GIBCO-BRL; catalog No. 31985) containing 10%(v/v) fetal bovine serum and 1%(v/v) penicillin-streptomycin (GIBCO BRL). "NT2 cells cultured in the presence of retinoic acid" means the cells resulted from transferring undifferentiated NT2 cells into a retinoic acid-containing medium, which consists of D-MEM (GIBCO BRL; catalog No. 11965), 10%(v/v) fetal bovine serum, 1%(v/v) penicillin-streptomycin and 10 µM retinoic acid (GIBCO-BRL), and the subsequent successive culture therein for 5 weeks. "NT2 cells that were cultured in the presence of retinoic acid and then further cultured in the presence of cell-division inhibitor added" means NT2 cells resulted from transferring NT2 cells cultured in the presence of retinoic acid for 5 weeks into a cell-division inhibitor-containing medium, which consisted of D-MEM(GIBCO BRL; catalog No.11965), 10%(v/v) fetal bovine serum, 1%(v/v) penicillin-streptomycin, 10µM retinoic acid, 10µM FudR (5-fluoro-2'-deoxyuridine: GIBCO BRL), 10µM Urd (Uridine: GIBCO BRL) and 1µM araC (Cytosine β -D-Arabinofuranoside: GIBCO BRL), and the subsequence successive culture for 2 weeks. Each of the cells were treated with trypsin and then harvested. Total RNAs were extracted from the cells by using S.N.A.P.⁽™⁾ Total RNA Isolation kit (Invitrogen). The labeling of probe used for hybridization was carried out by using 10µg of the total RNA according to the same methods as described above. The data were obtained in triplicate (n=3). The data of signal value representing gene expression level in the cells in the presence of stimulation for inducing differentiation were compared with those in the absence of the stimulation. The comparison was performed by statistical treatment of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Tables 186-365 show the expression level of each cDNA in undifferentiated NT2 cells, NT2 cells cultured in the presence of RA, and NT2 cells that were cultured in the presence of RA and that were further cultured in the presence of cell-division inhibitor added.

Averaged signal values (M₁, M₂) and sample variances (s₁², s₂²) were calculated for each gene in each of the cells, and then, the pooled sample variances s² were obtained from the sample variances of the two types of cells to be compared. The t values were determined according to the following formula: t=(M1-M2)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information on an increase (+) or decrease (-) in the expression level of a gene in the treated cells when the level is compared with that of untreated undifferentiated cells.
Clones of which expression levels increased by RA are as follows: HEMBA1000121, HEMBA1000275, HEMBA1000300, HEMBA1000634, HEMBA1000671, HEMBA1000875, HEMBA1001184, HEMBA1001390, HEMBA1001886, HEMBA1002163, HEMBA1002227, HEMBA1002420, HEMBA1002421, HEMBA1003072, HEMBA1003120, HEMBA1003294, HEMBA1003497, HEMBA1004007, HEMBA1004110, HEMBA1004391, HEMBA1004444, HEMBA1005230, HEMBA1005246, HEMBA1005267, HEMBA1005489, HEMBA1005913, HEMBA1006299, HEMBA1006357, HEMBA1006517, HEMBA1006544, HEMBA1006658, HEMBA1006749, HEMBA1007063, HEMBA1007241, HEMBB1000447, HEMBB1000542, HEMBB1000567, HEMBB1000642, HEMBB1000668, HEMBB1001026, HEMBB1001847, HEMBB1002051, HEMBB1002120, HEMBB1002228, HEMBB1002693, MAMMA1000106, MAMMA1000141, MAMMA1000473, MAMMA1000528, MAMMA1000810, MAMMA1000881, MAMMA1001634, MAMMA1001957, MAMMA1002205, MAMMA1002224, NT2RM2000423, NT2RM2000497, NT2RM2000582, NT2RM2001126, NT2RM2001902, NT2RM4000198, NT2RM4000284, NT2RM4000593, NT2RM4001321, NT2RP1000002, NT2RP1000050, NT2RP1000181, NT2RP1000261, NT2RP1000465, NT2RP1000468, NT2RP1000579, NT2RP1000679, NT2RP2000092, NT2RP2000479, NT2RP2000610, NT2RP2000663, NT2RP2000694, NT2RP2000903, NT2RP2001388, NT2RP2001538, NT2RP2001878, NT2RP2002015, NT2RP2002304, NT2RP2002721, NT2RP2002824, NT2RP2002942, NT2RP2002974, NT2RP2002976, NT2RP2003179, NT2RP2003302, NT2RP2003383, NT2RP2003469, NT2RP2003664, NT2RP2003940, NT2RP2004069, NT2RP2004108, NT2RP2004524, NT2RP2004556, NT2RP2004670, NT2RP2005069, NT2RP2005247, NT2RP2005425, NT2RP2005463, NT2RP2005514, NT2RP2005535, NT2RP2005541, NT2RP2005774, NT2RP2005878, NT2RP2005883, NT2RP2005887, NT2RP2006099, NT2RP2006134, NT2RP3000011, NT2RP3000125, NT2RP3000171, NT2RP3000232, NT2RP3000460, NT2RP3000481, NT2RP3000652, NT2RP3000677, NT2RP3000818, NT2RP3000820, NT2RP3001044, NT2RP3001061, NT2RP3001170, NT2RP3001240, NT2RP3001322, NT2RP3001388, NT2RP3001542, NT2RP3001592, NT2RP3001976, NT2RP3002790, NT2RP3002900, NT2RP3002983, NT2RP3003000, NT2RP3003354, NT2RP3003532, NT2RP3003729, NT2RP3003874, NT2RP3003939, NT2RP3004025, NT2RP3004083, NT2RP3004090, NT2RP3004130, NT2RP3004202, NT2RP3004294, NT2RP3004640, NT2RP4000108, NT2RP4000634, NT2RP4002451, NT2RP4002715, OVARC1000090, OVARC1000208, OVARC1000275, OVARC1000553, OVARC1000775, OVARC1000853, OVARC1000873, OVARC1000916, OVARC1000995, OVARC1001030, OVARC1001049, OVARC1001132, OVARC1001596, OVARC1002178, PLACE1000258, PLACE1000442, PLACE1000927, PLACE1000986, PLACE1001100, PLACE1001123, PLACE1001795, PLACE1002518, PLACE1002547, PLACE1002967, PLACE1003407, PLACE1003428, PLACE1003644, PLACE1003839, PLACE1004078, PLACE1004441, PLACE1004450, PLACE1005669, PLACE1005682, PLACE1005736, PLACE1005768, PLACE1005815, PLACE1006073, PLACE1006208, PLACE1007296, PLACE1007626, PLACE1008282, PLACE1008984, PLACE1008985, PLACE1010445, PLACE1011708, PLACE1011978, PLACE4000455, SKNMC1000004, THYRO1000036, THYRO1000580, THYRO1000776, THYRO1000999, THYRO1001063, THYRO1001128, THYRO1001205, THYRO1001327, THYRO1001523, THYRO1001725, THYRO1001770, Y79AA1000207, Y79AA1000226, Y79AA1000270, Y79AA1001056, Y79AA1001062, Y79AA1001090, Y79AA1001727, Y79AA1002213, Y79AA1002381.
Clones of which expression levels decreased by RA are as follows: BNGH41000020, HEMBA1005070, NT2RP2005027, NT2RP3003473, Y79AA1002376.
Clones of which expression levels increase by RA/inhibitor are as follows: HEMBA1000128, HEMBA1000875, HEMBA1001390, HEMBA1002163, HEMBA1002227, HEMBA1002421, HEMBA1004391, HEMBA1004454, HEMBA1004785, HEMBA1005913, HEMBA1006171, HEMBA1006299, HEMBA1006335, HEMBA1006544, HEMBA1007241, HEMBB1000447, HEMBB1000668, MAMMA1000994, MAMMA1001344, NT2RM2000582, NT2RP1001004, NT2RP2000663, NT2RP2000694, NT2RP2000903, NT2RP2001388, NT2RP2002674, NT2RP2002974, NT2RP2003383, NT2RP2004069, NT2RP2004606, NT2RP2004837, NT2RP2005069, NT2RP2005425, NT2RP2005463, NT2RP2005541, NT2RP2005883, NT2RP2005887, NT2RP3000460, NT2RP3000838, NT2RP3001044, NT2RP3001240, NT2RP3001388, NT2RP3002721, NT2RP3002738, NT2RP3003469, NT2RP3004083, NT2RP3004130, NT2RP3004202, NT2RP3004294, NT2RP3004640, NT2RP4000108, NT2RP4002451, NT2RP4002715, OVARC1000275, OVARC1000467, OVARC1000553, OVARC1000853, OVARC1000873, OVARC1000916, OVARC1000995, OVARC1001030, OVARC1001222, OVARC1001596, OVARC1002058, OVARC1002178, PLACE1000927, PLACE1001123, PLACE1001407, PLACE1001464, PLACE1001564, PLACE1001795, PLACE1002547, PLACE1003407, PLACE1003644, PLACE1003845, PLACE1004441, PLACE1004482, PLACE1005410, PLACE1005601, PLACE1005725, PLACE1005736, PLACE1006093, PLACE1006219, PLACE1006290, PLACE1006716, PLACE1007296, PLACE1007626, PLACE1008359, PLACE1010968, PLACE1011364, PLACE1011824, THYRO1000678, THYRO1000776, THYRO1000999, THYRO1001113, THYRO1001237, THYRO1001523, Y79AA1000226, Y79AA1000888, Y79AA1001430.

Clones of which expression levels decrease by RA/inhibitor are as follows: HEMBA1000349, HEMBA1001297, HEMBA1001878, HEMBA1005070, HEMBA1006482, HEMBB1001959, NT2RM2001939, NT2RP1000981, NT2RP2001469, NT2RP3003473, OVARC1001132, PLACE1001655, Y79AA1000127, Y79AA1002381.

Clones of which expression levels increase in the presence of both RA and RA/inhibitor are as follows: HEMBA1000875, HEMBA1001390, HEMBA1002163, HEMBA1002227, HEMBA1002421, HEMBA1004391, HEMBA1005913, HEMBA1006299, HEMBA1006544, HEMBA1007241, HEMBB1000447, HEMBB1000668, NT2RM2000582, NT2RP2000663, NT2RP2000694, NT2RP2000903, NT2RP2001388, NT2RP2002974, NT2RP2003383, NT2RP2004069, NT2RP2005069, NT2RP2005425, NT2RP2005463, NT2RP2005541, NT2RP2005883, NT2RP2005887, NT2RP3000460, NT2RP3001044, NT2RP3001240, NT2RP3001388, NT2RP3004083, NT2RP3004130, NT2RP3004202, NT2RP3004294, NT2RP3004640, NT2RP4000108, NT2RP4002451, NT2RP4002715, OVARC1000275, OVARC1000553, OVARC1000853, OVARC1000873, OVARC1000916, OVARC1000995, OVARC1001030, OVARC1001596, OVARC1002178, PLACE1000927, PLACE1001123, PLACE1001795, PLACE1002547, PLACE1003407, PLACE1003644, PLACE1004441, PLACE1005736, PLACE1007296, PLACE1007626, THYRO1000776, THYRO1000999, THYRO1001523, Y79AA1000226.

Clones of which expression levels decrease in the presence of both RA and RA/inhibitor are as follows:HEMBA1005070 and NT2RP3003473.

These are neurological disease-associated clones.

### Analysis of rheumatoid arthritis-associated genes

The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center, http://www.rheuma-net.or.jp/). Recent studies have also revealed that tissue necrosis factor (TNF)- α participates in the onset (Current opinion in immunology 1999, 11, 657-662). When the expression of a gene exhibits responsiveness to the action of TNF on synovial cells, the gene is considered to be involved in rheumatoid arthritis.

A survey was performed for genes of which expression levels are varied in response to TNF-α in the primary^{•} cell culture of synovial tissue. The primary cultured cells of the smooth muscle (Cell Applications) were grown to be confluent in a culture dish, and then, human TNF-α (Boehringer-Mannheim) was added at a final concentration of 10 ng/ml thereto. The culture was further continued for 24 hours.

Total RNA was extracted from the cells by using S.N.A.P.⁽™⁾ Total RNA Isolation kit (Invitrogen). The labeling of probe used for hybridization was carried out by using 10µg of the total RNA according to the same methods as described above. The data were obtained in tripricate (n=3). The data of signal value representing gene expression level in the cells in the presence of TNF stimulation were compared with those in the absence of the stimulation. The comparison was performed by statistical treatment of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Table 366 shows the expression level of each cDNA in synovial cells cultured in the absence or presence of TNF.

Averaged signal values (M₁, M₂) and sample variances (s₁², s₂²) for each gene were calculated in each of the cells, and then, the pooled sample variances s² were obtained from the sample variances of the two types of cells to be compared. The t-values were determined according to the following formula: t=(M₁-M₂)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information of an increase (+) or decrease (-) in the expression level of a gene in the stimulated cells when the level is compared with that of unstimulated cells.

Clones of which expression levels are elevated by TNF-α are as follows:
BNGH41000020, HEMBA1000349, HEMBA1000634, HEMBA1000671, HEMBA1000835, HEMBA1000962, HEMBA1002178, HEMBA1002195, HEMBA1002239, HEMBA1002420, HEMBA1002524, HEMBA1002992, HEMBA1003315, HEMBA1003392, HEMBA1003487, HEMBA1003602, HEMBA1004067, HEMBA1004797, HEMBA1005337, HEMBA1005489, HEMBA1006916, HEMBB1000668, HEMBB1000905, HEMBB1001547, HEMBB1001573, HEMBB1002041, HEMBB1002663, MAMMA1000652, MAMMA1000810, MAMMA1001634, MAMMA1002091, MAMMA1002234, NT2RM2000306, NT2RM4000417, NT2RP1000002, NT2RP1000181, NT2RP1000740, NT2RP2000694, NT2RP2001921, NT2RP2002527, NT2RP2004495, NT2RP2004606, NT2RP2005163, NT2RP2005463, NT2RP2006134, NT2RP3000171, NT2RP3000652, NT2RP3001195, NT2RP3001976, NT2RP3003473, NT2RP3003874, NT2RP3004090, NT2RP3004294, NT2RP3004557, NT2RP3004647, NT2RP4000108, NT2RP4001001, NT2RP4001877, OVARC1000090, OVARC1000105, OVARC1000275, OVARC1000439, OVARC1001607, PLACE1000740, PLACE1000927, PLACE1001016, PLACE1001100, PLACE1001464, PLACE1001500, PLACE1001918, PLACE1002095, PLACE1002547, PLACE1003644, PLACE1004519, PLACE1005031, PLACE1005410, PLACE1005736, PLACE1006219, PLACE1006809, PLACE1008716, PLACE1010081, THYRO1001770, Y79AA1000127, Y79AA1000207, Y79AA1000270, Y79AA1000876, Y79AA1001013, Y79AA1001264, Y79AA1001272, Y79AA1001328, Y79AA1001430, Y79AA1001530, Y79AA1001799.

Clones of which expression levels decrease by TNF-α are as follows:
NT2RM4000326, NT2RP1000300, NT2RP2000514, NT2RP2001755, NT2RP2006042, NT2RP3000481, NT2RP3002790.

These are rheumatoid arthritis-associated clones.

### EXAMPLE 16

### Search for a signal sequence, transmembrane region and functional domain in deduced amino acid sequences

The deduced amino acid sequences from the full-length nucleotide sequences were examined to predict the presence of a signal sequence in their amino-termini as well as the presence of a transmembrane region. The amino acid sequences were also searched for a protein functional domain (motif). The examinations for a signal sequence in the amino-terminus, for a transmembrane region and for a functional domain were performed by using PSORT [K. Nakai & M. Kanehisa, Genomics, 14:897-911 (1992)], SOSUI [T. Hirokawa et al., Bioinformatics, 14:378-379 (1998)] (Mitsui Knowledge Industry Co., Ltd.) and Pfam (http://www.sanger.ac.uk/Software/Pfam/index.shtml), respectively. When the presence of a signal sequence or a transmembrane region in the amino-terminus was predicted in the amino acid sequence by PSORT or SOSUI, the protein was predicted to be a secretory protein or a transmembrane protein. When the amino acid sequence matched a functional domain in the Pfam search for a functional domain, the function of the protein is predictable based on the matching data, for example, by referring to the functional categories in PROSITE (http://www.expasy.ch/cgi-bin/prosite-list.pl). The functional domain search can be performed by using PROSITE instead of Pfam.

Search results obtained by using the respective software programs are indicated below.

Clones whose deduced amino acid sequences were predicted to have signal sequences by PSORT search are as follows:
HEMBA1000713, HEMBA1002420, HEMBA1002421, HEMBA1003101, HEMBA1004110, HEMBA1006707, HEMBA1006902, HEMBB1001530, HEMBB1001573, HEMBB1001978, HEMBB1002162, HEMBB1002245, HEMBB1002427, MAMMA1000102, MAMMA1000118, MAMMA1000457, MAMMA1001043, MAMMA1001344, MAMMA1001893, MAMMA1002070, MAMMA1002165, MAMMA1002633, NT2RM2000241, NT2RM2000410, NT2RM2001941, NT2RM4001325, NT2RP1001563, NT2RP2001495, NT2RP2002063, NT2RP2002721, NT2RP2003383, NT2RP2003593, NT2RP2003655, NT2RP2003664, NT2RP2004179, NT2RP2004205, NT2RP2004524, NT2RP2005463, NT2RP3000460, NT2RP3001012, NT2RP3001858, NT2RP3002836, NT2RP3003076, NT2RP3003532, NT2RP3004133, NT2RP3004309, NT2RP4001467, NT2RP4002451, OVARC1000298, OVARC1000811, PLACE1000231, PLACE1000740, PLACE1001183, PLACE1001536, PLACE1001564, PLACE1002095, PLACE1002374, PLACE1003839, PLACE1004482, PLACE1005005, PLACE1005250, PLACE1005383, PLACE1005410, PLACE1005544, PLACE1005569, PLACE1006093, PLACE1006277, PLACE1006809, PLACE1007626, PLACE1008359, PLACE1009067, PLACE1010251, PLACE1011236, SKNMC1000004, SKNMC1000014, THYRO1000099, THYRO1000196, THYRO1001237, THYRO1001327, THYRO1001523, THYRO1001702, THYRO1001725, Y79AA1000426, Y79AA1000521, Y79AA1000959, Y79AA1001013, Y79AA1001264, Y79AA1001328, Y79AA1001427, Y79AA1001430, Y79AA1001795, Y79AA1001803, Y79AA1002022,
Clones whose deduced amino acid sequences were predicted to have transmembrane regions by SOSUI search are as follows: BNGH41000091, HEMBA1000121, HEMBA1000349, HEMBA1000477, HEMBA1000713, HEMBA1000940, HEMBA1000962, HEMBA1001221, HEMBA1001228, HEMBA1001621, HEMBA1002167, HEMBA1002195, HEMBA1002227, HEMBA1002421, HEMBA1003101, HEMBA1003392, HEMBA1003530, HEMBA1003732, HEMBA1003945, HEMBA1004391, HEMBA1004454, HEMBA1004797, HEMBA1004982, HEMBA1005449, HEMBA1005522, HEMBA1005545, HEMBA1005698, HEMBA1006171, HEMBA1006299, HEMBA1006311, HEMBA1006335, HEMBA1006357, HEMBA1006430, HEMBA1006724, HEMBA1006960, HEMBB1000407, HEMBB1000447, HEMBB1000567, HEMBB1000679, HEMBB1000905, HEMBB1001026, HEMBB1001407, HEMBB1001573, HEMBB1001978, HEMBB1002041, HEMBB1002162, HEMBB1002245, HEMBB1002427, HEMBB1002693, MAMMA1000102, MAMMA1000106, MAMMA1000118, MAMMA1000141, MAMMA1000204, MAMMA1000226, MAMMA1000457, MAMMA1000473, MAMMA1000591, MAMMA1000681, MAMMA1000810, MAMMA1000986, MAMMA1001043, MAMMA1001141, MAMMA1001237, MAMMA1001344, MAMMA1001893, MAMMA1001957, MAMMA1001978, MAMMA1002070, MAMMA1002091, MAMMA1002095, MAMMA1002633, NT2RM1000580, NT2RM1000855, NT2RM1000858, NT2RM2000410, NT2RM2000565, NT2RM2001626, NT2RM2001939, NT2RM2001941, NT2RM4000444, NT2RM4000587, NT2RM4000648, NT2RM4000997, NT2RM4001325, NT2RM4001735, NT2RM4001768, NT2RM4002352, NT2RP1000050, NT2RP1000181, NT2RP1000261, NT2RP1000300, NT2RP1000448, NT2RP1000551, NT2RP1000613, NT2RP1000981, NT2RP1001563, NT2RP2000479, NT2RP2000533, NT2RP2000649,
NT2RP2000663, NT2RP2000694, NT2RP2000818, NT2RP2000903, NT2RP2001200, NT2RP2001276, NT2RP2001495, NT2RP2001915, NT2RP2001956, NT2RP2002188, NT2RP2002232, NT2RP2002527, NT2RP2002533, NT2RP2002721, NT2RP2002824, NT2RP2002942, NT2RP2002976, NT2RP2003042, NT2RP2003390, NT2RP2003469, NT2RP2003593, NT2RP2003655, NT2RP2003664, NT2RP2003950, NT2RP2004179, NT2RP2004205, NT2RP2004495, NT2RP2004524, NT2RP2004556, NT2RP2004606, NT2RP2004648, NT2RP2004794, NT2RP2005163, NT2RP2005181, NT2RP2005463, NT2RP2005597, NT2RP2005666, NT2RP2005883, NT2RP2005994, NT2RP2006004, NT2RP2006269, NT2RP2006512, NT2RP2006580, NT2RP3000169, NT2RP3000171, NT2RP3000304, NT2RP3000460, NT2RP3000616, NT2RP3000721, NT2RP3000818, NT2RP3000907, NT2RP3000921, NT2RP3001159, NT2RP3001195, NT2RP3001240, NT2RP3001271, NT2RP3001322, NT2RP3001388, NT2RP3001560, NT2RP3001592, NT2RP3001650, NT2RP3001738, NT2RP3002015, NT2RP3002311, NT2RP3002342, NT2RP3002411, NT2RP3002790, NT2RP3002836, NT2RP3002900, NT2RP3002958, NT2RP3003000, NT2RP3003354, NT2RP3003532, NT2RP3003535, NT2RP3003614, NT2RP3004025, NT2RP3004075, NT2RP3004083, NT2RP3004090, NT2RP3004130, NT2RP3004294, NT2RP3004309, NT2RP3004345, NT2RP3004406, NT2RP3004481, NT2RP3004552, NT2RP4001001, NT2RP4001009, NT2RP4001467, NT2RP4001879, NT2RP4002187, NT2RP4002451, NT2RP4002750, OVARC1000003, OVARC1000105, OVARC1000307, OVARC1000439, OVARC1000553, OVARC1001030, OVARC1001336,
OVARC1001570, PLACE1000231, PLACE1000560, PLACE1000740, PLACE1000912, PLACE1000914, PLACE1000927, PLACE1001016, PLACE1001183, PLACE1001231, PLACE1001401, PLACE1001407, PLACE1001464, PLACE1001536, PLACE1001564, PLACE1001655, PLACE1001836, PLACE1001918, PLACE1001949, PLACE1002518, PLACE1002726, PLACE1002967, PLACE1003573, PLACE1003737, PLACE1003839, PLACE1003845, PLACE1003852, PLACE1004279, PLACE1004282, PLACE1004441, PLACE1004637, PLACE1004648, PLACE1004816, PLACE1004887, PLACE1005003, PLACE1005005, PLACE1005410, PLACE1005544, PLACE1005569, PLACE1005660, PLACE1005725, PLACE1005745, PLACE1005927, PLACE1006290, PLACE1006443, PLACE1006959, PLACE1007096, PLACE1007296, PLACE1007626, PLACE1007881, PLACE1008359, PLACE1008469, PLACE1008716, PLACE1008985, PLACE1009196, PLACE1009279, PLACE1009527, PLACE1009546, PLACE1009600, PLACE1010011, PLACE1010078, PLACE1010445, PLACE1010713, PLACE1010784, PLACE1010968, PLACE1011236, PLACE1011516, PLACE3000181, THYRO1000400, THYRO1000678, THYRO1000776, THYRO1000956, THYRO1001102, THYRO1001113, THYRO1001205, THYRO1001237, THYRO1001242, THYRO1001266, THYRO1001327, THYRO1001478, THYRO1001523, THYRO1001641, THYRO1001702, THYRO1001725, Y79AA1000207, Y79AA1000226, Y79AA1000270, Y79AA1000521, Y79AA1000888, Y79AA1001013, Y79AA1001212, Y79AA1001264, Y79AA1001328, Y79AA1001426, Y79AA1001427, Y79AA1001727, Y79AA1001787, Y79AA1001795, Y79AA1001803, Y79AA1002058, Y79AA1002129, Y79AA1002213, Y79AA1002373,

Names of clones whose deduced amino acid sequences were predicted to have functional domains by Pfam search, and names of the matched functional domains are shown below. When multiple functional domains matched a clone, each domain name was indicated, separated by a double-slash mark, //.
HEMBA1000006//Src homology domain 3
HEMBA1000128//SCP-like extracellular Proteins
HEMBA1000349//ABC transporters
HEMBA1000462//RNA recognition motif. (aka RRM, RBD, or RNP domain)
HEMBA1000590//EGF-like domain//von Willebrand factor type A domain HEMBA1000671//Zinc finger, C2H2 type
HEMBA1000732//EGF-like domain
HEMBA1000940//Connexin
HEMBA1001221//EGF-like domain//Kazal-type serine protease inhibitor domain
HEMBA1001621//7 transmembrane receptor (rhodopsin family)
HEMBA1001878//WD domain, G-beta repeats
HEMBA1002048//Zinc finger, C2H2 type
HEMBA1002167//Carboxylesterases
HEMBA1002551//WD domain, G-beta repeats
HEMBA1002992//Ubiquitin family
HEMBA1003047//CUB domain
HEMBA1003120//Zinc finger, C2H2 type
HEMBA1003230//EGF-like domain
HEMBA1003392//Low-density lipoprotein receptor domain class A
HEMBA1003497//Zinc finger, C2H2 type
HEMBA1004250//Cadherin
HEMBA1004391//Fibronectin type III domain//IG superfamily
HEMBA1004454//4 transmembrane segments integral membrane proteins
HEMBA1004785//'chromo' (CHRromatin Organization Modifier) domain
HEMBA1005246//Zinc finger, C2H2 type
HEMBA1005267//Ank repeat
HEMBA1005545//7 transmembrane receptor (rhodopsin family)
HEMBA1005929//Eukaryotic protein kinase domain
HEMBA1005945//Mitochondrial carrier proteins
HEMBA1006572//Zinc finger, C2H2 type
HEMBA1006707//EGF-like domain//von Willebrand factor type A domain
HEMBA1006749//EGF-like domain//von Willebrand factor type A domain
HEMBA1006770//RNA recognition motif. (aka RRM, RBD, or RNP domain)
HEMBA1006902//EGF-like domain//von Willebrand factor type A domain
HEMBB1000106//Zinc finger, CCHC class
HEMBB1000668//WD domain, G-beta repeats
HEMBB1000881//Thrombospondin type 1 domain
HEMBB1000905//WD domain, G-beta repeats
HEMBB1002041//EGF-like domain//Kazal-type serine protease inhibitor domain
HEMBB1002245//IG superfamily
HEMBB1002302//Zinc finger, CCHC class
HEMBB1002465//Acyl-CoA dehydrogenases
HEMBB1002661//Helix-loop-helix DNA-binding domain
MAMMA1000204//C2 domain
MAMMA1000457//FAD/NAD-binding domain in oxidoreductases
MAMMA1000681//7 transmembrane receptor (rhodopsin family)
MAMMA1000881//Eukaryotic protein kinase domain//Protein kinase C terminal domain
MAMMA1001150//Phorbol esters / diacylglycerol binding domain//Eukaryotic protein kinase domain
MAMMA1001310//WD domain, G-beta repeats
MAMMA1001532//Zinc finger, C2H2 type
MAMMA1001615//Helix-loop-helix DNA-binding domain
MAMMA1002070//Kringle domain
MAMMA1002080//Ras family (contains ATP/GTP binding P-loop)
MAMMA1002095//E1-E2 ATPases
MAMMA1002165//Insulin-like growth factor binding proteins
NT2RM1000789//HMG (high mobility group) box
NT2RM1000855//eubacterial secY protein
NT2RM1000899//Mitochondrial carrier proteins
NT2RM2000589//PH (pleckstrin homology) domain
NT2RM2000632//Helicases conserved C-terminal domain
NT2RM2001792//Fibrinogen beta and gamma chains, C-terminal globular domain
NT2RM2001902//Eukaryotic protein kinase domain
NT2RM2001939//7 transmembrane receptor (rhodopsin family)
NT2RM2001941//7 transmembrane receptor (rhodopsin family)
NT2RM4000284//Class I Histocompatibility antigen, domains alpha 1 and 2
NT2RM4000326//Zinc finger, C2H2 type
NT2RM4000417//C2 domain
NT2RM4000444//ABC transporters
NT2RM4001377//PH (pleckstrin homology) domain
NT2RM4001768//Alcohol/other dehydrogenases, short chain type
NT2RM4002352//Low-density lipoprotein receptor domain class A
NT2RP1000181//Heme-binding domain in cytochrome b5 and oxidoreductases
NT2RP1000271//Zinc finger, C2H2 type
NT2RP1000325//Mitochondrial carrier proteins
NT2RP1000613//Eukaryotic-type carbonic anhydrases
NT2RP1000981//IG superfamily
NT2RP1001004//Thrombospondin type 1 domain
NT2RP1001020//Eukaryotic protein kinase domain
NT2RP1001031//WD domain, G-beta repeats
NT2RP1001563//EGF-like domain//Lectin C-type domain short and long forms//SCP-like extracellular Proteins
NT2RP2000092//Zinc finger, C2H2 type
NT2RP2000514//Fibronectin type III domain//IG superfamily
NT2RP2000649//Zinc-binding metalloprotease domain
NT2RP2000712//Zinc finger, C2H2 type
NT2RP2000739//Zinc finger, C2H2 type
NT2RP2001514//E1-E2 ATPases
NT2RP2001529//Eukaryotic protein kinase domain
NT2RP2001755//Thrombospondin type 1 domain
NT2RP2001769//Eukaryotic protein kinase domain
NT2RP2002188//Carboxylesterases
NT2RP2002527//Heme-binding domain in cytochrome b5 and oxidoreductases
NT2RP2002564//Zinc finger, C2H2 type
NT2RP2002942//IG superfamily
NT2RP2003179//Eukaryotic protein kinase domain
NT2RP2003302//Zinc finger, C2H2 type
NT2RP2003390//DnaJ, prokaryotic heat shock protein
NT2RP2003469//Sugar (and other) transporters
NT2RP2003545//Eukaryotic protein kinase domain
NT2RP2003593//Thioredoxins
NT2RP2003940//Zinc finger, C2H2 type
NT2RP2004108//Zinc finger, C2H2 type
NT2RP2004205//IG superfamily
NT2RP2004670//Eukaryotic protein kinase domain
NT2RP2004847//Zinc finger, C2H2 type
NT2RP2005181//Amino acid permeases
NT2RP2005247//Zinc finger, C3HC4 type (RING finger)
NT2RP2005391//Fibronectin type III domain
NT2RP2005535//Zinc finger, C2H2 type
NT2RP2005774//Zinc finger, C2H2 type
NT2RP2005878//Alcohol/other dehydrogenases, short chain type
NT2RP2005941//Homeobox domain//'Paired box' domain
NT2RP2006004//Fibronectin type III domain
NT2RP3000011//WD domain, G-beta repeats
NT2RP3000022//Eukaryotic protein kinase domain
NT2RP3000063//Zinc finger, C2H2 type
NT2RP3000148//Zinc finger, C2H2 type
NT2RP3000172//Eukaryotic protein kinase domain
NT2RP3000201//Eukaryotic protein kinase domain
NT2RP3000232//Zinc finger, C2H2 type
NT2RP3000304//Low-density lipoprotein receptor domain class A//Low-density lipoprotein receptor domain class B
NT2RP3000436//Thioredoxins
NT2RP3000460//eubacterial secY protein
NT2RP3000616//Fibronectin type III domain
NT2RP3000652//Zinc finger, C2H2 type
NT2RP3000676//Mitochondrial carrier proteins
NT2RP3000789//KH domain family of RNA binding proteins
NT2RP3000820//WD domain, G-beta repeats
NT2RP3000838//PH (pleckstrin homology) domain
NT2RP3000907//E1-E2 ATPases
NT2RP3000921//IG superfamily
NT2RP3001195//Sugar (and other) transporters
NT2RP3001240//eubacterial secY protein
NT2RP3001388//C2 domain
NT2RP3001650//CUB domain//Low-density lipoprotein receptor domain class A
NT2RP3001738//Heme-binding domain in cytochrome b5 and oxidoreductases
NT2RP3001976//Zinc finger, C2H2 type
NT2RP3002281//RNA recognition motif. (aka RRM, RBD, or RNP domain)
NT2RP3002411//Alcohol/other dehydrogenases, short chain type
NT2RP3002721//Citrate synthase
NT2RP3003000//Ion transport proteins
NT2RP3003527//Eukaryotic protein kinase domain
NT2RP3003535//TPR Domain
NT2RP3003849//C2 domain
NT2RP3004067//Src homology domain 3
NT2RP3004090//Zinc finger, C3HC4 type (RING finger)
NT2RP3004481//IG superfamily
NT2RP3004552//CUB domain//Sushi domain
NT2RP3004647//Mitochondrial carrier proteins
NT2RP4000108//Intermediate filament proteins
NT2RP4000634//Eukaryotic protein kinase domain
NT2RP4000962//Eukaryotic protein kinase domain
NT2RP4001009//Zinc-binding metalloprotease domain
NT2RP4001877//RNA recognition motif. (aka RRM, RBD, or RNP domain)
NT2RP4002187//Alcohol/other dehydrogenases, short chain type
NT2RP4002750//Amino acid permeases
OVARC1000105//Ubiquitin-conjugating enzymes
OVARC1000255//Eukaryotic protein kinase domain
OVARC1000313//Thioredoxins
OVARC1000410//Fibrinogen beta and gamma chains, C-terminal globular domain
OVARC1000529//Eukaryotic protein kinase domain
OVARC1000811//CUB domain//Kringle domain
OVARC1000916//Eukaryotic protein kinase domain
OVARC1001049//Helix-loop-helix DNA-binding domain
OVARC1001338//Eukaryotic protein kinase domain
OVARC1001569//Eukaryotic protein kinase domain
OVARC1001570//Eukaryotic aspartyl proteases
PLACE1000231//WAP-type (Whey Acidic Protein) 'four-disulfide core'
PLACE1000258//Zinc finger, C2H2 type
PLACE1000740//EGF-like domain
PLACE1000907//Zinc finger, C2H2 type
PLACE1001016//lon transport proteins
PLACE1001500//Helicases conserved C-terminal domain
PLACE1001655//lon transport proteins
PLACE1001795//SCP-like extracellular Proteins
PLACE1001949//E1-E2 ATPases
PLACE1002329//Src homology domain 3
PLACE1002355//Alpha-2-macroglobulin family//Kazal-type serine protease inhibitor domain
PLACE1002374//Cysteine proteases
PLACE1002518//Zinc finger, C3HC4 type (RING finger)
PLACE1002911//IG superfamily
PLACE1003135//Eukaryotic protein kinase domain
PLACE1003163//Enoyl-CoA hydratase/isomerase
PLACE1003573//Lectin C-type domain short and long forms
PLACE1004166//Bromodomain
PLACE1004305//Ras family (contains ATP/GTP binding P-loop)
PLACE1004441//7 transmembrane receptor (rhodopsin family)
PLACE1004520//IG superfamily
PLACE1004816//Fibrinogen beta and gamma chains, C-terminal globular domain
PLACE1004887//Zinc finger, C3HC4 type (RING finger)
PLACE1005003//Trypsin
PLACE1005383//EGF-like domain
PLACE1005410//eubacterial secY protein
PLACE1005426//IG superfamily
PLACE1005519//Eukaryotic protein kinase domain
PLACE1005539//Heat shock hsp20 proteins
PLACE1005544//IG superfamily
PLACE1005569//IG superfamily
PLACE1005682//Zinc finger, C3HC4 type (RING finger)
PLACE1005736//PH (pleckstrin homology) domain
PLACE1006079//Homeobox domain
PLACE1006716//C1q domain
PLACE1008282//Eukaryotic protein kinase domain
PLACE1008549//Ets-domain
PLACE1008744//EGF-like domain//Sushi domain
PLACE1009067//Src homology domain 3
PLACE1010081//Eukaryotic protein kinase domain
PLACE1010251//EGF-like domain
PLACE1010713//Alcohol/other dehydrogenases, short chain type
PLACE1010784//7 transmembrane receptor (rhodopsin family)
PLACE1010968//Fibronectin type III domain
PLACE1011181//ATPases associated with various cellular activities (AAA)
PLACE1011364//Eukaryotic protein kinase domain
PLACE1011407//Zinc finger, C2H2 type
PLACE1011708//CUB domain
PLACE1011824//Eukaryotic protein kinase domain
PLACE1011978//Zinc finger, C2H2 type
PLACE3000181//Cadherin
PLACE3000213//Sushi domain
PLACE4000354//EGF-like domain//Sushi domain
SKNMC1000082//Mitochondrial carrier proteins
THYRO1000196//Cadherin
THYRO1000580//Zinc finger, C2H2 type
THYRO1000678//Connexin
THYRO1000795//Mitochondrial carrier proteins
THYRO1000956//7 transmembrane receptor (rhodopsin family)
THYRO1001113//C2 domain
THYRO1001266//Sodium:solute symporter family
THYRO1001457//Phorbol esters / diacylglycerol binding domain//Eukaryotic prote i n kinase domain
THYRO1001478//EF hand
THYRO1001593//Eukaryotic protein kinase domain
THYRO1001700//Eukaryotic protein kinase domain
THYRO1001770//Eukaryotic protein kinase domain
Y79AA1000030//WW/rsp5/WWP domain containing proteins
Y79AA1000426//Transforming growth factor beta like domain
Y79AA1000777//WD domain, G-beta repeats
Y79AA1000876//Thioredoxins
Y79AA1000967//Eukaryotic protein kinase domain
Y79AA1001090//Ank repeat
Y79AA1001264//DnaJ, prokaryotic heat shock protein
Y79AA1001328//EGF-like domain
Y79AA1001427//FAD/NAD-binding domain in oxidoreductases
Y79AA1001523//Bromodomain//Zinc finger, C3HC4 type (RING finger)
Y79AA1001530//Tubulin
Y79AA1001727//IG superfamily
Y79AA1001787//E1-E2 ATPases
Y79AA1001799//Mitochondrial carrier proteins
Y79AA1002022//IG superfamily
Y79AA1002381//Eukaryotic protein kinase domain

### EXAMPLE 17

### Functional categories based on the full-length nucleotide sequences

Prediction of functions of proteins encoded by the clones and the categorization thereof were performed based on the results of homology search (see homology search result 10) of the databases, GenBank, Swiss-Prot and UniGene for the full-length nucleotide sequences of 826 clones as well as based on the results of domain search (see Example 16) of the deduced amino acid sequences encoded by the full-length nucleotide sequences. (HEMBA1005337, NT2RM1000407, NT2RM2001767, and NT2RP3003939 were excluded because of the absence of full-length sequence.)

There are 611 clones that presumably encode proteins belonging to any of categories of secretory and/or membrane proteins, glycoprotein-associated proteins, signal transduction-associated proteins, transcription-associated proteins and disease-associated proteins.

The clones presumably encoding proteins categorized into secretory and/or membrane proteins are those which matched the full-length sequences of Swiss-Prot database with keywords "growth factor", "cytokine", "hormone", "signal", "transmembrane", "membrane", "extracellular matrix", "receptor", "G-protein coupled receptor", "ionic channel", "voltage-gated channel", "calcium channel", "cell adhesion", "collagen" or "connective tissue"; those which matched the data, suggesting that the proteins are secretory and/or membrane proteins; or those which matched the full-length sequences of GenBank or UniGene database with similar description; and, further, those predicted to have an N-terminal signal sequence or a transmembrane region as a result of domain search for the amino acid sequences deduced from the full-length nucleotide sequences.

The clones presumably encoding proteins categorized into glycoprotein-associated proteins are those which matched the full-length sequences of Swiss-Prot database with the keywords "glycoprotein"; those which matched the data, suggesting that the proteins are glycoprotein; or those which matched the full-length sequences of GenBank or UniGene database.

The clones presumably encoding proteins categorized into signal transduction-associated proteins are those which matched the full-length sequences of Swiss-Prot database with the keywords "serine/threonine-protein kinase", "tyrosine-protein kinase" or "SH3 domain"; those which matched the data, suggesting that the proteins are signal transduction-associated proteins (for example, "ADP-ribosylation factor"); or those which matched the the full-length sequences of GenBank or UniGene database with similar description.

The clones presumably encoding proteins categorized into transcription-associated proteins are those which matched the full-length sequences of Swiss-Prot database with the keywords "transcription regulation", "zinc finger" or "homeobox"; those which matched the data, suggesting that the proteins are transcription-associated proteins; or those which matched the full-length sequences of GenBank or UniGene database with similar description.

The clones presumably encoding proteins categorized into disease-associated proteins are those which matched the full-length sequences of Swiss-Prot database with the keywords "disease mutation" or "syndrome"; those which matched the data, suggesting that the proteins are disease-associated proteins; or those which matched the full-length sequences of Swiss-Prot database and GenBank or UniGene database where the matched sequences are those of genes or proteins which had been deposited in the database of Online Mendelian Inheritance in Man (OMIM) (http://www.ncbi.nlm.nih.gov/Omim/), which is a database of human genes and diseases.

The following 437 clones were categorized into secretory and/or membrane proteins.
BNGH41000020, BNGH41000087, BNGH41000091, HEMBA1000121, HEMBA1000128, HEMBA1000349, HEMBA1000477, HEMBA1000590, HEMBA1000713, HEMBA1000732, HEMBA1000745, HEMBA1000835, HEMBA1000940, HEMBA1000962, HEMBA1001221, HEMBA1001228, HEMBA1001621, HEMBA1002131, HEMBA1002163, HEMBA1002167, HEMBA1002178, HEMBA1002195, HEMBA1002227, HEMBA1002420, HEMBA1002421, HEMBA1002767, HEMBA1003047, HEMBA1003101, HEMBA1003230, HEMBA1003392, HEMBA1003530, HEMBA1003602, HEMBA1003732, HEMBA1003945, HEMBA1004110, HEMBA1004250, HEMBA1004391, HEMBA1004444, HEMBA1004454, HEMBA1004505, HEMBA1004797, HEMBA1004982, HEMBA1005070, HEMBA1005449, HEMBA1005522, HEMBA1005545, HEMBA1005698, HEMBA1005945, HEMBA1006171, HEMBA1006299, HEMBA1006311, HEMBA1006335, HEMBA1006357, HEMBA1006430, HEMBA1006482, HEMBA1006707, HEMBA1006724, HEMBA1006749, HEMBA1006902, HEMBA1006960, HEMBA1007241, HEMBB1000407, HEMBB1000447, HEMBB1000567, HEMBB1000679, HEMBB1000881, HEMBB1001026, HEMBB1001048, HEMBB1001407, HEMBB1001530, HEMBB1001573, HEMBB1001847, HEMBB1001978, HEMBB1002041, HEMBB1002162, HEMBB1002245, HEMBB1002427, HEMBB1002693, MAMMA1000102, MAMMA1000106, MAMMA1000118, MAMMA1000141, MAMMA1000204, MAMMA1000226, MAMMA1000457, MAMMA1000473, MAMMA1000496, MAMMA1000591, MAMMA1000681, MAMMA1000810, MAMMA1000986, MAMMA1000994, MAMMA1001043, MAMMA1001141, MAMMA1001237, MAMMA1001344, MAMMA1001418, MAMMA1001893, MAMMA1001957, MAMMA1001978,
MAMMA1002070, MAMMA1002091, MAMMA1002095, MAMMA1002165, MAMMA1002234, MAMMA1002586, MAMMA1002633, MAMMA1003126, NT2RM1000462, NT2RM1000542, NT2RM1000580, NT2RM1000855, NT2RM1000858, NT2RM1000899, NT2RM2000241, NT2RM2000410, NT2RM2000423, NT2RM2000565, NT2RM2001626, NT2RM2001792, NT2RM2001939, NT2RM2001941, NT2RM4000198, NT2RM4000284, NT2RM4000417, NT2RM4000444, NT2RM4000587, NT2RM4000593, NT2RM4000648, NT2RM4000761, NT2RM4000997, NT2RM4001325, NT2RM4001735, NT2RM4001768, NT2RM4001843, NT2RM4002352, NT2RP1000050, NT2RP1000181, NT2RP1000261, NT2RP1000300, NT2RP1000325, NT2RP1000448, NT2RP1000551, NT2RP1000613, NT2RP1000981, NT2RP1001004, NT2RP1001563, NT2RP2000479, NT2RP2000533, NT2RP2000616, NT2RP2000649, NT2RP2000663, NT2RP2000694, NT2RP2000818, NT2RP2000903, NT2RP2001200, NT2RP2001276, NT2RP2001480, NT2RP2001495, NT2RP2001514, NT2RP2001755, NT2RP2001915, NT2RP2001956, NT2RP2002063, NT2RP2002188, NT2RP2002232, NT2RP2002527, NT2RP2002533, NT2RP2002721, NT2RP2002824, NT2RP2002942, NT2RP2002976, NT2RP2003042, NT2RP2003210, NT2RP2003383, NT2RP2003390, NT2RP2003469, NT2RP2003593, NT2RP2003655, NT2RP2003664, NT2RP2003950, NT2RP2004179, NT2RP2004205, NT2RP2004495, NT2RP2004524, NT2RP2004556, NT2RP2004606, NT2RP2004648, NT2RP2004794, NT2RP2005027, NT2RP2005163, NT2RP2005181, NT2RP2005378, NT2RP2005463, NT2RP2005541, NT2RP2005597, NT2RP2005666, NT2RP2005883, NT2RP2005994, NT2RP2006004,
NT2RP2006042, NT2RP2006269, NT2RP2006512, NT2RP2006580, NT2RP3000169, NT2RP3000171, NT2RP3000304, NT2RP3000436, NT2RP3000460, NT2RP3000616, NT2RP3000676, NT2RP3000721, NT2RP3000818, NT2RP3000907, NT2RP3000921, NT2RP3001012, NT2RP3001159, NT2RP3001195, NT2RP3001240, NT2RP3001271, NT2RP3001322, NT2RP3001388, NT2RP3001560, NT2RP3001592, NT2RP3001650, NT2RP3001738, NT2RP3001858, NT2RP3002015, NT2RP3002160, NT2RP3002311, NT2RP3002342, NT2RP3002411, NT2RP3002737, NT2RP3002790, NT2RP3002836, NT2RP3002900, NT2RP3002958, NT2RP3003000, NT2RP3003076, NT2RP3003354, NT2RP3003532, NT2RP3003535, NT2RP3003614, NT2RP3004025, NT2RP3004075, NT2RP3004083, NT2RP3004130, NT2RP3004133, NT2RP3004309, NT2RP3004345, NT2RP3004406, NT2RP3004481, NT2RP3004552, NT2RP3004625, NT2RP3004647, NT2RP4001001, NT2RP4001009, NT2RP4001467, NT2RP4001879, NT2RP4002187, NT2RP4002451, NT2RP4002750, OVARC1000003, OVARC1000105, OVARC1000298, OVARC1000307, OVARC1000313, OVARC1000410, OVARC1000439, OVARC1000553, OVARC1000811, OVARC1000873, OVARC1000956, OVARC1001030, OVARC1001163, OVARC1001336, OVARC1001570, OVARC1001607, OVARC1001725, OVARC1001991, PLACE1000033, PLACE1000231, PLACE1000560, PLACE1000740, PLACE1000912, PLACE1000914, PLACE1000927, PLACE1001016, PLACE1001123, PLACE1001183, PLACE1001231, PLACE1001340, PLACE1001401, PLACE1001407, PLACE1001464, PLACE1001516, PLACE1001536, PLACE1001564, PLACE1001655, PLACE1001795,
PLACE1001836, PLACE1001918, PLACE1001949, PLACE1002080, PLACE1002095, PLACE1002355, PLACE1002374, PLACE1002518, PLACE1002547, PLACE1002726, PLACE1002905, PLACE1002911, PLACE1002967, PLACE1003407, PLACE1003573, PLACE1003737, PLACE1003772, PLACE1003839, PLACE1003845, PLACE1003852, PLACE1004279, PLACE1004282, PLACE1004441, PLACE1004450, PLACE1004482, PLACE1004520, PLACE1004630, PLACE1004637, PLACE1004648, PLACE1004816, PLACE1005003, PLACE1005005, PLACE1005031, PLACE1005383, PLACE1005410, PLACE1005426, PLACE1005544, PLACE1005569, PLACE1005660, PLACE1005725, PLACE1005745, PLACE1005878, PLACE1005927, PLACE1006071, PLACE1006093, PLACE1006208, PLACE1006277, PLACE1006290, PLACE1006443, PLACE1006716, PLACE1006809, PLACE1006959. PLACE1007081, PLACE1007096, PLACE1007296, PLACE1007626, PLACE1007845, PLACE1007881, PLACE1008359, PLACE1008469, PLACE1008716, PLACE1008744, PLACE1008985, PLACE1009067, PLACE1009196, PLACE1009279, PLACE1009527, PLACE1009546, PLACE1009600, PLACE1009982, PLACE1010011, PLACE1010078, PLACE1010251, PLACE1010445, PLACE1010713, PLACE1010784, PLACE1010827, PLACE1010968, PLACE1011116, PLACE1011181, PLACE1011236, PLACE1011516, PLACE1011708, PLACE3000181, PLACE3000213, PLACE4000354, SKNMC1000004, SKNMC1000014, SKNMC1000082, THYRO1000036, THYRO1000099, THYRO1000196, THYRO1000400, THYRO1000584, THYRO1000678, THYRO1000776, THYRO1000795, THYRO1000956, THYRO1001102, THYRO1001113,
THYRO1001205, THYRO1001237, THYRO1001242, THYRO1001266, THYRO1001327, THYRO1001456, THYRO1001478, THYRO1001523, THYRO1001529, THYRO1001641, THYRO1001702, THYRO1001725, Y79AA1000207, Y79AA1000226, Y79AA1000270, Y79AA1000426, Y79AA1000521, Y79AA1000876, Y79AA1000888, Y79AA1000959, Y79AA1001013, Y79AA1001212, Y79AA1001264, Y79AA1001328, Y79AA1001426, Y79AA1001427, Y79AA1001430, Y79AA1001727, Y79AA1001787, Y79AA1001795, Y79AA1001799, Y79AA1001803, Y79AA1002022, Y79AA1002058, Y79AA1002129, Y79AA1002213, Y79AA1002373,

The following 146 clones were categorized into glycoprotein-associated proteins.
BNGH41000087, BNGH41000091, HEMBA1000349, HEMBA1000590, HEMBA1000745, HEMBA1000835, HEMBA1001221, HEMBA1001228, HEMBA1001621, HEMBA1002131, HEMBA1002178, HEMBA1002421, HEMBA1002767, HEMBA1003230, HEMBA1003392, HEMBA1004250, HEMBA1004391, HEMBA1004444, HEMBA1004505, HEMBA1005449, HEMBA1005522, HEMBA1005545, HEMBA1006707, HEMBA1006749, HEMBA1006902, HEMBB1000679, HEMBB1000881, HEMBB1001048, HEMBB1002120, HEMBB1002245, HEMBB1002427, MAMMA1000102, MAMMA1000591, MAMMA1000681, MAMMA1001043, MAMMA1001237, MAMMA1002070, MAMMA1002586, MAMMA1003126, NT2RM1000462, NT2RM1000580, NT2RM2001792, NT2RM2001818, NT2RM2001939, NT2RM2001941, NT2RM4000198, NT2RM4000284, NT2RM4000417, NT2RM4000648, NT2RM4000997, NT2RM4001325, NT2RM4002352, NT2RP1000613, NT2RP1000981, NT2RP1001004, NT2RP2000616, NT2RP2000694, NT2RP2000903, NT2RP2001480, NT2RP2001755, NT2RP2002533, NT2RP2003042, NT2RP2003210, NT2RP2004205, NT2RP2004606, NT2RP2005027, NT2RP2005181, NT2RP2005541, NT2RP2005597, NT2RP2005883, NT2RP2006004, NT2RP2006042, NT2RP2006269, NT2RP3000304, NT2RP3000616, NT2RP3000921, NT2RP3001650, NT2RP3002160, NT2RP3002737, NT2RP3002958, NT2RP3003000, NT2RP3003532, NT2RP3004130, NT2RP3004133, NT2RP3004481, NT2RP3004552, NT2RP3004640, NT2RP4000108, NT2RP4001467, NT2RP4002750, OVARC1000003, OVARC1000553, OVARC1000811, OVARC1000873, OVARC1001336, OVARC1001607, OVARC1001991, PLACE1000033, PLACE1000740, PLACE1001016,
PLACE1001123, PLACE1001231, PLACE1001464, PLACE1001655, PLACE1001836, PLACE1002355, PLACE1002374, PLACE1002905, PLACE1002911, PLACE1003573, PLACE1003737, PLACE1003772, PLACE1003839, PLACE1004282, PLACE1004441, PLACE1004450, PLACE1004520, PLACE1004648, PLACE1005003, PLACE1005426, PLACE1006071, PLACE1006073, PLACE1006290, PLACE1007081, PLACE1007845, PLACE1008716, PLACE1008744, PLACE1008985, PLACE1010251, PLACE1010784, PLACE1010968, PLACE1011116, PLACE3000181, PLACE3000213, PLACE4000354, THYRO1000036, THYRO1000196, THYRO1000584, THYRO1000956, THYRO1001266, Y79AA1000270, Y79AA1000426, Y79AA1001727, Y79AA1001795, Y79AA1002022, Y79AA1002213,

The following 55 clones were categorized into signal transduction-associated proteins.
HEMBA1000006, HEMBA1002195, HEMBA1002227, HEMBA1002551, HEMBA1005084, HEMBA1005929, HEMBA1006658, HEMBA1006916, MAMMA1000881, MAMMA1001150, MAMMA1001310, MAMMA1002142, NT2RM2001902, NT2RP1001020, NT2RP1001031, NT2RP2001469, NT2RP2001529, NT2RP2001769, NT2RP2003179, NT2RP2003545, NT2RP2004670, NT2RP3000011, NT2RP3000022, NT2RP3000172, NT2RP3000201, NT2RP3000820, NT2RP3003527, NT2RP3003849, NT2RP3003874, NT2RP3004067,
NT2RP4000634, NT2RP4000962, OVARC1000255, OVARC1000529, OVARC1000916, OVARC1001338, OVARC1001569, PLACE1002329, PLACE1003135, PLACE1003598, PLACE1005519, PLACE1006208, PLACE1008282, PLACE1008297, PLACE1010081, PLACE1011364, PLACE1011824, THYRO1001457, THYRO1001593, THYRO1001700, THYRO1001770, Y79AA1000777, Y79AA1000967, Y79AA1002376, Y79AA1002381,

The following 80 clones were categorized into transcription -associated proteins.
HEMBA1000462, HEMBA1000671, HEMBA1001297, HEMBA1001390, HEMBA1001886, HEMBA1002048, HEMBA1003120, HEMBA1003497, HEMBA1004785, HEMBA1005230, HEMBA1005246, HEMBA1006276, HEMBA1006572, HEMBA1007226, HEMBB1000106, HEMBB1000905, HEMBB1001959, HEMBB1002051, HEMBB1002661, MAMMA1001094, MAMMA1001532, MAMMA1001615, NT2RM1000789, NT2RM2000632, NT2RM2000773, NT2RM4000326, NT2RP1000271, NT2RP1000468, NT2RP2000092, NT2RP2000610, NT2RP2000712, NT2RP2000739, NT2RP2001538, NT2RP2001662, NT2RP2001817, NT2RP2001948, NT2RP2002564, NT2RP2002974, NT2RP2003138, NT2RP2003302, NT2RP2003940, NT2RP2004108, NT2RP2004847, NT2RP2005247, NT2RP2005391, NT2RP2005535, NT2RP2005774, NT2RP2005941, NT2RP2006092, NT2RP3000148, NT2RP3000232, NT2RP3000378, NT2RP3000652, NT2RP3001976, NT2RP3004090, NT2RP3004119, NT2RP3004294, OVARC1001049, OVARC1001086, OVARC1001132, OVARC1001807, PLACE1000258, PLACE1000442, PLACE1000907, PLACE1003529, PLACE1004166, PLACE1004168, PLACE1004887, PLACE1005250, PLACE1005682, PLACE1006079, PLACE1008549, PLACE1011407, PLACE1011978, THYRO1000580, Y79AA1000030, Y79AA1001090, Y79AA1001523, Y79AA1002334, Y79AA1002378,

The following 85 clones were categorized into disease-associated proteins.
BNGH41000020, HEMBA1000349, HEMBA1000590, HEMBA1000671, HEMBA1000835, HEMBA1001184, HEMBA1001228, HEMBA1001886, HEMBA1003120, HEMBA1004250, HEMBA1005246, HEMBA1005267, HEMBA1006707, HEMBA1006749, HEMBA1006902, HEMBA1006916, HEMBA1007013, HEMBB1002120, MAMMA1000204, MAMMA1002080, NT2RM2000632, NT2RM2001126, NT2RM2001558, NT2RP1000271, NT2RP1000465, NT2RP1000579, NT2RP2000447, NT2RP2000514, NT2RP2000739, NT2RP2001223, NT2RP2001529, NT2RP2001562, NT2RP2002674, NT2RP2003369, NT2RP2004108, NT2RP2004205, NT2RP2005535, NT2RP2005941, NT2RP2006004, NT2RP3000059, NT2RP3000125, NT2RP3000201, NT2RP3000232, NT2RP3000616, NT2RP3000677, NT2RP3000838, NT2RP3000921, NT2RP3001542, NT2RP3002286, NT2RP3002721, NT2RP3002737, NT2RP3002738, NT2RP3004481, OVARC1000208, OVARC1000275, OVARC1000331, OVARC1000410, OVARC1001086, OVARC1001132, OVARC1001607, OVARC1001725, OVARC1001952, PLACE1000258, PLACE1000442, PLACE1000907, PLACE1001100, PLACE1001500, PLACE1002905, PLACE1002967, PLACE1003407, PLACE1003428, PLACE1005005, PLACE1005239, PLACE1005815, PLACE1007028, PLACE1008716, PLACE1011407, PLACE1011978, PLACE2000118, THYRO1000580, THYRO1000866, THYRO1001071, THYRO1001478, Y79AA1001062, Y79AA1001530,

Out of them, the following 67 clones are those which matched the data of Swiss-Prot database and GenBank or UniGene database, genes or proteins which had been deposited in the database of Online Mendelian Inheritance in Man (OMIM) (http://www.ncbi.nlm.nih.gov/Omim/), which is a database of human genes and diseases. (The corresponding OMIM numbers are indicated after the clone names.)
HEMBA1000349(*600046), HEMBA1000590(*603897), HEMBA1000671(*602277), HEMBA1001886(*603899), HEMBA1003120(*602277), HEMBA1004250(*600976), HEMBA1005246(*602291), HEMBA1005267(*106410), HEMBA1006707(*603897), HEMBA1006749(*603897), HEMBA1006902(*603897), HEMBA1006916(*601524), HEMBA1007013(*603730), HEMBB1002120(*603367), MAMMA1002080(*602672), NT2RM2001126(*603785), NT2RM2001558(*604689), NT2RP1000271(*603899), NT2RP1000465(*602231), NT2RP2000447(*602580),
NT2RP2000514(*602431), NT2RP2000739(*194558), NT2RP2001223(*603558), NT2RP2001529(*603289), NT2RP2001562(*603371), NT2RP2002674(*132811), NT2RP2003369(*179555), NT2RP2004108(*601260), NT2RP2004205(*601610), NT2RP2005535(*603899), NT2RP2006004(*600245), NT2RP3000059(*106410), NT2RP3000125(*180202), NT2RP3000201(*604666), NT2RP3000232(*602277), NT2RP3000616(*600245), NT2RP3000677(*142765), NT2RP3000838(*190370), NT2RP3001542(*191161), NT2RP3002286(*604331),
NT2RP3002721(*118950), NT2RP3002738 (*602265), NT2RP3004481(*601610), OVARC1000208(*603603), OVARC1000275(*125647), OVARC1000331(*139265), OVARC1000410(*603874), OVARC1001086(*603862), OVARC1001725(*603046), OVARC1001952(*190370), PLACE1000258(*603971), PLACE1000442(*601260), PLACE1000907(*194558), PLACE1001500(*603781), PLACE1002905(*125950), PLACE1003428(*603570), PLACE1005005(*603124), PLACE1005239(*603365), PLACE1007028(*602131), PLACE1011407(*602277),
PLACE1011978(*603971), PLACE2000118(*301000), THYRO1000580(*602277), THYRO1000866(*604045), THYRO1001071(*603533), Y79AA1001062(*191161), Y79AA1001530(*602662),

Out of 215 clones excluding the above-mentioned clones, HEMBB1000668 and NT2RM4001377 presumably belong to a group of signal transduction-associated proteins, based on the results of domain search by Pfam.

HEMBB1002302 presumably belong to a group of transcription-associated proteins, based on the results of domain search by Pfam.

In the 437 clones categorized into secretory and/or transmembrane proteins on the basis of their full-length sequences, 410 clones were also predicted to encode proteins having functions of secretory and/or membrane proteins on the basis of their partial nucleotide sequences (5' sequences). In the 146 clones categorized into glycoprotein-associated proteins on the basis of their full-length sequences, 124 clones were also predicted to encode proteins having functions of glycoprotein-associated proteins on the basis of their partial nucleotide sequences. In the 57 clones categorized into signal transduction-associated proteins on the basis of their full-length sequences, 46 clones were also predicted to encode proteins having functions of signal transduction-associated proteins on the basis of their partial nucleotide sequences. In the 81 clones categorized into transcription-associated proteins on the basis of their full-length sequences, 57 clones were also predicted to encode proteins having functions of transcription-associated proteins on the basis of their partial nucleotide sequences. In the 85 clones categorized into disease-associated proteins on the basis of their full-length sequences, 6 clones were also predicted to encode proteins having functions of disease-associated proteins on the basis of their partial nucleotide sequences. The number of clones which were predicted to encode disease-associated proteins based on the full-length nucleotide sequences is much greater than that predicted based on the partial sequences. The reason is that the full-length sequences were categorized by using the data found in the OMIM database into the category of disease-associated proteins.

When the predicted functions based on the partial sequences were different from those based on the full-length sequences, several reasons were presumed; the ORF is too short in the partial sequence as compared with that of the full-length sequence; alternatively, P value for the partial sequence was greater than that for the full-length, that is, as compared with the probability of occurrence of the predicted function found in the full-length sequence, the probability was lower in the partial sequence. A protein does not always belong solely to a single category of the above-described functional categories, and therefore, additional functions can be found for the cDNA clones by further analyses.

It is unclear, by the analyses for the full-length sequences so far, whether or not the remaining 212 clones encode proteins belonging to any of the categories of secretory and/or membrane proteins, glycoprotein-associated proteins, signal transduction-associated proteins, transcription-associated proteins or disease-associated proteins. Nonetheless, the functions which were predicted based on the partial sequences can be verified by further analyses.

Among the 212 clones, there are 38 clones that presumably belong to the category of enzymes and/or metabolism-associated proteins, cell division- and/or cell proliferation-associated proteins, cytoskeleton-associated proteins, nuclear proteins, DNA- and/or RNA-binding proteins, ATP- and/or GTP-binding proteins, protein synthesis- and/or protein transport-associated proteins, or cellular defense-associated proteins. The clones containing results of homology search of Swiss-Prot database were categorized by considering the keywords and mentioned items in the matching data. The clones containing results of homology search of GenBank or UniGene database were categorized by considering the definitions and mentioned items in the matching data.

When the matching data contained keywords such as "metabolism", "oxidoreductase" and "E.C. No. (Enzyme commission number)", the clones were herein defined as clones presumably belonging to the category of enzymes and/or metabolism-associated proteins. When the matching data contained keywords such as "cell division", "cell cycle", "mitosis", "chromosomal protein", "cell growth" and "apoptosis", the clones were herein defined as clones presumably belonging to the category of cell division- or cell proliferation-associated proteins. When the matching data contained keywords such as "structural protein", "cytoskeleton", "actin-binding" and "microtubules", the clones were herein defined as clones presumably belonging to the category of cytoskeleton-associated proteins. When the matching data contained keywords such as "nuclear protein", the clones were herein defined as clones presumably belonging to the category of nuclear proteins. When the matching data contained keywords such as "DNA-binding" and "RNA-binding", the clones were herein defined as clones presumably belonging to the category of DNA- or RNA-binding proteins. When the matching data contained keywords such as "ATP-binding" and "GTP-binding", the clones were herein defined as clones presumably belonging to the category of ATP- and/or GTP-binding proteins. When the matching data contained keywords such as "translation regulation", "protein biosynthesis", "amino-acid biosynthesis", "ribosomal protein", "protein transport" and "signal recognition particle", the clones were herein defined as clones presumably belonging to the category of protein synthesis- and/or protein transport-associated proteins. When the matching data contained keywords such as "heat shock", "DNA repair" and "DNA damage", the clones were herein defined as clones presumably belonging to the category of cellular defense-associated proteins.

The following 10 clones presumably belong to enzymes and/or metabolism-associated proteins.
HEMBA1003315, HEMBB1002465, MAMMA1000614, NT2RP2000178, NT2RP2001388, NT2RP2001903, NT2RP2002304, NT2RP2005878, NT2RP3001685, PLACE1006219

The following 4 clones presumably belong to cell division-associated and/or cell proliferation-associated proteins.
MAMMA1000403, NT2RM2000497, NT2RP2000394, Y79AA1002121

The following 6 clones presumably belong to cytoskeleton-associated proteins.
MAMMA1001609, NT2RM2000589, NT2RP3000063, PLACE1004078, PLACE1004492, PLACE1008657

The following 7 clones presumably belong to nuclear proteins.
HEMBA1001878, HEMBA1002992, MAMMA1000614, NT2RM4000965, NT2RM2001738, NT2RP2001388, Y79AA1002121

The following 5 clones presumably belong to DNA- and/or RNA-binding proteins.
HEMBA1003072, HEMBA1006770, HEMBA1007332, NT2RM2000497, Y79AA1002121

The following 7 clones presumably belong to ATP- and/or GTP-binding proteins.
HEMBA1002316, MAMMA1001609, NT2RM2000306, NT2RM2000497, NT2RP2000178, NT2RP3003729, PLACE1004305

The following 7 clones presumably belong to protein synthesis- and/or protein transport-associated proteins.
NT2RM4000965, NT2RP2005069, NT2RP3000481, NT2RP3000789, NT2RP4001877, OVARC1001833, OVARC1002058,

The following clone presumably belongs to cellular defense-associated proteins.
PLACE1005539

Although it is unclear whether or not 26 out of 174 clones other than the above-mentioned clones belong to any of the above-described categories, these clones are predicted to have some functions, based on the homology search using their full-length sequences. The clone names and the gene definitions found in the result of homology search are shown below, separated by a double-slash, //
HEMBA1000634//Homo sapiens T-cell activation protein (PGR1) gene, complete cds.
HEMBA1002524//Human MHC Class I region proline rich protein mRNA, complete cds.
HEMBA1003399//MVP1 PROTEIN.
HEMBA1005489//Mus musculus semaphorin cytoplasmic domain-associated protein 3A (Semcap3) mRNA, complete cds.
HEMBB1000542//Mus musculus bromodomain-containing protein BP75 mRNA, complete cds.
MAMMA1000788//Bos taurus P14 (p14) mRNA, complete cds.
MAMMA1002128//ABC1 PROTEIN HOMOLOG PRECURSOR.
NT2RM2000514//Homo sapiens F-box protein Fbx21 (FBX21) mRNA, complete cds.
NT2RM2000622//Mus musculus F-box protein FBL10 mRNA, partial cds.
NT2RM4000100//Homo sapiens Leman coiled-coil protein (LCCP) mRNA, complete cds.
NT2RP2005425//Homo sapiens mRNA for AKAP450 protein.
NT2RP3001170//Mus musculus activity-dependent neuroprotective protein (Adnp) mRNA, complete cds.
NT2RP3002571//Bos taurus mRNA for lyncein.
NT2RP3004557//Human Ki nuclear autoantigen mRNA, complete cds.
OVARC1001596//Homo sapiens Arf-like 2 binding protein BART1 mRNA, complete cds.
PLACE1002153//Homo sapiens TACC2 protein (TACC2) mRNA, partial cds.
PLACE1003163//Homo sapiens DBI-related protein mRNA, complete cds.
PLACE1005736//Human mRNA for BAS-GRIP protein.
PLACE1007702//Mus musculus TRA1 mRNA, complete cds.
PLACE1011045//Homo sapiens E1-like protein mRNA, complete cds.
THYRO1000061//Mus musculus mRNA for UBE-1c1, UBE-1c2, UBE-1c3, complete cds.
THYRO1000964//Drosophila melanogaster Pelle associated protein Pellino (Pli) mRNA, complete cds.
Y79AA1000776//Mus musculus mRNA for GSG1, complete cds.
Y79AA1001056//Homo sapiens MAID protein mRNA, complete cds.
Y79AA1001272//Homo sapiens retinoic acid repressible protein (RARG-1) mRNA, complete cds.
Y79AA1001793//Mus musculus mRNA for GSG1, complete cds.

So far, useful information for presuming the functions are unavailable for the remaining 148 clones, of which names are listed below.
HEMBA1000275, HEMBA1000300, HEMBA1000443, HEMBA1000875, HEMBA1000907, HEMBA1001272, HEMBA1001296, HEMBA1001563, HEMBA1002164, HEMBA1002239, HEMBA1002985, HEMBA1003294, HEMBA1003487, HEMBA1004007, HEMBA1004067, HEMBA1004085, HEMBA1004952, HEMBA1004971, HEMBA1005145, HEMBA1005430, HEMBA1005913, HEMBA1006016, HEMBA1006517, HEMBA1006544, HEMBA1006912, HEMBA1007057, HEMBA1007063, HEMBA1007291, HEMBB1000276, HEMBB1000309, HEMBB1000642, HEMBB1001200, HEMBB1001547, HEMBB1002039, HEMBB1002228, HEMBB1002663, MAMMA1000046, MAMMA1000449, MAMMA1000528, MAMMA1000652, MAMMA1000706, MAMMA1000814, MAMMA1001066, MAMMA1001284, MAMMA1001623, MAMMA1001634, MAMMA1001901, MAMMA1002087, MAMMA1002205, MAMMA1002224, NT2RM2000582, NT2RM2001643, NT2RM4000115, NT2RM4000295, NT2RM4001321, NT2RP1000002, NT2RP1000239, NT2RP1000679, NT2RP1000740, NT2RP1000903, NT2RP2000240, NT2RP2001878, NT2RP2001921, NT2RP2002015, NT2RP2002409, NT2RP2002510, NT2RP2003599, NT2RP2003931, NT2RP2004069, NT2RP2004141, NT2RP2004447, NT2RP2004837, NT2RP2005514, NT2RP2005632, NT2RP2005887, NT2RP2006099, NT2RP2006134, NT2RP3000427, NT2RP3000444, NT2RP3000645, NT2RP3000871, NT2RP3001044, NT2RP3001061, NT2RP3001754, NT2RP3002281, NT2RP3002324, NT2RP3002353, NT2RP3002409, NT2RP3002448, NT2RP3002664, NT2RP3002887, NT2RP3002983, NT2RP3003448, NT2RP3003469, NT2RP3003473, NT2RP3003559, NT2RP3003963, NT2RP3004000, NT2RP3004202, NT2RP3004321,
NT2RP3004355, NT2RP3004374, NT2RP4002715, OVARC1000090, OVARC1000137, OVARC1000467, OVARC1000775, OVARC1000853, OVARC1000995, OVARC1001222, OVARC1001260, OVARC1001727, OVARC1002178, PLACE1000986, PLACE1001114, PLACE1001229, PLACE1001788, PLACE1003438, PLACE1003460, PLACE1003644, PLACE1004028, PLACE1004199, PLACE1004519, PLACE1005601, PLACE1005669, PLACE1005768, PLACE1006515, PLACE1006786, PLACE1007040, PLACE1007077, PLACE1007591, PLACE1007971, PLACE1008984, PLACE1009735, PLACE2000219, PLACE4000455, THYRO1000846, THYRO1000999, THYRO1001063, THYRO1001128, THYRO1001471, THYRO1001495, THYRO1001608, THYRO1001803, Y79AA1000127, Y79AA1000750, Y79AA1001592, Y79AA1001863,

### EXAMPLE 18

### Expression frequency analysis using PCR

Many genes acting at the downstream of TNF-α and IL-1 β among inflammation-associated cytokines have been previously identified. The respective stimulations are transduced through independent pathways of signaling cascade. There exists another signaling cascade for both stimulations, wherein NF- κ B is a common transducing molecule shared by the two stimulations (J. Leukoc. Biol., 1994, 56(5): 542-547). It has also been revealed that many inflammation-associated genes, including IL-2, IL-6 and G-CSF, are varied in their expression levels in response to the signal through the common pathway (Trends Genet. 1999, 15(6): 229-235). A survey was performed by using ATAC-PCR method (adaptor-competitive PCR method: Nucleic Acids Res. 1997, Nov 15; 25(22): 4694-6) for genes of which expression levels were varied depending on stimulation of inflammatory cytokines, TNF-α and IL-1β. It is possible that genes of which expression is varied in response to this stimulation also participate in inflammation.

Jurkat cells (Dainippon Pharmaceutical Co., Ltd.: catalog No. 06-152) were cultured in a PRMI1640 medium (Nikken Biological and Medical Institute: catalog No. 14-501F) containing 10% fetal calf serum until the cell count reached 10⁷ cells. The cells were transferred into a fresh medium containing 10 ng/ml TNF-α (recombinant Tumor Necrosis Factor; Wako pure chemical Industries Inc.: catalog No. 201-13461) or IL-1β (recombinant Interleukin-1β; PeprotechEC: catalog No. 200-01B) and, further, cultured at 37°C under an atmosphere of 5% CO₂. The cells cultured in the presence of TNF-α were harvested 1, 3 and 7 hours after addition of TNF-α. The cells cultured in the presence of IL-1β were harvested 1 and 7 hours after addition of IL-1β. Total RNA was extracted from each of the cells by AGPC method (Acid-Guanidinium-Phenol-Chloroform method: Ana Biochem. 1987, Apr; 162(1):156-9). Total RNA was also extracted form the cells in the absence of any stimulation of TNF-α and IL-1β.

ATAC-PCR analysis is performed basically according to the same procedure as described in "DNA Microarray and Advanced PCR Methods" (Cell Engineering, p. 104-112, (additional volume, Genome Science Series 1), Muramatsu & Naba (eds.), Shujunnsya). Adaptor ligation reaction was performed for an internal standard sample (which was used for preparing a calibration curve for the assessment of the test samples) and test samples in the following independent two reaction systems. Combinations of each type of the 6 adaptors (AD-1, AD-2, AD-3, AD-4, AD-5, and AD-6: see the sequences shown below) with each sample are as follows:

### Reaction system A

AD1: internal standard sample (× 10 concentration)
AD2: sample before stimulation
AD3: internal standard sample (×3 concentration)
AD4: sample with IL-1 stimulation for 1 hour
AD5: sample with IL-1 stimulation for 7 hours
AD6: internal standard sample (×1 concentration)
   Reaction system B
AD1: internal standard sample (×1 concentration)
AD2: sample with TNF stimulation for 1 hour
AD3: sample with TNF stimulation for 3 hours
AD4: internal standard sample (×3 concentration)
AD5: sample with TNF stimulation for 7 hours
AD6: internal standard sample (×10 concentration)

### Adaptor sequence

AD1;
SEQ ID NO:4180//5'-GTACATATTGTCGTTAGAACGCG-3'
SEQ ID NO:4181//3'-CATGTATAACAGCAATCTTGCGCCTAG-5'

AD2;
SEQ ID NO:4182//5'-GTACATATTGTCGTTAGAACGCGACT-3'
SEQ ID NO:4183//3'-CATGTATAACAGCAATCTTGCGCTGACTAG-5'

AD3;
SEQ ID NO:4184//5'-GTACATATTGTCGTTAGAACGCGCATACT-3'
SEQ ID NO:4185//3'-CATGTATAACAGCAATCTTGCGCGTATCACTAG-5'

AD4;
SEQ ID NO:4186//5'-GTACATATTGTCGTTAGAACGCGATCCATACT-3'
SEQ ID NO:4187//3'-CATGTATAACAGCAATCTTGCGCTAGGTATGACTAG-5'

AD5;
SEQ ID NO:4188//5'-GTACATATTGTCGTTAGAACGCGTCAATCCATACT-3'
SEQ ID NO:4189//3'-CATGTATAACAGCAATCTTGCGCAGTTAGGTATGACTAG-5'

AD6;
SEQ ID NO:4190//5'-GTACATATTGTCGTTAGAACGCGTACTCAATCCATACT-3'
SEQ ID NO:4191//3'-CATGTATAACAGCAATCTTGCGCATGAGTTAGGTATGACTAG-5'

In this assay, the internal standard samples used were total RNA from cultured cells or human tissues from which the cDNA libraries originated. The cultured cells and the total RNAs from tissues are indicated below. Culture of the cells was performed according to the method as described in the supplier's instruction manual. RNA preparation was carried out by standard methods.
Human teratocarcinoma cell NT-2 (Stratagene, catalog No. 204101)
Human neuroblastoma cell SK-N-MC (Dainippon Pharmaceutical Co., Ltd., catalog No. 04-010)
Human neuroblastoma cell Y79 (Dainippon Pharmaceutical Co., Ltd., catalog No. 04-018)
Human placenta tissues total RNA (BioChin, catalog No. 064008)
Human breast tissue total RNA (Clontech, catalog No. 64037-1)

PCR primers used for amplification of specific genes, and names of the corresponding cDNA clones are shown below. The assay was not carried out for clones of which corresponding internal standard sample could not be prepared for the assay. The gene-specific primers were designed so that the PCR products derived from the cDNAs with adaptor were 70-200 bp in size. Sequence of the adaptor-specific primer (labeled with fluorescent dye (FAM)) used for the competitive PCR was GTACATATTGTCGTTAGAACGC (22 nucleotides, SEQ ID NO: 4192). PCR was performed basically at 94°C for 5 minutes; and at 94°C for 30 seconds, at 50°C for 60 seconds, and at 72°C for 60 seconds for 30 cycles. The annealing temperature was, however, changed in some PCR experiments.

Nucleotide sequence of clone-specific primer (all the primers consist of 20 nucleotides) used in this experiment

Clone names, primer sequences, and SEQ ID NOs were shown in this order, separated with a double-slash mark, //

MAMMA1000046//GTTACATCCAAGCATACAGG//SEQ ID NO:4193

MAMMA1000102//ACGGGGTCTCATTCTGACAC//SEQ ID NO:4194

MAMMA1000141//CAAGGTAACACGAGTCTATC//SEQ ID NO:4195

MAMMA1000226//ACTGAGGGGCAAAGGAGAGA//SEQ ID NO:4196

MAMMA1000403//ATTTTCCTGGAGAGCCGACT//SEQ ID NO:4197

MAMMA1000473//TGGAAAGTGTACCGGAATTG//SEQ ID NO:4198

MAMMA1000496//TCAATCTGGCGCTCTGTCAC//SEQ ID NO:4199

MAMMA1000614//AGTTCTTTACATGCTGAGGT//SEQ ID NO:4200

MAMMA1000652//TGGTGGAAGACTGGGTTTGC//SEQ ID NO:4201

MAMMA1000706//ATGGTCTTGTGGTGCCAGGT//SEQ ID NO:4202

MAMMA1000788//TGTCCAAAAGCCACACAGAG//SEQ ID NO:4203

MAMMA1000810//ATACTTCCCGCACCCCAAAA//SEQ ID NO:4204

MAMMA1000814//CAGGGTTTCTGCATGTTGGC//SEQ ID NO:4205

MAMMA1000881//ATGGAGTTTCACTCTTGTTG//SEQ ID NO:4206

MAMMA1000986//TGCTGCTTCTTTACATGGGA//SEQ ID NO:4207

MAMMA1000994//CAGGATAGAGGTTGCAGGCT//SEQ ID NO:4208

MAMMA1001066//GATGGGGTCTCACTCTGTCA//SEQ ID NO:4209

MAMMA1001094//ACGTCCAGAAACTACAGGGT//SEQ ID NO:4210

MAMMA1001141//ACTGTACTTAGGATGCTTCA//SEQ ID NO:4211

MAMMA1001237//GGGCAACCCTATGTAGATGA//SEQ ID NO:4212

MAMMA1001284//GTCTGCTCTGTTACATAGGG//SEQ ID NO:4213

MAMMA1001310//ACGCCTGTAATCCCAACCCA//SEQ ID NO:4214

MAMMA1001344//GCCAGTTTGTTCTAGGATGC//SEQ ID NO:4215

MAMMA1001532//ACATCTATAAGGCTGTTTGC//SEQ ID NO:4216

MAMMA1001609//GGGTCTCACTCTGTTACCCA//SEQ ID NO:4217

MAMMA1001615//CAAGGGACACTGAGAACTGG//SEQ ID NO:4218

MAMMA1001623//GGATTGATGCCCGATACTTA//SEQ ID NO:4219

MAMMA1001901//GATAGGGTCTCATTCTGTTA//SEQ ID NO:4220

MAMMA1001957//TAGTAGAGACGGGGTTTCAC//SEQ ID NO:4221

MAMMA1001978//CTCCCTCAGACGCTTTATTG//SEQ ID NO:4222

MAMMA1002070//GAAGAGAAACTGGGGCATCC//SEQ ID NO:4223

MAMMA1002080//ACTCTCCCTCACTACCACTG//SEQ ID NO:4224

MAMMA1002087//AGCTCGTCTCATGGGCAACT//SEQ ID NO:4225

MAMMA1002091//CTGACGTAGGTGAGGTCCAT//SEQ ID NO:4226

MAMMA1002095//CATGTTTATGGTCAGGTAGT//SEQ ID NO:4227

MAMMA1002128//TGGAGCAGATGAGGAAGGAG//SEQ ID NO:4228

MAMMA1002142//TCTCTTAAACATGCAACAGG//SEQ ID NO:4229

MAMMA1002165//CTCAAACACCCAGGCTCAAG//SEQ ID NO:4230

MAMMA1002234//TTTGTCTTCCTCTAAACCAG//SEQ ID NO:4231

MAMMA1002586//CTCCACCGAAAAGACCCATT//SEQ ID NO:4232

MAMMA1002633//CACAGCAGCATCTCCAAGCA//SEQ ID NO:4233

MAMMA1003126//ACAGTATCAGAGGAGCAGGA//SEQ ID NO:4234

NT2RM1000462//AGAGCCGAGGACATTTGAGG//SEQ ID NO:4235

NT2RM1000542//CAAGGGCACGTTTCAGCACT//SEQ ID NO:4236

NT2RM1000789//TTCGCTTGCTCTTCTCTGGA//SEQ ID NO:4237

NT2RM1000855//AGCAAGGTCTCCAGAGTGTG//SEQ ID NO:4238

NT2RM1000858//GAACAGGAAAATGCTCTCAG//SEQ ID NO:4239

NT2RM2000241//CTGCTTGCCTGCCTTGTAGT//SEQ ID NO:4240

NT2RM2000306//TAGTCCCTTTCCTGATGGTC//SEQ ID NO:4241

NT2RM2000497//TTAGTAGAGACGGTGTTTCA//SEQ ID NO:4242

NT2RM2000514//TGTTCTCTTTCTTTGCACTG//SEQ ID NO:4243

NT2RM2000582//GGGAATAACATCTACAACCT//SEQ ID NO:4244

NT2RM2000588//CCCCAGAAACAGAGAAGGCT//SEQ ID NO:4245

NT2RM2000589//TAAGGCATGTGTCCTCTAAG//SEQ ID NO:4246

NT2RM2000622//ATCTCGGCTATGAACTGCTC//SEQ ID NO:4247

NT2RM2000773//TTCTGCCCCTCCTCAAACCT//SEQ ID NO:4248

NT2RM2001126//GCAACAGCTTCTTCATGGGT//SEQ ID NO:4249

NT2RM2001558//TCGCCCACACTGCATCCTTT//SEQ ID NO:4250

NT2RM2001626//TCGGGTGGCAGTGTGTTGAA//SEQ ID NO:4251

NT2RM2001643//GTAGTTCTCTCTGAAGAATC//SEQ ID NO:4252

NT2RM2001738//CCCAGCACTTTTATTTGTAG//SEQ ID NO:4253

NT2RM2001792//AGTGTAGTTTGGAGATGAGA//SEQ ID NO:4254

NT2RM2001902//TCCCCATCCAGCCACAGAAA//SEQ ID NO:4255

NT2RM2002109//ATTGCCTATAGAAAGTCAGC//SEQ ID NO:4256

NT2RM4000100//GCATTTATAGGGCTCAAGAT//SEQ ID NO:4257

NT2RM4000115//TAGTTTCGACTTCTGGTTCA//SEQ ID NO:4258

NT2RM4000284//ACATCCTTCAAATCAGCATC//SEQ ID NO:4259

NT2RM4000295//GGTCTCACGCTGCTCACAAA//SEQ ID NO:4260

NT2RM4000417//TGCTGTGTAGTATTCCTTAG//SEQ ID NO:4261

NT2RM4000761//CTAATACAATGCCAGTCAGG//SEQ ID NO:4262

NT2RM4001377//CTAGCTTCCTCTCCCAACTG//SEQ ID NO:4263

NT2RM4001735//TCATCACGACTGCTGTAGAG//SEQ ID NO:4264

NT2RM4001768//TTAGTAGAGGTGGGGTTTCA//SEQ ID NO:4265

NT2RM4001843//CAAGGGCACGTTTCAGCACT//SEQ ID NO:4266

NT2RP1000239//TTAGCAGTGTATCCTTTCCG//SEQ ID NO:4267

NT2RP1000465//TTGCCCAGGCTAGTCTCGAA//SEQ ID NO:4268

NT2RP1000468//TTGCCCAGGCTAGTCTCGAA//SEQ ID NO:4269

NT2RP1000679//GGCCTCAGTTCCTTGCATTT//SEQ ID NO:4270

NT2RP1000740//CGCTATATTTCATGGGCTTT//SEQ ID NO:4271

NT2RP1001031//CATGTTACCTATACACACCA//SEQ ID NO:4272

NT2RP2000092//CACACACACATGAACTCATT//SEQ ID NO:4273

NT2RP2000178//ATCTTTGTGACAGCTCCAG//SEQ ID NO:4274

NT2RP2000240//CCATTCCACTCATACCAAGA//SEQ ID NO:4275

NT2RP2000447//CTCTTGGCATGATAGGTCTT//SEQ ID NO:4276

NT2RP2000610//GACATGAGACAAAATTAGCC//SEQ ID NO:4277

NT2RP2000616//AAAATAACTGCCTGGGAGGT//SEQ ID NO:4278

NT2RP2000712//GAAGGTAGAGCTTGAGAGAG//SEQ ID NO:4279

NT2RP2000739//AGCAACAGGGAATGGAGGTG//SEQ ID NO:4280

NT2RP2000818//GGGAAGAAATGAGACAAAGA//SEQ ID NO:4281

NT2RP2001200//ACCACATAGCTGCAGGAAAG//SEQ ID NO:4282

NT2RP2001276//TTGCCGTTGGTGCTGGTAGT//SEQ ID NO:4283

NT2RP2001388//TGGCACAATCTCCGCTCACT//SEQ ID NO:4284

NT2RP2001469//TAATGGGTGGTGGGAGCTGA//SEQ ID NO:4285

NT2RP2001538//GAAAAGGGTAGGCAGCAAGG//SEQ ID NO:4286

NT2RP2001562//CACCCTGCCACAAGACATTT//SEQ ID NO:4287

NT2RP2001662//AAGAGGCTGGGCAAATGGCA//SEQ ID NO:4288

NT2RP2001755//CAAGCAAATAATCCAGCCAT//SEQ ID NO:4289

NT2RP2001817//CTAAAGCAACAGAGGAATAC//SEQ ID NO:4290

NT2RP2001921//TGTGGGGTTGGTCTTTGGAA//SEQ ID NO:4291

NT2RP2001948//GCATTGAGGACTTTTCCAGA//SEQ ID NO:4292

NT2RP2002015//GTAGTTCTCTCTGAAGAATC//SEQ ID NO:4293

NT2RP2003138//ATGGAAGAGGCTGAGGCAAA//SEQ ID NO:4294

NT2RP2003194//CACCTCTCATGTTTCTGCAC//SEQ ID NO:4295

NT2RP2003302//AGAGGACAGTTGGAAGATTT//SEQ ID NO:4296

NT2RP2003390//CTGTGGGTGATTTTCTGGCA//SEQ ID NO:4297

NT2RP2003593//CTGACCCCTAAGTAAATGAA//SEQ ID NO:4298

NT2RP2003664//GGGTGTGATGTTTACTTCTC//SEQ ID NO:4299

NT2RP2003950//CTGAAGGAGGTACTGACACT//SEQ ID NO:4300

NT2RP2004069//CATCACCAAGGTATTAAGCC//SEQ ID NO:4301

NT2RP2004108//GATAGGTTACTGGTGAATTG//SEQ ID NO:4302

NT2RP2005069//GAAAATGAACCTGTAGCCTT//SEQ ID NO:4303

NT2RP2005378//ATTCTGGCTCCCTCTTCCTC//SEQ ID NO:4304

NT2RP2005391//CAAGGTGGATTTTACATGGG//SEQ ID NO:4305

NT2RP2005535//GAATTATCGCCATGTCTCTT//SEQ ID NO:4306

NT2RP2005597//GACCAAGACCCTTCAACCTC//SEQ ID NO:4307

NT2RP2006092//TCCTACGTTGTGGTGCAGTG//SEQ ID NO:4308

NT2RP2006134//TGACTGCCAATTAGAAACCT//SEQ ID NO:4309

NT2RP2006208//AAAATTGGCTTCTGCCTAGT//SEQ ID NO:4310

NT2RP2006476//GTTCACCTTCACATCCAAAA//SEQ ID NO:4311

NT2RP3000011//TGGCACAATCTCAGCTCACT//SEQ ID NO:4312

NT2RP3000031//TTCTGGGCTGGAGTAGTGGT//SEQ ID NO:4313

NT2RP3000063//AGATAGTCACATAGACAGAG//SEQ ID NO:4314

NT2RP3000125//ACACATCCAACCCTTCACTT//SEQ ID NO:4315

NT2RP3000148//AACAAGTCCAGCCCCAGAAG//SEQ ID NO:4316

NT2RP3000169//CCAAGCACGCCATATGAAGC//SEQ ID NO:4317

NT2RP3000171//CAGAATTTTGCCACGAGGAT//SEQ ID NO:4318

NT2RP3000172//GGCACAGACACCATCCTTGA//SEQ ID NO:4319

NT2RP3000201//AATGGGGTTTTGCCAGGTTG//SEQ ID NO:4320

NT2RP3000232//ACCTTCATACAACTTTTCCC//SEQ ID NO:4321

NT2RP3000304//TGGTCTGCCTCATCCTTCTC//SEQ ID NO:4322

NT2RP3000378//GAAAAGGGTAGGCAGCAAGG//SEQ ID NO:4323

NT2RP3000427//ATGTAAAGTGTGGAGTACCC//SEQ ID NO:4324

NT2RP3000444//TTCTTCTCAGTCACCTCCAC//SEQ ID NO:4325

NT2RP3000616//GTGATAGTAACACAATCCTG//SEQ ID NO:4326

NT2RP3000645//TGGCCAGTGTATGAGAGGTC//SEQ ID NO:4327

NT2RP3000676//CAAGACAACAAAACAGAAGG//SEQ ID NO:4328

NT2RP3000677//TCCATTTTAGCTGTAGACAC//SEQ ID NO:4329

NT2RP3000721//GAGACTGTGATGCCTTGGTG//SEQ ID NO:4330

NT2RP3000789//AACTGATGGCTTCTGTCTCC//SEQ ID NO:4331

NT2RP3000818//GGGAGAAGCCTAGAAAAGAA//SEQ ID NO:4332

NT2RP3000820//TCACATTCAAGCTCTACGTC//SEQ ID NO:4333

NT2RP3000838//CTAGCTTCCTCTCCCAACTG//SEQ ID NO:4334

NT2RP3000871//GATTGTGCTTGGAGCTTGGC//SEQ ID NO:4335

NT2RP3000907//GGGAGATGAAGAGGAAGCAG//SEQ ID NO:4336

NT2RP3000921//ACATGGGTAGCACTCCTTTT//SEQ ID NO:4337

NT2RP3001012//ACCCATTTCTACCTCTCTTA//SEQ ID NO:4338

NT2RP3001044//AGCAAGGCAATGAGGATACT//SEQ ID NO:4339

NT2RP3001159//AAATGTAGGGGTGGGGAAGT//SEQ ID NO:4340

NT2RP3001240//AGCAAGGTCTCCAGAGTGTG//SEQ ID NO:4341

NT2RP3001271//GTGCTTCTACCATTGAGGAT//SEQ ID NO:4342

NT2RP3001542//TGGTCCTCACTGCCTTCAAA//SEQ ID NO:4343

NT2RP3001560//CGCCTCCACAAACAAACCCT//SEQ ID NO:4344

NT2RP3001592//TAAAAGGGAGGATGTTCAGT//SEQ ID NO:4345

NT2RP3001650//GCATCTTCTGGTAGTTGTCC//SEQ ID NO:4346

NT2RP3001685//GATTCTCCTTGCCTCAGCCT//SEQ ID NO:4347

NT2RP3001754//AGTTAGTGGTGCCTGCTTCC//SEQ ID NO:4348

NT2RP3001976//CTCACTGGCATTTAGCTGGT//SEQ ID NO:4349

NT2RP3002015//CAACAACCTTCCTCCTACCC//SEQ ID NO:4350

NT2RP3002281//TAAACAGTCAACCAATGCTC//SEQ ID NO:4351

NT2RP3002286//AAGCAAGAGATTTGGAGGAA//SEQ ID NO:4352

NT2RP3002353//GGCTGGAACTCAATTTCTGC//SEQ ID NO:4353

NT2RP3002409//GTACCCCTAGTGAAGACCTG//SEQ ID NO:4354

NT2RP3002411//TCAATAGCTACCTGTAGAGT//SEQ ID NO:4355

NT2RP3002448//TCTTCCTACGACATAACCAT//SEQ ID NO:4356

NT2RP3002571//GCCCAAAATCCAACAGTAAA//SEQ ID NO:4357

NT2RP3002721//AGTGGCTTGTATTCTGTGGA//SEQ ID NO:4358

NT2RP3002737//GTGAGGTACTTGCATATCCG//SEQ ID NO:4359

NT2RP3002738//TCCCCGATGAACACCAGCTT//SEQ ID NO:4360

NT2RP3002790//CTGGAGCCTGATTATGAGAA//SEQ ID NO:4361

NT2RP3002836//CTTTAGCAACATAACCTCCA//SEQ ID NO:4362

NT2RP3002900//TTTCTTCCTCCCTAACACAT//SEQ ID NO:4363

NT2RP3002958//GCCTTCTTCCAGCTCTACAT//SEQ ID NO:4364

NT2RP3002983//AATTTCCTTGCTAGAAGGCT//SEQ ID NO:4365

NT2RP3003354//GGCTGACACCTATTATCCCA//SEQ ID NO:4366

NT2RP3003448//TGTAGTCCCAGCTATTCAGG//SEQ ID NO:4367

NT2RP3003473//AATTAATCTCTGGGTAGCAC//SEQ ID NO:4368

NT2RP3003527//TGTCAATGGCCAGGTCGTAG//SEQ ID NO:4369

NT2RP3003532//TAAACTGACTTCCTCTTGGG//SEQ ID NO:4370

NT2RP3003535//CTAATGCCAGTGTTTCAAGA//SEQ ID NO:4371

NT2RP3003559//AATTCTTGTCCTGCTTTTGC//SEQ ID NO:4372

NT2RP3003614//GGGTCTTTGGATTGAGTGTC//SEQ ID NO:4373

NT2RP3003963//CACGATTATCTCTGCCAAAA//SEQ ID NO:4374

NT2RP3004000//TGGCCAGTGTATGAGAGGTC//SEQ ID NO:4375

NT2RP3004025//CACATTCTGGTGGAAAAGCA//SEQ ID NO:4376

NT2RP3004067//TGATGACACGCAGCACTTCC//SEQ ID NO:4377

NT2RP3004075//TTTCAAGTCAACACCTGCAC//SEQ ID NO:4378

NT2RP3004090//TACACTACAGATGGGCAAAA//SEQ ID NO:4379

NT2RP3004119//TCTAAGGCTGGGTACAGTGG//SEQ ID NO:4380

NT2RP3004130//ACATTTCTCCTCCATACGCA//SEQ ID NO:4381

NT2RP3004133//TAACCGCACTATGAGGAAAG//SEQ ID NO:4382

NT2RP3004202//CAGTGGCCTGGTAATAGAGT//SEQ ID NO:4383

NT2RP3004294//ATATTCCACTCCCCATTCGG//SEQ ID NO:4384

NT2RP3004321//GACCAACATGCTAGAAGTGC//SEQ ID NO:4385

NT2RP3004345//AACTCTCATTCCATAAGGTG//SEQ ID NO:4386

NT2RP3004406//CACCTAAAAGACTAATCCCT//SEQ ID NO:4387

NT2RP3004552//GAATCCAAAAGCCGGTAGGG//SEQ ID NO:4388

NT2RP3004557//GAAAGAGGTCAAAGTACCTG//SEQ ID NO:4389

NT2RP3004625//ATACAGGCAGCAGGAATCAC//SEQ ID NO:4390

NT2RP3004647//CAAGACAACAAAACAGAAGG//SEQ ID NO:4391

NT2RP4000634//ATGAAGAGTTACCTATGTGG//SEQ ID NO:4392

NT2RP4000962//AACCCTGGGCTTGGAATTCA//SEQ ID NO:4393

NT2RP4001001//TTTCACAATGCTACAGAGGT//SEQ ID NO:4394

NT2RP4001009//AATGTCAGCGGAGCAAAAGA//SEQ ID NO:4395

NT2RP4001467//GTTGAGAATGCTGGACTTGA//SEQ ID NO:4396

NT2RP4001877//AATCATATAGTCCCAGGTTG//SEQ ID NO:4397

NT2RP4001879//GTGTGAGAGTAGTTGGGGAA//SEQ ID NO:4398

NT2RP4002187//TCAATAGCTACCTGTAGAGT//SEQ ID NO:4399

NT2RP4002451//CATAAACAGTGACAGCAGAA//SEQ ID NO:4400

NT2RP4002715//AGCAAGGCAATGAGGATACT//SEQ ID NO:4401

NT2RP4002750//CAGCATTTAGGTGTGACGAT//SEQ ID NO:4402

PLACE1000040//ACATTGCTTGAGTCTTGCCA//SEQ ID NO:4403

PLACE1000986//CAAGGAGGTAATAGGGAGAT//SEQ ID NO:4404

PLACE1002080//CTCACTCTGTCATCGAGGCT//SEQ ID NO:4405

PLACE1002547//GTACCCCTAGTGAAGACCTG//SEQ ID NO:4406

PLACE1002911//CAAAGGCAAGTGTTCAGGCT//SEQ ID NO:4407

PLACE1003407//GACTACAAAACCCTGGAAAG//SEQ ID NO:4408

PLACE1003573//GGTGGCATGTAAATAAGACT//SEQ ID NO:4409

PLACE1004078//CTCAGCCTTCCAAGTAGCAG//SEQ ID NO:4410

PLACE1004199//GGAATCTGGGAGTCAAAATC//SEQ ID NO:4411

PLACE1004305//GCACCACTTTCGTCTTGAGC//SEQ ID NO:4412

PLACE1004450//GGCACTTAGCTTCTTGTTTT//SEQ ID NO:4413

PLACE1004492//TGGGCATCAATAAACACCTC//SEQ ID NO:4414

PLACE1004630//TGCTTTTGTATGGCCTGGGA//SEQ ID NO:4415

PLACE1004816//ATGTGGACAGGCAAGCAGAG//SEQ ID NO:4416

PLACE1005031//GGTGTTCCCCTTCTTGTTTG//SEQ ID NO:4417

PLACE1005539//AGAGGTAGAATGAGGGGAAA//SEQ ID NO:4418

PLACE1005569//CTGAACCCTGGCCTGTGAAA//SEQ ID NO:4419

PLACE1005601//AGATGGGGACTATGAAGAGG//SEQ ID NO:4420

PLACE1005736//CCTGTAATCCCAGCACTTTG//SEQ ID NO:4421

PLACE1005815//AGAGACAGAGTTTTGCTCTT//SEQ ID NO:4422

PLACE1005927//GGTGTAGCCCATTGTAGAGG//SEQ ID NO:4423

PLACE1006071//TTTGCTCTTTAACTCTGCCT//SEQ ID NO:4424

PLACE1006073//TAAAGGGCAGGGTGGAGGAA//SEQ ID NO:4425

PLACE1006079//TGATGTCCACTGTCTTATTG//SEQ ID NO:4426

PLACE1006786//CAAGGAGGTAATAGGGAGAT//SEQ ID NO:4427

PLACE1007077//ATGTGCTGGTAAGTGAAATC//SEQ ID NO:4428

PLACE1007081//GCCGGCCAAGTTTACACGAA//SEQ ID NO:4429

PLACE1007845//TAACCGCACTATGAGGAAAG//SEQ ID NO:4430

PLACE1007971//GACAATTCAACTGGAAGACC//SEQ ID NO:4431

PLACE1008282//CGTGATGACTGCCAACTCCA//SEQ ID NO:4432

PLACE1008359//AACAGGGTCCCATTTATTTG//SEQ ID NO:4433

PLACE1008469//TTGTCATCCTCCTGCCTCTG//SEQ ID NO:4434

PLACE1008657//CTCAGCCTTCCAAGTAGCAG//SEQ ID NO:4435

PLACE1008744//TAAACAAGACCCAGCACCAT//SEQ ID NO:4436

PLACE1008984//TCGAGACCGCTTCCCATAGA//SEQ ID NO:4437

PLACE1009546//AGGTAGCCGATGACAAAGCC//SEQ ID NO:4438

PLACE1010011//GGATATAAAGACAAGGATGC//SEQ ID NO:4439

PLACE1010713//TCAATAGCTACCTGTAGAGT//SEQ ID NO:4440

PLACE1011019//AAGGAGTGAAGCTCCATGCC//SEQ ID NO:4441

PLACE1011116//CCATTACAAACCCTTTAACC//SEQ ID NO:4442

PLACE1011181//TGTTATCCATTGTTCACCTG//SEQ ID NO:4443

PLACE1011364//TGTTGACGCTGATTTAGGCA//SEQ ID NO:4444

PLACE3000213//TAAACAAGACCCAGCACCAT//SEQ ID NO:4445

PLACE4000354//TAAACAAGACCCAGCACCAT//SEQ ID NO:4446

PLACE4000455//CAAGGAGGTAATAGGGAGAT//SEQ ID NO:4447

SKNMC1000014//CGAGACAGGGTCTTGGTTTG//SEQ ID NO:4448

SKNMC1000082//TTTCCTTCGGCTGGTATGGC//SEQ ID NO:4449

Y79AA1000030//TCCTACGTTGTGGTGCAGTG//SEQ ID NO:4450

Y79AA1000037//AGCACCCCTTATTGGAAGAT//SEQ ID NO:4451

Y79AA1000127//GATGGTTACTCCTCTTTGGT//SEQ ID NO:4452

Y79AA1000226//TGCTGATGCCTCTGTTCCCT//SEQ ID NO:4453

Y79AA1000270//GAAACAACCAAGCACCCCAT//SEQ ID NO:4454

Y79AA1000750//ATAAGGGCAGCTGGGAAGTG//SEQ ID NO:4455

Y79AA1000776//TGTTAGTAGCAGGAGGAAGC//SEQ ID NO:4456

Y79AA1000777//ACAGAGTTCAGTCCCCTTTA//SEQ ID NO:4457

Y79AA1000876//GTTAGAGGACACTGGCATCA//SEQ ID NO:4458

Y79AA1000888//ACTGACTTGAGGAATAAGCC//SEQ ID NO:4459

Y79AA1000959//AGAGTGAGAGCATGGGAGGT//SEQ ID NO:4460

Y79AA1000967//GGCACAGACACCATCCTTGA//SEQ ID NO:4461

Y79AA1001056//ACAAATGAGCCTGAAAAGTC//SEQ ID NO:4462

Y79AA1001062//TGGTCCTCACTGCCTTCAAA//SEQ ID NO:4463

Y79AA1001090//AGTGCCCTCAAAGCTCCAGT//SEQ ID NO:4464

Y79AA1001212//ACGAAAGCACTCAAATGTCA//SEQ ID NO:4465

Y79AA1001272//GAATGAAATGTGGTTGAGCA//SEQ ID NO:4466

Y79AA1001426//AATGATTCGGGGCAGCAGGA//SEQ ID NO:4467

Y79AA1001427//GAGAGAGACACACACAGAAA//SEQ ID NO:4468

Y79AA1001523//AGTTTATACCAGCATTGGC//SEQ ID NO:4469

Y79AA1001530//GGTGTAGAAGTAAAATGGGA//SEQ ID NO:4470

Y79AA1001592//GATTGTGTTCTCTTACTCCT//SEQ ID NO:4471

Y79AA1001727//GCTCCACCTGACGTTCTTTA//SEQ ID NO:4472

Y79AA1001795//GTCTCCCATATCGCTGTCTT//SEQ ID NO:4473

Y79AA1001803//CACTTTCTAATAACCCTTGG//SEQ ID NO:4474

Y79AA1001863//TTGGGATTGGAAACCGCATT//SEQ ID NO:4475

Y79AA1001874//AGAAACCACTGAGGCCCAAG//SEQ ID NO:4476

Y79AA1002058//CAGAAGCAGAAGCAGGAGCA//SEQ ID NO:4477

Y79AA1002121//ATTTACTGCGATTTCTCCTG//SEQ ID NO:4478

Y79AA1002129//GAGTTTCTTTGCTAGTTCCA//SEQ ID NO:4479

Y79AA1002334//ATATTTGTGTTTGCCTTGGG//SEQ ID NO:4480

Y79AA1002373//GGATGGCTGGGTCAAATGGT//SEQ ID NO:4481

Y79AA1002376//AATGATGGCTAGGGTGACTT//SEQ ID NO:4482

Y79AA1002378//TCTTCCCACATTCGTTACAC//SEQ ID NO:4483

Y79AA1002381//AGGGAGTAGATGTTGGTAAA//SEQ ID NO:4484

The result of expression frequency analysis is shown in Table 367. Only clones with correlation coefficient of 0.9 or higher are indicated in this Table. Clones that are not presented in the Table include clones for which the assay could not performed because of low expression levels thereof in internal standard samples or because of unexpectedly smaller or larger sizes of the PCR products.

Among the clones that could be analyzed, clones of which expression levels increased by two fold in response to the IL-1β stimulation 1 or 7 hours after the stimulation are: NT2RM2000514, NT2RP3001159, MAMMA1001237 and MAMMA1000614.

Clones of which expression levels increased by two fold in response to the TNF-α stimulation 1, 3 or 7 hours after the stimulation are:
NT2RM2000582, NT2RM2002109, NT2RP1000679, NT2RP2003664, NT2RP2005597, NT2RP2004108, NT2RP3001592, NT2RP3002738, NT2RP3004133, NT2RP3004321, NT2RP3004557, NT2RP3004294, MAMMA1001237, MAMMA1000141, MAMMA1000788, MAMMA1002070, PLACE1002547, PLACE1003573, PLACE1004305, PLACE1008744, PLACE1011181, PLACE1010713, PLACE1010011, Y79AA1000776, Y79AA1002129,

Among the clones of which expression levels increased in response to IL-1β stimulation, MAMMA1001237 was a clone of which expression level was varied in response to TNF-α stimulation. Among clones showing higher expression levels (with relative value of 5 or higher) prior to the stimulation, PLACE1002080 is an example of clones of which expression was suppressed by the stimulation. The expression of the clone decreased by three or more fold in response to the stimulation. These genes were found to be associated with inflammatory reaction induced by IL-1β or TNF-α.

In Example 15, the genes of which expression levels were varied by culturing in the presence of TNF-α were analyzed by hybridization with high-density DNA filter. As for 3 clones (NT2RP3004557, NT2RP3004294 and PLACE1002547), the results obtained by ATAC-PCR method were similar to those obtained by hybridization method. However, the results obtained by ATAC-PCR method were not necessarily consistent with those obtained by the hybridization method. Possible reasons for the inconsistency are the difference in cells used between the two experiments, unavailability of some data in the ATAC-PCR experiment, and the difference in the method of data treatment.

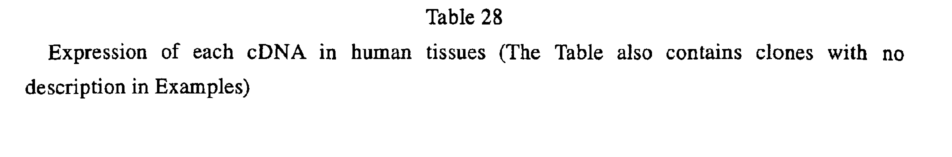

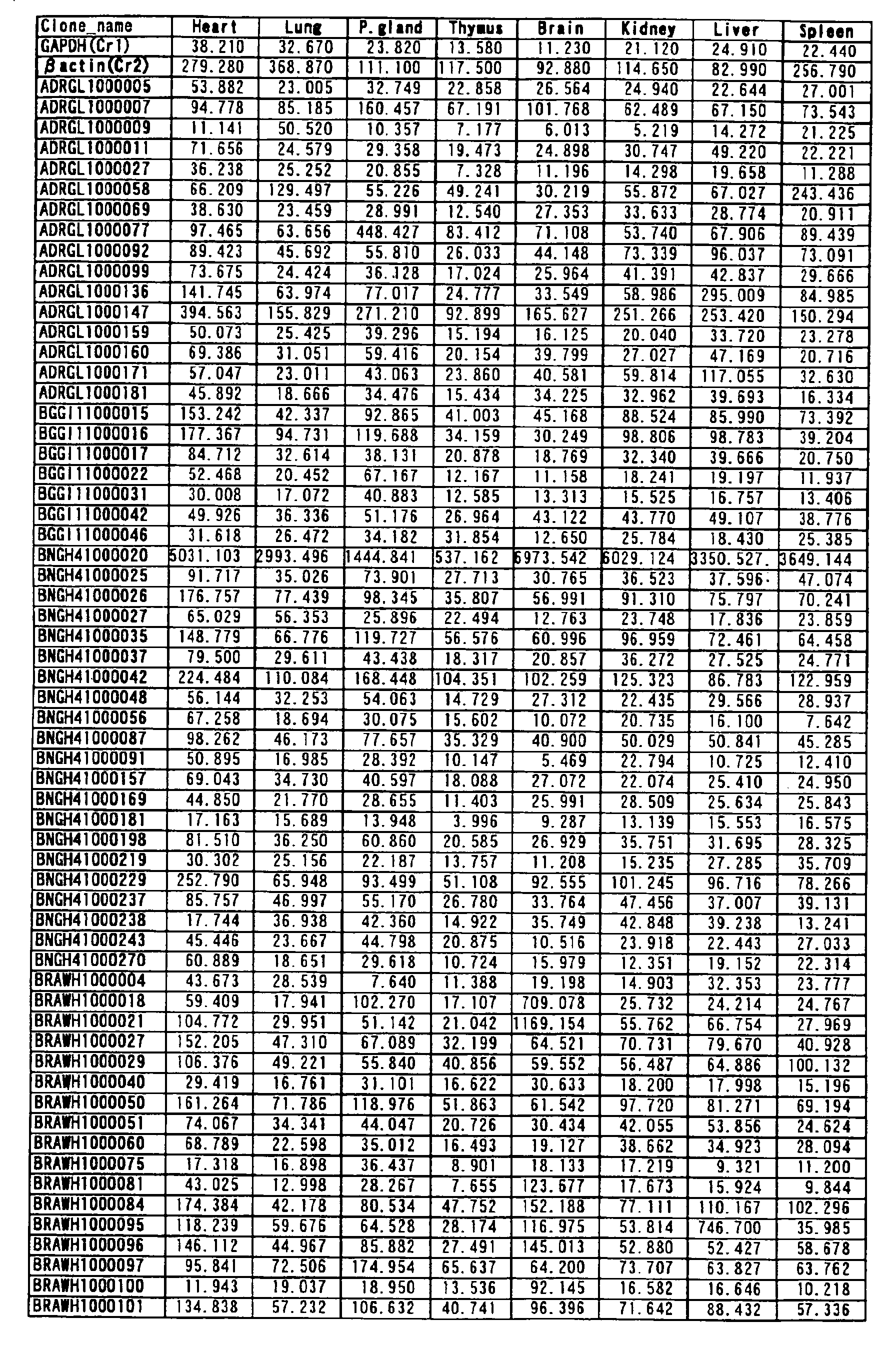

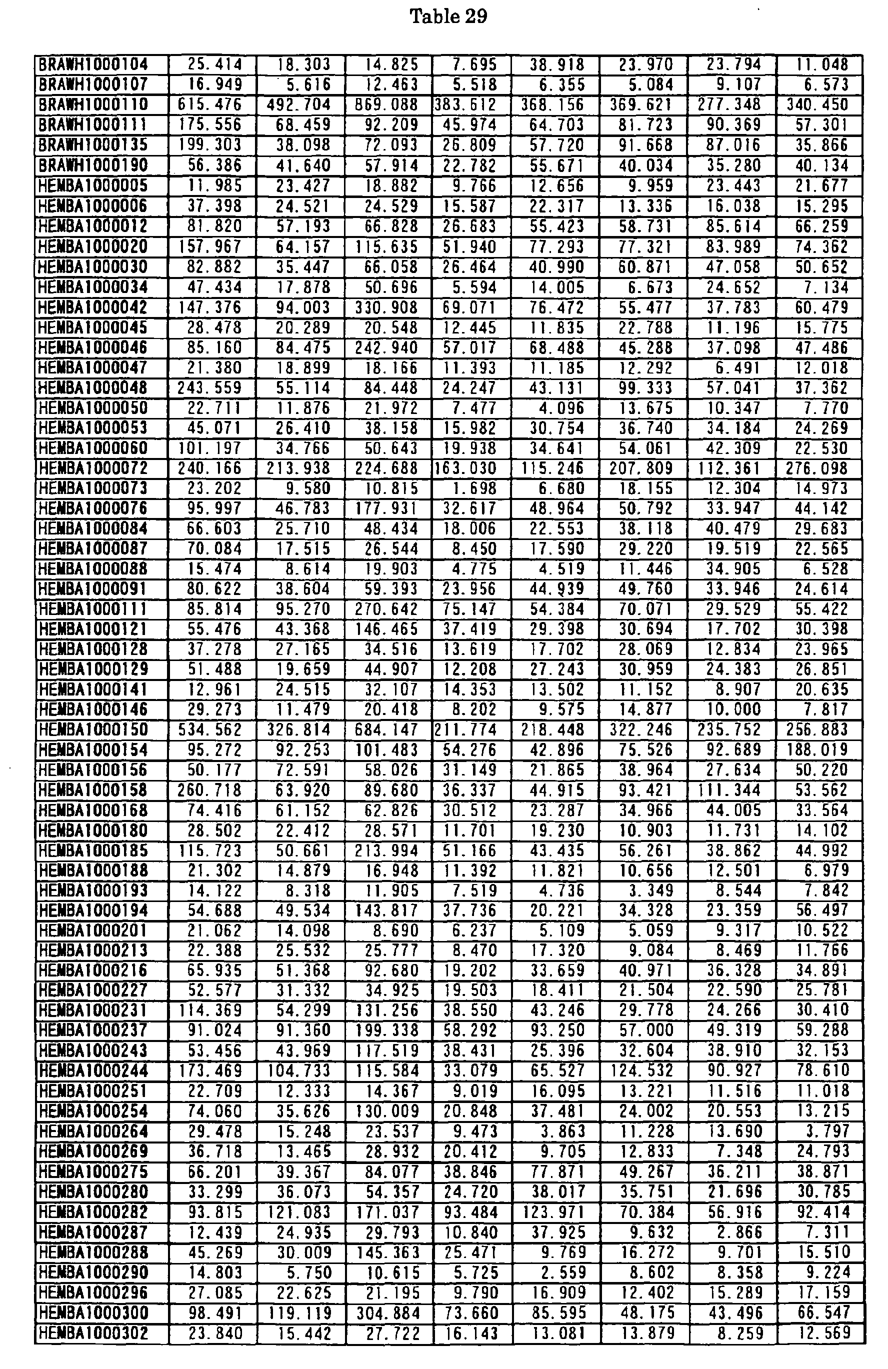

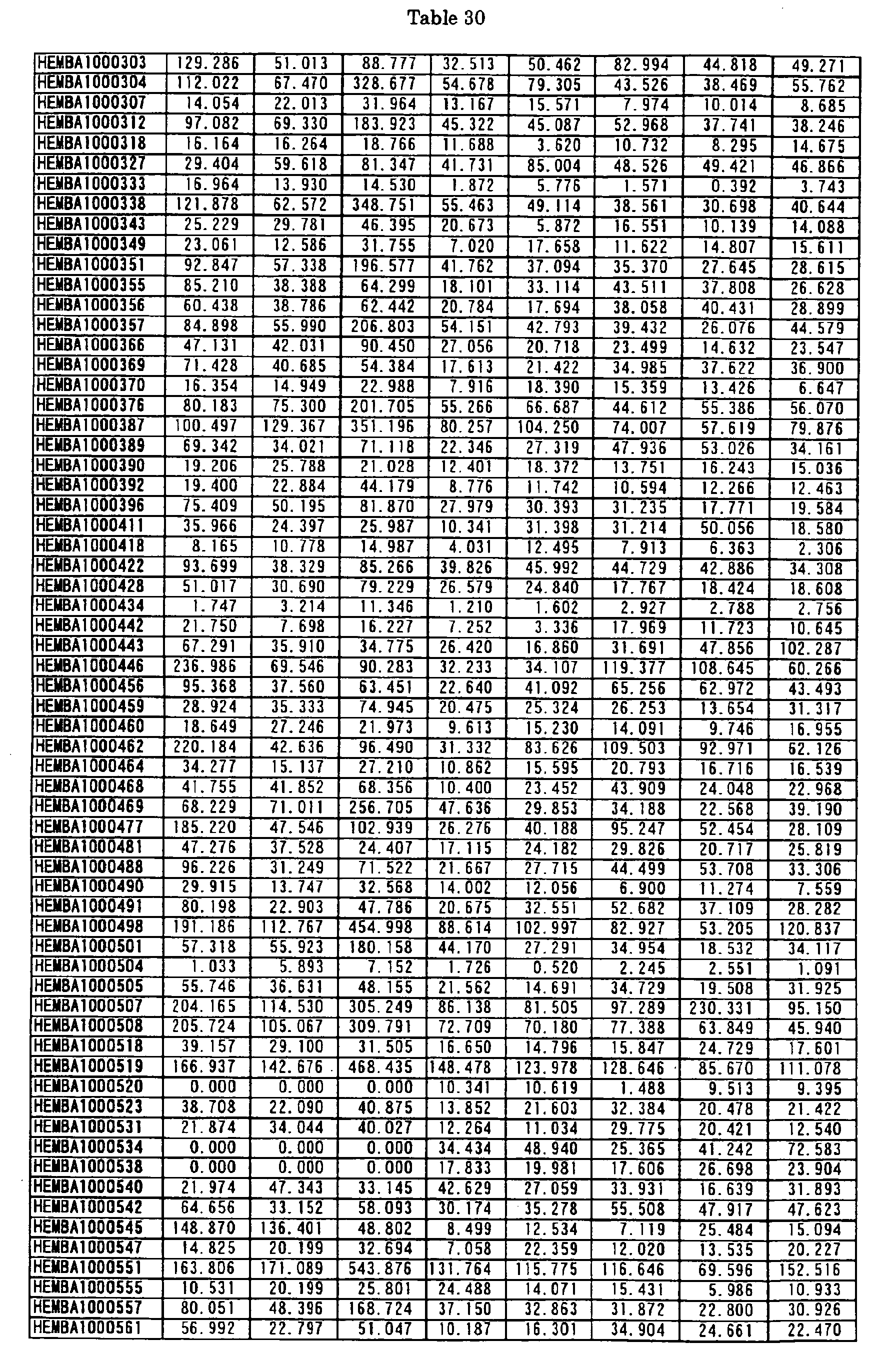

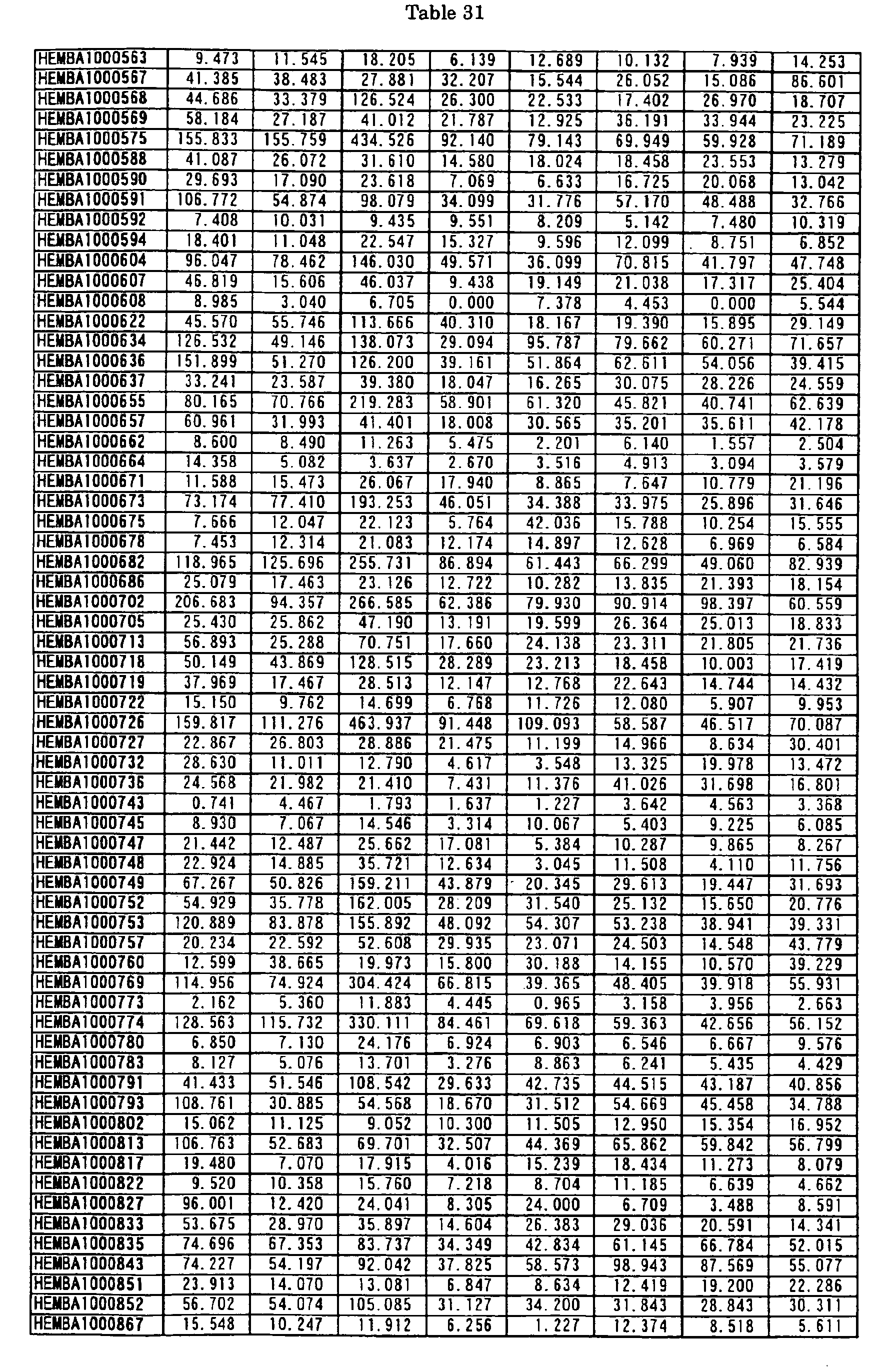

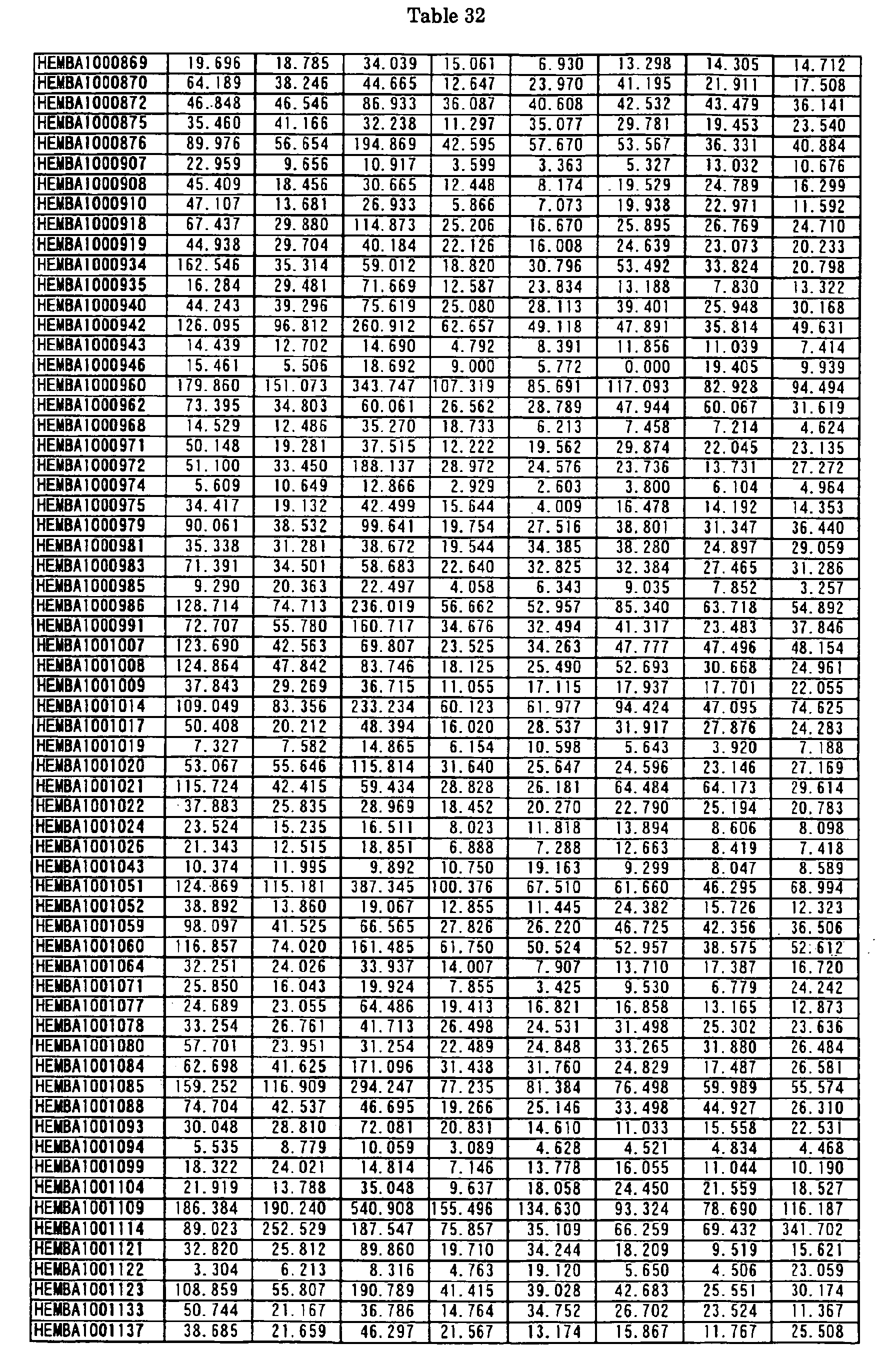

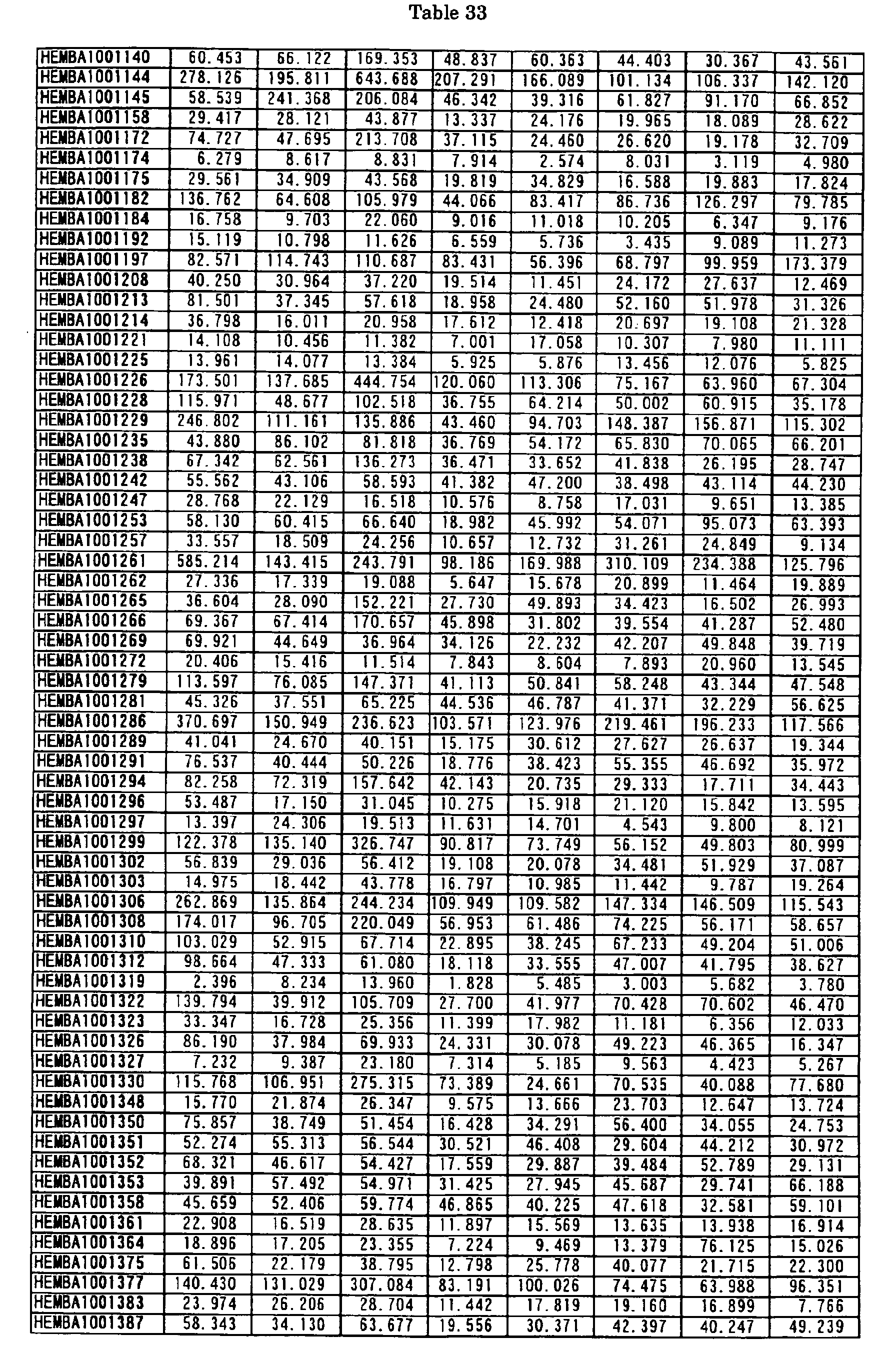

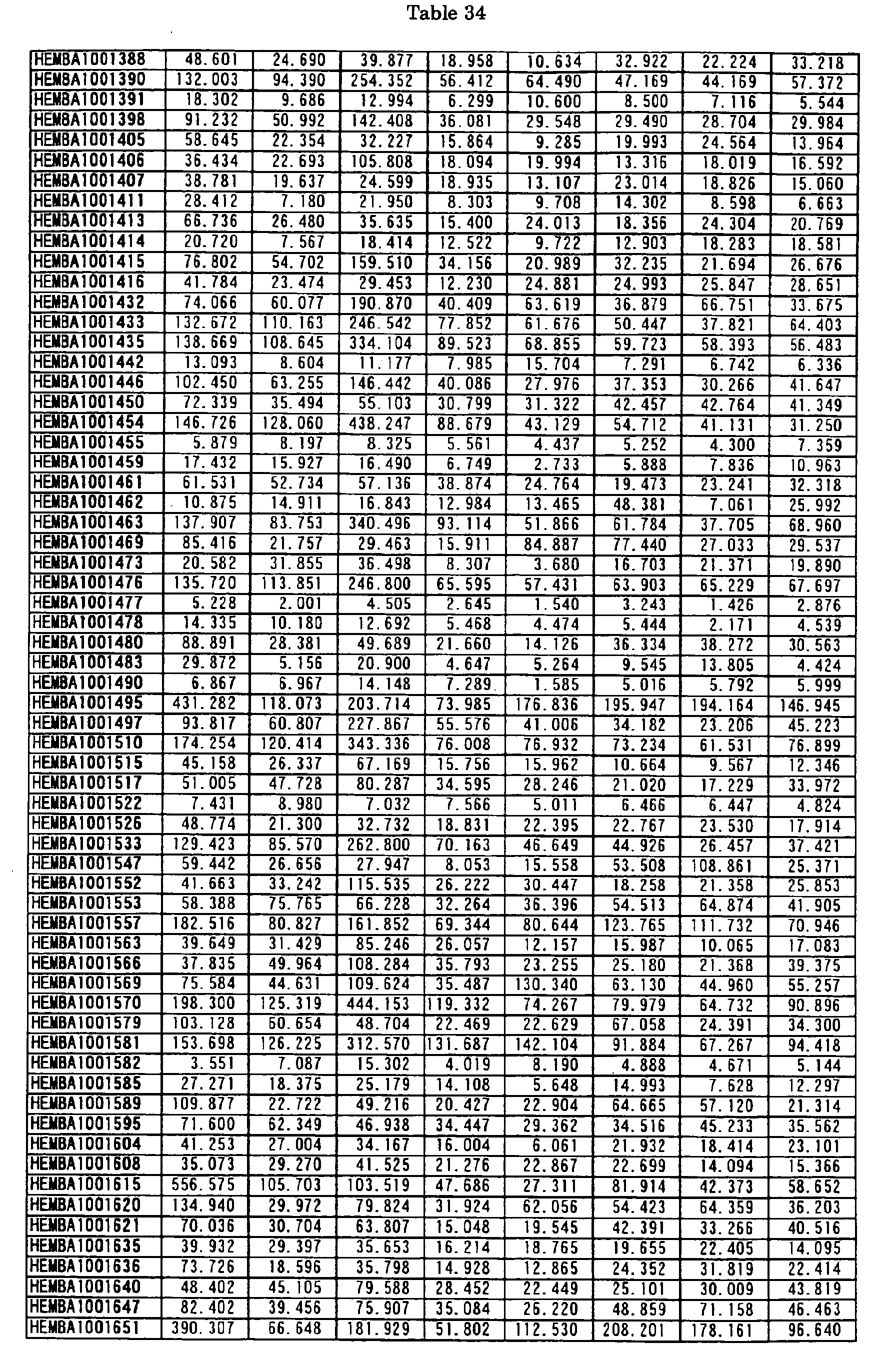

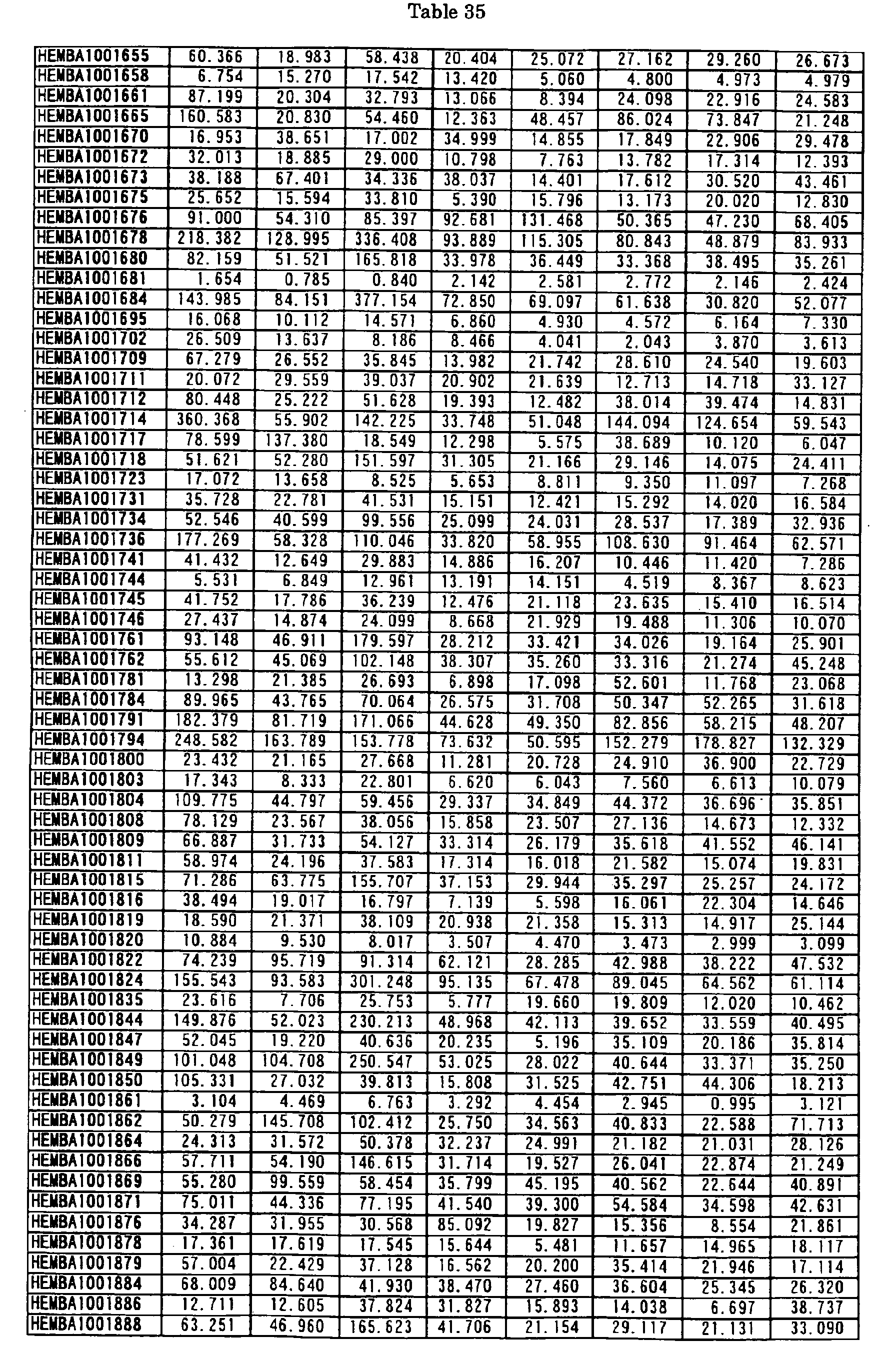

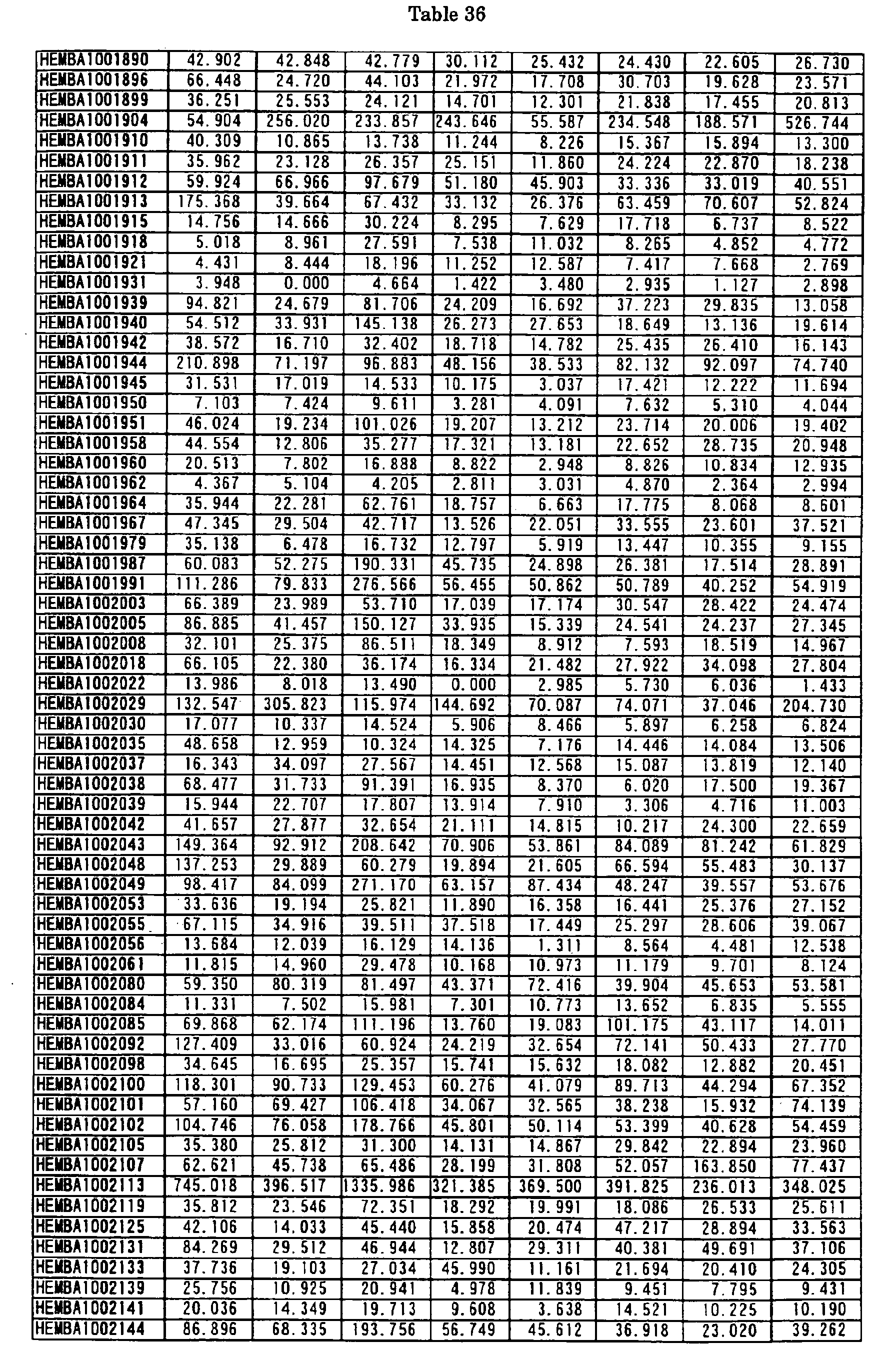

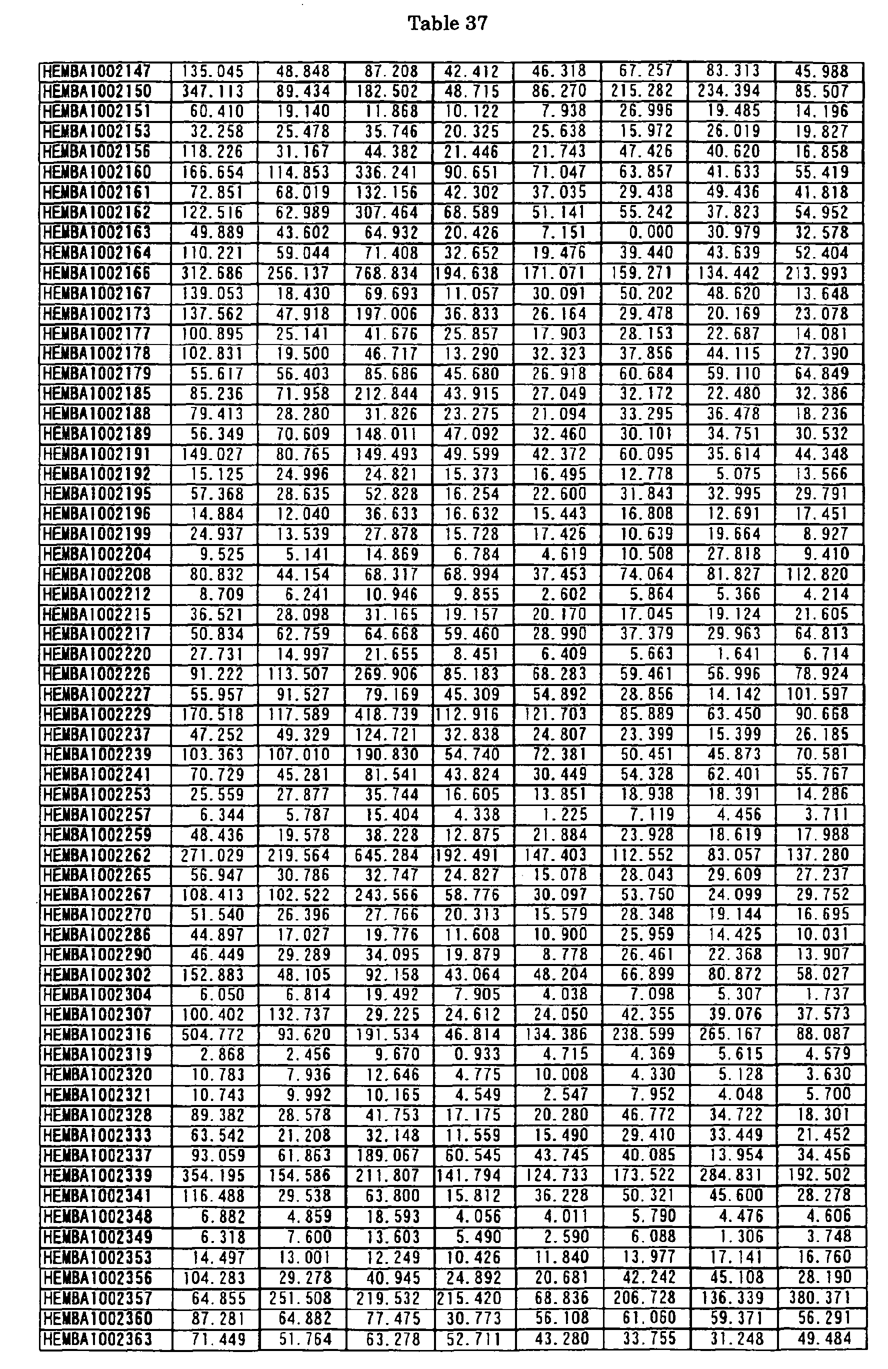

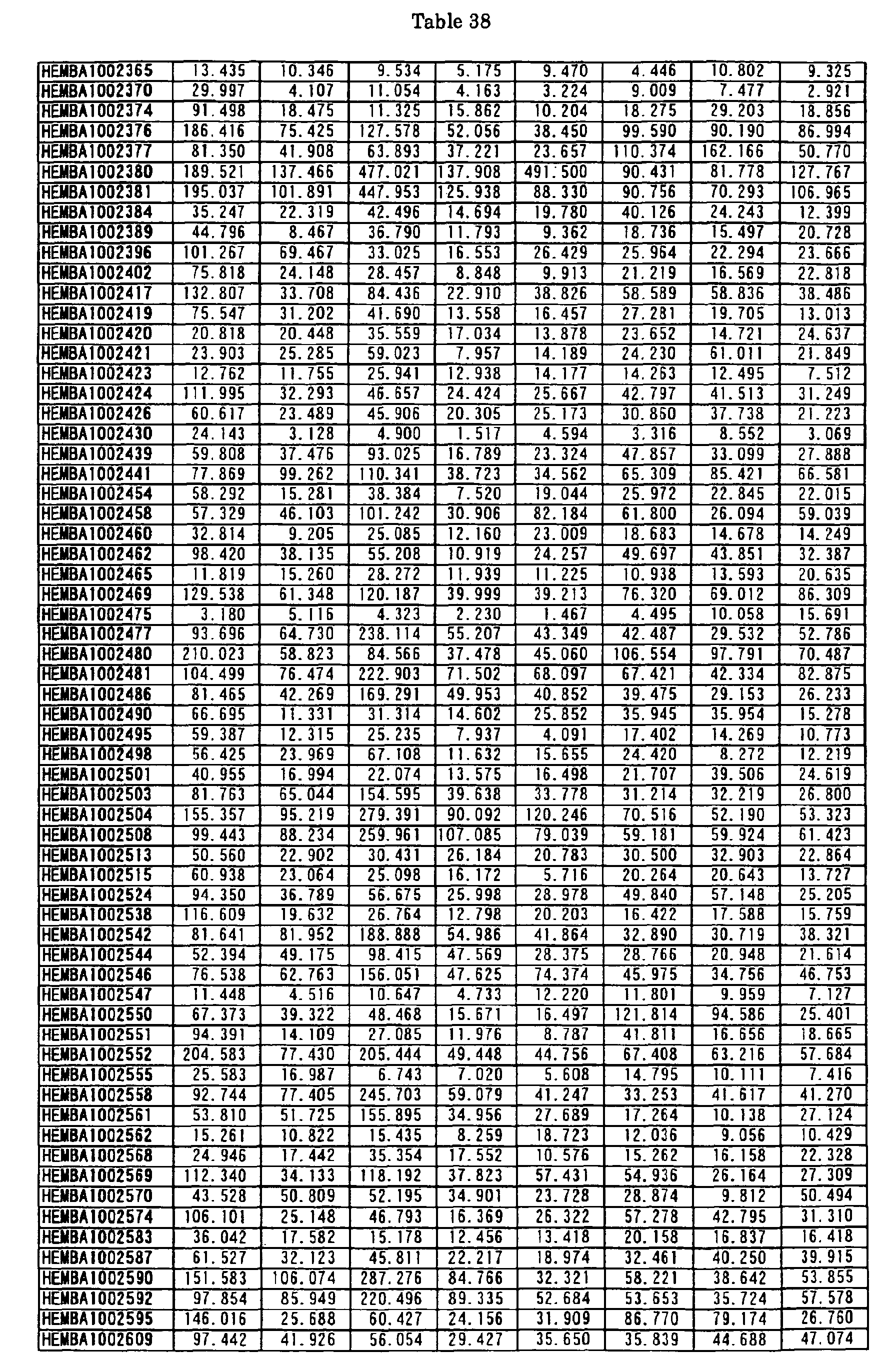

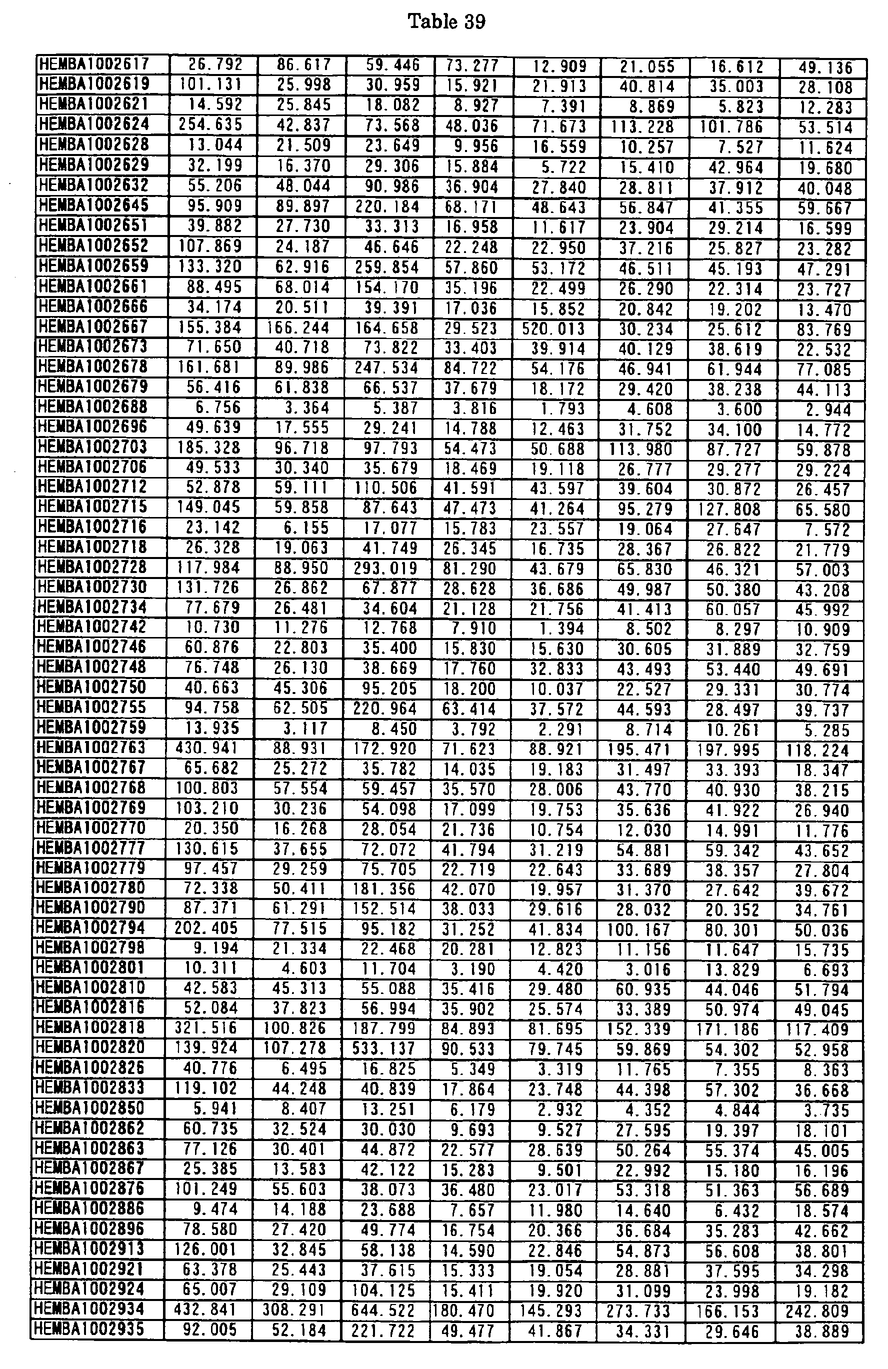

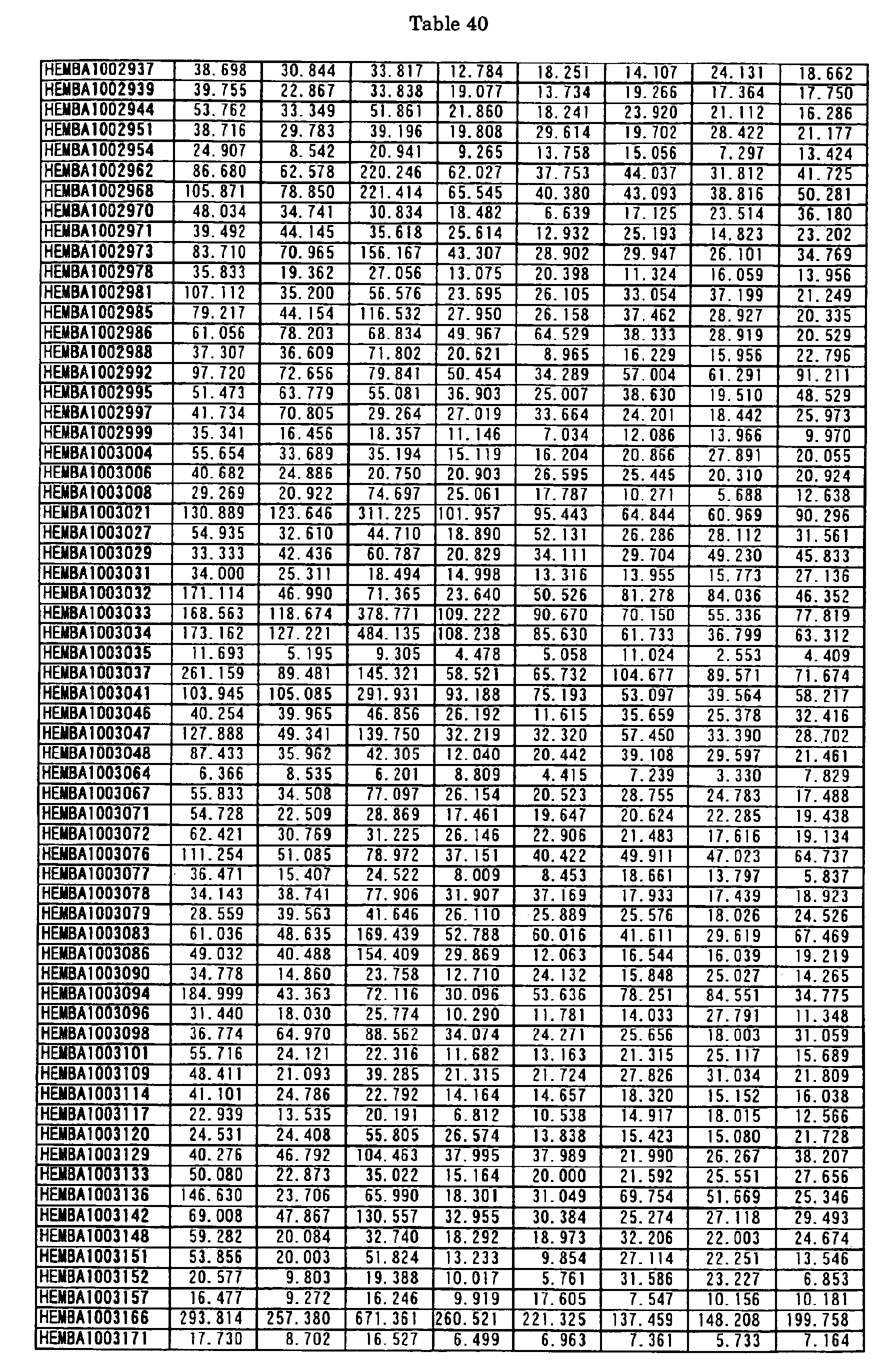

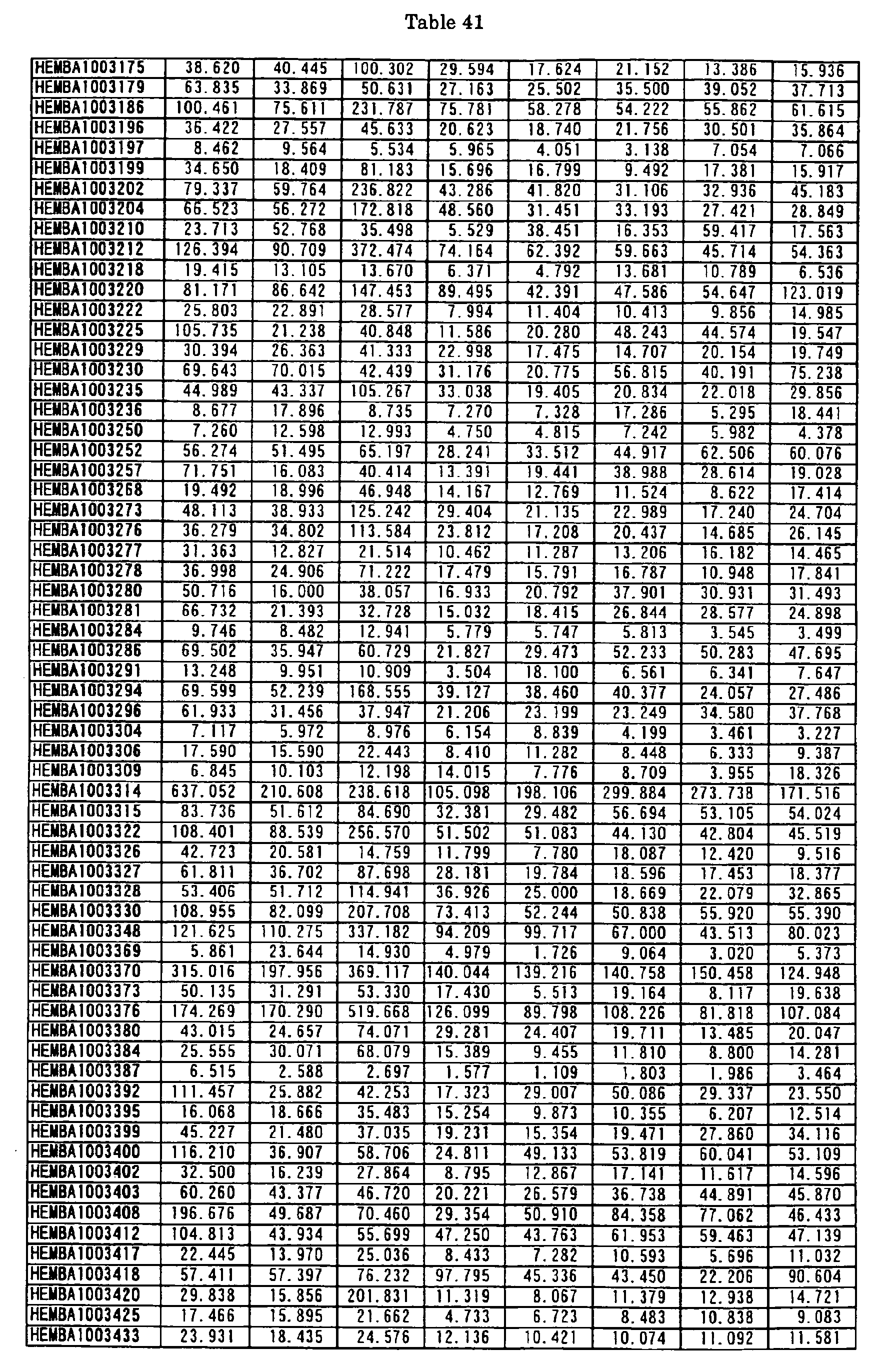

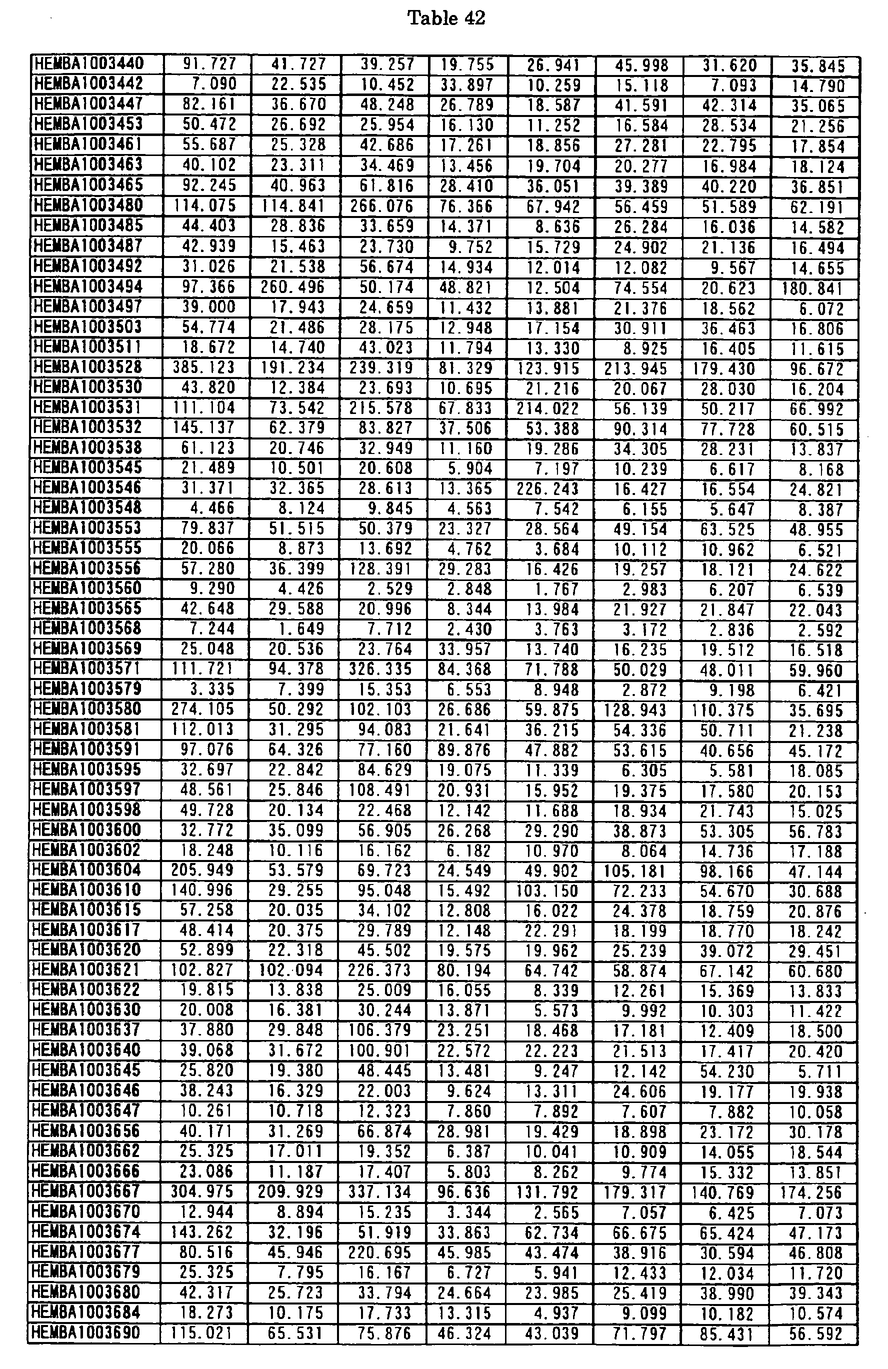

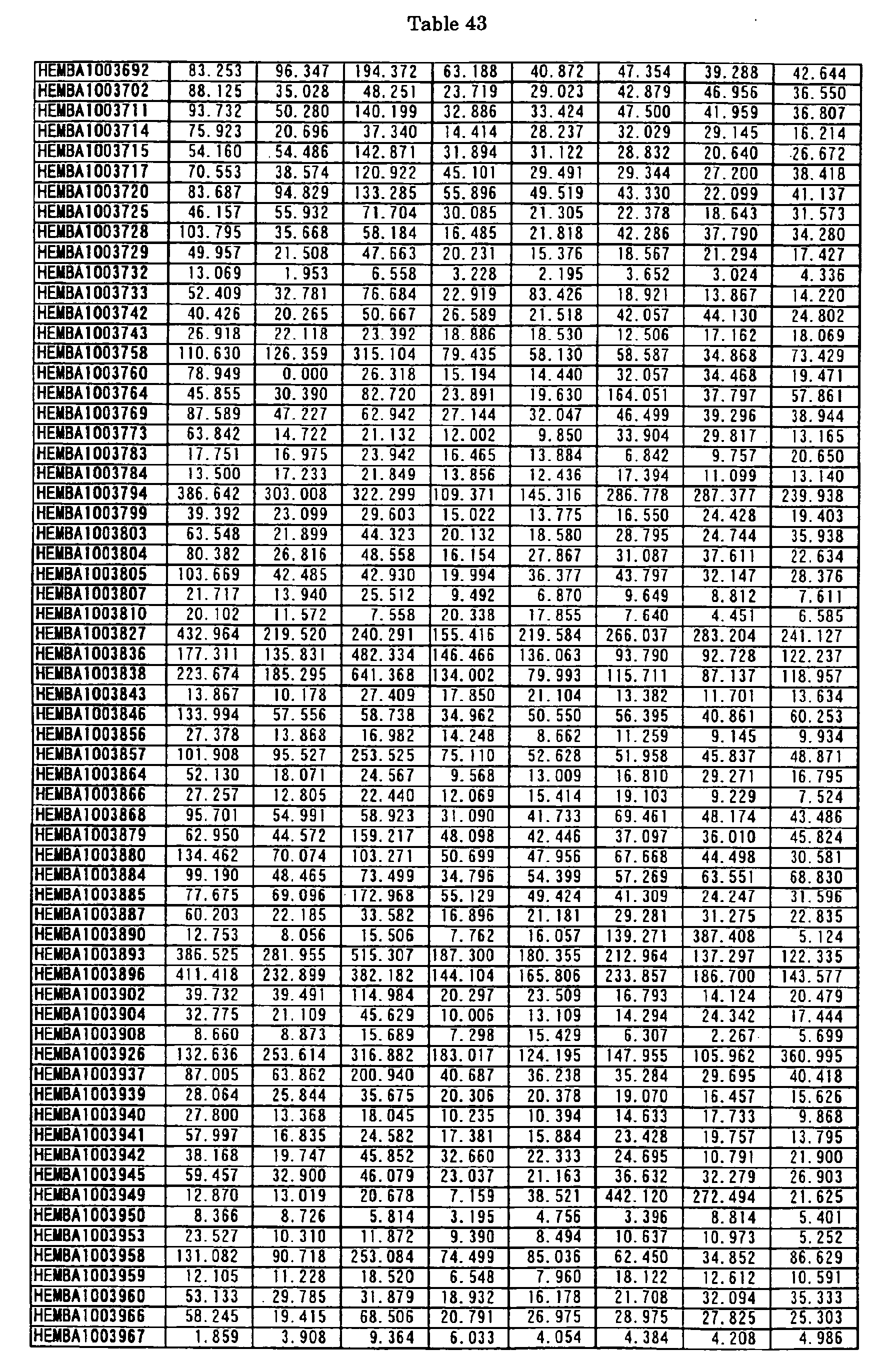

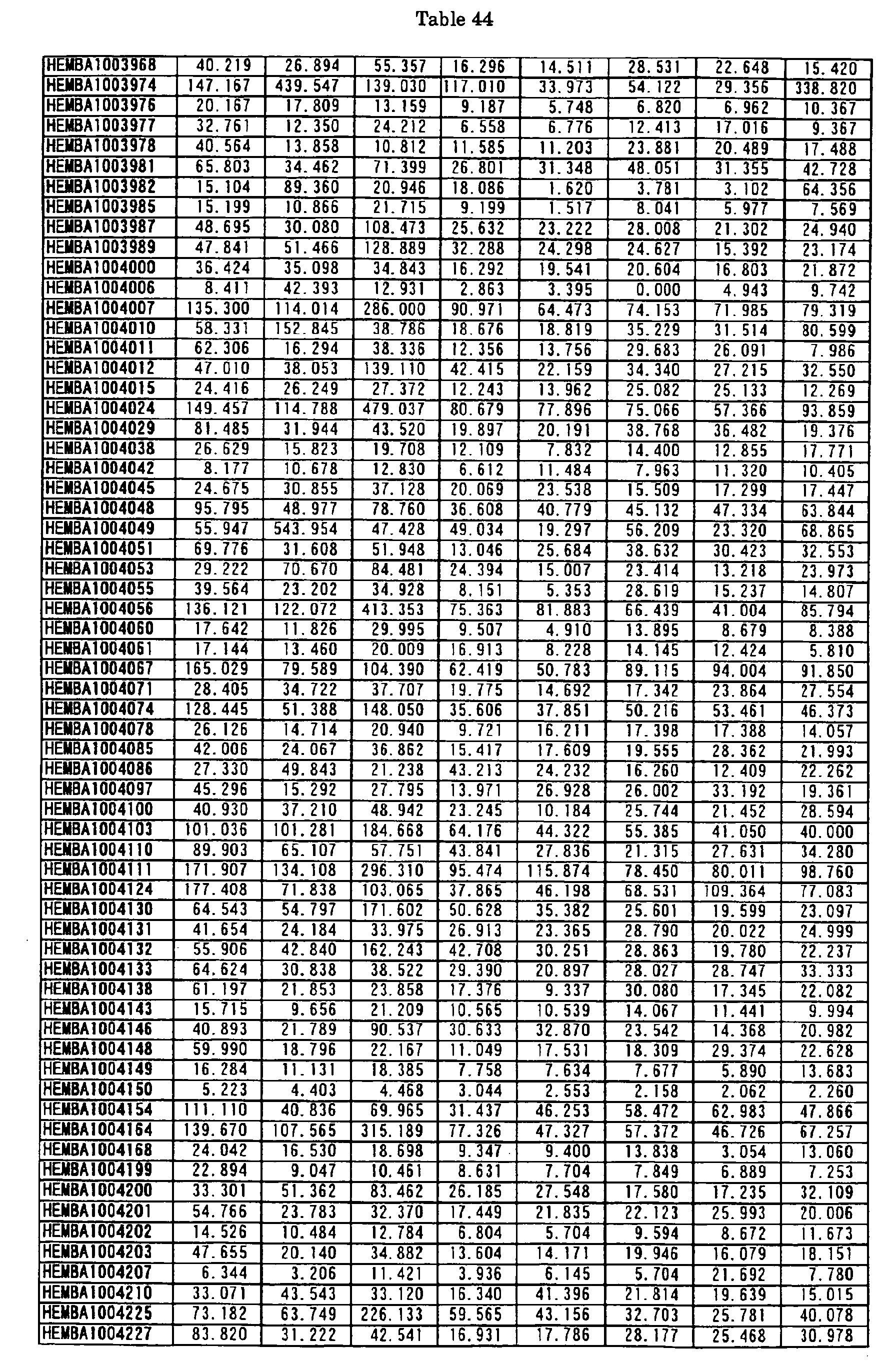

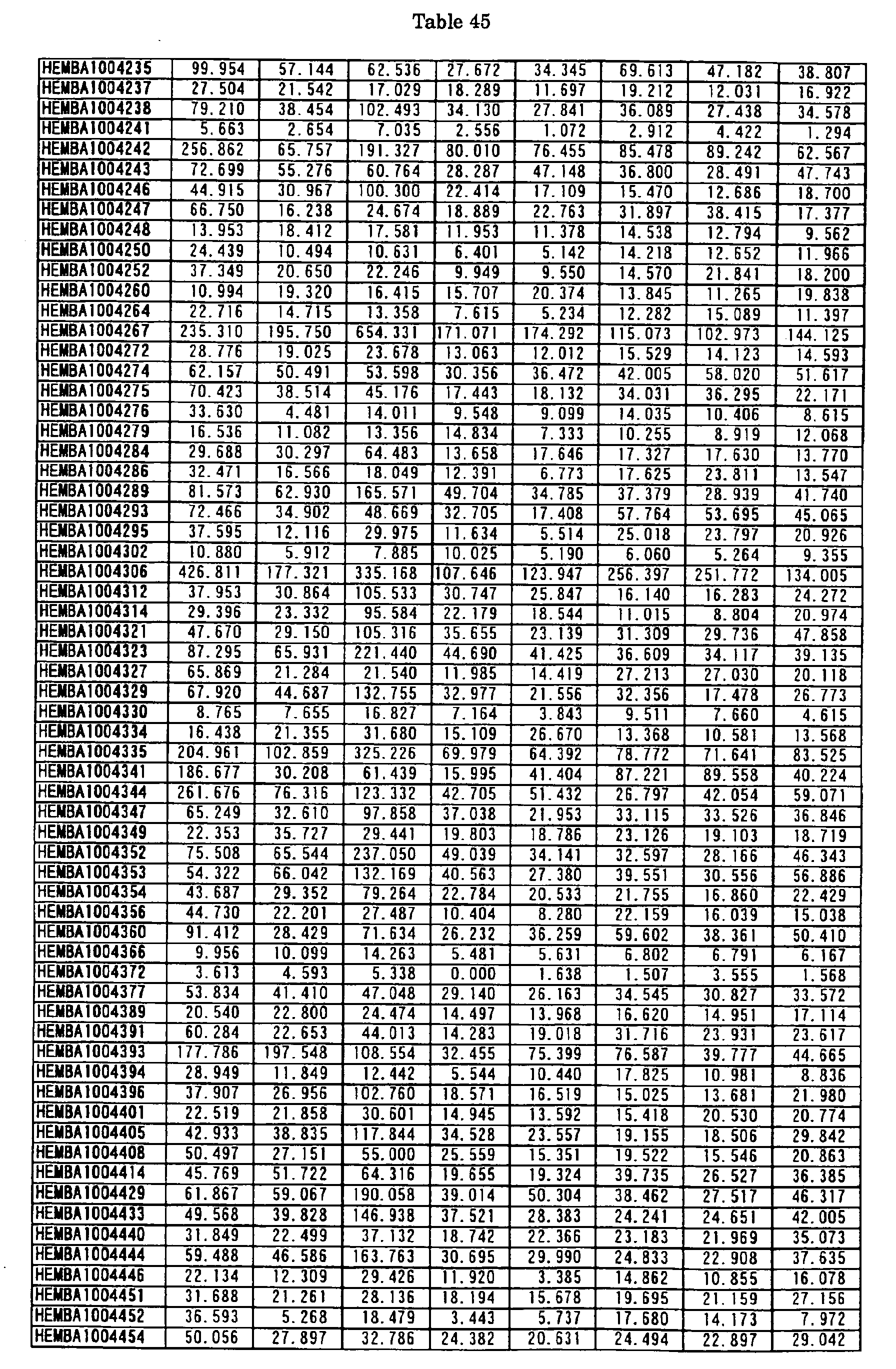

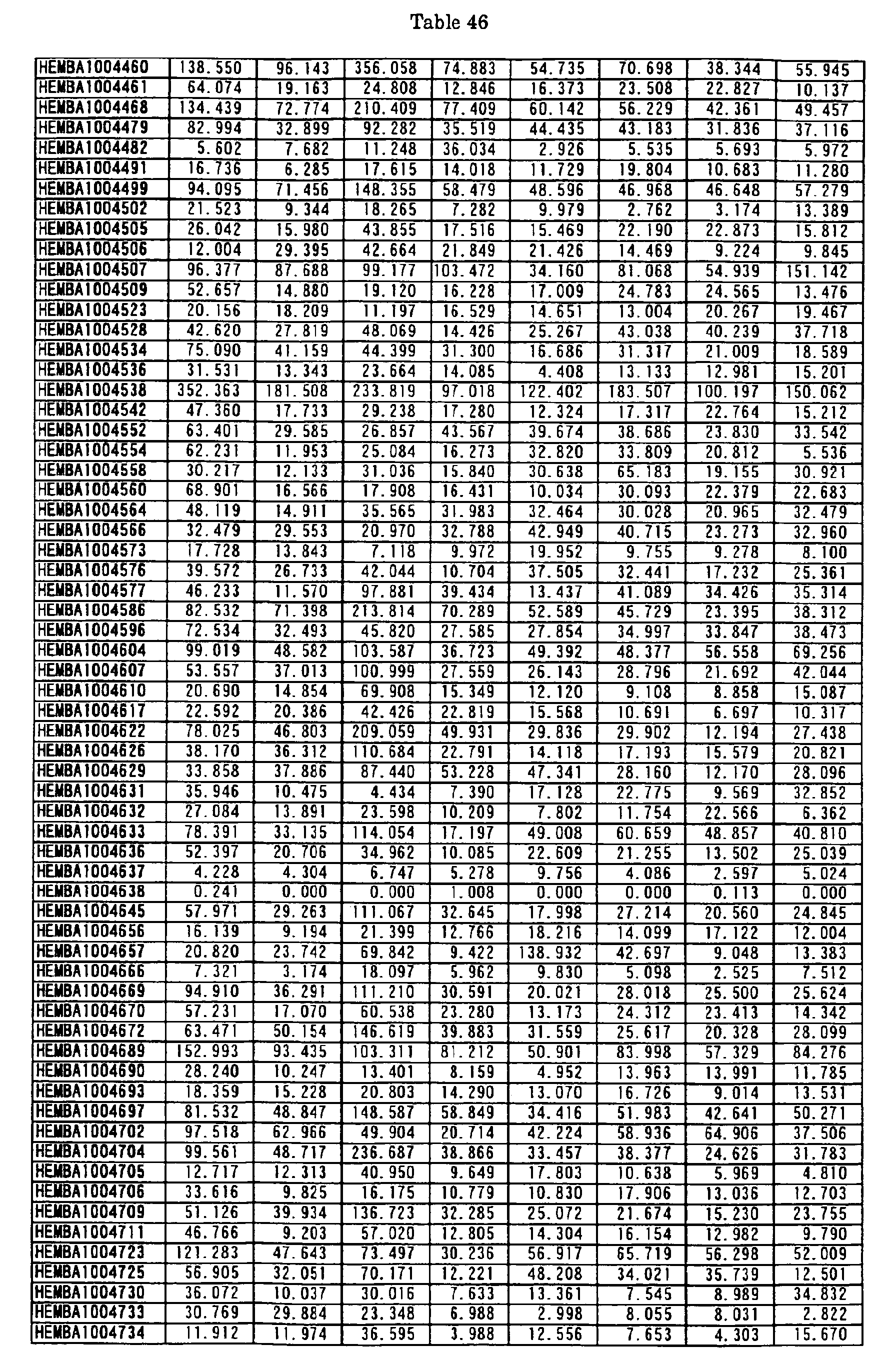

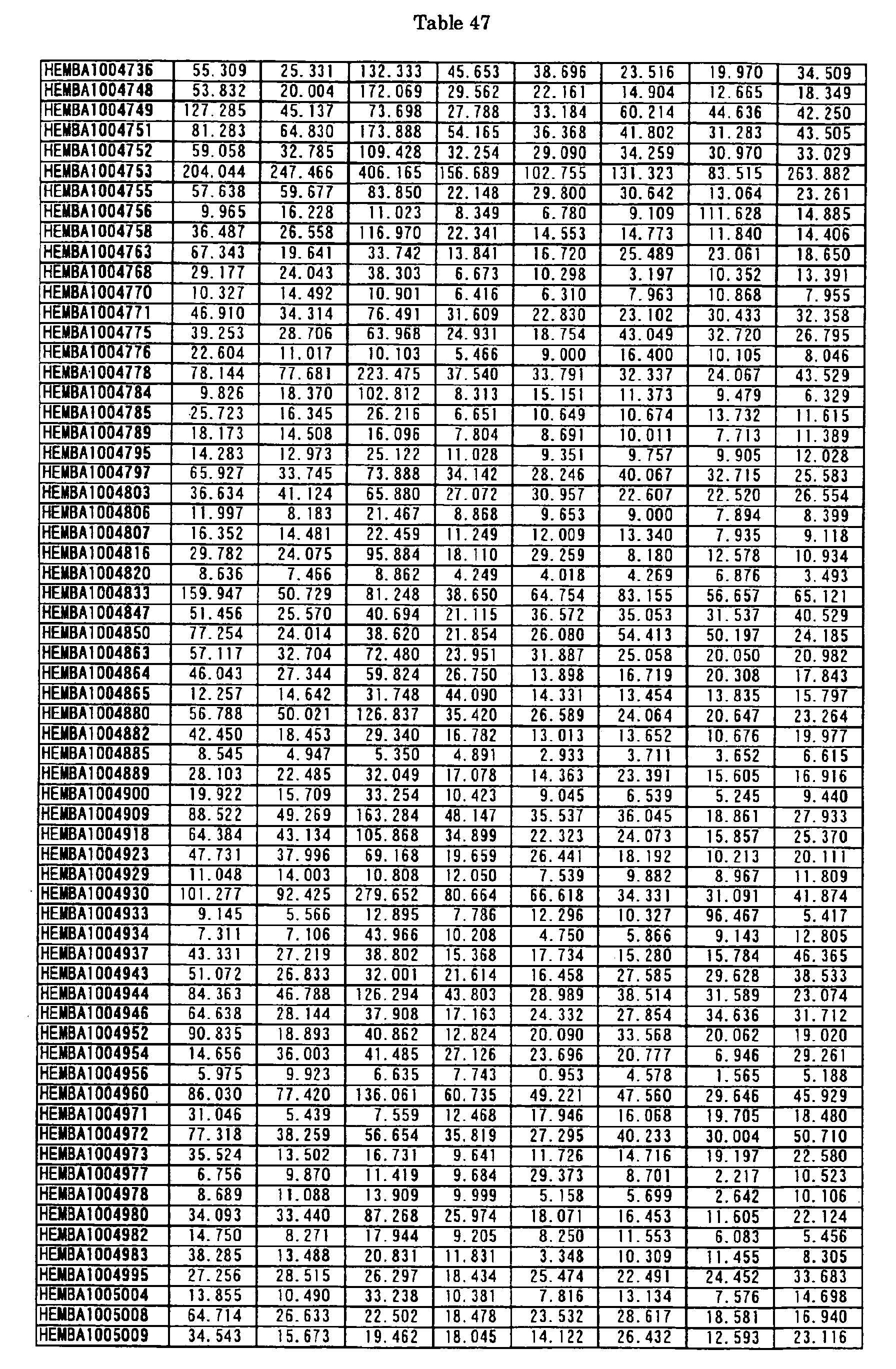

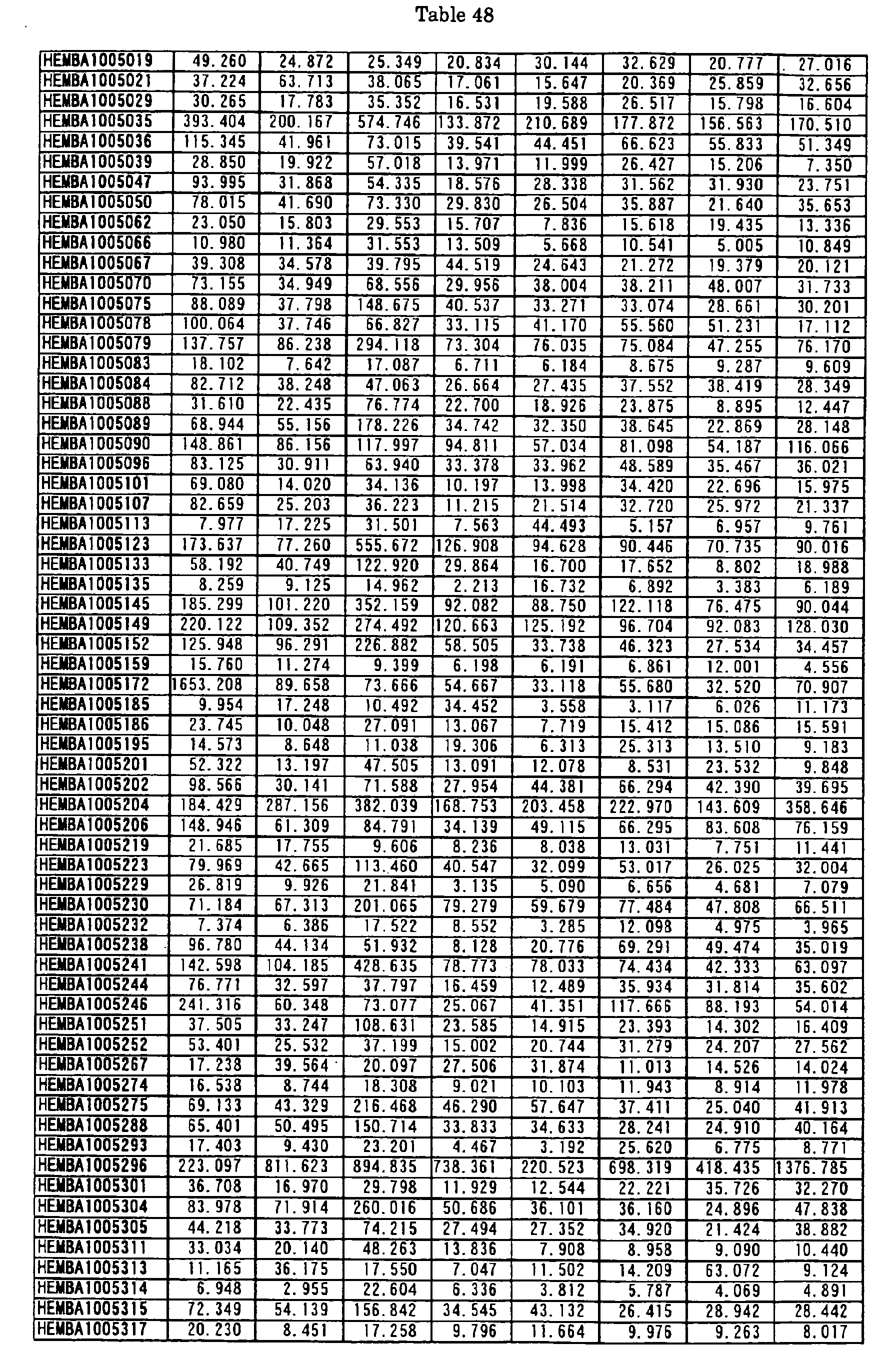

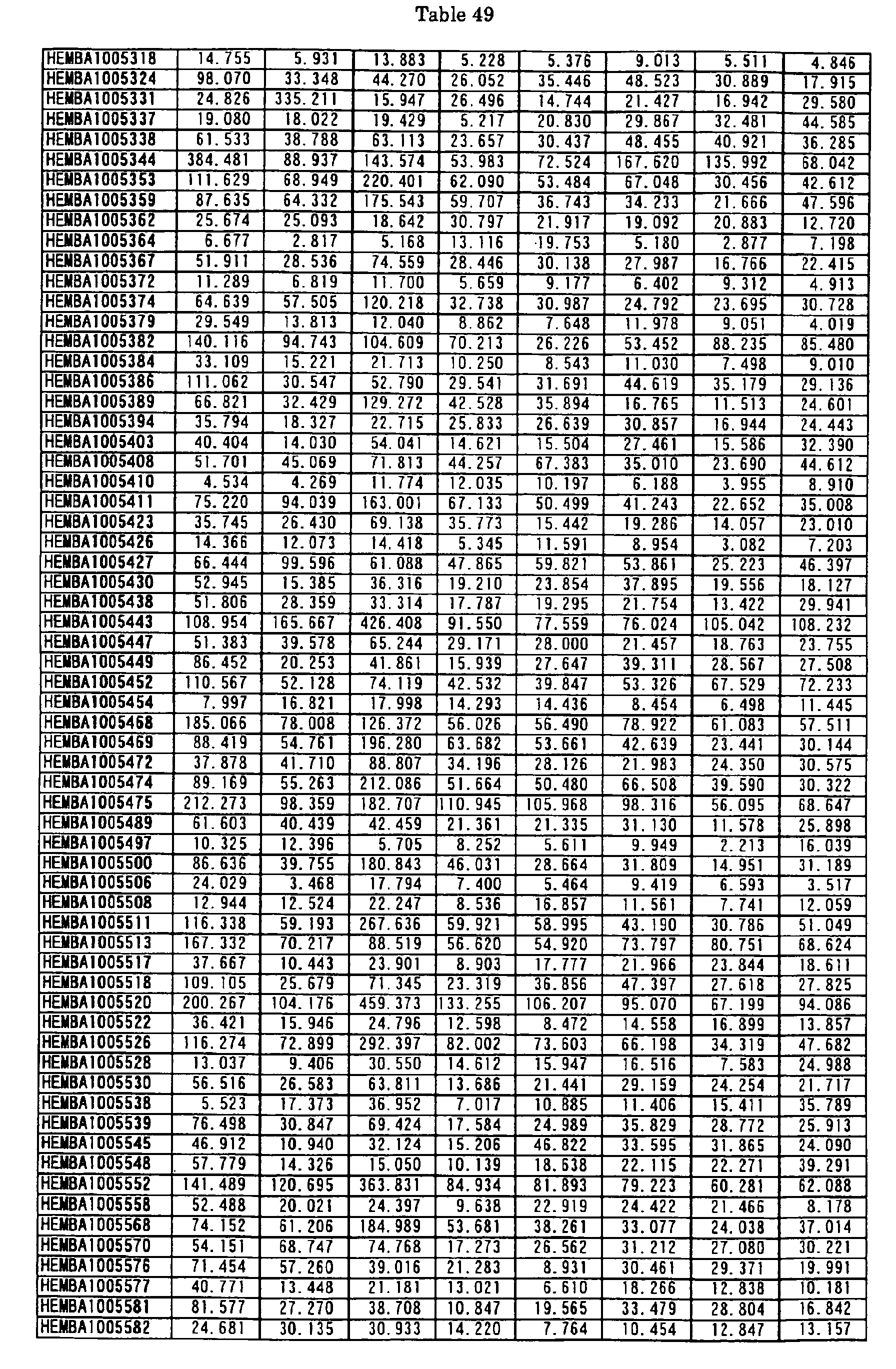

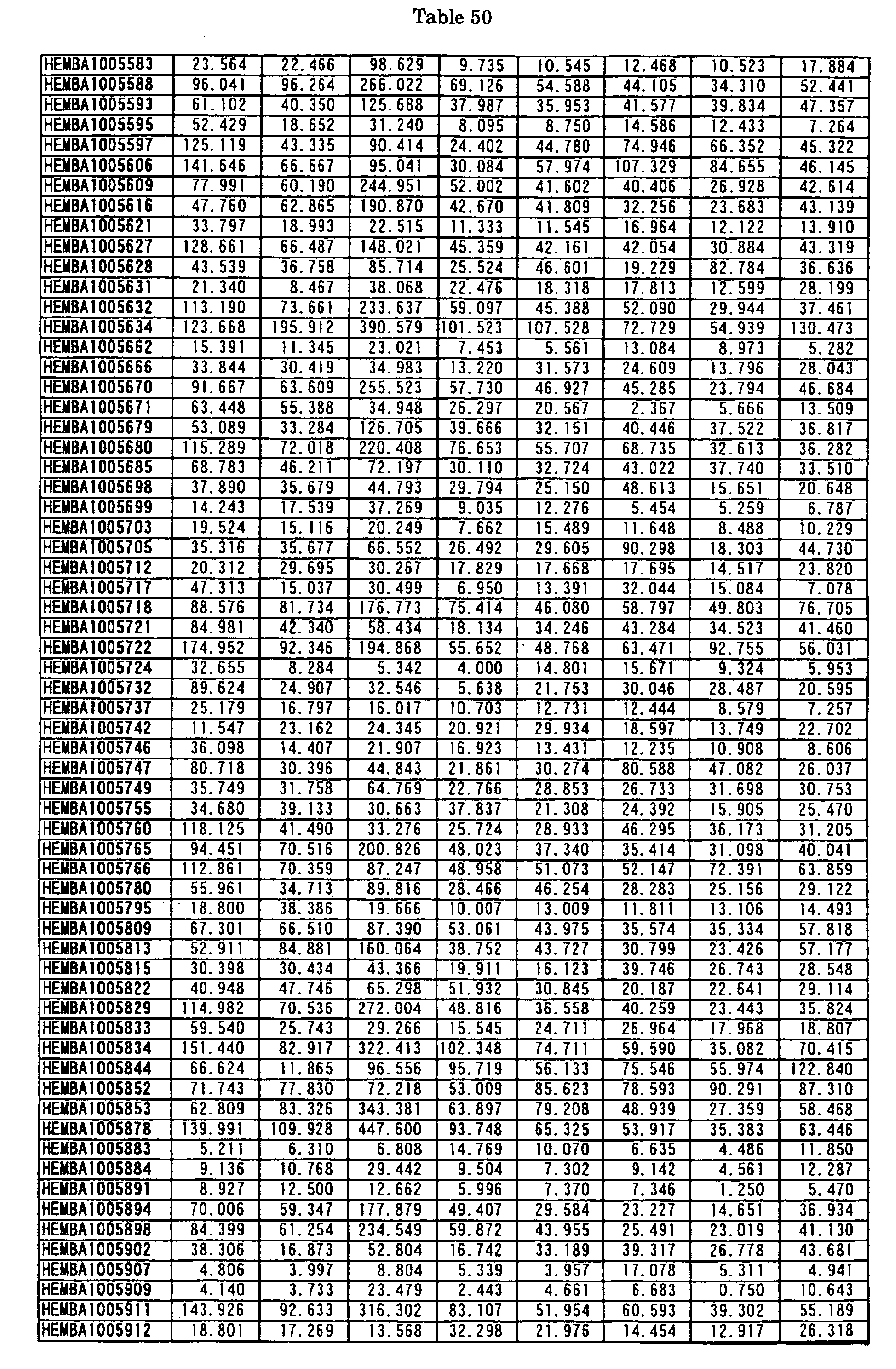

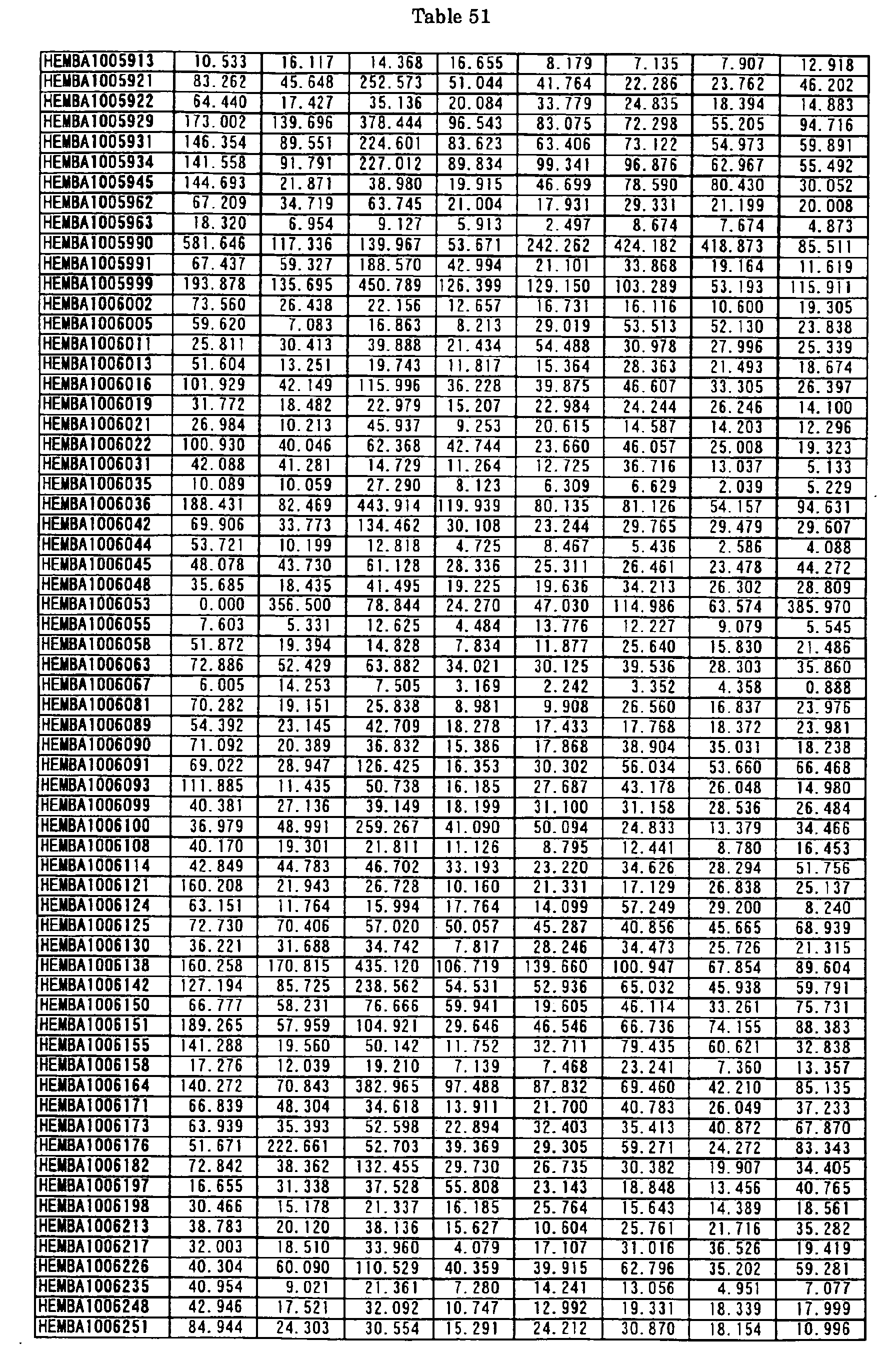

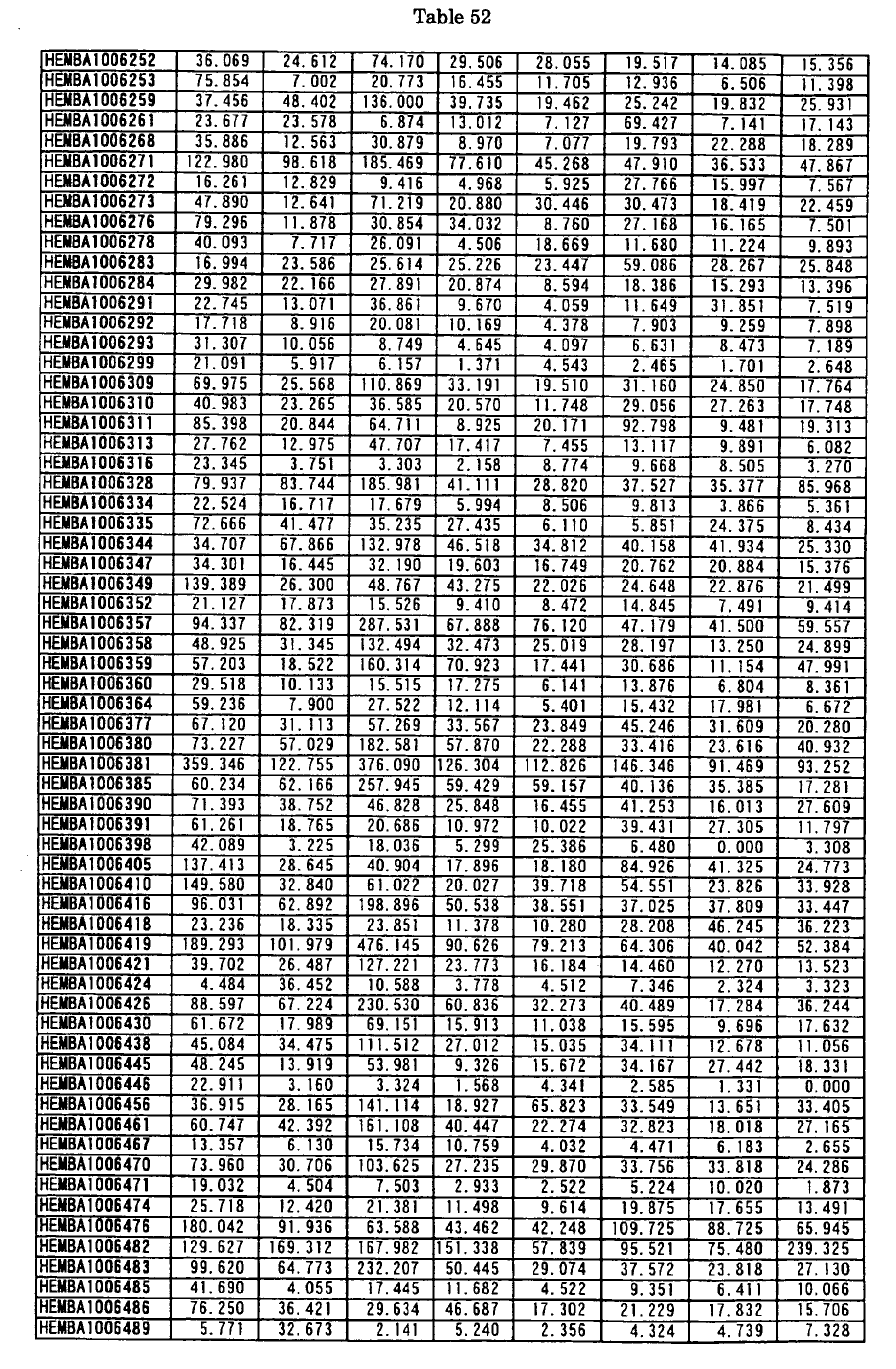

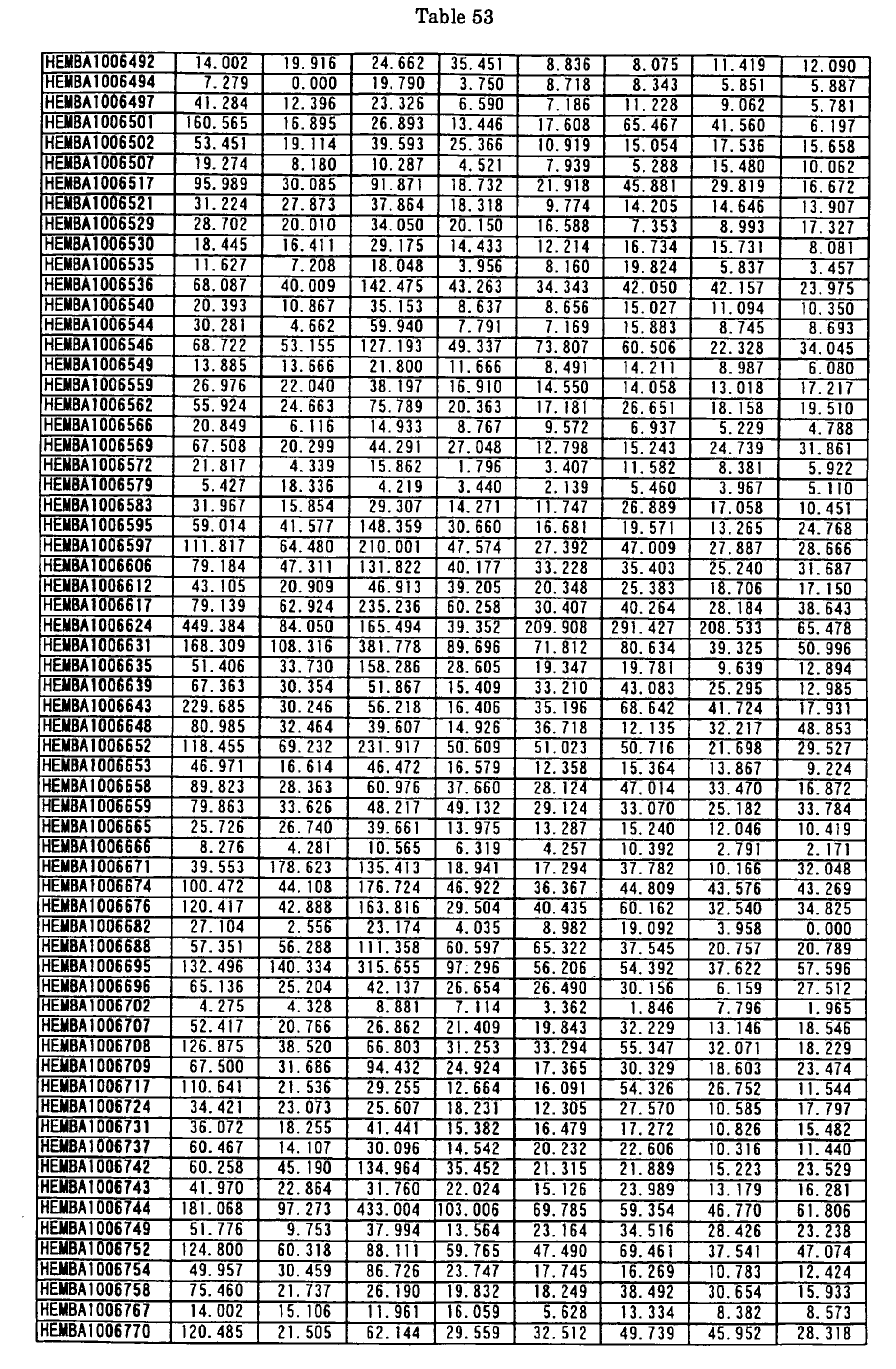

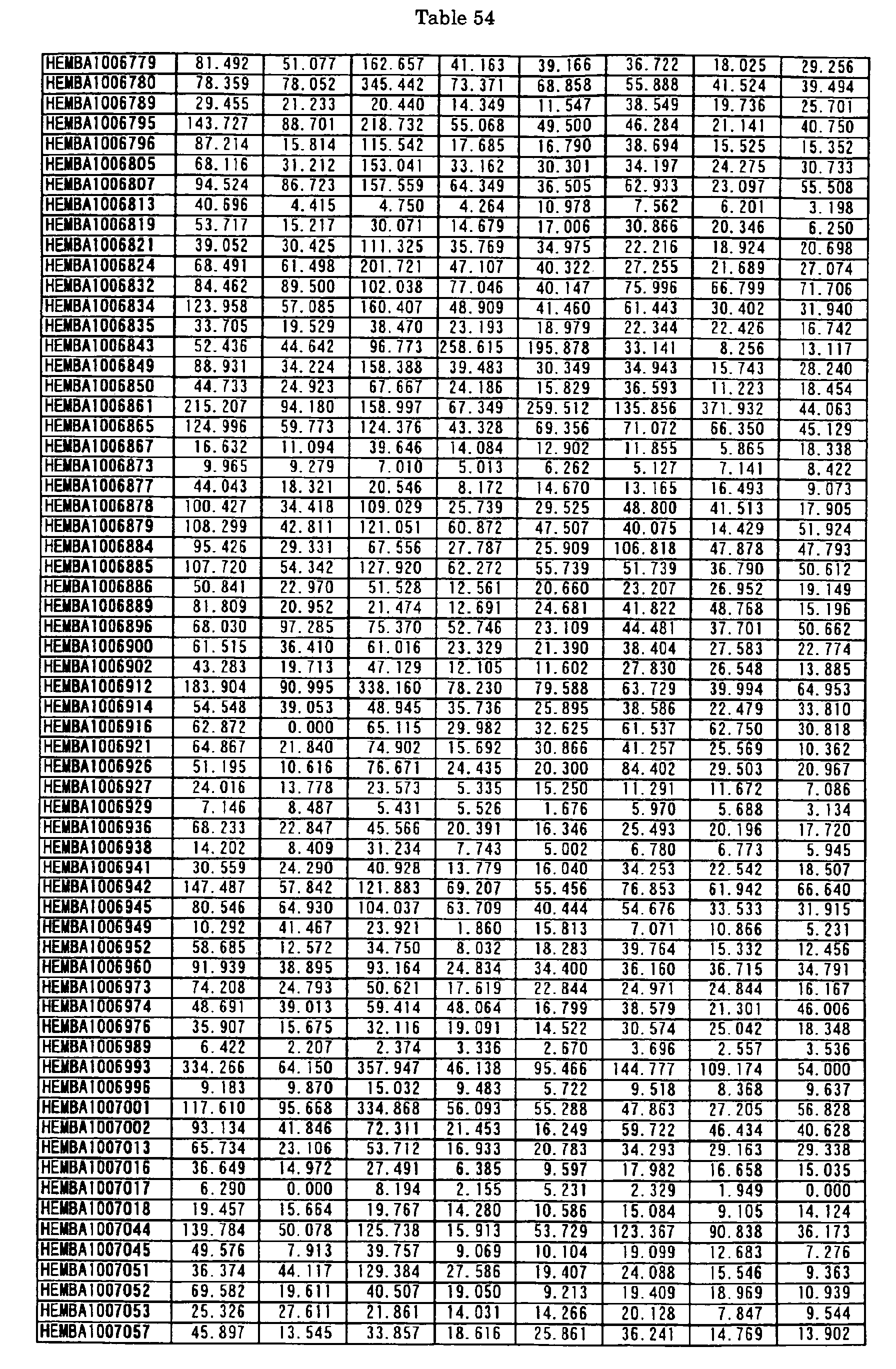

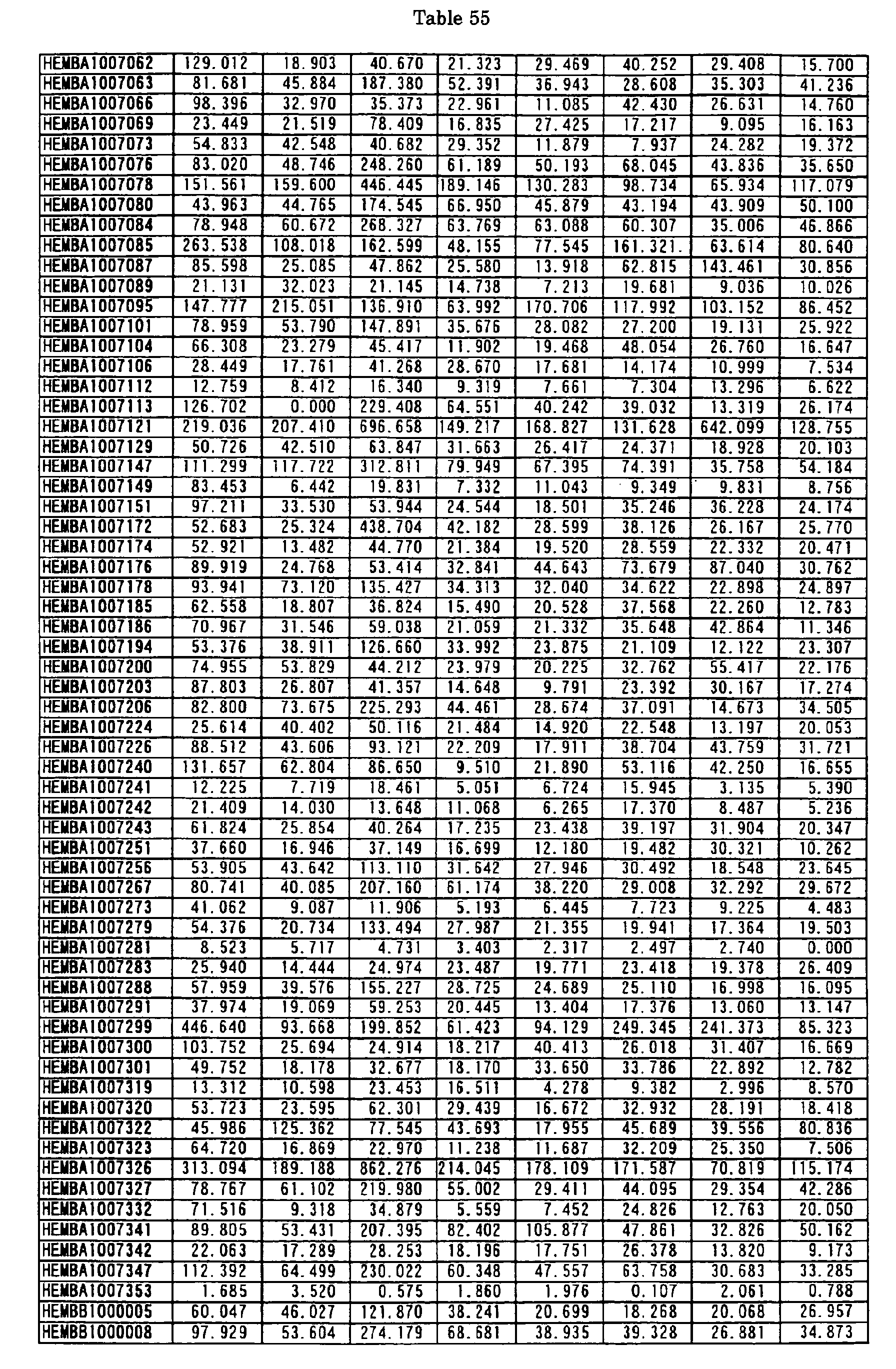

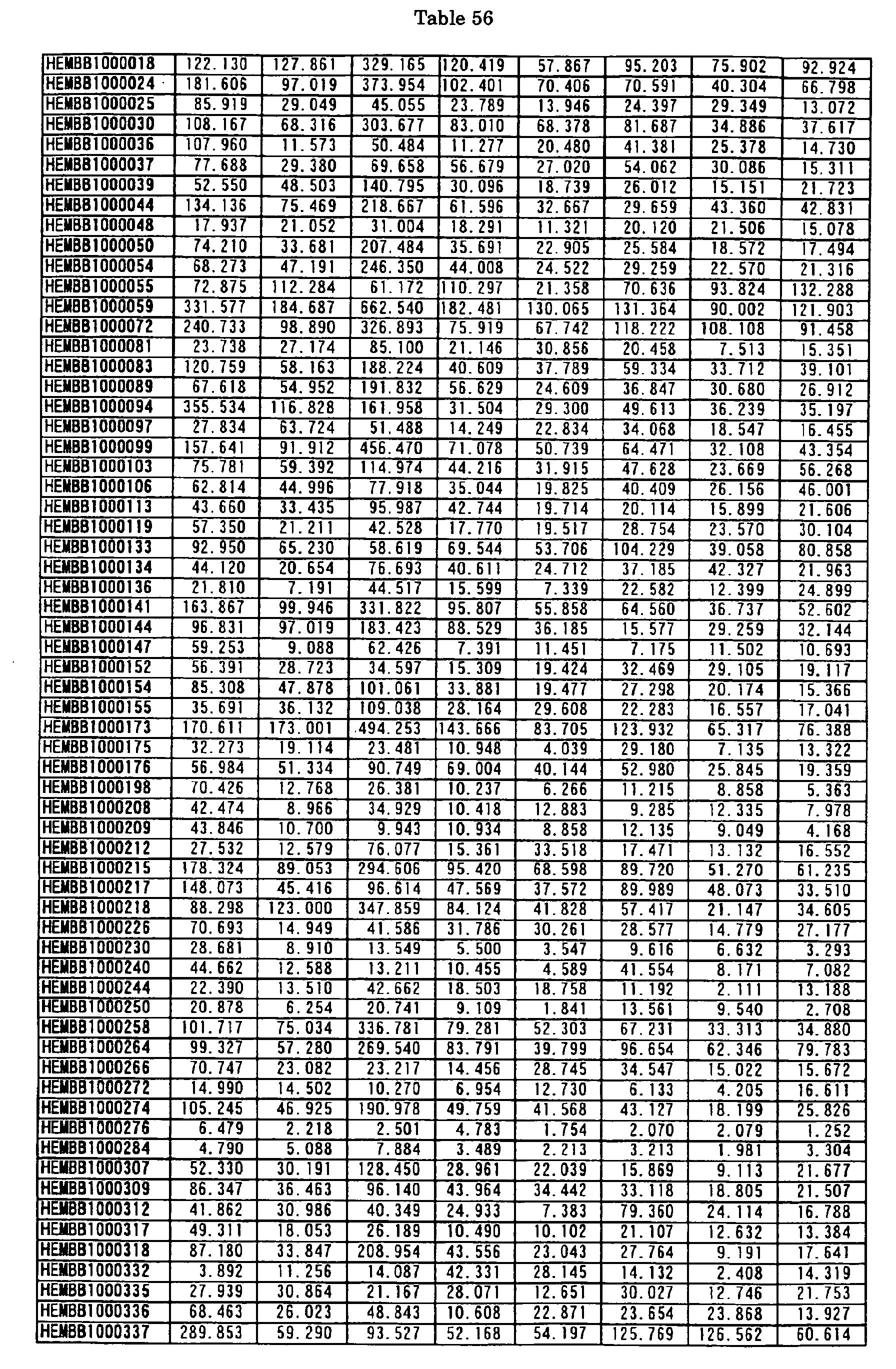

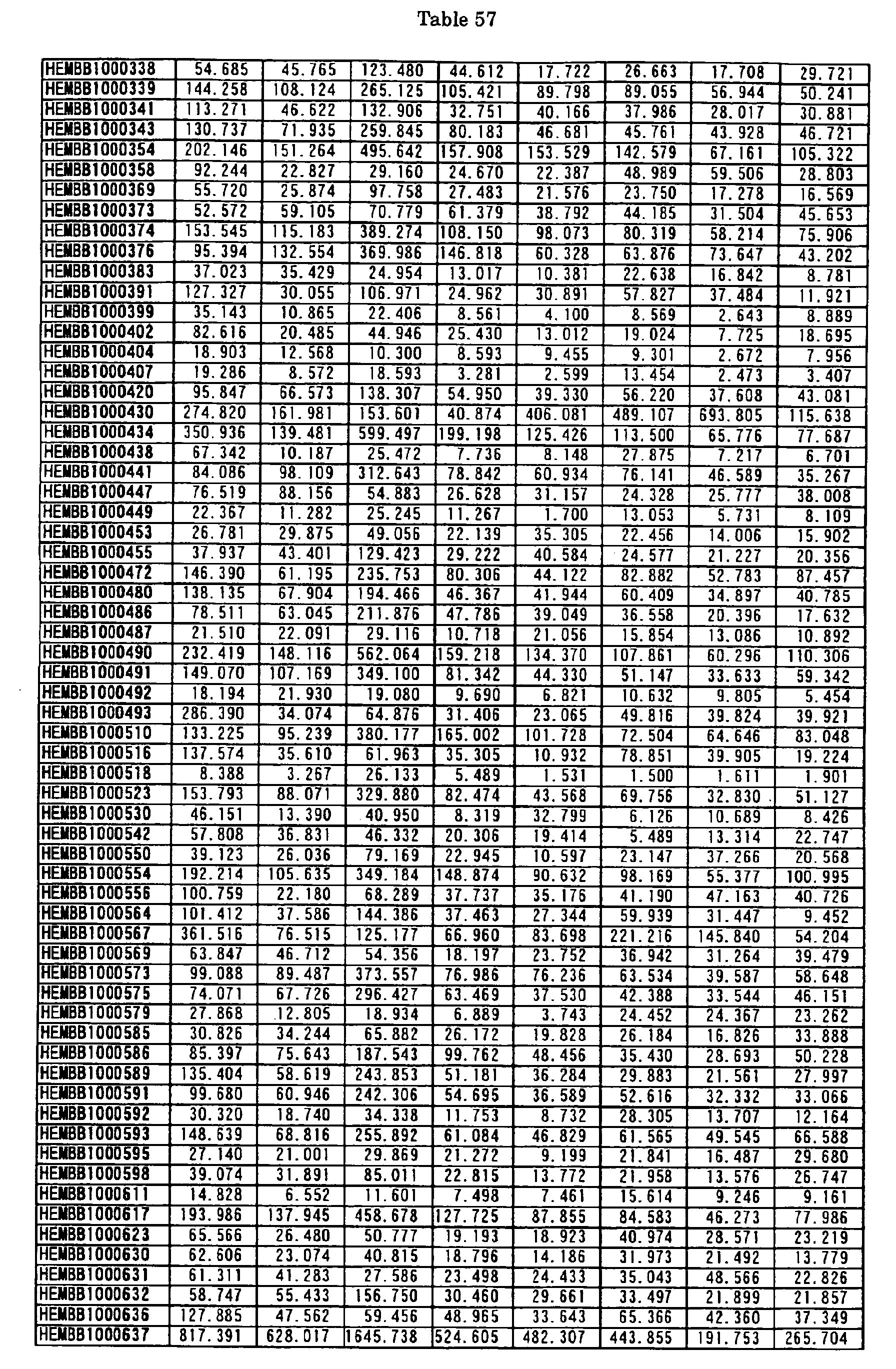

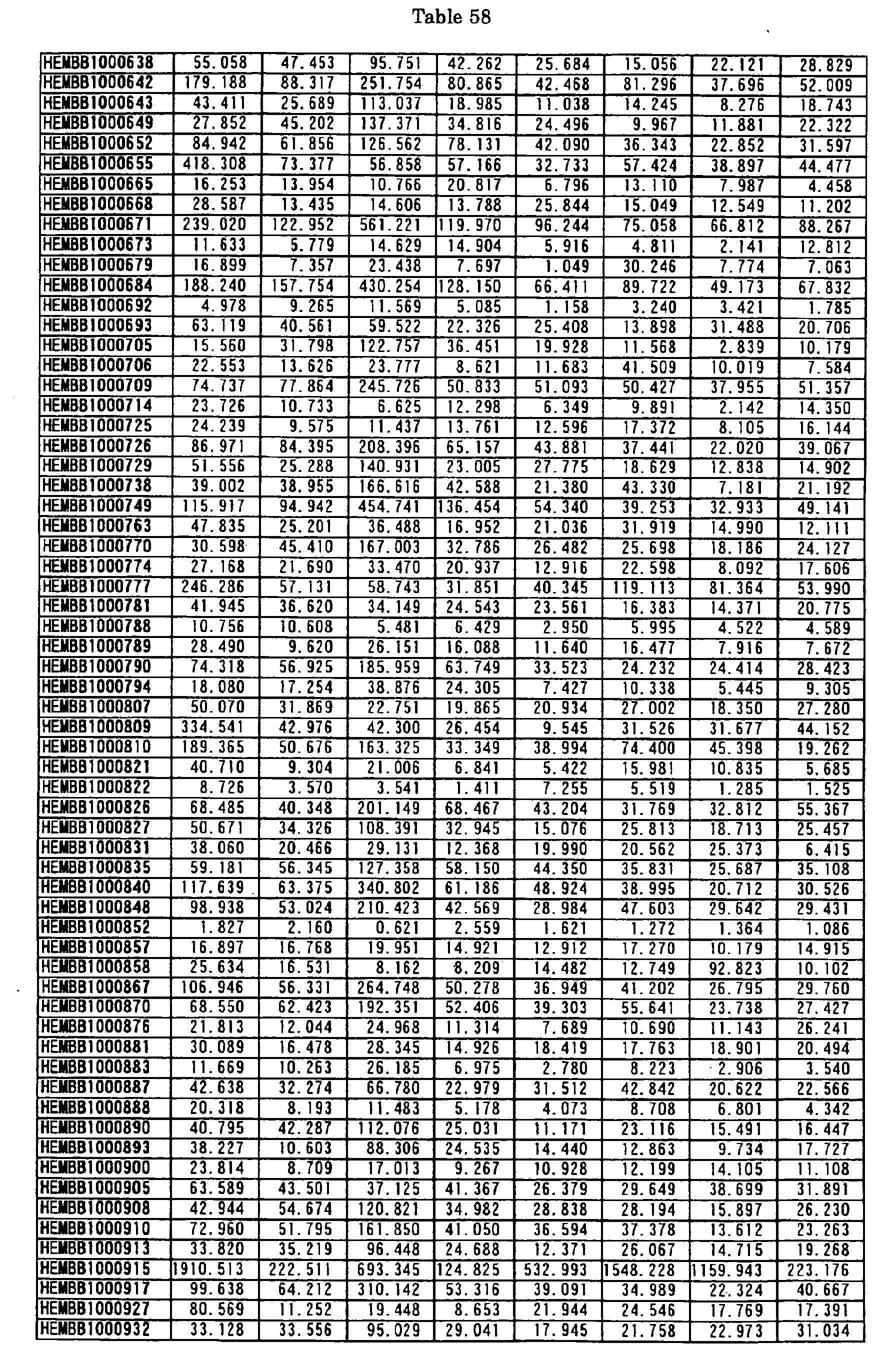

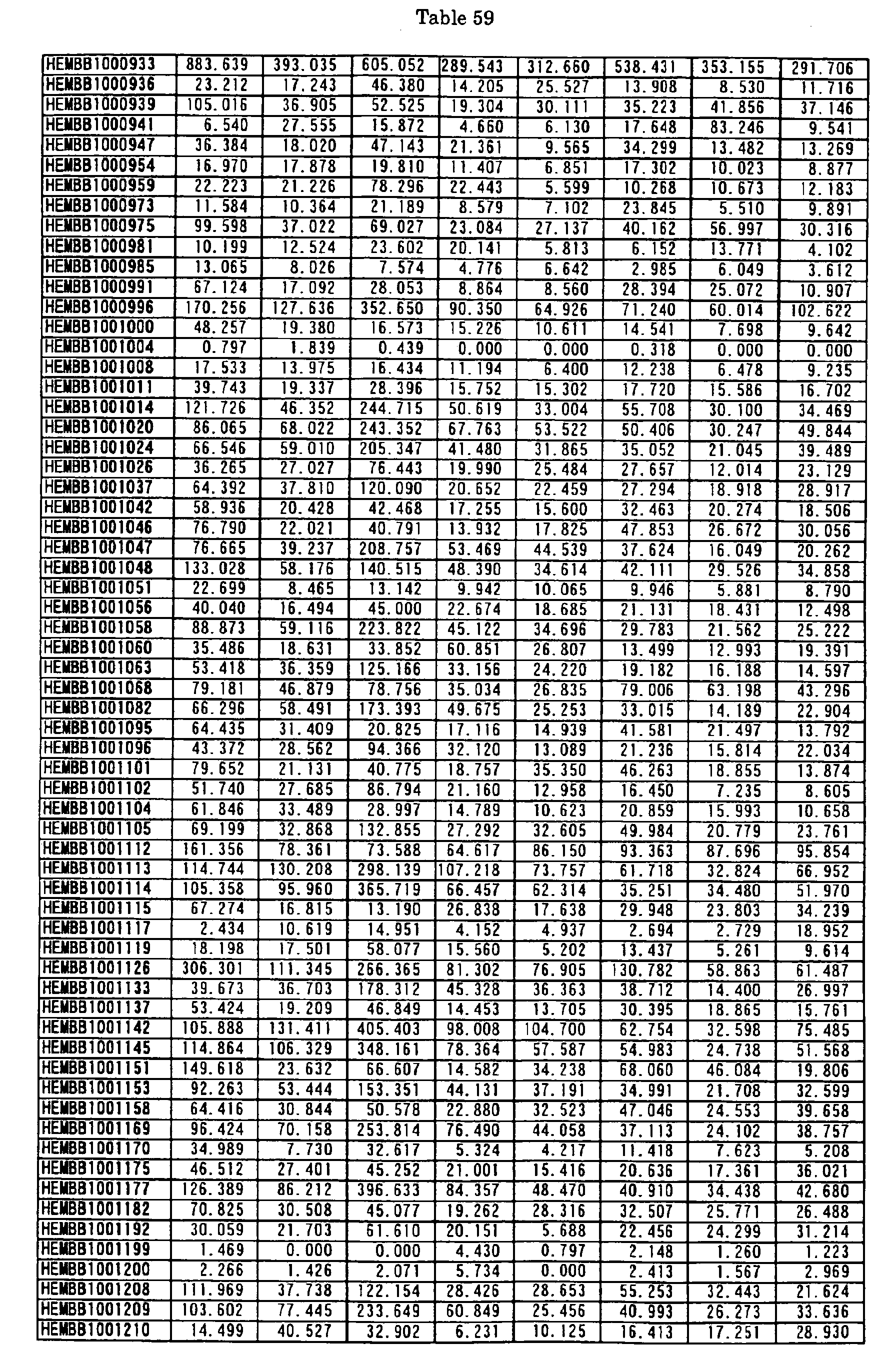

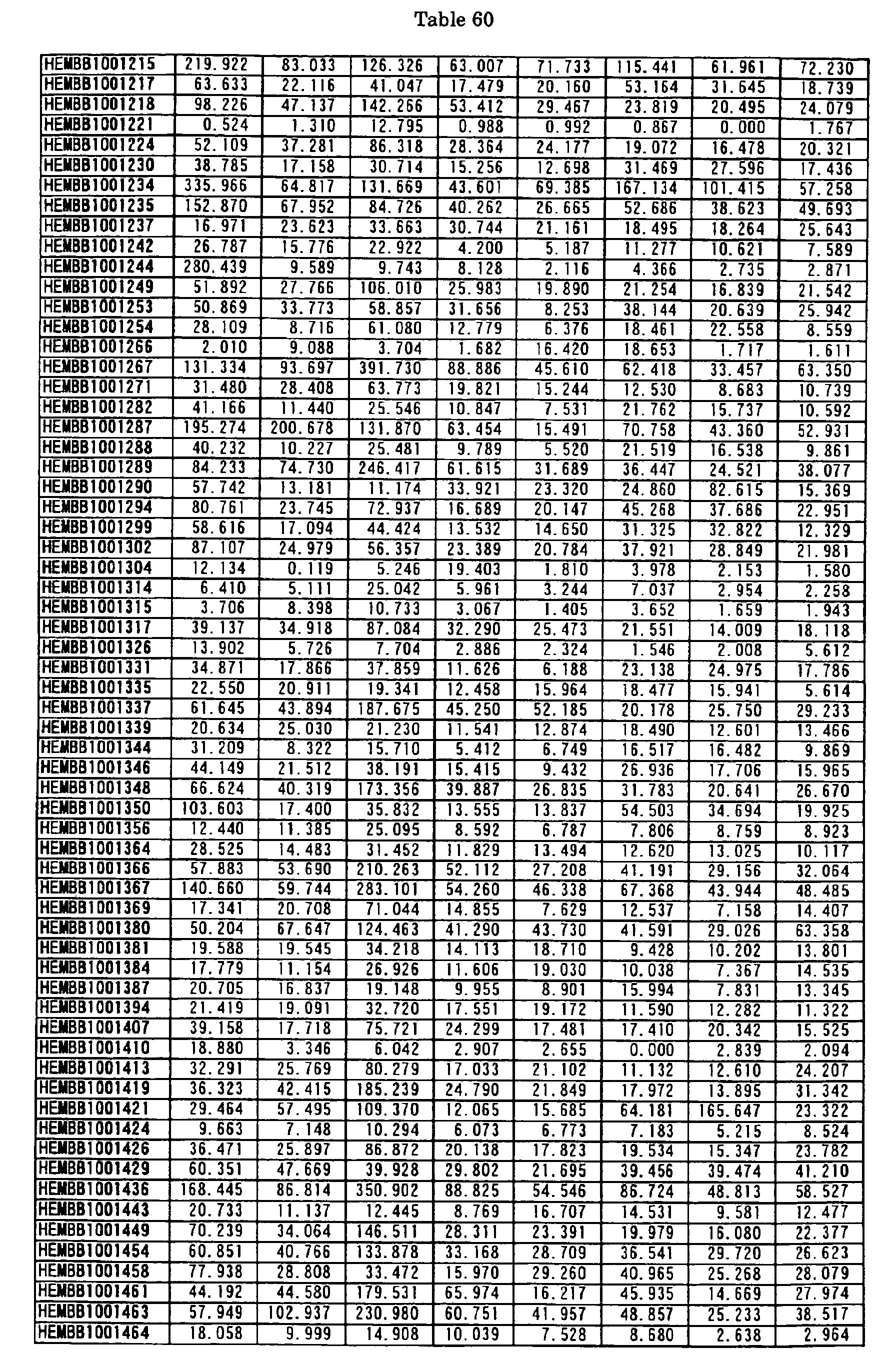

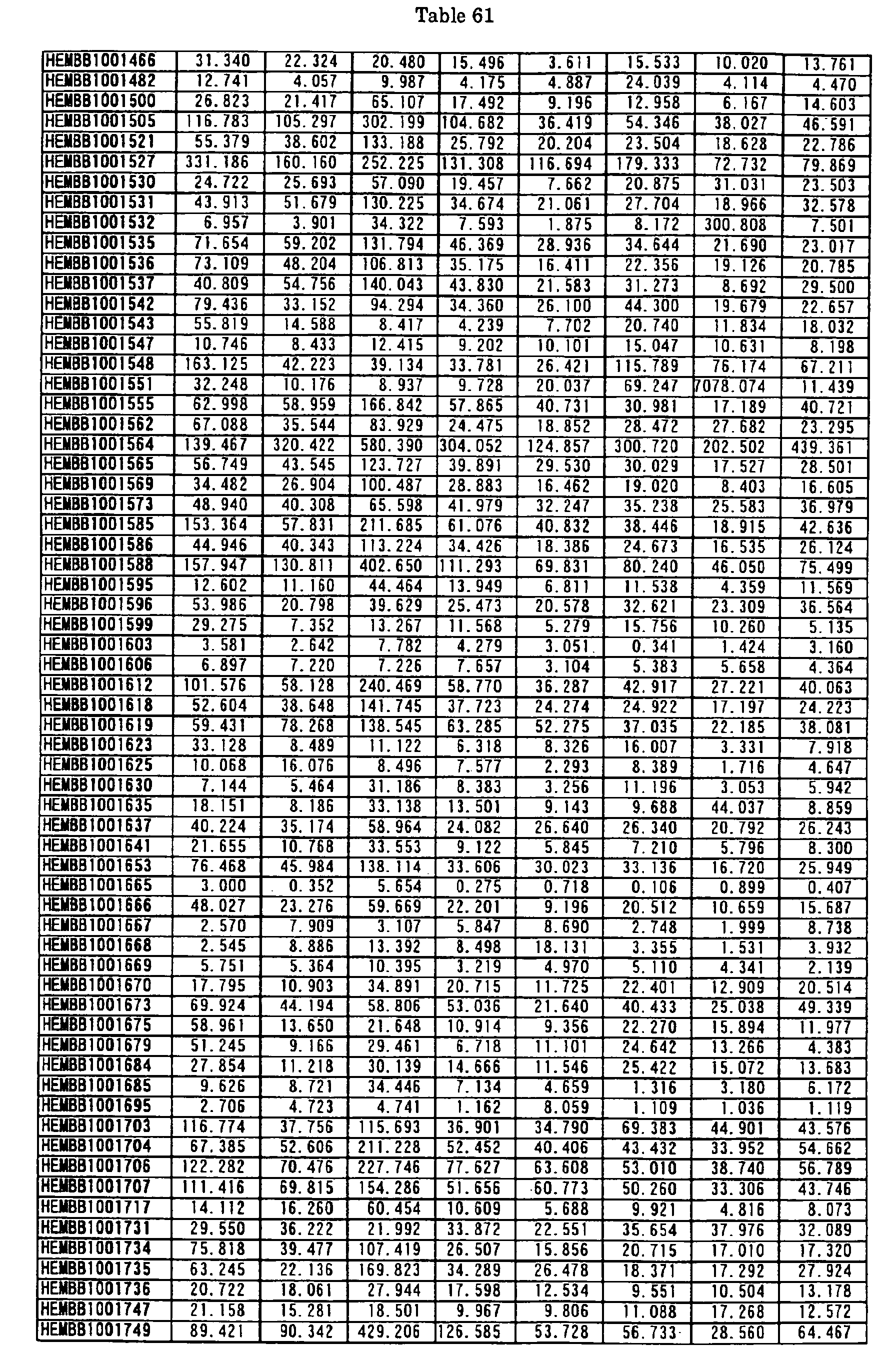

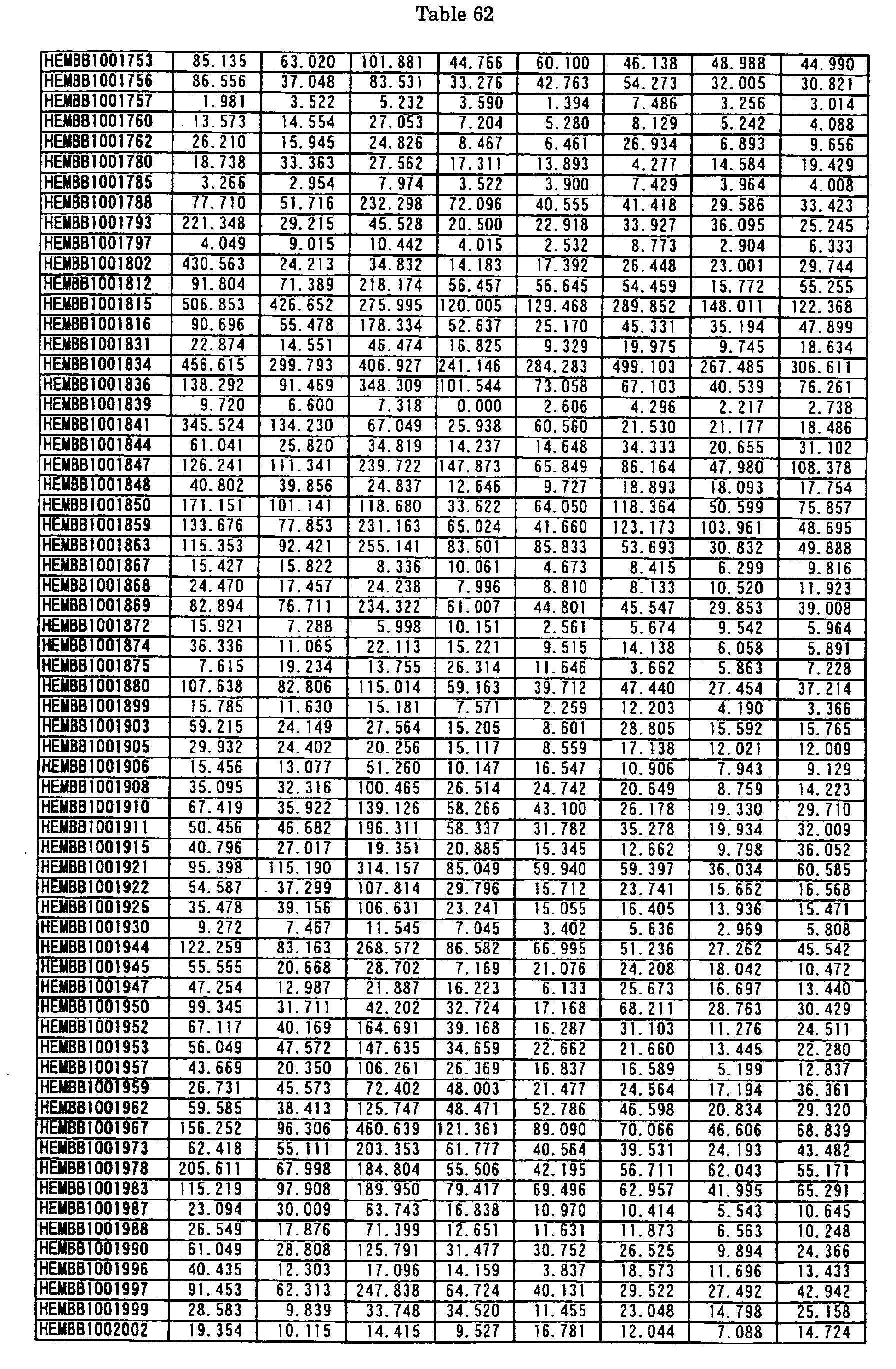

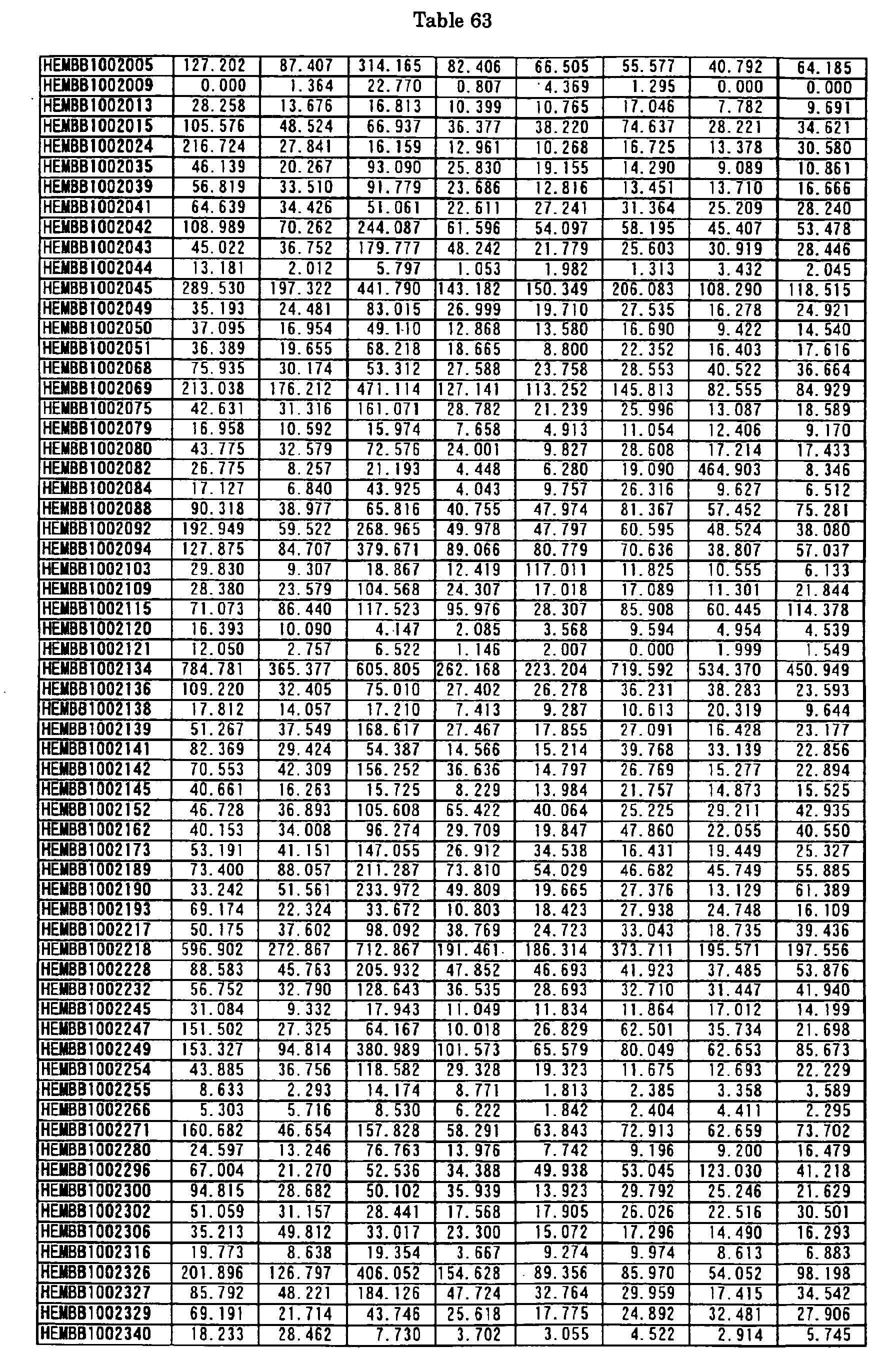

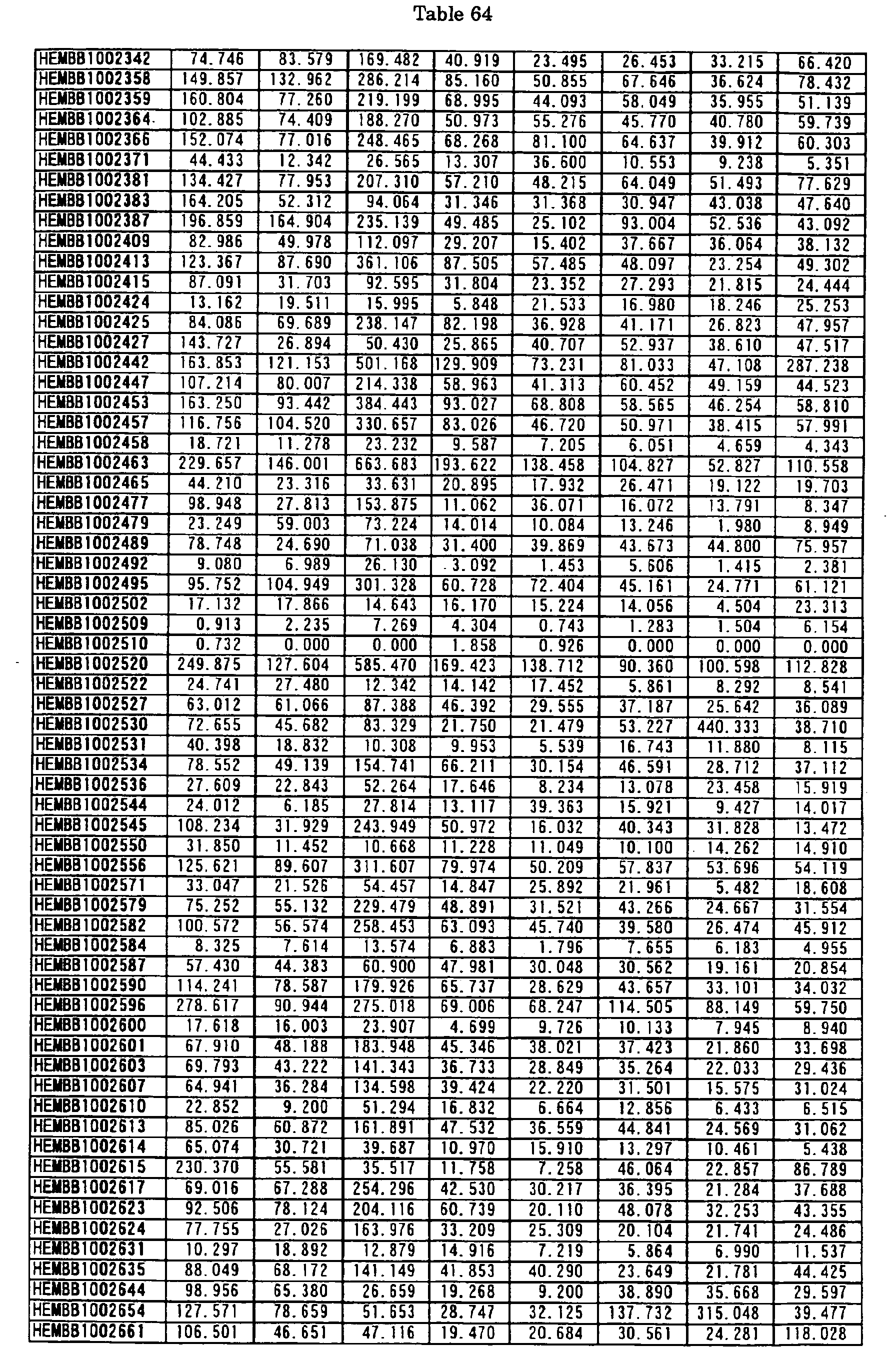

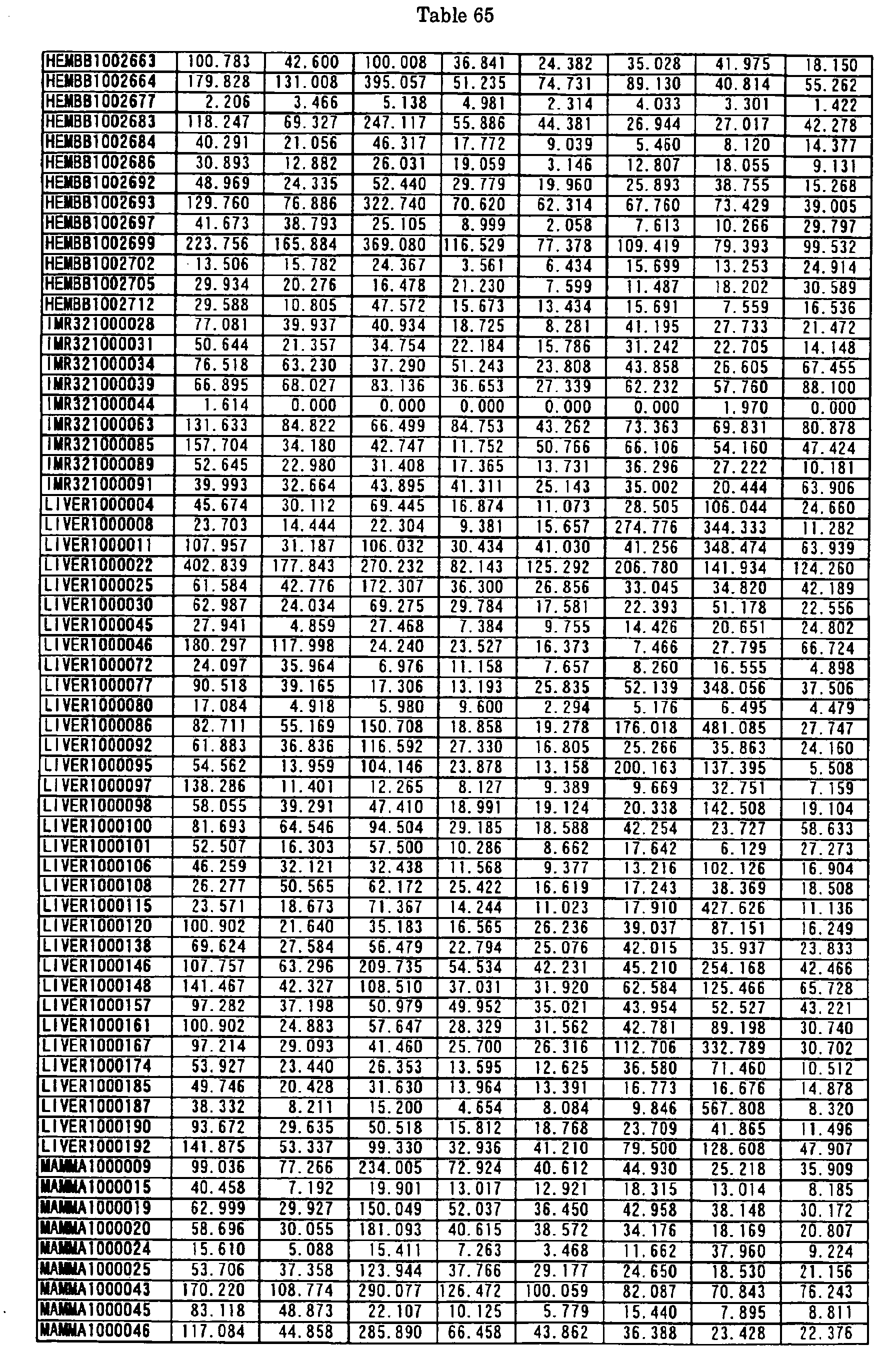

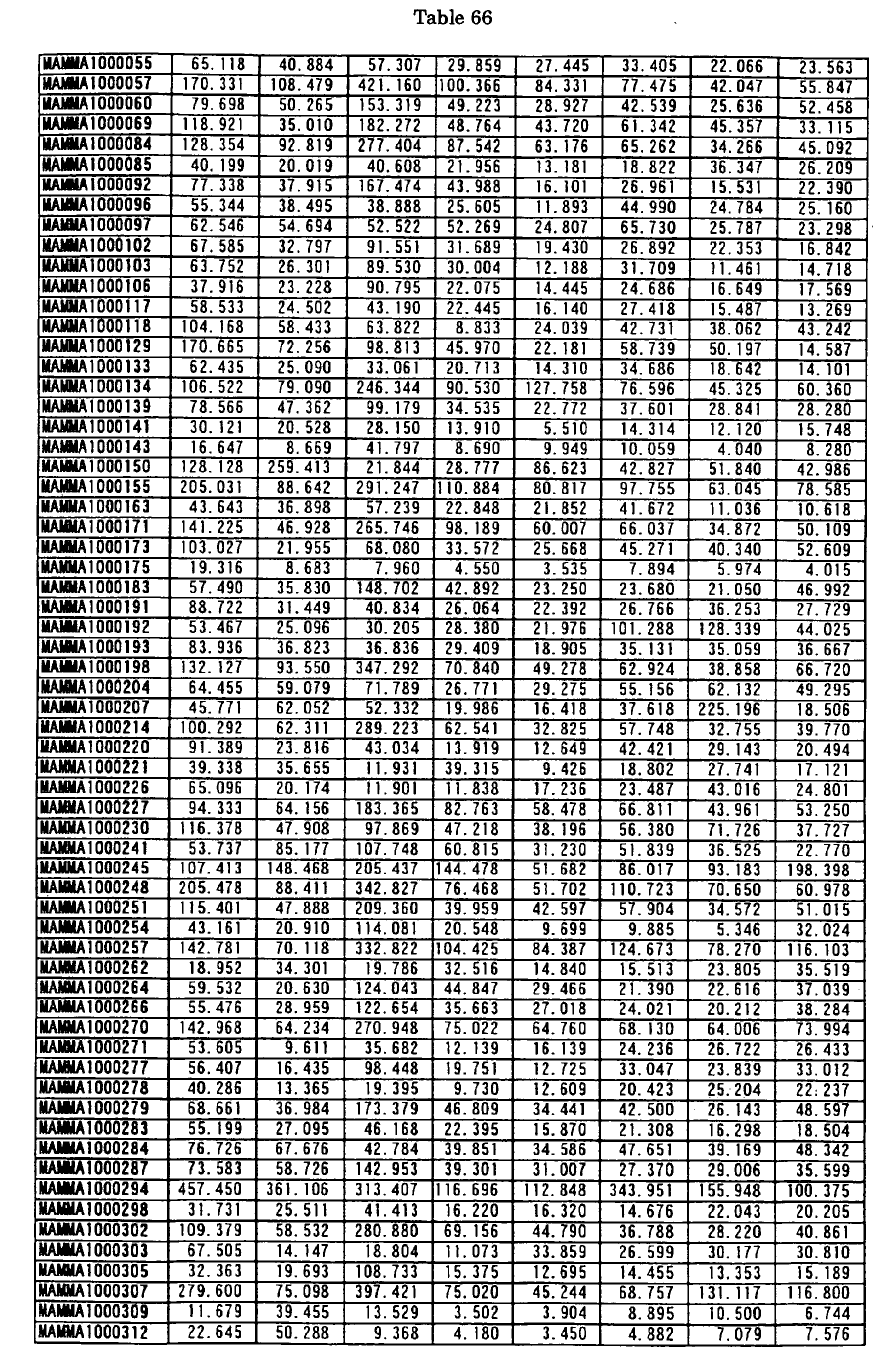

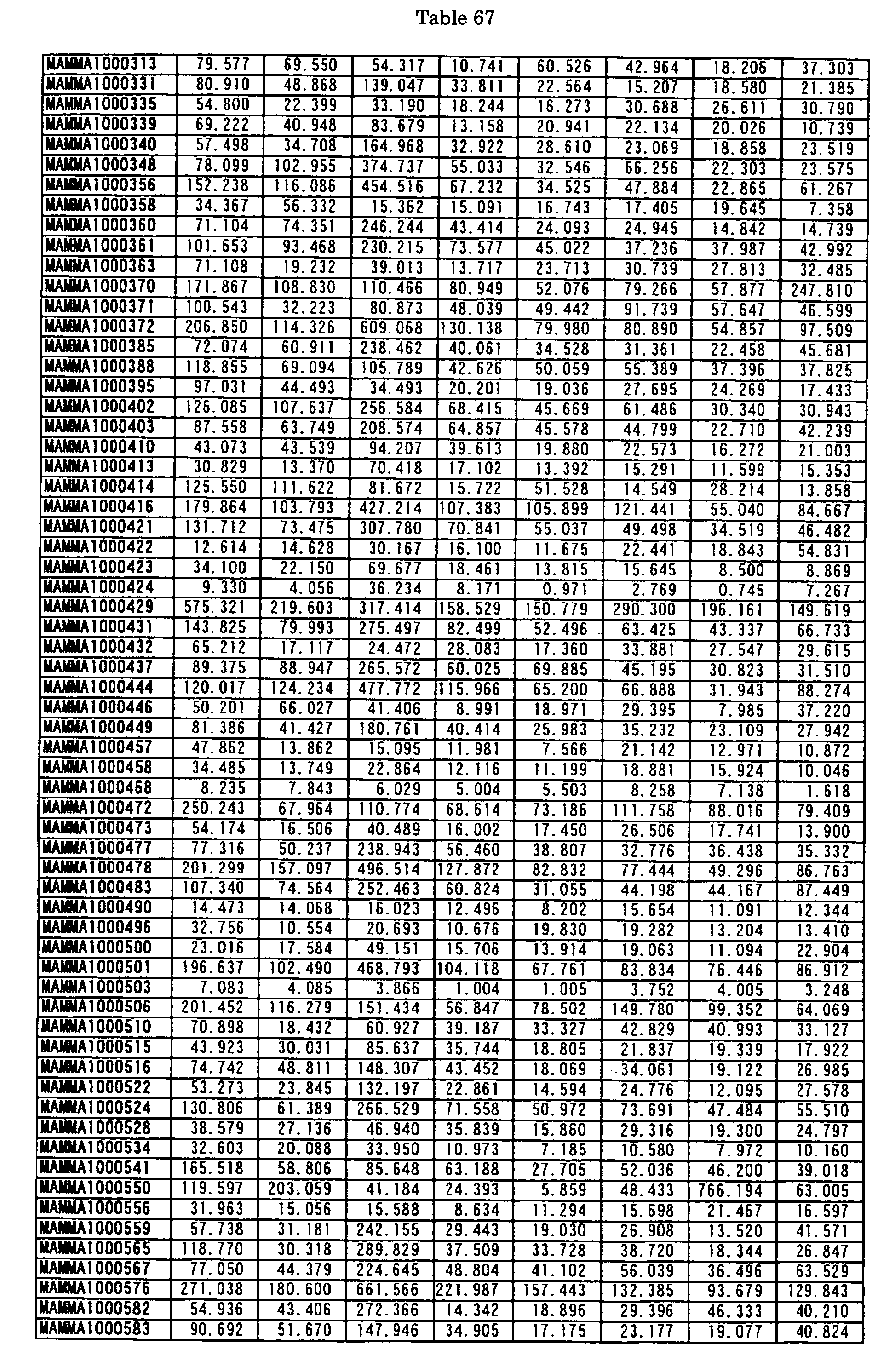

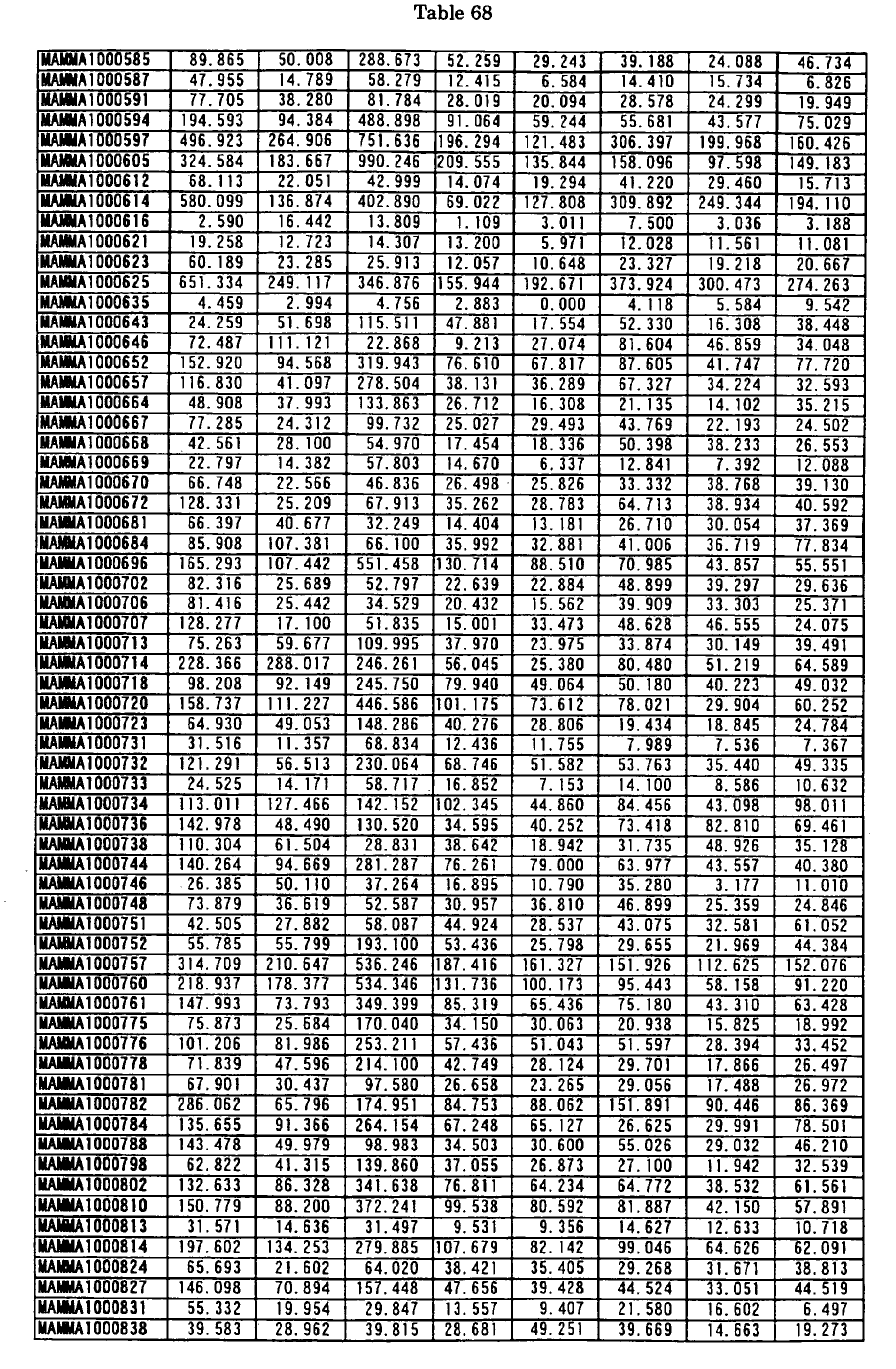

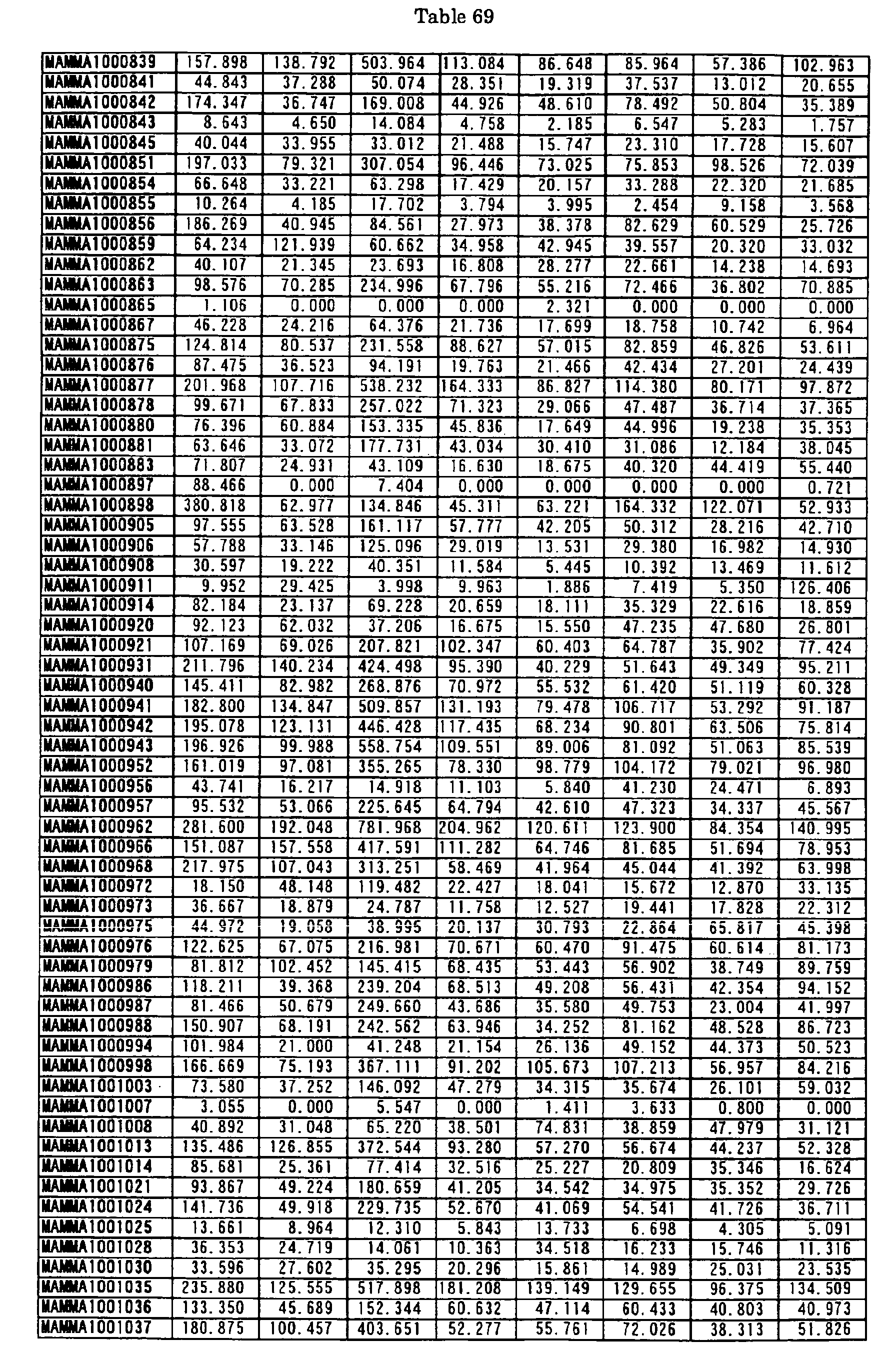

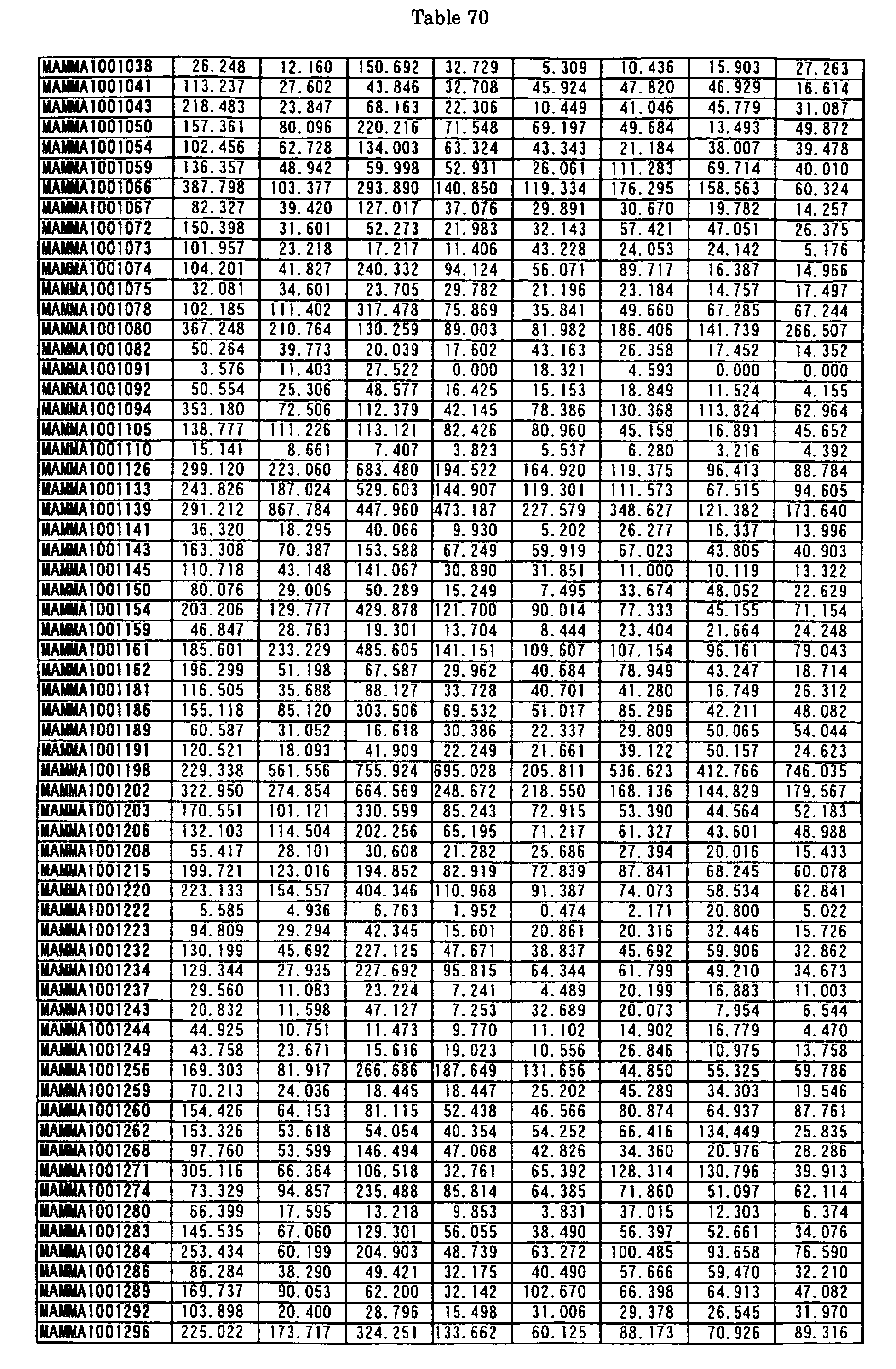

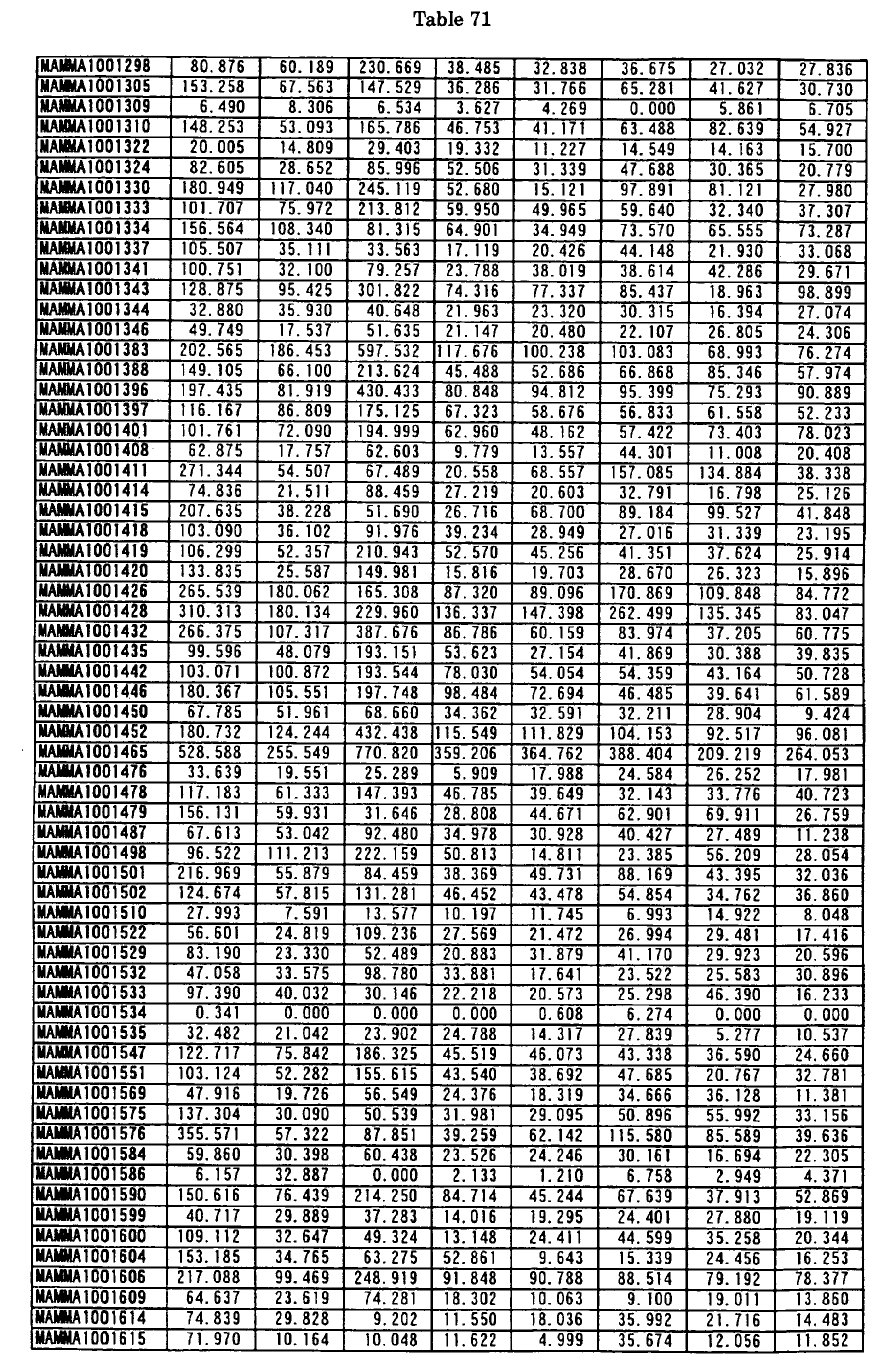

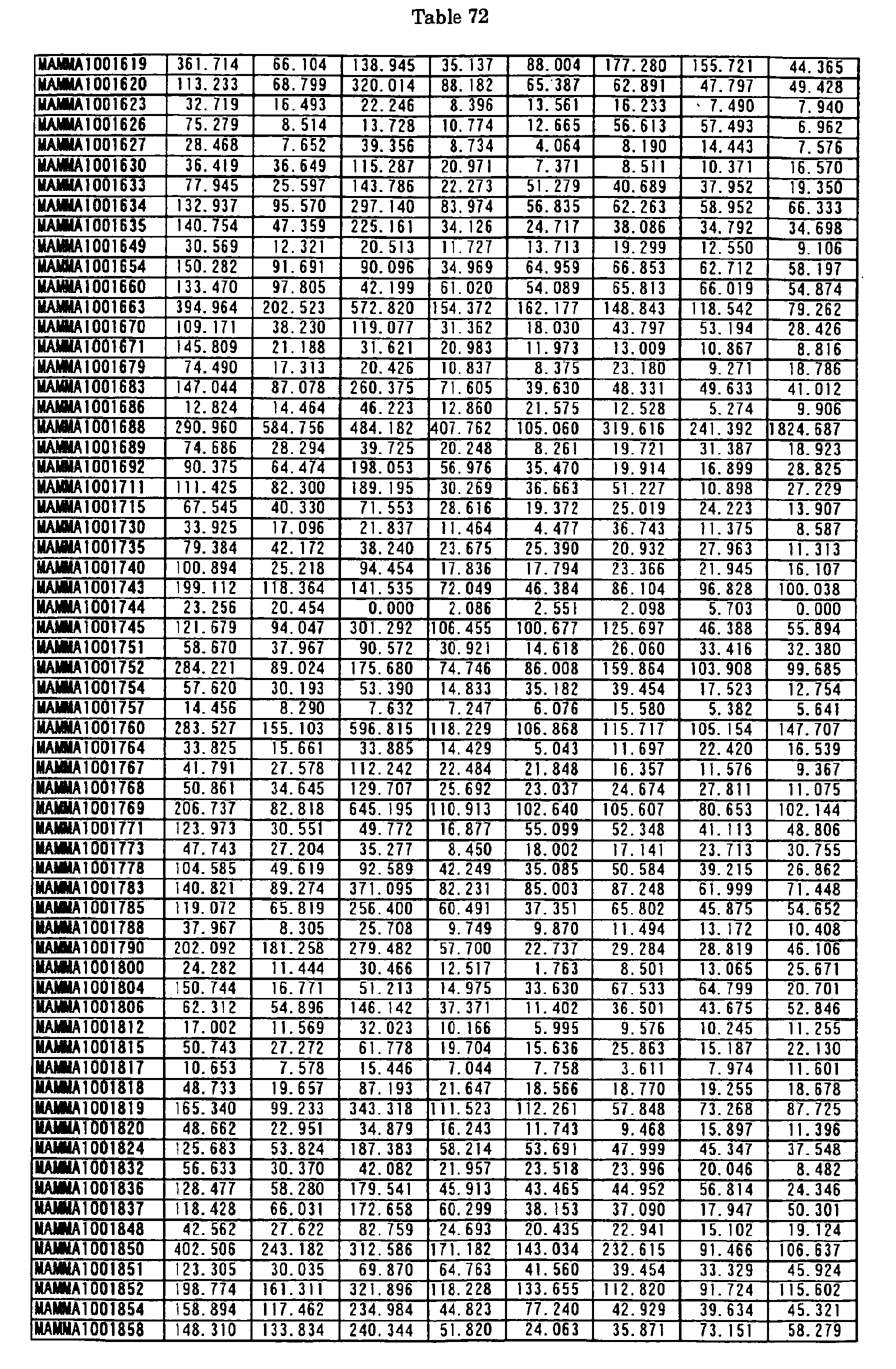

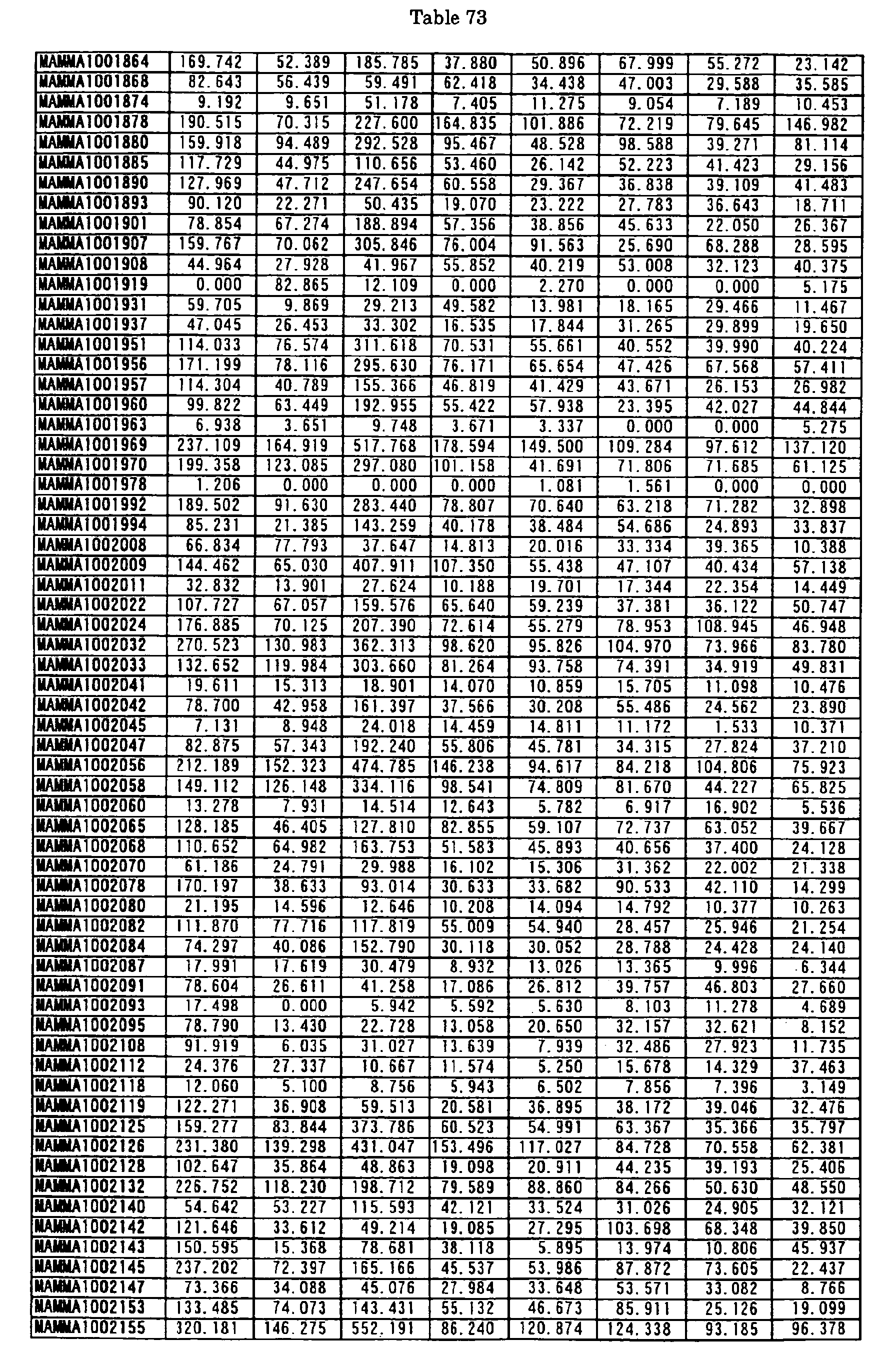

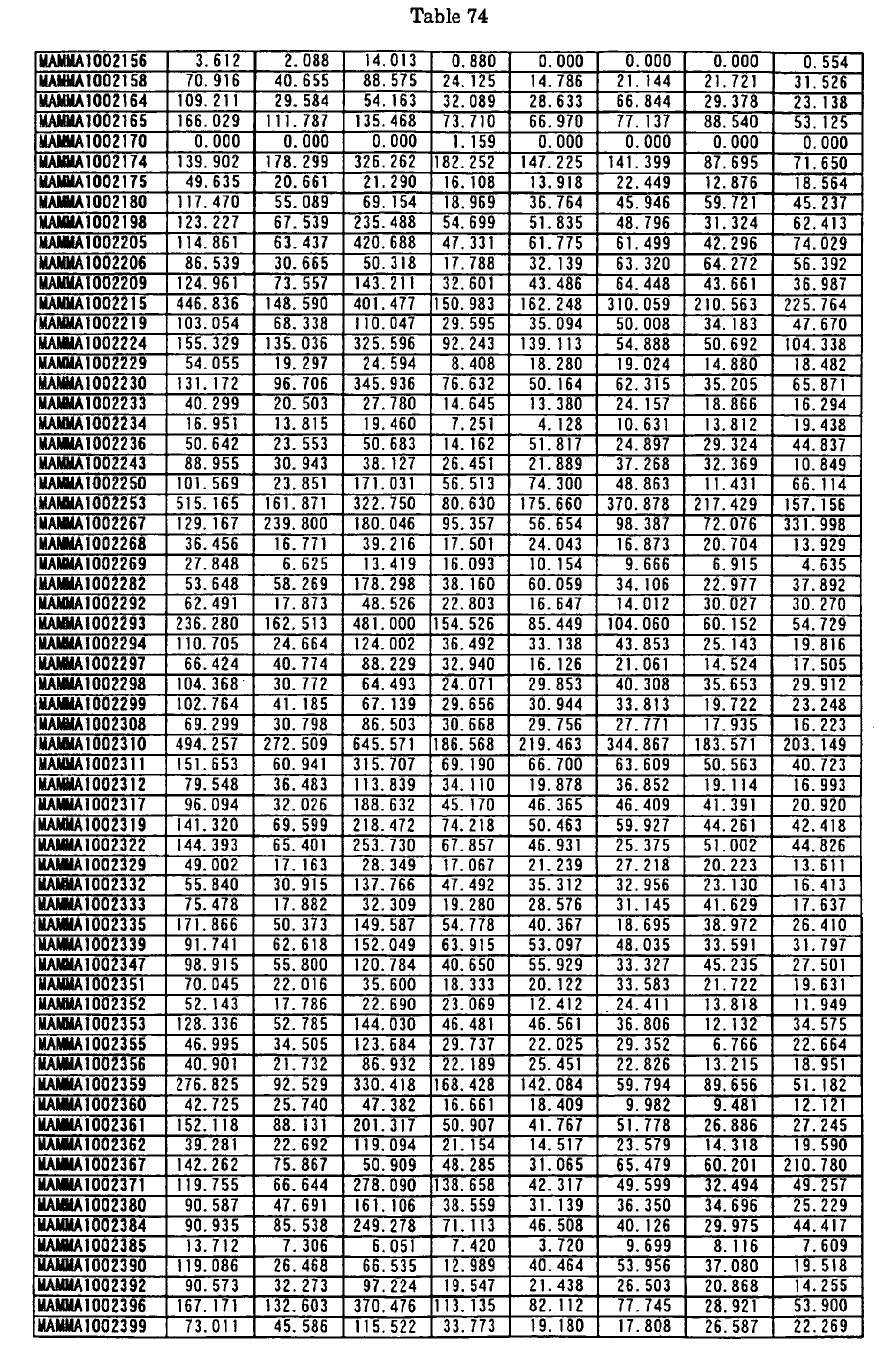

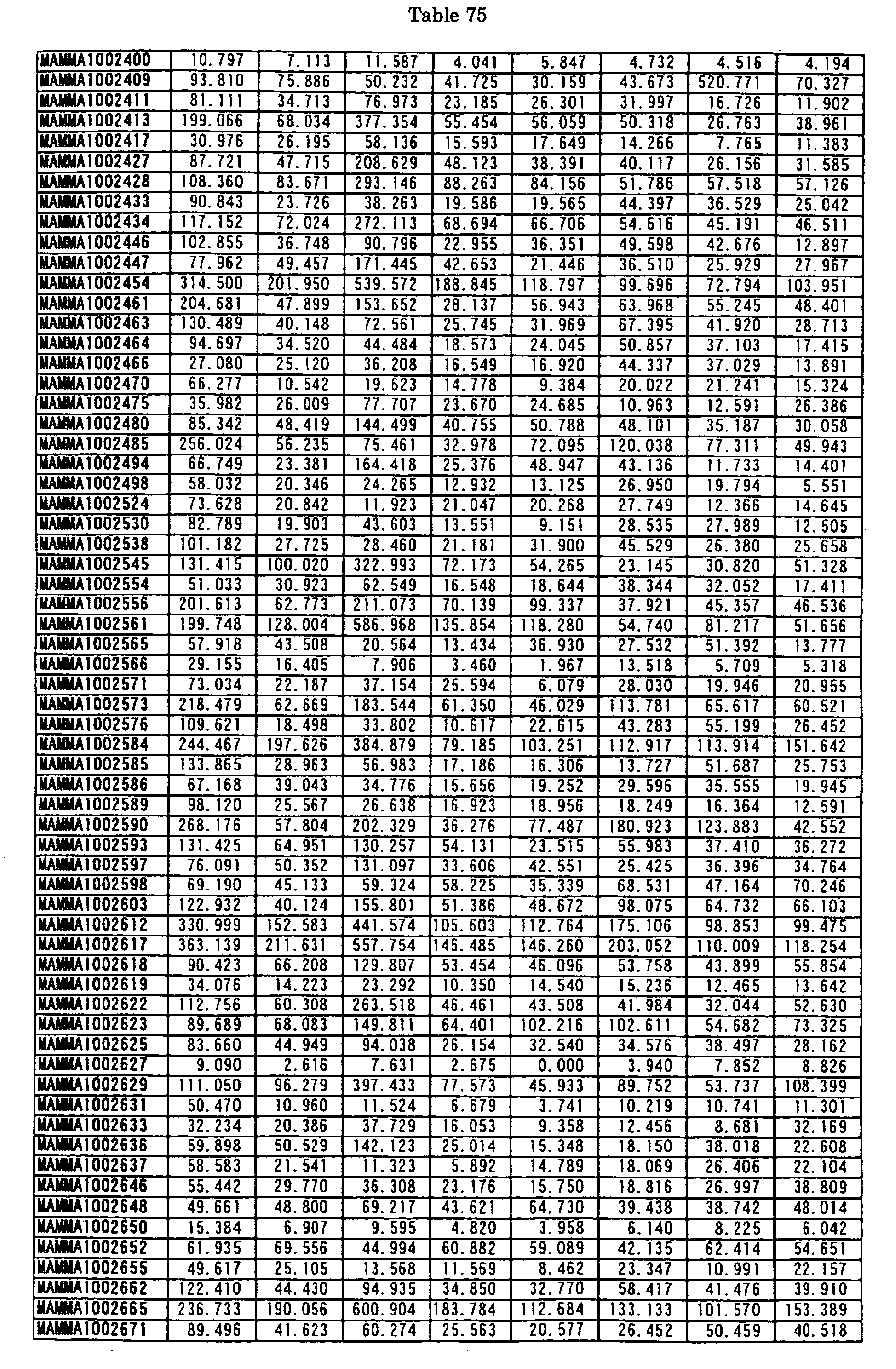

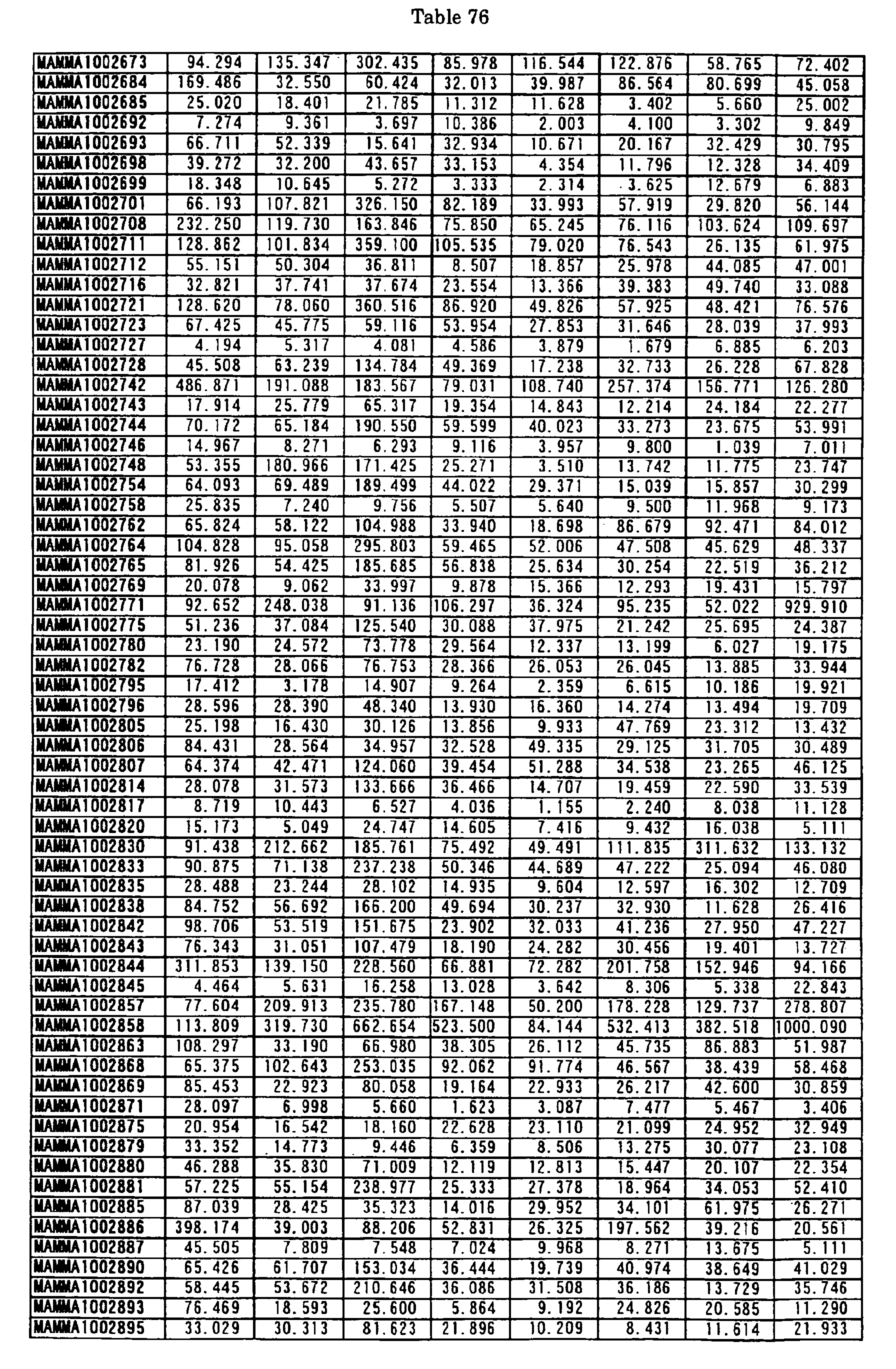

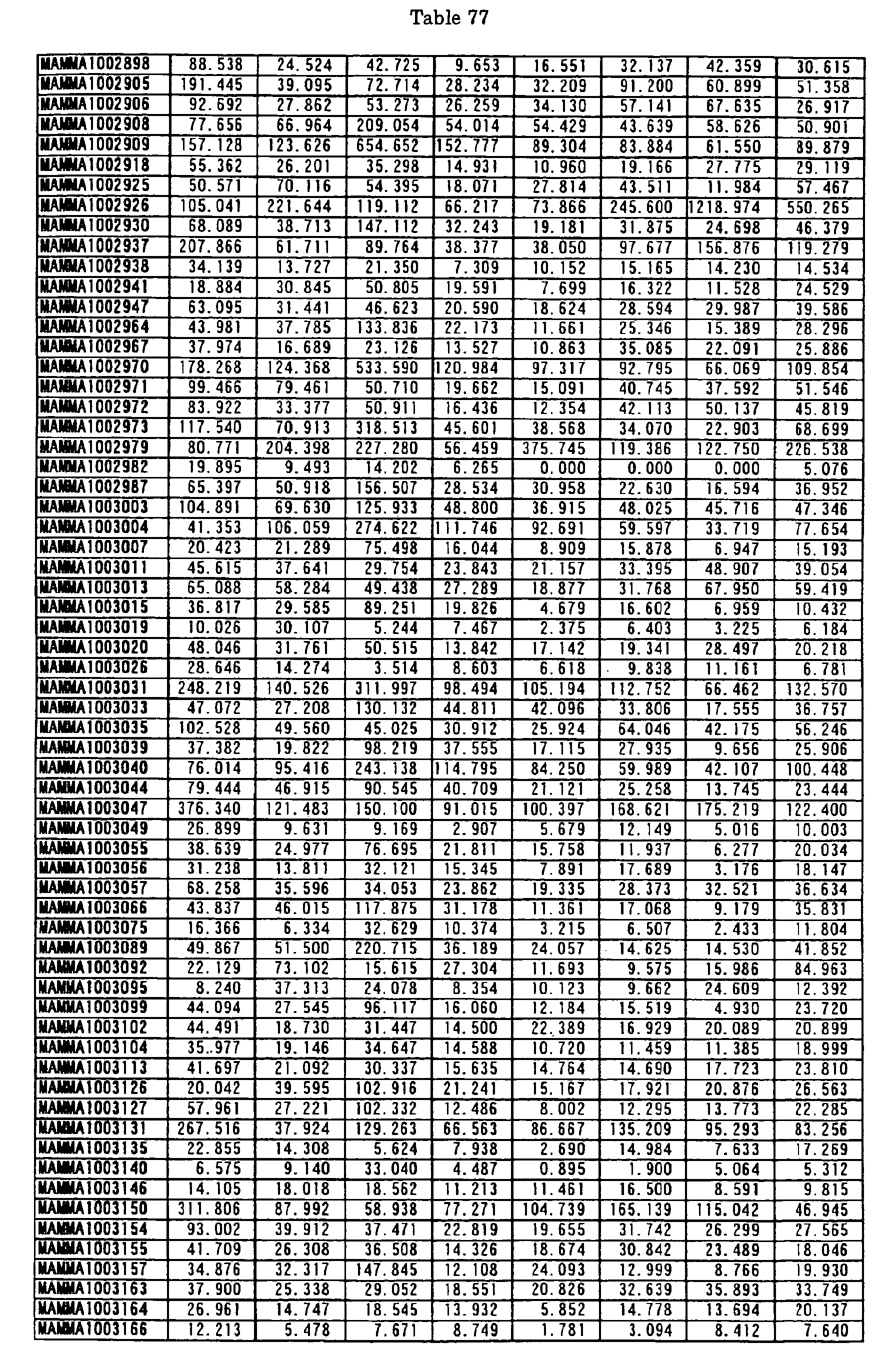

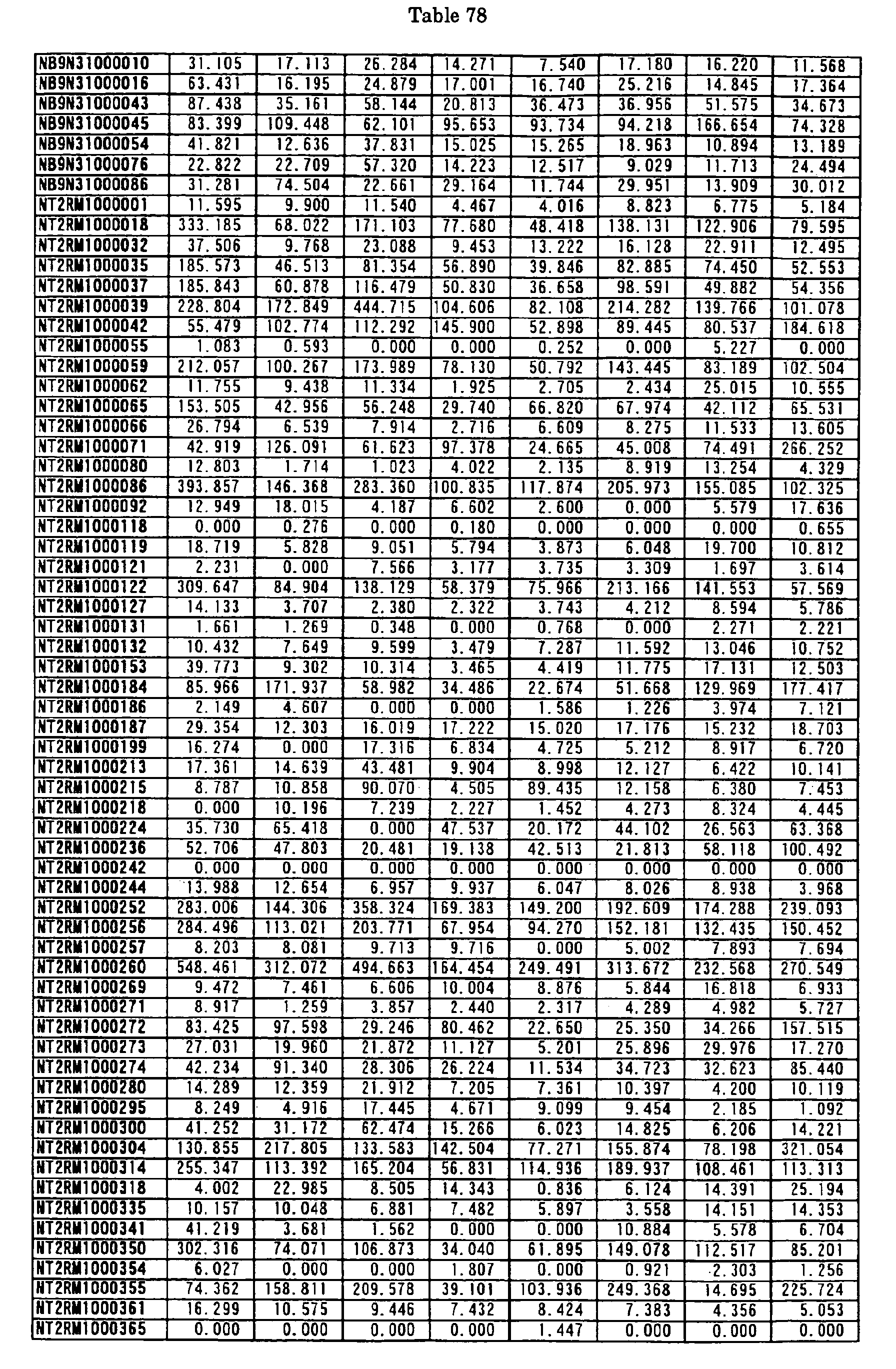

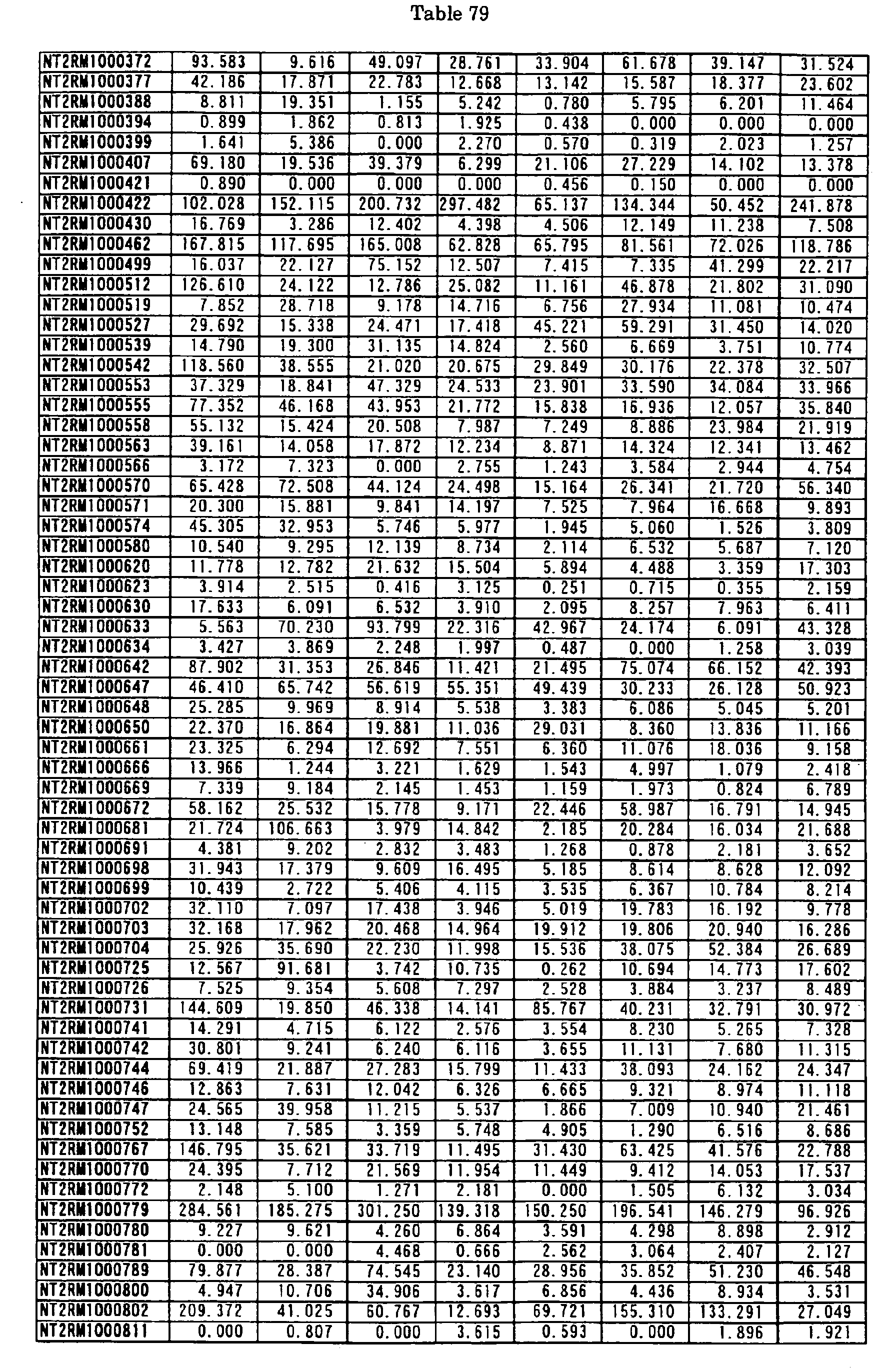

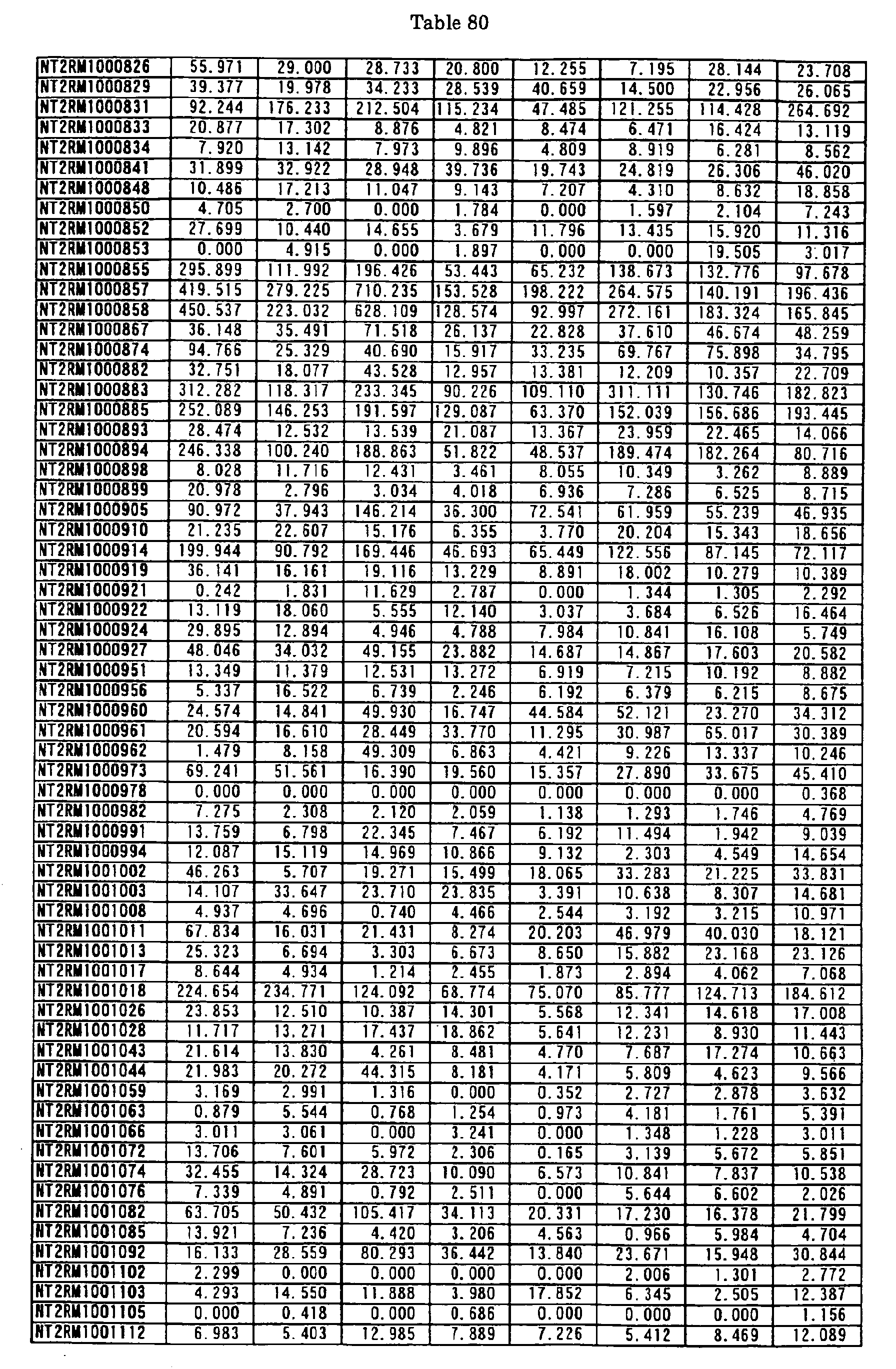

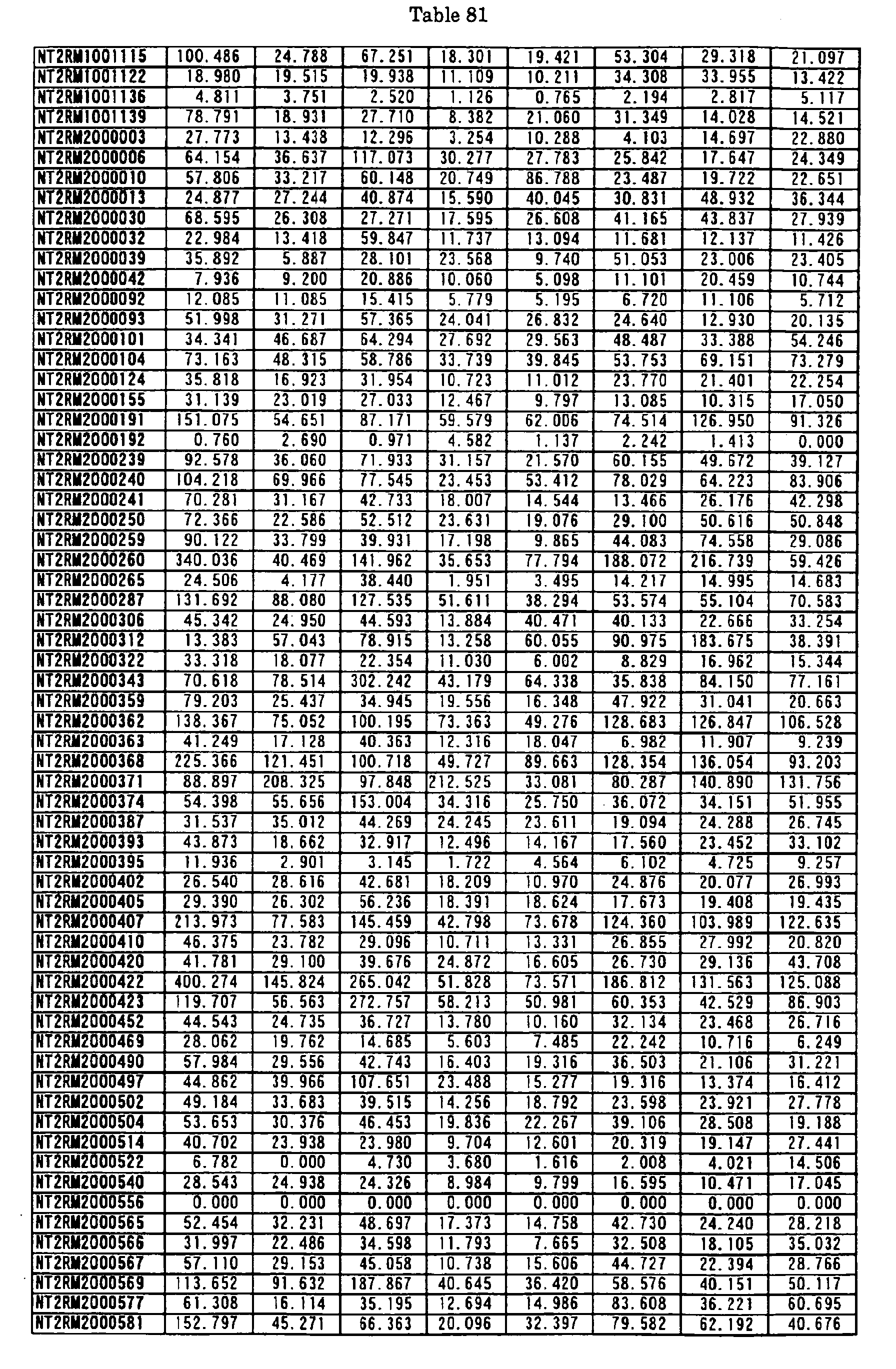

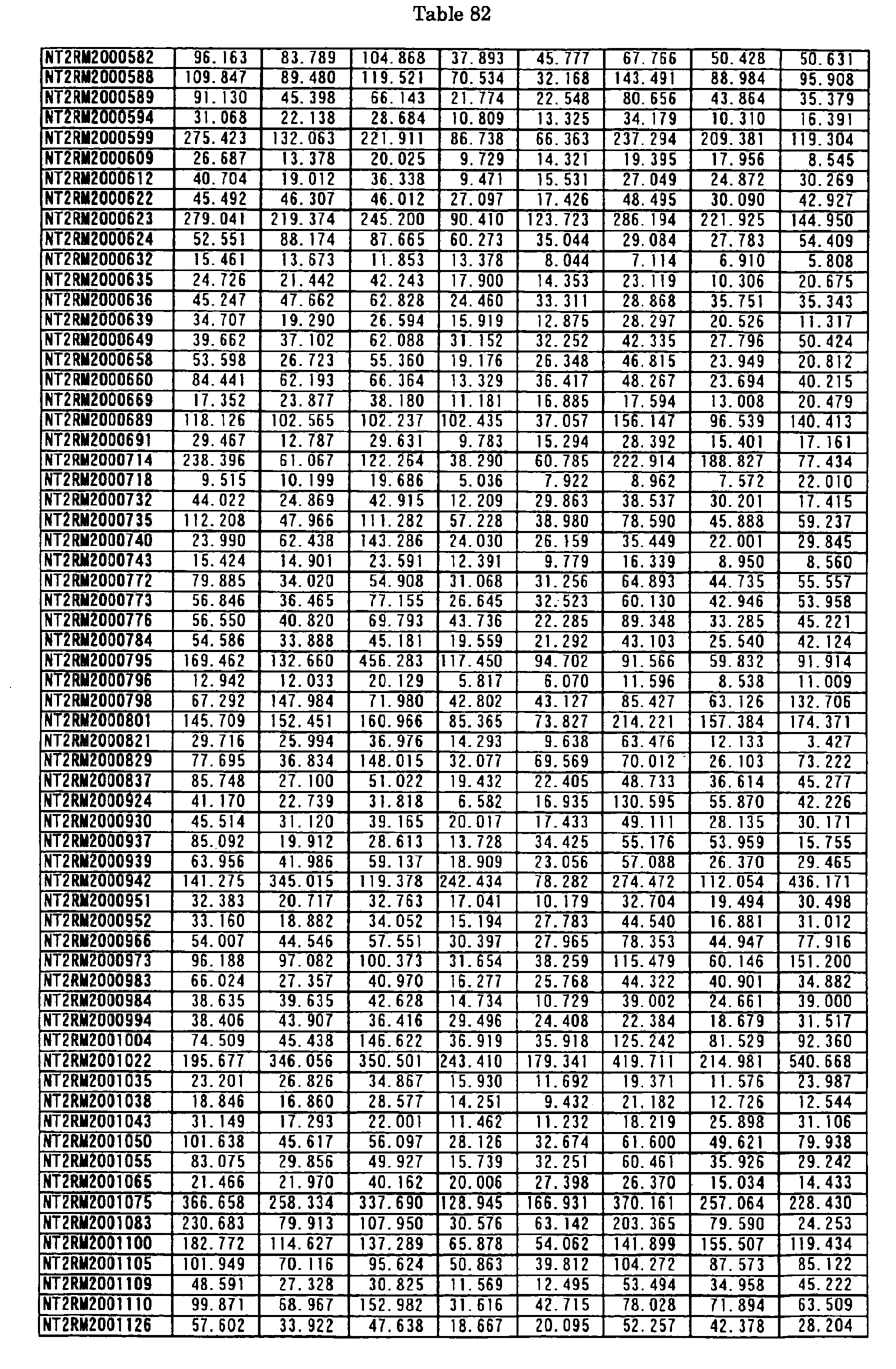

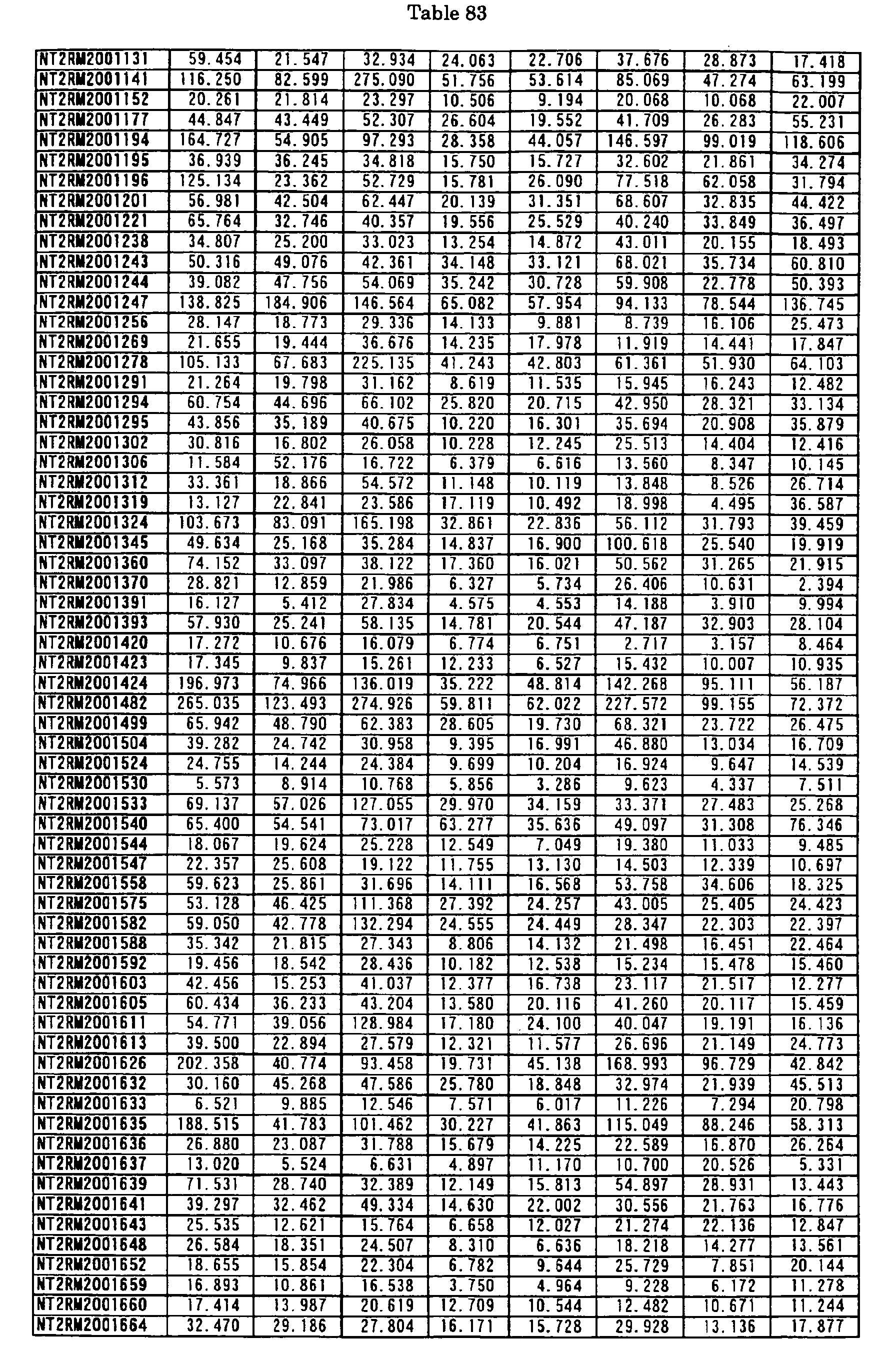

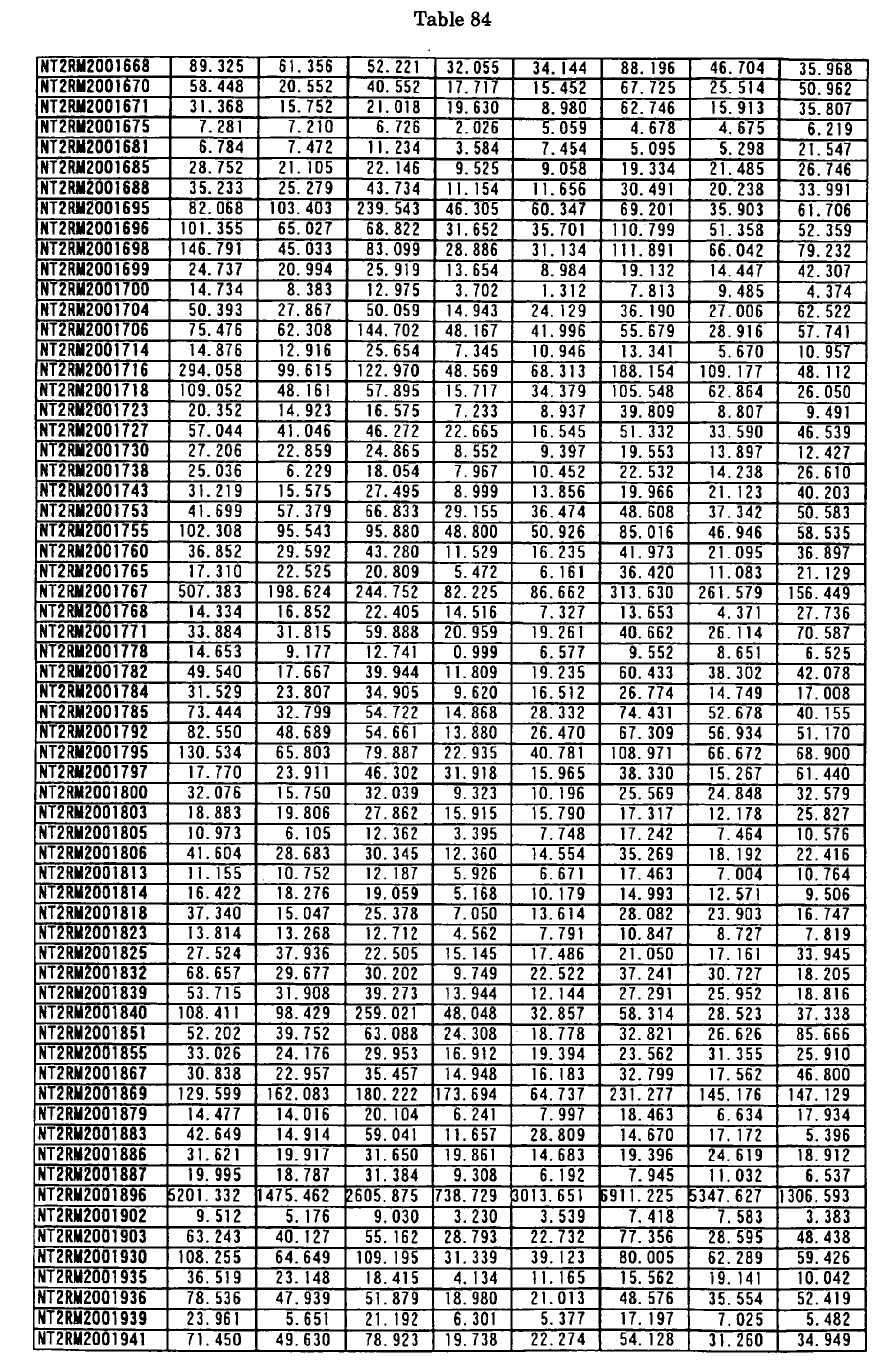

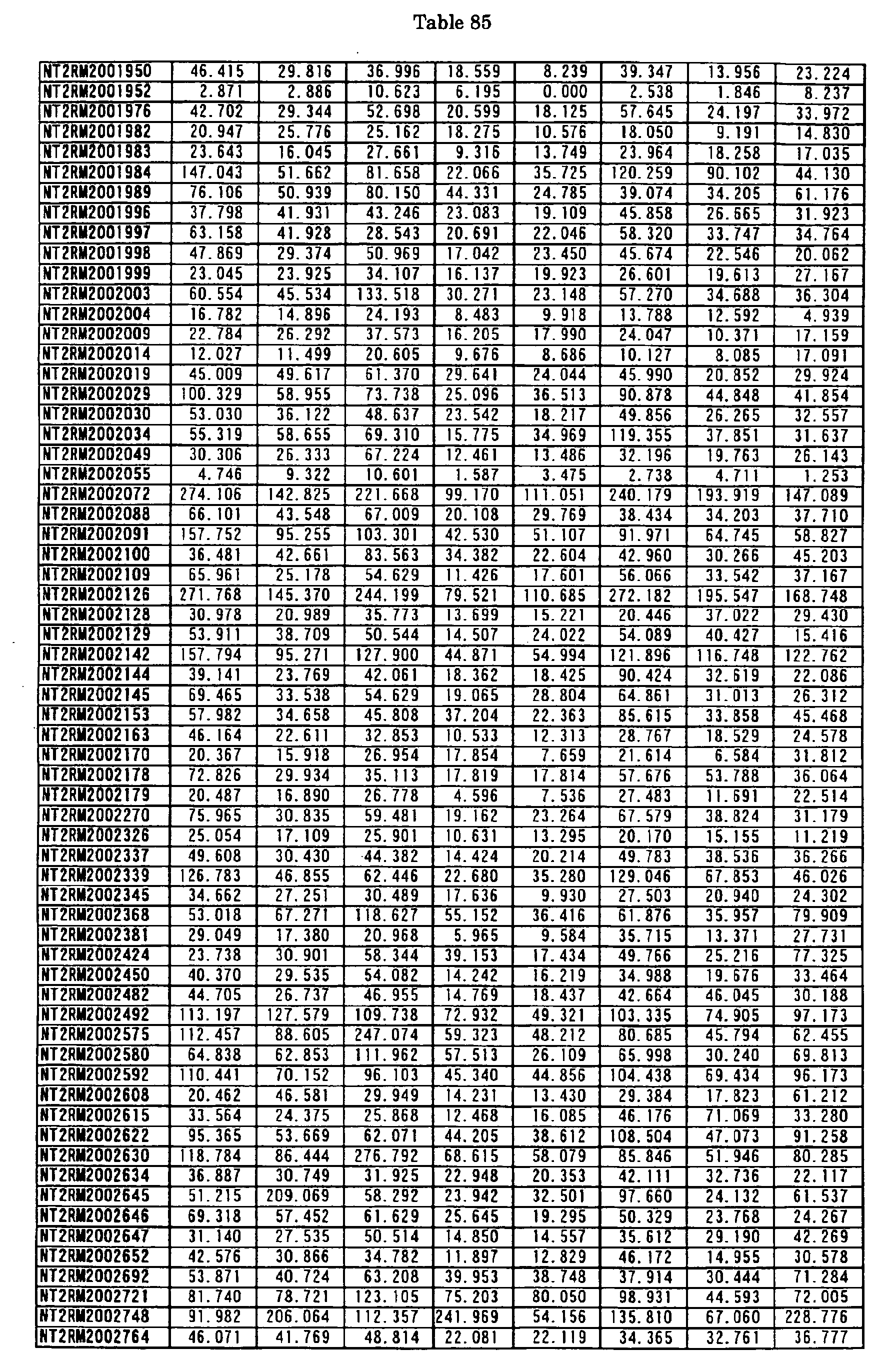

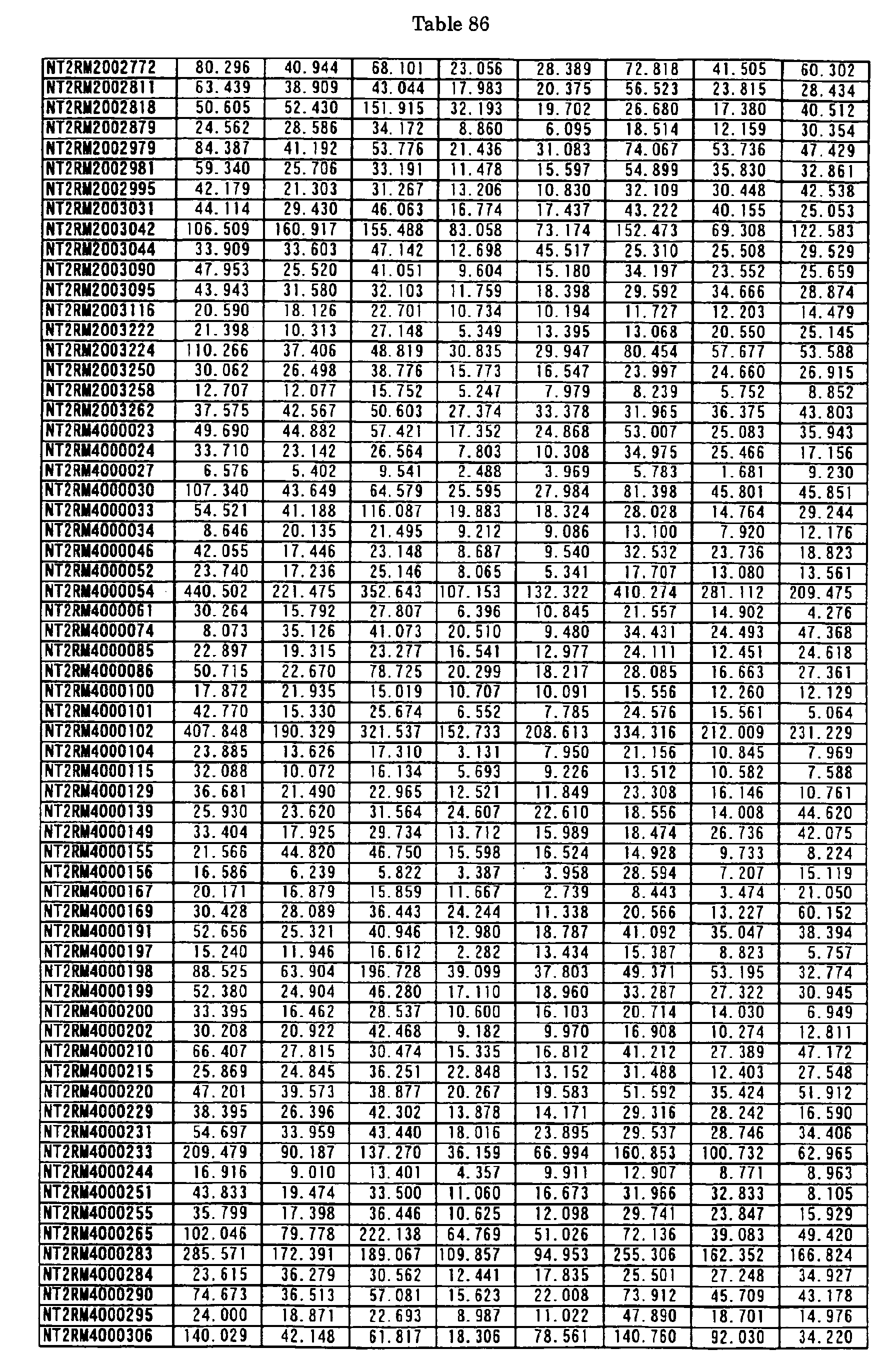

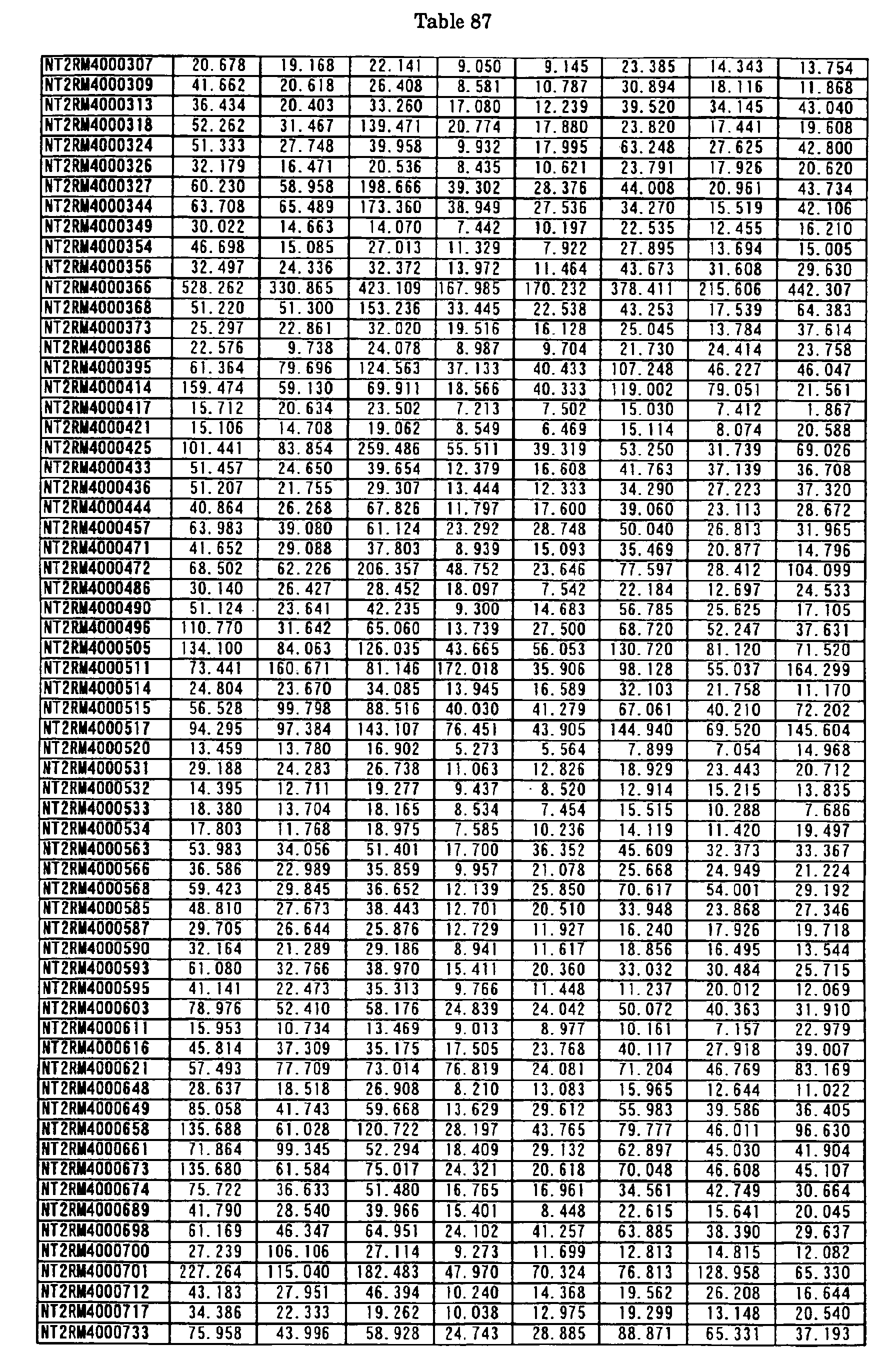

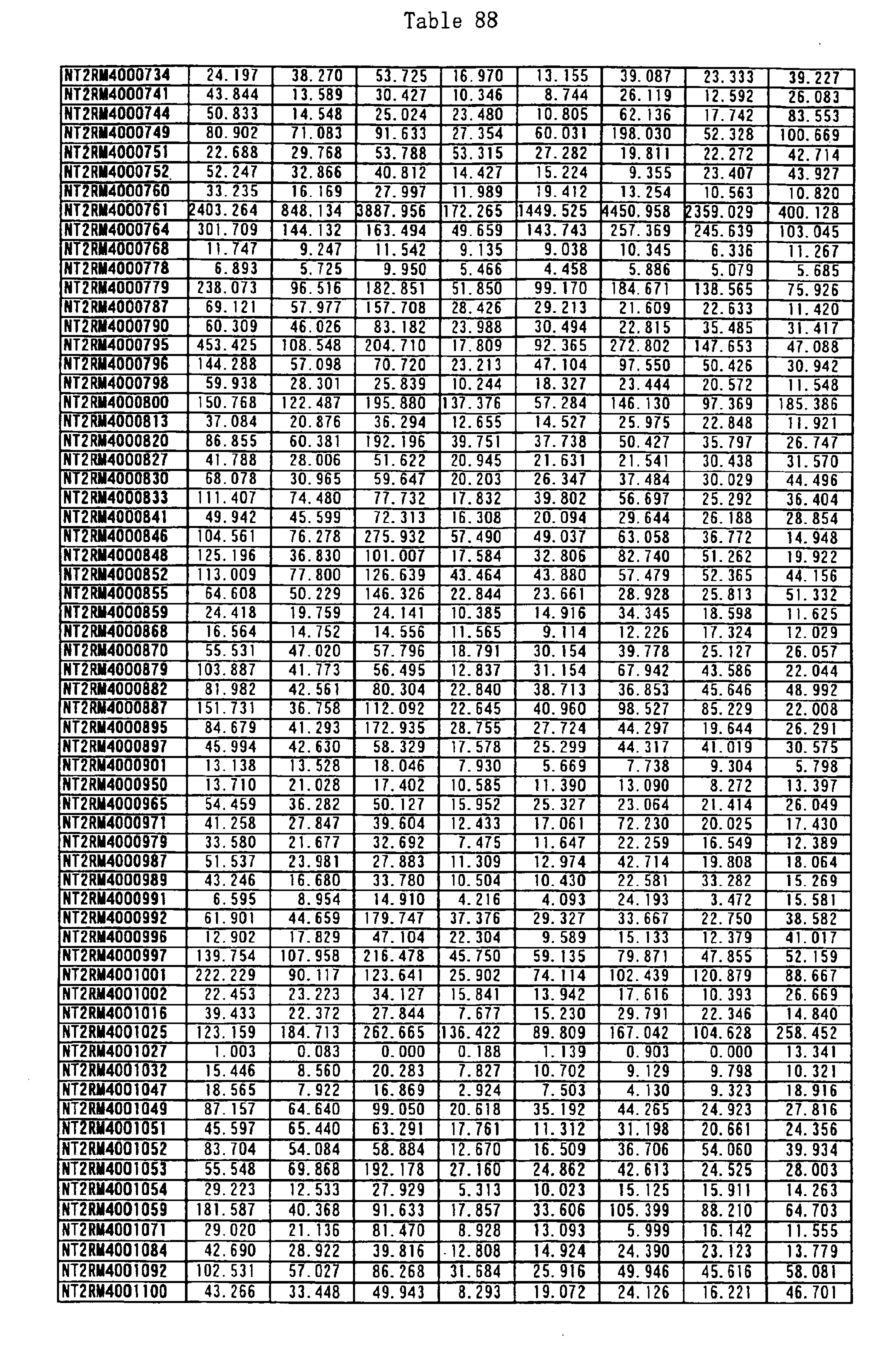

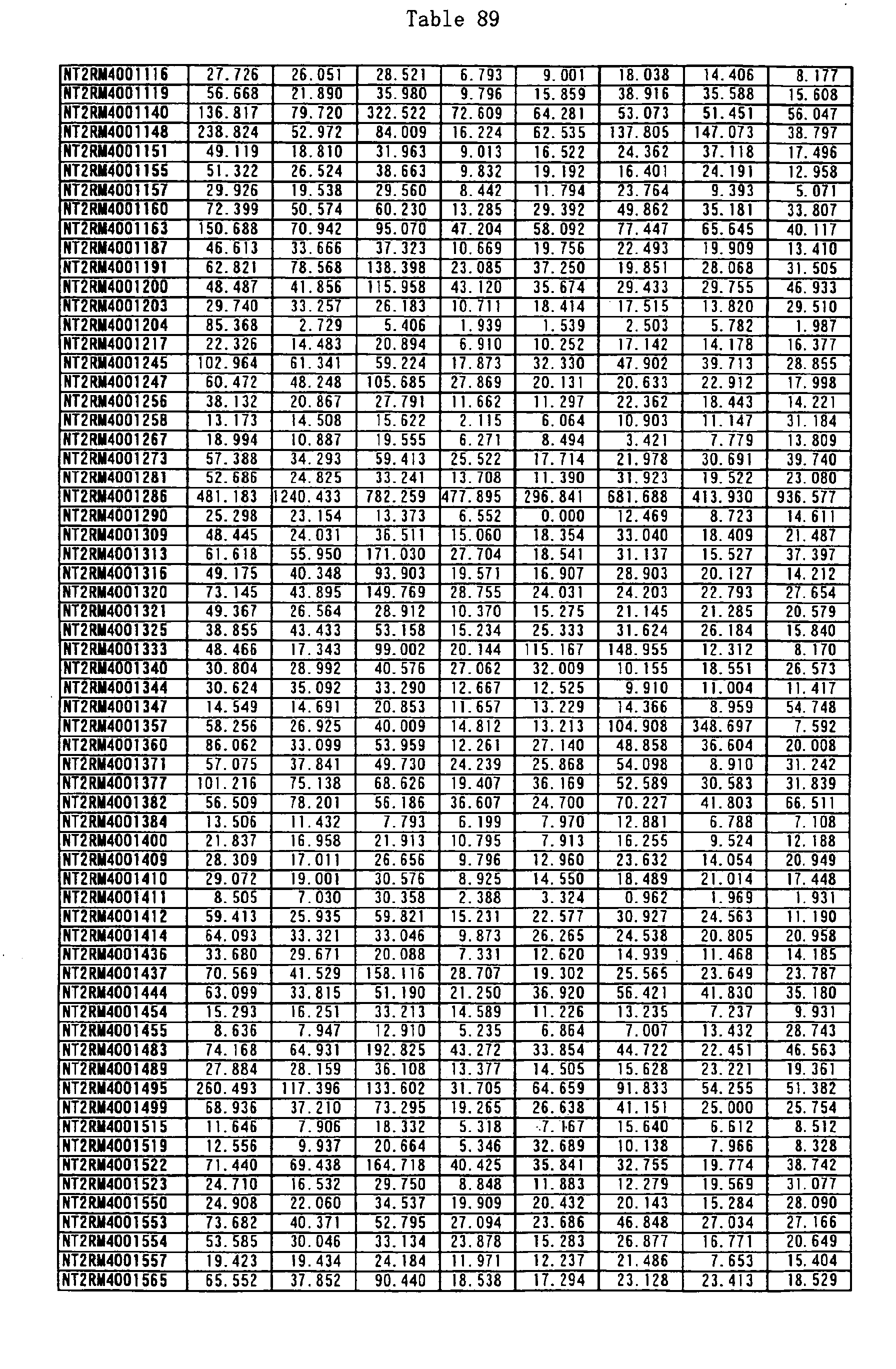

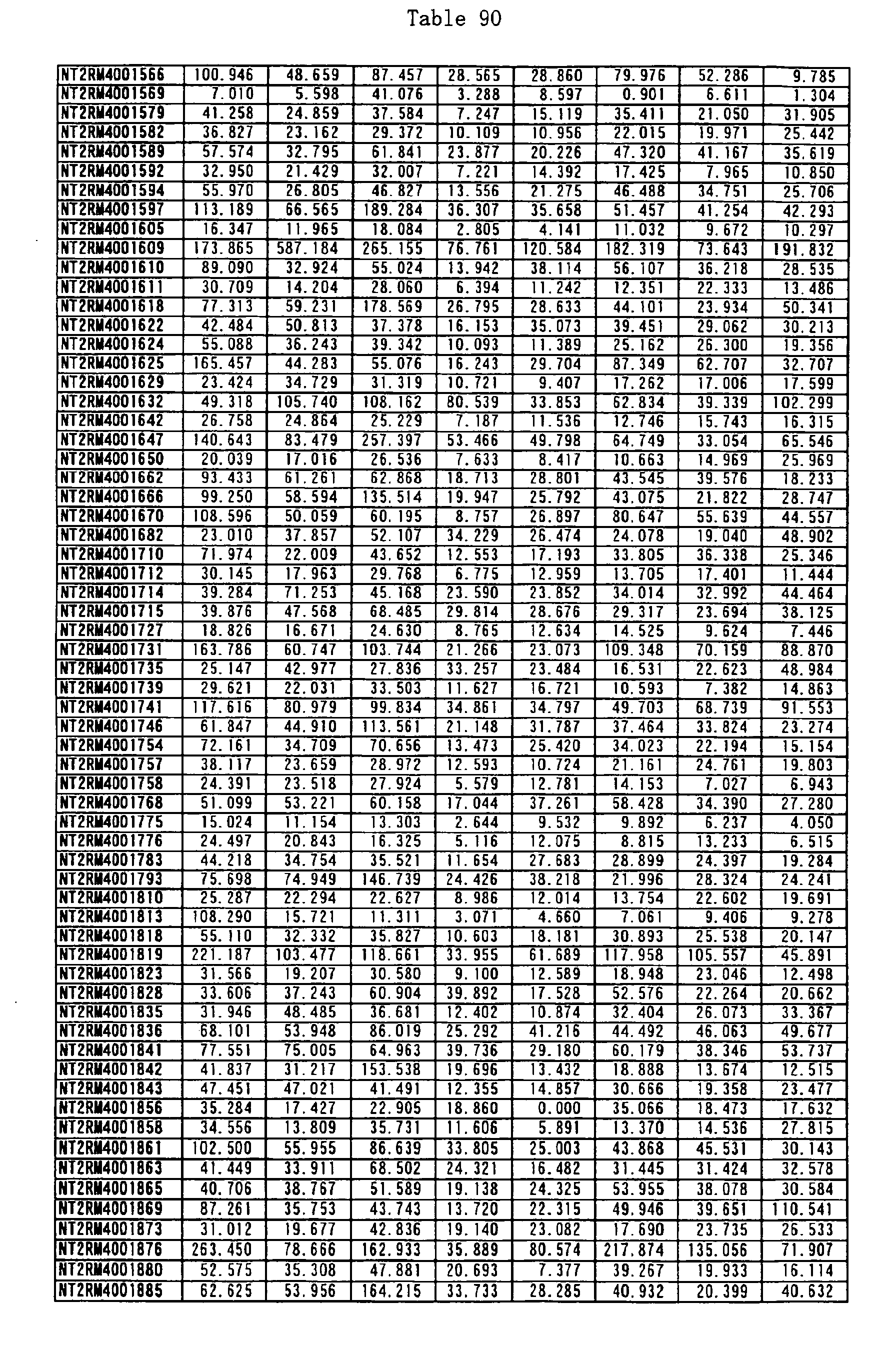

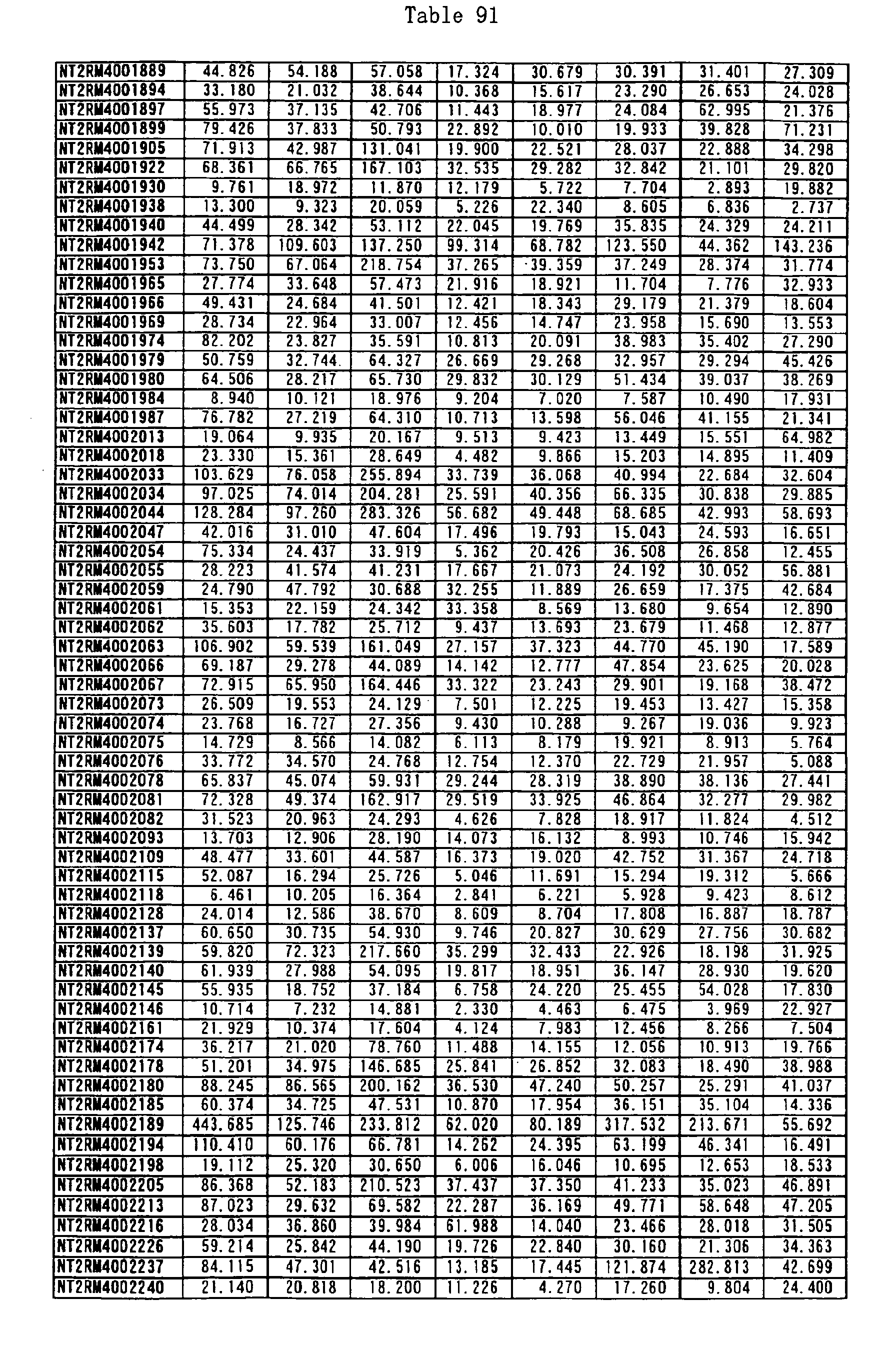

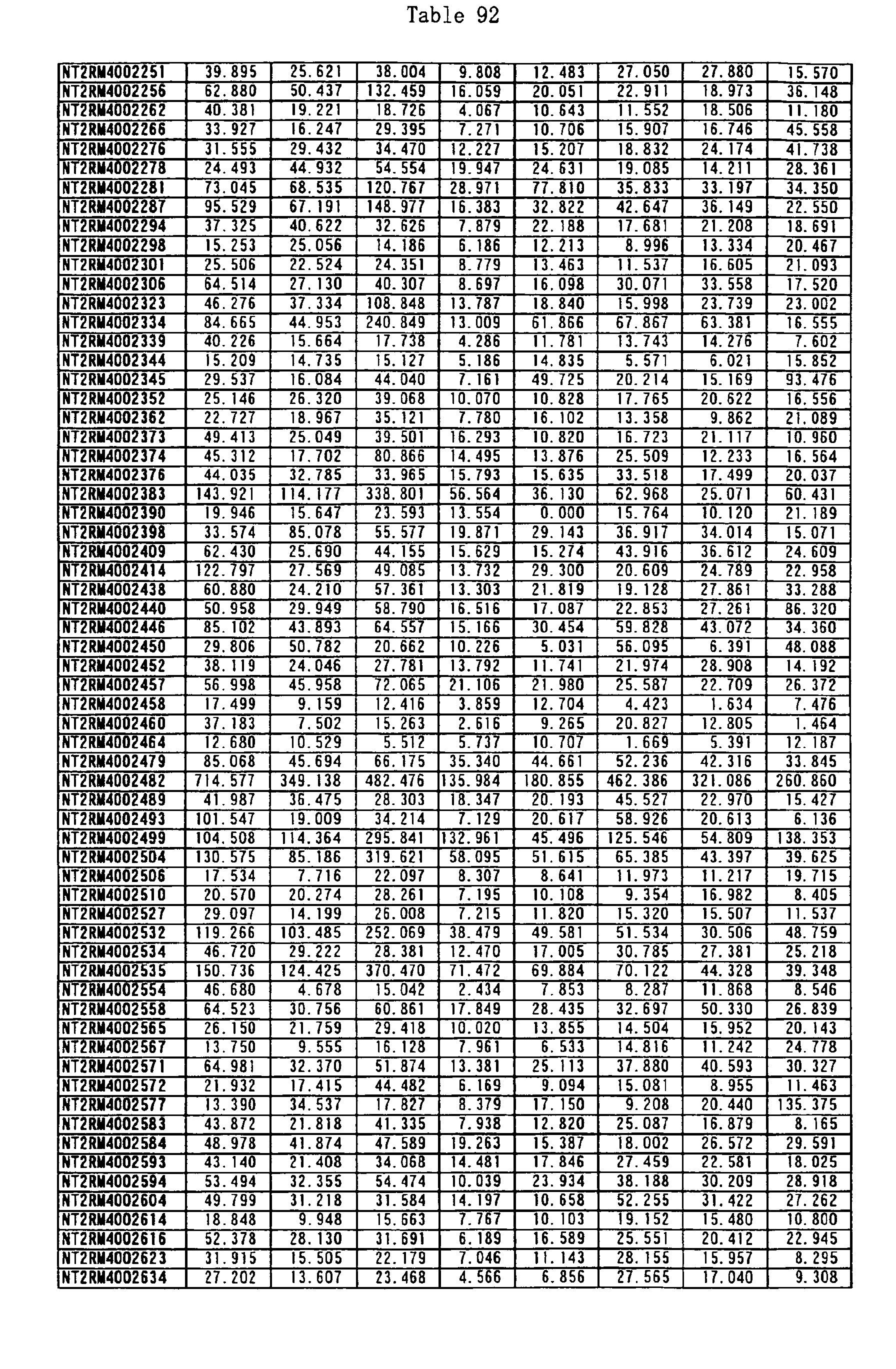

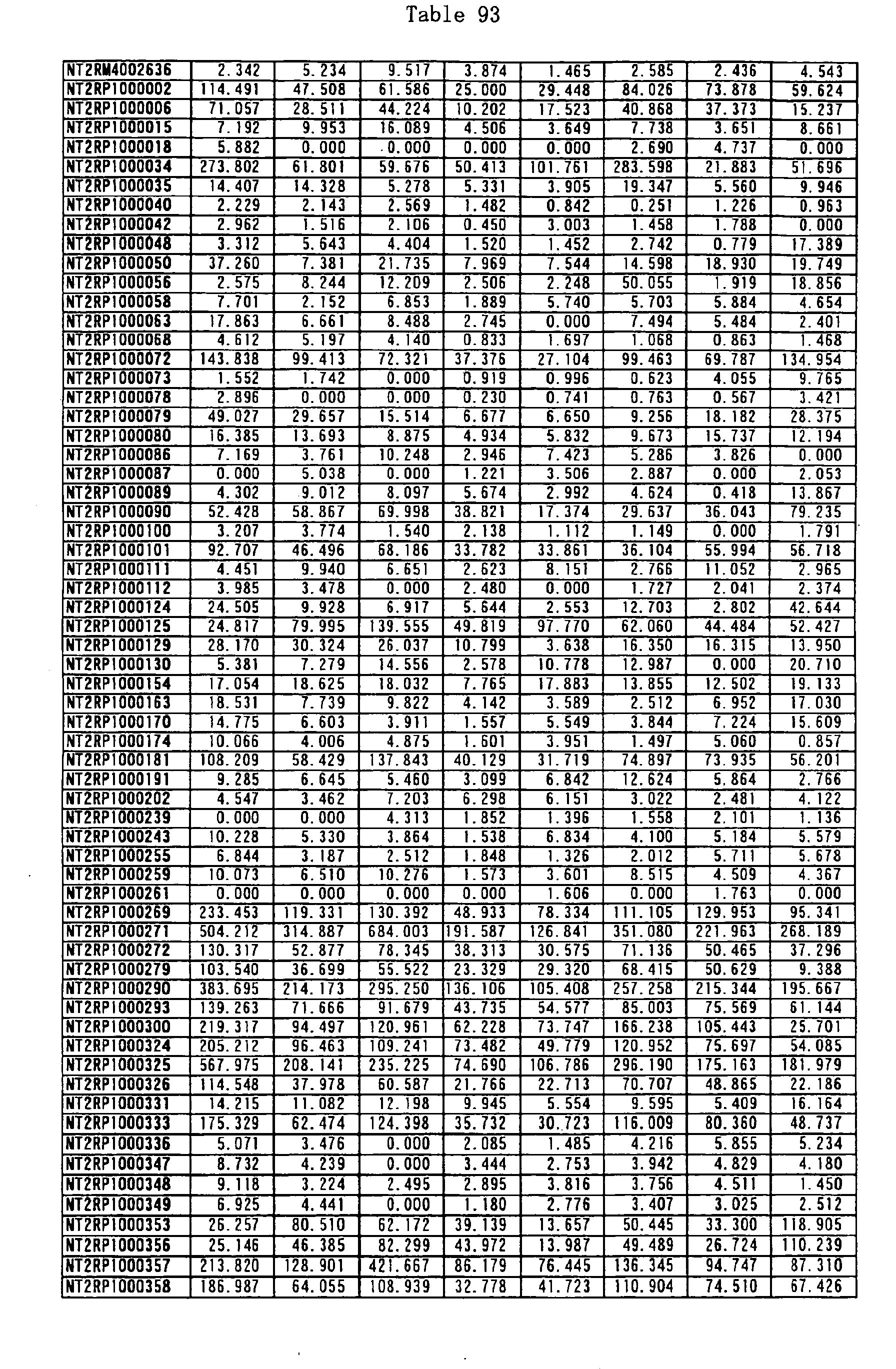

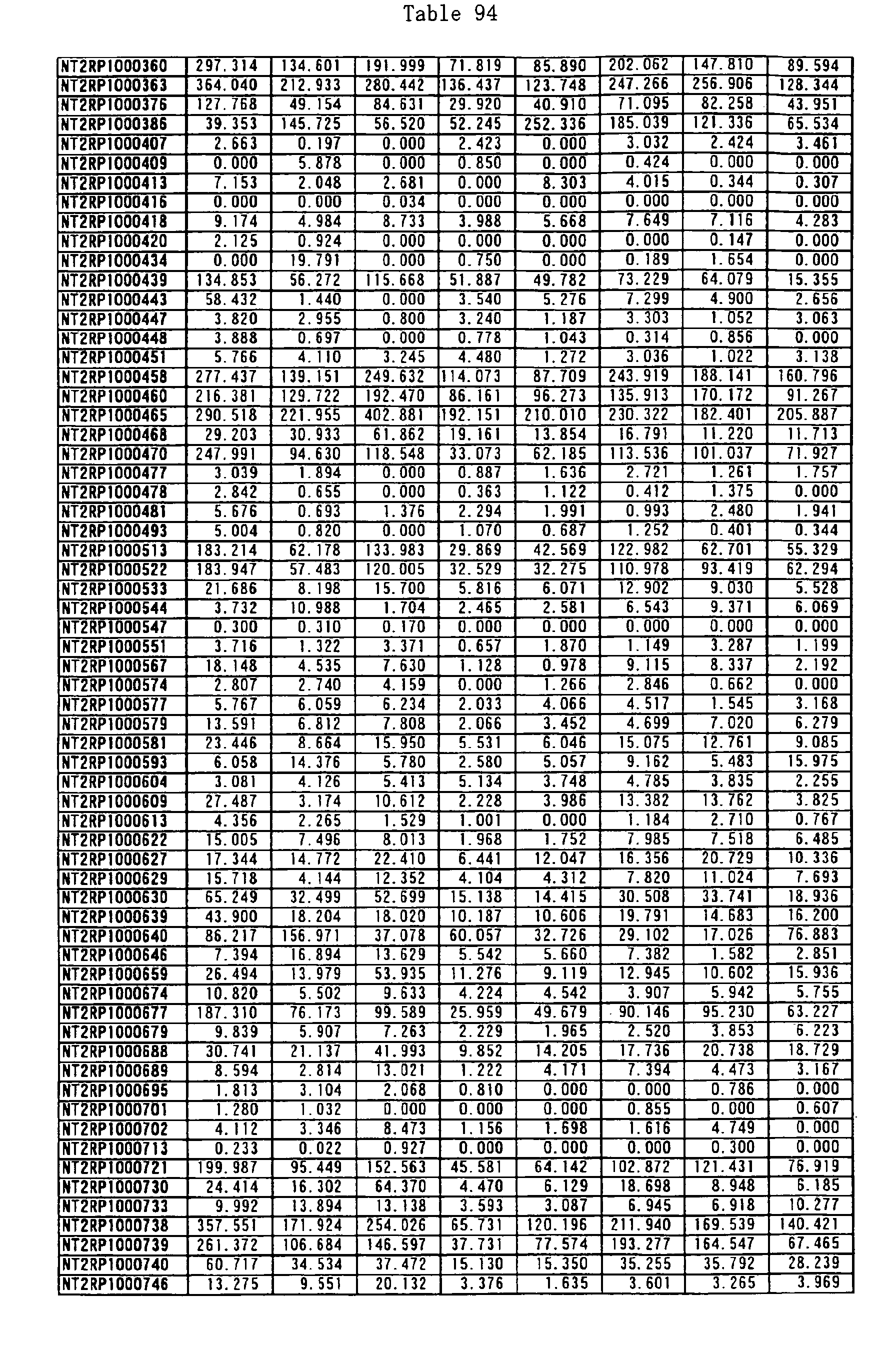

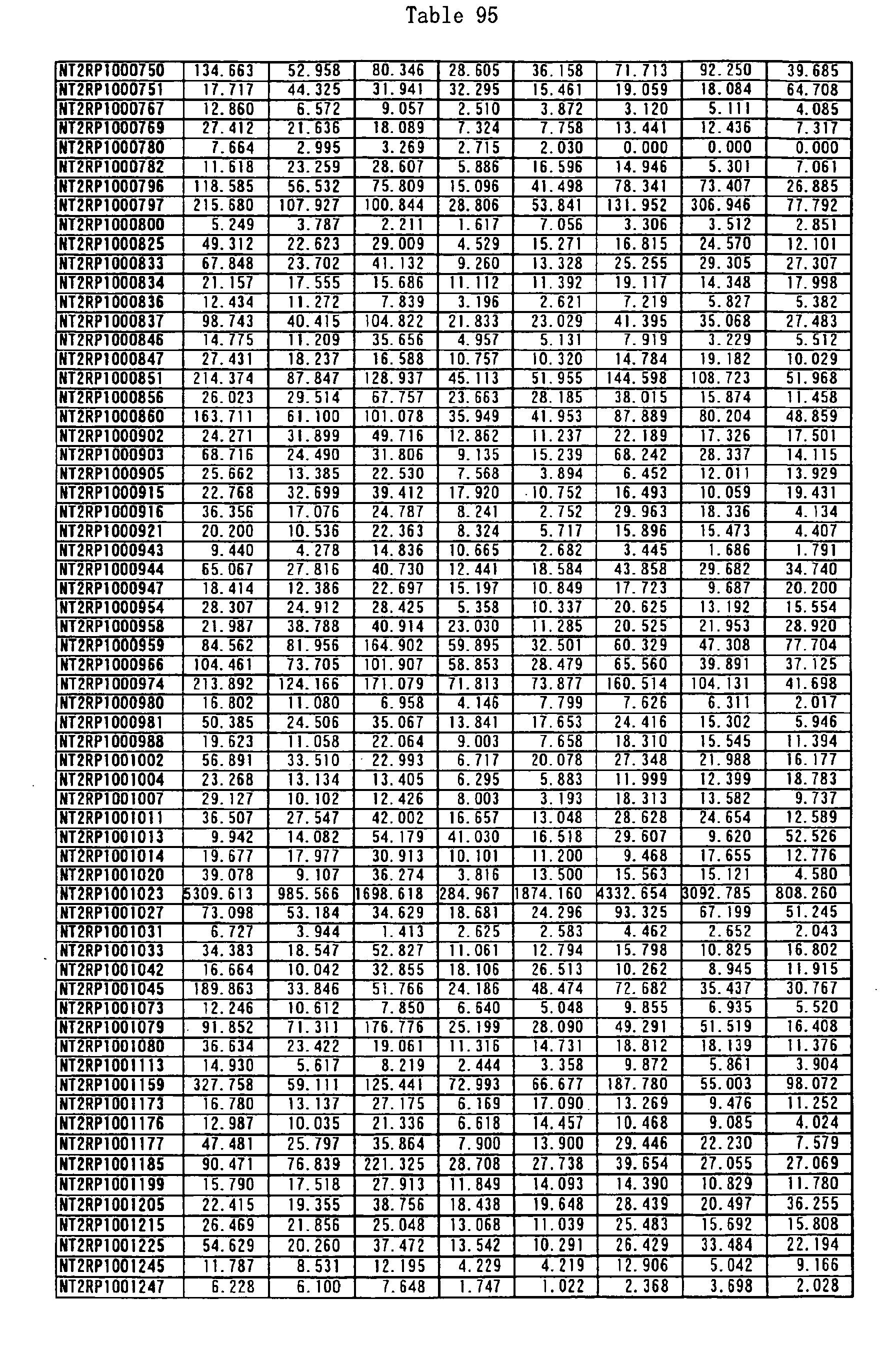

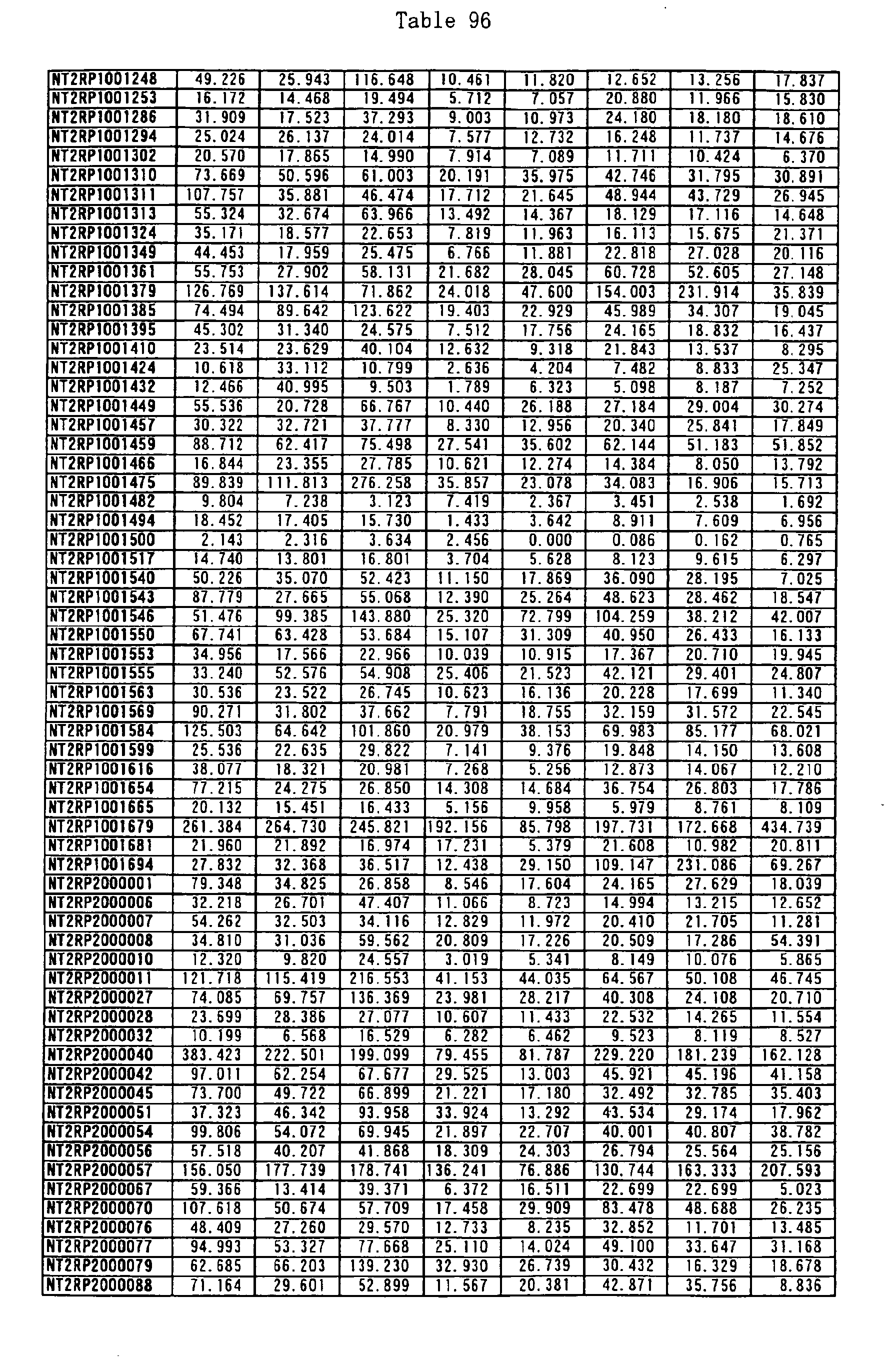

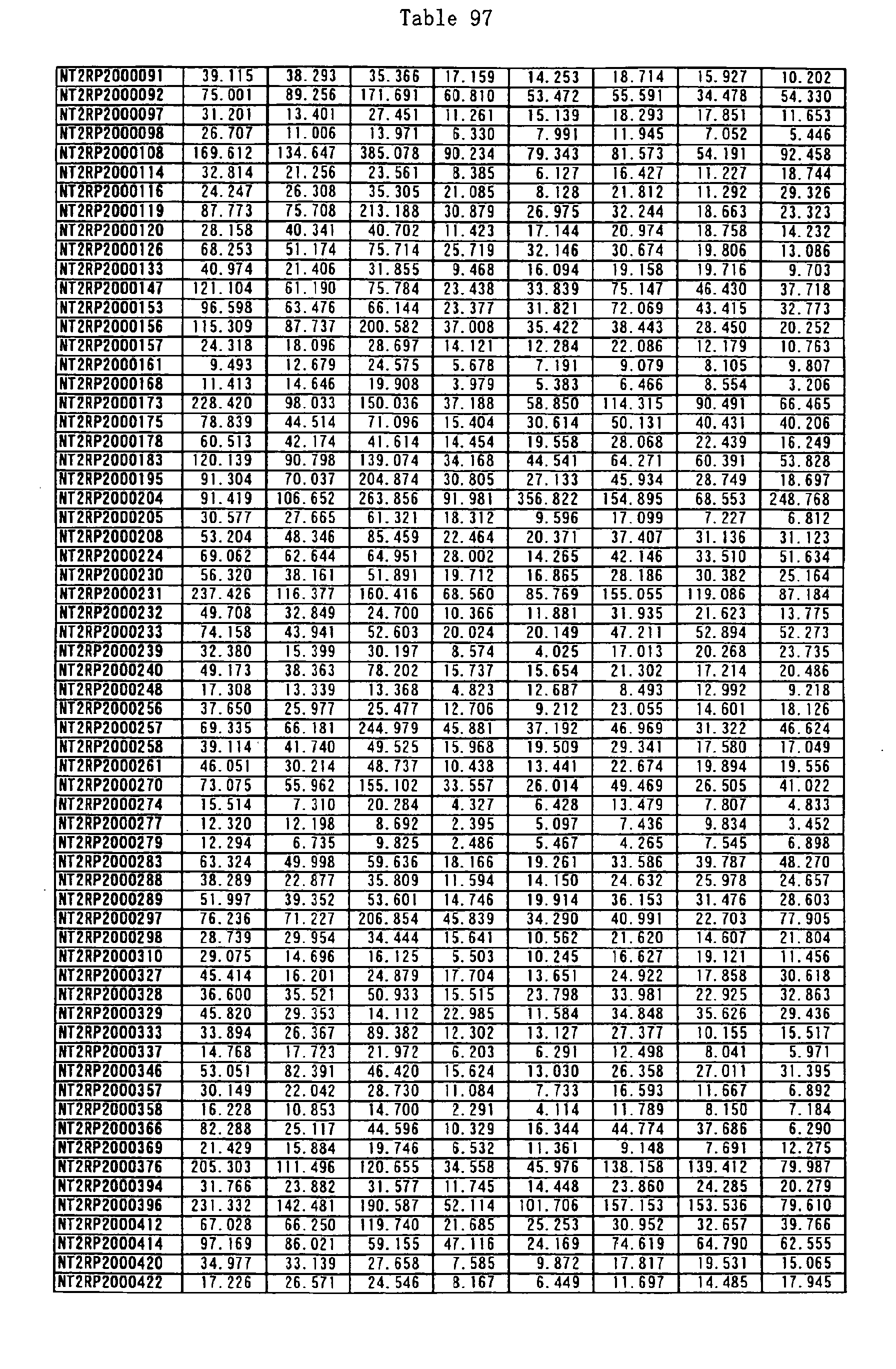

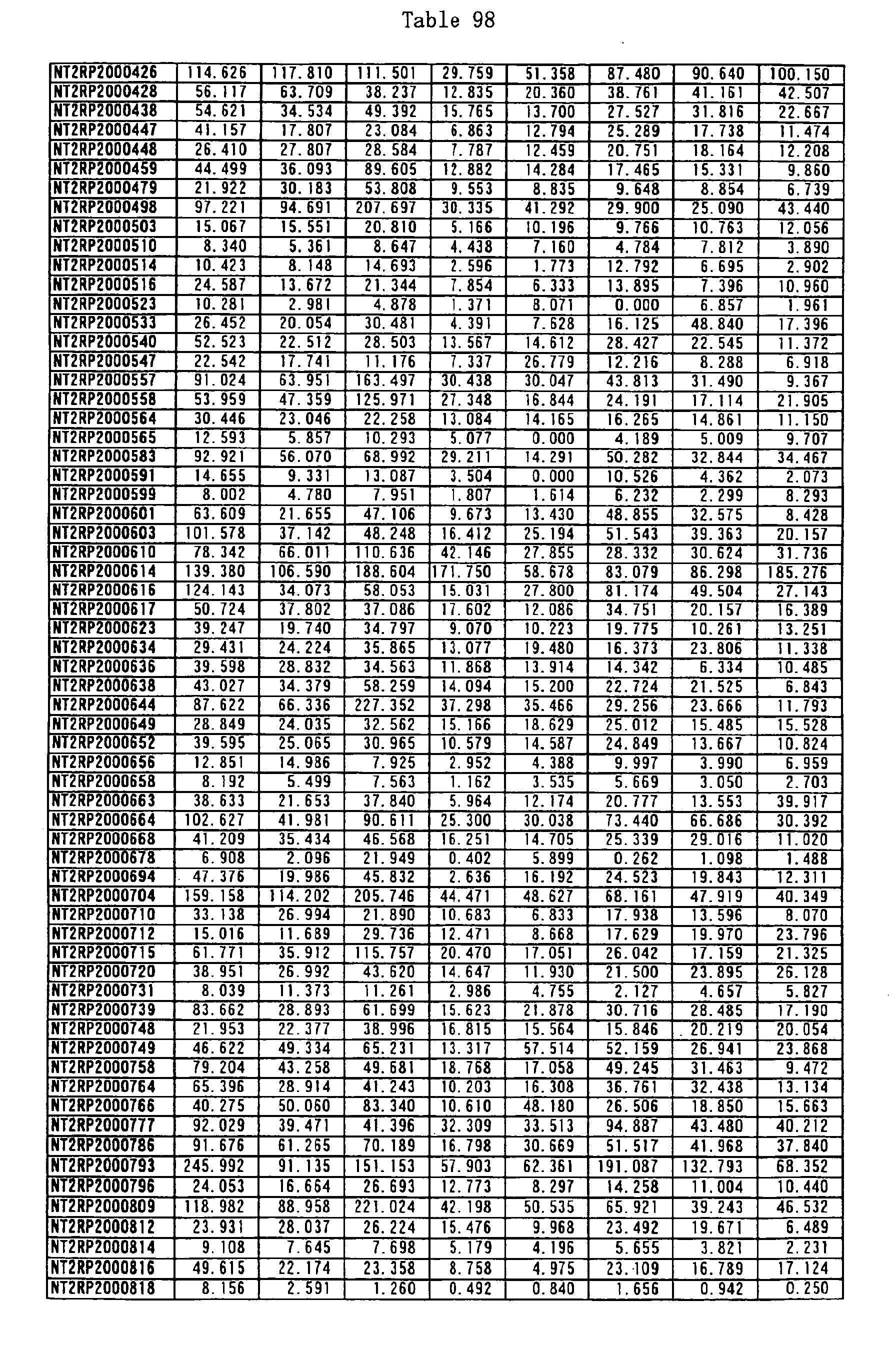

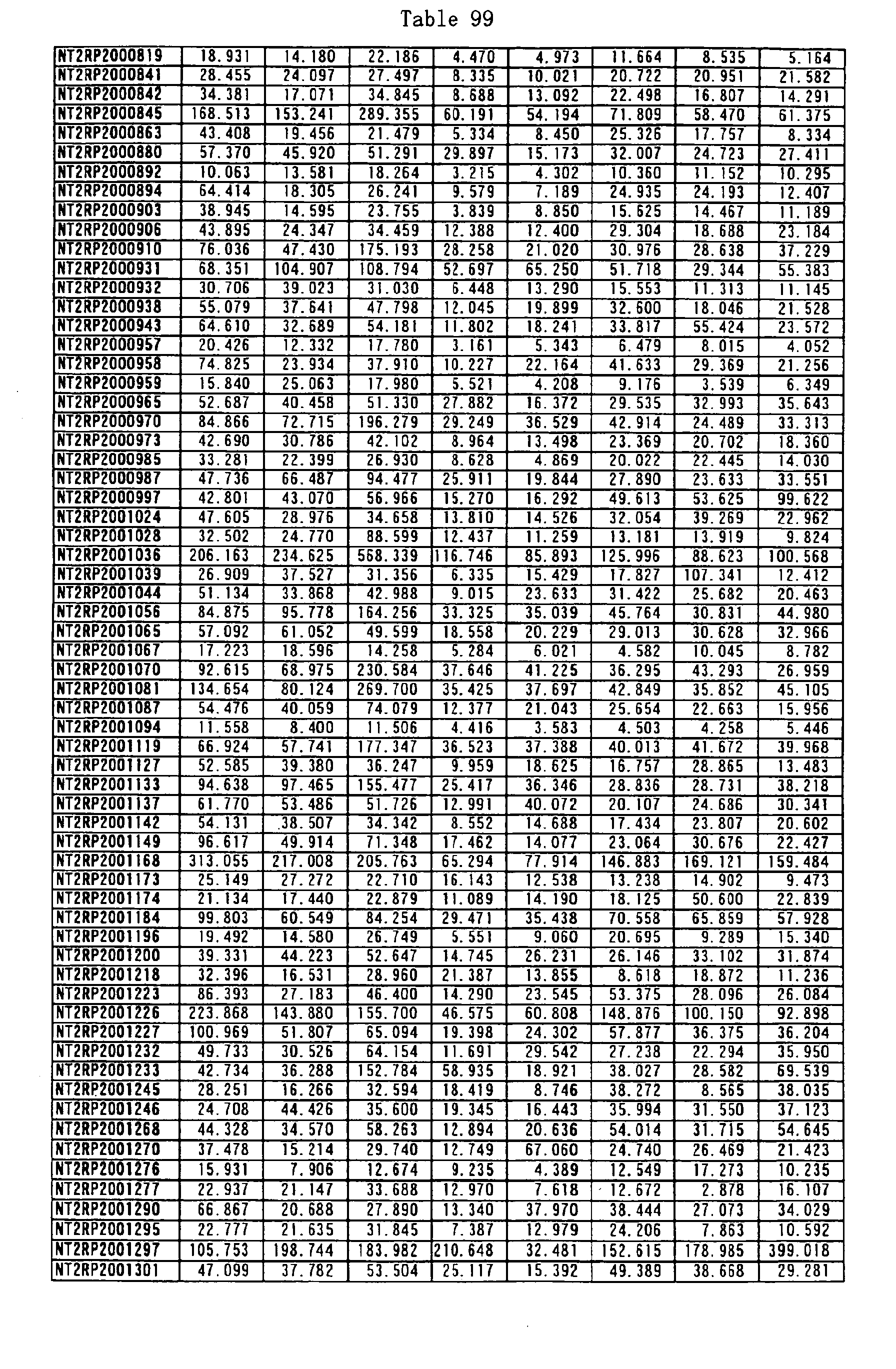

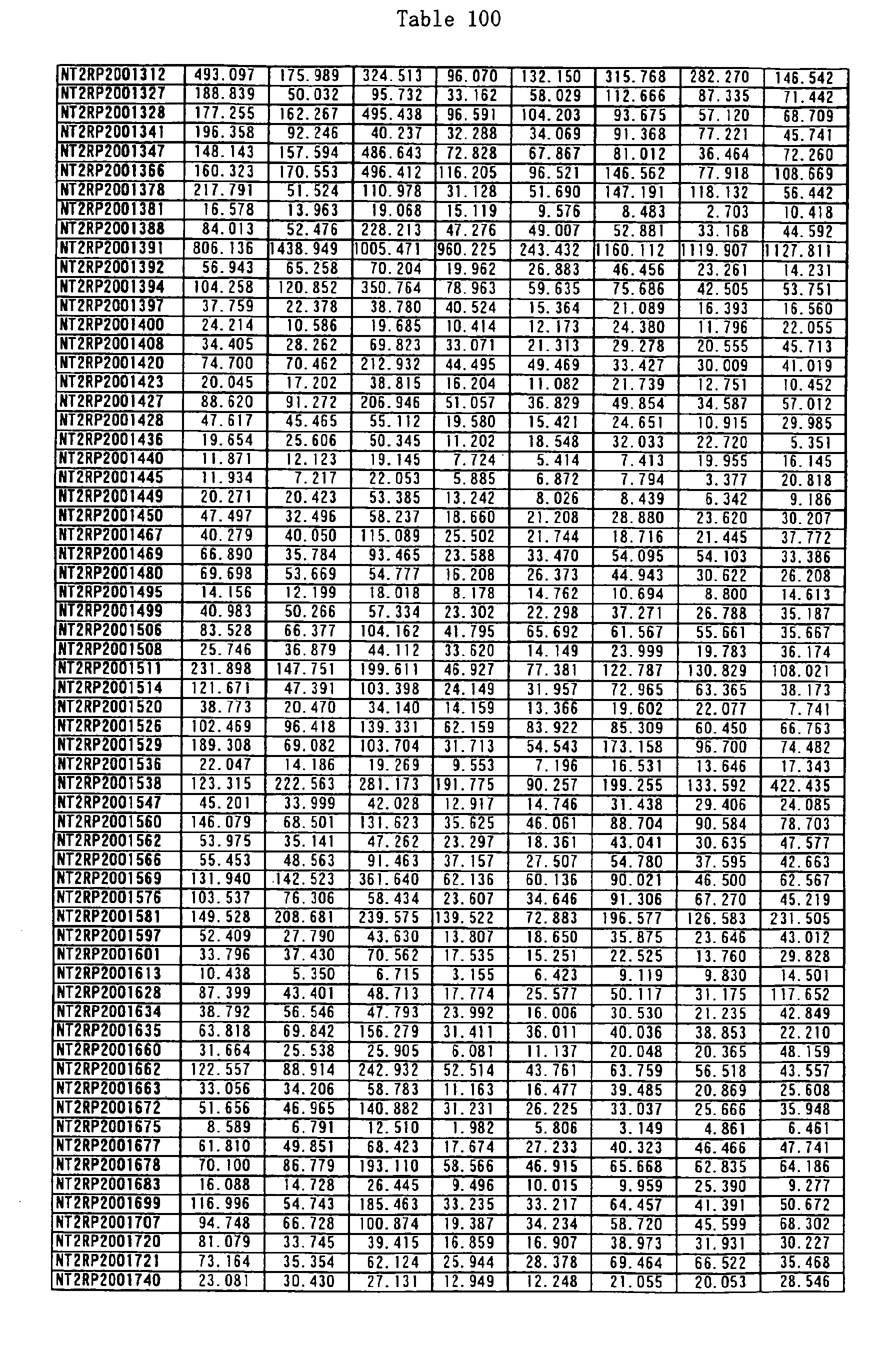

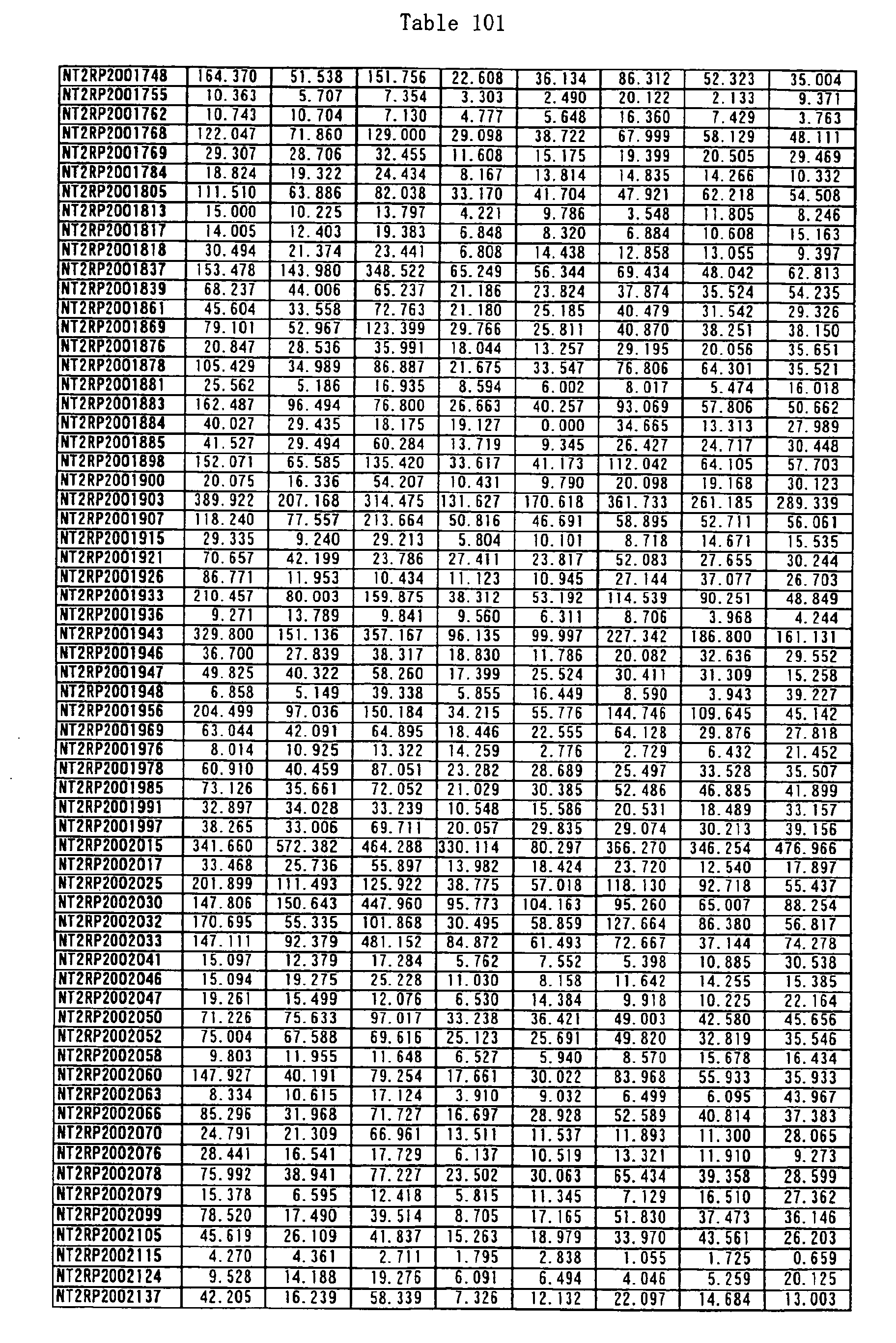

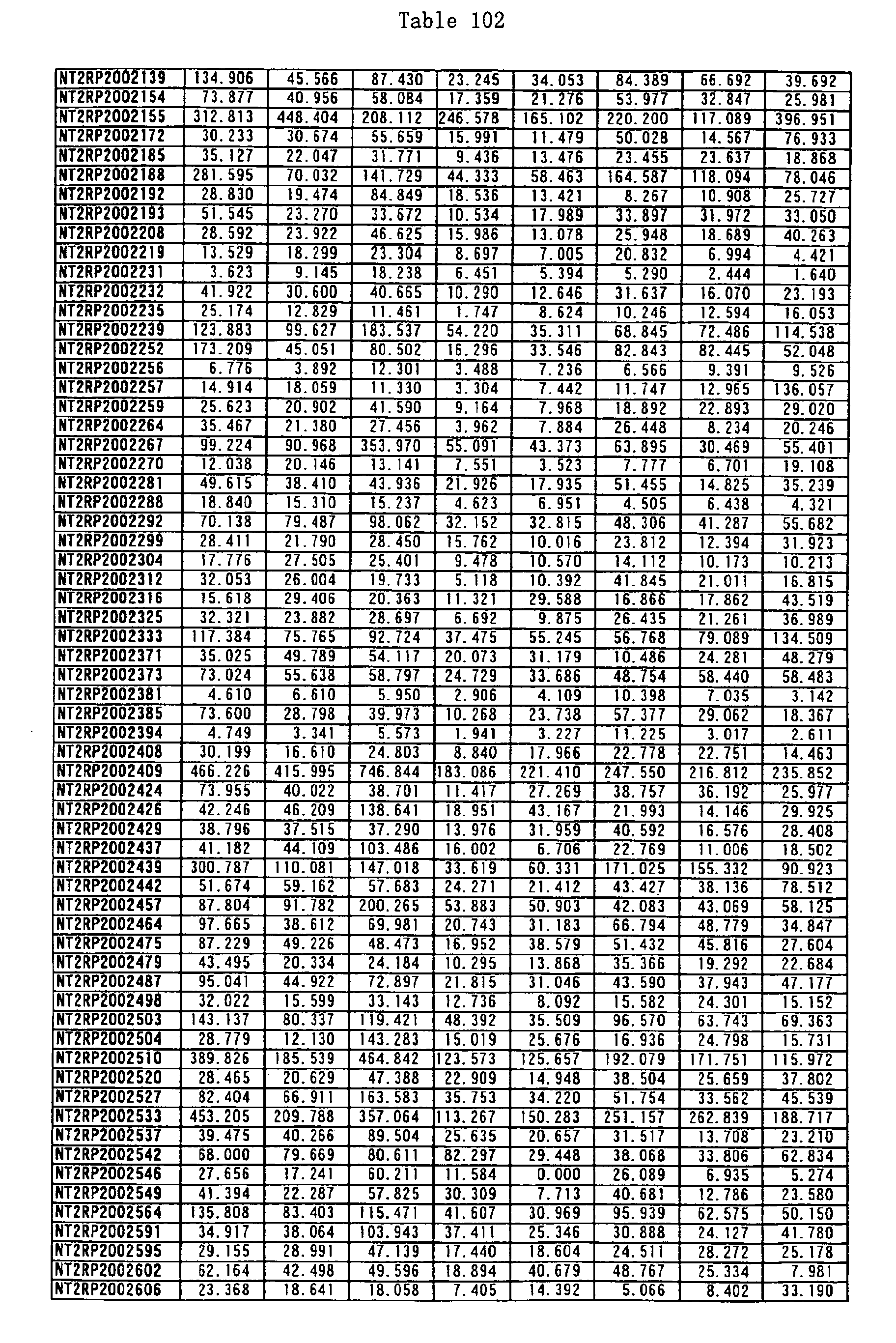

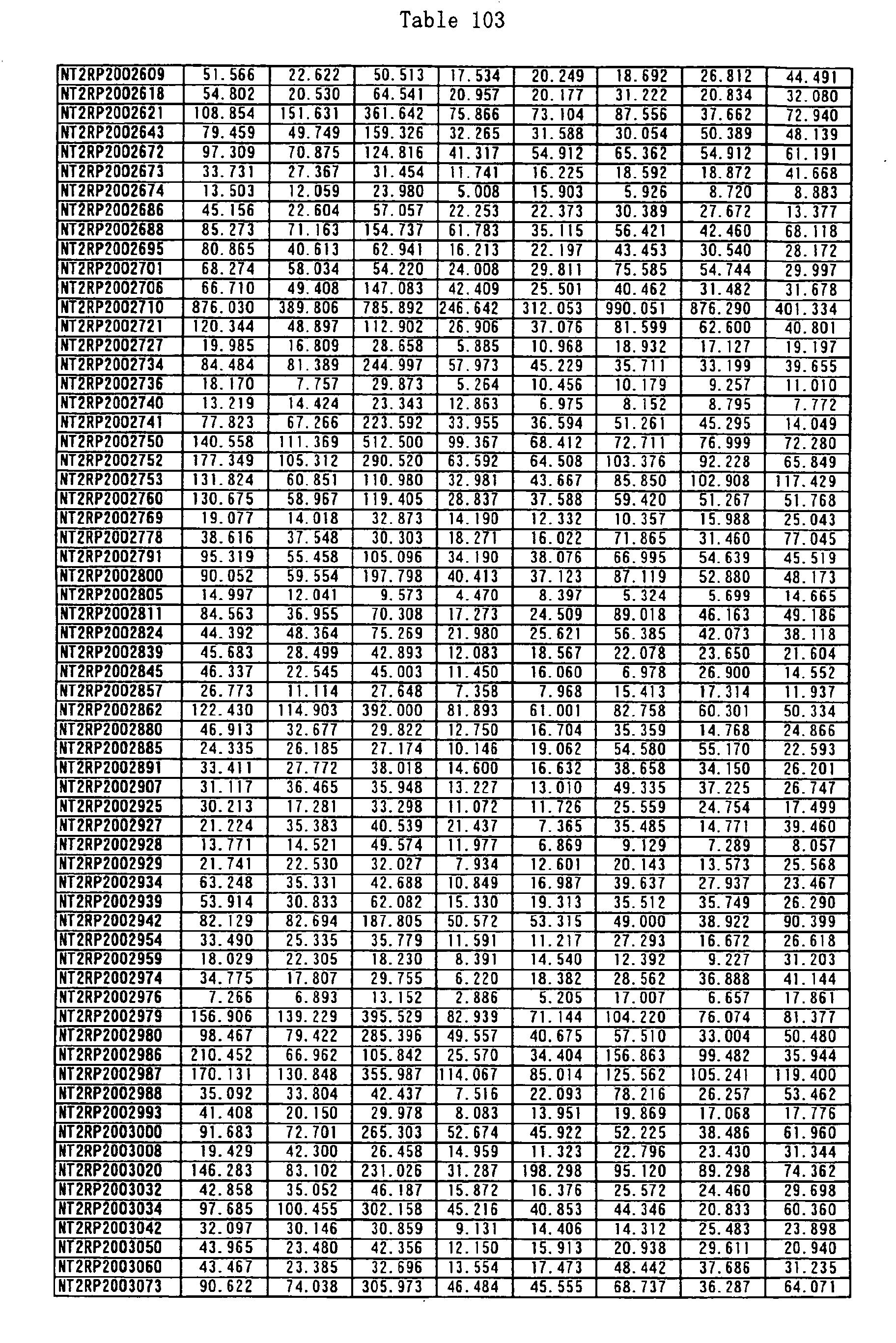

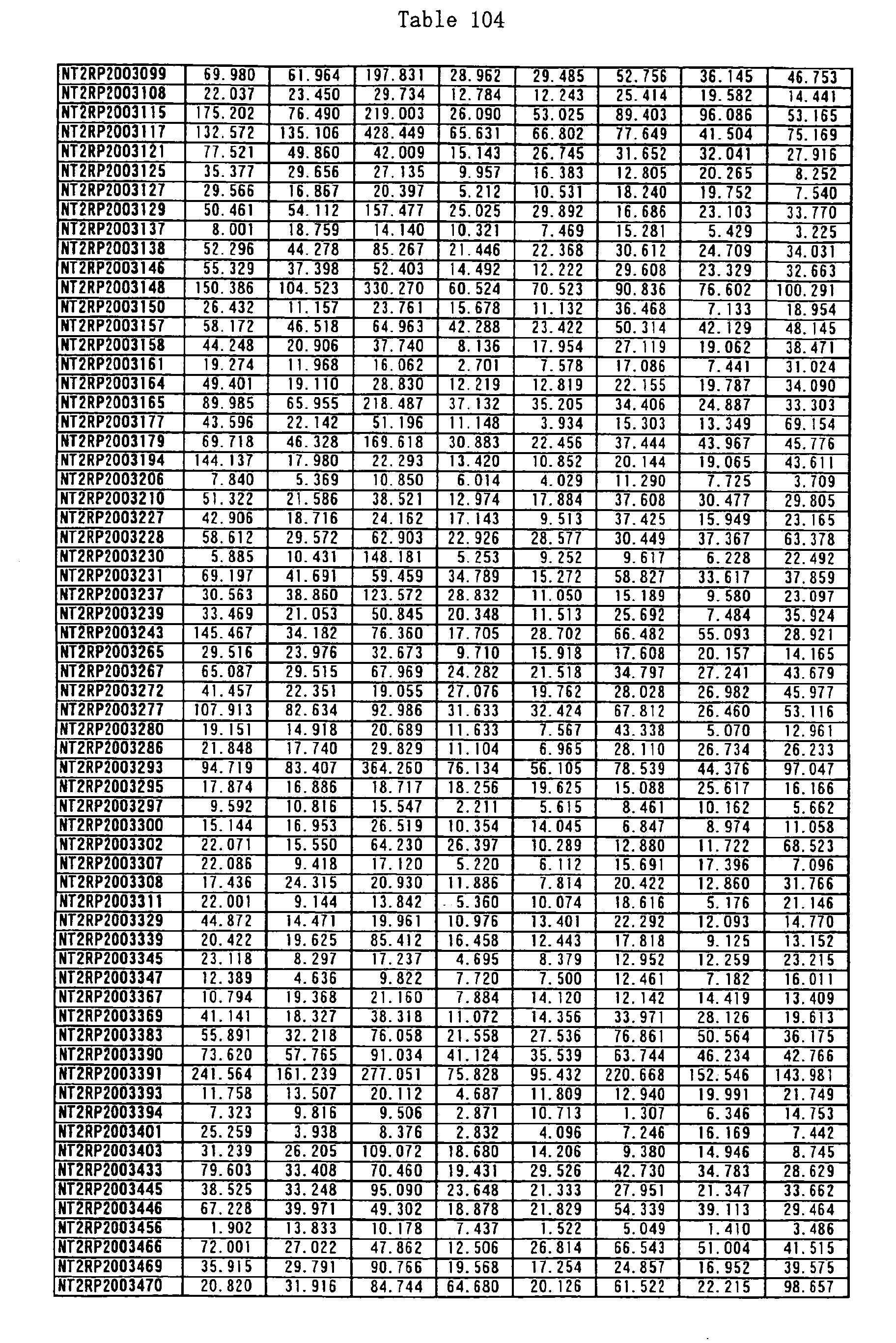

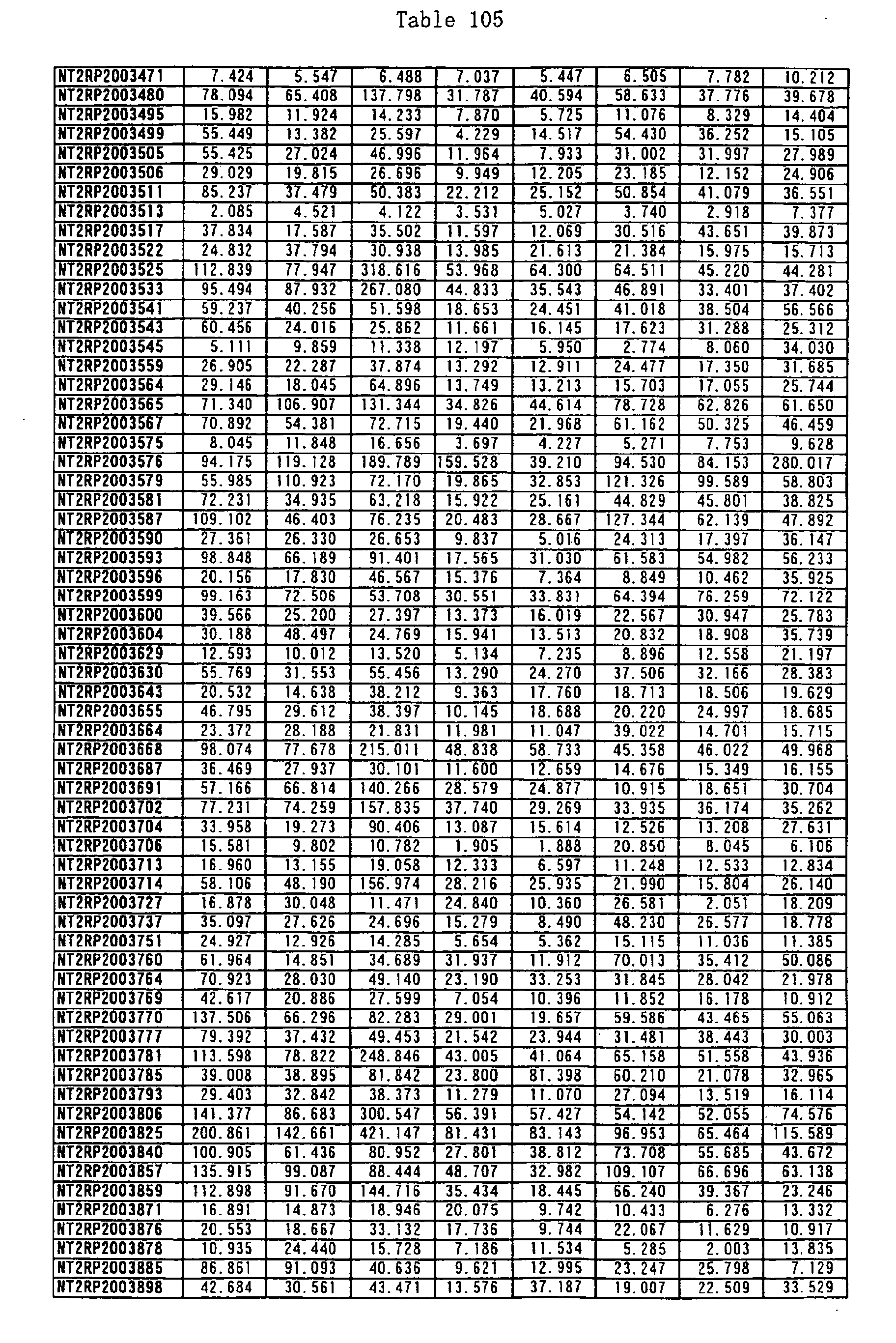

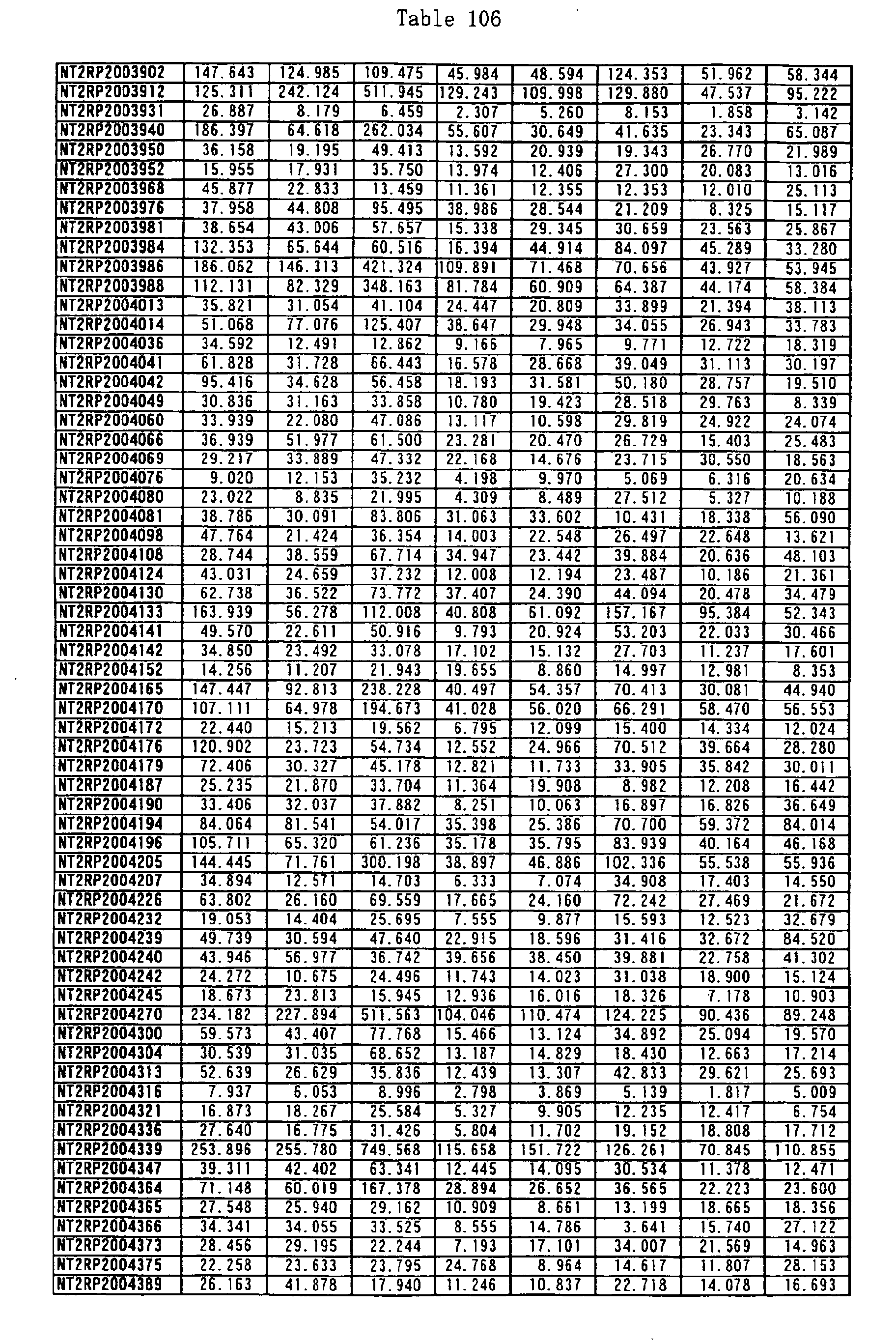

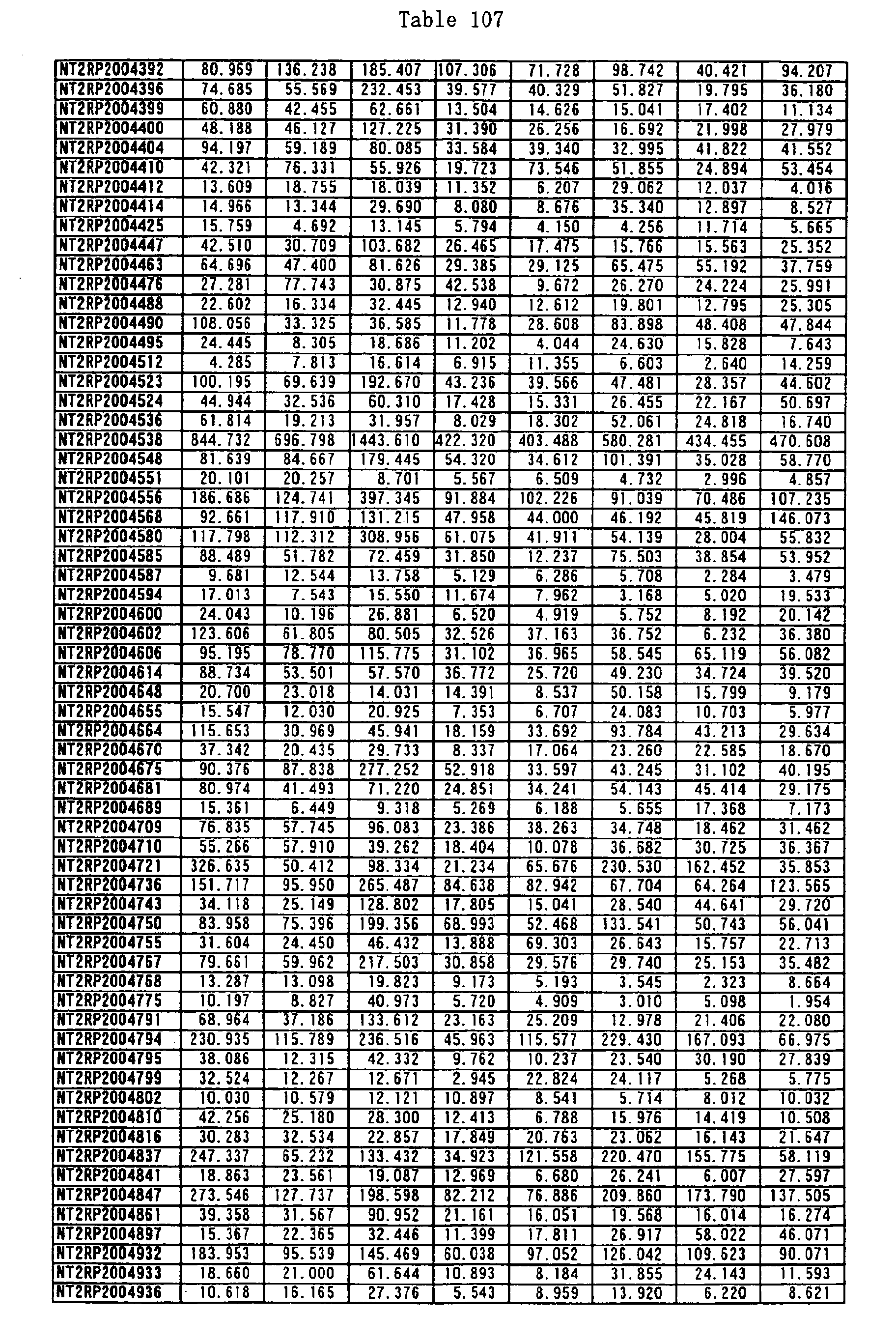

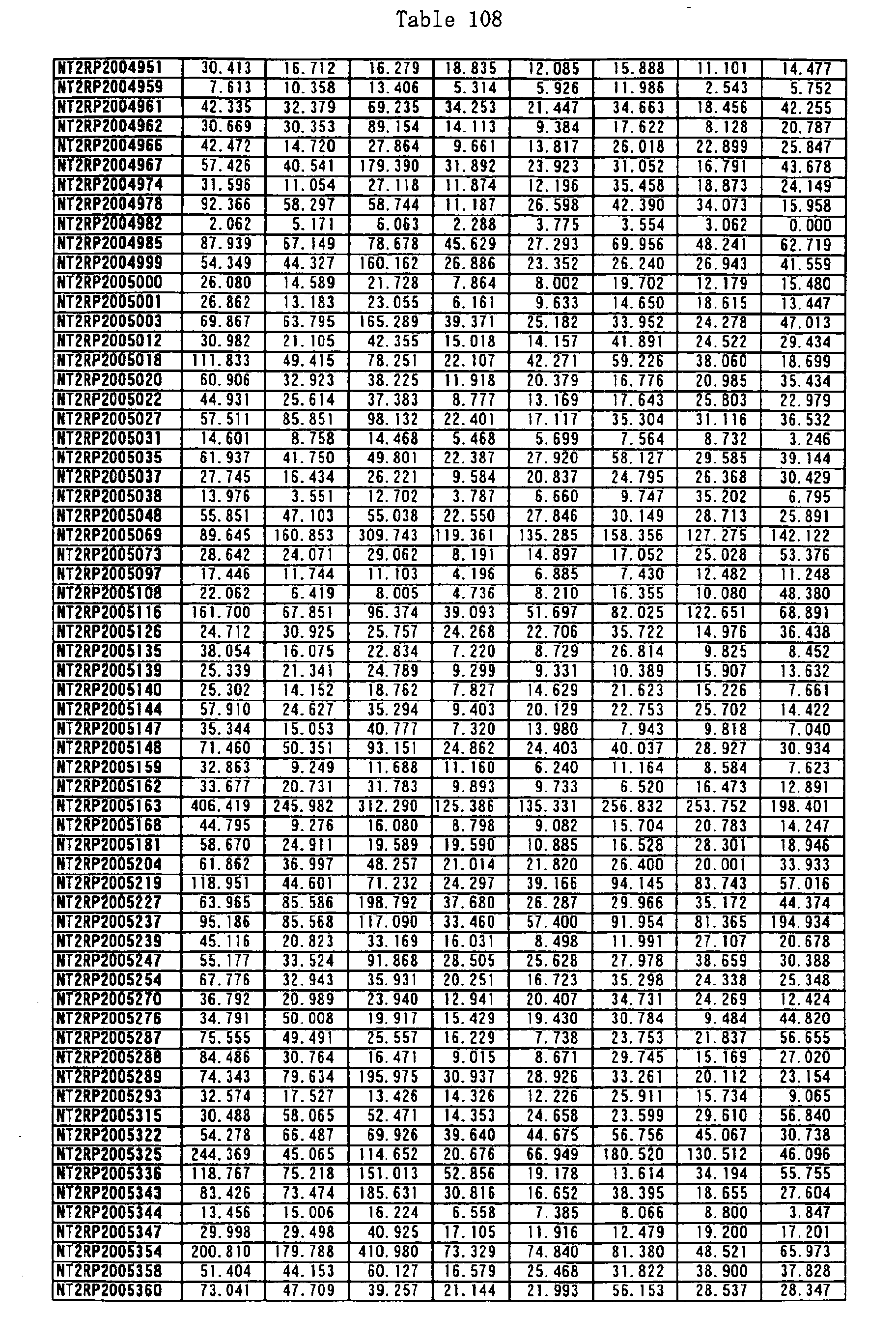

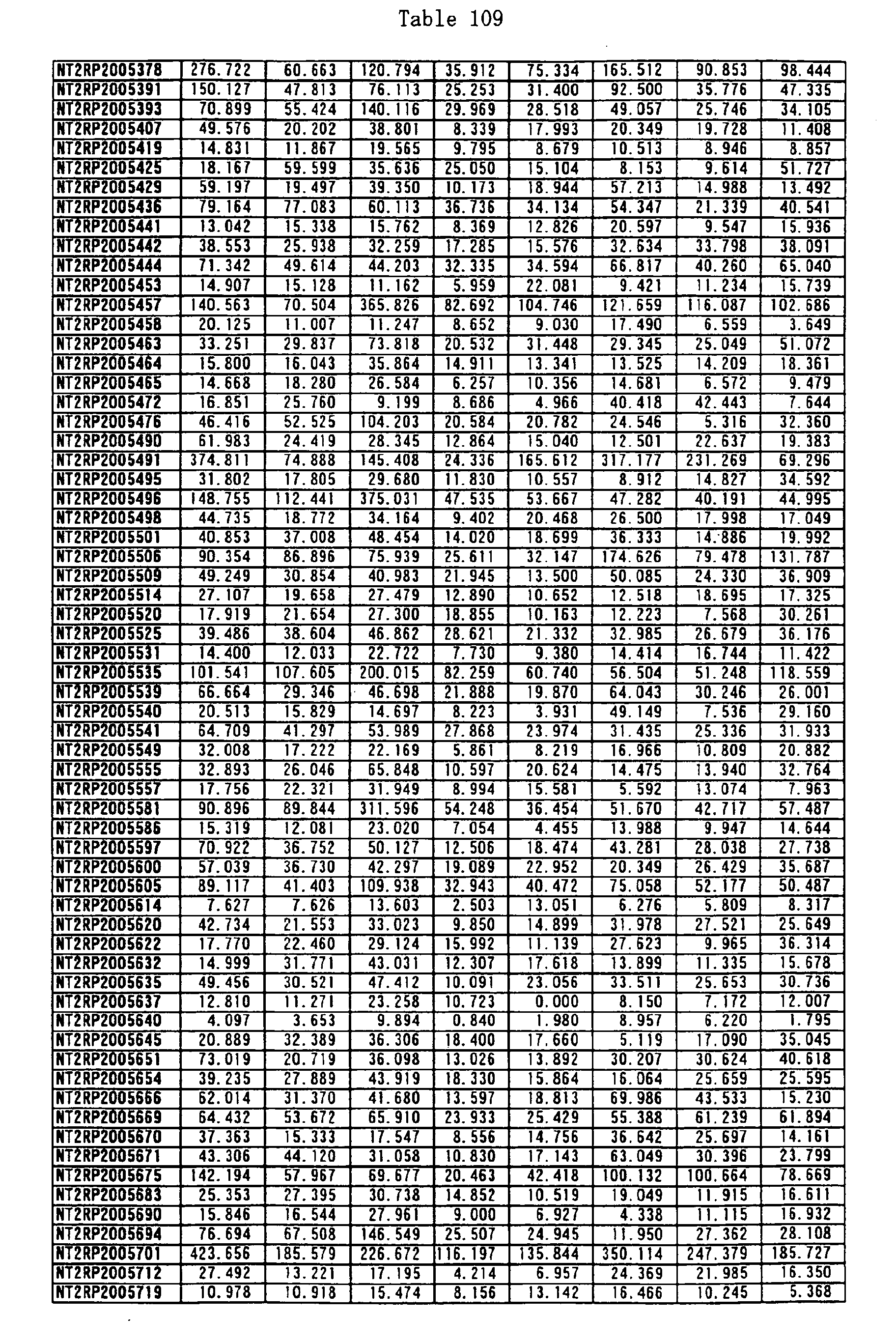

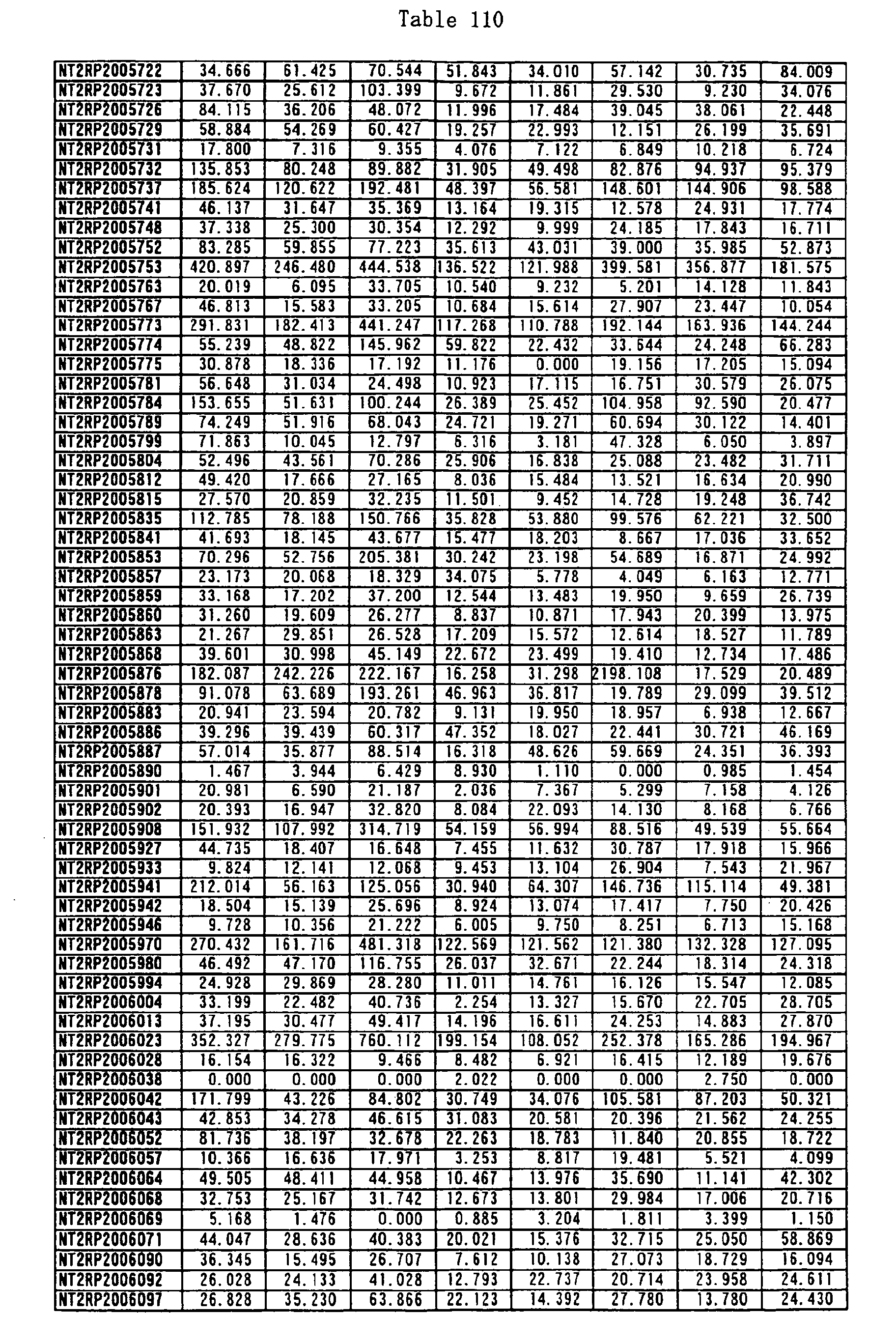

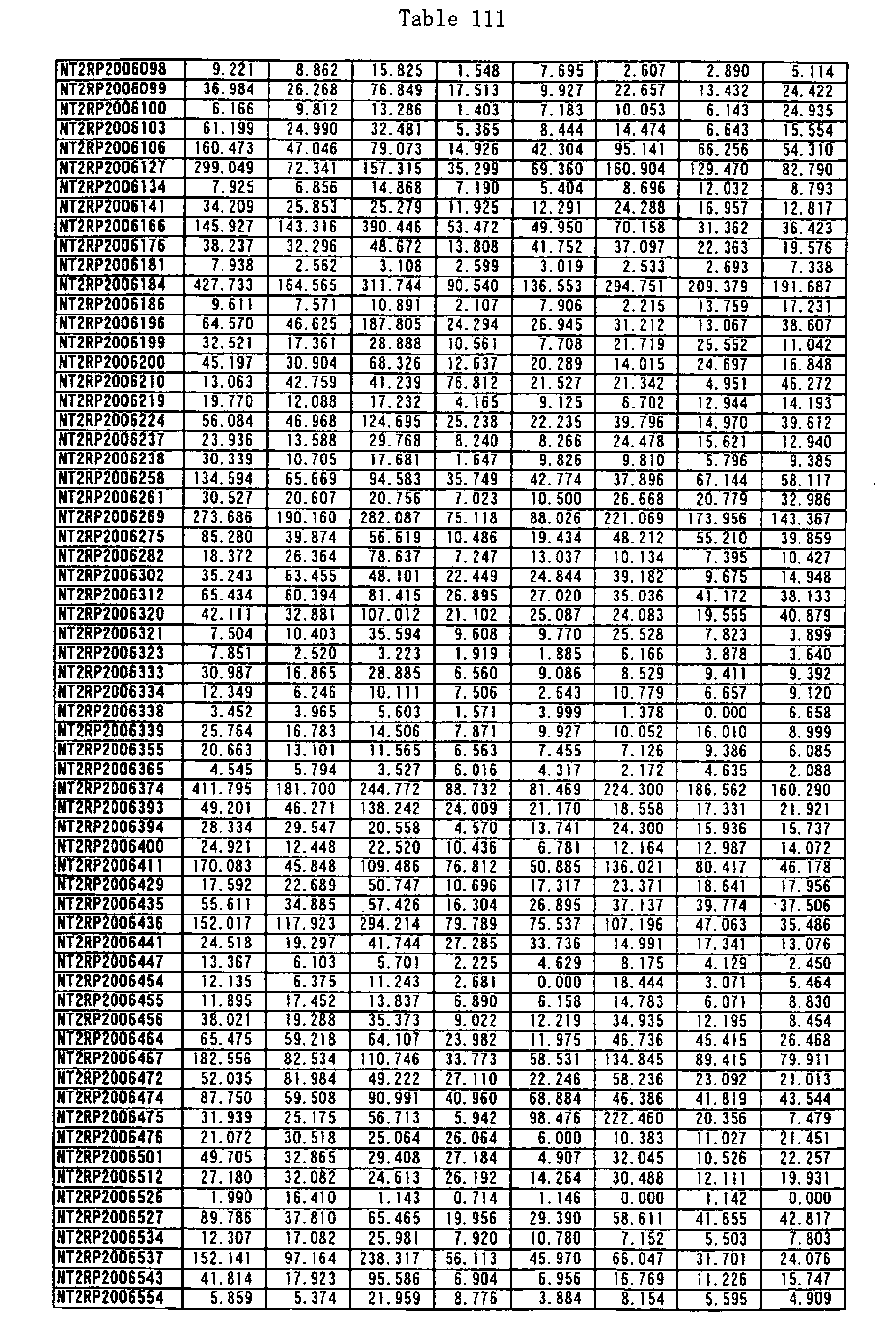

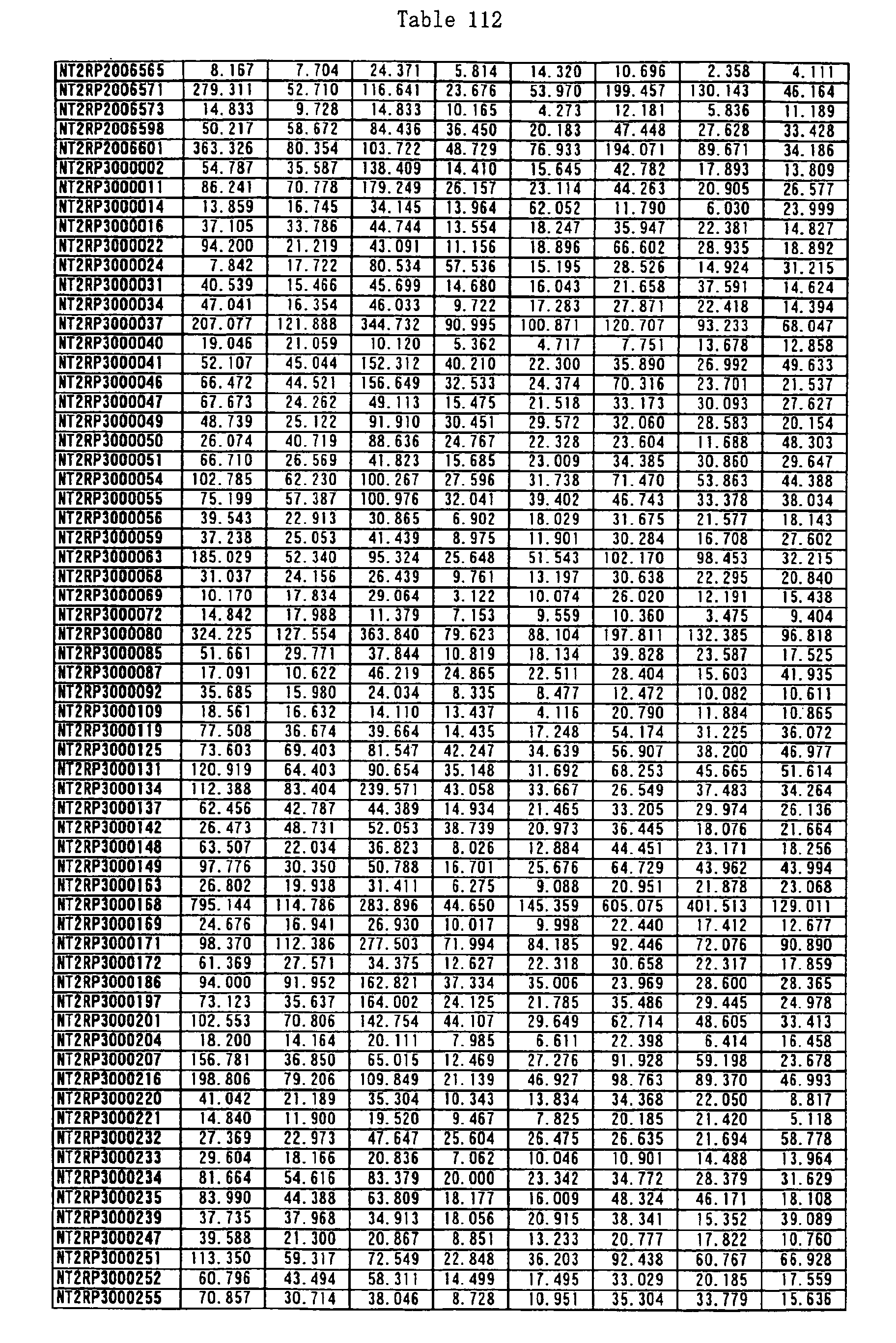

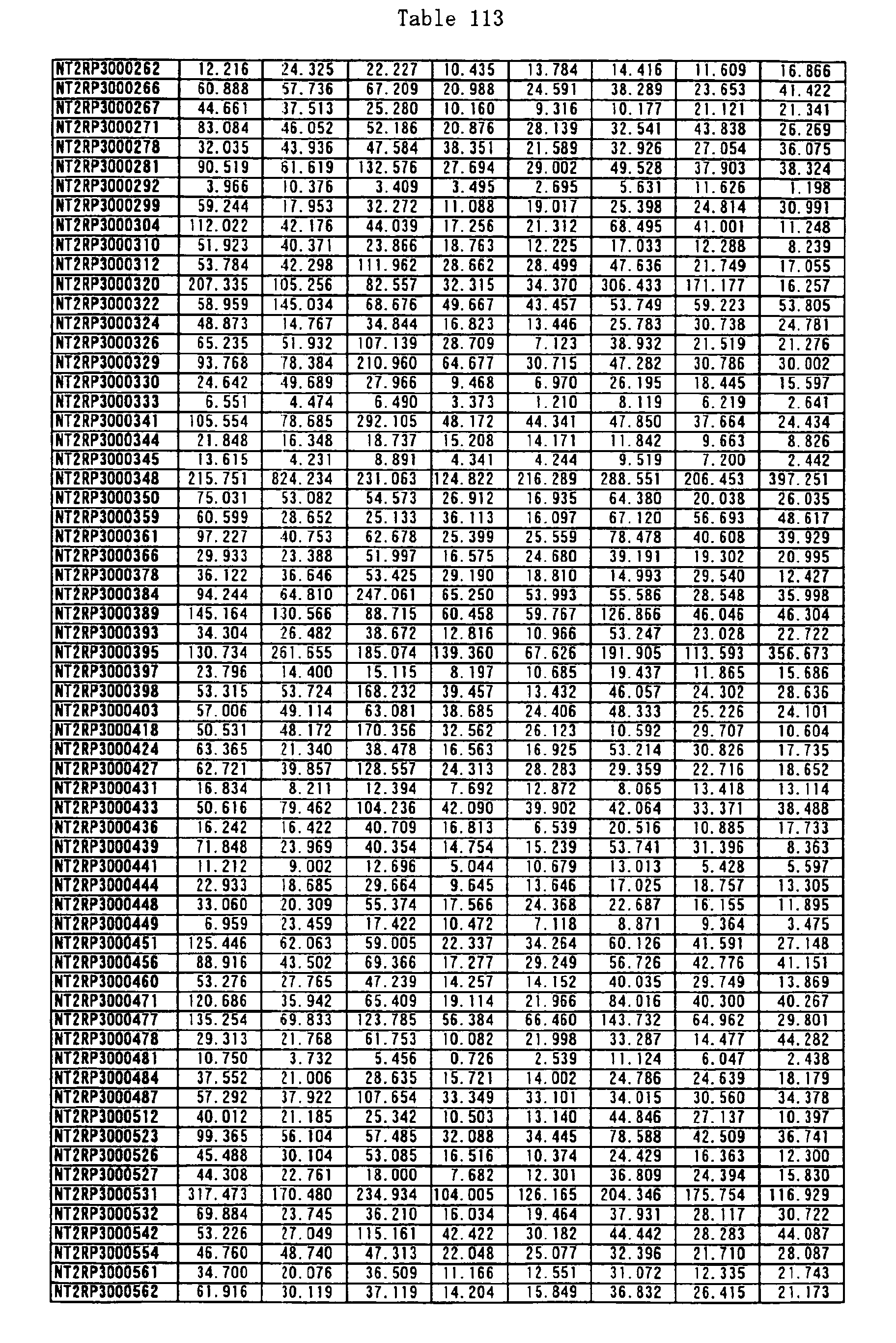

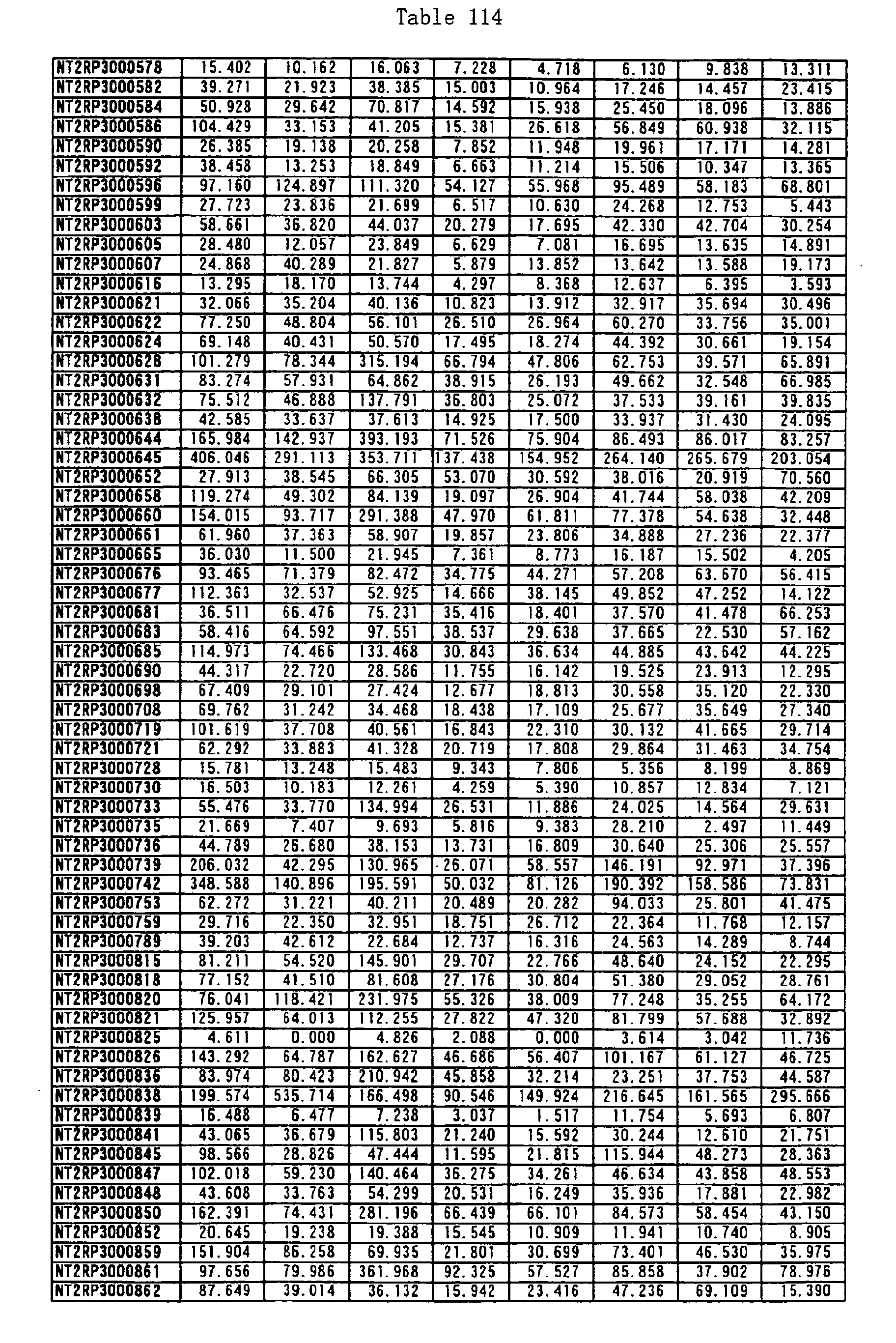

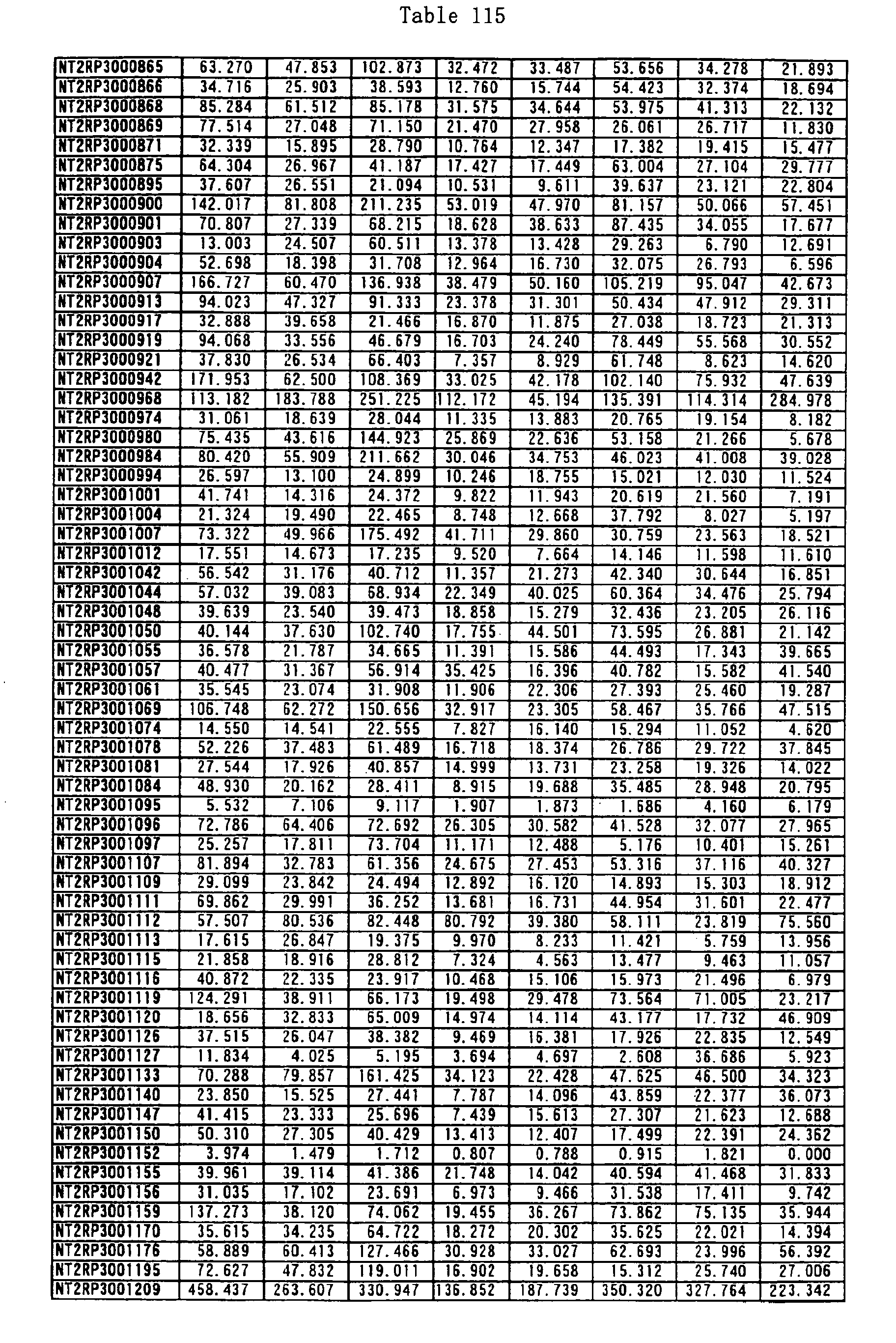

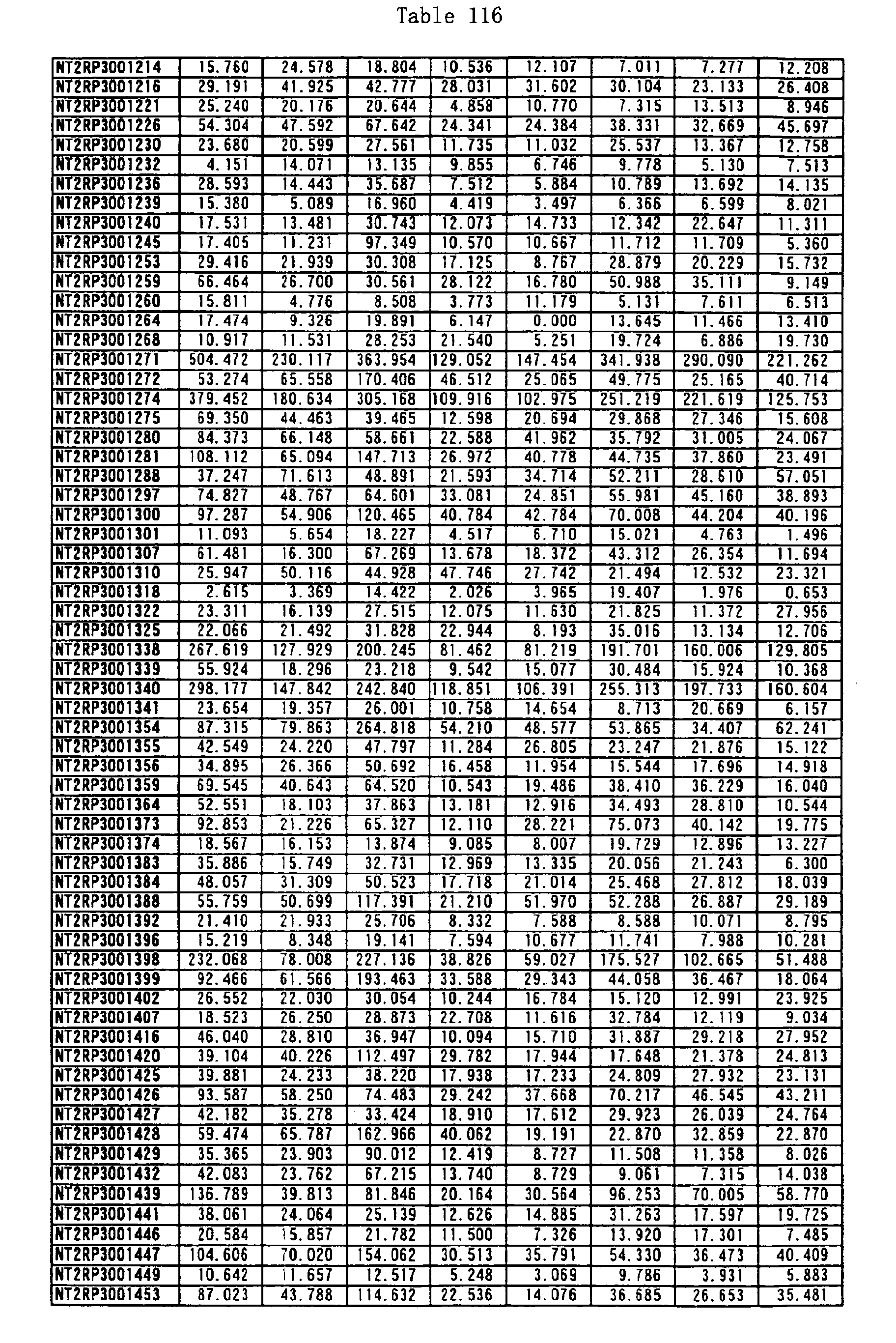

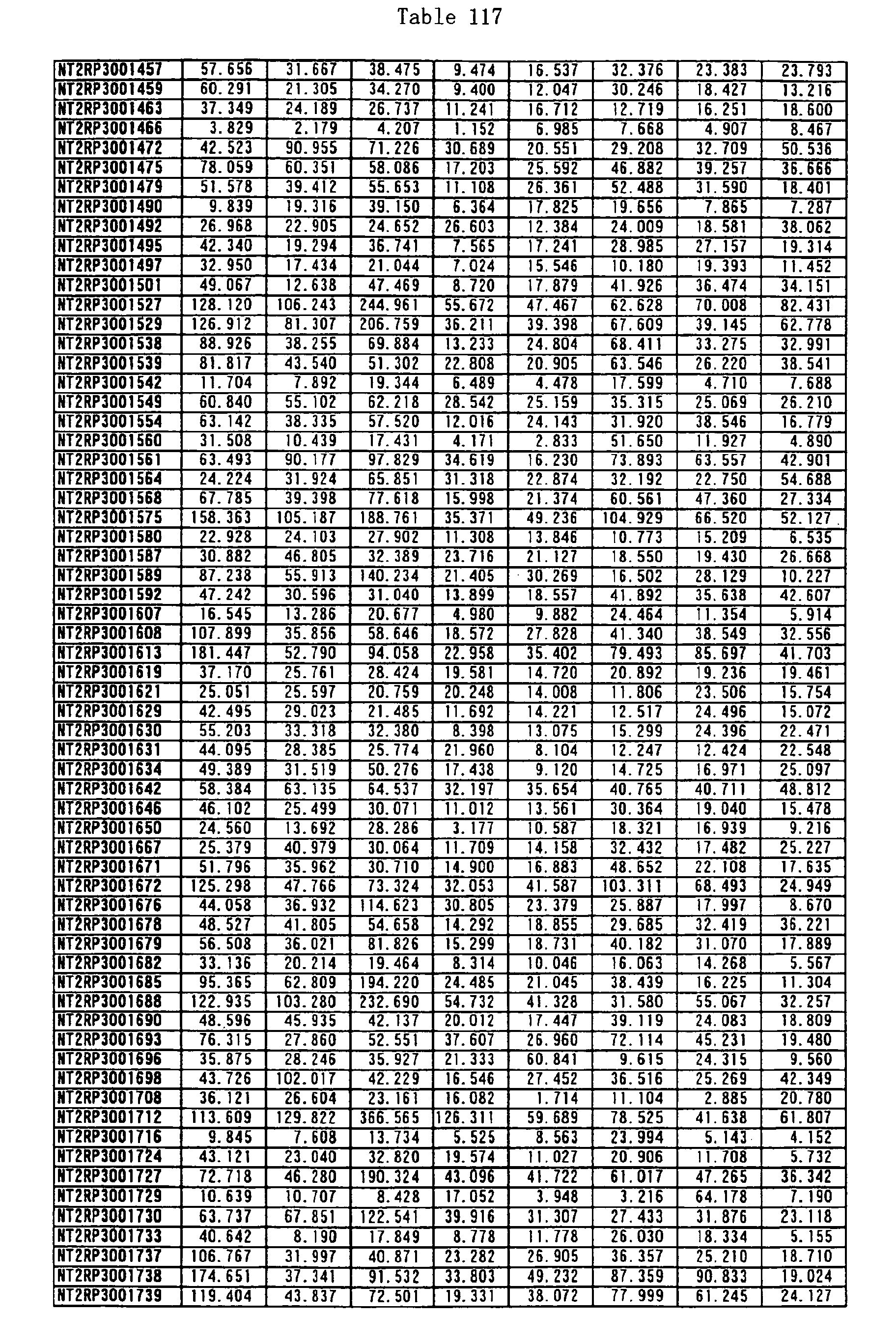

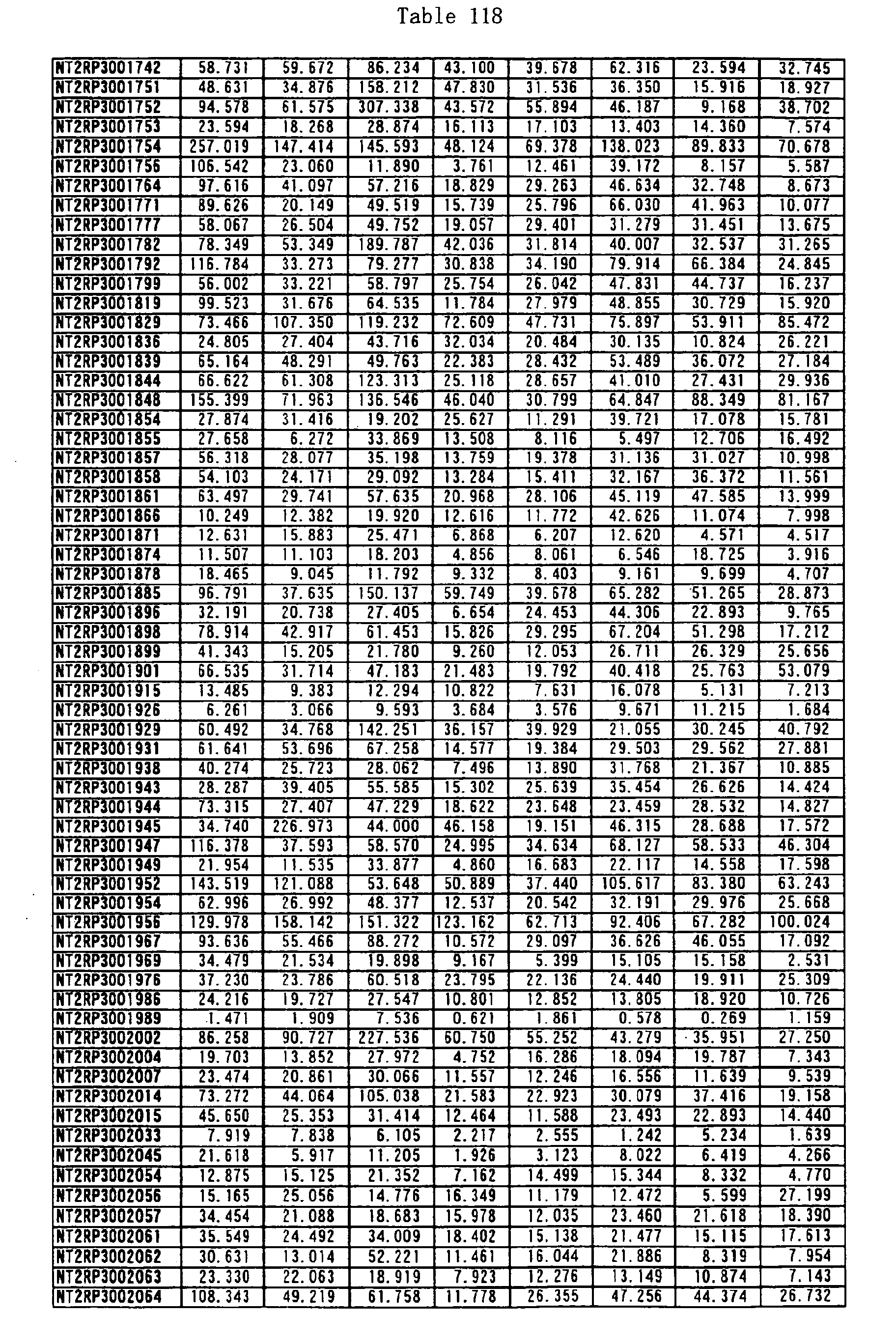

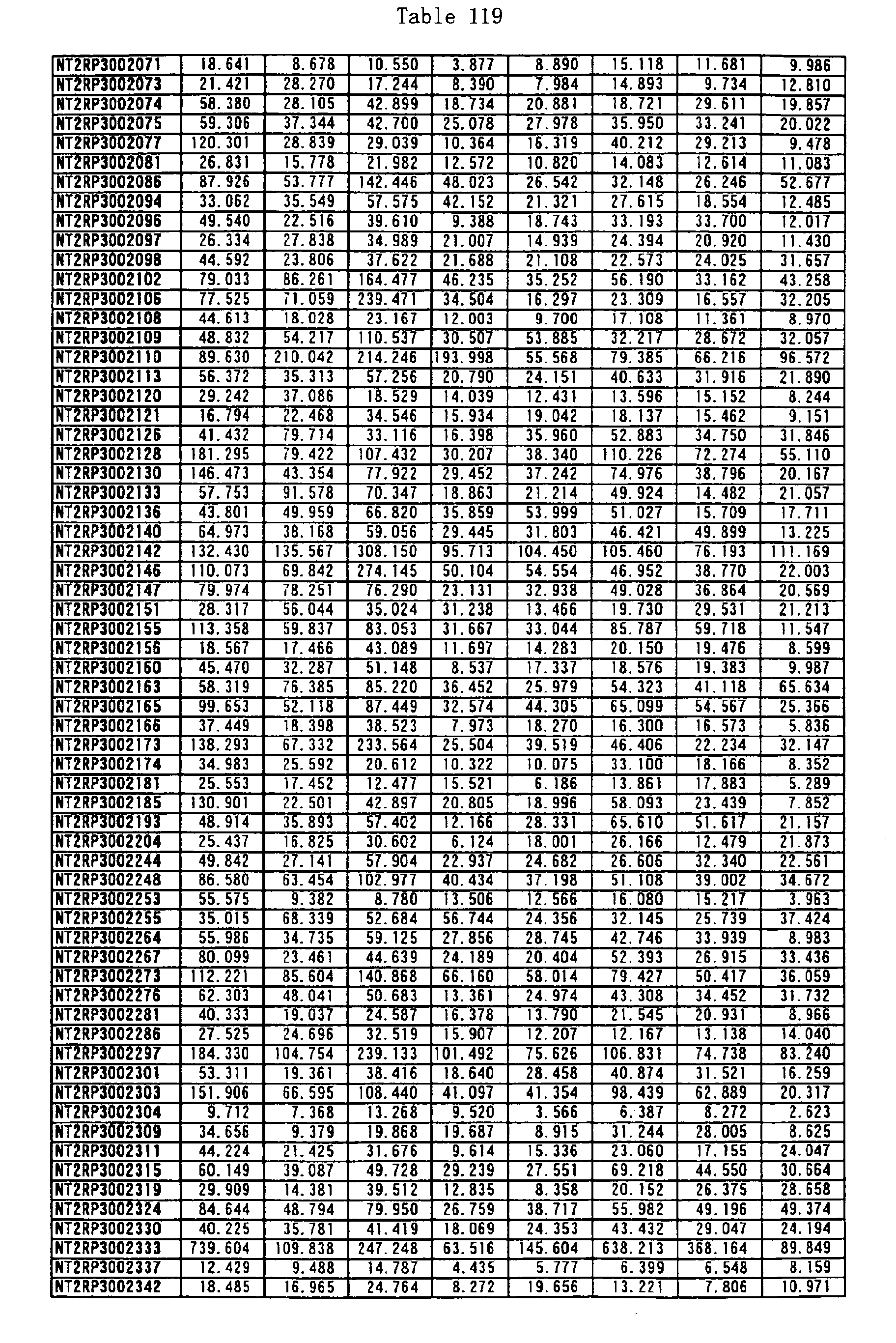

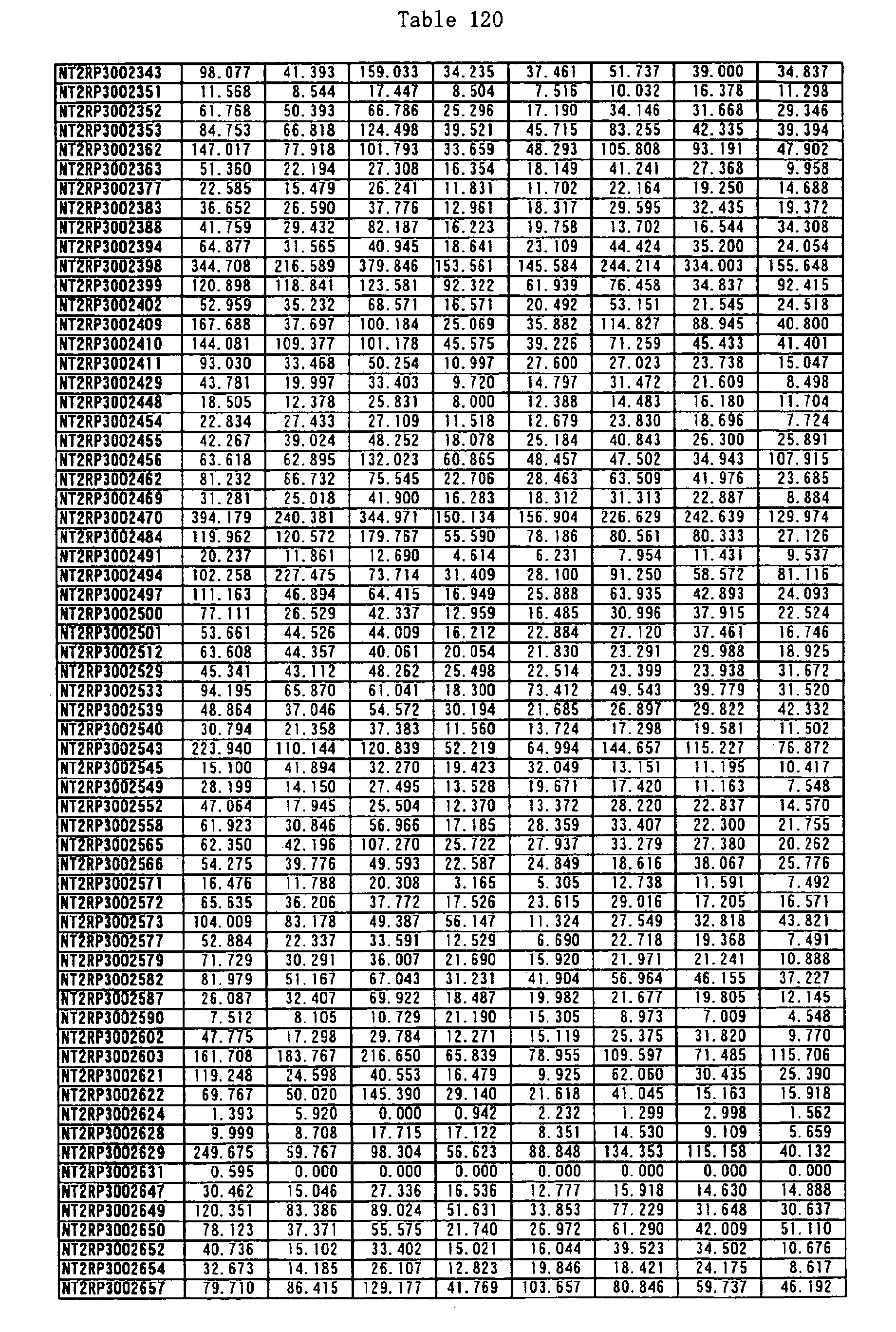

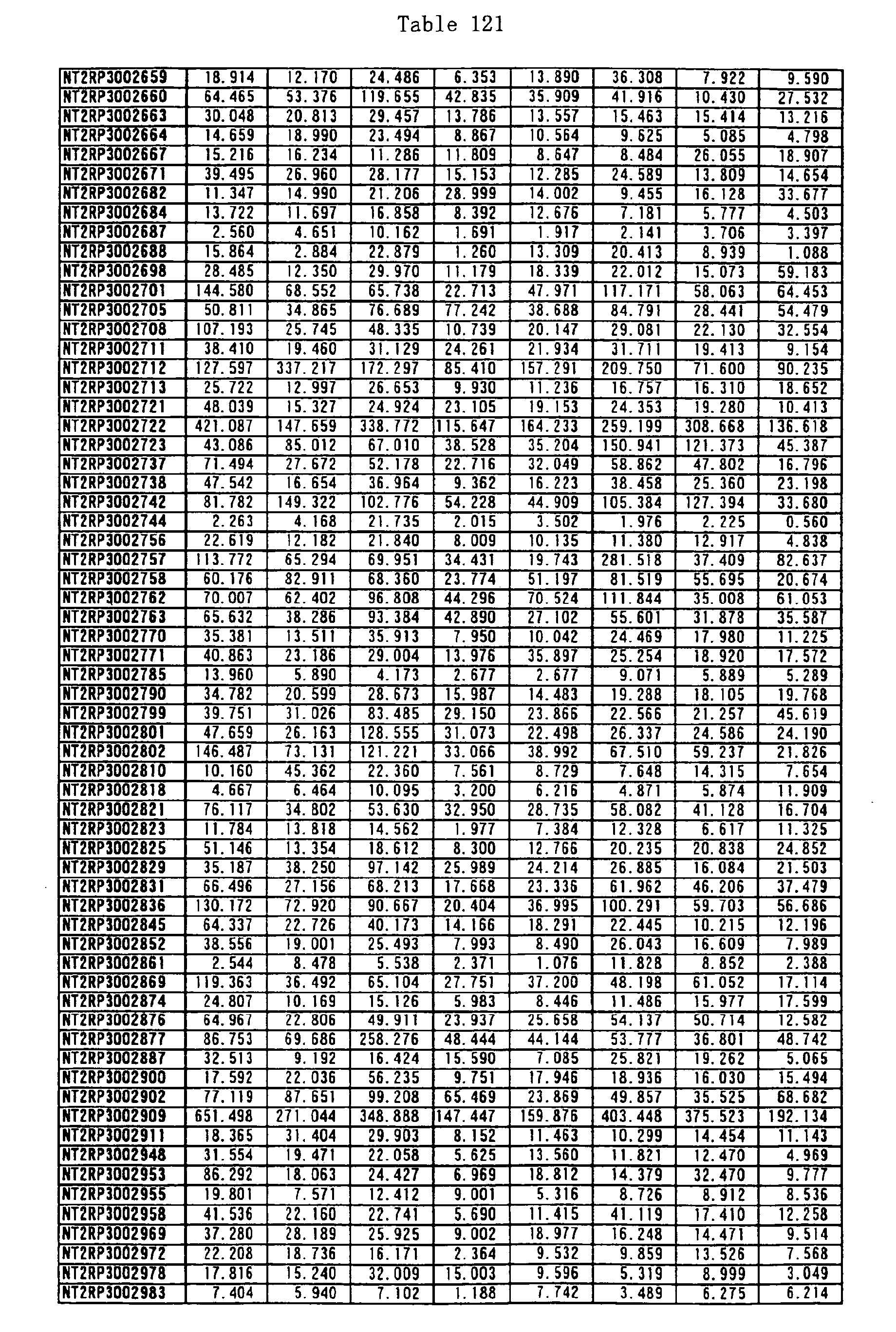

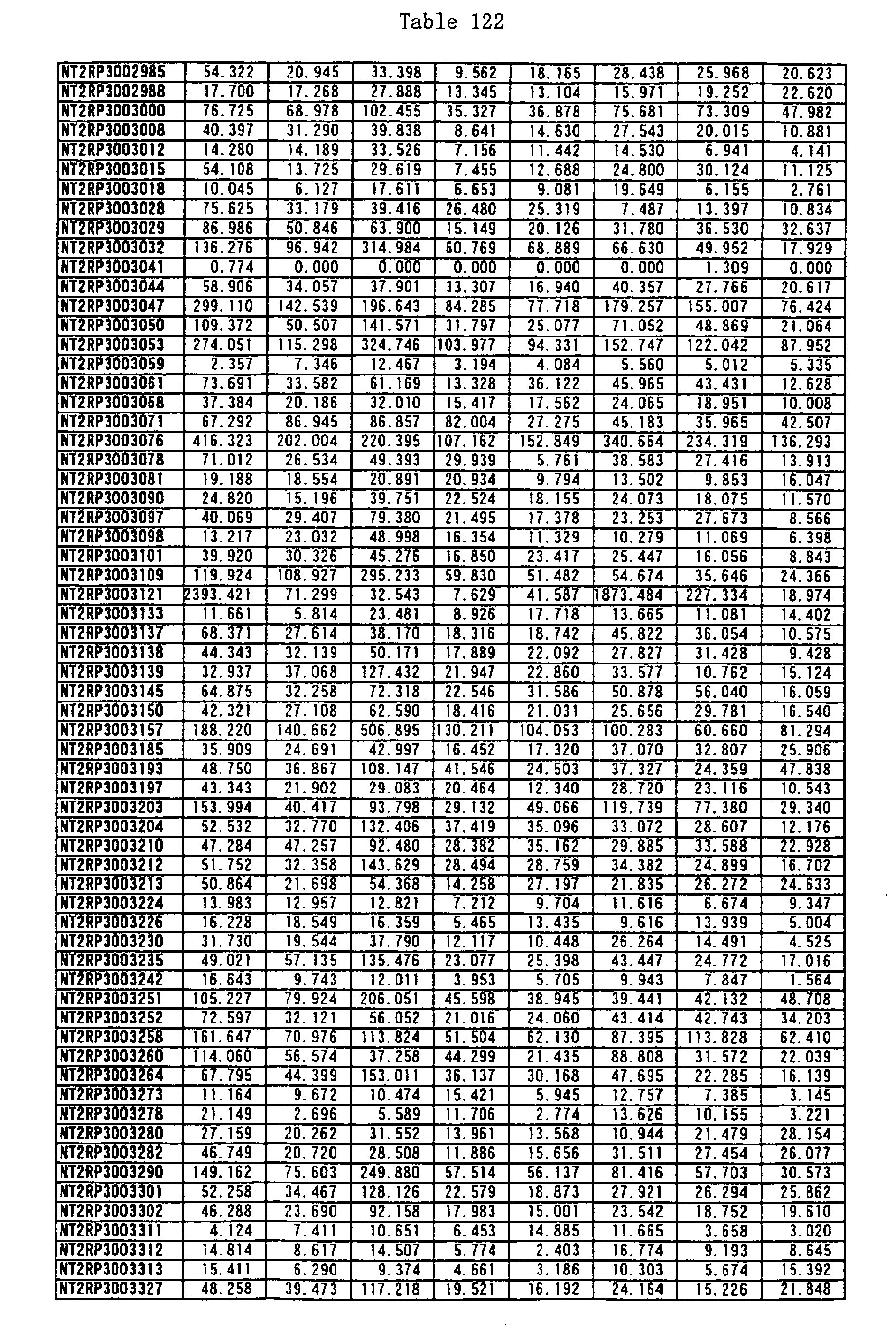

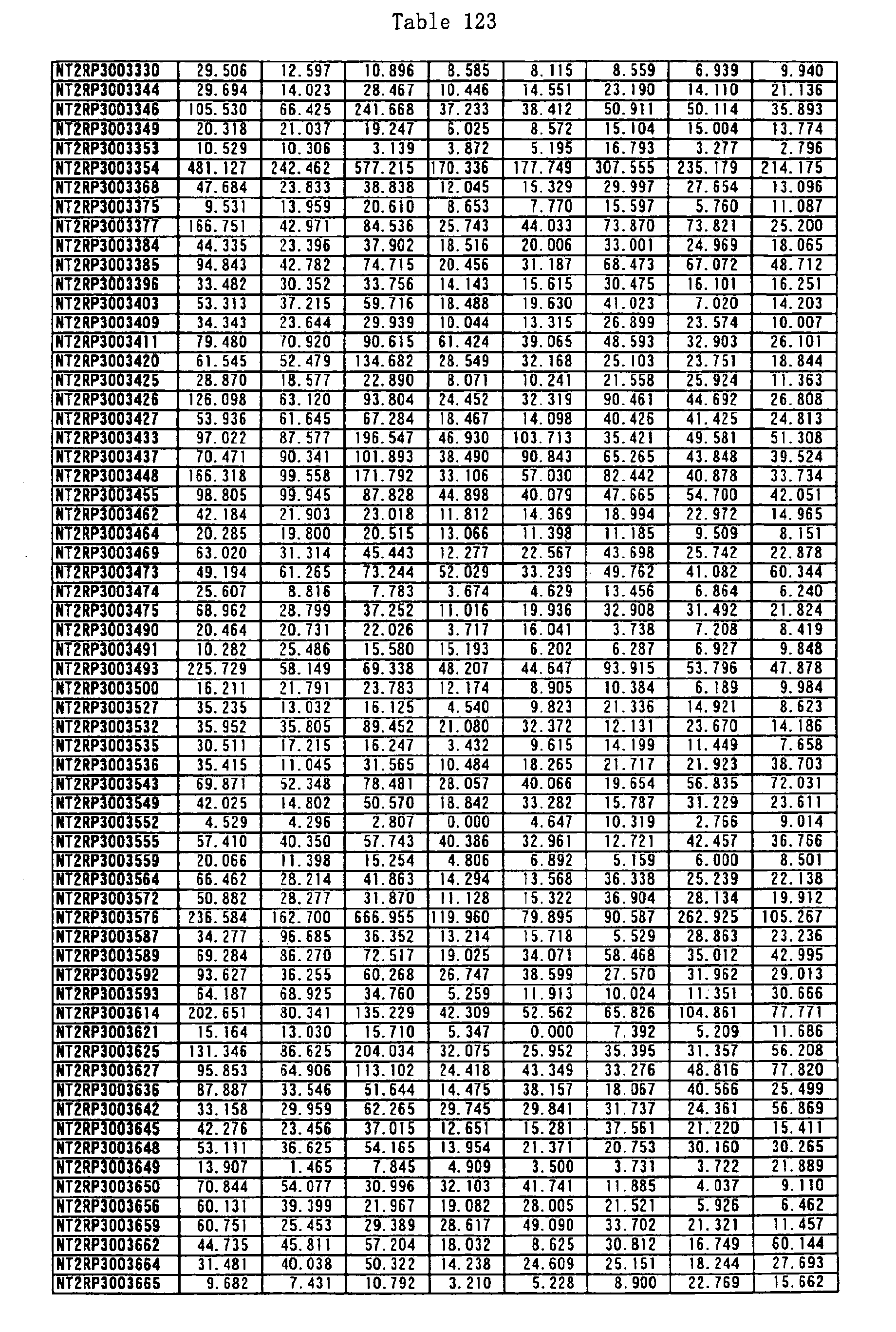

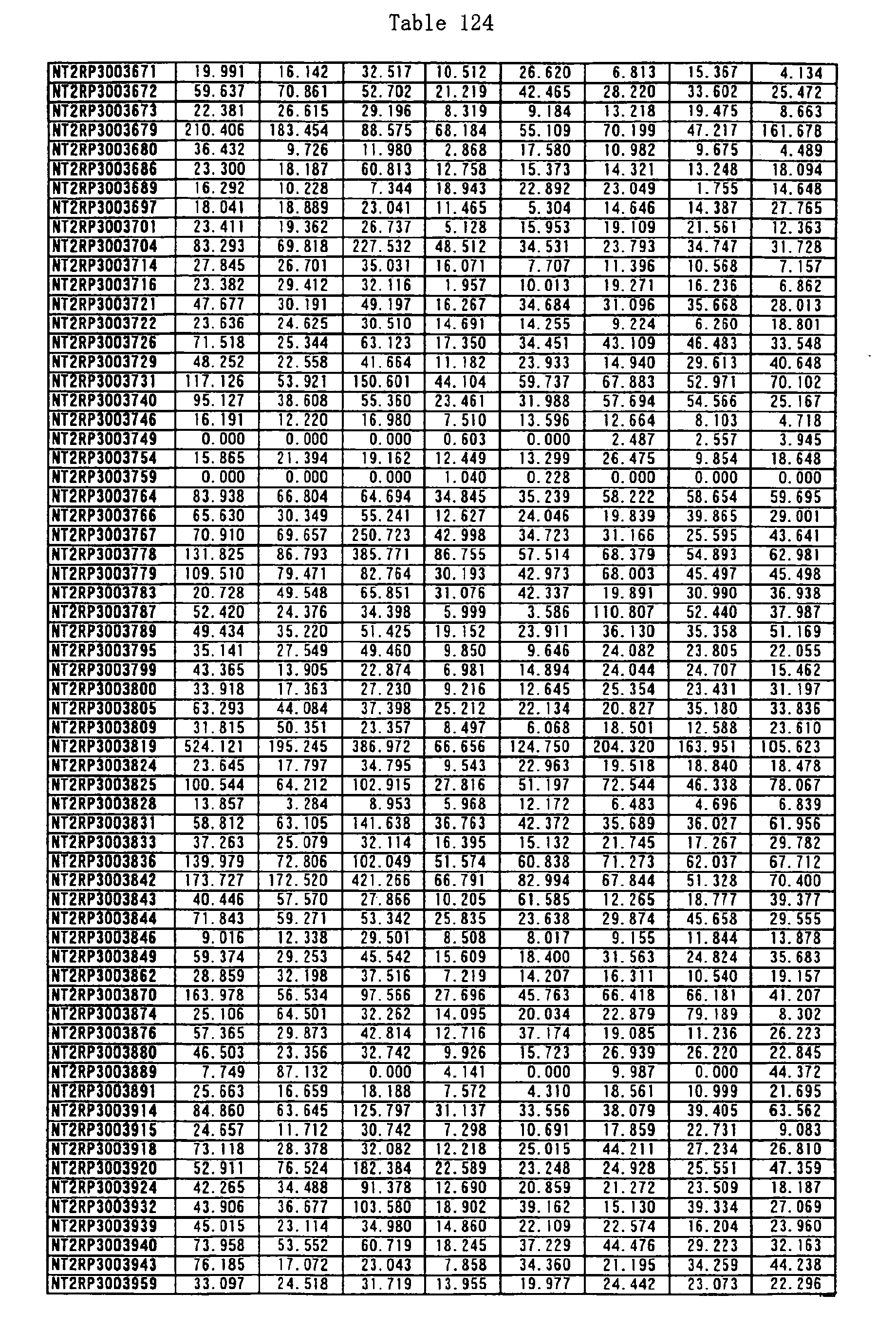

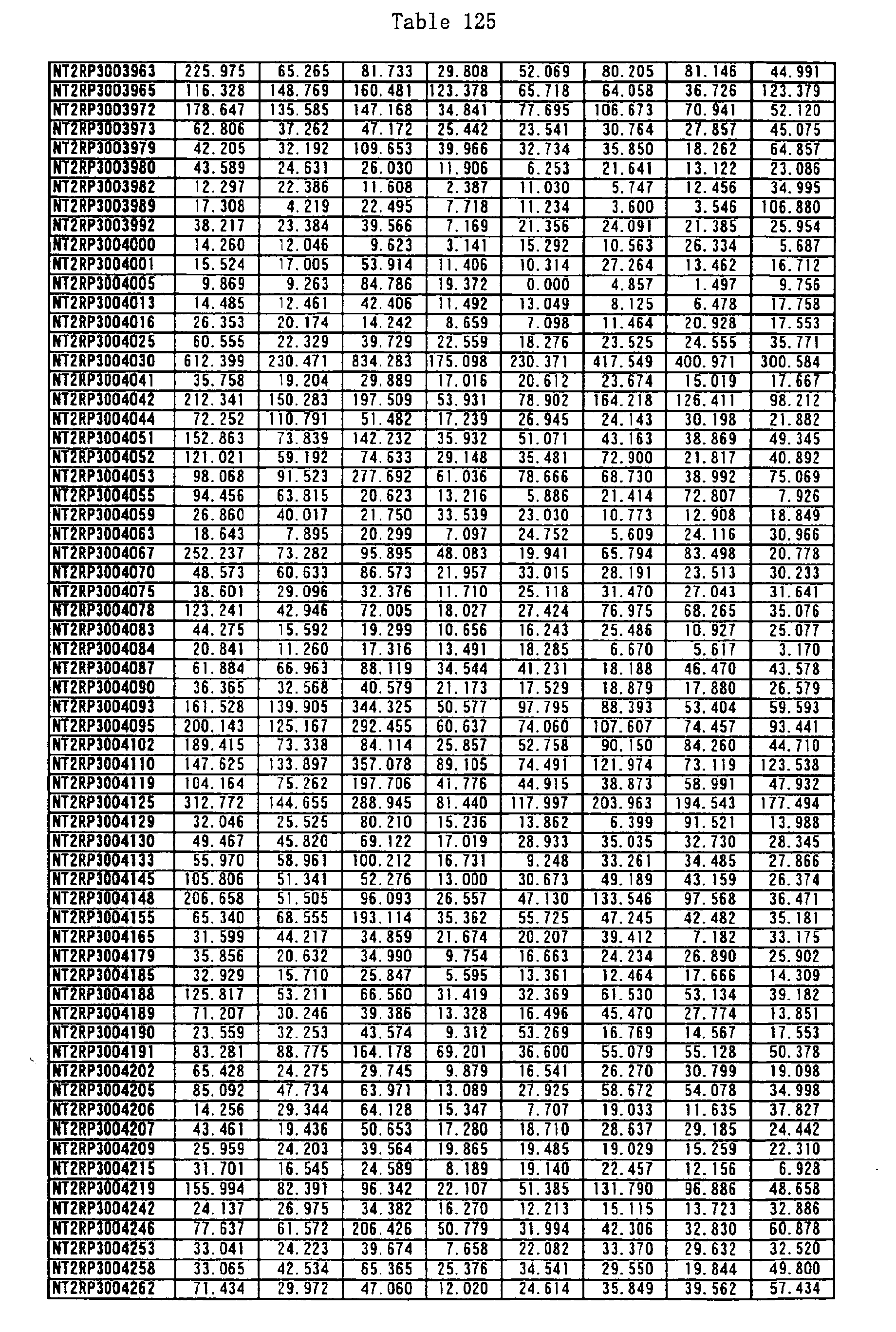

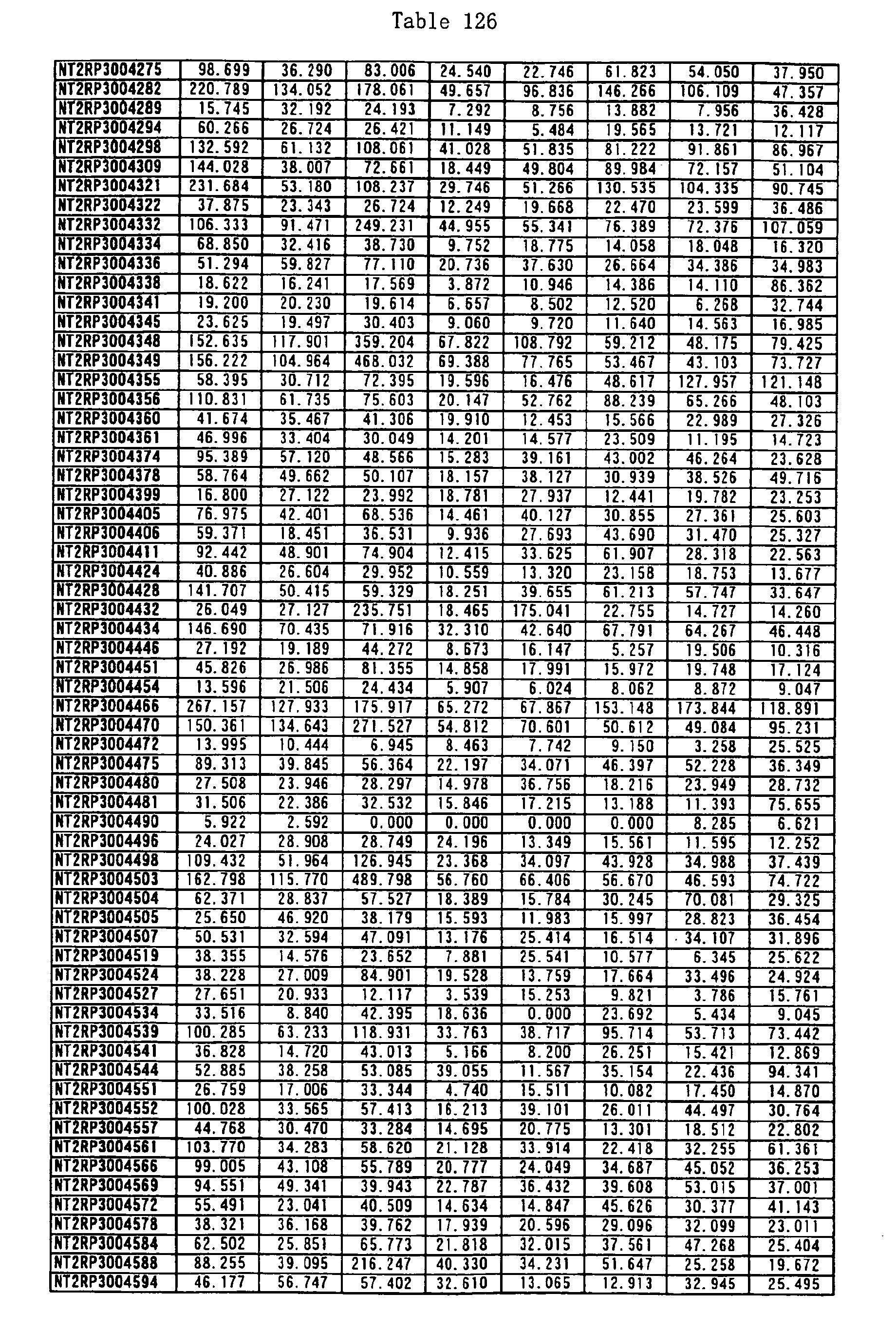

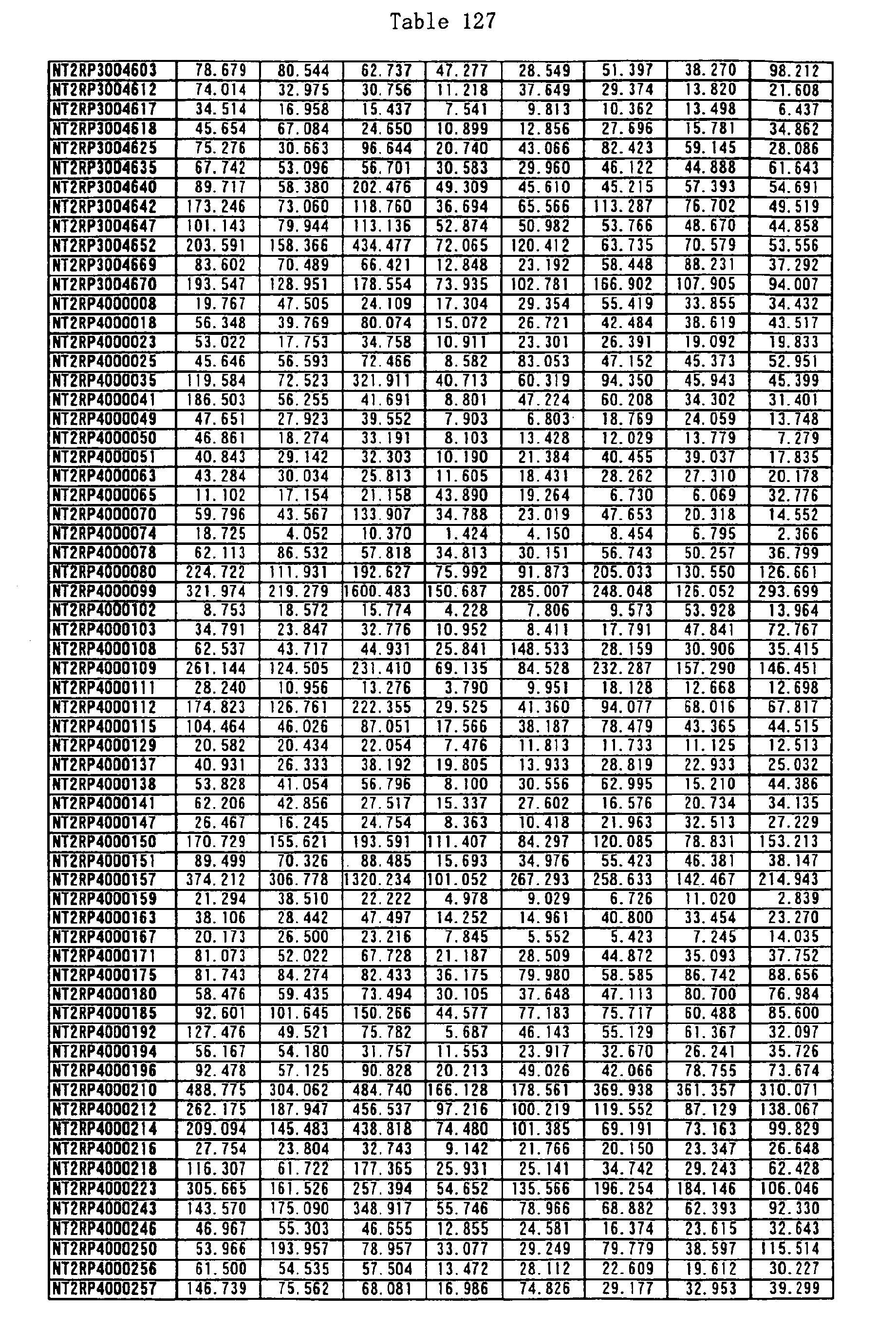

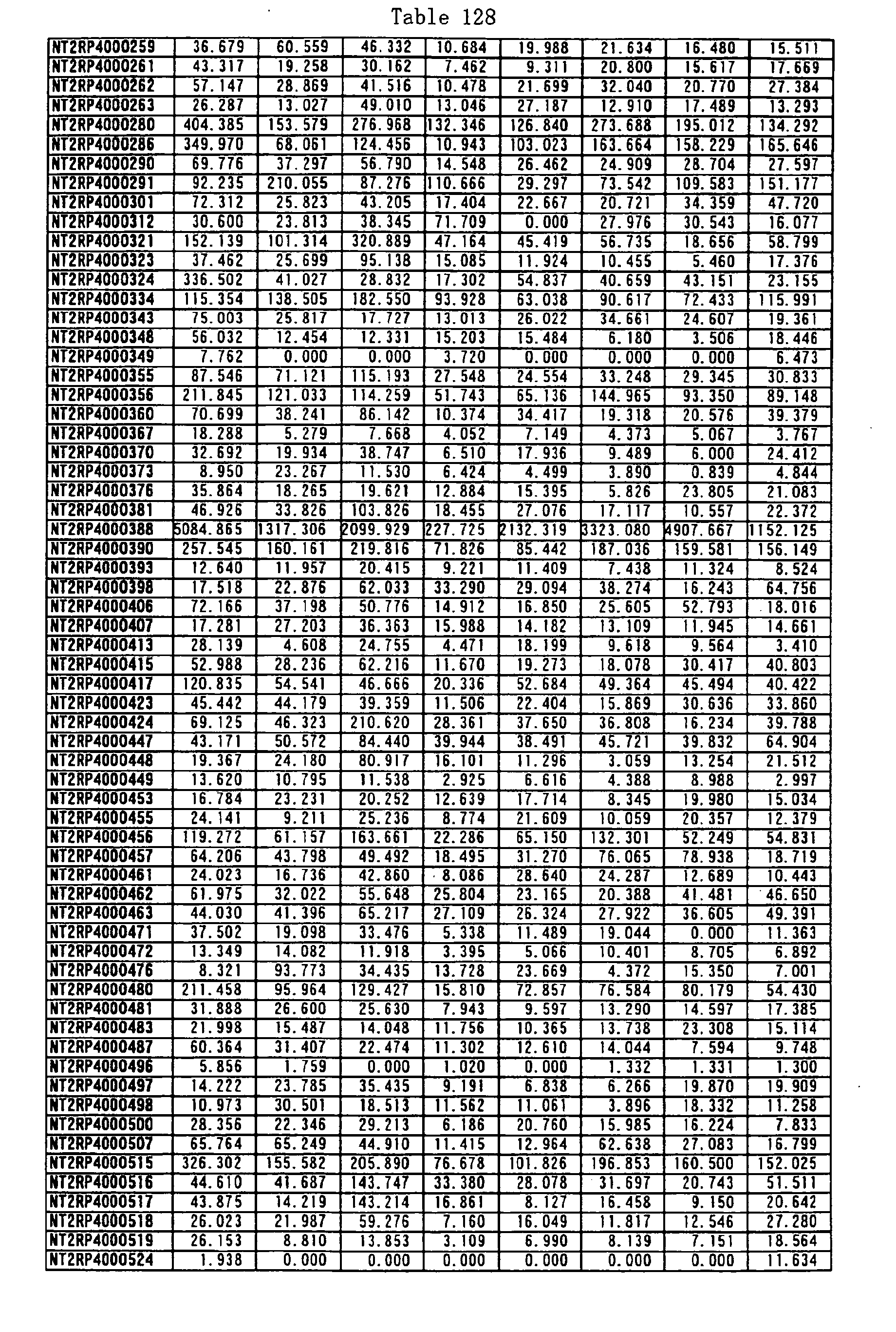

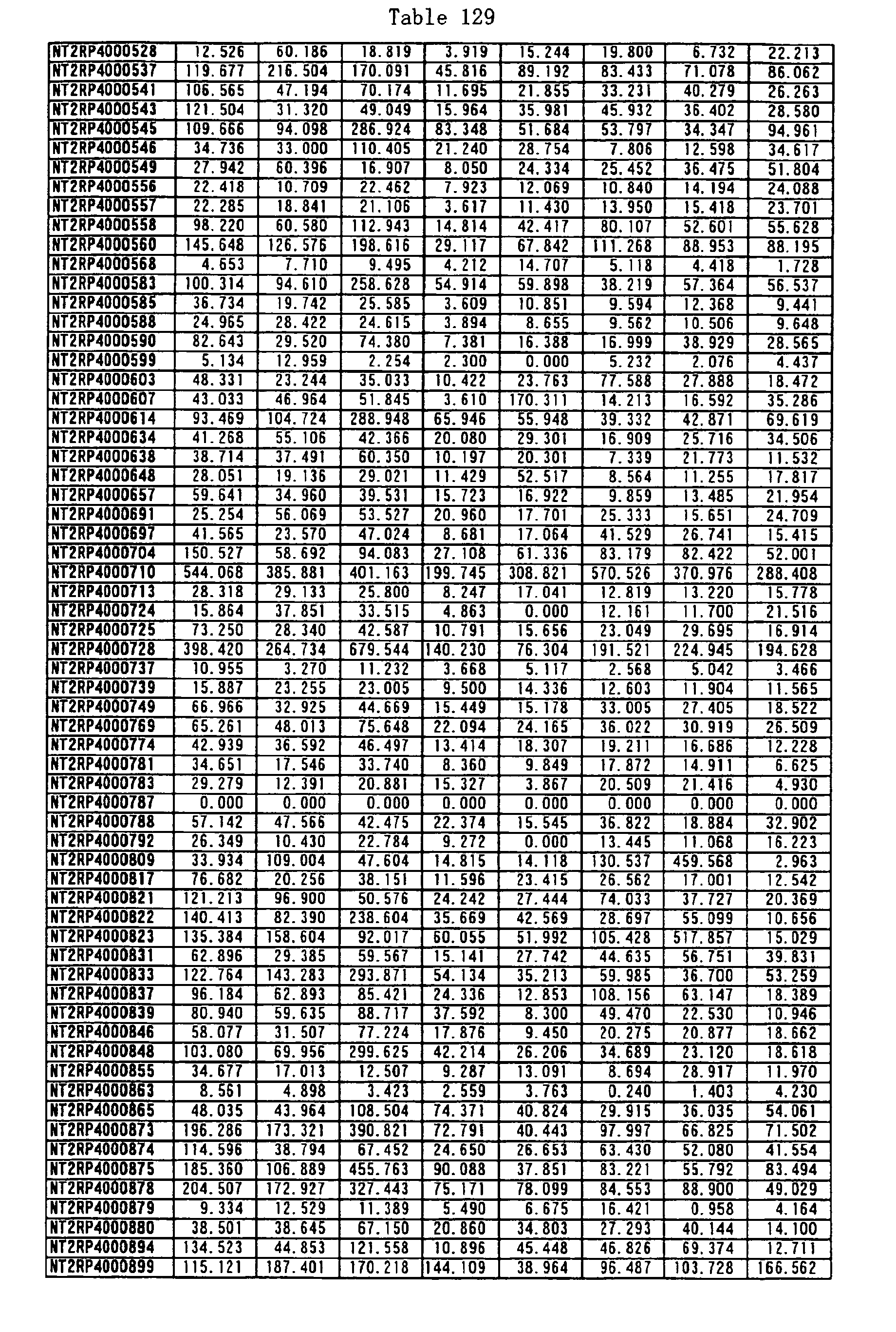

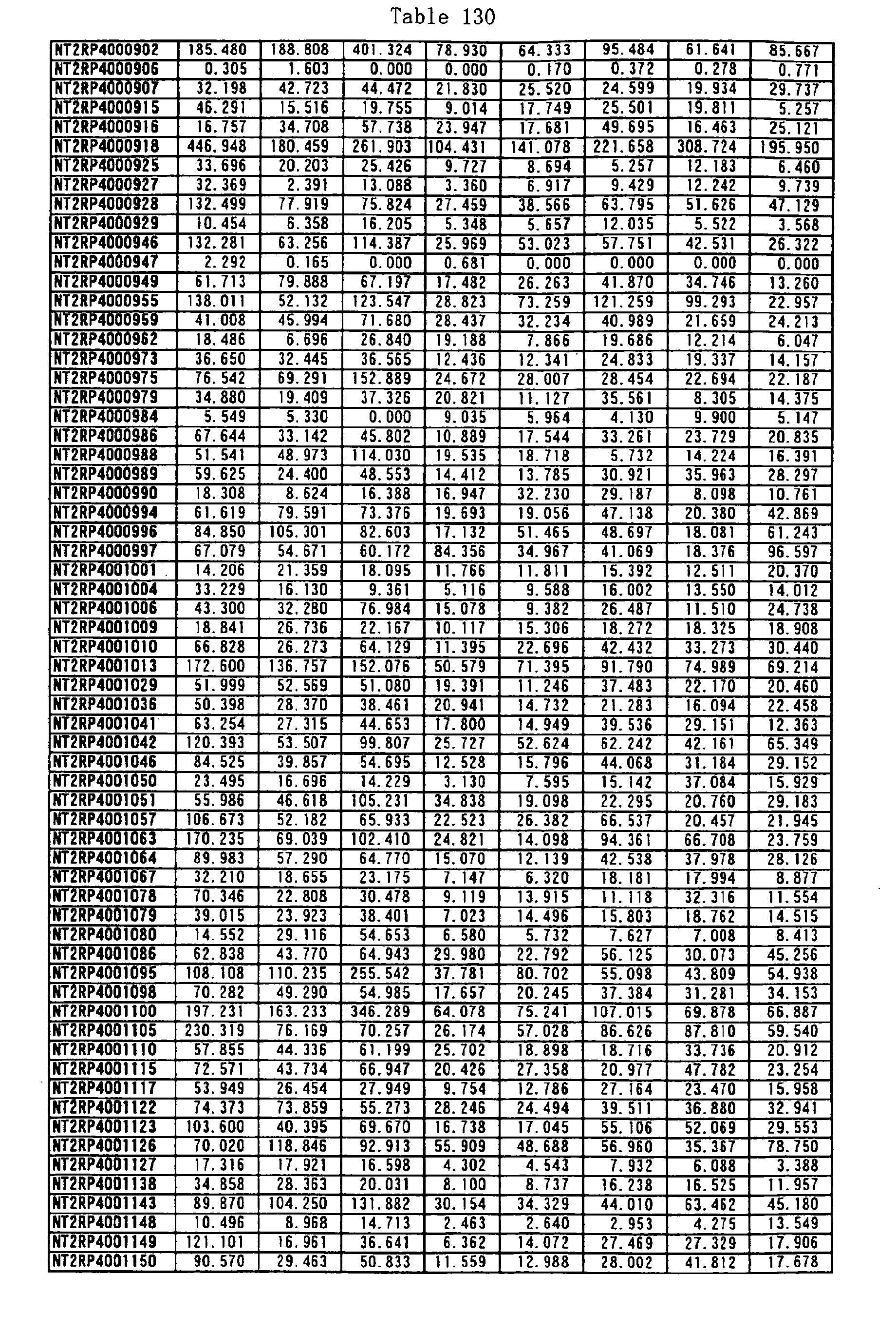

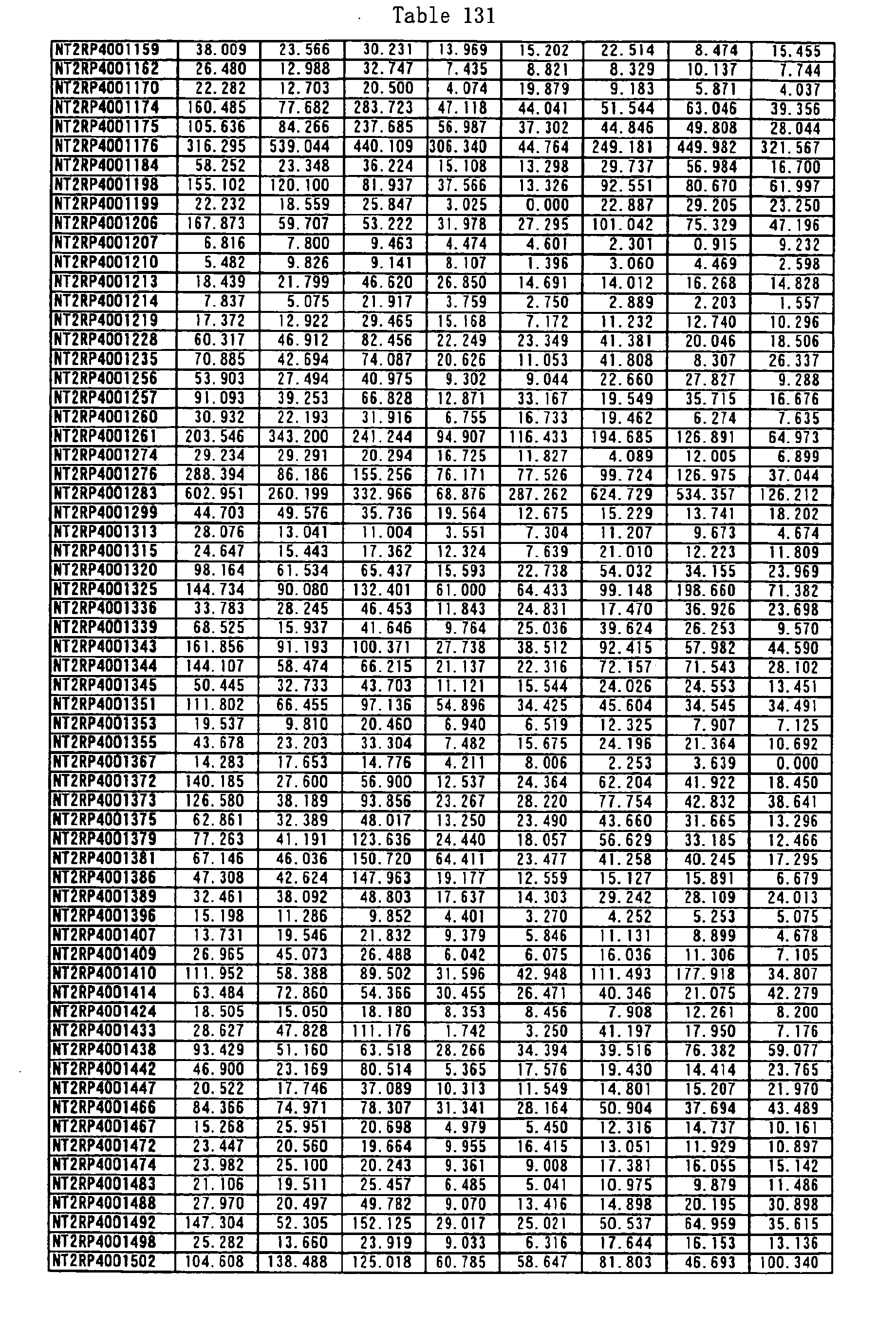

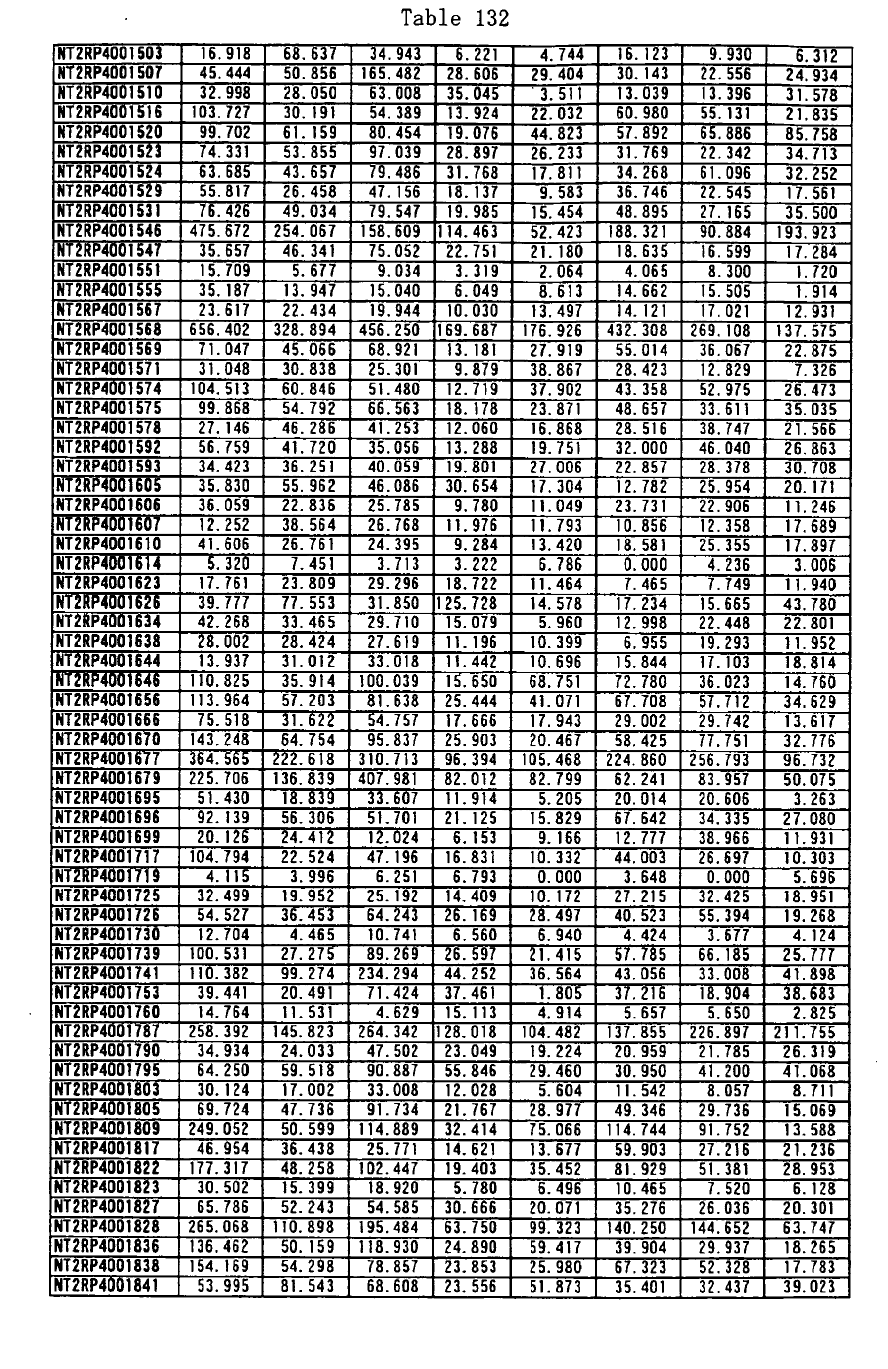

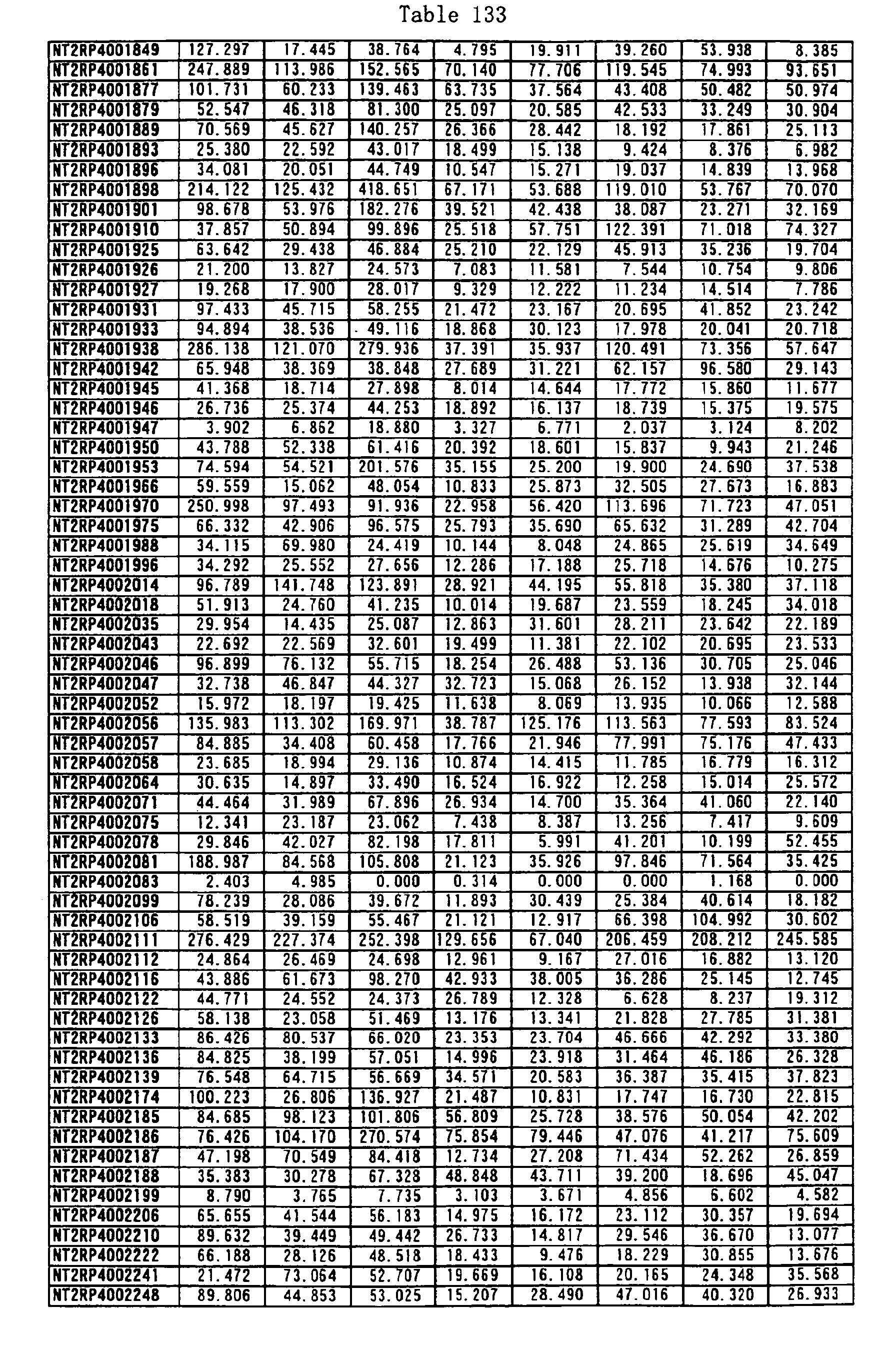

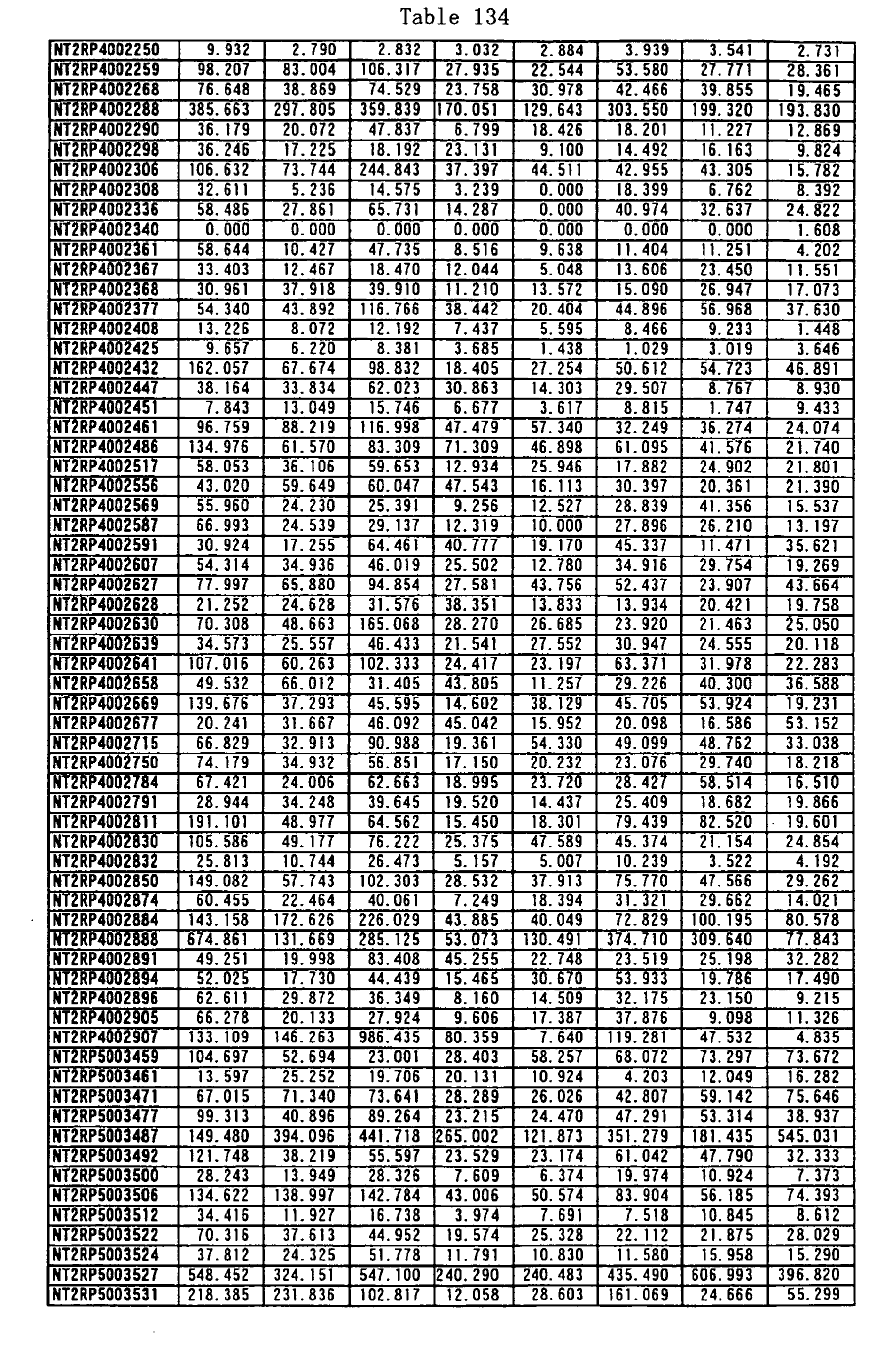

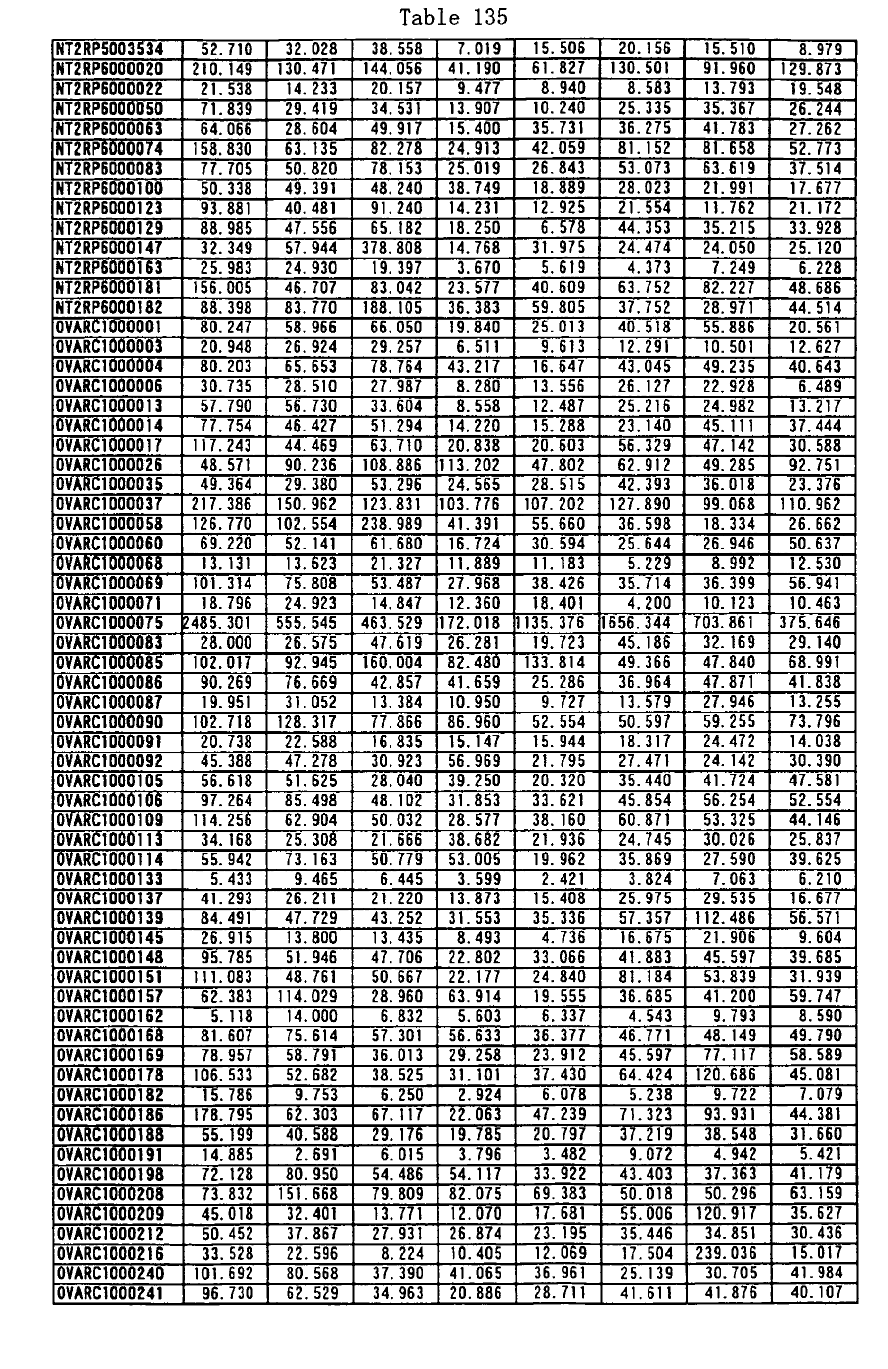

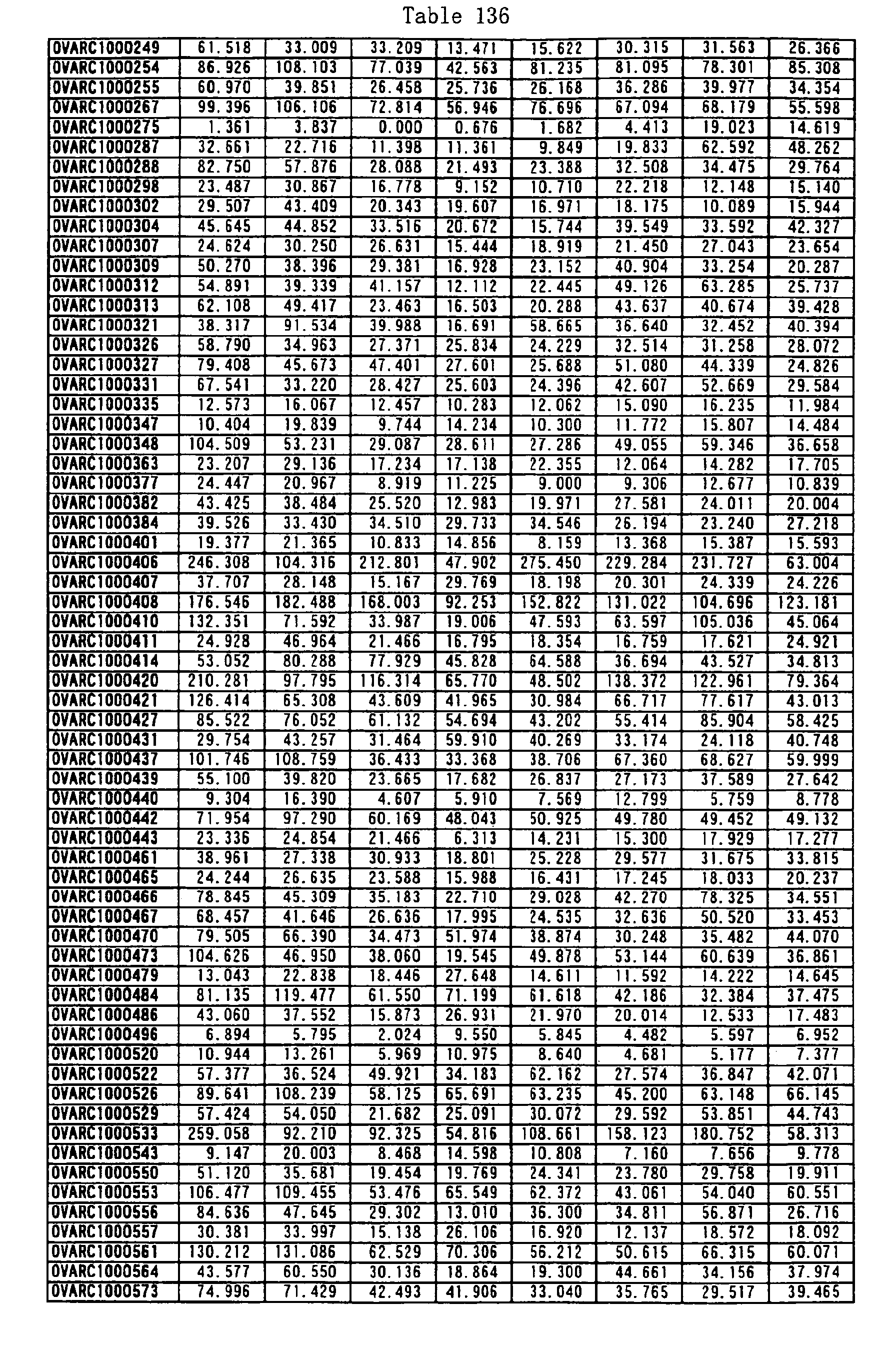

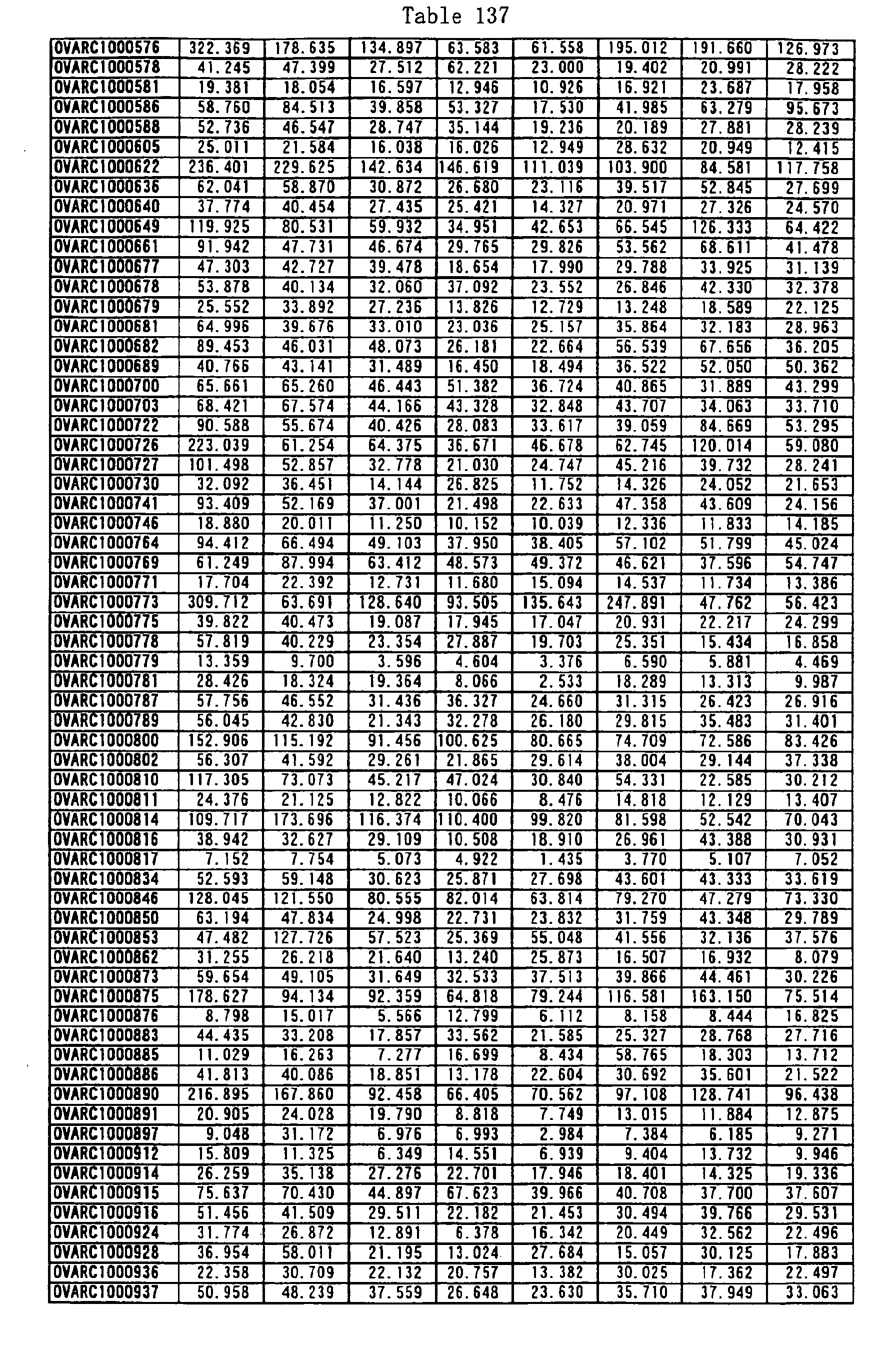

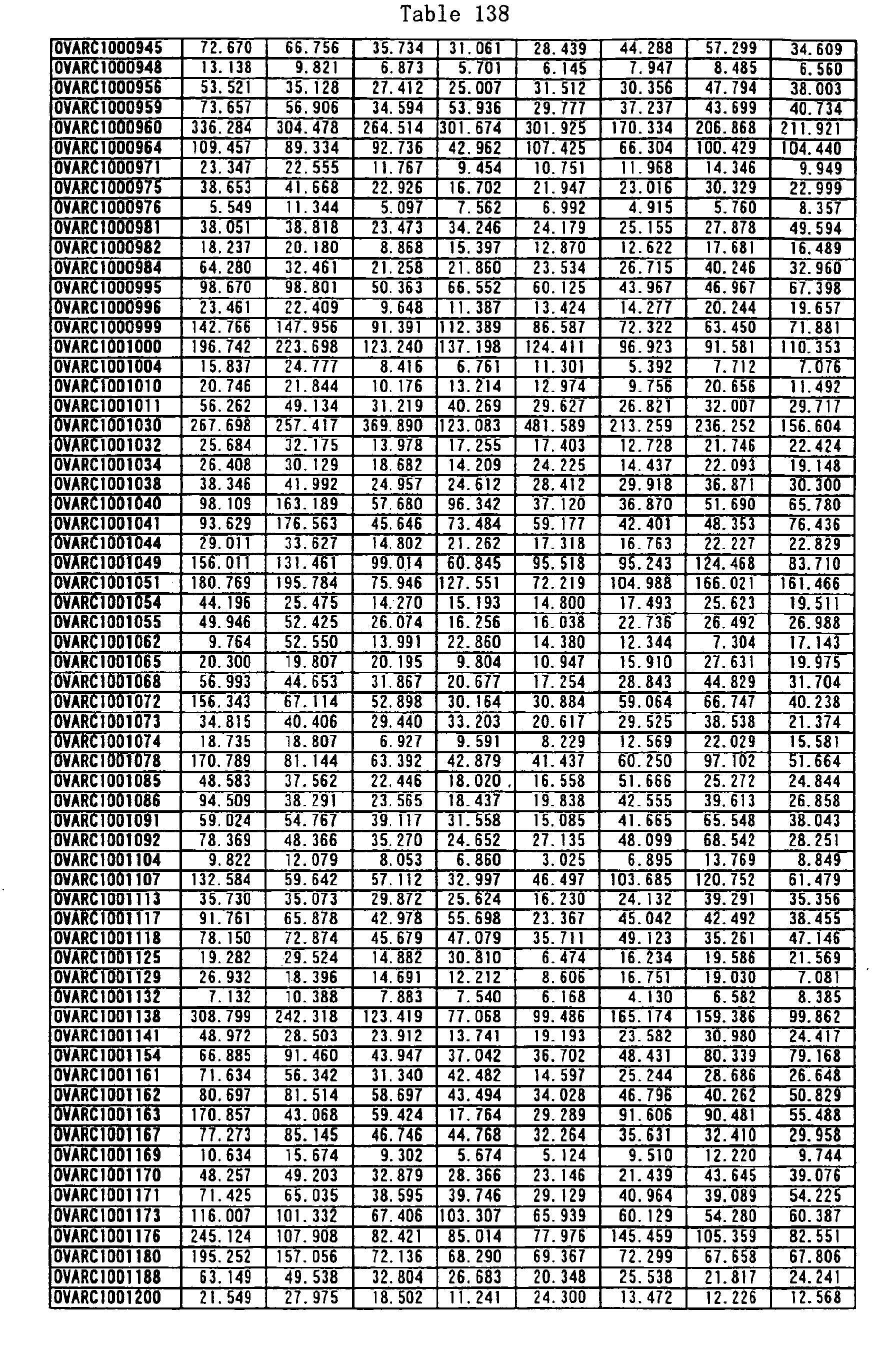

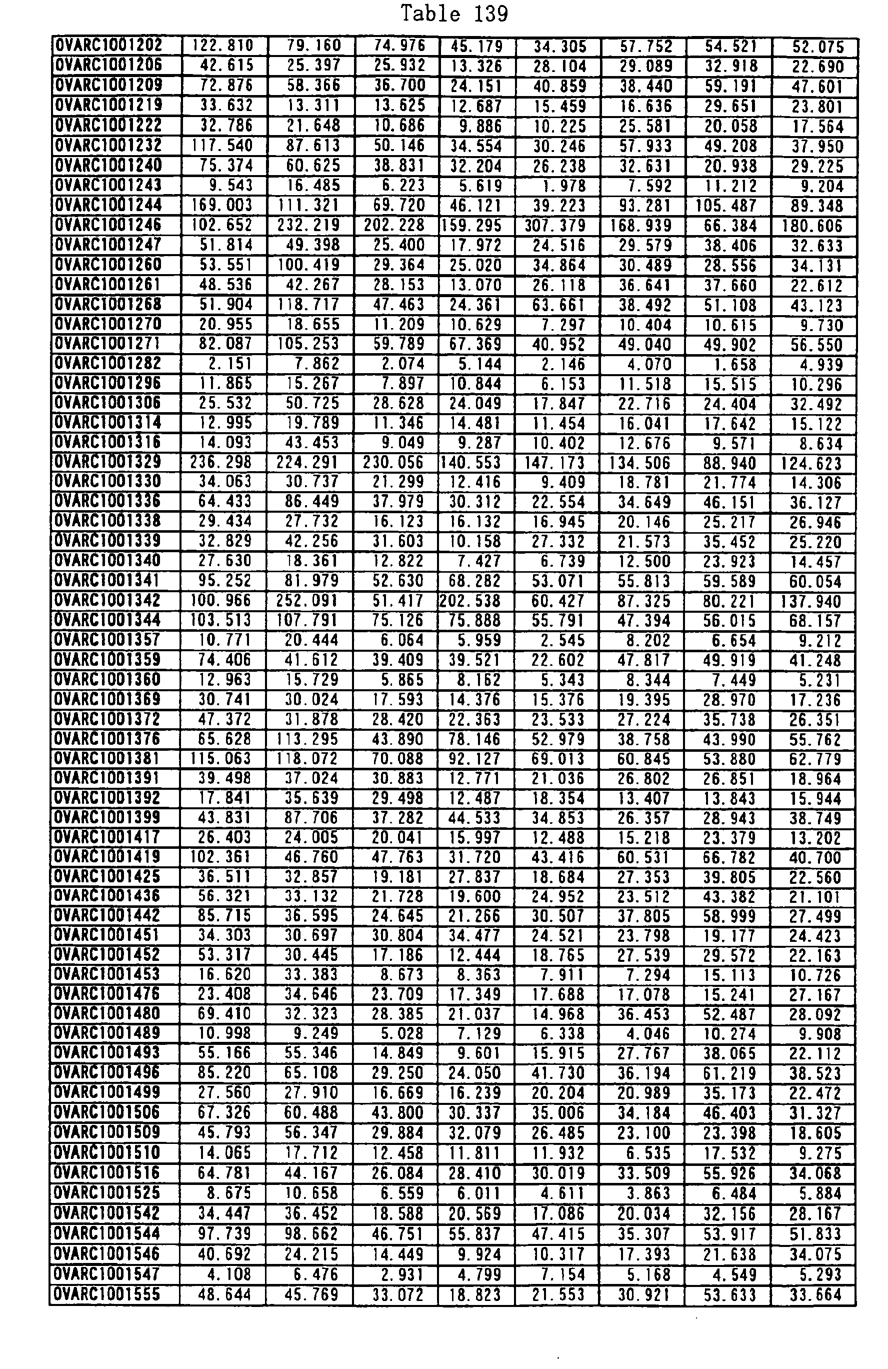

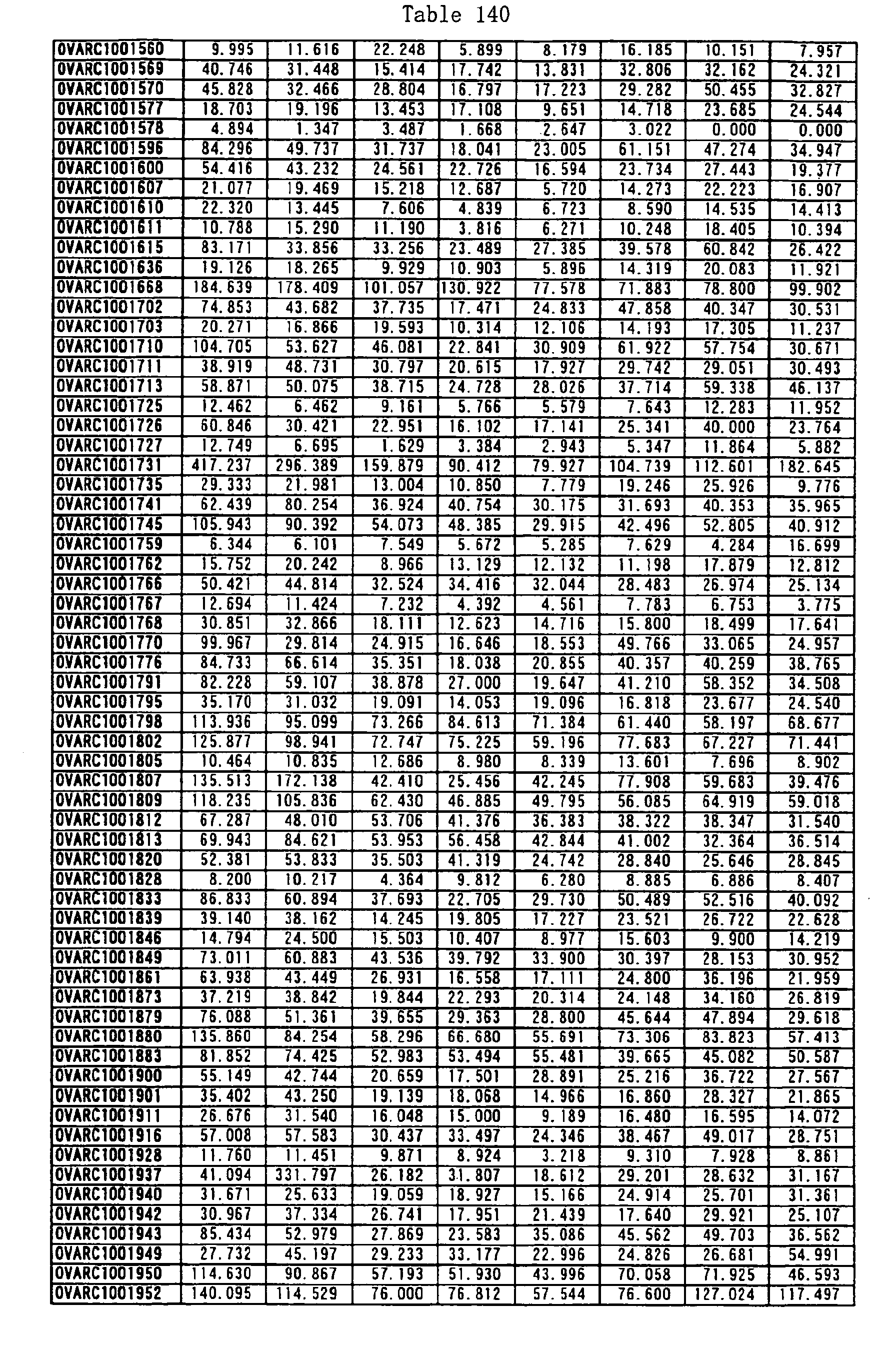

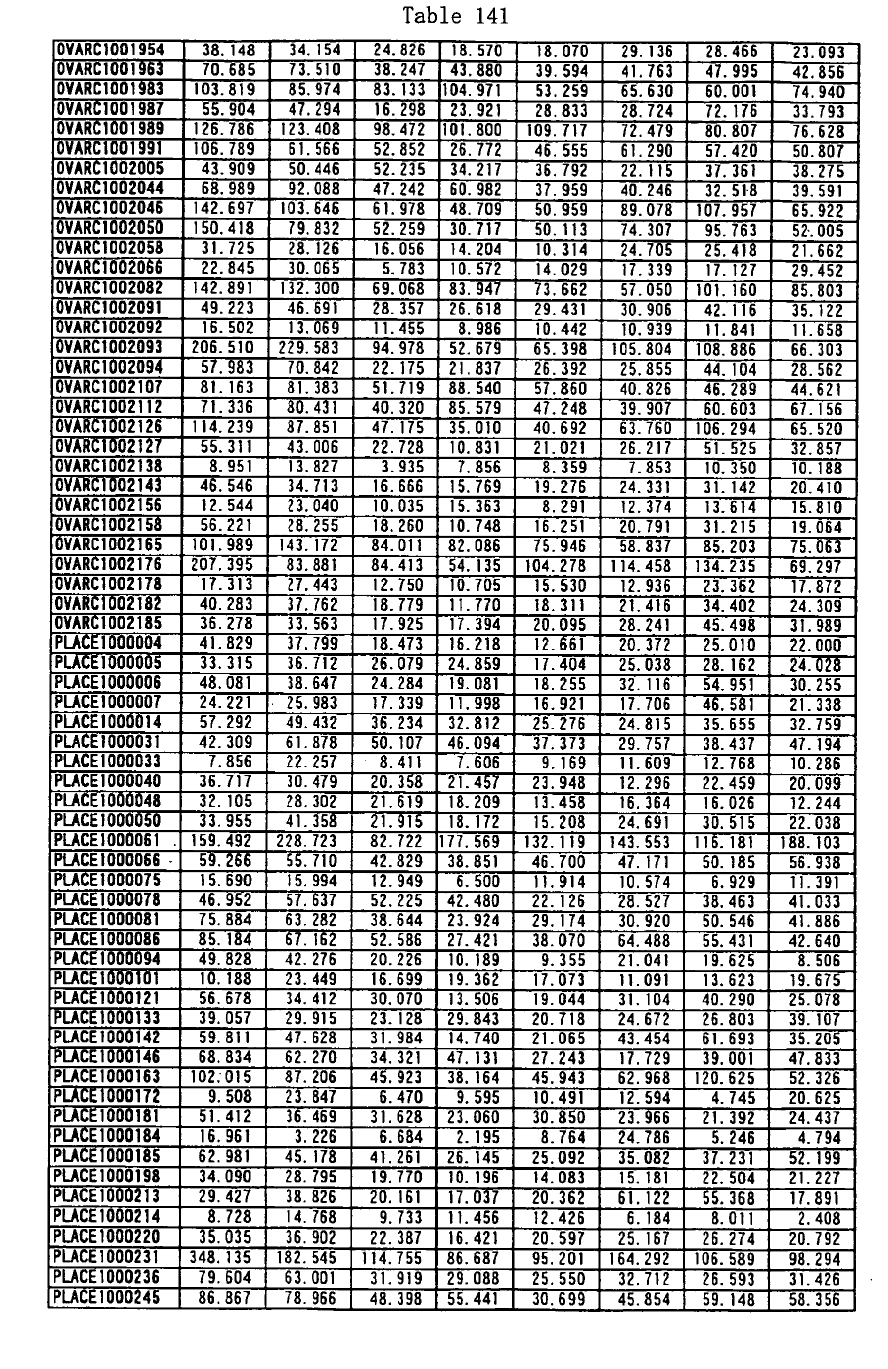

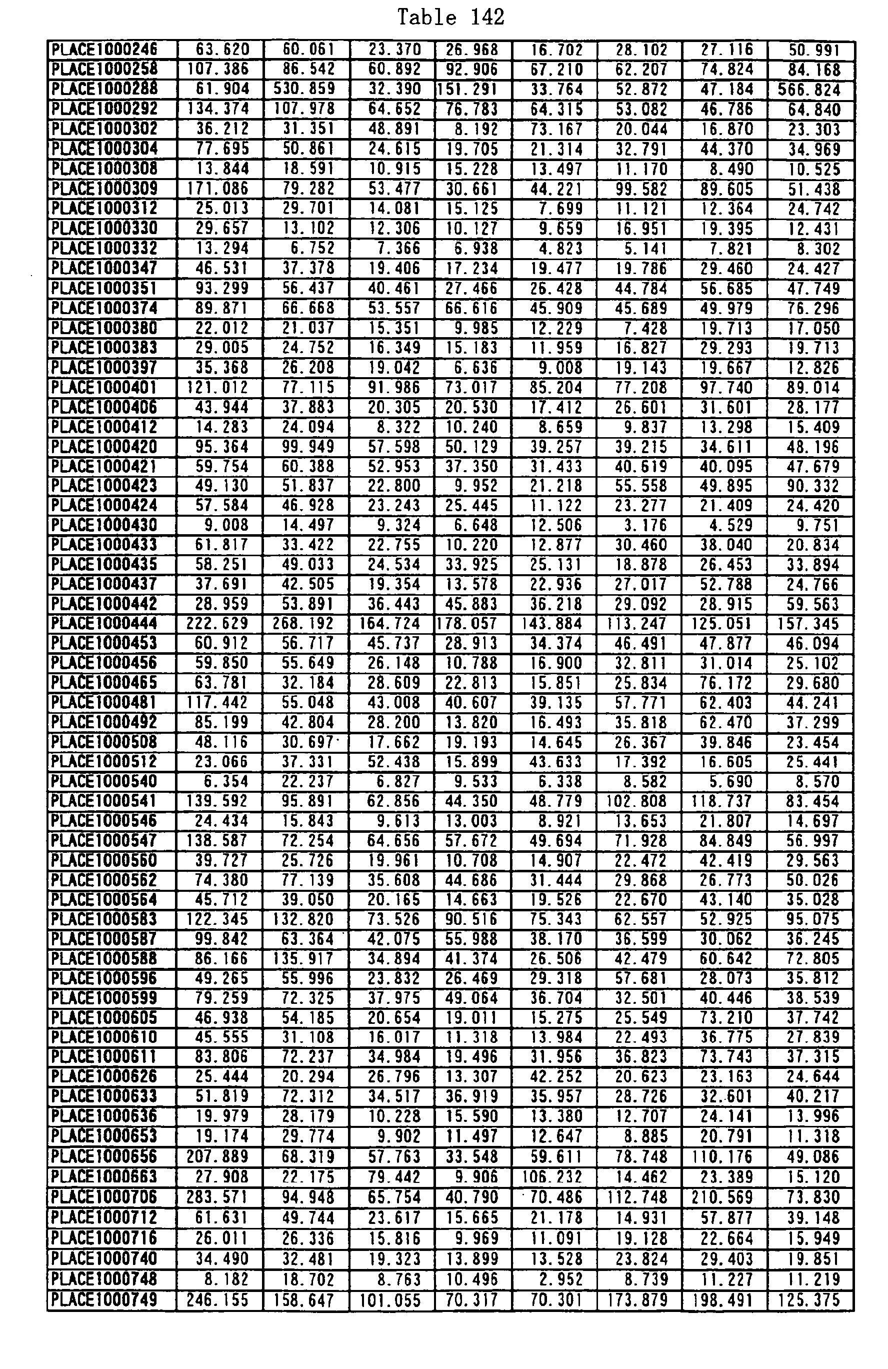

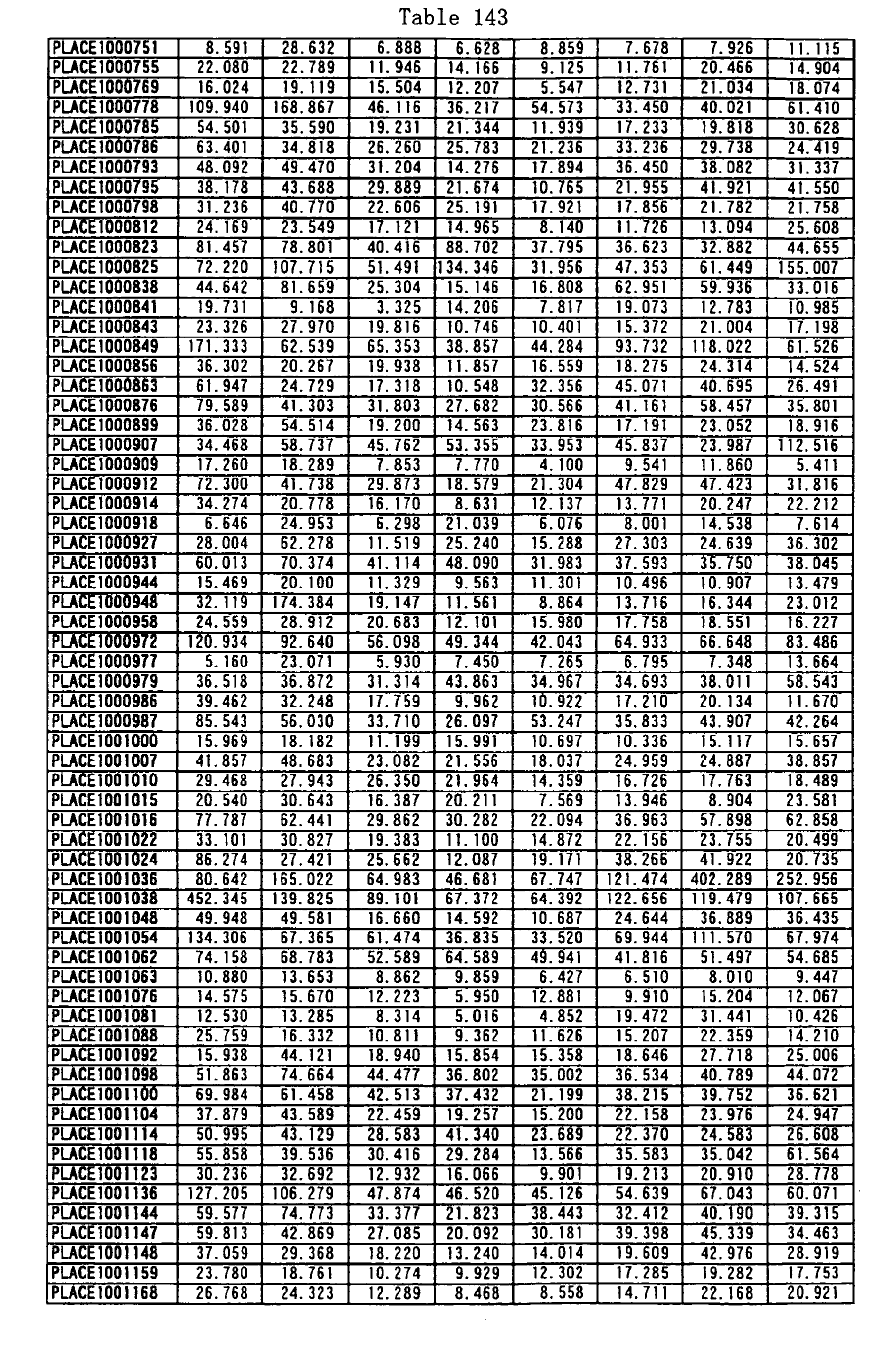

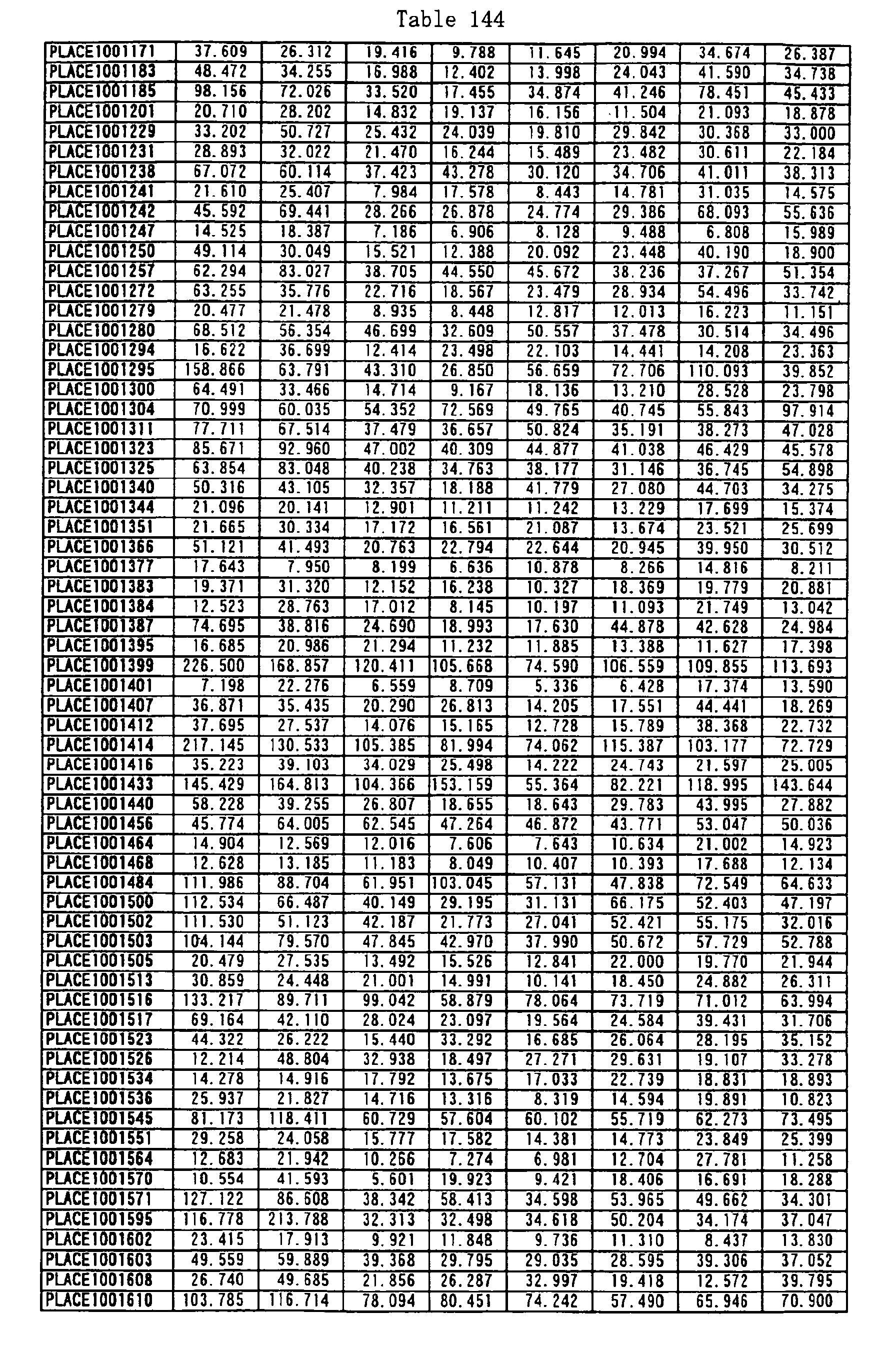

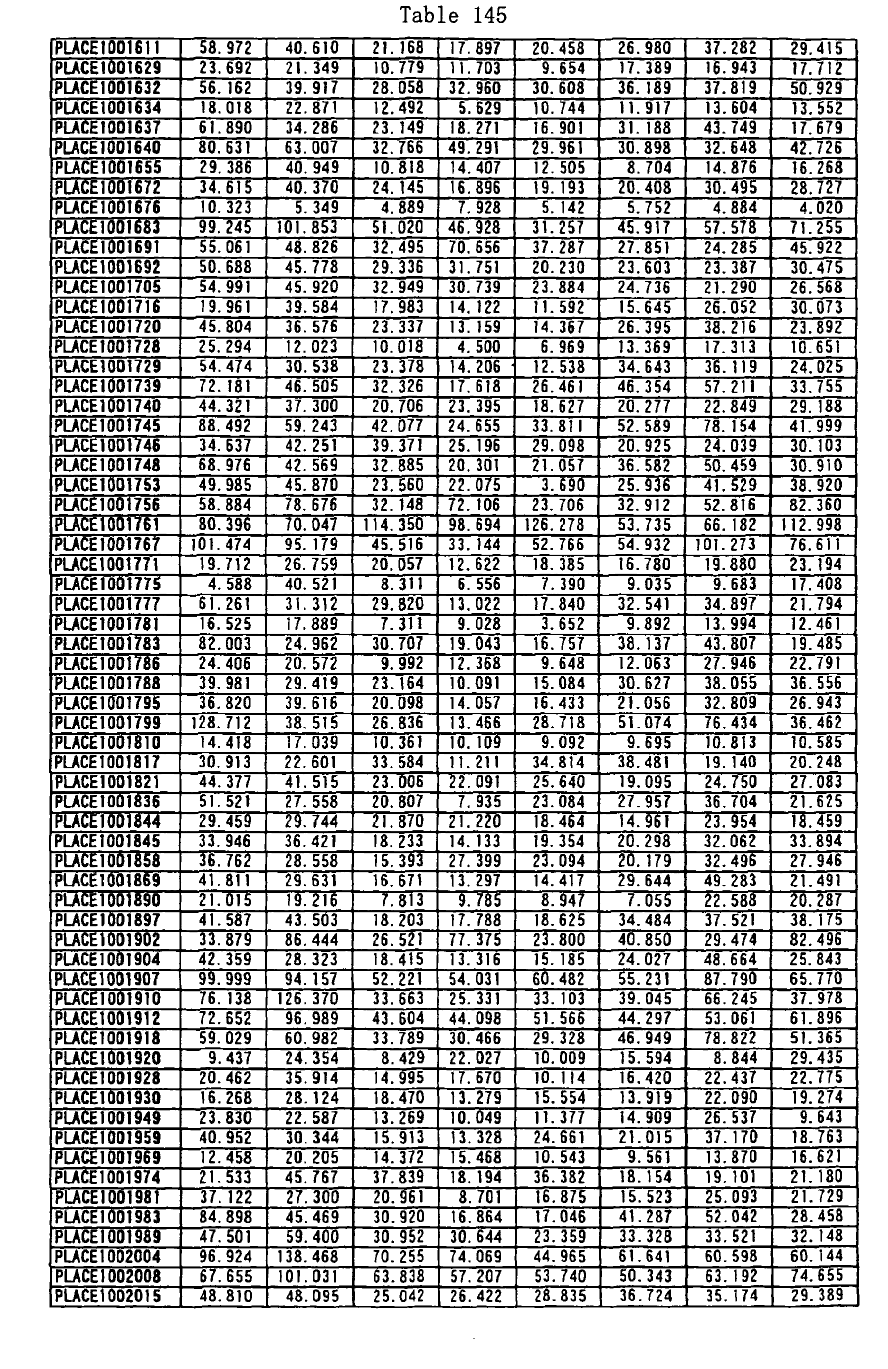

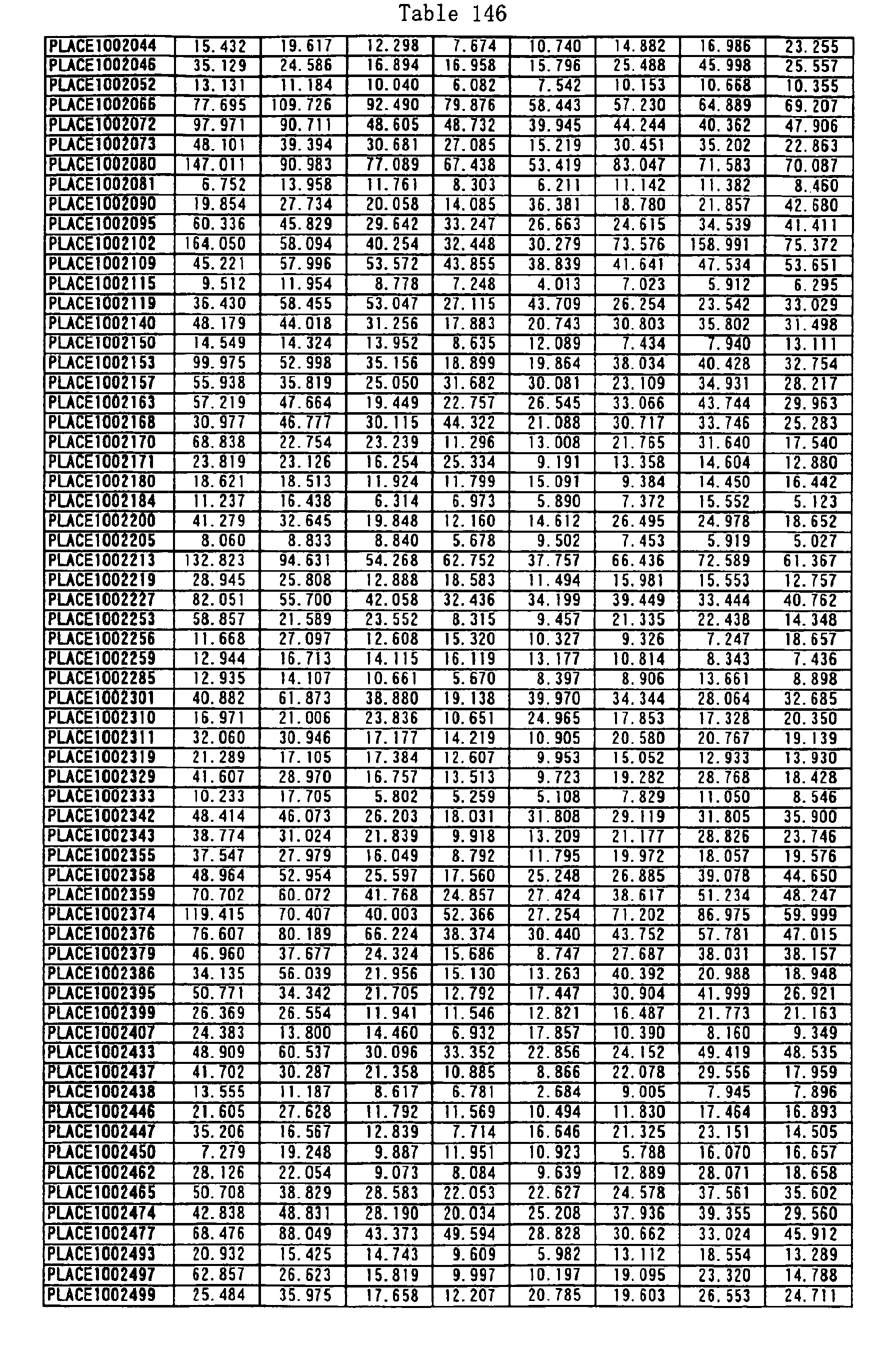

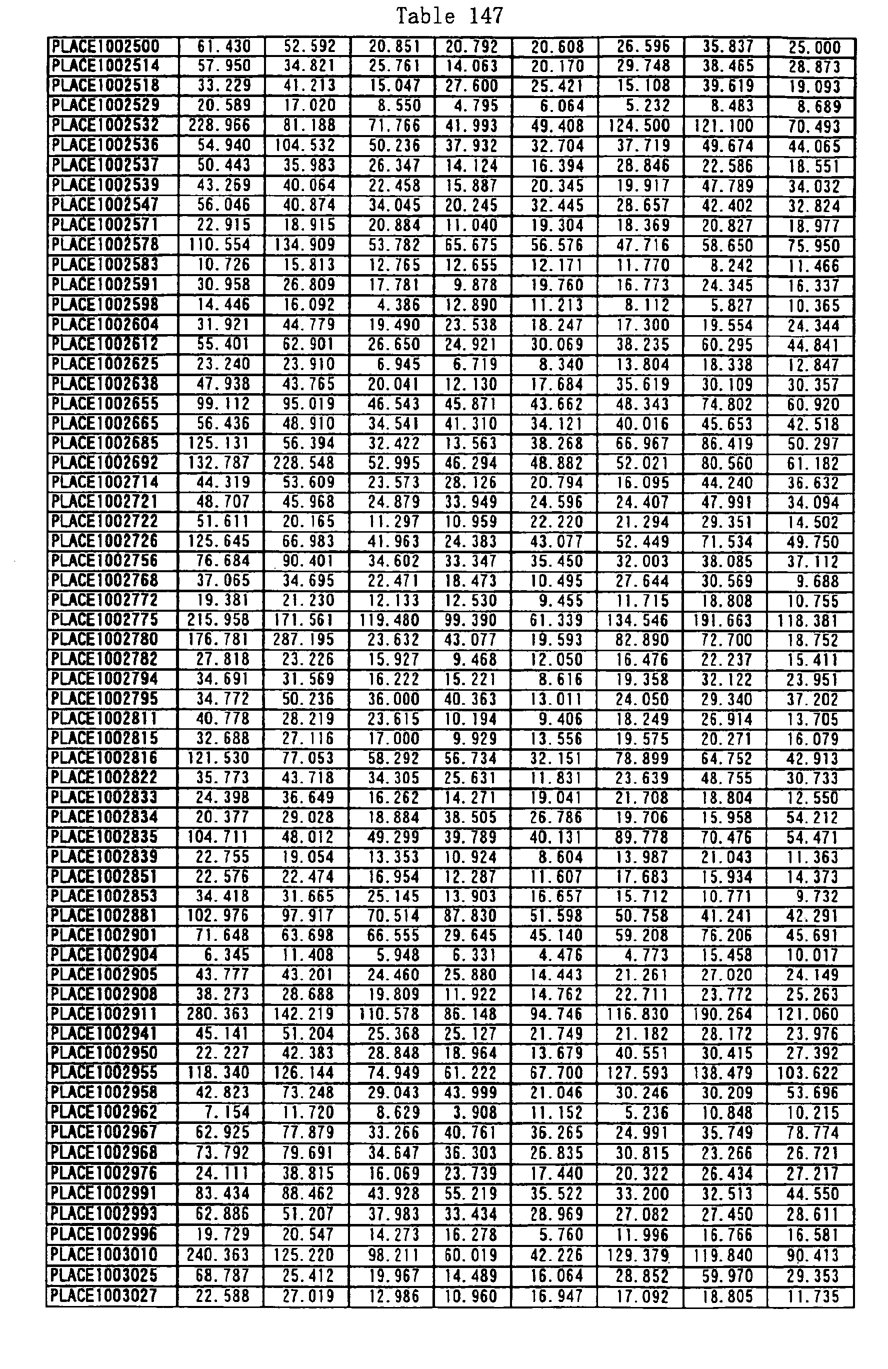

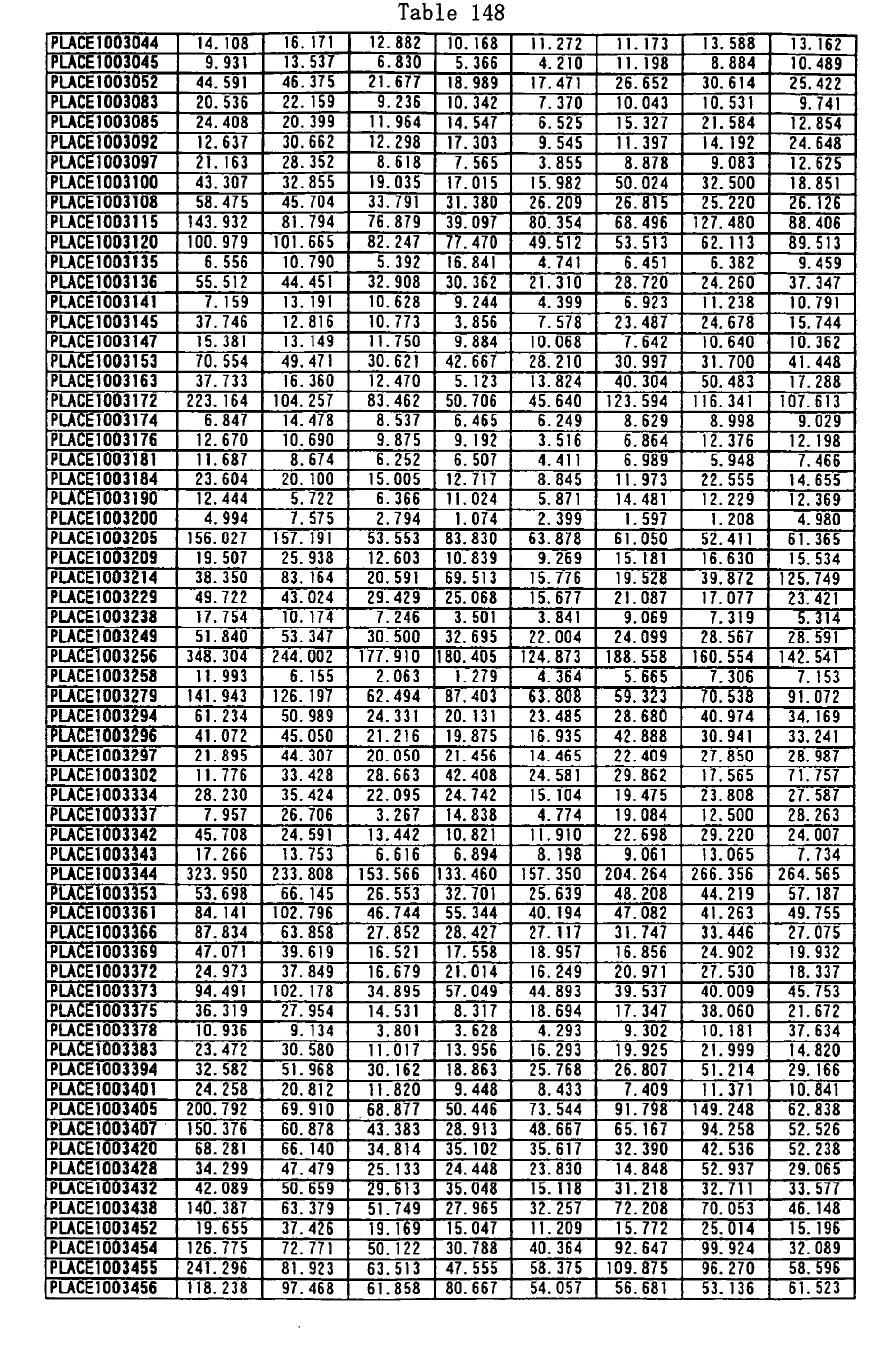

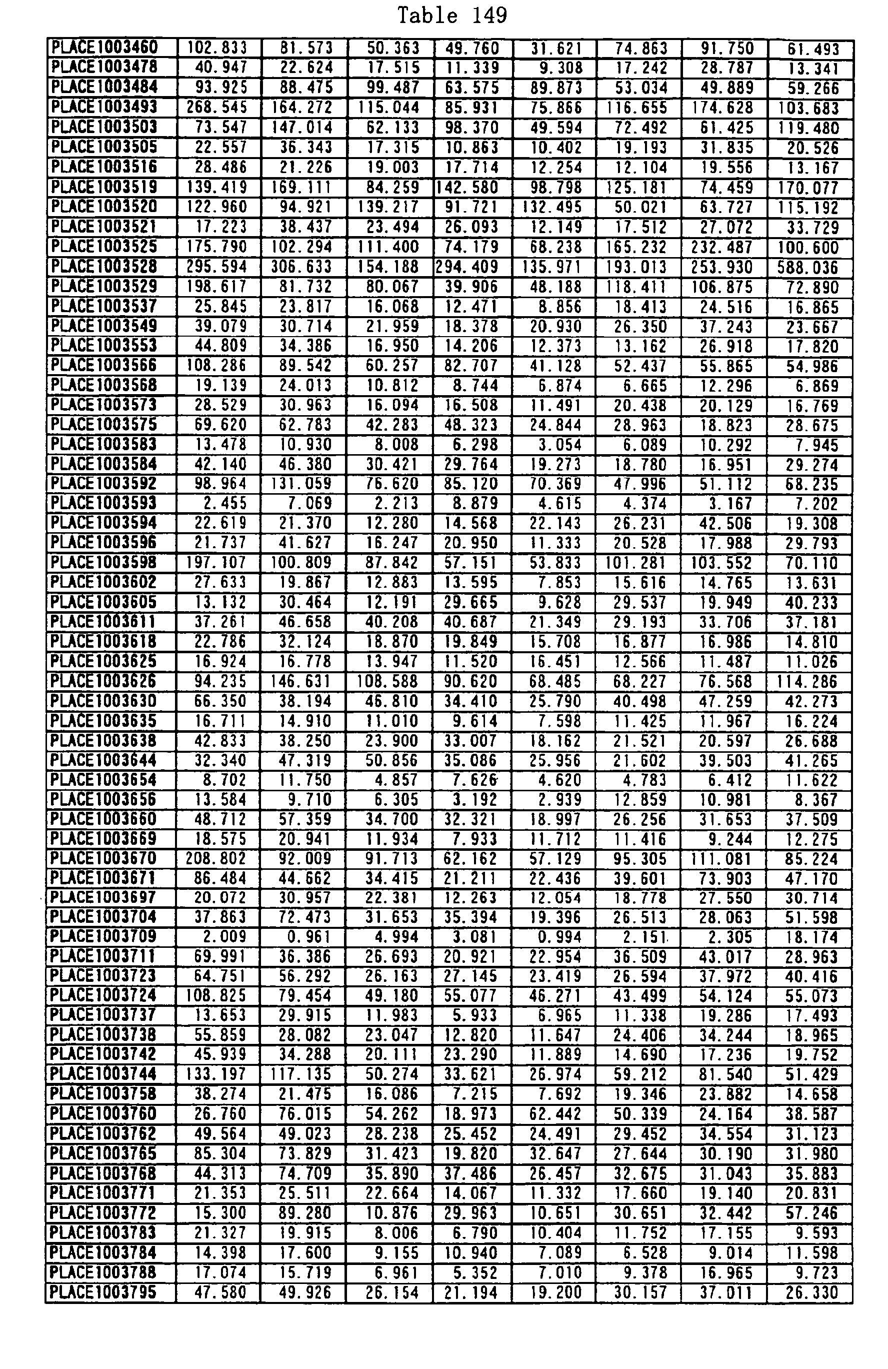

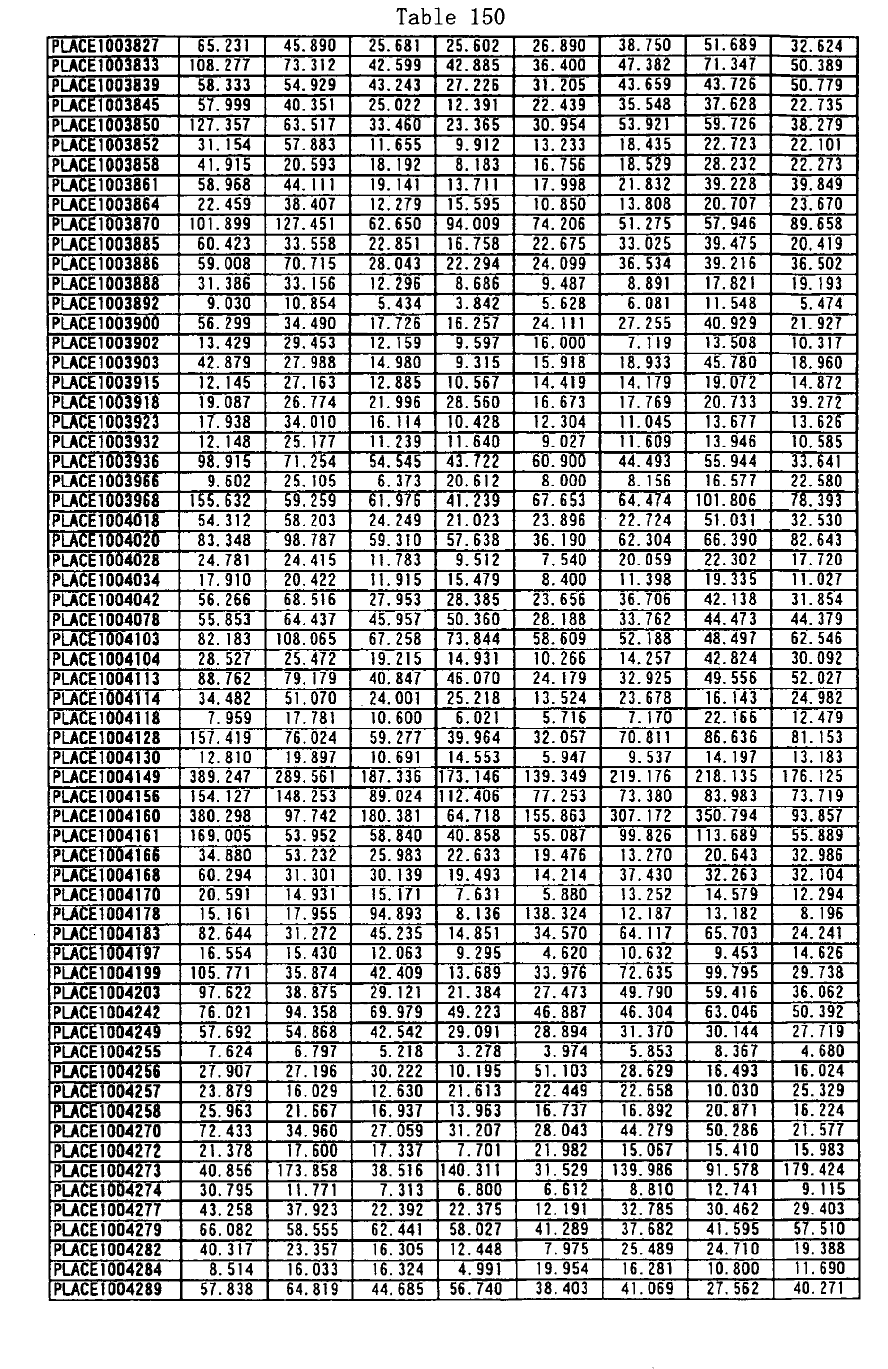

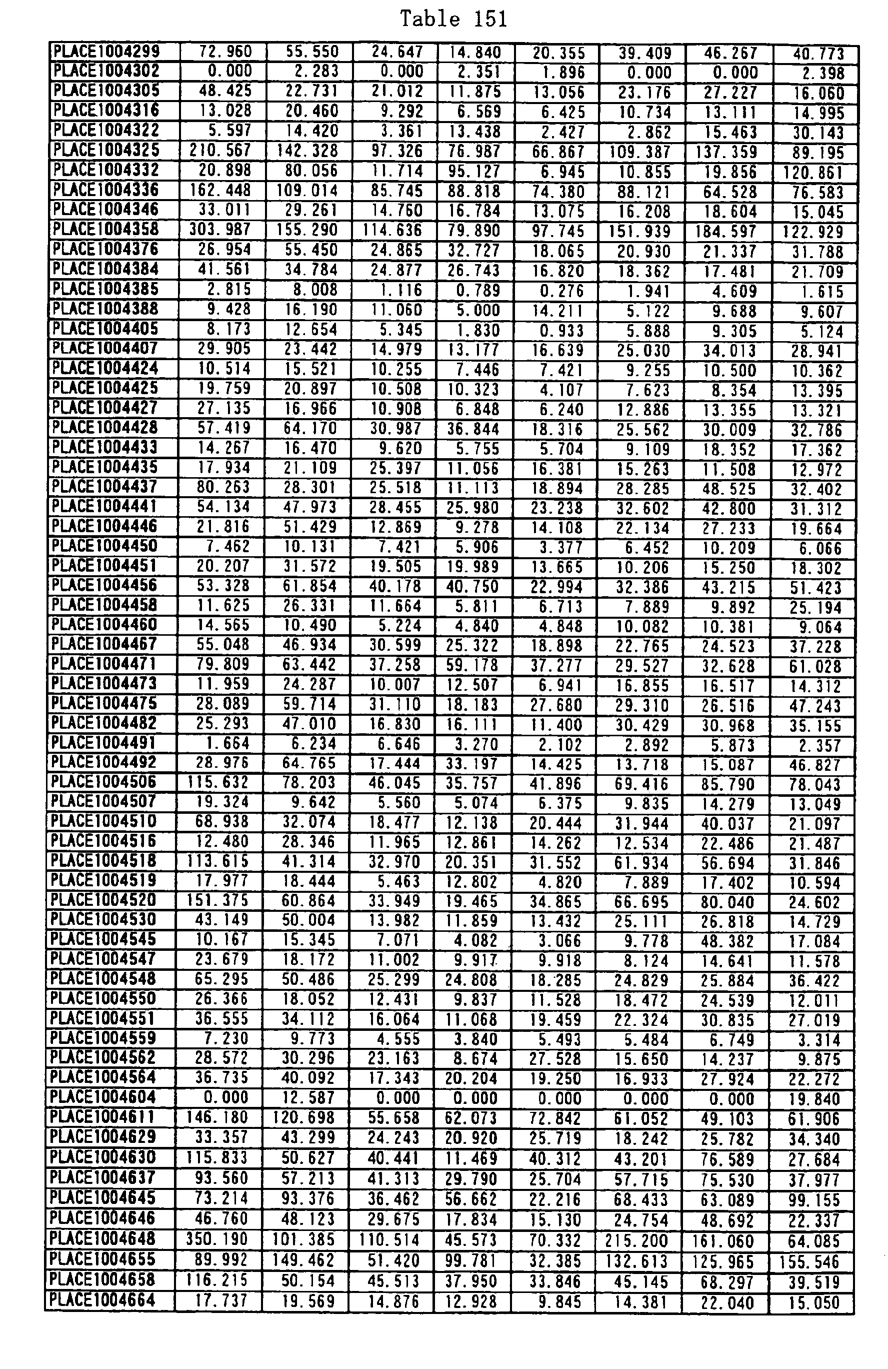

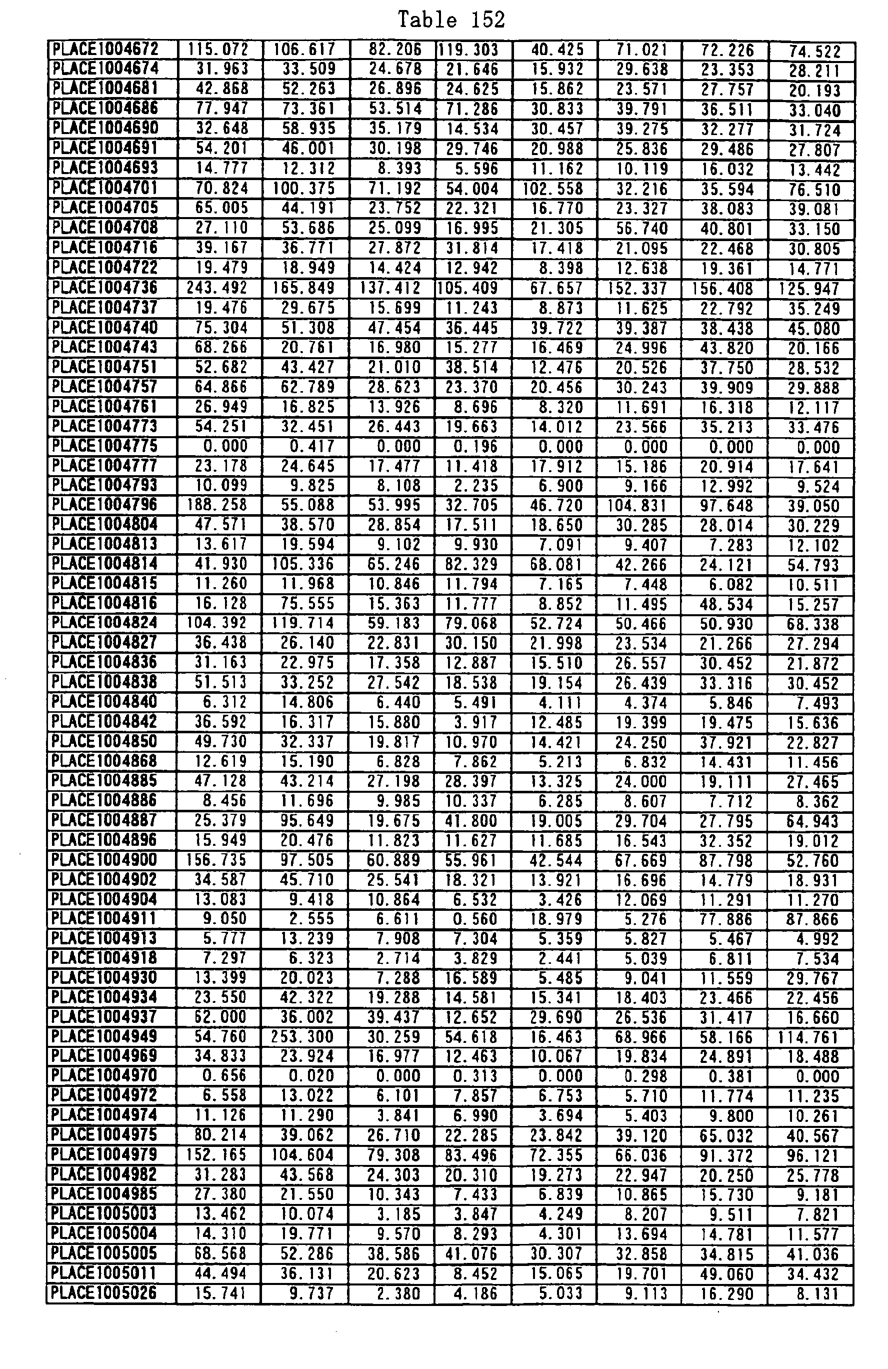

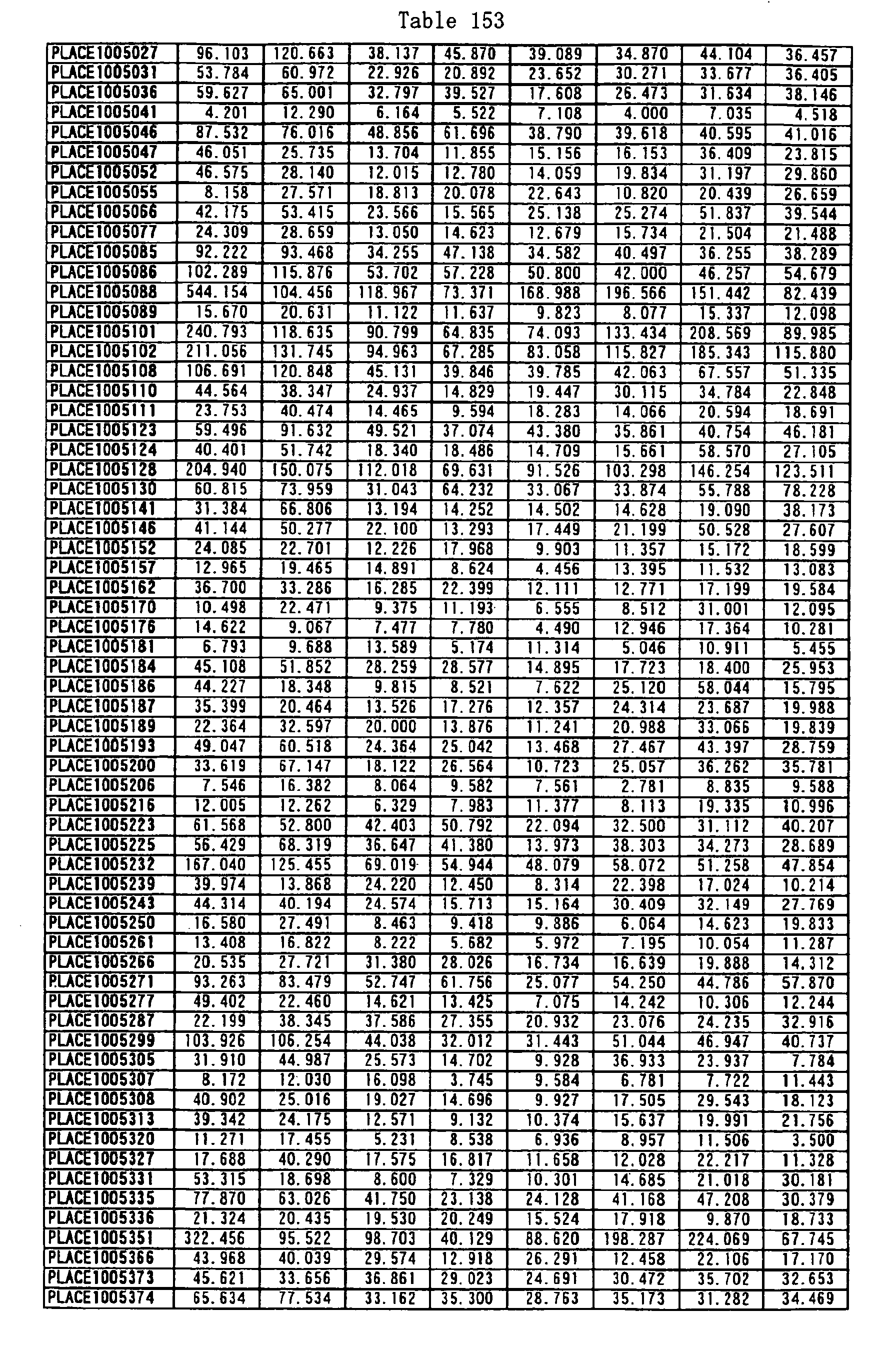

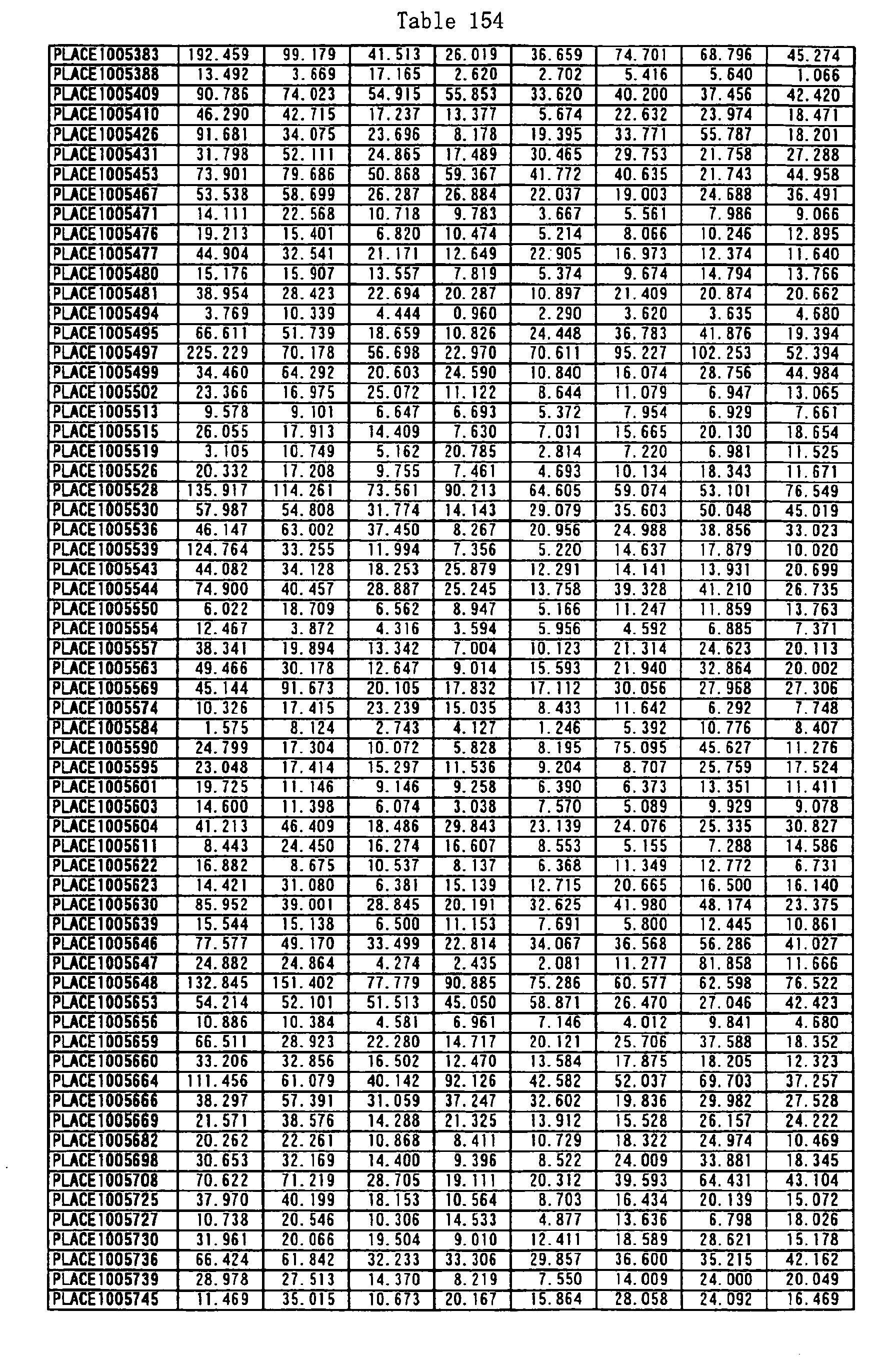

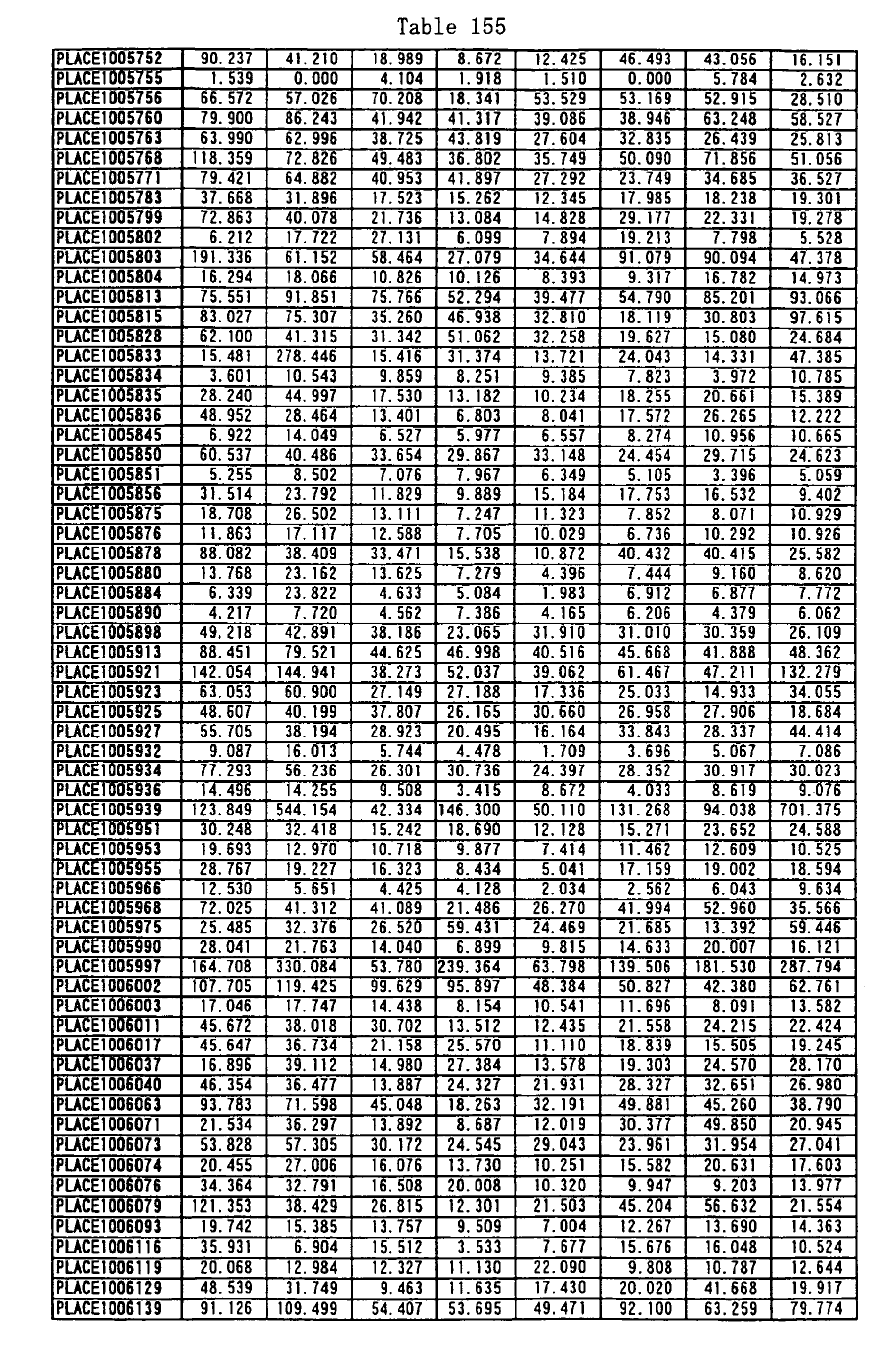

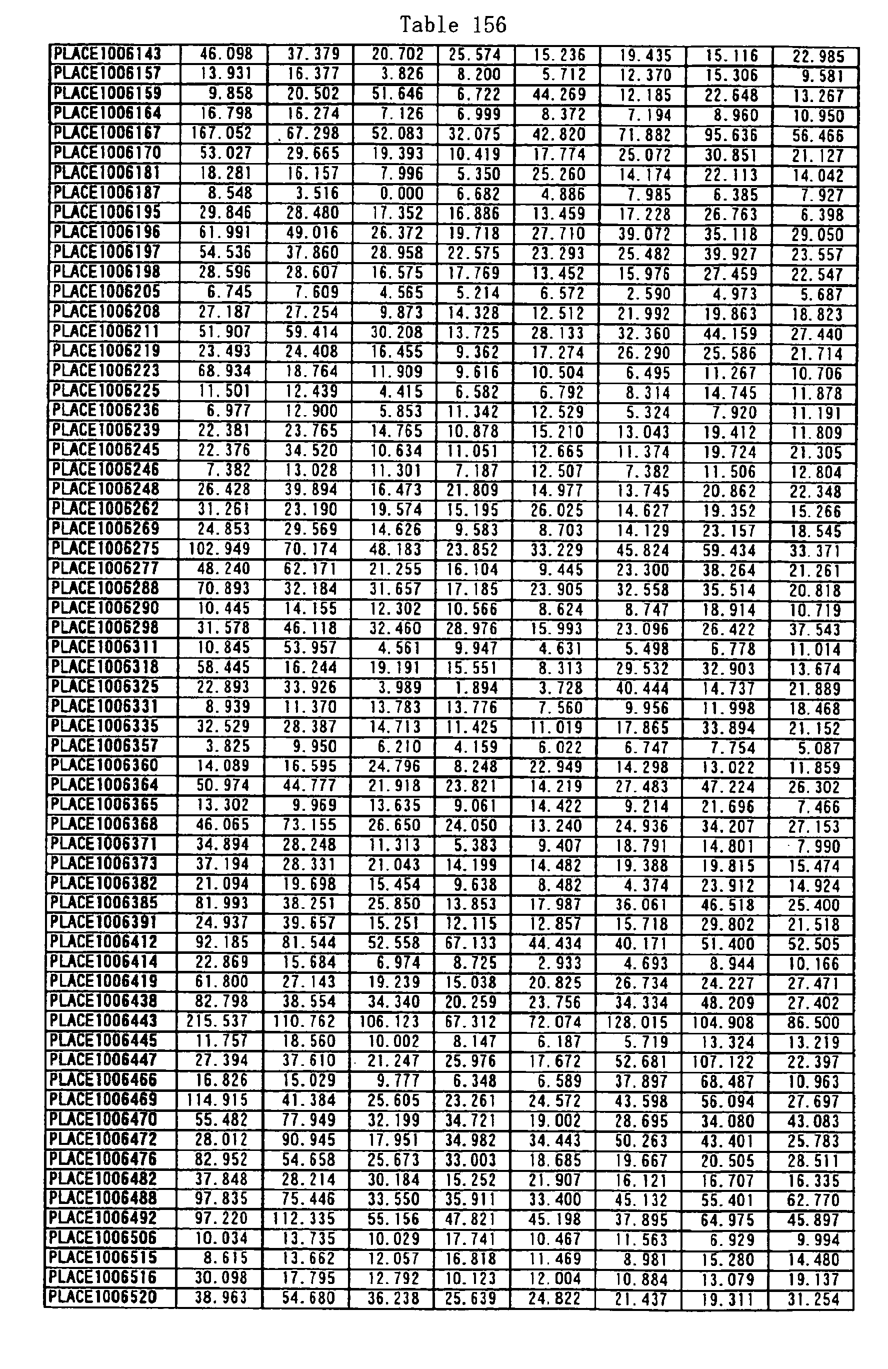

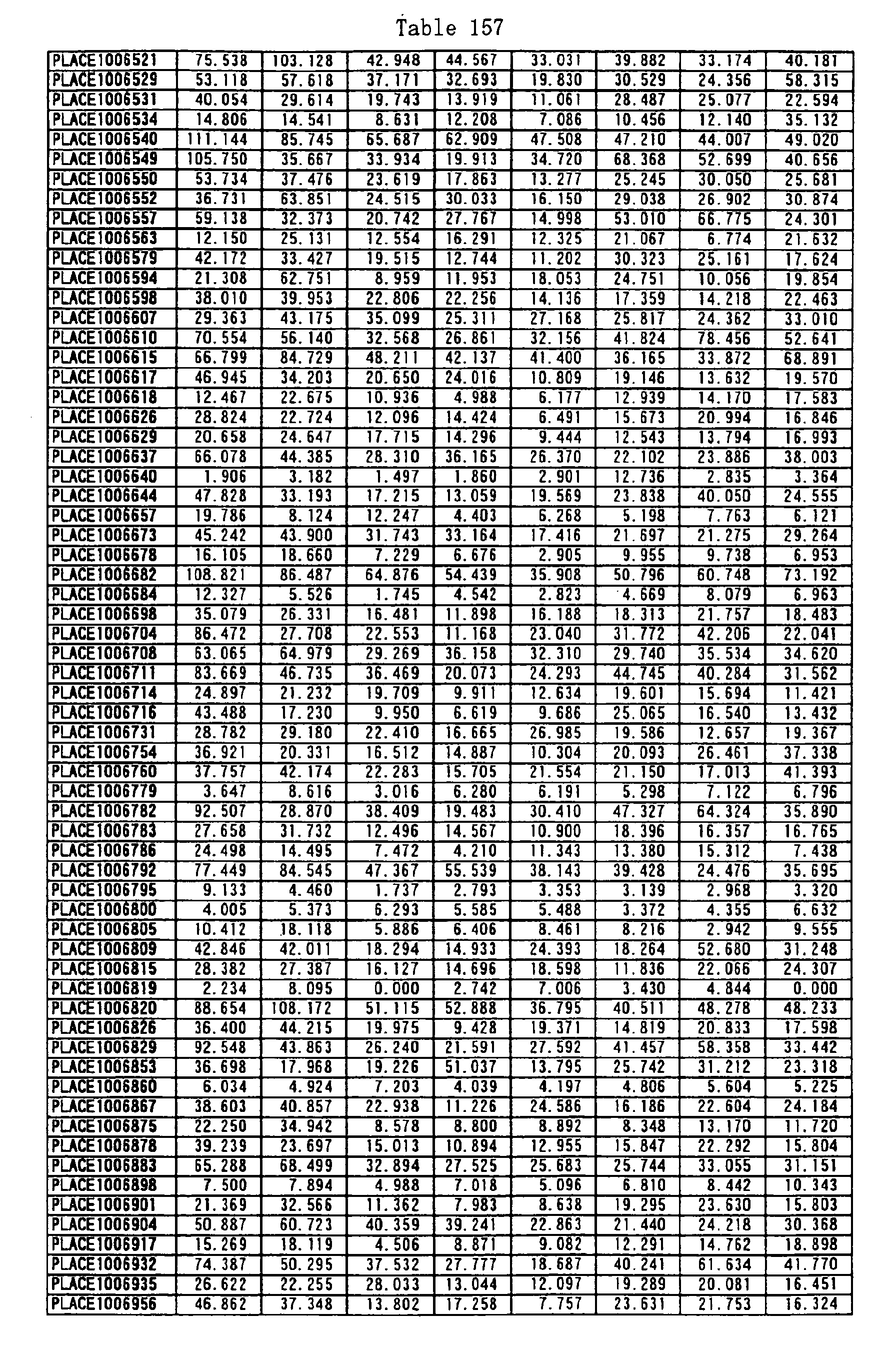

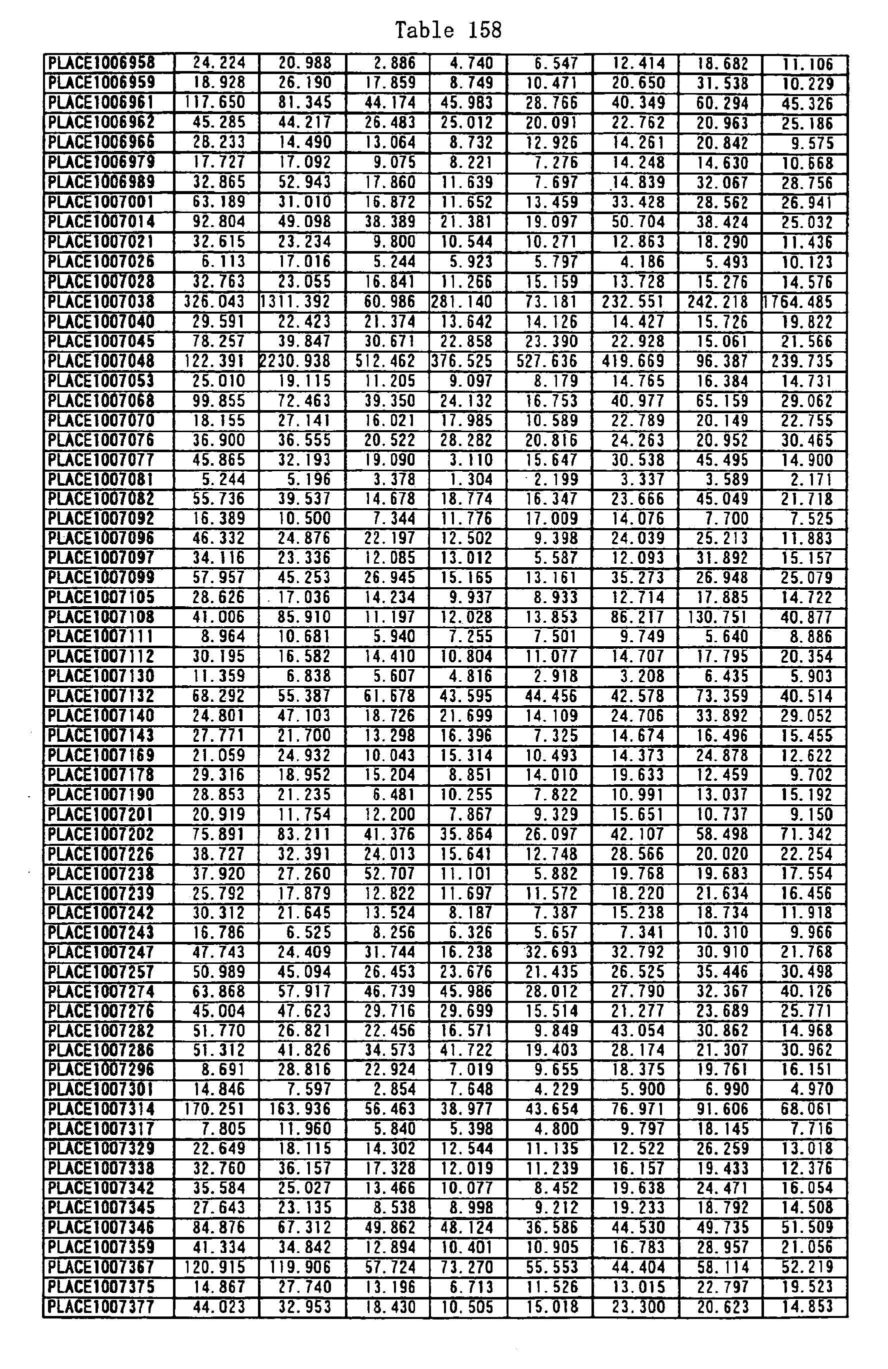

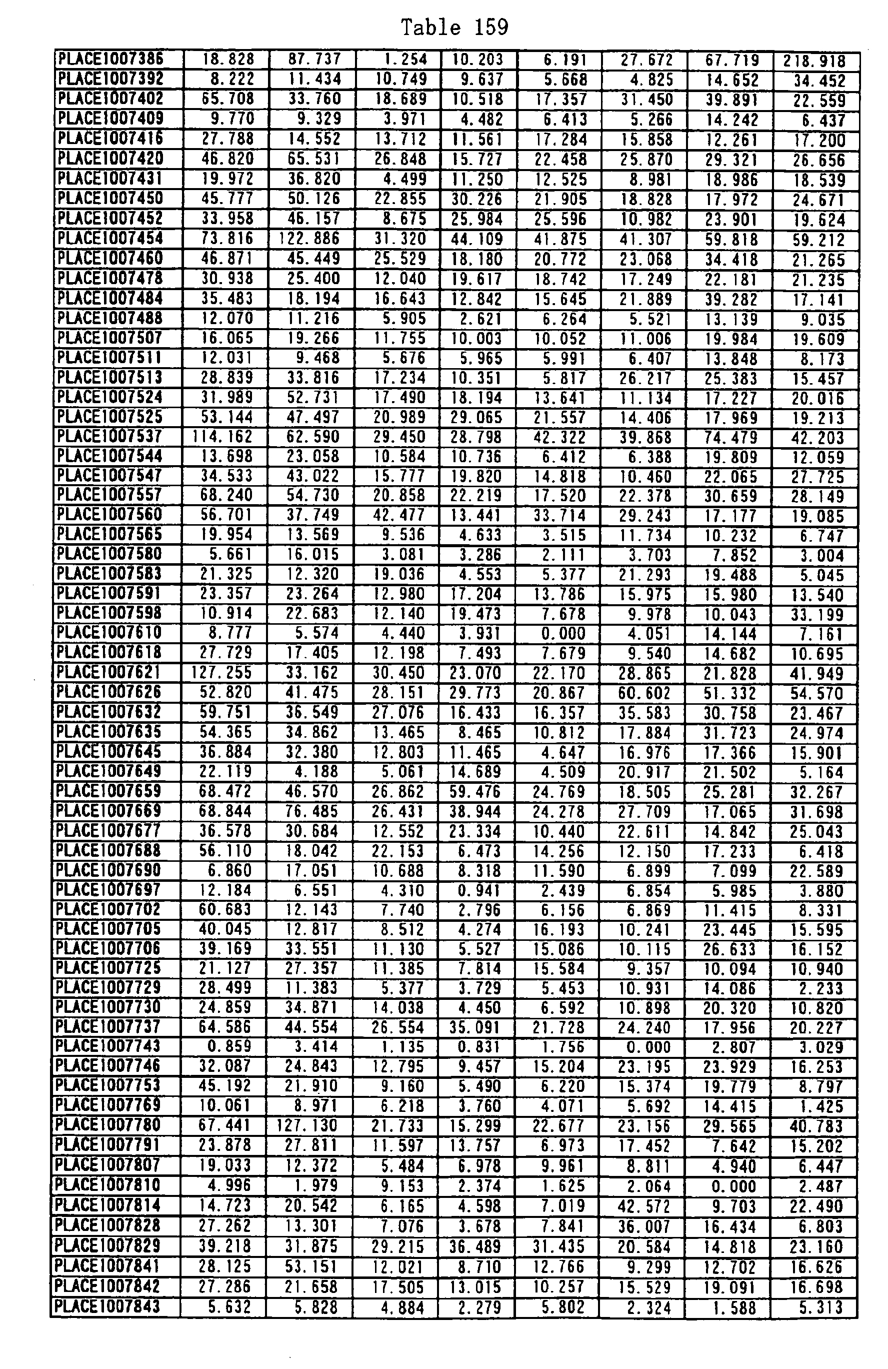

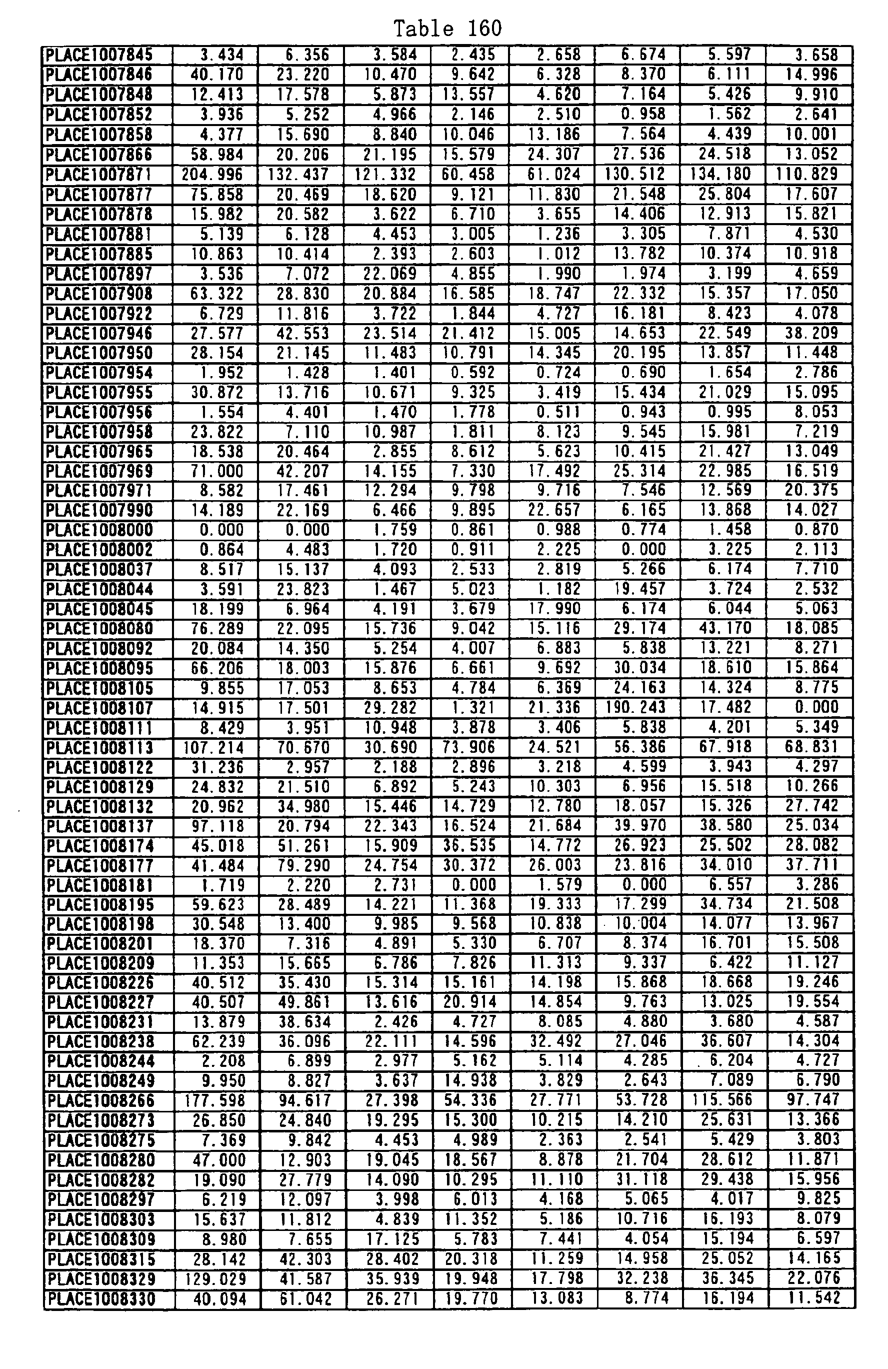

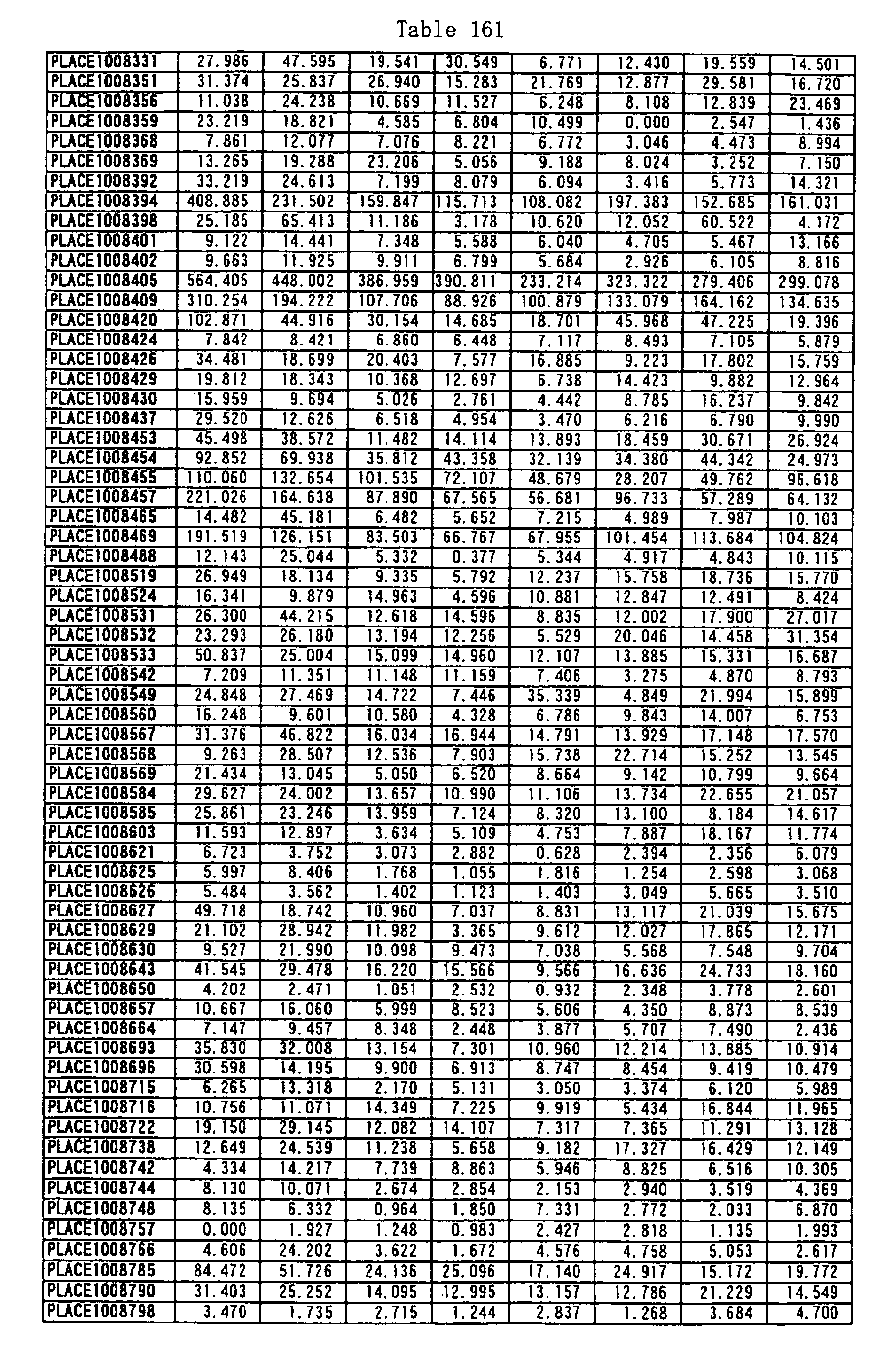

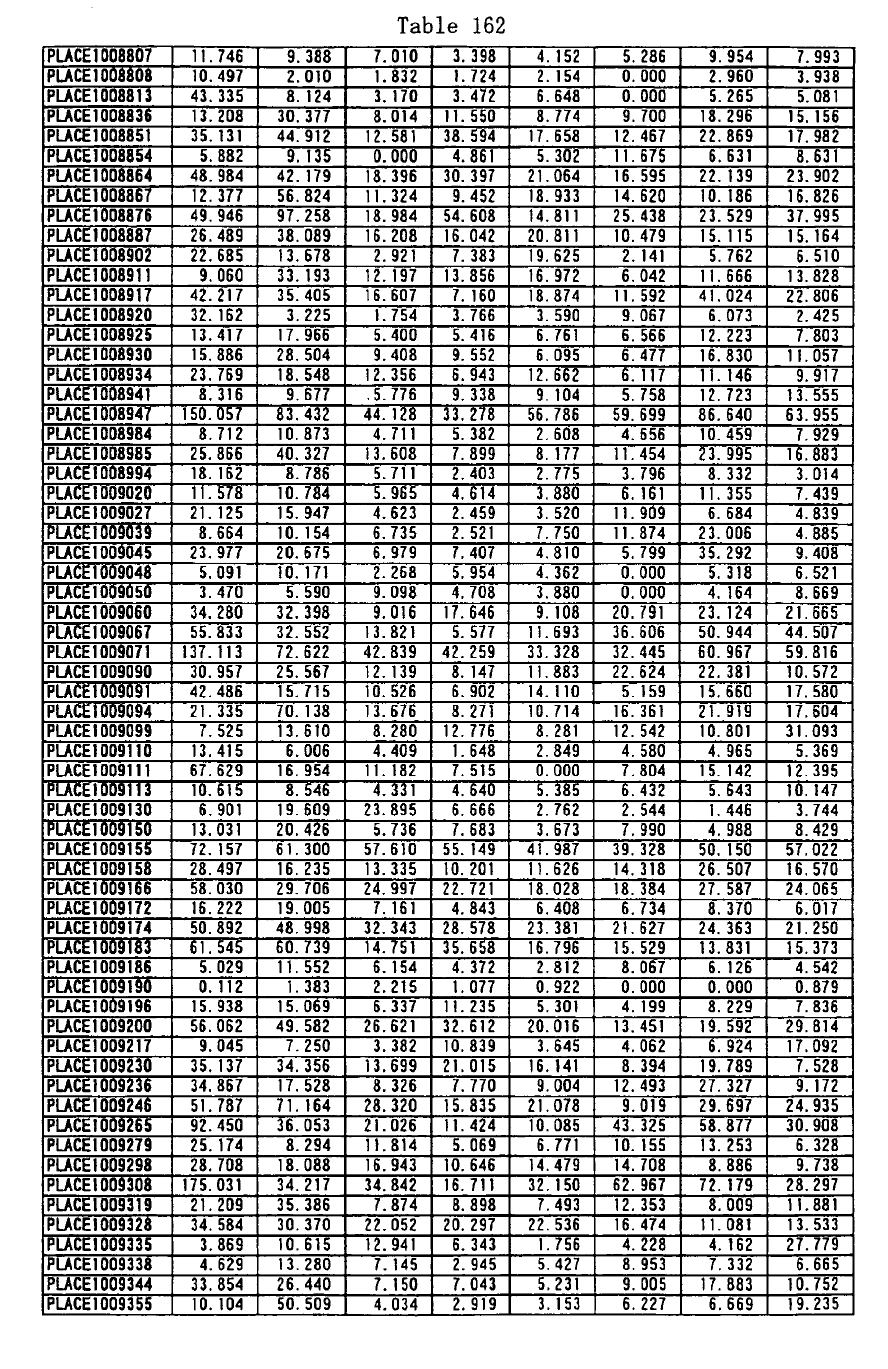

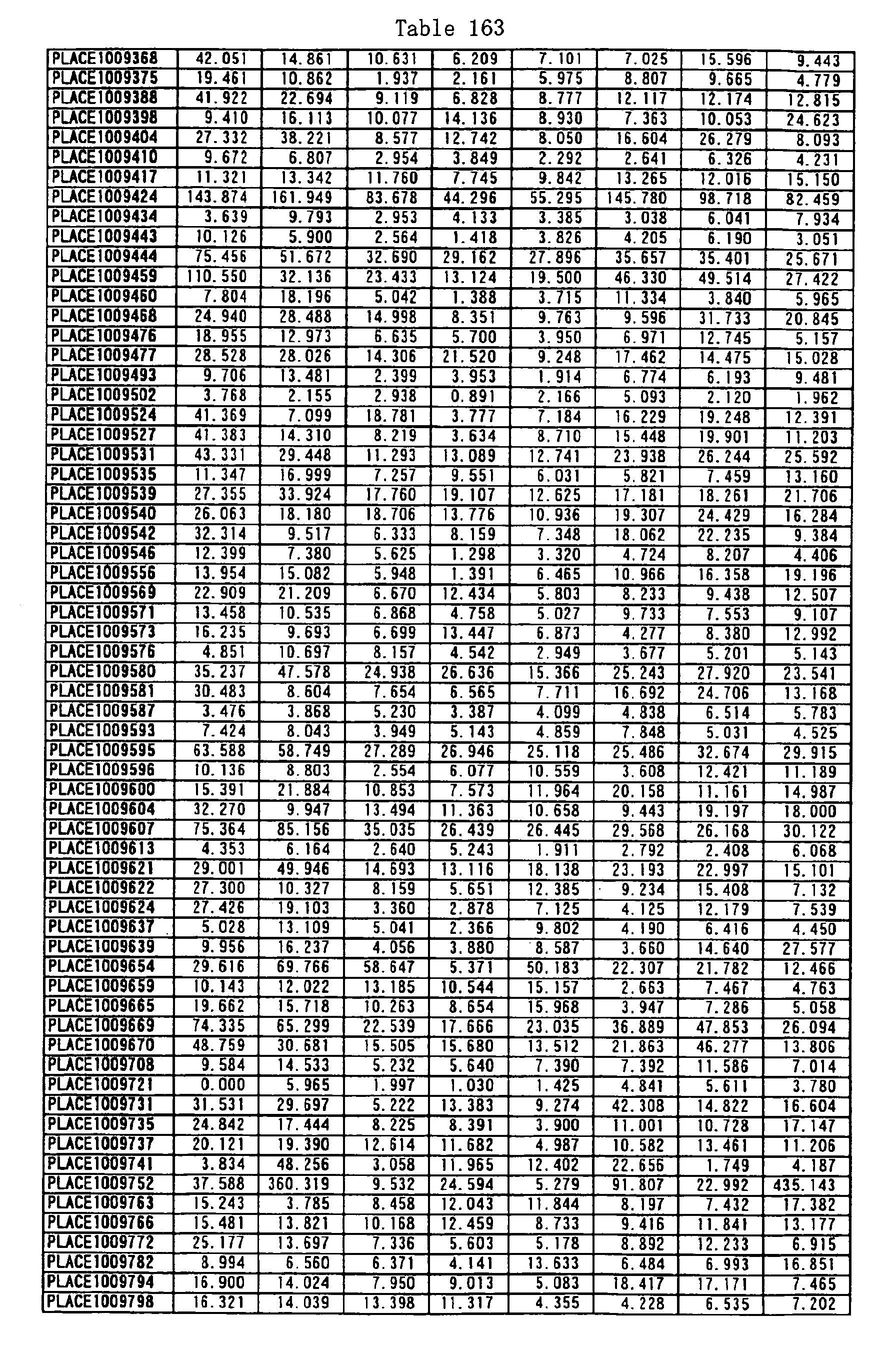

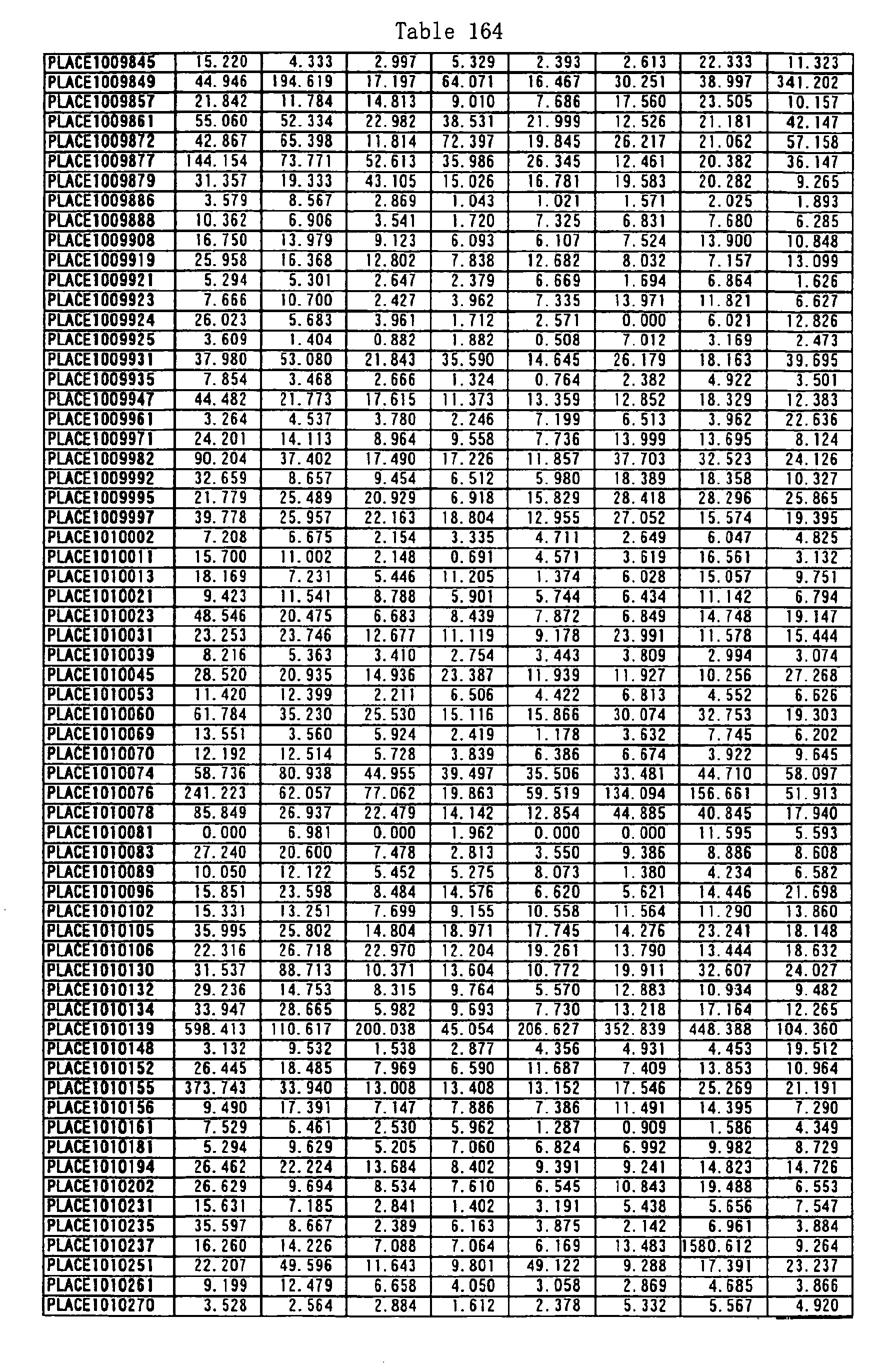

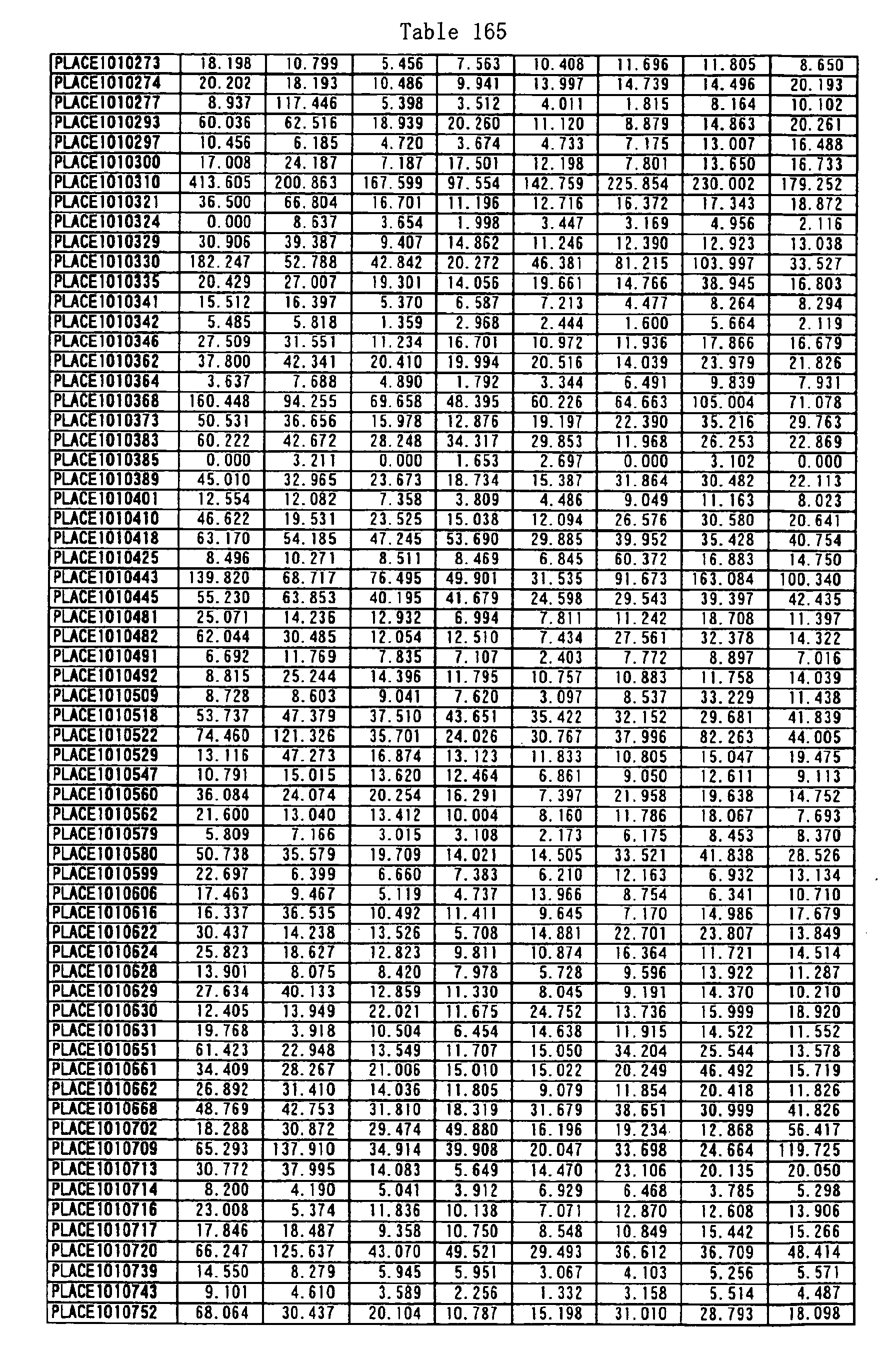

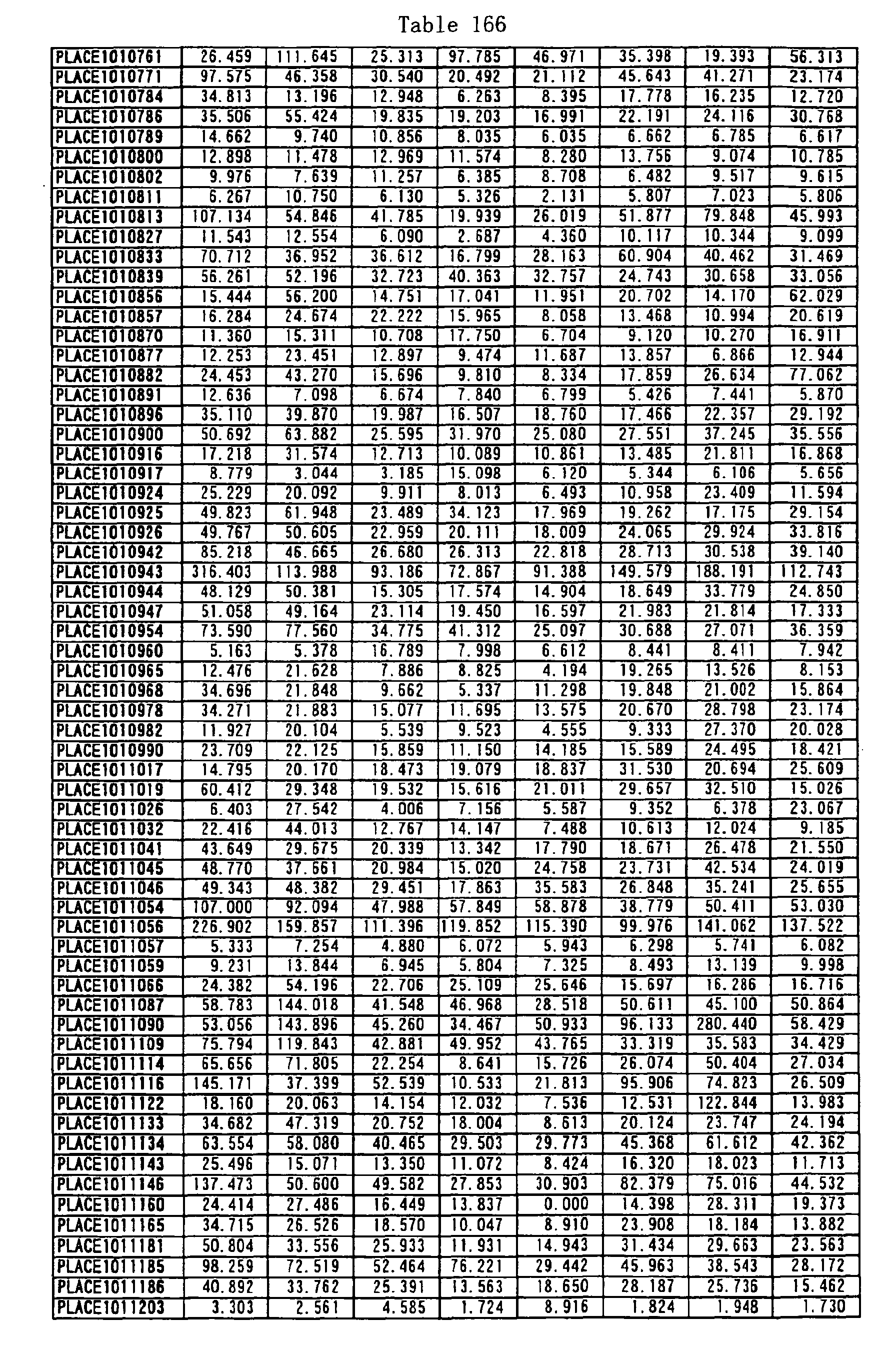

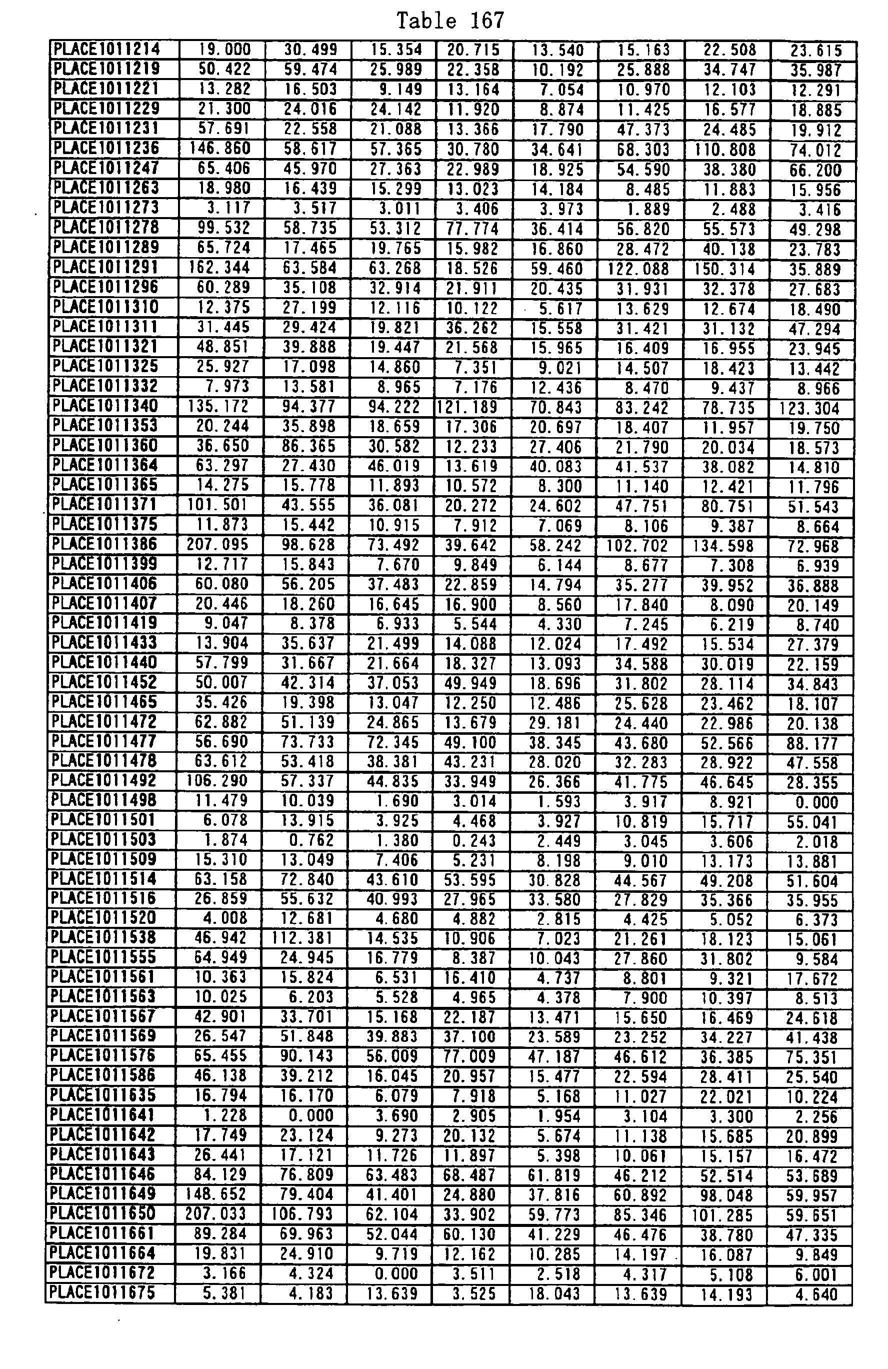

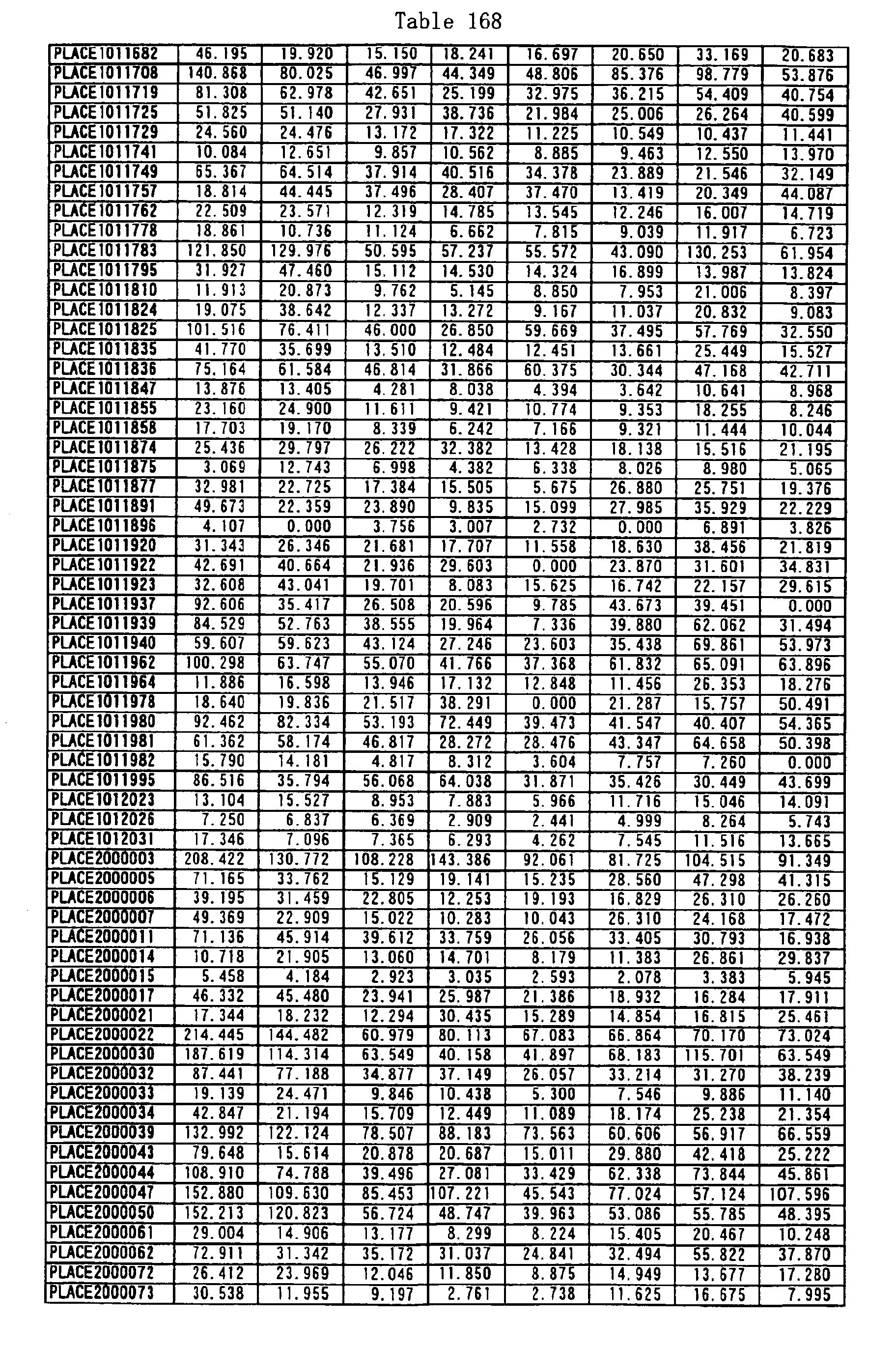

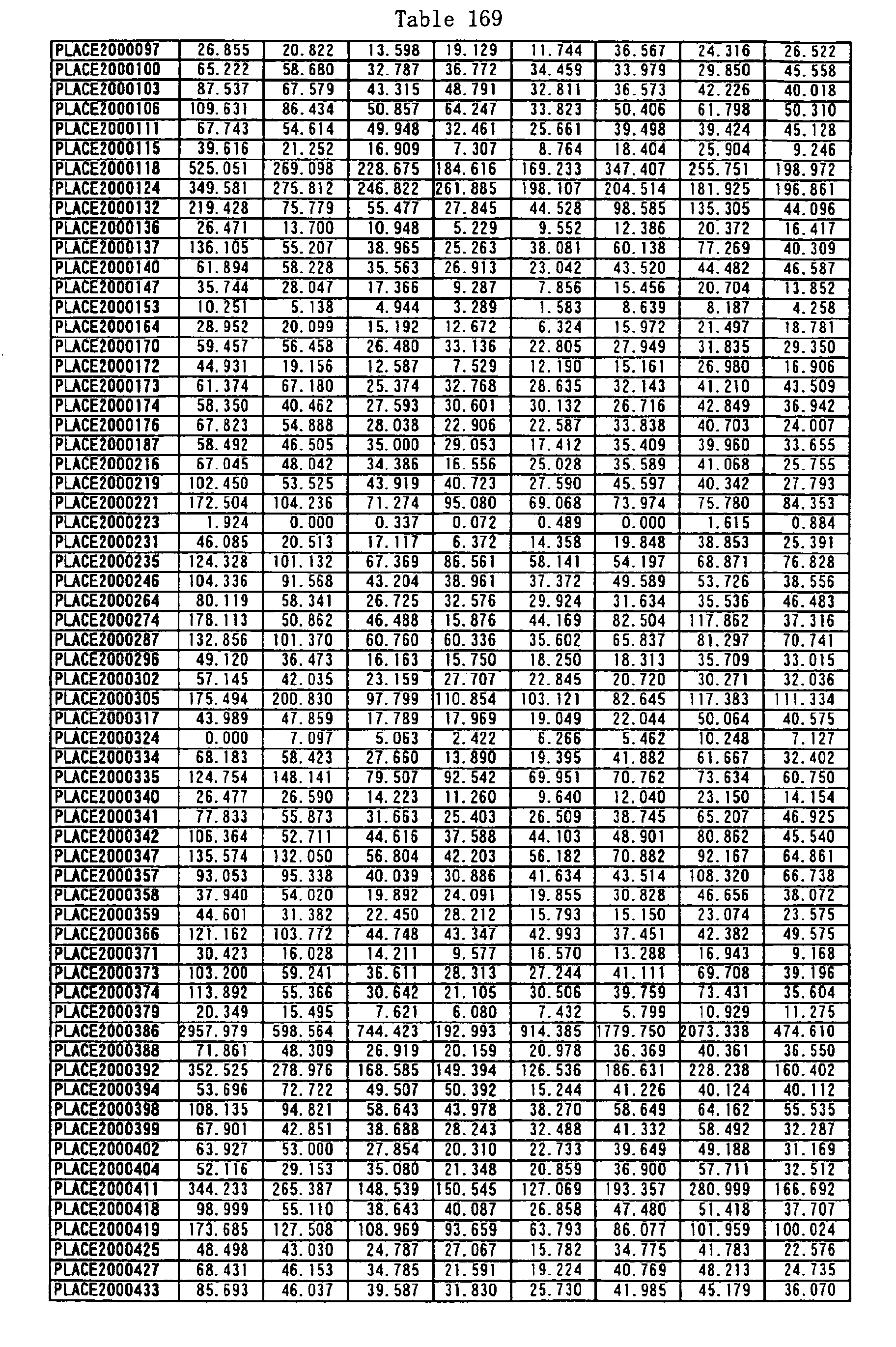

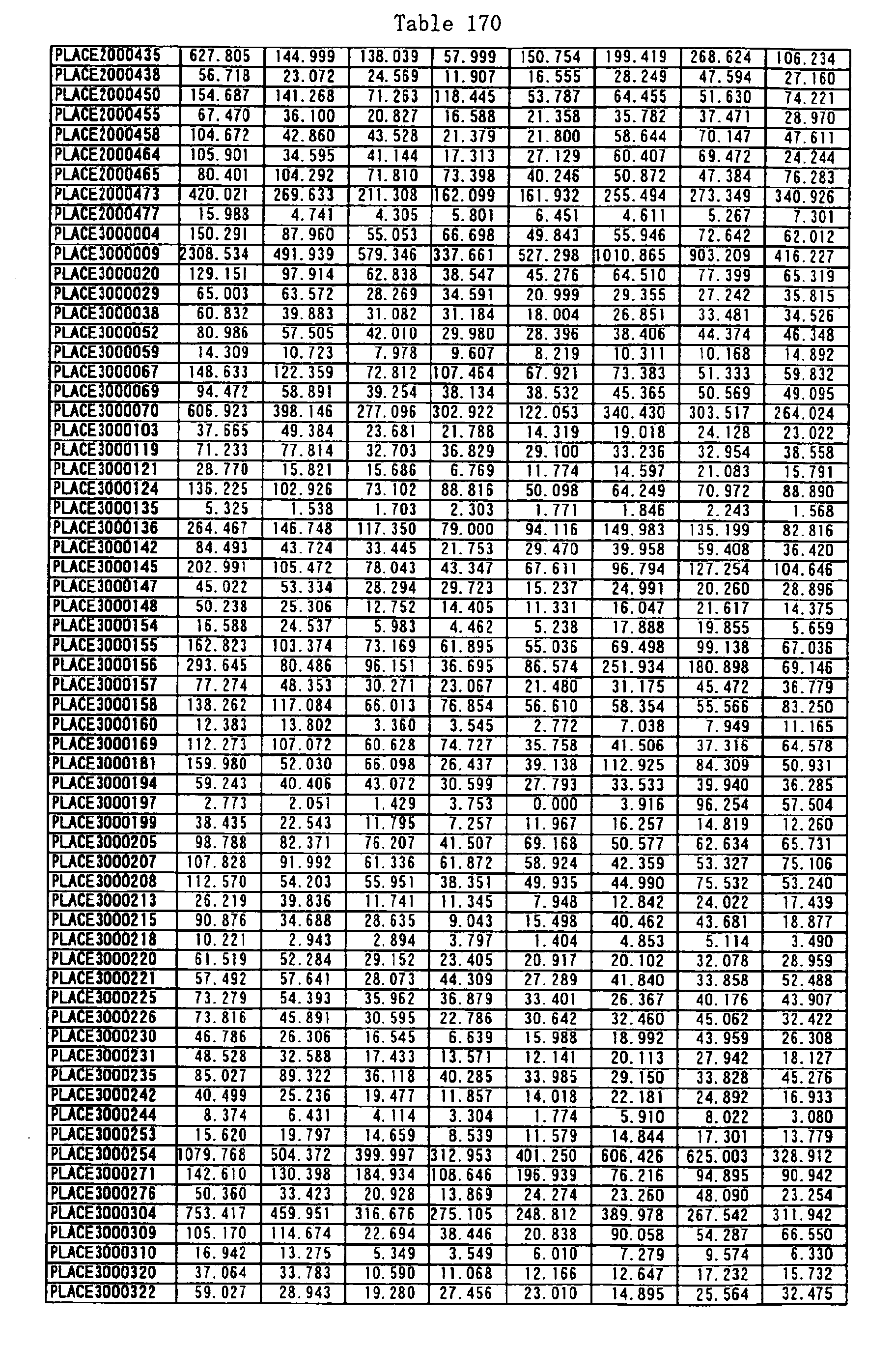

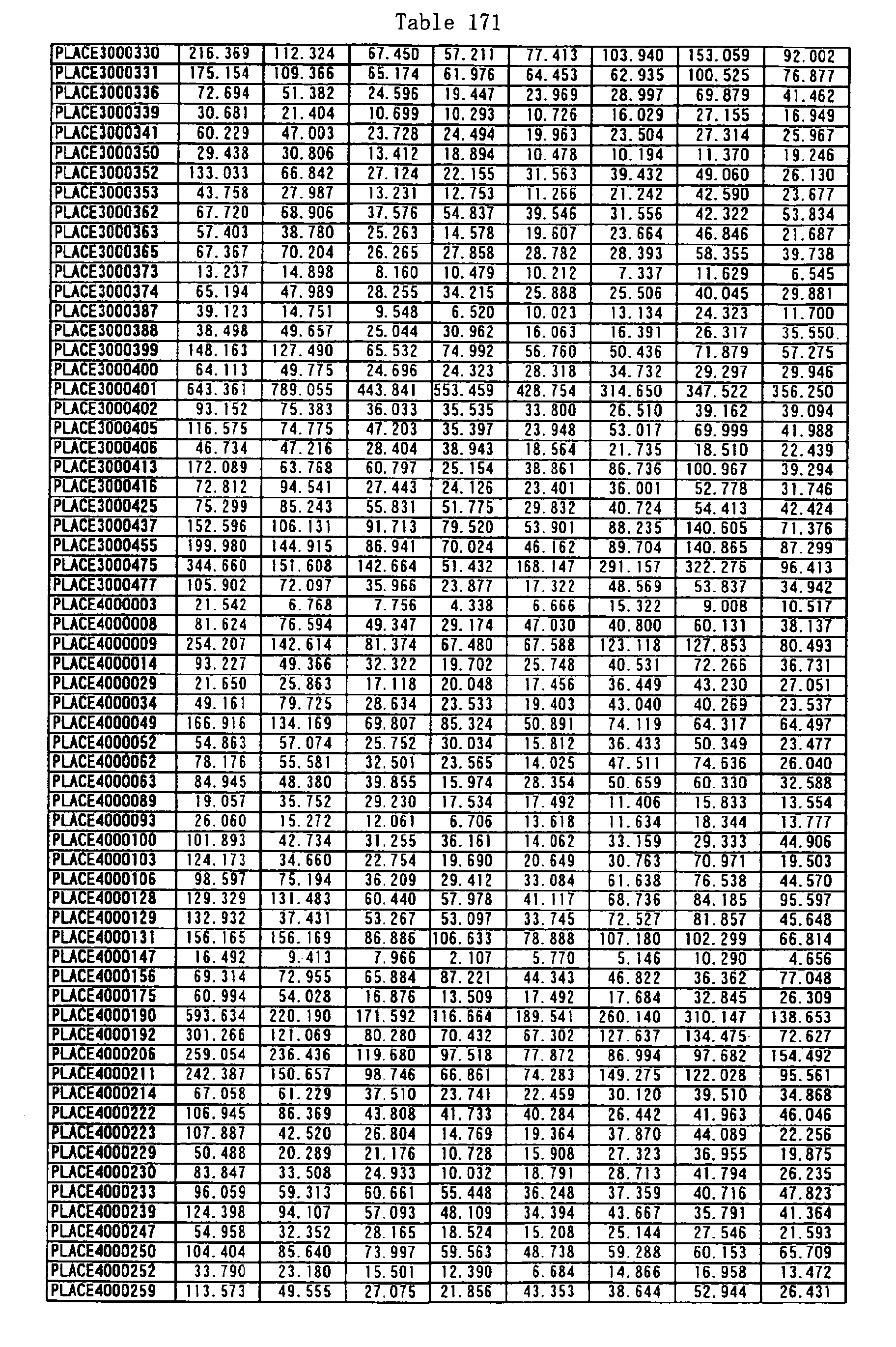

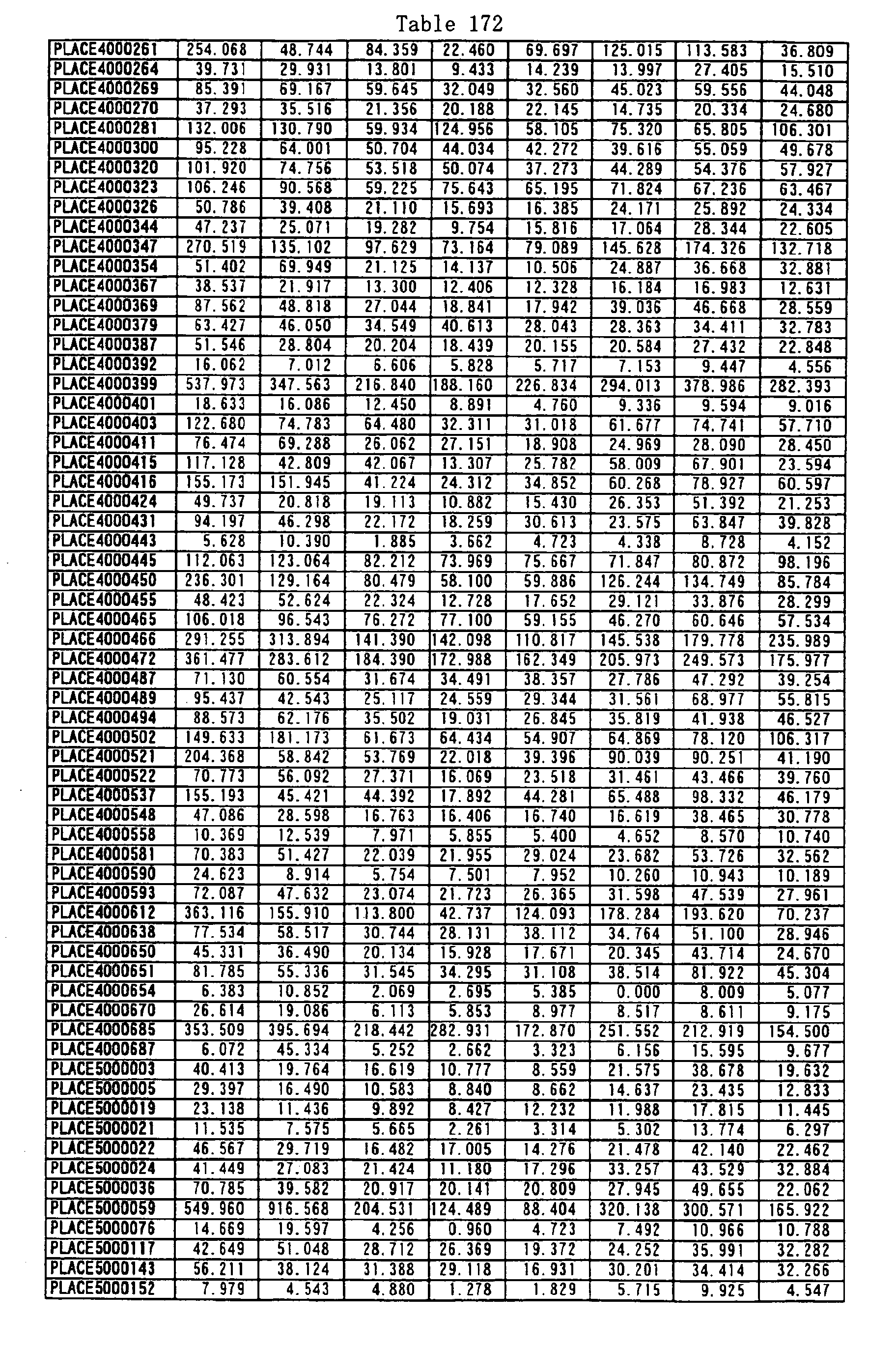

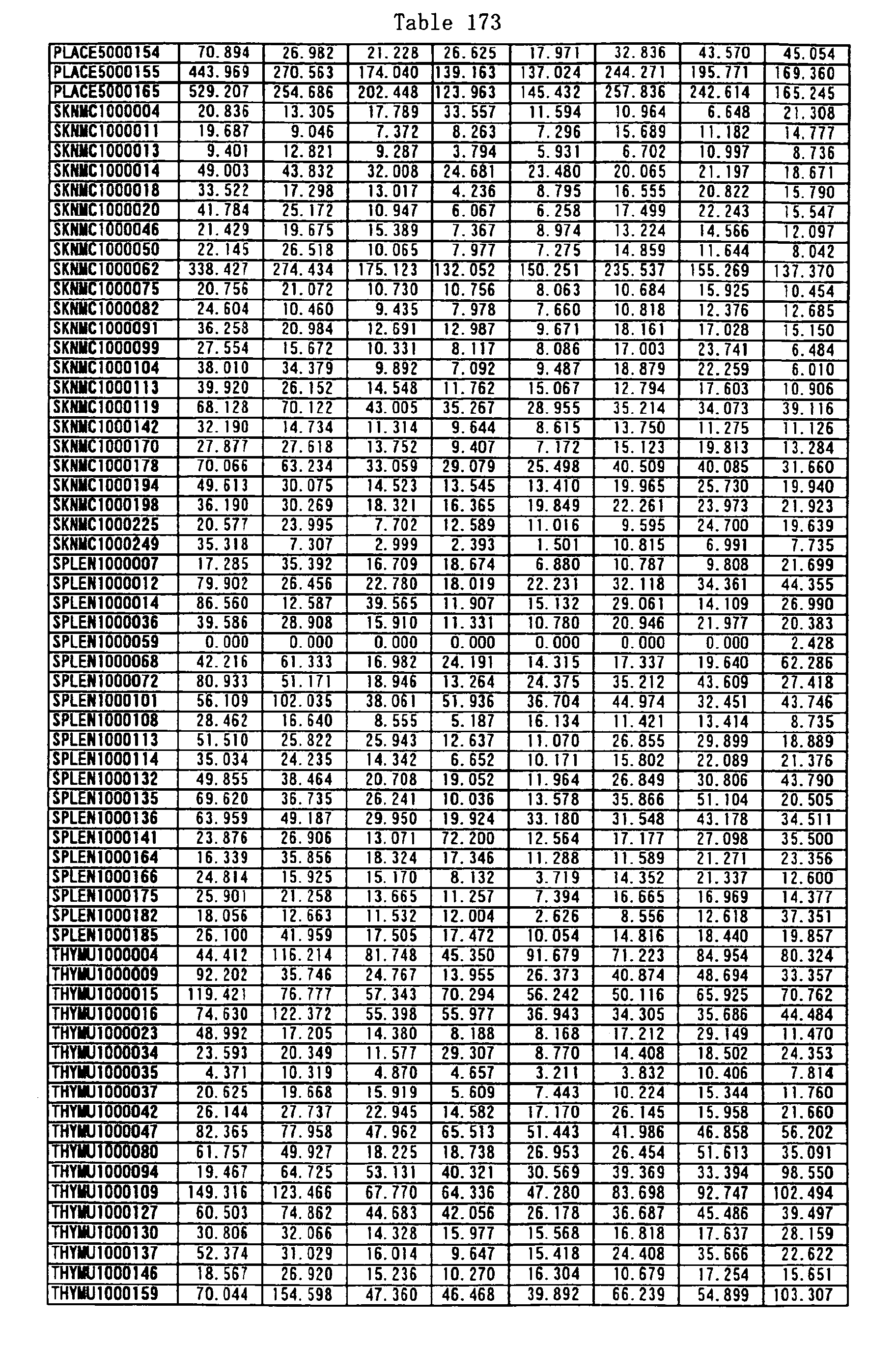

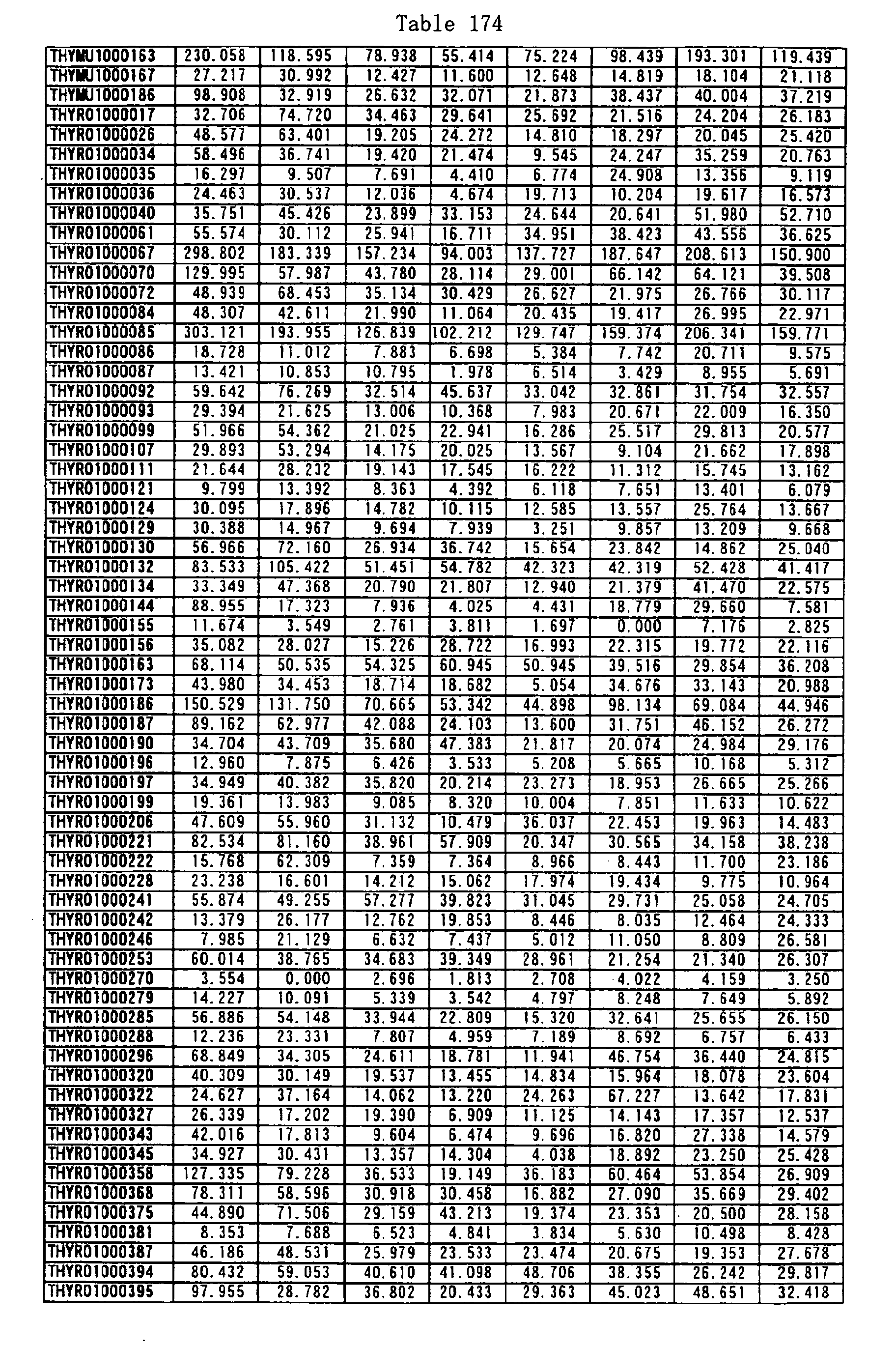

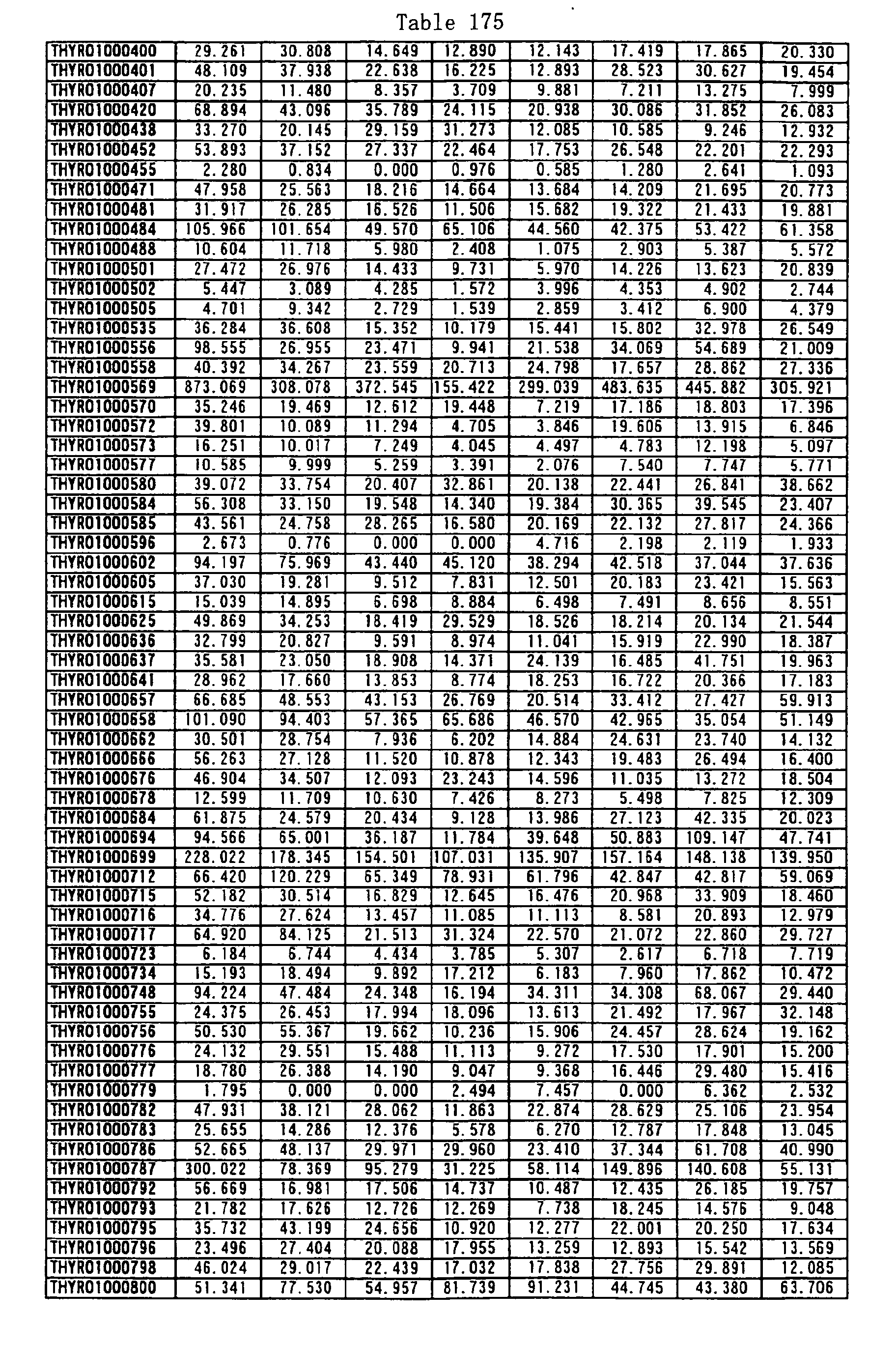

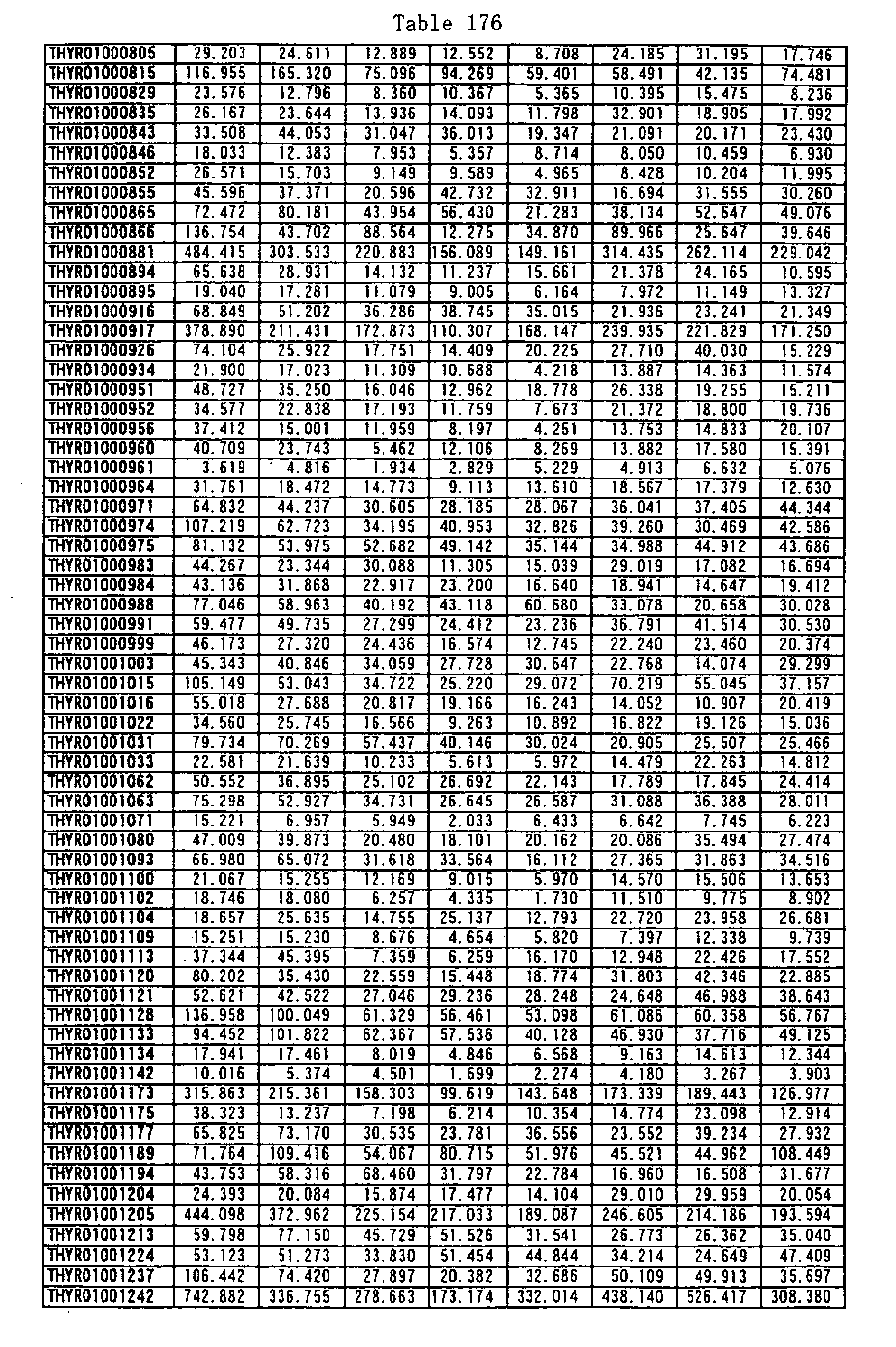

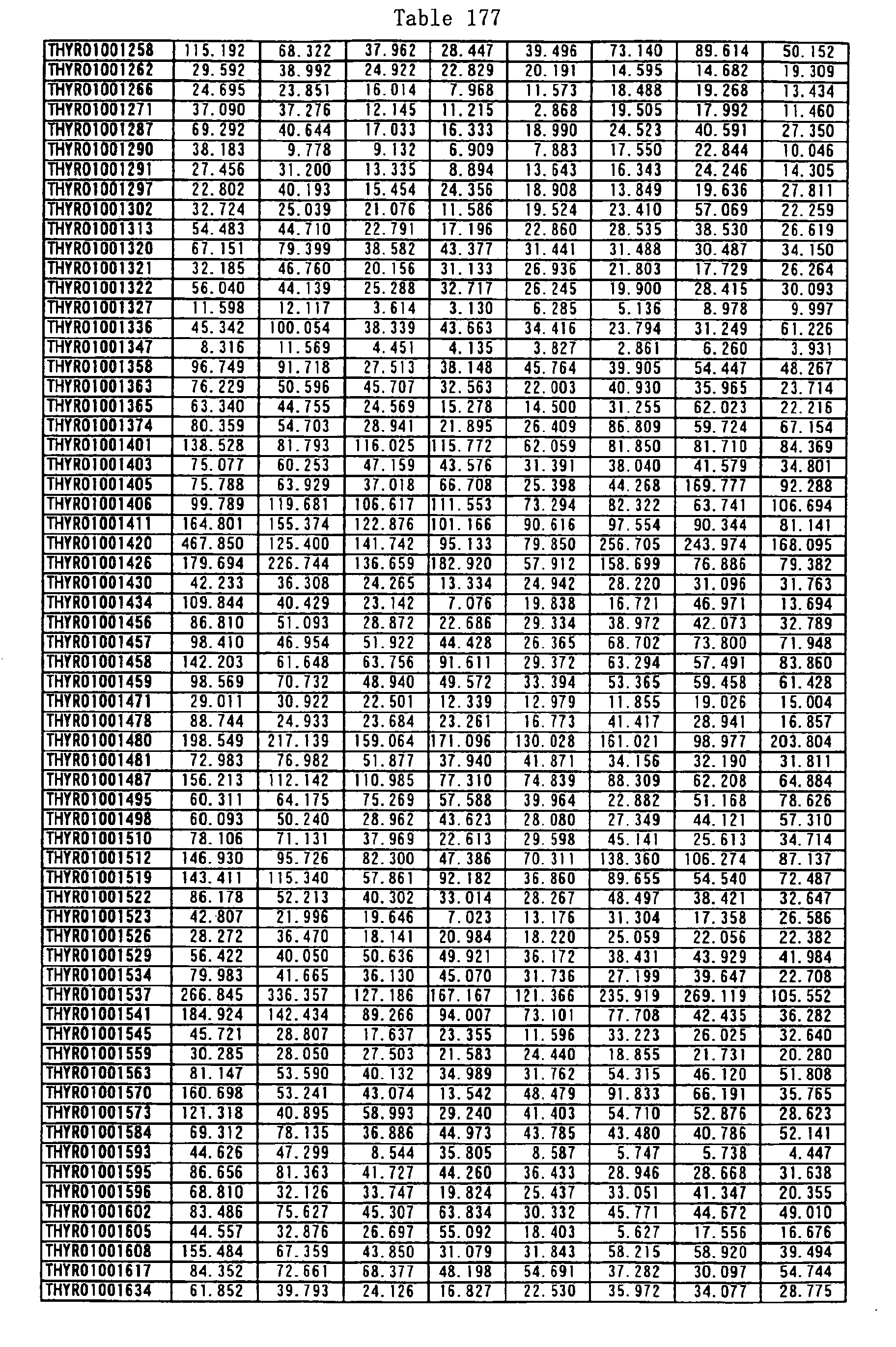

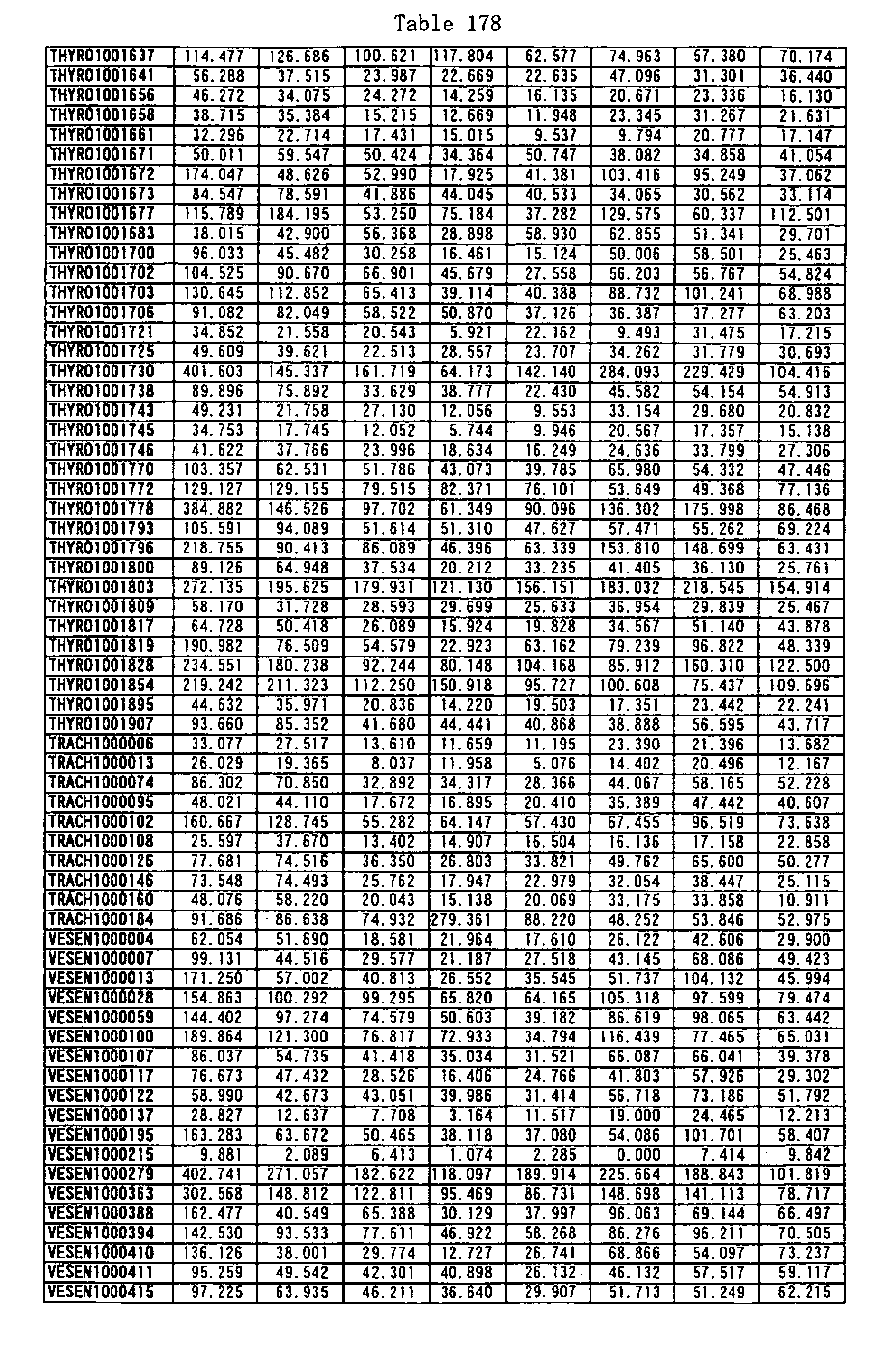

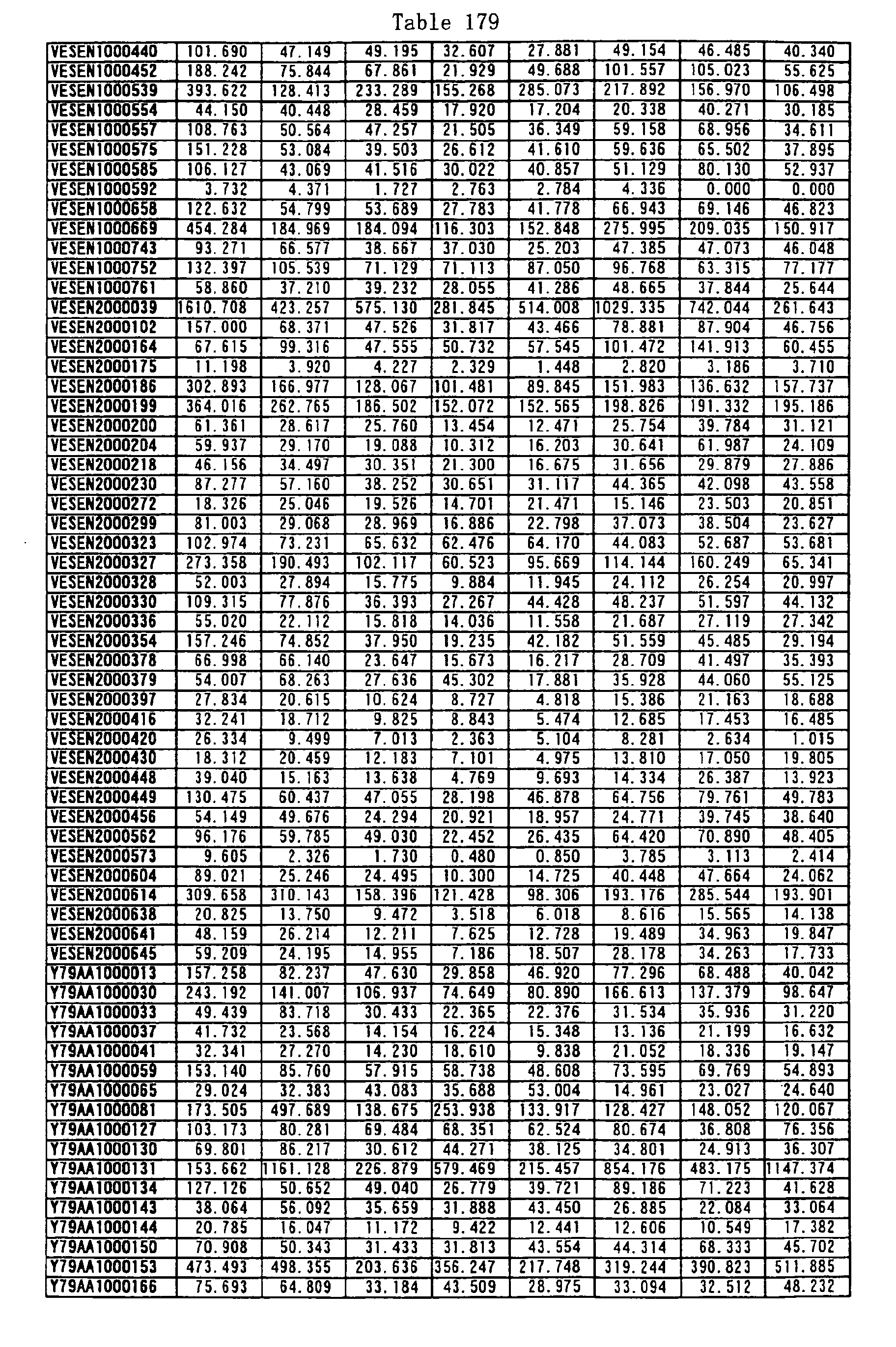

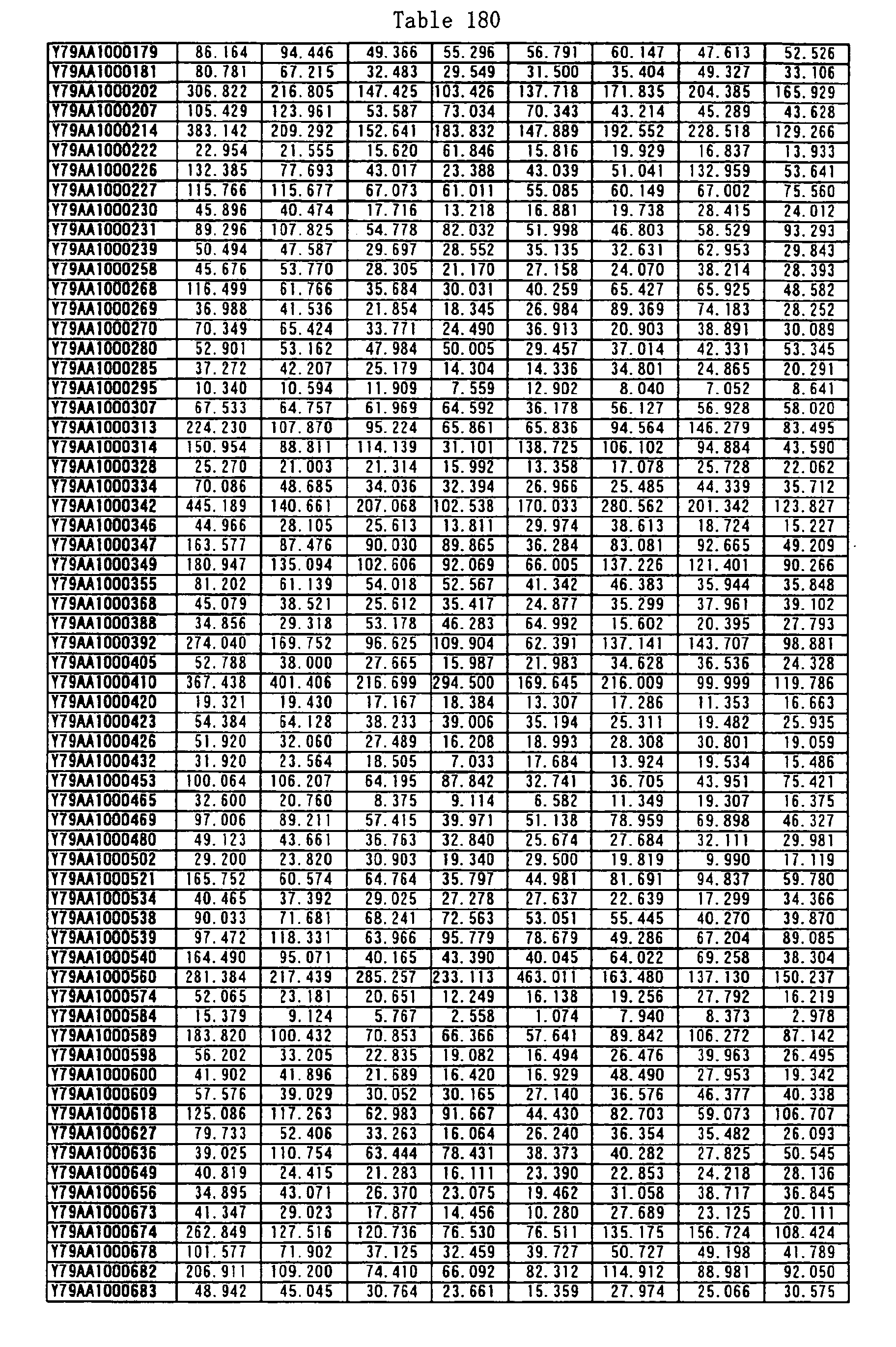

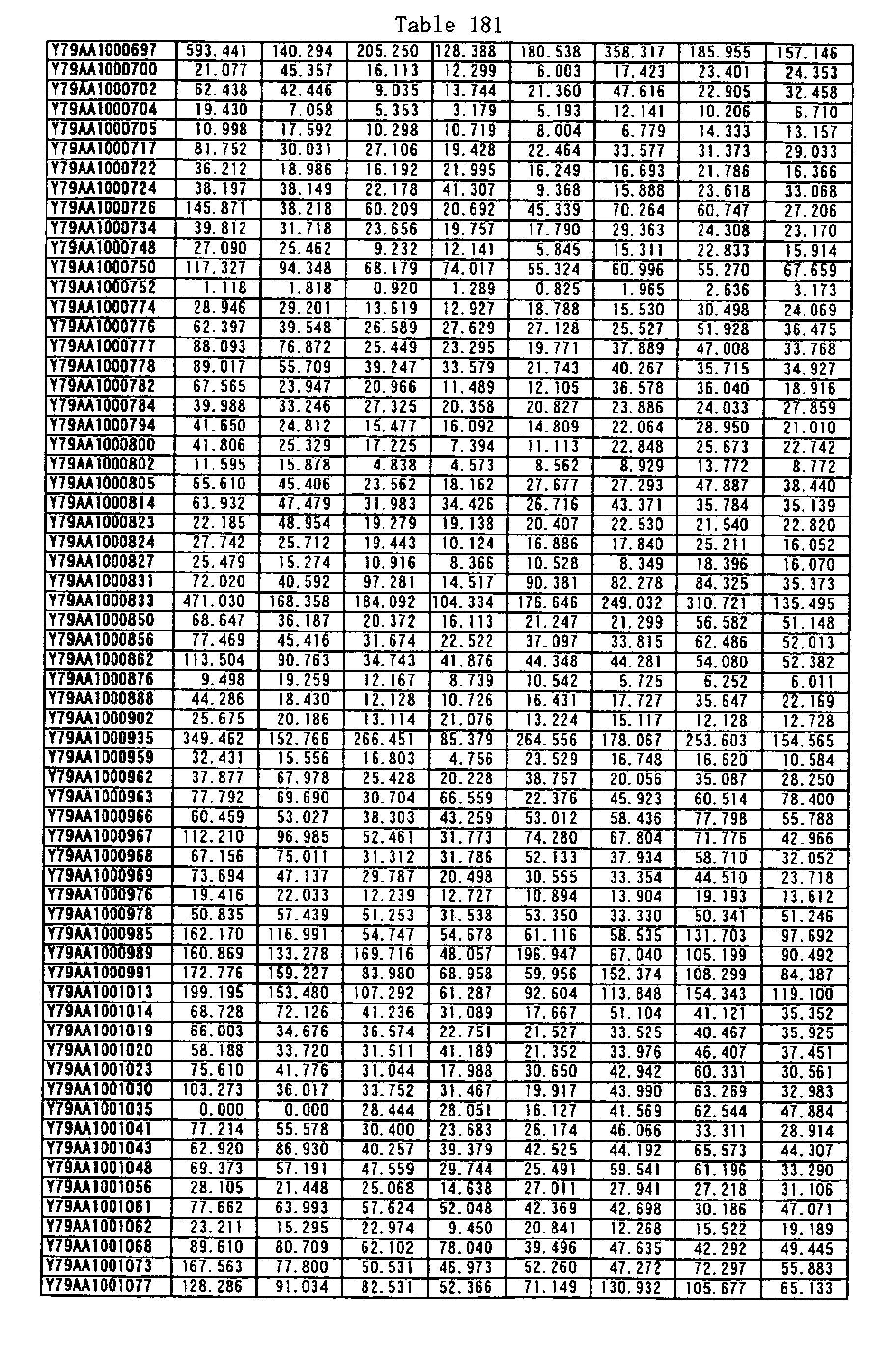

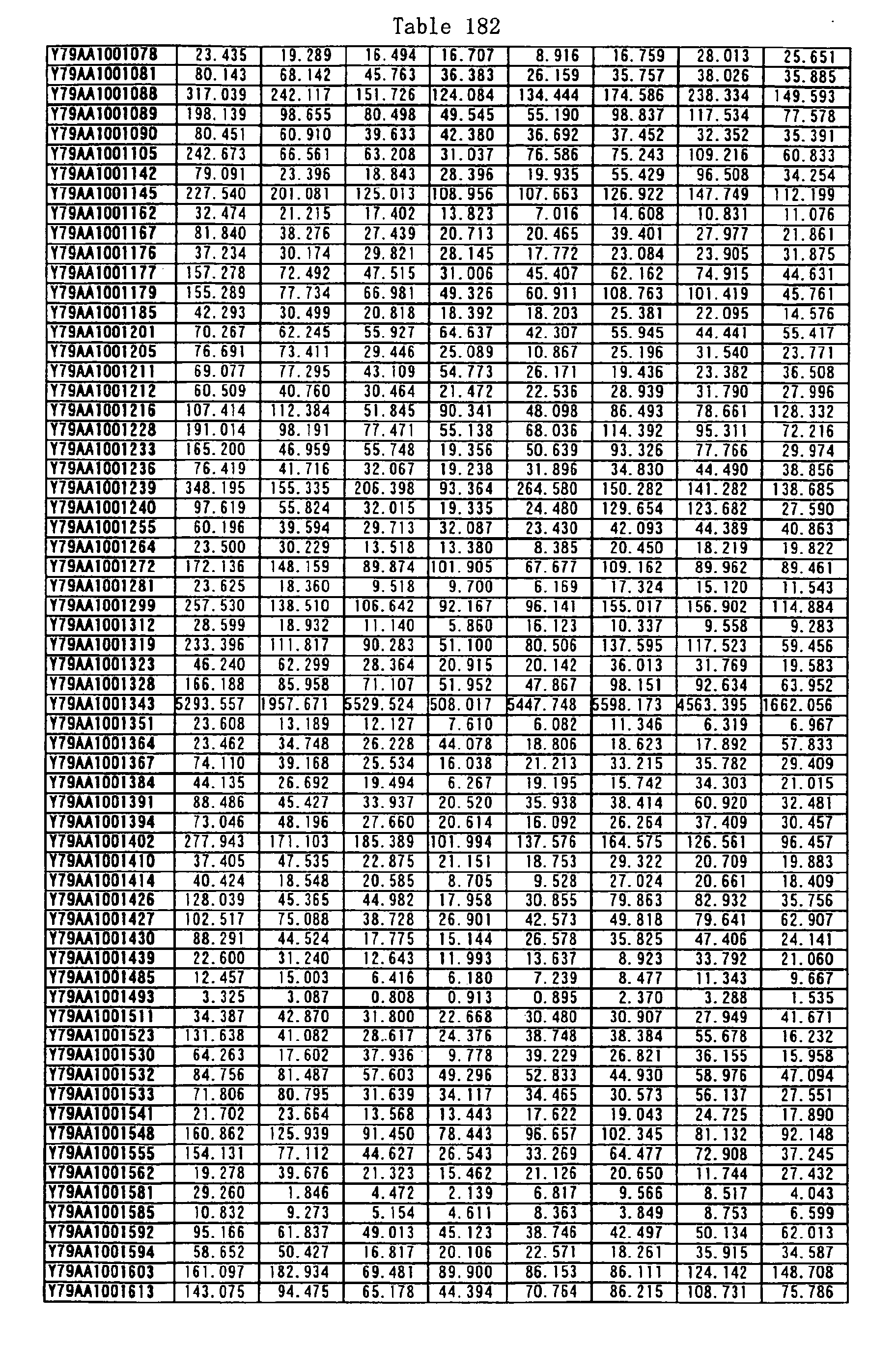

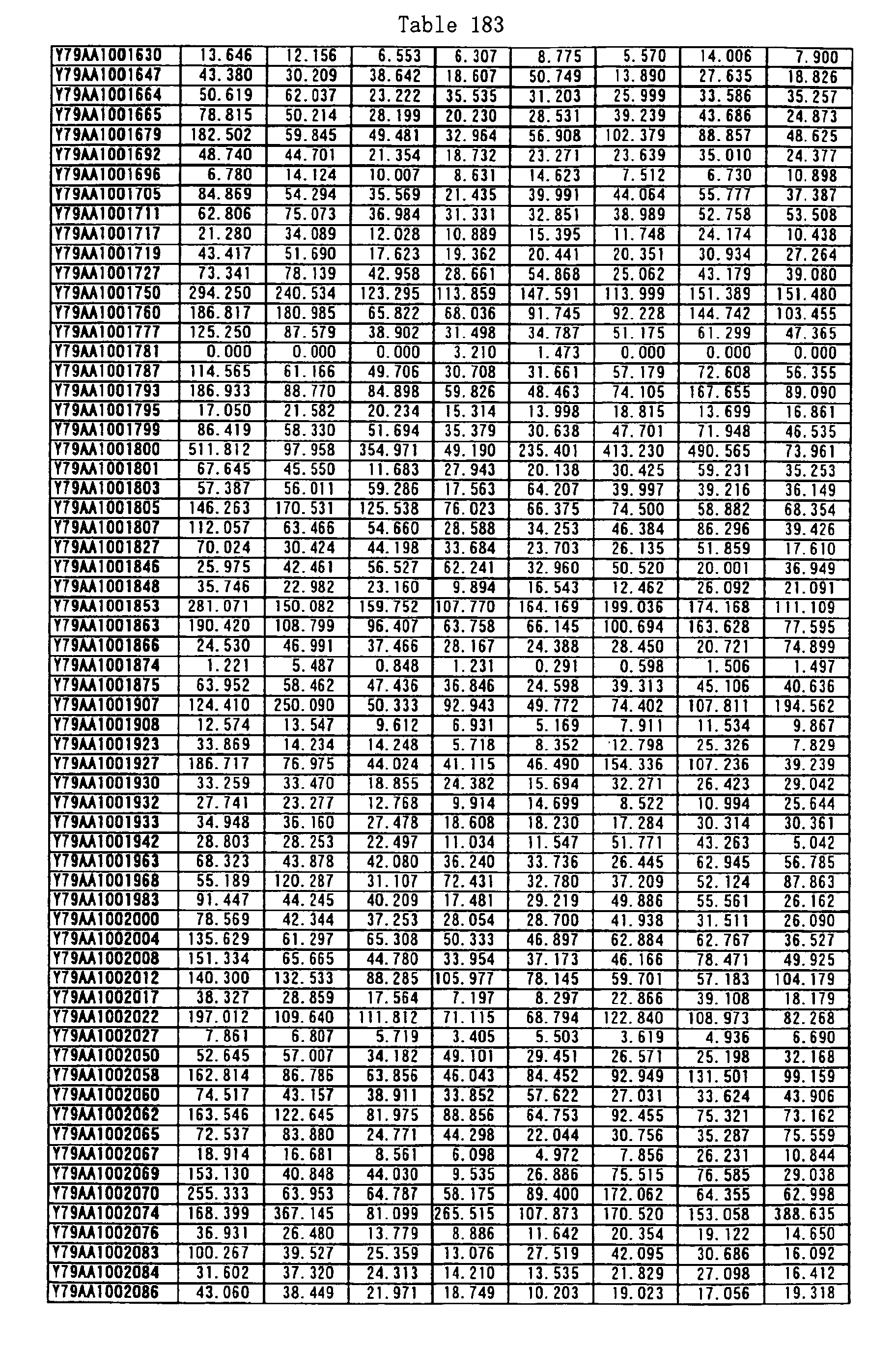

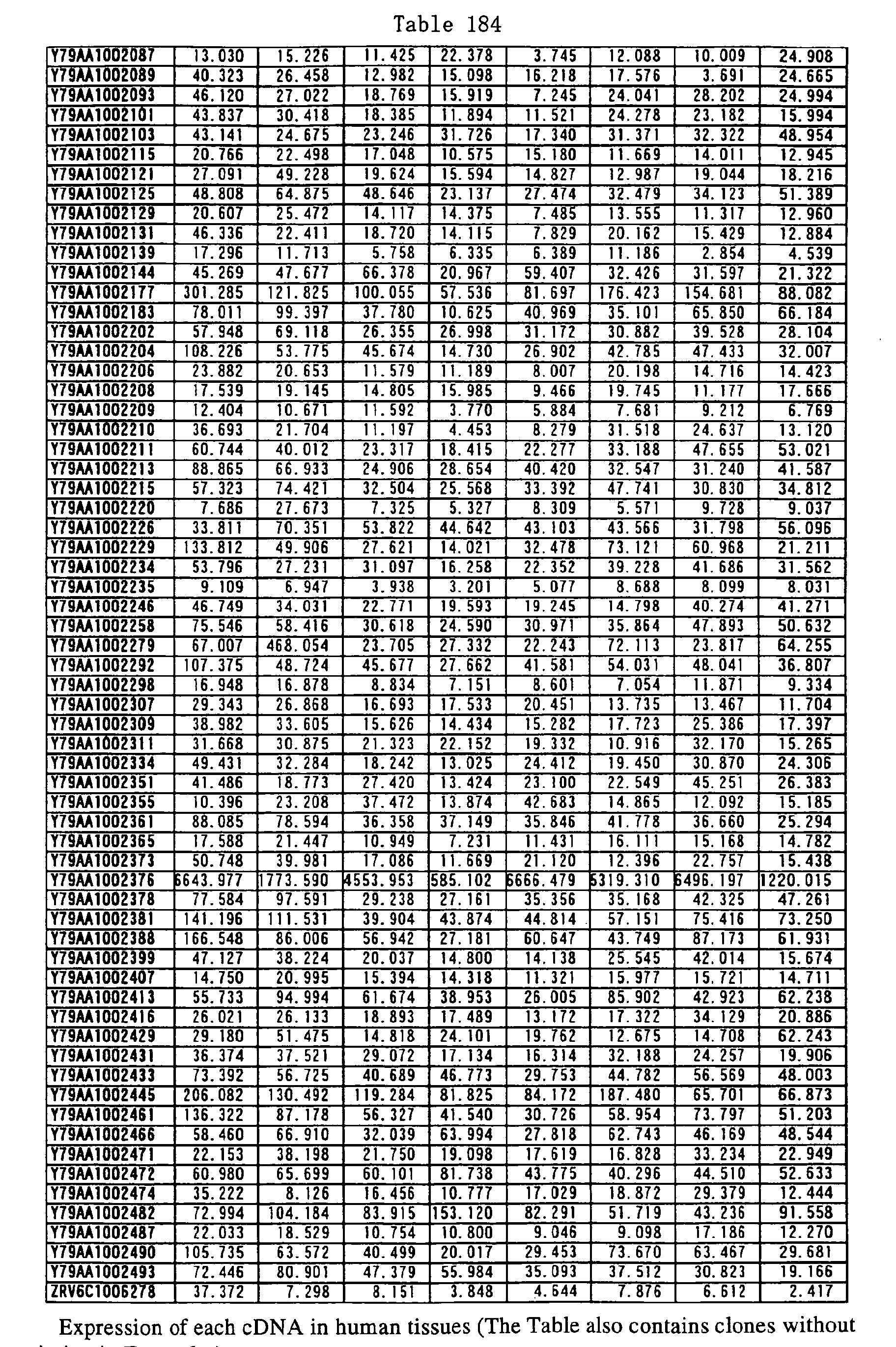

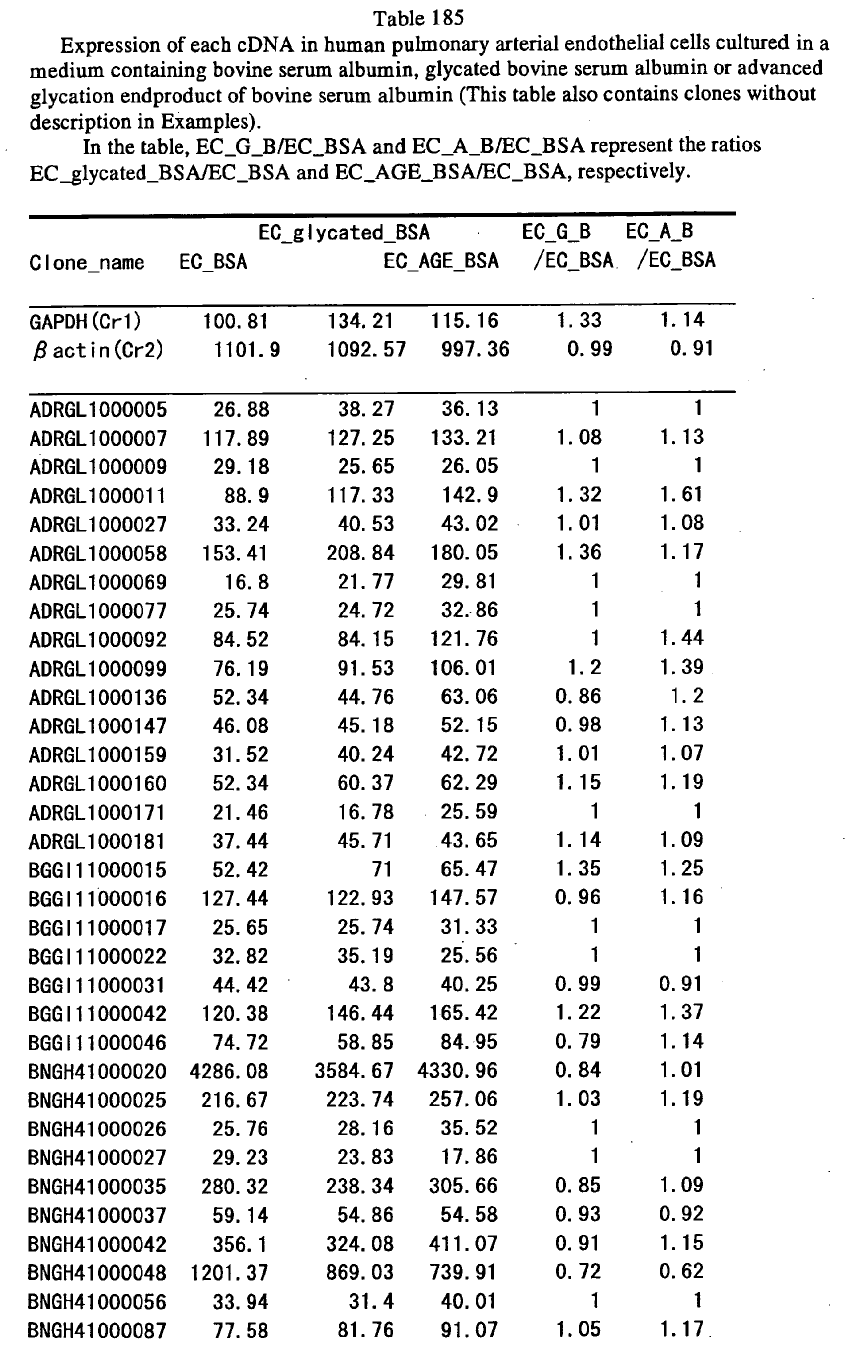

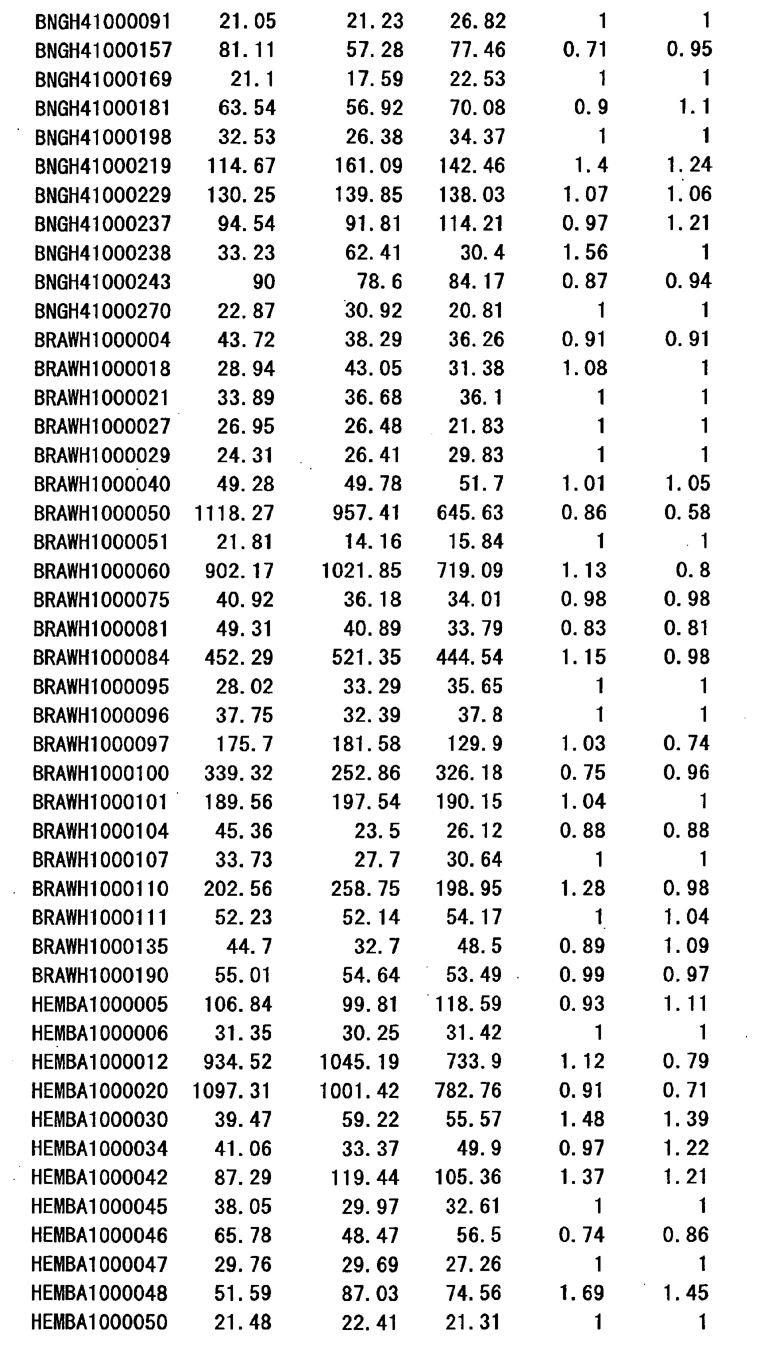

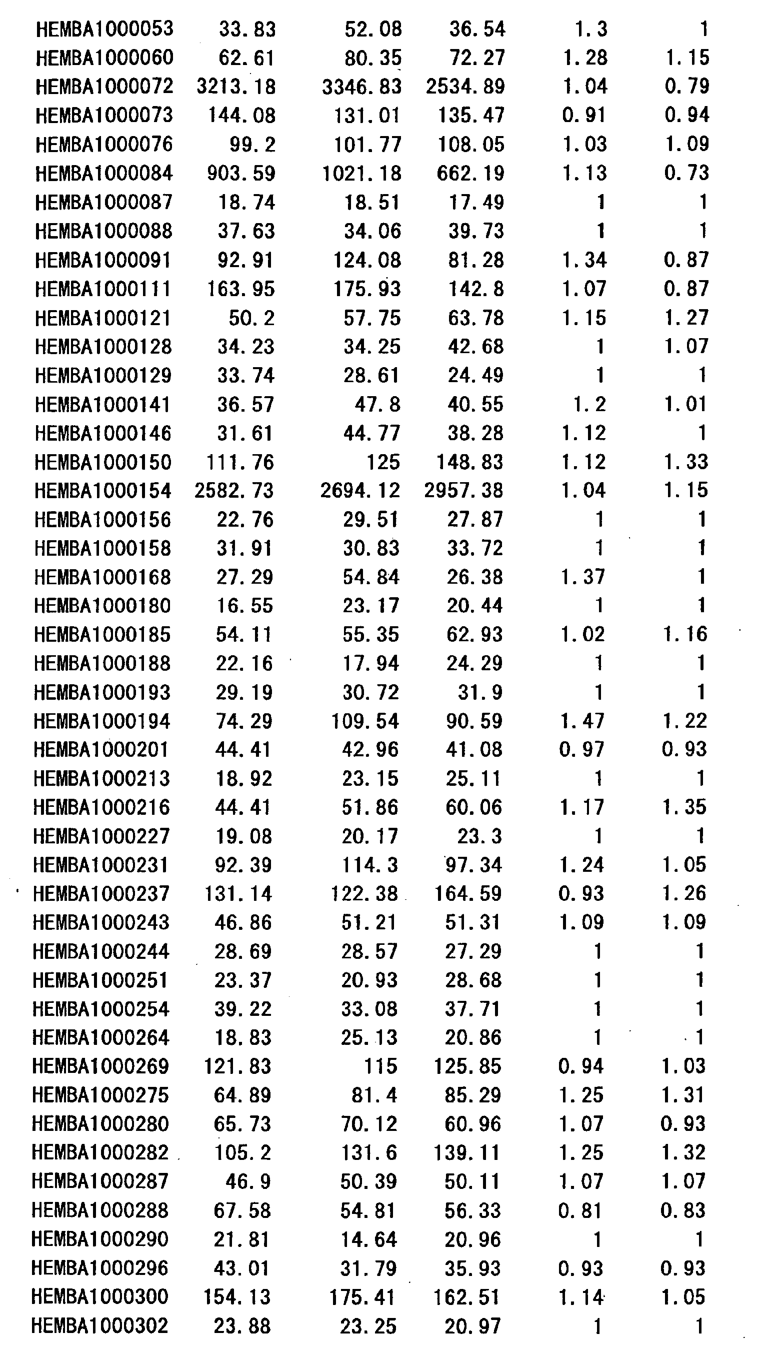

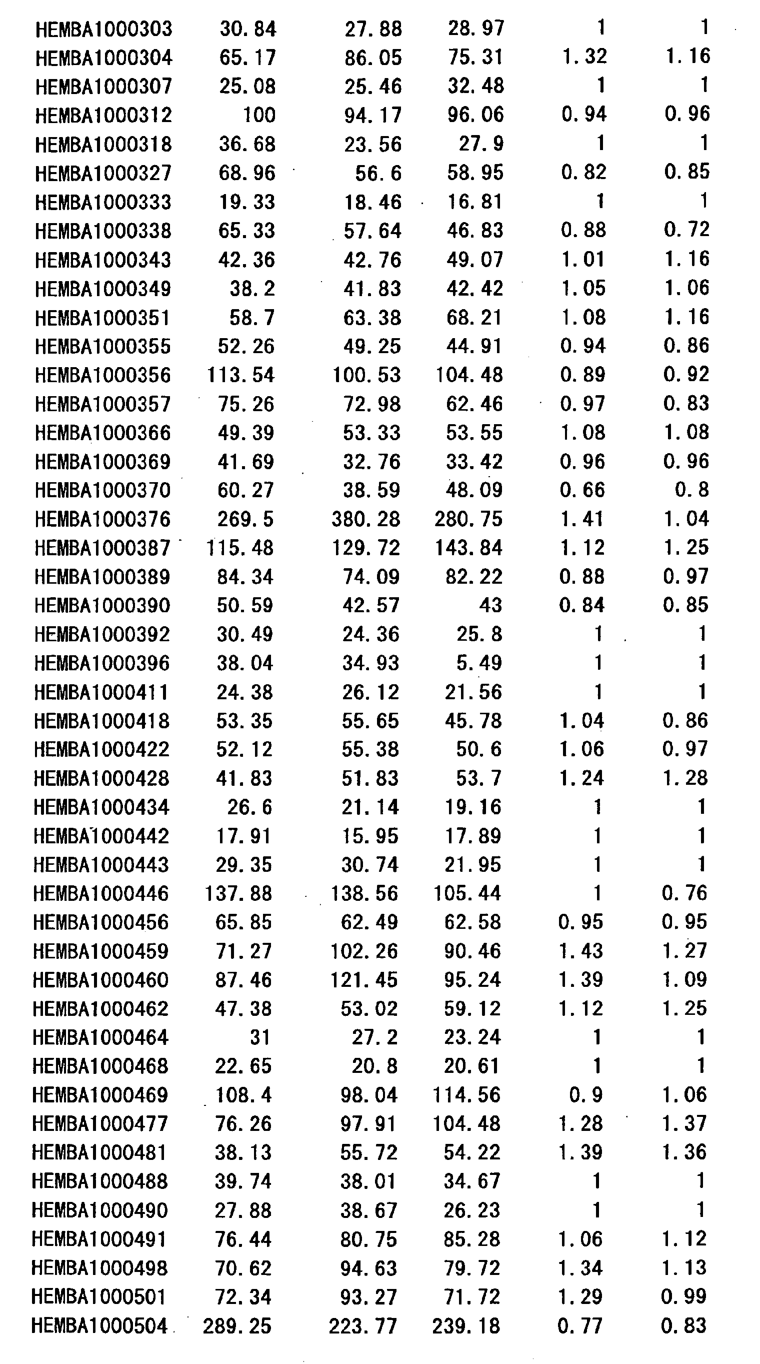

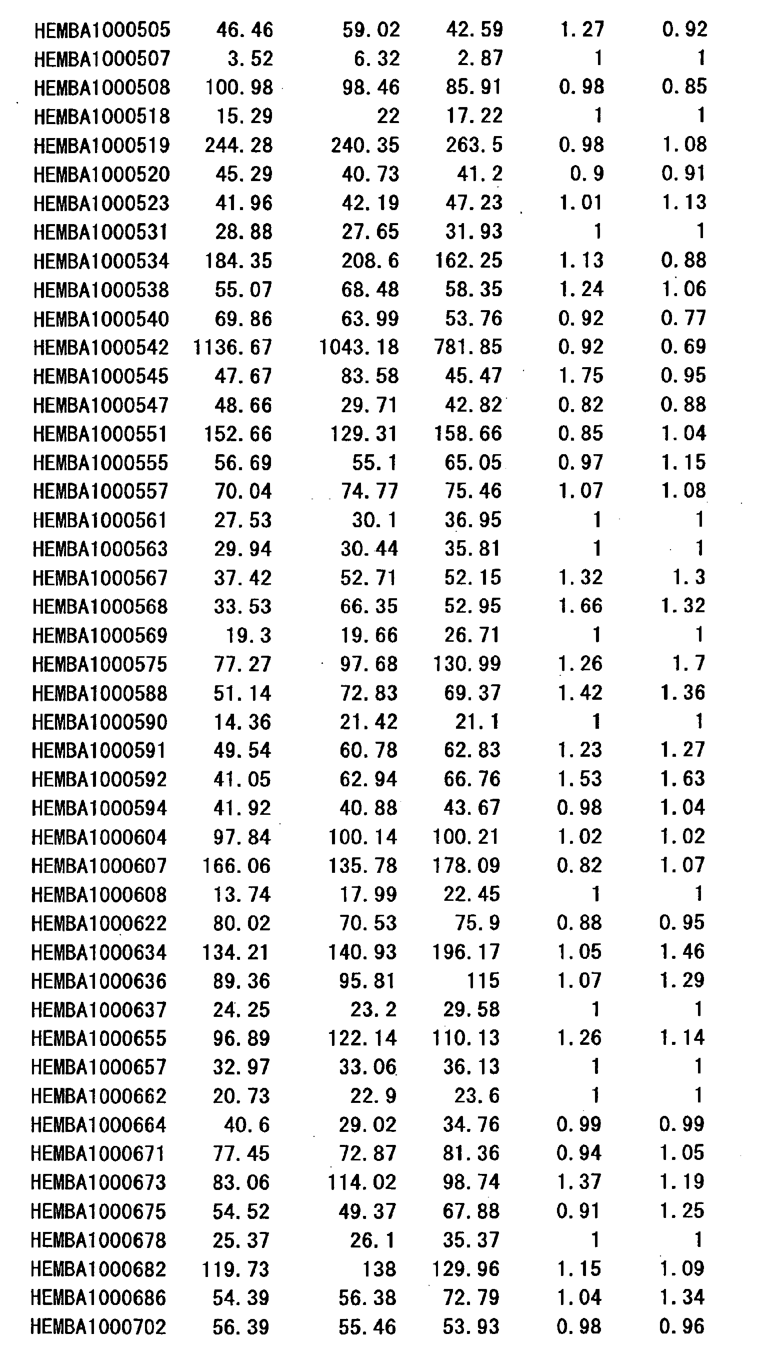

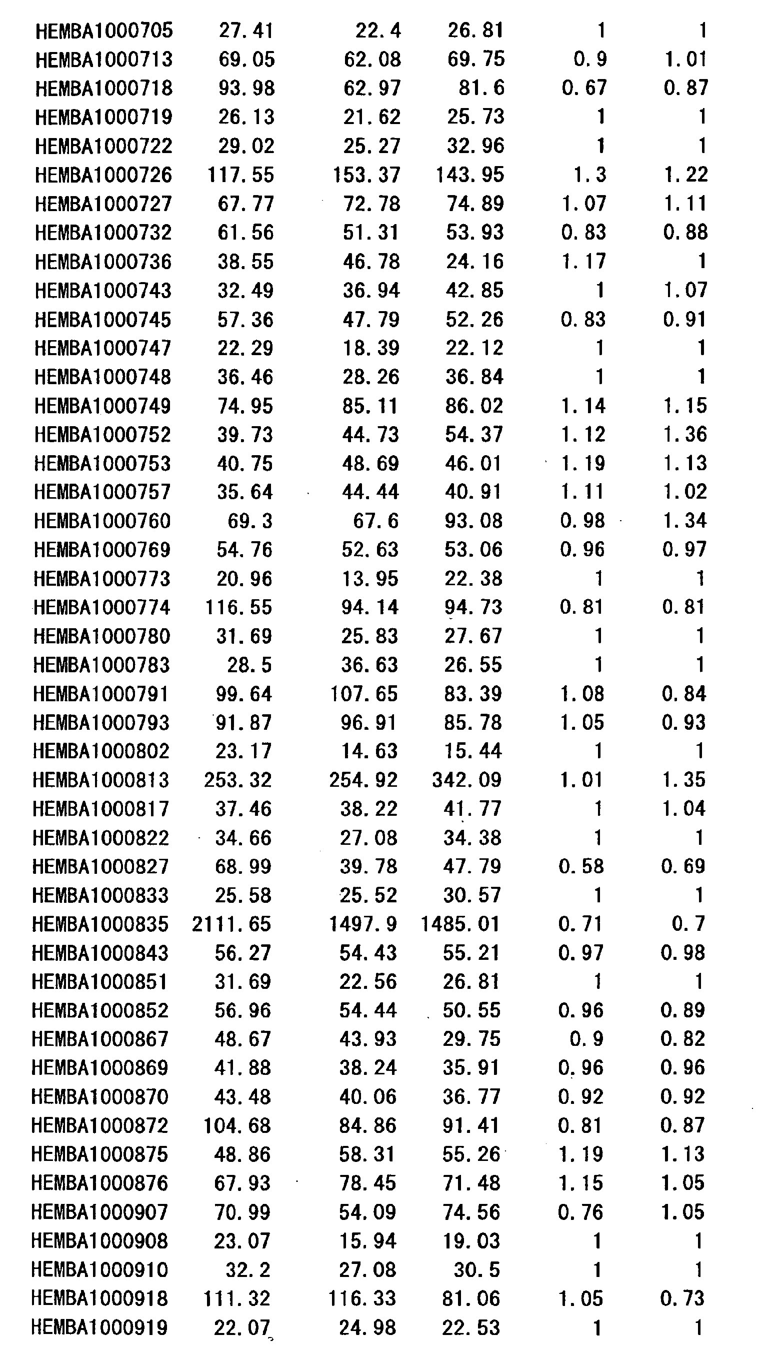

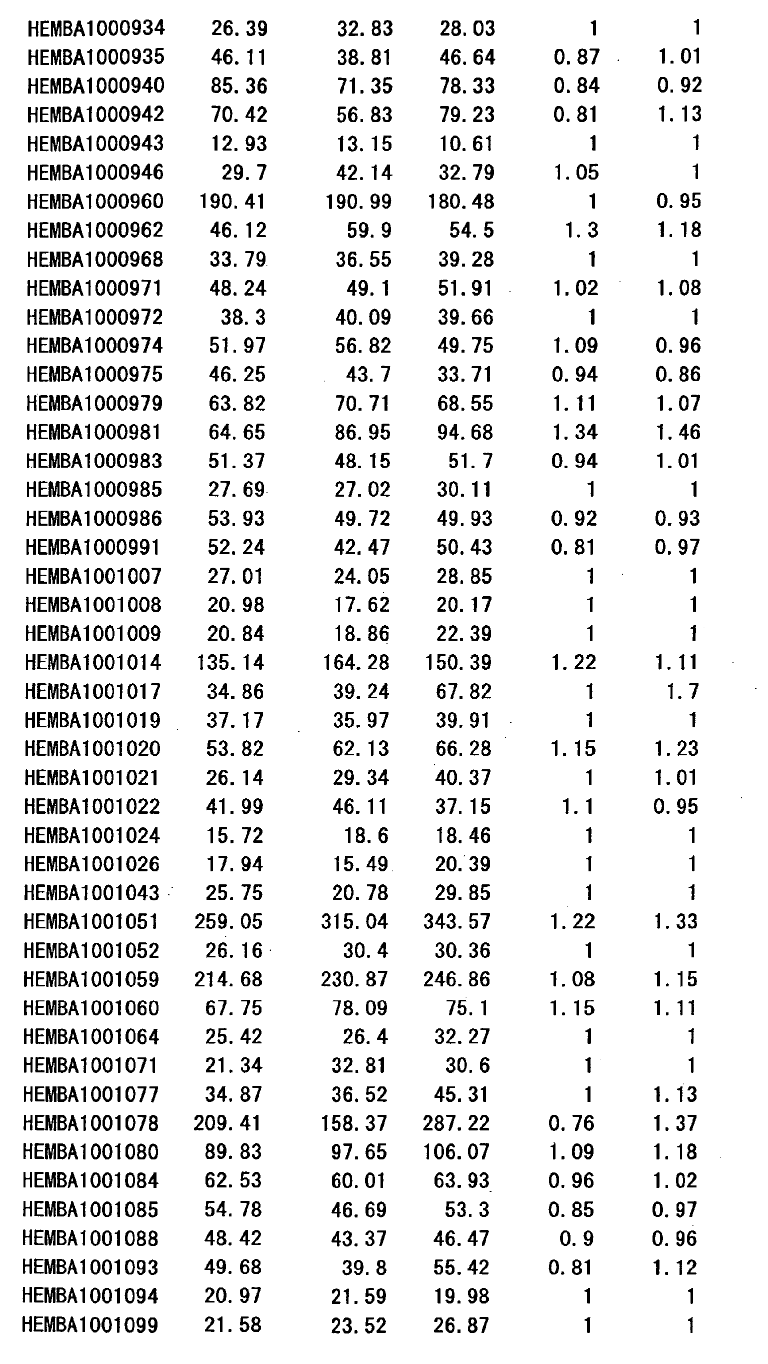

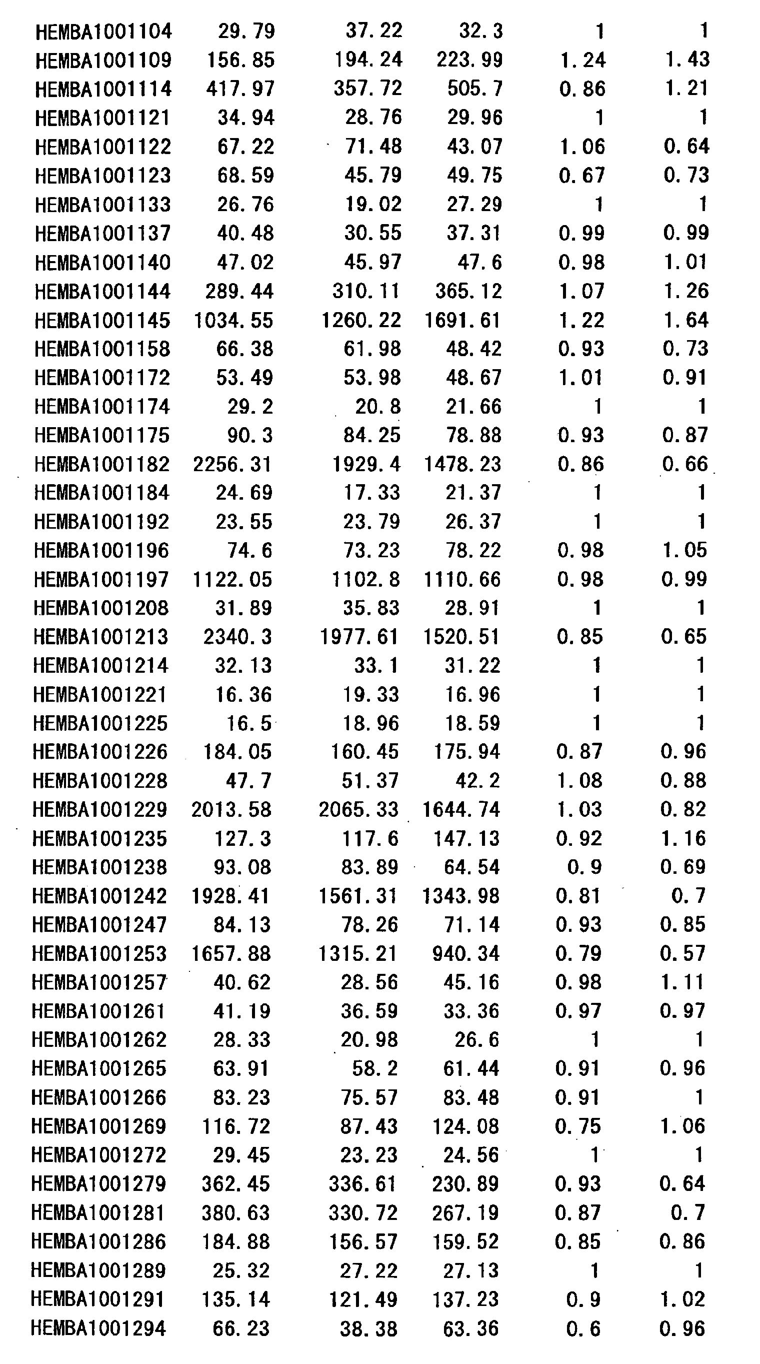

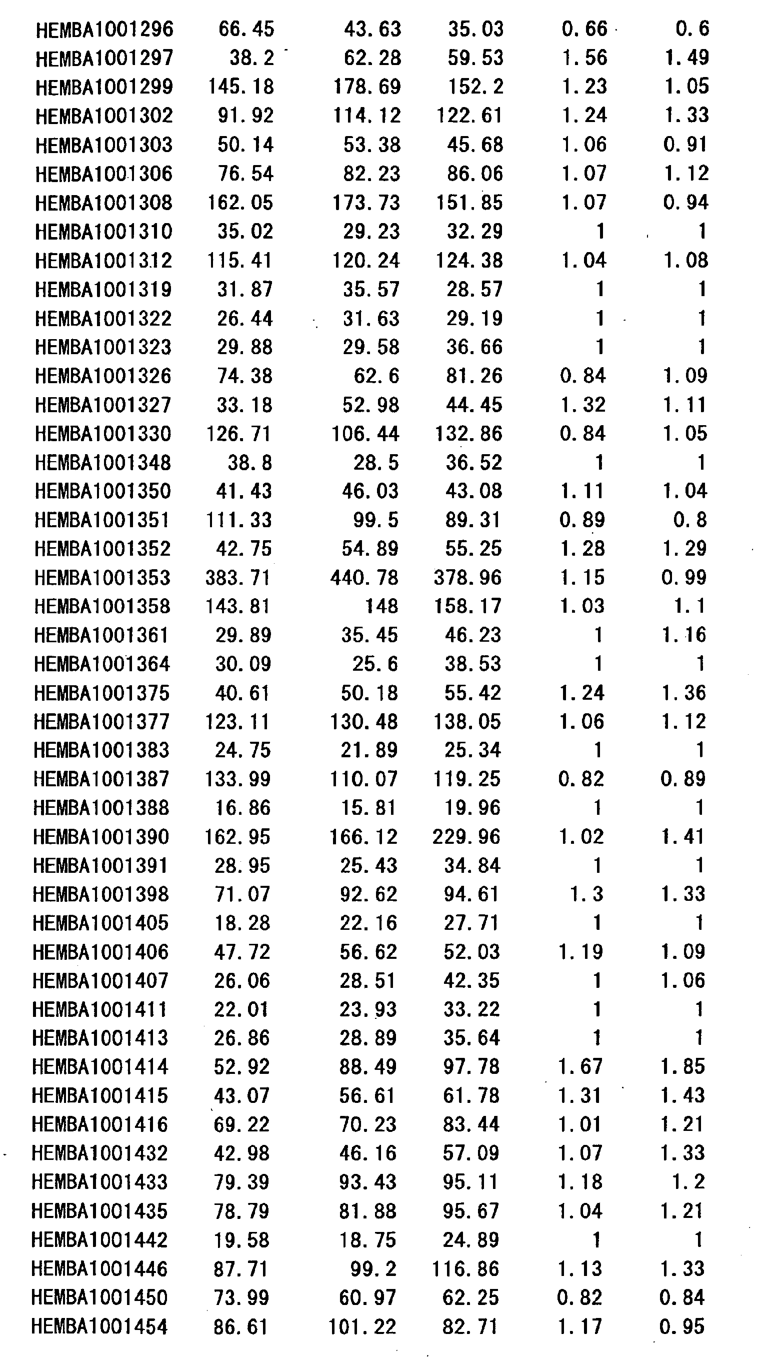

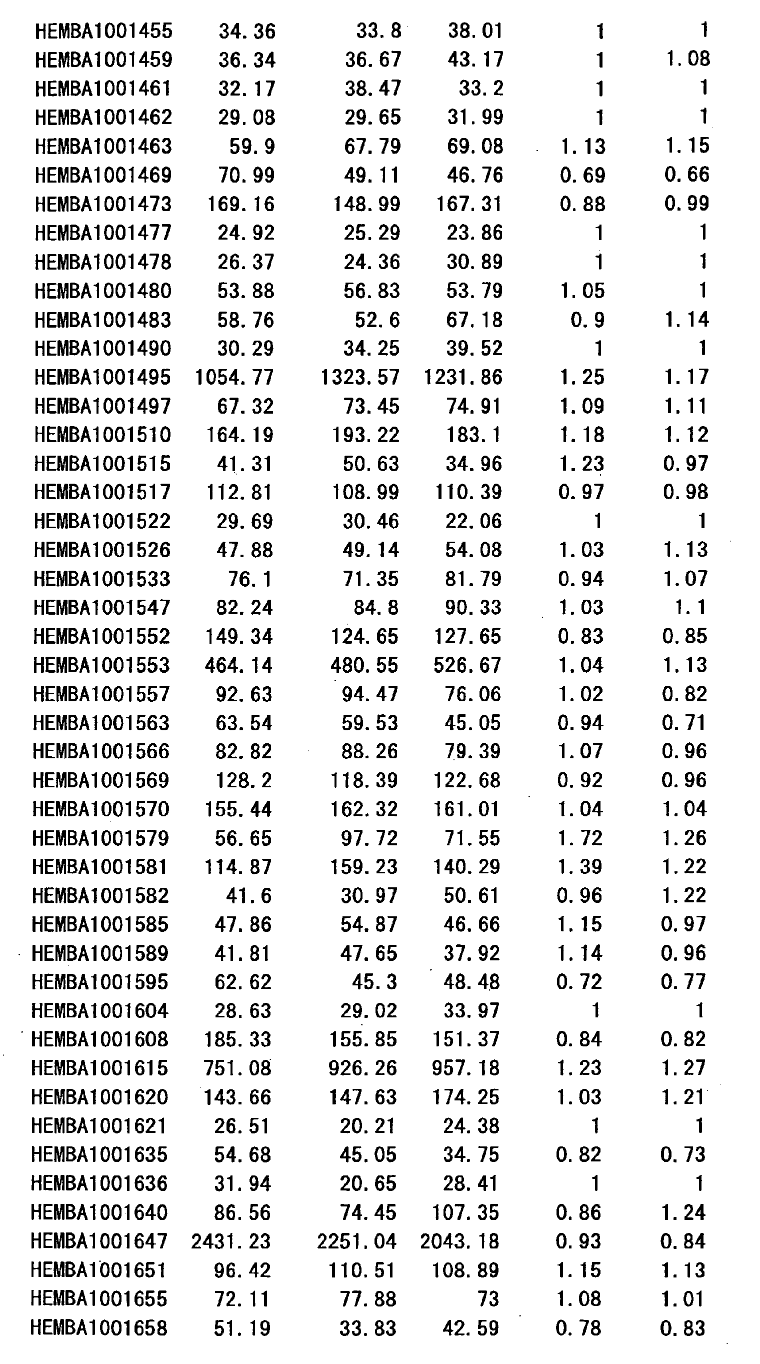

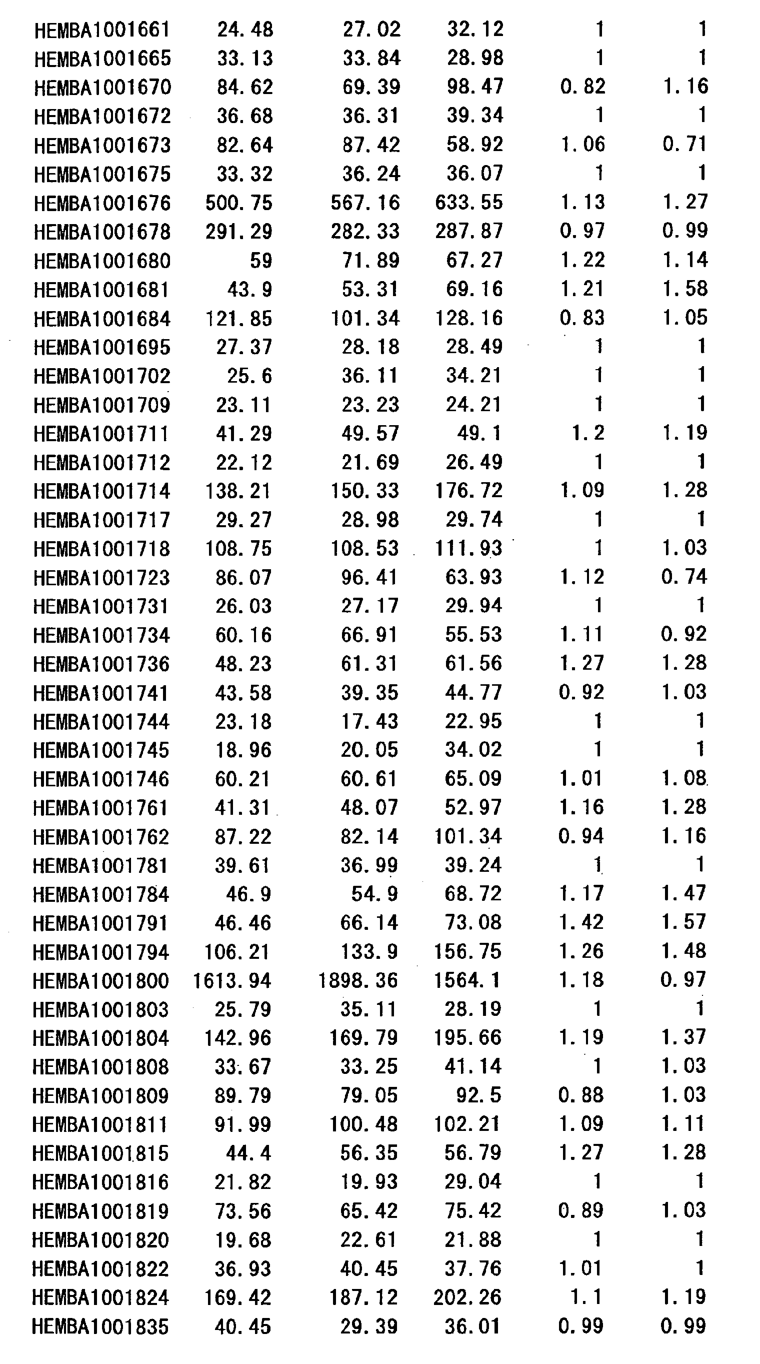

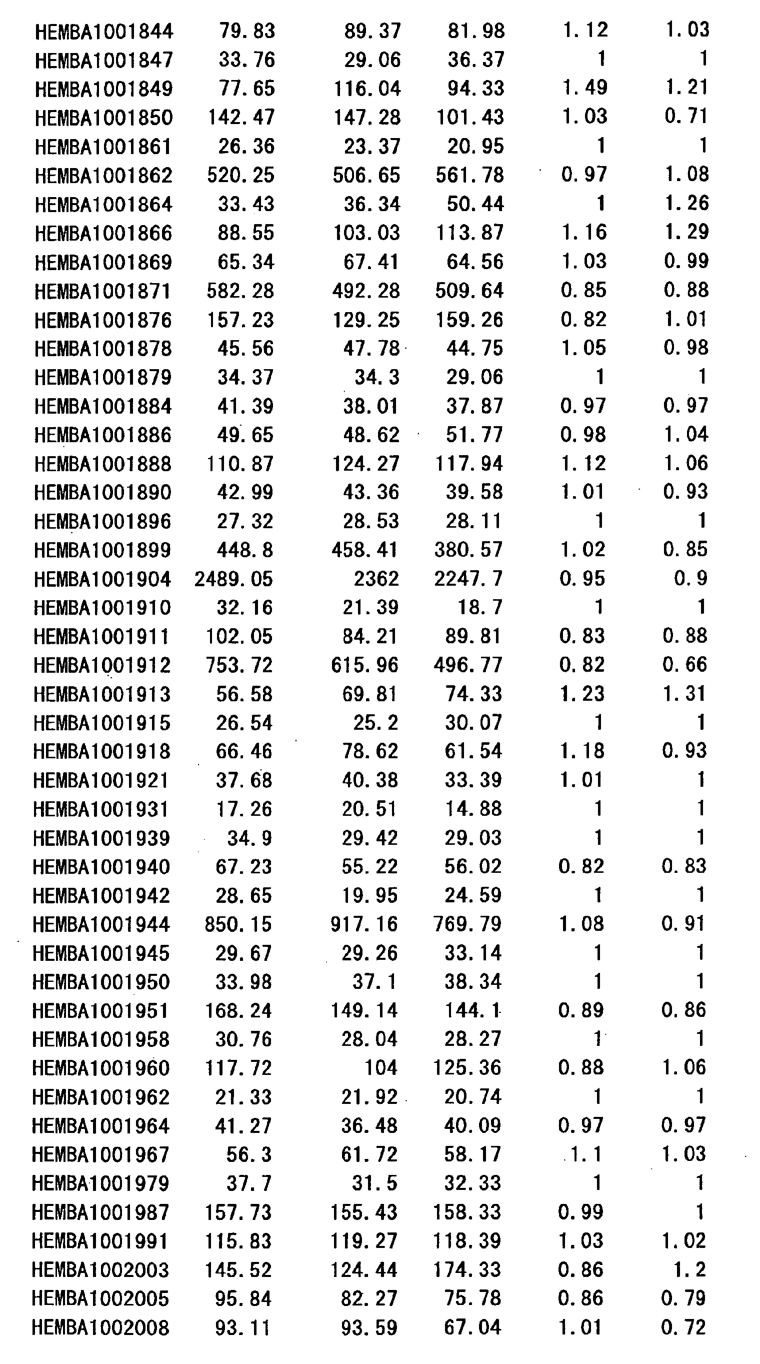

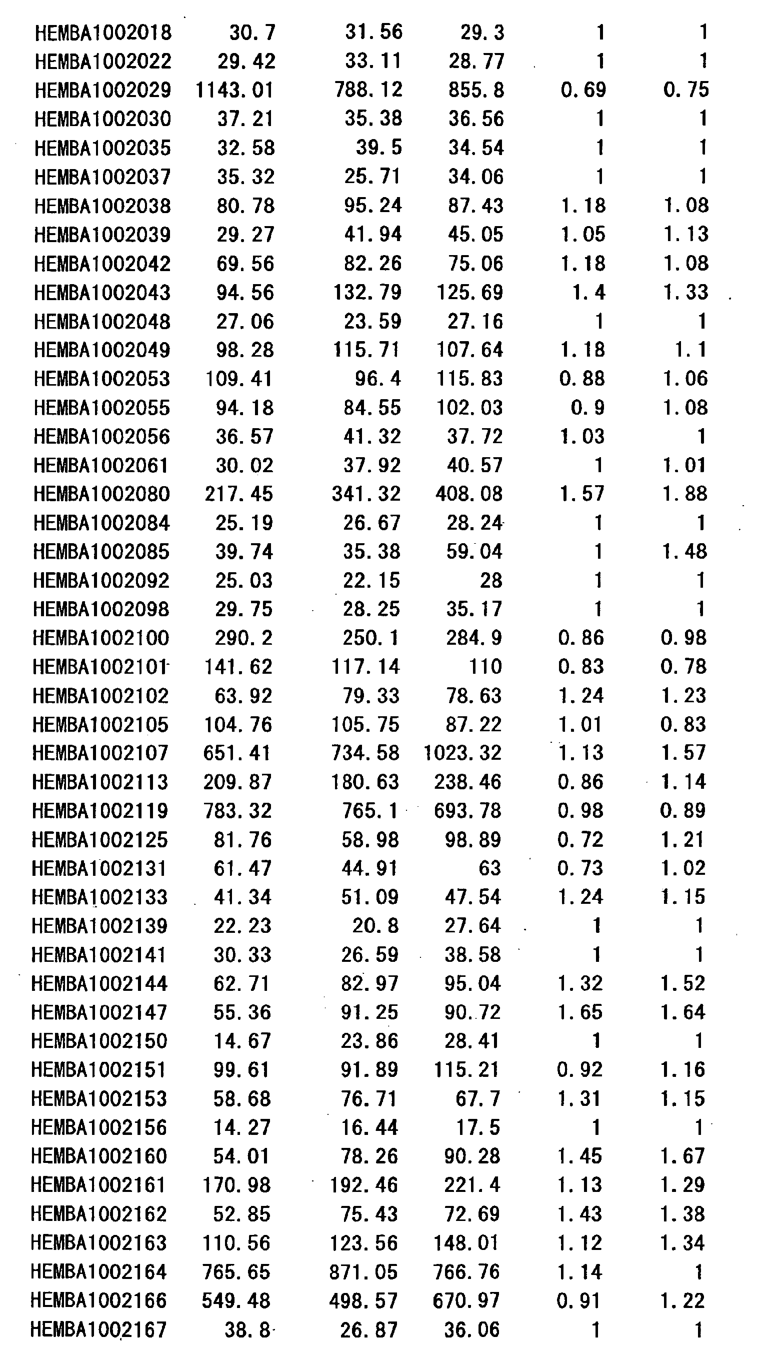

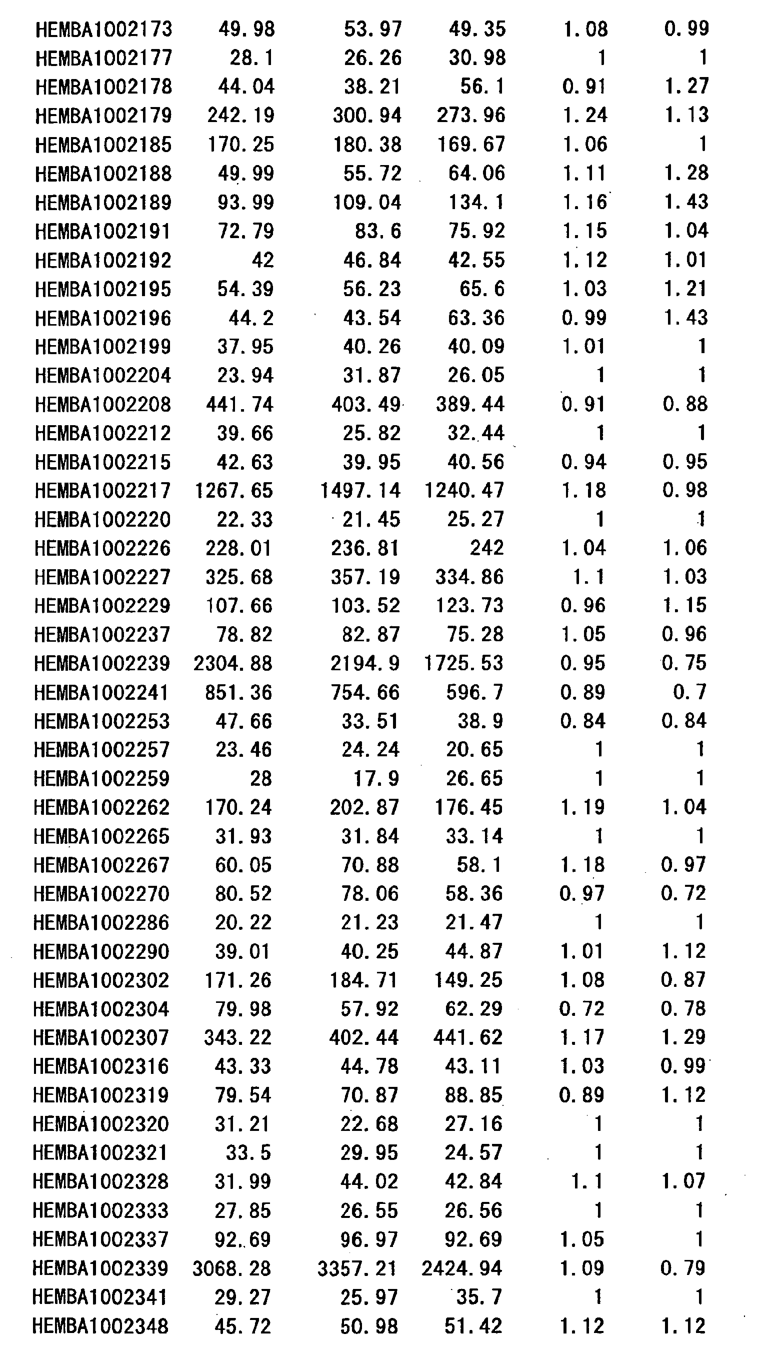

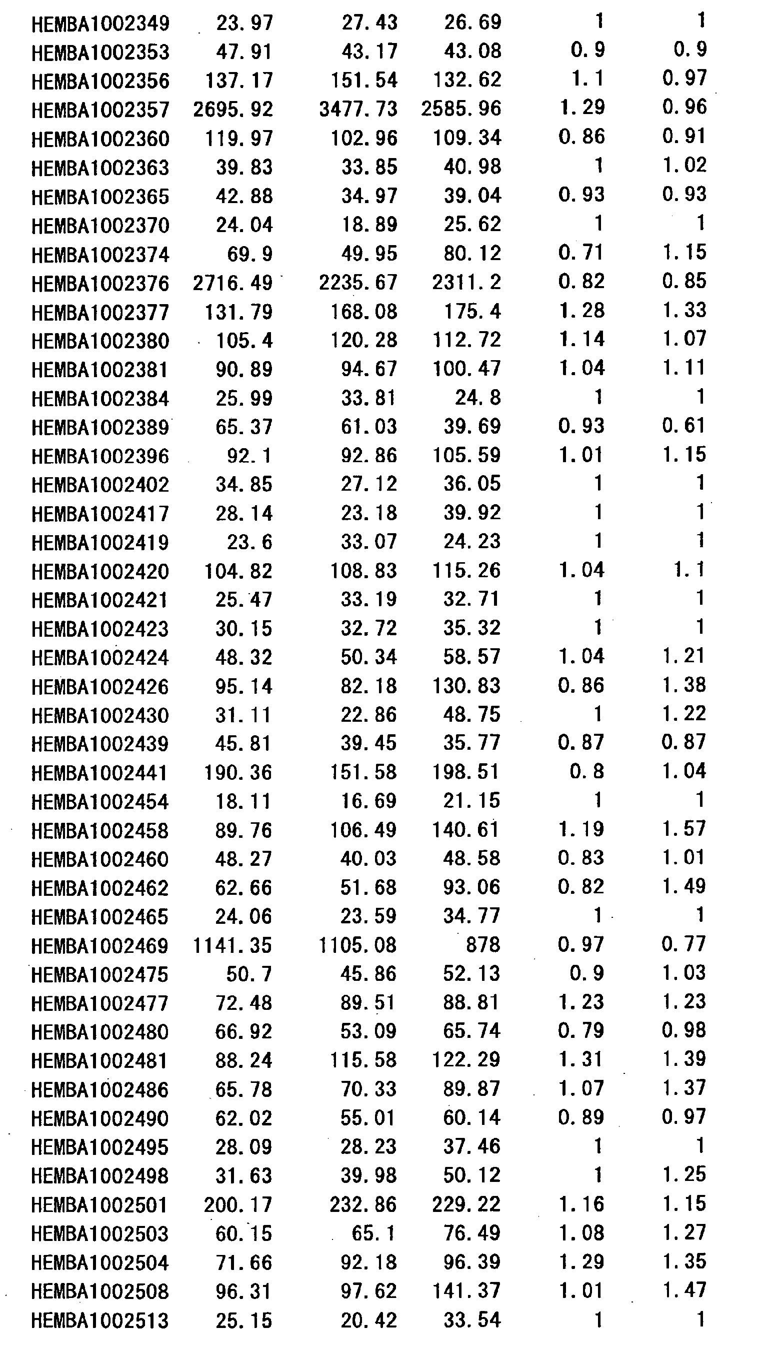

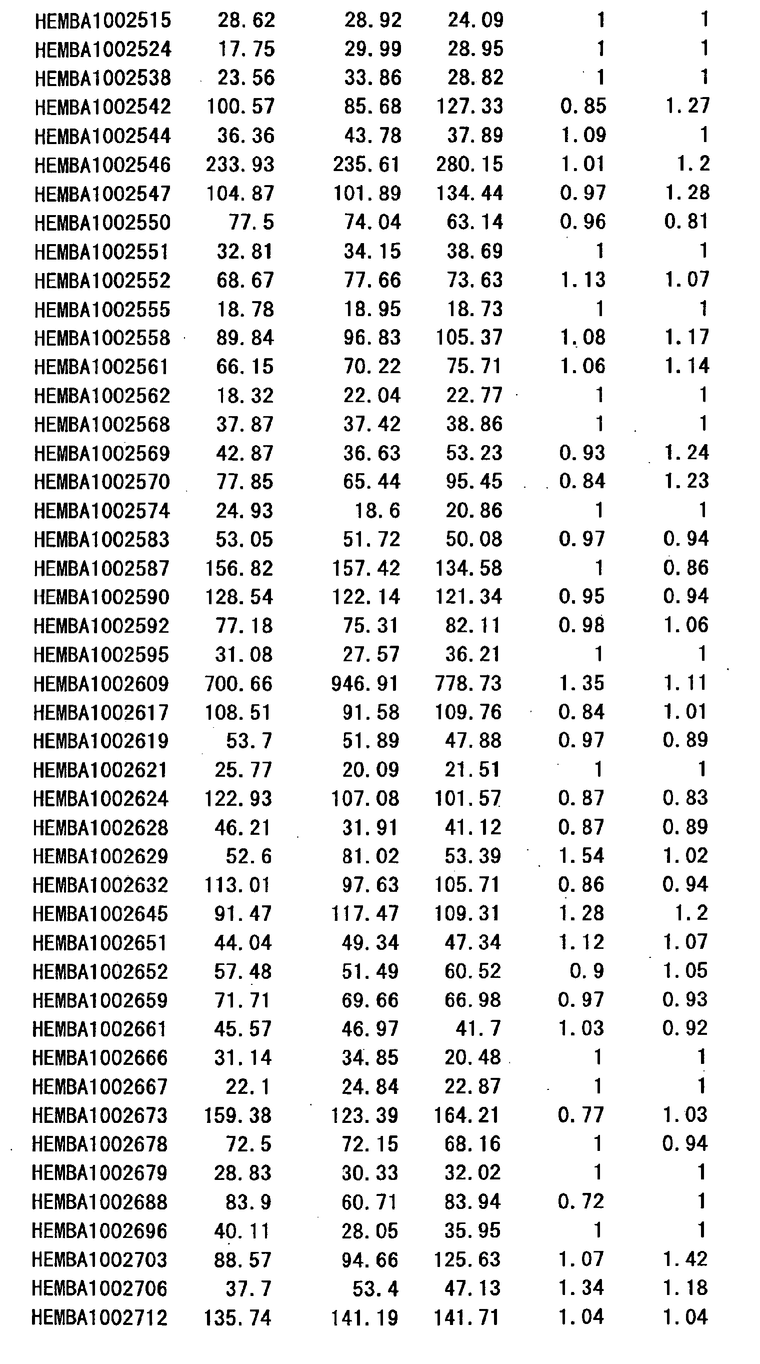

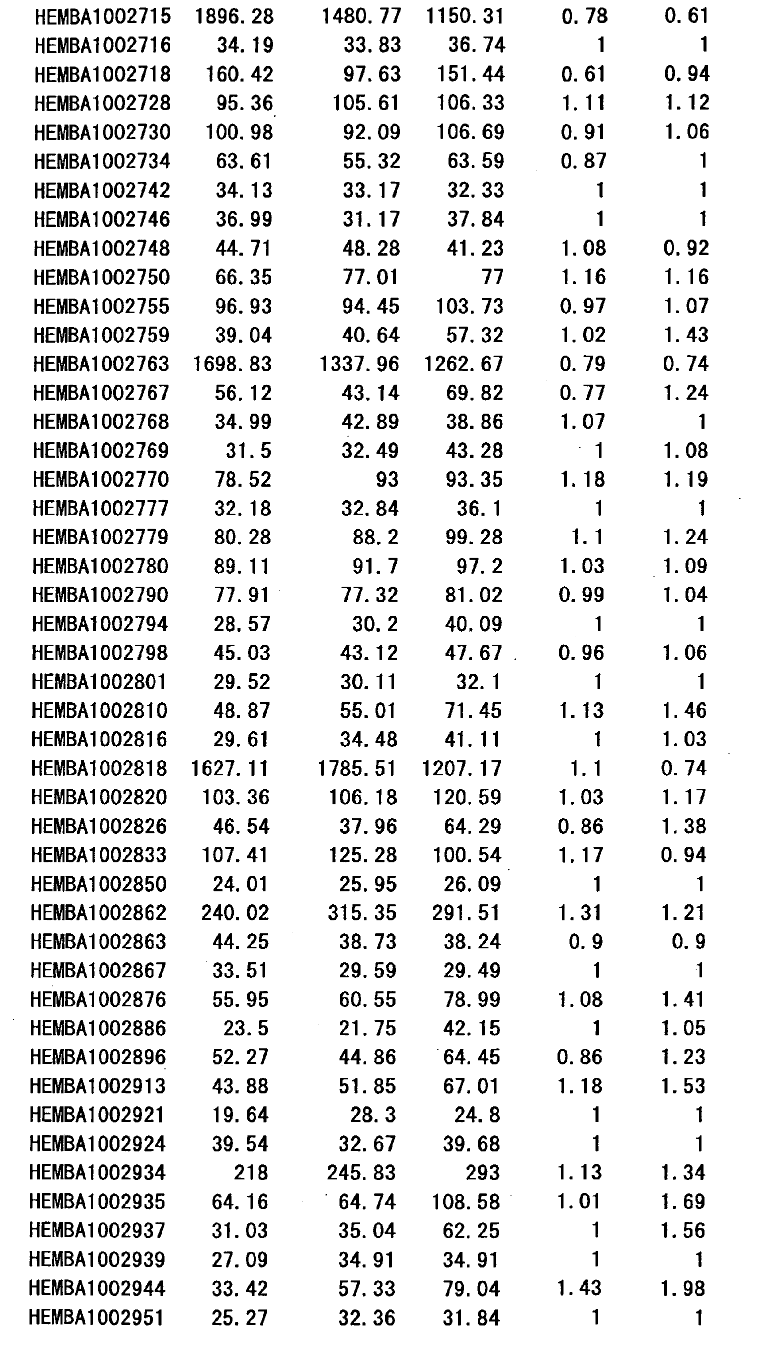

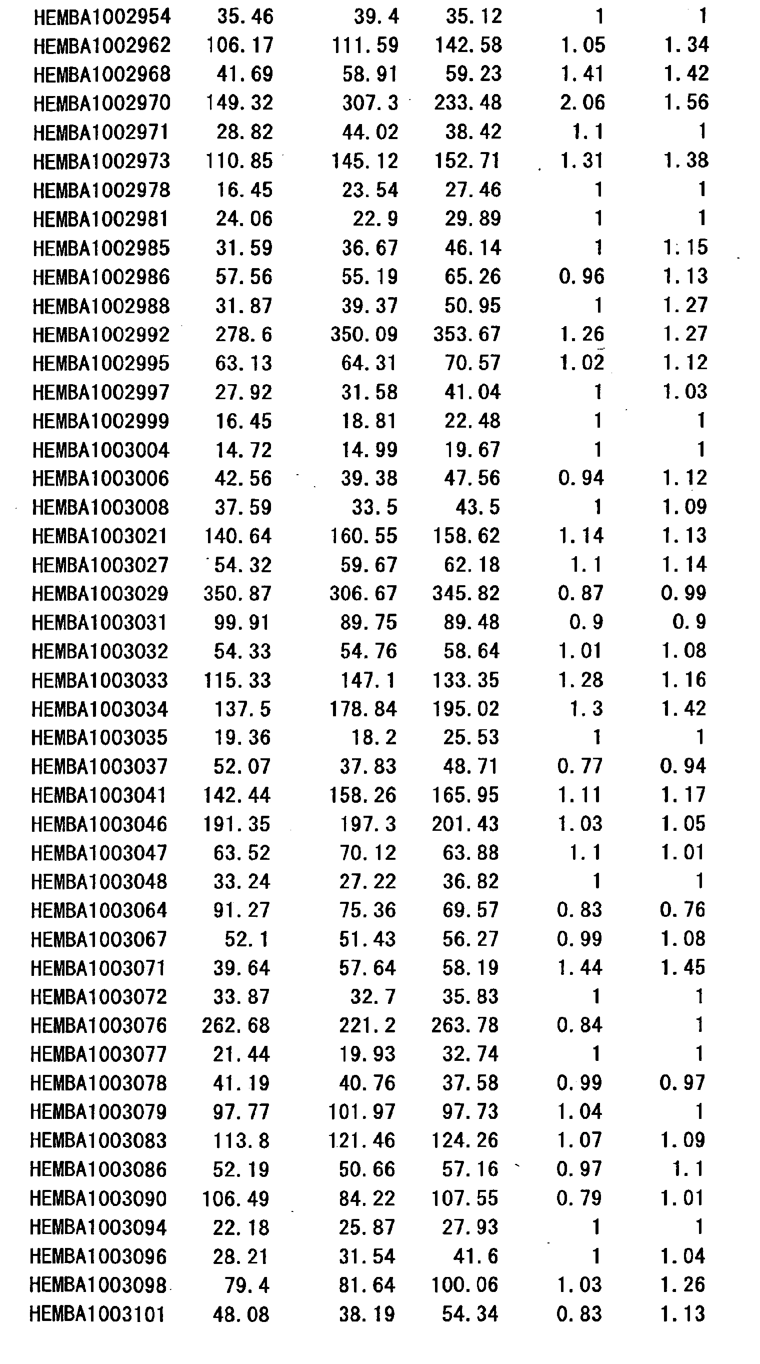

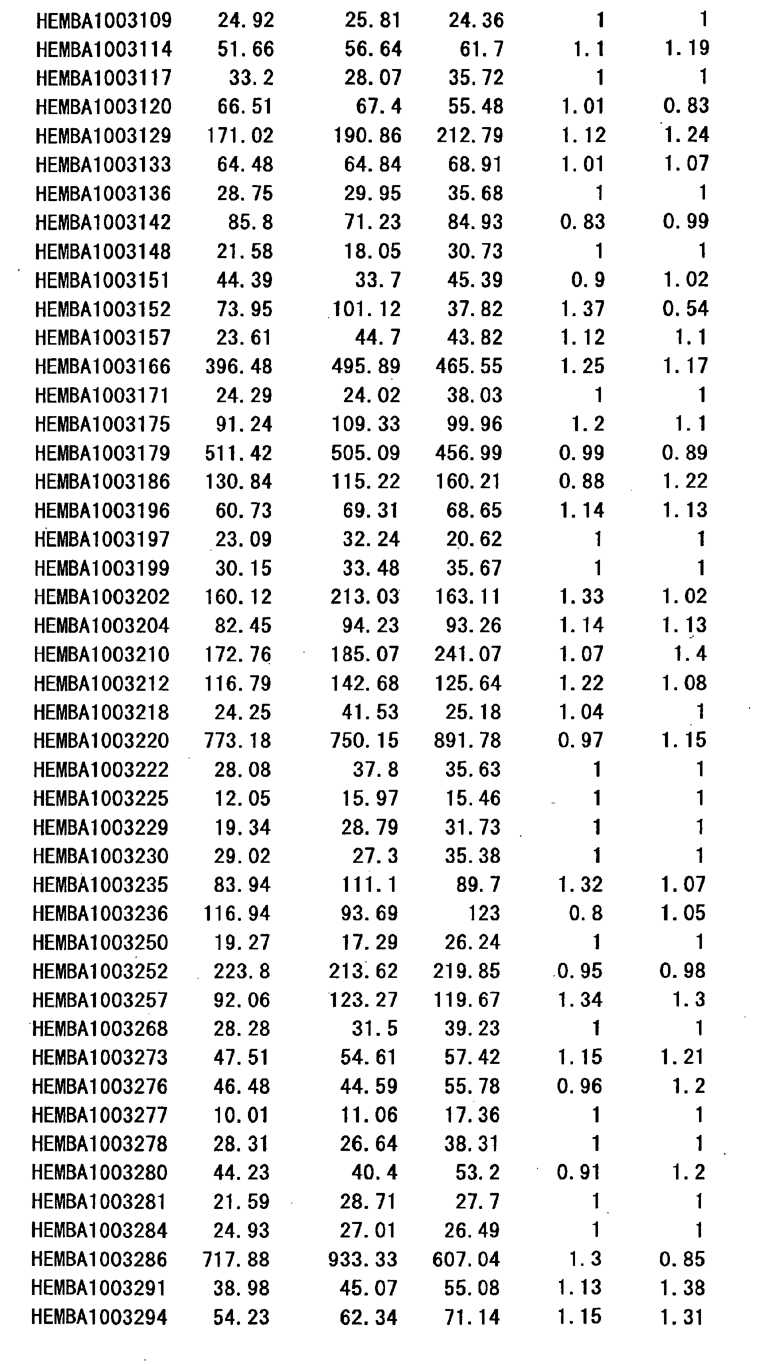

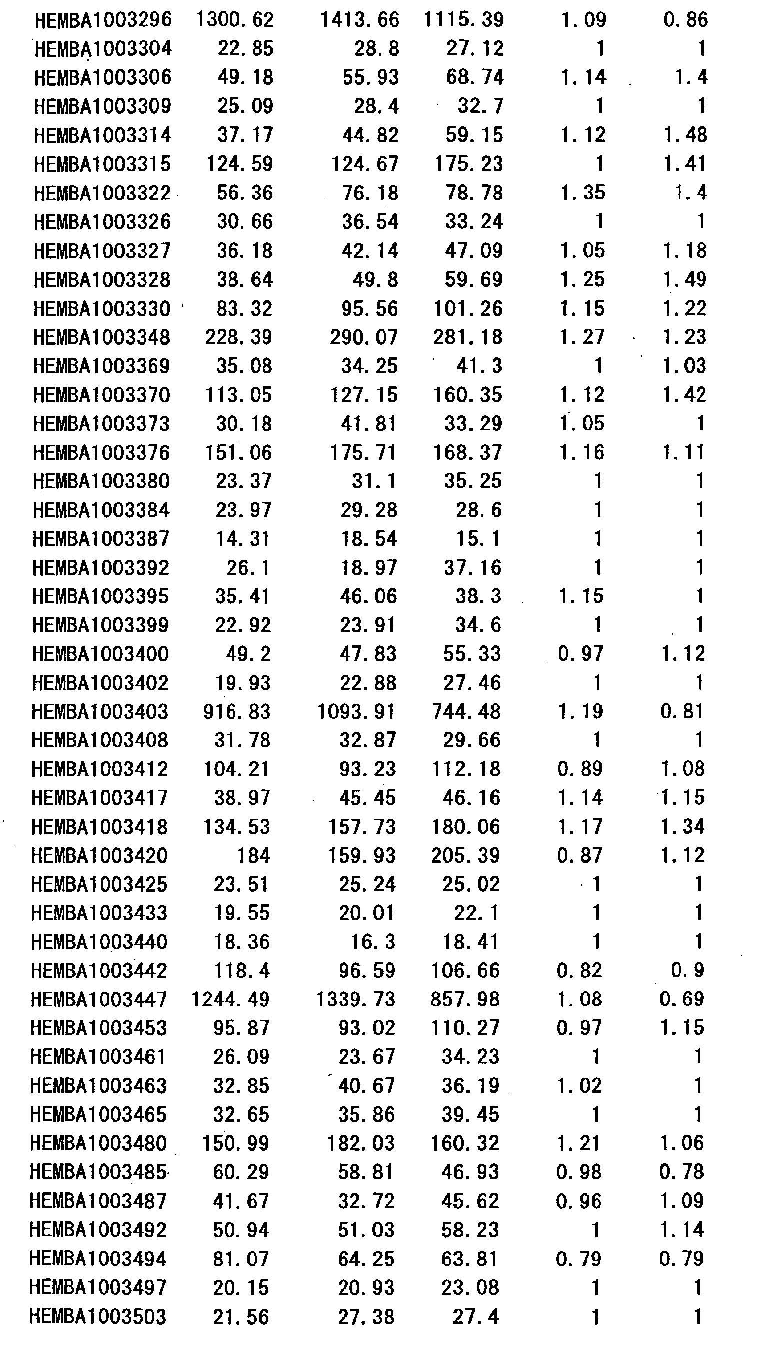

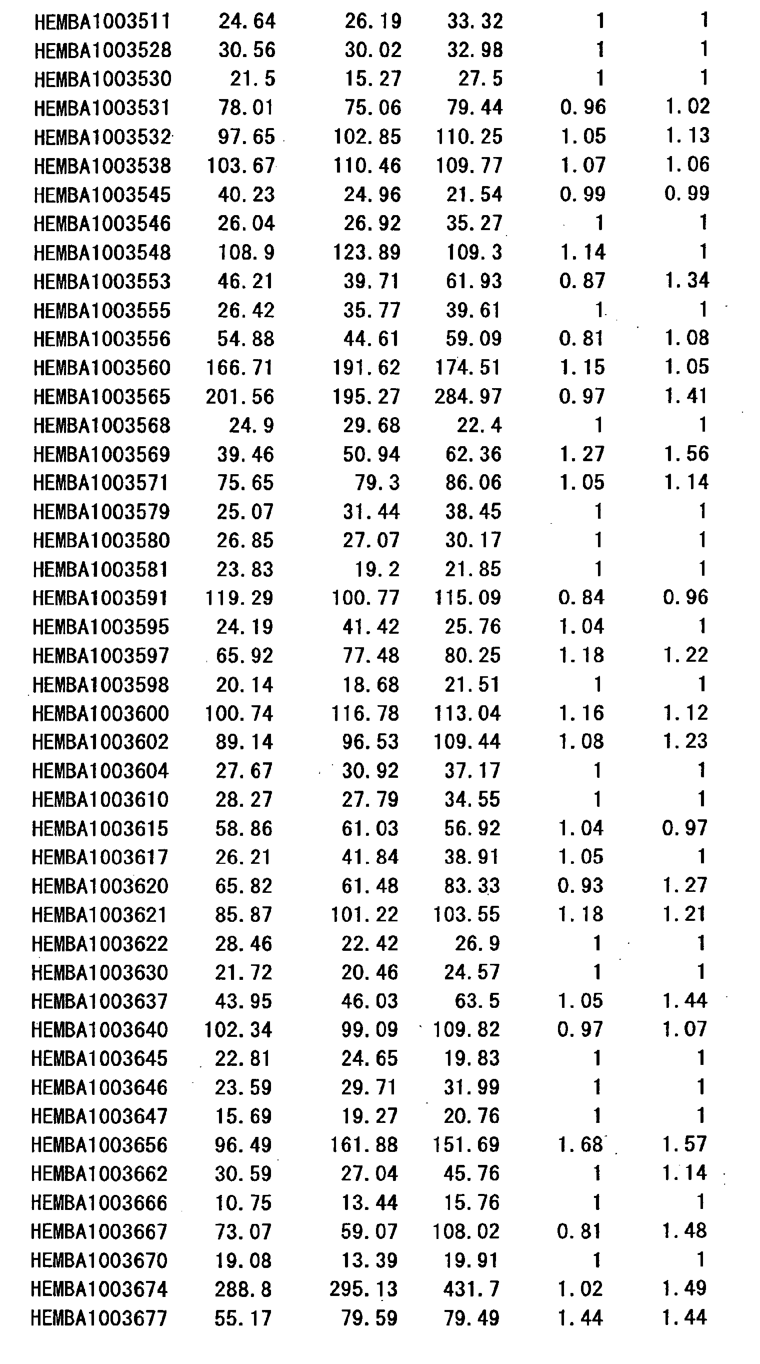

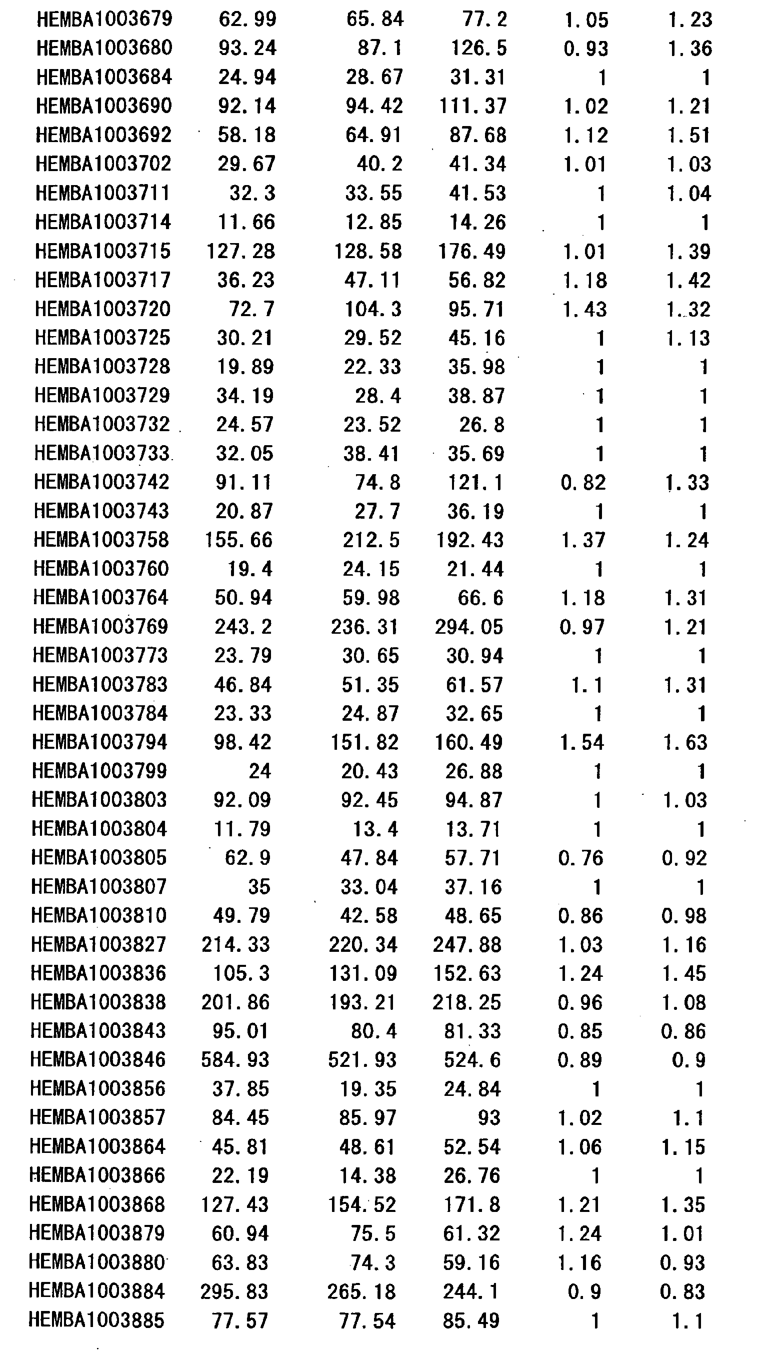

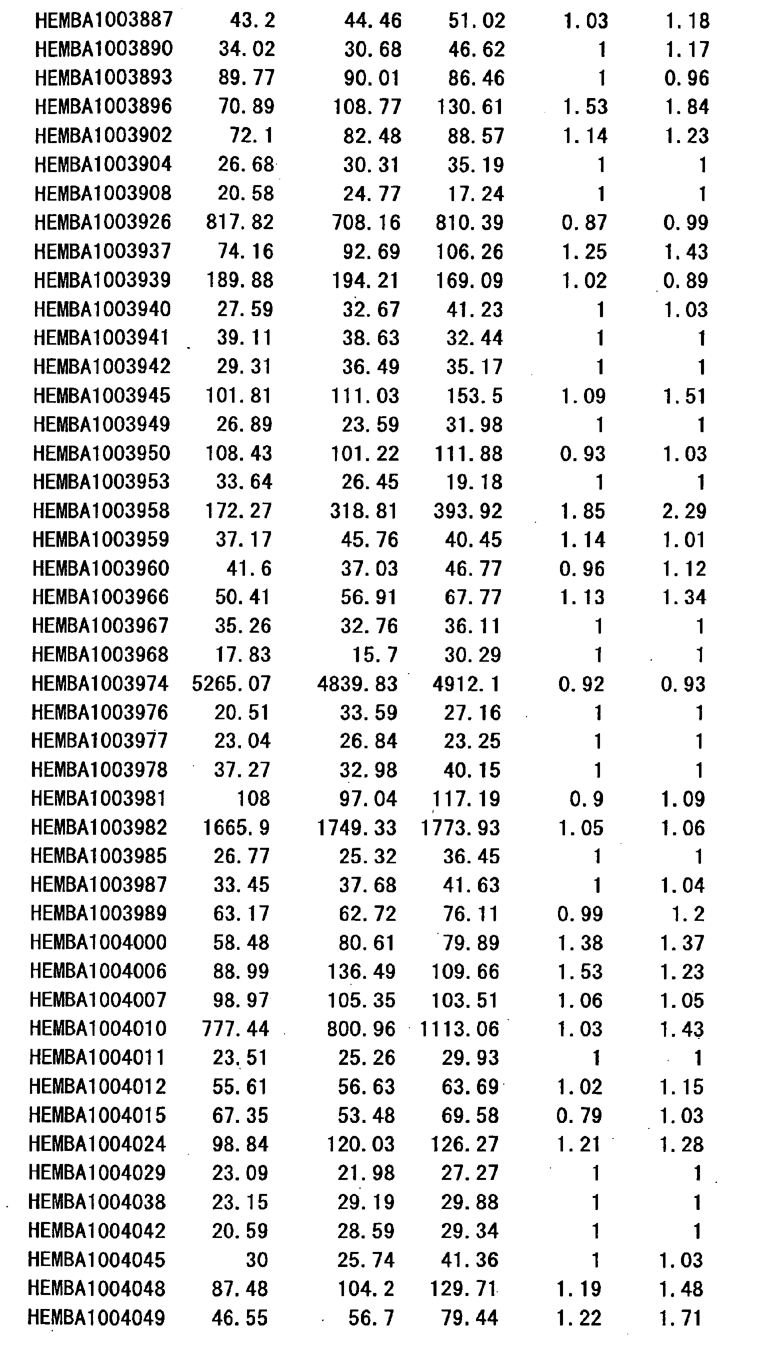

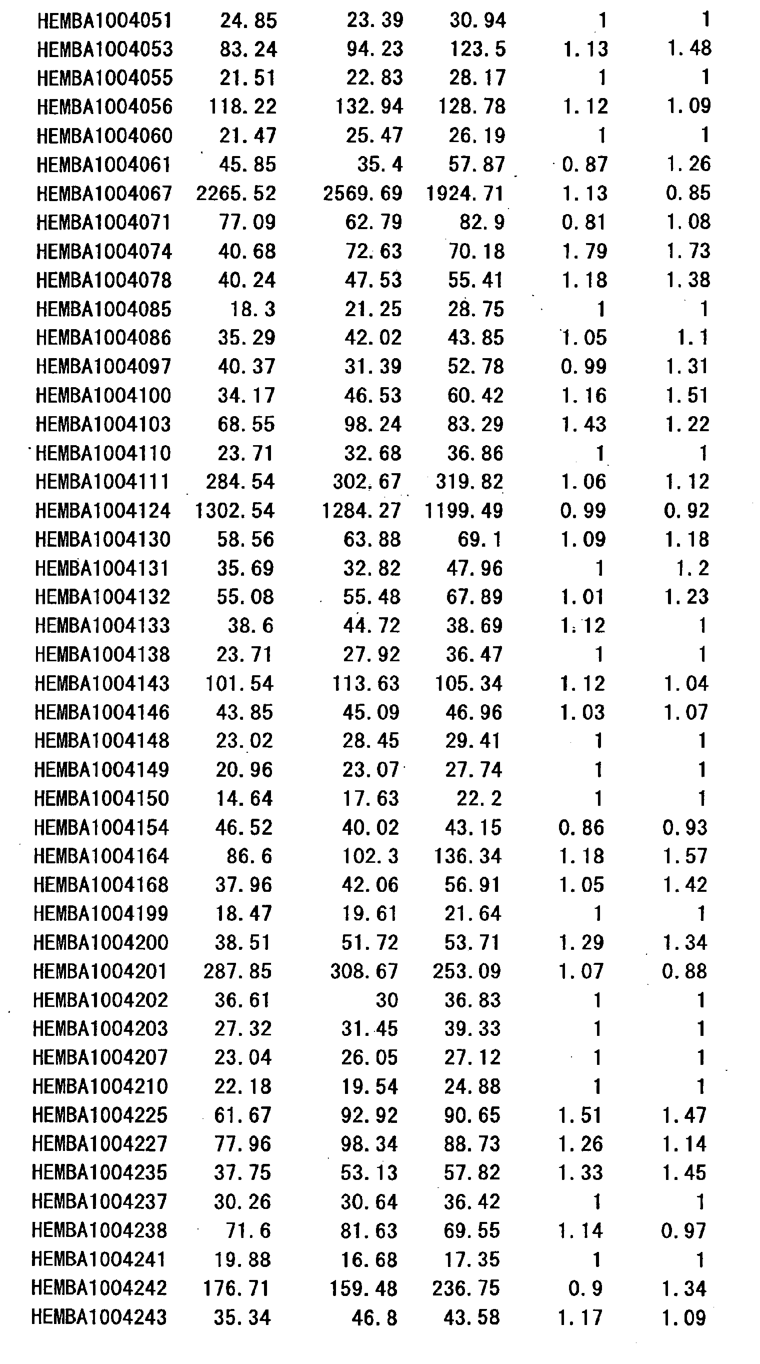

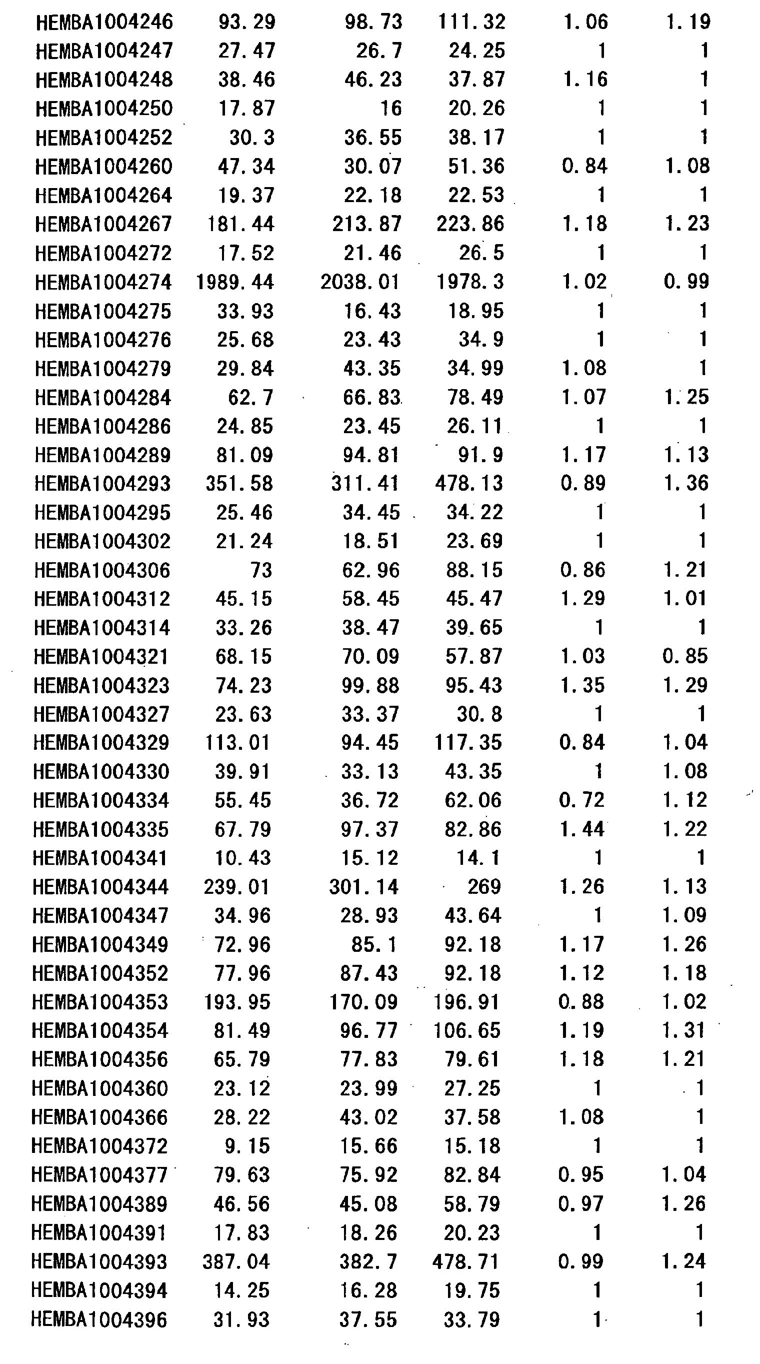

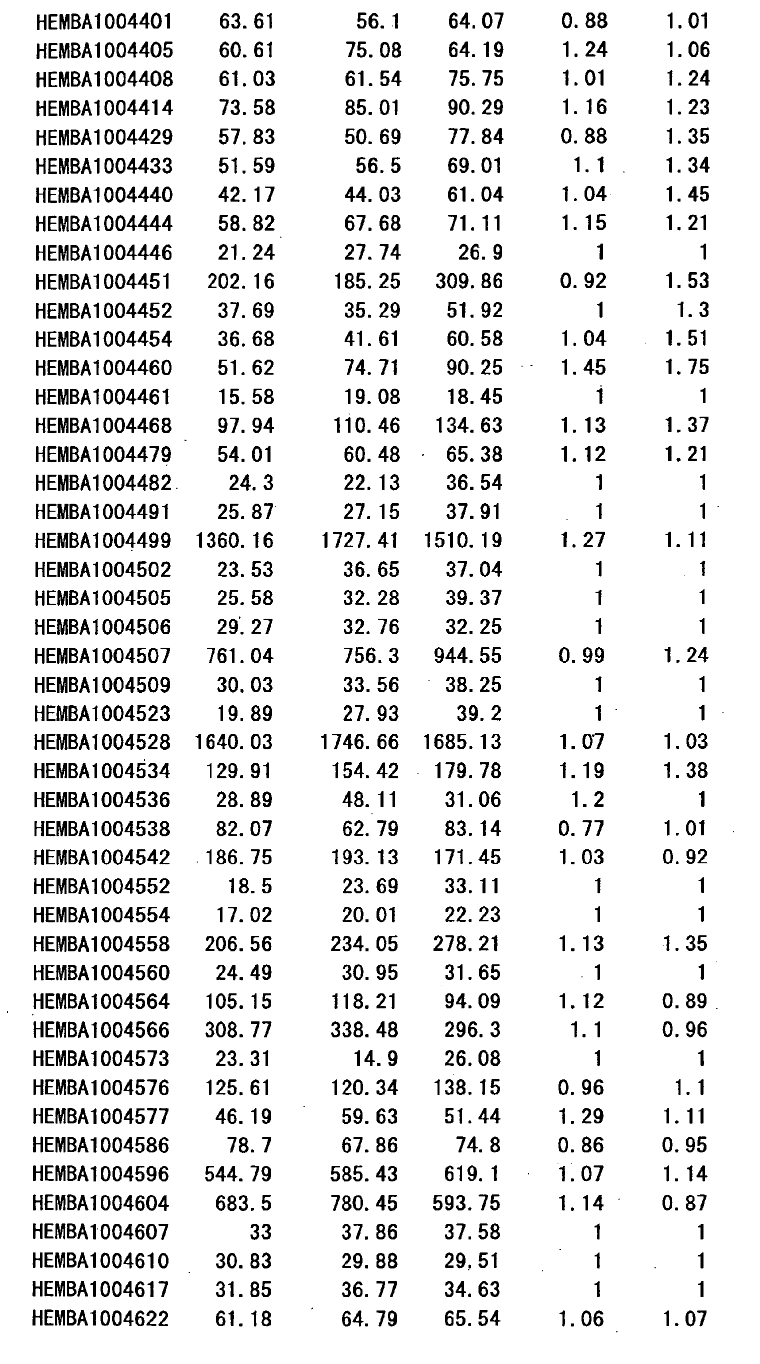

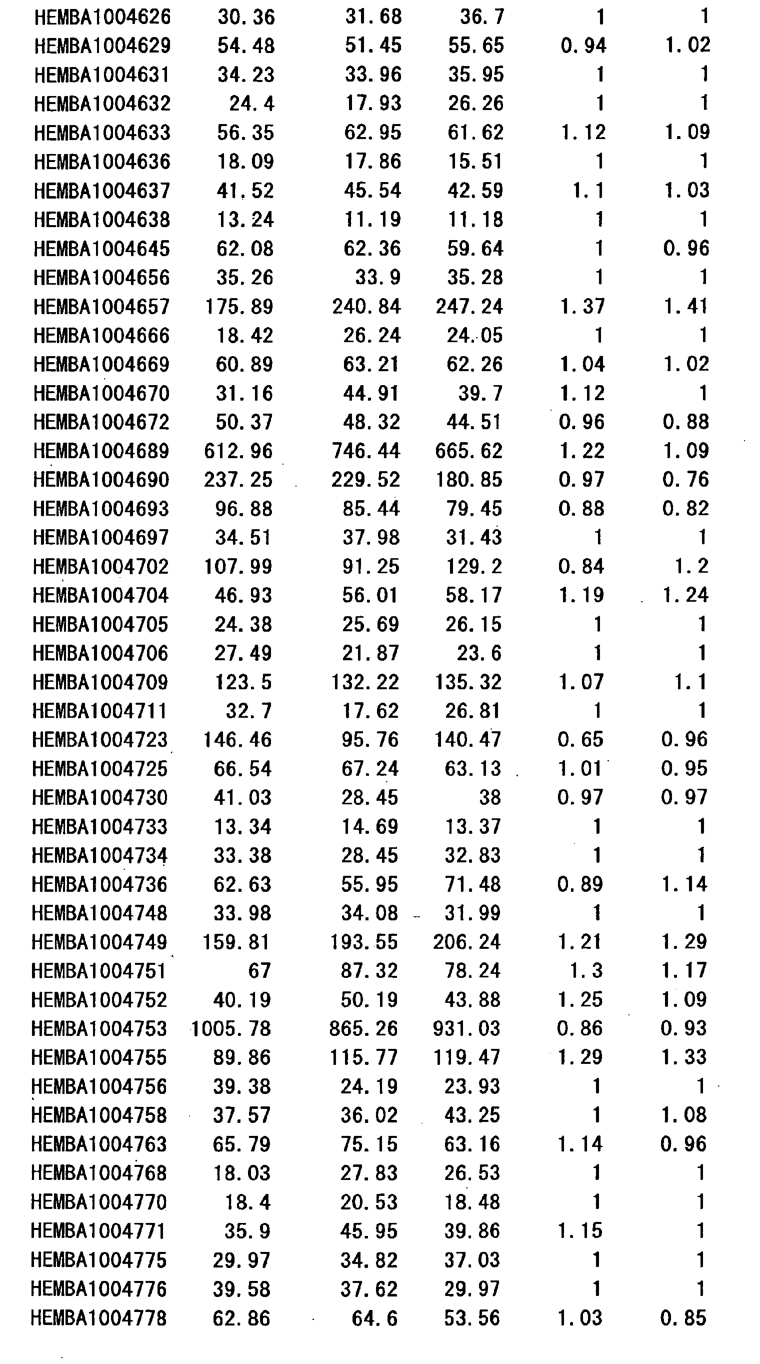

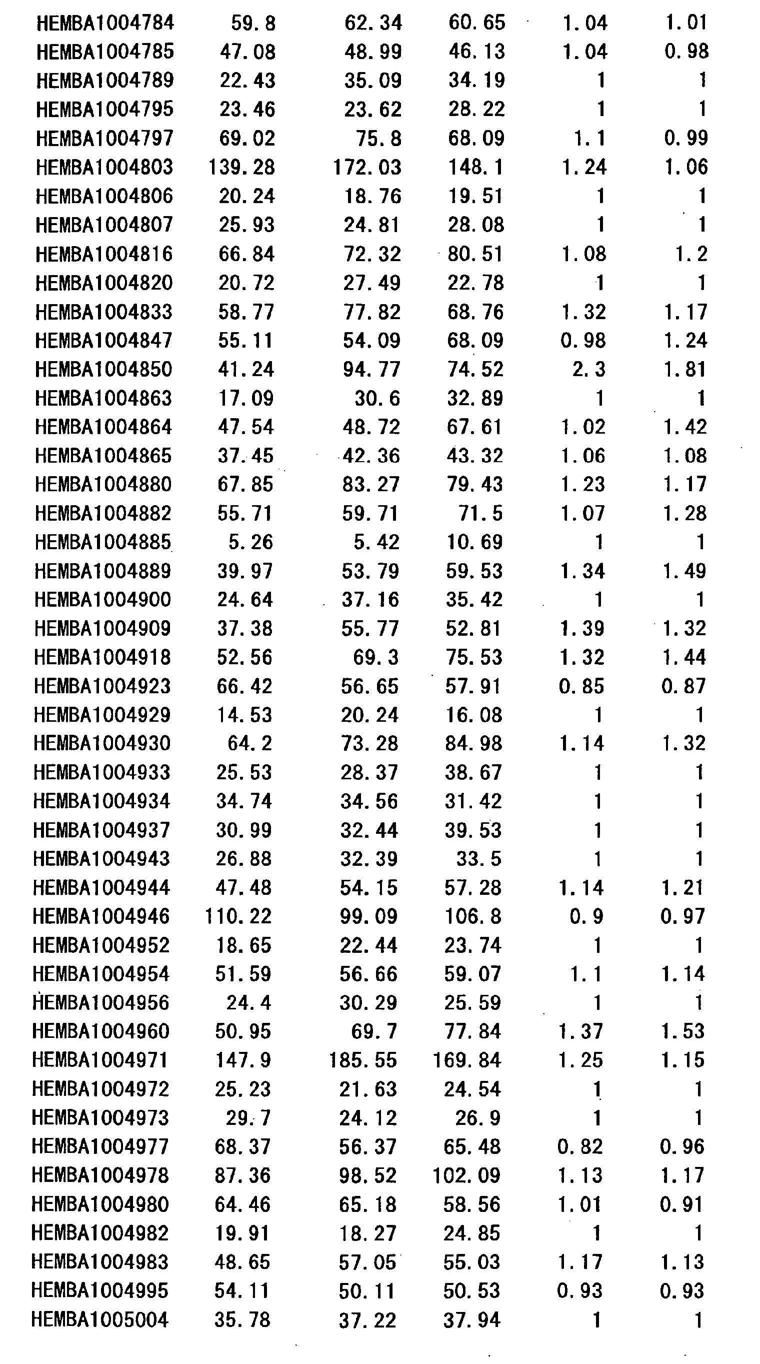

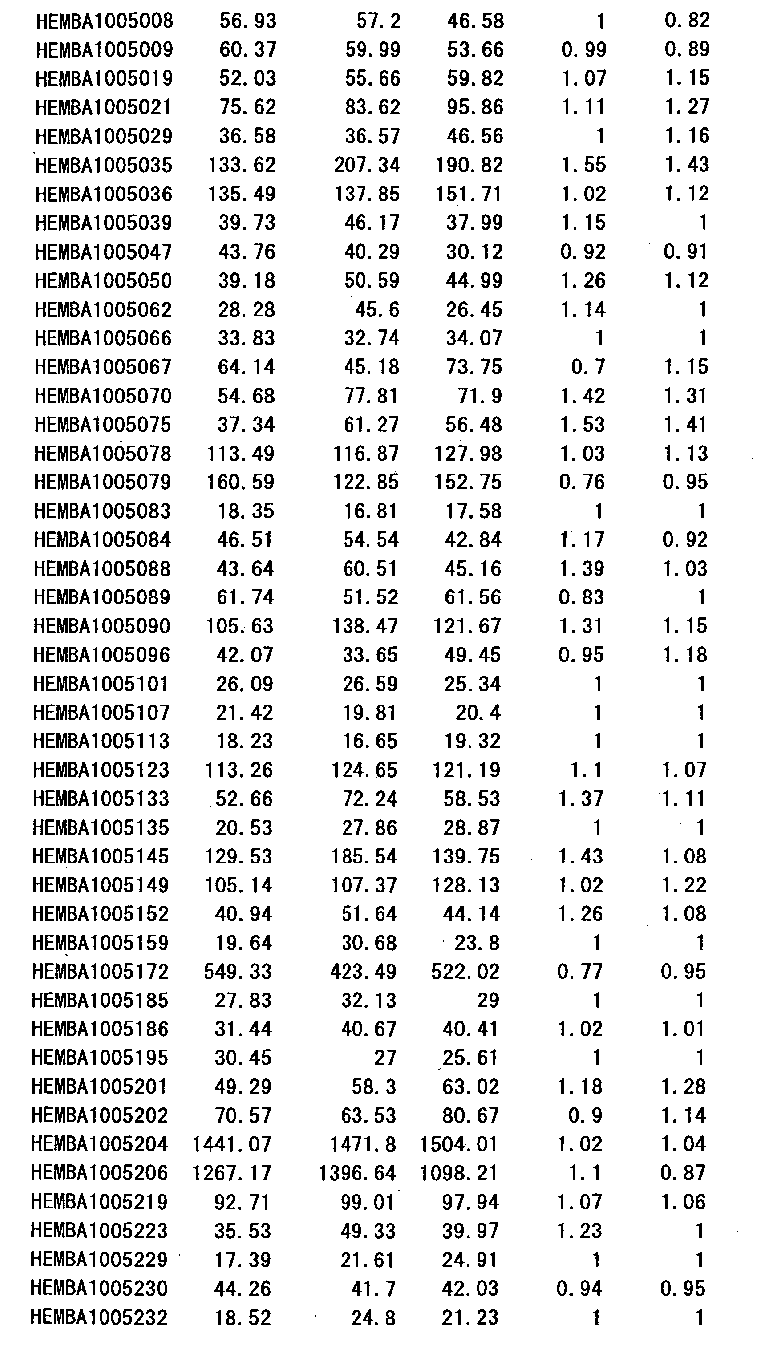

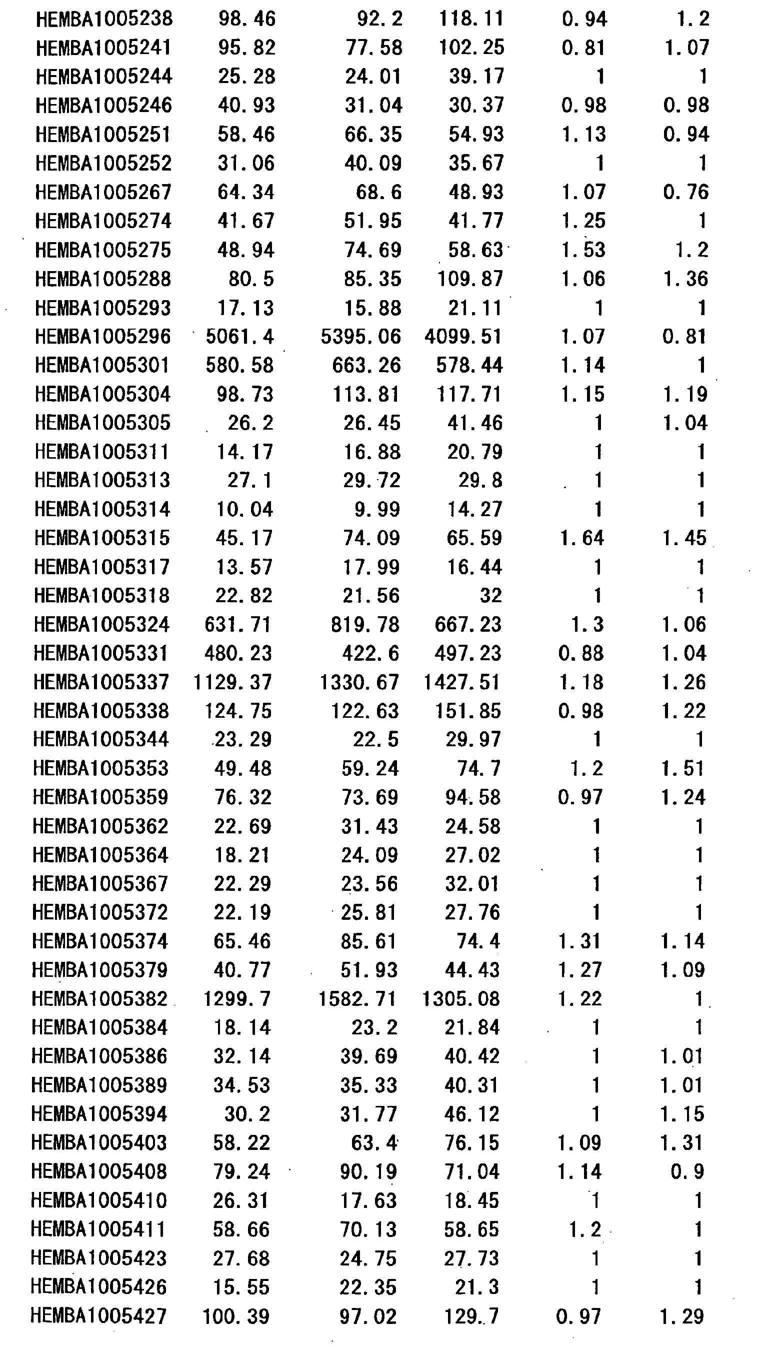

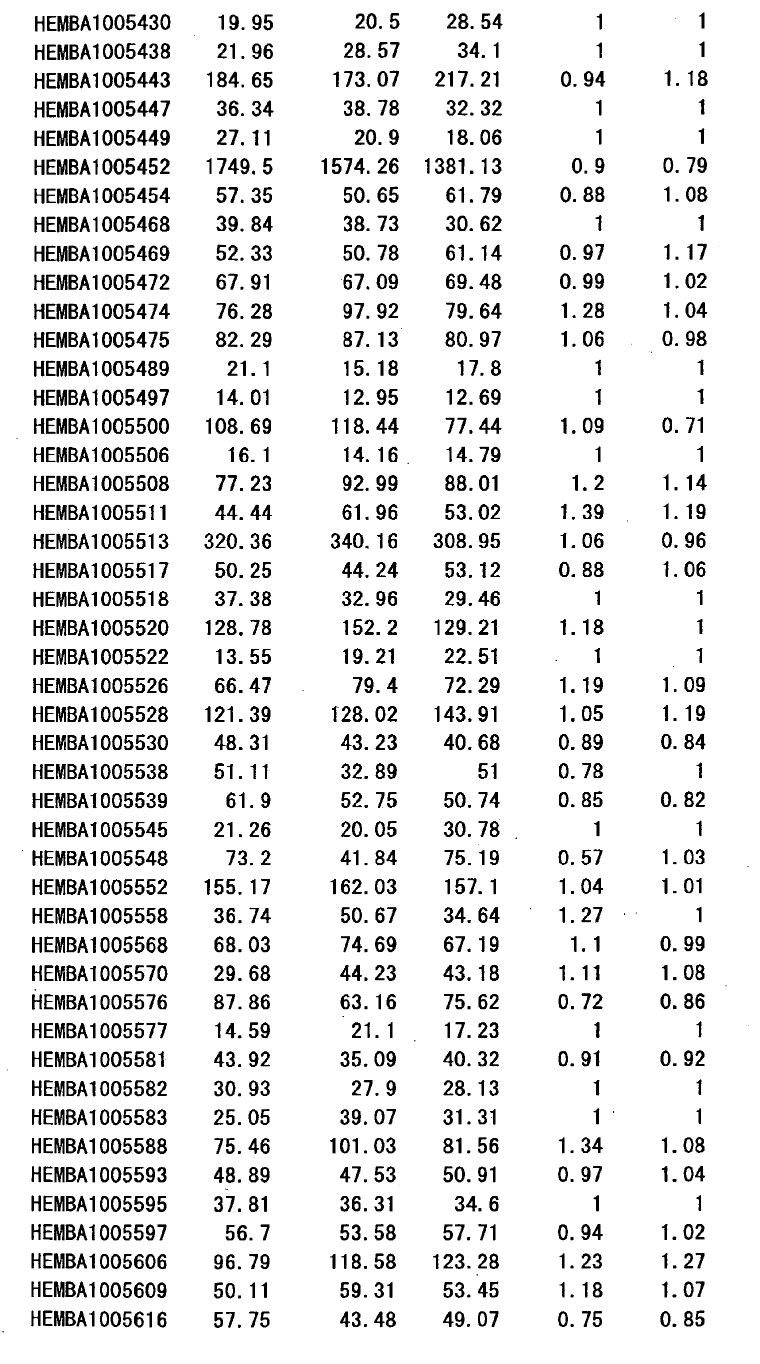

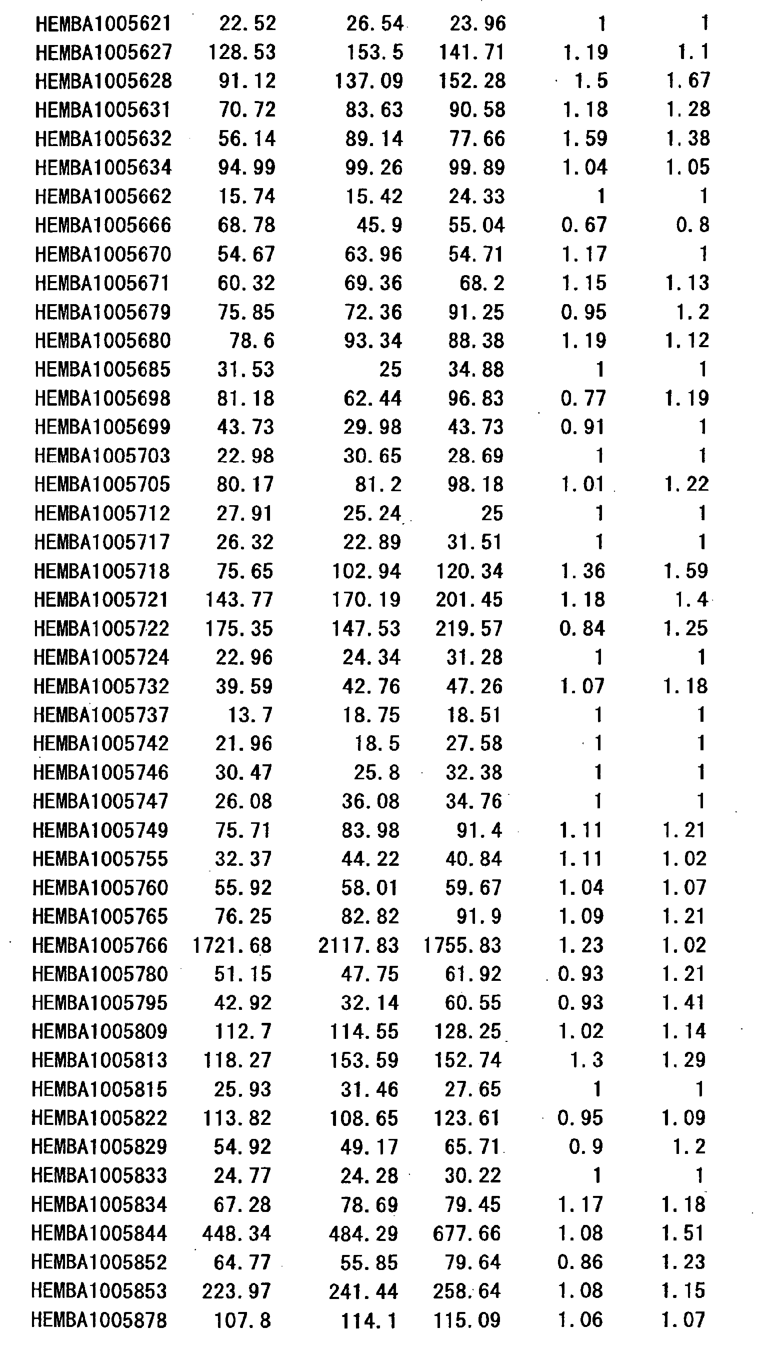

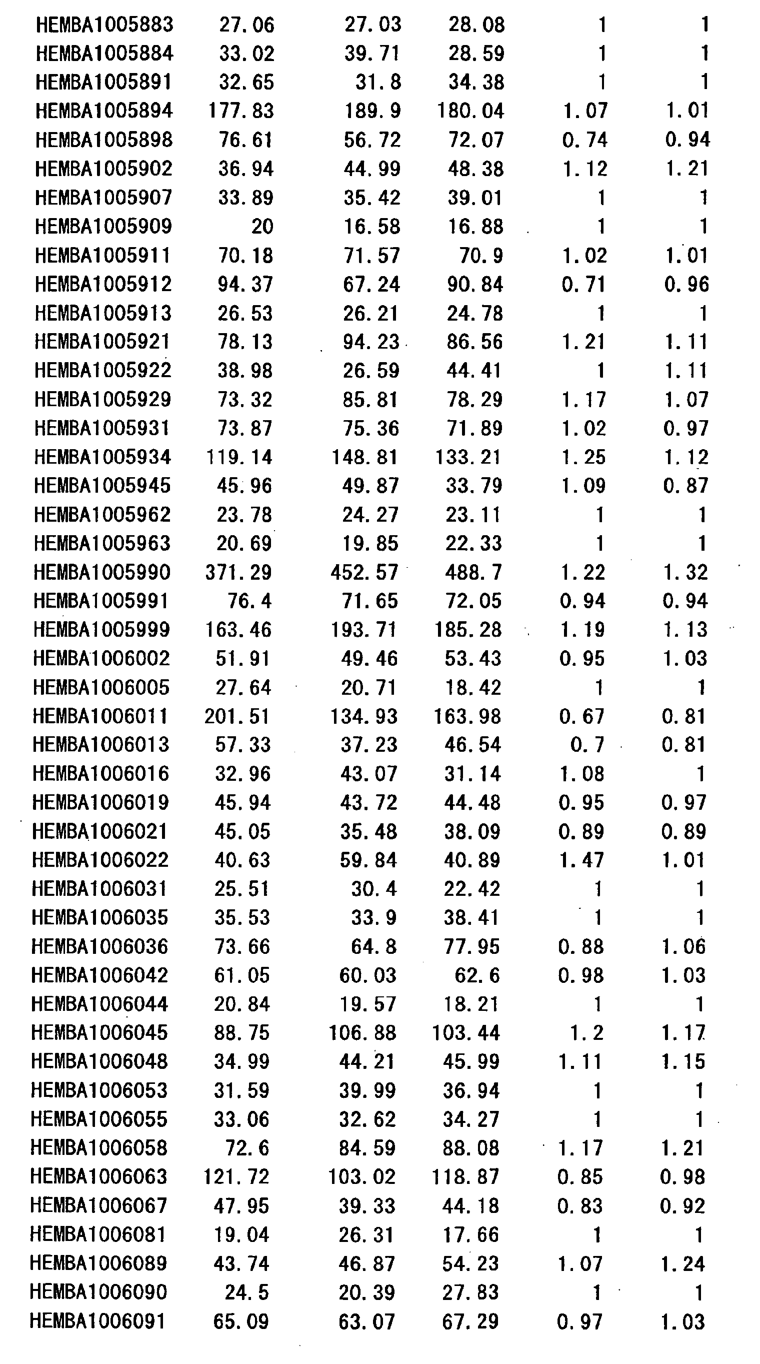

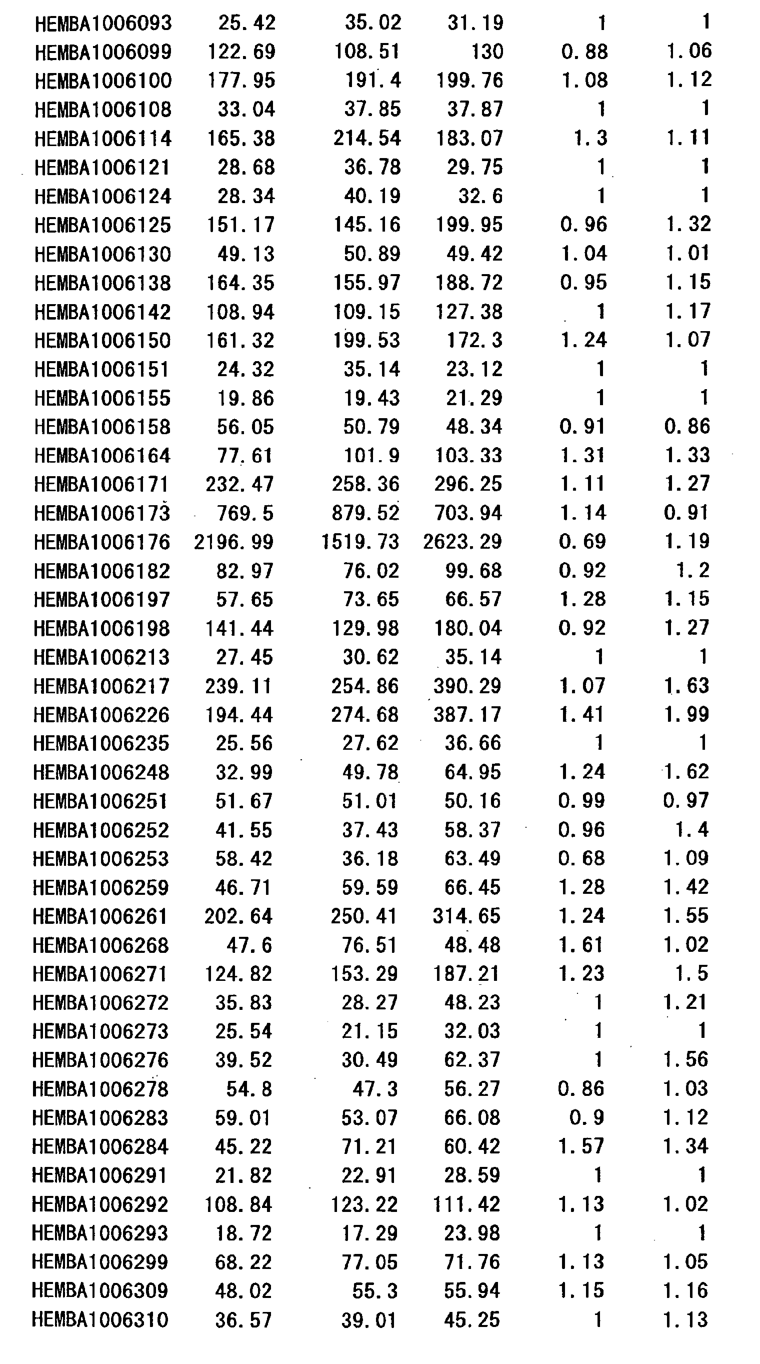

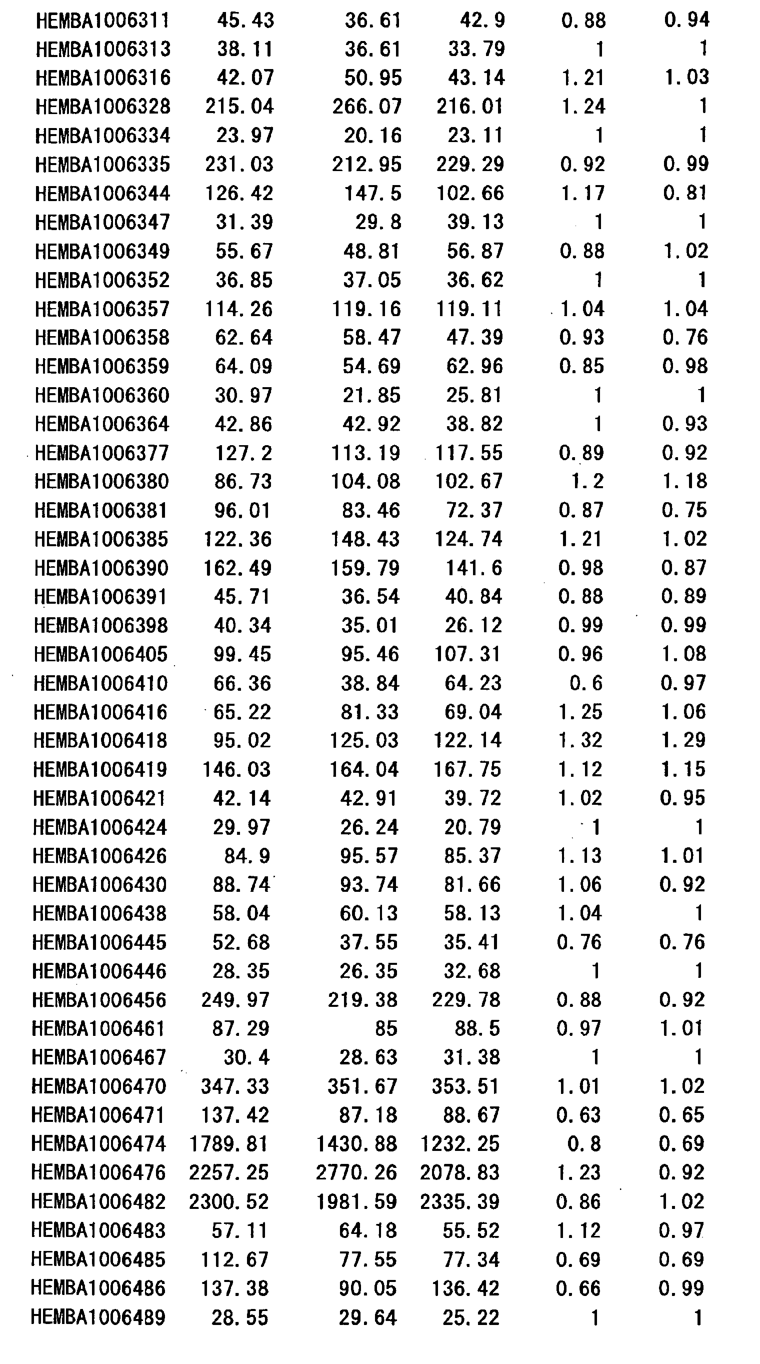

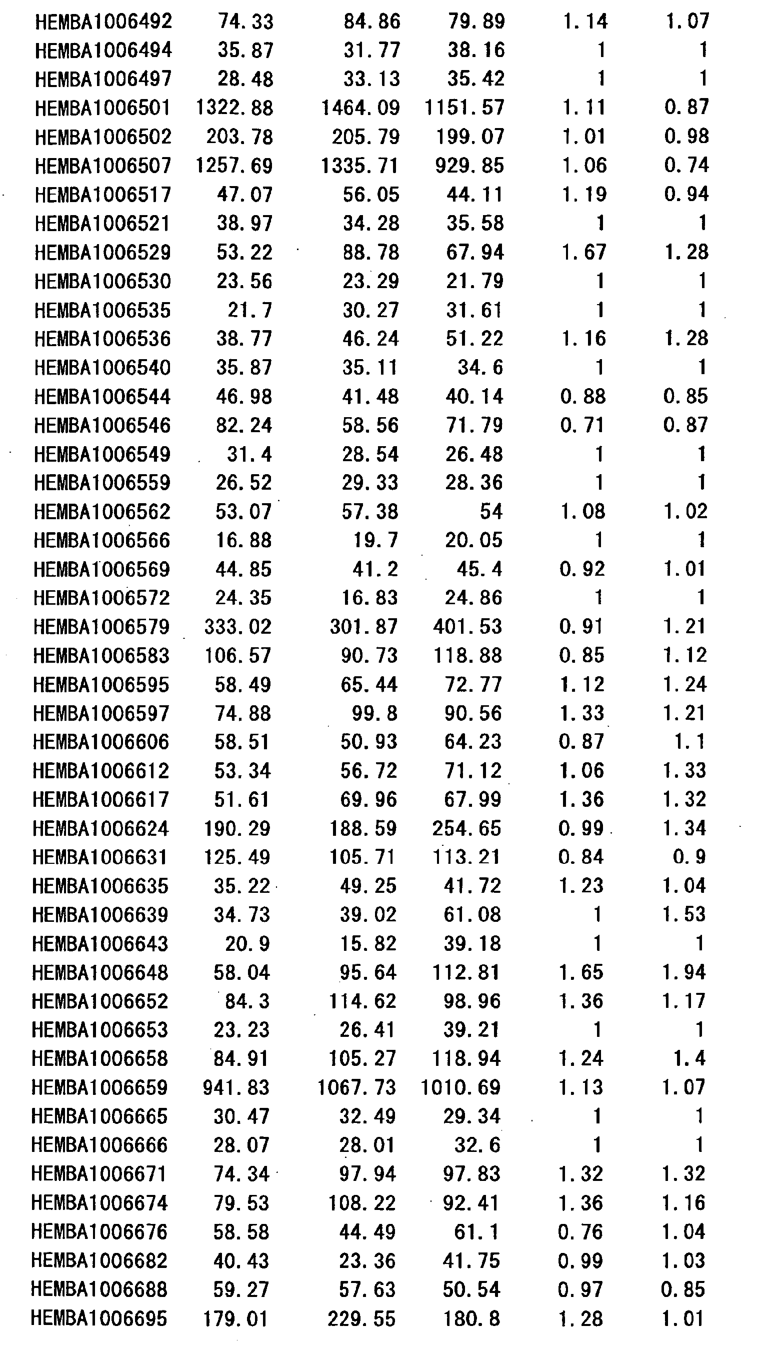

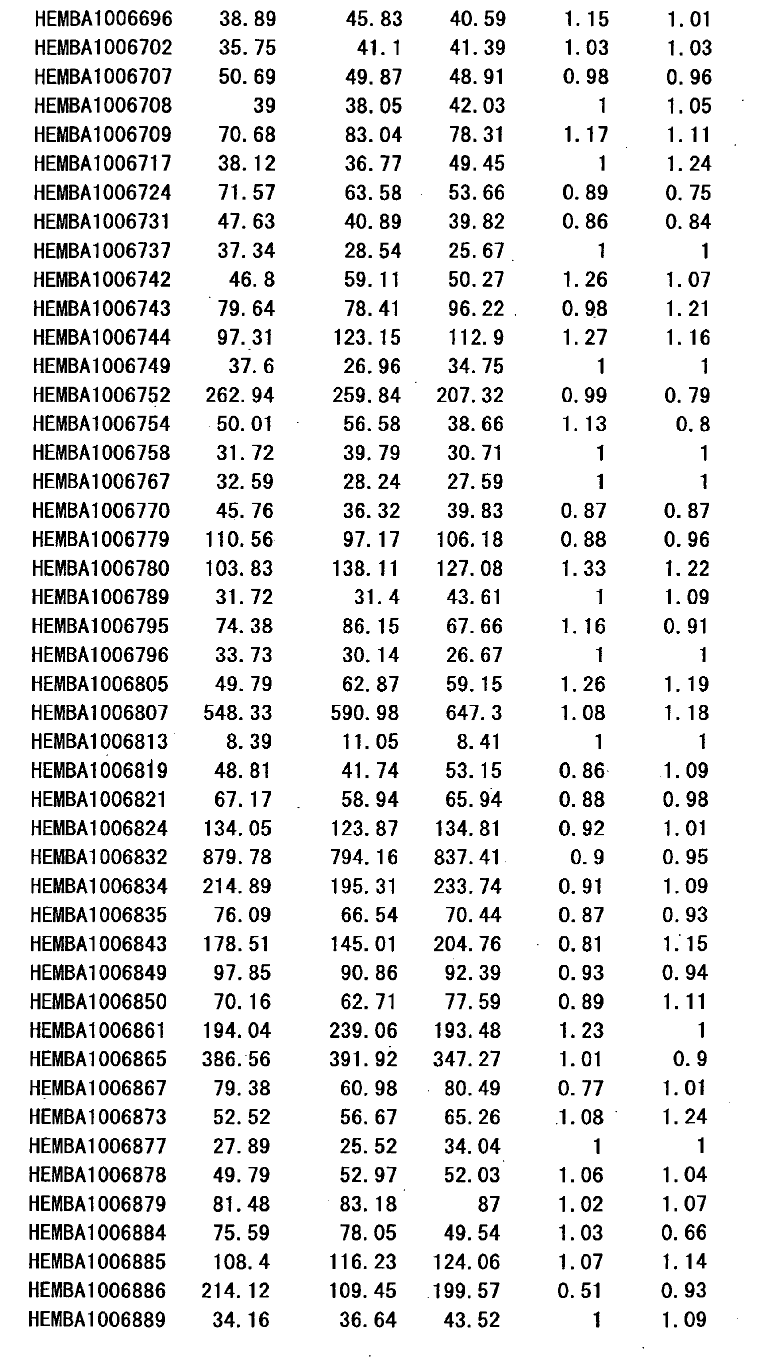

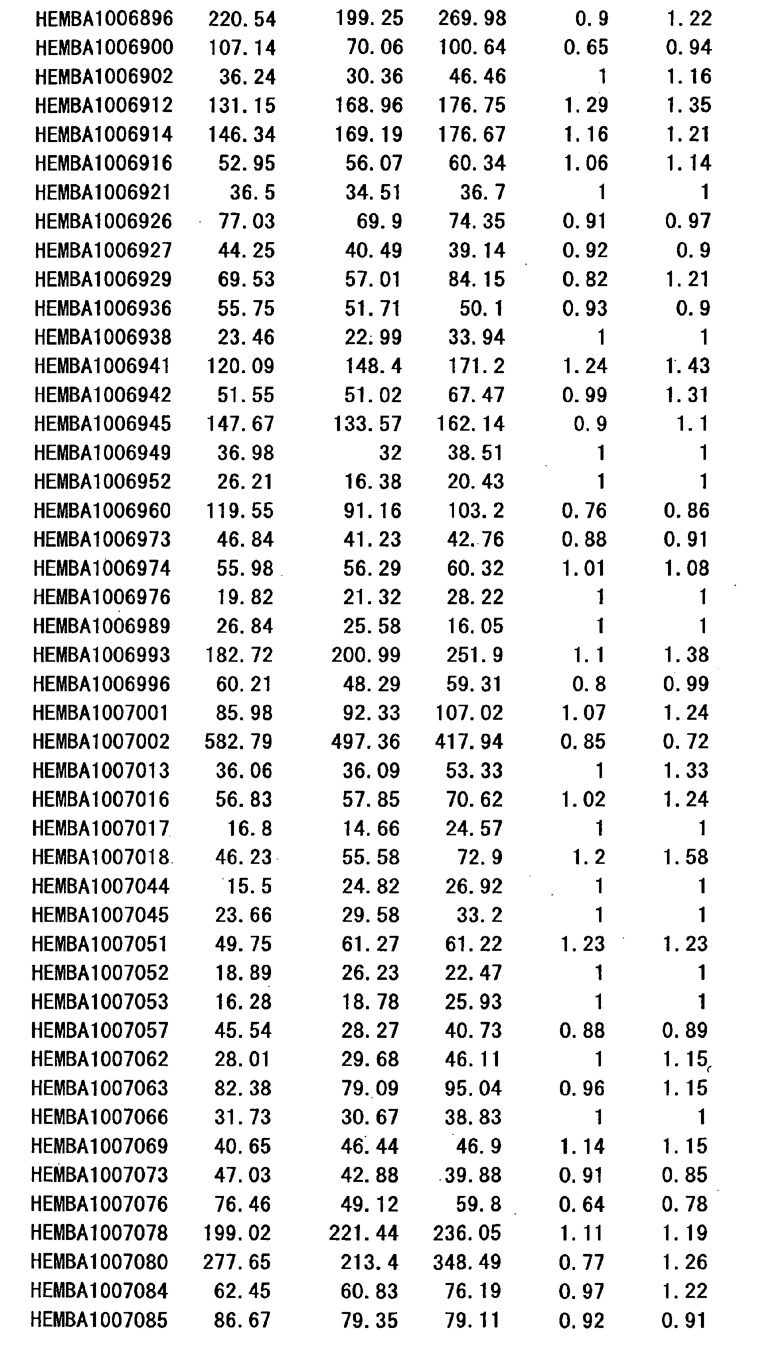

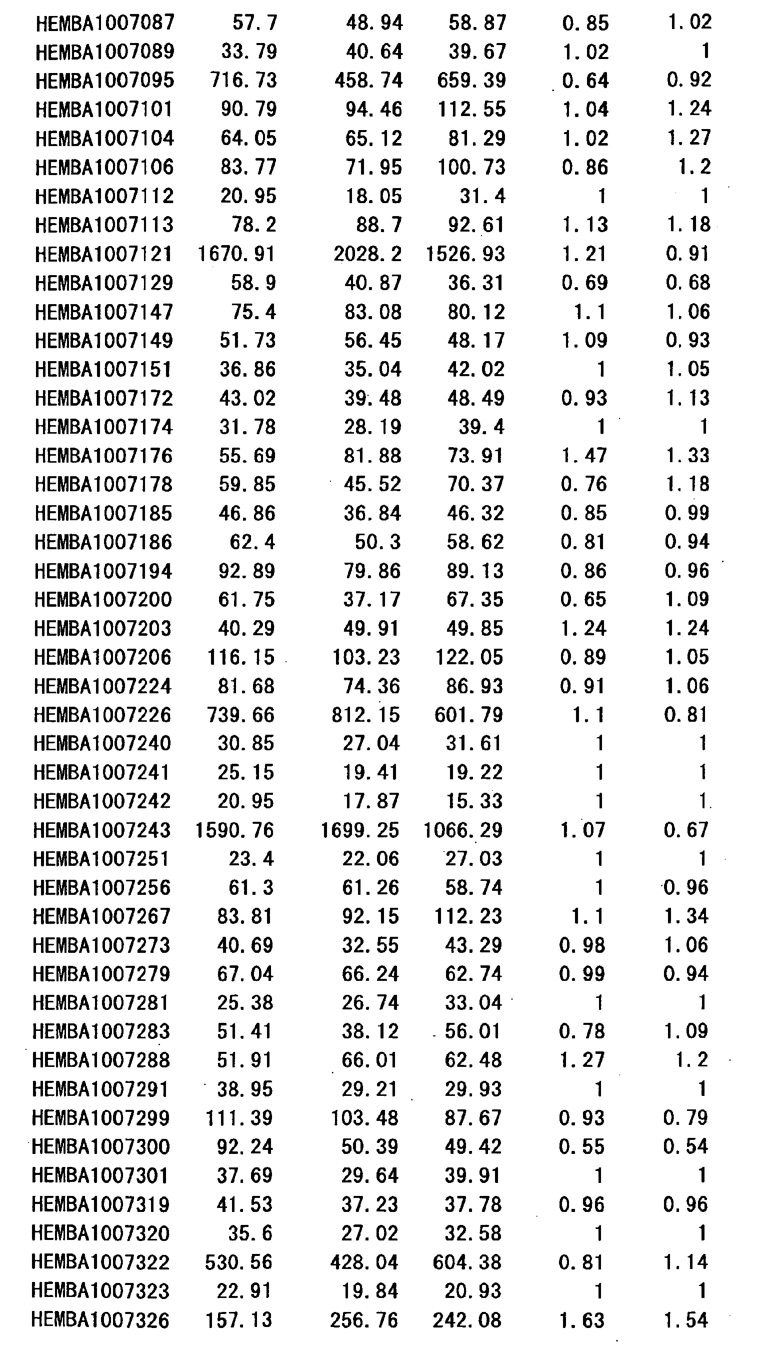

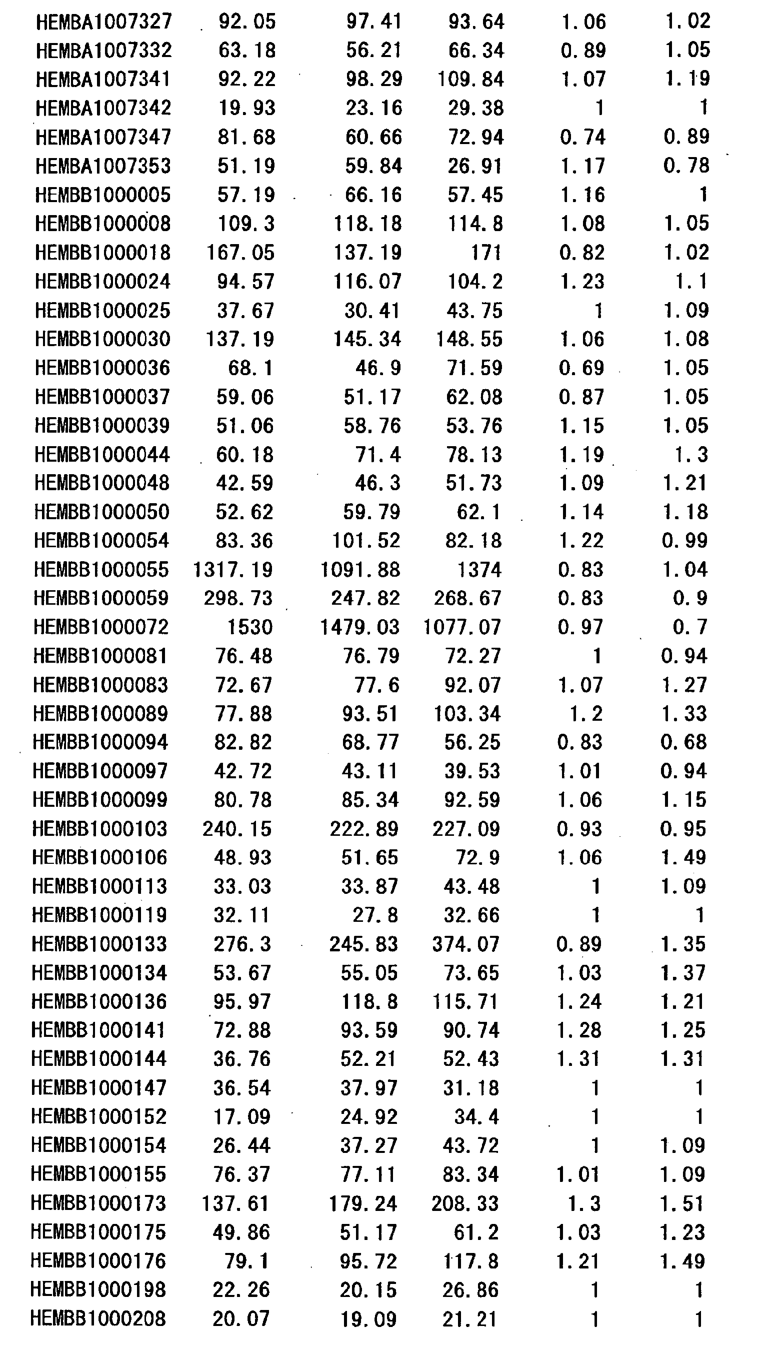

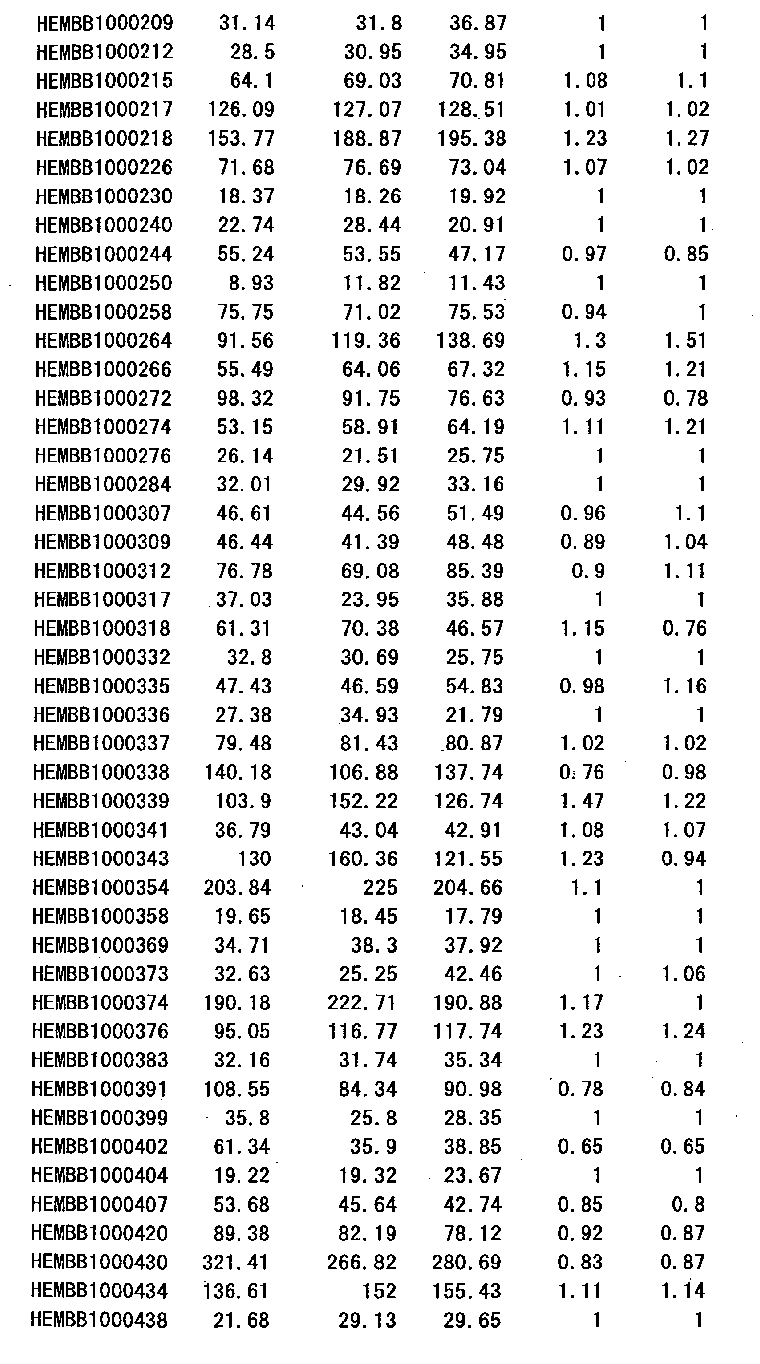

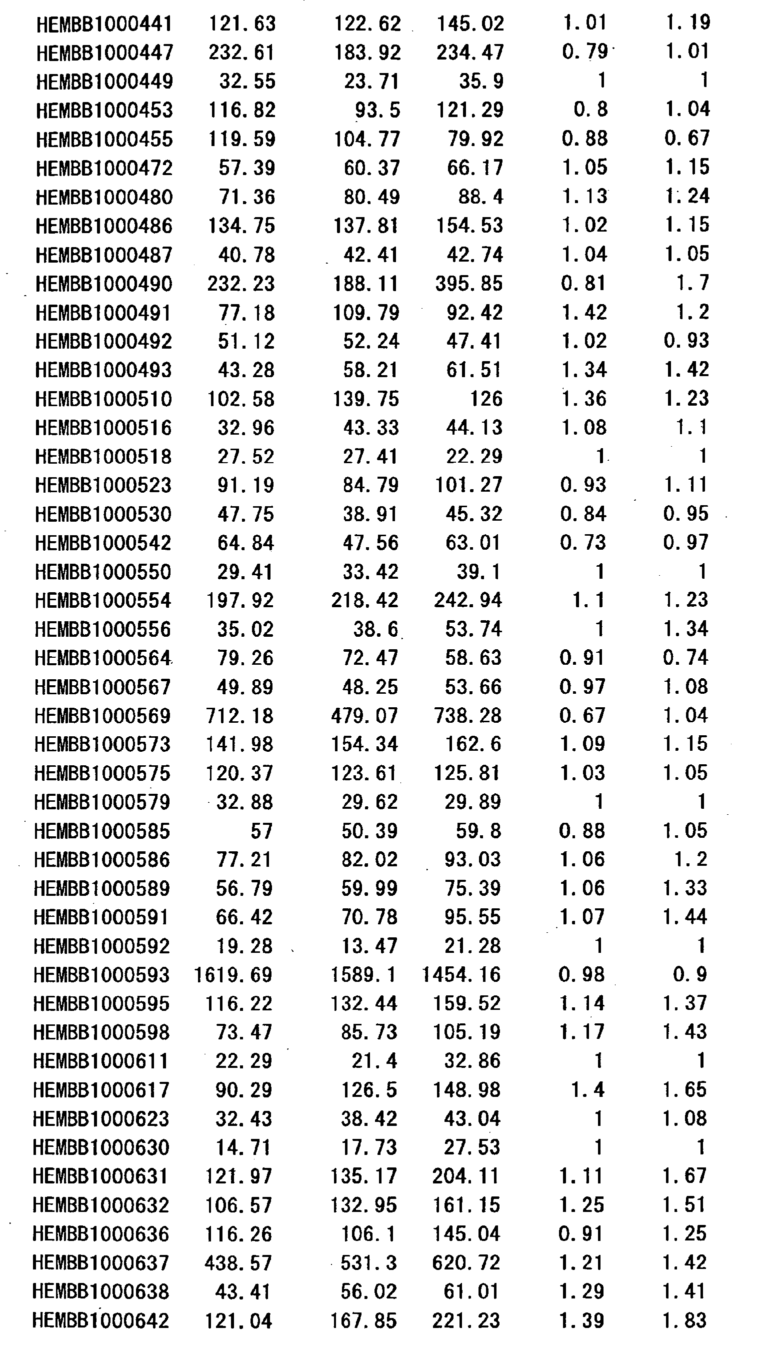

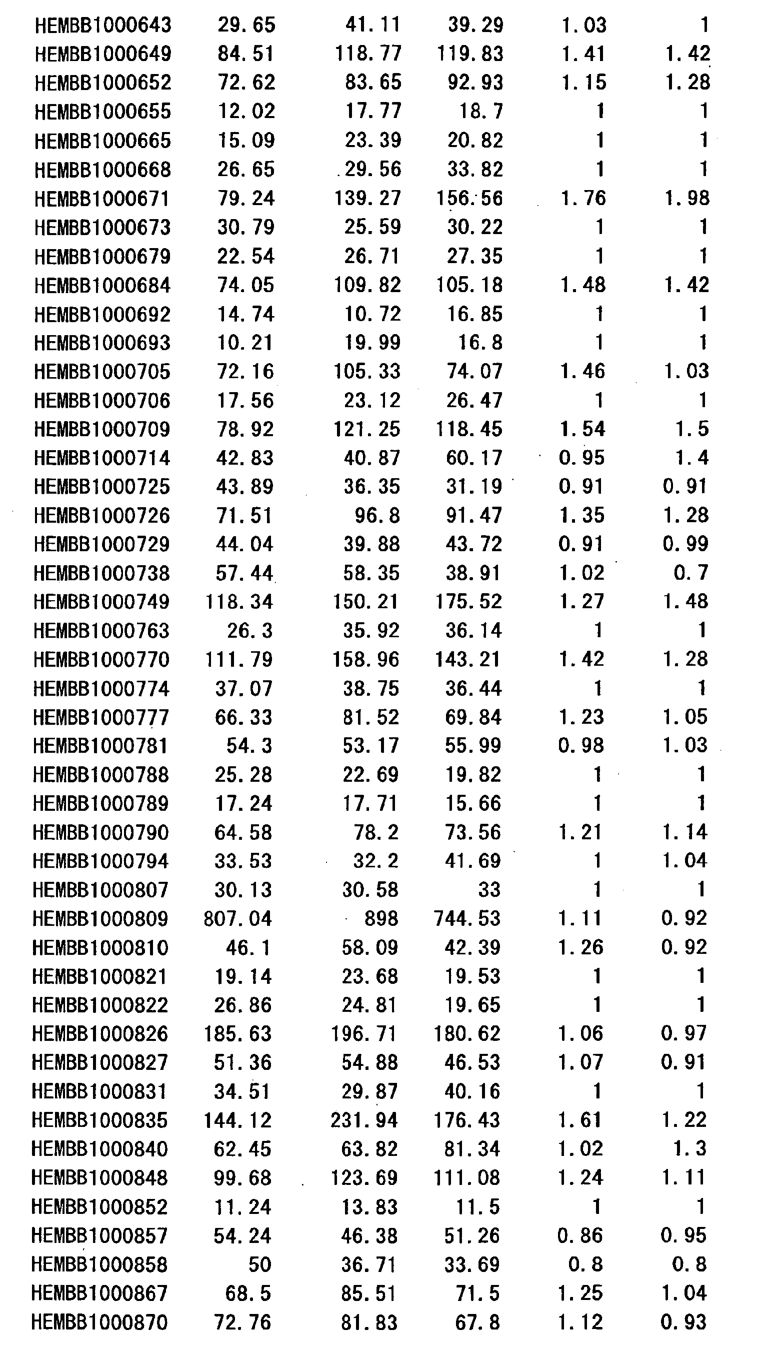

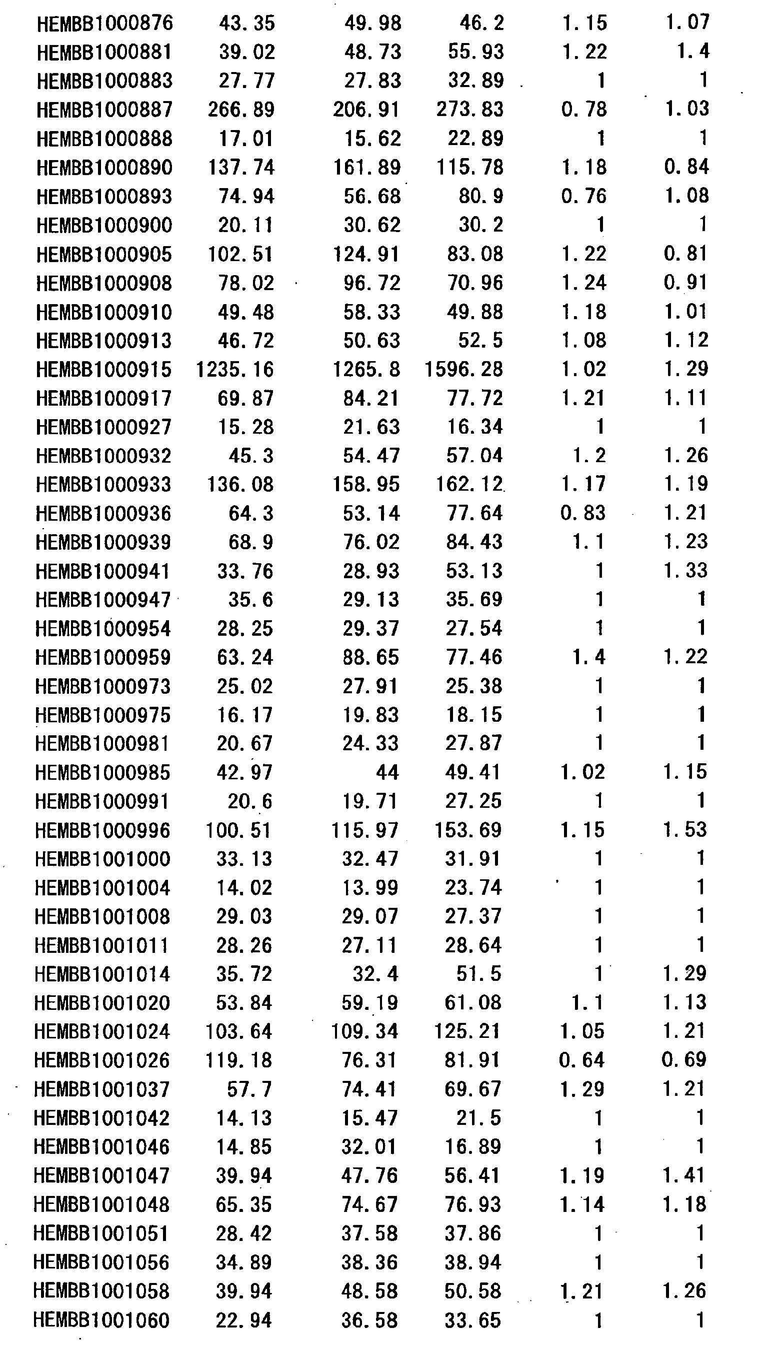

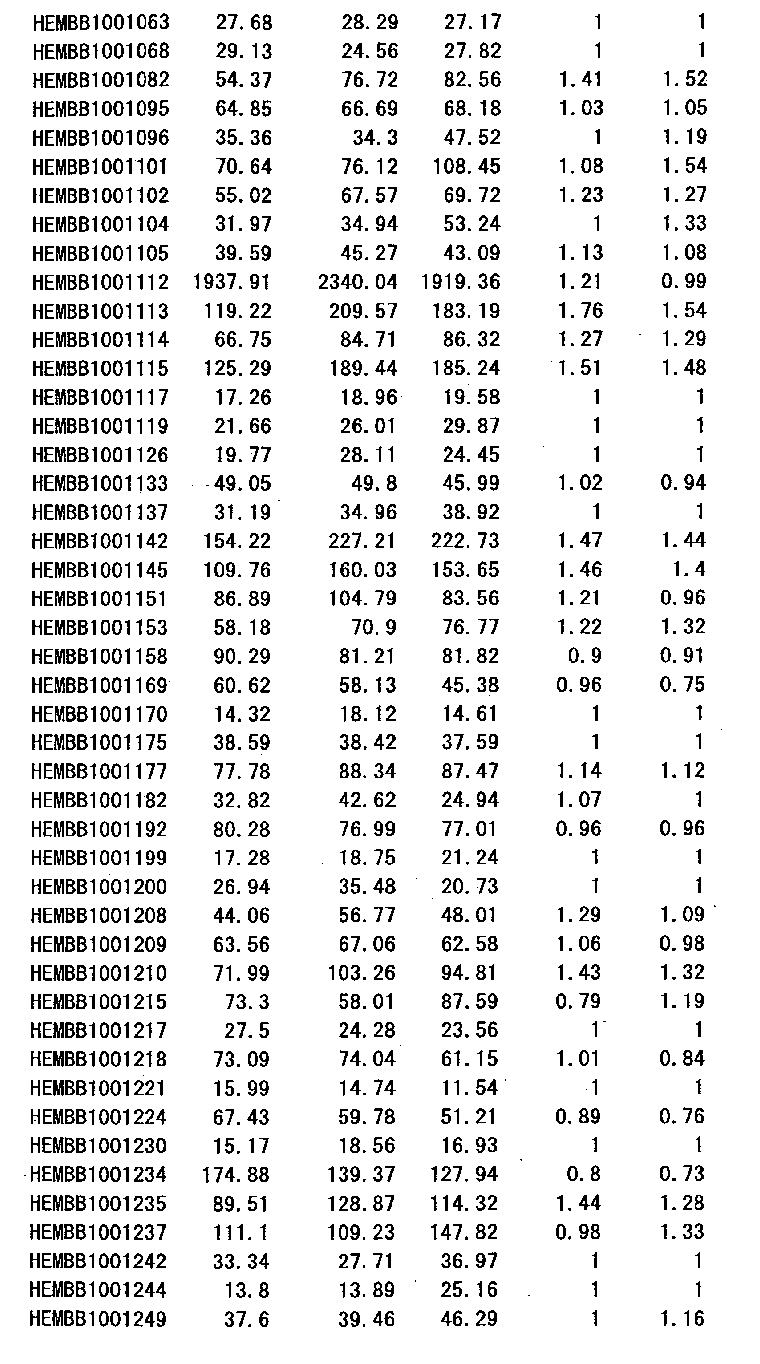

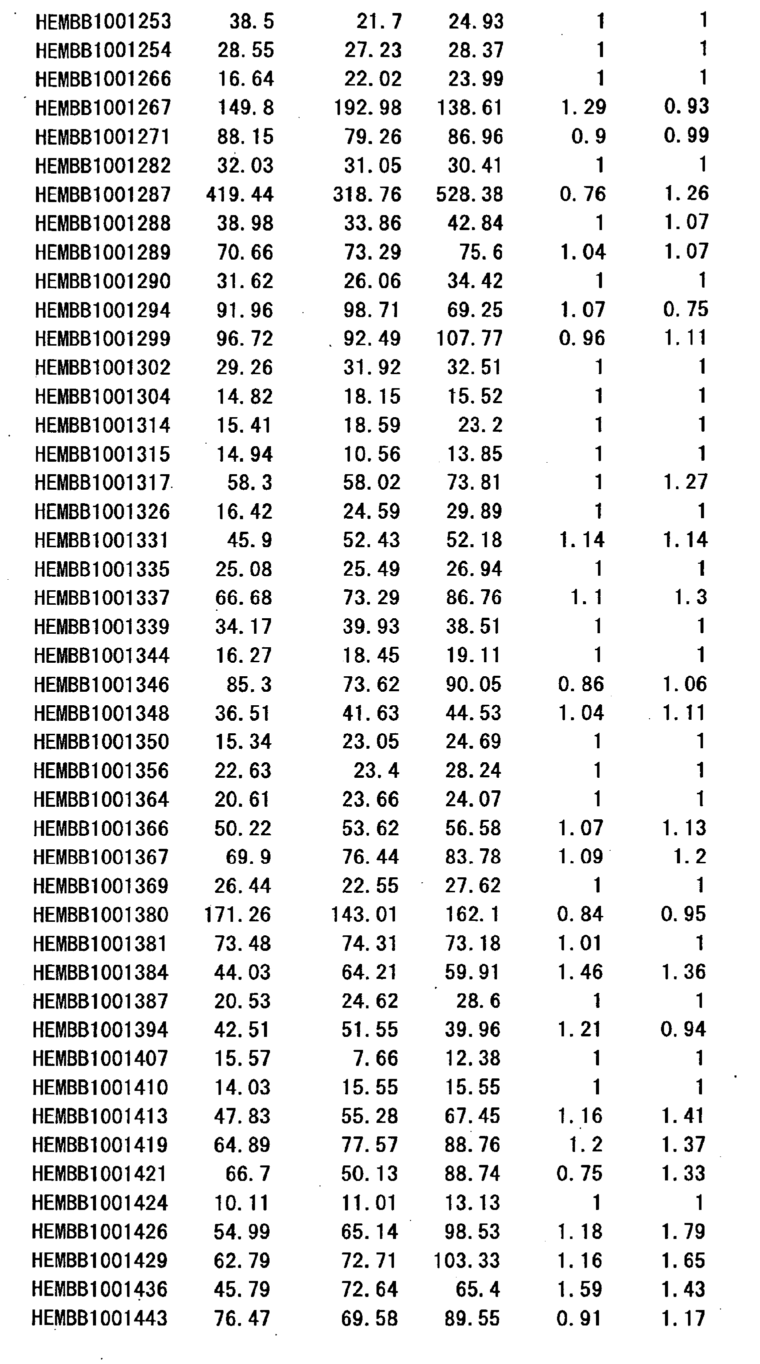

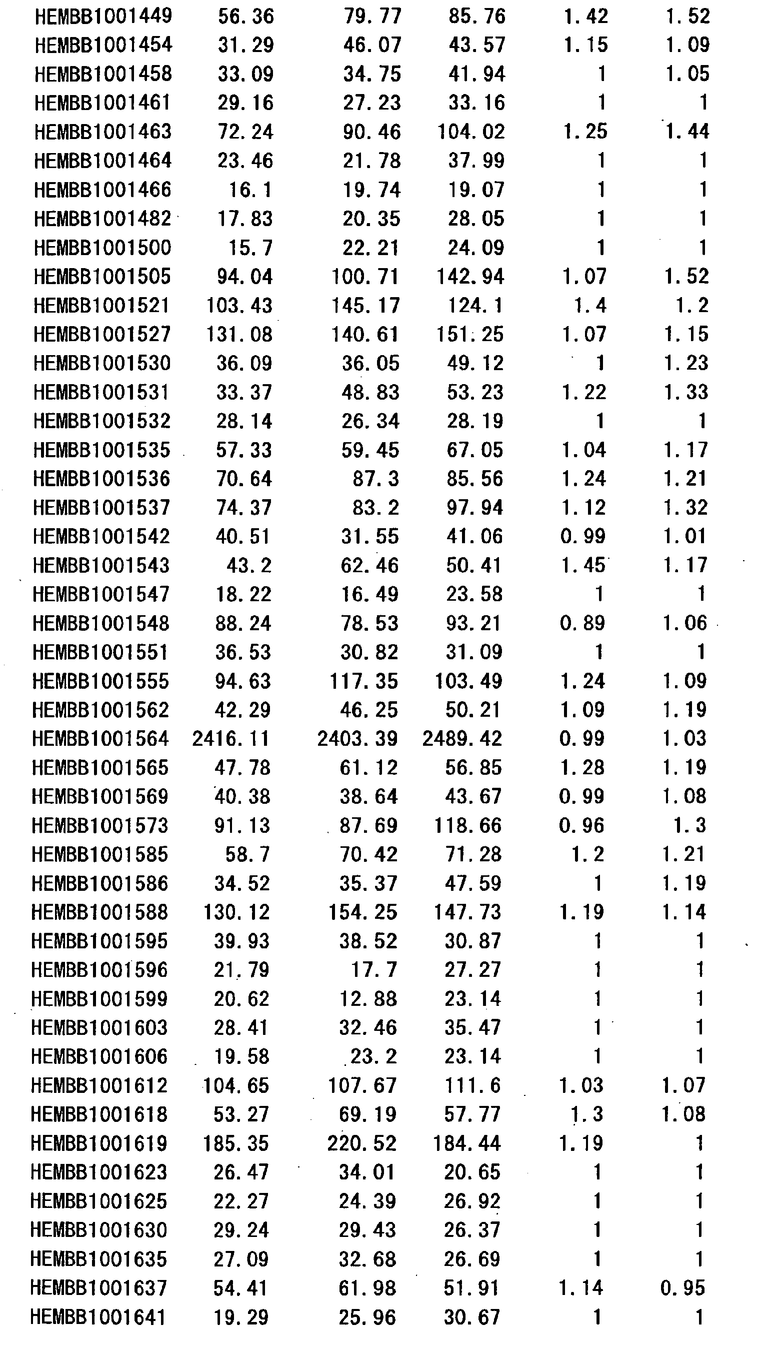

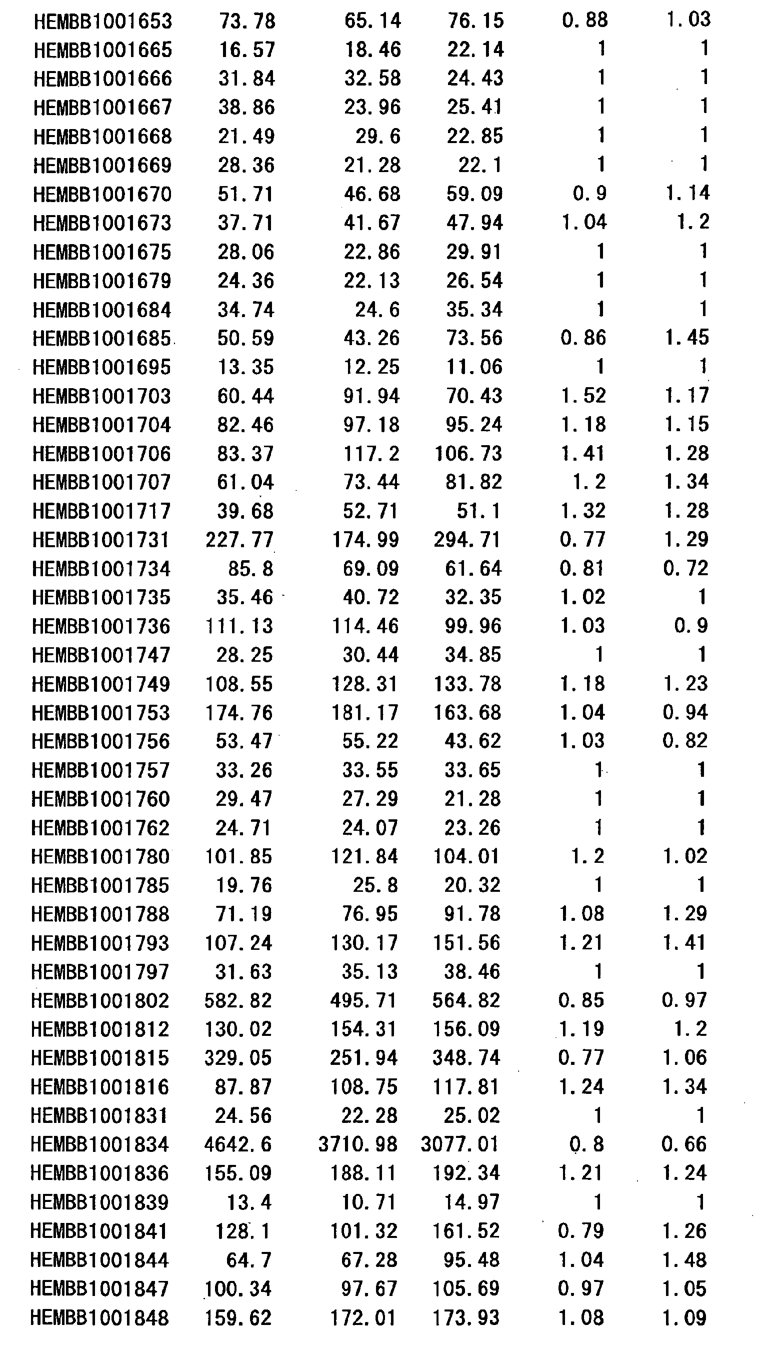

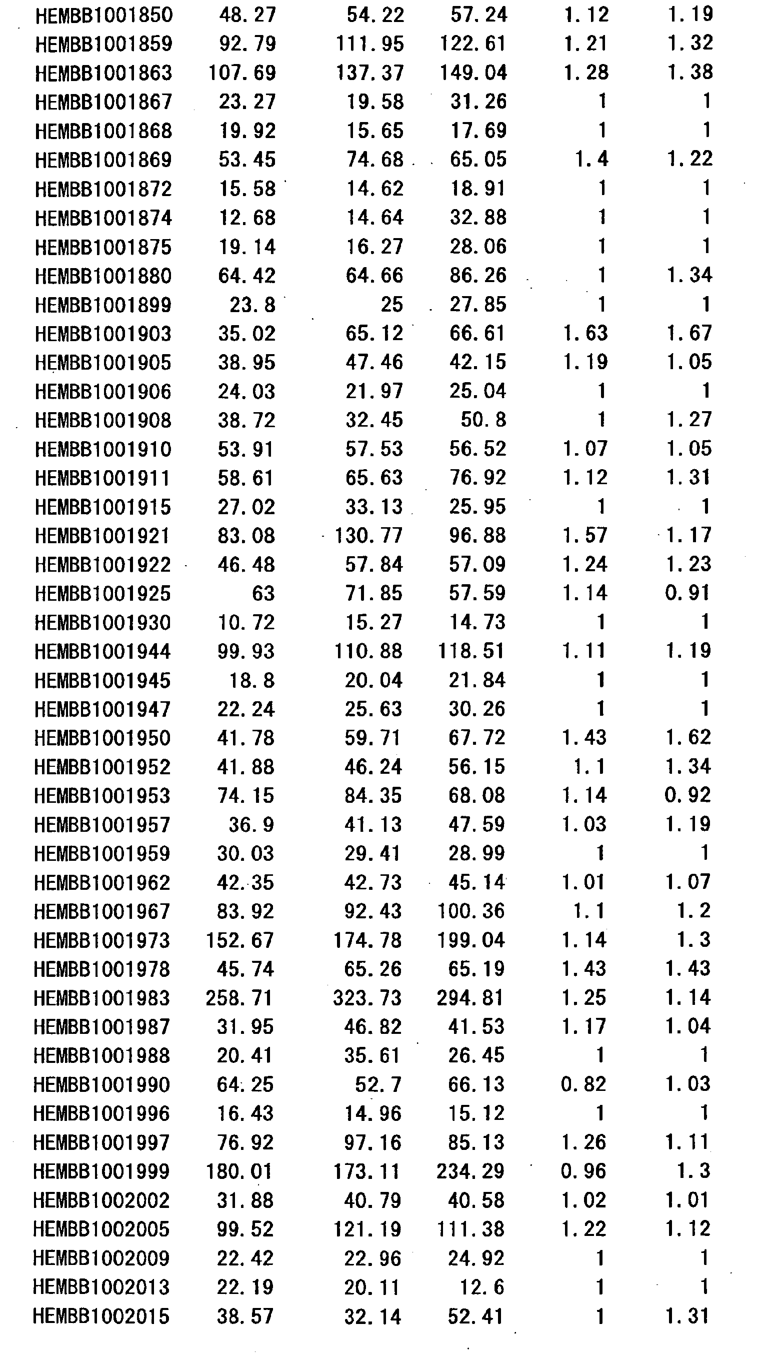

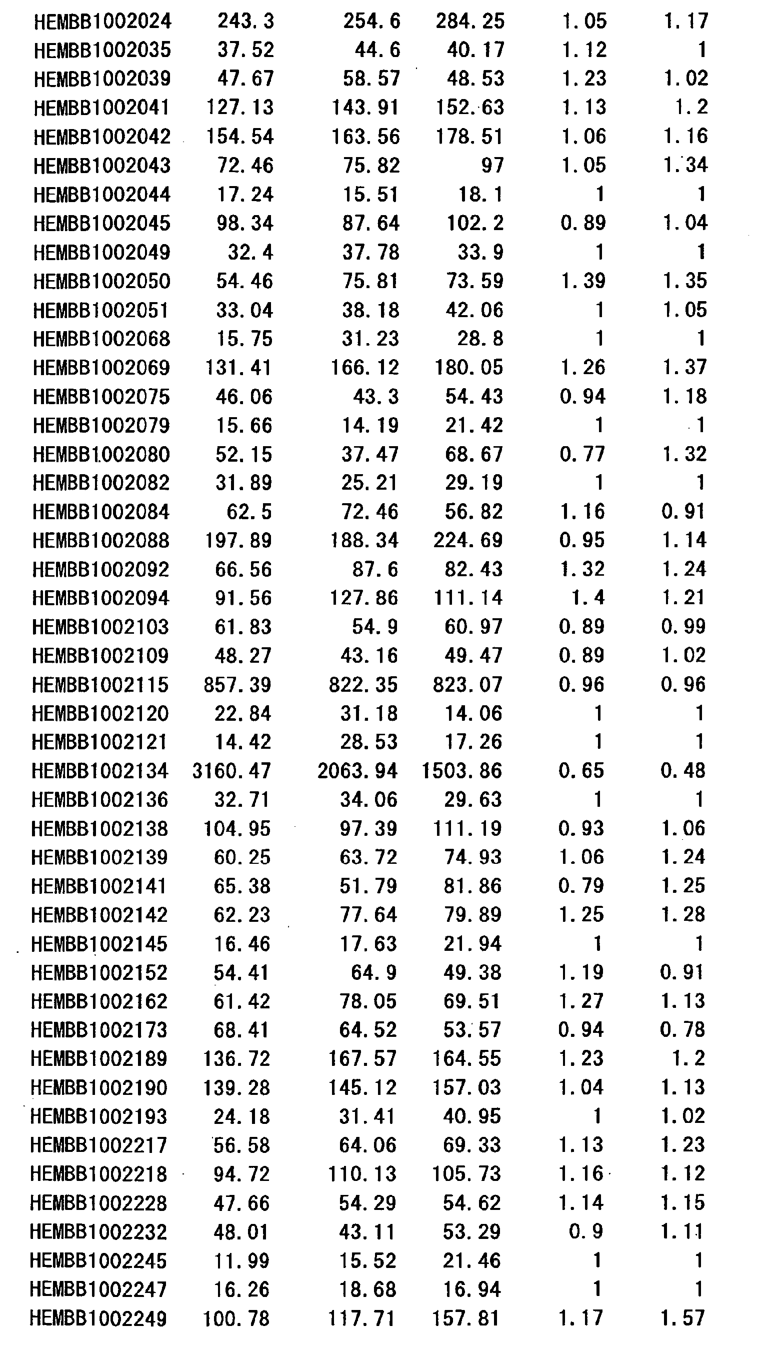

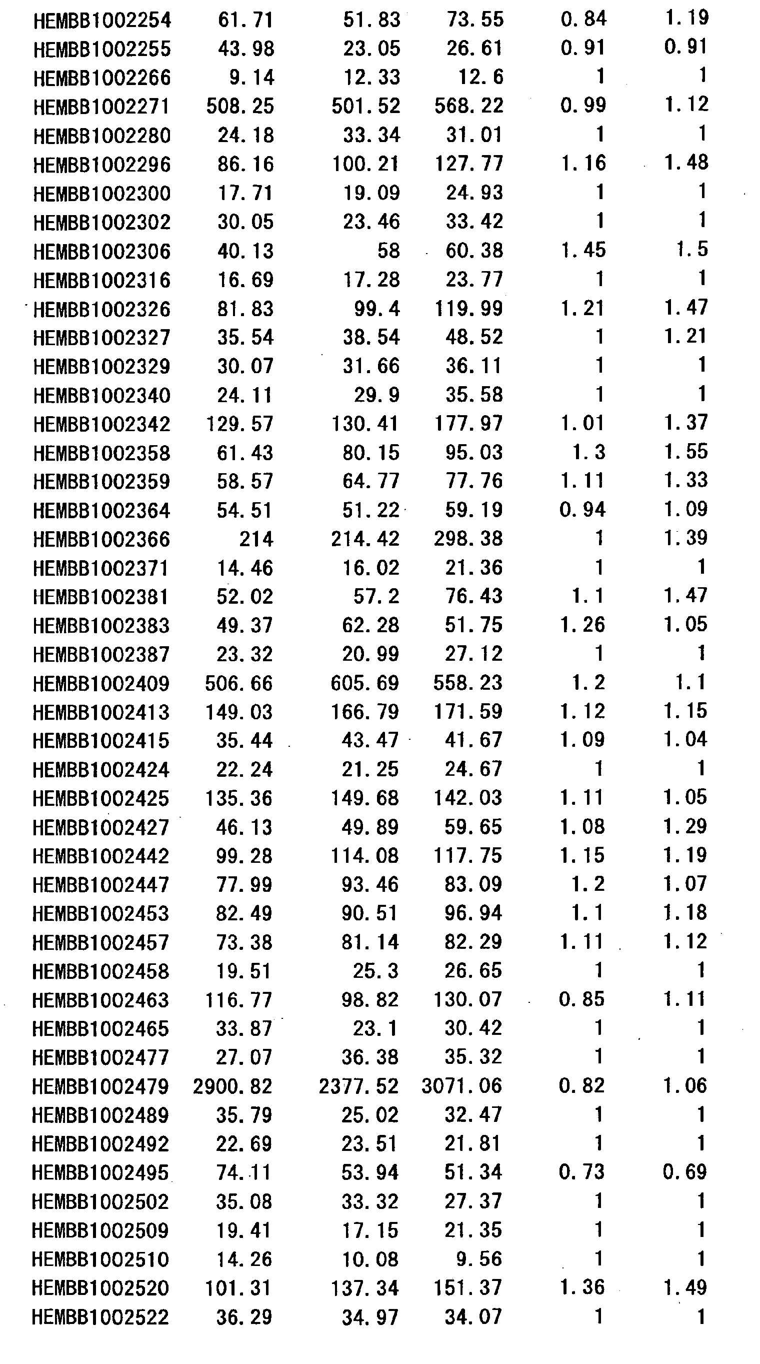

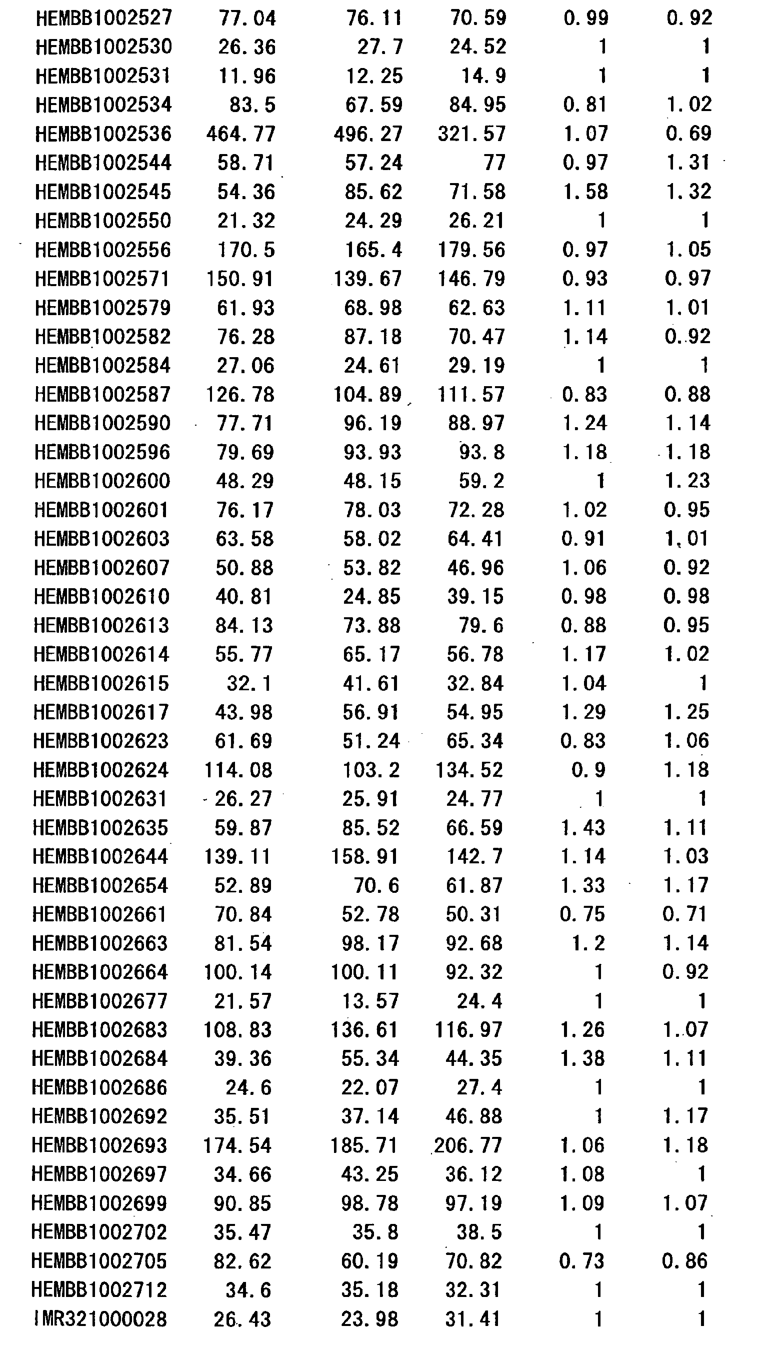

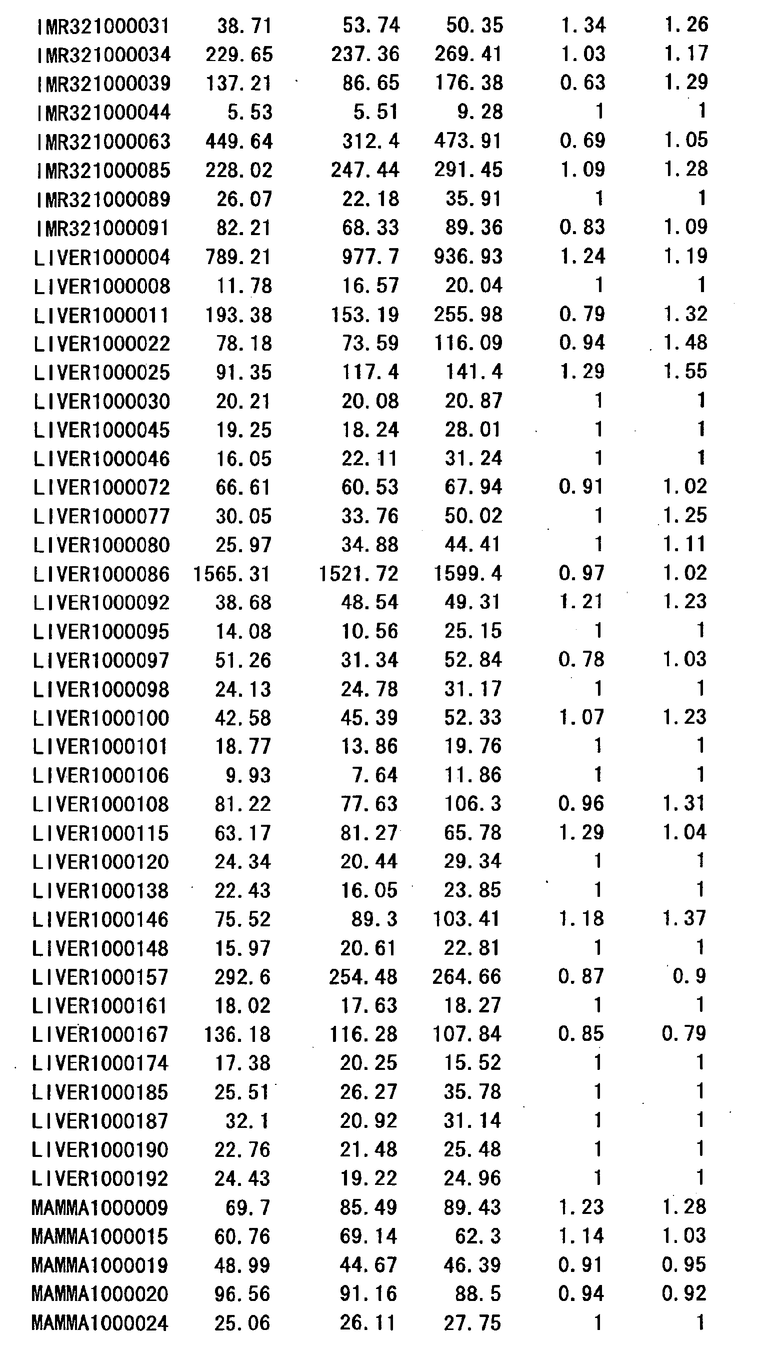

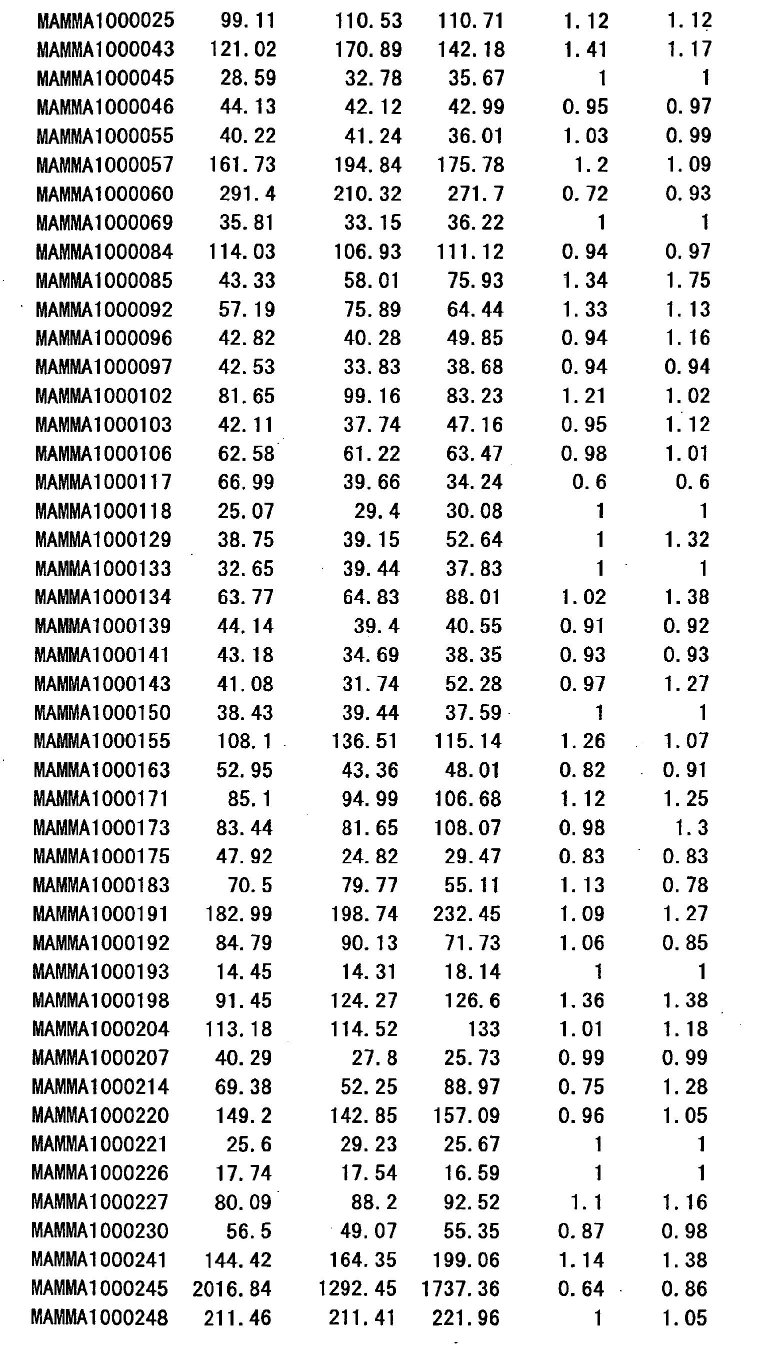

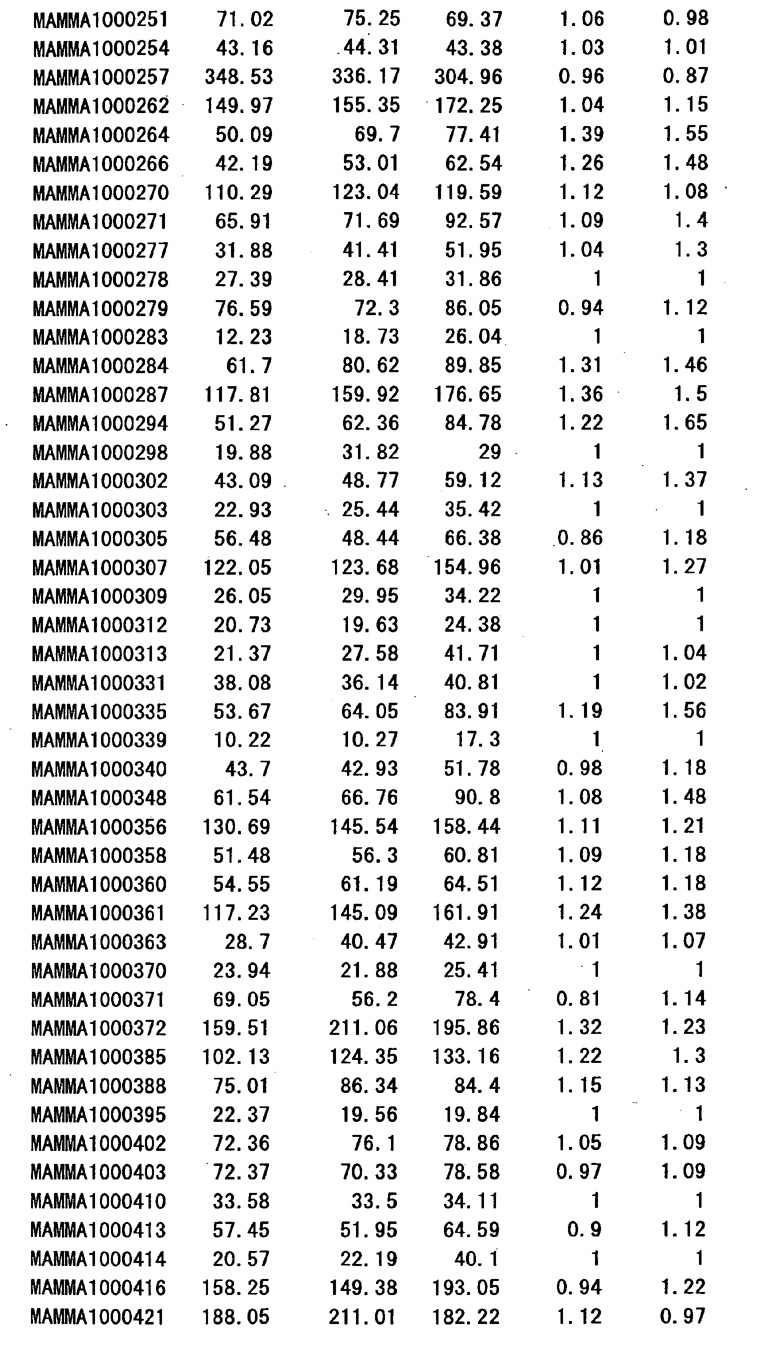

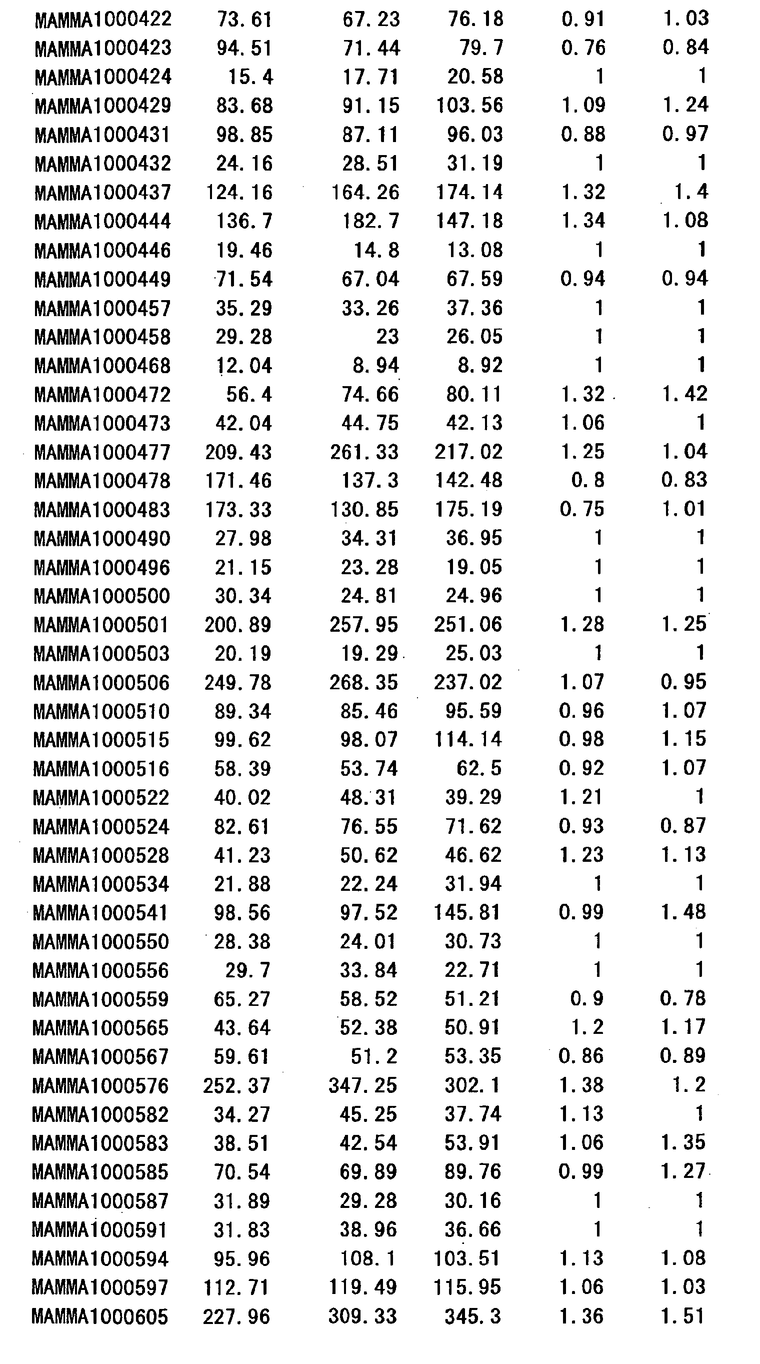

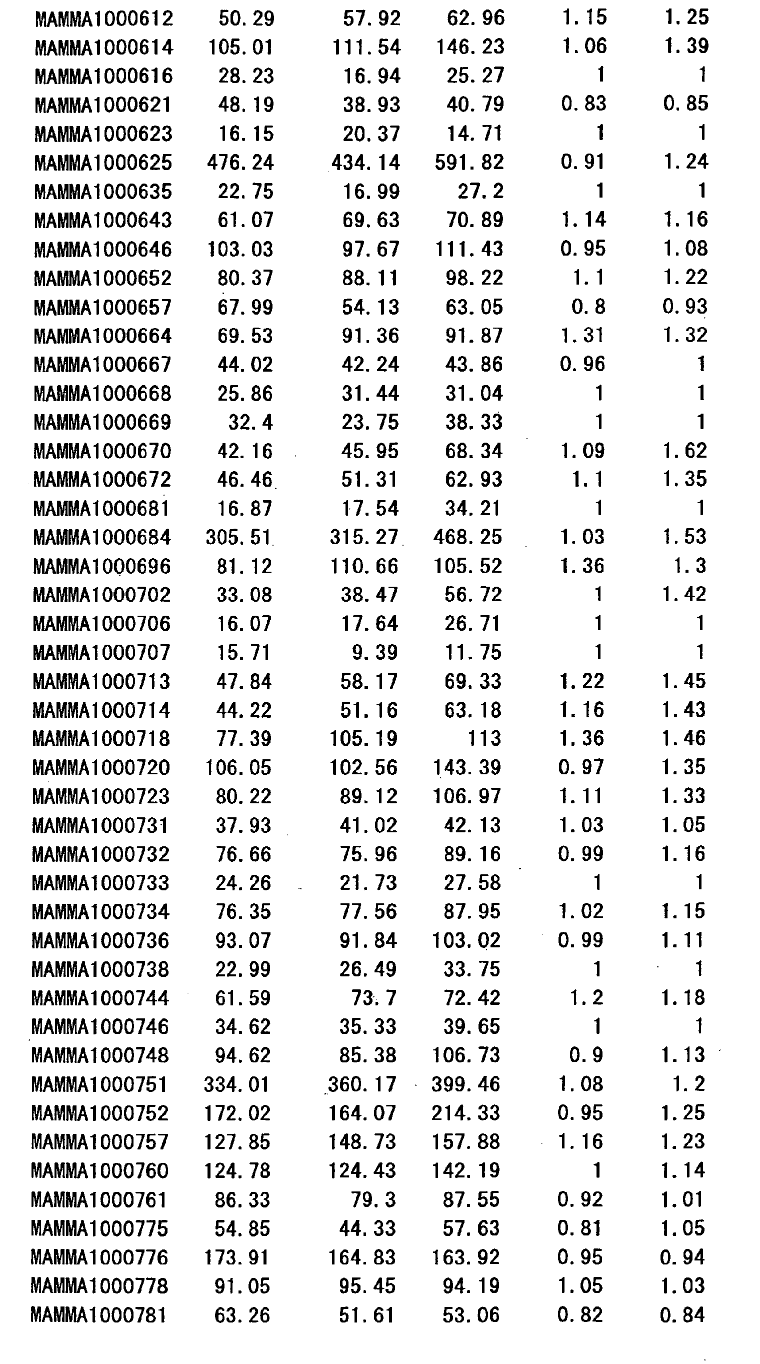

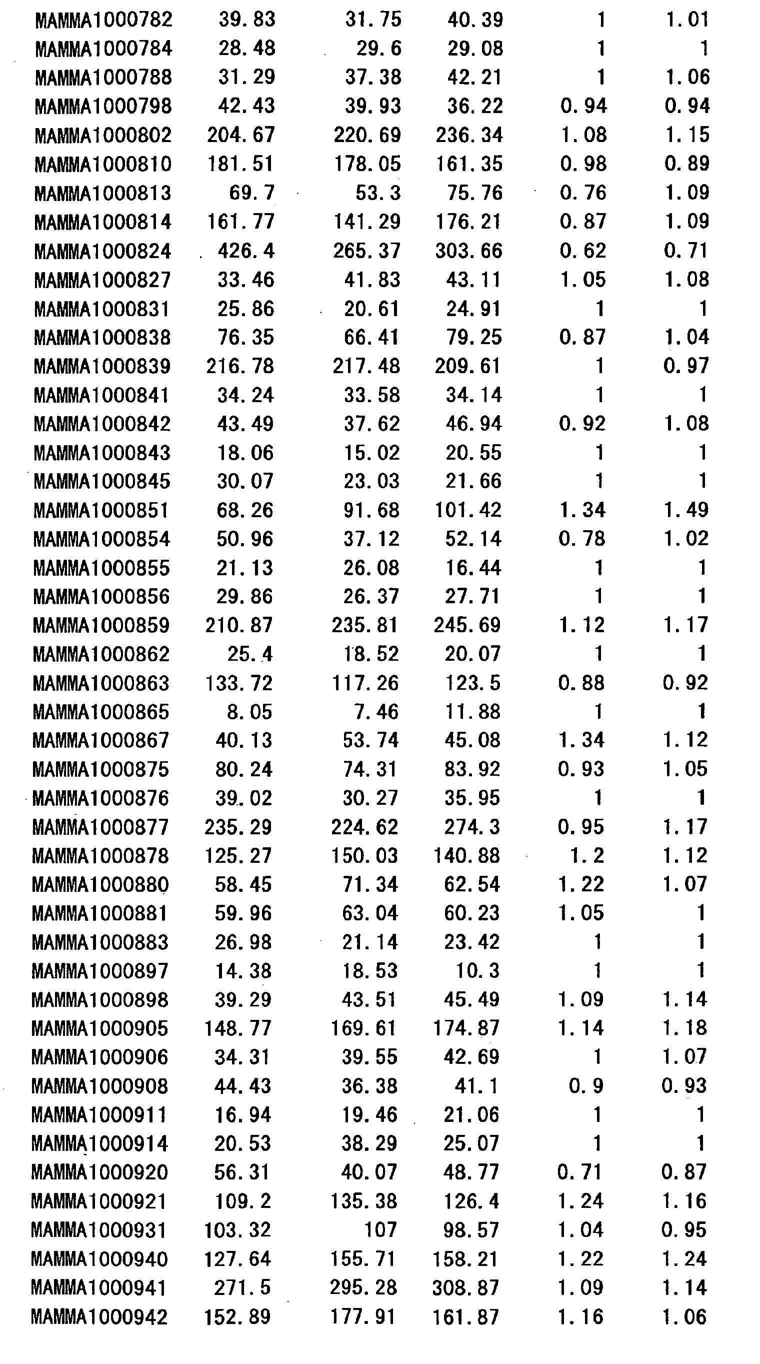

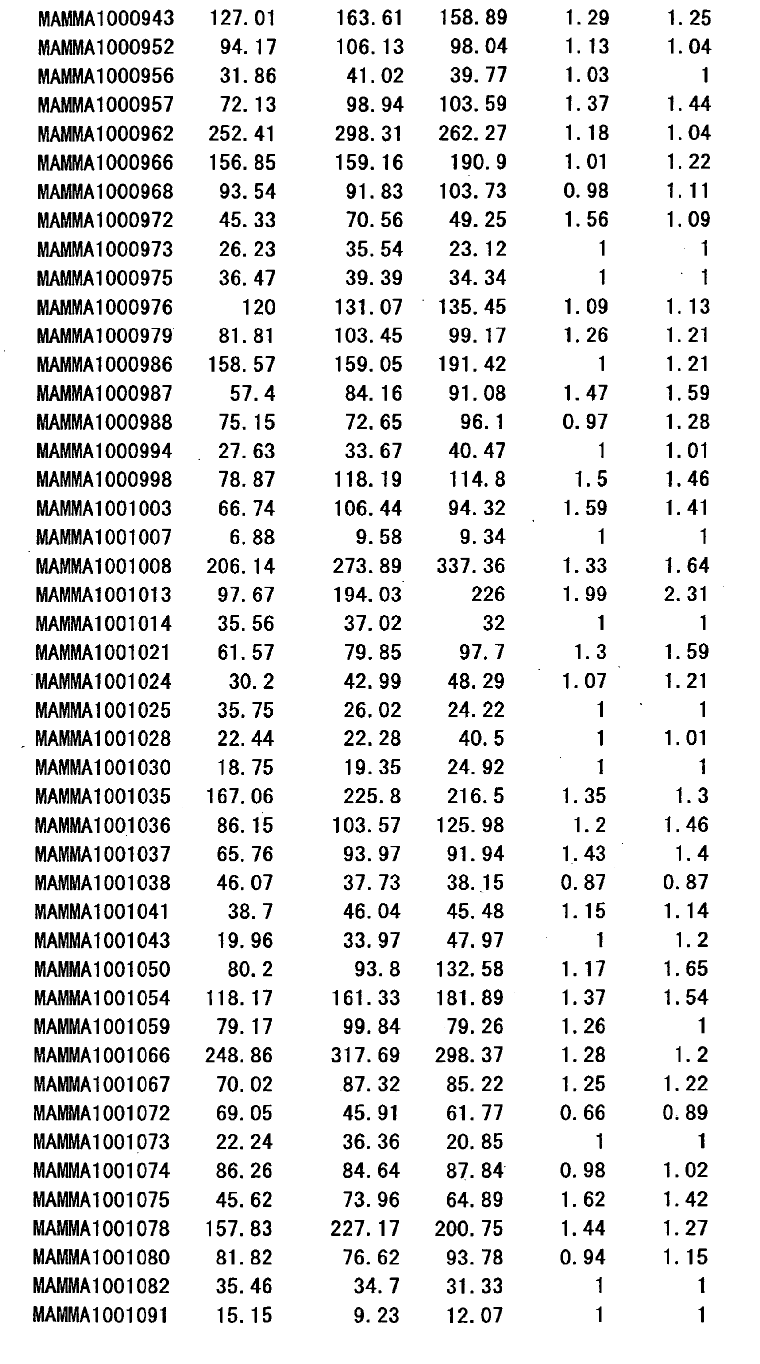

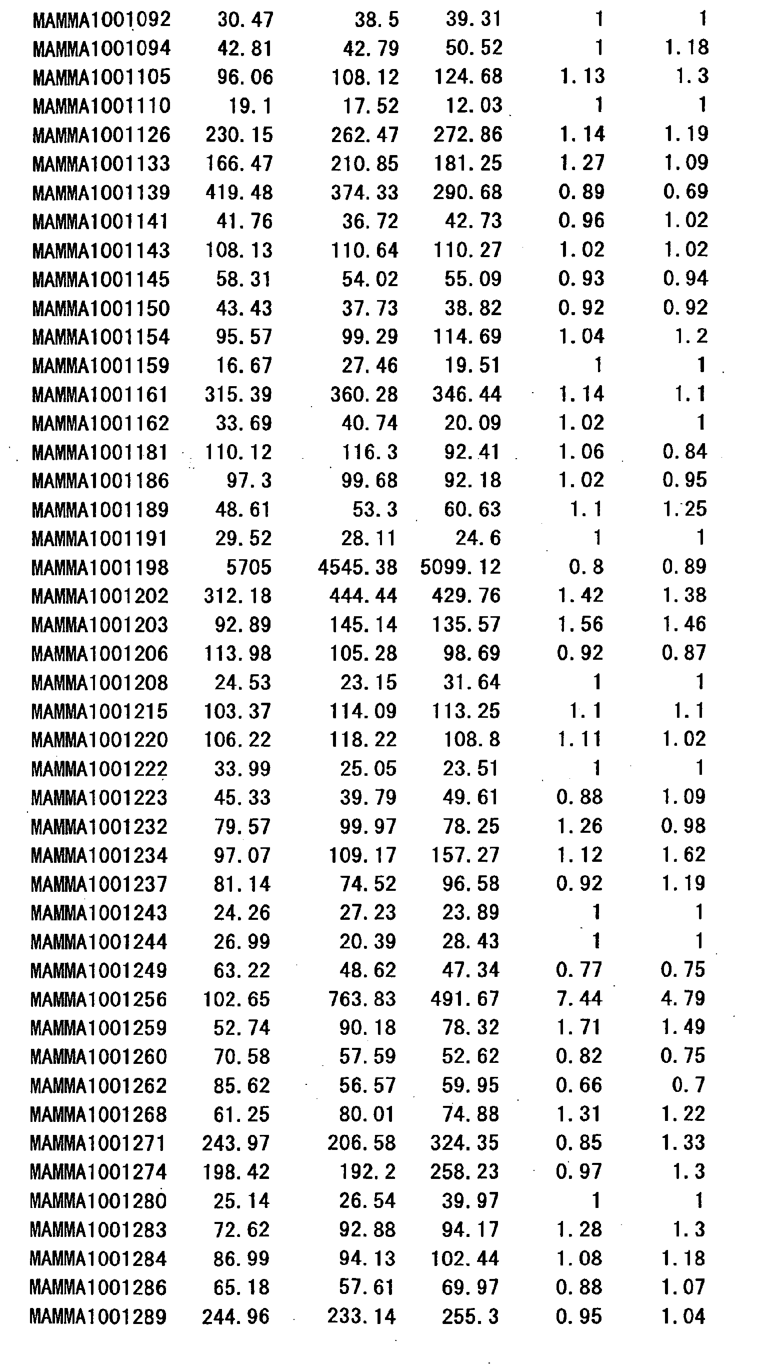

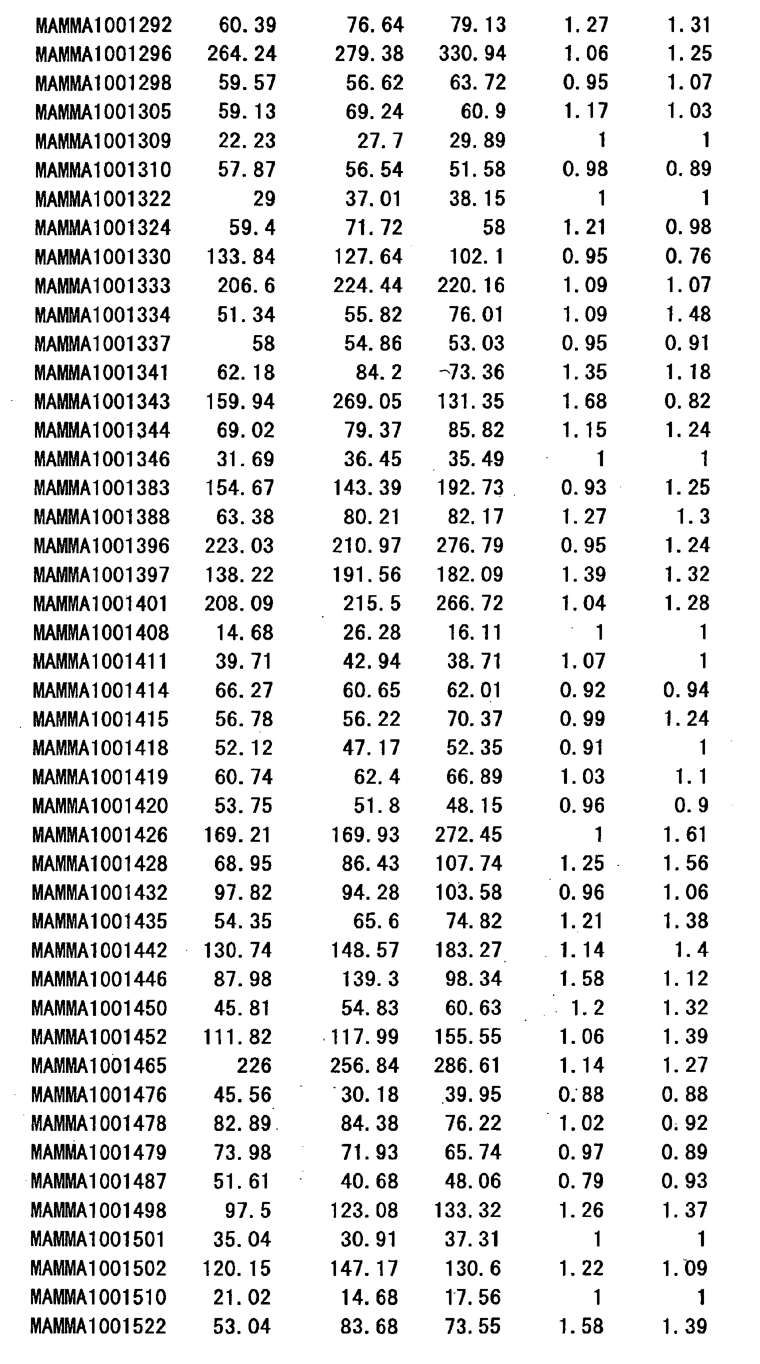

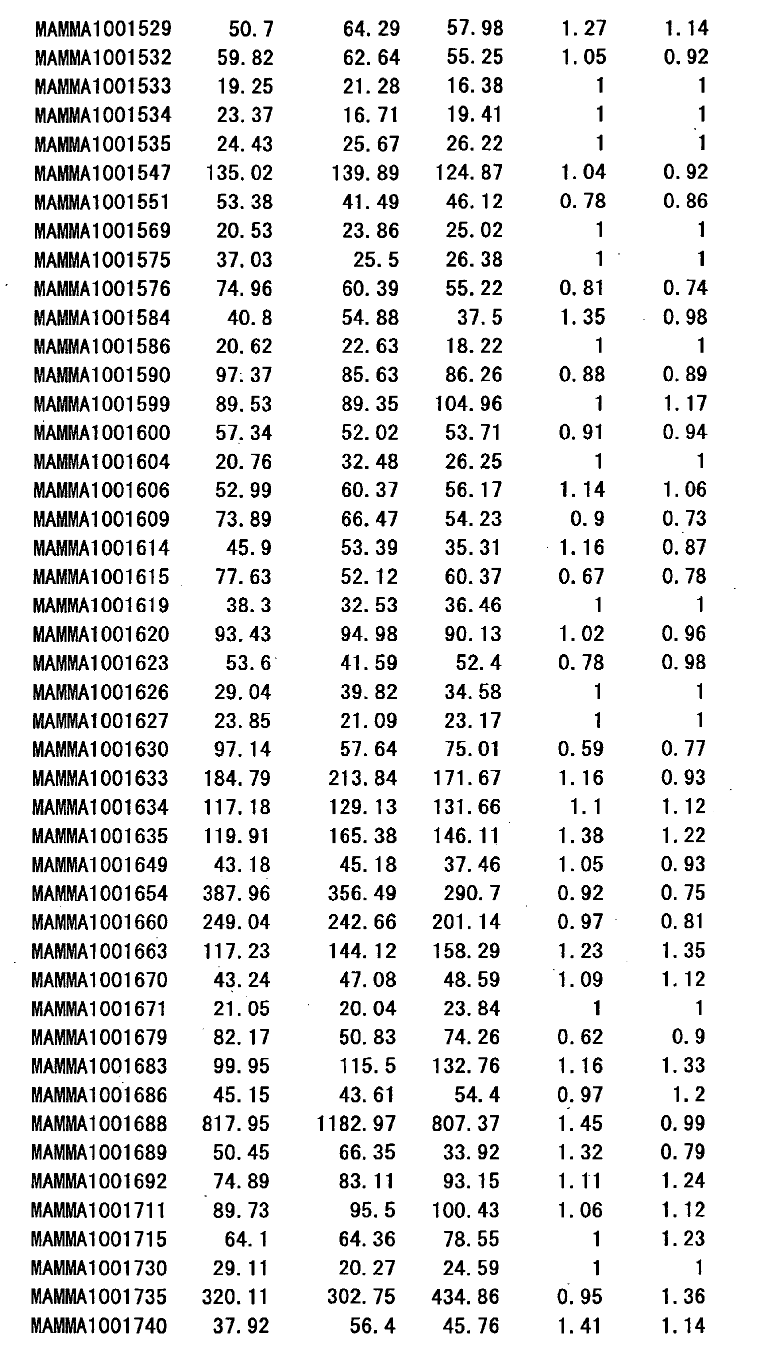

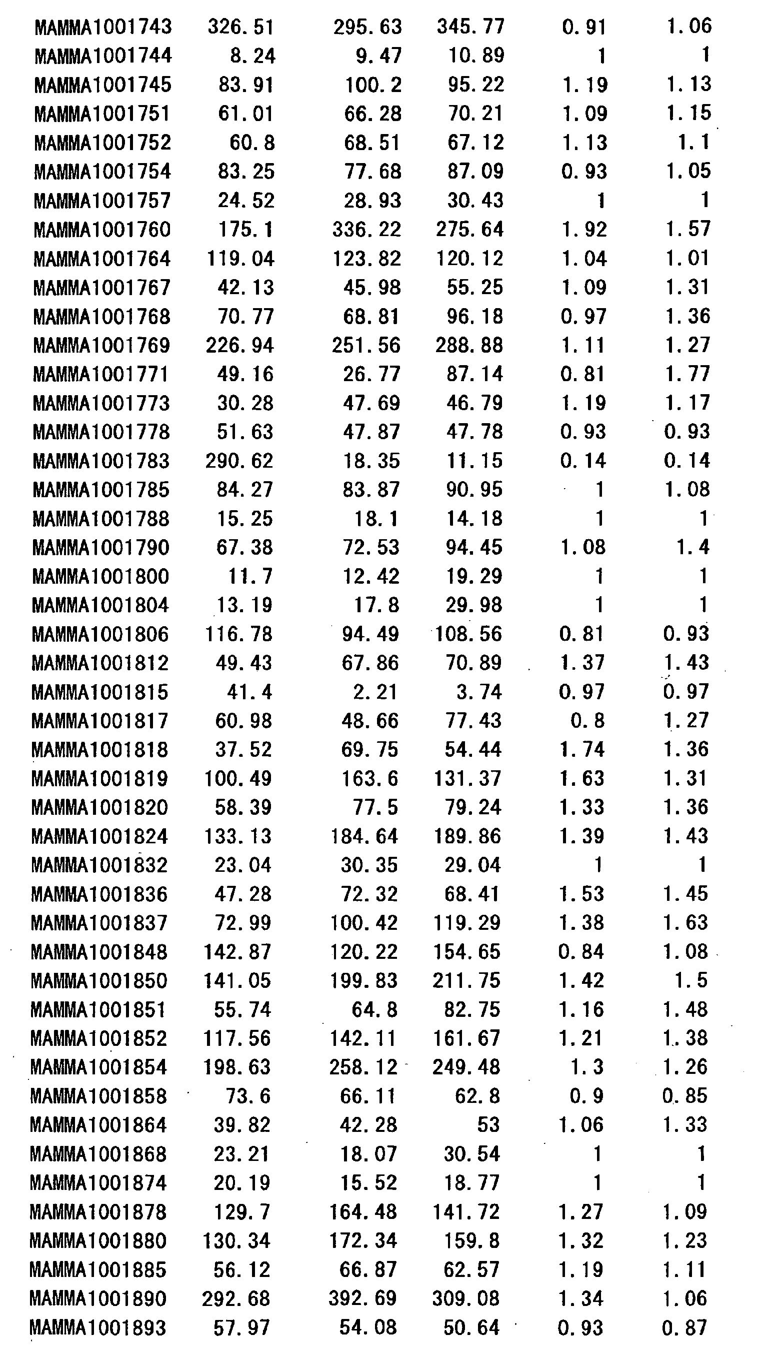

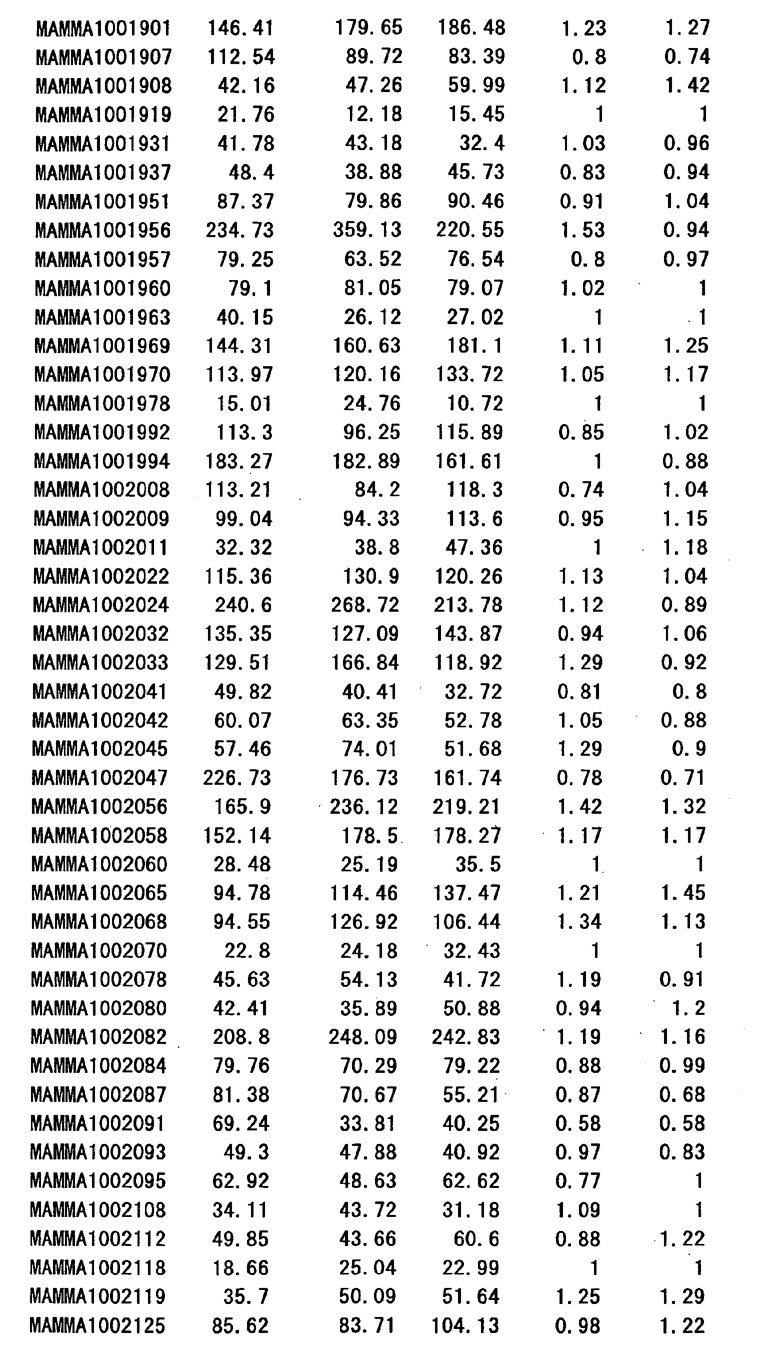

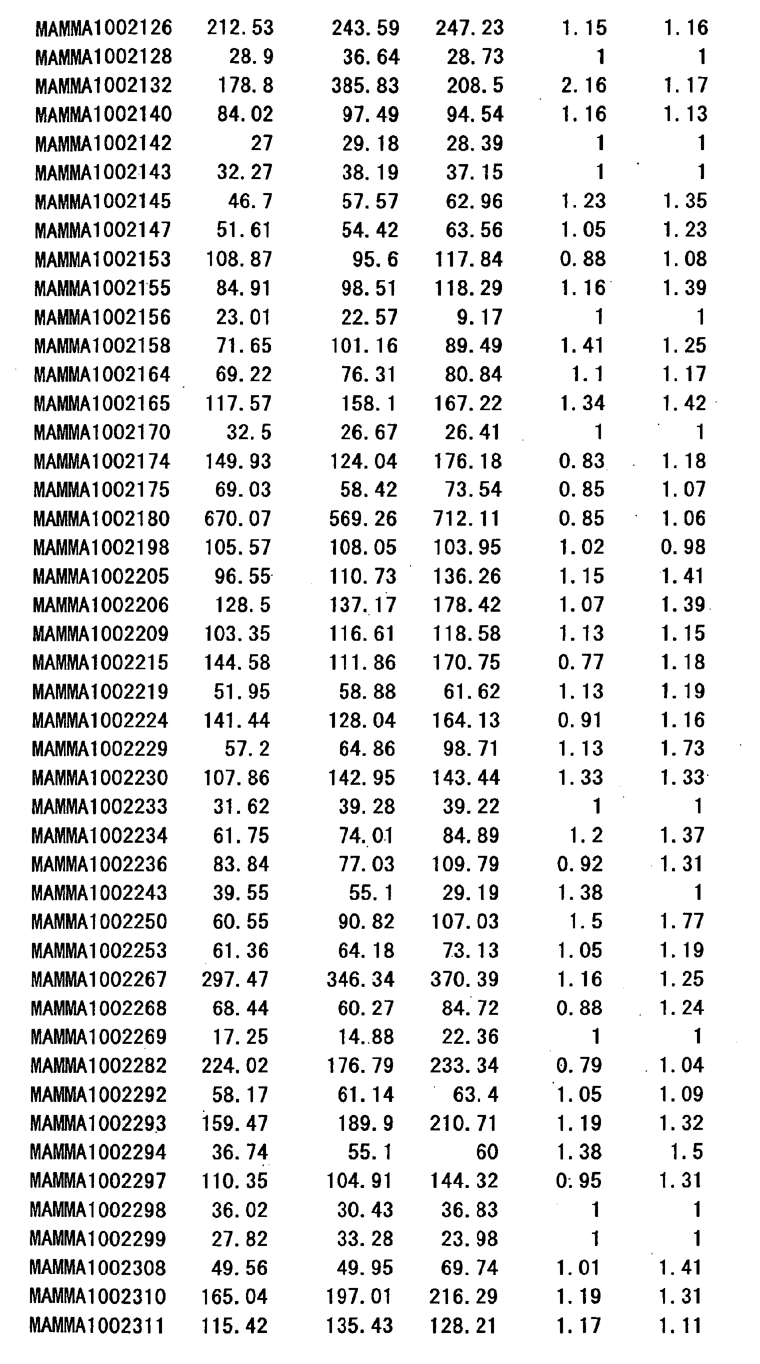

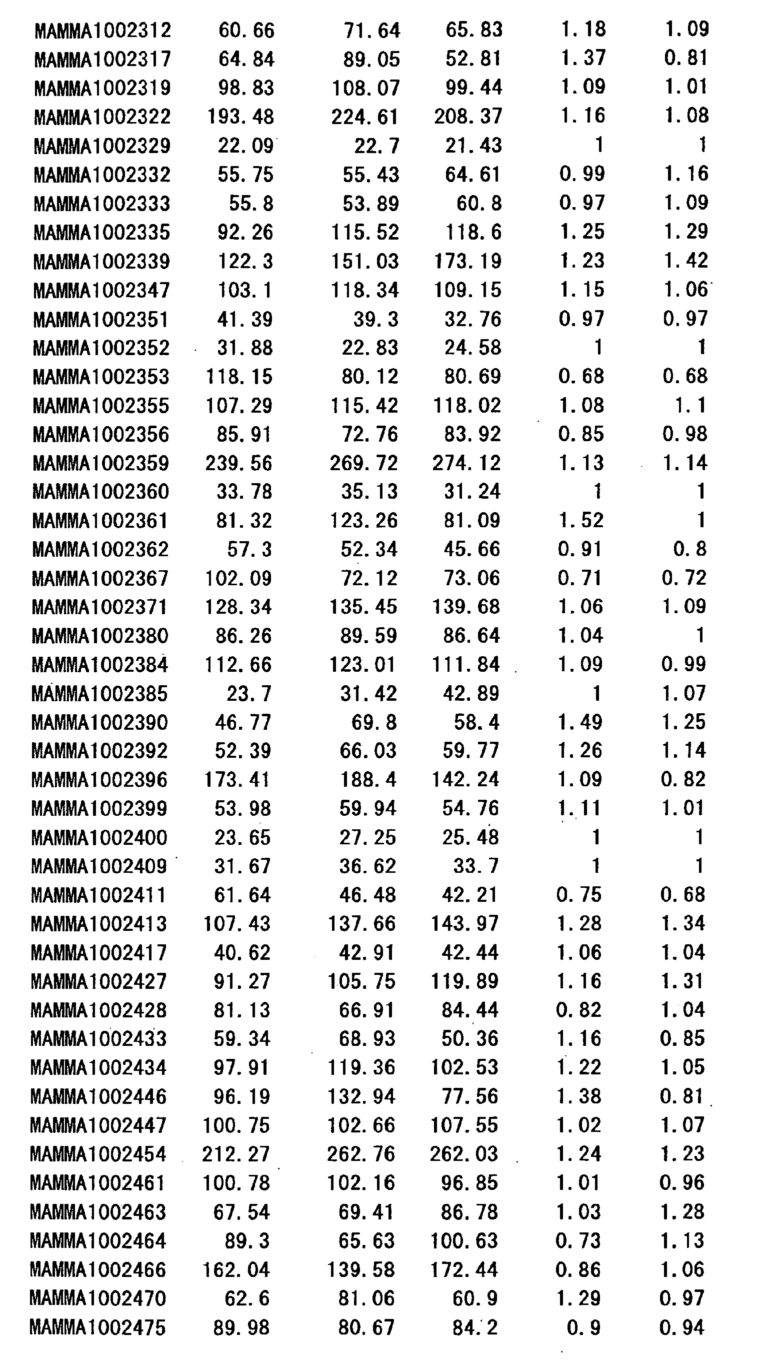

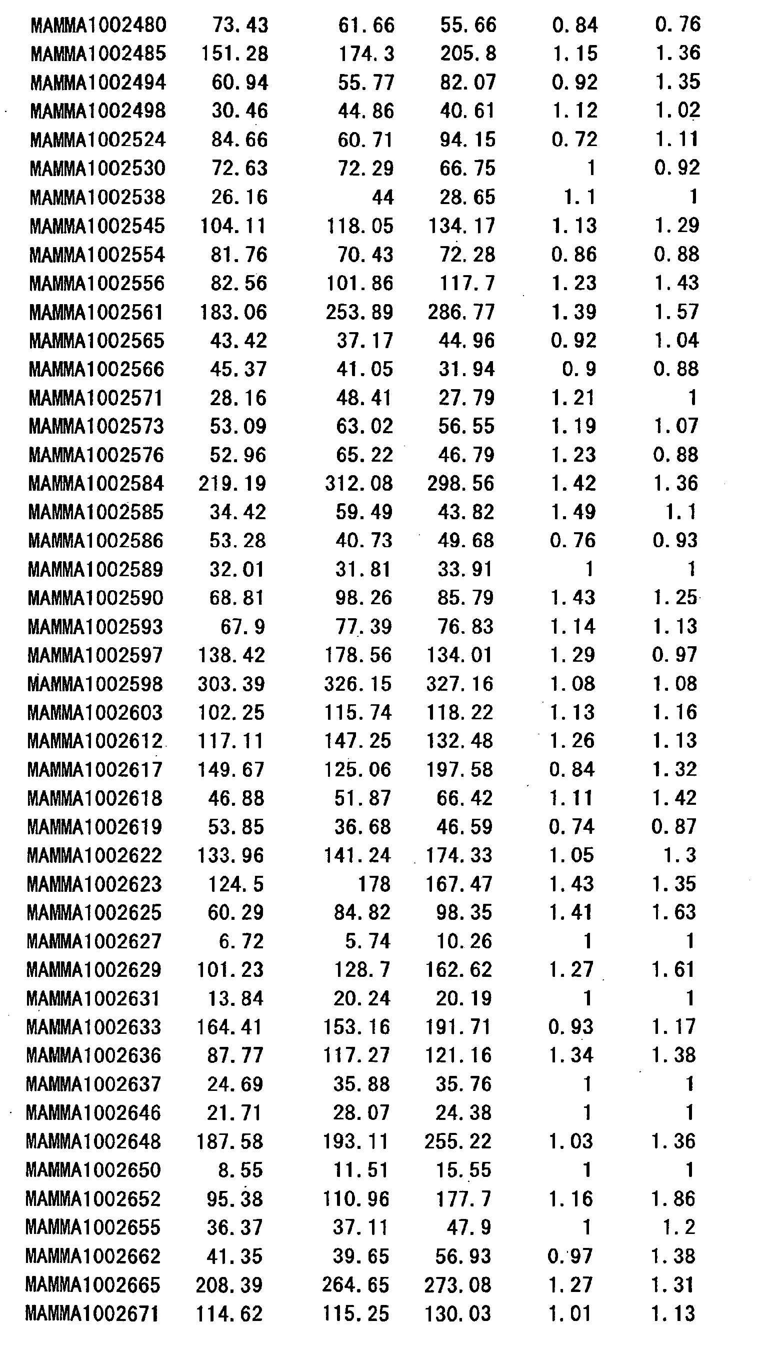

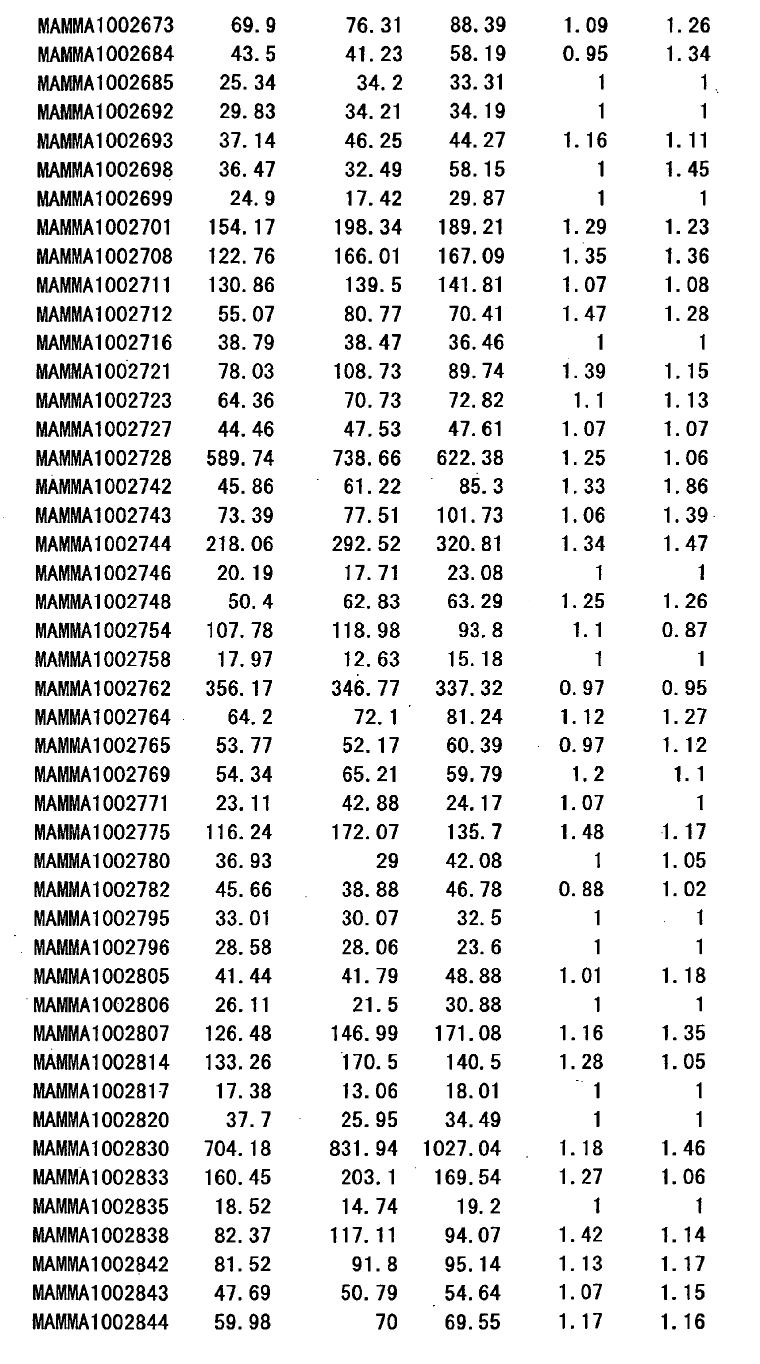

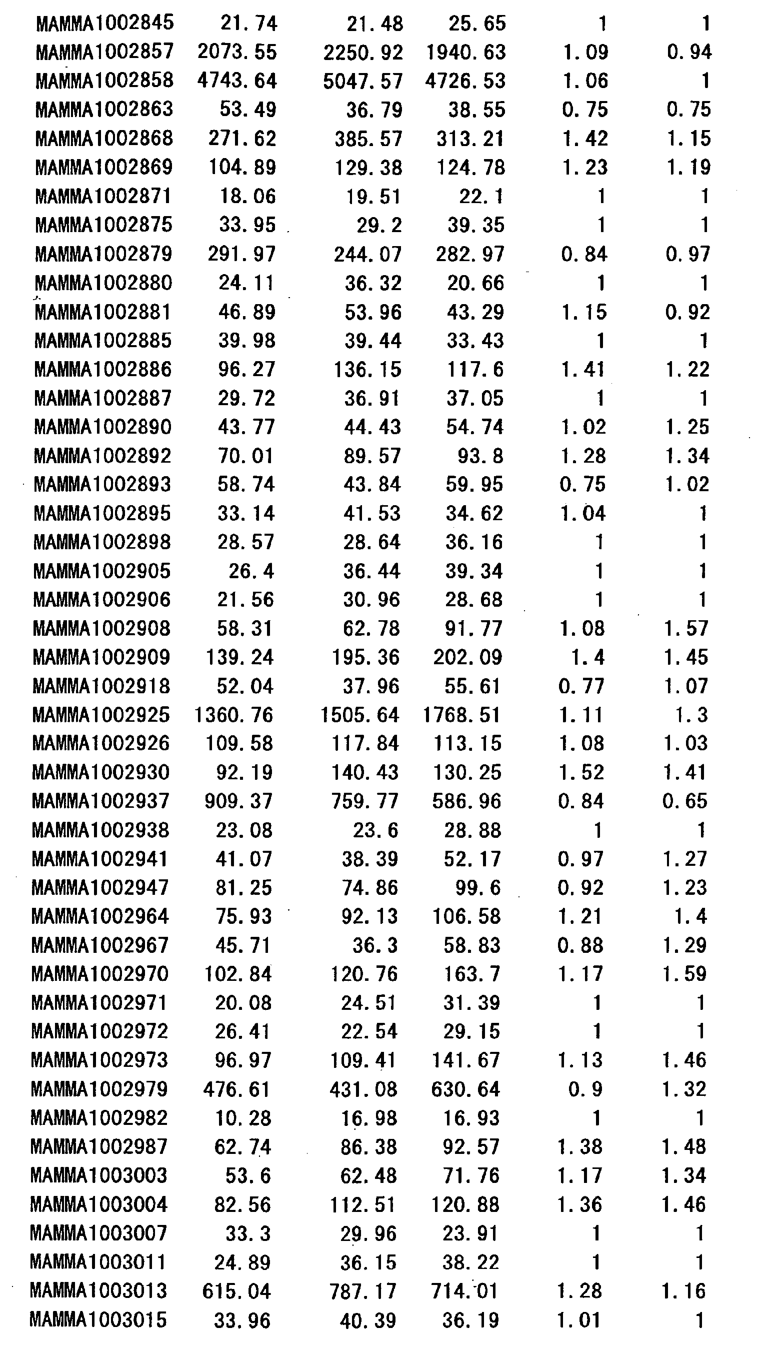

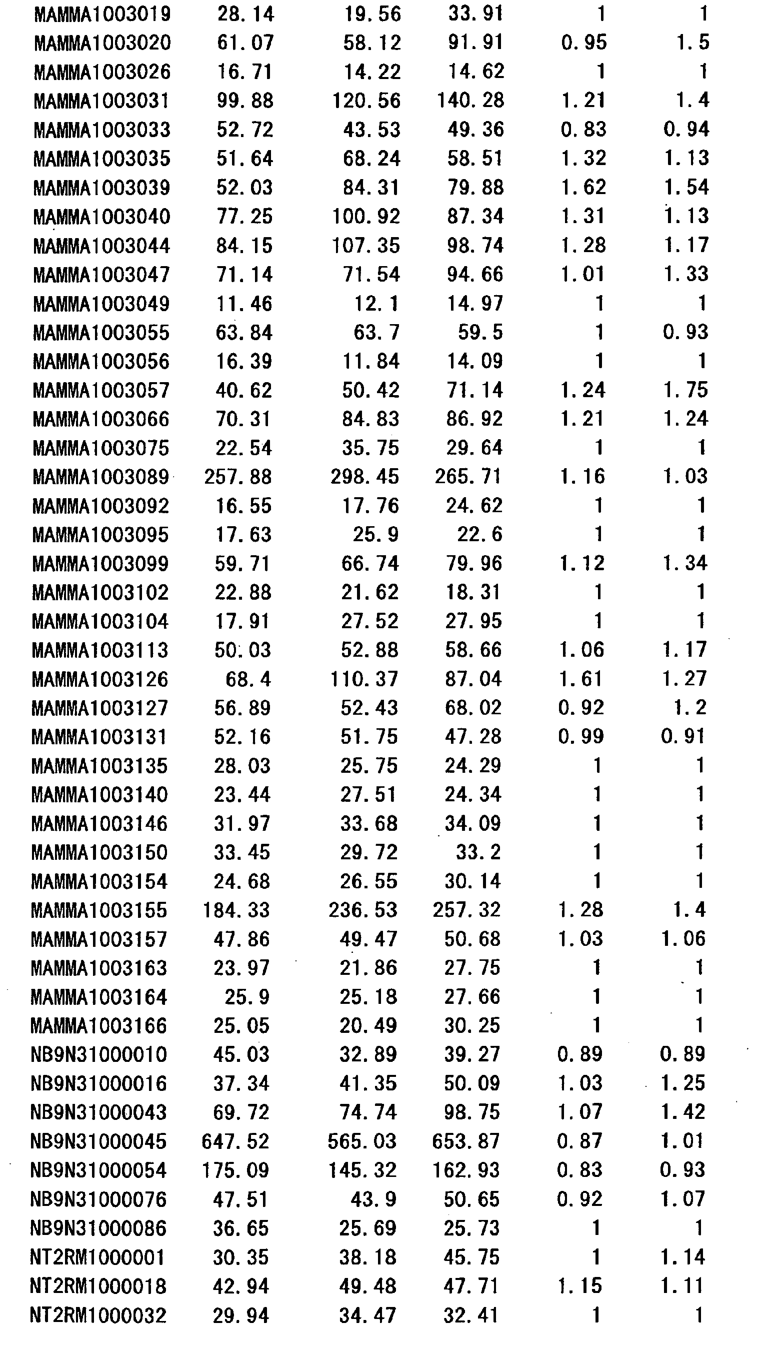

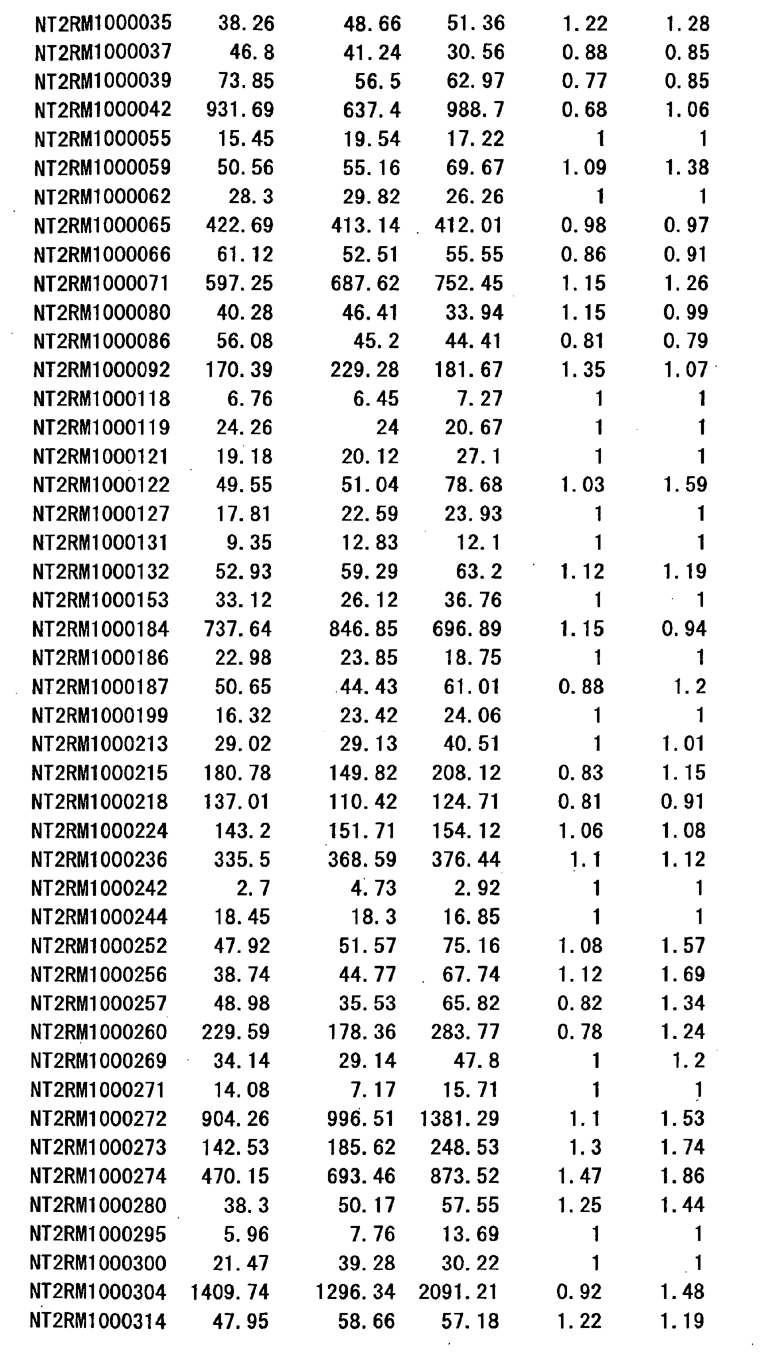

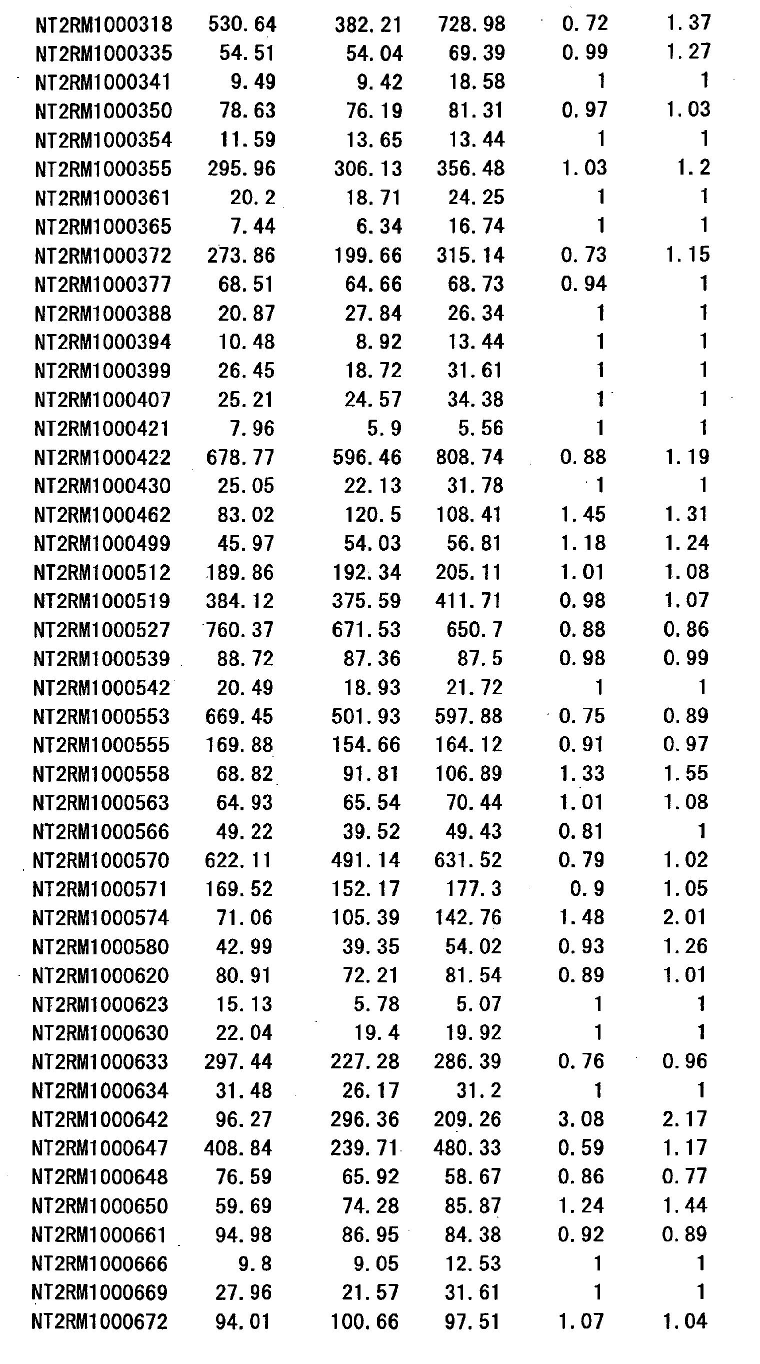

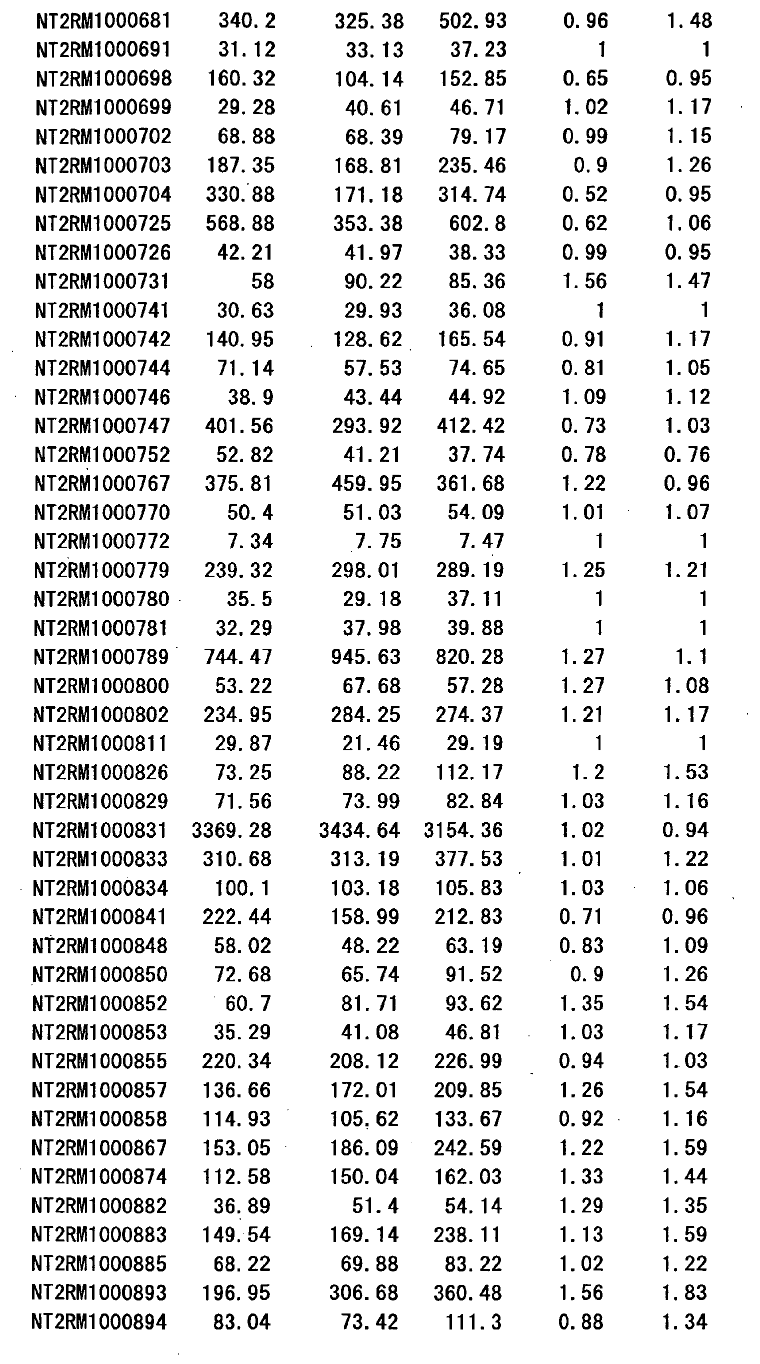

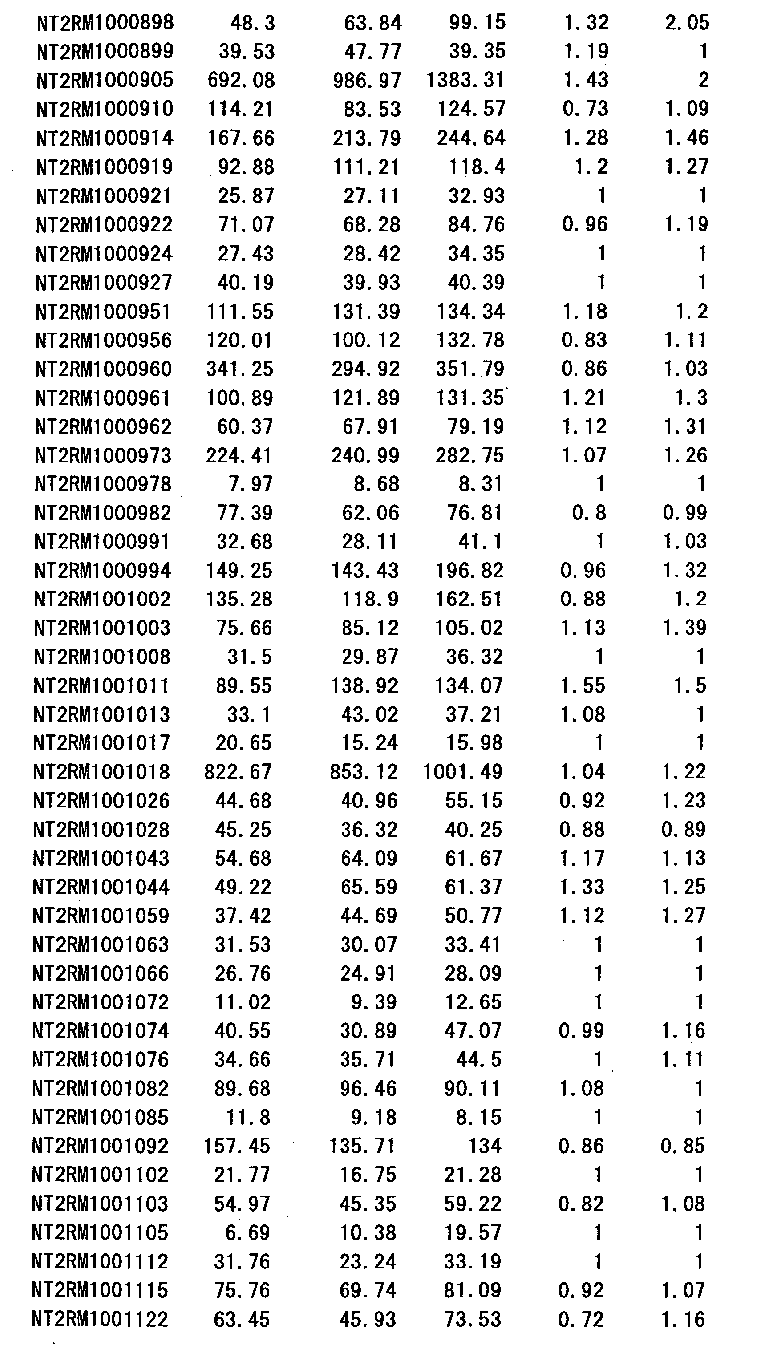

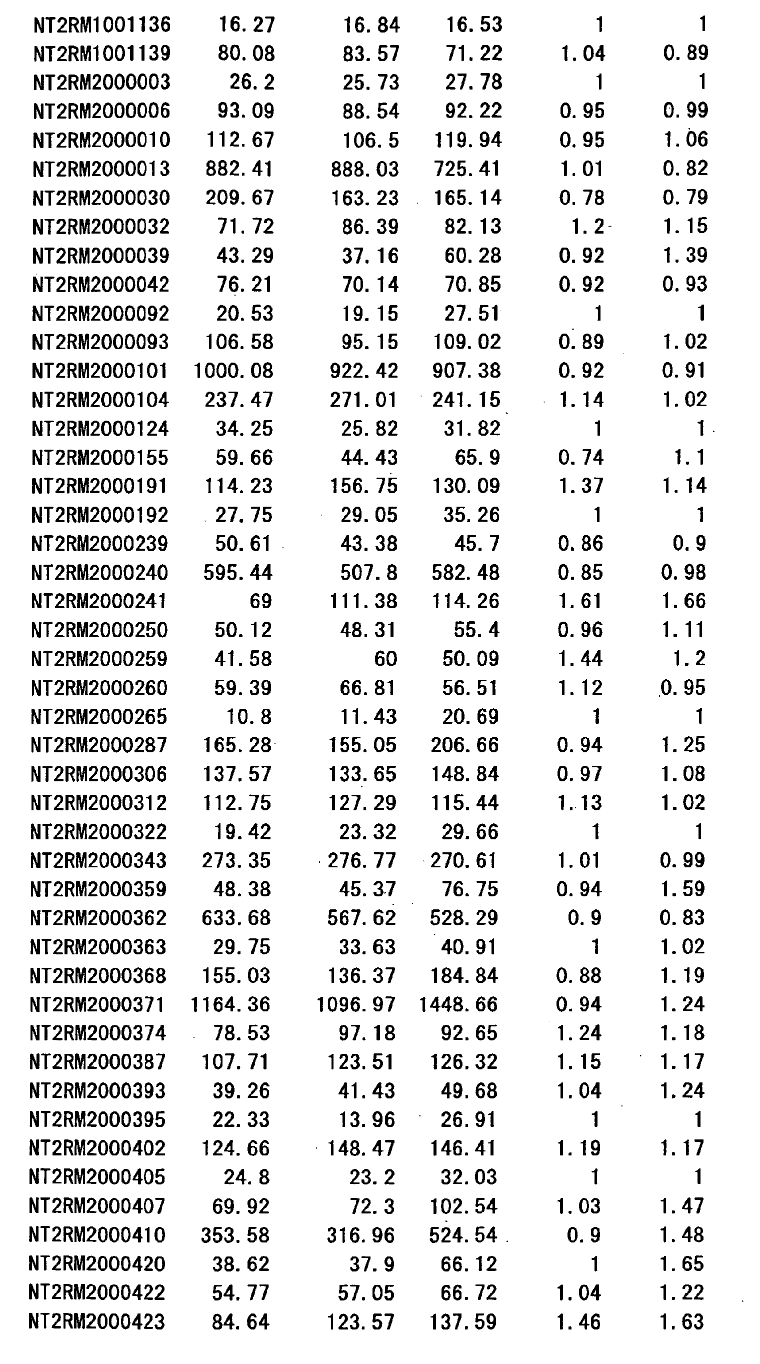

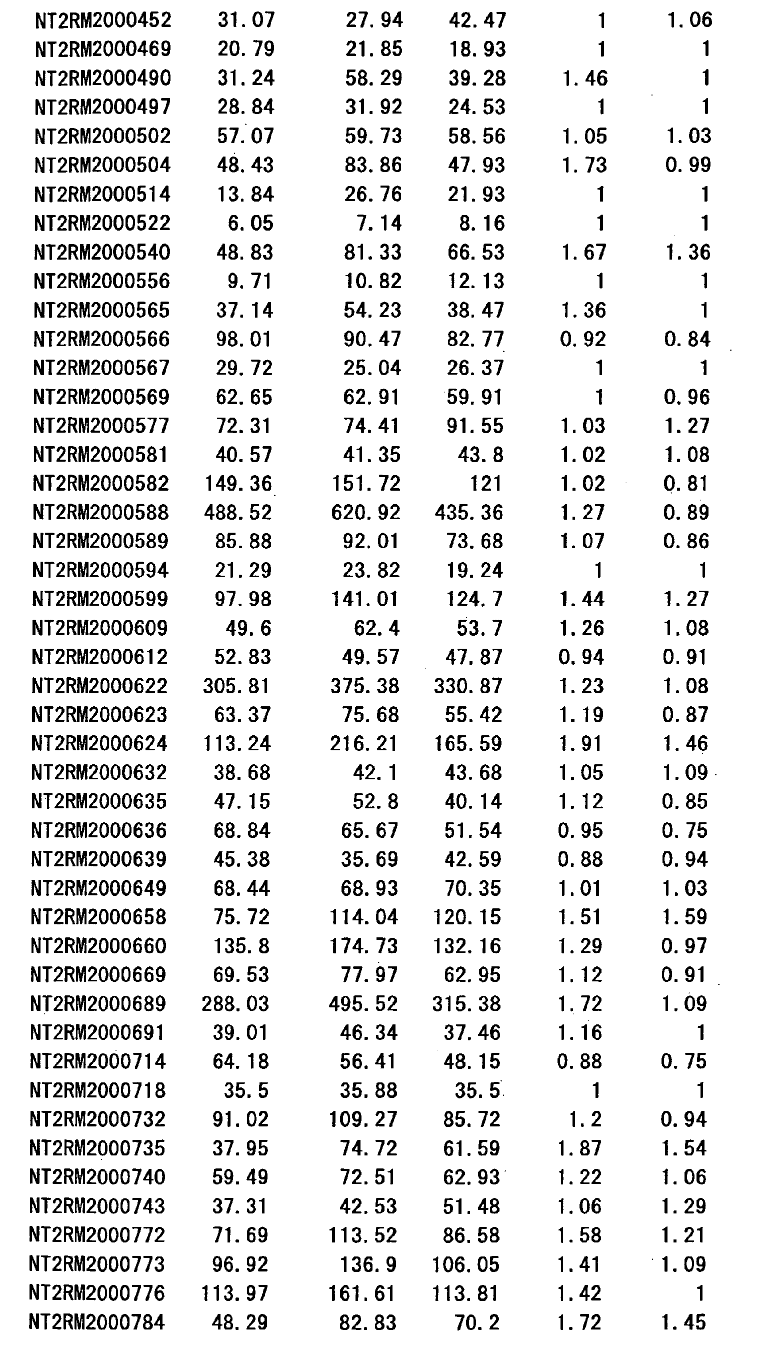

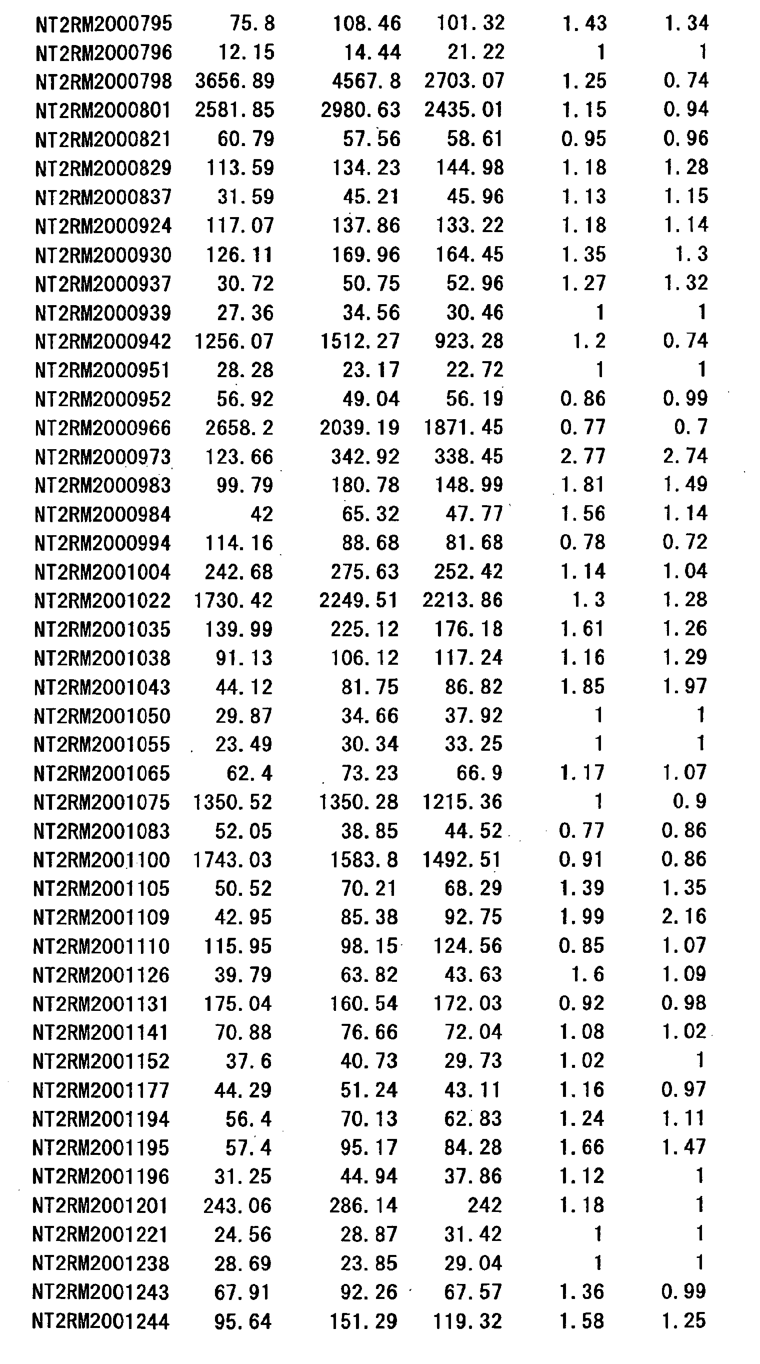

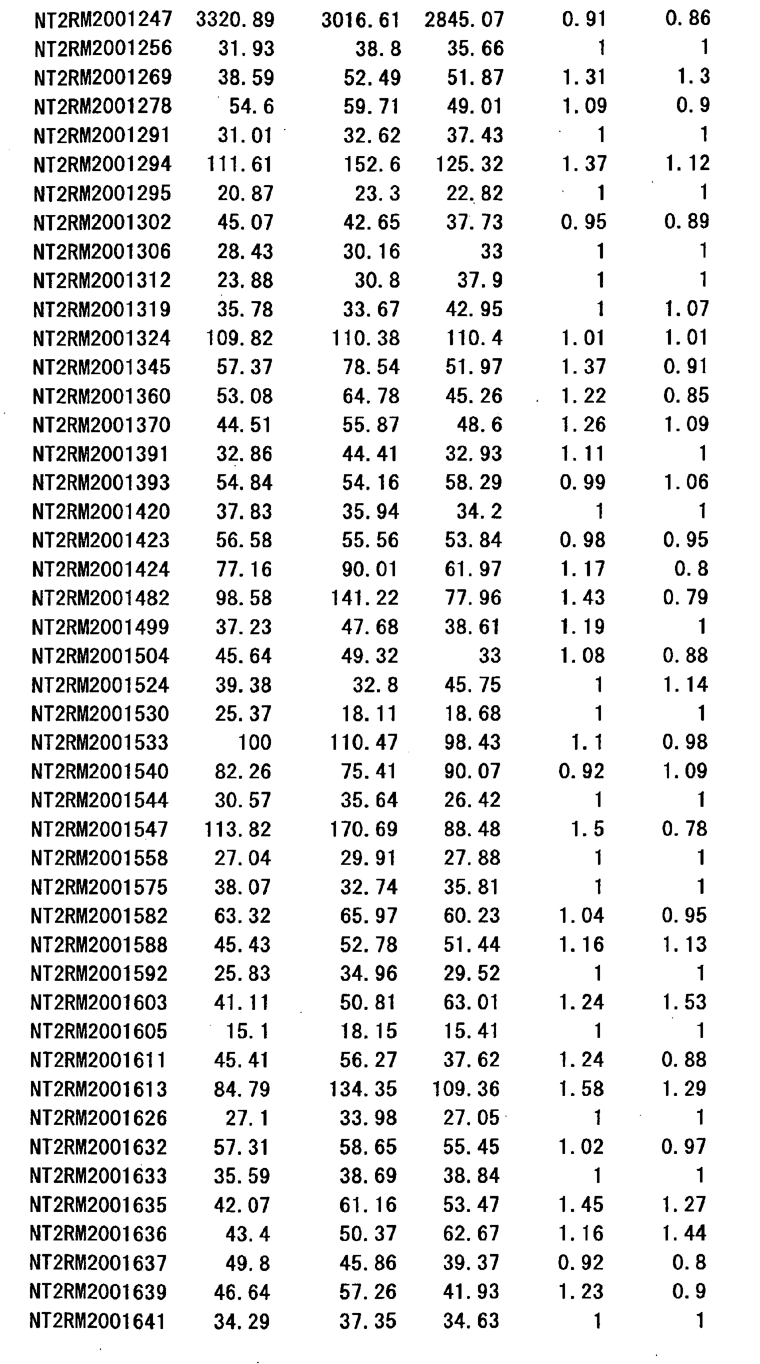

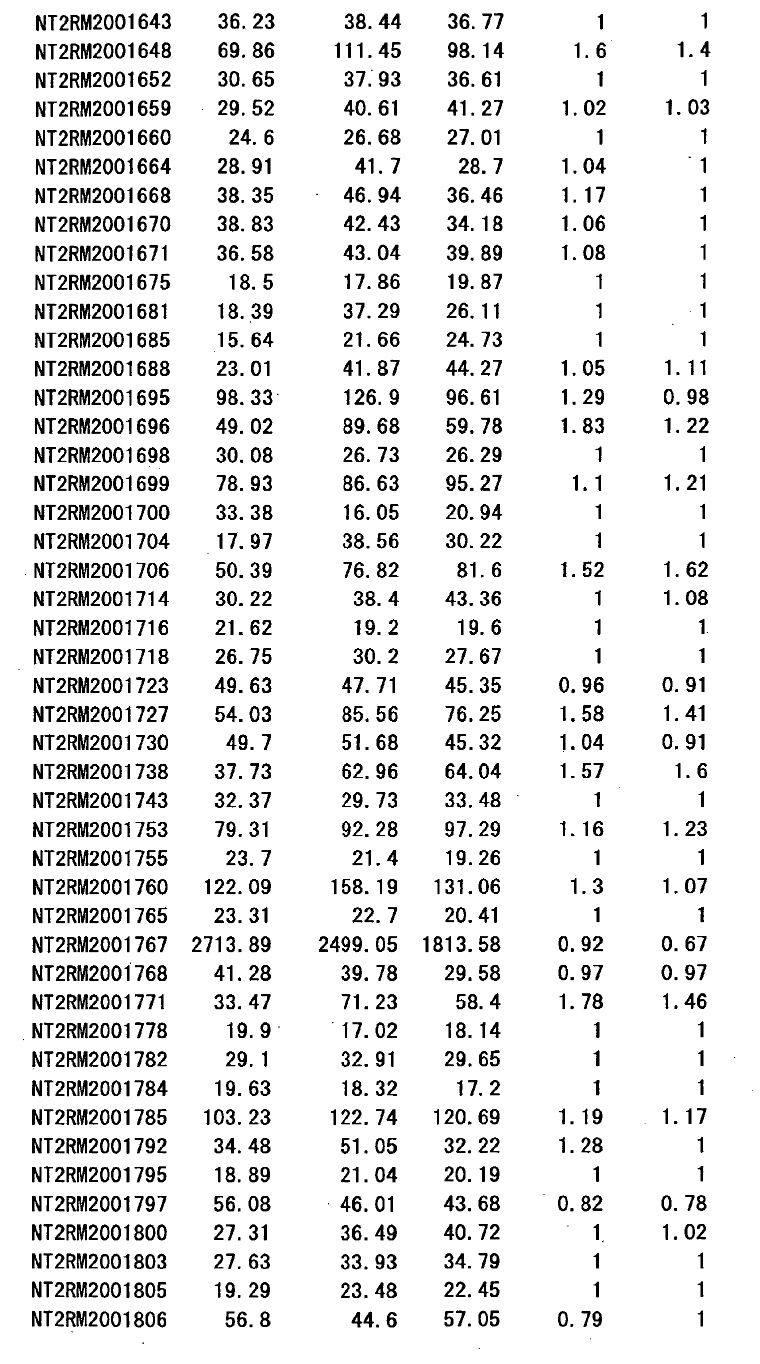

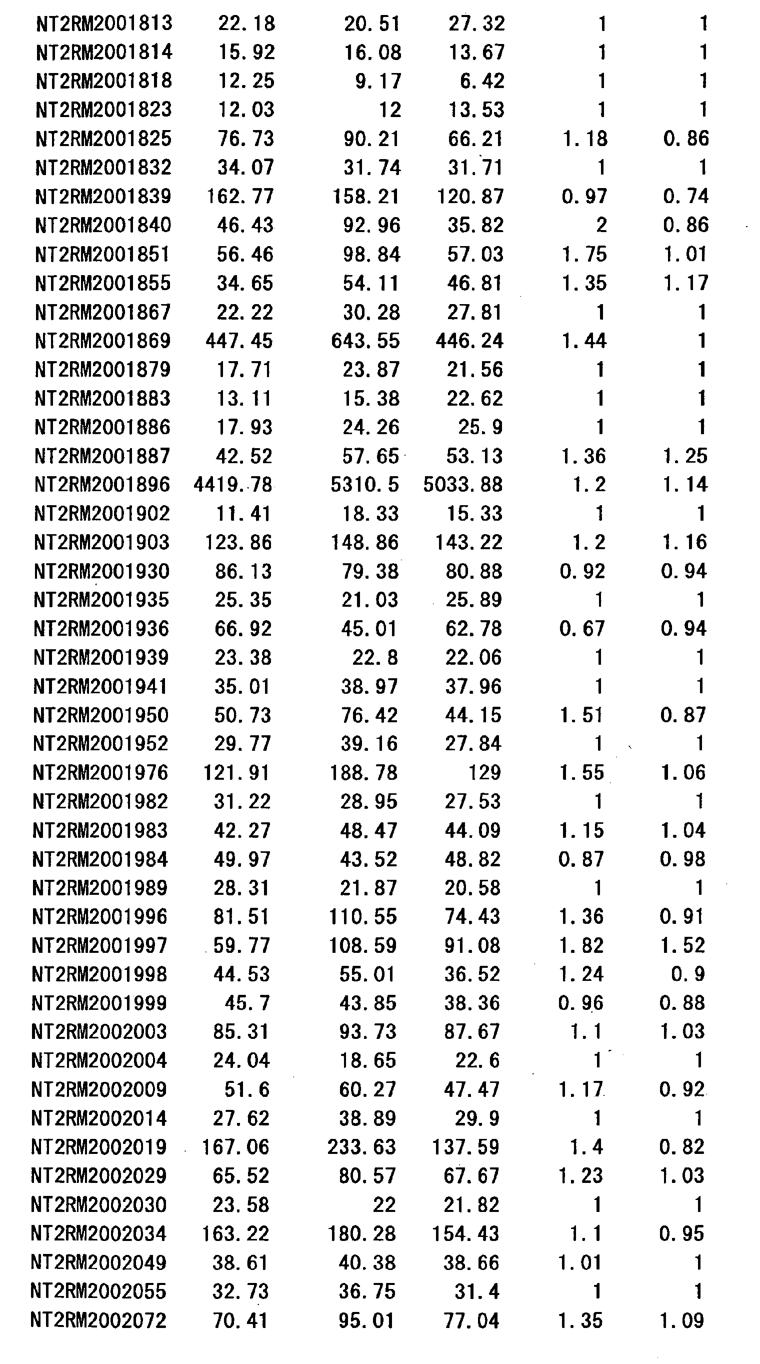

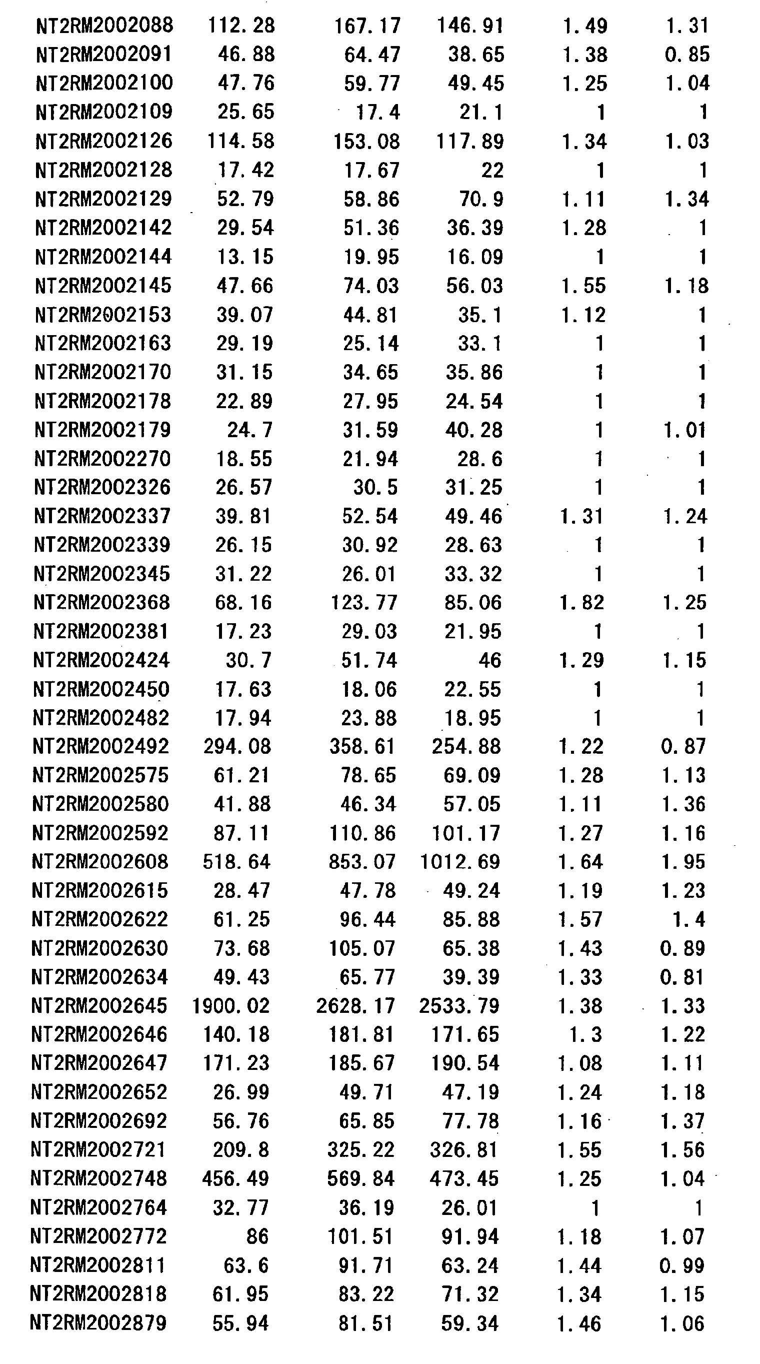

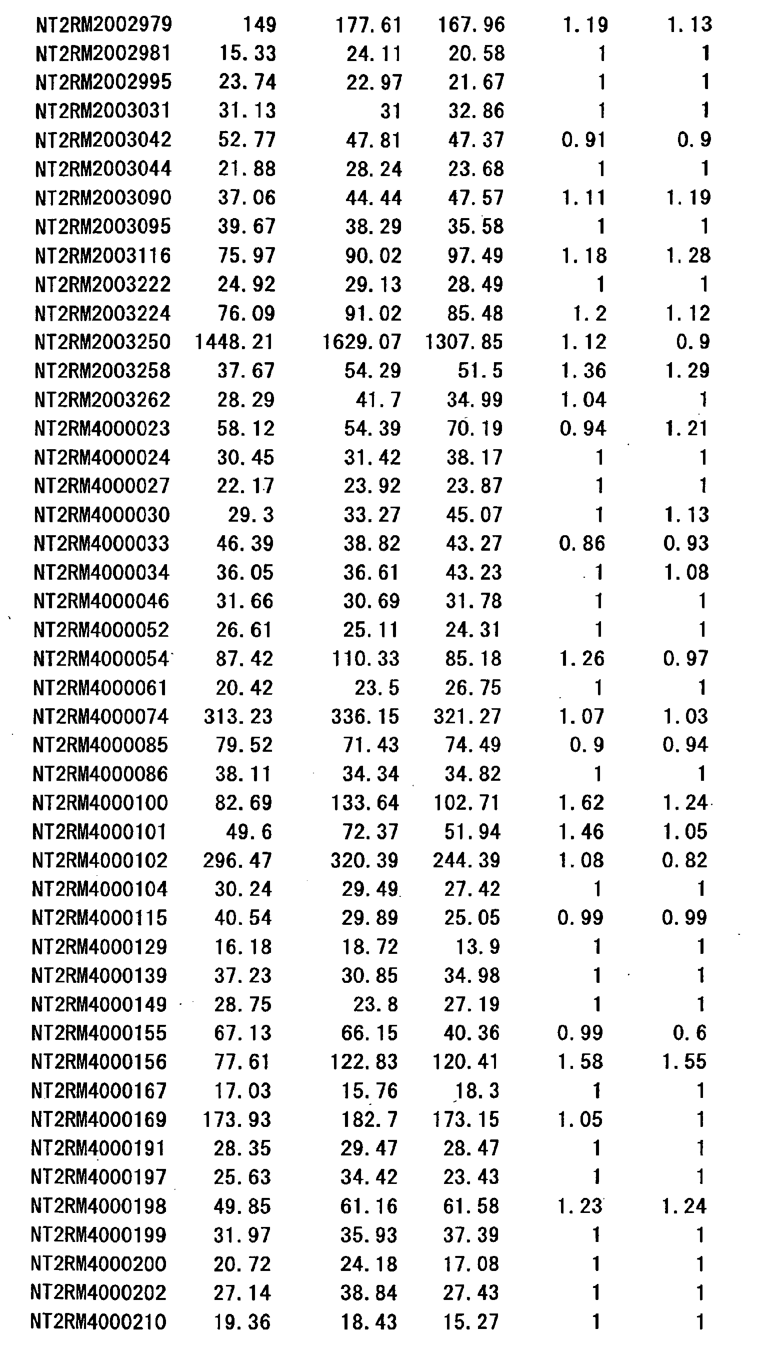

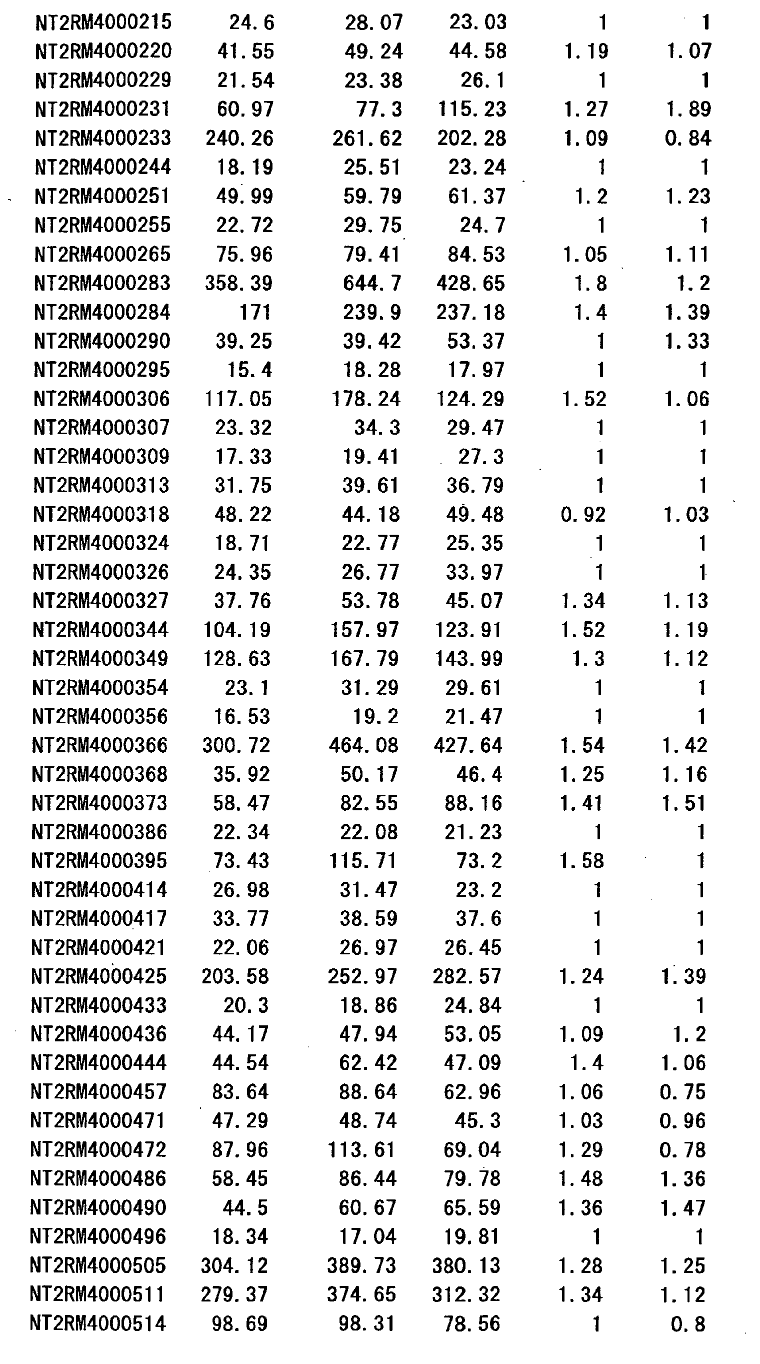

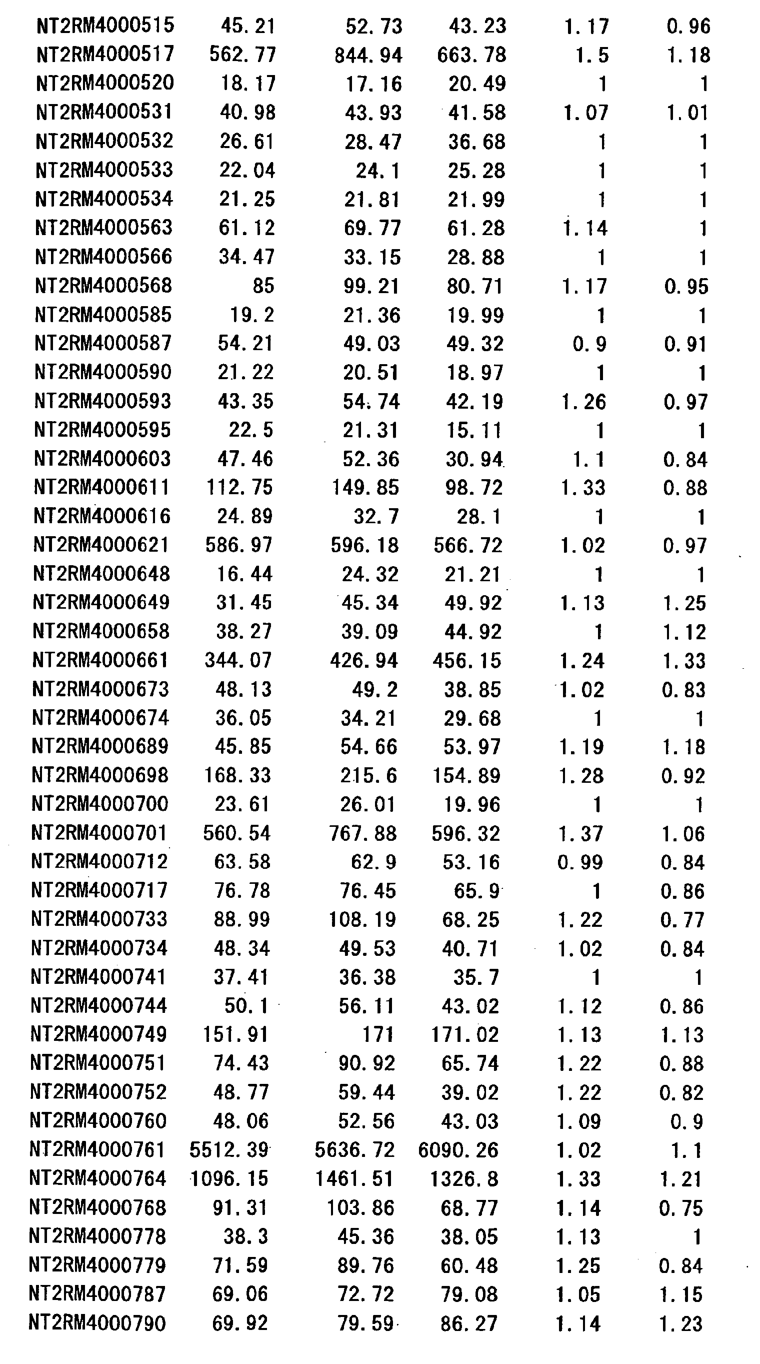

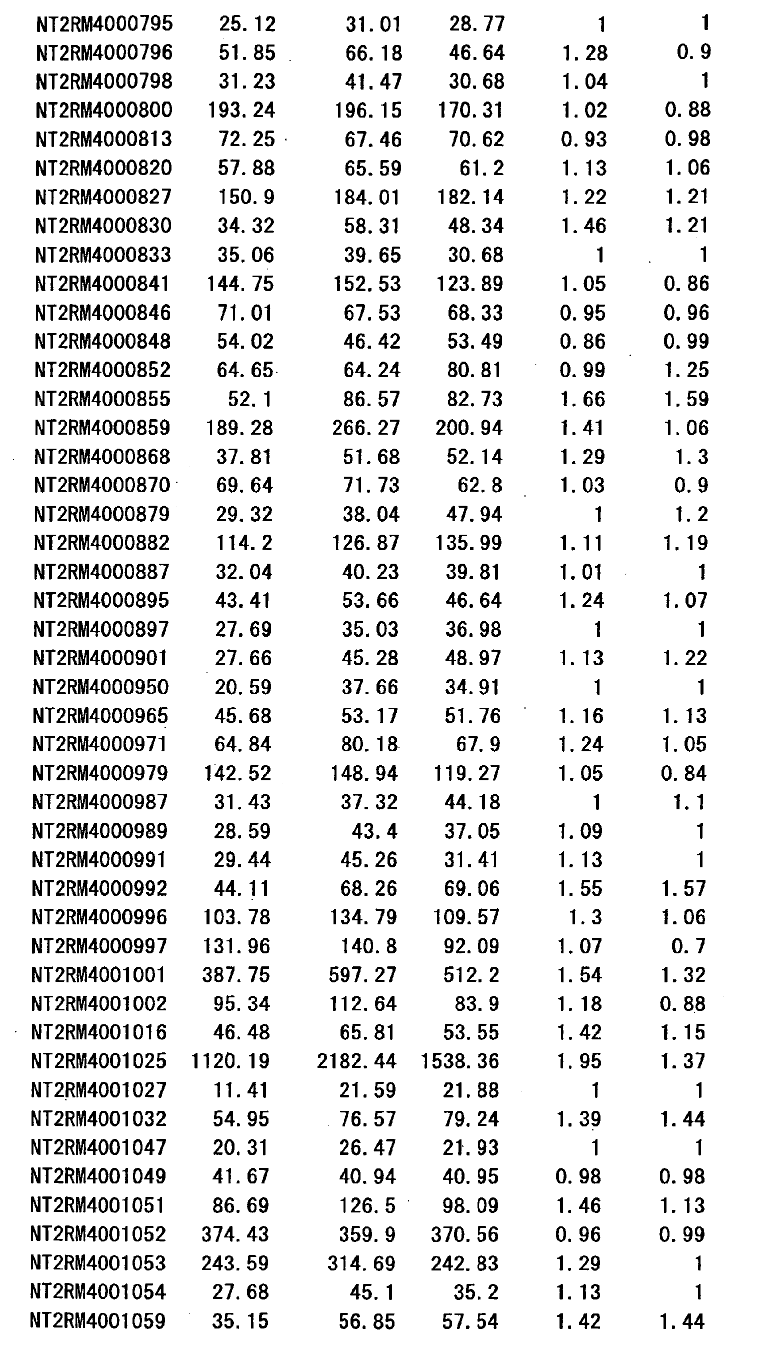

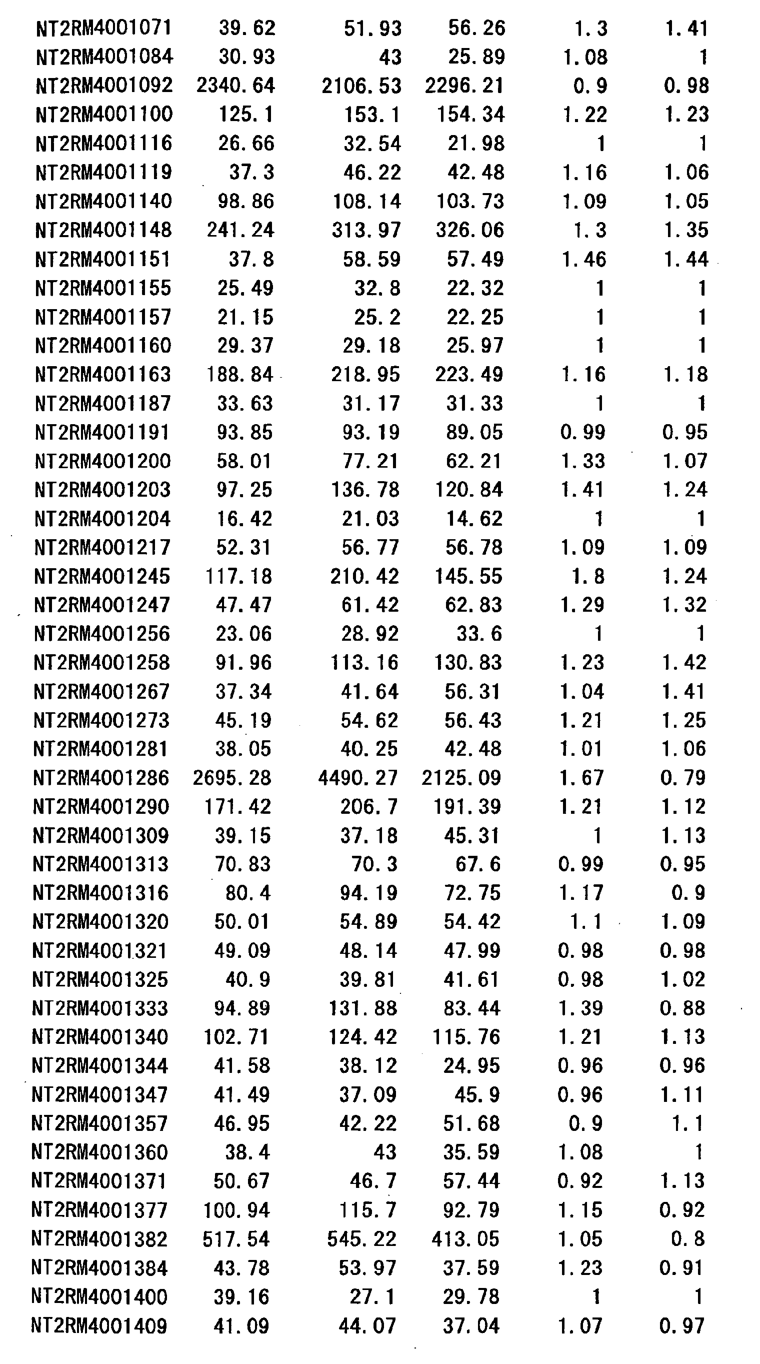

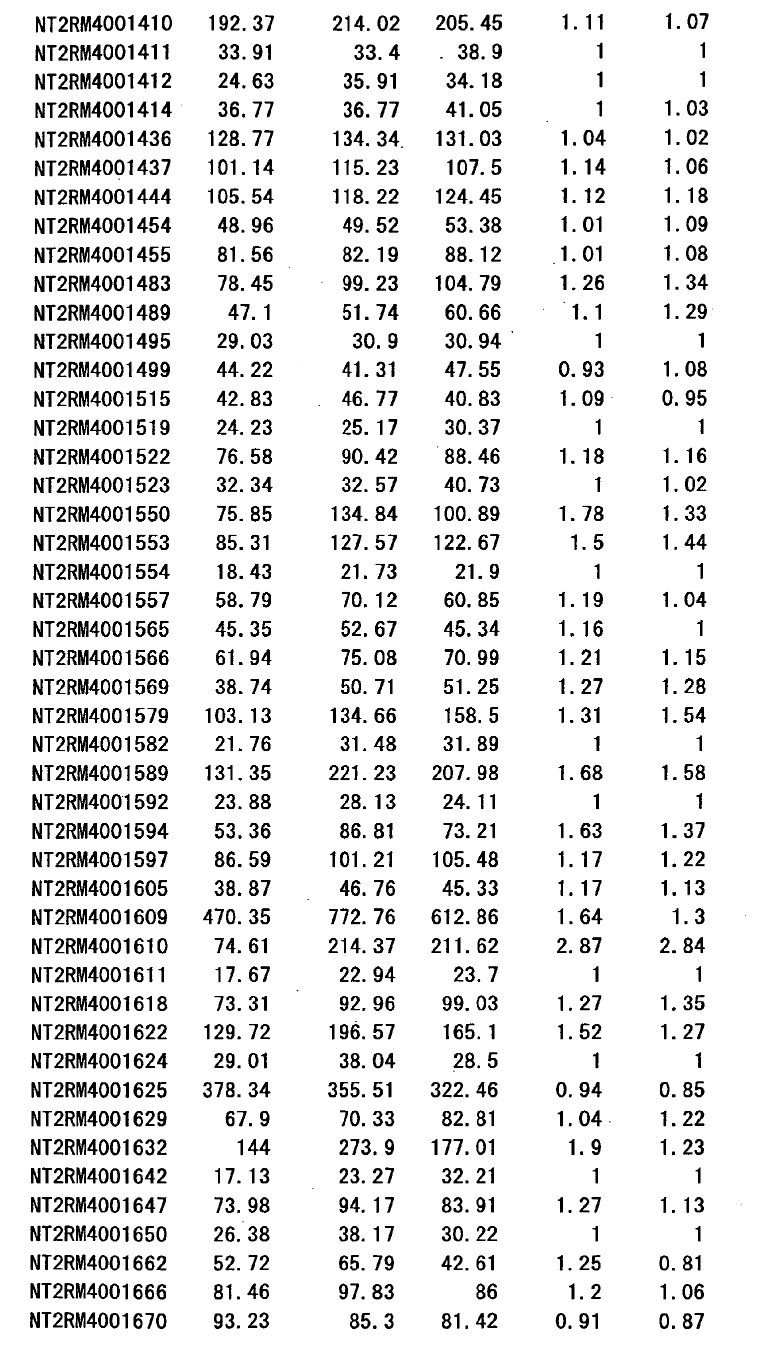

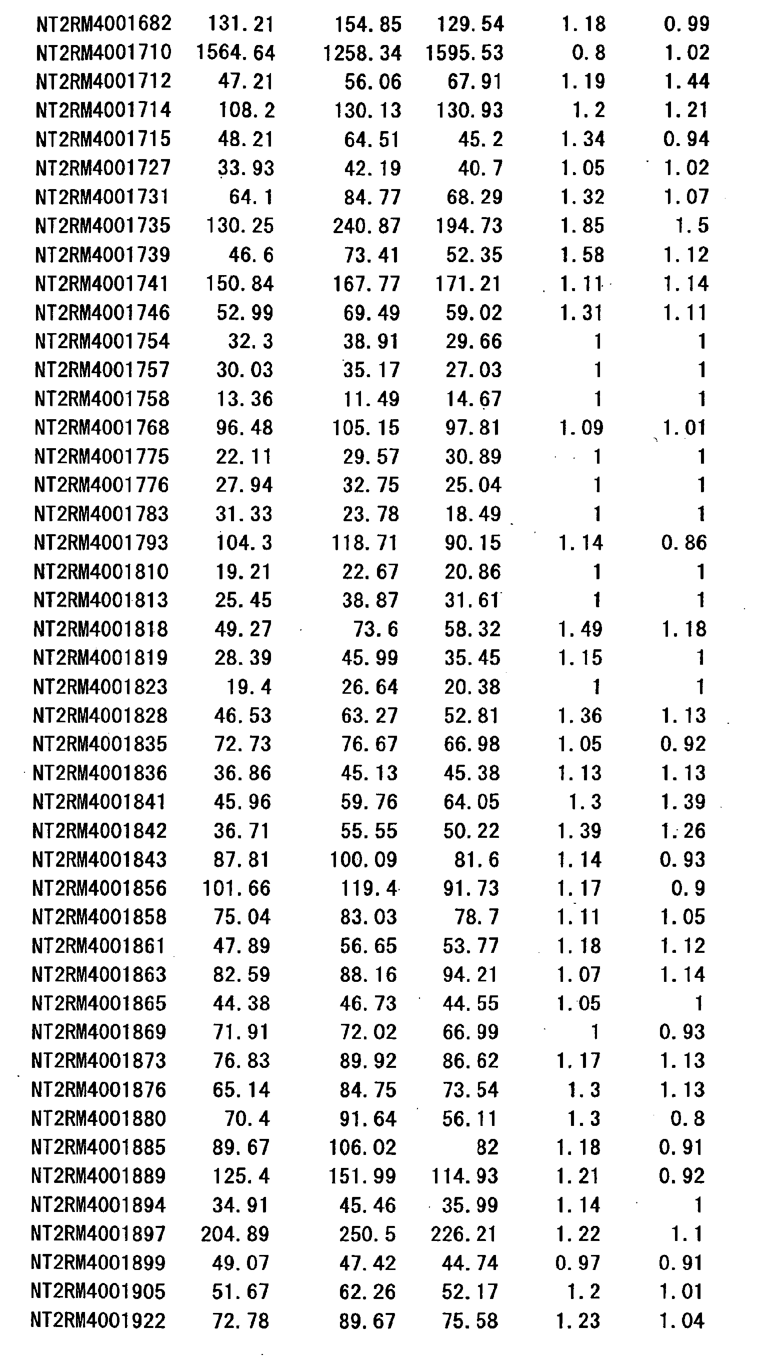

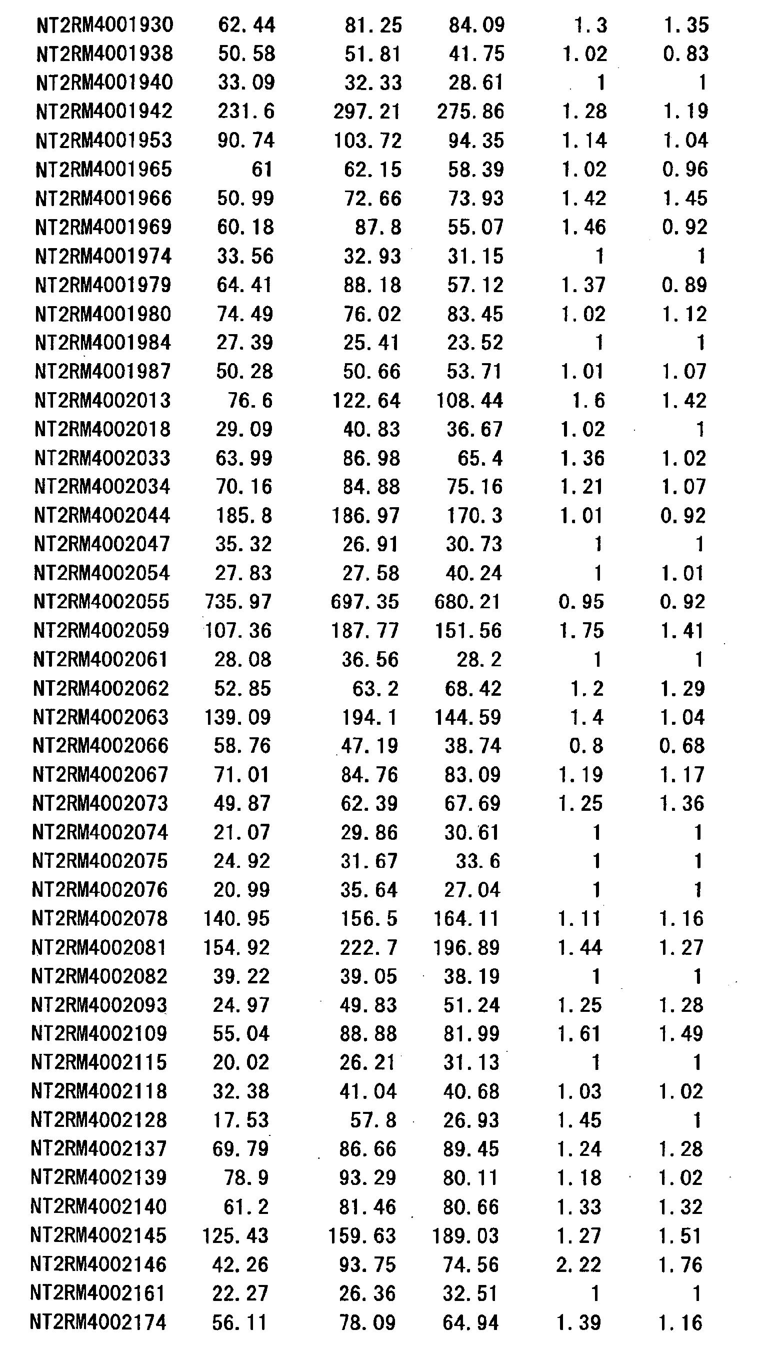

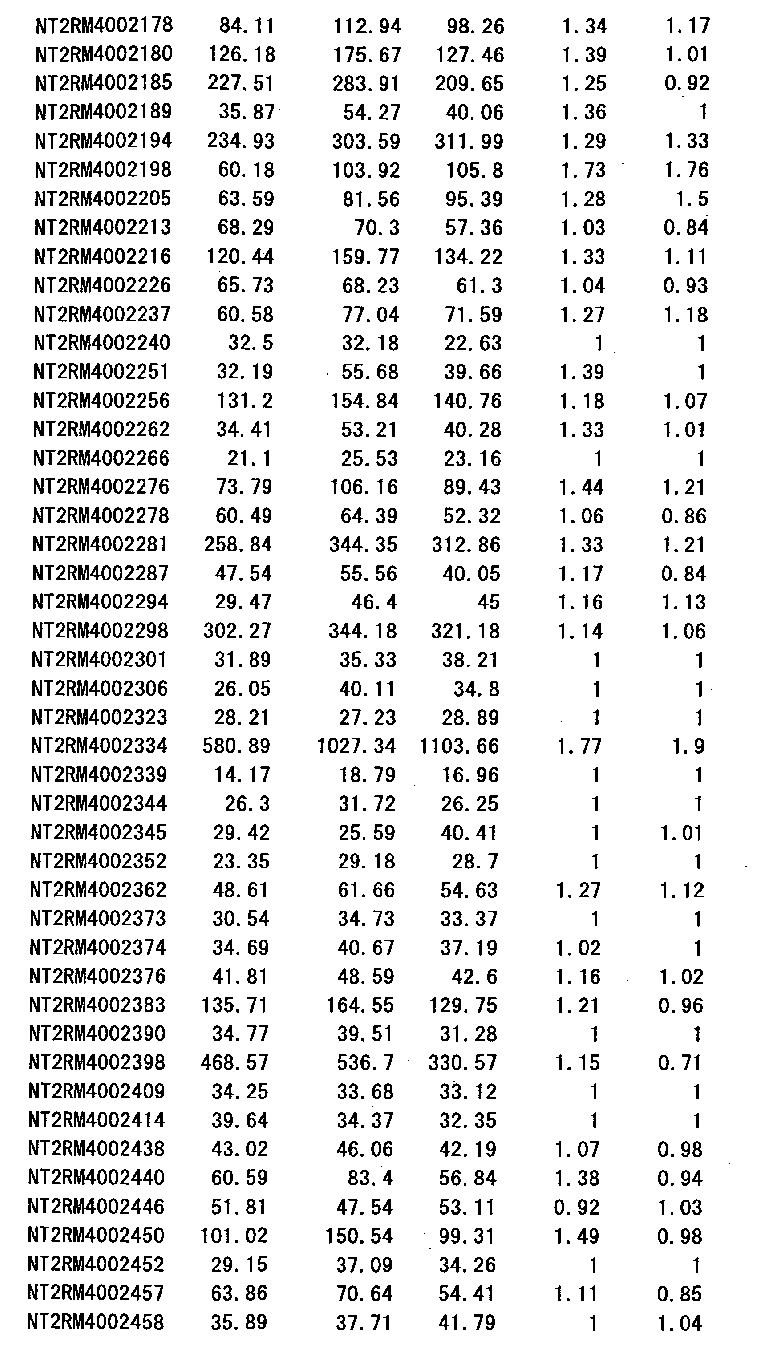

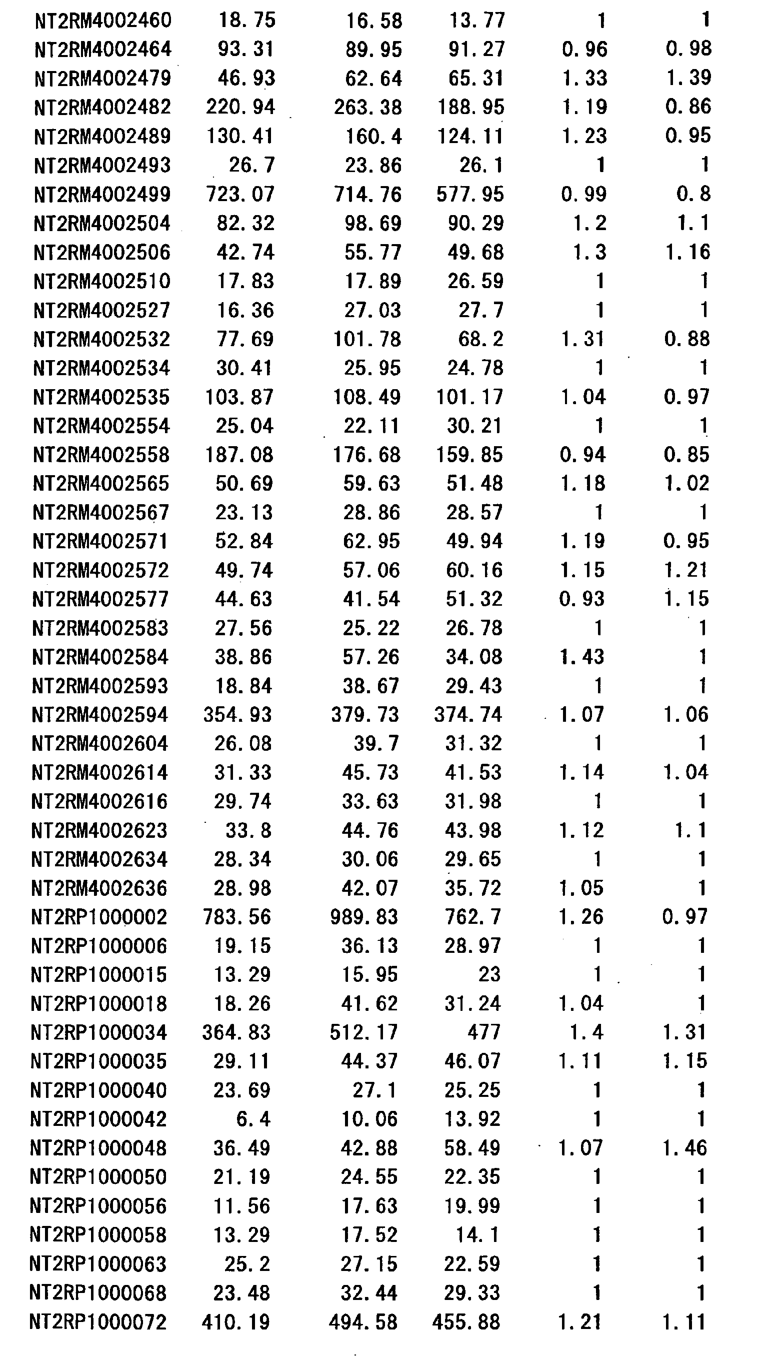

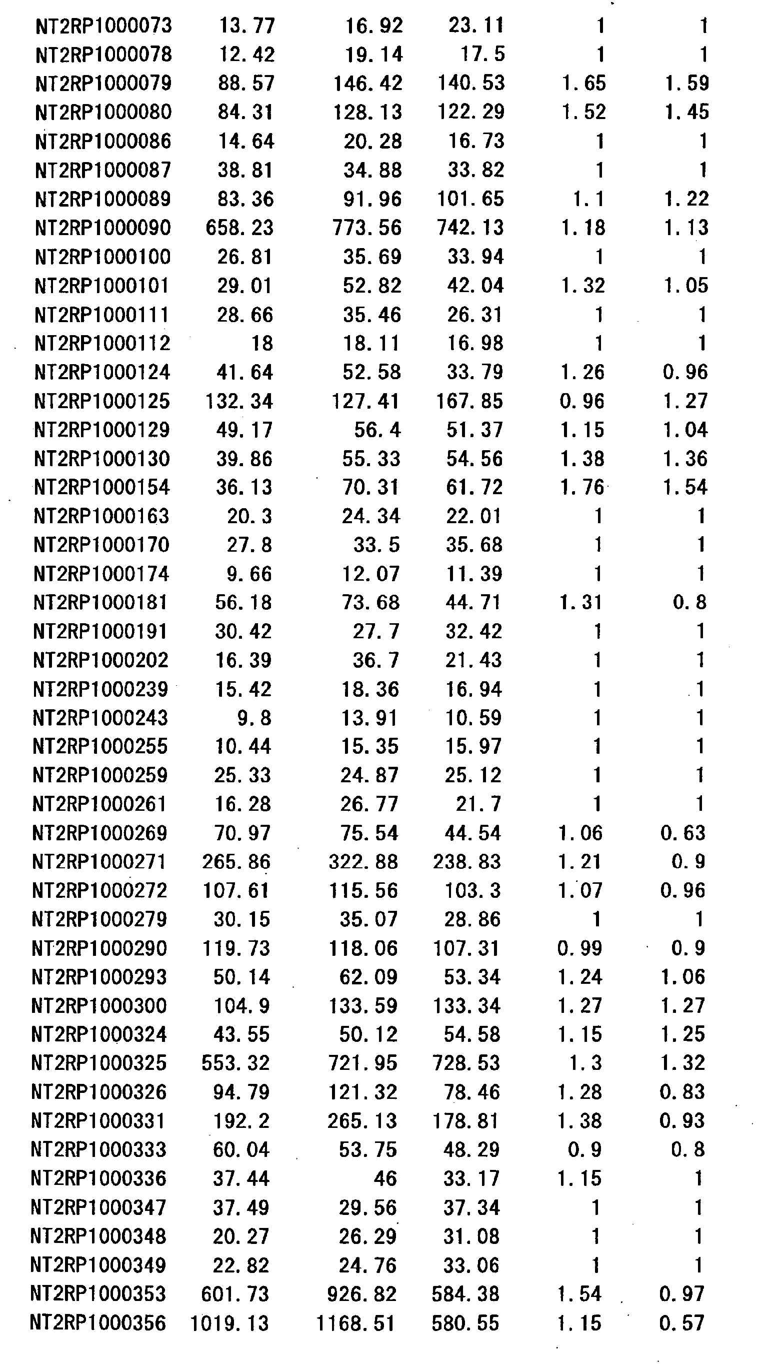

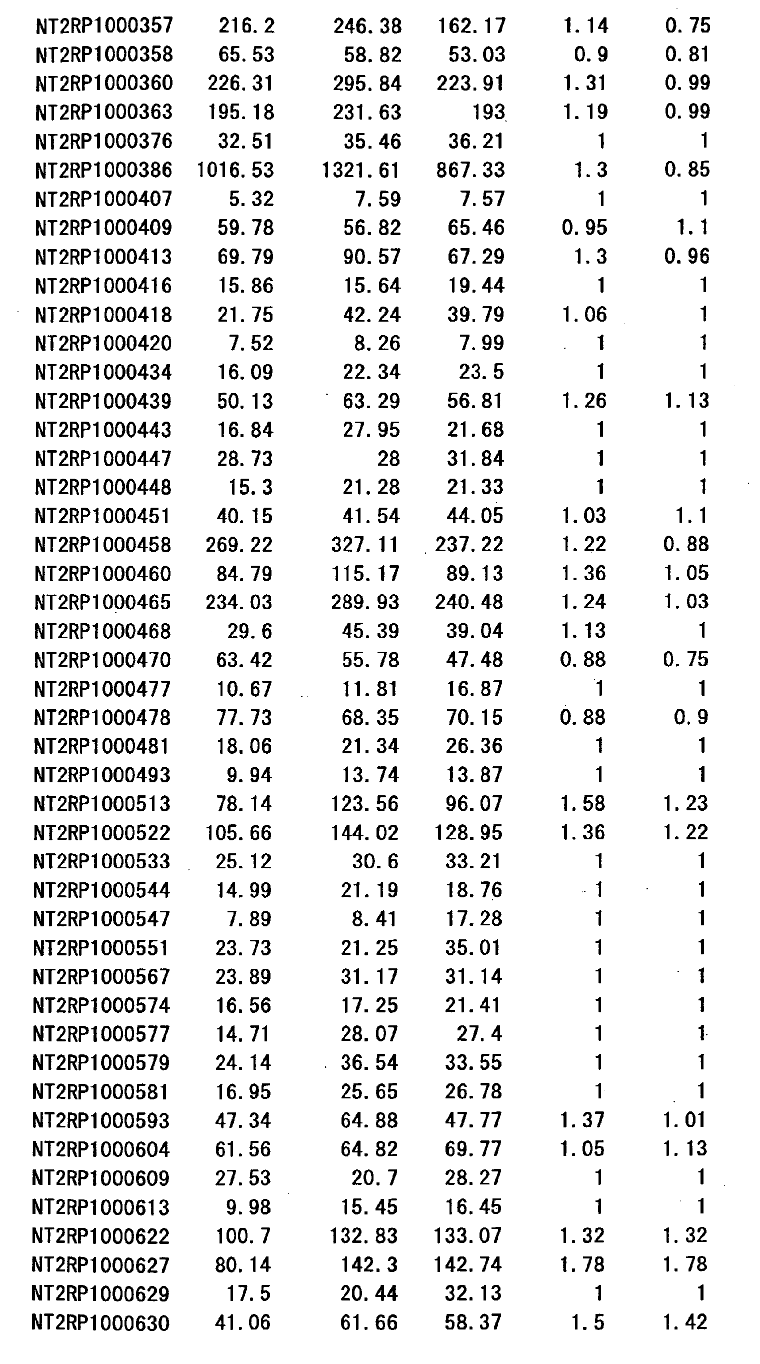

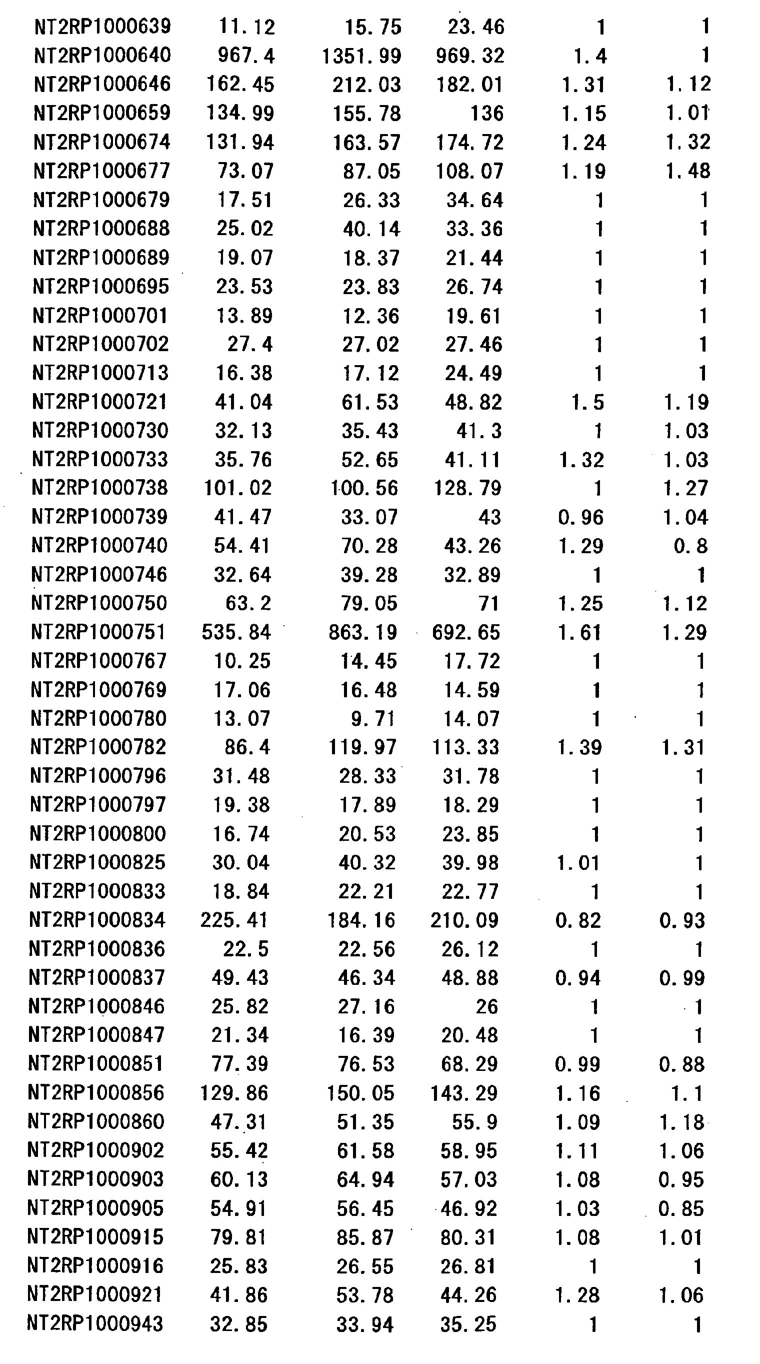

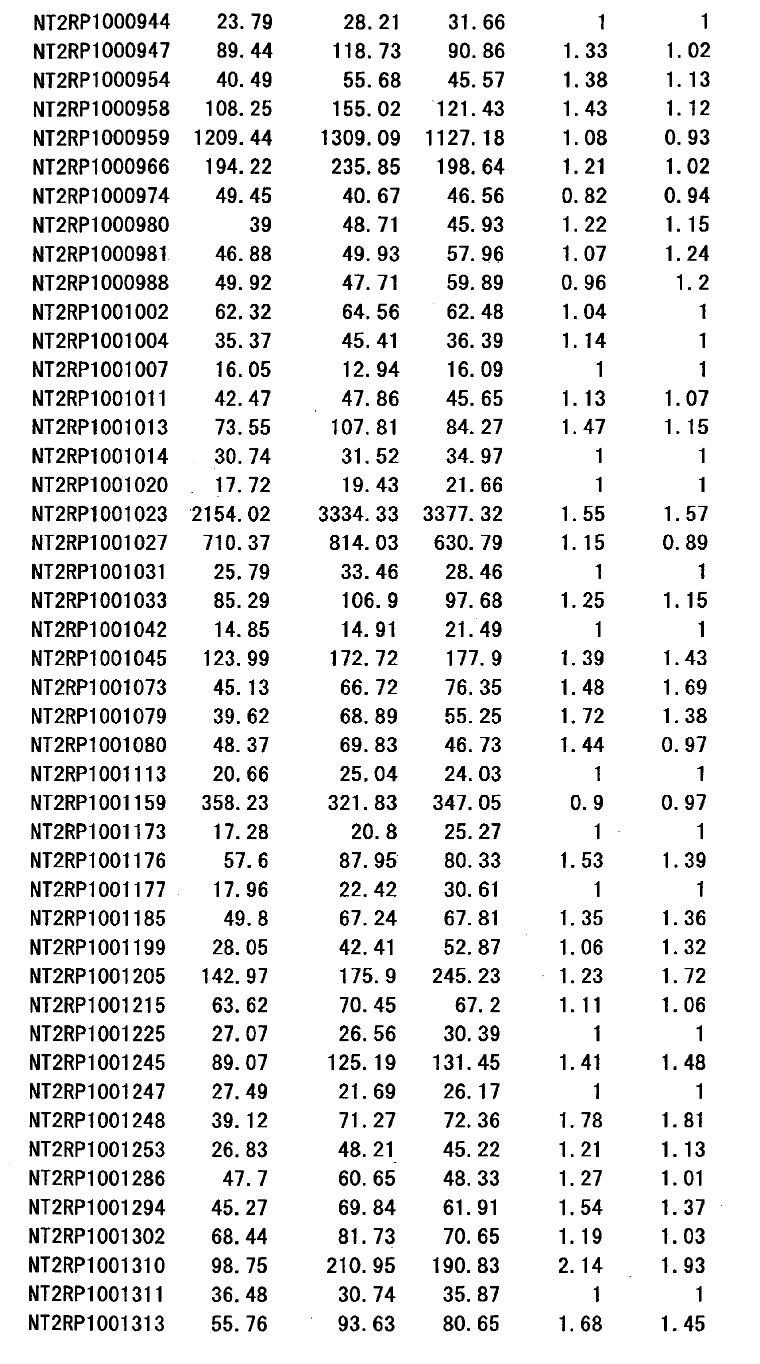

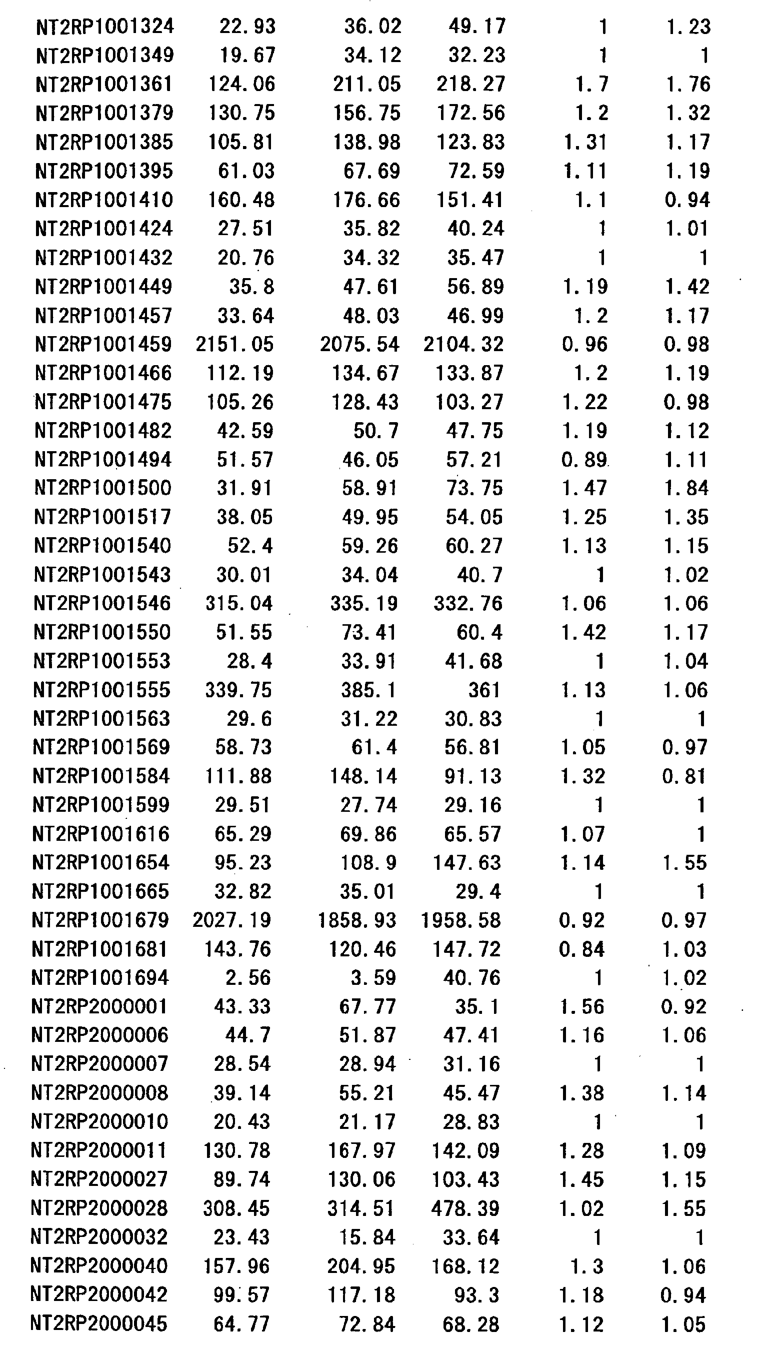

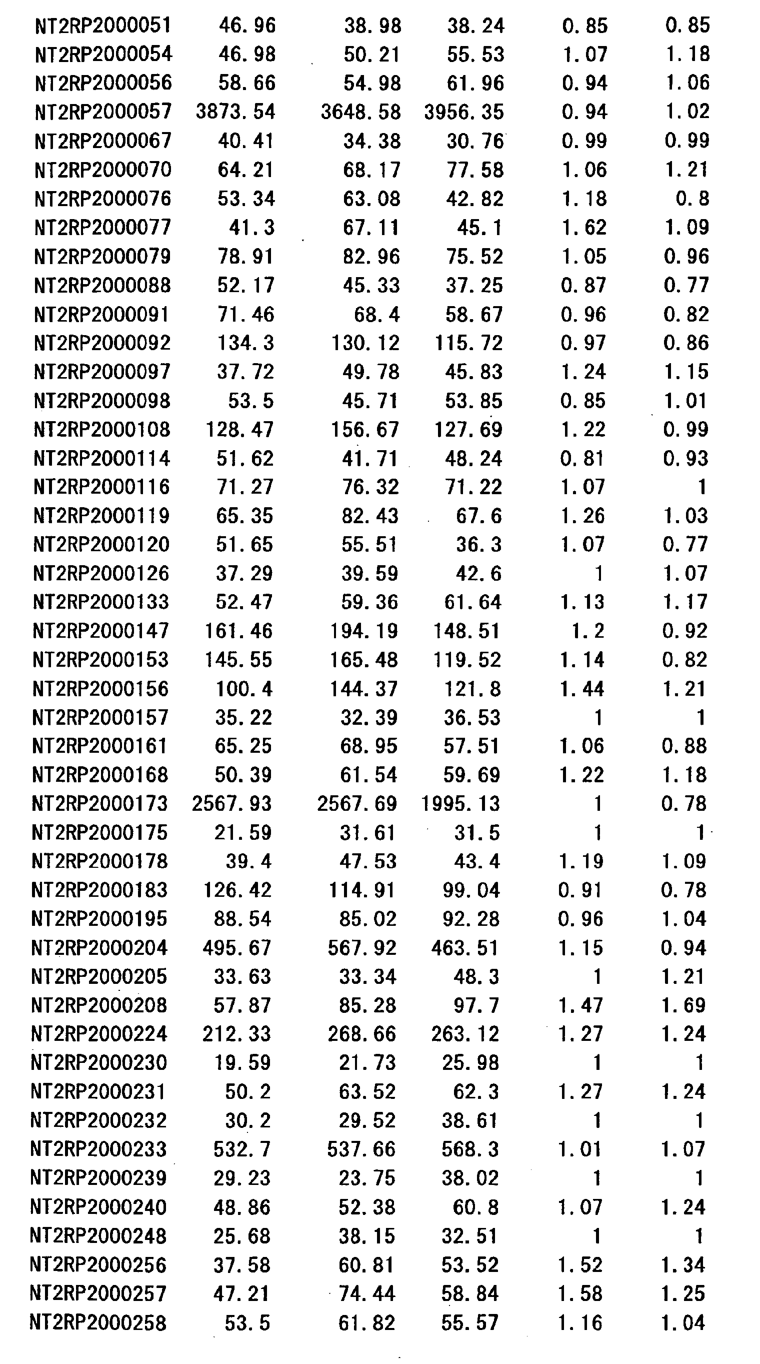

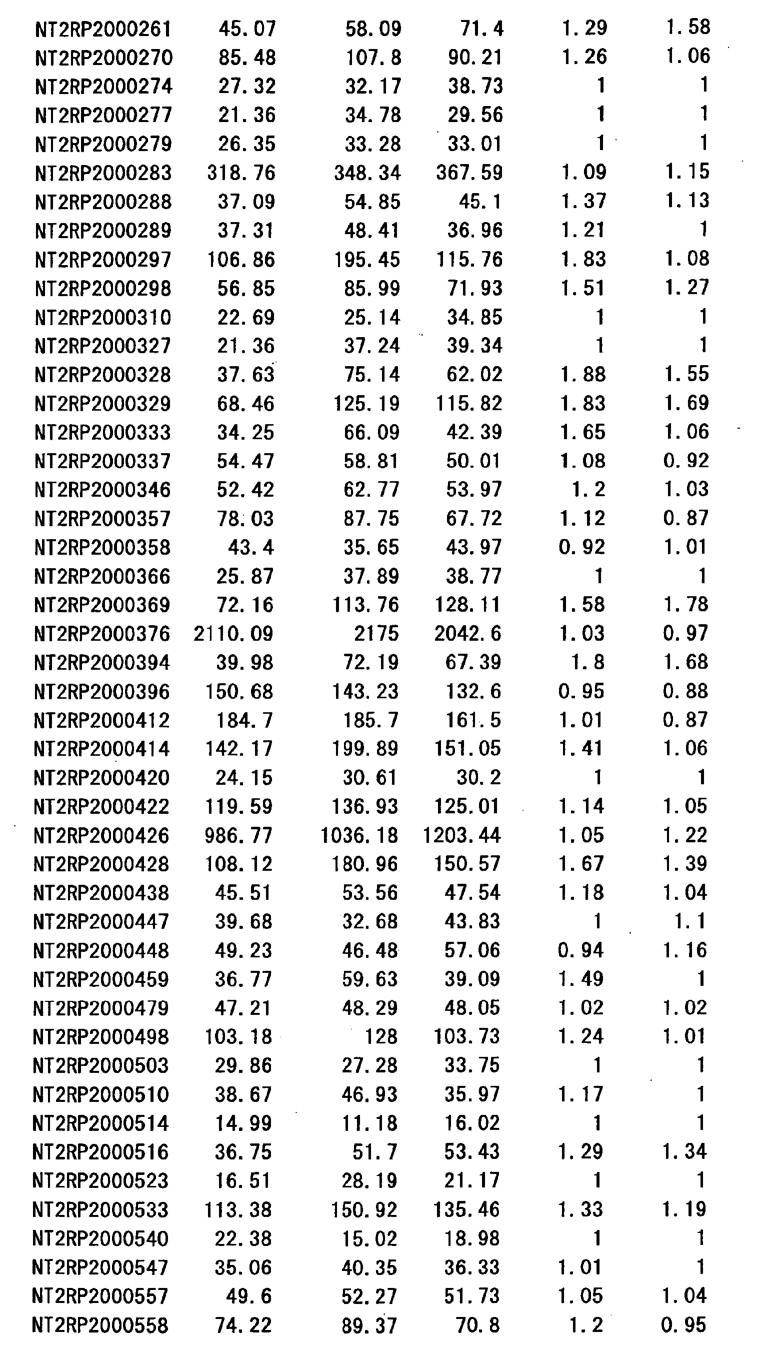

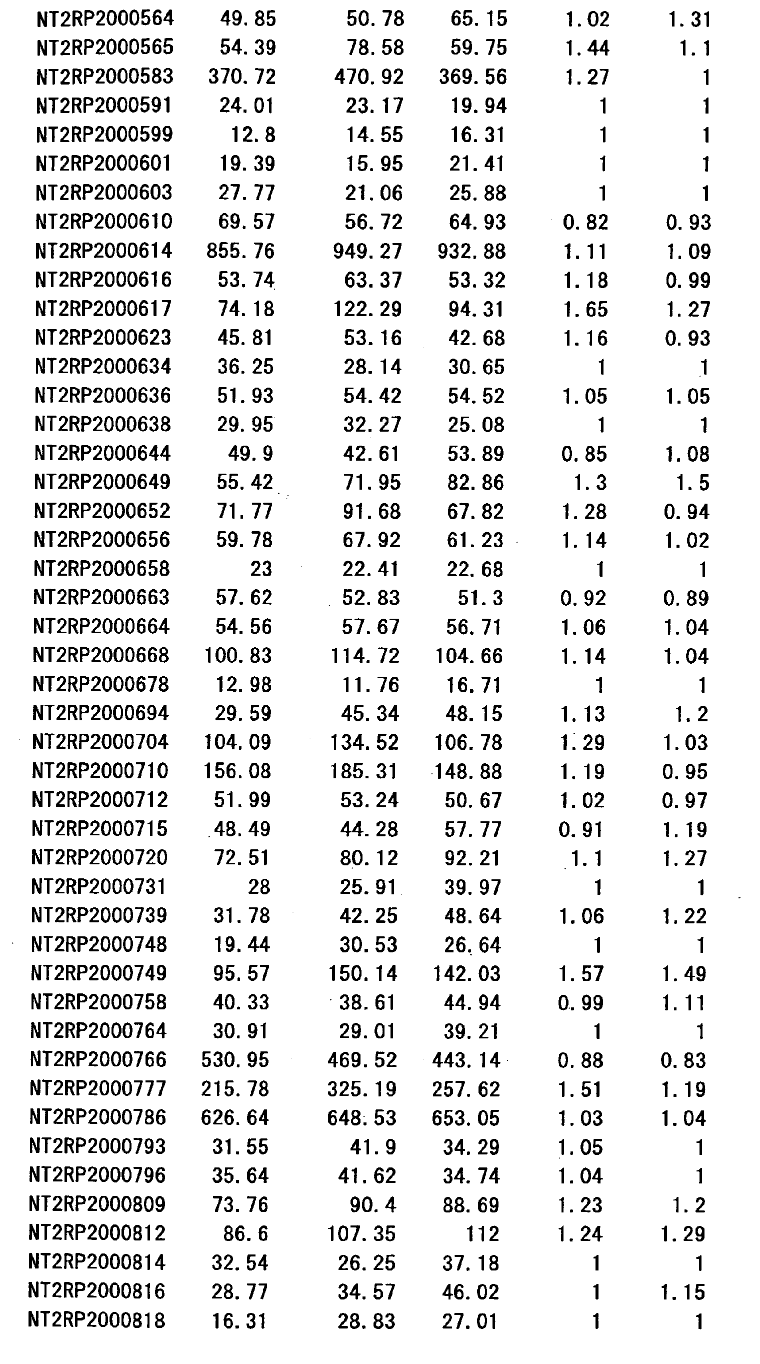

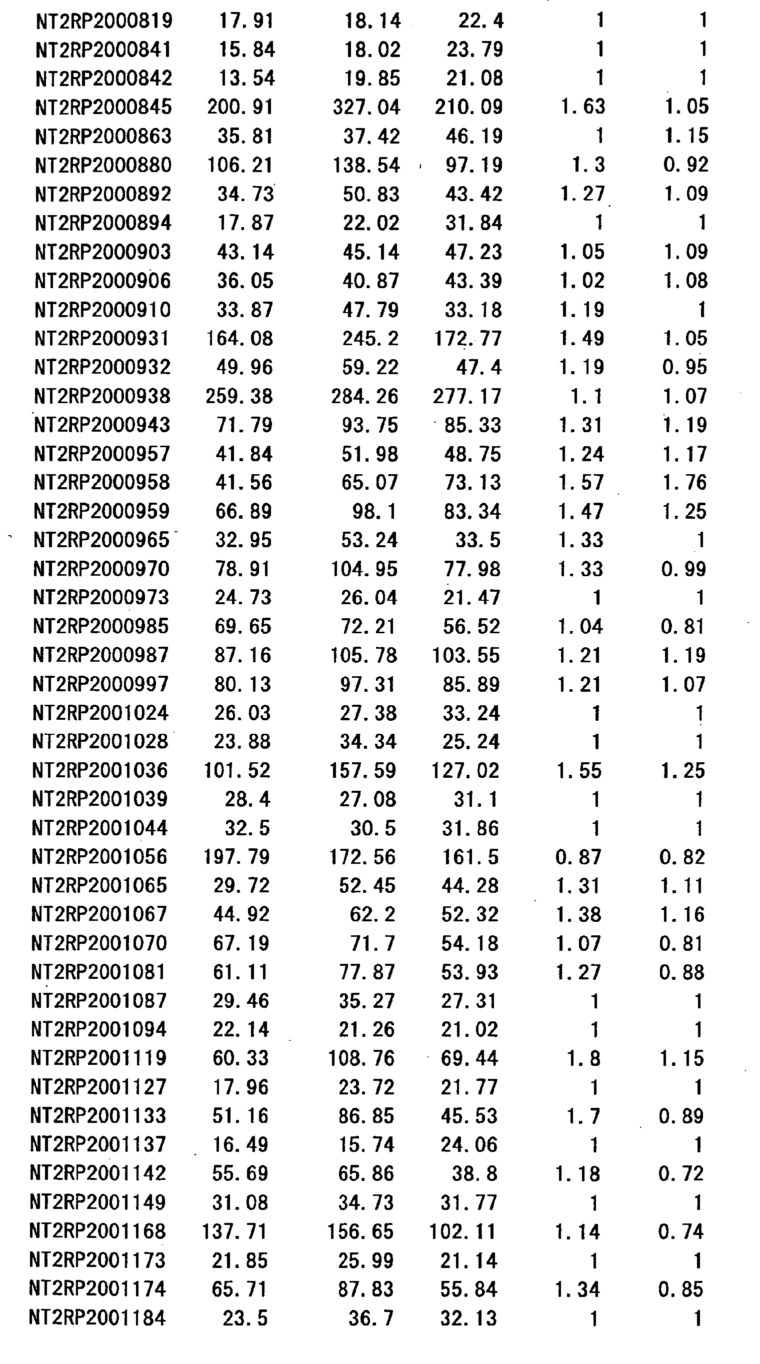

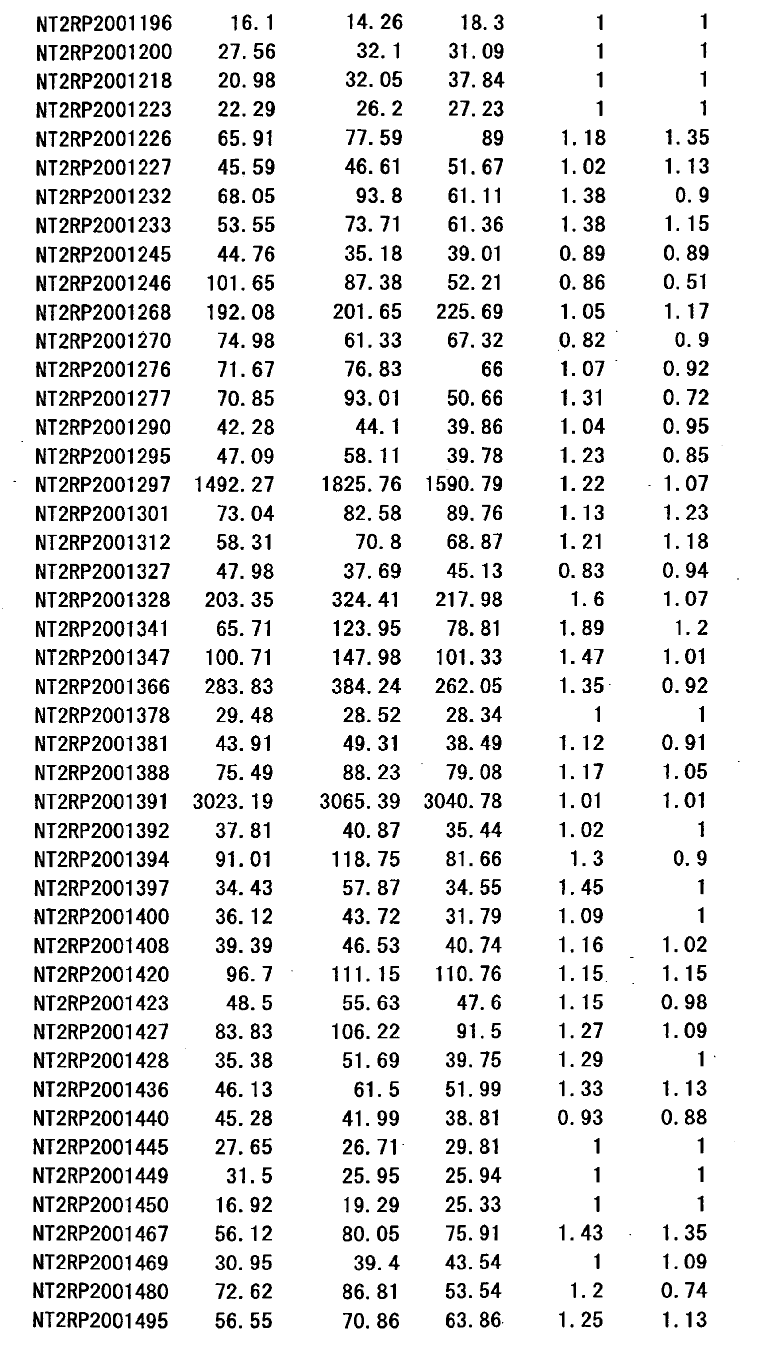

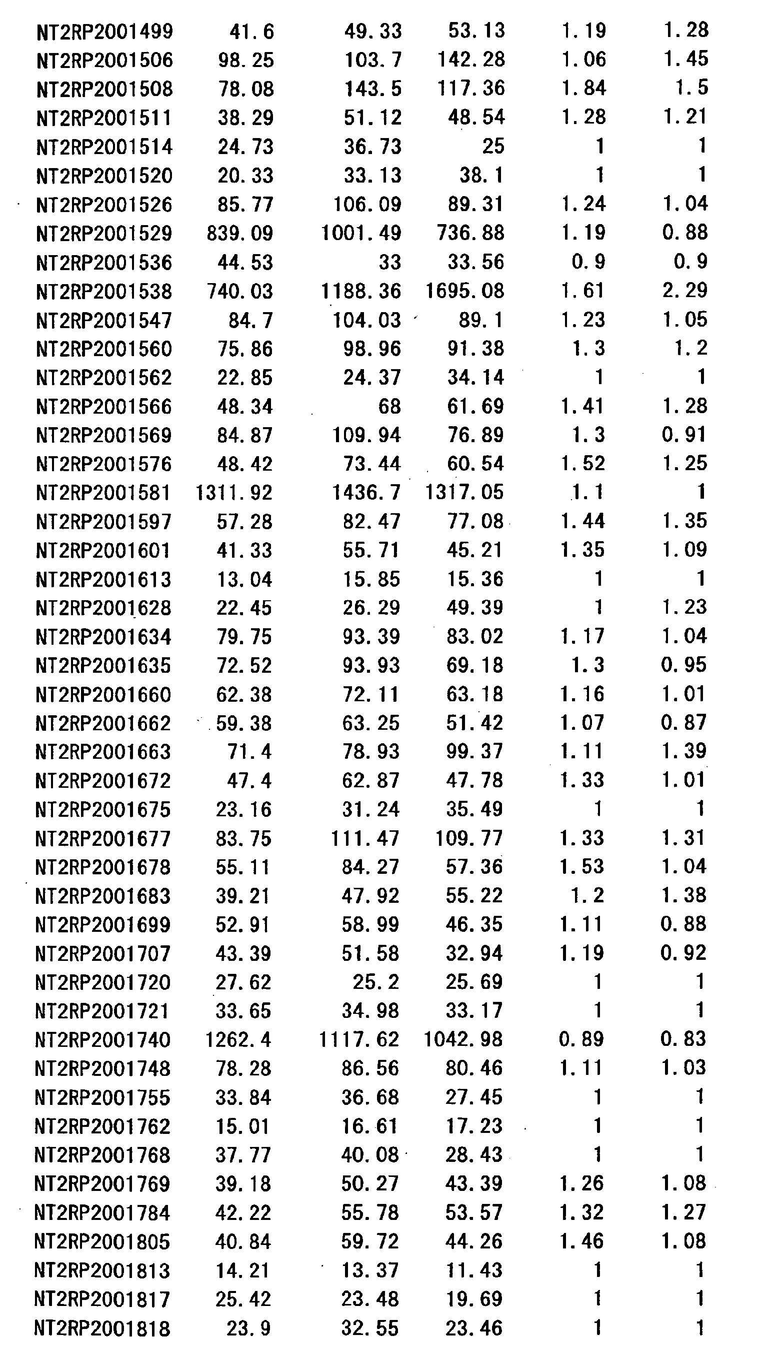

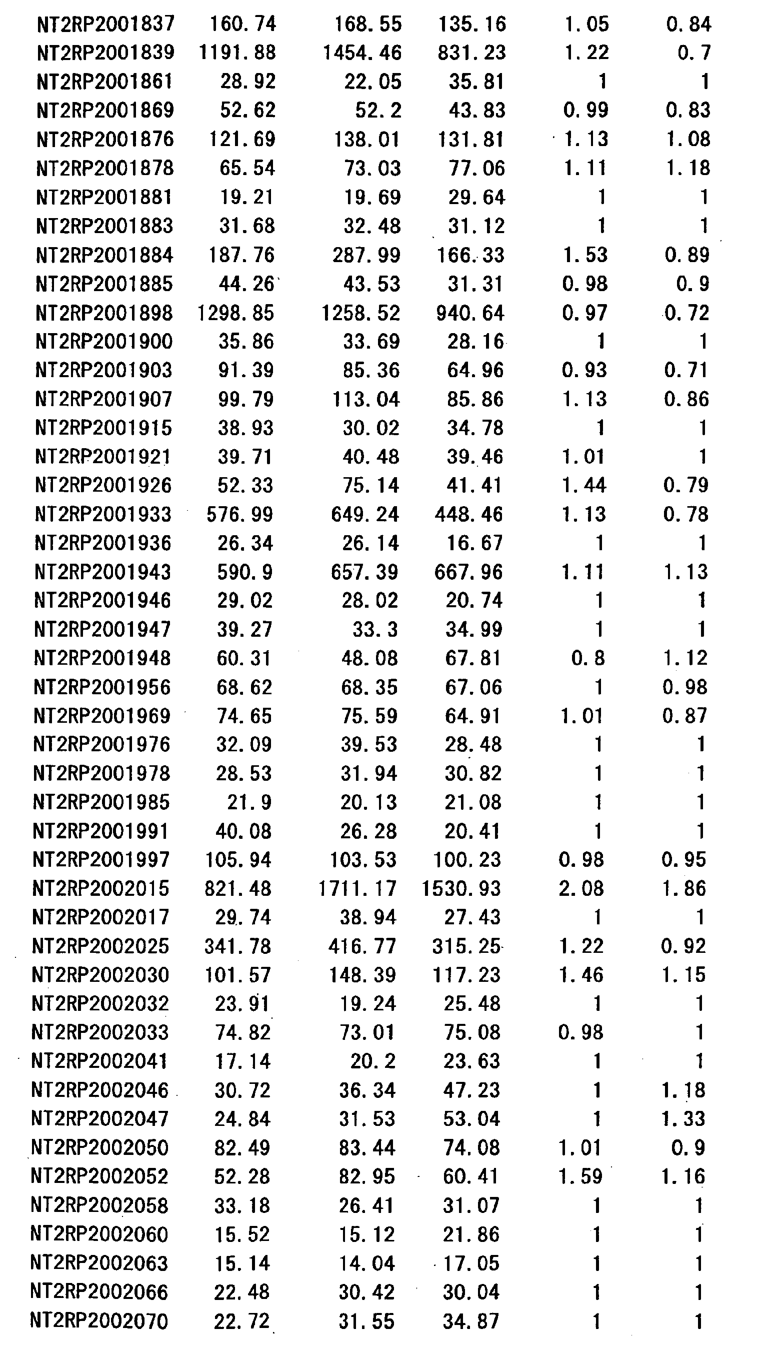

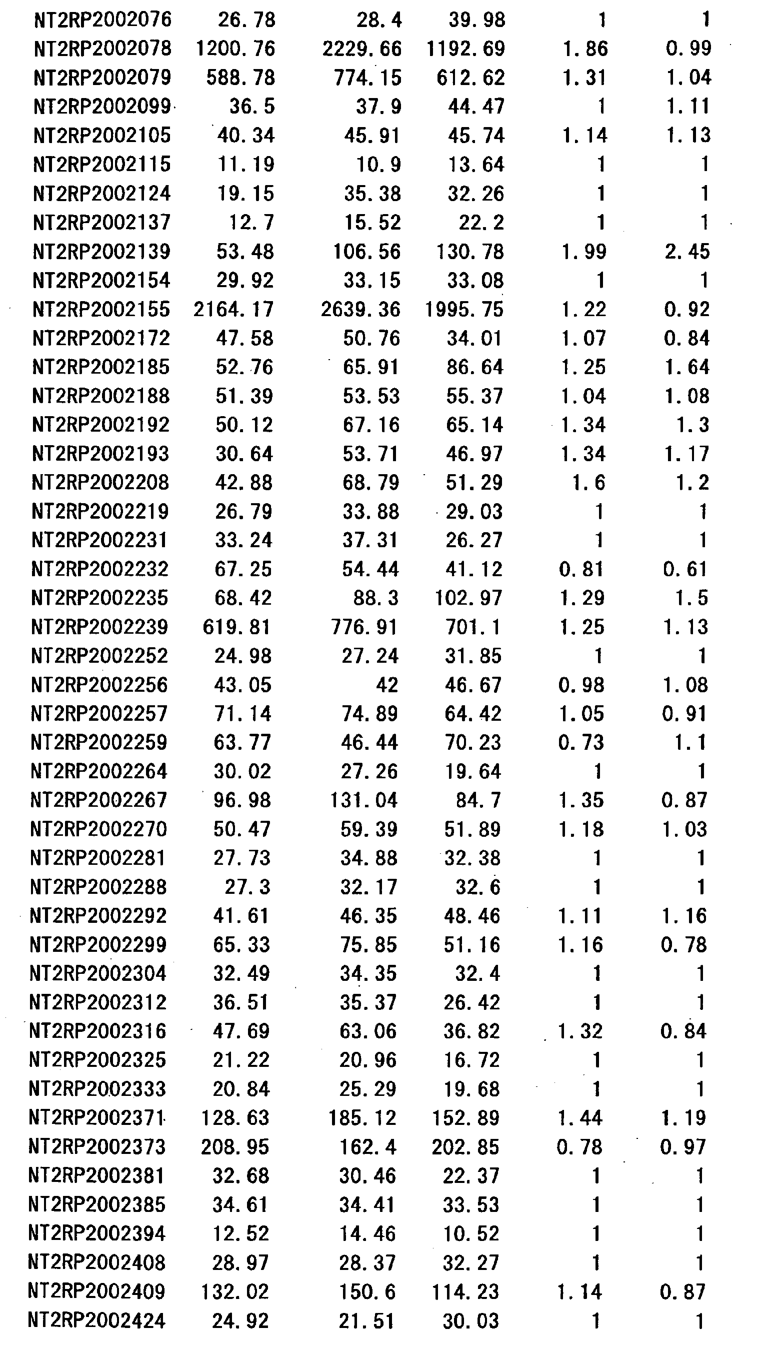

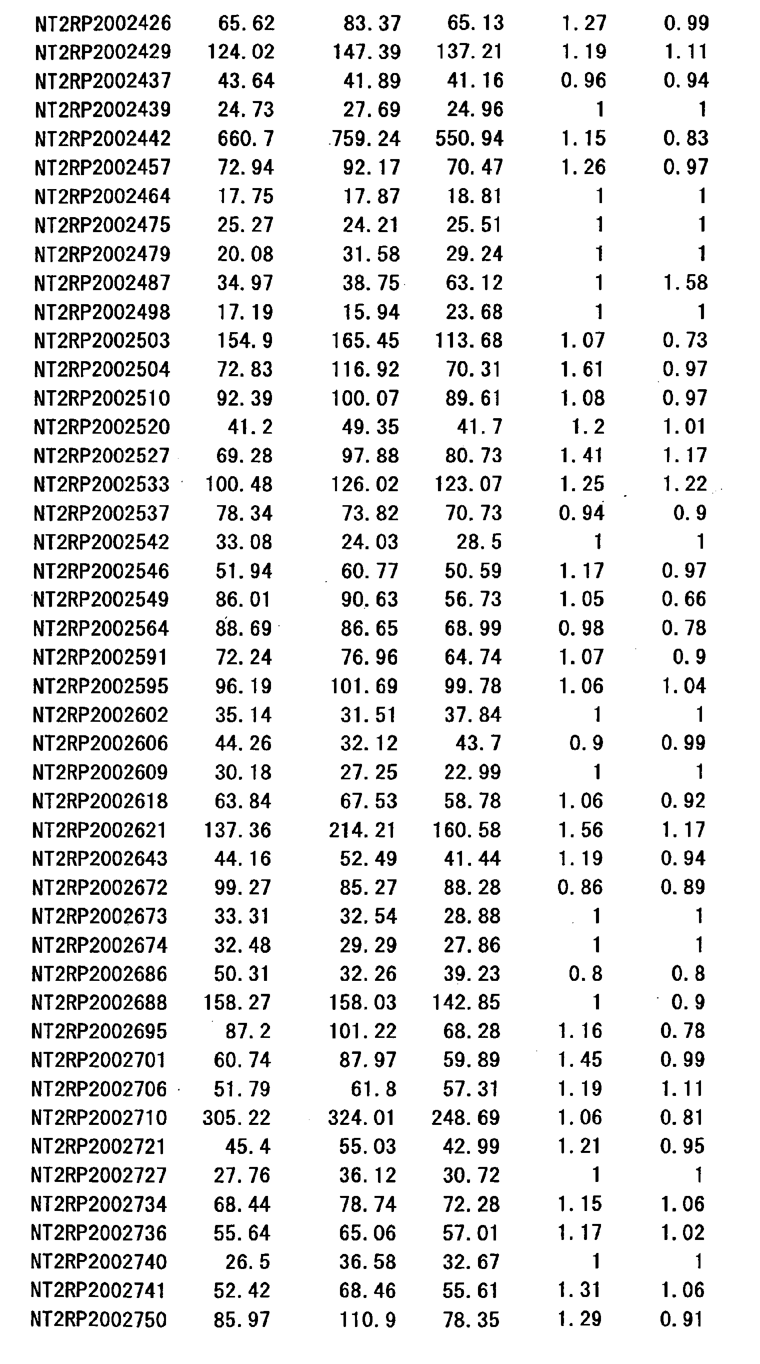

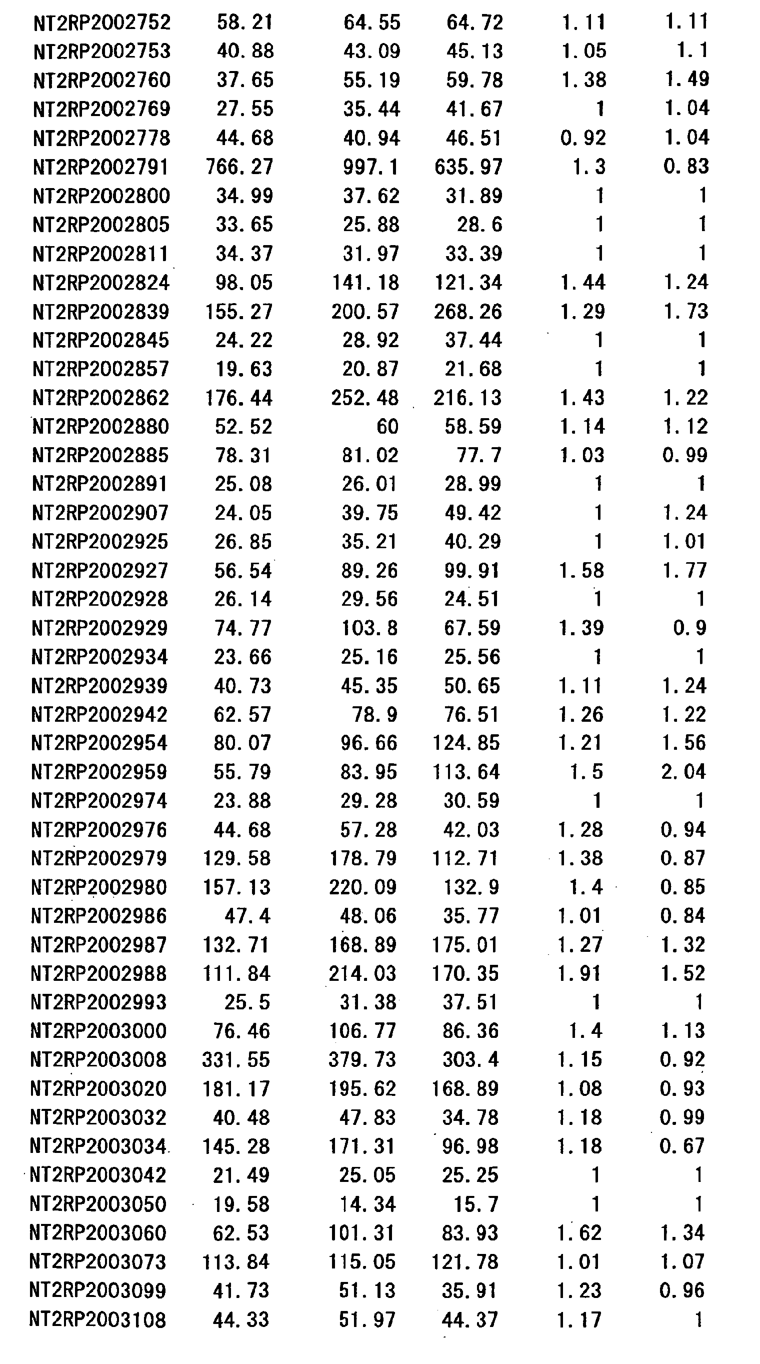

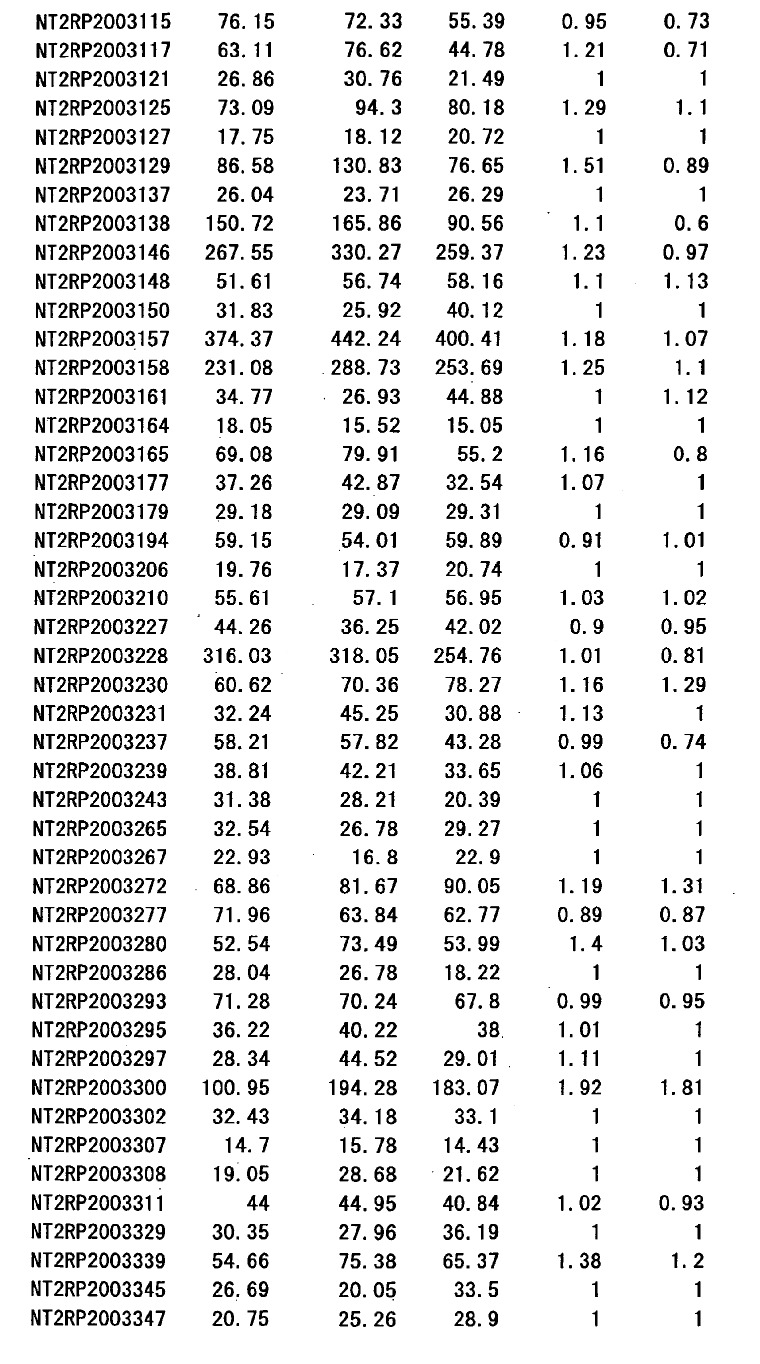

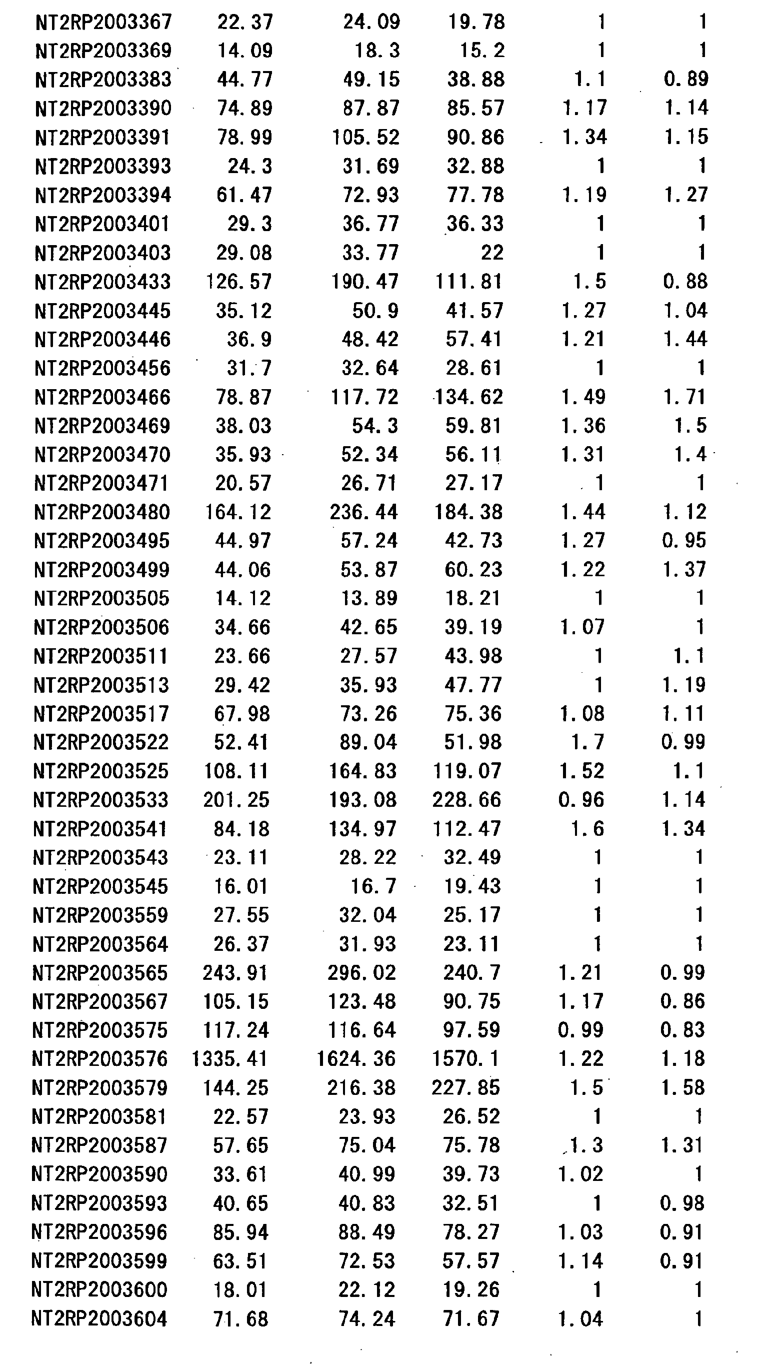

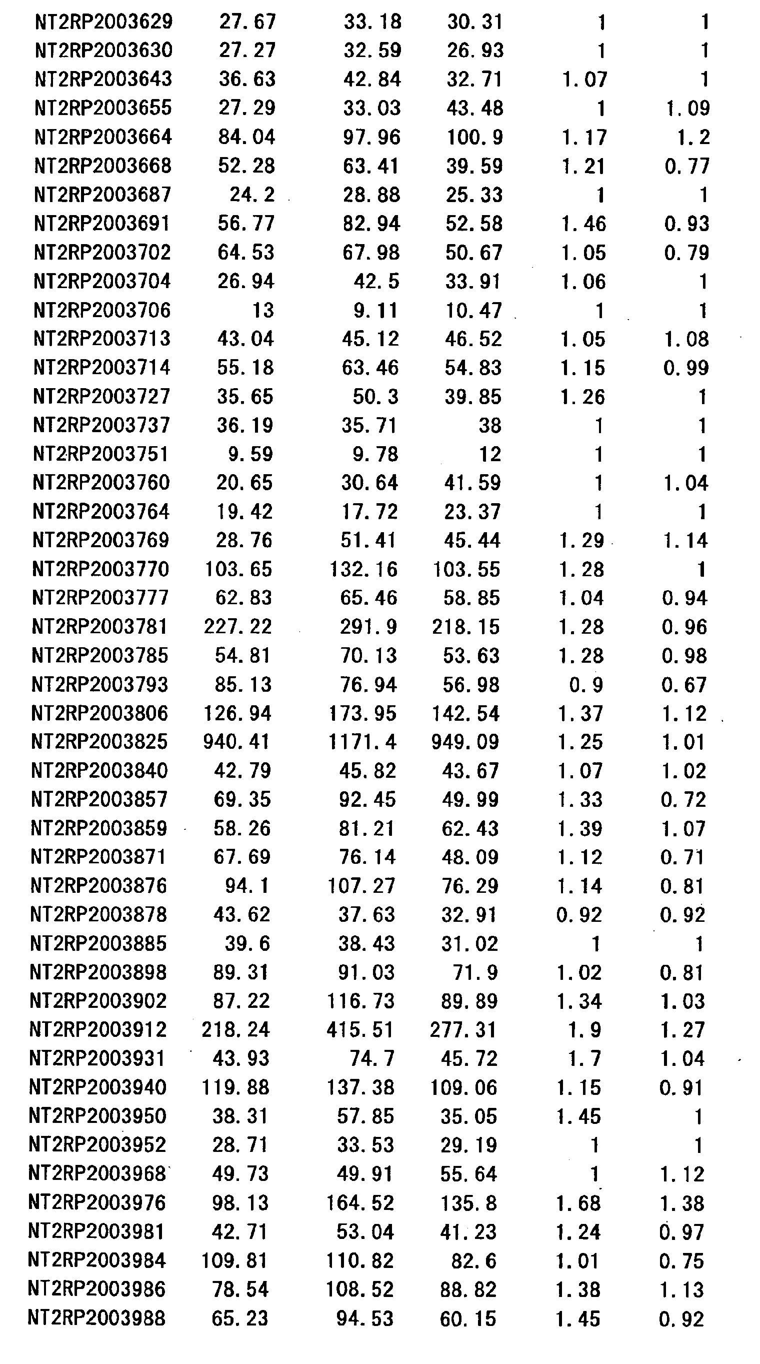

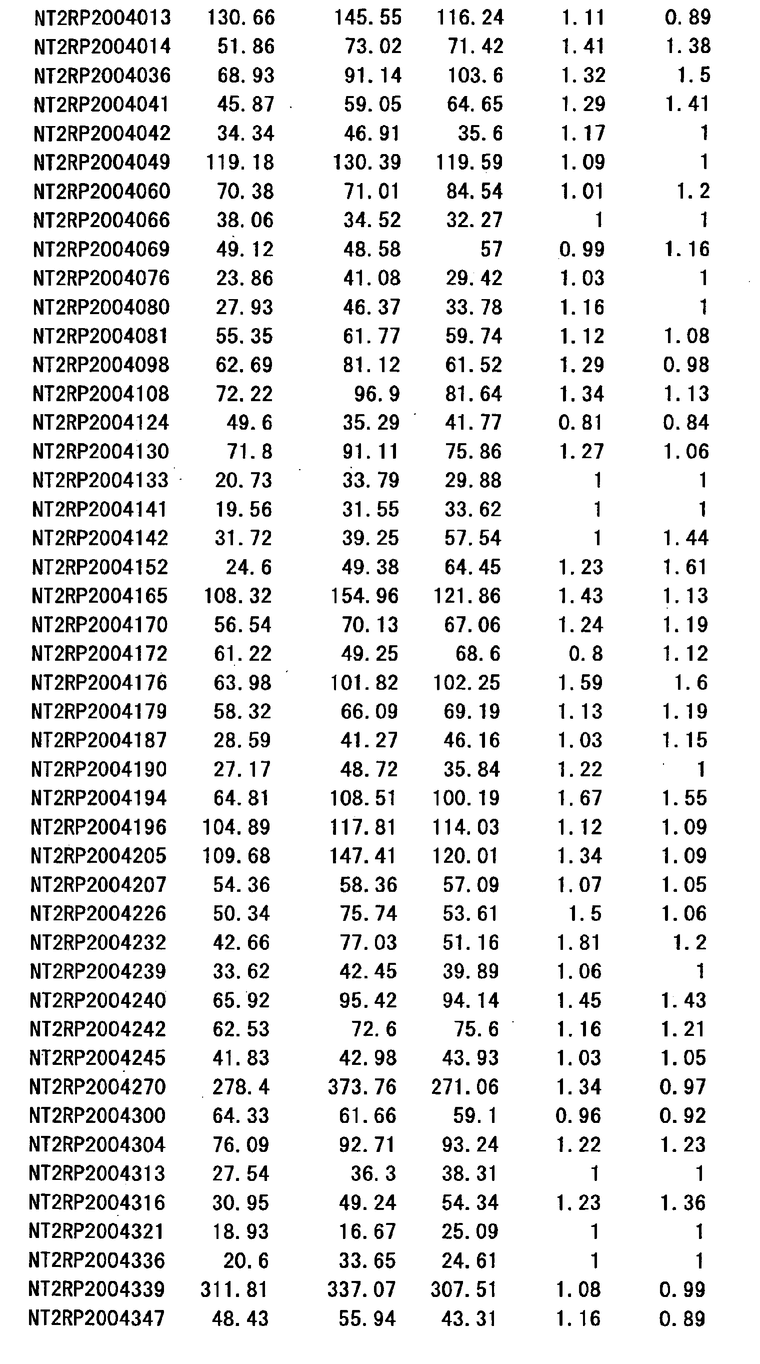

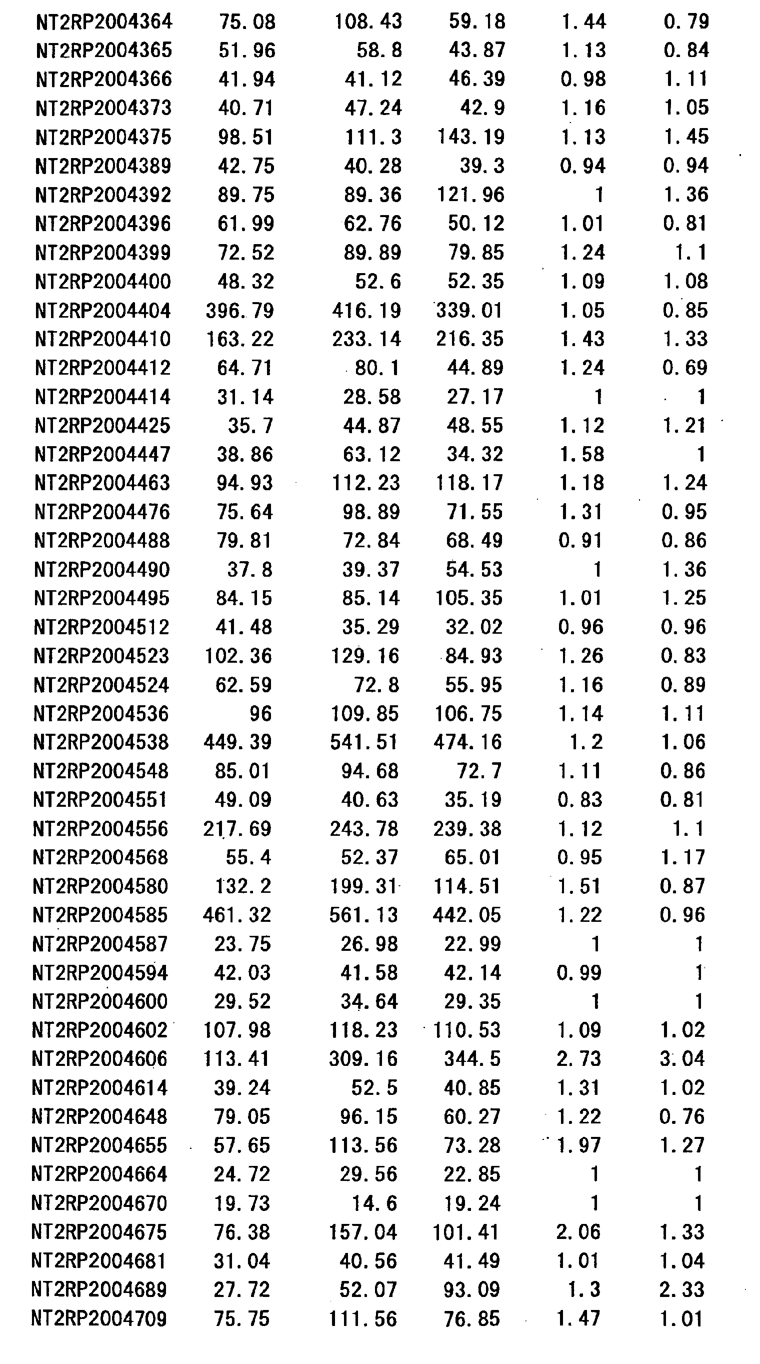

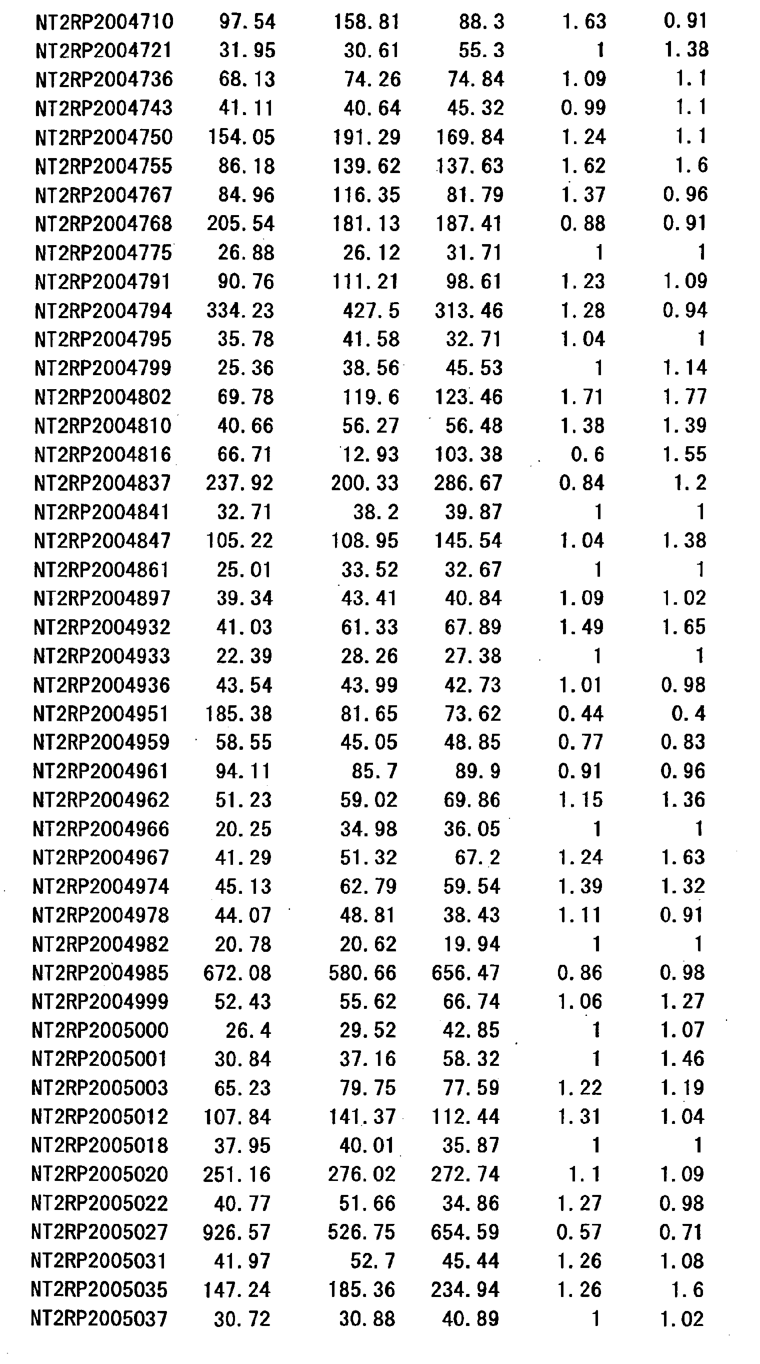

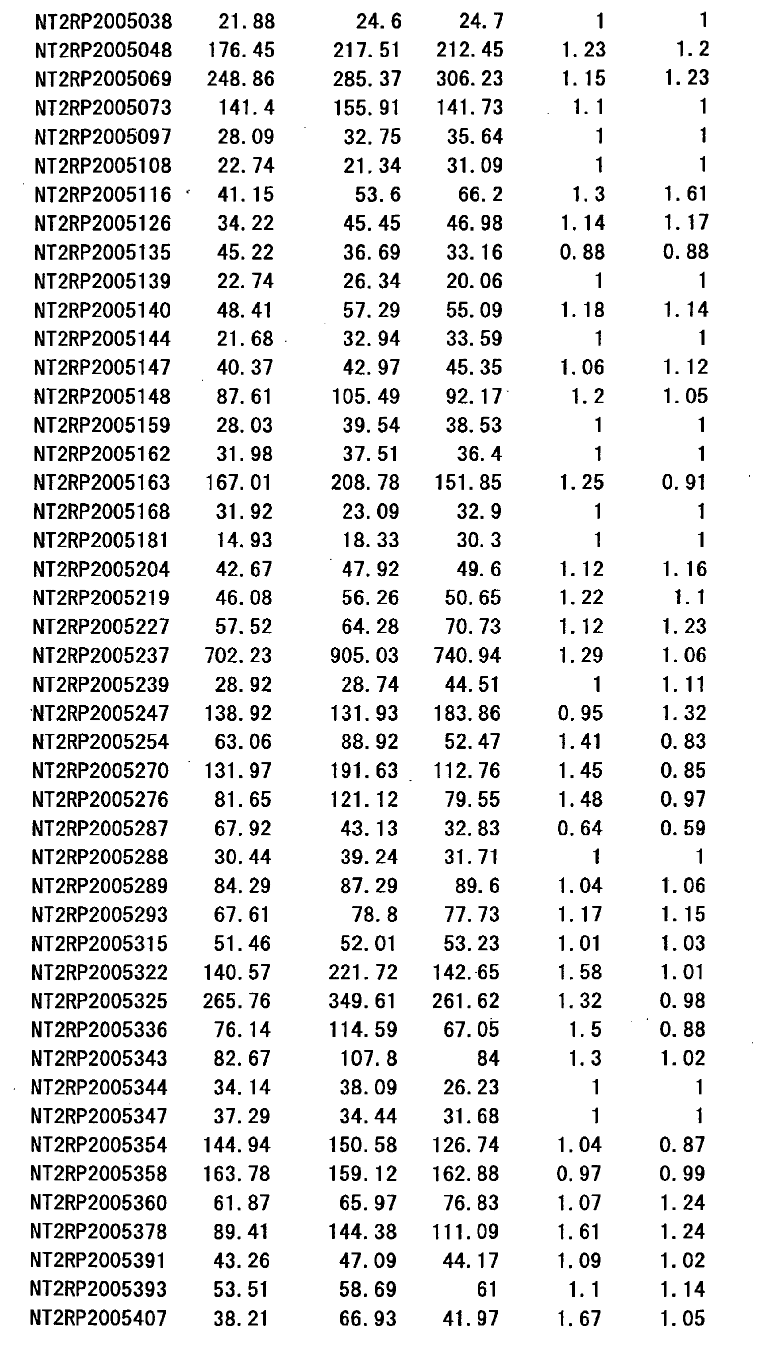

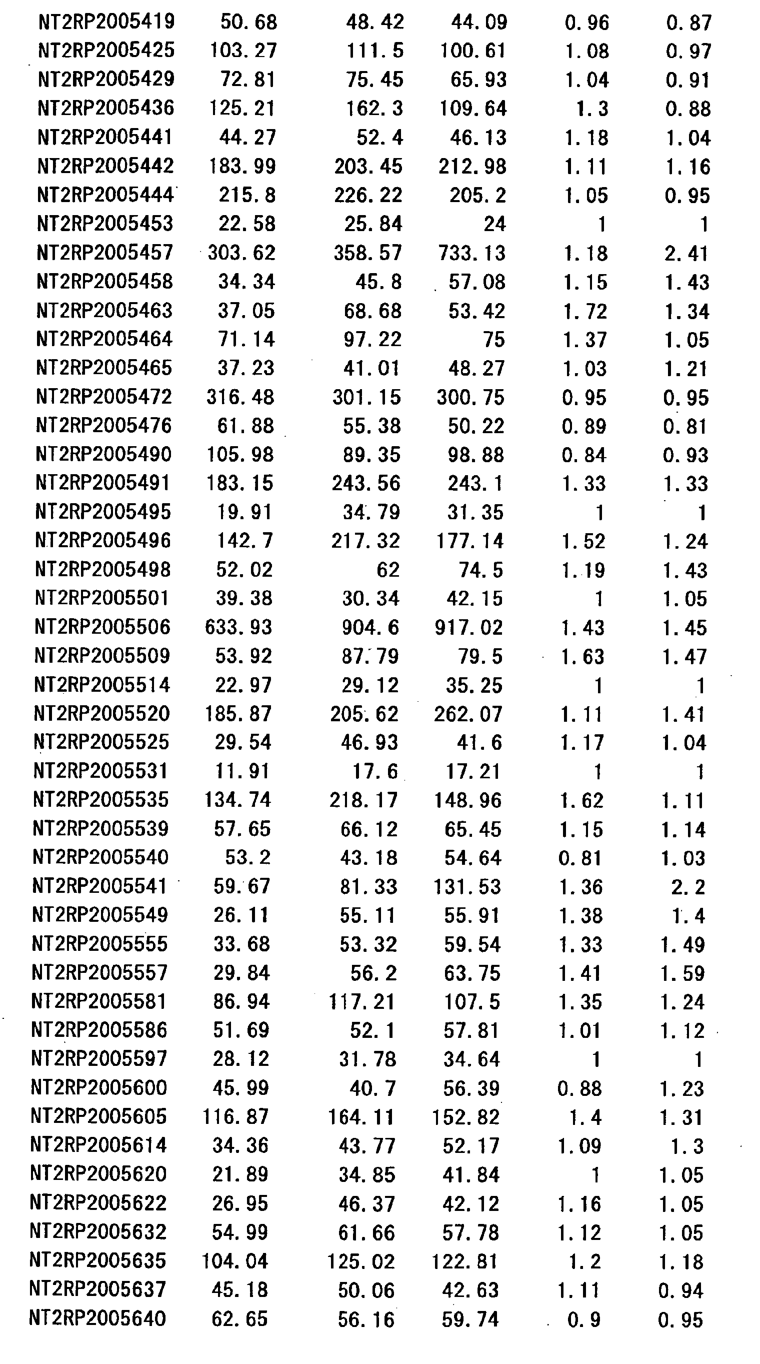

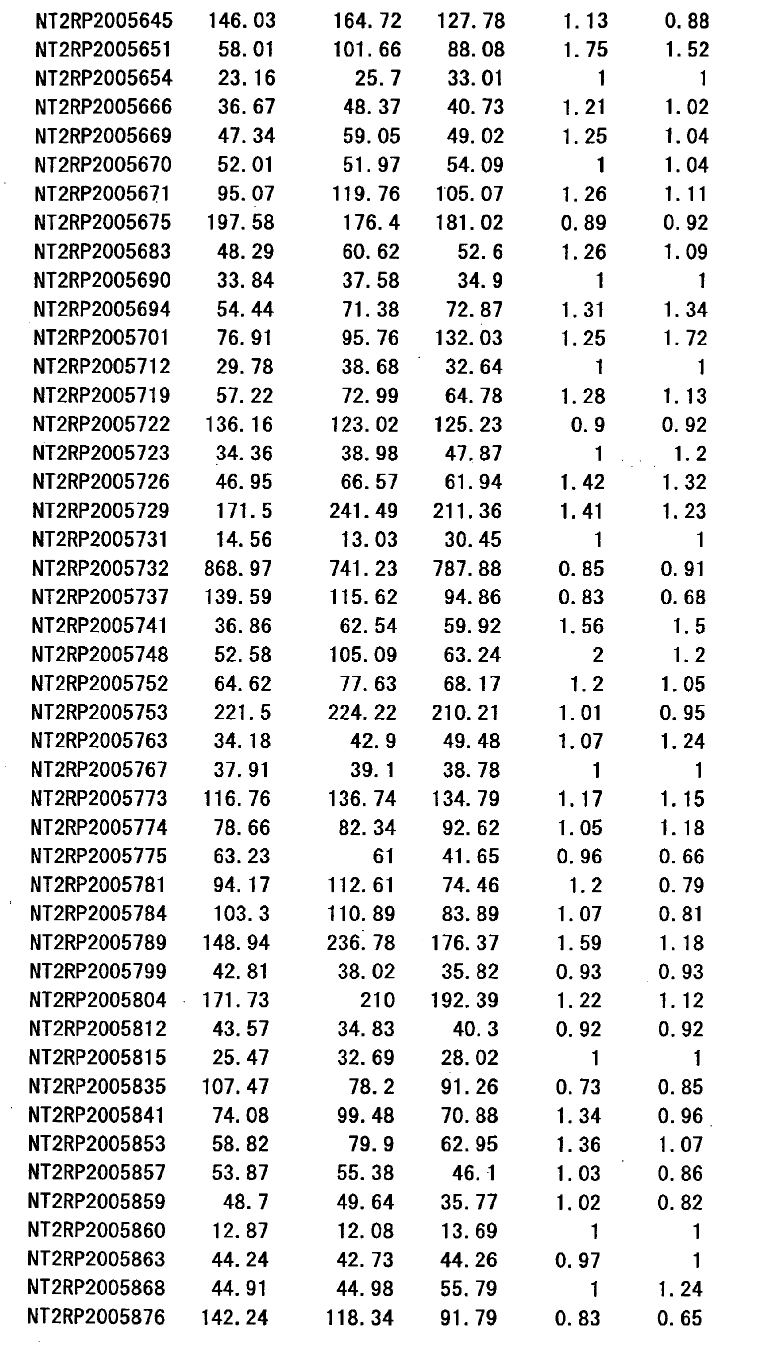

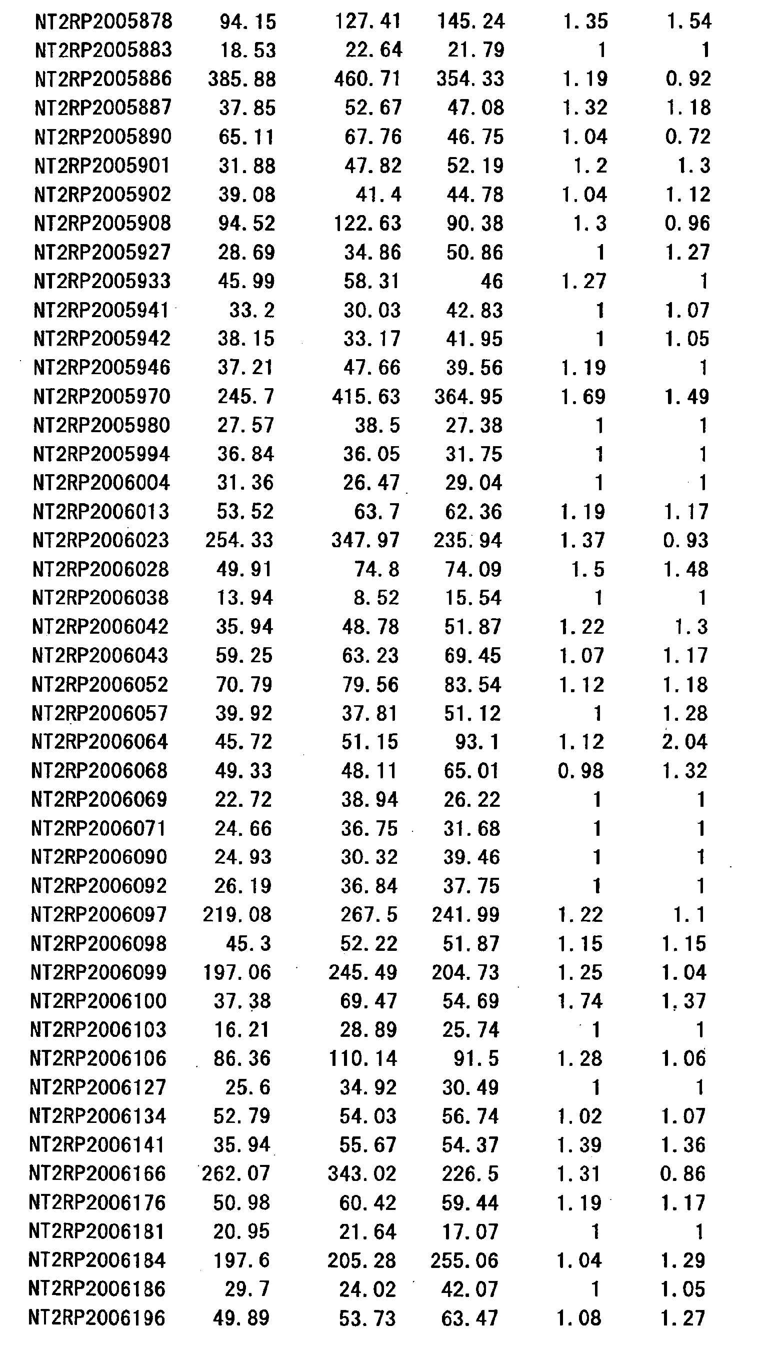

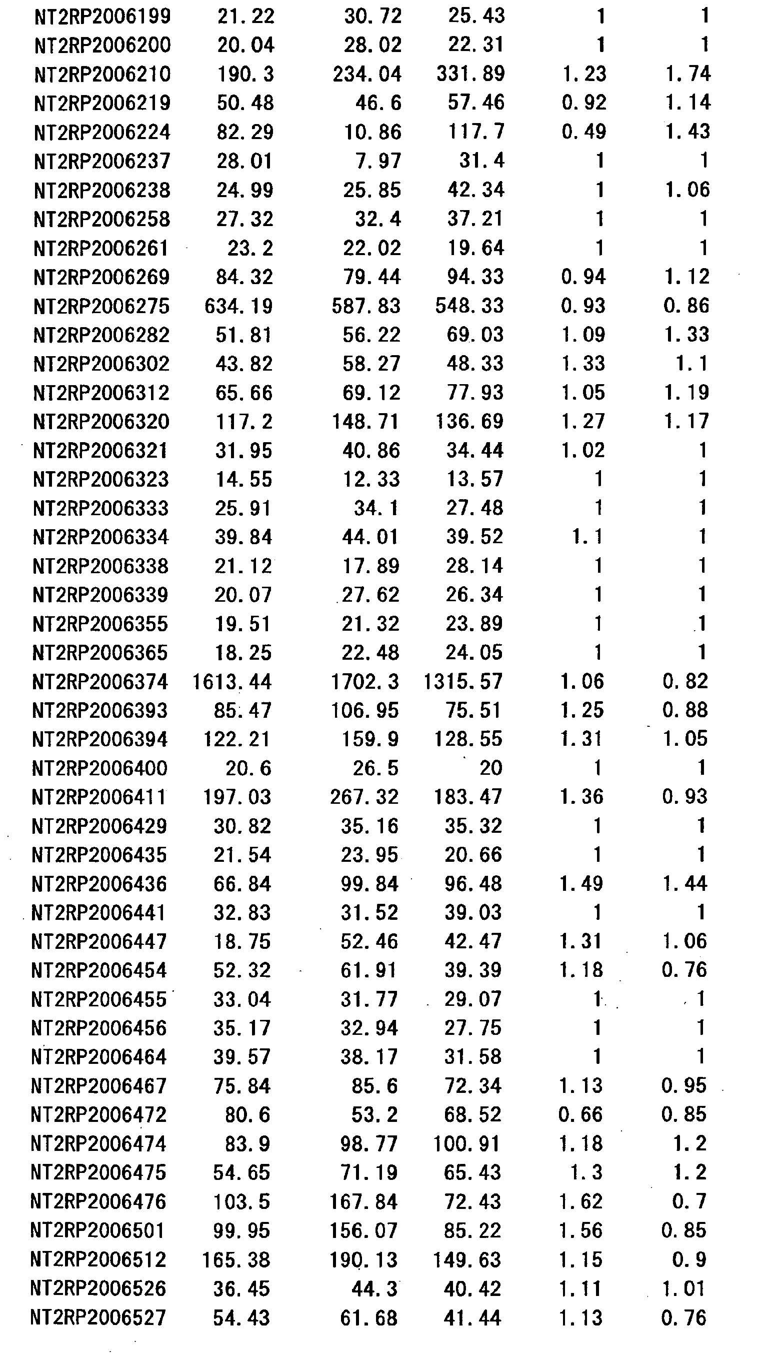

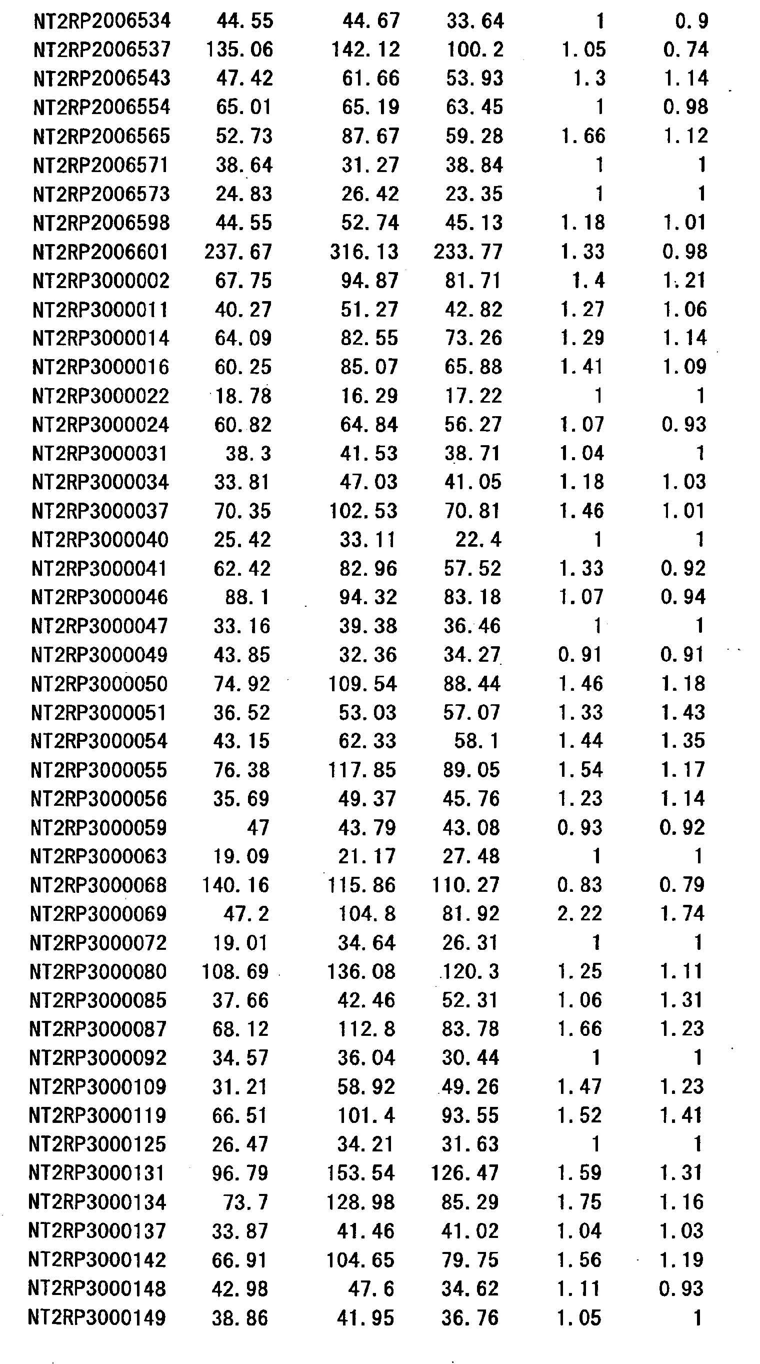

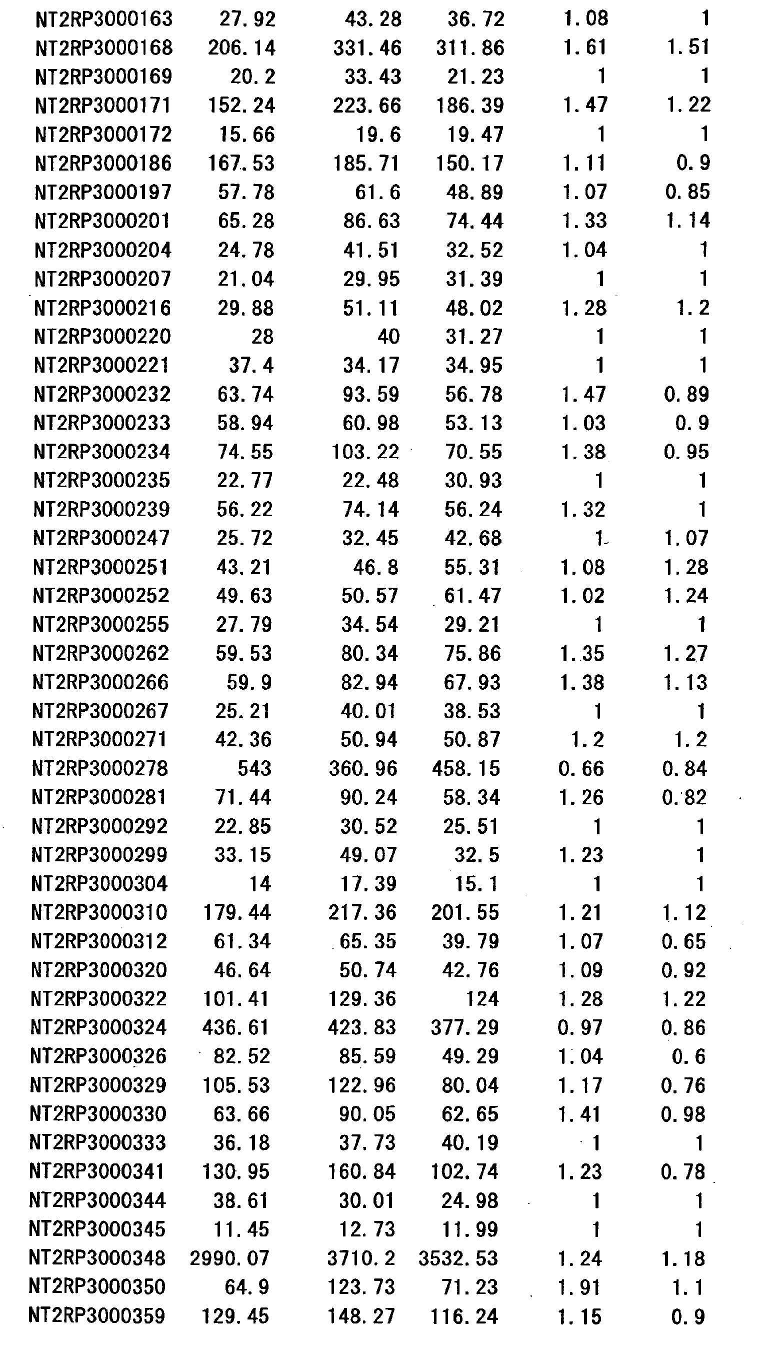

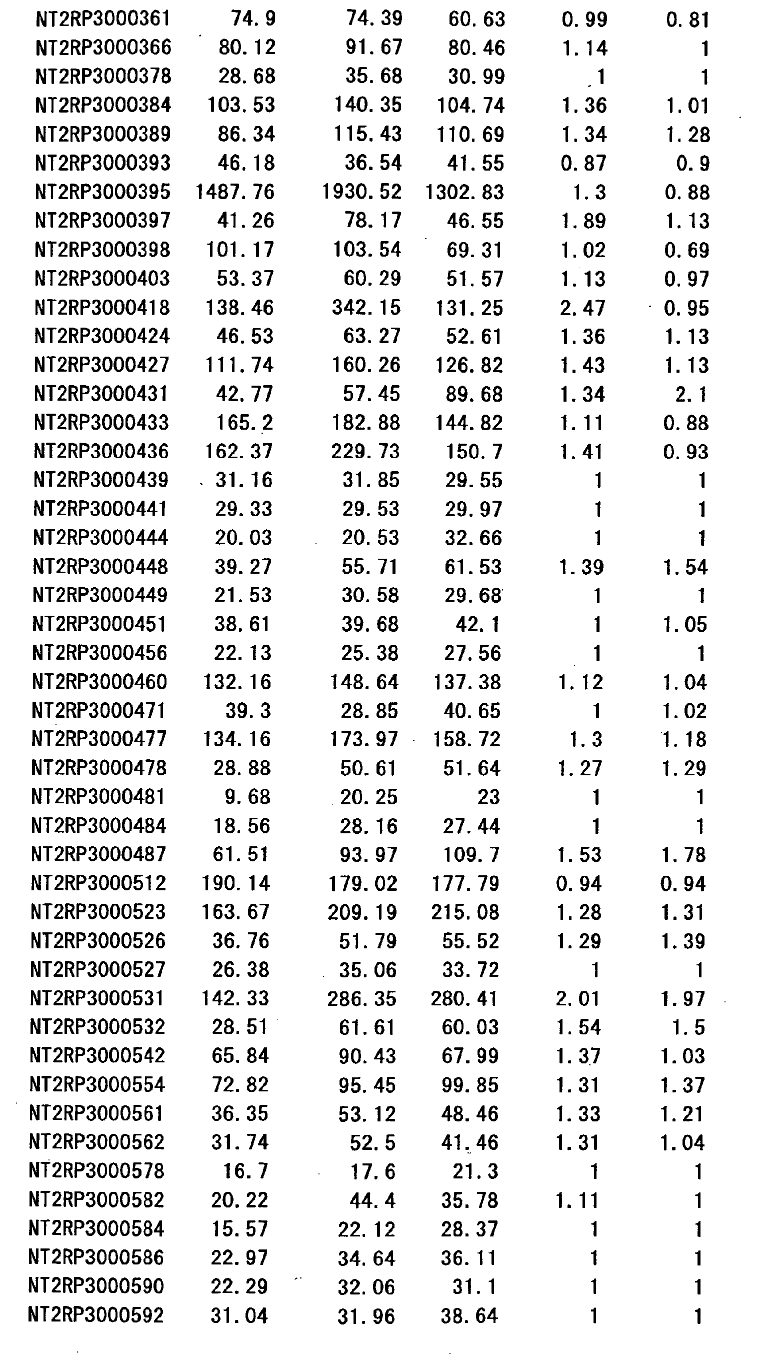

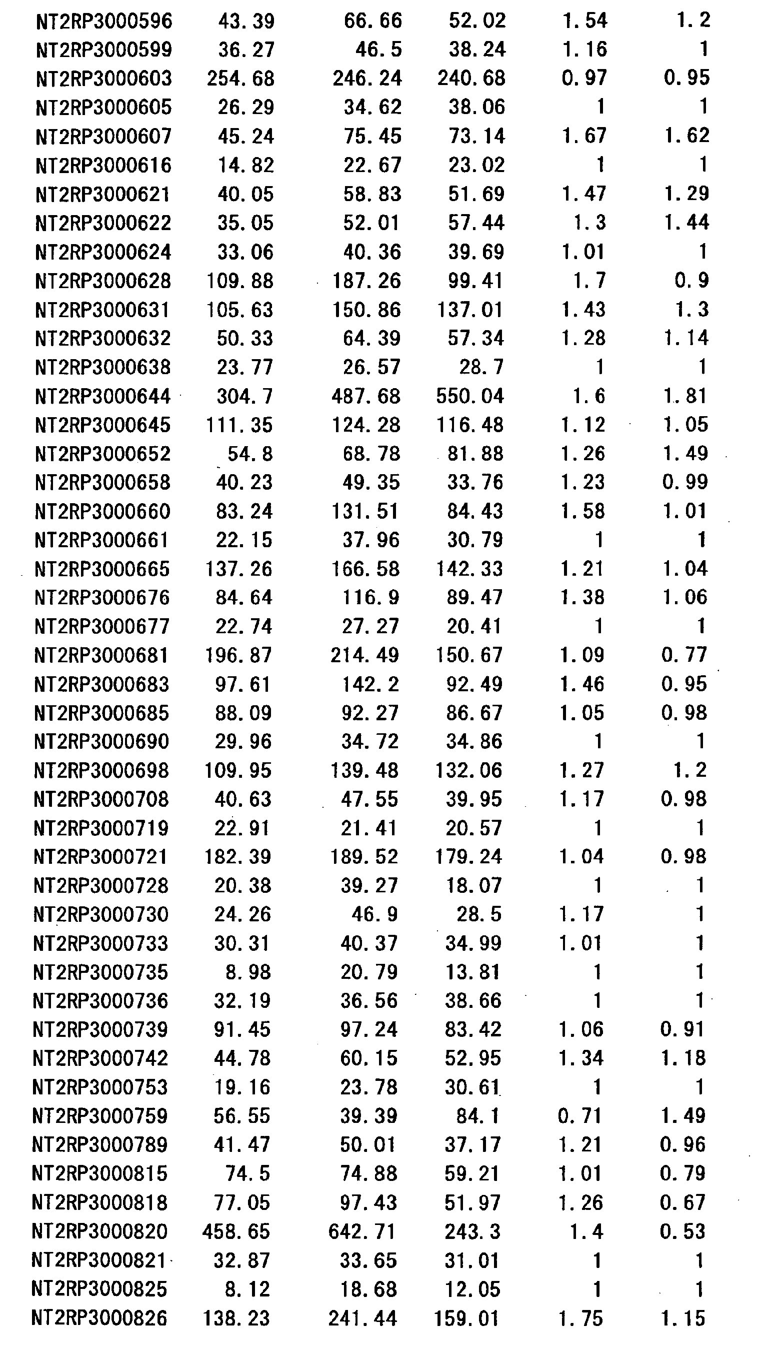

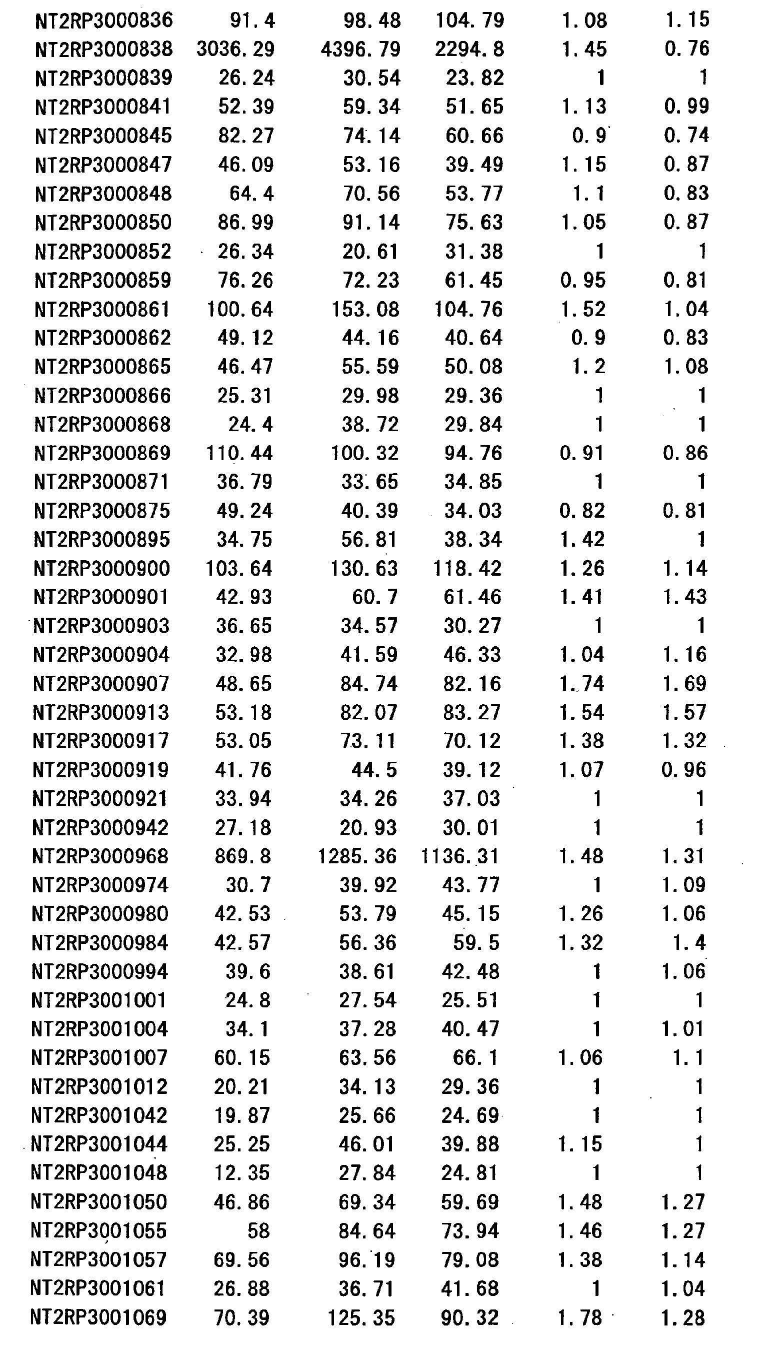

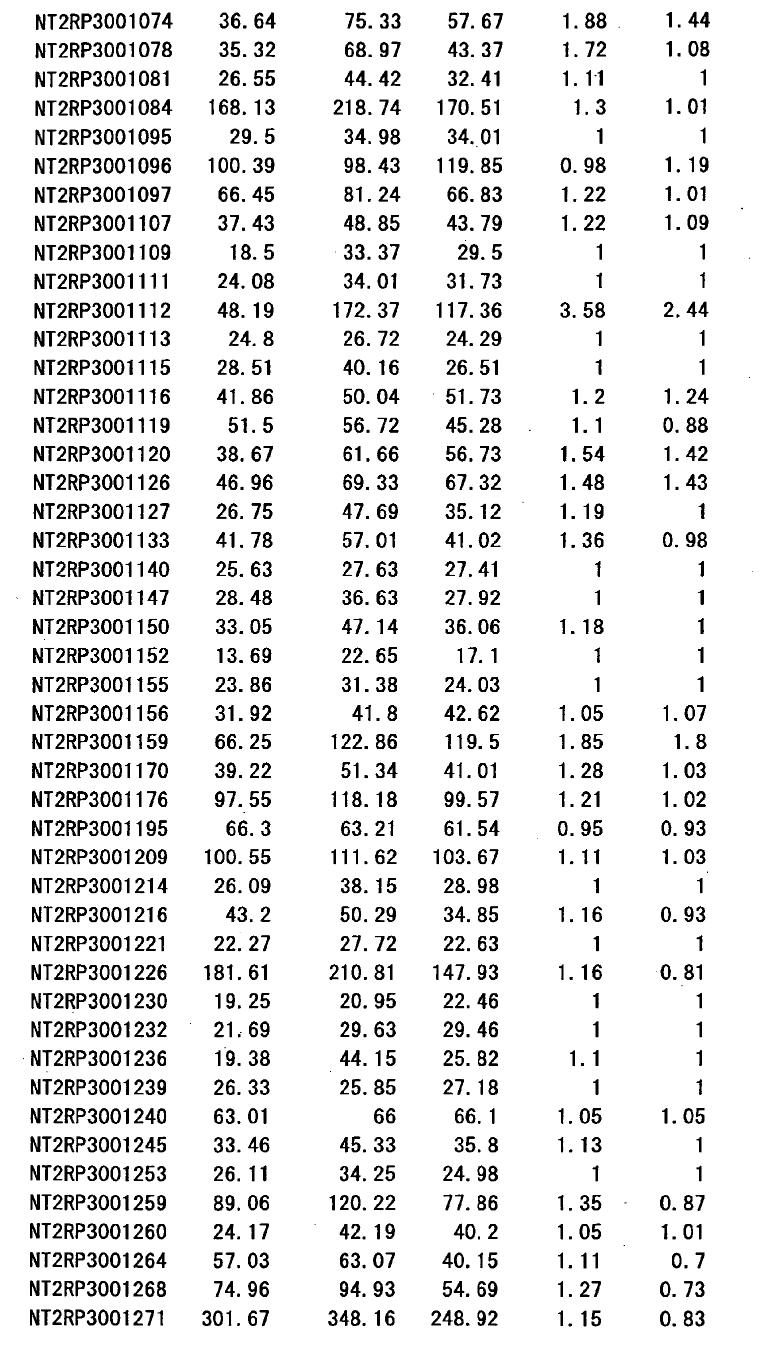

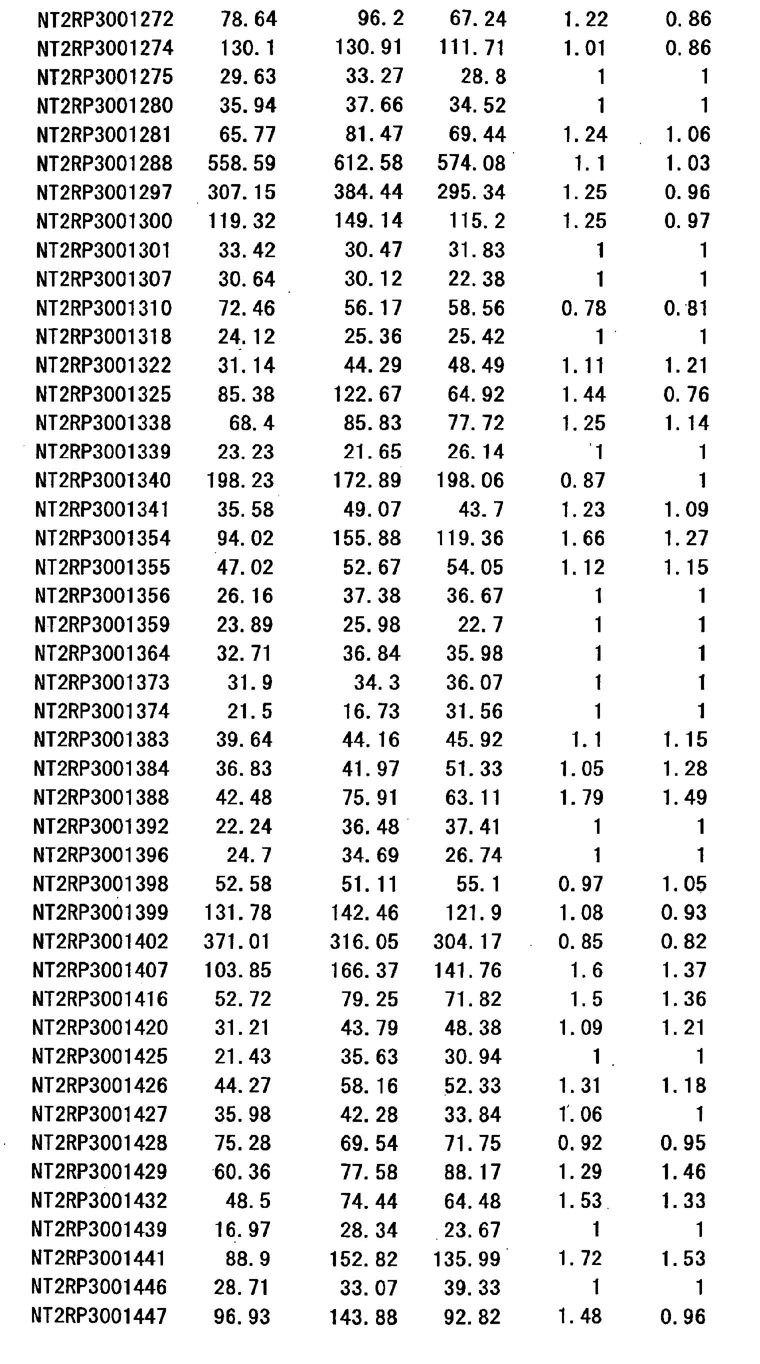

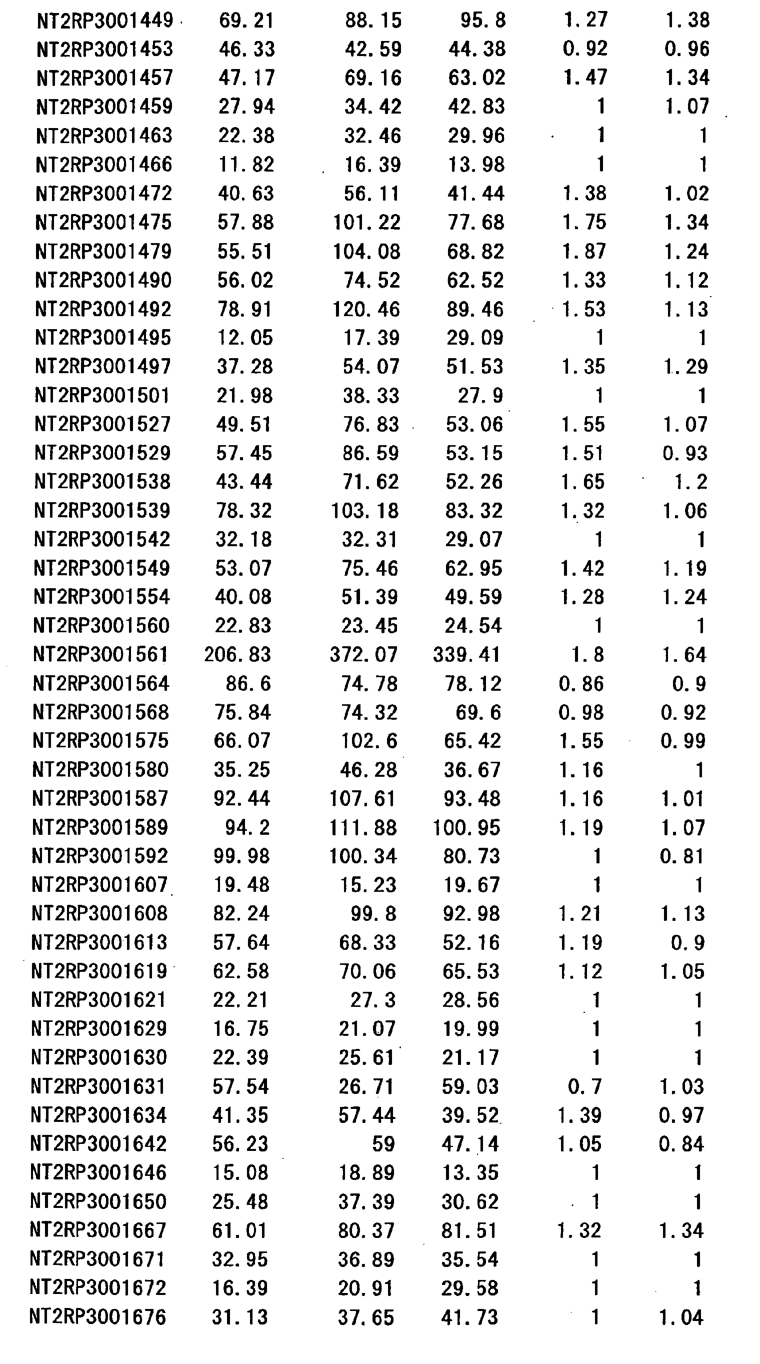

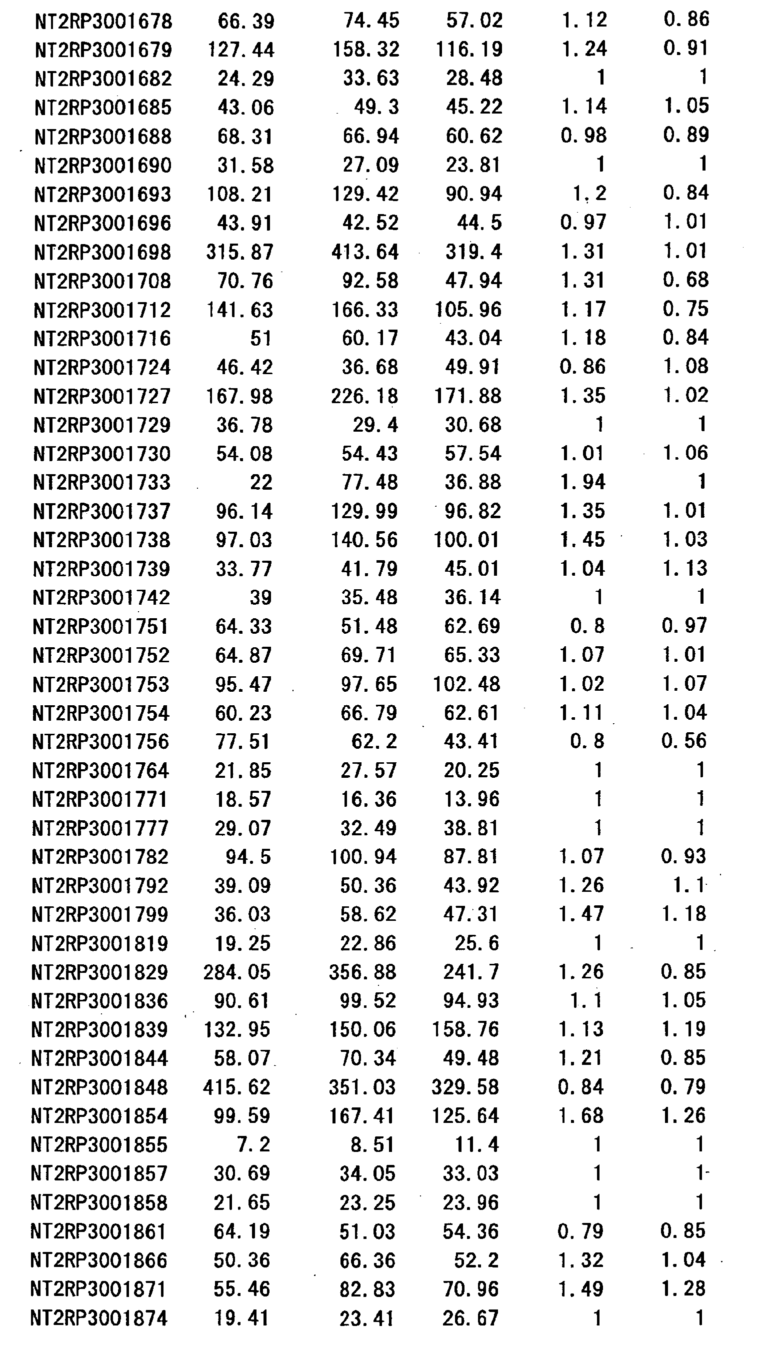

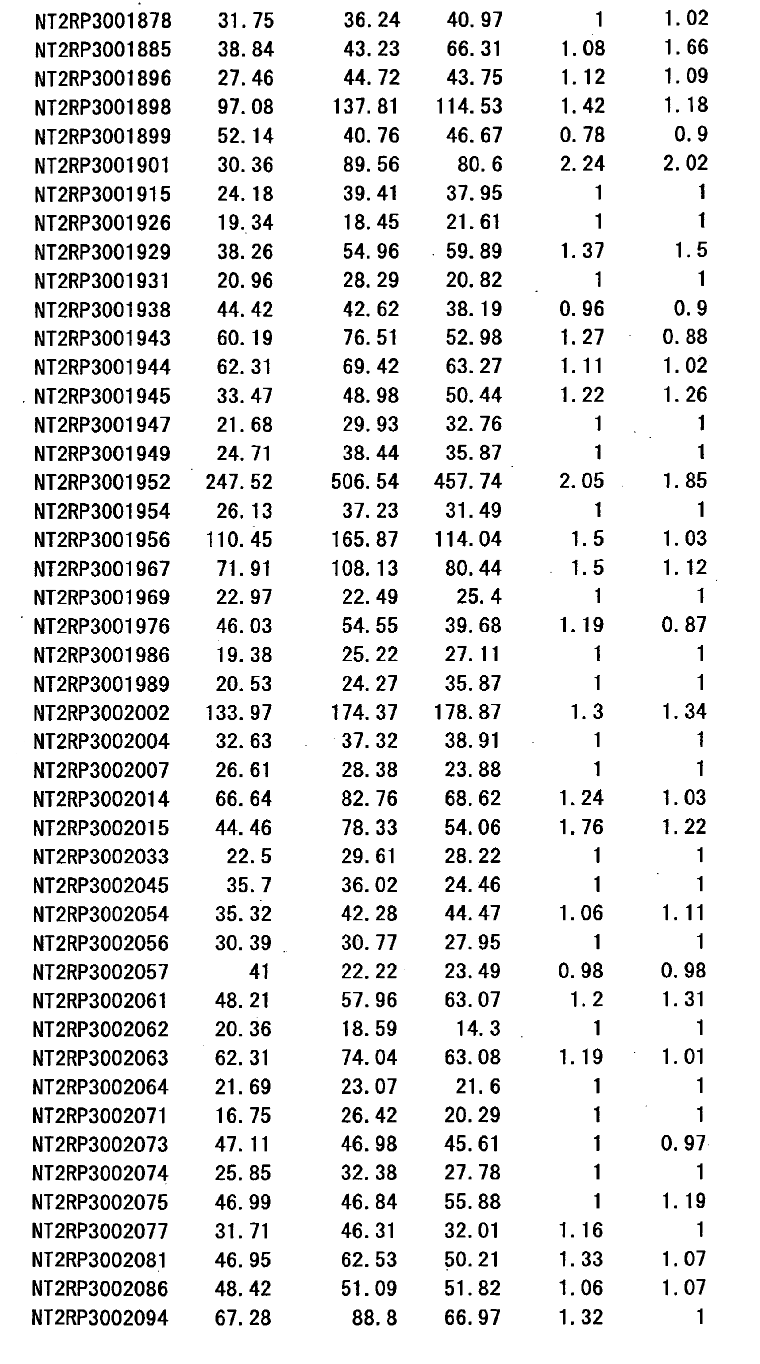

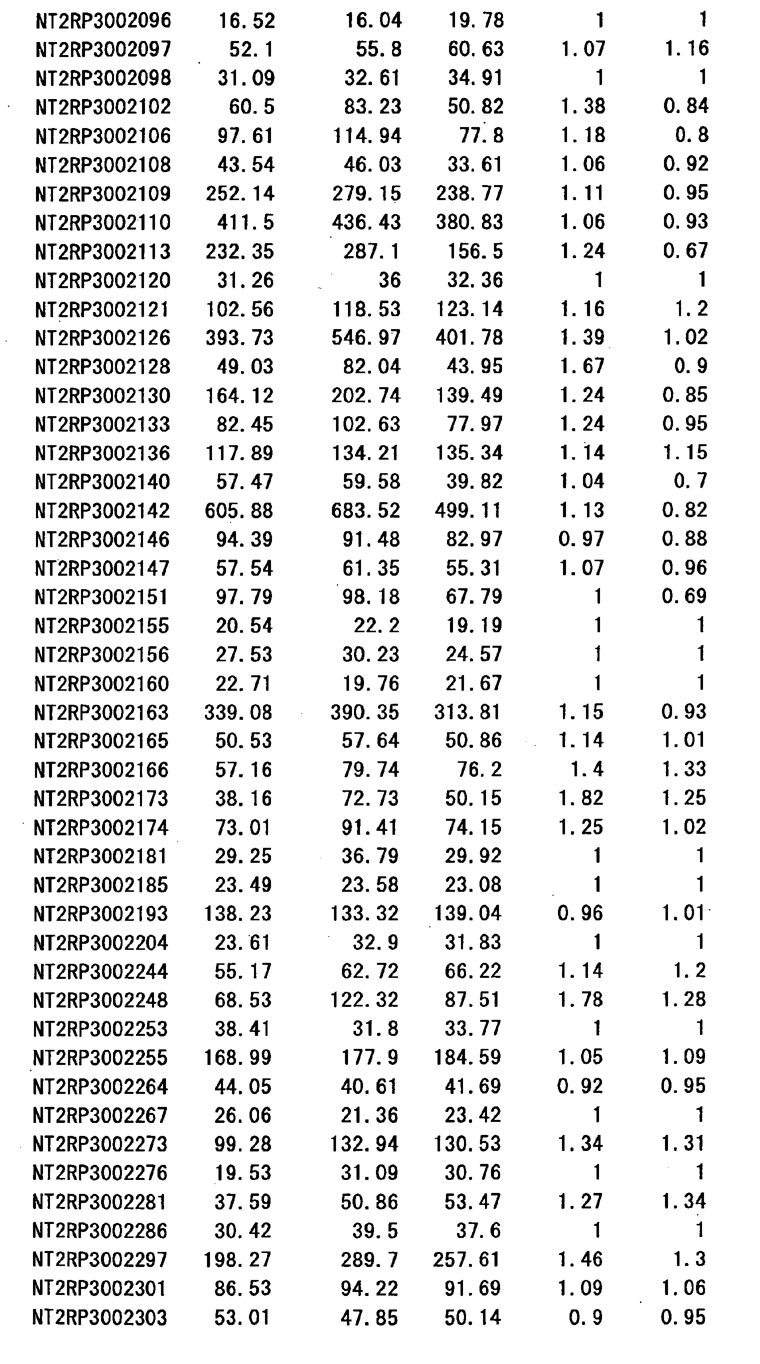

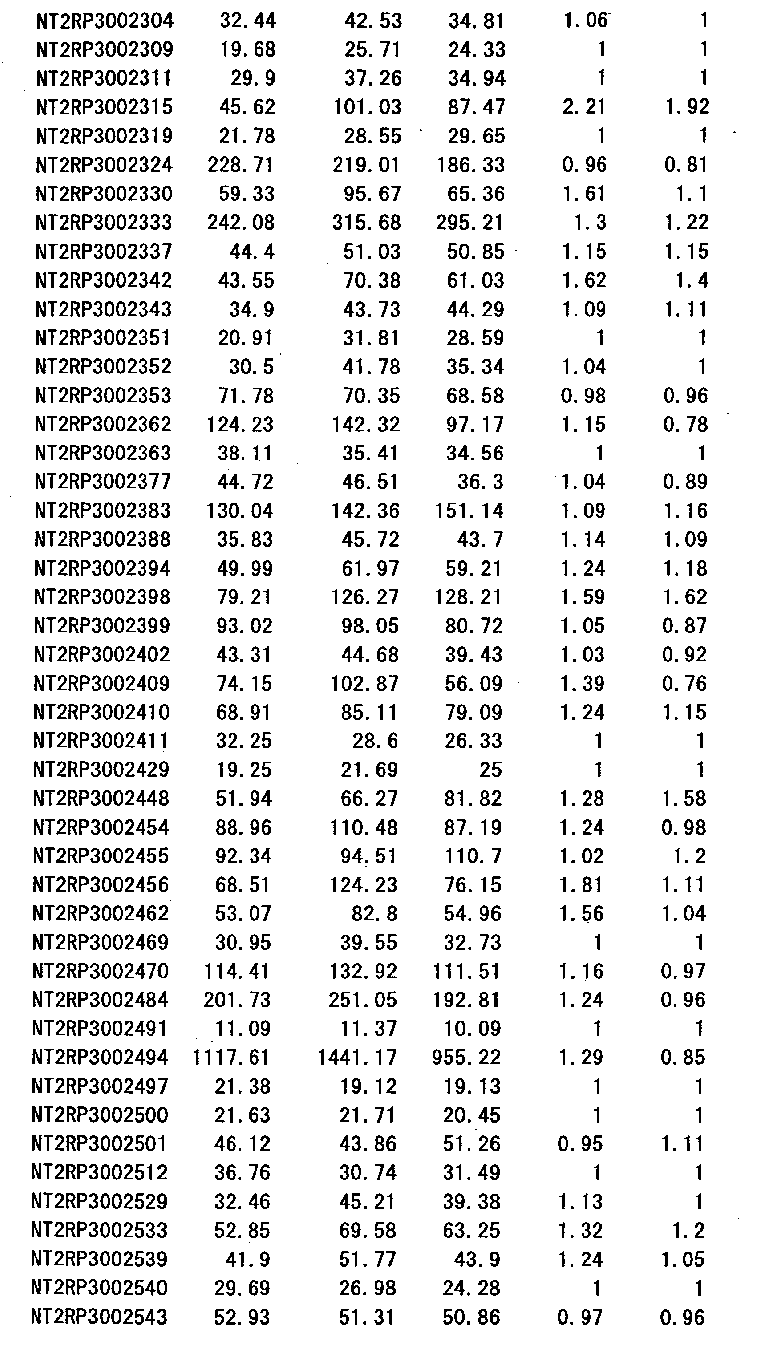

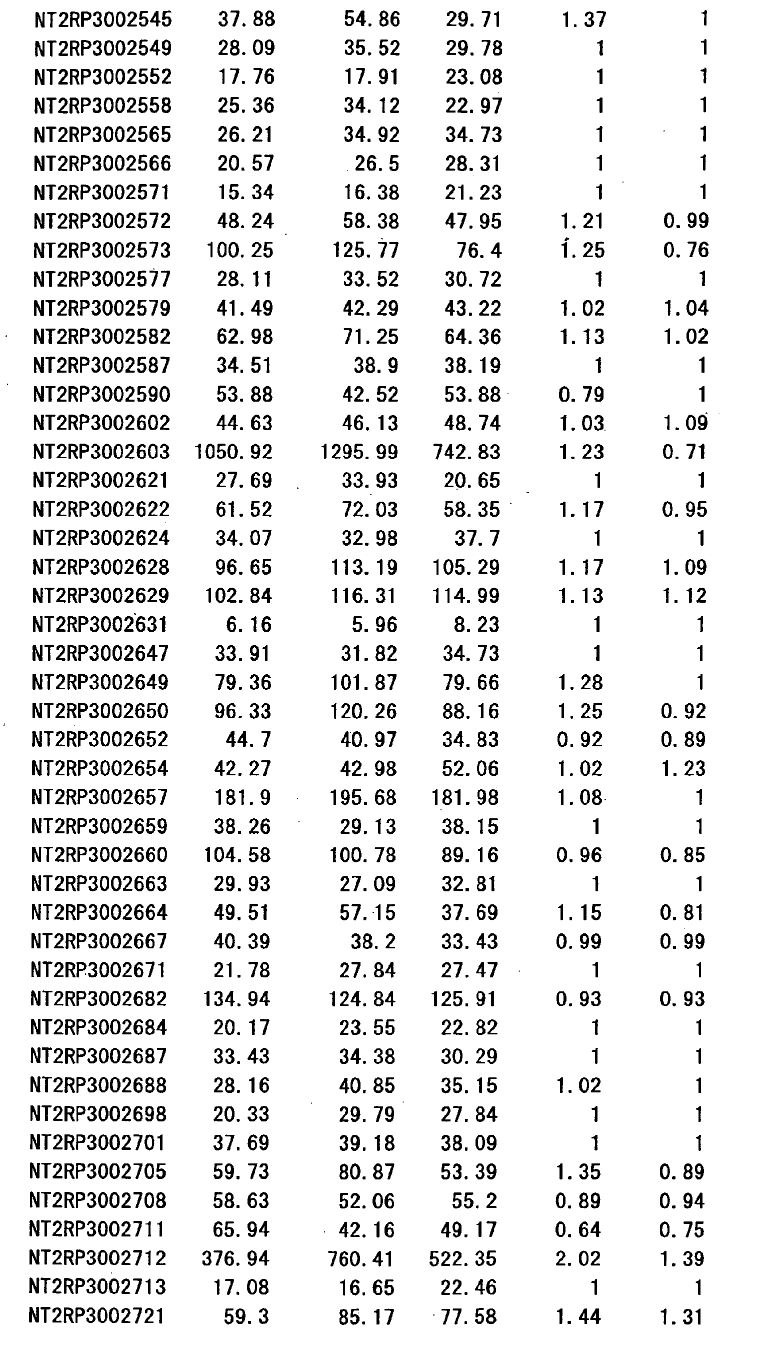

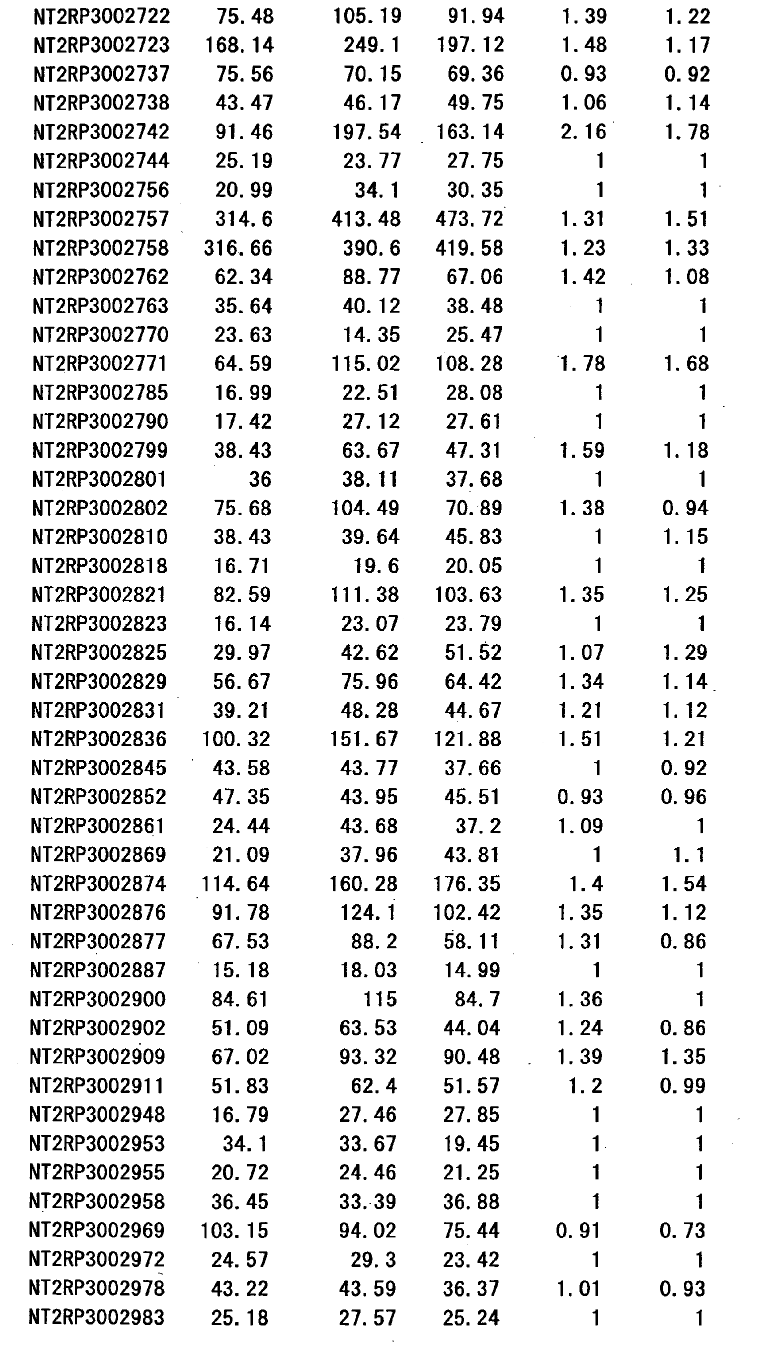

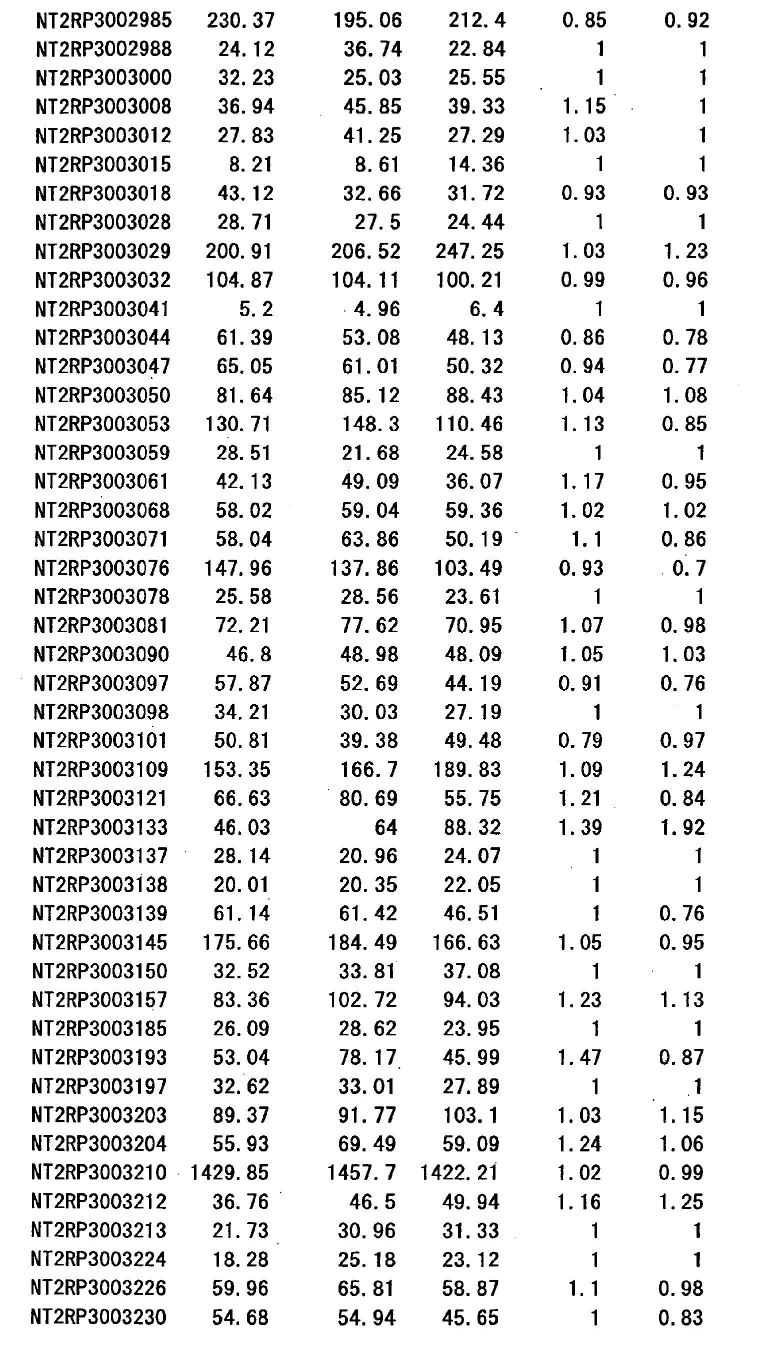

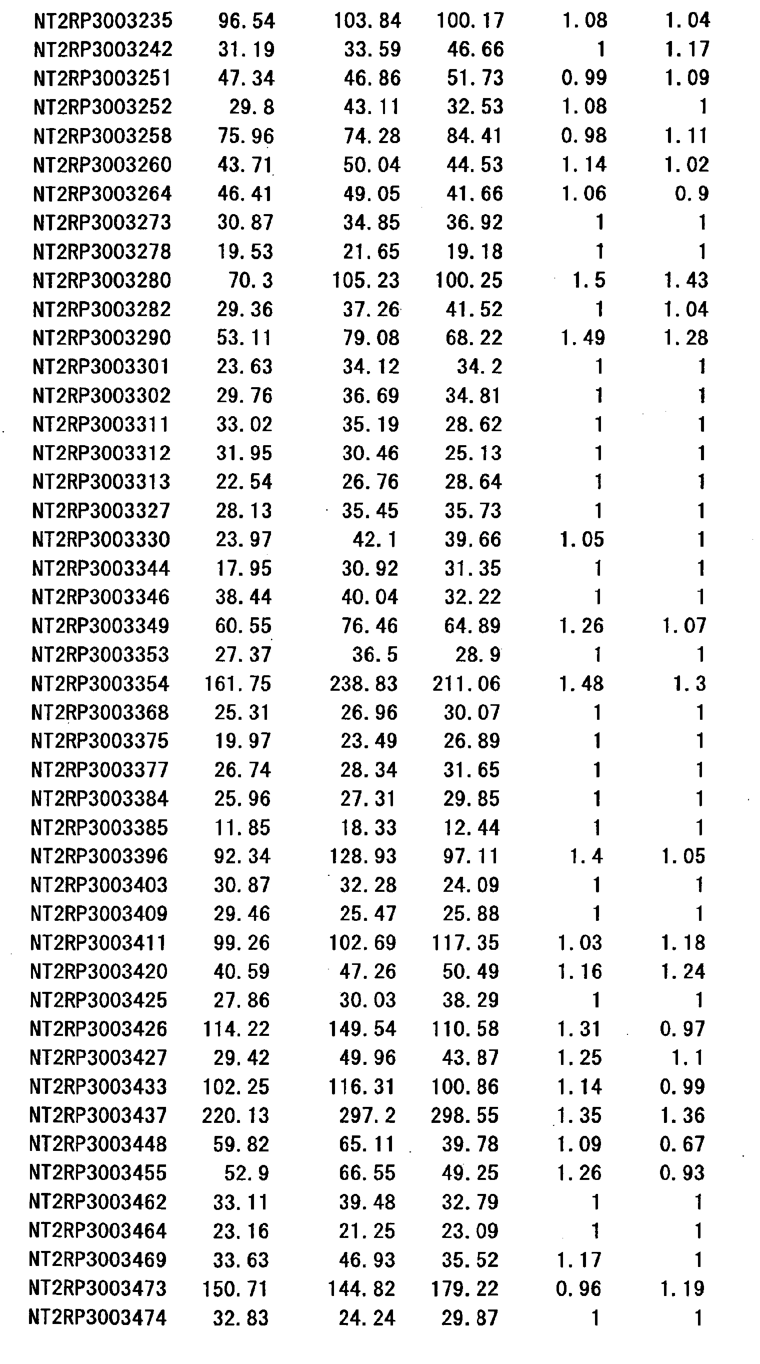

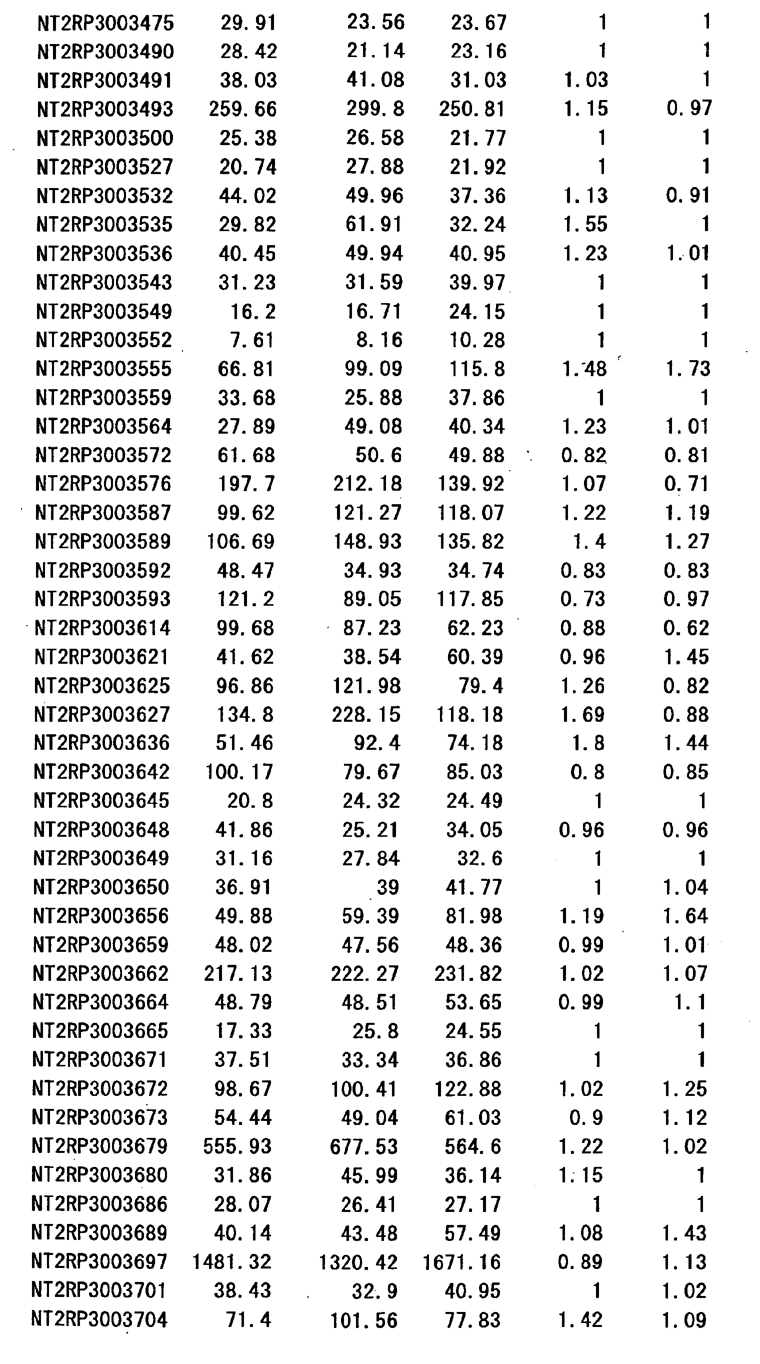

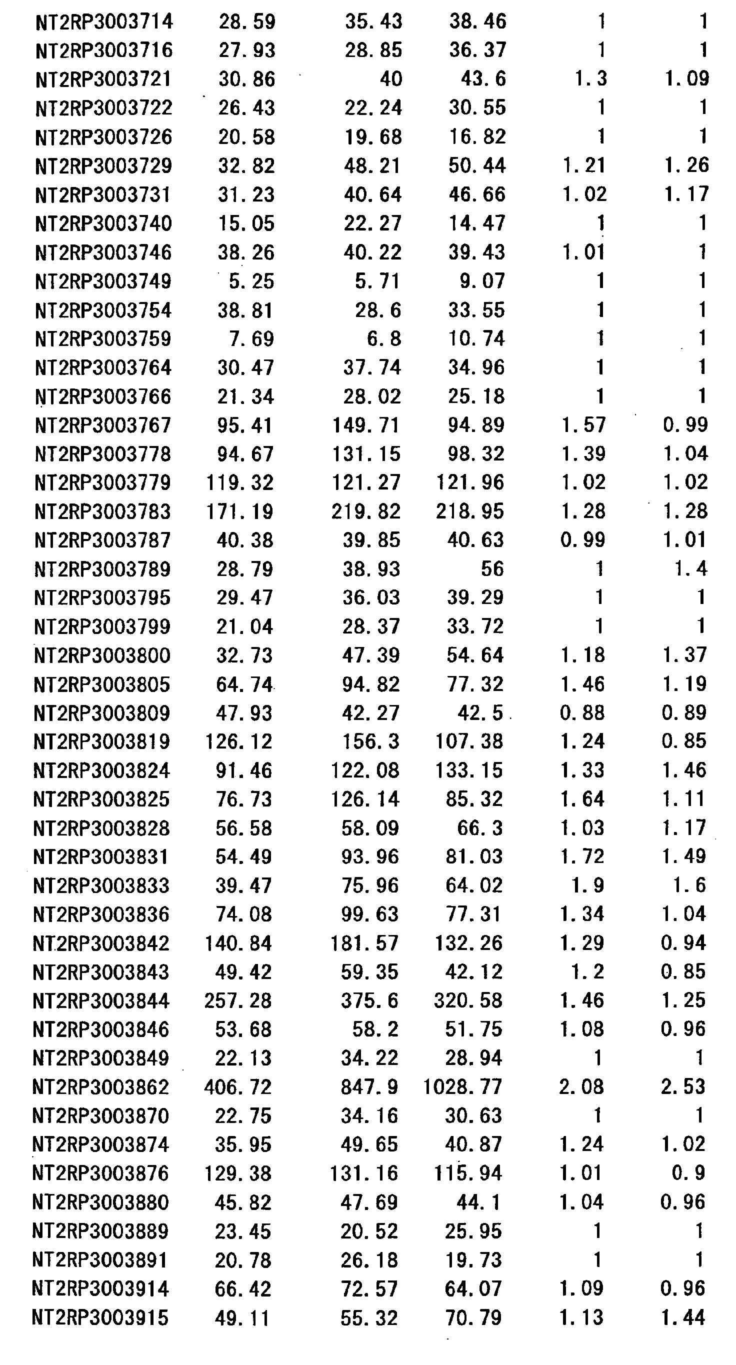

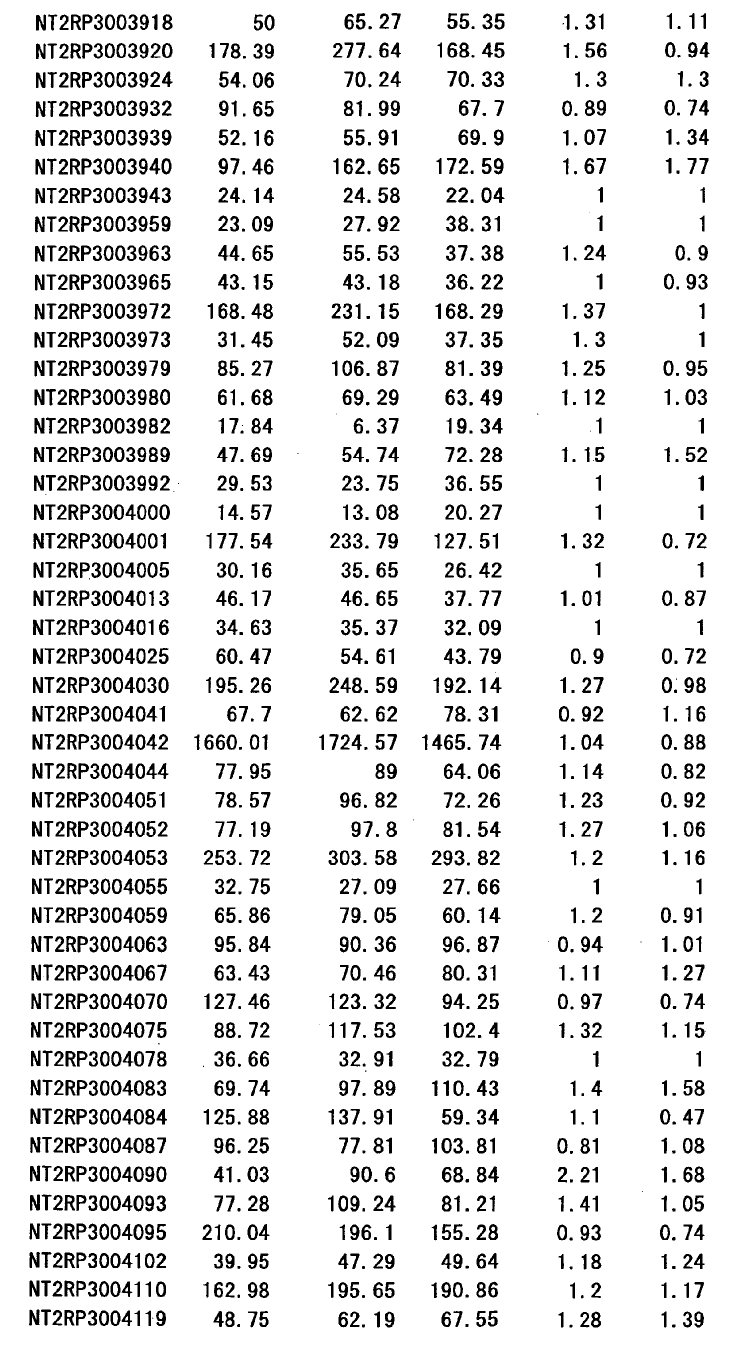

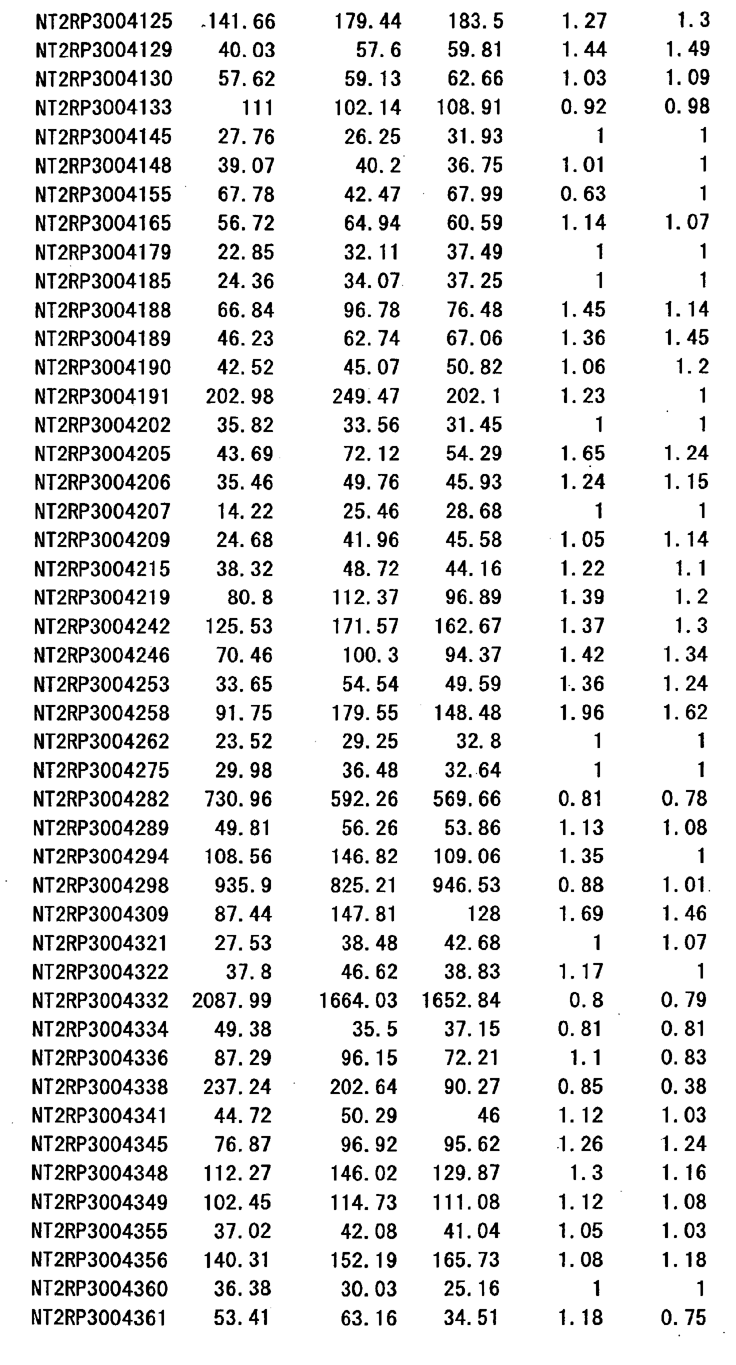

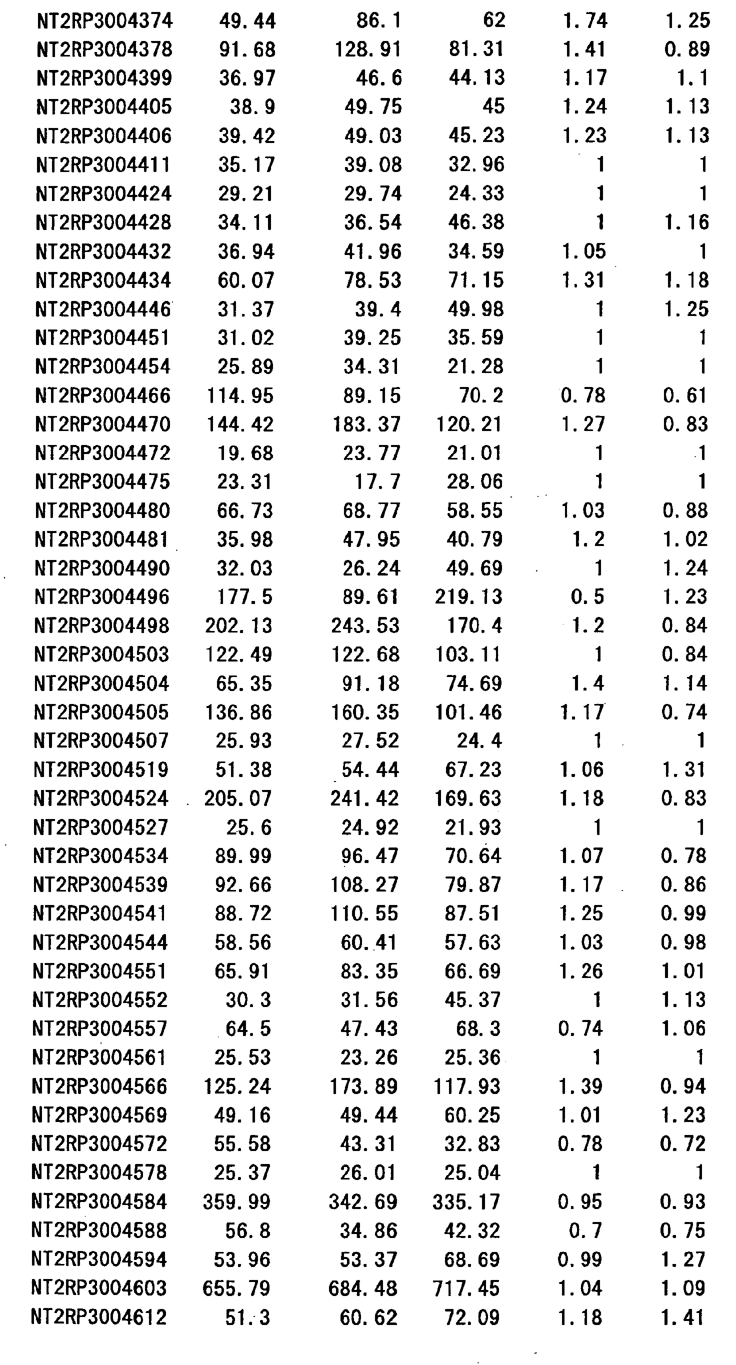

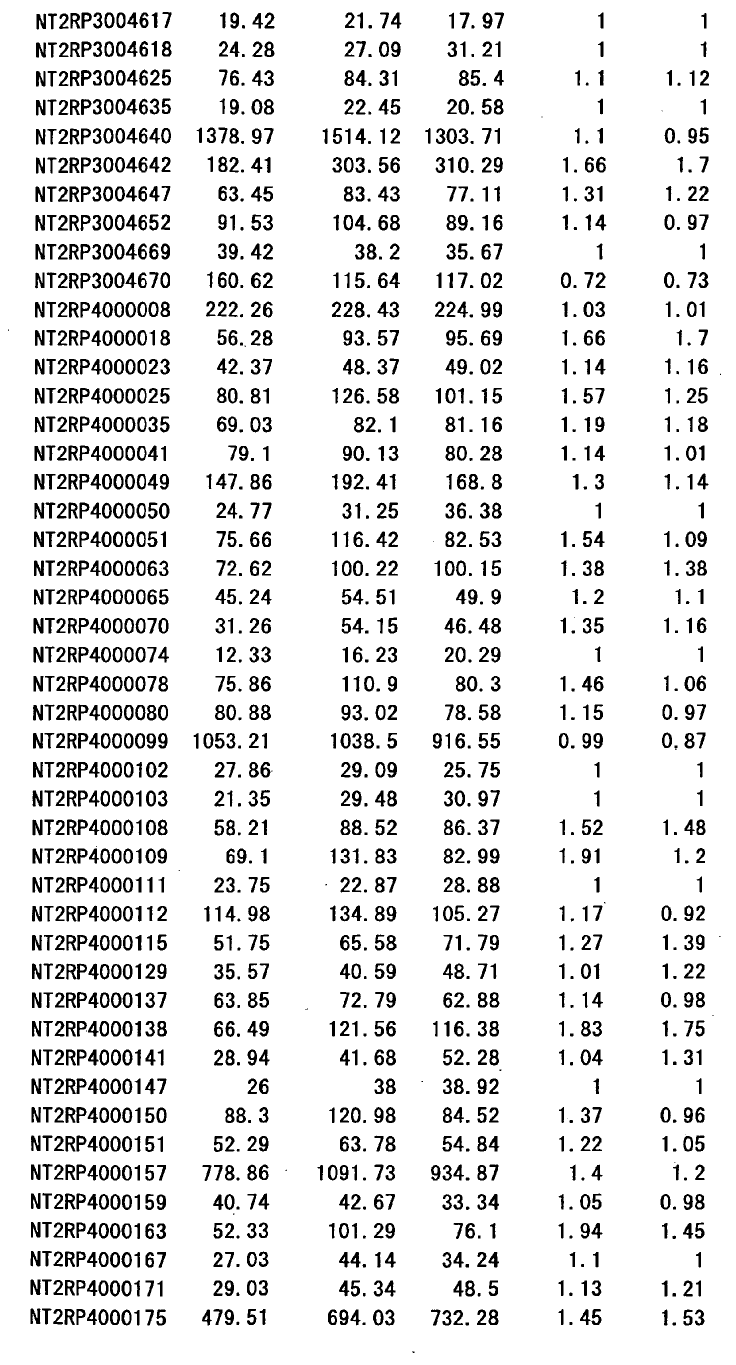

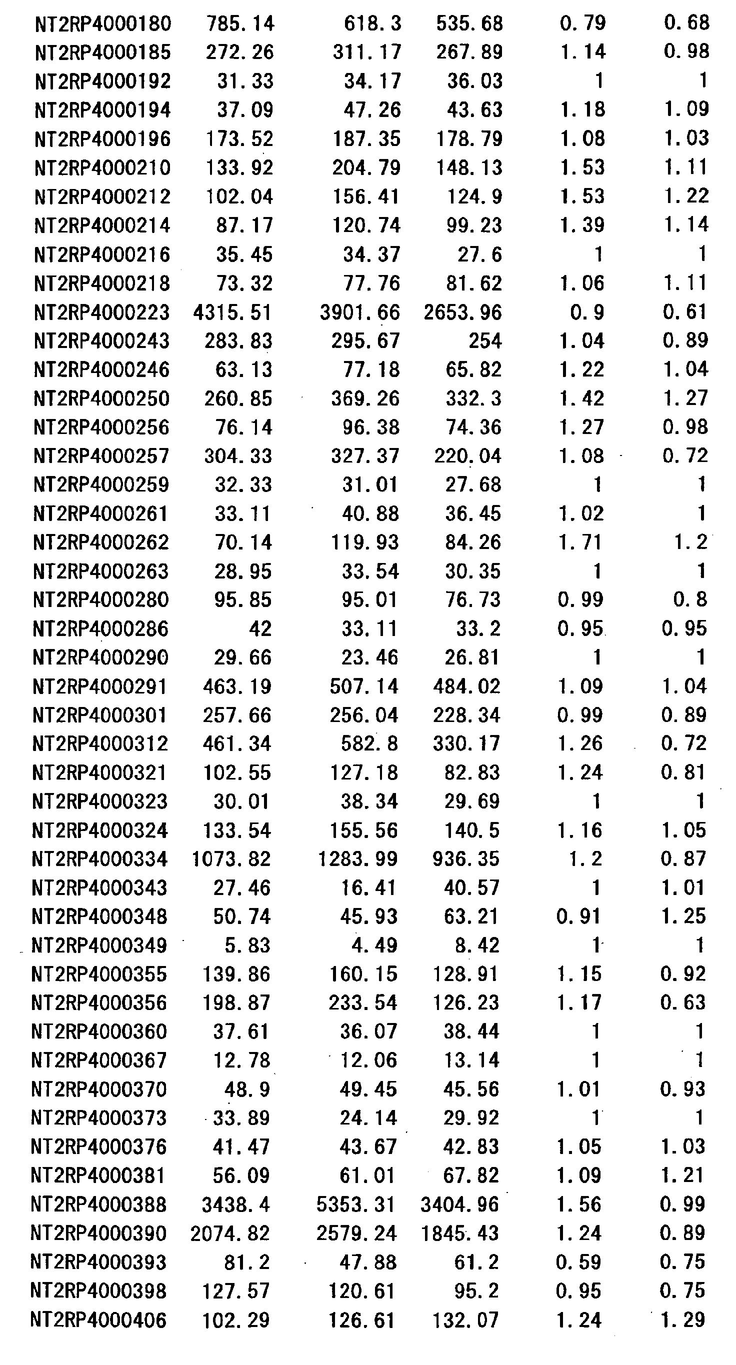

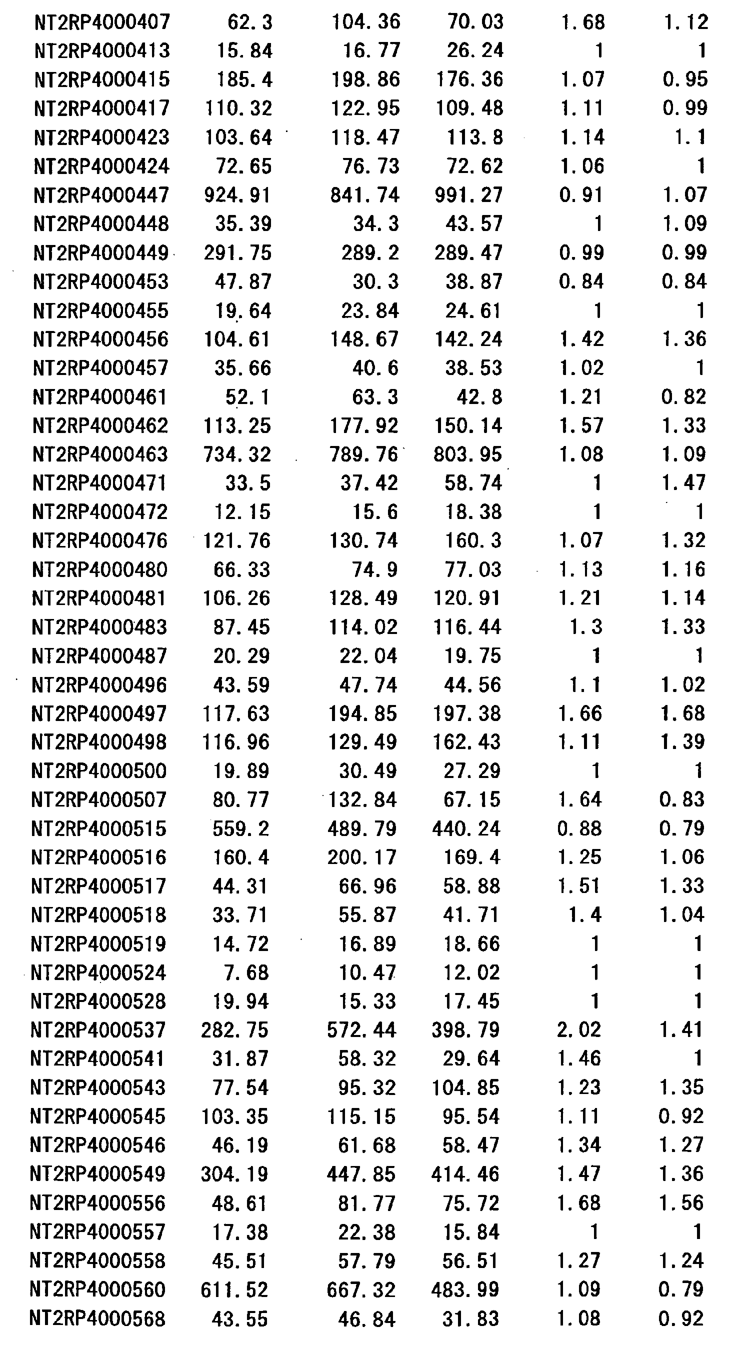

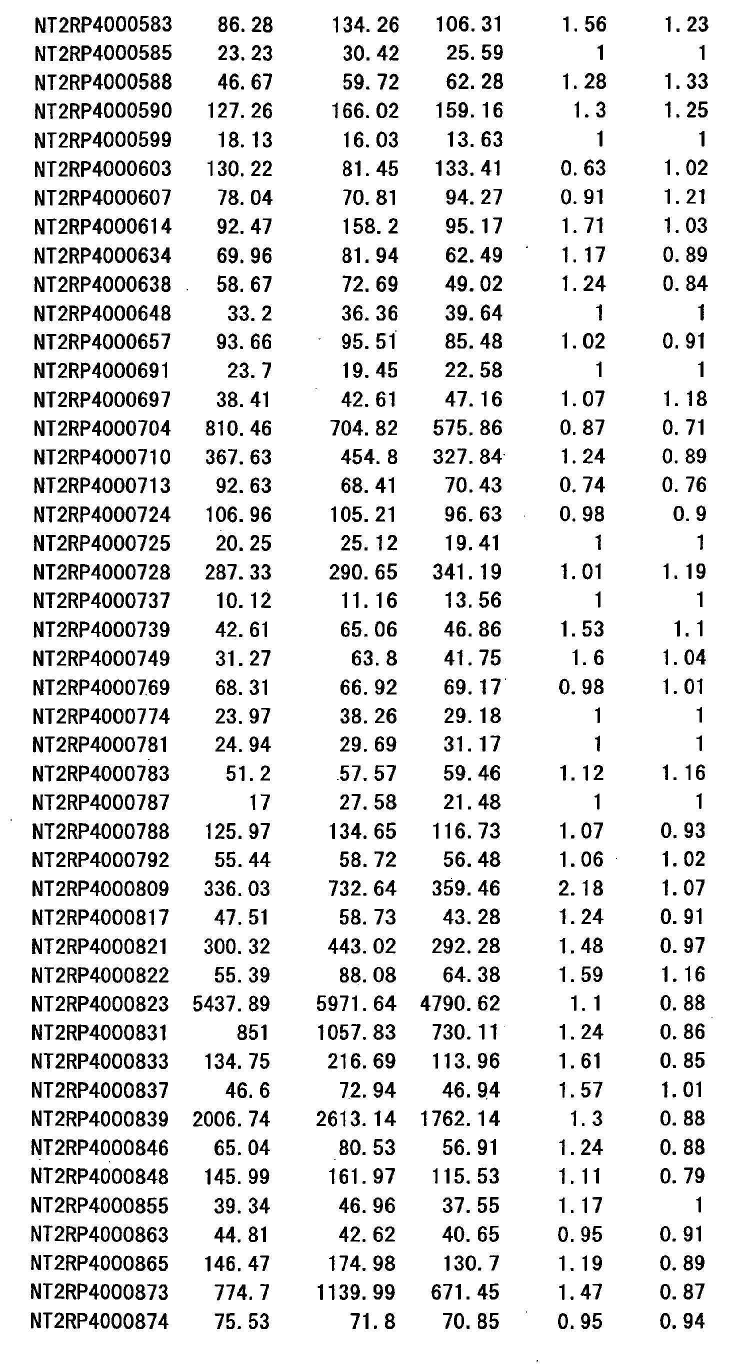

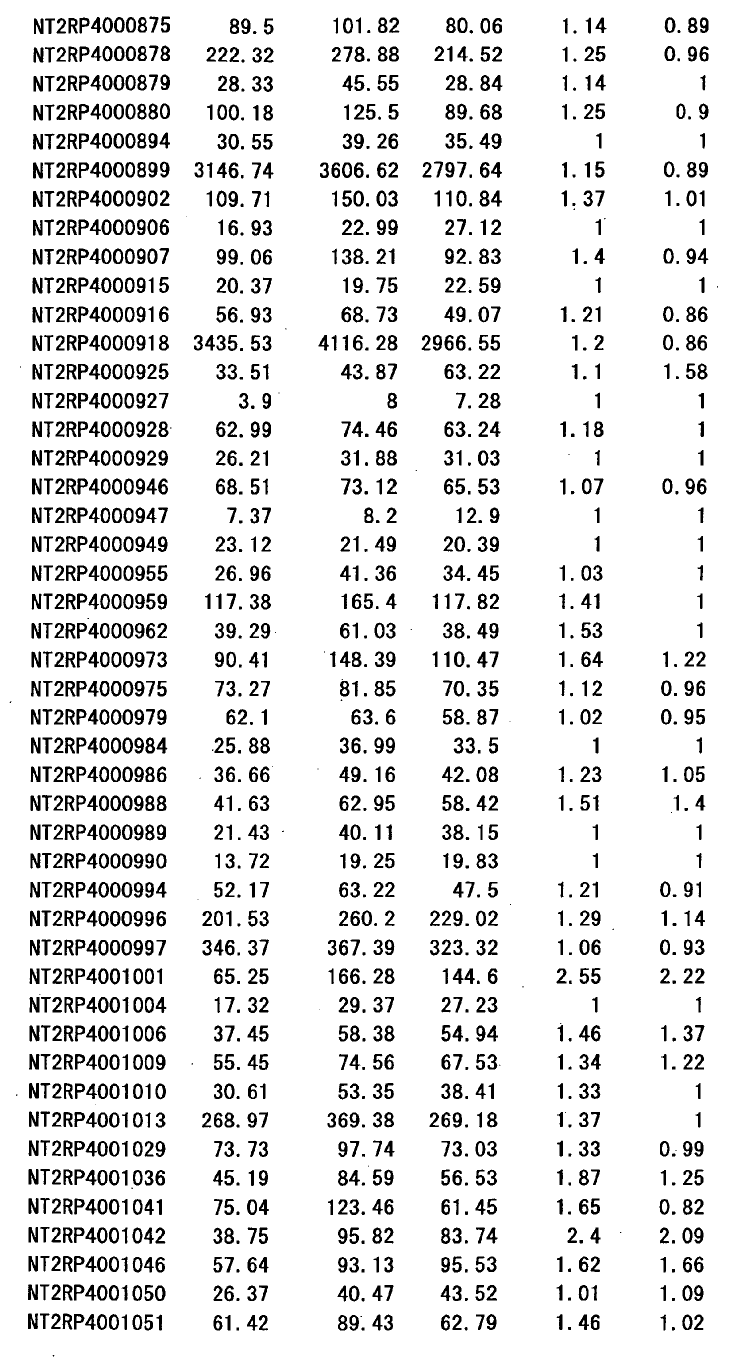

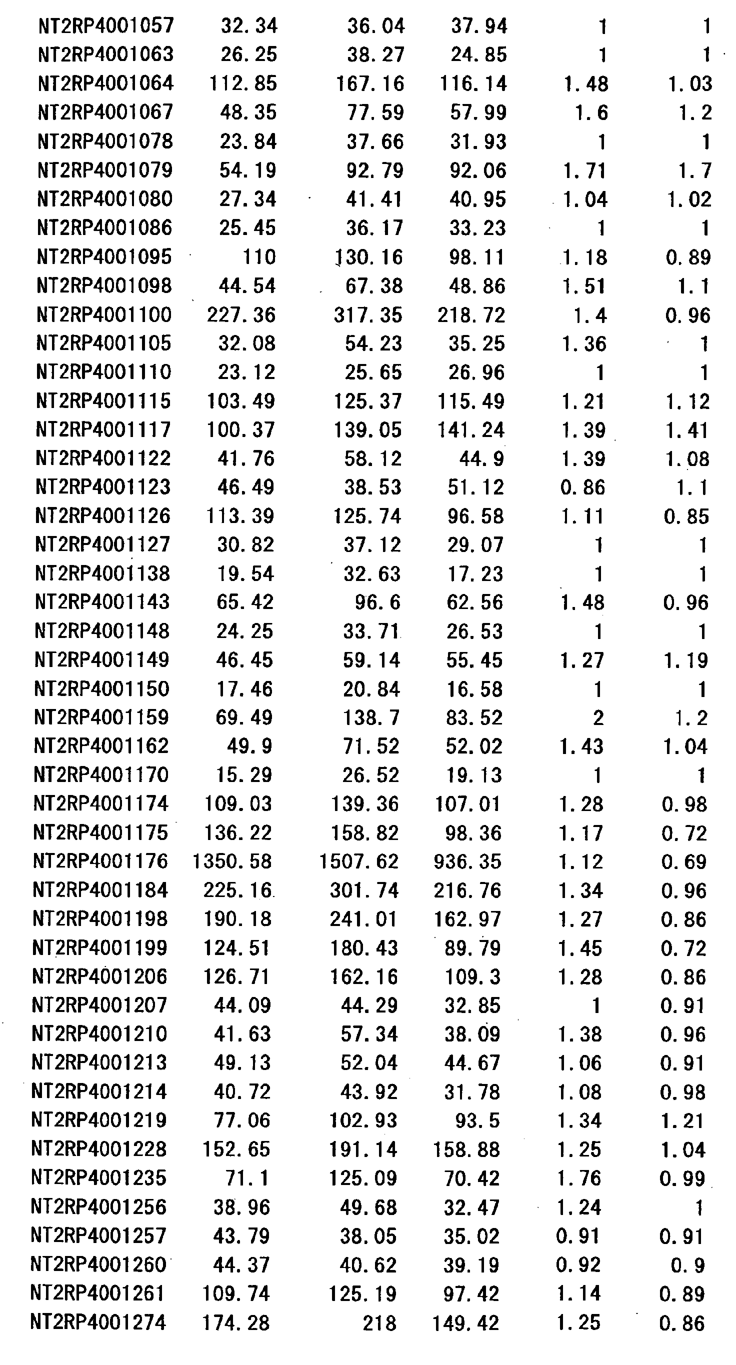

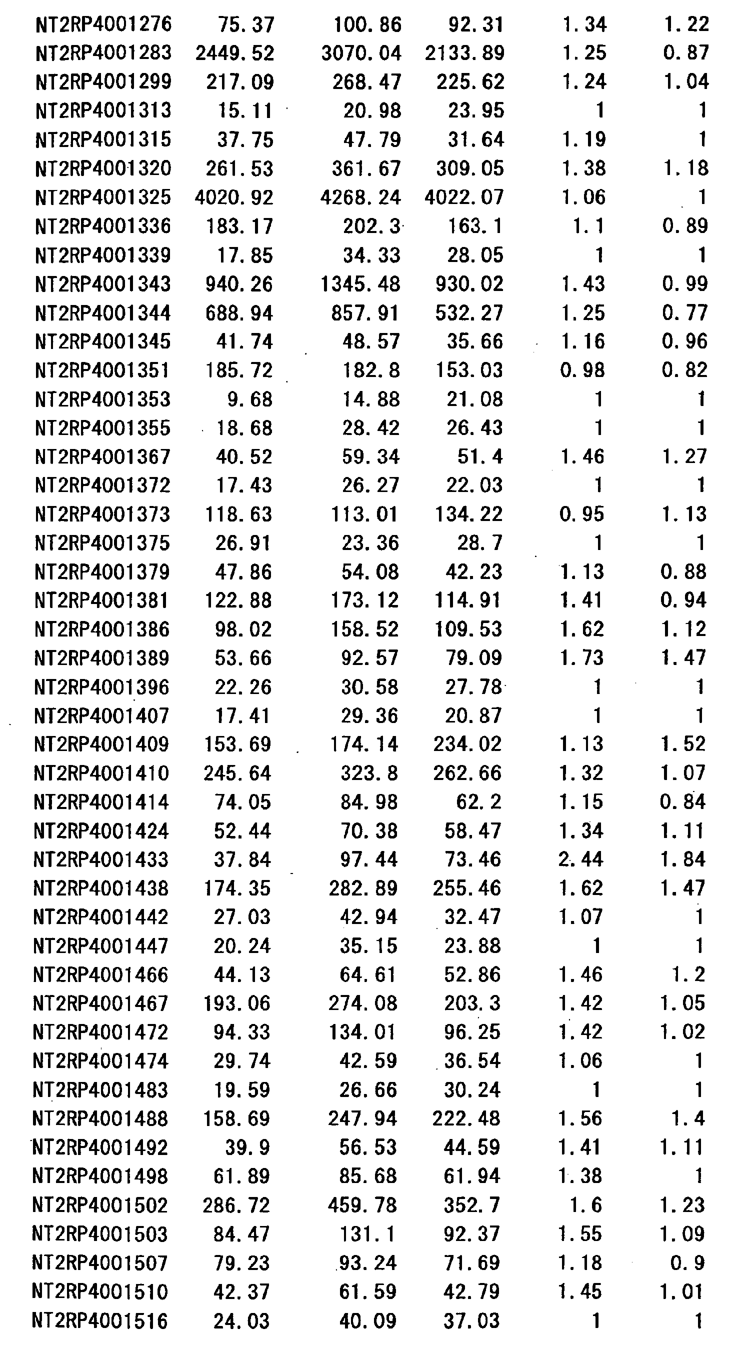

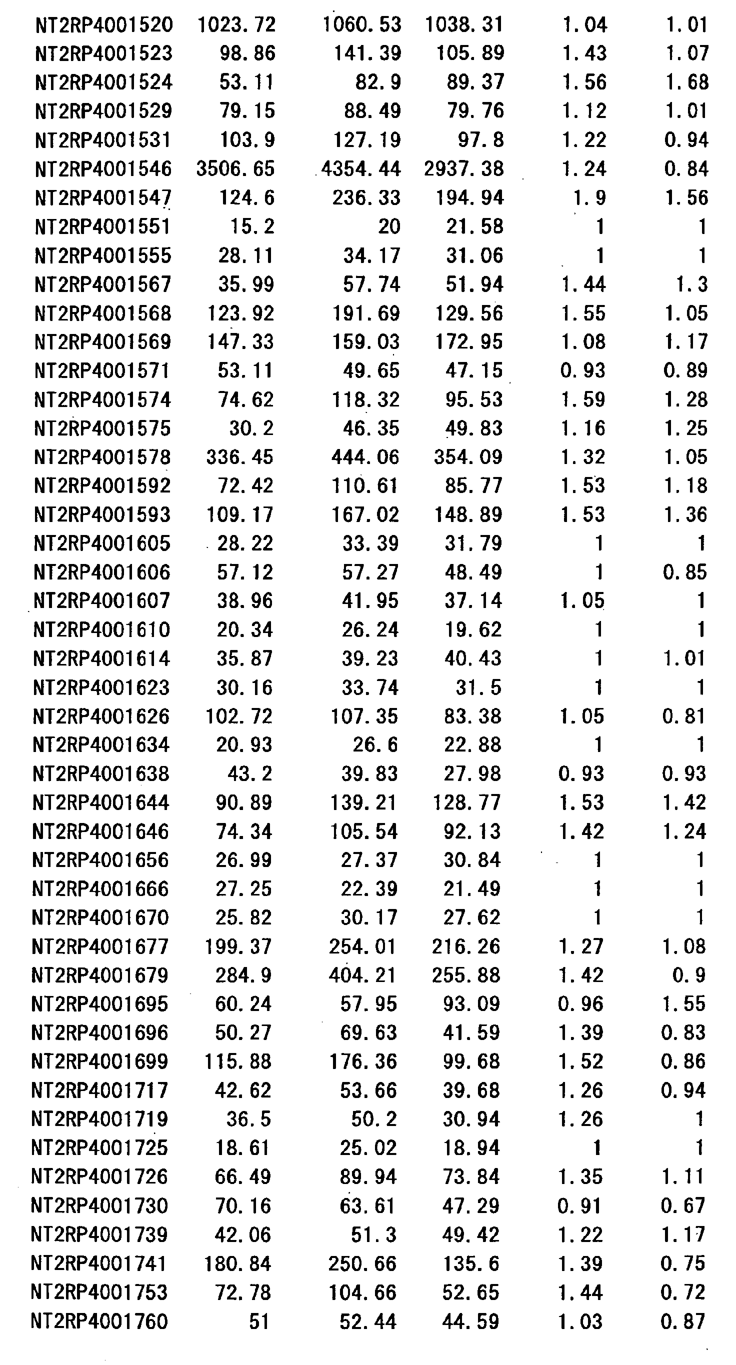

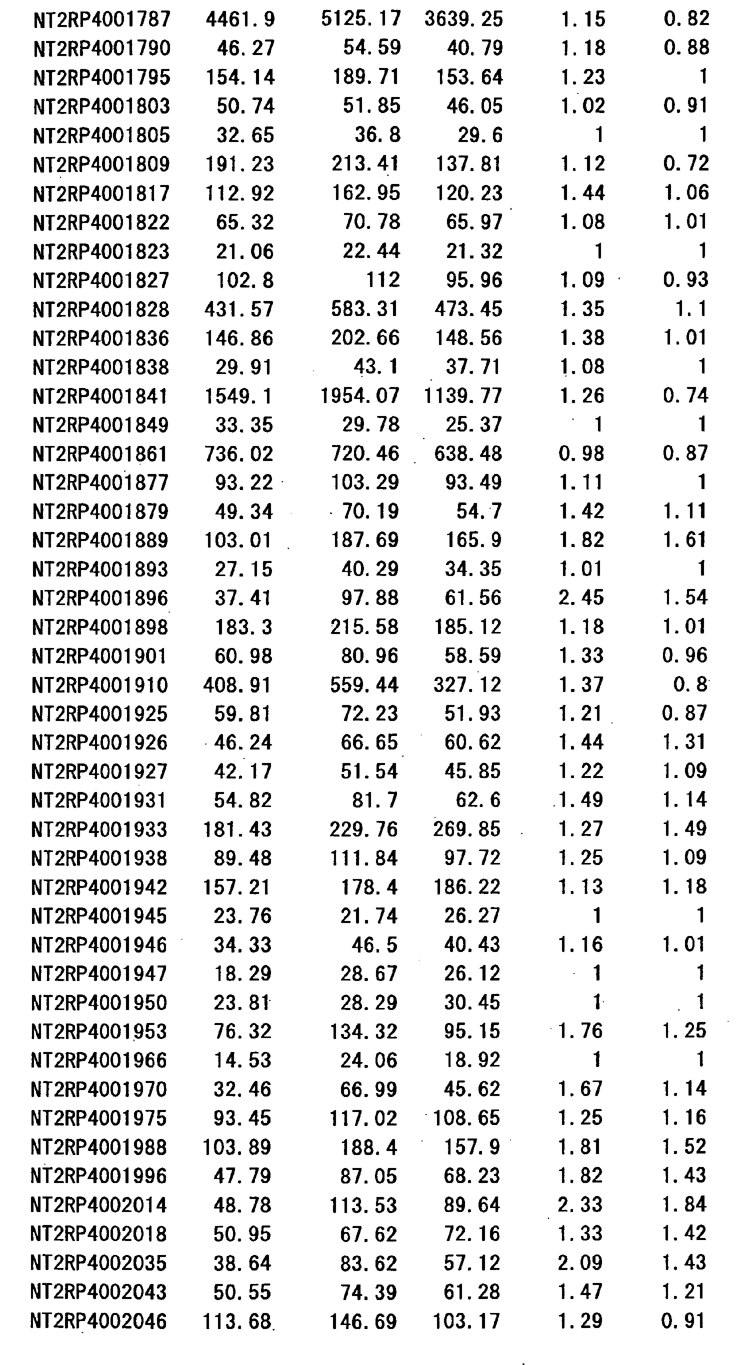

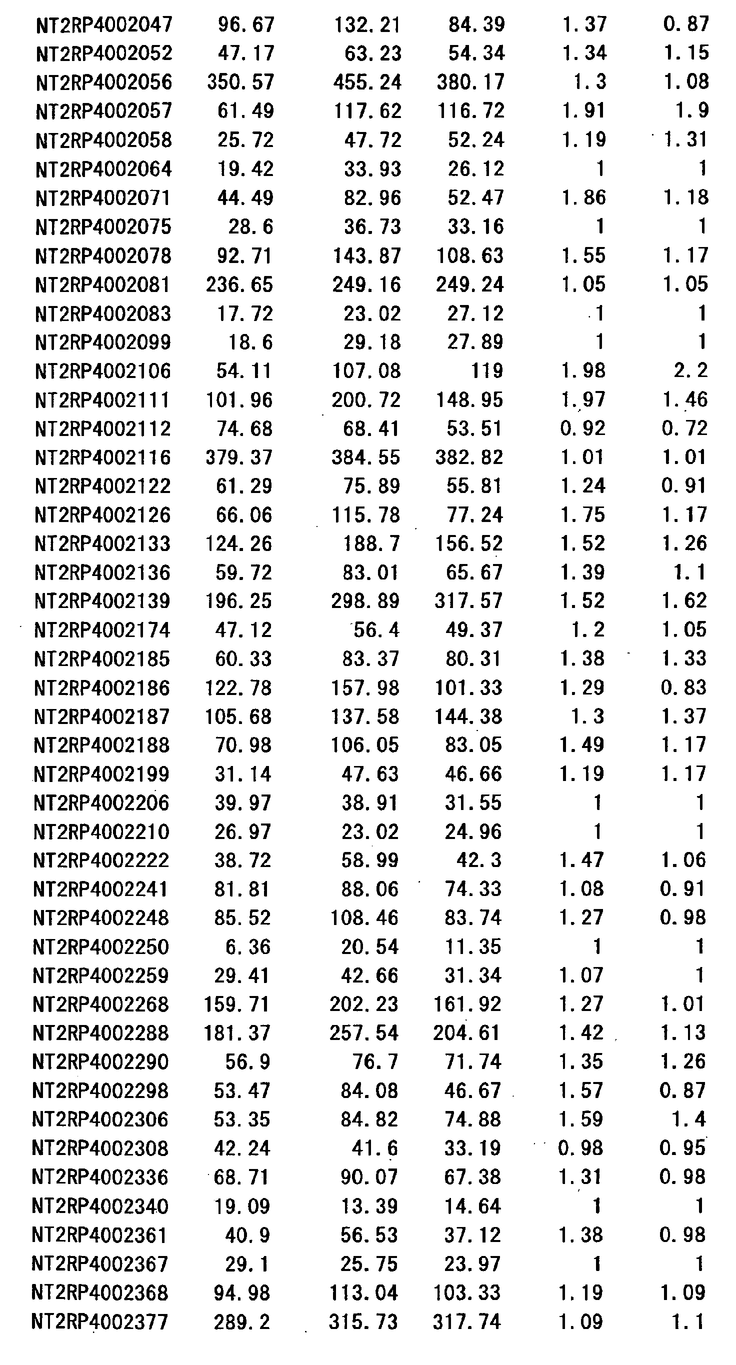

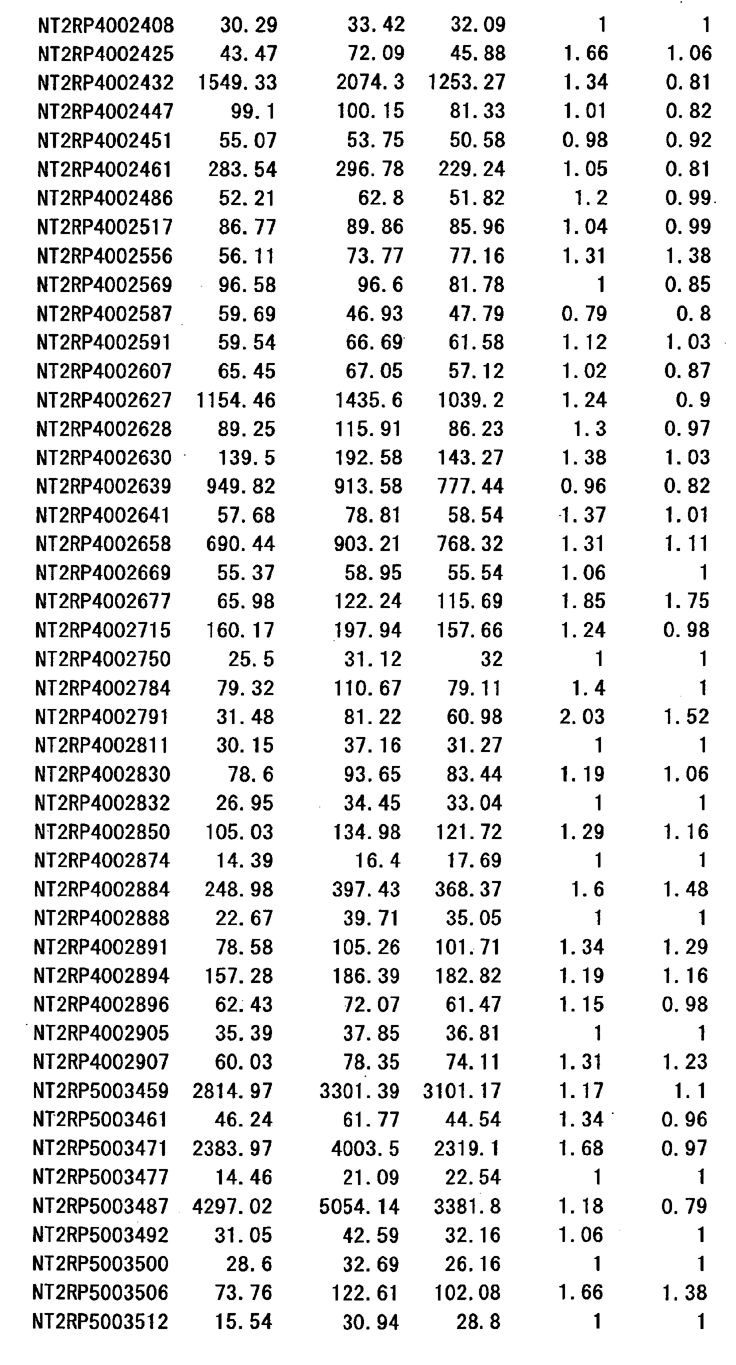

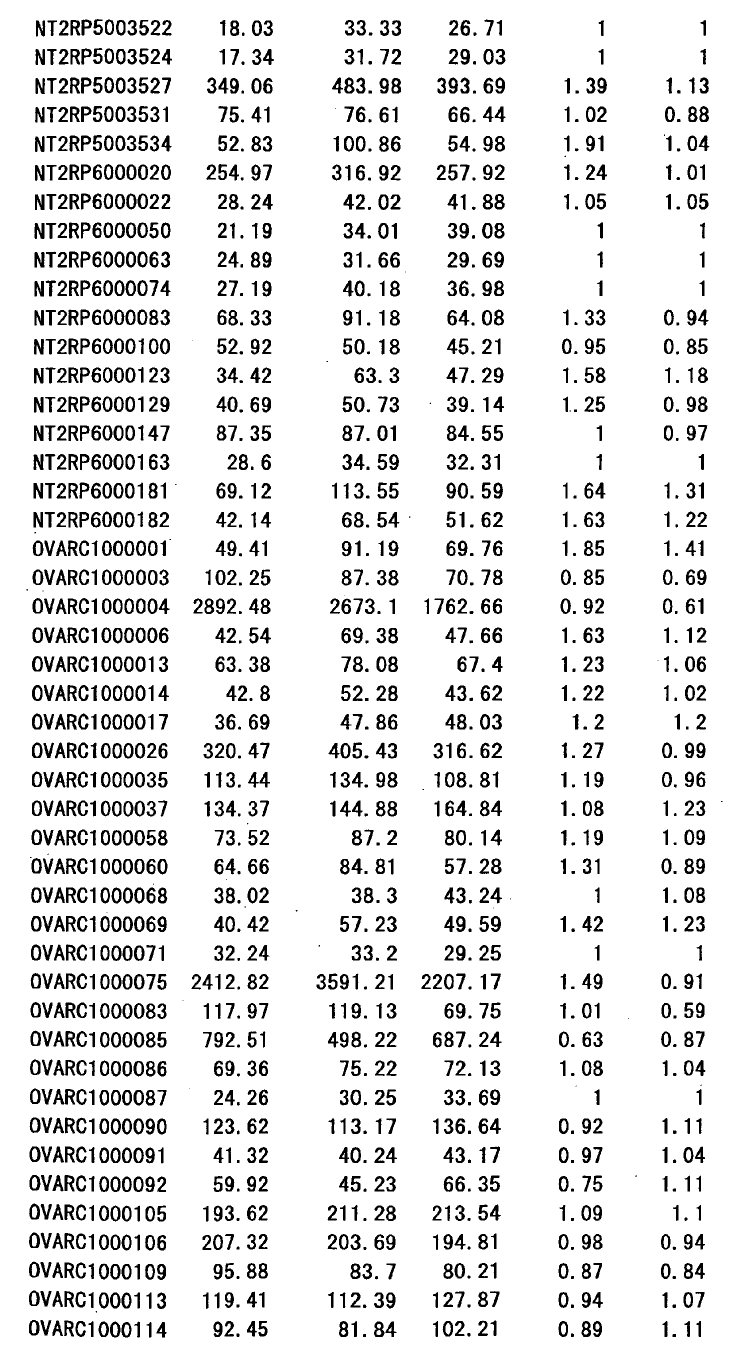

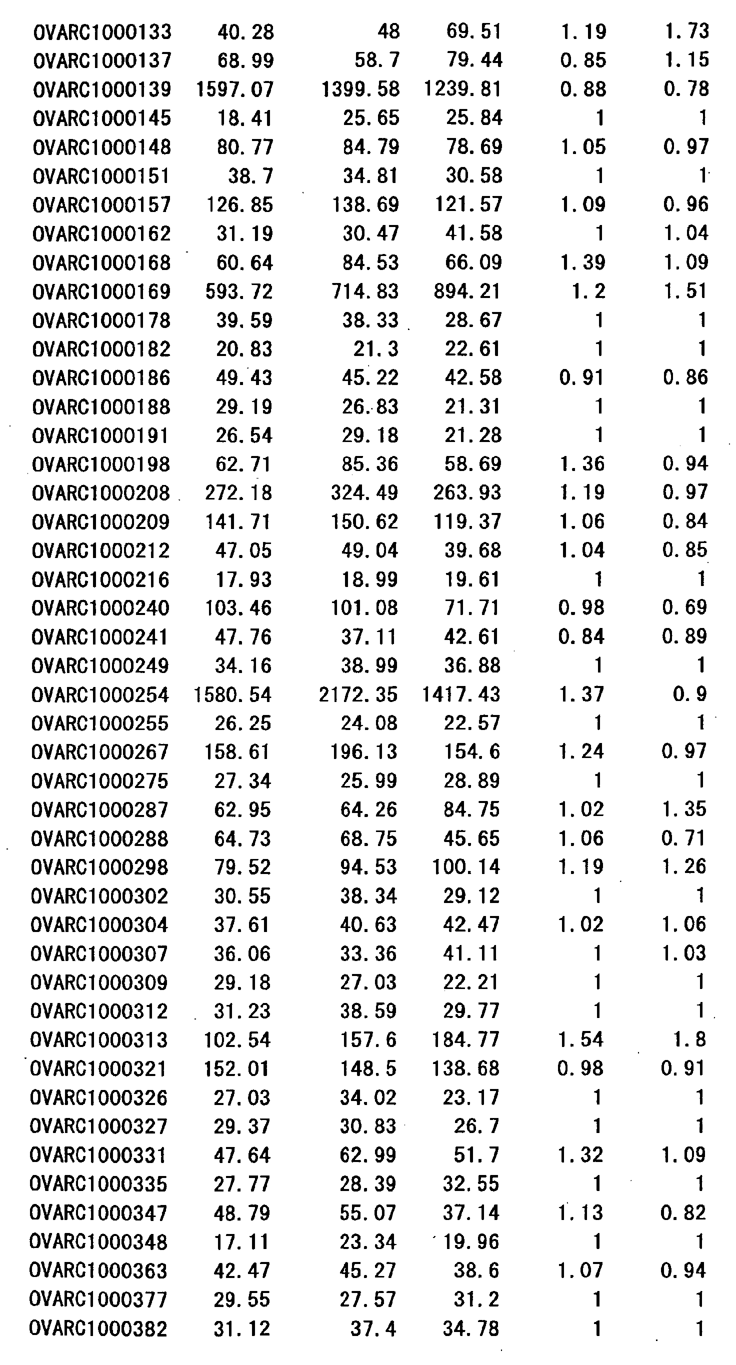

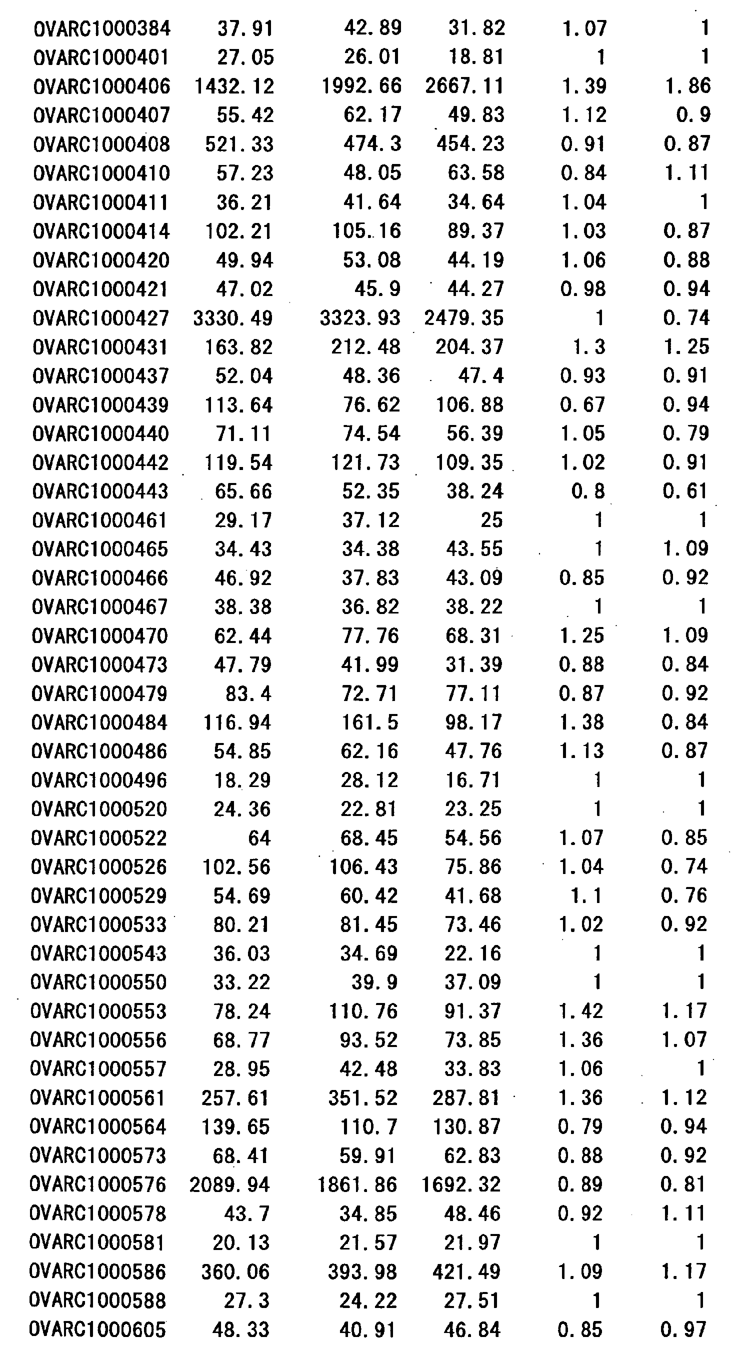

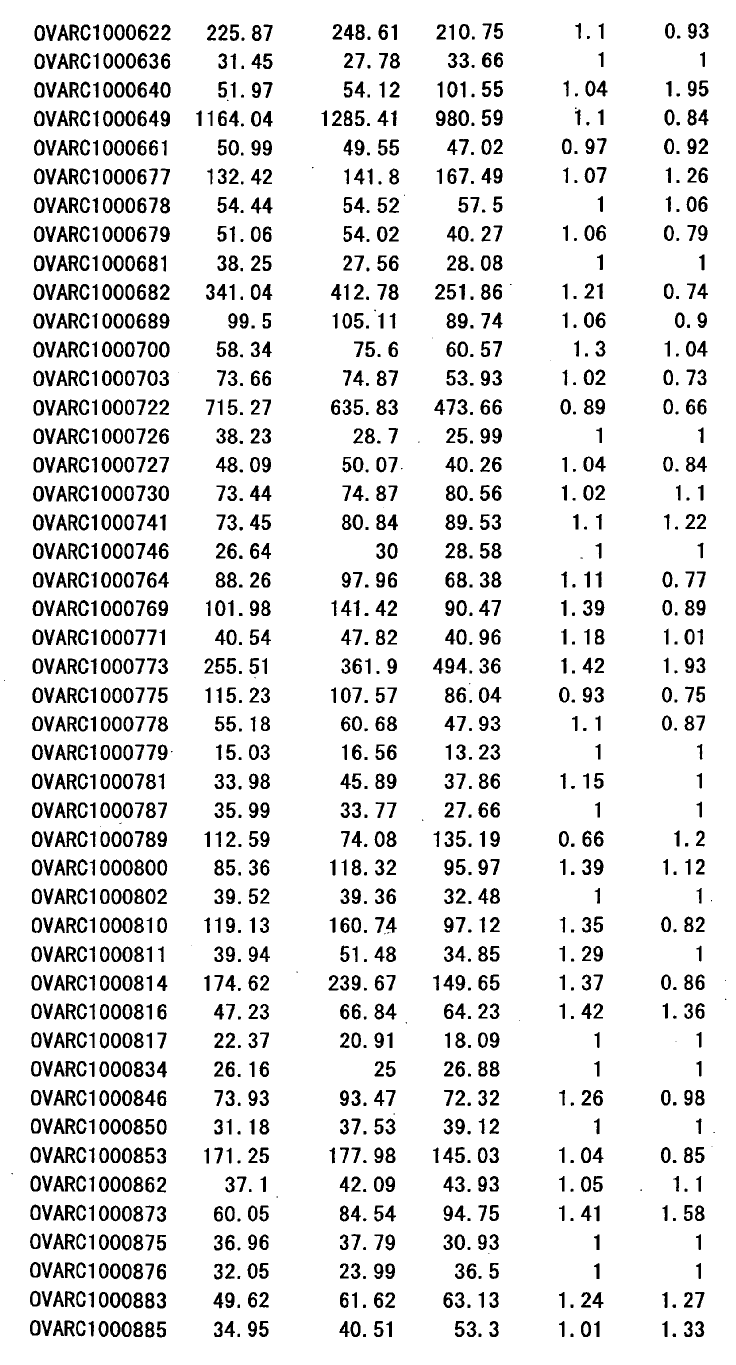

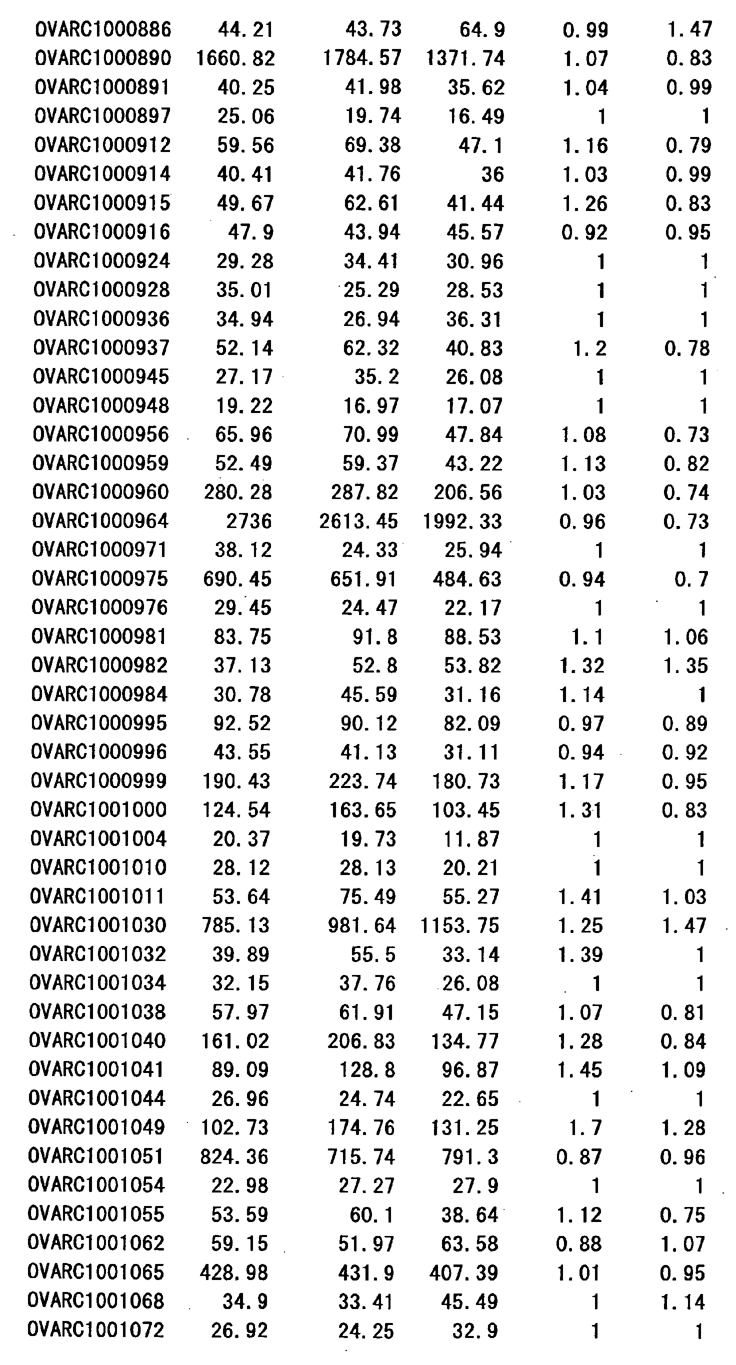

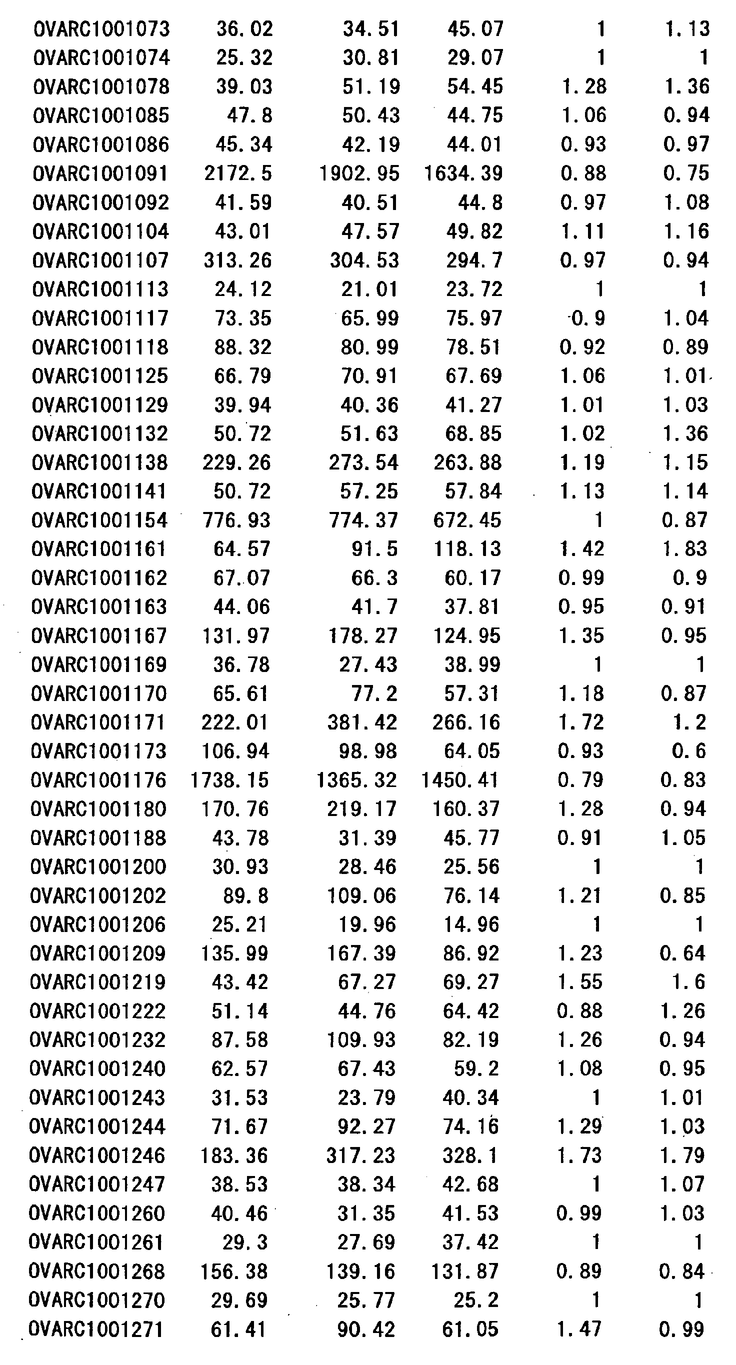

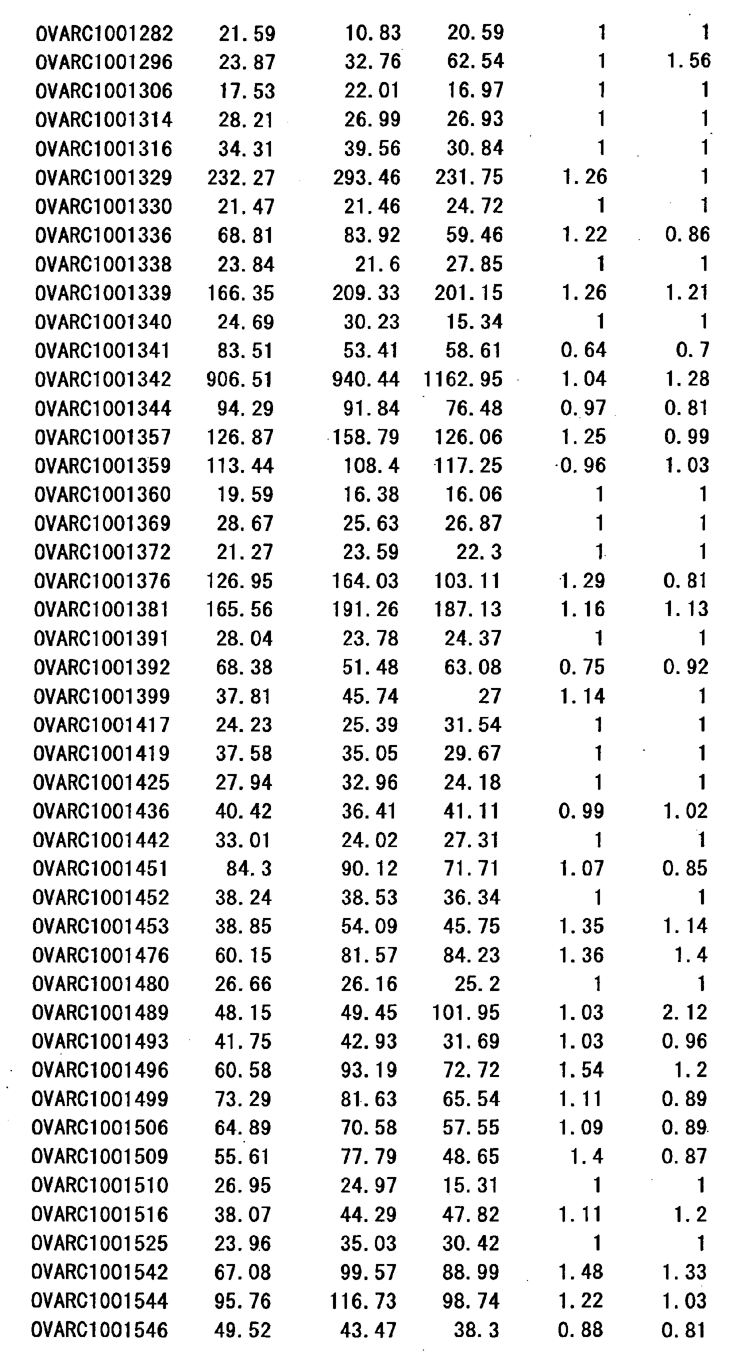

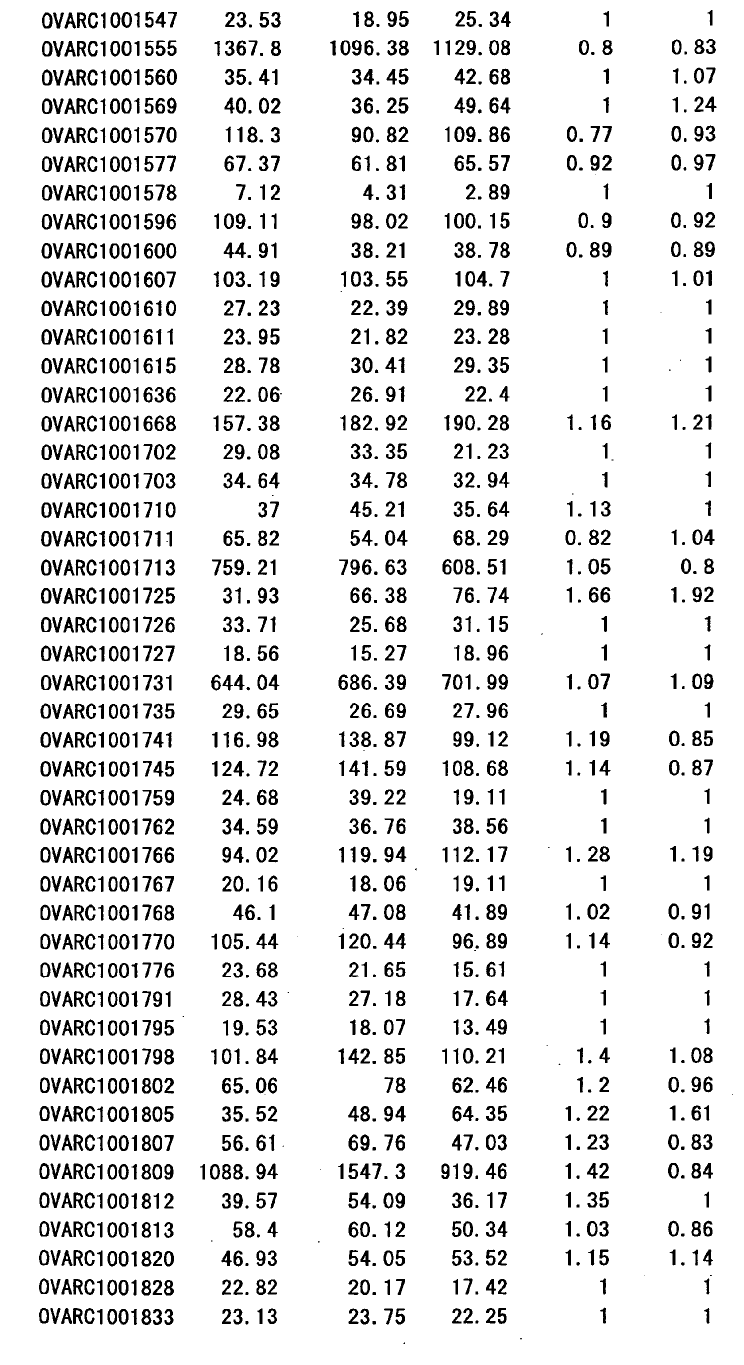

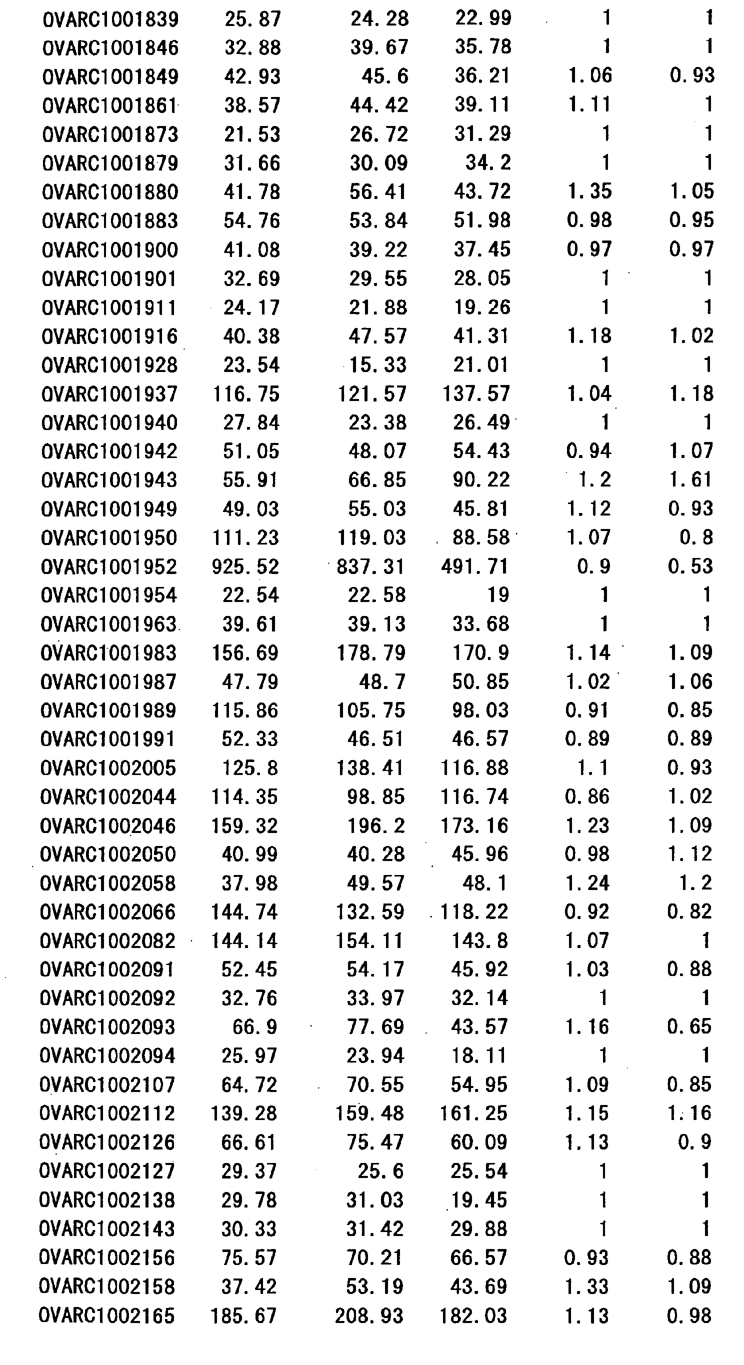

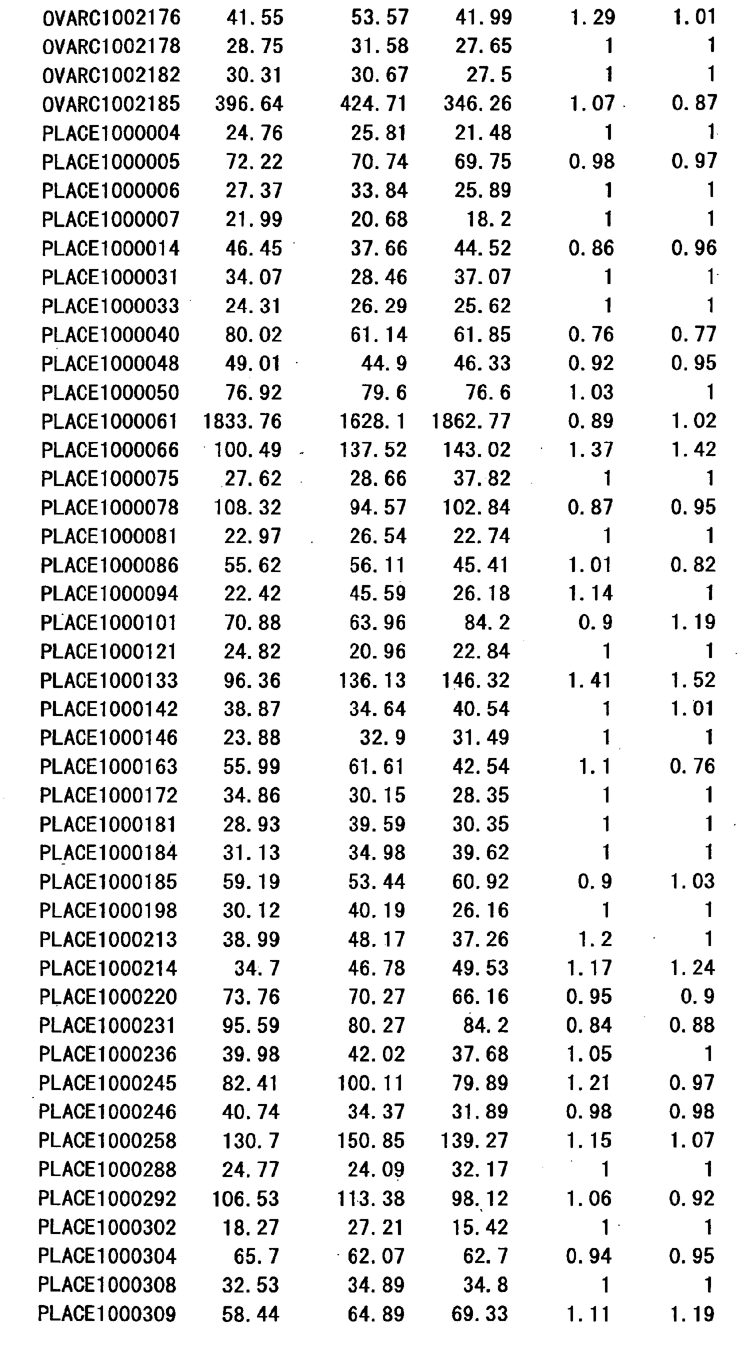

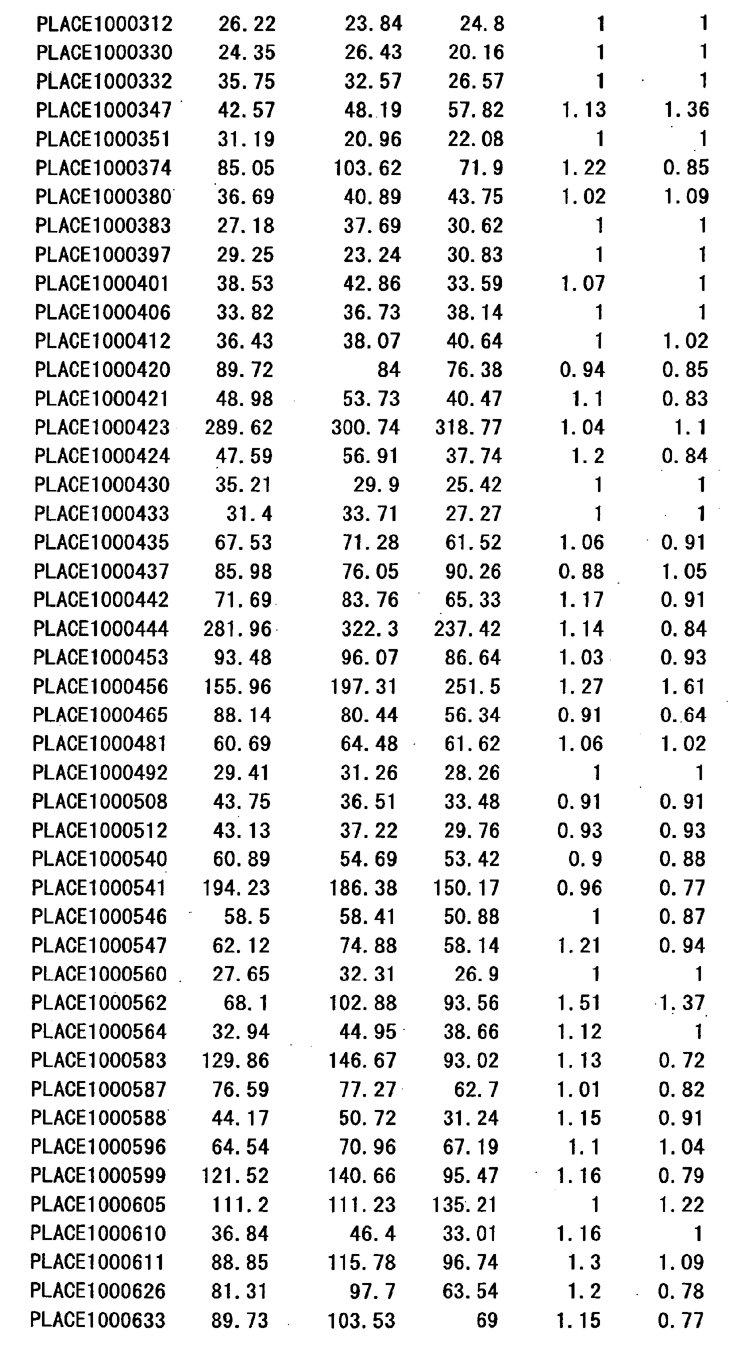

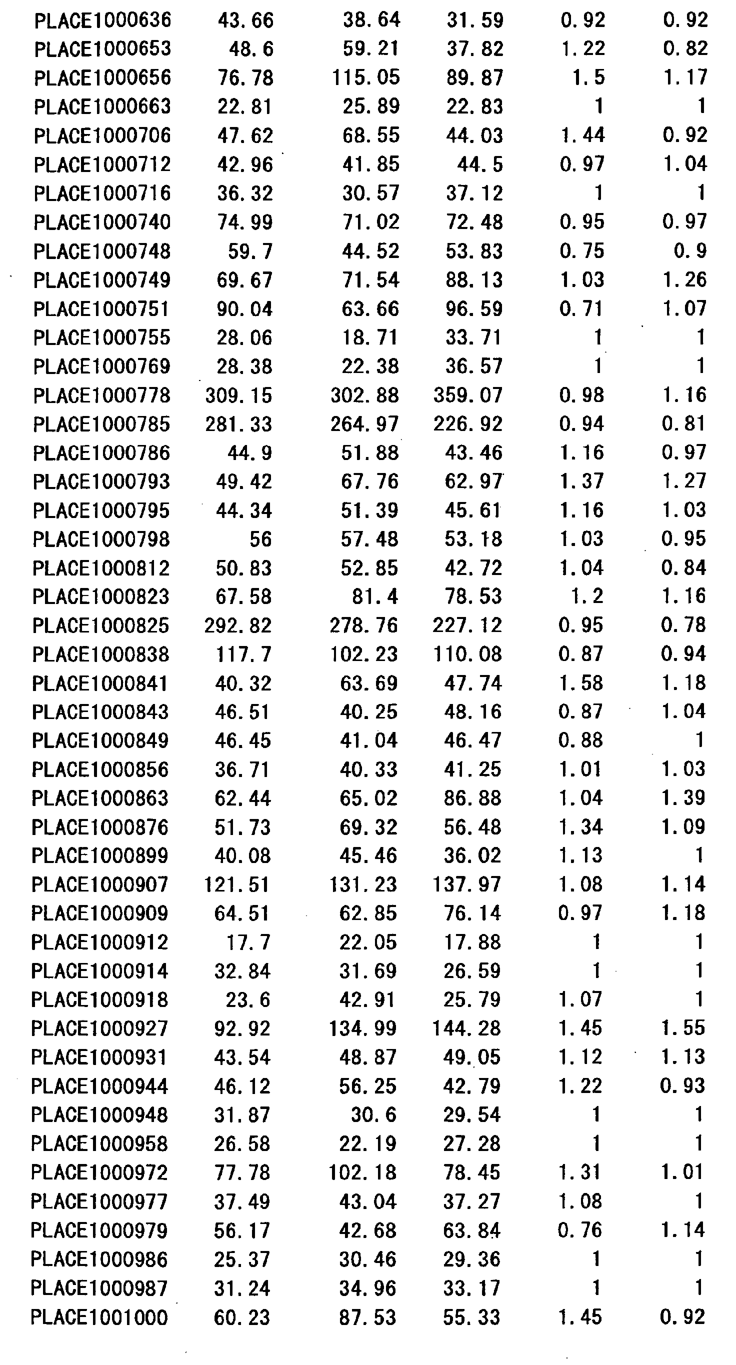

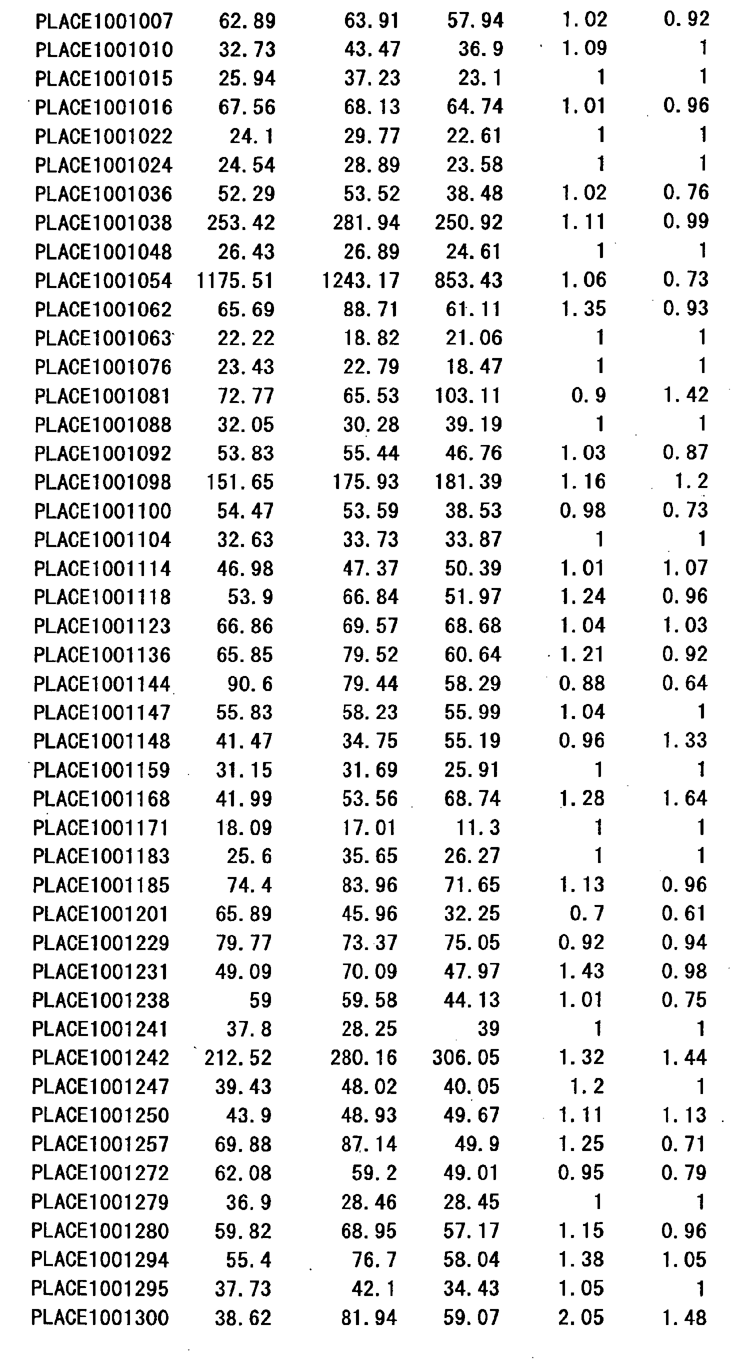

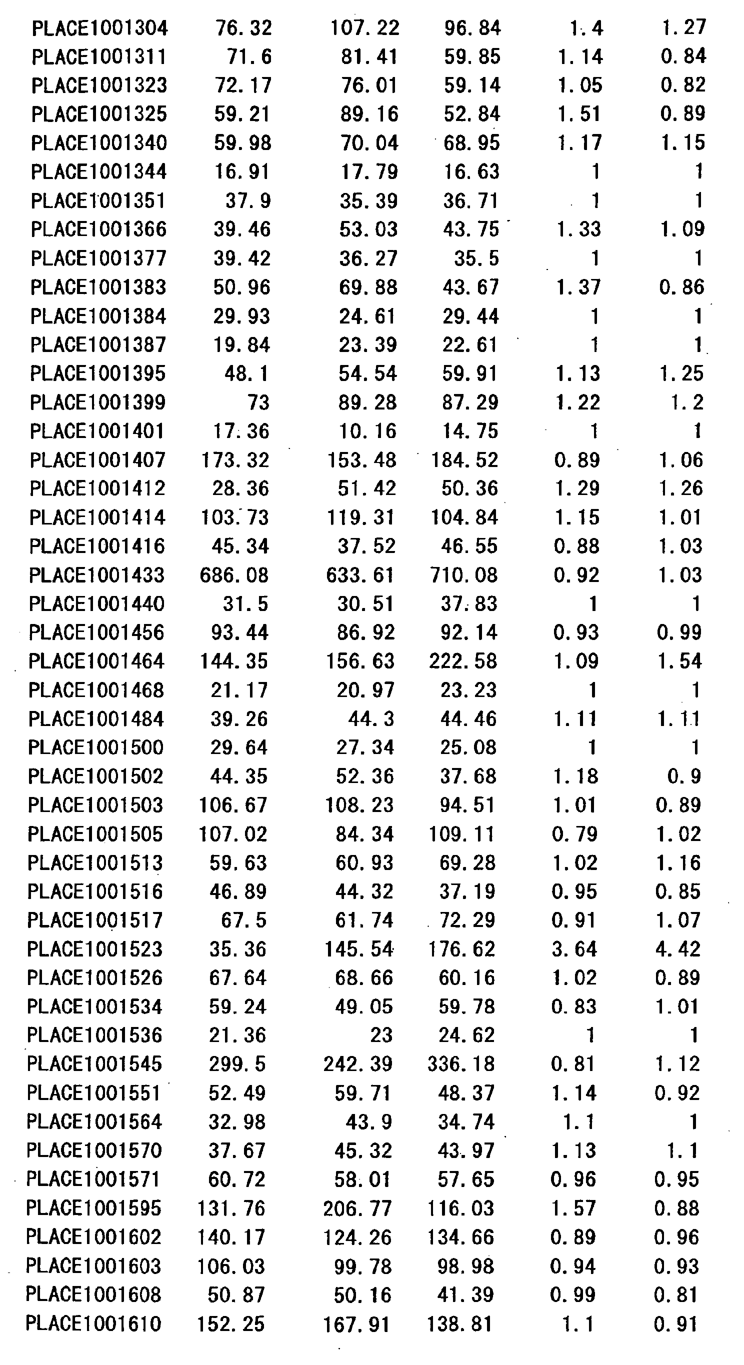

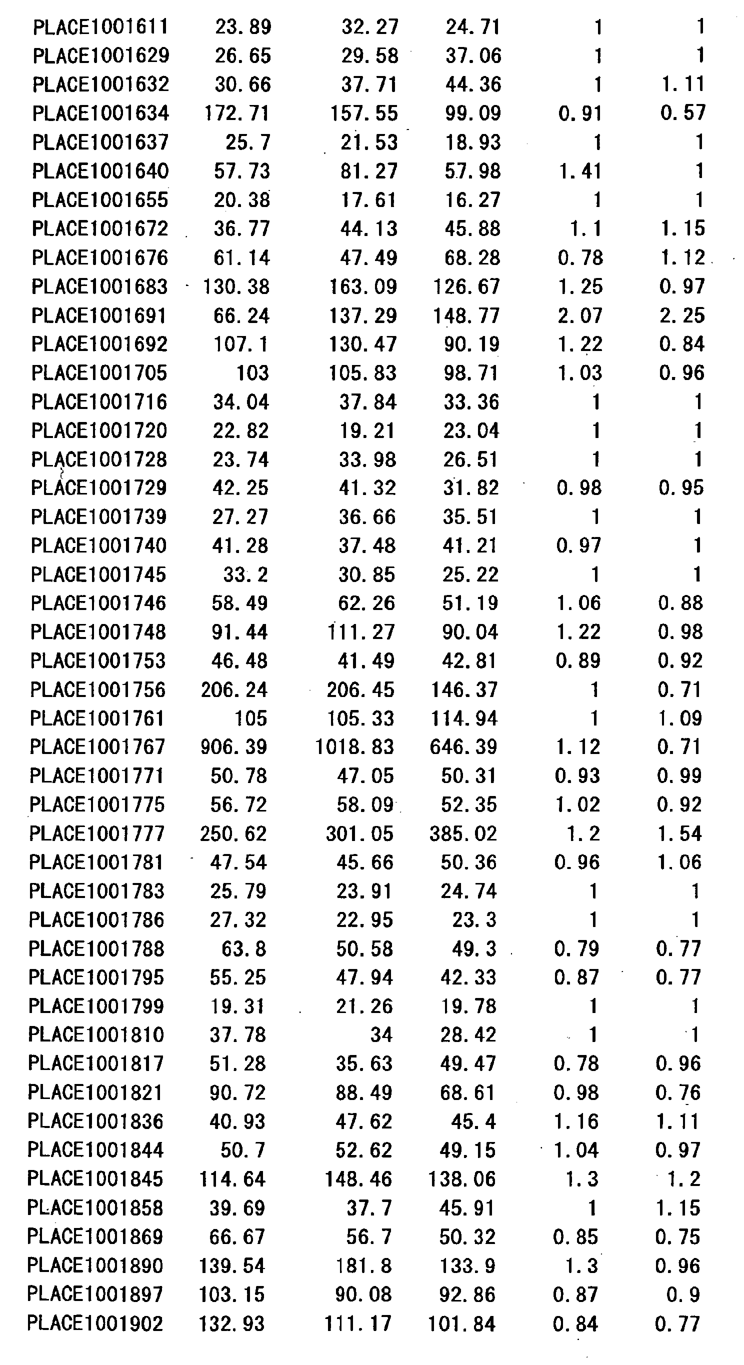

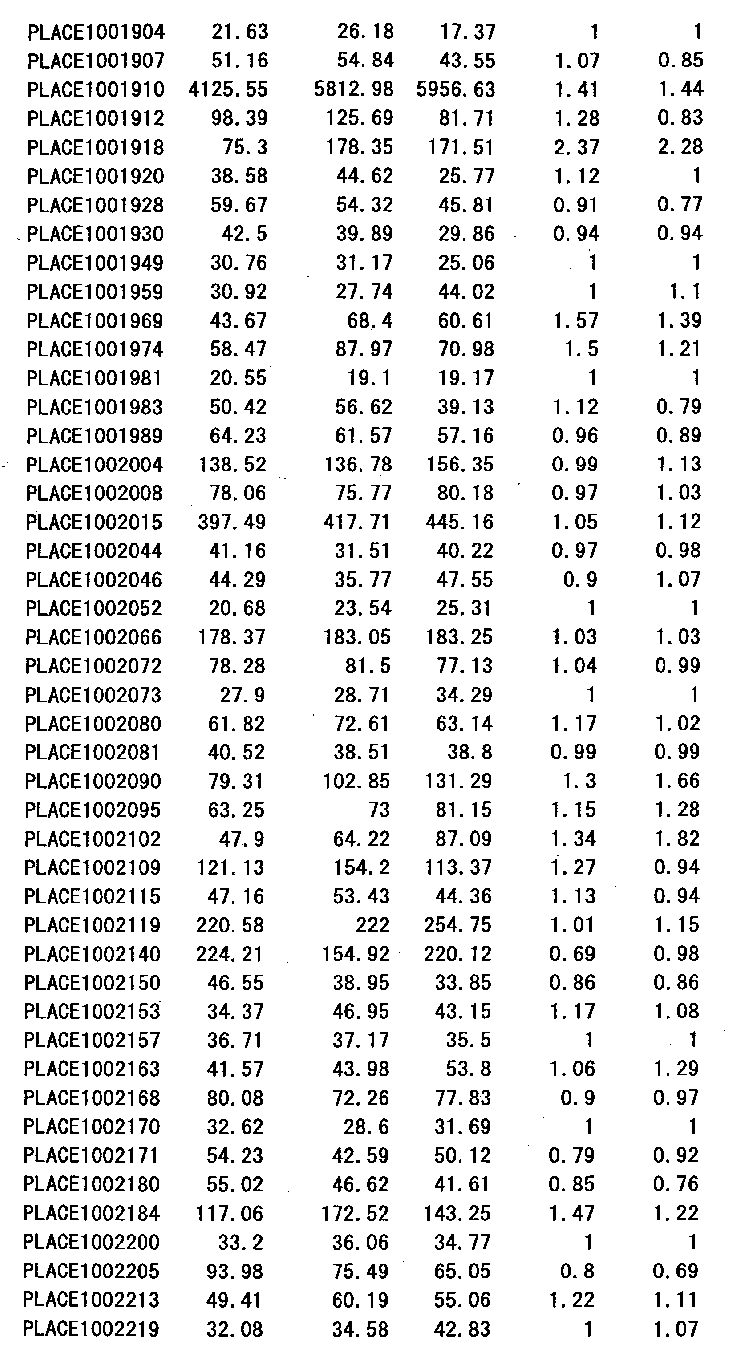

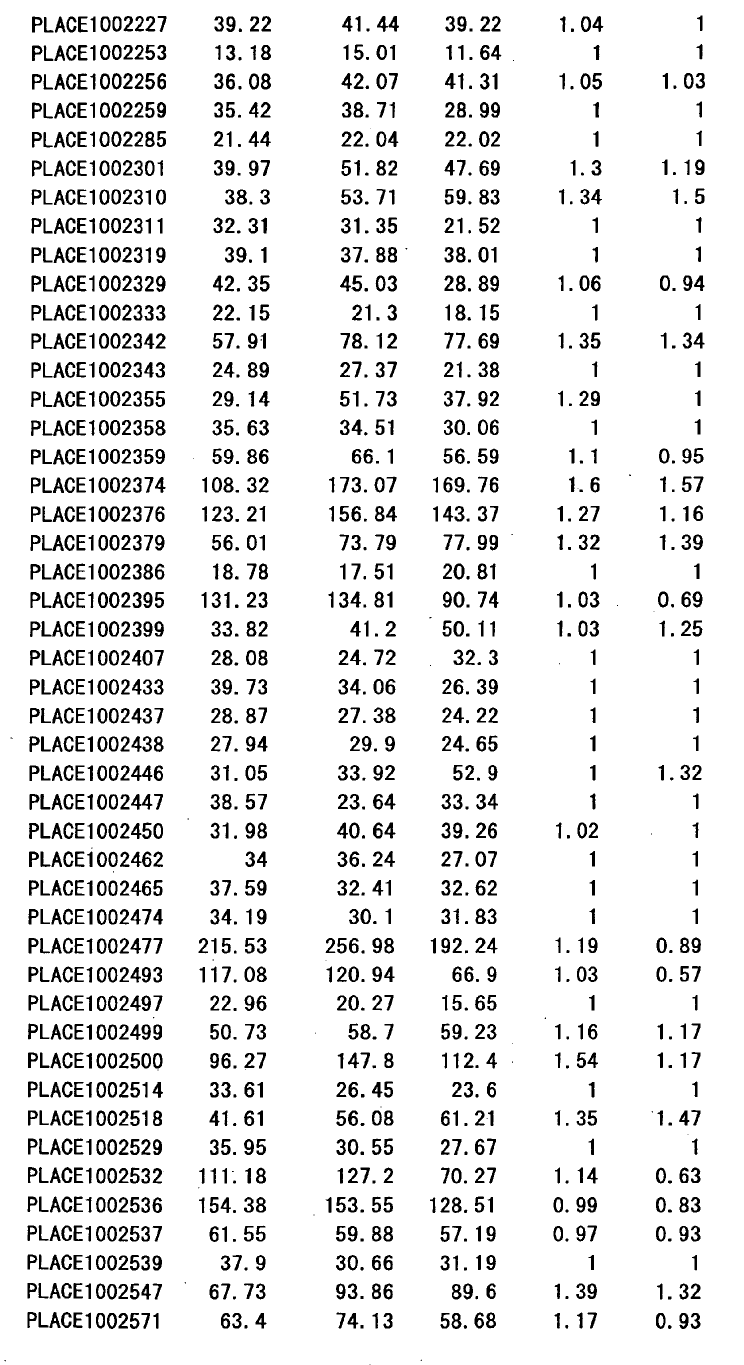

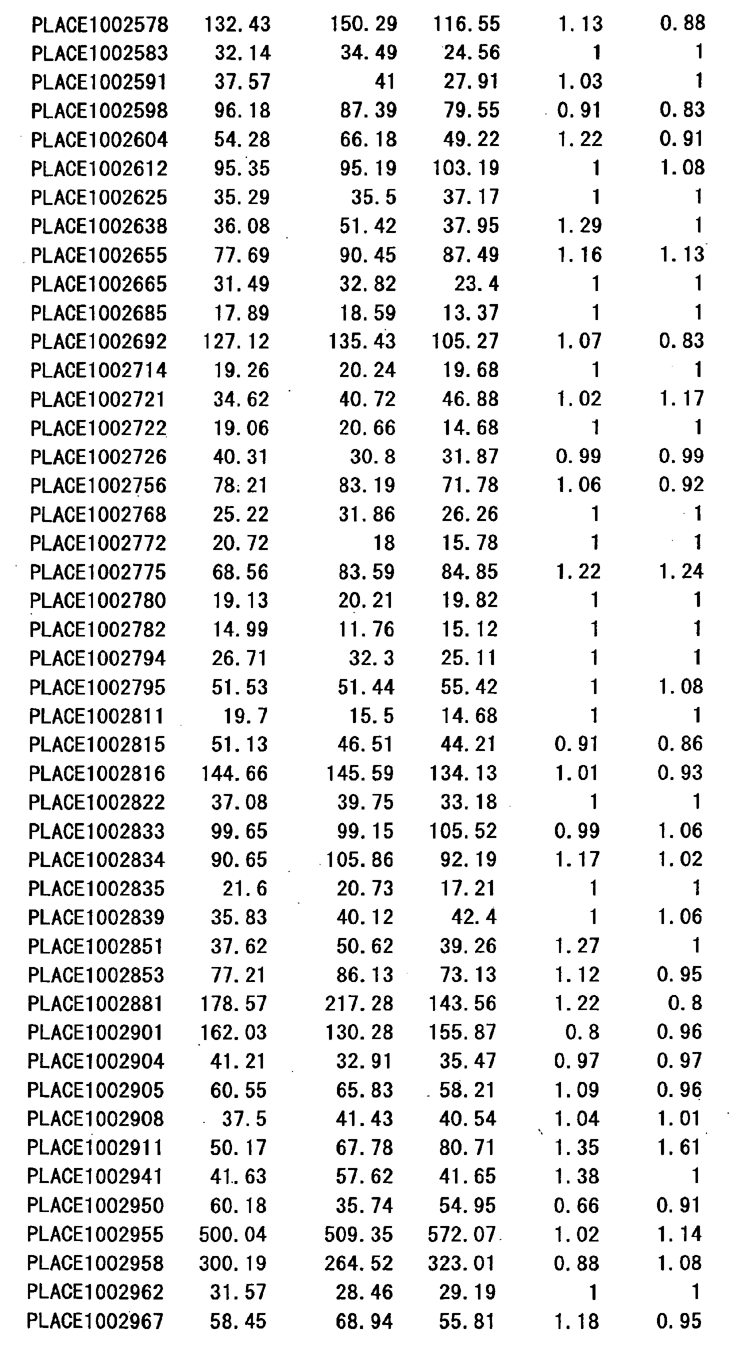

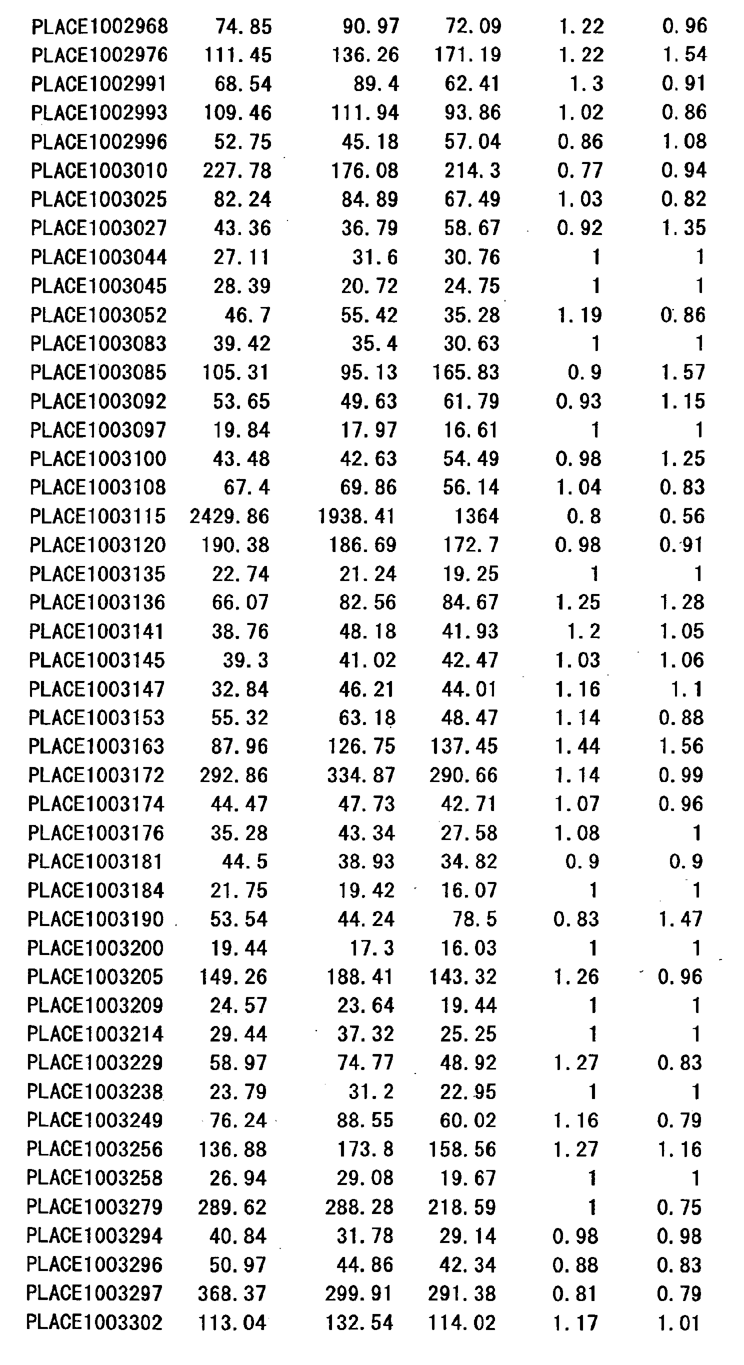

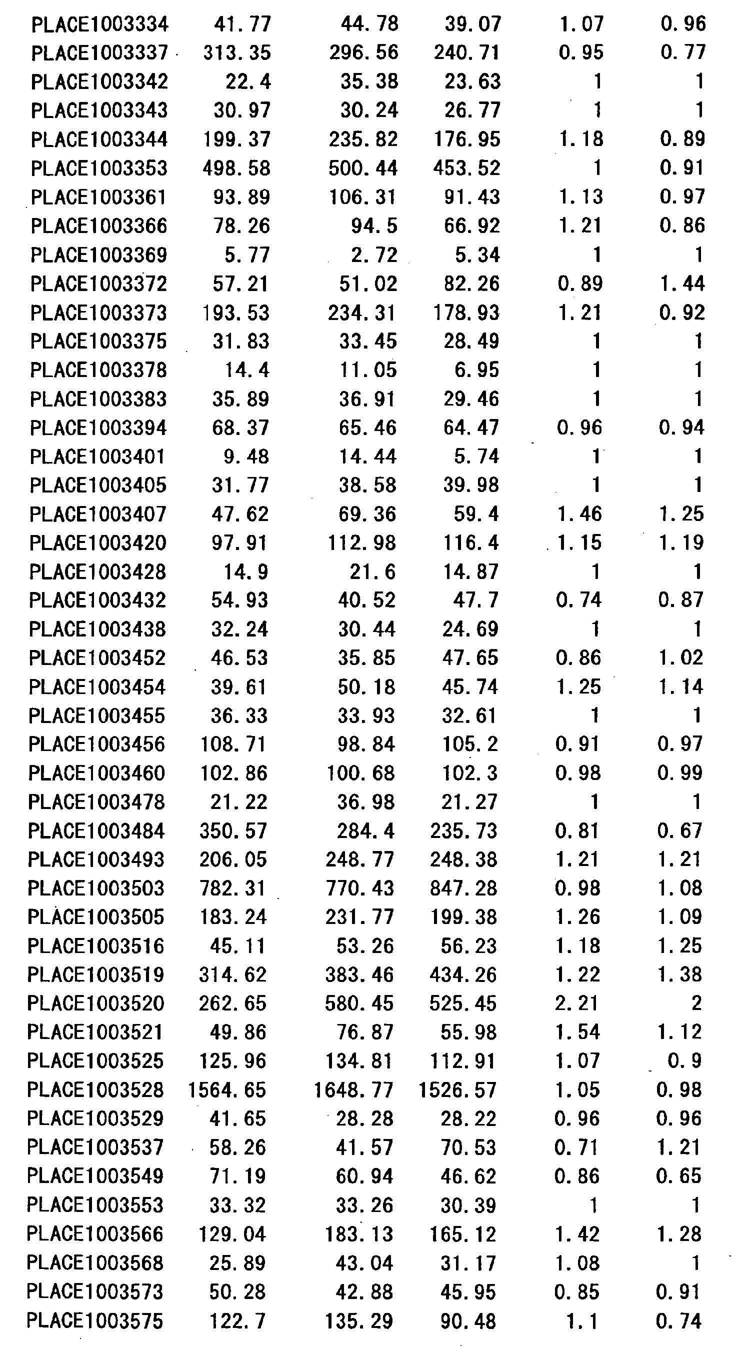

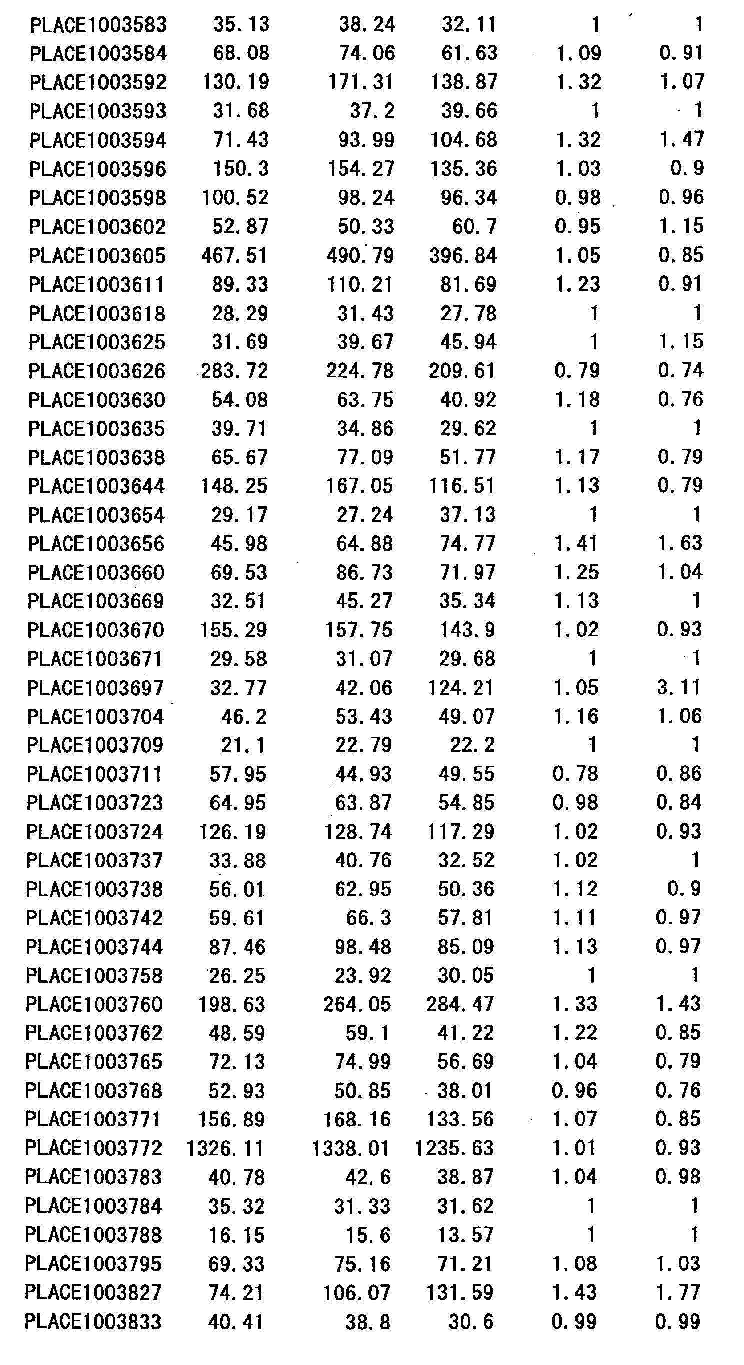

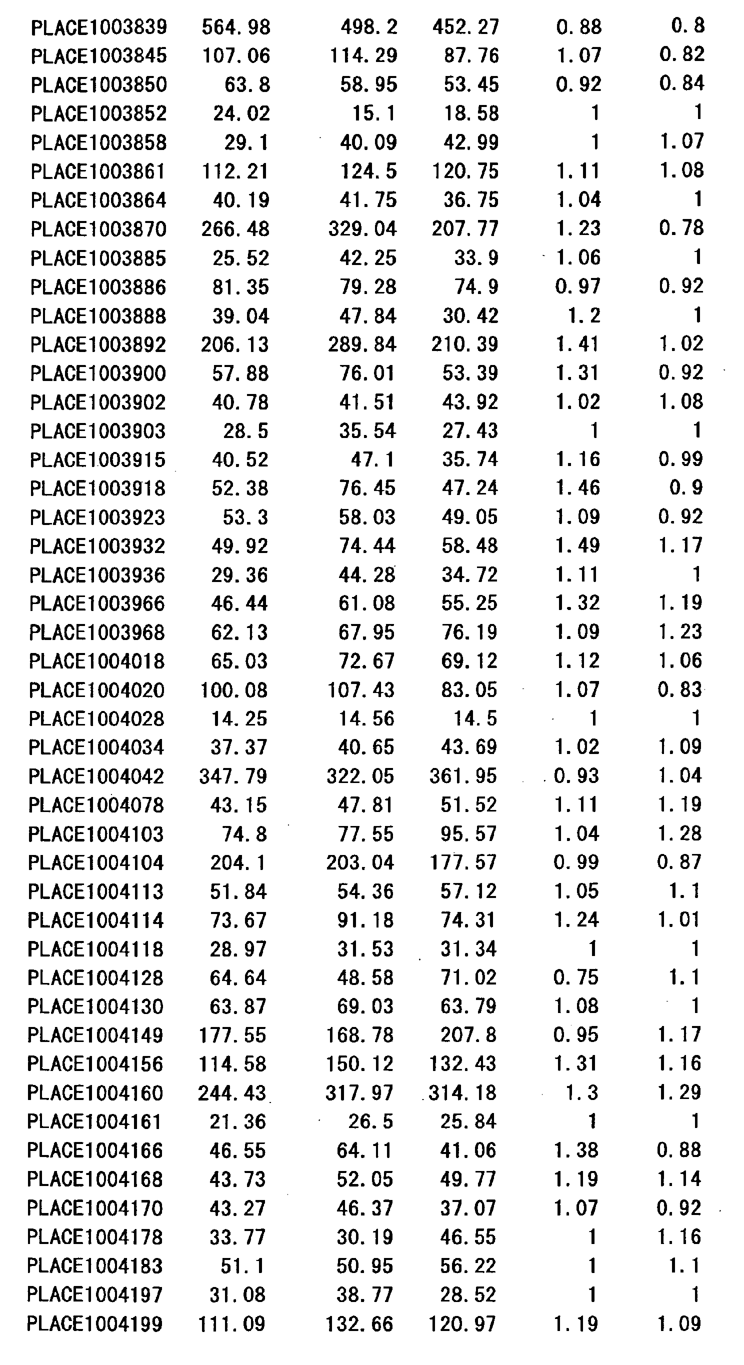

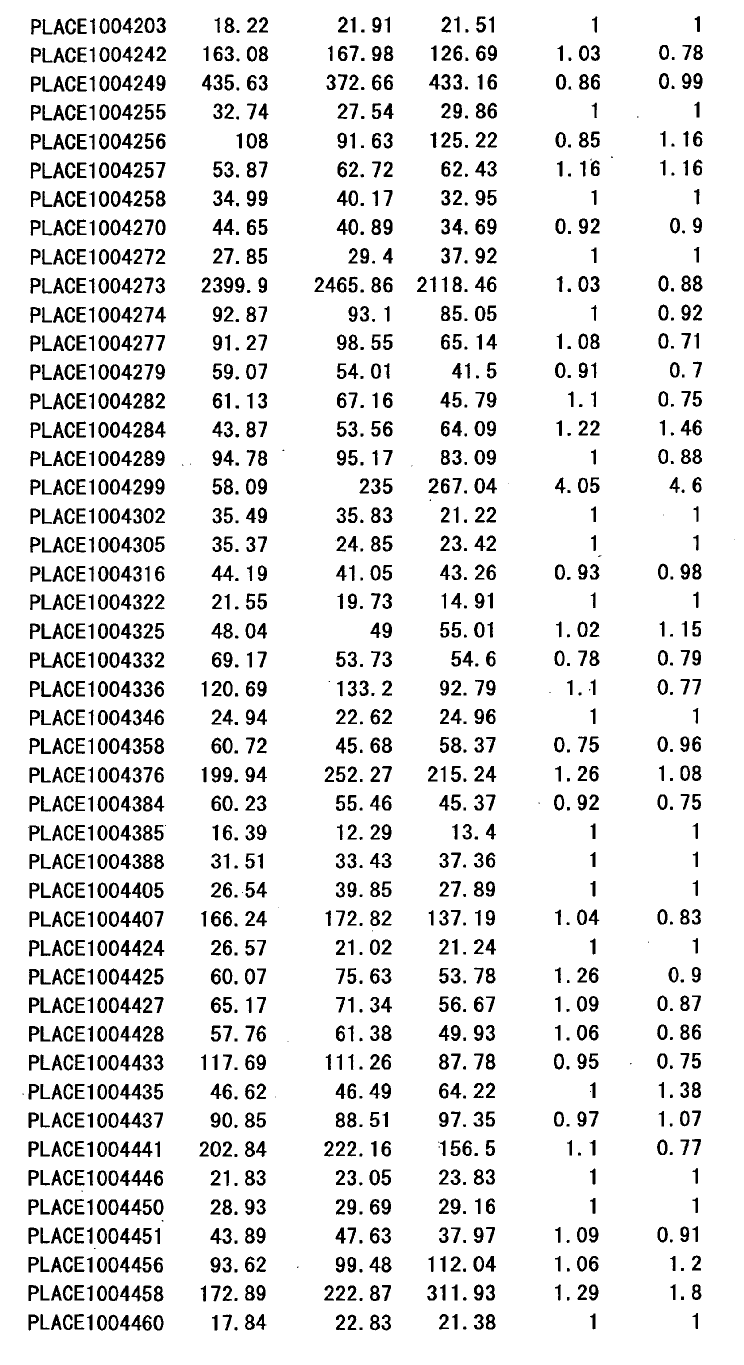

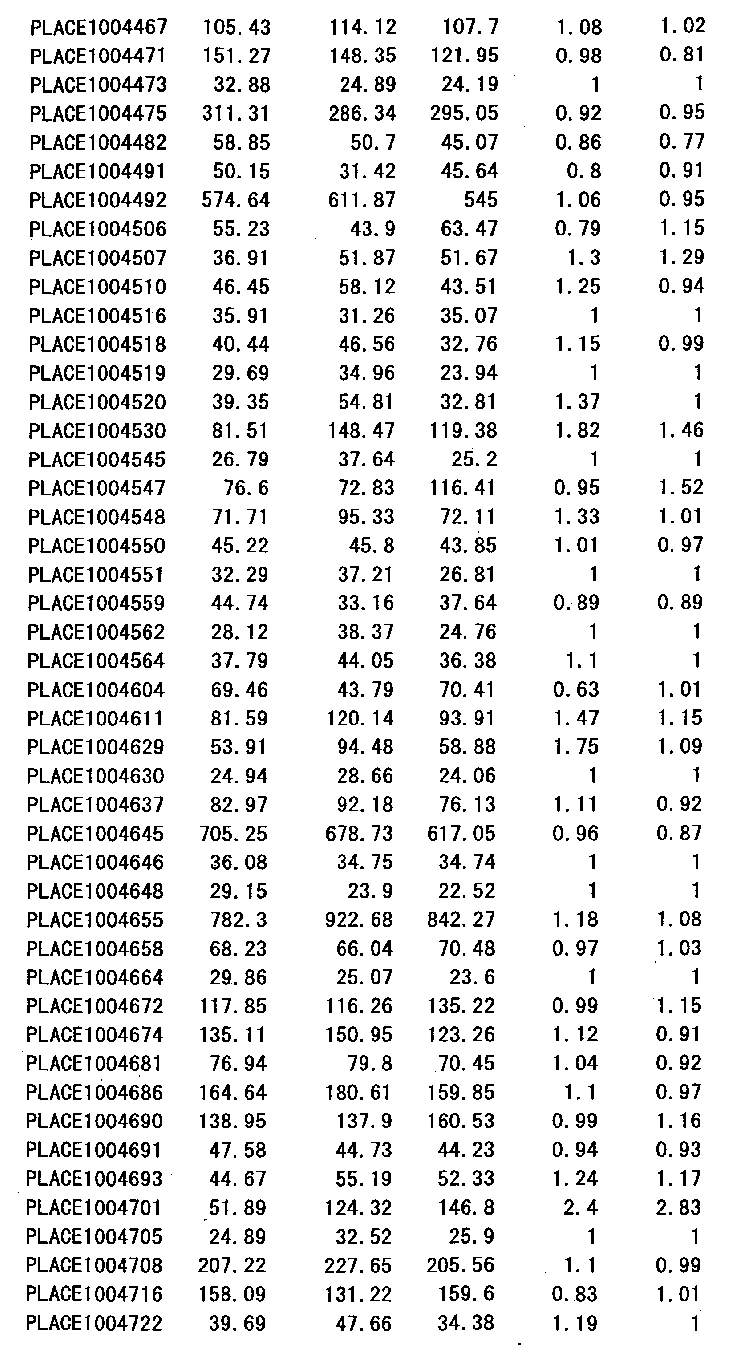

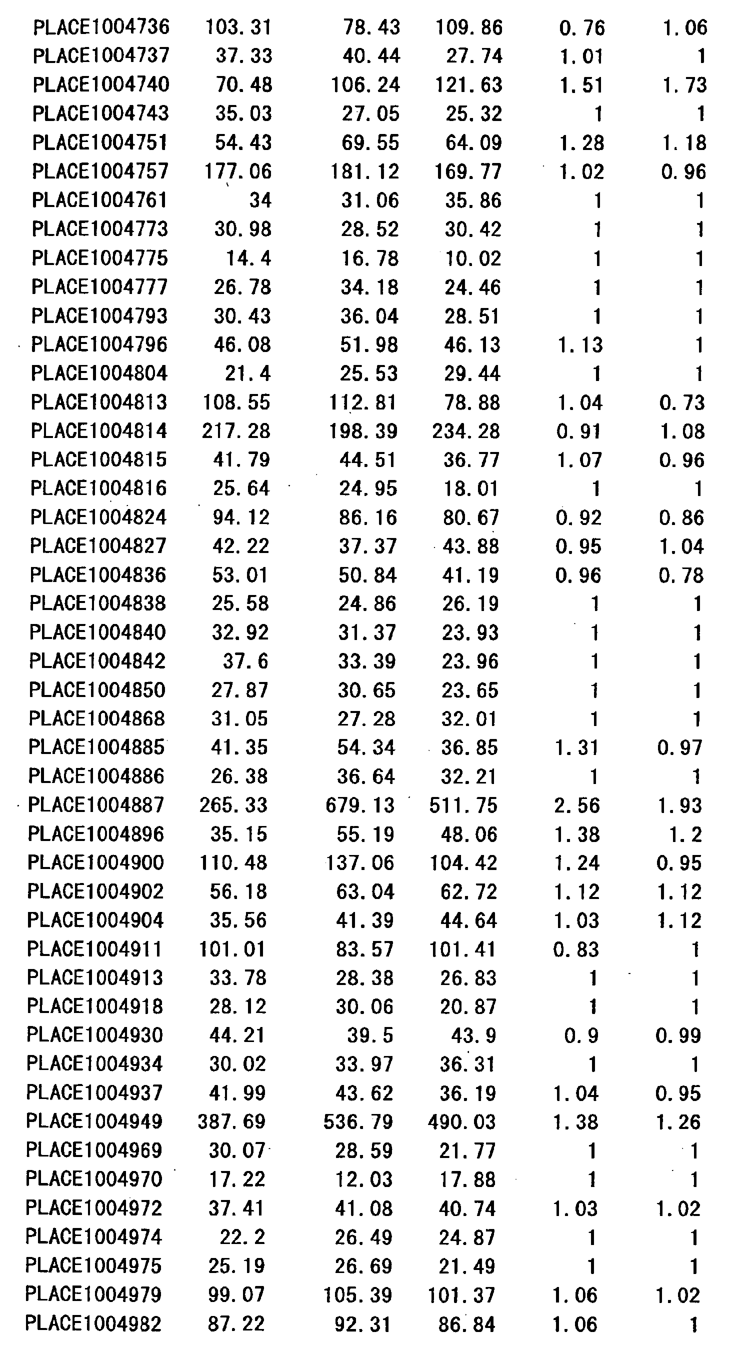

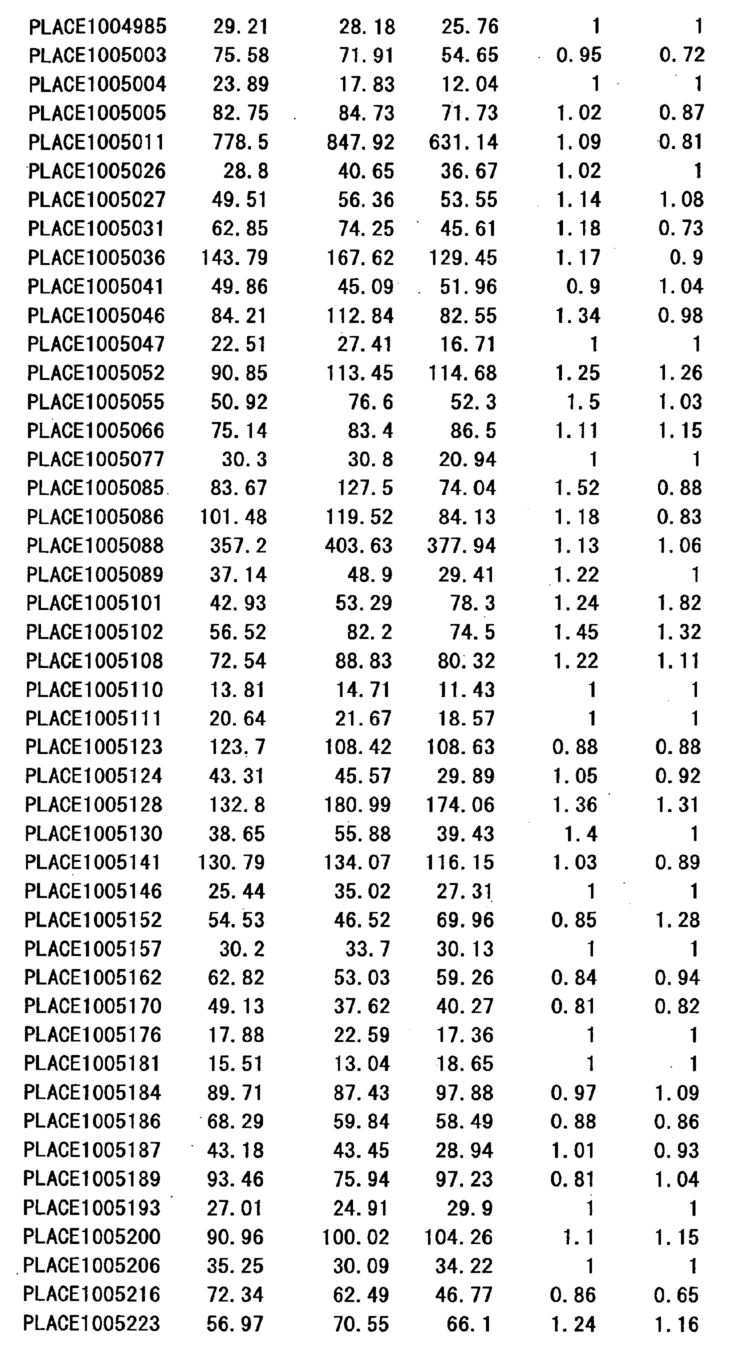

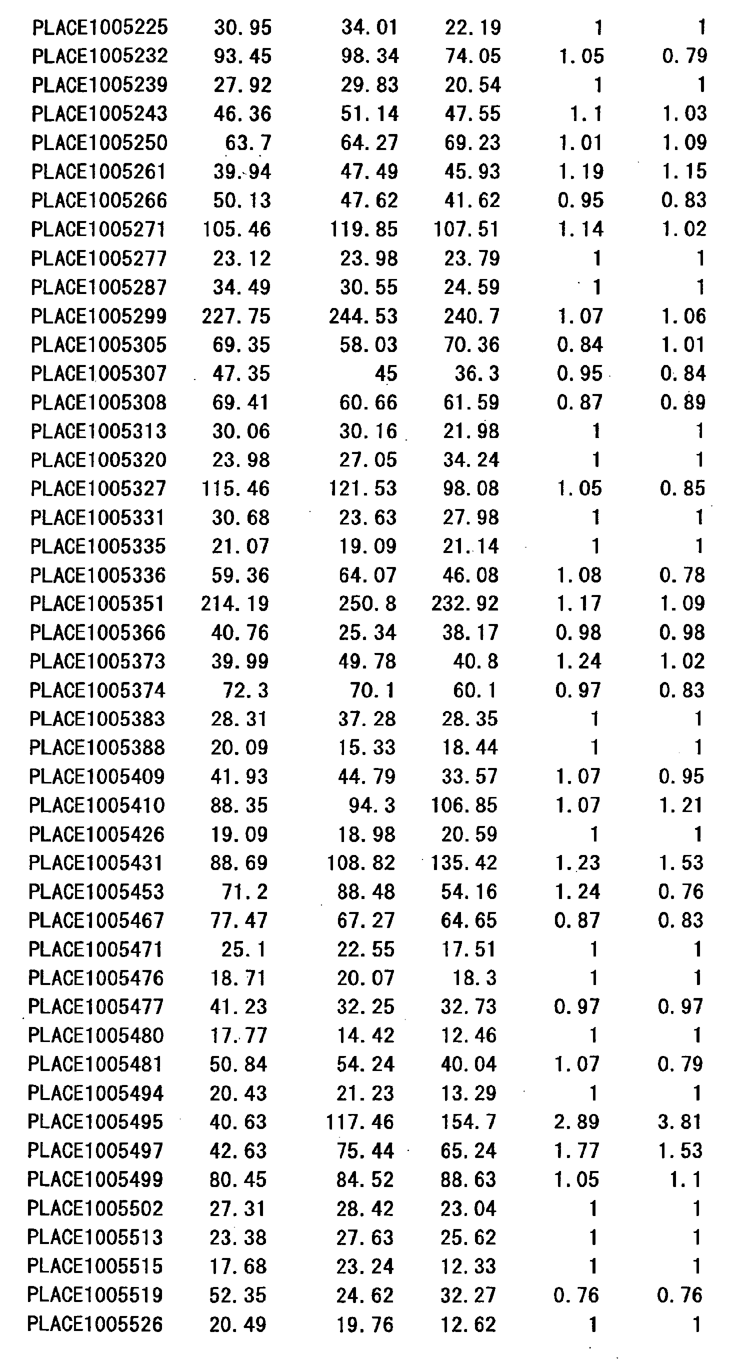

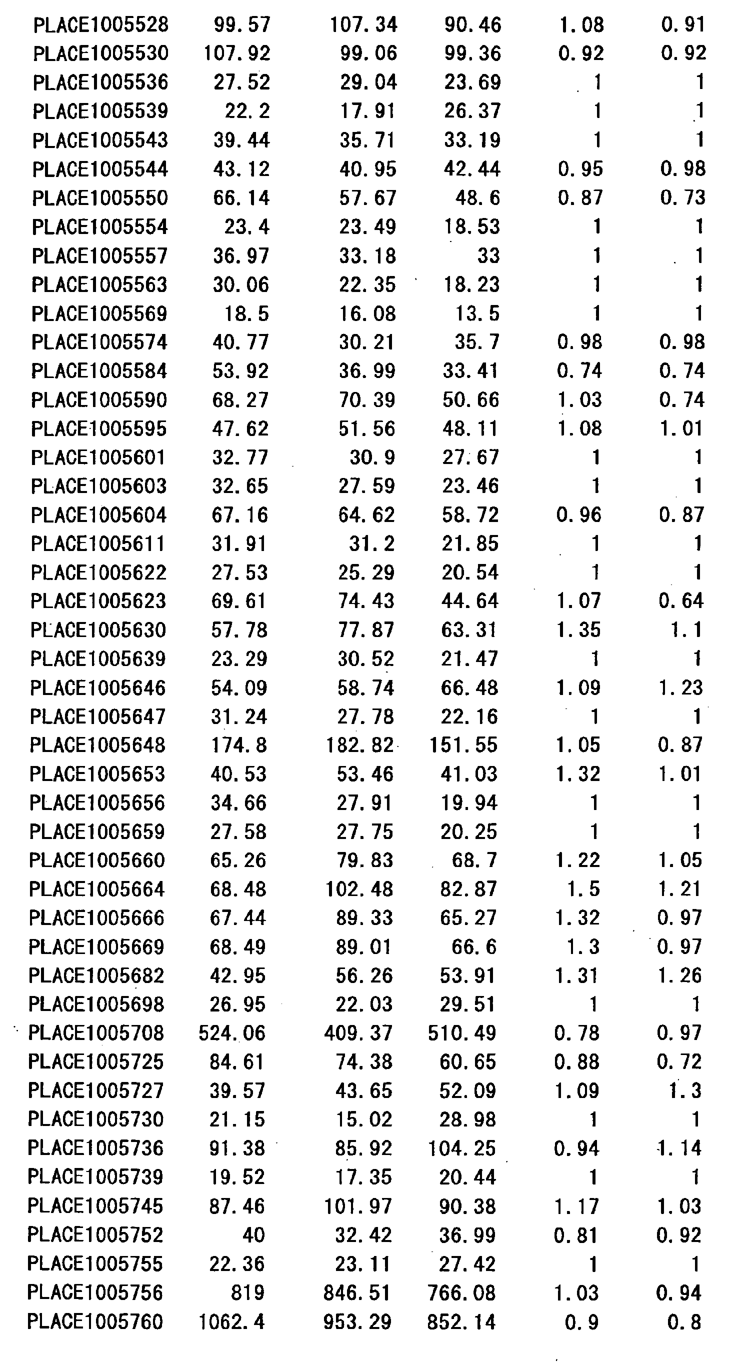

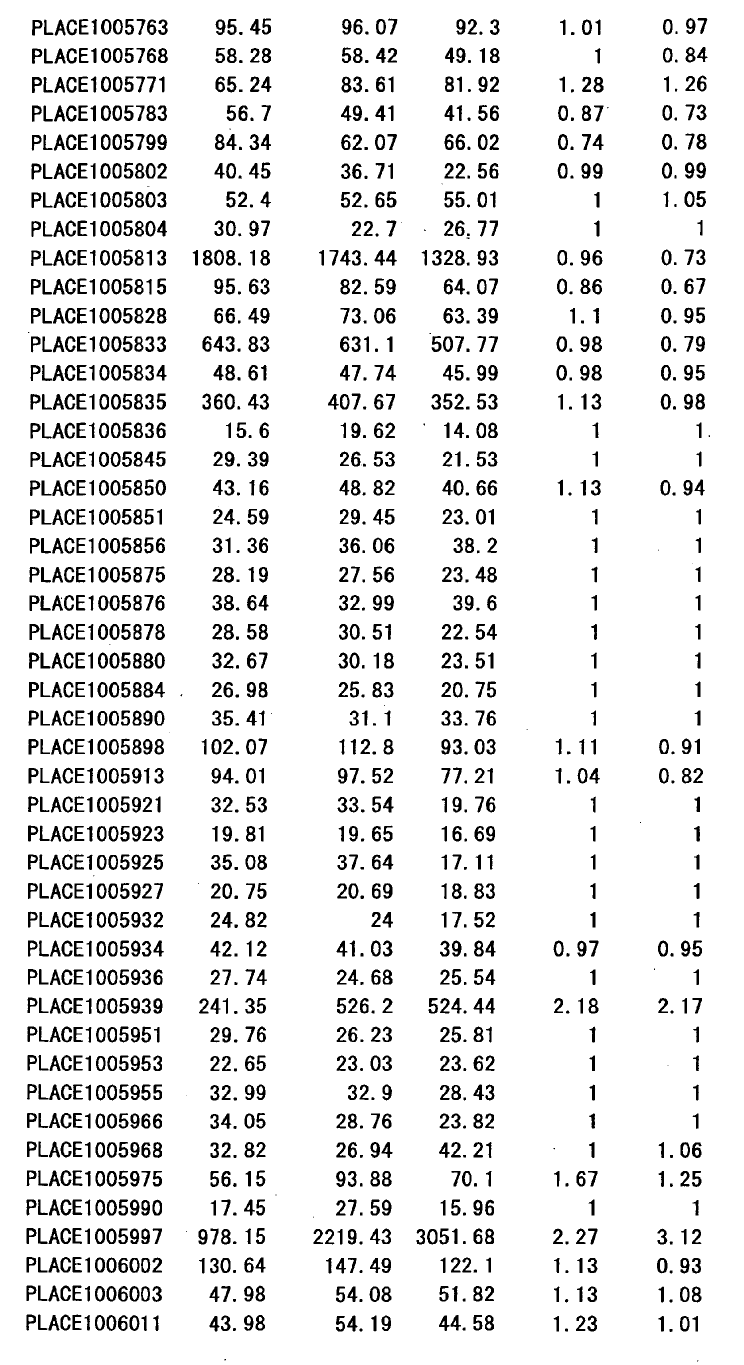

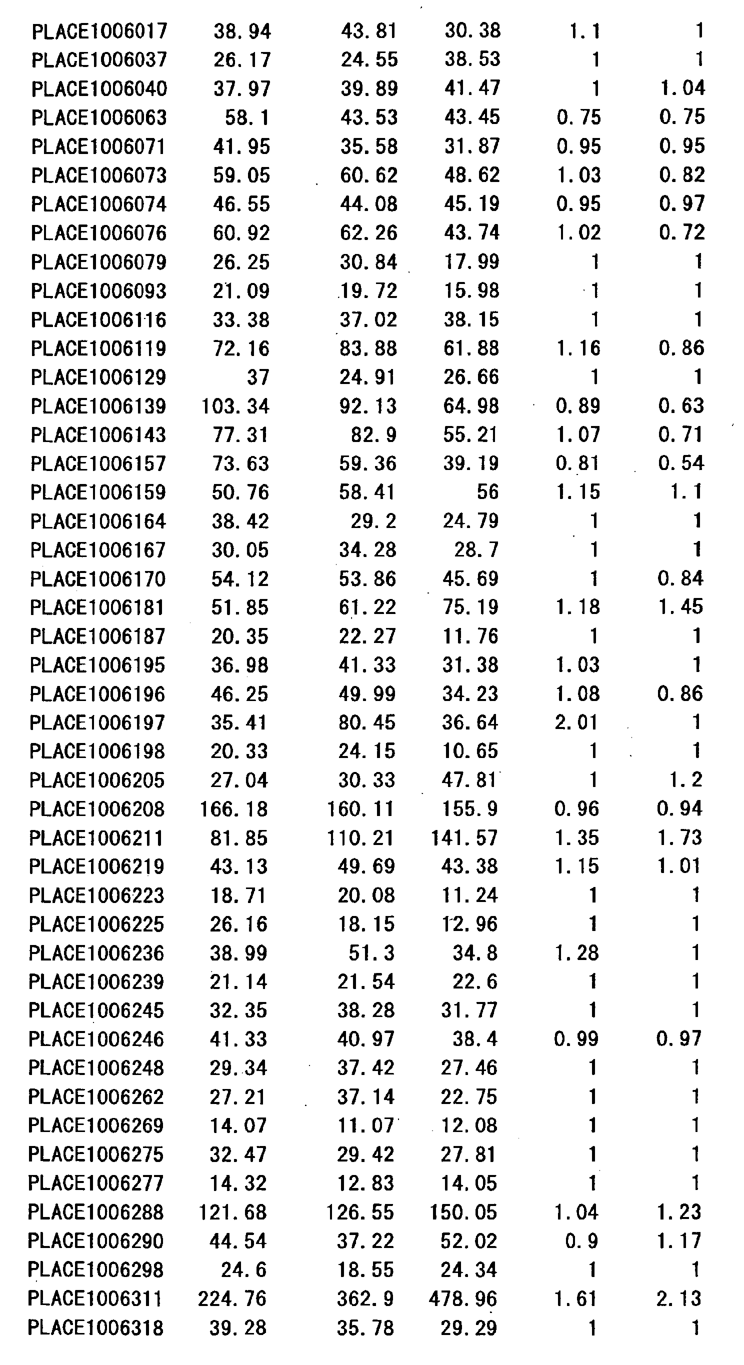

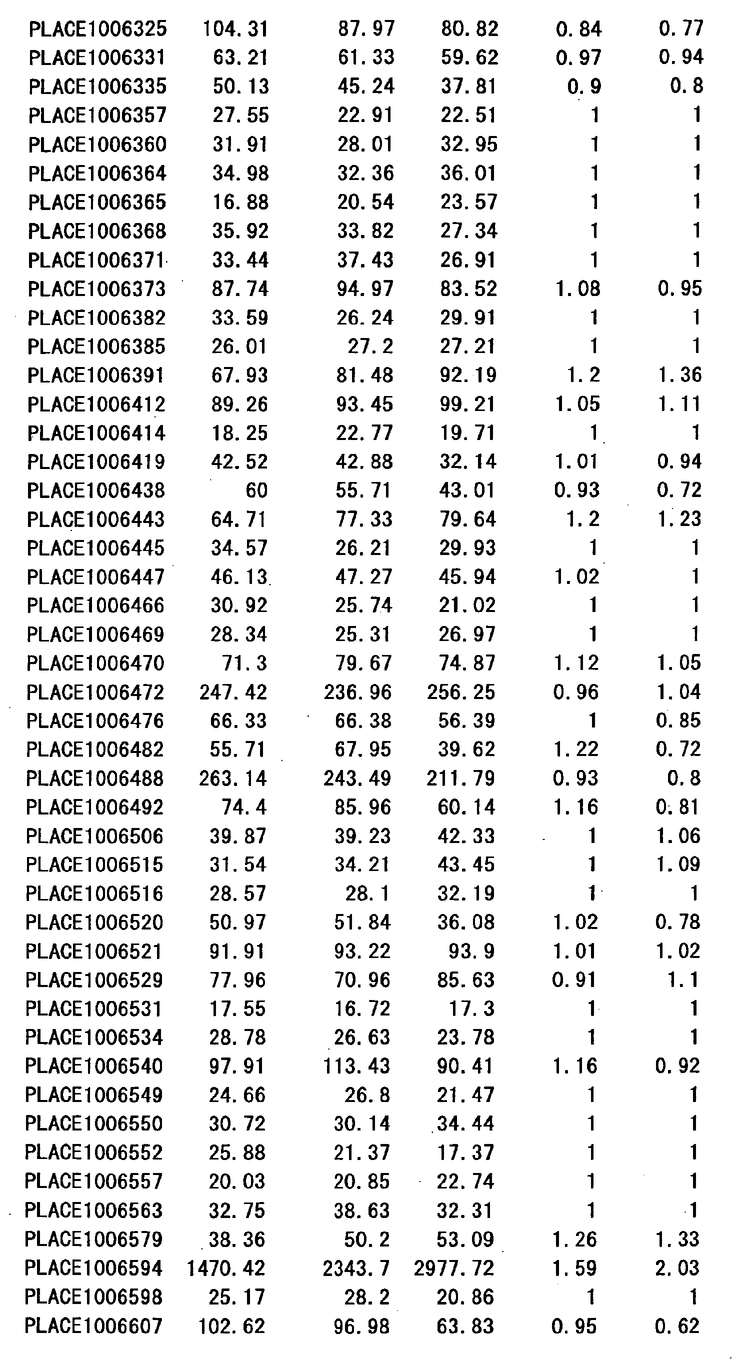

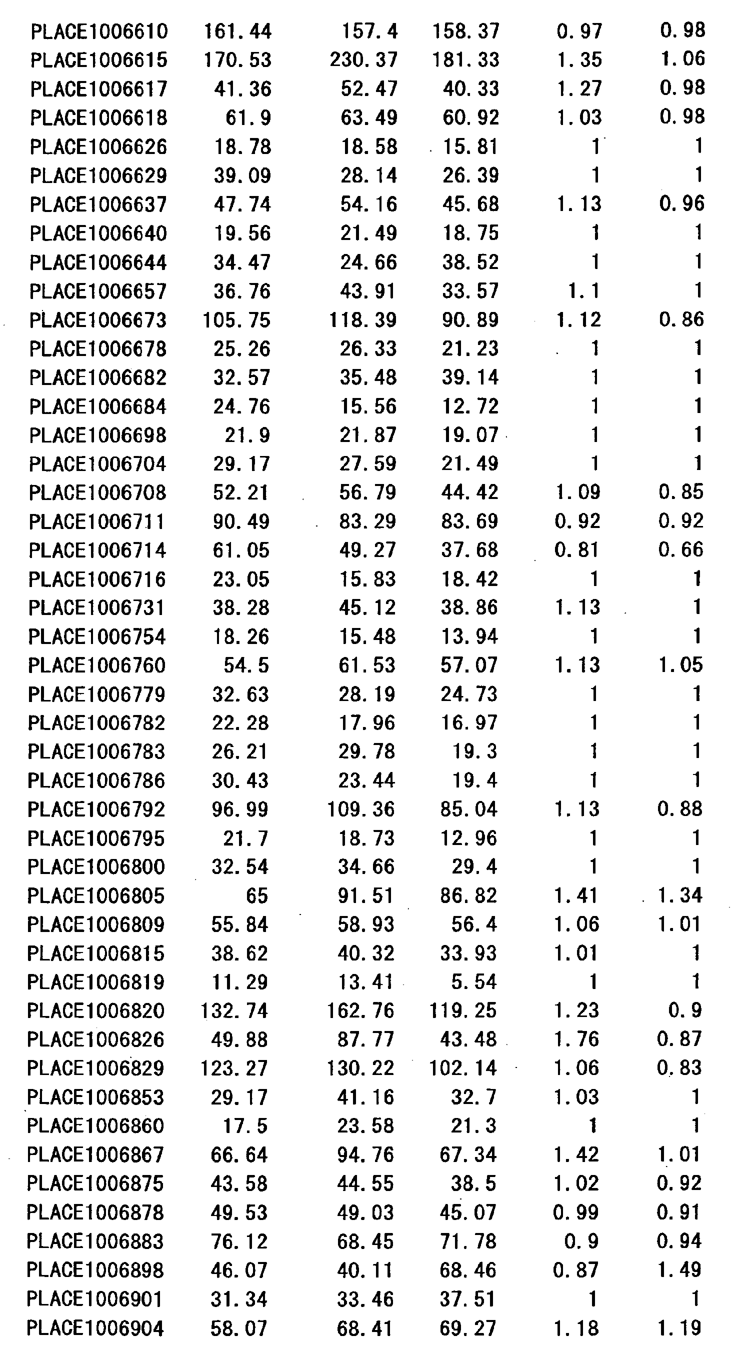

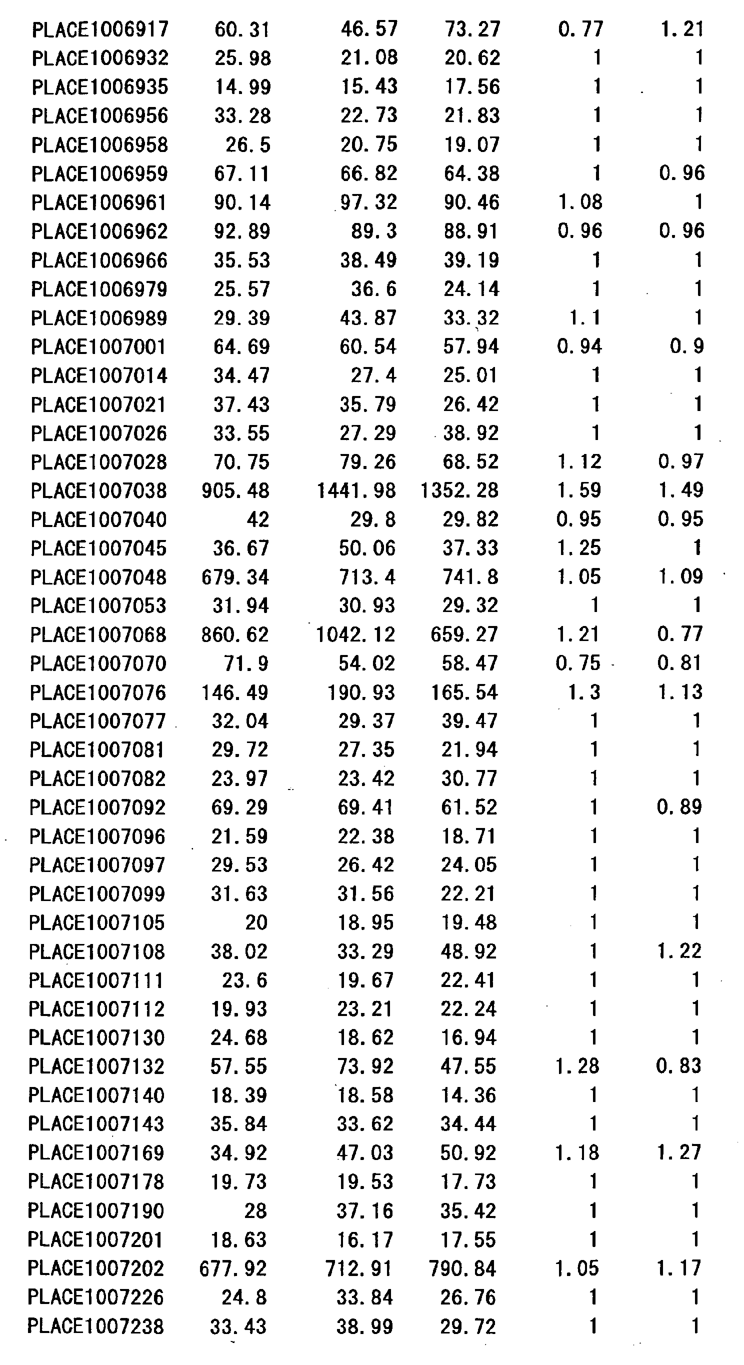

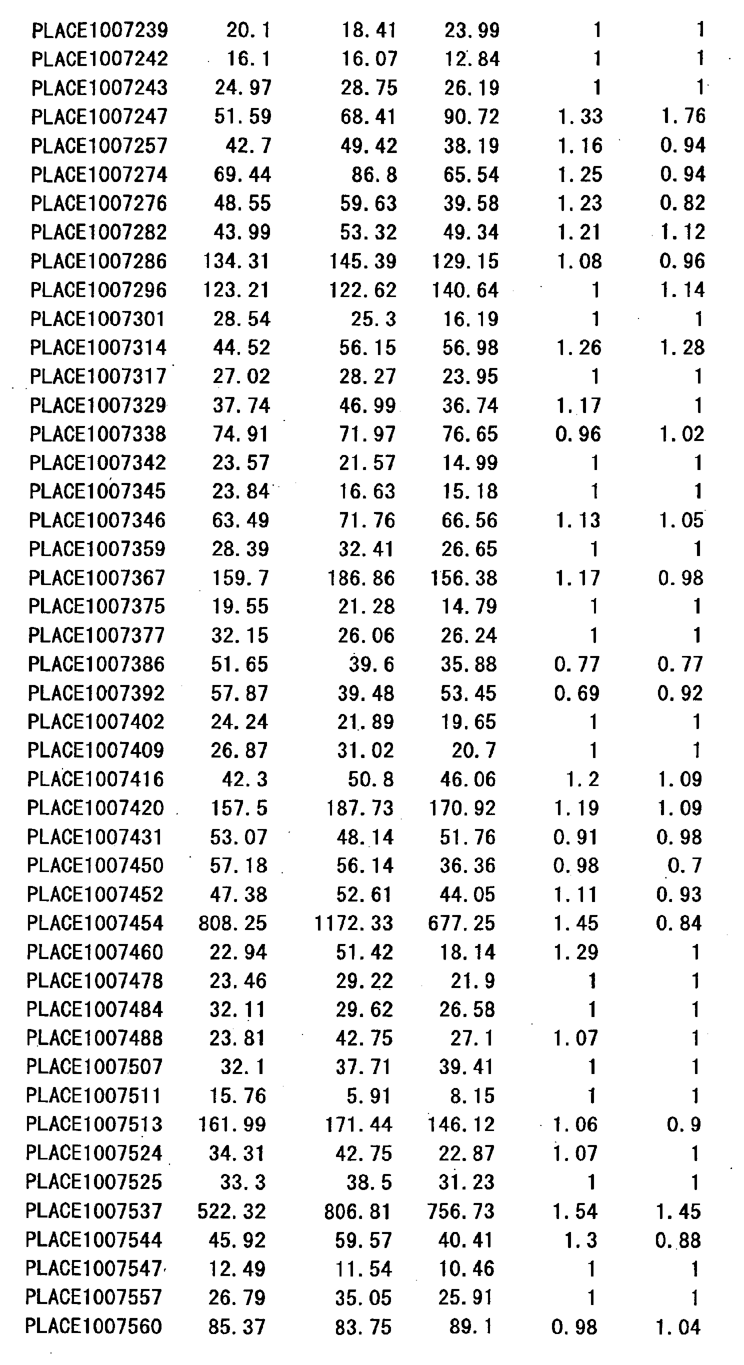

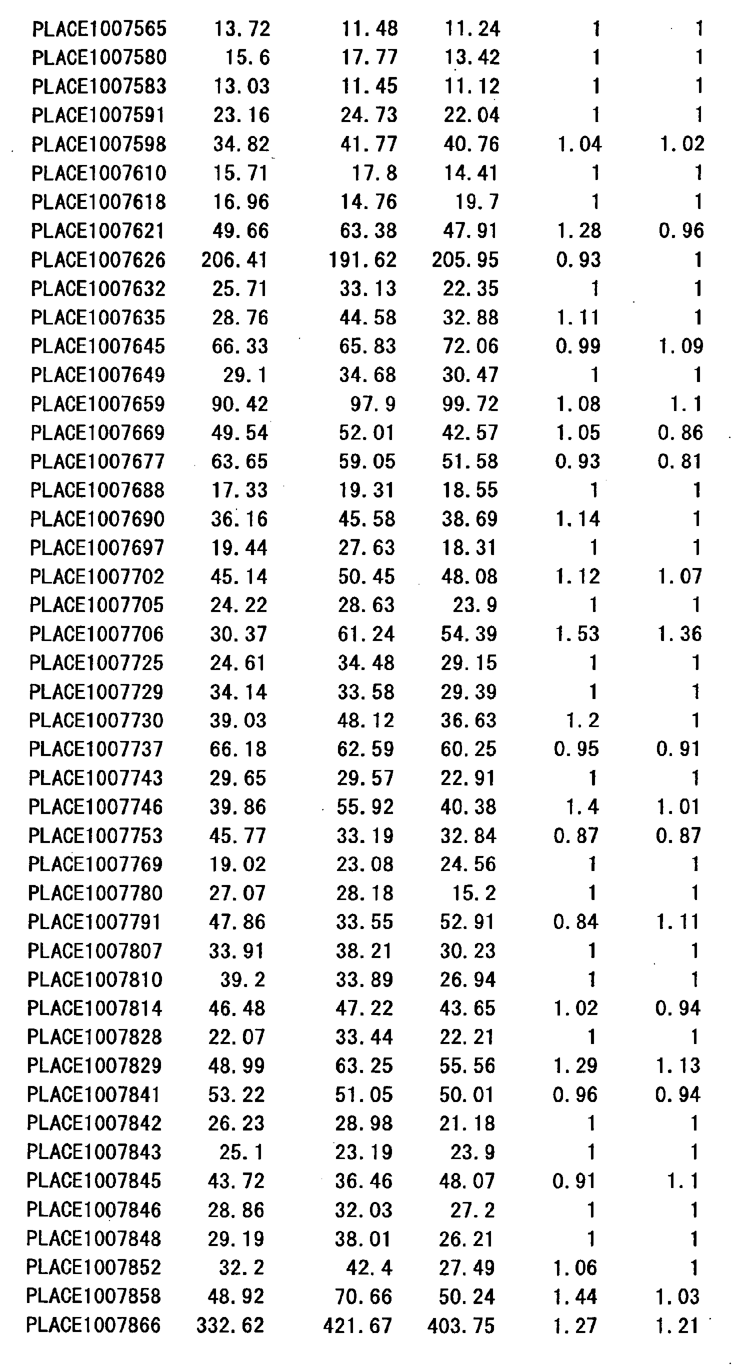

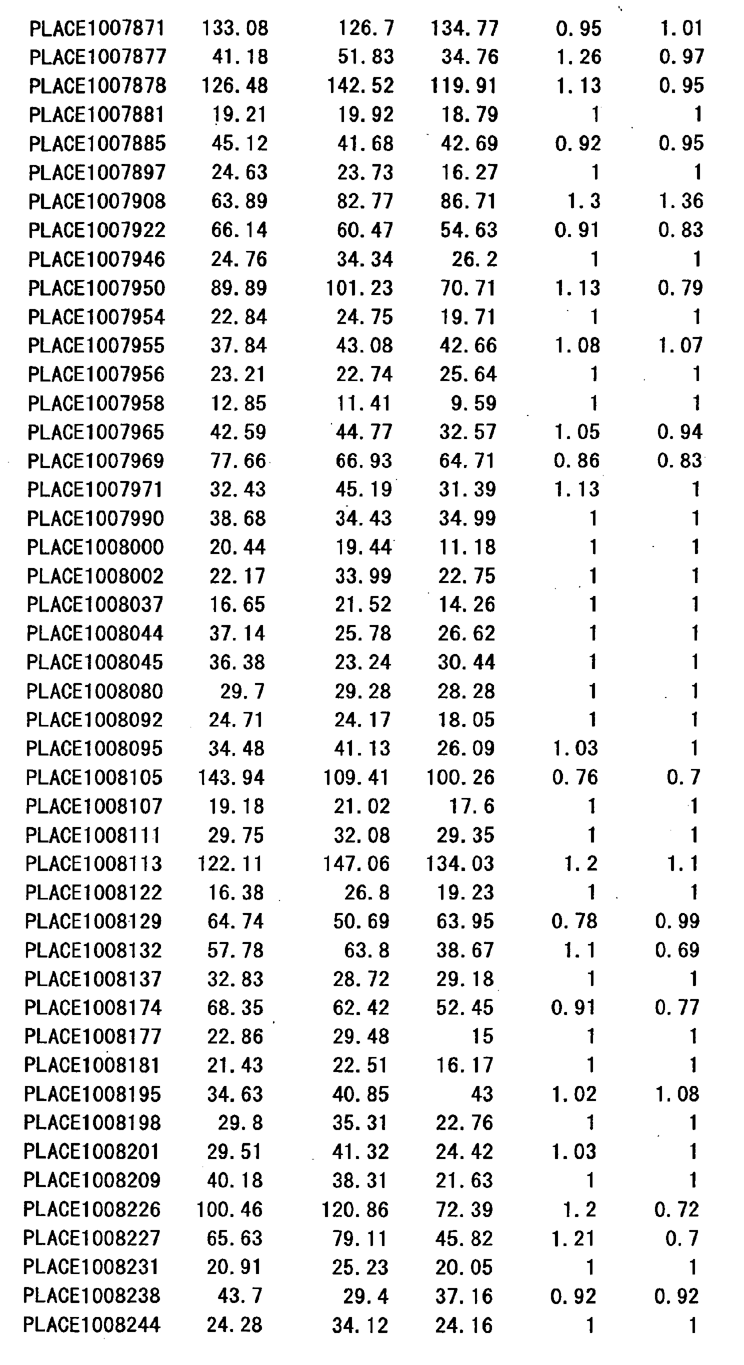

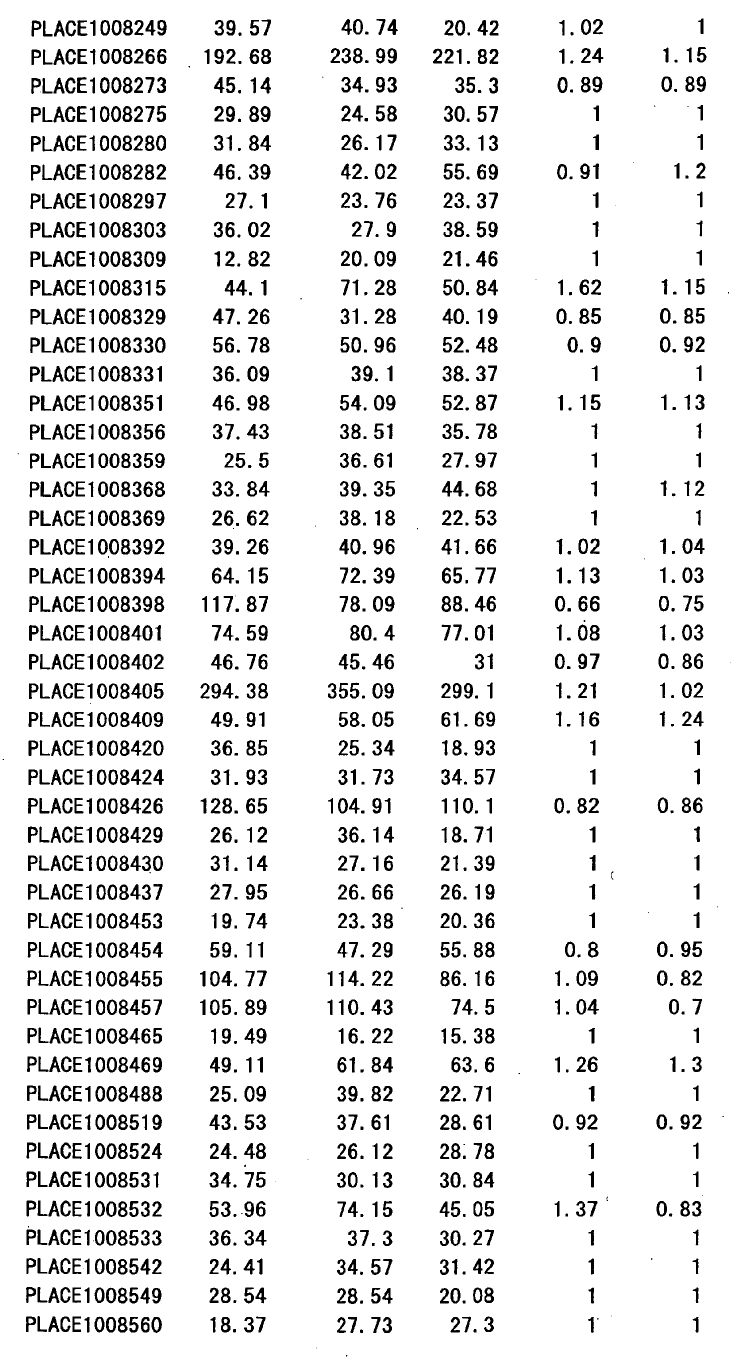

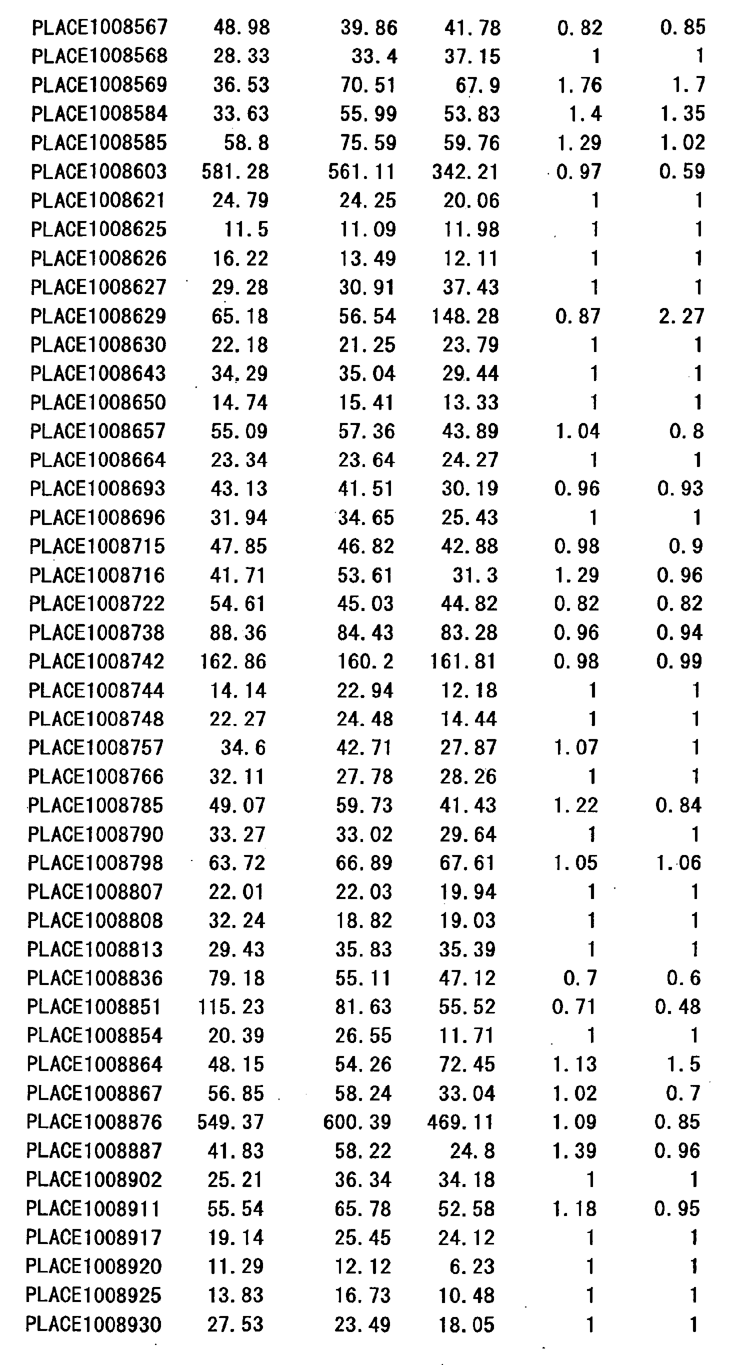

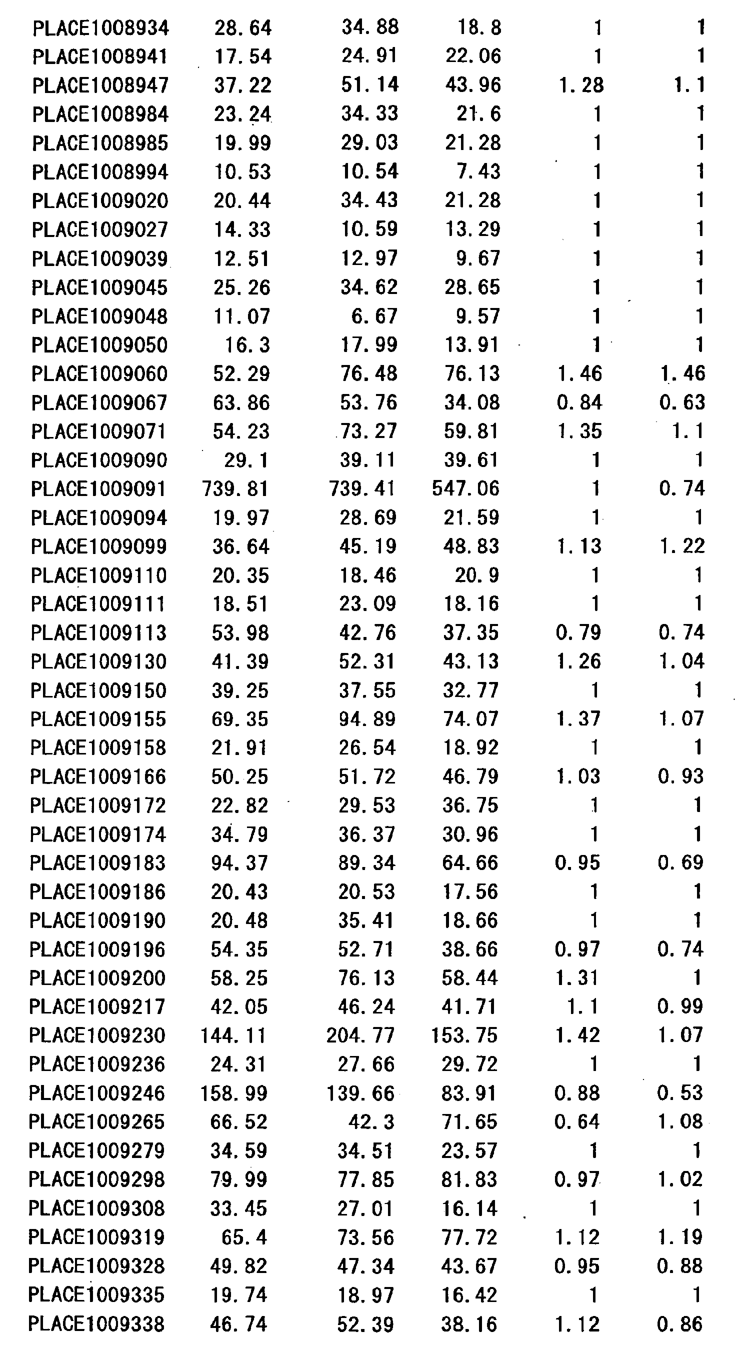

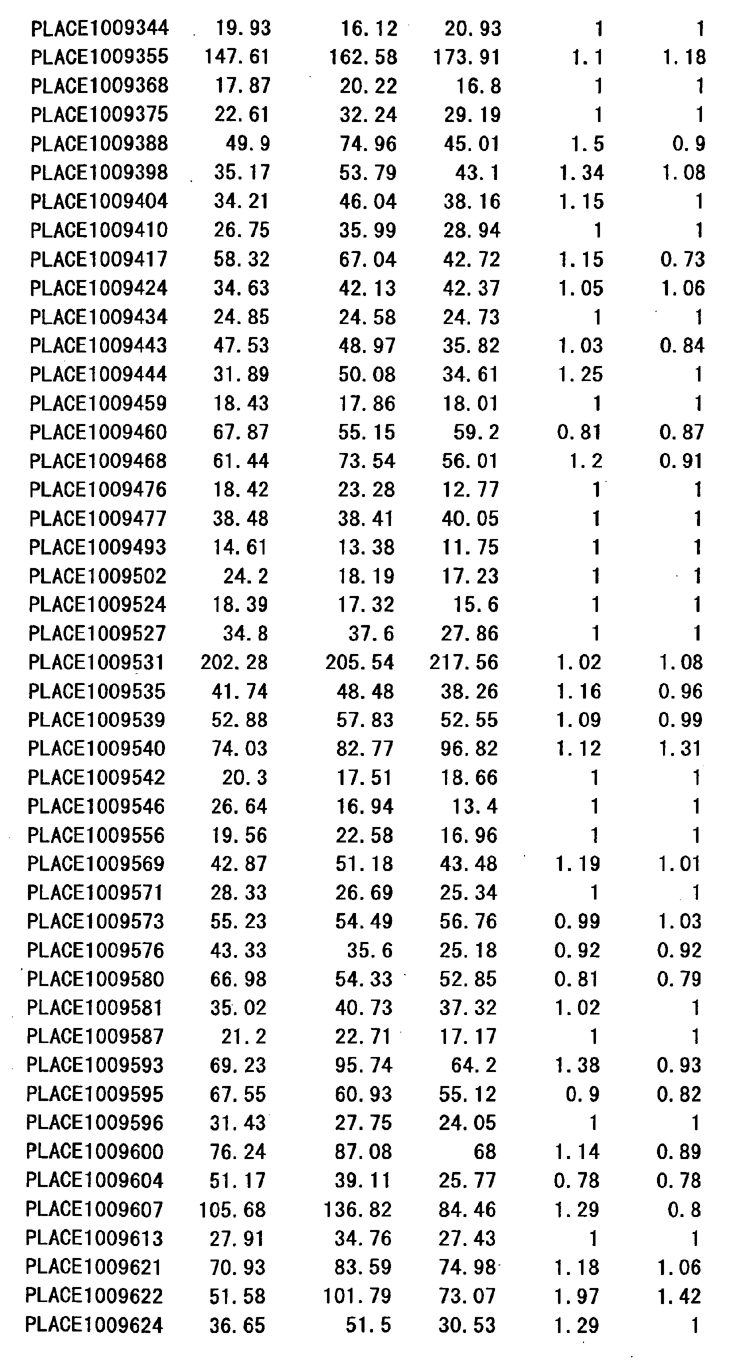

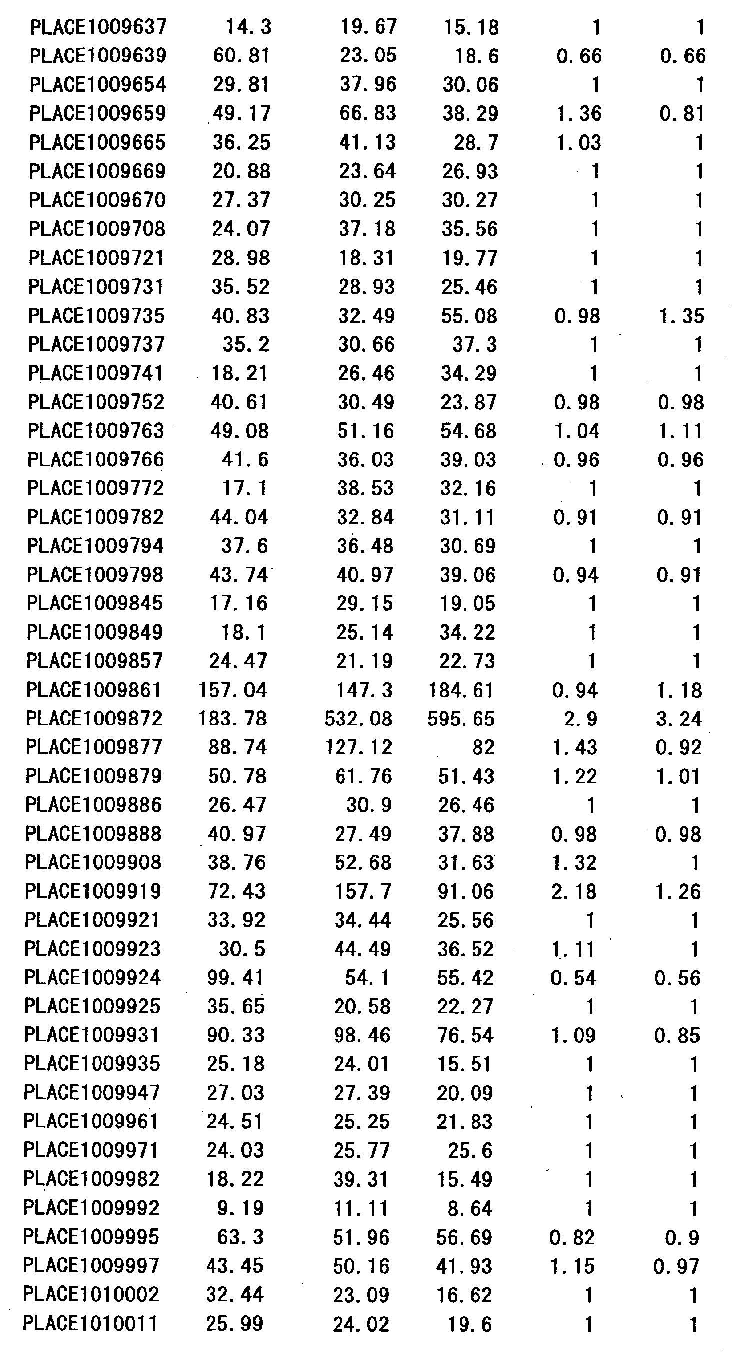

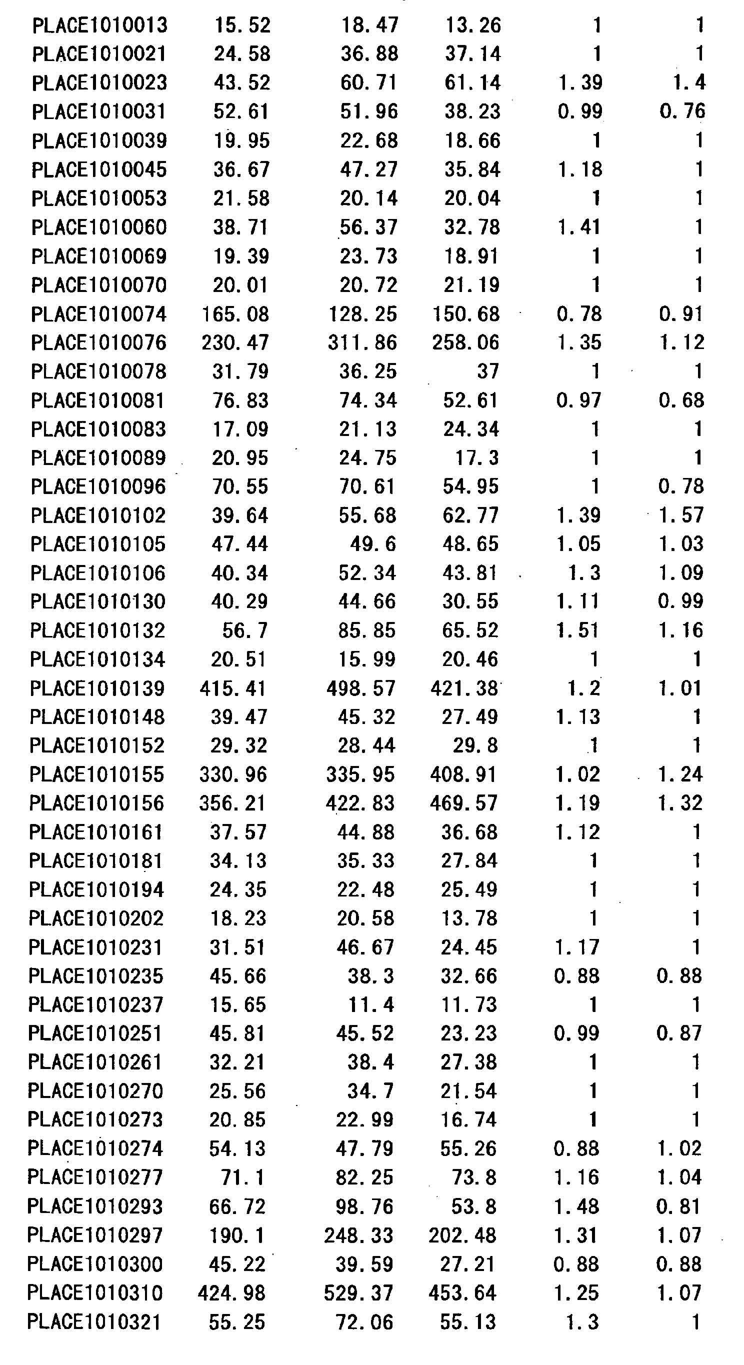

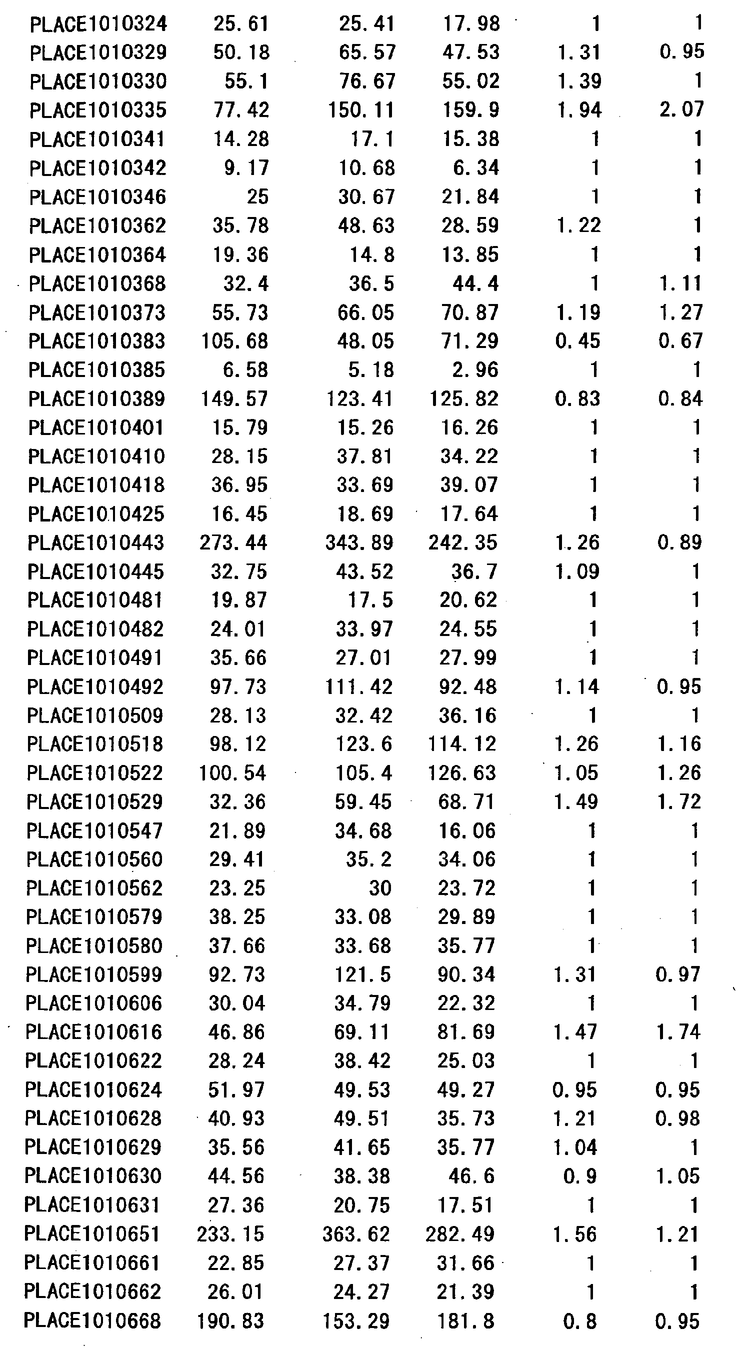

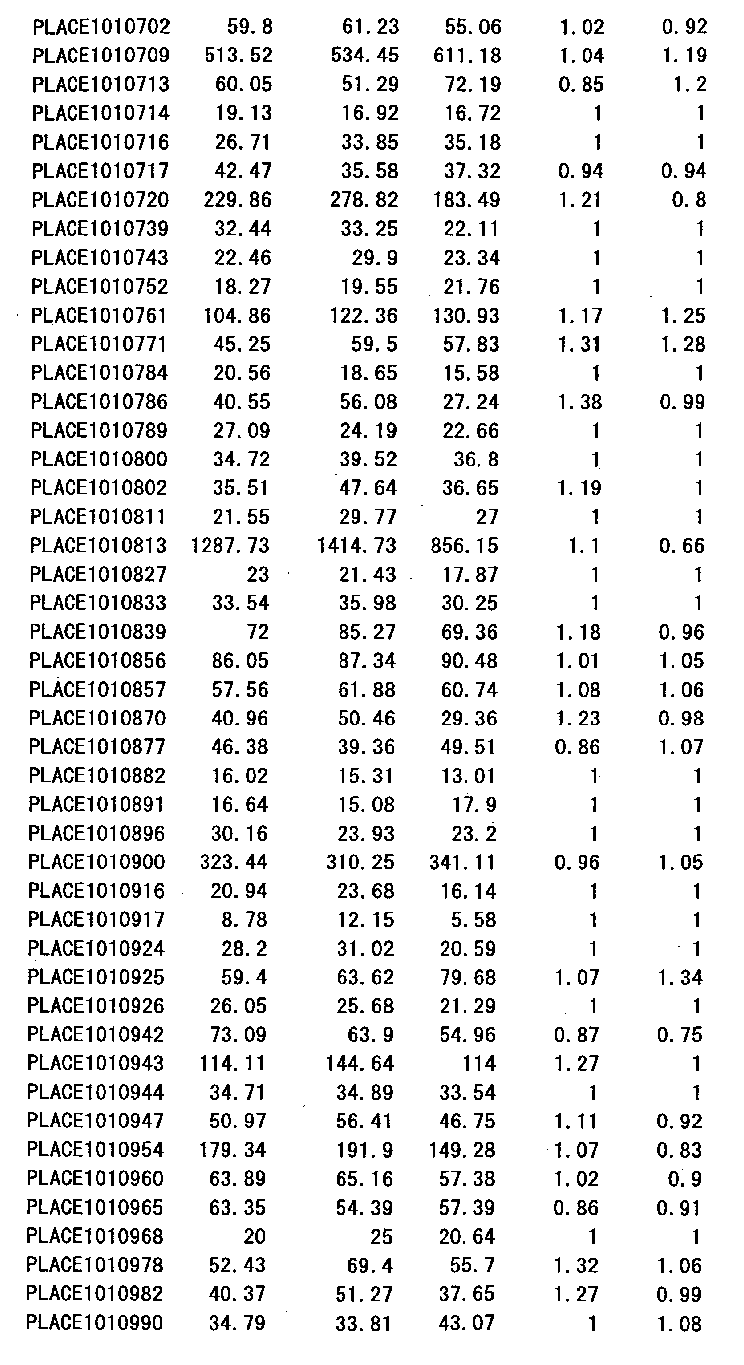

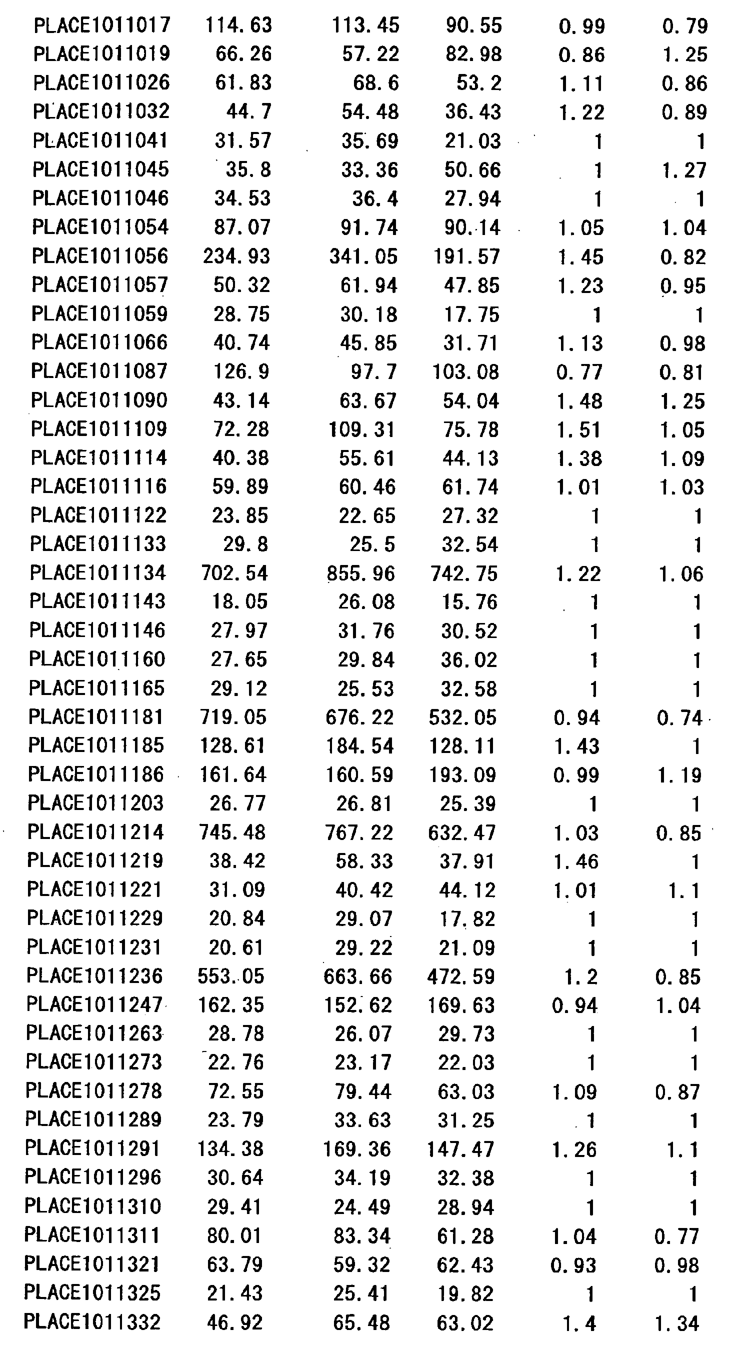

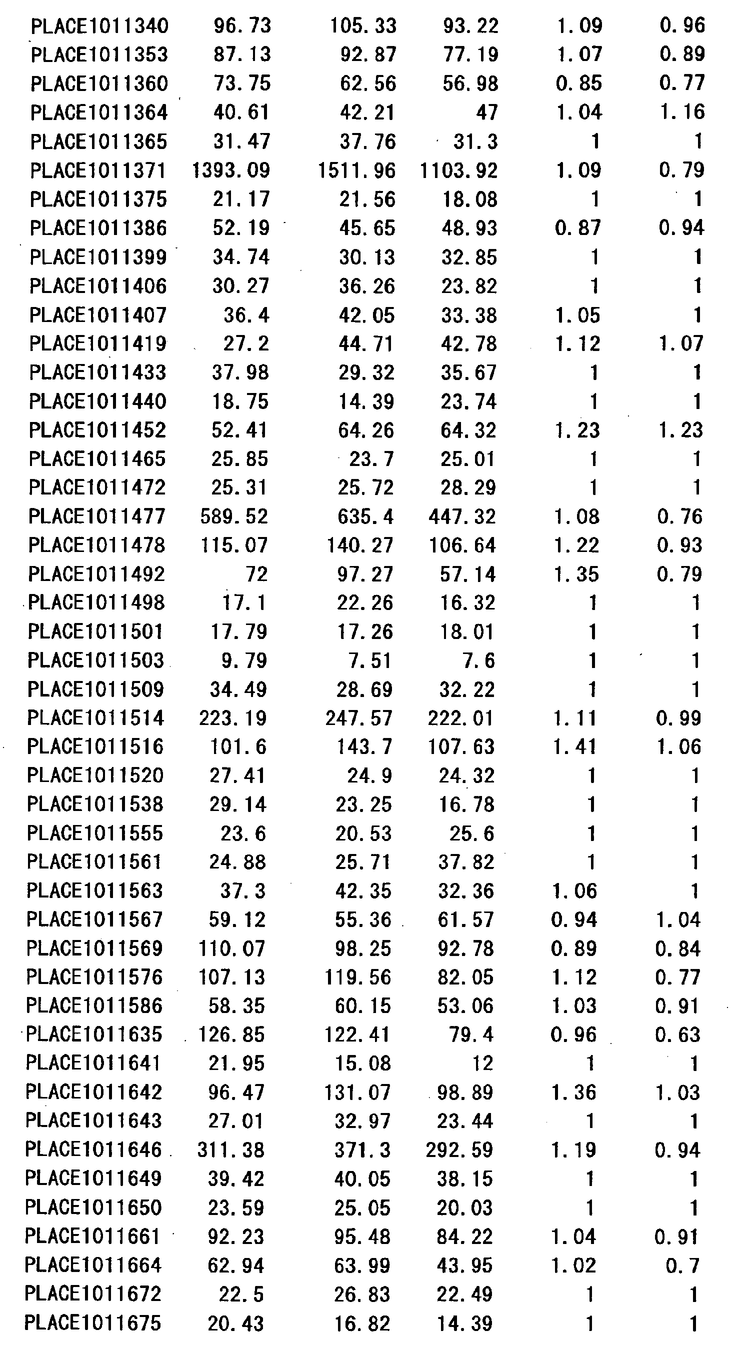

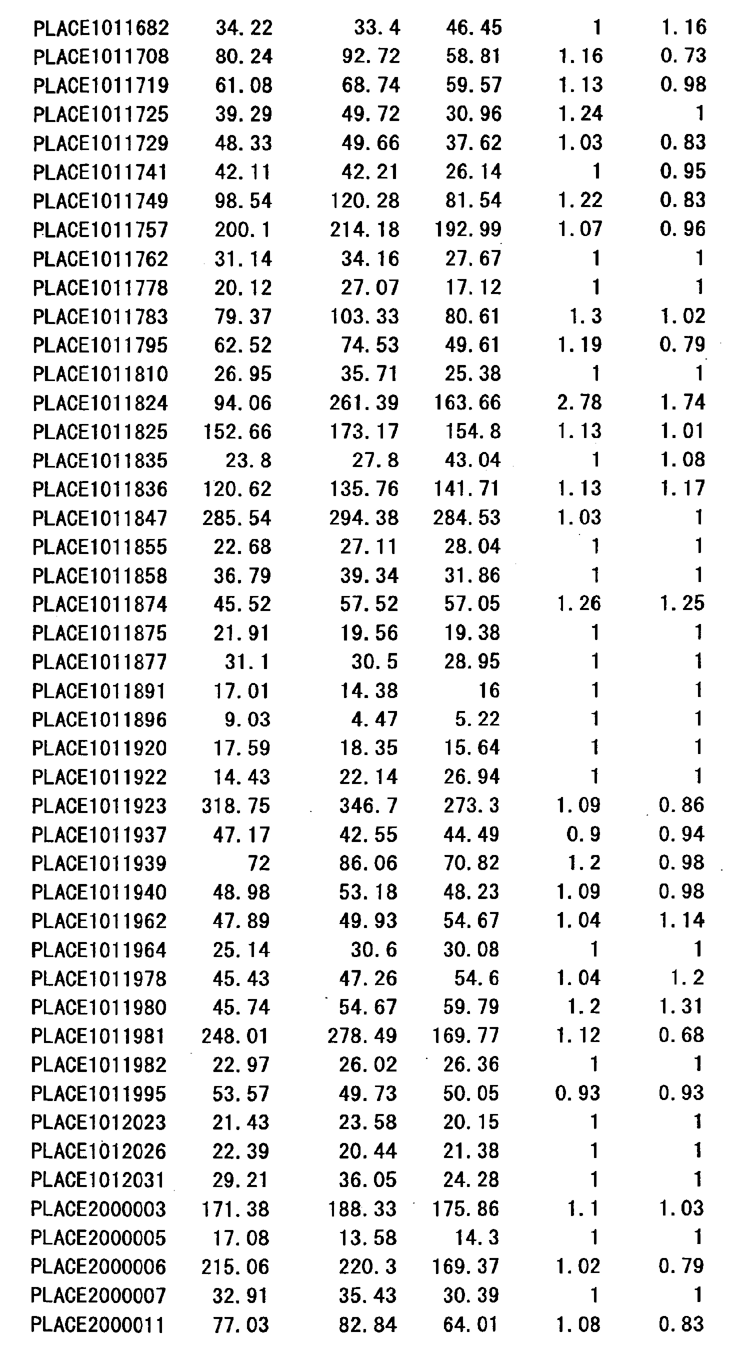

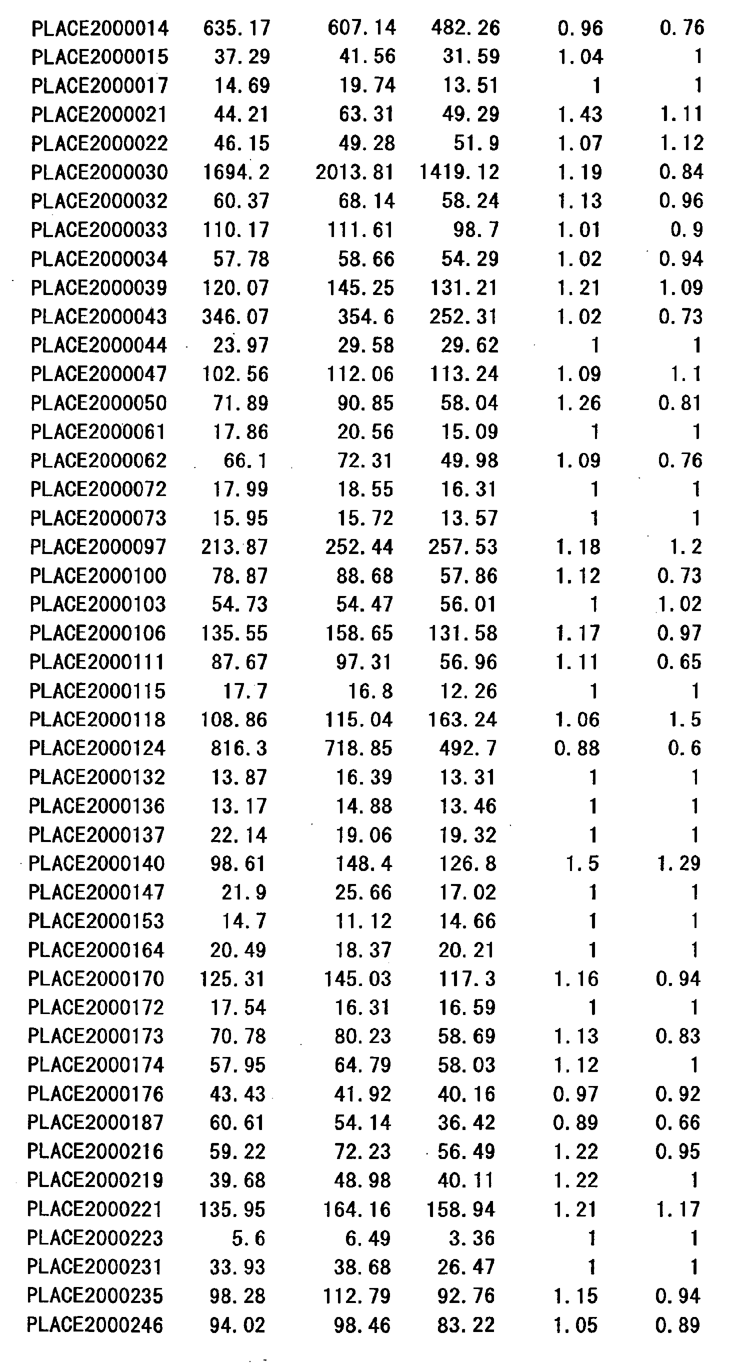

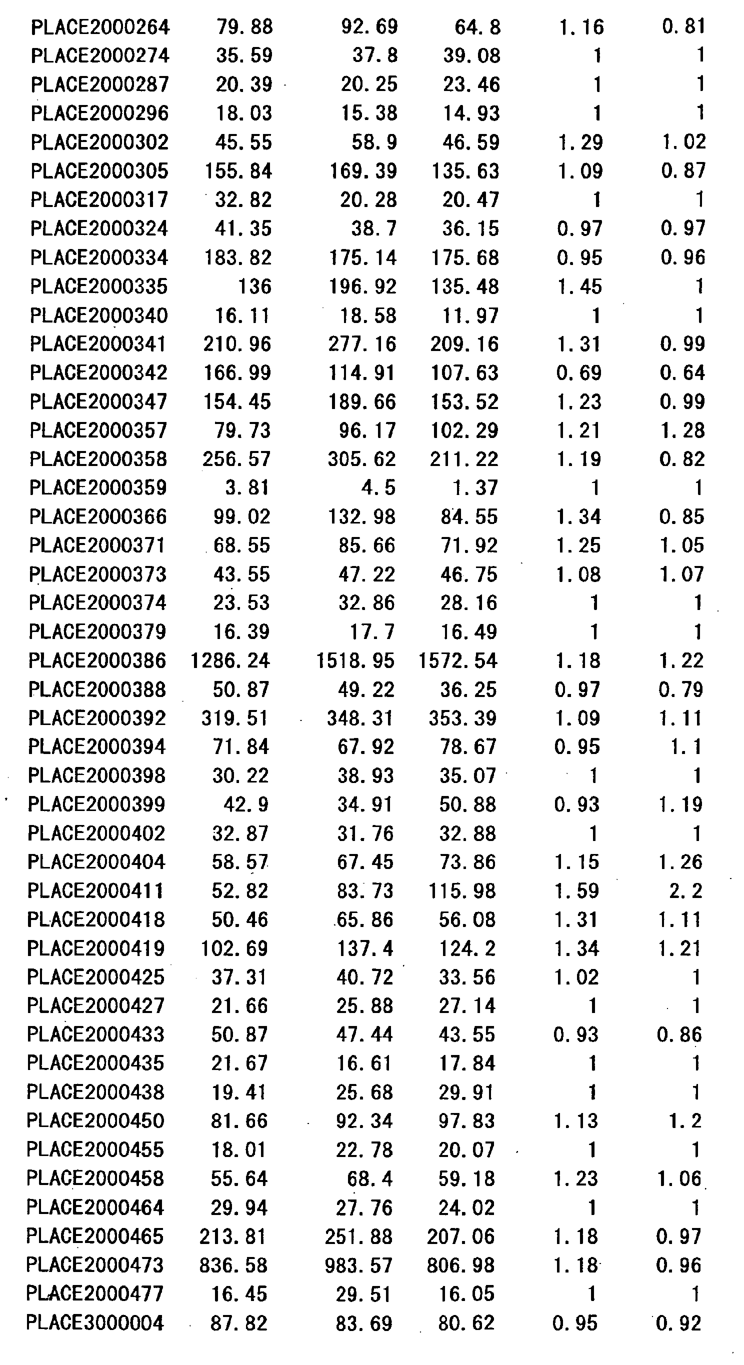

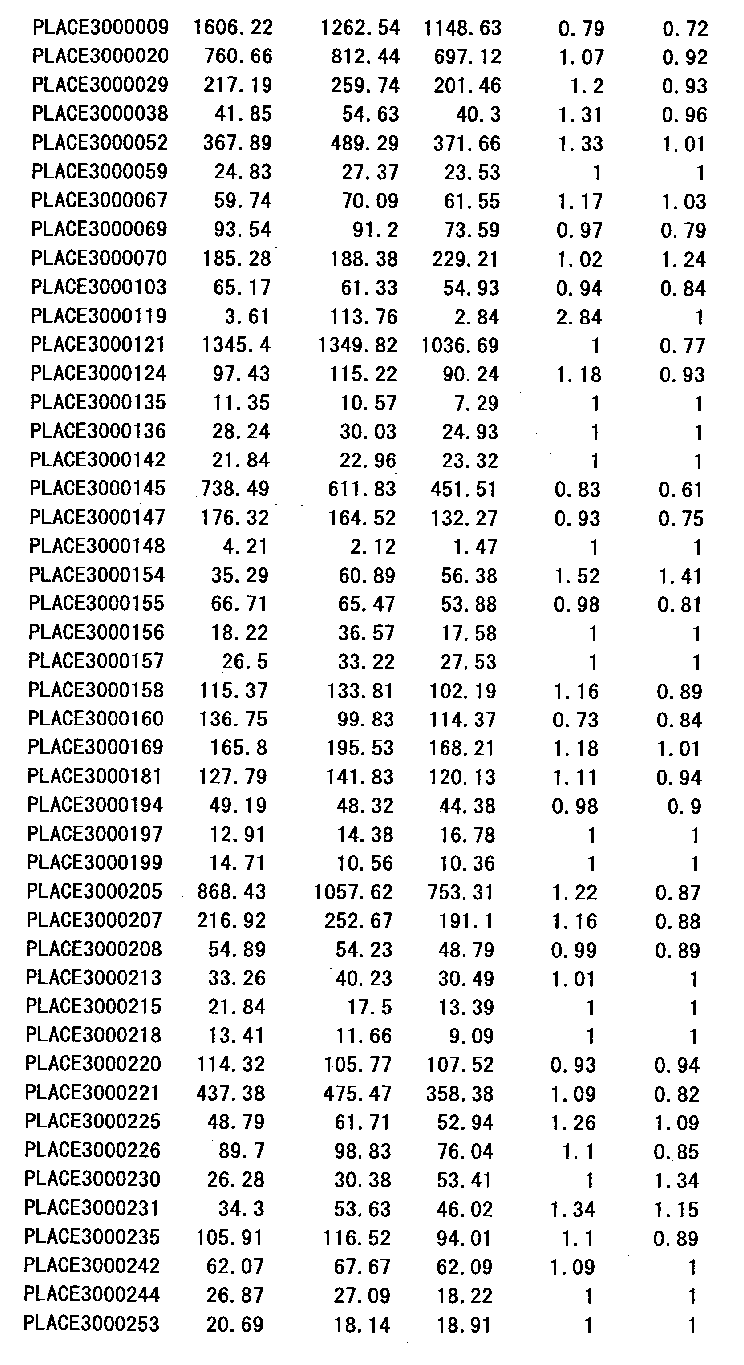

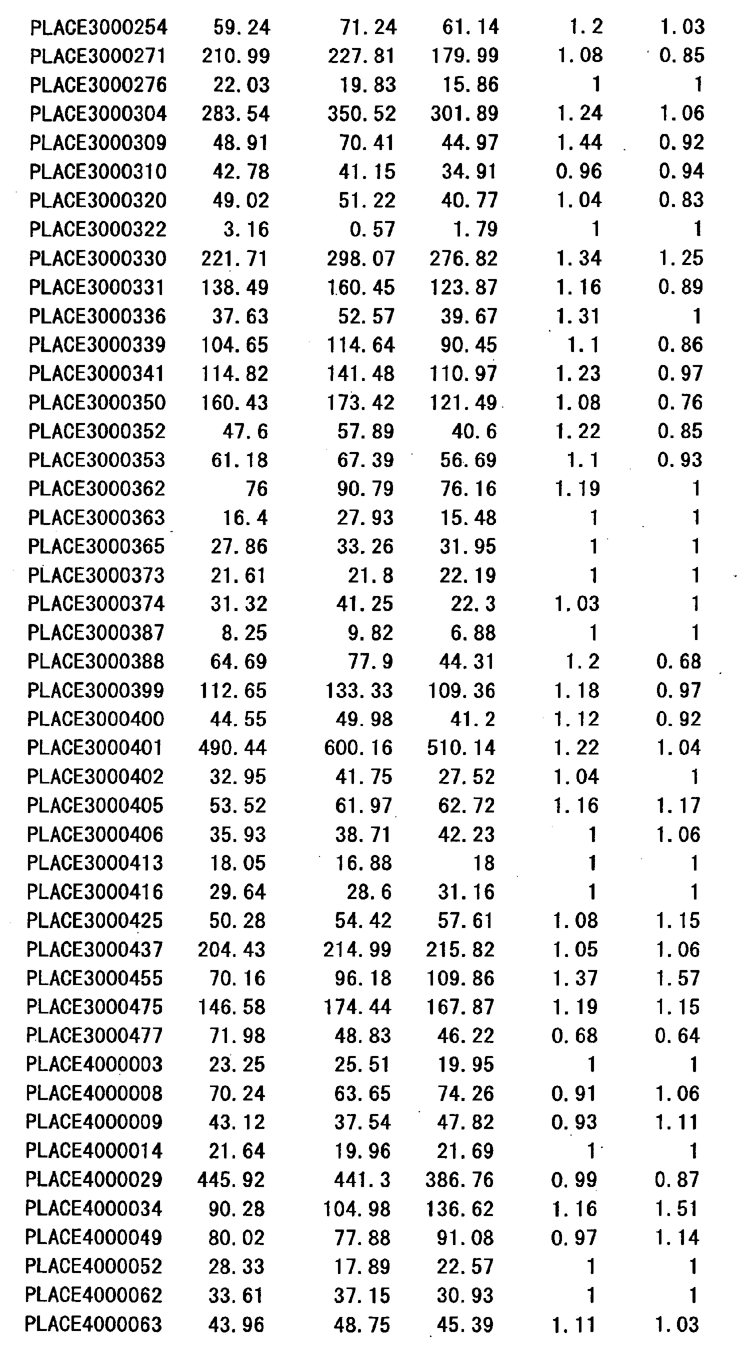

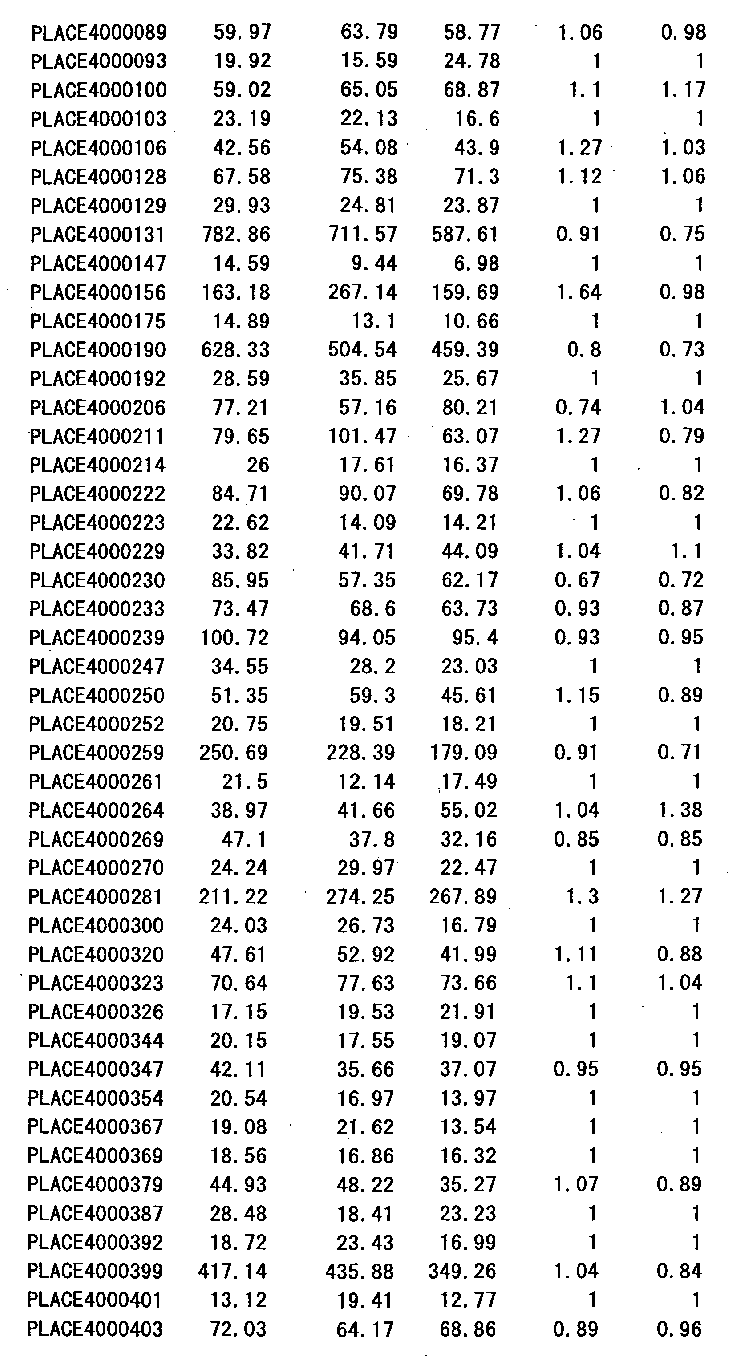

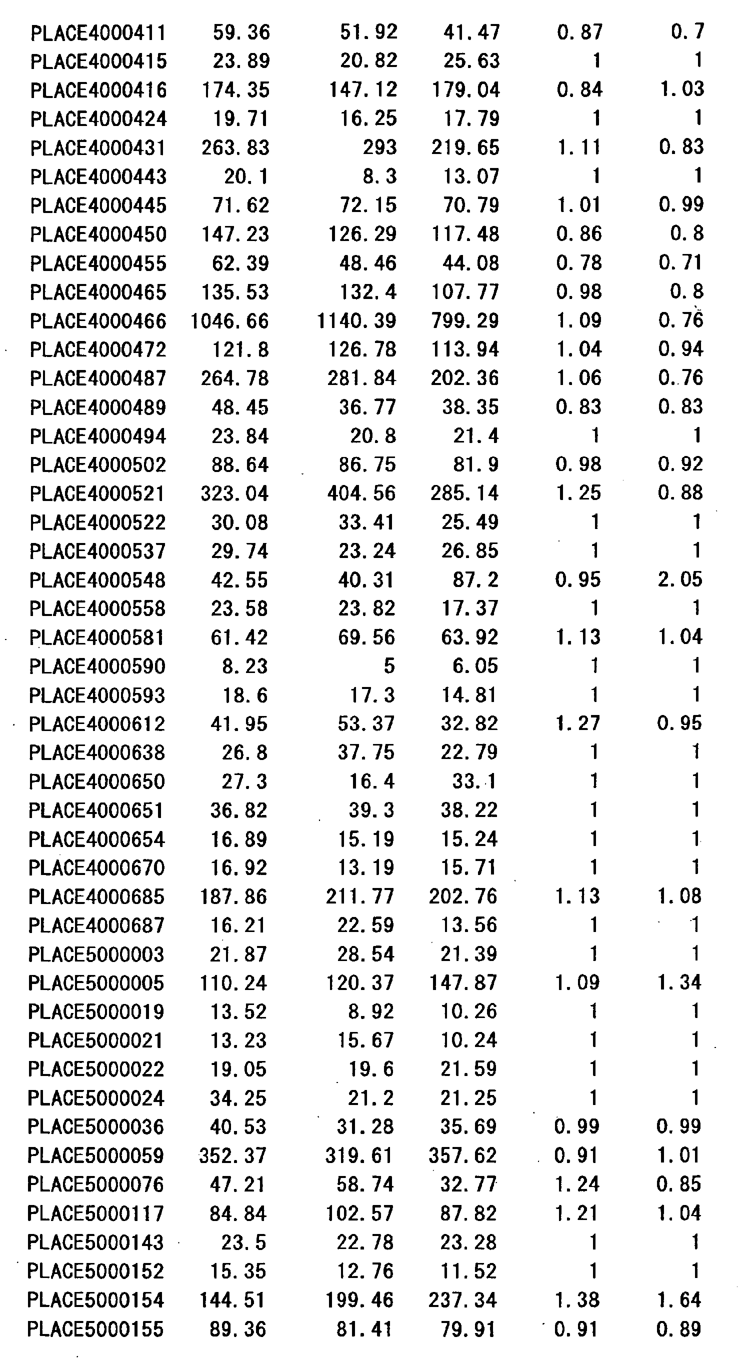

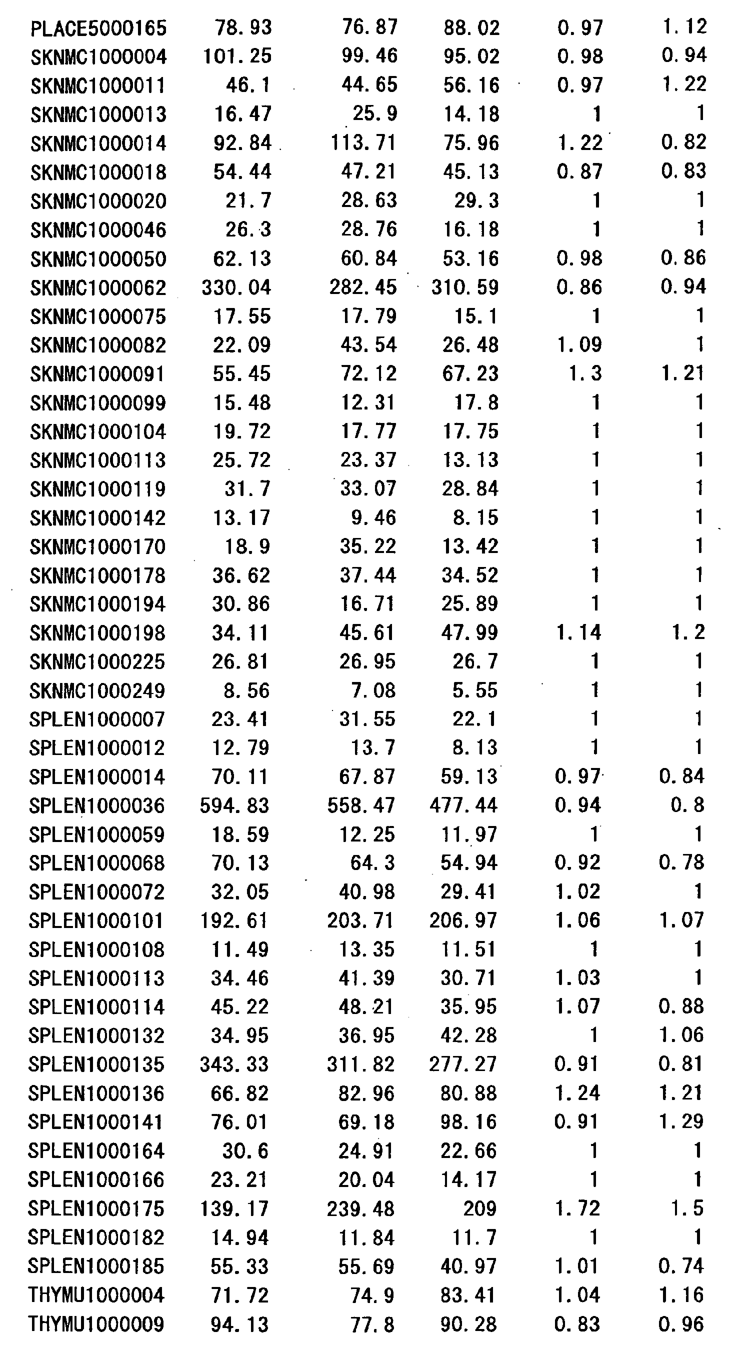

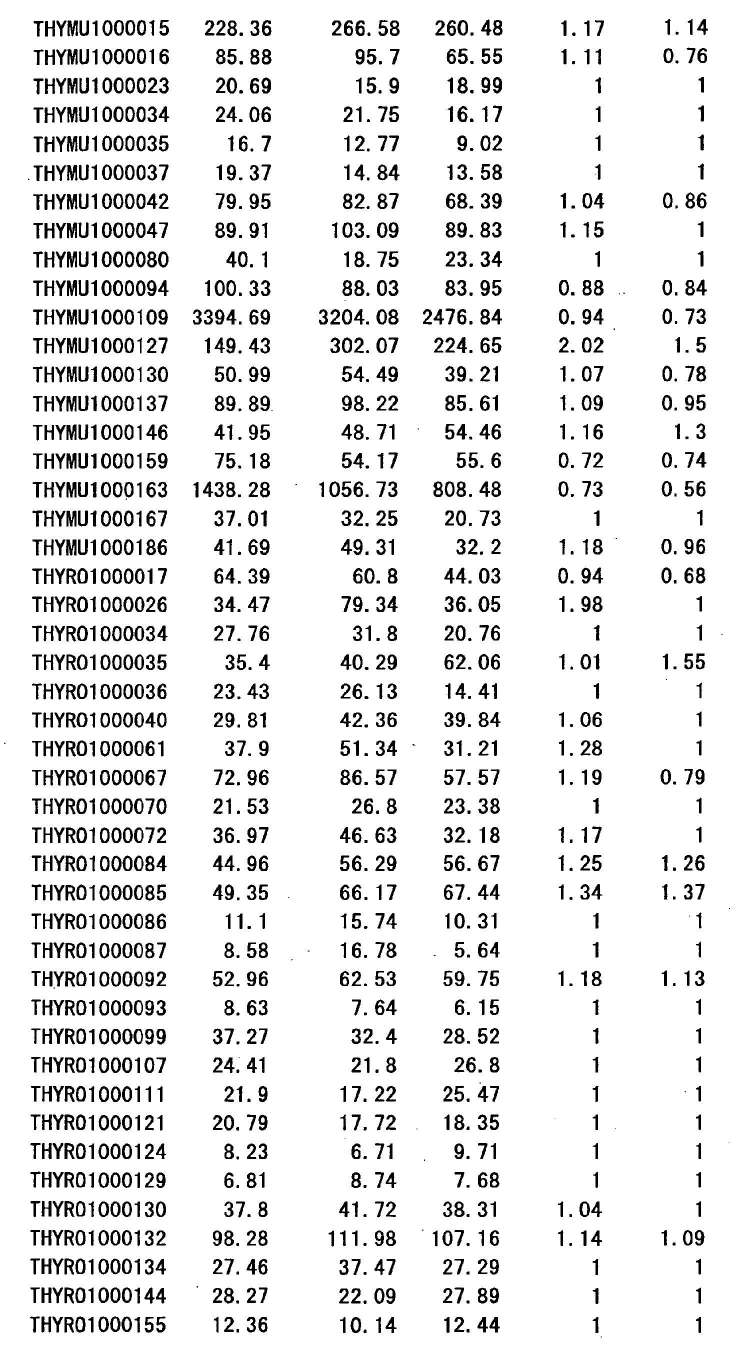

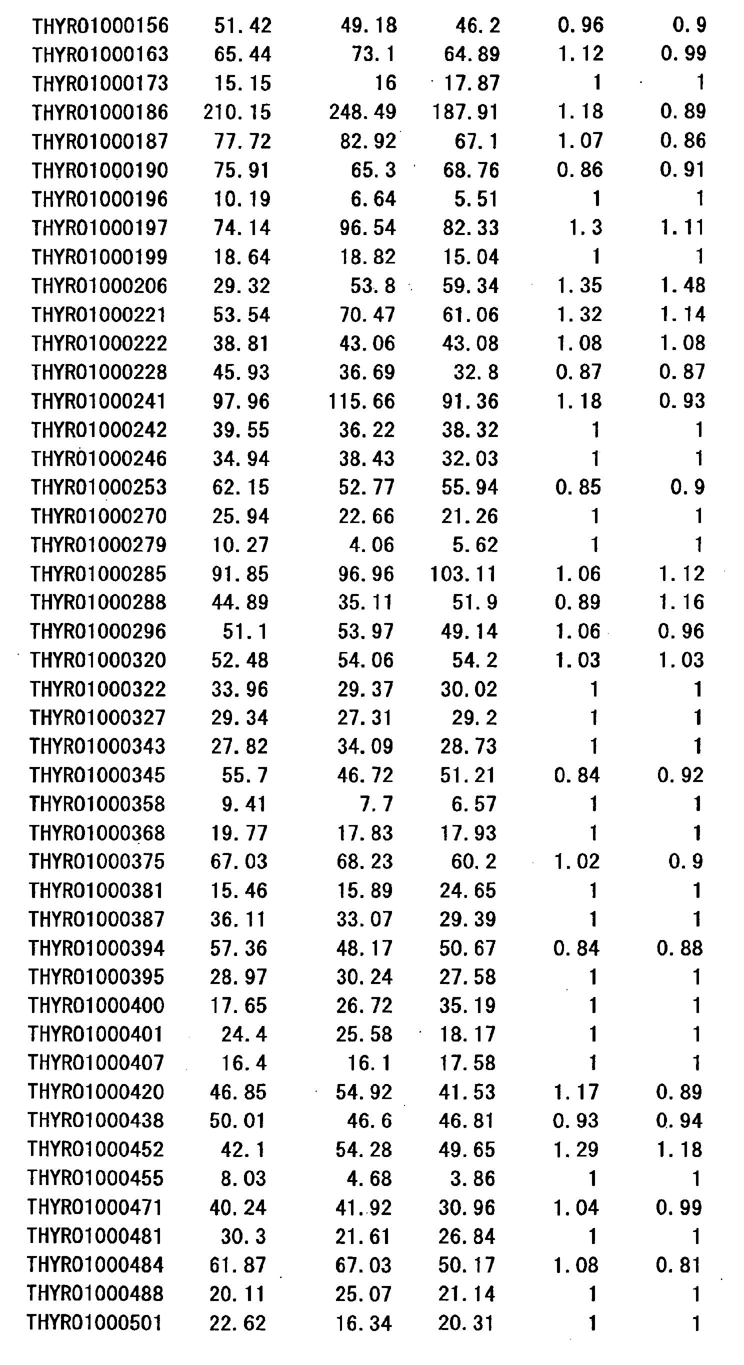

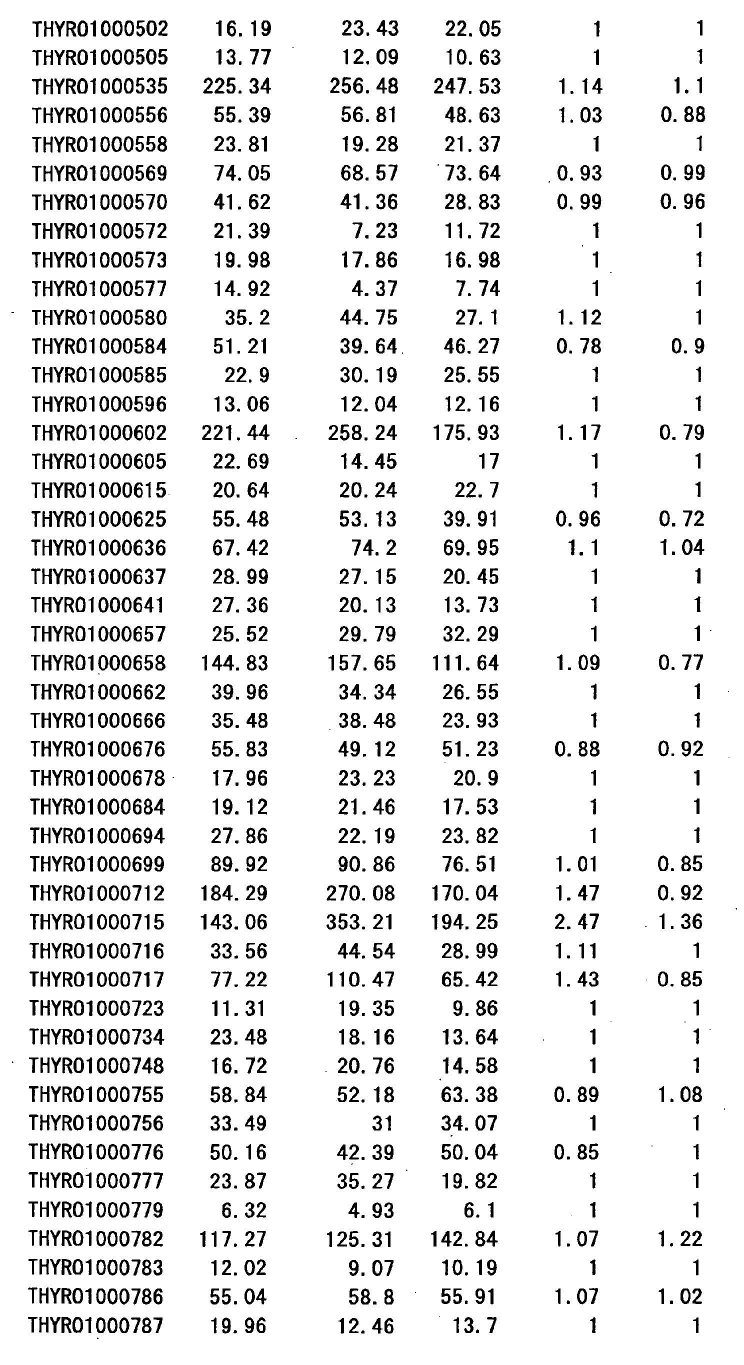

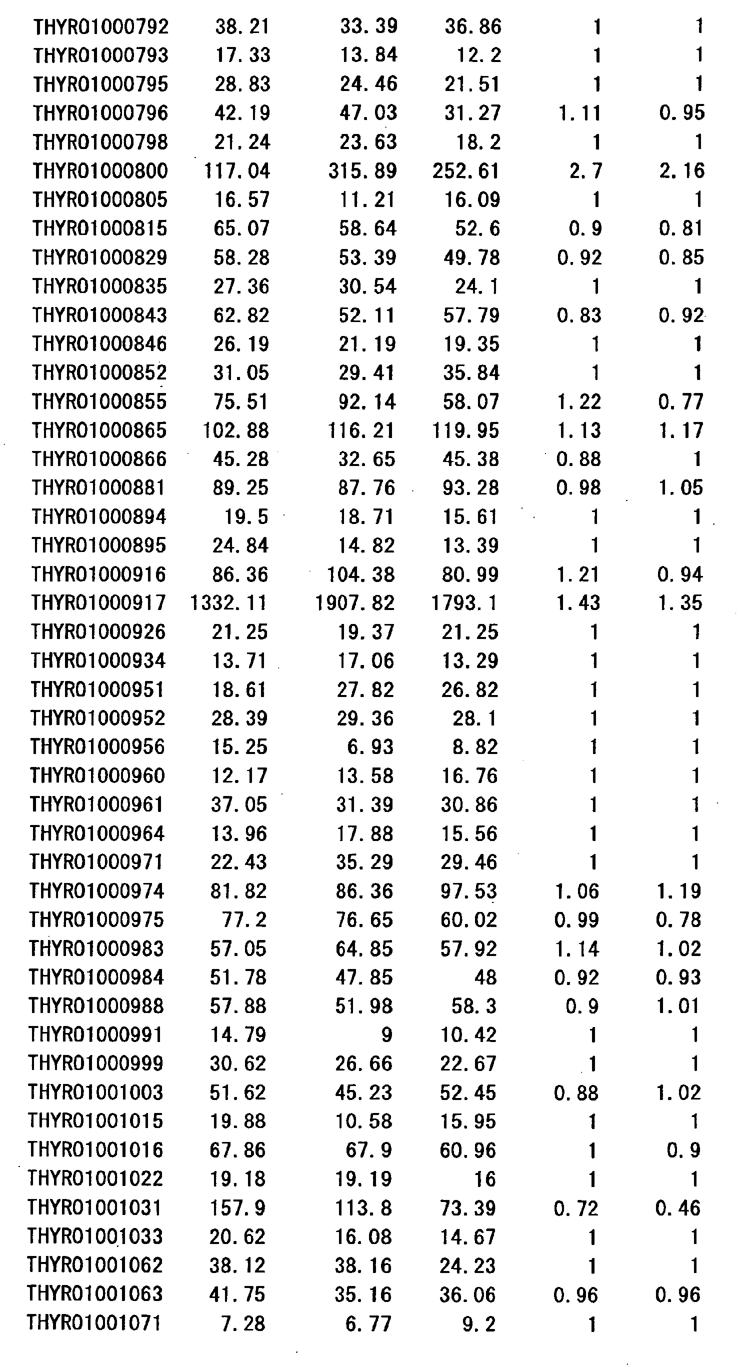

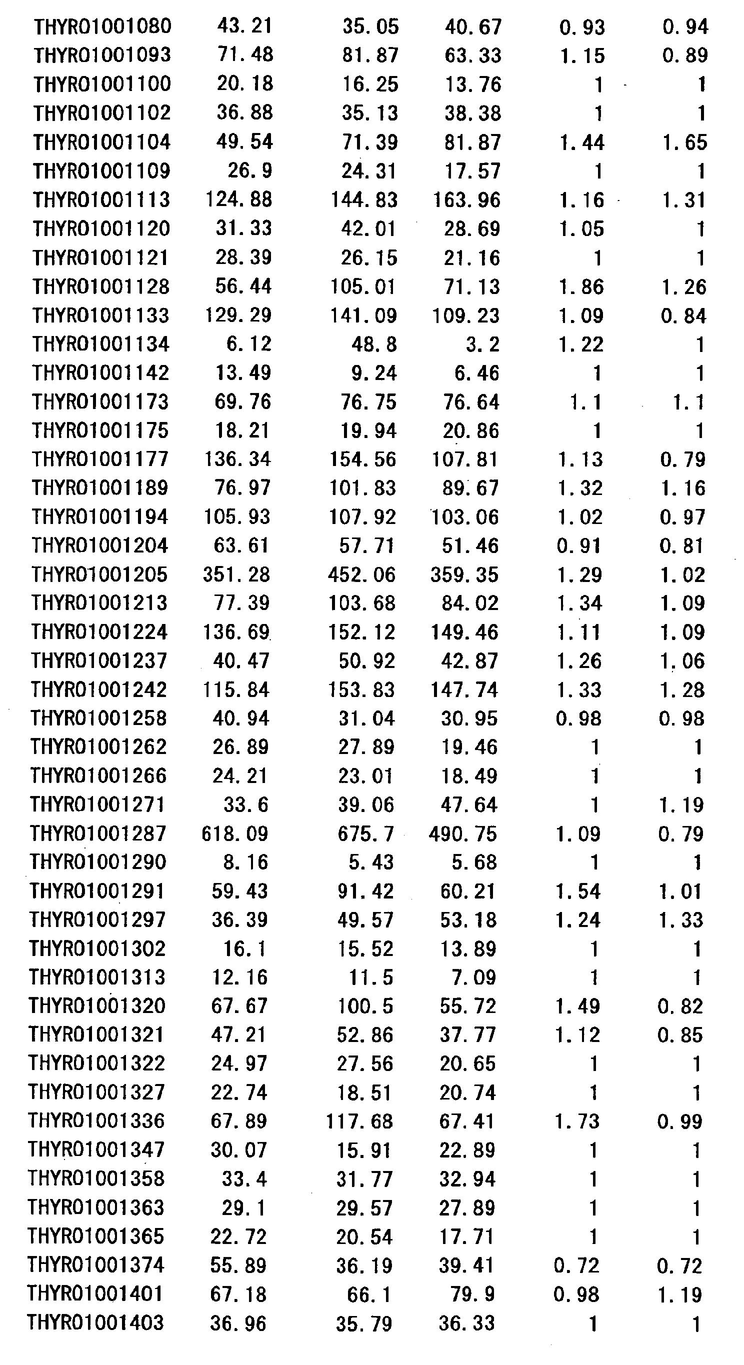

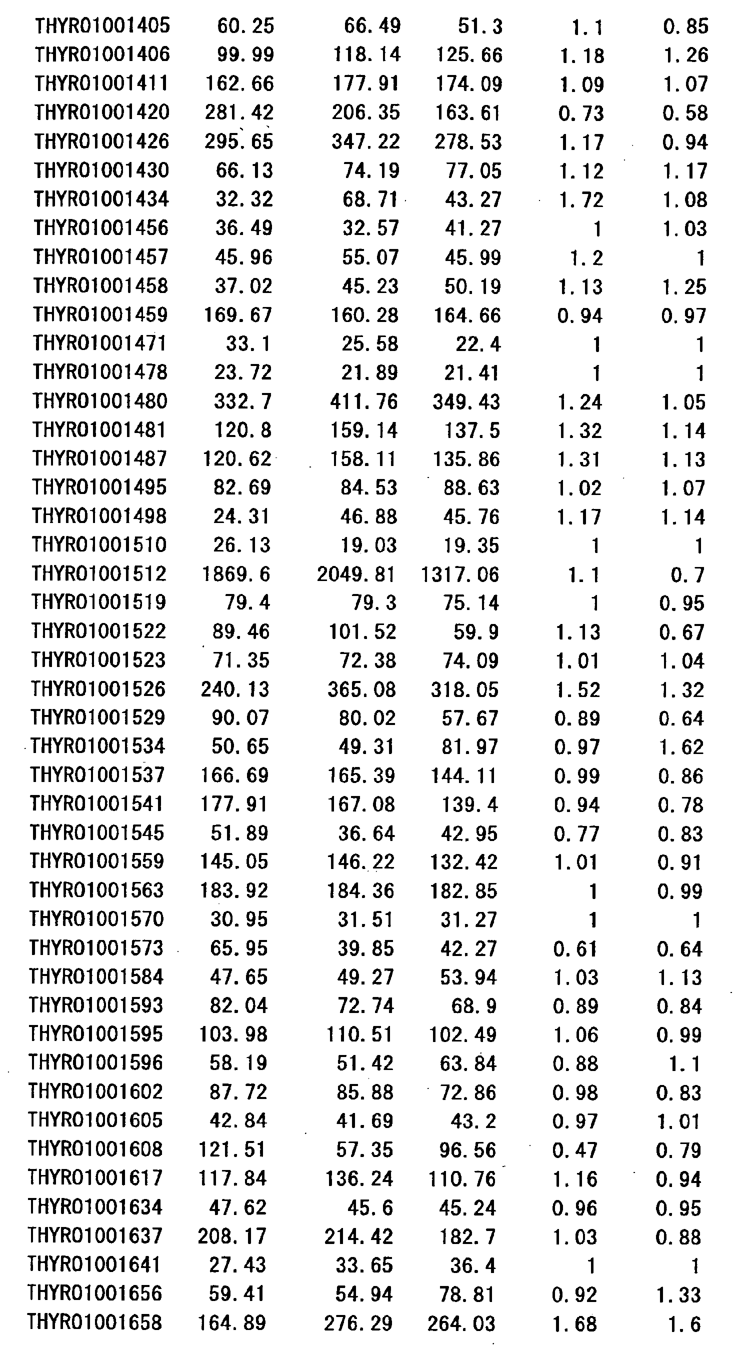

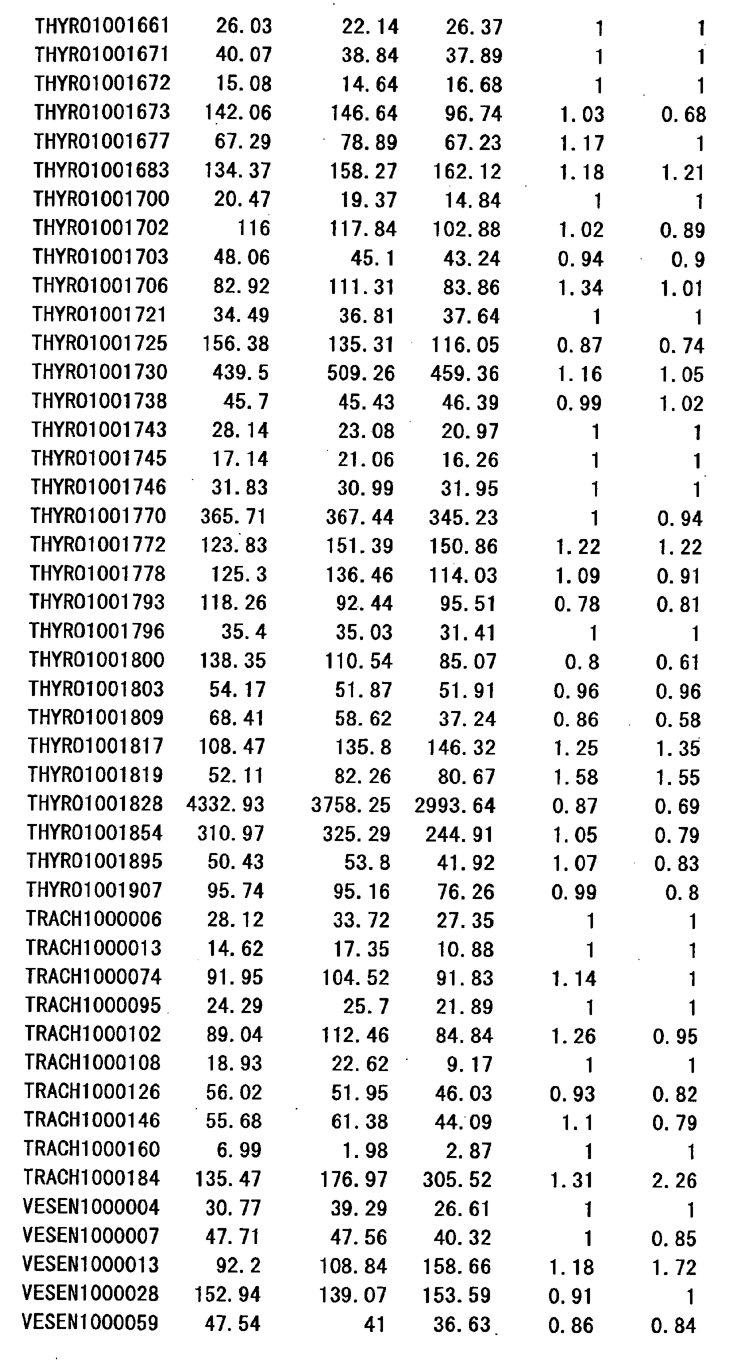

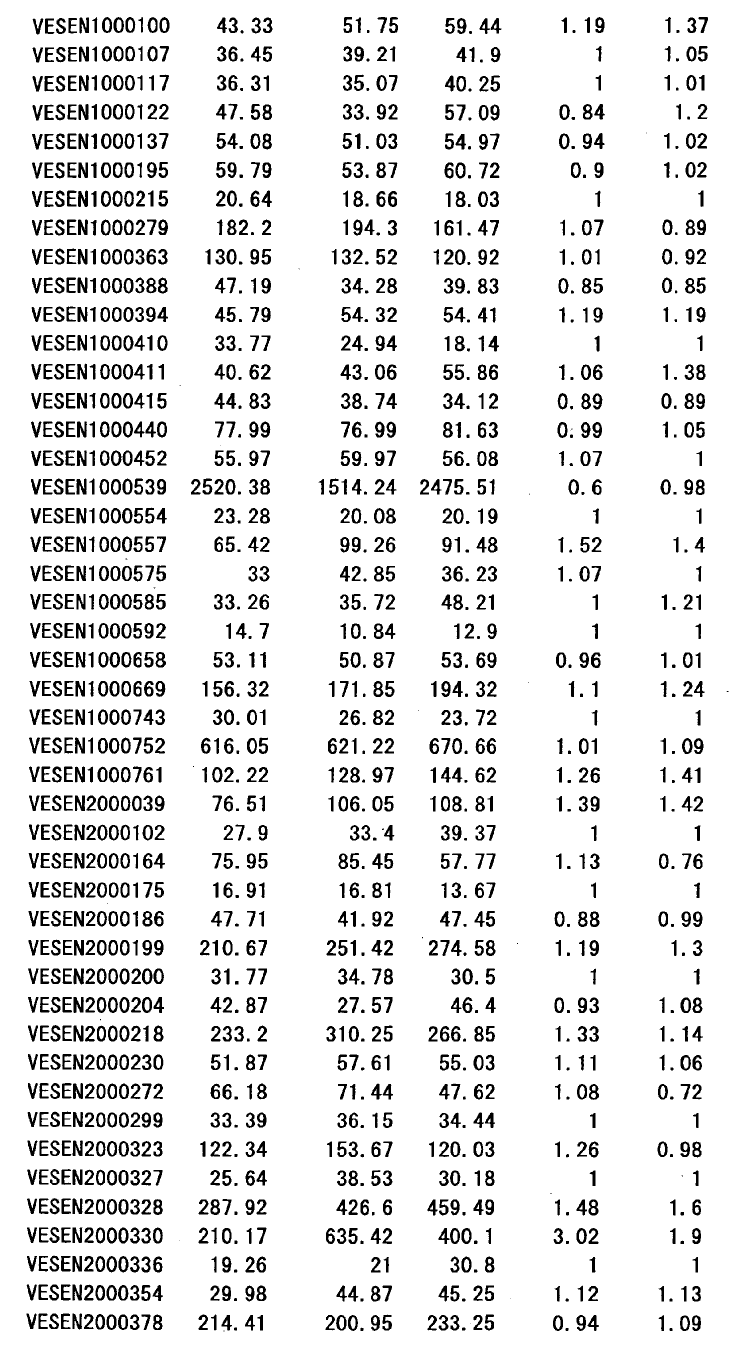

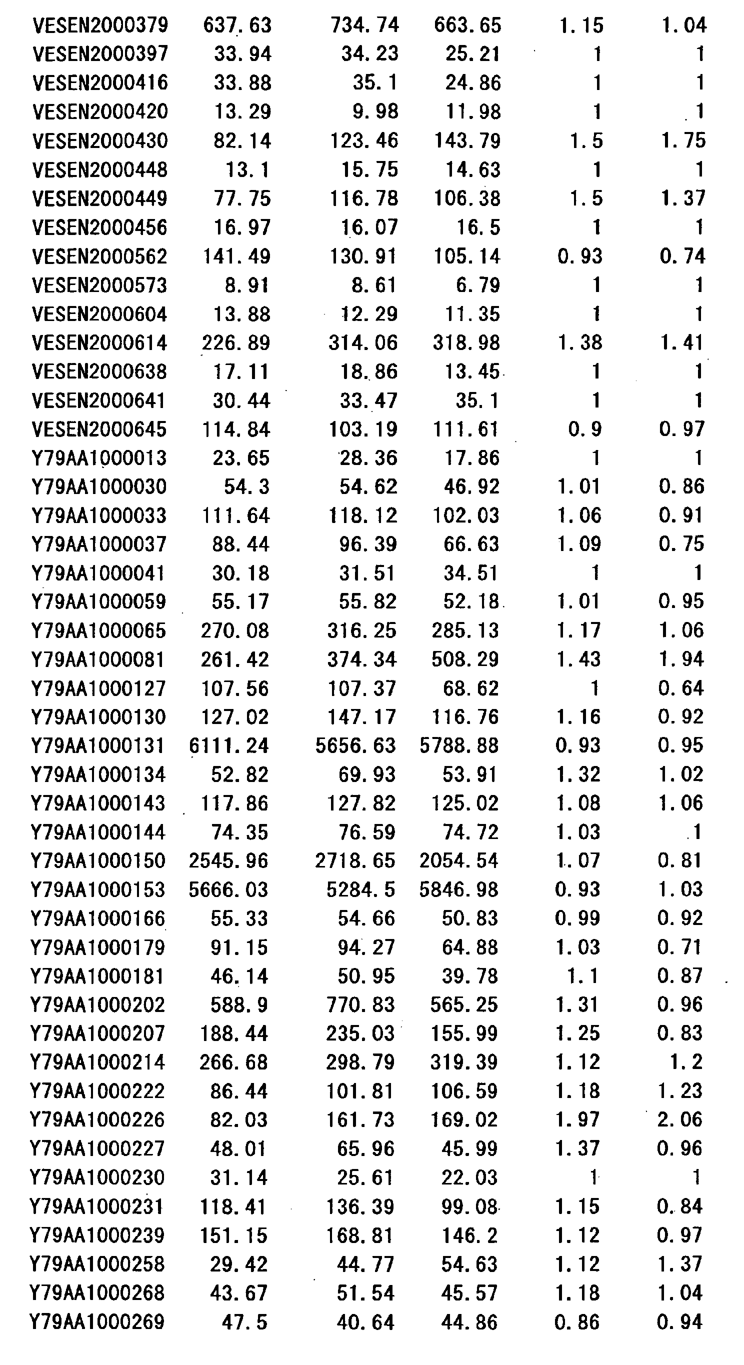

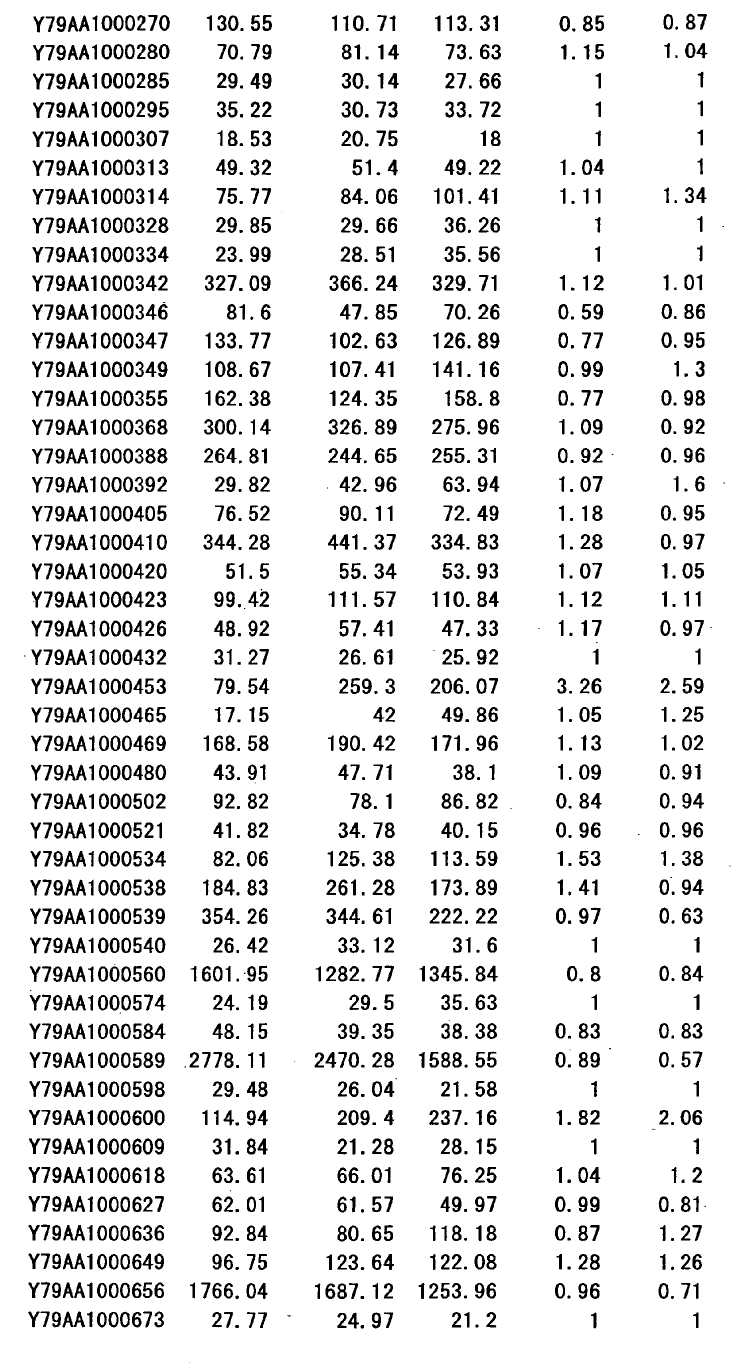

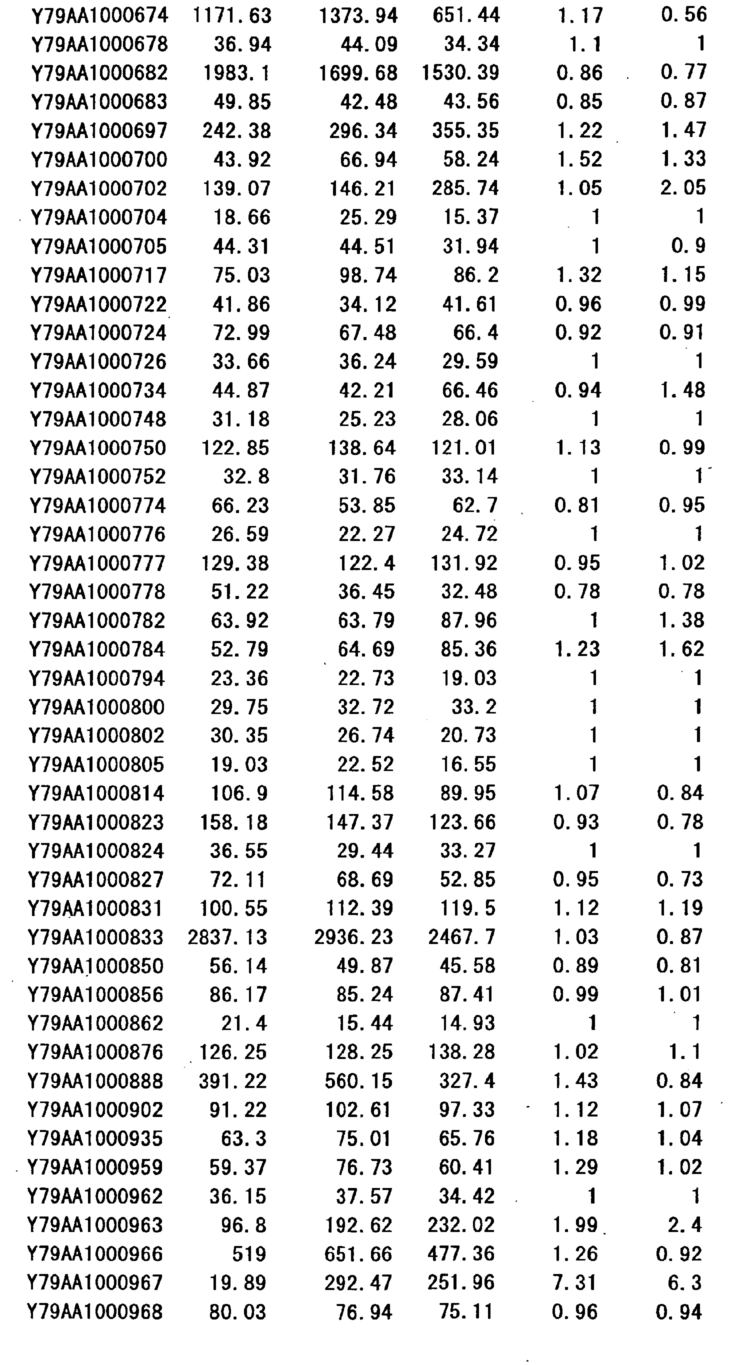

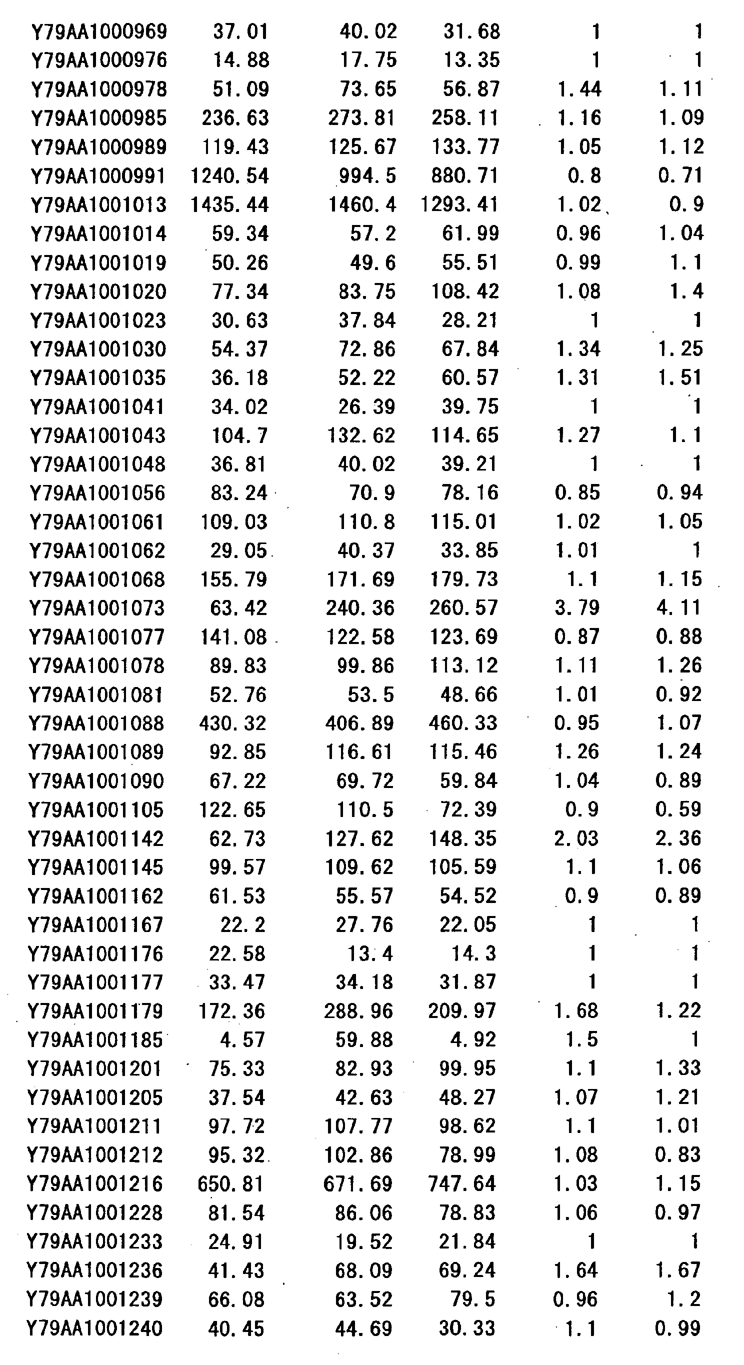

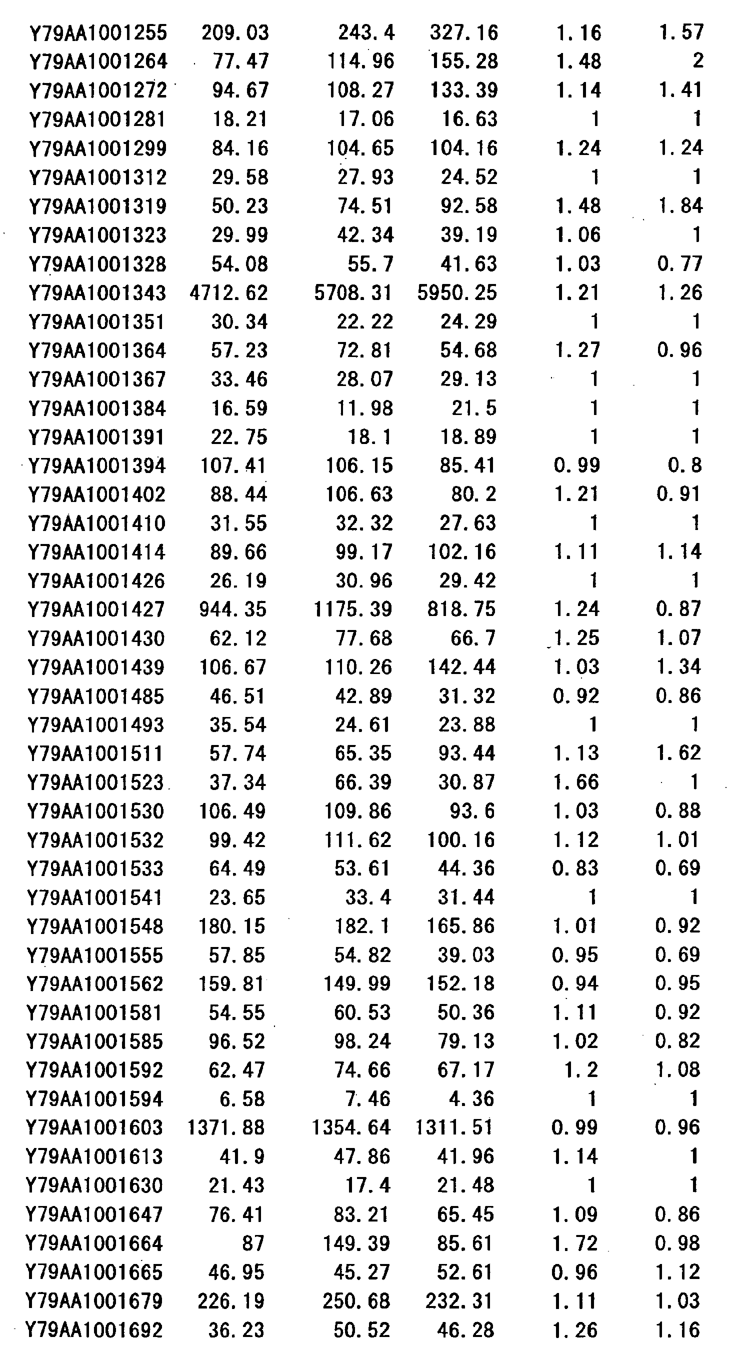

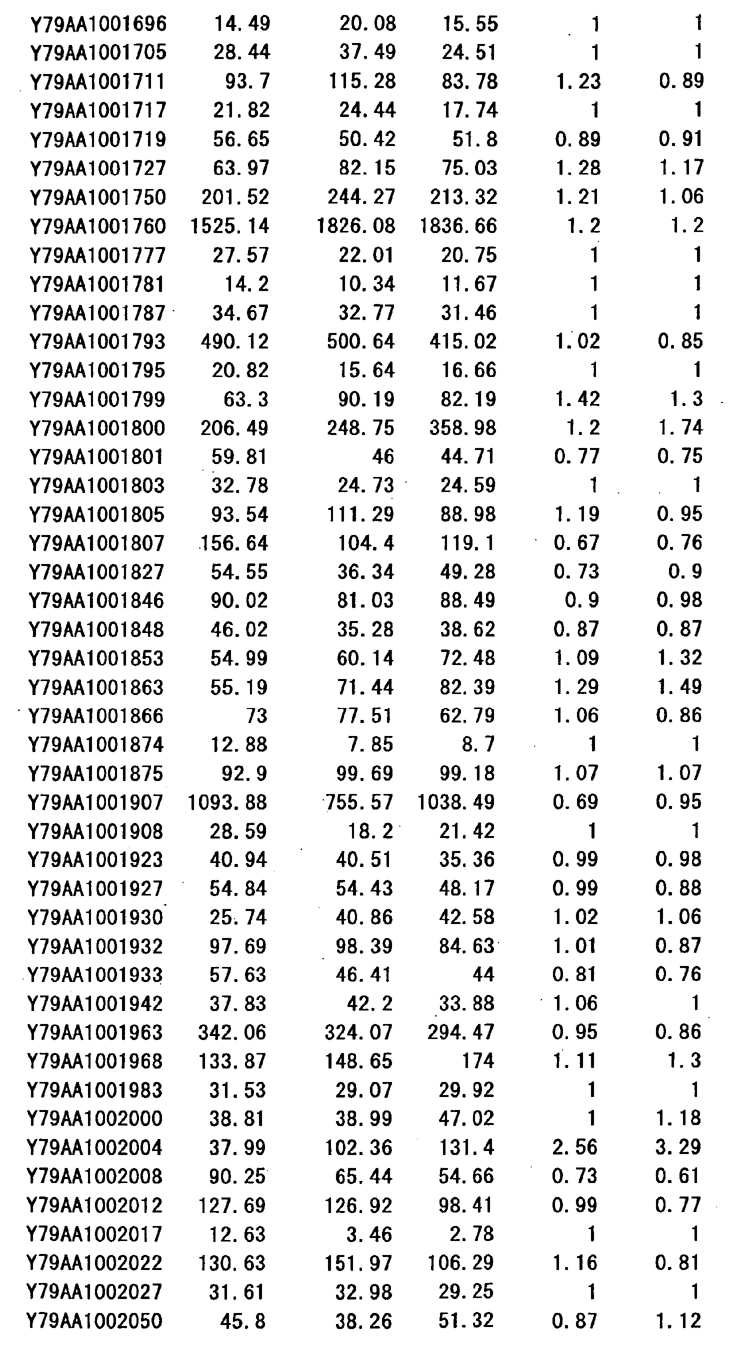

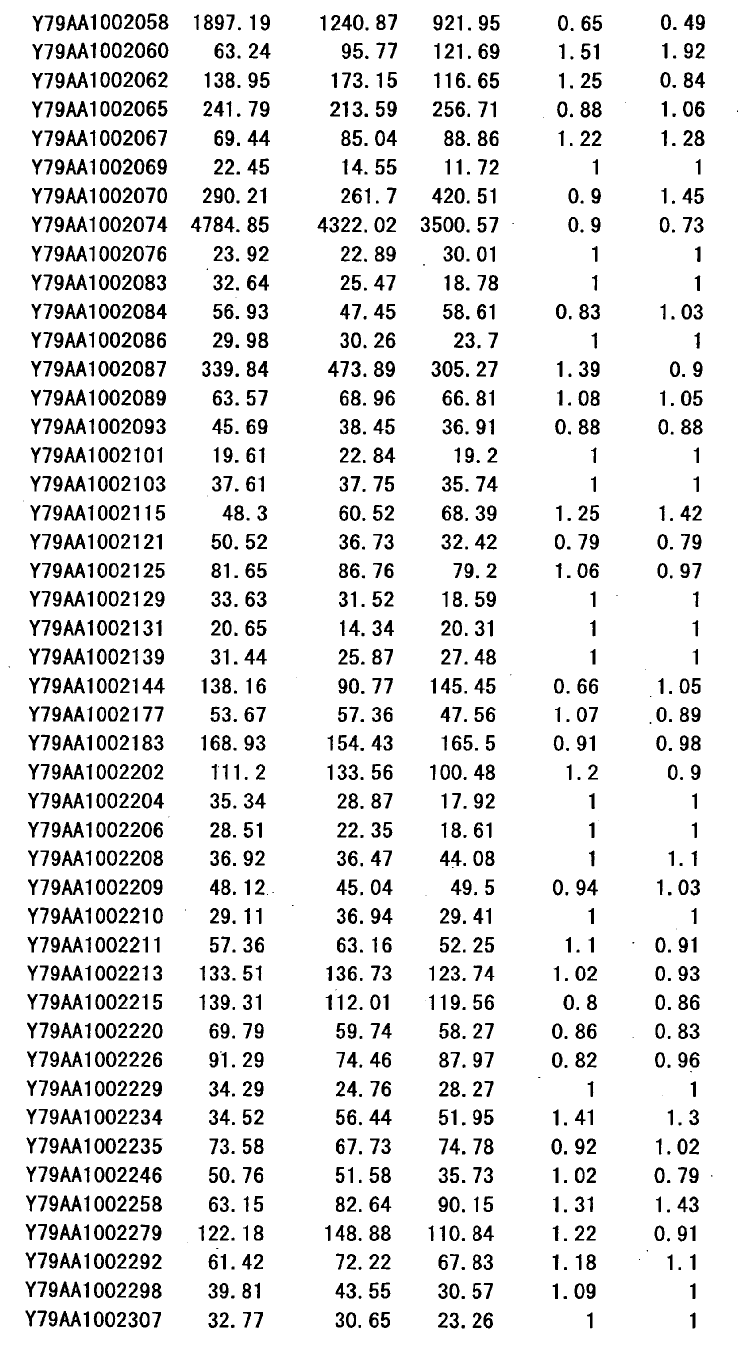

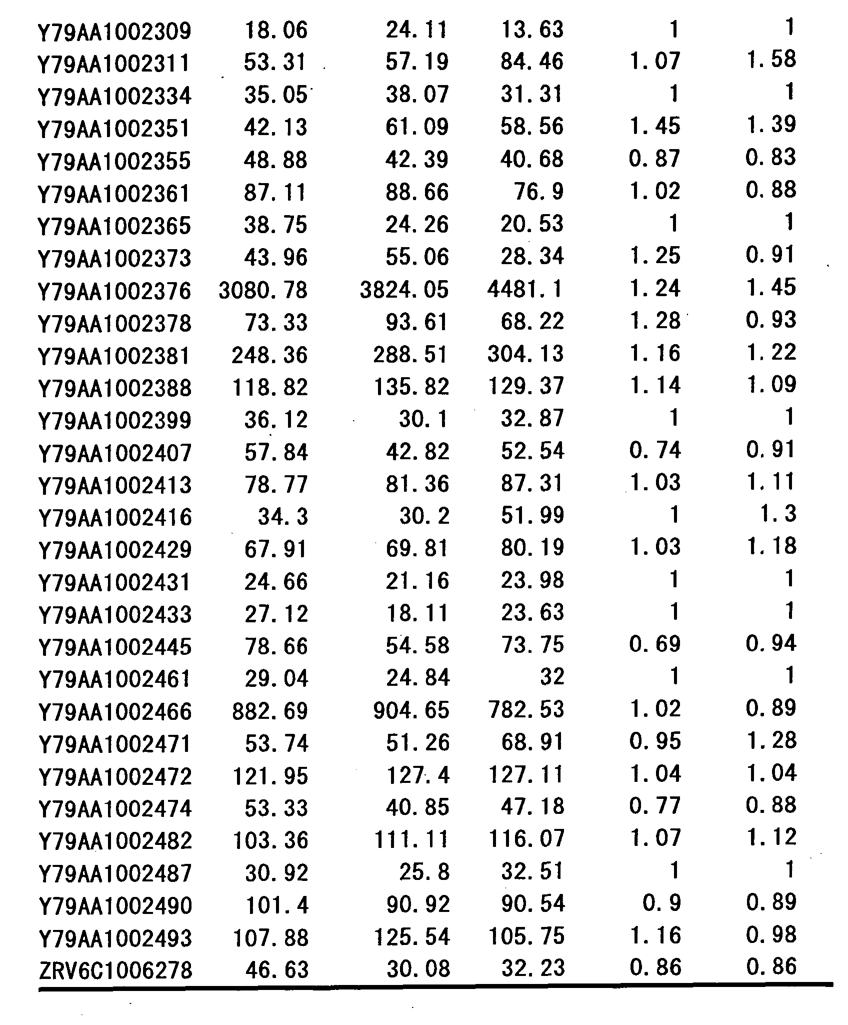

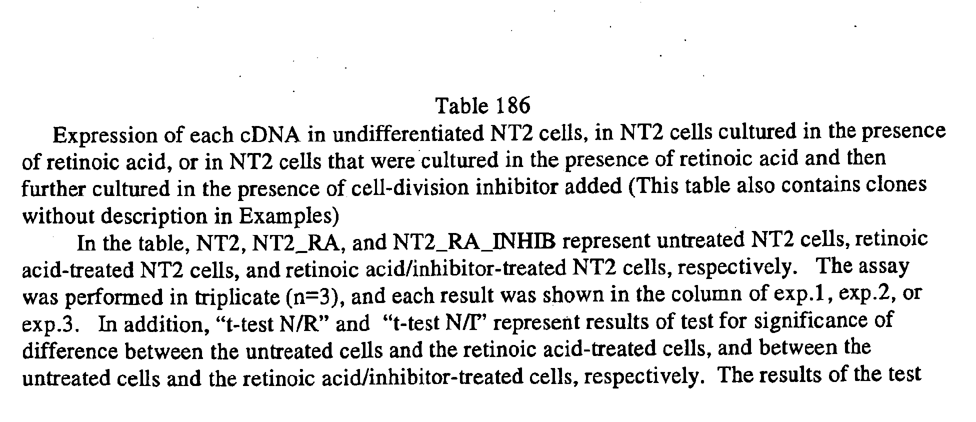

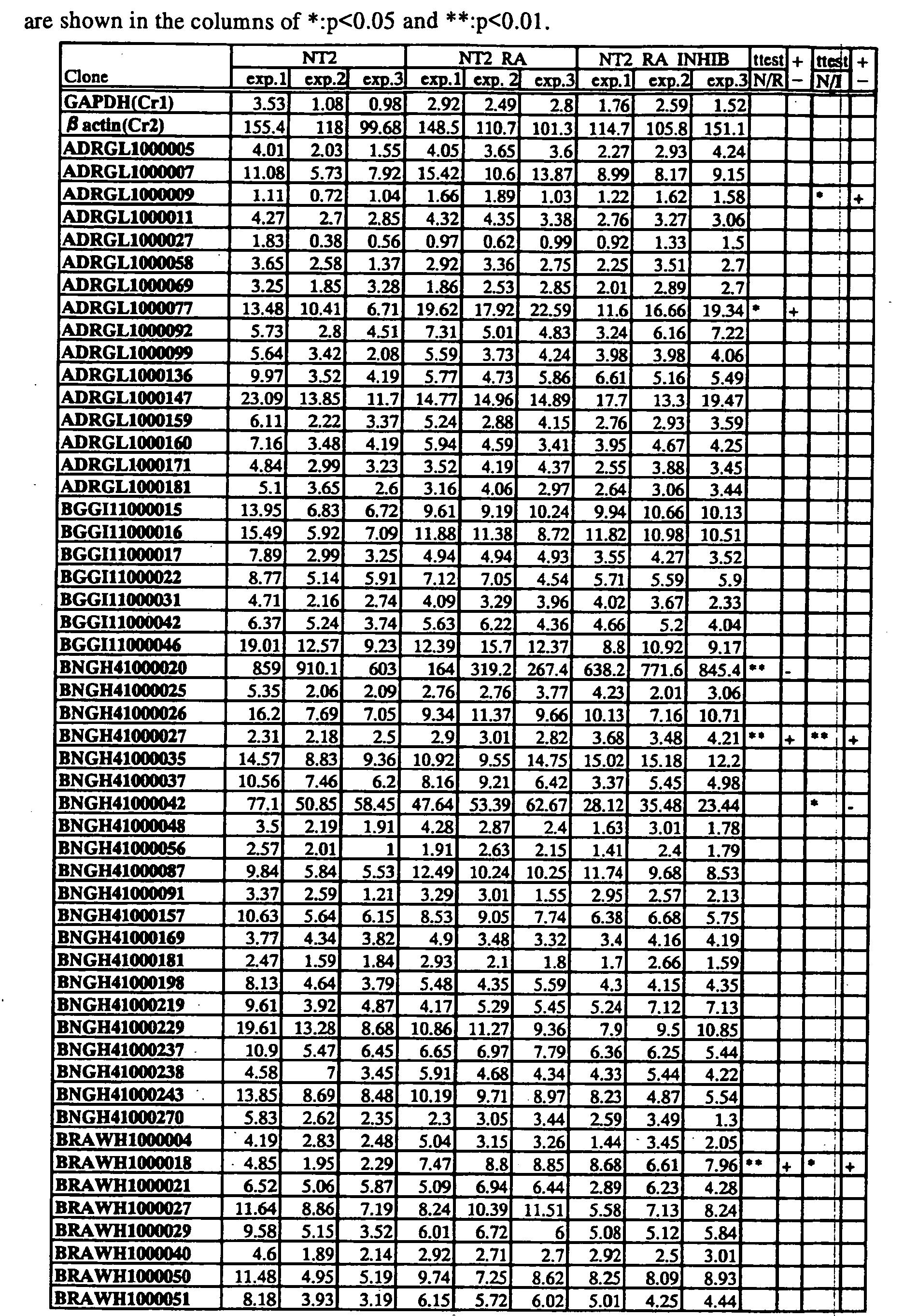

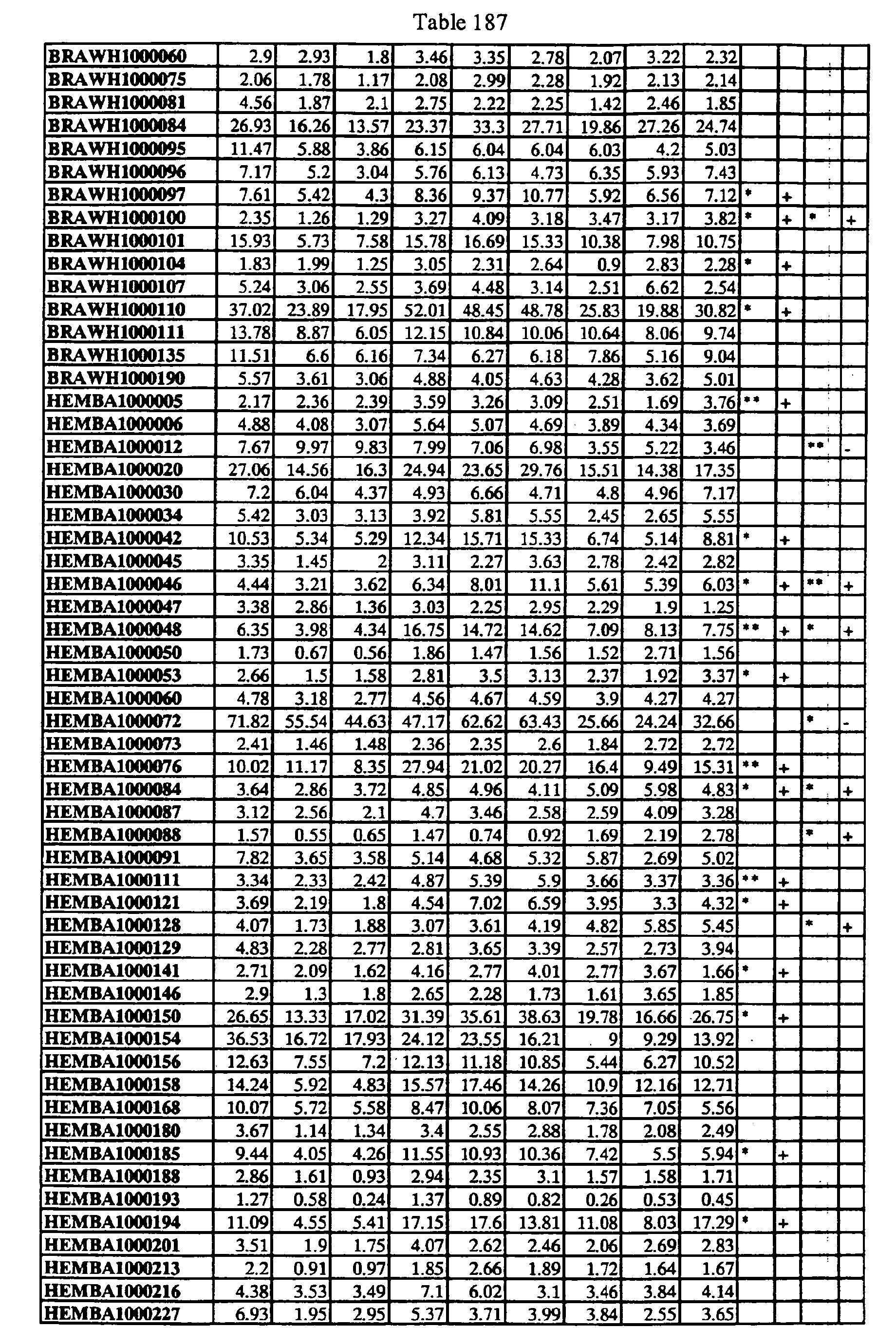

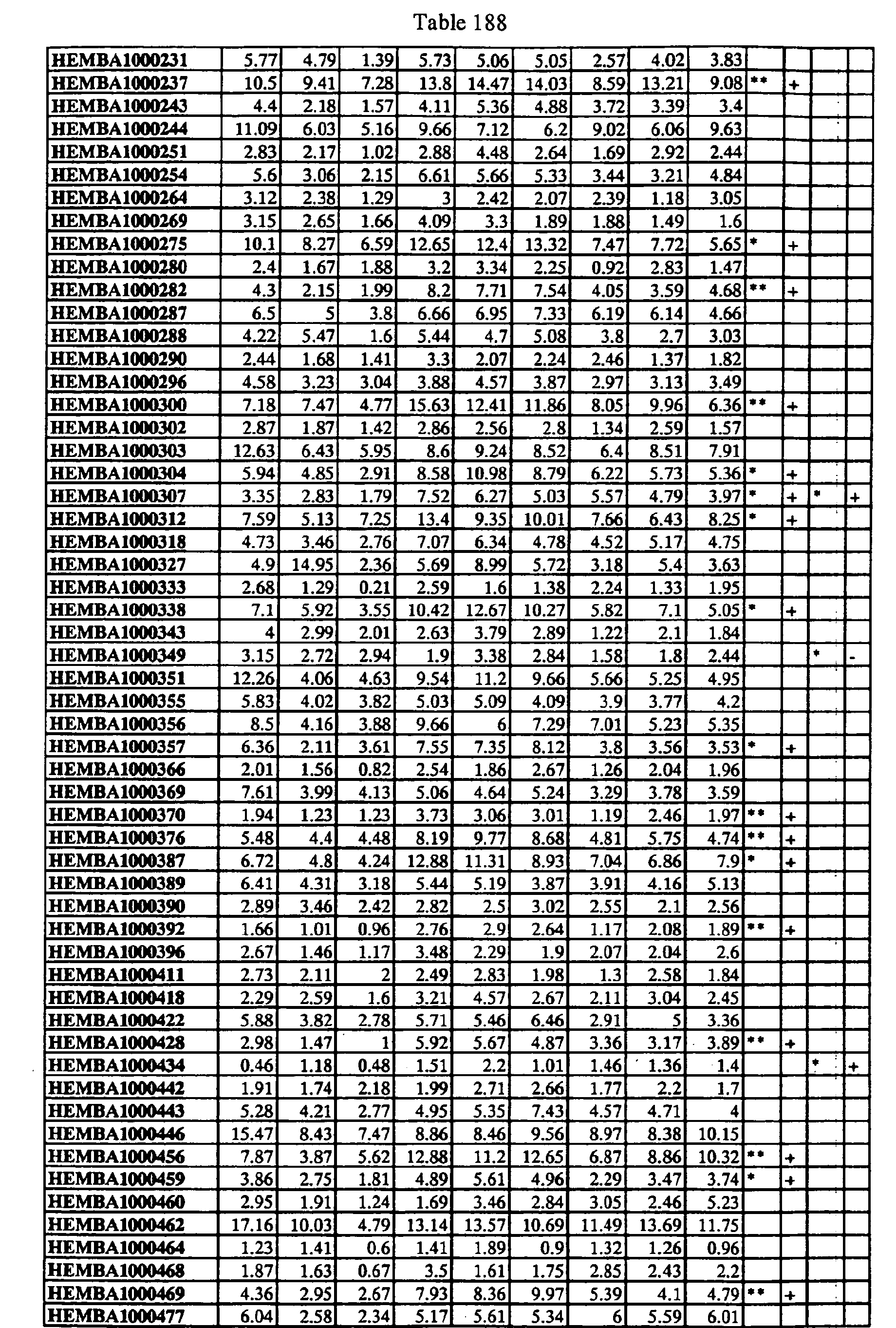

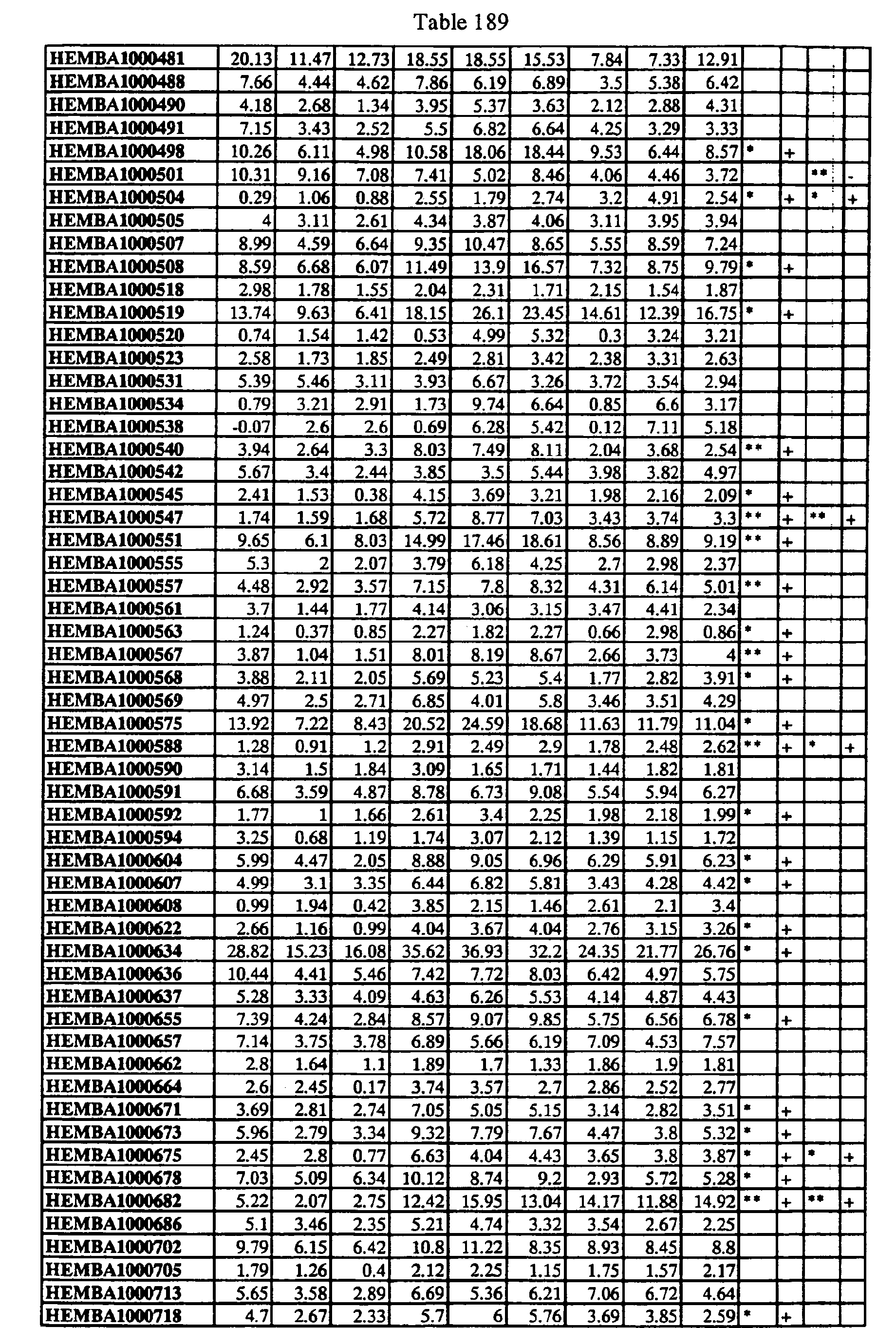

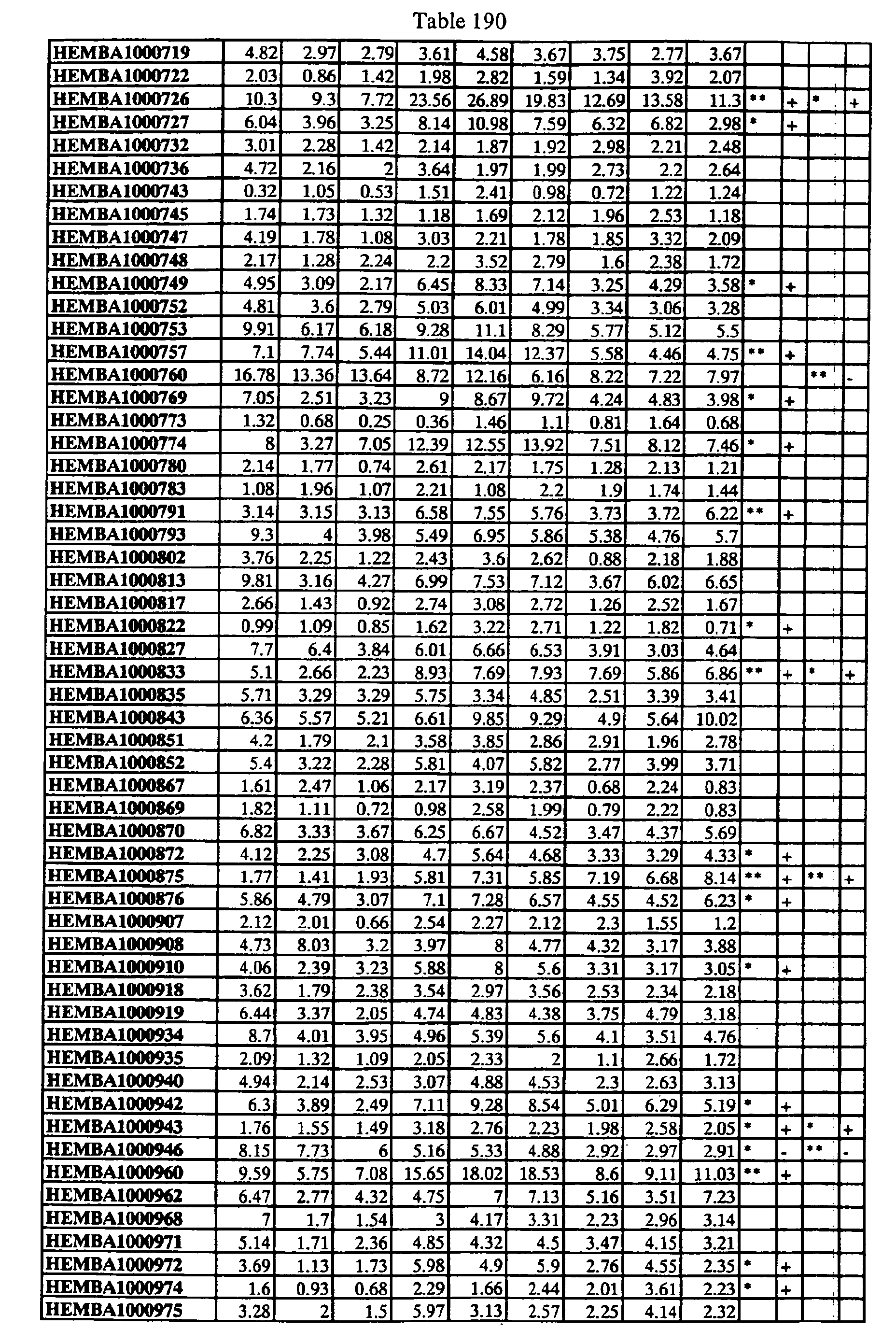

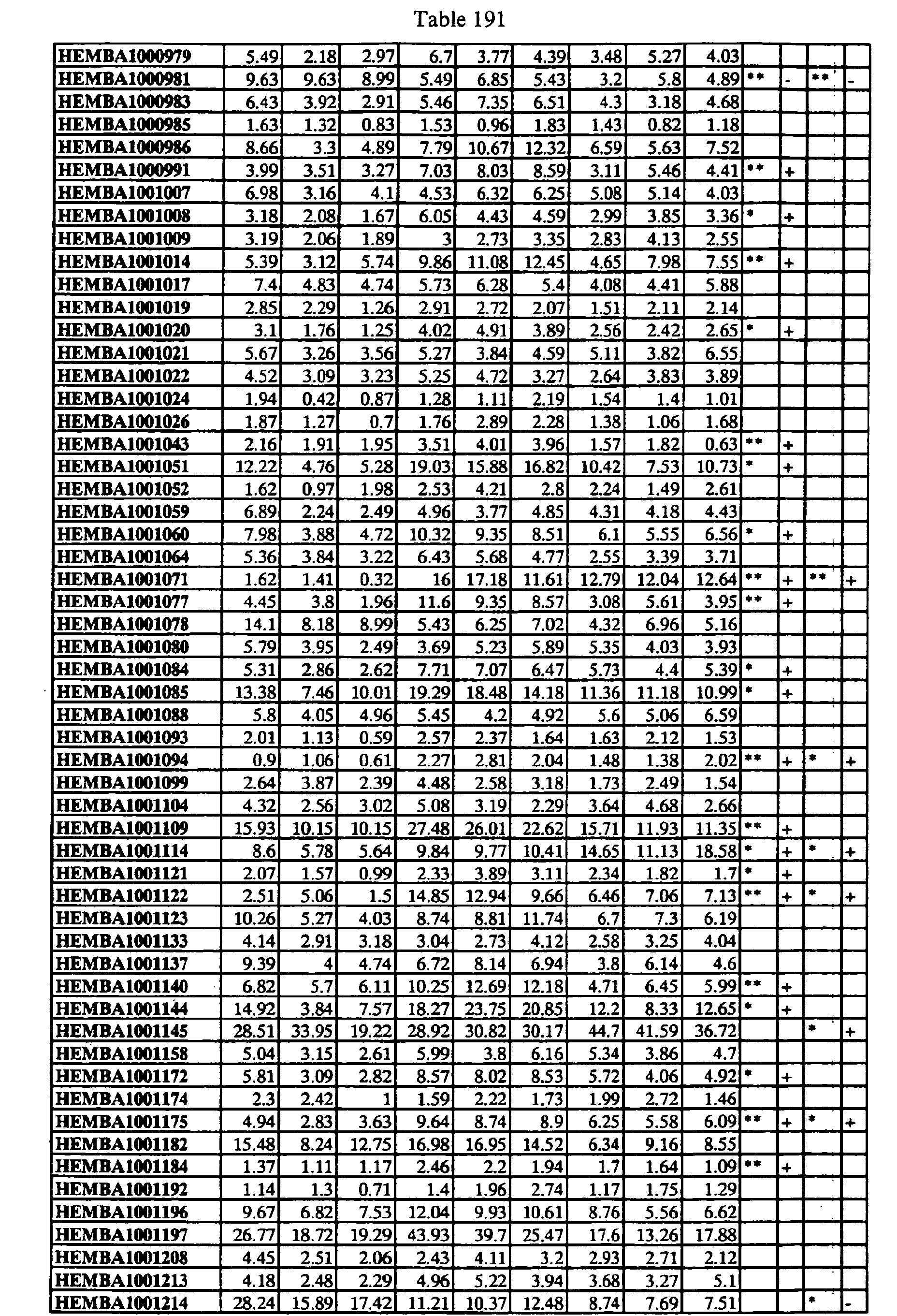

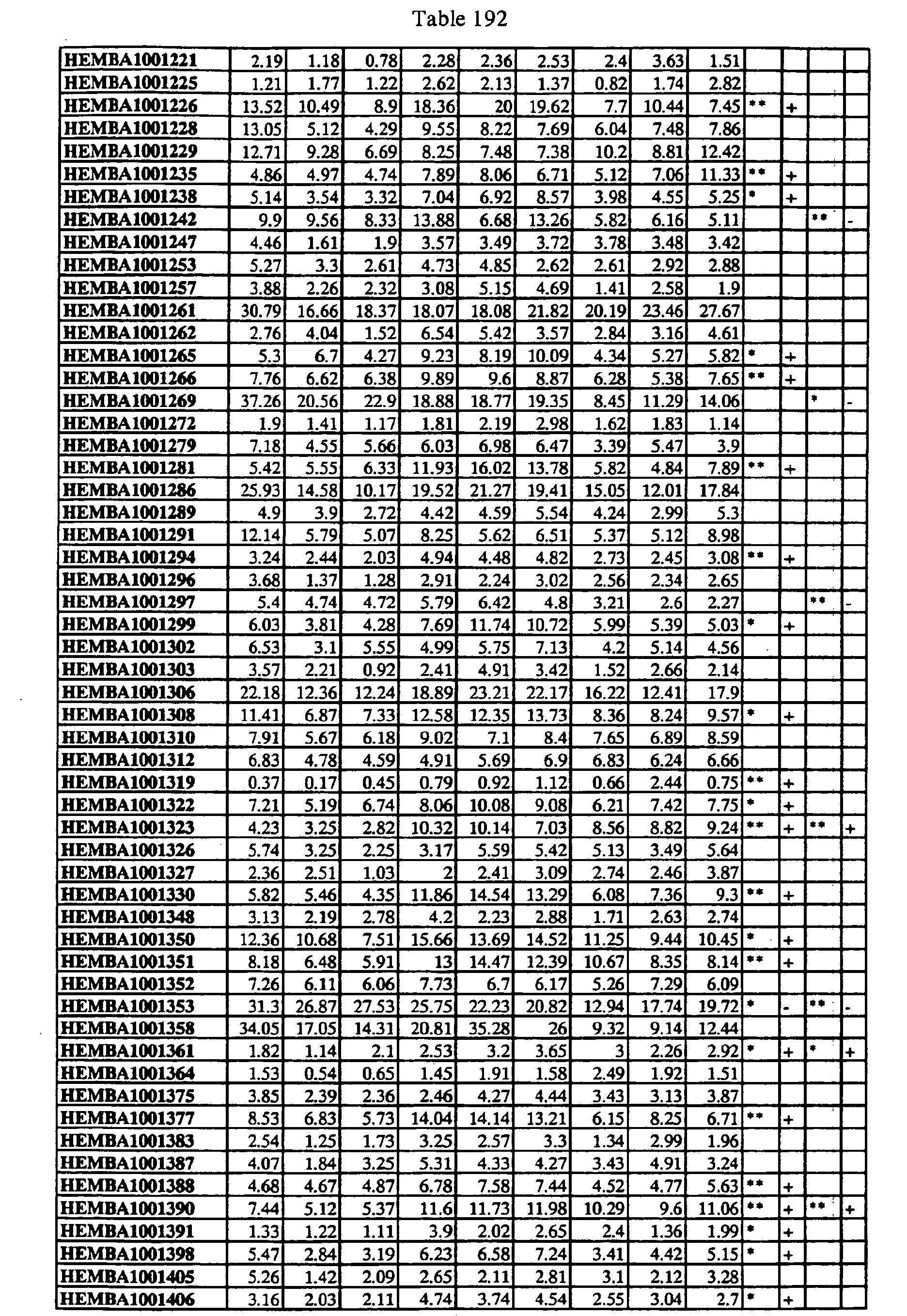

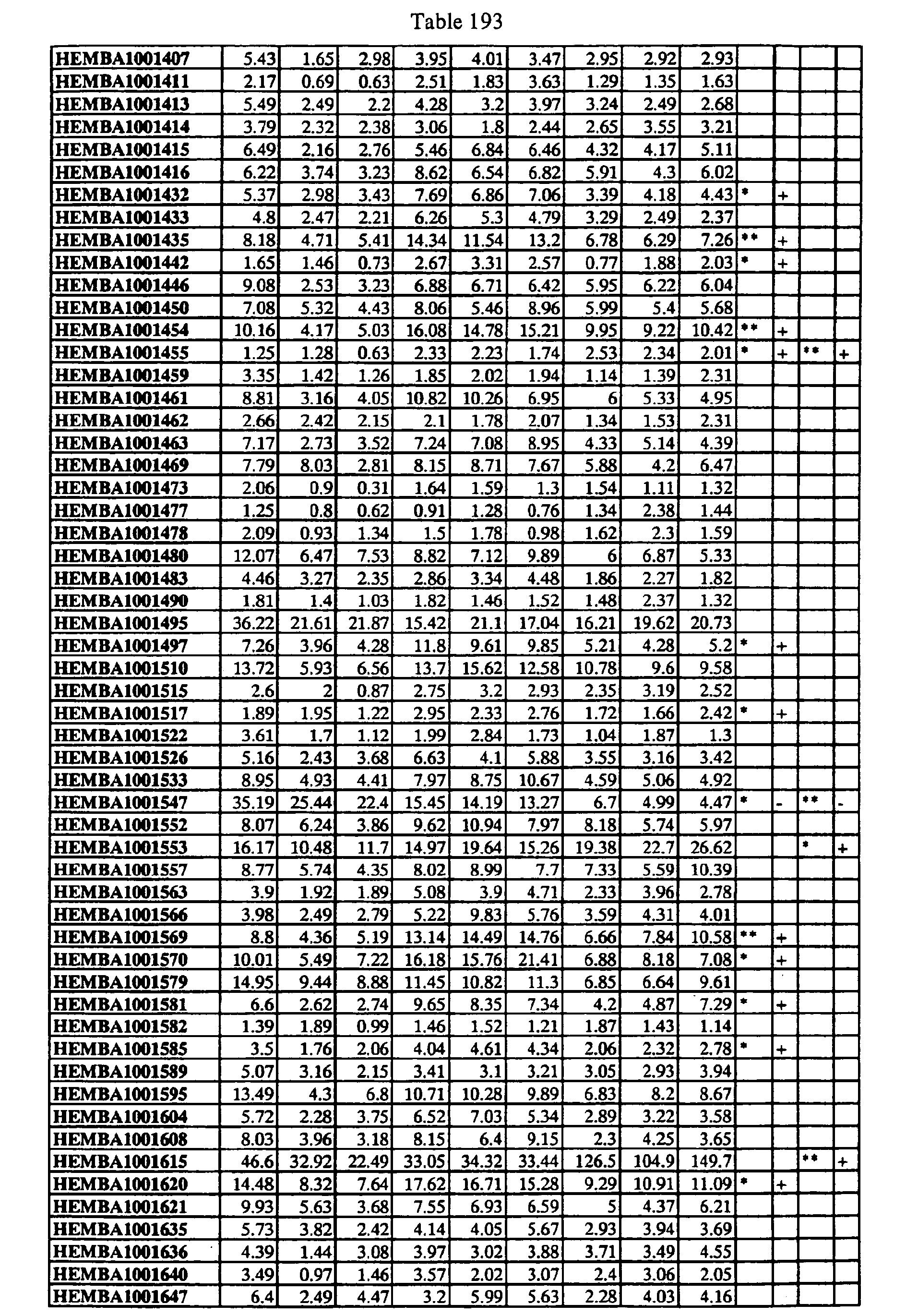

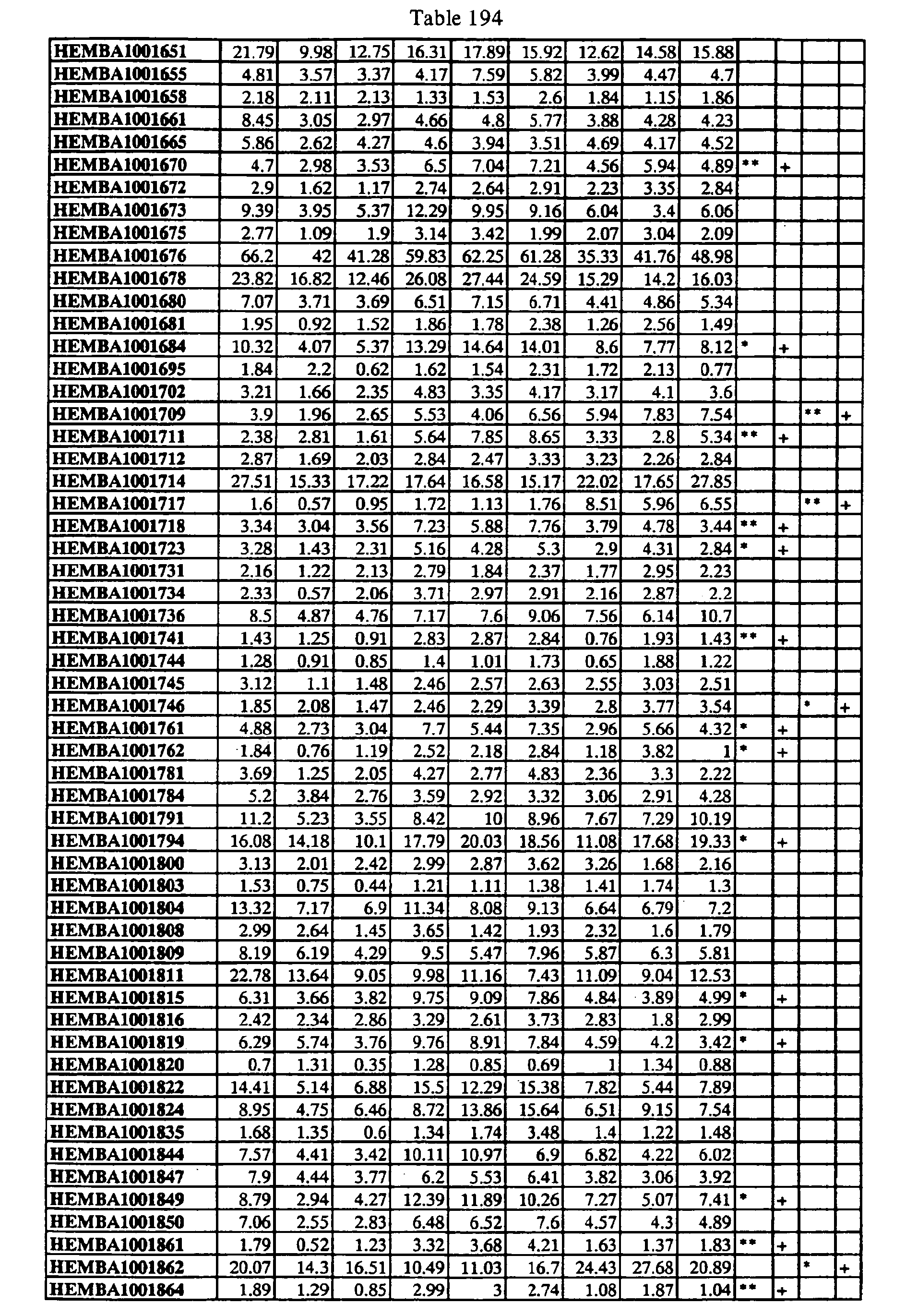

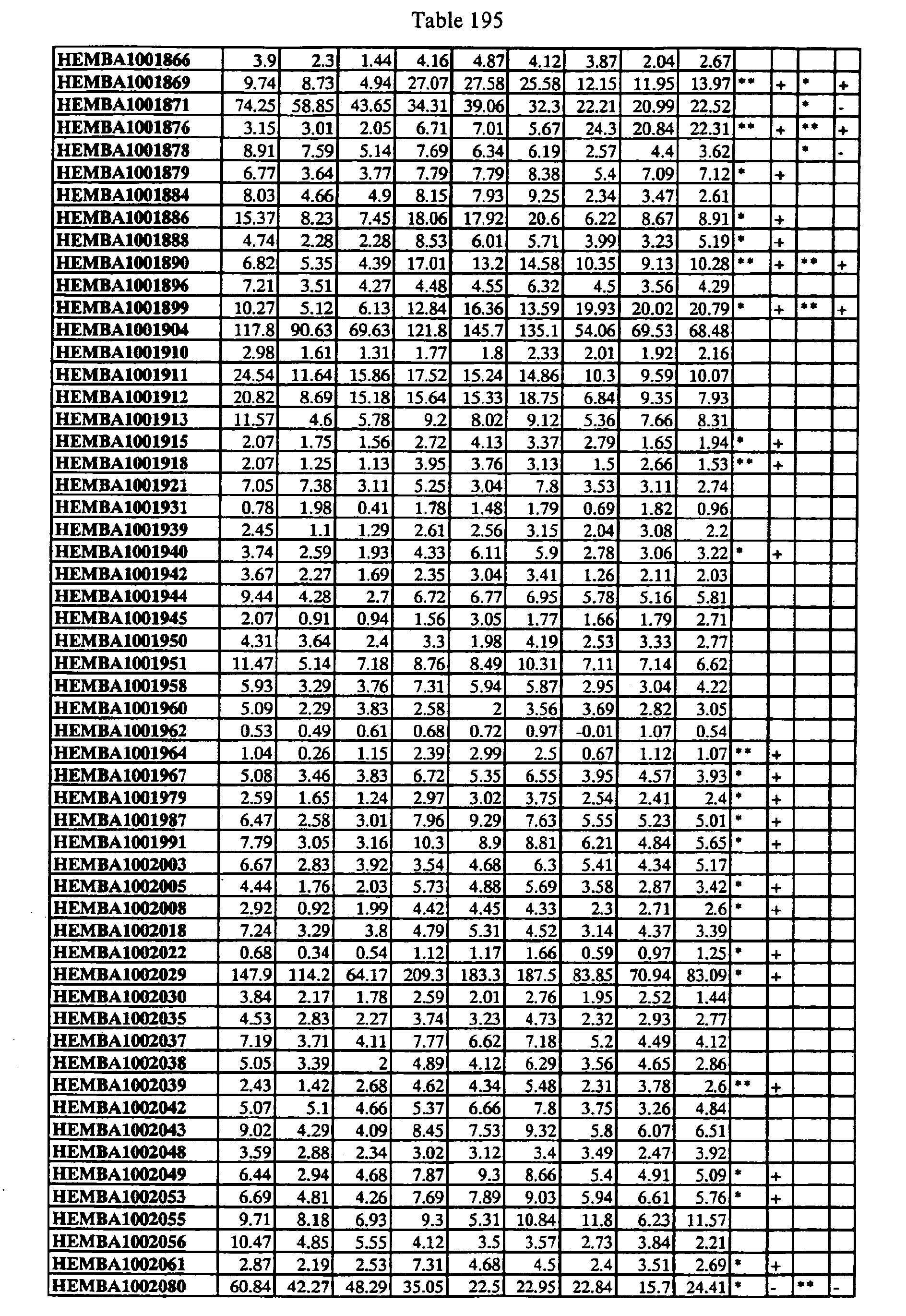

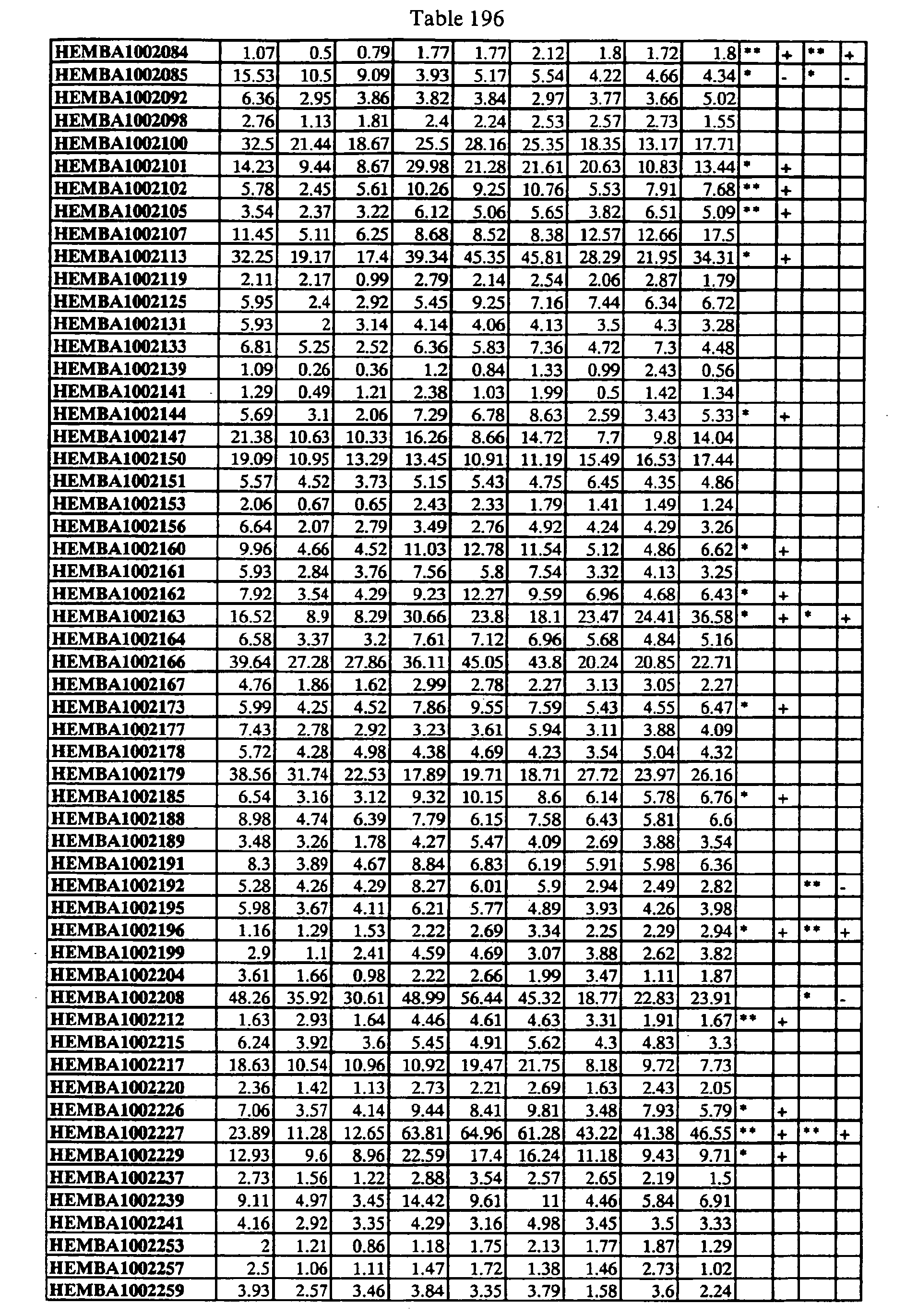

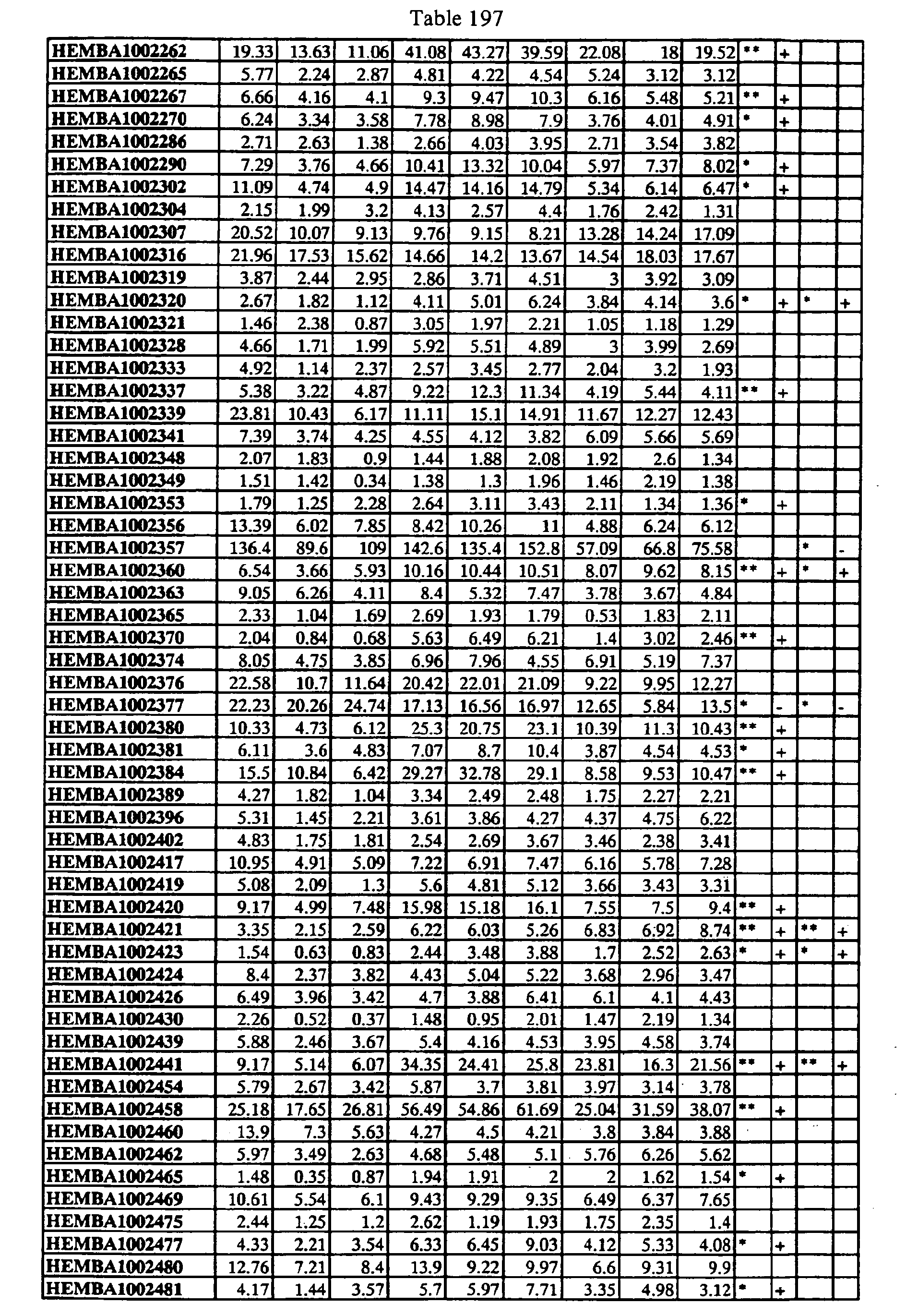

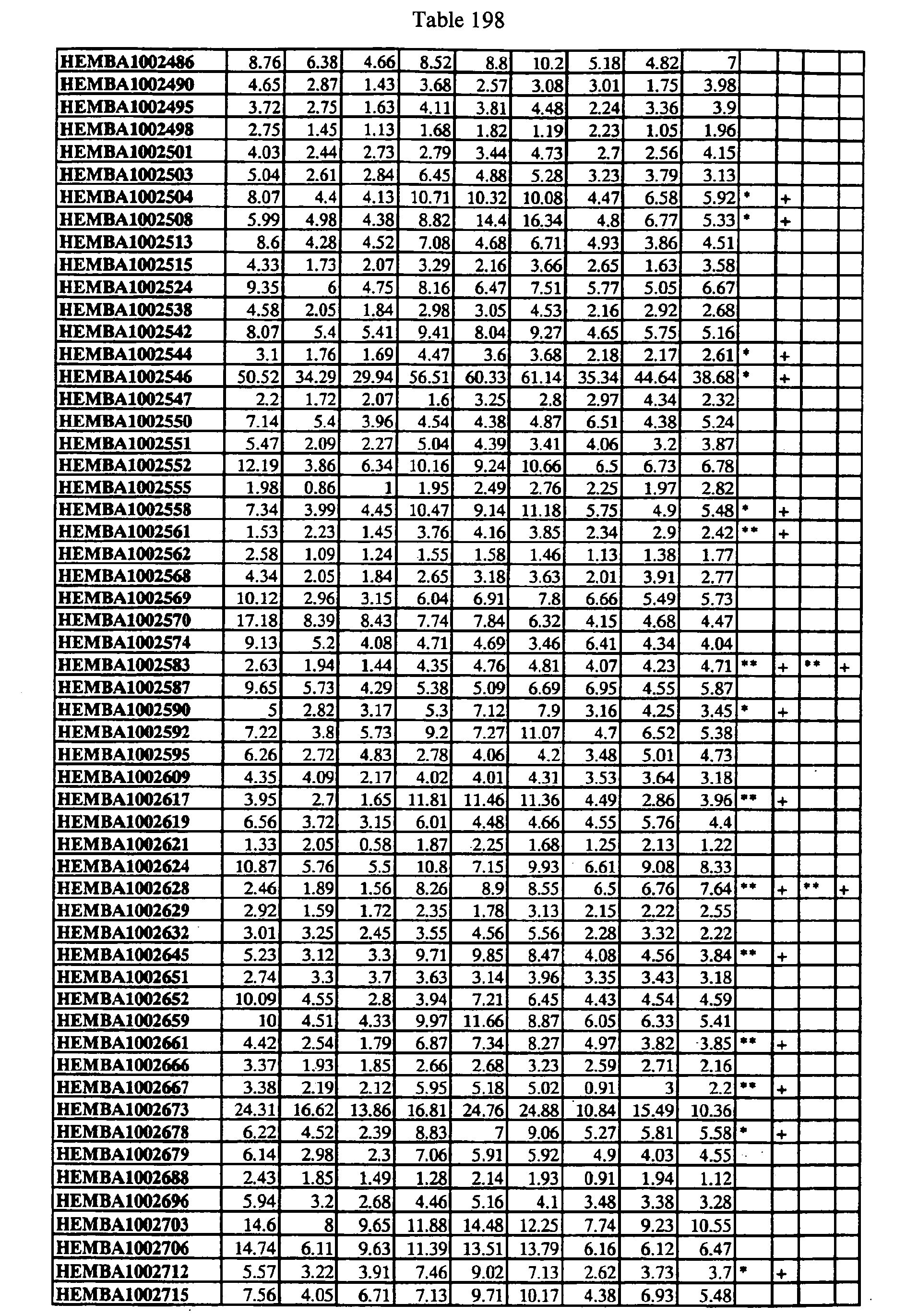

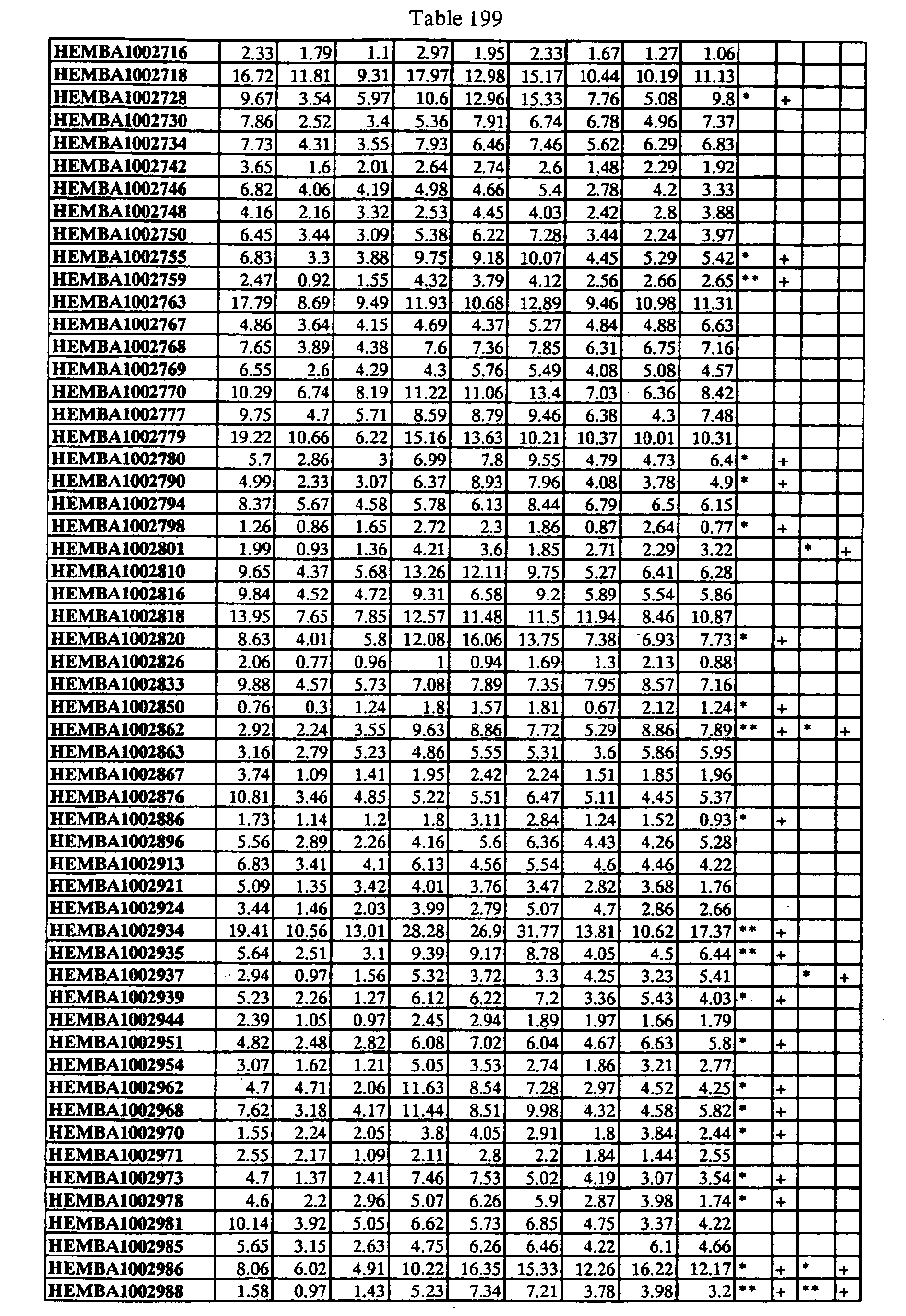

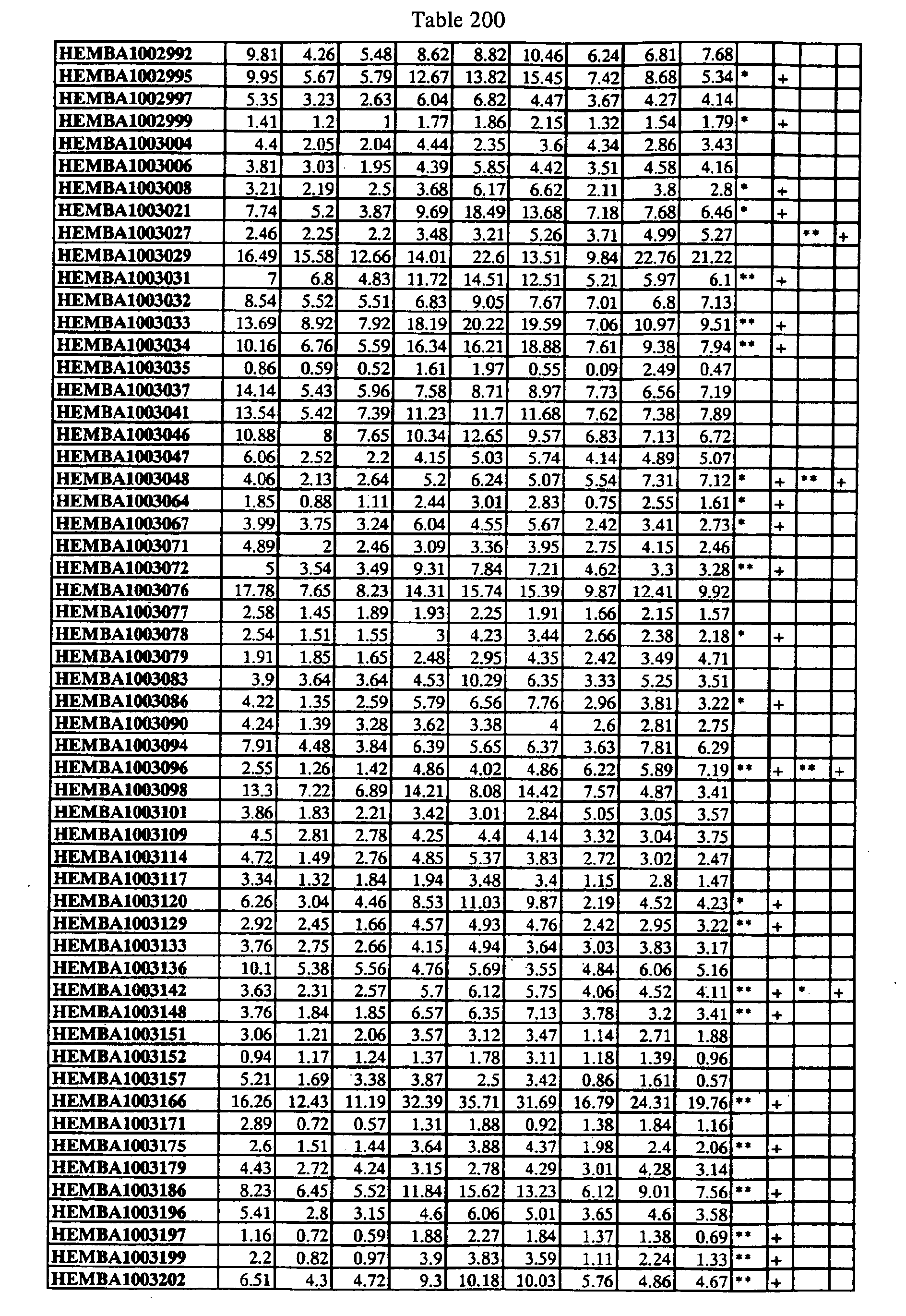

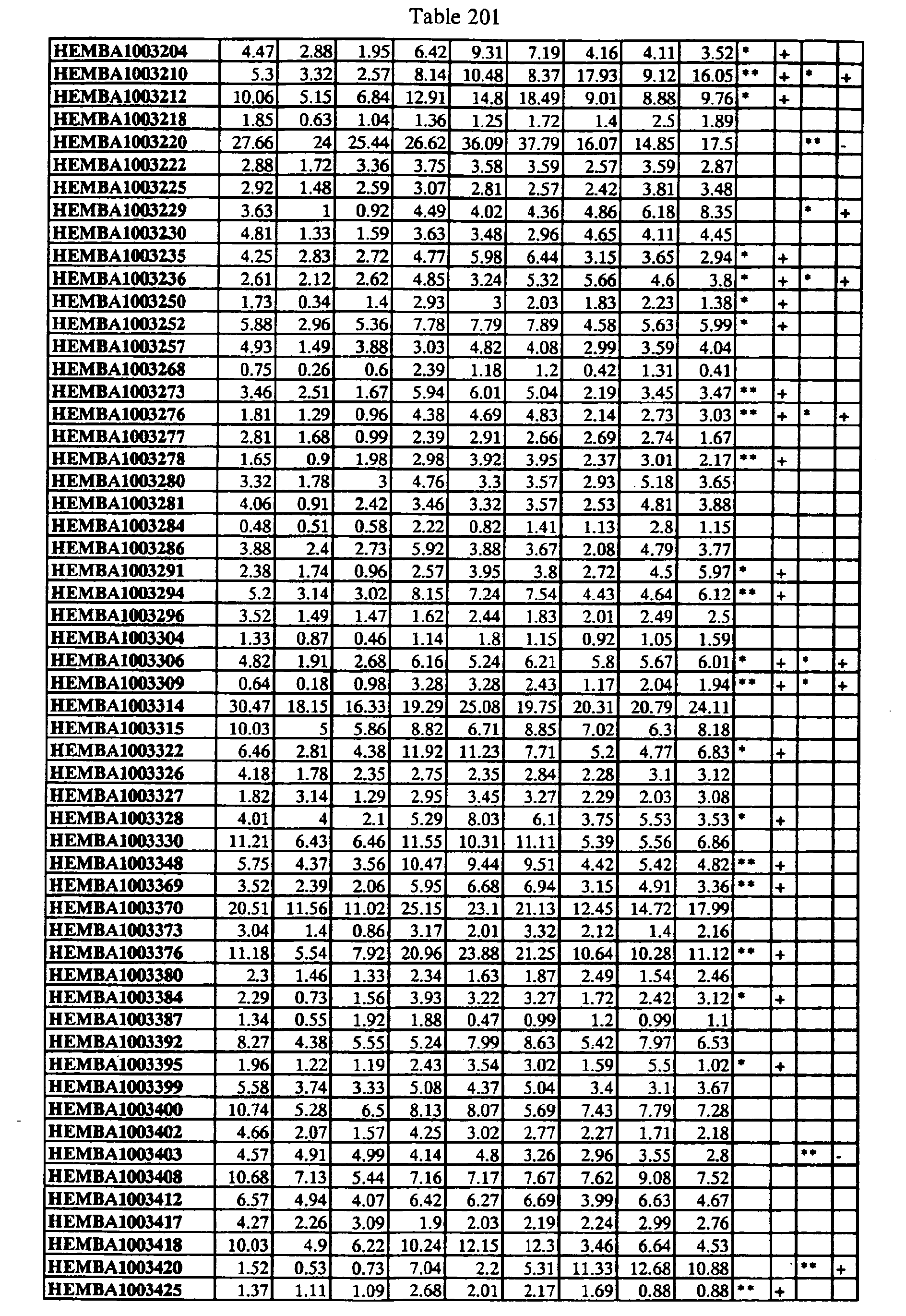

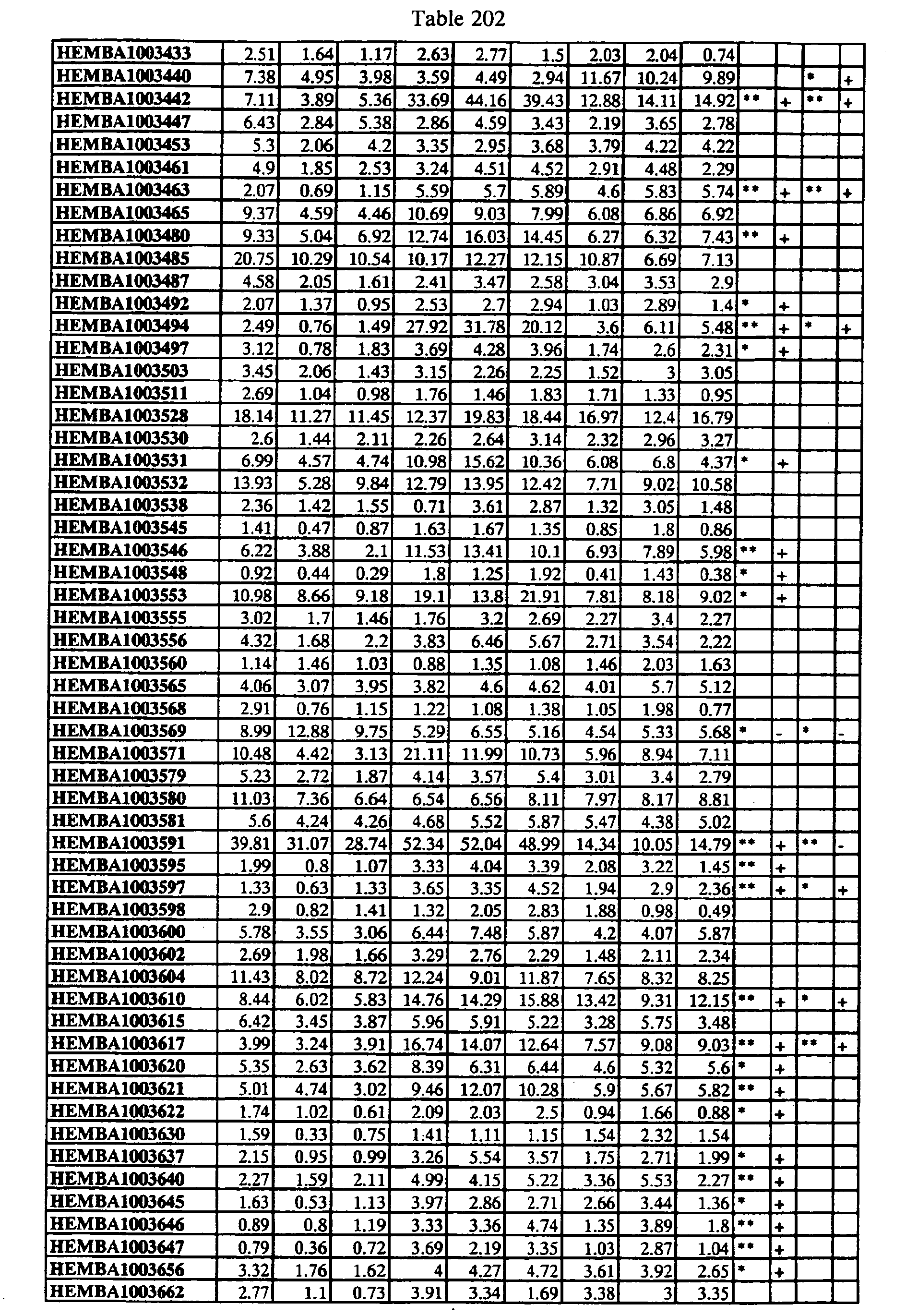

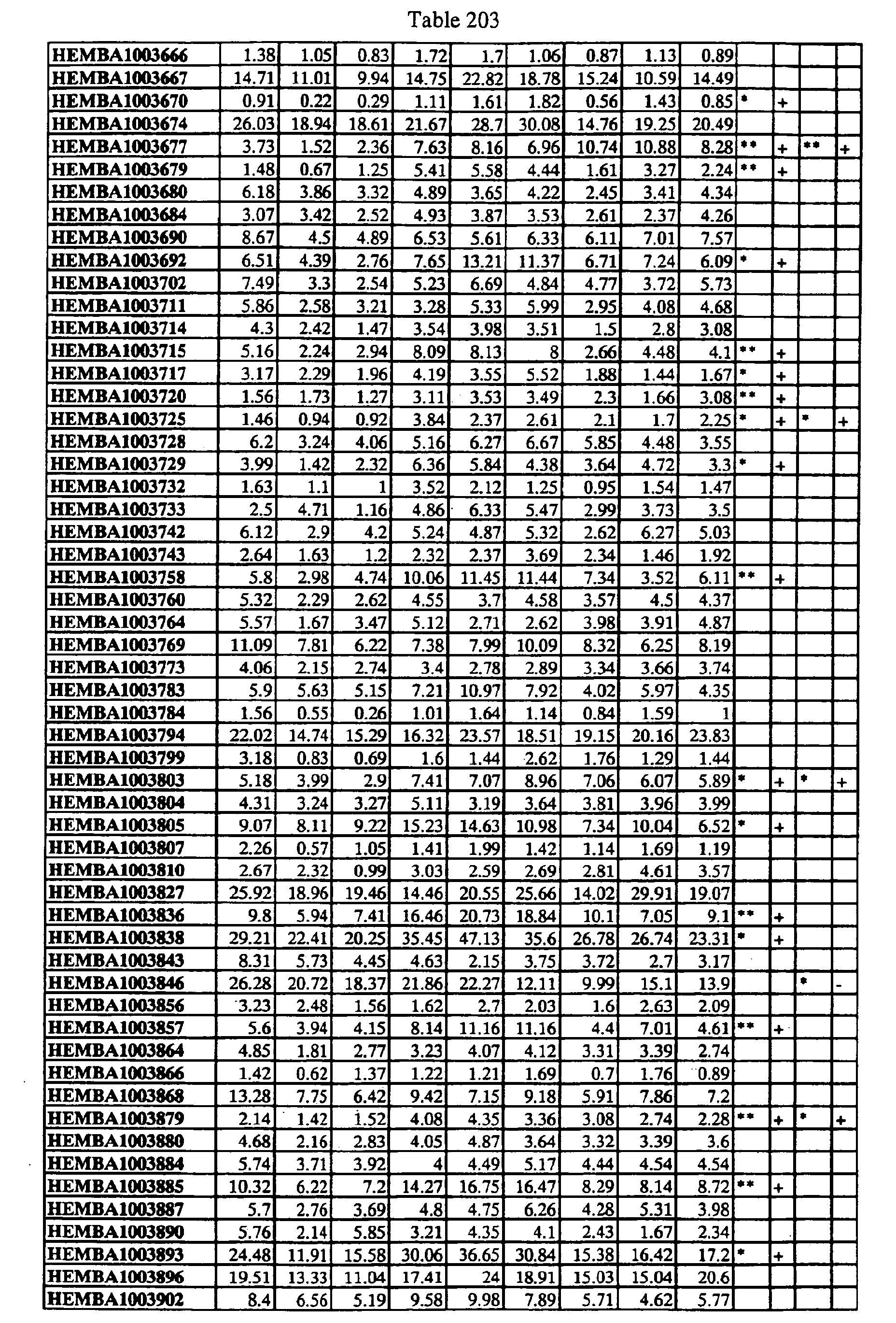

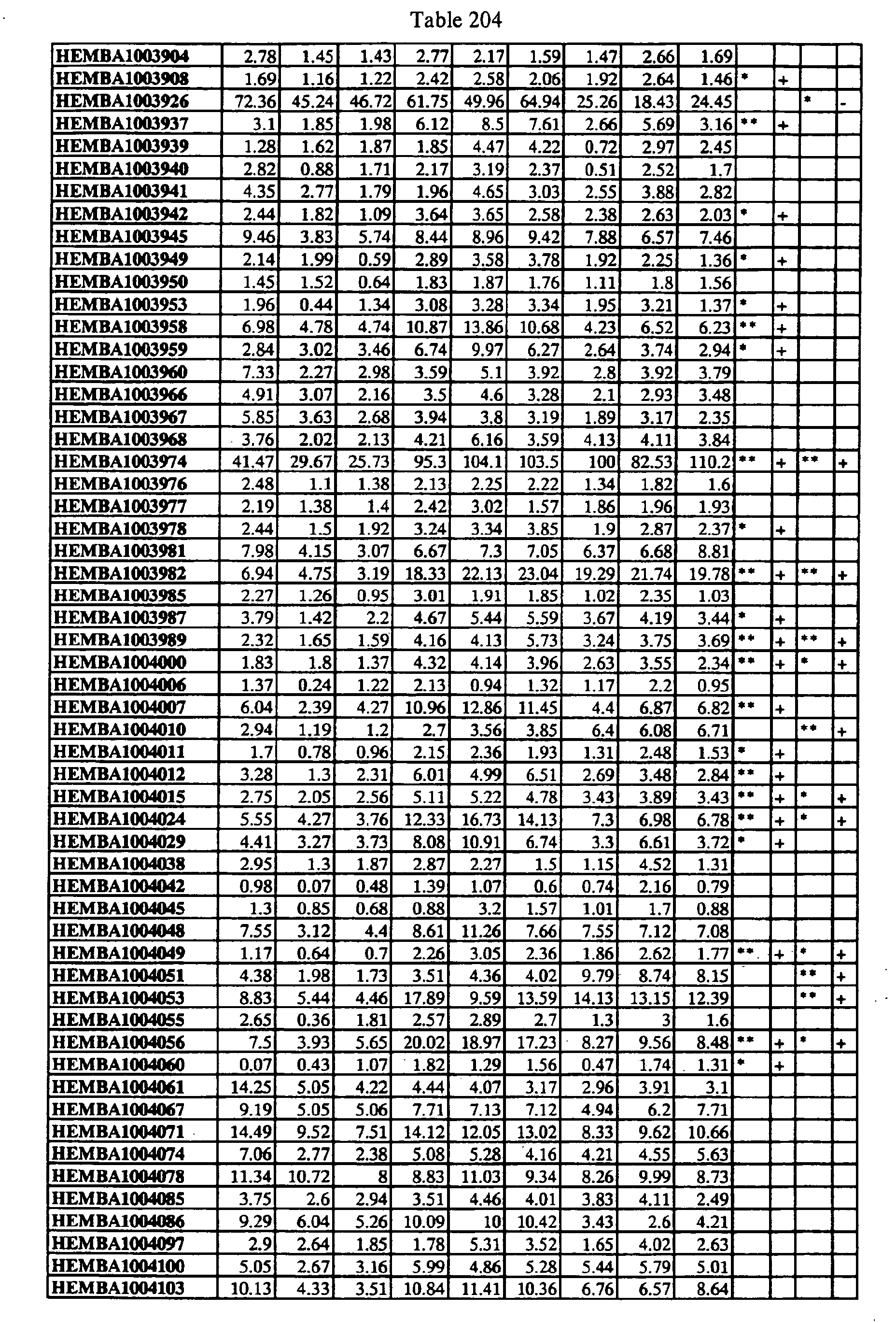

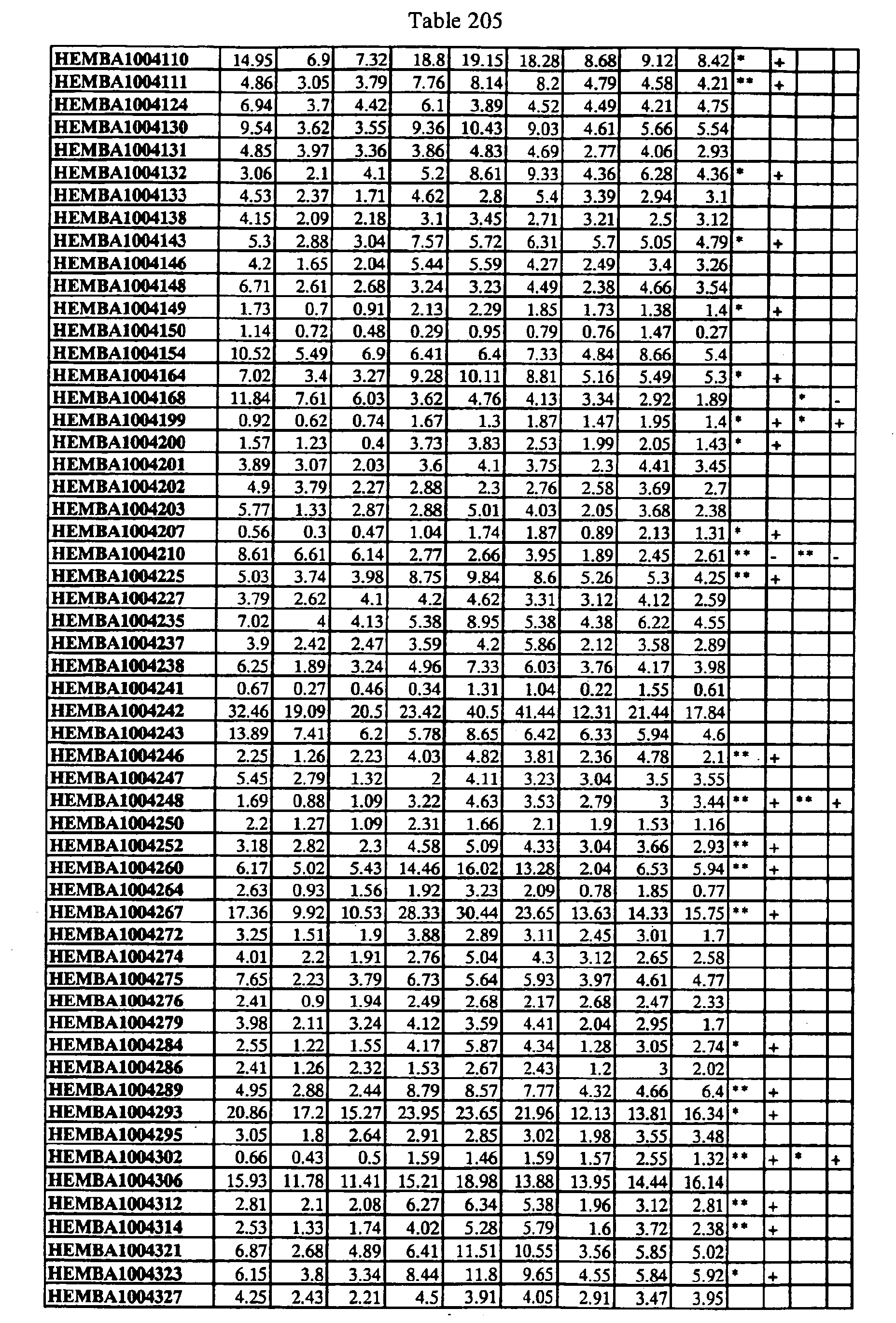

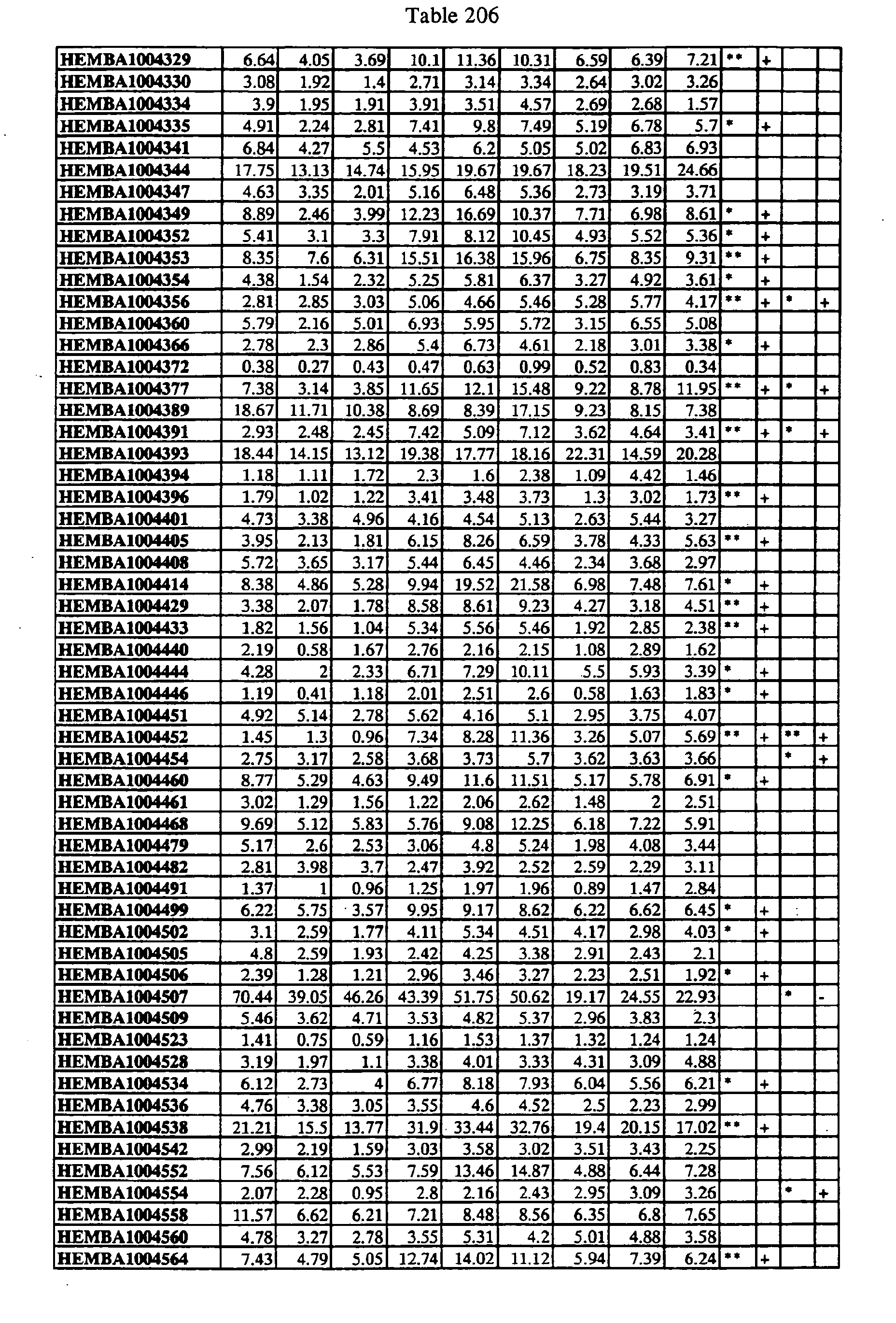

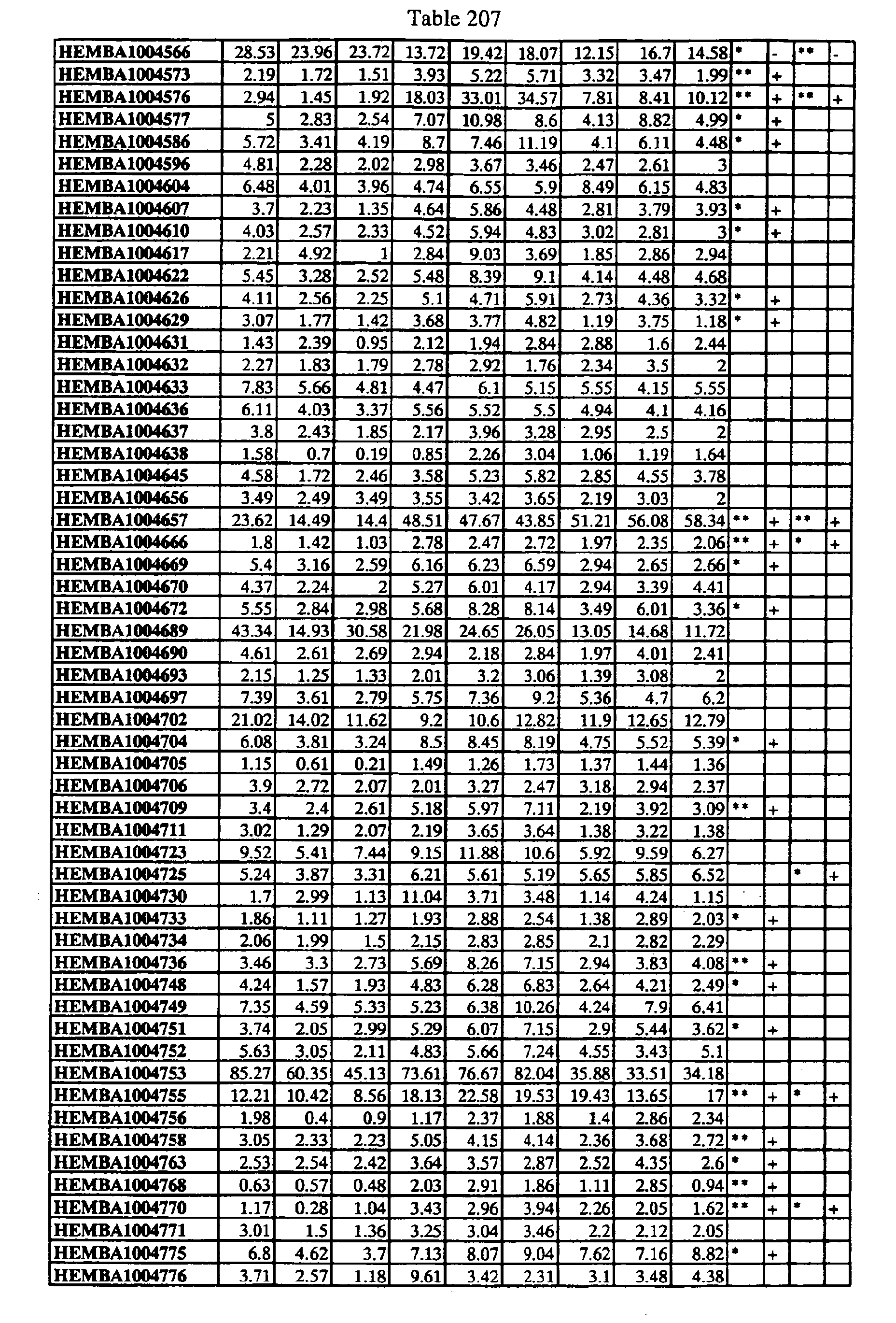

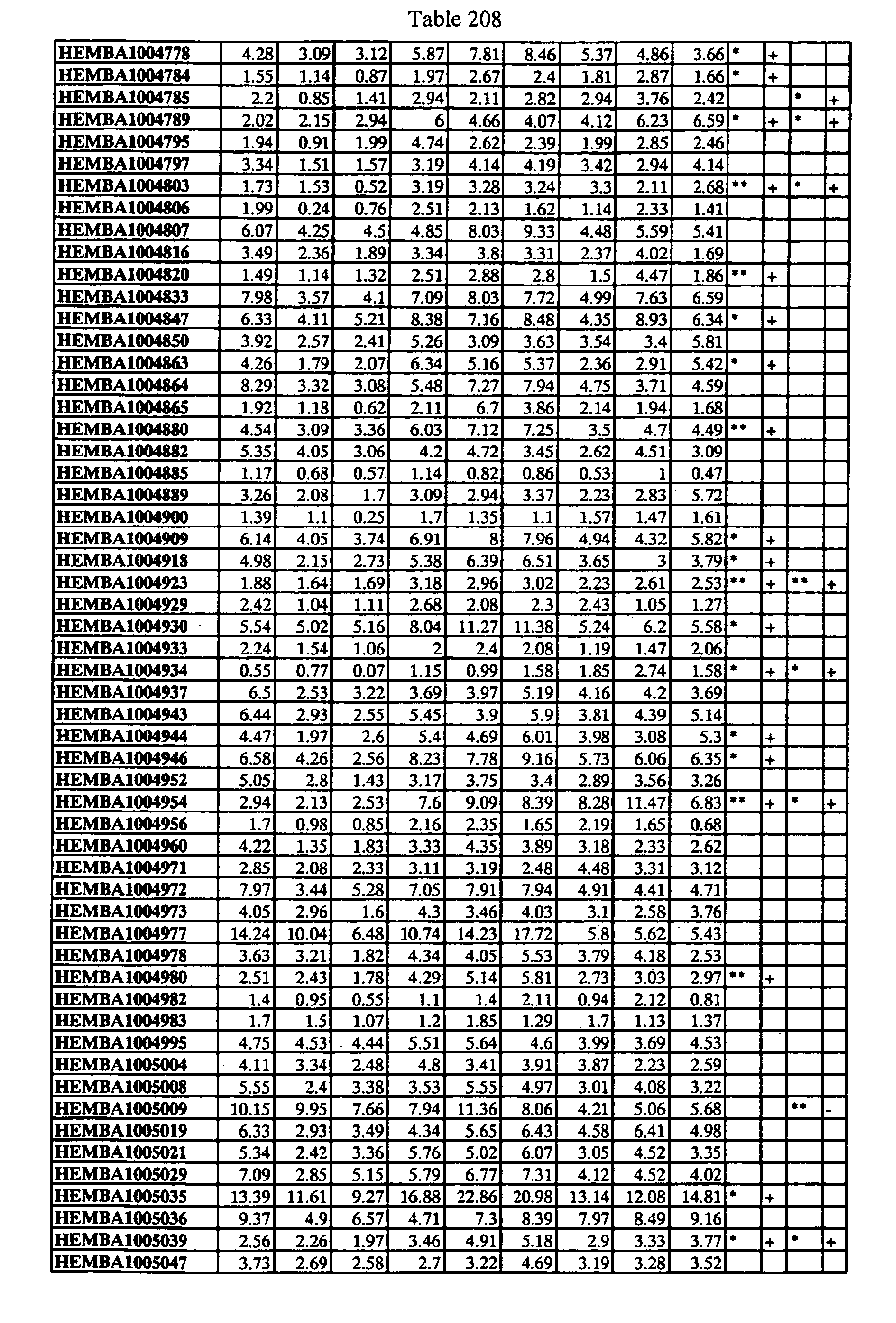

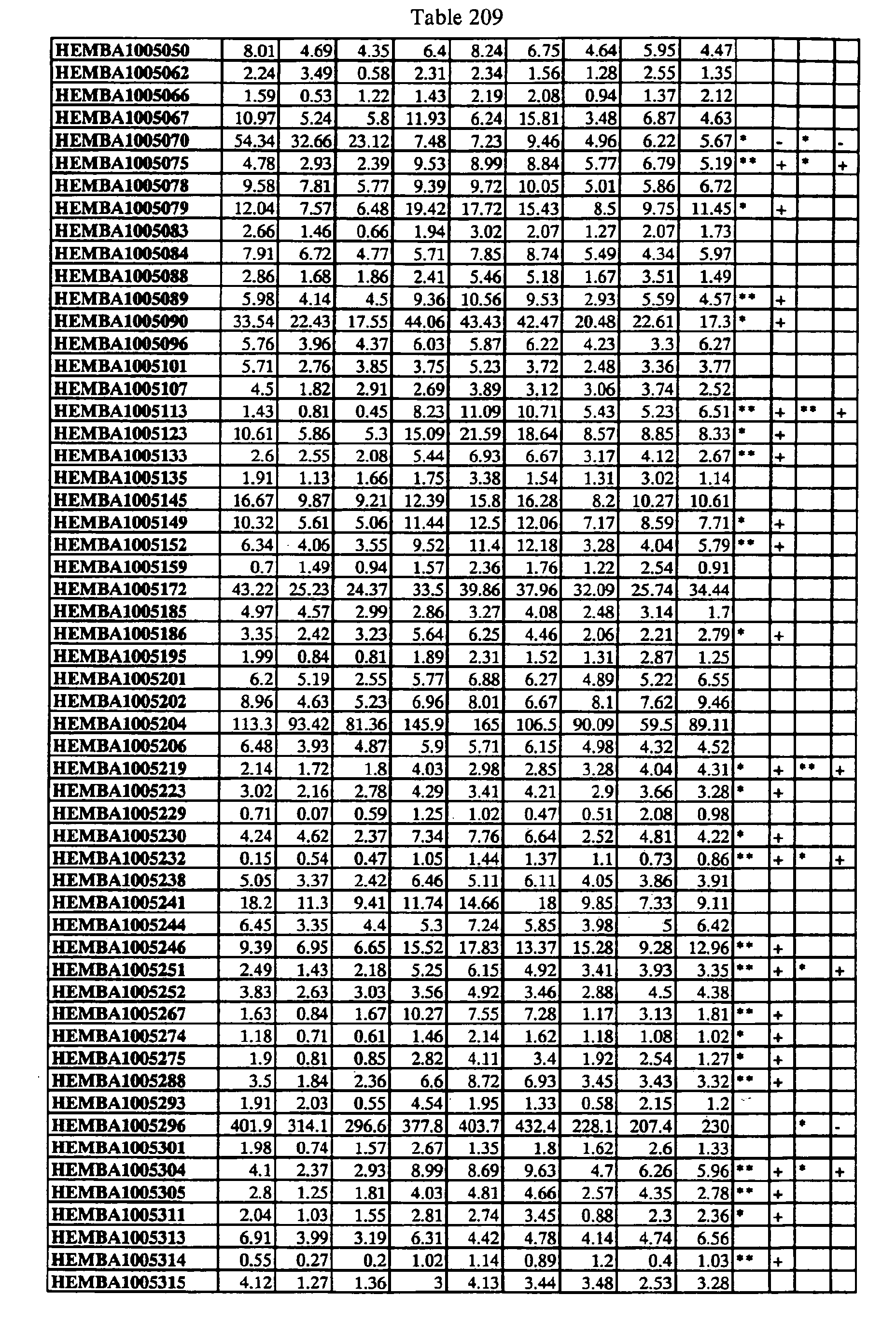

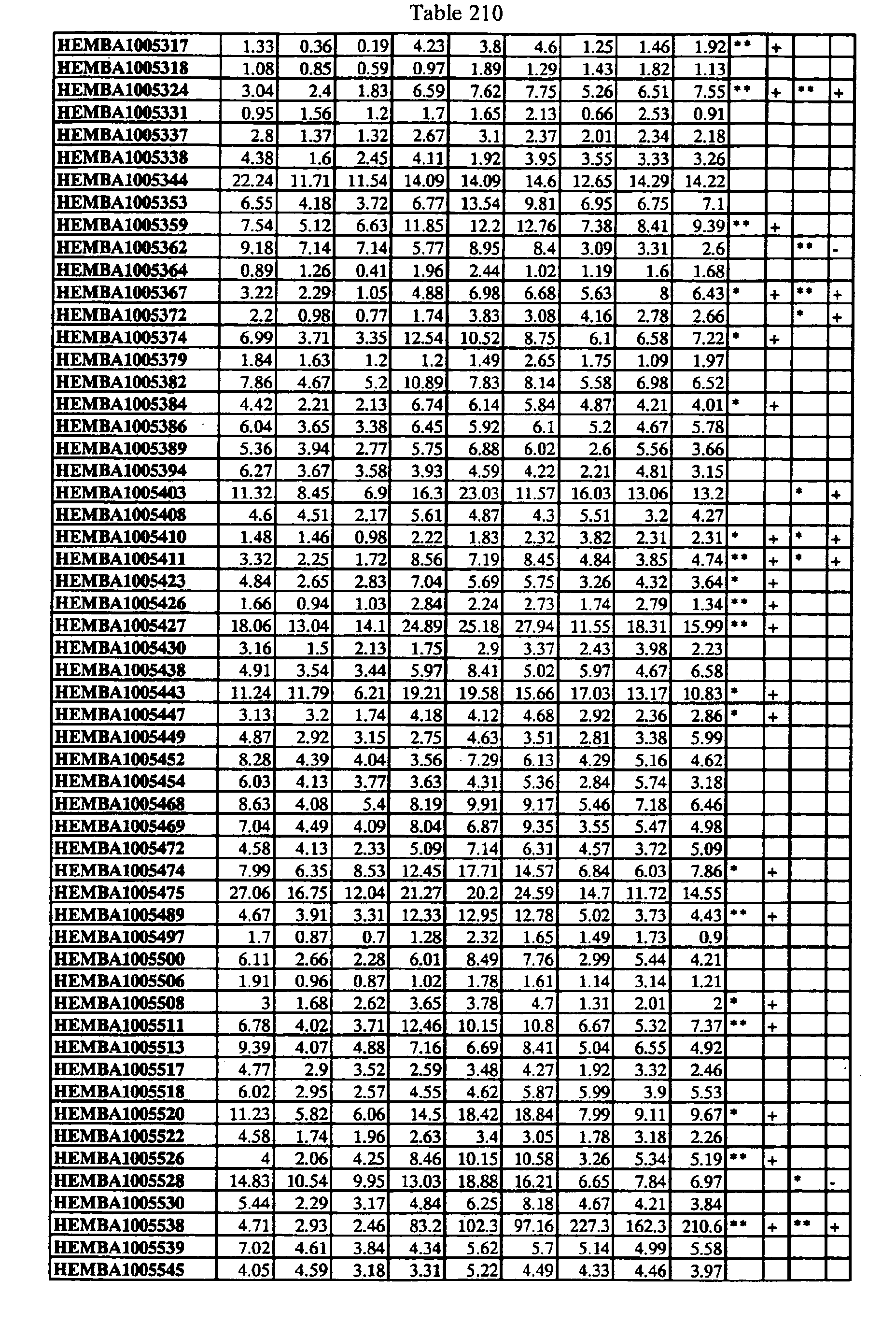

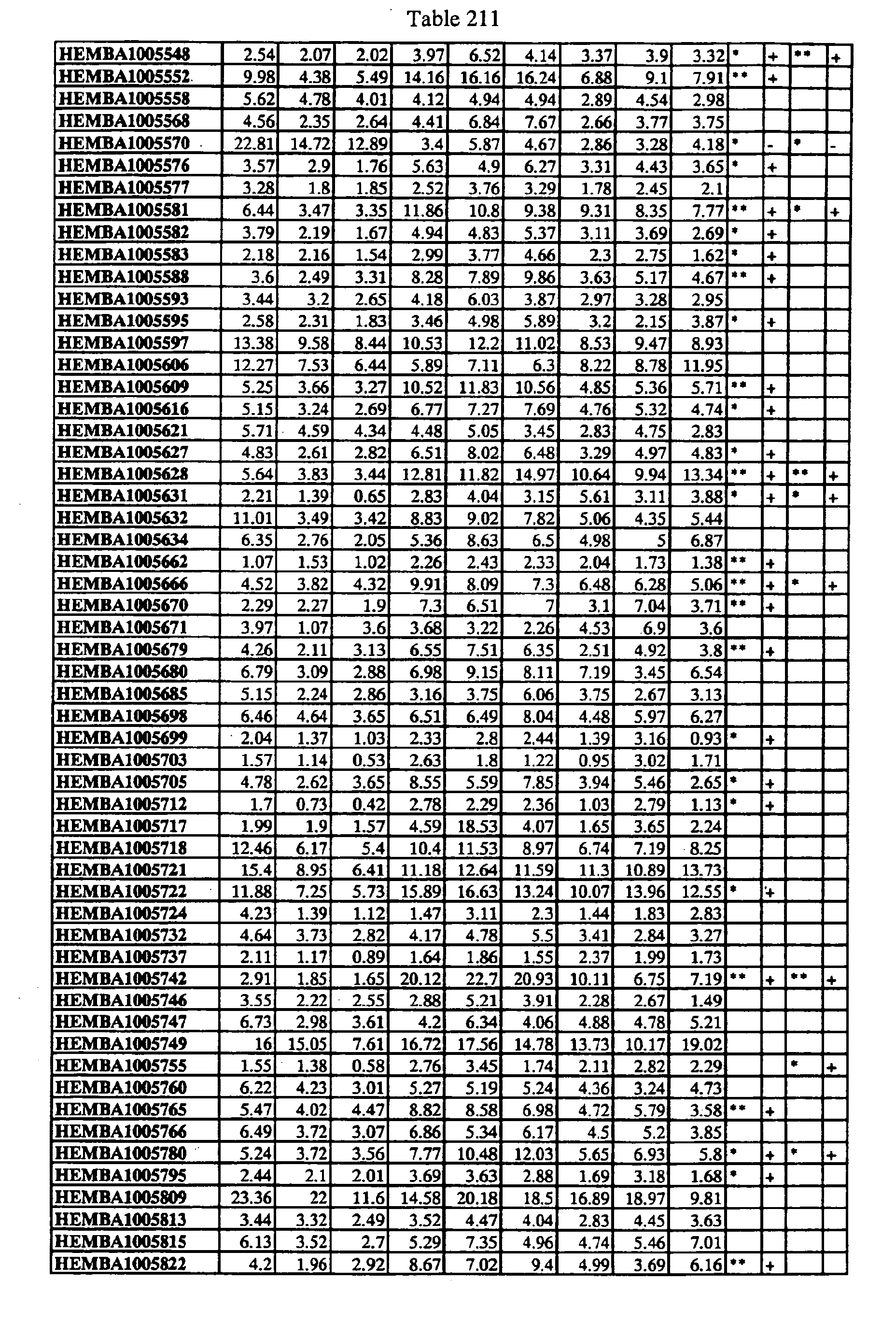

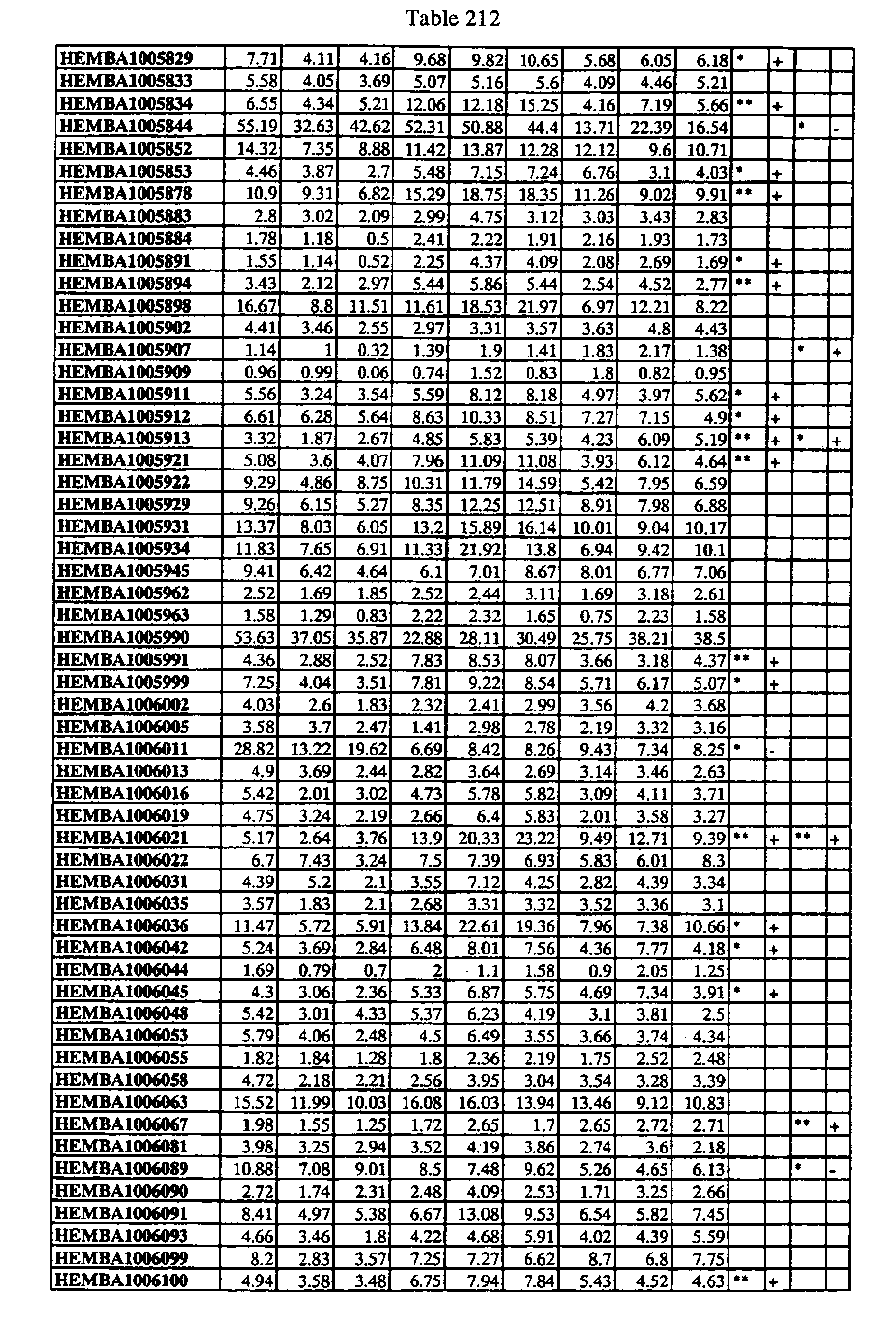

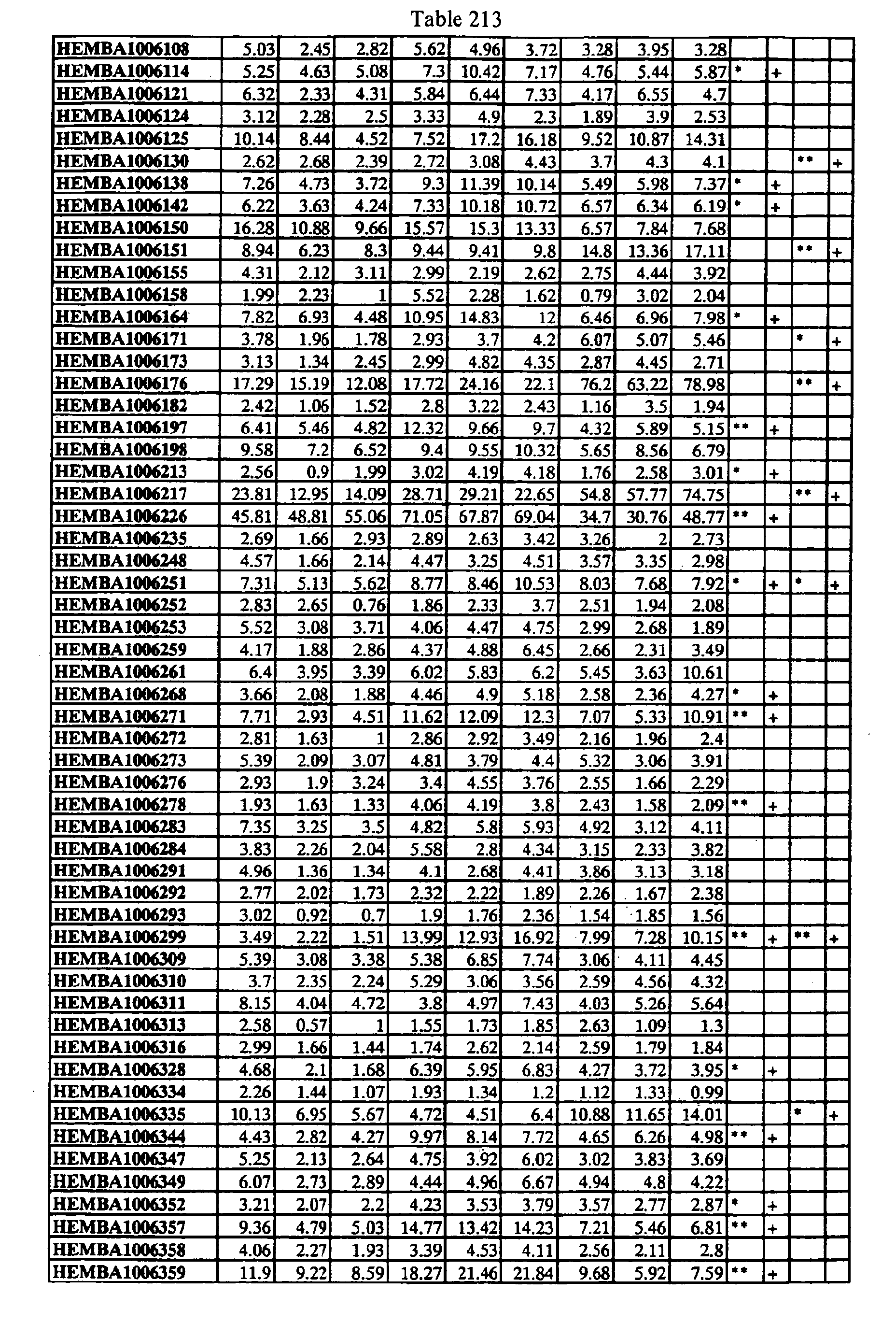

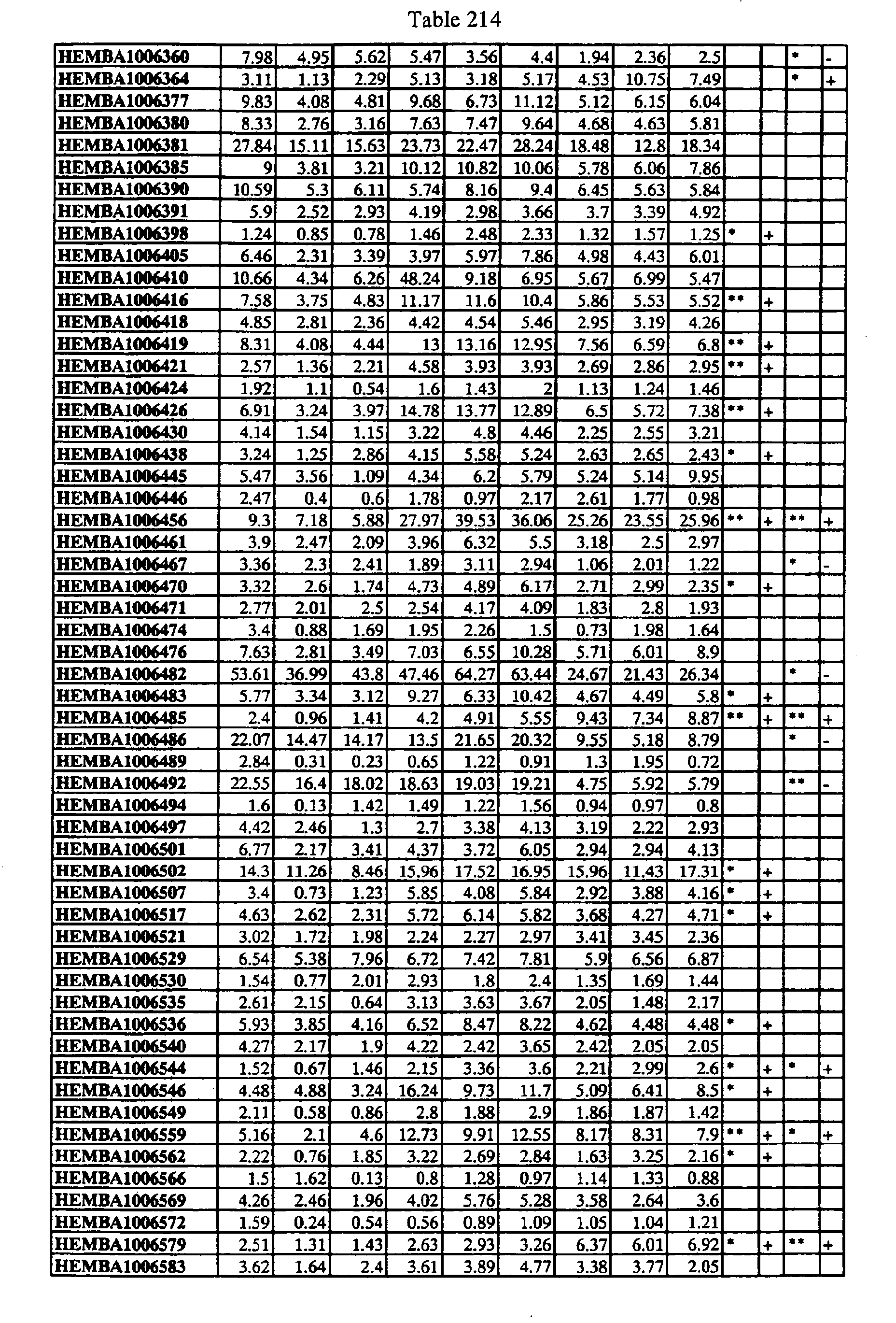

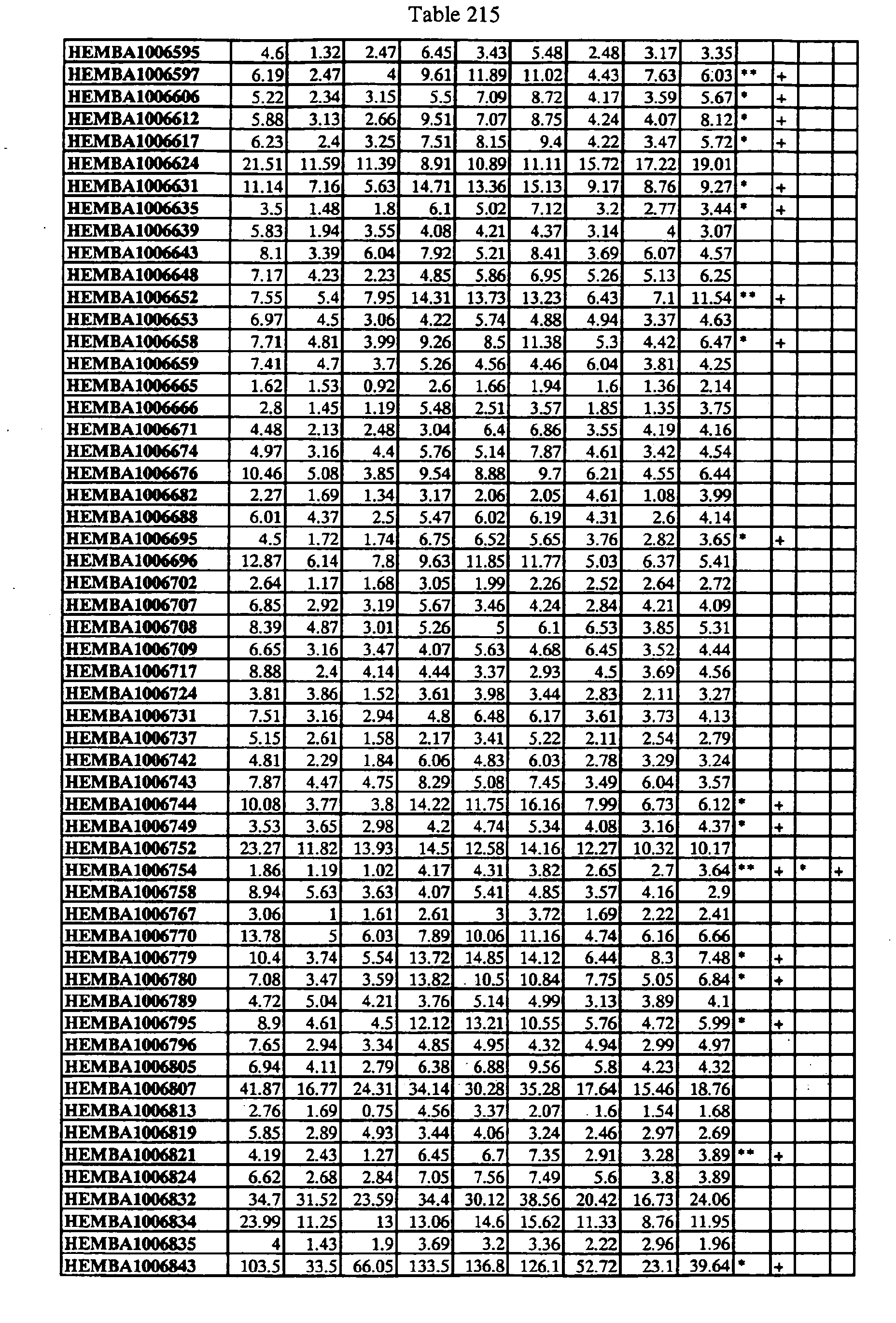

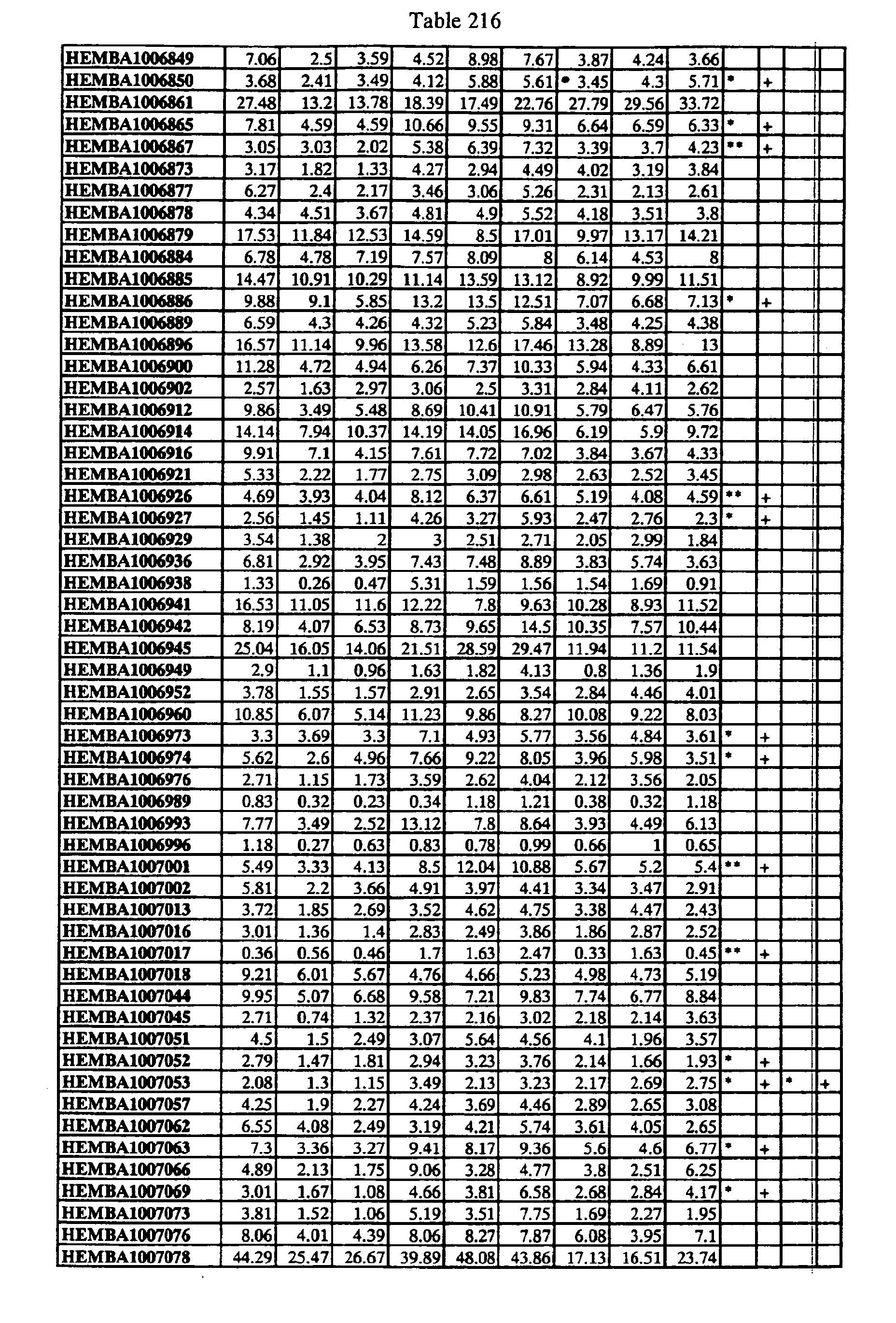

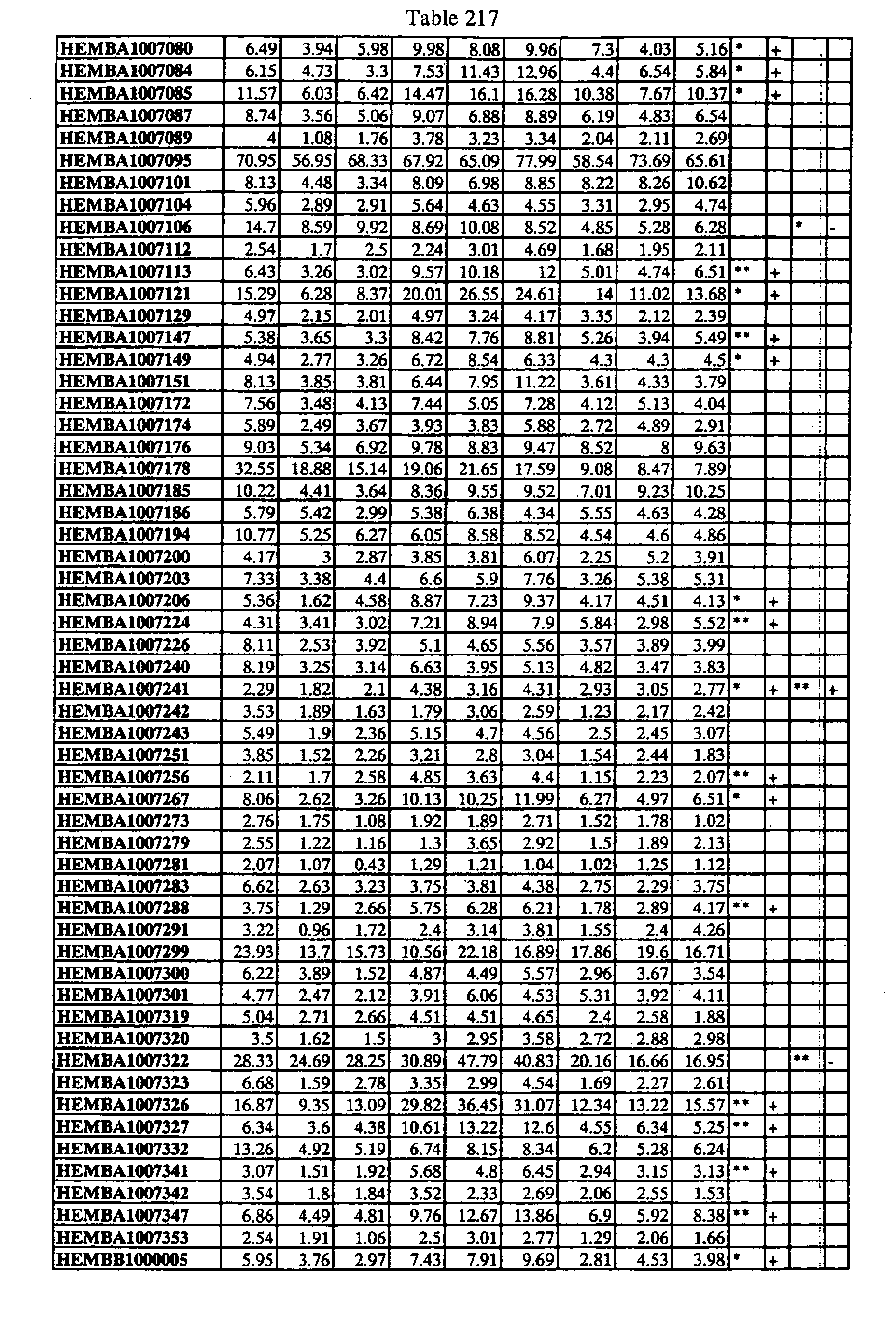

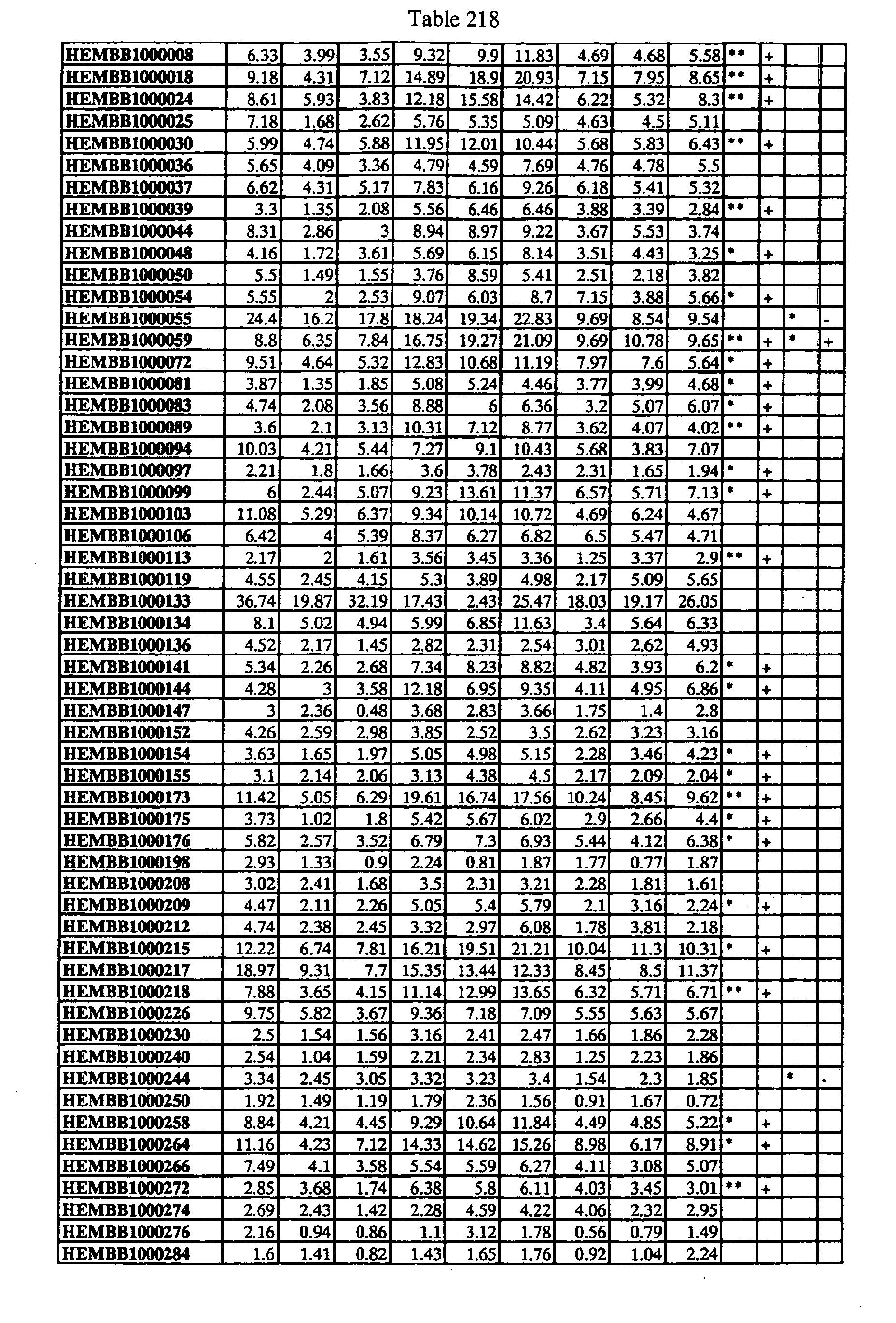

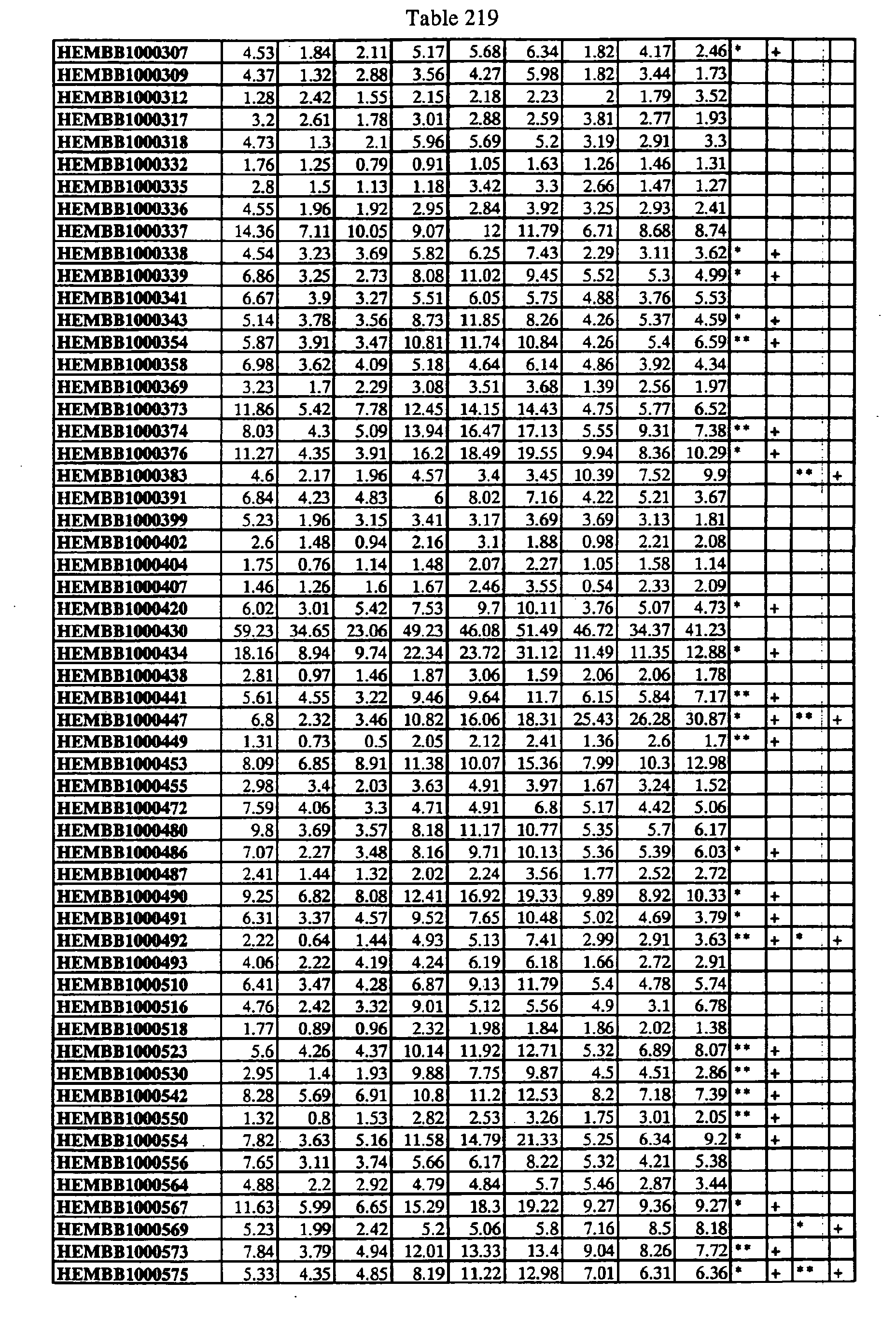

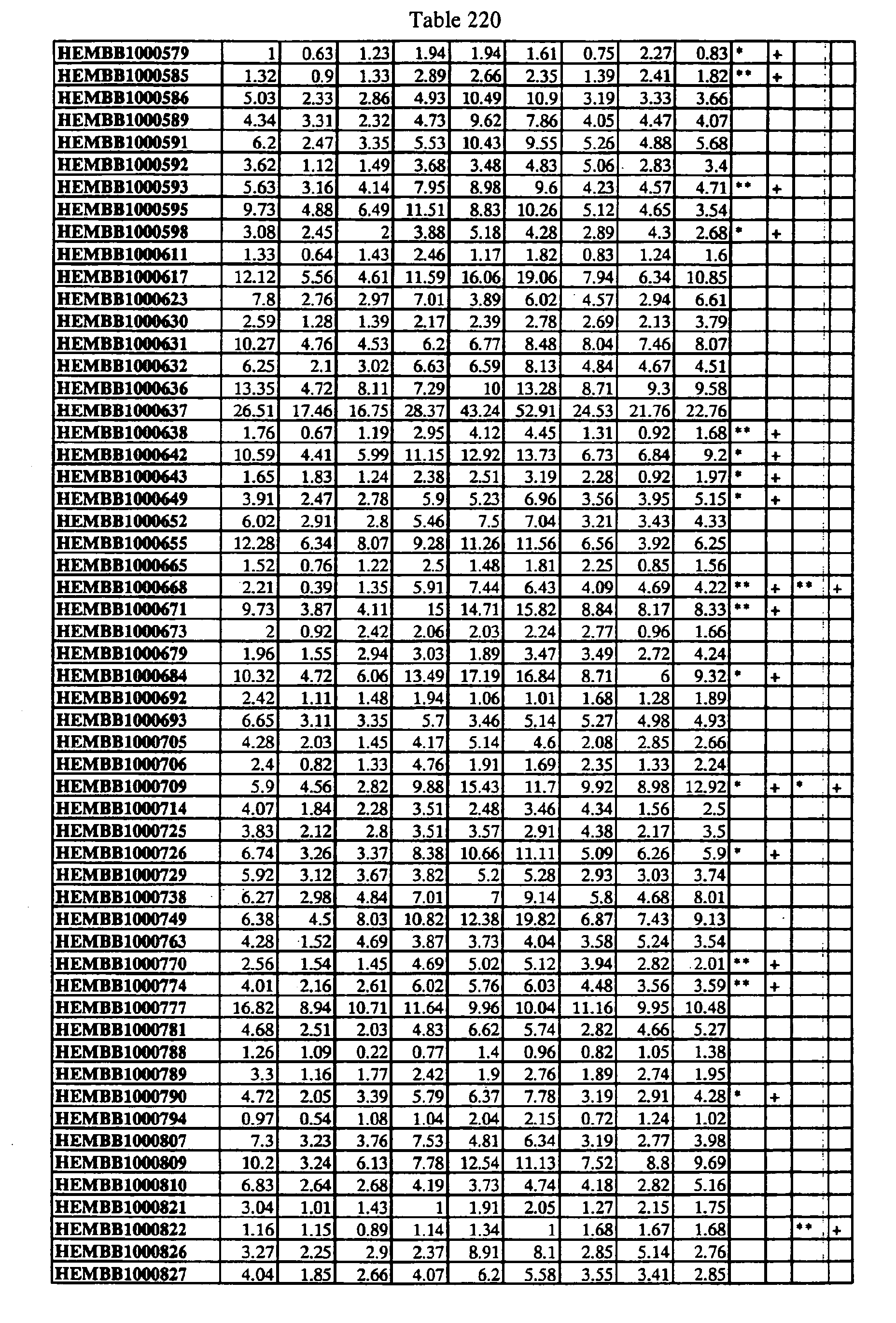

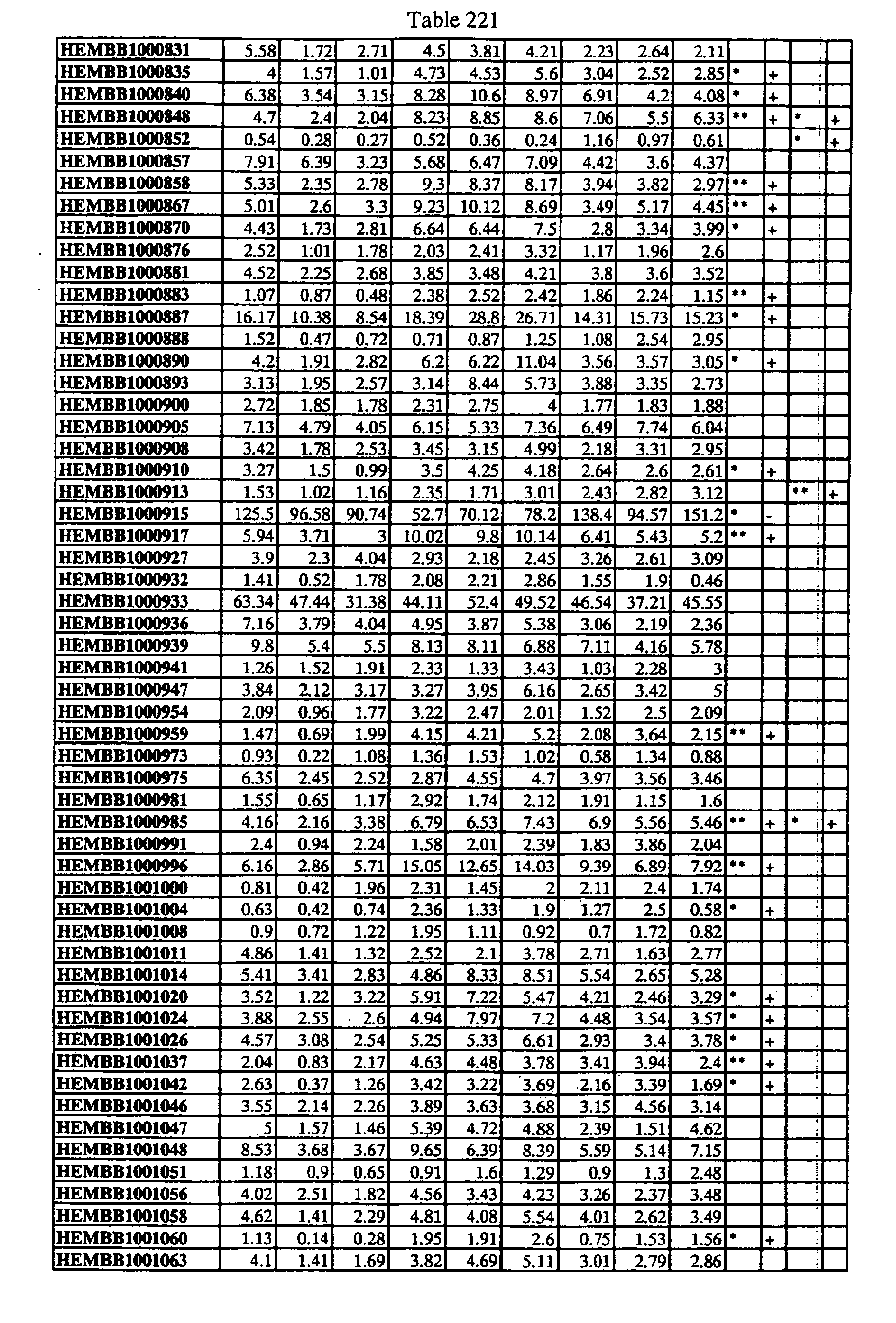

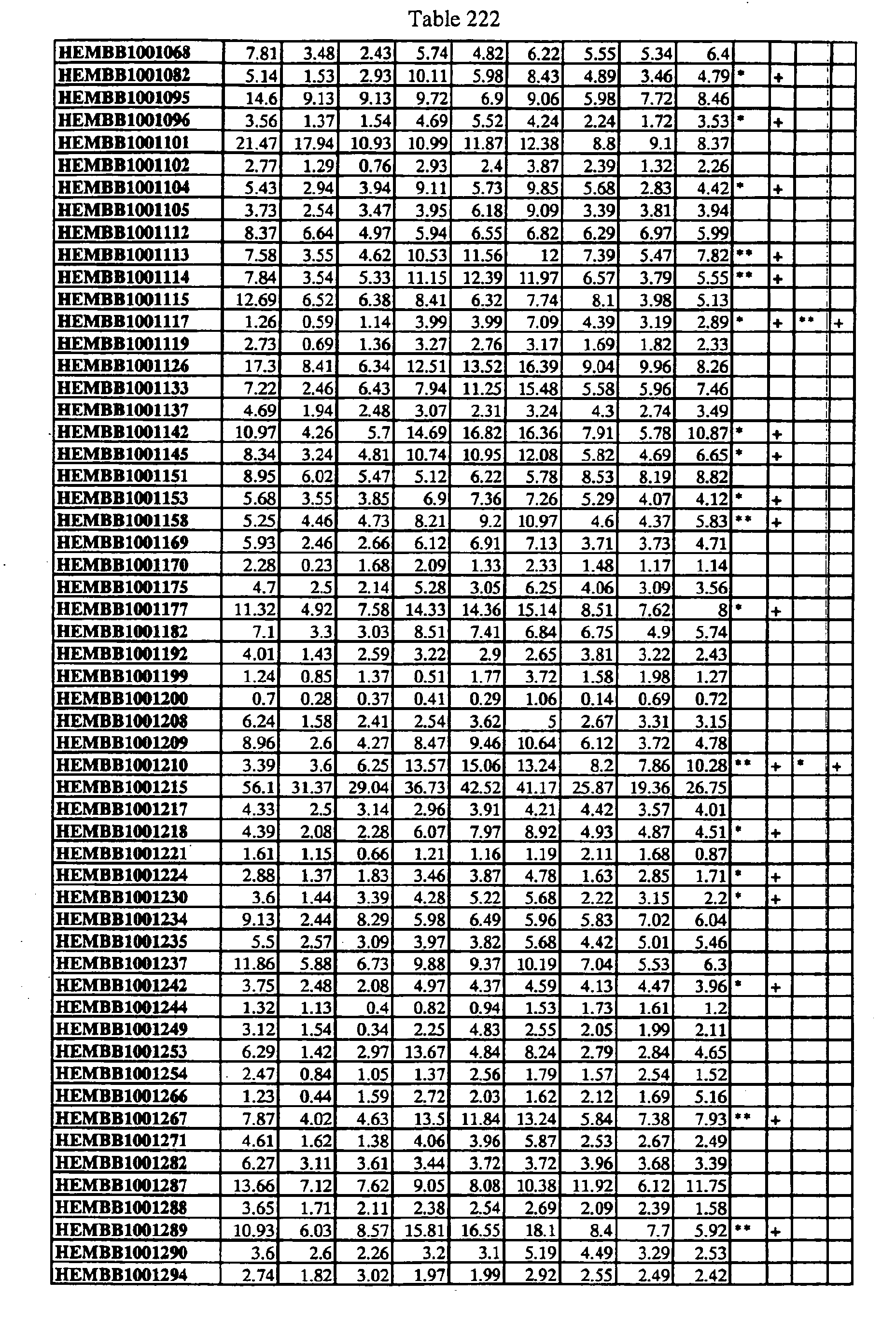

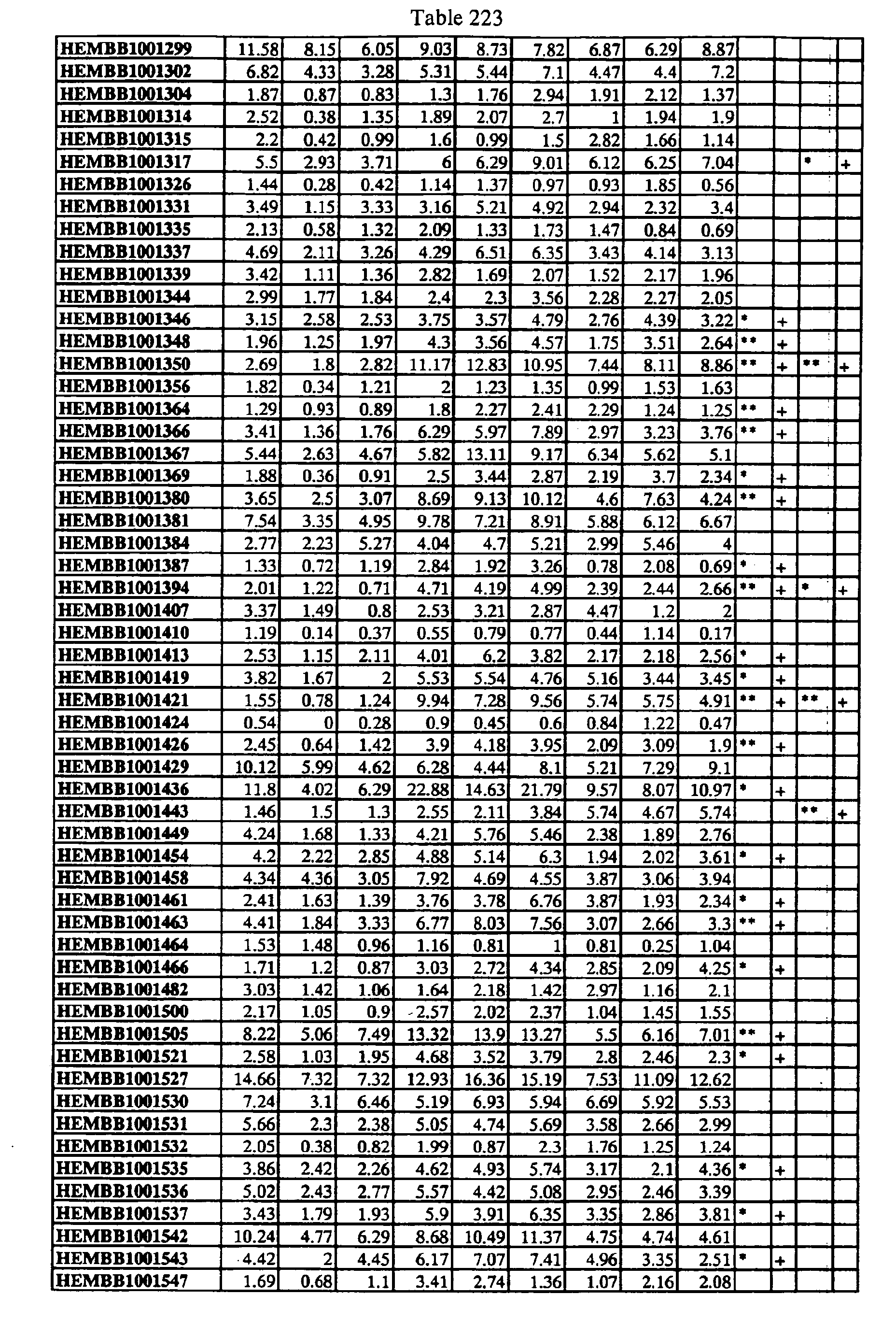

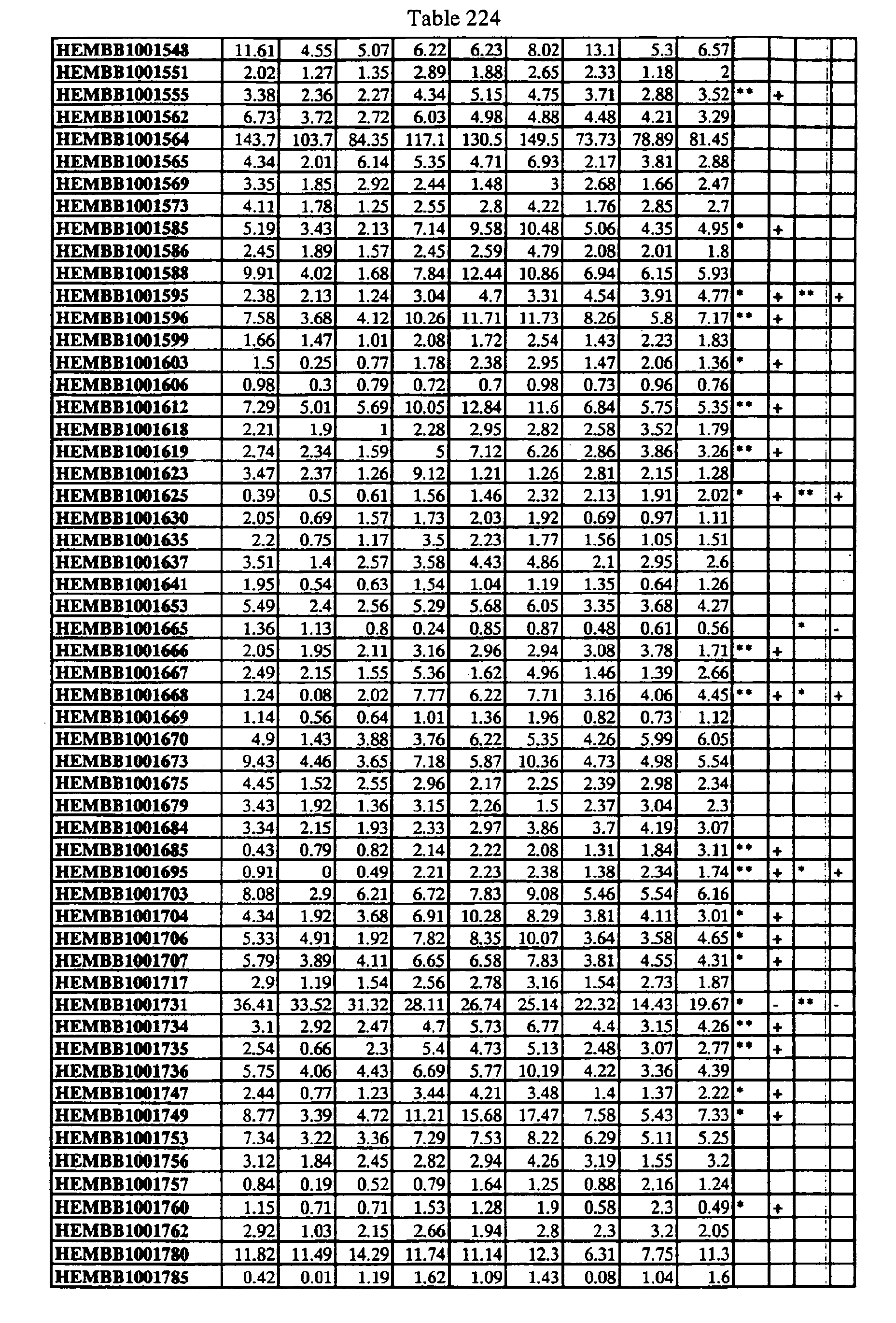

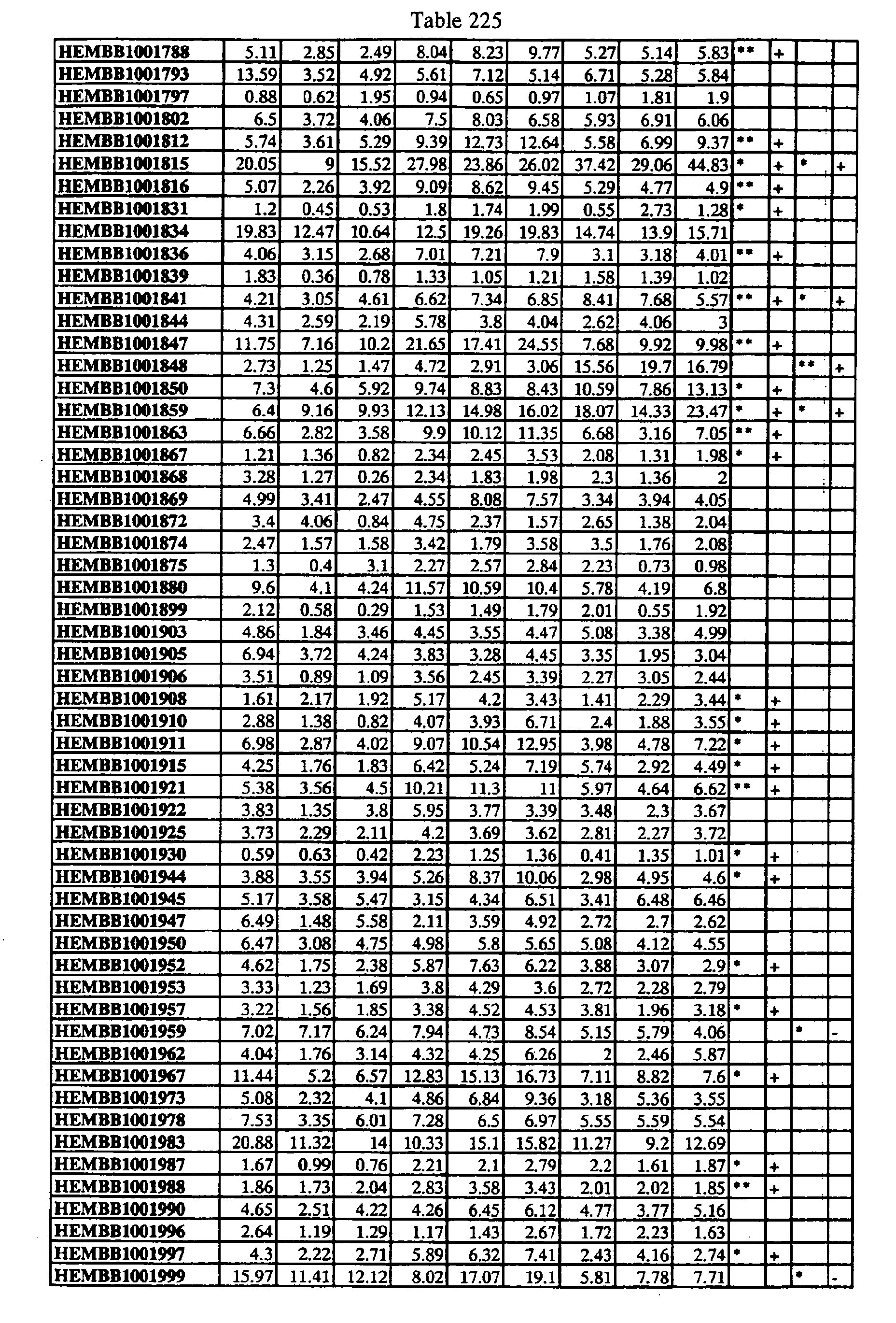

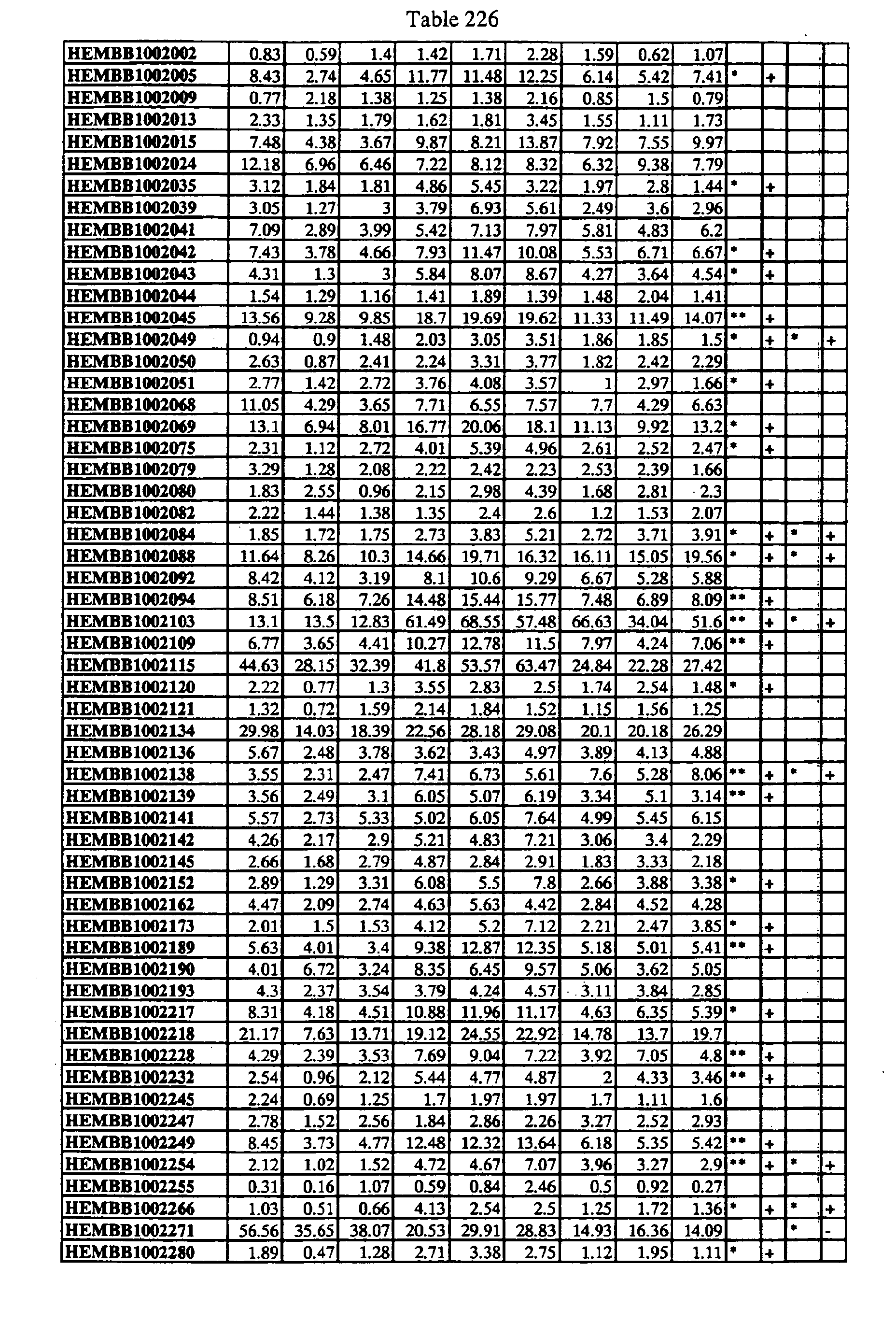

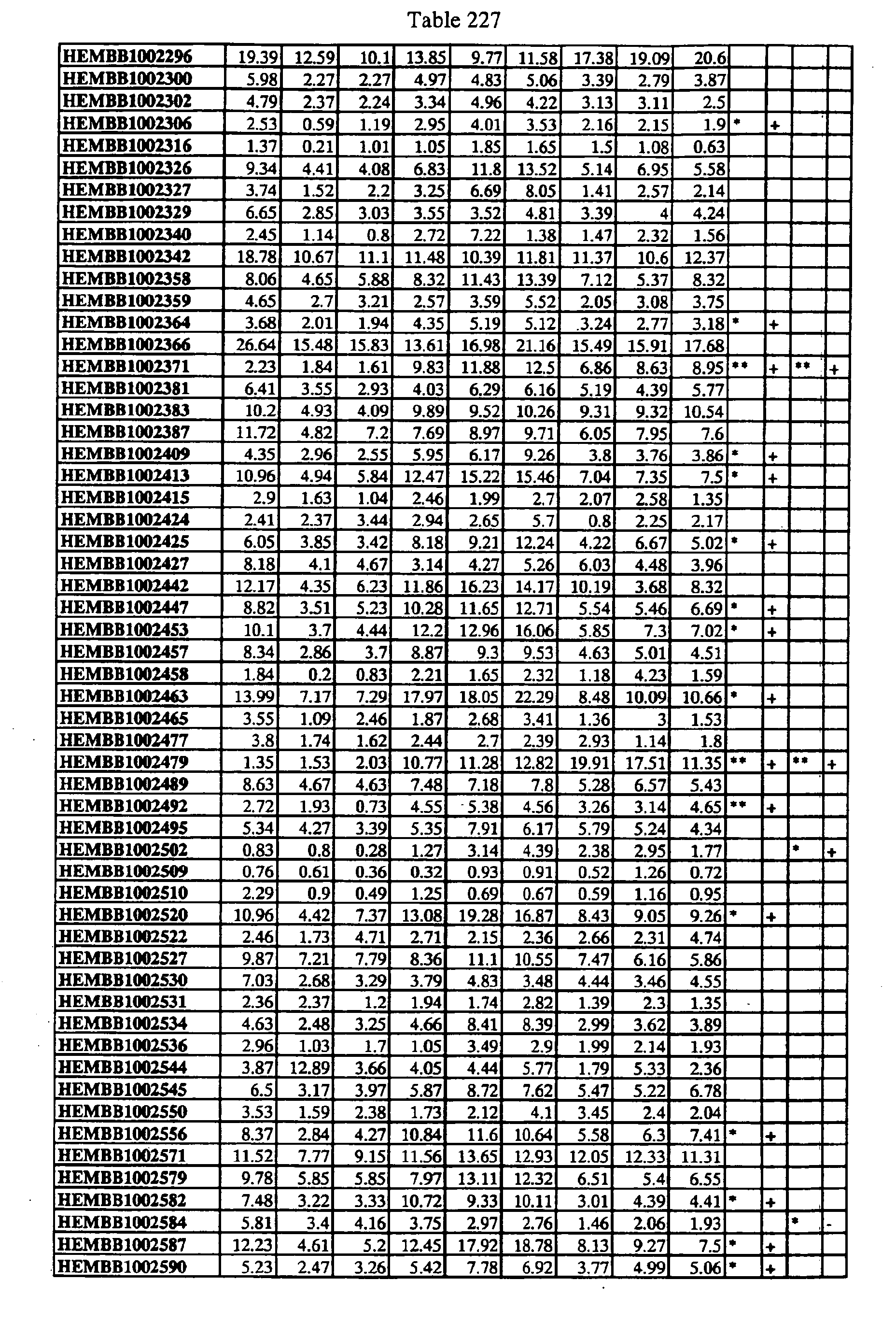

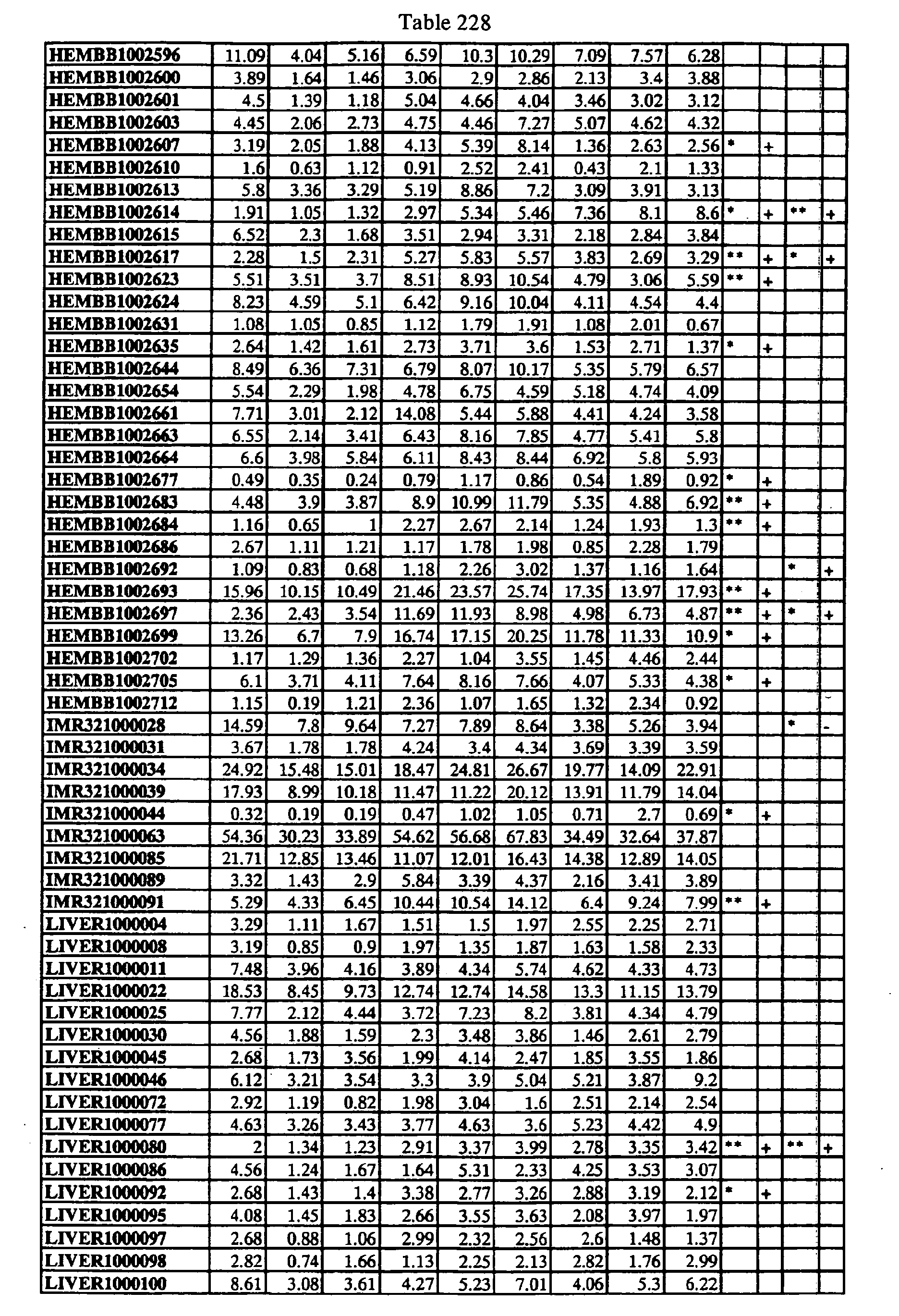

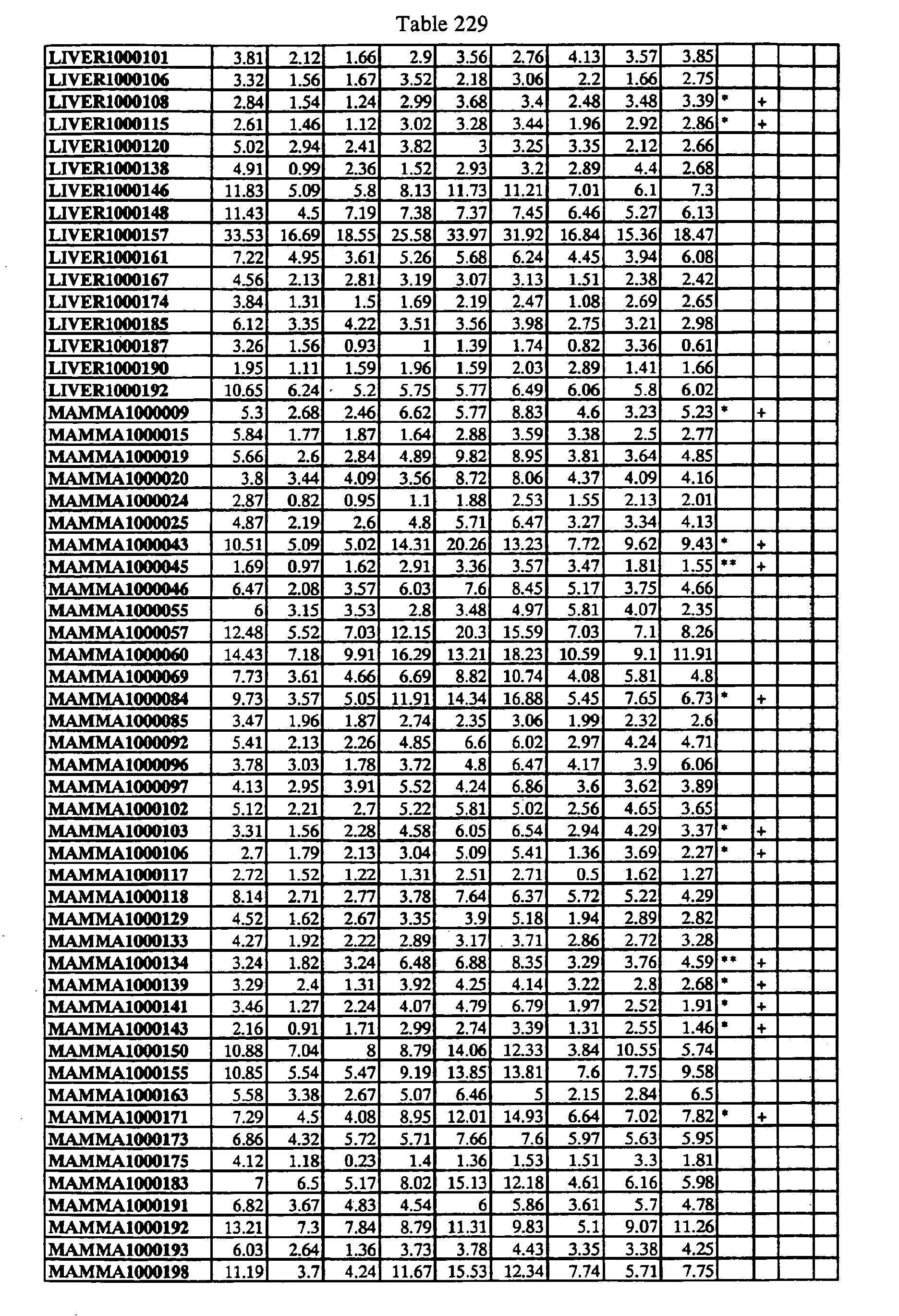

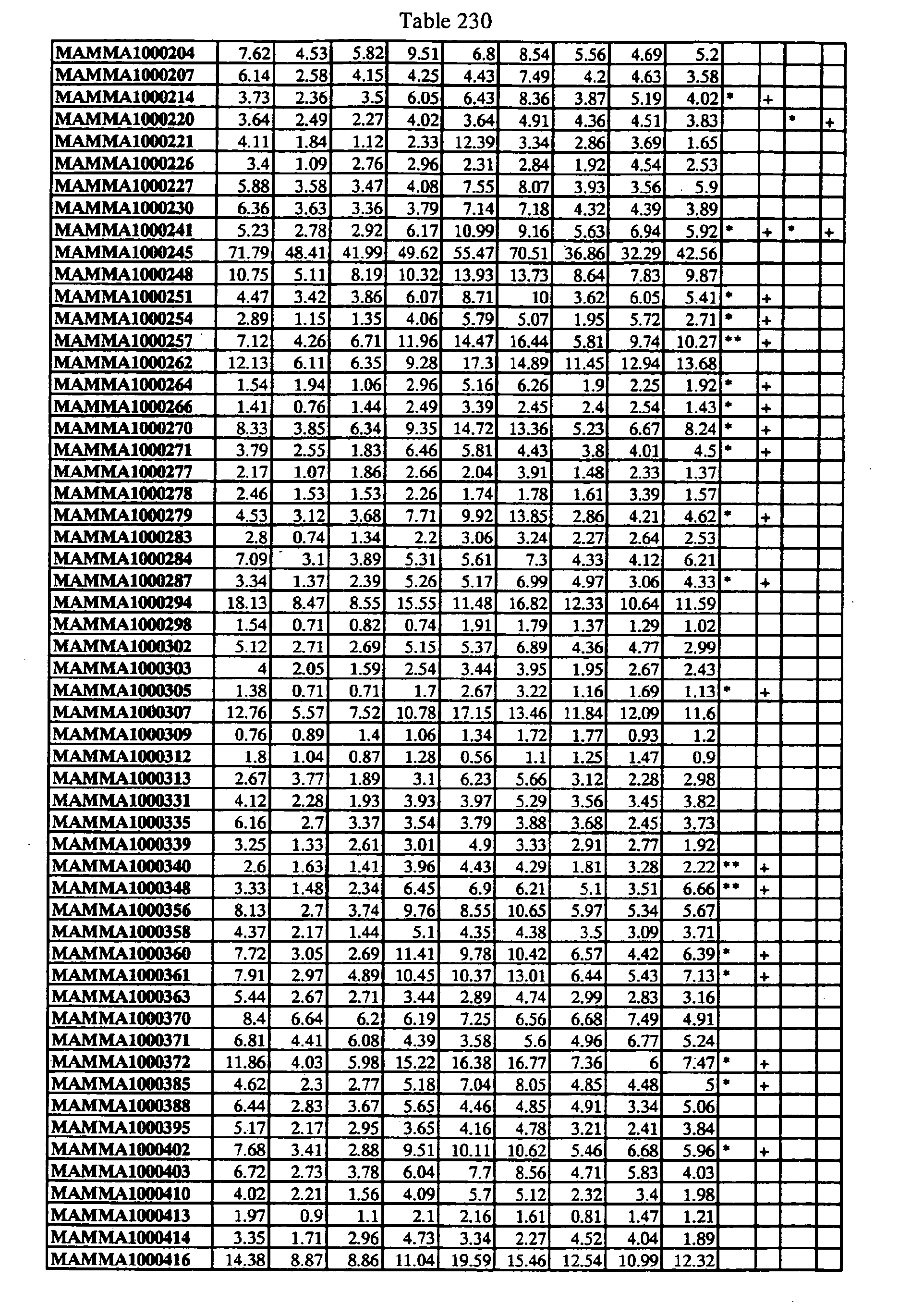

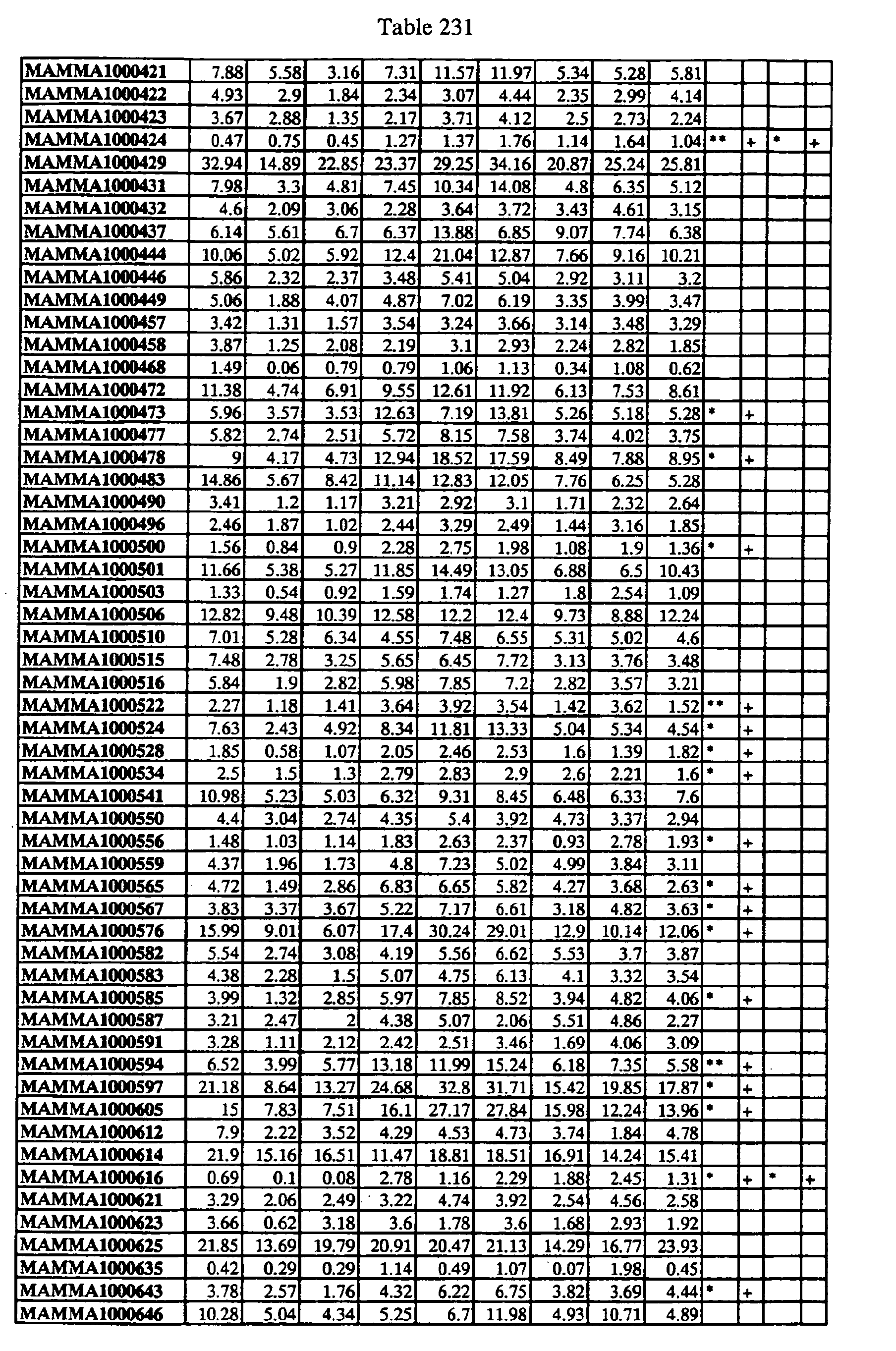

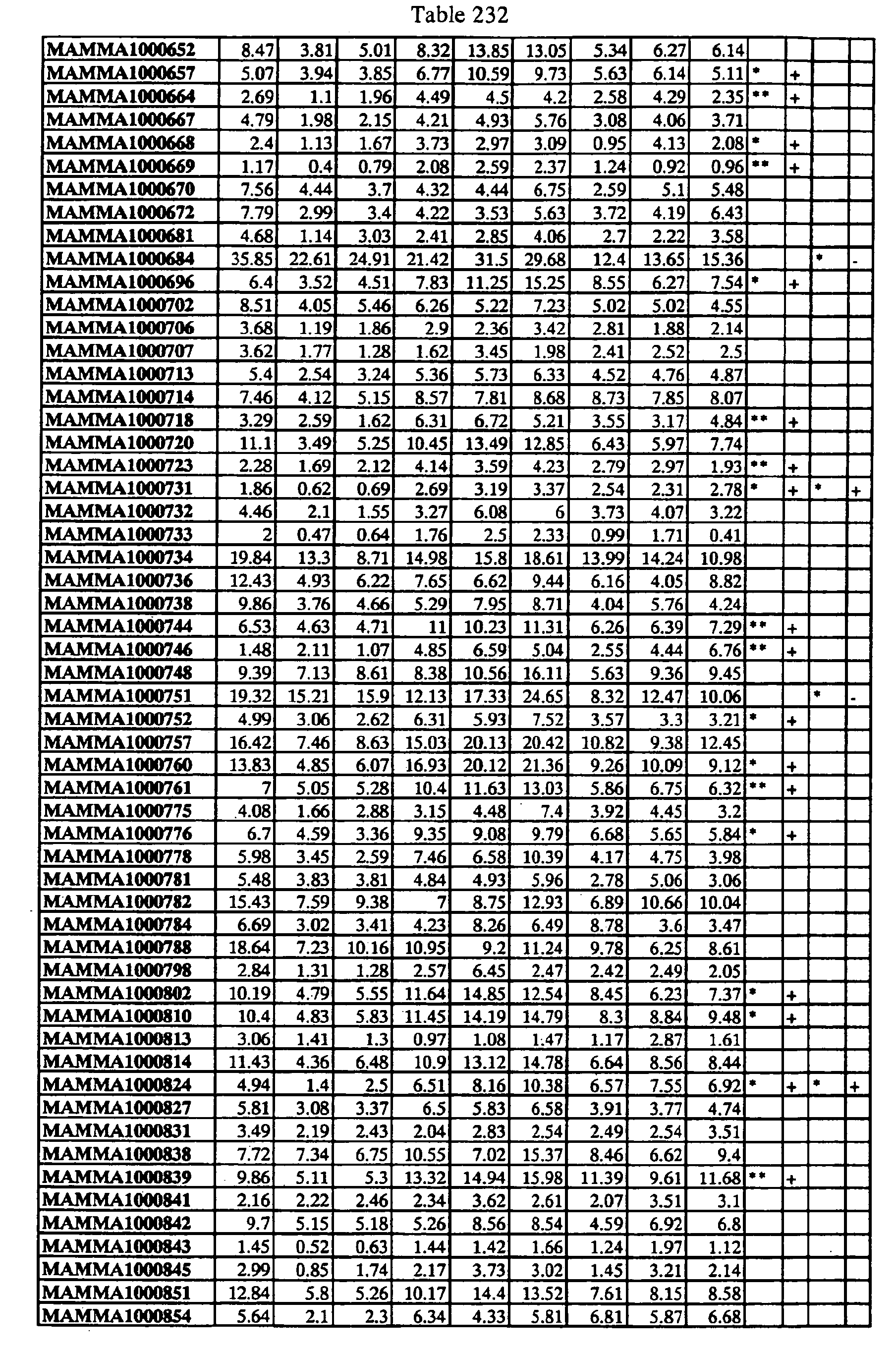

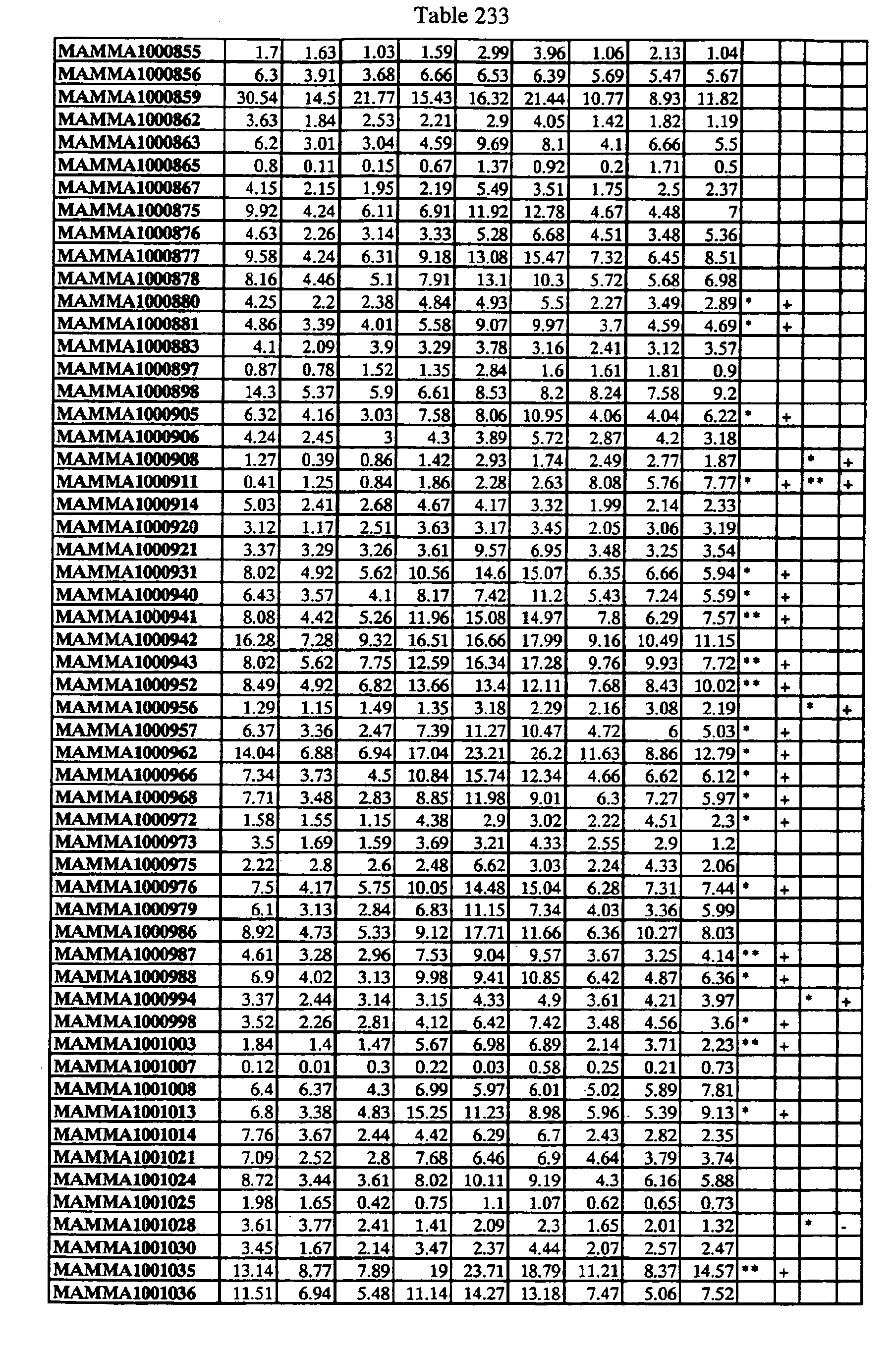

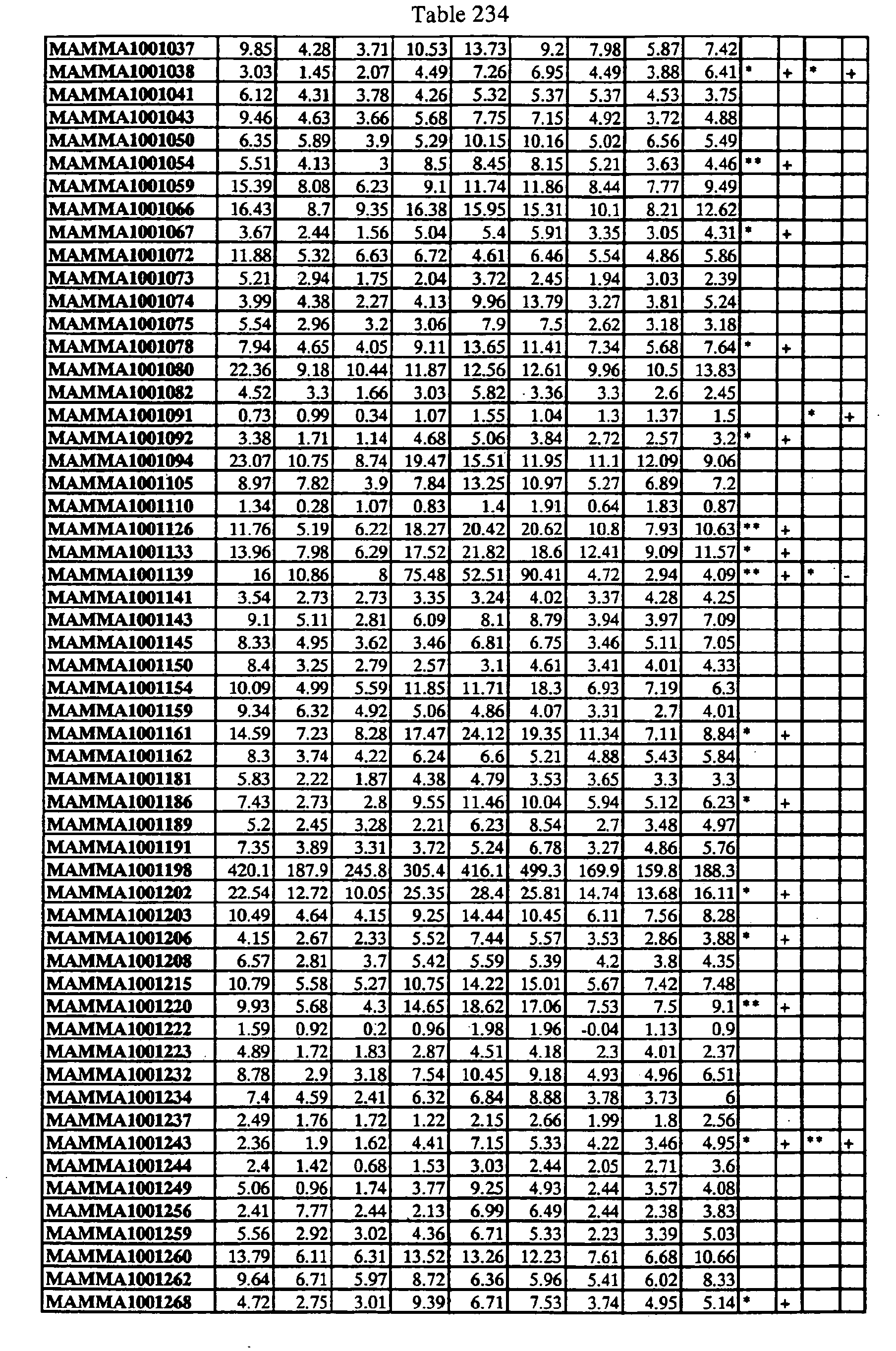

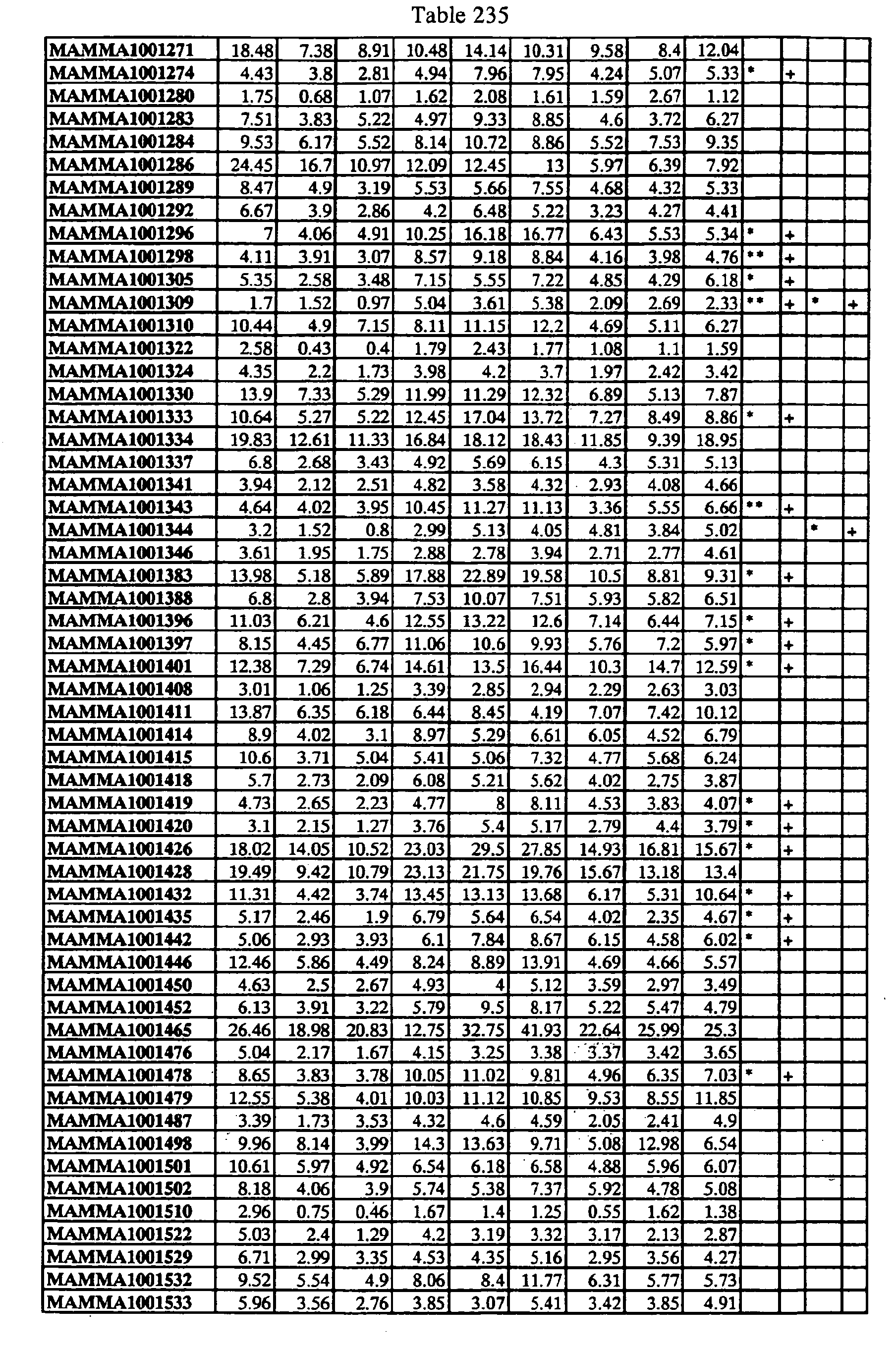

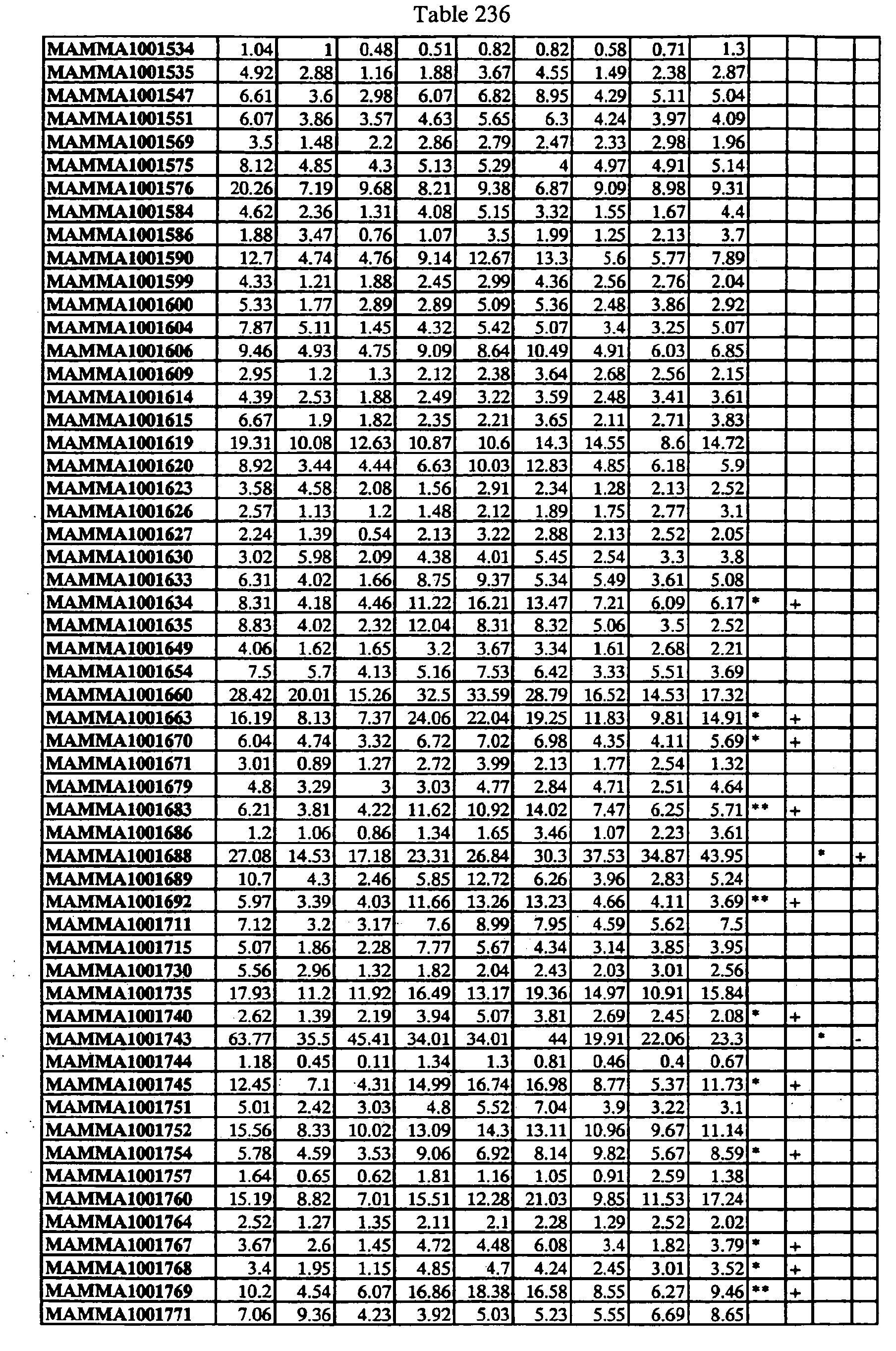

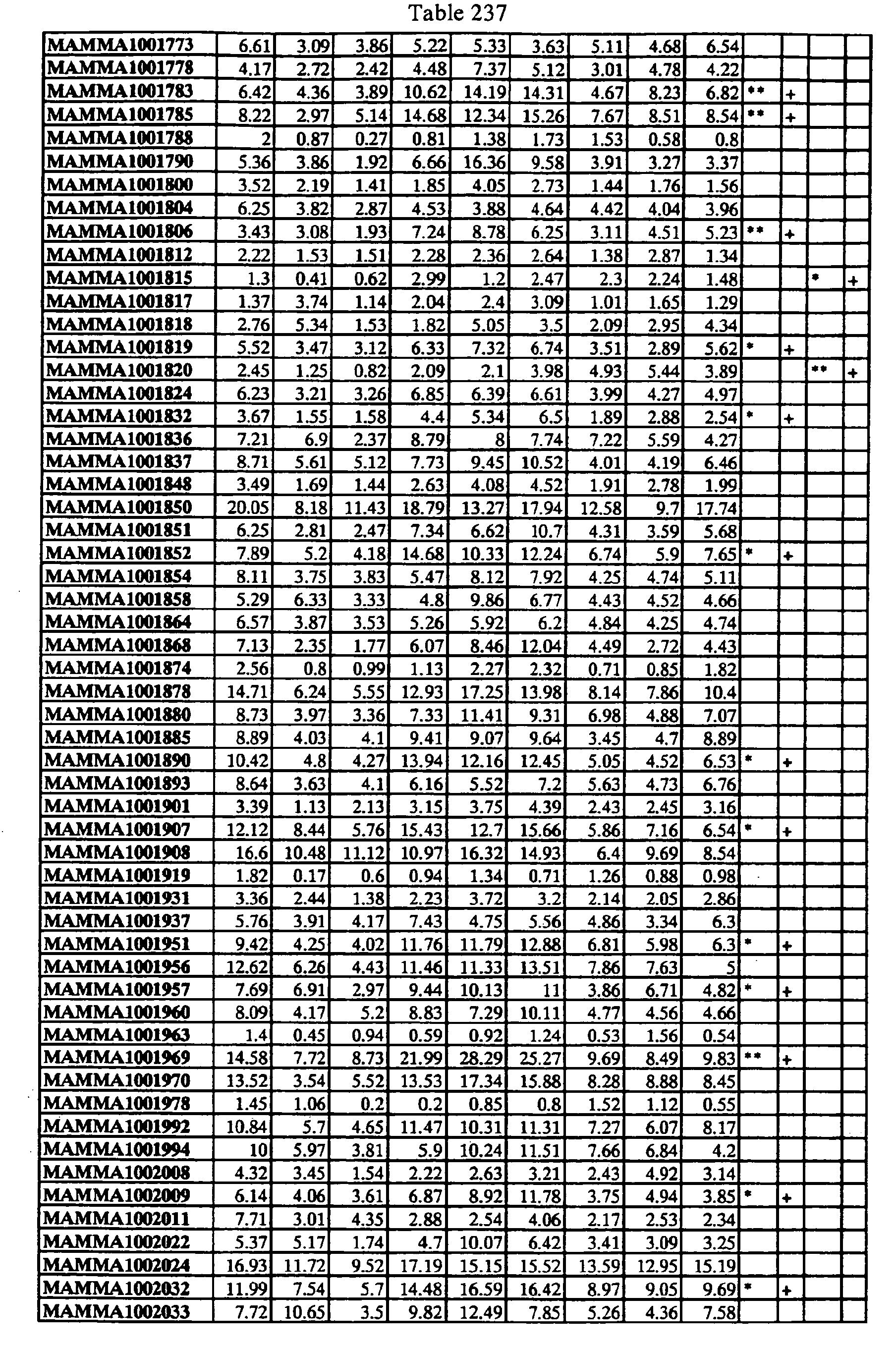

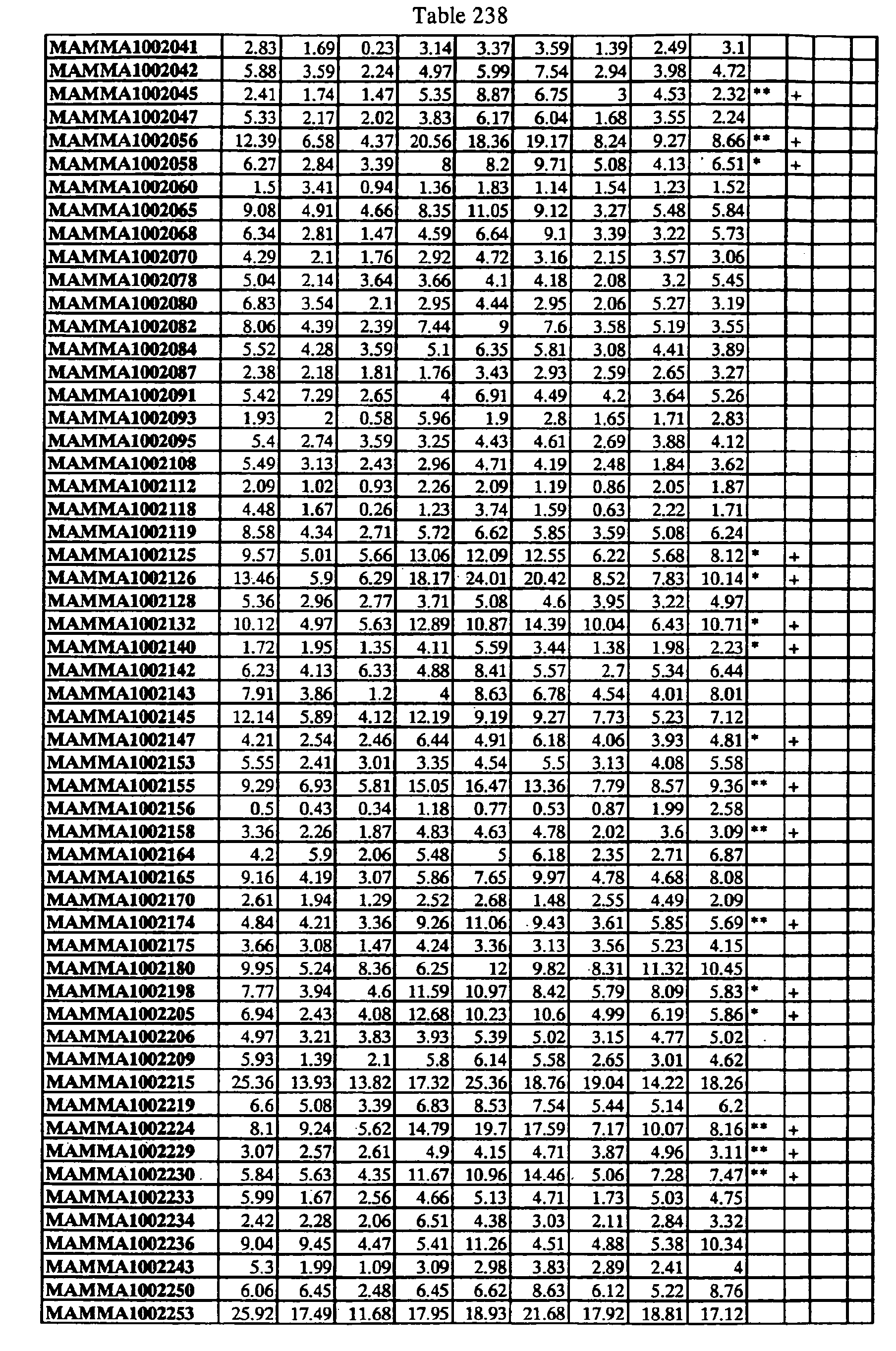

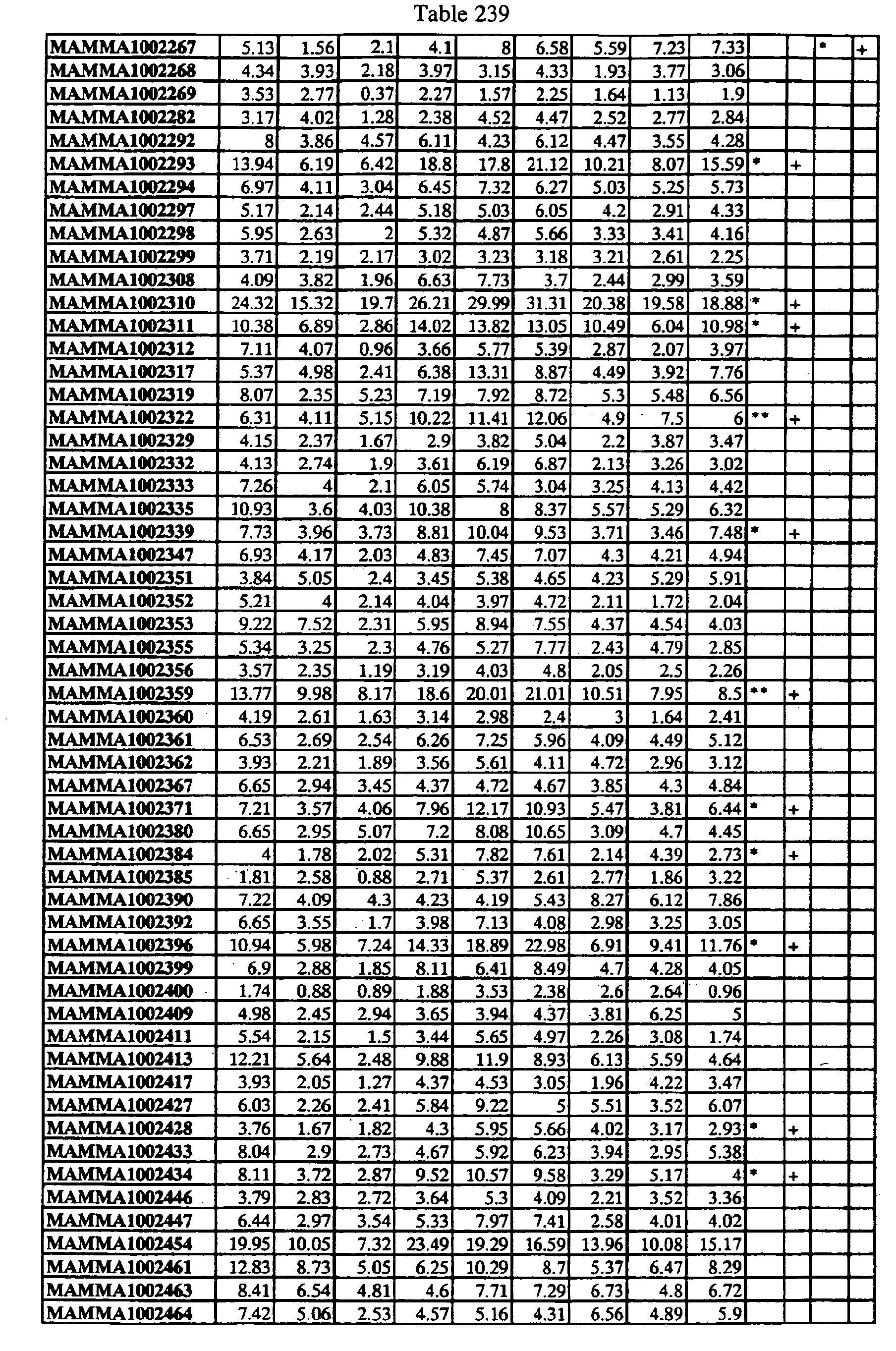

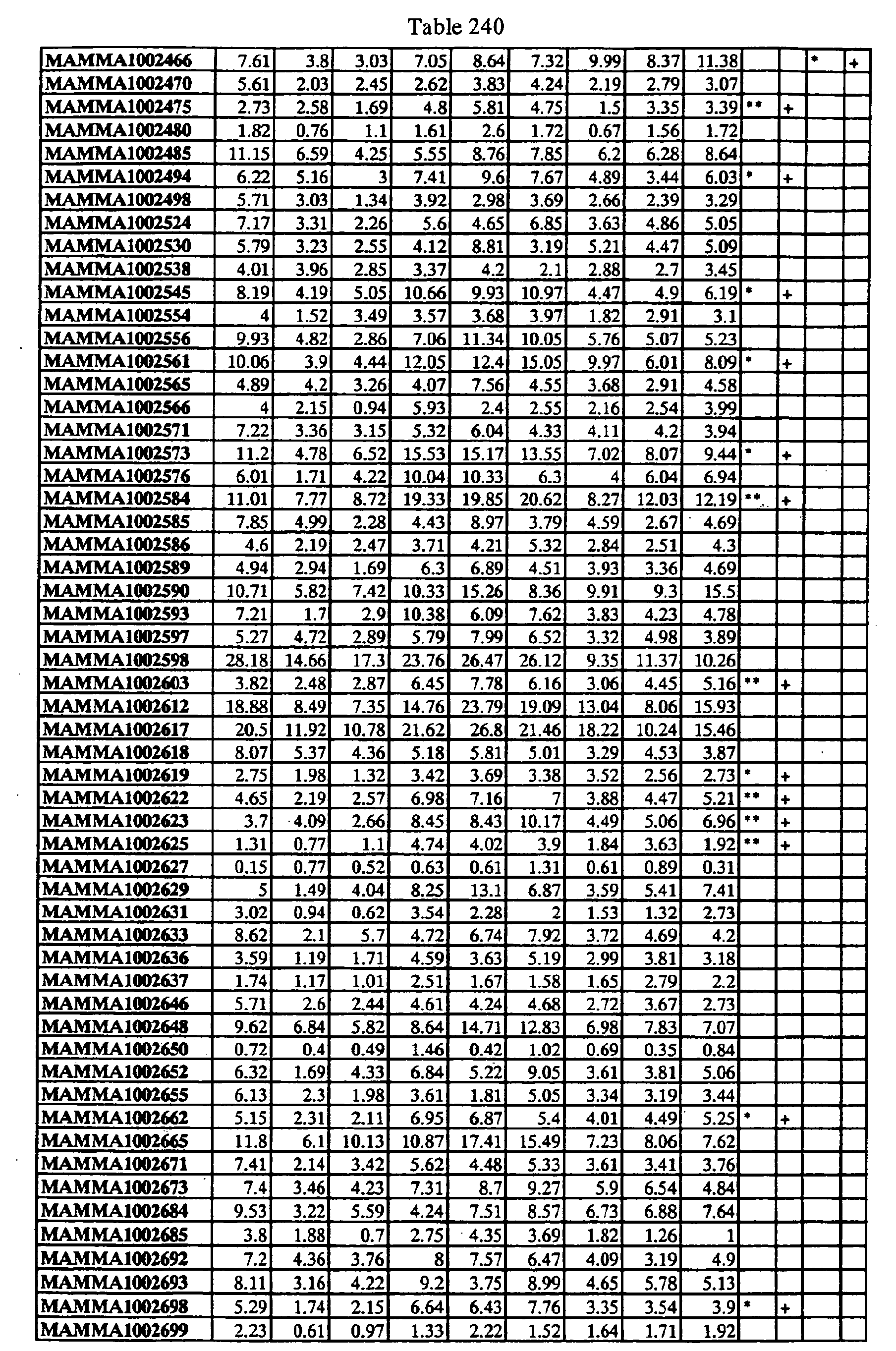

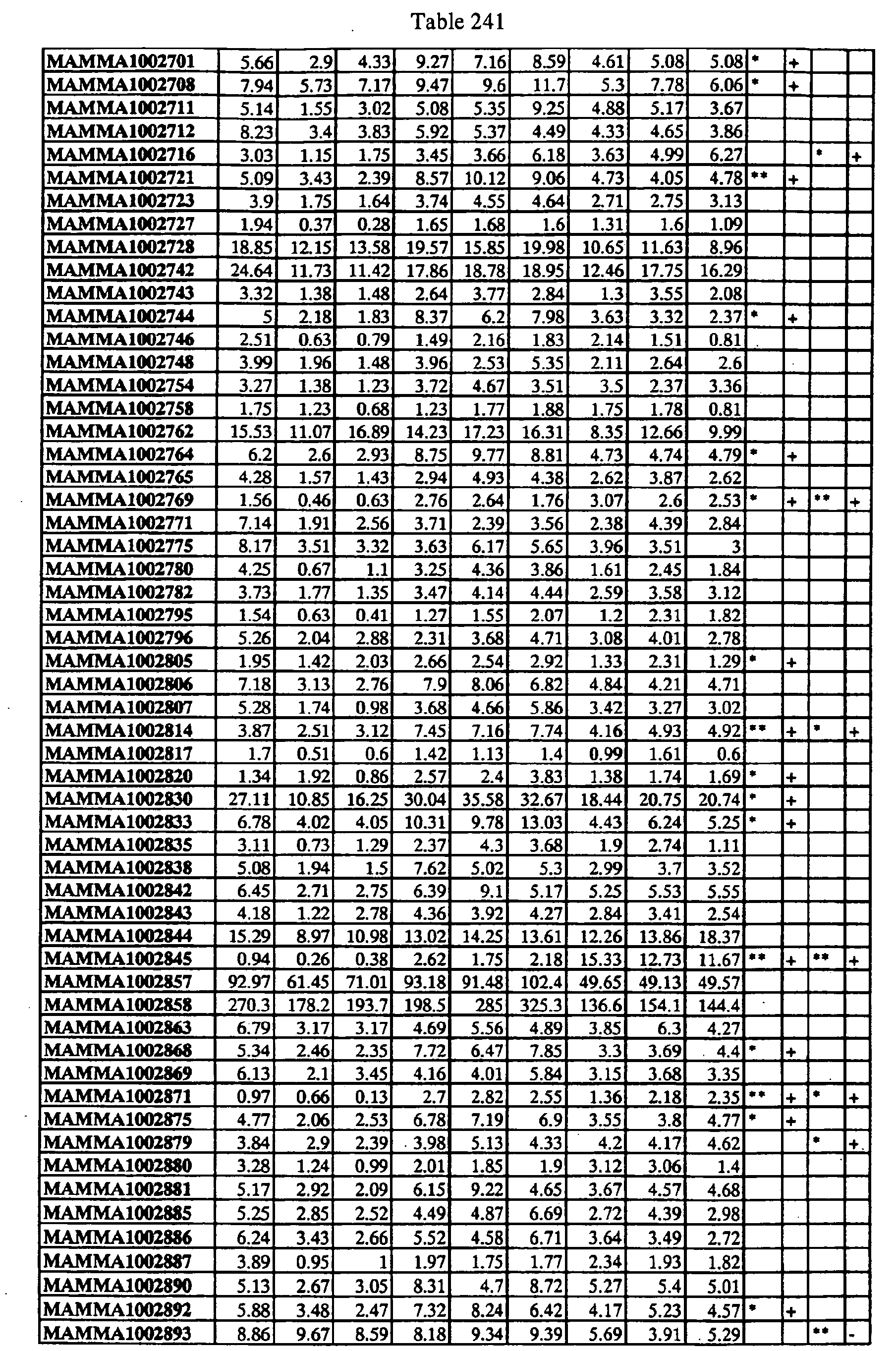

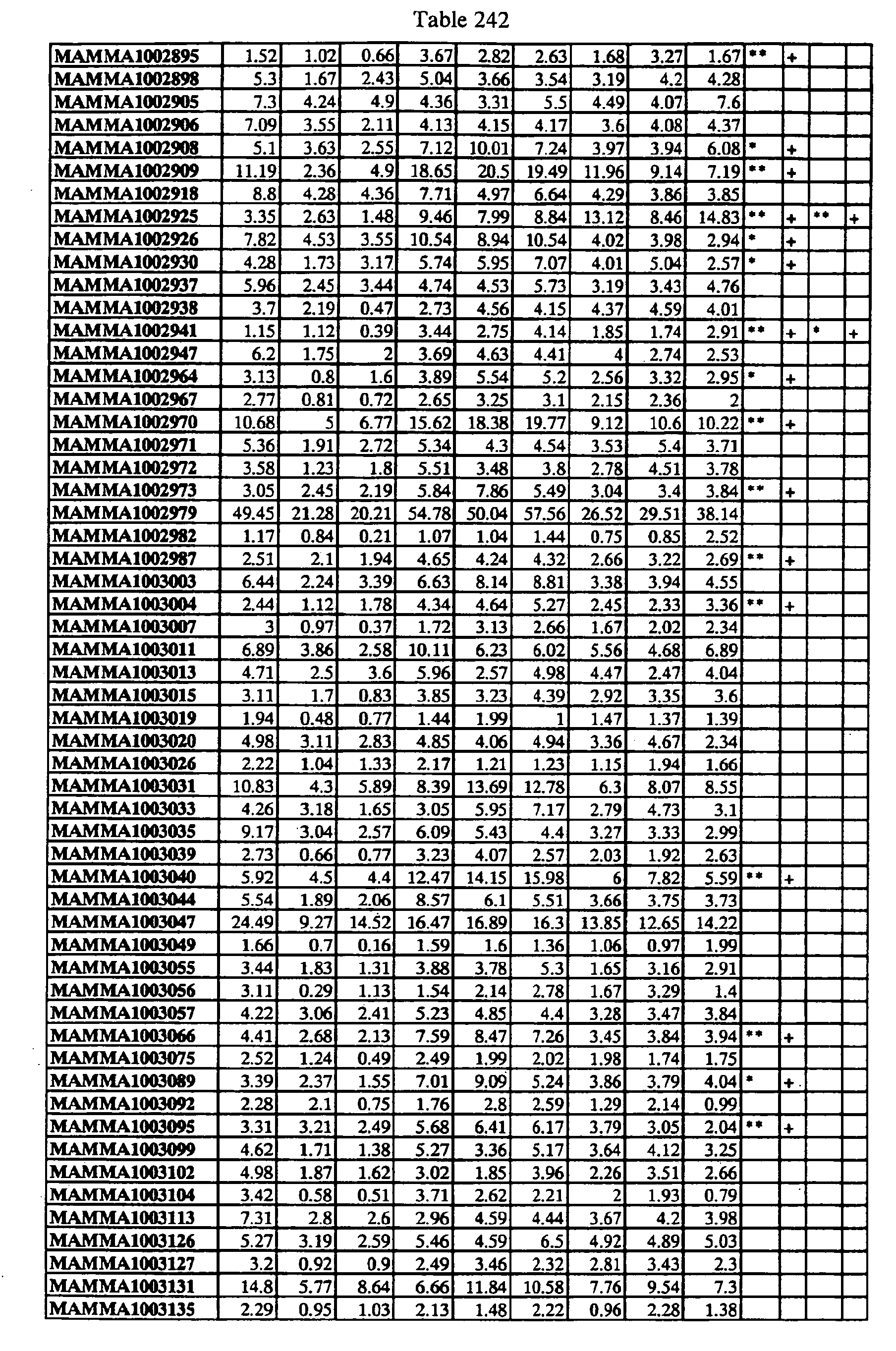

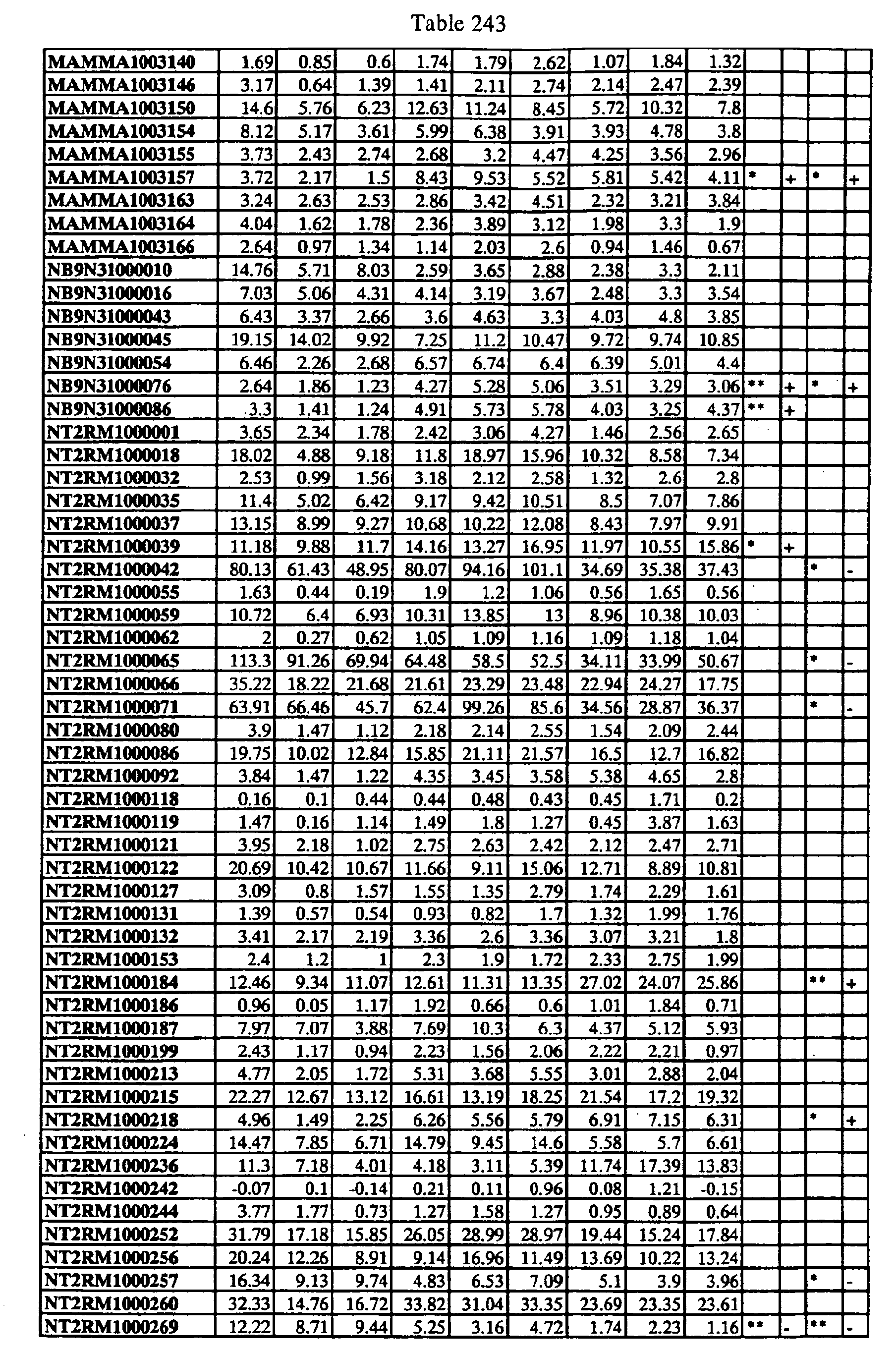

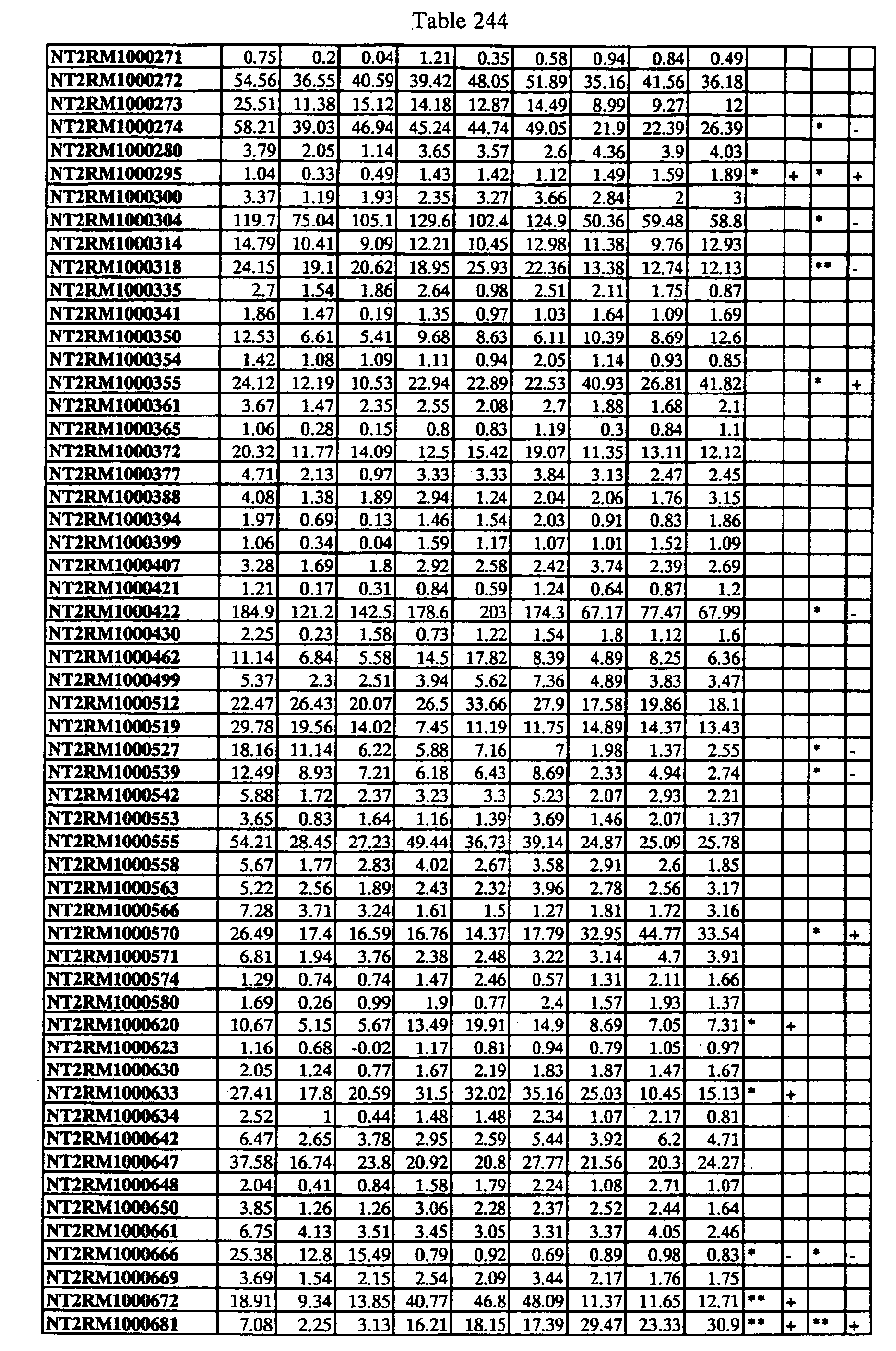

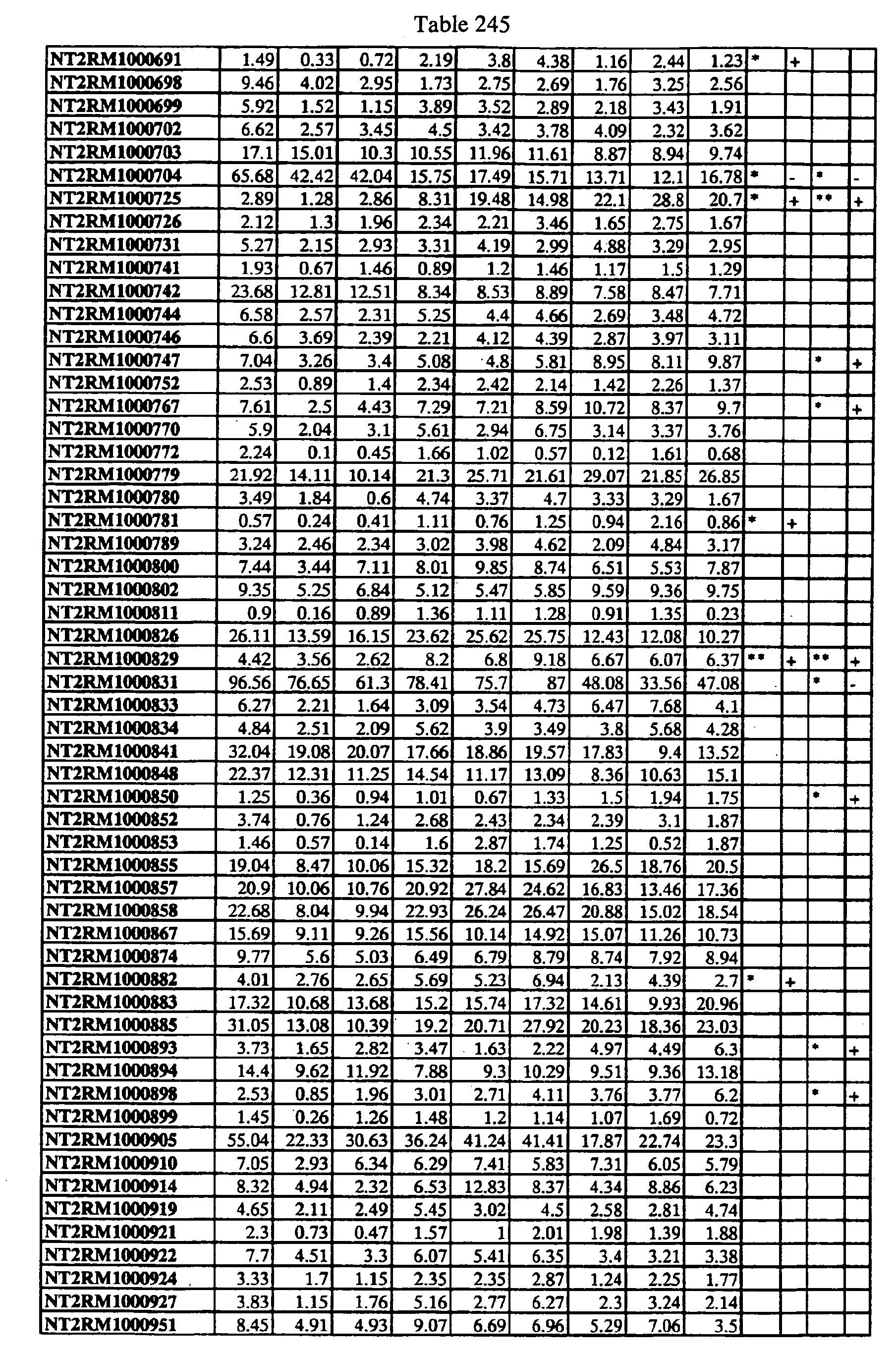

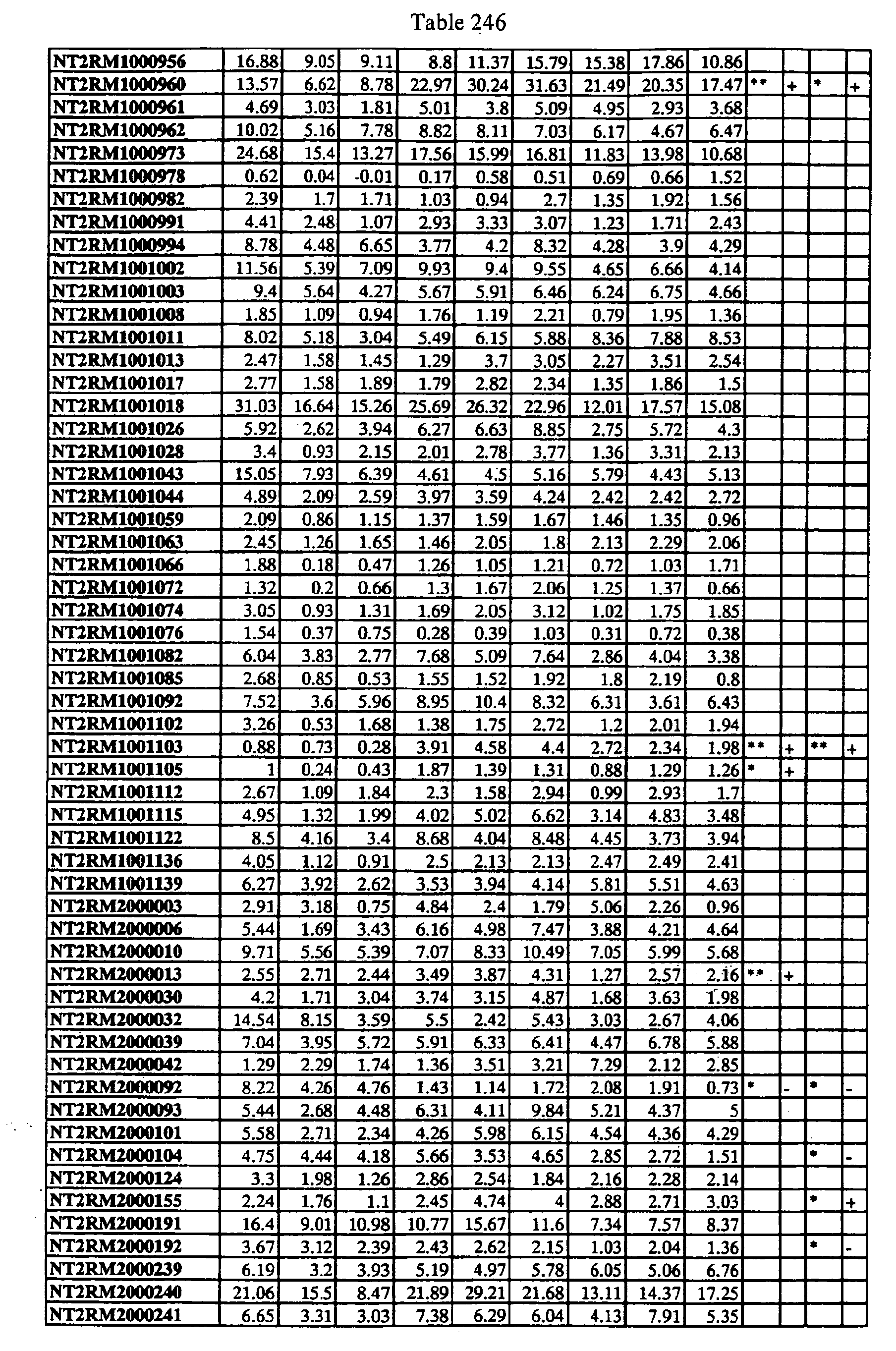

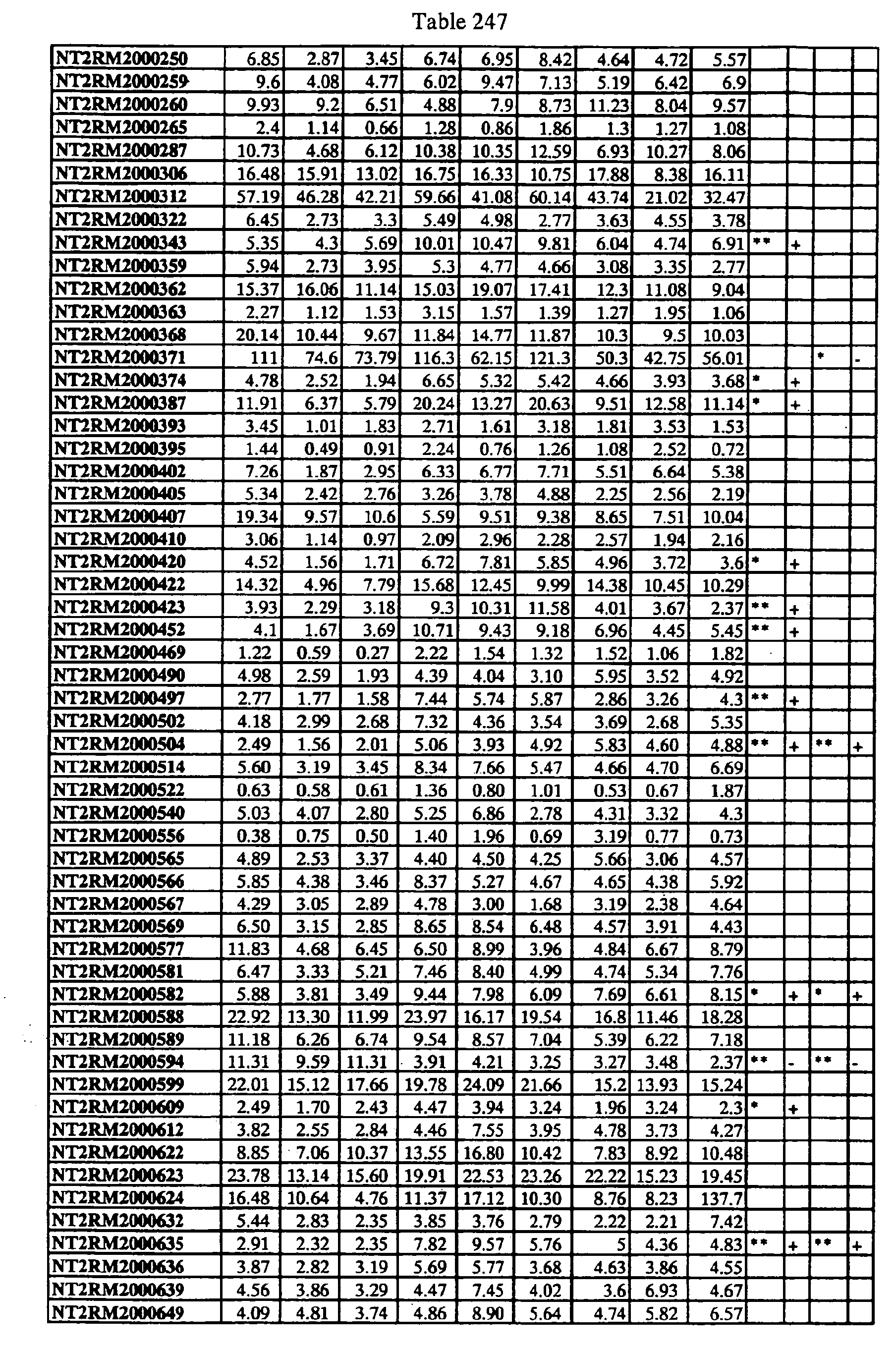

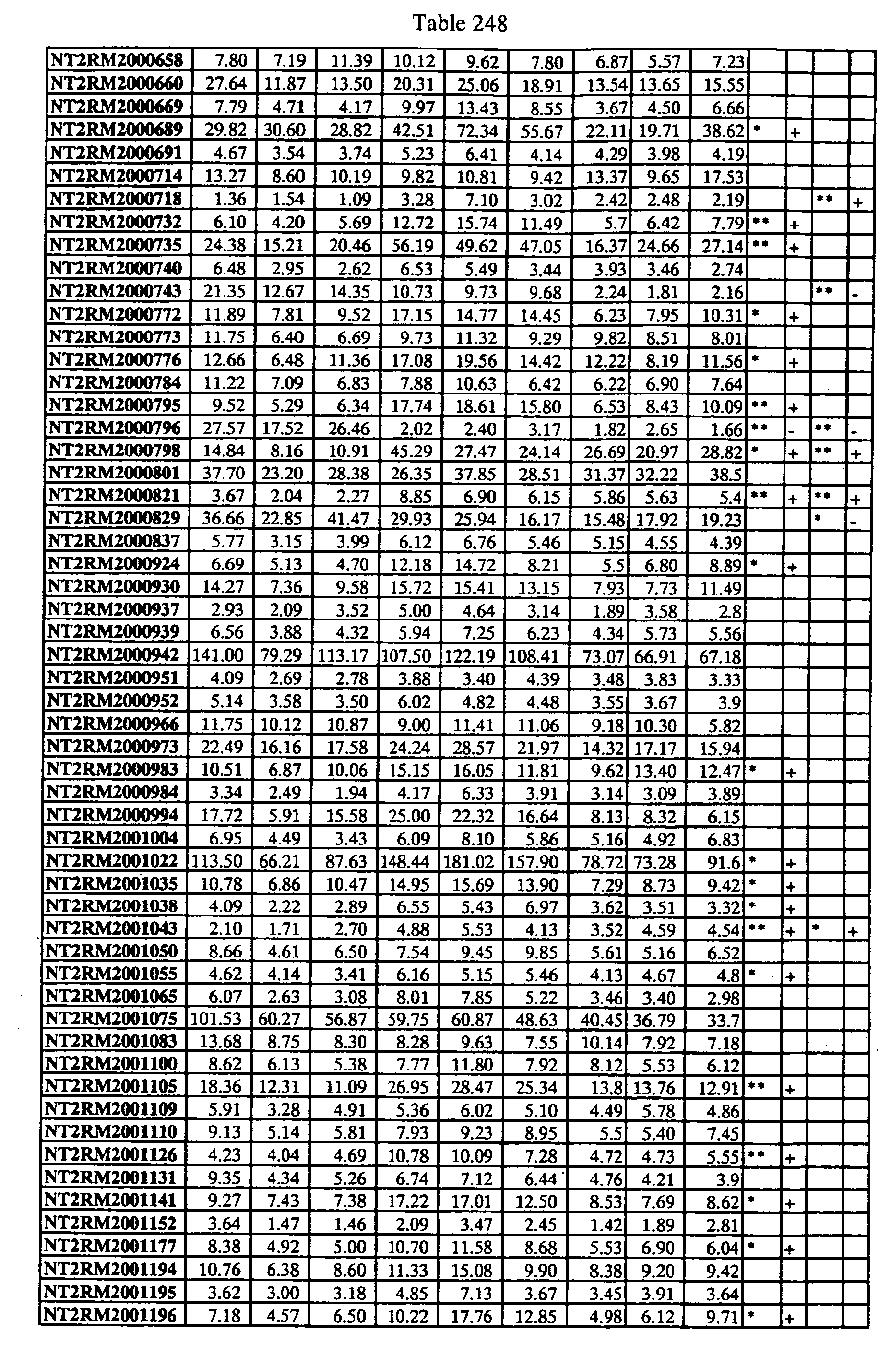

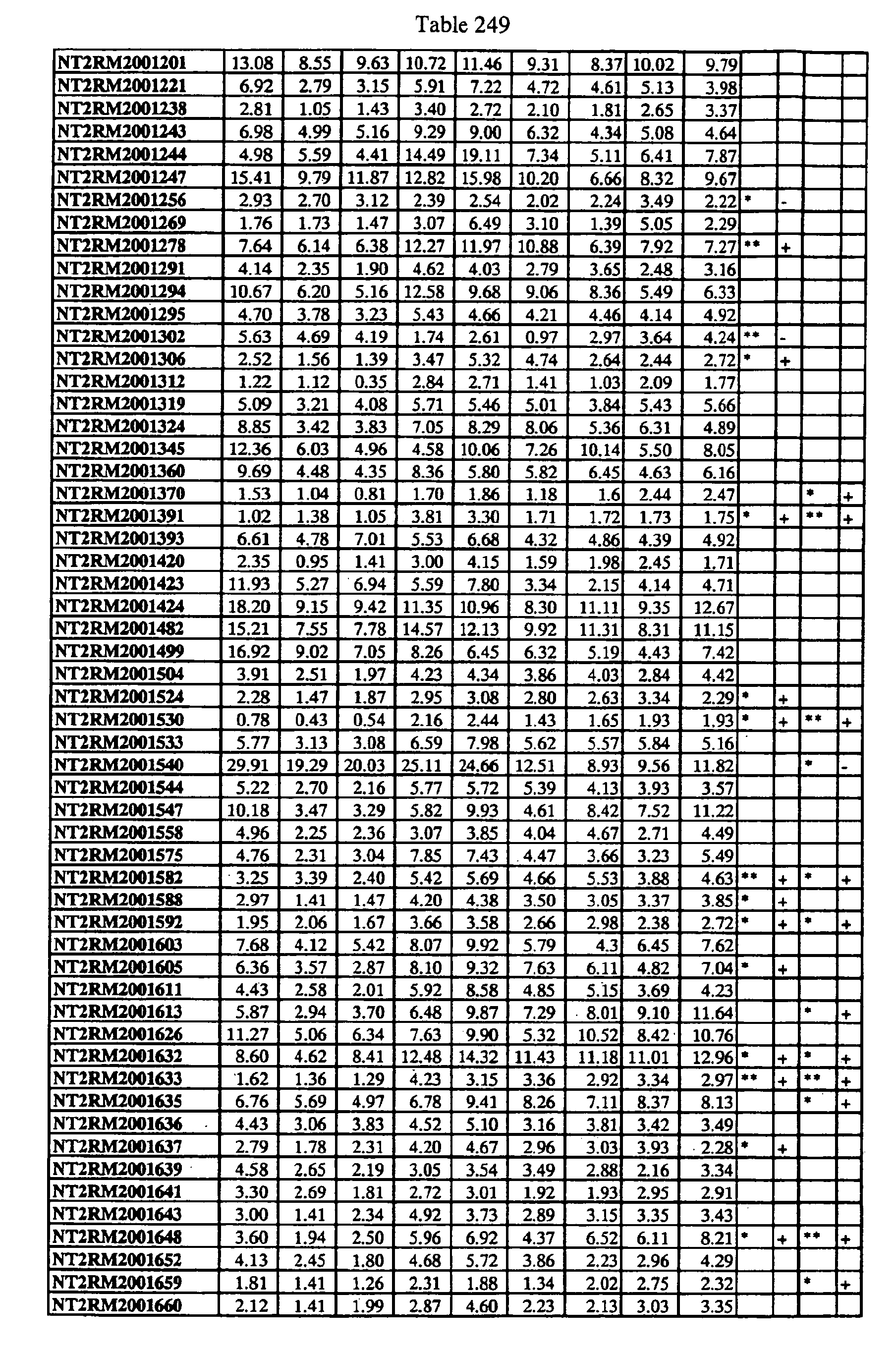

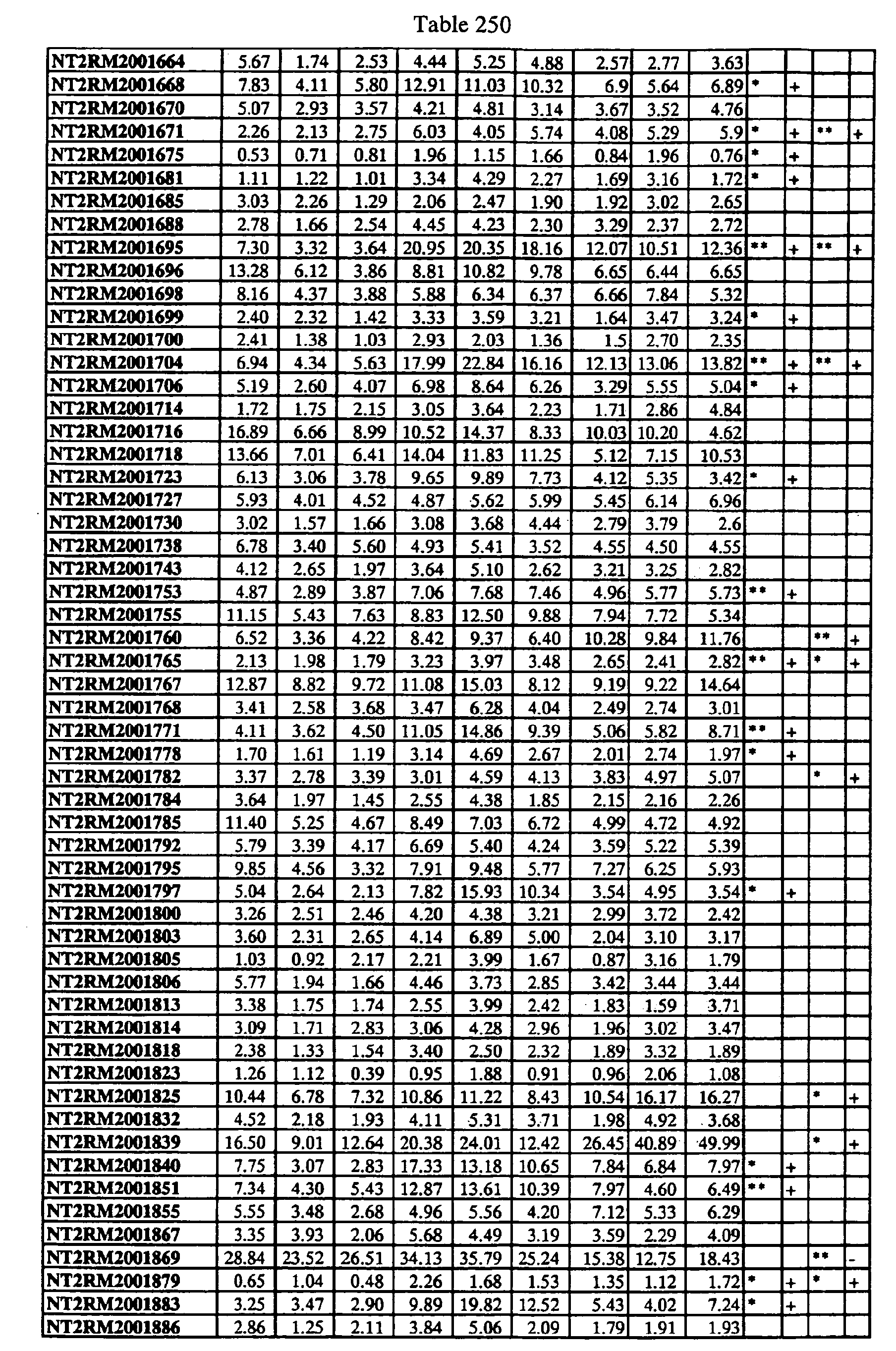

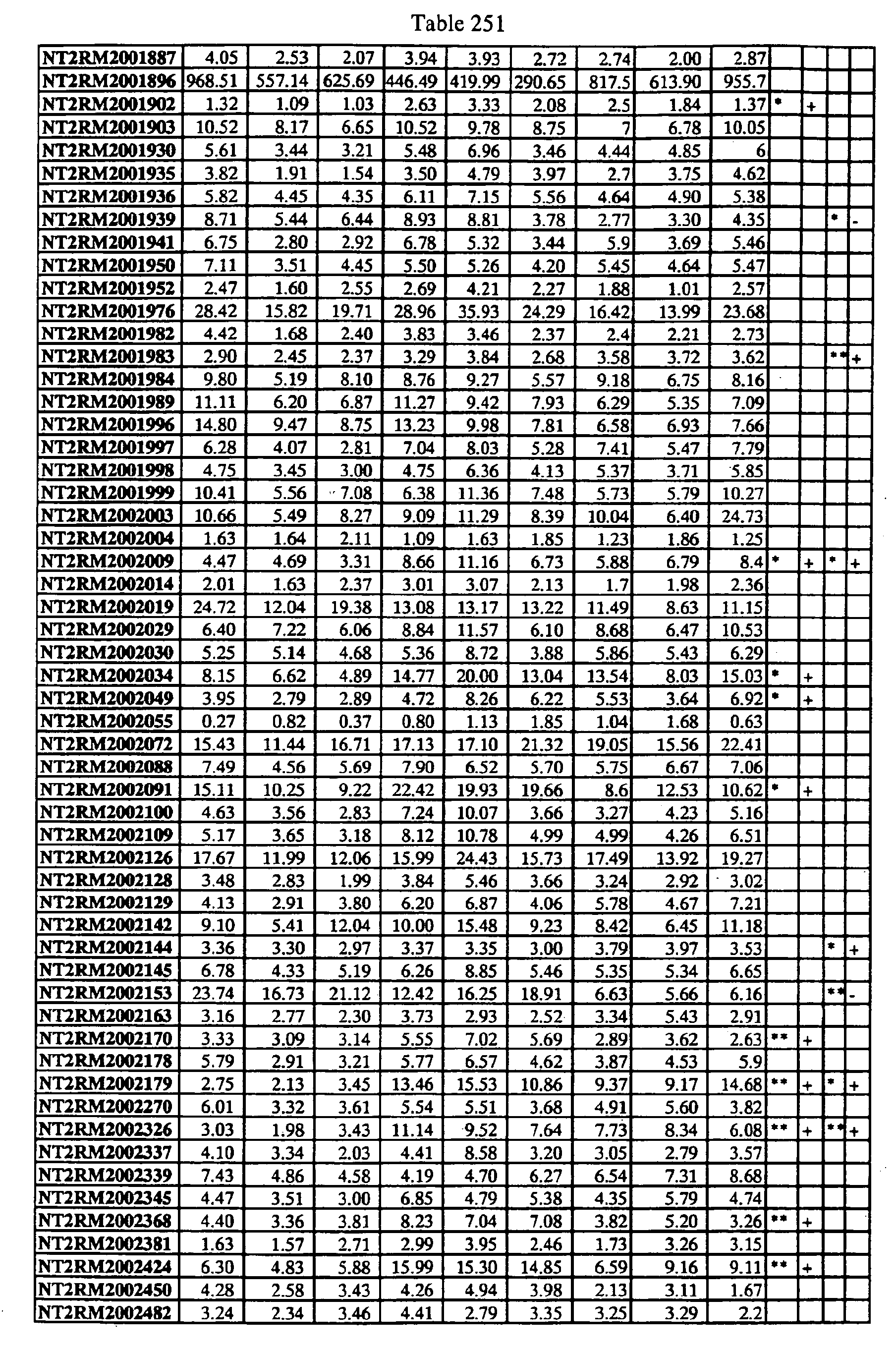

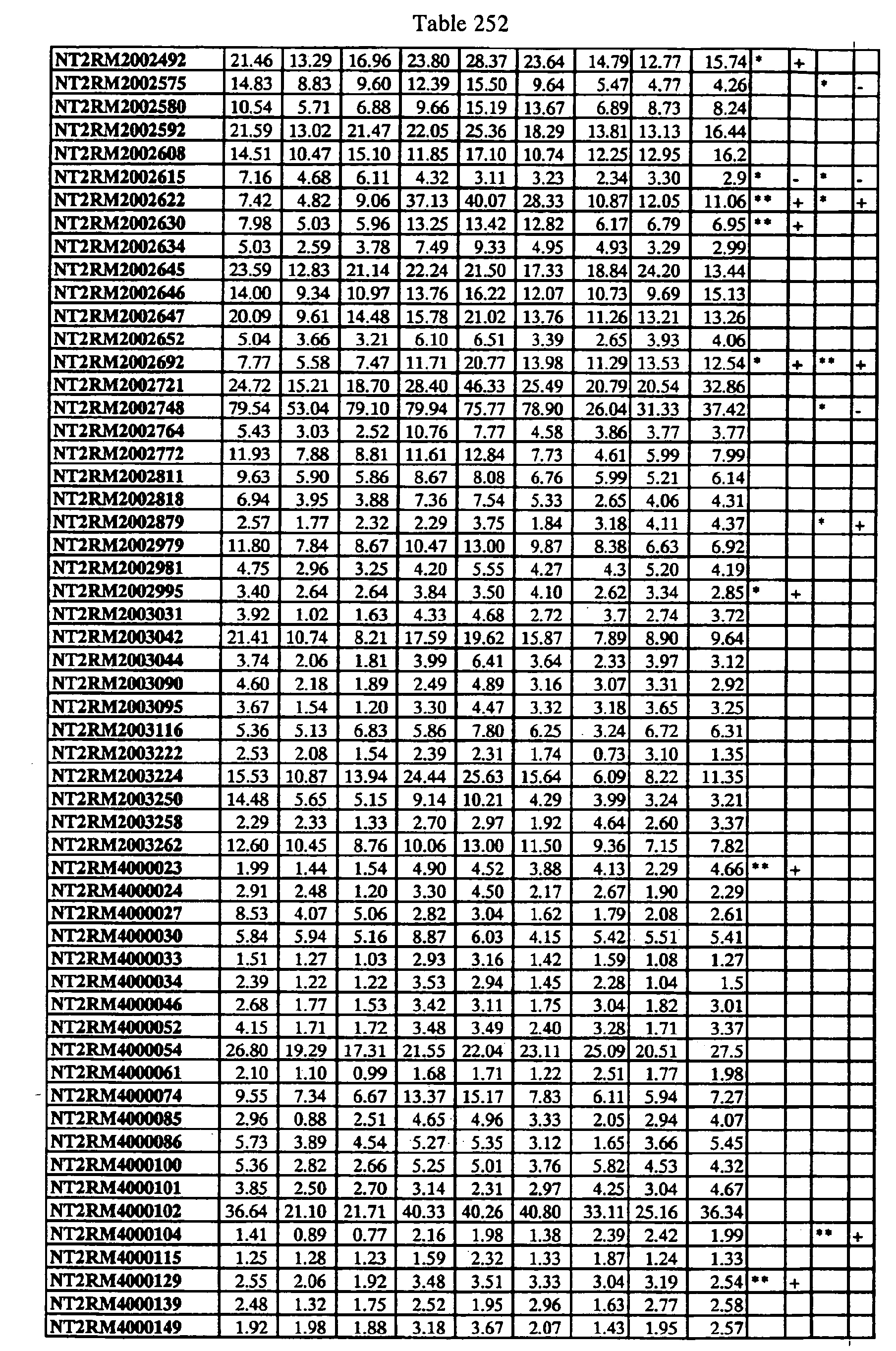

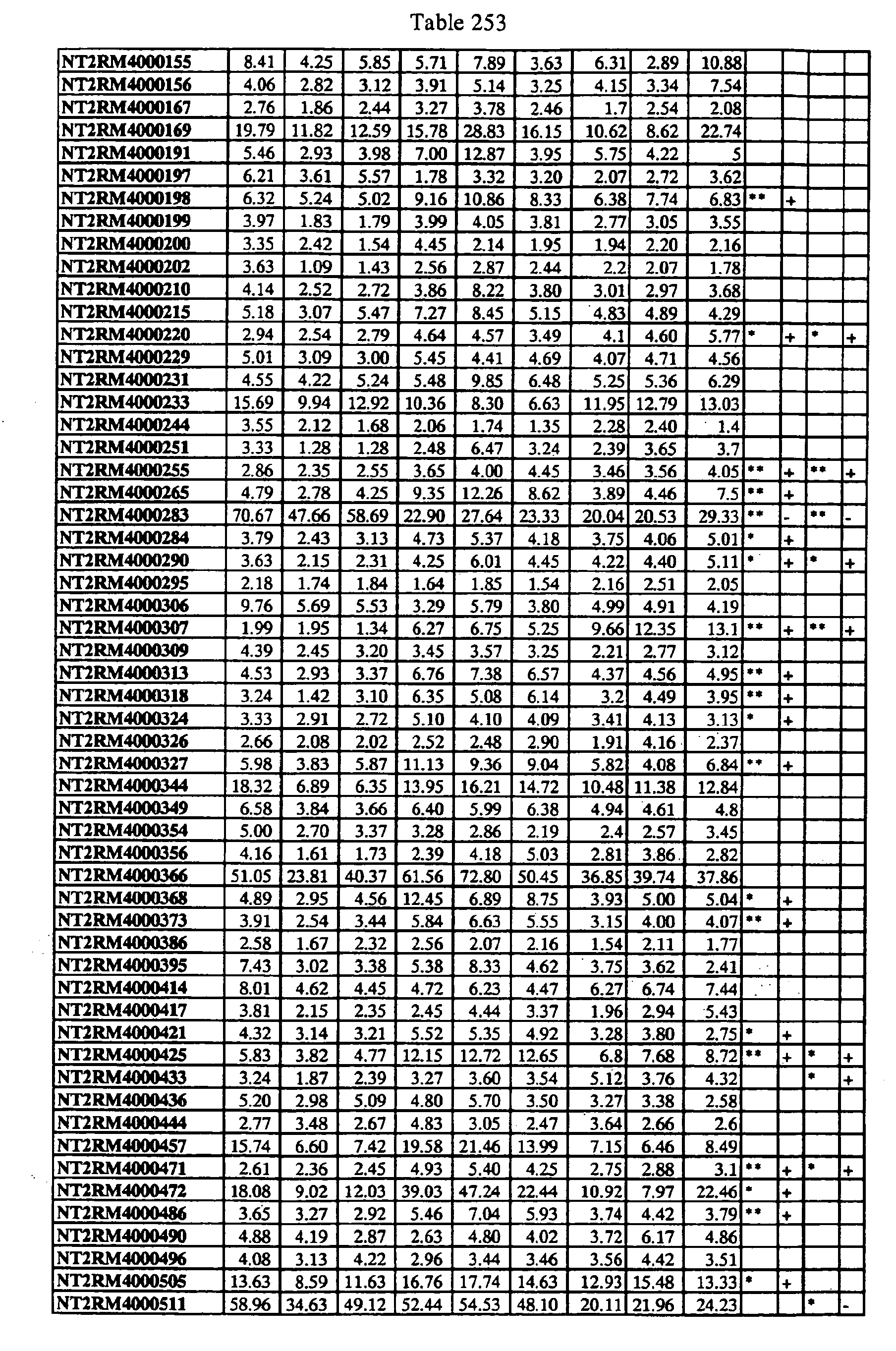

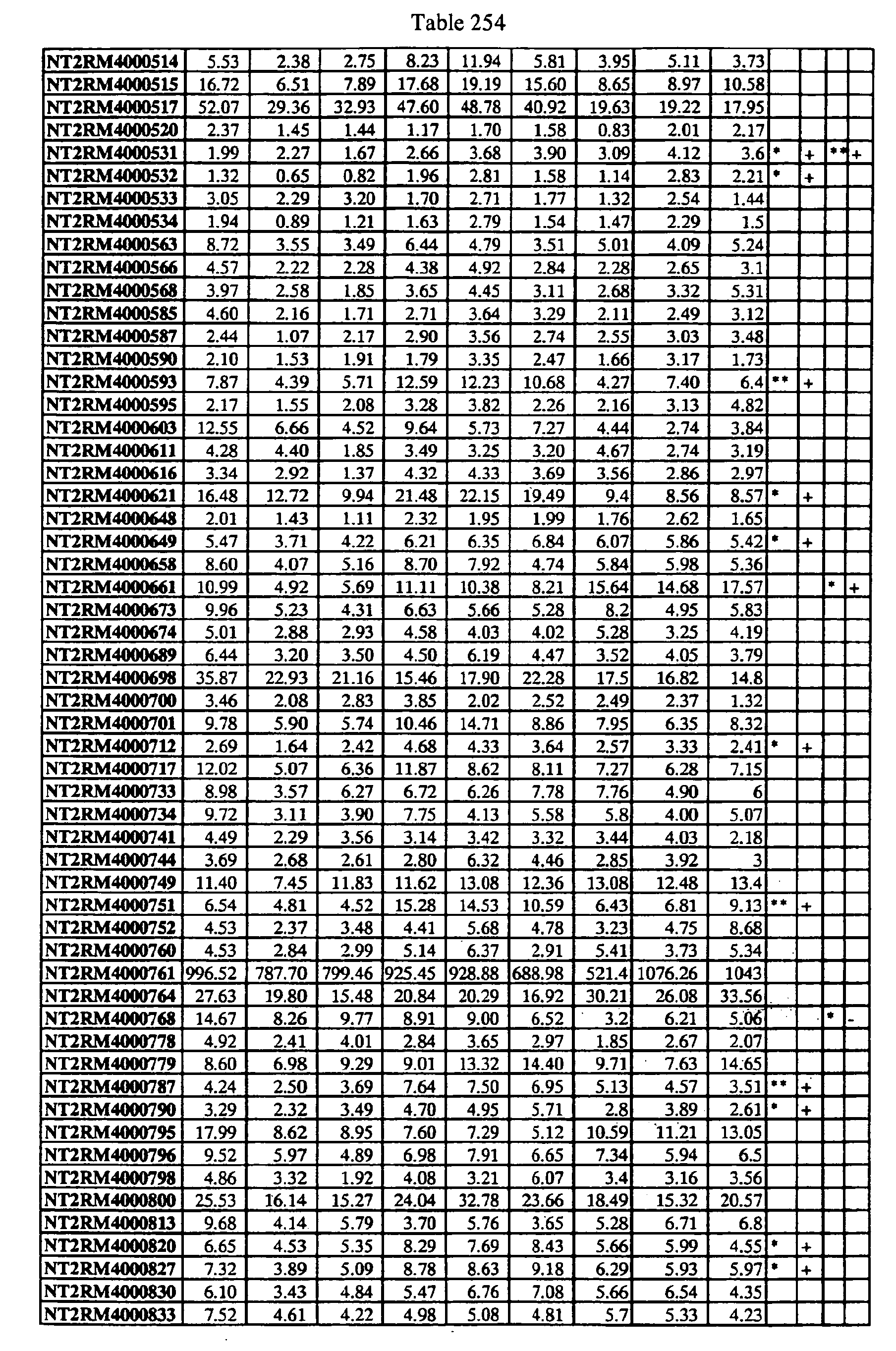

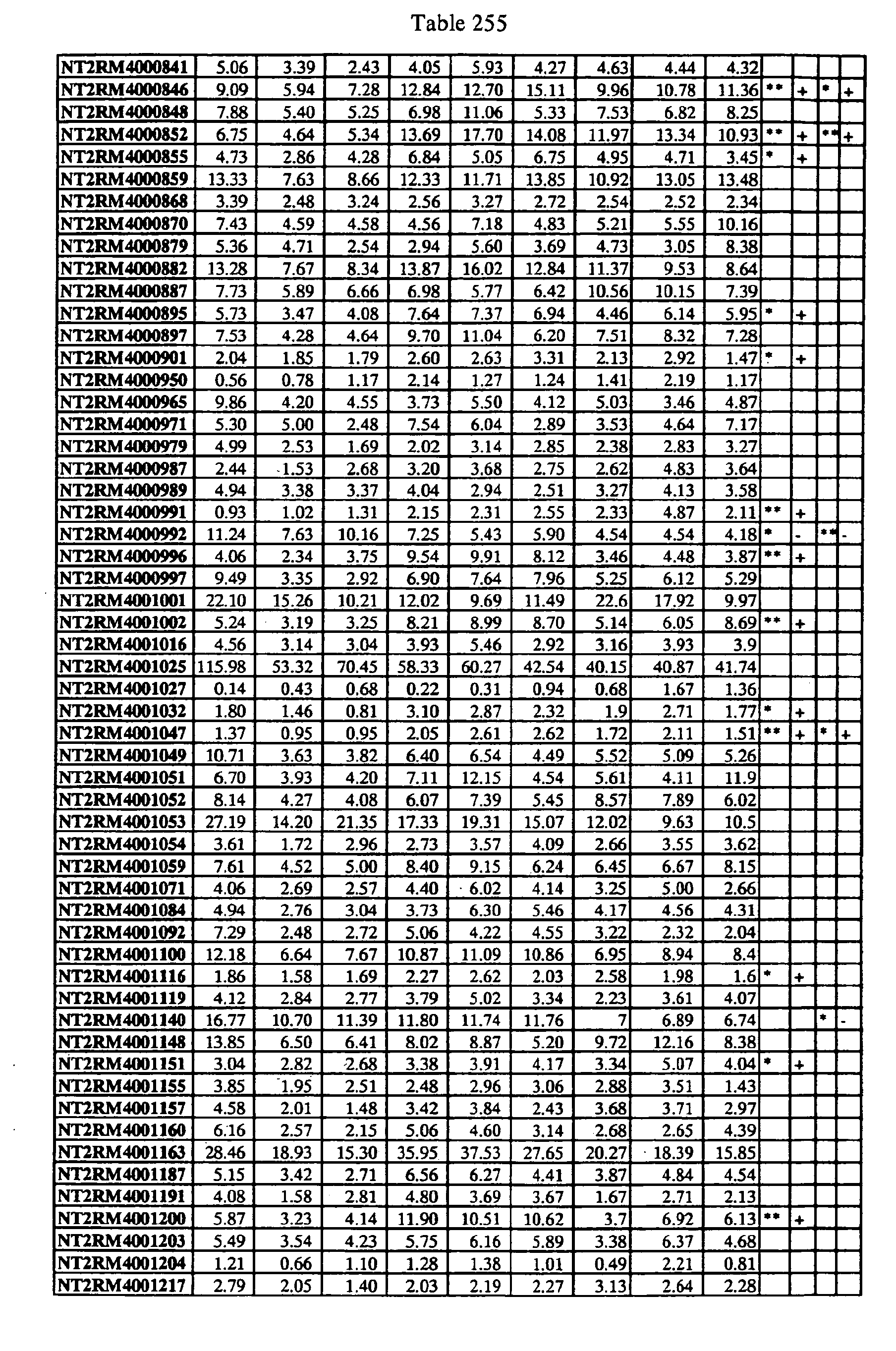

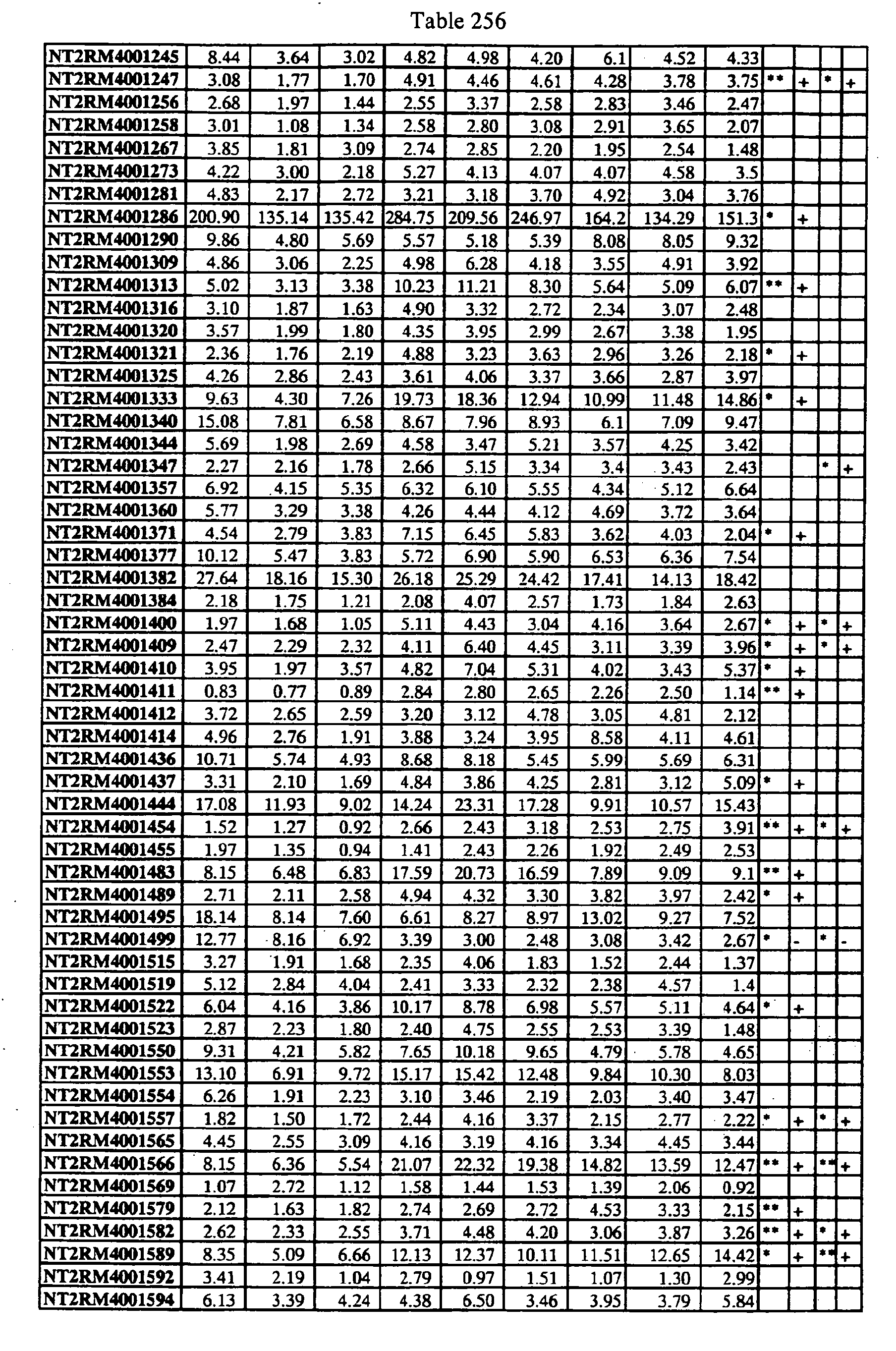

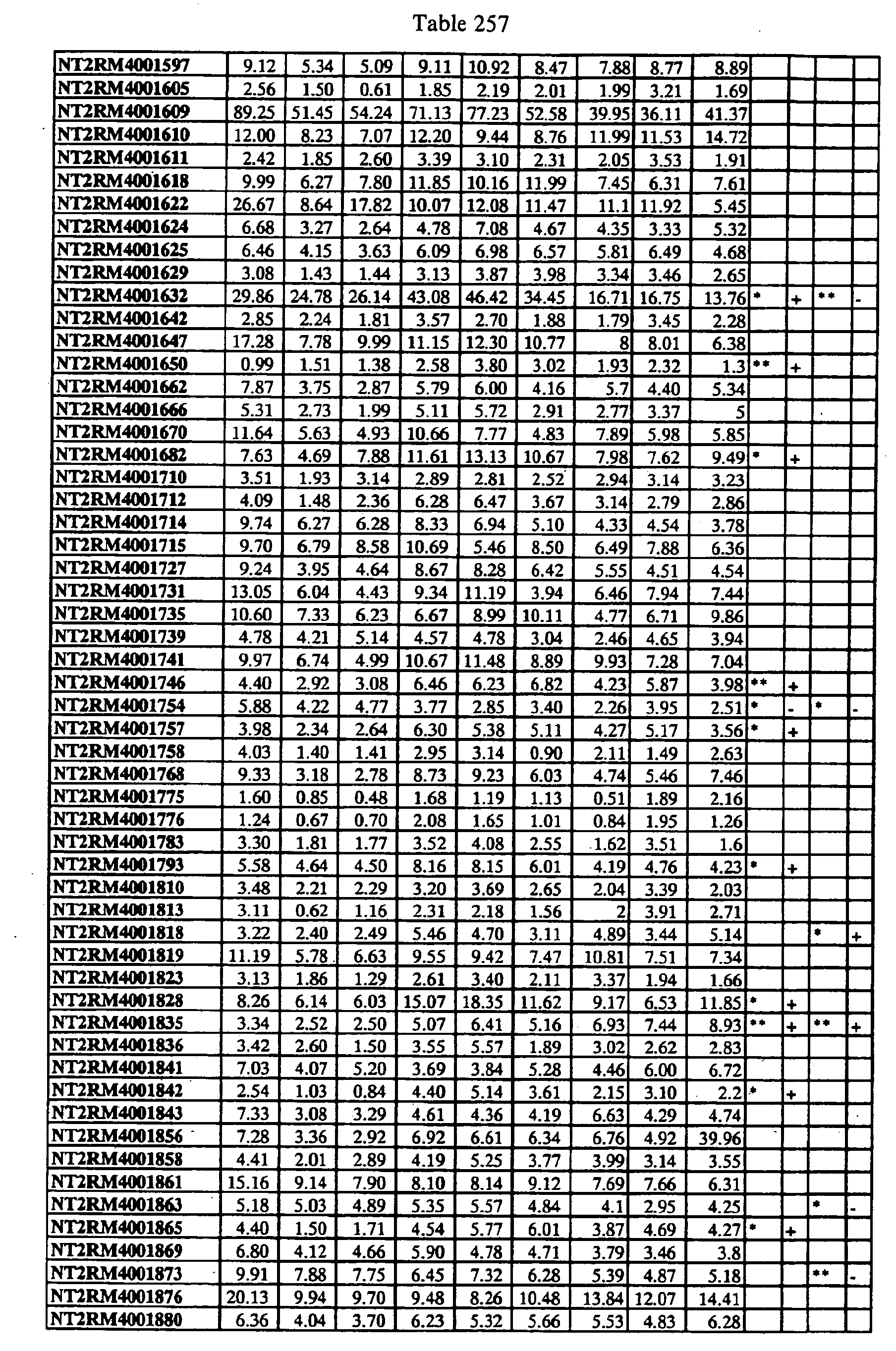

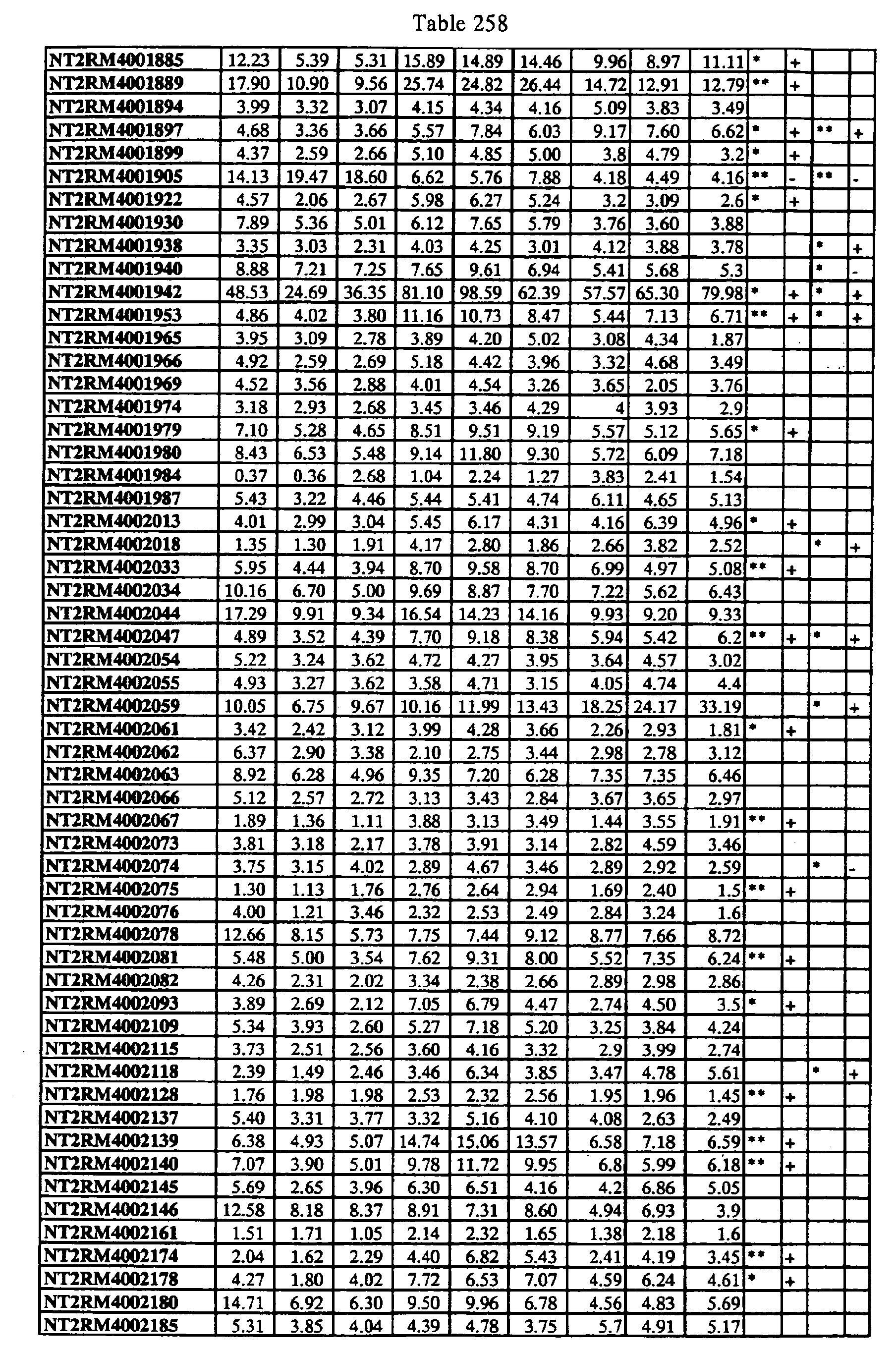

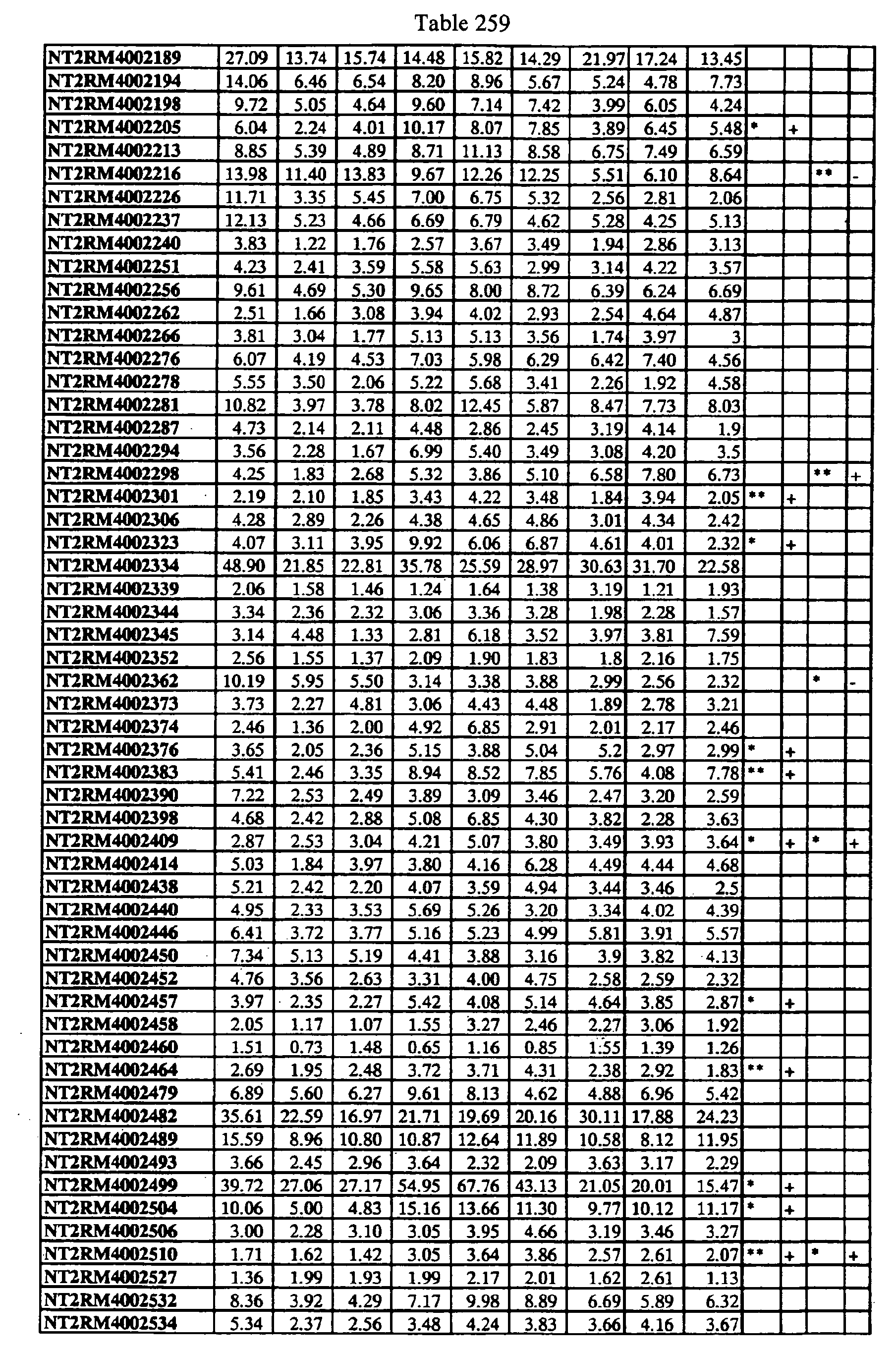

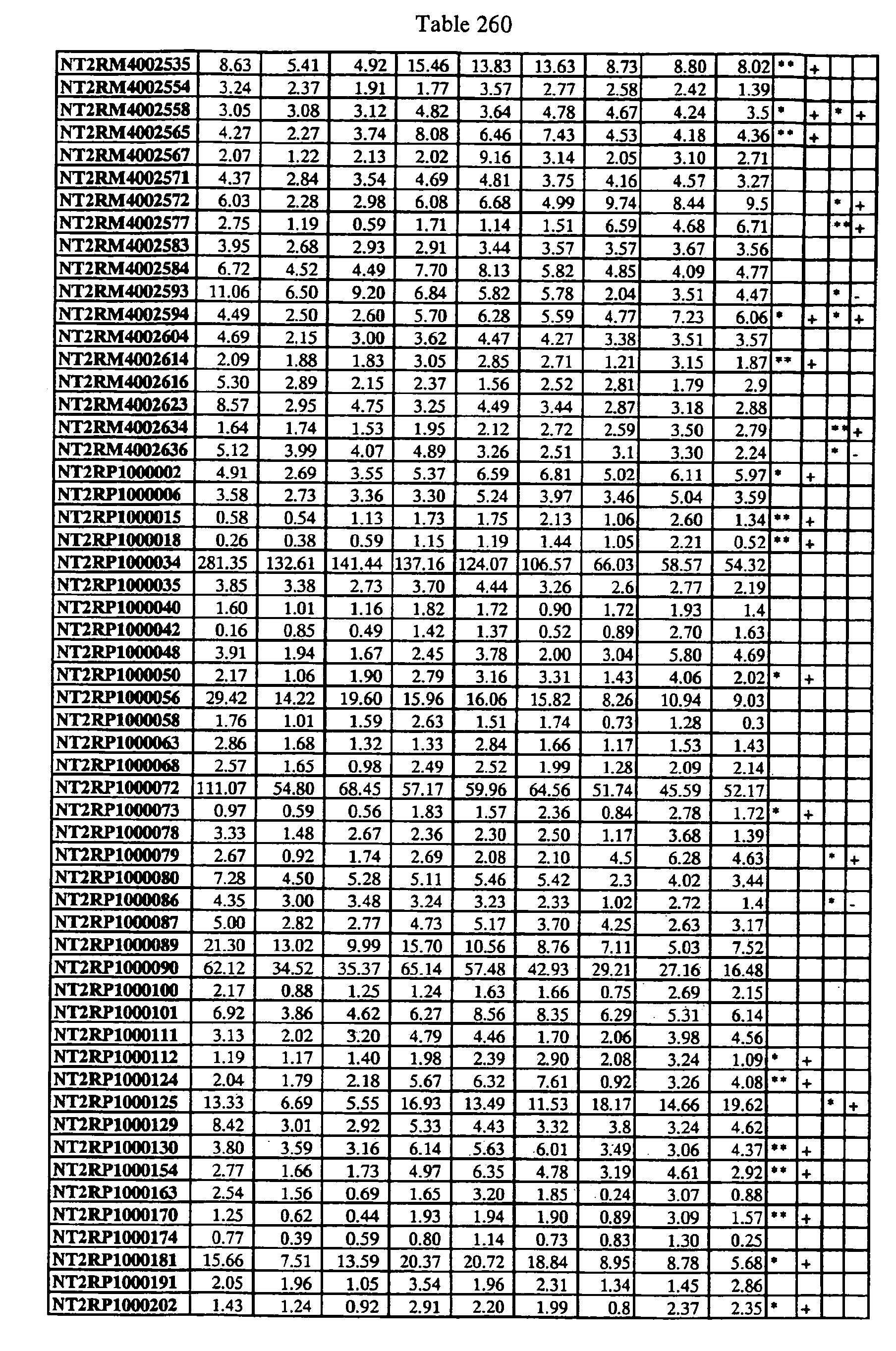

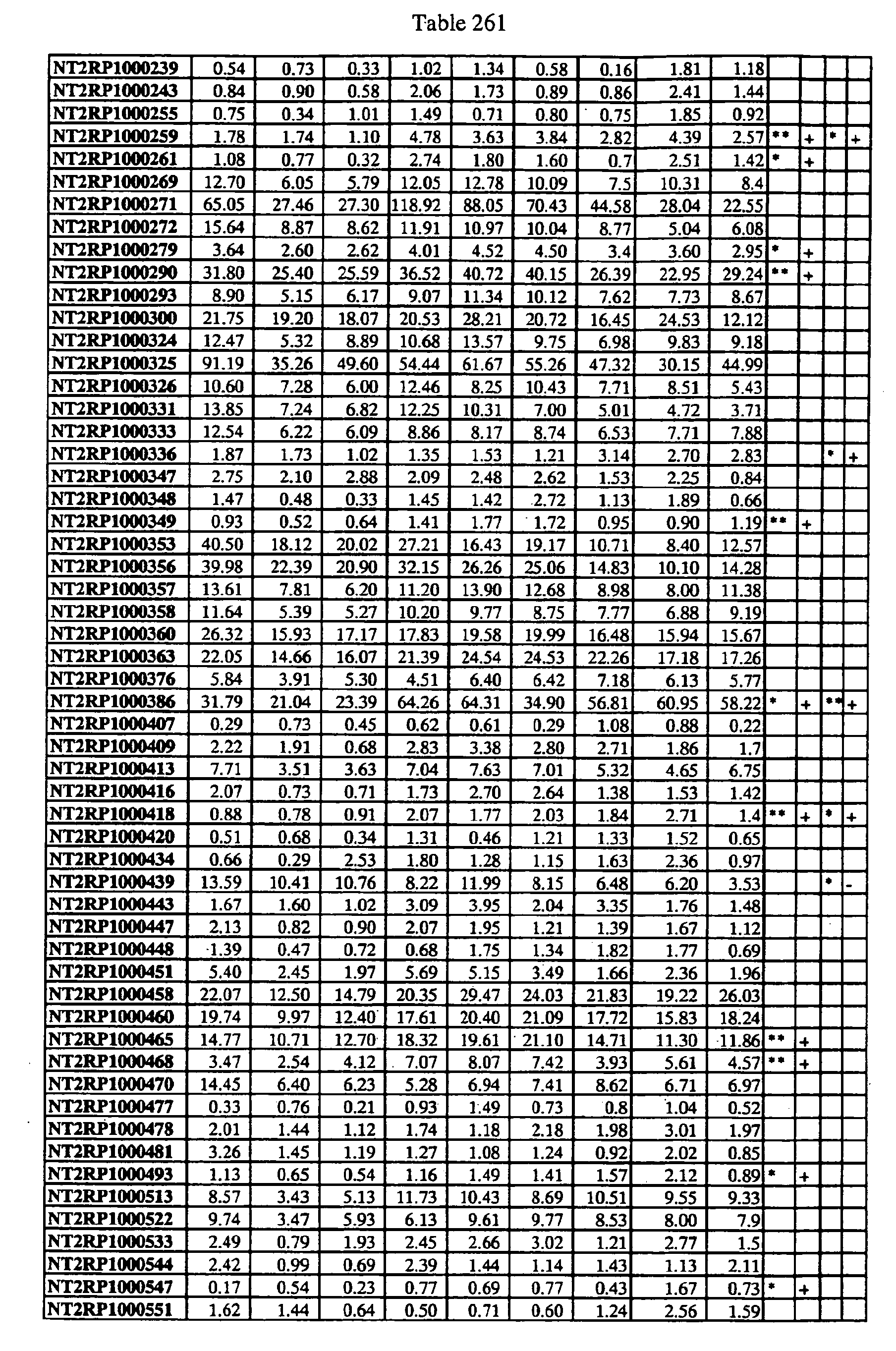

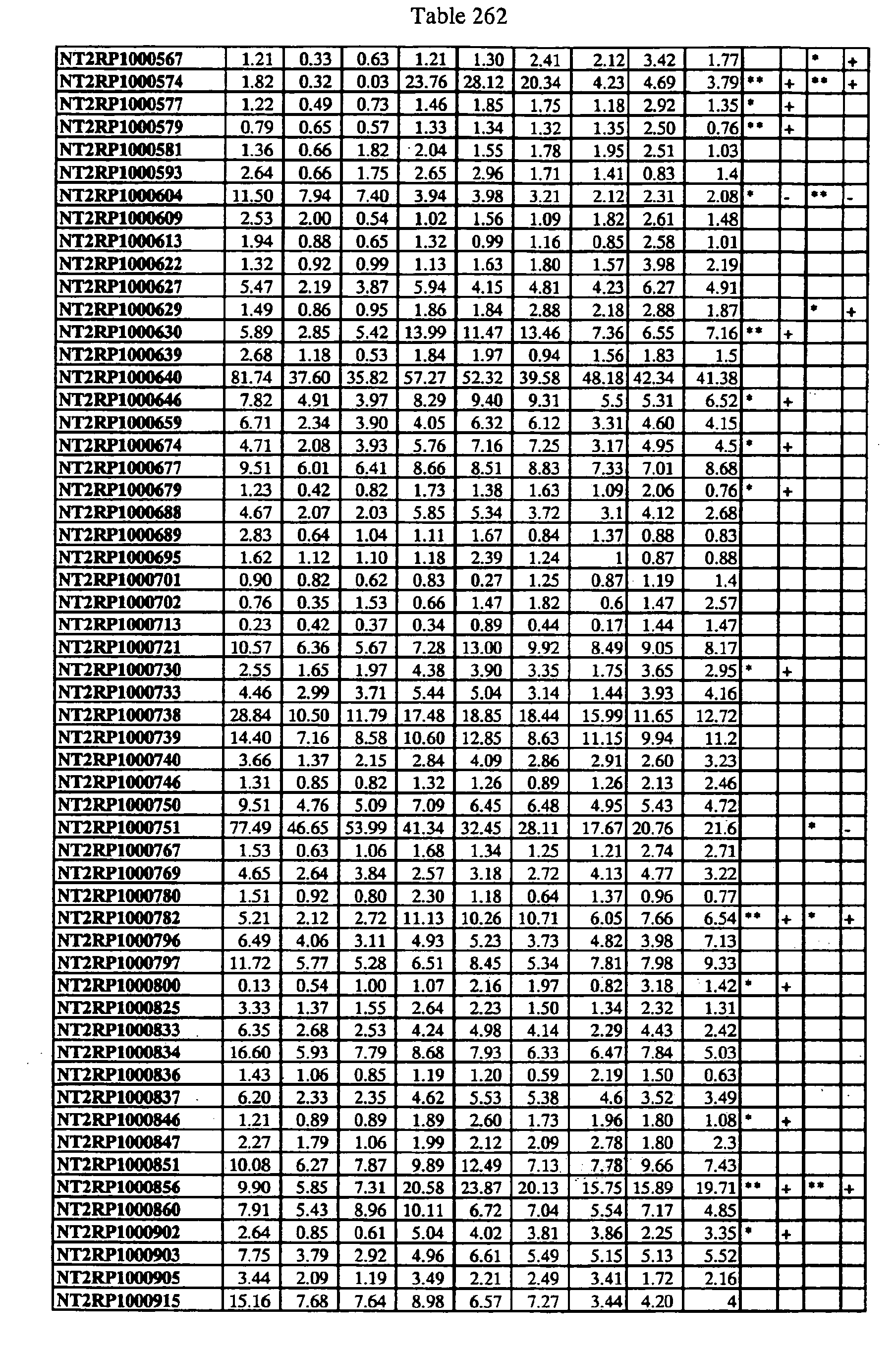

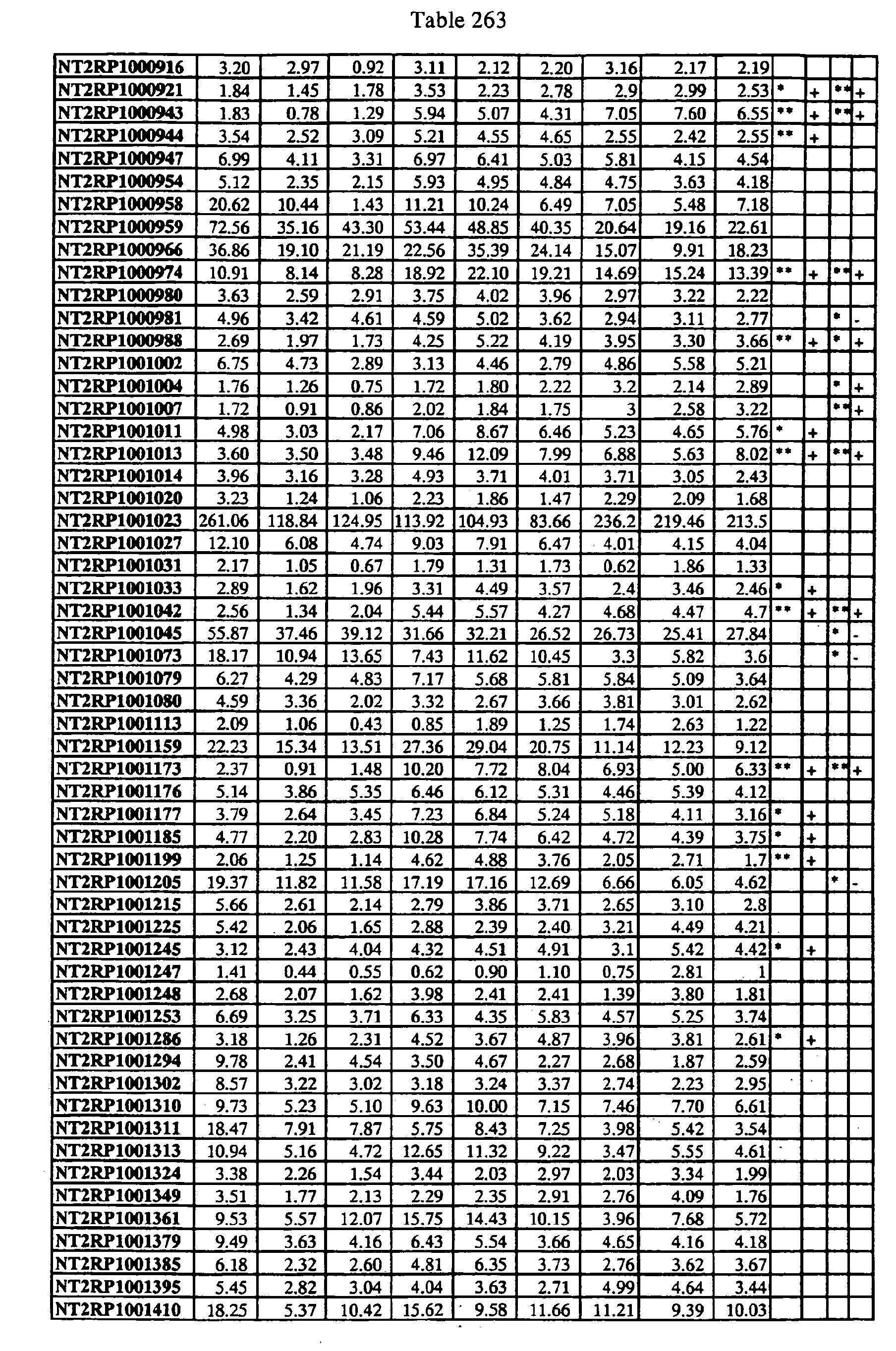

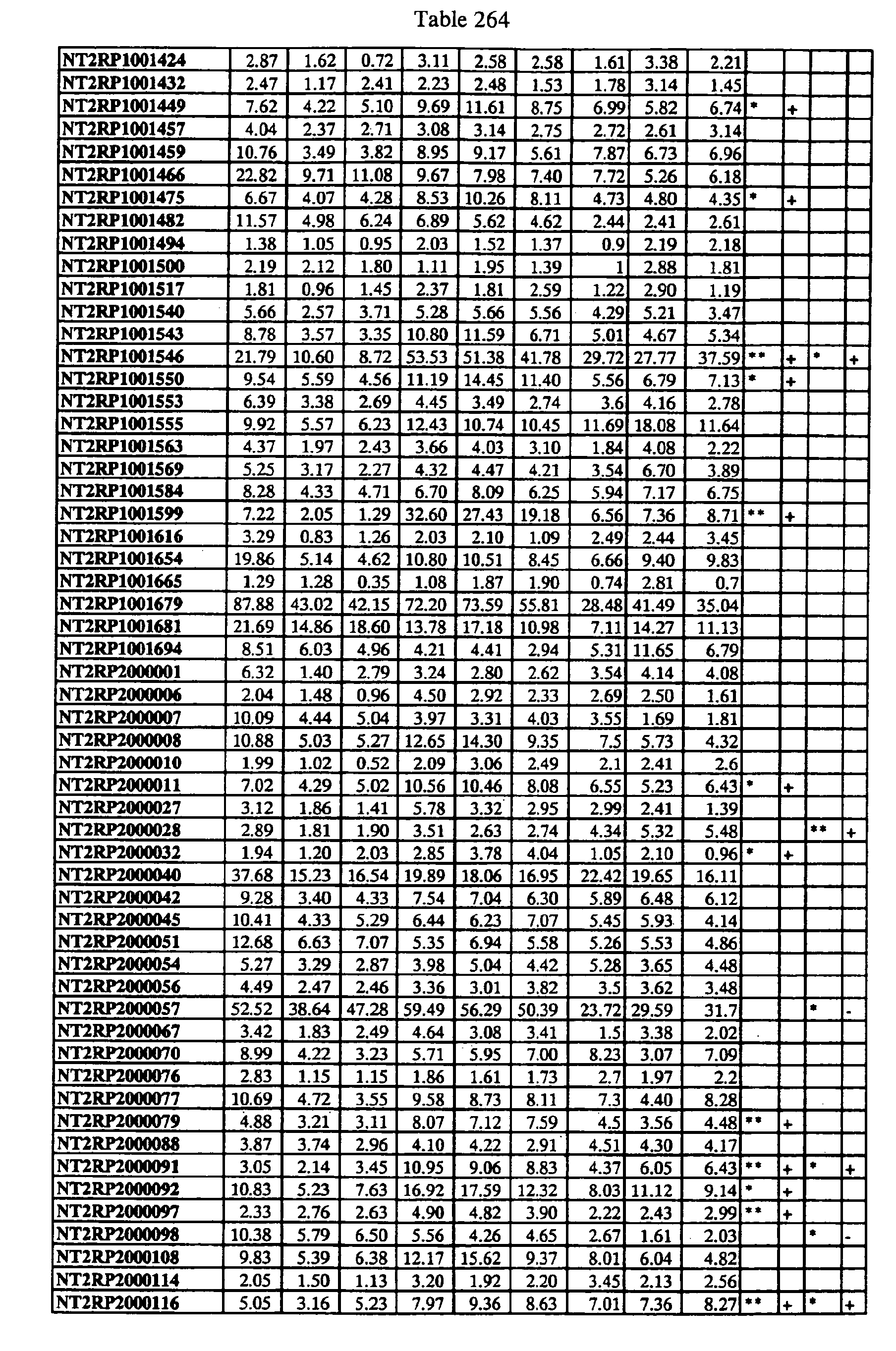

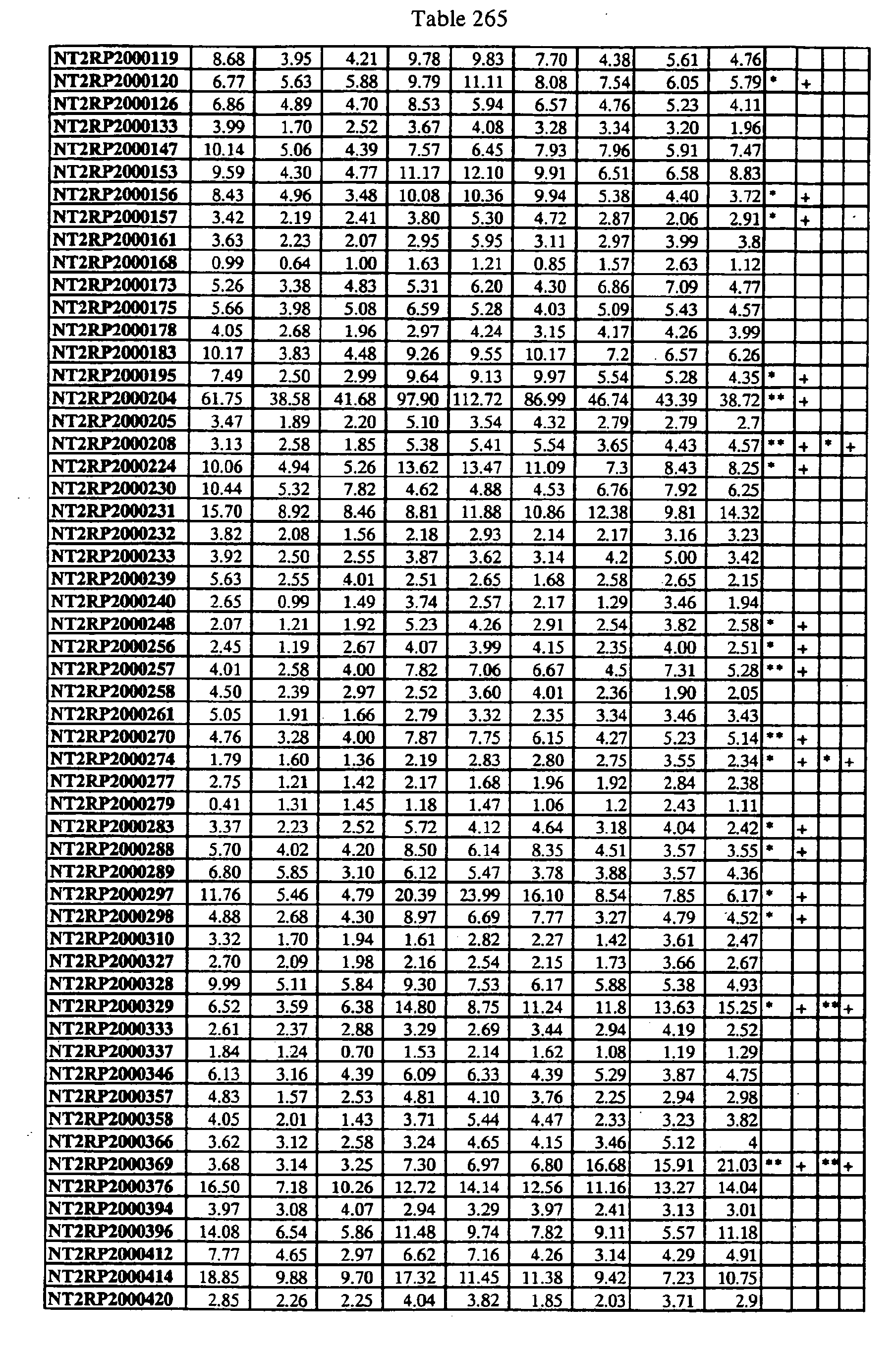

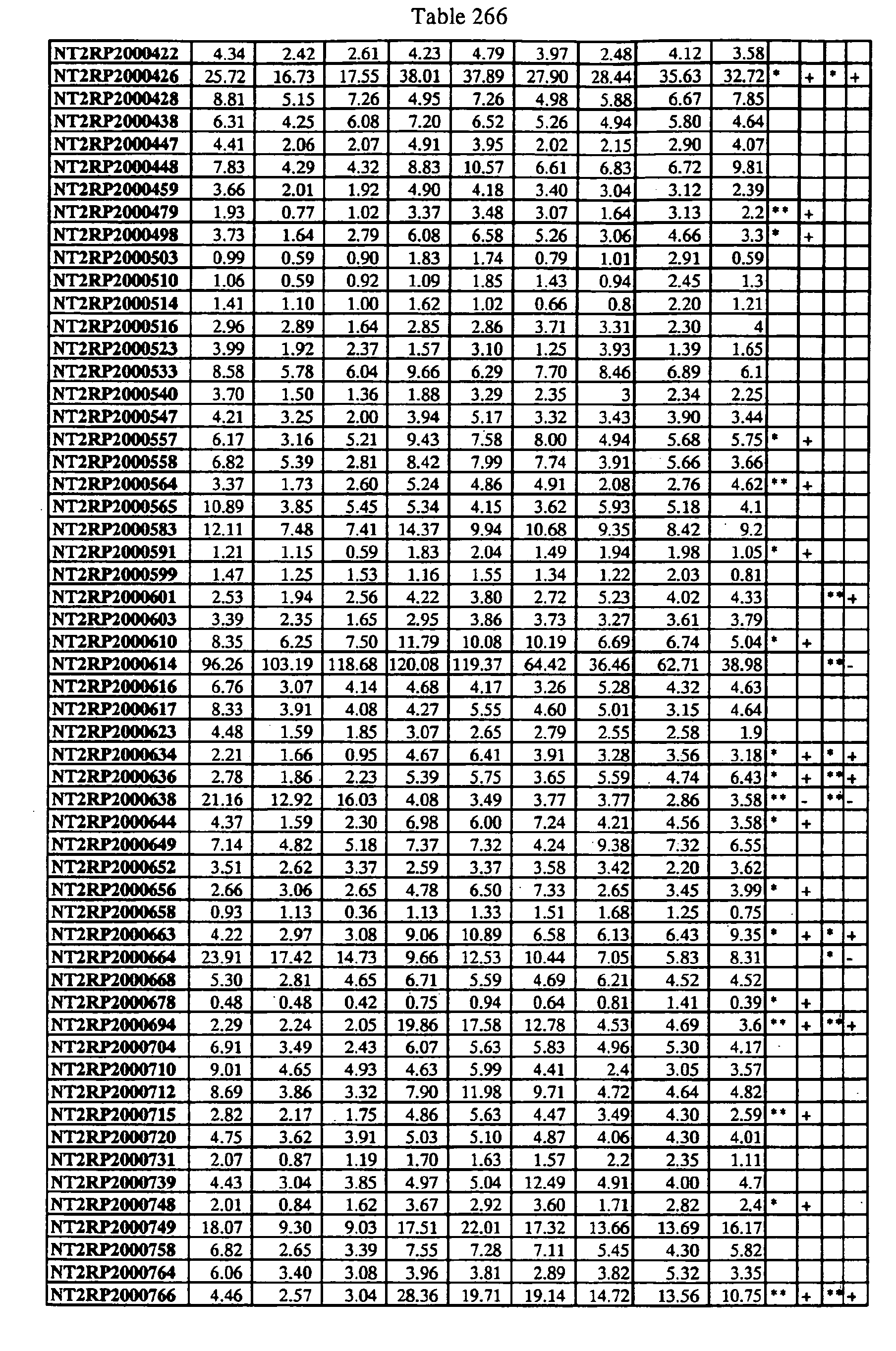

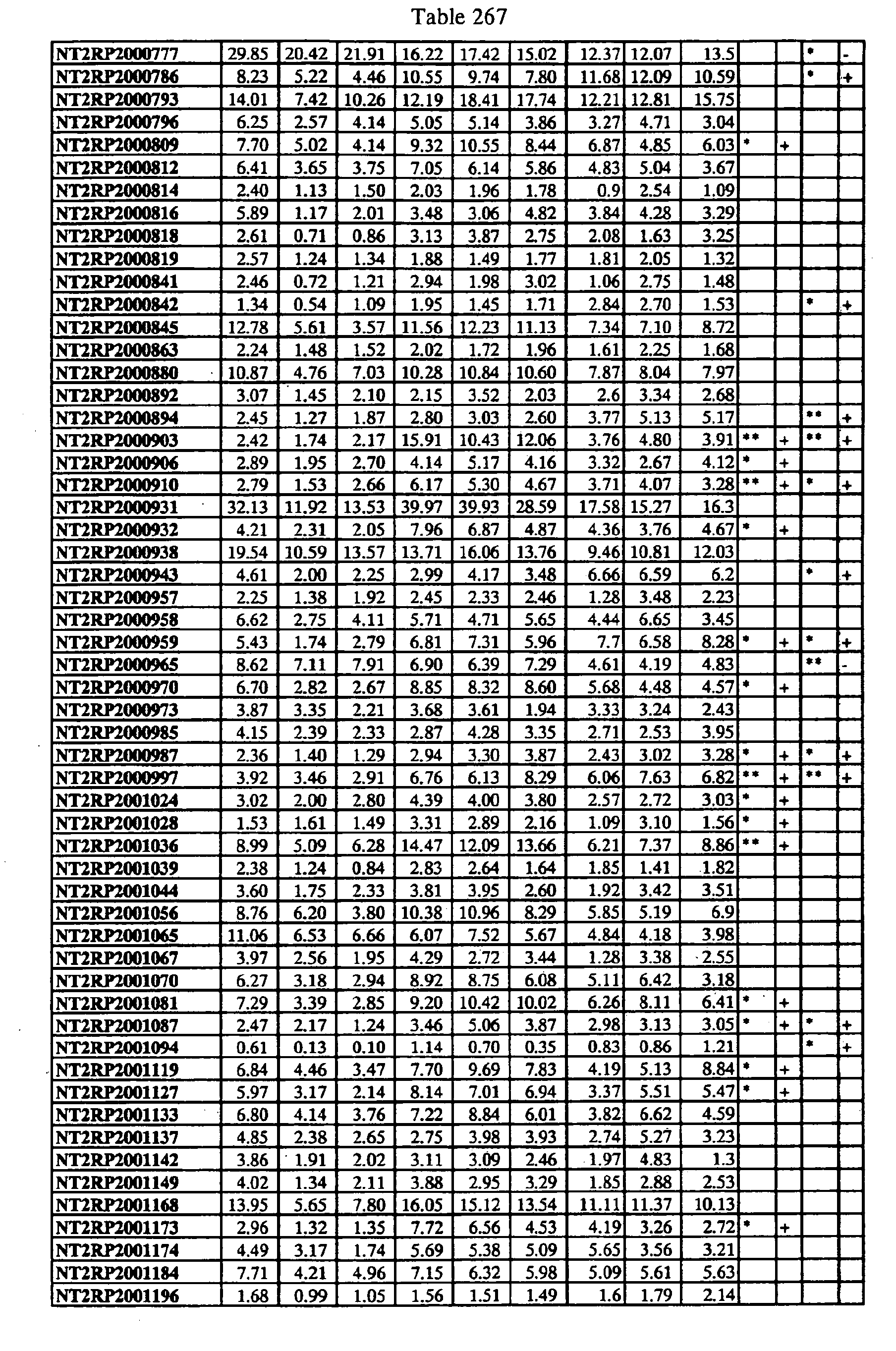

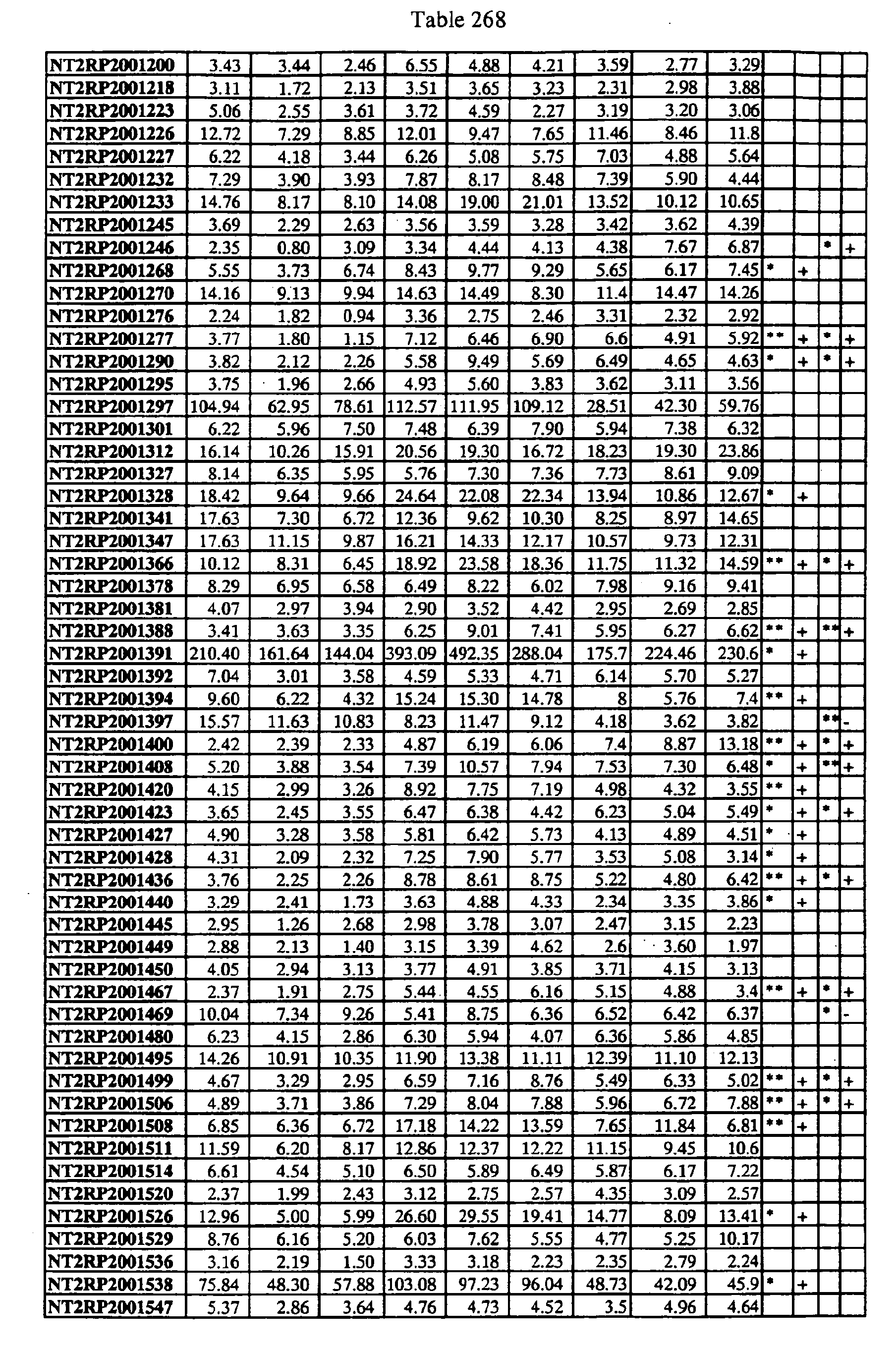

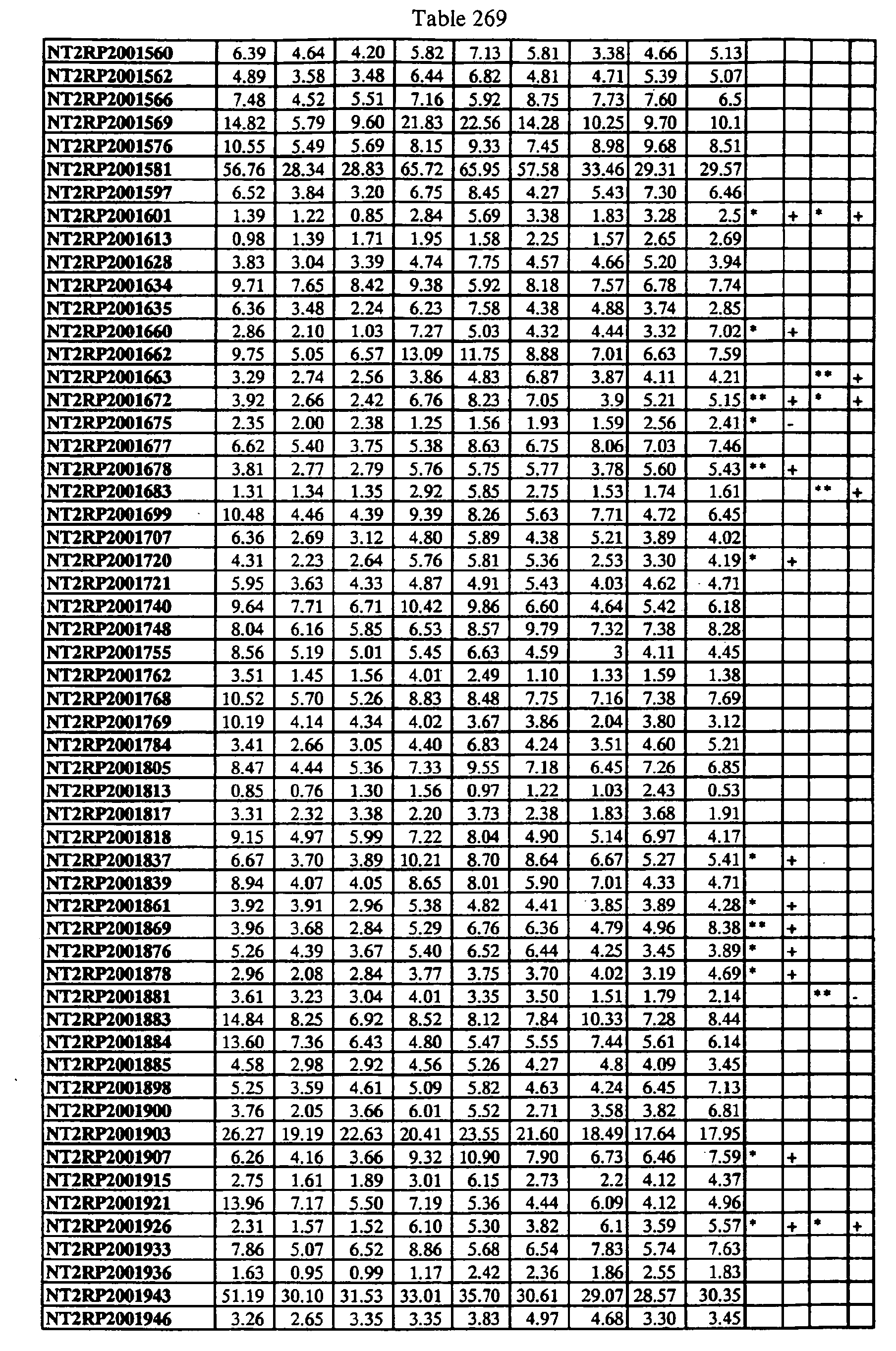

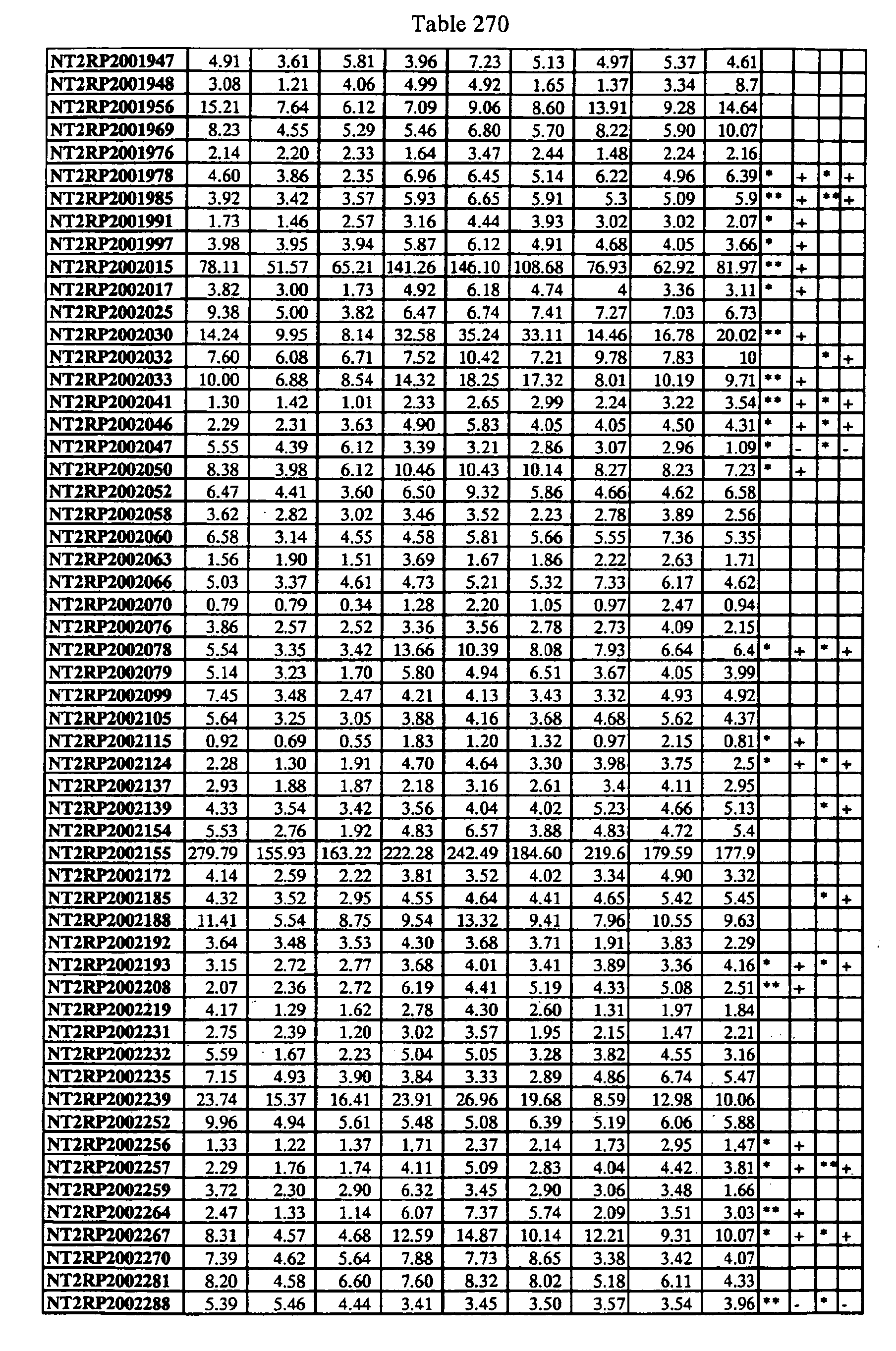

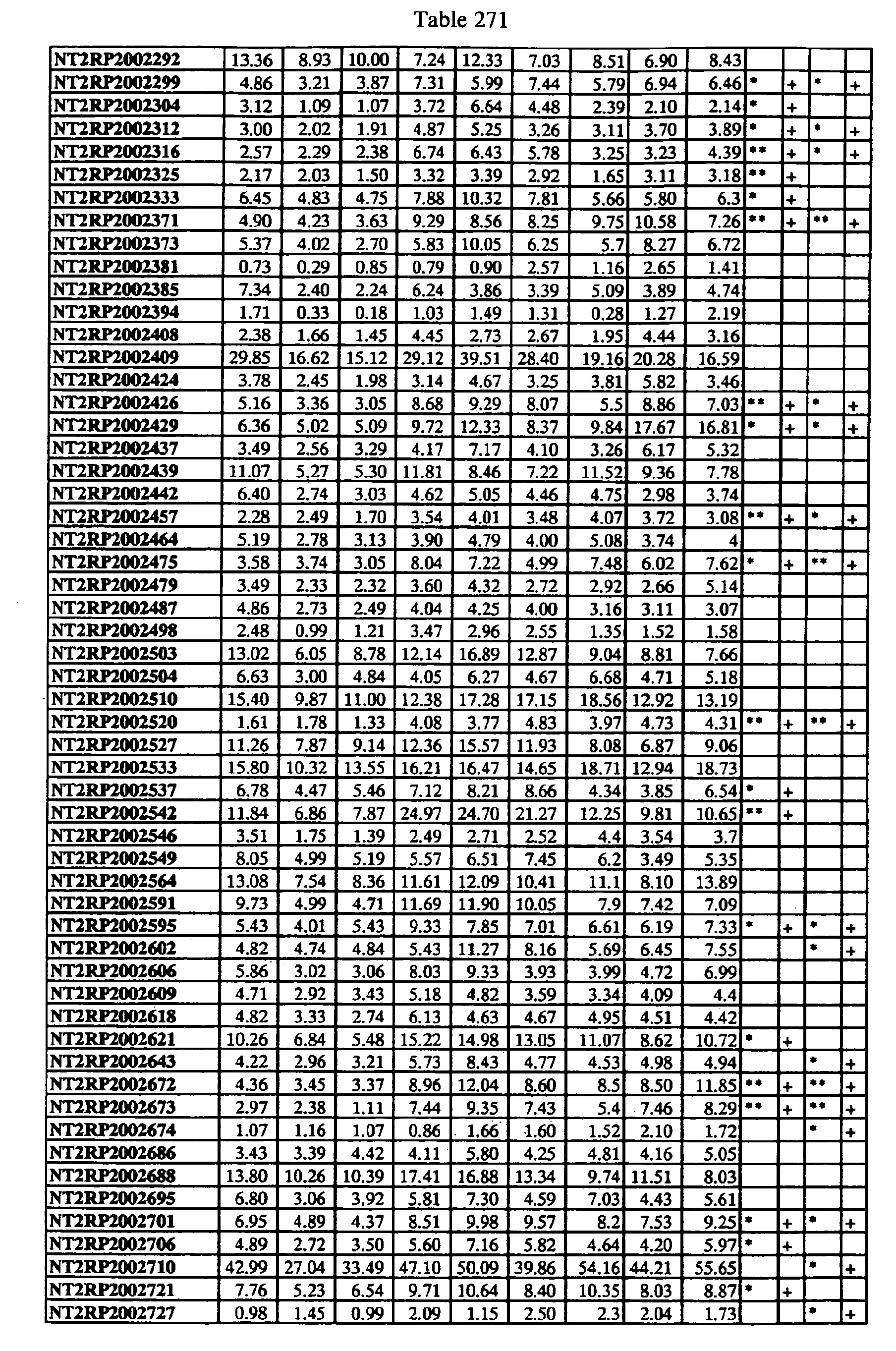

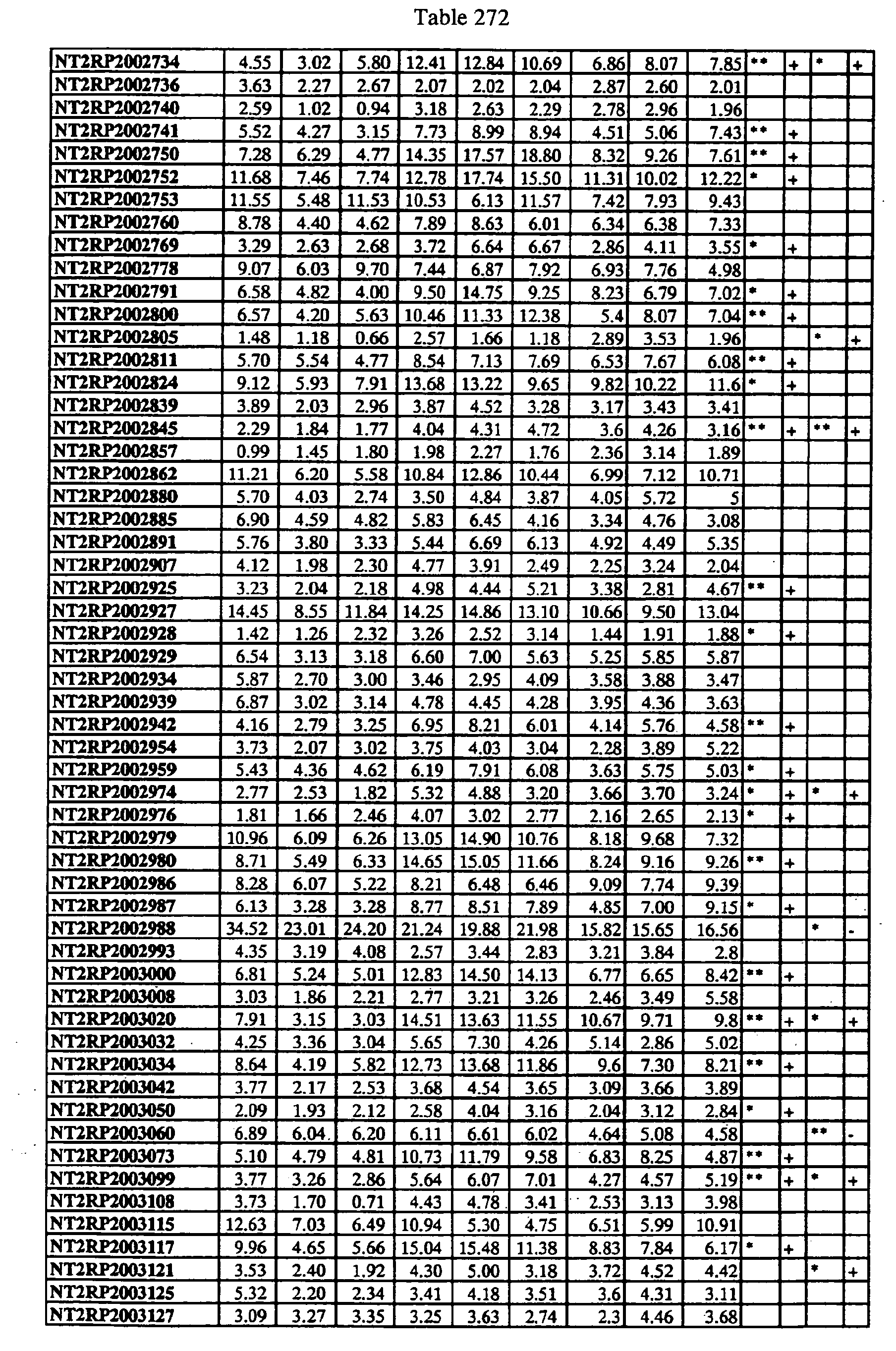

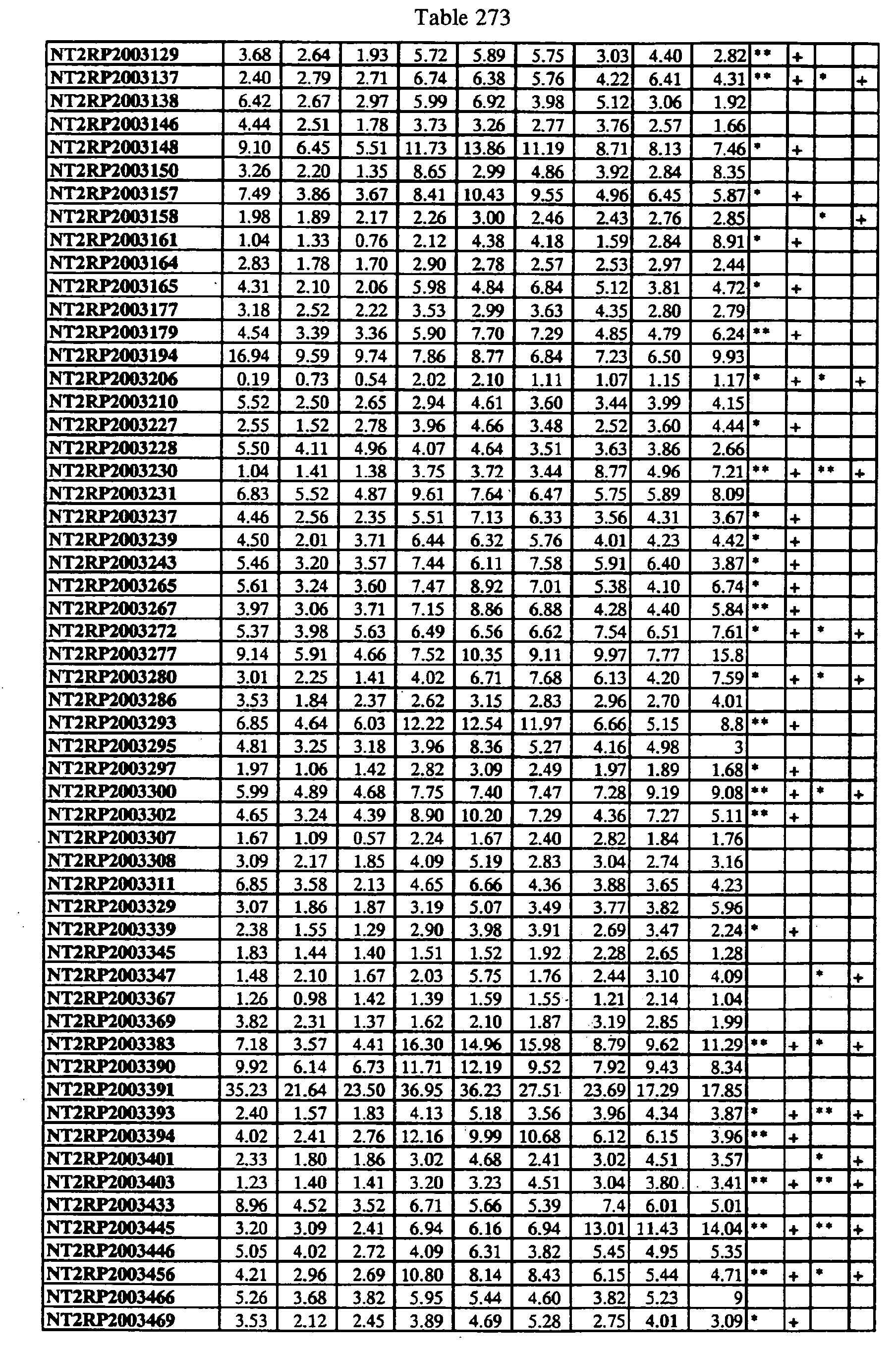

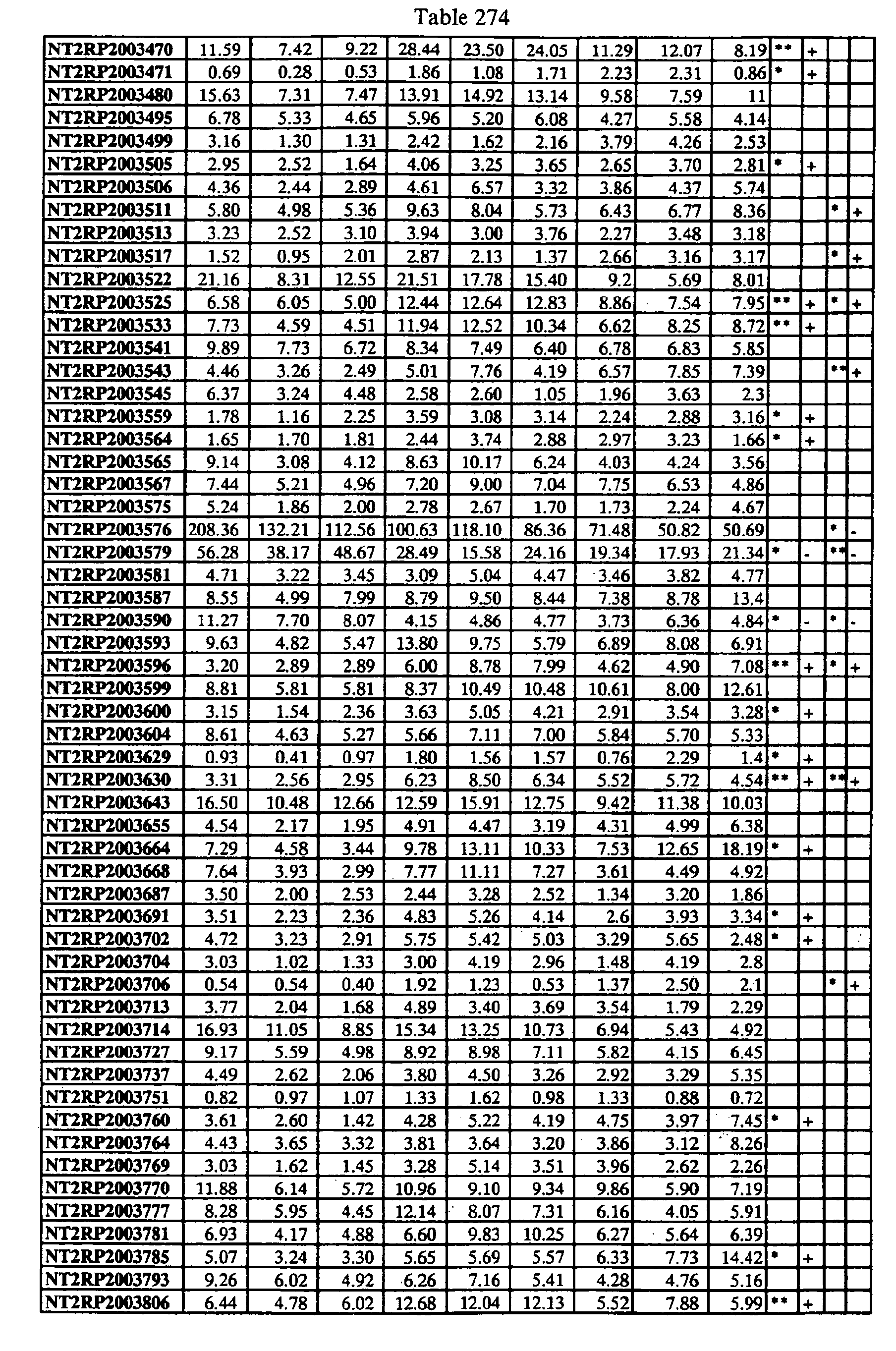

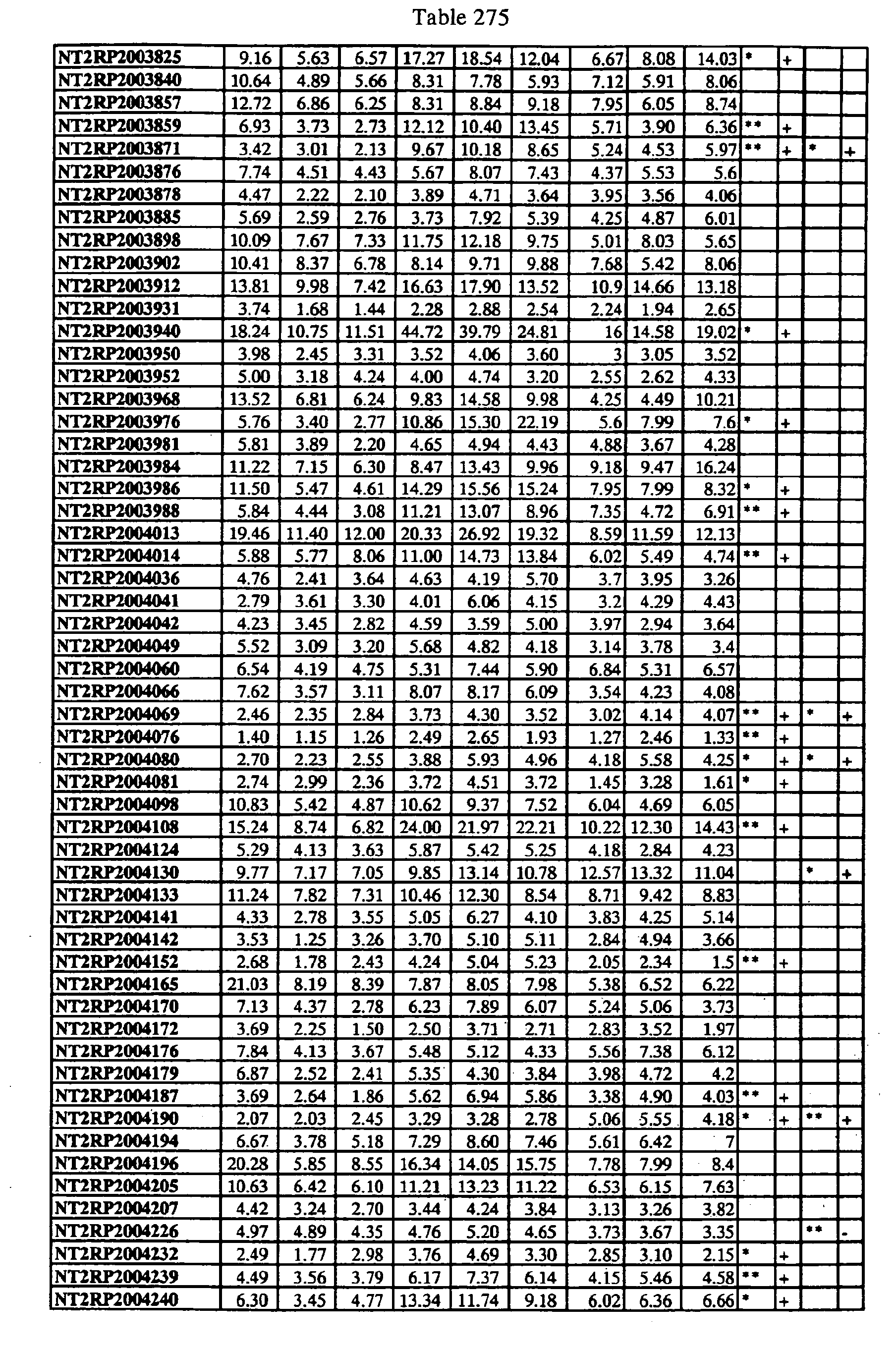

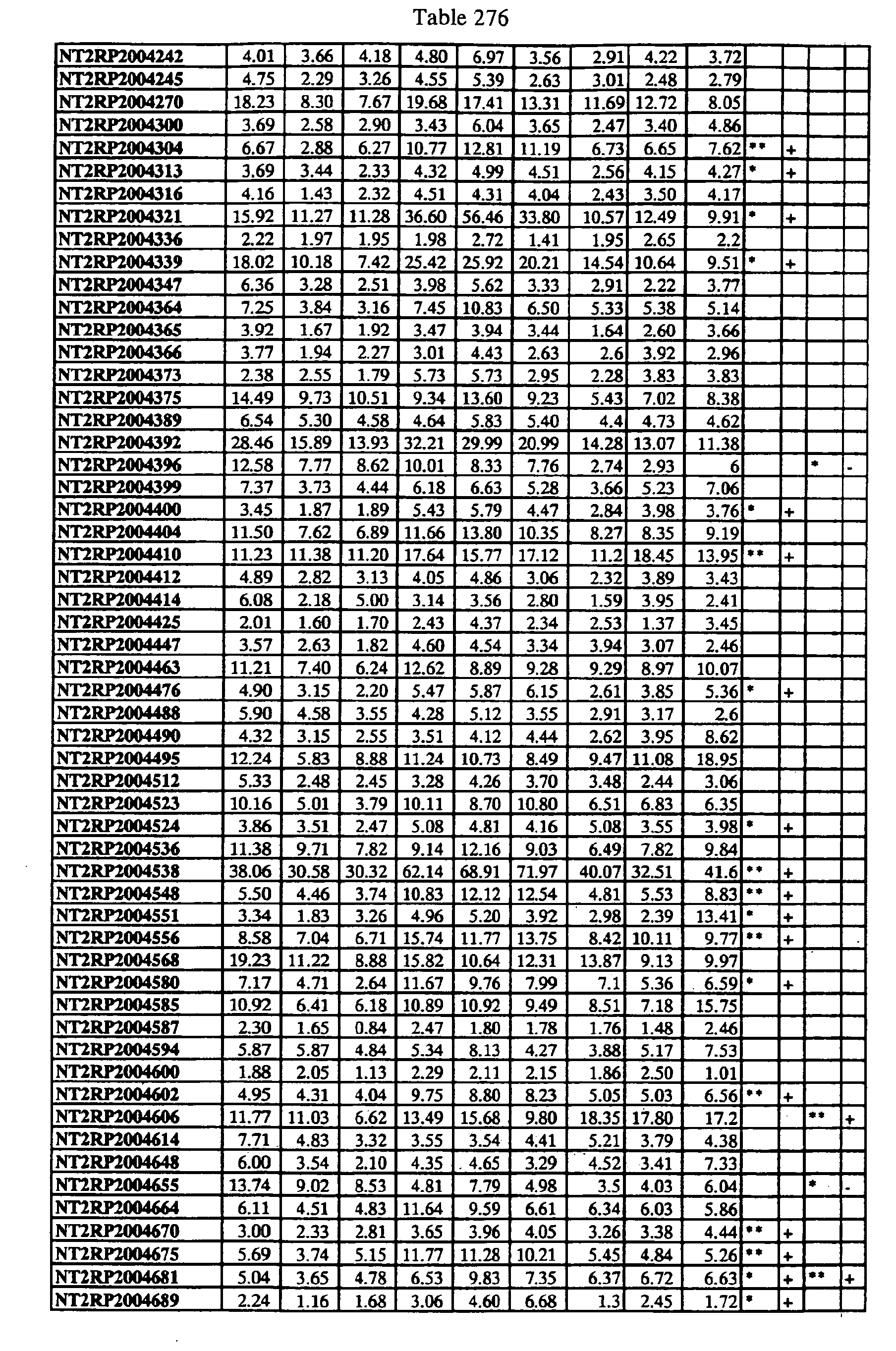

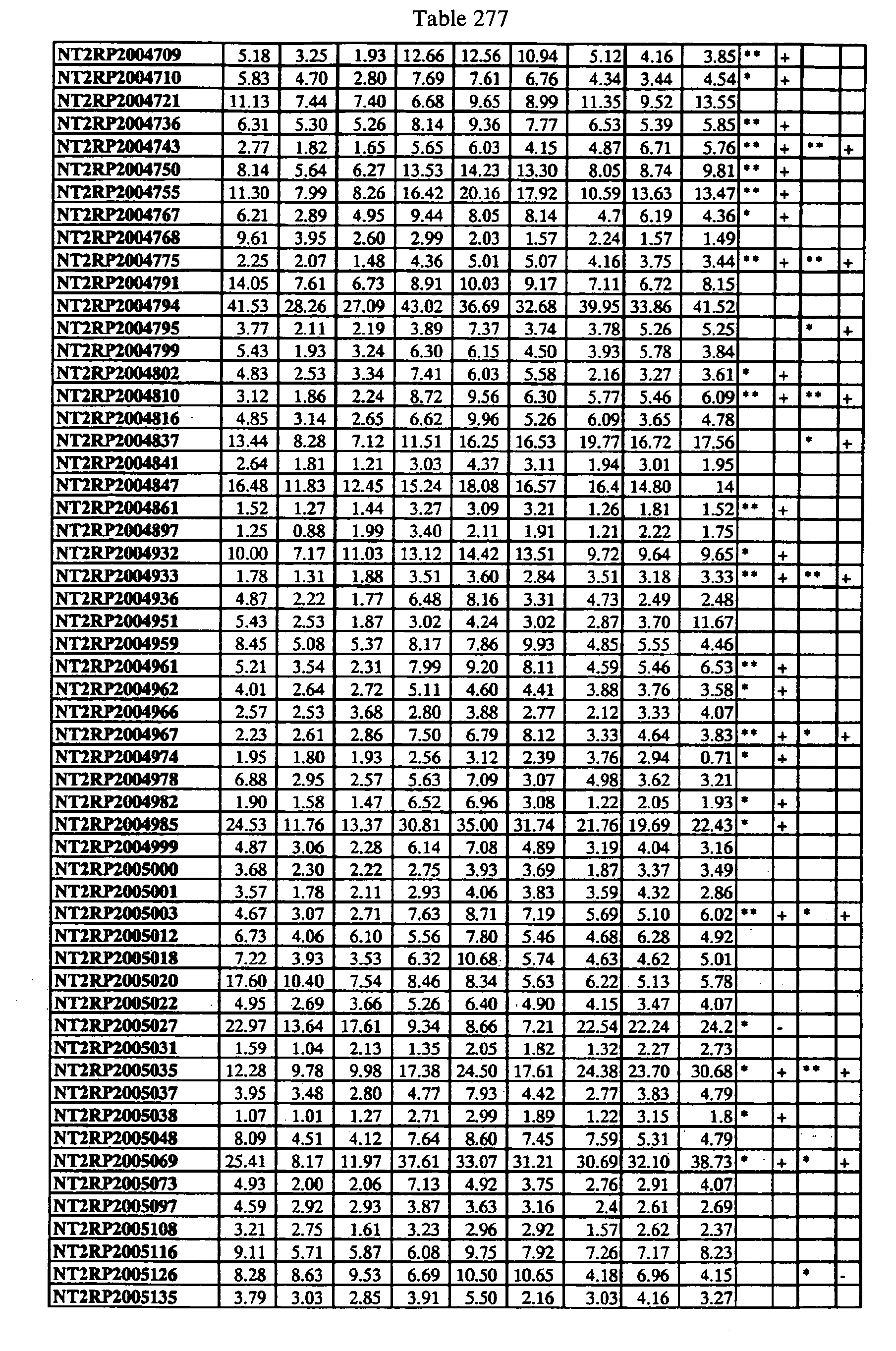

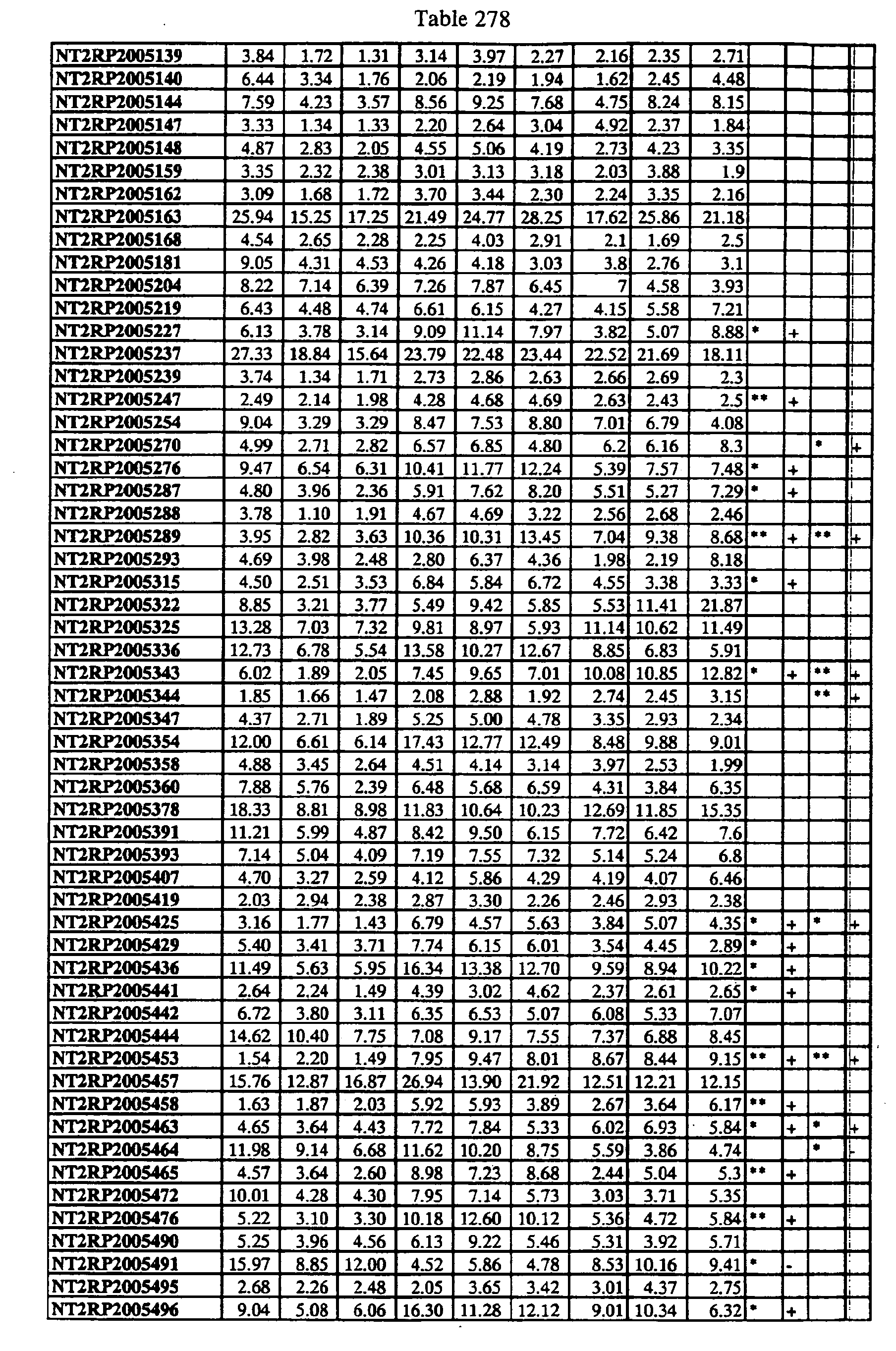

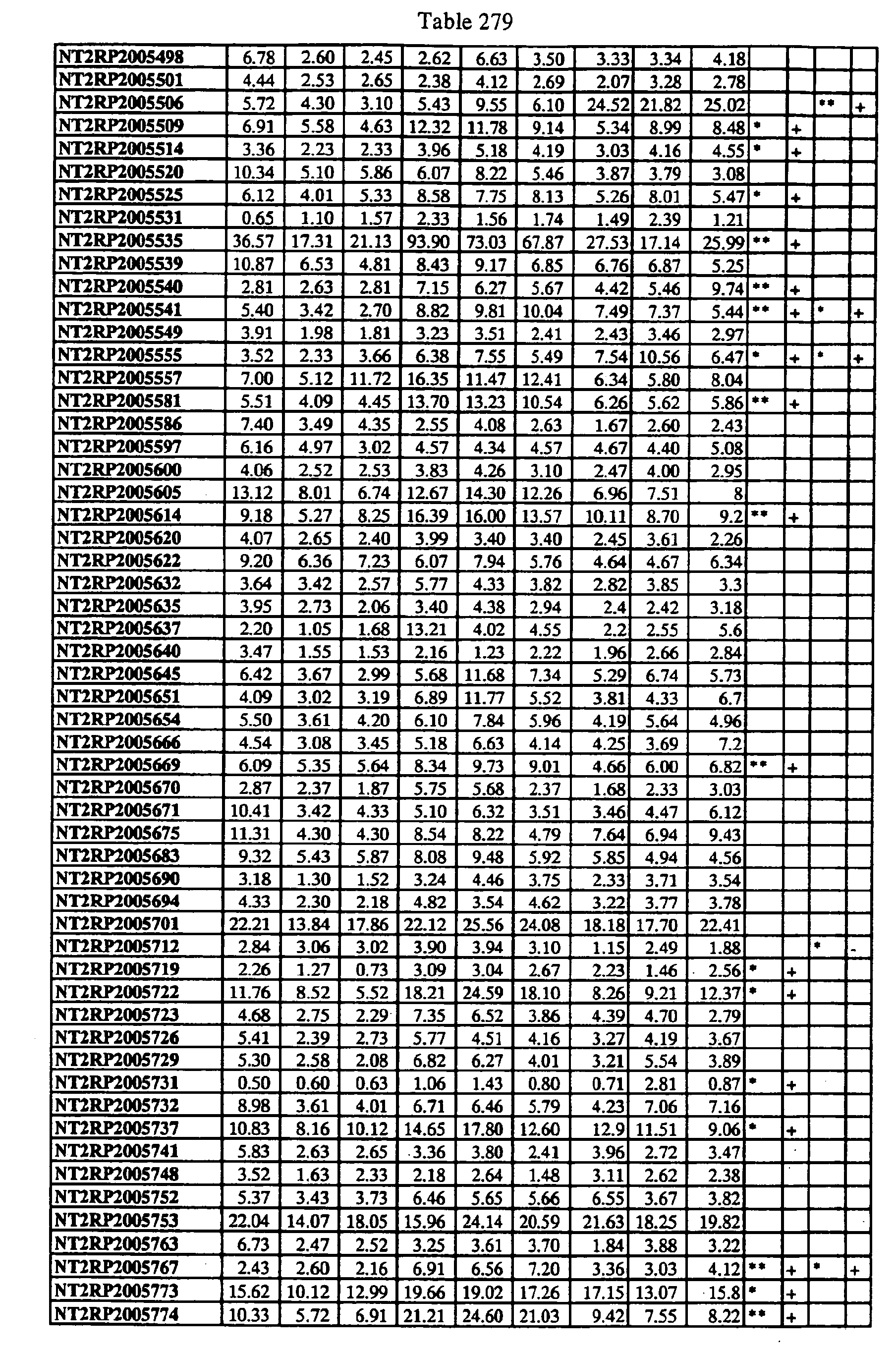

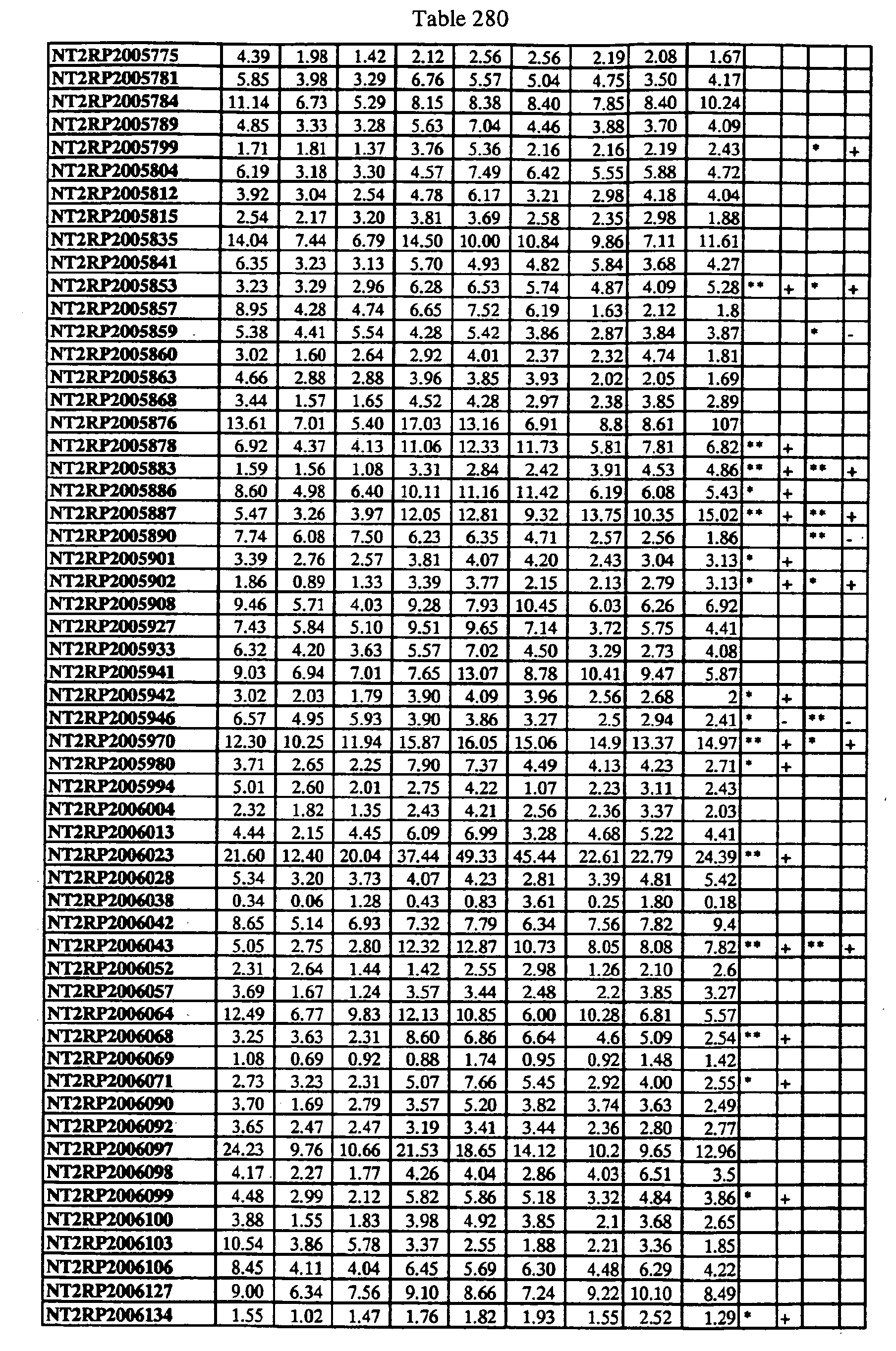

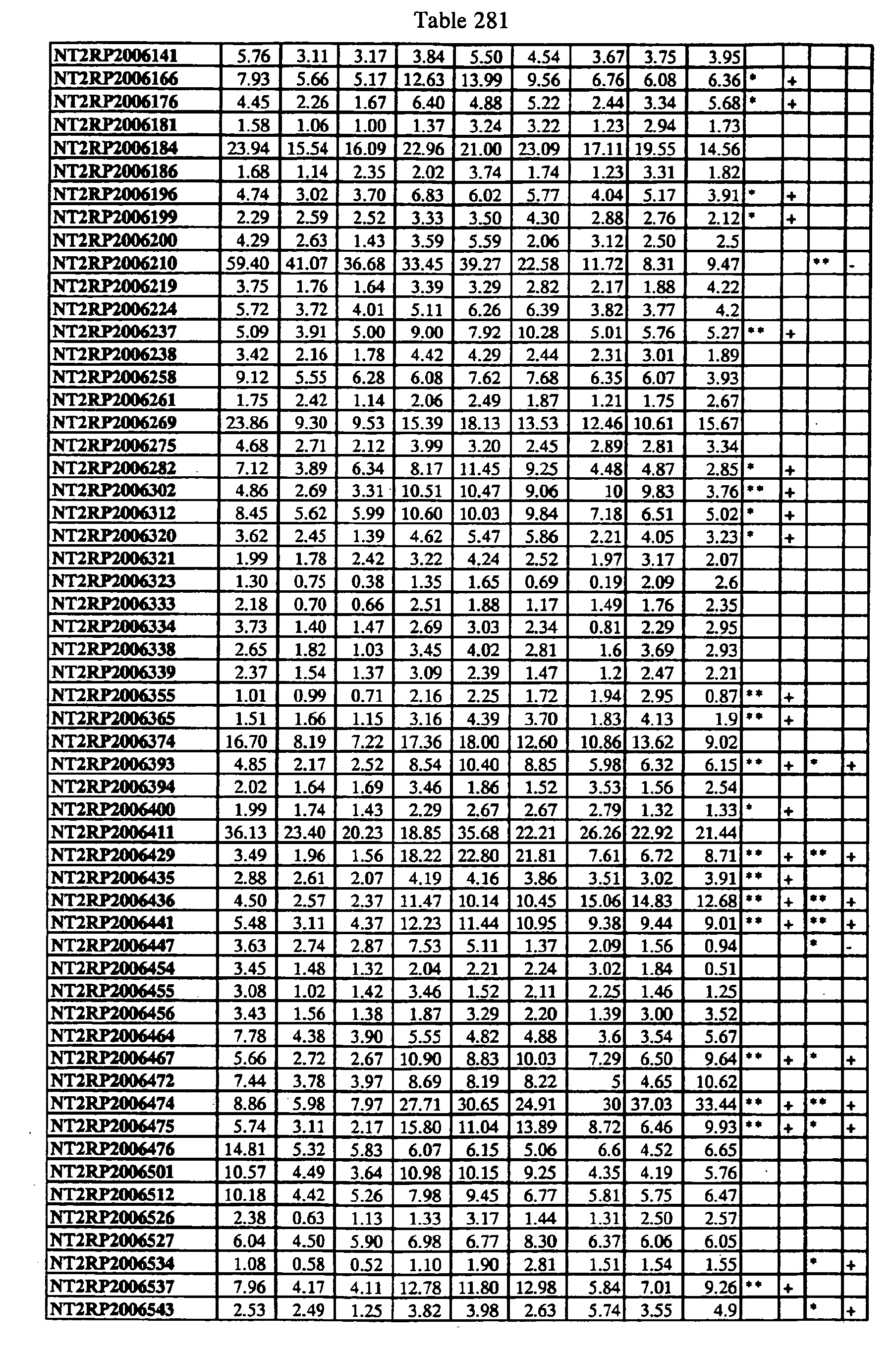

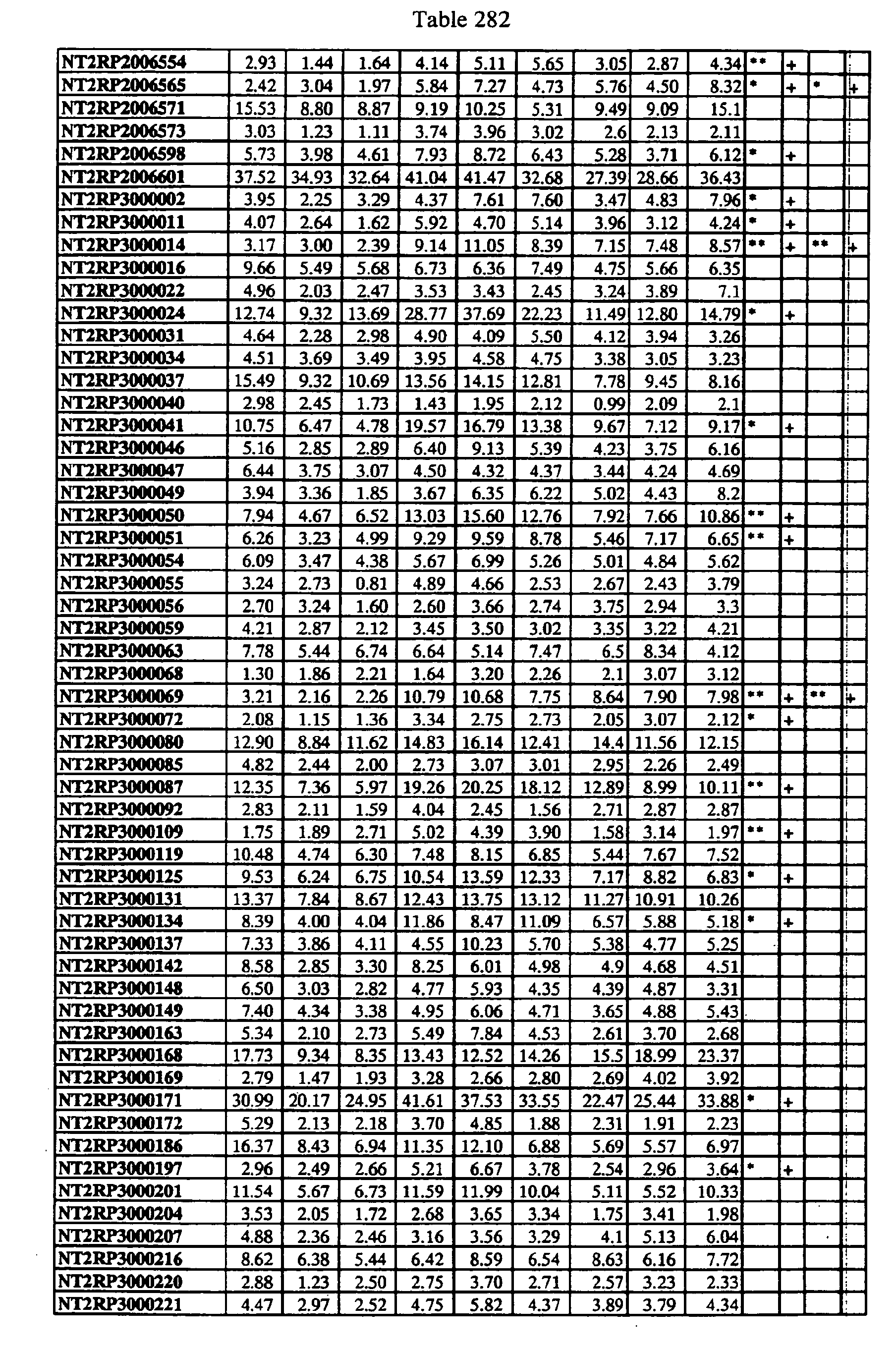

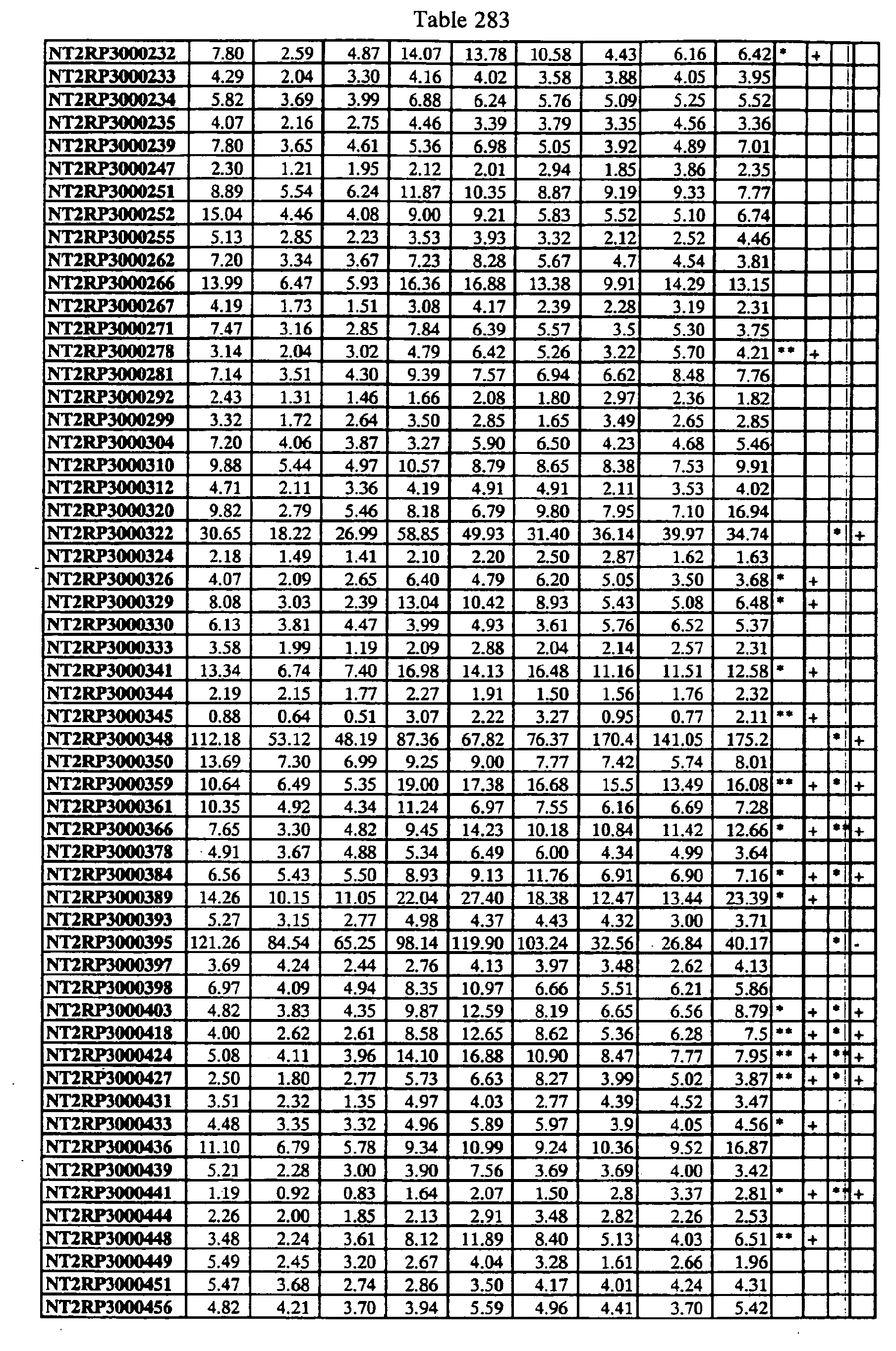

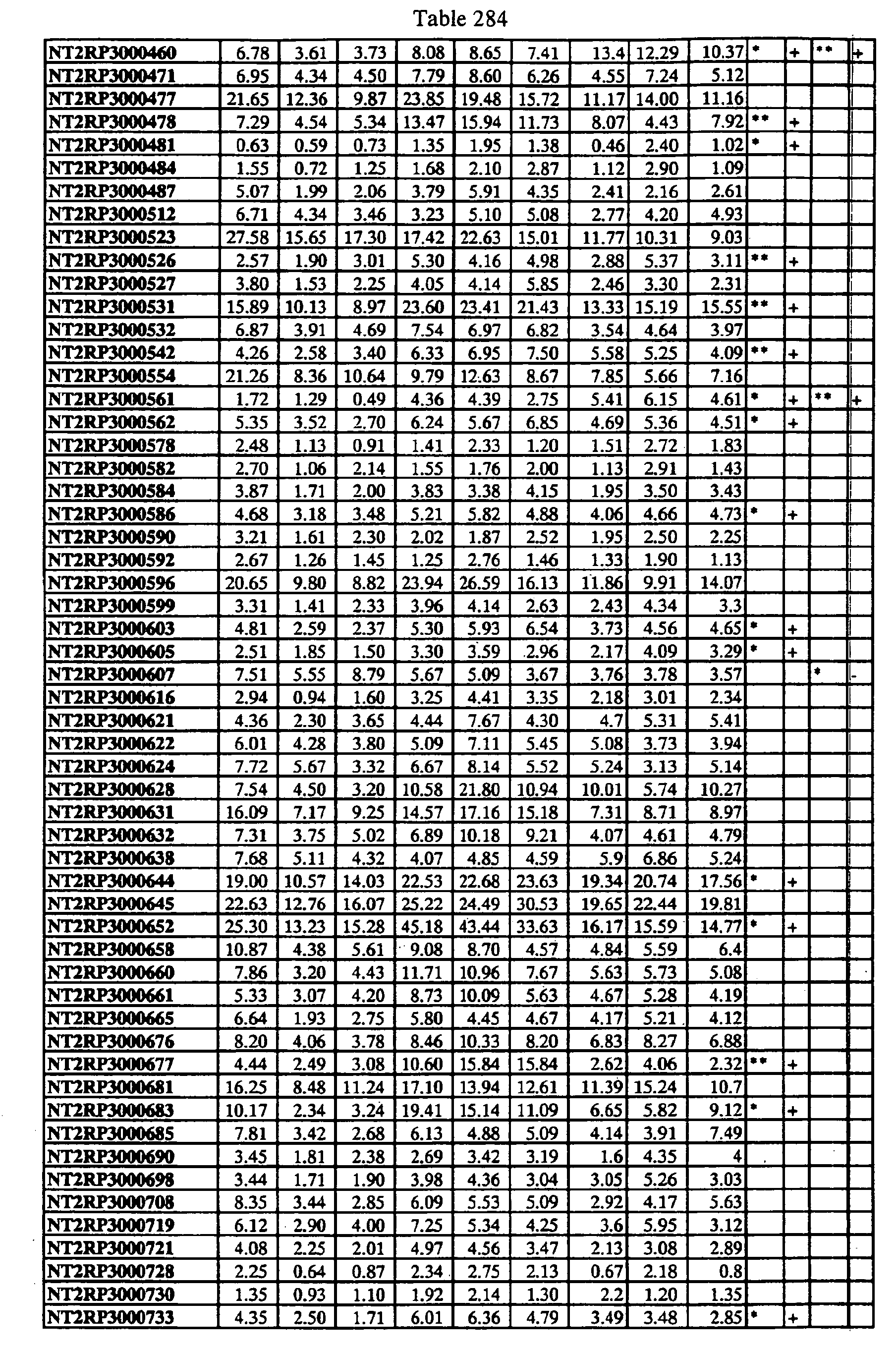

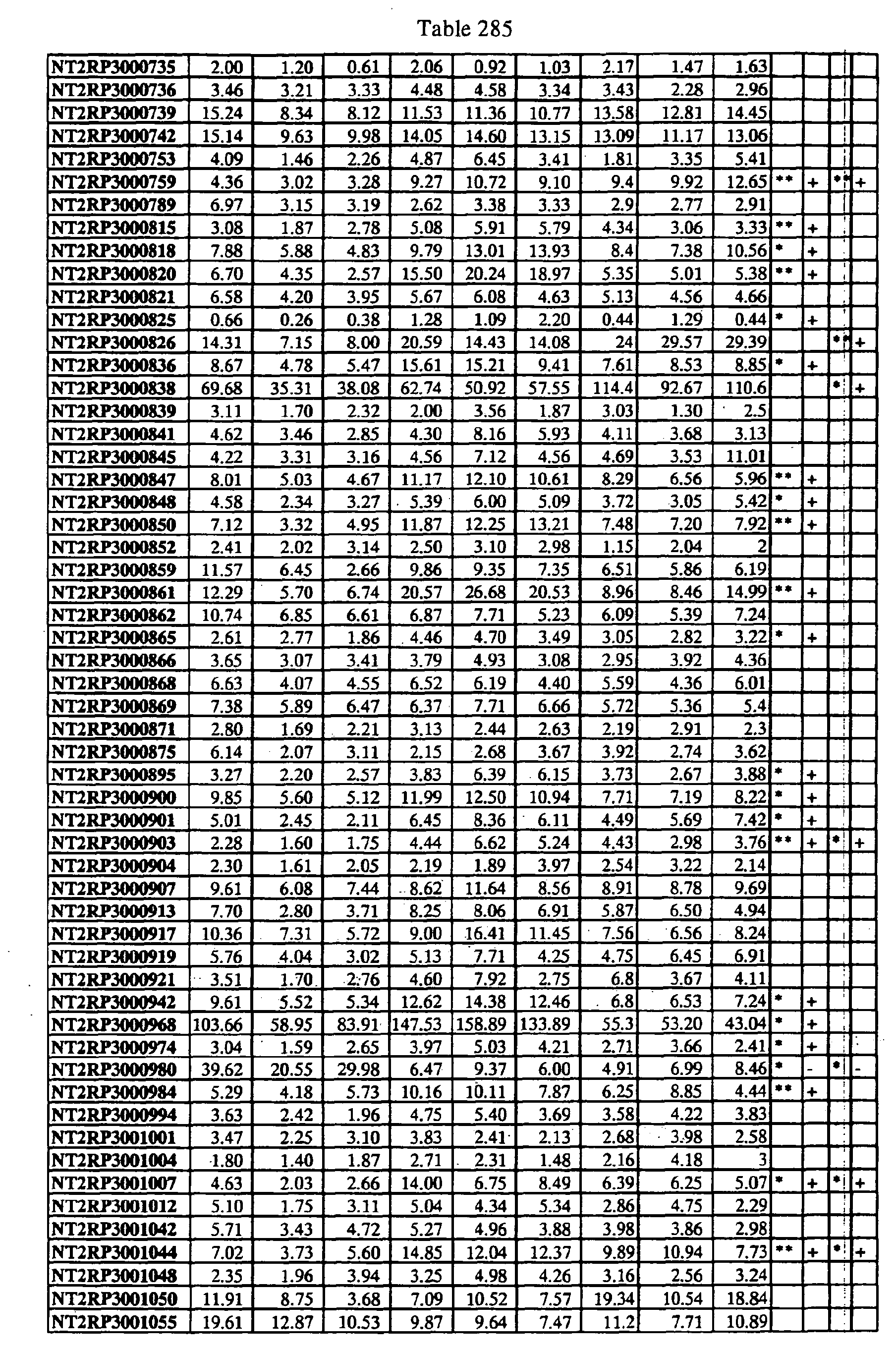

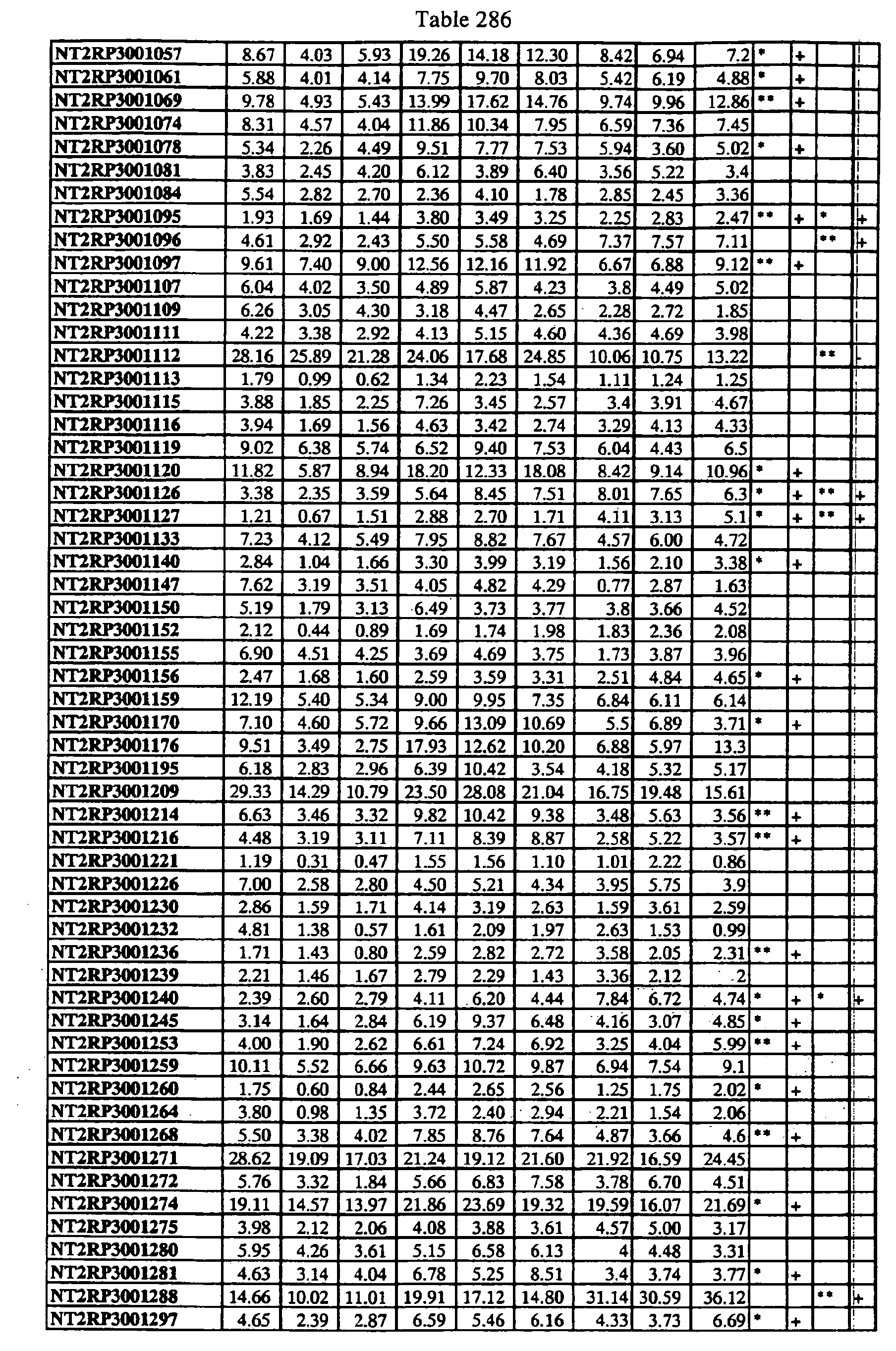

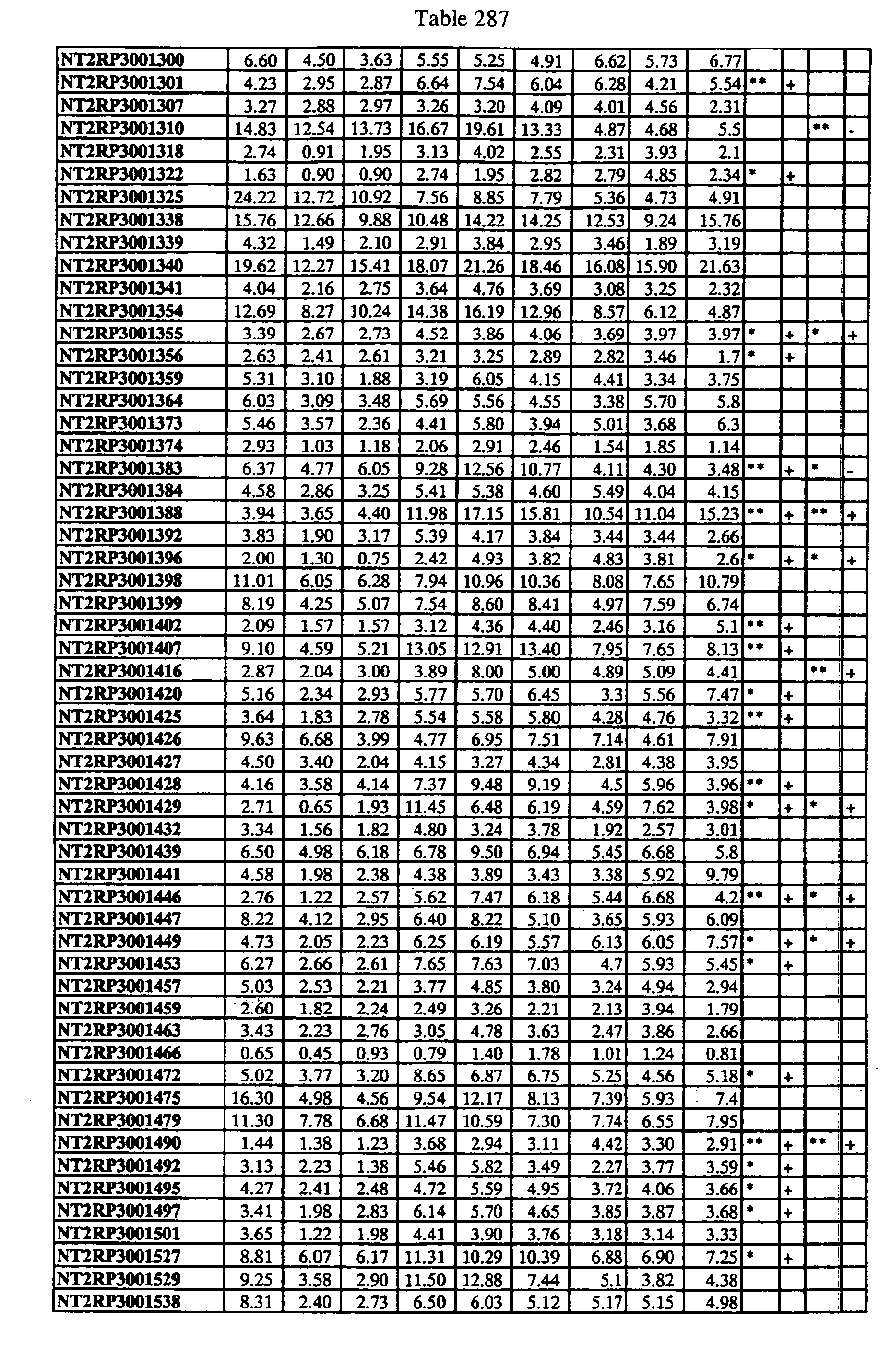

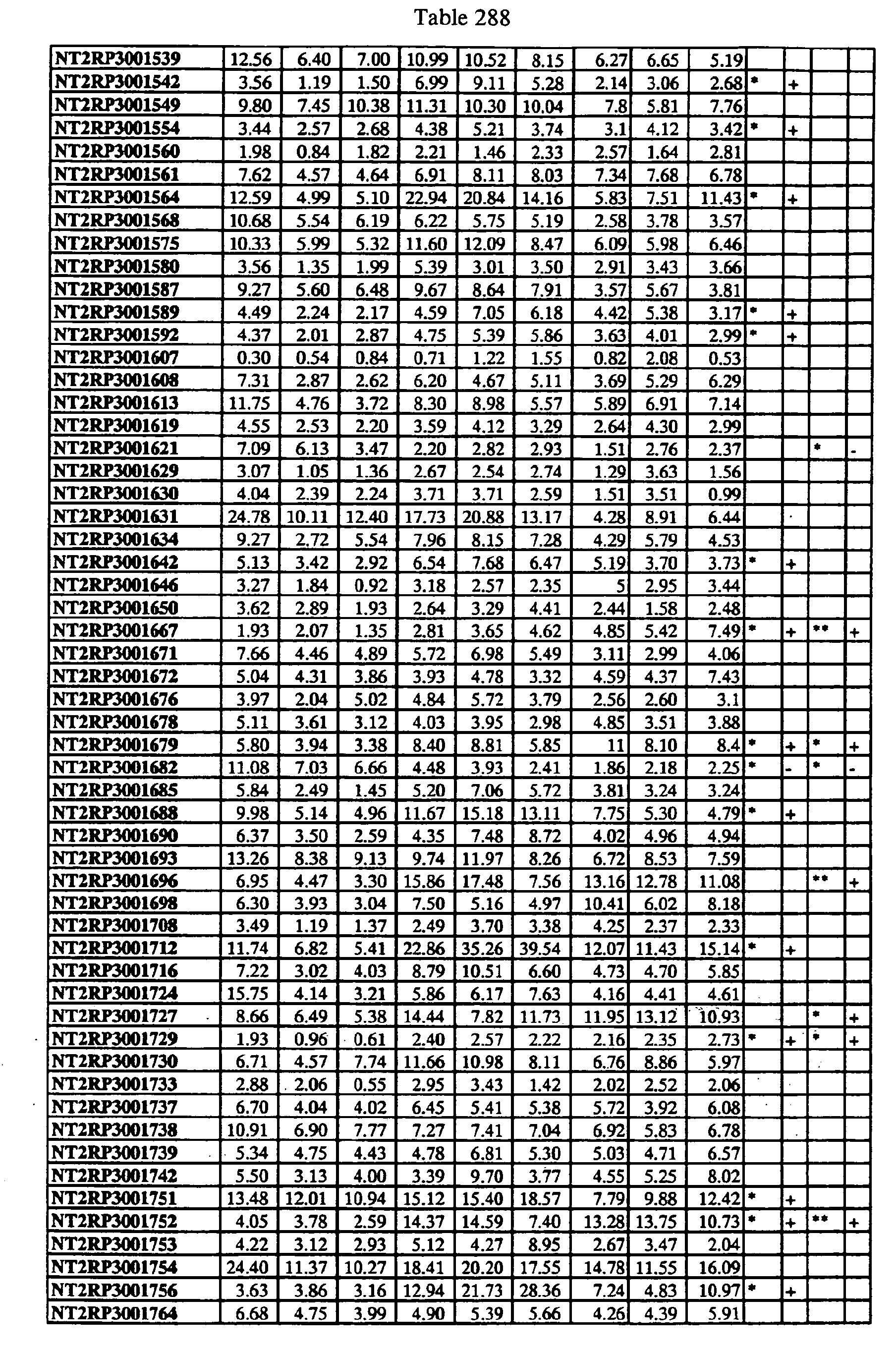

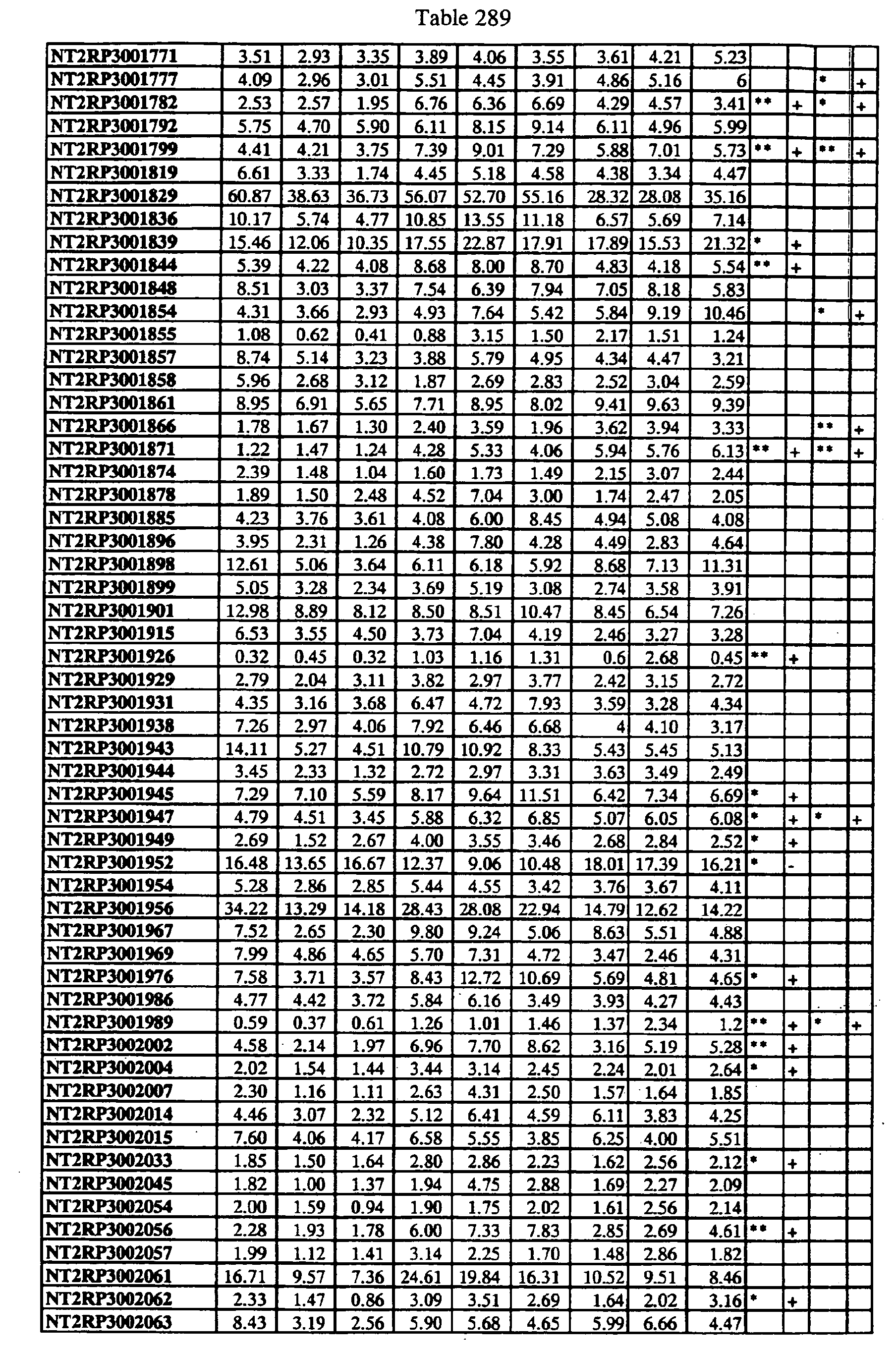

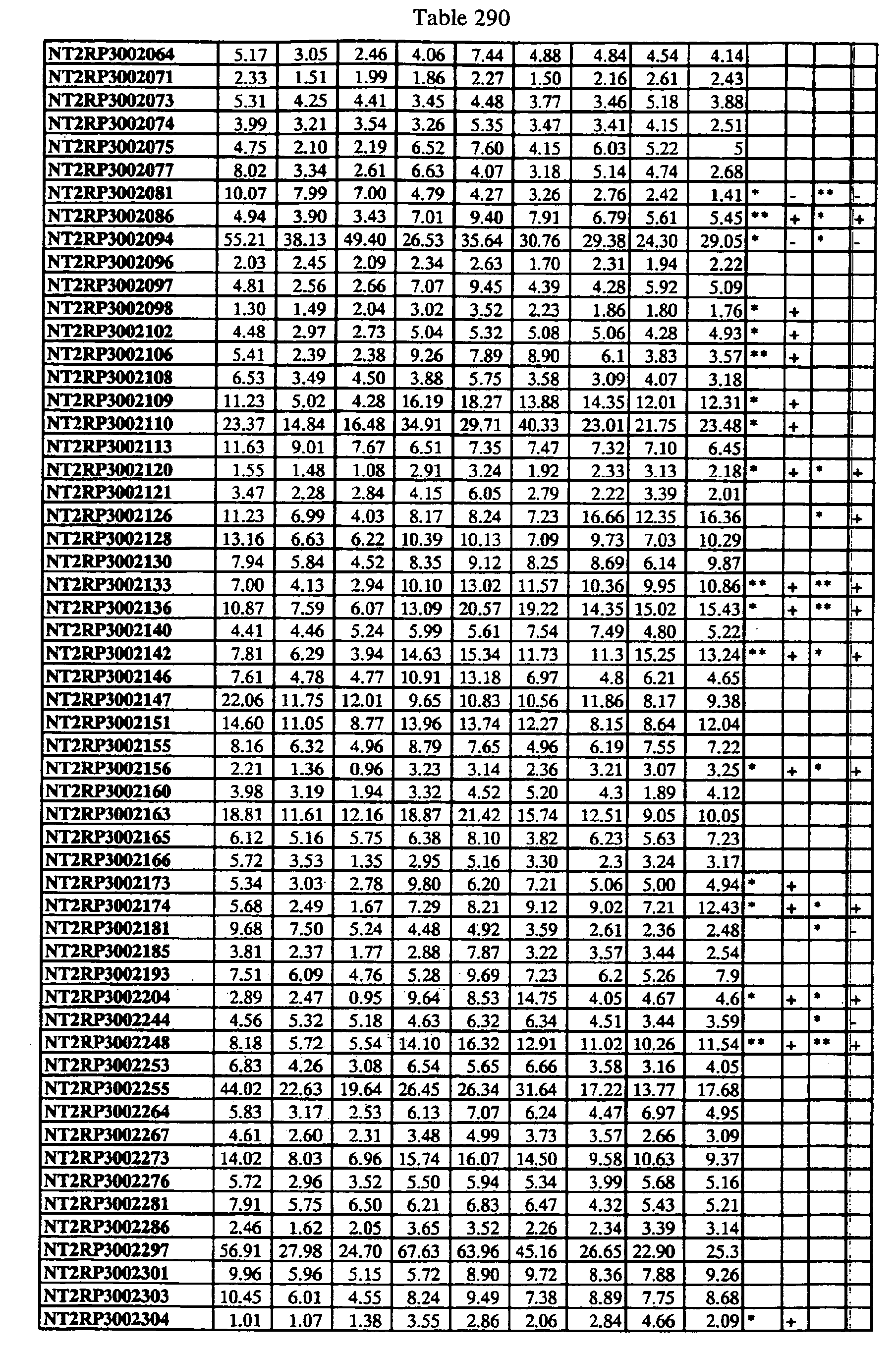

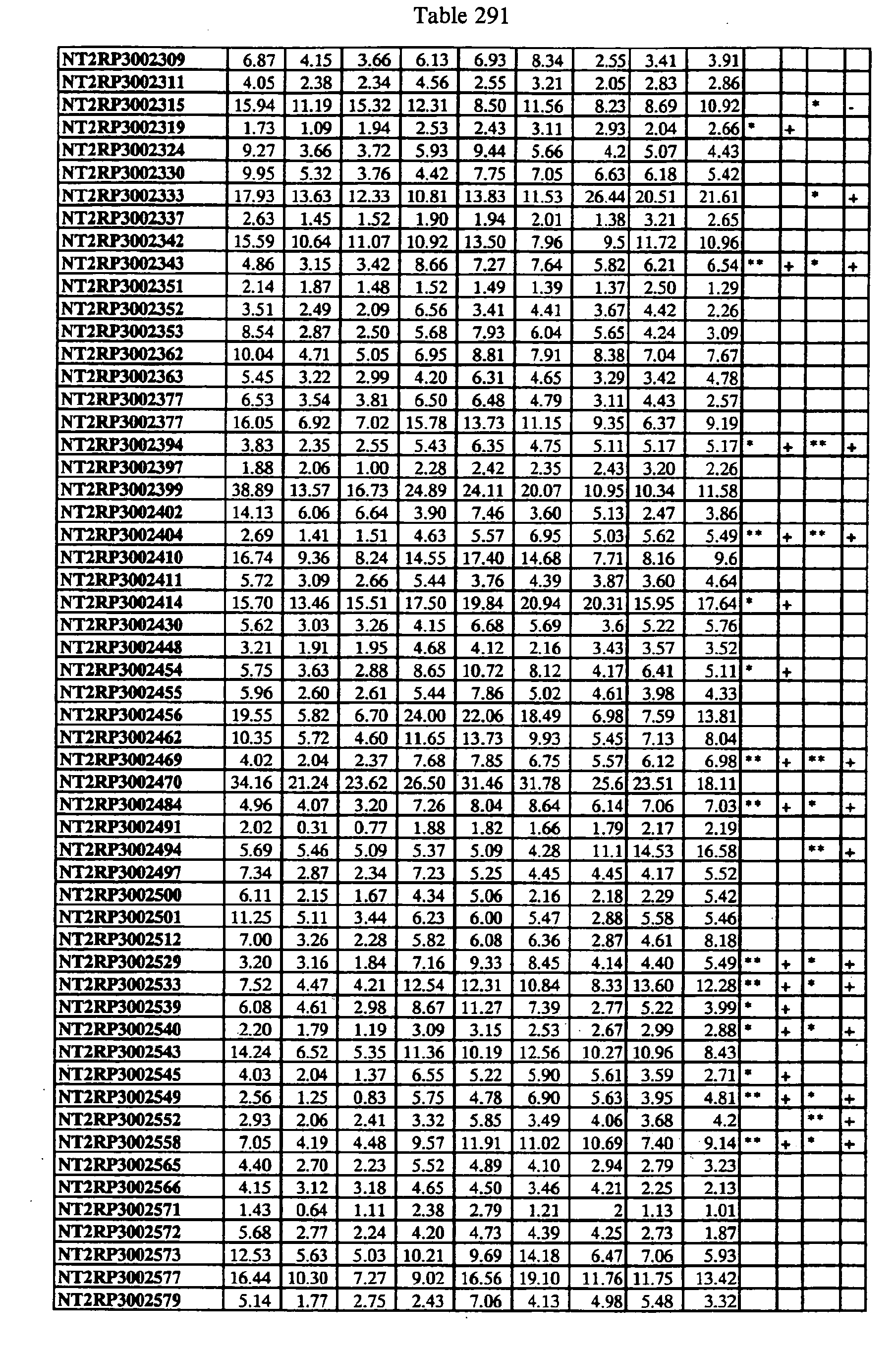

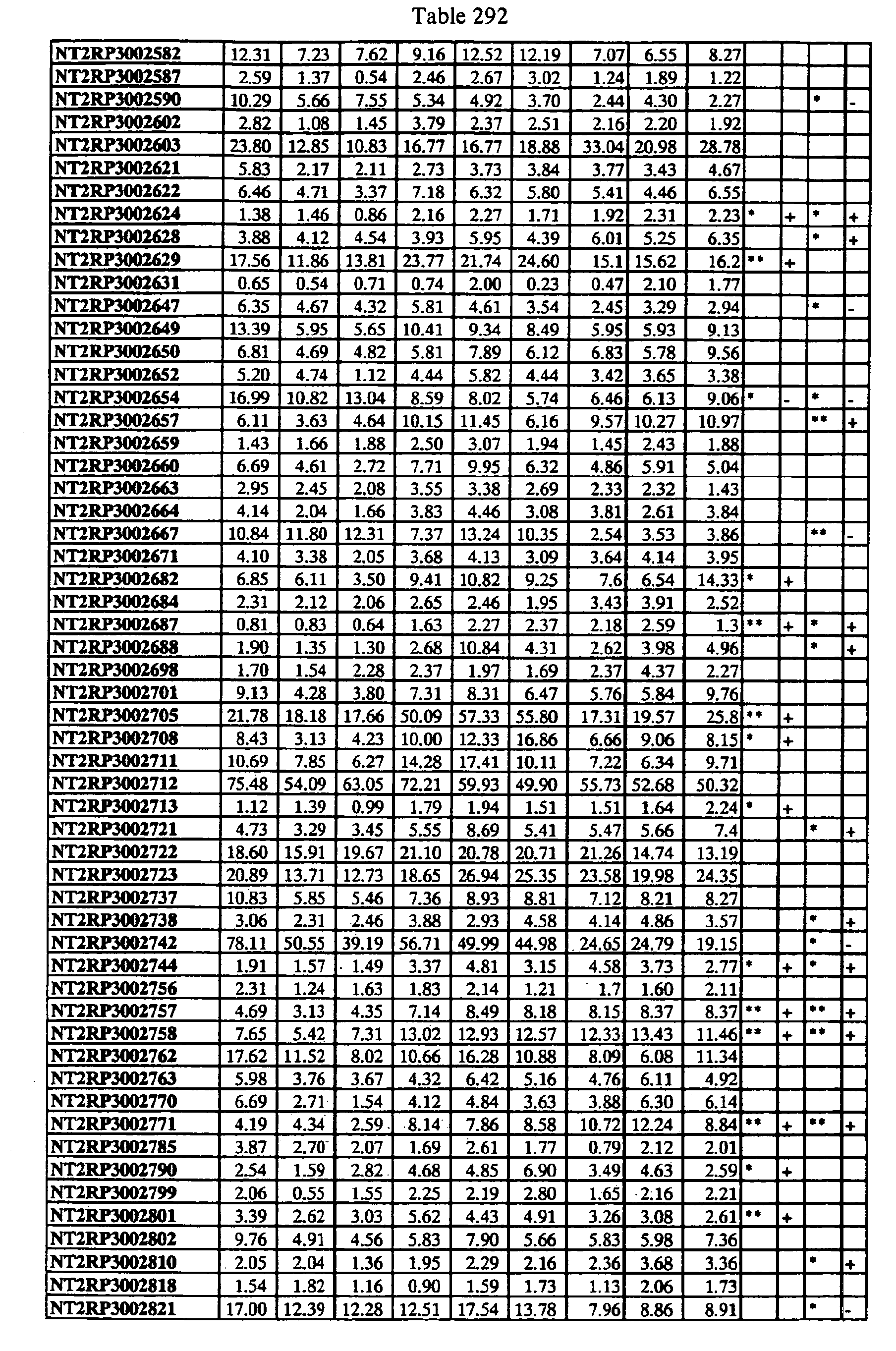

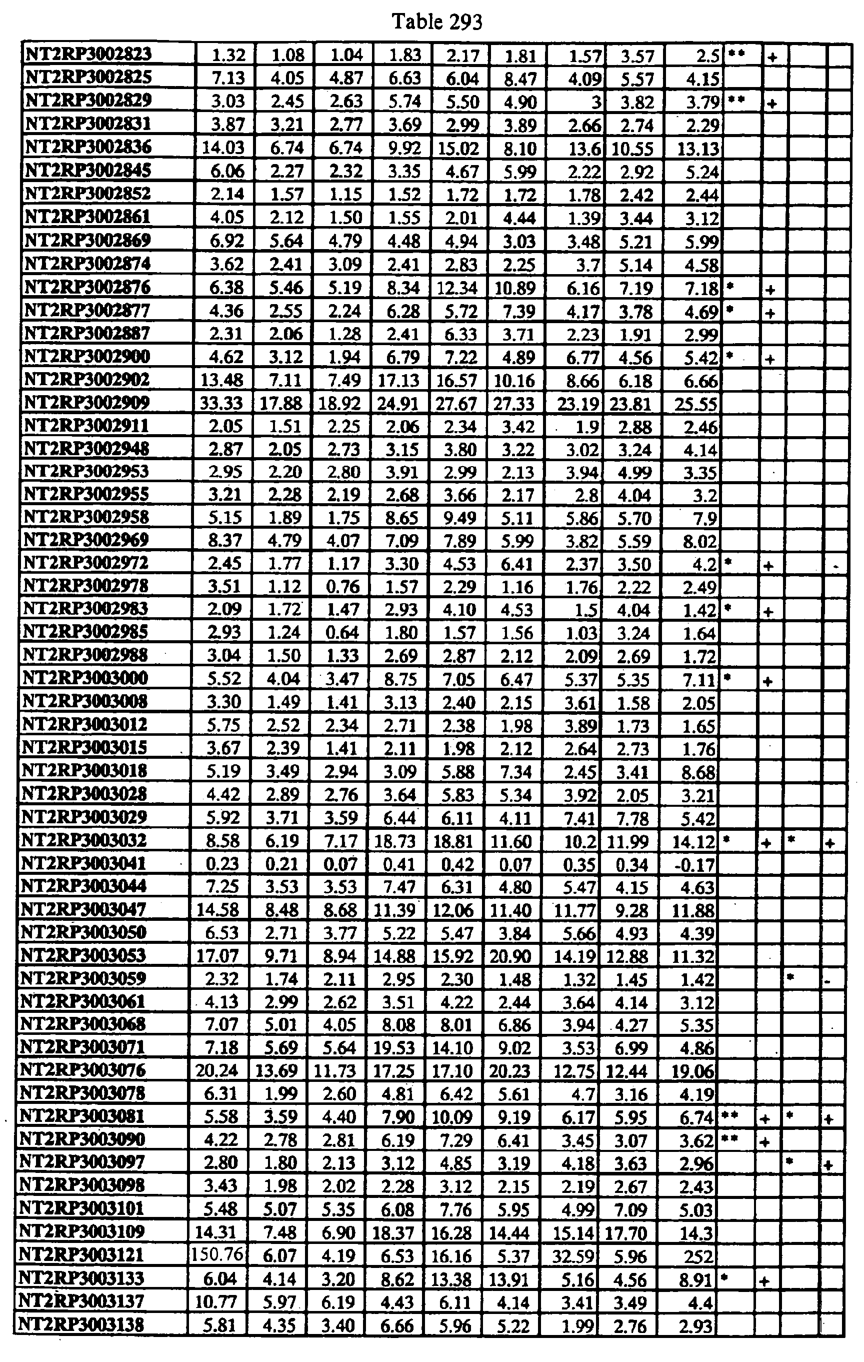

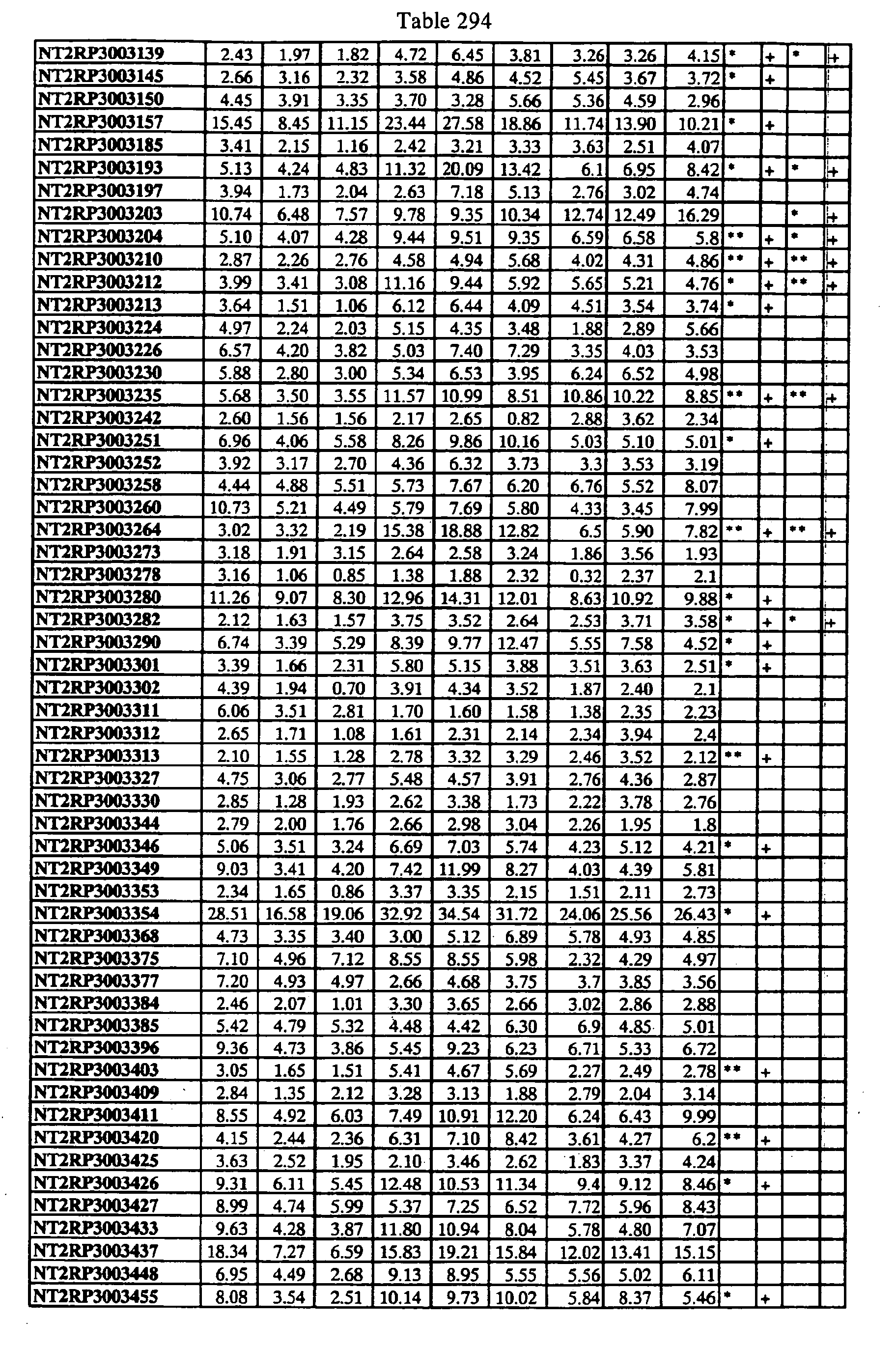

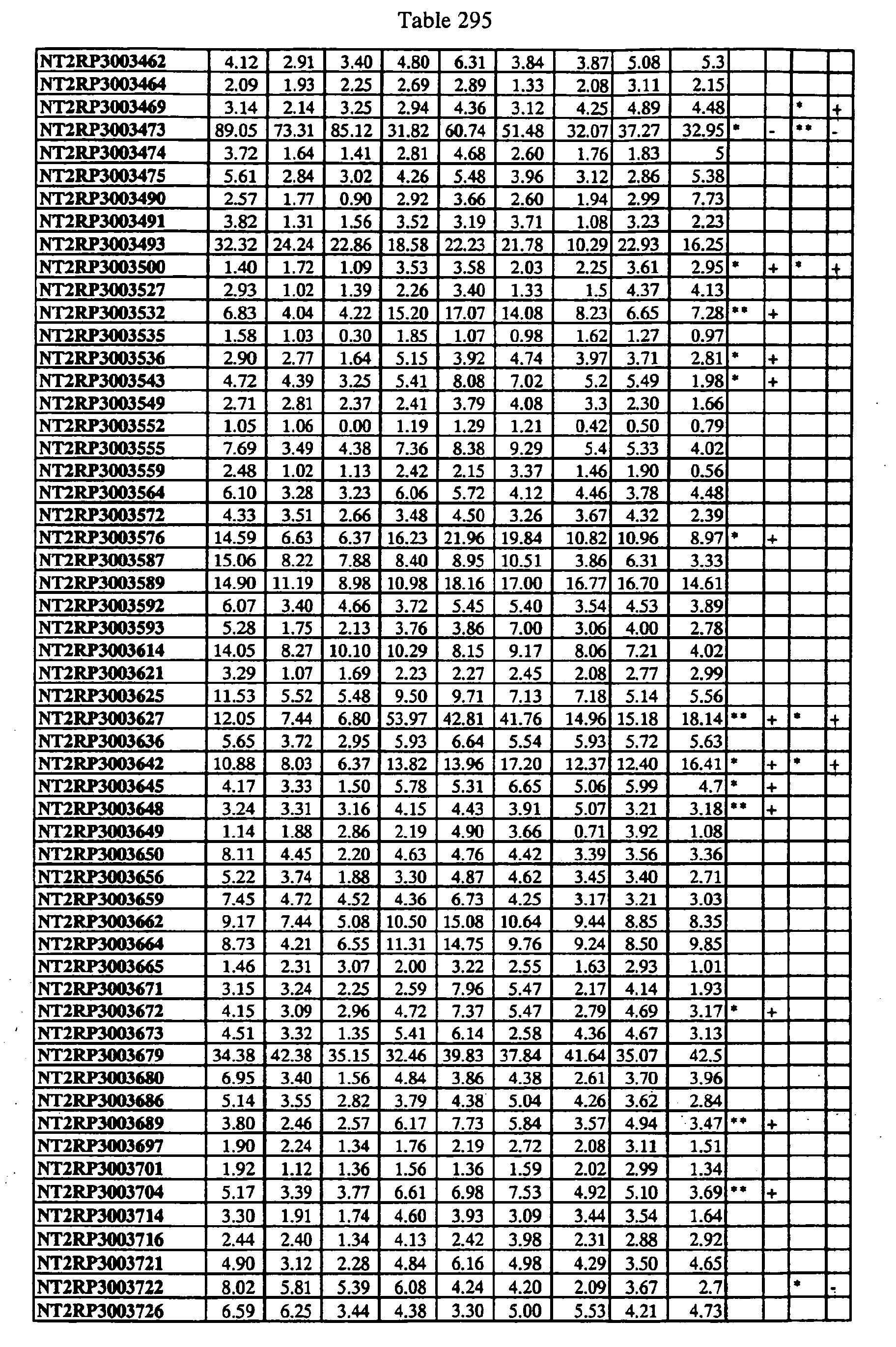

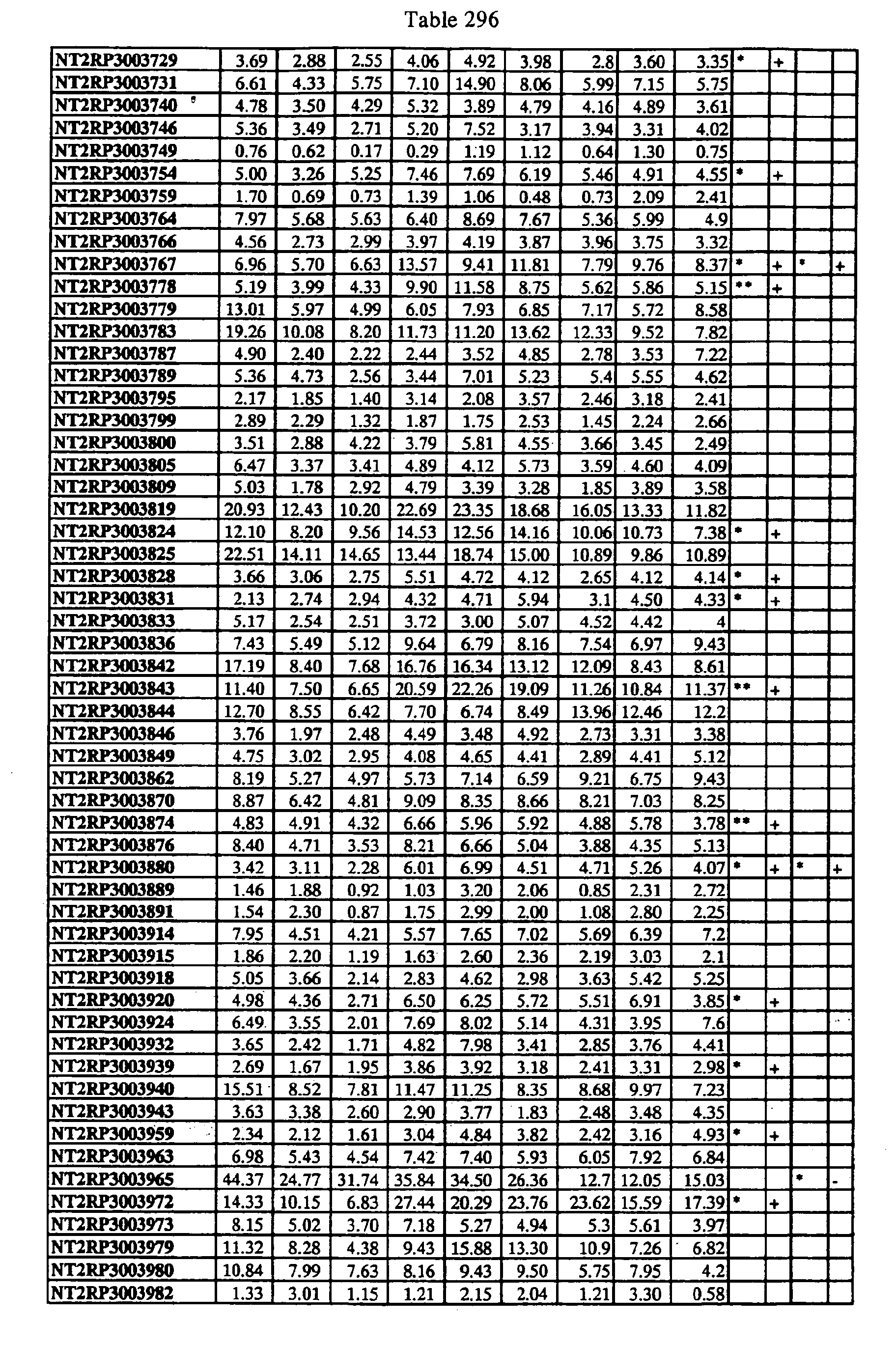

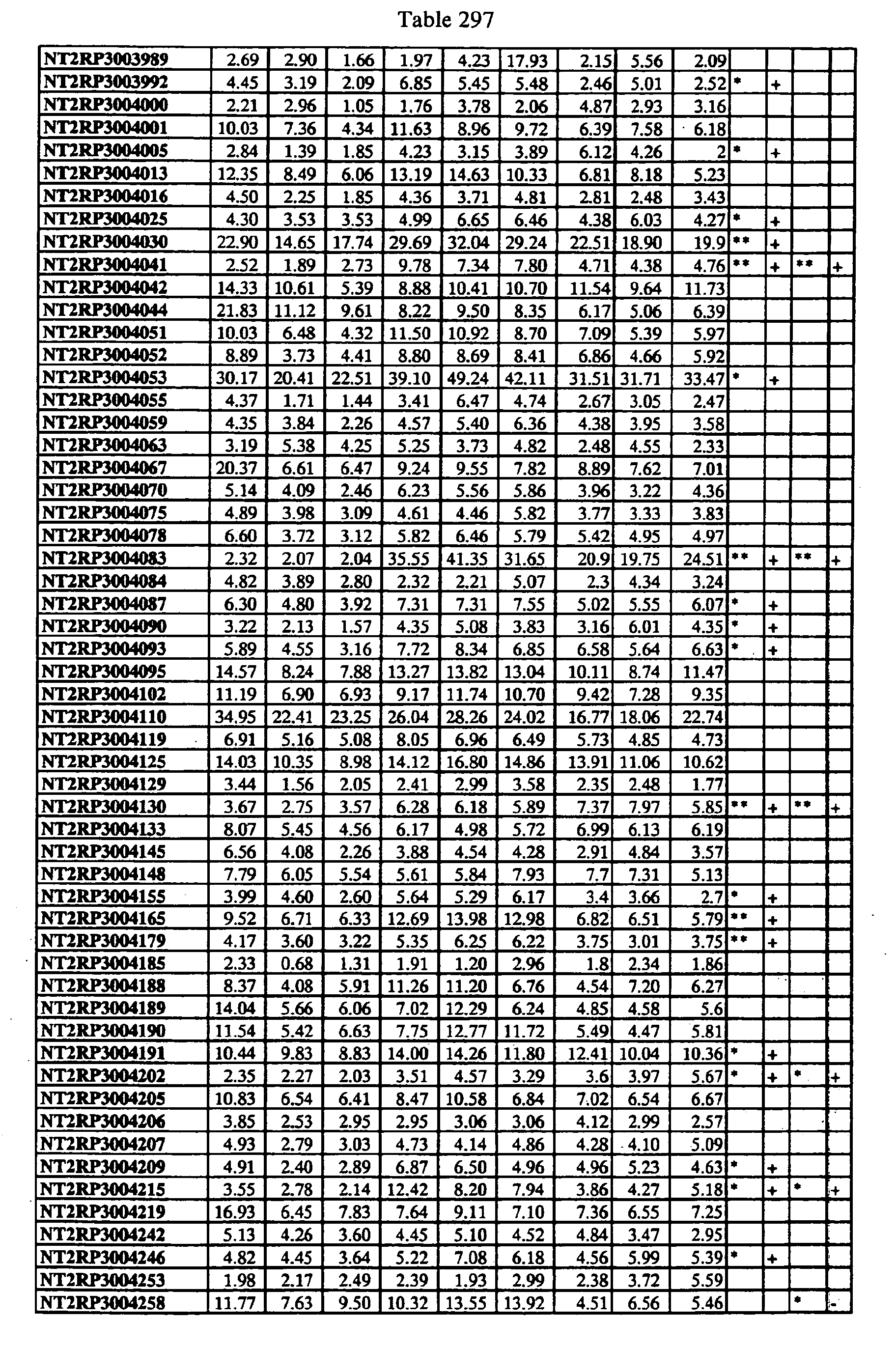

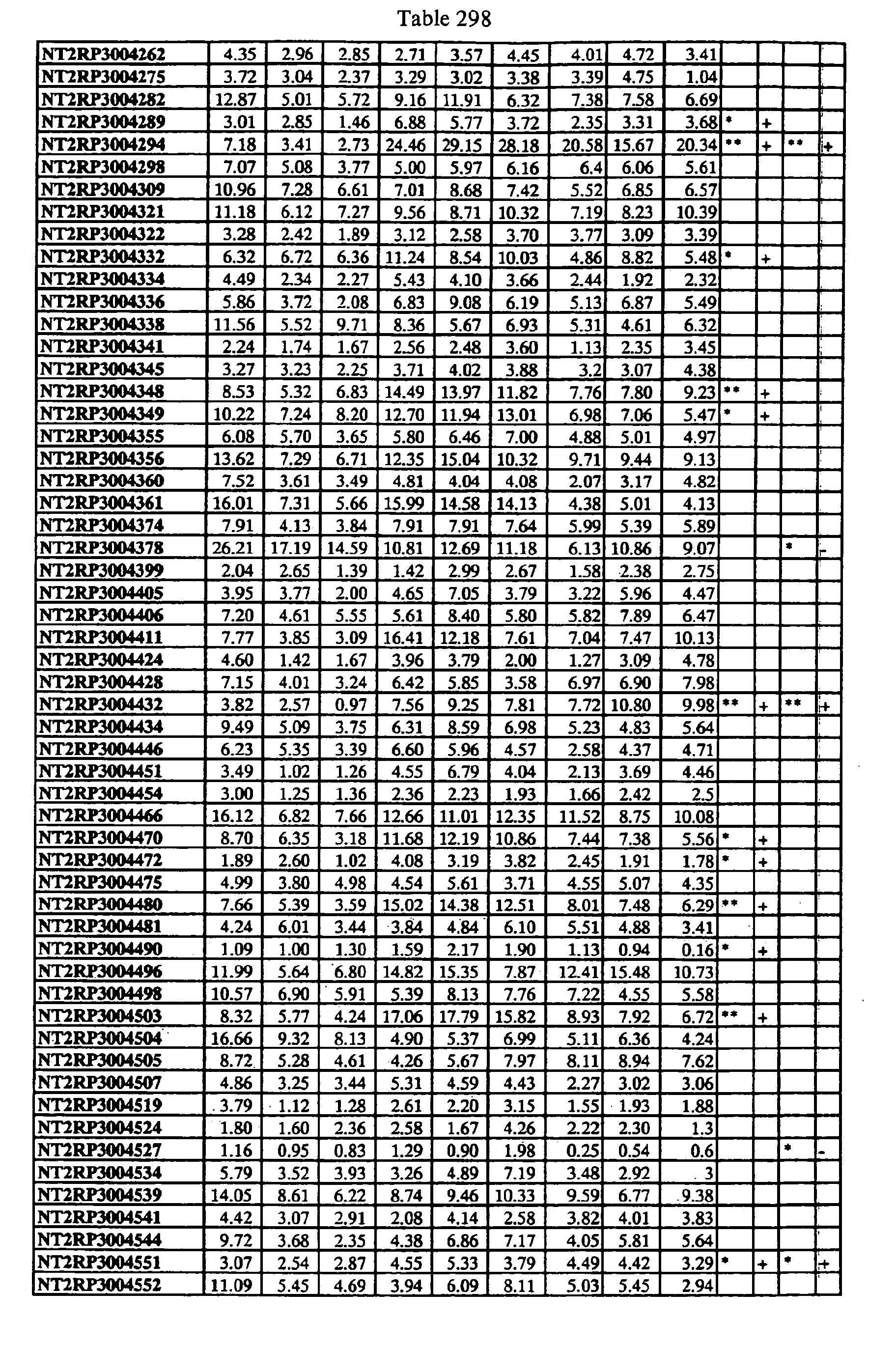

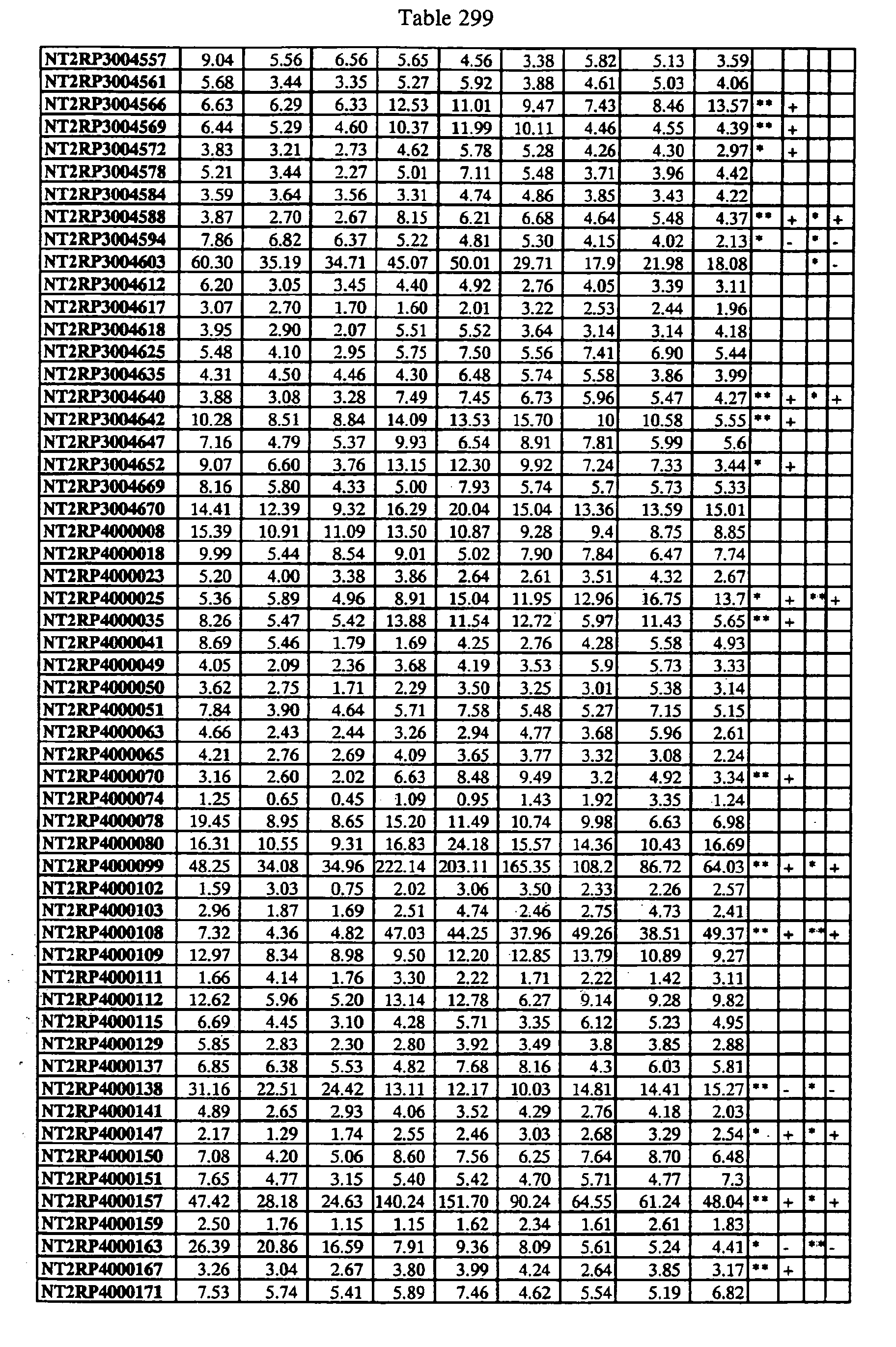

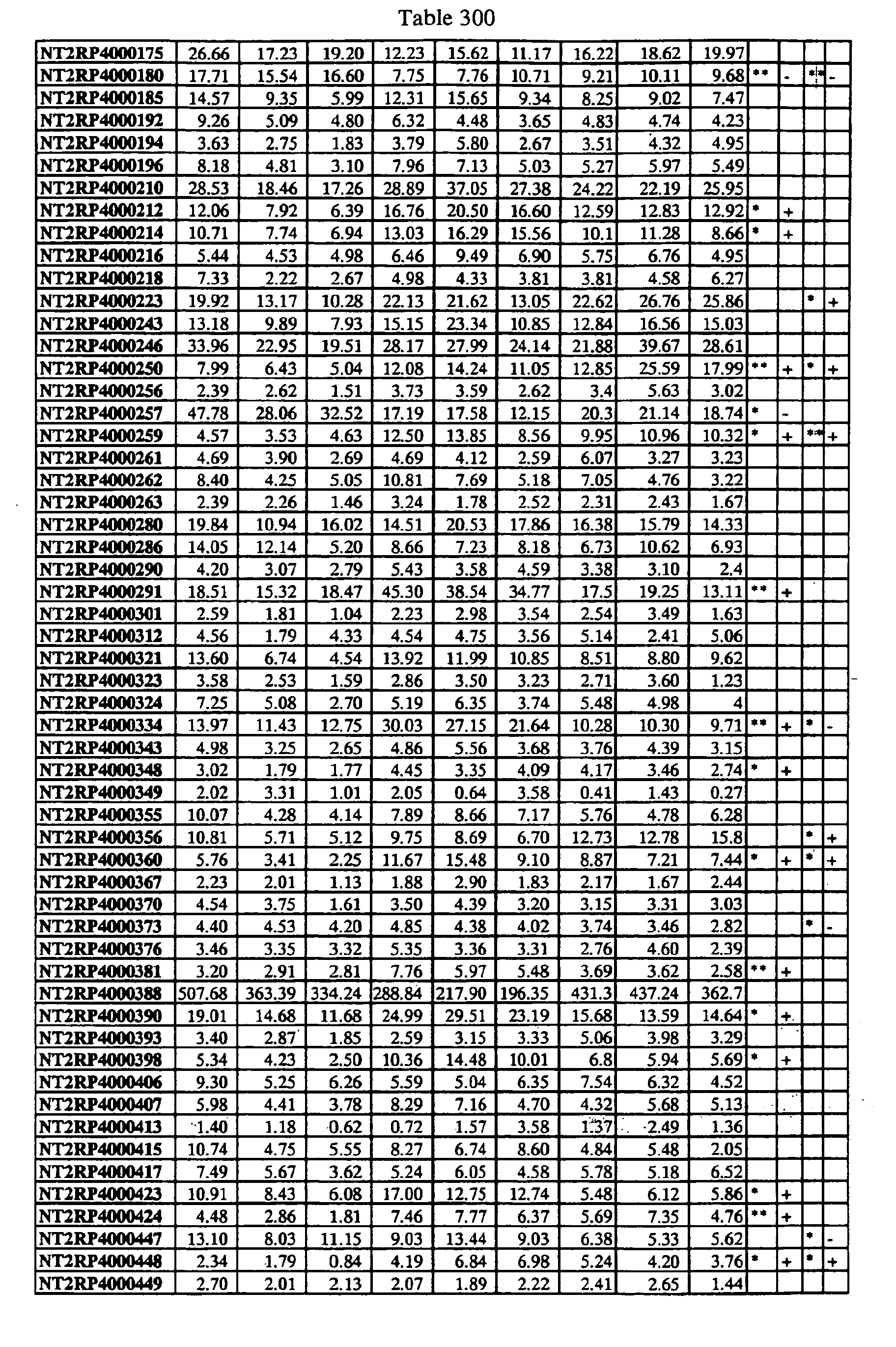

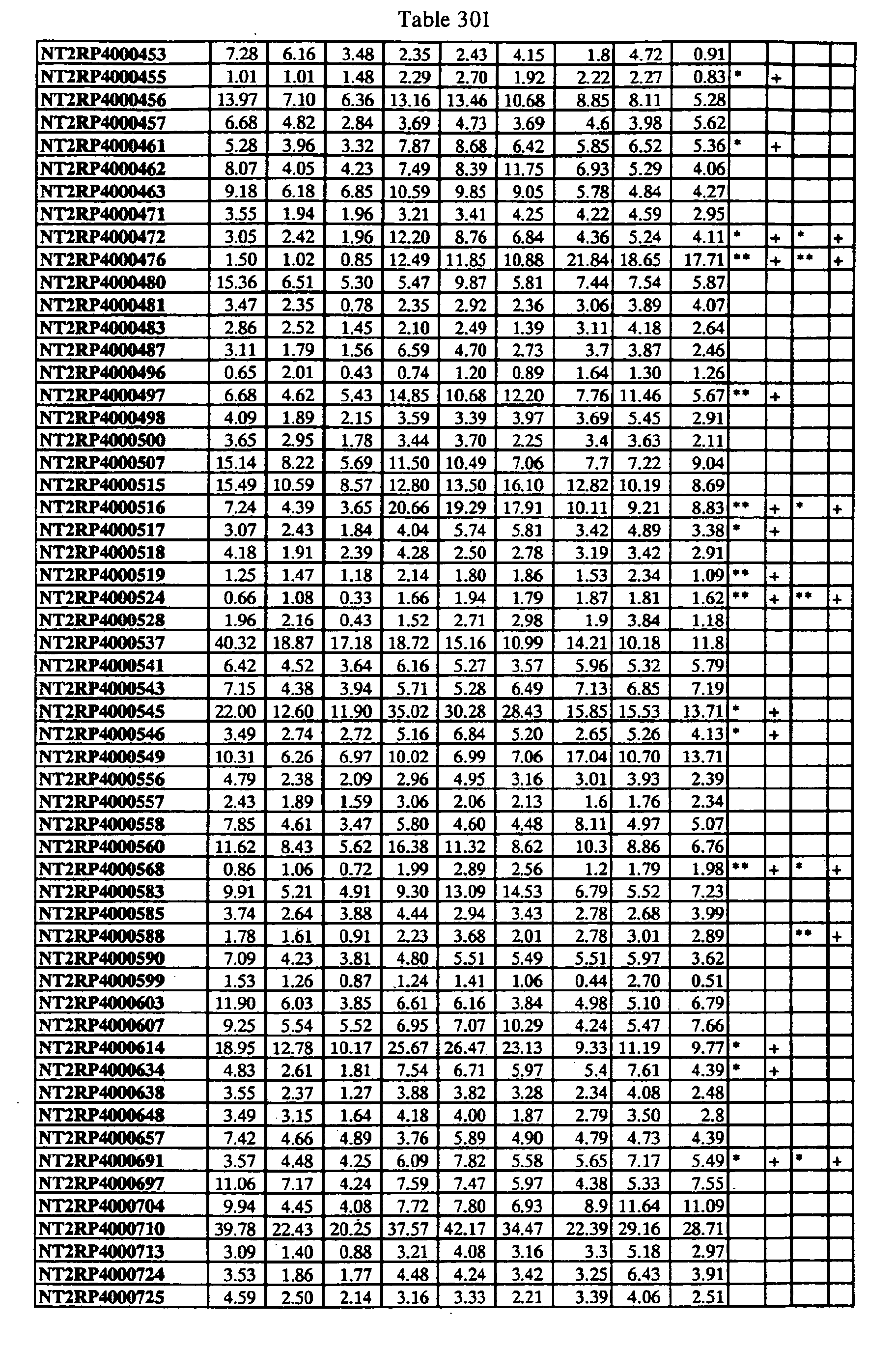

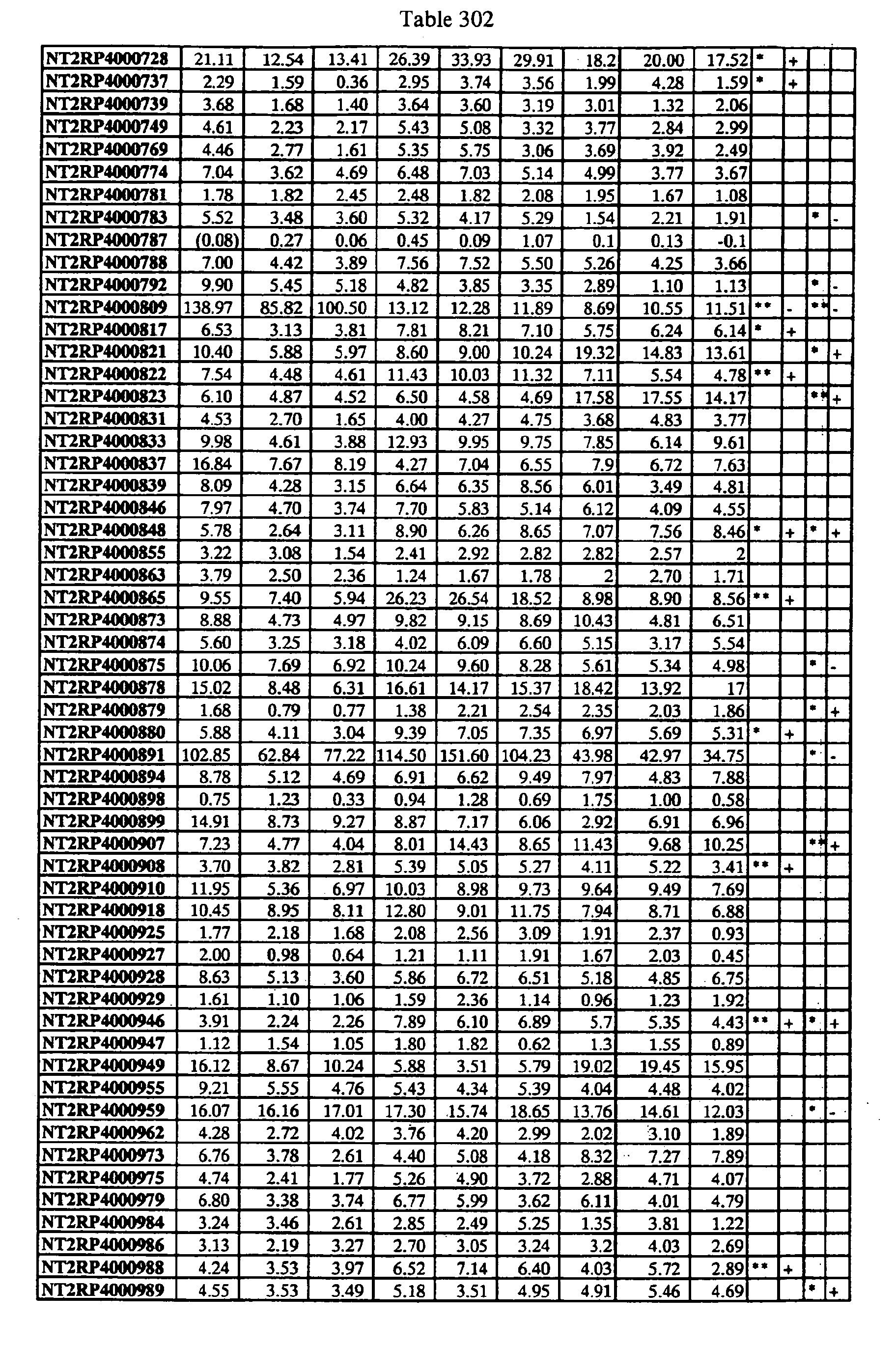

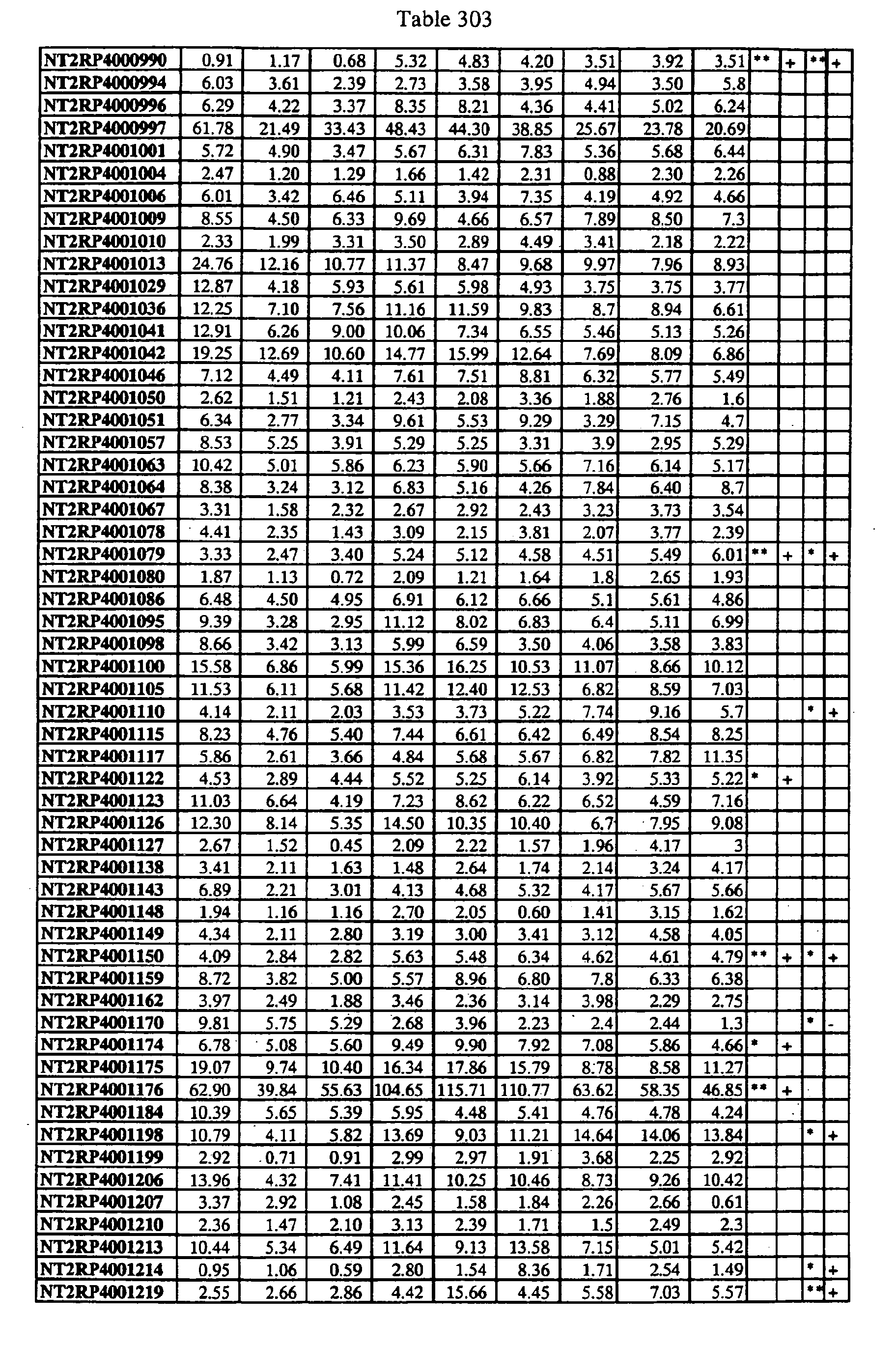

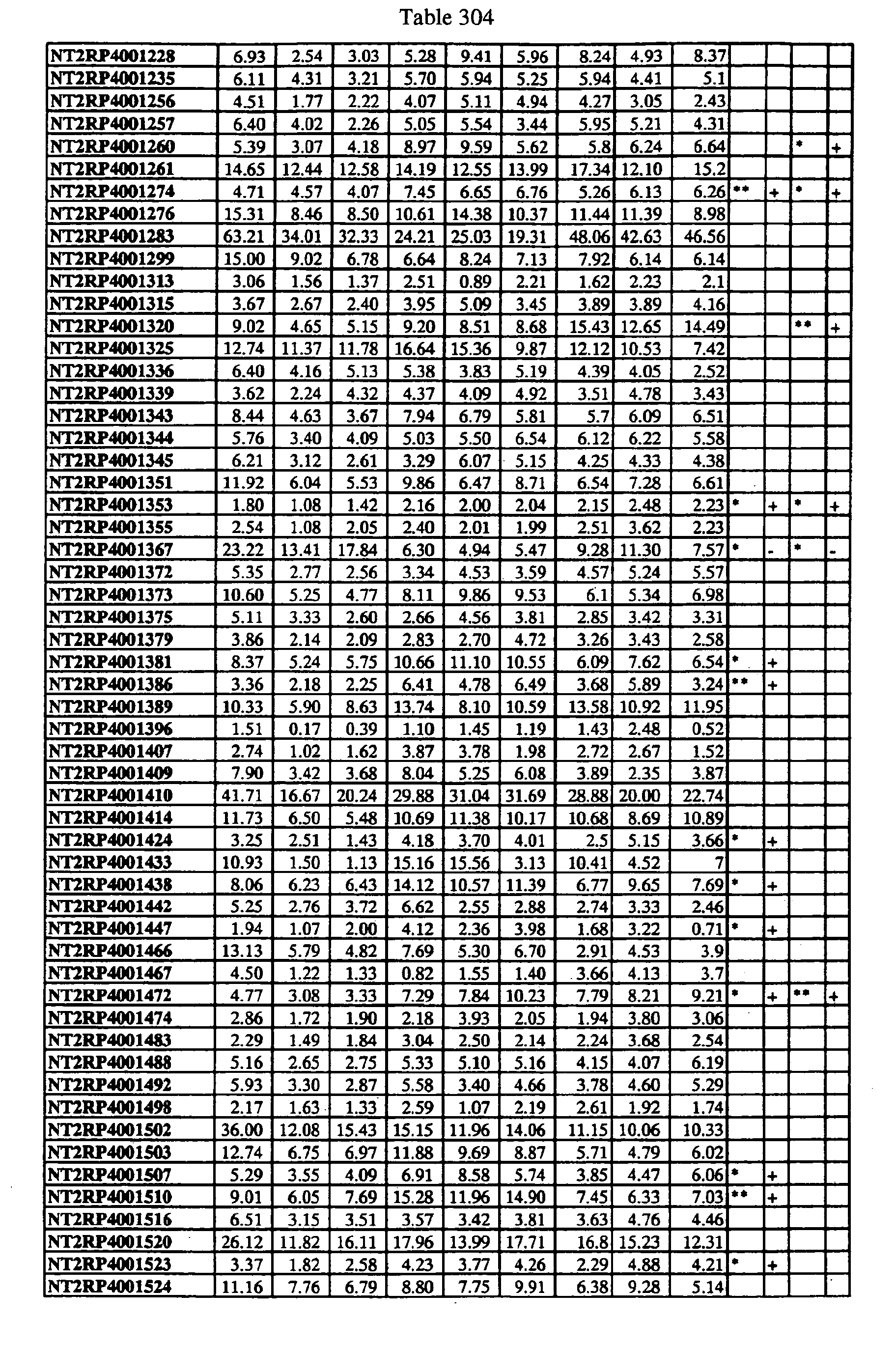

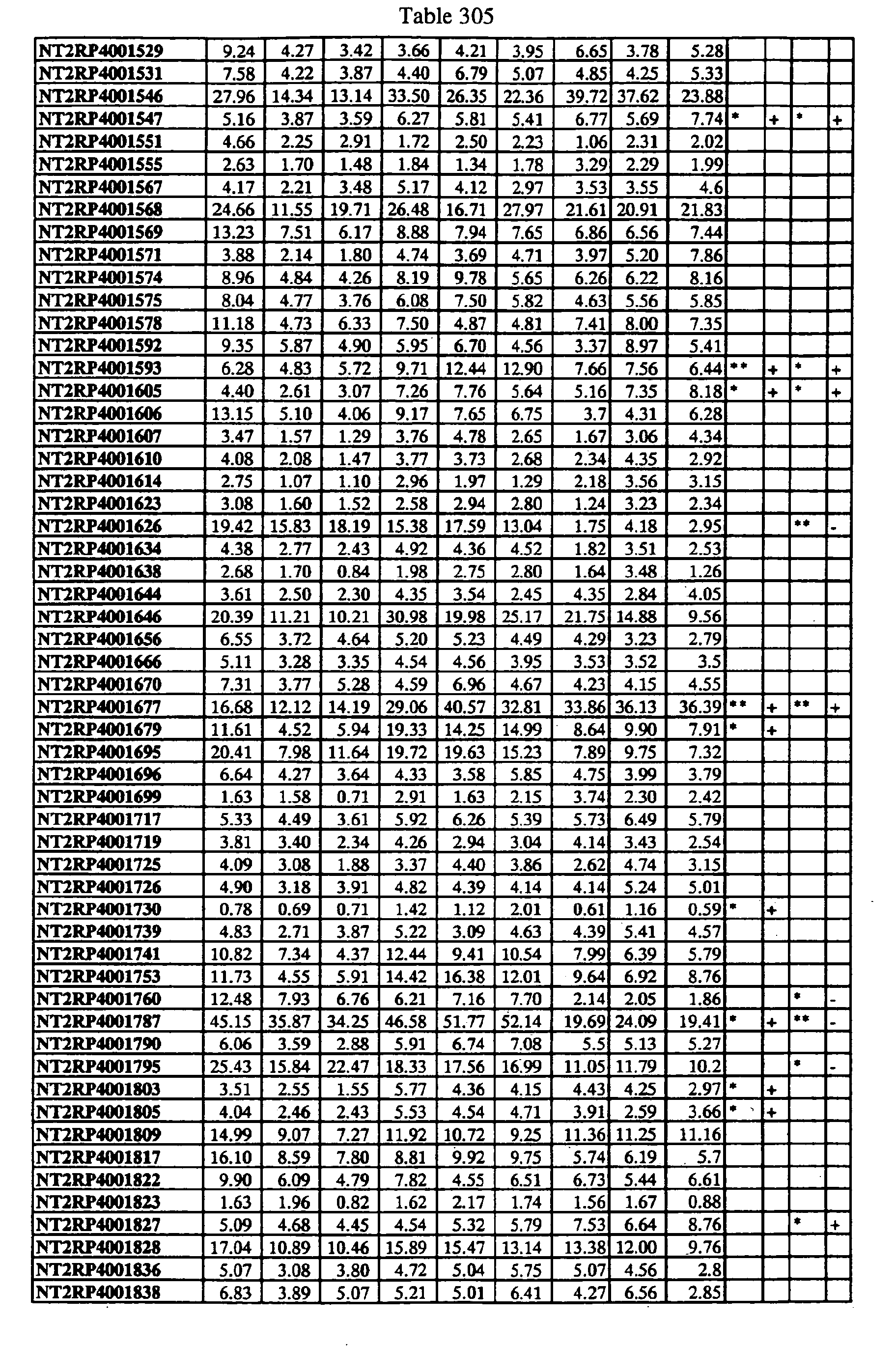

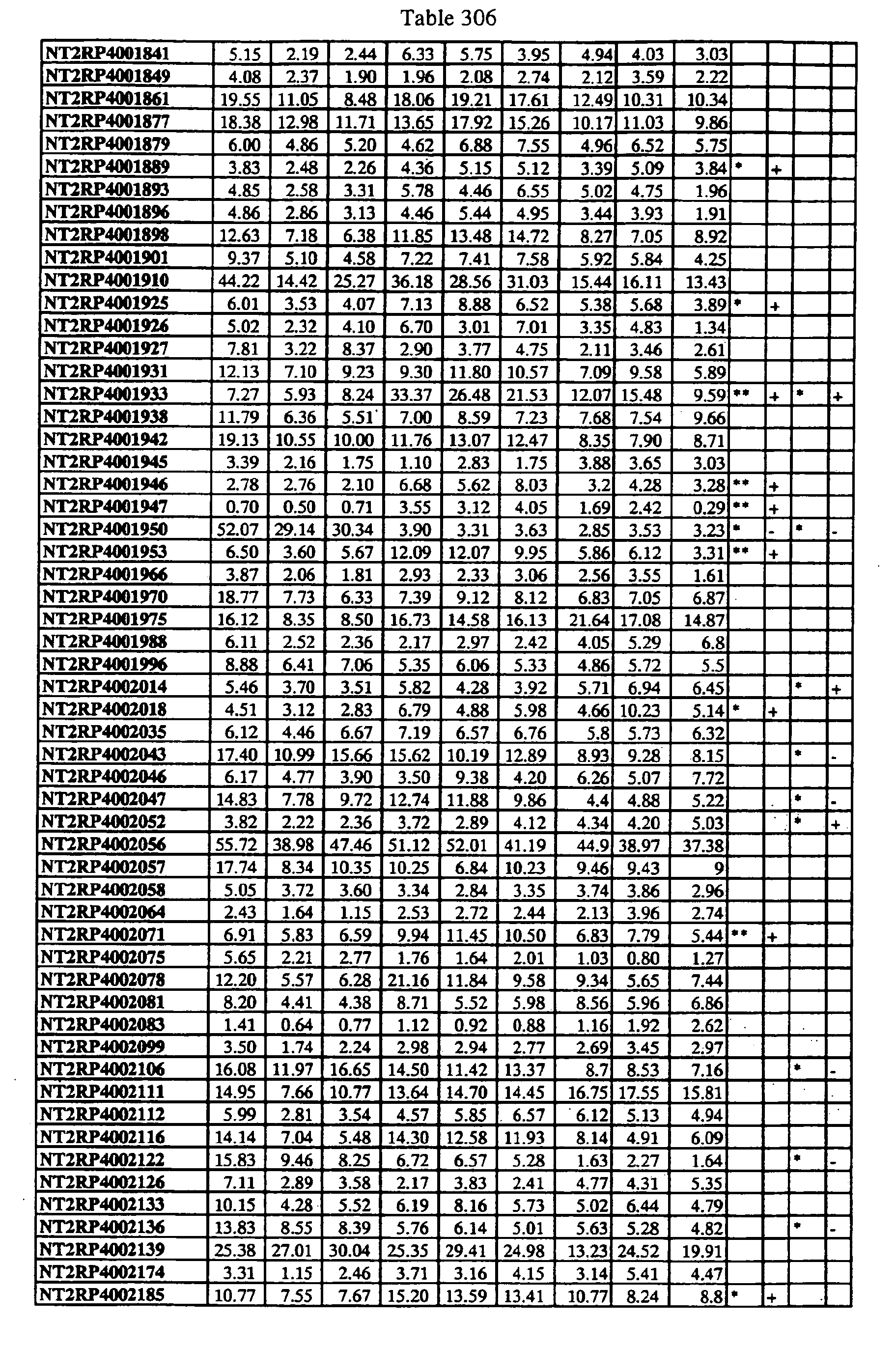

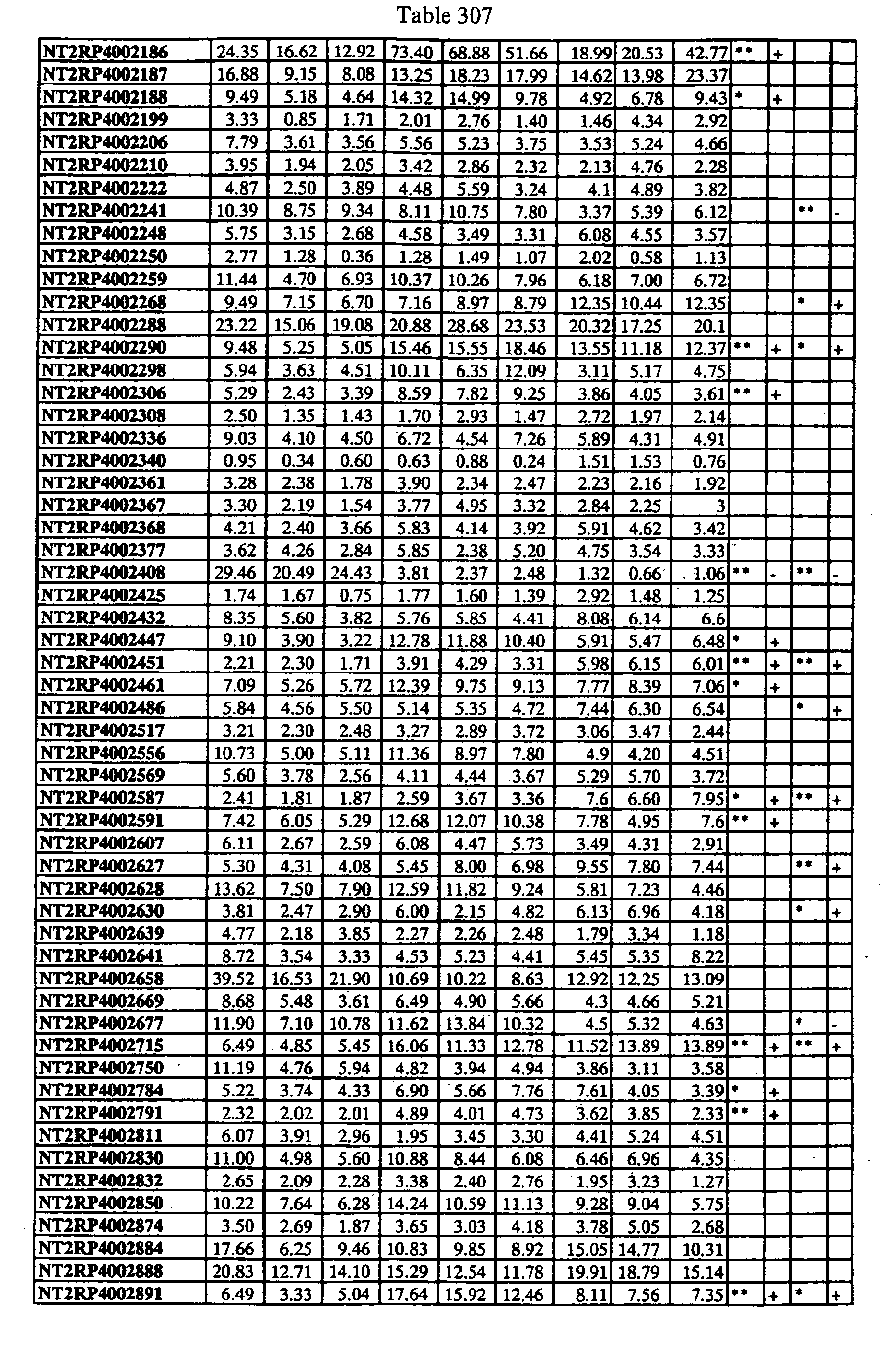

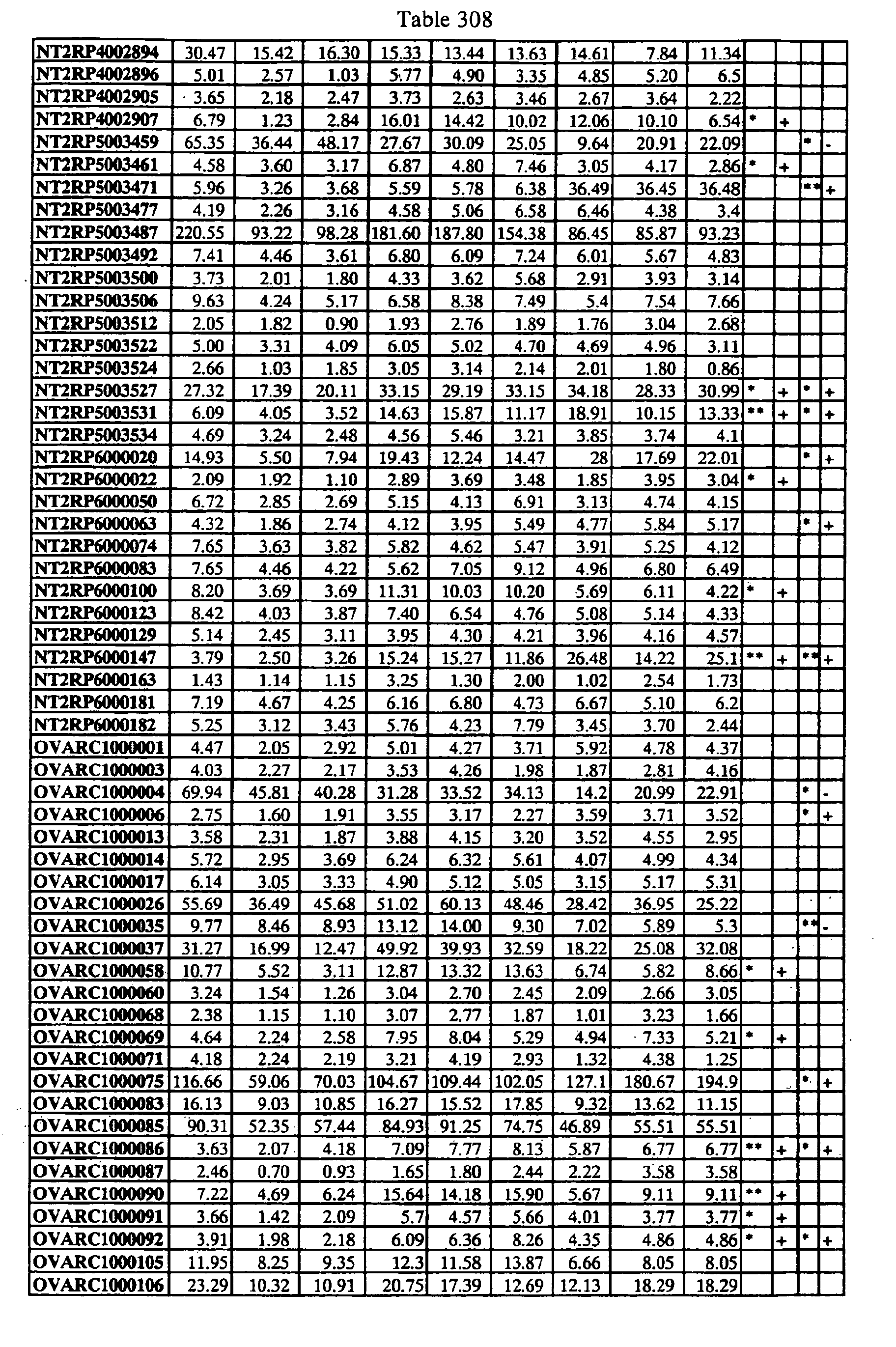

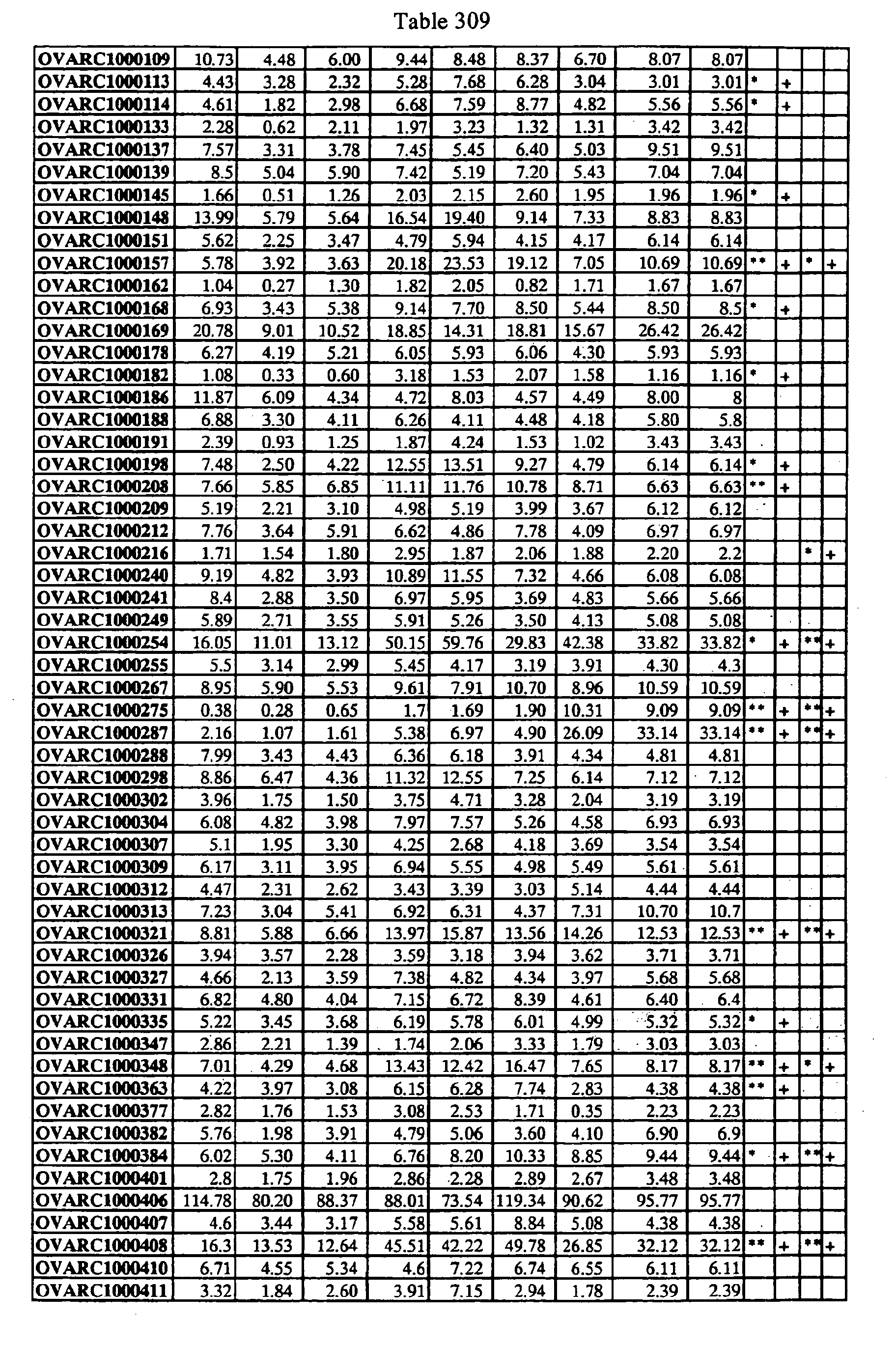

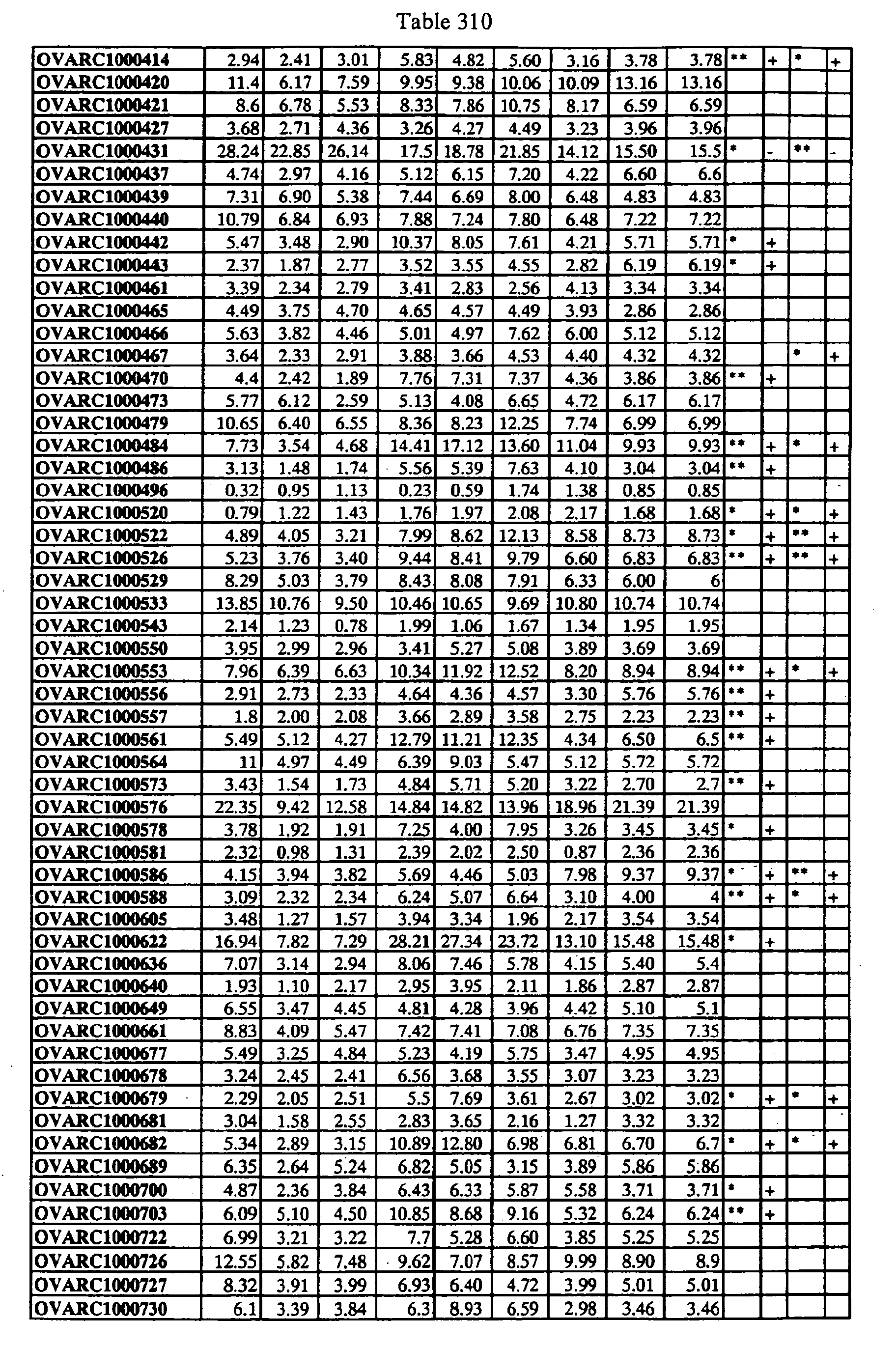

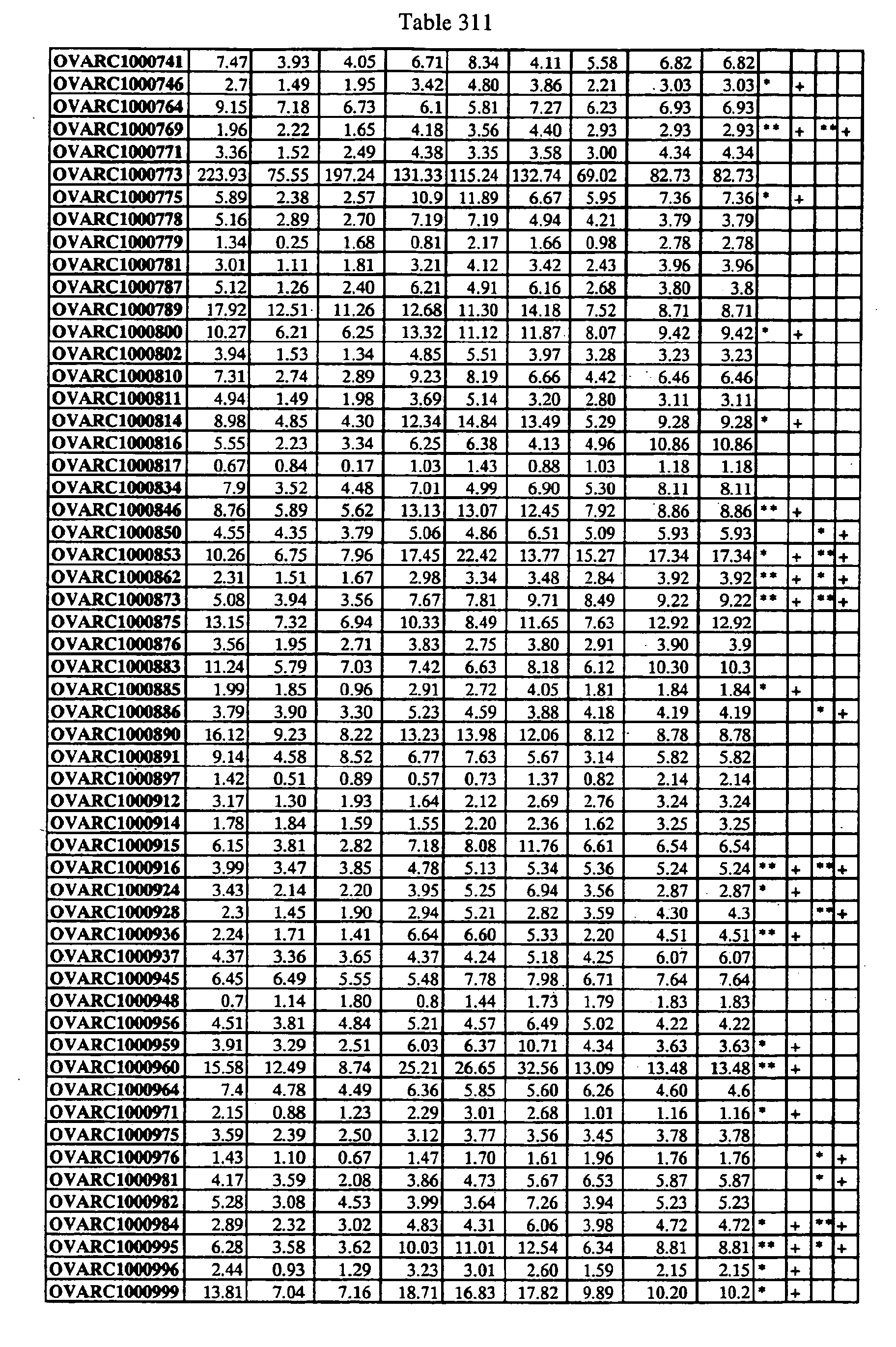

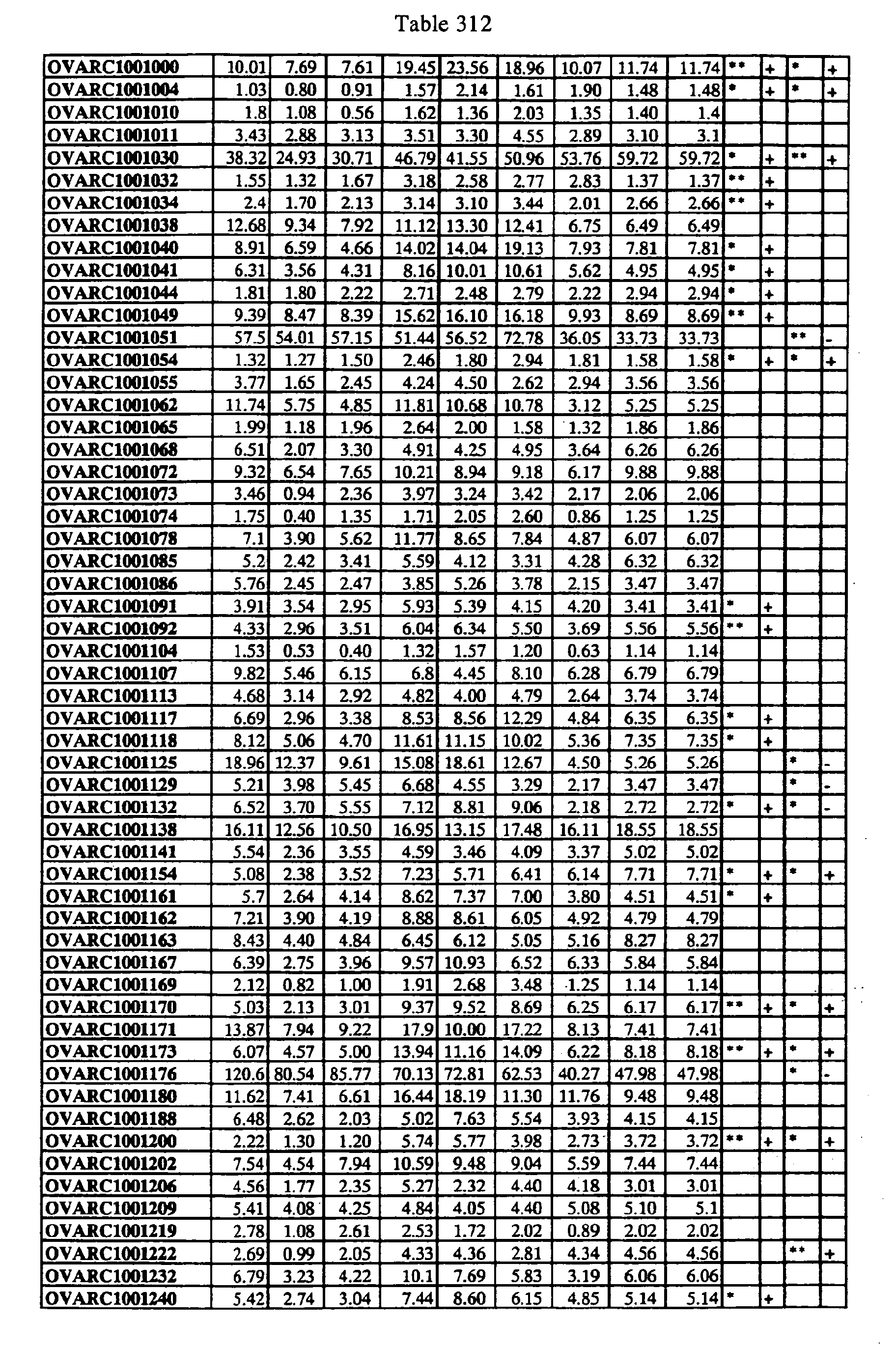

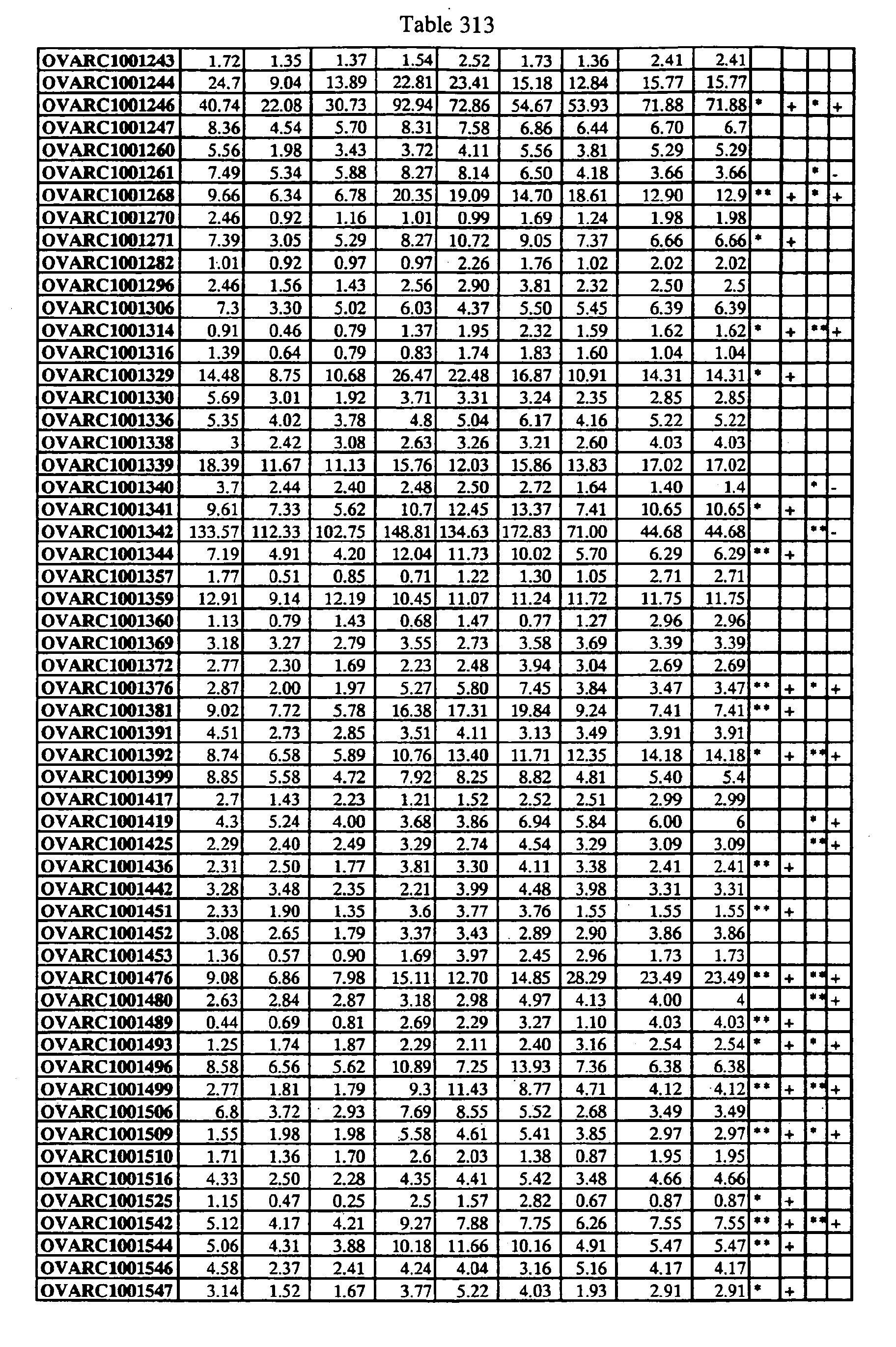

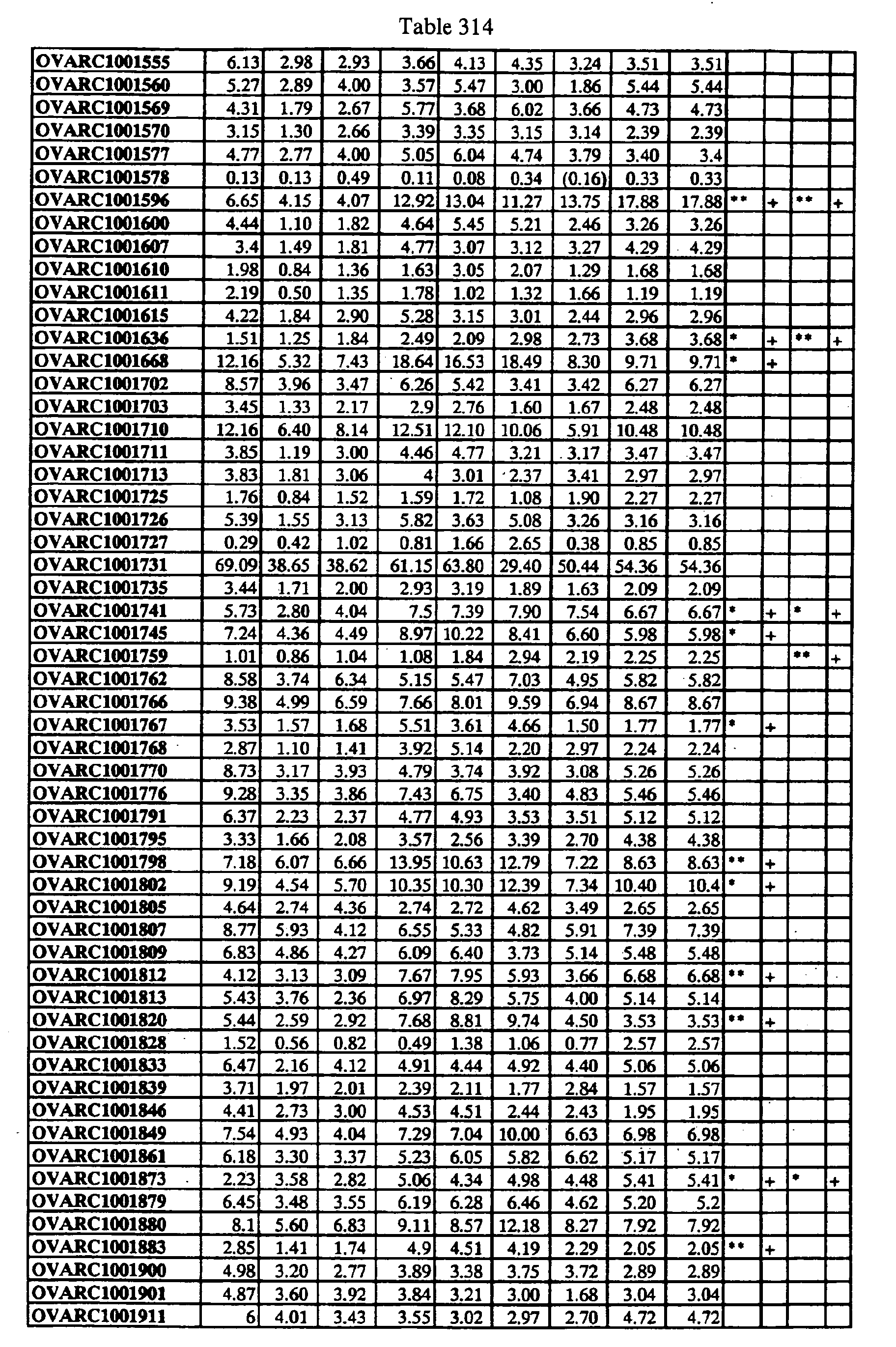

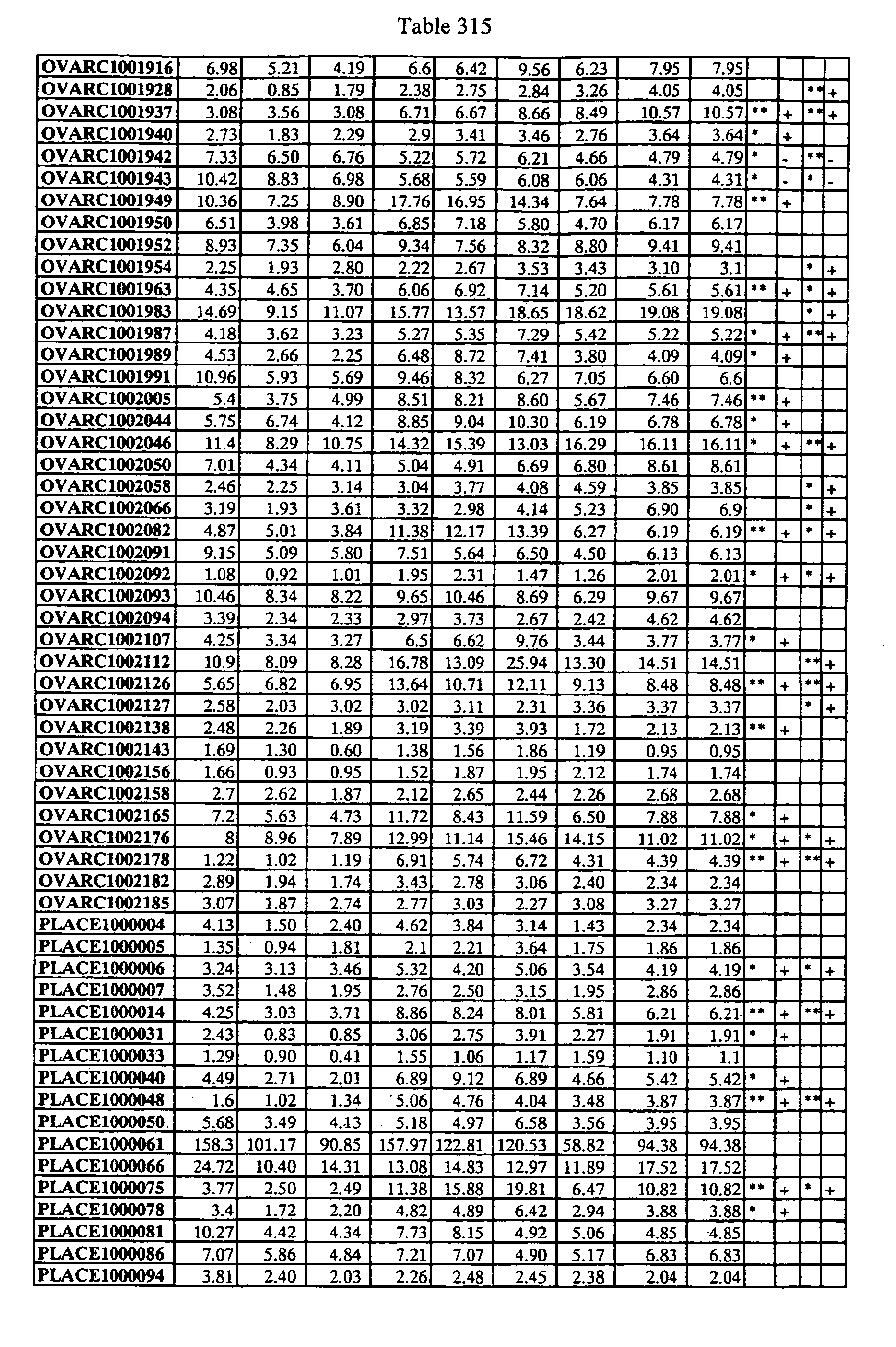

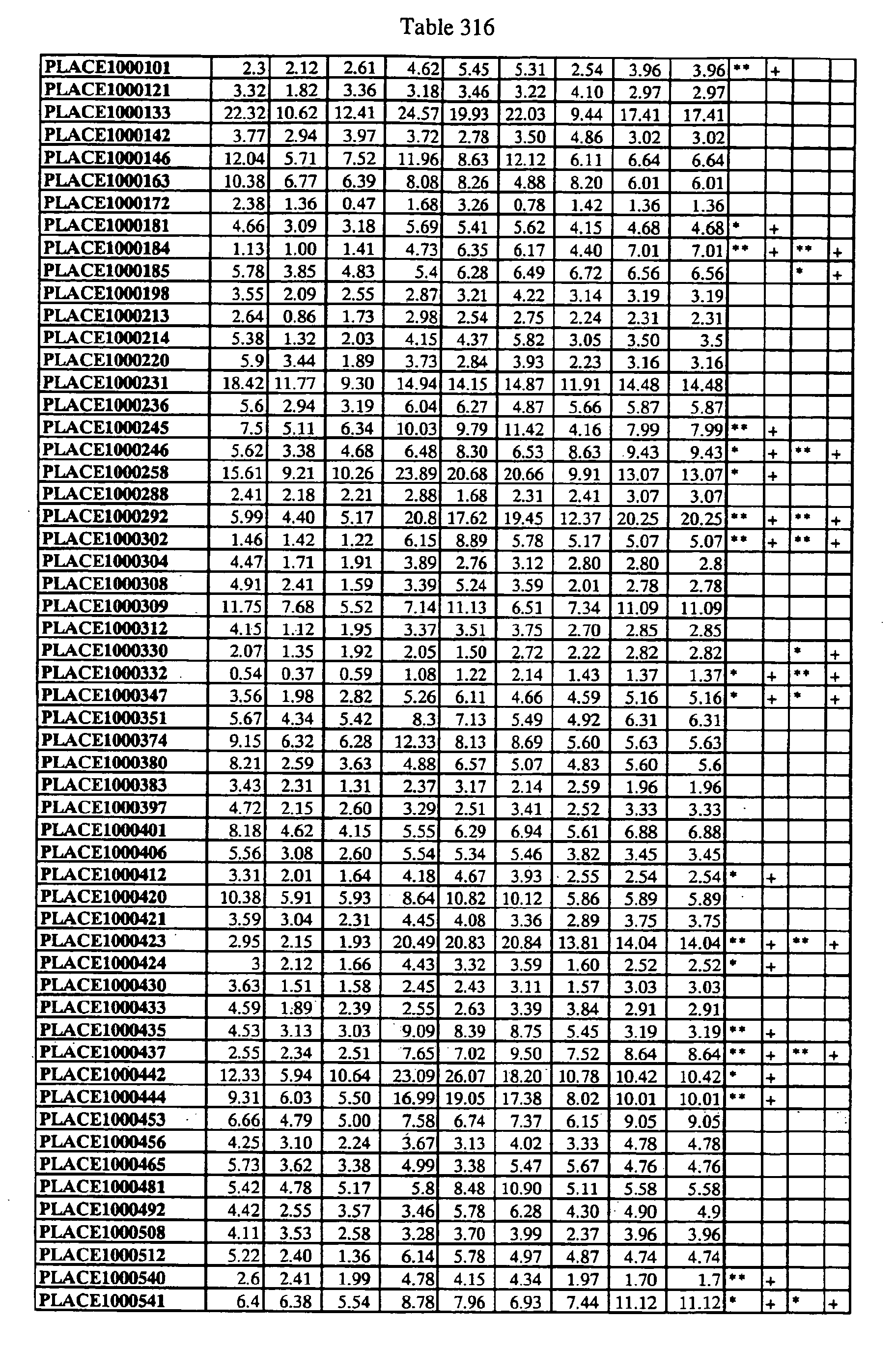

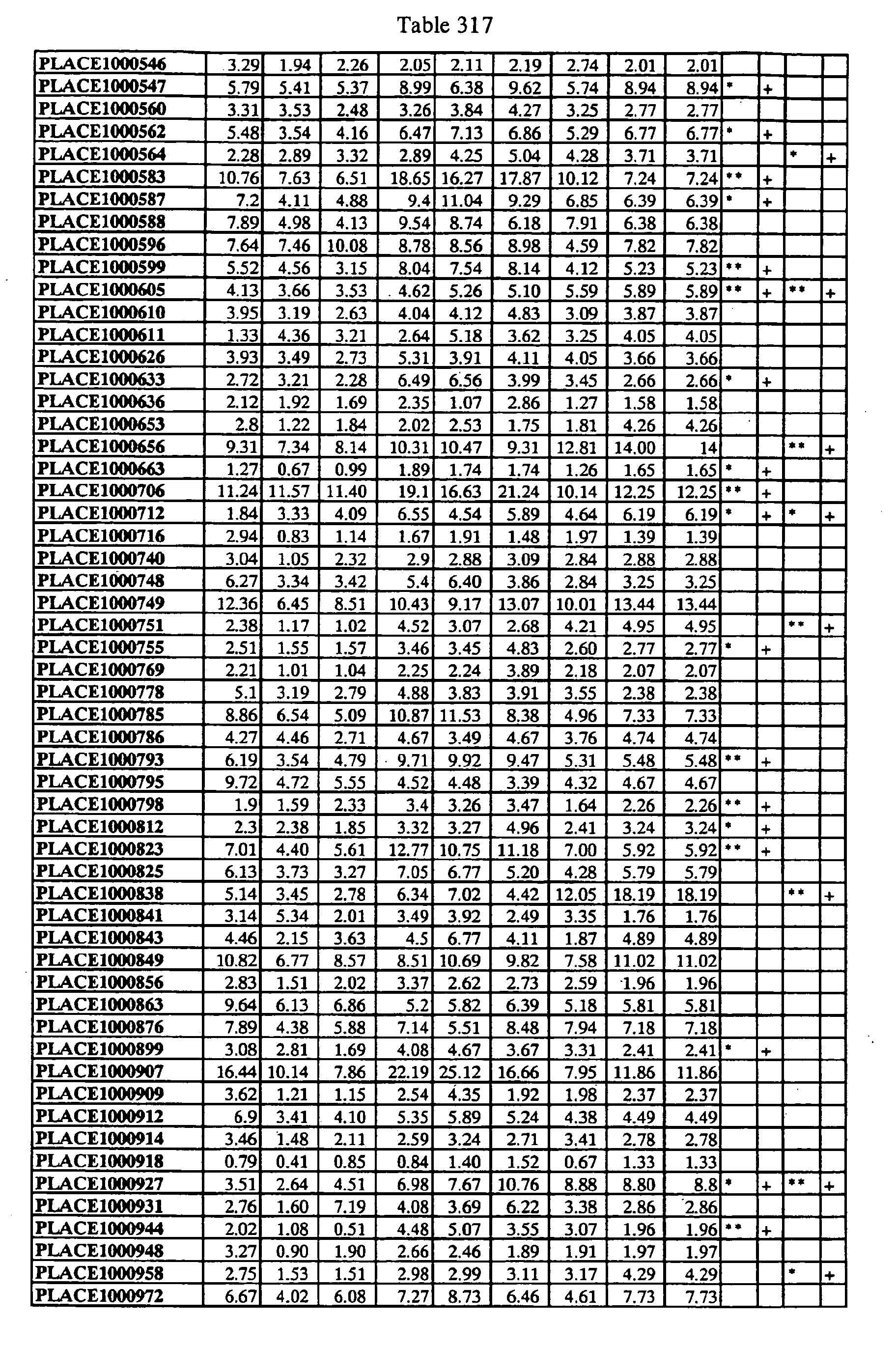

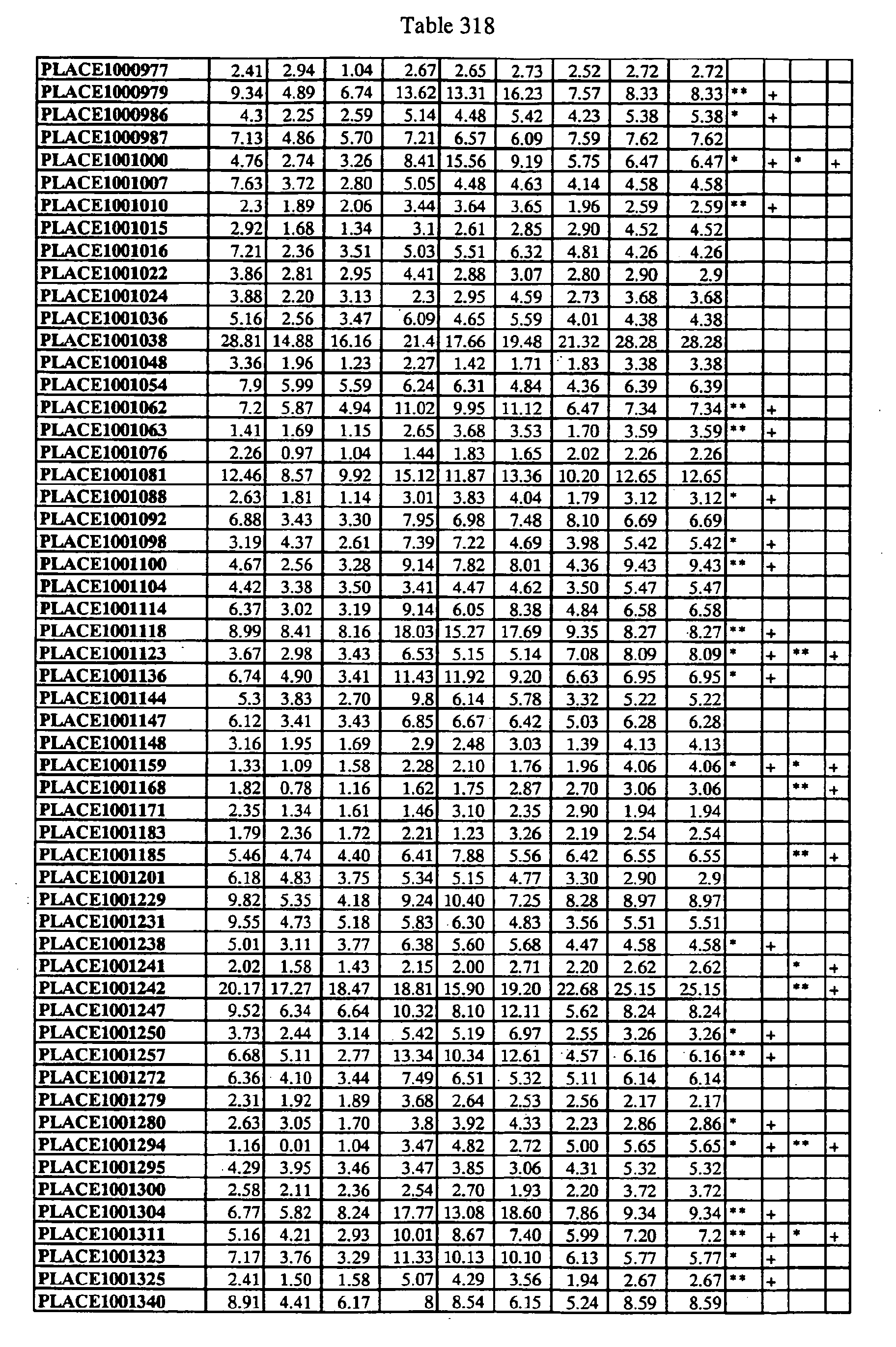

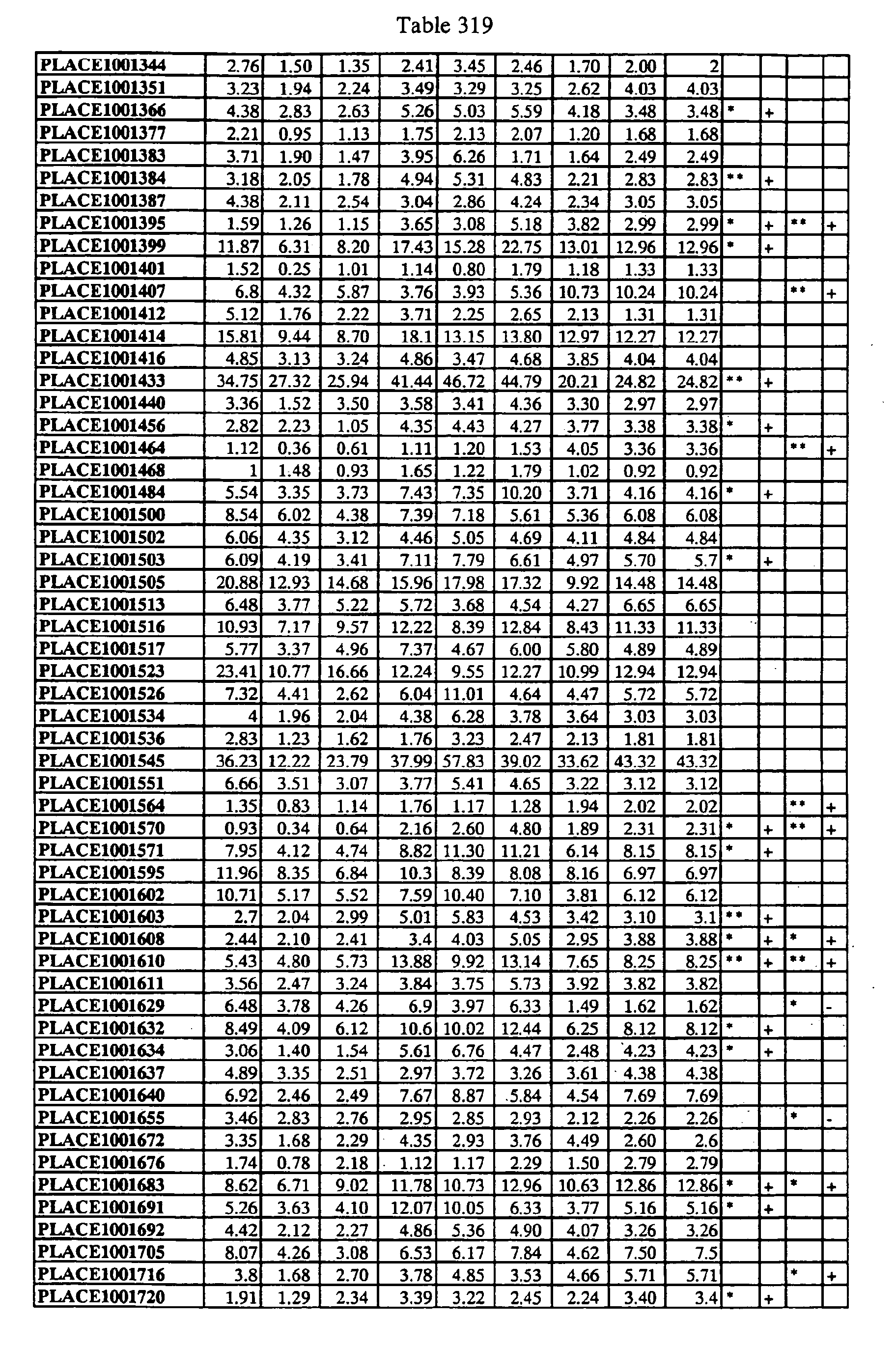

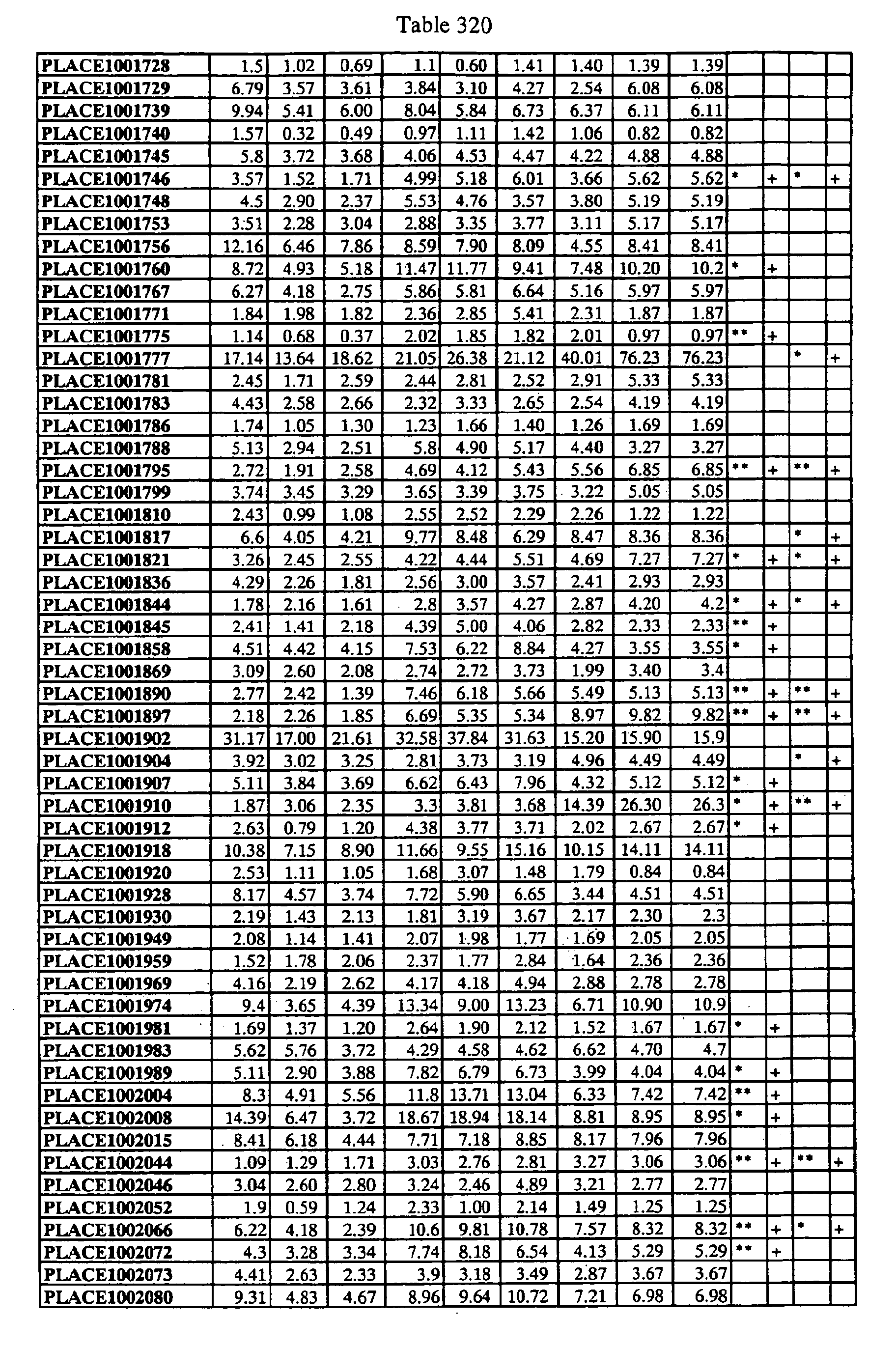

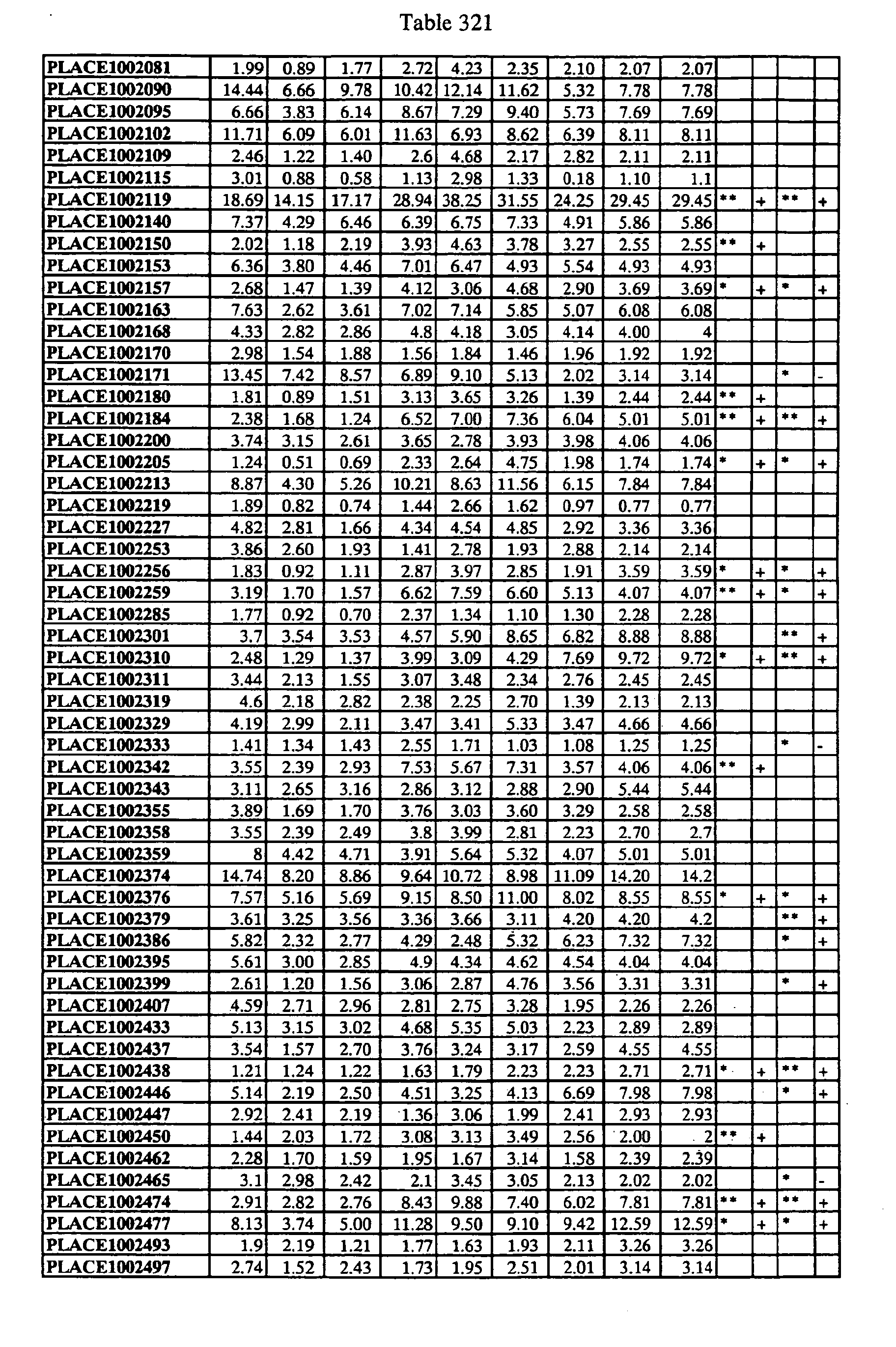

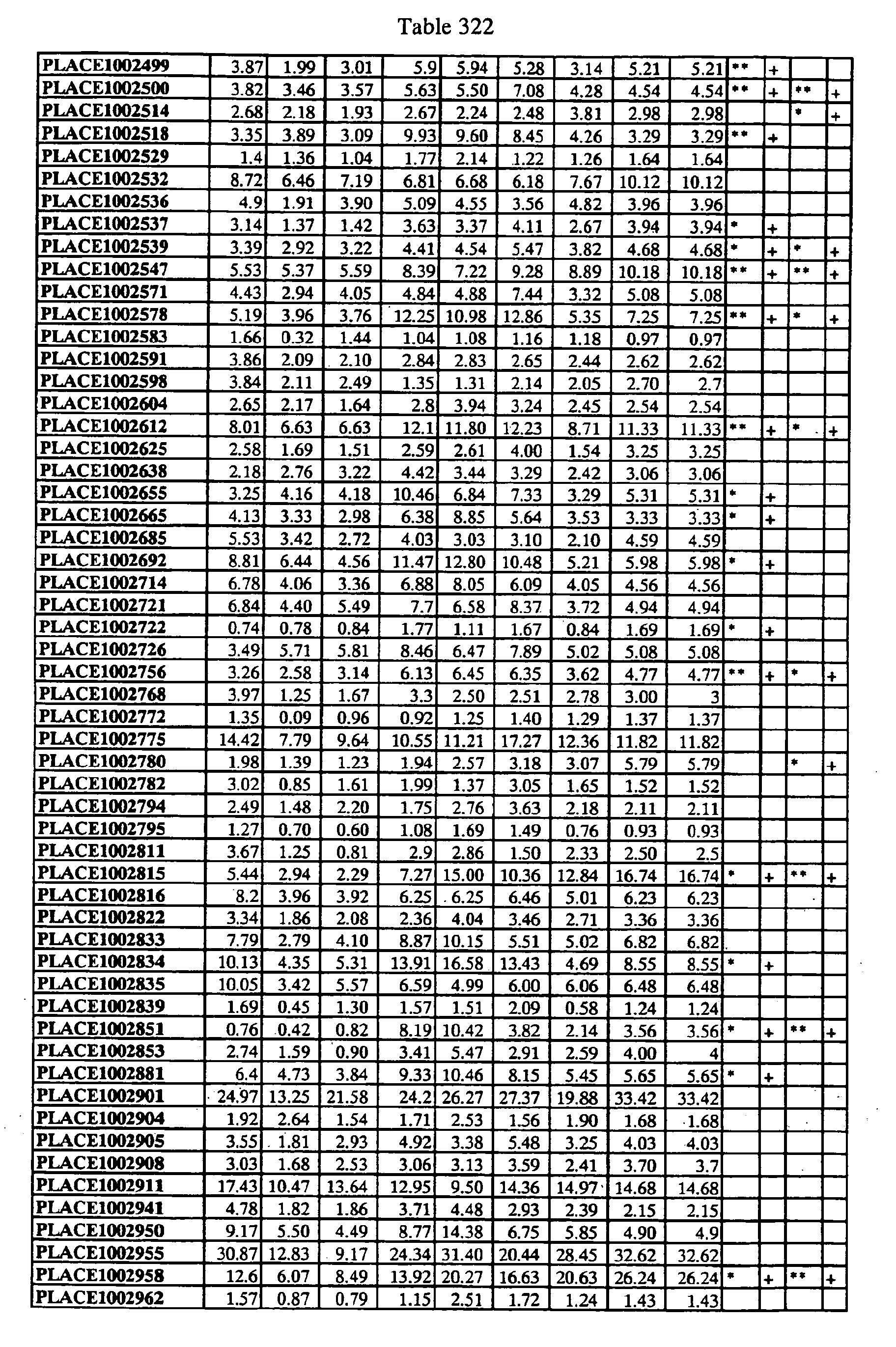

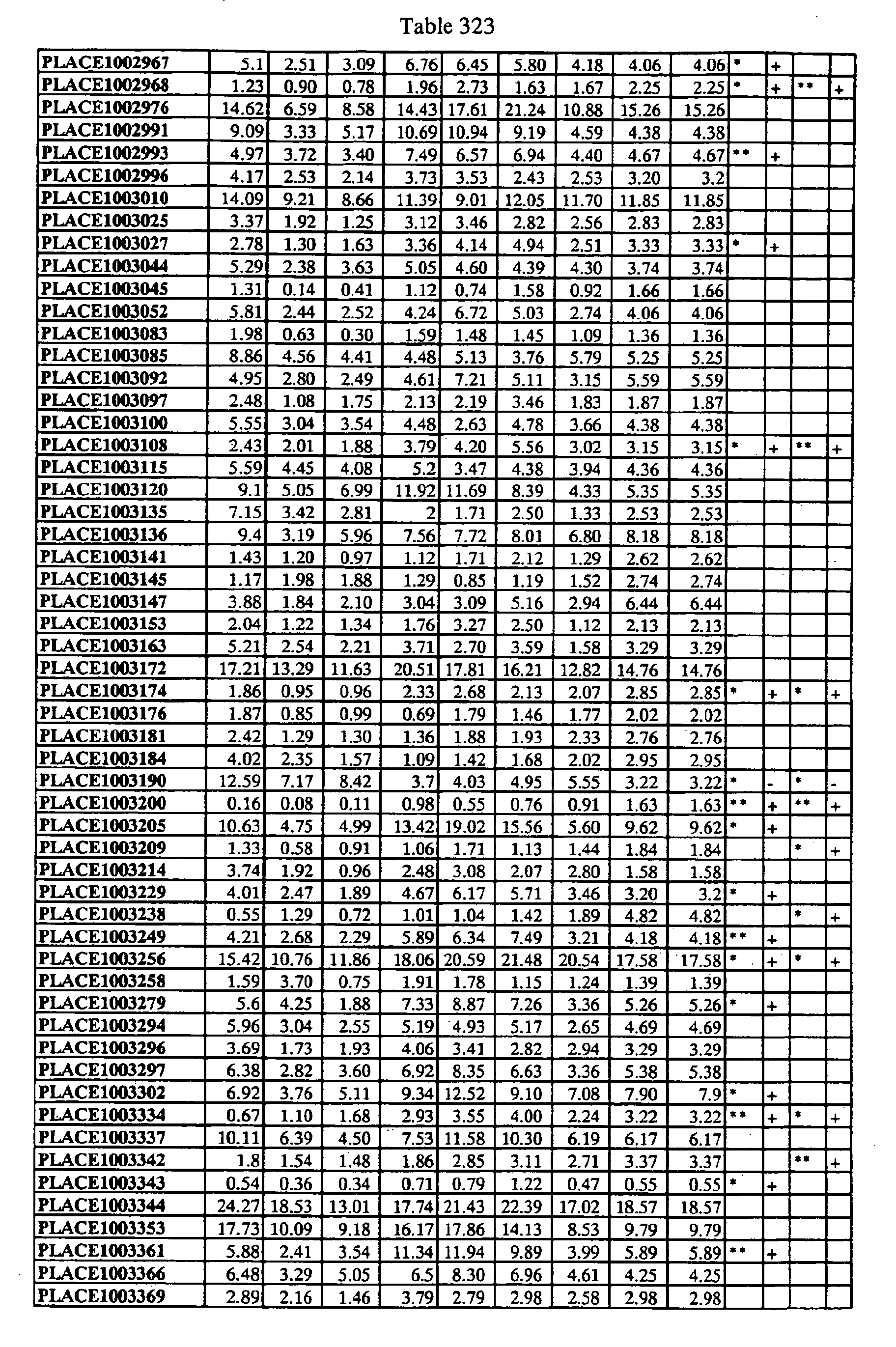

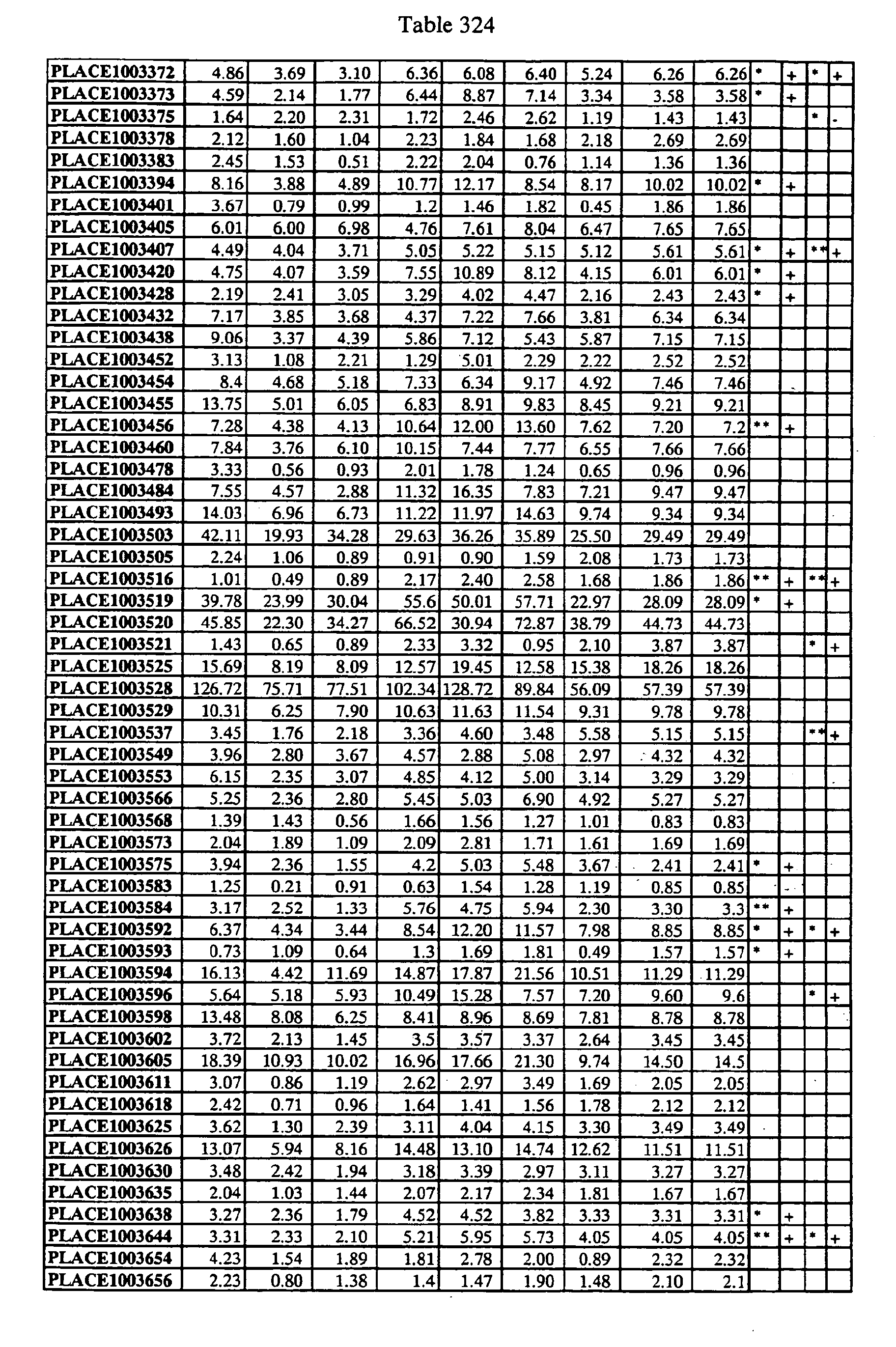

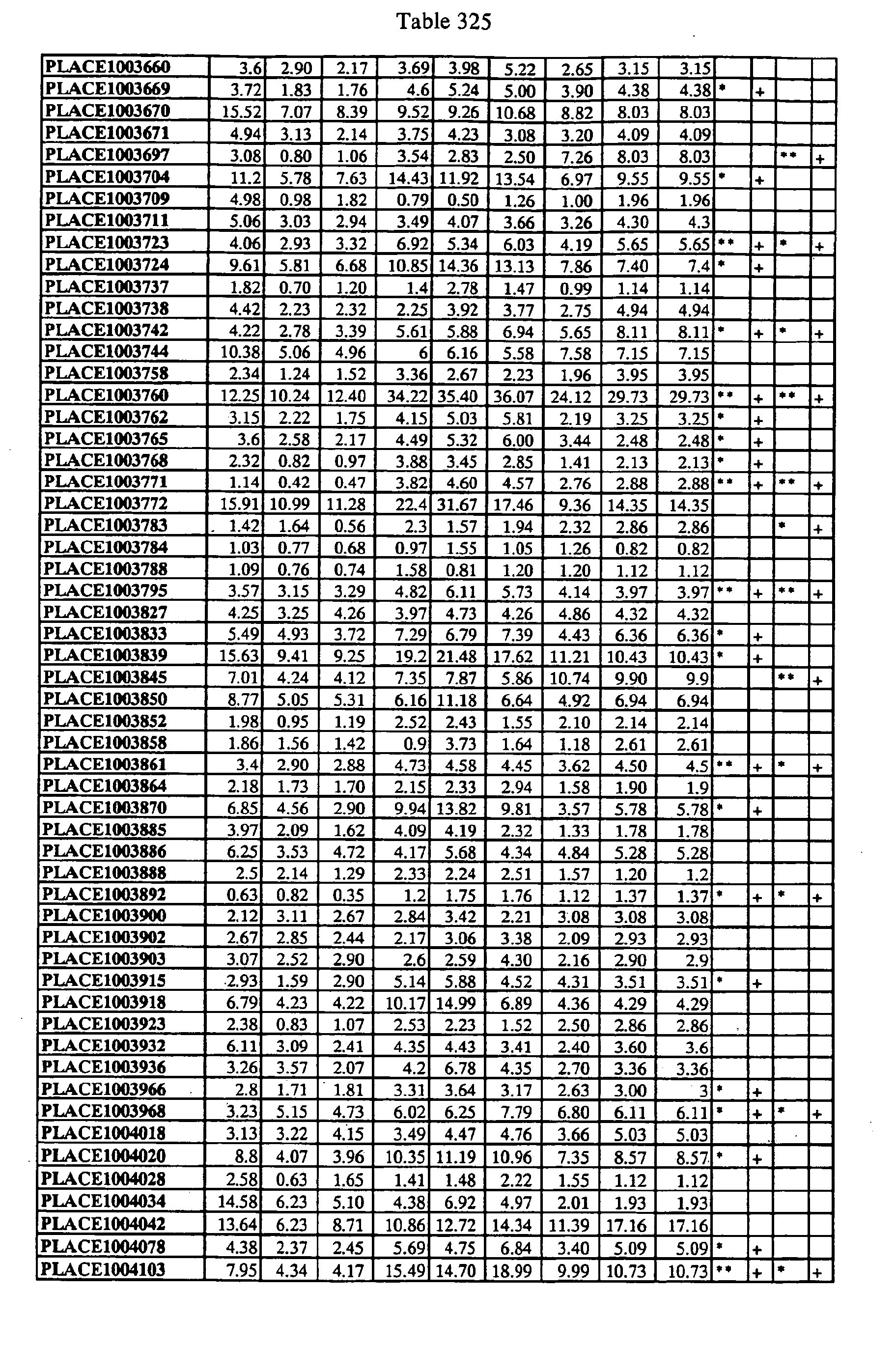

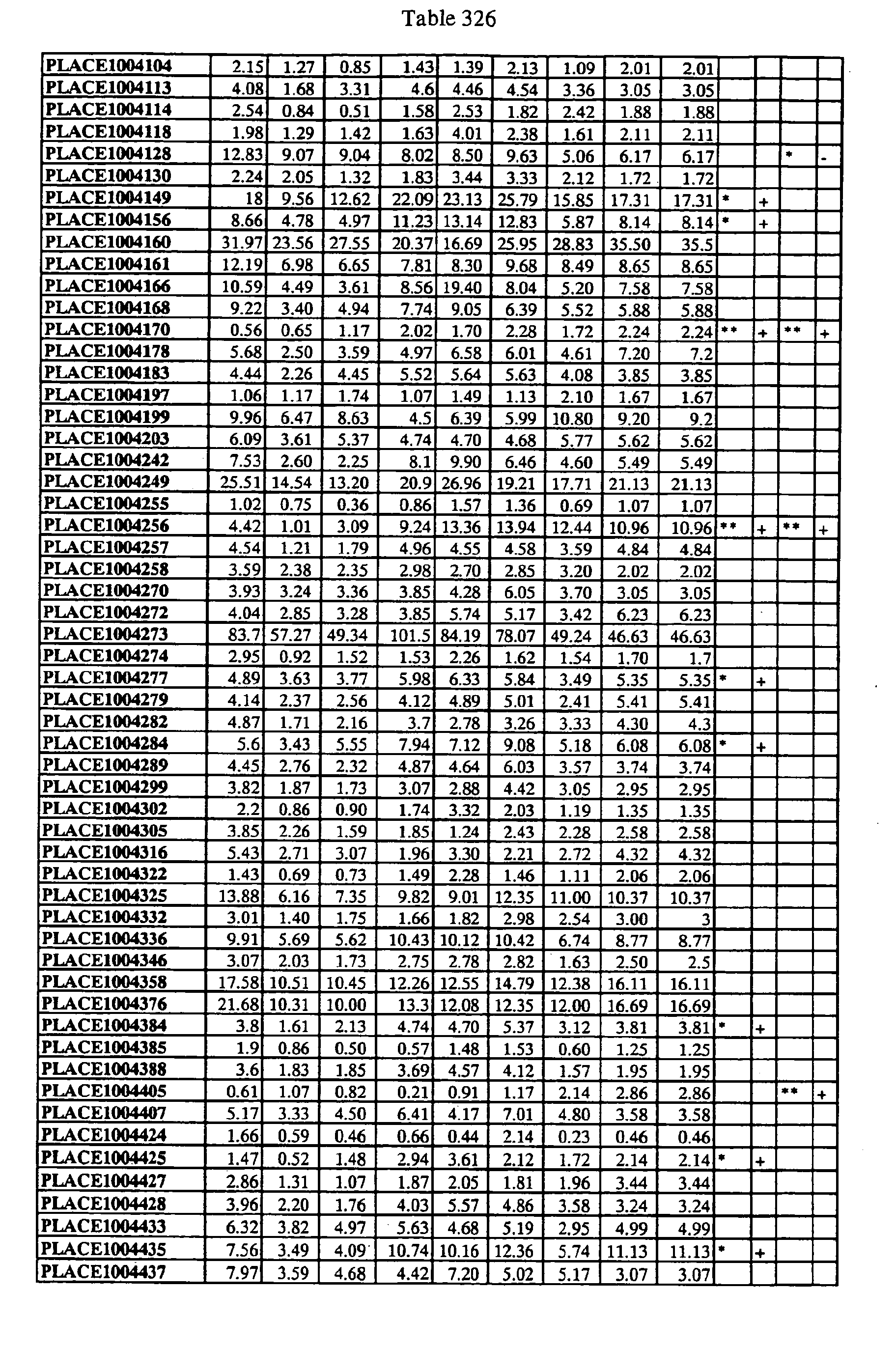

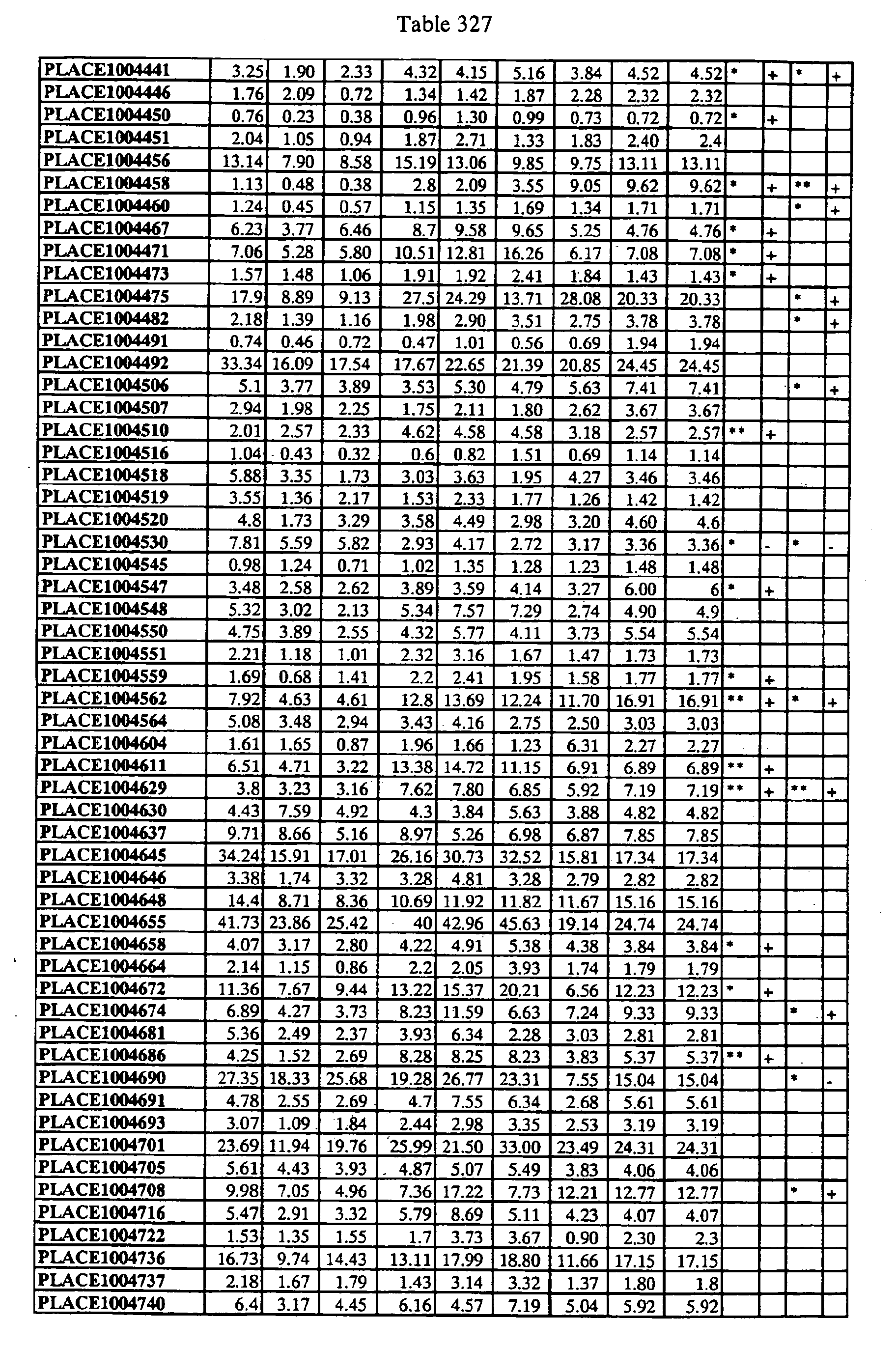

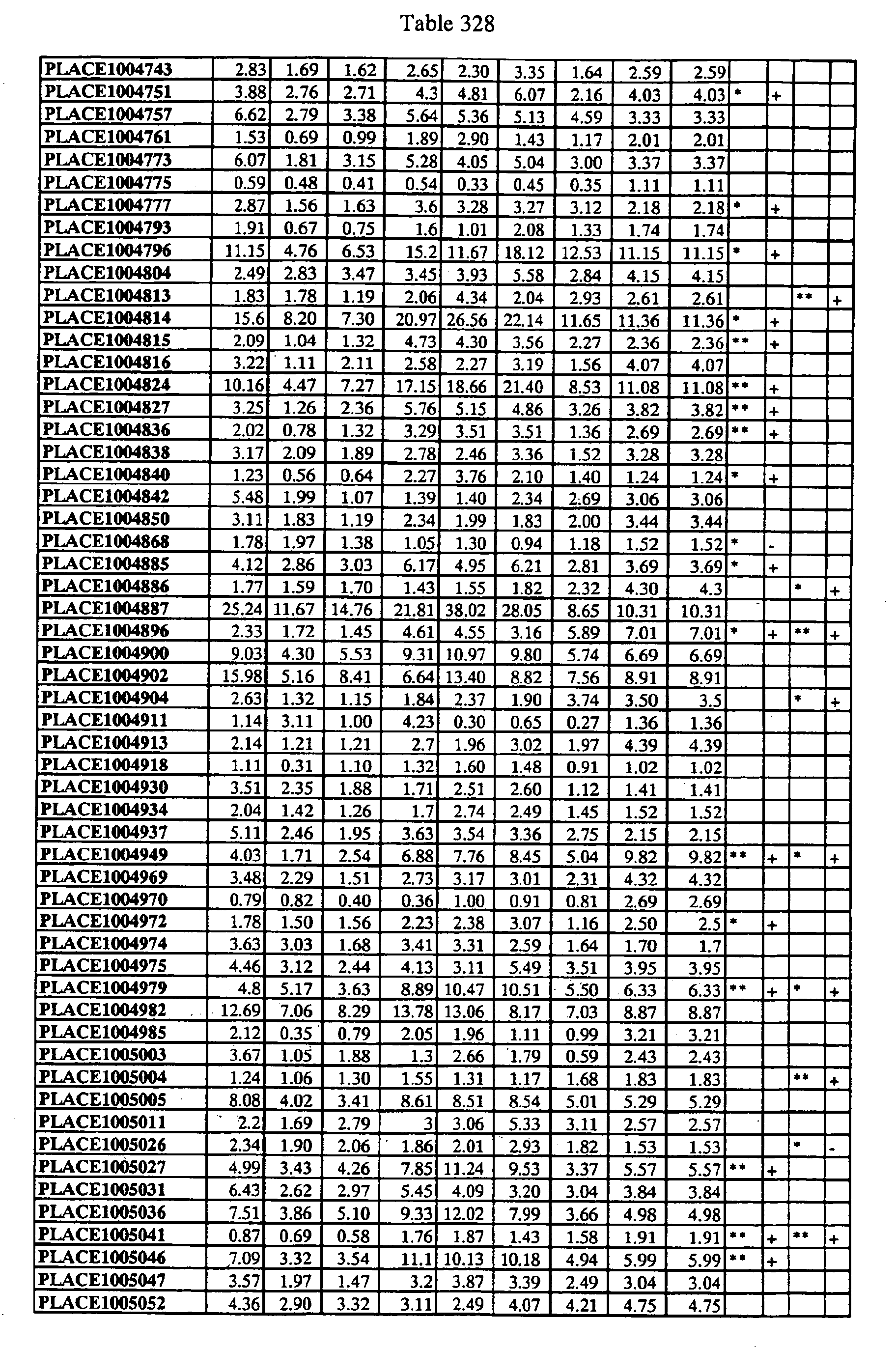

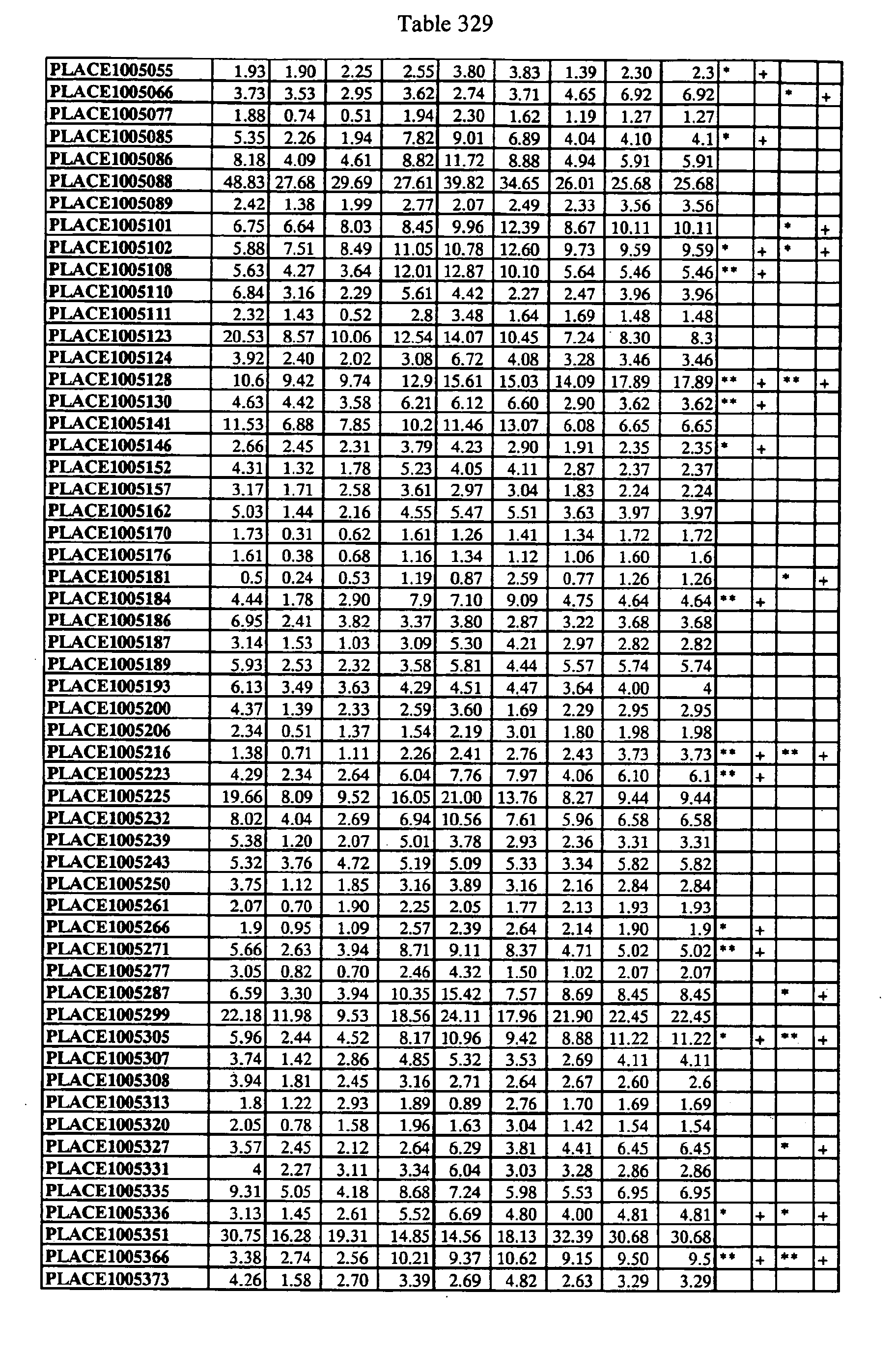

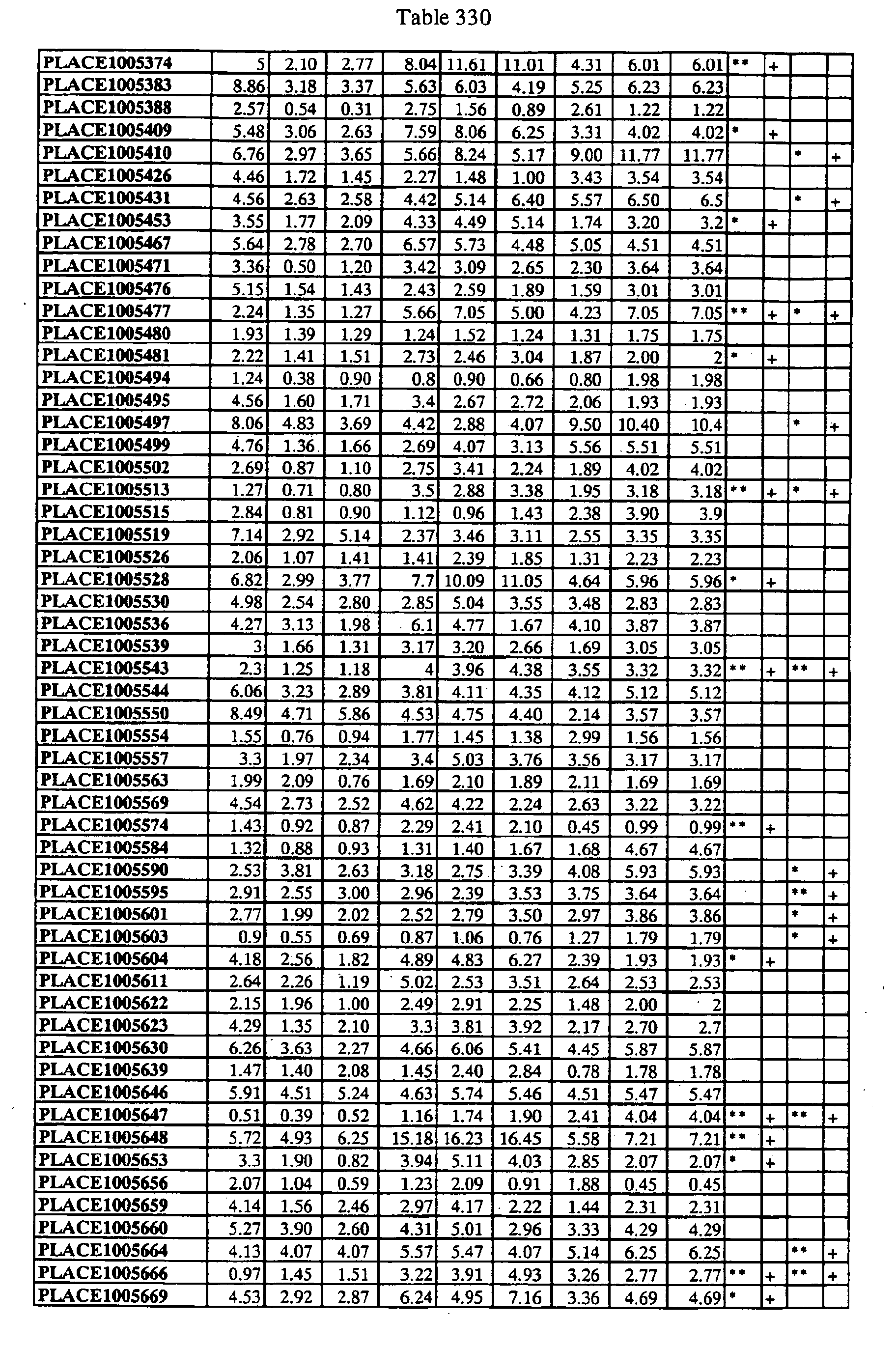

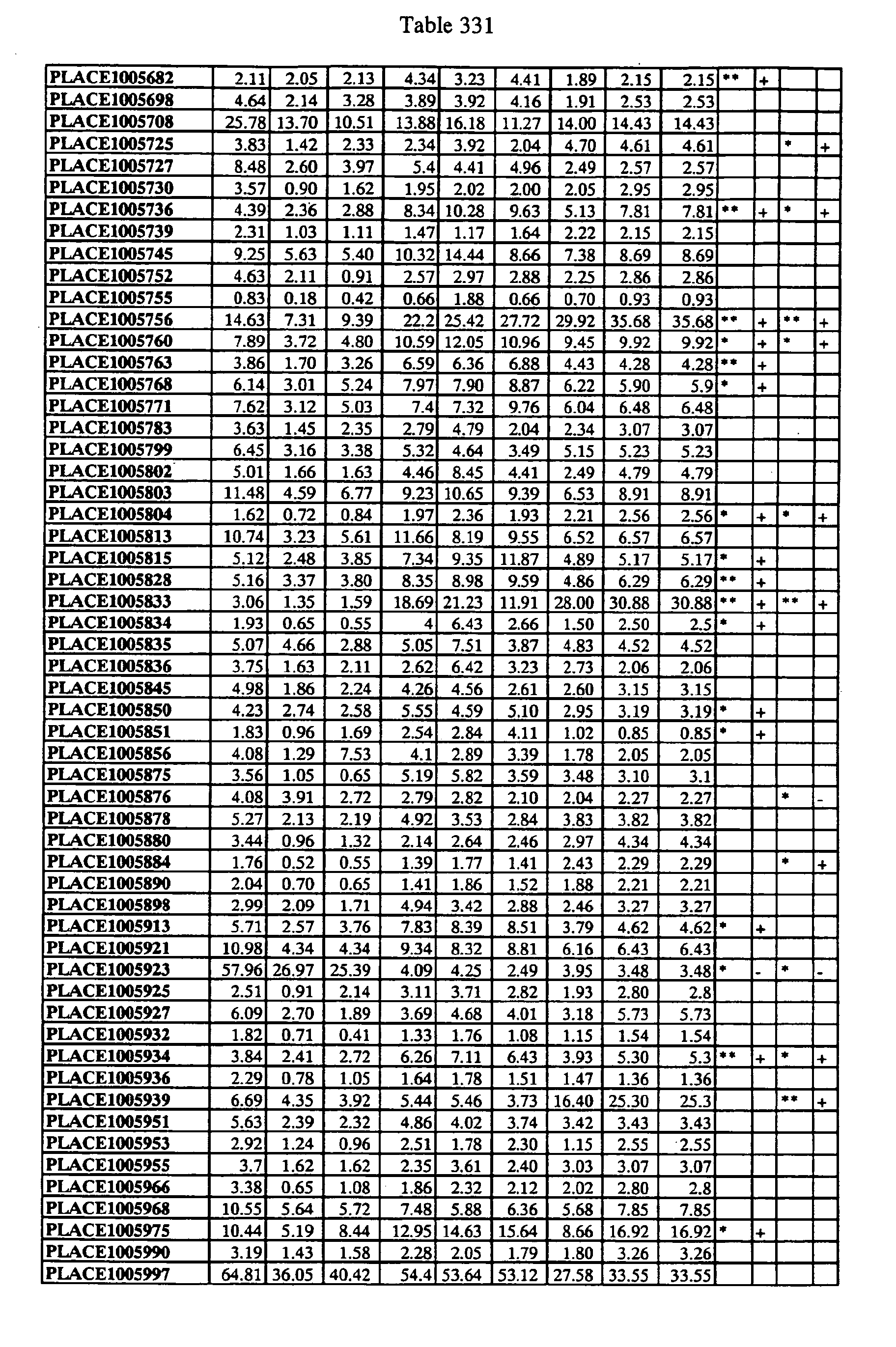

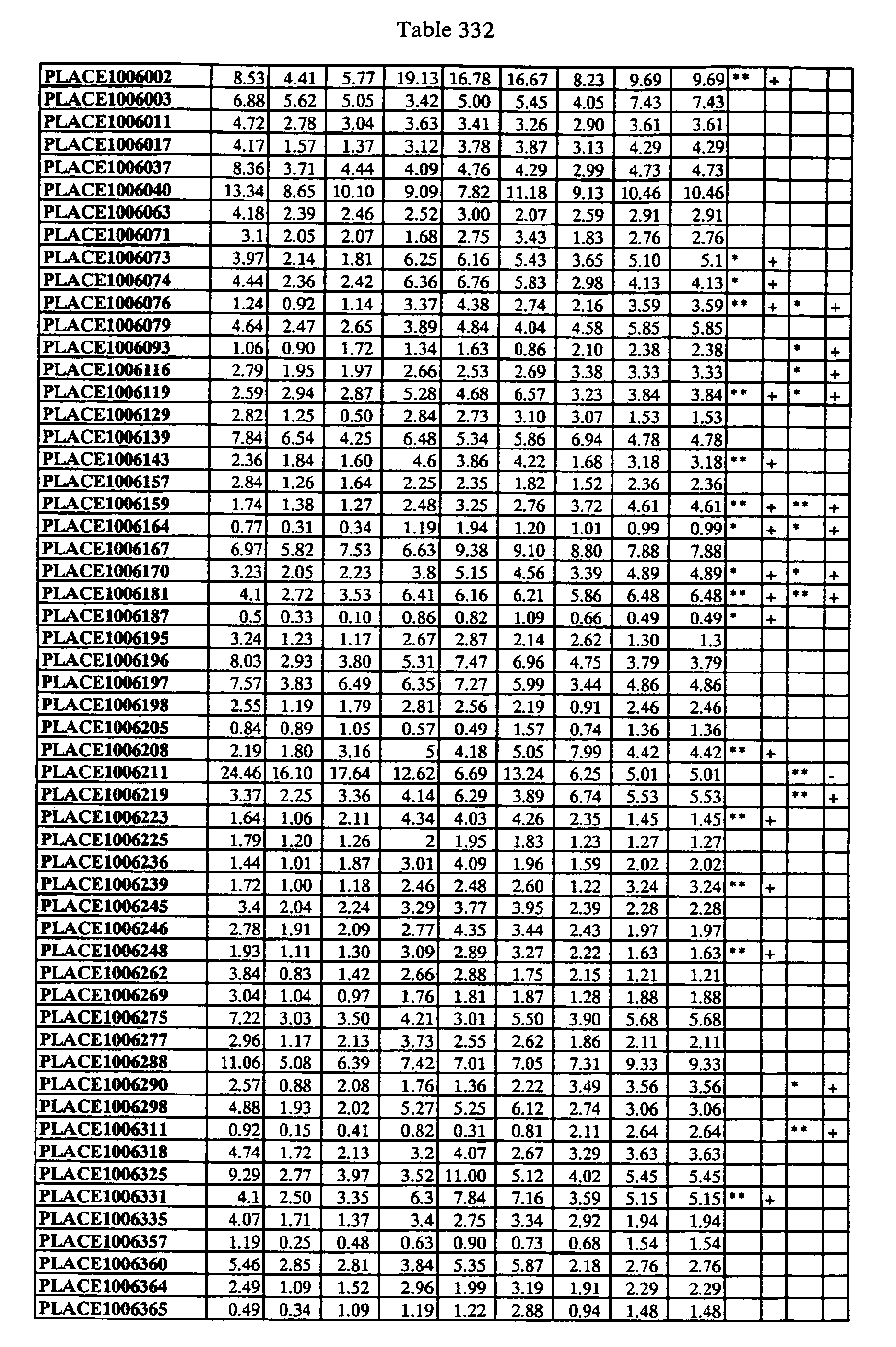

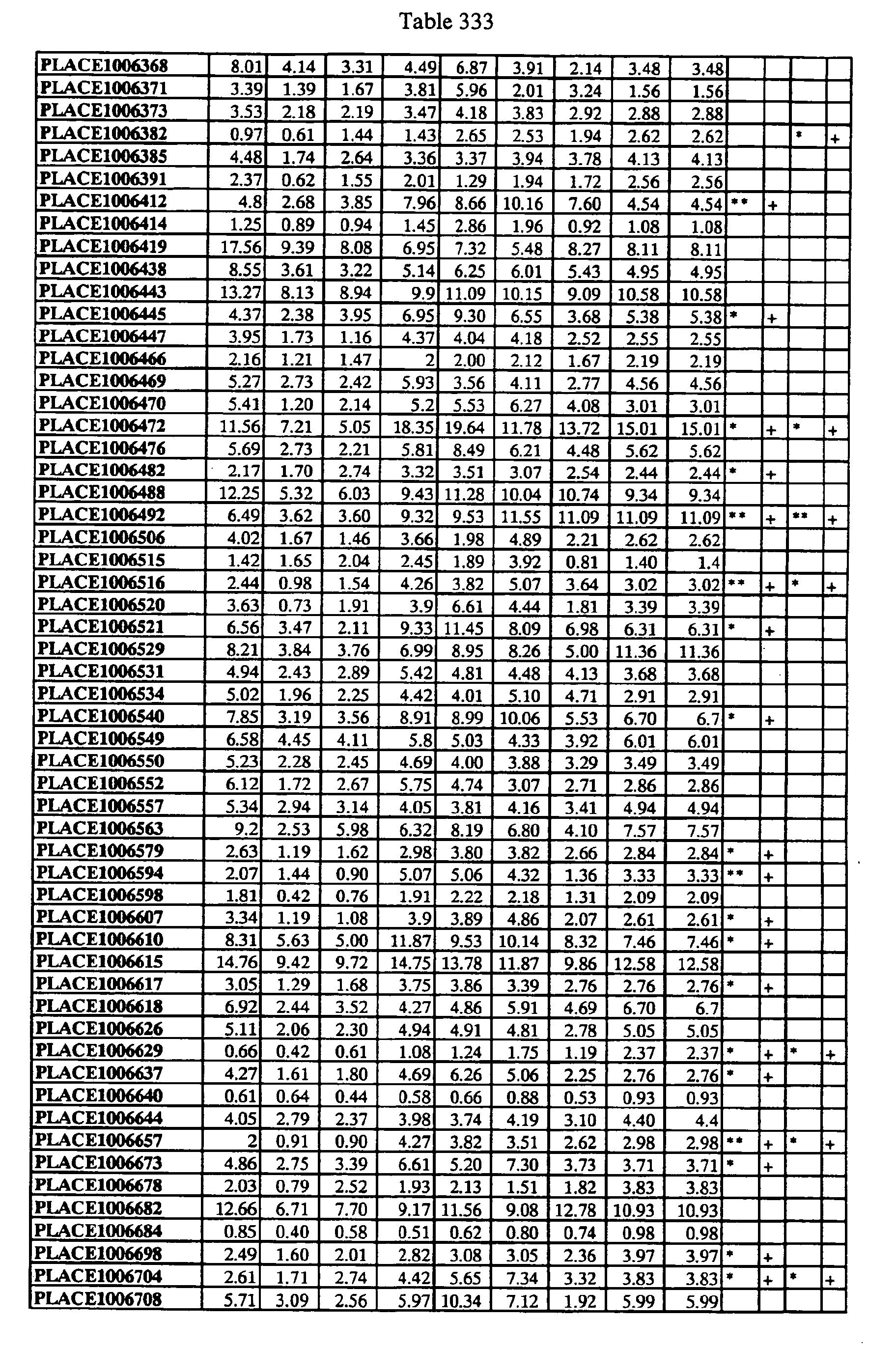

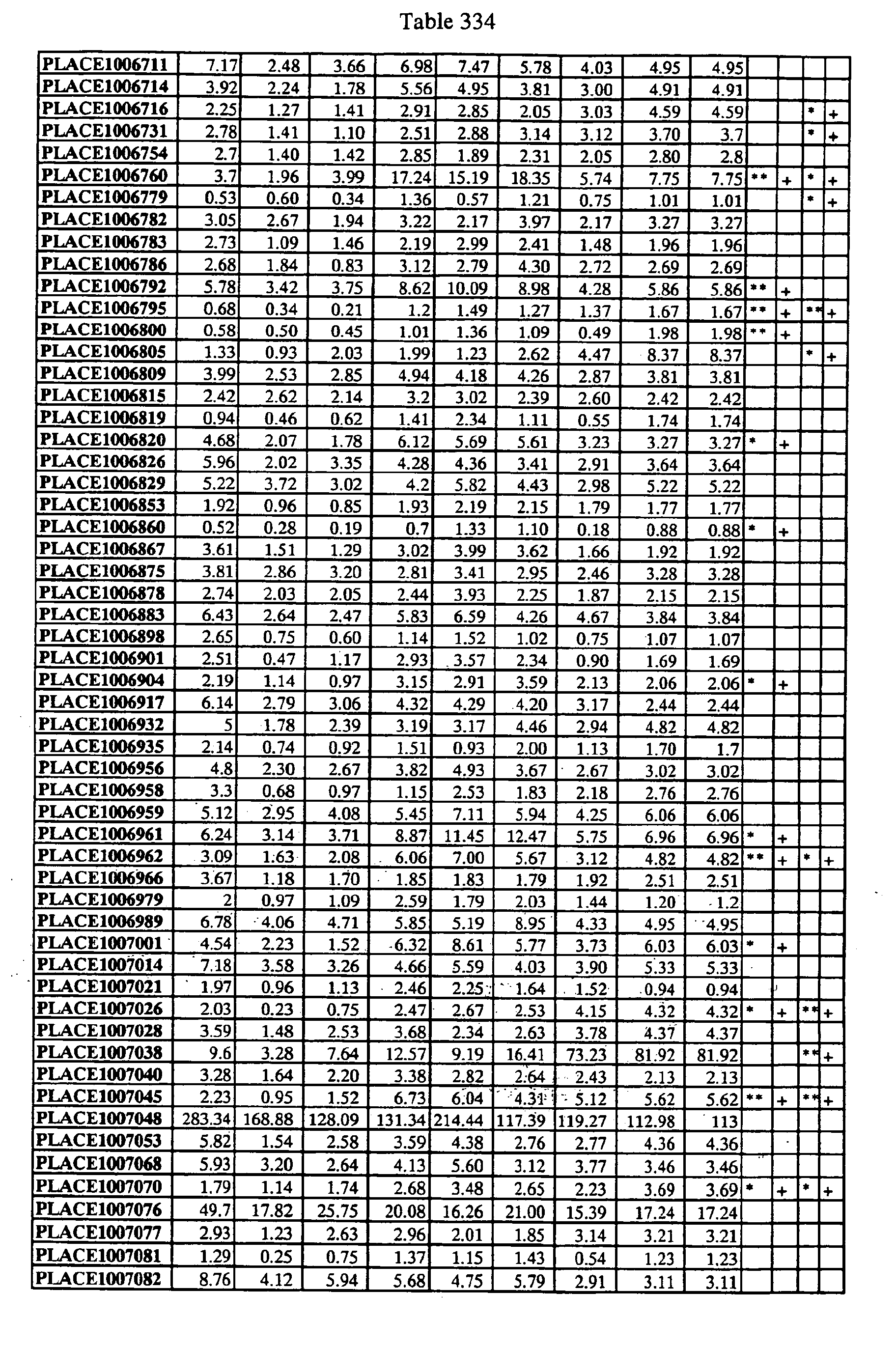

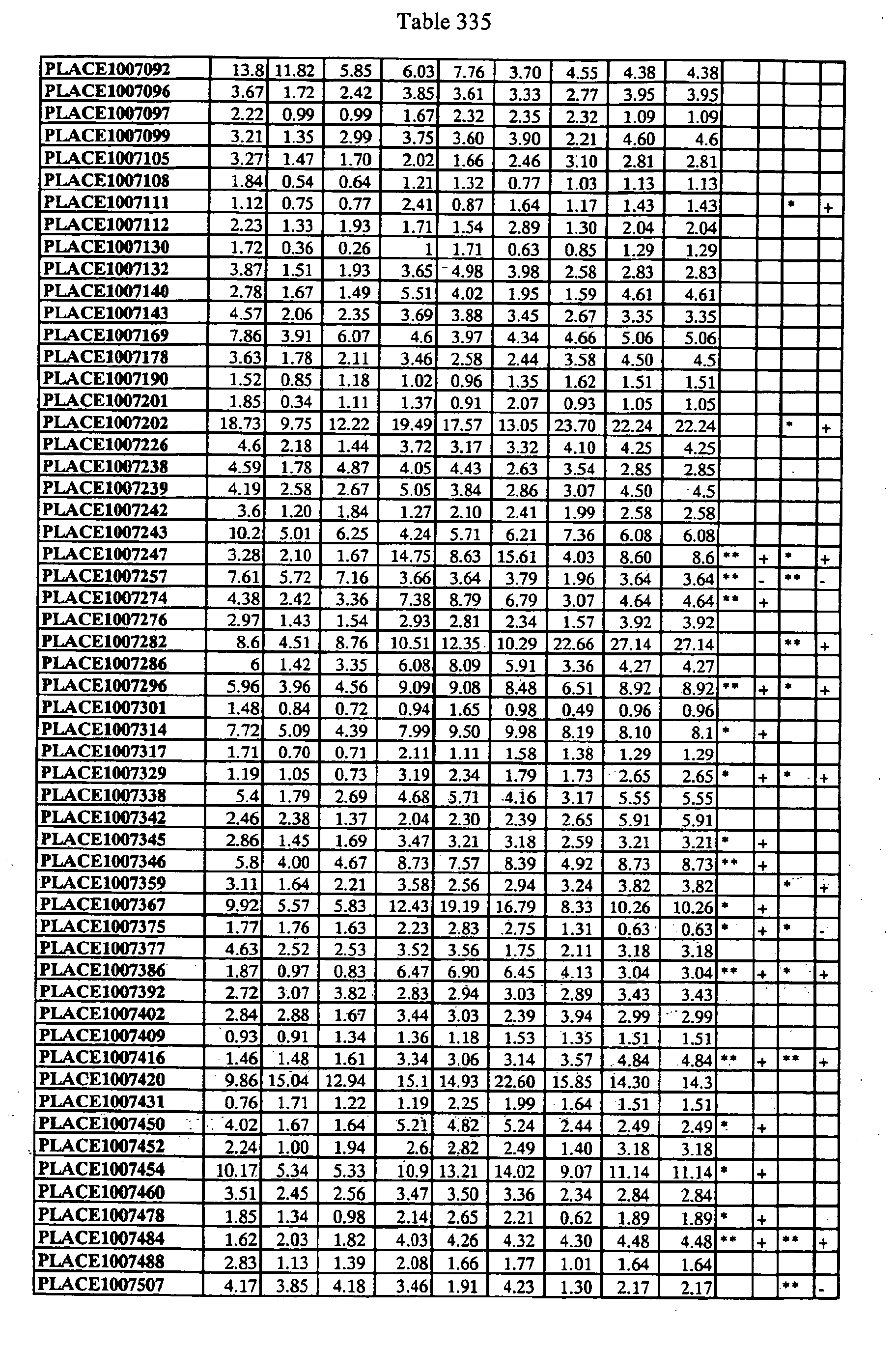

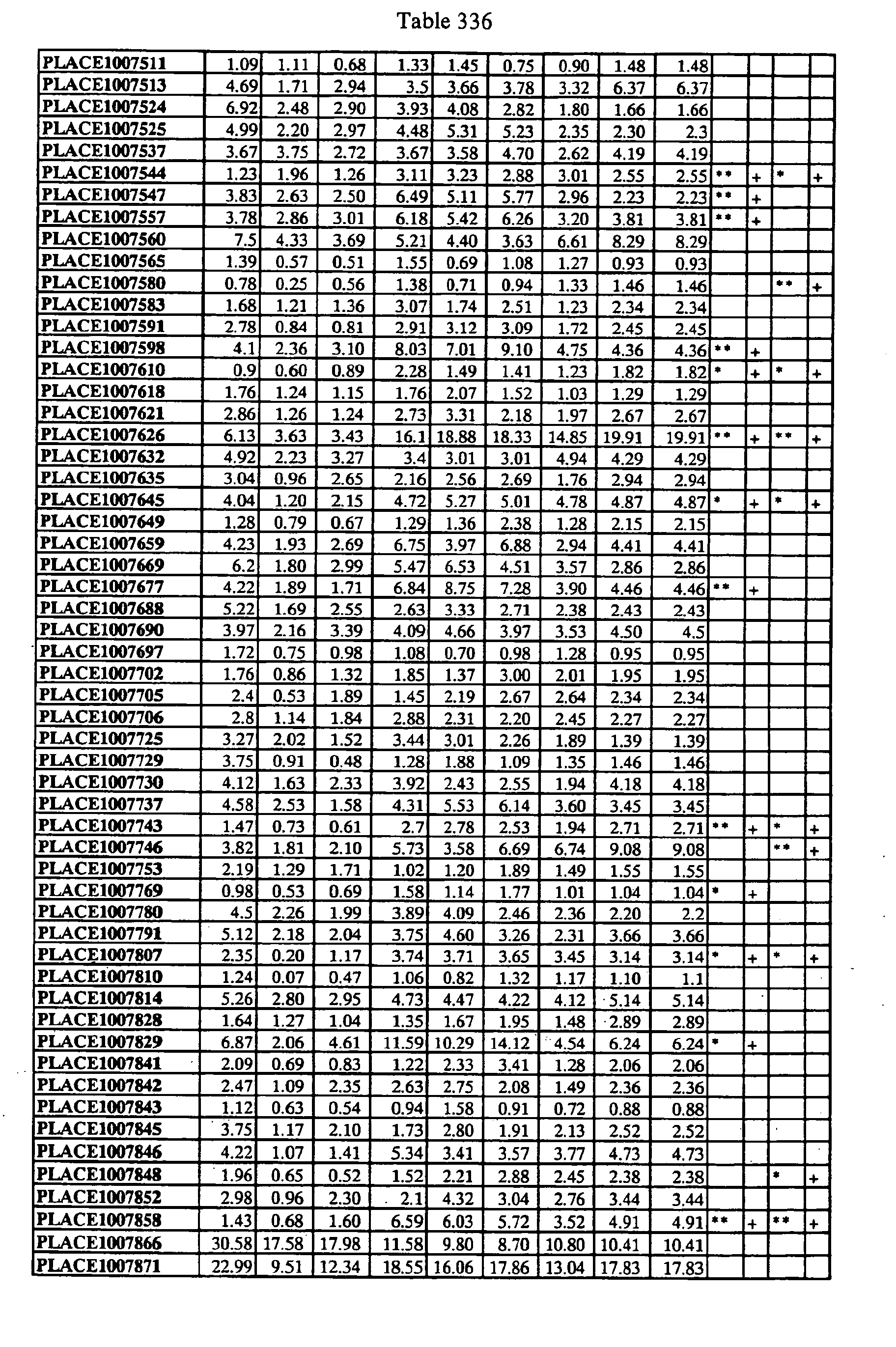

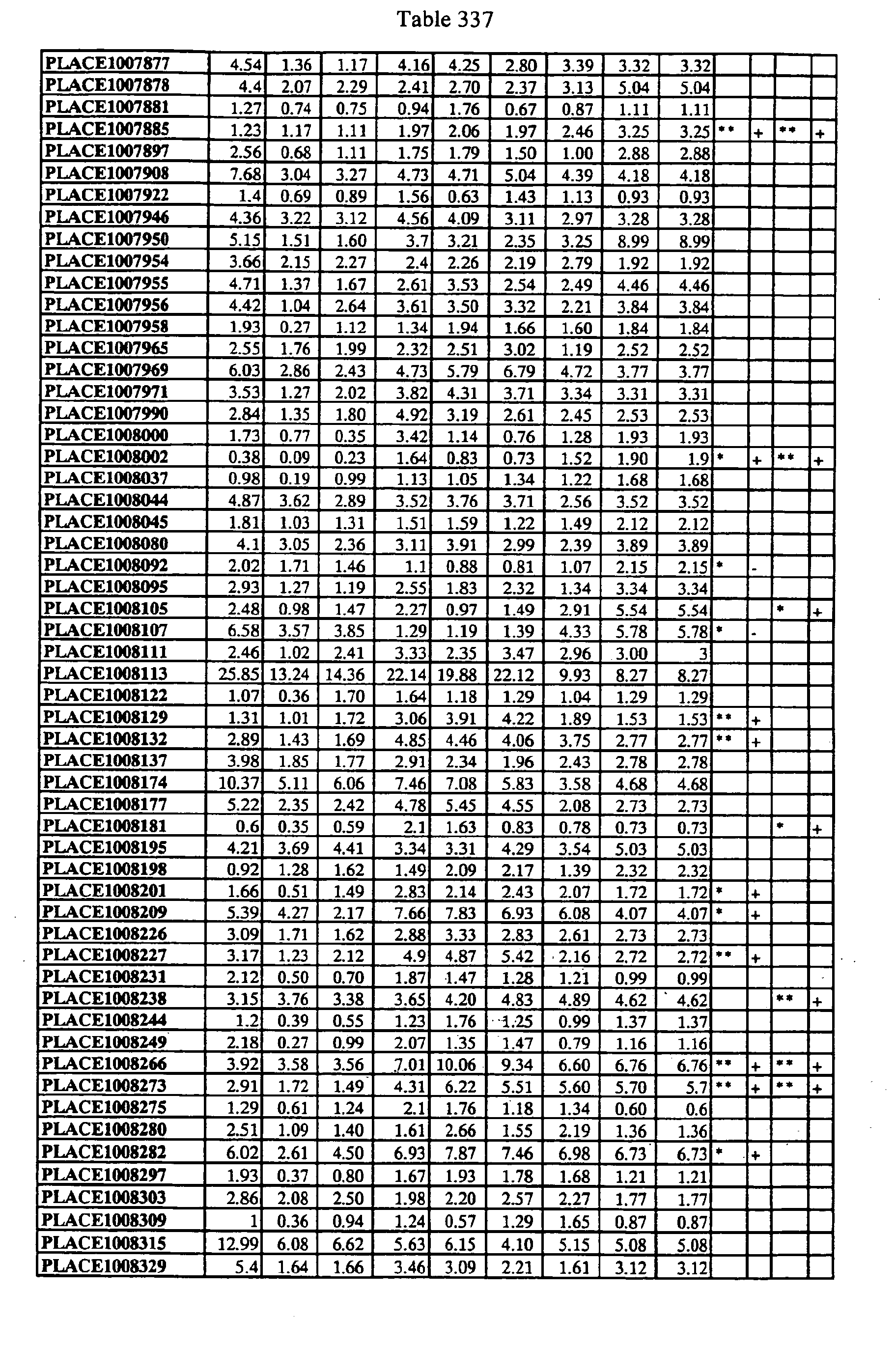

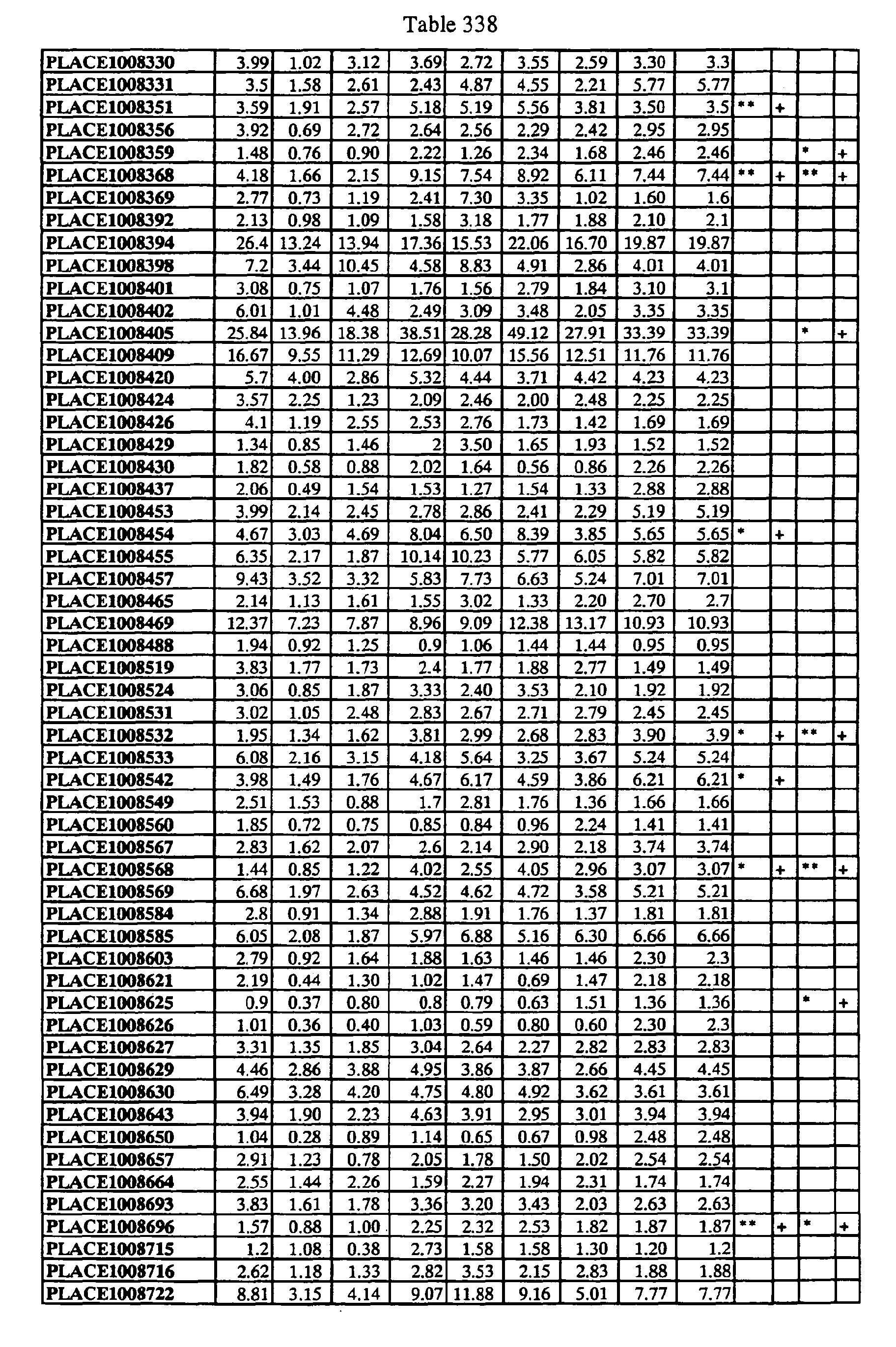

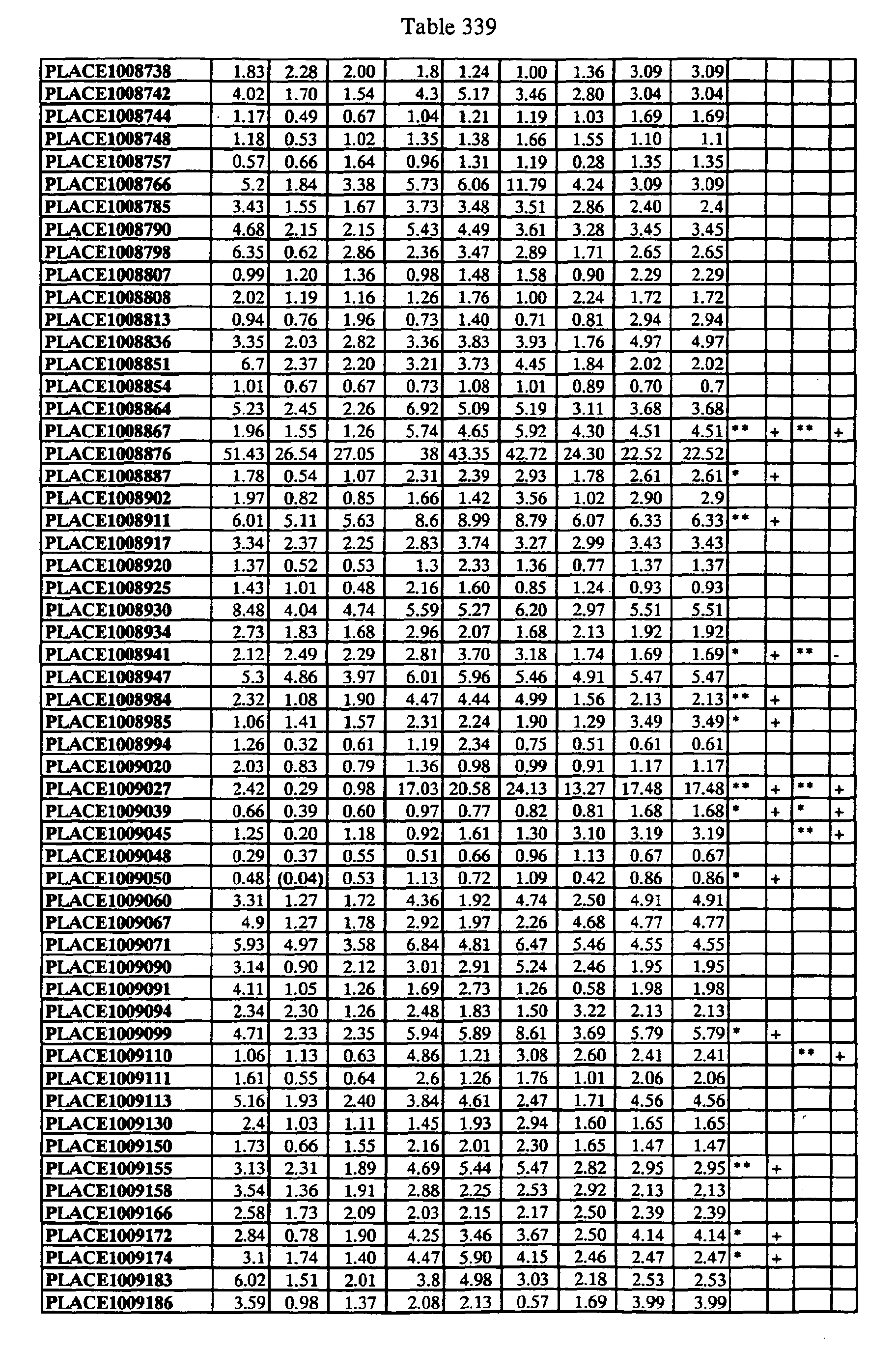

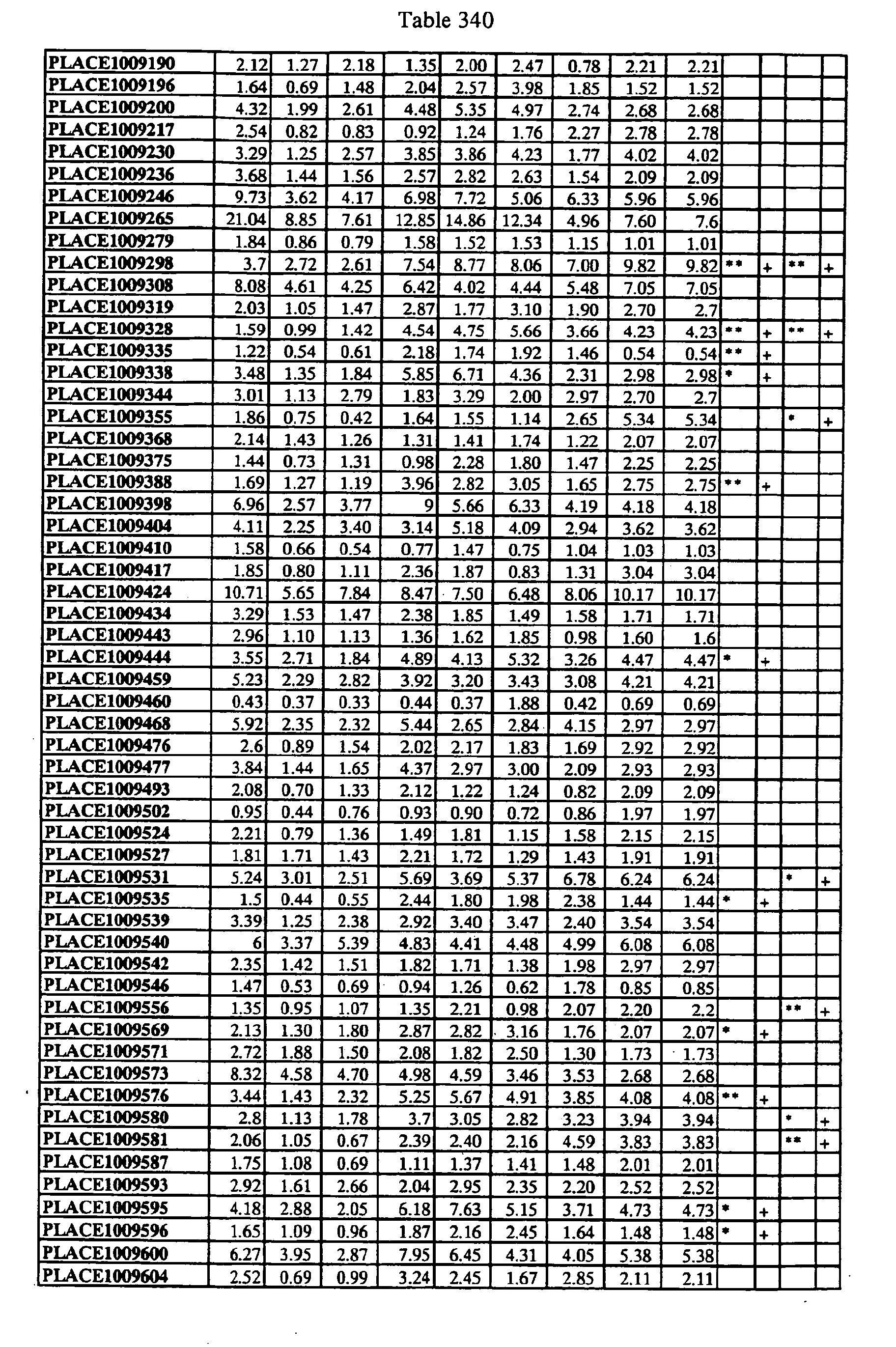

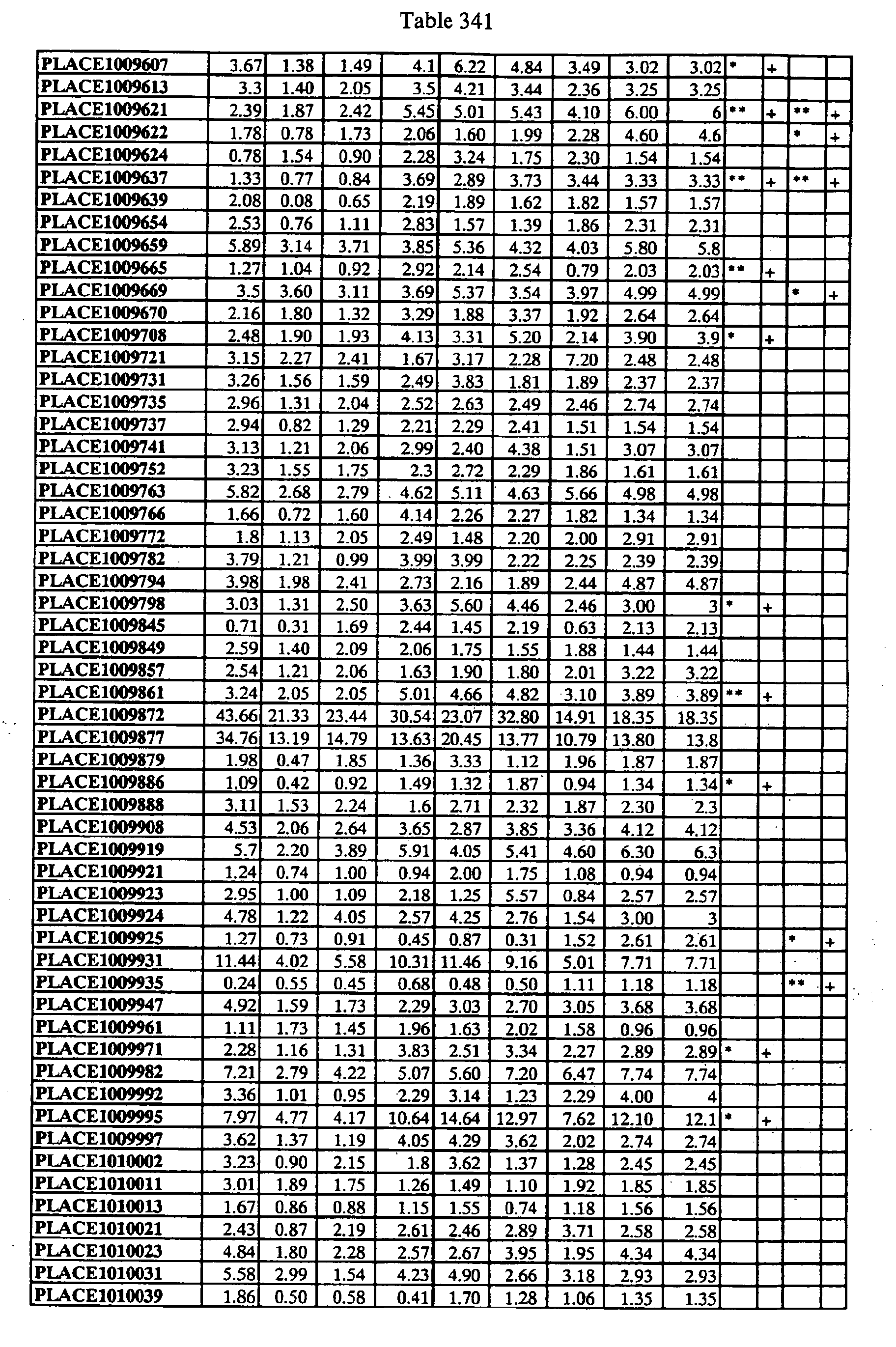

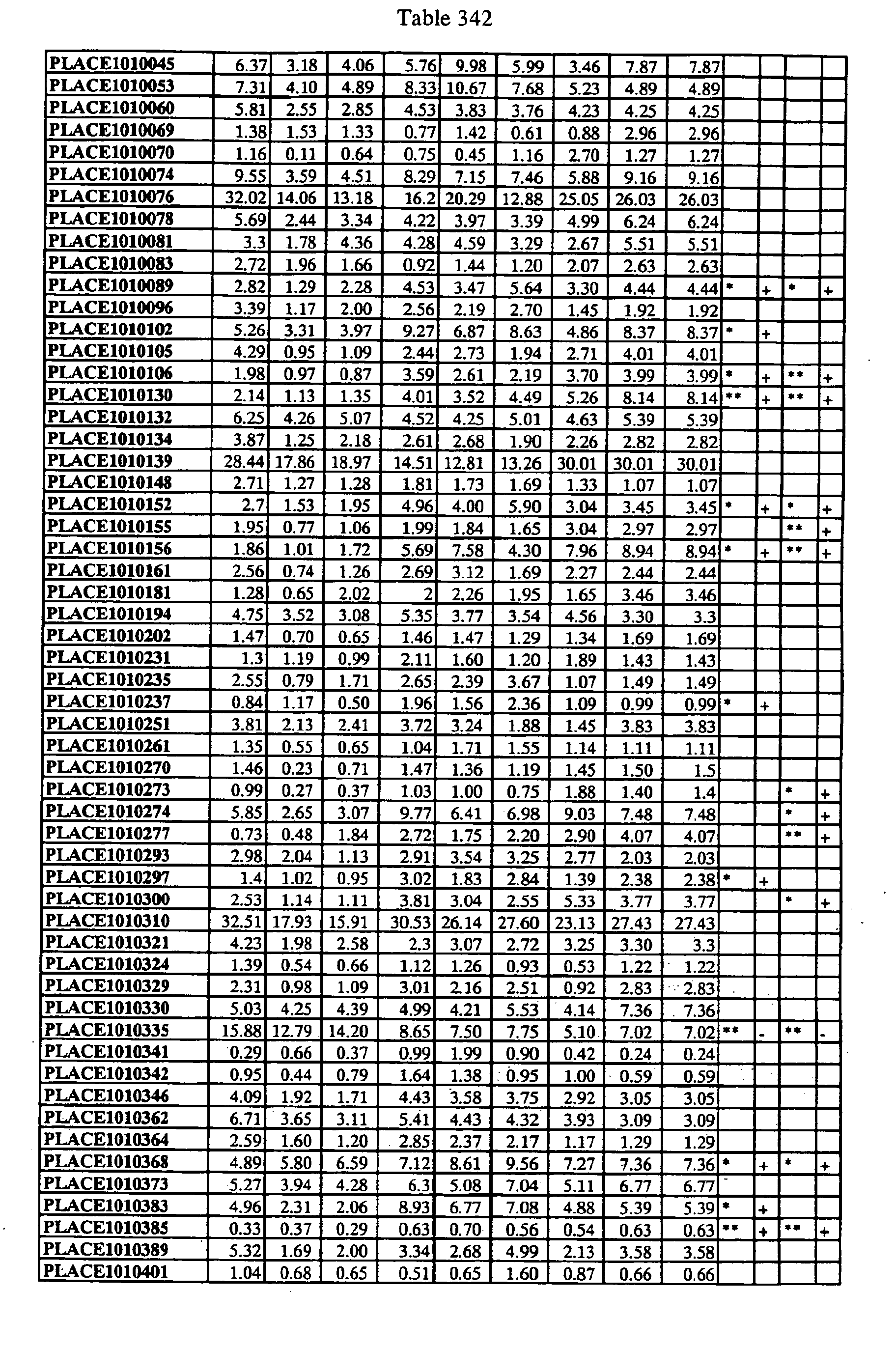

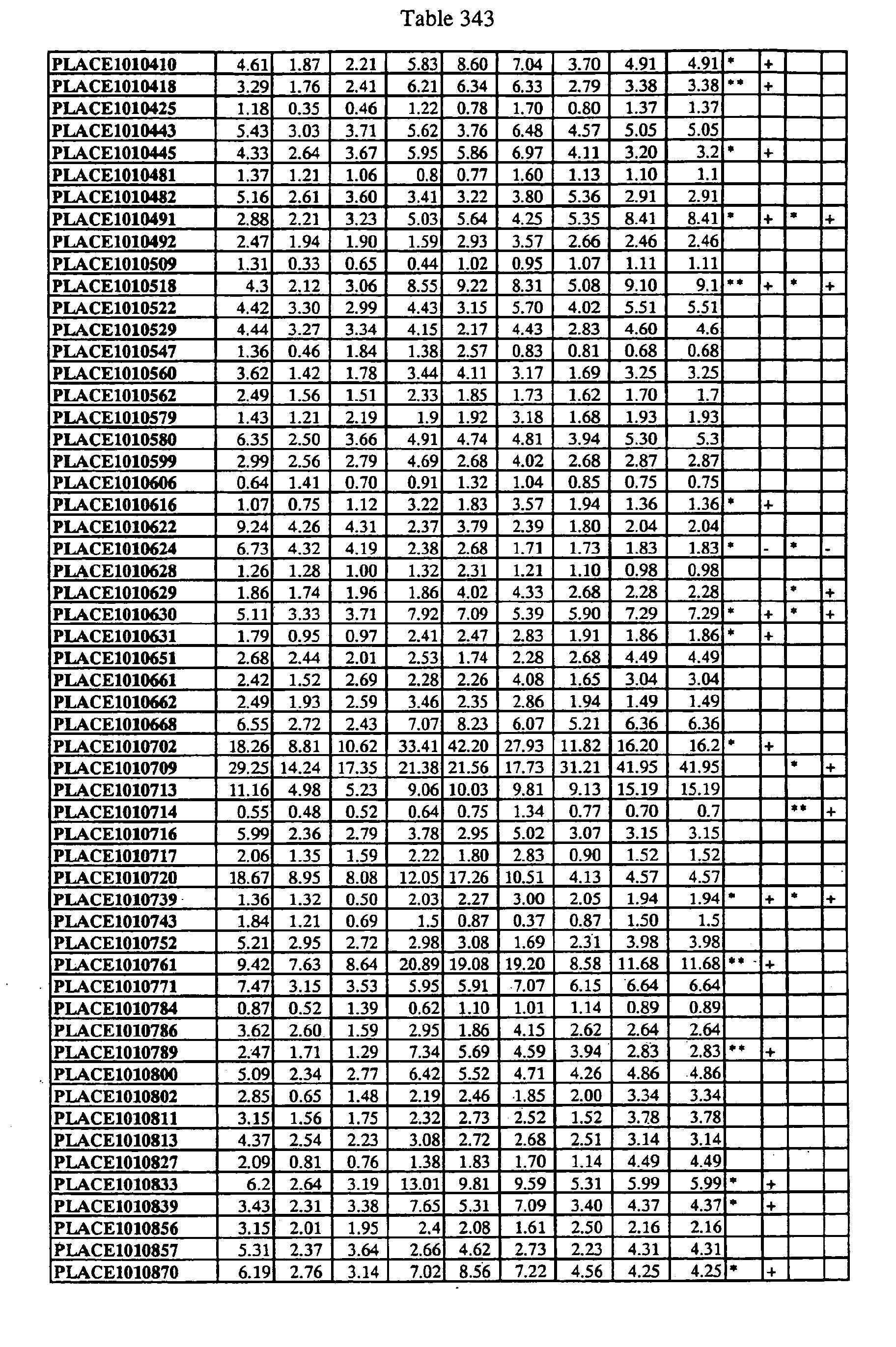

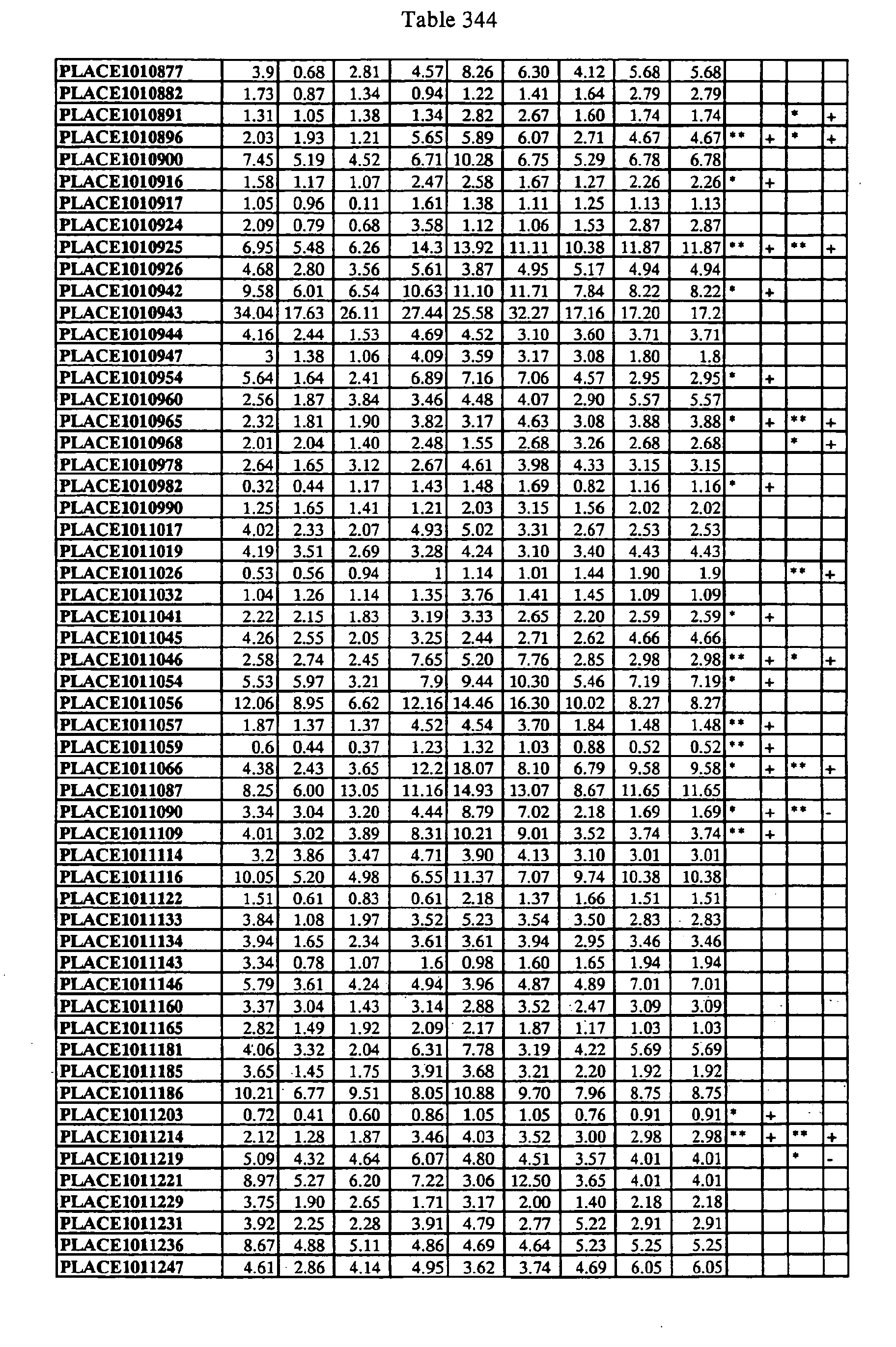

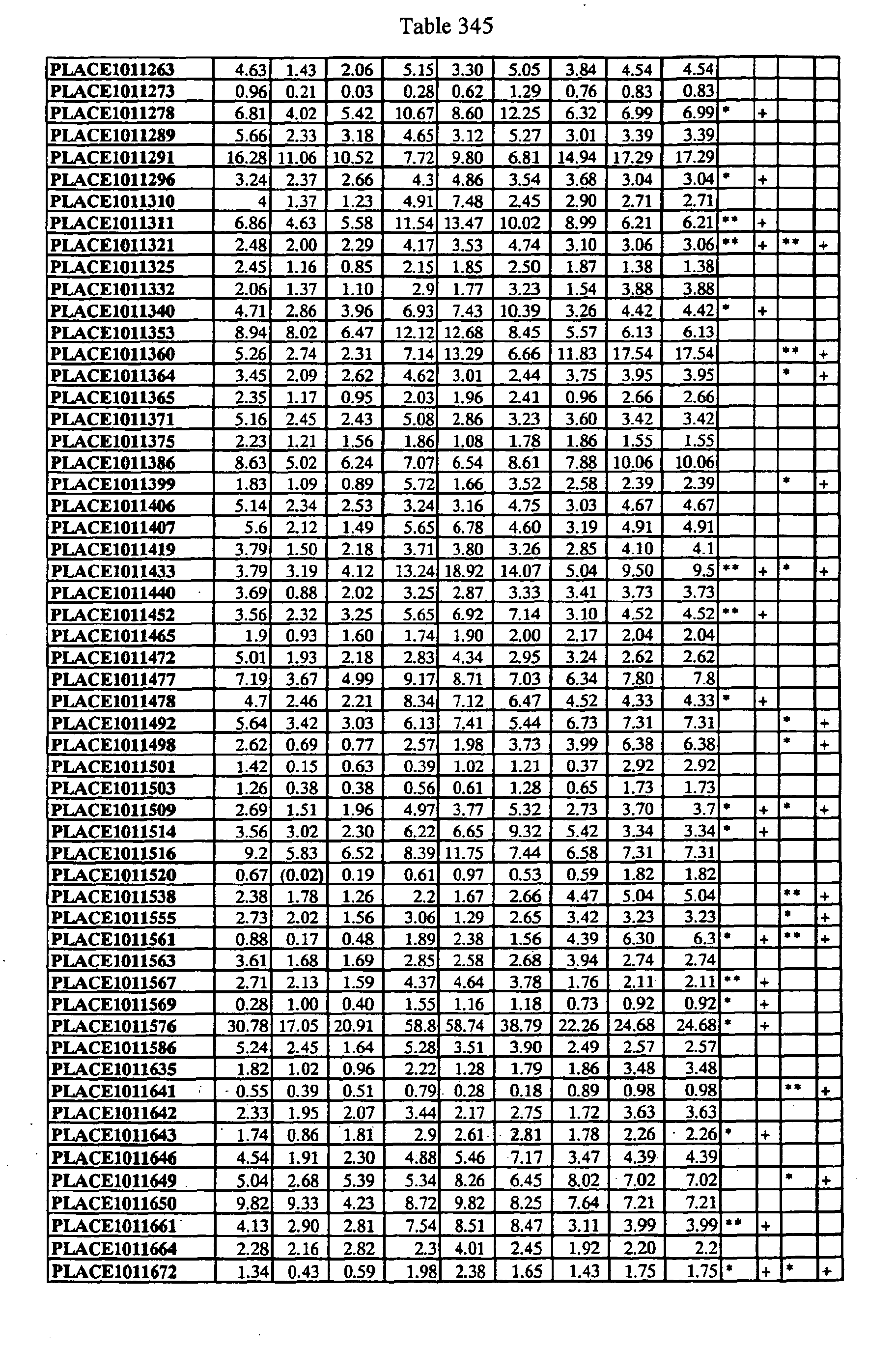

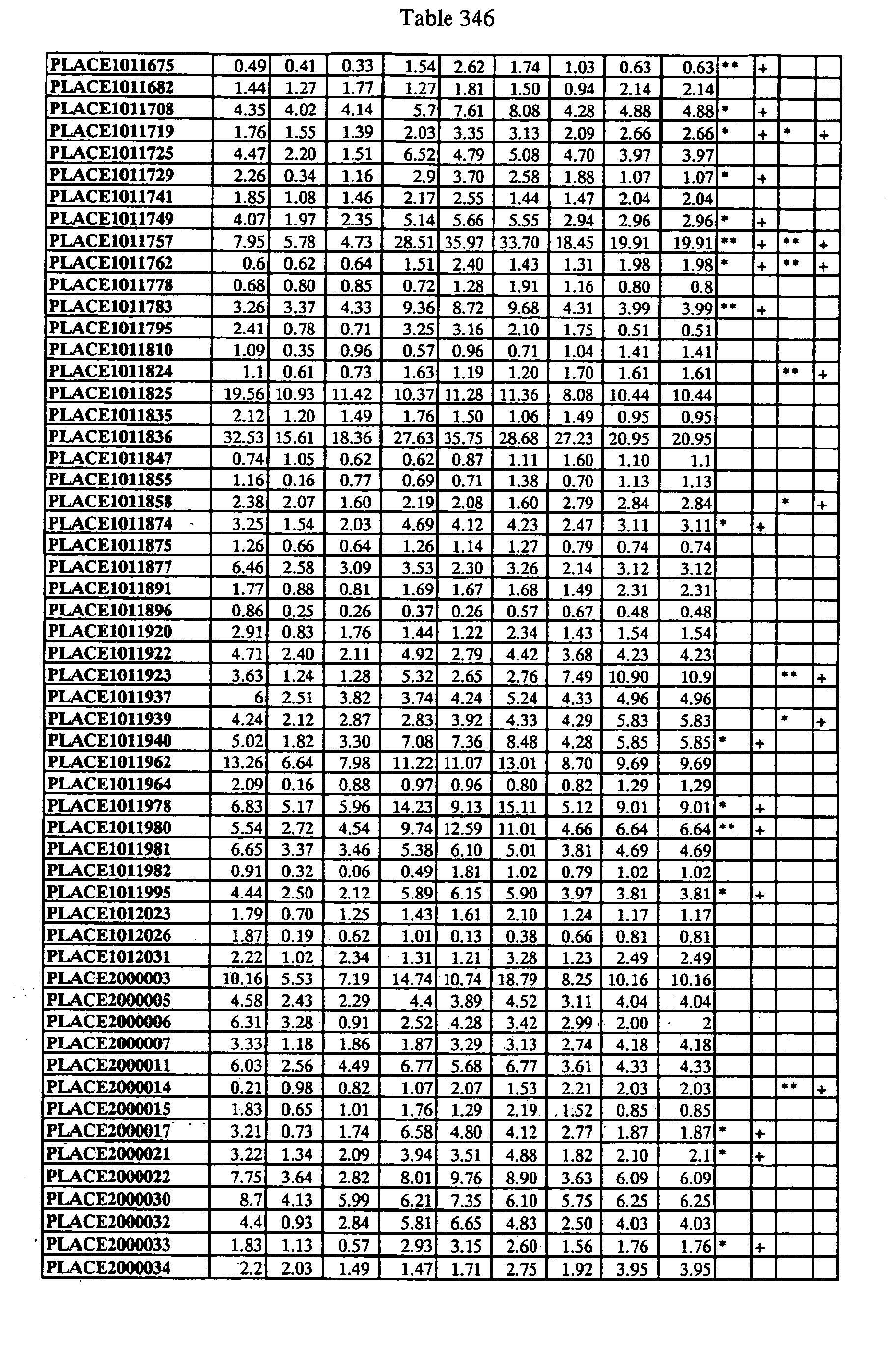

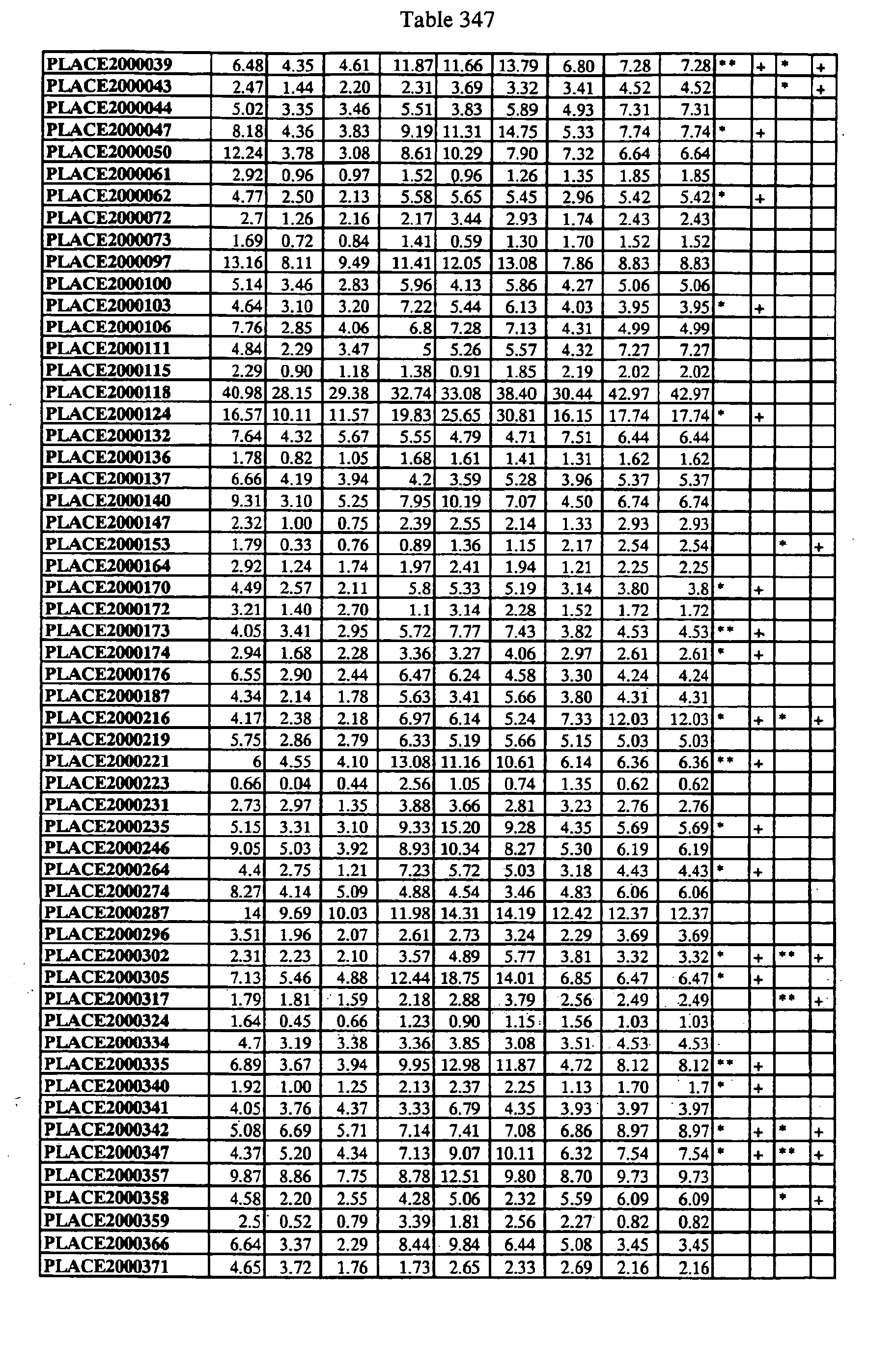

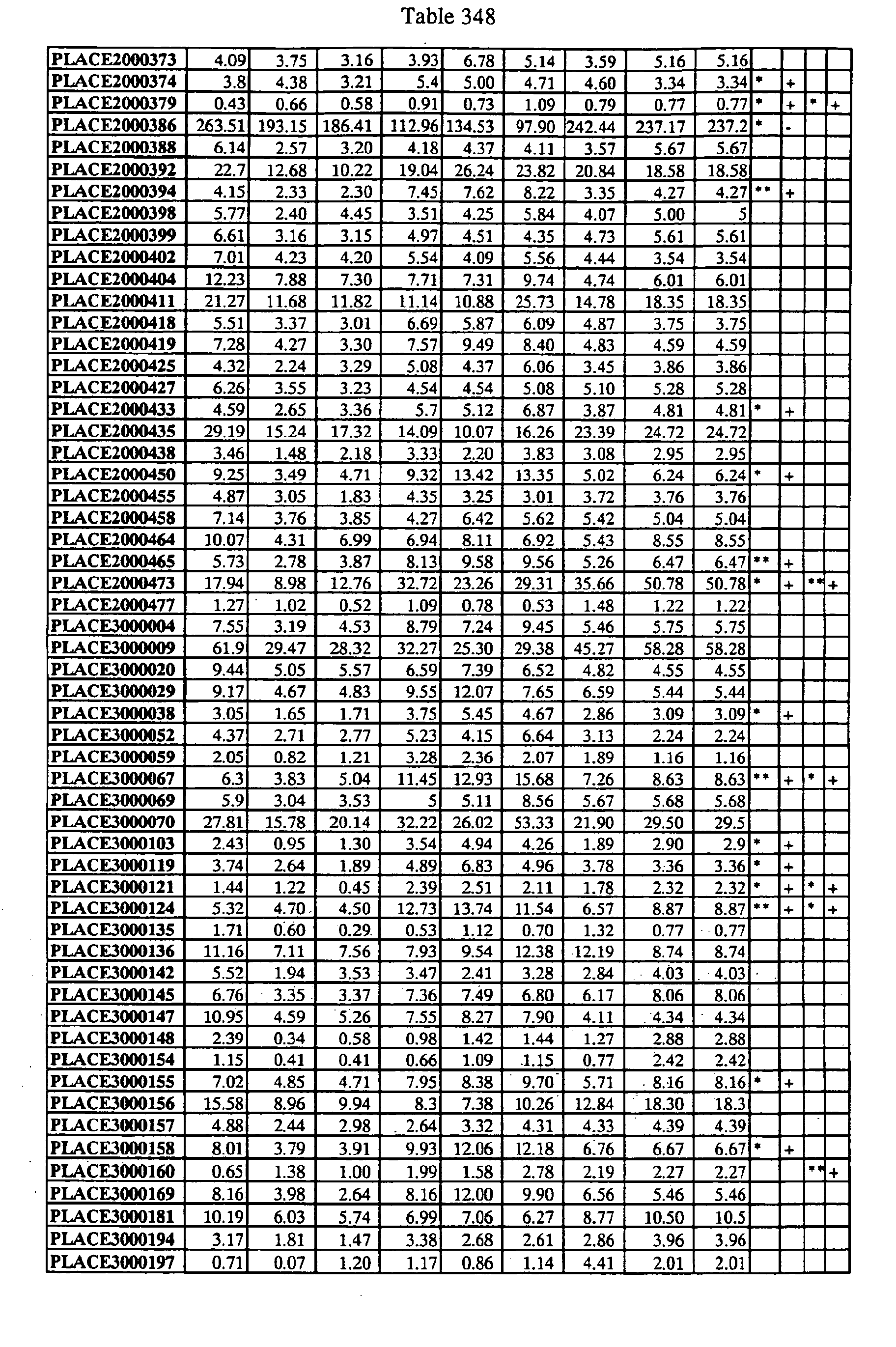

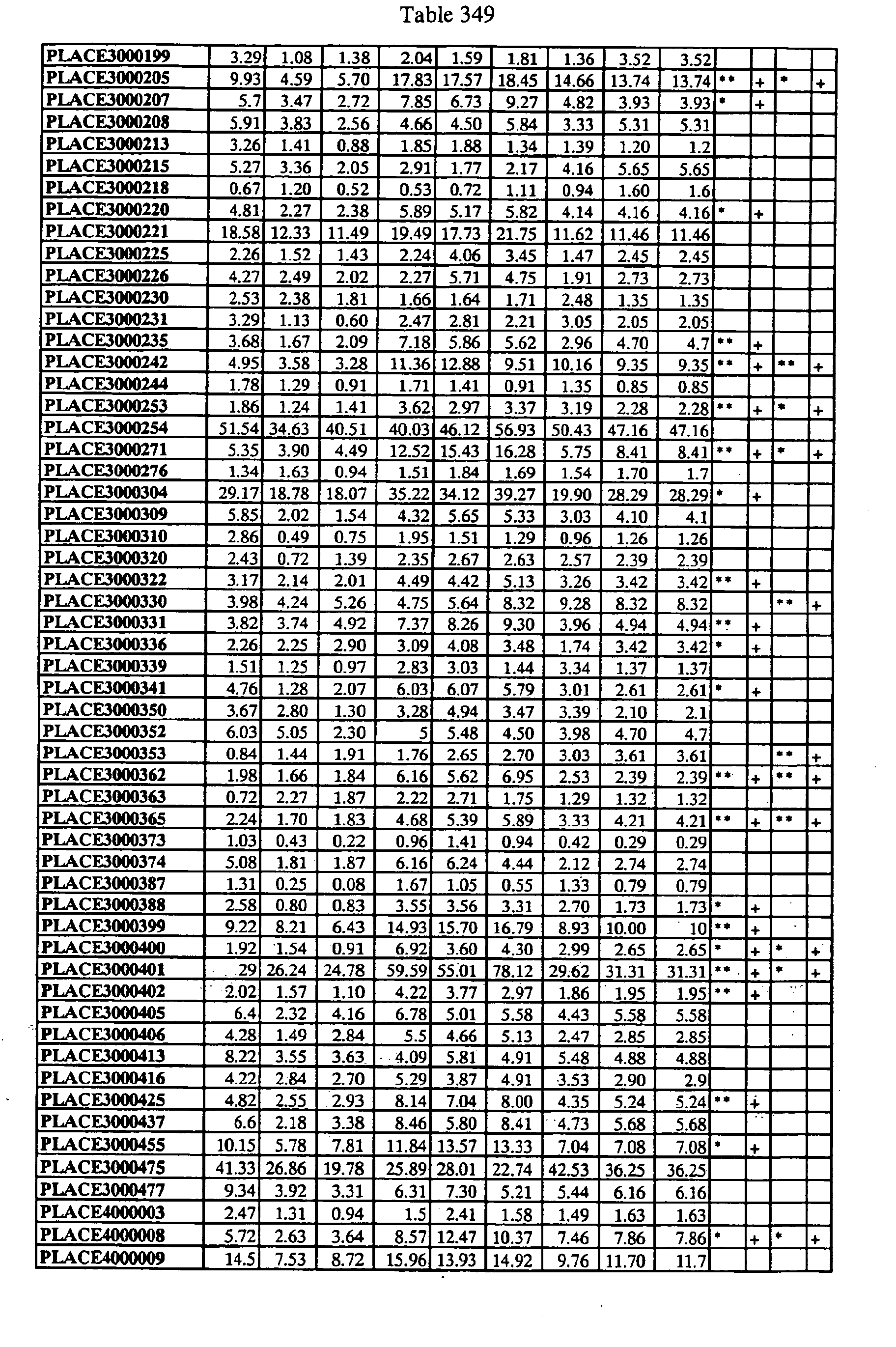

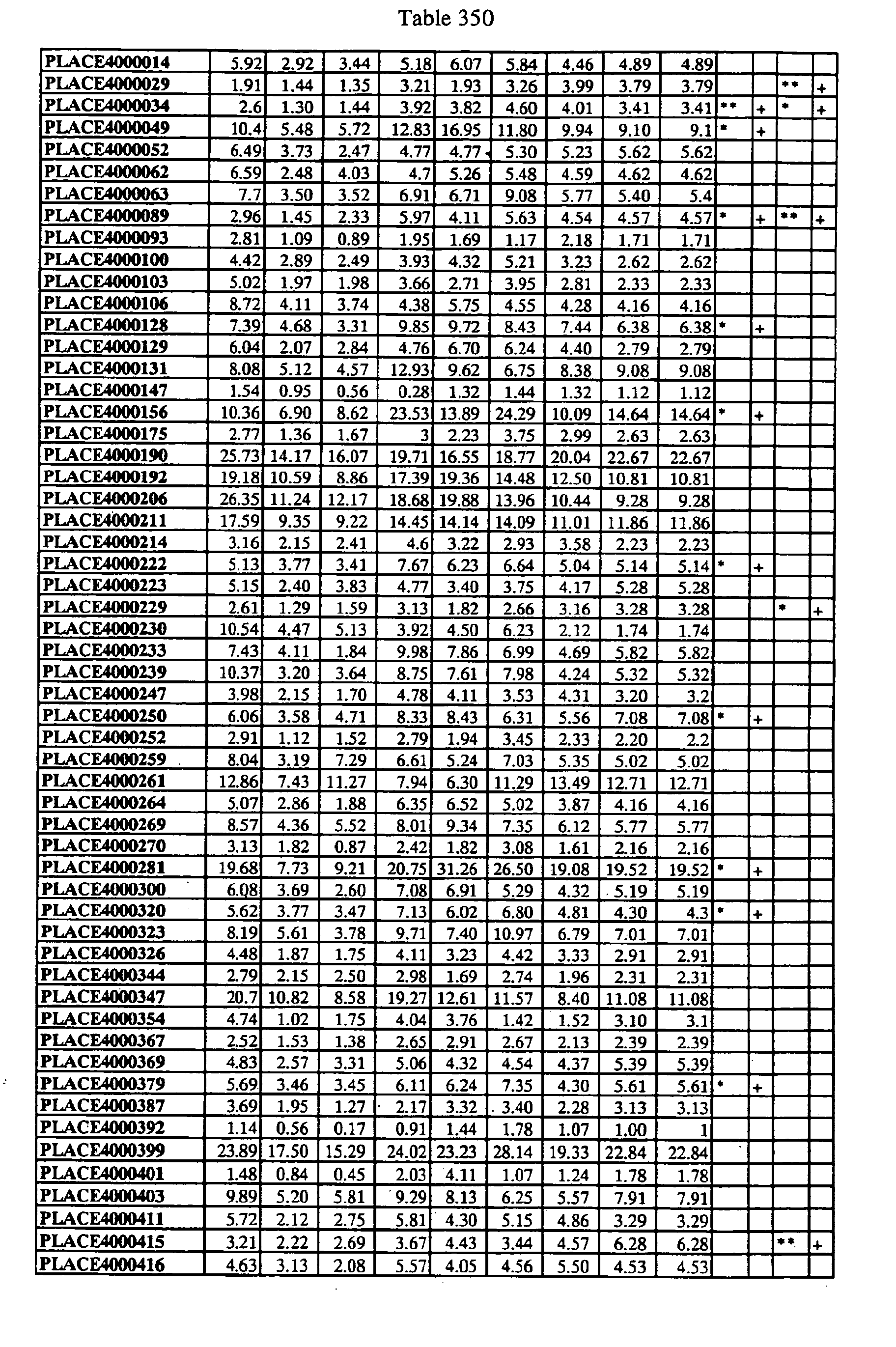

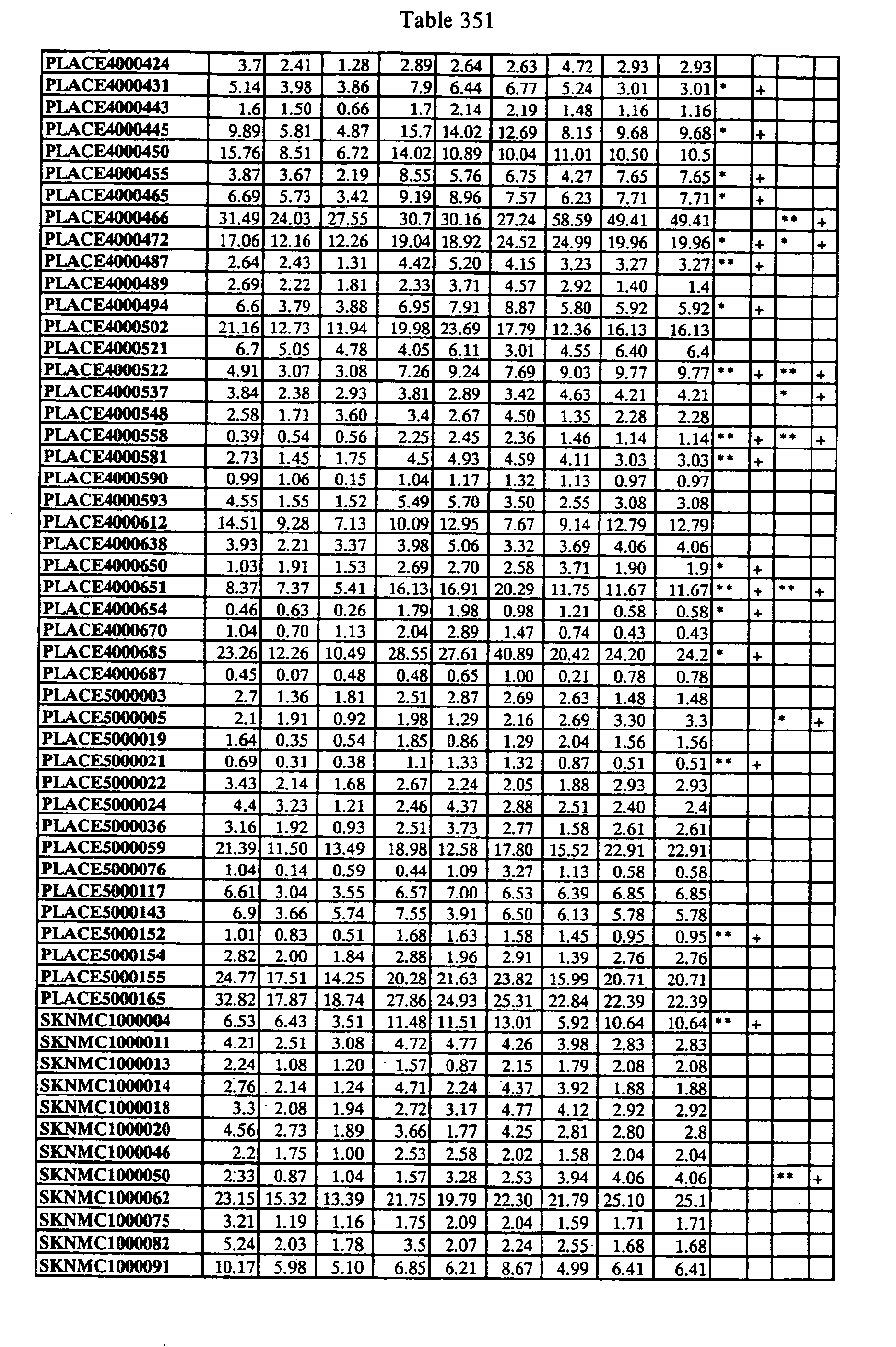

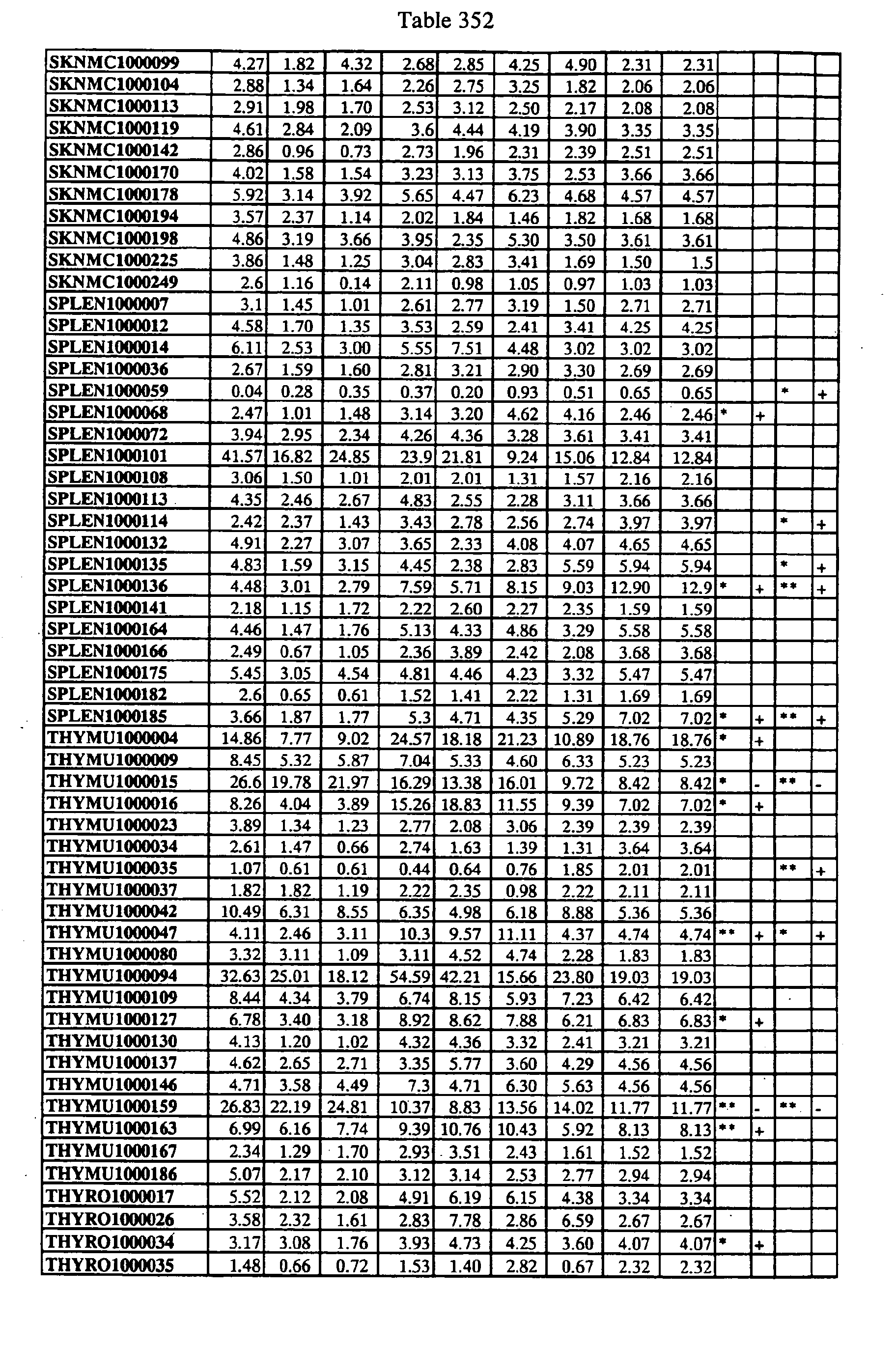

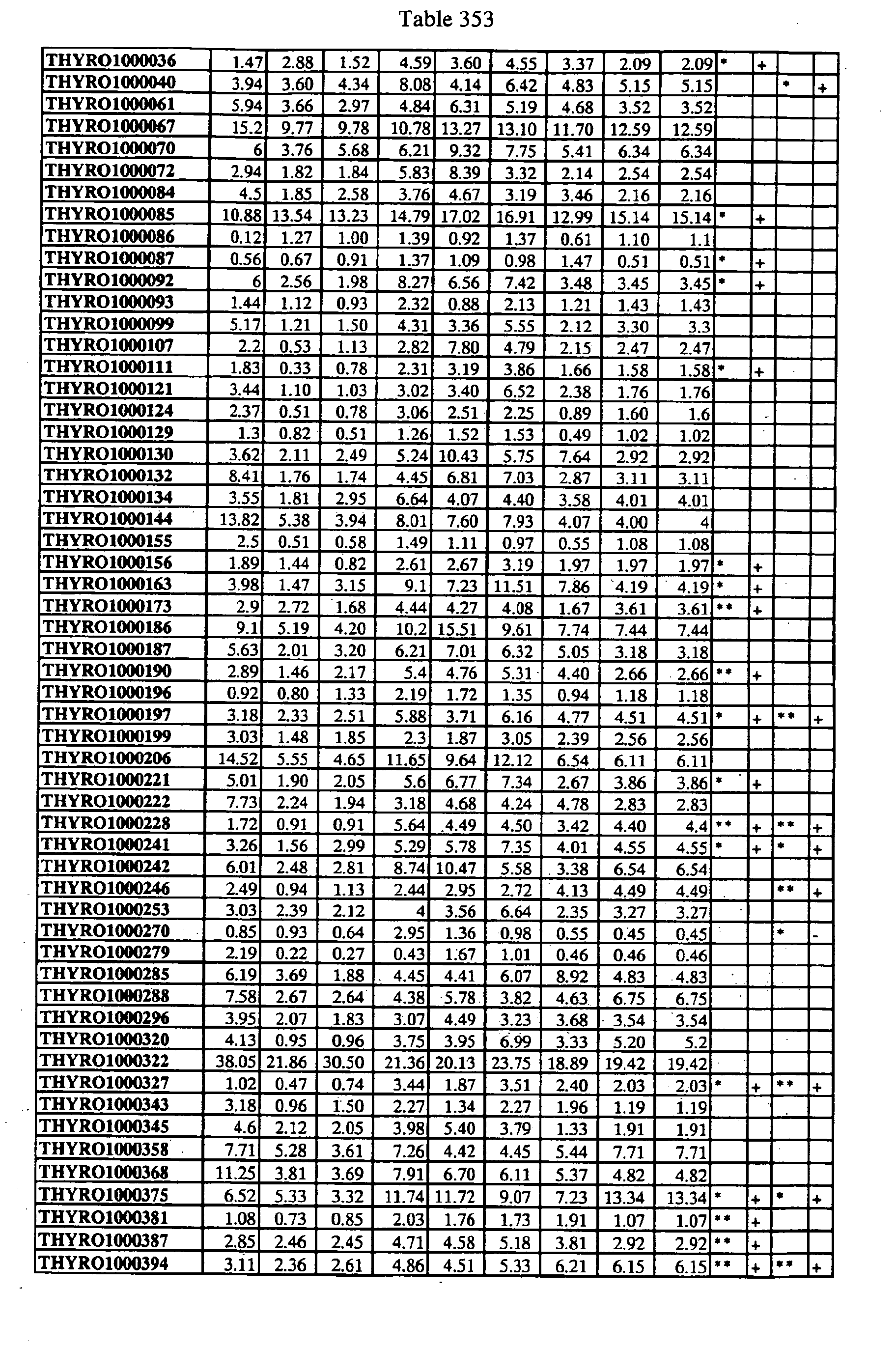

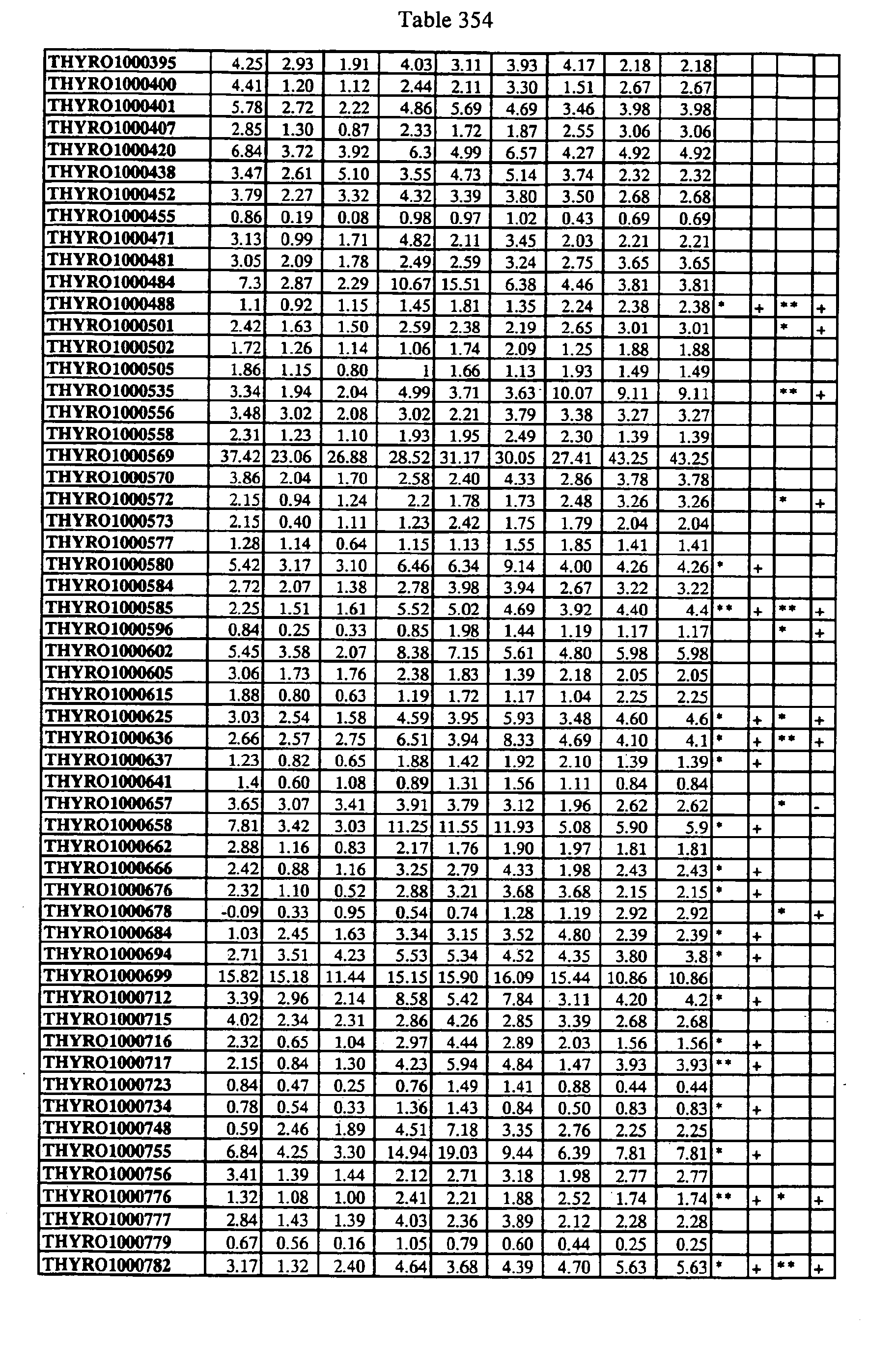

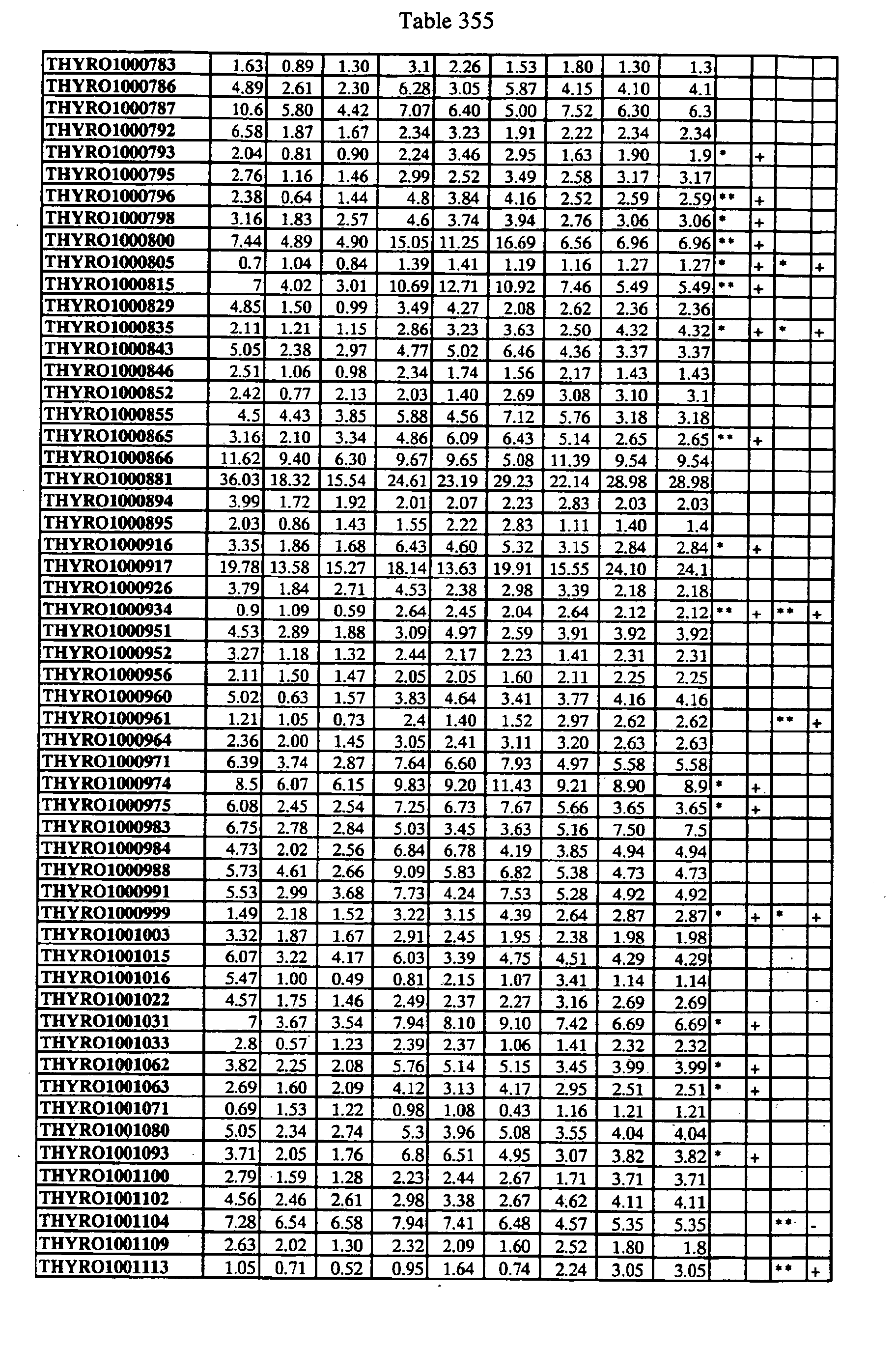

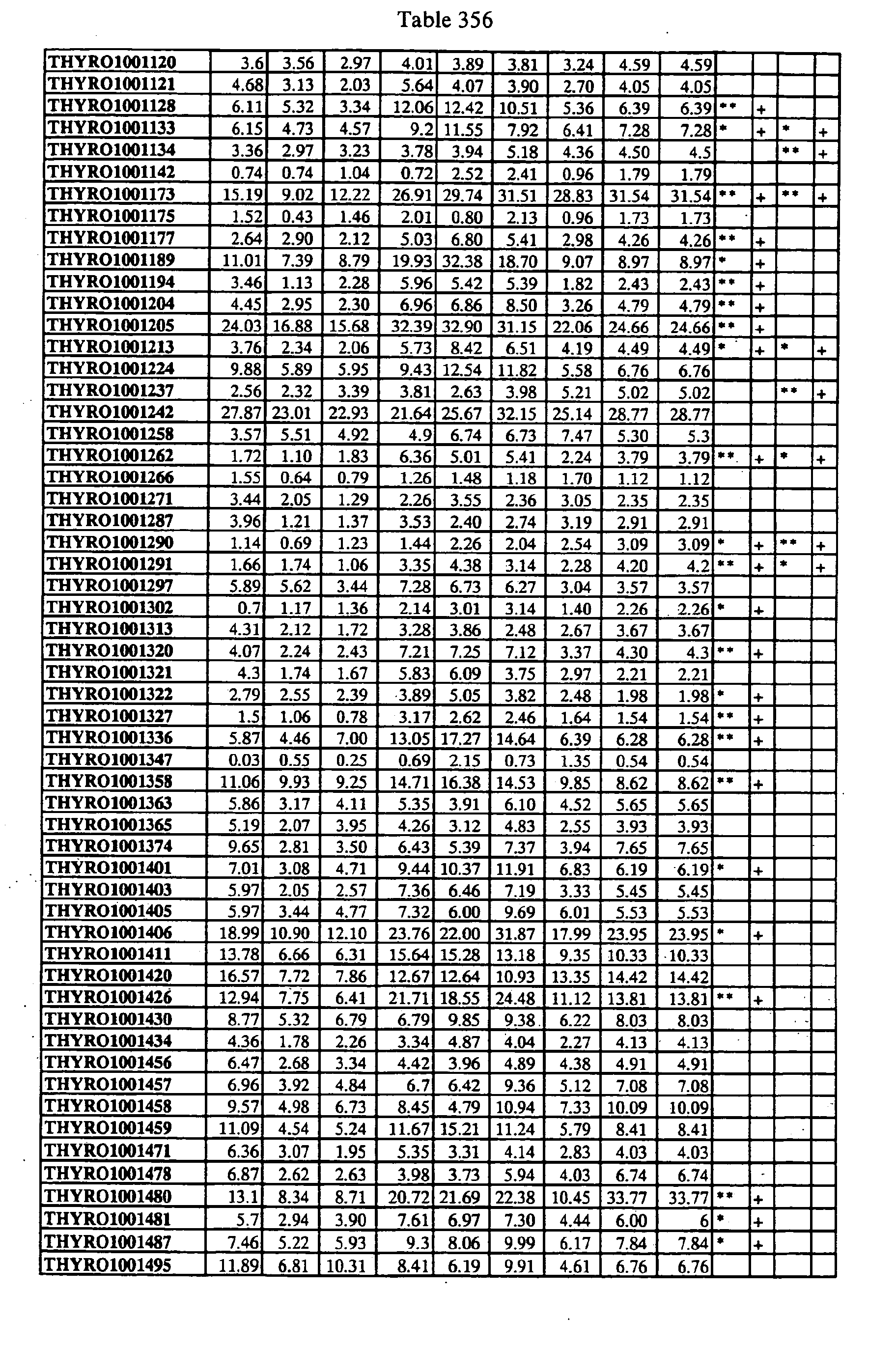

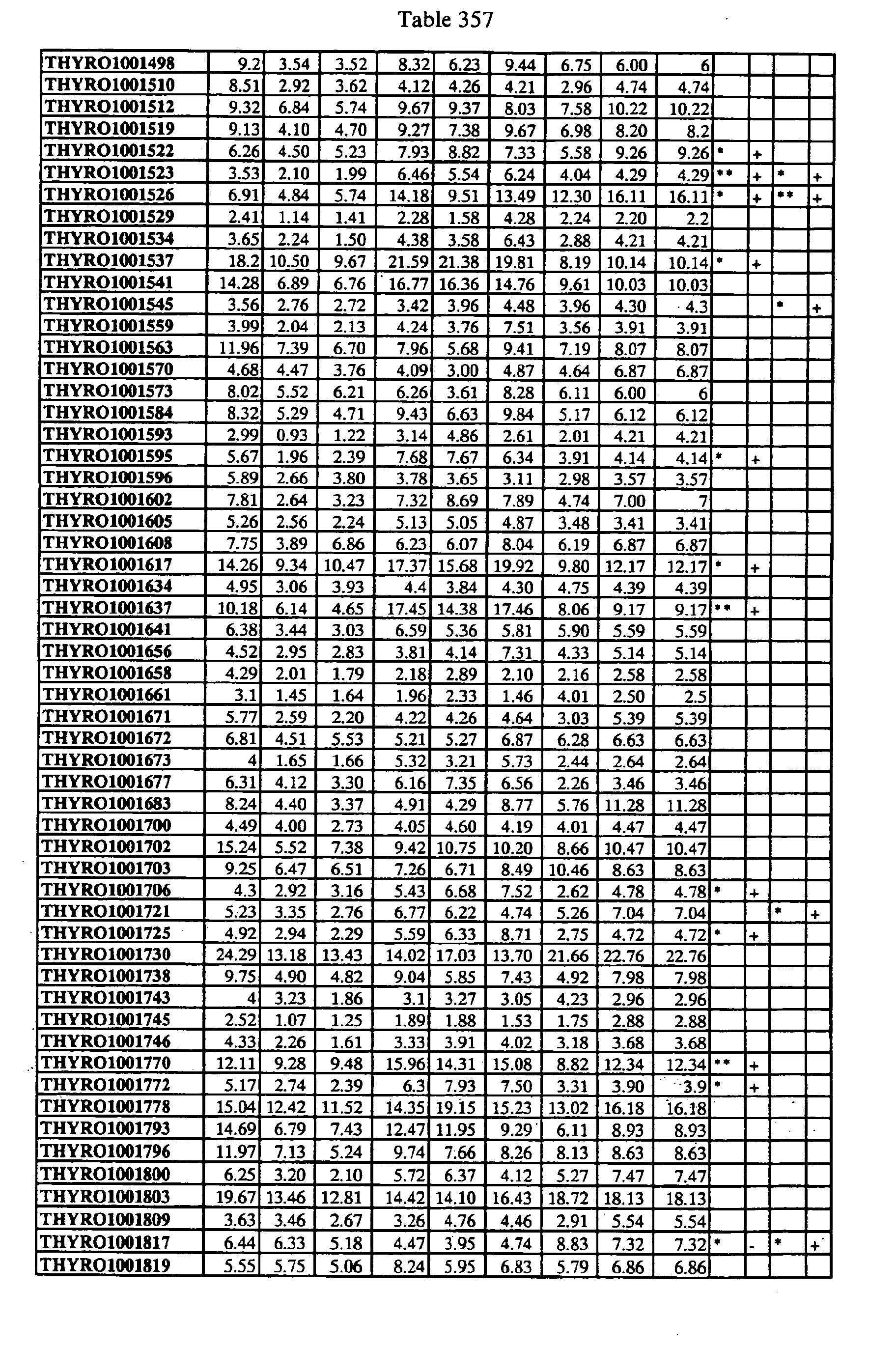

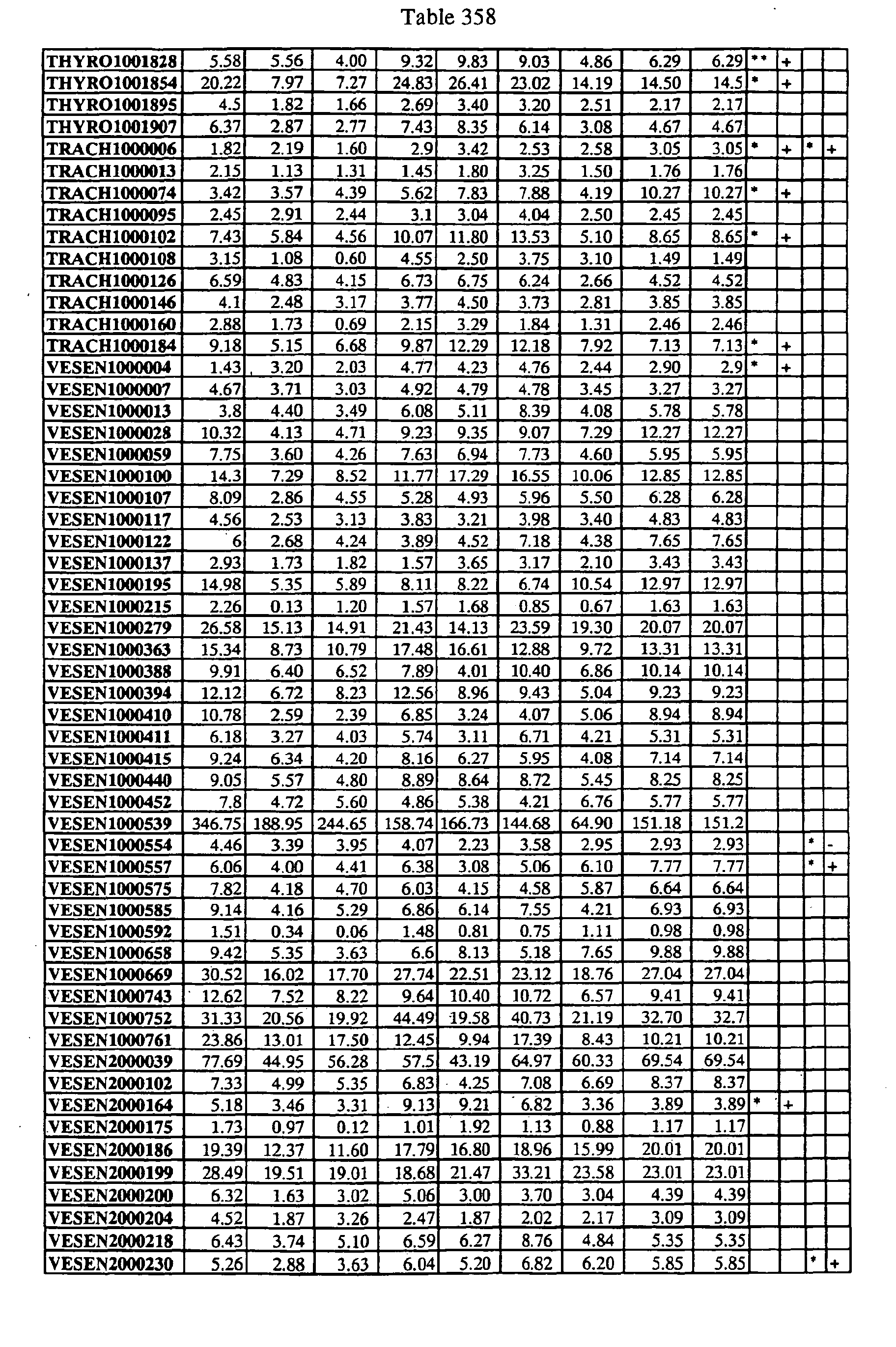

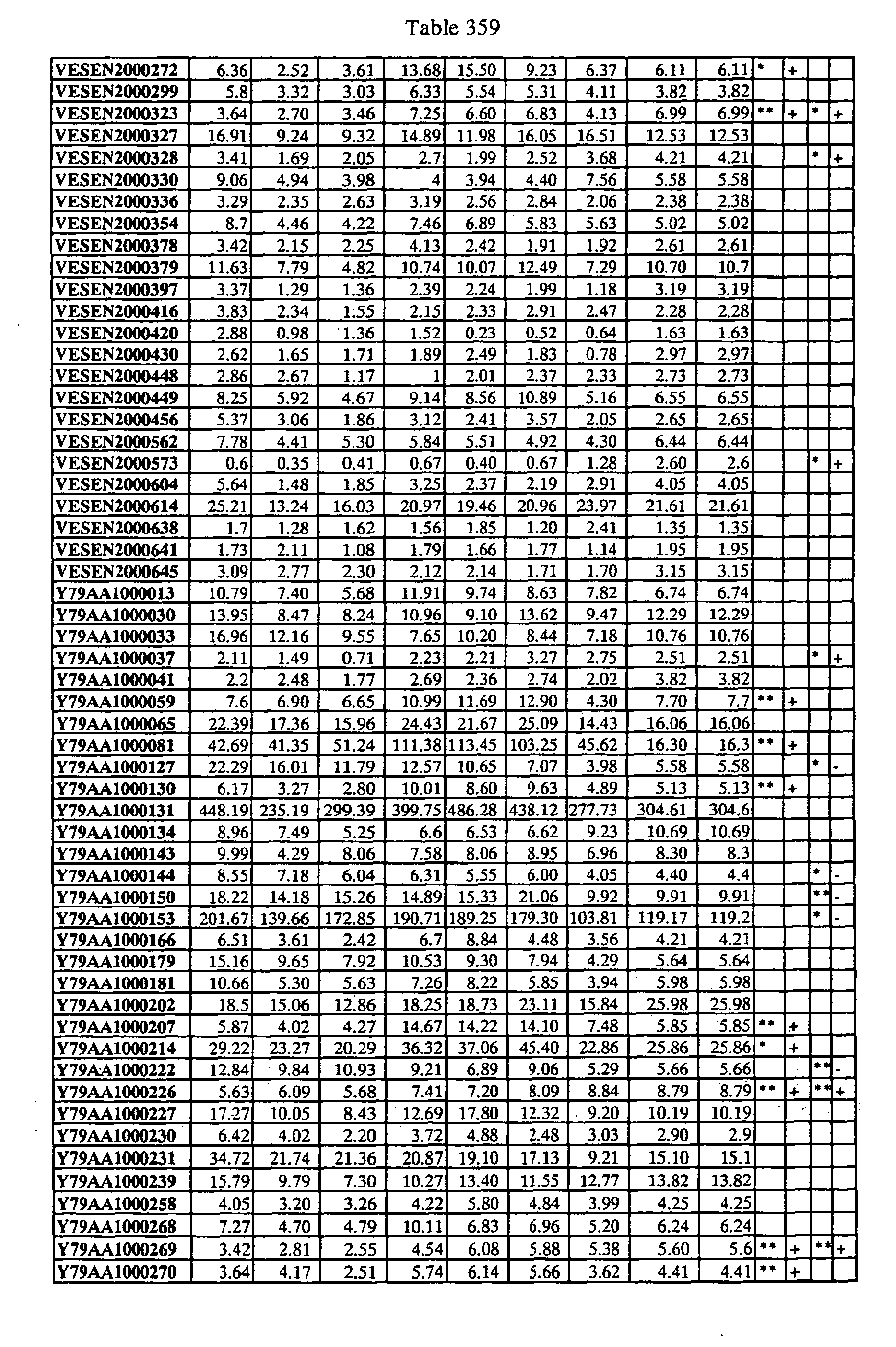

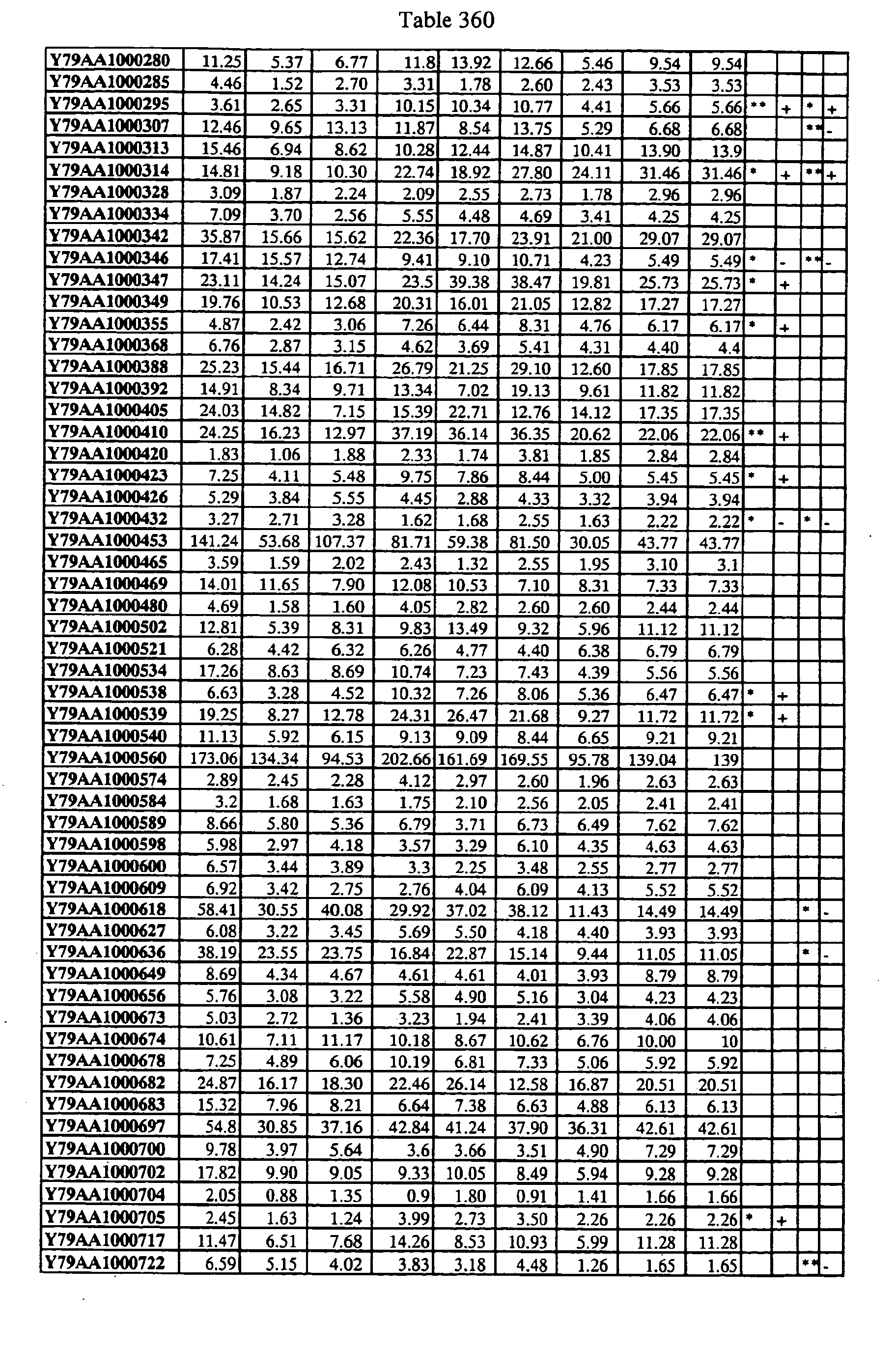

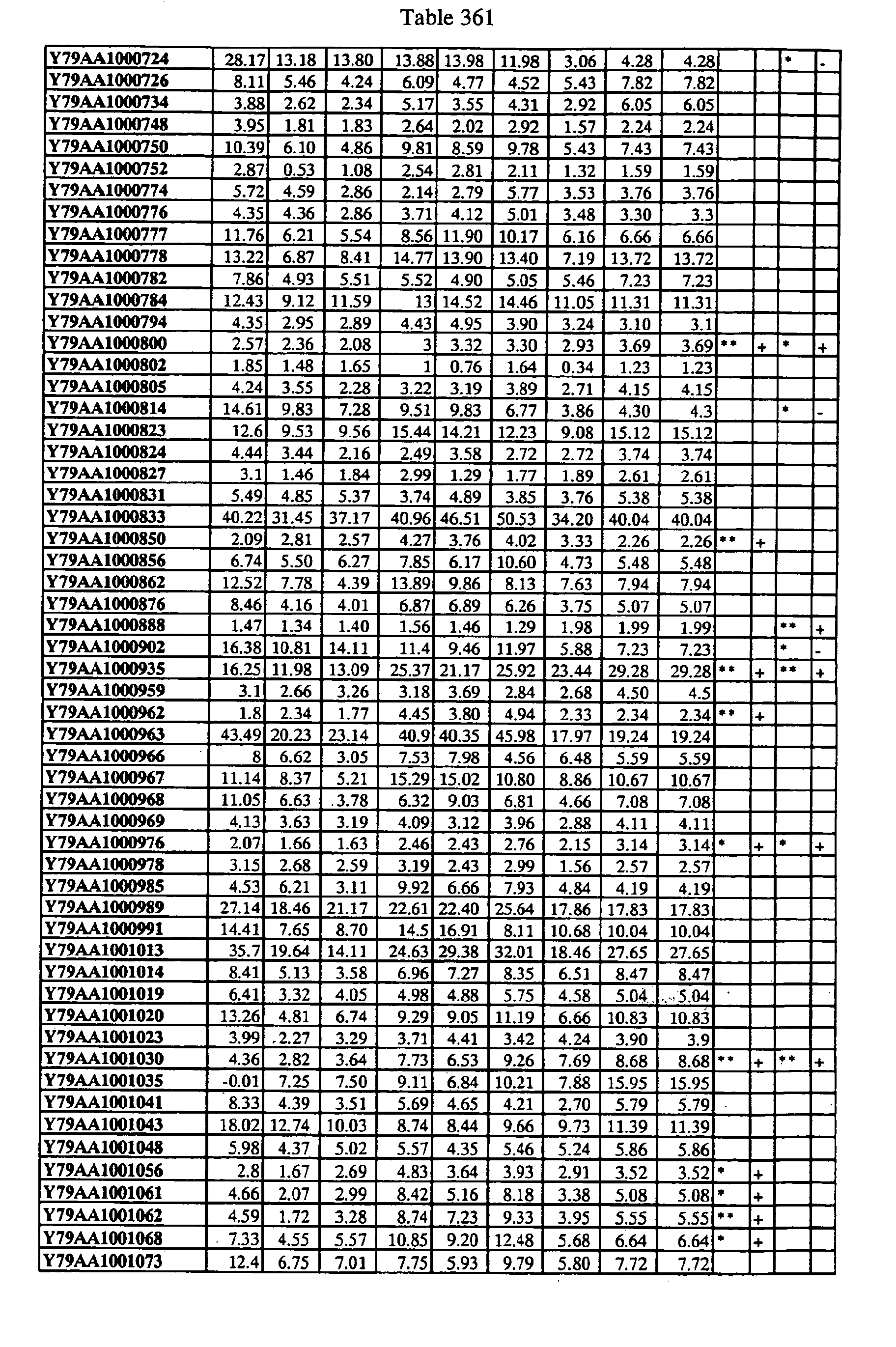

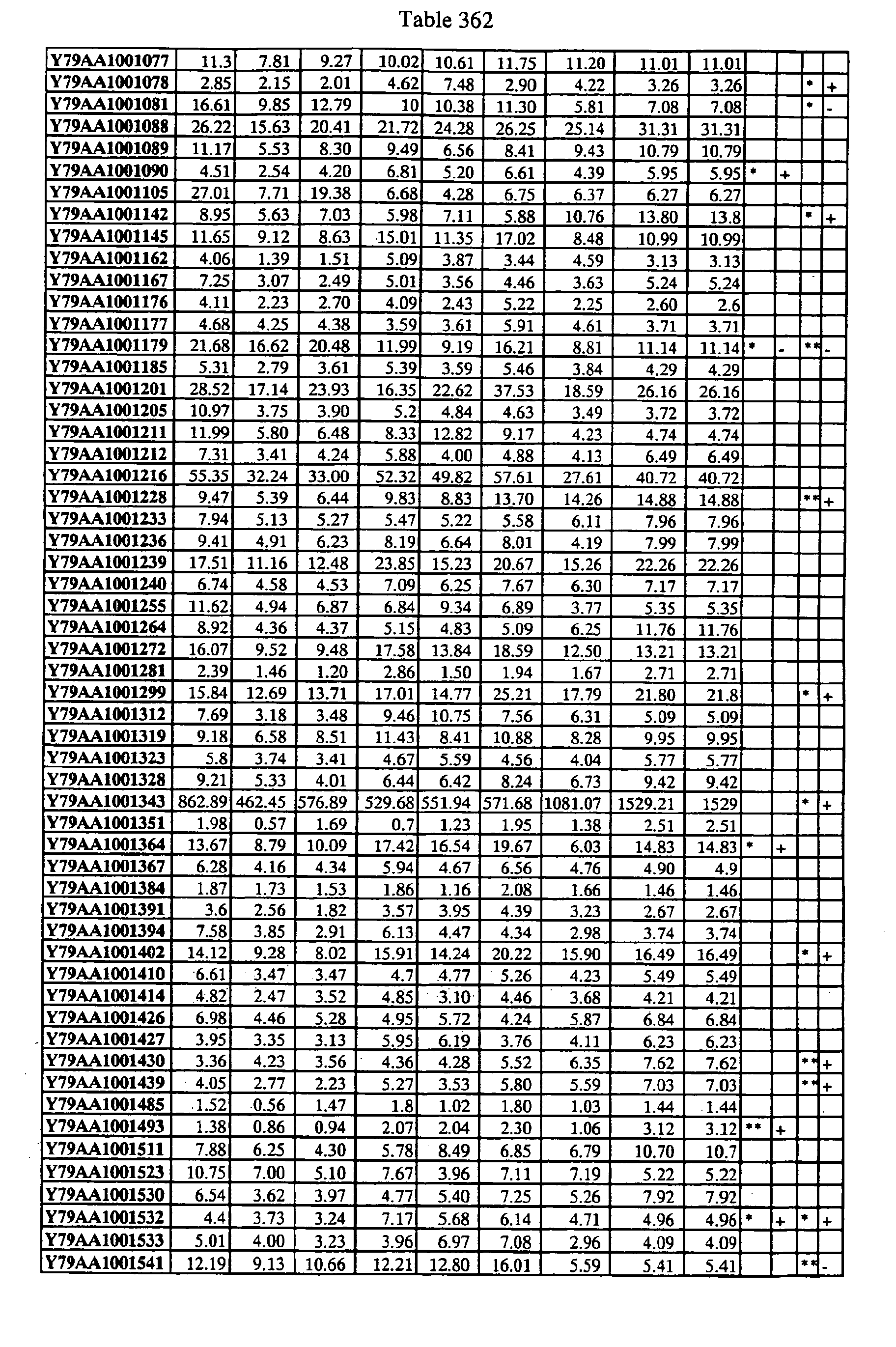

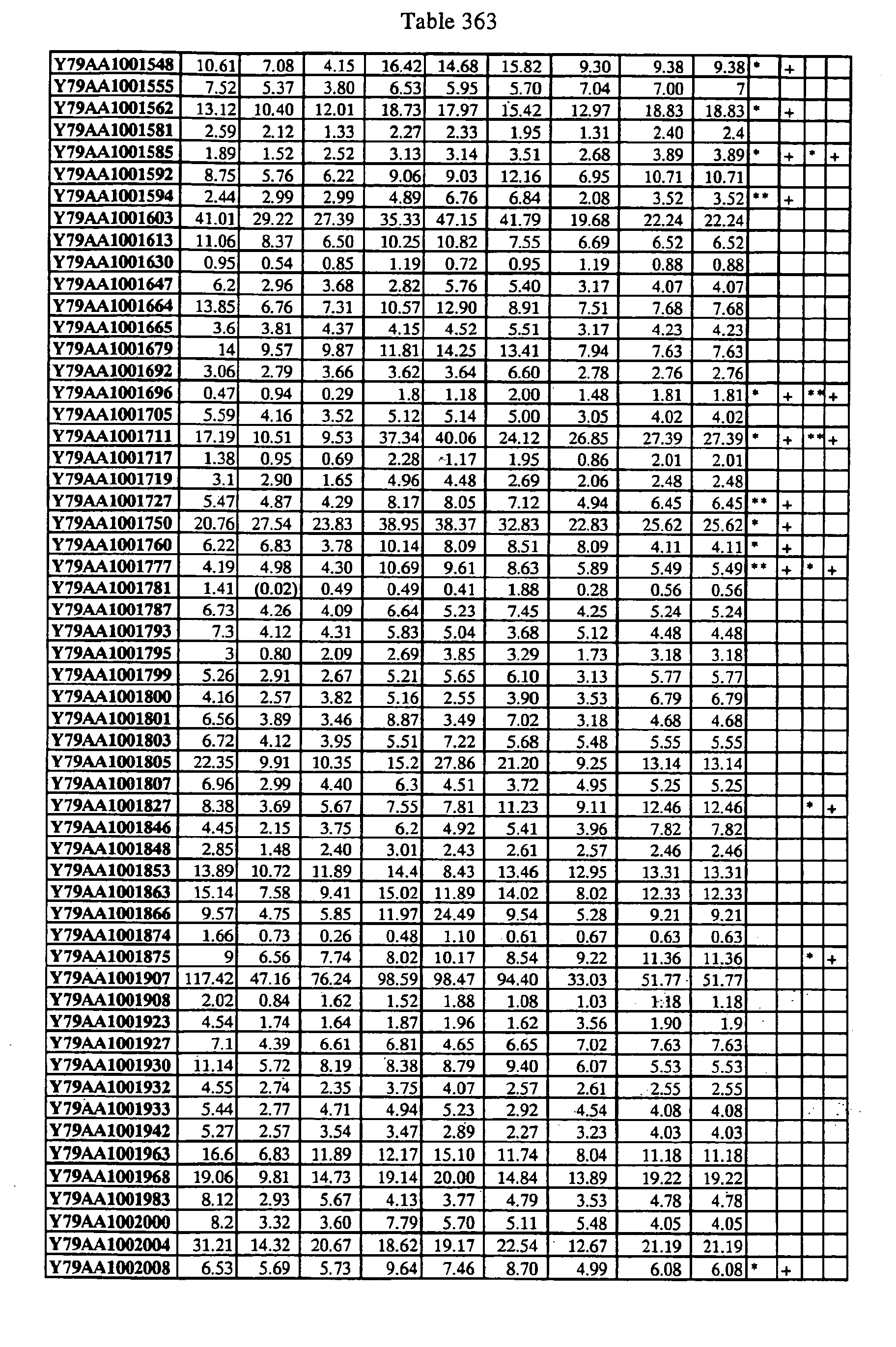

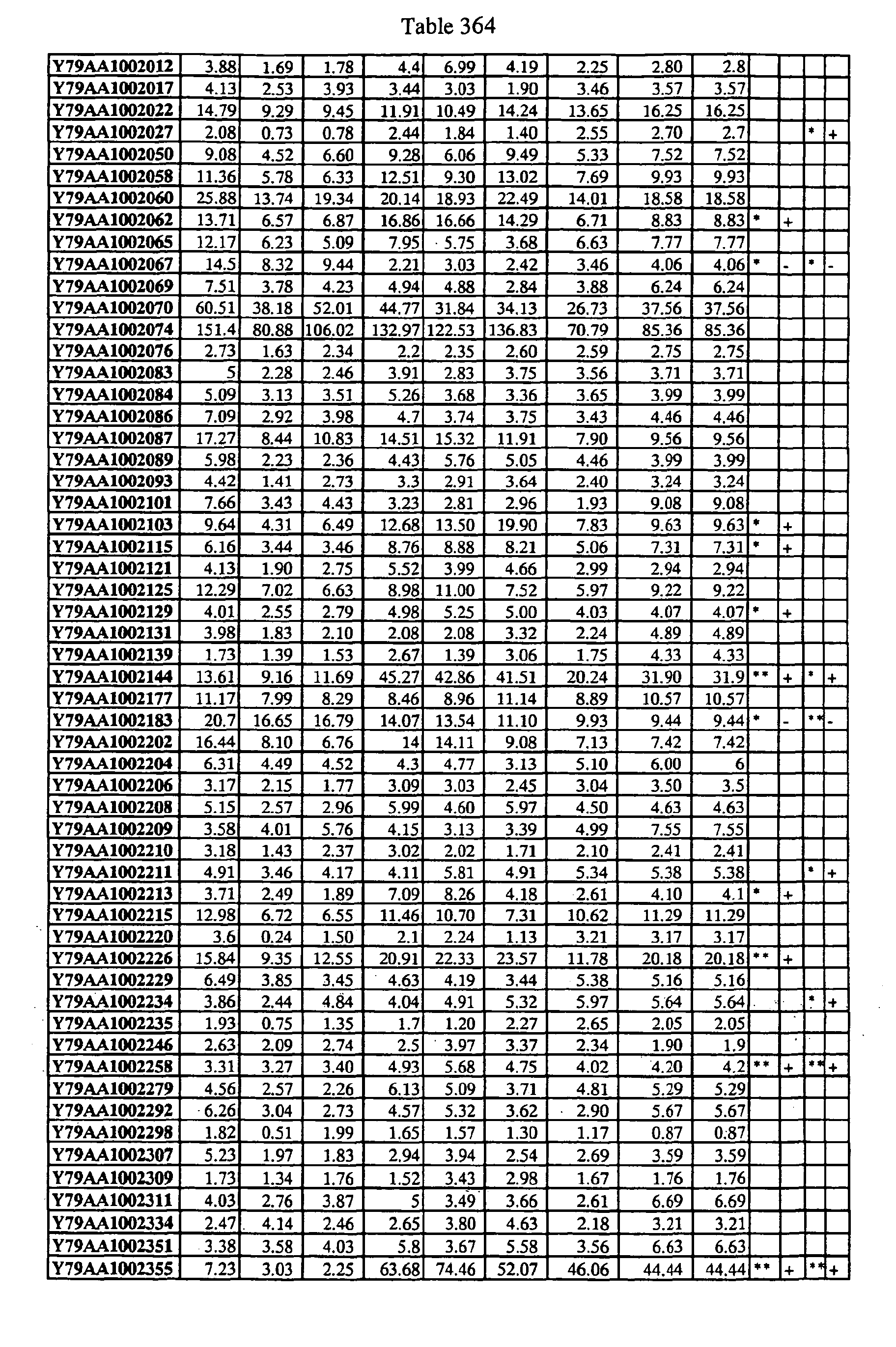

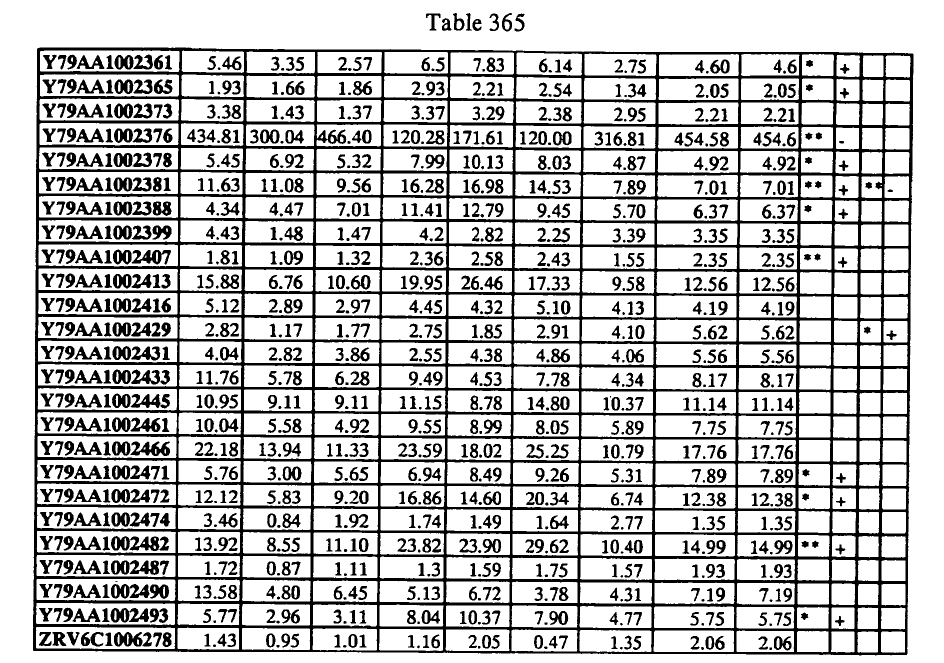

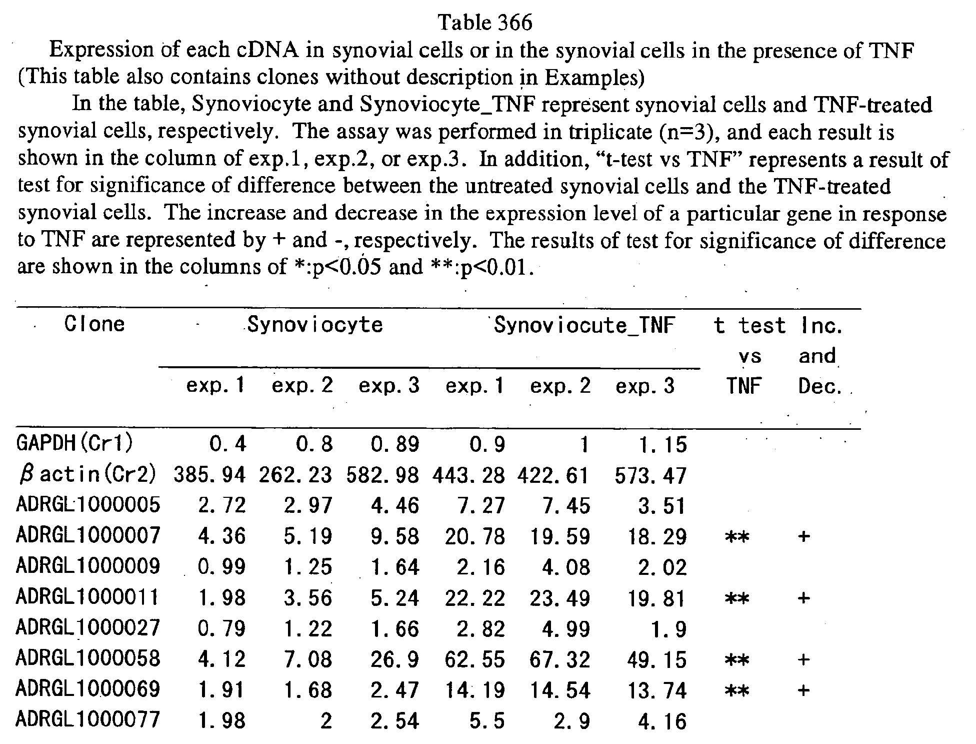

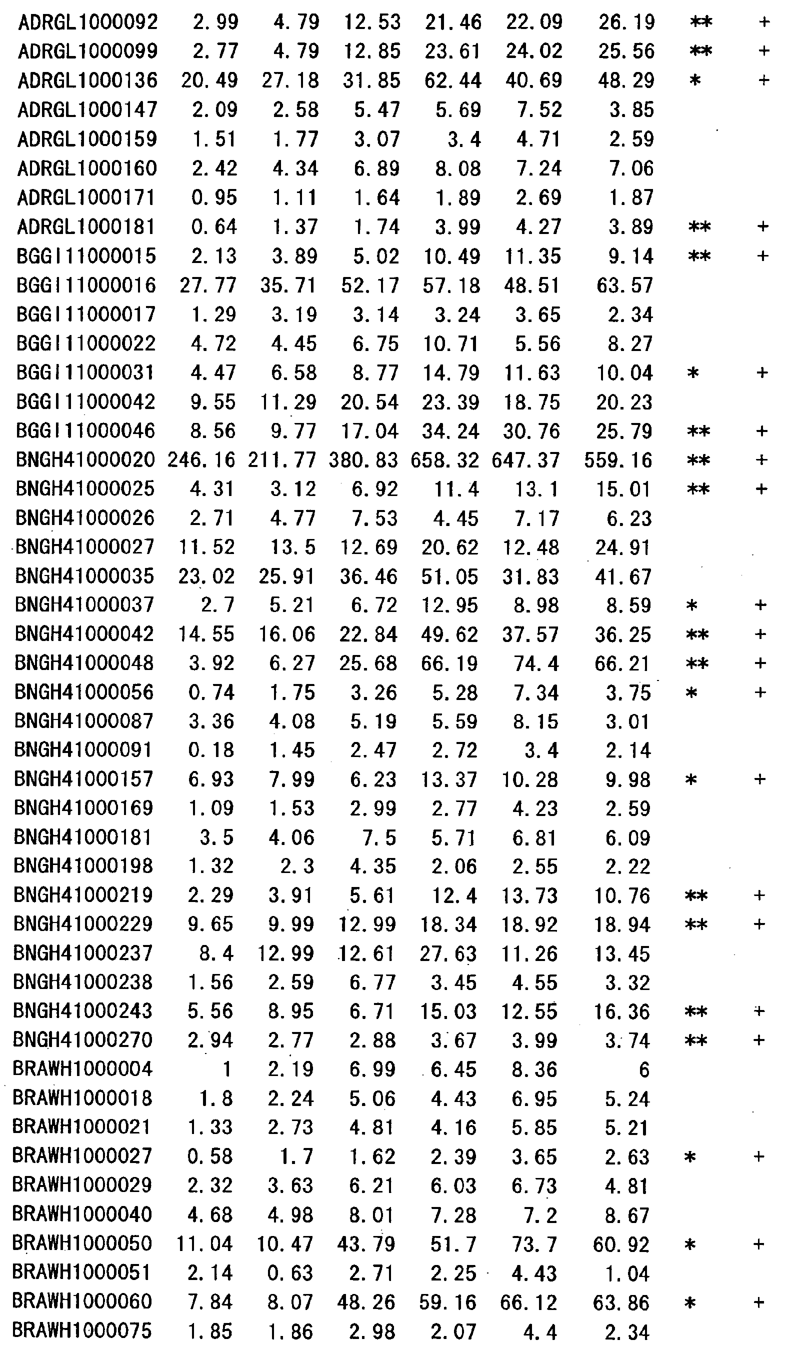

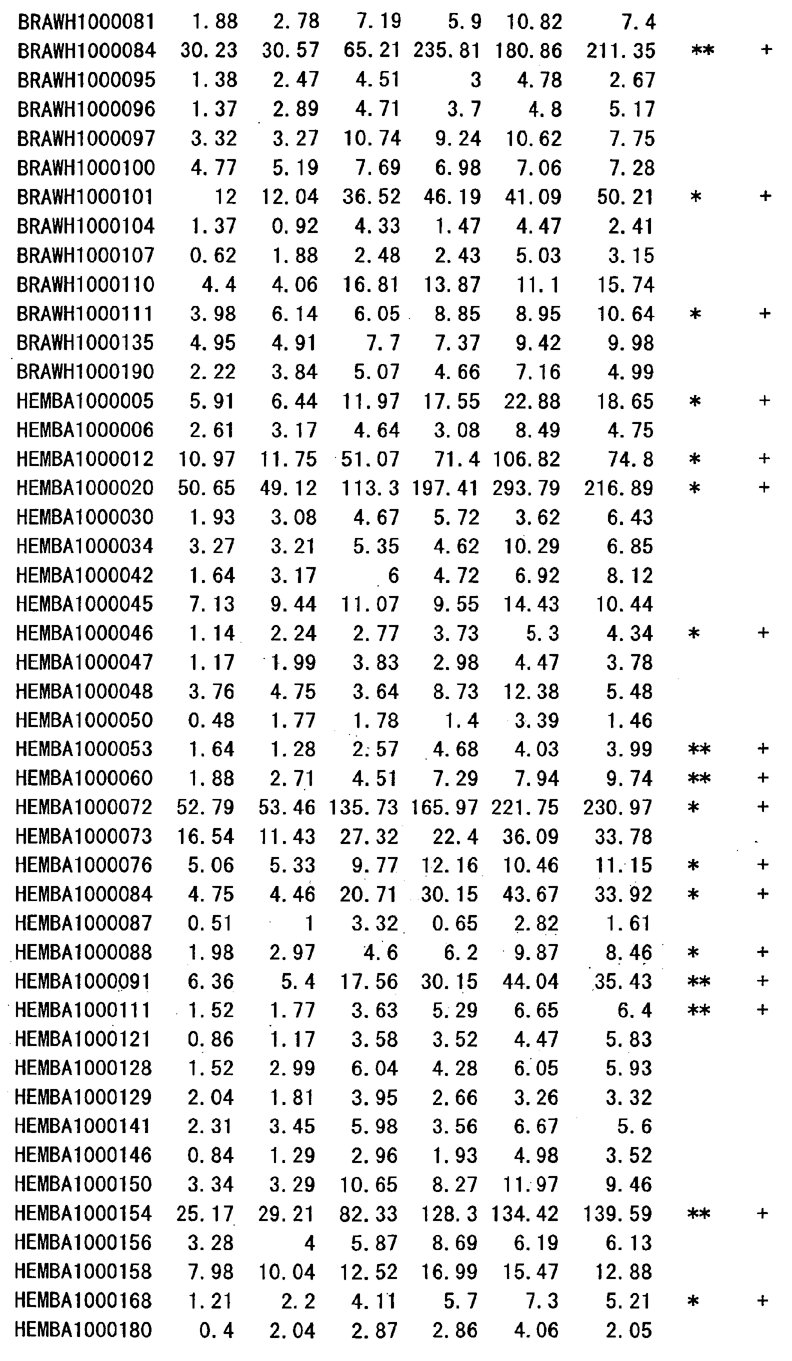

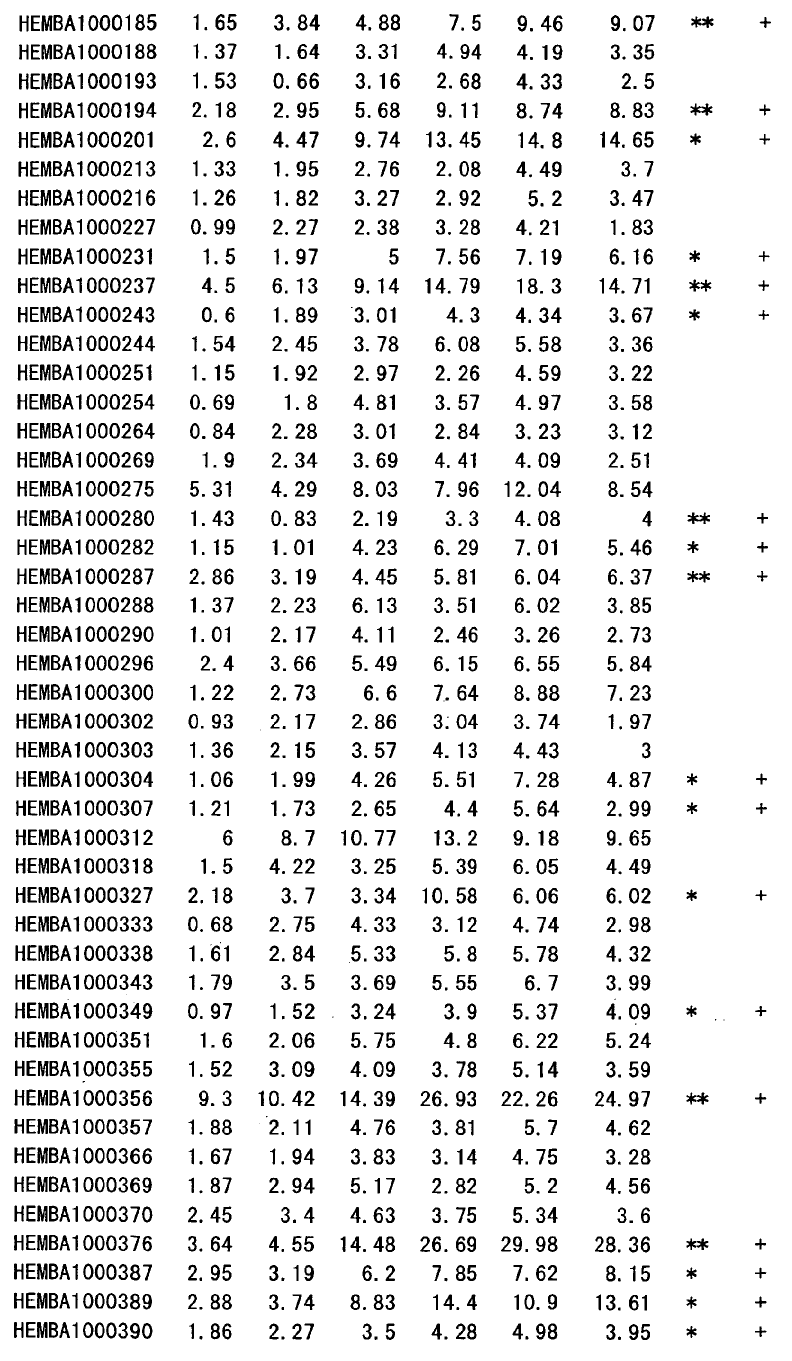

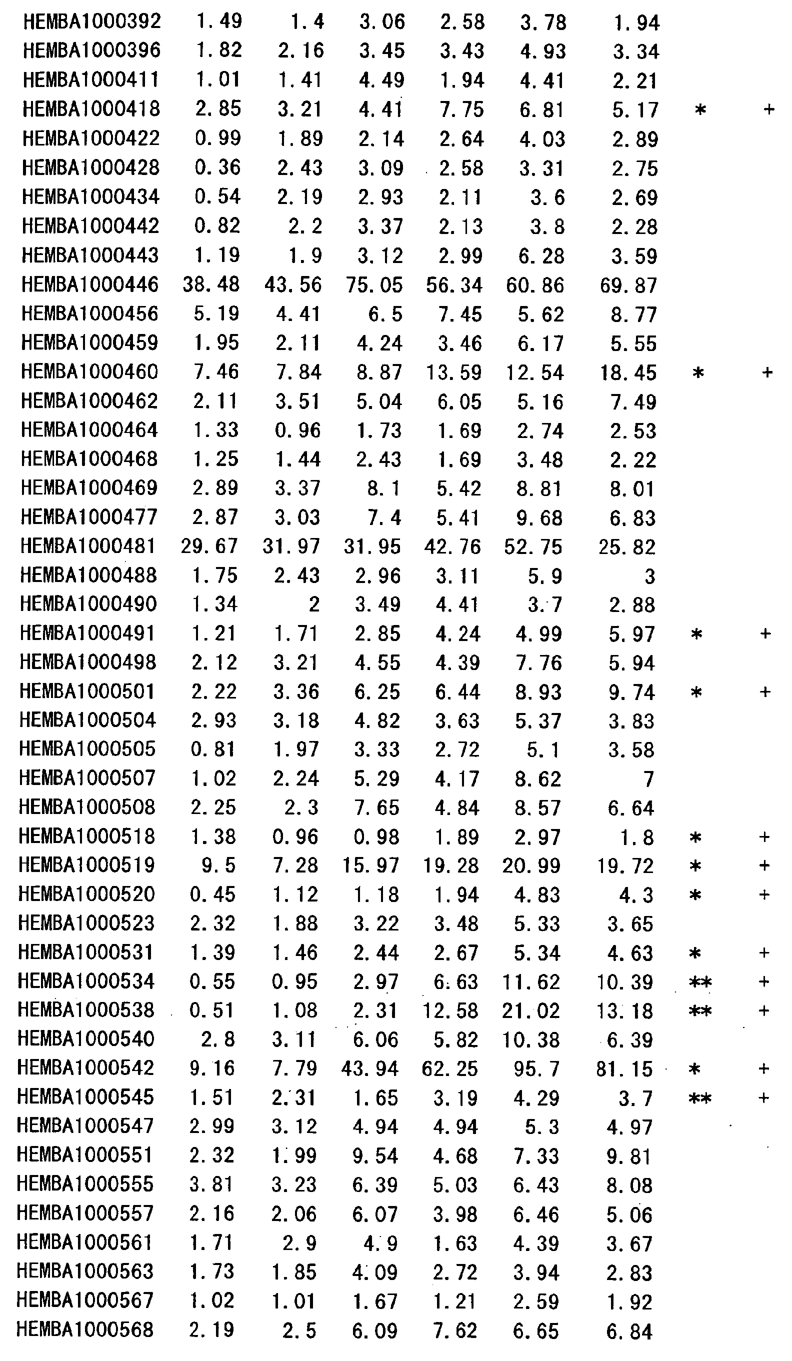

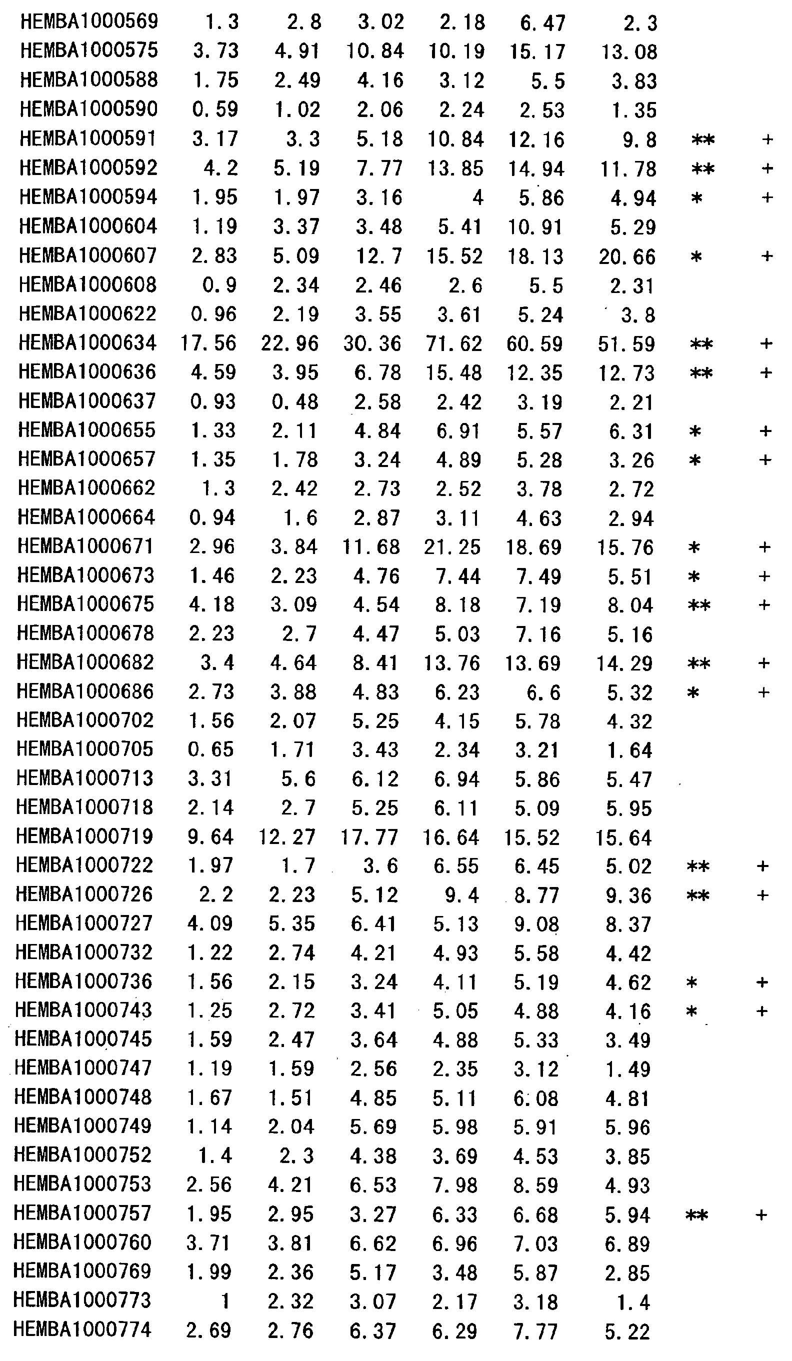

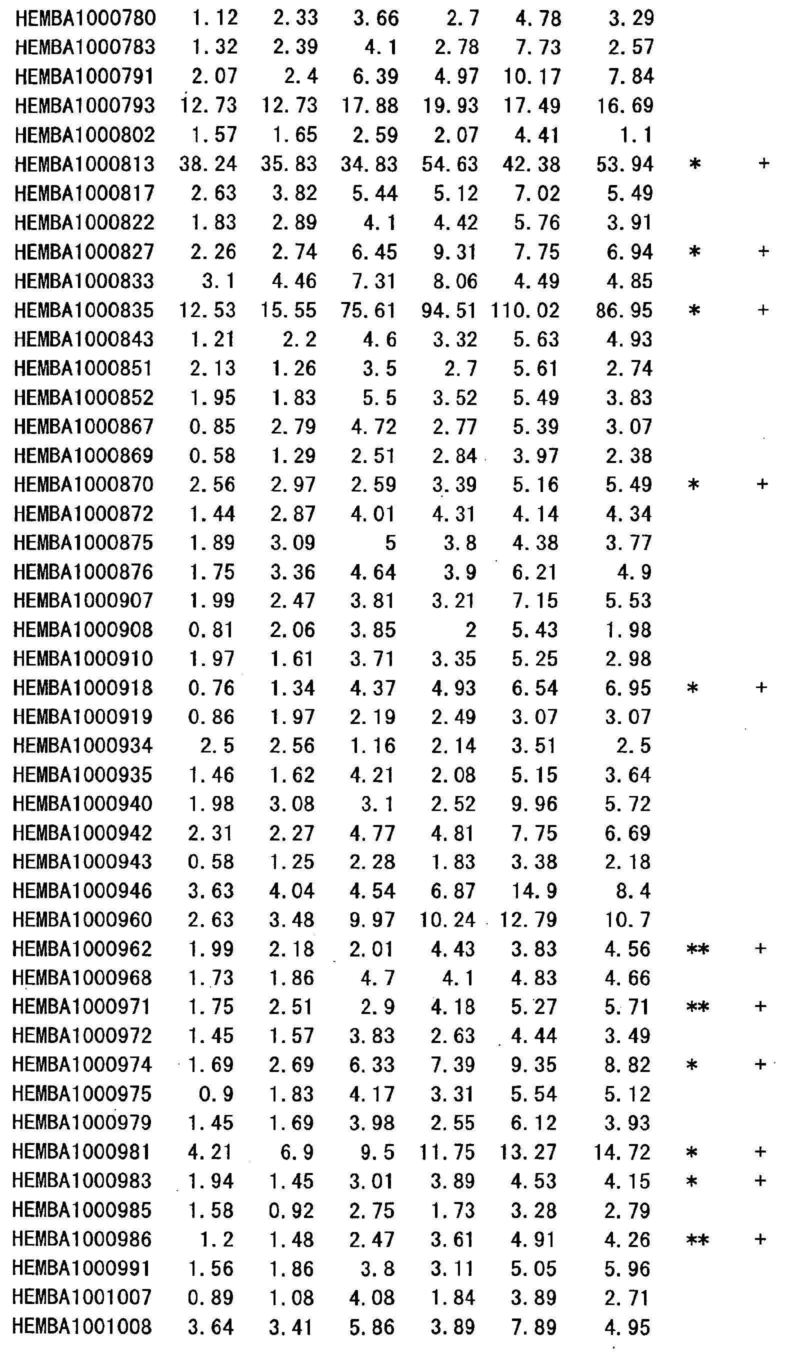

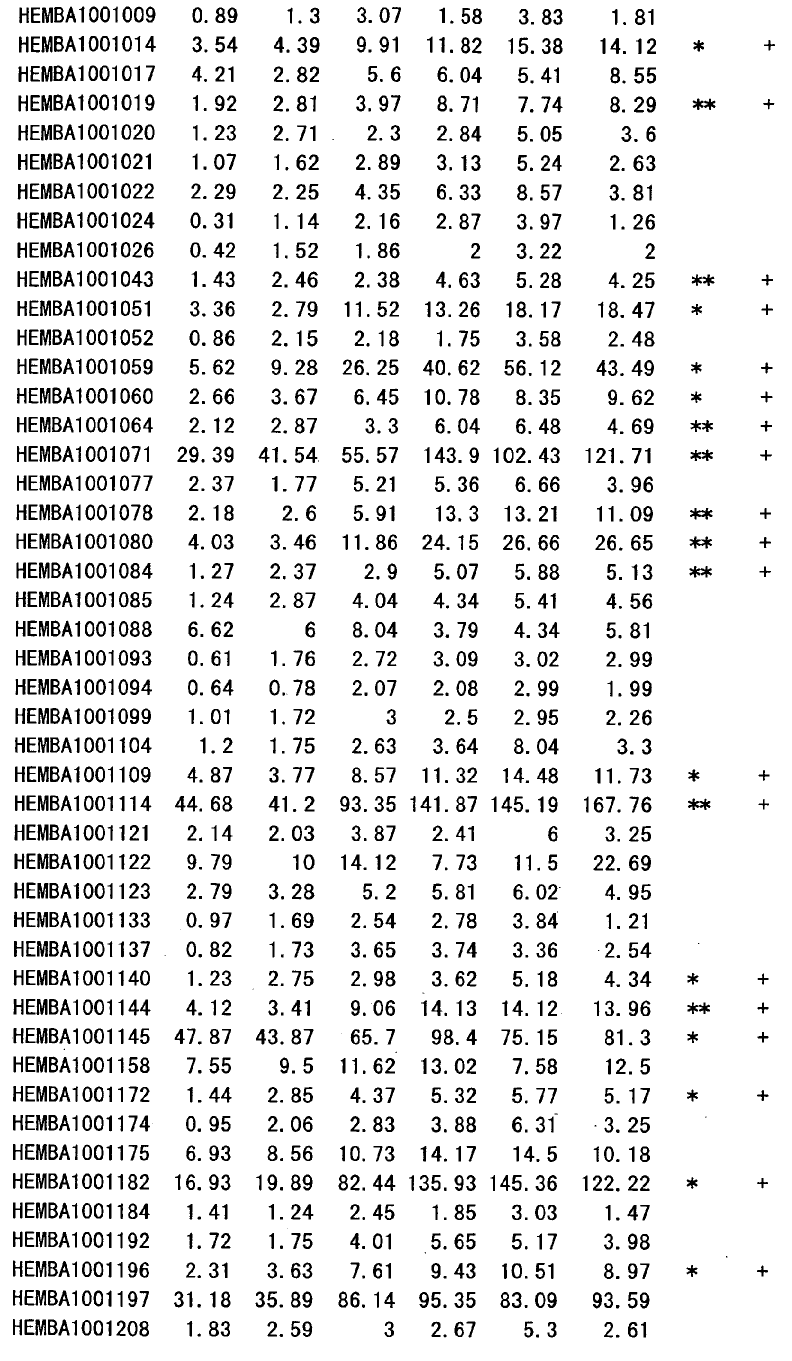

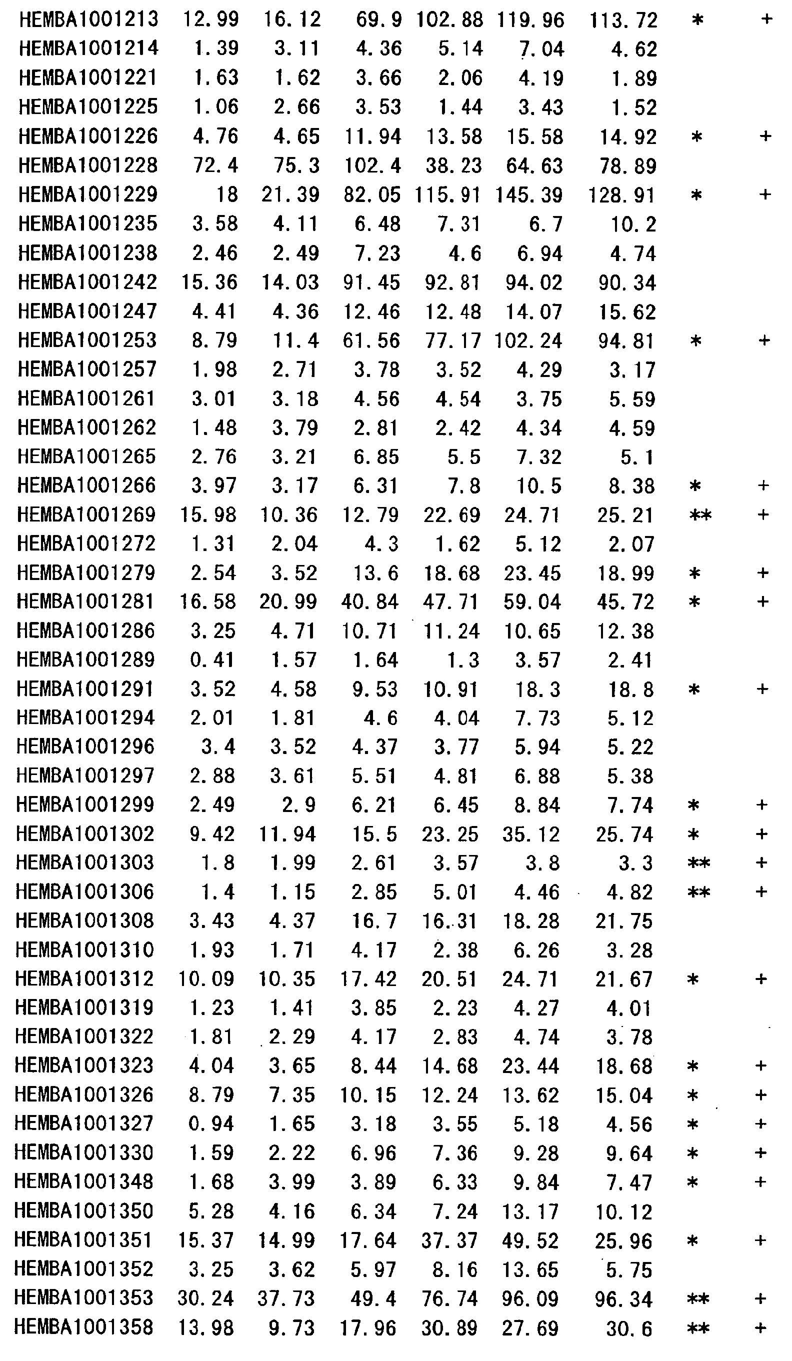

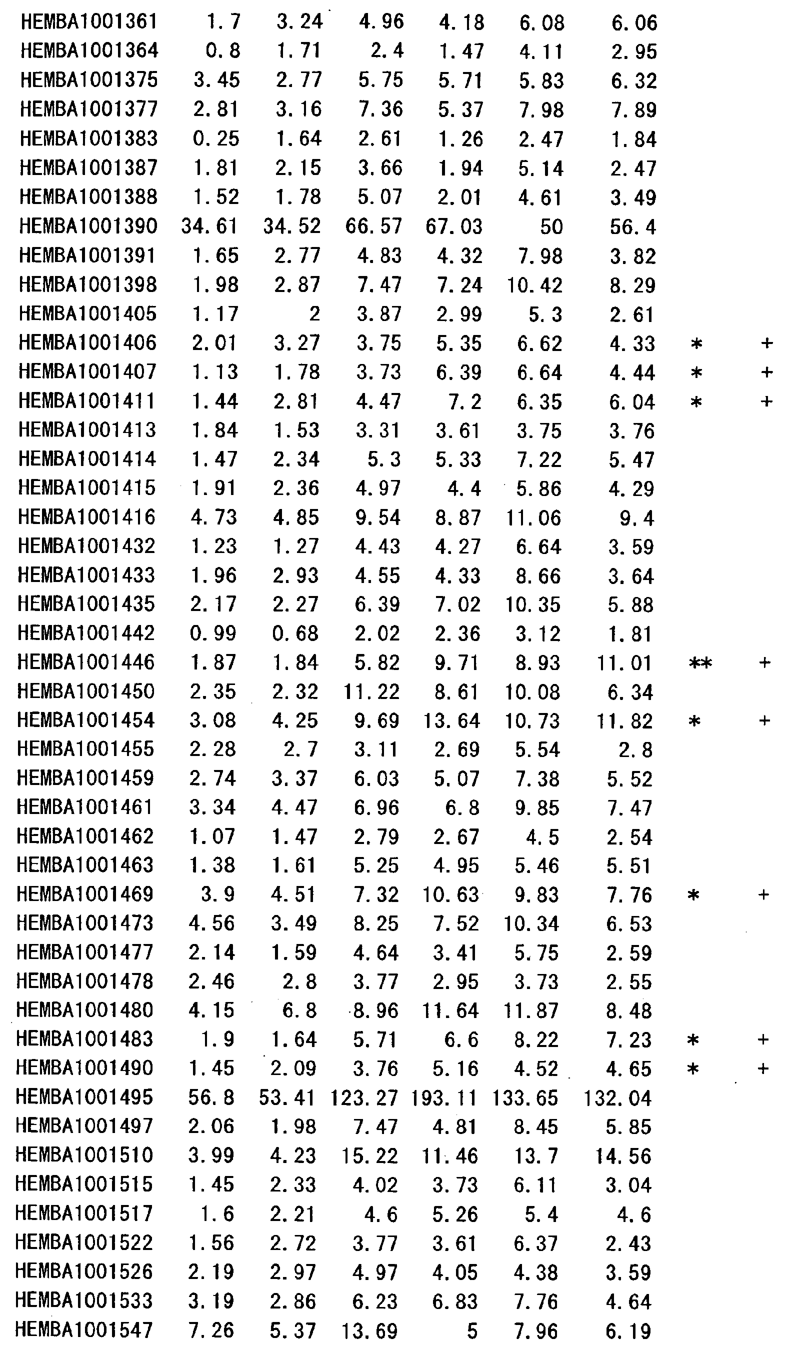

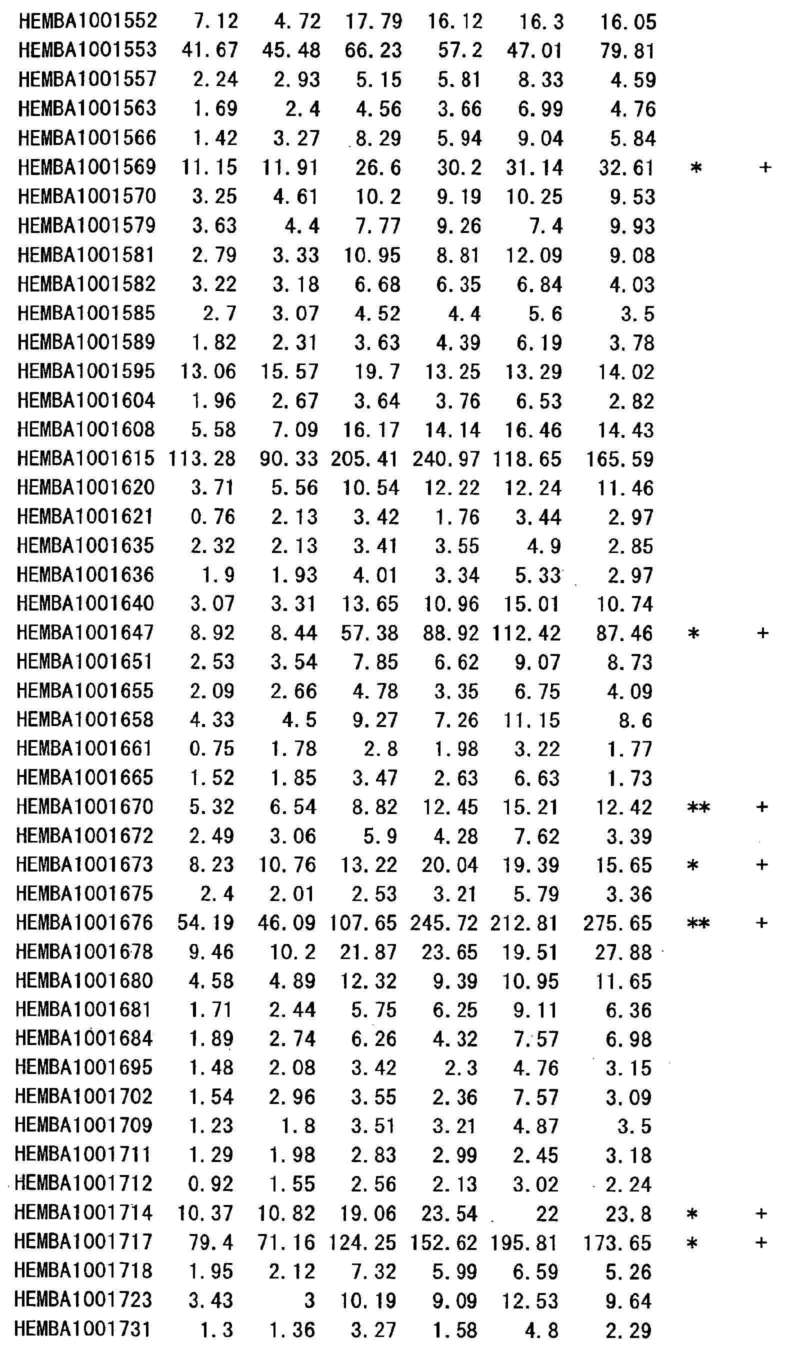

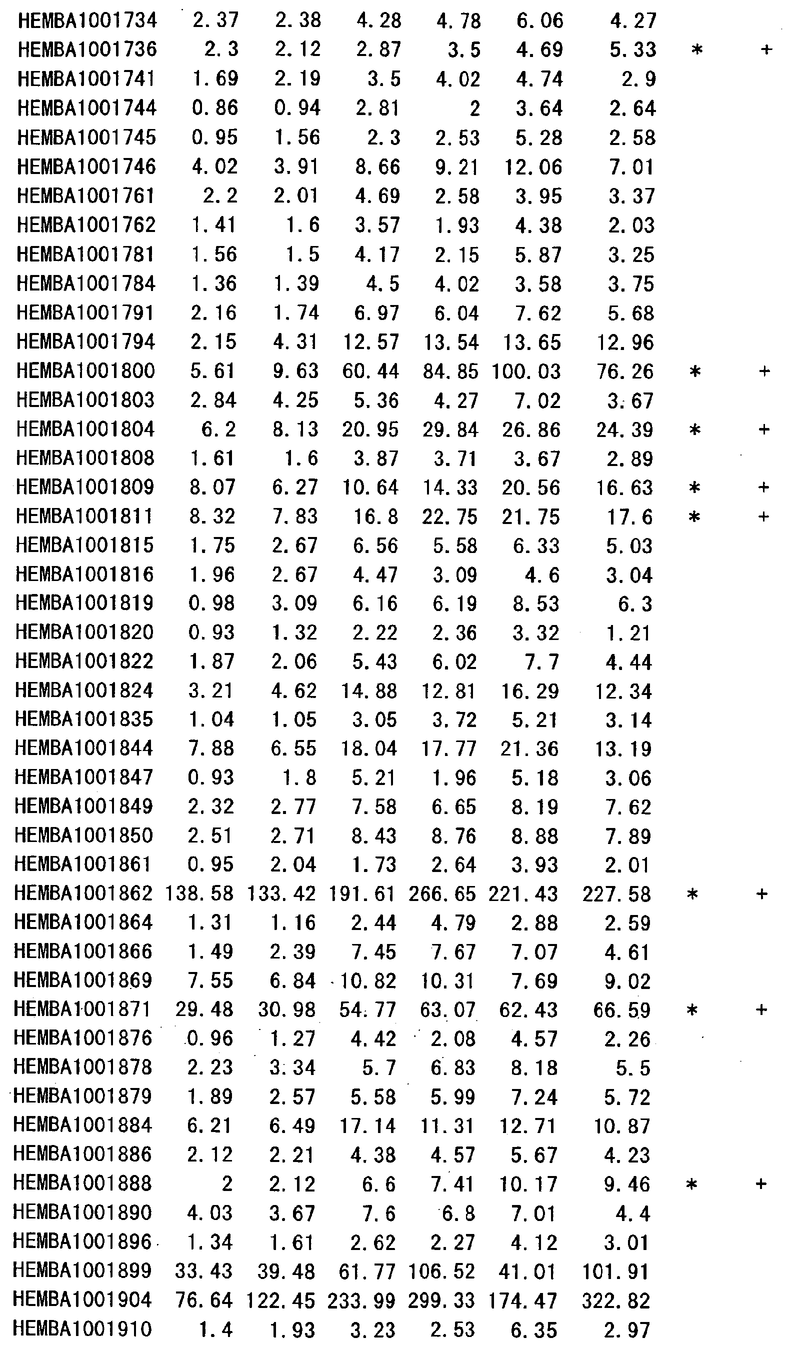

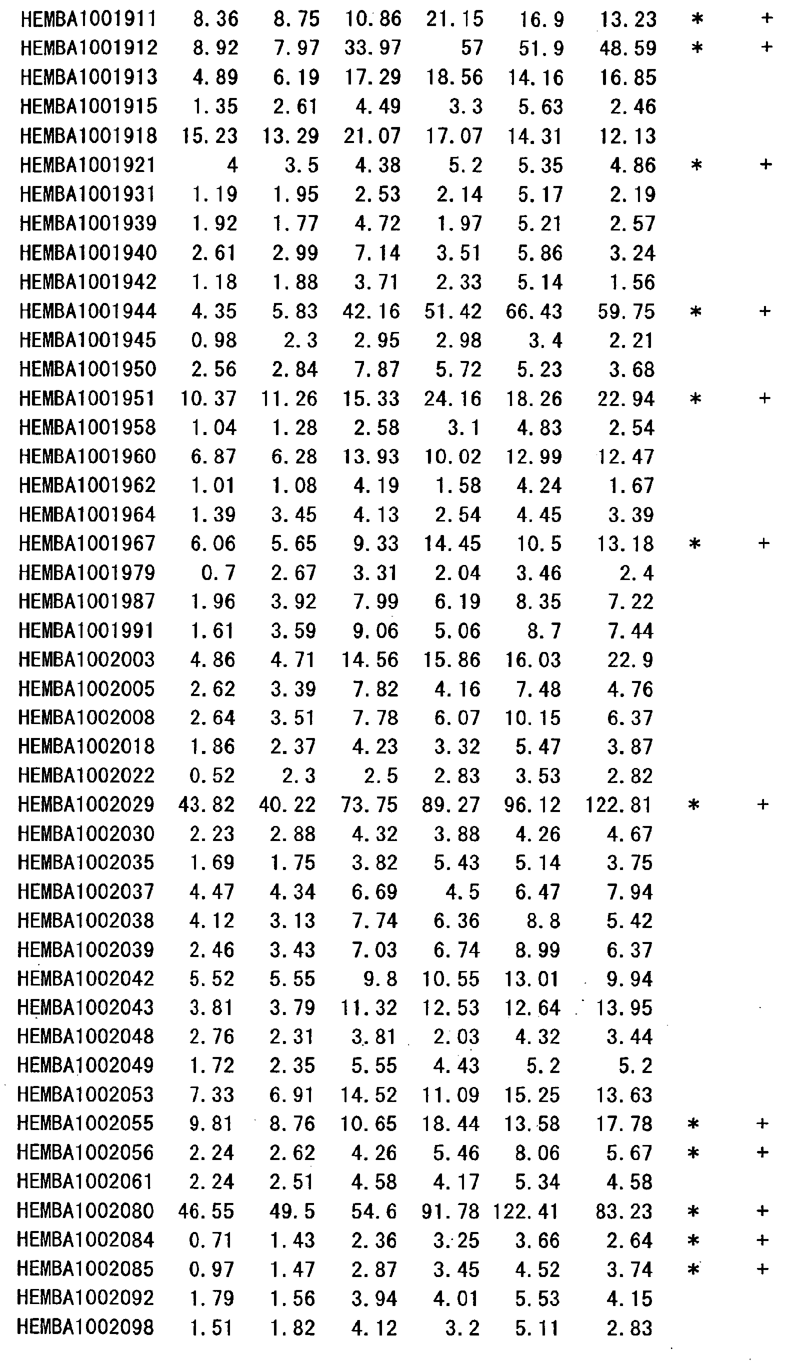

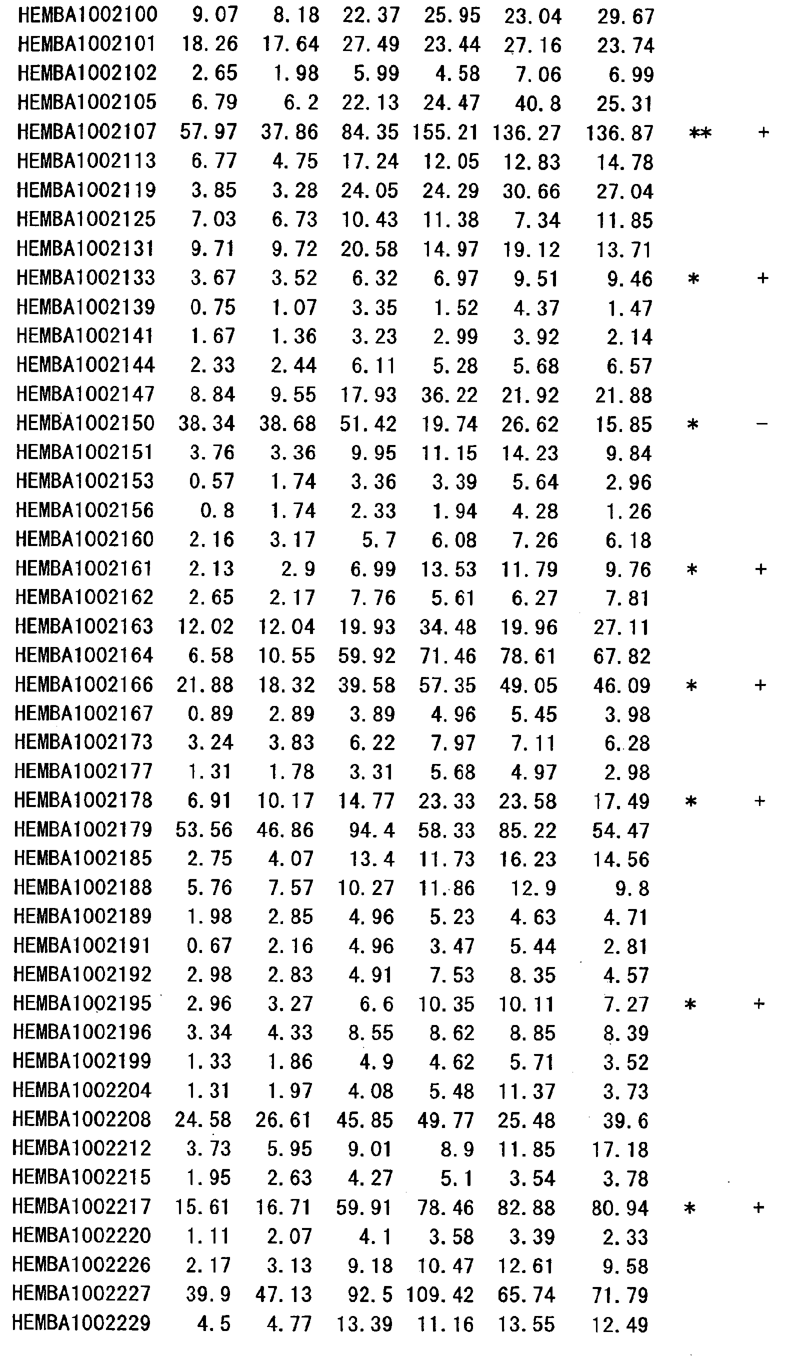

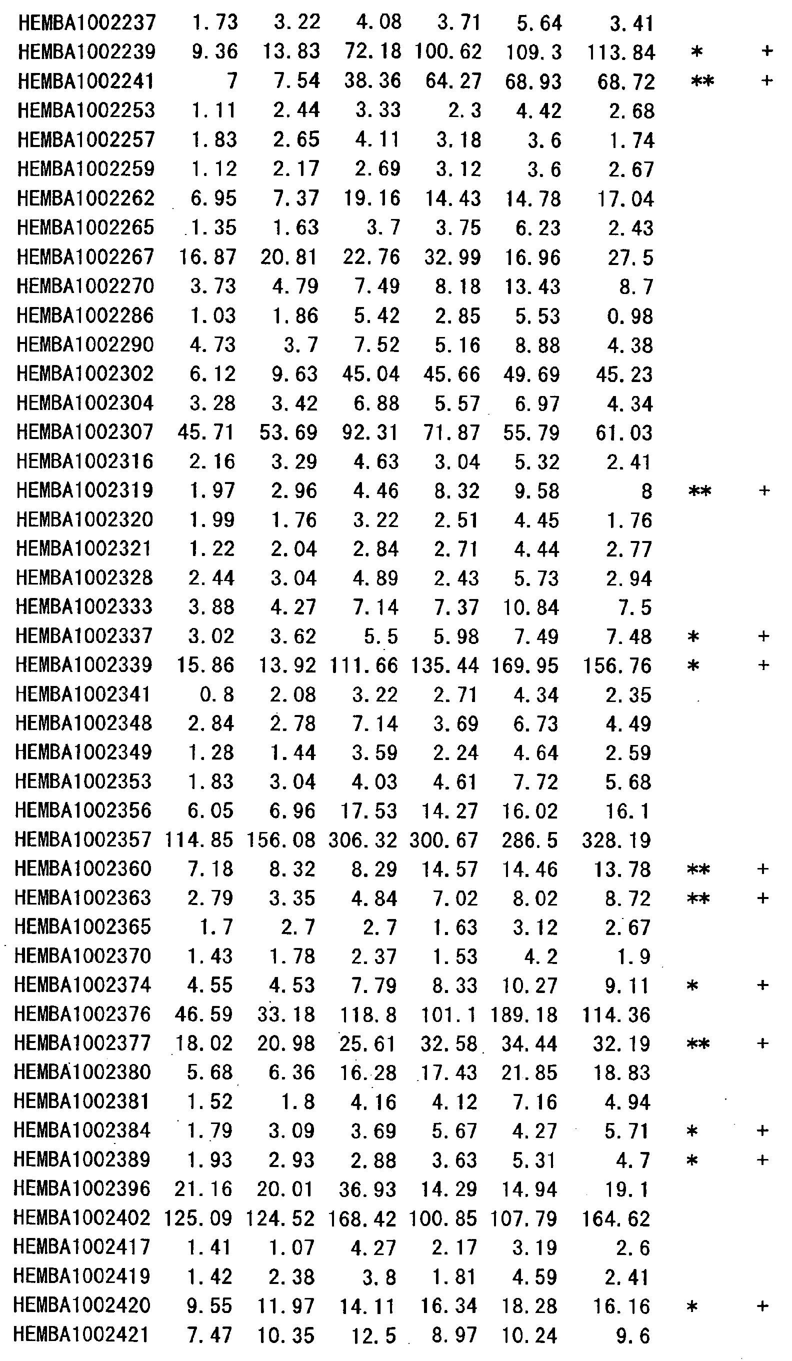

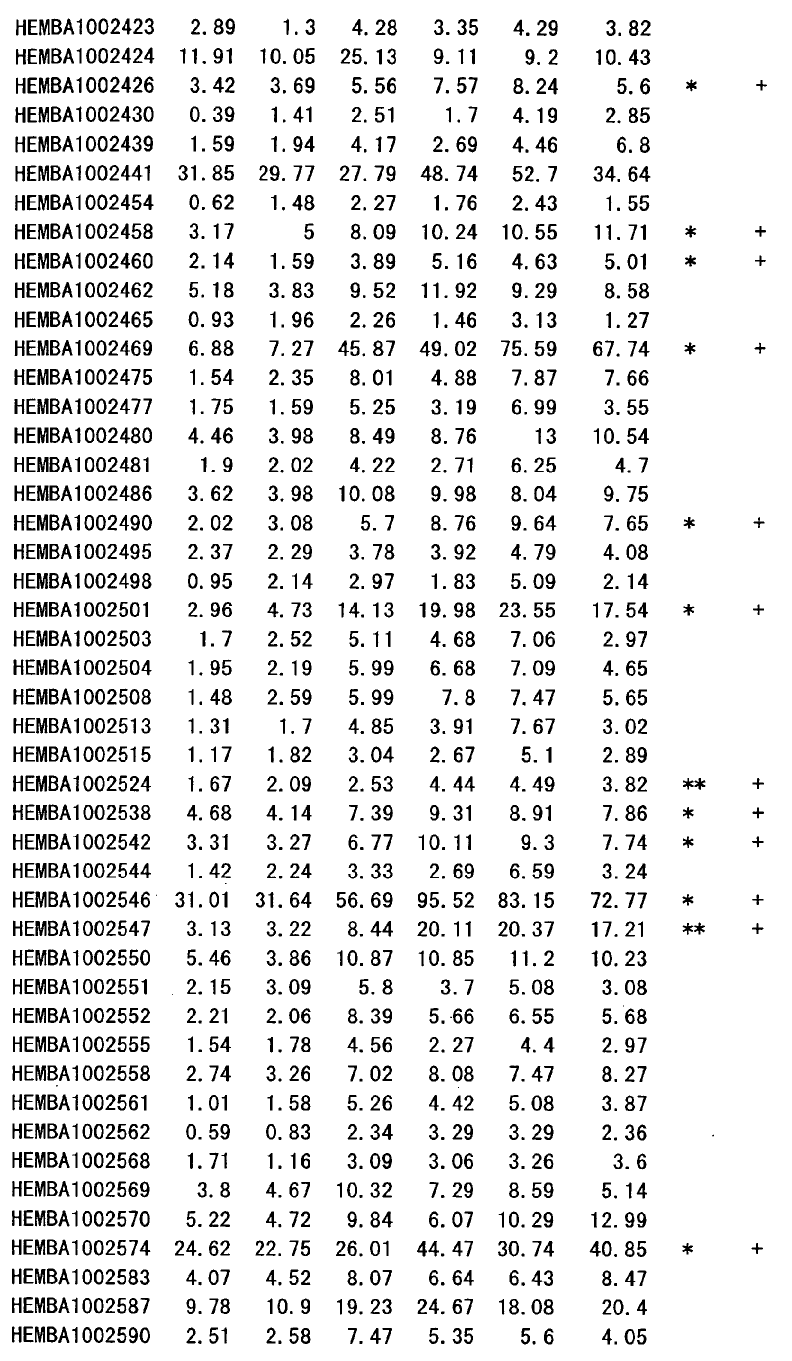

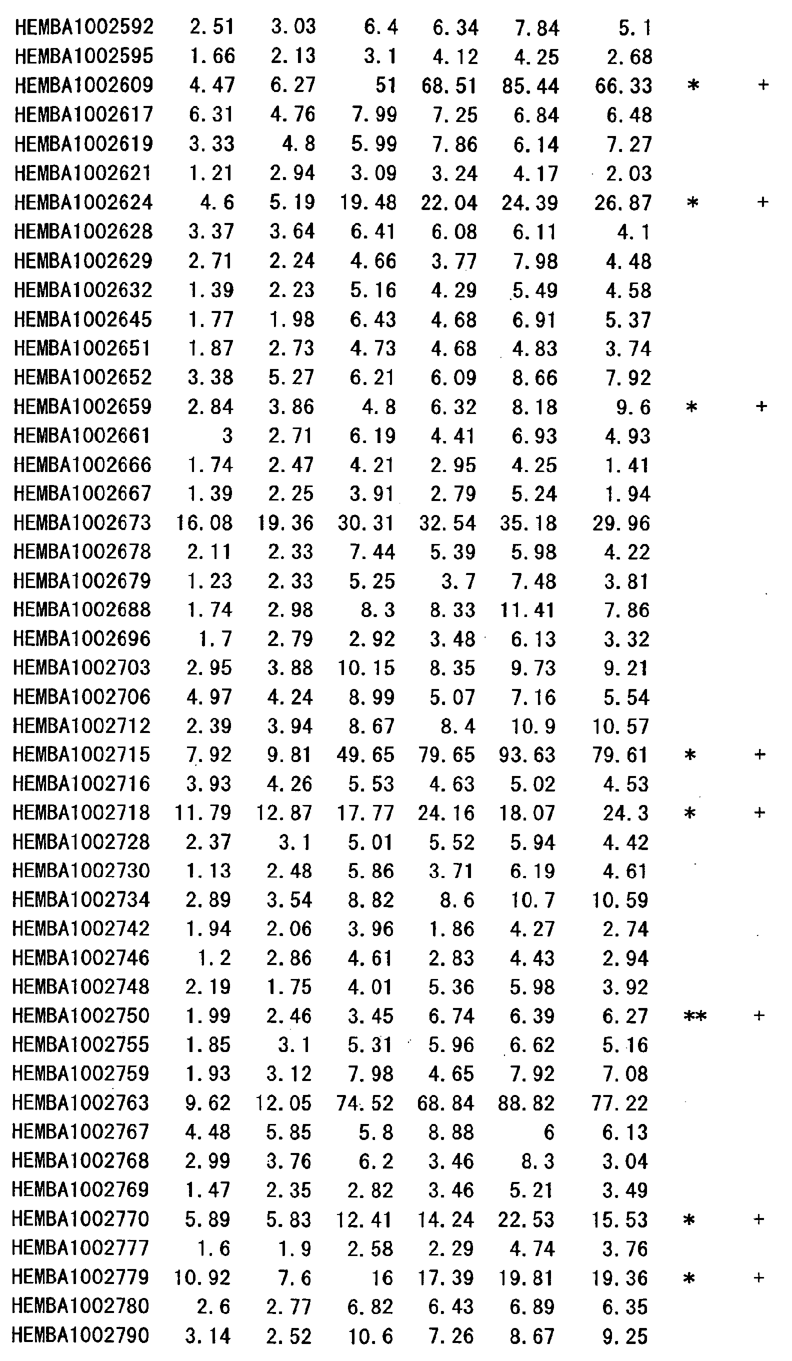

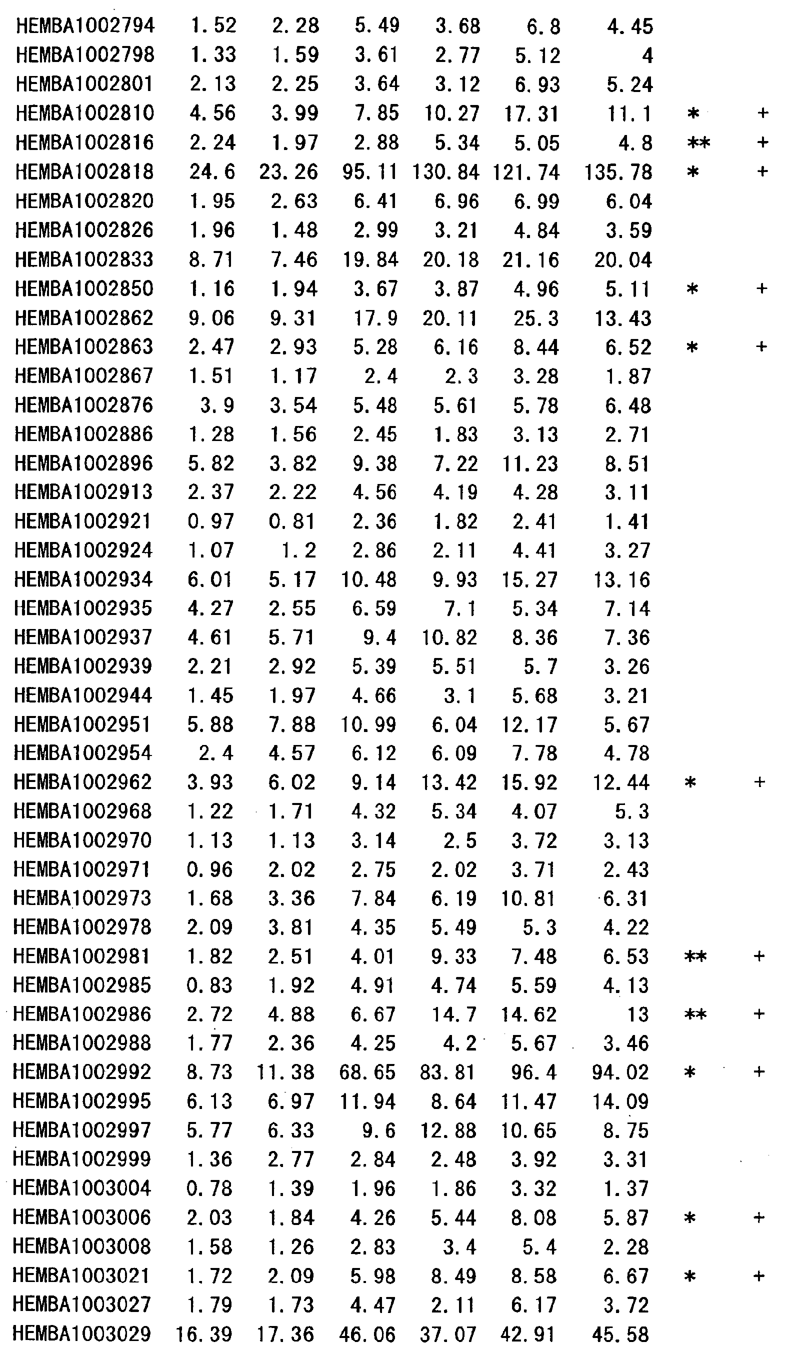

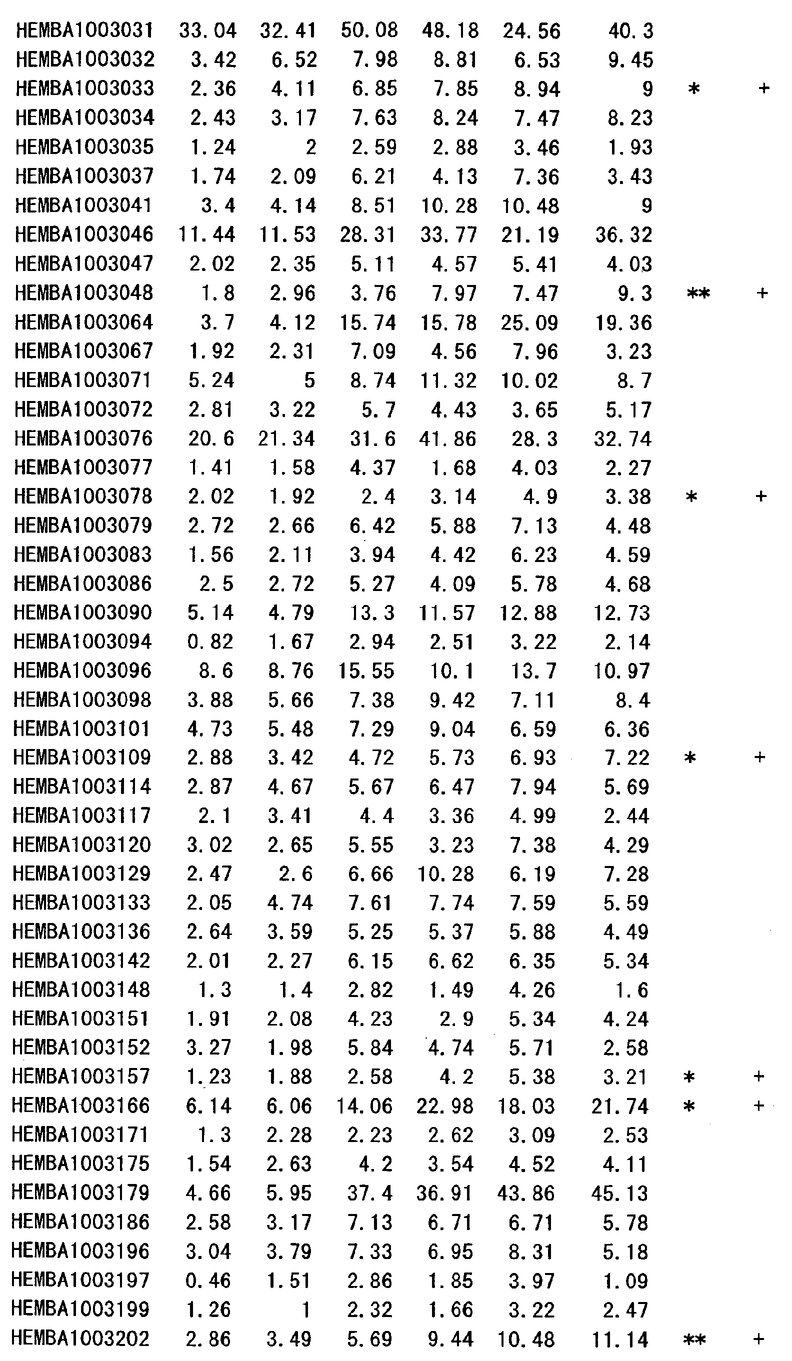

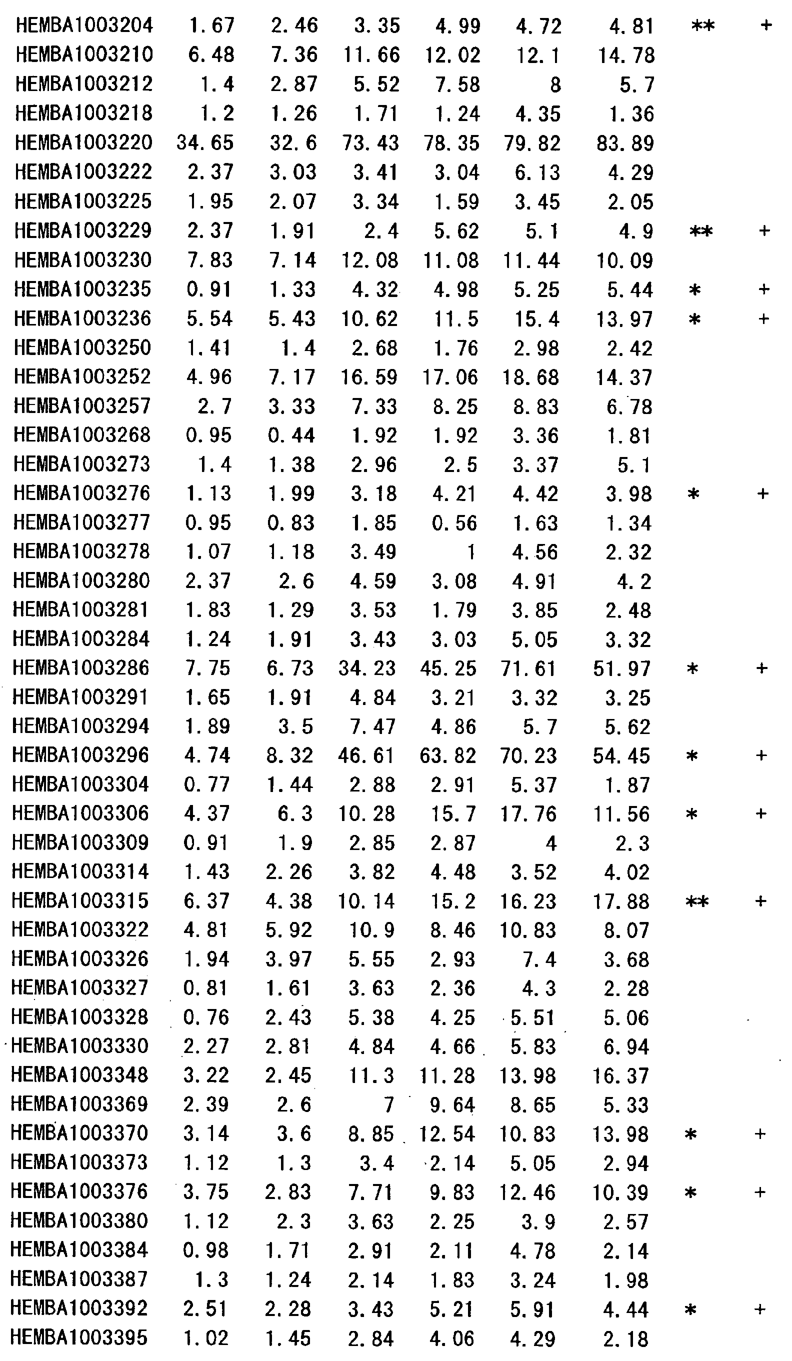

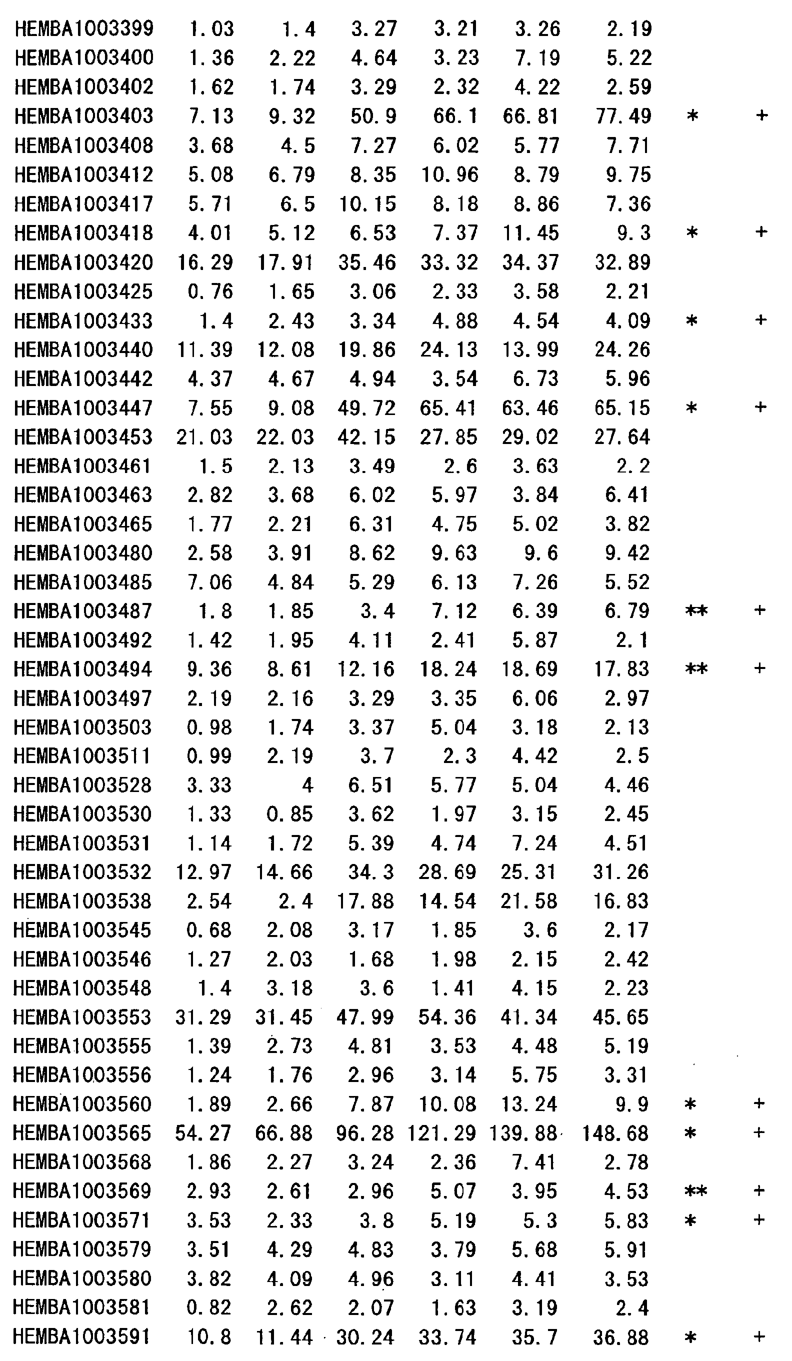

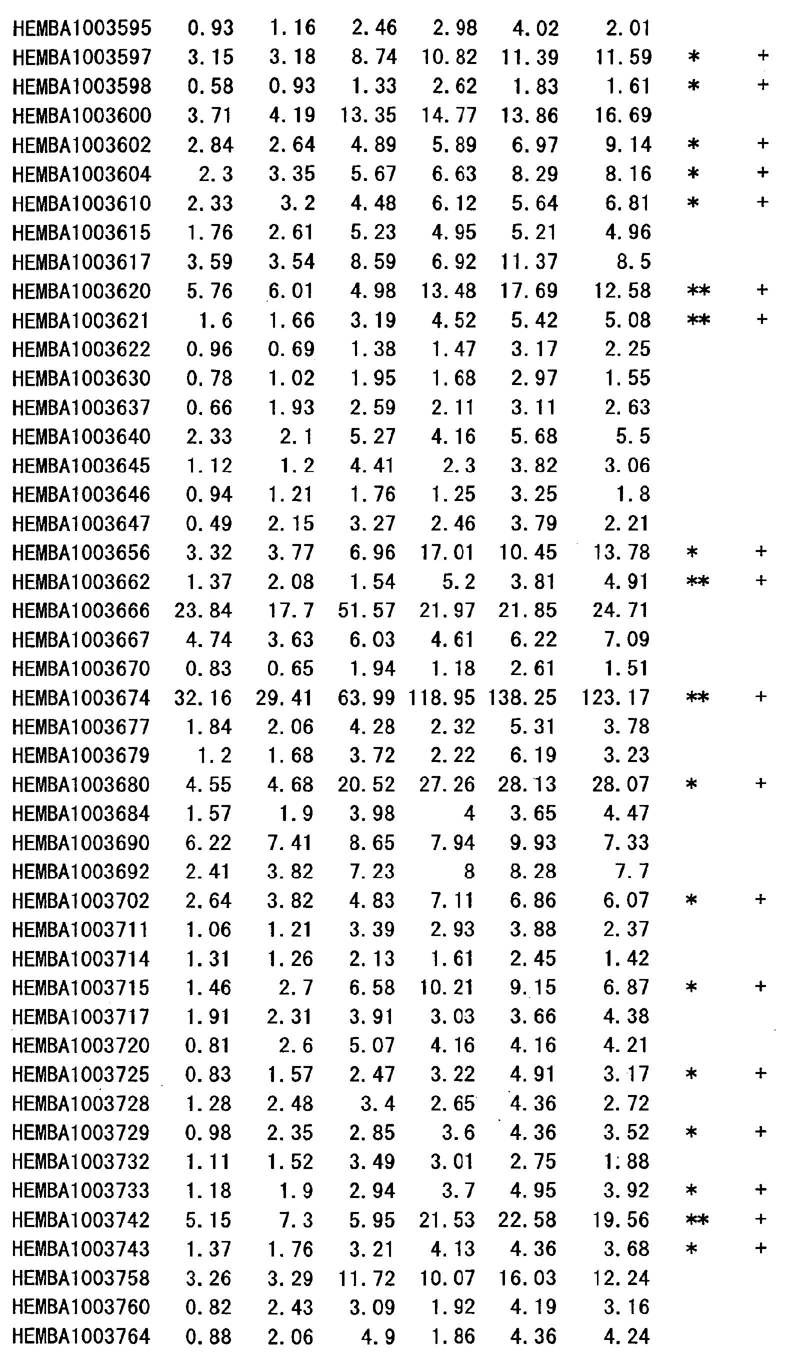

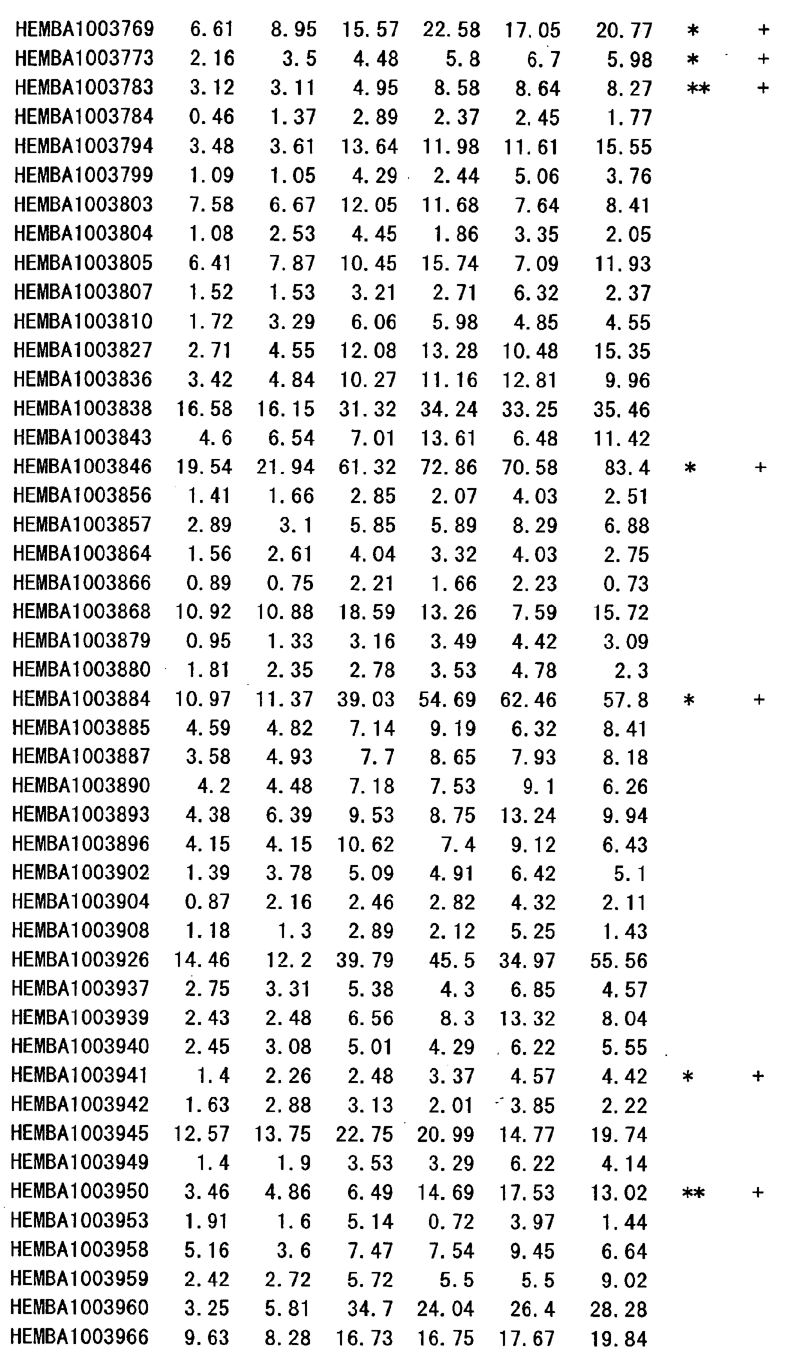

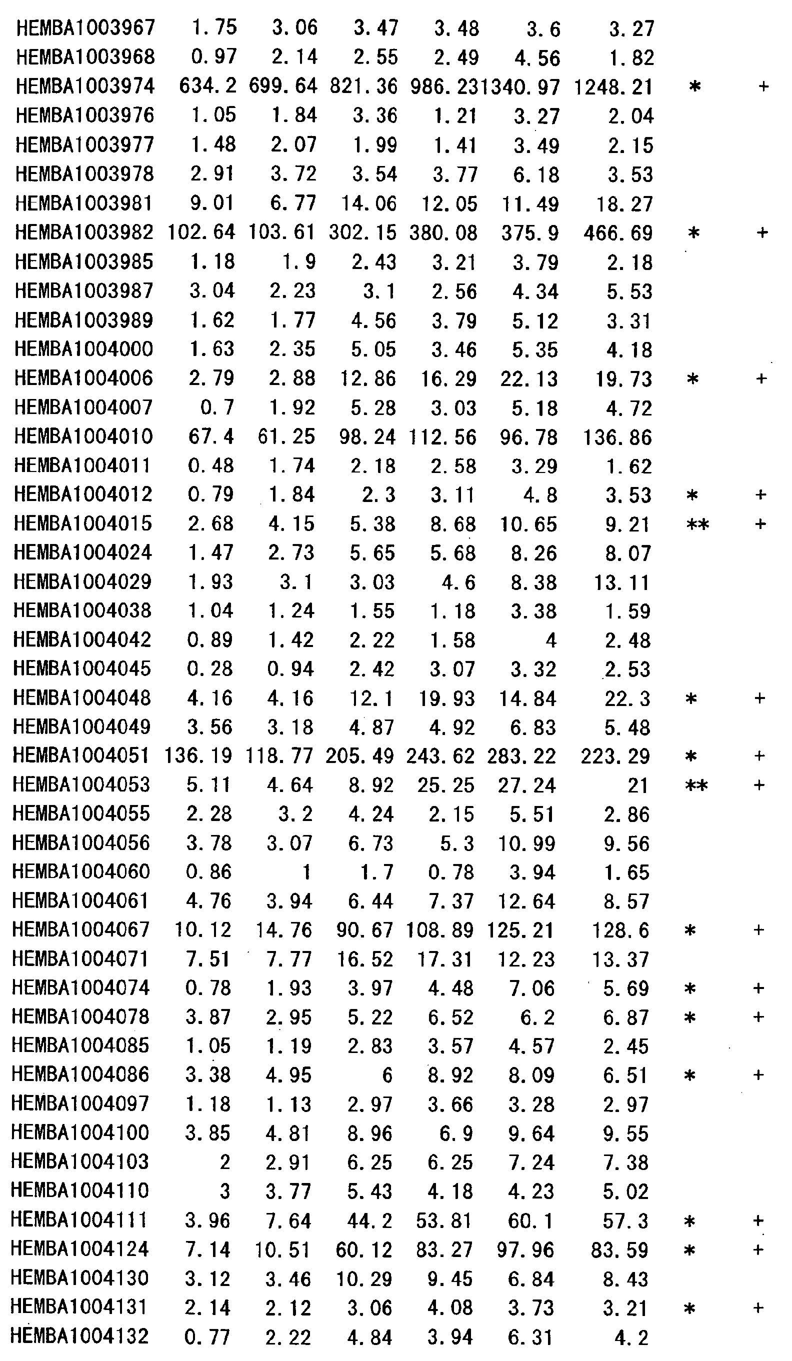

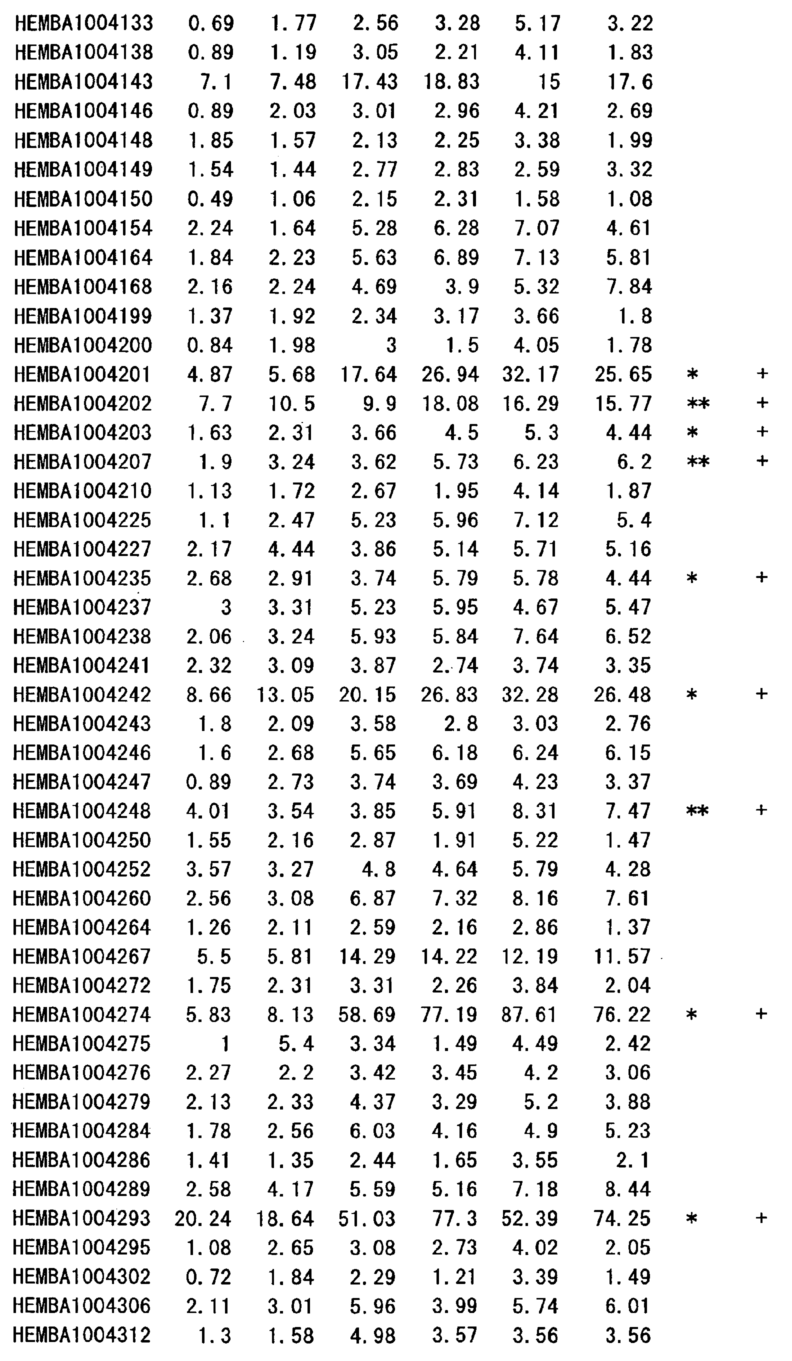

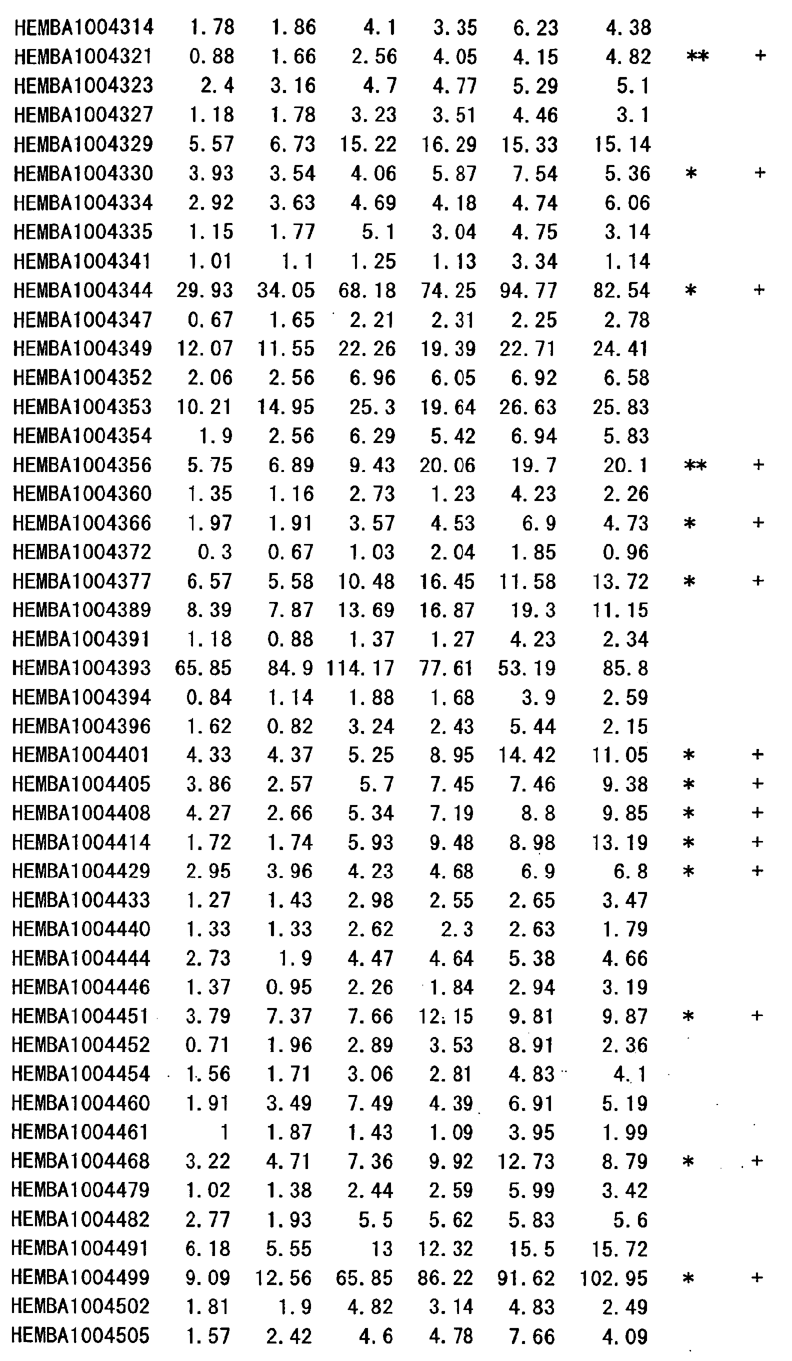

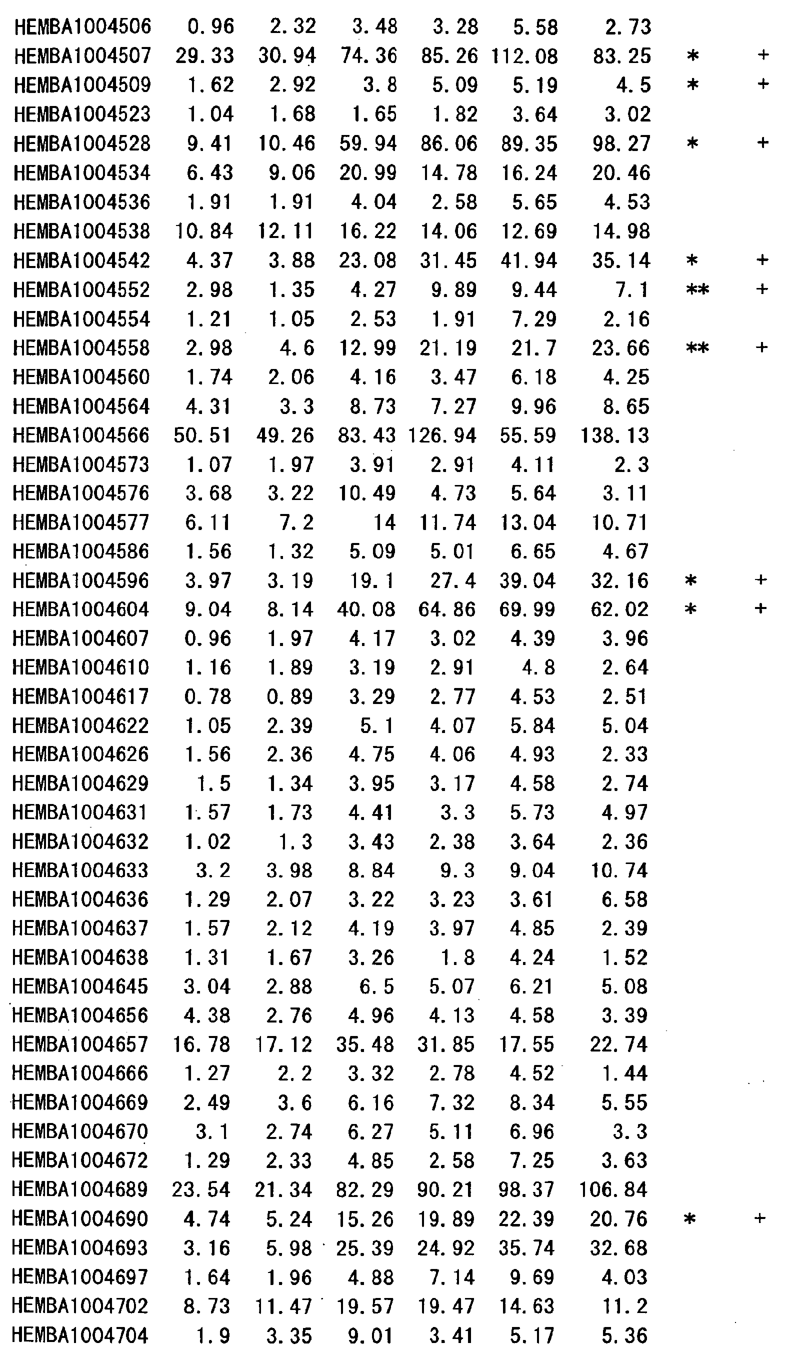

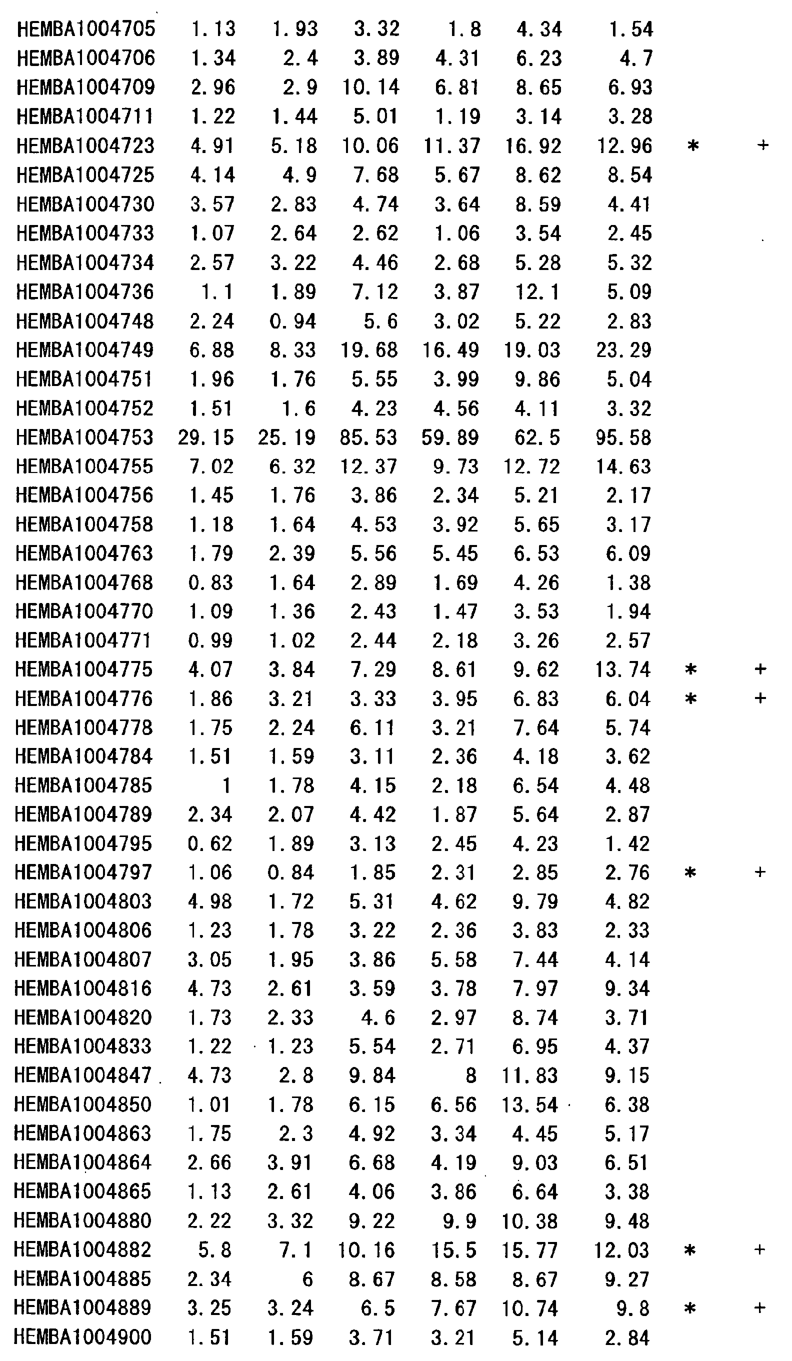

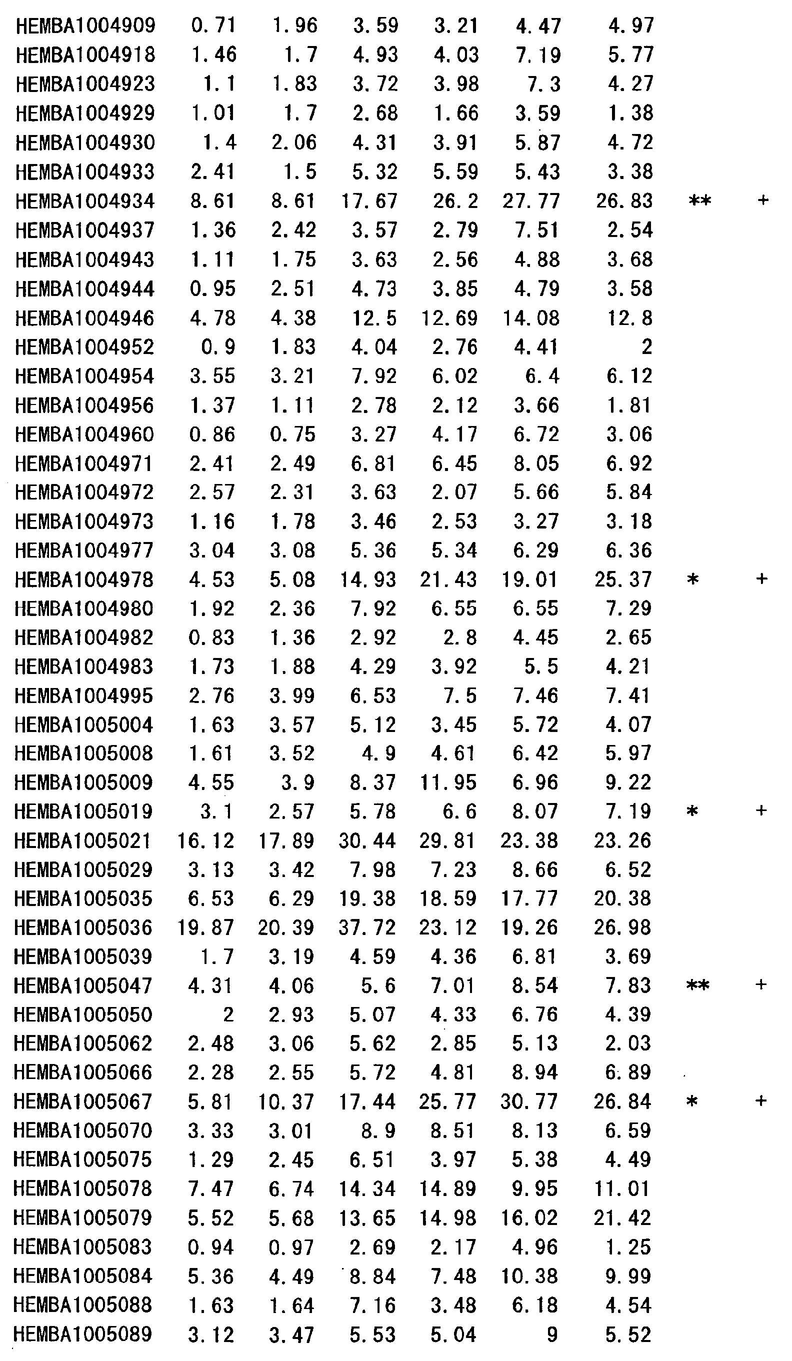

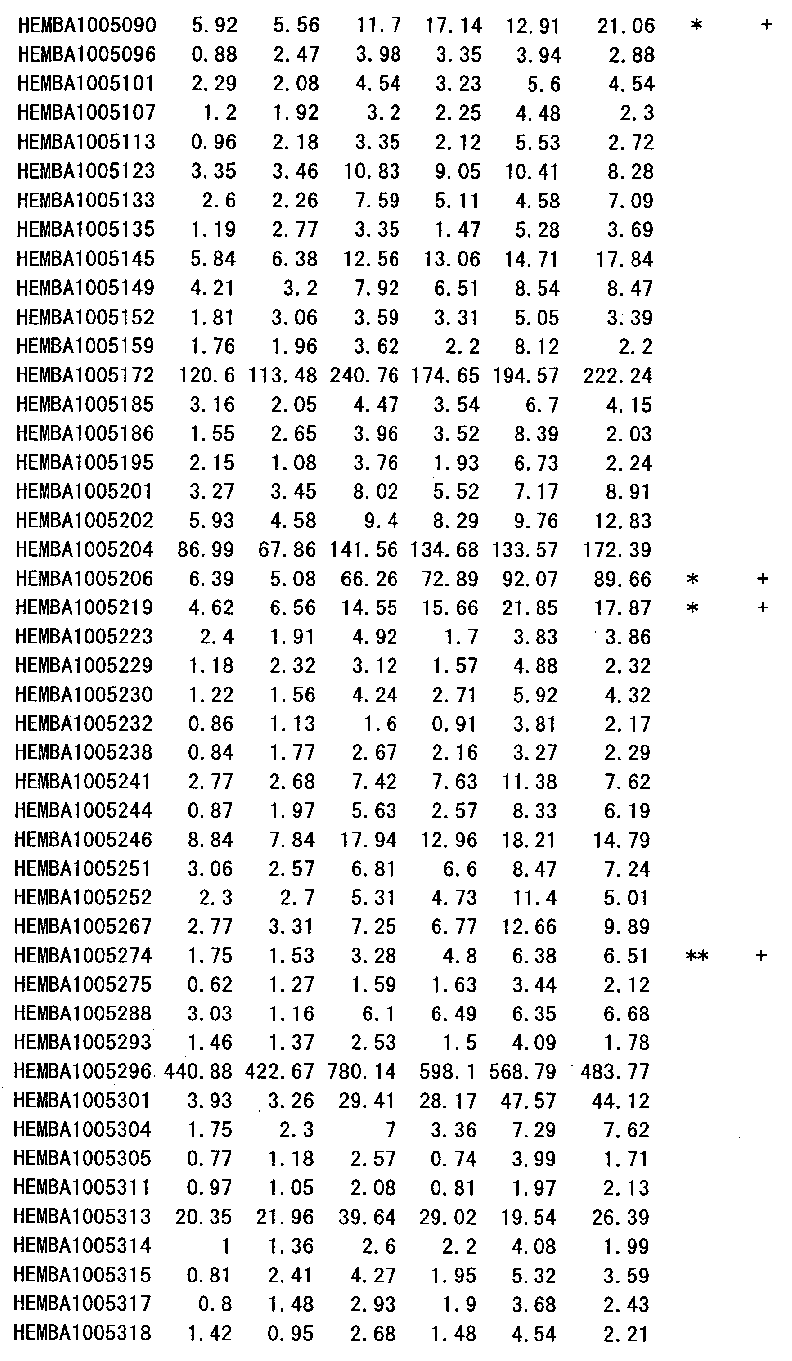

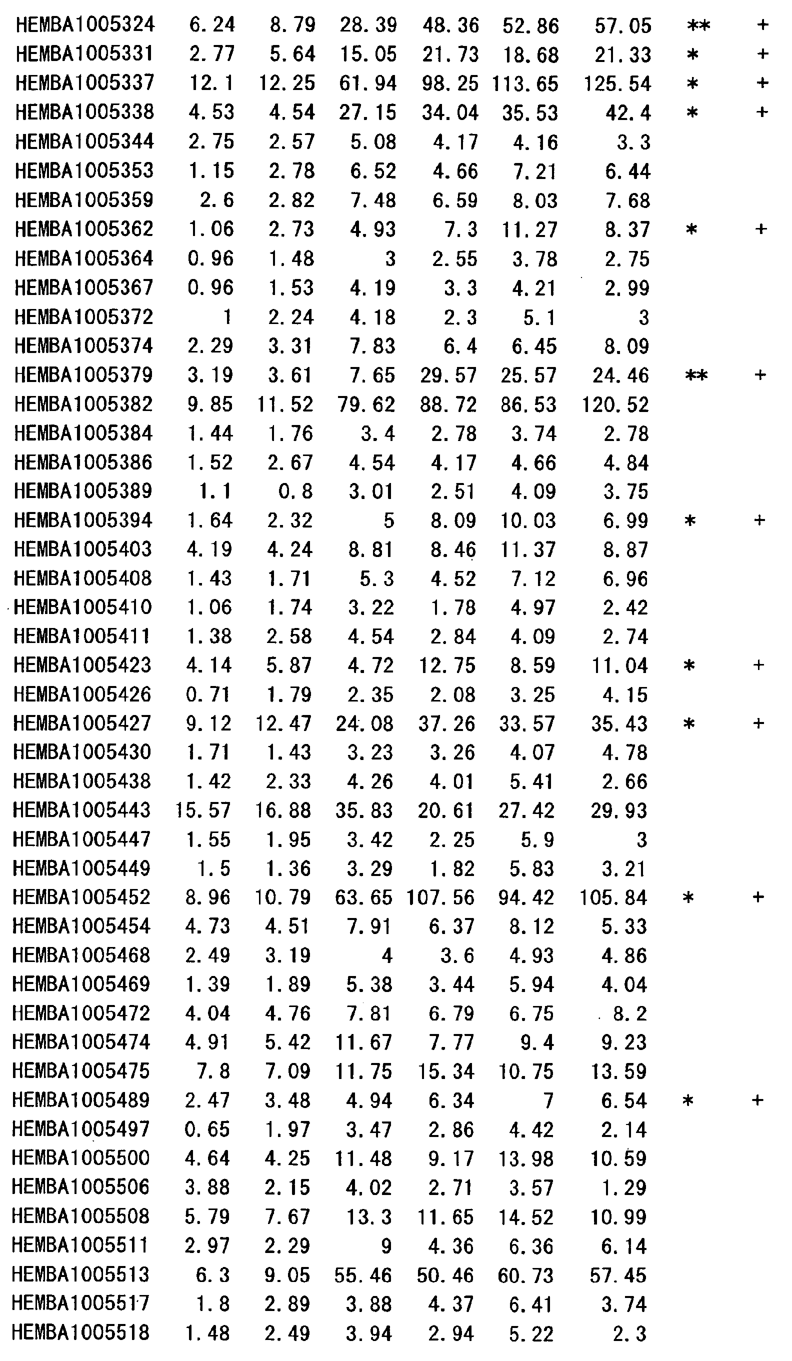

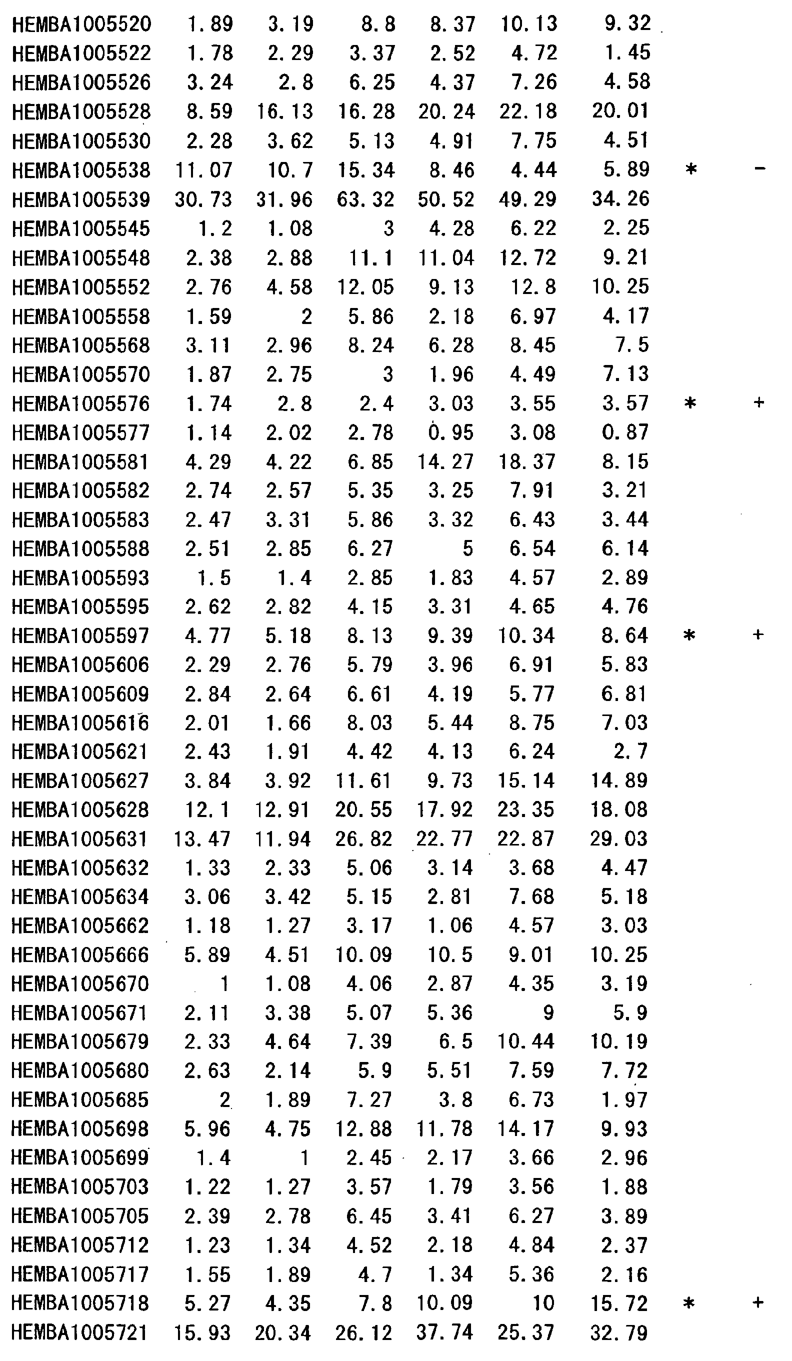

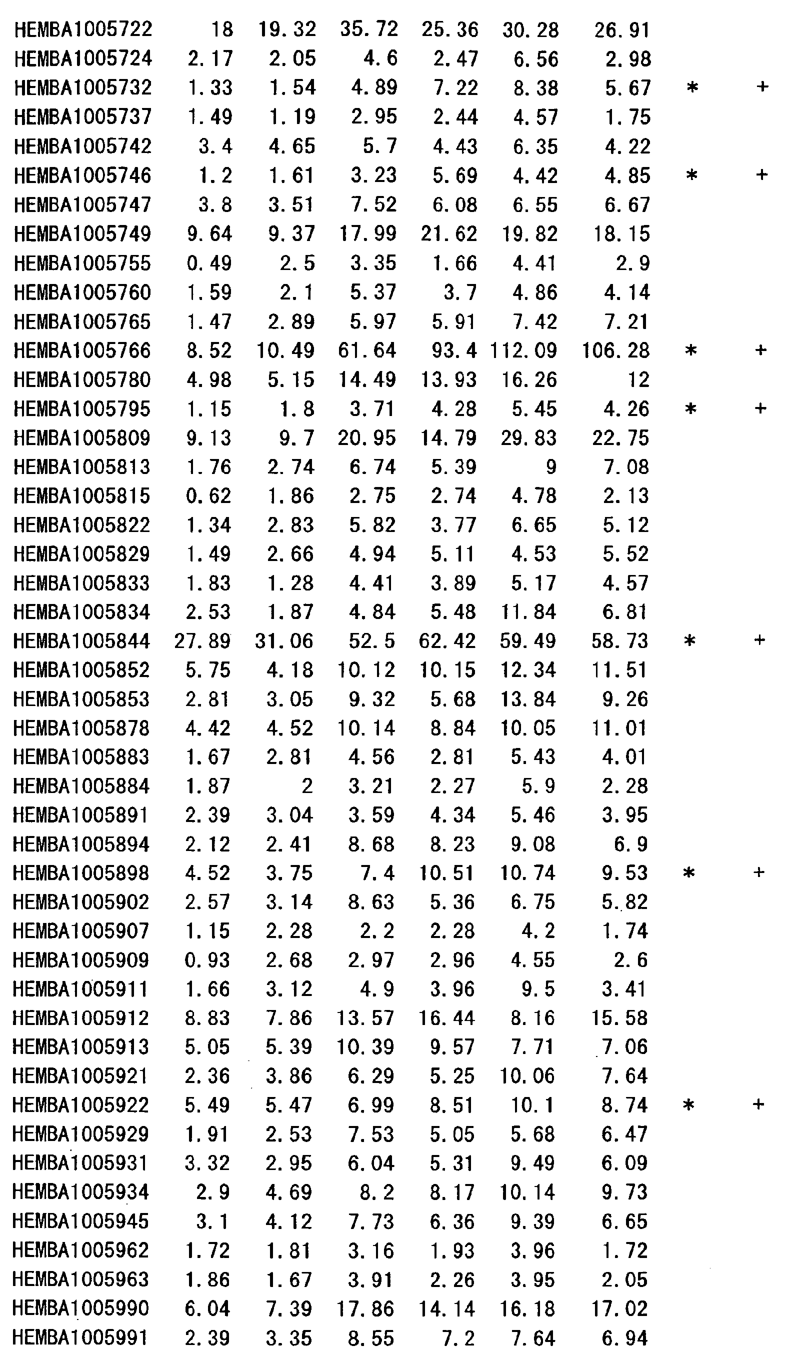

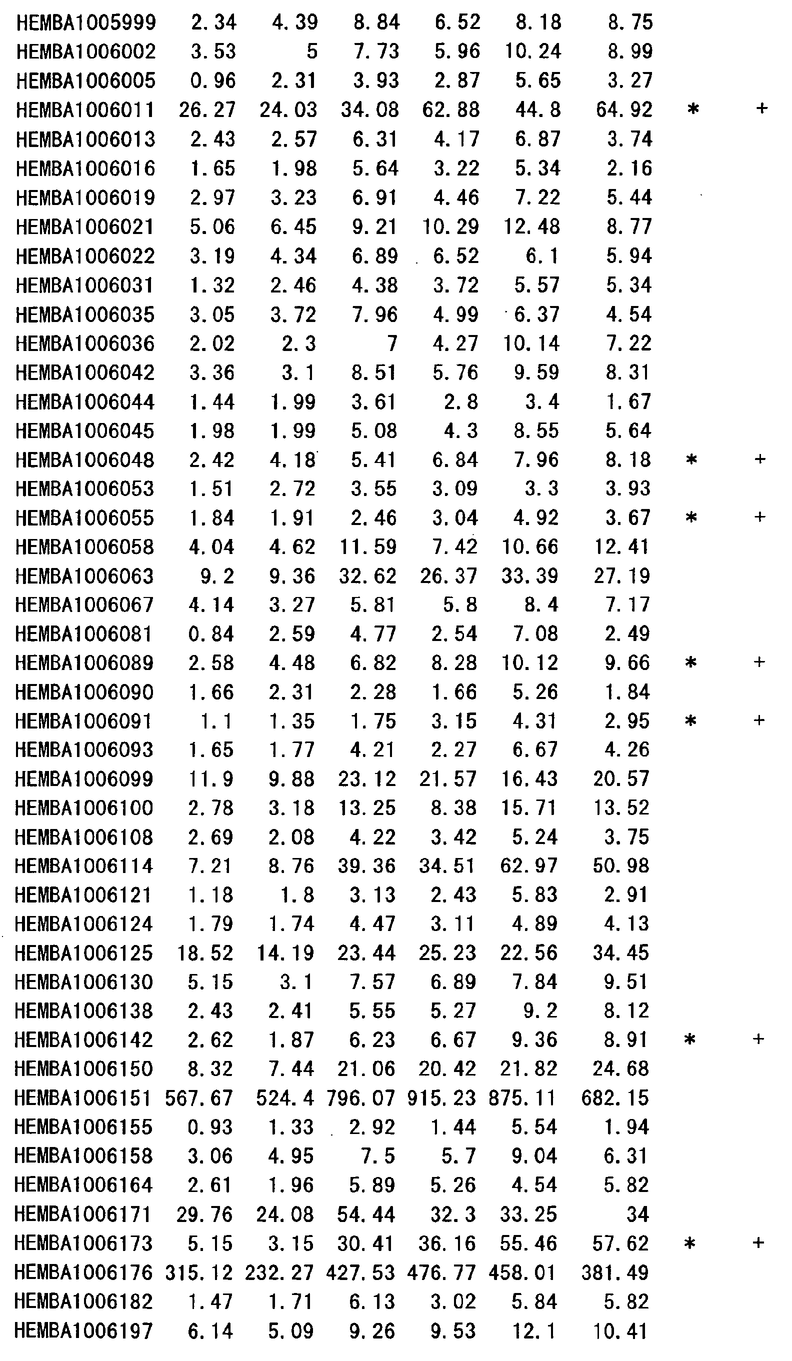

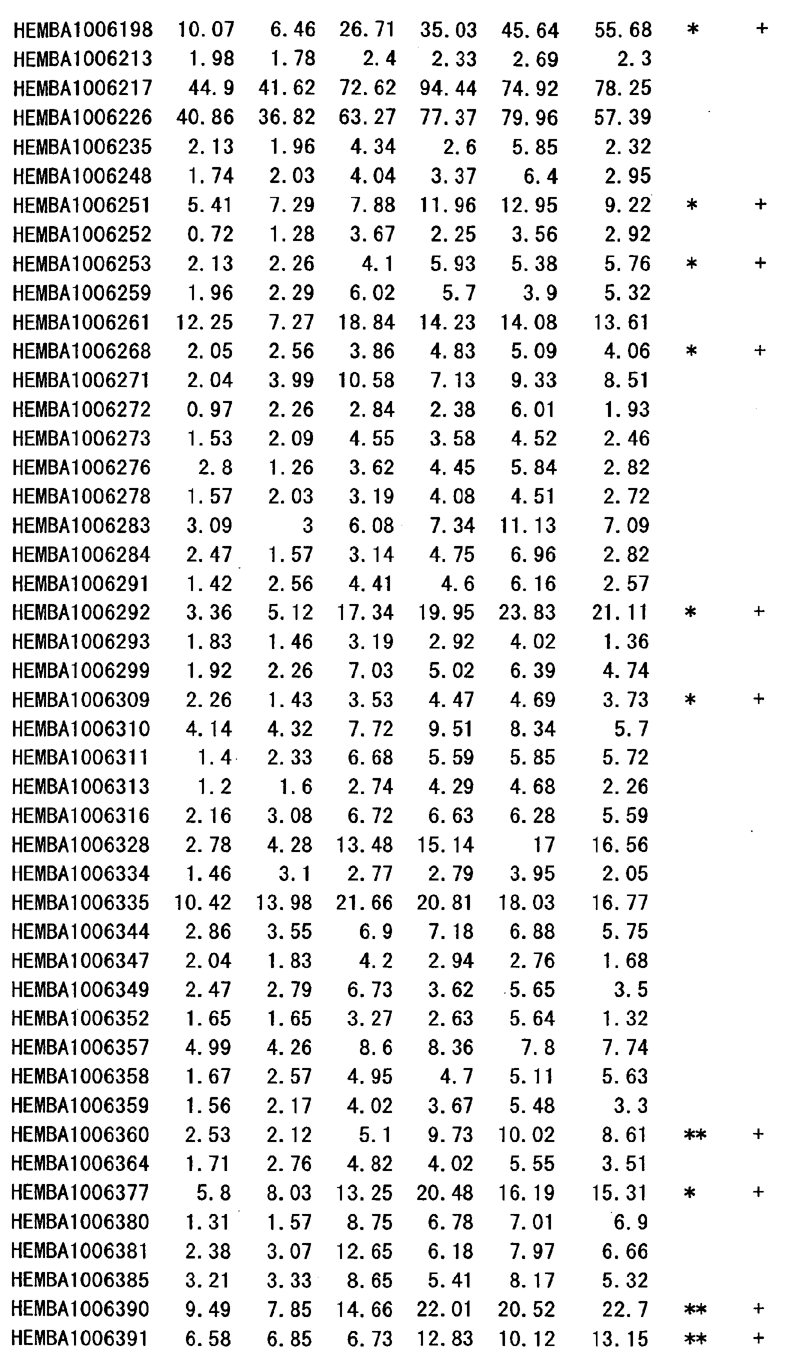

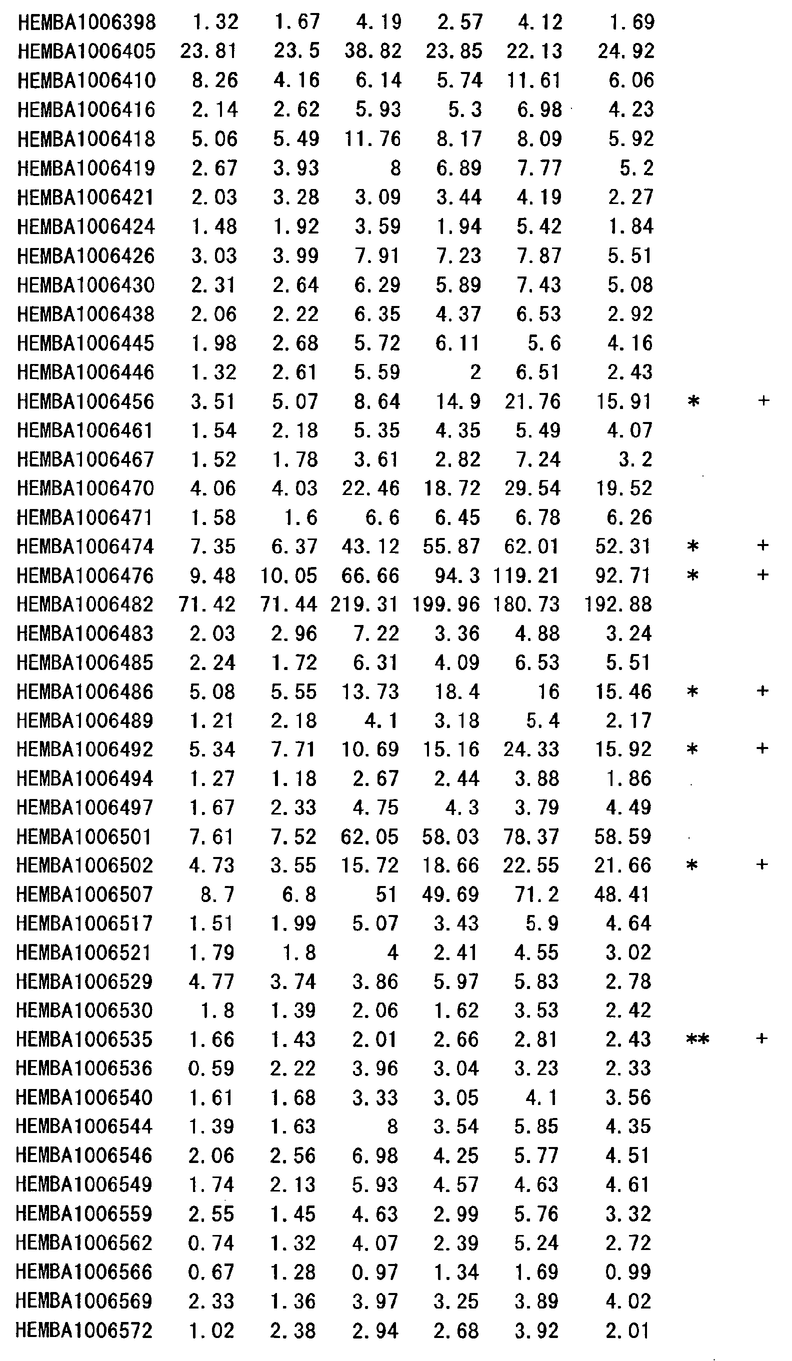

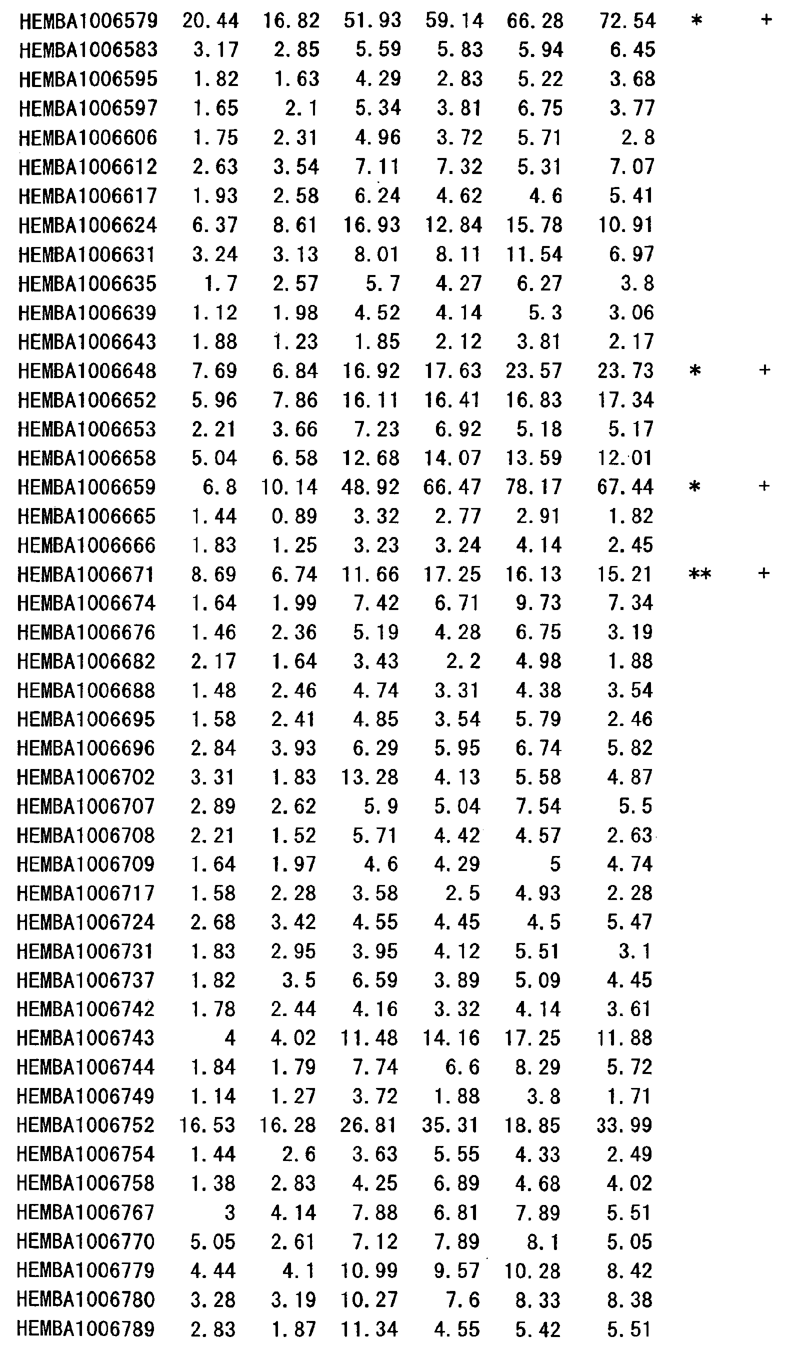

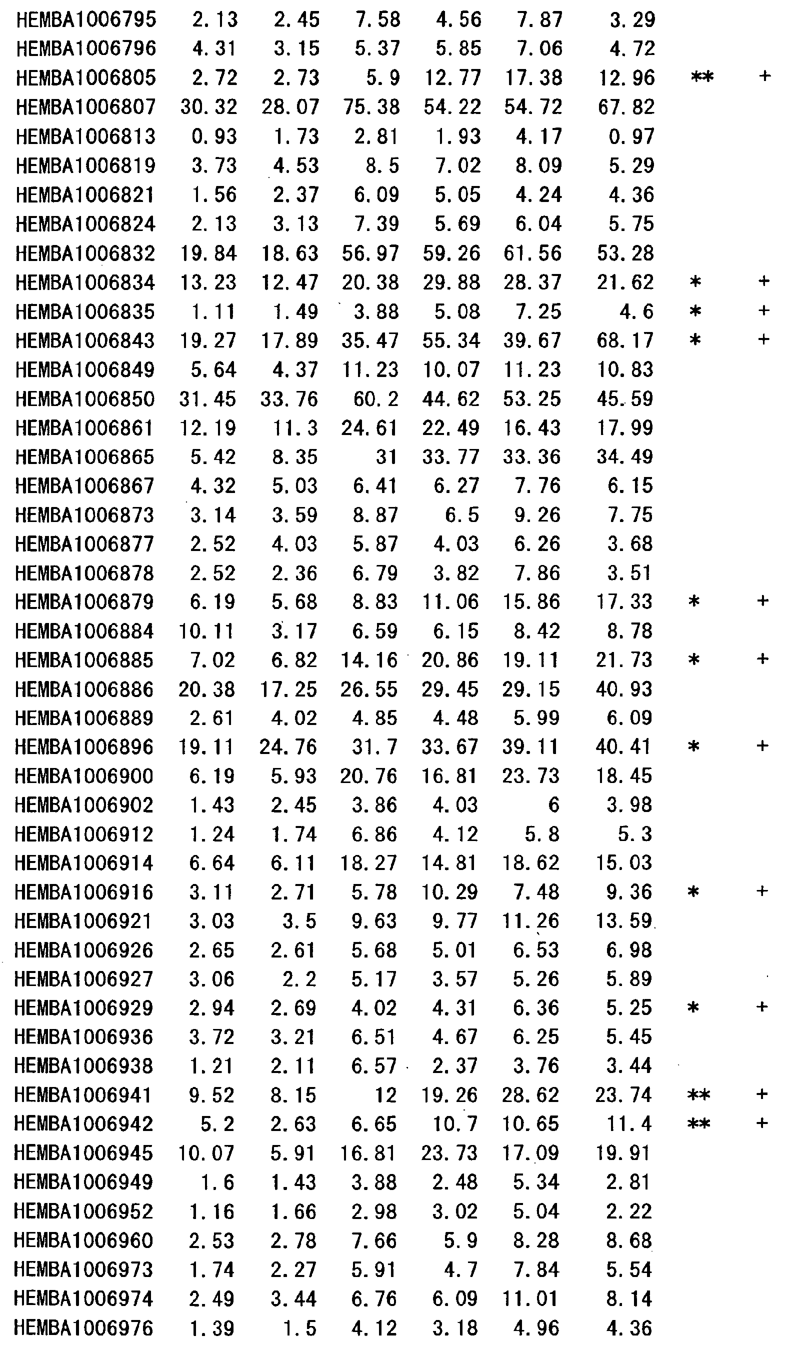

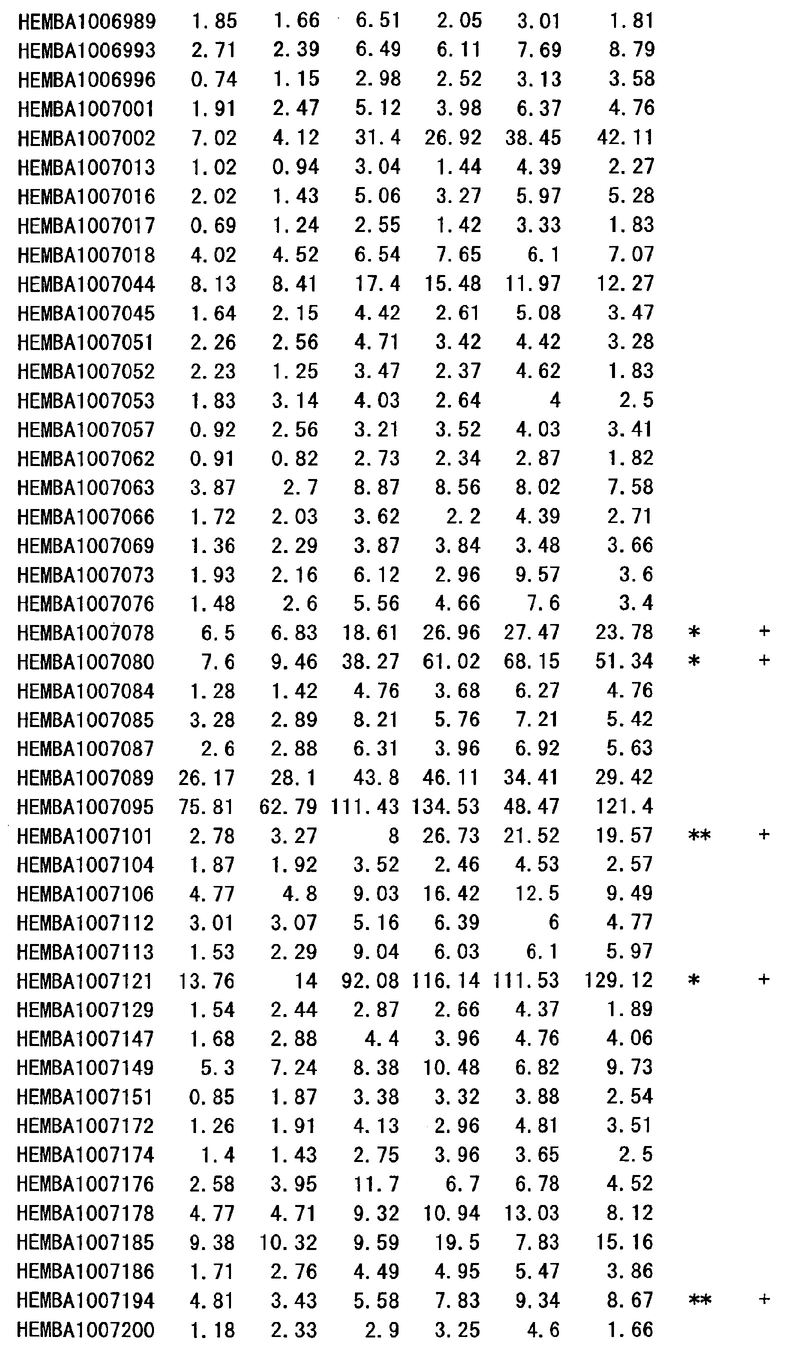

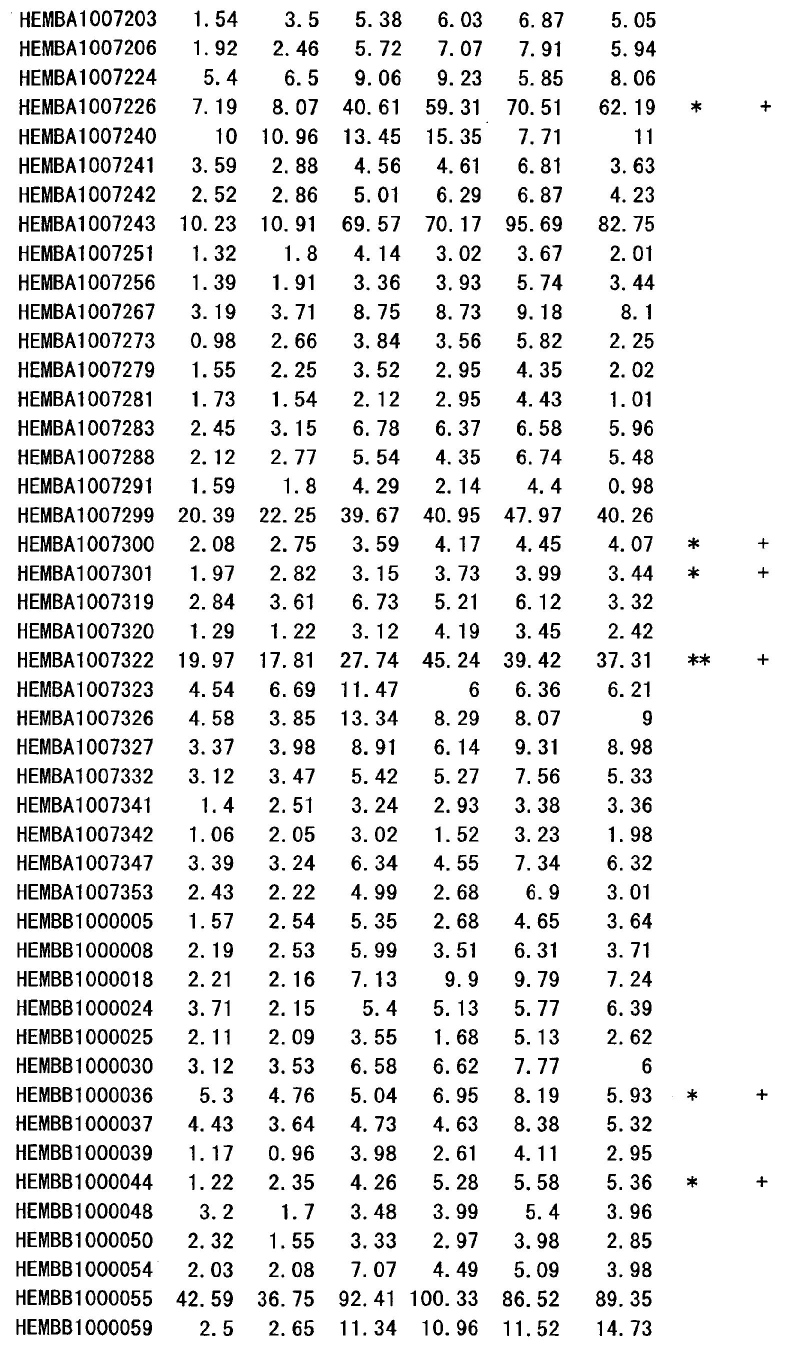

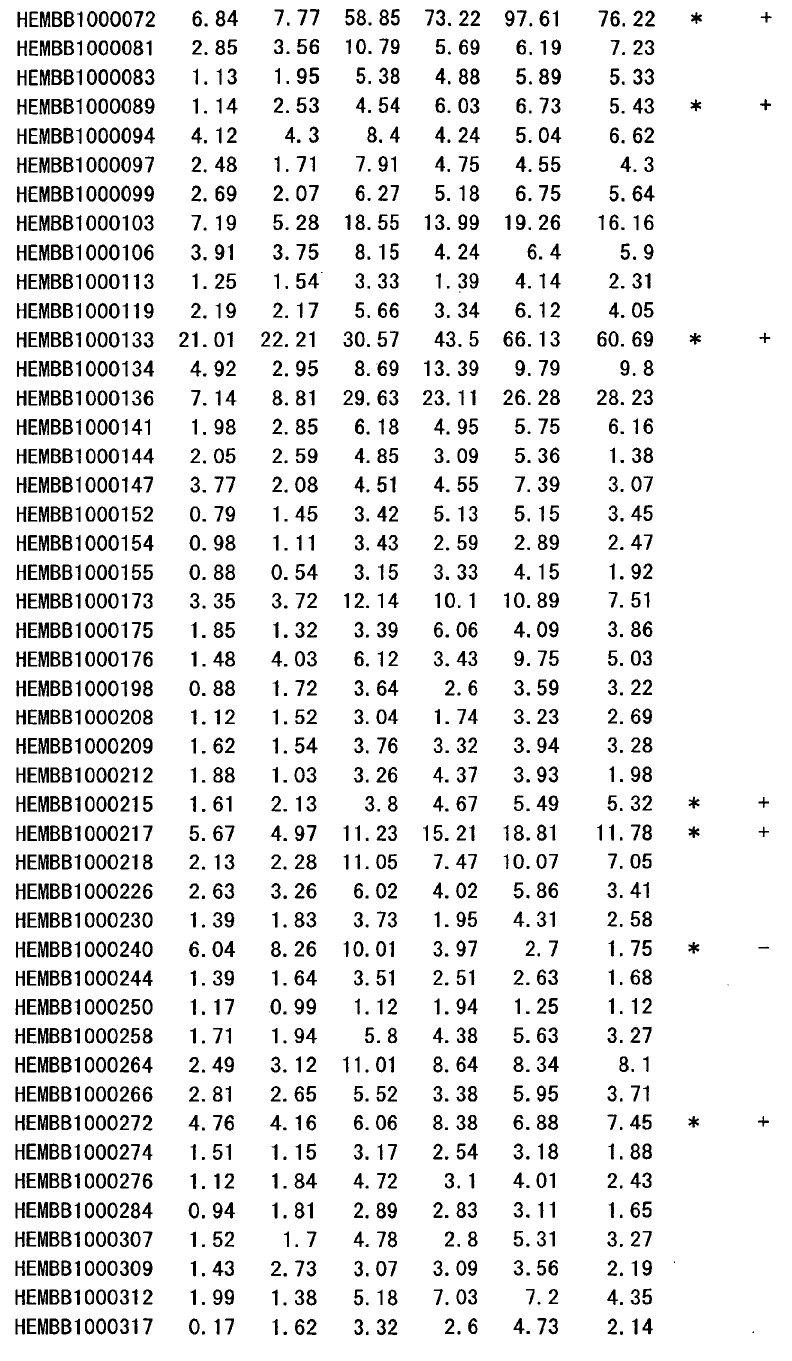

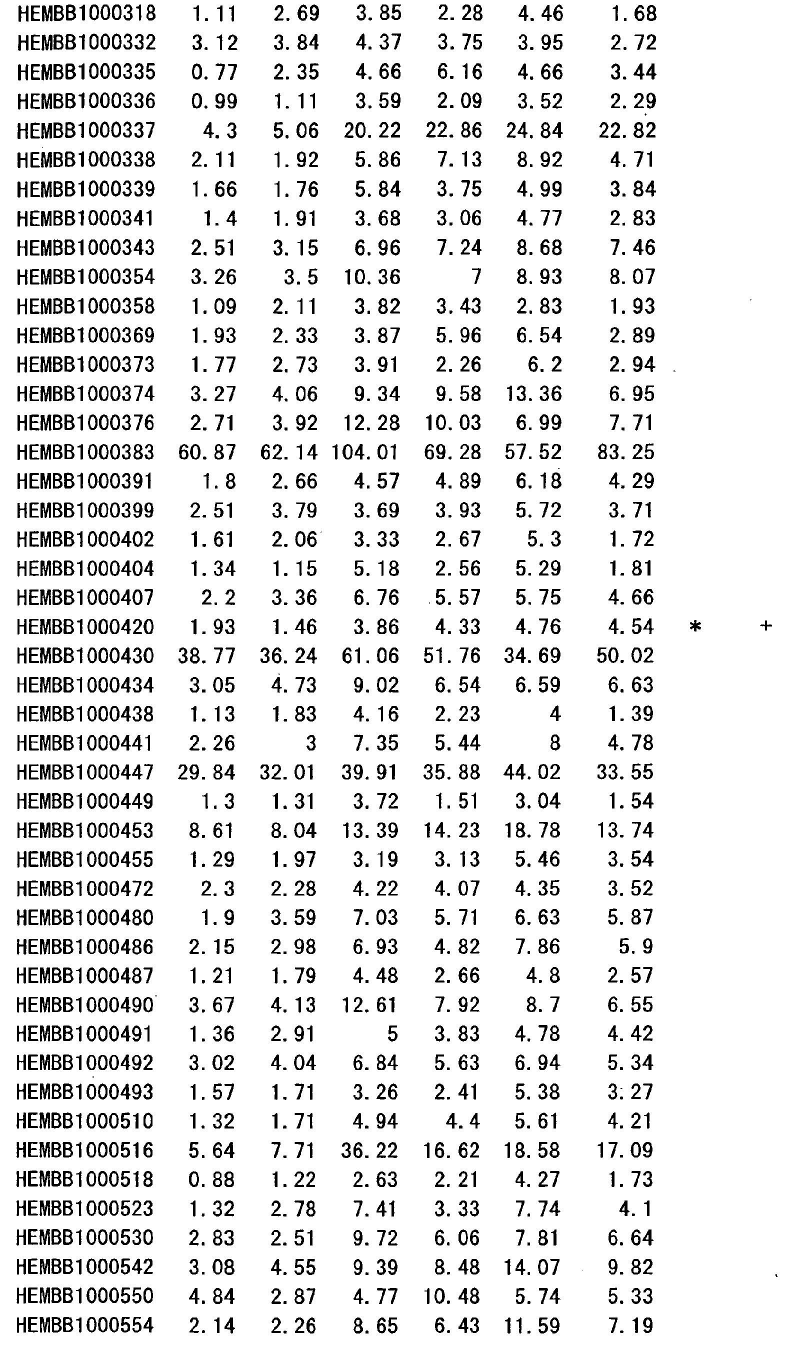

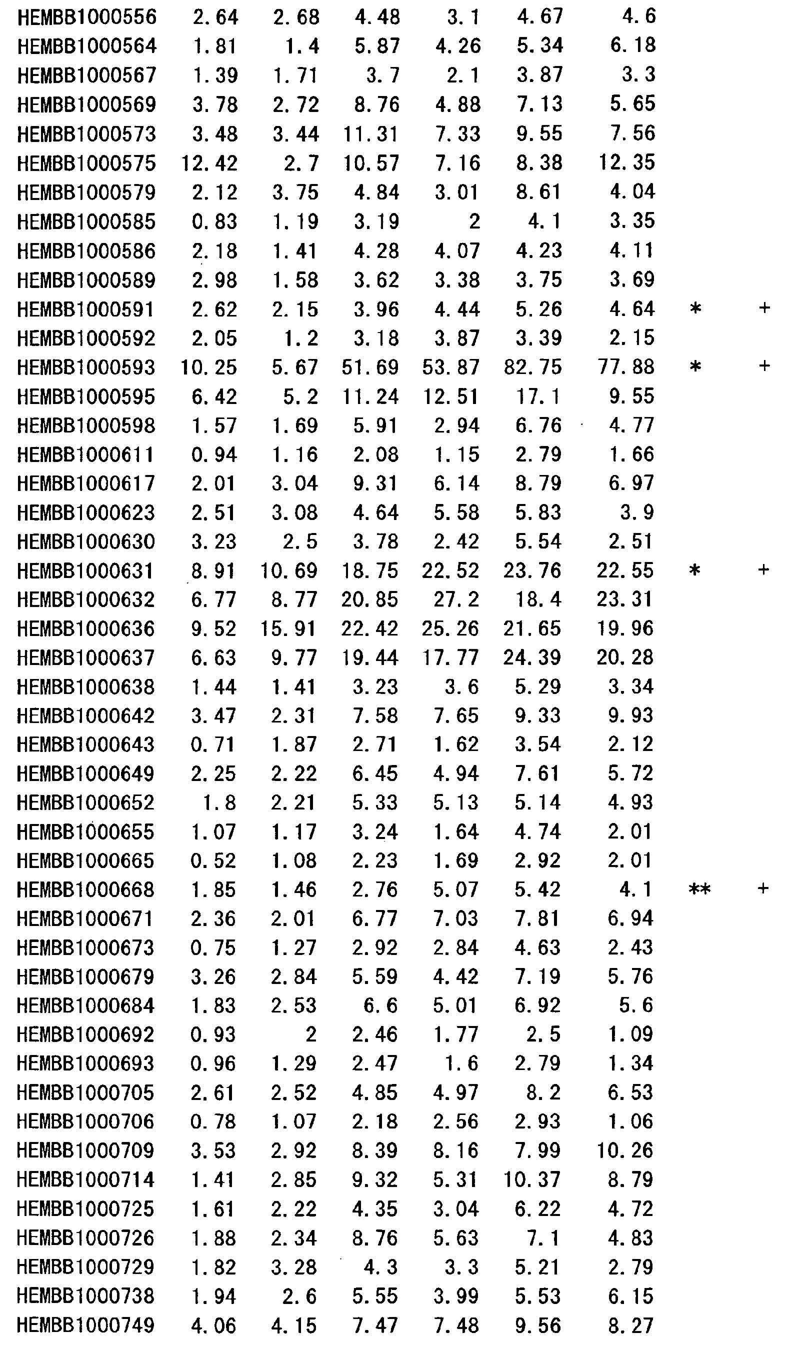

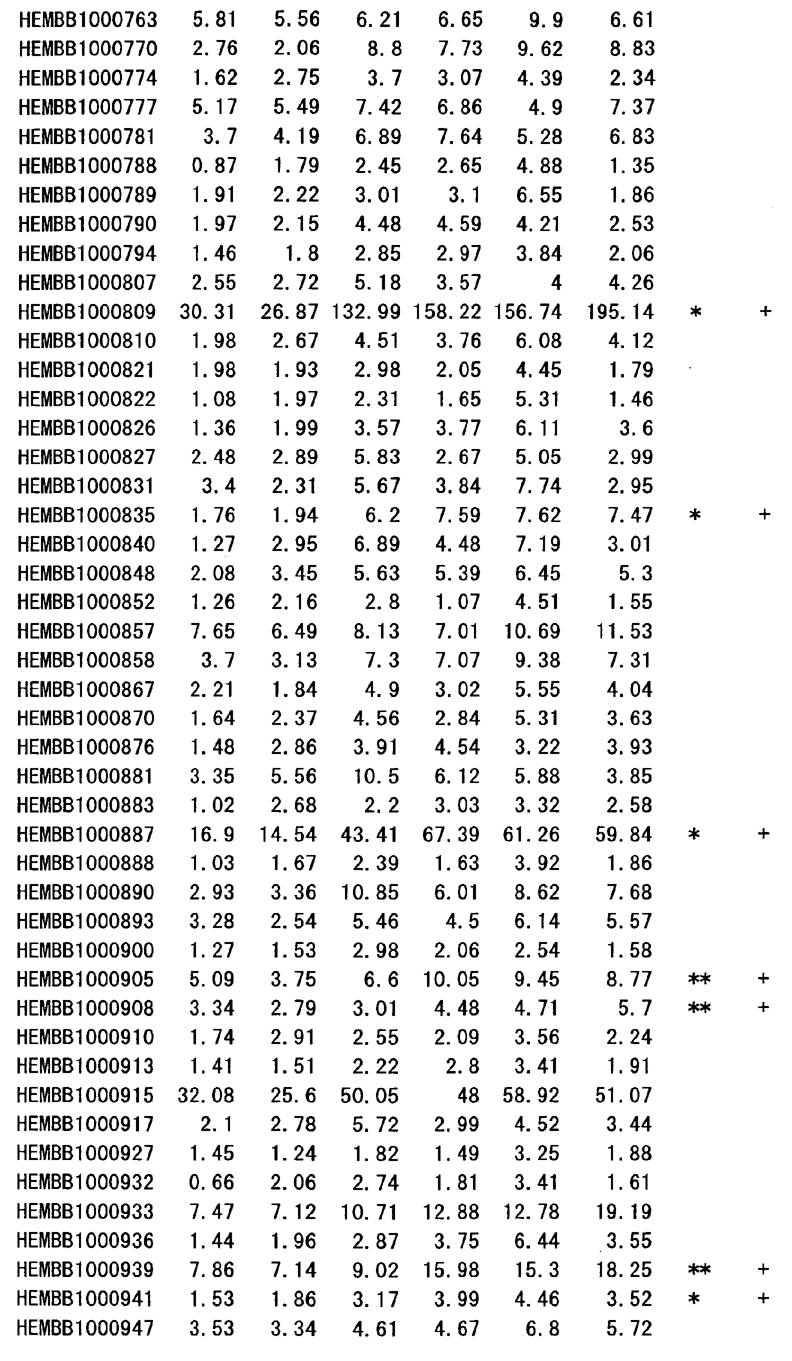

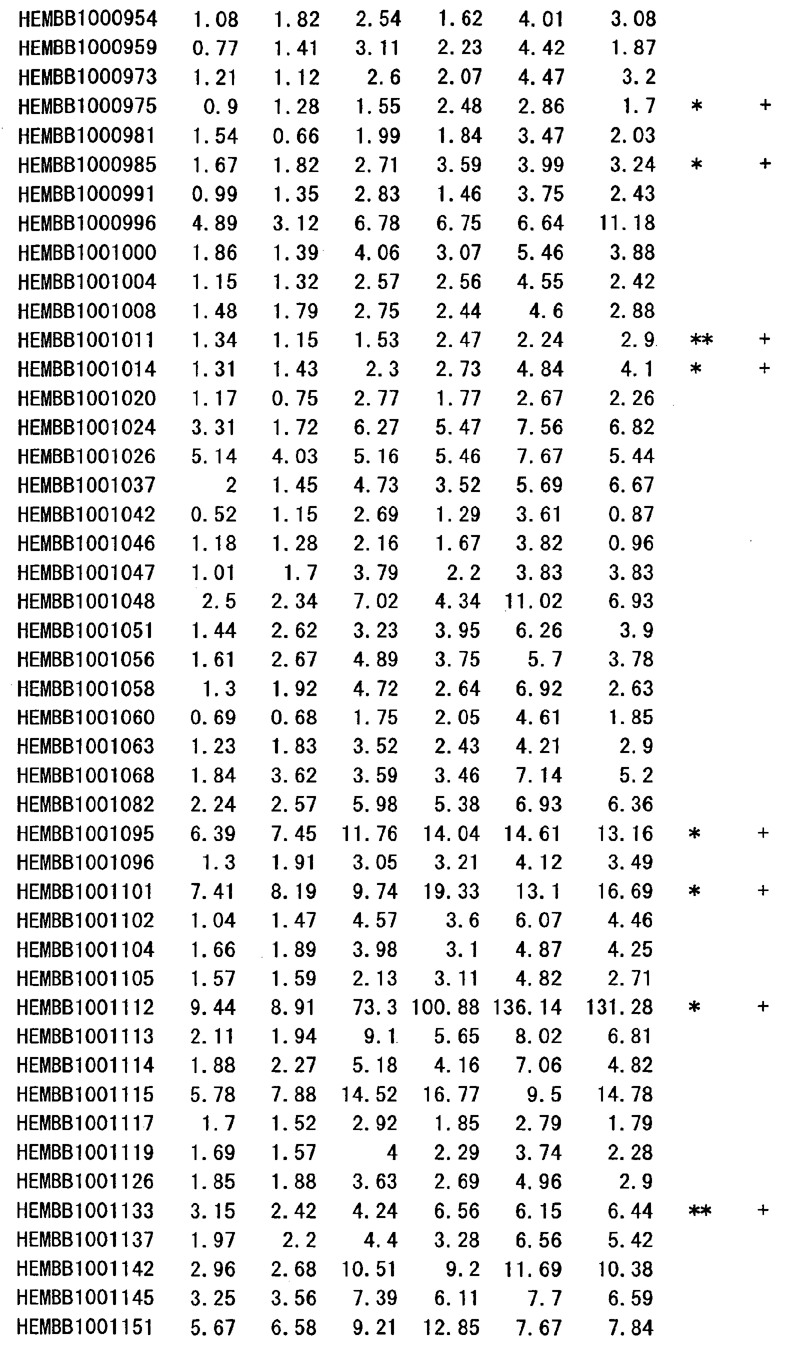

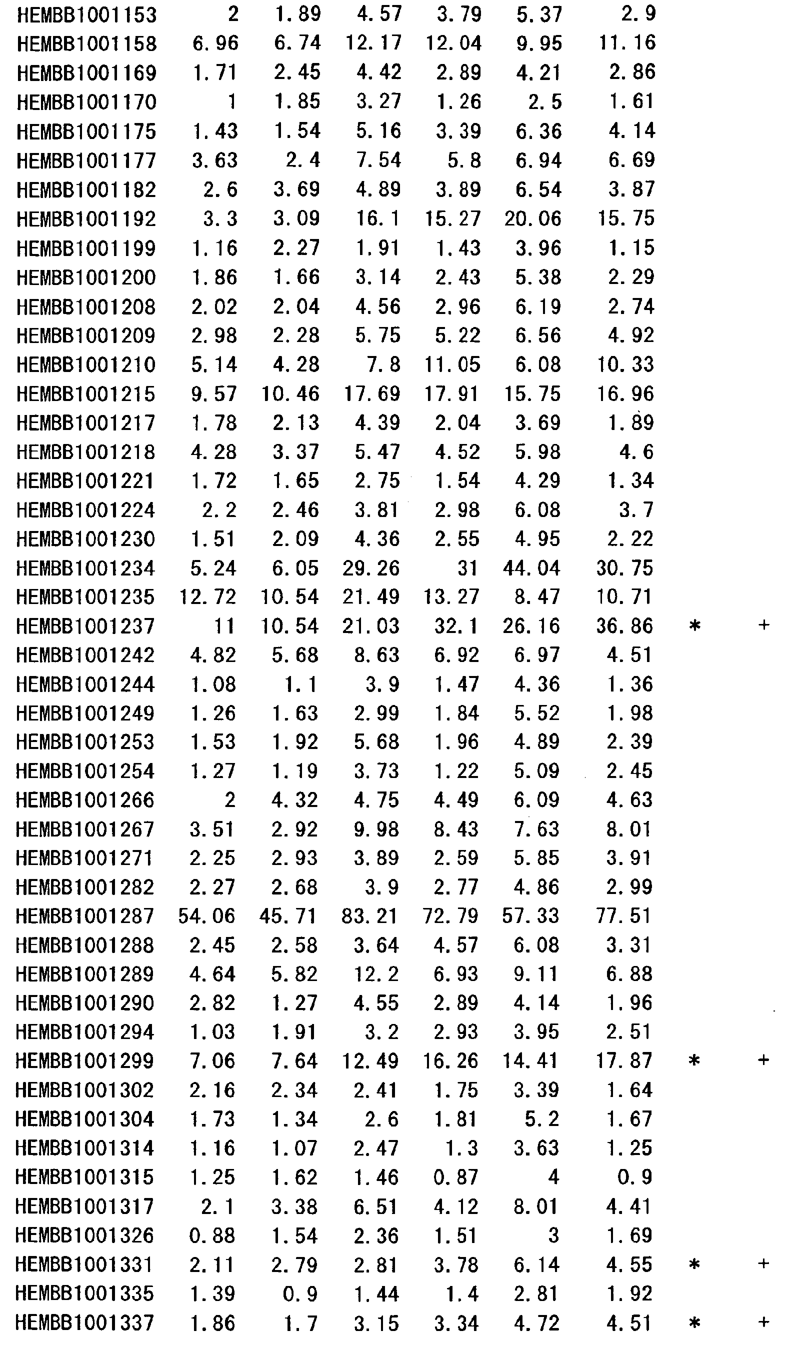

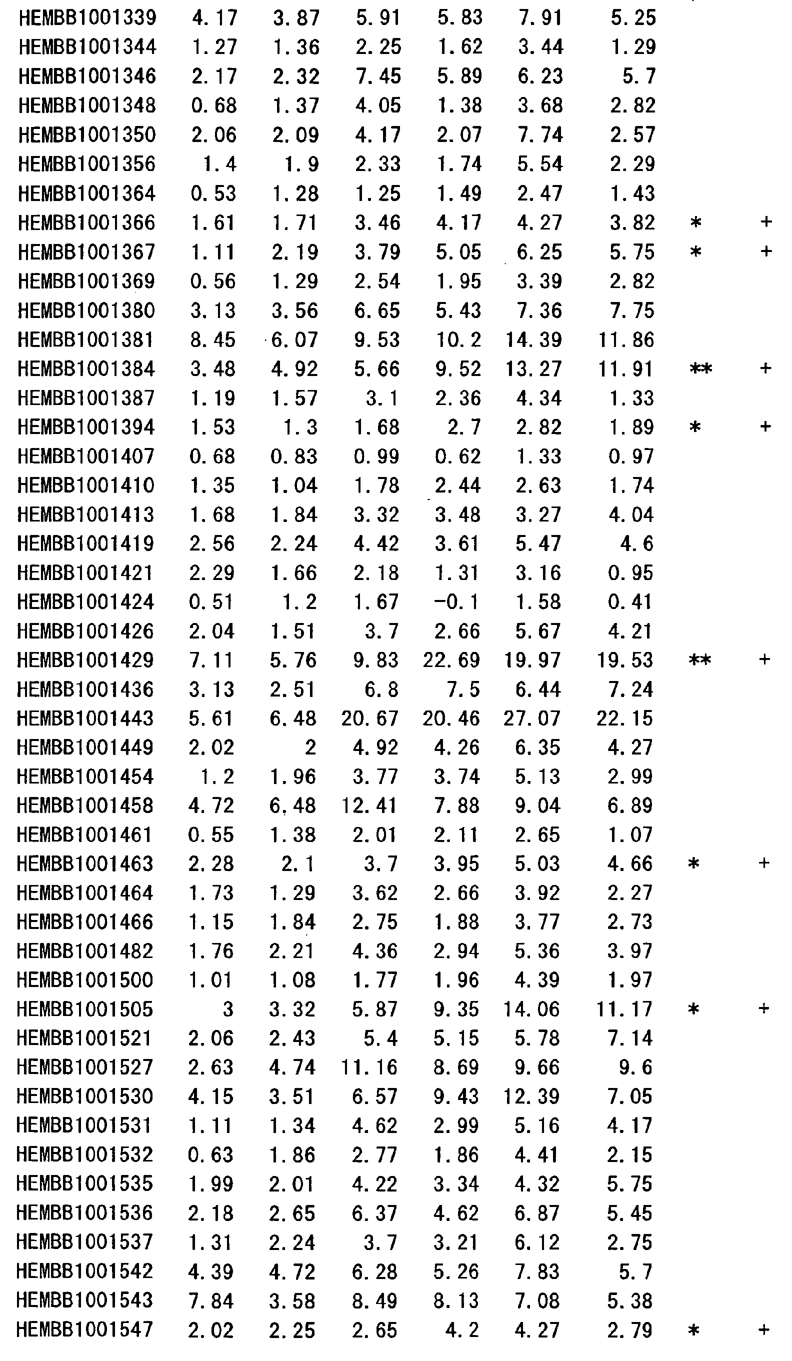

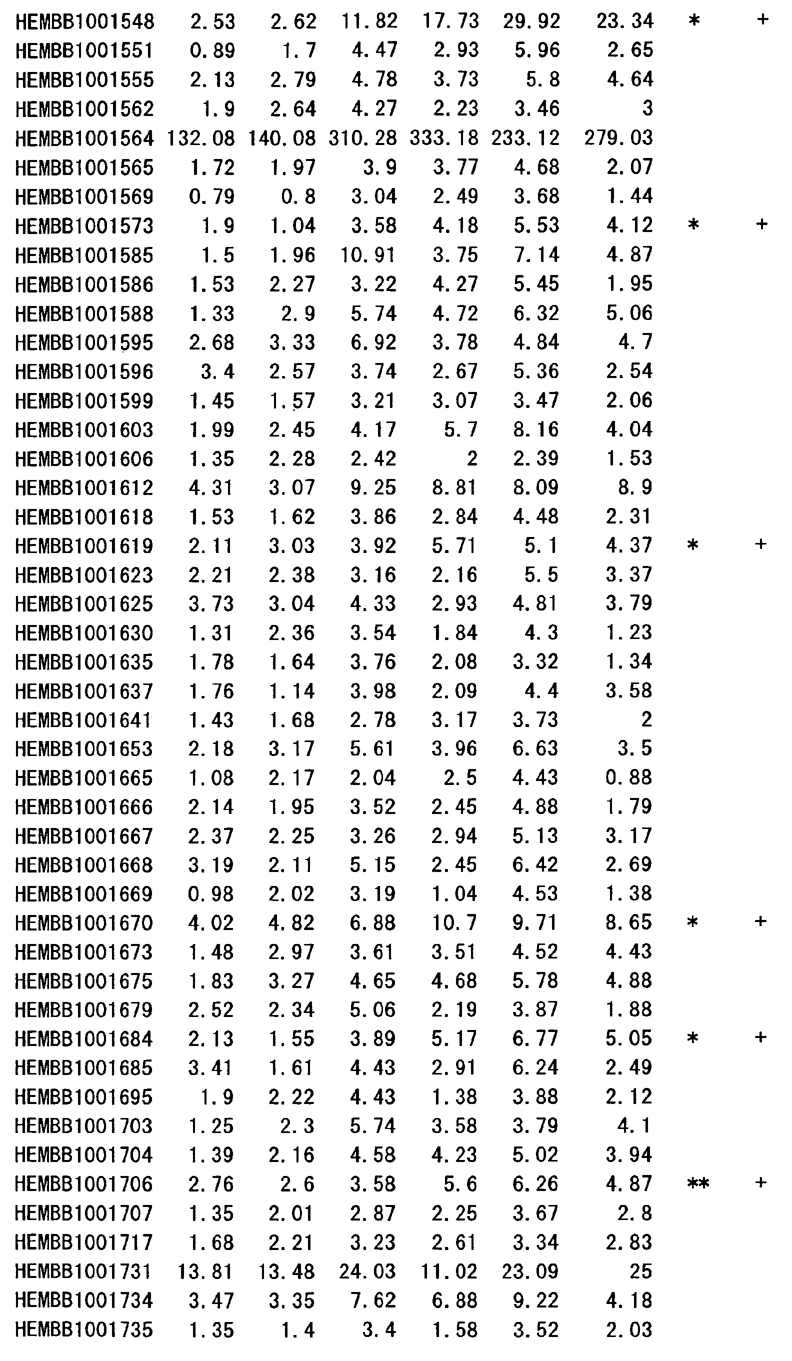

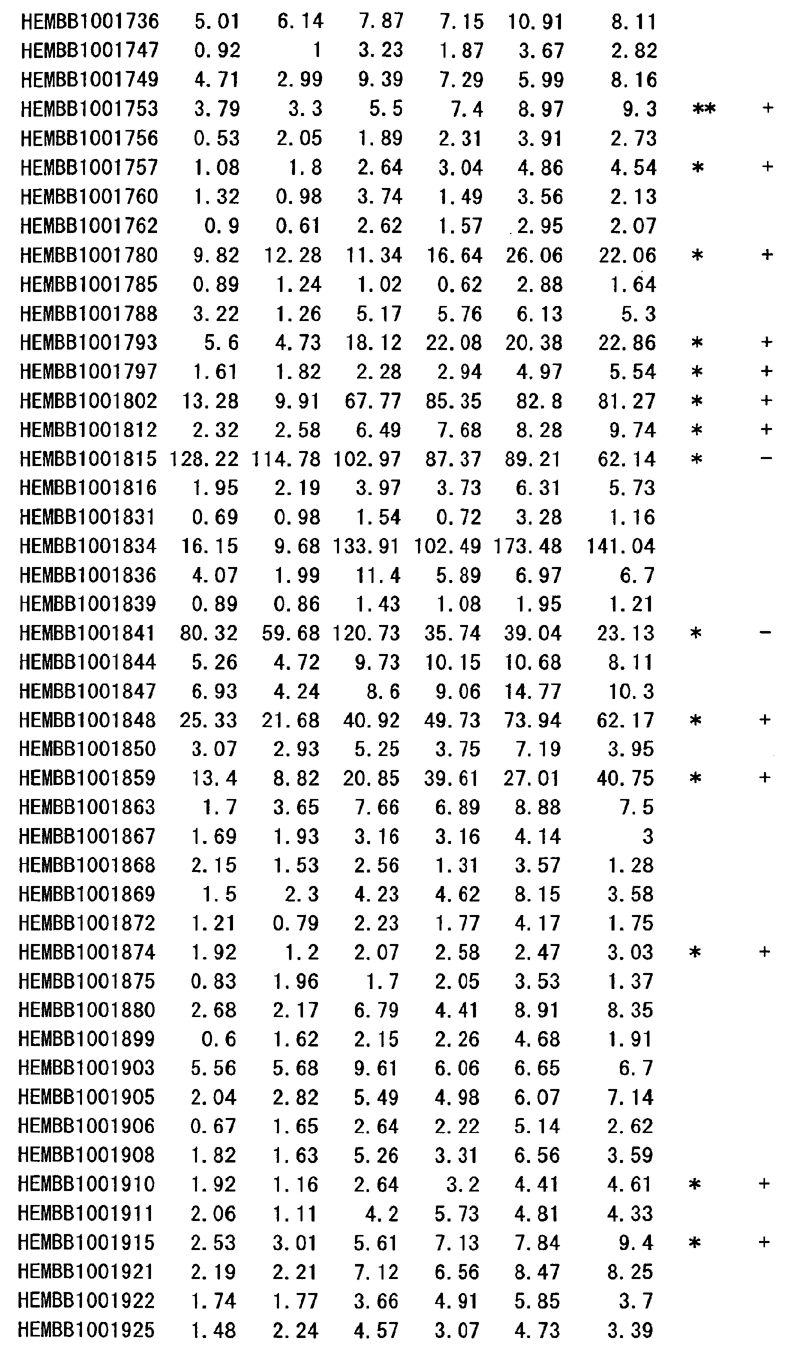

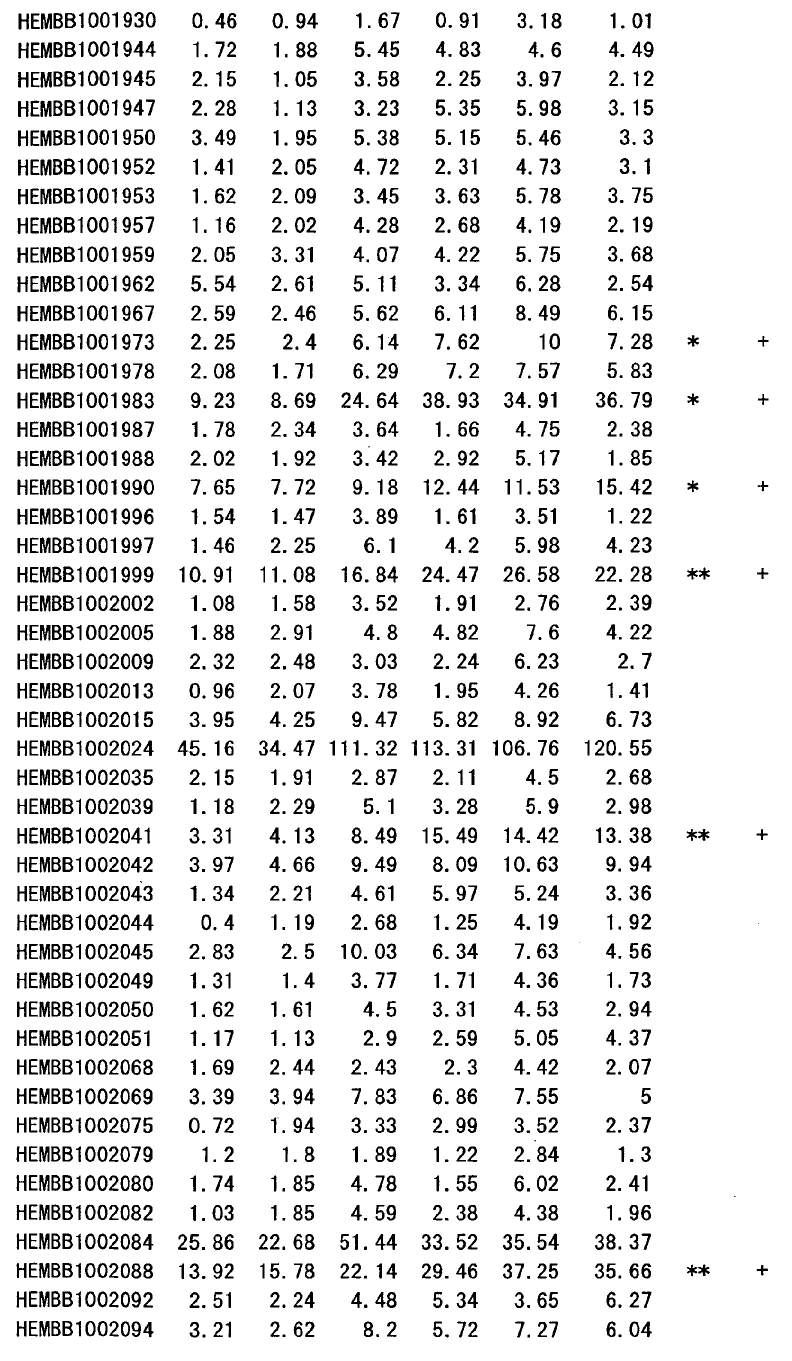

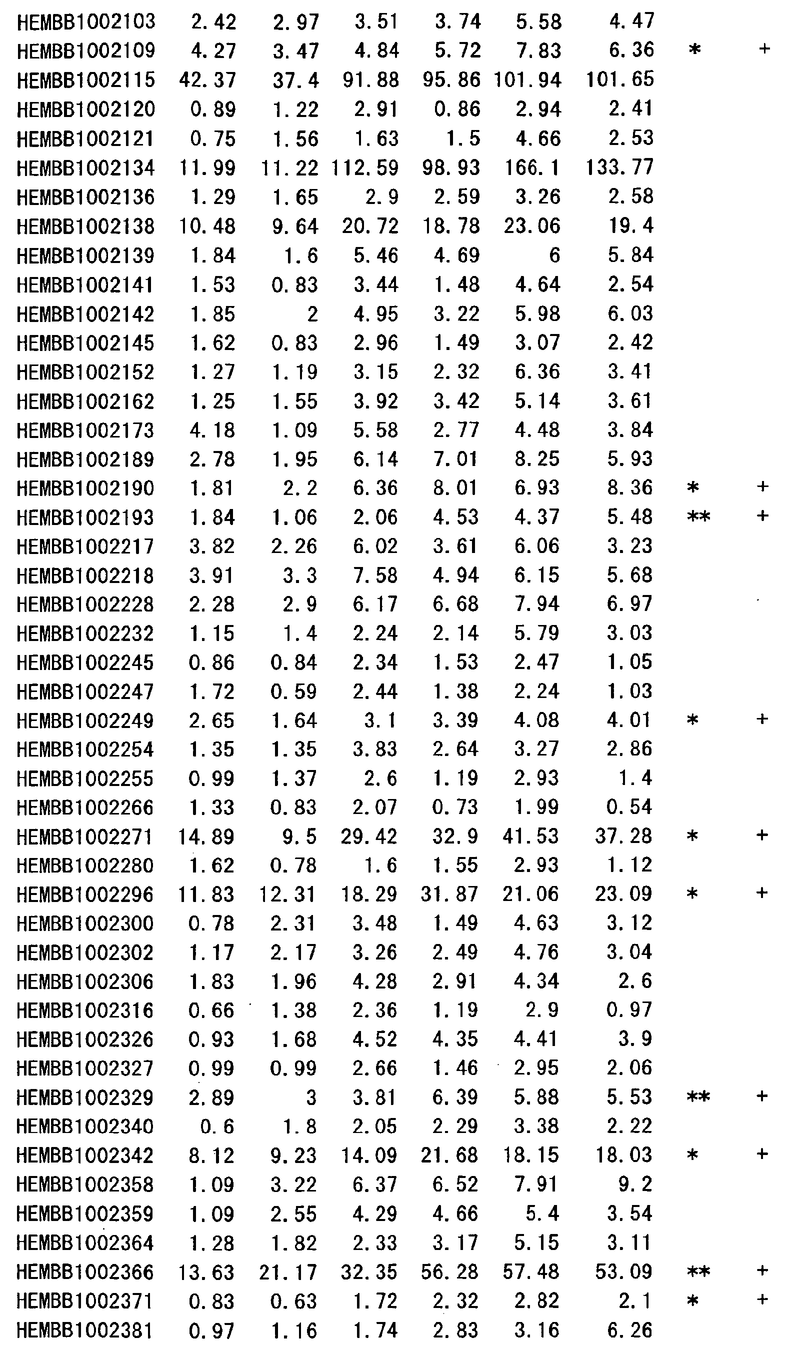

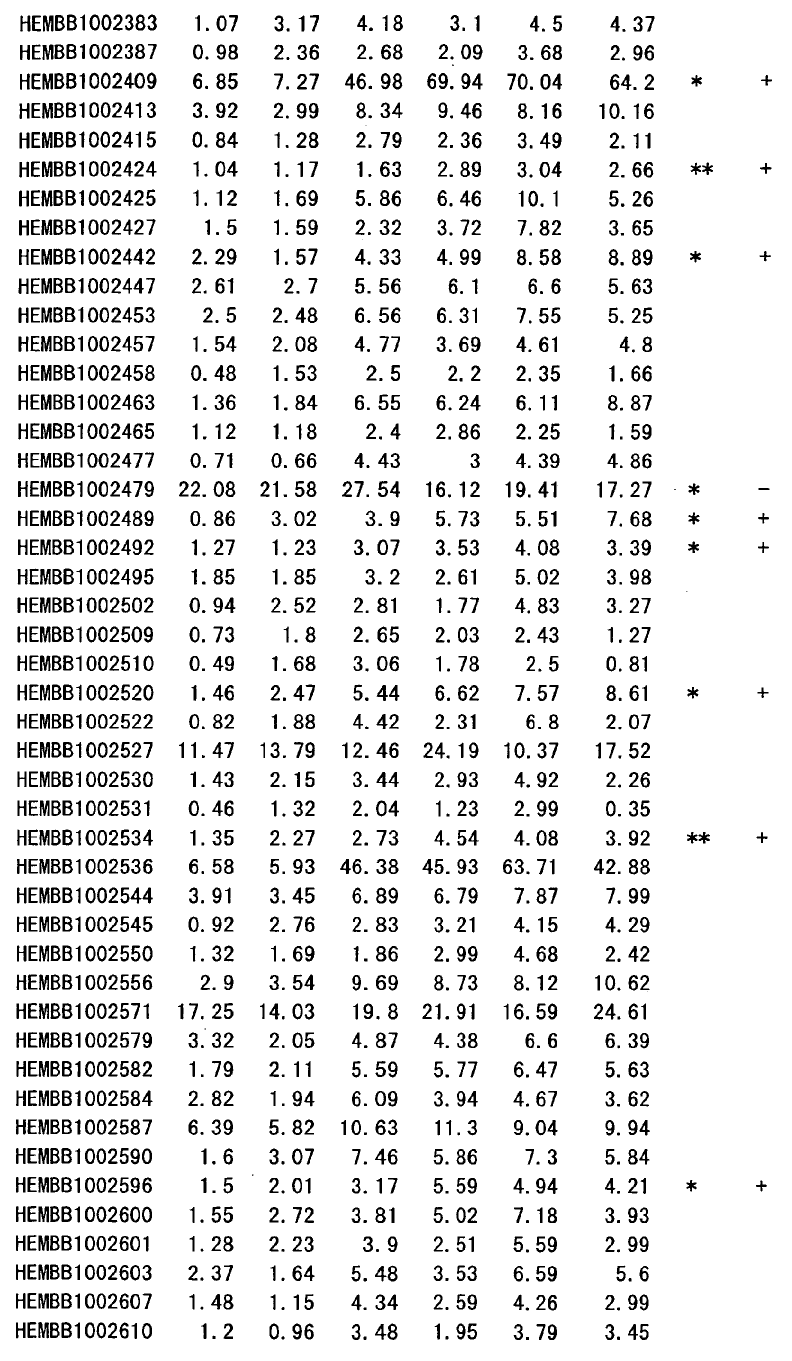

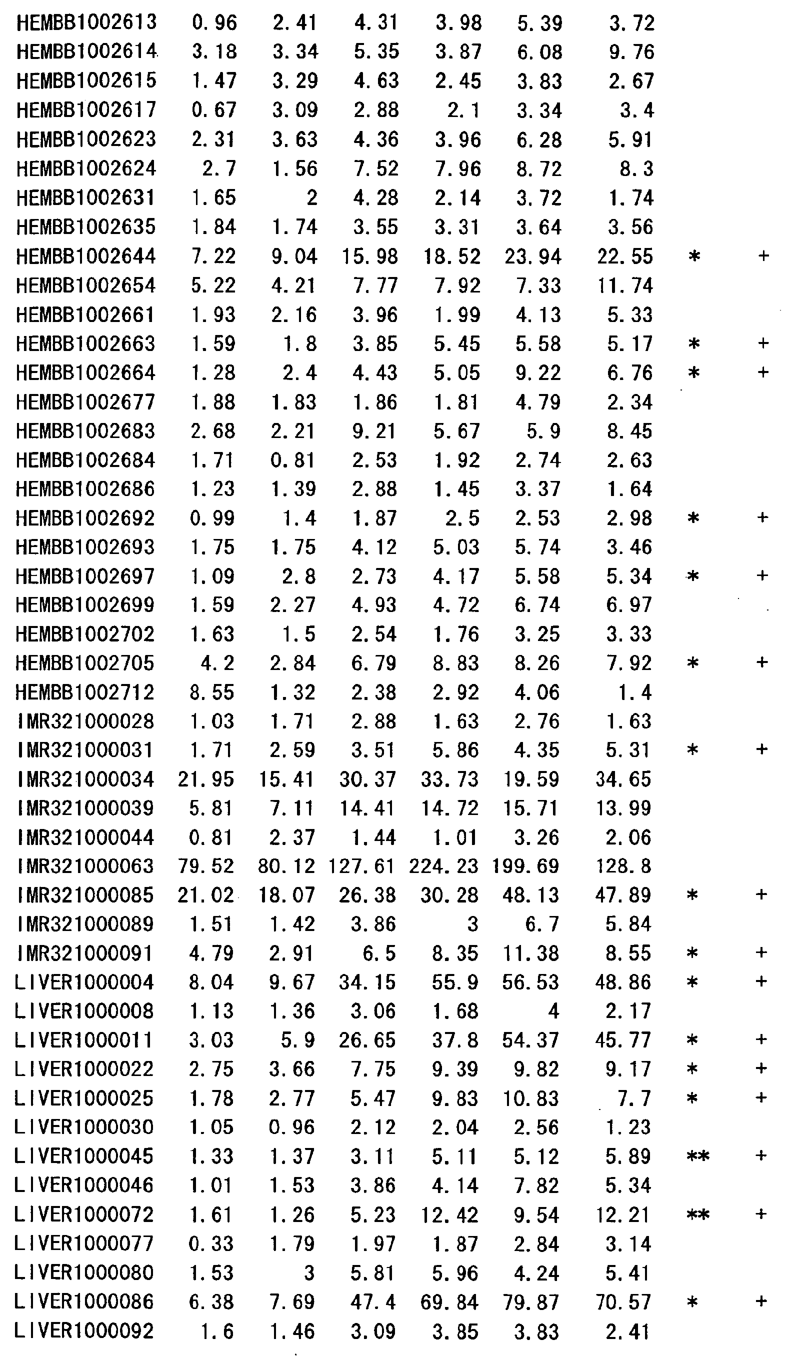

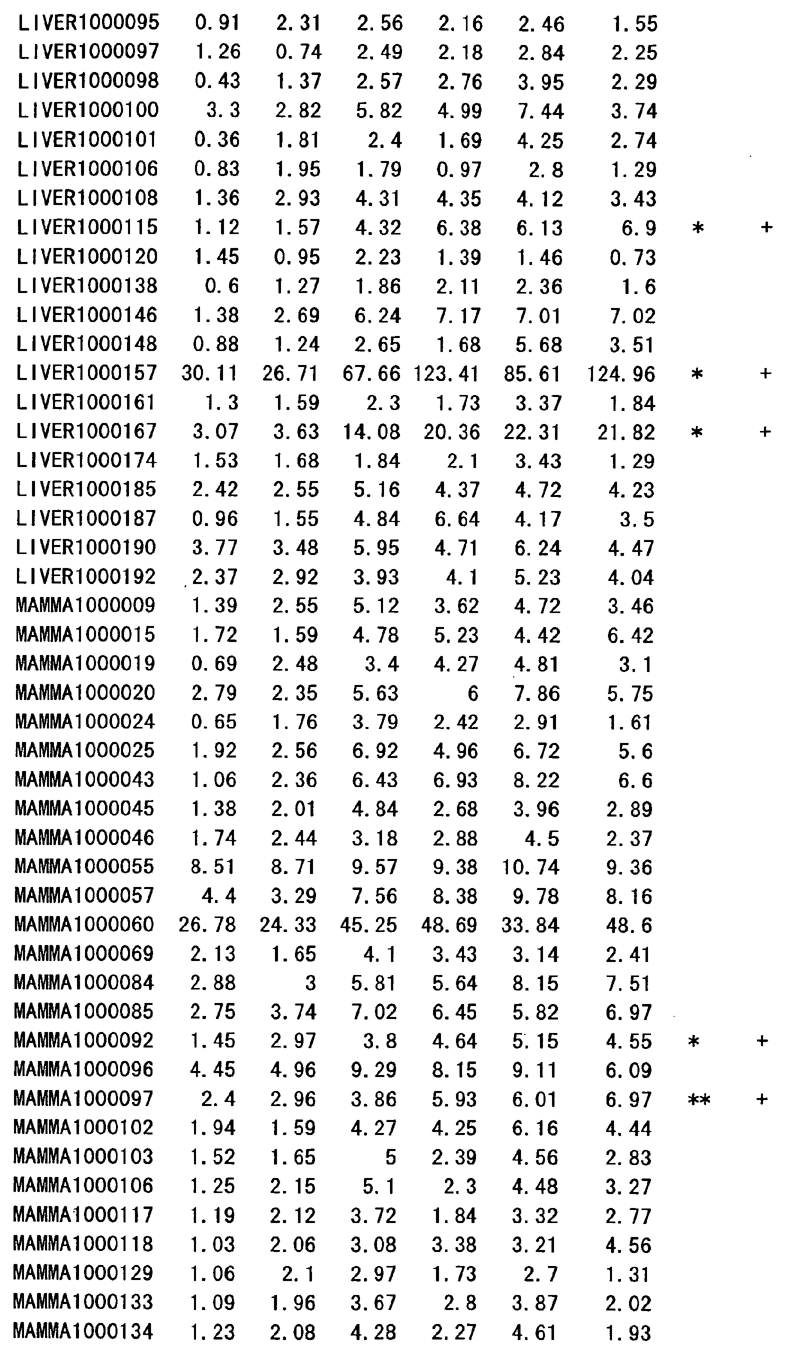

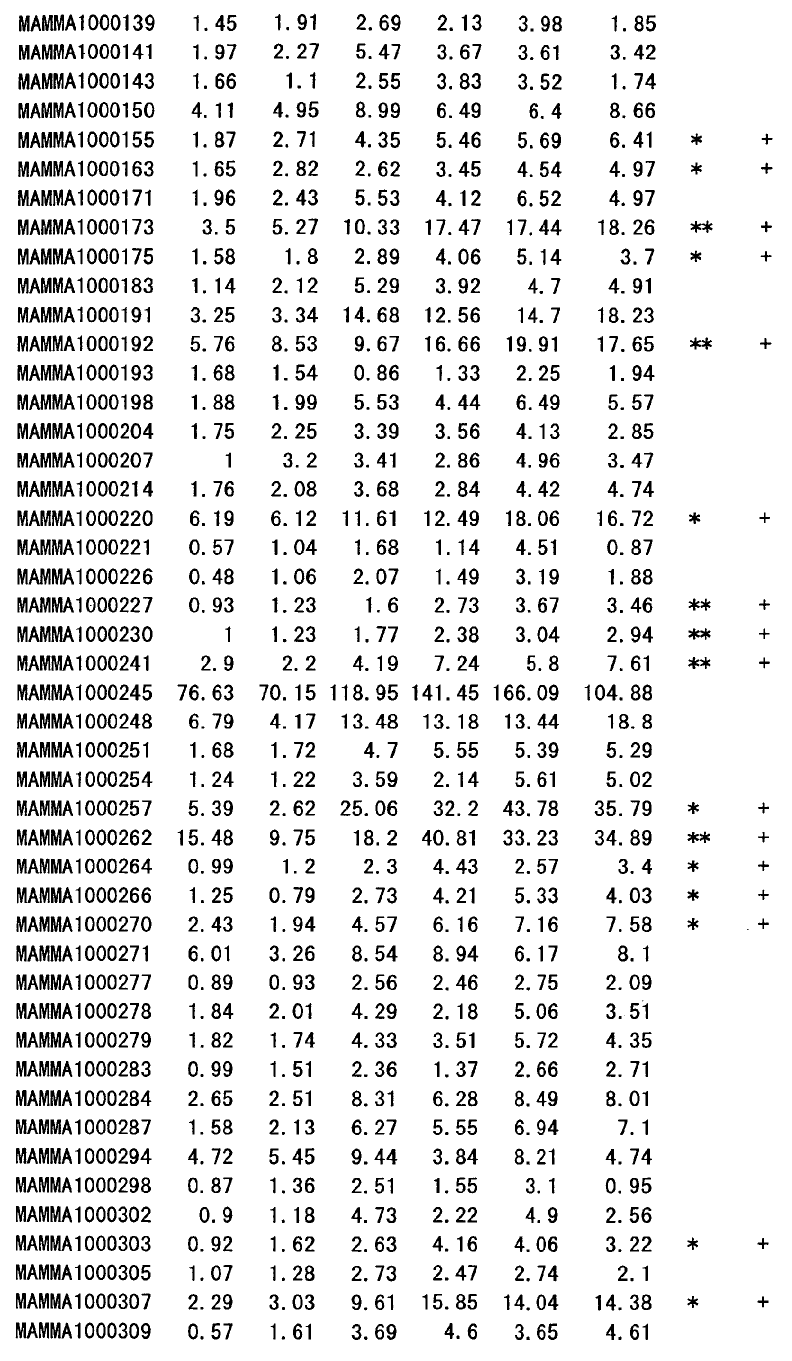

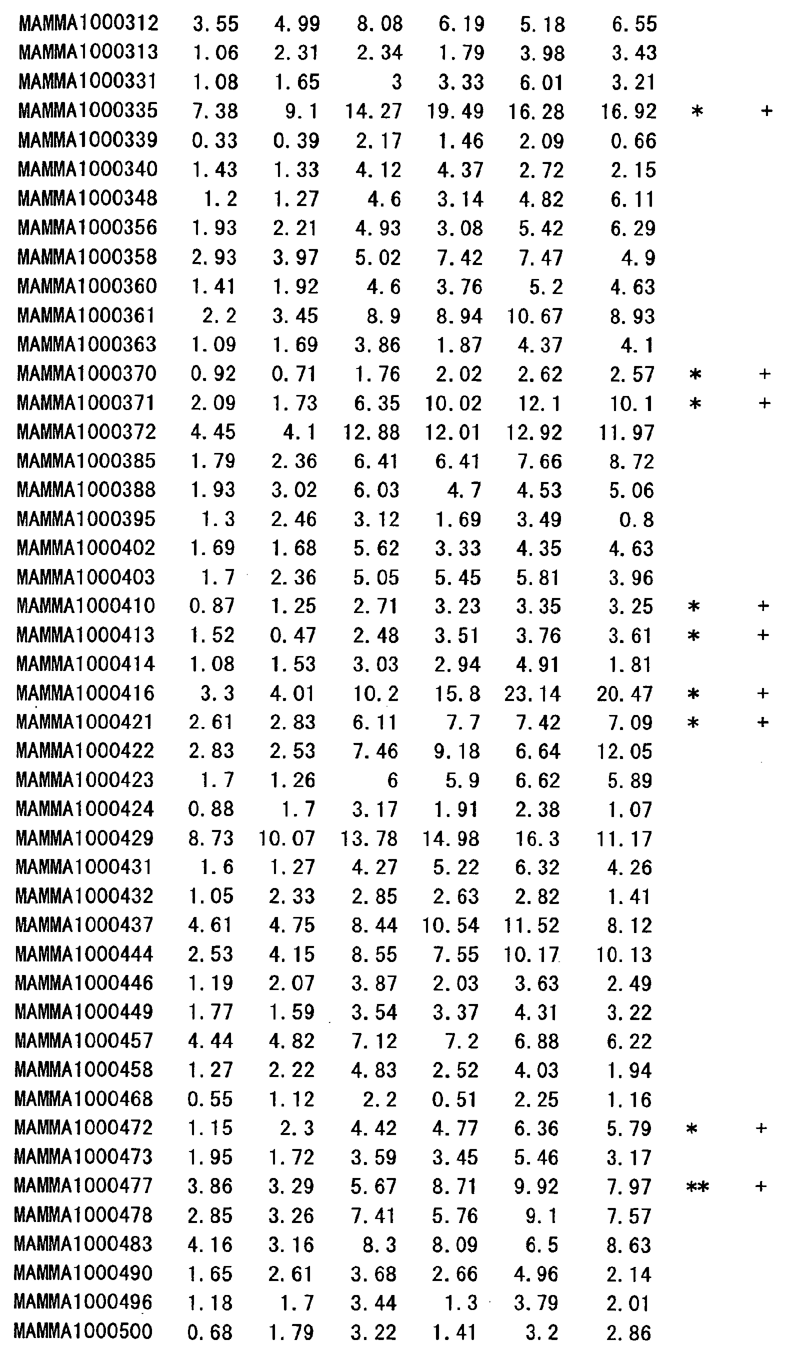

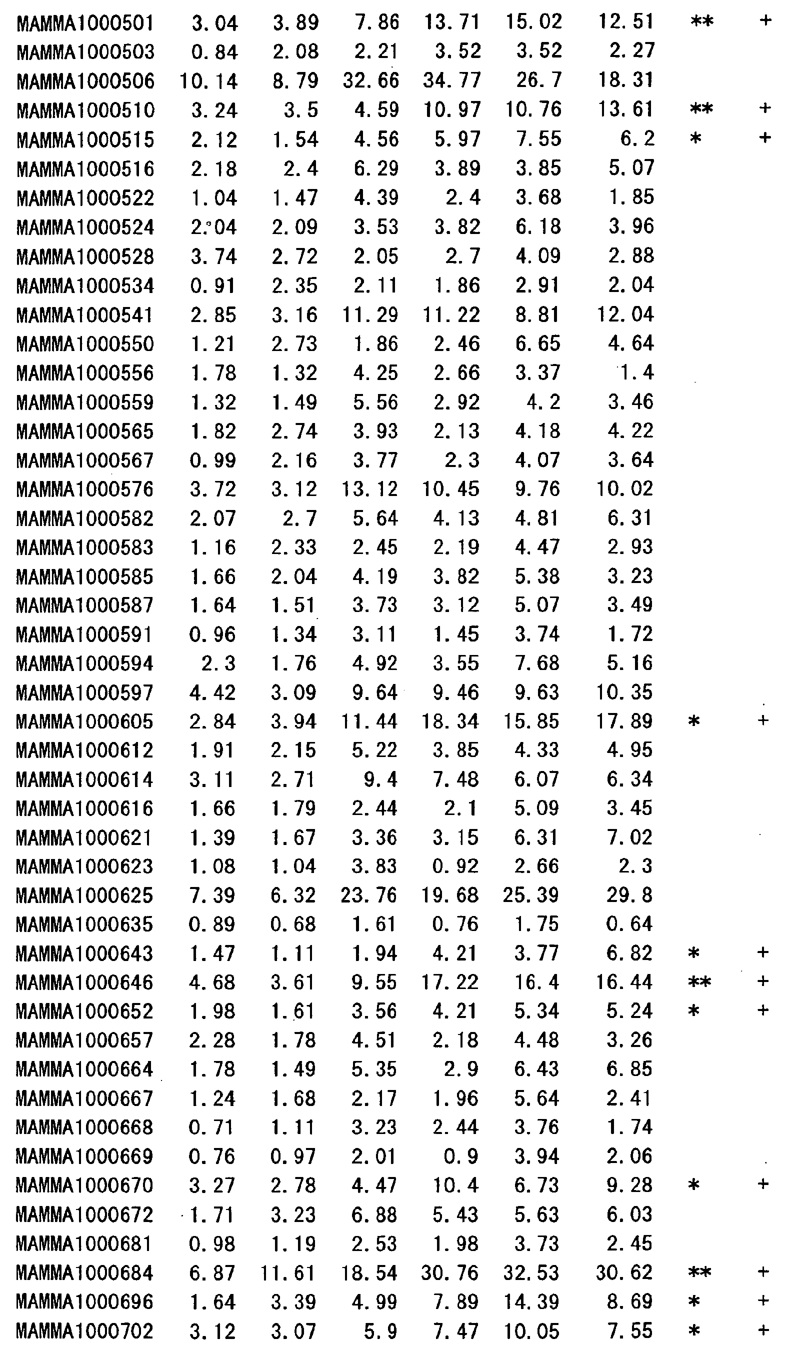

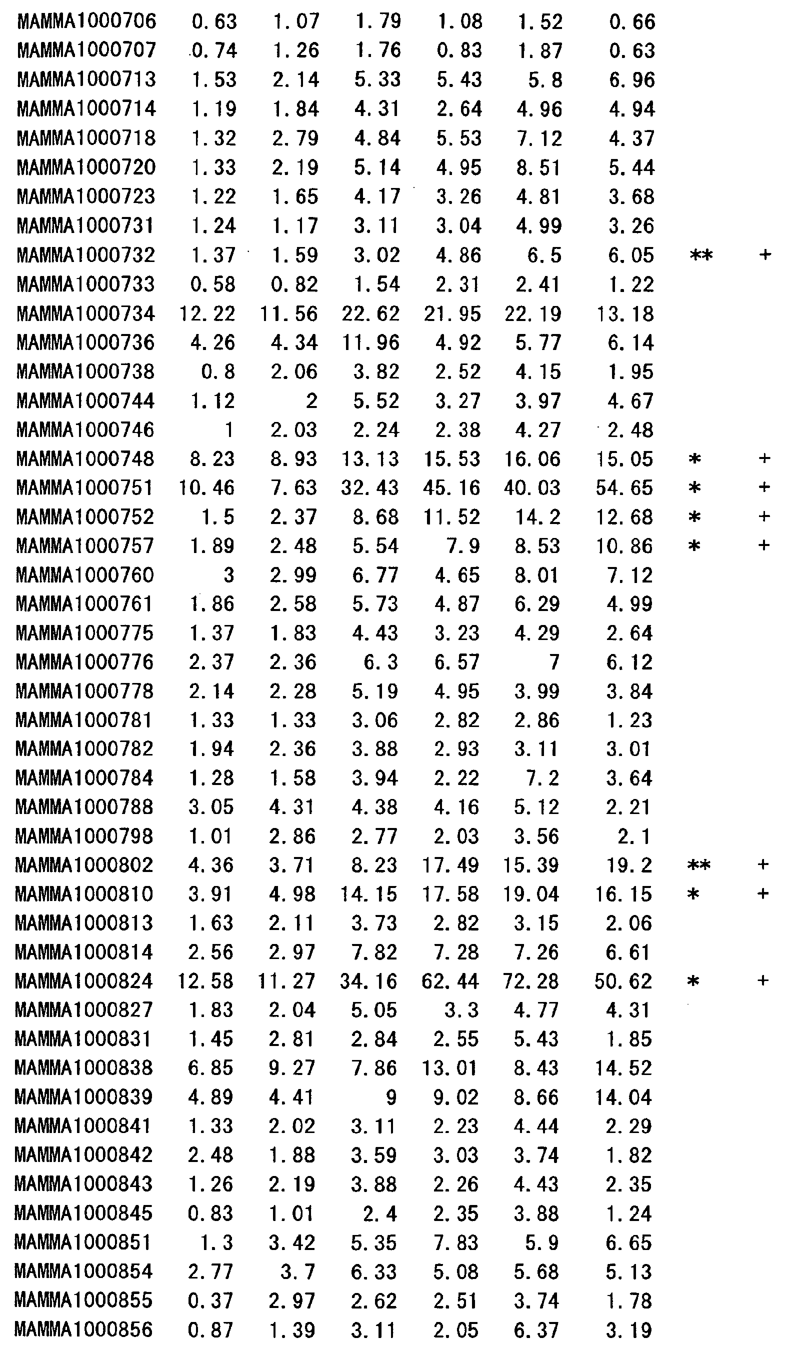

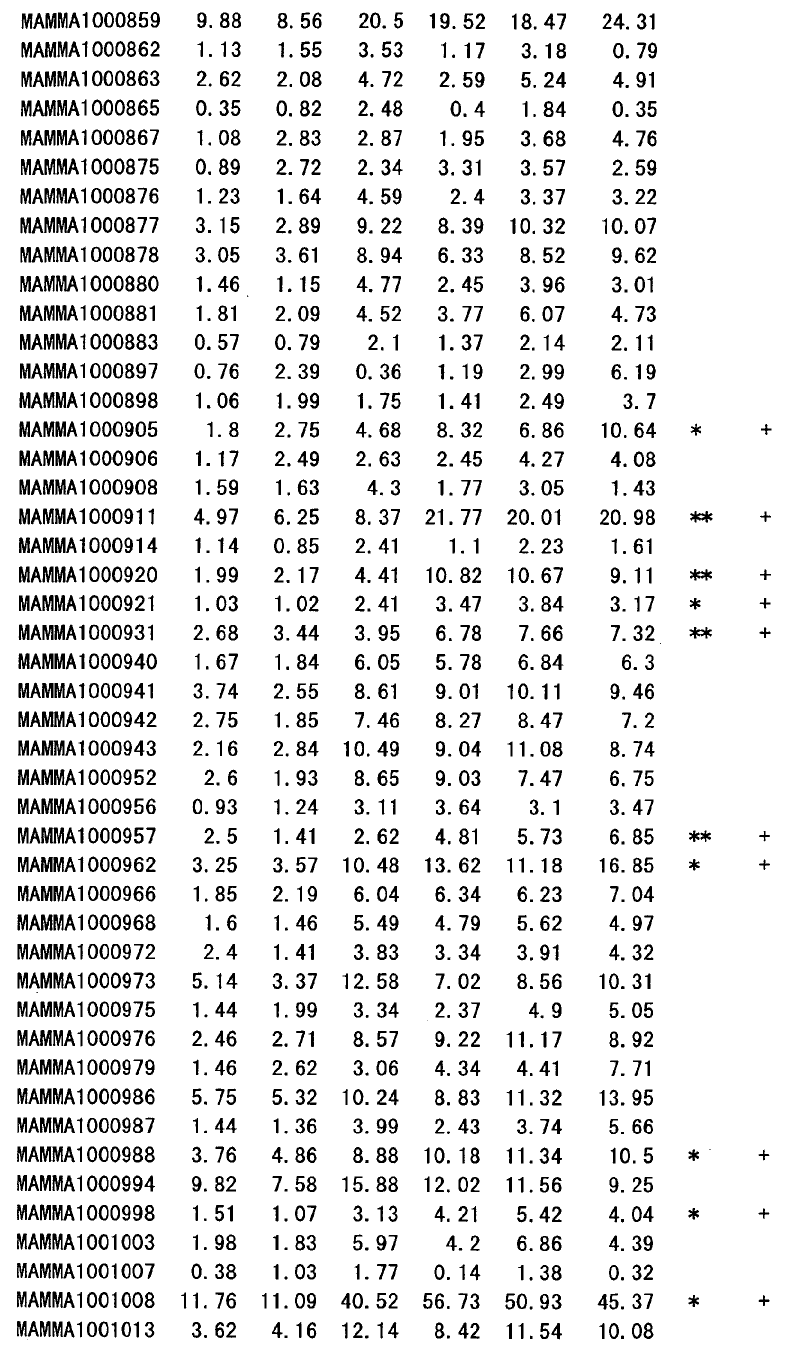

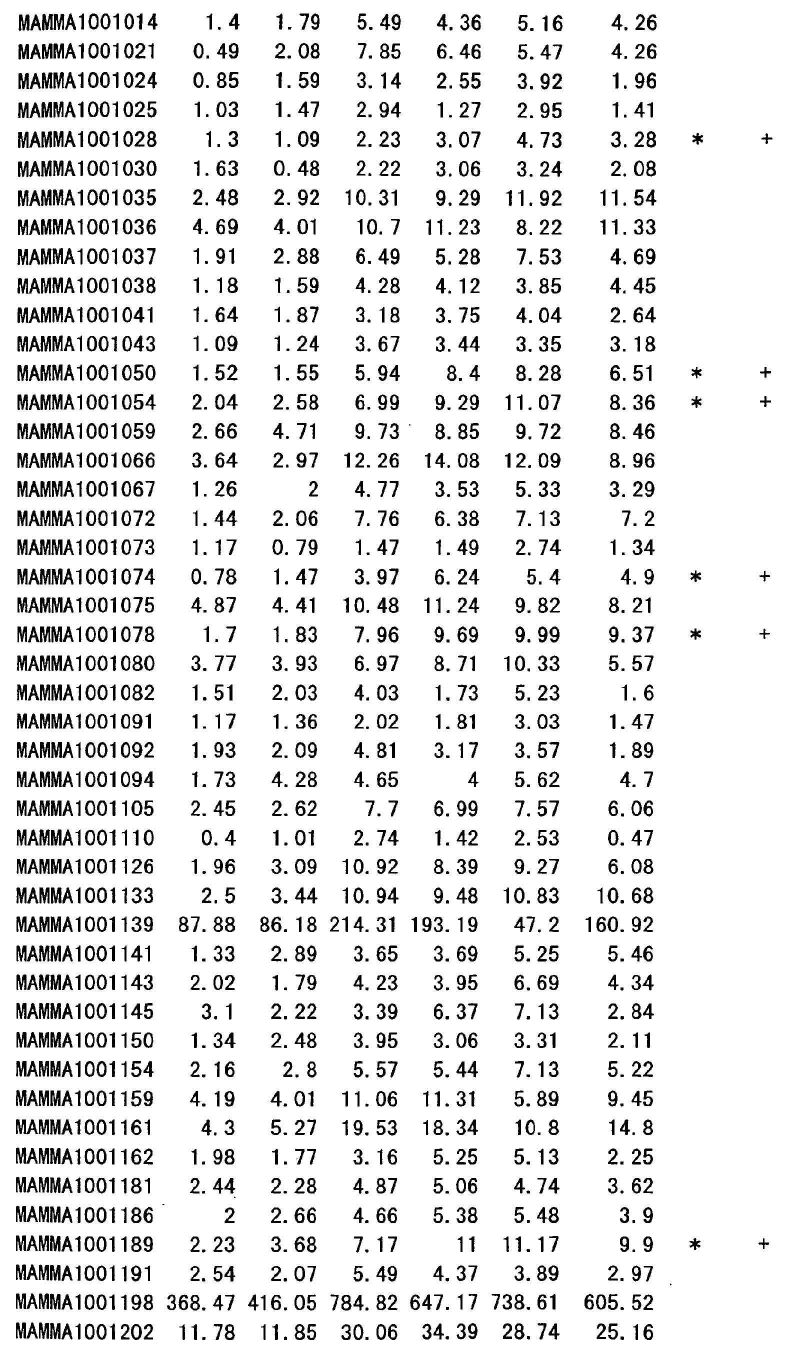

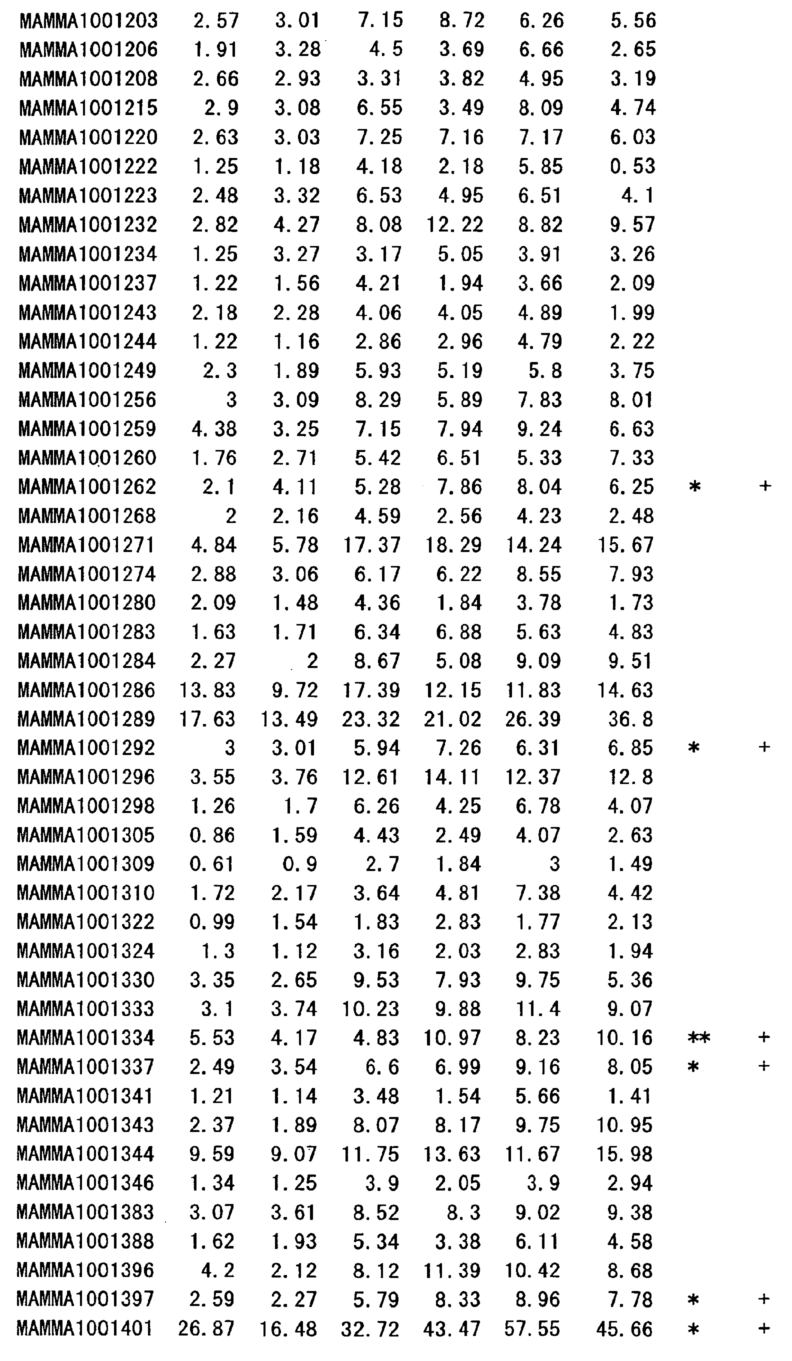

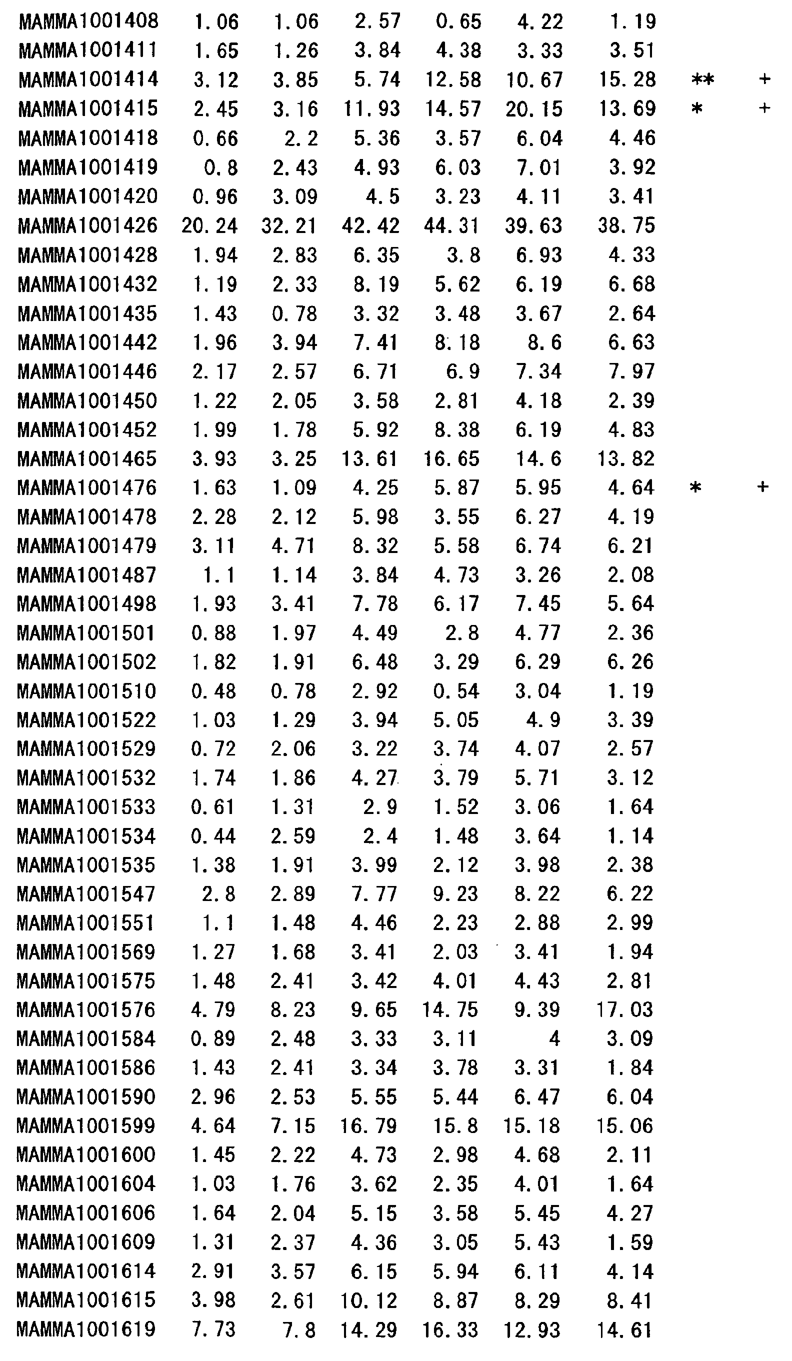

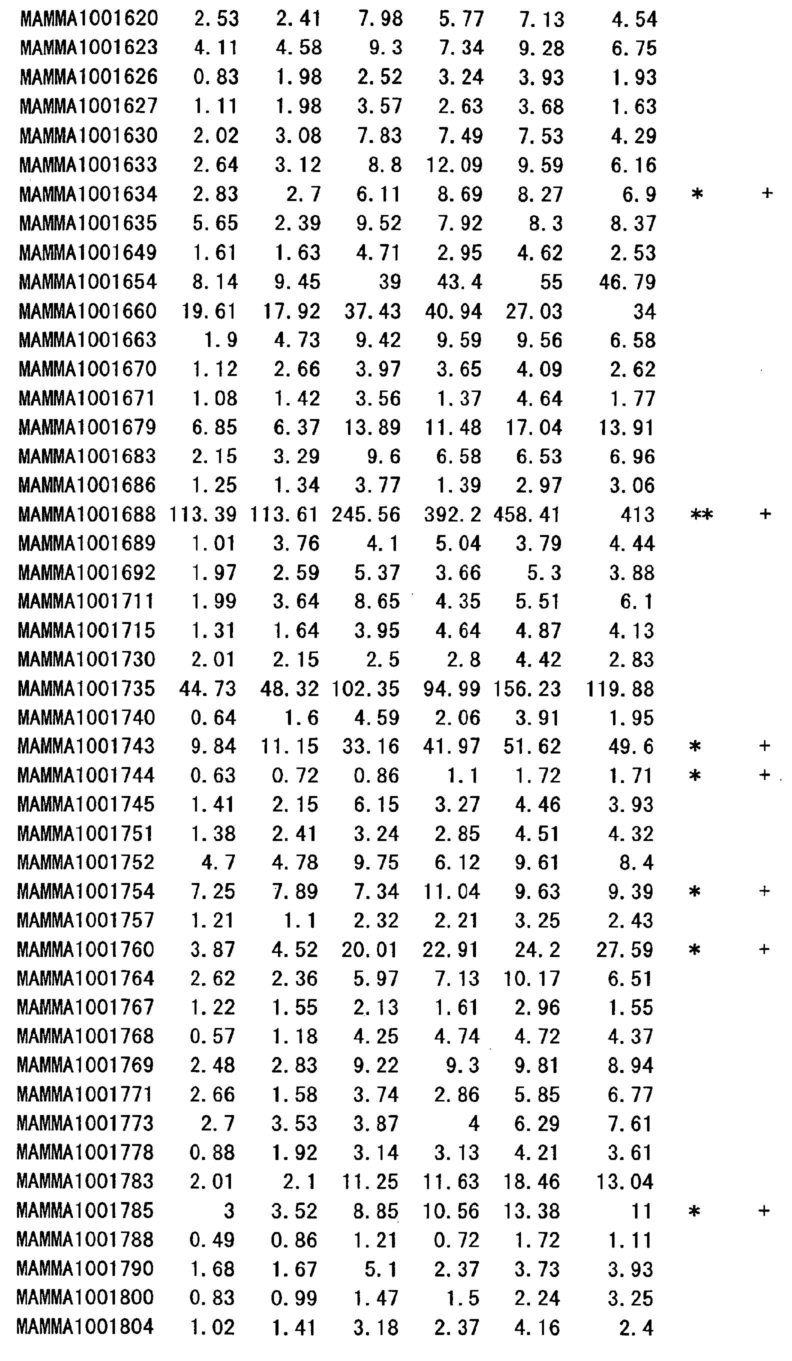

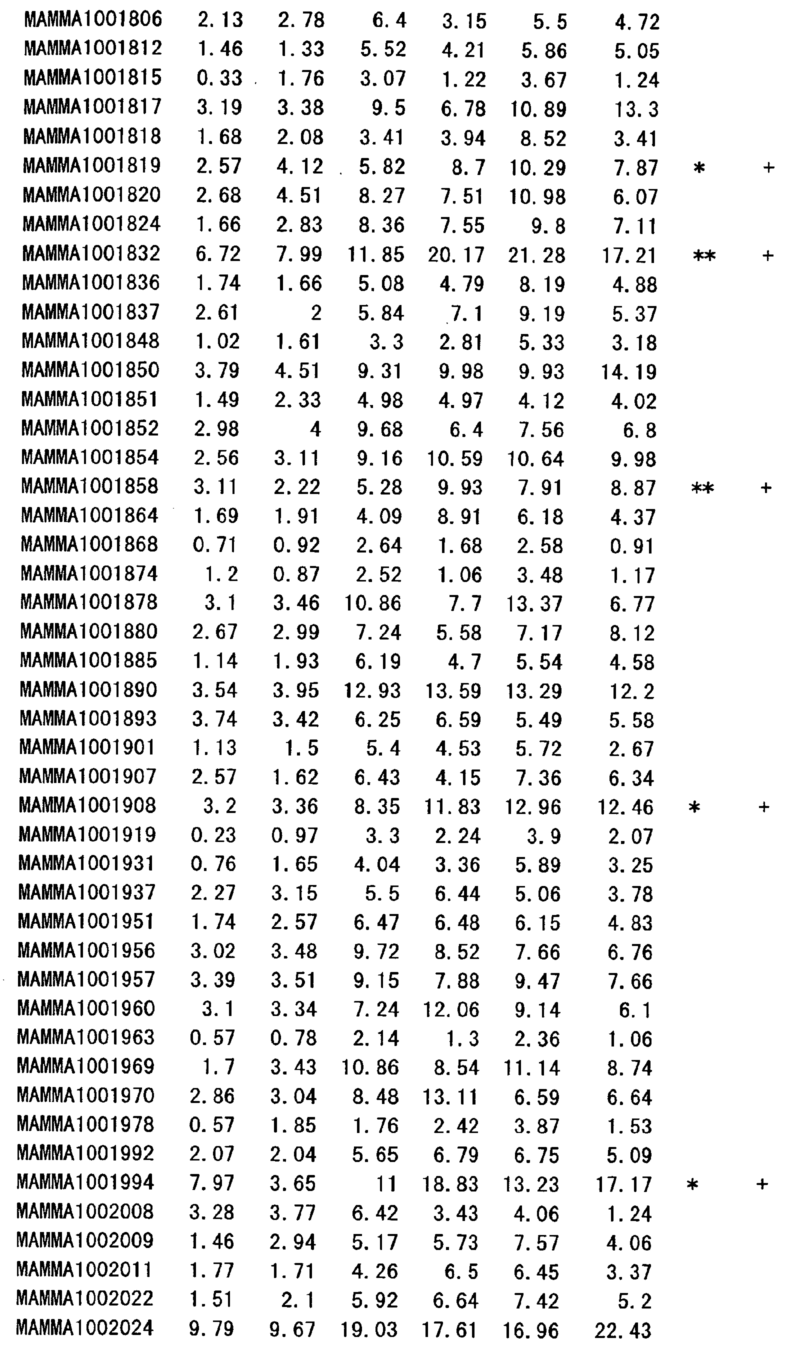

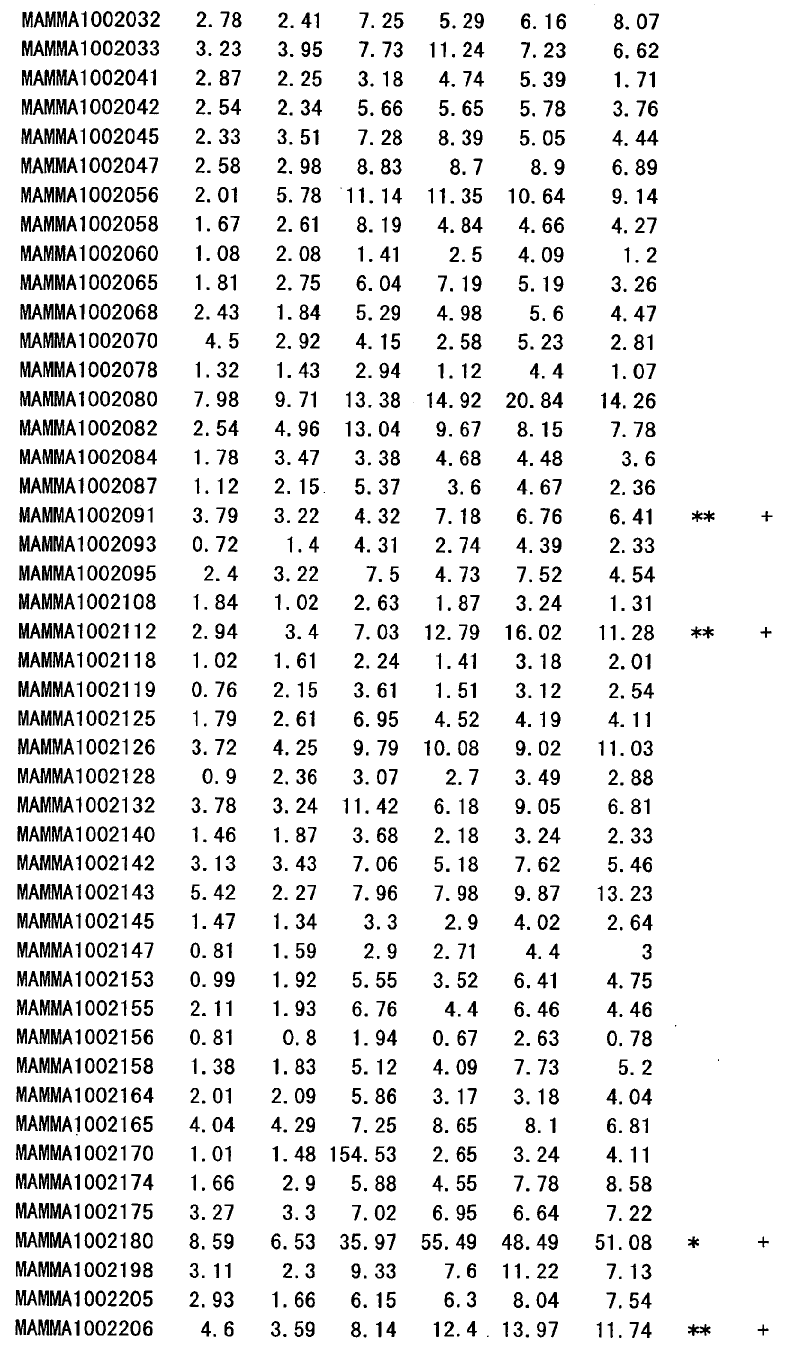

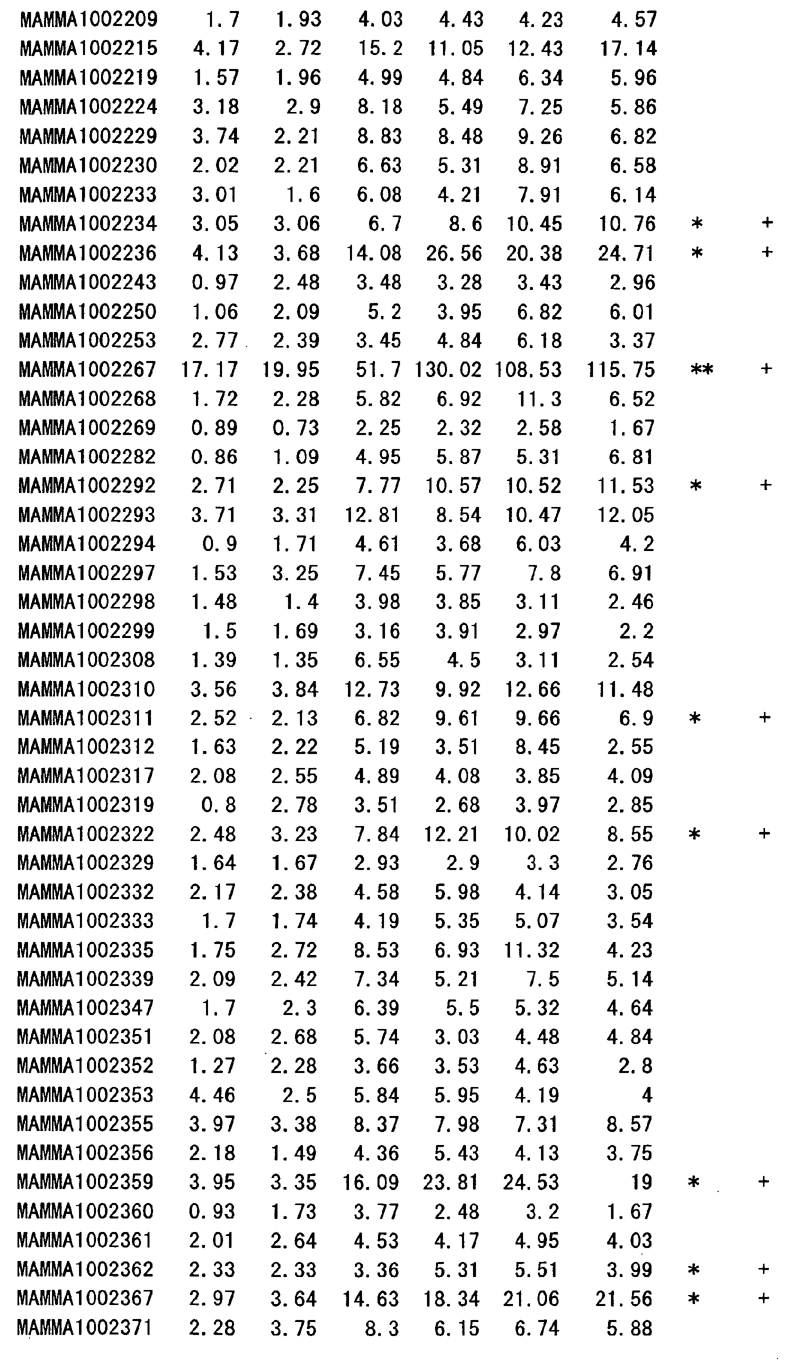

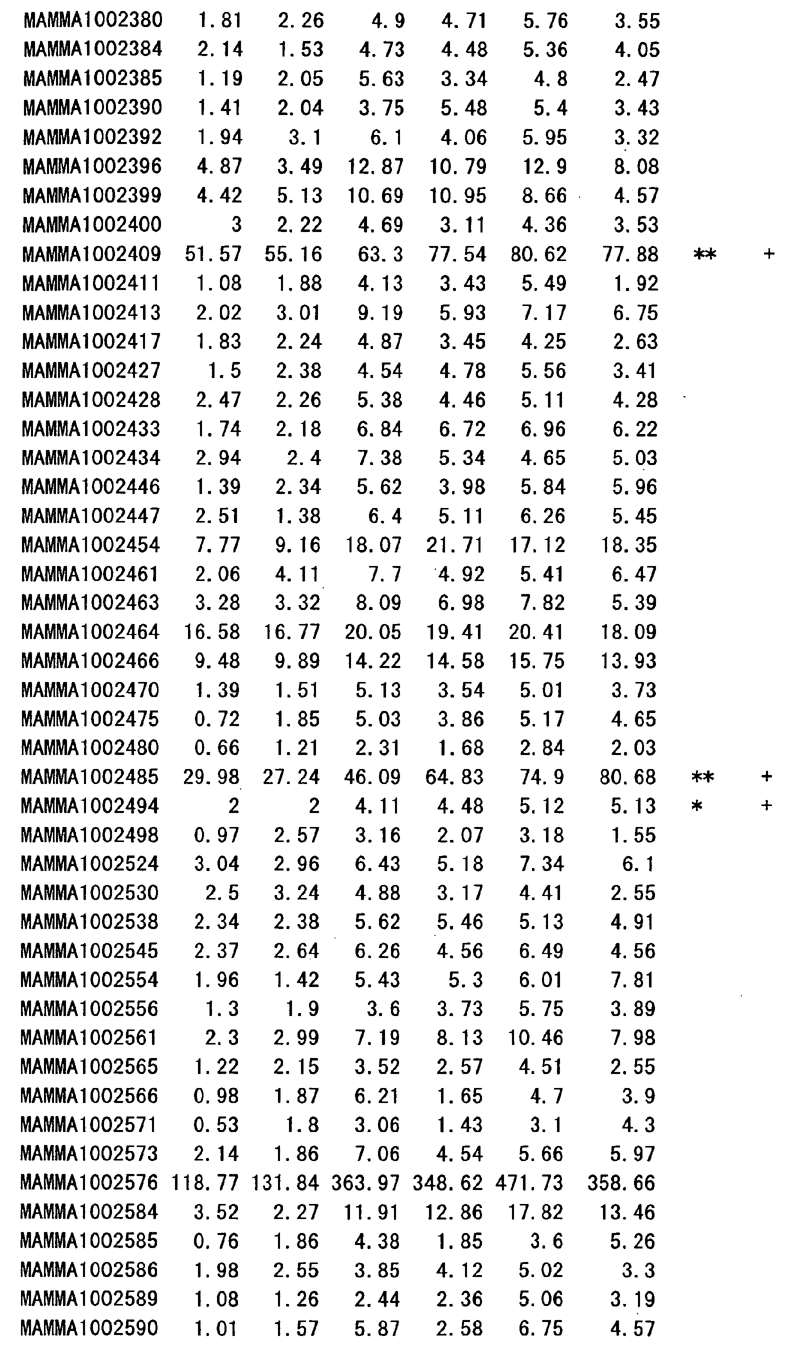

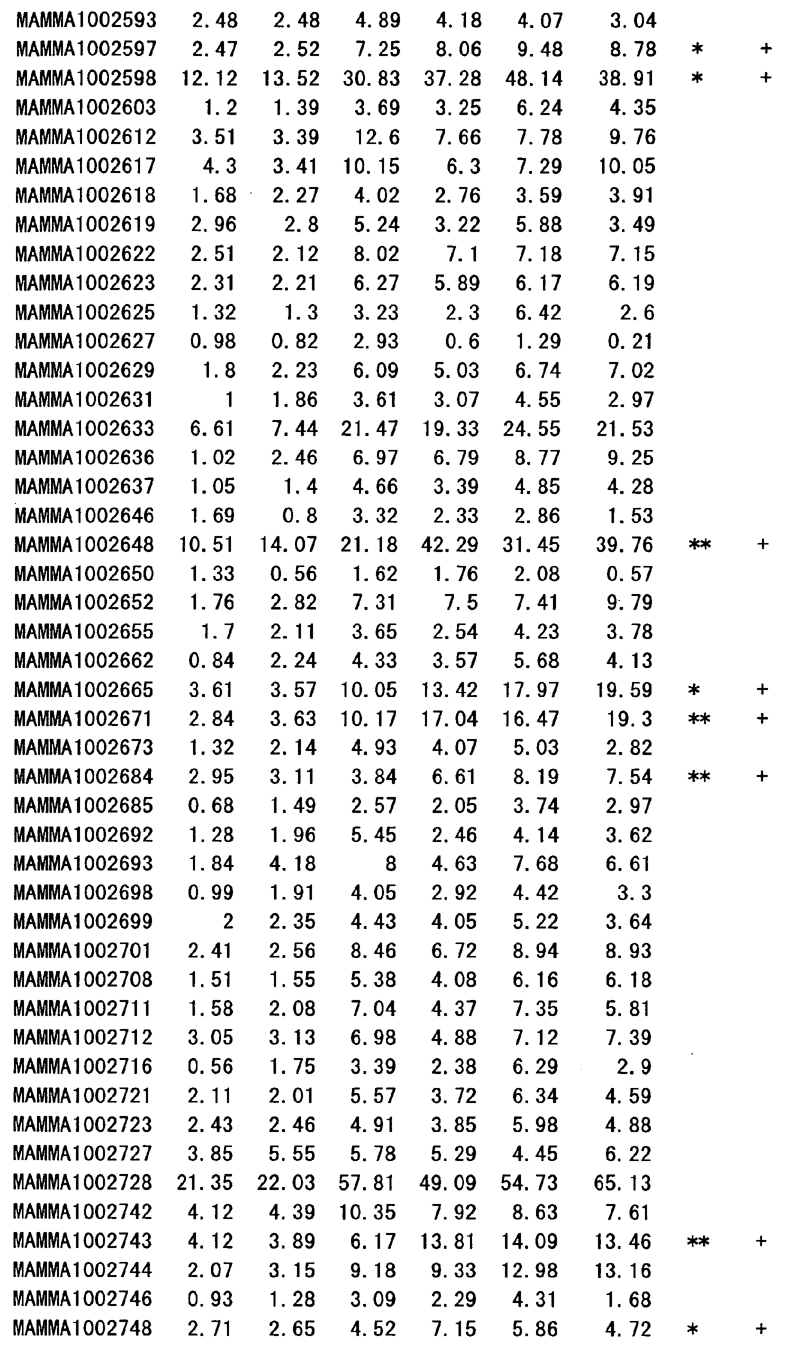

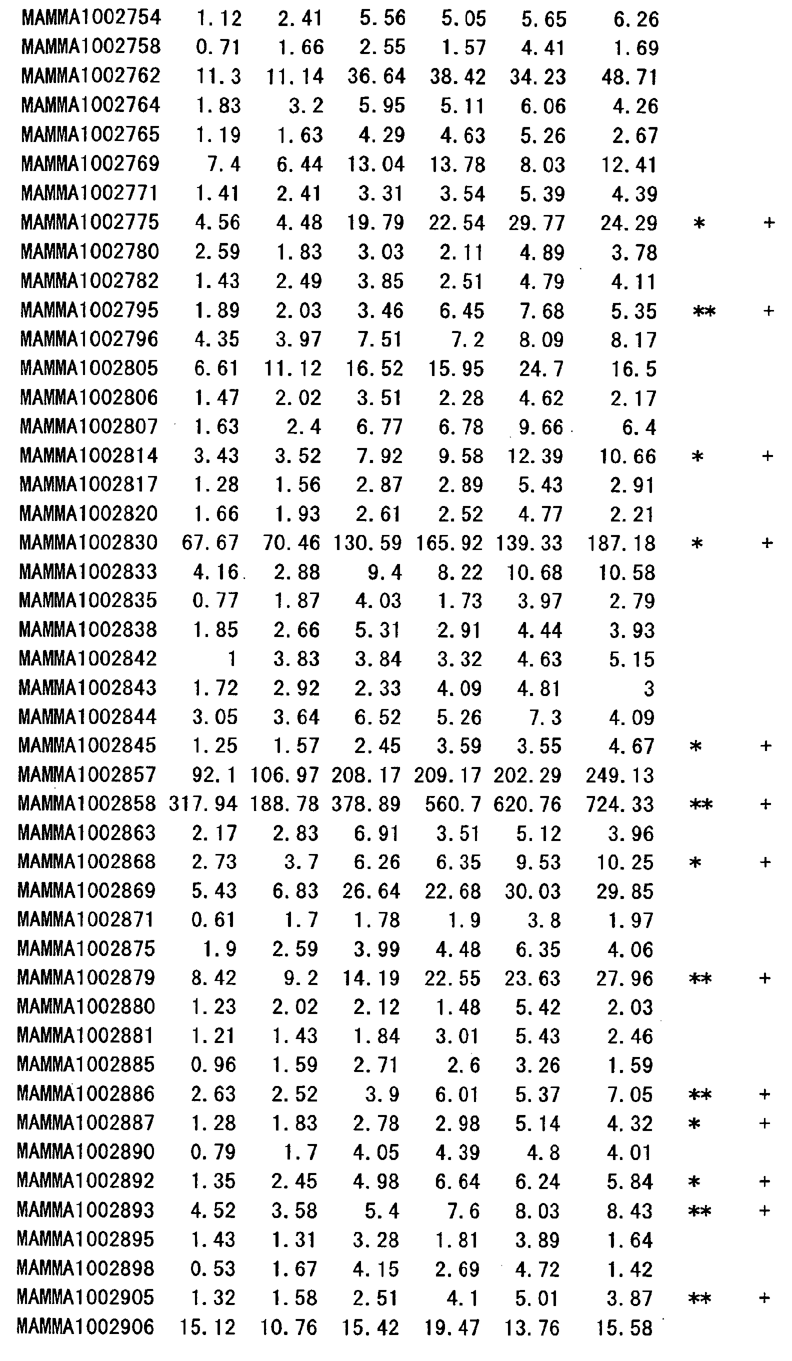

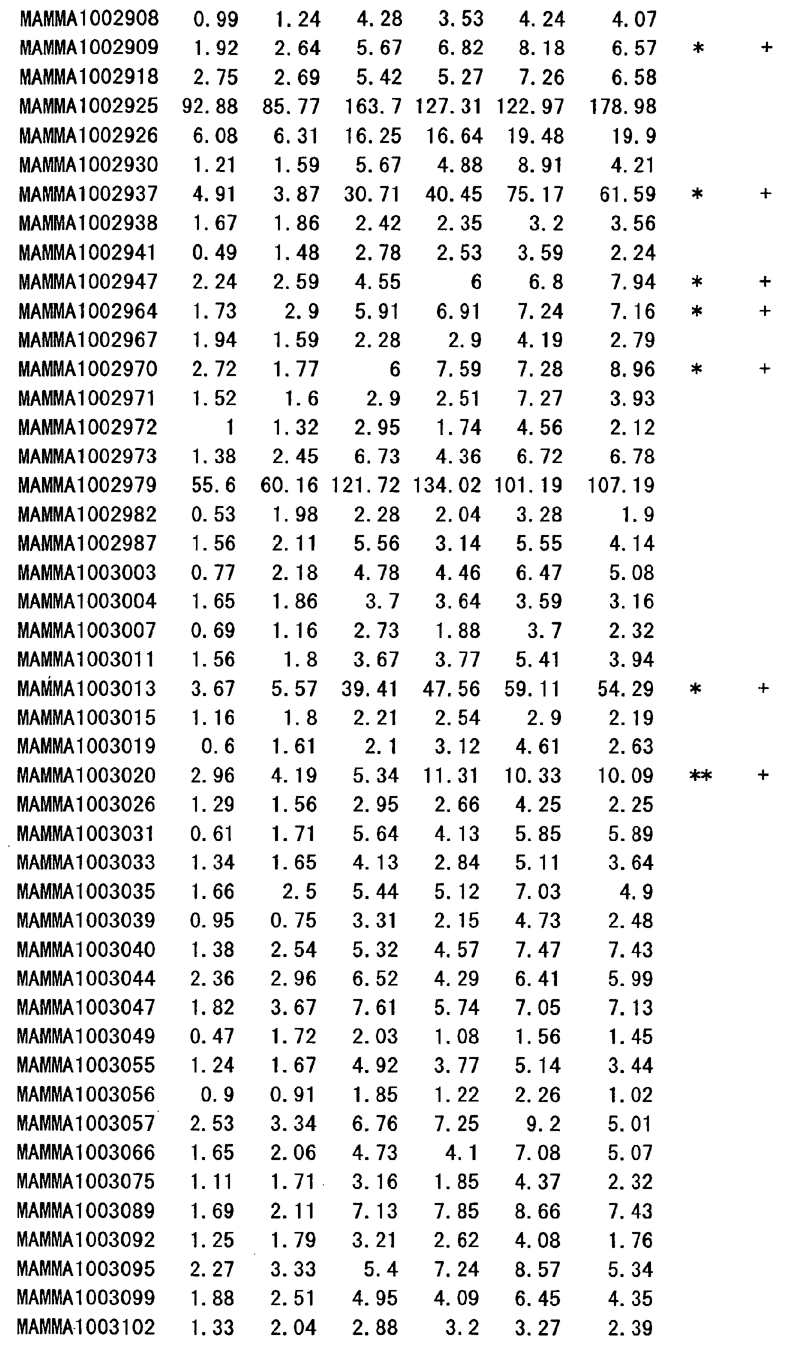

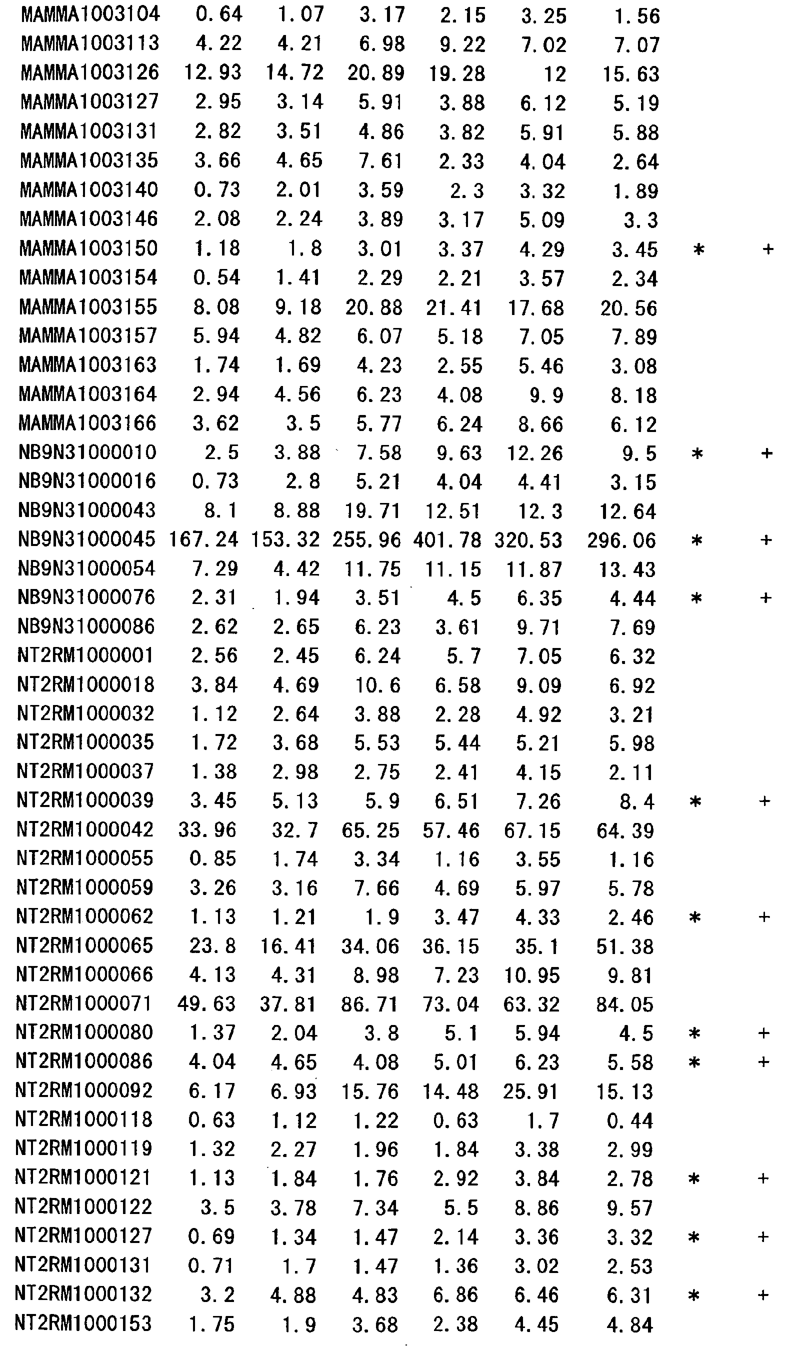

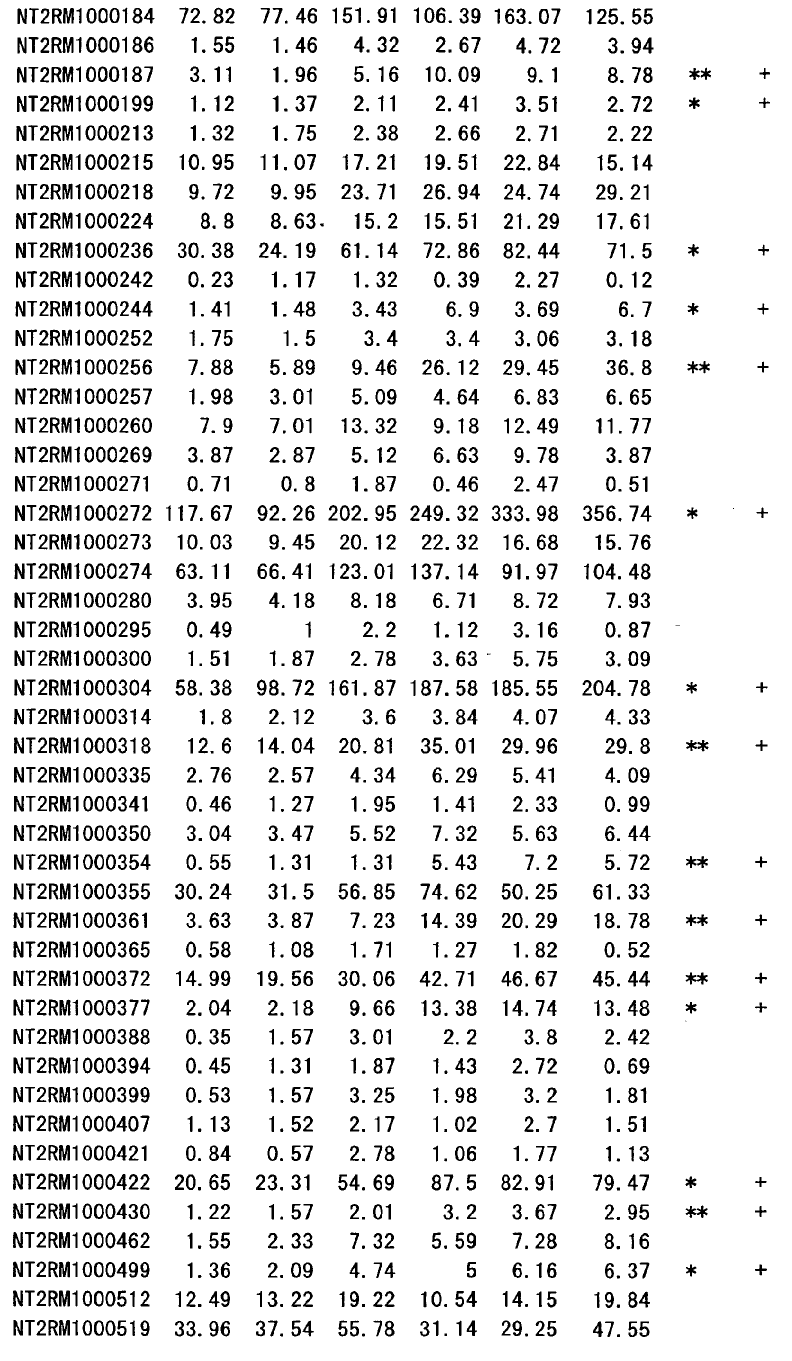

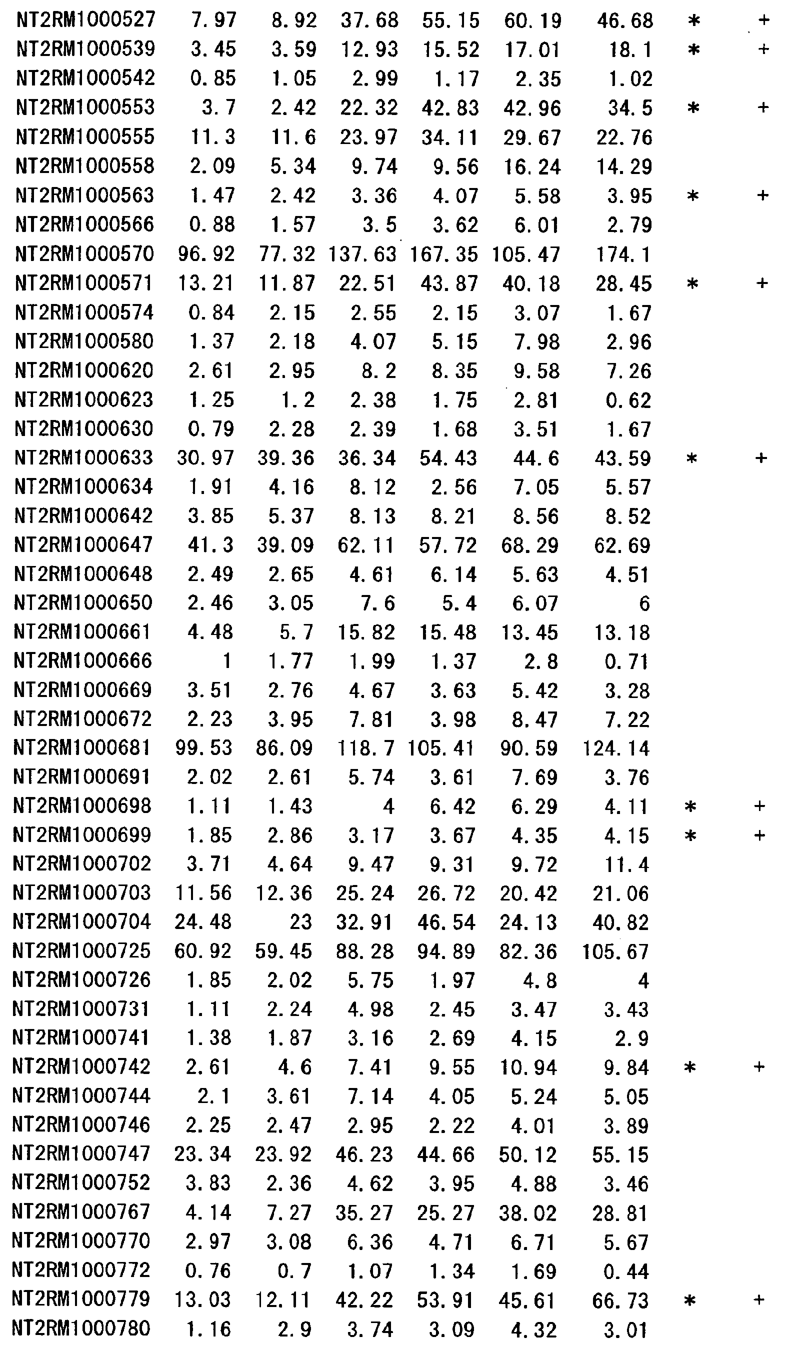

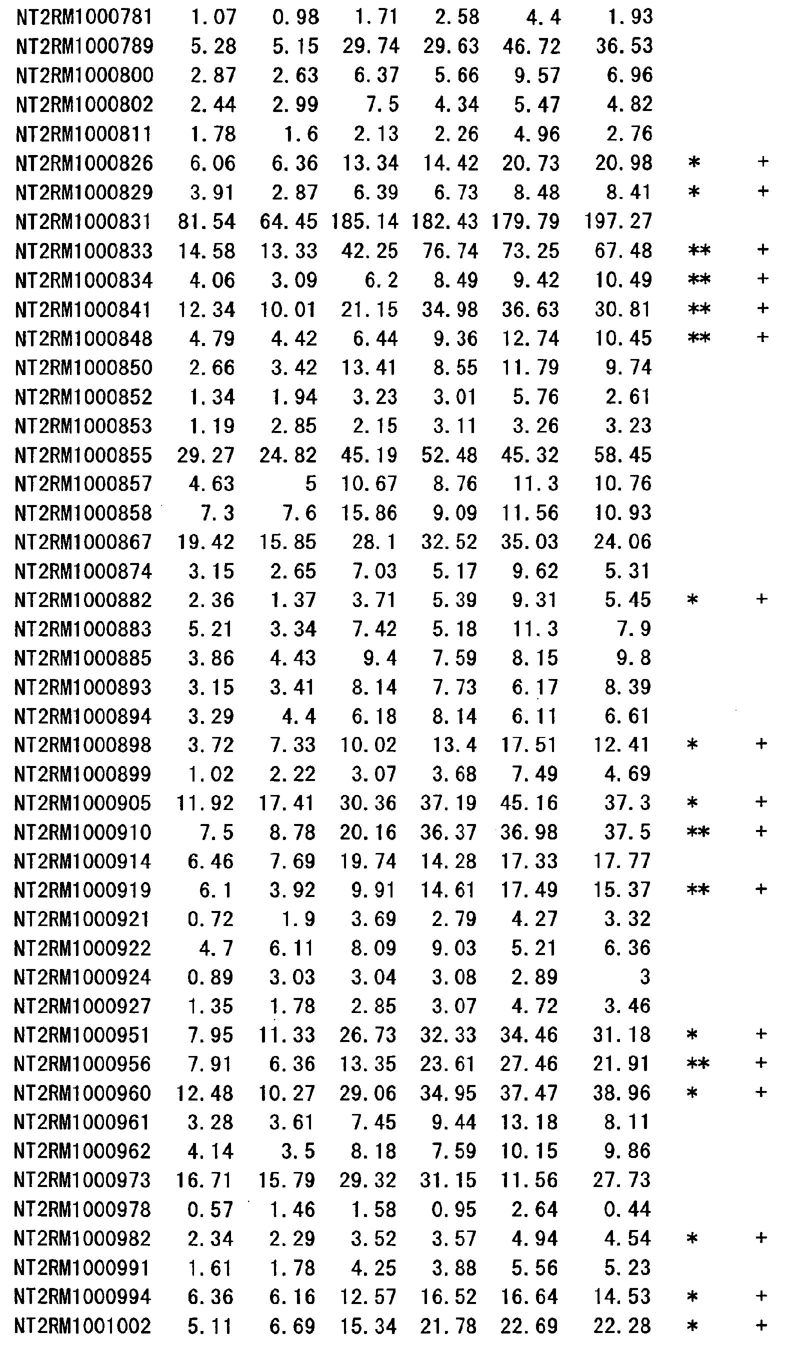

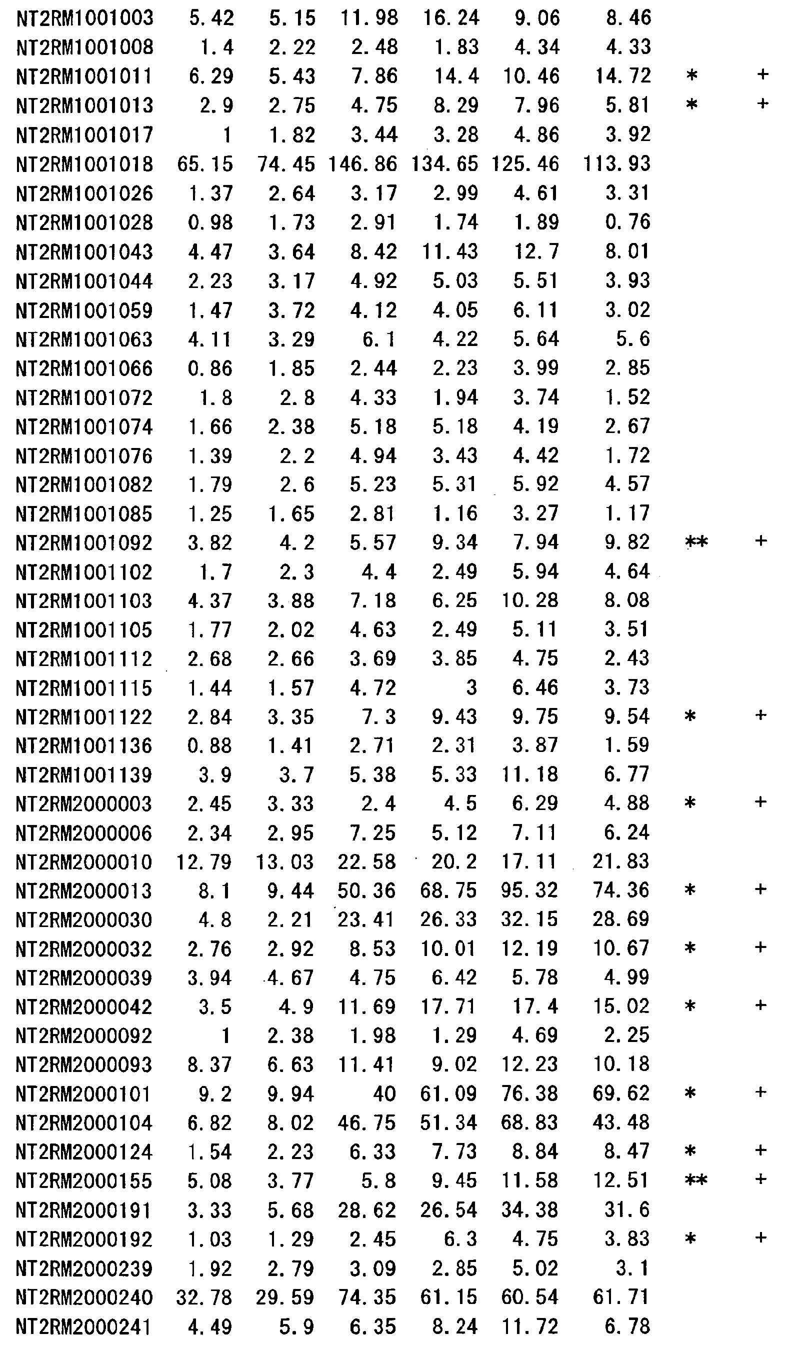

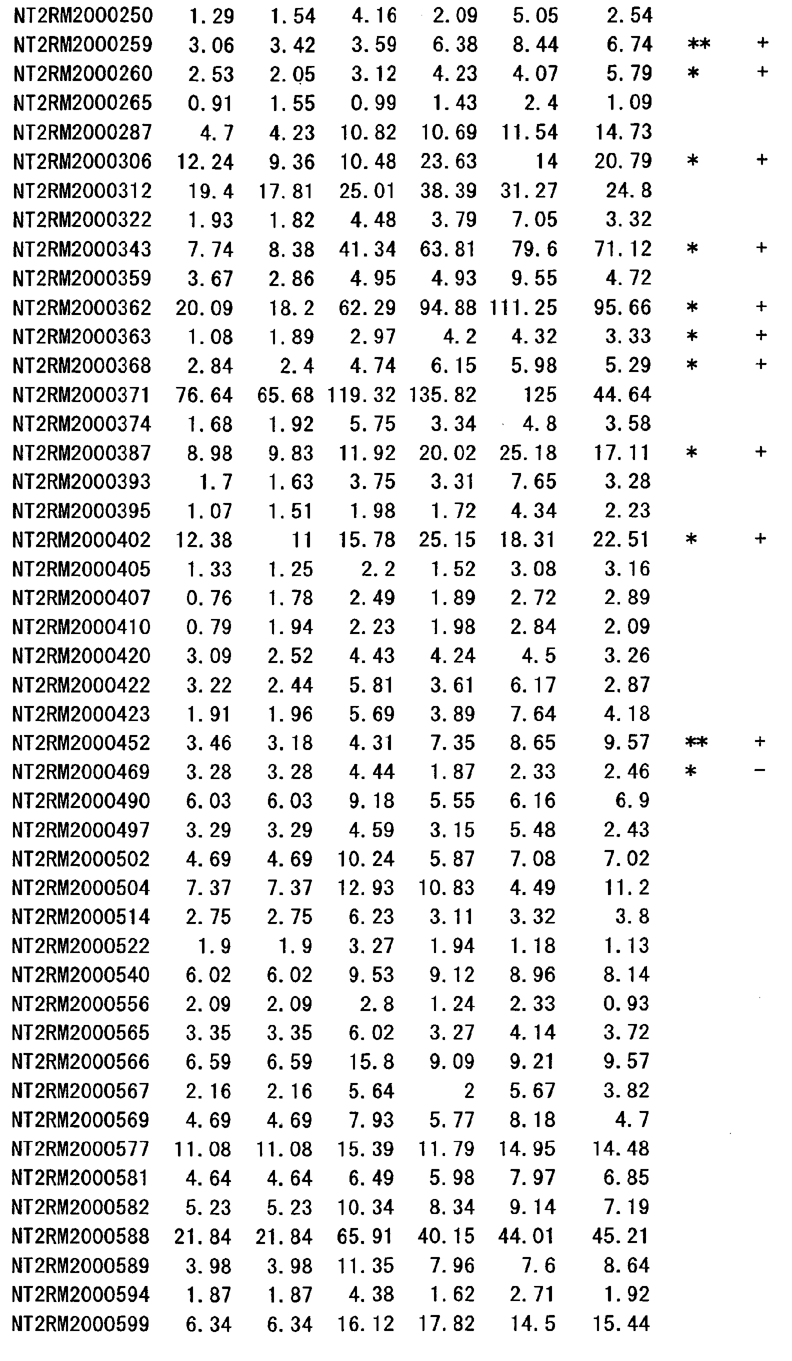

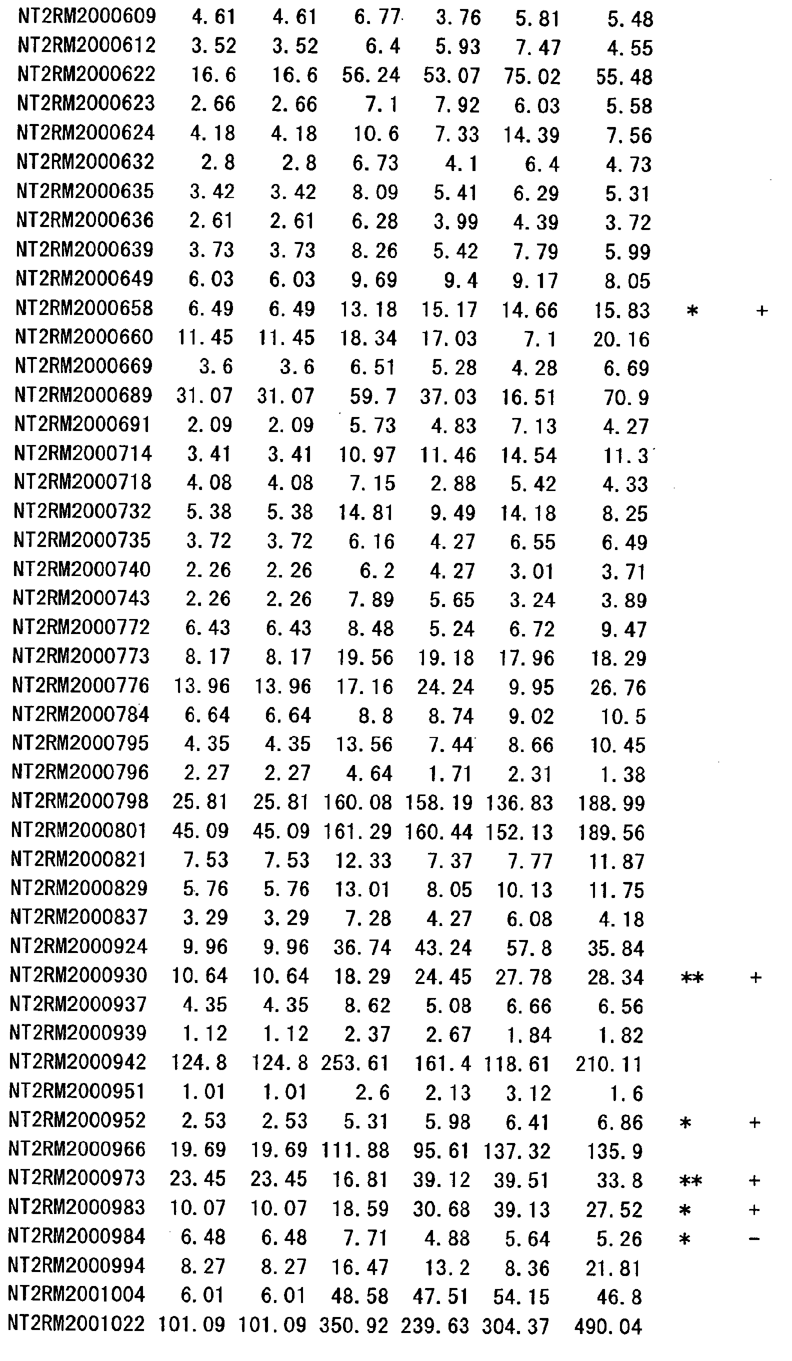

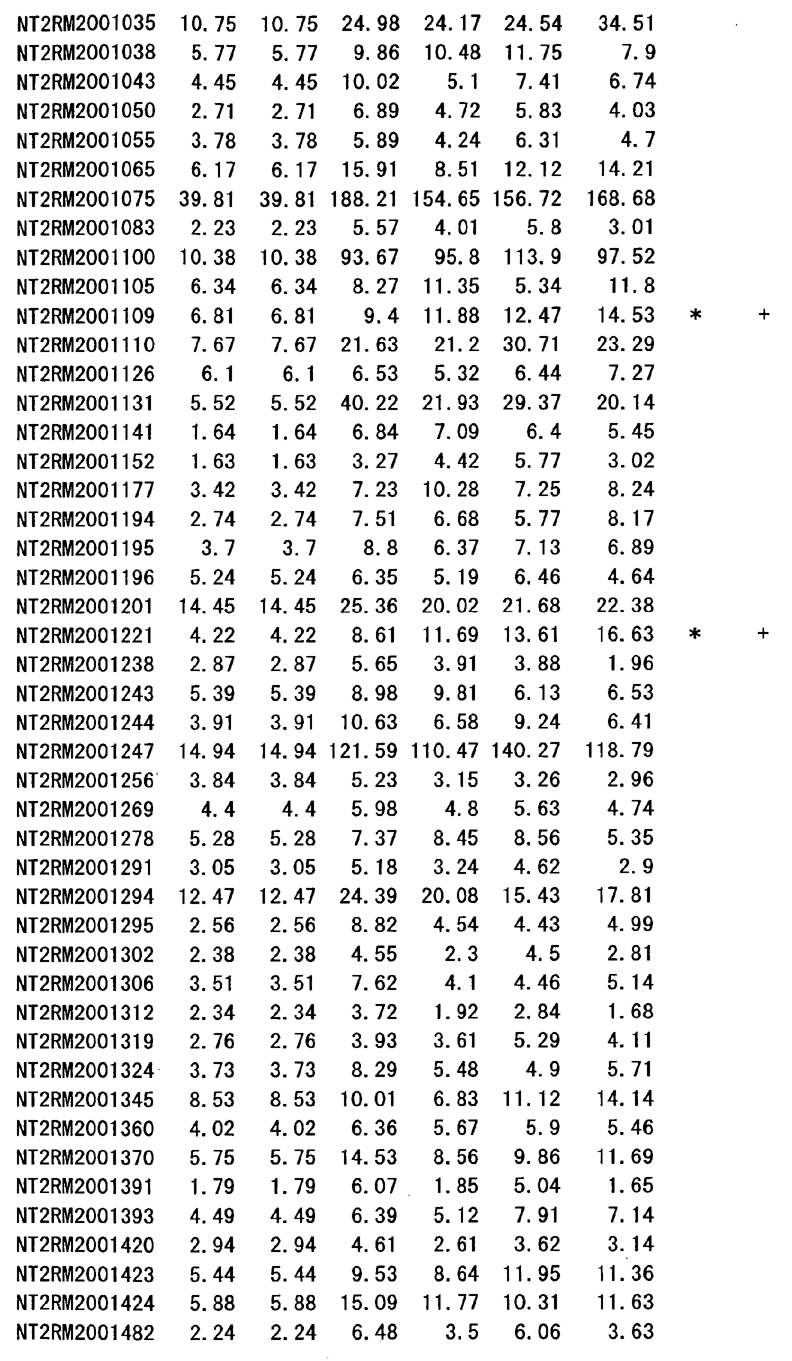

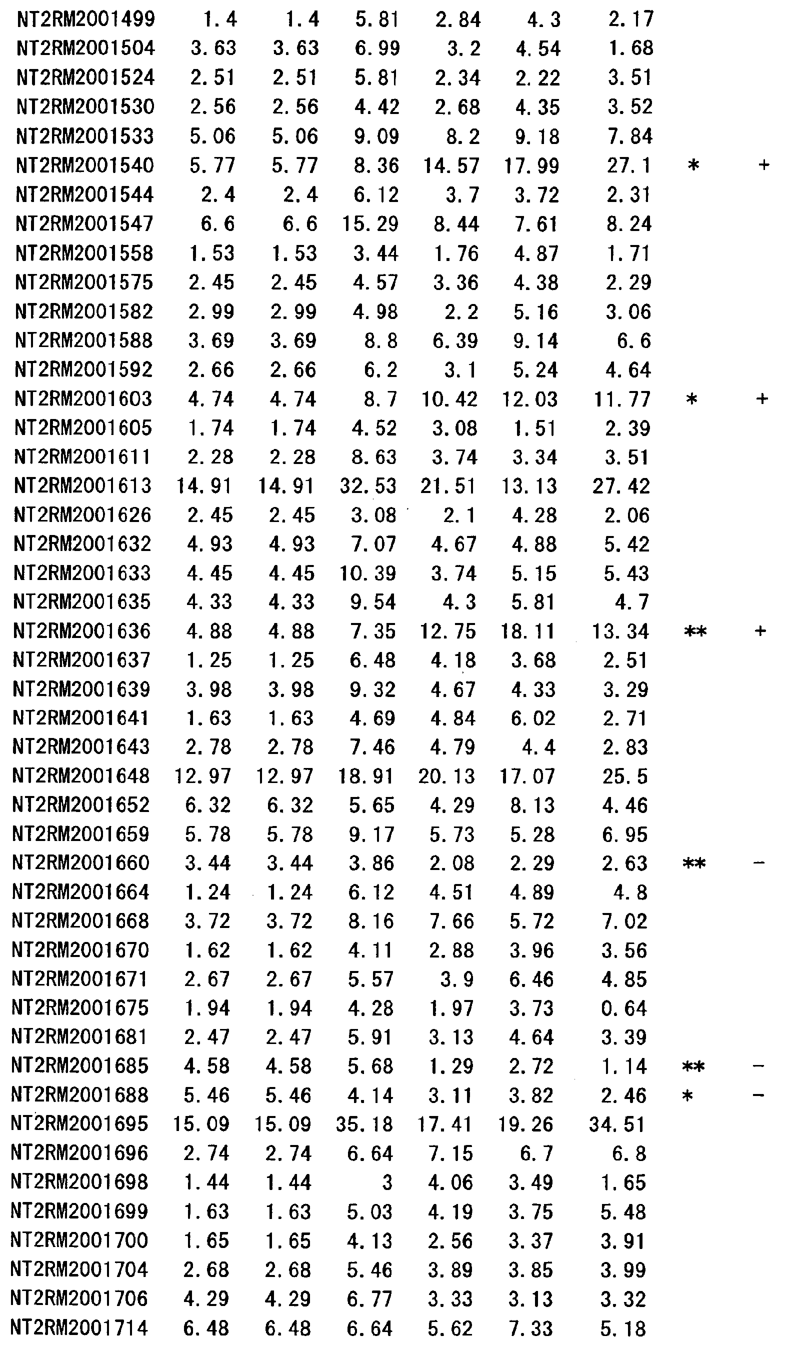

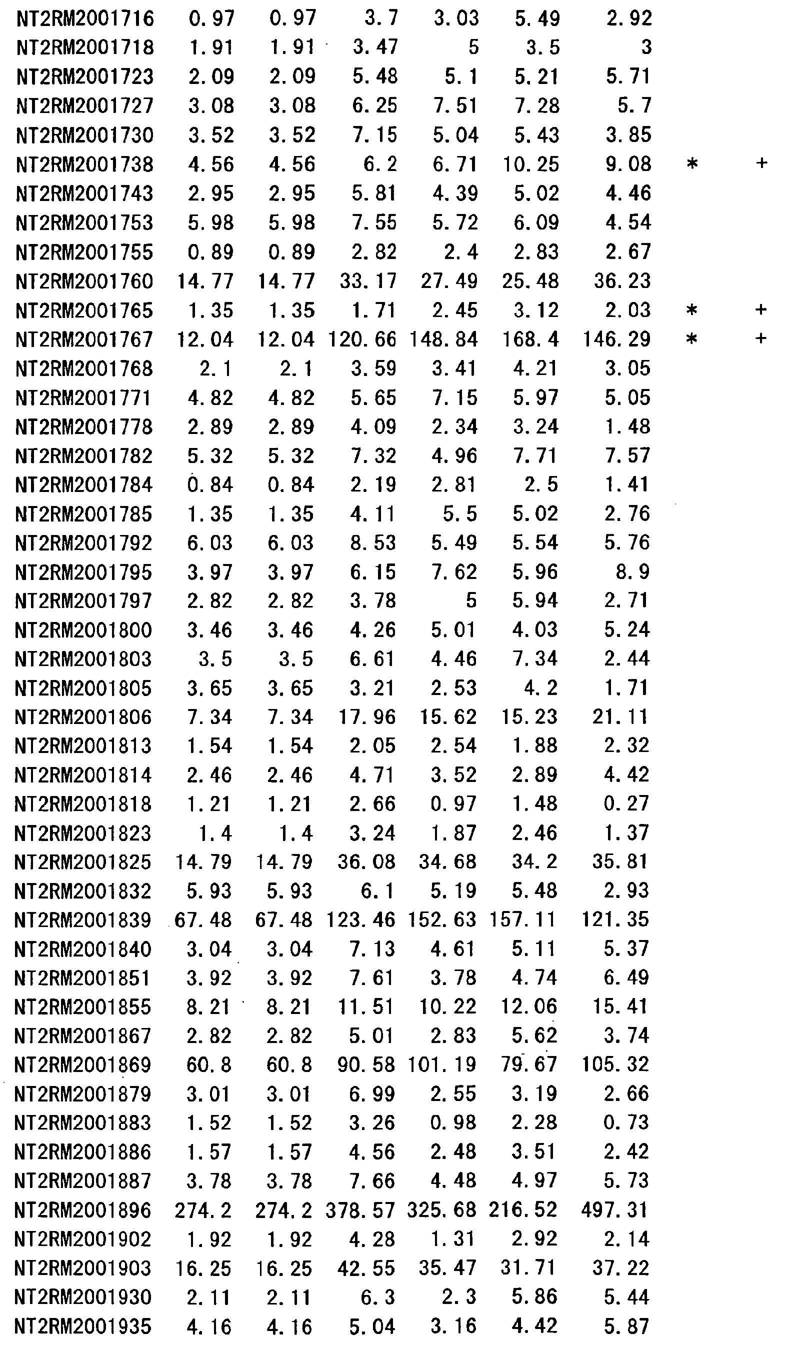

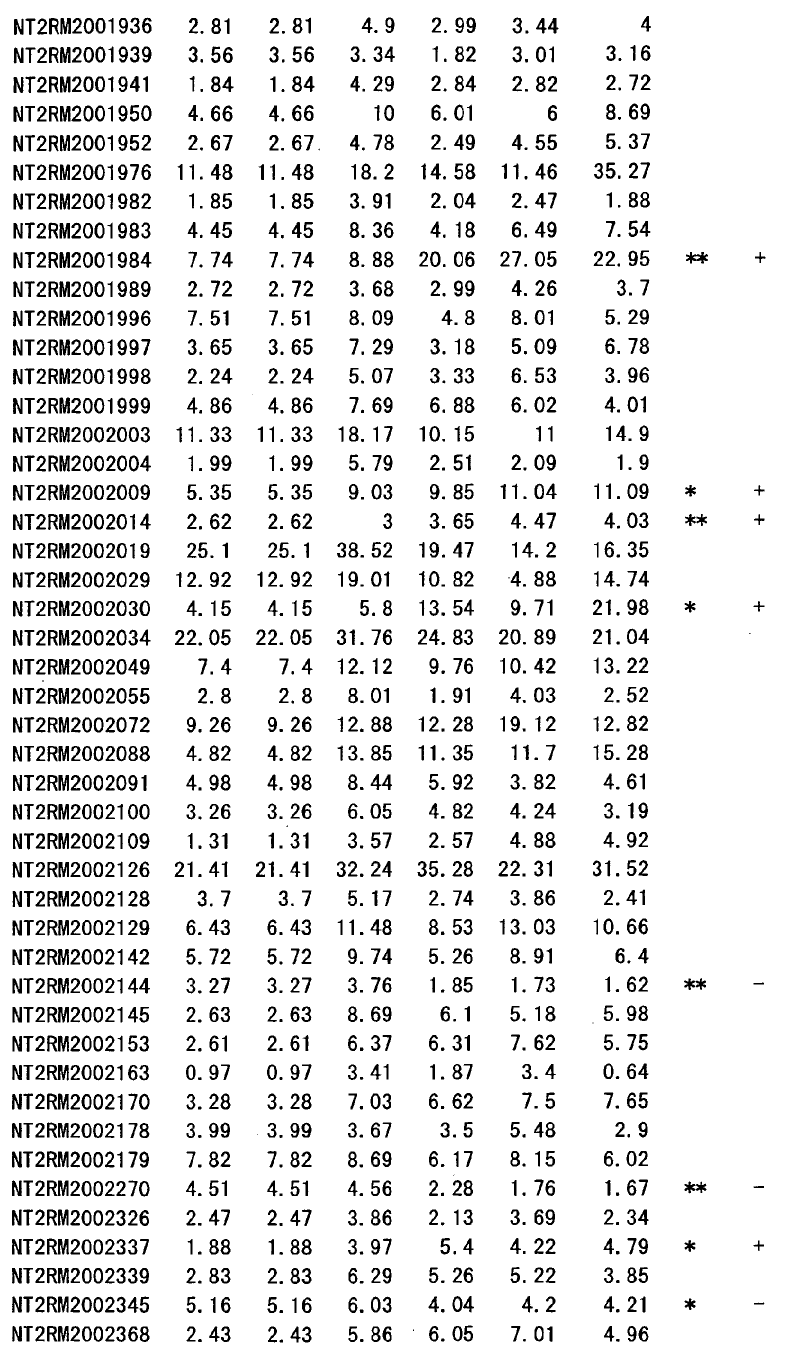

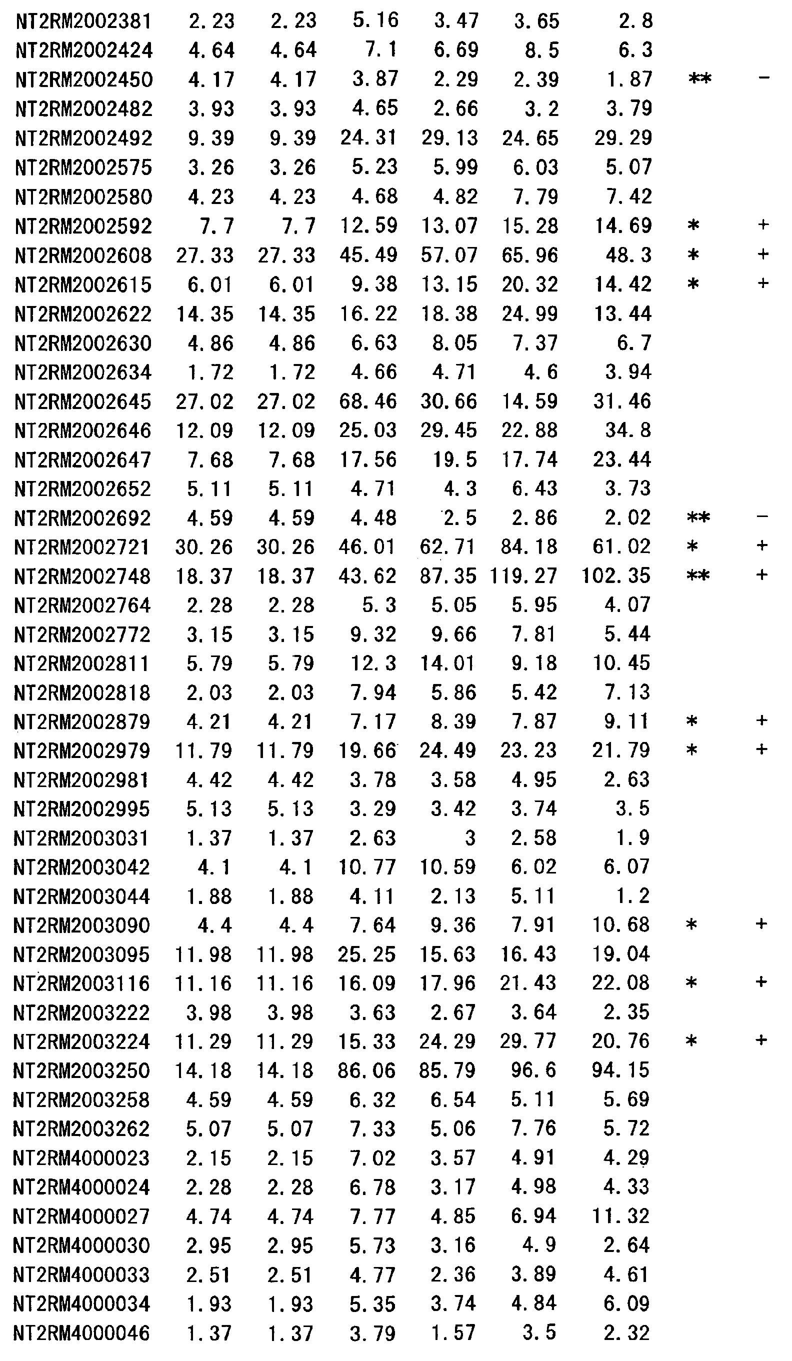

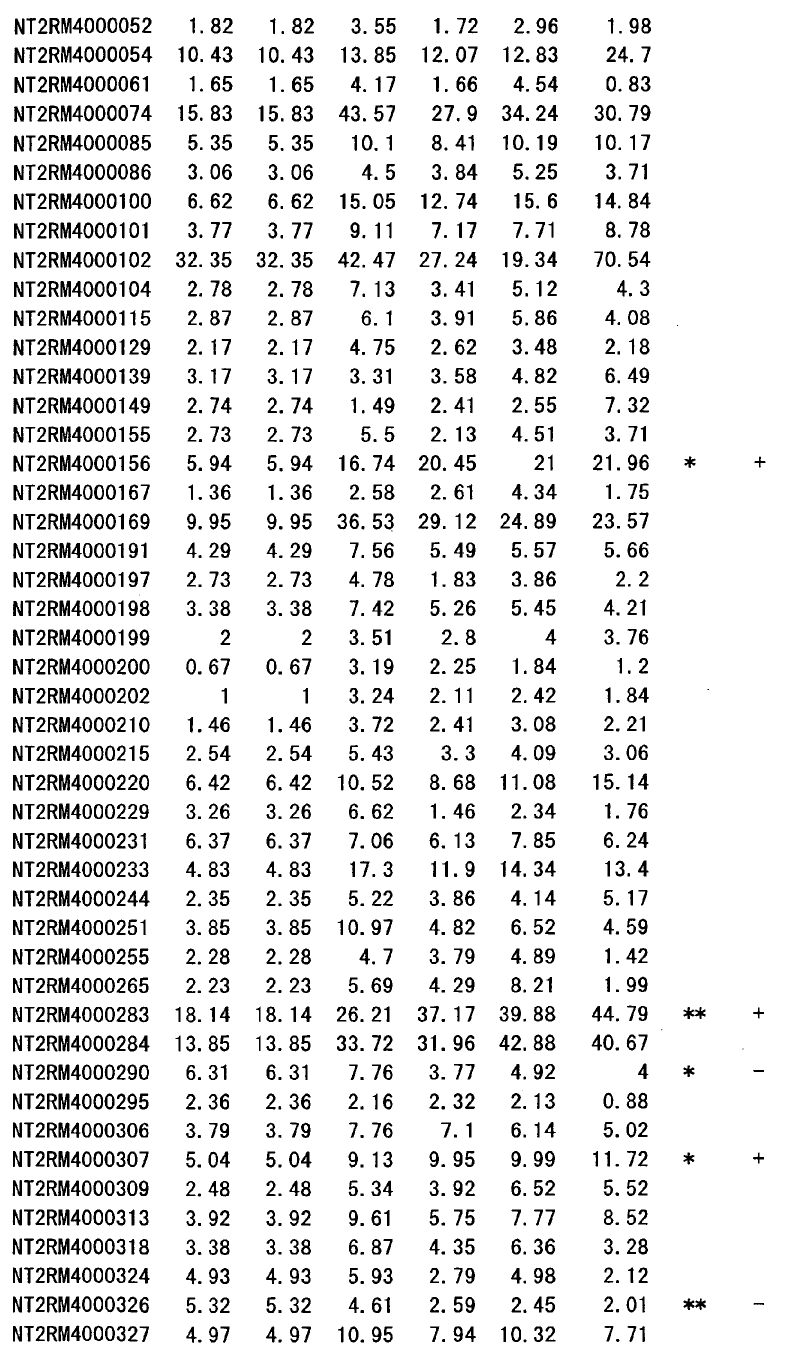

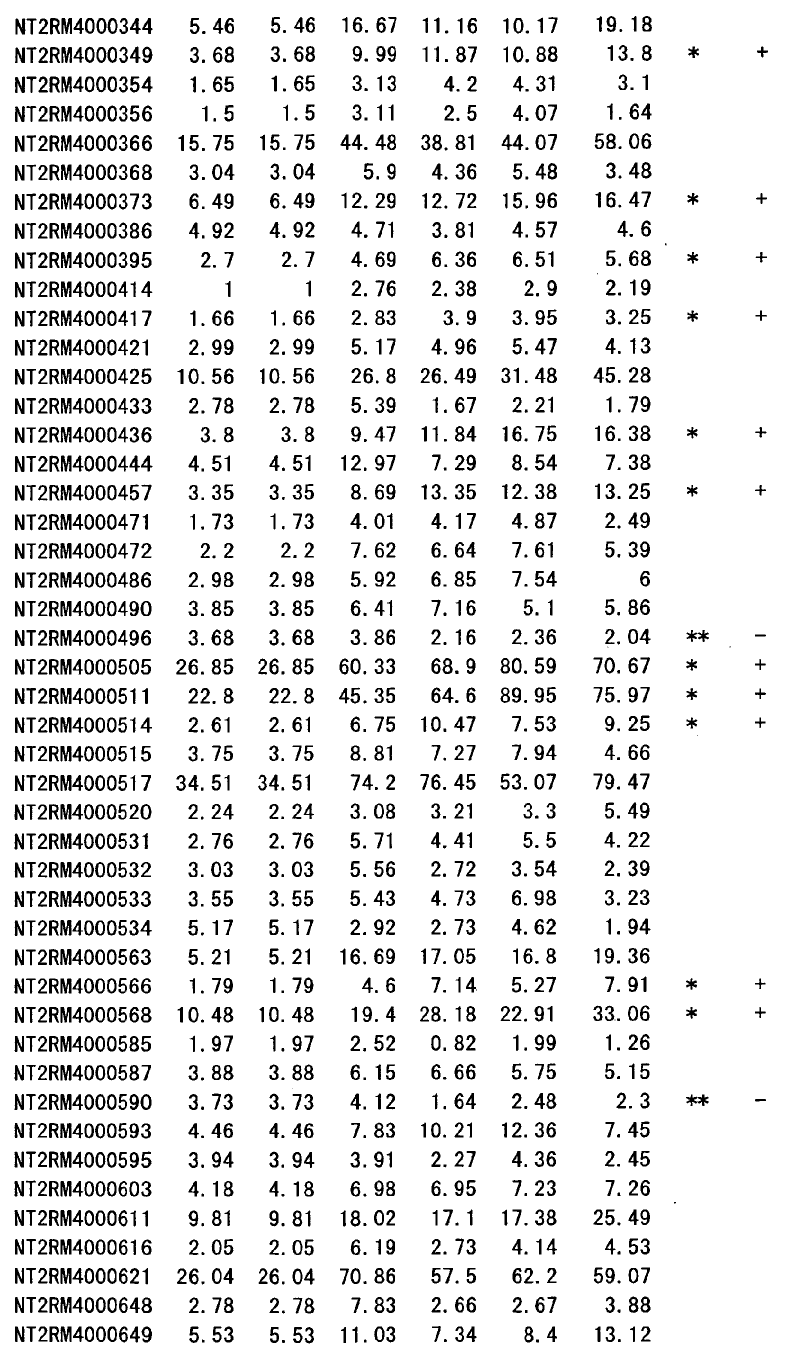

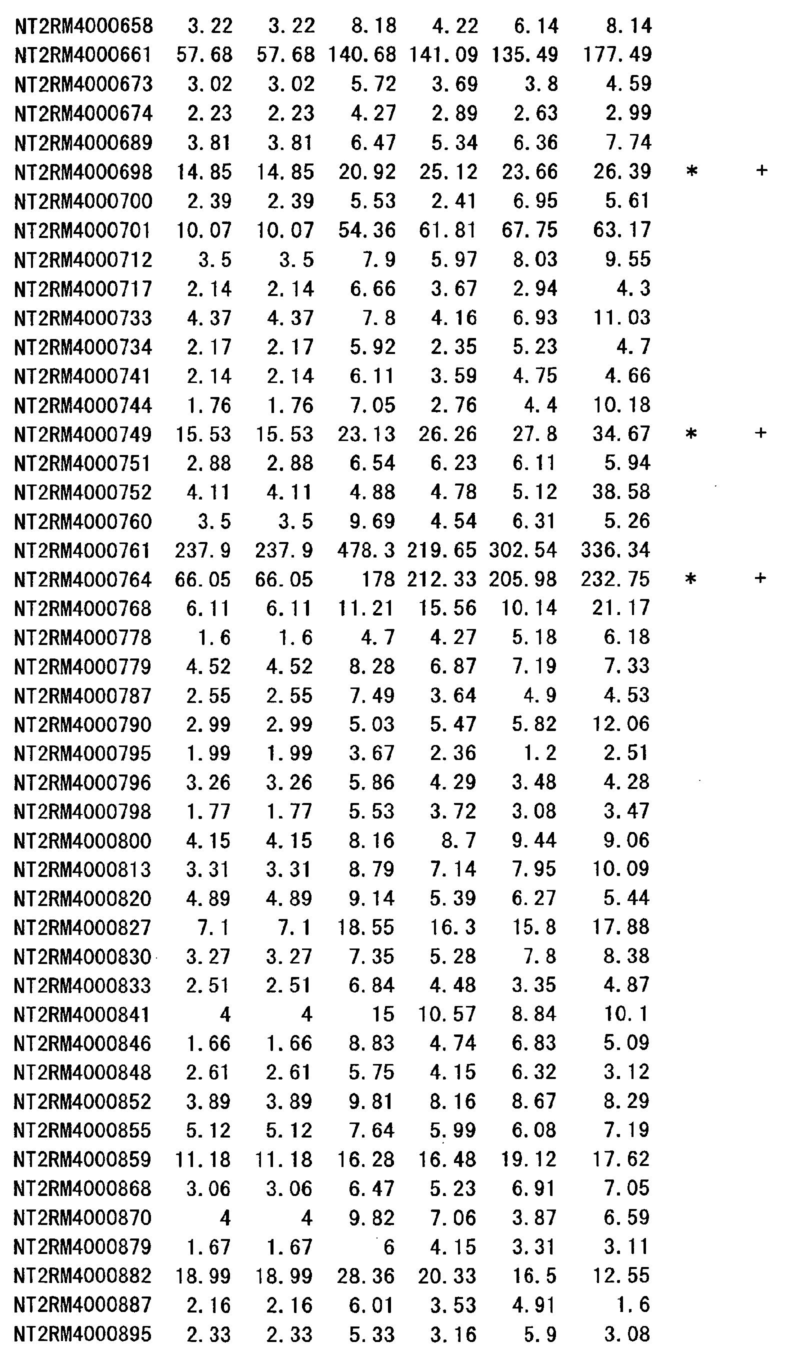

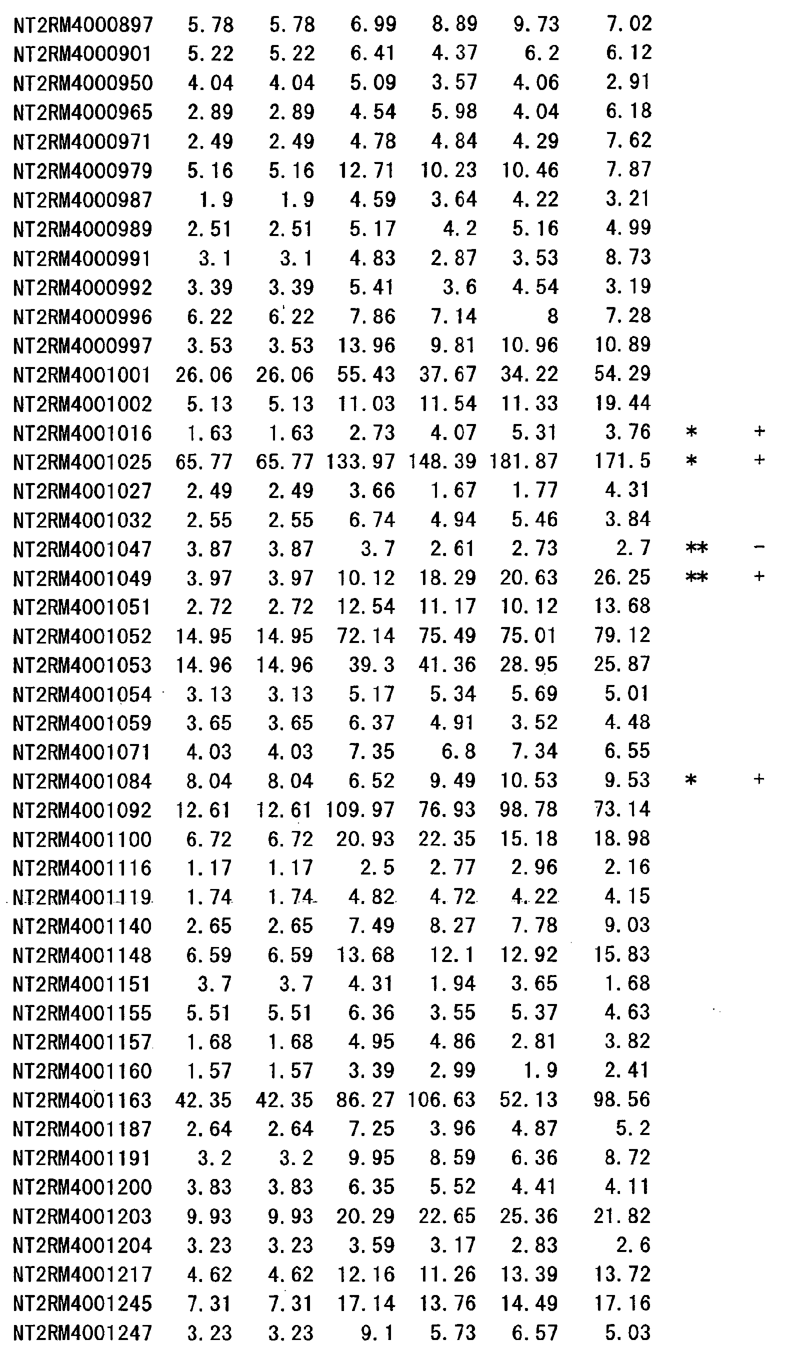

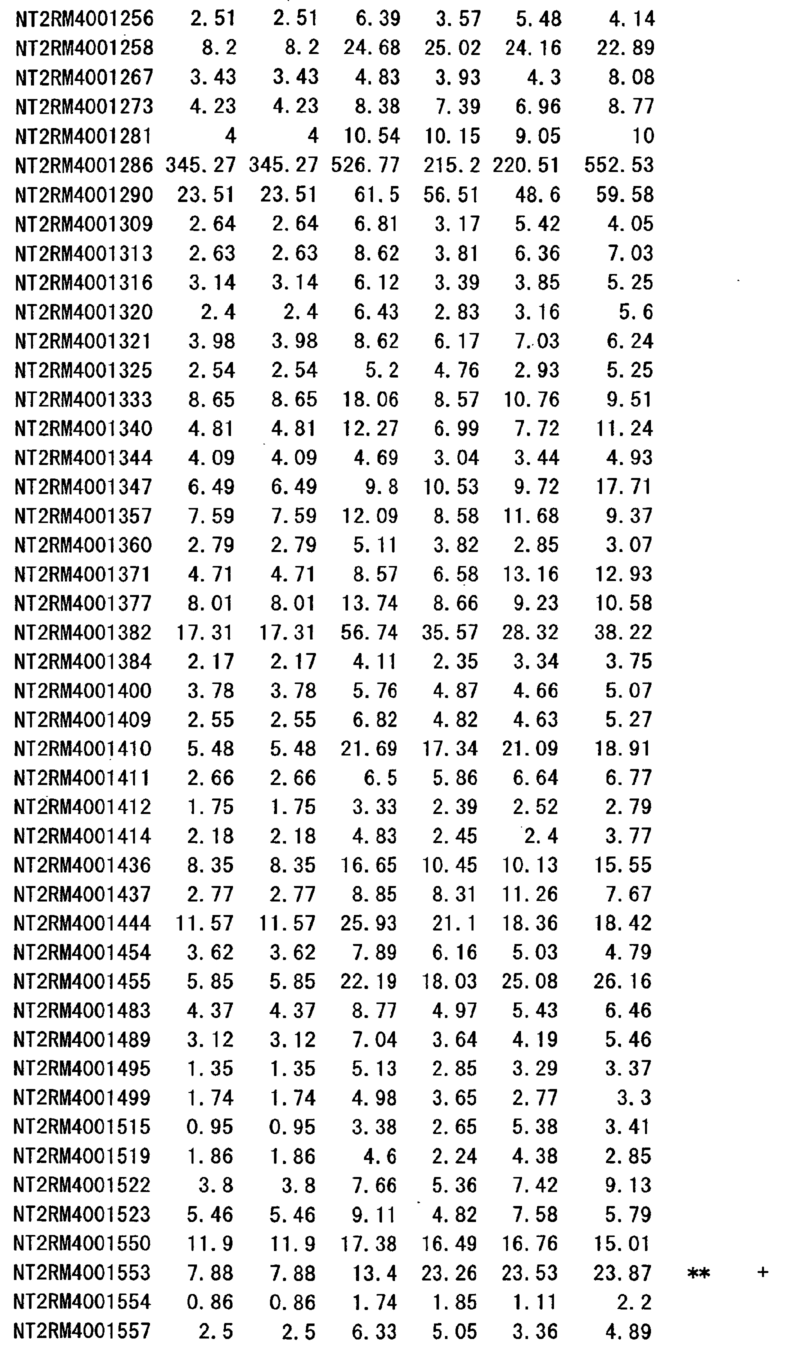

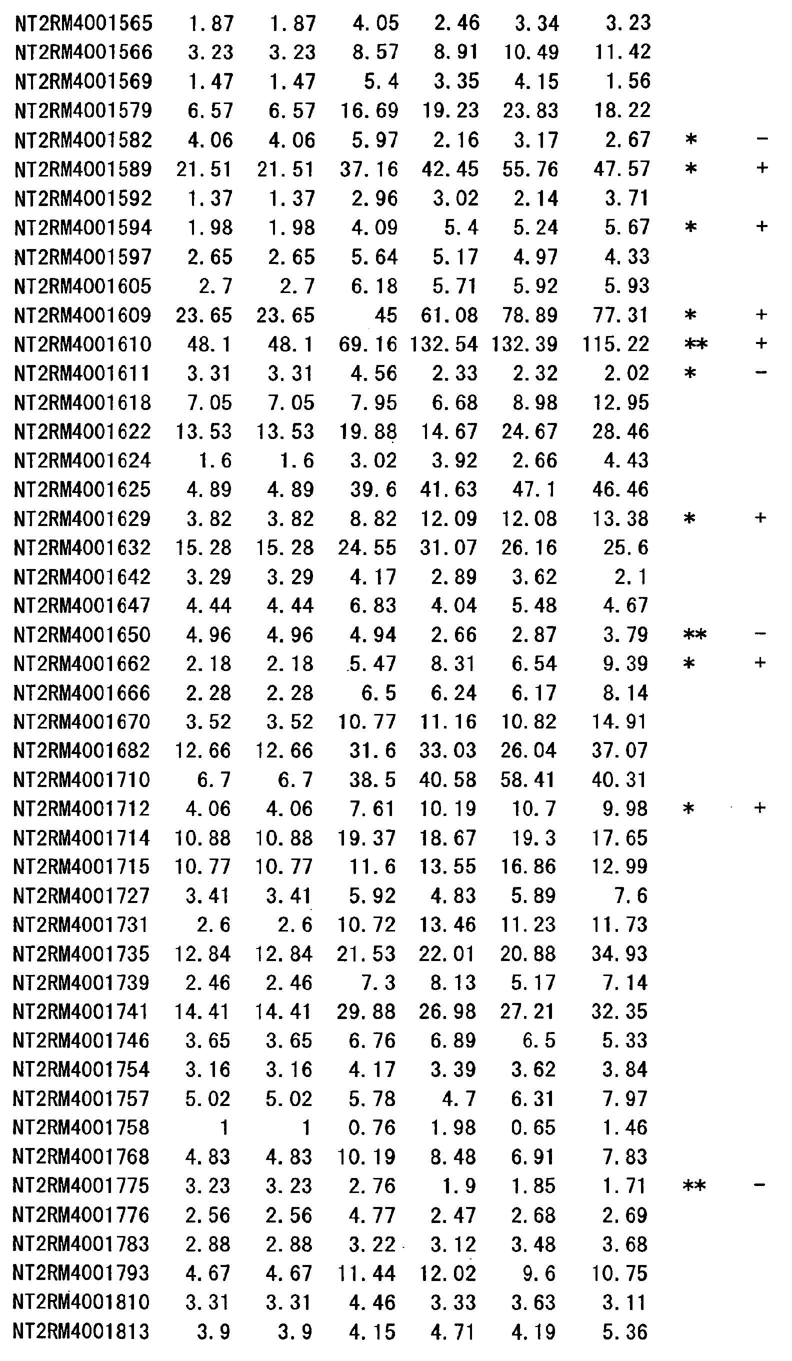

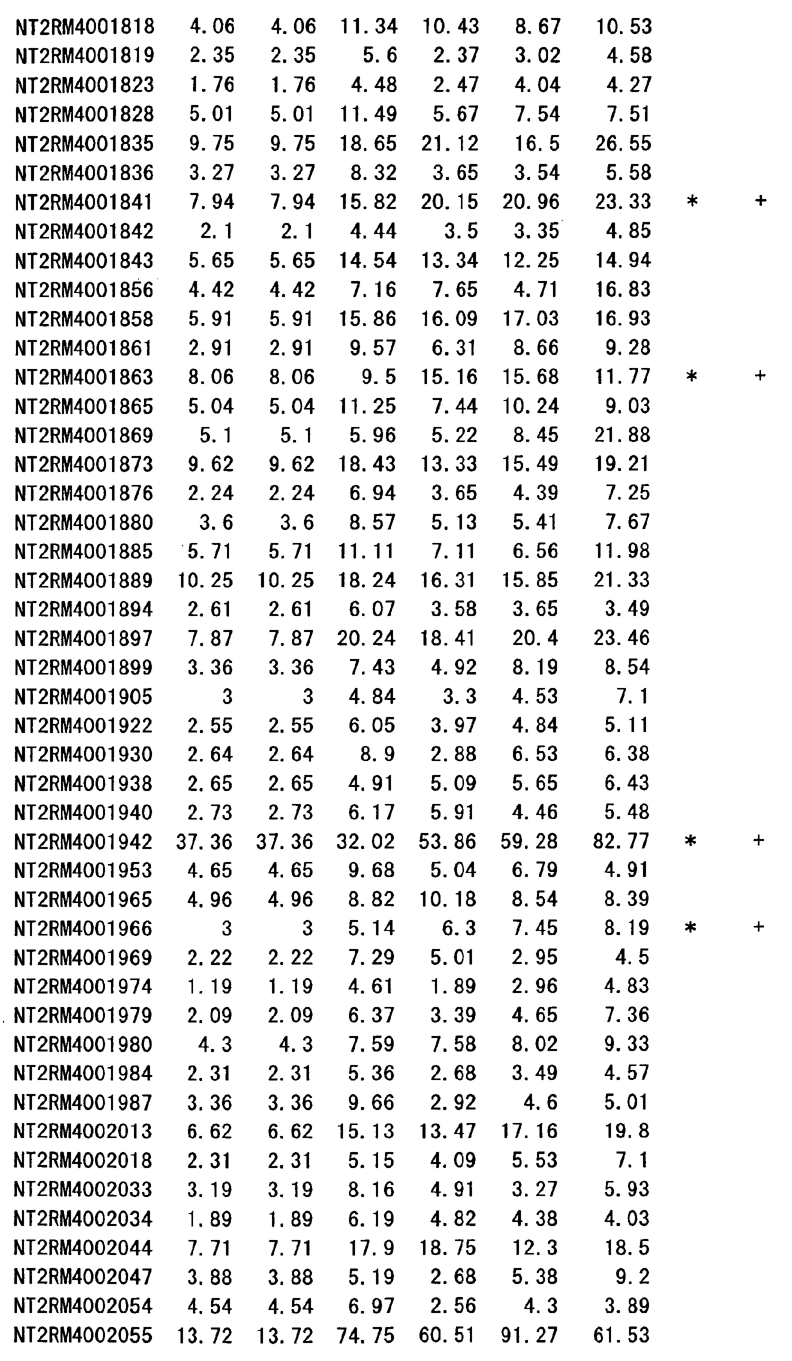

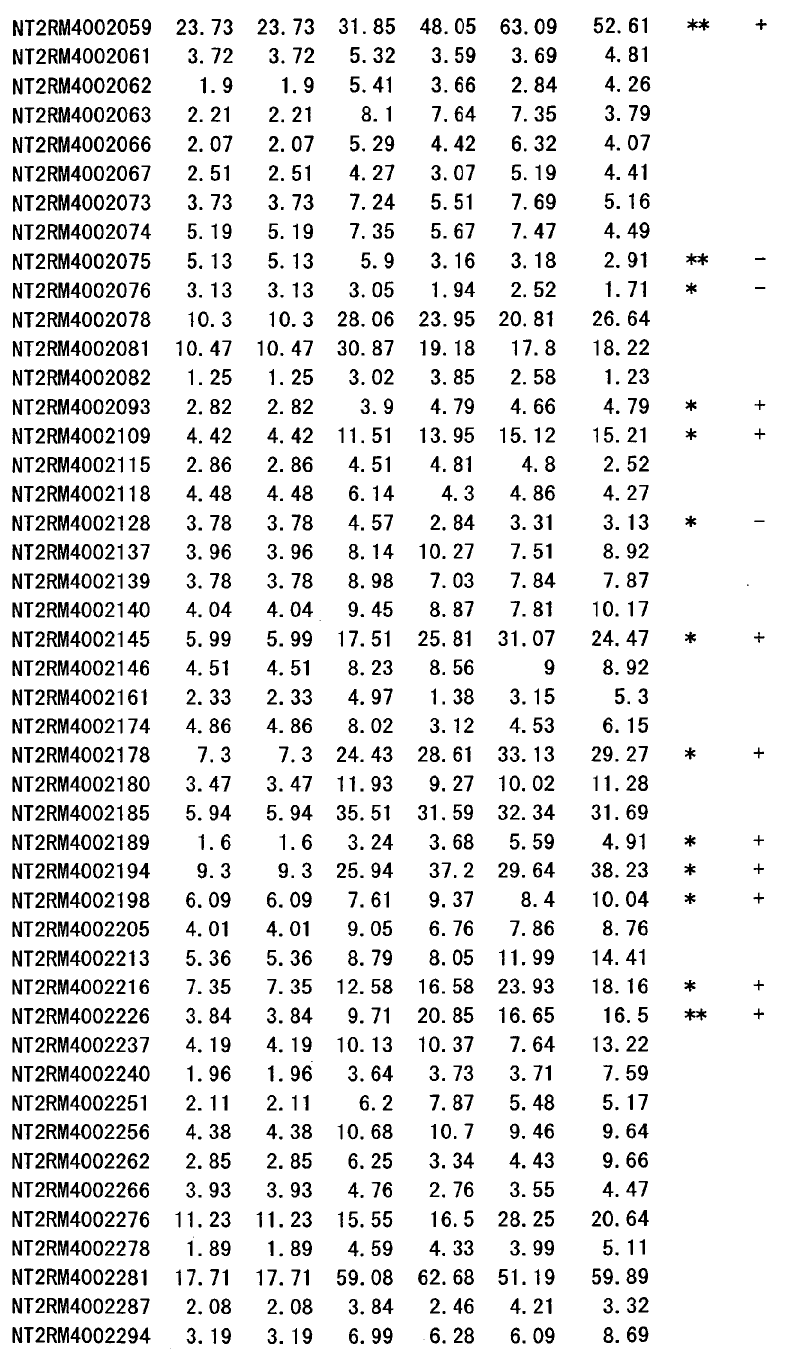

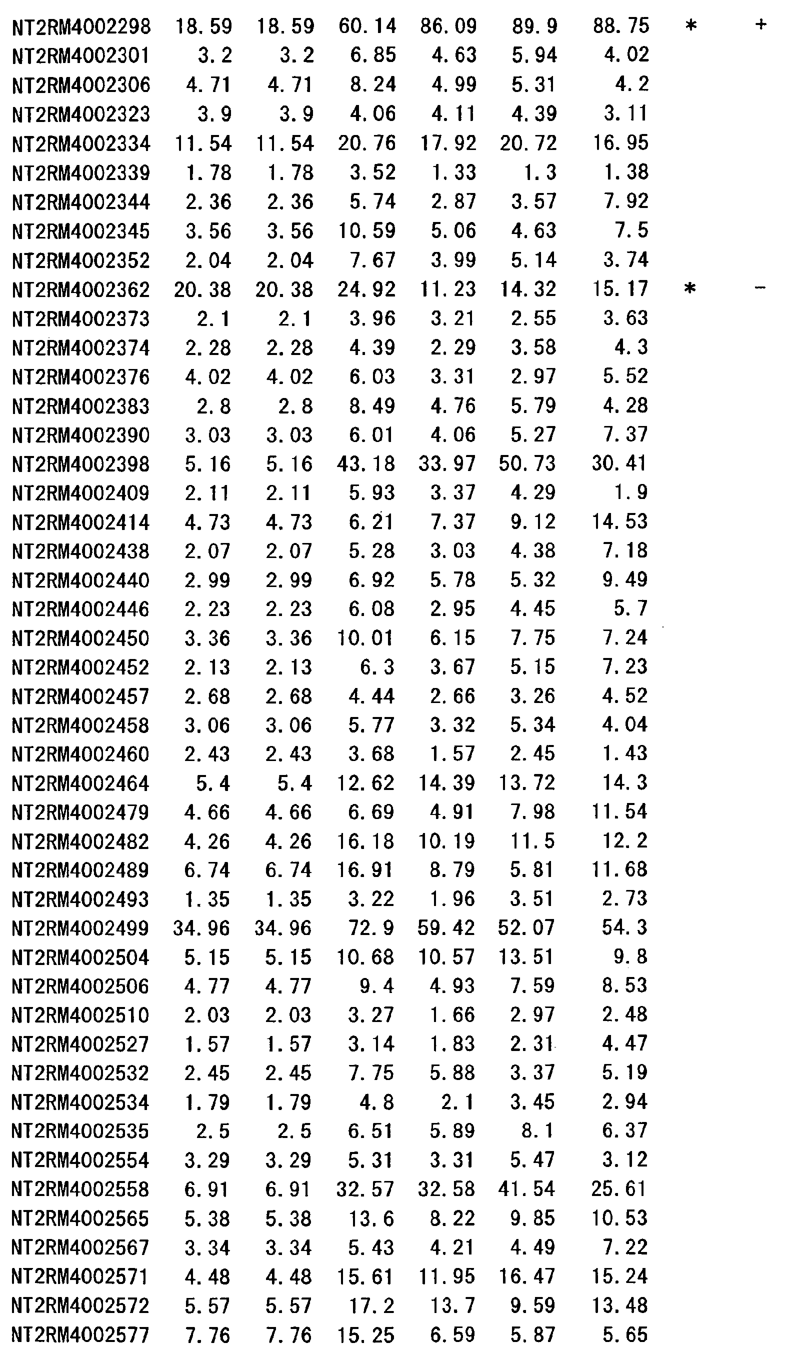

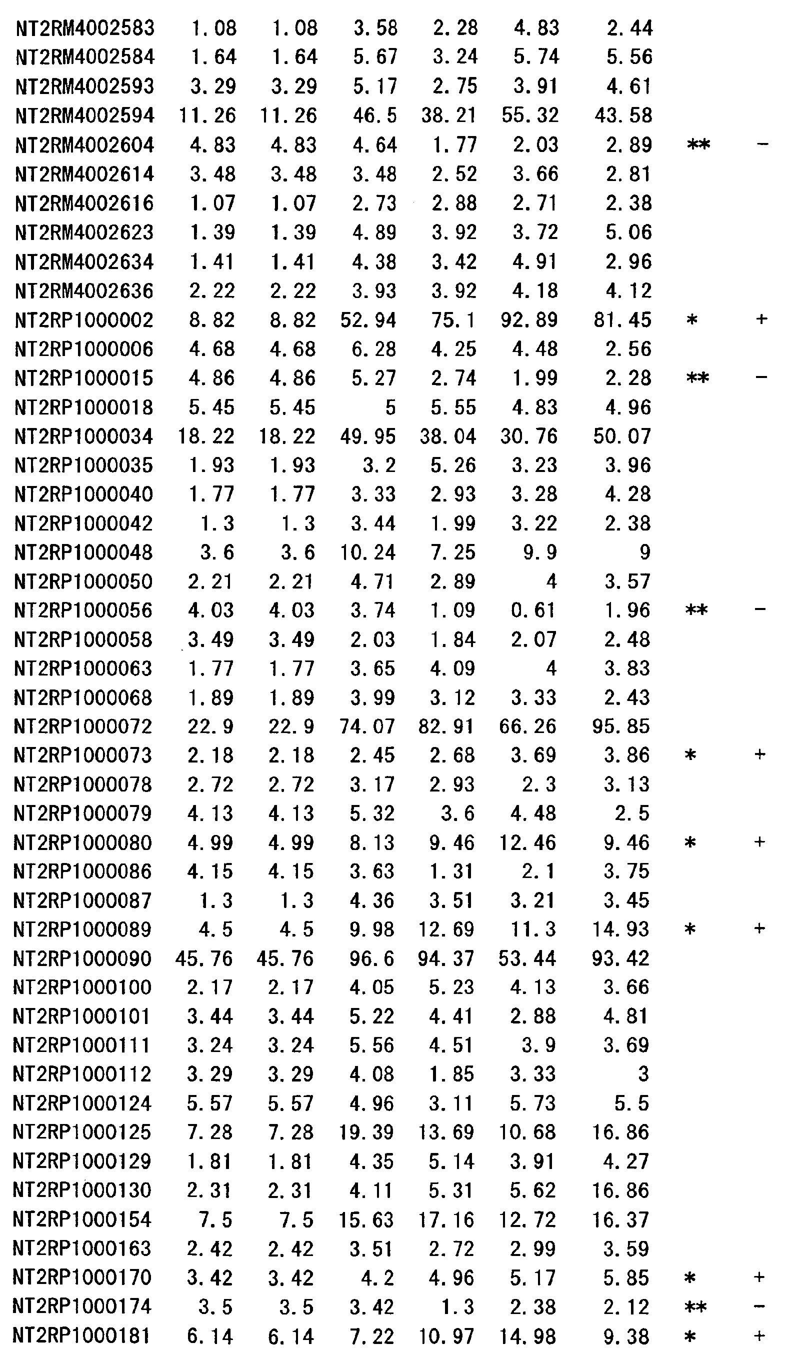

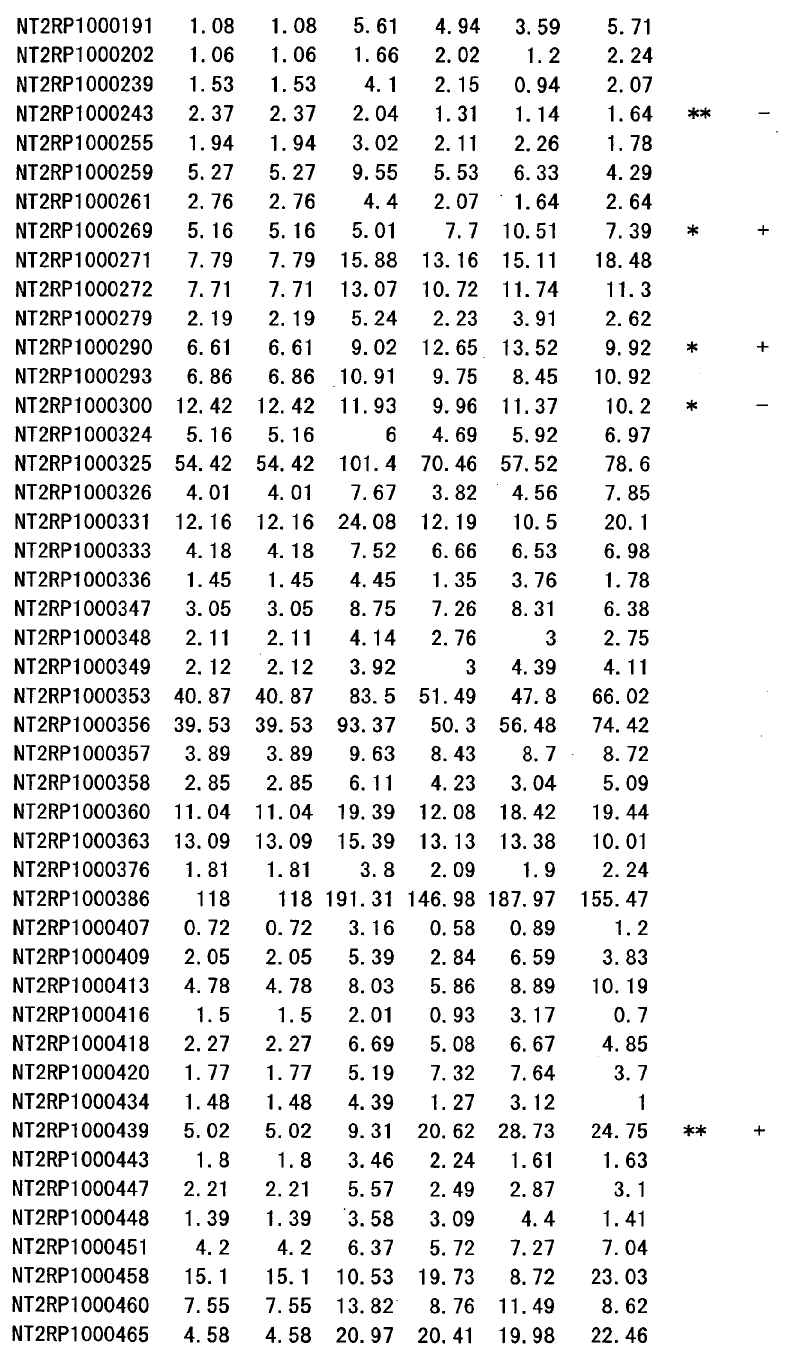

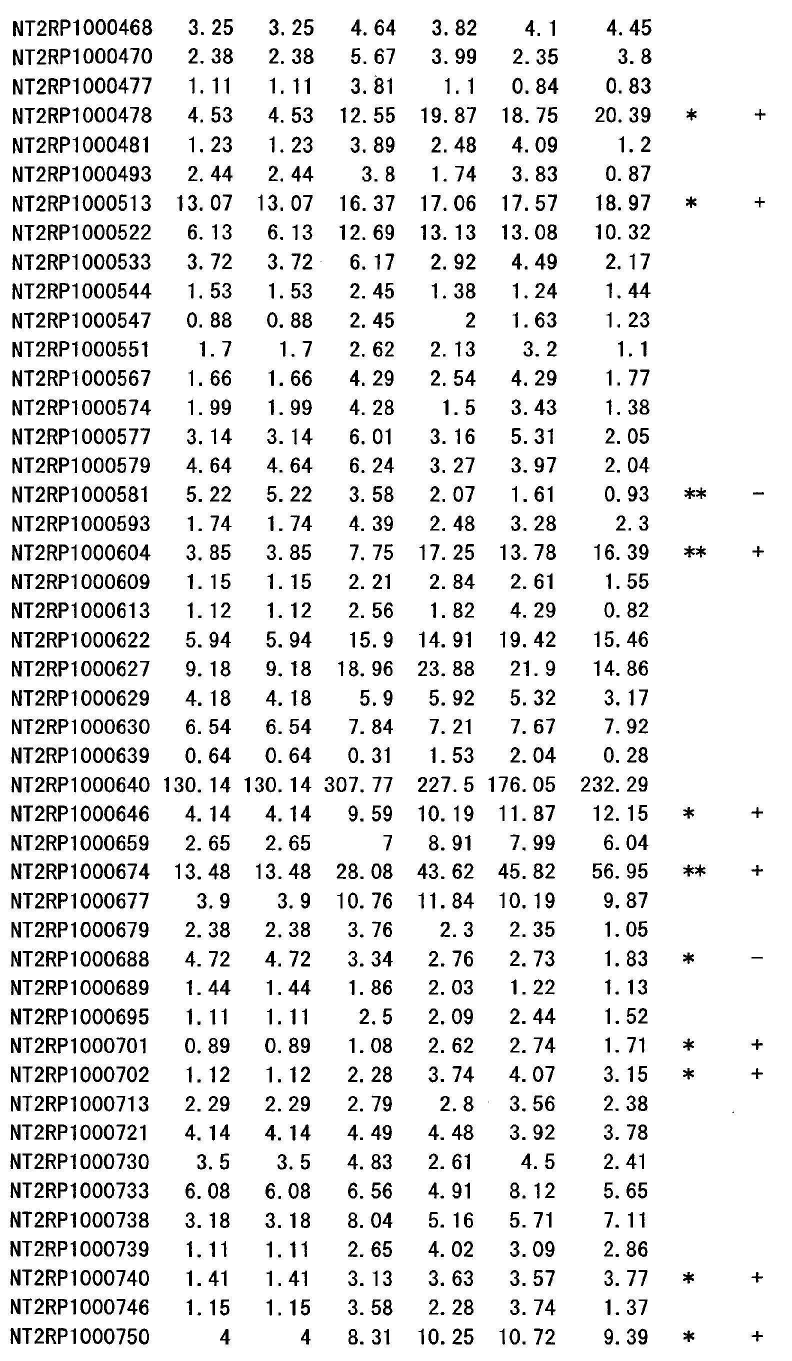

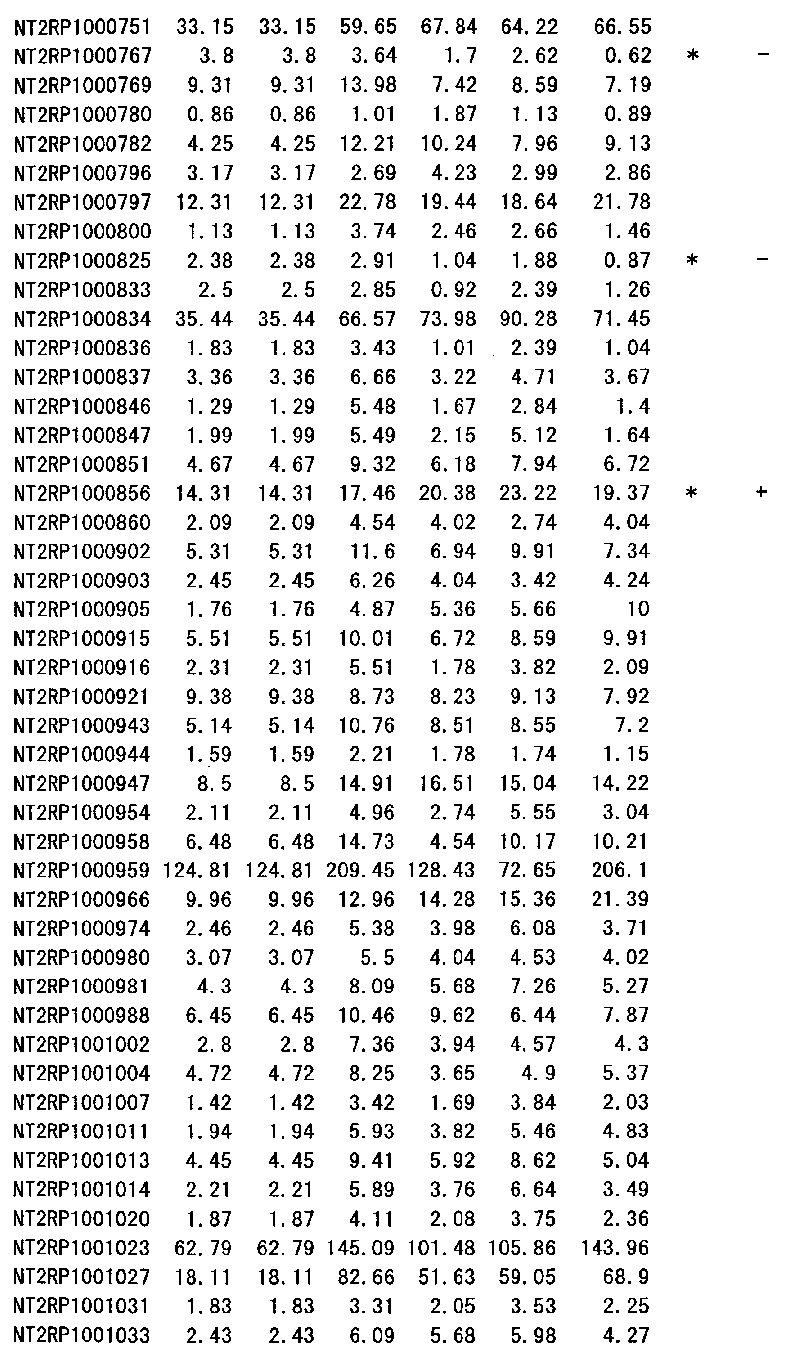

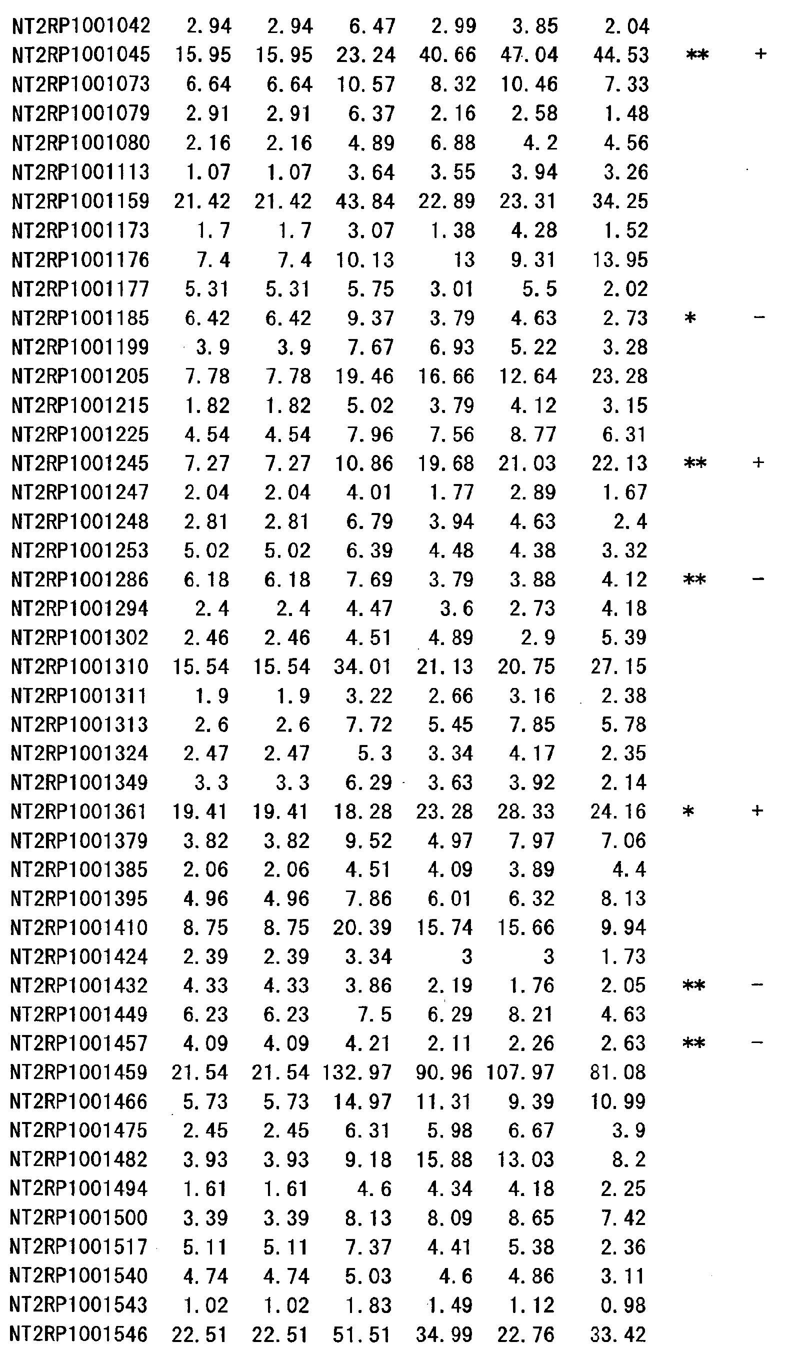

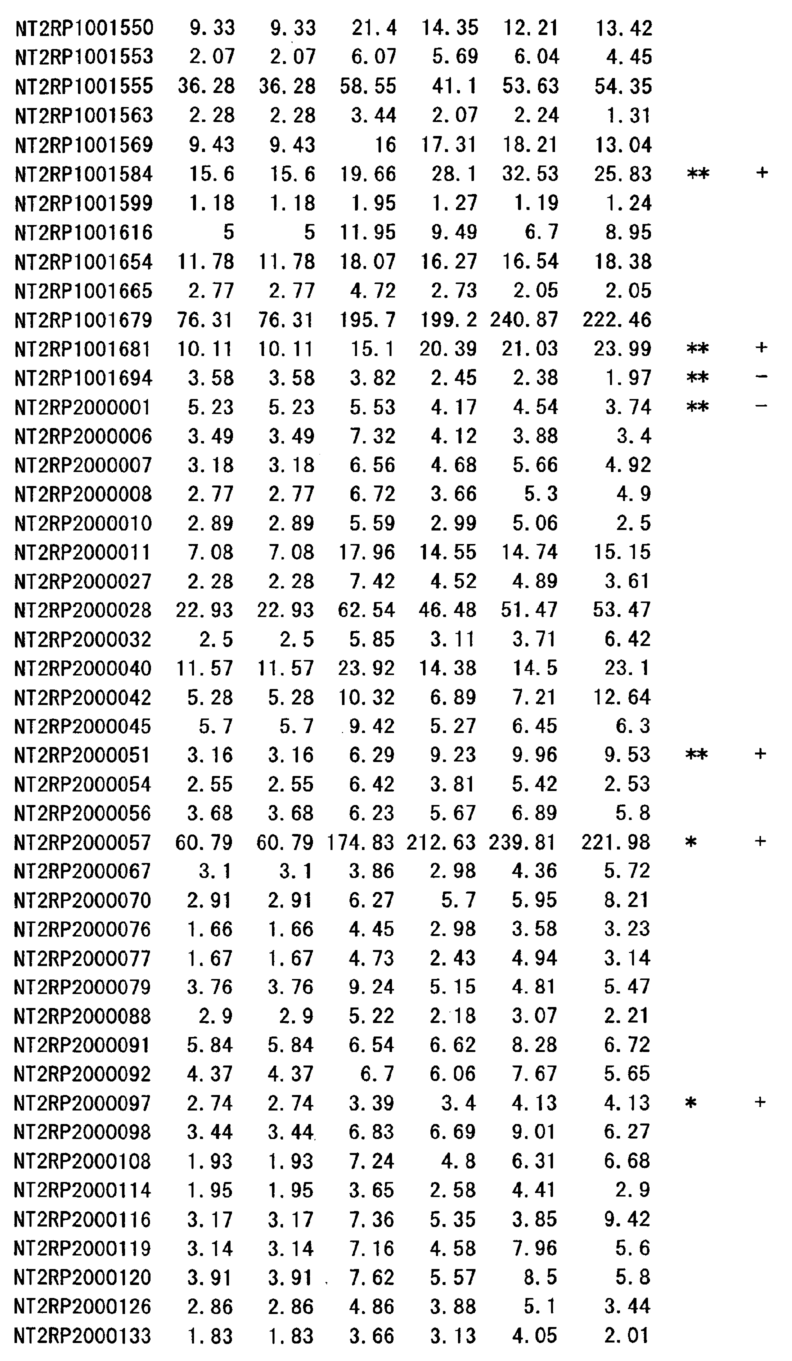

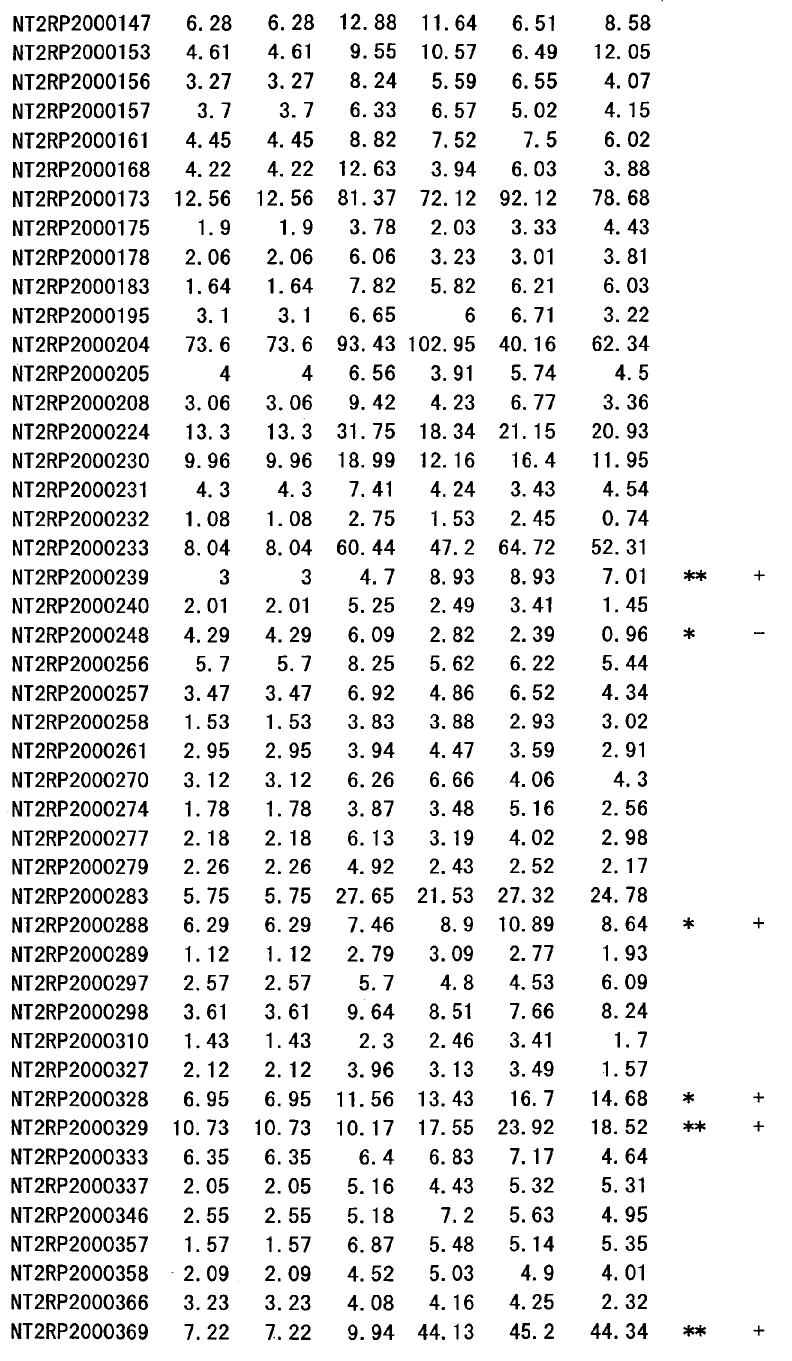

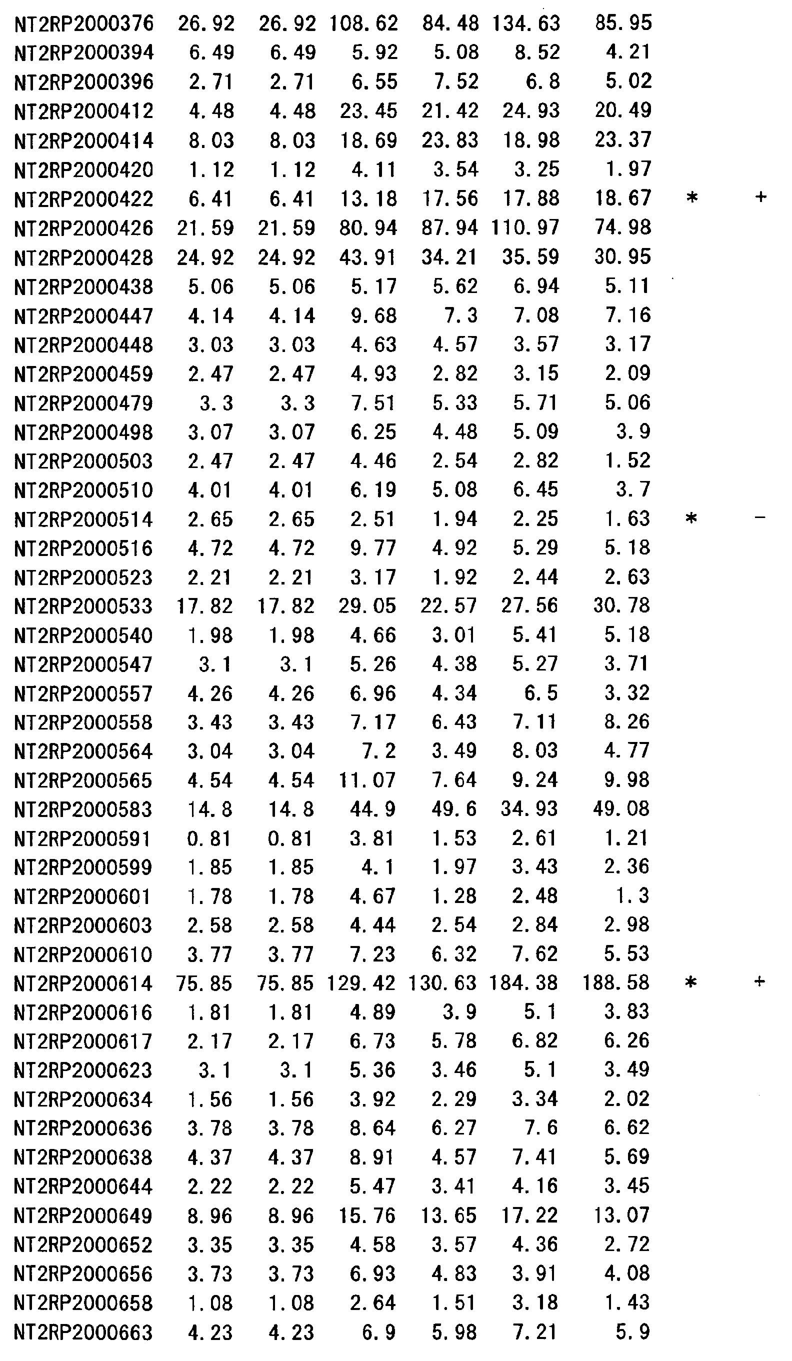

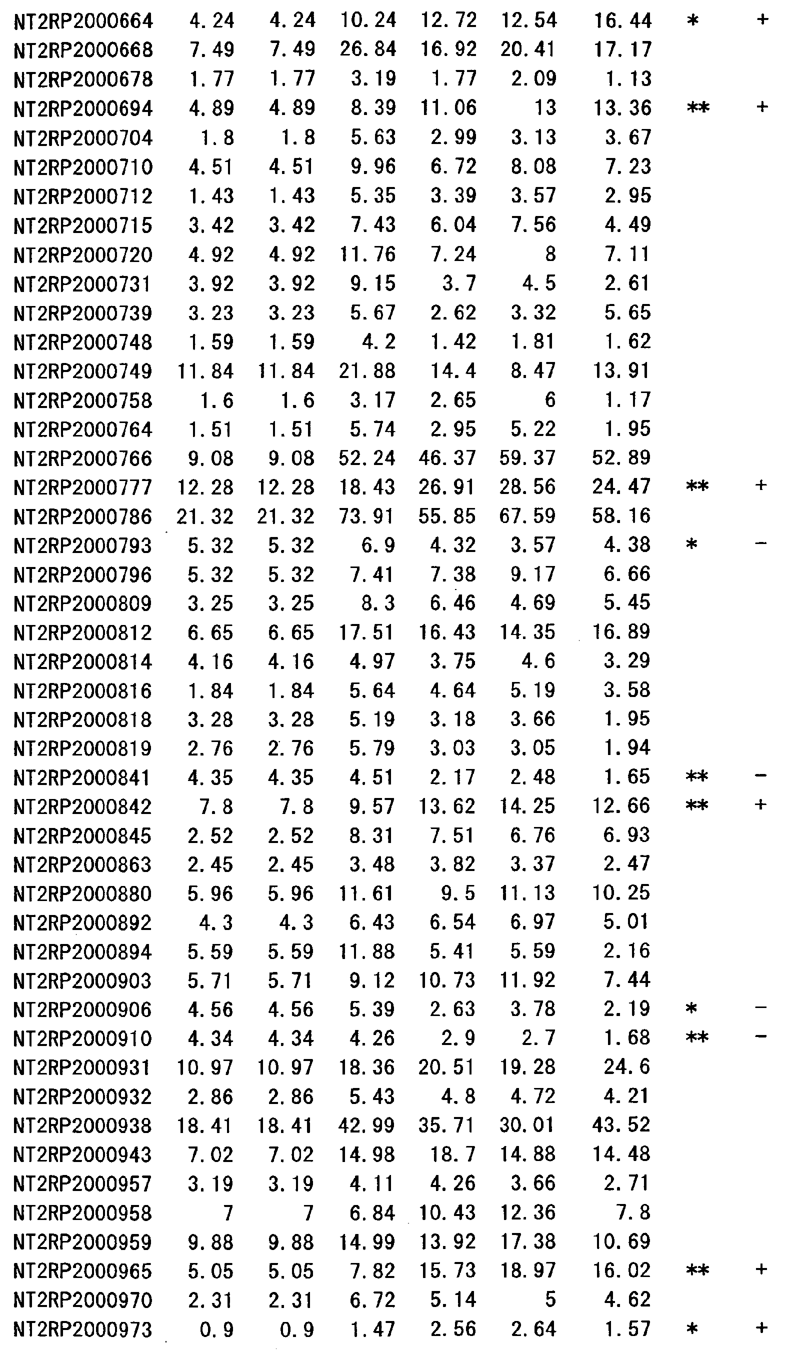

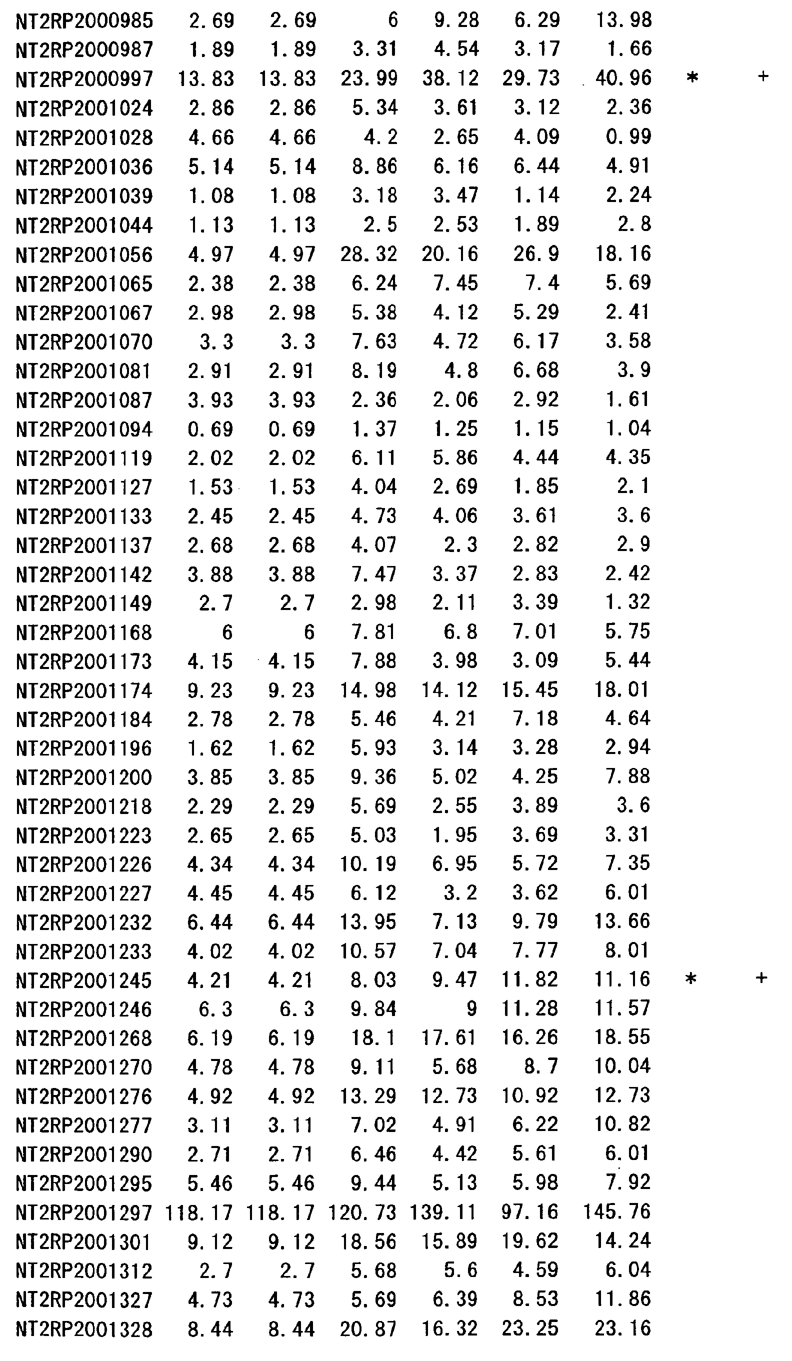

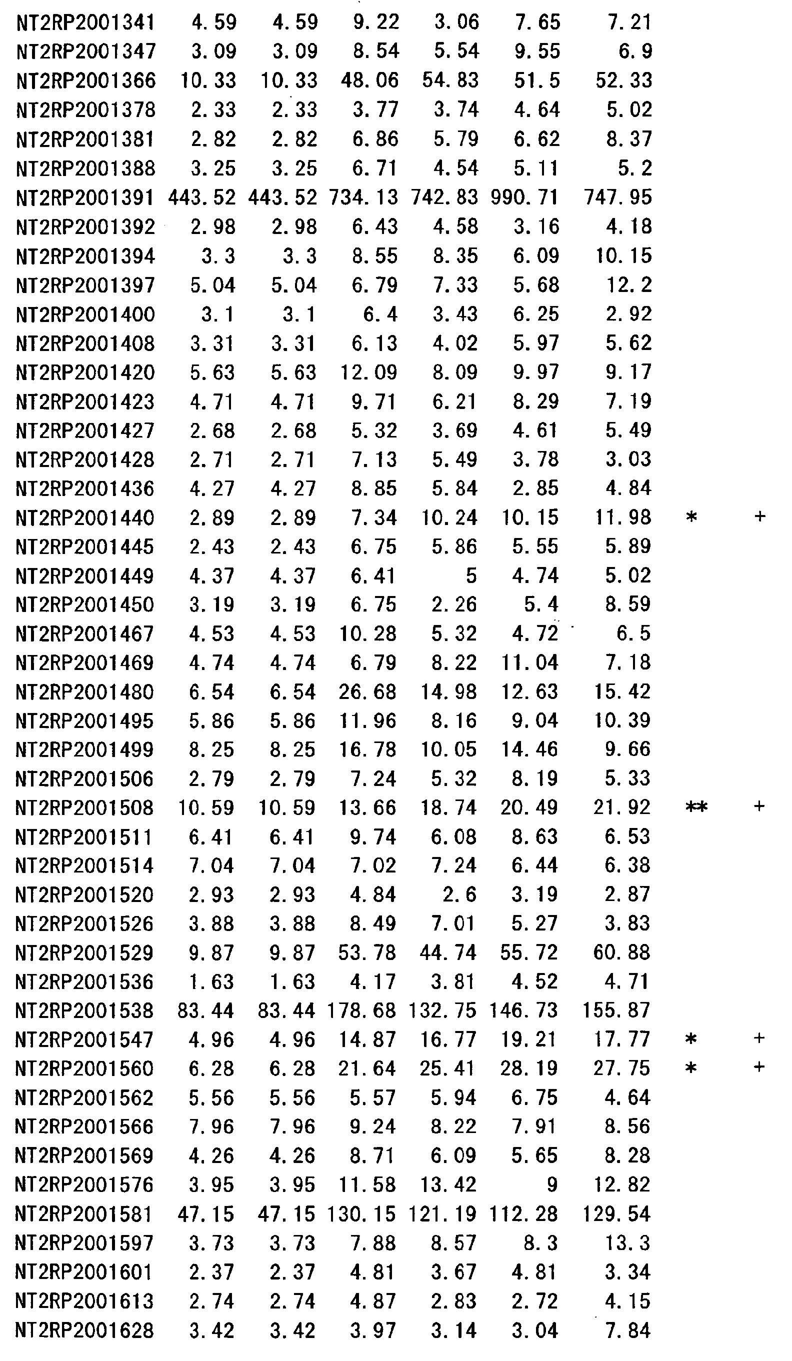

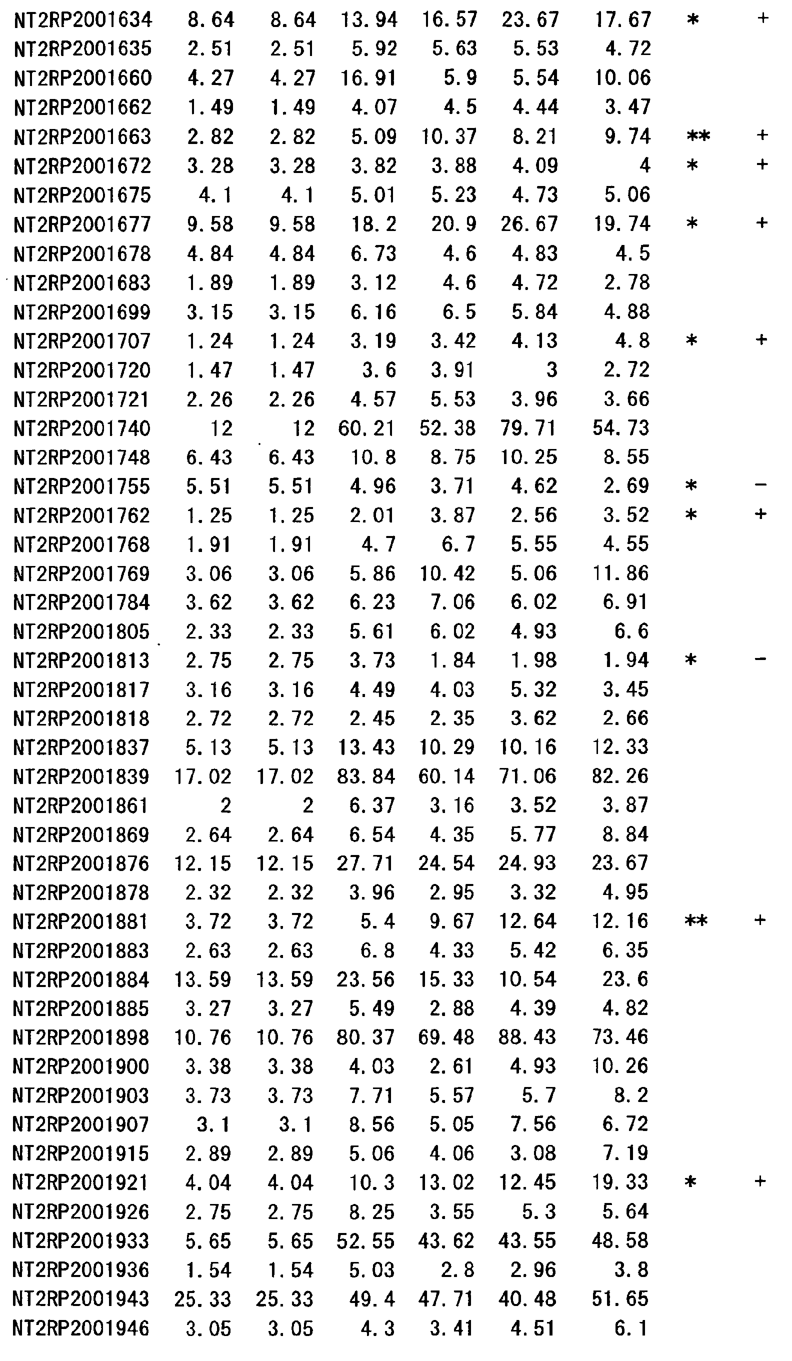

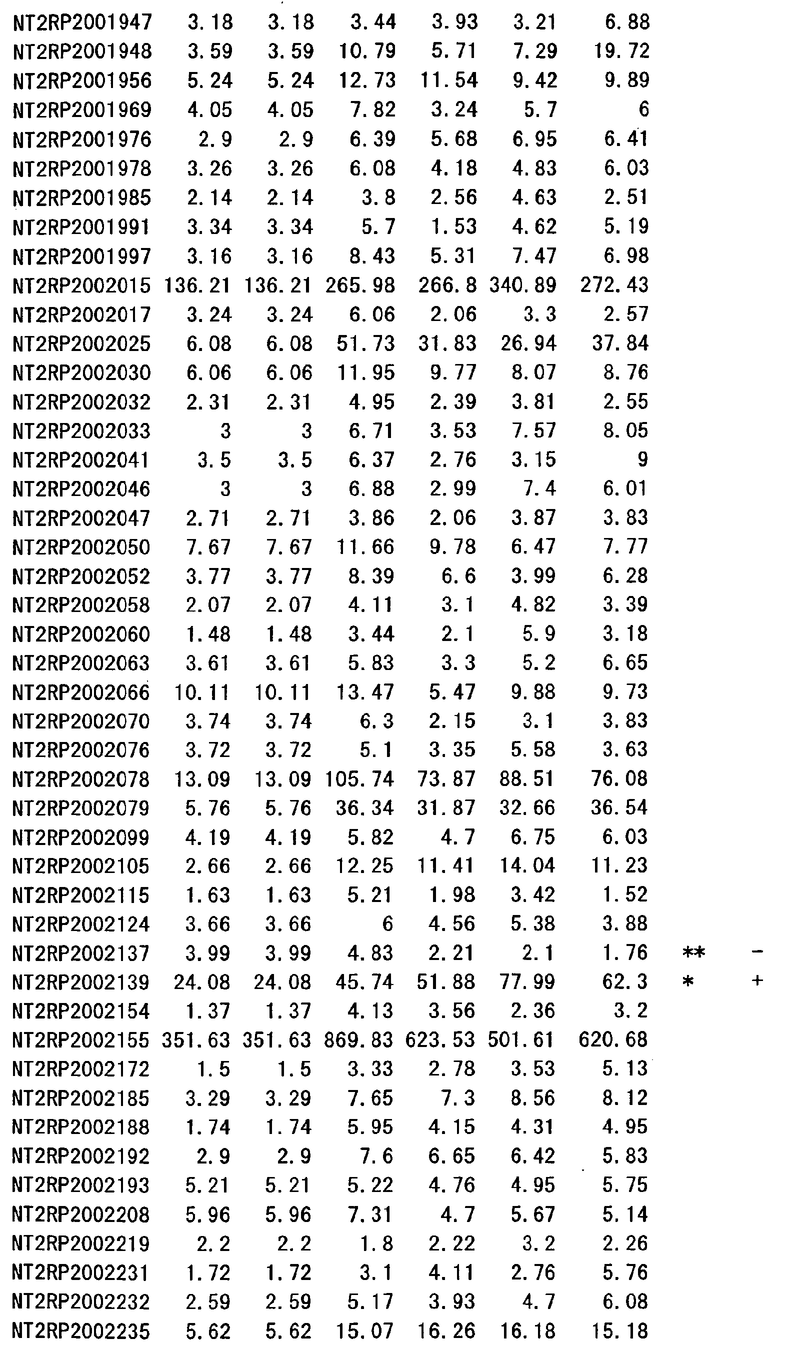

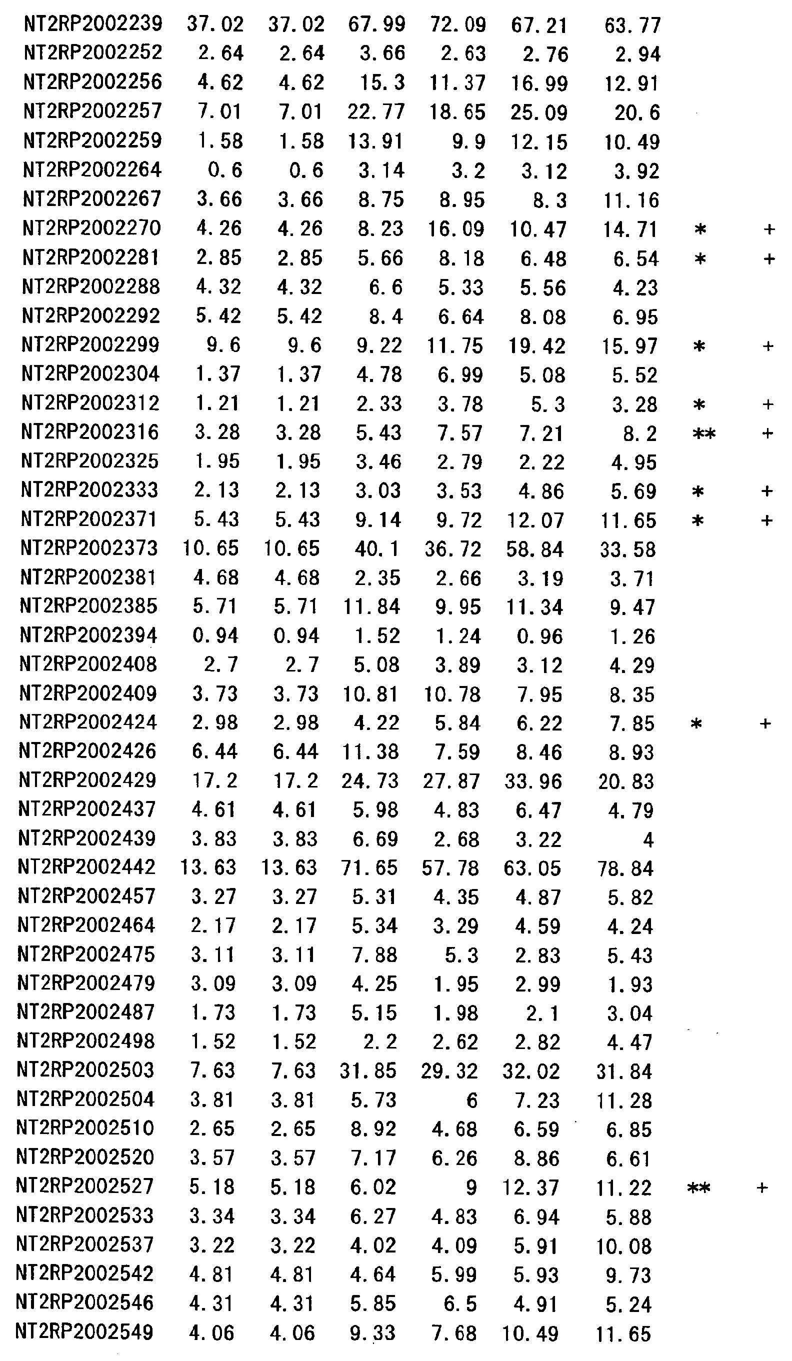

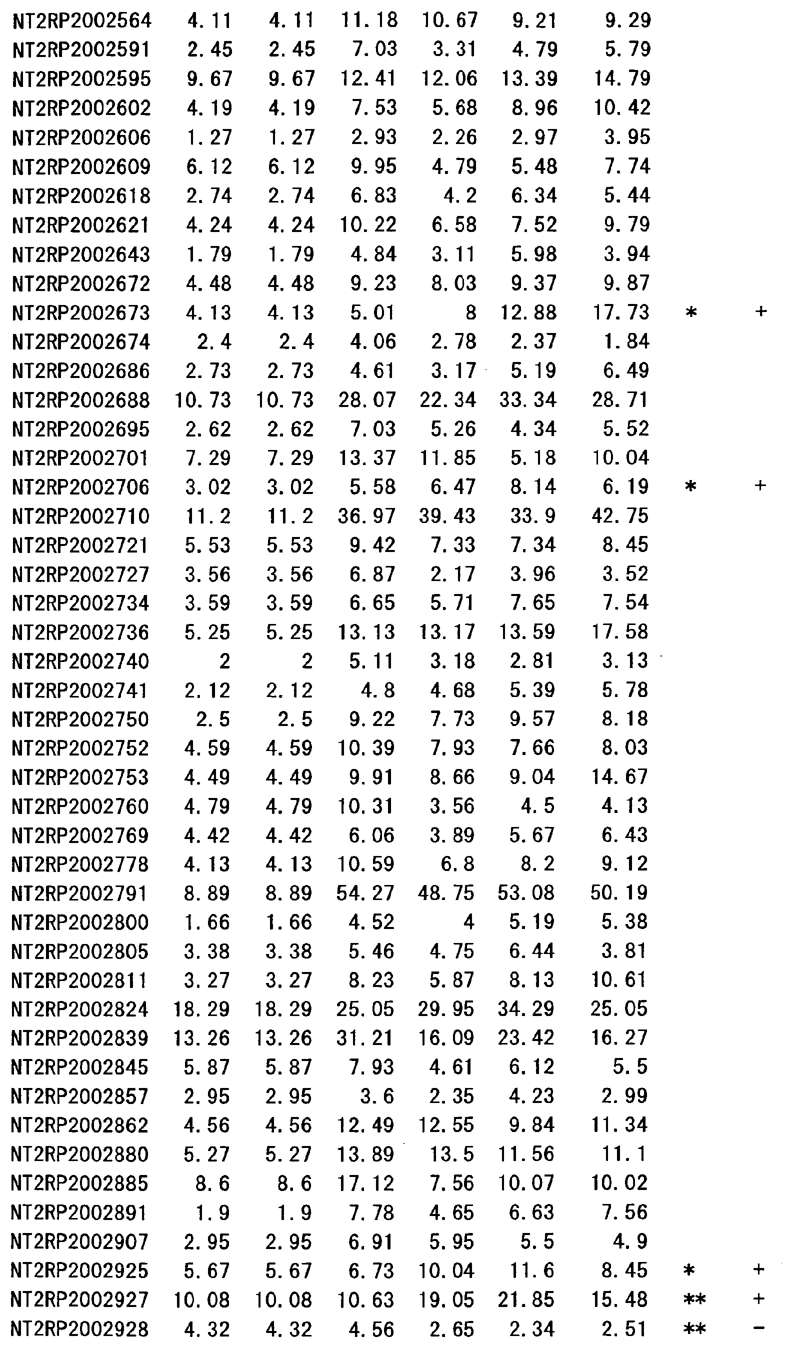

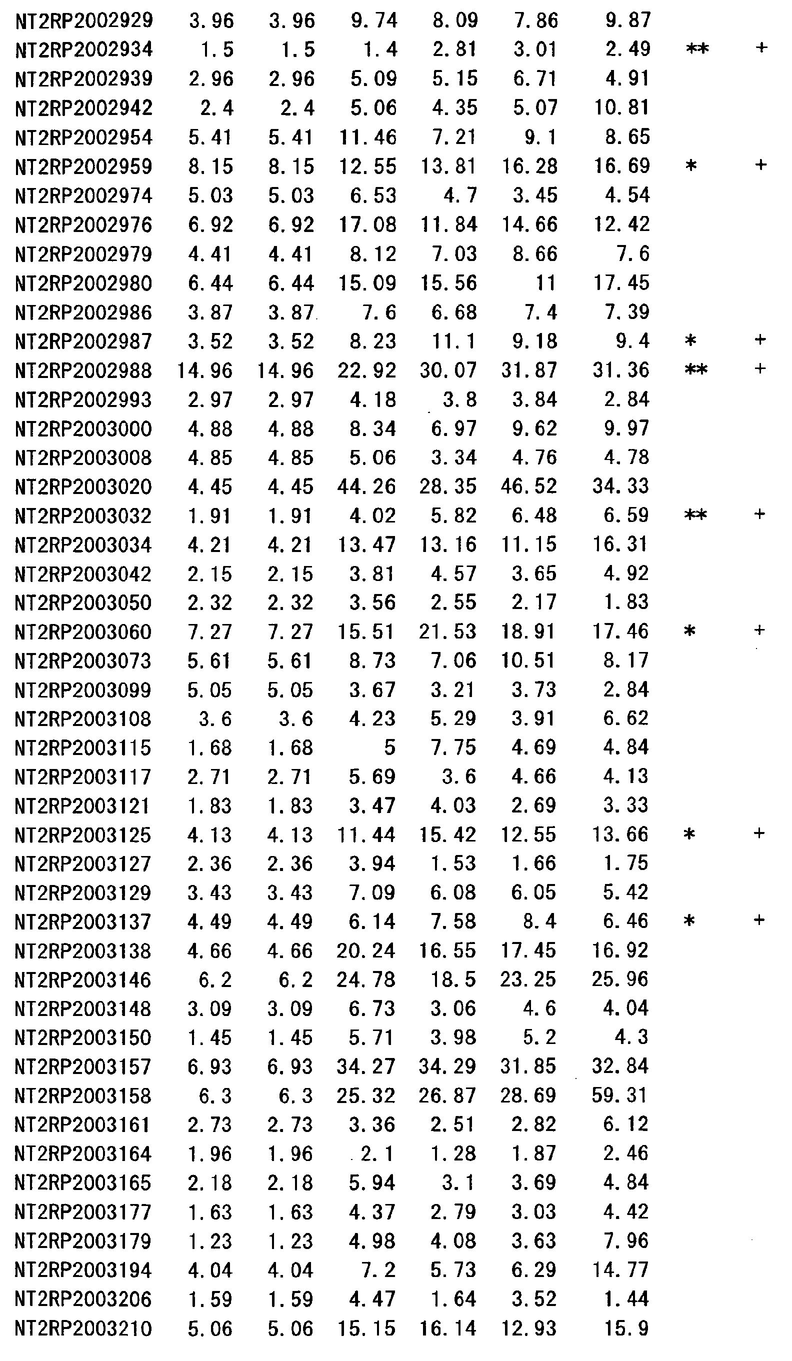

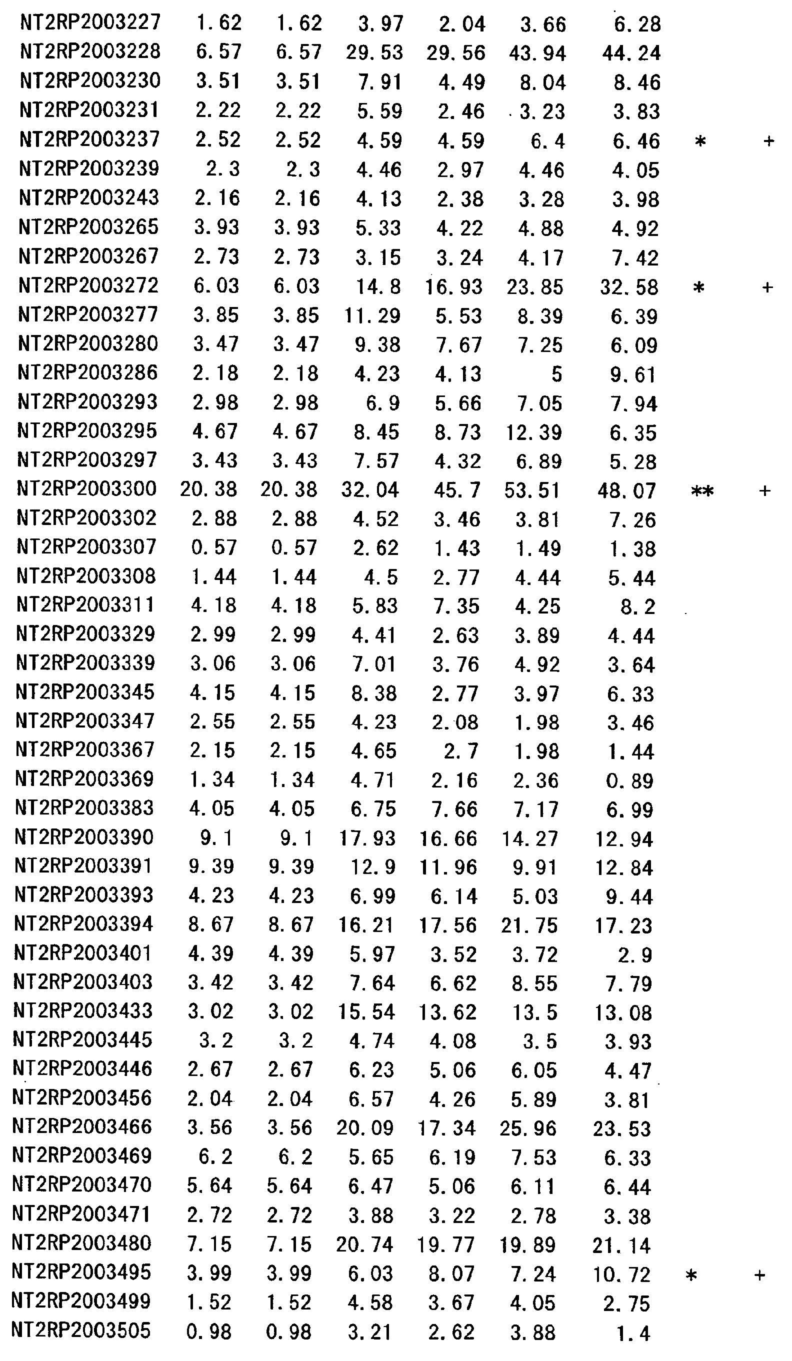

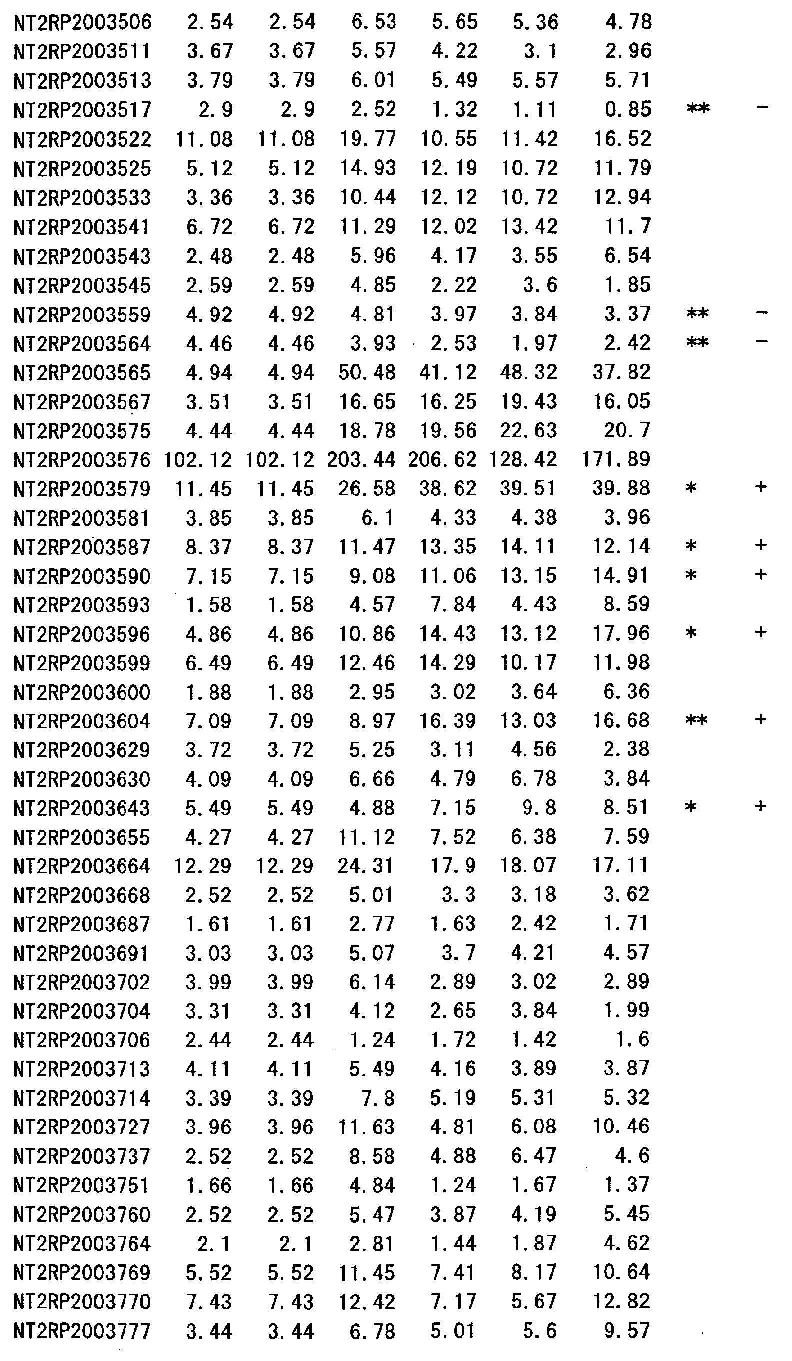

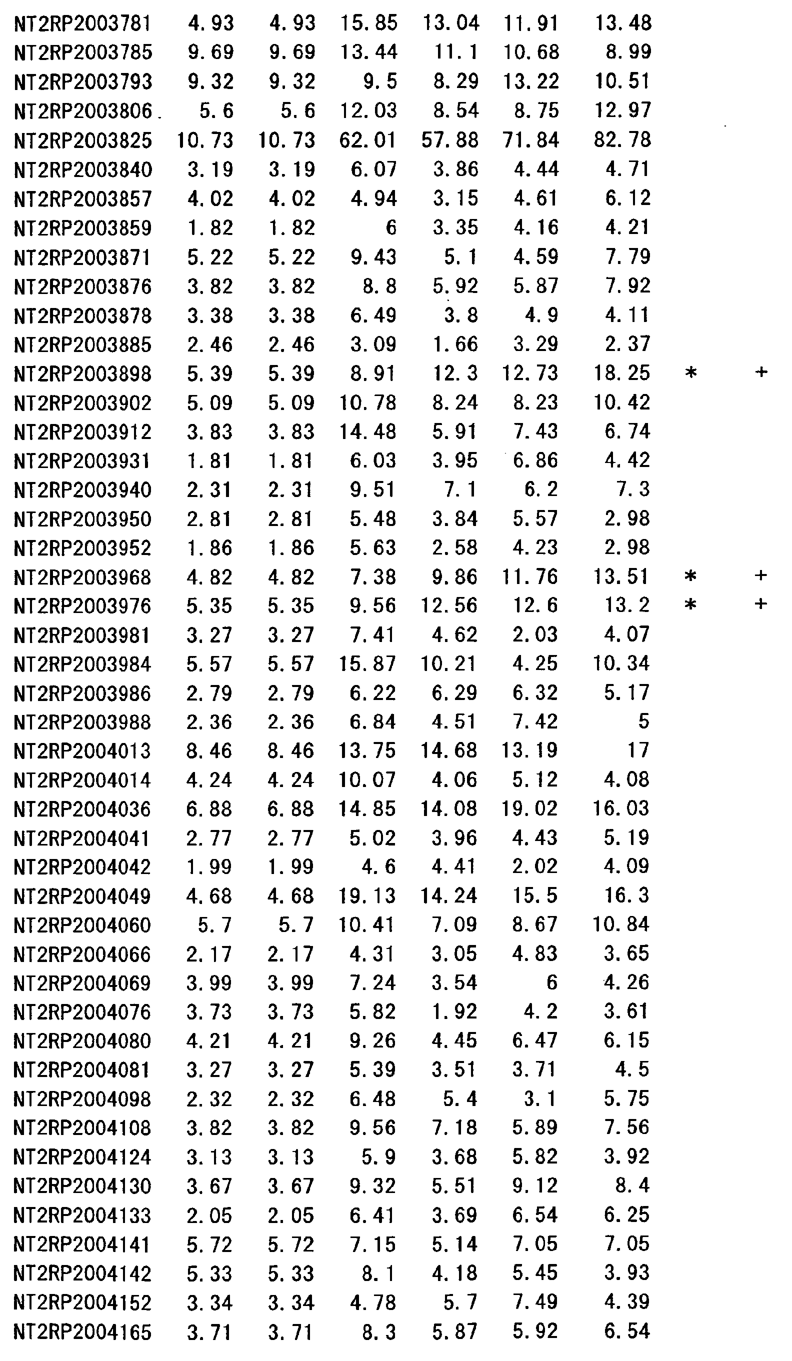

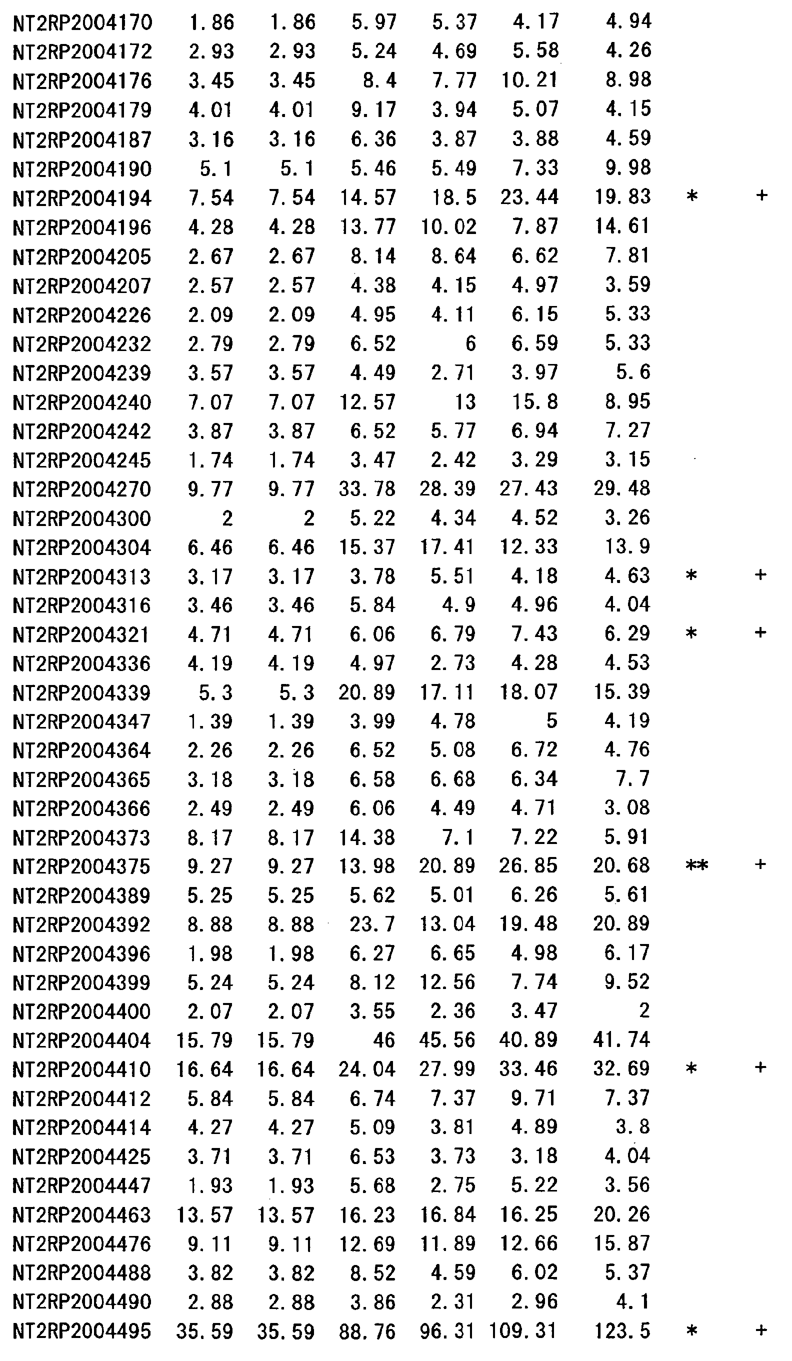

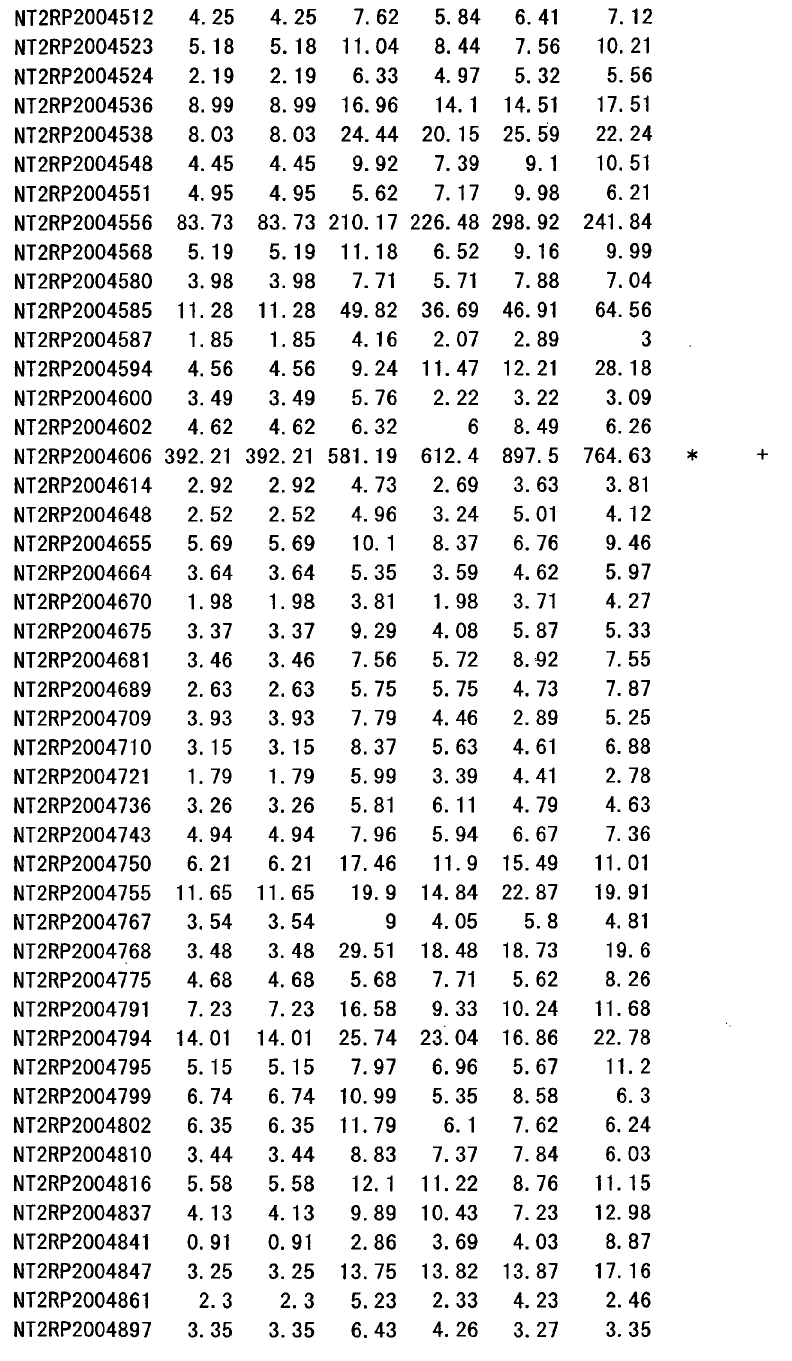

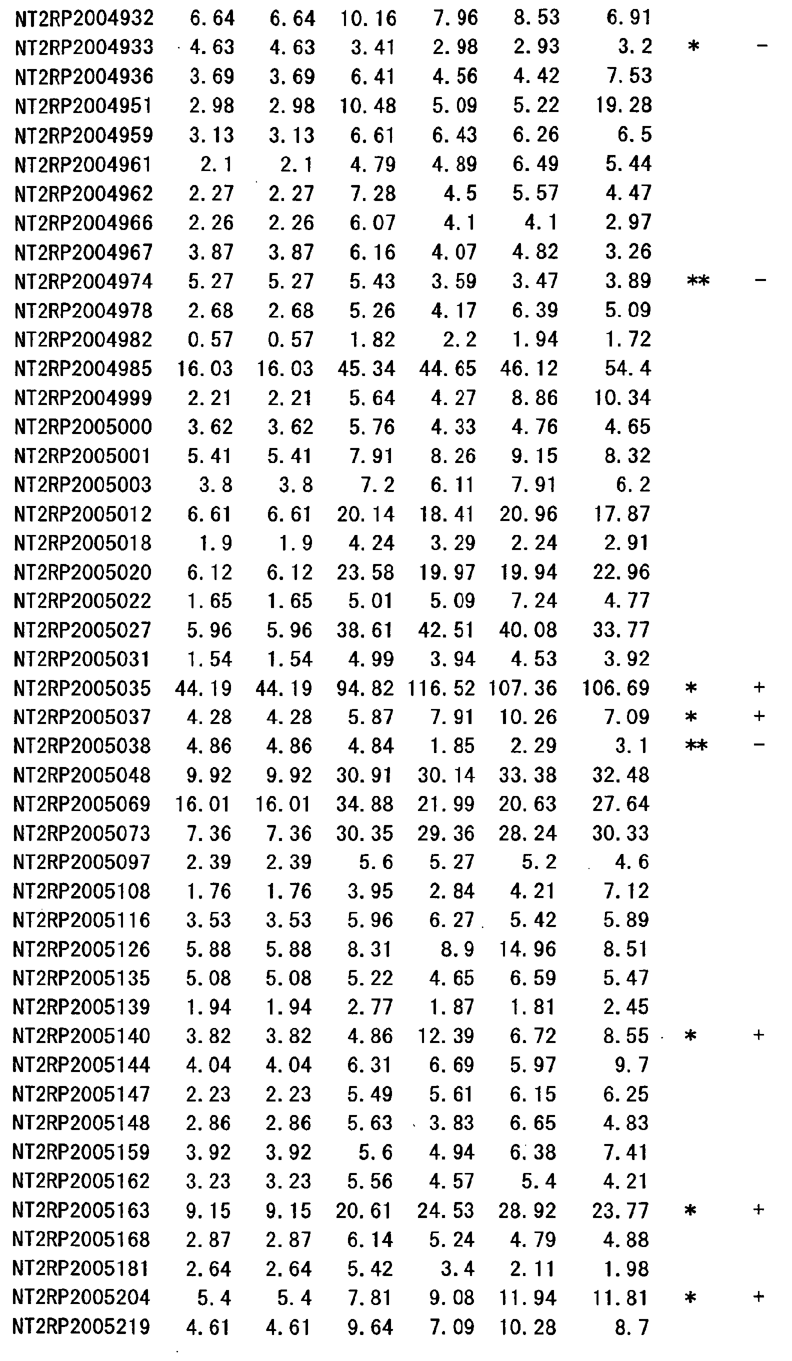

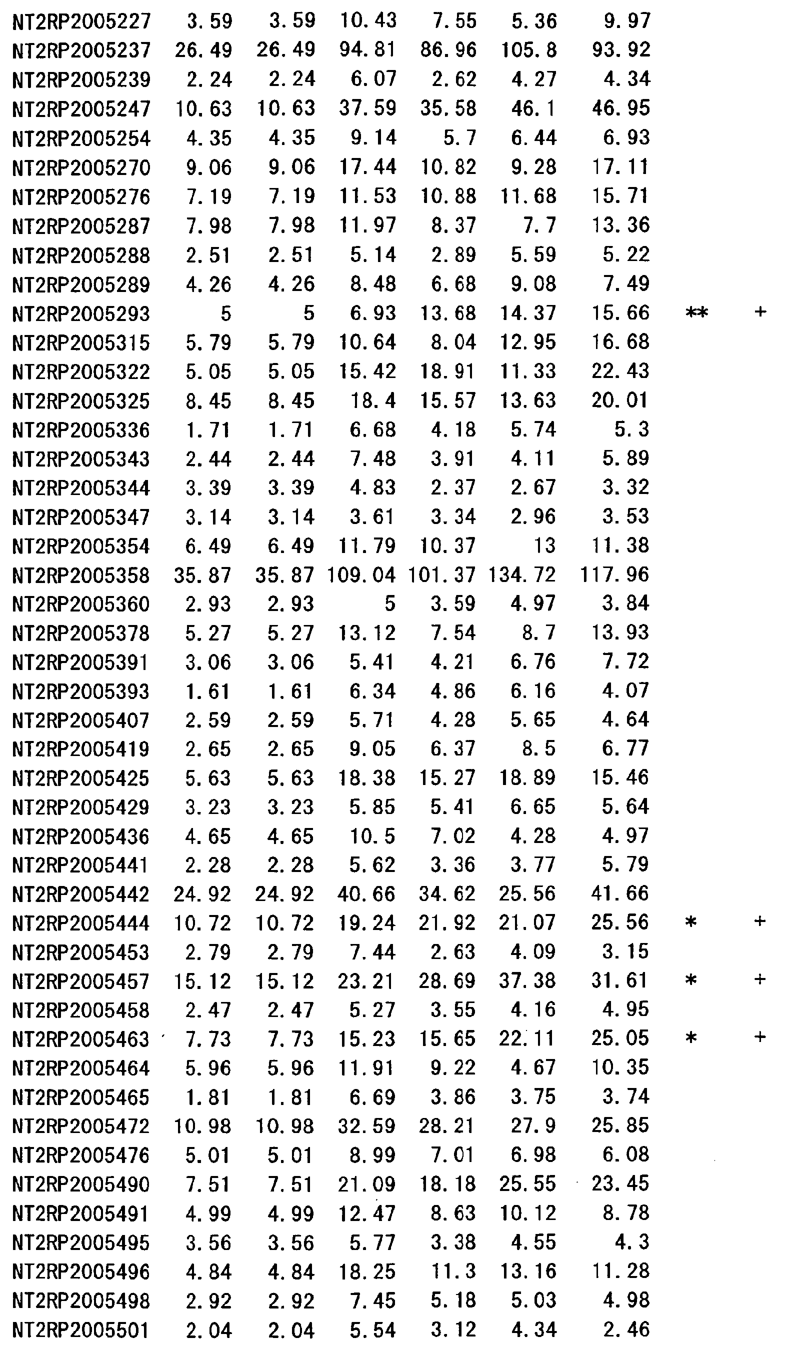

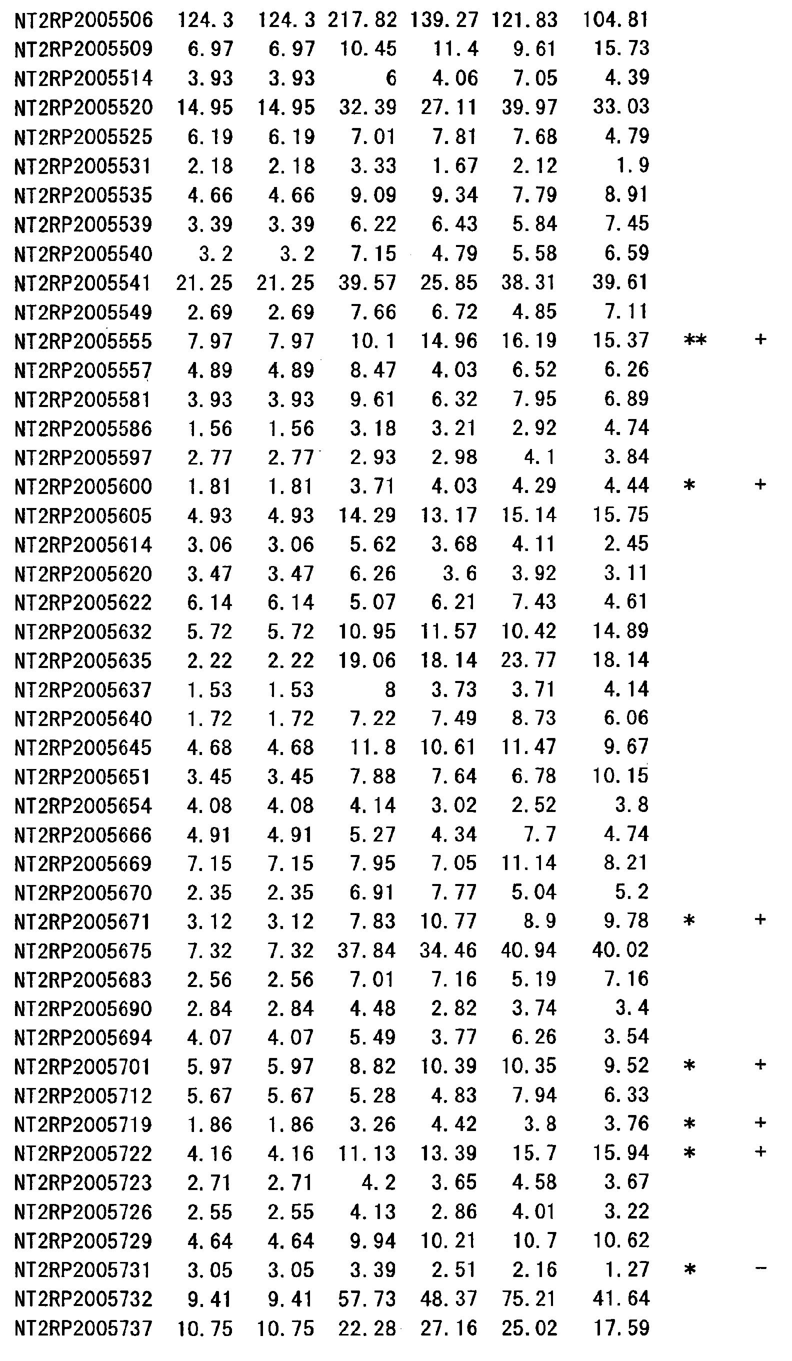

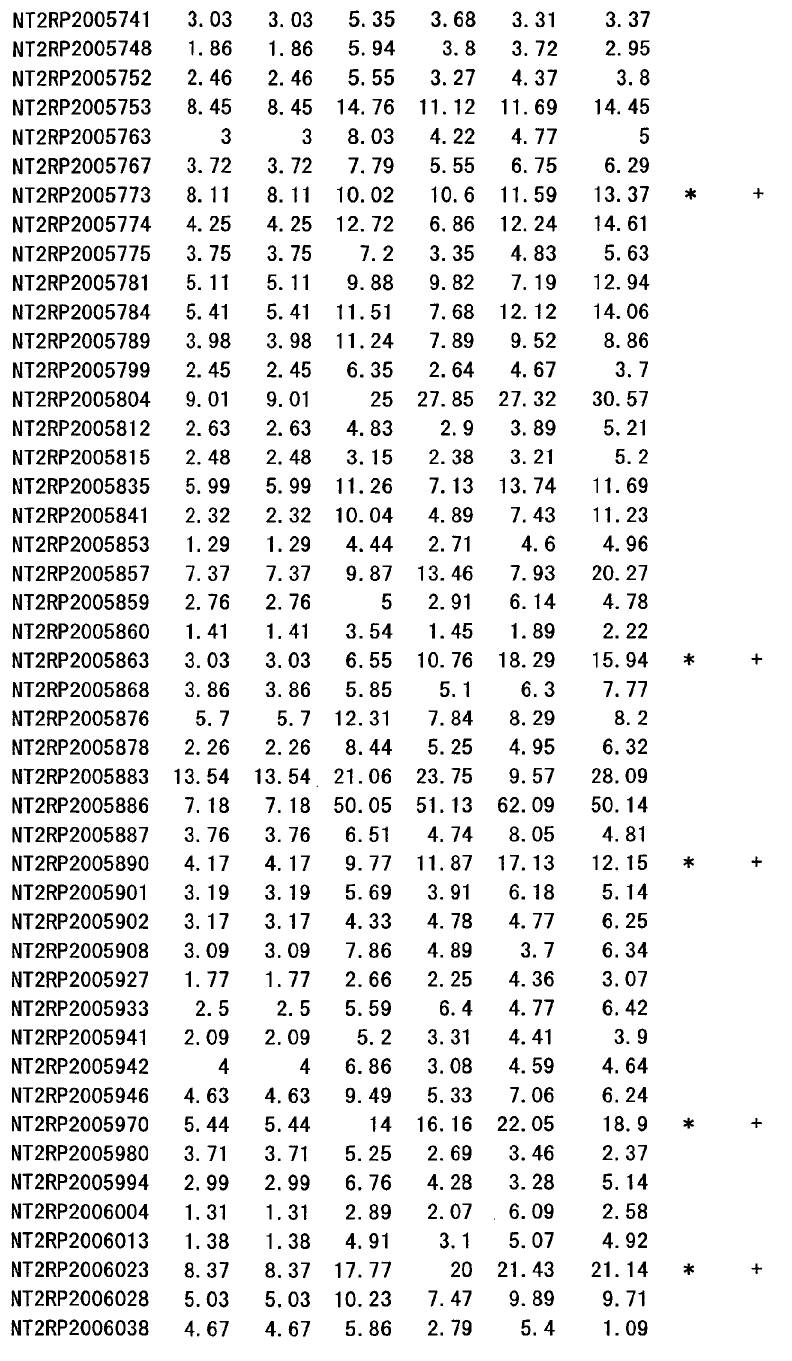

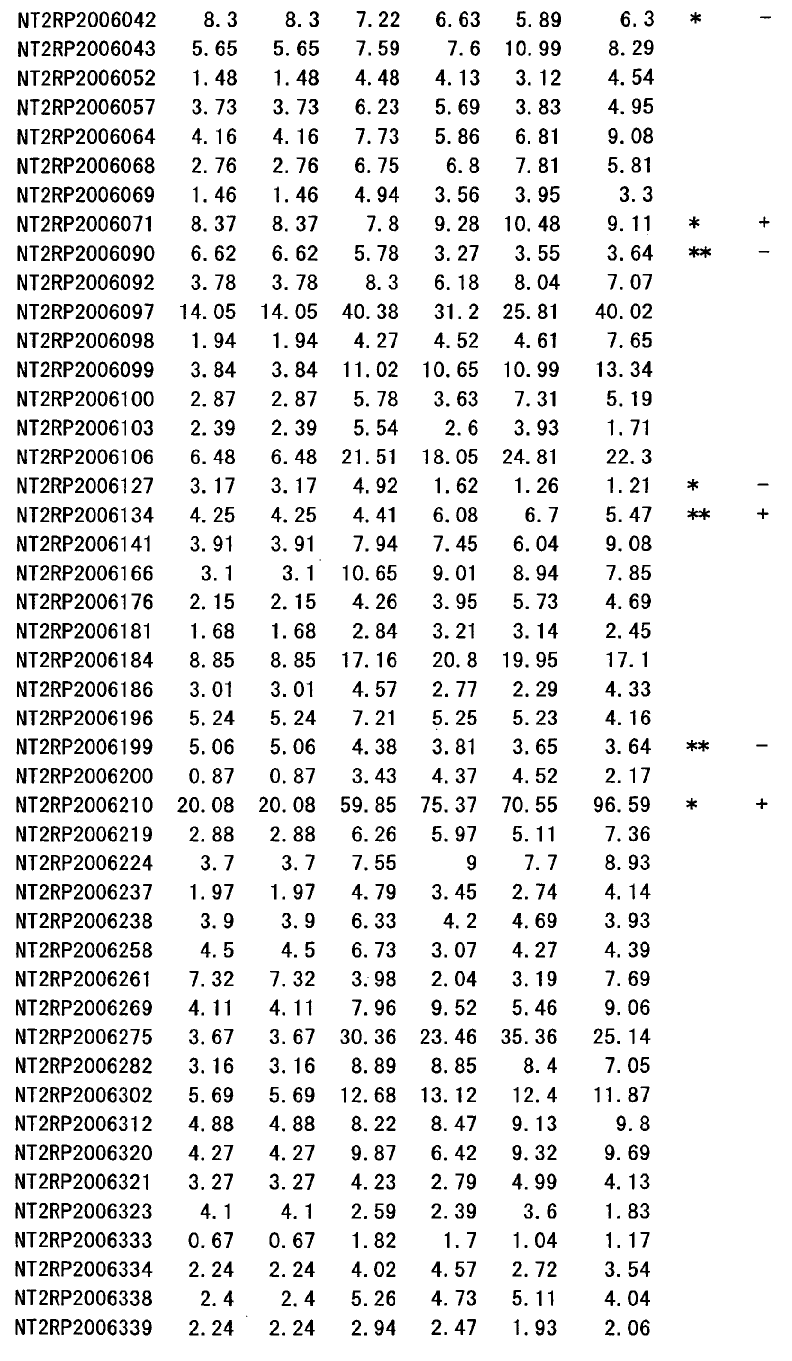

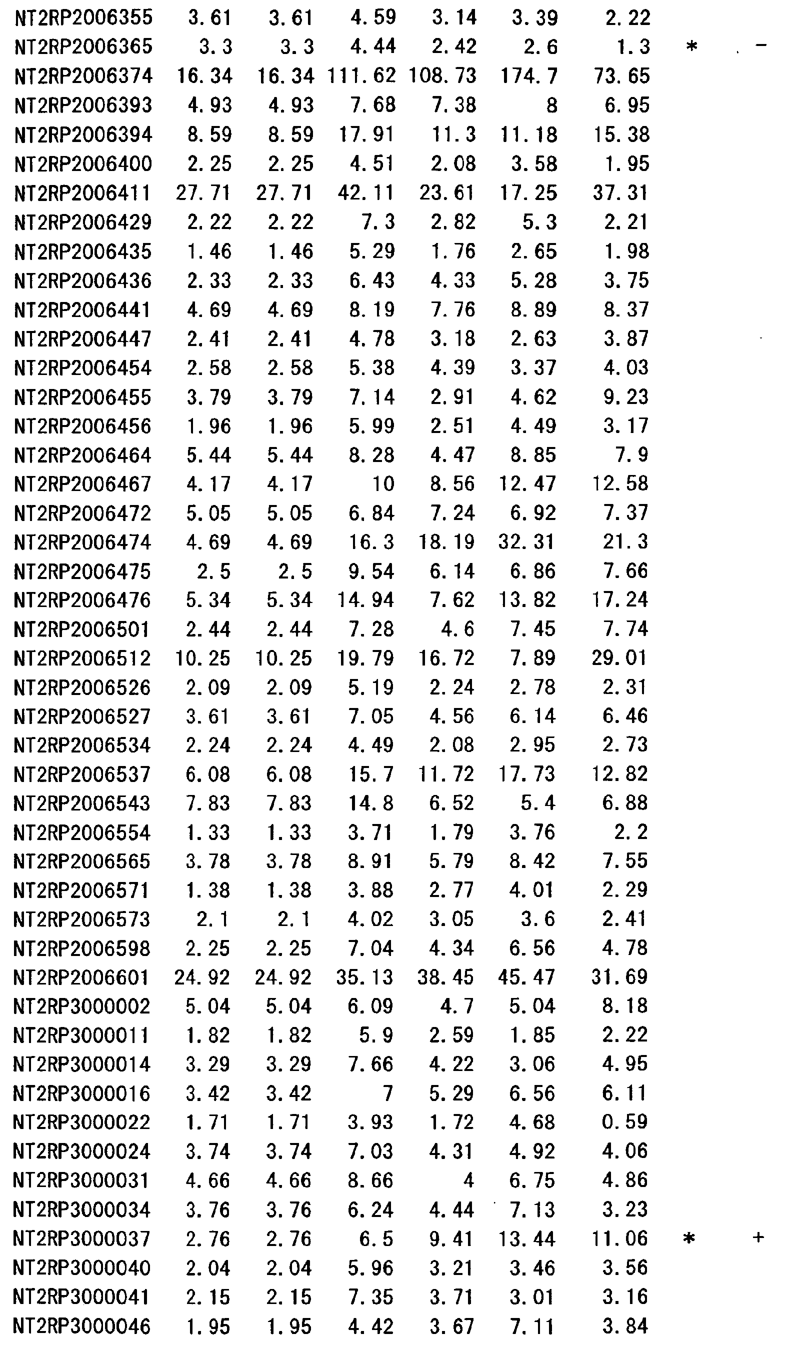

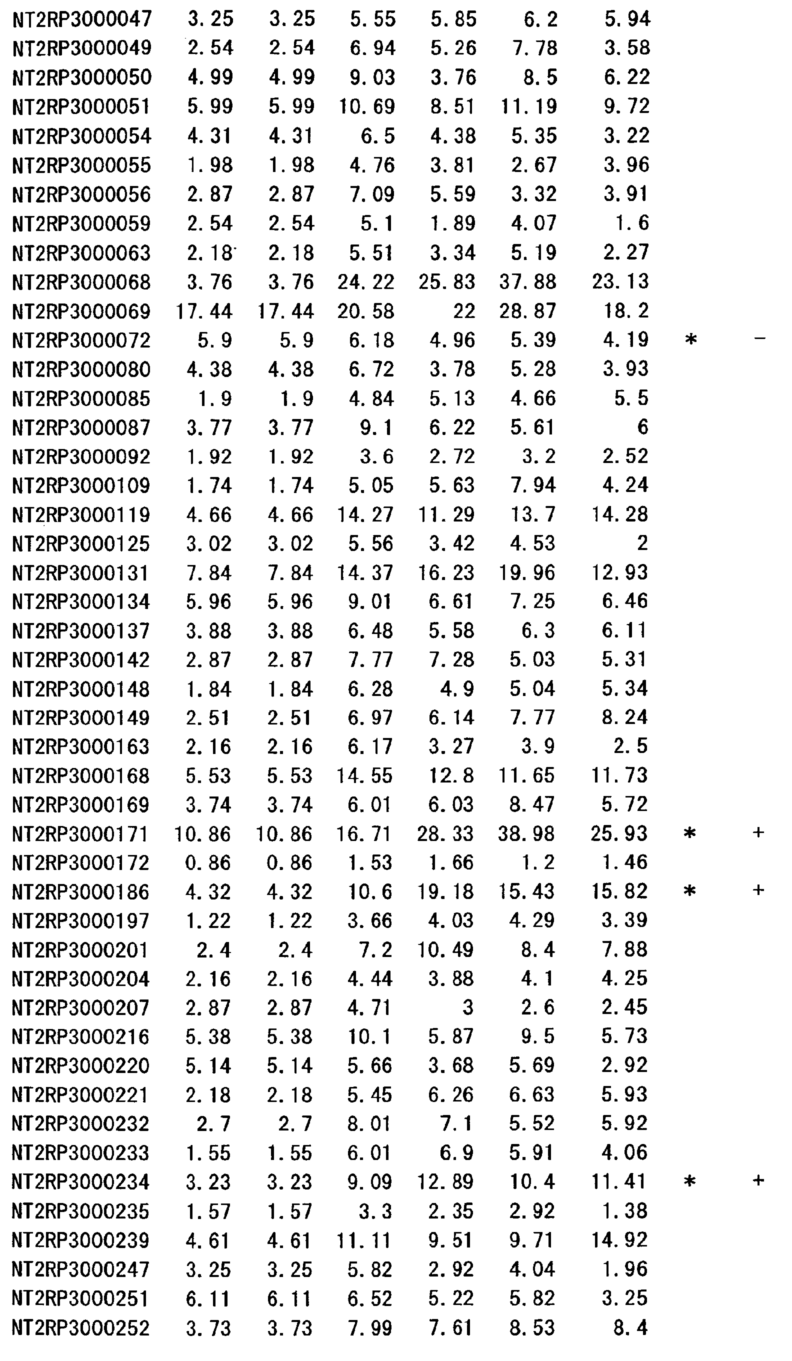

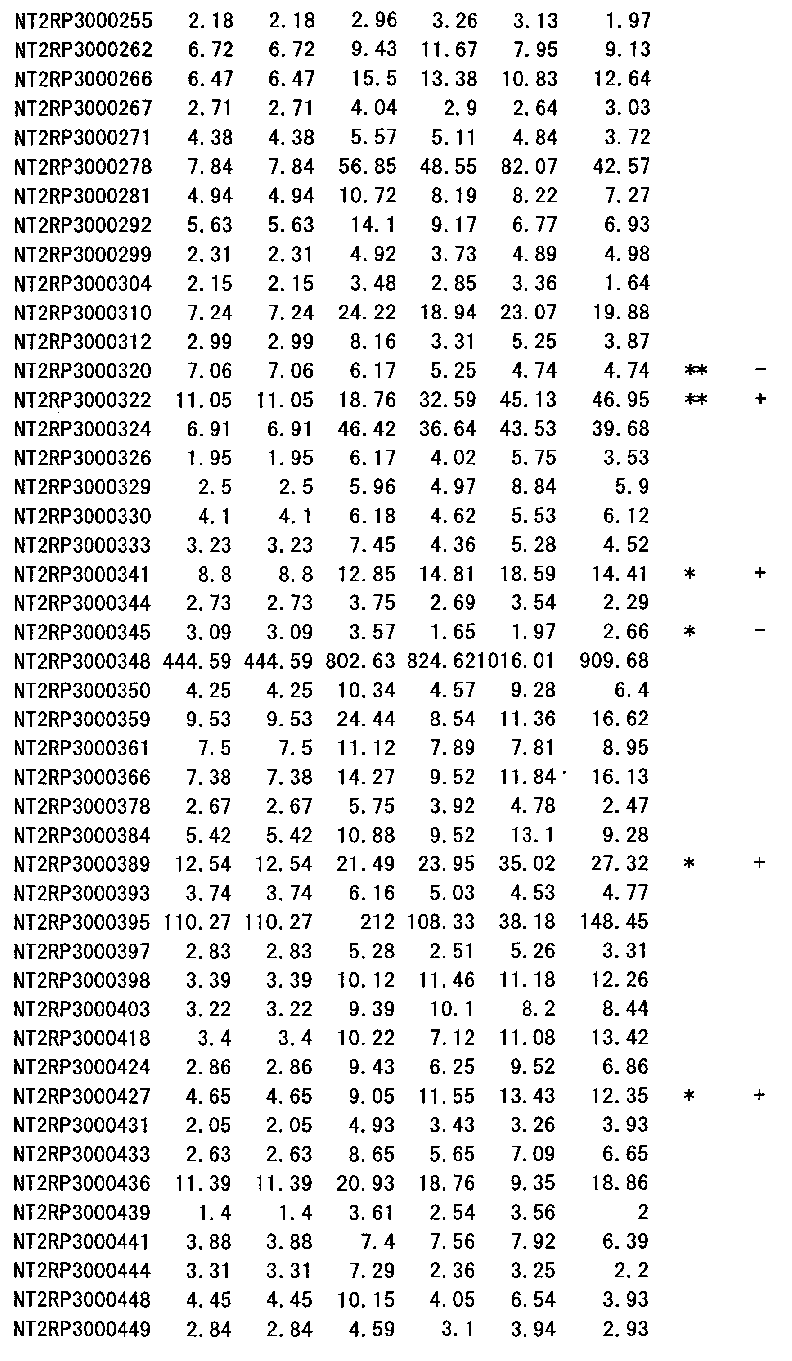

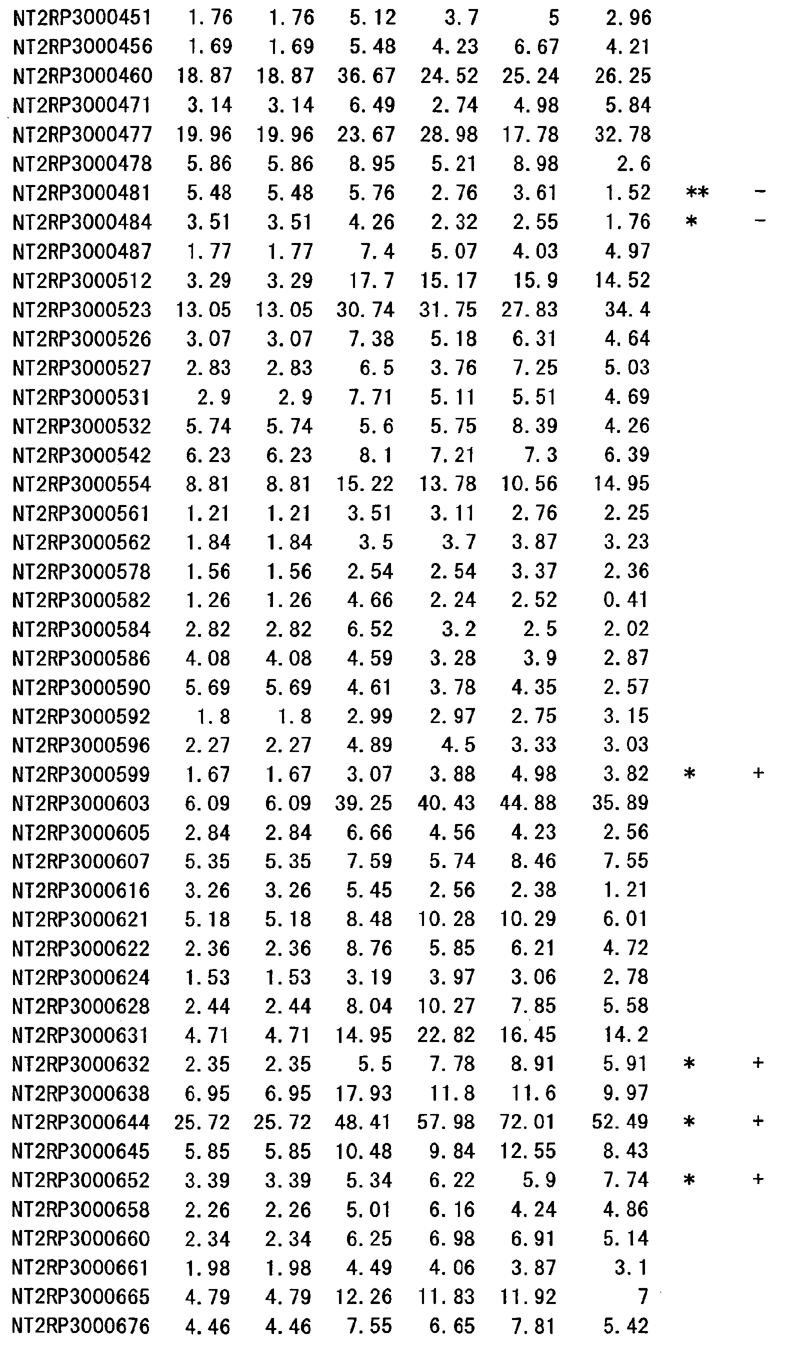

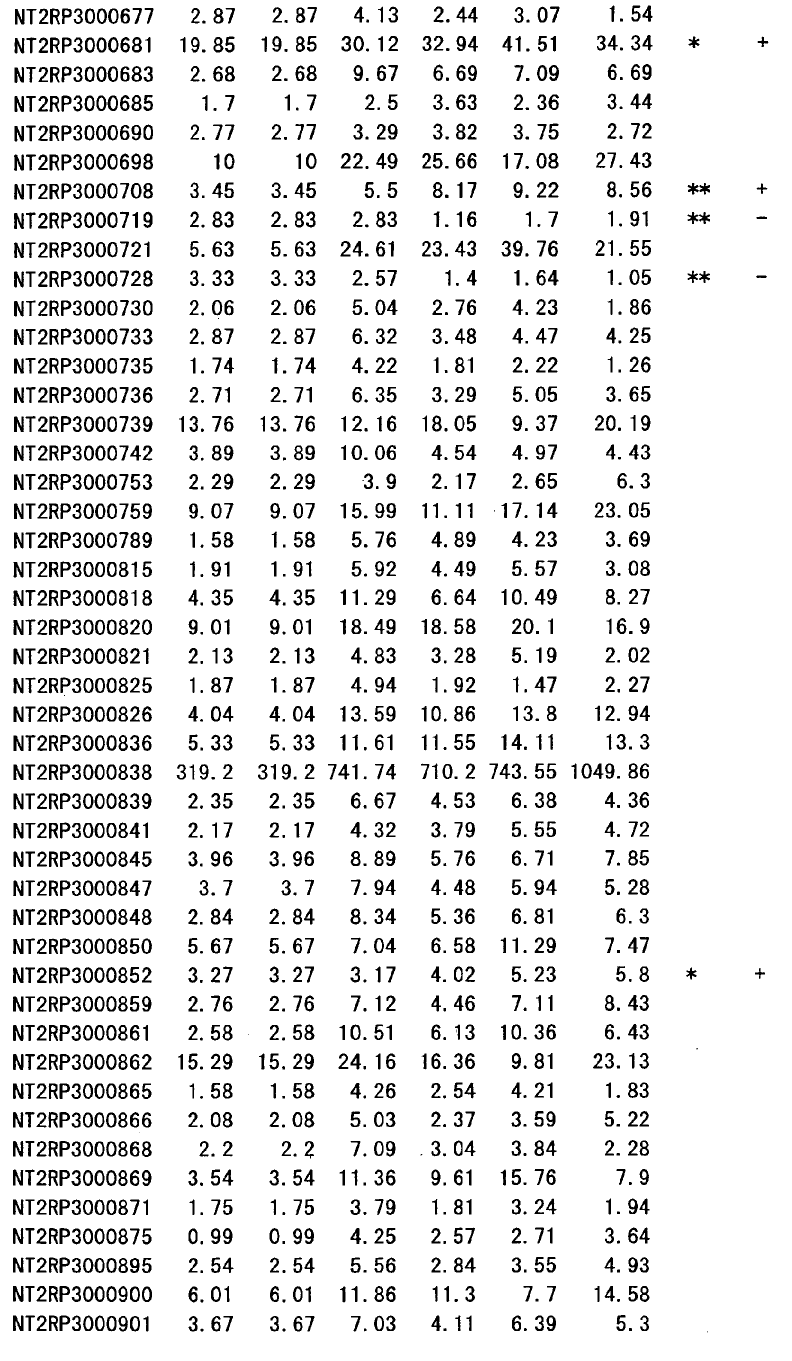

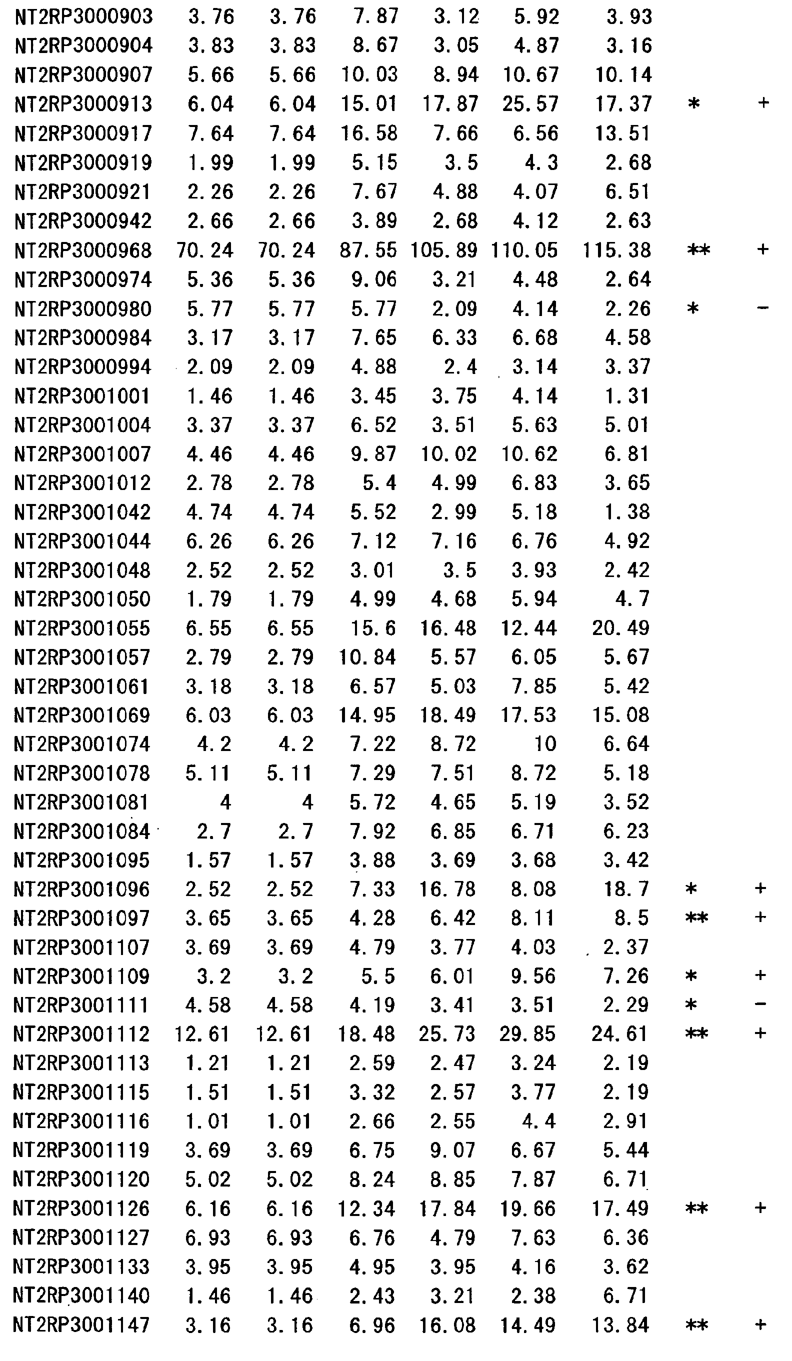

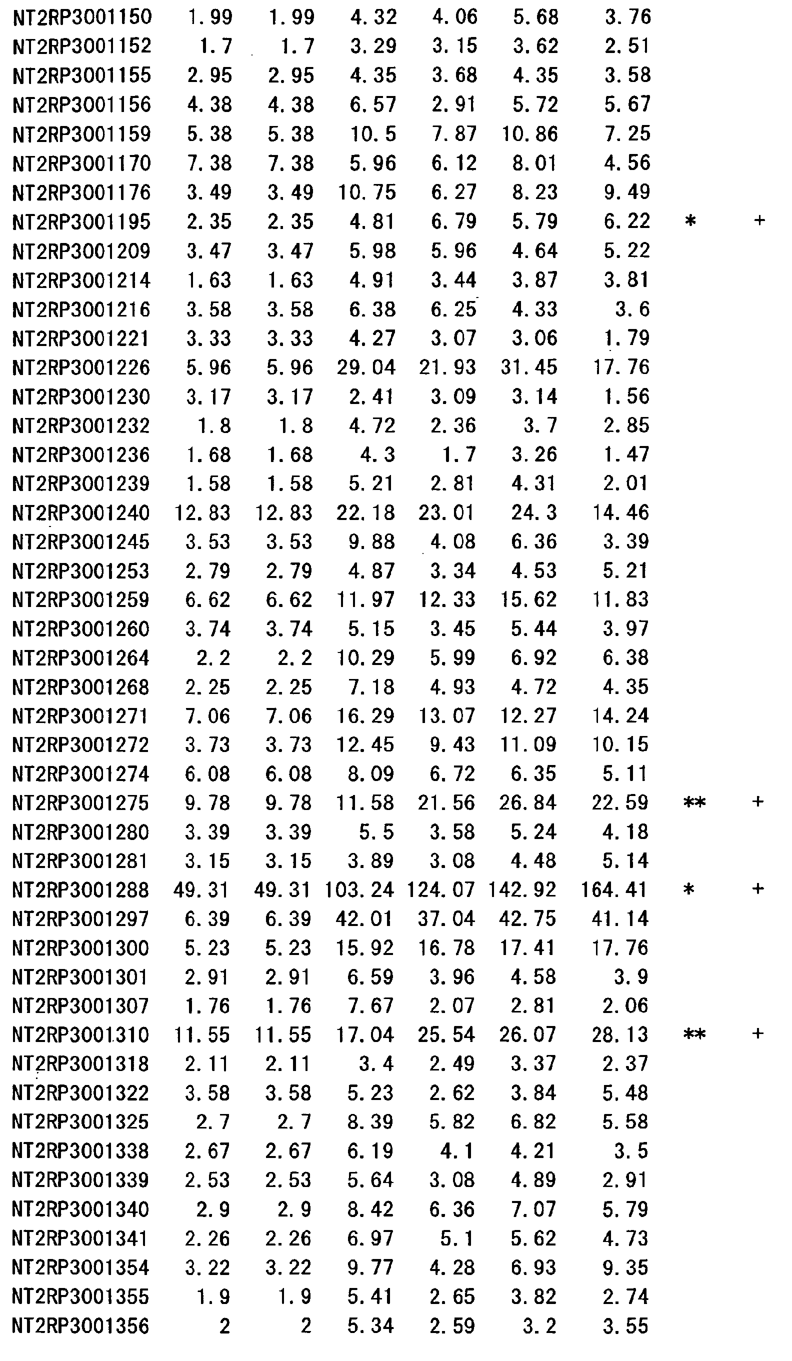

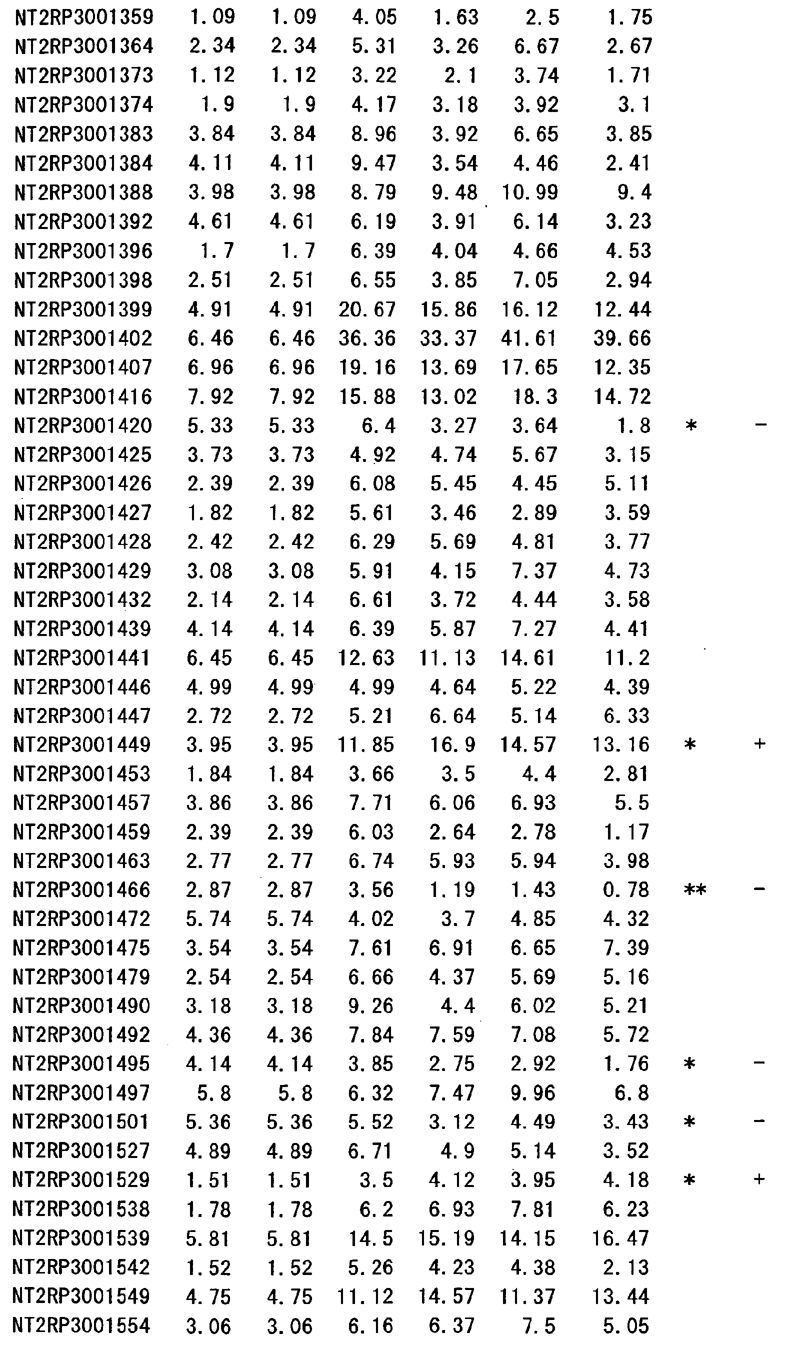

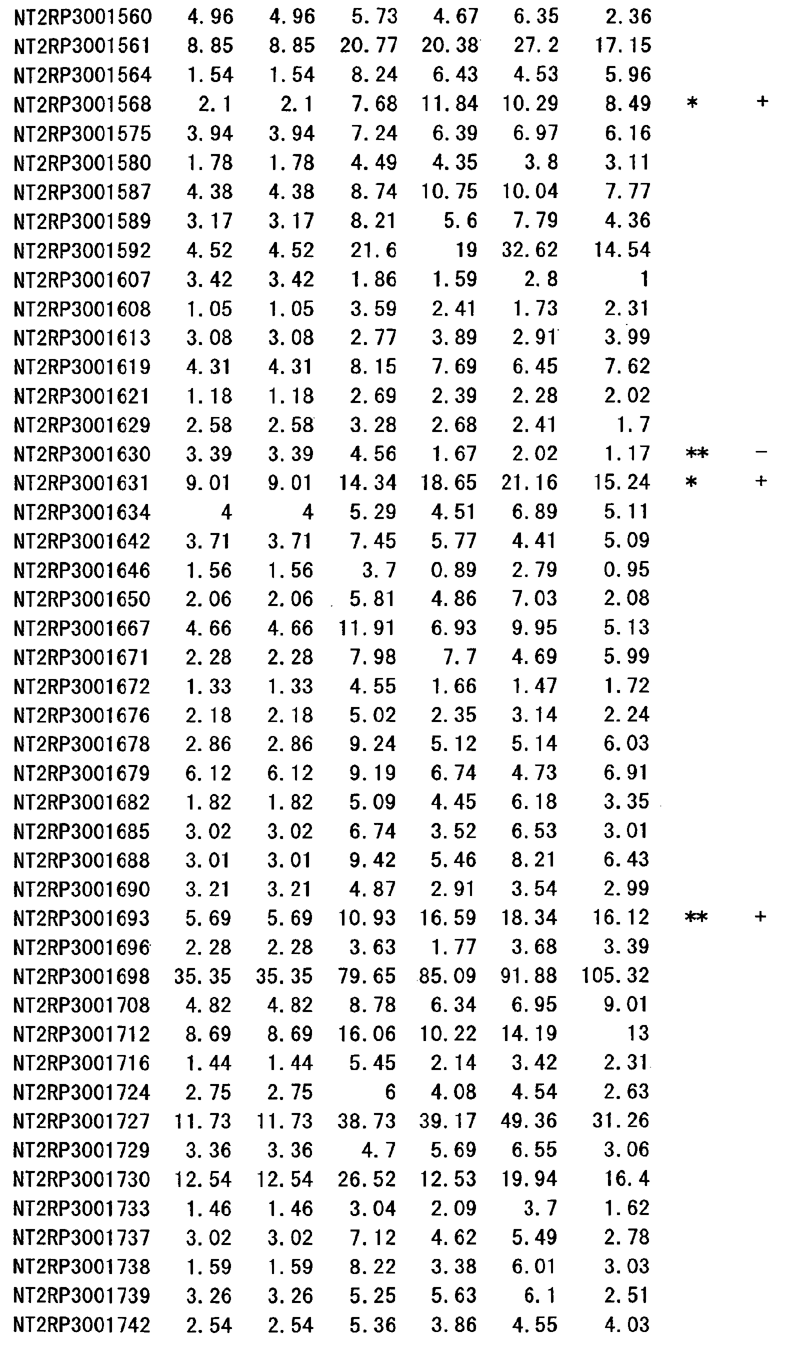

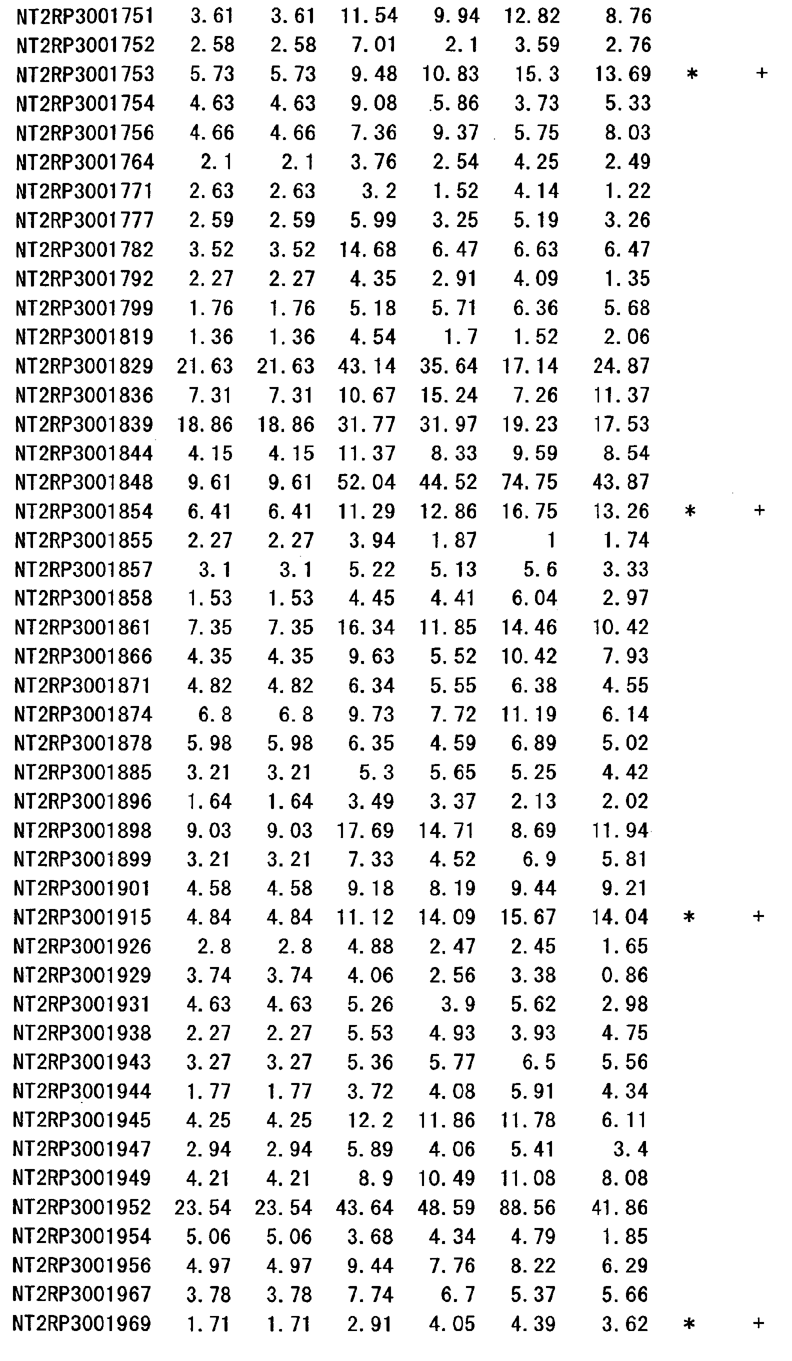

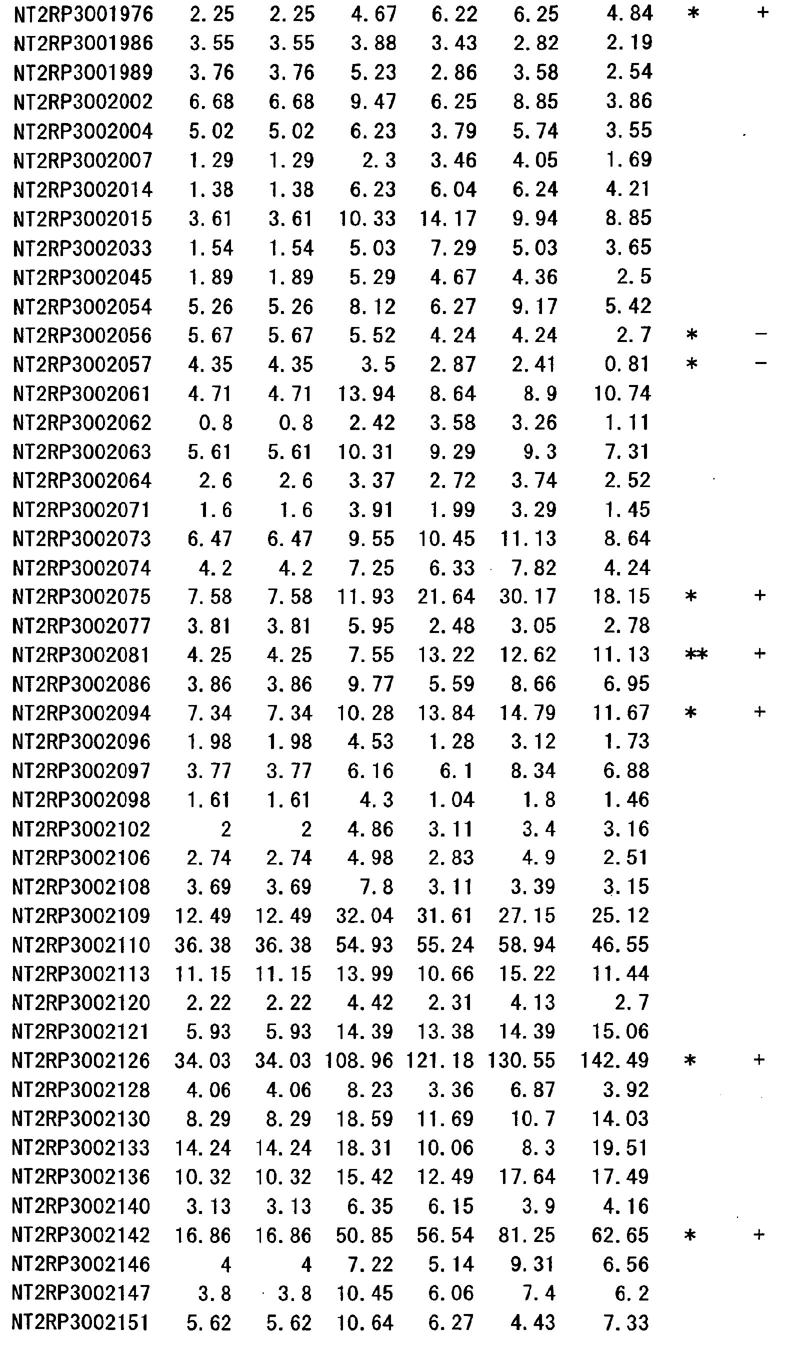

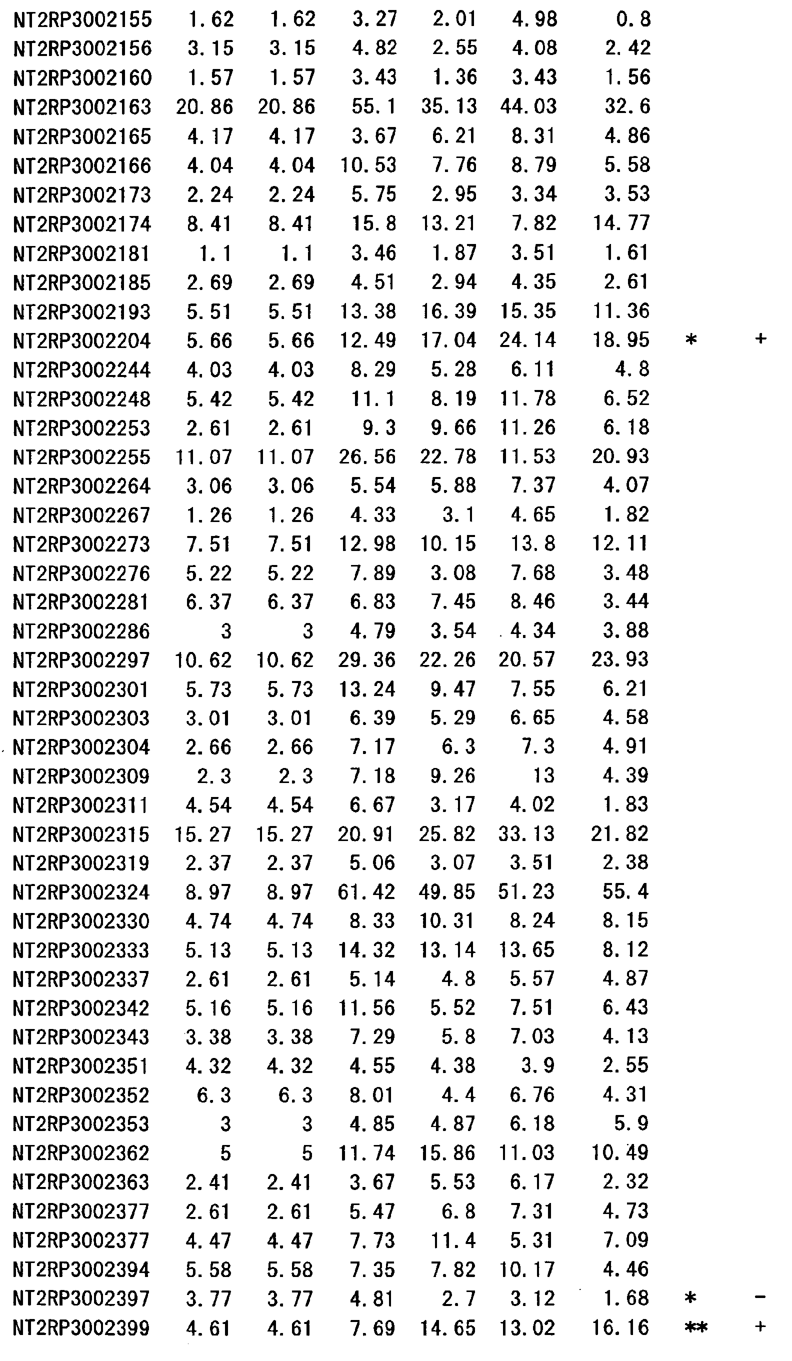

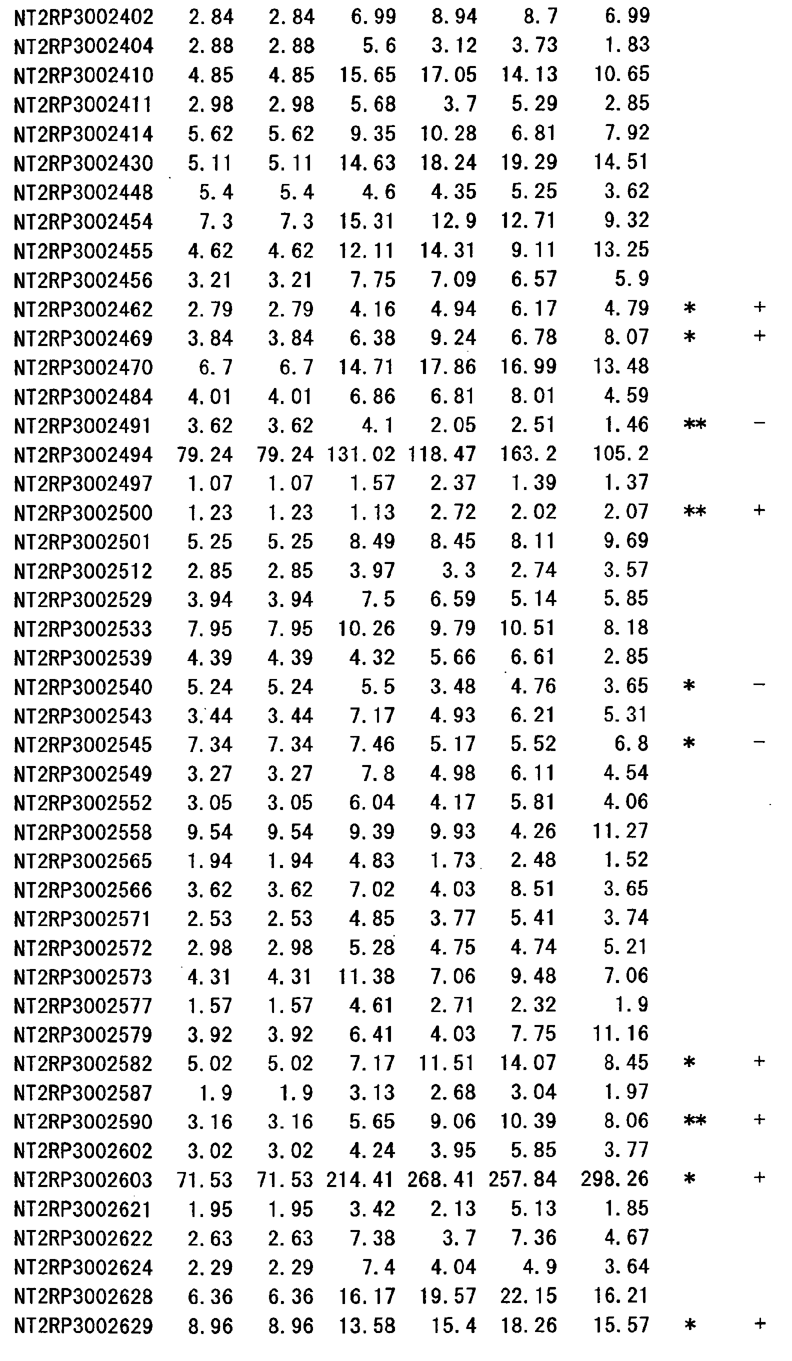

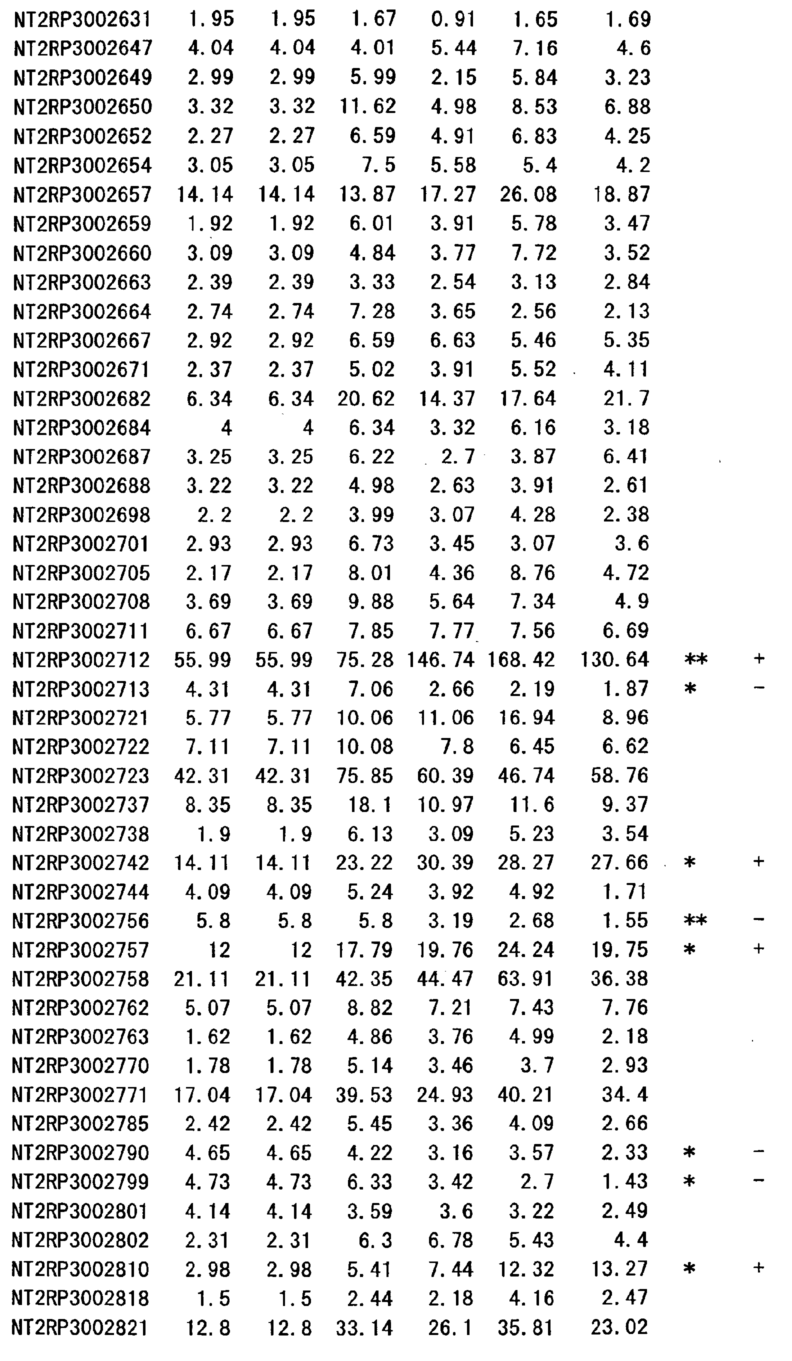

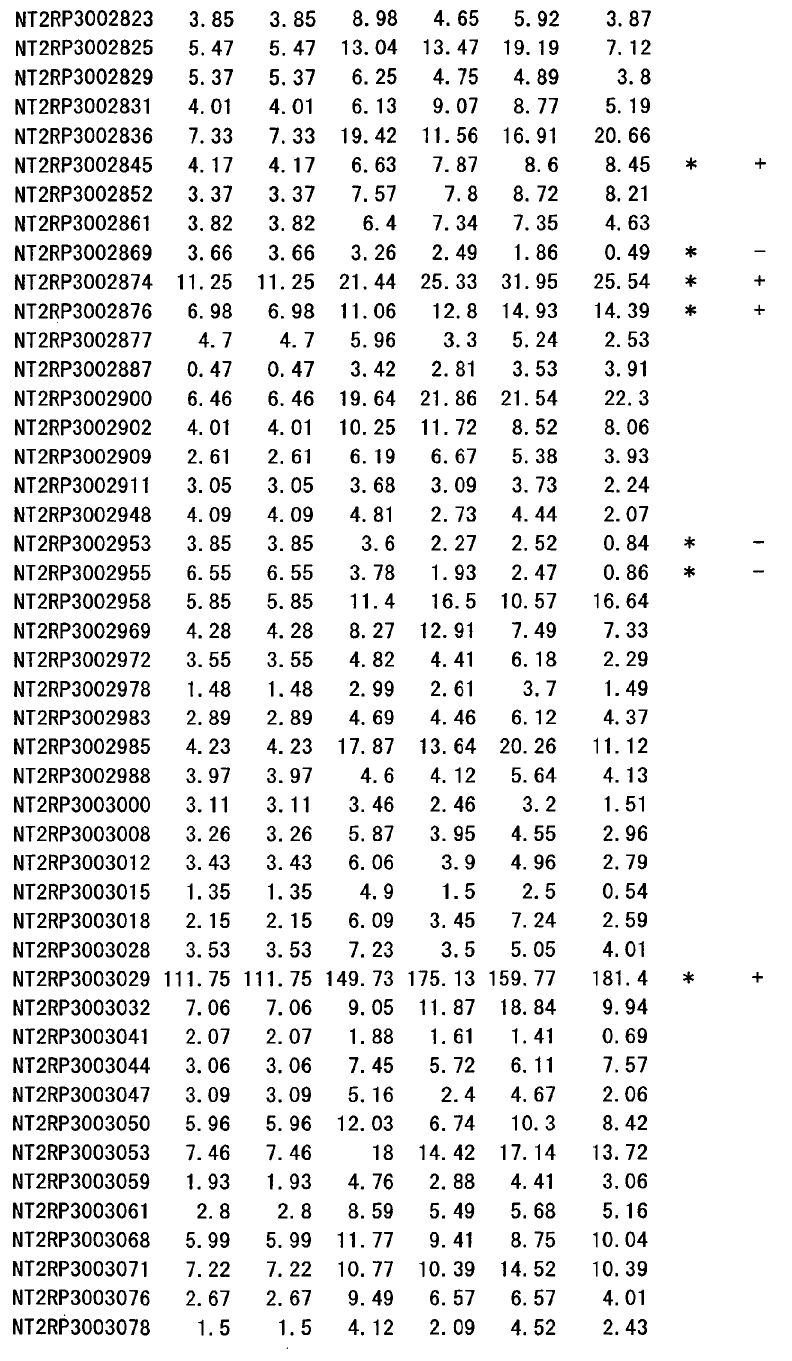

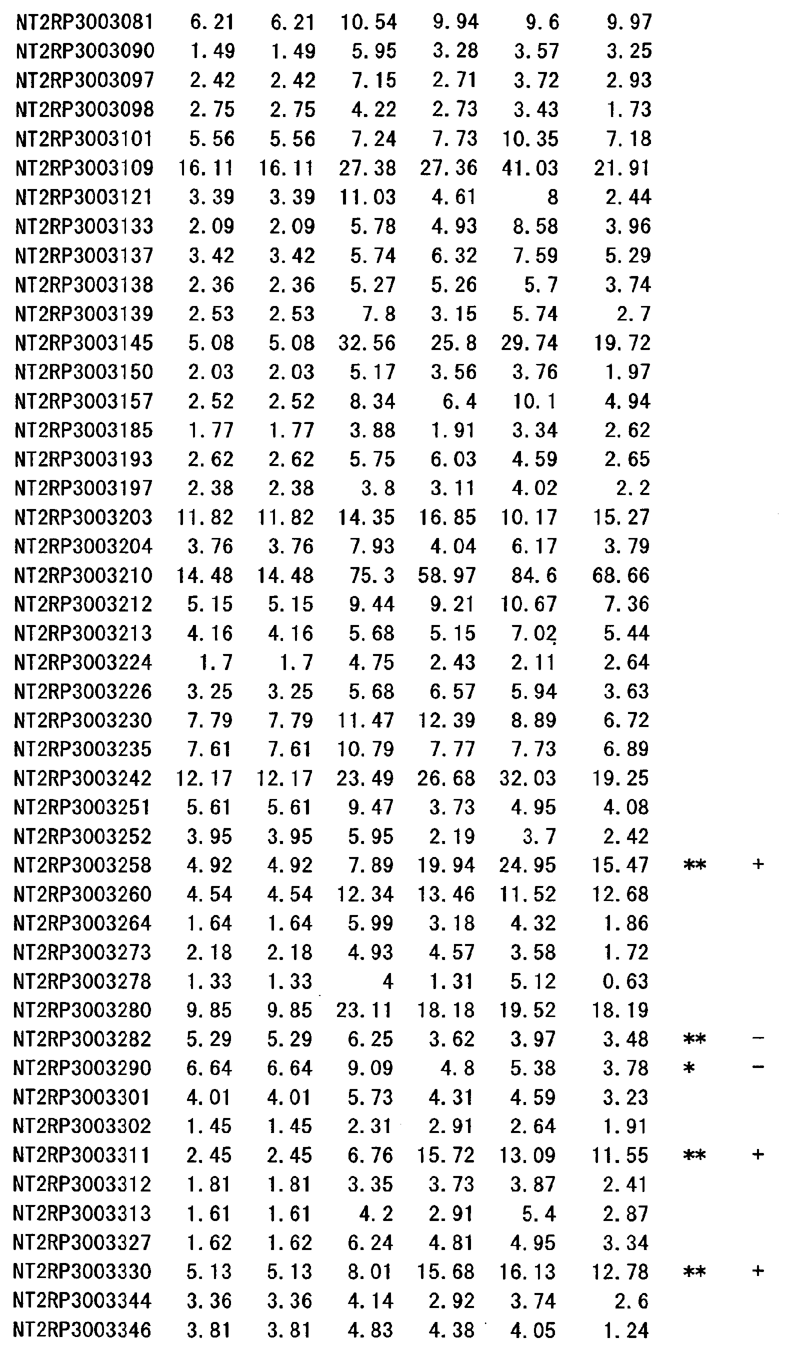

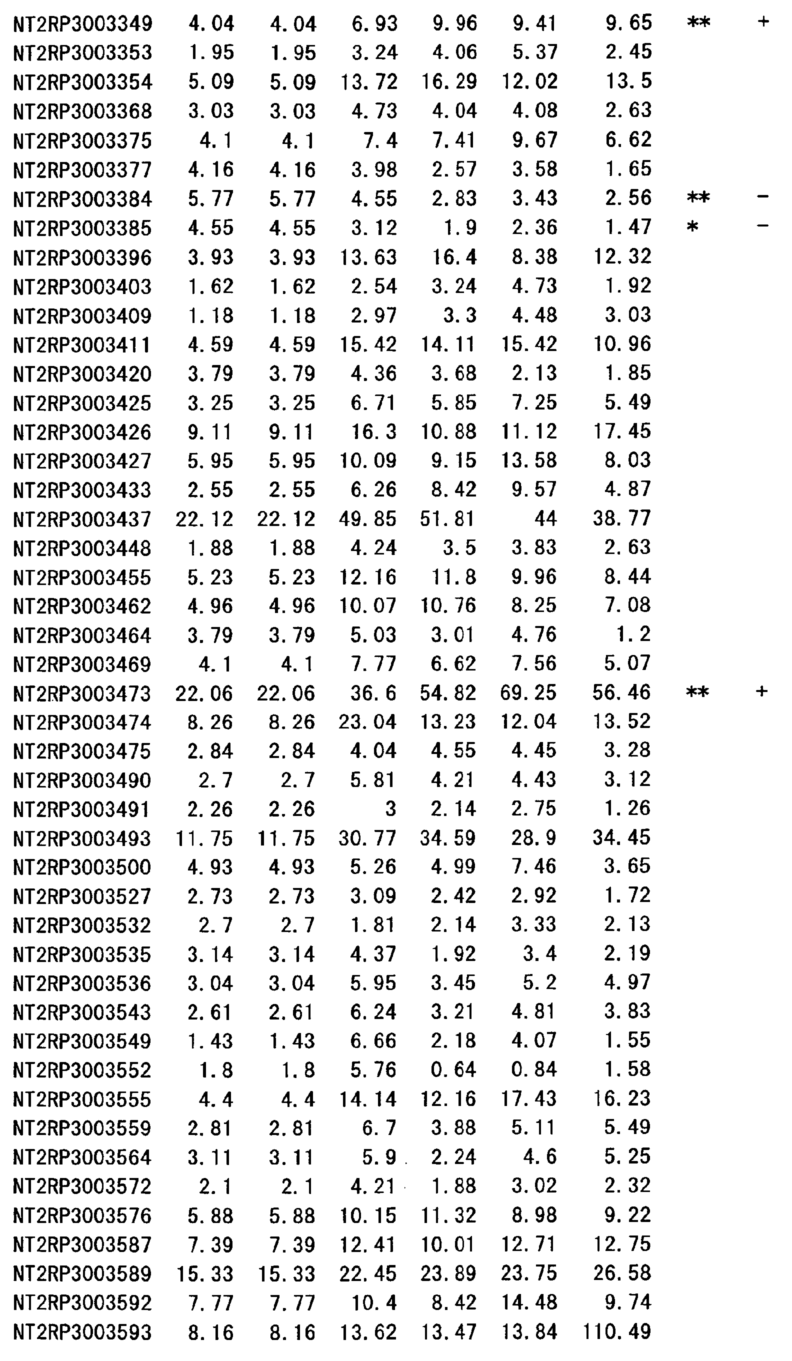

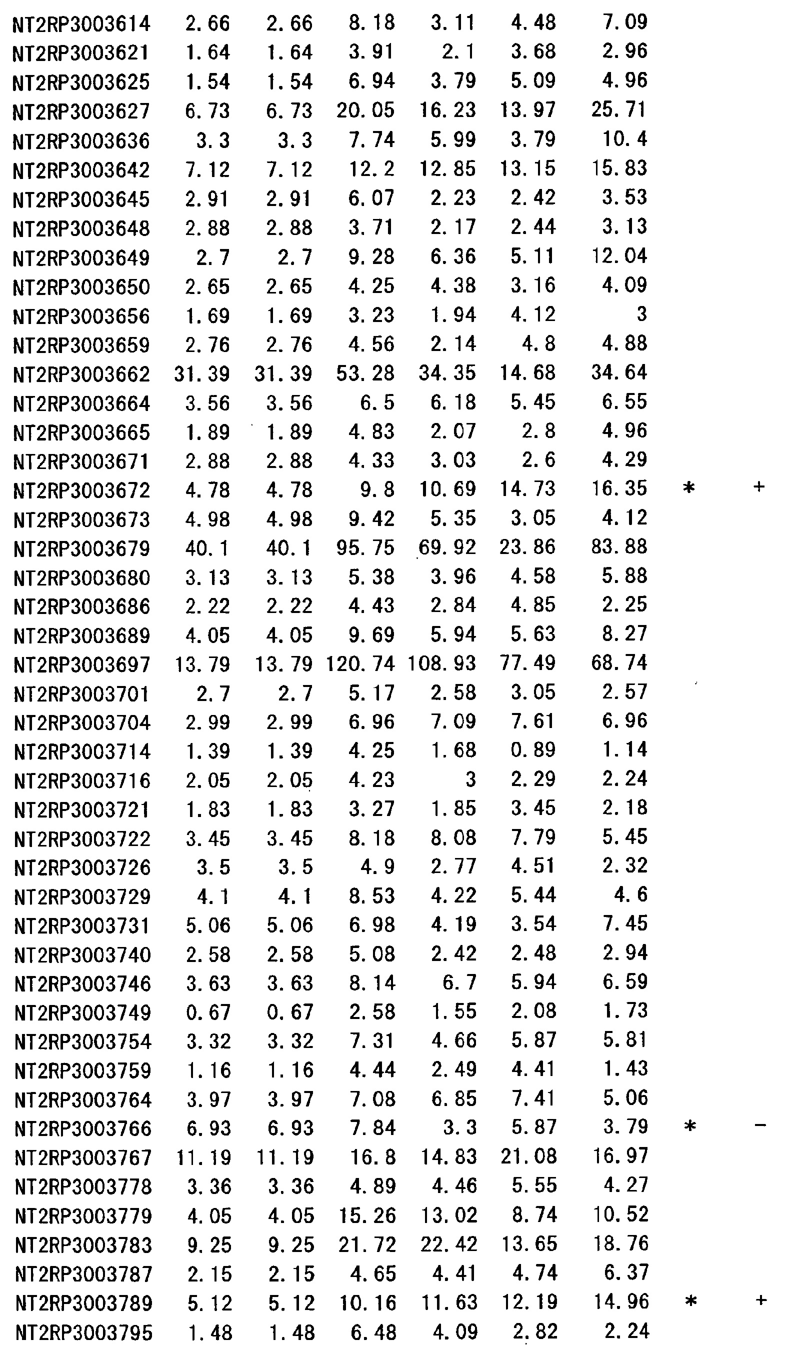

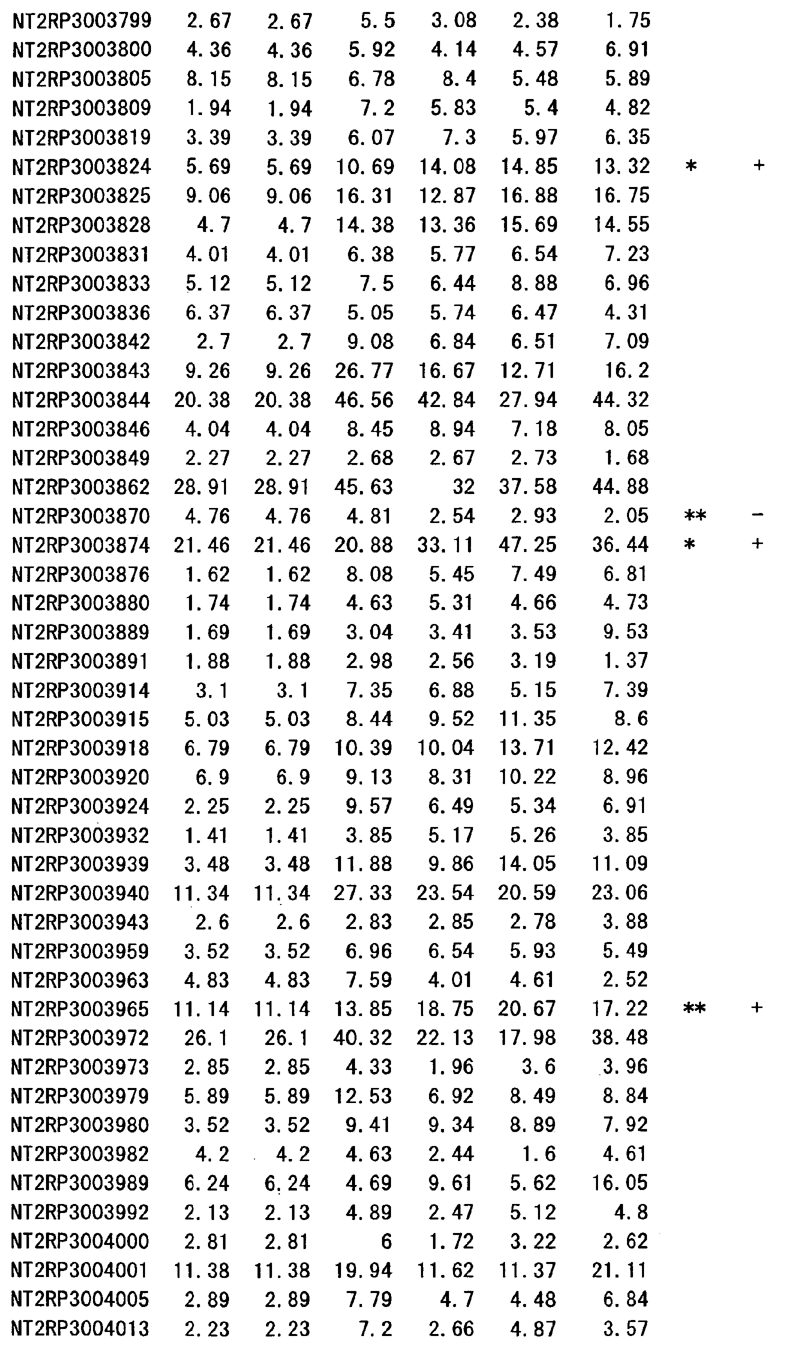

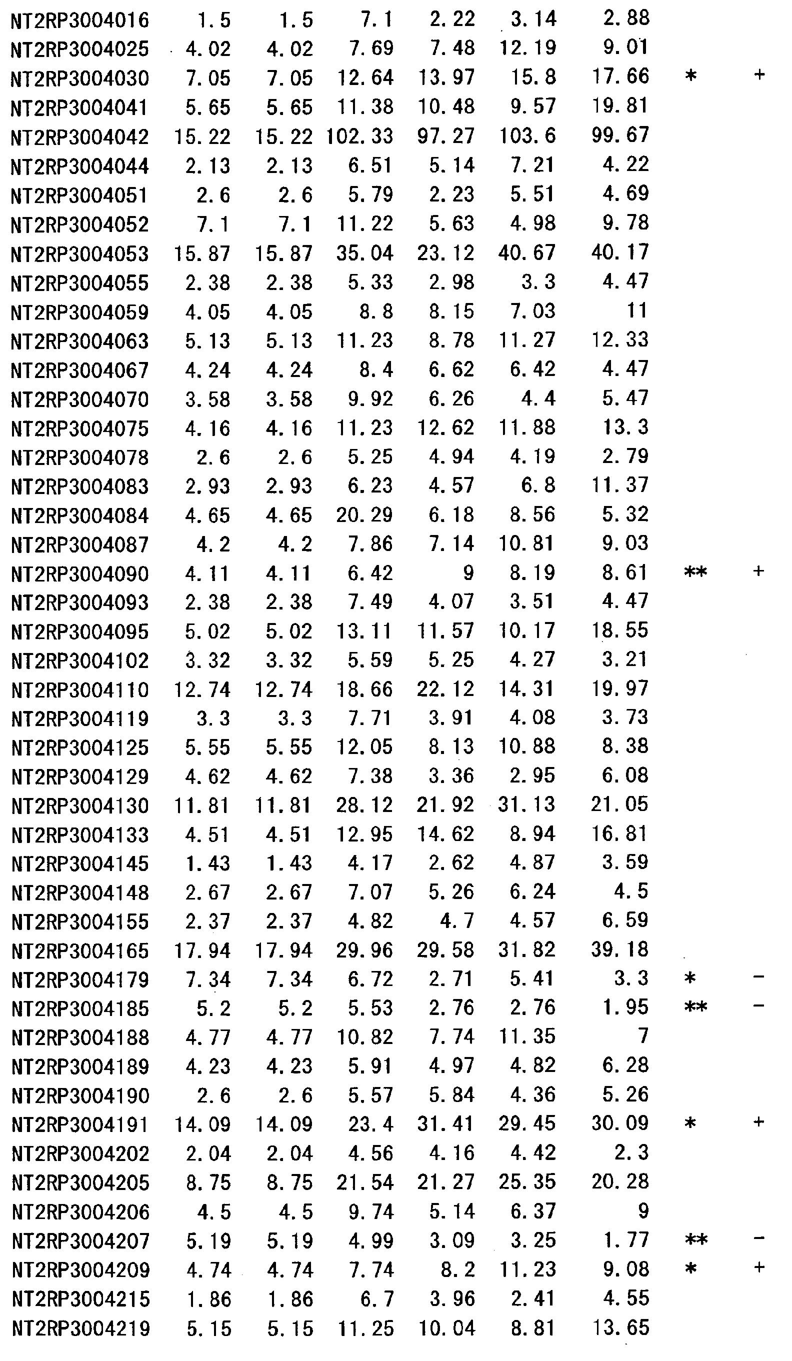

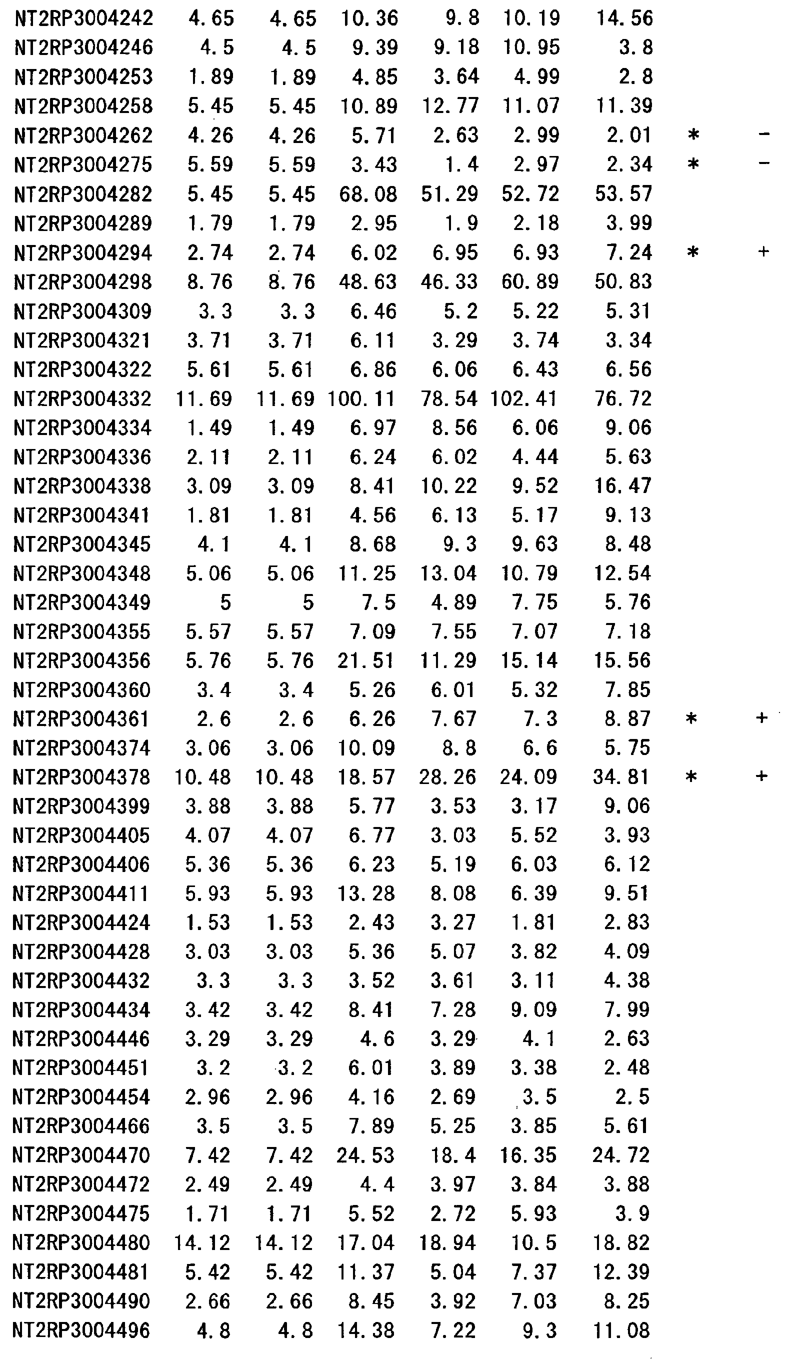

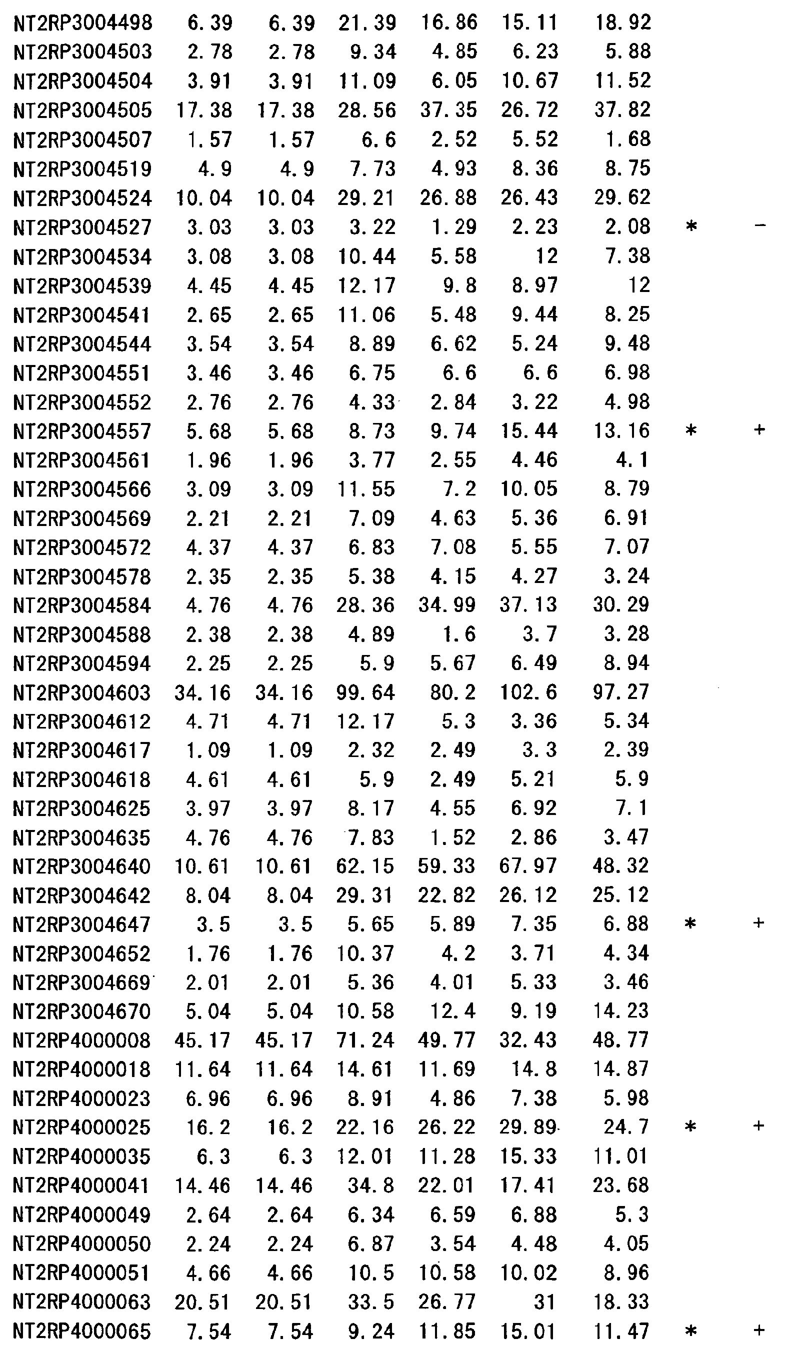

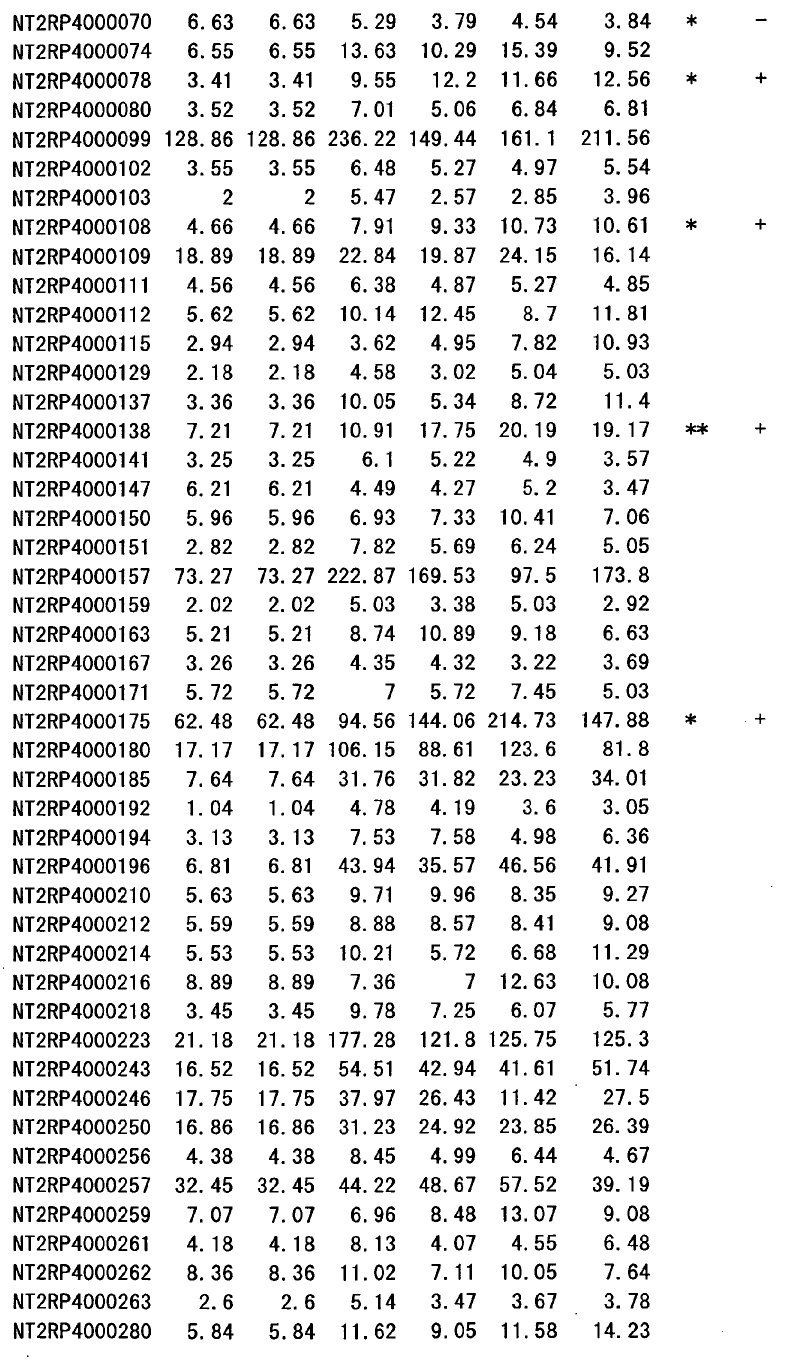

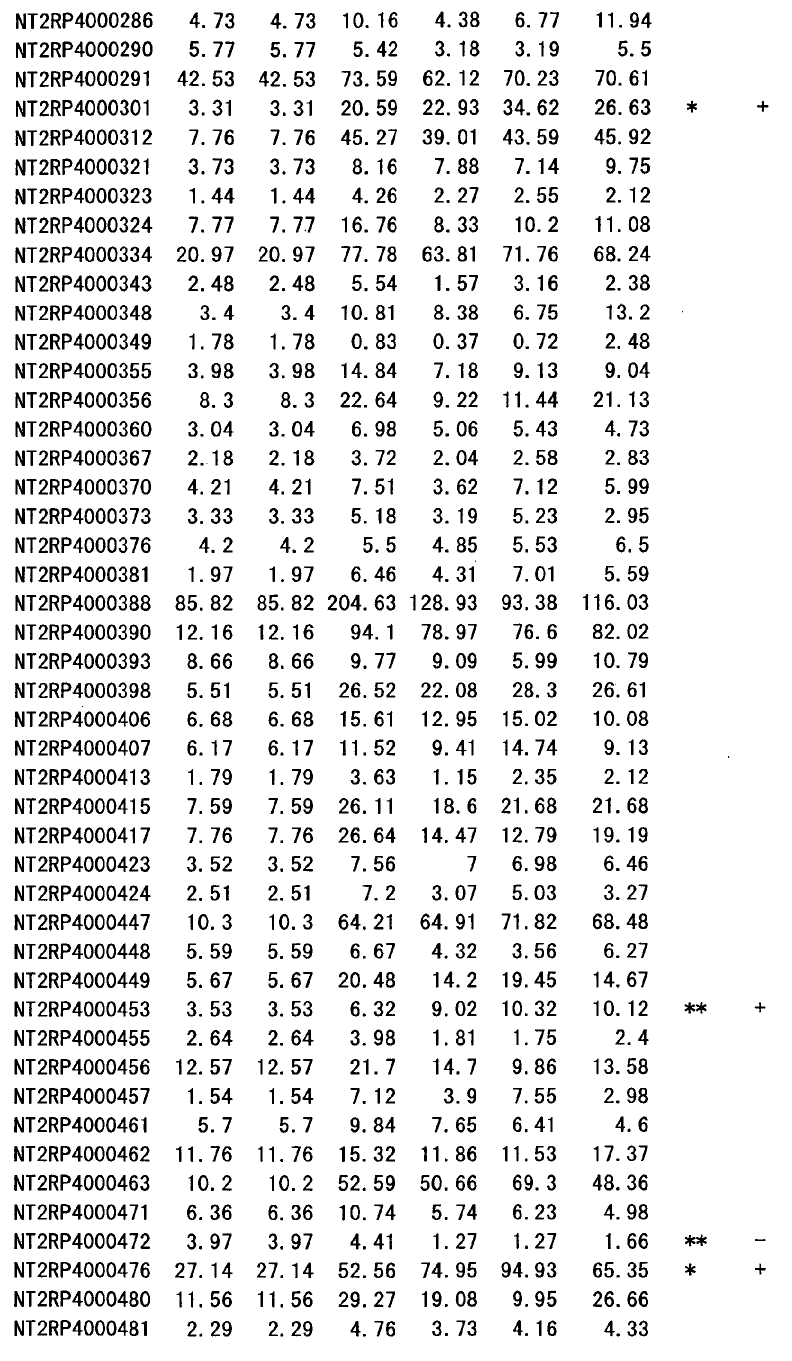

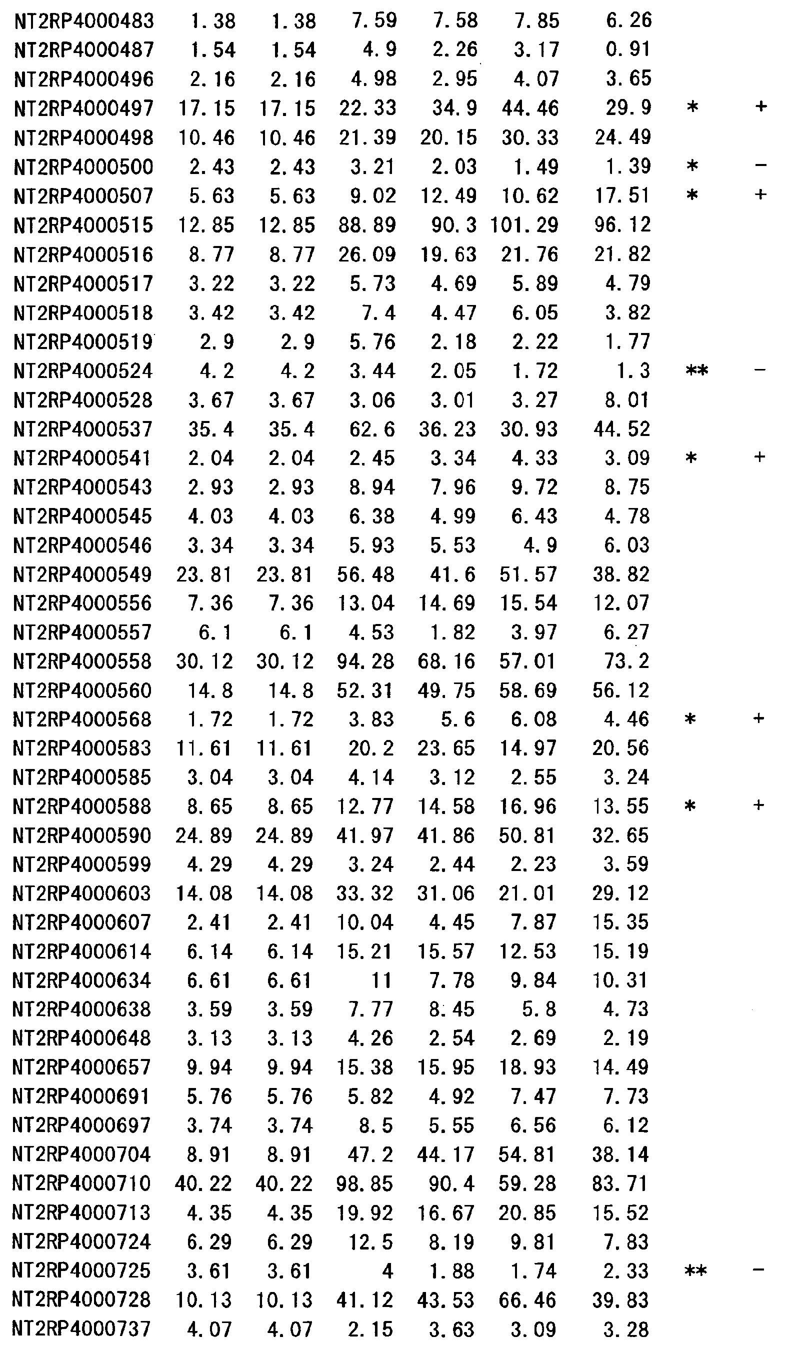

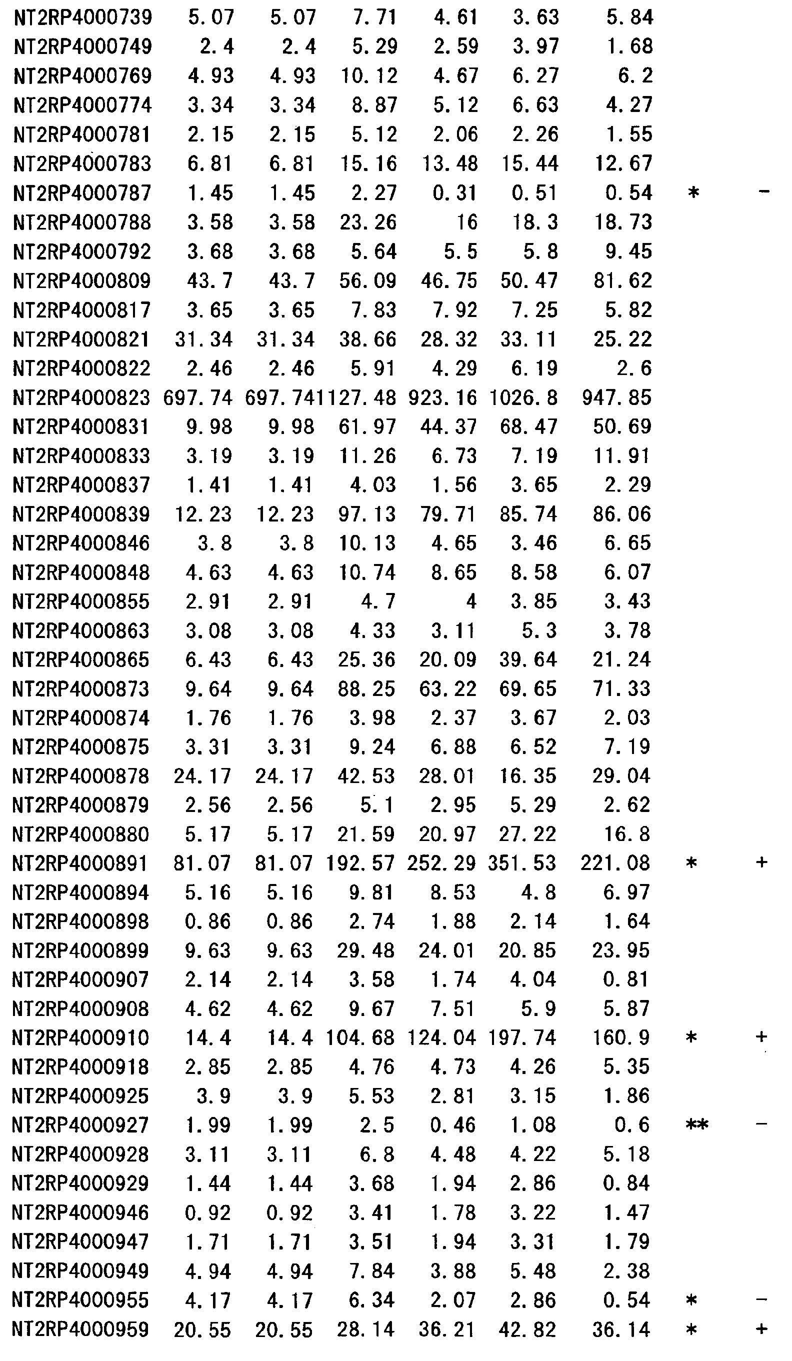

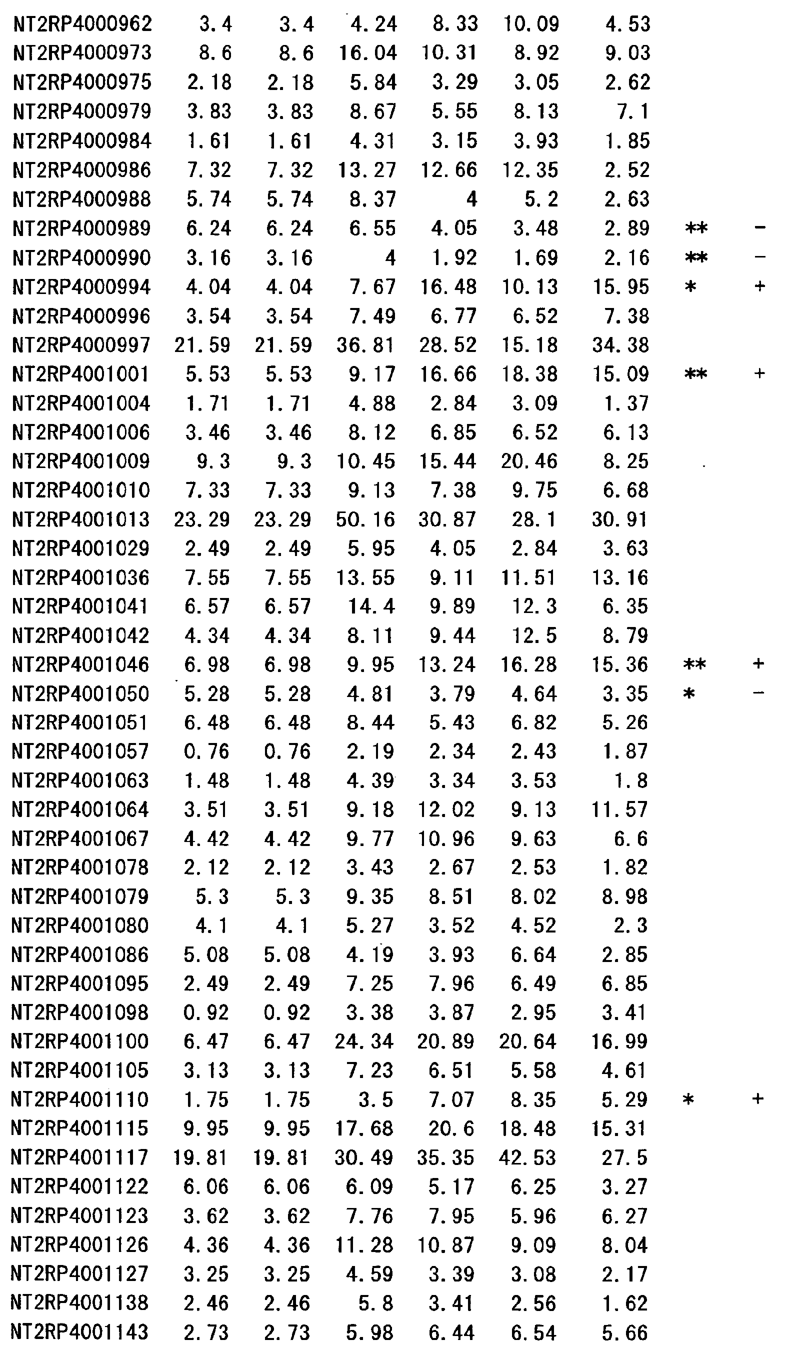

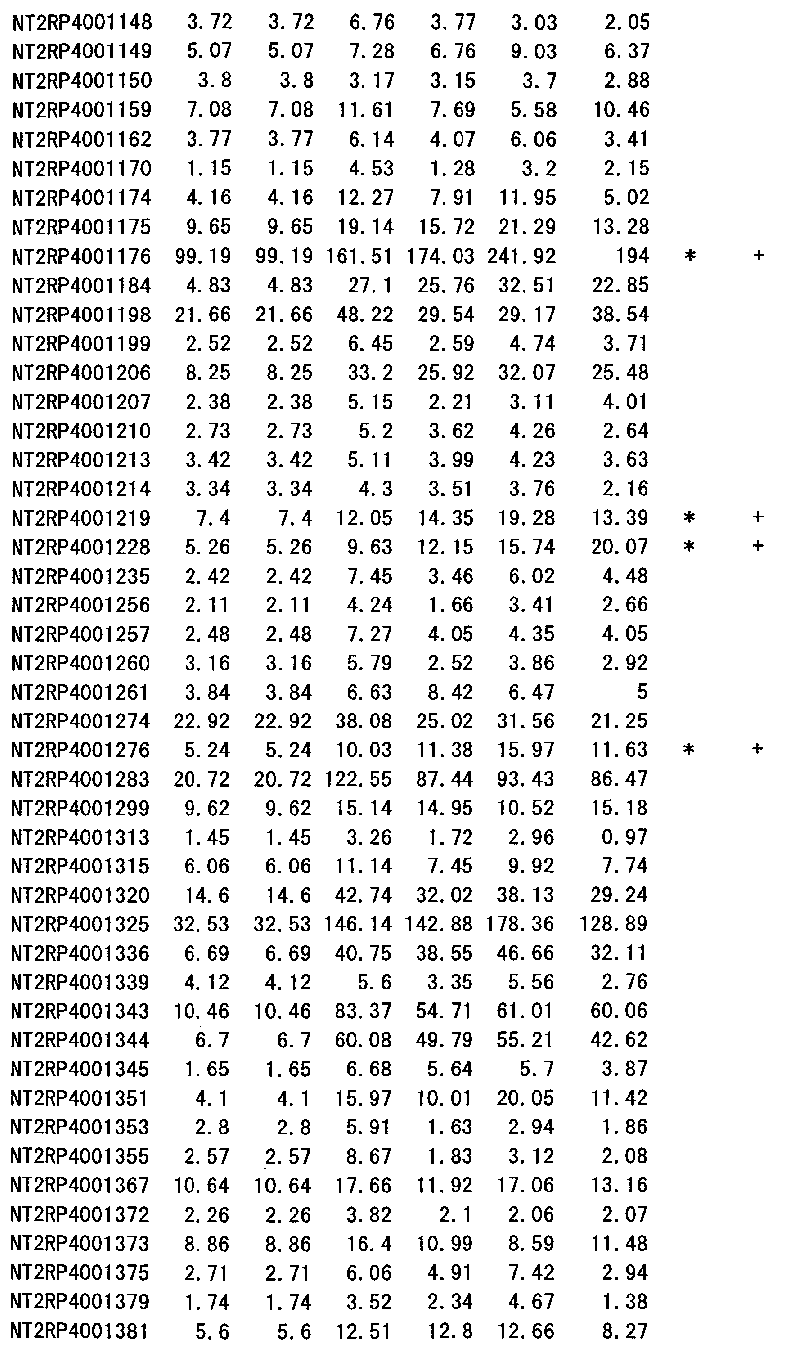

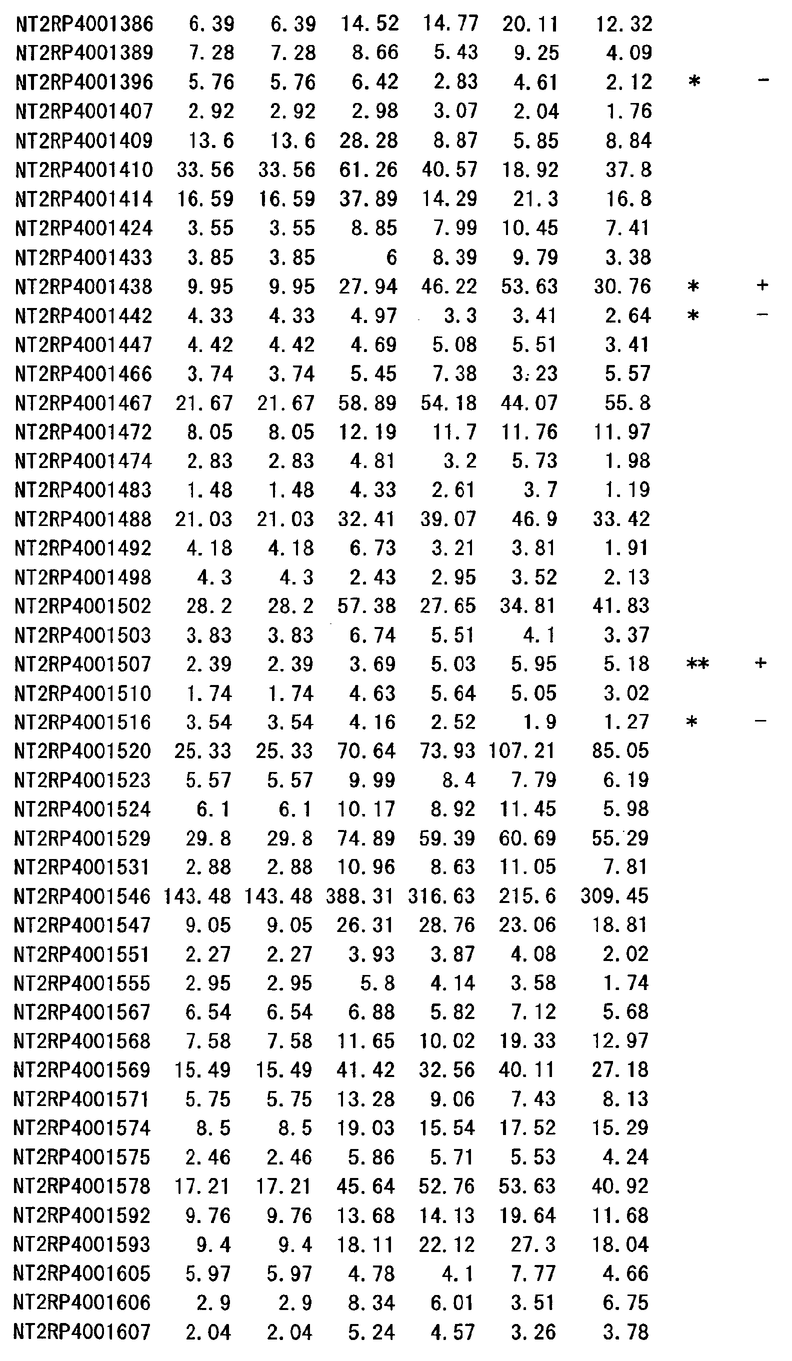

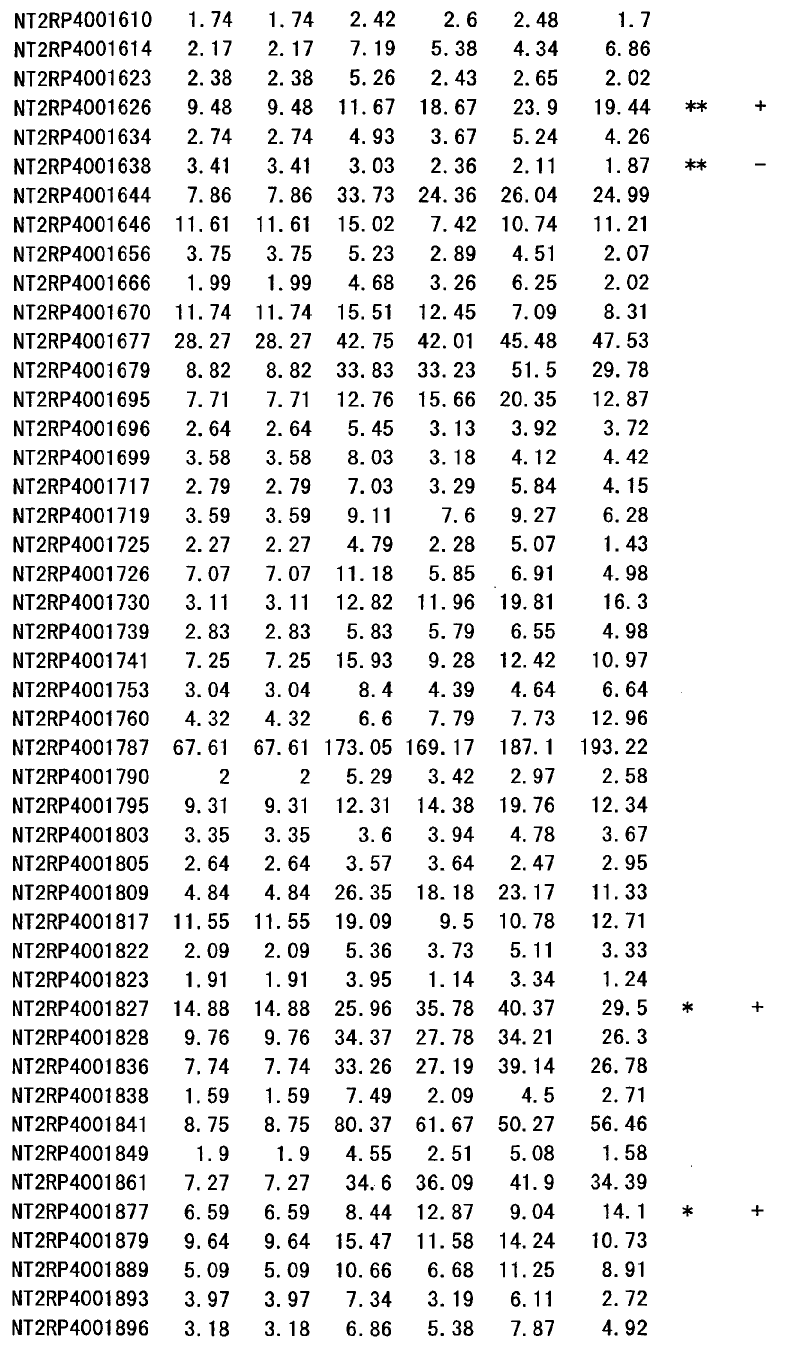

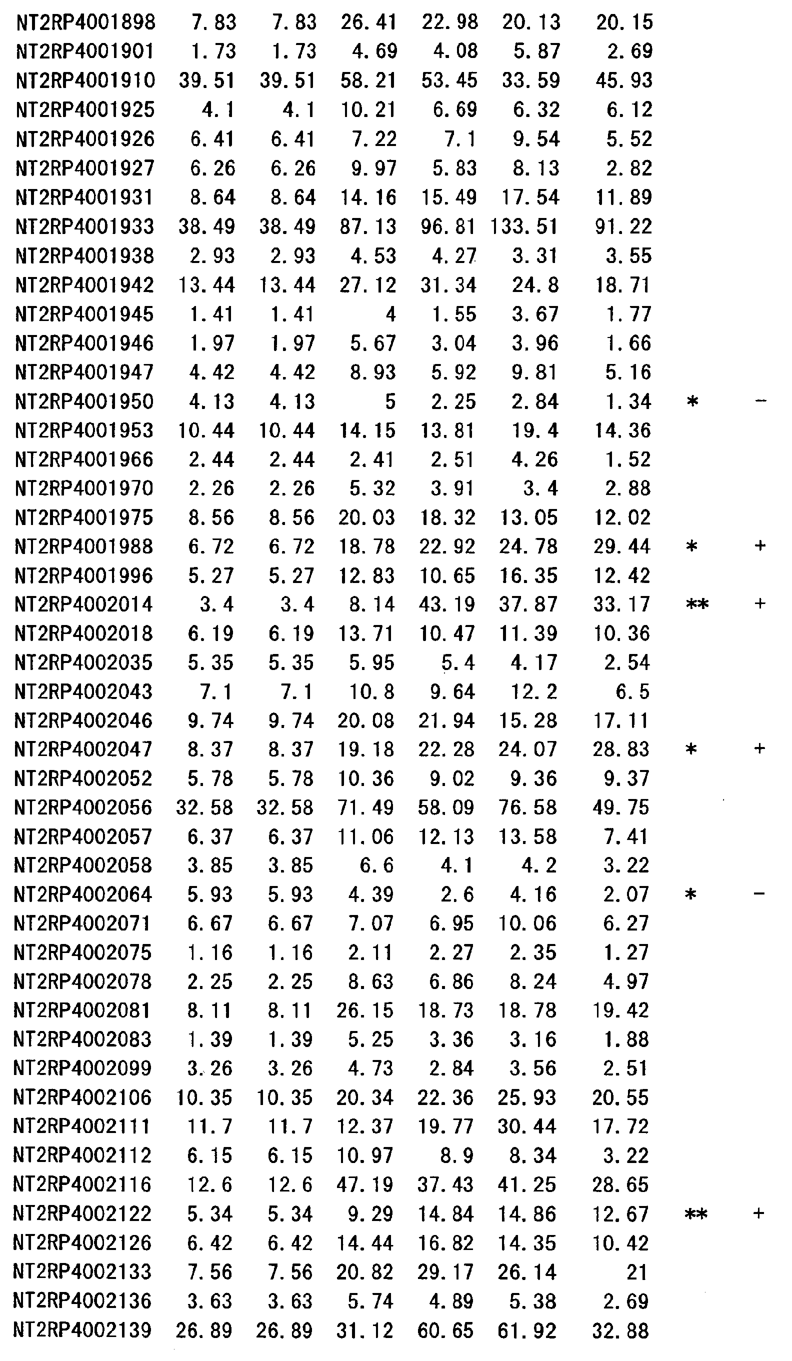

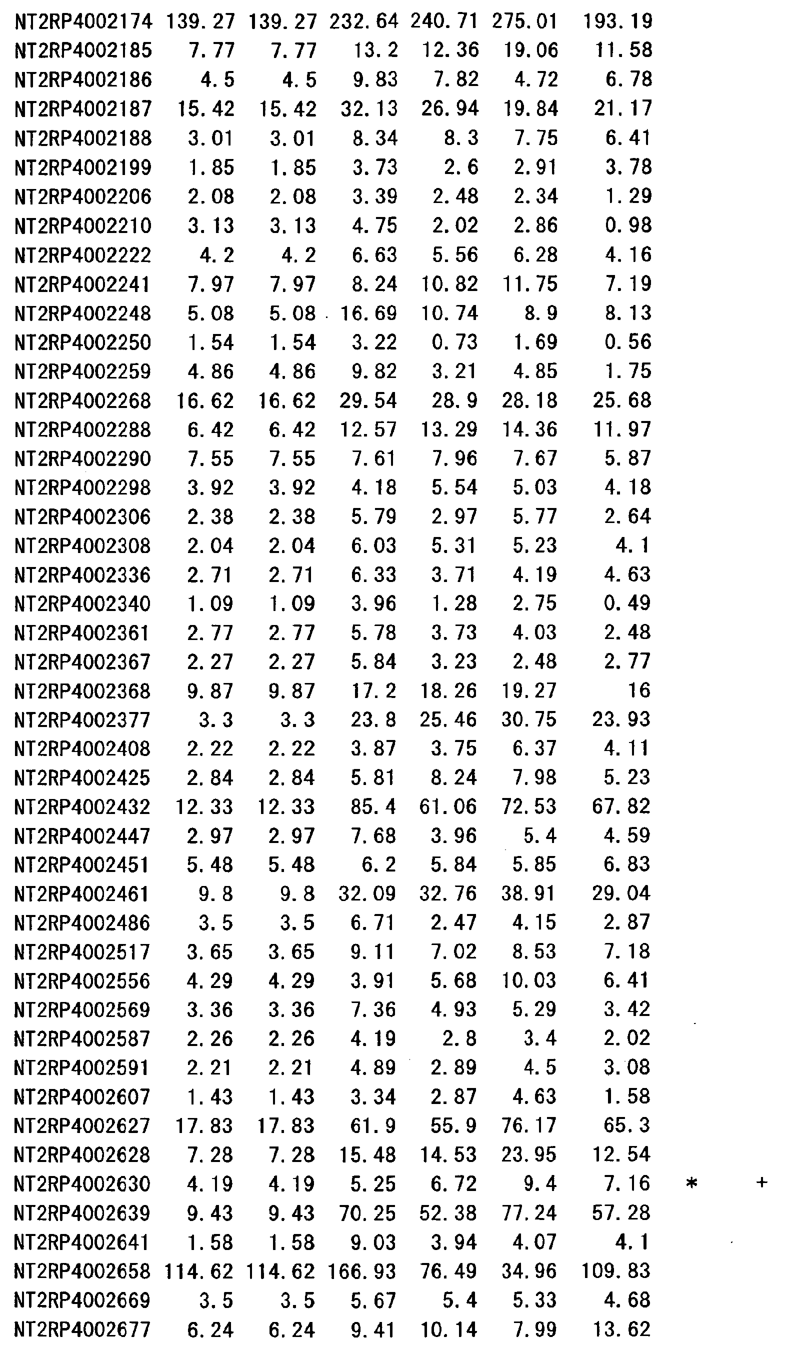

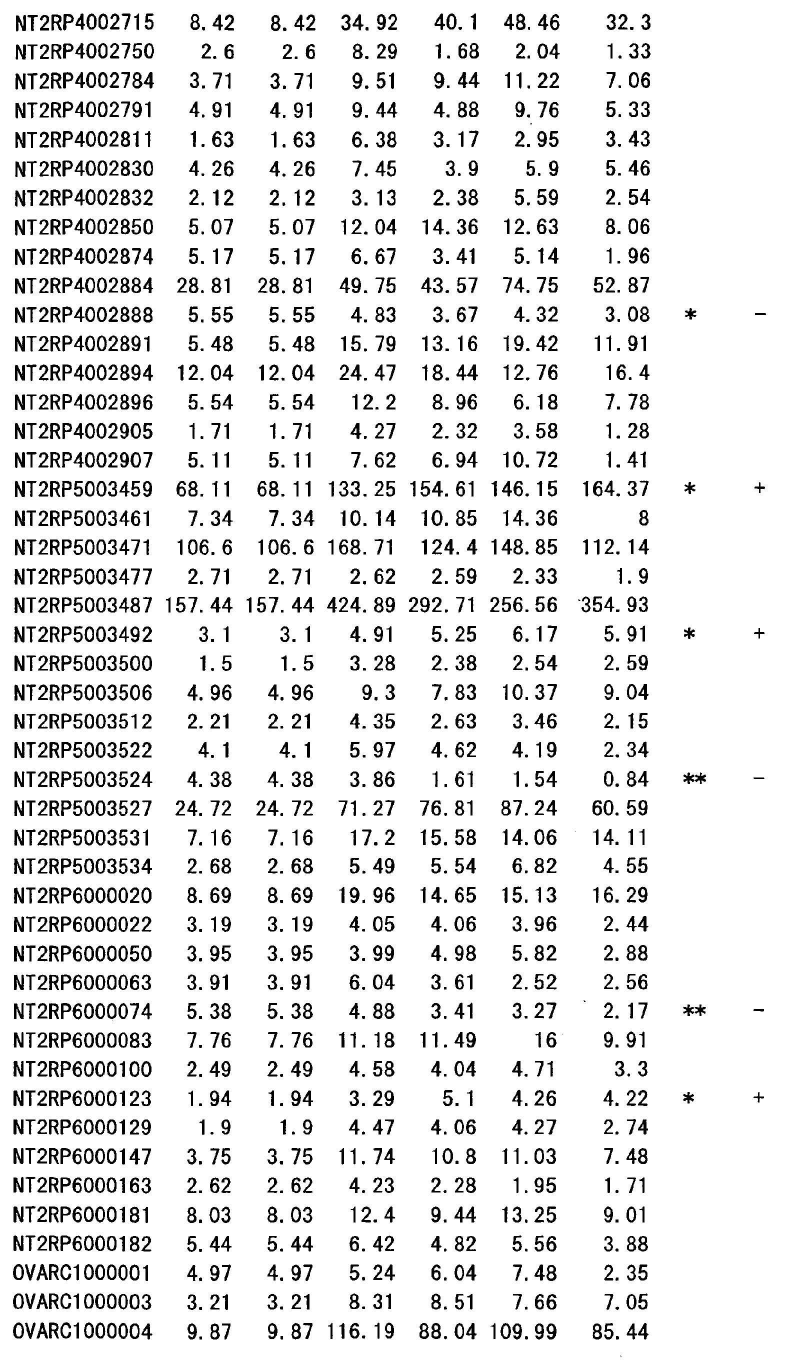

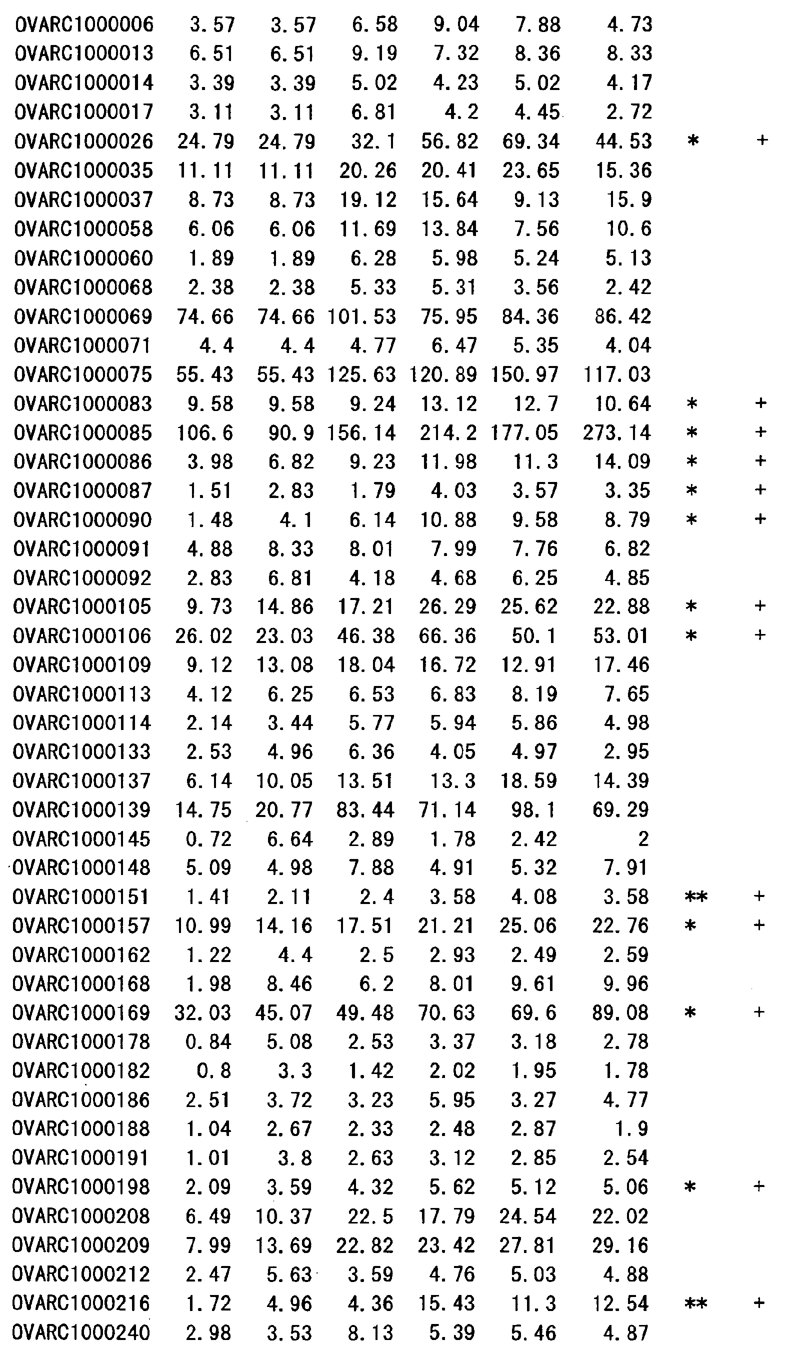

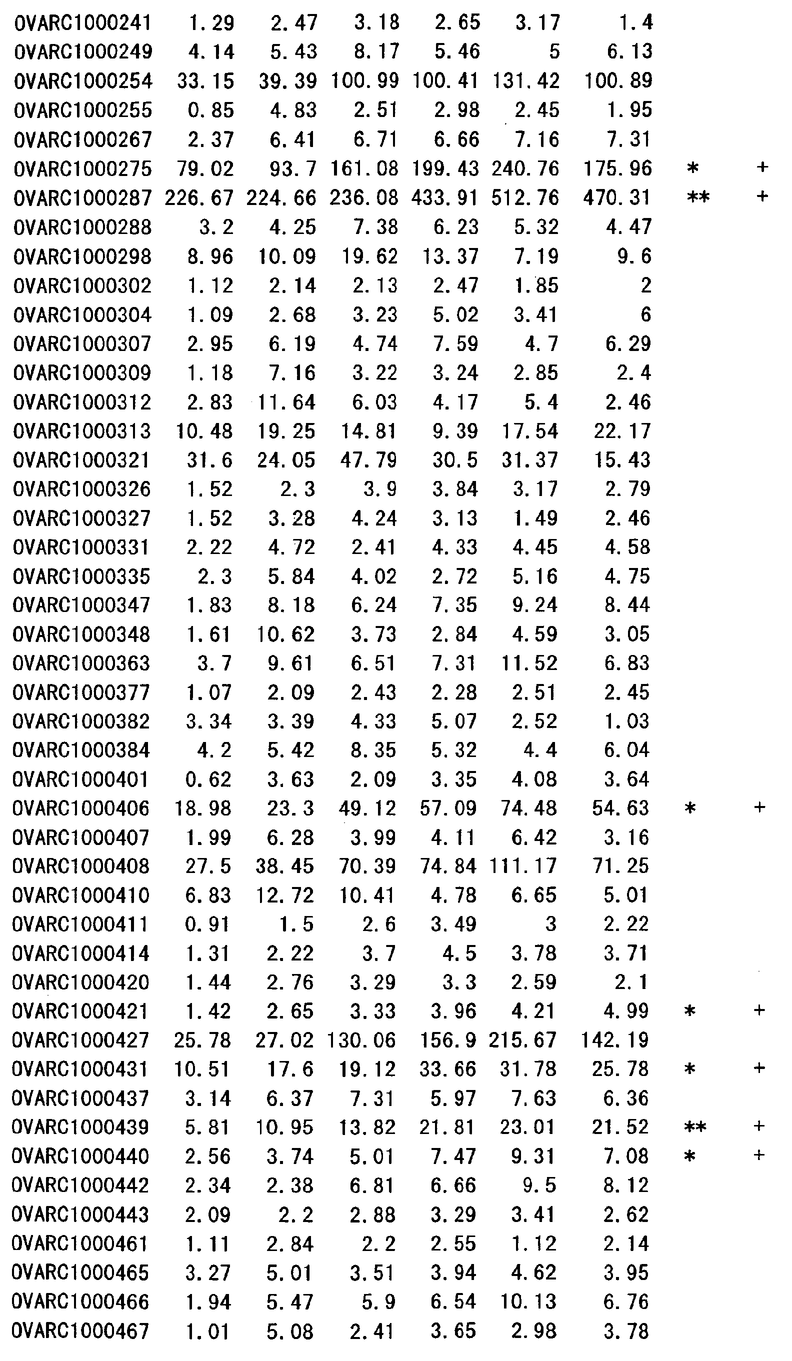

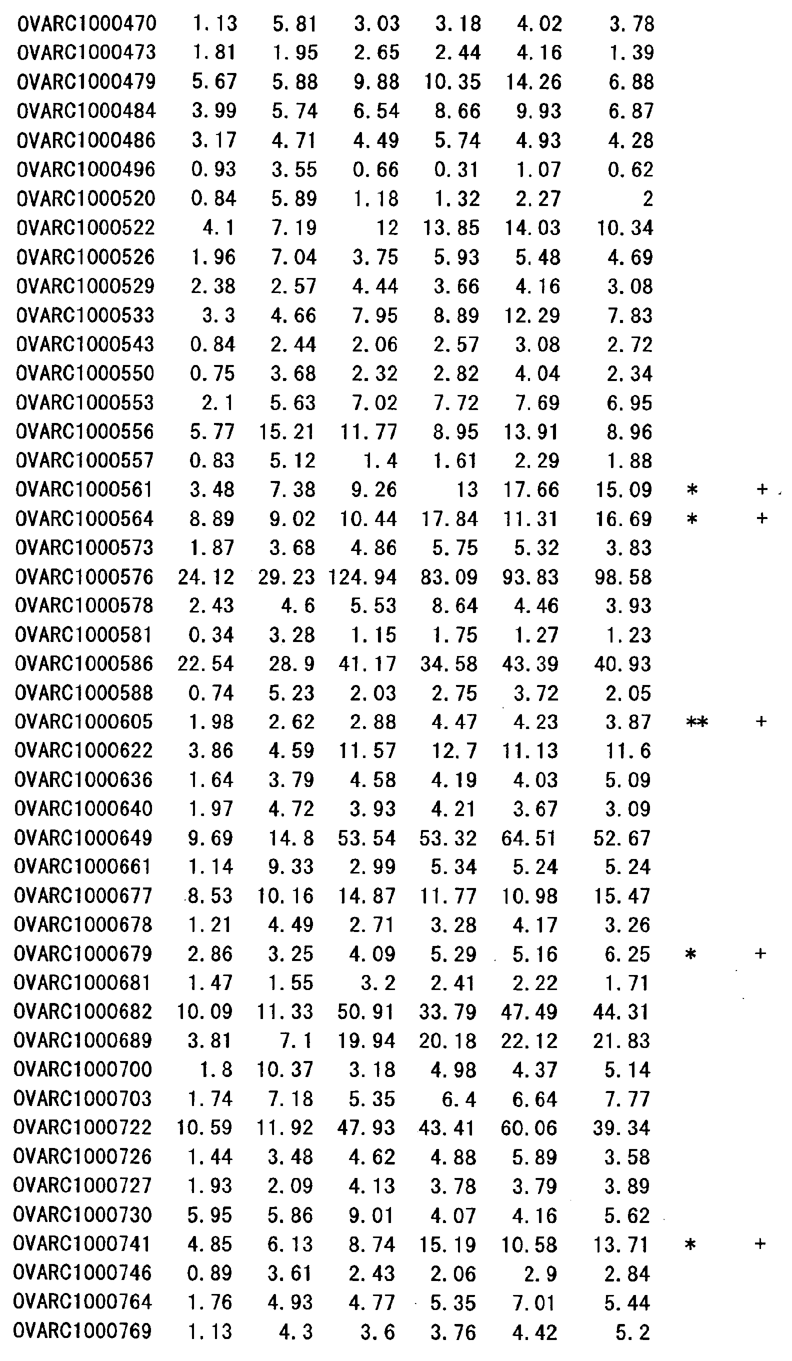

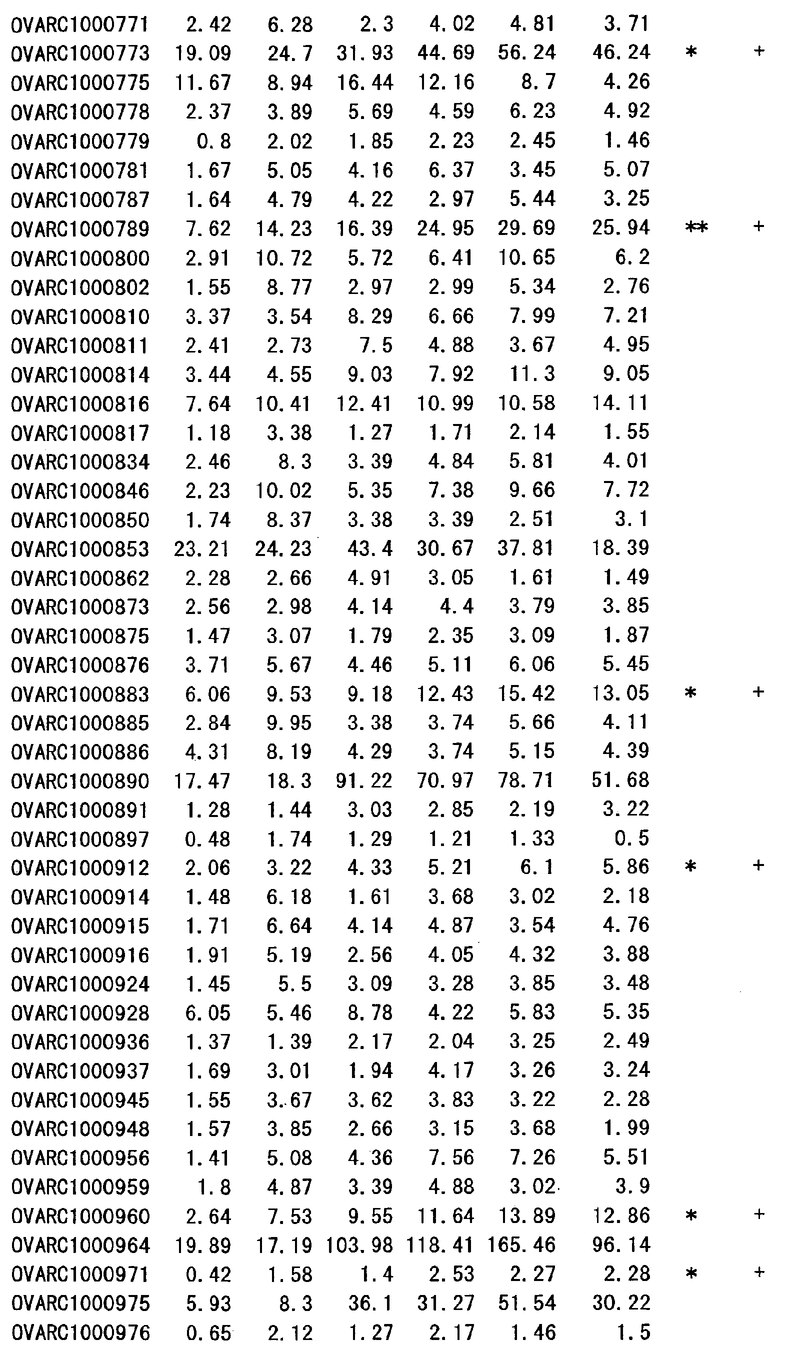

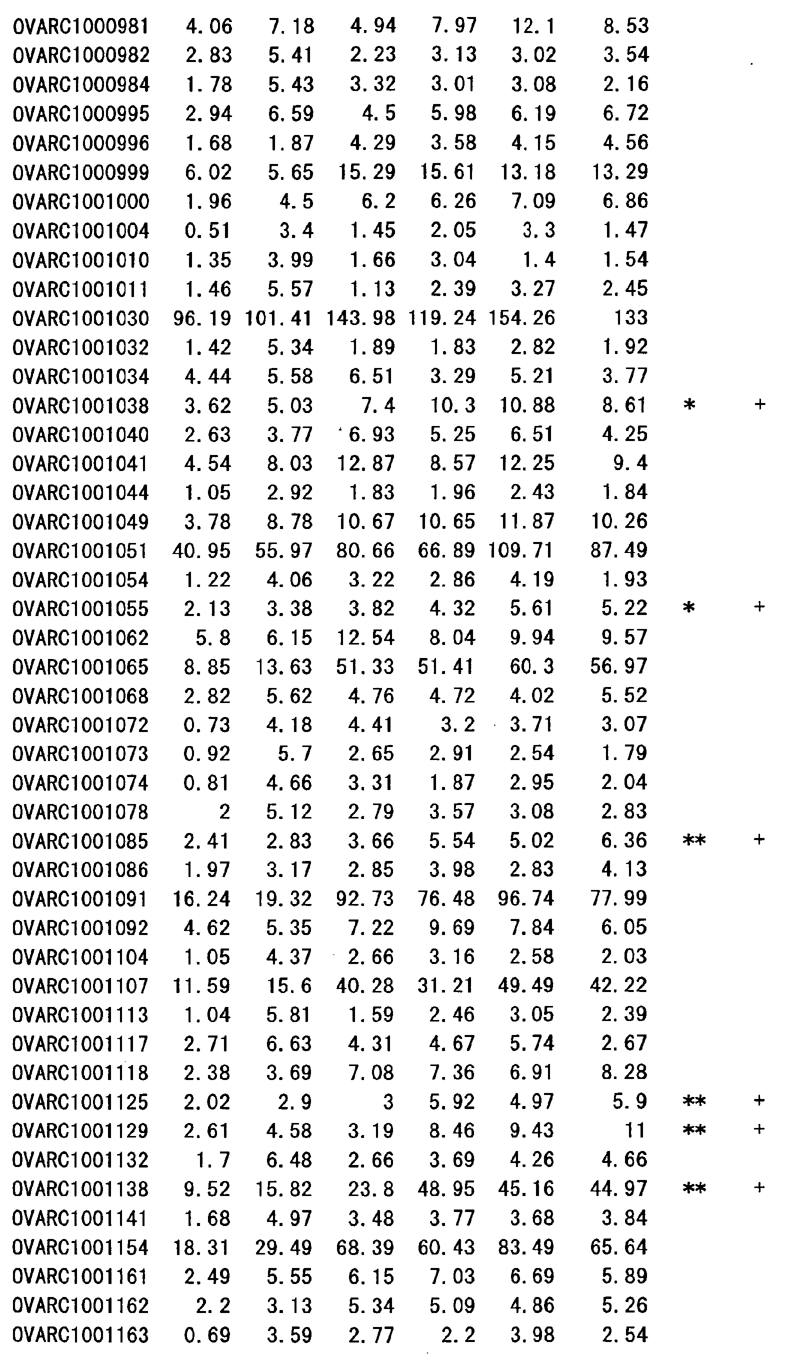

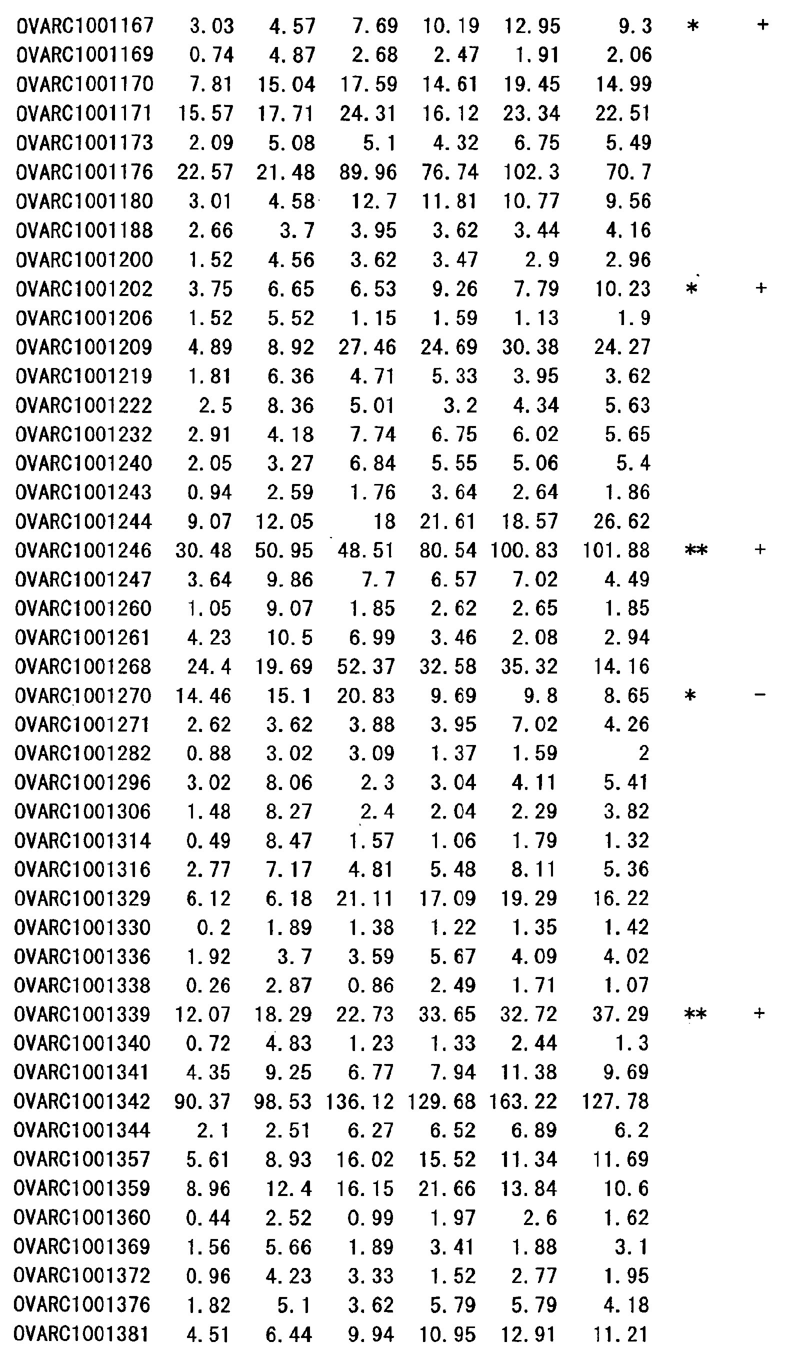

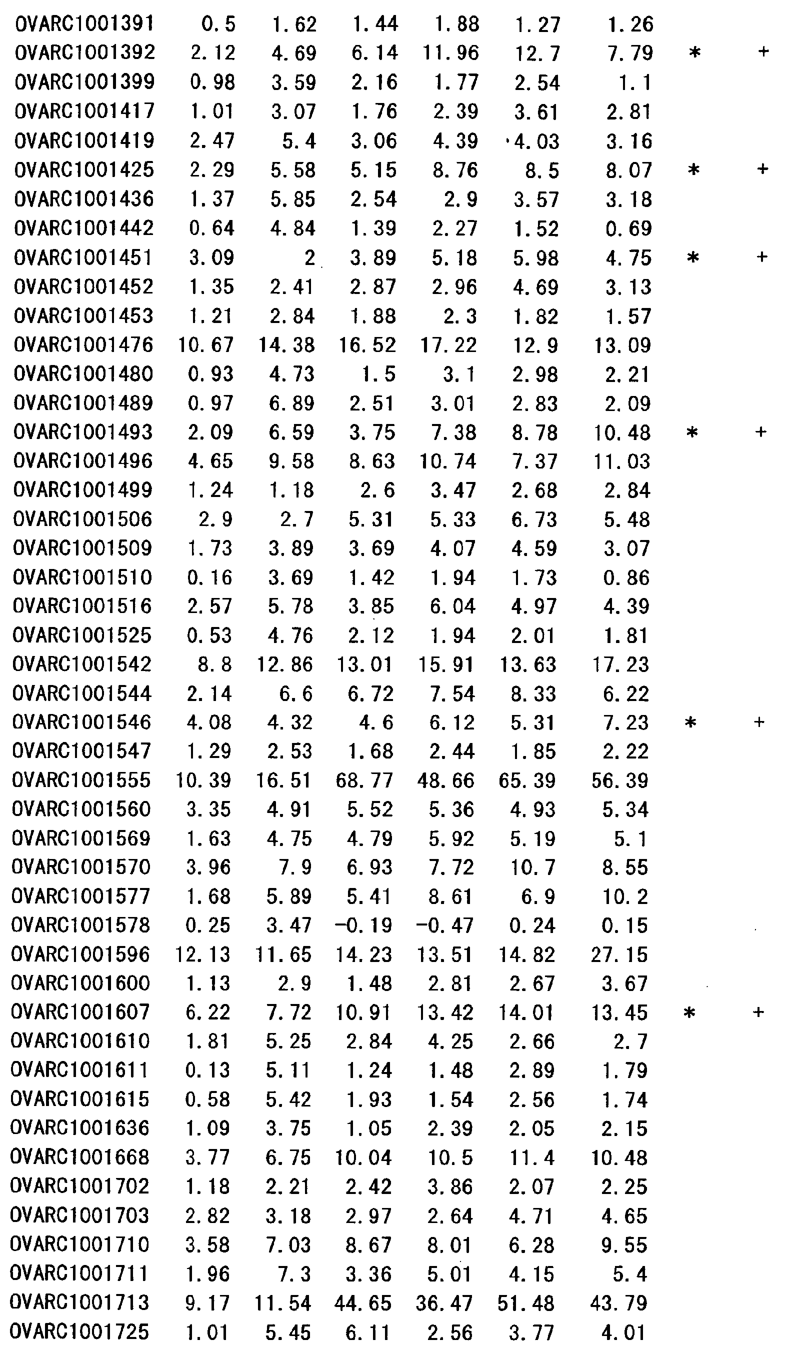

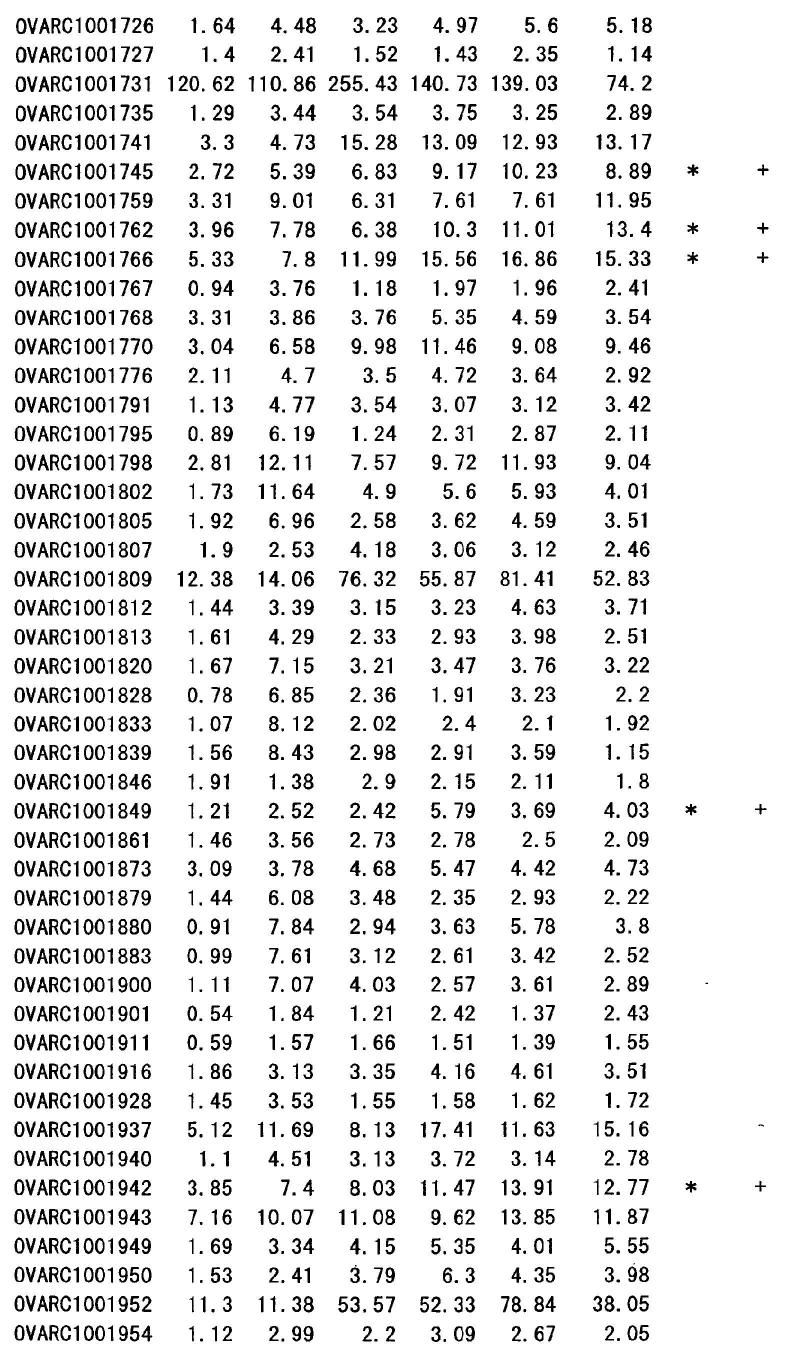

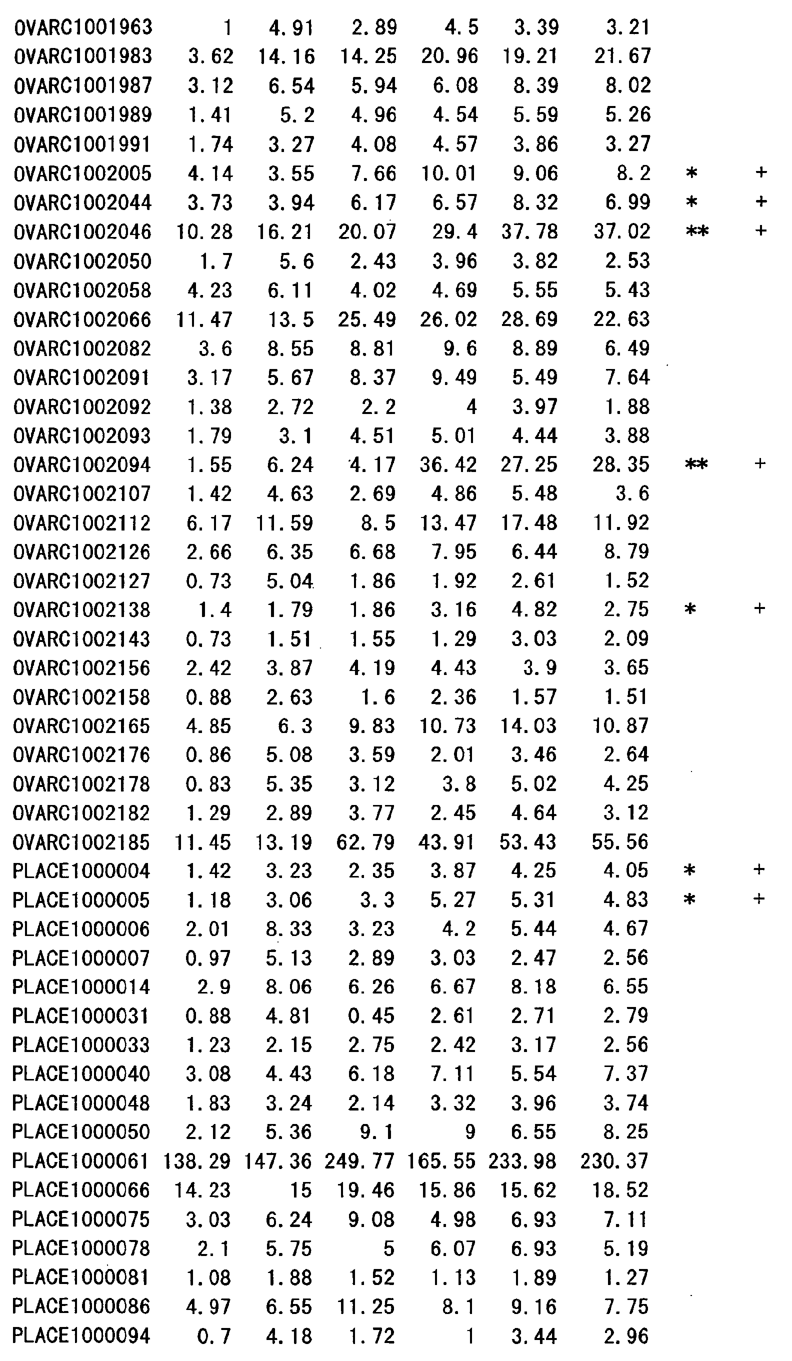

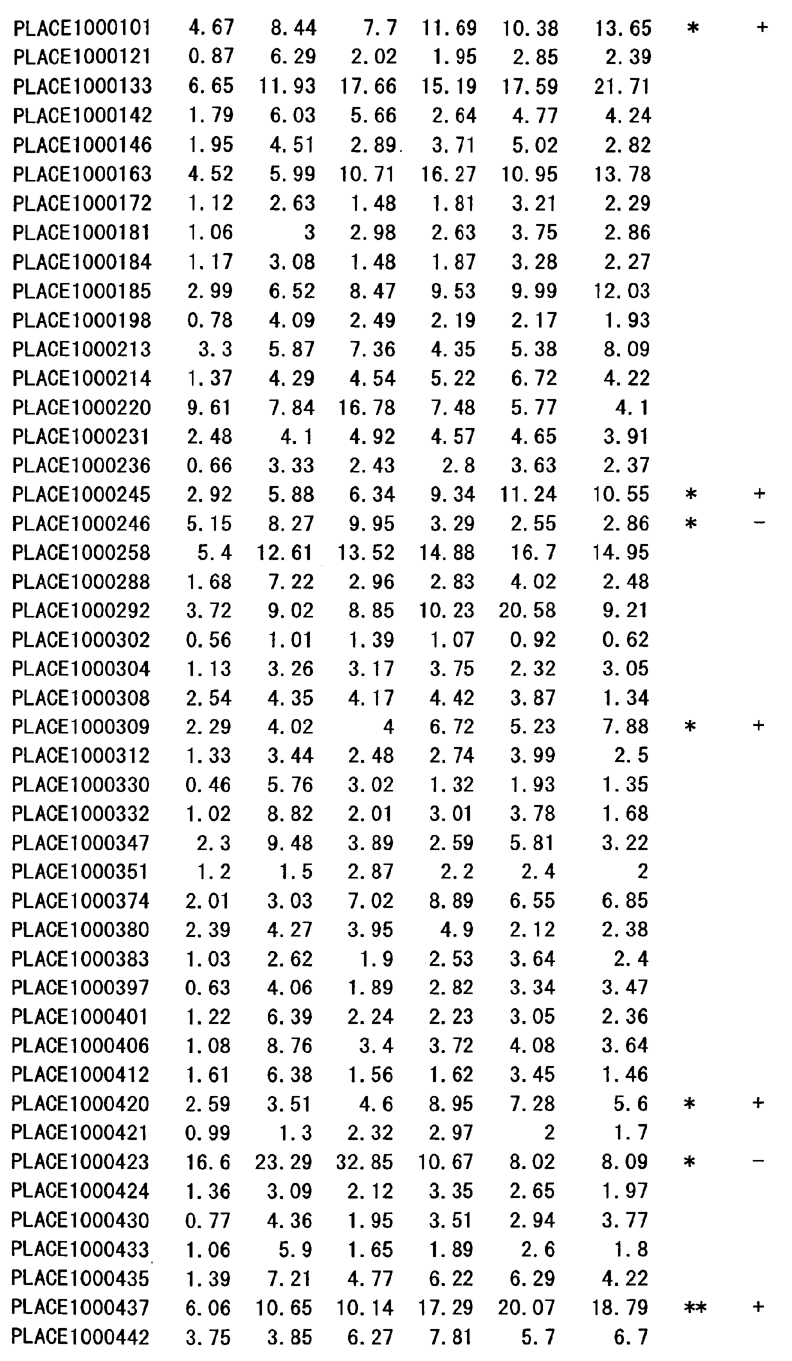

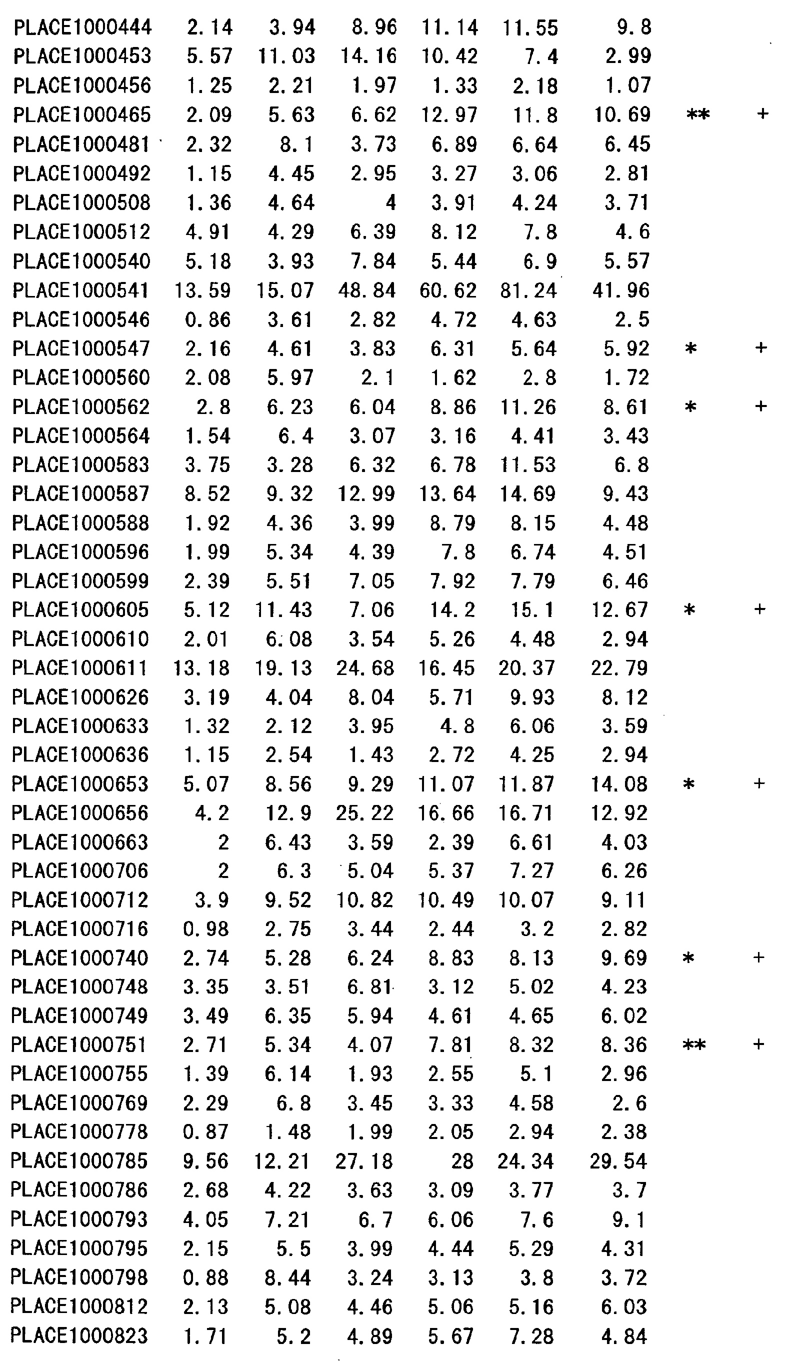

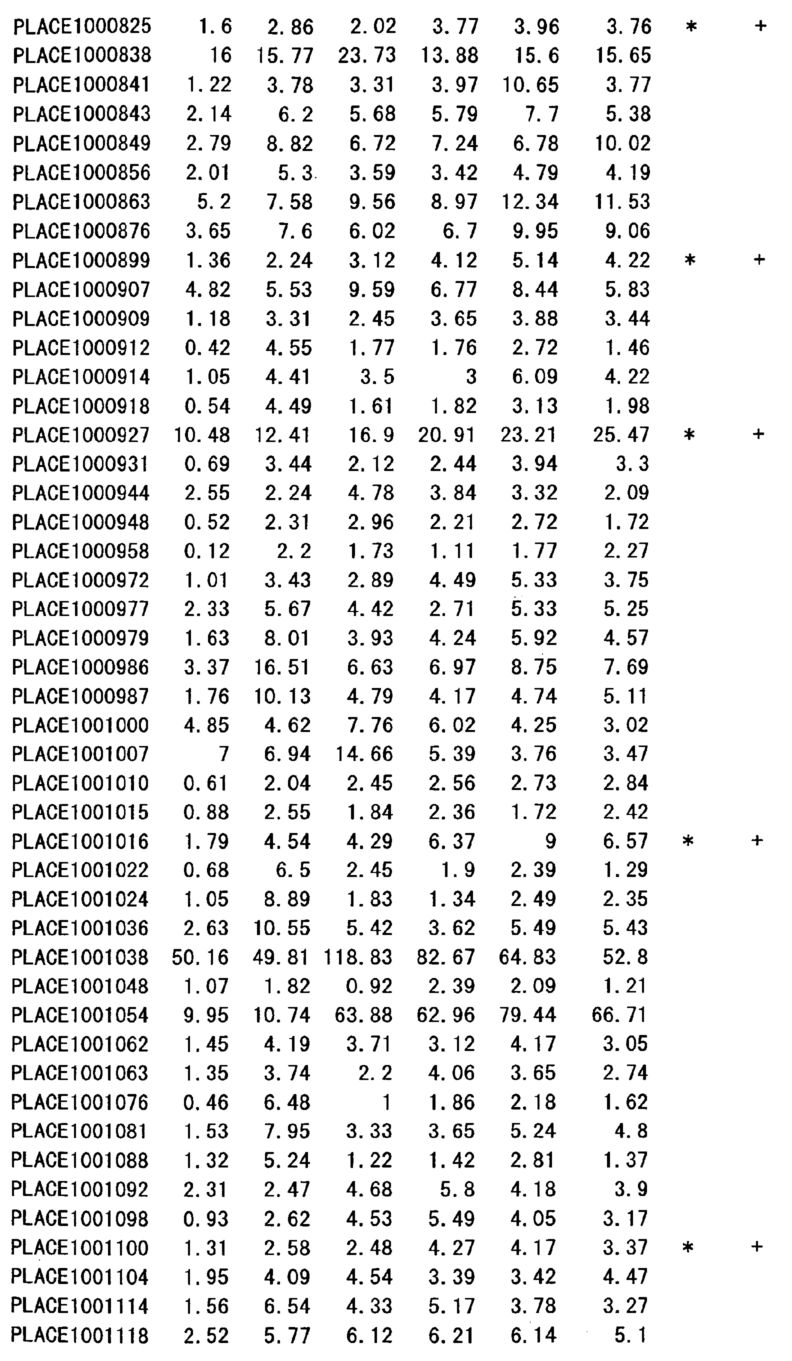

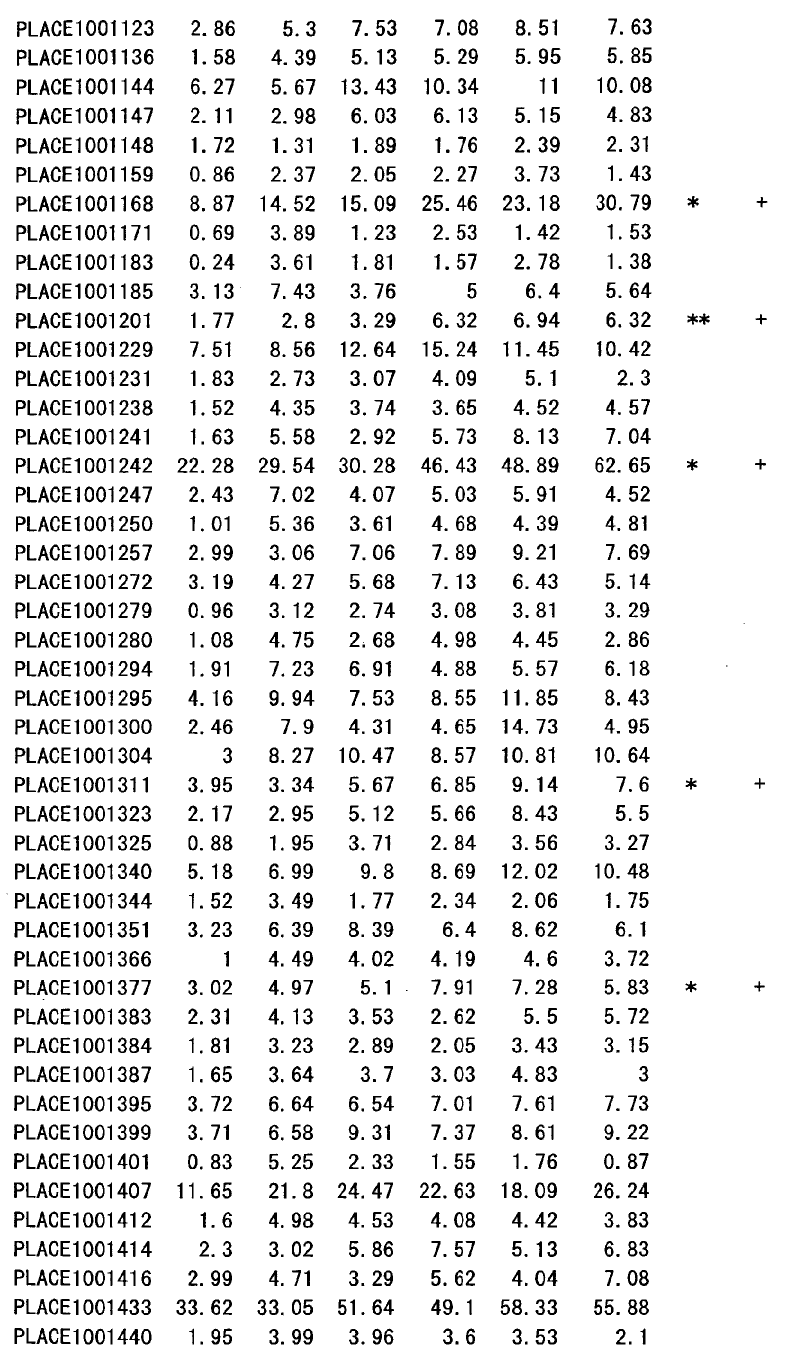

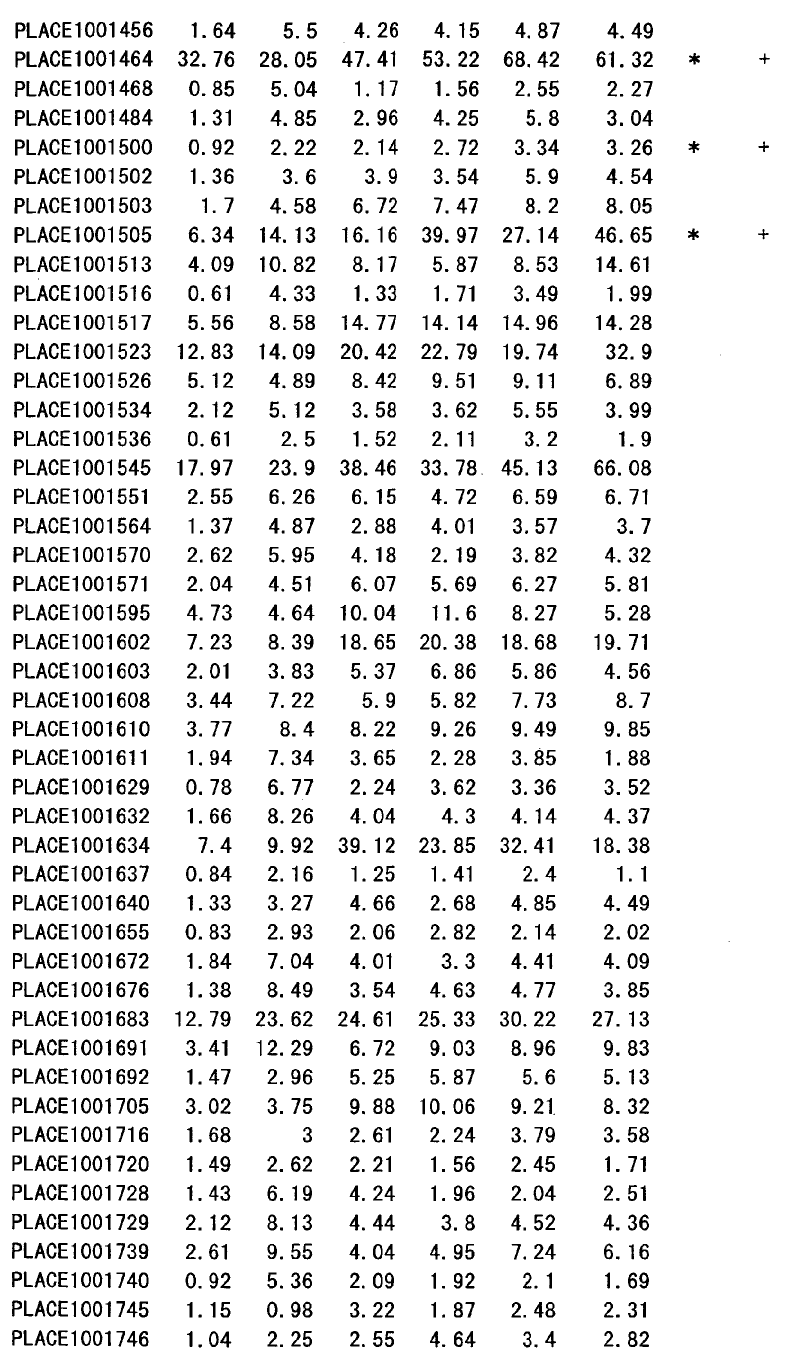

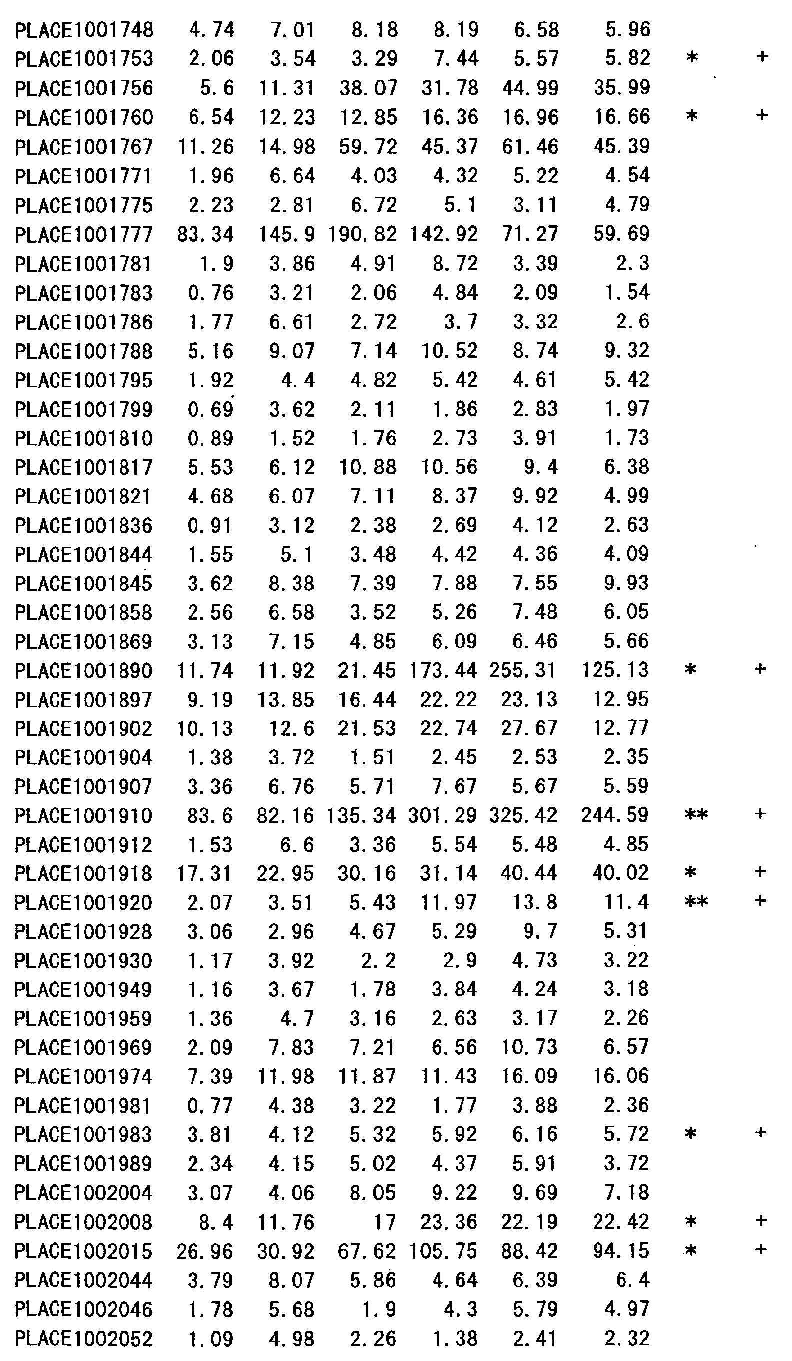

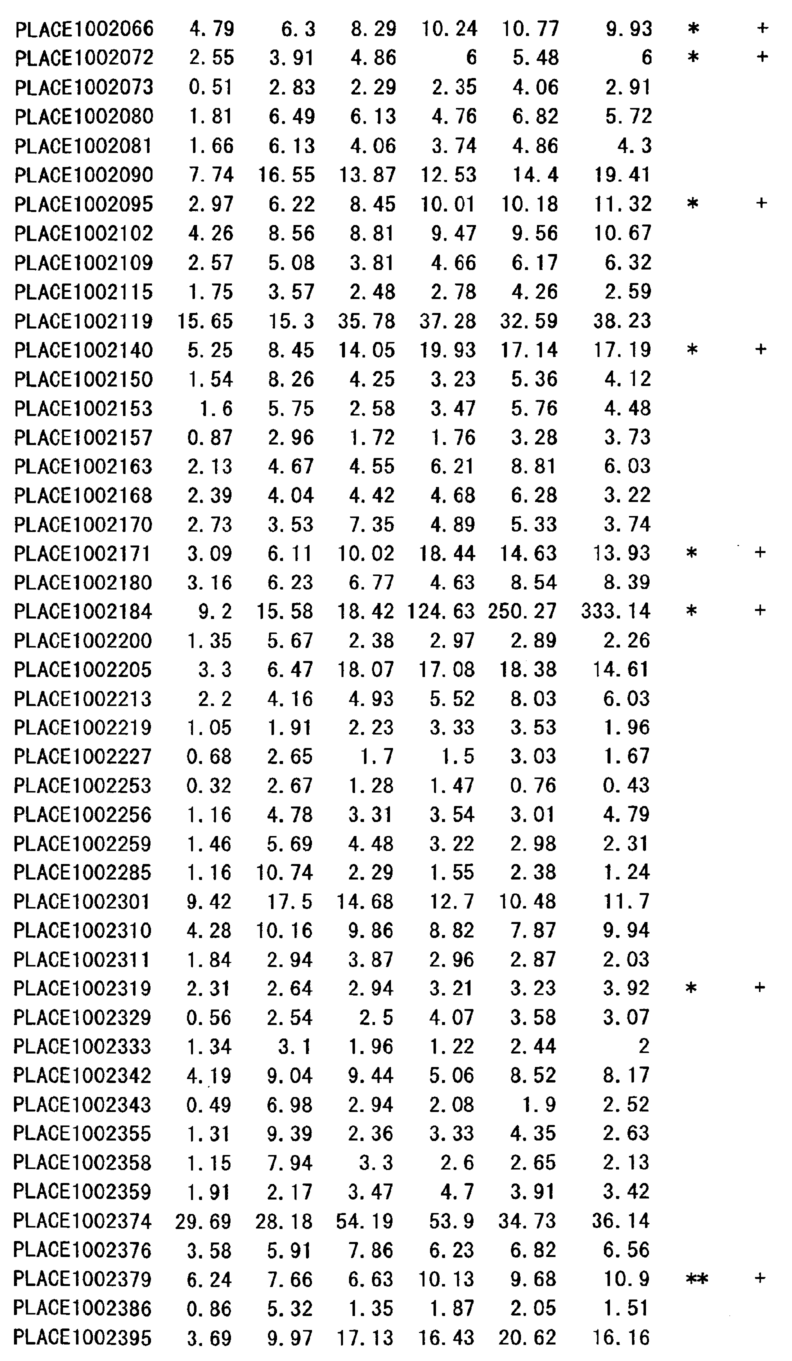

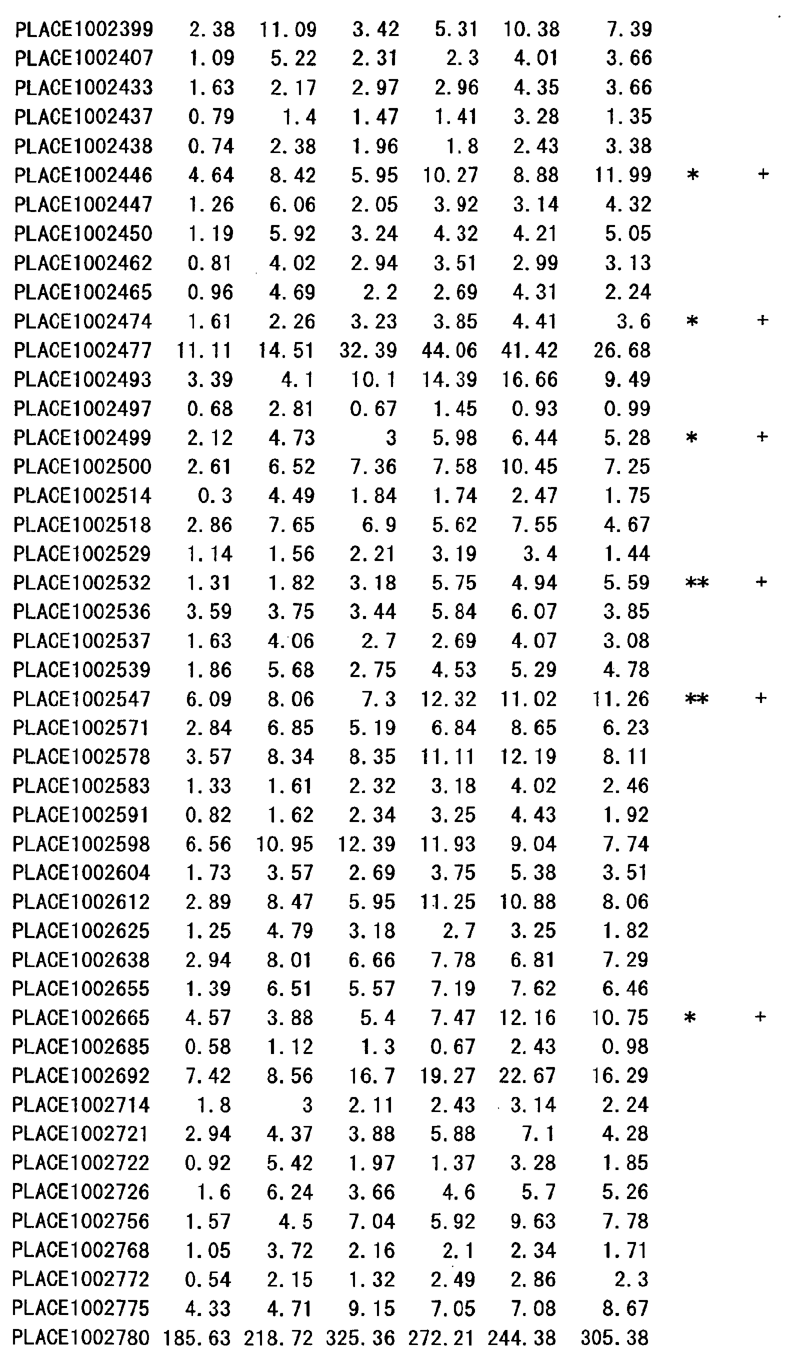

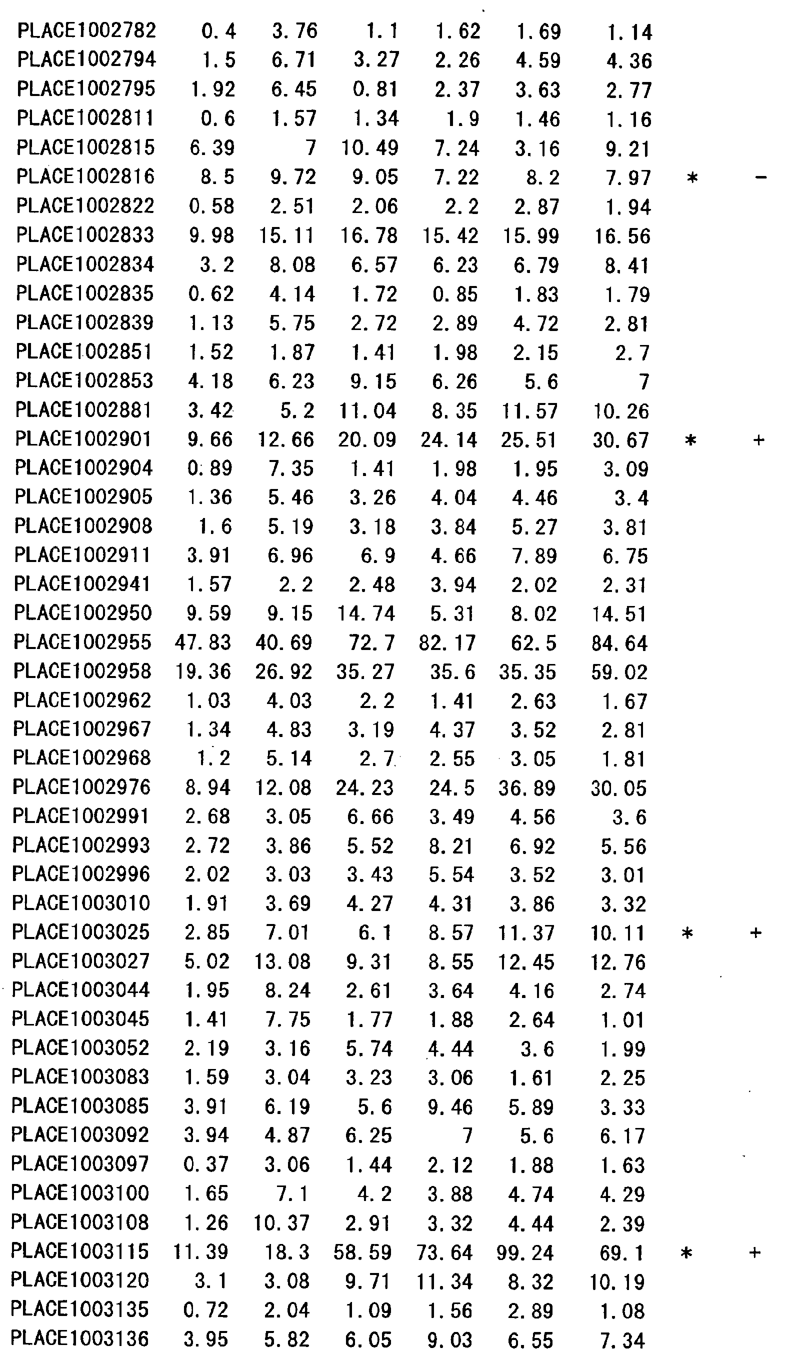

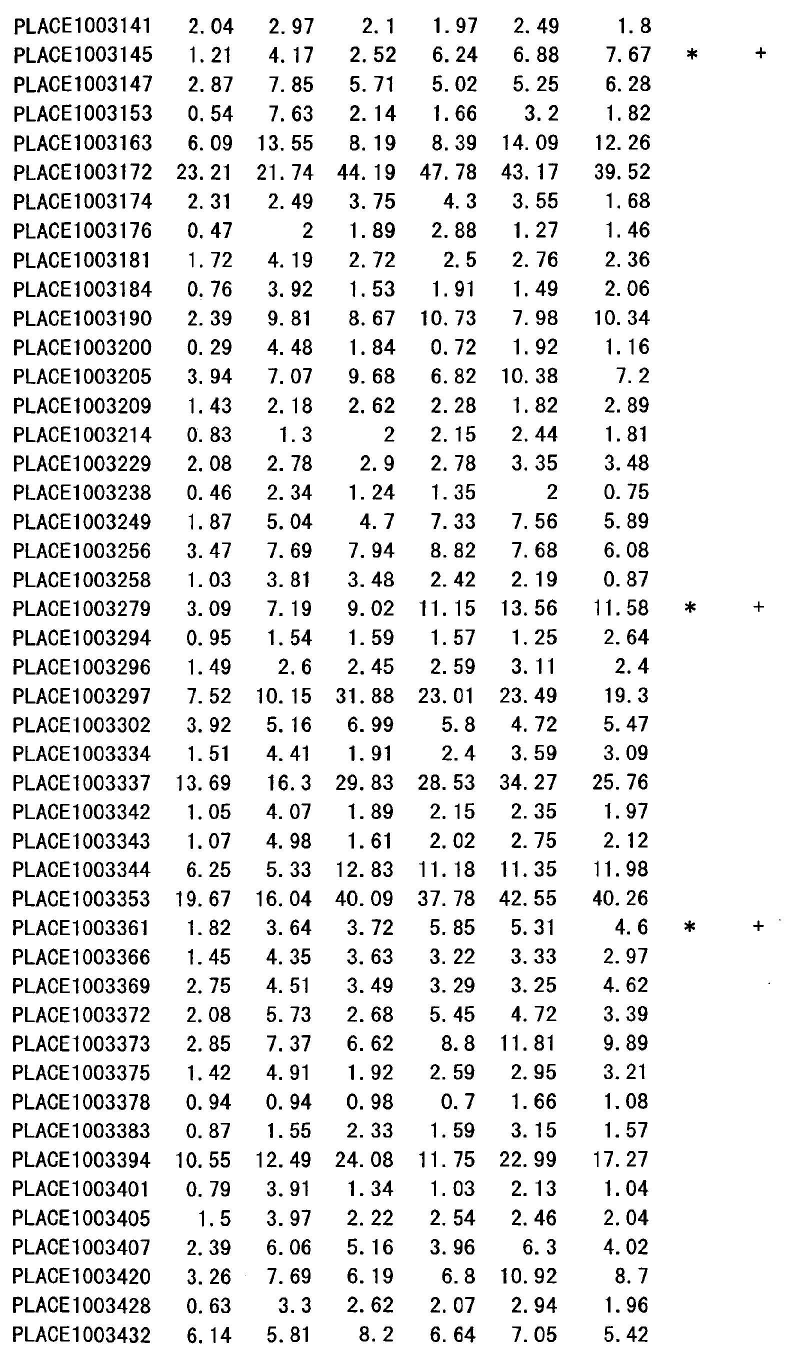

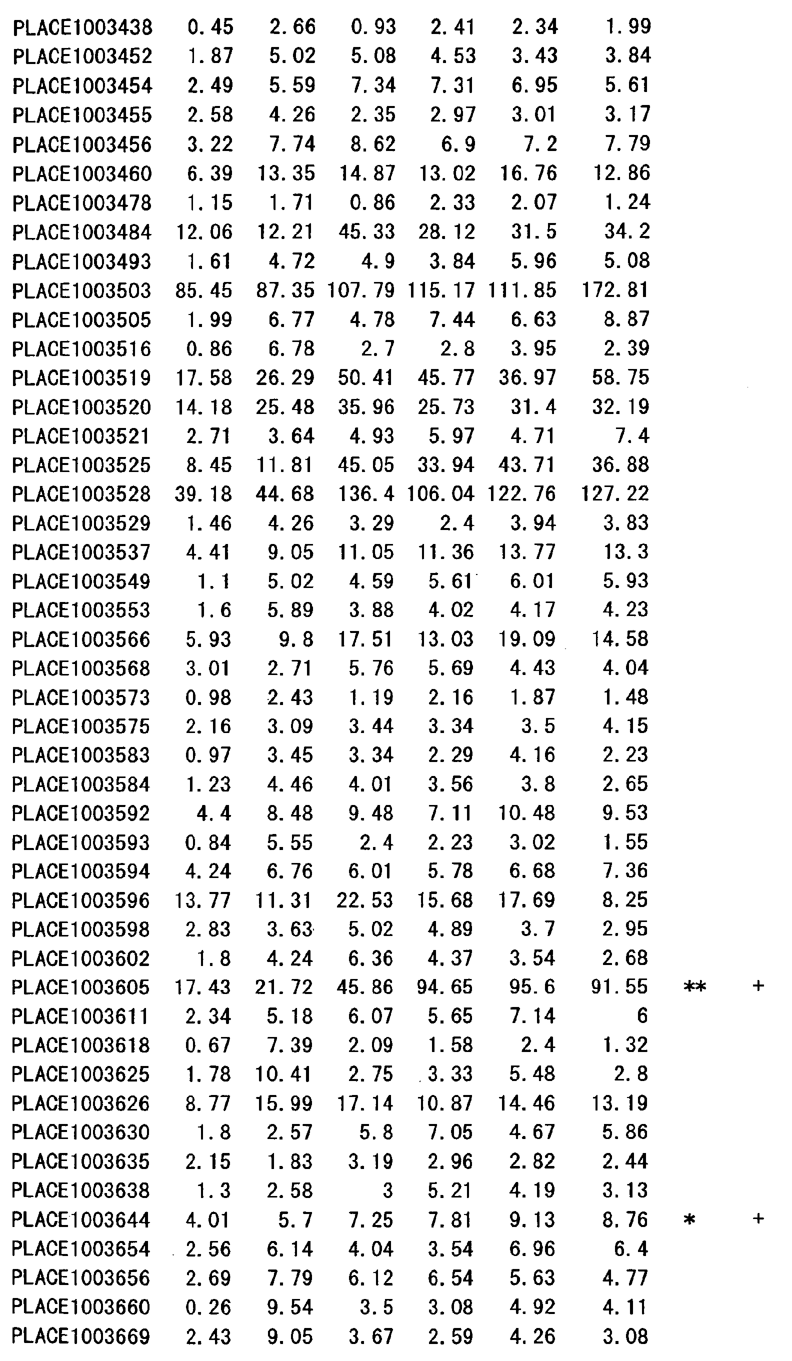

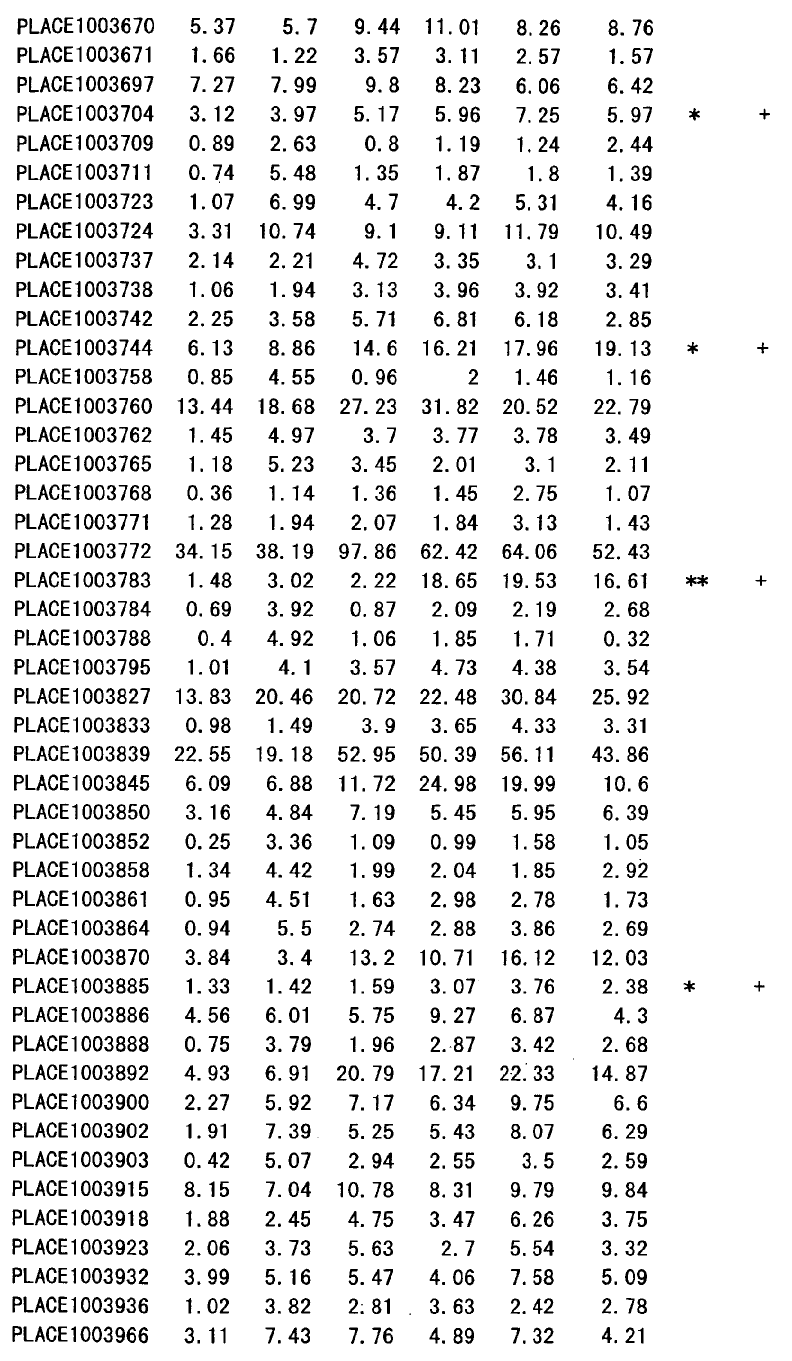

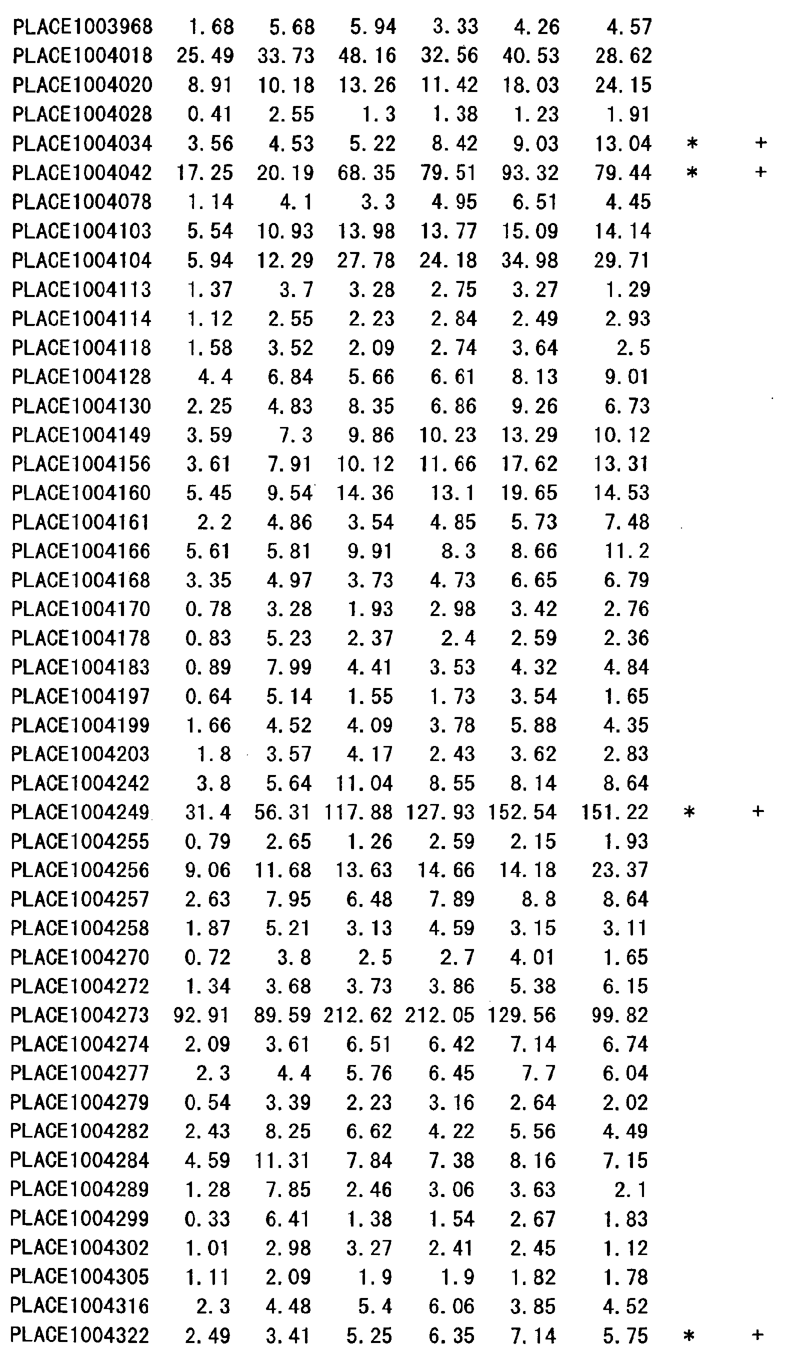

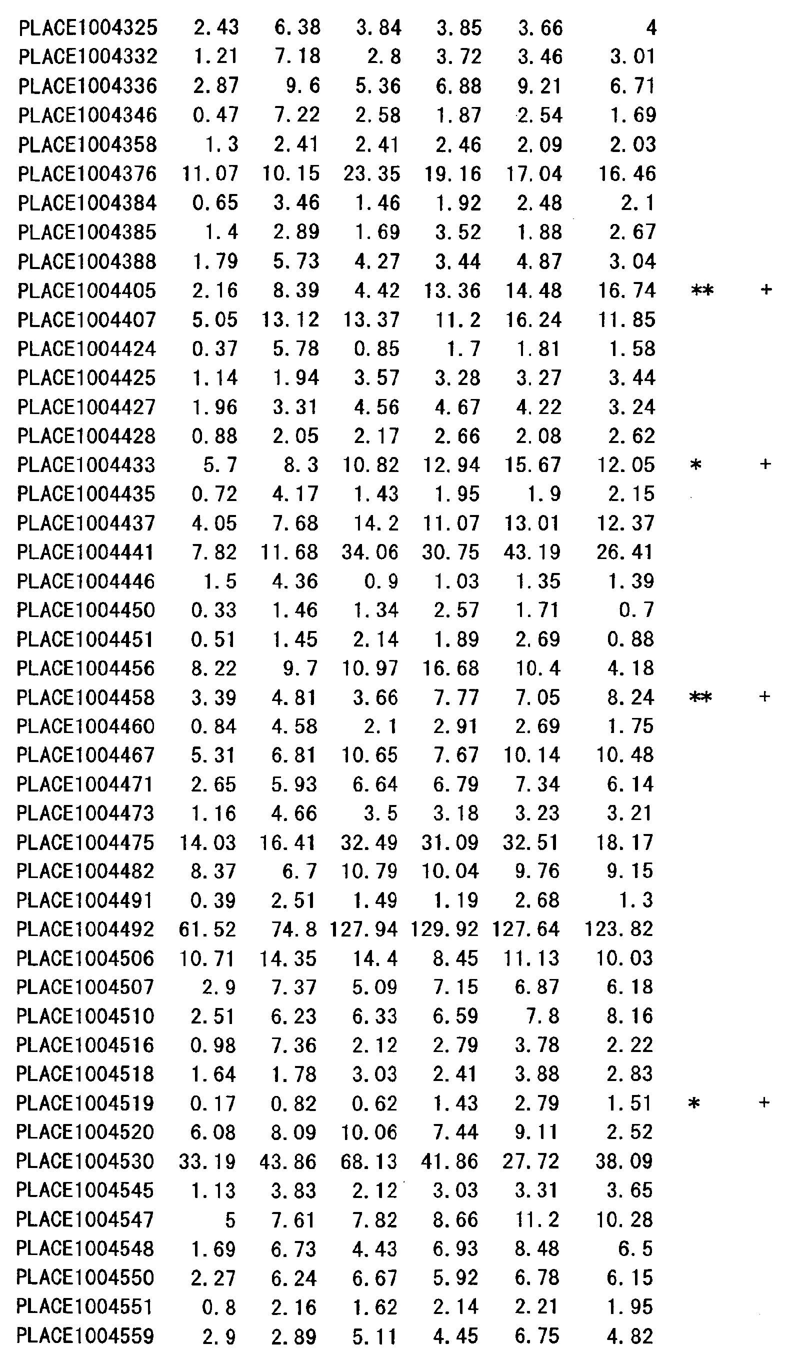

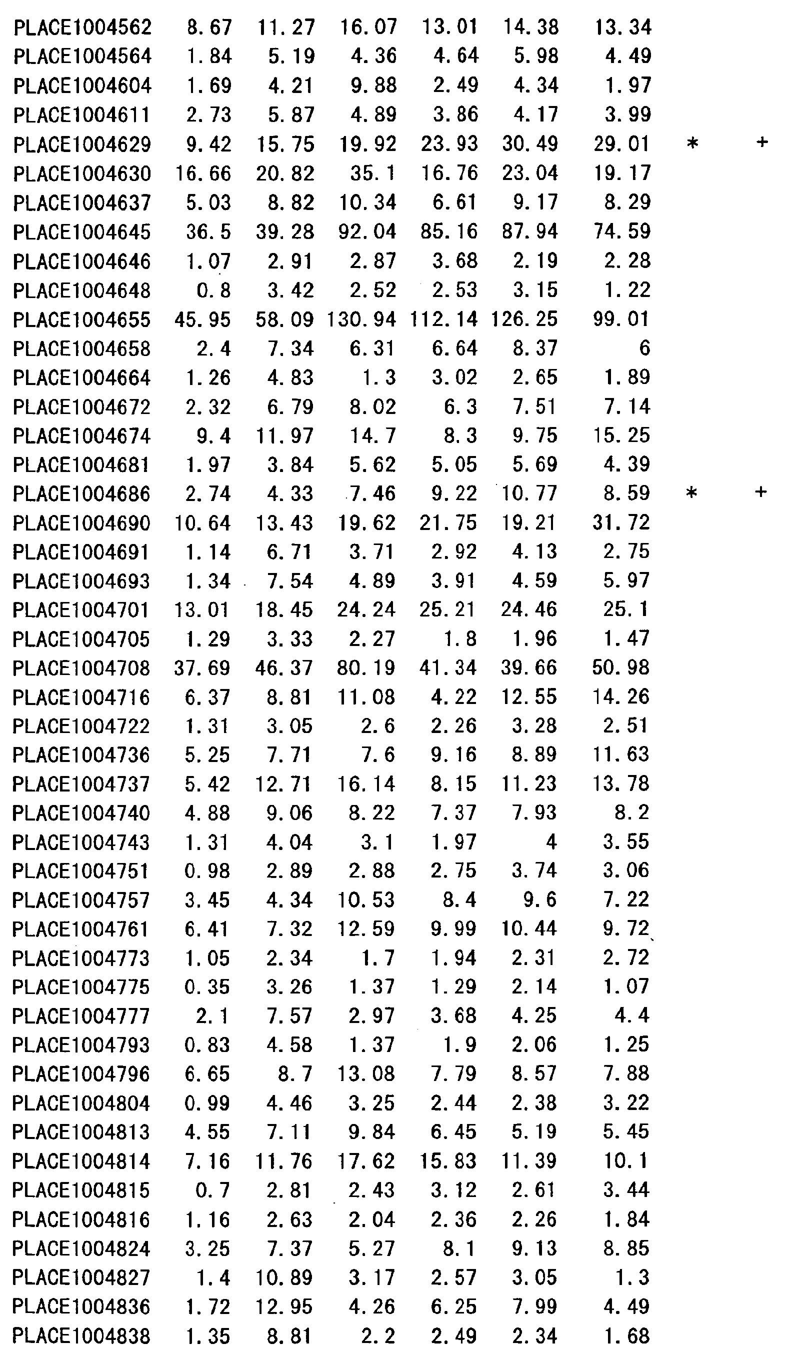

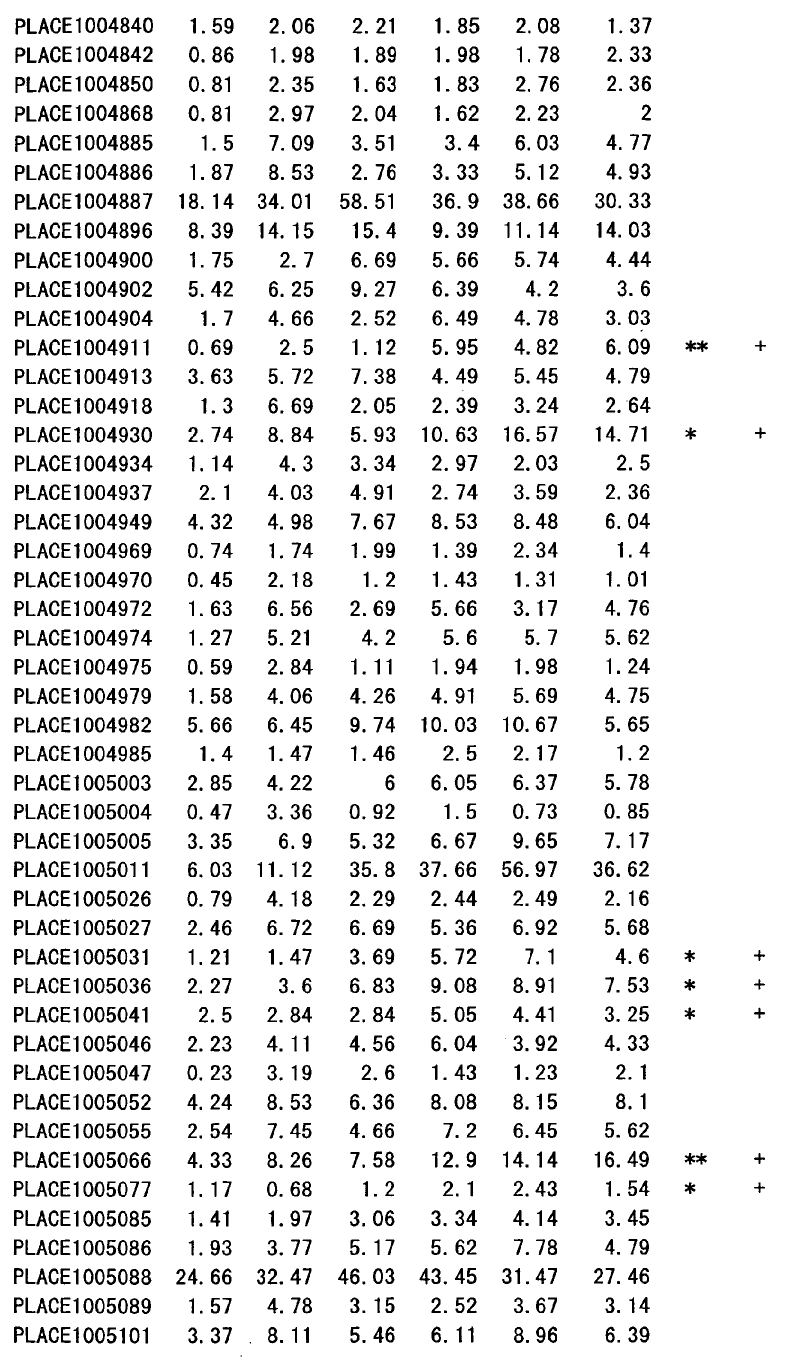

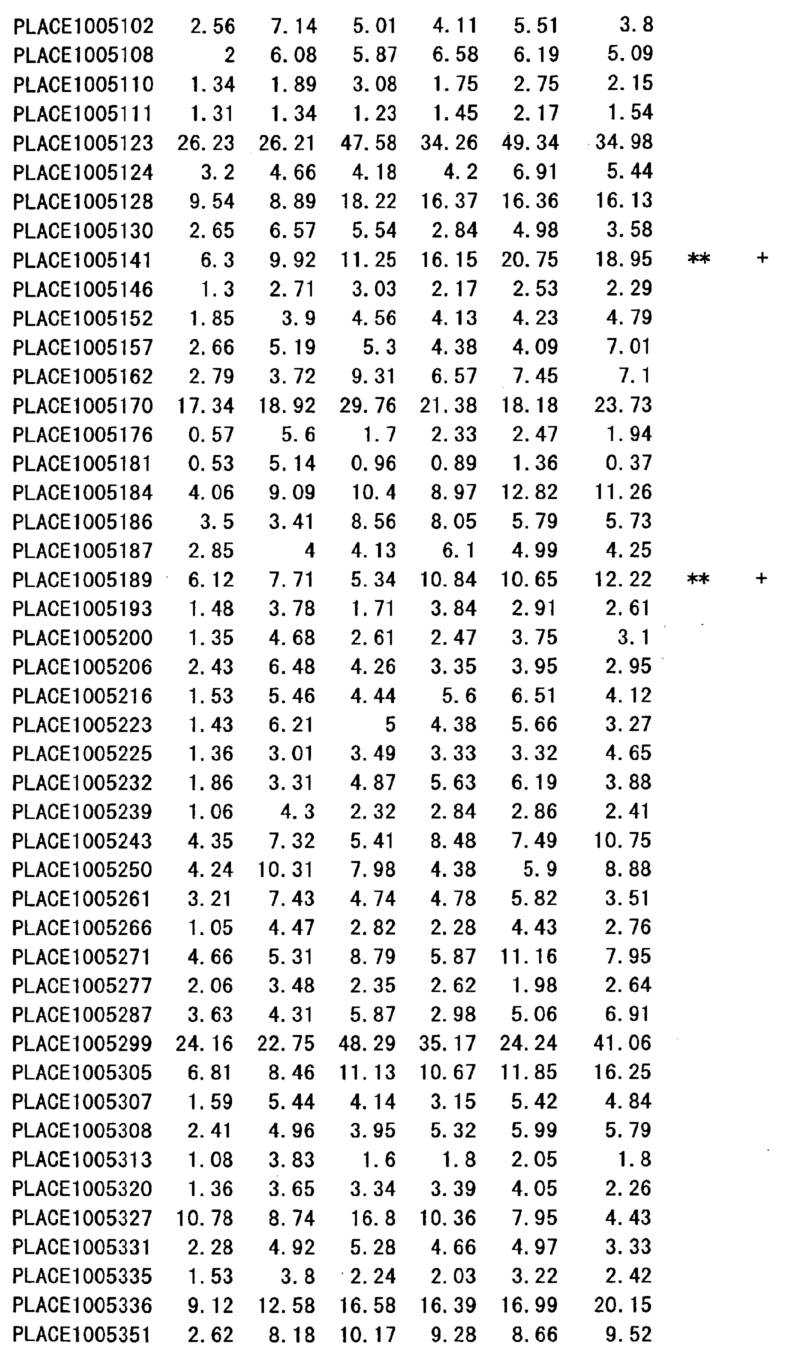

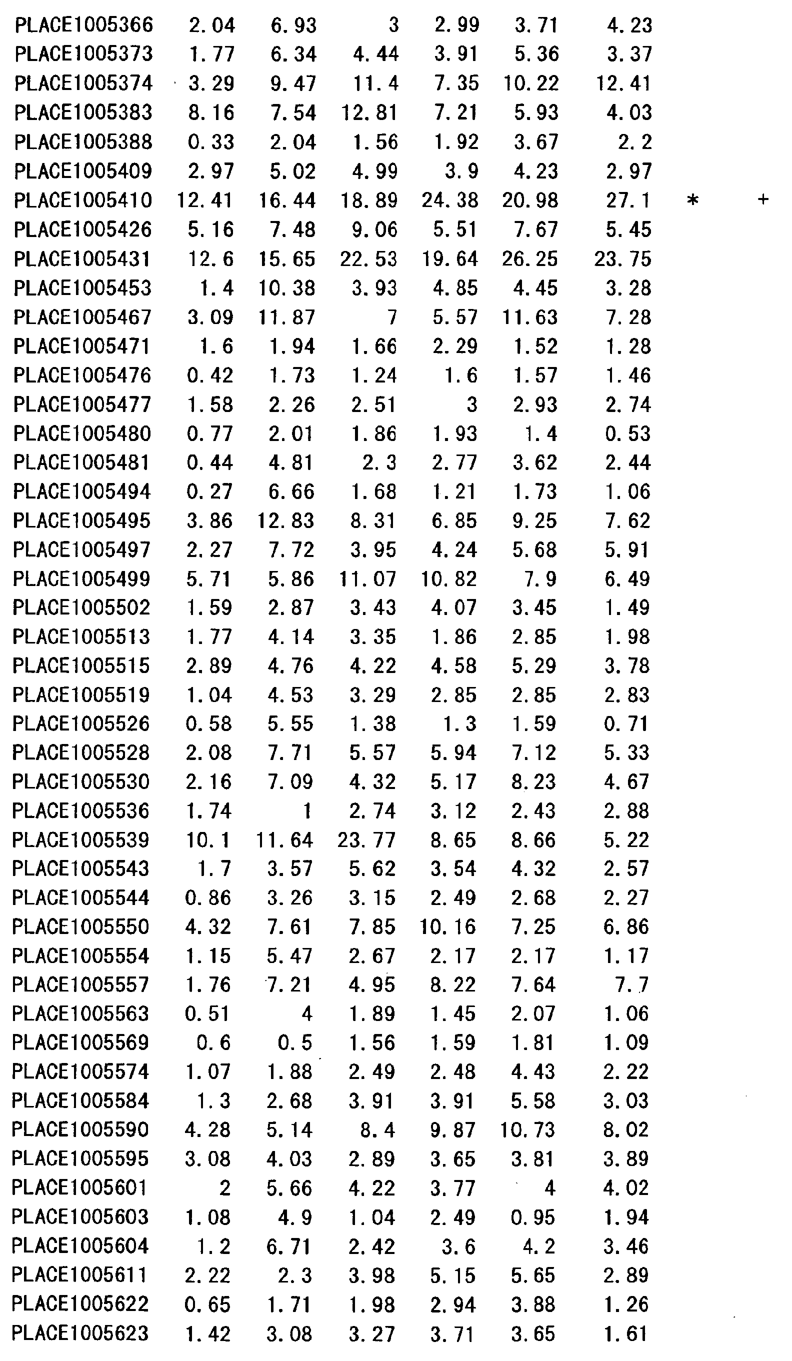

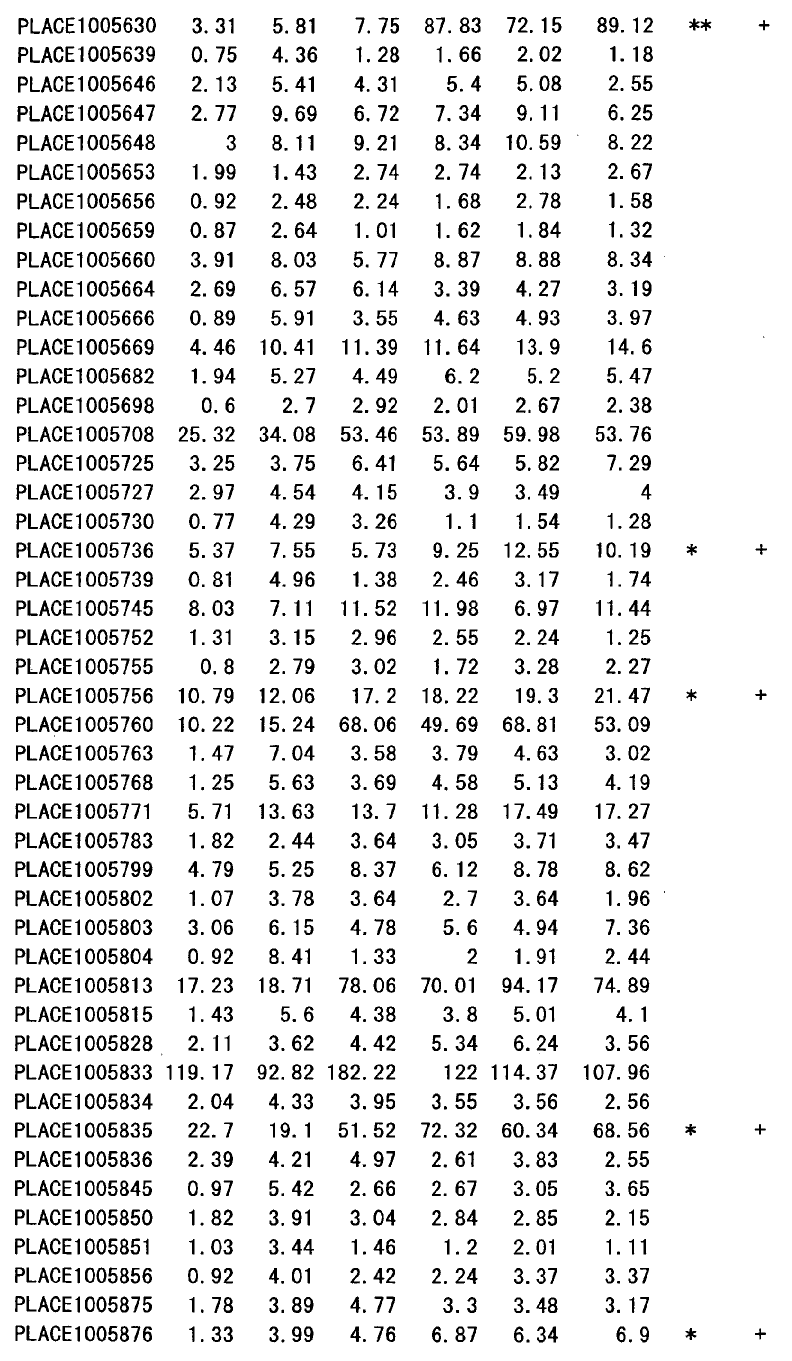

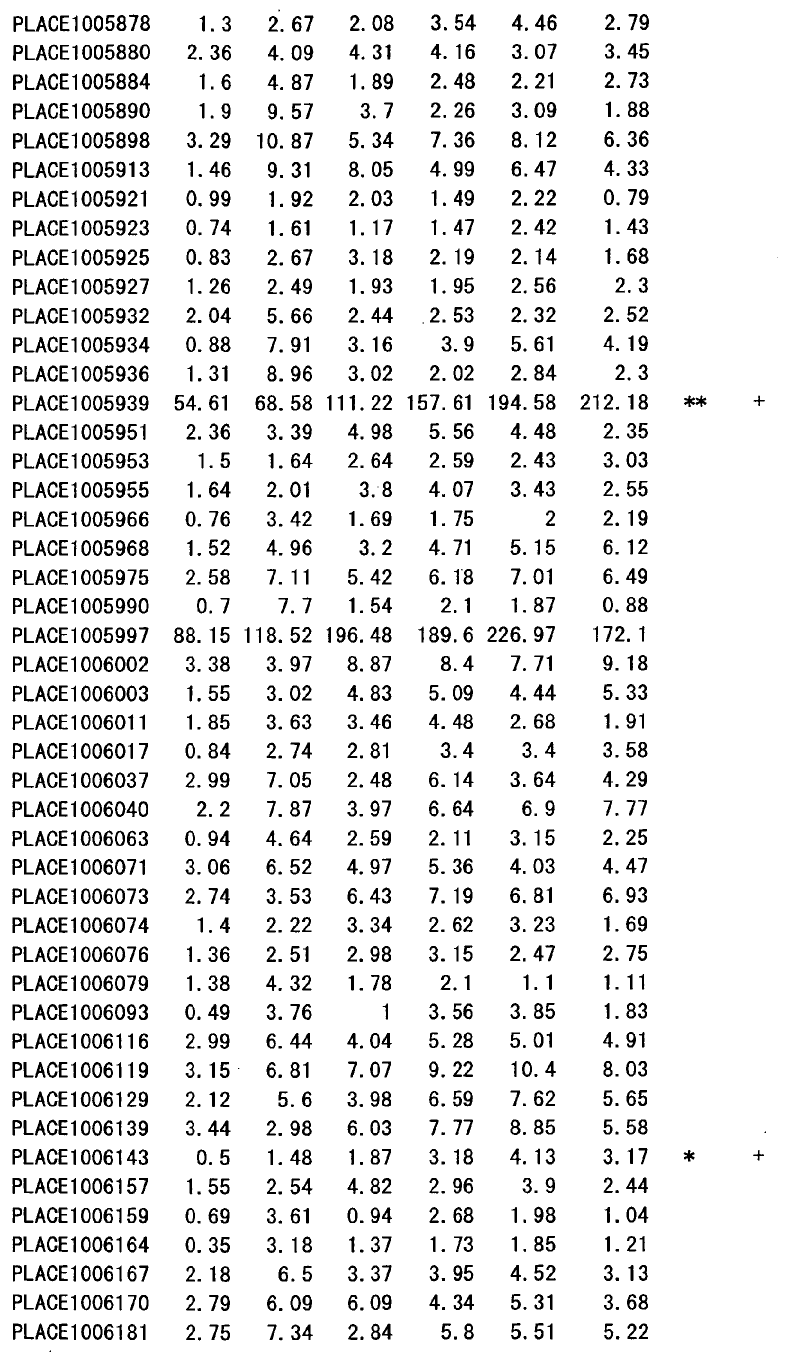

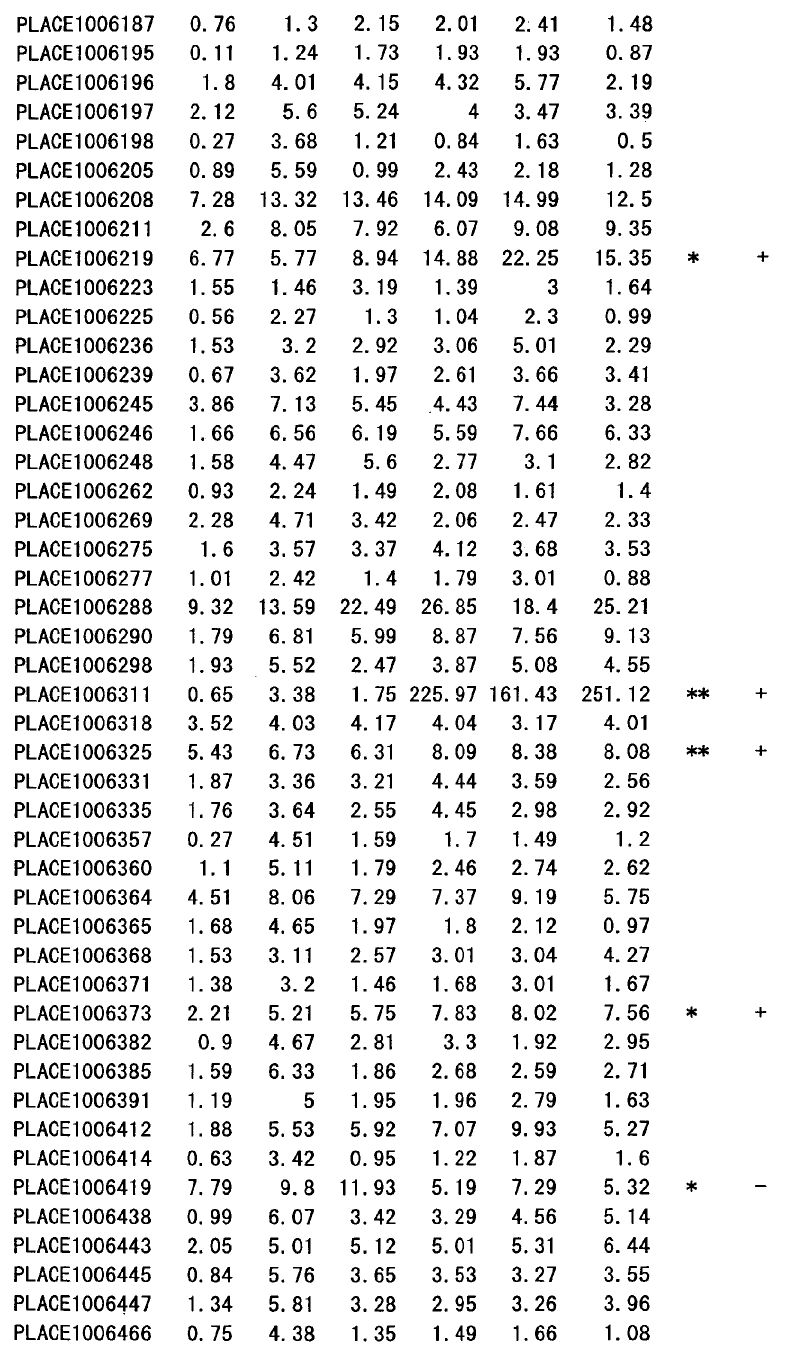

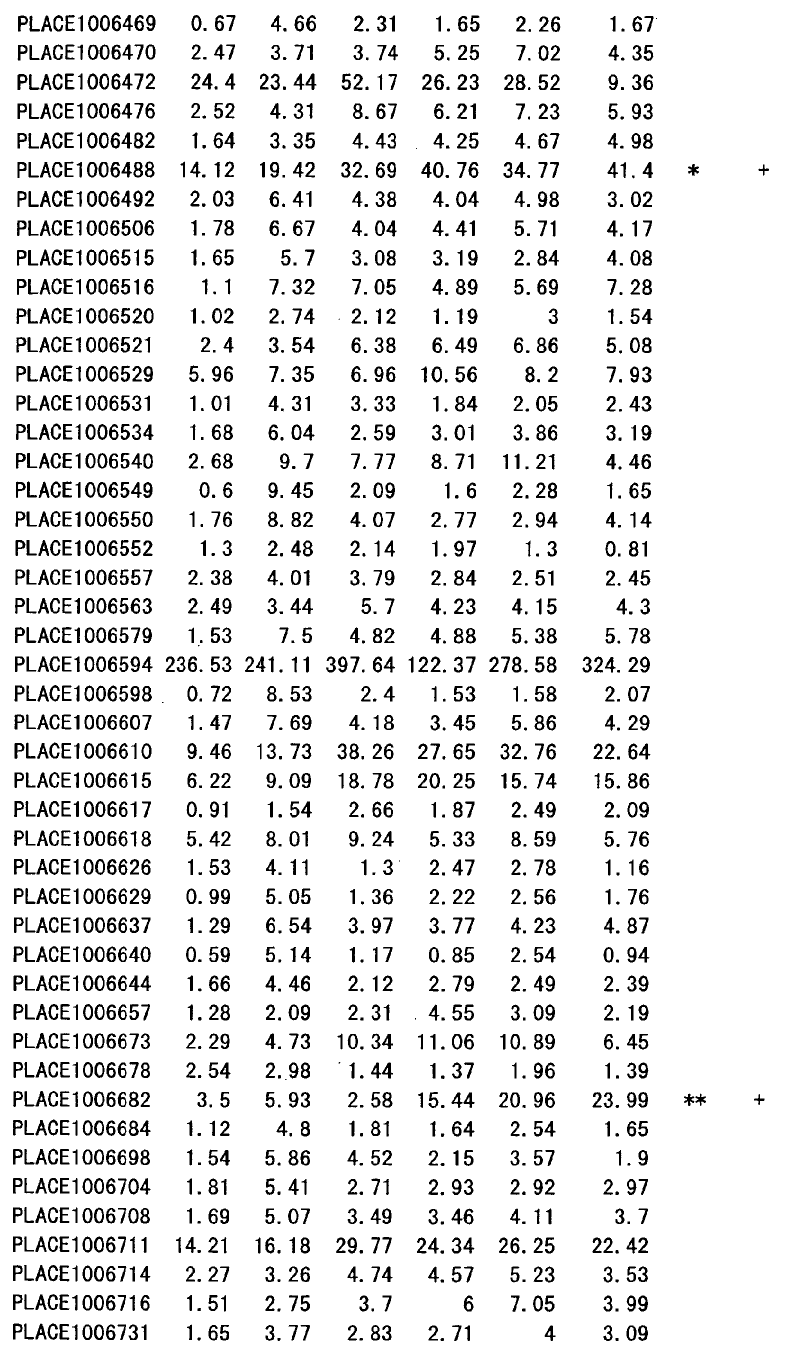

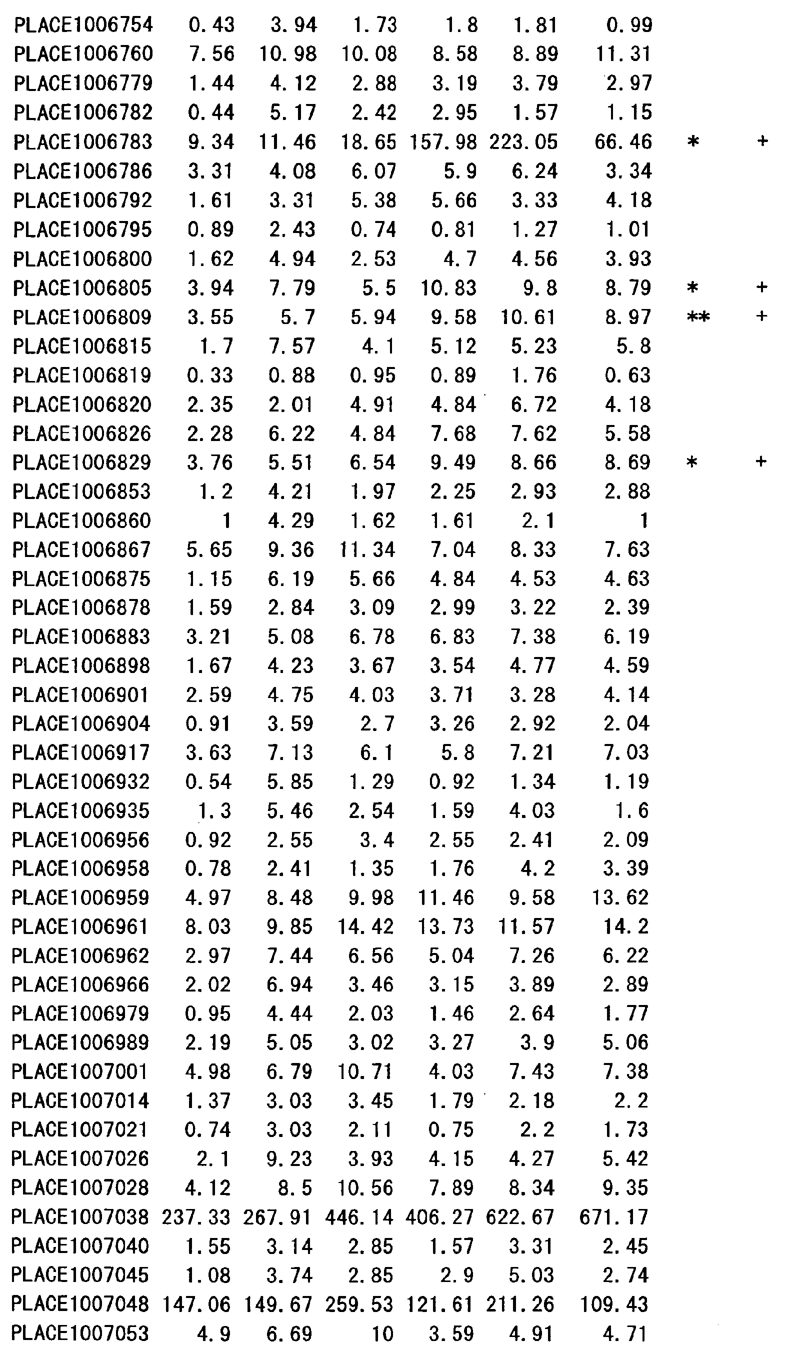

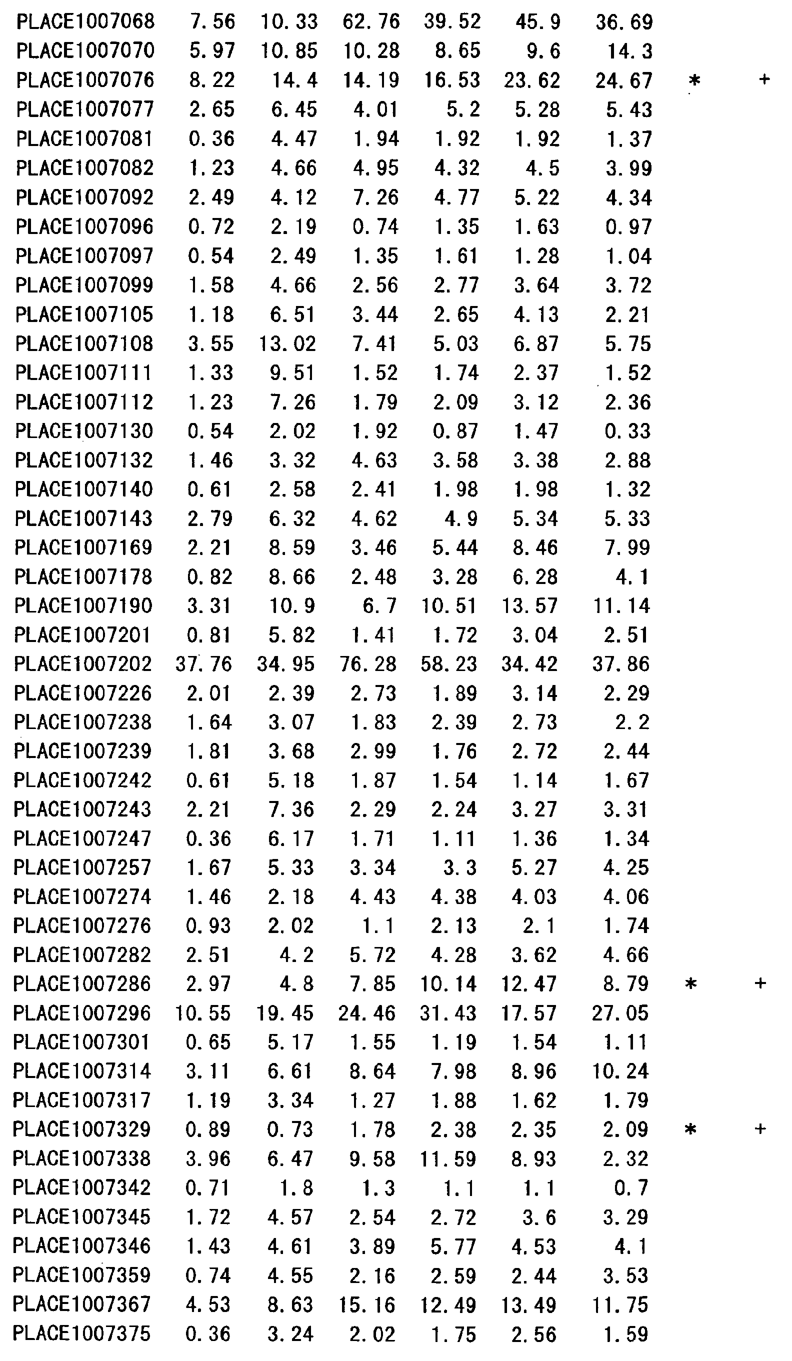

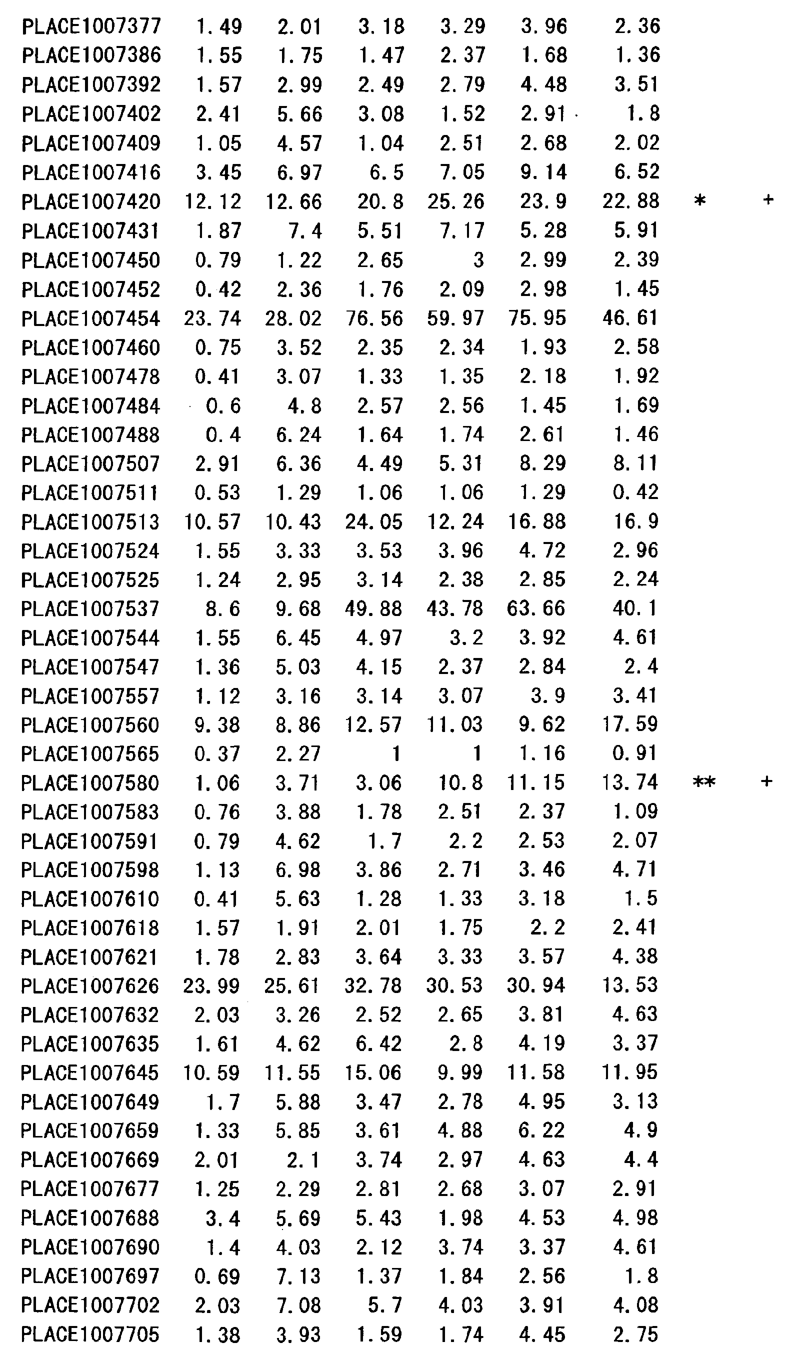

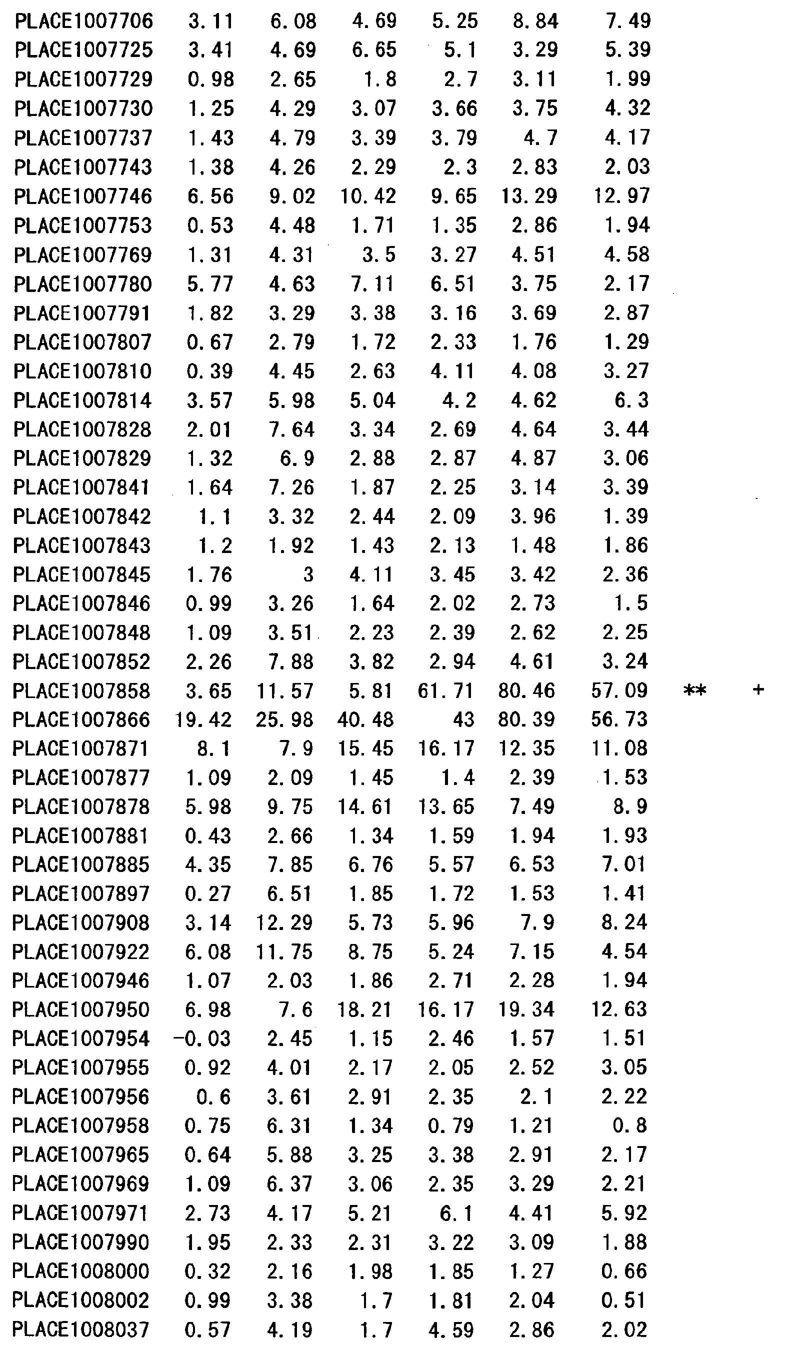

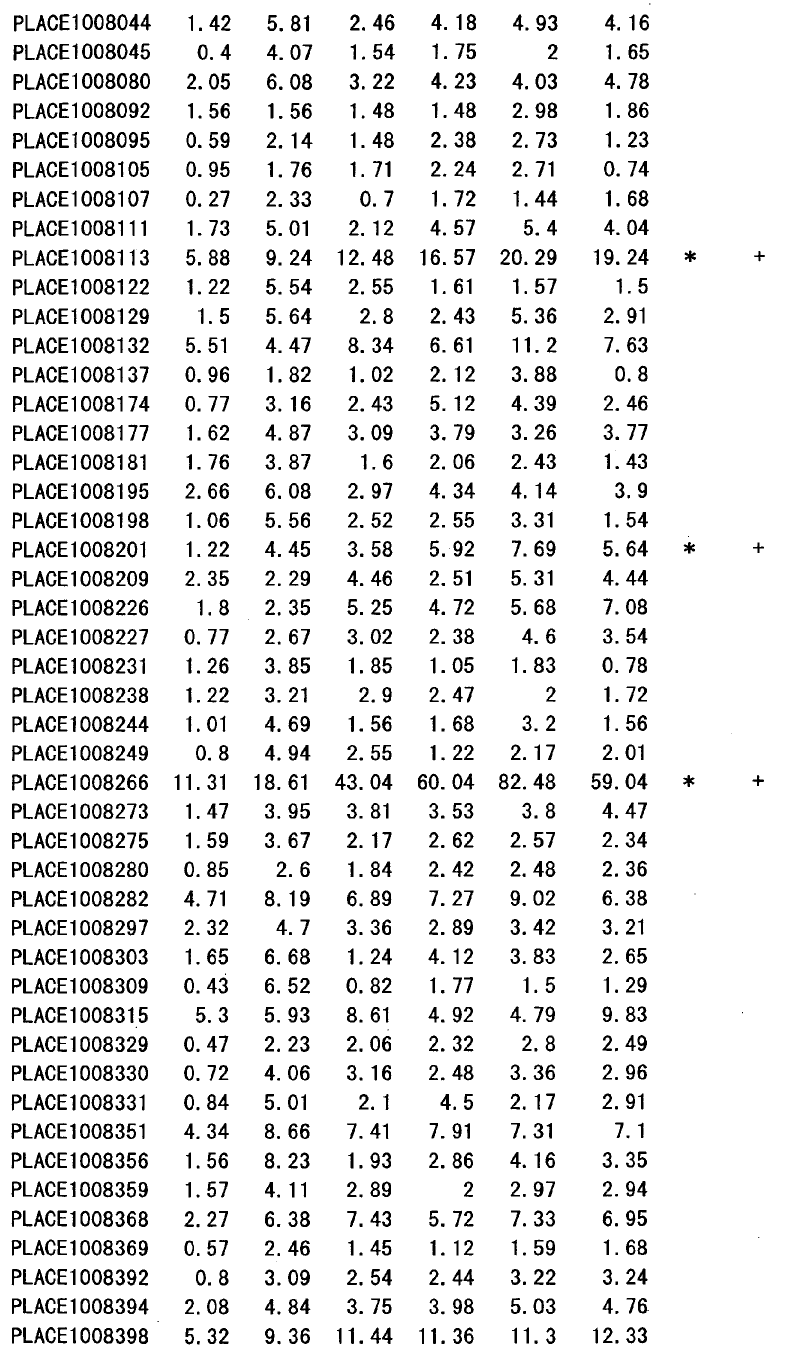

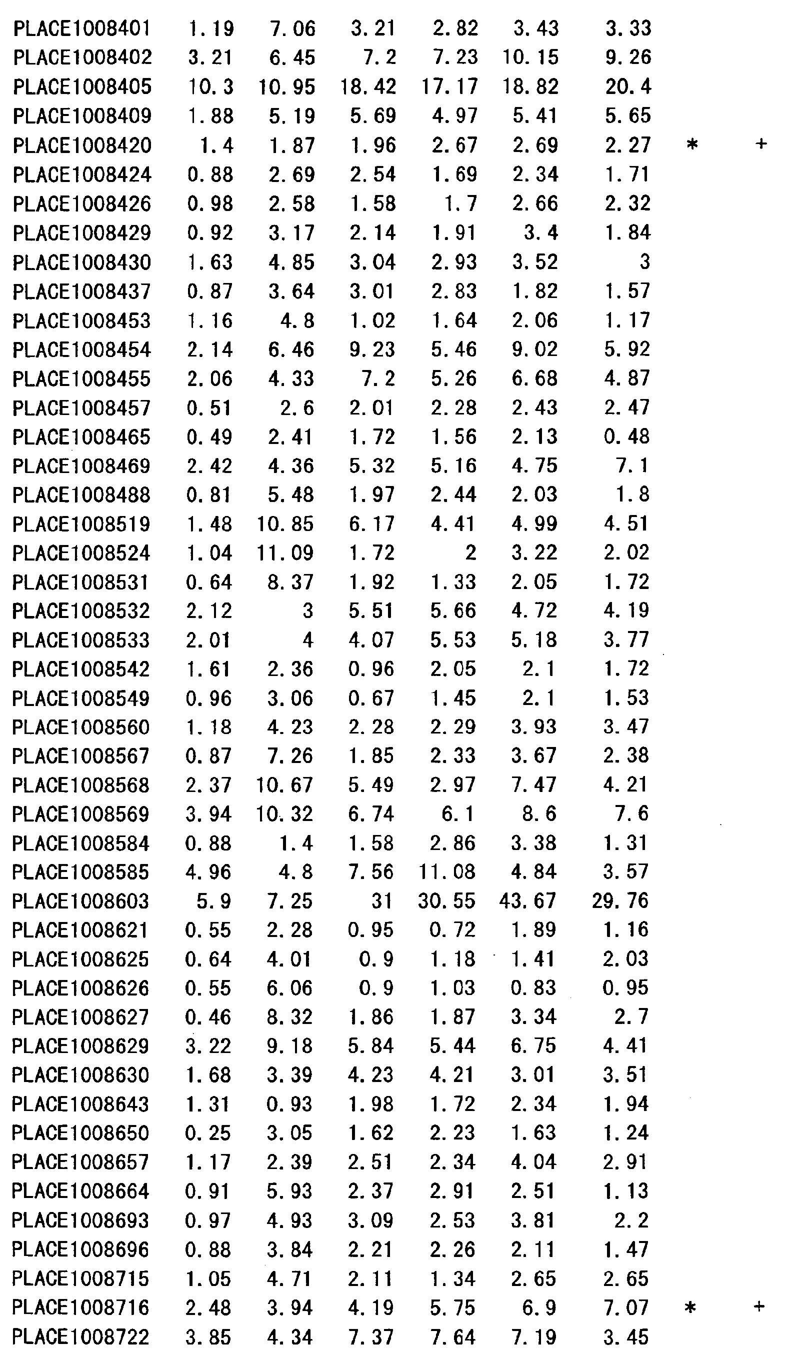

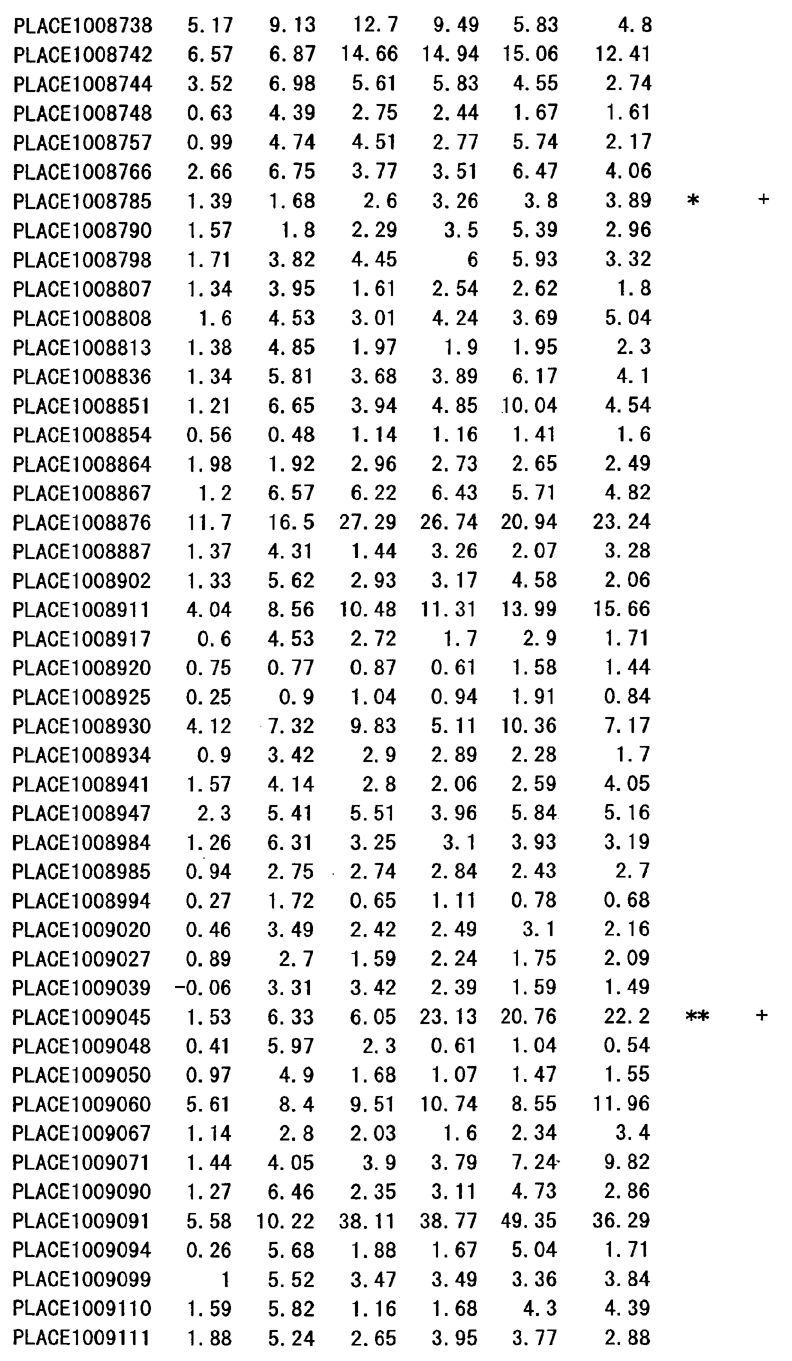

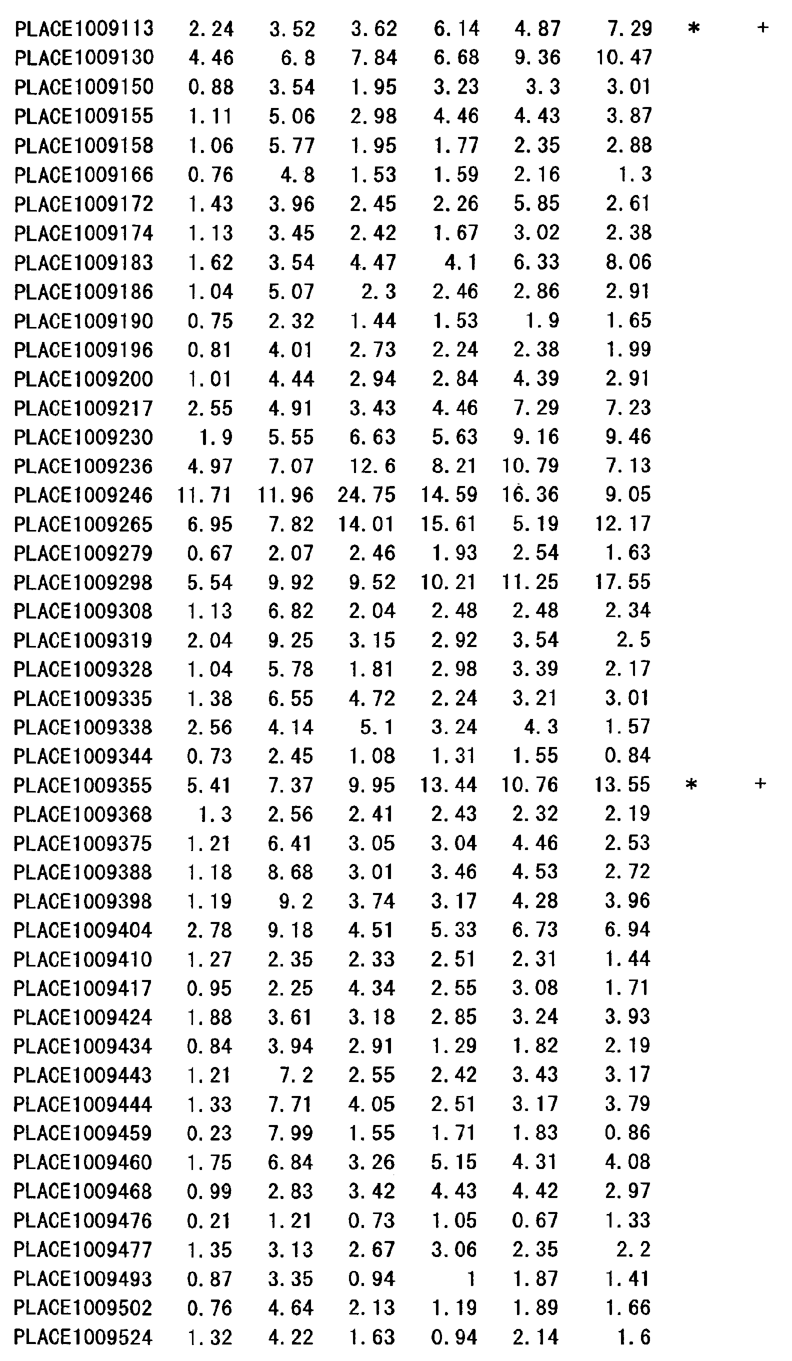

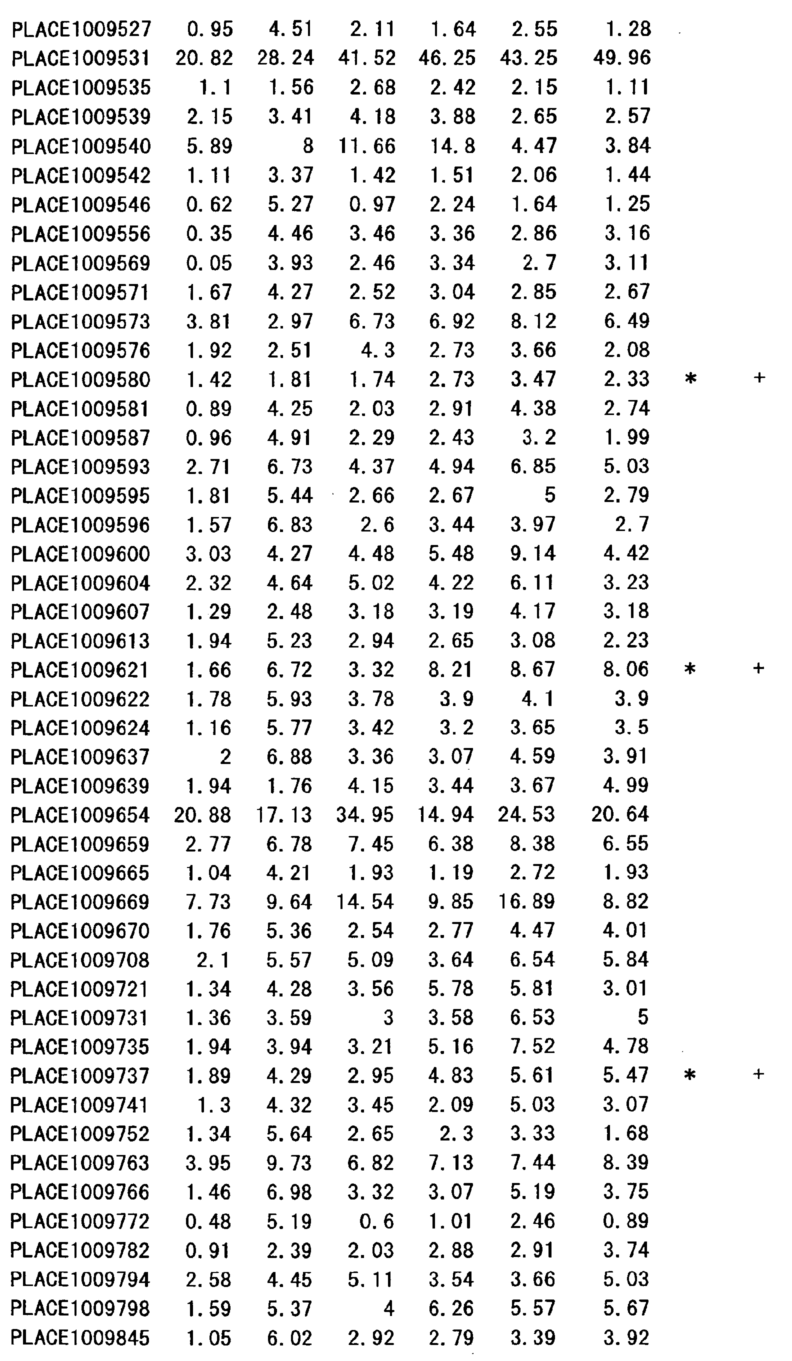

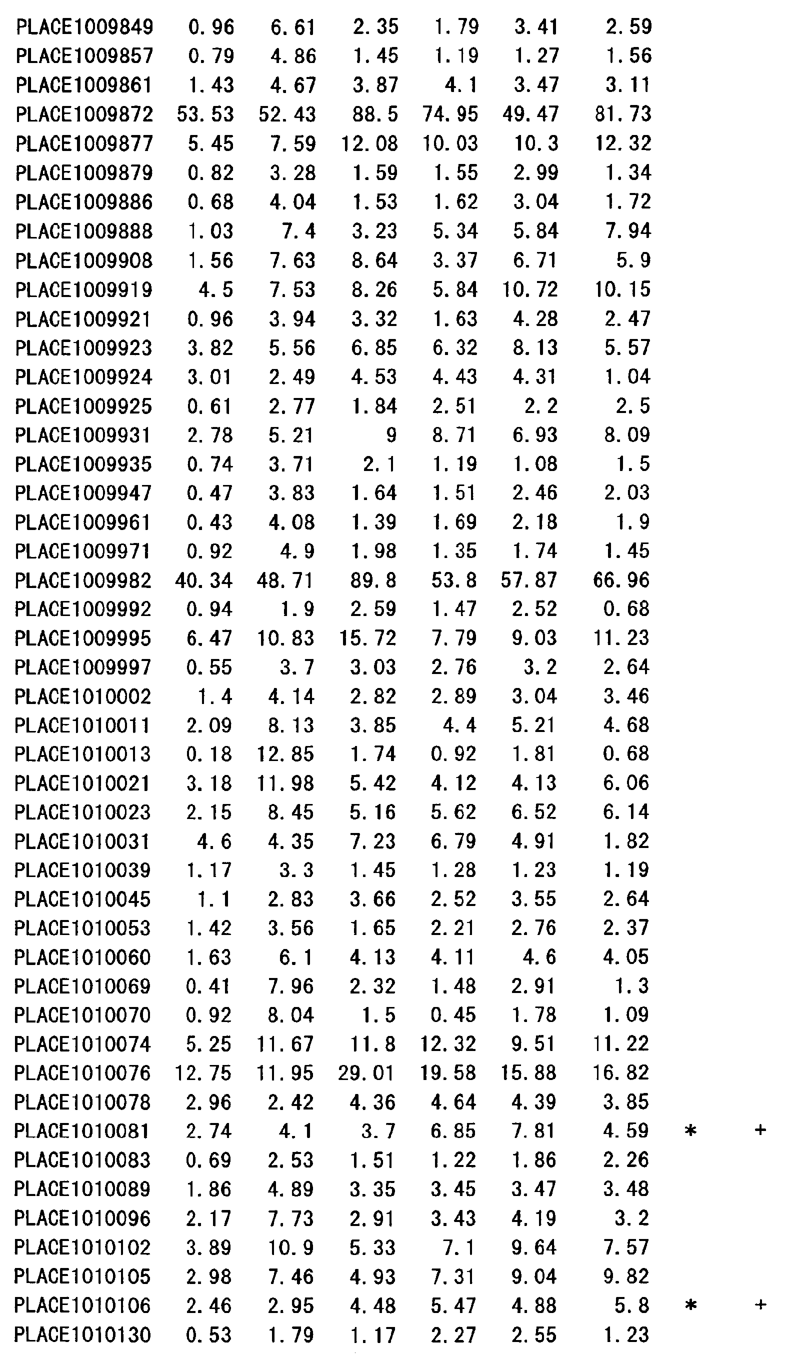

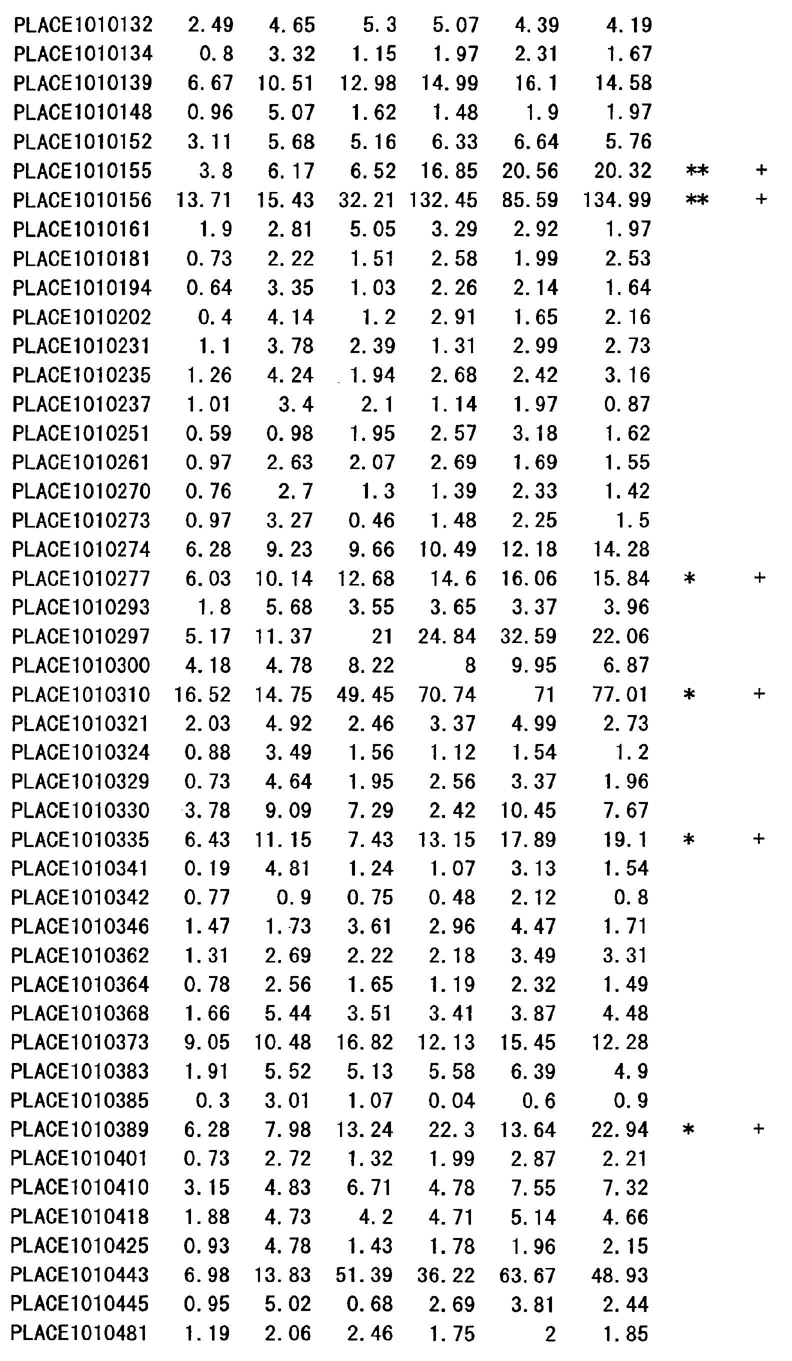

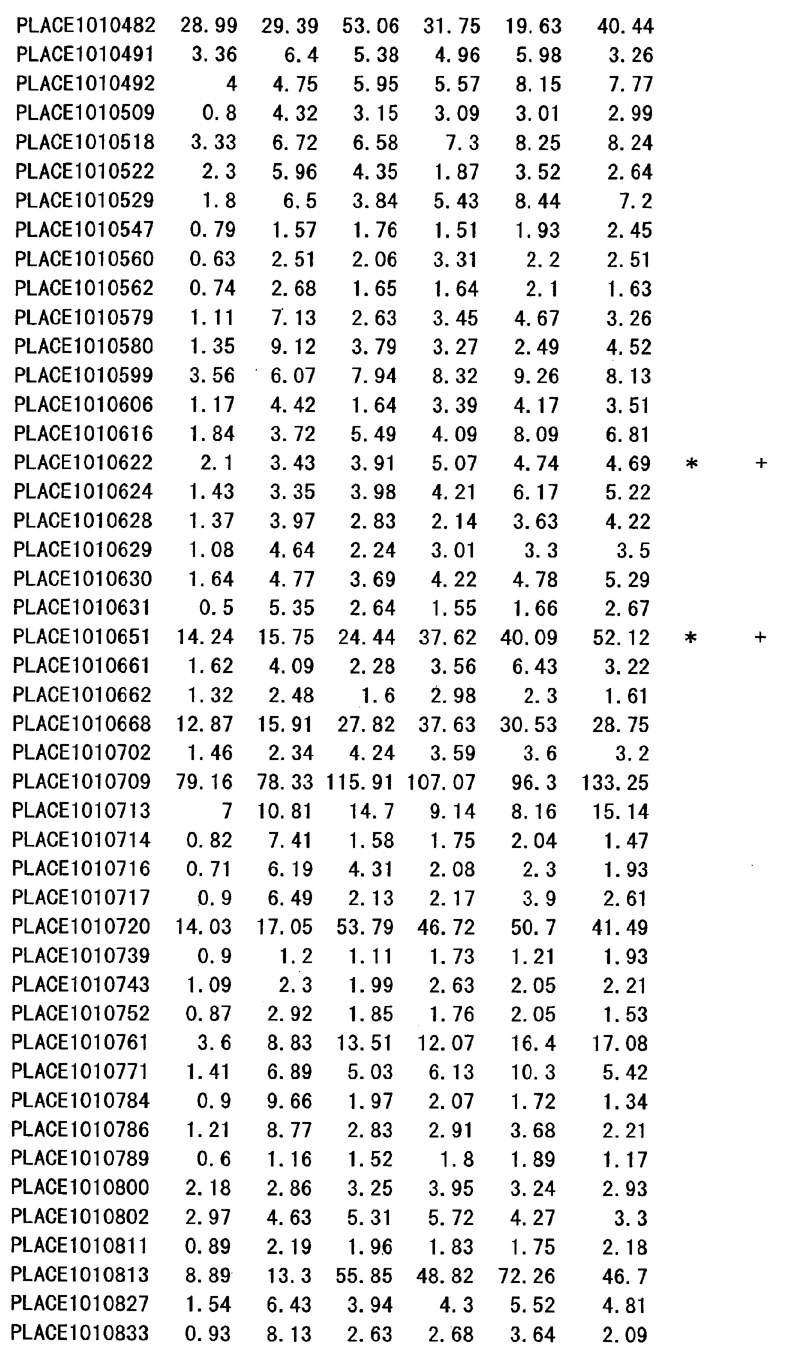

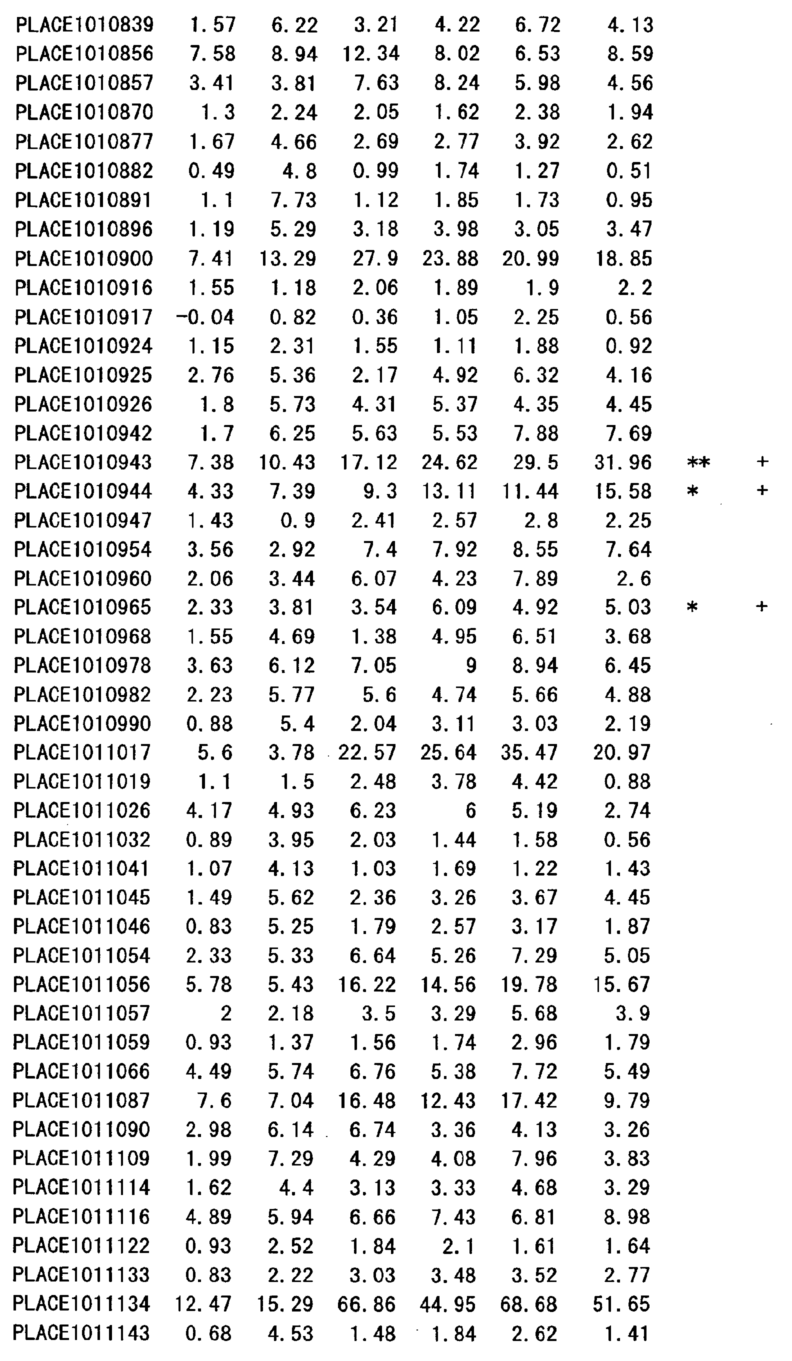

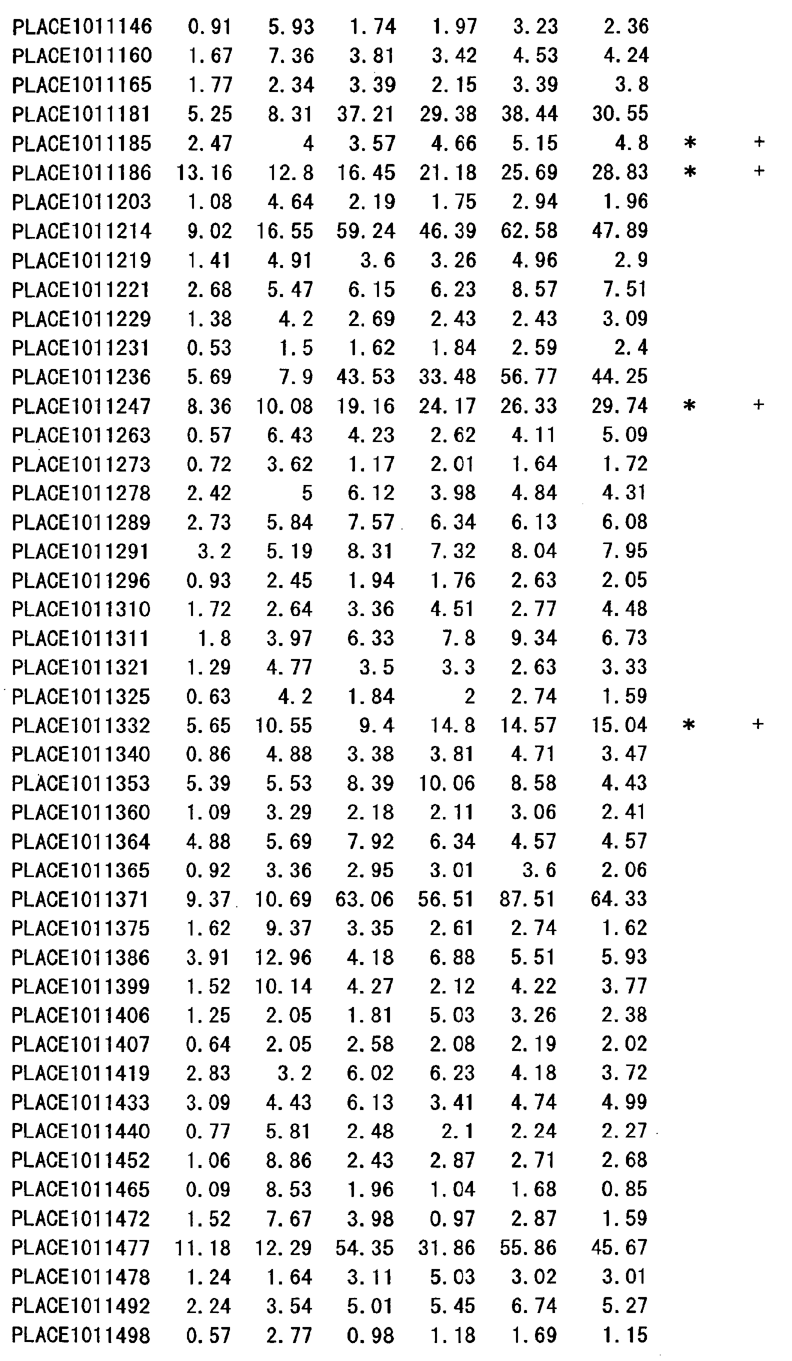

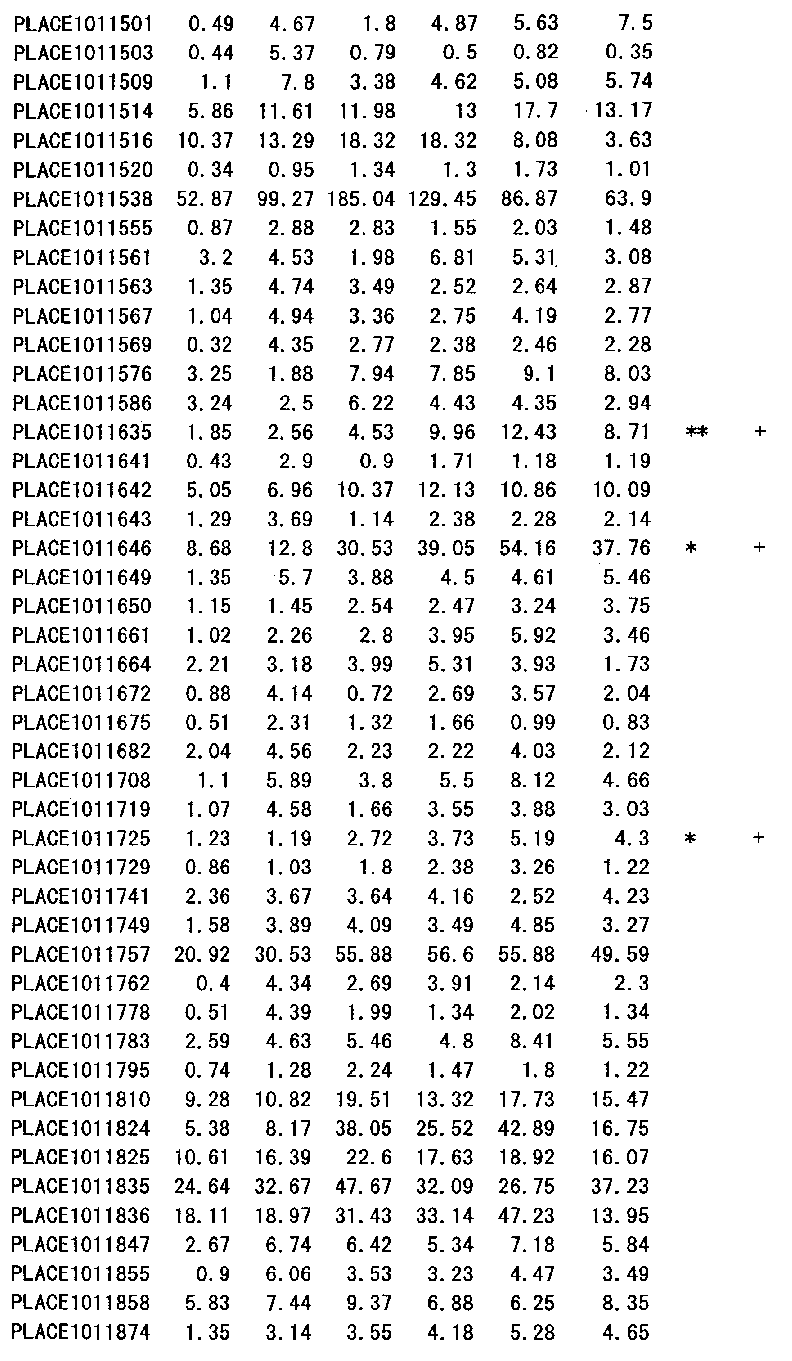

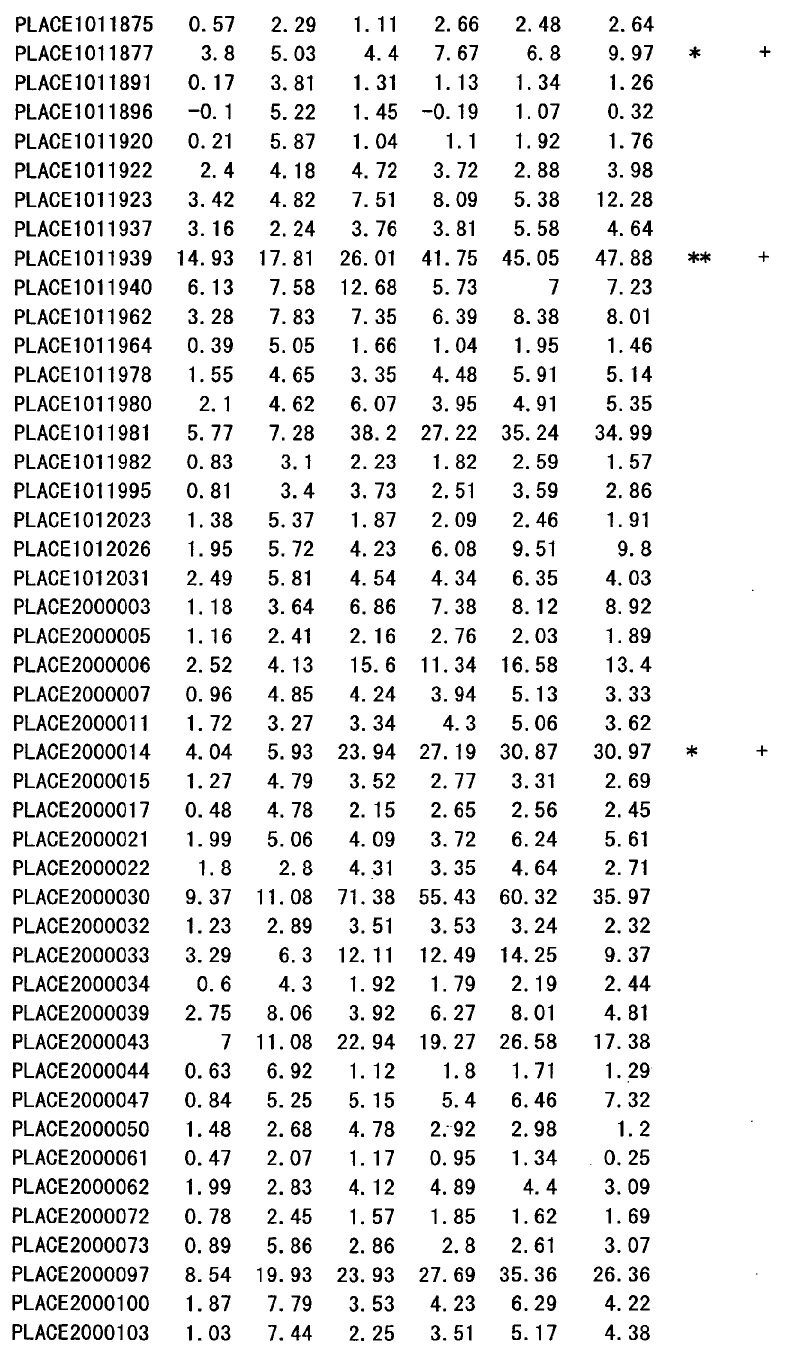

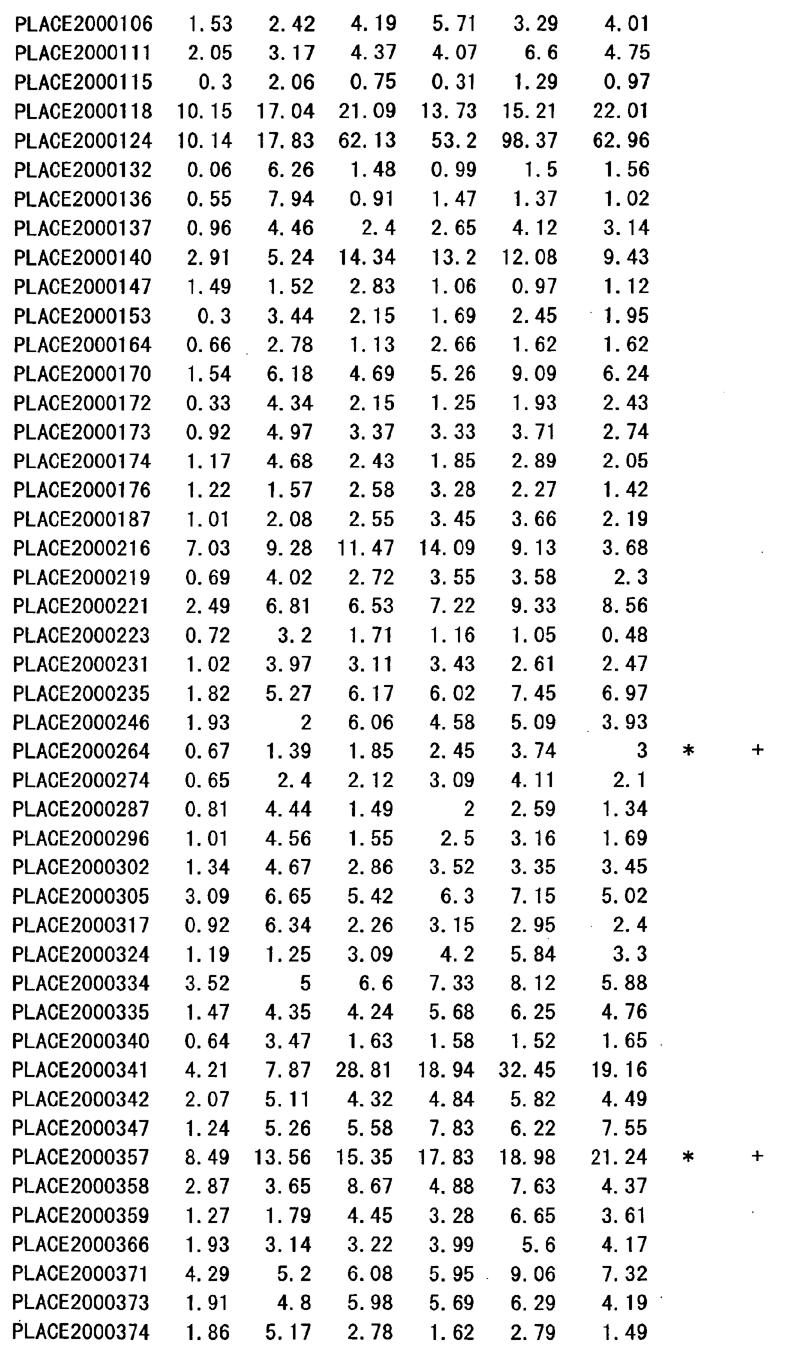

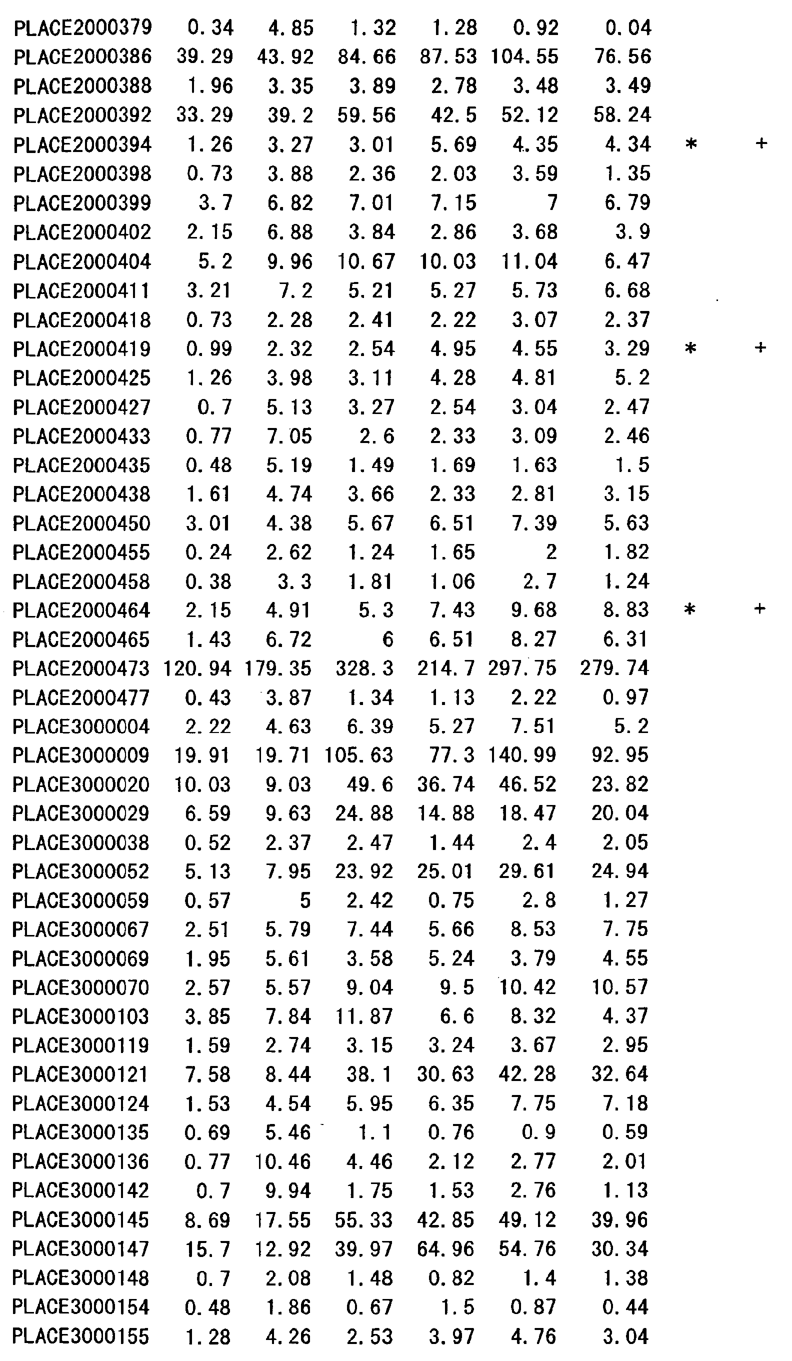

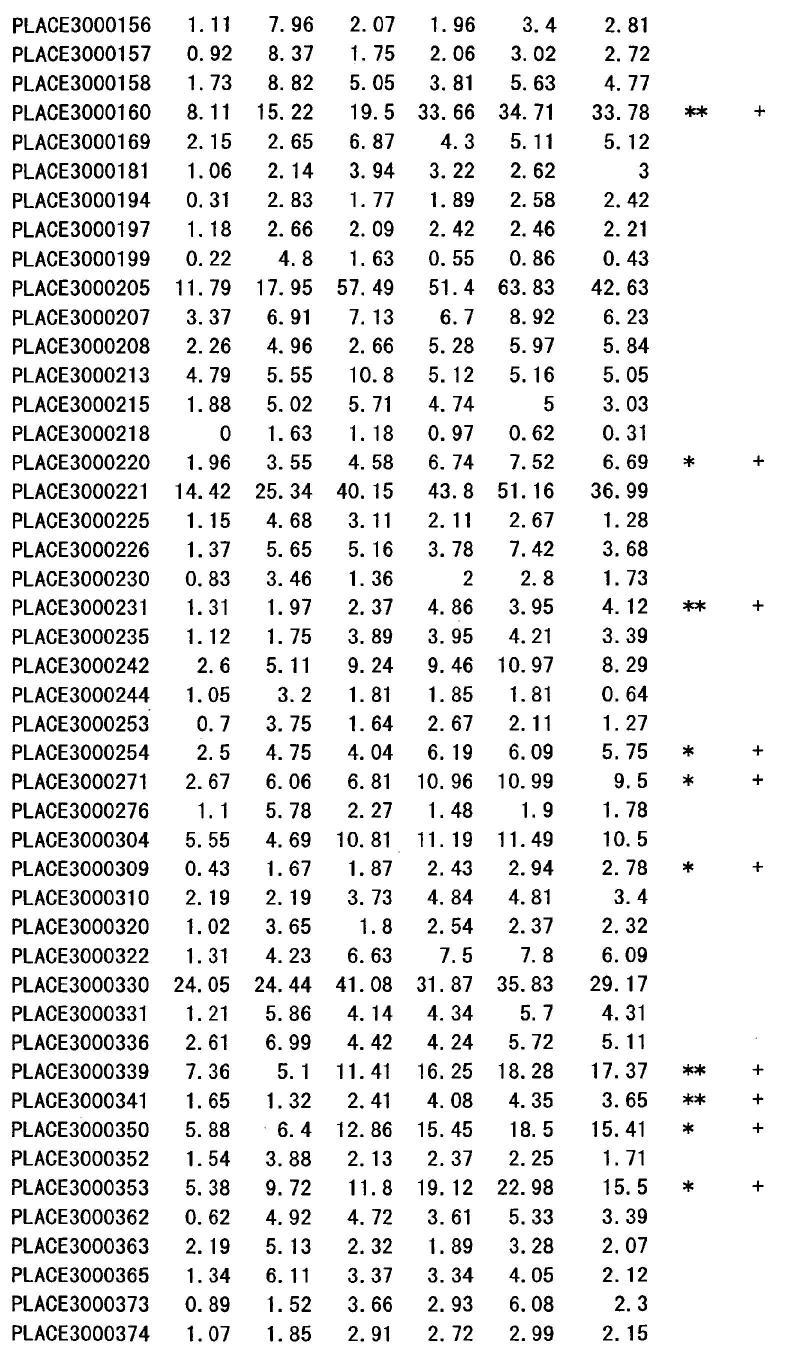

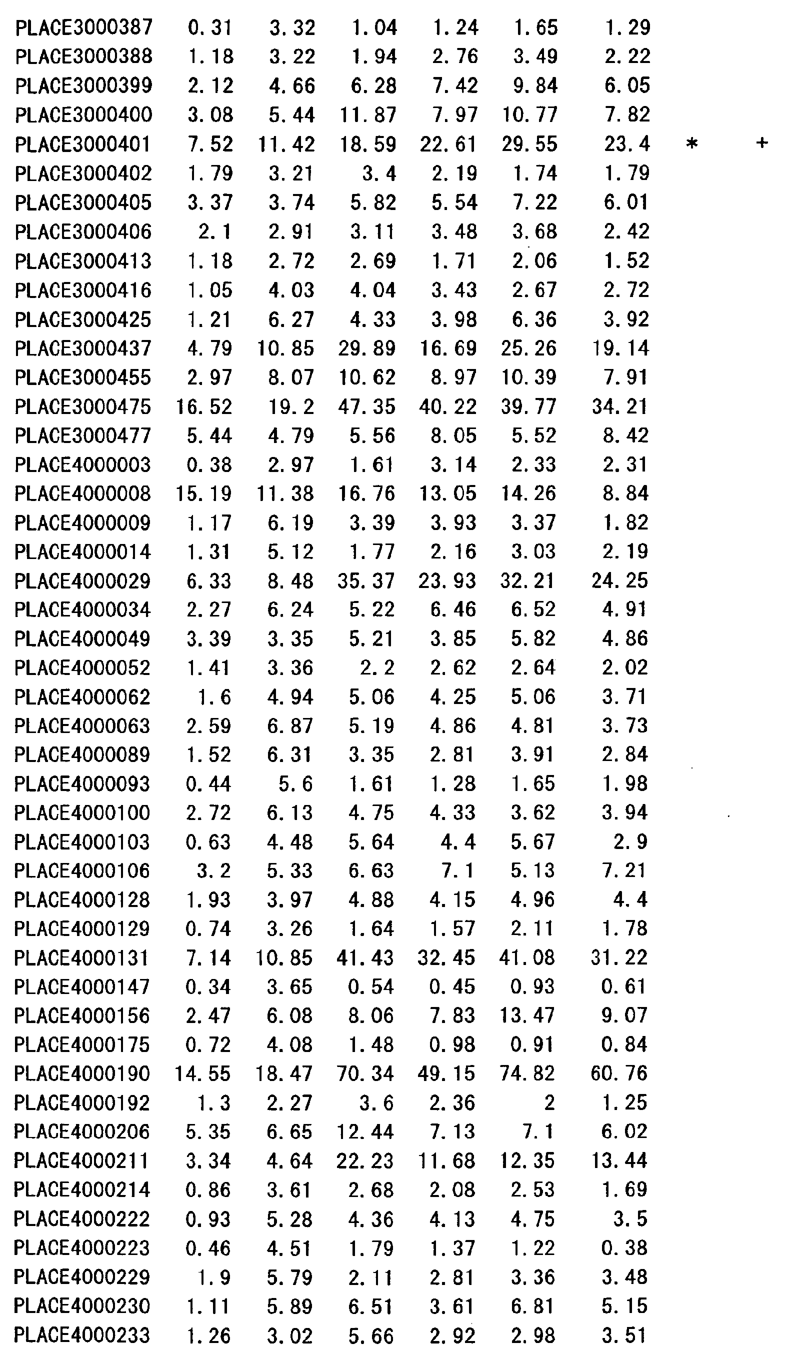

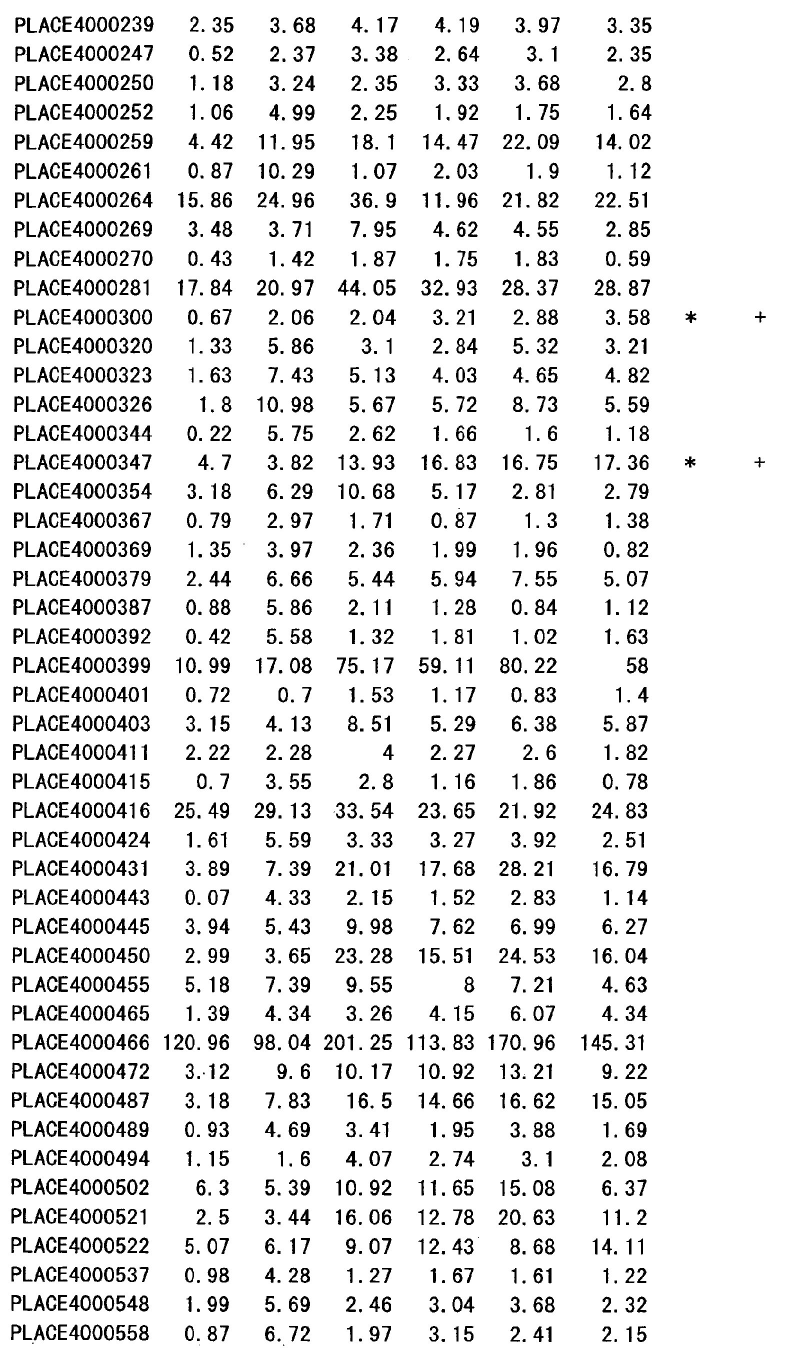

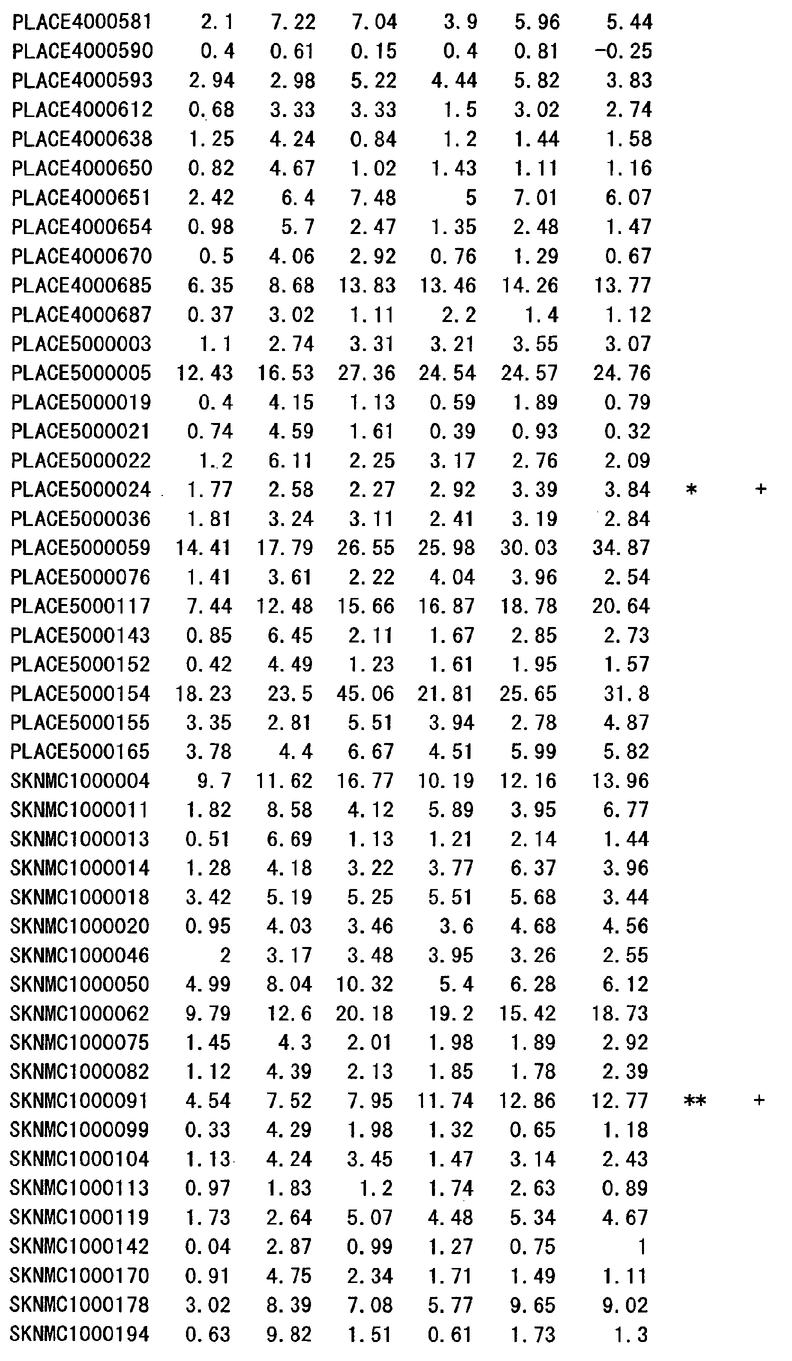

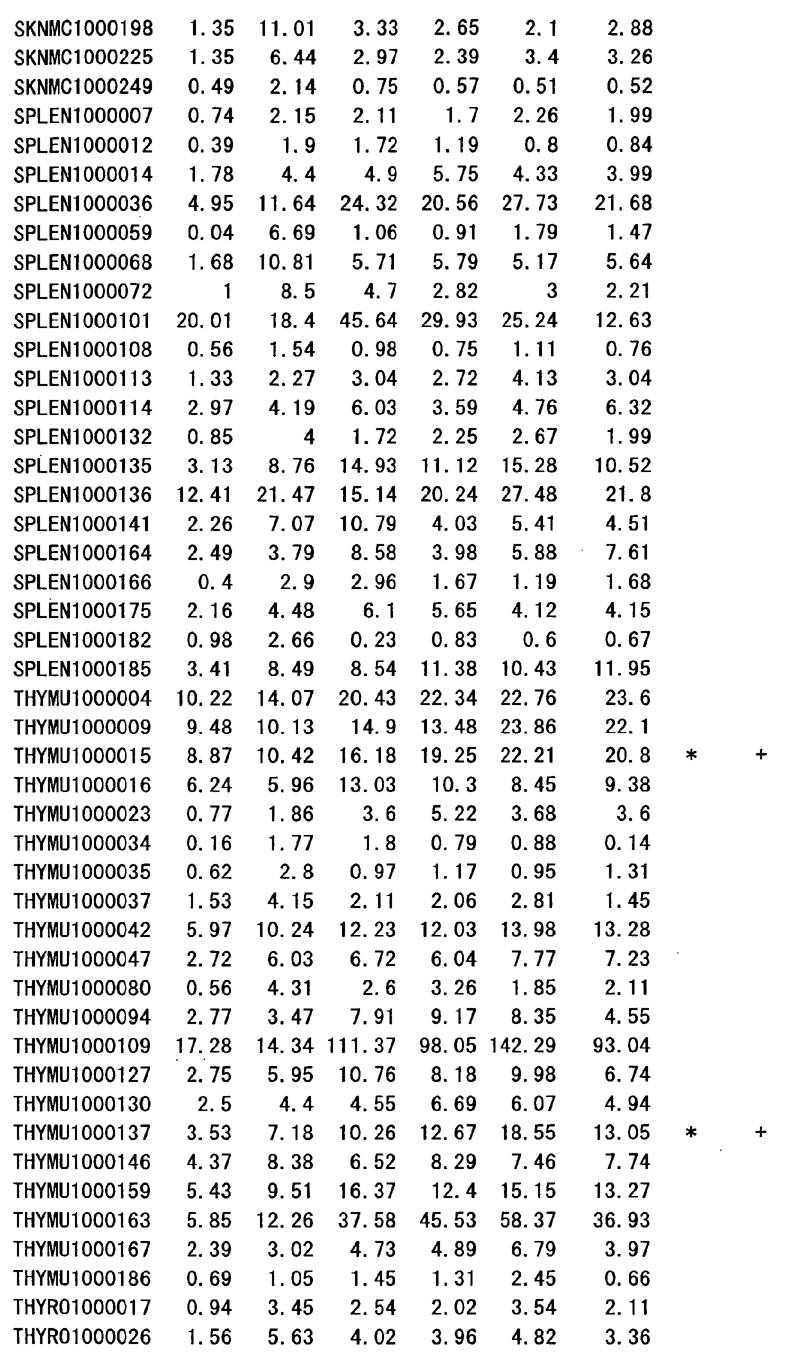

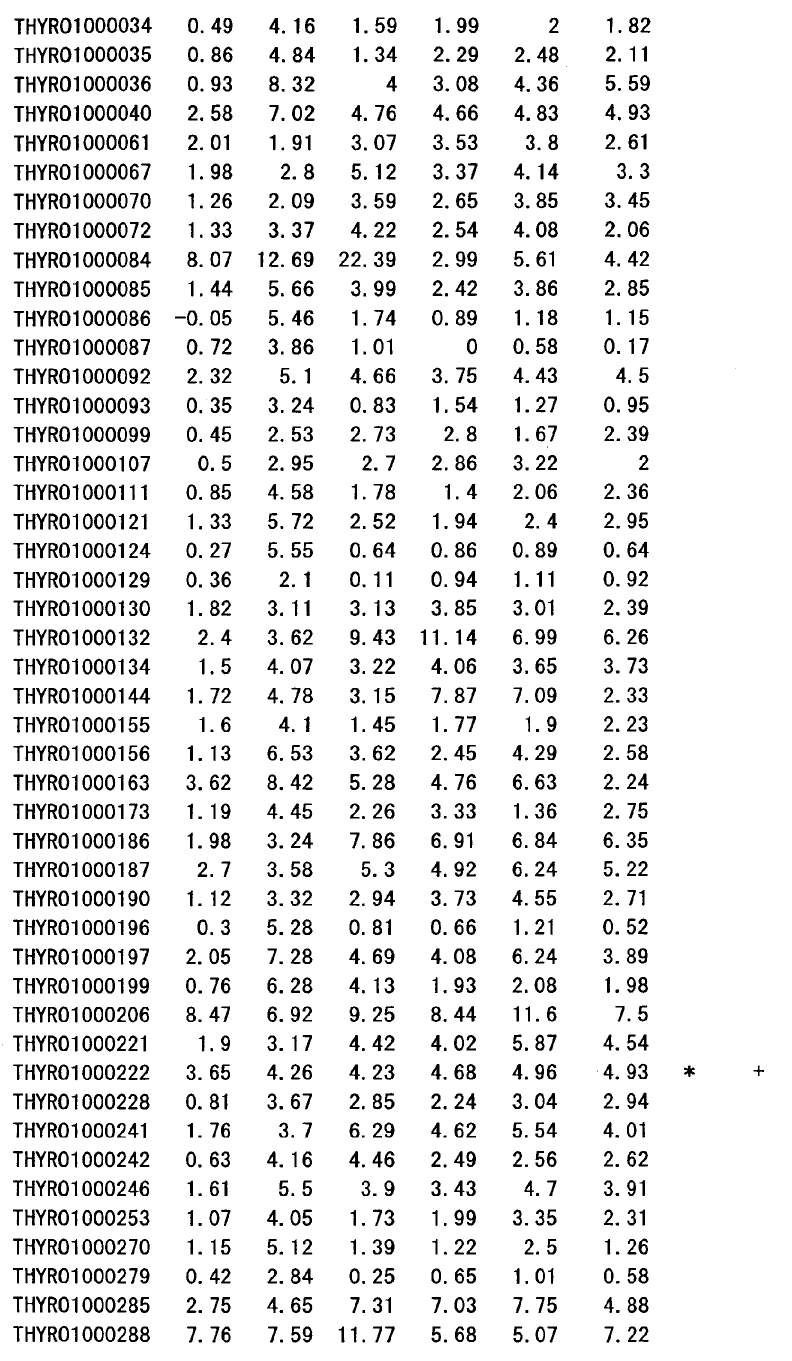

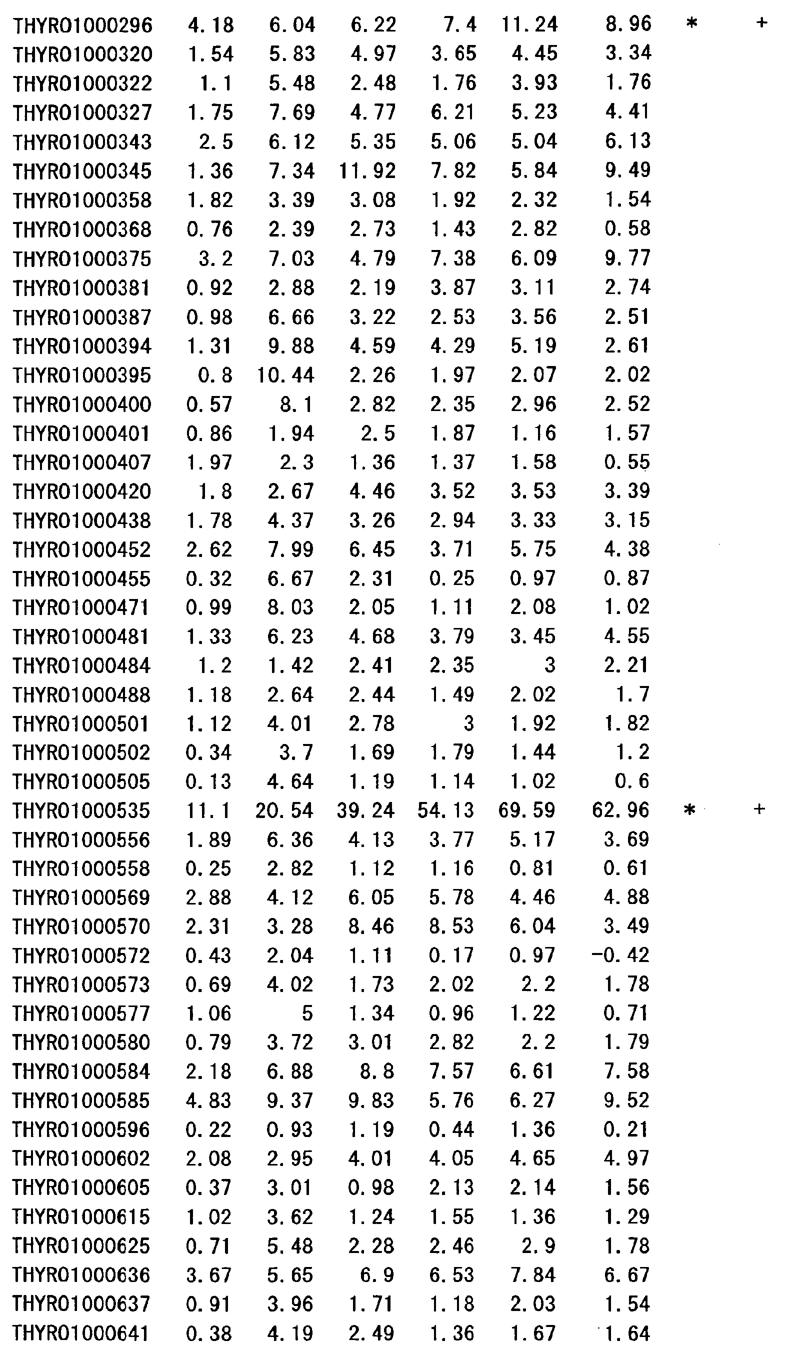

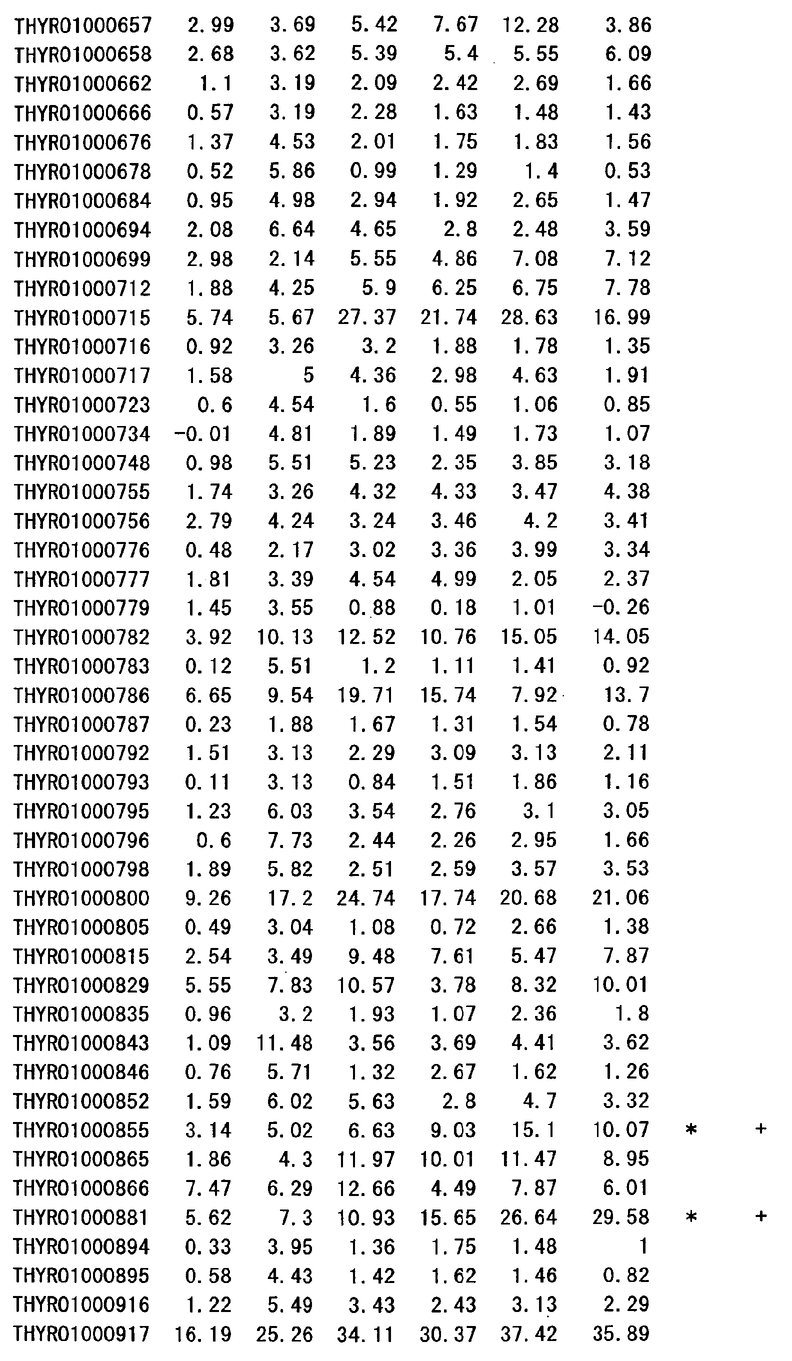

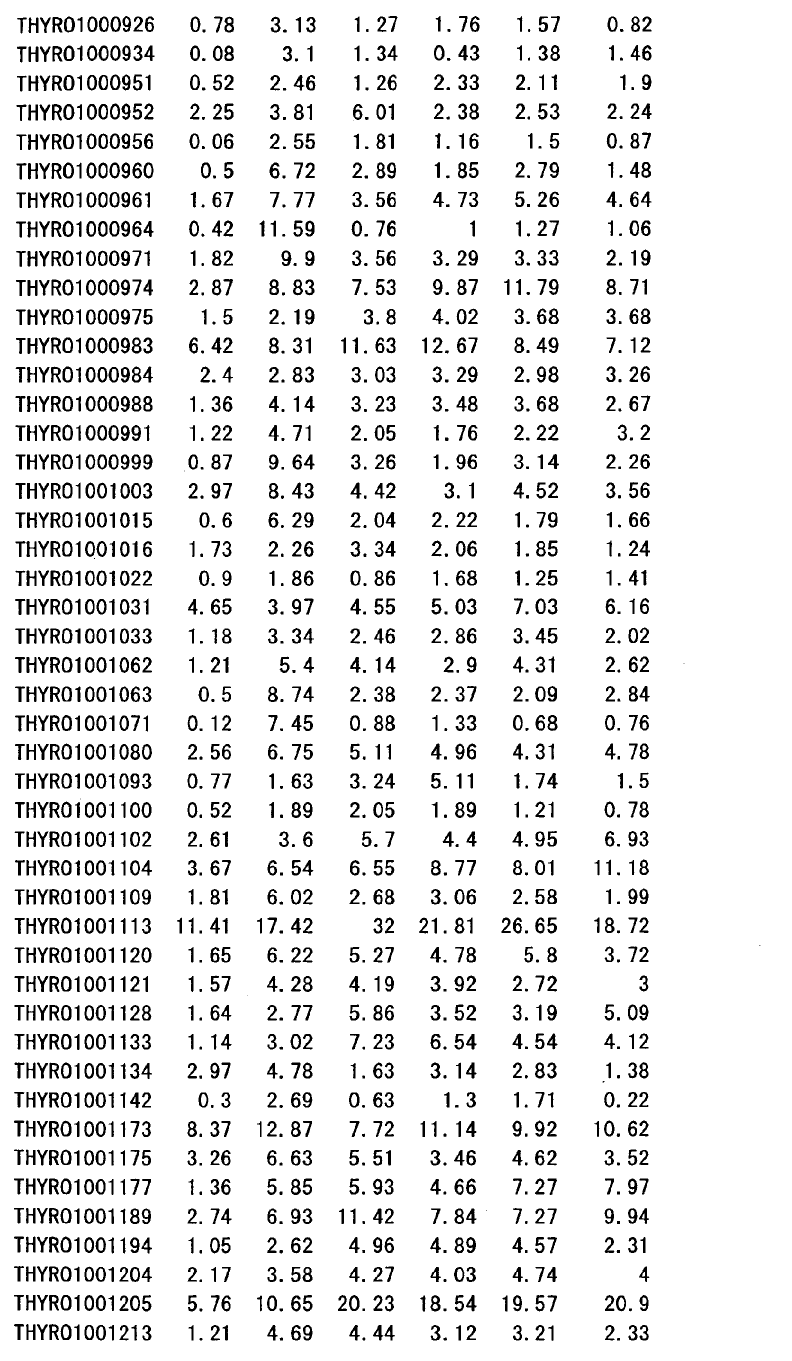

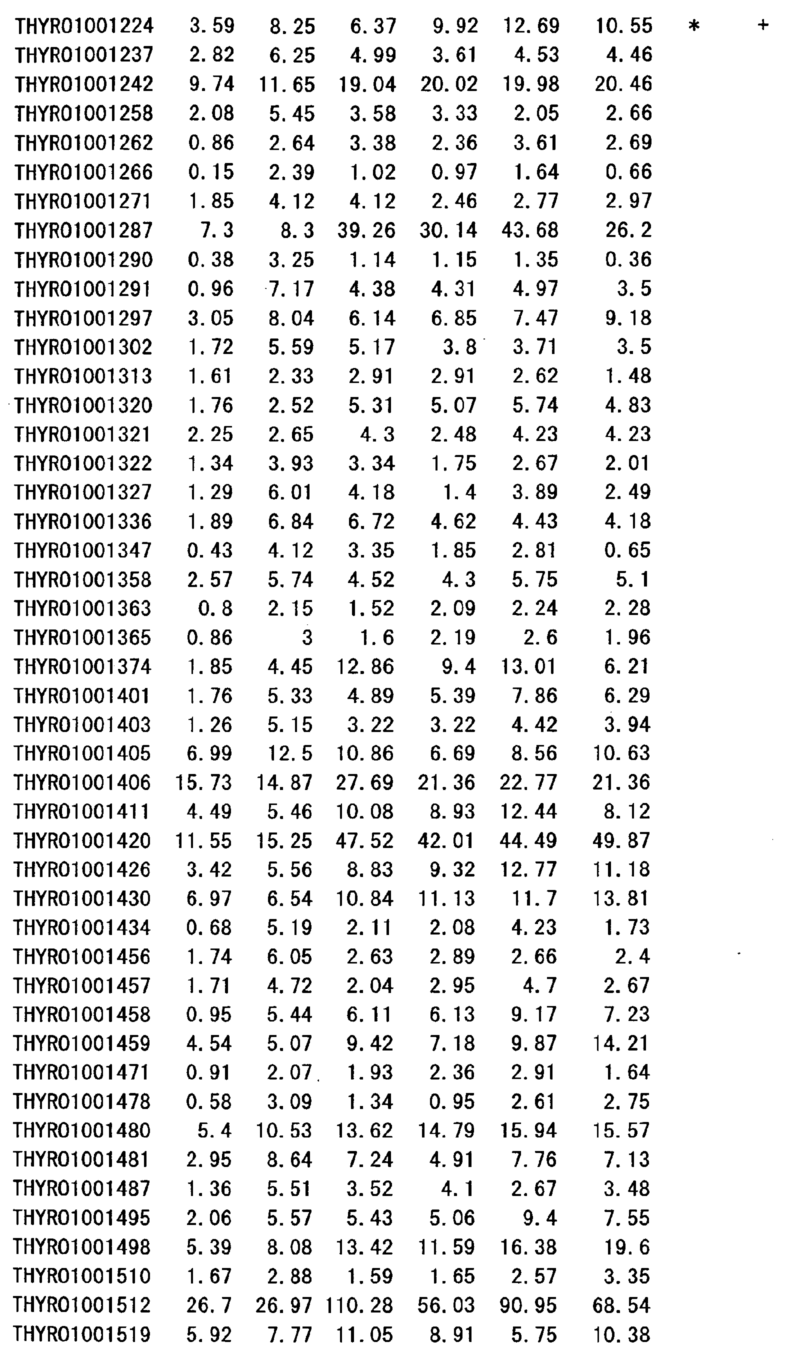

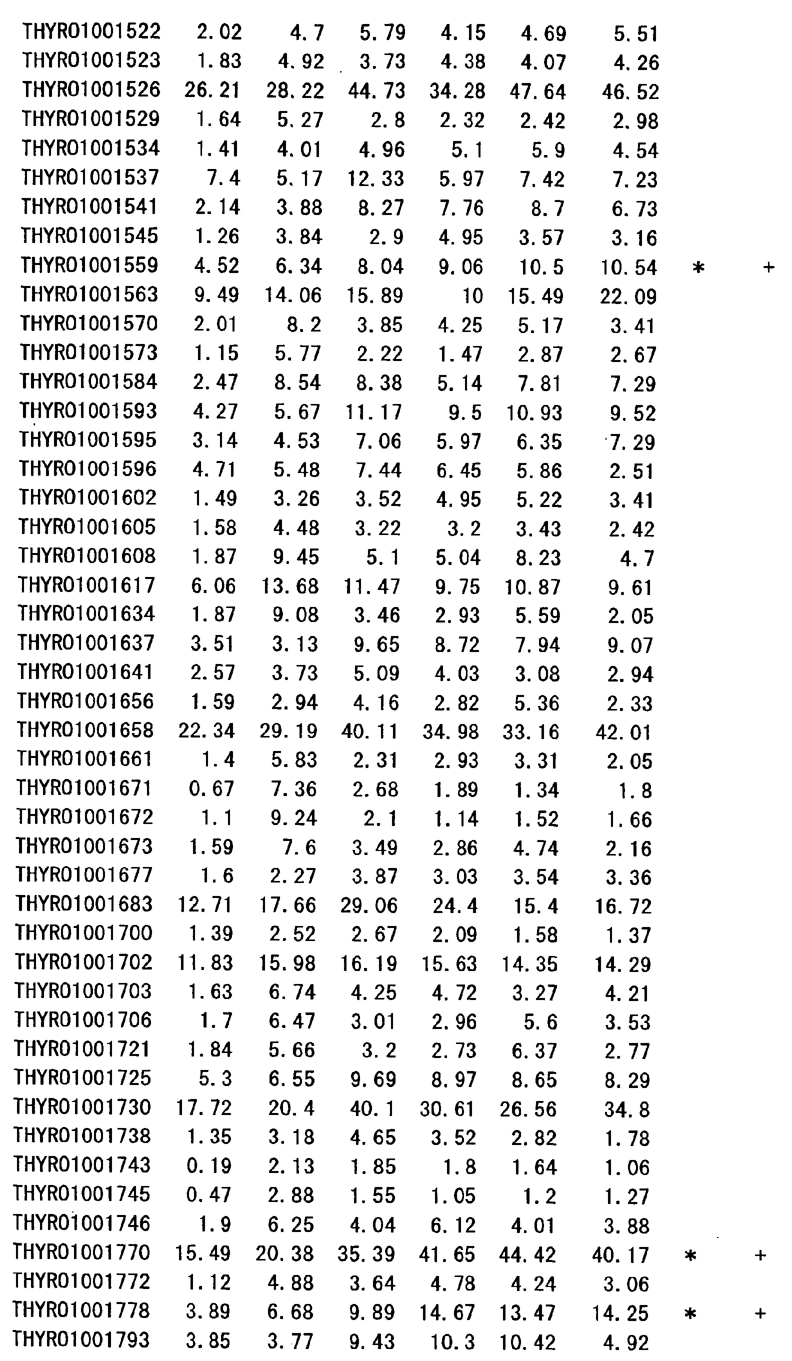

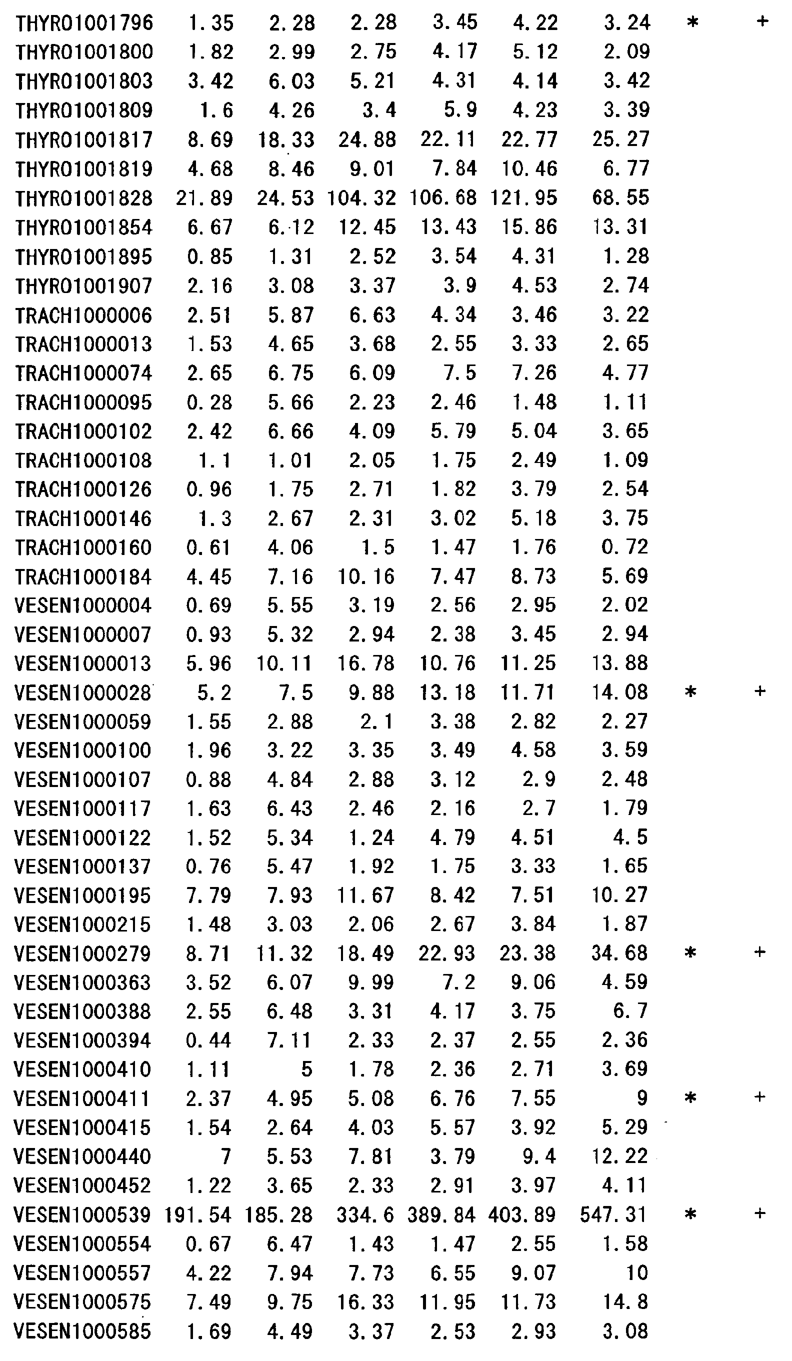

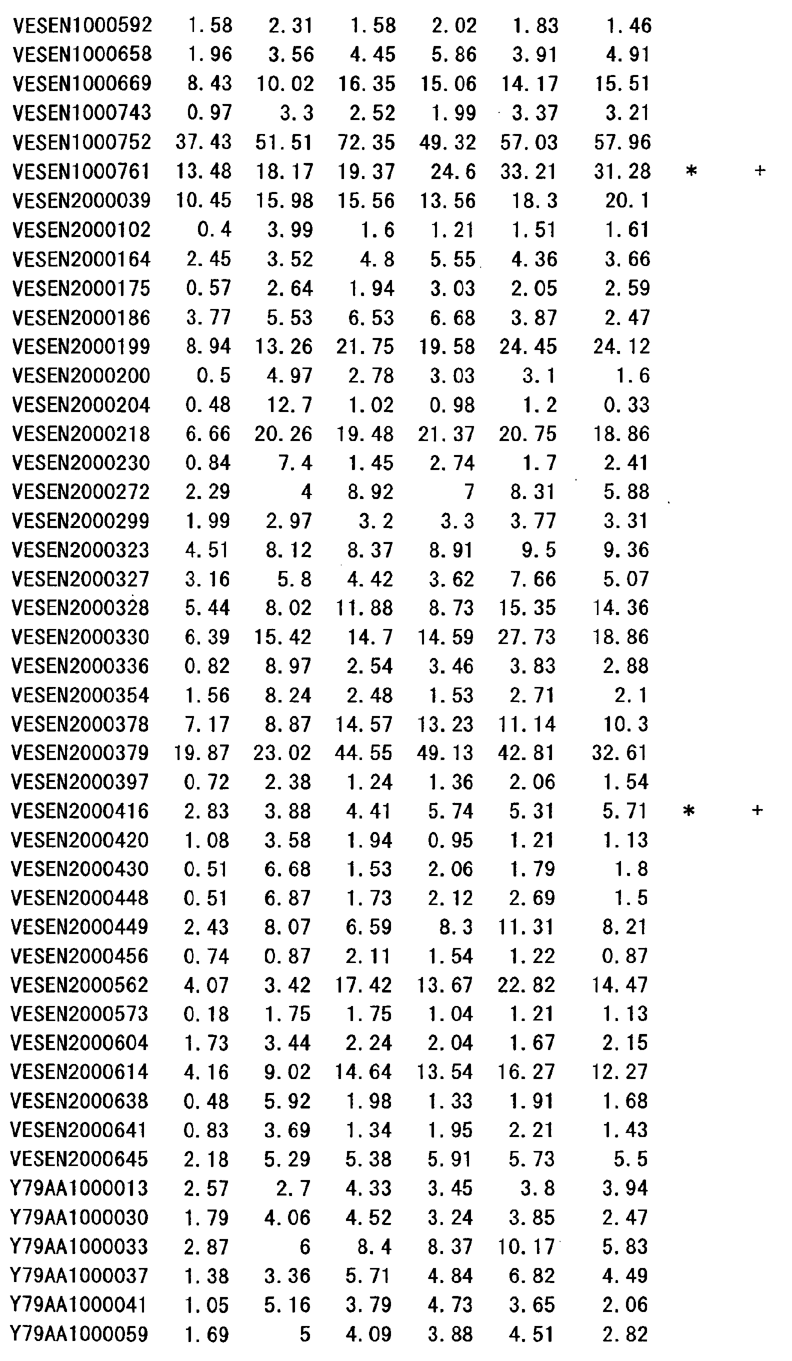

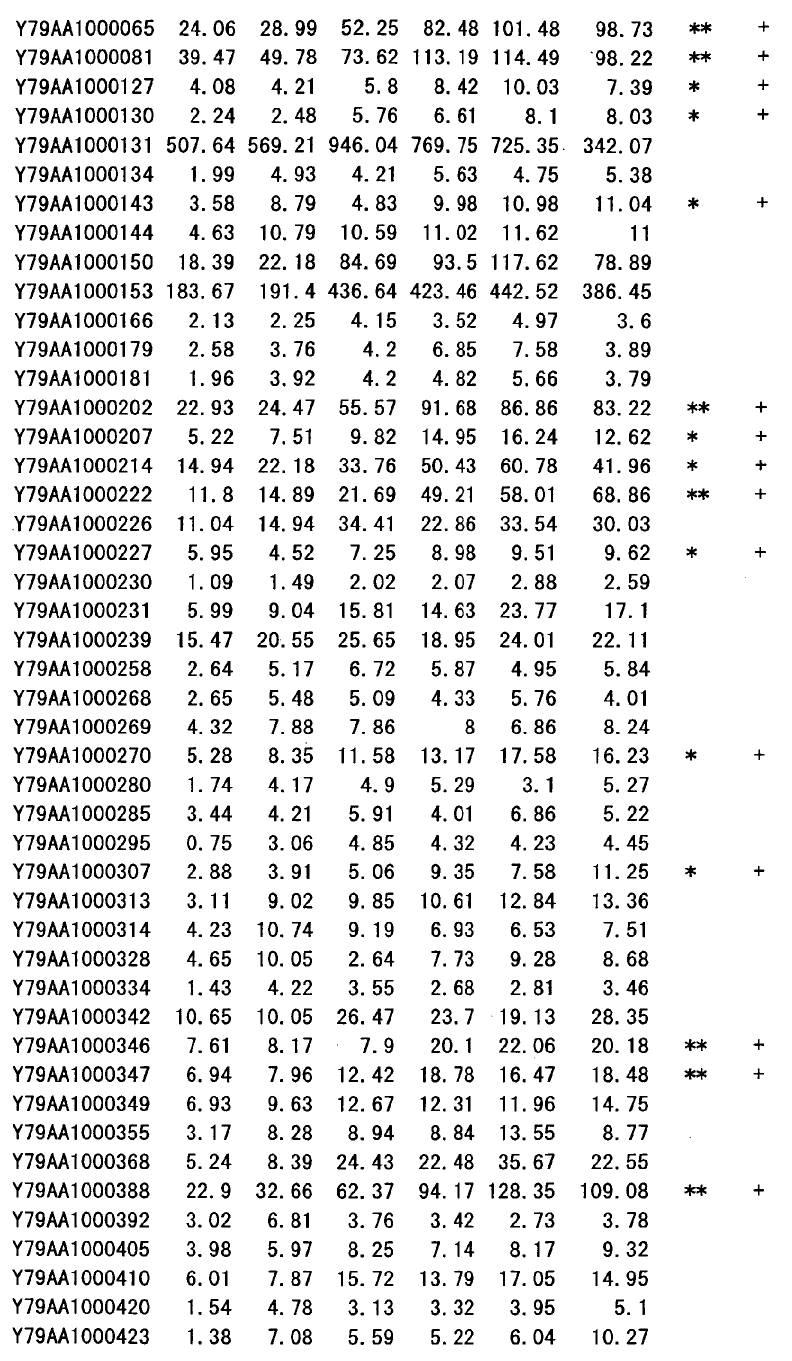

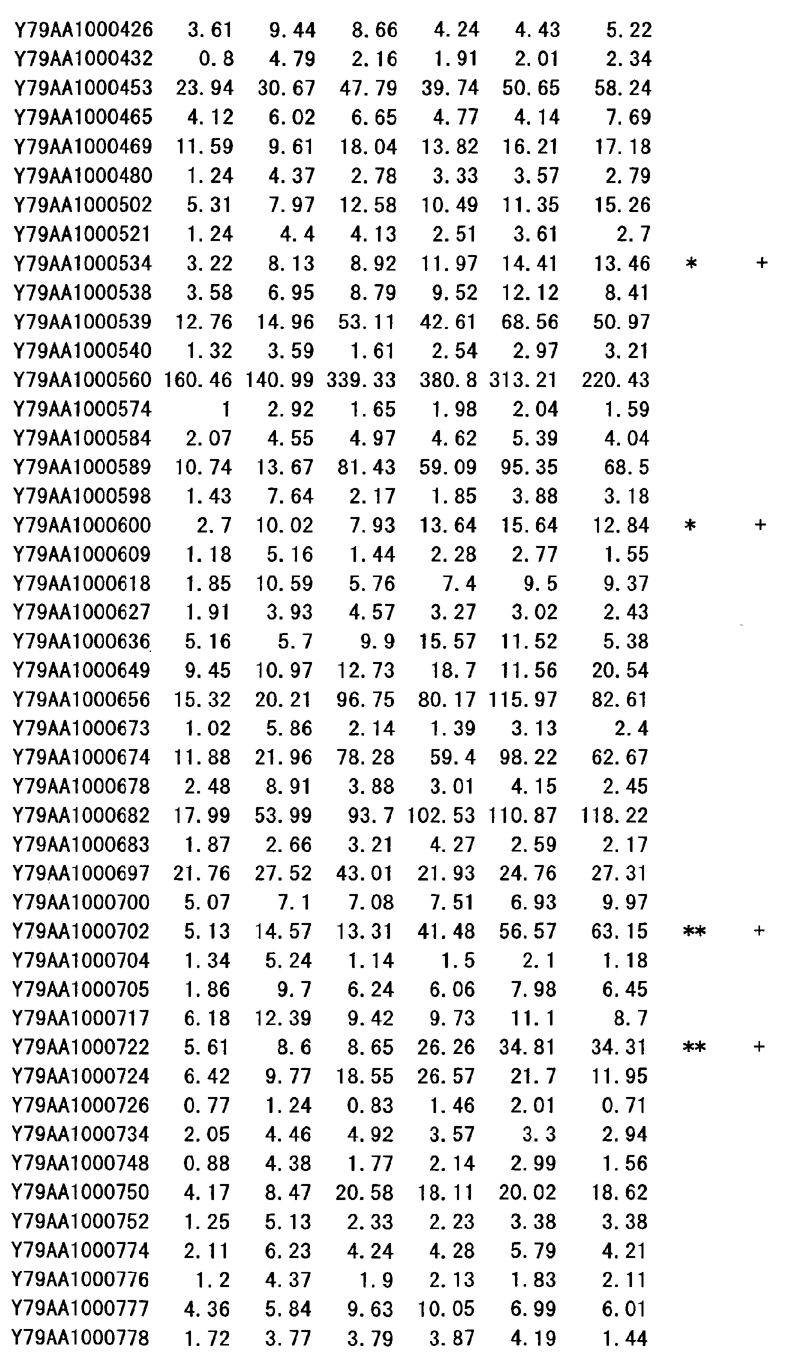

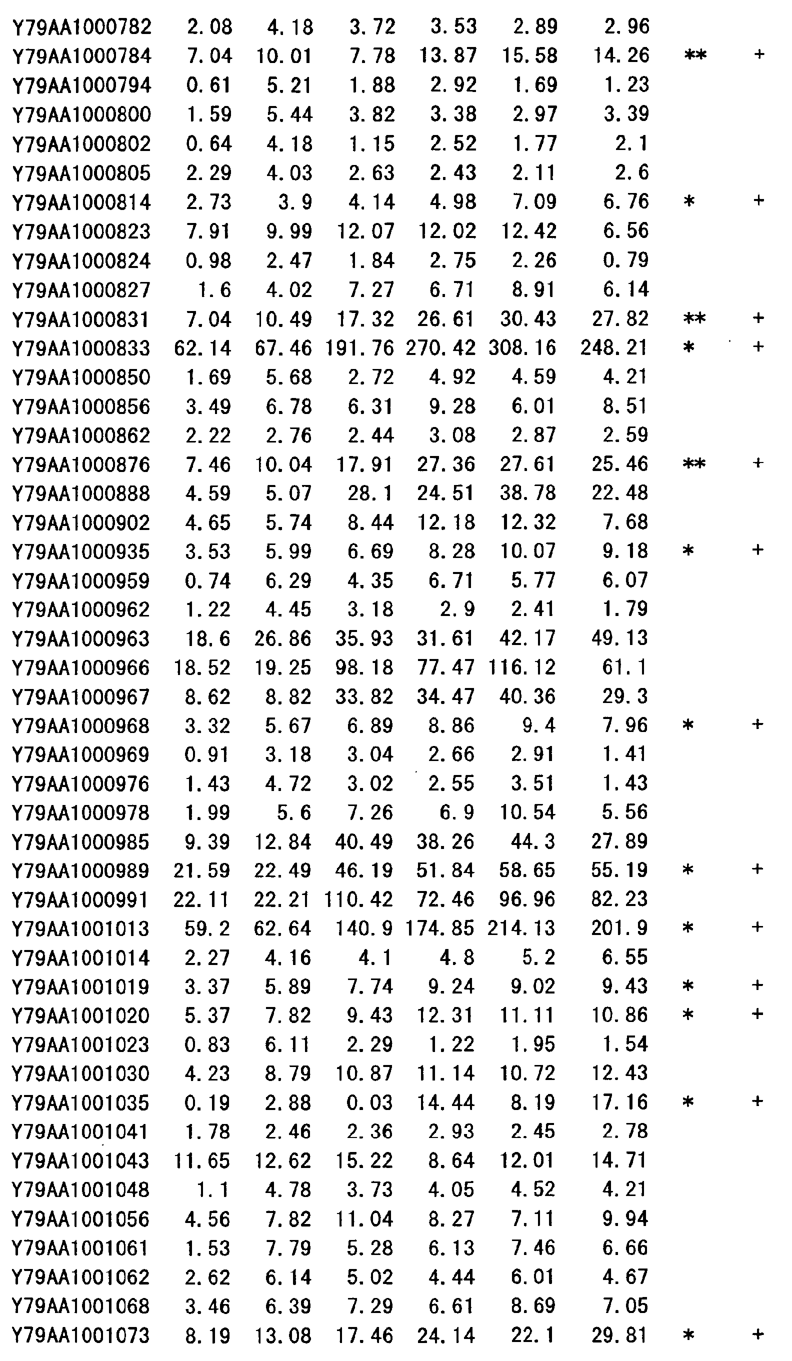

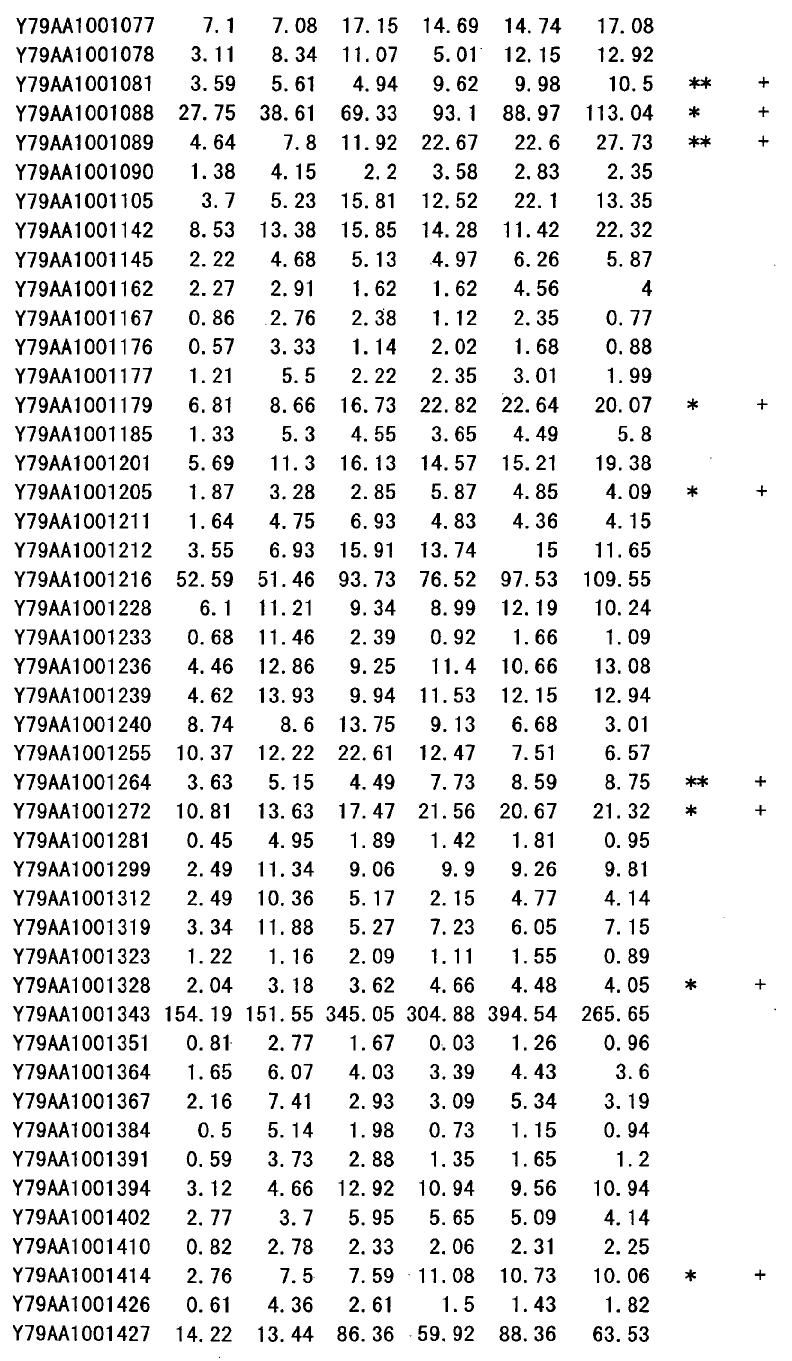

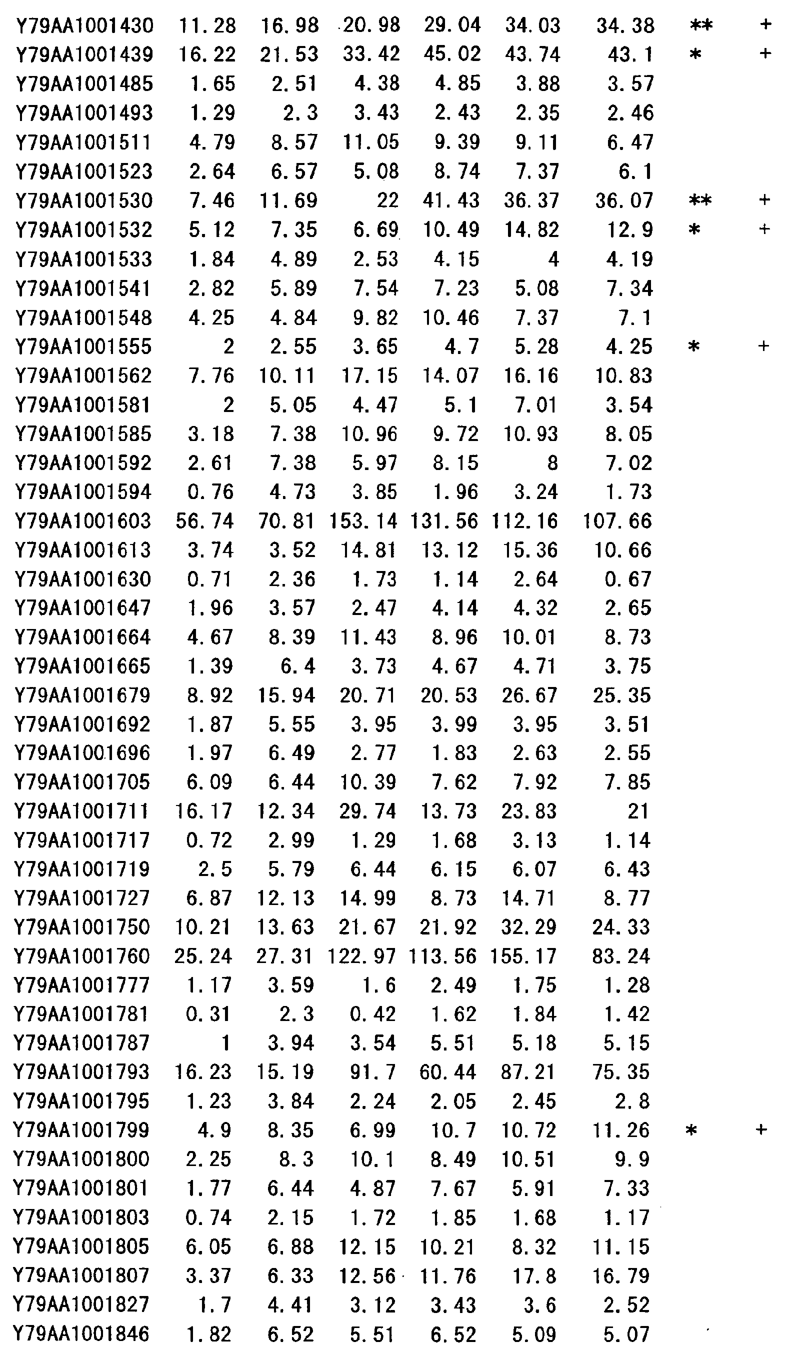

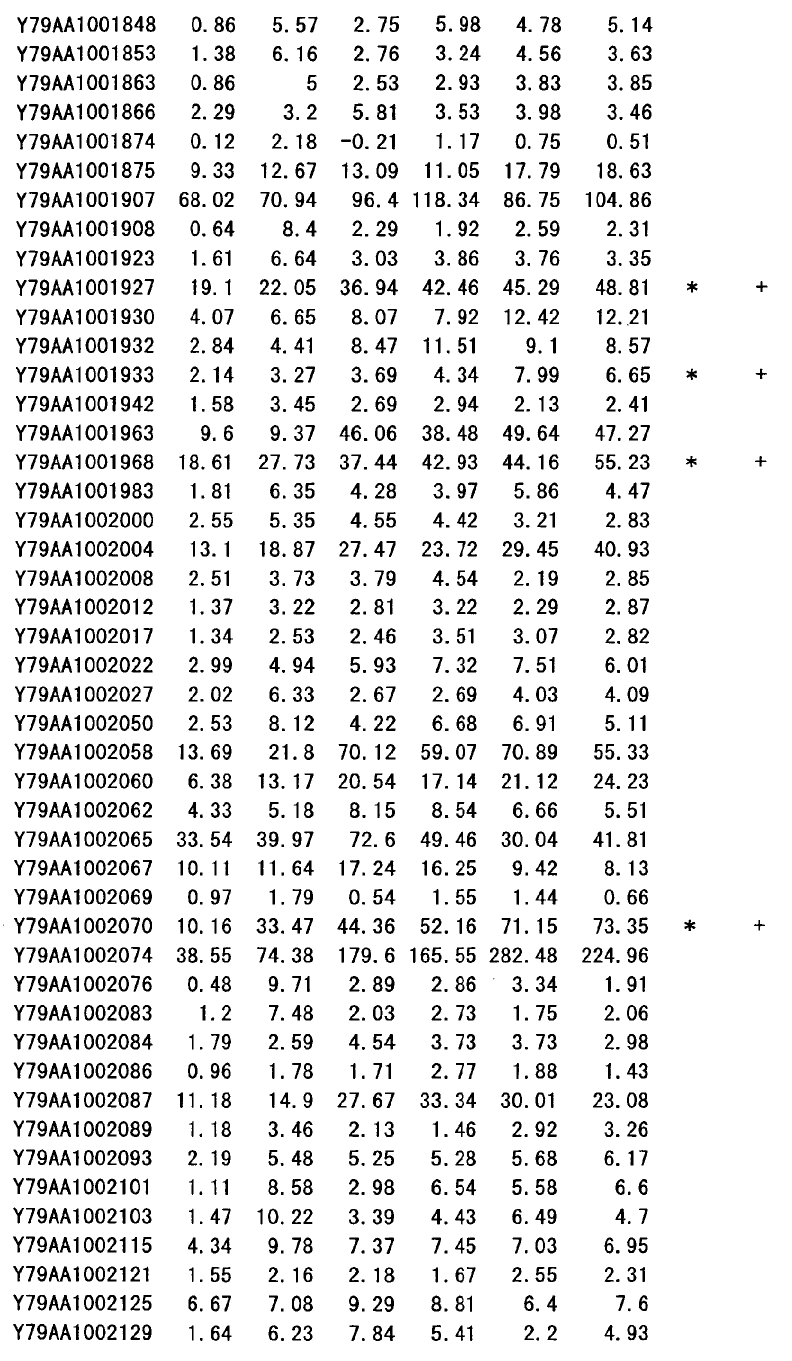

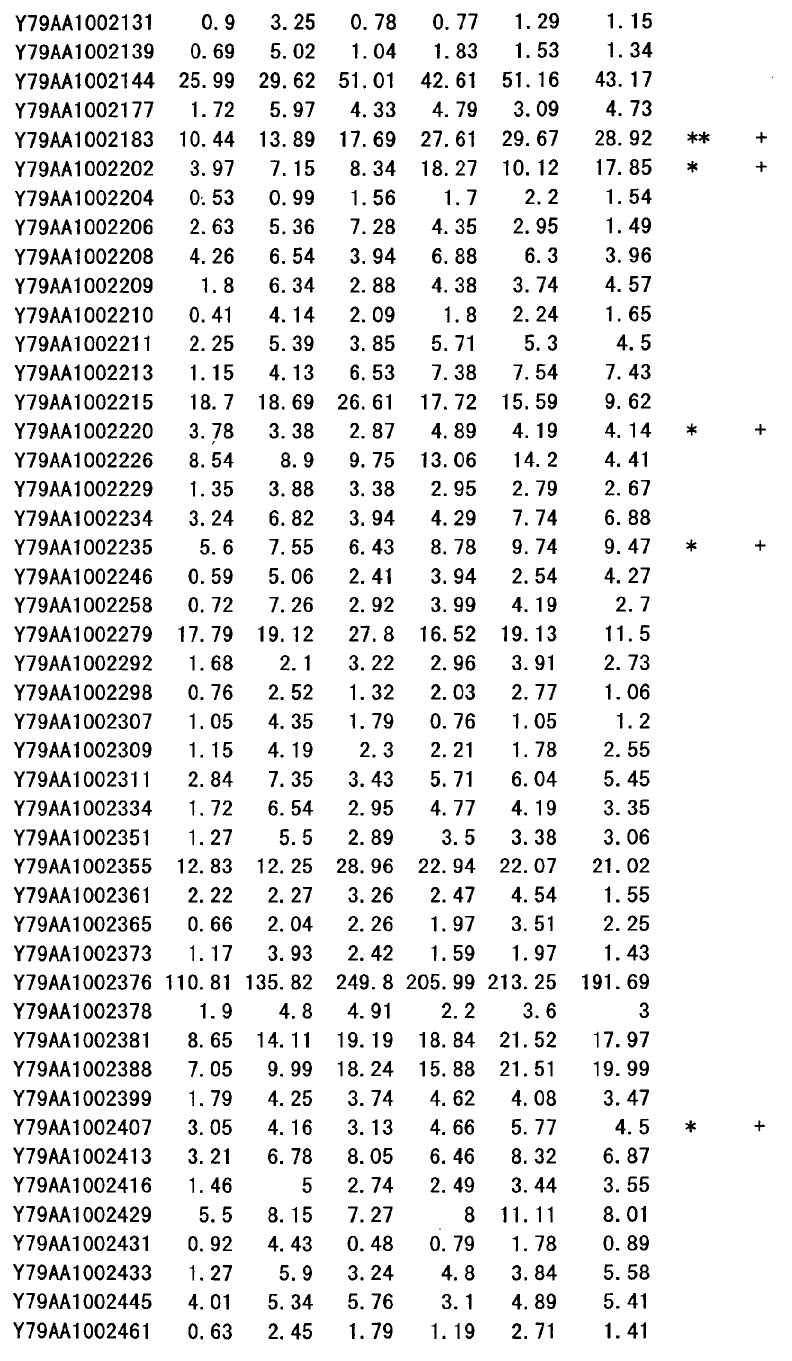

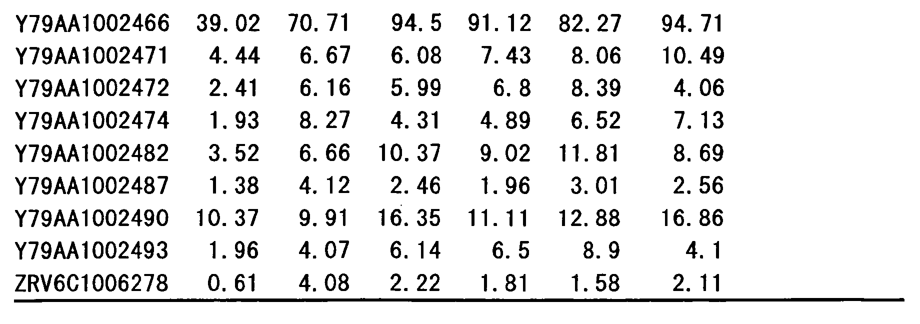

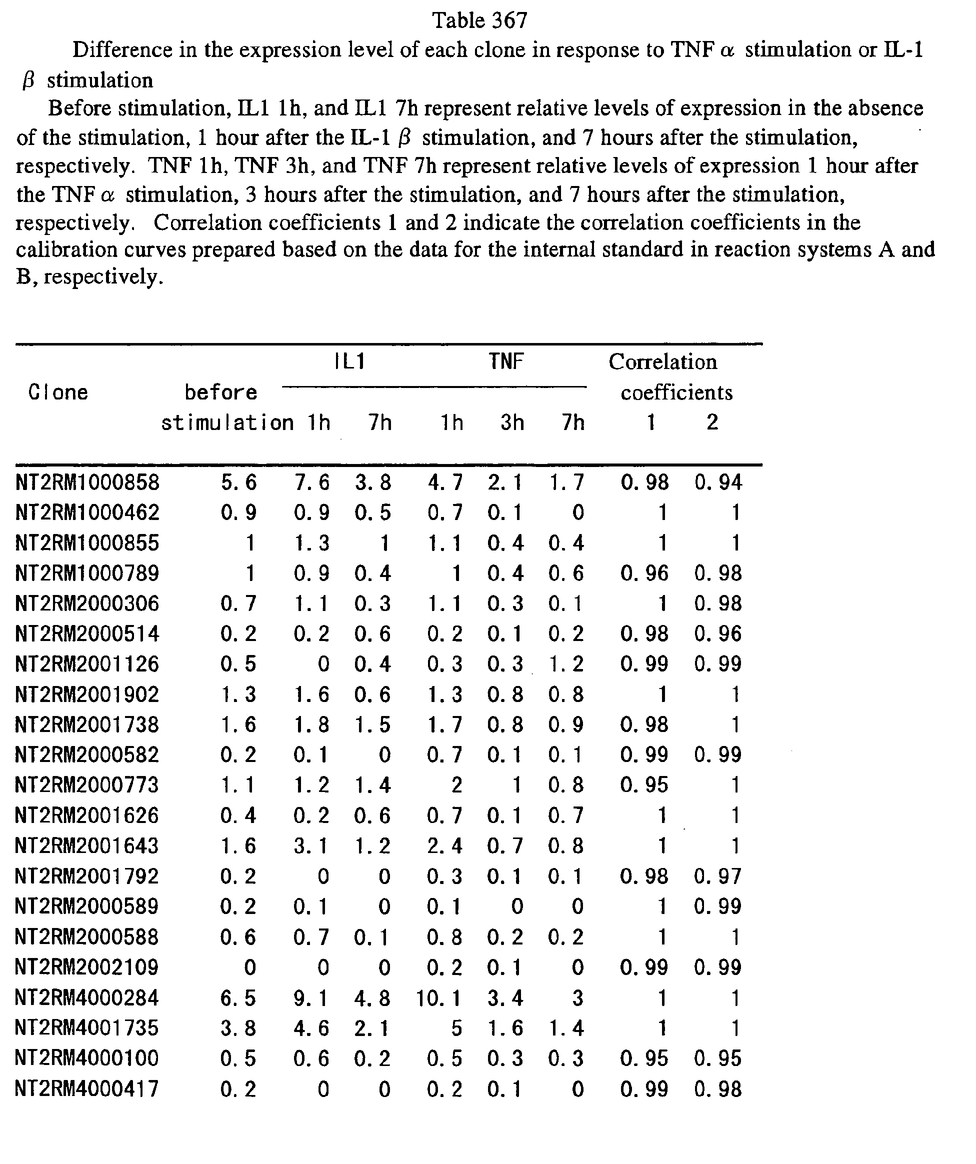

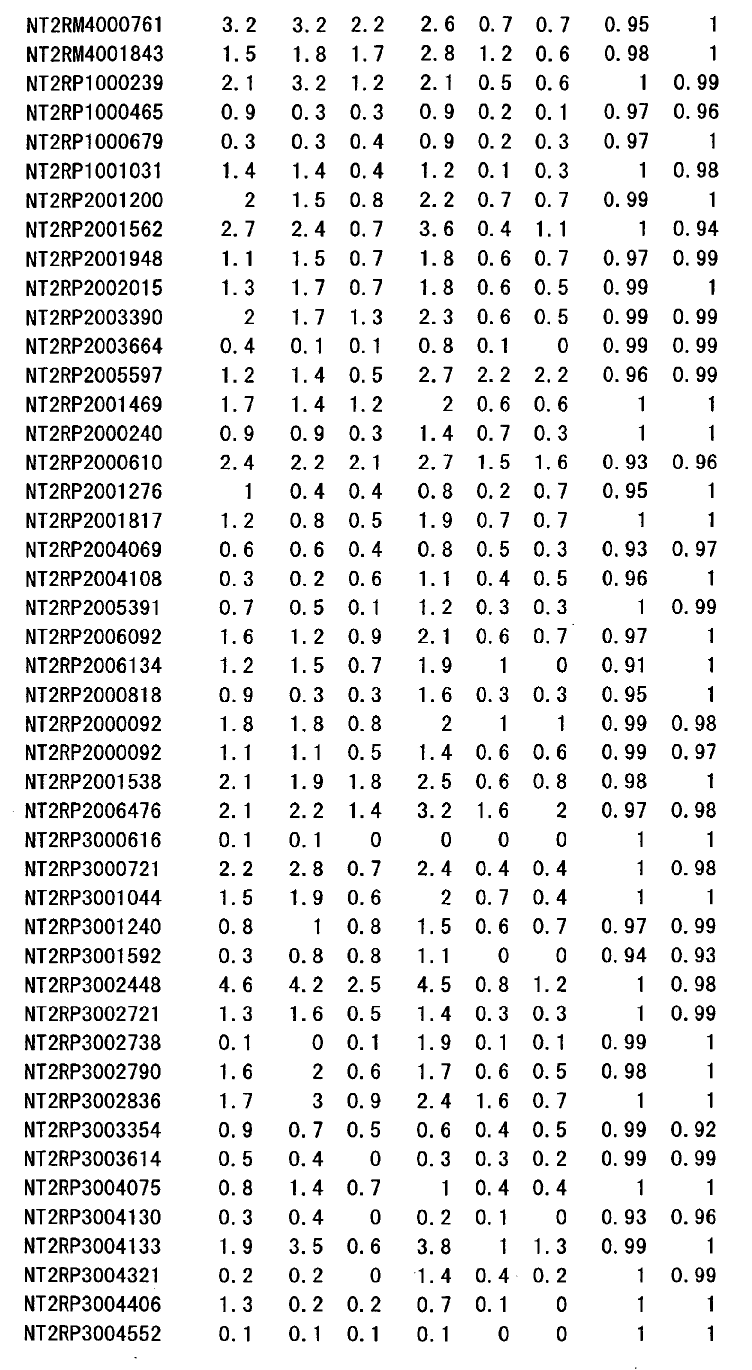

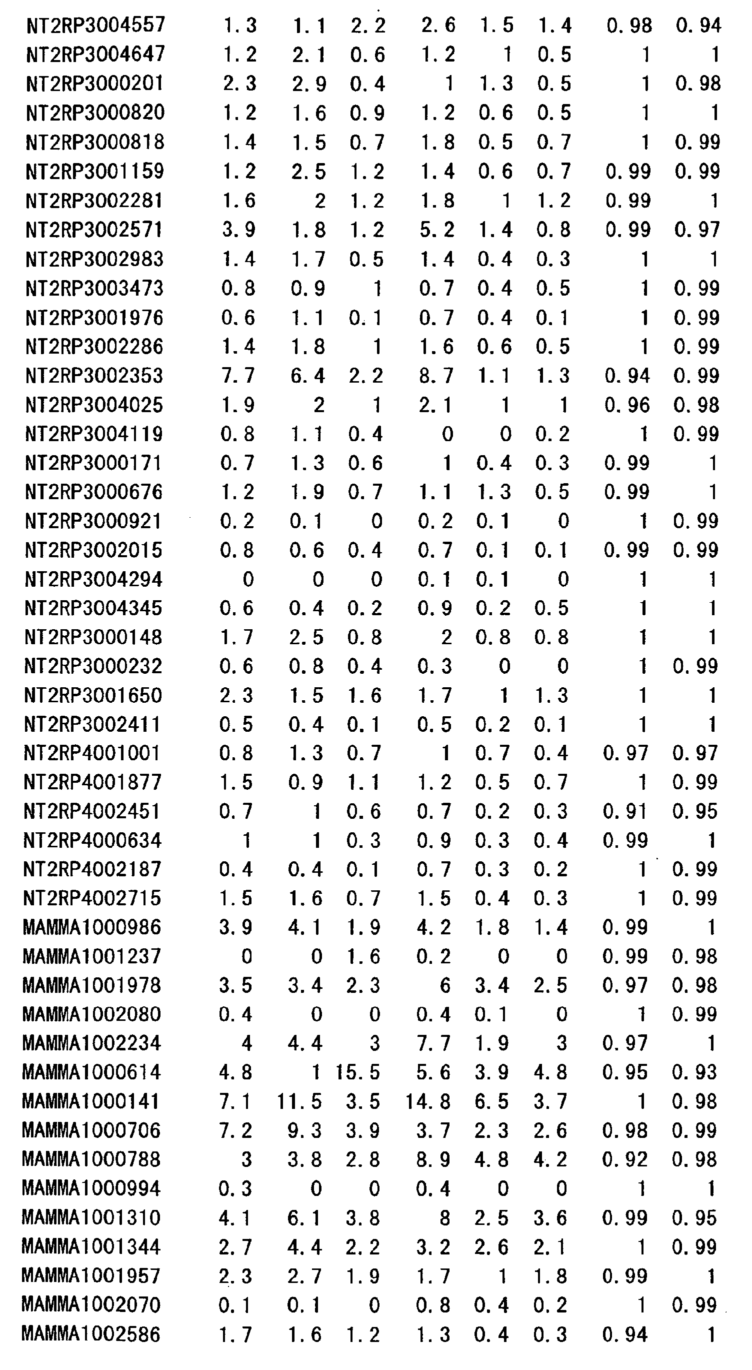

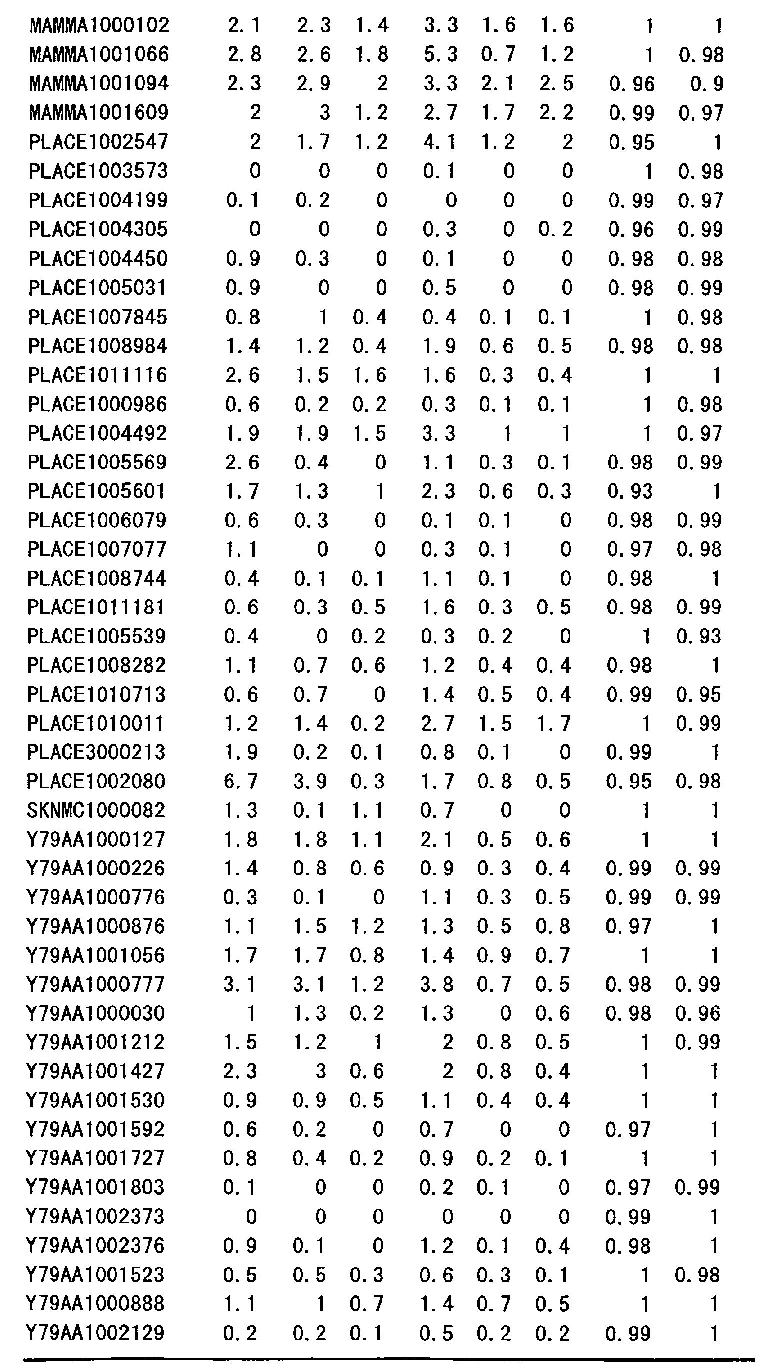

The present invention has provided a total of 830 novel full length cDNA clones. As has not yet proceeded the isolation of full length cDNA within the human, the invention has a large significance. Those proteins such as secretory proteins, membrane proteins, and proteins associated with signal transduction, glycoprotein, and transcription are known to be associated with many diseases. Those genes and proteins associating with diseases are useful for developing medicines as they can be used as a diagnostic marker, or a target for gene therapy or developing medicines that is capable of regulating their expression and activity. Especially, the cDNA clones encoding a secretion protein are extremely important for medicinal industry since the protein itself is expected to be effective as a medicine, and also the gene may have potential to be associating with many diseases. Moreover, those proteins such as membrane proteins, and proteins associated with signal transduction, glycoprotein, transcription, and diseases, and the genes encoding the proteins may be used as a disease marker. These cDNA clones are also important for medicinal industry as they may be effective for treating diseases through the regulation of the expression and activity of their encoded proteins.

| | |
|---|---|
| The names of the representative sequences of the clusters (groups) and the corresponding SEQ IDs. | |
| HRIFA000016a : 1573 | HRIFA017855a : 1979 |
| HRIFA000071a : 1574 | HRIFA017921a : 1980 |
| HRIFA000116a : 1575 | HRIFA018075a : 1981 |
| HRIFA000123a : 1576 | HRIFA018092a : 1982 |
| HRIFA000264a : 1577 | HRIFA018131a : 1983 |
| HRIFA000284a : 1578 | HRIFA018134a : 1984 |
| HRIFA000327a : 1579 | HRIFA018238a : 1985 |
| HRIFA000415a : 1580 | HRIFA018262a : 1986 |
| HRIFA000432a : 1581 | HRIFA018287a : 1987 |
| HRIFA000446a : 1582 | HRIFA018447a : 1988 |
| HRIFA000553a 1583 | HRIFA018580a : 1989 |
| HRIFA000564a : 1584 | HRIFA018666a : 1990 |
| HRIFA000631a : 1585 | HRIFA018688a : 1991 |
| HRIFA000683a : 1586 | HRIFA018754a : 1992 |
| HRIFA000695a : 1587 | HRIFA018794a : 1993 |
| HRIFA000776a : 1588 | HRIFA018827a : 1994 |
| HRIFA000814a : 1589 | HRIFA018849a : 1995 |
| HRIFA000822a : 1590 | HRIFA018870a : 1996 |
| HRIFA000845a : 1591 | HRIFA018904a : 1997 |
| HRIFA000899a : 1592 | HRIFA018931a : 1998 |
| HRIFA000974a : 1593 | HRIFA018993a : 1999 |
| HRIFA001099a : 1594 | HRIFA019105a : 2000 |
| HRIFA001132a : 1595 | HRIFA019136a : 2001 |
| HRIFA001138a : 1596 | HRIFA019175a : 2002 |
| HRIFA001179a : 1597 | HRIFA019185a : 2003 |
| HRIFA001200a : 1598 | HRIFA019262a : 2004 |
| HRIFA001201a : 1599 | HRIFA019412a : 2005 |
| HRIFA001337a : 1600 | HRIFA019437a : 2006 |
| HRIFA001341a : 1601 | HRIFA019466a : 2007 |
| HRIFA001413a : 1602 | HRIFA019490a : 2008 |
| HRIFA001439a : 1603 | HRIFA019498a : 2009 |
| HRIFA001489a : 1604 | HRIFA019532a : 2010 |
| HRIFA001558a : 1605 | HRIFA019651a : 2011 |
| HRIFA001712a : 1606 | HRIFA019867a : 2012 |
| HRIFA001720a : 1607 | HRIFA019869a : 2013 |
| HRIFA001866a : 1608 | HRIFA019958a : 2014 |
| HRIFA001942a : 1609 | HRIFA019960a : 2015 |
| HRIFA001971a : 1610 | HRIFA020109a : 2016 |
| HRIFA001972a : 1611 | HRIFA020144a : 2017 |
| HRIFA001975a : 1612 | HRIFA020163a : 2018 |
| HRIFA001984a : 1613 | HRIFA020184a : 2019 |
| HRIFA002037a : 1614 | HRIFA020272a : 2020 |
| HRIFA002063a : 1615 | HRIFA020335a : 2021 |
| HRIFA002102a : 1616 | HRIFA020349a : 2022 |
| HRIFA002195a : 1617 | HRIFA020453a : 2023 |
| HRIFA002284a : 1618 | HRIFA020466a : 2024 |
| HRIFA002309a : 1619 | HRIFA020661a : 2025 |
| HRIFA002384a : 1620 | HRIFA020693a : 2026 |
| HRIFA002503a : 1621 | HRIFA020707a : 2027 |
| HRIFA002689a : 1622 | HRIFA020748a : 2028 |
| HRIFA002694a : 1623 | HRIFA020862a : 2029 |
| HRIFA002743a : 1624 | HRIFA020883a : 2030 |
| HRIFA002762a : 1625 | HRIFA020965a : 2031 |
| HRIFA002766a : 1626 | HRIFA021007a : 2032 |
| HRIFA002787a : 1627 | HRIFA021040a : 2033 |
| HRIFA002805a : 1628 | HRIFA021061a : 2034 |
| HRIFA002891a : 1629 | HRIFA021069a : 2035 |
| HRIFA002919a : 1630 | HRIFA021136a : 2036 |
| HRIFA002980a : 1631 | HRIFA021213a : 2037 |
| HRIFA002985a : 1632 | HRIFA021224a : 2038 |
| HRIFA003055a : 1633 | HRIFA021323a : 2039 |
| HRIFA003063a : 1634 | HRIFA021398a : 2040 |
| HRIFA003093a : 1635 | HRIFA021399a : 2041 |
| HRIFA003169a : 1636 | HRIFA021445a : 2042 |
| HRIFA003340a : 1637 | HRIFA021448a : 2043 |
| HRIFA003357a : 1638 | HRIFA021494a : 2044 |
| HRIFA003379a : 1639 | HRIFA021499a : 2045 |
| HRIFA003402a : 1640 | HRIFA021543a : 2046 |
| HRIFA003504a : 1641 | HRIFA021594a : 2047 |
| HRIFA003592a : 1642 | HRIFA021611a : 2048 |
| HRIFA003635a : 1643 | HRIFA021620a : 2049 |
| HRIFA003640a : 1644 | HRIFA021637a : 2050 |
| HRIFA003883a : 1645 | HRIFA021651a : 2051 |
| HRIFA003892a : 1646 | HRIFA021754a : 2052 |
| HRIFA003946a : 1647 | HRIFA021781a : 2053 |
| HRIFA004006a : 1648 | HRIFA021787a : 2054 |
| HRIFA004034a : 1649 | HRIFA021794a : 2055 |
| HRIFA004112a : 1650 | HRIFA021855a : 2056 |
| HRIFA004145a : 1651 | HRIFA021886a : 2057 |
| HRIFA004162a : 1652 | HRIFA021906a : 2058 |
| HRIFA004401a : 1653 | HRIFA021985a : 2059 |
| HRIFA004426a : 1654 | HRIFA022055a : 2060 |
| HRIFA004490a : 1655 | HRIFA022065a : 2061 |
| HRIFA004523a : 1656 | HRIFA022106a : 2062 |
| HRIFA004663a : 1657 | HRIFA022139a : 2063 |
| HRIFA004696a : 1658 | HRIFA022156a : 2064 |
| HRIFA004714a : 1659 | HRIFA022166a : 2065 |
| HRIFA004745a : 1660 | HRIFA022177a : 2066 |
| HRIFA004780a : 1661 | HRIFA022182a : 2067 |
| HRIFA004852a : 1662 | HRIFA022203a : 2068 |
| HRIFA004919a : 1663 | HRIFA022227a : 2069 |
| HRIFA005072a : 1664 | HRIFA022234a : 2070 |
| HRIFA005102a : 1665 | HRIFA022249a : 2071 |
| HRIFA005184a : 1666 | HRIFA022262a : 2072 |
| HRIFA005214a : 1667 | HRIFA022265a : 2073 |
| HRIFA005231a : 1668 | HRIFA022328a : 2074 |
| HRIFA005240a : 1669 | HRIFA022335a : 2075 |
| HRIFA005255a : 1670 | HRIFA022348a : 2076 |
| HRIFA005271a : 1671 | HRIFA022411a : 2077 |
| HRIFA005296a : 1672 | HRIFA022423a : 2078 |
| HRIFA005300a : 1673 | HRIFA022462a : 2079 |
| HRIFA005356a : 1674 | HRIFA022493a : 2080 |
| HRIFA005369a : 1675 | HRIFA022528a : 2081 |
| HRIFA005372a : 1676 | HRIFA022546a : 2082 |
| HRIFA005392a : 1677 | HRIFA022564a : 2083 |
| HRIFA005409a : 1678 | HRIFA022616a : 2084 |
| HRIFA005420a : 1679 | HRIFA022671a : 2085 |
| HRIFA005438a : 1680 | HRIFA022691a : 2086 |
| HRIFA005462a : 1681 | HRIFA022702a : 2087 |
| HRIFA005500a : 1682 | HRIFA022707a : 2088 |
| HRIFA005540a : 1683 | HRIFA022714a : 2089 |
| HRIFA005644a : 1684 | HRIFA022728a : 2090 |
| HRIFA005650a : 1685 | HRIFA022729a : 2091 |
| HRIFA005702a : 1686 | HRIFA022737a : 2092 |
| HRIFA005720a : 1687 | HRIFA022776a : 2093 |
| HRIFA005728a : 1688 | HRIFA022782a : 2094 |
| HRIFA005732a : 1689 | HRIFA022794a : 2095 |
| HRIFA005760a : 1690 | HRIFA022865a : 2096 |
| HRIFA005781a : 1691 | HRIFA022875a : 2097 |
| HRIFA005944a : 1692 | HRIFA022890a : 2098 |
| HRIFA006018a : 1693 | HRIFA022895a : 2099 |
| HRIFA006183a : 1694 | HRIFA022985a : 2100 |
| HRIFA006216a : 1695 | HRIFA023007a : 2101 |
| HRIFA006250a : 1696 | HRIFA023048a : 2102 |
| HRIFA006298a : 1697 | HRIFA023069a : 2103 |
| HRIFA006448a : 1698 | HRIFA023129a : 2104 |
| HRIFA006494a : 1699 | HRIFA023154a : 2105 |
| HRIFA006510a : 1700 | HRIFA023212a : 2106 |
| HRIFA006566a : 1701 | HRIFA023227a : 2107 |
| HRIFA006572a : 1702 | HRIFA023257a : 2108 |
| HRIFA006586a : 1703 | HRIFA023304a : 2109 |
| HRIFA006596a : 1704 | HRIFA023305a : 2110 |
| HRIFA006609a : 1705 | HRIFA023434a : 2111 |
| HRIFA006633a : 1706 | HRIFA023464a : 2112 |
| HRIFA006642a : 1707 | HRIFA023489a : 2113 |
| HRIFA006649a : 1708 | HRIFA023521a : 2114 |
| HRIFA006667a : 1709 | HRIFA023605a : 2115 |
| HRIFA006730a : 1710 | HRIFA023619a : 2116 |
| HRIFA006798a : 1711 | HRIFA023634a : 2117 |
| HRIFA006926a : 1712 | HRIFA023767a : 2118 |
| HRIFA007013a : 1713 | HRIFA023894a : 2119 |
| HRIFA007026a : 1714 | HRIFA023923a : 2120 |
| HRIFA007032a : 1715 | HRIFA024088a : 2121 |
| HRIFA007068a : 1716 | HRIFA024132a : 2122 |
| HRIFA007152a : 1717 | HRIFA024185a : 2123 |
| HRIFA007219a : 1718 | HRIFA024197a : 2124 |
| HRIFA007228a : 1719 | HRIFA024218a : 2125 |
| HRIFA007243a : 1720 | HRIFA024255a : 2126 |
| HRIFA037838a : 1721 | HRIFA024305a : 2127 |
| HRIFA007256a : 1722 | HRIFA024392a : 2128 |
| HRIFA007262a : 1723 | HRIFA024423a : 2129 |
| HRIFA007352a : 1724 | HRIFA024473a : 2130 |
| HRIFA007424a : 1725 | HRIFA024482a : 2131 |
| HRIFA007435a : 1726 | HRIFA024504a : 2132 |
| HRIFA007463a : 1727 | HRIFA024533a : 2133 |
| HRIFA007493a : 1728 | HRIFA024543a : 2134 |
| HRIFA007512a : 1729 | HRIFA024554a : 2135 |
| HRIFA007532a : 1730 | HRIFA024718a : 2136 |
| HRIFA007541a : 1731 | HRIFA024767a : 2137 |
| HRIFA007547a : 1732 | HRIFA024841a : 2138 |
| HRIFA007565a : 1733 | HRIFA024884a : 2139 |
| HRIFA007571a : 1734 | HRIFA024893a : 2140 |
| HRIFA007619a : 1735 | HRIFA024921a : 2141 |
| HRIFA007659a : 1736 | HRIFA024937a : 2142 |
| HRIFA007722a : 1737 | HRIFA024978a : 2143 |
| HRIFA007728a : 1738 | HRIFA024994a : 2144 |
| HRIFA007745a : 1739 | HRIFA025033a : 2145 |
| HRIFA007829a : 1740 | HRIFA025046a : 2146 |
| HRIFA007909a : 1741 | HRIFA025125a : 2147 |
| HRIFA007985a : 1742 | HRIFA025143a : 2148 |
| HRIFA008000a : 1743 | HRIFA025250a : 2149 |
| HRIFA008099a : 1744 | HRIFA025261a : 2150 |
| HRIFA008131a : 1745 | HRIFA025276a : 2151 |
| HRIFA034865a : 1746 | HRIFA025290a : 2152 |
| HRIFA008186a : 1747 | HRIFA025327a : 2153 |
| HRIFA008200a : 1748 | HRIFA025353a : 2154 |
| HRIFA008212a : 1749 | HRIFA025360a : 2155 |
| HRIFA008252a : 1750 | HRIFA025479a : 2156 |
| HRIFA008284a : 1751 | HRIFA025488a : 2157 |
| HRIFA008314a : 1752 | HRIFA025492a : 2158 |
| HRIFA008362a : 1753 | HRIFA025498a : 2159 |
| HRIFA008387a : 1754 | HRIFA025514a : 2160 |
| HRIFA008426a : 1755 | HRIFA025636a : 2161 |
| HRIFA008459a : 1756 | HRIFA025695a : 2162 |
| HRIFA008483a : 1757 | HRIFA025703a : 2163 |
| HRIFA008547a : 1758 | HRIFA025706a : 2164 |
| HRIFA008596a : 1759 | HRIFA025766a : 2165 |
| HRIFA008606a : 1760 | HRIFA025771a : 2166 |
| HRIFA008611a : 1761 | HRIFA025778a : 2167 |
| HRIFA008661a : 1762 | HRIFA025786a : 2168 |
| HRIFA008717a : 1763 | HRIFA025792a : 2169 |
| HRIFA008753a : 1764 | HRIFA025800a : 2170 |
| HRIFA008784a : 1765 | HRIFA025904a : 2171 |
| HRIFA008790a : 1766 | HRIFA025907a : 2172 |
| HRIFA008827a : 1767 | HRIFA037840a : 2173 |
| HRIFA008949a : 1768 | HRIFA025936a : 2174 |
| HRIFA008976a : 1769 | HRIFA025966a : 2175 |
| HRIFA008981a : 1770 | HRIFA025978a : 2176 |
| HRIFA008989a : 1771 | HRIFA026089a : 2177 |
| HRIFA009071a : 1772 | HRIFA026121a : 2178 |
| HRIFA009101a : 1773 | HRIFA026151a : 2179 |
| HRIFA009123a : 1774 | HRIFA026153a : 2180 |
| HRIFA009136a : 1775 | HRIFA026203a : 2181 |
| HRIFA009171a : 1776 | HRIFA026210a : 2182 |
| HRIFA009214a : 1777 | HRIFA026242a : 2183 |
| HRIFA009220a : 1778 | HRIFA026265a : 2184 |
| HRIFA009339a : 1779 | HRIFA026303a : 2185 |
| HRIFA009372a : 1780 | HRIFA026316a : 2186 |
| HRIFA009392a : 1781 | HRIFA026346a : 2187 |
| HRIFA009414a : 1782 | HRIFA026351a : 2188 |
| HRIFA009451a : 1783 | HRIFA026364a : 2189 |
| HRIFA009482a : 1784 | HRIFA026382a : 2190 |
| HRIFA009578a : 1785 | HRIFA026465a : 2191 |
| HRIFA009762a : 1786 | HRIFA026490a : 2192 |
| HRIFA009764a : 1787 | HRIFA026496a : 2193 |
| HRIFA009783a : 1788 | HRIFA026519a : 2194 |
| HRIFA009825a : 1789 | HRIFA026564a : 2195 |
| HRIFA009852a : 1790 | HRIFA026576a : 2196 |
| HRIFA009881a : 1791 | HRIFA026615a : 2197 |
| HRIFA009983a : 1792 | HRIFA026618a : 2198 |
| HRIFA010005a : 1793 | HRIFA026659a : 2199 |
| HRIFA010034a : 1794 | HRIFA026735a : 2200 |
| HRIFA010070a : 1795 | HRIFA026764a : 2201 |
| HRIFA010078a : 1796 | HRIFA026789a : 2202 |
| HRIFA010085a : 1797 | HRIFA026813a : 2203 |
| HRIFA010090a : 1798 | HRIFA026860a : 2204 |
| HRIFA010130a : 1799 | HRIFA026889a : 2205 |
| HRIFA010152a : 1800 | HRIFA026899a : 2206 |
| HRIFA010176a : 1801 | HRIFA026918a : 2207 |
| HRIFA010206a : 1802 | HRIFA026923a : 2208 |
| HRIFA010301a : 1803 | HRIFA027012a : 2209 |
| HRIFA010319a : 1804 | HRIFA027045a : 2210 |
| HRIFA010322a : 1805 | HRIFA027125a : 2211 |
| HRIFA010361a : 1806 | HRIFA027173a : 2212 |
| HRIFA010394a : 1807 | HRIFA027179a : 2213 |
| HRIFA010425a : 1808 | HRIFA027187a : 2214 |
| HRIFA010460a : 1809 | HRIFA027265a : 2215 |
| HRIFA010466a : 1810 | HRIFA027327a : 2216 |
| HRIFA010490a : 1811 | HRIFA027329a : 2217 |
| HRIFA010593a : 1812 | HRIFA027355a : 2218 |
| HRIFA010736a : 1813 | HRIFA027485a : 2219 |
| HRIFA010790a : 1814 | HRIFA027493a : 2220 |
| HRIFA010799a : 1815 | HRIFA027536a : 2221 |
| HRIFA010859a : 1816 | HRIFA027549a : 2222 |
| HRIFA010891a : 1817 | HRIFA027619a : 2223 |
| HRIFA010942a : 1818 | HRIFA027622a : 2224 |
| HRIFA010975a : 1819 | HRIFA027625a : 2225 |
| HRIFA010988a : 1820 | HRIFA027644a : 2226 |
| HRIFA011016a : 1821 | HRIFA027656a : 2227 |
| HRIFA011105a : 1822 | HRIFA027673a : 2228 |
| HRIFA011128a : 1823 | HRIFA027681a : 2229 |
| HRIFA011179a : 1824 | HRIFA027701a : 2230 |
| HRIFA011193a : 1825 | HRIFA027714a : 2231 |
| HRIFA011197a : 1826 | HRIFA027717a : 2232 |
| HRIFA011347a : 1827 | HRIFA027722a : 2233 |
| HRIFA011403a : 1828 | HRIFA027754a : 2234 |
| HRIFA011449a : 1829 | HRIFA027803a : 2235 |
| HRIFA011484a : 1830 | HRIFA027860a : 2236 |
| HRIFA011512a : 1831 | HRIFA027867a : 2237 |
| HRIFA011580a : 1832 | HRIFA027874a : 2238 |
| HRIFA011659a : 1833 | HRIFA027940a : 2239 |
| HRIFA011802a : 1834 | HRIFA027961a : 2240 |
| HRIFA011820a : 1835 | HRIFA028061a : 2241 |
| HRIFA011926a : 1836 | HRIFA028157a : 2242 |
| HRIFA011947a : 1837 | HRIFA028187a : 2243 |
| HRIFA012022a : 1838 | HRIFA028262a : 2244 |
| HRIFA012069a : 1839 | HRIFA028371a : 2245 |
| HRIFA012151a : 1840 | HRIFA028401a : 2246 |
| HRIFA012167a : 1841 | HRIFA028402a : 2247 |
| HRIFA012278a : 1842 | HRIFA028440a : 2248 |
| HRIFA012282a : 1843 | HRIFA028465a : 2249 |
| HRIFA012283a : 1844 | HRIFA028468a : 2250 |
| HRIFA012290a : 1845 | HRIFA028497a : 2251 |
| HRIFA012333a : 1846 | HRIFA028501a : 2252 |
| HRIFA012354a : 1847 | HRIFA028511a : 2253 |
| HRIFA012417a : 1848 | HRIFA028573a : 2254 |
| HRIFA012427a : 1849 | HRIFA028576a : 2255 |
| HRIFA012436a : 1850 | HRIFA028592a : 2256 |
| HRIFA012480a : 1851 | HRIFA028614a : 2257 |
| HRIFA012513a : 1852 | HRIFA028651a : 2258 |
| HRIFA012515a : 1853 | HRIFA028652a : 2259 |
| HRIFA012584a : 1854 | HRIFA028654a : 2260 |
| HRIFA012625a : 1855 | HRIFA028708a : 2261 |
| HRIFA012692a : 1856 | HRIFA028790a : 2262 |
| HRIFA012702a : 1857 | HRIFA028804a : 2263 |
| HRIFA012737a : 1858 | HRIFA028867a : 2264 |
| HRIFA012761a : 1859 | HRIFA028911a : 2265 |
| HRIFA012795a : 1860 | HRIFA028926a : 2266 |
| HRIFA012881a : 1861 | HRIFA028983a : 2267 |
| HRIFA012885a : 1862 | HRIFA029002a : 2268 |
| HRIFA012914a : 1863 | HRIFA029050a : 2269 |
| HRIFA012969a : 1864 | HRIFA029107a : 2270 |
| HRIFA012990a : 1 1865 | HRIFA029208a : 2271 |
| HRIFA012999a : 1866 | HRIFA029209a : 2272 |
| HRIFA013092a : 1867 | HRIFA029256a : 2273 |
| HRIFA013103a : 1868 | HRIFA029263a : 2274 |
| HRIFA013135a : 1869 | HRIFA029274a : 2275 |
| HRIFA013235a : 1870 | HRIFA029278a : 2276 |
| HRIFA013254a : 1871 | HRIFA029285a : 2277 |
| HRIFA013265a : 1872 | HRIFA029317a : 2278 |
| HRIFA013276a : 1873 | HRIFA029327a : 2279 |
| HRIFA013279a : 1874 | HRIFA029349a : 2280 |
| HRIFA013288a : 1875 | HRIFA029393a : 2281 |
| HRIFA013376a : 1876 | HRIFA029398a : 2282 |
| HRIFA013477a : 1877 | HRIFA029425a : 2283 |
| HRIFA013586a : 1878 | HRIFA029434a : 2284 |
| HRIFA013589a : 1879 | HRIFA029440a : 2285 |
| HRIFA013620a : 1880 | HRIFA029460a : 2286 |
| HRIFA013668a : 1881 | HRIFA029467a : 2287 |
| HRIFA013726a : 1882 | HRIFA029508a : 2288 |
| HRIFA013744a : 1883 | HRIFA029511a : 2289 |
| HRIFA013899a : 1884 | HRIFA029577a : 2290 |
| HRIFA013911a : 1885 | HRIFA029602a : 2291 |
| HRIFA013919a : 1886 | HRIFA029649a : 2292 |
| HRIFA013932a : 1887 | HRIFA029715a : 2293 |
| HRIFA013980a : 1888 | HRIFA029730a : 2294 |
| HRIFA014006a : 1889 | HRIFA029779a : 2295 |
| HRIFA014024a : 1890 | HRIFA029792a : 2296 |
| HRIFA014056a : 1891 | HRIFA029802a : 2297 |
| HRIFA014111a : 1892 | HRIFA029866a : 2298 |
| HRIFA014133a : 1893 | HRIFA029932a : 2299 |
| HRIFA014178a : 1894 | HRIFA030025a : 2300 |
| HRIFA014185a : 1895 | HRIFA030045a : 2301 |
| HRIFA014303a : 1896 | HRIFA030097a : 2302 |
| HRIFA014336a : 1897 | HRIFA030103a : 2303 |
| HRIFA014396a : 1898 | HRIFA030106a : 2304 |
| HRIFA014397a : 1899 | HRIFA030147a : 2305 |
| HRIFA014417a : 1900 | HRIFA030203a : 2306 |
| HRIFA014465a : 1901 | HRIFA030237a : 2307 |
| HRIFA014467a : 1902 | HRIFA030248a : 2308 |
| HRIFA014482a : 1903 | HRIFA030250a : 2309 |
| HRIFA014500a : 1904 | HRIFA030264a : 2310 |
| HRIFA014561a : 1905 | HRIFA030342a : 2311 |
| HRIFA014568a : 1906 | HRIFA030370a : 2312 |
| HRIFA014590a : 1907 | HRIFA030371a : 2313 |
| HRIFA014598a : 1908 | HRIFA030381a : 2314 |
| HRIFA014620a : 1909 | HRIFA030385a : 2315 |
| HRIFA014621a : 1910 | HRIFA030394a : 2316 |
| HRIFA014688a : 1911 | HRIFA030408a : 2317 |
| HRIFA014692a : 1912 | HRIFA030411a : 2318 |
| HRIFA014702a : 1913 | HRIFA030448a : 2319 |
| HRIFA014819a : 1914 | HRIFA030456a : 2320 |
| HRIFA014868a : 1915 | HRIFA030461a : 2321 |
| HRIFA014951a : 1916 | HRIFA030472a : 2322 |
| HRIFA014953a : 1917 | HRIFA030509a : 2323 |
| HRIFA014967a : 1918 | HRIFA030511a : 2324 |
| HRIFA015063a : 1919 | HRIFA030545a : 2325 |
| HRIFA015070a : 1920 | HRIFA030566a : 2326 |
| HRIFA015122a : 1921 | HRIFA030599a : 2327 |
| HRIFA015129a : 1922 | HRIFA030629a : 2328 |
| HRIFA015219a : 1923 | HRIFA030642a : 2329 |
| HRIFA015236a : 1924 | HRIFA030662a : 2330 |
| HRIFA015246a : 1925 | HRIFA030839a : 2331 |
| HRIFA015351a : 1926 | HRIFA030981a : 2332 |
| HRIFA015409a : 1927 | HRIFA031062a : 2333 |
| HRIFA015423a : 1928 | HRIFA031075a : 2334 |
| HRIFA015443a : 1929 | HRIFA031091a : 2335 |
| HRIFA015453a : 1930 | HRIFA031126a : 2336 |
| HRIFA015471a : 1931 | HRIFA031249a : 2337 |
| HRIFA015486a : 1932 | HRIFA031336a : 2338 |
| HRIFA015506a : 1933 | HRIFA031350a : 2339 |
| HRIFA015536a : 1934 | HRIFA031395a : 2340 |
| HRIFA015547a : 1935 | HRIFA031397a : 2341 |
| HRIFA015568a : 1936 | HRIFA031438a : 2342 |
| HRIFA015671a : 1937 | HRIFA031472a : 2343 |
| HRIFA015682a : 1938 | HRIFA031510a : 2344 |
| HRIFA015756a : 1939 | HRIFA031672a : 2345 |
| HRIFA015764a : 1940 | HRIFA031869a : 2346 |
| HRIFA015802a : 1941 | HRIFA031871a : 2347 |
| HRIFA015811a : 1942 | HRIFA031895a : 2348 |
| HRIFA015902a : 1943 | HRIFA031935a : 2349 |
| HRIFA015947a : 1944 | HRIFA031986a : 2350 |
| HRIFA015995a : 1945 | HRIFA032009a : 2351 |
| HRIFA016070a : 1946 | HRIFA032011a : 2352 |
| HRIFA016129a : 1947 | HRIFA032066a : 2353 |
| HRIFA016214a : 1948 | HRIFA032067a : 2354 |
| HRIFA016240a : 1949 | HRIFA032070a : 2355 |
| HRIFA016255a : 1950 | HRIFA032073a : 2356 |
| HRIFA016290a : 1951 | HRIFA032079a : 2357 |
| HRIFA016430a : 1952 | HRIFA032097a : 2358 |
| HRIFA016599a : 1953 | HRIFA032161a : 2359 |
| HRIFA016623a : 1954 | HRIFA032186a : 2360 |
| HRIFA016639a : 1955 | HRIFA032224a : 2361 |
| HRIFA016654a : 1956 | HRIFA032257a : 2362 |
| HRIFA016669a : 1957 | HRIFA032271a : 2363 |
| HRIFA016758a : 1958 | HRIFA032274a : 2364 |
| HRIFA016838a : 1959 | HRIFA032275a : 2365 |
| HRIFA016963a : 1960 | HRIFA032360a : 2366 |
| HRIFA017031a : 1961 | HRIFA032389a : 2367 |
| HRIFA017146a : 1962 | HRIFA032433a : 2368 |
| HRIFA017190a : 1963 | HRIFA032453a : 2369 |
| HRIFA017257a : 1964 | HRIFA032478a : 2370 |
| HRIFA017295a : 1965 | HRIFA032506a : 2371 |
| HRIFA017312a : 1966 | HRIFA032511a : 2372 |
| HRIFA017456a : 1967 | HRIFA032530a : 2373 |
| HRIFA017457a : 1968 | HRIFA032587a : 2374 |
| HRIFA017509a : 1969 | HRIFA032605a : 2375 |
| HRIFA017594a : 1970 | HRIFA032642a : 2376 |
| HRIFA017643a : 1971 | HRIFA032696a : 2377 |
| HRIFA017670a : 1972 | HRIFA032730a : 2378 |
| HRIFA017703a : 1973 | HRIFA032820a : 2379 |
| HRIFA017729a : 1974 | HRIFA032984a : 2380 |
| HRIFA017791a : 1975 | HRIFA033349a : 2381 |
| HRIFA017801a : 1976 | HRIFA033718a : 2382 |
| HRIFA017818a : 1977 | HRIFA034010a : 2383 |
| HRIFA017836a : 1978 | |

| | |
|---|---|
| The names of the internal sequences that are used in the selection of the clones from the representative sequences, and the corresponding SEQ IDs. | |
| AA533598 : 2384 | HRIFA036799a : 2463 |
| AI051329 : 2385 | HRIFA037138a : 2464 |
| HRIFA000595a : 2386 | HRIFA037776a : 2465 |
| HRIFA000667a : 2387 | HRIFA037838a : 2466 |
| HRIFA000878a : 2388 | HRIRA000001a : 2467 |
| HRIFA001269a : 2389 | HRIRA000041a : 2468 |
| HRIFA001283a : 2390 | HRIRA000058a : 2469 |
| HRIFA002000a : 2391 | HRIRA000260a : 2470 |
| HRIFA002196a : 2392 | HRIRA000490a : 2471 |
| HRIFA003583a : 2393 | HRIRA000522a : 2472 |
| HRIFA005077a : 2394 | HRIRA000553a : 2473 |
| HRIFA005781a : 2395 | HRIRA000563a : 2474 |
| HRIFA006216a : 2396 | HRIRA000640a : 2475 |
| HRIFA006468a : 2397 | HRIRA000725a : 2476 |
| HRIFA006822a : 2398 | HRIRA000998a : 2477 |
| HRIFA007048a : 2399 | HRIRA001053a : 2478 |
| HRIFA007661a : 2400 | HRIRA001314a : 2479 |
| HRIFA007777a : 2401 | HRIRA001443a : 2480 |
| HRIFA007997a : 2402 | HRIRA001473a : 2481 |
| HRIFA008312a : 2403 | HRIRA001648a : 2482 |
| HRIFA009250a : 2404 | HRIRA001690a : 2483 |
| HRIFA009495a : 2405 | HRIRA001726a : 2484 |
| HRIFA009607a : 2406 | HRIRA001884a : 2485 |
| HRIFA009923a : 2407 | HRIRA002098a : 2486 |
| HRIFA009978a : 2408 | HRIRA002100a : 2487 |
| HRIFA010730a : 2409 | HRIRA002155a : 2488 |
| HRIFA011029a : 2410 | HRIRA002307a : 2489 |
| HRIFA011416a : 2411 | HRIRA002442a : 2490 |
| HRIFA011461a : 2412 | HRIRA002446a : 2491 |
| HRIFA012670a : 2413 | HRIRA002479a : 2492 |
| HRIFA012717a : 2414 | HRIRA002945a : 2493 |
| HRIFA012802a : 2415 | HRIRA003028a : 2494 |
| HRIFA013357a : 2416 | HRIRA003108a : 2495 |
| HRIFA013484a : 2417 | HRIRA003139a : 2496 |
| HRIFA015333a : 2418 | HRIRA003819a : 2497 |
| HRIFA015375a : 2419 | HRIRA004049a : 2498 |
| HRIFA015663a : 2420 | HRIRA004286a : 2499 |
| HRIFA016287a : 2421 | HRIRA004583a : 2500 |
| HRIFA016302a : 2422 | HRIRA004691a : 2501 |
| HRIFA016782a : 2423 | HRIRA004783a : 2502 |
| HRIFA018555a : 2424 | HRIRA005152a : 2503 |
| HRIFA019338a : 2425 | HRIRA005221a : 2504 |
| HRIFA020315a : 2426 | HRIRA005227a : 2505 |
| HRIFA020806a : 2427 | HRIRA005305a : 2506 |
| HRIFA022264a : 2428 | HRIRA005563a : 2507 |
| HRIFA022923a : 2429 | HRIRA006263a : 2508 |
| HRIFA023027a : 2430 | HRIRA006324a : 2509 |
| HRIFA023218a : 2431 | HRIRA006517a : 2510 |
| HRIFA023363a : 2432 | HRIRA006580a : 2511 |
| HRIFA023434a : 2433 | HRIRA007665a : 2512 |
| HRIFA023444a : 2434 | HRIRA007680a : 2513 |
| HRIFA023551a : 2435 | HRIRA008129a : 2514 |
| HRIFA023558a : 2436 | HRIRA008152a : 2515 |
| HRIFA023641a : 2437 | HRIRA008276a : 2516 |
| HRIFA023798a : 2438 | HRIRA008329a : 2517 |
| HRIFA024330a : 2439 | HRIRA008854a : 2518 |
| HRIFA024338a : 2440 | HRIRA008896a : 2519 |
| HRIFA024384a : 2441 | HRIRA008958a : 2520 |
| HRIFA024644a : 2442 | HRIRA009551a : 2521 |
| HRIFA025170a : 2443 | HRIRA009828a : 2522 |
| HRIFA025496a : 2444 | HRIRA010472a : 2523 |
| HRIFA025565a : 2445 | HRIRA012442a : 2524 |
| HRIFA025651a : 2446 | HRIRA012921a : 2525 |
| HRIFA026224a : 2447 | HRIRA013325a : 2526 |
| HRIFA026729a : 2448 | HRIRA013644a : 2527 |
| HRIFA026925a : 2449 | HRIRA013675a : 2528 |
| HRIFA028501a : 2450 | HRIRA013702a : 2529 |
| HRIFA029454a : 2451 | HRIRA013757a : 2530 |
| HRIFA030181a : 2452 | HRIRA013951a : 2531 |
| HRIFA032701a : 2453 | HRIRA014256a : 2532 |
| HRIFA032801a : 2454 | HRIRA014380a : 2533 |
| HRIFA033384a : 2455 | HRIRA015831a : 2534 |
| HRIFA033682a : 2456 | HRIRA015904a : 2535 |
| HRIFA033930a : 2457 | HRIRA016124a : 2536 |
| HRIFA034817a : 2458 | HRIRA017071a : 2537 |
| HRIFA035409a : 2459 | HRIRA018191a : 2538 |
| HRIFA035542a : 2460 | HRIRA020304a : 2539 |
| HRIFA035577a : 2461 | HRIRA000579a : 2540 |
| HRIFA036630a : 2462 | |

The internal sequences include EST, HRIFA(the representative sequence of the 5'-end), and HRIRA (the representative sequence of the 3'-end).

**Table 370**

| clone | the name of full-length nucleotide sequences | SEQ ID NO of the full-length nucleotide sequences | SEQ ID NO of the deduced amino acid sequences |
|---|---|---|---|
| HEMBA1000006 | C-HEMBA1000006 | 2547 | 2548 |
| nnnnnnnnnnnn | C-nnnnnnnnnnnn | nnnn | nnnn |
| HEMBA1000121 | C-HEMBA1000121 | 2551 | 2552 |
| HEMBA1000128 | C-HEMBA1000128 | 2553 | 2554 |
| HEMBA1000275 | C-HEMBA1000275 | 2555 | 2556 |
| HEMBA1000300 | C-HEMBA1000300 | 2557 | |
| HEMBA1000349 | C-HEMBA1000349 | 2558 | 2559 |
| HEMBA1000443 | C-HEMBA1000443 | 2560 | 2561 |
| HEMBA1000590 | C-HEMBA1000590 | 2562 | 2563 |
| HEMBA1000634 | C-HEMBA1000634 | 2564 | 2565 |
| HEMBA1000713 | C-HEMBA1000713 | 2566 | 2567 |
| HEMBA1000745 | C-HEMBA1000745 | 2568 | 2569 |
| HEMBA1000907 | C-HEMBA1000907 | 2570 | 2571 |
| HEMBA1000940 | C-HEMBA1000940 | 2572 | 2573 |
| HEMBA1000962 | C-HEMBA1000962 | 2574 | 2575 |
| HEMBA1001221 | C-HEMBA1001221 | 2576 | 2577 |
| HEMBA1001228 | C-HEMBA1001228 | 2578 | 2579 |
| HEMBA1001297 | C-HEMBA1001297 | 2580 | |
| HEMBA1001390 | C-HEMBA1001390 | 2581 | 2582 |
| HEMBA1001563 | C-HEMBA1001563 | 2583 | |
| HEMBA1001621 | C-HEMBA1001621 | 2584 | 2585 |
| nnnnnnnnnnnn | C-nnnnnnnnnnnn | nnnn | nnnn |
| HEMBA1001878 | C-HEMBA1001878 | 2588 | 2589 |
| HEMBA1002131 | C-HEMBA1002131 | 2590 | 2591 |
| HEMBA1002163 | C-HEMBA1002163 | 2592 | 2593 |
| HEMBA1002164 | C-HEMBA1002164 | 2594 | 2595 |
| HEMBA1002167 | C-HEMBA1002167 | 2596 | 2597 |
| HEMBA1002178 | C-HEMBA1002178 | 2598 | 2599 |
| nnnnnnnnnnnn | C-nnnnnnnnnnnn | nnnn | nnnn |
| HEMBA1002195 | C-HEMBA1002195 | 2602 | 2603 |
| HEMBA1002227 | C-HEMBA1002227 | 2604 | 2605 |
| HEMBA1002239 | C-HEMBA1002239 | 2606 | |
| HEMBA1002316 | C-HEMBA1002316 | 2607 | 2608 |
| HEMBA1002420 | C-HEMBA1002420 | 2609 | 2610 |
| HEMBA1002421 | C-HEMBA1002421 | 2611 | 2612 |
| HEMBA1002524 | C-HEMBA1002524 | 2613 | 2614 |
| HEMBA1002551 | C-HEMBA1002551 | 2615 | 2616 |
| HEMBA1002767 | C-HEMBA1002767 | 2617 | 2618 |
| HEMBA1002992 | C-HEMBA1002992 | 2619 | 2620 |
| HEMBA1003047 | C-HEMBA1003047 | 2621 | 2622 |
| HEMBA1003072 | C-HEMBA1003072 | 2623 | 2624 |
| HEMBA1003101 | C-HEMBA1003101 | 2625 | 2626 |
| HEMBA1003230 | C-HEMBA1003230 | 2627 | 2628 |
| HEMBA1003294 | C-HEMBA1003294 | 2629 | |
| HEMBA1003315 | C-HEMBA1003315 | 2630 | 2631 |
| HEMBA1003392 | C-HEMBA1003392 | 2632 | 2633 |
| HEMBA1003399 | C-HEMBA1003399 | 2634 | 2635 |
| HEMBA1003487 | C-HEMBA1003487 | 2636 | 2637 |
| HEMBA1003530 | C-HEMBA1003530 | 2638 | 2639 |
| HEMBA1003602 | C-HEMBA1003602 | 2640 | 2641 |
| HEMBA1003732 | C-HEMBA1003732 | 2642 | 2643 |
| HEMBA1003945 | C-HEMBA1003945 | 2644 | 2645 |
| HEMBA1004110 | C-HEMBA1004110 | 2646 | 2647 |
| HEMBA1004250 | C-HEMBA1004250 | 2648 | 2649 |
| HEMBA1004391 | C-HEMBA1004391 | 2650 | 2651 |
| HEMBA1004444 | C-HEMBA1004444 | 2652 | 2653 |
| HEMBA1004454 | C-HEMBA1004454 | 2654 | 2655 |
| HEMBA1004505 | C-HEMBA1004505 | 2656 | 2657 |
| HEMBA1004797 | C-HEMBA1004797 | 2658 | 2659 |
| HEMBA1004982 | C-HEMBA1004982 | 2660 | 2661 |
| HEMBA1005070 | C-HEMBA1005070 | 2662 | 2663 |
| HEMBA1005084 | C-HEMBA1005084 | 2664 | 2665 |
| HEMBA1005145 | C-HEMBA1005145 | 2666 | 2667 |
| HEMBA1005430 | C-HEMBA1005430 | 2668 | 2669 |
| HEMBA1005449 | C-HEMBA1005449 | 2670 | 2671 |
| HEMBA1005489 | C-HEMBA1005489 | 2672 | 2673 |
| HEMBA1005522 | C-HEMBA1005522 | 2674 | 2675 |
| HEMBA1005545 | C-HEMBA1005545 | 2676 | 2677 |
| HEMBA1005698 | C-HEMBA1005698 | 2678 | 2679 |
| HEMBA1005913 | C-HEMBA1005913 | 2680 | |
| HEMBA1005929 | C-HEMBA1005929 | 2681 | 2682 |
| HEMBA1005945 | C-HEMBA1005945 | 2683 | 2684 |
| HEMBA1006016 | C-HEMBA1006016 | 2685 | |
| HEMBA1006171 | C-HEMBA1006171 | 2686 | 2687 |
| HEMBA1006299 | C-HEMBA1006299 | 2688 | 2689 |
| HEMBA1006311 | C-HEMBA1006311 | 2690 | 2691 |
| HEMBA1006335 | C-HEMBA1006335 | 2692 | 2693 |
| HEMBA1006430 | C-HEMBA1006430 | 2694 | 2695 |
| HEMBA1006482 | C-HEMBA1006482 | 2696 | 2697 |
| HEMBA1006572 | C-HEMBA1006572 | 2698 | 2699 |
| HEMBA1006707 | C-HEMBA1006707 | 2700 | 2701 |
| HEMBA1006724 | C-HEMBA1006724 | 2702 | 2703 |
| HEMBA1006902 | C-HEMBA1006902 | 2704 | 2705 |
| HEMBA1006916 | C-HEMBA1006916 | 2706 | 2707 |
| HEMBA1006960 | C-HEMBA1006960 | 2708 | 2709 |
| HEMBA1007013 | C-HEMBA1007013 | 2710 | 2711 |
| HEMBA1007057 | C-HEMBA1007057 | 2712 | 2713 |
| HEMBA1007241 | C-HEMBA1007241 | 2714 | |
| HEMBA1007291 | C-HEMBA1007291 | 2715 | 2716 |
| HEMBA1007332 | C-HEMBA1007332 | 2717 | |
| HEMBB1000276 | C-HEMBB1000276 | 2718 | |
| HEMBB1000447 | C-HEMBB1000447 | 2719 | 2720 |
| HEMBB1000642 | C-HEMBB1000642 | 2721 | |
| HEMBB1000668 | C-HEMBB1000668 | 2722 | 2723 |
| HEMBB1000679 | C-HEMBB1000679 | 2724 | 2725 |
| HEMBB1000881 | C-HEMBB1000881 | 2726 | 2727 |
| HEMBB1000905 | C-HEMBB1000905 | 2728 | 2729 |
| HEMBB1001026 | C-HEMBB1001026 | 2730 | 2731 |
| HEMBB1001048 | C-HEMBB1001048 | 2732 | 2733 |
| HEMBB1001200 | C-HEMBB1001200 | 2734 | |
| HEMBB1001407 | C-HEMBB1001407 | 2735 | 2736 |
| HEMBB1001530 | C-HEMBB1001530 | 2737 | 2738 |
| HEMBB1001573 | C-HEMBB1001573 | 2739 | 2740 |
| nnnnnnnnnnnn | C-nnnnnnnnnnnn | nnnn | nnnn |
| HEMBB1001847 | C-HEMBB1001847 | 2743 | 2744 |
| HEMBB1001978 | C-HEMBB1001978 | 2745 | 2746 |
| HEMBB1002162 | C-HEMBB1002162 | 2747 | 2748 |
| HEMBB1002228 | C-HEMBB1002228 | 2749 | |
| HEMBB1002245 | C-HEMBB1002245 | 2750 | 2751 |
| HEMBB1002427 | C-HEMBB1002427 | 2752 | 2753 |
| HEMBB1002465 | C-HEMBB1002465 | 2754 | 2755 |
| HEMBB1002663 | C-HEMBB1002663 | 2756 | 2757 |
| HEMBB1002693 | C-HEMBB1002693 | 2758 | 2759 |
| MAMMA1000046 | C-MAMMA1000046 | 2760 | |
| MAMMA1000118 | C-MAMMA1000118 | 2761 | 2762 |
| nnnnnnnnnnnn | C-nnnnnnnnnnnn | nnnn | nnnn |
| MAMMA1000449 | C-MAMMA1000449 | 2765 | |
| MAMMA1000457 | C-MAMMA1000457 | 2766 | 2767 |
| MAMMA1000591 | C-MAMMA1000591 | 2768 | 2769 |
| MAMMA1000681 | C-MAMMA1000681 | 2770 | 2771 |
| MAMMA1001043 | C-MAMMA1001043 | 2772 | 2773 |
| MAMMA1001893 | C-MAMMA1001893 | 2774 | 2775 |
| NT2RM2000241 | C-NT2RM2000241 | 2776 | 2777 |
| NT2RM2000306 | C-NT2RM2000306 | 2778 | 2779 |
| NT2RM2000410 | C-NT2RM2000410 | 2780 | 2781 |
| NT2RM2000423 | C-NT2RM2000423 | 2782 | 2783 |
| NT2RM2000497 | C-NT2RM2000497 | 2784 | 2785 |
| NT2RM2000514 | C-NT2RM2000514 | 2786 | 2787 |
| NT2RM2000622 | C-NT2RM2000622 | 2788 | 2789 |
| NT2RM2001126 | C-NT2RM2001126 | 2790 | 2791 |
| NT2RM2001902 | C-NT2RM2001902 | 2792 | 2793 |
| NT2RM2001939 | C-NT2RM2001939 | 2794 | 2795 |
| NT2RM2001941 | C-NT2RM2001941 | 2796 | 2797 |
| NT2RM4000198 | C-NT2RM4000198 | 2798 | 2799 |
| NT2RM4000284 | C-NT2RM4000284 | 2800 | 2801 |
| NT2RM4000295 | C-NT2RM4000295 | 2802 | 2803 |
| NT2RM4000326 | C-NT2RM4000326 | 2804 | 2805 |
| NT2RM4000444 | C-NT2RM4000444 | 2806 | 2807 |
| NT2RM4000587 | C-NT2RM4000587 | 2808 | 2809 |
| NT2RM4000648 | C-NT2RM4000648 | 2810 | 2811 |
| NT2RM4000997 | C-NT2RM4000997 | 2812 | 2813 |
| NT2RM4001321 | C-NT2RM4001321 | 2814 | 2815 |
| NT2RM4001325 | C-NT2RM4001325 | 2816 | 2817 |
| NT2RM4001735 | C-NT2RM4001735 | 2818 | 2819 |
| NT2RM4002352 | C-NT2RM4002352 | 2820 | 2821 |
| NT2RP1000002 | C-NT2RP1000002 | 2822 | 2823 |
| NT2RP1000050 | C-NT2RP1000050 | 2824 | 2825 |
| NT2RP1000181 | C-NT2RP1000181 | 2826 | 2827 |
| NT2RP1000261 | C-NT2RP1000261 | 2828 | 2829 |
| NT2RP1000300 | C-NT2RP1000300 | 2830 | 2831 |
| NT2RP1000325 | C-NT2RP1000325 | 2832 | 2833 |
| NT2RP1000448 | C-NT2RP1000448 | 2834 | 2835 |
| NT2RP1000551 | C-NT2RP1000551 | 2836 | 2837 |
| NT2RP1000579 | C-NT2RP1000579 | 2838 | 2839 |
| NT2RP1000613 | C-NT2RP1000613 | 2840 | 2841 |
| NT2RP1000903 | C-NT2RP1000903 | 2842 | 2843 |
| NT2RP1000981 | C-NT2RP1000981 | 2844 | 2845 |
| NT2RP1001004 | C-NT2RP1001004 | 2846 | 2847 |
| NT2RP1001020 | C-NT2RP1001020 | 2848 | 2849 |
| NT2RP1001563 | C-NT2RP1001563 | 2850 | 2851 |
| NT2RP2000394 | C-NT2RP2000394 | 2852 | 2853 |
| NT2RP2000479 | C-NT2RP2000479 | 2854 | 2855 |
| NT2RP2000514 | C-NT2RP2000514 | 2856 | 2857 |
| NT2RP2000533 | C-NT2RP2000533 | 2858 | 2859 |
| NT2RP2000649 | C-NT2RP2000649 | 2860 | 2861 |
| NT2RP2000663 | C-NT2RP2000663 | 2862 | 2863 |
| NT2RP2000694 | C-NT2RP2000694 | 2864 | 2865 |
| NT2RP2000903 | C-NT2RP2000903 | 2866 | 2867 |
| NT2RP2001480 | C-NT2RP2001480 | 2868 | 2869 |
| NT2RP2001495 | C-NT2RP2001495 | 2870 | 2871 |
| NT2RP2001514 | C-NT2RP2001514 | 2872 | 2873 |
| NT2RP2001529 | C-NT2RP2001529 | 2874 | 2875 |
| NT2RP2001769 | C-NT2RP2001769 | 2876 | 2877 |
| NT2RP2001878 | C-NT2RP2001878 | 2878 | 2879 |
| NT2RP2001903 | C-NT2RP2001903 | 2880 | 2881 |
| NT2RP2001915 | C-NT2RP2001915 | 2882 | 2883 |
| NT2RP2001956 | C-NT2RP2001956 | 2884 | 2885 |
| NT2RP2002063 | C-NT2RP2002063 | 2886 | 2887 |
| NT2RP2002188 | C-NT2RP2002188 | 2888 | 2889 |
| NT2RP2002232 | C-NT2RP2002232 | 2890 | 2891 |
| NT2RP2002304 | C-NT2RP2002304 | 2892 | 2893 |
| NT2RP2002409 | C-NT2RP2002409 | 2894 | 2895 |
| NT2RP2002510 | C-NT2RP2002510 | 2896 | 2897 |
| NT2RP2002527 | C-NT2RP2002527 | 2898 | 2899 |
| NT2RP2002533 | C-NT2RP2002533 | 2900 | 2901 |
| NT2RP2002564 | C-NT2RP2002564 | 2902 | 2903 |
| NT2RP2002674 | C-NT2RP2002674 | 2904 | 2905 |
| NT2RP2002721 | C-NT2RP2002721 | 2906 | 2907 |
| NT2RP2002824 | C-NT2RP2002824 | 2908 | 2909 |
| NT2RP2002942 | C-NT2RP2002942 | 2910 | 2911 |
| NT2RP2002974 | C-NT2RP2002974 | 2912 | 2913 |
| NT2RP2002976 | C-NT2RP2002976 | 2914 | 2915 |
| NT2RP2003042 | C-NT2RP2003042 | 2916 | 2917 |
| NT2RP2003179 | C-NT2RP2003179 | 2918 | 2919 |
| NT2RP2003210 | C-NT2RP2003210 | 2920 | 2921 |
| NT2RP2003369 | C-NT2RP2003369 | 2922 | 2923 |
| NT2RP2003383 | C-NT2RP2003383 | 2924 | 2925 |
| NT2RP2003469 | C-NT2RP2003469 | 2926 | 2927 |
| NT2RP2003545 | C-NT2RP2003545 | 2928 | 2929 |
| NT2RP2003593 | C-NT2RP2003593 | 2930 | 2931 |
| NT2RP2003599 | C-NT2RP2003599 | 2932 | 2933 |
| NT2RP2003655 | C-NT2RP2003655 | 2934 | 2935 |
| NT2RP2003931 | C-NT2RP2003931 | 2936 | 2937 |
| NT2RP2004141 | C-NT2RP2004141 | 2938 | 2939 |
| NT2RP2004179 | C-NT2RP2004179 | 2940 | 2941 |
| NT2RP2004205 | C-NT2RP2004205 | 2942 | 2943 |
| NT2RP2004447 | C-NT2RP2004447 | 2944 | 2945 |
| NT2RP2004495 | C-NT2RP2004495 | 2946 | 2947 |
| NT2RP2004524 | C-NT2RP2004524 | 2948 | 2949 |
| NT2RP2004556 | C-NT2RP2004556 | 2950 | 2951 |
| NT2RP2004606 | C-NT2RP2004606 | 2952 | 2953 |
| NT2RP2004648 | C-NT2RP2004648 | 2954 | 2955 |
| NT2RP2004670 | C-NT2RP2004670 | 2956 | 2957 |
| NT2RP2004794 | C-NT2RP2004794 | 2958 | 2959 |
| NT2RP2004837 | C-NT2RP2004837 | 2960 | 2961 |
| NT2RP2004847 | C-NT2RP2004847 | 2962 | 2963 |
| nnnnnnnnnnnn | C-nnnnnnnnnnnn | nnnn | nnnn |
| NT2RP2005027 | C-NT2RP2005027 | 2966 | |
| NT2RP2005163 | C-NT2RP2005163 | 2967 | 2968 |
| NT2RP2005181 | C-NT2RP2005181 | 2969 | 2970 |
| NT2RP2005247 | C-NT2RP2005247 | 2971 | 2972 |
| NT2RP2005425 | C-NT2RP2005425 | 2973 | 2974 |
| NT2RP2005463 | C-NT2RP2005463 | 2975 | 2976 |
| NT2RP2005514 | C-NT2RP2005514 | 2977 | 2978 |
| NT2RP2005541 | C-NT2RP2005541 | 2979 | 2980 |
| NT2RP2005632 | C-NT2RP2005632 | 2981 | 2982 |
| NT2RP2005878 | C-NT2RP2005878 | 2983 | 2984 |
| NT2RP2005883 | C-NT2RP2005883 | 2985 | 2986 |
| NT2RP2005887 | C-NT2RP2005887 | 2987 | 2988 |
| NT2RP2005941 | C-NT2RP2005941 | 2989 | 2990 |
| NT2RP2005994 | C-NT2RP2005994 | 2991 | 2992 |
| NT2RP2006042 | C-NT2RP2006042 | 2993 | 2994 |
| NT2RP2006269 | C-NT2RP2006269 | 2995 | 2996 |
| NT2RP2006512 | C-NT2RP2006512 | 2997 | 2998 |
| NT2RP3000059 | C-NT2RP3000059 | 2999 | 3000 |
| NT2RP3000063 | C-NT2RP3000063 | 3001 | 3002 |
| NT2RP3000125 | C-NT2RP3000125 | 3003 | 3004 |
| NT2RP3000169 | C-NT2RP3000169 | 3005 | 3006 |
| NT2RP3000172 | C-NT2RP3000172 | 3007 | 3008 |
| NT2RP3000201 | C-NT2RP3000201 | 3009 | 3010 |
| NT2RP3000436 | C-NT2RP3000436 | 3011 | 3012 |
| NT2RP3000460 | C-NT2RP3000460 | 3013 | 3014 |
| NT2RP3000616 | C-NT2RP3000616 | 3015 | 3016 |
| NT2RP3000721 | C-NT2RP3000721 | 3017 | 3018 |
| NT2RP3000820 | C-NT2RP3000820 | 3019 | 3020 |
| NT2RP3000871 | C-NT2RP3000871 | 3021 | 3022 |
| NT2RP3000907 | C-NT2RP3000907 | 3023 | 3024 |
| NT2RP3001012 | C-NT2RP3001012 | 3025 | 3026 |
| NT2RP3001044 | C-NT2RP3001044 | 3027 | 3028 |
| NT2RP3001061 | C-NT2RP3001061 | 3029 | 3030 |
| NT2RP3001170 | C-NT2RP3001170 | 3031 | 3032 |
| NT2RP3001195 | C-NT2RP3001195 | 3033 | 3034 |
| NT2RP3001240 | C-NT2RP3001240 | 3035 | 3036 |
| NT2RP3001322 | C-NT2RP3001322 | 3037 | 3038 |
| NT2RP3001388 | C-NT2RP3001388 | 3039 | 3040 |
| nnnnnnnnnnnn | C-nnnnnnnnnnnn | nnnn | nnnn |
| nnnnnnnnnnnn | C-nnnnnnnnnnnn | nnnn | nnnn |
| NT2RP3001560 | C-NT2RP3001560 | 3045 | 3046 |
| NT2RP3001592 | C-NT2RP3001592 | 3047 | 3048 |
| NT2RP3001738 | C-NT2RP3001738 | 3049 | 3050 |
| NT2RP3001754 | C-NT2RP3001754 | 3051 | 3052 |
| NT2RP3001858 | C-NT2RP3001858 | 3053 | 3054 |
| NT2RP3002160 | C-NT2RP3002160 | 3055 | 3056 |
| NT2RP3002311 | C-NT2RP3002311 | 3057 | 3058 |
| NT2RP3002342 | C-NT2RP3002342 | 3059 | 3060 |
| NT2RP3002448 | C-NT2RP3002448 | 3061 | 3062 |
| NT2RP3002721 | C-NT2RP3002721 | 3063 | 3064 |
| NT2RP3002738 | C-NT2RP3002738 | 3065 | 3066 |
| NT2RP3002790 | C-NT2RP3002790 | 3067 | 3068 |
| NT2RP3002836 | C-NT2RP3002836 | 3069 | 3070 |
| NT2RP3002958 | C-NT2RP3002958 | 3071 | 3072 |
| NT2RP3003000 | C-NT2RP3003000 | 3073 | 3074 |
| NT2RP3003076 | C-NT2RP3003076 | 3075 | 3076 |
| NT2RP3003354 | C-NT2RP3003354 | 3077 | 3078 |
| NT2RP3003469 | C-NT2RP3003469 | 3079 | 3080 |
| NT2RP3003527 | C-NT2RP3003527 | 3081 | 3082 |
| NT2RP3003535 | C-NT2RP3003535 | 3083 | 3084 |
| NT2RP3003559 | C-NT2RP3003559 | 3085 | 3086 |
| NT2RP3003614 | C-NT2RP3003614 | 3087 | 3088 |
| NT2RP3003729 | C-NT2RP3003729 | 3089 | 3090 |
| NT2RP3003849 | C-NT2RP3003849 | 3091 | 3092 |
| NT2RP3003874 | C-NT2RP3003874 | 3093 | 3094 |
| NT2RP3003963 | C-NT2RP3003963 | 3095 | 3096 |
| NT2RP3004000 | C-NT2RP3004000 | 3097 | 3098 |
| NT2RP3004075 | C-NT2RP3004075 | 3099 | 3100 |
| NT2RP3004083 | C-NT2RP3004083 | 3101 | 3102 |
| NT2RP3004090 | C-NT2RP3004090 | 3103 | 3104 |
| NT2RP3004130 | C-NT2RP3004130 | 3105 | 3106 |
| NT2RP3004133 | C-NT2RP3004133 | 3107 | 3108 |
| NT2RP3004202 | C-NT2RP3004202 | 3109 | 3110 |
| NT2RP3004309 | C-NT2RP3004309 | 3111 | 3112 |
| NT2RP3004321 | C-NT2RP3004321 | 3113 | 3114 |
| NT2RP3004355 | C-NT2RP3004355 | 3115 | 3116 |
| NT2RP3004374 | C-NT2RP3004374 | 3117 | 3118 |
| NT2RP3004406 | C-NT2RP3004406 | 3119 | 3120 |
| NT2RP3004552 | C-NT2RP3004552 | 3121 | 3122 |
| NT2RP3004557 | C-NT2RP3004557 | 3123 | 3124 |
| NT2RP3004625 | C-NT2RP3004625 | 3125 | 3126 |
| NT2RP3004640 | C-NT2RP3004640 | 3127 | 3128 |
| NT2RP3004647 | C-NT2RP3004647 | 3129 | 3130 |
| NT2RP4000108 | C-NT2RP4000108 | 3131 | 3132 |
| NT2RP4000962 | C-NT2RP4000962 | 3133 | 3134 |
| NT2RP4001009 | C-NT2RP4001009 | 3135 | 3136 |
| NT2RP4001467 | C-NT2RP4001467 | 3137 | 3138 |
| OVARC1000090 | C-OVARC1000090 | 3139 | |
| OVARC1000105 | C-OVARC1000105 | 3140 | 3141 |
| OVARC1000137 | C-OVARC1000137 | 3142 | |
| OVARC1000208 | C-OVARC1000208 | 3143 | |
| OVARC1000255 | C-OVARC1000255 | 3144 | 3145 |
| OVARC1000275 | C-OVARC1000275 | 3146 | 3147 |
| OVARC1000298 | C-OVARC1000298 | 3148 | 3149 |
| OVARC1000410 | C-OVARC1000410 | 3150 | 3151 |
| OVARC1000439 | C-OVARC1000439 | 3152 | 3153 |
| OVARC1000467 | C-OVARC1000467 | 3154 | 3155 |
| OVARC1000529 | C-OVARC1000529 | 3156 | 3157 |
| OVARC1000775 | C-OVARC1000775 | 3158 | 3159 |
| nnnnnnnnnnnn | C-nnnnnnnnnnnn | nnnn | nnnn |
| OVARC1000811 | C-OVARC1000811 | 3162 | 3163 |
| OVARC1000853 | C-OVARC1000853 | 3164 | 3165 |
| OVARC1000916 | C-OVARC1000916 | 3166 | 3167 |
| OVARC1000956 | C-OVARC1000956 | 3168 | 3169 |
| OVARC1001030 | C-OVARC1001030 | 3170 | 3171 |
| OVARC1001049 | C-OVARC1001049 | 3172 | 3173 |
| OVARC1001086 | C-OVARC1001086 | 3174 | 3175 |
| OVARC1001132 | C-OVARC1001132 | 3176 | 3177 |
| OVARC1001163 | C-OVARC1001163 | 3178 | 3179 |
| OVARC1001222 | C-OVARC1001222 | 3180 | |
| OVARC1001338 | C-OVARC1001338 | 3181 | 3182 |
| OVARC1001569 | C-OVARC1001569 | 3183 | 3184 |
| OVARC1001596 | C-OVARC1001596 | 3185 | 3186 |
| OVARC1001725 | C-OVARC1001725 | 3187 | |
| OVARC1001727 | C-OVARC1001727 | 3188 | |
| OVARC1001807 | C-OVARC1001807 | 3189 | 3190 |
| OVARC1001991 | C-OVARC1001991 | 3191 | 3192 |
| OVARC1002058 | C-OVARC1002058 | 3193 | 3194 |
| OVARC1002178 | C-OVARC1002178 | 3195 | |
| PLACE1000033 | C-PLACE1000033 | 3196 | 3197 |
| PLACE1000231 | C-PLACE1000231 | 3198 | 3199 |
| PLACE1000258 | C-PLACE1000258 | 3200 | 3201 |
| PLACE1000442 | C-PLACE1000442 | 3202 | 3203 |
| PLACE1000560 | C-PLACE1000560 | 3204 | 3205 |
| PLACE1000740 | C-PLACE1000740 | 3206 | 3207 |
| PLACE1000912 | C-PLACE1000912 | 3208 | 3209 |
| PLACE1000914 | C-PLACE1000914 | 3210 | 3211 |
| PLACE1000927 | C-PLACE1000927 | 3212 | 3213 |
| PLACE1001016 | C-PLACE1001016 | 3214 | 3215 |
| nnnnnnnnnnnn | C-nnnnnnnnnnnn | nnnn | nnnn |
| PLACE1001100 | C-PLACE1001100 | 3218 | |
| PLACE1001114 | C-PLACE1001114 | 3219 | 3220 |
| PLACE1001123 | C-PLACE1001123 | 3221 | |
| PLACE1001183 | C-PLACE1001183 | 3222 | 3223 |
| PLACE1001229 | C-PLACE1001229 | 3224 | 3225 |
| PLACE1001231 | C-PLACE1001231 | 3226 | 3227 |
| nnnnnnnnnnnn | C-nnnnnnnnnnnn | nnnn | nnnn |
| PLACE1001340 | C-PLACE1001340 | 3230 | 3231 |
| PLACE1001401 | C-PLACE1001401 | 3232 | 3233 |
| PLACE1001407 | C-PLACE1001407 | 3234 | 3235 |
| PLACE1001464 | C-PLACE1001464 | 3236 | 3237 |
| PLACE1001500 | C-PLACE1001500 | 3238 | 3239 |
| PLACE1001516 | C-PLACE1001516 | 3240 | 3241 |
| PLACE1001536 | C-PLACE1001536 | 3242 | 3243 |
| PLACE1001564 | C-PLACE1001564 | 3244 | 3245 |
| PLACE1001655 | C-PLACE1001655 | 3246 | 3247 |
| nnnnnnnnnnnn | C-nnnnnnnnnnnn | nnnn | nnnn |
| PLACE1001788 | C-PLACE1001788 | 3250 | 3251 |
| PLACE1001795 | C-PLACE1001795 | 3252 | 3253 |
| PLACE1001836 | C-PLACE1001836 | 3254 | 3255 |
| PLACE1001918 | C-PLACE1001918 | 3256 | 3257 |
| PLACE1001949 | C-PLACE1001949 | 3258 | 3259 |
| PLACE1002080 | C-PLACE1002080 | 3260 | 3261 |
| PLACE1002095 | C-PLACE1002095 | 3262 | 3263 |
| PLACE1002153 | C-PLACE1002153 | 3264 | 3265 |
| PLACE1002329 | C-PLACE1002329 | 3266 | 3267 |
| PLACE1002355 | C-PLACE1002355 | 3268 | 3269 |
| PLACE1002374 | C-PLACE1002374 | 3270 | 3271 |
| PLACE1002518 | C-PLACE1002518 | 3272 | 3273 |
| PLACE1002547 | C-PLACE1002547 | 3274 | 3275 |
| PLACE1002726 | C-PLACE1002726 | 3276 | 3277 |
| PLACE1002905 | C-PLACE1002905 | 3278 | 3279 |
| PLACE1002911 | C-PLACE1002911 | 3280 | 3281 |
| PLACE1002967 | C-PLACE1002967 | 3282 | 3283 |
| PLACE1003135 | C-PLACE1003135 | 3284 | 3285 |
| PLACE1003163 | C-PLACE1003163 | 3286 | 3287 |
| PLACE1003428 | C-PLACE1003428 | 3288 | 3289 |
| PLACE1003438 | C-PLACE1003438 | 3290 | 3291 |
| PLACE1003460 | C-PLACE1003460 | 3292 | 3293 |
| PLACE1003529 | C-PLACE1003529 | 3294 | 3295 |
| PLACE1003573 | C-PLACE1003573 | 3296 | 3297 |
| PLACE1003598 | C-PLACE1003598 | 3298 | 3299 |
| PLACE1003644 | C-PLACE1003644 | 3300 | |
| PLACE1003737 | C-PLACE1003737 | 3301 | 3302 |
| PLACE1003772 | C-PLACE1003772 | 3303 | 3304 |
| PLACE1003839 | C-PLACE1003839 | 3305 | 3306 |
| PLACE1003845 | C-PLACE1003845 | 3307 | 3308 |
| PLACE1003852 | C-PLACE1003852 | 3309 | 3310 |
| PLACE1004028 | C-PLACE1004028 | 3311 | |
| PLACE1004166 | C-PLACE1004166 | 3312 | 3313 |
| PLACE1004168 | C-PLACE1004168 | 3314 | 3315 |
| PLACE1004199 | C-PLACE1004199 | 3316 | 3317 |
| PLACE1004279 | C-PLACE1004279 | 3318 | 3319 |
| PLACE1004282 | C-PLACE1004282 | 3320 | 3321 |
| PLACE1004305 | C-PLACE1004305 | 3322 | 3323 |
| PLACE1004441 | C-PLACE1004441 | 3324 | 3325 |
| PLACE1004450 | C-PLACE1004450 | 3326 | 3327 |
| PLACE1004482 | C-PLACE1004482 | 3328 | 3329 |
| PLACE1004519 | C-PLACE1004519 | 3330 | 3331 |
| PLACE1004520 | C-PLACE1004520 | 3332 | 3333 |
| PLACE1004630 | C-PLACE1004630 | 3334 | 3335 |
| PLACE1004637 | C-PLACE1004637 | 3336 | 3337 |
| PLACE1004648 | C-PLACE1004648 | 3338 | 3339 |
| nnnnnnnnnnnn | C-nnnnnnnnnnnn | nnnn | nnnn |
| PLACE1004816 | C-PLACE1004816 | 3342 | 3343 |
| PLACE1004887 | C-PLACE1004887 | 3344 | 3345 |
| PLACE1005003 | C-PLACE1005003 | 3346 | 3347 |
| PLACE1005005 | C-PLACE1005005 | 3348 | 3349 |
| PLACE1005031 | C-PLACE1005031 | 3350 | 3351 |
| PLACE1005239 | C-PLACE1005239 | 3352 | 3353 |
| PLACE1005250 | C-PLACE1005250 | 3354 | 3355 |
| PLACE1005383 | C-PLACE1005383 | 3356 | 3357 |
| PLACE1005410 | C-PLACE1005410 | 3358 | 3359 |
| PLACE1005426 | C-PLACE1005426 | 3360 | 3361 |
| PLACE1005519 | C-PLACE1005519 | 3362 | 3363 |
| PLACE1005544 | C-PLACE1005544 | 3364 | 3365 |
| PLACE1005660 | C-PLACE1005660 | 3366 | 3367 |
| PLACE1005669 | C-PLACE1005669 | 3368 | 3369 |
| PLACE1005682 | C-PLACE1005682 | 3370 | 3371 |
| PLACE1005725 | C-PLACE1005725 | 3372 | 3373 |
| PLACE1005736 | C-PLACE1005736 | 3374 | 3375 |
| PLACE1005768 | C-PLACE1005768 | 3376 | 3377 |
| PLACE1005878 | C-PLACE1005878 | 3378 | 3379 |
| PLACE1006093 | C-PLACE1006093 | 3380 | 3381 |
| PLACE1006208 | C-PLACE1006208 | 3382 | 3383 |
| PLACE1006219 | C-PLACE1006219 | 3384 | 3385 |
| PLACE1006277 | C-PLACE1006277 | 3386 | 3387 |
| PLACE1006290 | C-PLACE1006290 | 3388 | 3389 |
| PLACE1006443 | C-PLACE1006443 | 3390 | 3391 |
| PLACE1006515 | C-PLACE1006515 | 3392 | 3393 |
| PLACE1006716 | C-PLACE1006716 | 3394 | 3395 |
| PLACE1006809 | C-PLACE1006809 | 3396 | 3397 |
| PLACE1006959 | C-PLACE1006959 | 3398 | 3399 |
| PLACE1007028 | C-PLACE1007028 | 3400 | 3401 |
| PLACE1007040 | C-PLACE1007040 | 3402 | 3403 |
| PLACE1007096 | C-PLACE1007096 | 3404 | 3405 |
| nnnnnnnnnnnn | C-nnnnnnnnnnnn | nnnn | nnnn |
| PLACE1007296 | C-PLACE1007296 | 3408 | 3409 |
| PLACE1007591 | C-PLACE1007591 | 3410 | |
| PLACE1007626 | C-PLACE1007626 | 3411 | 3412 |
| PLACE1007702 | C-PLACE1007702 | 3413 | 3414 |
| PLACE1007845 | C-PLACE1007845 | 3415 | 3416 |
| PLACE1007881 | C-PLACE1007881 | 3417 | 3418 |
| PLACE1008297 | C-PLACE1008297 | 3419 | 3420 |
| nnnnnnnnnnnn | C-nnnnnnnnnnnn | nnnn | nnnn |
| PLACE1008469 | C-PLACE1008469 | 3423 | 3424 |
| PLACE1008549 | C-PLACE1008549 | 3425 | 3426 |
| PLACE1008657 | C-PLACE1008657 | 3427 | 3428 |
| PLACE1008716 | C-PLACE1008716 | 3429 | 3430 |
| PLACE1008984 | C-PLACE1008984 | 3431 | 3432 |
| PLACE1008985 | C-PLACE1008985 | 3433 | 3434 |
| PLACE1009067 | C-PLACE1009067 | 3435 | 3436 |
| PLACE1009196 | C-PLACE1009196 | 3437 | 3438 |
| PLACE1009279 | C-PLACE1009279 | 3439 | 3440 |
| PLACE1009527 | C-PLACE1009527 | 3441 | 3442 |
| PLACE1009546 | C-PLACE1009546 | 3443 | 3444 |
| PLACE1009600 | C-PLACE1009600 | 3445 | 3446 |
| PLACE1009735 | C-PLACE1009735 | 3447 | 3448 |
| PLACE1009982 | C-PLACE1009982 | 3449 | 3450 |
| PLACE1010078 | C-PLACE1010078 | 3451 | 3452 |
| PLACE1010081 | C-PLACE1010081 | 3453 | 3454 |
| PLACE1010251 | C-PLACE1010251 | 3455 | 3456 |
| PLACE1010784 | C-PLACE1010784 | 3457 | 3458 |
| PLACE1010827 | C-PLACE1010827 | 3459 | 3460 |
| PLACE1010968 | C-PLACE1010968 | 3461 | 3462 |
| PLACE1011045 | C-PLACE1011045 | 3463 | 3464 |
| PLACE1011116 | C-PLACE1011116 | 3465 | 3466 |
| PLACE1011236 | C-PLACE1011236 | 3467 | 3468 |
| PLACE1011407 | C-PLACE1011407 | 3469 | 3470 |
| PLACE1011516 | C-PLACE1011516 | 3471 | 3472 |
| PLACE1011708 | C-PLACE1011708 | 3473 | 3474 |
| PLACE1011824 | C-PLACE1011824 | 3475 | 3476 |
| PLACE1011978 | C-PLACE1011978 | 3477 | 3478 |
| PLACE2000118 | C-PLACE2000118 | 3479 | 3480 |
| PLACE2000219 | C-PLACE2000219 | 3481 | |
| SKNMC1000004 | C-SKNMC1000004 | 3482 | 3483 |
| THYRO1000036 | C-THYRO1000036 | 3484 | 3485 |
| THYRO1000061 | C-THYRO1000061 | 3486 | 3487 |
| THYRO1000099 | C-THYRO1000099 | 3488 | 3489 |
| THYRO1000196 | C-THYRO1000196 | 3490 | 3491 |
| THYRO1000400 | C-THYRO1000400 | 3492 | 3493 |
| THYRO1000580 | C-THYRO1000580 | 3494 | 3495 |
| THYRO1000584 | C-THYRO1000584 | 3496 | 3497 |
| THYRO1000678 | C-THYRO1000678 | 3498 | 3499 |
| THYRO1000795 | C-THYRO1000795 | 3500 | 3501 |
| THYRO1000846 | C-THYRO1000846 | 3502 | 3503 |
| THYRO1000866 | C-THYRO1000866 | 3504 | 3505 |
| THYRO1000956 | C-THYRO1000956 | 3506 | 3507 |
| THYRO1000999 | C-THYRO1000999 | 3508 | |
| THYRO1001063 | C-THYRO1001063 | 3509 | 3510 |
| THYRO1001071 | C-THYRO1001071 | 3511 | 3512 |
| THYRO1001102 | C-THYRO1001102 | 3513 | 3514 |
| THYRO1001113 | C-THYRO1001113 | 3515 | 3516 |
| THYRO1001128 | C-THYRO1001128 | 3517 | 3518 |
| THYRO1001205 | C-THYRO1001205 | 3519 | 3520 |
| THYRO1001237 | C-THYRO1001237 | 3521 | 3522 |
| THYRO1001266 | C-THYRO1001266 | 3523 | 3524 |
| THYRO1001327 | C-THYRO1001327 | 3525 | 3526 |
| THYRO1001456 | C-THYRO1001456 | 3527 | 3528 |
| THYRO1001457 | C-THYRO1001457 | 3529 | 3530 |
| THYRO1001471 | C-THYRO1001471 | 3531 | 3532 |
| THYRO1001478 | C-THYRO1001478 | 3533 | 3534 |
| THYRO1001495 | C-THYRO1001495 | 3535 | 3536 |
| THYRO1001523 | C-THYRO1001523 | 3537 | 3538 |
| THYRO1001529 | C-THYRO1001529 | 3539 | 3540 |
| THYRO1001700 | C-THYRO1001700 | 3541 | 3542 |
| THYRO1001702 | C-THYRO1001702 | 3543 | 3544 |
| THYRO1001725 | C-THYRO1001725 | 3545 | 3546 |
| THYRO1001803 | C-THYRO1001803 | 3547 | 3548 |
| Y79AA1000127 | C-Y79AA1000127 | 3549 | 3550 |
| Y79AA1000207 | C-Y79AA1000207 | 3551 | 3552 |
| Y79AA1000226 | C-Y79AA1000226 | 3553 | 3554 |
| Y79AA1000270 | C-Y79AA1000270 | 3555 | 3556 |
| Y79AA1000426 | C-Y79AA1000426 | 3557 | 3558 |
| Y79AA1000521 | C-Y79AA1000521 | 3559 | 3560 |
| Y79AA1000776 | C-Y79AA1000776 | 3561 | 3562 |
| Y79AA1000777 | C-Y79AA1000777 | 3563 | 3564 |
| nnnnnnnnnnnn | C-nnnnnnnnnnnn | nnnn | nnnn |
| Y79AA1000876 | C-Y79AA1000876 | 3567 | 3568 |
| Y79AA1000959 | C-Y79AA1000959 | 3569 | 3570 |
| Y79AA1000967 | C-Y79AA1000967 | 3571 | 3572 |
| Y79AA1001013 | C-Y79AA1001013 | 3573 | 3574 |
| Y79AA1001056 | C-Y79AA1001056 | 3575 | 3576 |
| Y79AA1001062 | C-Y79AA1001062 | 3577 | 3578 |
| Y79AA1001090 | C-Y79AA1001090 | 3579 | 3580 |
| Y79AA1001264 | C-Y79AA1001264 | 3581 | 3582 |
| Y79AA1001272 | C-Y79AA1001272 | 3583 | 3584 |
| Y79AA1001328 | C-Y79AA1001328 | 3585 | 3586 |
| Y79AA1001430 | C-Y79AA1001430 | 3587 | 3588 |
| Y79AA1002022 | C-Y79AA1002022 | 3589 | 3590 |
| BNGH41000020 | C-BNGH41000020 | 3595 | 3596 |
| BNGH41000091 | C-BNGH41000091 | 3597 | 3598 |
| HEMBA1000462 | C-HEMBA1000462 | 3599 | 3600 |
| HEMBA1000477 | C-HEMBA1000477 | 3601 | 3602 |
| HEMBA1000671 | C-HEMBA1000671 | 3603 | 3604 |
| HEMBA1000732 | C-HEMBA1000732 | 3605 | 3606 |
| HEMBA1000835 | C-HEMBA1000835 | 3607 | 3608 |
| HEMBA1000875 | C-HEMBA1000875 | 3609 | |
| HEMBA1001184 | C-HEMBA1001184 | 3610 | |
| HEMBA1001272 | C-HEMBA1001272 | 3611 | |
| HEMBA1001296 | C-HEMBA1001296 | 3612 | |
| HEMBA1002048 | C-HEMBA1002048 | 3613 | 3614 |
| HEMBA1002985 | C-HEMBA1002985 | 3615 | 3616 |
| HEMBA1003120 | C-HEMBA1003120 | 3617 | 3618 |
| HEMBA1003497 | C-HEMBA1003497 | 3619 | 3620 |
| HEMBA1004007 | C-HEMBA1004007 | 3621 | |
| HEMBA1004085 | C-HEMBA1004085 | 3622 | 3623 |
| HEMBA1004785 | C-HEMBA1004785 | 3624 | 3625 |
| HEMBA1004952 | C-HEMBA1004952 | 3626 | |
| HEMBA1004971 | C-HEMBA1004971 | 3627 | |
| HEMBA1005230 | C-HEMBA1005230 | 3628 | |
| HEMBA1005246 | C-HEMBA1005246 | 3629 | 3630 |
| HEMBA1005267 | C-HEMBA1005267 | 3631 | 3632 |
| HEMBA1006276 | C-HEMBA1006276 | 3633 | |
| HEMBA1006357 | C-HEMBA1006357 | 3634 | 3635 |
| HEMBA1006517 | C-HEMBA1006517 | 3636 | 3637 |
| HEMBA1006544 | C-HEMBA1006544 | 3638 | 3639 |
| HEMBA1006749 | C-HEMBA1006749 | 3640 | 3641 |
| HEMBA1006770 | C-HEMBA1006770 | 3642 | 3643 |
| HEMBA1006912 | C-HEMBA1006912 | 3644 | |
| HEMBA1007063 | C-HEMBA1007063 | 3645 | 3646 |
| HEMBB1000106 | C-HEMBB1000106 | 3647 | 3648 |
| HEMBB1000407 | C-HEMBB1000407 | 3649 | 3650 |
| HEMBB1000542 | C-HEMBB1000542 | 3651 | 3652 |
| HEMBB1001547 | C-HEMBB1001547 | 3653 | 3654 |
| HEMBB1001959 | C-HEMBB1001959 | 3655 | 3656 |
| HEMBB1002039 | C-HEMBB1002039 | 3657 | |
| HEMBB1002041 | C-HEMBB1002041 | 3658 | 3659 |
| HEMBB1002051 | C-HEMBB1002051 | 3660 | 3661 |
| HEMBB1002120 | C-HEMBB1002120 | 3662 | 3663 |
| HEMBB1002302 | C-HEMBB1002302 | 3664 | 3665 |
| HEMBB1002661 | C-HEMBB1002661 | 3666 | 3667 |
| MAMMA1000106 | C-MAMMA1000106 | 3668 | 3669 |
| MAMMA1000141 | C-MAMMA1000141 | 3670 | 3671 |
| MAMMA1000204 | C-MAMMA1000204 | 3672 | 3673 |
| MAMMA1000226 | C-MAMMA1000226 | 3674 | 3675 |
| MAMMA1000403 | C-MAMMA1000403 | 3676 | 3677 |
| MAMMA1000473 | C-MAMMA1000473 | 3678 | 3679 |
| MAMMA1000496 | C-MAMMA1000496 | 3680 | 3681 |
| MAMMA1000528 | C-MAMMA1000528 | 3682 | |
| MAMMA1000614 | C-MAMMA1000614 | 3683 | 3684 |
| MAMMA1000652 | C-MAMMA1000652 | 3685 | |
| MAMMA1000706 | C-MAMMA1000706 | 3686 | 3687 |
| MAMMA1000788 | C-MAMMA1000788 | 3688 | 3689 |
| MAMMA1000810 | C-MAMMA1000810 | 3690 | 3691 |
| MAMMA1000814 | C-MAMMA1000814 | 3692 | 3693 |
| MAMMA1000881 | C-MAMMA1000881 | 3694 | 3695 |
| MAMMA1000986 | C-MAMMA1000986 | 3696 | 3697 |
| MAMMA1000994 | C-MAMMA1000994 | 3698 | 3699 |
| MAMMA1001141 | C-MAMMA1001141 | 3700 | 3701 |
| MAMMA1001150 | C-MAMMA1001150 | 3702 | 3703 |
| MAMMA1001237 | C-MAMMA1001237 | 3704 | 3705 |
| MAMMA1001284 | C-MAMMA1001284 | 3706 | 3707 |
| MAMMA1001310 | C-MAMMA1001310 | 3708 | 3709 |
| MAMMA1001344 | C-MAMMA1001344 | 3710 | 3711 |
| MAMMA1001418 | C-MAMMA1001418 | 3712 | 3713 |
| MAMMA1001532 | C-MAMMA1001532 | 3714 | 3715 |
| MAMMA1001615 | C-MAMMA1001615 | 3716 | 3717 |
| MAMMA1001623 | C-MAMMA1001623 | 3718 | 3719 |
| MAMMA1001634 | C-MAMMA1001634 | 3720 | 3721 |
| MAMMA1001957 | C-MAMMA1001957 | 3722 | 3723 |
| MAMMA1001978 | C-MAMMA1001978 | 3724 | 3725 |
| MAMMA1002070 | C-MAMMA1002070 | 3726 | 3727 |
| MAMMA1002080 | C-MAMMA1002080 | 3728 | 3729 |
| MAMMA1002087 | C-MAMMA1002087 | 3730 | 3731 |
| MAMMA1002095 | C-MAMMA1002095 | 3732 | 3733 |
| MAMMA1002128 | C-MAMMA1002128 | 3734 | 3735 |
| MAMMA1002142 | C-MAMMA1002142 | 3736 | 3737 |
| MAMMA1002165 | C-MAMMA1002165 | 3738 | 3739 |
| MAMMA1002205 | C-MAMMA1002205 | 3740 | |
| MAMMA1002234 | C-MAMMA1002234 | 3741 | 3742 |
| MAMMA1002586 | C-MAMMA1002586 | 3743 | 3744 |
| MAMMA1002633 | C-MAMMA1002633 | 3745 | 3746 |
| MAMMA1003126 | C-MAMMA1003126 | 3747 | 3748 |
| NT2RM1000580 | C-NT2RM1000580 | 3749 | 3750 |
| NT2RM1000858 | C-NT2RM1000858 | 3751 | 3752 |
| NT2RM2000565 | C-NT2RM2000565 | 3753 | 3754 |
| NT2RM2000582 | C-NT2RM2000582 | 3755 | |
| NT2RM2000589 | C-NT2RM2000589 | 3756 | 3757 |
| NT2RM2000632 | C-NT2RM2000632 | 3758 | 3759 |
| NT2RM2000773 | C-NT2RM2000773 | 3760 | |
| NT2RM2001558 | C-NT2RM2001558 | 3761 | 3762 |
| NT2RM2001626 | C-NT2RM2001626 | 3763 | 3764 |
| NT2RM2001643 | C-NT2RM2001643 | 3765 | 3766 |
| NT2RM2001738 | C-NT2RM2001738 | 3767 | 3768 |
| NT2RM2001792 | C-NT2RM2001792 | 3769 | 3770 |
| NT2RM2001818 | C-NT2RM2001818 | 3771 | 3772 |
| NT2RM4000100 | C-NT2RM4000100 | 3773 | 3774 |
| NT2RM4000115 | C-NT2RM4000115 | 3775 | 3776 |
| NT2RM4000417 | C-NT2RM4000417 | 3777 | 3778 |
| NT2RM4000593 | C-NT2RM4000593 | 3779 | 3780 |
| NT2RM4000761 | C-NT2RM4000761 | 3781 | 3782 |
| NT2RM4000965 | C-NT2RM4000965 | 3783 | 3784 |
| NT2RM4001377 | C-NT2RM4001377 | 3785 | 3786 |
| NT2RM4001768 | C-NT2RM4001768 | 3787 | 3788 |
| NT2RM4001843 | C-NT2RM4001843 | 3789 | 3790 |
| NT2RP1000239 | C-NT2RP1000239 | 3791 | 3792 |
| NT2RP1000465 | C-NT2RP1000465 | 3793 | 3794 |
| NT2RP1000468 | C-NT2RP1000468 | 3795 | 3796 |
| NT2RP1000679 | C-NT2RP1000679 | 3797 | 3798 |
| NT2RP1000740 | C-NT2RP1000740 | 3799 | 3800 |
| NT2RP1001031 | C-NT2RP1001031 | 3801 | 3802 |
| NT2RP2000178 | C-NT2RP2000178 | 3803 | 3804 |
| NT2RP2000240 | C-NT2RP2000240 | 3805 | |
| NT2RP2000447 | C-NT2RP2000447 | 3806 | 3807 |
| NT2RP2000610 | C-NT2RP2000610 | 3808 | 3809 |
| NT2RP2000616 | C-NT2RP2000616 | 3810 | 3811 |
| NT2RP2000712 | C-NT2RP2000712 | 3812 | 3813 |
| NT2RP2000739 | C-NT2RP2000739 | 3814 | 3815 |
| NT2RP2000818 | C-NT2RP2000818 | 3816 | 3817 |
| NT2RP2001200 | C-NT2RP2001200 | 3818 | 3819 |
| NT2RP2001223 | C-NT2RP2001223 | 3820 | 3821 |
| NT2RP2001276 | C-NT2RP2001276 | 3822 | 3823 |
| NT2RP2001388 | C-NT2RP2001388 | 3824 | 3825 |
| NT2RP2001469 | C-NT2RP2001469 | 3826 | 3827 |
| NT2RP2001562 | C-NT2RP2001562 | 3828 | 3829 |
| NT2RP2001662 | C-NT2RP2001662 | 3830 | 3831 |
| NT2RP2001755 | C-NT2RP2001755 | 3832 | 3833 |
| NT2RP2001817 | C-NT2RP2001817 | 3834 | 3835 |
| NT2RP2001948 | C-NT2RP2001948 | 3836 | 3837 |
| NT2RP2002015 | C-NT2RP2002015 | 3838 | 3839 |
| NT2RP2003390 | C-NT2RP2003390 | 3840 | 3841 |
| NT2RP2003664 | C-NT2RP2003664 | 3842 | 3843 |
| NT2RP2003940 | C-NT2RP2003940 | 3844 | 3845 |
| NT2RP2004069 | C-NT2RP2004069 | 3846 | 3847 |
| NT2RP2004108 | C-NT2RP2004108 | 3848 | 3849 |
| nnnnnnnnnnnn | C-nnnnnnnnnnnn | 3850 | 3851 |
| NT2RP2005069 | C-NT2RP2005069 | 3852 | 3853 |
| NT2RP2005378 | C-NT2RP2005378 | 3854 | 3855 |
| NT2RP2005391 | C-NT2RP2005391 | 3856 | 3857 |
| NT2RP2005597 | C-NT2RP2005597 | 3858 | 3859 |
| NT2RP2005666 | C-NT2RP2005666 | 3860 | 3861 |
| NT2RP2006004 | C-NT2RP2006004 | 3862 | 3863 |
| NT2RP2006092 | C-NT2RP2006092 | 3864 | 3865 |
| NT2RP2006134 | C-NT2RP2006134 | 3866 | 3867 |
| NT2RP3000011 | C-NT2RP3000011 | 3868 | 3869 |
| NT2RP3000022 | C-NT2RP3000022 | 3870 | 3871 |
| NT2RP3000171 | C-NT2RP3000171 | 3872 | 3873 |
| NT2RP3000304 | C-NT2RP3000304 | 3874 | 3875 |
| NT2RP3000378 | C-NT2RP3000378 | 3876 | 3877 |
| NT2RP3000444 | C-NT2RP3000444 | 3878 | 3879 |
| NT2RP3000645 | C-NT2RP3000645 | 3880 | 3881 |
| NT2RP3000676 | C-NT2RP3000676 | 3882 | 3883 |
| NT2RP3000677 | C-NT2RP3000677 | 3884 | 3885 |
| NT2RP3000789 | C-NT2RP3000789 | 3886 | 3887 |
| NT2RP3000818 | C-NT2RP3000818 | 3888 | 3889 |
| NT2RP3000838 | C-NT2RP3000838 | 3890 | 3891 |
| NT2RP3000921 | C-NT2RP3000921 | 3892 | 3893 |
| NT2RP3001159 | C-NT2RP3001159 | 3894 | 3895 |
| NT2RP3001271 | C-NT2RP3001271 | 3896 | 3897 |
| NT2RP3001542 | C-NT2RP3001542 | 3898 | 3899 |
| NT2RP3001685 | C-NT2RP3001685 | 3900 | 3901 |
| NT2RP3001976 | C-NT2RP3001976 | 3902 | 3903 |
| NT2RP3002015 | C-NT2RP3002015 | 3904 | 3905 |
| NT2RP3002281 | C-NT2RP3002281 | 3906 | 3907 |
| NT2RP3002286 | C-NT2RP3002286 | 3908 | 3909 |
| NT2RP3002324 | C-NT2RP3002324 | 3910 | 3911 |
| NT2RP3002353 | C-NT2RP3002353 | 3912 | 3913 |
| NT2RP3002571 | C-NT2RP3002571 | 3914 | 3915 |
| NT2RP3002664 | C-NT2RP3002664 | 3916 | 3917 |
| NT2RP3002737 | C-NT2RP3002737 | 3918 | 3919 |
| NT2RP3002887 | C-NT2RP3002887 | 3920 | 3921 |
| NT2RP3002900 | C-NT2RP3002900 | 3922 | 3923 |
| NT2RP3002983 | C-NT2RP3002983 | 3924 | 3925 |
| NT2RP3003473 | C-NT2RP3003473 | 3926 | 3927 |
| NT2RP3003532 | C-NT2RP3003532 | 3928 | 3929 |
| NT2RP3004025 | C-NT2RP3004025 | 3930 | 3931 |
| NT2RP3004067 | C-NT2RP3004067 | 3932 | 3933 |
| NT2RP3004119 | C-NT2RP3004119 | 3934 | 3935 |
| NT2RP3004294 | C-NT2RP3004294 | 3936 | 3937 |
| NT2RP3004345 | C-NT2RP3004345 | 3938 | 3939 |
| NT2RP4000634 | C-NT2RP4000634 | 3940 | 3941 |
| NT2RP4001001 | C-NT2RP4001001 | 3942 | 3943 |
| NT2RP4001877 | C-NT2RP4001877 | 3944 | 3945 |
| NT2RP4001879 | C-NT2RP4001879 | 3946 | 3947 |
| NT2RP4002187 | C-NT2RP4002187 | 3948 | 3949 |
| NT2RP4002451 | C-NT2RP4002451 | 3950 | 3951 |
| NT2RP4002750 | C-NT2RP4002750 | 3952 | 3953 |
| OVARC1000003 | C-OVARC1000003 | 3954 | 3955 |
| OVARC1000313 | C-OVARC1000313 | 3956 | 3957 |
| OVARC1000331 | C-OVARC1000331 | 3958 | 3959 |
| OVARC1000553 | C-OVARC1000553 | 3960 | 3961 |
| OVARC1000873 | C-OVARC1000873 | 3962 | 3963 |
| OVARC1000995 | C-OVARC1000995 | 3964 | |
| OVARC1001260 | C-OVARC1001260 | 3965 | |
| OVARC1001336 | C-OVARC1001336 | 3966 | 3967 |
| OVARC1001570 | C-OVARC1001570 | 3968 | 3969 |
| OVARC1001607 | C-OVARC1001607 | 3970 | 3971 |
| OVARC1001833 | C-OVARC1001833 | 3972 | 3973 |
| OVARC1001952 | C-OVARC1001952 | 3974 | 3975 |
| PLACE1000986 | C-PLACE1000986 | 3976 | |
| PLACE1003407 | C-PLACE1003407 | 3977 | 3978 |
| PLACE1004078 | C-PLACE1004078 | 3979 | 3980 |
| PLACE1004492 | C-PLACE1004492 | 3981 | 3982 |
| PLACE1005539 | C-PLACE1005539 | 3983 | 3984 |
| PLACE1005569 | C-PLACE1005569 | 3985 | 3986 |
| PLACE1005601 | C-PLACE1005601 | 3987 | |
| PLACE1005745 | C-PLACE1005745 | 3988 | 3989 |
| PLACE1005815 | C-PLACE1005815 | 3990 | 3991 |
| PLACE1005927 | C-PLACE1005927 | 3992 | 3993 |
| PLACE1006071 | C-PLACE1006071 | 3994 | 3995 |
| PLACE1006073 | C-PLACE1006073 | 3996 | 3997 |
| PLACE1006079 | C-PLACE1006079 | 3998 | 3999 |
| PLACE1006786 | C-PLACE1006786 | 4000 | |
| PLACE1007077 | C-PLACE1007077 | 4001 | 4002 |
| PLACE1007971 | C-PLACE1007971 | 4003 | |
| PLACE1008282 | C-PLACE1008282 | 4004 | 4005 |
| PLACE1008359 | C-PLACE1008359 | 4006 | 4007 |
| PLACE1008744 | C-PLACE1008744 | 4008 | 4009 |
| PLACE1010445 | C-PLACE1010445 | 4010 | 4011 |
| PLACE1010713 | C-PLACE1010713 | 4012 | 4013 |
| nnnnnnnnnnnn | C-nnnnnnnnnnnn | 4014 | 4015 |
| PLACE1011181 | C-PLACE1011181 | 4016 | 4017 |
| PLACE1011364 | C-PLACE1011364 | 4018 | 4019 |
| PLACE3000181 | C-PLACE3000181 | 4020 | 4021 |
| SKNMC1000014 | C-SKNMC1000014 | 4022 | 4023 |
| SKNMC1000082 | C-SKNMC1000082 | 4024 | 4025 |
| THYRO1000964 | C-THYRO1000964 | 4026 | 4027 |
| THYRO1001242 | C-THYRO1001242 | 4028 | 4029 |
| THYRO1001608 | C-THYRO1001608 | 4030 | 4031 |
| THYRO1001641 | C-THYRO1001641 | 4032 | 4033 |
| THYRO1001770 | C-THYRO1001770 | 4034 | 4035 |
| Y79AA1000030 | C-Y79AA1000030 | 4036 | 4037 |
| Y79AA1001212 | C-Y79AA1001212 | 4038 | 4039 |
| Y79AA1001426 | C-Y79AA1001426 | 4040 | 4041 |
| Y79AA1001427 | C-Y79AA1001427 | 4042 | 4043 |
| Y79AA1001523 | C-Y79AA1001523 | 4044 | 4045 |
| Y79AA1001530 | C-Y79AA1001530 | 4046 | 4047 |
| Y79AA1001592 | C-Y79AA1001592 | 4048 | 4049 |
| Y79AA1001727 | C-Y79AA1001727 | 4050 | 4051 |
| Y79AA1001787 | C-Y79AA1001787 | 4052 | 4053 |
| Y79AA1001793 | C-Y79AA1001793 | 4054 | 4055 |
| Y79AA1001795 | C-Y79AA1001795 | 4056 | 4057 |
| Y79AA1001799 | C-Y79AA1001799 | 4058 | 4059 |
| Y79AA1001803 | C-Y79AA1001803 | 4060 | 4061 |
| Y79AA1001863 | C-Y79AA1001863 | 4062 | 4063 |
| Y79AA1002058 | C-Y79AA1002058 | 4064 | 4065 |
| Y79AA1002121 | C-Y79AA1002121 | 4066 | 4067 |
| Y79AA1002213 | C-Y79AA1002213 | 4068 | 4069 |
| Y79AA1002373 | C-Y79AA1002373 | 4070 | 4071 |
| Y79AA1002376 | C-Y79AA1002376 | 4072 | 4073 |
| Y79AA1002378 | C-Y79AA1002378 | 4074 | 4075 |
| Y79AA1002381 | C-Y79AA1002381 | 4076 | 4077 |
| BNGH41000087 | C-BNGH41000087 | 4078 | 4079 |
| HEMBA1001886 | C-HEMBA1001886 | 4080 | 4081 |
| HEMBA1004067 | C-HEMBA1004067 | 4082 | 4083 |
| HEMBA1007226 | C-HEMBA1007226 | 4084 | 4085 |
| HEMBB1000309 | C-HEMBB1000309 | 4086 | 4087 |
| HEMBB1000567 | C-HEMBB1000567 | 4088 | 4089 |
| MAMMA1000102 | C-MAMMA1000102 | 4090 | 4091 |
| MAMMA1001066 | C-MAMMA1001066 | 4092 | 4093 |
| MAMMA1001094 | C-MAMMA1001094 | 4094 | 4095 |
| MAMMA1001609 | C-MAMMA1001609 | 4096 | 4097 |
| MAMMA1001901 | C-MAMMA1001901 | 4098 | |
| MAMMA1002091 | C-MAMMA1002091 | 4099 | 4100 |
| NT2RM1000462 | C-NT2RM1000462 | 4101 | 4102 |
| NT2RM1000542 | C-NT2RM1000542 | 4103 | 4104 |
| NT2RM1000789 | C-NT2RM1000789 | 4105 | 4106 |
| NT2RM1000855 | C-NT2RM1000855 | 4107 | 4108 |
| NT2RM1000899 | C-NT2RM1000899 | 4109 | 4110 |
| NT2RP2000092 | C-NT2RP2000092 | 4111 | 4112 |
| NT2RP2001538 | C-NT2RP2001538 | 4113 | 4114 |
| NT2RP2001921 | C-NT2RP2001921 | 4115 | 4116 |
| NT2RP2003138 | C-NT2RP2003138 | 4117 | 4118 |
| NT2RP2003302 | C-NT2RP2003302 | 4119 | 4120 |
| NT2RP2003950 | C-NT2RP2003950 | 4121 | 4122 |
| NT2RP2005535 | C-NT2RP2005535 | 4123 | 4124 |
| NT2RP2005774 | C-NT2RP2005774 | 4125 | 4126 |
| NT2RP3000148 | C-NT2RP3000148 | 4127 | 4128 |
| NT2RP3000232 | C-NT2RP3000232 | 4129 | 4130 |
| NT2RP3000427 | C-NT2RP3000427 | 4131 | |
| NT2RP3000652 | C-NT2RP3000652 | 4132 | 4133 |
| NT2RP3001650 | C-NT2RP3001650 | 4134 | 4135 |
| NT2RP3002409 | C-NT2RP3002409 | 4136 | |
| NT2RP3002411 | C-NT2RP3002411 | 4137 | 4138 |
| NT2RP3003448 | C-NT2RP3003448 | 4139 | |
| NT2RP4002715 | C-NT2RP4002715 | 4140 | 4141 |
| OVARC1000307 | C-OVARC1000307 | 4142 | 4143 |
| PLACE1000907 | C-PLACE1000907 | 4144 | 4145 |
| PLACE1007081 | C-PLACE1007081 | 4146 | 4147 |
| PLACE1010011 | C-PLACE1010011 | 4148 | 4149 |
| PLACE3000213 | C-PLACE3000213 | 4150 | 4151 |
| PLACE4000354 | C-PLACE4000354 | 4152 | 4153 |
| PLACE4000455 | C-PLACE4000455 | 4154 | |
| THYRO1000776 | C-THYRO1000776 | 4155 | 4156 |
| THYRO1001593 | C-THYRO1001593 | 4157 | 4158 |
| Y79AA1000750 | C-Y79AA1000750 | 4159 | 4160 |
| Y79AA1000888 | C-Y79AA1000888 | 4161 | 4162 |
| Y79AA1002129 | C-Y79AA1002129 | 4163 | 4164 |
| Y79AA1002334 | C-Y79AA1002334 | 4165 | 4166 |
| MAMMA1002224 | C-MAMMA1002224 | 4167 | |
| NT2RP1000271 | C-NT2RP1000271 | 4168 | 4169 |
| NT2RP3000481 | C-NT2RP3000481 | 4170 | 4171 |
| NT2RP3004481 | C-NT2RP3004481 | 4172 | 4173 |
| HEMBA1006658 | C-HEMBA1006658 | 4174 | 4175 |
| NT2RP2006099 | C-NT2RP2006099 | 4176 | 4177 |
| NT2RP2006580 | C-NT2RP2006580 | 4178 | 4179 |

### Homology search result 1

The result of the homology search in the SwissProt using the representative sequences of the 5'-ends.

Indicated are from the top,
the name of the representative sequence of the cluster,
definition of the top hit data,
the P-value: the length of the sequence used for comparison (nucleotide):similarity (%),
the organism of which the top hit data is obtained,
the Accession No. of the top hit data.

Homology search results of the representative sequences of the 5' -end cluster to the data in SwissProt database are shown only for the representative sequences of the cluster from which clones were selected based on the homology search results.
The P-value is the score which is determined by taking into account the statistic probability of occurrence between the two sequences, and generally low score reflects high similarity. (Altschul, S.F., Gish, W., Miller, W., Myers, E.W. & Lipman, D.J. (1990) "Basic local alignment search tool." J. Mol. Biol. 215:403-410; Gish, W. & States, D.J. (1993) "Identification of protein coding regions by database similarity search." Nature Genet. 3:266-272).
HRIFA000016a
GLYCINE-RICH CELL WALL STRUCTURAL PROTEIN 1.8 PRECURSOR (GRP 1.8).
9.2e-05:178:32
PHASEOLUS VULGARIS (KIDNEY BEAN) (FRENCH BEAN).
P10496
HRIFA000071a
CIRCUMSPOROZOITE PROTEIN PRECURSOR (CS).
5.8e-05:194:29
PLASMODIUM SIMIUM.
Q03110
HRIFA000116a
HYPOTHETICAL 68.7 KD PROTEIN ZK757.1 IN CHROMOSOME III.
6.2e-06:83:27
CAENORHABDITIS ELEGANS.
P34679
HRIFA000123a
PATHOGENESIS-RELATED PROTEIN 1 PRECURSOR (PR-1).
6.2e-08:89:34
ARABIDOPSIS THALIANA (MOUSE-EAR CRESS).
P33154
HRIFA000264a
PROCOLLAGEN ALPHA 1(I) CHAIN PRECURSOR.
1.4e-06:231:34
GALLUS GALLUS (CHICKEN).
P02457
HRIFA000327a
ATP-BINDING CASSETTE TRANSPORTER 1.
2.0e-16:238:31
MUS MUSCULUS (MOUSE).
P41233
HRIFA000415a
PROLINE-RICH PROTEIN MP-2 PRECURSOR.
3.6e-06:120:35
MUS MUSCULUS (MOUSE).
P05142
HRIFA000432a
PUTATIVE GENERAL NEGATIVE REGULATOR OF TRANSCRIPTION C16C9.04C.
2.2e-21:86:52
SCHIZOSACCHAROMYCES POMBE (FISSION YEAST).
Q09818
HRIFA000446a
HYPOTHETICAL 64.8 KD PROTEIN IN GDI1-COX15 INTERGENIC REGION.
2.5e-09:138:34
SACCHAROMYCES CEREVISIAE (BAKER'S YEAST).
P40085
HRIFA000553a
CARTILAGE MATRIX PROTEIN PRECURSOR (MATRILIN-1).
1.7e-27:117:48
GALLUS GALLUS (CHICKEN).
P05099
HRIFA000564a
ERYTHROCYTE BAND 7 INTEGRAL MEMBRANE PROTEIN (STOMATIN) (PROTEIN 7.2B).
2.9e-28:163:38
MUS MUSCULUS (MOUSE).
P54116
HRIFA00063 1 a
ZINC FINGER PROTEIN 140.
8.2e-45:155:47
HOMO SAPIENS (HUMAN).
P52738
HRIFA000683a
FIBRILLIN 1 PRECURSOR.
4.8e-18:77:46
HOMO SAPIENS (HUMAN).
P35555
HRIFA000695a
"SALIVARY PROLINE-RICH PROTEIN PRECURSOR (CLONES CP3, CP4 AND CP5) [CONTAINS: BASIC PEPTIDE IB-6" PEPTIDE P-H].
4.0e-06:105:33
HOMO SAPIENS (HUMAN).
P04280
HRIFA000776a
FIBRILLIN 2 PRECURSOR.
1.6e-42:214:44
HOMO SAPIENS (HUMAN).
P35556
HRIFA000814a
ZINC FINGER PROTEIN 133.
4.4e-16:49:87
HOMO SAPIENS (HUMAN).
P52736
HRIFA000845a
PROCOLLAGEN ALPHA 1(I) CHAIN PRECURSOR.
6.0e-06:172:34
MUS MUSCULUS (MOUSE).
P11087
HRIFA001099a
CYCLIC-AMP-DEPENDENT TRANSCRIPTION FACTOR ATF-5 (FRAGMENT).
0.92:38:34
HOMO SAPIENS (HUMAN).
P18849
HRIFA001132a
AGRIN PRECURSOR.
1.3e-26:239:32
GALLUS GALLUS (CHICKEN).
P31696
HRIFA001138a
CARTILAGE OLIGOMERIC MATRIX PROTEIN PRECURSOR (COMP).
5.9e-114:147:83
HOMO SAPIENS (HUMAN).
P49747
HRIFA001200a
TRANS-ACTING TRANSCRIPTIONAL PROTEIN ICPO (P135 PROTEIN) (IER 2.9/ER2.6).
0.035:119:34
BOVINE HERPESVIRUS TYPE 1 (STRAIN JURA).
P29128
HRIFA001337a
LOW-DENSITY LIPOPROTEIN RECEPTOR PRECURSOR (LDL RECEPTOR).
2.4e-17:98:42
CRICETULUS GRISEUS (CHINESE HAMSTER).
P35950
HRIFA001341a
NEUROFILAMENT TRIPLET L PROTEIN (68 KD NEUROFILAMENT PROTEIN) (NF-L) (NF68).
1.2e-102:248:87
RATTUS NORVEGICUS (RAT).
P19527
HRIFA001413a
BACTENECIN 7 PRECURSOR (BAC7) (PR-59).
0.0032:33:63
BOS TAURUS (BOVINE).
P19661
HRIFA001439a
B-CELL GROWTH FACTOR PRECURSOR (BCGF-12 KD).
0.00031:34:61
HOMO SAPIENS (HUMAN).
P20931
HRIFA001489a
PROBABLE G PROTEIN-COUPLED RECEPTOR APJ.
8.4e-65:105:72
HOMO SAPIENS (HUMAN).
P35414
HRIFA001558a
HISTIDINE-RICH GLYCOPROTEIN PRECURSOR.
0.0048:80:31
PLASMODIUM LOPHURAE.
P04929
HRIFA001712a
PUTATIVE SERINE/THREONINE-PROTEIN KINASE PKWA (EC 2.7.1.-).
2.5e-19:169:31
THERMOMONOSPORA CURVATA.
P49695
HRIFA001720a
ZINC FINGER PROTEIN 85 (ZINC FINGER PROTEIN HPF4) (HTF1).
1.4e-94:273:64
HOMO SAPIENS (HUMAN).
Q03923
HRIFA001866a
EARLY ANTIGEN PROTEIN D (EA-D).
0.10:93:34
EPSTEIN-BARR VIRUS (STRAIN B95-8) (HUMAN HERPESVIRUS 4).
P03191
HRIFA001942a
"PROCOLLAGEN-LYSINE,2-OXOGLUTARATE 5-DIOXYGENASE 1 PRECURSOR (EC 1.14.11.4) (LYSYL HYDROXYLASE 1) (LH1)."
4.7e-12:140:30
GALLUS GALLUS (CHICKEN).
P24802
HRIFA001971a
HYPOTHETICAL 46.3 KD PROTEIN IN PTA1-CDC24 INTERGENIC REGION.
2.5e-10:86:30
SACCHAROMYCES CEREVISIAE (BAKER'S YEAST).
P39727
HRIFA001972a
LAMININ ALPHA-1 CHAIN PRECURSOR (LAMININ A CHAIN).
0.10:100:34
MUS MUSCULUS (MOUSE).
P19137
HRIFA001975a
ACETYLCHOLINESTERASE PRECURSOR (EC 3.1.1.7).
6.5e-30:243:33
MUS MUSCULUS (MOUSE).
P21836
HRIFA001984a
"PROCOLLAGEN-LYSINE,2-OXOGLUTARATE 5-DIOXYGENASE 1 PRECURSOR (EC 1.14.11.4) (LYSYL HYDROXYLASE 1) (LH1)."
1.2e-11:140:30
GALLUS GALLUS (CHICKEN).
P24802
HRIFA002063a
GNS 1 PROTEIN.
1.3e-05:127:30
SACCHAROMYCES CEREVISIAE (BAKER'S YEAST).
P25358
HRIFA002102a
MUCIN 2 PRECURSOR (INTESTINAL MUCIN 2).
2.9e-07:241:30
HOMO SAPIENS (HUMAN).
Q02817
HRIFA002284a
ACIDIC PROLINE-RICH PROTEIN PRECURSOR (CLONE PRP33).
3.8e-05:104:34
RATTUS NORVEGICUS (RAT).
P04474
HRIFA002309a
PUTATIVE SERINE/THREONINE-PROTEIN KINASE PKWA (EC 2.7.1.-).
1.5e-08:110:37
THERMOMONOSPORA CURVATA.
P49695
HRIFA002384a
GAP JUNCTION ALPHA-6 PROTEIN (CONNEXIN 45) (CX45).
1.8e-31:94:42
HOMO SAPIENS (HUMAN).
P36383
HRIFA002503a
N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
6.1e-92:246:67
MUS MUSCULUS (MOUSE).
P15535
HRIFA002689a
TRANSCRIPTION FACTOR GATA-6 (GATA BINDING FACTOR-6) (DNA BINDING PROTEIN GATA-GT2).
0.38:49:34
RATTUS NORVEGICUS (RAT).
P46153
HRIFA002694a
ANTER-SPECIFIC PROLINE-RICH PROTEIN APG PRECURSOR.
4.7e-05:93:37
ARABIDOPSIS THALIANA (MOUSE-EAR CRESS).
P40602
HRIFA002743a
BONE MORPHOGENETIC PROTEIN 1 PRECURSOR (EC 3.4.24.-) (BMP-1).
1.2e-23:216:31
HOMO SAPIENS (HUMAN).
P13497
HRIFA002762a
PROLINE-RICH PROTEIN MP-2 PRECURSOR.
5.1e-09:129:41
MUS MUSCULUS (MOUSE).
P05142
HRIFA002766a
FIBROMODULIN PRECURSOR (FM) (COLLAGEN-BINDING 59 KD PROTEIN).
1.8e-12:139:34
HOMO SAPIENS (HUMAN).
Q06828
HRIFA002787a
PROCOLLAGEN ALPHA 2(I) CHAIN PRECURSOR.
1.6e-10:124:37
HOMO SAPIENS (HUMAN).
P08123
HRIFA002805a
ZINC FINGER PROTEIN 140.
3.6e-23:43:74
HOMO SAPIENS (HUMAN).
P52738
HRIFA002891 a
"FIBULIN-1, ISOFORM C PRECURSOR (BASEMENT-MEMBRANE PROTEIN 90) (BM-90)."
2.0e-41:239:39
MUS MUSCULUS (MOUSE).
Q08878
HRIFA002919a
BEM46 PROTEIN (FRAGMENT).
1.0e-12:171:32
SCHIZOSACCHAROMYCES POMBE (FISSION YEAST).
P54069
HRIFA002980a
LOW-DENSITY LIPOPROTEIN RECEPTOR-RELATED PROTEIN 1 PRECURSOR (LRP) (ALPHA-2-MACROGLOBULIN RECEPTOR) (A2MR).
8.7e-32:202:37
GALLUS GALLUS (CHICKEN).
P98157
HRIFA003055a
PROLINE-RICH PROTEIN MP-2 PRECURSOR.
3.4e-08:175:29
MUS MUSCULUS (MOUSE).
P05142
HRIFA003063a
B-CELL LYMPHOMA 6 PROTEIN HOMOLOG.
2.8e-15:123:34
MUS MUSCULUS (MOUSE).
P41183
HRIFA003093a
PROLINE-RICH PROTEIN MP-2 PRECURSOR.
1.3e-11:142:37
MUS MUSCULUS (MOUSE).
P05142
HRIFA003340a
TRANS-ACTING TRANSCRIPTIONAL PROTEIN ICP0 (IMMEDIATE-EARLY PROTEIN IE110) (VMW110) (ALPHA-0 PROTEIN).
2.3e-05:200:31
HERPES SIMPLEX VIRUS (TYPE 1 / STRAIN 17).
P08393
HRIFA003357a
GLUCOSE REPRESSION MEDIATOR PROTEIN.
0.0023:190:26
SACCHAROMYCES CEREVISIAE (BAKER'S YEAST).
P14922
HRIFA003402a
COLLAGEN ALPHA 1(II) CHAIN (FRAGMENTS).
3.6e-05:194:27
BOS TAURUS (BOVINE).
P02459
HRIFA003504a
CADHERIN-RELATED TUMOR SUPPRESSOR PRECURSOR (FAT PROTEIN).
1.4e-08:150:33
DROSOPHILA MELANOGASTER (FRUIT FLY).
P33450
HRIFA003592a
CD9 ANTIGEN.
0.0053:24:70
BOS TAURUS (BOVINE).
P30932
HRIFA003635a
"MANNOSYL-OLIGOSACCHARIDE ALPHA-1,2-MANNOSIDASE ISOFORM 1 (EC 3.2.1.113) (MAN(9)-ALPHA-MANNOSIDASE)."
5.3e-45:239:43
DROSOPHILA MELANOGASTER (FRUIT FLY).
P53624
HRIFA003640a
PROCYCLIC FORM SPECIFIC POLYPEPTIDE A-BETA PRECURSOR (PROCYCLIN) (PARP A-BETA).
0.00018:28:64
TRYPANOSOMA BRUCEI BRUCEI.
P09791
HRIFA003883a
TRANSCRIPTIONAL REPRESSOR PROTEIN YY1 (YIN AND YANG 1) (YY-1) (DELTA TRANSCRIPTION FACTOR) (NF-E1) (UCR-MOTIF DNA-BINDING PROTEIN).
1.0:57:35
MUS MUSCULUS (MOUSE).
Q00899
HRIFA003892a
MULTIDRUG RESISTANCE PROTEIN 2 (MULTIDRUG-EFFLUX TRANSPORTER 2).
6.5e-08:144:25
BACILLUS SUBTILIS.
P39843
HRIFA003946a
PROLINE-RICH PROTEIN MP-2 PRECURSOR.
1.4e-06:85:37
MUS MUSCULUS (MOUSE).
P05142
HRIFA004006a
ZINC FINGER PROTEIN 140.
6.2e-20:83:66
HOMO SAPIENS (HUMAN).
P52738
HRIFA004034a
B-CELL LYMPHOMA 3-ENCODED PROTEIN (BCL-3 PROTEIN).
1.4e-15:192:32
HOMO SAPIENS (HUMAN).
P20749
HRIFA004112a
CADHERIN-RELATED TUMOR SUPPRESSOR PRECURSOR (FAT PROTEIN).
7.2e-26:193:37
DROSOPHILA MELANOGASTER (FRUIT FLY).
P33450
HRIFA004162a
ERYTHROCYTE BAND 7 INTEGRAL MEMBRANE PROTEIN (STOMATIN) (PROTEIN 7.2B).
3.6e-10:117:29
MUS MUSCULUS (MOUSE).
P54116
HRIFA004401 a
LACTOSE OPERON REPRESSOR.
1.1e-07:36:86
ESCHERICHIA COLI.
P03023
HRIFA004426a
ATRIAL GLAND-SPECIFIC ANTIGEN PRECURSOR (AGSA).
5.1e-11:85:41
APLYSIA CALIFORNICA (CALIFORNIA SEA HARE).
P15287
HRIFA004490a
BETA-GALACTOSIDASE PRECURSOR (EC 3.2.1.23) (LACTASE) (ACID BETA-GALACTOSIDASE).
5.3e-19:101:44
MUS MUSCULUS (MOUSE).
P23780
HRIFA004523a
GLYCOSYLTRANSFERASE ALG2 (EC 2.4.1.-).
2.6e-36:180:43
SACCHAROMYCES CEREVISIAE (BAKER'S YEAST).
P43636
HRIFA004663a
T-CELL-SPECIFIC TRANSCRIPTION FACTOR 1 (TCF-1) (T-CELL FACTOR 1) (TRANSCRIPTION FACTOR-7).
1.2e-40:112:75
MUS MUSCULUS (MOUSE).
Q00417
HRIFA004696a
PROTEIN TRANSPORT PROTEIN SEC61 ALPHA SUBUNIT.
1.1e-62:145:84
CANIS FAMILIARIS (DOG).
P38377
HRIFA004714a
HYPOTHETICAL 36.7 KD PROTEIN AH6.2 IN CHROMOSOME II.
2.3e-50:127:54
CAENORHABDITIS ELEGANS.
Q09201
HRIFA004745a
MITOCHONDRIAL RNA SPLICING PROTEIN MSR4.
5.0e-17:107:43
SACCHAROMYCES CEREVISIAE (BAKER'S YEAST).
P23500
HRIFA004780a
EXTENSIN PRECURSOR (PROLINE-RICH GLYCOPROTEIN).
7.2e-07:142:30
ZEA MAYS (MAIZE).
P14918
HRIFA004919a
GLYCINE-RICH CELL WALL STRUCTURAL PROTEIN 1.8 PRECURSOR (GRP 1.8).
1.5e-25:156:46
PHASEOLUS VULGARIS (KIDNEY BEAN) (FRENCH BEAN).
P10496
HRIFA005072a
GLYCINE-RICH CELL WALL STRUCTURAL PROTEIN (CLONE W10-1) (FRAGMENT).
8.3e-05:24:62
LYCOPERSICON ESCULENTUM (TOMATO).
Q01157
HRIFA005102a
A-AGGLUTININ ATTACHMENT SUBUNIT PRECURSOR.
2.5e-07:188:31
SACCHAROMYCES CEREVISIAE (BAKER'S YEAST).
P32323
HRIFA005184a
CYTOCHROME B5.
3.4e-11:117:29
SACCHAROMYCES CEREVISIAE (BAKER'S YEAST).
P40312
HRIFA005214a
TRANS-ACTING TRANSCRIPTIONAL PROTEIN ICP0 (VMW118 PROTEIN).
5.9e-05:141:33
HERPES SIMPLEX VIRUS (TYPE 2 / STRAIN HG52).
P28284
HRIFA005231a
ORM1 PROTEIN.
1.7e-18:137:35
SACCHAROMYCES CEREVISIAE (BAKER'S YEAST).
P53224
HRIFA005240a
ZINC FINGER PROTEIN 85 (ZINC FINGER PROTEIN HPF4) (HTF1).
6.3e-81:194:70
HOMO SAPIENS (HUMAN).
Q03923
HRIFA005255a
HYPOTHETICAL 57.1 KD PROTEIN IN MAP2-TEL1 INTERGENIC REGION.
1.5e-07:202:24
SACCHAROMYCES CEREVISIAE (BAKER'S YEAST).
P38176
HRIFA005271a
MITOCHONDRIAL PHOSPHATE CARRIER PROTEIN PRECURSOR.
1.2e-55:86:81
HOMO SAPIENS (HUMAN).
Q00325
HRIFA005296a
INSULIN PROMOTER FACTOR 1 (IPF-1) (ISLET/DUODENUM HOMEOBOX-1) (IDX-1) (SOMATOSTATIN TRANSACTIVATING FACTOR-1) (STF-1) (PANCREAS/DUODENUM HOMEOBOX-1) (GLUCOSE SENSITIVE FACTOR) (GSF).
0.82:90:34
HOMO SAPIENS (HUMAN).
P52945
HRIFA005300a
SKIN SECRETORY PROTEIN XP2 PRECURSOR (APEG PROTEIN).
1.6e-07:178:30
XENOPUS LAEVIS (AFRICAN CLAWED FROG).
P17437
HRIFA005369a
EBNA-1 NUCLEAR PROTEIN.
2.3e-07:101:39
EPSTEIN-BARR VIRUS (STRAIN B95-8) (HUMAN HERPESVIRUS 4).
P03211
HRIFA005372a
CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
1.1e-14:97:38
PETROMYZON MARINUS (SEA LAMPREY).
P25210
HRIFA005392a
SYNDECAN-2 PRECURSOR (FIBROGLYCAN) (HEPARAN SULFATE PROTEOGLYCAN CORE PROTEIN) (HSPG) (SYND2).
1.3e-50:126:84
HOMO SAPIENS (HUMAN).
P34741
HRIFA005409a
HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
7.1e-66:197:64
HOMO SAPIENS (HUMAN).
P30825
HRIFA005420a
INTERFERON-RELATED PROTEIN PC4 (TPA INDUCED SEQUENCE 7) (TIS7 PROTEIN).
1.5e-33:221:41
MUS MUSCULUS (MOUSE).
P19182
HRIFA005438a
SUCCINATE DEHYDROGENASE [UBIQUINONE] FLAVOPROTEIN SUBUNIT PRECURSOR (EC 1.3.5.1) (FP) (FLAVOPROTEIN SUBUNIT OF COMPLEX II).
6.4e-71:175:68
HOMO SAPIENS (HUMAN).
P31040
HRIFA005462a
CARBONIC ANHYDRASE VI (EC 4.2.1.1) (SALIVARY) (CARBONATE DEHYDRATASE VI).
1.4e-19:137:37
OVIS ARIES (SHEEP).
P08060
HRIFA005500a
EBNA-1 NUCLEAR PROTEIN.
0.00042:54:50
EPSTEIN-BARR VIRUS (STRAIN B95-8) (HUMAN HERPESVIRUS 4).
P03211
HRIFA005540a
CYCLIC-AMP-DEPENDENT TRANSCRIPTION FACTOR ATF-6 (FRAGMENT).
0.12:47:29
HOMO SAPIENS (HUMAN).
P18850
HRIFA005644a
VACUOLAR ATP SYNTHASE SUBUNIT AC45 PRECURSOR (EC 3.6.1.34) (V-ATPASE AC45 SUBUNIT):
1.2e-102:233:87
BOS TAURUS (BOVINE).
P40682
HRIFA005702a
CELL SURFACE GLYCOPROTEIN MUC18 PRECURSOR (MELANOMA-ASSOCIATED ANTIGEN MUC18) (MELANOMA-ASSOCIATED ANTIGEN A32) (S-ENDO 1
ENDOTHELIAL- ASSOCIATED ANTIGEN) (CD146 ANTIGEN) (MELANOMA ADHESION MOLECULE).
8.7e-05:174:28
HOMO SAPIENS (HUMAN).
P43121
HRIFA005720a
F-SPONDIN PRECURSOR.
8.9e-12:155:31
XENOPUS LAEVIS (AFRICAN CLAWED FROG).
P35447
HRIFA005728a
SPORULATION-SPECIFIC PROTEIN 1 (EC 2.7.1.-).
1.7e-05:126:29
SACCHAROMYCES CEREVISIAE (BAKER'S YEAST).
P08458
HRIFA005732a
PUTATIVE SERINE/THREONINE-PROTEIN KINASE PKWA (EC 2.7.1.-).
4.4e-26:159:38
THERMOMONOSPORA CURVATA.
P49695
HRIFA005760a
FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).
3.8e-15:53:54
HOMO SAPIENS (HUMAN).
P16442
HRIFA005781a
ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
5.2e-47:228:47
HOMO SAPIENS (HUMAN).
P37058
HRIFA005944a
PROCOLLAGEN ALPHA 1(II) CHAIN PRECURSOR [CONTAINS: CHONDROCALCIN].
2.5e-06:142:35
MUS MUSCULUS (MOUSE).
P28481
HRIFA006183a
ZINC FINGER PROTEIN 136.
1.3e-42:129:62
HOMO SAPIENS (HUMAN).
P52737
HRIFA006250a
HOMEOTIC GENE REGULATOR (BRAHMA PROTEIN).
0.0038:75:37
DROSOPHILA MELANOGASTER (FRUIT FLY).
P25439
HRIFA006298a
EBNA-1 NUCLEAR PROTEIN.
1.4e-05:80:42
EPSTEIN-BARR VIRUS (STRAIN B95-8) (HUMAN HERPESVIRUS 4).
P03211
HRIFA006448a
SKIN SECRETORY PROTEIN XP2 PRECURSOR (APEG PROTEIN).
8.5e-05:183:28
XENOPUS LAEVIS (AFRICAN CLAWED FROG).
P17437
HRIFA006494a
AXONIN-1 PRECURSOR (AXONAL GLYCOPROTEIN TAG-1) (TRANSIENT AXONAL GLYCOPROTEIN 1).
1.2e-18:201:33
HOMO SAPIENS (HUMAN).
Q02246
HRIFA00651 Oa
CORNICHON PROTEIN.
6.0e-53:144:66
DROSOPHILA MELANOGASTER (FRUIT FLY).
P49858
HRIFA006566a
CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
6.6e-15:97:38
PETROMYZON MARINUS (SEA LAMPREY).
P25210
HRIFA006572a
PROCOLLAGEN ALPHA 1(I) CHAIN PRECURSOR.
7.2e-05:158:29
MUS MUSCULUS (MOUSE).
P11087
HRIFA006586a
HYPOTHETICAL 53.3 KD PROTEIN IN HXT8-CAN1 INTERGENIC REGION.
1.3e-13:219:26
SACCHAROMYCES CEREVISIAE (BAKER'S YEAST).
P39981
HRIFA006596a
POTENTIAL CAAX PRENYL PROTEASE 1 (EC 3.4.24.-) (PRENYL PROTEIN- SPECIFIC ENDOPROTEASE 1) (PPSEP 1).
7.2e-22:241:32
SCHIZOSACCHAROMYCES POMBE (FISSION YEAST).
Q10071
HRIFA006609a
PANCREATIC HORMONE PRECURSOR (PANCREATIC POLYPEPTIDE) (PP).
0.61:28:46
"GALLUS GALLUS (CHICKEN), AND MELEAGRIS GALLOPAVO (COMMON TURKEY)."
P01306
HRIFA006633a
COLLAGEN ALPHA 1(XVI) CHAIN PRECURSOR.
7.8e-07:170:34
HOMO SAPIENS (HUMAN).
Q07092
HRIFA006642a
AMALGAM PROTEIN PRECURSOR.
1.5e-09:185:28
DROSOPHILA MELANOGASTER (FRUIT FLY).
P15364
HRIFA006649a
ZINC FINGER PROTEIN 85 (ZINC FINGER PROTEIN HPF4) (HTF1).
1.7e-50:166:50
HOMO SAPIENS (HUMAN).
Q03923
HRIFA006667a
ZINC FINGER PROTEIN 85 (ZINC FINGER PROTEIN HPF4) (HTF1).
6.8e-45:180:43
HOMO SAPIENS (HUMAN).
Q03923
HRIFA006730a
SYG1 PROTEIN.
1.8e-14:164:35
SACCHAROMYCES CEREVISIAE (BAKER'S YEAST).
P40528
HRIFA006798a
SKIN SECRETORY PROTEIN XP2 PRECURSOR (APEG PROTEIN).
0.22:149:34
XENOPUS LAEVIS (AFRICAN CLAWED FROG).
P17437
HRIFA006926a
SYNAPTOTAGMIN IV.
3.6e-19:168:38
RATTUS NORVEGICUS (RAT).
P50232
HRIFA007013a
MIC1 PROTEIN.
1.4e-13:115:38
SACCHAROMYCES CEREVISIAE (BAKER'S YEAST).
P53258
HRIFA007032a
CCAAT DISPLACEMENT PROTEIN (HOMEOBOX PROTEIN CLOX) (CLOX-1) (FRAGMENT).
0.00013:92:35
CANIS FAMILIARIS (DOG).
P39881
HRIFA007068a
EBNA-1 NUCLEAR PROTEIN.
7.0e-10:145:33
EPSTEIN-BARR VIRUS (STRAIN B95-8) (HUMAN HERPESVIRUS 4).
P03211
HRIFA007152a
TRANSCRIPTION FACTOR SOX-4.
0.90:47:44
HOMO SAPIENS (HUMAN).
Q06945
HRIFA007219a
THROMBOSPONDIN 3 PRECURSOR.
1.3e-105:209:88
HOMO SAPIENS (HUMAN).
P49746
HRIFA007228a
HYPOTHETICAL 53.3 KD PROTEIN IN HXT8-CAN1 INTERGENIC REGION.
2.3e-11:174:24
SACCHAROMYCES CEREVISIAE (BAKER'S YEAST).
P39981
HRIFA007243a
PROBABLE CALCIUM-TRANSPORTING ATPASE 6 (EC 3.6.1.38).
3.0e-18:163:36
SACCHAROMYCES CEREVISIAE (BAKER'S YEAST).
P39986
HRIFA007244a
EXTENSIN PRECURSOR (CELL WALL HYDROXYPROLINE-RICH GLYCOPROTEIN).
4.2e-05:81:33
NICOTIANA TABACUM (COMMON TOBACCO).
P13983
HRIFA007256a
DEATH-ASSOCIATED PROTEIN KINASE 1 (EC 2.7.1.-) (DAP KINASE 1).
2.3e-77:186:75
HOMO SAPIENS (HUMAN).
P53355
HRIFA007262a
PAIRED AMPHIPATHIC HELIX PROTEIN.
1.3e-06:152:26
SACCHAROMYCES CEREVISIAE (BAKER'S YEAST).
P22579
HRIFA007352a
5'-TG-3' INTERACTING FACTOR (HOMEOBOX PROTEIN TGIF).
4.2e-36:146:57
HOMO SAPIENS (HUMAN).
Q15583
HRIFA007424a
F-SPONDIN PRECURSOR.
8.9e-34:84:89
RATTUS NORVEGICUS (RAT).
P35446
HRIFA007435a
PROTEIN KINASE CEK1 (EC 2.7.1.-).
1.0e-37:159:53
SCHIZOSACCHAROMYCES POMBE (FISSION YEAST).
P38938
HRIFA007463a
HST1 PROTEIN (HOMOLOGOUS TO SIR2 PROTEIN 1).
4.8e-32:85:48
SACCHAROMYCES CEREVISIAE (BAKER'S YEAST).
P53685
HRIFA007493a
UBIQUITIN-CONJUGATING ENZYME E2-28.4 KD (EC 6.3.2.19) (UBIQUITIN- PROTEIN LIGASE) (UBIQUITIN CARRIER PROTEIN).
1.2e-47:171:56
SACCHAROMYCES CEREVISIAE (BAKER'S YEAST).
P33296
HRIFA007512a
EXTENSIN PRECURSOR (CELL WALL HYDROXYPROLINE-RICH GLYCOPROTEIN).
8.0e-07:173:28
NICOTIANA TABACUM (COMMON TOBACCO).
P13983
HRIFA007532a
"CALPAIN P94, LARGE [CATALYTIC] SUBUNIT (EC 3.4.22.17) (CALCIUM-ACTIVATED NEUTRAL PROTEINASE) (CANP) (P94 PROTEIN) (MUSCLE-SPECIFIC CALCIUM-ACTIVATED NEUTRAL PROTEASE 3 LARGE SUBUNIT)."
1.8e-10:110:37
HOMO SAPIENS (HUMAN).
P20807
HRIFA007547a
TRANS-ACTING TRANSCRIPTIONAL PROTEIN ICP0 (P135 PROTEIN) (IER 2.9/ER2.6).
0.068:51:45
BOVINE HERPESVIRUS TYPE 1 (STRAIN K22).
P29836
HRIFA007565a
COLLAGEN ALPHA 1(X) CHAIN PRECURSOR.
5.1e-08:121:37
HOMO SAPIENS (HUMAN).
Q03692
HRIFA007571a
ORM1 PROTEIN.
5.8e-17:106:36
SACCHAROMYCES CEREVISIAE (BAKER'S YEAST).
P53224
HRIFA007659a
HYPOTHETICAL 51.5 KD PROTEIN D2024.3 IN CHROMOSOME IV.
2.5e-47:213:41
CAENORHABDITIS ELEGANS.
P49191
HRIFA007722a
HYPOTHETICAL 24.5 KD PROTEIN IN SAP185-BCK1 INTERGENIC REGION.
7.7e-13:146:32
SACCHAROMYCES CEREVISIAE (BAKER'S YEAST).
P40857
HRIFA007728a
EXTENSIN PRECURSOR (CELL WALL HYDROXYPROLINE-RICH GLYCOPROTEIN).
9.1e-05:124:31
NICOTIANA TABACUM (COMMON TOBACCO).
P13983
HRIFA007745a
ACETYLCHOLINESTERASE PRECURSOR (EC 3.1.1.7) (ACHE).
7.0e-15:109:36
TORPEDO CALIFORNIA (PACIFIC ELECTRIC RAY).
P04058
HRIFA007829a
GLYCINE-RICH CELL WALL STRUCTURAL PROTEIN (CLONE W10-1) (FRAGMENT).
0.00045:16:68
LYCOPERSICON ESCULENTUM (TOMATO).
Q01157
HRIFA007909a
COLLAGEN ALPHA 1(1) CHAIN (FRAGMENTS).
6.1e-06:173:34
BOS TAURUS (BOVINE).
P02453
HRIFA007985a
T-CELL RECEPTOR BETA CHAIN PRECURSOR (ANA 11).
0.00079:97:37
ORYCTOLAGUS CUNICULUS (RABBIT).
P06333
HRIFA008000a
"DIHYDROPYRIDINE-SENSITIVE L-TYPE, CALCIUM CHANNEL ALPHA-2/DELTA SUBUNITS PRECURSOR."
1.6e-37:165:42
ORYCTOLAGUS CUNICULUS (RABBIT).
P13806
HRIFA008174a
COLLAGEN 1(X) CHAIN PRECURSOR.
4.5e-05:215:28
BOS TAURUS (BOVINE).
P23206
HRIFA008186a
ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
2.1e-25:118:46
HOMO SAPIENS (HUMAN).
P37058
HRIFA008200a
ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
7.9e-17:139:36
SACCHAROMYCES CEREVISIAE (BAKER'S YEAST).
P32802
HRIFA008212a
A-AGGLUTININ ATTACHMENT SUBUNIT PRECURSOR.
0.035:135:28
SACCHAROMYCES CEREVISIAE (BAKER'S YEAST).
P32323
HRIFA008252a
PROLINE-RICH PROTEIN MP-2 PRECURSOR.
0.00015:128:32
MUS MUSCULUS (MOUSE).
P05142
HRIFA008284a
NEURAL CELL ADHESION MOLECULE L1 PRECURSOR (N-CAM L1).
3.9e-18:153:30
HOMO SAPIENS (HUMAN).
P32004
HRIFA008314a
HYPOTHETICAL 149.7 KD PROTEIN IN IRE1-KSP1 INTERGENIC REGION.
2.1e-18:99:47
SACCHAROMYCES CEREVISIAE (BAKER'S YEAST).
P38800
HRIFA008362a
PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).
9.1e-42:135:57
GALLUS GALLUS (CHICKEN).
P53760
HRIFA008426a
HOMEODOMAIN PROTEIN AKR (AVIAN KNOTTED-RELATED PROTEIN).
1.3e-08:104:45
GALLUS GALLUS (CHICKEN).
Q90655
HRIFA008459a
CARBON CATABOLITE DEREPRESSING PROTEIN KINASE (EC 2.7.1.-).
5.5e-15:96:40
SACCHAROMYCES CEREVISIAE (BAKER'S YEAST).
P06782
HRIFA008483a
PROBABLE LONG-CHAIN FATTY ACID TRANSPORT PROTEIN.
7.4e-26:154:41
SACCHAROMYCES CEREVISIAE (BAKER'S YEAST).
P38225
HRIFA008547a
ZINC FINGER PROTEIN 136.
7.2e-57:228:50
HOMO SAPIENS (HUMAN).
P52737
HRIFA008596a
SKIN SECRETORY PROTEIN XP2 PRECURSOR (APEG PROTEIN).
1.6e-05:97:35
XENOPUS LAEVIS (AFRICAN CLAWED FROG).
P17437
HRIFA008611a
NPL1 PROTEIN (SEC63 PROTEIN).
8.1e-15:113:38
SACCHAROMYCES CEREVISIAE (BAKER'S YEAST).
P14906
HRIFA008661a
GALACTOSE-PROTON SYMPORT (GALACTOSE TRANSPORTER).
2.7e-16:184:29
ESCHERICHIA COLI.
P37021
HRIFA008717a
SERINE/THREONINE-PROTEIN KINASE NRK1 (EC 2.7.1.-) (N-RICH KINASE 1).
6.9e-32:198:41
SACCHAROMYCES CEREVISIAE (BAKER'S YEAST).
P38692
HRIFA008784a
HYPOTHETICAL 26.3 KD PROTEIN IN OYE2-GND1 INTERGENIC REGION.
2.2e-16:93:47
SACCHAROMYCES CEREVISIAE (BAKER'S YEAST).
P38869
HRIFA008790a
HYPOTHETICAL 15.7 KD PROTEIN IN NUP85-SSC1 INTERGENIC REGION.
4.2e-08:121:32
SACCHAROMYCES CEREVISIAE (BAKER'S YEAST).
P47111
HRIFA008976a
ACROSIN PRECURSOR (EC 3.4.21.10).
0.31:20:70
HOMO SAPIENS (HUMAN).
P10323
HRIFA008981 a
ZINC FINGER PROTEIN 85 (ZINC FINGER PROTEIN HPF4) (HTF1).
1.0e-84:126:74
HOMO SAPIENS (HUMAN).
Q03923
HRIFA008989a
TRANS-ACTING TRANSCRIPTIONAL PROTEIN ICP0 (IMMEDIATE-EARLY PROTEIN IE110) (VMW110) (ALPHA-0 PROTEIN).
1.2e-05:134:33
HERPES SIMPLEX VIRUS (TYPE 1 / STRAIN 17).
P08393
HRIFA009071 a
CELLULAR TUMOR ANTIGEN P53 (PHOSPHOPROTEIN P53).
0.14:104:31
HOMO SAPIENS (HUMAN).
P04637
HRIFA009101 a
ZINC FINGER PROTEIN 136.
6.5e-47:126:67
HOMO SAPIENS (HUMAN).
P52737
HRIFA009123a
SKIN SECRETORY PROTEIN XP2 PRECURSOR (APEG PROTEIN).
0.010:127:35
XENOPUS LAEVIS (AFRICAN CLAWED FROG).
P17437
HRIFA009136a
REGULATORY PROTEIN E2.
0.032:100:37
HUMAN PAPILLOMAVIRUS TYPE 25.
P36787
HRIFA009171a
BUTYROPHILIN PRECURSOR (BT).
1.6e-15:168:31
BOS TAURUS (BOVINE).
P18892
HRIFA009220a
HYPOTHETICAL 43.7 KD PROTEIN C24B11.08C IN CHROMOSOME I.
2.2e-48:268:41
SCHIZOSACCHAROMYCES POMBE (FISSION YEAST).
Q09895
HRIFA009339a
PROCOLLAGEN ALPHA 2(I) CHAIN PRECURSOR.
0.63:57:35
MUS MUSCULUS (MOUSE).
Q01149
HRIFA009451 a
METALLOPROTEINASE INHIBITOR 1 PRECURSOR (TIMP-1) (ERYTHROID POTENTIATING ACTIVITY) (EPA) (TISSUE INHIBITOR OF METALLOPROTEINASES) (FIBROBLAST COLLAGENASE INHIBITOR) (COLLAGENASE INHIBITOR).
1.7e-57:163:73
HOMO SAPIENS (HUMAN).
P01033
HRIFA009482a
BETA-GALACTOSIDASE PRECURSOR (EC 3.2.1.23) (LACTASE) (ACID BETA-GALACTOSIDASE).
7.7e-25:86:59
MUS MUSCULUS (MOUSE).
P23780
HRIFA009578a
HYPOTHETICAL 24.5 KD PROTEIN IN SAP185-BCK1 INTERGENIC REGION.
8.8e-10:199:26
SACCHAROMYCES CEREVISIAE (BAKER'S YEAST).
P40857
HRIFA009762a
CCAAT DISPLACEMENT PROTEIN (CDP) (CDP2) (FRAGMENT).
0.17:116:32
RATTUS NORVEGICUS (RAT).
P53565
HRIFA009783a
HYPOTHETICAL 85.7 KD PROTEIN C13G6.03 IN CHROMOSOME I.
6.2e-48:231:48
SCHIZOSACCHAROMYCES POMBE (FISSION YEAST).
Q09782
HRIFA009825a
ANTER-SPECIFIC PROLINE-RICH PROTEIN APG PRECURSOR.
4.0e-06:70:38
ARABIDOPSIS THALIANA (MOUSE-EAR CRESS).
P40602
HRIFA009852a
"NEURAL CELL ADHESION MOLECULE 1, LARGE ISOFORM PRECURSOR (N-CAM 180) [CONTAINS: N-CAM 140]."
4.0e-07:198:27
XENOPUS LAEVIS (AFRICAN CLAWED FROG).
P16170
HRIFA009881a
EXTENSIN PRECURSOR (PROLINE-RICH GLYCOPROTEIN).
1.5e-11:106:35
SORGHUM VULGARE (SORGHUM).
P24152
HRIFA009983a
G-BOX BINDING FACTOR (GBF).
3.8e-10:156:30
DICTYOSTELIUM DISCOIDEUM (SLIME MOLD).
P36417
HRIFA010005a
"M PROTEIN, SEROTYPE 49 PRECURSOR."
1.6e-05:183:27
STREPTOCOCCUS PYOGENES.
P16947
HRIFA010078a
HYPOTHETICAL 57.5 KD PROTEIN IN VMA7-RPS25A INTERGENIC REGION.
4.7e-05:194:31
SACCHAROMYCES CEREVISIAE (BAKER'S YEAST).
P53214
HRIFA010085a
ZINC FINGER PROTEIN 85 (ZINC FINGER PROTEIN HPF4) (HTF1).
2.9e-92:243:69
HOMO SAPIENS (HUMAN).
Q03923
HRIFA010090a
N-ACETYLGLUCOSAMINE-6-SULFATASE PRECURSOR (EC 3.1.6.14) (G6S) (GLUCOSAMINE-6-SULFATASE).
6.7e-16:78:51
HOMO SAPIENS (HUMAN).
P15586
HRIFA010130a
DOLICHYL-PHOSPHATE-MANNOSE--PROTEIN MANNOSYLTRANSFERASE 4 (EC
2.4.1.109).
5.6e-13:99:34
SACCHAROMYCES CEREVISIAE (BAKER'S YEAST).
P46971
HRIFA010152a
"ADENYLATE CYCLASE, TYPE V (EC 4.6.1.1) (ATP PYROPHOSPHATE-LYASE) (CA(2+)-INHIBITABLE ADENYLYL CYCLASE)."
2.3e-05:73:43
CANIS FAMILIARIS (DOG).
P30803
HRIFA010176a
HEPATOCYTE NUCLEAR FACTOR 3-BETA (HNF-3B).
0.066:105:31
MUS MUSCULUS (MOUSE).
P35583
HRIFA010301a
ANTER-SPECIFIC PROLINE-RICH PROTEIN APG PRECURSOR.
1.1e-09:120:34
ARABIDOPSIS THALIANA (MOUSE-EAR CRESS).
P40602
HRIFA010319a
DOPAMINE-BETA-MONOOXYGENASE PRECURSOR (EC 1.14.17.1) (DOPAMINE BETA- HYDROXYLASE) (DBH).
4.8e-23:185:32
RATTUS NORVEGICUS (RAT).
Q05754
HRIFA010361a
SKIN SECRETORY PROTEIN XP2 PRECURSOR (APEG PROTEIN).
2.6e-08:136:32
XENOPUS LAEVIS (AFRICAN CLAWED FROG).
P17437
HRIFA010394a
HYPOTHETICAL 51.5 KD PROTEIN D2024.3 IN CHROMOSOME IV.
3.3e-36:144:47
CAENORHABDITIS ELEGANS.
P49191
HRIFA010425a
A-AGGLUTININ ATTACHMENT SUBUNIT PRECURSOR.
1.9e-09:199:29
SACCHAROMYCES CEREVISIAE (BAKER'S YEAST).
P32323
HRIFA010460a
TRANSCRIPTIONAL ACTIVATOR FE65.
2.3e-27:101:54
RATTUS NORVEGICUS (RAT).
P46933
HRIFA010466a
SKIN SECRETORY PROTEIN XP2 PRECURSOR (APEG PROTEIN).
5.3e-07:123:34
XENOPUS LAEVIS (AFRICAN CLAWED FROG).
P17437
HRIFA010490a
TRANS-ACTING TRANSCRIPTIONAL PROTEIN ICP0 (EARLY PROTEIN 0) (EP0).
0.0031:118:30
PSEUDORABIES VIRUS (STRAIN INDIANA-FUNKHAUSER / BECKER) (PRV).
P29129
HRIFA010736a
PROTEIN Q300.
0.018:14:85
MUS MUSCULUS (MOUSE).
Q02722
HRIFA010790a
RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
1.6e-82:197:72
HOMO SAPIENS (HUMAN).
Q06495
HRIFA010799a
PROCOLLAGEN ALPHA 1(I) CHAIN PRECURSOR.
1.7e-05:220:30
GALLUS GALLUS (CHICKEN).
P02457
HRIFA010859a
ALPHA-2C-1 ADRENERGIC RECEPTOR (ALPHA-2C-1 ADRENOCEPTOR) (SUBTYPE C4).
0.063:134:33
HOMO SAPIENS (HUMAN).
P18825
HRIFA010891a
HYPOTHETICAL 22.7 KD PROTEIN IN PAS1-MST1 INTERGENIC REGION.
0.044:28:64
SACCHAROMYCES CEREVISIAE (BAKER'S YEAST).
P36015
HRIFA010975a
TYROSINE-PROTEIN KINASE SYK (EC 2.7.1.112) (SPLEEN TYROSINE KINASE).
8.5e-113:144:86
HOMO SAPIENS (HUMAN).
P43405
HRIFA010988a
GASTRIN PRECURSOR.
0.084:59:37
HOMO SAPIENS (HUMAN).
P01350
HRIFA011016a
PROTEIN DISULFIDE ISOMERASE-RELATED PROTEIN PRECURSOR (ERP72) (CALCIUM-BINDING PROTEIN 2) (CABP2).
3.1e-15:127:37
RATTUS NORVEGICUS (RAT).
P38659
HRIFA011105a
SALIVARY GLUE PROTEIN SGS-7 PRECURSOR.
0.97:41:43
DROSOPHILA MELANOGASTER (FRUIT FLY).
P02841
HRIFA011128a
GLYCINE-RICH CELL WALL STRUCTURAL PROTEIN (CLONE W10-1) (FRAGMENT).
0.0046:30:63
LYCOPERSICON ESCULENTUM (TOMATO).
Q01157
HRIFA011179a
PROBABLE SERINE/THREONINE-PROTEIN KINASE YKL101W (EC 2.7.1.-).
1.1e-20:127:42
SACCHAROMYCES CEREVISIAE (BAKER'S YEAST).
P34244
HRIFA011197a
DIPEPTIDYL PEPTIDASE IV (EC 3.4.14.5) (DPP IV) (THYMOCYTE-ACTIVATING MOLECULE) (THAM).
5.8e-26:169:40
MUS MUSCULUS (MOUSE).
P28843
HRIFA011449a
GALACTOSYLTRANSFERASE ASSOCIATED PROTEIN KINASE P58/GTA (EC 2.7.1.-).
1.9e-26:109:53
MUS MUSCULUS (MOUSE).
P24788
HRIFA011484a
D(4) DOPAMINE RECEPTOR (D(2C) DOPAMINE RECEPTOR).
0.00055:115:33
HOMO SAPIENS (HUMAN).
P21917
HRIFA011512a
POSSIBLE GLOBAL TRANSCRIPTION ACTIVATOR SNF2L2 (SNF2-ALPHA).
0.00024:139:25
HOMO SAPIENS (HUMAN).
P51531
HRIFA011580a
VITELLOGENIN II PRECURSOR (MAJOR VITELLOGENIN) [CONTAINS:
LIPOVITELLIN I (LVI) PHOSVITIN (PV) LIPOVITELLIN II (LVII) YGP40].
4.0e-08:182:32
GALLUS GALLUS (CHICKEN).
P02845
HRIFA011659a
VON WILLEBRAND FACTOR PRECURSOR.
9.8e-17:210:25
HOMO SAPIENS (HUMAN).
P04275
HRIFA011820a
ZINC FINGER PROTEIN 136.
1.9e-10:42:73
HOMO SAPIENS (HUMAN).
P52737
HRIFA011926a
TRANSCRIPTIONAL REGULATORY PROTEIN ALGP (ALGINATE REGULATORY PROTEIN ALGR3).
1.0:149:22
PSEUDOMONAS AERUGINOSA.
P15276
HRIFA011947a
ZINC FINGER PROTEIN 136.
1.3e-80:180:72
HOMO SAPIENS (HUMAN).
P52737
HRIFA012069a
A-AGGLUTININ ATTACHMENT SUBUNIT PRECURSOR.
0.0027:205:28
SACCHAROMYCES CEREVISIAE (BAKER'S YEAST).
P32323
HRIFA012151a
NEUROGENIC LOCUS NOTCH HOMOLOG PROTEIN 1 PRECURSOR.
0.00028:72:37
RATTUS NORVEGICUS (RAT).
Q07008
HRIFA012167a
HYPOTHETICAL SYMPORTER IN GLTS-SELC INTERGENIC REGION.
6.4e-09:145:28
ESCHERICHIA COLI.
P31435
HRIFA012278a
ZINC FINGER PROTEIN 140.
3.1e-14:88:52
HOMO SAPIENS (HUMAN).
P52738
HRIFA012354a
"SODIUM CHANNEL PROTEIN, BRAIN II ALPHA SUBUNIT."
2.1e-05:120:32
RATTUS NORVEGICUS (RAT).
P04775
HRIFA012427a
PROCOLLAGEN ALPHA 1(I) CHAIN PRECURSOR.
6.3e-08:250:28
MUS MUSCULUS (MOUSE).
P11087
HRIFA012436a
INTESTINAL MEMBRANE A4 PROTEIN (DIFFERENTIATION-DEPENDENT PROTEIN A4).
4.7e-09:95:31
HOMO SAPIENS (HUMAN).
Q04941
HRIFA012515a
SODIUM/GLUCOSE COTRANSPORTER 1 (NA(+)/GLUCOSE COTRANSPORTER 1) (HIGH AFFINITY SODIUM-GLUCOSE COTRANSPORTER).
3.5e-06:181:27
ORYCTOLAGUS CUNICULUS (RABBIT).
P11170
HRIFA012584a
MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
4.9e-14:136:29
NEUROSPORA CRASSA.
P23231
HRIFA012625a
"HIGH AFFINITY IMMUNOGLOBULIN EPSILON RECEPTOR BETA-SUBUNIT (FCERI) (IGE FC RECEPTOR, BETA-SUBUNIT)."
9.6e-12:103:40
RATTUS NORVEGICUS (RAT).
P13386
HRIFA012692a
BLOOM'S SYNDROME PROTEIN.
6.3e-26:203:34
HOMO SAPIENS (HUMAN).
P54132
HRIFA012702a
EXTENSIN PRECURSOR (PROLINE-RICH GLYCOPROTEIN).
1.9e-07:153:30
ZEA MAYS (MAIZE).
P14918
HRIFA012737a
LEUCOCYTE ANTIGEN CD97 PRECURSOR.
1.6e-09:170:24
HOMO SAPIENS (HUMAN).
P48960
HRIFA012795a
VOLTAGE-GATED POTASSIUM CHANNEL PROTEIN KV2.1 (DRK1).
3.0e-34:189:39
RATTUS NORVEGICUS (RAT).
P15387
HRIFA012885a
HYPOTHETICAL 30.6 KD PROTEIN IN SCP160-SMC3 INTERGENIC REGION PRECURSOR.
2.9e-21:159:40
SACCHAROMYCES CEREVISIAE (BAKER'S YEAST).
P47032
HRIFA012914a
ENV POLYPROTEIN PRECURSOR (COAT POLYPROTEIN) [CONTAINS: OUTER MEMBRANE PROTEIN GP70 TRANSMEMBRANE PROTEIN P20E].
3.4e-29:134:47
BABOON ENDOGENOUS VIRUS (STRAIN M7).
P10269
HRIFA012969a
ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
1.2e-30:228:32
SACCHAROMYCES CEREVISIAE (BAKER'S YEAST).
P32802
HRIFA012990a
PROBABLE CALCIUM-TRANSPORTING ATPASE 6 (EC 3.6.1.38).
7.4e-20:181:33
SACCHAROMYCES CEREVISIAE (BAKER'S YEAST).
P39986
HRIFA013092a
OUTER MEMBRANE PROTEIN H.8 PRECURSOR.
0.0039:51:39
NEISSERIA GONORRHOEAE.
P11910
HRIFA013103a
N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
0.25:50:34
MUS MUSCULUS (MOUSE).
P15535
HRIFA013135a
CELL SURFACE GLYCOPROTEIN 1 PRECURSOR (OUTER LAYER PROTEIN B) (S-LAYER PROTEIN 1).
1.6e-05:214:28
CLOSTRIDIUM THERMOCELLUM.
Q06852
HRIFA013235a
PROCOLLAGEN ALPHA 1(III) CHAIN PRECURSOR.
1.9e-05:113:40
HOMO SAPIENS (HUMAN).
P02461
HRIFA013254a
COMPLEMENT C4 PRECURSOR [CONTAINS: C4A ANAPHYLATOXIN].
3.8e-13:123:41
MUS MUSCULUS (MOUSE).
P01029
HRIFA013265a
CATHEPSIN L PRECURSOR (EC 3.4.22.15) (MAJOR EXCRETED PROTEIN) (MEP).
7.0e-107:225:86
HOMO SAPIENS (HUMAN).
P07711
HRIFA013276a
5'-NUCLEOTIDASE PRECURSOR (EC 3.1.3.5) (ECTO-NUCLEOTIDASE) (5'-NT) (CD73 ANTIGEN).
2.2e-117:270:85
HOMO SAPIENS (HUMAN).
P21589
HRIFA013279a
CIRCUMSPOROZOITE PROTEIN PRECURSOR (CS).
4.9e-05:127:37
PLASMODIUM VIVAX.
P08677
HRIFA013376a
MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
8.0e-23:230:31
NEUROSPORA CRASSA.
P23231
HRIFA013477a
OX-2 MEMBRANE GLYCOPROTEIN PRECURSOR (FRAGMENT).
5.8e-87:197:87
HOMO SAPIENS (HUMAN).
P41217
HRIFA013586a
ENDOZEPINE-RELATED PROTEIN PRECURSOR (MEMBRANE-ASSOCIATED DIAZEPAM BINDING INHIBITOR) (MA-DBI).
3.8e-31:93:64
BOS TAURUS (BOVINE).
P07106
HRIFA013589a
T-CELL SURFACE PROTEIN TACTILE PRECURSOR (CD96 ANTIGEN).
5.0e-06:95:35
HOMO SAPIENS (HUMAN).
P40200
HRIFA013620a
"HIGH AFFINITY IMMUNOGLOBULIN EPSILON RECEPTOR BETA-SUBUNIT (FCERI) (IGE FC RECEPTOR, BETA-SUBUNIT)."
7.1e-08:95:37
MUS MUSCULUS (MOUSE).
P20490
HRIFA013726a
SERINE/THREONINE-PROTEIN KINASE STE20 (EC 2.7.1.-).
1.5e-33:99:50
SACCHAROMYCES CEREVISIAE (BAKER'S YEAST).
Q03497
HRIFA013744a
ENDOZEPINE-RELATED PROTEIN PRECURSOR (MEMBRANE-ASSOCIATED DIAZEPAM BINDING INHIBITOR) (MA-DBI).
7.5e-15:105:38
BOS TAURUS (BOVINE).
P07106
HRIFA013911a
BIOTINIDASE PRECURSOR (EC 3.5.1.12).
7.8e-37:104:46
HOMO SAPIENS (HUMAN).
P43251
HRIFA013919a
MUCIN 2 PRECURSOR (INTESTINAL MUCIN 2).
1.2e-10:170:32
HOMO SAPIENS (HUMAN).
Q02817
HRIFA013932a
"SALIVARY PROLINE-RICH PROTEIN PRECURSOR (CLONES CP3, CP4 AND CP5) [CONTAINS: BASIC PEPTIDE IB-6" PEPTIDE P-H].
2.6e-05:168:34
HOMO SAPIENS (HUMAN).
P04280
HRIFA013980a
TRANSCRIPTION REGULATORY PROTEIN SNF5 (SWI/SNF COMPLEX COMPONENT SNF5) (TRANSCRIPTION FACTOR TYE4).
0.00036:157:27
SACCHAROMYCES CEREVISIAE (BAKER'S YEAST).
P18480
HRIFA014006a
T-CELL SURFACE GLYCOPROTEIN YE1/48 (T LYMPHOCYTE ANTIGEN A1) (LY49-A ANTIGEN).
9.4e-16:185:28
MUS MUSCULUS (MOUSE).
P20937
HRIFA014024a
TRANS-ACTING TRANSCRIPTIONAL PROTEIN ICP0 (P135 PROTEIN) (IER 2.9/ER2.6).
0.0013:102:44
BOVINE HERPESVIRUS TYPE 1 (STRAIN JURA).
P29128
HRIFA014056a
PROTEIN Q300.
5.1e-05:24:70
MUS MUSCULUS (MOUSE).
Q02722
HRIFA014111a
TOLL PROTEIN PRECURSOR.
5.5e-08:203:27
DROSOPHILA MELANOGASTER (FRUIT FLY).
P08953
HRIFA014133a
PROLINE-RICH PROTEIN MP-2 PRECURSOR.
1.6e-06:143:33
MUS MUSCULUS (MOUSE).
P05142
HRIFA014185a
LEUCOCYTE ANTIGEN CD97 PRECURSOR.
6.0e-14:192:30
HOMO SAPIENS (HUMAN).
P48960
HRIFA014336a
"GELSOLIN PRECURSOR, PLASMA (ACTIN-DEPOLYMERIZING FACTOR) (ADF) (BREVIN) (FRAGMENT)."
2.8e-70:198:58
SUS SCROFA (PIG).
P20305
HRIFA014396a
CREB-BINDING PROTEIN.
2.6e-07:101:34
MUS MUSCULUS (MOUSE).
P45481
HRIFA014397a
GENERAL NEGATIVE REGULATOR OF TRANSCRIPTION SUBUNIT 1.
5.2e-05:147:30
SACCHAROMYCES CEREVISIAE (BAKER'S YEAST).
P25655
HRIFA014465a
HYPOTHETICAL 59.1 KD PROTEIN ZK637.1 IN CHROMOSOME III.
2.8e-11:166:30
CAENORHABDITIS ELEGANS.
P30638
HRIFA014500a
HYPOTHETICAL 71.4 KD PROTEIN IN NMD3-EN02 INTERGENIC REGION.
1.0e-14:149:35
SACCHAROMYCES CEREVISIAE (BAKER'S YEAST).
P38862
HRIFA014561a
PROBABLE G PROTEIN-COUPLED RECEPTOR GPR1.
4.1e-70:156:89
HOMO SAPIENS (HUMAN).
P46091
HRIFA014568a
AMINOPEPTIDASE N (EC 3.4.11.2) (MICROSOMAL AMINOPEPTIDASE).
2.4e-40:196:44
RATTUS NORVEGICUS (RAT).
P15684
HRIFA014590a
ATP SYNTHASE PROTEIN 8 (EC 3.6.1.34) (A6L).
0.18:26:30
GALLUS GALLUS (CHICKEN).
P14093
HRIFA014598a
SKIN SECRETORY PROTEIN XP2 PRECURSOR (APEG PROTEIN).
4.9e-05:124:29
XENOPUS LAEVIS (AFRICAN CLAWED FROG).
P17437
HRIFA014620a
ENL PROTEIN.
0.58:170:30
HOMO SAPIENS (HUMAN).
Q03111
HRIFA014621a
PREGNANCY-SPECIFIC BETA-1 GLYCOPROTEIN PRECURSOR.
2.7e-50:150:74
HOMO SAPIENS (HUMAN).
P11462
HRIFA014688a
INTEGRIN BETA-6 SUBUNIT PRECURSOR.
6.9e-31:189:39
HOMO SAPIENS (HUMAN).
P18564
HRIFA014702a
PROLINE-RICH PROTEIN MP-2 PRECURSOR.
6.4e-05:89:40
MUS MUSCULUS (MOUSE).
P05142
HRIFA014819a
MICROFIBRIL-ASSOCIATED GLYCOPROTEIN 4.
7.8e-26:117:46
HOMO SAPIENS (HUMAN).
P55083
HRIFA014868a
SALIVARY GLUE PROTEIN SGS-3 PRECURSOR.
8.9e-08:195:29
DROSOPHILA ERECTA (FRUIT FLY).
P13730
HRIFA014951a
PLASMINOGEN (EC 3.4.21.7) (FRAGMENT).
4.1e-23:132:39
EQUUS CABALLUS (HORSE).
P80010
HRIFA014967a
CHLORINE CHANNEL PROTEIN P64.
2.0e-52:142:76
BOS TAURUS (BOVINE).
P35526
HRIFA015063a
ZINC FINGER PROTEIN 136.
6.6e-53:229:48
HOMO SAPIENS (HUMAN).
P52737
HRIFA015070a
SERINE/THREONINE-PROTEIN KINASE NRK1 (EC 2.7.1.-) (N-RICH KINASE 1).
9.3e-24:143:41
SACCHAROMYCES CEREVISIAE (BAKER'S YEAST).
P38692
HRIFA015122a
REGULATORY PROTEIN E2.
0.45:129:30
HUMAN PAPILLOMAVIRUS TYPE 5.
P06921
HRIFA015219a
FIBRILLIN 1 PRECURSOR (MP340).
9.9e-09:132:32
BOS TAURUS (BOVINE).
P98133
HRIFA015246a
PREGNANCY-SPECIFIC BETA-1-GLYCOPROTEIN 4 PRECURSOR (PSBG-4).
2.4e-33:184:46
HOMO SAPIENS (HUMAN).
Q00888
HRIFA015351a
PROTEOGLYCAN LINK PROTEIN PRECURSOR (CARTILAGE LINK PROTEIN) (LP).
0.0021:122:30
RATTUS NORVEGICUS (RAT).
P03994
HRIFA015423a
B-CELL LYMPHOMA 3-ENCODED PROTEIN (BCL-3 PROTEIN).
1.2e-11:148:35
HOMO SAPIENS (HUMAN).
P20749
HRIFA015453a
RAC-FAMILY SERINE/THREONINE KINASE HOMOLOG (EC 2.7.1.-).
6.8e-11:91:37
DICTYOSTELIUM DISCOIDEUM (SLIME MOLD).
P54644
HRIFA015486a
BETA-2-GLYCOPROTEIN I PRECURSOR (APOLIPOPROTEIN H) (APO-H) (ACTIVATED PROTEIN C-BINDING PROTEIN) (APC INHIBITOR).
2.0e-22:208:27
MUS MUSCULUS (MOUSE).
Q01339
HRIFA015506a
COLORECTAL MUTANT CANCER PROTEIN (MCC PROTEIN).
1.3e-12:73:50
HOMO SAPIENS (HUMAN).
P23508
HRIFA015536a
CHLORINE CHANNEL PROTEIN P64.
1.2e-49:115:79
BOS TAURUS (BOVINE).
P35526
HRIFA015547a
BEM46 PROTEIN (FRAGMENT).
1.4e-33:137:49
SCHIZOSACCHAROMYCES POMBE (FISSION YEAST).
P54069
HRIFA015568a
HYPOTHETICAL 35.8 KD PROTEIN C12G12.12 IN CHROMOSOME I.
2.4e-16:152:34
SCHIZOSACCHAROMYCES POMBE (FISSION YEAST).
Q09875
HRIFA015756a
EBNA-2 NUCLEAR PROTEIN.
2.9e-15:28:75
EPSTEIN-BARR VIRUS (STRAIN B95-8) (HUMAN HERPESVIRUS 4).
P12978
HRIFA015802a
PROTEOGLYCAN LINK PROTEIN PRECURSOR (CARTILAGE LINK PROTEIN) (LP).
0.0035:122:30
RATTUS NORVEGICUS (RAT).
P03994
HRIFA015811a
GLYCOSYLTRANSFERASE ALG2 (EC 2.4.1.-).
6.2e-39:171:43
SACCHAROMYCES CEREVISIAE (BAKER'S YEAST).
P43636
HRIFA015902a
A-AGGLUTININ ATTACHMENT SUBUNIT PRECURSOR.
0.0075:161:29
SACCHAROMYCES CEREVISIAE (BAKER'S YEAST).
P32323
HRIFA015947a
ZINC FINGER Y-CHROMOSOMAL PROTEIN 1.
0.035:98:28
MUS MUSCULUS (MOUSE).
P10925
HRIFA015995a
PROCOLLAGEN ALPHA 1(III) CHAIN PRECURSOR.
6.2e-08:221:37
HOMO SAPIENS (HUMAN).
P02461
HRIFA016070a
"COMPLEMENT C1Q SUBCOMPONENT, A CHAIN PRECURSOR."
1.0e-18:179:35
HOMO SAPIENS (HUMAN).
P02745
HRIFA016214a
PROLINE-RICH PROTEIN MP-2 PRECURSOR.
4.0e-05:96:42
MUS MUSCULUS (MOUSE).
P05142
HRIFA016240a
HYPOTHETICAL 65.3 KD PROTEIN IN PRE3-SAG1 INTERGENIC REGION.
8.5e-05:103:33
SACCHAROMYCES CEREVISIAE (BAKER'S YEAST).
P47082
HRIFA016255a
EBNA-1 NUCLEAR PROTEIN.
4.5e-09:219:33
EPSTEIN-BARR VIRUS (STRAIN B95-8) (HUMAN HERPESVIRUS 4).
P03211
HRIFA016290a
COAGULATION FACTOR V PRECURSOR (ACTIVATED PROTEIN C COFACTOR).
6.7e-21:182:41
HOMO SAPIENS (HUMAN).
P12259
HRIFA016430a
ER LUMEN PROTEIN RETAINING RECEPTOR 1 (KDEL RECEPTOR 1).
7.1e-50:120:86
HOMO SAPIENS (HUMAN).
P24390
HRIFA016599a
MEIOTIC RECOMBINATION PROTEIN REC104.
0.57:73:31
SACCHAROMYCES CEREVISIAE (BAKER'S YEAST).
P33323
HRIFA016639a
"GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE)."
8.0e-06:206:23
SACCHAROMYCES CEREVISIAE (BAKER'S YEAST).
P08640
HRIFA016654a
HEME-REGULATED EUKARYOTIC INITIATION FACTOR EIF-2-ALPHA KINASE (EC 2.7.1.-) (HRI).
1.1e-78:181:86
ORYCTOLAGUS CUNICULUS (RABBIT).
P33279
HRIFA016669a
ANTER-SPECIFIC PROLINE-RICH PROTEIN APG PRECURSOR.
1.4e-08:87:36
ARABIDOPSIS THALIANA (MOUSE-EAR CRESS).
P40602
HRIFA016758a
GLYCOSYLTRANSFERASE ALG2 (EC 2.4.1.-).
9.5e-17:158:40
SACCHAROMYCES CEREVISIAE (BAKER'S YEAST).
P43636
HRIFA016963a
FMRFAMIDE-RELATED NEUROPEPTIDES PRECURSOR.
6.2e-08:131:32
LYMNAEA STAGNALIS (GREAT POND SNAIL).
P42565
HRIFA017031a
MYOSIN HEAVY CHAIN KINASE A (EC 2.7.1.129) (MHCK A).
2.6e-11:152:34
DICTYOSTELIUM DISCOIDEUM (SLIME MOLD).
P42527
HRIFA017146a
D(4) DOPAMINE RECEPTOR (D(2C) DOPAMINE RECEPTOR).
0.0014:78:37
HOMO SAPIENS (HUMAN).
P21917
HRIFA017190a
FLI-1 ONCOGENE (ERGB TRANSCRIPTION FACTOR).
0.0026:89:30
HOMO SAPIENS (HUMAN).
Q01543
HRIFA017257a
"GELSOLIN PRECURSOR, PLASMA (ACTIN-DEPOLYMERIZING FACTOR) (ADF) (BREVIN) (AGEL)."
2.5e-79:261:57
HOMO SAPIENS (HUMAN).
P06396
HRIFA017295a
"ALPHA-1,6-MANNOSYL-GLYCOPROTEIN BETA-1,2-N-ACETYLGLUCOSAMINYLTRANSFERASE (EC 2.4.1.143) (N-GLYCOSYL-OLIGOSACCHARIDE-GLYCOPROTEIN N-ACETYLGLUCOSAMINYLTRANSFERASE
II) (BETA-1,2-N-ACETYLGLUCOSAMINYLTRANSFERASE II) (GNT-II) (GLCNAC-T II)."
3.4e-20:66:78
HOMO SAPIENS (HUMAN).
Q10469
HRIFA017312a
C4B-BINDING PROTEIN ALPHA CHAIN PRECURSOR (PROLINE-RICH PROTEIN) (PRP).
2.7e-19:221:33
HOMO SAPIENS (HUMAN).
P04003
HRIFA017456a
LAMININ ALPHA-1 CHAIN PRECURSOR (LAMININ A CHAIN).
0.11:94:35
MUS MUSCULUS (MOUSE).
P19137
HRIFA017457a
SYNAPTOTAGMIN II.
7.2e-07:98:35
MUS MUSCULUS (MOUSE).
P46097
HRIFA017643a
NOV PROTEIN HOMOLOG PRECURSOR (NOVH).
2.2e-07:81:41
HOMO SAPIENS (HUMAN).
P48745
HRIFA017670a
TRANS-GOLGI NETWORK INTEGRAL MEMBRANE PROTEIN TGN38 PRECURSOR.
4.9e-06:172:27
RATTUS NORVEGICUS (RAT).
P19814
HRIFA017703a
PUTATIVE SERINE/THREONINE-PROTEIN KINASE PKWA (EC 2.7.1.-).
1.9e-16:129:34
THERMOMONOSPORA CURVATA.
P49695
HRIFA017791a
MUCIN 2 PRECURSOR (INTESTINAL MUCIN 2).
0.012:71:38
HOMO SAPIENS (HUMAN).
Q02817
HRIFA017801a
PROLINE-RICH PROTEIN MP-2 PRECURSOR.
4.5e-07:86:39
MUS MUSCULUS (MOUSE).
P05142
HRIFA017818a
ATP SYNTHASE C CHAIN (EC 3.6.1.34) (LIPID-BINDING PROTEIN).
1.0:32:40
STREPTOMYCES LIVIDANS.
P50014
HRIFA017836a
"TETRACYCLINE RESISTANCE PROTEIN, CLASS H (TETA(H))."
1.3e-08:113:31
PASTEURELLA MULTOCIDA.
P51564
HRIFA017855a
ORM1 PROTEIN.
1.7e-18:137:35
SACCHAROMYCES CEREVISIAE (BAKER'S YEAST).
P53224
HRIFA017921a
TRANS-ACTING TRANSCRIPTIONAL PROTEIN ICP0 (IMMEDIATE-EARLY PROTEIN IE110) (VMW110) (ALPHA-0 PROTEIN).
2.0e-09:182:35
HERPES SIMPLEX VIRUS (TYPE 1 / STRAIN 17).
P08393
HRIFA018092a
"DIACYLGLYCEROL CHOLINEPHOSPHOTRANSFERASE (EC 2.7.8.2) (SN-1,2-DIACYLGLYCEROL CHOLINEPHOSPHOTRANSFERASE) (CHOPT)."
2.1e-20:119:42
SACCHAROMYCES CEREVISIAE (BAKER'S YEAST).
P17898
HRIFA018131a
ORM1 PROTEIN.
2.6e-20:137:37
SACCHAROMYCES CEREVISIAE (BAKER'S YEAST).
P53224
HRIFA018134a
SERINE/THREONINE-PROTEIN KINASE CTR1 (EC 2.7.1.37).
1.1e-11:147:32
ARABIDOPSIS THALIANA (MOUSE-EAR CRESS).
Q05609
HRIFA018238a
NEUROGENIC LOCUS NOTCH HOMOLOG PROTEIN 1 PRECURSOR (MOTCH PROTEIN).
8.6e-06:74:44
MUS MUSCULUS (MOUSE).
Q01705
HRIFA018262a
PUTATIVE SERINE/THREONINE-PROTEIN KINASE PKWA (EC 2.7.1.-).
6.4e-10:71:38
THERMOMONOSPORA CURVATA.
P49695
HRIFA018287a
HYPOTHETICAL 57.5 KD PROTEIN IN VMA7-RPS25A INTERGENIC REGION.
1.5e-06:214:32
SACCHAROMYCES CEREVISIAE (BAKER'S YEAST).
P53214
HRIFA018447a
SKIN SECRETORY PROTEIN XP2 PRECURSOR (APEG PROTEIN).
0.00065:133:33
XENOPUS LAEVIS (AFRICAN CLAWED FROG).
P17437
HRIFA018580a
COP-COATED VESICLE MEMBRANE PROTEIN P24 PRECURSOR (FRAGMENT).
2.1e-18:109:41
CRICETULUS GRISEUS (CHINESE HAMSTER).
P49020
HRIFA018666a
PROTEIN-TYROSINE PHOSPHATASE DLAR PRECURSOR (EC 3.1.3.48) (PROTEIN-TYROSINE-PHOSPHATE PHOSPHOHYDROLASE).
1.7e-06:191:28
DROSOPHILA MELANOGASTER (FRUIT FLY).
P16621
HRIFA018688a
PHLB PROTEIN PRECURSOR.
1.9e-06:110:35
SERRATIA LIQUEFACIENS.
P18954
HRIFA018754a
"GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE)."
1.8e-06:195:27
SACCHAROMYCES CEREVISIAE (BAKER'S YEAST).
P08640
HRIFA018794a
MSP1 PROTEIN HOMOLOG.
3.2e-06:93:25
CAENORHABDITIS ELEGANS.
P54815
HRIFA018827a
HYPOTHETICAL 59.1 KD PROTEIN ZK637.1 IN CHROMOSOME III.
3.1e-17:180:28
CAENORHABDITIS ELEGANS.
P30638
HRIFA018870a
HYPOTHETICAL 35.6 KD PROTEIN IN SPC1-ILV3 INTERGENIC REGION.
4.7e-09:70:37
SACCHAROMYCES CEREVISIAE (BAKER'S YEAST).
P47088
HRIFA018904a
MYOTONIN-PROTEIN KINASE (EC 2.7.1.-) (MYOTONIC DISTROPHY PROTEIN KINASE) (MDPK) (DM-KINASE) (DMK) (DMPK) (MT-PK).
5.5e-12:142:32
HOMO SAPIENS (HUMAN).
Q09013
HRIFA018931a
ZINC FINGER PROTEIN 140.
2.9e-10:47:74
HOMO SAPIENS (HUMAN).
P52738
HRIFA018993a
HYPOTHETICAL 21.5 KD PROTEIN IN SEC15-SAP4 INTERGENIC REGION.
1.2e-13:117:34
SACCHAROMYCES CEREVISIAE (BAKER'S YEAST).
P53073
HRIFA019105a
DORSAL-VENTRAL PATTERNING TOLLOID PROTEIN PRECURSOR (EC 3.4.24.-).
7.5e-22:203:32
DROSOPHILA MELANOGASTER (FRUIT FLY).
P25723
HRIFA019136a
"MYRISTOYLATED ALANINE-RICH C-KINASE SUBSTRATE (MARCKS) (PROTEIN KINASE C SUBSTRATE, 80 KD PROTEIN, LIGHT CHAIN) (PKCSL) (80K-L PROTEIN)."
1.0e-25:74:81
HOMO SAPIENS (HUMAN).
P29966
HRIFA019175a
PROTEIN KINASE WIS1 (EC 2.7.1.-).
1.3e-14:84:39
SCHIZOSACCHAROMYCES POMBE (FISSION YEAST).
P33886
HRIFA019262a
ZINC FINGER PROTEIN 85 (ZINC FINGER PROTEIN HPF4) (HTF1).
2.5e-55:188:50
HOMO SAPIENS (HUMAN).
Q03923
HRIFA019412a
CATHEPSIN E PRECURSOR (EC 3.4.23.34).
1.4e-09:121:33
CAVIA PORCELLUS (GUINEA PIG).
P25796
HRIFA019437a
REGULATORY PROTEIN E2.
0.26:77:37
HUMAN PAPILLOMAVIRUS TYPE 14.
P36783
HRIFA019466a
EBNA-1 NUCLEAR PROTEIN.
2.7e-19:130:43
EPSTEIN-BARR VIRUS (STRAIN B95-8) (HUMAN HERPESVIRUS 4).
P03211
HRIFA019490a
TRANSCRIPTION REGULATORY PROTEIN SNF5 (SWI/SNF COMPLEX COMPONENT SNF5) (TRANSCRIPTION FACTOR TYE4).
1.1e-09:132:34
SACCHAROMYCES CEREVISIAE (BAKER'S YEAST).
P18480
HRIFA019498a
VOLTAGE-GATED POTASSIUM CHANNEL PROTEIN SHAL (SHAL2).
5.6e-05:87:36
DROSOPHILA MELANOGASTER (FRUIT FLY).
P17971
HRIFA019532a
EBNA-1 NUCLEAR PROTEIN.
1.8e-05:67:49
EPSTEIN-BARR VIRUS (STRAIN B95-8) (HUMAN HERPESVIRUS 4).
P03211
HRIFA019651a
ACIDIC PHOSPHOPROTEIN PRECURSOR (50 KD ANTIGEN).
6.1e-05:31:64
PLASMODIUM CHABAUDI.
Q02752
HRIFA019867a
RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
8.2e-34:103:71
RATTUS NORVEGICUS (RAT).
Q06496
HRIFA019869a
SERINE/THREONINE-PROTEIN KINASE FUSED (EC 2.7.1.-).
7.2e-29:83:49
DROSOPHILA MELANOGASTER (FRUIT FLY).
P23647
HRIFA019958a
REPRESSOR PROTEIN CI (FRAGMENT).
0.99:45:37
BACTERIOPHAGE 434.
P16117
HRIFA020144a
SKIN SECRETORY PROTEIN XP2 PRECURSOR (APEG PROTEIN).
2.8e-06:176:30
XENOPUS LAEVIS (AFRICAN CLAWED FROG).
P17437
HRIFA020184a
NON-RECEPTOR TYROSINE KINASE SPORE LYSIS A (EC 2.7.1.112) (TYROSINE-PROTEIN KINASE 1).
1.9e-10:102:37
DICTYOSTELIUM DISCOIDEUM (SLIME MOLD).
P18160
HRIFA020272a
MUSCARINIC ACETYLCHOLINE RECEPTOR M3.
5.5e-91:211:85
HOMO SAPIENS (HUMAN).
P20309
HRIFA020335a
PUTATIVE SERINE/THREONINE-PROTEIN KINASE P78 (EC 2.7.1.-).
5.0e-104:275:72
HOMO SAPIENS (HUMAN).
P27448
HRIFA020349a
BRITTLE-1 PROTEIN PRECURSOR.
6.0e-30:214:35
ZEA MAYS (MAIZE).
P29518
HRIFA020453a
PROTEIN TRANSPORT PROTEIN SEC22 (PROTEIN SLY2).
2.5e-08:132:28
SACCHAROMYCES CEREVISIAE (BAKER'S YEAST).
P22214
HRIFA020693a
TUMOR SUPPRESSOR PROTEIN DCC PRECURSOR (COLORECTAL CANCER SUPPRESSOR).
3.9e-09:96:35
HOMO SAPIENS (HUMAN).
P43146
HRIFA020707a
PROCYCLIC FORM SPECIFIC POLYPEPTIDE B-ALPHA PRECURSOR (PROCYCLIN) (PARP B-ALPHA) (PSSA-1).
3.4e-09:95:33
TRYPANOSOMA BRUCEI BRUCEI.
Q06084
HRIFA020748a
EXTENSIN PRECURSOR (CELL WALL HYDROXYPROLINE-RICH GLYCOPROTEIN).
3.2e-09:210:28
NICOTIANA TABACUM (COMMON TOBACCO).
P13983
HRIFA020862a
MODIFIER 3 PROTEIN (M33).
5.6e-26:76:61
MUS MUSCULUS (MOUSE).
P30658
HRIFA020883a
PROTEIN Q300.
0.00054:21:66
MUS MUSCULUS (MOUSE).
Q02722
HRIFA021007a
TRANS-ACTING TRANSCRIPTIONAL PROTEIN ICP0 (IMMEDIATE-EARLY PROTEIN IE110) (VMW110) (ALPHA-0 PROTEIN).
0.092:73:36
HERPES SIMPLEX VIRUS (TYPE 1 / STRAIN 17).
P08393
HRIFA021040a
TRANSCRIPTION FACTOR GATA-4 (GATA BINDING FACTOR-4).
0.98:63:39
HOMO SAPIENS (HUMAN).
P43694
HRIFA021061a
SKIN SECRETORY PROTEIN XP2 PRECURSOR (APEG PROTEIN).
2.8e-09:162:31
XENOPUS LAEVIS (AFRICAN CLAWED FROG).
P17437
HRIFA021213a
OLIGOSACCHARYL TRANSFERASE STT3 SUBUNIT HOMOLOG.
2.0e-38:96:72
CAENORHABDITIS ELEGANS.
P46975
HRIFA021224a
RENAL TRANSCRIPTION FACTOR KID-1 (TRANSCRIPTION FACTOR 17).
2.8e-06:55:52
RATTUS NORVEGICUS (RAT).
Q02975
HRIFA021398a
COAGULATION FACTOR VII PRECURSOR (EC 3.4.21.21).
2.5e-17:78:51
ORYCTOLAGUS CUNICULUS (RABBIT).
P98139
HRIFA021445a
PRE-B-CELL LEUKEMIA TRANSCRIPTION FACTOR-1 (HOMEOBOX PROTEIN PBX1) (HOMEOBOX PROTEIN PRL).
0.38:146:31
HOMO SAPIENS (HUMAN).
P40424
HRIFA021494a
EBNA-1 NUCLEAR PROTEIN.
6.8e-07:116:41
EPSTEIN-BARR VIRUS (STRAIN B95-8) (HUMAN HERPESVIRUS 4).
P03211
HRIFA021499a
CARTILAGE MATRIX PROTEIN PRECURSOR (MATRILIN-1).
7.1e-34:159:50
GALLUS GALLUS (CHICKEN).
P05099
HRIFA021543a
ANTITHROMBIN-III PRECURSOR (ATIII) (FRAGMENT).
0.0087:50:40
GALLUS GALLUS (CHICKEN).
Q03352
HRIFA021620a
PLATELET FACTOR 4 (PF-4).
0.019:65:27
SUS SCROFA (PIG).
P30034
HRIFA021637a
CARTILAGE MATRIX PROTEIN PRECURSOR (MATRILIN-1).
6.0e-37:147:53
GALLUS GALLUS (CHICKEN).
P05099
HRIFA021651a
CARG-BINDING FACTOR-A (CBF-A).
2.6e-11:170:30
MUS MUSCULUS (MOUSE).
Q99020
HRIFA021754a
CARTILAGE MATRIX PROTEIN PRECURSOR (MATRILIN-1).
1.2e-37:137:51
GALLUS GALLUS (CHICKEN).
P05099
HRIFA021781a
DNA-REPAIR PROTEIN COMPLEMENTING XP-D CELLS (XERODERMA PIGMENTOSUM GROUP D COMPLEMENTING PROTEIN) (DNA EXCISION REPAIR PROTEIN ERCC-2).
7.1e-19:199:31
HOMO SAPIENS (HUMAN).
P18074
HRIFA021787a
PROTEIN Q300.
0.051:13:84
MUS MUSCULUS (MOUSE).
Q02722
HRIFA021794a
RAC-FAMILY SERINE/THREONINE KINASE HOMOLOG (EC 2.7.1.-).
1.6e-07:90:32
DICTYOSTELIUM DISCOIDEUM (SLIME MOLD).
P54644
HRIFA021855a
SKIN SECRETORY PROTEIN XP2 PRECURSOR (APEG PROTEIN).
8.6e-06:163:30
XENOPUS LAEVIS (AFRICAN CLAWED FROG).
P17437
HRIFA021906a
S-ANTIGEN PROTEIN PRECURSOR.
2.1e-09:226:28
PLASMODIUM FALCIPARUM (ISOLATE V1).
P09593
HRIFA022055a
BETA-LYTIC METALLOENDOPEPTIDASE PRECURSOR (EC 3.4.24.32) (BETA-LYTIC PROTEASE).
0.63:118:31
ACHROMOBACTER LYTICUS.
P27458
HRIFA022065a
BETA GALACTOSIDASE-RELATED PROTEIN PRECURSOR.
9.7e-24:235:34
HOMO SAPIENS (HUMAN).
P16279
HRIFA022139a
HYPOTHETICAL 85.7 KD PROTEIN C13G6.03 IN CHROMOSOME I.
2.1e-57:232:52
SCHIZOSACCHAROMYCES POMBE (FISSION YEAST).
Q09782
HRIFA022156a
"GLUTENIN, HIGH MOLECULAR WEIGHT SUBUNIT PW212 PRECURSOR."
1.4e-07:133:35
TRITICUM AESTIVUM (WHEAT).
P08489
HRIFA022166a
EXCISION REPAIR PROTEIN ERCC-6 (COCKAYNE SYNDROME PROTEIN CSB).
3.5e-28:194:35
HOMO SAPIENS (HUMAN).
Q03468
HRIFA022177a
PUTATIVE SERINE/THREONINE-PROTEIN KINASE PKWA (EC 2.7.1.-).
2.2e-12:137:32
THERMOMONOSPORA CURVATA.
P49695
HRIFA022182a
SERINE/THREONINE-PROTEIN KINASE MAK (EC 2.7.1.-) (MALE GERM CELL-ASSOCIATED KINASE).
1.2e-47:121:79
RATTUS NORVEGICUS (RAT).
P20793
HRIFA022203a
COLLAGEN ALPHA 1(III) CHAIN.
1.1e-05:211:33
BOS TAURUS (BOVINE).
P04258
HRIFA022227a
POTENTIAL CAAX PRENYL PROTEASE 1 (EC 3.4.24.-) (PRENYL PROTEIN- SPECIFIC ENDOPROTEASE 1) (PPSEP 1).
3.2e-31:229:36
SCHIZOSACCHAROMYCES POMBE (FISSION YEAST).
Q10071
HRIFA022234a
CARBOXYPEPTIDASE KEX1 PRECURSOR (EC 3.4.16.6) (CARBOXYPEPTIDASE D).
1.8e-08:110:30
SACCHAROMYCES CEREVISIAE (BAKER'S YEAST).
P09620
HRIFA022249a
ZINC FINGER PROTEIN 133.
1.1e-34:84:48
HOMO SAPIENS (HUMAN).
P52736
HRIFA022265a
CALCIUM/CALMODULIN-DEPENDENT PROTEIN KINASE TYPE IV CATALYTIC CHAIN (EC 2.7.1.123) (CAM KINASE-GR) (CAMK IV) [CONTAINS: CALSPERMIN]. 5.1e-26:188:40
RATTUS NORVEGICUS (RAT).
P13234
HRIFA022328a
SCO1 PROTEIN PRECURSOR.
5.4e-25:84:45
SACCHAROMYCES CEREVISIAE (BAKER'S YEAST).
P23833
HRIFA022335a
TRANS-ACTING TRANSCRIPTIONAL PROTEIN ICP0 (IMMEDIATE-EARLY PROTEIN IE110) (VMW110) (ALPHA-0 PROTEIN).
0.21:121:29
HERPES SIMPLEX VIRUS (TYPE 1 / STRAIN 17).
P08393
HRIFA022348a
AGAMOUS PROTEIN.
1.0:40:42
ARABIDOPSIS THALIANA (MOUSE-EAR CRESS).
P17839
HRIFA022411a
TRANS-ACTING TRANSCRIPTIONAL PROTEIN ICP0 (IMMEDIATE-EARLY PROTEIN IE110) (VMW110) (ALPHA-0 PROTEIN).
0.00059:111:35
HERPES SIMPLEX VIRUS (TYPE 1 / STRAIN 17).
P08393
HRIFA022423a
HYPOTHETICAL 24.5 KD PROTEIN IN SAP185-BCK1 INTERGENIC REGION.
2.5e-15:106:42
SACCHAROMYCES CEREVISIAE (BAKER'S YEAST).
P40857
HRIFA022462a
RETINOIC ACID RECEPTOR RXR-BETA.
0.0010:124:33
HOMO SAPIENS (HUMAN).
P28702
HRIFA022493a
ALPHA-2A ADRENERGIC RECEPTOR (ALPHA-2A ADRENOCEPTOR) (ALPHA-2AAR).
0.0018:130:34
MUS MUSCULUS (MOUSE).
Q01338
HRIFA022528a
EXTENSIN PRECURSOR (PROLINE-RICH GLYCOPROTEIN).
3.2e-23:230:28
ZEA MAYS (MAIZE).
P14918
HRIFA022546a
NINAC SHORT PROTEIN (EC 2.7.1.-).
8.5e-42:209:43
DROSOPHILA MELANOGASTER (FRUIT FLY).
P10677
HRIFA022564a
ZINC FINGER PROTEIN 140.
7.9e-23:116:51
HOMO SAPIENS (HUMAN).
P52738
HRIFA022616a
LOW-DENSITY LIPOPROTEIN RECEPTOR-RELATED PROTEIN 1 PRECURSOR (LRP) (ALPHA-2-MACROGLOBULIN RECEPTOR) (A2MR) (APOLIPOPROTEIN E RECEPTOR) (APOER) (CD91).
7.4e-36:172:43
HOMO SAPIENS (HUMAN).
Q07954
HRIFA022671 a
PAIRED AMPHIPATHIC HELIX PROTEIN.
2.0e-26:186:36
SACCHAROMYCES CEREVISIAE (BAKER'S YEAST).
P22579
HRIFA022691 a
FIBRINOGEN-LIKE PROTEIN A PRECURSOR (FREP-A).
1.4e-44:229:41
PARASTICHOPUS PARVIMENSIS (SEA CUCUMBER).
P19477
HRIFA022702a
PROCOLLAGEN ALPHA 1(I) CHAIN PRECURSOR.
1.1e-08:146:38
GALLUS GALLUS (CHICKEN).
P02457
HRIFA022707a
GC-RICH SEQUENCE DNA-BINDING FACTOR (GCF) (TRANSCRIPTION FACTOR 9) (TCF-9).
7.0e-40:229:37
HOMO SAPIENS (HUMAN).
P16383
HRIFA022714a
"AMELOGENIN, CLASS I PRECURSOR."
0.62:96:31
BOS TAURUS (BOVINE).
P02817
HRIFA022728a
ACROSIN PRECURSOR (EC 3.4.21.10) (53 KD FUCOSE-BINDING PROTEIN).
1.7e-06:28:64
SUS SCROFA (PIG).
P08001
HRIFA022729a
"ALPHA-1,6-MANNOSYL-GLYCOPROTEIN BETA-1,2-N-ACETYLGLUCOSAMINYLTRANSFERASE (EC 2.4.1.143) (N-GLYCOSYL-OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II)."
7.7e-29:69:84
HOMO SAPIENS (HUMAN).
Q10469
HRIFA022737a
TENASCIN PRECURSOR (TN) (HEXABRACHION) (CYTOTACTIN) (NEURONECTIN) (GMEM) (JI) (MIOTENDINOUS ANTIGEN) (GLIOMA-ASSOCIATED-EXTRACELLULAR MATRIX ANTIGEN) (GP 150-225).
6.7e-19:170:37
GALLUS GALLUS (CHICKEN).
P10039
HRIFA022776a
PROBABLE PROTEIN DISULFIDE ISOMERASE P5 PRECURSOR (EC 5.3.4.1).
4.0e-20:199:31
MEDICAGO SATIVA (ALFALFA).
P38661
HRIFA022782a
CIRCUMSPOROZOITE PROTEIN PRECURSOR (CS).
3.7e-09:184:36
PLASMODIUM CYNOMOLGI (STRAIN BEROK).
P08672
HRIFA022865a
COLLAGEN ALPHA 1(III) CHAIN.
2.5e-09:169:33
BOS TAURUS (BOVINE).
P04258
HRIFA022875a
BONE PROTEOGLYCAN II PRECURSOR (PG-S2) (DECORIN).
9.1e-14:115:33
BOS TAURUS (BOVINE).
P21793
HRIFA022890a
SKIN SECRETORY PROTEIN XP2 PRECURSOR (APEG PROTEIN).
1.8e-10:237:30
XENOPUS LAEVIS (AFRICAN CLAWED FROG).
P17437
HRIFA022895a
ZINC FINGER PROTEIN 85 (ZINC FINGER PROTEIN HPF4) (HTF1).
2.4e-106:283:67
HOMO SAPIENS (HUMAN).
Q03923
HRIFA022985a
PROCYCLIC FORM SPECIFIC POLYPEPTIDE B-ALPHA PRECURSOR (PROCYCLIN) (PARP B-ALPHA) (PSSA-1).
3.0e-10:33:72
TRYPANOSOMA BRUCEI BRUCEI.
Q06084
HRIFA023007a
MHC CLASS II REGULATORY FACTOR RFX1 (RFX) (ENHANCER FACTOR C) (EF-C).
1.1e-27:66:54
HOMO SAPIENS (HUMAN).
P22670
HRIFA023048a
COLLAGEN ALPHA 1(I) CHAIN (FRAGMENTS).
2.2e-07:221:33
RATTUS NORVEGICUS (RAT).
P02454
HRIFA023069a
BASEMENT MEMBRANE-SPECIFIC HEPARAN SULFATE PROTEOGLYCAN CORE PROTEIN PRECURSOR (HSPG) (PERLECAN) (PLC).
3.4e-08:149:31
HOMO SAPIENS (HUMAN).
P98160
HRIFA023129a
HISTIDINE-RICH GLYCOPROTEIN PRECURSOR.
4.2e-06:37:51
PLASMODIUM LOPHURAE.
P04929
HRIFA023154a
GLYCOPROTEIN X PRECURSOR.
8.2e-05:140:27
EQUINE HERPESVIRUS TYPE 1 (STRAIN AB4P) (EHV-1).
P28968
HRIFA023212a
A-AGGLUTININ ATTACHMENT SUBUNIT PRECURSOR.
8.3e-10:249:32
SACCHAROMYCES CEREVISIAE (BAKER'S YEAST).
P32323
HRIFA023227a
GALACTOSE-PROTON SYMPORT (GALACTOSE TRANSPORTER).
9.2e-15:180:30
ESCHERICHIA COLI.
P37021
HRIFA023257a
PROTEIN TRANSPORT PROTEIN SEC61 ALPHA SUBUNIT.
2.4e-118:229:88
RATTUS NORVEGICUS (RAT).
P38378
HRIFA023304a
PROBABLE CALCIUM-TRANSPORTING ATPASE 3 (EC 3.6.1.38) (ENDOPLASMIC RETICULUM CA2+-ATPASE).
1.3e-23:222:29
SACCHAROMYCES CEREVISIAE (BAKER'S YEAST).
P39524
HRIFA023434a
VOLTAGE-GATED POTASSIUM CHANNEL PROTEIN KV1.6 (RCK2) (KV2).
0.00018:157:30
RATTUS NORVEGICUS (RAT).
P17659
HRIFA023464a
GLYCINE-RICH CELL WALL STRUCTURAL PROTEIN 2 PRECURSOR.
1.0e-11:75:46
ORYZA SATIVA (RICE).
P29834
HRIFA023489a
HYPOTHETICAL 111.9 KD PROTEIN C22H10.03C IN CHROMOSOME I.
4.4e-09:230:23
SCHIZOSACCHAROMYCES POMBE (FISSION YEAST).
Q10297
HRIFA023634a
EBNA-1 NUCLEAR PROTEIN.
1.8e-08:113:45
EPSTEIN-BARR VIRUS (STRAIN B95-8) (HUMAN HERPESVIRUS 4).
P03211
HRIFA023767a
CYTOCHROME B5.
1.1e-12:92:38
SACCHAROMYCES CEREVISIAE (BAKER'S YEAST).
P40312
HRIFA023894a
PROLINE-RICH PROTEIN MP-2 PRECURSOR.
3.6e-05:80:40
MUS MUSCULUS (MOUSE).
P05142
HRIFA023923a
CYTOCHROME C OXIDASE POLYPEPTIDE I (EC 1.9.3.1).
4.2e-76:128:85
HOMO SAPIENS (HUMAN).
P00395
HRIFA024088a
NEURON-SPECIFIC X11 PROTEIN (FRAGMENT).
1.1e-05:118:32
MUS MUSCULUS (MOUSE).
P98084
HRIFA024132a
VOLTAGE-GATED POTASSIUM CHANNEL PROTEIN KV1.9.
6.5e-40:136:61
HOMO SAPIENS (HUMAN).
P51787
HRIFA024185a
ETS-DOMAIN TRANSCRIPTION FACTOR ERF.
0.55:128:29
HOMO SAPIENS (HUMAN).
P50548
HRIFA024197a
MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
7.5e-09:93:34
NEUROSPORA CRASSA.
P23231
HRIFA024218a
PROCOLLAGEN ALPHA 1(I) CHAIN PRECURSOR.
6.7e-06:180:36
HOMO SAPIENS (HUMAN).
P02452
HRIFA024255a
HYPOTHETICAL 116.3 KD PROTEIN C26F1.09 IN CHROMOSOME I.
4.8e-23:172:33
SCHIZOSACCHAROMYCES POMBE (FISSION YEAST).
Q10496
HRIFA024305a
CYCLIC-AMP-DEPENDENT TRANSCRIPTION FACTOR ATF-6 (FRAGMENT).
0.047:47:29
HOMO SAPIENS (HUMAN).
P18850
HRIFA024392a
TRANSMEMBRANE PROTEIN SEX PRECURSOR.
6.7e-24:119:43
HOMO SAPIENS (HUMAN).
P51805
HRIFA024423a
COP-COATED VESICLE MEMBRANE PROTEIN P24 PRECURSOR (FRAGMENT).
2.1e-18:109:41
CRICETULUS GRISEUS (CHINESE HAMSTER).
P49020
HRIFA024473a
COLLAGEN ALPHA 1(I) CHAIN (FRAGMENTS).
3.3e-05:106:41
BOS TAURUS (BOVINE).
P02453
HRIFA024482a
PISTIL-SPECIFIC EXTENSIN-LIKE PROTEIN PRECURSOR (PELP).
1.2e-07:99:31
NICOTIANA TABACUM (COMMON TOBACCO).
Q03211
HRIFA024504a
ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
2.6e-43:205:49
HOMO SAPIENS (HUMAN).
P37058
HRIFA024543a
GLYCOPROTEIN X PRECURSOR.
1.5e-06:257:28
EQUINE HERPESVIRUS TYPE 1 (STRAIN AB4P) (EHV-1).
P28968
HRIFA024718a
BETA-GALACTOSIDASE PRECURSOR (EC 3.2.1.23) (LACTASE) (ACID BETA-GALACTOSIDASE).
5.3e-45:168:52
MUS MUSCULUS (MOUSE).
P23780
HRIFA024767a
SODIUM CHANNEL PROTEIN (NA+ CHANNEL).
7.4e-30:221:31
ELECTROPHORUS ELECTRICUS (ELECTRIC EEL).
P02719
HRIFA024884a
HYPOTHETICAL 13.6 KD PROTEIN IN SPT4-ROM1 INTERGENIC REGION.
0.0089:23:65
SACCHAROMYCES CEREVISIAE (BAKER'S YEAST).
P53245
HRIFA024893a
REGULATORY PROTEIN E2.
0.0021:167:31
HUMAN PAPILLOMAVIRUS TYPE 8.
P06422
HRIFA024937a
GNS1 PROTEIN.
1.0e-15:173:33
SACCHAROMYCES CEREVISIAE (BAKER'S YEAST).
P25358
HRIFA024978a
MUCIN 2 PRECURSOR (INTESTINAL MUCIN 2).
0.00019:150:32
HOMO SAPIENS (HUMAN).
Q02817
HRIFA024994a
EXTENSIN PRECURSOR (CELL WALL HYDROXYPROLINE-RICH GLYCOPROTEIN).
5.3e-22:145:46
NICOTIANA TABACUM (COMMON TOBACCO).
P13983
HRIFA025033a
TRANS-ACTING TRANSCRIPTIONAL PROTEIN ICPO (VMW118 PROTEIN).
0.50:215:29
HERPES SIMPLEX VIRUS (TYPE 2 / STRAIN HG52).
P28284
HRIFA025046a
PROBABLE CALCIUM-TRANSPORTING ATPASE 6 (EC 3.6.1.38).
1.7e-41:104:48
SACCHAROMYCES CEREVISIAE (BAKER'S YEAST).
P39986
HRIFA025250a
"PROTEIN KINASE C, BRAIN ISOZYME (EC 2.7.1.-) (PKC) (DPKC53E(BR))."
7.4e-17:126:34
DROSOPHILA MELANOGASTER (FRUIT FLY).
P05130
HRIFA025261a
MYOSIN I ALPHA (MMI-ALPHA).
2.3e-64:141:84
MUS MUSCULUS (MOUSE).
P46735
HRIFA025290a
EBNA-1 NUCLEAR PROTEIN.
0.016:79:40
EPSTEIN-BARR VIRUS (STRAIN B95-8) (HUMAN HERPESVIRUS 4).
P03211
HRIFA025327a
PROLINE-RICH PROTEIN MP-2 PRECURSOR.
2.3e-06:104:37
MUS MUSCULUS (MOUSE).
P05142
HRIFA025353a
GLYCINE-RICH CELL WALL STRUCTURAL PROTEIN 2 PRECURSOR.
1.0e-11:75:46
ORYZA SATIVA (RICE).
P29834
HRIFA025479a
PROTEASE DEGS PRECURSOR (EC 3.4.21.-).
3.0e-05:112:33
ESCHERICHIA COLI.
P31137
HRIFA025488a
PROCOLLAGEN ALPHA 1(III) CHAIN PRECURSOR (FRAGMENTS).
9.5e-05:104:40
MUS MUSCULUS (MOUSE).
P08121
HRIFA025492a
SERINE/THREONINE-SPECIFIC PROTEIN KINASE MINIBRAIN HOMOLOG (EC 2.7.1.-) (HP86) (DYRK).
1.8e-53:159:69
HOMO SAPIENS (HUMAN).
Q13627
HRIFA025636a
MITOCHONDRIAL RESPIRATORY CHAIN COMPLEXES ASSEMBLY PROTEIN RCA1 (EC 3.4.24.-) (TAT-BINDING HOMOLOG 12).
4.7e-32:81:66
SACCHAROMYCES CEREVISIAE (BAKER'S YEAST).
P40341
HRIFA025695a
PEREGRIN (BR140 PROTEIN).
3.1e-40:227:43
HOMO SAPIENS (HUMAN).
P55201
HRIFA025703a
CELL SURFACE ANTIGEN 114/A10 PRECURSOR.
1.8e-08:71:42
MUS MUSCULUS (MOUSE).
P19467
HRIFA025706a
GLYCOSYLTRANSFERASE ALG2 (EC 2.4.1.-).
1.2e-28:111:44
SACCHAROMYCES CEREVISIAE (BAKER'S YEAST).
P43636
HRIFA025766a
CYTOCHROME B5.
4.2e-13:133:33
ORYCTOLAGUS CUNICULUS (RABBIT).
P00169
HRIFA025771a
HYPOTHETICAL 22.2 KD PROTEIN IN NSR1-TIF4631 INTERGENIC REGION.
6.7e-10:129:31
SACCHAROMYCES CEREVISIAE (BAKER'S YEAST).
P53288
HRIFA025778a
A-AGGLUTININ ATTACHMENT SUBUNIT PRECURSOR.
1.5e-05:212:30
SACCHAROMYCES CEREVISIAE (BAKER'S YEAST).
P32323
HRIFA025800a
HYPOTHETICAL 37.4 KD PROTEIN IN IRR1-TIM44 INTERGENIC REGION.
3.7e-18:165:33
SACCHAROMYCES CEREVISIAE (BAKER'S YEAST).
P40544
HRIFA025904a
COMPLEMENT RECEPTOR TYPE 1 PRECURSOR (C3B/C4B RECEPTOR) (CD35 ANTIGEN).
2.6e-05:211:28
HOMO SAPIENS (HUMAN).
P17927
HRIFA025907a
INTERFERON GAMMA UP-REGULATED I-5111 PROTEIN PRECURSOR (IGUP I-5111).
2.1e-38:176:38
HOMO SAPIENS (HUMAN).
Q06323
HRIFA025913a
DOLICHYL-PHOSPHATE-MANNOSE--PROTEIN MANNOSYLTRANSFERASE 4 (EC
2.4.1.109).
2.5e-32:185:37
SACCHAROMYCES CEREVISIAE (BAKER'S YEAST).
P46971
HRIFA025936a
TRANSCRIPTIONAL ACTIVATOR FE65.
3.4e-09:43:46
RATTUS NORVEGICUS (RAT).
P46933
HRIFA025966a
SYNAPTOTAGMIN III.
4.5e-05:93:33
RATTUS NORVEGICUS (RAT).
P40748
HRIFA025978a
"GLUTENIN, HIGH MOLECULAR WEIGHT SUBUNIT DX5 PRECURSOR."
3.5e-06:224:28
TRITICUM AESTIVUM (WHEAT).
P10388
HRIFA026089a
BUTYROPHILIN PRECURSOR (BT).
1.1e-12:146:29
BOS TAURUS (BOVINE).
P18892
HRIFA026121a
FAS ANTIGEN LIGAND (APOPTOSIS ANTIGEN LIGAND) (APTL).
9.7e-06:72:43
HOMO SAPIENS (HUMAN).
P48023
HRIFA026242a
HYPOTHETICAL 73.0 KD PROTEIN IN CLA4-MID1 INTERGENIC REGION.
7.4e-09:188:26
SACCHAROMYCES CEREVISIAE (BAKER'S YEAST).
P48566
HRIFA026265a
DNA BINDING PROTEIN S1FA.
0.67:43:37
ARABIDOPSIS THALIANA (MOUSE-EAR CRESS).
P42551
HRIFA026303a
SALIVARY PROLINE-RICH PROTEIN PO PRECURSOR (ALLELE S).
0.014:88:32
HOMO SAPIENS (HUMAN).
P10163
HRIFA026316a
EBNA-2 NUCLEAR PROTEIN.
1.5e-07:82:35
EPSTEIN-BARR VIRUS (STRAIN B95-8) (HUMAN HERPESVIRUS 4).
P12978
HRIFA026351a
FLI-1 ONCOGENE (ERGB TRANSCRIPTION FACTOR).
0.019:89:31
HOMO SAPIENS (HUMAN).
Q01543
HRIFA026364a
PROBABLE G PROTEIN-COUPLED RECEPTOR EDG-1.
8.3e-40:167:49
RATTUS NORVEGICUS (RAT).
P48303
HRIFA026382a
T-CELL RECEPTOR BETA CHAIN PRECURSOR (ANA 11).
6.2e-10:135:38
ORYCTOLAGUS CUNICULUS (RABBIT).
P06333
HRIFA026465a
COLLAGEN ALPHA 1(IX) CHAIN PRECURSOR (FRAGMENTS).
8.6e-07:158:35
GALLUS GALLUS (CHICKEN).
P12106
HRIFA026496a
ZINC FINGER PROTEIN 140.
5.9e-24:122:52
HOMO SAPIENS (HUMAN).
P52738
HRIFA026519a
PROLINE-RICH PROTEIN MP-2 PRECURSOR.
1.3e-08:130:36
MUS MUSCULUS (MOUSE).
P05142
HRIFA026564a
GLYCOPROTEIN X PRECURSOR.
1.8e-10:225:25
EQUINE HERPESVIRUS TYPE 1 (STRAIN AB4P) (EHV-1).
P28968
HRIFA026576a
"ADP,ATP CARRIER PROTEIN, HEART/SKELETAL MUSCLE ISOFORM T1 (ADP/ATP TRANSLOCASE 1) (ADENINE NUCLEOTIDE TRANSLOCATOR 1) (ANT 1)."
1.7e-09:116:34
HOMO SAPIENS (HUMAN).
P12235
HRIFA026615a
REGULATORY PROTEIN E2.
0.0024:132:31
HUMAN PAPILLOMAVIRUS TYPE 9.
P36780
HRIFA026618a
PROTEIN Q300.
1.2e-05:27:66
MUS MUSCULUS (MOUSE).
Q02722
HRIFA026659a
SERINE/THREONINE-PROTEIN KINASE SGK (EC 2.7.1.-) (SERUM/GLUCOCORTICOID-REGULATED KINASE).
2.0e-10:81:45
RATTUS NORVEGICUS (RAT).
Q06226
HRIFA026764a
MRC OX-45 SURFACE ANTIGEN PRECURSOR (BCM1 SURFACE ANTIGEN) (BLAST-1) (CD48).
3.4e-05:162:25
RATTUS NORVEGICUS (RAT).
P10252
HRIFA026789a
PUTATIVE GENERAL NEGATIVE REGULATOR OF TRANSCRIPTION C16C9.04C.
8.1e-22:175:38
SCHIZOSACCHAROMYCES POMBE (FISSION YEAST).
Q09818
HRIFA026813a
"PROTEIN KINASE C, MU TYPE (EC 2.7.1.-) (NPKC-MU)."
7.1e-89:256:67
HOMO SAPIENS (HUMAN).
Q15139
HRIFA026860a
MONOCARBOXYLATE TRANSPORTER 2 (MCT 2).
2.6e-19:103:43
MESOCRICETUS AURATUS (GOLDEN HAMSTER).
P53988
HRIFA026923a
CCAAT DISPLACEMENT PROTEIN (HOMEOBOX PROTEIN CLOX) (CLOX-1) (FRAGMENT).
0.18:119:36
CANIS FAMILIARIS (DOG).
P39881
HRIFA027012a
"MANNOSYL-OLIGOSACCHARIDE ALPHA-1,2-MANNOSIDASE (EC 3.2.1.113) (MAN(9)-ALPHA-MANNOSIDASE) (ALPHA-MANNOSIDASE 1A)."
1.8e-44:234:41
MUS MUSCULUS (MOUSE).
P45700
HRIFA027045a
HYPOTHETICAL PROTEIN HI0519.
2.7e-27:181:38
HAEMOPHILUS INFLUENZAE.
P44742
HRIFA027125a
ZINC FINGER PROTEIN 133.
3.9e-33:70:61
HOMO SAPIENS (HUMAN).
P52736
HRIFA027173a
TRANS-ACTING TRANSCRIPTIONAL PROTEIN ICP0 (VMW118 PROTEIN).
0.15:137:27
HERPES SIMPLEX VIRUS (TYPE 2 / STRAIN HG52).
P28284
HRIFA027179a
EXCISION REPAIR PROTEIN ERCC-6 (COCKAYNE SYNDROME PROTEIN CSB).
3.6e-30:90:77
HOMO SAPIENS (HUMAN).
Q03468
HRIFA027187a
B-CELL GROWTH FACTOR PRECURSOR (BCGF-12 KD).
4.7e-11:44:61
HOMO SAPIENS (HUMAN).
P20931
HRIFA027327a
COLLAGEN ALPHA 1(X) CHAIN PRECURSOR.
3.8e-07:184:35
HOMO SAPIENS (HUMAN).
Q03692
HRIFA027329a
SALIVARY GLUE PROTEIN SGS-3 PRECURSOR.
9.1e-08:195:29
DROSOPHILA ERECTA (FRUIT FLY).
P13730
HRIFA027355a
B-CELL GROWTH FACTOR PRECURSOR (BCGF-12 KD).
1.9e-06:33:72
HOMO SAPIENS (HUMAN).
P20931
HRIFA027485a
COLLAGEN ALPHA 1(XI) CHAIN PRECURSOR.
0.00099:174:36
HOMO SAPIENS (HUMAN).
P12107
HRIFA027536a
VITELLINE MEMBRANE PROTEIN VM26AB PRECURSOR (PROTEIN TU-4) (PROTEIN SV23).
0.0042:104:35
DROSOPHILA MELANOGASTER (FRUIT FLY).
P13238
HRIFA027549a
D(4) DOPAMINE RECEPTOR (D(2C) DOPAMINE RECEPTOR).
0.00023:101:44
HOMO SAPIENS (HUMAN).
P21917
HRIFA027622a
GUANOSINE-DIPHOSPHATASE (EC 3.6.1.42) (GDPASE).
2.2e-23:146:45
SACCHAROMYCES CEREVISIAE (BAKER'S YEAST).
P32621
HRIFA027625a
CALCIUM-TRANSPORTING ATPASE 1 (EC 3.6.1.38) (GOLGI CA2+-ATPASE).
1.1e-57:220:54
SACCHAROMYCES CEREVISIAE (BAKER'S YEAST).
P13586
HRIFA027644a
COLLAGEN ALPHA 1(I) CHAIN (FRAGMENTS).
7.5e-05:72:40
RATTUS NORVEGICUS (RAT).
P02454
HRIFA027656a
NON-RECEPTOR TYROSINE KINASE SPORE LYSIS A (EC 2.7.1.112) (TYROSINE-PROTEIN KINASE 1).
1.6e-13:149:34
DICTYOSTELIUM DISCOIDEUM (SLIME MOLD).
P18160
HRIFA027673a
CONNECTIVE TISSUE GROWTH FACTOR PRECURSOR.
6.4e-06:47:57
HOMO SAPIENS (HUMAN).
P29279
HRIFA027681a
SPORULATION-SPECIFIC PROTEIN 1 (EC 2.7.1.-).
1.1e-13:158:31
SACCHAROMYCES CEREVISIAE (BAKER'S YEAST).
P08458
HRIFA027714a
HYPOTHETICAL 146.8 KD PROTEIN C34E10.5 IN CHROMOSOME III.
7.2e-06:146:30
CAENORHABDITIS ELEGANS.
P46580
HRIFA027722a
SIGNAL RECOGNITION PARTICLE 68 KD PROTEIN (SRP68).
2.7e-105:242:85
CANIS FAMILIARIS (DOG).
Q00004
HRIFA027860a
SKIN SECRETORY PROTEIN XP2 PRECURSOR (APEG PROTEIN).
8.3e-08:168:32
XENOPUS LAEVIS (AFRICAN CLAWED FROG).
P17437
HRIFA027867a
STANNIOCALCIN PRECURSOR (STC) (CORPUSCLES OF STANNIUS PROTEIN) (CS) (HYPOCALCIN) (TELEOCALCIN).
1.0:100:27
ANGUILLA AUSTRALIS (AUSTRALIAN EEL).
P18301
HRIFA027940a
INHIBIN BETA C CHAIN PRECURSOR (ACTIVIN BETA-C CHAIN).
8.7e-15:149:38
HOMO SAPIENS (HUMAN).
P55103
HRIFA028061 a
PUTATIVE SERINE/THREONINE-PROTEIN KINASE PKWA (EC 2.7.1.-).
9.7e-07:157:26
THERMOMONOSPORA CURVATA.
P49695
HRIFA028157a
HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
2.8e-71:201:68
HOMO SAPIENS (HUMAN).
P30825
HRIFA028187a
EBNA-1 NUCLEAR PROTEIN.
1.5e-09:131:38
EPSTEIN-BARR VIRUS (STRAIN B95-8) (HUMAN HERPESVIRUS 4).
P03211
HRIFA028262a
CELLULAR NUCLEIC ACID BINDING PROTEIN (CNBP).
7.2e-09:99:33
MUS MUSCULUS (MOUSE).
P53996
HRIFA028371 a
PLASMINOGEN (EC 3.4.21.7) (FRAGMENT).
1.0e-08:103:33
RATTUS NORVEGICUS (RAT).
Q01177
HRIFA028402a
PUTATIVE SERINE/THREONINE-PROTEIN KINASE PKWA (EC 2.7.1.-).
3.2e-33:204:39
THERMOMONOSPORA CURVATA.
P49695
HRIFA028440a
COLLAGEN ALPHA 4(IV) CHAIN PRECURSOR.
1.9e-07:192:36
HOMO SAPIENS (HUMAN).
P53420
HRIFA028468a
CALCIUM/CALMODULIN-DEPENDENT PROTEIN KINASE TYPE IV CATALYTIC CHAIN (EC 2.7.1.123) (CAM KINASE-GR) (CAMK IV) [CONTAINS: CALSPERMIN].
5.8e-32:178:44
RATTUS NORVEGICUS (RAT).
P13234
HRIFA028501a
VOLTAGE-GATED POTASSIUM CHANNEL PROTEIN KV1.6 (RCK2) (KV2).
6.3e-05:161:31
RATTUS NORVEGICUS (RAT).
P17659
HRIFA028511a
ANKYRIN HOMOLOG PRECURSOR.
3.0e-19:176:34
CHROMATIUM VINOSUM.
Q06527
HRIFA028576a
ACROSIN PRECURSOR (EC 3.4.21.10).
4.8e-08:78:46
ORYCTOLAGUS CUNICULUS (RABBIT).
P48038
HRIFA028614a
HISTIDINE-RICH GLYCOPROTEIN PRECURSOR.
1.0e-08:82:39
PLASMODIUM LOPHURAE.
P04929
HRIFA028651a
BAND 3 ANION TRANSPORT PROTEIN.
1.3e-18:156:32
GALLUS GALLUS (CHICKEN).
P15575
HRIFA028790a
PROBABLE CALCIUM-TRANSPORTING ATPASE 6 (EC 3.6.1.38).
5.0e-18:212:29
SACCHAROMYCES CEREVISIAE (BAKER'S YEAST).
P39986
HRIFA028804a
CCAAT-BINDING FACTOR (CBF).
0.98:232:23
MUS MUSCULUS (MOUSE).
P53569
HRIFA028867a
REGULATORY PROTEIN E2.
0.0057:124:31
HUMAN PAPILLOMAVIRUS TYPE 25.
P36787
HRIFA028911a
HYPOTHETICAL 118.4 KD PROTEIN IN BAT2-DAL5 INTERGENIC REGION PRECURSOR.
1.2e-09:206:33
SACCHAROMYCES CEREVISIAE (BAKER'S YEAST).
P47179
HRIFA028983a
HOMEOTIC GENE REGULATOR (BRAHMA PROTEIN).
0.0051:115:33
DROSOPHILA MELANOGASTER (FRUIT FLY).
P25439
HRIFA029002a
FIBRINOGEN BETA CHAIN.
3.2e-25:121:45
BOS TAURUS (BOVINE).
P02676
HRIFA029050a
RETINAL-CADHERIN PRECURSOR (R-CADHERIN) (R-CAD).
1.2e-10:134:32
GALLUS GALLUS (CHICKEN).
P24503
HRIFA029208a
RENAL TRANSCRIPTION FACTOR KID-1 (TRANSCRIPTION FACTOR 17).
1.4e-14:64:59
RATTUS NORVEGICUS (RAT).
Q02975
HRIFA029209a
"ALPHA-MANNOSIDASE II (EC 3.2.1.114) (MANNOSYL-OLIGOSACCHARIDE 1,3-1,6-ALPHA-MANNOSIDASE) (MAN II) (GOLGI ALPHA-MANNOSIDASE II)."
2.3e-12:114:37
MUS MUSCULUS (MOUSE).
P27046
HRIFA029256a
GAP JUNCTION BETA-2 PROTEIN (CONNEXIN 26) (CX26).
1.8e-35:89:75
HOMO SAPIENS (HUMAN).
P29033
HRIFA029263a
SARCALUMENIN PRECURSOR.
2.1e-16:161:31
ORYCTOLAGUS CUNICULUS (RABBIT).
P13666
HRIFA029278a
"SALIVARY PROLINE-RICH PROTEIN PRECURSOR (CLONES CP3, CP4 AND CP5) [CONTAINS: BASIC PEPTIDE IB-6" PEPTIDE P-H].
3.5e-10:204:32
HOMO SAPIENS (HUMAN).
P04280
HRIFA029285a
GLYCOPROTEIN 25L PRECURSOR (GP25L).
4.9e-58:197:55
CANIS FAMILIARIS (DOG).
P27869
HRIFA029317a
HIGH AFFINITY SULPHATE TRANSPORTER 2.
2.3e-25:83:50
STYLOSANTHES HAMATA.
P53392
HRIFA029327a
MITOCHONDRIAL 2-OXOGLUTARATE/MALATE CARRIER PROTEIN (OGCP).
9.1e-34:227:37
BOS TAURUS (BOVINE).
P22292
HRIFA029349a
CUTICLE COLLAGEN 12 PRECURSOR.
5.1e-09:190:33
CAENORHABDITIS ELEGANS.
P20630
HRIFA029393a
PROBABLE G PROTEIN-COUPLED RECEPTOR APJ.
9.7e-69:165:84
HOMO SAPIENS (HUMAN).
P35414
HRIFA029398a
TRANS-ACTING TRANSCRIPTIONAL PROTEIN ICP0 (P135 PROTEIN) (IER 2.9/ER2.6).
0.011:170:34
BOVINE HERPESVIRUS TYPE 1 (STRAIN JURA).
P29128
HRIFA029425a
ALPHA CRYSTALLIN B CHAIN (ALPHA(B)-CRYSTALLIN).
2.0e-08:99:32
BOS TAURUS (BOVINE).
P02510
HRIFA029434a
"SALIVARY PROLINE-RICH PROTEIN PRECURSOR (CLONES CP3, CP4 AND CP5) [CONTAINS: BASIC PEPTIDE IB-6" PEPTIDE P-H].
2.6e-05:232:32
HOMO SAPIENS (HUMAN).
P04280
HRIFA029440a
SKIN SECRETORY PROTEIN XP2 PRECURSOR (APEG PROTEIN).
0.00046:131:33
XENOPUS LAEVIS (AFRICAN CLAWED FROG).
P17437
HRIFA029460a
SYNAPTOTAGMIN III.
1.5e-08:102:35
RATTUS NORVEGICUS (RAT).
P40748
HRIFA029467a
GLYCOPROTEIN X PRECURSOR.
5.2e-07:182:31
EQUINE HERPESVIRUS TYPE 1 (STRAIN AB4P) (EHV-1).
P28968
HRIFA029508a
PROPERDIN PRECURSOR.
1.9e-06:218:32
HOMO SAPIENS (HUMAN).
P27918
HRIFA029511a
POTASSIUM CHANNEL PROTEIN EAG.
2.3e-66:139:61
DROSOPHILA MELANOGASTER (FRUIT FLY).
Q02280
HRIFA029602a
SEX-DETERMINING REGION Y PROTEIN (TESTIS-DETERMINING FACTOR).
1.0:37:37
SUS SCROFA (PIG).
P36393
HRIFA029649a
TRANS-ACTING TRANSCRIPTIONAL PROTEIN ICPO (VMW118 PROTEIN).
0.30:99:34
HERPES SIMPLEX VIRUS (TYPE 2 / STRAIN HG52).
P28284
HRIFA029715a
GROWTH ARREST AND DNA-DAMAGE-INDUCIBLE PROTEIN GADD153 (DNA-DAMAGE INDUCIBLE TRANSCRIPT 3) (DDIT3) (C/EBP-HOMOLOGOUS PROTEIN) (CHOP).
0.54:95:30
HOMO SAPIENS (HUMAN).
P35638
HRIFA029730a
HISTIDINE-RICH GLYCOPROTEIN PRECURSOR.
3.8e-05:131:29
PLASMODIUM LOPHURAE.
P04929
HRIFA029792a
PUTATIVE SERINE/THREONINE-PROTEIN KINASE PKWA (EC 2.7.1.-).
9.0e-09:178:30
THERMOMONOSPORA CURVATA.
P49695
HRIFA029802a
TRAM PROTEIN (TRANSLOCATING CHAIN-ASSOCIATING MEMBRANE PROTEIN).
7.2e-73:204:69
CANIS FAMILIARIS (DOG).
Q01685
HRIFA029866a
PROTEIN KINASE BYR2 (EC 2.7.1.-) (PROTEIN KINASE STE8) (MAPK KINASE KINASE) (MAPKKK).
1.2e-27:144:45
SCHIZOSACCHAROMYCES POMBE (FISSION YEAST).
P28829
HRIFA029932a
F-SPONDIN PRECURSOR.
9.1e-24:191:37
XENOPUS LAEVIS (AFRICAN CLAWED FROG).
P35447
HRIFA030025a
ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
1.0e-11:138:31
SACCHAROMYCES CEREVISIAE (BAKER'S YEAST).
P32802
HRIFA030045a
SARCALUMENIN PRECURSOR.
2.4e-20:151:32
ORYCTOLAGUS CUNICULUS (RABBIT).
P13666
HRIFA030103a
HYPOTHETICAL 57.5 KD PROTEIN IN VMA7-RPS25A INTERGENIC REGION.
2.1e-05:215:29
SACCHAROMYCES CEREVISIAE (BAKER'S YEAST).
P53214
HRIFA030106a
SCO-SPONDIN (FRAGMENT).
0.53:60:36
BOS TAURUS (BOVINE).
P98167
HRIFA030147a
PUTATIVE MITOCHONDRIAL CARRIER YGR096W.
1.8e-10:93:34
SACCHAROMYCES CEREVISIAE (BAKER'S YEAST).
P53257
HRIFA030250a
ENAMELIN (TUFTELIN).
3.7e-108:250:86
BOS TAURUS (BOVINE).
P27628
HRIFA030264a
SODIUM/GLUCOSE COTRANSPORTER 1 (NA(+)/GLUCOSE COTRANSPORTER 1) (HIGH AFFINITY SODIUM-GLUCOSE COTRANSPORTER).
3.3e-09:119:27
ORYCTOLAGUS CUNICULUS (RABBIT).
P11170
HRIFA030342a
ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
1.5e-42:203:49
HOMO SAPIENS (HUMAN).
P37058
HRIFA030370a
HYPOTHETICAL 56.2 KD PROTEIN CY31.25C.
8.0e-12:88:48
MYCOBACTERIUM TUBERCULOSIS.
Q10555
HRIFA030371a
"PROTEIN KINASE C, MU TYPE (EC 2.7.1.-) (NPKC-MU)."
1.6e-68:228:59
HOMO SAPIENS (HUMAN).
Q15139
HRIFA030381a
COLLAGEN 1(X) CHAIN PRECURSOR.
3.0e-05:204:30
GALLUS GALLUS (CHICKEN).
P08125
HRIFA030385a
COLLAGEN ALPHA 1(X) CHAIN PRECURSOR.
0.029:162:31
HOMO SAPIENS (HUMAN).
Q03692
HRIFA030411a
SERINE PALMITOYLTRANSFERASE 2 (EC 2.3.1.50) (LONG CHAIN BASE BIOSYNTHESIS PROTEIN 2) (SPT 2).
1.2e-27:115:53
SCHIZOSACCHAROMYCES POMBE (FISSION YEAST).
Q09925
HRIFA030448a
PUTATIVE SERINE/THREONINE-PROTEIN KINASE P78 (EC 2.7.1.-).
2.5e-92:225:77
HOMO SAPIENS (HUMAN).
P27448
HRIFA030456a
PROLINE-RICH PROTEIN MP-2 PRECURSOR.
9.3e-08:127:35
MUS MUSCULUS (MOUSE).
P05142
HRIFA030461a
CUTICLE COLLAGEN 12 PRECURSOR.
0.046:140:31
CAENORHABDITIS ELEGANS.
P20630
HRIFA030472a
NUC-1 NEGATIVE REGULATORY PROTEIN PREG.
0.0030:98:31
NEUROSPORA CRASSA.
Q06712
HRIFA030509a
"INTERFERON-INDUCED, DOUBLE-STRANDED RNA-ACTIVATED PROTEIN KINASE (EC 2.7.1.-) (INTERFERON-INDUCIBLE RNA-DEPENDENT PROTEIN KINASE) (P68 KINASE) (P1/EIF-2A PROTEIN KINASE)."
2.5e-09:65:43
HOMO SAPIENS (HUMAN).
P19525
HRIFA030511a
T-LYMPHOCYTE MATURATION-ASSOCIATED PROTEIN.
0.00010:99:33
HOMO SAPIENS (HUMAN).
P21145
HRIFA030545a
PROBABLE SERINE/THREONINE-PROTEIN KINASE YNL020C (EC 2.7.1.-).
7.6e-21:165:35
SACCHAROMYCES CEREVISIAE (BAKER'S YEAST).
P53974
HRIFA030566a
"GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE)."
2.7e-07:221:30
SACCHAROMYCES CEREVISIAE (BAKER'S YEAST).
P08640
HRIFA030599a
GLYCOPROTEIN X PRECURSOR.
2.8e-05:236:27
EQUINE HERPESVIRUS TYPE 1 (STRAIN AB4P) (EHV-1).
P28968
HRIFA030629a
PROTEIN DISULFIDE ISOMERASE PRECURSOR (PDI) (EC 5.3.4.1) (PROLYL 4-HYDROXYLASE BETA SUBUNIT) (CELLULAR THYROID HORMONE BINDING PROTEIN) (P55).
3.5e-16:115:38
BOS TAURUS (BOVINE).
P05307
HRIFA030642a
SULFATED SURFACE GLYCOPROTEIN 185 (SSG 185).
2.5e-12:93:47
VOLVOX CARTERI.
P21997
HRIFA030662a
NADH-UBIQUINONE OXIDOREDUCTASE CHAIN 1 (EC 1.6.5.3).
9.1e-120:279:83
HOMO SAPIENS (HUMAN).
P03886
HRIFA030839a
HYPOTHETICAL GENE 51 MEMBRANE PROTEIN.
1.0:66:27
ICTALURID HERPESVIRUS 1 (CHANNEL CATFISH VIRUS) (CCV).
Q00135
HRIFA031091a
PROTEIN Q300.
0.0042:27:62
MUS MUSCULUS (MOUSE).
Q02722
HRIFA031126a
P2Y PURINOCEPTOR 5 (P2Y5) (PURINERGIC RECEPTOR 5) (RB INTRON ENCODED G-PROTEIN COUPLED RECEPTOR).
1.3e-06:70:34
HOMO SAPIENS (HUMAN).
P43657
HRIFA031249a
ACIDIC PROLINE-RICH PROTEIN PRECURSOR (CLONE PRP33).
5.9e-05:166:31
RATTUS NORVEGICUS (RAT).
P04474
HRIFA031336a
CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
6.6e-15:97:38
PETROMYZON MARINUS (SEA LAMPREY).
P25210
HRIFA031395a
COLD SHOCK PROTEIN CSPB (FRAGMENT).
0.95:32:40
BACILLUS GLOBISPORUS.
P41018
HRIFA031397a
REGULATORY PROTEIN E2.
0.0077:145:35
HUMAN PAPILLOMAVIRUS TYPE 47.
P22420
HRIFA031438a
GLUCOSE REPRESSION MEDIATOR PROTEIN.
1.3e-06:176:26
SACCHAROMYCES CEREVISIAE (BAKER'S YEAST).
P14922
HRIFA031869a
TRANSCRIPTION FACTOR HES-1 (HAIRY AND ENHANCER OF SPLIT 1) (HAIRY-LIKE) (RHL).
1.7e-18:163:41
RATTUS NORVEGICUS (RAT).
Q04666
HRIFA031935a
EXTENSIN PRECURSOR (PROLINE-RICH GLYCOPROTEIN).
1.8e-06:192:32
ZEA MAYS (MAIZE).
P14918
HRIFA031986a
SERINE/THREONINE-PROTEIN KINASE PAK-ALPHA (EC 2.7.1.-) (P68-PAK) (P21-ACTIVATED KINASE) (ALPHA-PAK) (PROTEIN KINASE MUK2).
2.4e-49:222:47
RATTUS NORVEGICUS (RAT).
P35465
HRIFA032009a
PROBABLE G PROTEIN-COUPLED RECEPTOR FROM T-CELLS PRECURSOR (GLUCOCORTICOID-INDUCED RECEPTOR).
1.0e-17:118:36
MUS MUSCULUS (MOUSE).
P30731
HRIFA032011a
MUSCARINIC ACETYLCHOLINE RECEPTOR M4.
7.8e-35:184:32
HOMO SAPIENS (HUMAN).
P08173
HRIFA032070a
MITOCHONDRIAL RNA SPLICING PROTEIN MSR4.
2.1e-18:107:44
SACCHAROMYCES CEREVISIAE (BAKER'S YEAST).
P23500
HRIFA032073a
SECRETOGRANIN III PRECURSOR (SGIII).
9.7e-69:182:76
MUS MUSCULUS (MOUSE).
P47867
HRIFA032079a
HYPOTHETICAL 49.3 KD PROTEIN C30D11.06C IN CHROMOSOME I.
3.5e-12:96:39
SCHIZOSACCHAROMYCES POMBE (FISSION YEAST).
Q09906
HRIFA032097a
GLYCOPROTEIN J.
0.023:61:32
HERPES SIMPLEX VIRUS (TYPE 1 / STRAIN 17).
P06480
HRIFA032161 a
CCAAT/ENHANCER BINDING PROTEIN DELTA (C/EBP DELTA) (NUCLEAR FACTOR NF-IL6-BETA) (NF-IL6-BETA).
0.22:56:42
HOMO SAPIENS (HUMAN).
P49716
HRIFA032186a
D-BINDING PROTEIN (DBP) (ALBUMIN D BOX-BINDING PROTEIN) (TAXREB302).
0.86:50:38
HOMO SAPIENS (HUMAN).
Q10586
HRIFA032224a
HYPOTHETICAL 52.7 KD PROTEIN C38C10.2 IN CHROMOSOME III.
2.6e-43:196:45
CAENORHABDITIS ELEGANS.
Q03567
HRIFA032257a
GLUCOSE REPRESSION MEDIATOR PROTEIN.
4.7e-07:204:25
SACCHAROMYCES CEREVISIAE (BAKER'S YEAST).
P14922
HRIFA032274a
ZINC FINGER PROTEIN 38 (ZFP-38) (CTFIN51) (TRANSCRIPTION FACTOR RU49).
7.8e-60:163:74
MUS MUSCULUS (MOUSE).
Q07231
HRIFA032275a
CELL DIVISION CONTROL PROTEIN 28 (EC 2.7.1.-).
7.2e-41:179:38
SACCHAROMYCES CEREVISIAE (BAKER'S YEAST).
P00546
HRIFA032360a
HYPOTHETICAL 84.3 KD PROTEIN ZK945.10 IN CHROMOSOME II.
3.0e-05:198:28
CAENORHABDITIS ELEGANS.
Q09625
HRIFA0323 89a
EBNA-1 NUCLEAR PROTEIN.
1.3e-05:86:39
EPSTEIN-BARR VIRUS (STRAIN B95-8) (HUMAN HERPESVIRUS 4). P03211
HRIFA032433a
GONADOTROPIN-RELEASING HORMONE RECEPTOR (GNRH-R).
3.1e-14:54:53
RATTUS NORVEGICUS (RAT).
P30969
HRIFA032453a
BUTYROPHILIN PRECURSOR (BT).
5.9e-13:162:32
BOS TAURUS (BOVINE).
P18892
HRIFA032478a
GLYCOPROTEIN X PRECURSOR.
3.8e-06:253:28
EQUINE HERPESVIRUS TYPE 1 (STRAIN AB4P) (EHV-1).
P28968
HRIFA032506a
COLLAGEN ALPHA 3(VI) CHAIN PRECURSOR.
1.2e-06:226:34
HOMO SAPIENS (HUMAN).
P12111
HRIFA032511a
COLLAGEN ALPHA 1(XVI) CHAIN PRECURSOR.
8.7e-09:229:34
HOMO SAPIENS (HUMAN).
Q07092
HRIFA032530a
SKIN SECRETORY PROTEIN XP2 PRECURSOR (APEG PROTEIN).
9.0e-05:159:33
XENOPUS LAEVIS (AFRICAN CLAWED FROG).
P17437
HRIFA032587a
SYNAPTOTAGMIN (P65).
3.2e-08:72:52
APLYSIA CALIFORNICA (CALIFORNIA SEA HARE).
P41823
HRIFA032605a
ANTIGEN PEPTIDE TRANSPORTER 1 (APT1) (PEPTIDE TRANSPORTER TAP1) (PEPTIDE TRANSPORTER PSF1) (PEPTIDE SUPPLY FACTOR 1) (PSF-1) (PEPTIDE TRANSPORTER INVOLVED IN ANTIGEN PROCESSING 1).
8.4e-37:192:41
HOMO SAPIENS (HUMAN).
Q03518
HRIFA032642a
PROLINE-RICH PROTEIN MP-2 PRECURSOR.
5.0e-05:127:33
MUS MUSCULUS (MOUSE).
P05142
HRIFA032696a
COLLAGEN ALPHA 1(II) CHAIN (FRAGMENTS).
1.4e-13:200:38
BOS TAURUS (BOVINE).
P02459
HRIFA032730a
K-GLYPICAN PRECURSOR.
4.8e-67:180:68
MUS MUSCULUS (MOUSE).
P51655
HRIFA032820a
GLUTAMIC ACID-RICH PROTEIN PRECURSOR.
7.5e-05:192:23
PLASMODIUM FALCIPARUM (ISOLATE FC27 / PAPUA NEW GUINEA).
P13816

### Homology search result 2

Homology of representative sequences of the 5'-end cluster to the data in Swiss-Prot database Representative sequence of the 5'-end cluster exhibiting relatively high homology (304 cluster: "exhibiting relatively high homology" means that the P value is 10⁻¹⁰ or less)
HRIFA000327a, HRIFA000432a, HRIFA000553a, HRIFA000564a, HRIFA000631a, HRIFA000683a, HRIFA000776a, HRIFA000814a, HRIFA001132a, HRIFA001138a, HRIFA001337a, HRIFA001341a, HRIFA001489a, HRIFA001712a, HRIFA001720a, HRIFA001942a, HRIFA001975a, HRIFA001984a, HRIFA002384a, HRIFA002503a, HRIFA002743a, HRIFA002766a, HRIFA002805a, HRIFA002891a, HRIFA002919a, HRIFA002980a, HRIFA003063a, HRIFA003093a, HRIFA003635a, HRIFA004006a, HRIFA004034a, HRIFA004112a, HRIFA004426a, HRIFA004490a, HRIFA004523a, HRIFA004663a, HRIFA004696a, HRIFA004714a, HRIFA004745a, HRIFA004919a, HRIFA005184a, HRIFA005231a, HRIFA005240a, HRIFA005271a, HRIFA005372a, HRIFA005392a, HRIFA005409a, HRIFA005420a, HRIFA005438a, HRIFA005462a, HRIFA005644a, HRIFA005720a, HRIFA005732a, HRIFA005760a, HRIFA005781a, HRIFA006183a, HRIFA006494a, HRIFA006510a, HRIFA006566a, HRIFA006586a, HRIFA006596a, HRIFA006649a, HRIFA006667a, HRIFA006730a, HRIFA006926a, HRIFA007013a, HRIFA007219a, HRIFA007228a, HRIFA007243a, HRIFA007352a, HRIFA007424a, HRIFA007435a, HRIFA007463a, HRIFA007493a, HRIFA007571a, HRIFA007659a, HRIFA007722a, HRIFA007745a, HRIFA008000a, HRIFA008200a, HRIFA008284a, HRIFA008314a, HRIFA008362a, HRIFA008459a, HRIFA008483a, HRIFA008547a, HRIFA008611a, HRIFA008661a, HRIFA008717a, HRIFA008784a, HRIFA008981a, HRIFA009101a, HRIFA009171a, HRIFA009220a, HRIFA009451a, HRIFA009482a, HRIFA009783a, HRIFA009881a, HRIFA010085a, HRIFA010090a, HRIFA010130a, HRIFA010319a, HRIFA010394a, HRIFA010460a, HRIFA010790a, HRIFA010975a, HRIFA011016a, HRIFA011179a, HRIFA011197a, HRIFA011449a, HRIFA011659a, HRIFA011947a, HRIFA012278a, HRIFA012584a, HRIFA012625a, HRIFA012692a, HRIFA012795a, HRIFA012885a, HRIFA012914a, HRIFA012969a, HRIFA012990a, HRIFA013254a, HRIFA013265a, HRIFA013276a, HRIFA013376a, HRIFA013477a, HRIFA013586a, HRIFA013726a, HRIFA013744a, HRIFA013911a, HRIFA014006a, HRIFA014185a, HRIFA014336a, HRIFA014465a, HRIFA014500a, HRIFA014561a, HRIFA014568a, HRIFA014621a, HRIFA014688a, HRIFA014819a, HRIFA014951a, HRIFA014967a, HRIFA015063a, HRIFA015070a, HRIFA015246a, HRIFA015423a, HRIFA015453a, HRIFA015486a, HRIFA015506a, HRIFA015536a, HRIFA015547a, HRIFA015568a, HRIFA015756a, HRIFA015811a, HRIFA016070a, HRIFA016290a, HRIFA016430a, HRIFA016654a, HRIFA016758a, HRIFA017031a, HRIFA017257a, HRIFA017295a, HRIFA017312a, HRIFA017703a, HRIFA017855a, HRIFA018092a, HRIFA018131a, HRIFA018134a, HRIFA018580a, HRIFA018827a, HRIFA018904a, HRIFA018993a, HRIFA019105a, HRIFA019136a, HRIFA019175a, HRIFA019262a, HRIFA019466a, HRIFA019867a, HRIFA019869a, HRIFA020272a, HRIFA020335a, HRIFA020349a, HRIFA020862a, HRIFA021213a, HRIFA021398a, HRIFA021499a, HRIFA021637a, HRIFA021651a, HRIFA021754a, HRIFA021781a, HRIFA022065a, HRIFA022139a, HRIFA022166a, HRIFA022177a, HRIFA022182a, HRIFA022227a, HRIFA022249a, HRIFA022265a, HRIFA022328a, HRIFA022423a, HRIFA022528a, HRIFA022546a, HRIFA022564a, HRIFA022616a, HRIFA022671a, HRIFA022691a, HRIFA022707a, HRIFA022729a, HRIFA022737a, HRIFA022776a, HRIFA022875a, HRIFA022895a, HRIFA023007a, HRIFA023227a, HRIFA023257a, HRIFA023304a, HRIFA023464a, HRIFA023767a, HRIFA023923a, HRIFA024132a, HRIFA024255a, HRIFA024392a, HRIFA024423a, HRIFA024504a, HRIFA024718a, HRIFA024767a, HRIFA024937a, HRIFA024994a, HRIFA025046a, HRIFA025250a, HRIFA025261a, HRIFA025353a, HRIFA025492a, HRIFA025636a, HRIFA025695a, HRIFA025706a, HRIFA025766a, HRIFA025800a, HRIFA025907a, HRIFA025913a, HRIFA026089a, HRIFA026364a, HRIFA026496a, HRIFA026789a, HRIFA026813a, HRIFA026860a, HRIFA027012a, HRIFA027045a, HRIFA027125a, HRIFA027179a, HRIFA027187a, HRIFA027622a, HRIFA027625a, HRIFA027656a, HRIFA027681a, HRIFA027722a, HRIFA027940a, HRIFA028157a, HRIFA028402a, HRIFA028468a, HRIFA028511a, HRIFA028651a, HRIFA028790a, HRIFA029002a, HRIFA029208a, HRIFA029209a, HRIFA029256a, HRIFA029263a, HRIFA029285a, HRIFA029317a, HRIFA029327a, HRIFA029393a, HRIFA029511a, HRIFA029802a, HRIFA029866a, HRIFA029932a, HRIFA030025a, HRIFA030045a, HRIFA030250a, HRIFA030342a, HRIFA030370a, HRIFA030371a, HRIFA030411a, HRIFA030448a, HRIFA030545a, HRIFA030629a, HRIFA030642a, HRIFA030662a, HRIFA031336a, HRIFA031869a, HRIFA031986a, HRIFA032009a, HRIFA032011a, HRIFA032070a, HRIFA032073a, HRIFA032079a, HRIFA032224a, HRIFA032274a, HRIFA032275a, HRIFA032433a, HRIFA032453a, HRIFA032605a, HRIFA032696a, HRIFA032730a,

### Homology search result 3

Representative sequence of the 5'-end cluster exhibiting relatively low homology (221 cluster: "exhibiting relatively low homology" means that the P value is higher than 10⁻¹⁰ and 10⁻⁴ or less)
HRIFA000016a, HRIFA000071a, HRIFA000116a, HRIFA000123a, HRIFA000264a, HRIFA000415a, HRIFA000446a, HRIFA000695a, HRIFA000845a, HRIFA001971a, HRIFA002063a, HRIFA002102a, HRIFA002284a, HRIFA002309a, HRIFA002694a, HRIFA002762a, HRIFA002787a, HRIFA003055a, HRIFA003340a, HRIFA003402a, HRIFA003504a, HRIFA003892a, HRIFA003946a, HRIFA004162a, HRIFA004401a, HRIFA004780a, HRIFA005072a, HRIFA005102a, HRIFA005214a, HRIFA005255a, HRIFA005300a, HRIFA005369a, HRIFA005702a, HRIFA005728a, HRIFA005944a, HRIFA006298a, HRIFA006448a, HRIFA006572a, HRIFA006633a, HRIFA006642a, HRIFA007068a, HRIFA007244a, HRIFA007262a, HRIFA007512a, HRIFA007532a, HRIFA007565a, HRIFA007728a, HRIFA007909a, HRIFA008174a, HRIFA008426a, HRIFA008596a, HRIFA008790a, HRIFA008989a, HRIFA009578a, HRIFA009825a, HRIFA009852a, HRIFA009983a, HRIFA010005a, HRIFA010078a, HRIFA010152a, HRIFA010301a, HRIFA010361a, HRIFA010425a, HRIFA010466a, HRIFA010799a, HRIFA011580a, HRIFA011820a, HRIFA012167a, HRIFA012354a, HRIFA012427a, HRIFA012436a, HRIFA012515a, HRIFA012702a, HRIFA012737a, HRIFA013135a, HRIFA013235a, HRIFA013279a, HRIFA013589a, HRIFA013620a, HRIFA013919a, HRIFA013932a, HRIFA014056a, HRIFA014111a, HRIFA014133a, HRIFA014396a, HRIFA014397a, HRIFA014598a, HRIFA014702a, HRIFA014868a, HRIFA015219a, HRIFA015995a, HRIFA016214a, HRIFA016240a, HRIFA016255a, HRIFA016639a, HRIFA016669a, HRIFA016963a, HRIFA017457a, HRIFA017643a, HRIFA017670a, HRIFA017801a, HRIFA017836a, HRIFA017921a, HRIFA018238a, HRIFA018262a, HRIFA018287a, HRIFA018666a, HRIFA018688a, HRIFA018754a, HRIFA018794a, HRIFA018870a, HRIFA018931a, HRIFA019412a, HRIFA019490a, HRIFA019498a, HRIFA019532a, HRIFA019651a, HRIFA020144a, HRIFA020184a, HRIFA020453a, HRIFA020693a, HRIFA020707a, HRIFA020748a, HRIFA021061a, HRIFA021224a, HRIFA021494a, HRIFA021794a, HRIFA021855a, HRIFA021906a, HRIFA022156a, HRIFA022203a, HRIFA022234a, HRIFA022702a, HRIFA022728a, HRIFA022782a, HRIFA022865a, HRIFA022890a, HRIFA022985a, HRIFA023048a, HRIFA023069a, HRIFA023129a, HRIFA023154a, HRIFA023212a, HRIFA023489a, HRIFA023634a, HRIFA023894a, HRIFA024088a, HRIFA024197a, HRIFA024218a, HRIFA024473a, HRIFA024482a, HRIFA024543a, HRIFA025327a, HRIFA025479a, HRIFA025488a, HRIFA025703a, HRIFA025771a, HRIFA025778a, HRIFA025904a, HRIFA025966a, HRIFA025978a, HRIFA026121a, HRIFA026242a, HRIFA026316a, HRIFA026382a, HRIFA026465a, HRIFA026519a, HRIFA026564a, HRIFA026576a, HRIFA026618a, HRIFA026659a, HRIFA026764a, HRIFA027327a, HRIFA027329a, HRIFA027355a, HRIFA027644a, HRIFA027673a, HRIFA027714a, HRIFA027860a, HRIFA028061a, HRIFA028187a, HRIFA028262a, HRIFA028371a, HRIFA028440a, HRIFA028501a, HRIFA028576a, HRIFA028614a, HRIFA028911a, HRIFA029050a, HRIFA029278a, HRIFA029349a, HRIFA029425a, HRIFA029434a, HRIFA029460a, HRIFA029467a, HRIFA029508a, HRIFA029730a, HRIFA029792a, HRIFA030103a, HRIFA030147a, HRIFA030264a, HRIFA030381a, HRIFA030456a, HRIFA030509a, HRIFA030511a, HRIFA030566a, HRIFA030599a, HRIFA031126a, HRIFA031249a, HRIFA031438a, HRIFA031935a, HRIFA032257a, HRIFA032360a, HRIFA032389a, HRIFA032478a, HRIFA032506a, HRIFA032511a, HRIFA032530a, HRIFA032587a, HRIFA032642a, HRIFA032820a,

### Homology search result 4

Representative sequence of the 5'-end cluster exhibiting low homology (115 cluster: "exhibiting low homology" means that the P value is higher than 10⁻⁴ and 1 or less)
HRIFA001099a, HRIFA001200a, HRIFA001413a, HRIFA001439a, HRIFA001558a, HRIFA001866a, HRIFA001972a, HRIFA002689a, HRIFA003357a, HRIFA003592a, HRIFA003640a, HRIFA003883a, HRIFA005296a, HRIFA005500a, HRIFA005540a, HRIFA006250a, HRIFA006609a, HRIFA006798a, HRIFA007032a, HRIFA007152a, HRIFA007547a, HRIFA007829a, HRIFA007985a, HRIFA008212a, HRIFA008252a, HRIFA008976a, HRIFA009071a, HRIFA009123a, HRIFA009136a, HRIFA009339a, HRIFA009762a, HRIFA010176a, HRIFA010490a, HRIFA010736a, HRIFA010859a, HRIFA010891a, HRIFA010988a, HRIFA011105a, HRIFA011128a, HRIFA011484a, HRIFA011512a, HRIFA011926a, HRIFA012069a, HRIFA012151a, HRIFA013092a, HRIFA013103a, HRIFA013980a, HRIFA014024a, HRIFA014590a, HRIFA014620a, HRIFA015122a, HRIFA015351a, HRIFA015802a, HRIFA015902a, HRIFA015947a, HRIFA016599a, HRIFA017146a, HRIFA017190a, HRIFA017456a, HRIFA017791a, HRIFA017818a, HRIFA018447a, HRIFA019437a, HRIFA019958a, HRIFA020883a, HRIFA021007a, HRIFA021040a, HRIFA021445a, HRIFA021543a, HRIFA021620a, HRIFA021787a, HRIFA022055a, HRIFA022335a, HRIFA022348a, HRIFA022411a, HRIFA022462a, HRIFA022493a, HRIFA022714a, HRIFA023434a, HRIFA024185a, HRIFA024305a, HRIFA024884a, HRIFA024893a, HRIFA024978a, HRIFA025033a, HRIFA025290a, HRIFA026265a, HRIFA026303a, HRIFA026351a, HRIFA026615a, HRIFA026923a, HRIFA027173a, HRIFA027485a, HRIFA027536a, HRIFA027549a, HRIFA027867a, HRIFA028804a, HRIFA028867a, HRIFA028983a, HRIFA029398a, HRIFA029440a, HRIFA029602a, HRIFA029649a, HRIFA029715a, HRIFA030106a, HRIFA030385a, HRIFA030461a, HRIFA030472a, HRIFA030839a, HRIFA031091a, HRIFA031395a, HRIFA031397a, HRIFA032097a, HRIFA032161a, HRIFA032186a,

### Homology search result 5

The result of the homology search in the SwissProt using the clone sequences of the 5'-ends. Indicated are from the top,
the name of the clone sequence,
definition of the top hit data,
the P-value: the length of the sequence used for comparison (nucleotide):similarity (%),
the organism of which the top hit data is obtained,
the Accession No. of the top hit data.
Data were not shown for the clones in which the P-value was higher than 1.
F-BNGH41000020
NADH-UBIQUINONE OXIDOREDUCTASE CHAIN 1 (EC 1.6.5.3).
1.2e-119:279:83
HOMO SAPIENS (HUMAN).
P03886
F-BNGH41000087
PROPERDIN PRECURSOR.
2.5e-06:218:32
HOMO SAPIENS (HUMAN).
P27918
F-BNGH41000091
POTASSIUM CHANNEL PROTEIN EAG.
3.1e-66:139:61
DROSOPHILA MELANOGASTER (FRUIT FLY).
Q02280
F-HEMBA1000006
S-ANTIGEN PROTEIN PRECURSOR.
3.0e-05:164:31
PLASMODIUM FALCIPARUM (ISOLATE V1).
P09593
F-HEMBA1000121
HYPOTHETICAL 68.7 KD PROTEIN ZK757.1 IN CHROMOSOME III.
8.2e-06:83:27
CAENORHABDITIS ELEGANS.
P34679
F-HEMBA1000128
PATHOGENESIS-RELATED PROTEIN 1 PRECURSOR (PR-1).
8.2e-08:89:34
ARABIDOPSIS THALIANA (MOUSE-EAR CRESS).
P33154
F-HEMBA1000275
PROCOLLAGEN ALPHA 1(I) CHAIN PRECURSOR.
1.9e-06:231:34
GALLUS GALLUS (CHICKEN).
P02457
F-HEMBA1000300
!!!! ALU SUBFAMILY SX WARNING ENTRY !!!!
1. 4e-13:73:56
HOMO SAPIENS (HUMAN).
P39195
F-HEMBA1000349
ATP-BINDING CASSETTE TRANSPORTER 1.
2.6e-16:238:31
MUS MUSCULUS (MOUSE).
P41233
F-HEMBA1000443
PROLINE-RICH PROTEIN MP-2 PRECURSOR.
4.8e-06:120:35
MUS MUSCULUS (MOUSE).
P05142
F-HEMBA1000462
PUTATIVE GENERAL NEGATIVE REGULATOR OF TRANSCRIPTION C16C9.04C.
2.9e-21:86:52
SCHIZOSACCHAROMYCES POMBE (FISSION YEAST).
Q09818
F-HEMBA1000477
HYPOTHETICAL 64.8 KD PROTEIN IN GD11-COX15 INTERGENIC REGION.
3.3e-09:138:34
SACCHAROMYCES CEREVISIAE (BAKER'S YEAST).
P40085
F-HEMBA1000590
CARTILAGE MATRIX PROTEIN PRECURSOR (MATRILIN-1).
2.2e-27:117:48
GALLUS GALLUS (CHICKEN).
P05099
F-HEMBA1000634
TRANS-ACTING TRANSCRIPTIONAL PROTEIN ICPO (IMMEDIATE-EARLY PROTEIN IE110) (VMW110) (ALPHA-0 PROTEIN).
0.00027:85:43
HERPES SIMPLEX VIRUS (TYPE 1 / STRAIN 17).
P08393
F-HEMBA1000671
ZINC FINGER PROTEIN 140.
1.1e-44:155:47
HOMO SAPIENS (HUMAN).
P52738
F-HEMBA1000713
BLADDER CANCER 10 KD PROTEIN.
1.5e-42:81:97
HOMO SAPIENS (HUMAN).
060629
F-HEMBA1000732
FIBRILLIN 1 PRECURSOR.
6.3e-18:77:46
HOMO SAPIENS (HUMAN).
P35555
F-HEMBA1000745
SALIVARY PROLINE-RICH PROTEIN PRECURSOR (CLONES CP3, CP4 AND CP5) [CONTAINS:
BASIC PEPTIDE IB-6; PEPTIDE P-H].
5.2e-06:105:33
HOMO SAPIENS (HUMAN).
P04280
F-HEMBA1000835
FIBRILLIN 2 PRECURSOR.
2.1e-42:214:44
HOMO SAPIENS (HUMAN).
P35556
F-HEMBA1000875
ZINC FINGER PROTEIN 133.
5.8e-16:49:87
HOMO SAPIENS (HUMAN).
P52736
F-HEMBA1000907
PROCOLLAGEN ALPHA 1(I) CHAIN PRECURSOR.
2.2e-05:172:34
MUS MUSCULUS (MOUSE).
P11087
F-HEMBA1000940
GAP JUNCTION CX43. 4 PROTE I N (CONNEXIN 43. 4) (CX43.4).
1.4e-20:90:42
BRACHYDANIO RERIO (ZEBRAFISH) (ZEBRA DANIO).
Q92052
F-HEMBA1000962
WATER-STRESS INDUCIBLE PROTEIN RAB21.
0.089:122:25
ORYZA SATIVA (RICE).
P12253
F-HEMBA1001184
SH3BGR PROTEIN (21-GLUTAMIC ACID-RICH PROTEIN) (21-GARP).
4.9e-33:100:60
HOMO SAPIENS (HUMAN).
P55822
F-HEMBA1001221
AGRIN PRECURSOR.
1.7e-26:239:32
GALLUS GALLUS (CHICKEN).
P31696
F-HEMBA1001228
CARTILAGE OLIGOMERIC MATRIX PROTEIN PRECURSOR (COMP).
7.7e-114:147:83
HOMO SAPIENS (HUMAN).
P49747
F-HEMBA1001272
SPLICEOSOME ASSOCIATED PROTEIN 49 (SAP 49) (SF3B53).
5.8e-06:129:33
HOMO SAPIENS (HUMAN).
Q15427
F-HEMBA1001296
TRANSCRIPTION FACTOR BF-2 (BRAIN FACTOR 2) (BF2) (CBF-2) (T-14-6).
0.0019:115:36
GALLUS GALLUS (CHICKEN).
Q98937
F-HEMBA1001297
50S RIBOSOMAL PROTEIN L37E (L35E).
0. 65:40:40
HALOARCULA MARISMORTUI (HALOBACTERIUM MARISMORTUI).
P32410
F-HEMBA1001390
SPIDROIN 2 (DRAGLINE SILK FIBROIN 2) (FRAGMENT).
0.00050:89:33
NEPHILA CLAVIPES (ORB SPIDER).
P46804
F-HEMBA1001563
B-CELL GROWTH FACTOR PRECURSOR (BCGF-12 KD).
0.00041:34:61
HOMO SAPIENS (HUMAN).
P20931
F-HEMBA1001621
PROBABLE G PROTEIN-COUPLED RECEPTOR APJ.
1.1e-64:105:72
HOMO SAPIENS (HUMAN).
P35414
F-HEMBA1001878
VEGETATIBLE INCOMPATIBILITY PROTEIN HET-E-1.
1.3e-24:170:35
PODOSPORA ANSERINA.
Q00808
F-HEMBA1001886
ZINC FINGER PROTEIN 85 (ZINC FINGER PROTEIN HPF4) (HTF1).
1.8e-94:273:64
HOMO SAPIENS (HUMAN).
Q03923
F-HEMBA1002048
EARLY ANTIGEN PROTEIN D (EA-D).
0.13:93:34
EPSTEIN-BARR VIRUS (STRAIN B95-8) (HUMAN HERPESVIRUS 4).
P03191
F-HEMBA1002131
PROCOLLAGEN-LYSINE, 2-OXOGLUTARATE 5-DIOXYGENASE 1 PRECURSOR (EC 1.14.11.4) (LYSYL HYDROXYLASE 1) (LH1).
6.3e-12:140:30
GALLUS GALLUS (CHICKEN).
P24802
F-HEMBA1002163
HYPOTHETICAL 40.7 KD PROTEIN IN DAK1-ORC1 INTERGENIC REGION.
2.1e-10:204:27
SACCHAROMYCES CEREVISIAE (BAKER'S YEAST).
Q04651
F-HEMBA1002164
BIOTIN CARBOXYL CARRIER PROTEIN OF ACETYL-COA CARBOXYLASE PRECURSOR (EC 6.4.1.2) (BCCP).
0.022:62:32
GLYCINE MAX (SOYBEAN).
Q42783
F-HEMBA1002167
ACETYLCHOLINESTERASE PRECURSOR (EC 3.1.1.7).
5.2e-31:247:31
BUNGARUS FASCIATUS (BANDED KRAIT).
Q92035
F-HEMBA1002178
PROCOLLAGEN-LYSINE,2-OXOGLUTARATE 5-DIOXYGENASE 1 PRECURSOR (EC 1.14.11.4) (LYSYL HYDROXYLASE 1) (LH1).
1.5e-11:140:30
GALLUS GALLUS (CHICKEN).
P24802
F-HEMBA1002195
VEGETATIBLE INCOMPATIBILITY PROTEIN HET-E-1.
5.0e-07:52:36
PODOSPORA ANSERINA.
Q00808
F-HEMBA1002227
MYRISTOYLATED ALANINE-RICH C-KINASE SUBSTRATE (MARCKS) (ACAMP-81).
0. 00063 : 21 :100
BOS TAURUS (BOVINE).
P12624
F-HEMBA1002239
!!!! ALU SUBFAMILY SC WARNING ENTRY !!!!
1.5e-33:101:70
HOMO SAPIENS (HUMAN).
P39192
F-HEMBA1002316
EXTENSIN PRECURSOR (PROLINE-RICH GLYCOPROTEIN).
4.6e-08:186:32
SORGHUM VULGARE (SORGHUM).
P24152
F-HEMBA1002420
WISKOTT-ALDRICH SYNDROME PROTEIN HOMOLOG (WASP).
0.0078:19:68
MUS MUSCULUS (MOUSE).
P70315
F-HEMBA1002421
SYNDECAN-2 PRECURSOR (FIBROGLYCAN) (HEPARAN SULFATE PROTEOGLYCAN CORE PROTEIN) (HSPG) (SYND2).
1.1e-52:107:97
HOMO SAPIENS (HUMAN).
P34741
F-HEMBA1002524
ACIDIC PROLINE-RICH PROTEIN PRECURSOR (CLONE PRP33).
5.0e-05:104:34
RATTUS NORVEGICUS (RAT).
P04474
F-HEMBA1002551
HYPOTHETICAL WD-REPEAT PROTEIN SLR0143.
9.9e-09:128:29
SYNECHOCYSTIS SP. (STRAIN PCC 6803).
P74442
F-HEMBA1002767
N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
8.0e-92:246:67
MUS MUSCULUS (MOUSE).
P15535
F-HEMBA1002985
TRANSCRIPTION FACTOR GATA-6 (GATA BINDING FACTOR-6).
0.060:49:34
MUS MUSCULUS (MOUSE).
Q61169
F-HEMBA1002992
HOLOTRICIN 3 PRECURSOR.
0.0035:64:37
HOLOTRICHIA DIOMPHALIA.
Q25055
F-HEMBA1003047
BONE MORPHOGENETIC PROTEIN 1 PRECURSOR (EC 3.4.24.-) (BMP-1).
1. 5e-23:216:31
HOMO SAPIENS (HUMAN).
P13497
F-HEMBA1003072
PROLINE-RICH PROTEIN MP-2 PRECURSOR.
6.8e-09:129:41
MUS MUSCULUS (MOUSE).
P05142
F-HEMBA1003101
PROCOLLAGEN ALPHA 2(I) CHAIN PRECURSOR.
2.2e-10:124:37
HOMO SAPIENS (HUMAN).
P08123
F-HEMBA1003120
ZINC FINGER PROTEIN 140.
4.8e-23:43:74
HOMO SAPIENS (HUMAN).
P52738
F-HEMBA1003230
FIBULIN-1, ISOFORM C PRECURSOR (BASEMENT-MEMBRANE PROTEIN 90) (BM-90).
2.7e-41:239:39
MUS MUSCULUS (MOUSE).
Q08878
F-HEMBA1003294
!!!! ALU SUBFAMILY SQ WARNING ENTRY !!!!
7.0e-34:84:69
HOMO SAPIENS (HUMAN).
P39194
F-HEMBA1003315
GLYCINE-RICH CELL WALL STRUCTURAL PROTEIN 1.8 PRECURSOR (GRP 1.8).
0. 00012:178:32
PHASEOLUS VULGARIS (KIDNEY BEAN) (FRENCH BEAN).
P10496
F-HEMBA1003392
LOW-DENSITY LIPOPROTEIN RECEPTOR-RELATED PROTEIN 1 PRECURSOR (LRP) (ALPHA-2-MACROGLOBULIN RECEPTOR) (A2MR).
1. 1e-31:202:37
GALLUS GALLUS (CHICKEN).
P98157
F-HEMBA1003399
MVP1 PROTEIN.
5.6e-12:67:38
SACCHAROMYCES CEREVISIAE (BAKER'S YEAST).
P40959
F-HEMBA1003487
PROLINE-RICH PROTEIN MP-2 PRECURSOR.
4.5e-08:175:29
MUS MUSCULUS (MOUSE).
P05142
F-HEMBA1003497
ZINC FINGER PROTEIN 151 (POLYOMAVIRUS LATE INITIATOR PROMOTER BINDING PROTEIN) (LP-1) (ZINC FINGER PROTEIN Z13).
9.3e-18:171:33
MUS MUSCULUS (MOUSE).
Q60821
F-HEMBA1003530
SALIVARY PROLINE-RICH PROTEIN II-1 (FRAGMENT).
9. 9e-12:122:35
HOMO SAPIENS (HUMAN).
P81489
F-HEMBA1003602
PROLINE-RICH PROTEIN MP-3 (FRAGMENT).
0.98:114:33
MUS MUSCULUS (MOUSE).
P05143
F-HEMBA1003732
TRANSCRIPTIONAL REGULATORY PROTEIN ALGP (ALGINATE REGULATORY PROTEIN ALGR3).
0.35:225:28
PSEUDOMONAS AERUGINOSA.
P15276
F-HEMBA1003945
HYPOTHETICAL 43.7 KD PROTEIN C24B11.08C IN CHROMOSOME I.
2.9e-48:268:41
SCHIZOSACCHAROMYCES POMBE (FISSION YEAST).
Q09895
F-HEMBA1004007
THYROID TRANSCRIPTION FACTOR 1 (THYROID NUCLEAR FACTOR 1) (TTF-1) (FRAGMENT).
0.90:60:30
CAVIA PORCELLUS (GUINEA PIG).
P97273
F-HEMBA1004067
TRANS-ACTING TRANSCRIPTIONAL PROTEIN ICPO (IMMEDIATE-EARLY PROTEIN IE110) (VMW110) (ALPHA-0 PROTEIN).
3.0e-05:200:31
HERPES SIMPLEX VIRUS (TYPE 1 / STRAIN 17).
P08393
F-HEMBA1004085
GLUCOSE REPRESSION MEDIATOR PROTEIN.
0.0030:190:26
SACCHAROMYCES CEREVISIAE (BAKER'S YEAST).
P14922
F-HEMBA1004110
EPIDERMAL GROWTH FACTOR RECEPTOR SUBSTRATE SUBSTRATE 15 (PROTEIN EPS15).
1.2e-14:102:36
MUS MUSCULUS (MOUSE).
P42567
F-HEMBA1004250
CADHERIN-RELATED TUMOR SUPPRESSOR PRECURSOR (FAT PROTEIN).
1.8e-08:150:33
DROSOPHILA MELANOGASTER (FRUIT FLY).
P33450
F-HEMBA1004391
TUMOR SUPPRESSOR PROTEIN DCC PRECURSOR.
4.5e-09:96:35
MUS MUSCULUS (MOUSE).
P70211
F-HEMBA1004444
GLYCOPROTEIN 25L PRECURSOR (GP25L).
4.6e-41:148:52
CANIS FAMILIARIS (DOG).
P27869
F-HEMBA1004454
CD9 ANTIGEN.
0.0070:24:70
BOS TAURUS (BOVINE).
P30932
F-HEMBA1004505
MANNOSYL-OLIGOSACCHARIDE ALPHA-1,2-MANNOSIDASE ISOFORM 1 (EC 3.2.1.113) (MAN(9)-ALPHA-MANNOSIDASE).
7.0e-45:239:43
DROSOPHILA MELANOGASTER (FRUIT FLY).
P53624
F-HEMBA1004785
MODIFIER 3 PROTEIN (M33).
7.4e-26:76:61
MUS MUSCULUS (MOUSE).
P30658
F-HEMBA1004797
PROTEIN Q300.
0.00071:21:66
MUS MUSCULUS (MOUSE).
Q02722
F-HEMBA1004952
EBNA-1 NUCLEAR PROTEIN.
2.4e-05:67:49
EPSTEIN-BARR VIRUS (STRAIN B95-8) (HUMAN HERPESVIRUS 4).
P03211
F-HEMBA1004971
F-HEMBA1004982
MULTIDRUG RESISTANCE PROTEIN 2 (MULTIDRUG-EFFLUX TRANSPORTER 2).
8.6e-08:144:25
BACILLUS SUBTILIS.
P39843
F-HEMBA1005070
HYPOTHETICAL PROTEIN KIAA0310.
1.0e-38:140:68
HOMO SAPIENS (HUMAN).
015027
F-HEMBA1005084
NON-RECEPTOR TYROSINE KINASE SPORE LYSIS A (EC 2.7.1.112) (TYROSINE- PROTEIN KINASE 1).
2.5e-10:102:37
DICTYOSTELIUM DISCOIDEUM (SLIME MOLD).
P18160
F-HEMBA1005145
PROLINE-RICH PROTEIN MP-2 PRECURSOR.
1.8e-06:85:37
MUS MUSCULUS (MOUSE).
P05142
F-HEMBA1005230
ZINC FINGER PROTEIN 140.
8.2e-20:83:66
HOMO SAPIENS (HUMAN).
P52738
F-HEMBA1005246
TRANSCRIPTION REGULATORY PROTEIN SNF5 (SWI/SNF COMPLEX COMPONENT SNF5) (TRANSCRIPTION FACTOR TYE4).
1.5e-09:132:34
SACCHAROMYCES CEREVISIAE (BAKER'S YEAST).
P18480
F-HEMBA1005267
B-CELL LYMPHOMA 3-ENCODED PROTEIN (BCL-3 PROTEIN).
1.9e-15:192:32
HOMO SAPIENS (HUMAN).
P20749
F-HEMBA1005337
ACIDIC PHOSPHOPROTEIN PRECURSOR (50 KD ANTIGEN).
8.0e-05:31:64
PLASMODIUM CHABAUDI.
Q02752
F-HEMBA1005430
MALE SPECIFIC SPERM PROTEIN MST84DB.
0.34:42:42
DROSOPHILA MELANOGASTER (FRUIT FLY).
Q01643
F-HEMBA1005449
PROCYCLIC FORM SPECIFIC POLYPEPTIDE B-ALPHA PRECURSOR (PROCYCLIN) (PARP B-ALPHA) (PSSA-1).
4.5e-09:95:33
TRYPANOSOMA BRUCEI BRUCEI.
Q06084
F-HEMBA1005489
CHANNEL ASSOCIATED PROTEIN OF SYNAPSE-110 (CHAPSYN-110).
7.2e-05:90:36
HOMO SAPIENS (HUMAN).
Q15700
F-HEMBA1005522
COAGULATION FACTOR VII PRECURSOR (EC 3.4.21.21).
3.3e-17:78:51
ORYCTOLAGUS CUNICULUS (RABBIT).
P98139
F-HEMBA1005545
MUSCARINIC ACETYLCHOLINE RECEPTOR M3.
7.2e-91:211:85
HOMO SAPIENS (HUMAN).
P20309
F-HEMBA1005698
PROTEIN TRANSPORT PROTEIN SEC22 (PROTEIN SLY2).
3.3e-08:132:28
SACCHAROMYCES CEREVISIAE (BAKER'S YEAST).
P22214
F-HEMBA1005913
HYPOTHETICAL 5.8 KD PROTEIN.
0.97:43:30
CLOVER YELLOW MOSAIC VIRUS (CYMV).
P16485
F-HEMBA1005929
PUTATIVE SERINE/THREONINE-PROTEIN KINASE P78 (EC 2.7.1.-).
6.6e-104:275:72
HOMO SAPIENS (HUMAN).
P27448
F-HEMBA1005945
BRITTLE-1 PROTEIN PRECURSOR.
7.8e-30:214:35
ZEA MAYS (MAIZE).
P29518
F-HEMBA1006016
!!!! ALU SUBFAMILY J WARNING ENTRY !!!!
1.9e-07:34:76
HOMO SAPIENS (HUMAN).
P39188
F-HEMBA1006171
PROBABLE E5 PROTEIN.
0.98:66:31
HUMAN PAPILLOMAVIRUS TYPE 33.
P06426
F-HEMBA1006276
ZINC FINGER PROTEIN 90 (ZFP-90) (ZINC FINGER PROTEIN NK10).
4.1e-07:56:57
MUS MUSCULUS (MOUSE).
Q61967
F-HEMBA1006299
BASIC PROLINE-RICH PEPTIDE P-E (IB-9).
0.11:38:28
HOMO SAPIENS (HUMAN).
P02811
F-HEMBA1006311
ZINC FINGER PROTEIN 23 (ZINC FINGER PROTEIN KOX16) (FRAGMENT).
0.91:22:45
HOMO SAPIENS (HUMAN).
P17027
F-HEMBA1006335
PERIPHERAL MYELIN PROTEIN 22 (PMP-22) (GROWTH-ARREST-SPECIFIC PROTEIN 3) (GAS3).
0.017:125:27
MUS MUSCULUS (MOUSE).
P16646
F-HEMBA1006357
SECRETORY CARRIER-ASSOCIATED MEMBRANE PROTEIN 2.
5.2e-40:136:52
HOMO SAPIENS (HUMAN).
015127
F-HEMBA1006430
OLIGOSACCHARYL TRANSFERASE STT3 SUBUNIT HOMOLOG.
2.7e-38:96:72
CAENORHABDITIS ELEGANS.
P46975
F-HEMBA1006482
SC01 PROTEIN PRECURSOR.
7.1e-25:84:45
SACCHAROMYCES CEREVISIAE (BAKER'S YEAST).
P23833
F-HEMBA1006517
HYPOTHETICAL 93.4 KD PROTEIN IN STE3-GIN10 INTERGENIC REGION.
0.48:145:30
SACCHAROMYCES CEREVISIAE (BAKER'S YEAST).
P34239
F-HEMBA1006544
TRANSCRIPTION INITIATION FACTOR TFIID 135 KD SUBUNIT (TAFII-135) (TAF11135) (TAFII-130) (TAFII130).
7.0e-11:210:37
HOMO SAPIENS (HUMAN).
000268
F-HEMBA1006572
ANTITHROMBIN-III PRECURSOR (ATIII) (FRAGMENT).
0.011:50:40
GALLUS GALLUS (CHICKEN).
Q03352
F-HEMBA1006658
SERINE/THREONINE-PROTEIN KINASE MIG-15 (EC 2.7.1.-).
4.6e-44:234:45
CAENORHABDITIS ELEGANS.
Q23356
F-HEMBA1006707
CARTILAGE MATRIX PROTEIN PRECURSOR (MATRILIN-1).
9.3e-34:159:50
GALLUS GALLUS (CHICKEN).
P05099
F-HEMBA1006724
PLATELET FACTOR 4 (PF-4).
0.025:65:27
SUS SCROFA (PIG).
P30034
F-HEMBA1006749
CARTILAGE MATRIX PROTEIN PRECURSOR (MATRILIN-1).
7.9e-37:147:53
GALLUS GALLUS (CHICKEN).
P05099
F-HEMBA1006770
FLOWERING TIME CONTROL PROTEIN FCA.
3.4e-27:139:39
ARABIDOPSIS THALIANA (MOUSE-EAR CRESS).
004425
F-HEMBA1006902
CARTILAGE MATRIX PROTEIN PRECURSOR (MATRILIN-1).
1. 5e-37:137:51
GALLUS GALLUS (CHICKEN).
P05099
F-HEMBA1006912
TRANS-ACTING TRANSCRIPTIONAL PROTEIN ICPO (IMMEDIATE-EARLY PROTEIN IE110) (VMW110) (ALPHA-0 PROTEIN).
0.27:121:29
HERPES SIMPLEX VIRUS (TYPE 1 / STRAIN 17).
P08393
F-HEMBA1006916
SKIN SECRETORY PROTEIN XP2 PRECURSOR (APEG PROTEIN).
1.1e-05:163:30
XENOPUS LAEVIS (AFRICAN CLAWED FROG).
P17437
F-HEMBA1006960
SMALL PROLINE-RICH PROTEIN 2-1.
1.0:34:35
HOMO SAPIENS (HUMAN).
P35326
F-HEMBA1007013
S-ANTIGEN PROTEIN PRECURSOR.
2.8e-09:226:28
PLASMODIUM FALCIPARUM (ISOLATE V1).
P09593
F-HEMBA1007057
PROCYCLIC FORM SPECIFIC POLYPEPTIDE B-ALPHA PRECURSOR (PROCYCLIN) (PARP B-ALPHA) (PSSA-1).
4.0e-10:33:72
TRYPANOSOMA BRUCEI BRUCEI.
Q06084
F-HEMBA1007063
AGAMOUS PROTEIN.
1.0:40:42
ARABIDOPSIS THALIANA (MOUSE-EAR CRESS).
P17839
F-HEMBA1007226
PUTATIVE CUTICLE COLLAGEN C09G5.5.
0.10:105:38
CAENORHABDITIS ELEGANS.
Q09456
F-HEMBA1007241
HYPOTHETICAL 24.5 KD PROTEIN IN SAP185-BCK1 INTERGENIC REGION.
3.3e-15:106:42
SACCHAROMYCES CEREVISIAE (BAKER'S YEAST).
P40857
F-HEMBA1007291
RETINOIC ACID RECEPTOR RXR-BETA.
0.0013:124:33
HOMO SAPIENS (HUMAN).
P28702
F-HEMBA1007332
ALPHA-2A ADRENERGIC RECEPTOR (ALPHA-2A ADRENOCEPTOR) (ALPHA-2AAR).
0.0024:130:34
MUS MUSCULUS (MOUSE).
Q01338
F-HEMBB1000106
CELLULAR NUCLEIC ACID BINDING PROTEIN (CNBP).
9.5e-09:99:33
MUS MUSCULUS (MOUSE).
P53996
F-HEMBB1000276
F-HEMBB1000309
F-HEMBB1000407
TRANS-ACTING TRANSCRIPTIONAL PROTEIN ICPO (VMW118 PROTEIN).
0.38:99:34
HERPES SIMPLEX VIRUS (TYPE 2 / STRAIN HG52).
P28284
F-HEMBB1000447
HISTIDINE-RICH GLYCOPROTEIN PRECURSOR.
0.0076:80:31
PLASMODIUM LOPHURAE.
P04929
F-HEMBB1000542
BETA-2 BUNGAROTOXIN B CHAIN PRECURSOR (BUNGAROTOXIN, B2 CHAIN).
0.017:53:33
BUNGARUS MULTICINCTUS (MANY-BANDED KRAIT).
P00989
F-HEMBB1000567
HISTIDINE-RICH GLYCOPROTEIN PRECURSOR.
5.0e-05:131:29
PLASMODIUM LOPHURAE.
P04929
F-HEMBB1000642
BASIC PROLINE-RICH PEPTIDE IB-1.
0.0074:66:31
HOMO SAPIENS (HUMAN).
P04281
F-HEMBB1000668
VEGETATIBLE INCOMPATIBILITY PROTEIN HET-E-1.
7.3e-10:184:32
PODOSPORA ANSERINA.
Q00808
F-HEMBB1000679
TRAM PROTEIN (TRANSLOCATING CHAIN-ASSOCIATING MEMBRANE PROTEIN).
9.5e-73:204:69
CANIS FAMILIARIS (DOG).
Q01685
F-HEMBB1000881
F-SPONDIN PRECURSOR.
1. 2e-23:191 :37
XENOPUS LAEVIS (AFRICAN CLAWED FROG).
P35447
F-HEMBB1000905
TRANSCRIPTIONAL REPRESSOR RCO-1.
0.068:105:34
NEUROSPORA CRASSA.
P78706
F-HEMBB1001026
ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
1.3e-11:138:31
SACCHAROMYCES CEREVISIAE (BAKER'S YEAST).
P32802
F-HEMBB1001048
SARCALUMENIN PRECURSOR.
3.1e-20:151:32
ORYCTOLAGUS CUNICULUS (RABBIT).
P13666
F-HEMBB1001200
HYPOTHETICAL GENE 51 MEMBRANE PROTEIN.
1.0:66:27
ICTALURID HERPESVIRUS 1 (CHANNEL CATFISH VIRUS) (CCV). Q00135
F-HEMBB1001407
!!!! ALU SUBFAMILY SQ WARNING ENTRY !!!!
2.0e-24:58:60
HOMO SAPIENS (HUMAN).
P39194
F-HEMBB1001530
SLS1 PROTEIN PRECURSOR.
0. 0012:37:51
YARROWIA LIPOLYTICA (CANDIDA LIPOLYTICA).
Q99158
F-HEMBB1001547
HYPOTHETICAL 71.7 KD PROTEIN F52H3.2 IN CHROMOSOME II.
4.1e-49:200:55
CAENORHABDITIS ELEGANS.
Q20680
F-HEMBB1001573
PROTEIN Q300.
0.0055:27:62
MUS MUSCULUS (MOUSE).
Q02722
F-HEMBB1001847
ACIDIC PROLINE-RICH PROTEIN PRECURSOR (CLONE PRP33).
7.8e-05:166:31
RATTUS NORVEGICUS (RAT).
P04474
F-HEMBB1001959
CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
8.8e-15:97:38
PETROMYZON MARINUS (SEA LAMPREY).
P25210
F-HEMBB1001978
MICROCIN B17 PROCESSING PROTEIN MCBC.
0.049:100:31
ESCHERICHIA COLI.
P23185
F-HEMBB1002039
COLD SHOCK PROTEIN CSPB (FRAGMENT).
0.98:32:40
BACILLUS GLOBISPORUS.
P41018
F-HEMBB1002041
REGULATORY PROTEIN E2.
0.010:145:35
HUMAN PAPILLOMAVIRUS TYPE 47.
P22420
F-HEMBB1002051
FLI-1 ONCOGENE (ERGB TRANSCRIPTION FACTOR).
0.0056:89:31
HOMO SAPIENS (HUMAN).
Q01543
F-HEMBB1002120
UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).
1.4e-08:154:30
RATTUS NORVEGICUS (RAT).
P56558
F-HEMBB1002162
IMMEDIATE-EARLY PROTEIN IE180.
0.86:130:31
PSEUDORABIES VIRUS (STRAIN INDIANA-FUNKHAUSER / BECKER) (PRV).
P11675
F-HEMBB1002228
PTB-ASSOCIATED SPLICING FACTOR (PSF).
0.00092:97:34
HOMO SAPIENS (HUMAN).
P23246
F-HEMBB1002245
PROSTAGLANDIN F2-ALPHA RECEPTOR REGULATORY PROTEIN PRECURSOR (PROSTAGLANDIN F2-ALPHA RECEPTOR ASSOCIATED PROTEIN).
2.5e-55:128:88
RATTUS NORVEGICUS (RAT).
Q62786
F-HEMBB1002302
REGULATORY PROTEIN E2.
0.042:100:37
HUMAN PAPILLOMAVIRUS TYPE 25.
P36787
F-HEMBB1002427
FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).
5.0e-15:53:54
HOMO SAPIENS (HUMAN).
P16442
F-HEMBB1002465
ACYL-COA DEHYDROGENASE (EC 1.3.99.-).
8.2e-35:162:50
BACILLUS SUBTILIS.
P45857
F-HEMBB1002661
TRANSCRIPTION FACTOR HES-1 (C-HAIRY1).
2. 2e-18:159:40
GALLUS GALLUS (CHICKEN).
057337
F-HEMBB1002663
F-HEMBB1002693
GAG POLYPROTEIN [CONTAINS: CORE PROTEIN P15: INNER COAT PROTEIN P12; CORE SHELL PROTEIN P30; NUCLEOPROTEIN P10].
0.83:74:28
DUPLAN MURINE LEUKEMIA VIRUS.
P23090
F-MAMMA1000046
!!!! ALU SUBFAMILY SB2 WARNING ENTRY !!!!
2.3e-24:98:67
HOMO SAPIENS (HUMAN).
P39191
F-MAMMA1000102
APOLIPOPROTEIN L PRECURSOR (APO-L).
4.3e-22:213:34
HOMO SAPIENS (HUMAN).
014791
F-MAMMA1000106
PISTIL-SPECIFIC EXTENSIN-LIKE PROTEIN PRECURSOR (PELP).
1.6e-07:99:31
NICOTIANA TABACUM (COMMON TOBACCO).
Q03211
F-MAMMA1000118
HYPOTHETICAL 29.3 KD PROTEIN (ORF92).
0.00059:155:30
ORGYIA PSEUDOTSUGATA MULTICAPSID POLYHEDROSIS VIRUS (OPMNPV).
010341
F-MAMMA1000141
!!!! ALU SUBFAMILY SX WARNING ENTRY !!!!
0.00011:39:66
HOMO SAPIENS (HUMAN).
P39195
F-MAMMA1000204
SYNAPTOTAGMIN III (SYTIII).
5.9e-05:93:33
MUS MUSCULUS (MOUSE).
035681
F-MAMMA1000226
GLUTENIN, HIGH MOLECULAR WEIGHT SUBUNIT DX5 PRECURSOR.
4.6e-06:224:28
TRITICUM AESTIVUM (WHEAT).
P10388
F-MAMMA1000403
COLLAGEN ALPHA 1(IX) CHAIN PRECURSOR (FRAGMENTS).
1.1e-06:158:35
GALLUS GALLUS (CHICKEN).
P12106
F-MAMMA1000449
SYNAPSINS IA AND IB (BRAIN PROTEIN 4.1).
6.3e-05:137:32
HOMO SAPIENS (HUMAN).
P17600
F-MAMMA1000457
NADH-CYTOCHROME B5 REDUCTASE (EC 1.6.2.2).
7.6e-48:151:62
BOS TAURUS (BOVINE).
P07514
F-MAMMA1000473
SPERM PROTAMINE P1.
0.024:29:44
DROMICIOPS AUSTRALIS (MONITO DEL MONTE) (DROMICIOPS GLIROIDES).
P42132
F-MAMMA1000496
HYPOTHETICAL 73.0 KD PROTEIN IN CLA4-MID1 INTERGENIC REGION.
9.8e-09:188:26
SACCHAROMYCES CEREVISIAE (BAKER'S YEAST).
P48566
F-MAMMA1000528
DNA BINDING PROTEIN S1FA.
0.77:43:37
ARABIDOPSIS THALIANA (MOUSE-EAR CRESS).
P42551
F-MAMMA1000591
SALIVARY PROLINE-RICH PROTEIN PO PRECURSOR (ALLELE S).
0.018:88:32
HOMO SAPIENS (HUMAN).
P10163
F-MAMMA1000614
HYPOTHETICAL 29.3 KD PROTEIN (ORF92).
7.5e-08:148:36
ORGYIA PSEUDOTSUGATA MULTICAPSID POLYHEDROSIS VIRUS (OPMNPV).
010341
F-MAMMA1000652
!!!! ALU SUBFAMILY SB WARNING ENTRY !!!!
5.3e-36:56:85
HOMO SAPIENS (HUMAN).
P39189
F-MAMMA1000681
PROBABLE G PROTEIN-COUPLED RECEPTOR EDG-1.
7.5e-41:167:51
MUS MUSCULUS (MOUSE).
008530
F-MAMMA1000706
T-CELL RECEPTOR BETA CHAIN PRECURSOR (ANA 11).
8.1e-10:135:38
ORYCTOLAGUS CUNICULUS (RABBIT).
P06333
F-MAMMA1000788
HYPOTHETICAL 57.5 KD PROTEIN IN VMA7-RPS25A INTERGENIC REGION.
2.0e-06:214:32
SACCHAROMYCES CEREVISIAE (BAKER'S YEAST).
P53214
F-MAMMA1000810
REGULATORY PROTEIN E2.
0.0031:132:31
HUMAN PAPILLOMAVIRUS TYPE 9.
P36780
F-MAMMA1000814
PROTEIN Q300.
1.6e-05:27:66
MUS MUSCULUS (MOUSE).
Q02722
F-MAMMA1000881
SERINE/THREONINE-PROTEIN KINASE SGK (EC 2.7.1.-) (SERUM/GLUCOCORTICOID-REGULATED KINASE).
2.7e-10:81:45
RATTUS NORVEGICUS (RAT).
Q06226
F-MAMMA1000986
INVOLUCRIN.
0.95:125:24
SUS SCROFA (PIG).
P18175
F-MAMMA1000994
CUTICLE COLLAGEN 2C (FRAGMENT).
0.00062:97:34
HAEMONCHUS CONTORTUS.
P16252
F-MAMMA1001043
MRC OX-45 SURFACE ANTIGEN PRECURSOR (BCM1 SURFACE ANTIGEN) (BLAST-1) (CD48).
4.5e-05:162:25
RATTUS NORVEGICUS (RAT).
P10252
F-MAMMA1001066
B-CELL GROWTH FACTOR PRECURSOR (BCGF-12 KD).
2.6e-06:33:72
HOMO SAPIENS (HUMAN).
P20931
F-MAMMA1001094
PUTATIVE GENERAL NEGATIVE REGULATOR OF TRANSCRIPTION C16C9.04C.
1.1e-21:175:38
SCHIZOSACCHAROMYCES POMBE (FISSION YEAST).
Q09818
F-MAMMA1001141
PTB-ASSOCIATED SPLICING FACTOR (PSF).
0.13:196:27
HOMO SAPIENS (HUMAN).
P23246
F-MAMMA1001150
PROTEIN KINASE C, MU TYPE (EC 2.7.1.-) (NPKC-MU).
9.4e-89:256:67
HOMO SAPIENS (HUMAN).
Q15139
F-MAMMA1001237
MONOCARBOXYLATE TRANSPORTER 2 (MCT 2).
3.5e-19:103:43
MESOCRICETUS AURATUS (GOLDEN HAMSTER).
P53988
F-MAMMA1001284
AUTOIMMUNE REGULATOR (APECED PROTEIN).
0.027:178:30
HOMO SAPIENS (HUMAN).
043918
F-MAMMA1001310
HYPOTHETICAL 57.3 KD TRP-ASP REPEATS CONTAINING PROTEIN IN POM152- REC114 INTERGENIC REGION.
1.9e-14:151:31
SACCHAROMYCES CEREVISIAE (BAKER'S YEAST).
Q04225
F-MAMMA1001344
MALE SPECIFIC SPERM PROTEIN MST84DC.
0.16:35:42
DROSOPHILA MELANOGASTER (FRUIT FLY).
Q01644
F-MAMMA1001418
HYPOTHETICAL PROTEIN H10519.
3.5e-27:181:38
HAEMOPHILUS INFLUENZAE.
P44742
F-MAMMA1001532
ZINC FINGER PROTEIN 90 (ZFP-90) (ZINC FINGER PROTEIN NK10).
1.1e-34:78:58
MUS MUSCULUS (MOUSE).
Q61967
F-MAMMA1001609
TRANS-ACTING TRANSCRIPTIONAL PROTEIN ICPO (VMW118 PROTEIN).
0.20:137:27
HERPES SIMPLEX VIRUS (TYPE 2 / STRAIN HG52).
P28284
F-MAMMA1001615
5E5 ANTIGEN.
2.3e-07:205:34
RATTUS NORVEGICUS (RAT).
Q63003
F-MAMMA1001623
EXCISION REPAIR PROTEIN ERCC-6 (COCKAYNE SYNDROME PROTEIN CSB).
4.8e-30:90:77
HOMO SAPIENS (HUMAN).
Q03468
F-MAMMA1001634
B-CELL GROWTH FACTOR PRECURSOR (BCGF-12 KD).
6.1e-11:44:61
HOMO SAPIENS (HUMAN). P20931
F-MAMMA1001893
COLLAGEN ALPHA 1(XI) CHAIN PRECURSOR.
0.0013:174:36
HOMO SAPIENS (HUMAN).
P12107
F-MAMMA1001901
!!!! ALU SUBFAMILY SX WARNING ENTRY !!!!
1. 3e-21 : 65 : 66
HOMO SAPIENS (HUMAN).
P39195
F-MAMMA1001957
VITELLINE MEMBRANE PROTEIN VM26AB PRECURSOR (PROTEIN TU-4) (PROTEIN SV23).
0.0055:104:35
DROSOPHILA MELANOGASTER (FRUIT FLY).
P13238
F-MAMMA1001978
D(4) DOPAMINE RECEPTOR (D(2C) DOPAMINE RECEPTOR).
0.00030:101:44
HOMO SAPIENS (HUMAN).
P21917
F-MAMMA1002070
PLASM I NOGEN (EC 3.4.21.7) (FRAGMENT).
1.4e-08:103:33
RATTUS NORVEGICUS (RAT).
Q01177
F-MAMMA1002080
FMRFAMIDE-RELATED NEUROPEPTIDES PRECURSOR.
8.2e-08:131:32
LYMNAEA STAGNALIS (GREAT POND SNAIL).
P42565
F-MAMMA1002087
MALE SPECIFIC SPERM PROTEIN MST84DD.
0.65:24:45
DROSOPHILA MELANOGASTER (FRUIT FLY).
Q01645
F-MAMMA1002091
APYRASE PRECURSOR (EC 3.6.1.5) (ATP-DIPHOSPHATASE) (ADENOSINE DIPHOSPHATASE) (ADPASE) (ATP-DIPHOSPHOHYDROLASE).
2.6e-24:155:43
SOLANUM TUBEROSUM (POTATO).
P80595
F-MAMMA1002095
CALCIUM-TRANSPORTING ATPASE 1 (EC 3.6.1.38) (P-TYPE CALCIUM ATPASE).
2.3e-58:213:56
YARROWIA LIPOLYTICA (CANDIDA LIPOLYTICA).
043108
F-MAMMA1002128
COLLAGEN ALPHA 1(I) CHAIN (FRAGMENTS).
9.9e-05:72:40
RATTUS NORVEGICUS (RAT).
P02454
F-MAMMA1002142
NON-RECEPTOR TYROSINE KINASE SPORE LYSIS A (EC 2.7.1.112) (TYROSINE- PROTEIN KINASE 1).
2.1e-13:149:34
DICTYOSTELIUM DISCOIDEUM (SLIME MOLD).
P18160
F-MAMMA1002165
CONNECTIVE TISSUE GROWTH FACTOR PRECURSOR.
8.4e-06:47:57
HOMO SAPIENS (HUMAN).
P29279
F-MAMMA1002205
!!!! ALU SUBFAMILY J WARNING ENTRY !!!!
5.9e-26:56:78
HOMO SAPIENS (HUMAN).
P39188
F-MAMMA1002224
!!!! ALU SUBFAMILY SQ WARNING ENTRY !!!!
3.7e-16:62:67
HOMO SAPIENS (HUMAN).
P39194
F-MAMMA1002234
SIGNAL RECOGNITION PARTICLE 68 KD PROTEIN (SRP68).
3.5e-105:242:85
CANIS FAMILIARIS (DOG).
Q00004
F-MAMMA1002586
MANNOSYL-OLIGOSACCHARIDE ALPHA-1,2-MANNOSIDASE (EC 3.2.1.113) (MAN(9)-ALPHA-MANNOSIDASE) (ALPHA-MANNOSIDASE 1A).
4.7e-24:203:35
MUS MUSCULUS (MOUSE).
P45700
F-MAMMA1002633
!!!! ALU SUBFAMILY J WARNING ENTRY !!!!
7.3e-27:49:73
HOMO SAPIENS (HUMAN).
P39188
F-MAMMA1003126
SARCALUMENIN PRECURSOR.
7.9e-30:156:35
ORYCTOLAGUS CUNICULUS (RABBIT).
P13666
F-NT2RM1000407
LACTOSE OPERON REPRESSOR.
1.4e-07:36:86
ESCHERICHIA COLI.
P03023
F-NT2RM1000462
ATRIAL GLAND-SPECIFIC ANTIGEN PRECURSOR (AGSA).
6.7e-11:85:41
APLYSIA CALIFORNICA (CALIFORNIA SEA HARE).
P15287
F-NT2RM1000542
BETA-GALACTOSIDASE PRECURSOR (EC 3.2.1.23) (LACTASE) (ACID BETA-GALACTOSIDASE).
3.5e-19:104:48
FELIS SILVESTRIS CATUS (CAT).
019015
F-NT2RM1000580
GLYCOSYLTRANSFERASE ALG2 (EC 2.4.1.-).
3.4e-36:180:43
SACCHAROMYCES CEREVISIAE (BAKER'S YEAST).
P43636
F-NT2RM1000789
T-CELL-SPECIFIC TRANSCRIPTION FACTOR 1 (TCF-1) (T-CELL FACTOR 1) (TRANSCRIPTION FACTOR-7).
1.5e-40:112:75
MUS MUSCULUS (MOUSE).
Q00417
F-NT2RM1000855
PROTEIN TRANSPORT PROTEIN SEC61 ALPHA SUBUNIT.
2.5e-81:152:94
CANIS FAMILIARIS (DOG).
P38377
F-NT2RM1000858
HYPOTHETICAL 36.7 KD PROTEIN AH6.2 IN CHROMOSOME II.
3.1e-50:127:54
CAENORHABDITIS ELEGANS.
Q09201
F-NT2RM1000899
MITOCHONDRIAL RNA SPLICING PROTEIN MSR4.
6.6e-17:107:43
SACCHAROMYCES CEREVISIAE (BAKER'S YEAST).
P23500
F-NT2RM2000241
DOUBLESEX PROTEIN, MALE-SPECIFIC.
0.0021:64:32
DROSOPHILA MELANOGASTER (FRUIT FLY).
P23023
F-NT2RM2000306
PUTATIVE GTP-BINDING PROTEIN W08E3.3.
1.1e-69:198:69
CAENORHABDITIS ELEGANS.
P91917
F-NT2RM2000410
BETA-LYTIC METALLOENDOPEPTIDASE PRECURSOR (EC 3.4.24.32) (BETA-LYTIC PROTEASE).
0.73:118:31
ACHROMOBACTER LYTICUS.
P27458
F-NT2RM2000423
BETA GALACTOSIDASE-RELATED PROTEIN PRECURSOR.
1.3e-23:235:34
HOMO SAPIENS (HUMAN).
P16279
F-NT2RM2000497
DNA-REPAIR PROTEIN COMPLEMENTING XP-D CELLS (XERODERMA PIGMENTOSUM GROUP D COMPLEMENTING PROTEIN) (DNA EXCISION REPAIR PROTEIN ERCC-2).
9.4e-19:199:31
CRICETULUS GRISEUS (CHINESE HAMSTER).
Q60452
F-NT2RM2000514
HYPOTHETICAL PROTEIN H11558.
7.7e-06:82:34
HAEMOPHILUS INFLUENZAE.
P45252
F-NT2RM2000565
HYPOTHETICAL 85.7 KD PROTEIN C13G6.03 IN CHROMOSOME I.
2.8e-57:232:52
SCHIZOSACCHAROMYCES POMBE (FISSION YEAST).
Q09782
F-NT2RM2000582
PROTEIN Q300.
0. 066:13:84
MUS MUSCULUS (MOUSE).
Q02722
F-NT2RM2000589
RAC-FAMILY SERINE/THREONINE KINASE HOMOLOG (EC 2.7.1.-).
2.1e-07:90:32
DICTYOSTELIUM DISCOIDEUM (SLIME MOLD).
P54644
F-NT2RM2000622
GLUTENIN, HIGH MOLECULAR WEIGHT SUBUNIT PW212 PRECURSOR.
1.9e-07:133:35
TRITICUM AESTIVUM (WHEAT).
P08489
F-NT2RM2000632
EXCISION REPAIR PROTEIN ERCC-6 (COCKAYNE SYNDROME PROTEIN CSB).
4.6e-28:194:35
HOMO SAPIENS (HUMAN).
Q03468
F-NT2RM2000773
MYC-ASSOCIATED ZINC FINGER PROTEIN (MAZI) (PUR-1) (ZF87).
3.4e-24:156:47
HOMO SAPIENS (HUMAN).
P56270
F-NT2RM2001126
NEURON-SPECIFIC X11 PROTEIN (FRAGMENT).
1.5e-05:118:32
MUS MUSCULUS (MOUSE).
P98084
F-NT2RM2001558
MAJOR FIBROUS SHEATH PROTEIN PRECURSOR (FSC1) (P82).
1.9e-24:164:40
MUS MUSCULUS (MOUSE).
Q60662
F-NT2RM2001626
HYPOTHETICAL 118.4 KD PROTEIN IN BAT2-DAL5 INTERGENIC REGION PRECURSOR.
1.6e-09:206:33
SACCHAROMYCES CEREVISIAE (BAKER'S YEAST).
P47179
F-NT2RM2001643
HYPOTHETICAL PROTEIN MJ1025.
0.21:203:22
METHANOCOCCUS JANNASCHII.
Q58431
F-NT2RM2001738
REGULATORY PROTEIN E2.
0.0076:124:31
HUMAN PAPILLOMAVIRUS TYPE 25.
P36787
F-NT2RM2001767
HOMEOTIC GENE REGULATOR (BRAHMA PROTEIN).
0.0068:115:33
DROSOPHILA MELANOGASTER (FRUIT FLY).
P25439
F-NT2RM2001792
FIBRINOGEN BETA CHAIN.
4.3e-25:121:45
BOS TAURUS (BOVINE).
P02676
F-NT2RM2001818
EXTENSIN PRECURSOR (PROLINE-RICH GLYCOPROTEIN).
2.4e-06:192:32
ZEA MAYS (MAIZE).
P14918
F-NT2RM2001902
SERINE/THREONINE-PROTEIN KINASE PAK-BETA (EC 2.7.1.-) (BETA-PAK) (P21-ACTIVATED KINASE 3) (P65-PAK).
2.3e-52:250:45
RATTUS NORVEGICUS (RAT).
Q62829
F-NT2RM2001939
PROBABLE G PROTEIN-COUPLED RECEPTOR GPR19 (GPR-NGA).
4.0e-97:204:92
HOMO SAPIENS (HUMAN).
Q15760
F-NT2RM2001941
MUSCARINIC ACETYLCHOLINE RECEPTOR M4.
1.0e-34:184:32
HOMO SAPIENS (HUMAN).
P08173
F-NT2RM4000100
EBNA-1 NUCLEAR PROTEIN.
1.7e-05:86:39
EPSTEIN-BARR VIRUS (STRAIN B95-8) (HUMAN HERPESVIRUS 4).
P03211
F-NT2RM4000115
DNA-DIRECTED RNA POLYMERASE II LARGEST SUBUNIT (EC 2.7.7.6) (RNA POLYMERASE II SUBUNIT 1).
9.5e-05:116:35
SCHIZOSACCHAROMYCES POMBE (FISSION YEAST).
P36594
F-NT2RM4000198
BUTYROPHILIN PRECURSOR (BT).
8.6e-14:162:33
MUS MUSCULUS (MOUSE).
Q62556
F-NT2RM4000284
COLLAGEN ALPHA 2(VI) CHAIN (FRAGMENT).
0.86:95:37
HOMO SAPIENS (HUMAN).
P12110
F-NT2RM4000295
COLLAGEN ALPHA 1 (XVI) CHAIN PRECURSOR.
1.1e-08:229:34
HOMO SAPIENS (HUMAN).
Q07092
F-NT2RM4000326
SH3 DOMAIN-BINDING PROTEIN 3BP-2.
6.1e-05:187:31
HOMO SAPIENS (HUMAN).
P78314
F-NT2RM4000417
SYNAPTOTAGMIN (P65).
4.2e-08:72:52
APLYSIA CALIFORNICA (CALIFORNIA SEA HARE).
P41823
F-NT2RM4000444
ANTIGEN PEPTIDE TRANSPORTER 1 (APT1) (PEPTIDE TRANSPORTER TAP1) (PEPTIDE TRANSPORTER PSF1) (PEPTIDE SUPPLY FACTOR 1) (PSF-1) (PEPTIDE TRANSPORTER INVOLVED IN ANTIGEN PROCESSING 1).
1. 1e-36:192:41
HOMO SAPIENS (HUMAN).
Q03518
F-NT2RM4000587
COLLAGEN ALPHA 1(II) CHAIN (FRAGMENTS).
1.8e-13:200:38
BOS TAURUS (BOVINE).
P02459
F-NT2RM4000593
F-NT2RM4000648
K-GLYPICAN PRECURSOR.
6.4e-67:180:68
MUS MUSCULUS (MOUSE).
P51655
F-NT2RM4000761
CYTOCHROME C OXIDASE POLYPEPTIDE I (EC 1.9.3.1).
2.3e-53:107:81
RATTUS NORVEGICUS (RAT).
P05503
F-NT2RM4000965
PUTATIVE IMPORTIN BETA-4 SUBUNIT (KARYOPHERIN BETA-4 SUBUNIT).
4. 9e-14:188:34
SCHIZOSACCHAROMYCES POMBE (FISSION YEAST).
060100
F-NT2RM4000997
HISTONE H1C (CLONE XLHW2).
0.88:73:26
XENOPUS LAEVIS (AFRICAN CLAWED FROG).
P15866
F-NT2RM4001321
HOMEOBOX PROTEIN HOX-A2.
0.27:77:37
GALLUS GALLUS (CHICKEN).
Q08727
F-NT2RM4001325
CHONDROITIN 6-SULFOTRANSFERASE (EC 2.8.2.17) (C6ST).
3.8e-30:184:39
GALLUS GALLUS (CHICKEN).
Q92179
F-NT2RM4001377
HYPOTHETICAL BHLF1 PROTEIN.
5.9e-06:216:33
EPSTEIN-BARR VIRUS (STRAIN B95-8) (HUMAN HERPESVIRUS 4).
P03181
F-NT2RM4001735
GNS1 PROTEIN.
0.0028:114:29
SACCHAROMYCES CEREVISIAE (BAKER'S YEAST).
P25358
F-NT2RM4001768
PROBABLE OXIDOREDUCTASE (EC 1.-.-.-).
8.6e-24:205:36
STREPTOMYCES ANTIBIOTICUS.
Q03326
F-NT2RM4001843
BETA-GALACTOSIDASE PRECURSOR (EC 3.2.1.23) (LACTASE).
4.6e-33:258:37
XANTHOMONAS MANIHOTIS.
P48982
F-NT2RM4002352
BASEMENT MEMBRANE PROTEOGLYCAN PRECURSOR (PERLECAN HOMOLOG).
1.0e-15:85:45
CAENORHABDITIS ELEGANS.
Q06561
F-NT2RP1000002
GLYCINE-RICH CELL WALL STRUCTURAL PROTEIN (CLONE W10-1) (FRAGMENT).
0.00011:24:62
LYCOPERSICON ESCULENTUM (TOMATO).
Q01157
F-NT2RP1000050
A-AGGLUTININ ATTACHMENT SUBUNIT PRECURSOR.
2.5e-07:198:33
SACCHAROMYCES CEREVISIAE (BAKER'S YEAST).
P32323
F-NT2RP1000181
CYTOCHROME B5.
4.4e-11:117:29
SACCHAROMYCES CEREVISIAE (BAKER'S YEAST).
P40312
F-NT2RP1000239
TRANS-ACTING TRANSCRIPTIONAL PROTEIN ICPO (VMW118 PROTEIN).
7.8e-05:141:33
HERPES SIMPLEX VIRUS (TYPE 2 / STRAIN HG52).
P28284
F-NT2RP1000261
ORM1 PROTEIN.
2.2e-18:137:35
SACCHAROMYCES CEREVISIAE (BAKER'S YEAST).
P53224
F-NT2RP1000271
ZINC FINGER PROTEIN 85 (ZINC FINGER PROTEIN HPF4) (HTF1).
8.3e-81:194:70
HOMO SAPIENS (HUMAN).
Q03923
F-NT2RP1000300
HYPOTHETICAL 57.1 KD PROTEIN IN MAP2-TEL1 INTERGENIC REGION.
2.0e-07:202:24
SACCHAROMYCES CEREVISIAE (BAKER'S YEAST).
P38176
F-NT2RP1000325
MITOCHONDRIAL PHOSPHATE CARRIER PROTEIN PRECURSOR.
1.6e-55:86:81
HOMO SAPIENS (HUMAN).
Q00325
F-NT2RP1000448
PROLINE-RICH PEPTIDE P-B.
0.094:32:43
HOMO SAPIENS (HUMAN).
P02814
F-NT2RP1000465
EBNA-1 NUCLEAR PROTEIN.
3.1e-07:101:39
EPSTEIN-BARR VIRUS (STRAIN B95-8) (HUMAN HERPESVIRUS 4).
P03211
F-NT2RP1000468
CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
1.4e-14:97:38
PETROMYZON MARINUS (SEA LAMPREY).
P25210
F-NT2RP1000551
INTERFERON-RELATED PROTEIN PC4 (TPA INDUCED SEQUENCE 7) (TIS7 PROTEIN).
1.9e-33:221:41
MUS MUSCULUS (MOUSE).
P19182
F-NT2RP1000579
SUCCINATE DEHYDROGENASE [UBIQUINONE] FLAVOPROTEIN SUBUNIT PRECURSOR (EC 1.3.5.1) (FP) (FLAVOPROTEIN SUBUNIT OF COMPLEX II).
3.4e-68:247:62
HOMO SAPIENS (HUMAN).
P31040
F-NT2RP1000613
CARBONIC ANHYDRASE VI (EC 4.2.1.1) (SALIVARY) (CARBONATE DEHYDRATASE VI).
1.9e-19:137:37
OVIS ARIES (SHEEP).
P08060
F-NT2RP1000679
EBNA-1 NUCLEAR PROTEIN.
0.00055:54:50
EPSTEIN-BARR VIRUS (STRAIN B95-8) (HUMAN HERPESVIRUS 4).
P03211
F-NT2RP1000740
TRANSCRIPTION INITIATION FACTOR TFIID 135 KD SUBUNIT (TAFII-135) (TAFII135) (TAFII-130) (TAFII130).
0.071:71:45
HOMO SAPIENS (HUMAN).
000268
F-NT2RP1000903
SPORE COAT PROTEIN SP96.
0.016:124:26
DICTYOSTELIUM DISCOIDEUM (SLIME MOLD).
P14328
F-NT2RP1000981
CELL SURFACE A33 ANTIGEN PRECURSOR.
1.1e-08:196:28
HOMO SAPIENS (HUMAN).
Q99795
F-NT2RP1001004
F-SPONDIN PRECURSOR.
1.2e-11:155:31
XENOPUS LAEVIS (AFRICAN CLAWED FROG).
P35447
F-NT2RP1001020
SPORULATION-SPECIFIC PROTEIN 1 (EC 2.7.1.-).
2.2e-05:126:29
SACCHAROMYCES CEREVISIAE (BAKER'S YEAST).
P08458
F-NT2RP1001031
PUTATIVE SERINE/THREONINE-PROTEIN KINASE PKWA (EC 2.7.1.-).
5.8e-26:159:38
THERMOMONOSPORA CURVATA.
P49695
F-NT2RP1001563
METALLOTHIONEIN-III (MT-III) (GROWTH INHIBITORY FACTOR) (GIF).
0.00036:42:40
BOS TAURUS (BOVINE).
P37359
F-NT2RP2000092
ZINC FINGER PROTEIN 136.
2.9e-44:129:62
HOMO SAPIENS (HUMAN).
P52737
F-NT2RP2000178
HOMEOTIC GENE REGULATOR (BRAHMA PROTEIN).
0.0050:75:37
DROSOPHILA MELANOGASTER (FRUIT FLY).
P25439
F-NT2RP2000240
PUTATIVE CUTICLE COLLAGEN C09G5.5.
9.2e-08:137:34
CAENORHABDITIS ELEGANS.
Q09456
F-NT2RP2000394
PROCYCLIC FORM SPECIFIC POLYPEPTIDE A-BETA PRECURSOR (PROCYCLIN) (PARP A-BETA).
0.00019:28:64
TRYPANOSOMA BRUCEI BRUCEI.
P09791
F-NT2RP2000447
GOLGIN-95.
6.4e-25:55:67
HOMO SAPIENS (HUMAN).
Q08379
F-NT2RP2000479
PROBABLE E5B PROTEIN.
1.0:32:37
HUMAN PAPILLOMAVIRUS TYPE 6B.
P06461
F-NT2RP2000514
AXONIN-1 PRECURSOR (AXONAL GLYCOPROTEIN TAG-1) (TRANSIENT AXONAL GLYCOPROTEIN 1).
1.5e-18:201:33
HOMO SAPIENS (HUMAN).
Q02246
F-NT2RP2000533
CORNICHON PROTEIN.
5.6e-52:144:65
DROSOPHILA MELANOGASTER (FRUIT FLY).
P49858
F-NT2RP2000610
CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
8.7e-15:97:38
PETROMYZON MARINUS (SEA LAMPREY).
P25210
F-NT2RP2000616
PROCOLLAGEN ALPHA 1(I) CHAIN PRECURSOR.
0.028:163:30
MUS MUSCULUS (MOUSE).
P11087
F-NT2RP2000649
POTENTIAL CAAX PRENYL PROTEASE 1 (EC 3.4.24.-) (PRENYL PROTEIN- SPECIFIC ENDOPROTEASE 1) (PPSEP 1).
9.5e-22:241:32
SCHIZOSACCHAROMYCES POMBE (FISSION YEAST).
Q10071
F-NT2RP2000663
PANCREATIC HORMONE PRECURSOR (PANCREATIC POLYPEPTIDE) (PP).
0.71:28:46
GALLUS GALLUS (CHICKEN), AND MELEAGRIS GALLOPAVO (COMMON TURKEY).
P01306
F-NT2RP2000694
WISKOTT-ALDRICH SYNDROME PROTEIN HOMOLOG (WASP).
8. 8e-10:90:42
MUS MUSCULUS (MOUSE).
P70315
F-NT2RP2000712
ZINC FINGER PROTEIN 85 (ZINC FINGER PROTEIN HPF4) (HTF1).
2.3e-50:166:50
HOMO SAPIENS (HUMAN).
Q03923
F-NT2RP2000739
ZINC FINGER PROTEIN 85 (ZINC FINGER PROTEIN HPF4) (HTF1).
8.9e-45:180:43
HOMO SAPIENS (HUMAN).
Q03923
F-NT2RP2000818
SYG1 PROTEIN.
2.4e-14:164:35
SACCHAROMYCES CEREVISIAE (BAKER'S YEAST).
P40528
F-NT2RP2000903
SKIN SECRETORY PROTEIN XP2 PRECURSOR (APEG PROTEIN).
0.28:149:34
XENOPUS LAEVIS (AFRICAN CLAWED FROG).
P17437
F-NT2RP2001200
MIC1 PROTEIN.
1.8e-13:115:38
SACCHAROMYCES CEREVISIAE (BAKER'S YEAST).
P53258
F-NT2RP2001223
CCAAT DISPLACEMENT PROTEIN (HOMEOBOX PROTEIN CLOX) (CLOX-1) (FRAGMENT).
0.00017:92:35
CANIS FAMILIARIS (DOG).
P39881
F-NT2RP2001276
NPDC-1 PROTEIN PRECURSOR.
4.9e-35:96:71
MUS MUSCULUS (MOUSE).
Q64322
F-NT2RP2001388
CECROPIN B PRECURSOR.
0.98:31:51
DROSOPHILA MELANOGASTER (FRUIT FLY).
P14956
F-NT2RP2001469
VEGETATIBLE INCOMPATIBILITY PROTEIN HET-E-1.
6.0e-07:146:22
PODOSPORA ANSERINA.
Q00808
F-NT2RP2001480
THROMBOSPONDIN 3 PRECURSOR.
2.1e-100:209:88
HOMO SAPIENS (HUMAN).
P49746
F-NT2RP2001495
HYPOTHETICAL 53.3 KD PROTEIN IN HXT8-CAN1 INTERGENIC REGION.
3.1e-11:174:24
SACCHAROMYCES CEREVISIAE (BAKER'S YEAST).
P39981
F-NT2RP2001514
PROBABLE CALCIUM-TRANSPORTING ATPASE 6 (EC 3.6.1.38).
4.0e-18:163:36
SACCHAROMYCES CEREVISIAE (BAKER'S YEAST).
P39986
F-NT2RP2001529
DEATH-ASSOCIATED PROTEIN KINASE 1 (EG 2.7.1.-) (DAP KINASE 1).
4.4e-83:186:78
HOMO SAPIENS (HUMAN).
P53355
F-NT2RP2001538
PAIRED AMPHIPATHIC HELIX PROTEIN.
1.7e-06:152:26
SACCHAROMYCES CEREVISIAE (BAKER'S YEAST).
P22579
F-NT2RP2001562
CLATHRIN LIGHT CHAIN B (BRAIN AND LYMPHOCYTE LCB).
0.0093:124:27
HOMO SAPIENS (HUMAN).
P09497
F-NT2RP2001662
5'-TG-3' INTERACTING FACTOR (HOMEOBOX PROTEIN TGIF).
5.6e-36:146:57
HOMO SAPIENS (HUMAN).
Q15583
F-NT2RP2001755
F-SPONDIN PRECURSOR.
1.2e-33:84:89
RATTUS NORVEGICUS (RAT).
P35446
F-NT2RP2001769
PROTEIN KINASE CEK1 (EG 2.7.1.-).
1.3e-37:159:53
SCHIZOSACCHAROMYCES POMBE (FISSION YEAST).
P38938
F-NT2RP2001817
HST1 PROTEIN (HOMOLOGOUS TO SIR2 PROTEIN 1).
6.4e-32:85:48
SACCHAROMYCES CEREVISIAE (BAKER'S YEAST).
P53685
F-NT2RP2001878
EXTENSIN PRECURSOR (CELL WALL HYDROXYPROLINE-RICH GLYCOPROTEIN).
1.1e-06:173:28
NICOTIANA TABACUM (COMMON TOBACCO).
P13983
F-NT2RP2001903
CALPAIN P94, LARGE [CATALYTIC] SUBUNIT (EC 3.4.22.17) (CALCIUM- ACTIVATED NEUTRAL PROTEINASE) (CANP) (P94 PROTEIN) (MUSCLE-SPECIFIC CALCIUM-ACTIVATED NEUTRAL PROTEASE 3 LARGE SUBUNIT).
2.4e-10:110:37
HOMO SAPIENS (HUMAN).
P20807
F-NT2RP2001915
TRANS-ACTING TRANSCRIPTIONAL PROTEIN ICPO (VMW118 PROTEIN).
0.0069:74:39
HERPES SIMPLEX VIRUS (TYPE 2 / STRAIN HG52).
P28284
F-NT2RP2001921
TRANS-ACTING TRANSCRIPTIONAL PROTEIN ICPO (P135 PROTEIN) (IER 2.9/ER2.6).
0.016:51:45
BOVINE HERPESVIRUS TYPE 1 (STRAIN K22).
P29836
F-NT2RP2001948
COLLAGEN ALPHA 1(X) CHAIN PRECURSOR.
6.7e-08:121:37
HOMO SAPIENS (HUMAN).
Q03692
F-NT2RP2001956
ORM1 PROTEIN.
7. 6e-17:106:36
SACCHAROMYCES CEREVISIAE (BAKER'S YEAST).
P53224
F-NT2RP2002015
HOMEOBOX PROTEIN HOX-A2.
0.12:77:37
GALLUS GALLUS (CHICKEN).
Q08727
F-NT2RP2002063
HYPOTHETICAL 51.5 KD PROTEIN D2024.3 IN CHROMOSOME IV.
3.2e-47:213:41
CAENORHABDITIS ELEGANS.
P49191
F-NT2RP2002188
ACETYLCHOLINESTERASE PRECURSOR (EC 3.1.1.7) (ACHE).
9.2e-15:109:36
TORPEDO CALIFORNICA (PACIFIC ELECTRIC RAY).
P04058
F-NT2RP2002232
HYPOTHETICAL 85.7 KD PROTEIN C13G6.03 IN CHROMOSOME I.
2.0e-12:92:50
SCHIZOSACCHAROMYCES POMBE (FISSION YEAST).
Q09782
F-NT2RP2002304
GLYCINE-RICH CELL WALL STRUCTURAL PROTEIN (CLONE W10-1) (FRAGMENT).
0.00059:16:68
LYCOPERSICON ESCULENTUM (TOMATO).
Q01157
F-NT2RP2002409
COLLAGEN ALPHA 1(I) CHAIN (FRAGMENTS).
0.00039:184:33
BOS TAURUS (BOVINE).
P02453
F-NT2RP2002510
T-CELL RECEPTOR BETA CHAIN PRECURSOR (ANA 11).
0. 0010:97:37
ORYCTOLAGUS CUNICULUS (RABBIT).
P06333
F-NT2RP2002527
CYTOCHROME B5.
1.3e-11:77:38
SACCHAROMYCES CEREVISIAE (BAKER'S YEAST).
P40312
F-NT2RP2002533
DIHYDROPYRIDINE-SENSITIVE L-TYPE, CALCIUM CHANNEL ALPHA-2/DELTA SUBUNITS PRECURSOR.
2.0e-37:165:42
ORYCTOLAGUS CUNICULUS (RABBIT).
P13806
F-NT2RP2002564
EXTENSIN PRECURSOR (CELL WALL HYDROXYPROLINE-RICH GLYCOPROTEIN).
4.7e-06:81:35
NICOTIANA TABACUM (COMMON TOBACCO).
P13983
F-NT2RP2002674
SOLUBLE EPOXIDE HYDROLASE (SEH) (EC 3.3.2.3) (EPOXIDE HYDRATASE) (CYTOSOLIC EPOXIDE HYDROLASE) (CEH).
2.4e-25:147:41
HOMO SAPIENS (HUMAN).
P34913
F-NT2RP2002721
GLUCOSE 6-PHOSPHATE TRANSLOCASE.
0.0073:88:26
HOMO SAPIENS (HUMAN).
043826
F-NT2RP2002824
ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
1.0e-16:139:36
SACCHAROMYCES CEREVISIAE (BAKER'S YEAST).
P32802
F-NT2RP2002942
NEURAL CELL ADHESION MOLECULE L1 PRECURSOR (N-CAM L1).
5.1e-18:153:30
HOMO SAPIENS (HUMAN).
P32004
F-NT2RP2002974
HOMEOBOX PROTEIN SIX5 (DM LOCUS-ASSOCIATED HOMEODOMAIN PROTEIN HOMOLOG) (FRAGMENT).
3.6e-80:187:84
MUS MUSCULUS (MOUSE).
P70178
F-NT2RP2002976
HYPOTHETICAL 149.7 KD PROTEIN IN IRE1-KSP1 INTERGENIC REGION.
2.8e-18:99:47
SACCHAROMYCES CEREVISIAE (BAKER'S YEAST).
P38800
F-NT2RP2003042
PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).
1.2e-41:135:57
GALLUS GALLUS (CHICKEN).
P53760
F-NT2RP2003138
5'-TG-3' INTERACTING FACTOR (HOMEOBOX PROTEIN TGIF).
3.3e-09:104:45
MUS MUSCULUS (MOUSE).
P70284
F-NT2RP2003179
CARBON CATABOLITE DEREPRESSING PROTEIN KINASE (EC 2.7.1.-).
7.2e-15:96:40
SACCHAROMYCES CEREVISIAE (BAKER'S YEAST).
P06782
F-NT2RP2003210
LONG-CHAIN FATTY ACID TRANSPORT PROTEIN (FATP).
6.2e-69:235:57
MUS MUSCULUS (MOUSE).
Q60714
F-NT2RP2003302
ZINC FINGER PROTEIN 136.
9.7e-52:140:52
HOMO SAPIENS (HUMAN).
P52737
F-NT2RP2003369
SALIVARY PROLINE-RICH PROTEIN PO (ALLELE M) [CONTAINS: PEPTIDE P-D] (FRAGMENT).
0.00020:87:32
HOMO SAPIENS (HUMAN).
P10161
F-NT2RP2003383
LONG NEUROTOXIN 2 (TOXINS I AND V).
0.86:38:39
DENDROASPIS VIRIDIS (WESTERN GREEN MAMBA).
P01395
F-NT2RP2003390
NPL1 PROTEIN (SEC63 PROTEIN).
1.1e-14:113:38
SACCHAROMYCES CEREVISIAE (BAKER'S YEAST).
P14906
F-NT2RP2003469
MYO-INOSITOL TRANSPORTER 2.
1.7e-09:148:28
SACCHAROMYCES CEREVISIAE (BAKER'S YEAST).
P30606
F-NT2RP2003545
SERINE/THREONINE-PROTEIN KINASE NRK1 (EC 2.7.1.-) (N-RICH KINASE 1).
9.2e-32:198:41
SACCHAROMYCES CEREVISIAE (BAKER'S YEAST).
P38692
F-NT2RP2003593
PROLINE-RICH PROTEIN MP-2 PRECURSOR.
0.00019:128:32
MUS MUSCULUS (MOUSE).
P05142
F-NT2RP2003599
ATP-DEPENDENT BILE ACID PERMEASE.
0.88:69:34
SACCHAROMYCES CEREVISIAE (BAKER'S YEAST).
P32386
F-NT2RP2003655
HYPOTHETICAL 26.3 KD PROTEIN IN OYE2-GND1 INTERGENIC REGION.
2. 9e-16:93:47
SACCHAROMYCES CEREVISIAE (BAKER'S YEAST).
P38869
F-NT2RP2003664
HYPOTHETICAL 15.7 KD PROTEIN IN NUP85-SSC1 INTERGENIC REGION.
5.6e-08:121:32
SACCHAROMYCES CEREVISIAE (BAKER'S YEAST).
P47111
F-NT2RP2003931
ACROSIN PRECURSOR (EC 3.4.21.10).
0.38:20:70
HOMO SAPIENS (HUMAN).
P10323
F-NT2RP2003940
ZINC FINGER PROTEIN 85 (ZINC FINGER PROTEIN HPF4) (HTF1).
1.3e-84:126:74
HOMO SAPIENS (HUMAN).
Q03923
F-NT2RP2003950
TRANS-ACTING TRANSCRIPTIONAL PROTEIN ICPO (IMMEDIATE-EARLY PROTEIN IE110) (VMW110) (ALPHA-0 PROTE I N) .
1.5e-05:134:33
HERPES SIMPLEX VIRUS (TYPE 1 / STRAIN 17).
P08393
F-NT2RP2004069
HYPOTHETICAL 26.4 KD PROTEIN EEED8.8 IN CHROMOSOME II.
4.3e-13:68:54
CAENORHABDITIS ELEGANS.
Q09297
F-NT2RP2004108
ZINC FINGER PROTEIN 136.
8.6e-47:126:67
HOMO SAPIENS (HUMAN).
P52737
F-NT2RP2004141
SKIN SECRETORY PROTEIN XP2 PRECURSOR (APEG PROTEIN).
0.013:127:35
XENOPUS LAEVIS (AFRICAN CLAWED FROG).
P17437
F-NT2RP2004179
GLYCEROPHOSPHORYL DIESTER PHOSPHODIESTERASE (EC 3.1.4.46) (GLYCEROPHOSPHODIESTER PHOSPHODIESTERASE).
5.9e-10:110:36
ESCHERICHIA COLI.
P10908
F-NT2RP2004205
MYELIN-OLIGODENDROCYTE GLYCOPROTEIN PRECURSOR.
4.6e-10:99:34
HOMO SAPIENS (HUMAN).
Q16653
F-NT2RP2004447
PROCOLLAGEN ALPHA 2(I) CHAIN PRECURSOR.
0.86:48:37
MUS MUSCULUS (MOUSE).
Q01149
F-NT2RP2004495
HYPOTHETICAL 53.3 KD PROTEIN IN HXT8-CAN1 INTERGENIC REGION.
0.031:135:31
SACCHAROMYCES CEREVISIAE (BAKER'S YEAST).
P39981
F-NT2RP2004524
HYPOTHETICAL 18.7 KD PROTEIN IN HMS1-ABF2 INTERGENIC REGION.
0.042:96:23
SACCHAROMYCES CEREVISIAE (BAKER'S YEAST).
Q04767
F-NT2RP2004556
SALIVARY PROLINE-RICH PROTEIN II-1 (FRAGMENT).
0.0082:87:35
HOMO SAPIENS (HUMAN).
P81489
F-NT2RP2004606
METALLOPROTEINASE INHIBITOR 1 PRECURSOR (TIMP-1) (ERYTHROID POTENTIATING ACTIVITY) (EPA) (TISSUE INHIBITOR OF METALLOPROTEINASES) (FIBROBLAST COLLAGENASE INHIBITOR) (COLLAGENASE INHIBITOR).
2.2e-57:163:73
HOMO SAPIENS (HUMAN).
P01033
F-NT2RP2004648
BETA-GALACTOSIDASE PRECURSOR (EC 3.2.1.23) (LACTASE) (ACID BETA-GALACTOSIDASE).
3.2e-25:90:62
FELIS SILVESTRIS CATUS (CAT).
019015
F-NT2RP2004670
CALCIUM/CALMODULIN-DEPENDENT PROTEIN KINASE TYPE I (EC 2.7.1.123) (CAM KINASE I).
6. 6e-14:108:34
RATTUS NORVEGICUS (RAT).
Q63450
F-NT2RP2004794
HYPOTHETICAL 24.5 KD PROTEIN IN SAP185-BCK1 INTERGENIC REGION.
5. 7e-11:140:31
SACCHAROMYCES CEREVISIAE (BAKER'S YEAST).
P40857
F-NT2RP2004837
F-NT2RP2004847
ADULT ENHANCER FACTOR 1 (AEF-1).
7.9e-09:81:37
DROSOPHILA MELANOGASTER (FRUIT FLY).
P39413
F-NT2RP2005027
GLUCOSE TRANSPORTER TYPE 3, BRAIN.
3.6e-64:130:96
HOMO SAPIENS (HUMAN).
P11169
F-NT2RP2005069
CCAAT DISPLACEMENT PROTEIN (CDP) (CDP2) (FRAGMENT).
0.22:116:32
RATTUS NORVEGICUS (RAT).
P53565
F-NT2RP2005163
ANTER-SPECIFIC PROLINE-RICH PROTEIN APG PRECURSOR.
5.3e-06:70:38
ARABIDOPSIS THALIANA (MOUSE-EAR CRESS).
P40602
F-NT2RP2005181
HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
4.2e-54:153:69
HOMO SAPIENS (HUMAN).
P30825
F-NT2RP2005247
EXTENSIN PRECURSOR (PROLINE-RICH GLYCOPROTEIN).
2.0e-11:106:35
SORGHUM VULGARE (SORGHUM).
P24152
F-NT2RP2005378
A-AGGLUTININ ATTACHMENT SUBUNIT PRECURSOR.
0.11:97:32
SACCHAROMYCES CEREVISIAE (BAKER'S YEAST).
P32323
F-NT2RP2005391
G-BOX BINDING FACTOR (GBF).
5.1e-10:156:30
DICTYOSTELIUM DISCOIDEUM (SLIME MOLD).
P36417
F-NT2RP2005425
M PROTEIN, SEROTYPE 49 PRECURSOR.
2.1e-05:183:27
STREPTOCOCCUS PYOGENES.
P16947
F-NT2RP2005463
OVOMUCOID (FRAGMENT).
1.0:21:52
BAMBUSICOLA THORACICA (CHINESE BAMBOO-PARTRIDGE).
P52259
F-NT2RP2005514
MOBC PROTEIN.
1.0:26:53
THIOBACILLUS FERROOXIDANS.
P22899
F-NT2RP2005535
ZINC FINGER PROTEIN 85 (ZINC FINGER PROTEIN HPF4) (HTF1).
3.8e-92:243:69
HOMO SAPIENS (HUMAN).
Q03923
F-NT2RP2005541
N-ACETYLGLUCOSAMINE-6-SULFATASE PRECURSOR (EC 3.1.6.14) (G6S) (GLUCOSAMINE-6-SULFATASE).
8.8e-16:78:51
HOMO SAPIENS (HUMAN).
P15586
F-NT2RP2005597
DOLICHYL-PHOSPHATE-MANNOSE--PROTEIN MANNOSYLTRANSFERASE 4 (EC 2.4.1.109).
7.4e-13:99:34
SACCHAROMYCES CEREVISIAE (BAKER'S YEAST).
P46971
F-NT2RP2005632
ADENYLATE CYCLASE, TYPE V (EC 4.6.1.1) (ATP PYROPHOSPHATE-LYASE) (CA(2+)-INHIBITABLE ADENYLYL CYCLASE).
3.0e-05:73:43
CANIS FAMILIARIS (DOG).
P30803
F-NT2RP2005666
HEPATOCYTE NUCLEAR FACTOR 3-BETA (HNF-3B).
0.086:105:31
MUS MUSCULUS (MOUSE).
P35583
F-NT2RP2005774
ZINC FINGER PROTEIN 136.
7.8e-33:128:57
HOMO SAPIENS (HUMAN).
P52737
F-NT2RP2005878
PUTATIVE STEROID DEHYDROGENASE KIK-I (EC 1.1.1.-).
6.8e-23:96:48
MUS MUSCULUS (MOUSE).
070503
F-NT2RP2005883
DOPAMINE-BETA-MONOOXYGENASE PRECURSOR (EC 1.14.17.1) (DOPAMINE BETA-HYDROXYLASE) (DBH).
6.4e-23:185:32
RATTUS NORVEGICUS (RAT).
Q05754
F-NT2RP2005887
DNA-DIRECTED RNA POLYMERASE SUBUNIT K (EC 2.7.7.6).
1.0:40:30
METHANOCOCCUS JANNASCHII.
Q57650
F-NT2RP2005941
SKIN SECRETORY PROTEIN XP2 PRECURSOR (APEG PROTEIN).
3.5e-08:136:32
XENOPUS LAEVIS (AFRICAN CLAWED FROG).
P17437
F-NT2RP2005994
HYPOTHETICAL 51.5 KD PROTEIN D2024.3 IN CHROMOSOME IV.
4.4e-36:144:47
CAENORHABDITIS ELEGANS.
P49191
F-NT2RP2006004
BONE PROTEOGLYCAN II PRECURSOR (PG-S2) (DECORIN) (PG40) (DERMATAN SULFATE PROTEOGLYCAN-II) (DSPG).
0.030:28:50
RATTUS NORVEGICUS (RAT).
Q01129
F-NT2RP2006042
HYPOTHETICAL PROTEIN KIAA0144.
1.2e-22:228:39
HOMO SAPIENS (HUMAN).
Q14157
F-NT2RP2006092
TRANSCRIPTIONAL ACTIVATOR FE65.
3.1e-27:101:54
RATTUS NORVEGICUS (RAT).
P46933
F-NT2RP2006099
SKIN SECRETORY PROTEIN XP2 PRECURSOR (APEG PROTEIN).
7.0e-07:123:34
XENOPUS LAEVIS (AFRICAN CLAWED FROG).
P17437
F-NT2RP2006134
TRANS-ACTING TRANSCRIPTIONAL PROTEIN ICPO (EARLY PROTEIN 0) (EP0).
0.0041:118:30
PSEUDORABIES VIRUS (STRAIN INDIANA-FUNKHAUSER / BECKER) (PRV).
P29129
F-NT2RP2006269
DOLICHYL-PHOSPHATE-MANNOSE-PROTEIN MANNOSYLTRANSFERASE 4 (EC 2.4.1.109).
6.3e-17:119:36
SACCHAROMYCES CEREVISIAE (BAKER'S YEAST).
P46971
F-NT2RP2006512
GNS1 PROTEIN.
8.6e-14:186:30
SACCHAROMYCES CEREVISIAE (BAKER'S YEAST).
P25358
F-NT2RP3000011
PUTATIVE SERINE/THREONINE-PROTEIN KINASE PKWA (EC 2.7.1.-).
2.9e-12:137:32
THERMOMONOSPORA CURVATA.
P49695
F-NT2RP3000022
SERINE/THREONINE-PROTEIN KINASE MAK (EC 2.7.1.-) (MALE GERM CELL- ASSOCIATED KINASE).
1.6e-47:121:79
RATTUS NORVEGICUS (RAT).
P20793
F-NT2RP3000059
COLLAGEN ALPHA 1(III) CHAIN.
1.5e-05:211:33
BOS TAURUS (BOVINE).
P04258
F-NT2RP3000063
EXTENSIN PRECURSOR (PROLINE-RICH GLYCOPROTEIN).
4.2e-23:230:28
ZEA MAYS (MAIZE).
P14918
F-NT2RP3000125
CARBOXYPEPTIDASE KEX1 PRECURSOR (EC 3.4.16.6) (CARBOXYPEPTIDASE D).
2.3e-08:110:30
SACCHAROMYCES CEREVISIAE (BAKER'S YEAST).
P09620
F-NT2RP3000148
ZINC FINGER PROTEIN 133.
1.4e-34:84:48
HOMO SAPIENS (HUMAN).
P52736
F-NT2RP3000169
SMALL PROLINE-RICH PROTEIN 2-1.
0.00092:14:57
HOMO SAPIENS (HUMAN).
P35326
F-NT2RP3000171
24.1 KD PROTEIN IN VMA12-APN1 INTERGENIC REGION.
4.6e-10:134:32
SACCHAROMYCES CEREVISIAE (BAKER'S YEAST).
P28707
F-NT2RP3000172
CALCIUM/CALMODULIN-DEPENDENT PROTEIN KINASE TYPE I (EC 2.7.1.123) (CAM KINASE I).
1. 8e-33:161 :42
RATTUS NORVEGICUS (RAT).
Q63450
F-NT2RP3000201
SERINE/THREONINE-PROTEIN KINASE MIG-15 (EC 2.7.1.-).
4.1e-79:254:64
CAENORHABDITIS ELEGANS.
Q23356
F-NT2RP3000232
ZINC FINGER PROTEIN 184 (FRAGMENT).
8.5e-23:119:45
HOMO SAPIENS (HUMAN).
Q99676
F-NT2RP3000304
LOW-DENSITY LIPOPROTEIN RECEPTOR-RELATED PROTEIN 1 PRECURSOR (LRP) (ALPHA-2-MACROGLOBULIN RECEPTOR) (A2MR) (APOLIPOPROTEIN E RECEPTOR) (APOER) (CD91).
9.8e-36:172:43
HOMO SAPIENS (HUMAN).
Q07954
F-NT2RP3000378
PAIRED AMPHIPATHIC HELIX PROTEIN.
2.7e-26:186:36
SACCHAROMYCES CEREVISIAE (BAKER'S YEAST).
P22579
F-NT2RP3000427
5E5 ANTIGEN.
0.086:204:31
RATTUS NORVEGICUS (RAT).
Q63003
F-NT2RP3000436
PROBABLE PROTEIN DISULFIDE ISOMERASE P5 PRECURSOR (EC 5.3.4.1).
1.3e-23:106:33
CAENORHABDITIS ELEGANS.
Q11067
F-NT2RP3000444
TRANSCRIPTION INITIATION FACTOR TFIID 135 KD SUBUNIT (TAFII-135) (TAFII135) (TAFII-130) (TAFII130).
0.00052:166:36
HOMO SAPIENS (HUMAN).
000268
F-NT2RP3000460
PROTEIN TRANSPORT PROTEIN SEC61 ALPHA SUBUNIT.
1.0e-98:194:100
RATTUS NORVEGICUS (RAT).
P38378
F-NT2RP3000481
NONSENSE-MEDIATED MRNA DECAY PROTEIN 5.
7.4e-19:217:29
SACCHAROMYCES CEREVISIAE (BAKER'S YEAST).
P46970
F-NT2RP3000616
BONE PROTEOGLYCAN II PRECURSOR (PG-S2) (DECORIN).
1.2e-13:115:33
BOS TAURUS (BOVINE).
P21793
F-NT2RP3000645
SKIN SECRETORY PROTEIN XP2 PRECURSOR (APEG PROTEIN).
2.3e-10:237:30
XENOPUS LAEVIS (AFRICAN CLAWED FROG).
P17437
F-NT2RP3000652
ZINC FINGER PROTEIN 85 (ZINC FINGER PROTEIN HPF4) (HTF1).
3.1e-106:283:67
HOMO SAPIENS (HUMAN).
Q03923
F-NT2RP3000676
ADP,ATP CARRIER PROTEIN, HEART/SKELETAL MUSCLE ISOFORM T1 (ADP/ATP TRANSLOCASE 1) (ADENINE NUCLEOTIDE TRANSLOCATOR 1) (ANT 1).
7.4e-07:102:32
HOMO SAPIENS (HUMAN).
P12235
F-NT2RP3000677
MHC CLASS II REGULATORY FACTOR RFX1 (RFX) (ENHANCER FACTOR C) (EF-C).
1.5e-27:66:54
HOMO SAPIENS (HUMAN).
P22670
F-NT2RP3000721
HYPOTHETICAL 62.5 KD PROTEIN IN SEC53-ACT1 INTERGENIC REGION.
1.6e-22:208:32
SACCHAROMYCES CEREVISIAE (BAKER'S YEAST).
P43560
F-NT2RP3000789
ONCONEURAL VENTRAL ANTIGEN-1 (NOVA-1) (PARANEOPLASTIC RI ANTIGEN) (VENTRAL NEURON-SPECIFIC PROTEIN 1).
1.0e-07:190:26
HOMO SAPIENS (HUMAN).
P51513
F-NT2RP3000818
HYPOTHETICAL 67.5 KD PROTEIN IN APE1/LAP4-CWP1 INTERGENIC REGION.
5.9e-05:100:32
SACCHAROMYCES CEREVISIAE (BAKER'S YEAST).
P34248
F-NT2RP3000820
BIOTIN SYNTHASE (EC 2.8.1.6) (BIOTIN SYNTHETASE).
0.92:97:26
SYNECHOCYSTIS SP. (STRAIN PCC 6803).
P73538
F-NT2RP3000838
SKIN SECRETORY PROTEIN XP2 PRECURSOR (APEG PROTEIN).
6.4e-07:231:31
XENOPUS LAEVIS (AFRICAN CLAWED FROG).
P17437
F-NT2RP3000871
COLLAGEN ALPHA 1(I) CHAIN (FRAGMENTS).
2.8e-07:221:33
RATTUS NORVEGICUS (RAT).
P02454
F-NT2RP3000907
PROBABLE CALCIUM-TRANSPORTING ATPASE 6 (EC 3.6.1.38).
2.2e-41:104:48
SACCHAROMYCES CEREVISIAE (BAKER'S YEAST).
P39986
F-NT2RP3000921
BASEMENT MEMBRANE-SPECIFIC HEPARAN SULFATE PROTEOGLYCAN CORE PROTEIN PRECURSOR (HSPG) (PERLECAN) (PLC).
4. 5e-08:149:31
HOMO SAPIENS (HUMAN).
P98160
F-NT2RP3001012
HISTIDINE-RICH GLYCOPROTEIN PRECURSOR.
5.5e-06:37:51
PLASMODIUM LOPHURAE.
P04929
F-NT2RP3001044
RNA POLYMERASE PRINCIPAL SIGMA FACTOR HRDA.
0.10:61:31
STREPTOMYCES COELICOLOR.
P18182
F-NT2RP3001061
GLYCOPROTEIN X PRECURSOR.
0.00011:140:27
EQUINE HERPESVIRUS TYPE 1 (STRAIN AB4P) (EHV-1).
P28968
F-NT2RP3001159
A-AGGLUTININ ATTACHMENT SUBUNIT PRECURSOR.
1.1e-09:249:32
SACCHAROMYCES CEREVISIAE (BAKER'S YEAST).
P32323
F-NT2RP3001170
POU DOMAIN PROTEIN 1 (DJPOU1).
0.020:173:29
DUGESIA JAPONICA (PLANARIAN).
P31370
F-NT2RP3001195
GALACTOSE-PROTON SYMPORT (GALACTOSE TRANSPORTER).
1.2e-14:180:30
ESCHERICHIA COLI.
P37021
F-NT2RP3001240
PROTEIN TRANSPORT PROTEIN SEC61 ALPHA SUBUNIT.
3.1e-118:229:88
RATTUS NORVEGICUS (RAT).
P38378
F-NT2RP3001271
EBNA-1 NUCLEAR PROTEIN.
2.3e-08:113:45
EPSTEIN-BARR VIRUS (STRAIN B95-8) (HUMAN HERPESVIRUS 4). P03211
F-NT2RP3001322
PROBABLE CALCIUM-TRANSPORTING ATPASE 3 (EC 3.6.1.38) (ENDOPLASMIC RETICULUM CA2+-ATPASE).
1.7e-23:222:29
SACCHAROMYCES. CEREVISIAE (BAKER'S YEAST).
P39524
F-NT2RP3001388
SYNAPTOTAGMIN IV.
4.8e-19:168:38
RATTUS NORVEGICUS (RAT).
P50232
F-NT2RP3001542
TUMOR NECROSIS FACTOR, ALPHA-INDUCED PROTEIN 1, ENDOTHELIAL (B12 PROTEIN).
2. 7e-12:132:37
HOMO SAPIENS (HUMAN).
Q13829
F-NT2RP3001560
SYNAPSINS IA AND IB.
0.59:104:35
BOS TAURUS (BOVINE).
P17599
F-NT2RP3001592
GLYCINE-RICH CELL WALL STRUCTURAL PROTEIN 2 PRECURSOR.
1.3e-11:75:46
ORYZA SATIVA (RICE).
P29834
F-NT2RP3001650
CCAAT DISPLACEMENT PROTEIN (HOMEOBOX PROTEIN CLOX) (CLOX-1) (FRAGMENT).
0.23:119:36
CANIS FAMILIARIS (DOG).
P39881
F-NT2RP3001685
HYPOTHETICAL PROTEIN IN MMSB 3'REGION (ORF1) (FRAGMENT).
2.2e-48:207:48
PSEUDOMONAS AERUGINOSA.
P28812
F-NT2RP3001738
CYTOCHROME B5.
9.5e-13:133:33
ORYCTOLAGUS CUNICULUS (RABBIT).
P00169
F-NT2RP3001754
SYNAPSINS IA AND IB (BRAIN PROTEIN 4.1).
7.9e-05:117:29
HOMO SAPIENS (HUMAN).
P17600
F-NT2RP3001858
CUTICLE COLLAGEN 2.
0.030:118:35
CAENORHABDITIS ELEGANS.
P17656
F-NT2RP3001976
ZINC FINGER PROTEIN 140.
7.8e-24:122:52
HOMO SAPIENS (HUMAN).
P52738
F-NT2RP3002015
PROCOLLAGEN ALPHA 1(I) CHAIN PRECURSOR.
0.018:224:30
GALLUS GALLUS (CHICKEN).
P02457
F-NT2RP3002160
SKIN SECRETORY PROTEIN XP2 PRECURSOR (APEG PROTEIN).
0.0058:206:29
XENOPUS LAEVIS (AFRICAN CLAWED FROG).
P17437
F-NT2RP3002281
HETEROGENEOUS NUCLEAR RIBONUCLEOPROTEIN F (HNRNP F).
1.3e-14:86:40
HOMO SAPIENS (HUMAN).
P52597
F-NT2RP3002286
ETS-DOMAIN TRANSCRIPTION FACTOR ERF.
0.65:128:29
HOMO SAPIENS (HUMAN).
P50548
F-NT2RP3002311
BETA-GALACTOSIDASE PRECURSOR (EC 3.2.1.23) (LACTASE) (ACID BETA-GALACTOSIDASE).
6.1e-46:172:54
FELIS SILVESTRIS CATUS (CAT).
019015
F-NT2RP3002324
HYPOTHETICAL 13.6 KD PROTEIN IN SPT4-ROM1 INTERGENIC REGION.
0. 012:23:65
SACCHAROMYCES CEREVISIAE (BAKER'S YEAST).
P53245
F-NT2RP3002342
HYPOTHETICAL 53.3 KD PROTEIN IN HXT8-CAN1 INTERGENIC REGION.
1. 8e-13:219:26
SACCHAROMYCES CEREVISIAE (BAKER'S YEAST).
P39981
F-NT2RP3002353
REGULATORY PROTEIN E2.
0.0027:167:31
HUMAN PAPILLOMAVIRUS TYPE 8.
P06422
F-NT2RP3002409
MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
9.9e-09:93:34
NEUROSPORA CRASSA.
P23231
F-NT2RP3002411
PUTATIVE STEROID DEHYDROGENASE KIK-I (EC 1.1.1.-).
5.6e-107:254:80
MUS MUSCULUS (MOUSE).
070503
F-NT2RP3002448
TRANSCRIPTION INITIATION FACTOR TFIID 135 KD SUBUNIT (TAFII-135) (TAFII135) (TAFII-130) (TAFII130).
1.5e-05:163:33
HOMO SAPIENS (HUMAN).
000268
F-NT2RP3002571
HYPOTHETICAL 116.3 KD PROTEIN C26F1.09 IN CHROMOSOME I.
6.4e-23:172:33
SCHIZOSACCHAROMYCES POMBE (FISSION YEAST).
Q10496
F-NT2RP3002664
CYCLIC-AMP-DEPENDENT TRANSCRIPTION FACTOR ATF-6 (FRAGMENT).
0.062:47:29
HOMO SAPIENS (HUMAN).
P18850
F-NT2RP3002721
CITRATE SYNTHASE, MITOCHONDRIAL PRECURSOR (EC 4.1.3.7).
6.2e-140:283:92
SUS SCROFA (PIG).
P00889
F-NT2RP3002737
VOLTAGE-GATED POTASSIUM CHANNEL PROTEIN KV1.9.
4.1e-40:136:61
MUS MUSCULUS (MOUSE).
P97414
F-NT2RP3002738
SALIVARY PROLINE-RICH PROTEIN PO PRECURSOR (ALLELE S).
0.029:195:28
HOMO SAPIENS (HUMAN).
P10163
F-NT2RP3002790
PROLINE-RICH PROTEIN MP-2 PRECURSOR.
1.7e-08:130:36
MUS MUSCULUS (MOUSE).
P05142
F-NT2RP3002836
TRANSMEMBRANE PROTEIN SEX PRECURSOR.
8.9e-24:119:43
HOMO SAPIENS (HUMAN).
P51805
F-NT2RP3002887
SALIVARY PROLINE-RICH PROTEIN PRECURSOR (CLONE CP7) [CONTAINS: BASIC PEPTIDE P-F] (FRAGMENT).
2.9e-11:198:34
HOMO SAPIENS (HUMAN).
P02812
F-NT2RP3002900
COP-COATED VESICLE MEMBRANE PROTEIN P24 PRECURSOR (FRAGMENT).
2.8e-18:109:41
CRICETULUS GRISEUS (CHINESE HAMSTER).
P49020
F-NT2RP3002958
TRANS-GOLGI NETWORK INTEGRAL MEMBRANE PROTEIN TGN38 PRECURSOR.
6.4e-06:172:27
RATTUS NORVEGICUS (RAT).
P19814
F-NT2RP3002983
COLLAGEN ALPHA 1(I) CHAIN (FRAGMENTS).
4.4e-05:106:41
BOS TAURUS (BOVINE).
P02453
F-NT2RP3003000
SODIUM CHANNEL PROTEIN (NA+ CHANNEL).
9.7e-30:221:31
ELECTROPHORUS ELECTRICUS (ELECTRIC EEL).
P02719
F-NT2RP3003076
ENVELOPE GLYCOPROTEIN GP340 (MEMBRANE ANTIGEN) (MA) [CONTAINS: GLYCOPROTEIN GP220].
0.00033:173:30
EPSTEIN-BARR VIRUS (STRAIN B95-8) (HUMAN HERPESVIRUS 4).
P03200
F-NT2RP3003354
SECRETORY CARRIER-ASSOCIATED MEMBRANE PROTEIN 3.
2.0e-54:204:51
MUS MUSCULUS (MOUSE).
035609
F-NT2RP3003448
PROTEASE DEGS PRECURSOR (EC 3.4.21.-).
4.0e-05:112:33
ESCHERICHIA COLI.
P31137
F-NT2RP3003469
!!!! ALU SUBFAMILY SQ WARNING ENTRY !!!!
1.2e-17:70:64
HOMO SAPIENS (HUMAN).
P39194
F-NT2RP3003473
BACTENECIN 7 PRECURSOR (BAC7) (PR-59).
0.0037:33:63
BOS TAURUS (BOVINE).
P19661
F-NT2RP3003527
SERINE/THREONINE-SPECIFIC PROTEIN KINASE MINIBRAIN HOMOLOG (EC 2.7.1.-) (HP86) (DYRK).
1.8e-53:159:69
HOMO SAPIENS (HUMAN).
Q13627
F-NT2RP3003532
OX-2 MEMBRANE GLYCOPROTEIN PRECURSOR (FRAGMENT).
2.3e-114:219:97
HOMO SAPIENS (HUMAN).
P41217
F-NT2RP3003535
HYPOTHETICAL 7.2 KD PROTEIN IN RPS2 3'REGION (ORF57).
0.98:36:30
ASTASIA LONGA (EUGLENOPHYCEAN ALGA).
P34774
F-NT2RP3003559
MALE SPECIFIC SPERM PROTEIN MST84DB.
0.047:29:48
DROSOPHILA MELANOGASTER (FRUIT FLY).
Q01643
F-NT2RP3003614
TRYPSIN INHIBITOR 11 (BDTI-II).
0.98:23:39
BRYONIA DIOICA (RED BRYONY).
P11968
F-NT2RP3003729
HYPOTHETICAL 42.1 KD PROTEIN IN SNZ1-YPK2 INTERGENIC REGION.
4.1e-11:204:30
SACCHAROMYCES CEREVISIAE (BAKER'S YEAST).
Q03151
F-NT2RP3003849
PROTEIN KINASE C, BRAIN ISOZYME (EG 2.7.1.-) (PKC) (DPKC53E(BR)).
9.7e-17:126:34
DROSOPHILA MELANOGASTER (FRUIT FLY).
P05130.
F-NT2RP3003874
MYOSIN I ALPHA (MMI-ALPHA).
3.1e-64:141:84
MUS MUSCULUS (MOUSE).
P46735
F-NT2RP3003939
CELL DIVISION PROTEIN FTSH HOMOLOG 4 (EC 3.4.24.-).
7.1e-34:76:61
SYNECHOCYSTIS SP. (STRAIN PCC 6803).
P72991
F-NT2RP3003963
HISTIDINE-RICH, METAL BINDING POLYPEPTIDE.
0.95:31:38
HELICOBACTER PYLORI (CAMPYLOBACTER PYLORI).
Q48251
F-NT2RP3004000
DNA-DIRECTED RNA POLYMERASE II LARGEST SUBUNIT (EC 2.7.7.6) (RPB1) (FRAGMENT).
7.1e-07:187:29
CRICETULUS GRISEUS (CHINESE HAMSTER).
P11414
F-NT2RP3004025
EBNA-1 NUCLEAR PROTEIN.
0.022:79:40
EPSTEIN-BARR VIRUS (STRAIN B95-8) (HUMAN HERPESVIRUS 4).
P03211
F-NT2RP3004067
COLLAGEN ALPHA 1(X) CHAIN PRECURSOR.
5.0e-07:184:35
HOMO SAPIENS (HUMAN).
Q03692
F-NT2RP3004075
GLYCINE-RICH CELL WALL STRUCTURAL PROTEIN PRECURSOR.
2.9e-07:92:40
HORDEUM VULGARE (BARLEY).
P17816
F-NT2RP3004083
ATP SYNTHASE PROTEIN 8 (EC 3.6.1.34) (A6L).
0.013:24:45
COTURNIX COTURNIX JAPONICA (JAPANESE QUAIL).
P50682
F-NT2RP3004090
SALIVARY GLUE PROTEIN SGS-3 PRECURSOR.
1.2e-07:195:29
DROSOPHILA ERECTA (FRUIT FLY).
P13730
F-NT2RP3004119
PEREGRIN (BR140 PROTEIN).
4.1e-40:227:43
HOMO SAPIENS (HUMAN).
P55201
F-NT2RP3004130
CELL SURFACE ANTIGEN 114/A10 PRECURSOR.
2.4e-08:71:42
MUS MUSCULUS (MOUSE).
P19467
F-NT2RP3004133
GLYCOSYLTRANSFERASE ALG2 (EC 2.4.1.-).
1.5e-28:111:44
SACCHAROMYCES CEREVISIAE (BAKER'S YEAST).
P43636
F-NT2RP3004202
PROLINE-RICH PROTEIN MP-2 PRECURSOR.
3.0e-06:104:37
MUS MUSCULUS (MOUSE).
P05142
F-NT2RP3004294
HYPOTHETICAL 22.2 KD PROTEIN IN NSR1-TIF4631 INTERGENIC REGION.
8. 8e-10:129:31
SACCHAROMYCES CEREVISIAE (BAKER'S YEAST).
P53288
F-NT2RP3004309
A-AGGLUTININ ATTACHMENT SUBUNIT PRECURSOR.
1.9e-05:212:30
SACCHAROMYCES CEREVISIAE (BAKER'S YEAST).
P32323
F-NT2RP3004321
REGULATORY PROTEIN SIR2 HOMOLOG (LMSIR2RP).
2.8e-09:81:40
LEISHMANIA MAJOR.
Q25337
F-NT2RP3004345
GLYCINE-RICH CELL WALL STRUCTURAL PROTEIN 2 PRECURSOR.
1.3e-11:75:46
ORYZA SATIVA (RICE).
P29834
F-NT2RP3004355
HYDROGENASE EXPRESSION/FORMATION PROTEIN HUPV.
0.81:154:26
AZOTOBACTER CHROOCOCCUM MCD 1.
Q43959
F-NT2RP3004374
HOMEOBOX PROTEIN HOX-A2.
0.28:77:37
GALLUS GALLUS (CHICKEN).
Q08727
F-NT2RP3004406
HYPOTHETICAL 37.4 KD PROTEIN IN IRR1-TIM44 INTERGENIC REGION.
4.9e-18:165:33
SACCHAROMYCES CEREVISIAE (BAKER'S YEAST).
P40544
F-NT2RP3004481
BUTYROPHILIN PRECURSOR (BT).
4.0e-13:152:31
HOMO SAPIENS (HUMAN).
Q13410
F-NT2RP3004552
COMPLEMENT RECEPTOR TYPE 1 PRECURSOR (C3B/C4B RECEPTOR) (CD35 ANTIGEN).
3.4e-05:211:28
HOMO SAPIENS (HUMAN).
P17927
F-NT2RP3004557
INTERFERON GAMMA UP-REGULATED I-5111 PROTEIN PRECURSOR (IGUP I-5111).
1.6e-23:129:35
HOMO SAPIENS (HUMAN).
Q06323
F-NT2RP3004625
GLYCOPROTEIN X PRECURSOR.
2.4e-10:225:25
EQUINE HERPESVIRUS TYPE 1 (STRAIN AB4P) (EHV-1).
P28968
F-NT2RP3004640
ENAMELIN (TUFTELIN).
2.6e-70:167:85
BOS TAURUS (BOVINE).
P27628
F-NT2RP3004647
ADP,ATP CARRIER PROTEIN, HEART/SKELETAL MUSCLE ISOFORM T1 (ADP/ATP TRANSLOCASE 1) (ADENINE NUCLEOTIDE TRANSLOCATOR 1) (ANT 1).
4.6e-10:116:34
HOMO SAPIENS (HUMAN).
P12235
F-NT2RP4000108
NEUROFILAMENT TRIPLET L PROTEIN (68 KD NEUROFILAMENT PROTEIN) (NF-L) (NF68).
3.4e-107:255:87
RATTUS NORVEGICUS (RAT).
P19527
F-NT2RP4000634
MAPK/ERK KINASE KINASE 2 (EC 2.7.1.-) (MEK KINASE 2) (MEKK 2).
7.9e-142:267:88
MUS MUSCULUS (MOUSE).
Q61083
F-NT2RP4000962
SPORULATION-SPECIFIC PROTEIN 1 (EC 2.7.1.-).
1.5e-13:158:31
SACCHAROMYCES CEREVISIAE (BAKER'S YEAST).
P08458
F-NT2RP4001001
F-NT2RP4001009
POTENTIAL CAAX PRENYL PROTEASE 1 (EC 3.4.24.-) (PRENYL PROTEIN- SPECIFIC ENDOPROTEASE 1) (PPSEP 1).
7.7e-24:235:31
SCHIZOSACCHAROMYCES POMBE (FISSION YEAST).
Q10071
F-NT2RP4001467
5'-NUCLEOTIDASE PRECURSOR (EC 3.1.3.5) (ECTO-NUCLEOTIDASE) (5'-NT) (CD73 ANTIGEN).
1.2e-120:237:97
HOMO SAPIENS (HUMAN).
P21589
F-NT2RP4001877
GLYCINE-RICH RNA-BINDING PROTEIN.
1.4e-08:89:34
DAUCUS CAROTA (CARROT).
Q03878
F-NT2RP4001879
VERY HYPOTHETICAL 8.9 KD PROTEIN CY441.05 PRECURSOR.
0.98:49:34
MYCOBACTERIUM TUBERCULOSIS.
P71934
F-NT2RP4002187
PUTATIVE STEROID DEHYDROGENASE KIK-I (EC 1.1.1.-).
4.5e-98:246:78
MUS MUSCULUS (MOUSE).
070503
F-NT2RP4002451
CUTICLE COLLAGEN 2.
0.85:92:35
CAENORHABDITIS ELEGANS.
P17656
F-NT2RP4002715
HYPOTHETICAL 13.6 KD PROTEIN IN SPT4-ROM1 INTERGENIC REGION.
0.47:31:48
SACCHAROMYCES CEREVISIAE (BAKER'S YEAST).
P53245
F-NT2RP4002750
HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
3.3e-63:185:67
HOMO SAPIENS (HUMAN).
P30825
F-OVARC1000003
RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
2.2e-82:197:72
HOMO SAPIENS (HUMAN).
Q06495
F-OVARC1000090
HOMEOBOX PROTEIN HOX-B1 (GHOX-LAB).
0.049:120:32
GALLUS GALLUS (CHICKEN).
P31259
F-OVARC1000105
UBIQUITIN-CONJUGATING ENZYME E2-28.4 KD (EC 6.3.2.19) (UBIQUITIN- PROTEIN LIGASE) (UBIQUITIN CARRIER PROTEIN).
8.6e-47:159:58
SACCHAROMYCES CEREVISIAE (BAKER'S YEAST).
P33296
F-OVARC1000137
HYPOTHETICAL 22.7 KD PROTEIN IN PAS1-MST1 INTERGENIC REGION.
0.058:28:64
SACCHAROMYCES CEREVISIAE (BAKER'S YEAST).
P36015
F-OVARC1000208
!!!! ALU SUBFAMILY SX WARNING ENTRY !!!!
2.2e-12:51:74
HOMO SAPIENS (HUMAN).
P39195
F-OVARC1000255
TYROSINE-PROTEIN KINASE SYK (EC 2.7.1.112) (SPLEEN TYROSINE KINASE).
1.1e-112:144:86
HOMO SAPIENS (HUMAN).
P43405
F-OVARC1000275
GASTRIN PRECURSOR.
0.11:59:37
HOMO SAPIENS (HUMAN).
P01350
F-OVARC1000298
PROLINE-RICH PROTEIN MP-3 (FRAGMENT).
0.014:74:39
MUS MUSCULUS (MOUSE).
P05143
F-OVARC1000307
EXTENSIN PRECURSOR (CELL WALL HYDROXYPROLINE-RICH GLYCOPROTEIN).
1.0:33:54
NICOTIANA TABACUM (COMMON TOBACCO).
P13983
F-OVARC1000313
PROTEIN DISULFIDE ISOMERASE-RELATED PROTEIN PRECURSOR (ERP72) (CALCIUM-BINDING PROTEIN 2) (CABP2).
4.0e-15:127:37
RATTUS NORVEGICUS (RAT).
P38659
F-OVARC1000331
GMP REDUCTASE (EC 1.6.6.8) (GUANOSINE 5'-MONOPHOSPHATE OXIDOREDUCTASE).
2.0e-24:64:84
HOMO SAPIENS (HUMAN).
P36959
F-OVARC1000410
FIBRINOGEN-LIKE PROTEIN A PRECURSOR (FREP-A).
1.9e-44:229:41
PARASTICHOPUS PARVIMENSIS (SEA CUCUMBER).
P19477
F-OVARC1000439
SALIVARY GLUE PROTEIN SGS-7 PRECURSOR.
0.99:41:43
DROSOPHILA MELANOGASTER (FRUIT FLY).
P02841
F-OVARC1000467
GLYCINE-RICH CELL WALL STRUCTURAL PROTEIN (CLONE W10-1) (FRAGMENT).
0.0061:30:63
LYCOPERSICON ESCULENTUM (TOMATO).
Q01157
F-OVARC1000529
PROBABLE SERINE/THREONINE-PROTEIN KINASE YKL101W (EC 2.7.1.-).
1.5e-20:127:42
SACCHAROMYCES CEREVISIAE (BAKER'S YEAST).
P34244
F-OVARC1000553
DIPEPTIDYL PEPTIDASE IV (EG 3.4.14.5) (DPP IV) (THYMOCYTE-ACTIVATING MOLECULE) (THAM).
7.6e-26:169:40
MUS MUSCULUS (MOUSE).
P28843
F-OVARC1000775
METALLOTHIONEIN (MT).
0.91:31:38
CARASSIUS AURATUS (GOLDFISH).
P52723
F-OVARC1000811
COAGULATION FACTOR XII PRECURSOR (EC 3.4.21.38) (HAGEMAN FACTOR) (HAF).
2.8e-11:69:43
HOMO SAPIENS (HUMAN).
P00748
F-OVARC1000853
CUTICLE COLLAGEN 40.
0. 00013:130:33
CAENORHABDITIS ELEGANS.
P34804 ,
F-OVARC1000873
MALE SPECIFIC SPERM PROTEIN MST84DB.
0.00015:53:33
DROSOPHILA MELANOGASTER (FRUIT FLY).
Q01643
F-OVARC1000916
GALACTOSYLTRANSFERASE ASSOCIATED PROTEIN KINASE P58/GTA (EC 2.7.1.-).
2.5e-26:109:53
MUS MUSCULUS (MOUSE).
P24788
F-OVARC1000956
D(4) DOPAMINE RECEPTOR (D(2C) DOPAMINE RECEPTOR).
0.00073:115:33
HOMO SAPIENS (HUMAN).
P21917
F-OVARC1000995
POSSIBLE GLOBAL TRANSCRIPTION ACTIVATOR SNF2L2 (SNF2-ALPHA).
0.00031:139:25
HOMO SAPIENS (HUMAN).
P51531
F-OVARC1001030
5E5 ANTIGEN.
1.9e-09:89:41
RATTUS NORVEGICUS (RAT).
Q63003
F-OVARC1001049
PROCOLLAGEN ALPHA 1(I) CHAIN PRECURSOR.
1.5e-08:146:38
GALLUS GALLUS (CHICKEN).
P02457
F-OVARC1001086
VITELLOGENIN II PRECURSOR (MAJOR VITELLOGENIN) [CONTAINS: LIPOVITELLIN I (LVI); PHOSVITIN (PV); LIPOVITELLIN II (LVII); YGP40].
5.3e-08:182:32
GALLUS GALLUS (CHICKEN).
P02845
F-OVARC1001132
GC-RICH SEQUENCE DNA-BINDING FACTOR (GCF) (TRANSCRIPTION FACTOR 9) (TCF-9).
9.2e-40:229:37
HOMO SAPIENS (HUMAN).
P16383
F-OVARC1001163
HYPOTHETICAL 49.3 KD PROTEIN C30D11.06C IN CHROMOSOME I.
8.8e-05:38:44
SCHIZOSACCHAROMYCES POMBE (FISSION YEAST).
Q09906
F-OVARC1001222
AMELOGENIN, CLASS I PRECURSOR.
0.72:96:31
BOS TAURUS (BOVINE).
P02817
F-OVARC1001260
F-OVARC1001336
RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
1.1e-33:103:71
RATTUS NORVEGICUS (RAT).
Q06496
F-OVARC1001338
SERINE/THREONINE-PROTEIN KINASE UNC-51 (EG 2.7.1.-).
3.8e-30:89:46
CAENORHABDITIS ELEGANS.
Q23023
F-OVARC1001569
ACROSIN PRECURSOR (EC 3.4.21.10) (53 KD FUCOSE-BINDING PROTEIN).
2.2e-06:28:64
SUS SCROFA (PIG).
P08001
F-OVARC1001570
CATHEPSIN E PRECURSOR (EG 3.4.23.34).
1.8e-09:121:33
CAVIA PORCELLUS (GUINEA PIG).
P25796
F-OVARC1001596
REGULATORY PROTEIN E2.
0.33:77:37
HUMAN PAPILLOMAVIRUS TYPE 14.
P36783
F-OVARC1001607
ALPHA-1,6-MANNOSYL-GLYCOPROTEIN BETA-1,2-N- ACETYLGLUCOSAMINYLTRANSFERASE (EC 2.4.1.143) (N-GLYCOSYL- OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II).
1.0e-28:69:84
HOMO SAPIENS (HUMAN).
Q10469
F-OVARC1001725
F-OVARC1001727
F-OVARC1001807
EARLY RESPONSE PROTEIN NAK1 (TR3 ORPHAN RECEPTOR).
2.4e-51:153:75
HOMO SAPIENS (HUMAN).
P22736
F-OVARC1001833
CIS-GOLGI MATRIX PROTEIN GM130.
1. 2e-55:169 : 75
RATTUS NORVEGICUS (RAT).
Q62839
F-OVARC1001952
EBNA-1 NUCLEAR PROTEIN.
3.5e-19:130:43
EPSTEIN-BARR VIRUS (STRAIN B95-8) (HUMAN HERPESVIRUS 4).
P03211
F-OVARC1001991
HYPOTHETICAL PROTEIN KIAA0176 (FRAGMENT).
3.7e-16:141:43
HOMO SAPIENS (HUMAN).
Q14681
F-OVARC1002058
LAMININ ALPHA-5 CHAIN (FRAGMENT).
2.8e-22:163:33
MUS MUSCULUS (MOUSE).
Q61001
F-OVARC1002178
TRANS-ACTING TRANSCRIPTIONAL PROTEIN ICPO (IMMEDIATE-EARLY PROTEIN IE110) (VMW110) (ALPHA-0 PROTEIN).
0.12:73:36
HERPES SIMPLEX VIRUS (TYPE 1 / STRAIN 17).
P08393
F-PLACE1000033
VON WILLEBRAND FACTOR PRECURSOR.
1.7e-19:190:28
CANIS FAMILIARIS (DOG).
Q28295
F-PLACE1000231
DNA-BINDING PROTEIN MNB1A.
0.24:60:33
ZEA MAYS (MAIZE).
P38564
F-PLACE1000258
ZINC FINGER PROTEIN 177.
3.6e-19:55:61
HOMO SAPIENS (HUMAN).
Q13360
F-PLACE1000442
ZINC FINGER PROTEIN 136.
1.7e-80:180:72
HOMO SAPIENS (HUMAN).
P52737
F-PLACE1000560
COLICIN E9 (EC 3.1.21.1).
0.015:47:44
ESCHERICHIA COLI.
P09883
F-PLACE1000740
NEUROGENIC LOCUS NOTCH HOMOLOG PROTEIN 4 PRECURSOR (TRANSFORMING PROTEIN INT-3).
1.6e-05:75:36
MUS MUSCULUS (MOUSE).
P31695
F-PLACE1000907
ZINC FINGER PROTEIN 141.
2.8e-15:43:88
HOMO SAPIENS (HUMAN).
Q15928
F-PLACE1000912
PROBABLE E4 PROTEIN (E1^E4).
0.19:46:36
HUMAN PAPILLOMAVIRUS TYPE 6B.
P06459
F-PLACE1000914
MALE SPECIFIC SPERM PROTEIN MST87F.
0.054:27:44
DROSOPHILA MELANOGASTER (FRUIT FLY).
P08175
F-PLACE1000927
HYPOTHETICAL PROTEIN H10044.
3.9e-07:139:30
HAEMOPHILUS INFLUENZAE.
P44477
F-PLACE1000986
F-PLACE1001016
SODIUM CHANNEL PROTEIN, BRAIN II ALPHA SUBUNIT.
2.7e-05:120:32
RATTUS NORVEGICUS (RAT).
P04775
F-PLACE1001100
F-PLACE1001114
PROCOLLAGEN ALPHA 1(I) CHAIN PRECURSOR.
2.5e-07:250:28
MUS MUSCULUS (MOUSE).
P11087
F-PLACE1001123
INTESTINAL MEMBRANE A4 PROTEIN (DIFFERENTIATION-DEPENDENT PROTEIN A4).
6.2e-09:95:31
HOMO SAPIENS (HUMAN).
Q04941
F-PLACE1001183
NONHISTONE CHROMOSOMAL PROTEIN HMG-17.
0.31:52:34
GALLUS GALLUS (CHICKEN).
P02314
F-PLACE1001229
NADH-UBIQUINONE OXIDOREDUCTASE CHAIN 5 (EG 1.6.5.3) (FRAGMENT).
1.0:38:47
ARTEMIA SALINA (BRINE SHRIMP).
P19047
F-PLACE1001231
SODIUM/GLUCOSE COTRANSPORTER 1 (NA(+)/GLUCOSE COTRANSPORTER 1) (HIGH AFFINITY SODIUM-GLUCOSE COTRANSPORTER).
4.7e-06:181:27
ORYCTOLAGUS CUNICULUS (RABBIT).
P11170
F-PLACE1001340
MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
6.5e-14:136:29
NEUROSPORA CRASSA.
P23231
F-PLACE1001401
HIGH AFFINITY IMMUNOGLOBULIN EPSILON RECEPTOR BETA-SUBUNIT (FCERI) (IGE FC RECEPTOR, BETA-SUBUNIT).
1.3e-11:103:40
RATTUS NORVEGICUS (RAT).
P13386
F-PLACE1001407
INTEGUMENTARY MUCIN C.1 (FIM-C.1) (FRAGMENT).
0.013:121:32
XENOPUS LAEVIS (AFRICAN CLAWED FROG).
Q05049
F-PLACE1001464
5'-NUCLEOTIDASE PRECURSOR (EG 3.1.3.5) (ECTO-NUCLEOTIDASE) (5'-NT) (CD73 ANTIGEN).
1.4e-119:246:89
HOMO SAPIENS (HUMAN).
P21589
F-PLACE1001500
BLOOM'S SYNDROME PROTEIN.
8.3e-26:203:34
HOMO SAPIENS (HUMAN).
P54132
F-PLACE1001516
GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).
7.4e-07:204:30
SACCHAROMYCES CEREVISIAE (BAKER'S YEAST).
P08640
F-PLACE1001536
F-PLACE1001564
LEUCOCYTE ANTIGEN CD97 PRECURSOR.
2.1e-09:170:24
HOMO SAPIENS (HUMAN).
P48960
F-PLACE1001655
VOLTAGE-GATED POTASSIUM CHANNEL PROTEIN KV2.1 (DRK1).
4.0e-34:189:39
RATTUS NORVEGICUS (RAT).
P15387
F-PLACE1001788
HYPOTHETICAL 36.7 KD PROTEIN C2F7.02C IN CHROMOSOME I.
6.2e-21:75:58
SCHIZOSACCHAROMYCES POMBE (FISSION YEAST).
Q09695
F-PLACE1001795
HYPOTHETICAL 30.6 KD PROTEIN IN SCP160-SMC3 INTERGENIC REGION PRECURSOR.
3.8e-21:159:40
SACCHAROMYCES CEREVISIAE (BAKER'S YEAST).
P47032
F-PLACE1001836
ENV POLYPROTEIN PRECURSOR (COAT POLYPROTEIN) [CONTAINS: OUTER MEMBRANE PROTEIN GP70; TRANSMEMBRANE PROTEIN P20E].
4.5e-29:134:47
BABOON ENDOGENOUS VIRUS (STRAIN M7).
P10269
F-PLACE1001918
ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
1.5e-30:228:32
SACCHAROMYCES CEREVISIAE (BAKER'S YEAST).
P32802
F-PLACE1001949
PROBABLE CALCIUM-TRANSPORTING ATPASE 9 (EC 3.6.1.38).
5.1e-36:210:46
SACCHAROMYCES CEREVISIAE (BAKER'S YEAST).
Q12697
F-PLACE1002080
HYPOTHETICAL PROTEIN KIAA0288 (HA6116).
3.5e-26:207:45
HOMO SAPIENS (HUMAN).
P56524
F-PLACE1002095
N-ACETYLLACTOSAMINE SYNTHASE (EG 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
0.32:50:34
MUS MUSCULUS (MOUSE).
P15535
F-PLACE1002153
CELL SURFACE GLYCOPROTEIN 1 PRECURSOR (OUTER LAYER PROTEIN B) (S-LAYER PROTEIN 1).
0.00021:214:26
CLOSTRIDIUM THERMOCELLUM.
Q06852
F-PLACE1002329
EPIDERMAL GROWTH FACTOR RECEPTOR KINASE SUBSTRATE EPS8.
1.1e-35:179:44
MUS MUSCULUS (MOUSE).
Q08509
F-PLACE1002355
COMPLEMENT C4 PRECURSOR [CONTAINS: C4A ANAPHYLATOXIN] (FRAGMENTS).
1.0e-14:183:32
BOS TAURUS (BOVINE).
P01030
F-PLACE1002374
CATHEPSIN L PRECURSOR (EC 3.4.22.15) (MAJOR EXCRETED PROTEIN) (MEP).
9.2e-107:225:86
HOMO SAPIENS (HUMAN).
P07711
F-PLACE1002518
HYPOTHETICAL 13.2 KD PROTEIN IN ORC2-TIP1 INTERGENIC REGION.
6.1e-05:59:44
SACCHAROMYCES CEREVISIAE (BAKER'S YEAST).
P38239
F-PLACE1002547
MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
1.0e-22:230:31
NEUROSPORA CRASSA.
P23231
F-PLACE1002726
COLLAGEN ALPHA 1(I) CHAIN (FRAGMENT).
0.61:25:48
ORYCTOLAGUS CUNICULUS (RABBIT).
P02456
F-PLACE1002905
ENDOZEPINE-RELATED PROTEIN PRECURSOR (MEMBRANE-ASSOCIATED DIAZEPAM BINDING INHIBITOR) (MA-DBI).
5.0e-31:93:64
BOS TAURUS (BOVINE).
P07106
F-PLACE1002911
T-CELL SURFACE PROTEIN TACTILE PRECURSOR (CD96 ANTIGEN).
6.6e-06:95:35
HOMO SAPIENS (HUMAN).
P40200
F-PLACE1002967
HIGH AFFINITY IMMUNOGLOBULIN EPSILON RECEPTOR BETA-SUBUNIT (FCERI) (IGE FC RECEPTOR, BETA-SUBUNIT).
9.4e-08:95:37
MUS MUSCULUS (MOUSE).
P20490
F-PLACE1003135
SERINE/THREONINE-PROTEIN KINASE STE20 (EC 2.7.1.-).
1.9e-33:99:50
SACCHAROMYCES CEREVISIAE (BAKER'S YEAST).
Q03497
F-PLACE1003163
ENDOZEPINE-RELATED PROTEIN PRECURSOR (MEMBRANE-ASSOCIATED DIAZEPAM BINDING INHIBITOR) (MA-DBI).
9.8e-15:105:38
BOS TAURUS (BOVINE).
P07106
F-PLACE1003407
CLN5 PROTEIN.
4.2e-109:217:89
HOMO SAPIENS (HUMAN).
075503
F-PLACE1003428
BIOTINIDASE PRECURSOR (EC 3.5.1.12).
1.0e-36:104:46
HOMO SAPIENS (HUMAN).
P43251
F-PLACE1003438
HYPOTHETICAL 104.4 KD PROTEIN C17A5.16 IN CHROMOSOME I.
1.1e-10:148:33
SCHIZOSACCHAROMYCES POMBE (FISSION YEAST).
013776
F-PLACE1003460
2-HYDROXY-6-KETONONA-2,4-DIENEDIOIC ACID HYDROLASE (EC 3.7.1.-).
0.00028:134:27
ESCHERICHIA COLI.
P77044
F-PLACE1003529
TRANSCRIPTION REGULATORY PROTEIN SNF5 (SWI/SNF COMPLEX COMPONENT SNF5) (TRANSCRIPTION FACTOR TYE4).
0.00047:157:27
SACCHAROMYCES CEREVISIAE (BAKER'S YEAST).
P18480
F-PLACE1003573
T-CELL SURFACE GLYCOPROTEIN YE1/48 (T LYMPHOCYTE ANTIGEN A1) (LY49-A ANTIGEN).
0.022:129:25
MUS MUSCULUS (MOUSE).
P20937
F-PLACE1003598
TRANS-ACTING TRANSCRIPTIONAL PROTEIN ICPO (P135 PROTEIN) (IER 2.9/ER2.6).
0.0017:102:44
BOVINE HERPESVIRUS TYPE 1 (STRAIN JURA).
P29128
F-PLACE1003644
PROTEIN Q300.
6.7e-05:24:70
MUS MUSCULUS (MOUSE).
Q02722
F-PLACE1003737
TOLL PROTEIN PRECURSOR.
7.3e-08:203:27
DROSOPHILA MELANOGASTER (FRUIT FLY).
P08953
F-PLACE1003772
SALIVARY PROLINE-RICH PROTEIN II-1 (FRAGMENT).
3.7e-07:141:32
HOMO SAPIENS (HUMAN).
P81489
F-PLACE1003839
PROLINE-RICH PROTEIN MP-3 (FRAGMENT).
1.3e-09:201:31
MUS MUSCULUS (MOUSE).
P05143
F-PLACE1003845
PUTATIVE UDP-GLUCOSE 4-EPIMERASE (EG 5.1.3.2) (GALACTOWALDENASE) (UDP-GALACTOSE 4-EPIMERASE).
5.0e-13:103:33
METHANOCOCCUS JANNASCHII.
Q57664
F-PLACE1003852
CELL SURFACE GLYCOPROTEIN EMR1 PRECURSOR (EMR1 HORMONE RECEPTOR).
2.0e-18:189:29
HOMO SAPIENS (HUMAN).
Q14246
F-PLACE1004028
HYPOTHETICAL 9.7 KD PROTEIN IN PURC-PURL INTERGENIC REGION.
0.97:47:31
BACILLUS SUBTILIS.
P12049
F-PLACE1004078
ADSEVERIN (SCINDERIN) (SC).
5.3e-98:176:90
BOS TAURUS (BOVINE).
Q28046
F-PLACE1004166
CREB-BINDING PROTEIN.
9.6e-08:107:34
HOMO SAPIENS (HUMAN).
Q92793
F-PLACE1004168
GENERAL NEGATIVE REGULATOR OF TRANSCRIPTION SUBUNIT 1.
6.8e-05:147:30
SACCHAROMYCES CEREVISIAE (BAKER'S YEAST).
P25655
F-PLACE1004199
!!!! ALU SUBFAMILY J WARNING ENTRY !!!!
4.2e-05:65:52
HOMO SAPIENS (HUMAN).
P39188
F-PLACE1004279
HYPOTHETICAL 59.1 KD PROTEIN ZK637.1 IN CHROMOSOME III.
3.6e-11:166:30
CAENORHABDITIS ELEGANS.
P30638
F-PLACE1004282
HISTONE H1C (CLONE XLHW2).
0.74:73:26
XENOPUS LAEVIS (AFRICAN CLAWED FROG).
P15866
F-PLACE1004305
RAS-RELATED PROTEIN RAC1.
2.3e-23:161:39
DROSOPHILA MELANOGASTER (FRUIT FLY).
P40792
F-PLACE1004441
PROBABLE G PROTEIN-COUPLED RECEPTOR GPR1.
5.4e-70:156:89
HOMO SAPIENS (HUMAN).
P46091
F-PLACE1004450
AMINOPEPTIDASE N (EC 3.4.11.2) (MICROSOMAL AMINOPEPTIDASE). 3.1e-40:196:44
RATTUS NORVEGICUS (RAT).
P15684
F-PLACE1004482
ATP SYNTHASE PROTEIN 8 (EC 3.6.1.34) (A6L).
0.23:26:30
GALLUS GALLUS (CHICKEN).
P14093
F-PLACE1004492
COLLAGEN ALPHA 1(I) CHAIN (FRAGMENTS).
1.2e-05:150:34
BOS TAURUS (BOVINE).
P02453
F-PLACE1004519
ENL PROTEIN.
0.68:170:30
HOMO SAPIENS (HUMAN).
Q03111
F-PLACE1004520
PREGNANCY-SPECIFIC BETA-1 GLYCOPROTEIN PRECURSOR.
3.5e-50:150:74
HOMO SAPIENS (HUMAN).
P11462
F-PLACE1004630
INTEGRIN BETA-6 SUBUNIT PRECURSOR.
9.1e-31:189:39
HOMO SAPIENS (HUMAN).
P18564
F-PLACE1004637
MALE SPECIFIC SPERM PROTEIN MST84DA.
0.47:29:44
DROSOPHILA MELANOGASTER (FRUIT FLY).
Q01642
F-PLACE1004648
PROLINE-RICH PROTEIN MP-2 PRECURSOR.
8.4e-05:89:40
MUS MUSCULUS (MOUSE).
P05142
F-PLACE1004816
MICROFIBRIL-ASSOCIATED GLYCOPROTEIN 4.
1.0e-25:117:46
HOMO SAPIENS (HUMAN).
P55083
F-PLACE1004887
SALIVARY GLUE PROTEIN SGS-3 PRECURSOR.
8.4e-09:195:30
DROSOPHILA ERECTA (FRUIT FLY).
P13730
F-PLACE1005003
PROSTASIN PRECURSOR (EC 3.4.21.-).
1.2e-24:139:40
HOMO SAPIENS (HUMAN).
Q16651
F-PLACE1005005
UBIQUITIN-CONJUGATING ENZYME E2 G2 (EC 6.3.2.19) (UBIQUITIN-PROTEIN LIGASE) (UBIQUITIN CARRIER PROTEIN).
2.5e-28:51:84
HOMO SAPIENS (HUMAN).
P56554
F-PLACE1005031
CHLORINE CHANNEL PROTEIN P64.
2.7e-52:142:76
BOS TAURUS (BOVINE).
P35526
F-PLACE1005239
SPLICING FACTOR, ARGININE/SERINE-RICH 6 (PRE-MRNA SPLICING FACTOR SRP55) (FRAGMENT).
0.27:78:26
ORYCTOLAGUS CUNICULUS (RABBIT).
018776
F-PLACE1005250
HYPOTHETICAL 9.0 KD PROTEIN IN ADH4 5'REGION.
0.22:35:48
SACCHAROMYCES CEREVISIAE (BAKER'S YEAST).
P53056
F-PLACE1005383
FIBRILLIN 1 PRECURSOR.
6.7e-09:134:32
MUS MUSCULUS (MOUSE).
Q61554
F-PLACE1005410
PROTEIN TRANSPORT PROTEIN SEC61 ALPHA SUBUNIT.
9.5e-105:204:100
RATTUS NORVEGICUS (RAT).
P38378
F-PLACE1005426
PREGNANCY-SPECIFIC BETA-1-GLYCOPROTEIN 4 PRECURSOR (PSBG-4).
3.2e-33:184:46
HOMO SAPIENS (HUMAN).
Q00888
F-PLACE1005519
SERINE/THREONINE-PROTEIN KINASE NRK1 (EC 2.7.1.-) (N-RICH KINASE 1).
1.2e-23:143:41
SACCHAROMYCES CEREVISIAE (BAKER'S YEAST).
P38692
F-PLACE1005539
ANTER-SPECIFIC PROLINE-RICH PROTEIN APG (PROTEIN CEX) (FRAGMENT).
5.5e-05:94:37
BRASSICA NAPUS (RAPE).
P40603
F-PLACE1005544
CELL SURFACE A33 ANTIGEN PRECURSOR.
0.00015:132:31
HOMO SAPIENS (HUMAN).
Q99795
F-PLACE1005569
PROTEOGLYCAN LINK PROTEIN PRECURSOR (CARTILAGE LINK PROTEIN) (LP).
0.00092:122:31
EQUUS CABALLUS (HORSE).
Q28381
F-PLACE1005601
TOXIN S4C8.
0.34:32:37
DENDROASPIS JAMESONI KAIMOSAE (EASTERN JAMESON'S MAMBA).
P25683
F-PLACE1005660
SALIVARY GLUE PROTEIN SGS-7 PRECURSOR.
0.99:41:43
DROSOPHILA MELANOGASTER (FRUIT FLY).
P02841
F-PLACE1005669
COLLAGEN ALPHA 1(VII) CHAIN PRECURSOR (LONG-CHAIN COLLAGEN) (LC COLLAGEN).
0.0078:105:37
HOMO SAPIENS (HUMAN).
Q02388
F-PLACE1005682
THYROID RECEPTOR INTERACTING PROTEIN 9 (TRIP9).
2.7e-12:81:41
HOMO SAPIENS (HUMAN).
Q15653
F-PLACE1005725
HYPOTHETICAL 55.1 KD PROTEIN B0416.5 IN CHROMOSOME X.
7.5e-08:142:31
CAENORHABDITIS ELEGANS.
Q11073
F-PLACE1005736
RAC-FAMILY SERINE/THREONINE KINASE HOMOLOG (EC 2.7.1.-).
9.0e-11:91:37
DICTYOSTELIUM DISCOIDEUM (SLIME MOLD).
P54644
F-PLACE1005745
ORM1 PROTEIN.
2.2e-18:137:35
SACCHAROMYCES CEREVISIAE (BAKER'S YEAST).
P53224
F-PLACE1005768
NEUROTOXINS I AND I' PRECURSOR (AAH I AND AAH I').
0.63:13:69
ANDROCTONUS AUSTRALIS HECTOR (SAHARA SCORPION).
P01479
F-PLACE1005815
COLORECTAL MUTANT CANCER PROTEIN (MCC PROTEIN).
1.8e-12:73:50
HOMO SAPIENS (HUMAN).
P23508
F-PLACE1005878
CHLORINE CHANNEL PROTEIN P64.
1.6e-49:115:79
BOS TAURUS (BOVINE).
P35526
F-PLACE1005927
HYPOTHETICAL 35.8 KD PROTEIN C12G12.12 IN CHROMOSOME I.
3.2e-16:152:34
SCHIZOSACCHAROMYCES POMBE (FISSION YEAST).
Q09875
F-PLACE1006071
LAMININ BETA-1 CHAIN PRECURSOR (LAMININ B1 CHAIN).
4.1e-08:215:26
MUS MUSCULUS (MOUSE).
P02469
F-PLACE1006073
SPIDROIN 2 (DRAGLINE SILK FIBROIN 2) (FRAGMENT).
2.1e-05:137:34
NEPHILA CLAVIPES (ORB SPIDER).
P46804
F-PLACE1006079
HOMEOBOX PROTEIN DLX-3.
1.5e-58:144:83
HOMO SAPIENS (HUMAN).
060479
F-PLACE1006093
SYNAPSINS IA AND IB (BRAIN PROTEIN 4.1).
3.8e-05:72:40
HOMO SAPIENS (HUMAN).
P17600
F-PLACE1006208
EBNA-2 NUCLEAR PROTEIN.
3.8e-15:28:75
EPSTEIN-BARR VIRUS (STRAIN B95-8) (HUMAN HERPESVIRUS 4). P12978
F-PLACE1006219
UDP-GLUCOSE 4-EPIMERASE (EC 5.1.3.2) (GALACTOWALDENASE) (UDP- GALACTOSE 4-EPIMERASE) (FRAGMENT).
2.0e-09:38:42
KLEBSIELLA PNEUMONIAE.
P45602
F-PLACE1006277
CELL SURFACE A33 ANTIGEN PRECURSOR.
1.2e-07:183:29
HOMO SAPIENS (HUMAN).
Q99795
F-PLACE1006290
GLYCOSYLTRANSFERASE ALG2 (EC 2.4.1.-).
8.2e-39:171:43
SACCHAROMYCES CEREVISIAE (BAKER'S YEAST).
P43636
F-PLACE1006443
HYPOTHETICAL 60.0 KD PROTEIN IN IMP1-HLJ1 INTERGENIC REGION.
0.0010:155:27
SACCHAROMYCES CEREVISIAE (BAKER'S YEAST).
Q03795
F-PLACE1006515
ZINC FINGER Y-CHROMOSOMAL PROTEIN 1.
0.046:98:28
MUS MUSCULUS (MOUSE).
P10925
F-PLACE1006716
30 KD ADIPOCYTE COMPLEMENT-RELATED PROTEIN PRECURSOR (ACRP30) (ADIPOCYTE SPECIFIC PROTEIN ADIPOQ).
3.6e-25:177:35
MUS MUSCULUS (MOUSE).
Q60994
F-PLACE1006786
PROBABLE NONSPECIFIC LIPID-TRANSFER PROTEIN (LTP) (BASIC PROTEIN) (WBP) (FRAGMENT).
1.0:19:42
TRITICUM AESTIVUM (WHEAT).
P26913
F-PLACE1006809
SLS1 PROTEIN PRECURSOR.
0.0011.37.51
YARROWIA LIPOLYTICA (CANDIDA LIPOLYTICA).
Q99158
F-PLACE1006959
PROLINE-RICH PROTEIN MP-2 PRECURSOR.
5.3e-05:96:41
MUS MUSCULUS (MOUSE).
P05142
F-PLACE1007028
EBNA-1 NUCLEAR PROTEIN.
5.9e-09:219:33
EPSTEIN-BARR VIRUS (STRAIN B95-8) (HUMAN HERPESVIRUS 4).
P03211
F-PLACE1007040
SALIVARY PROLINE-RICH PROTEIN PRECURSOR (CLONE CP7) [CONTAINS: BASIC PEPTIDE P-F] (FRAGMENT).
0. 68:138:24
HOMO SAPIENS (HUMAN).
P02812
F-PLACE1007077
SERINE/THREONINE-PROTEIN KINASE CLA4 (EG 2.7.1.-).
0.73:177:25
SACCHAROMYCES CEREVISIAE (BAKER'S YEAST).
P48562
F-PLACE1007081
COAGULATION FACTOR V PRECURSOR (ACTIVATED PROTEIN C COFACTOR).
3.0e-20:182:39
BOS TAURUS (BOVINE).
Q28107
F-PLACE1007096
HYPOTHETICAL SYMPORTER SLL1374.
2.8e-14:162:30
SYNECHOCYSTIS SP. (STRAIN PCC 6803).
P74168
F-PLACE1007296
ER LUMEN PROTEIN RETAINING RECEPTOR 1 (KDEL RECEPTOR 1).
9.4e-50:120:86
HOMO SAPIENS (HUMAN).
P24390
F-PLACE1007591
MEIOTIC RECOMBINATION PROTEIN REC104.
0.68:73:31
SACCHAROMYCES CEREVISIAE (BAKER'S YEAST).
P33323
F-PLACE1007626
PTB-ASSOCIATED SPLICING FACTOR (PSF).
0.00083:97:34
HOMO SAPIENS (HUMAN).
P23246
F-PLACE1007702
ANTER-SPECIFIC PROLINE-RICH PROTEIN APG PRECURSOR.
1.9e-08:87:36
ARABIDOPSIS THALIANA (MOUSE-EAR CRESS).
P40602
F-PLACE1007845
GLYCOSYLTRANSFERASE ALG2 (EC 2.4.1.-).
1.3e-16:158:40
SACCHAROMYCES CEREVISIAE (BAKER'S YEAST).
P43636
F-PLACE1007881
HYPOTHETICAL 69.4 KD PROTEIN F13H10.3 IN CHROMOSOME IV.
1.2e-11:113:37
CAENORHABDITIS ELEGANS.
Q19425
F-PLACE1007971
METALLOTHIONEIN 20-III ISOFORMS A AND B (MT-20-IIIA AND MT-20-IIIB).
1.0:32:43
MYTILUS EDULIS (BLUE MUSSEL).
P80253
F-PLACE1008282
HEME-REGULATED EUKARYOTIC INITIATION FACTOR EIF-2-ALPHA KINASE (EC 2.7.1.-) (HRI).
8.1e-87:178:87
ORYCTOLAGUS CUNICULUS (RABBIT).
P33279
F-PLACE1008297
MYOSIN HEAVY CHAIN KINASE B (EC 2.7.1.129) (MHCK B).
3.6e-17:187:33
DICTYOSTELIUM DISCOIDEUM (SLIME MOLD).
P90648
F-PLACE1008359
BEM46 PROTEIN (FRAGMENT).
4.9e-07:103:33
SCHIZOSACCHAROMYCES POMBE (FISSION YEAST).
P54069
F-PLACE1008469
D(4) DOPAMINE RECEPTOR (D(2C) DOPAMINE RECEPTOR).
0.0018:78:37
HOMO SAPIENS (HUMAN).
P21917
F-PLACE1008549
FLI-1 ONCOGENE (ERGB TRANSCRIPTION FACTOR).
0.0034:89:30
HOMO SAPIENS (HUMAN).
Q01543
F-PLACE1008657
ADSEVERIN (SCINDERIN) (SC).
6.7e-127:257:91
BOS TAURUS (BOVINE).
Q28046
F-PLACE1008716
ALPHA-1,6-MANNOSYL-GLYCOPROTEIN BETA-1,2-N- ACETYLGLUCOSAMINYLTRANSFERASE (EC 2.4.1.143) (N-GLYCOSYL- OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II).
4.5e-20:66:78
HOMO SAPIENS (HUMAN).
Q10469
F-PLACE1008744
C4B-BINDING PROTEIN ALPHA CHAIN PRECURSOR (PROLINE-RICH PROTEIN) (PRP).
3.6e-19:221:33
HOMO SAPIENS (HUMAN).
P04003
F-PLACE1008984
BIOTIN CARBOXYL CARRIER PROTEIN OF ACETYL-COA CARBOXYLASE PRECURSOR (EC 6.4.1.2) (BCCP).
0.089:61:31
GLYCINE MAX (SOYBEAN).
Q42783
F-PLACE1008985
SYNAPTOTAGMIN V.
8.6e-09:123:35
HOMO SAPIENS (HUMAN).
000445
F-PLACE1009067
HYPOTHETICAL 33.4 KD PROTEIN.
4.3e-09:60:50
HOMO SAPIENS (HUMAN).
Q04323
F-PLACE1009196
SPERM PROTAMINE P1 (CYSTEINE-RICH PROTAMINE).
0.050:23:34
GORILLA GORILLA GORILLA (LOWLAND GORILLA).
P35303
F-PLACE1009279
8.6 KD TRANSGLUTAMINASE SUBSTRATE.
1.4e-07:62:35
TACHYPLEUS TRIDENTATUS (JAPANESE HORSESHOE CRAB). P81281
F-PLACE1009527
MUCIN 2 PRECURSOR (INTESTINAL MUCIN 2).
0.037:71:38
HOMO SAPIENS (HUMAN).
Q02817
F-PLACE1009546
PROLINE-RICH PROTEIN MP-2 PRECURSOR.
5.9e-07:86:39
MUS MUSCULUS (MOUSE).
P05142
F-PLACE1009600
TETRACYCLINE RESISTANCE PROTEIN, CLASS H (TETA(H)).
1.7e-08:113:31
PASTEURELLA MULTOCIDA.
P51564
F-PLACE1009735
TRANS-ACTING TRANSCRIPTIONAL PROTEIN ICPO (IMMEDIATE-EARLY PROTEIN IE110) (VMW110) (ALPHA-0 PROTEIN).
2.6e-09:182:35
HERPES SIMPLEX VIRUS (TYPE 1 / STRAIN 17).
P08393
F-PLACE1009982
REGULATORY PROTEIN E2.
0.99:94:28
HUMAN PAPILLOMAVIRUS TYPE 8.
P06422
F-PLACE1010011
DIACYLGLYCEROL CHOLINEPHOSPHOTRANSFERASE (EC 2.7.8.2) (SN-1,2- DIACYLGLYCEROL CHOLINEPHOSPHOTRANSFERASE) (CHOPT).
2.8e-20:119:42
SACCHAROMYCES CEREVISIAE (BAKER'S YEAST).
P17898
F-PLACE1010078
ORM1 PROTEIN.
3.4e-20:137:37
SACCHAROMYCES CEREVISIAE (BAKER'S YEAST).
P53224
F-PLACE1010081
SERINE/THREONINE-PROTEIN K I NASE CTR1 (EC 2.7.1.37).
1.5e-11:147:32
ARABIDOPSIS THALIANA (MOUSE-EAR CRESS).
Q05609
F-PLACE1010251
NEL-LIKE PROTEIN (FRAGMENT).
1.8e-10:73:42
HOMO SAPIENS (HUMAN).
Q92832
F-PLACE1010445
HYPOTHETICAL BHLF1 PROTEIN.
0.0042:227:33
EPSTEIN-BARR VIRUS (STRAIN B95-8) (HUMAN HERPESVIRUS 4).
P03181
F-PLACE1010713
PUTAT I VE STERO I D DEHYDROGENASE KIK-I (EC 1.1.1.-).
1.5e-77:177:80
MUS MUSCULUS (MOUSE).
070503
F-PLACE1010784
P2Y PURINOCEPTOR 5 (P2Y5) (PURINERGIC RECEPTOR 5) (6H1).
1.7e-18:102:40
GALLUS GALLUS (CHICKEN).
P32250
F-PLACE1010827
COP-COATED VESICLE MEMBRANE PROTEIN P24 PRECURSOR (FRAGMENT).
2.8e-18:109:41
CRICETULUS GRISEUS (CHINESE HAMSTER).
P49020
F-PLACE1010968
PROTEIN-TYROSINE PHOSPHATASE DLAR PRECURSOR (EC 3.1.3.48) (PROTEIN- TYROSINE-PHOSPHATE PHOSPHOHYDROLASE).
2.3e-06:191:28
DROSOPHILA MELANOGASTER (FRUIT FLY).
P16621
F-PLACE1011045
HYPOTHETICAL 71.4 KD PROTEIN IN NMD3-EN02 INTERGENIC REGION.
6.0e-14:153:34
SACCHAROMYCES CEREVISIAE (BAKER'S YEAST).
P38862
F-PLACE1011116
GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).
2.3e-06:195:27
SACCHAROMYCES CEREVISIAE (BAKER'S YEAST).
P08640
F-PLACE1011181
MSP1 PROTEIN HOMOLOG.
4.3e-06:93:25
CAENORHABDITIS ELEGANS.
P54815
F-PLACE1011236
HYPOTHETICAL 59.1 KD PROTEIN ZK637.1 IN CHROMOSOME III.
4.1e-17:180:28
CAENORHABDITIS ELEGANS.
P30638
F-PLACE1011364
HYPOTHETICAL 141.2 KD PROTEIN EEED8.9 IN CHROMOSOME II.
2.1e-24:158:41
CAENORHABDITIS ELEGANS.
Q09298
F-PLACE1011407
ZINC FINGER PROTEIN 140.
3.8e-10:47:74
HOMO SAPIENS (HUMAN).
P52738
F-PLACE1011516
HYPOTHETICAL 21.5 KD PROTEIN IN SEC15-SAP4 INTERGENIC REGION.
1.6e-13:117:34
SACCHAROMYCES CEREVISIAE (BAKER'S YEAST).
P53073
F-PLACE1011708
DORSAL-VENTRAL PATTERNING TOLLOID PROTEIN PRECURSOR (EC 3.4.24.-).
9.9e-22:203:32
DROSOPHILA MELANOGASTER (FRUIT FLY).
P25723
F-PLACE1011824
SERINE/THREONINE-PROTEIN KINASE PAK-BETA (EC 2.7.1.-) (BETA-PAK) (P21-ACTIVATED KINASE 3) (CDC42/RAC EFFECTOR KINASE PAK-B).
1.6e-15:103:36
MUS MUSCULUS (MOUSE).
Q61036
F-PLACE1011978
ZINC FINGER PROTEIN 85 (ZINC FINGER PROTEIN HPF4) (HTF1).
3.3e-55:188:50
HOMO SAPIENS (HUMAN).
Q03923
F-PLACE2000118
EXTENSIN PRECURSOR (CELL WALL HYDROXYPROLINE-RICH GLYCOPROTEIN).
2.8e-23:169:43
NICOTIANA TABACUM (COMMON TOBACCO).
P13983
F-PLACE2000219
MALE SPECIFIC SPERM PROTEIN MST84DA.
0.11:29:41
DROSOPHILA MELANOGASTER (FRUIT FLY).
Q01642
F-PLACE3000181
CADHERIN-RELATED TUMOR SUPPRESSOR PRECURSOR (FAT PROTEIN).
9.5e-26:193:37
DROSOPHILA MELANOGASTER (FRUIT FLY).
P33450
F-PLACE3000213
COMPLEMENT RECEPTOR TYPE 1 PRECURSOR (C3B/C4B RECEPTOR) (CD35 ANTIGEN).
2.3e-23:191:34
HOMO SAPIENS (HUMAN).
P17927
F-PLACE4000354
E-SELECTIN PRECURSOR (ENDOTHELIAL LEUKOCYTE ADHESION MOLECULE 1) (ELAM-1) (LEUKOCYTE-ENDOTHELIAL CELL ADHESION MOLECULE 2) (LECAM2) (CD62E).
3.2e-25:150:30
ORYCTOLAGUS CUNICULUS (RABBIT).
P27113
F-PLACE4000455
IG KAPPA CHAIN PRECURSOR V-III REGION (VG) (FRAGMENT).
0.66:52:36
HOMO SAPIENS (HUMAN).
P04433
F-SKNMC1000004
OPTOMOTOR-BLIND PROTEIN (LETHAL(1)OPTOMOTOR-BLIND) (L(1)OMB) (BIFID PROTEIN).
0.079:88:30
DROSOPHILA MELANOGASTER (FRUIT FLY).
Q24432
F-SKNMC1000014
SCO-SPONDIN (FRAGMENT).
0.63:60:36
BOS TAURUS (BOVINE).
P98167
F-SKNMC1000082
PUTATIVE MITOCHONDRIAL CARRIER YGR096W.
2.4e-10:93:34
SACCHAROMYCES CEREVISIAE (BAKER'S YEAST).
P53257
F-THYRO1000036
PROLINE-RICH PROTEIN MP-3 (FRAGMENT).
0.72:69:36
MUS MUSCULUS (MOUSE).
P05143
F-THYRO1000061
COLLAGEN ALPHA 1(XII) CHAIN (FRAGMENTS).
0.0068:70:38
BOS TAURUS (BOVINE).
P25508
F-THYRO1000099
SPIDROIN 2 (DRAGLINE SILK FIBROIN 2) (FRAGMENT).
0.0063:207:28
NEPHILA CLAVIPES (ORB SPIDER).
P46804
F-THYRO1000196
RETINAL-CADHERIN PRECURSOR (R-CADHERIN) (R-CAD).
1.6e-10:134:32
GALLUS GALLUS (CHICKEN).
P24503
F-THYRO1000400
ERYTHROCYTE BAND 7 INTEGRAL MEMBRANE PROTEIN (STOMATIN) (PROTEIN 7.2B).
3. 9e-28:163:38
MUS MUSCULUS (MOUSE).
P54116
F-THYRO1000580
RENAL TRANSCRIPTION FACTOR KID-1 (TRANSCRIPTION FACTOR 17).
3.3e-15:64:62
MUS MUSCULUS (MOUSE).
Q61751
F-THYRO1000584
EPIDIDYMIS-SPECIFIC ALPHA-MANNOSIDASE (EC 3.2.1.24) (ALPHA-D-MANNOSIDE MANNOHYDROLASE) (135 KD PROTEIN).
1.5e-89:197:72
SUS SCROFA (PIG).
Q28949
F-THYRO1000678
GAP JUNCTION BETA-6 PROTEIN (CONNEXIN 30) (CX30).
7.7e-39:89:87
MUS MUSCULUS (MOUSE).
P70689
F-THYRO1000776
HIGH AFFINITY SULPHATE TRANSPORTER 2.
3.0e-25:83:50
STYLOSANTHES HAMATA.
P53392
F-THYRO1000795
MITOCHONDRIAL 2-OXOGLUTARATE/MALATE CARRIER PROTEIN (OGCP).
1.2e-33:227:37
BOS TAURUS (BOVINE).
P22292
F-THYRO1000846
CUTICLE COLLAGEN 12 PRECURSOR.
6.7e-09:190:33
CAENORHABDITIS ELEGANS.
P20630
F-THYRO1000866
HYPOTHETICAL 146.8 KD PROTEIN C34E10.5 IN CHROMOSOME III.
0. 12:85:31
CAENORHABDITIS ELEGANS.
P46580
F-THYRO1000956
PROBABLE G PROTEIN-COUPLED RECEPTOR APJ.
1.3e-68:165:84
HOMO SAPIENS (HUMAN).
P35414
F-THYRO1000964
TRANS-ACTING TRANSCRIPTIONAL PROTEIN ICPO (P135 PROTEIN) (IER 2.9/ER2.6).
0.015:170:34
BOVINE HERPESVIRUS TYPE 1 (STRAIN JURA).
P29128
F-THYRO1000999
CRYPTDIN-RELATED PROTEIN 4C-2 PRECURSOR (CRS4C).
0.28:40:45
MUS MUSCULUS (MOUSE).
P50715
F-THYRO1001063
SALIVARY PROLINE-RICH PROTEIN PRECURSOR (CLONES CP3, CP4 AND CP5) [CONTAINS:
BASIC PEPTIDE IB-6; PEPTIDE P-H].
3.5e-05:232:32
HOMO SAPIENS (HUMAN).
P04280
F-THYRO1001071
SKIN SECRETORY PROTEIN XP2 PRECURSOR (APEG PROTEIN).
0.00061:131:33
XENOPUS LAEVIS (AFRICAN CLAWED FROG).
P17437
F-THYRO1001102
TRANSCRIPTION INITIATION FACTOR TFIID 135 KD SUBUNIT (TAFII-135) (TAFII135) (TAFII-130) (TAFII130).
0.25:94:38
HOMO SAPIENS (HUMAN).
000268
F-THYRO1001113
SYNAPTOTAGMIN III (SYTIII).
2.0e-08:102:35
MUS MUSCULUS (MOUSE).
035681
F-THYRO1001128
GLYCOPROTEIN X PRECURSOR.
6.8e-07:182:31
EQUINE HERPESVIRUS TYPE 1 (STRAIN AB4P) (EHV-1).
P28968
F-THYRO1001205
NEUROGRANIN (NG) (PROTEIN KINASE C SUBSTRATE 7.5 KD PROTEIN) (RC3).
0.91:33:42
RATTUS NORVEGICUS (RAT).
Q04940
F-THYRO1001237
HYPOTHETICAL PROTEIN IN NIFH2 3'REGION (FRAGMENT).
4.0e-07:68:38
METHANOCOCCUS THERMOLITHOTROPHICUS.
P05410
F-THYRO1001242
SYNAPSINS IA AND IB (BRAIN PROTEIN 4.1).
1.0:104:35
HOMO SAPIENS (HUMAN).
P17600
F-THYRO1001266
SODIUM/GLUCOSE COTRANSPORTER 1 (NA(+)/GLUCOSE COTRANSPORTER 1) (HIGH AFFINITY SODIUM-GLUCOSE COTRANSPORTER).
4.3e-09:119:27
ORYCTOLAGUS CUNICULUS (RABBIT).
P11170
F-THYRO1001327
HYPOTHETICAL 23.7 KD PROTEIN IN CYR1-OST1 INTERGENIC REGION.
1.7e-06:141:24
SACCHAROMYCES CEREVISIAE (BAKER'S YEAST).
P41544
F-THYRO1001456
HYPOTHETICAL 56.2 KD PROTEIN CY31.25C.
1.1e-11:88:48
MYCOBACTERIUM TUBERCULOSIS.
Q10555
F-THYRO1001457
PROTEIN KINASE C, MU TYPE (EC 2.7.1.-) (NPKC-MU).
2.1e-68:228:59
HOMO SAPIENS (HUMAN).
Q15139
F-THYRO1001471
COLLAGEN 1 (X) CHAIN PRECURSOR.
3.9e-05:204:30
GALLUS GALLUS (CHICKEN).
P08125
F-THYRO1001478
COLLAGEN ALPHA 1(X) CHAIN PRECURSOR.
0.038:162:31
HOMO SAPIENS (HUMAN).
Q03692
F-THYRO1001495
!!!! ALU SUBFAMILY SP WARNING ENTRY !!!!
4.8e-19:50:82
HOMO SAPIENS (HUMAN).
P39193
F-THYRO1001523
!!!! ALU SUBFAMILY SX WARNING ENTRY !!!!
5.0e-13:66:62
HOMO SAPIENS (HUMAN).
P39195
F-THYRO1001529
SERINE PALMITOYLTRANSFERASE 2 (EC 2.3.1.50) (LONG CHAIN BASE BIOSYNTHESIS PROTEIN 2) (SPT 2).
1.6e-27:115:53
SCHIZOSACCHAROMYCES POMBE (FISSION YEAST).
Q09925
F-THYRO1001593
PUTATIVE SERINE/THREONINE-PROTEIN KINASE P78 (EC 2.7.1.-).
3.3e-92:225:77
HOMO SAPIENS (HUMAN).
P27448
F-THYRO1001608
PROLINE-RICH PROTEIN MP-2 PRECURSOR.
1.2e-07:127:35
MUS MUSCULUS (MOUSE).
P05142
F-THYRO1001641
NUC-1 NEGATIVE REGULATORY PROTEIN PREG.
0.0039:98:31
NEUROSPORA CRASSA.
Q06712
F-THYRO1001700
INTERFERON-INDUCED, DOUBLE-STRANDED RNA-ACTIVATED PROTEIN KINASE (EC 2.7.1.-) (INTERFERON-INDUCIBLE RNA-DEPENDENT PROTEIN KINASE) (P68 KINASE) (P1/EIF-2A PROTEIN KINASE).
3.3e-09:65:43
HOMO SAPIENS (HUMAN).
P19525
F-THYRO1001702
MYELOID UPREGULATED PROTEIN.
7.8e-62:161:78
MUS MUSCULUS (MOUSE).
035682
F-THYRO1001725
PROTEIN KINASE C SUBSTRATE 80 KD PROTEIN (FRAGMENTS).
0.00061:82:41
RATTUS NORVEGICUS (RAT).
P20468
F-THYRO1001770
PROBABLE SERINE/THREONINE-PROTEIN KINASE YNL020C (EC 2.7.1.-).
1.0e-20:165:35
SACCHAROMYCES CEREVISIAE (BAKER'S YEAST).
P53974
F-THYRO1001803
GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).
3.6e-07:221:30
SACCHAROMYCES CEREVISIAE (BAKER'S YEAST).
P08640
F-Y79AA1000030
TRANSCRIPTIONAL ACTIVATOR FE65.
4.5e-09:43:46
RATTUS NORVEGICUS (RAT).
P46933
F-Y79AA1000127
FAS ANTIGEN LIGAND (APOPTOSIS ANTIGEN LIGAND) (APTL).
1.3e-05:72:43
HOMO SAPIENS (HUMAN).
P48023
F-Y79AA1000207
STANNIOCALCIN PRECURSOR (STC) (CORPUSCLES OF STANNIUS PROTEIN) (CS) (HYPOCALCIN) (TELEOCALCIN).
1.0:100:27
ANGUILLA AUSTRALIS (AUSTRALIAN EEL).
P18301
F-Y79AA1000226
HYPOTHETICAL 52.8 KD PROTEIN T05E11.5 IN CHROMOSOME IV.
2.6e-07:188:28
CAENORHABDITIS ELEGANS.
P49049
F-Y79AA1000270
VACUOLAR ATP SYNTHASE SUBUNIT AC45 PRECURSOR (EC 3.6.1.34) (V-ATPASE AC45 SUBUNIT).
1.6e-102:233:87
BOS TAURUS (BOVINE).
P40682
F-Y79AA1000426
INHIBIN BETA C CHAIN PRECURSOR (ACTIVIN BETA-C CHAIN).
1.1e-14:149:38
HOMO SAPIENS (HUMAN).
P55103
F-Y79AA1000521
MALE SPECIFIC SPERM PROTEIN MST84DD.
0.00079:60:36
DROSOPHILA MELANOGASTER (FRUIT FLY).
Q01645
F-Y79AA1000750
EBNA-1 NUCLEAR PROTEIN.
2.0e-09:131:38
EPSTEIN-BARR VIRUS (STRAIN B95-8) (HUMAN HERPESVIRUS 4).
P03211
F-Y79AA1000776
CORNIFIN (SMALL PROLINE-RICH PROTEIN I) (SPR-I) (SMALL PROLINE-RICH SQUAMOUS CELL MARKER) (SPRP).
0.080:44:40
SUS SCROFA (PIG).
P35323
F-Y79AA1000777
PUTATIVE SERINE/THREONINE-PROTEIN KINASE PKWA (EC 2.7.1.-).
4.2e-33:204:39
THERMOMONOSPORA CURVATA.
P49695
F-Y79AA1000876
PROTEIN DISULFIDE ISOMERASE PRECURSOR (PDI) (EC 5.3.4.1) (PROLYL 4-HYDROXYLASE BETA SUBUNIT) (CELLULAR THYROID HORMONE BINDING PROTEIN) (P55).
4.6e-16:115:38
BOS TAURUS (BOVINE).
P05307
F-Y79AA1000888
TRNA PSEUDOURIDINE SYNTHASE A (EC 4.2.1.70) (PSEUDOURIDYLATE SYNTHASE I) (PSEUDOURIDINE SYNTHASE I) (URACIL HYDROLYASE).
2.0e-09:159:35
TREPONEMA PALLIDUM.
083802
F-Y79AA1000959
HOMEOBOX PROTEIN HOX-B3 (HOX-2.7) (MH-23).
8.8e-08:72:38
MUS MUSCULUS (MOUSE).
P09026
F-Y79AA1000967
CALCIUM/CALMODUL!N-DEPENDENT PROTEIN KINASE TYPE I (EC 2.7.1.123) (CAM KINASE I).
1.1e-37:202:42
RATTUS NORVEGICUS (RAT).
Q63450
F-Y79AA1001013
SALIVARY PROLINE-RICH PROTEIN PO (ALLELE K) [CONTAINS: PEPTIDE P-D] (FRAGMENT).
0.038:128:28
HOMO SAPIENS (HUMAN).
P10162
F-Y79AA1001056
HYPOTHETICAL 7.1 KD PROTEIN IN TK-VS INTERGENIC REGION.
0.41:42:30
BACTERIOPHAGE T4.
P13307
F-Y79AA1001062
TUMOR NECROSIS FACTOR, ALPHA-INDUCED PROTEIN 1, ENDOTHELIAL (B12 PROTEIN).
9.9e-13:132:38
HOMO SAPIENS (HUMAN).
Q13829
F-Y79AA1001090
ANKYRIN HOMOLOG PRECURSOR.
4.0e-19:176:34
CHROMATIUM VINOSUM.
Q06527
F-Y79AA1001212
HYPOTHETICAL PROTEIN MJ0110.
0.095:55:34
METHANOCOCCUS JANNASCHII.
Q57574
F-Y79AA1001264
HYPOTHETICAL 39.9 KD PROTEIN T15H9.1 IN CHROMOSOME II PRECURSOR.
3.3e-53:177:55
CAENORHABDITIS ELEGANS.
Q10005
F-Y79AA1001272
ACROSIN PRECURSOR (EC 3.4.21.10).
6.3e-08:78:46
ORYCTOLAGUS CUNICULUS (RABBIT).
P48038
F-Y79AA1001328
DELTA-LIKE PROTEIN 1 PRECURSOR (DELTA1).
1.3e-08:118:39
RATTUS NORVEGICUS (RAT).
P97677
F-Y79AA1001426
BAND 3 ANION TRANSPORT PROTEIN.
1.7e-18:156:32
GALLUS GALLUS (CHICKEN).
P15575
F-Y79AA1001427
INDUCIBLE NITRATE REDUCTASE 2 (EC 1.6.6.1) (NR).
1.1e-49:131:51
GLYCINE MAX (SOYBEAN).
P39870
F-Y79AA1001430
RING CANAL PROTEIN (KELCH PROTEIN).
2.5e-24:157:40
DROSOPHILA MELANOGASTER (FRUIT FLY).
Q04652
F-Y79AA1001523
TRANSCRIPTION INTERMEDIARY FACTOR 1-BETA (NUCLEAR COREPRESSOR KAP-1) (KRAB-ASSOCIATED PROTEIN 1).
6.2e-15:141:39
HOMO SAPIENS (HUMAN).
Q13263
F-Y79AA1001530
TUBULIN BETA-5 CHAIN.
8.0e-76:204:76
HOMO SAPIENS (HUMAN).
P04350
F-Y79AA1001592
PTB-ASSOCIATED SPLICING FACTOR (PSF).
0.42:104:33
HOMO SAPIENS (HUMAN).
P23246
F-Y79AA1001727
AMALGAM PROTEIN PRECURSOR.
1.9e-09:185:28
DROSOPHILA MELANOGASTER (FRUIT FLY).
P15364
F-Y79AA1001787
PROBABLE CALCIUM-TRANSPORTING ATPASE 9 (EC 3.6.1.38).
7. 6e-43:210:45
SACCHAROMYCES CEREVISIAE (BAKER'S YEAST).
Q12697
F-Y79AA1001793
CORNIFIN (SMALL PROLINE-RICH PROTEIN I) (SPR-I) (SMALL PROLINE-RICH SQUAMOUS CELL MARKER) (SPRP).
0.077:44:40
SUS SCROFA (PIG).
P35323
F-Y79AA1001795
HYPOTHETICAL BHLF1 PROTEIN.
0. 00014:210:31
EPSTEIN-BARR VIRUS (STRAIN B95-8) (HUMAN HERPESVIRUS 4).
P03181
F-Y79AA1001799
MITOCHONDRIAL RNA SPLICING PROTEIN MSR4.
2.8e-18:107:44
SACCHAROMYCES CEREVISIAE (BAKER'S YEAST).
P23500
F-Y79AA1001803
SECRETOGRANIN III PRECURSOR (SGIII).
1.3e-68:182:76
MUS MUSCULUS (MOUSE).
P47867
F-Y79AA1001863
GLYCOPROTEIN J.
0.030:61:32
HERPES SIMPLEX VIRUS (TYPE 1 / STRAIN 17).
P06480
F-Y79AA1002022
WISKOTT-ALDRICH SYNDROME PROTEIN (WASP).
9.8e-08:127:37
HOMO SAPIENS (HUMAN).
P42768
F-Y79AA1002058
CCAAT/ENHANCER BINDING PROTEIN DELTA (C/EBP DELTA) (NUCLEAR FACTOR NF-IL6-BETA) (NF-IL6-BETA).
0.28:56:42
HOMO SAPIENS (HUMAN).
P49716
F-Y79AA1002121
D-BINDING PROTEIN (DBP) (ALBUMIN D BOX-BINDING PROTEIN).
0.71:57:36
MUS MUSCULUS (MOUSE).
Q60925
F-Y79AA1002129
TRANSCRIPTIONAL REGULATORY PROTEIN ALGP (ALGINATE REGULATORY PROTEIN ALGR3).
0. 98:158:24
PSEUDOMONAS AERUGINOSA.
P15276
F-Y79AA1002213
HYPOTHETICAL 52.7 KD PROTEIN C38C10.2 IN CHROMOSOME III.
4.7e-39:218:41
CAENORHABDITIS ELEGANS.
Q03567
F-Y79AA1002334
HYPOTHETICAL PROTEIN MJ1345.
1.8e-08:164:26
METHANOCOCCUS JANNASCHII.
Q58741
F-Y79AA1002373
CORNIFIN (SMALL PROLINE-RICH PROTEIN I) (SPR-I) (SMALL PROLINE-RICH SQUAMOUS CELL MARKER) (SPRP).
0.083:44:40
SUS SCROFA (PIG).
P35323
F-Y79AA1002376
DYNEIN INTERMEDIATE CHAIN 2, CYTOSOLIC (DH IC-2).
3.0e-91:214:83
RATTUS NORVEGICUS (RAT).
Q62871
F-Y79AA1002378
ZINC FINGER PROTEIN 38 (ZFP-38) (CTFIN51) (TRANSCRIPTION FACTOR RU49).
1.0e-59:163:74
MUS MUSCULUS (MOUSE).
Q07231
F-Y79AA1002381
CELL DIVISION CONTROL PROTEIN 28 (EC 2.7.1.-).
9.5e-41:179:38
SACCHAROMYCES CEREVISIAE (BAKER'S YEAST).
P00546

### Homology search result 6

The result of the homology search in the GenBank(http://www.ncbi.nlm.nih.gov/web/GenBank/) using the clone sequences of the 5'-ends. except EST and STS sequences
Indicated are from the top,
the name of the clone sequence,
definition of the top hit data,
the P-value: the length of the sequence used for comparison (nucleotide):similarity (%),
the Accession No. of the top hit data.

Data were not shown for the clones in which the P-value was higher than 1.
F-BNGH41000020
H.sapiens mitochondrial DNA, complete genome.
6.0e-188:913:97
X93334
F-BNGH41000087
Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.
7.1e-32:176:99
AL034418
F-BNGH41000091
Homo sapiens potassium channel h-eag.
1.6e-79:687:76
AJ001366
F-HEMBA1000006
S.erythraea second and third ORF's of eryA gene, complete cds.
0.95:243:64
M63677
F-HEMBA1000121
Human DNA sequence *** SEQUENCING IN PROGRESS *** from clone 796F18, WORKING
DRAFT SEQUENCE.
5.9e-70:450:89
AL031291
F-HEMBA1000128
Plasmodium falciparum DNA *** SEQUENCING IN PROGRESS *** from contig 3-14, complete sequence.
1.0:274:59
Z98549
F-HEMBA1000275
Herpes simplex virus type 2 (strain HG52), complete genome.
0.036:625:55
Z86099
F-HEMBA1000300
Homo sapiens chromosome 17, clone hRPK.178_C_3, complete sequence.
1.4e-40:343:80
AC005702
F-HEMBA1000349
Homo sapiens chromosome 17, clone hRPK.235_I_10, complete sequence.
7.5e-65:451:72
AC005922
F-HEMBA1000443
Human DNA sequence *** SEQUENCING IN PROGRESS *** from clone 222E13, WORKING
DRAFT SEQUENCE.
8.1e-77:216:95
Z93241
F-HEMBA1000462
Caenorhabditis elegans cosmid C49H3.
3.7e-06:98:82
U42436
F-HEMBA1000477
Mus musculus BALB/c putative growth factor GDF7 (Gdf7) gene, partial cds. 9.1e-05:190:65
U08339
F-HEMBA1000590
Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
3.0e-102:209:99
AL021578
F-HEMBA1000634
*** SEQUENCING IN PROGRESS *** Homo sapiens chromosome 4, BAG clone C0539L10; HTGS phase 1, WORKING DRAFT SEQUENCE, 15 unordered pieces.
2.0e-95:460:99
AC004480
F-HEMBA1000671 Homo sapiens chromosome 19, BAC CIT-B-393i15 (BC301323), complete sequence. 1.5e-28:259:69
AC006116
F-HEMBA1000713
Homo sapiens 10kD protein (BC10) mRNA, complete cds.
6.5e-126:442:97
AF053470
F-HEMBA1000732
Homo sapiens clone IMAGE Consortium 302831 latent transforming growth factor-beta binding protein 4 mRNA, partial cds.
1.7e-45:258:94
AF054502
F-HEMBA1000745
Streptomyces coelicolor cosmid 3F9.
3.5e-06:360:61
AL023862
F-HEMBA1000835
Homo sapiens fibrillin mRNA, complete cds.
1.3e-07:151:69
L13923
F-HEMBA1000875
Human Krueppel-type zinc finger protein (ZNF169) gene, partial cds.
2.6e-28:249:81
U28322
F-HEMBA1000907
Spermatozopsis similis mRNA for 95 kD basal apparatus-protein.
3.4e-09:599:60
AJ001438
F-HEMBA1000940
Homo sapiens connexin46.6 (Cx46.6) gene, complete cds.
1.7e-16:307:66
AF014643
F-HEMBA1000962
Homo sapiens Chromosome 11q12.2 PAC clone pDJ519o13 containing human gene for ferritin heavy chain (FTH), complete sequence.
0.00040:497:59
AC004228
F-HEMBA1001184
Homo sapiens SH3 domain binding glutamic acid-rich-like protein (SH3BGRL) mRNA, complete cds.
8.8e-23:404:67
AF042081
F-HEMBA1001221
Human transmembrane protein mRNA, complete cds.
2.4e-42:858:63
U19878
F-HEMBA1001228
Human germline oligomeric matrix protein (COMP) mRNA, complete cds.
1.9e-82:470:91
L32137
F-HEMBA1001272
Human Ig gamma-2 heavy chain switch region.
0.032:549:60
U39934
F-HEMBA1001296
H.sapiens mRNA for PQ-rich protein.
6.9e-07:73:98
Z50194
F-HEMBA1001297
Homo sapiens putative transcription factor CA150 mRNA, complete cds.
9.3e-14:143:81
AF017789
F-HEMBA1001390
Mus musculus polymerase I-transcript release factor mRNA, complete cds. 2.5e-56:464:81
AF036249
F-HEMBA1001563
Human DNA sequence from clone 71L16 on chromosome Xp11. Contains a probable Zinc Finger protein (pseudo)gene, an unknown putative gene, a pseudogene with high similarity to part of antigen KI-67, a putative Chondroitin 6-Sulfotransferase LIKE gene and a KIAA0267 LIKE putative Na(+)/H(+) exchanger protein gene. Contains a predicted CpG island, ESTs, STSs and GSSs and genomic markers DXS1003 and DXS1055, complete sequence.
3.1e-06:210:68
AL022165
F-HEMBA1001621
Human G protein-coupled receptor APJ gene, complete cds.
2.0e-98:516:95
U03642
F-HEMBA1001878
Homo sapiens U5 snRNP-specific 40 kDa protein mRNA, complete cds.
1.0e-170:810:98
AF090988
F-HEMBA1001886
Human repressor transcriptional factor (ZNF85) mRNA, complete cds.
3.3e-114:849:80
U35376
F-HEMBA1002048
HS_3058_B2_A11_MR CIT Approved Human Genomic Sperm Library D Homo sapiens genomic clone Plate=3058 Col=22 Row=B, genomic survey sequence.
3.8e-11:244:66
AQ103440
F-HEMBA1002131
Homo sapiens mRNA for KIAA0584 protein, partial cds.
3.5e-44:709:66
AB011156
F-HEMBA1002163
Homo sapiens chromosome X, clone 592, WORKING DRAFT SEQUENCE, 8 unordered pieces.
2.3e-28:373:71
AC002489
F-HEMBA1002164
Human DNA sequence *** SEQUENCING IN PROGRESS *** from clone 167A19, WORKING
DRAFT SEQUENCE.
1.3e-127:493:99
AL031427
F-HEMBA1002167
Rattus norvegicus neuroligin I mRNA, complete cds.
8.1e-155:850:91
U22952
F-HEMBA1002178
Homo sapiens mRNA for KIAA0584 protein, partial cds.
2.6e-46:794:65
AB011156
F-HEMBA1002195
Human lysosomal alpha-mannosidase (manB) gene, 5' flanking region and exon 1. 7.7e-35:255:86
U60885
F-HEMBA1002227
Homo sapiens mRNA for 80K-L protein, complete cds.
3.8e-137:382:95
D10522
F-HEMBA1002239
Homo sapiens Chromosome 16 BAC clone CIT987SK-A-485G10, complete sequence. 4.5e-43:452:74
AC003049
F-HEMBA1002316
Homo sapiens DNA sequence from PAC 29C18 on chromosome 22.
3.0e-22:609:67
Z97192
F-HEMBA1002420
Homo sapiens clone RG031N19, WORKING DRAFT SEQUENCE, 1 unordered pieces. 4.2e-142:322:98
AC005632
F-HEMBA1002421
Human heparan sulfate proteoglycan (HSPG) core protein, 3' end.
1. 3e-165: 778: 98
J04621
F-HEMBA1002524
Homo sapiens clone UWGC:y55c025 from 6p21, complete sequence.
1. 3e-153:313:96
AC004209
F-HEMBA1002551
Human potential CENP-C binding target sequence, 0.7 kb clone, partial sequence 2.
6.1e-16:108:97
U57994
F-HEMBA1002767
Homo sapiens chromosome 1p33-p34 beta-1,4-galactosyltransferase mRNA, complete cds.
1.4e-168:798:98
AF038660
F-HEMBA1002985
HS_3165_A2_C08_MR CIT Approved Human Genomic Sperm Library D Homo sapiens genomic clone Plate=3165 Col=16 Row=E, genomic survey sequence.
1.7e-08:127:76
AQ142051
F-HEMBA1002992
RPCI11-67B15.TJ RFCI11 Homo sapiens genomic clone R-67B15, genomic survey sequence.
2.7e-11:119:86
AQ201833
F-HEMBA1003047
Homo sapiens intrinsic factor-B12 receptor precursor, mRNA, complete cds. 4.5e-187:873:99
AF034611
F-HEMBA1003072
Gallus gallus single-strand DNA-binding protein csdp mRNA, partial cds. 4.1e-50:515:73
U68380
F-HEMBA1003101
Homo sapiens tyrosylprotein sulfotransferase-2 mRNA, complete cds.
5.3e-139-671:98
AF049891
F-HEMBA1003120
Homo sapiens chromosome 19, BAC CIT-B-393i15 (BC301323), complete sequence. 3.3e-44:213:73
AC006116
F-HEMBA1003230
Homo sapiens UP50 mRNA, complete cds.
5.5e-183:856:98
AF093118
F-HEMBA1003294
Human DNA sequence *** SEQUENCING IN PROGRESS *** from clone 495010, WORKING
DRAFT SEQUENCE.
4.2e-38:558:69
AL031121
F-HEMBA1003315
Arabidopsis thaliana genomic DNA, chromosome 5, TAC clone: K919, complete sequence.
1.2e-61:737:68
AB013390
F-HEMBA1003392
Homo sapiens LDL receptor-related protein 6 (LRP6) mRNA, complete cds.
2.9e-183:851:99
AF074264
F-HEMBA1003399
Homo sapiens clone DJ0826E18, WORKING DRAFT SEQUENCE, 4 unordered pieces. 8.7e-16:215:74
AC005282
F-HEMBA1003487
H. sapiens DNA sequence.
0.0075:158:67
Z22340
F-HEMBA1003497
Human DNA sequence *** SEQUENCING IN PROGRESS *** from clone 2705, WORKING
DRAFT SEQUENCE.
1.1e-109:538:98
AL033529
F-HEMBA1003530
S.scrofa mRNA for BM88 antigen.
2.8e-47:644:69
X82027
F-HEMBA1003602
Human (lambda) DNA for immunoglobulin light chain.
2.5e-94:551:91
D86997
F-HEMBA1003732
Homo sapiens clone DJ0935K16, complete sequence.
6.1e-151:777:96
AC006011
F-HEMBA1003945
Homo sapiens clone 638 unknown mRNA, complete sequence.
1.8e-76:310:93
AF091085
F-HEMBA1004007
F-HEMBA1004067
Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.
8.7e-133:718:94
AL031864
F-HEMBA1004085
Homo sapiens, clone hRPK. 2_A_1, complete sequence.
2.7e-58:256:80
AC006197
F-HEMBA1004110
Homo sapiens intersectin short form mRNA, complete cds.
3.8e-159:779:96
AF064243
F-HEMBA1004250
Homo sapiens chromosome 5, BAC clone 182a8 (LBNL H161), complete sequence. 1.2e-183:863:99
AC005752
F-HEMBA1004391
H.sapiens gene for neural cell adhesion molecule L1.
0.51:426:59
Z29373
F-HEMBA1004444
Homo sapiens clone DJ0971C03, WORKING DRAFT SEQUENCE, 18 unordered pieces. 3.3e-147:463:93
AC004938
F-HEMBA1004454
Homo sapiens tetraspan NET-4 mRNA, complete cds.
0.00036:230:62
AF065389
F-HEMBA1004505
D.melanogaster mRNA for alpha 1,2 mannosidase.
5.5e-17:663:58
X82640
F-HEMBA1004785
Gallus gallus mRNA for chromobox protein (CHCB3), complete cds.
6.6e-19:322:68
AB005619
F-HEMBA1004797
Haemonchus contortus GT microsatellite DNA sequence.
3.0e-08:175:71
U84474
F-HEMBA1004952
Mus musculus recombinant quaking gene sequence.
4.8e-15:398:65
U44942
F-HEMBA1004971
F-HEMBA1004982
F-HEMBA1005070
Human mRNA for KIAA0310 gene, complete cds.
2.5e-65:370:93
AB002308
F-HEMBA1005084
Mouse transcriptional control element.
0.0024:189:63
M17284
F-HEMBA1005145
Pseudorabies virus glycoprotein gp50 gene, complete cds.
0.00022:395:60
AF092447
F-HEMBA1005230
Homo sapiens chromosome 19, BAC CIT-B-393i15 (BC301323), complete sequence. 2.8e-102:302:94
AC006116
F-HEMBA1005246
Homo sapiens CAGH44 mRNA, partial cds.
5.0e-29:429:66
U80741
F-HEMBA1005267
Homo sapiens chromosome 3, olfactory receptor pseudogene cluster 1, complete sequence, and myosin light chain kinase (MLCK) pseudogene, partial sequence. 1.0e-43:320:87
AF042089
F-HEMBA1005337
Plasmodium falciparum MAL3P6, complete sequence.
4.1e-08:84:89
Z98551
F-HEMBA1005430
F-HEMBA1005449
T.aestivum mRNA for a proline-rich protein.
0.00097:385:61
X52472
F-HEMBA1005489
Homo sapiens Xq28 genomic DNA in the region of the ALD locus containing the genes for creatine transporter (SLC6A8), CDM, adrenoleukodystrophy (ALD), Na+-isocitrate dehydrogenase gamma subunit (IDH), and translocon-associated protein delta (TRAP) genes, complete cds, plexin related protein (PLEXR) and serine kinase (SK) genes, partial cds, Xq28lu1 gene and cytochrome C (CCp) pseudogene.
7. 8e-16:405:62
U52111
F-HEMBA1005522
O.cuniculus rACNG mRNA for aorta CNG channel.
5.9e-47:344:85
X59668
F-HEMBA1005545
Human m3 muscarinic acetylcholine receptor (CHRM3) gene, complete cds. 5.1e-173:810:98
U29589
F-HEMBA1005698
F-HEMBA1005913
HS_2249_B1_E01_MF CIT Approved Human Genomic Sperm Library D Homo sapiens genomic clone Plate=2249 Col=1 Row=J, genomic survey sequence.
0.17:215:61
AQ072649
F-HEMBA1005929
Homo sapiens chromosome 19, cosmid R31237, complete sequence.
7.0e-107:285:93
AC005581
F-HEMBA1005945
Oryctolagus cuniculus peroxisomal Ca-dependent solute carrier mRNA, complete cds.
1.8e-46:670:65
AF004161
F-HEMBA1006016
CIT-HSP-2334L16.TF CIT-HSP Homo sapiens genomic clone 2334L16, genomic survey sequence.
2.1e-13:246:69
AQ038406
F-HEMBA1006171
F-HEMBA1006276
Homo sapiens chromosome 19, CIT-HSP-444n24, complete sequence.
1.4e-144:416:93
AC005261
F-HEMBA1006299
F-HEMBA1006311
F-HEMBA1006335
Human DNA sequence from clone 496H19 on chromosome 6q24 Contains ESTs, complete sequence.
9.6e-61:370:91
AL023582
F-HEMBA1006357
Homo sapiens secretory carrier membrane protein (SCAMP2) mRNA, complete cds. 2.3e-26:389:67
AF005038
F-HEMBA1006430
Caenorhabditis elegans cosmid T12A2.
4.6e-23:283:72
U13019
F-HEMBA1006482
Homo sapiens h-sco1 (SC01) mRNA, nuclear gene encoding mitochondrial protein, complete cds.
1.9e-144:575:98
AF026852
F-HEMBA1006517
F-HEMBA1006544
Homo sapiens suppressor of white-apricot homolog 2 (SWAP2) gene, exons 12 and 13.
2.3e-151:732:97
AF042809
F-HEMBA1006572
HS_3058_B2_A11_MR CIT Approved Human Genomic Sperm Library D Homo sapiens genomic clone Plate=3058 Col=22 Row=B, genomic survey sequence.
1.9e-45:245:96
AQ103440
F-HEMBA1006658
Homo sapiens mRNA for KIAA0687 protein, partial cds.
3.6e-127:646:95
AB014587
F-HEMBA1006707
Human DNA sequence from clone 453C12 on chromosome 24q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
1.7e-118:397:98
AL021578
F-HEMBA1006724
H.sapiens CpG island DNA genomic Mse1 fragment, clone 40c2, forward read cpg40c2.ft1k.
1.4e-53:282:97
Z55440
F-HEMBA1006749
Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
3.9e-116:457:98
AL021578
F-HEMBA1006770
Xenopus laevis elav-type ribonucleoprotein (etr-1) mRNA, complete cds. 1.6e-53:280:81
U16800
F-HEMBA1006902
Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
4.9e-122:462:98
AL021578
F-HEMBA1006912
F-HEMBA1006916
Homo sapiens Grb14 mRNA, complete cds.
1.6e-118:651:92
L76687
F-HEMBA1006960
Human DNA sequence *** SEQUENCING IN PROGRESS *** from clone 110F11, WORKING
DRAFT SEQUENCE.
0.20:298:60
AL033526
F-HEMBA1007013
Rattus norvegicus repeat element associated with the Rasgrf1 gene.
8.0e-07:531:59
AF056927
F-HEMBA1007057
Human DNA sequence from clone 522J7 on chromosome 22q13.3. Contains part of a 60S Ribosomal protein L5 pseudogene and a Peregrin (BR140) LIKE gene downstream of a putative CpG island. Contains ESTs, STSs and GSSs, complete sequence.
0.27:277:64
Z98885
F-HEMBA1007063
F-HEMBA1007226
Human DNA sequence *** SEQUENCING IN PROGRESS *** from clone 310013, WORKING
DRAFT SEQUENCE.
0.00033:488:63
AL031658
F-HEMBA1007241
Caenorhabditis elegans cosmid T15B7.
0.068:304:59
AF022985
F-HEMBA1007291
Homo sapiens chromosome 19, fosmid 37502, complete sequence.
6.2e-123:587:98
AC004755
F-HEMBA1007332
Human HeLa mRNA isolated as a false positive in a two-hybrid-screen.
1.3e-30:172:97
U56430
F-HEMBB1000106
Human DNA sequence from clone 1170D6 on chromosome Xq22.3-23. Contains a pseudogene similar to U-SNRNP_associated Cyclophilin (USA-CYP, EC 5.2.1.8), ESTs, an STS and a GSS, complete sequence.
0.033:332:61
AL030995
F-HEMBB1000276
Dictyostelium discoideum gene encoding a novel glycoprotein.
0.00070:440:60
AJ005262
F-HEMBB1000309
Homo sapiens zinc finger protein (MBLL) mRNA, complete cds.
7.6e-34:180:100
AF061261
F-HEMBB1000407
Human Chromosome 11 pac pDJ393o15, WORKING DRAFT SEQUENCE, 8 unordered pieces. 0.16:228:64
AC000384
F-HEMBB1000447
Homo sapiens JWA protein mRNA, complete cds.
1.4e-158:750:98
AF070523
F-HEMBB1000542
Human DNA sequence from PAC 509L4 on chromosome 6q22.1-6q22.33. Contains SSX3 like pseudogene, EST, STS.
4. 3e-141:874:89
Z99496
F-HEMBB1000567
Human DNA for insulin-like growth factor II (IGF-2); exon 7 and additional
ORF.
9.7e-122:572:99
X07868
F-HEMBB1000642
F-HEMBB1000668
Caenorhabditis elegans cosmid K06A5.
0.00041:174:64
AF039038
F-HEMBB1000679
C.familiaris mRNA for TRAM-protein.
6.1e-100:756:80
X63678
F-HEMBB1000881
Danio rerio mRNA for MINDIN2, complete cds.
6.2e-40:581:66
AB006085
F-HEMBB1000905
Homo sapiens clone RG315H11, WORKING DRAFT SEQUENCE, 5 unordered pieces. 4.9e-91:209:94
AC005089
F-HEMBB1001026
Human p76 mRNA, complete cds.
1.9e-06:410:61
U81006
F-HEMBB1001048
Human Hpast (HPAST) mRNA, complete cds.
6.8e-55:524:75
AF001434
F-HEMBB1001200
Plasmodium falciparum 3D7 chromosome 12 PFYAC293 genomic sequence, WORKING DRAFT SEQUENCE, 9 unordered pieces.
4.4e-12:794:59
AC004157
F-HEMBB1001407
Homo sapiens chromosome 17, clone hRPC.971_F_3, WORKING DRAFT SEQUENCE, 1 ordered pieces.
2.7e-43:281:91
AC004150
F-HEMBB1001530
HS_2255_B1_F05_MR CIT Approved Human Genomic Sperm Library D Homo sapiens genomic clone Plate=2255 Col=9 Row=L, genomic survey sequence.
2.1e-14:95:97
AQ131814
F-HEMBB1001547
S.cerevisiae chromosome VII reading frame 0RF YGL236c.
1.1e-19:550:61
Z72758
F-HEMBB1001573
Homo sapiens 12p13.3 PAC RPCI5-951N9 (Roswell Park Cancer Institute Human PAC library) complete sequence.
2.7e-07:467:60
AC004672
F-HEMBB1001847
H.sapiens CpG island DNA genomic Mse1 fragment, clone 13d12, reverse read cpg13d12.rt1c.
1.1e-14:94:100
Z64565
F-HEMBB1001959
Human DNA sequence *** SEQUENCING IN PROGRESS *** from clone 537K23, WORKING
DRAFT SEQUENCE.
1.2e-82:492:90
AL034405
F-HEMBB1001978
Homo sapiens chromosome 10 clone CIT987SK-1057L21 map 10q25, complete sequence.
7.0e-23:239:76
AC005386
F-HEMBB1002039
Human DNA sequence from cosmid 315B17, between markers DXS366 and DXS87 on chromosome X contains ESTs.
3.5e-49:605:71
Z73967
F-HEMBB1002041
R.norvegicus mRNA for leucocyte common antigen-related protein (3941 bp). 3.5e-09:501:60
X83546
F-HEMBB1002051
Homo sapiens E74-like factor 5 (ELF5) mRNA, complete cds.
3.1e-95:454:99
AF049703
F-HEMBB1002120
F-HEMBB1002162
Homo sapiens genethonin 1 mRNA, complete cds.
7.0e-67:328:99
AF062534
F-HEMBB1002228
Homo sapiens unknown mRNA, complete cds.
1.6e-39:208:98
AF047439
F-HEMBB1002245
Rattus norvegicus prostaglandin F2a receptor regulatory protein precursor, mRNA, complete cds.
3.7e-68:424:87
U26595
F-HEMBB1002302
RPC111-18E11.TVB RPCI-11 Homo sapiens genomic clone RPCI-11-18E11, genomic survey sequence.
2.7e-15:101:98
B88081
F-HEMBB1002427
Homo sapiens chromosome 9q34, clone 70C11, complete sequence.
2.9e-123:249:90
AC002319
F-HEMBB1002465
Mouse short chain acyl-CoA dehydrogenase mRNA, complete cds.
7.9e-18:545:61
L11163
F-HEMBB1002661
Drosophila melanogaster; Chromosome 2R: Region 44A1-44A2; P1 clone DS07435, WORKING DRAFT SEQUENCE, 2 unordered pieces.
1.9e-07:187:67
AC005445
F-HEMBB1002663
F-HEMBB1002693
Homo sapiens full length insert cDNA clone ZD85G07.
2.1e-20:136:93
AF086462
F-MAMMA1000046
CIT-HSP-2166017.TF CIT-HSP Homo sapiens genomic clone 2166017, genomic survey sequence.
2.0e-60:345:92
B92334
F-MAMMA1000102
Human DNA sequence from cosmid B33F2 on chromosome 22 Contains ESTs.
3.0e-161:766:98
Z79996
F-MAMMA1000106
Rat gene for alpha 1B adrenergic receptor, promoter region and partial cds. 0.0025:247:64
D32045
F-MAMMA1000118
Canis familiaris betal adrenergic receptor (dogbetal) gene, complete cds. 6.1e-06:545:60
U73207
F-MAMMA1000141
Homo sapiens 12q24.2 PAC RPCI1-128M12 (Roswell Park Cancer Institute Human PAC library) complete sequence.
1.5e-10:151:78
AC004024
F-MAMMA1000204
Homo sapiens mRNA for LGMD2B protein.
2.1e-166:781:98
AJ007670
F-MAMMA1000226
*** SEQUENCING IN PROGRESS *** Homo sapiens chromosome 4, BAC clone B33108; HTGS phase 1, WORKING DRAFT SEQUENCE, 10 unordered pieces.
2.9e-35:188:100
AC004064
F-MAMMA1000403
Human vascular addressin MAdCAM-1 mRNA, complete cds.
0.00043:538:59
U82483
F-MAMMA1000449
Human DNA sequence *** SEQUENCING IN PROGRESS *** from clone 317C6, WORKING
DRAFT SEQUENCE.
0.090:514:60
Z97651
F-MAMMA1000457
H.sapiens mRNA for NADH-cytochrome b5 reductase.
5.5e-36:469:68
Y09501
F-MAMMA1000473
Caenorhabditis elegans cosmid B0491, complete sequence.
0.0052:187:64
Z49907
F-MAMMA1000496
Homo sapiens PAC clone DJ130H16 from 22q12.1-qter, complete sequence.
1. 2e-81 :318:92
AC004997
F-MAMMA1000528
P.falciparum complete gene map of plastid-like DNA (IR-B).
0. 016:343:58
X95276
F-MAMMA1000591
Mus musculus UDP-GalNAc:polypeptide N-acetylgalactosaminyltransferase-T3 mRNA, complete cds.
1.2e-24:493:63
U70538
F-MAMMA1000614
*** SEQUENCING IN PROGRESS *** Homo sapiens chromosome 4, BAC clone C0190L06; HTGS phase 1, WORKING DRAFT SEQUENCE, 21 unordered pieces.
7.5e-13:615:60
AC004670
F-MAMMA1000652
Homo sapiens Chromosome 16 BAC clone CIT987SK-A-116A10, complete sequence. 1.6e-59:451:82
AC004638
F-MAMMA1000681
Homo sapiens mRNA for putative G-protein coupled receptor, EDG6.
1.2e-32:636:65
AJ000479
F-MAMMA1000706
*** SEQUENCING IN PROGRESS *** Homo sapiens chromosome 4, BAG clone C0110D16; HTGS phase 1, WORKING DRAFT SEQUENCE, 7 unordered pieces.
6.8e-06:428:62
AC004578
F-MAMMA1000788
HS_3080_A2_B03_MR CIT Approved Human Genomic Sperm Library D Homo sapiens genomic clone Plate=3080 Col=6 Row=C, genomic survey sequence.
4.9e-35:204:94
AQ128409
F-MAMMA1000810
Homo sapiens DNA sequence from PAC 510L9 on chromosome 6p24.1-p25.3.
5.8e-06:246:65
AL022098
F-MAMMA1000814
Anadara trapezia (alpha 3.15L) hemoglobin alpha-chain (HBA) gene, exons 2 and 3, intron 2, including hypervariable microsatellite polymorphic repeat regions.
1.0e-12:176:75
L25098
F-MAMMA1000881
Rattus norvegicus serum and glucocorticoid-regulated kinase (sgk) mRNA, complete cds.
2.8e-07:283:63
L01624
F-MAMMA1000986
Homo sapiens chromosome 16 BAC clone CIT987SK-334D11 complete sequence. 1.8e-166:306:99
AF001550
F-MAMMA1000994
Mycobacterium tuberculosis H37Rv complete genome; segment 48/162.
0.75:260:61
AL021897
F-MAMMA1001043
H.sapiens mRNA for latent transforming growth factor-beta binding protein
(LTBP-2).
0.038:376:60
Z37976
F-MAMMA1001066
Homo sapiens DNA from chromosome 19-cosmid f24590 containing CAPNS and POL2RI, genomic sequence.
4.4e-15:162:72
AD001527
F-MAMMA1001094
Homo sapiens clone 243 unknown mRNA, complete sequence.
6,2e-181:844:99
AF091094
F-MAMMA1001141
Can is familiar is beta1 adrenergic receptor (dogbeta1) gene, complete cds. 1.3e-10:602:59
U73207
F-MAMMA1001150
M.musculus (Balb/c) mRNA for serine/threonine protein kinase.
7.7e-57:447:67
Z34524
F-MAMMA1001237
Rattus norvegicus monocarboxylate transporter MCT3 mRNA, complete cds. 1.5e-08:306:65
AF059258
F-MAMMA1001284
HS_3076_A1_F08_MF CIT Approved Human Genomic Sperm Library D Homo sapiens genomic clone Plate=3076 Col=15 Row=K, genomic survey sequence.
5.2e-53:307:93
AQ120674
F-MAMMA1001310
I(2)09851 Drosophila melanogaster P lethal line Drosophila melanogaster genomic Sequence recovered from 3' end of P element, genomic survey sequence. 0.00072:209:66
AQ025672
F-MAMMA1001344
Fugu rubripes neural cell adhesion molecule L1 homolog (L1-CAM) gene, complete cds; putative protein 1 (PUT1) gene, partial cds; mitosis-specific chromosome segregation protein SMC1 homolog (SMC1) gene, complete cds: and calcium channel alpha-1 subunit homolog (CCA1) and putative protein 2 (PUT2) genes, partial cds, complete sequence.
5.2e-05:164:67
AF026198
F-MAMMA1001418
Human Na+/nucleoside cotransporter (hCNT1a) mRNA, complete cds.
6.0e-35:622:63
U62966
F-MAMMA1001532
Homo sapiens PAC clone DJ0728D04, complete sequence.
5.2e-46:538:74
AC004865
F-MAMMA1001609
Homo sapiens chromosome 10 clone CIT-HSP-1240G16 map 10q25.1, complete sequence.
0.00031:592:57
AC005886
F-MAMMA1001615
H.sapiens CpG island DNA genomic Mse1 fragment, clone 71h9, reverse read cpg71h9.rt1a.
1.2e-25:146:99
Z62710
F-MAMMA1001623
Homo sapiens 12q24.2 BAC RPCI11-407A16 (Roswell Park Cancer Institute Human BAC Library) complete sequence.
3.9e-69:471:85
AC006065
F-MAMMA1001634
Human DNA sequence from PAC 93C23 on chromosome X. Contains steroid 5-alpha-reductase pseudogene, ESTs and STS.
2.2e-22:228:79
AL008713
F-MAMMA1001893
HS_3067_B2_H09_MF CIT Approved Human Genomic Sperm Library D Homo sapiens genomic clone Plate=3067 Col=18 Row=P, genomic survey sequence.
2.5e-29:188:93
AQ138065
F-MAMMA1001901
Human DNA sequence from clone 354J5 on chromosome 6q21-22. Contains pseudogene similar to zinc finger protein (ZPR1), EST, STS, GSS, complete sequence.
2.0e-23:287:71
Z95118
F-MAMMA1001957
Drosophila melanogaster, chromosome 2L, region 21C5-21D1, P1 clone DS07610, complete sequence.
1. 5e-14:192:66
AC004573
F-MAMMA1001978
Human immunoglobulin S(u) like sequence.
0.60:150:66
X15517
F-MAMMA1002070
Human PAC clone DJ515N1 from 22q11.2-q22, complete sequence.
3.9e-116:250:93
AC002073
F-MAMMA1002080
Mus musculus chromosome 11, clone mCIT.268_P_23, complete sequence.
1.1e-59:493:78
AC004807
F-MAMMA1002087
HS-1047-B2-A09-MR.abi CIT Human Genomic Sperm Library C Homo sapiens genomic clone Plate=CT 830 Col=18 Row=B, genomic survey sequence.
2.1e-31:174:98
B38457
F-MAMMA1002091
Homo sapiens CD39L2 (CD39L2) mRNA, complete cds.
1. 6e-156: 743: 98
AF039916
F-MAMMA1002095
Rat alternatively spliced mRNA.
4.9e-126:691:91
M93017
F-MAMMA1002128
Mus musculus C2C12 unknown mRNA, partial cds.
5.0e-41:353:77
U31629
F-MAMMA1002142
F-MAMMA1002165
Homo sapiens connective tissue growth factor related protein WISP-2 (WISP2) mRNA, complete cds.
1.2e-34:219:90
AF100780
F-MAMMA1002205
Homo Sapiens Chromosome X clone bWXD691, complete sequence.
8.1e-33:535:67
AC004386
F-MAMMA1002224
Human DNA sequence *** SEQUENCING IN PROGRESS *** from clone 229A8, WORKING
DRAFT SEQUENCE.
1.2e-31:274:82
Z86090
F-MAMMA1002234
Canine mRNA for 68kDA subunit of signal recognition particle (SRP68).
9.8e-145:736:91
X53744
F-MAMMA1002586
Drosophila melanogaster cosmid clone 86E4.
0.0071:306:58
AL021086
F-MAMMA1002633
Human DNA sequence *** SEQUENCING IN PROGRESS *** from clone 850H21, WORKING
DRAFT SEQUENCE.
3.9e-33:297:79
AL031680
F-MAMMA1003126
Human Hpast (HPAST) mRNA, complete cds.
1.7e-82:801:74
AF001434
F-NT2RM1000407
Cloning vector pUC-GM-INT, complete sequence.
9.4e-141:673:98
AF025392
F-NT2RM1000462
, complete sequence.
1.5e-86:232:82
AC005300
F-NT2RM1000542
Mouse beta-galactosidase (BGAL) gene, complete cds.
4.4e-17:468:62
M57734
F-NT2RM1000580
Caenorhabditis elegans cosmid F09E5.
1.6e-08:352:61
U37429
F-NT2RM1000789
Homo sapiens mRNA for hTCF-4.
1.1e-94:299:92
Y11306
F-NT2RM1000855
Canis familiaris sec61 homologue mRNA, complete cds.
6.6e-110:671:87
M96629
F-NT2RM1000858
tricarboxylate carrier [rats, liver, mRNA Partial, 2986 nt].
2.0e-65:716:70
S70011
F-NT2RM1000899
S.pombe chromosome I cosmid c8C9.
0.0010:300:59
Z99168
F-NT2RM2000241
Homo sapiens chromosome 12p13.3 clone RPCI11-350L7, WORKING DRAFT SEQUENCE, 72 unordered pieces.
0.99:201:65
AC005844
F-NT2RM2000306
Homo sapiens DNA sequence from PAC 257120 on chromosome 22q13.1-13.2. Contains cytochrome P450 pseudogenes CYP2D7P, CYP2D8P, CYP2D6(D), TCF20, NADH ubiquinone oxidoreductase B14 subunit, ESTs, CA repeat, STS, GSS.
1.1e-142:595:97
AL021878
F-NT2RM2000410
S.gregaria Abd-B gene.
0.076:172:66
X69161
F-NT2RM2000423
Arthrobacter sp. beta-galactosidase gene, complete cds.
4.2e-06:606:57
U78028
F-NT2RM2000497
Homo sapiens chromosome 17, clone hRPK.215_P_18, complete sequence.
1.2e-55:285:81
AC005969
F-NT2RM2000514
F-NT2RM2000565
Caenorhabditis elegans cosmid F28C6, complete sequence.
4.2e-18:539:62
Z68315
F-NT2RM2000582
P. zebra microsatellite locus DNA, 429bp.
0.00015:160:69
X99784
F-NT2RM2000589
Bos taurus myosin X, complete cds.
3.4e-139:817:88
U55042
F-NT2RM2000622
H. sapiens MFH-1 gene.
0.0010:466:57
Y08223
F-NT2RM2000632
Homo sapiens mRNA for TBP-associated factor 170 (TAFII170).
0.0052:331:59
AJ001017
F-NT2RM2000773
Oryctolagus cuniculus serum amyloid A-activating factor SAF-8 mRNA, partial cds.
2.9e-91:496:93
AF076786
F-NT2RM2001126
Homo sapiens multi PDZ domain protein MUPP1 (MUPP1) mRNA, complete cds. 1.6e-161:663:99
AF093419
F-NT2RM2001558
Homo sapiens protein kinase A binding protein AKAP110 mRNA, complete cds. 1.2e-164:770:98
AF093408
F-NT2RM2001626
F.rubripes GSS sequence, clone 060E22bA4, genomic survey sequence.
4.5e-46:606:68
Z88651
F-NT2RM2001643
HS-1053-B2-E09-MR.abi CIT Human Genomic Sperm Library C Homo sapiens genomic clone Plate=CT 775 Col=18 Row=J, genomic survey sequence.
2.5e-06:181:66
B41504
F-NT2RM2001738
S.capreolus ard2 gene and orf2, orf4 and orf5.
0.41:273:63
Y11036
F-NT2RM2001767
Human DNA sequence *** SEQUENCING IN PROGRESS *** from clone 50024, WORKING
DRAFT SEQUENCE.
8.0e-18:130:92
AL034380
F-NT2RM2001792
Homo sapiens mRNA for serum lectin P35, complete cds.
2.5e-12:244:67
D49353
F-NT2RM2001818
F-NT2RM2001902
Drosophila melanogaster mRNA for p21 activated kinase related protein. 7.2e-74:683:75
AJ011578
F-NT2RM2001939
Human G protein-coupled receptor GPR-NGA gene, complete cds.
1.4e-140:702:96
U55312
F-NT2RM2001941
Human gene for muscarinic acetylcholine receptor HM1.
6.3e-20:488:62
X15263
F-NT2RM4000100
Homo sapiens PAC clone DJ044L15 from Xq23, complete sequence.
7.7e-25:162:74
AC004827
F-NT2RM4000115
F-NT2RM4000198
F-NT2RM4000284
Human IgG Fc receptor hFcRn mRNA, complete cds.
7.3e-37:194:98
U12255
F-NT2RM4000295
Streptomyces chrysomallus actinomycin synthetase II (acmB) gene, complete cds. 1.6e-05:642:59
AF047717
F-NT2RM4000326
Homo sapiens complete genomic sequence between D16S3070 and D16S3275, containing Familial Mediterranean Fever gene disease.
1.0e-127:340:92
AJ003147
F-NT2RM4000417
Oncorhynchus kisutch microsatellite OKi20 DNA.
0.44:144:66
AF055444
F-NT2RM4000444
S.salar mRNA for transport-associated protein Tap2A.
1.7e-27:577:62
Z83328
F-NT2RM4000587
Homo sapines chromosome 19, cosmid R28058, complete sequence.
7.7e-16:388:64
AC005615
F-NT2RM4000593
F-NT2RM4000648
M. musculus mRNA for K-glypican.
1.4e-50:610:70
X83577
F-NT2RM4000761
Human mitochondrial DNA, fragment M1, encoding transfer RNAs, cytochrome oxidase I, and 2 URFs.
4.8e-167:787:98
M10546
F-NT2RM4000965
S.scrofa mRNA for calcium release channel (CRC).
0.044:356:60
X62880
F-NT2RM4000997
F-NT2RM4001321
HS-1053-B2-E09-MR.abi CIT Human Genomic Sperm Library C Homo sapiens genomic clone Plate=CT 775 Col=18 Row=J, genomic survey sequence.
1.3e-06:181:67
B41504
F-NT2RM4001325
Homo sapiens mRNA for chondroitin 6-sulfotransferase, complete cds.
6.6e-12:384:64
AB012192
F-NT2RM4001377
Homo sapiens mRNA for KIAA0638 protein, partial cds.
9.7e-155:719:99
AB014538
F-NT2RM4001735
Human DNA sequence *** SEQUENCING IN PROGRESS *** from clone 483K16, WORKING
DRAFT SEQUENCE.
1.3e-162:679:96
AL034374
F-NT2RM4001768
Human HepG2 partial cDNA, clone hmd3a07m5.
2.7e-52:271:98
D17020
F-NT2RM4001843
F-NT2RM4002352
Homo sapiens hLRp105 mRNA for LDL receptor related protein 105, complete cds.
1. 4e-155: 761 : 97
AB009462
F-NT2RP1000002
Mouse cAMP-dependent protein kinase beta subunit gene, exon 1.
1.7e-06:252:65
M21096
F-NT2RP1000050
Human HepG2 partial cDNA, clone hmd3g02m5.
7.1e-18:115:97
D17047
F-NT2RP1000181
Homo sapiens Chromosome 11q12.2 PAC clone pDJ519o13 containing human gene for ferritin heavy chain (FTH), complete sequence.
4.2e-139:427:98
AC004228
F-NT2RP1000239
Homo sapiens clone DT1P1E11 mRNA, CAG repeat region.
1.4e-90:524:91
U92989
F-NT2RP1000261
Homo sapiens hPMS1 gene, promoter region and exon 1.
2.5e-14:132:85
AB006462
F-NT2RP1000271
Homo sapiens DNA-binding protein mRNA, complete cds.
4.3e-139:678:97
AF038951
F-NT2RP1000300
Homo sapiens, complete sequence.
0.012:146:69
AC005854
F-NT2RP1000325
H. sapiens gene for phosphate carrier.
4.2e-110:438:98
X77337
F-NT2RP1000448
Streptomyces coelicolor cosmid 1A6.
0.79:209:61
AL023496
F-NT2RP1000465
Mus musculus nuclear protein NIP45 mRNA, complete cds.
2.2e-29:489:68
U76759
F-NT2RP1000468
Human DNA sequence *** SEQUENCING IN PROGRESS *** from clone 537K23, WORKING
DRAFT SEQUENCE.
1.6e-49:306:91
AL034405
F-NT2RP1000551
Homo sapiens putative interferon-related protein (SM15) mRNA, partial cds. 7.5e-139:742:93
U09585
F-NT2RP1000579
Human succinate dehydrogenase flavoprotein subunit (SDH) mRNA, complete cds. 3.6e-140:798:91
L21936
F-NT2RP1000613
Sequence 1 from patent US 5589579.
8.1e-10:468:58
I32995
F-NT2RP1000679
Homo sapiens chromosome 17, clone hRPC.4_G_17, complete sequence.
1.3e-112:448:89
AC003688
F-NT2RP1000740
H.sapiens CpG island DNA genomic Mse1 fragment, clone 34a2, reverse read cpg34a2.rt1a.
9.3e-14:211:73
Z60772
F-NT2RP1000903
HS_2256_B1_E10_MF CIT Approved Human Genomic Sperm Library D Homo sapiens genomic clone Plate=2256 Col=19 Row=J, genomic survey sequence.
9.0e-21:197:84
AQ084622
F-NT2RP1000981
F-NT2RP1001004
Danio rerio mRNA for MINDIN2, complete cds.
4.1e-22:472:63
AB006085
F-NT2RP1001020
Mus musculus clone OST66, genomic survey sequence.
1.5e-47:352:81
AF046696
F-NT2RP1001031
CIT-HSP-2330P23.TR CIT-HSP Homo sapiens genomic clone 2330P23, genomic survey sequence.
8.0e-26:145:99
AQ035969
F-NT2RP1001563
Homo sapiens clone DJ1125K23, WORKING DRAFT SEQUENCE, 21 unordered pieces. 0.096:405:59
AC004971
F-NT2RP2000092
Human zinc finger protein ZNF136.
1.8e-54:652:70
U09367
F-NT2RP2000178
Streptomyces coelicolor cosmid 3F9.
0.92:217:64
AL023862
F-NT2RP2000240
Homo sapiens chromosome 16 BAC clone CIT987SK-334D11 complete sequence. 2.9e-96:534:90
AF001550
F-NT2RP2000394
Gallus gallus p52 pro-apototic protein mRNA, complete cds.
2.9e-19:380:65
AF029071
F-NT2RP2000447
Homo sapiens clone DJ1129D05, complete sequence.
1.3e-109:289:98
AC005630
F-NT2RP2000479
Human DNA sequence from PAC 130G2 on chromosome 6p22.2-22.3. Contains ribosomal protein L29 pseudogene, ESTs and STSs.
0.0039:219:63
AL008627
F-NT2RP2000514
Homo sapiens roundabout 2 (robo2) mRNA, partial cds.
3.7e-89:461:95
AF040991
F-NT2RP2000533
Mus musculus cornichon mRNA, complete cds.
1.4e-113:677:89
AF022811
F-NT2RP2000610
Human DNA sequence *** SEQUENCING IN PROGRESS *** from clone 537K23, WORKING
DRAFT SEQUENCE.
4.3e-25:177:89
AL034405
F-NT2RP2000616
RPCI11-75J11.TK RPCI11 Homo sapiens genomic clone R-75J11, genomic survey sequence.
8.4e-34:135:91
AQ268877
F-NT2RP2000649
Homo sapiens CAAX prenyl protease (STE24) mRNA, complete cds.
1.2e-165:802:97
AF064867
F-NT2RP2000663
Human DNA sequence from cosmid U61B11, between markers DXS366 and DXS87 on chromosome X contains ESTs.
1.6e-106:365:97
Z73913
F-NT2RP2000694
Homo sapiens 5T4 oncofetal trophoblast glycoprotein gene.
4.2e-112:561:96
AJ012159
F-NT2RP2000712
HS_3071_A2_D05_MR CIT Approved Human Genomic Sperm Library D Homo sapiens genomic clone Plate=3071 Col=10 Row=G, genomic survey sequence. 7.6e-78:389:97
AQ166085
F-NT2RP2000739
Human mRNA for KIAA0326 gene, partial cds.
6.4e-24:574:62
AB002324
F-NT2RP2000818
Drosophila melanogaster, chromosome 2R, region 38A5-38B4, BAC clone BACR48M05, complete sequence.
0.00047:304:61
AC005719
F-NT2RP2000903
Homo sapiens 5T4 oncofetal trophoblast glycoprotein gene.
2.6e-110:541:97
AJ012159
F-NT2RP2001200
Homo sapiens mRNA for KIAA0676 protein, partial cds.
3.3e-110:540:96
AB014576
F-NT2RP2001223
HS-1054-B2-C02-MR.abi CIT Human Genomic Sperm Library C Homo sapiens genomic clone Plate=CT 776 Col=4 Row=F, genomic survey sequence.
7.2e-10:128:77
B41982
F-NT2RP2001276
Mouse regulatory protein (npdc-1) mRNA, complete cds.
1.2e-38:296:81
L03814
F-NT2RP2001388
RPCI11-30G23.TV RPCI-11 Homo sapiens genomic clone RPCI-11-30G23, genomic survey sequence.
0.32:53:94
B87787
F-NT2RP2001469
M.musculus tex292 mRNA (5' region).
4.2e-10:120:83
X80434
F-NT2RP2001480
Homo sapiens thrombospondin 3 (THBS3) gene, complete cds.
9.0e-140:686:96
L38969
F-NT2RP2001495
Human transporter protein (g17) mRNA, complete cds.
1.9e-35:581:64
U49082
F-NT2RP2001514
Drosophila melanogaster DNA sequence (P1 DS06882 (D310)), complete sequence. 3.7e-22:475:62
AC005115
F-NT2RP2001529
Homo sapiens mRNA for ZIP-kinase, complete cds.
4.6e-152:757:96
AB007144
F-NT2RP2001538
Sequence 11 from patent US 5624818.
1.4e-88:528:88
I41141
F-NT2RP2001562
Homo sapiens GLE1 (GLE1) mRNA, complete cds.
2.3e-117:572:97
AF058922
F-NT2RP2001662
Human DNA sequence *** SEQUENCING IN PROGRESS *** from clone 460J8, WORKING
DRAFT SEQUENCE.
6.1e-107:365:91
AL031662
F-NT2RP2001755
Sequence 9 from patent US 5750502.
1. 5e-53:518:75
AR007441
F-NT2RP2001769
A. sativa Aspk11 mRNA.
   4.7e-17:537:60
X79992
F-NT2RP2001817
Candida albicans SIR2 (SIR2) gene, complete cds.
4.6e-10:285:61
AF045774
F-NT2RP2001878
Mus musculus repeat element upstream of the Rasgrf1/Cdc25Mm gene.
5.0e-06:554:60
AF021791
F-NT2RP2001903
M.musculus mRNA for m-calpain.
3.1e-06:337:60
Y10139
F-NT2RP2001915
Homo sapiens chromosome 17, clone hRPK.63_A_1, complete sequence.
6.8e-28:488:65
AC005670
F-NT2RP2001921
Homo sapiens clone NH0332L11, complete sequence.
1.1e-77:148:99
AC005538
F-NT2RP2001948
Sequence 2 from patent US 5541311.
0.59:284:57
124091
F-NT2RP2001956
Feline c-sis proto-oncogene, segment 4.
0.99:101:69
M25356
F-NT2RP2002015
HS-1053-B2-E09-MR.abi CIT Human Genomic Sperm Library C Homo sapiens genomic clone Plate=CT 775 Col=18 Row=J, genomic survey sequence.
3.0e-06:181:65
B41504
F-NT2RP2002063
Homo sapiens chromosome 4 clone B207D4 map 4q25, complete sequence.
1.3e-108:418:94
AC004050
F-NT2RP2002188
Rattus norvegicus neuroligin 3 mRNA, complete cds.
1.0e-125:700:90
U41663
F-NT2RP2002232
F-NT2RP2002304
Human FMR1 gene, 5' end.
0.12:93:67
L19476
F-NT2RP2002409
Myxococcus xanthus response regulator FrzZ (frzZ) gene, partial cds; alanine dehydrogenase (aldA), putative ECF sigma factor RpoE1 (rpoE1), and response regulator homolog (frzS) genes, complete cds; and unknown genes.
9.0e-10:553:59
AF049107
F-NT2RP2002510
Mus musculus (129SV) DNA, unmapped BAC 10817, complete sequence.
4.2e-27:573:62
AC004093
F-NT2RP2002527
Homo sapiens Chromosome 11q12.2 PAC clone pDJ519o13 containing human gene for ferritin heavy chain (FTH), complete sequence.
3.2e-110:439:99
AC004228
F-NT2RP2002533
Homo sapiens putative tumor suppressor gene 26 protein alpha 2 delta calcium channel subunit mRNA, complete cds.
6.4e-141:726:95
AF040709
F-NT2RP2002564
Homo sapiens PAC clone DJ0979P20 from 7q33-q35, complete sequence.
2.6e-112:403:98
AC004941
F-NT2RP2002674
HS_3122_B2_A02_MR CIT Approved Human Genomic Sperm Library D Homo sapiens genomic clone Plate=3122 Col=4 Row=B, genomic survey sequence.
4.8e-13:86:100
AQ182907
F-NT2RP2002721
F-NT2RP2002824
Arabidopsis thaliana BAC T19D16 genomic sequence.
1.3e-12:135:69
U95973
F-NT2RP2002942
Homo sapiens mRNA for KIAA0806 protein, complete cds.
6.1e-145:758:94
AB018349
F-NT2RP2002974
Mus musculus mRNA for Six5, partial cds.
8.0e-84:588:82
D83146
F-NT2RP2002976
H.sapiens gene for phospholipase C beta 3, exon 14.
0.93:210:61
Z37557
F-NT2RP2003042
G.gallus mRNA for lecithin-cholesterol acyltransferase.
9.1e-26:462:65
X91011
F-NT2RP2003138
Human DNA sequence *** SEQUENCING IN PROGRESS *** from clone 460J8, WORKING
DRAFT SEQUENCE.
3.9e-142:702:96
AL031662
F-NT2RP2003179
Homo sapiens mRNA for KIAA0537 protein, complete cds.
3.3e-42:587:70
AB011109
F-NT2RP2003210
Mus musculus fatty acid transport protein 4 mRNA, partial cds.
2.6e-112:726:85
AF072759
F-NT2RP2003302
Human zinc finger protein ZNF136.
5.5e-63:691:69
U09367
F-NT2RP2003369
Homo sapiens chromosome 7q22 sequence, complete sequence.
2.0e-49:249:95
AF053356
F-NT2RP2003383
Homo sapiens chromosome 1 atrophin-1 related protein (DRPLA) mRNA, complete cds.
1.5e-159:817:95
AF016005
F-NT2RP2003390
Homo sapiens SEC63 (SEC63) mRNA, complete cds.
7.0e-115:554:98
AF100141
F-NT2RP2003469
Genomic sequence from Human 9q34, complete sequence.
5.6e-38:210:97
AC001644
F-NT2RP2003545
Homo sapiens STE20-like kinase 3 (mst-3) mRNA, complete cds.
2.2e-48:579:71
AF024636
F-NT2RP2003593
Human DNA sequence *** SEOUENCING IN PROGRESS *** from clone 971N18, WORKING
DRAFT SEQUENCE.
1.8e-90:326:99
AL021396
F-NT2RP2003599
F-NT2RP2003655
M. musculus tex261 mRNA.
5.3e-77:513:85
X81058
F-NT2RP2003664
Homo sapiens mRNA for leptin receptor gene-related protein.
1.7e-132:630:98
Y12670
F-NT2RP2003931
Homo sapiens chromosome 19, overlapping cosmids R27918 and R33775, complete sequence.
1. 3e-114:411 :97
AC004447
F-NT2RP2003940
Human ZNF43 mRNA.
1.4e-97:693:82
X59244
F-NT2RP2003950
Sequence 1 from patent US 5648238.
6.9e-13:143:79
I55887
F-NT2RP2004069
F-NT2RP2004108
Human zinc finger protein ZNF136.
1.5e-67:548:78
U09367
F-NT2RP2004141
Rattus norvegicus leukocyte common antigen receptor (LAR) gene, trans-spliced alternative untranslated exon.
8.0e-10:487:62
U87960
F-NT2RP2004179
Homo sapiens chromosome 11 from 11p15.5 region, complete sequence. 0.56:600:57
AF015416
F-NT2RP2004205
Homo sapiens chromosome 16, cosmid clone RT81 (LANL), complete sequence. 0.32:431:55
AC005356
F-NT2RP2004447
Homo sapiens Chromosome 11q13 BAC Clone 18h3, WORKING DRAFT SEQUENCE, 7 ordered pieces.
2.0e-23:252:79
AC000353
F-NT2RP2004495
Human transporter protein (g17) mRNA, complete cds.
3.6e-25:497:61
U49082
F-NT2RP2004524
Genomic sequence from Human 9q34, complete sequence.
5.9e-60:203:98
AC001644
F-NT2RP2004556
HS_3022_A1_A11_T7 CIT Approved Human Genomic Sperm Library D Homo sapiens genomic clone Plate=3022 Col=21 Row=A, genomic survey sequence.
1. 3e-51 :419:79
AQ119143
F-NT2RP2004606
cDNA encoding NIC(Natural Inhibitor of Collagenase).
1. 2e-113:617:92
E00985
F-NT2RP2004648
Felis catus lysosomal beta-galactosidase (Bgal) mRNA, complete cds.
1.5e-15:403:64
AF006749
F-NT2RP2004670
Rattus norvegicus vesicla-associate calmodulin-binding protein mRNA, complete cds.
1.1e-73:493:85
L22557
F-NT2RP2004794
Mus musculus mRNA for B-IND1 protein.
5.6e-12:109:86
Z97207
F-NT2RP2004837
Homo sapiens clone NH0001P09, WORKING DRAFT SEQUENCE, 1 unordered pieces. 2.8e-39:352:78
AC006030
F-NT2RP2004847
CIT-HSP-2357D24.TR CIT-HSP Homo sapiens genomic clone 2357D24, genomic survey sequence.
2.5e-35:196:96
AQ074738
F-NT2RP2005027
Human glucose transporter-like protein-III (GLUT3), complete cds.
2.2e-145:713:96
M20681
F-NT2RP2005069
Rat vacuolar protein sorting homolog r-vps33b mRNA, complete cds.
9.4e-51:200:90
U35245
F-NT2RP2005163
Mouse DNA fragment that hybridizes to HSV-1 SmaI A fragment.
1.4e-08:231:67
M11041
F-NT2RP2005181
Mus musculus cationic amino acid transporter (CAT3) mRNA, complete cds. 1.6e-96:575:85
U70859
F-NT2RP2005247
Mus musculus ret finger protein mRNA, complete cds.
1.8e-13:310:66
L46855
F-NT2RP2005378
RPCI11-21D23.TP RPCI-11 Homo sapiens genomic clone RPCI-11-21D23, genomic survey sequence.
3.0e-12:131:80
B85846
F-NT2RP2005391
S.muris mRNA for microneme antigen.
2.5e-10:345:61
Z26947
F-NT2RP2005425
Homo sapiens mRNA for KIAA0803 protein, partial cds.
1.0e-116:566:97
AB018346
F-NT2RP2005463
F-NT2RP2005514
F-NT2RP2005535
Homo sapiens DNA-binding protein mRNA, complete cds.
2.3e-125:726:90
AF038951
F-NT2RP2005541
CIT-HSP-2386E2.TF.1 CIT-HSP Homo sapiens genomic clone 2386E2, genomic survey sequence.
6.2e-20:152:88
AQ240341
F-NT2RP2005597
D.melanogaster mRNA for rotated abdomen protein.
0.088:270:57
X95956
F-NT2RP2005632
Gallus gallus chicken brain factor-2 (CBF-2) mRNA, complete cds.
2.0e-07:207:67
U47276
F-NT2RP2005666
Homo sapiens chromosome 11 clone CIT-HSP-1337H24, WORKING DRAFT SEQUENCE, 9 unordered pieces.
1.0:328:57
AC005849
F-NT2RP2005774
Human zinc finger protein ZNF136.
4.0e-44:451:74
U09367
F-NT2RP2005878
Mus musculus putative steroid dehydrogenase (KIK-I) mRNA, complete cds. 5.1e-16:382:63
AF064635
F-NT2RP2005883
Human DNA sequence from clone 248E1 on chromosome 6q23.1-23.3 Contains DOPAMINE-BETA-MONOOXYGENASE PRECURSOR, EF-1-ALPHA-2 pseudogene EST GSS and CA repeat, complete sequence.
1.5e-30:191:95
AL023578
F-NT2RP2005887
Homo sapiens clone NH0001P09, WORKING DRAFT SEQUENCE, 1 unordered pieces. 1.8e-50:394:79
AC006030
F-NT2RP2005941
Human DNA sequence from cosmid CFAT5, chromosome region 11p13 contains PAX6 exons 1-4, EST and CpG Islands.
9.5e-93:468:96
Z95332
F-NT2RP2005994
Homo sapiens chromosome 4 clone B207D4 map 4q25, complete sequence.
1.6e-139:692:96
AC004050
F-NT2RP2006004
CIT-HSP-2366J21.TF CIT-HSP Homo sapiens genomic clone 2366J21, genomic survey sequence.
6.6e-39:206:98
AQ080257
F-NT2RP2006042
Human mRNA for KIAA0144 gene, complete cds.
1.7e-10:220:69
D63478
F-NT2RP2006092
Homo sapiens chromosome 5, BAC clone 319C17 (LBNL H159), complete sequence. 3.6e-121:562:82
AC005214
F-NT2RP2006099
Human Chromosome 11 pac pDJ392a17, complete sequence.
8.7e-76:383:92
AC000385
F-NT2RP2006134
Homo sapiens Chromosome 22q11.2 Cosmid Clone 91c In DGCR Region, complete sequence.
0.055:125:71
AC000091
F-NT2RP2006269
D.melanogaster mRNA for rotated abdomen protein.
5.4e-05:357:58
X95956
F-NT2RP2006512
Sequence 1 from Patent EP 0285405.
3.7e-102:659:85
I05465
F-NT2RP3000011
RPCI11-43E12.TJ RFCI11 Homo sapiens genomic clone R-43E12, genomic survey sequence.
1.8e-10:113:84
AQ195722
F-NT2RP3000022
Human DNA sequence from clone 341E18 on chromosome 6p11.2-12.3. Contains a Serine/Threonine Protein Kinase gene (presumptive isolog of a Rat gene) and a novel alternatively spliced gene. Contains a putative CpG island, ESTs and GSSs, complete sequence.
6.7e-116:284:99
AL031178
F-NT2RP3000059
R.norvegicus mRNA for leucocyte common antigen-related protein (3941 bp). 0.0031:511:59
X83546
F-NT2RP3000063
Homo sapiens chromosome 19, fosmid 37502, complete sequence.
0.20:544:57
AC004755
F-NT2RP3000125
HS_3025_A1_D11_MR CIT Approved Human Genomic Sperm Library D Homo sapiens genomic clone Plate=3025 Col=21 Row=G, genomic survey sequence.
1.0e-21:161:88
AQ101452
F-NT2RP3000148
Human Chromosome 16 BAC clone CIT987SK-A-635H12, complete sequence.
5.2e-40:257:77
AC002310
F-NT2RP3000169
Homo sapiens MRS1 mRNA, complete cds.
3.4e-106:501:99
AF093239
F-NT2RP3000171
Mus musculus mRNA for B-IND1 protein.
1.8e-97:571:89
Z97207
F-NT2RP3000172
Rattus norvegicus vesicla-associate calmodulin-binding protein mRNA, complete cds.
2.0e-123:702:86
L22557
F-NT2RP3000201
Homo sapiens mRNA for KIAA0687 protein, partial cds.
9.2e-170:792:98
AB014587
F-NT2RP3000232
HS_3238_B2_D04_MR CIT Approved Human Genomic Sperm Library D Homo sapiens genomic clone Plate=3238 Col=8 Row=H, genomic survey sequence.
9.2e-24:174:88
AQ219879
F-NT2RP3000304
Homo sapiens LDL receptor-related protein 6 (LRP6) mRNA, complete cds. 3.3e-171:797:98
AF074264
F-NT2RP3000378
Mus musculus (clone pVZmSin3A9) mSin3A9 mRNA, complete cds.
5.8e-137:774:89
L38621
F-NT2RP3000427
Mouse cAMP-dependent protein kinase beta subunit gene, exon 1.
1.5e-18:390:65
M21096
F-NT2RP3000436
cDNA encoding a human novel protein disulfide isomerase like enzyme,EP52. 4.5e-05:353:59
E13330
F-NT2RP3000444
Human DNA sequence *** SEQUENCING IN PROGRESS *** from clone 117715, WORKING
DRAFT SEQUENCE.
9.7e-75:203:97
AL022315
F-NT2RP3000460
Canis familiaris sec61 homologue mRNA, complete cds.
7.1e-131:643:88
M96629
F-NT2RP3000481
Homo sapiens RanBP7/importin 7 mRNA, complete cds.
1.7e-162:770:98
AF098799
F-NT2RP3000616
Homo sapiens KIAA0405 mRNA, complete cds.
4.7e-31:579:62
AB007865
F-NT2RP3000645
Human chromosome 12p13 sequence, complete sequence.
5.9e-07:484:61
U47924
F-NT2RP3000652
Human ZNF43 mRNA.
4.4e-131:853:84
X59244
F-NT2RP3000676
Homo sapiens mRNA for KIAA0446 protein, complete cds.
2.7e-86:420:98
AB007915
F-NT2RP3000677
Human estrogen receptor-related protein (variant ER from breast cancer) mRNA, complete cds.
2.9e-21:125:100
M69296
F-NT2RP3000721
HS_2221_A2_C01_MF CIT Approved Human Genomic Sperm Library D Homo sapiens genomic clone Plate=2221 Col=2 Row=E, genomic survey sequence.
0.94:254:60
AQ253443
F-NT2RP3000789
Mus musculus coding region determinant binding protein mRNA, complete cds. 5.4e-139:827:87
AF061569
F-NT2RP3000818
Human DNA sequence *** SEQUENCING IN PROGRESS *** from clone 94M16, WORKING
DRAFT SEQUENCE.
3.0e-28:218:86
Z97201
F-NT2RP3000820
Mus musculus WSB-1 mRNA, complete cds.
1.1e-77:477:87
AF033186
F-NT2RP3000838
Homo sapiens mRNA for KIAA0638 protein, partial cds.
2.6e-77:682:79
AB014538
F-NT2RP3000871
Homo sapiens retinoblastoma-interacting protein (RBBP8) mRNA, complete cds. 5.8e-07:350:60
AF043431
F-NT2RP3000907
Drosophila melanogaster DNA sequence (P1 DS06882 (D310)), complete sequence. 1.7e-13:330:62
AC005115
F-NT2RP3000921
cDNA GA3-43 encoding novel polypeptide which appear when differentiate from embryo-tumor cell P19 to nerve cell.
6.8e-68:812:69
E12950
F-NT2RP3001012
cDNA encoding novel rat protein TIP120 which is formed of complex with TBP (TATA binding protein).
2.4e-129:692:92
E12829
F-NT2RP3001044
Homo sapiens clone NH0001P09, WORKING DRAFT SEQUENCE, 1 unordered pieces. 3.7e-60:393:79
AC006030
F-NT2RP3001061
F.rubripes GSS sequence, clone 154E17aC12, genomic survey sequence.
1.8e-07:239:62
AL018519
F-NT2RP3001159
Homo sapiens chromosome 11, BAC CIT-HSP-311e8 (BC269730) containing the hFEN1 gene, complete sequence.
4.4e-24:156:72
AC004770
F-NT2RP3001170
Homo sapiens mRNA for KIAA0784 protein, partial cds.
2.3e-181:859:98
AB018327
F-NT2RP3001195
Genomic sequence from Human 9q34, complete sequence.
3.8e-53:253:92
AC001644
F-NT2RP3001240
Canis familiaris sec61 homologue mRNA, complete cds.
1.4e-133:740:87
M96629
F-NT2RP3001271
Homo sapiens chromosome 19, cosmid F20237, complete sequence.
0.082:370:60
AC005775
F-NT2RP3001322
Homo sapiens mRNA for KIAA0566 protein, partial cds.
1.9e-38:728:63
AB011138
F-NT2RP3001388
Rattus norvegicus synaptotagmin XI mRNA, complete cds.
1.2e-103:701:83
AF000423
F-NT2RP3001542
Human Chromosome 11 Cosmid cSRL34e5, complete sequence.
8.6e-17:293:65
U73643
F-NT2RP3001560
Mouse mRNA for thymic epithelial cell surface antigen, complete cds.
7.8e-135:742:91
D67067
F-NT2RP3001592
Human Cdk-inhibitor p57KIP2 (KIP2) mRNA, complete cds.
7.2e-12:188:71
U22398
F-NT2RP3001650
Homo sapiens PAC clone DJ0722F20 from 7q31.1-q31.3, complete sequence. 1.9e-26:374:72
AC005281
F-NT2RP3001685
Homo sapiens 12q13.1 Cosmid C174F5 (Lawrence Livermore LL12NC01 or LL12NC02 human cosmid libraries) complete sequence.
4.6e-73:284:98
AC004550
F-NT2RP3001738
Homo sapiens chromosome 11, BAC CIT-HSP-311e8 (BC269730) containing the hFEN1 gene, complete sequence.
1.8e-21:186:67
AC004770
F-NT2RP3001754
Human DNA sequence *** SEQUENCING IN PROGRESS *** from clone 50024, WORKING
DRAFT SEQUENCE.
5.0e-21:131:96
AL034380
F-NT2RP3001858
Homo sapiens mRNA for KIAA0584 protein, partial cds.
5.9e-39:770:63
AB011156
F-NT2RP3001976
M.domesticus (C57BI/6J) mRNA for zinc finger protein 30.
2.0e-37:536:70
Z30174
F-NT2RP3002015
Homo sapiens huntingtin gene, partial exon.
0.024:175:65
L49359
F-NT2RP3002160
Homo sapiens chromosome 9q34, clone 70C11, complete sequence.
1.6e-95:249:91
AC002319
F-NT2RP3002281
Homo sapiens mRNA for KIAA0765 protein, partial cds.
1.6e-149:713:98
AB018308
F-NT2RP3002286
Mus musculus EGF repeat transmembrane protein mRNA, complete cds.
2.0e-136:756:92
U57368
F-NT2RP3002311
Mouse beta-galactosidase (BGAL) gene, complete cds.
1.0e-29:624:63
M57734
F-NT2RP3002324
Human DNA sequence from cosmid RJ14 from a contig from the tip of the short arm of chromosome 16, spanning 2Mb of 16p13.3. Contains ESTs and CpG island. 5.7e-122:655:93
Z69890
F-NT2RP3002342
Human transporter protein (g17) mRNA, complete cds.
9.8e-36:565:65
U49082
F-NT2RP3002353
Streptomyces phaeochromogenes plasmid pJV1, complete sequence.
0. 15:466:60
U23762
F-NT2RP3002409
Homo sapiens mRNA for KIAA0719 protein, complete cds.
2.0e-189:897:98
AB018262
F-NT2RP3002411
Mus musculus putative steroid dehydrogenase (KIK-I) mRNA, complete cds. 7.8e-122:796:84
AF064635
F-NT2RP3002448
R.norvegicus mRNA for leucocyte common antigen-related protein (3941 bp). 4.0e-11:403:64
X83546
F-NT2RP3002571
Bos taurus mRNA for lyncein.
8.7e-114:652:90
Y17923
F-NT2RP3002664
H.sapiens CpG island DNA genomic Mse1 fragment, clone 34a2, reverse read cpg34a2.rt1a.
6.1e-14:211:72
Z60772
F-NT2RP3002721
Homo sapiens citrate synthase mRNA, complete cds.
7.5e-179:873:96
AF047042
F-NT2RP3002737
Homo sapiens mRNA for HNSPC, complete cds.
1.4e-42:409:75
D82346
F-NT2RP3002738
Rattus norvegicus mRNA for Crk-associated substrate, p130, complete cds. 8.9e-122:812:83
D29766
F-NT2RP3002790
Human Cdk-inhibitor p57KIP2 (KIP2) mRNA, complete cds.
2.2e-15:626:62
U22398
F-NT2RP3002836
Homo sapiens mRNA for KIAA0463 protein, partial cds.
6.8e-152:717:99
AB007932
F-NT2RP3002887
R.norvegicus mRNA for leucocyte common antigen-related protein (3941 bp). 2.0e-05:491:59
X83546
F-NT2RP3002900
Cricetulus griseus COP-coated vesicle membrane protein CHOp24 mRNA, partial cds.
7.3e-13:327:66
U26264
F-NT2RP3002958
Mus musculus IgK chain (6S) intron with insertion/deletion mutations. 5.6e-22:403:66
L12153
F-NT2RP3002983
Homo sapiens genomic DNA, chromosome 21q11.1, segment 18/28, WORKING DRAFT
SEQUENCE.
1. 2e-118: 339: 99
AP000047
F-NT2RP3003000
Homo sapiens T-type calcium channel alpha-1 subunit mRNA, complete cds. 7.9e-88:555:88
AF051946
F-NT2RP3003076
Streptomyces coelicolor cosmid 2A11.
0.15:505:59
AL031184
F-NT2RP3003354
Homo sapiens secretory carrier membrane protein (SCAMP2) mRNA, complete cds. 1.2e-34:625:64
AF005038
F-NT2RP3003448
CIT-HSP-721P7.TV CIT-HSP Homo sapiens genomic clone 721P7, genomic survey sequence.
1. 2e-16:126:89
B50017
F-NT2RP3003469
Homo sapiens chromosome 19, cosmid F23990, complete sequence.
2.0e-18:126:94
AC005262
F-NT2RP3003473
Homo sapiens chromosome 17, clone hRPK.1003_J_3, complete sequence.
7.1e-68:474:71
AC005181
F-NT2RP3003527
Homo sapiens mRNA for protein kinase Dyrk1B.
1.4e-160:769:98
Y17999
F-NT2RP3003532
Mus musculus cell surface molecule 0X-2 mRNA, complete cds.
1.3e-96:712:80
AF004023
F-NT2RP3003535
Drosophila melanogaster (P1 DS02368 (D205)) DNA sequence, complete sequence. 0.027:155:65
AC004313
F-NT2RP3003559
H.sapiens CpG island DNA genomic Mse1 fragment, clone 171h5, reverse read cpg171h5.rt1a.
3.9e-50:261:97
Z59762
F-NT2RP3003614
Mus musculus semaphorin Vla mRNA, complete cds.
1. 7e-131:811:86
AF030430
F-NT2RP3003729
Homo sapiens chromosome 10 clone LA10NC01_15_E_11 map 10q26.3, WORKING DRAFT SEQUENCE, 3 unordered pieces.
1.4e-97:259:91
AC006171
F-NT2RP3003849
F-NT2RP3003874
M.musculus mRNA for myosin I heavy chain.
2.9e-151:863:89
X69987
F-NT2RP3003939
T24C19TF TAMU Arabidopsis thaliana genomic clone T24C19, genomic survey sequence.
1. 4e-19 : 293 : 68
B29025
F-NT2RP3003963
CIT-HSP-2050C19.TF CIT-HSP Homo sapiens genomic clone 2050C19, genomic survey sequence.
1.3e-16:111:95
B80539
F-NT2RP3004000
Homo sapiens klotho gene, exon 1.
0.042:430:60
AB009666
F-NT2RP3004025
Human DNA sequence from Fosmid 49D8 on chromosome 22, complete sequence.
0. 062:197:65
Z82186
F-NT2RP3004067
Human mRNA for KIAA0375 gene, complete cds.
1.7e-33:556:66
AB002373
F-NT2RP3004075
jd187 Trypanosome Shotgun M13 genomic Trypanosoma brucei brucei genomic clone 5H9, genomic survey sequence.
1.5e-12:438:61
B13419
F-NT2RP3004083
F-NT2RP3004090
Dog alpha-L-iduronidase (IDUA) gene, exons 7-12.
1.4e-06:469:60
L01060
F-NT2RP3004119
Human mRNA for KIAA0215 gene, complete cds.
1.3e-72:640:75
D86969
F-NT2RP3004130
F-NT2RP3004133
Pseudomonas aeruginosa phage phi CTX DNA, complete genome.
0.0018:421:60
Y13918
F-NT2RP3004202
F-NT2RP3004294
Xenopus laevis ER1 mRNA, complete cds.
5.0e-77:335:78
AF015454
F-NT2RP3004309
Homo sapiens chromosome 11, BAC CIT-HSP-311e8 (BC269730) containing the hFEN1 gene, complete sequence.
9.6e-25:231:65
AC004770
F-NT2RP3004321
Homo sapiens chromosome 11 from 11p15.5 region, complete sequence.
3.7e-80:279:95
AF015416
F-NT2RP3004345
Human Cdk-inhibitor p57KIP2 (KIP2) mRNA, complete cds.
7.2e-12:188:71
U22398
F-NT2RP3004355
HS_3212_A1_C08_MR CIT Approved Human Genomic Sperm Library D Homo sapiens genomic clone Plate=3212 Col=15 Row=E, genomic survey sequence.
0.061:266:65
AQ176625
F-NT2RP3004374
HS-1053-B2-E09-MR.abi CIT Human Genomic Sperm Library C Homo sapiens genomic clone Plate=CT 775 Col=18 Row=J, genomic survey sequence.
1.3e-06:181:67
B41504
F-NT2RP3004406
CIT-HSP-2340N18.TF CIT-HSP Homo sapiens genomic clone 2340N18, genomic survey sequence.
9.9e-74:359:99
AQ058326
F-NT2RP3004481
Mus musculus bassoon gene, exon 6 to 11.
0.0060:528:59
Y17038
F-NT2RP3004552
Mouse mRNA for seizure-related gene product 6 type 2 precursor, complete cds. 7.6e-40:731:64
D64009
F-NT2RP3004557
Human Ki nuclear autoantigen mRNA, complete cds.
8.1e-120:626:94
U11292
F-NT2RP3004625
Homo sapiens 1-1 receptor candidate protein mRNA, complete cds.
9.8e-151:710:98
AF082516
F-NT2RP3004640
Bos taurus tuftelin mRNA, complete cds.
8.2e-104:565:87
AF105228
F-NT2RP3004647
Homo sapiens mRNA for KIAA0446 protein, complete cds.
2.1e-109:524:98
AB007915
F-NT2RP4000108
Human gene for neurofilament subunit NF-L.
7.0e-158:862:93
X05608
F-NT2RP4000634
Sequence 11 from patent US 5753446.
2. 9e-155:828:92
AR008281
F-NT2RP4000962
Mus musculus clone OST66, genomic survey sequence.
6.0e-48:352:81
AF046696
F-NT2RP4001001
Homo sapiens chromosome 5, Bac clone 58g14 (LBNL H76), complete sequence. 4.8e-47:360:84
AC005915
F-NT2RP4001009
Homo sapiens CAAX prenyl protease (STE24) mRNA, complete cds.
5. 9e-175:828:98
AF064867
F-NT2RP4001467
Human placental cDNA coding for 5' nucleotidase (EC 3.1.3.5).
3.3e-159:742:98
X55740
F-NT2RP4001877
1.7e-27:401:69
AC005637
F-NT2RP4001879
F-NT2RP4002187
Mus musculus putative steroid dehydrogenase (KIK-I) mRNA, complete cds. 4.2e-115:777:83
AF064635
F-NT2RP4002451
Human DNA sequence *** SEQUENCING IN PROGRESS *** from clone 111B22, WORKING
DRAFT SEQUENCE.
6.1e-86:452:96
Z98200
F-NT2RP4002715
Homo sapiens clone NH0523H20, complete sequence.
3.6e-59:410:77
AC005041
F-NT2RP4002750
Mus musculus cationic amino acid transporter (CAT3) mRNA, complete cds. 3.4e-105:586:87
U70859
F-OVARC1000003
B.taurus mRNA for sodium dependent phosphate transporter.
9.0e-125:823:83
X81699
F-OVARC1000090
RPCI11-25E14.TP RPCI-11 Homo sapiens genomic clone RPCI-11-25E14, genomic survey sequence.
1.9e-06:151:74
B86784
F-OVARC1000105
S.cerevisiae UBC6 gene.
4.6e-25:525:64
X73234
F-OVARC1000137
Human SNARE protein Ykt6 (YKT6) mRNA, complete cds.
1.2e-33:184:98
U95735
F-OVARC1000208
Homo sapiens Chromosome 16 BAC clone CIT987SK-A-761H5, complete sequence.
1. 7e-79:362:91
AC002544
F-OVARC1000255
Porcine protein-tyrosine kinase (syk) mRNA, complete cds.
4.9e-116:424:88
M73237
F-OVARC1000275
Homo sapiens chromosome 21q22.3, PAC clones 314N7, 225L15, BAC clone 7B7, complete sequence bases 1..333303.
0.32:314:61
AJ011930
F-OVARC1000298
Homo sapiens clone RG031N19, WORKING DRAFT SEQUENCE, 1 unordered pieces. 2.5e-121:306:98
AC005632
F-OVARC1000307
Bovine herpesvirus type 1 genes for UL[27,28,29,30,31].
0.017:162:67
X94677
F-OVARC1000313
Homo sapiens mRNA for KIAA0573 protein, partial cds.
1.7e-119:585:97
AB011145
F-OVARC1000331
Sequence 2 from patent US 5756332.
1.9e-48:290:91
AR009648
F-OVARC1000410
Homo sapiens mRNA for angiopoietin-like factor.
4.6e-26:538:62
Y16132
F-OVARC1000439
F-OVARC1000467
HS_3008_A2_D12_MR CIT Approved Human Genomic Sperm Library D Homo sapiens genomic clone Plate=3008 Col=24 Row=G, genomic survey sequence.
2.0e-11:132:82
AQ116995
F-OVARC1000529
HS_3092_B2_C11_MF CIT Approved Human Genomic Sperm Library D Homo sapiens genomic clone Plate=3092 Col=22 Row=F, genomic survey sequence.
8.2e-12:115:84
AQ127947
F-OVARC1000553
Homo sapiens chromosome 19, cosmid R26894, complete sequence.
6.5e-92:221:96
AC005594
F-OVARC1000775
Human chromosome 3p21.1 gene sequence.
6.9e-69:380:95
L13435
F-OVARC1000811
Homo sapiens chromosome 16, cosmid clone 432A1 (LANL), complete sequence. 6.7e-77:500:86
AC004235
F-OVARC1000853
HS_3234_A1_F05_MR CIT Approved Human Genomic Sperm Library D Homo sapiens genomic clone Plate=3234 Col=9 Row=K, genomic survey sequence.
4.6e-05:111:71
AQ191345
F-OVARC1000873
Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.
8.2e-42:234:96
AL034418
F-OVARC1000916
Sequence 3 from patent US 5674748.
2.0e-55:422:84
I68139
F-OVARC1000956
Human DNA sequence from cosmid L241B9, Huntington's Disease Region, chromosome 4p16.3 contains polymorphic VNTR pYNZ32.
1.2e-107:540:97
Z69708
F-OVARC1000995
H.sapiens genomic DNA (chromosome 3; clone NL1106D).
4.3e-28:166:95
X87478
F-OVARC1001030
Human mRNA for KIAA0339 gene, complete cds.
2.1e-10:334:64
AB002337
F-OVARC1001049
*** SEQUENCING IN PROGRESS *** Homo sapiens chromosome 4, BAC clone C0190L06; HTGS phase 1, WORKING DRAFT SEQUENCE, 21 unordered pieces.
5.4e-12:420:62
AC004670
F-OVARC1001086
Homo sapiens cyclin T2a mRNA, complete cds.
1.9e-164:761:99
AF048731
F-OVARC1001132
Homo sapiens genomic DNA, chromosome 21q11.1, segment 9/28, WORKING DRAFT
SEQUENCE.
1.5e-89:328:75
AP000038
F-OVARC1001163
Caenorhabditis elegans cosmid F40E10, complete sequence.
3.8e-26:337:71
Z69792
F-OVARC1001222
CIT-HSP-2010I15.TR CIT-HSP Homo sapiens genomic clone 2010115, genomic survey sequence.
1.2e-08:171:70
B57734
F-OVARC1001260
F-OVARC1001336
B.taurus mRNA for sodium dependent phosphate transporter.
5.4e-83:622:80
X81699
F-OVARC1001338
HS_2181_B2_E11_MF CIT Approved Human Genomic Sperm Library D Homo sapiens genomic clone Plate=2181 Col=22 Row=J, genomic survey sequence.
2.3e-17:144:86
AQ022764
F-OVARC1001569
Mus musculus bone morphogenetic factor 11 (Bmp11) gene, exon 1.
2.9e-06:241:63
AF100904
F-OVARC1001570
Homo sapiens *** SEQUENCING IN PROGRESS ***, WORKING DRAFT SEQUENCE.
1.6e-10:235:64
AJ011929
F-OVARC1001596
Homo sapiens chromosome 17, clone hRPK.372_K_20, complete sequence.
2.2e-45:498:73
AC005951
F-OVARC1001607
Human beta-1,2-N-acetylglucosaminyltransferase II (MGAT2) gene, complete cds. 1.7e-38:323:80
U15128
F-OVARC1001725
Homo sapiens patched related protein TRC8 (TRC8) gene, exon 1 and partial cds. 3.5e-172:821:98
AF064800
F-OVARC1001727
Human DNA sequence from clone 48A11 on chromosome 20p12 Contains EST, STS, GSS, complete sequence.
2.2e-132:633:98
AL031132
F-OVARC1001807
Human TR3 orphan receptor mRNA, complete cds.
7.1e-90:566:87
L13740
F-OVARC1001833
Rattus norvegicus cis-Golgi matrix protein GM130 mRNA, complete cds. 5.2e-46:364:79
U35022
F-OVARC1001952
Homo sapiens FGFR-4 gene.
1.7e-14:392:62
Y13901
F-OVARC1001991
Human Chromosome 11 Cosmid cSRL34e5, complete sequence.
2.3e-06:298:64
U73643
F-OVARC1002058
, complete sequence.
1.3e-108:617:92
AC005500
F-OVARC1002178
Herpes simplex virus type 2 (strain HG52), complete genome.
0.43:234:63
Z86099
F-PLACE1000033
Mus musculus otogelin mRNA, complete cds.
5. 9e-18:579:59
U96411
F-PLACE1000231
Rattus norvegicus WAP four-disulfide core domain protein (ps20) mRNA, complete cds.
1.1e-18:273:68
AF037272
F-PLACE1000258
Human KRAB zinc finger protein (ZNF177) mRNA, complete cds.
1.2e-13:241:70
U37263
F-PLACE1000442
Human zinc finger protein ZNF136.
2.3e-87:774:76
U09367
F-PLACE1000560
Homo sapiens chromosome 5, BAC clone 194j18 (LBNL H158), complete sequence. 4.1e-107:318:96
AC005368
F-PLACE1000740
Rat notch 2 mRNA.
1.1e-37:399:74
M93661
F-PLACE1000907
RPCI11-73M20.TJ RPCI11 Homo sapiens genomic clone R-73M20, genomic survey sequence.
3.5e-21:147:92
AQ269030
F-PLACE1000912
F-PLACE1000914
Homo sapiens chromosome 17, clone HCIT145P4, WORKING DRAFT SEQUENCE, 8 unordered pieces.
1.8e-74:206:93
AC002093
F-PLACE1000927
Cowpox virus strain GRI-90 DNA (49 kb fragment).
6.8e-75:683:74
Y15035
F-PLACE1000986
RPCI11-75H23.TK RPCI11 Homo sapiens genomic clone R-75H23, genomic survey sequence.
1.0:316:57
AQ268409
F-PLACE1001016
Human dihydropyridine-sensitive L-type calcium channel alpha-1 subunit (CACNL1A3) mRNA, complete cds.
0.28:432:59
L33798
F-PLACE1001100
RPCI11-32N5.TK RPCI-11 Homo sapiens genomic clone RPCI-11-32N5, genomic survey sequence.
0.48:145:64
AQ047336
F-PLACE1001114
Lysobacter enzymogenes beta-lactamase gene sequence.
0.033:349:60
M97392
F-PLACE1001123
F.rubripes GSS sequence, clone 084A20aC12, genomic survey sequence. 9.7e-05:138:64
AL015804
F-PLACE1001183
Homo sapiens BAG clone RG318C11 from 7p14-p15, complete sequence.
0. 15:576:59
AC005091
F-PLACE1001229
F.rubripes GSS sequence, clone 144D13aC10, genomic survey sequence. 2.2e-21:271:70
AL017986
F-PLACE1001231
Rattus norvegicus sodium-dependent multi-vitamin transporter (SMVT) mRNA, complete cds.
6.4e-102:677:84
AF026554
F-PLACE1001340
Homo sapiens mRNA for KIAA0719 protein, complete cds.
1.3e-130:636:97
AB018262
F-PLACE1001401
CIT-HSP-2323H22. TR CIT-HSP Homo sapiens genomic clone 2323H22, genomic survey sequence.
6.4e-13:165:76
AQ028562
F-PLACE1001407
Human DNA sequence from clone 496H19 on chromosome 6q24 Contains ESTs, complete sequence.
2.4e-28:228:85
AL023582
F-PLACE1001464
Human placental cDNA coding for 5' nucleotidase (EC 3.1.3.5).
5.0e-151:742:96
X55740
F-PLACE1001500
CIT-HSP-2368L16. TR CIT-HSP Homo sapiens genomic clone 2368L16, genomic survey sequence.
1.1e-25:150:97
AQ078655
F-PLACE1001516
Homo sapiens Chromosome 16 BAC clone CIT987SK-A-575C2, complete sequence.
1. 2e-139: 676: 98
AC002425
F-PLACE1001536
Human Chromosome X clone bWXD173, WORKING DRAFT SEQUENCE, 2 ordered pieces. 1.7e-142:513:97
AC004387
F-PLACE1001564
Human DNA sequence *** SEQUENCING IN PROGRESS *** from clone 287G14, WORKING
DRAFT SEQUENCE.
2.9e-104:373:89
AL033377
F-PLACE1001655
Homo sapiens Shab-related delayed-rectifier K+ channel alpha subunit (KCNS3) mRNA, complete cds.
1.3e-123:585:98
AF043472
F-PLACE1001788
Homo sapiens mRNA for HYA22, complete cds.
9.9e-21:234:75
D88153
F-PLACE1001795
Drosophila melanogaster; Chromosome 3L; Region 83F1-83F2; P1 clone DS07437, WORKING DRAFT SEQUENCE, 3 unordered pieces.
1.4e-05:218:64
AC005985
F-PLACE1001836
Homo sapiens BAC clone GS155M11 from 7q21-q22, complete sequence.
4.9e-79:577:82
AC004022
F-PLACE1001918
Arabidopsis thaliana BAC T19D16 genomic sequence.
3.7e-24:417:63
U95973
F-PLACE1001949
S.cerevisiae chromosome XV reading frame ORF YOR291w.
3.6e-16:255:70
Z75199
F-PLACE1002080
Homo sapiens antigen NY-CO-9 (NY-CO-9) mRNA, partial cds.
7.5e-129:622:98
AF039691
F-PLACE1002095
Homo sapiens chromosome 5, P1 clone 1130f1 (LBNL H40), complete sequence. 2.3e-48:551:71
AC004219
F-PLACE1002153
Homo sapiens TACC2 protein (TACC2) mRNA, partial cds.
8.3e-161:764:98
AF095791
F-PLACE1002329
Sequence 12 from Patent WO 9000403.
6.9e-05:380:63
I09634
F-PLACE1002355
Homo sapiens protease-activated receptor 4 mRNA, complete cds. 2.8e-17:190:77
AF055917
F-PLACE1002374
Human mRNA for pro-cathepsin L (major excreted protein MEP).
6.2e-162:716:94
X12451
F-PLACE1002518
HS_3091_A1_F08_MF CIT Approved Human Genomic Sperm Library D Homo sapiens genomic clone Plate=3091 Col=15 Row=K, genomic survey sequence.
3.2e-74:316:94
AQ123005
F-PLACE1002547
Homo sapiens mRNA for KIAA0719 protein, complete cds.
2.6e-171:819:98
AB018262
F-PLACE1002726
CIT-HSP-2369G10. TR CIT-HSP Homo sapiens genomic clone 2369G10, genomic survey sequence.
4.8e-18:135:88
AQ075115
F-PLACE1002905
Drosophila melanogaster DNA sequence (P1 DS00906 (D99)), complete sequence. 3.7e-06:235:66
AC004154
F-PLACE1002911
Bovine herpesvirus 1 complete genome.
0.93:264:63
AJ004801
F-PLACE1002967
Homo sapiens IgE receptor beta chain (HTm4) mRNA, complete cds. 0.0041:302:60
L35848
F-PLACE1003135
Homo sapiens STE20-like kinase 3 (mst-3) mRNA, complete cds.
4.7e-49:450:75
AF024636
F-PLACE1003163
Homo sapiens DBI-related protein mRNA, complete cds.
4.7e-152:722:98
AF069301
F-PLACE1003407
Homo sapiens putative transmembrane protein (CLN5) mRNA, complete cds. 6.3e-141:682:97
AF068227
F-PLACE1003428
Human DNA sequence from clone 55C23 on chromosome 6q22.3-23.3 contains vanin-like genes VNN1 and VNN2, ESTs, GSSs,, complete sequence.
1.2e-116:286:100
AL032821
F-PLACE1003438
Pseudomonas alcaligenes outer membrane Xcp-secretion system gene cluster. 0.13:468:60
AF092918
F-PLACE1003460
HS_3234_A1_F05_MR CIT Approved Human Genomic Sperm Library D Homo sapiens genomic clone Plate=3234 Col=9 Row=K, genomic survey sequence.
5.8e-05:111:71
AQ191345
F-PLACE1003529
Homo sapiens clone DJ0981007, complete sequence.
5.8e-134:457:97
AC006017
F-PLACE1003573
Sequence 2 from patent US 5792648.
0.93:186:62
AR022348
F-PLACE1003598
Mus musculus mismatch repair protein (MSH6) gene, exon 1.
3.3e-07:311:63
AF031085
F-PLACE1003644
Homo sapiens Chromosome 11q23 PAC clone pDJ149k2 containing PLZF gene encoding kruppel-like zinc finger protein, complete sequence.
1.8e-06:138:74
AC001234
F-PLACE1003737
Homo sapiens Xp22-83 BAC GSHB-324M7 (Genome Systems Human BAC Library) complete sequence.
1.4e-165:791:98
AC005859
F-PLACE1003772
Human p300/CBP-associated factor (P/CAF) mRNA, complete cds.
2.2e-07:448:61
U57317
F-PLACE1003839
Homo sapiens Chromosome 16 BAC clone CIT987SK-A-69G12, complete sequence. 2.0e-106:525:97
AC004131
F-PLACE1003845
Caenorhabditis elegans cosmid D2096.
9.8e-26:386:69
U40800
F-PLACE1003852
Homo sapiens mRNA for KIAA0758 protein, partial cds.
7. 4e-171 :814:98
AB018301
F-PLACE1004028
F-PLACE1004078
Homo sapiens PAC clone DJ0722F20 from 7q31.1-q31.3, complete sequence. 2.0e-116:274:98
AC005281
F-PLACE1004166
HS_3223_A1_H09_T7 CIT Approved Human Genomic Sperm Library D Homo sapiens genomic clone Plate=3223 Col=17 Row=0, genomic survey sequence.
0.77:304:58
AQ193346
F-PLACE1004168
F-PLACE1004199
CIT-HSP-2328F14.TR CIT-HSP Homo sapiens genomic clone 2328F14, genomic survey sequence.
9.4e-16:186:76
AQ042262
F-PLACE1004279
Homo sapiens putative renal organic anion transporter 1 (hROAT1) mRNA, complete cds.
1.2e-18:456:62
AF057039
F-PLACE1004282
F-PLACE1004305
Homo sapiens mRNA for KIAA0740 protein, complete cds.
2.7e-121:612:96
AB018283
F-PLACE1004441
Human G protein-coupled receptor (GPR1) gene, complete cds.
2.4e-104:537:95
U13666
F-PLACE1004450
Pleuronectes americanus aminopeptidase N (ampN) mRNA, complete cds.
3.1e-20:601:60
AF012465
F-PLACE1004482
HS_3032_B1_C03_MR CIT Approved Human Genomic Sperm Library D Homo sapiens genomic clone Plate=3032 Col=5 Row=F, genomic survey sequence.
1.1e-86:423:98
AQ129106
F-PLACE1004492
Human DNA sequence from PAC 434P1 on chromosome 22. Contains inward rectifier potassium channel 4, (potassium channel, inwardly rectifying, subfamily J, member 4) (hippocampal inward rectifier) (HIR) (HRK1) (HIRK2) (KIR2.3), ESTs similar to lumen protein retaining receptor 2 (KDEL receptor 2), DEAD-box protein P72, ESTs, CpG islands.
0.17:180:67
Z97056
F-PLACE1004519
Human DNA sequence from clone 24o18 on chromosome 6p21.31-22.2 Contains zinc finger protein pseudogene, VNO-type olfactory receptor pseudogene, nuclear envelope pore membrane protein, EST, STS, GSS, complete sequence.
1.1e-75:432:84
AL021808
F-PLACE1004520
Human pregnancy-specific beta-1-glycoprotein mRNA PSG95, complete cds.
4.1e-109:606:92
M34715
F-PLACE1004630
Homo sapiens mRNA for osteoblast specific cysteine-rich protein, complete cds. 6.2e-138:749:92
AB008375
F-PLACE1004637
HS-1061-B1-E10-MF.abi CIT Human Genomic Sperm Library C Homo sapiens genomic clone Plate=CT 783 Col=19 Row=J, genomic survey sequence.
0.013:92:75
B45487
F-PLACE1004648
F-PLACE1004816
Homo sapiens mRNA for Hakata antigen, complete cds.
3.8e-98:590:90
D88587
F-PLACE1004887
Dog alpha-L-iduronidase (IDUA) gene, exons 7-12.
1.2e-06:469:60
L01060
F-PLACE1005003
Human SNC19 mRNA sequence.
4.8e-20:472:63
U20428
F-PLACE1005005
Homo sapiens chromosome 21q22.3, PAC clones 314N7, 225L15, BAC clone 7B7, complete sequence bases 1..333303.
7.8e-143:650:97
AJ011930
F-PLACE1005031
Bovine chlorine channel protein (p64) mRNA, complete cds.
7.1e-62:463:83
L16547
F-PLACE1005239
Homo sapiens mRNA for HIRIP3 protein, clone pH4-31.
2.2e-14:115:85
AJ223349
F-PLACE1005250
Mus musculus maternal transcript Maid (Maid) mRNA, complete cds.
3.3e-40:370:77
U50734
F-PLACE1005383
Homo sapiens UP50 mRNA, complete cds.
2.7e-126:633:96
AF093118
F-PLACE1005410
Rattus rattus sec61 homologue mRNA, complete cds.
1.9e-115:771:85
M96630
F-PLACE1005426
Homo sapiens chromosome 19, CIT-HSP BAG 490g23 (BC338531), complete sequence. 7.2e-113:391:96
AC005392
F-PLACE1005519
Homo sapiens STE20-like kinase 3 (mst-3) mRNA, complete cds.
1.0e-53:521:74
AF024636
F-PLACE1005539
c-erbB=proto-oncogene {exon 1, promoter} [chickens, Genomic, 700 nt].
3.6e-05:434:62
S66408
F-PLACE1005544
Mus musculus junctional adhesion molecule (Jam) mRNA, complete cds.
3.3e-56:575:74
U89915
F-PLACE1005569
Human DNA sequence *** SEQUENCING IN PROGRESS *** from clone 159A1, WORKING
DRAFT SEQUENCE.
1.1e-118:381:96
AL034397
F-PLACE1005601
Human PAC clone DJ515N1 from 22q11.2-q22, complete sequence.
3.9e-143:697:98
AC002073
F-PLACE1005660
F-PLACE1005669
Fruit fly (D.melanogaster) Glued mRNA, complete cds.
3.4e-14:275:66
J02932
F-PLACE1005682
Mus musculus Ankhzn mRNA, complete cds.
0.75:347:57
AB011370
F-PLACE1005725
Homo sapiens huntingtin (HD) gene, exon 1.
1.4e-06:425:62
L27350
F-PLACE1005736
F-PLACE1005745
HS_3039_B1_F12_MF CIT Approved Human Genomic Sperm Library D Homo sapiens genomic clone Plate=3039 Col=23 Row=L, genomic survey sequence. 1.0:283:59
AQ155068
F-PLACE1005768
Human DNA sequence from Fosmid 24E5 on chromosome 22q11.2-qter contains parvalbumin, ESTs, STS.
1. 5e-141 :719:96
Z82185
F-PLACE1005815
Sequence 1 from patent US 5571905.
0.088:199:62
I28535
F-PLACE1005878
Bovine chlorine channel protein (p64) mRNA, complete cds.
2.5e-54:394:84
L16547
F-PLACE1005927
HS_3138_B2_B03_MR CIT Approved Human Genomic Sperm Library D Homo sapiens genomic clone Plate=3138 Col=6 Row=D, genomic survey sequence.
8.0e-32:162:95
AQ183333
F-PLACE1006071
1.6e-180:877:96
AF028816
F-PLACE1006073
Homo sapiens mRNA for glucuronyltransferase I, complete cds.
1.7e-94:464:98
AB009598
F-PLACE1006079
Homo sapiens distal-less homeobox protein (DLX3) gene, complete cds.
5.2e-107:423:96
AF028233
F-PLACE1006093
jd187 Trypanosome Shotgun M13 genomic Trypanosoma brucei brucei genomic clone 5H9, genomic survey sequence.
0.00018:316:60
B13419
F-PLACE1006208
Bovine herpesvirus type 1 early-intermediate transcription control protein (BICP4) gene, complete cds.
1.4e-12:421:64
L14320
F-PLACE1006219
Caenorhabditis elegans cosmid D2096.
6.4e-25:386:69
U40800
F-PLACE1006277
Human DNA sequence *** SEQUENCING IN PROGRESS *** from clone 159A1, WORKING
DRAFT SEQUENCE.
7. 2e-135:381 :97
AL034397
F-PLACE1006290
Caenorhabditis elegans cosmid F09E5.
1.4e-08:354:61
U37429
F-PLACE1006443
Homo sapiens chromosome 17, clone HCIT145P4, WORKING DRAFT SEQUENCE, 8 unordered pieces.
2.9e-80:168:95
AC002093
F-PLACE1006515
Homo sapiens mRNA for KIAA0576 protein, partial cds.
4.2e-140:655:99
AB011148
F-PLACE1006716
Human DNA sequence from PAC 151B14 on chromosome 22q12-qter contains somatostatin receptor subtype 3 (SSTR3), tRNA, ESTs, CpG island and STS. 2.2e-51:621:70
Z86000
F-PLACE1006786
CITBI-E1-2502A9.TR CITBI-E1 Homo sapiens genomic clone 2502A9, genomic survey sequence.
0.43:237:64
AQ264473
F-PLACE1006809
HS_2255_B1_F05_MR CIT Approved Human Genomic Sperm Library D Homo sapiens genomic clone Plate=2255 Col=9 Row=L, genomic survey sequence.
2.1e-14:95:97
AQ131814
F-PLACE1006959
HS_3247_B1_E03_MR CIT Approved Human Genomic Sperm Library D Homo sapiens genomic clone Plate=3247 Col=5 Row=J, genomic survey sequence.
1.1e-09:199:70
AQ220414
F-PLACE1007028
Homo sapiens Chromsome 11p15.5 PAC clone pDJ754h15 containing cdk-inhibitor p57/KIP2 (CDKN1C) gene, complete sequence.
2.0e-24:658:62
AC005950
F-PLACE1007040
Mus musculus neuronal intermediate filament protein (alpha-internexin) gene, complete cds.
8.8e-09:585:62
L27220
F-PLACE1007077
F-PLACE1007081
RPCI11-31D7.TJ RPCI-11 Homo sapiens genomic clone RPCI-11-31D7, genomic survey sequence.
2.3e-42:228:97
AQ016433
F-PLACE1007096
F-PLACE1007296
Human mRNA for a presumptive KDEL receptor.
1.3e-71:542:83
X55885
F-PLACE1007591
Homo sapiens full length insert cDNA clone YP44A02.
1.1e-18:141:90
AF085890
F-PLACE1007626
Homo sapiens unknown mRNA, complete cds.
7. 8e-104:516:97
AF047439
F-PLACE1007702
Homo sapiens chromosome 17, clone 363G12, WORKING DRAFT SEQUENCE, 11 unordered pieces.
7.5e-50:439:77
AC002348
F-PLACE1007845
Caenorhabditis elegans cosmid F09E5.
4.4e-08:355:62
U37429
F-PLACE1007881
CITBI-E1-2517N6.TF CITBI-E1 Homo sapiens genomic clone 2517N6, genomic survey sequence.
1.4e-14:104:95
AQ279407
F-PLACE1007971
HS_3237_B2_F09_MR CIT Approved Human Genomic Sperm Library D Homo sapiens genomic clone Plate=3237 Col=18 Row=L, genomic survey sequence.
1. 2e-12:169:76
AQ206052
F-PLACE1008282
Homo sapiens clone DJ0042M02, WORKING DRAFT SEQUENCE, 20 unordered pieces. 4.5e-101:192:100
AC005995
F-PLACE1008297
Mycoplasma genitalium random genomic clone hg1, partial cds.
0.099:193:60
U02109
F-PLACE1008359
Homo sapiens DNA for (CGG)n trinucleotide repeat region, isolate CL16-1 (Chr.16).
0.53:185:65
AJ001218
F-PLACE1008469
Homo sapiens chromosome 17, clone HCIT145P4, WORKING DRAFT SEQUENCE, 8 unordered pieces.
1.2e-93:213:98
AC002093
F-PLACE1008549
Homo sapiens E74-like factor 5 (ELF5) mRNA, complete cds.
5.7e-144:693:98
AF049703
F-PLACE1008657
Bovine mRNA for adseverin, complete cds.
5.6e-140:782:90
D26549
F-PLACE1008716
Human beta-1,2-N-acetylglucosaminyltransferase II (MGAT2) gene, complete cds. 1.1e-133:648:97
U15128
F-PLACE1008744
Sequence 1 from patent US 5691147.
8.4e-91:475:95
I76197
F-PLACE1008984
Human DNA sequence *** SEQUENCING IN PROGRESS *** from clone 167A19, WORKING
DRAFT SEQUENCE.
4.2e-103:493:99
AL031427
F-PLACE1008985
Mus musculus synaptotagmin VIII mRNA, partial cds.
1.1e-23:289:72
U20107
F-PLACE1009067
H.sapiens CpG island DNA genomic Mse1 fragment, clone 52e12, forward read cpg52e12.ft1a.
1.2e-28:164:96
Z61442
F-PLACE1009196
F-PLACE1009279
H.sapiens mRNA for serine protease.
6.0e-10:327:64
Y07921
F-PLACE1009527
Human DNA-binding protein ABP/ZF mRNA, complete cds.
2.0e-19:125:96
U82613
F-PLACE1009546
S. lividans mercury resistance operon.
0.56:358:59
X65467
F-PLACE1009600
Mouse mRNA for tetracycline transporter-like protein, complete cds.
2.1e-128:718:91
D88315
F-PLACE1009735
Homo sapiens clone NH0523H20, complete sequence.
2.9e-128:613:99
AC005041
F-PLACE1009982
F-PLACE1010011
, complete sequence.
2.1e-26:234:83
AC005409
F-PLACE1010078
Saccharomyces cerevisiae chromosome XII cosmid 8300.
0.066:273:58
U19028
F-PLACE1010081
Homo sapiens serine/threonine kinase RICK (RICK) mRNA, complete cds. 7.0e-150:733:97
AF027706
F-PLACE1010251
Sequence 1 from patent US 5665588.
0.0012:309:62
I64695
F-PLACE1010445
Herpes simplex virus type 2 (strain HG52), complete genome.
9.4e-07:511:58
Z86099
F-PLACE1010713
Mus musculus putative steroid dehydrogenase (KIK-I) mRNA, complete cds. 2.1e-89:612:83
AF064635
F-PLACE1010784
Sequence 1 from patent US 5686597.
2.5e-103:505:98
I73723
F-PLACE1010827
Cricetulus griseus COP-coated vesicle membrane protein CHOp24 mRNA, partial cds.
7.3e-13:327:66
U26264
F-PLACE1010968
O.cuniculus mRNA for titin.
0.44:165:64
X64696
F-PLACE1011045
Homo sapiens E1-like protein mRNA, complete cds.
1.8e-127:595:99
AF094516
F-PLACE1011116
HS_2033_A2_E05_T7 CIT Approved Human Genomic Sperm Library D Homo sapiens genomic clone Plate=2033 Col=10 Row=I, genomic survey sequence.
8.3e-29:192:92
AQ229784
F-PLACE1011181
H.sapiens CpG island DNA genomic Mse1 fragment, clone 99f2, reverse read cpg99f2.rt1a.
4.8e-35:200:95
Z64239
F-PLACE1011236
Mus musculus mRNA for RST, complete cds.
4.5e-54:717:66
AB005451
F-PLACE1011364
Homo sapiens protein kinase/endoribonulcease (IRE1) mRNA, complete cds. 0.13:502:57
AF059198
F-PLACE1011407
M.domesticus (C57B)/6J) mRNA for zinc finger protein 30.
7.2e-15:313:68
Z30174
F-PLACE1011516
Drosophila melanogaster: Chromosome 3L; Region 79F1-80A2; BAC clone BACR48E05, WORKING DRAFT SEQUENCE, 4 unordered pieces.
1.8e-16:317:66
AC005720
F-PLACE1011708
Homo sapiens intrinsic factor-B12 receptor precursor, mRNA, complete cds. 1.8e-143:722:96
AF034611
F-PLACE1011824
Human Ste20-like kinase (MST2) mRNA, complete cds.
5.0e-100:561:92
U26424
F-PLACE1011978
Figure 2. Nucleotide and translated protein sequences of HPF1, -2, and -9. 9.6e-76:722:74
M27877
F-PLACE2000118
Homo sapiens DNA sequence from PAC 393P12 on chromosome Xp11.21. Contains a hypothetical protein KIAA0413 (KIAA0412, KIAA0065, KIAA0569) LIKE Zinc Finger protein pseudogene, a hypothetical Proline-rich protein KIAA0269 LIKE gene and a 60S Ribosomal Protein L7 LIKE pseudogene. Contains ESTs, an STS and a GSS (BAC end sequence), complete sequence.
5.2e-112:568:95
AL022578
F-PLACE2000219
Homo sapiens Down Syndrome critical region, partial sequence.
0.0059:144:71
AF015262
F-PLACE3000181
Sequence 102 from patent US 5643781.
4.1e-127:745:90
I51041
F-PLACE3000213
F-PLACE4000354
HS_3071_A2_B06_MF CIT Approved Human Genomic Sperm Library D Homo sapiens genomic clone Plate=3071 Col=12 Row=C, genomic survey sequence.
4.4e-12:335:64
AQ137396
F-PLACE4000455
Homo sapiens transcriptional enhancer factor (TEF1) DNA, complete CDS. 9.5e-118:563:98
M63896
F-SKNMC1000004
Homo sapiens clone RG031N19, WORKING DRAFT SEQUENCE, 1 unordered pieces. 2.9e-141:292:98
AC005632
F-SKNMC1000014
CIT-HSP-2359K11.TR CIT-HSP Homo sapiens genomic clone 2359K11, genomic survey sequence.
0.89:136:67
AQ075724
F-SKNMC1000082
H.sapiens CpG island DNA genomic Mse1 fragment, clone 26g3, reverse read cpg26g3.rt1b.
5.6e-06:60:98
Z65216
F-THYRO1000036
F-THYRO1000061
Homo sapiens chromosome 19, cosmid R28991, complete sequence.
2.4e-105:425:94
AC004623
F-THYRO1000099
Rattus norvegicus leukocyte common antigen receptor (LAR) gene, trans-spliced alternative untranslated exon.
0.35:609:57
U87960
F-THYRO1000196
Homo sapiens Ksp-cadherin (CDH16) mRNA, complete cds.
5.1e-125:475:98
AF016272
F-THYRO1000400
Mycobacterium tuberculosis sequence from clone y423.
1.0:264:59
AD000014
F-THYRO1000580
HS_3216_A1_H09_T7 CIT Approved Human Genomic Sperm Library D Homo sapiens genomic clone Plate=3216 Col=17 Row=0, genomic survey sequence.
2.8e-25:157:96
AQ184086
F-THYRO1000584
Boar mRNA for 135kDa protein, complete cds.
2.0e-104:787:80
D28521
F-THYRO1000678
M.musculus Cx30 gene.
6.9e-41:285:85
Z70023
F-THYRO1000776
Drosophila melanogaster DNA sequence (P1 DS08948 (D168)), complete sequence. 2.7e-10:389:59
AC004288
F-THYRO1000795
Rattus norvegicus mRNA for mitochondrial dicarboxylate carrier.
1.2e-107:736:83
AJ223355
F-THYRO1000846
CITBI-E1-2505H6.TR CITBI-E1 Homo sapiens genomic clone 2505H6, genomic survey sequence.
0.00025:351:61
AQ260270
F-THYRO1000866
Homo sapiens SKB1Hs mRNA, complete cds.
3.3e-91:529:89
AF015913
F-THYRO1000956
Human G protein-coupled receptor APJ gene, complete cds.
3.8e-148:724:97
U03642
F-THYRO1000964
Drosophila melanogaster Pelle associated protein Pellino (Pli) mRNA, complete cds.
5.0e-37:714:64
AF091624
F-THYRO1000999
CITBI-E1-2508B3.TF CITBI-E1 Homo sapiens genomic clone 2508B3, genomic survey sequence.
1.2e-06:280:62
AQ261426
F-THYRO1001063
H.sapiens (xs174) mRNA, 300bp.
1.6e-41:298:85
Z36825
F-THYRO1001071
Human mRNA for KIAA0154 gene, partial cds.
7.4e-16:197:73
D63876
F-THYRO1001102
Homo sapiens PAC clone DJ130H16 from 22q12.1-qter, complete sequence.
3.5e-10:128:83
AC004997
F-THYRO1001113
Homo sapiens mRNA for LGMD2B protein.
8.8e-52:684:68
AJ007670
F-THYRO1001128
Homo sapiens chromosome 9q34, clone 63G10, complete sequence.
1.2e-141:227:97
AC002096
F-THYRO1001205
F-THYRO1001237
Mus musculus interleukin-2 (II-2) gene, 5' end.
0.77:78:74
L07576
F-THYRO1001242
Mouse mRNA for thymic epithelial cell surface antigen, complete cds.
5.1e-127:721:90
D67067
F-THYRO1001266
Human sodium iodide symporter mRNA, complete cds.
2.7e-41:806:62
U66088
F-THYRO1001327
Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
3.1e-117:374:96
AL021578
F-THYRO1001456
F-THYRO1001457
M.musculus (Balb/c) mRNA for serine/threonine protein kinase.
1.8e-57:491:69
Z34524
F-THYRO1001471
Sequence 52 from Patent W09712992.
0.00019:546:58
A62364
F-THYRO1001478
F-THYRO1001495
Homo sapiens clone DJ1163L11, complete sequence.
4.4e-20:222:76
AC005230
F-THYRO1001523
Human DNA sequence *** SEQUENCING IN PROGRESS *** from clone 407F17, WORKING
DRAFT SEQUENCE.
8.8e-21:538:62
Z83845
F-THYRO1001529
M.musculus mRNA for serine palmitoyltransferase subunit B.
5.8e-32:448:66
X95642
F-THYRO1001593
Homo sapiens chromosome 19, cosmid R31237, complete sequence.
5.8e-91:213:98
AC005581
F-THYRO1001608
Homo sapiens T-cell receptor alpha delta locus from bases 1000498 to 1071650 (section 5 of 5) of the Complete Nucleotide Sequence.
0.0028:335:65
AE000662
F-THYRO1001641
Leishmania major chromosome 3 clone L6290 strain Friedlin, WORKING DRAFT SEQUENCE, 2 ordered pieces.
0.92:378:61
AC005928
F-THYRO1001700
HS_3220_A1_B08_MR CIT Approved Human Genomic Sperm Library D Homo sapiens genomic clone Plate=3220 Col=15 Row=C, genomic survey sequence.
1.0e-49:265:96
AQ184388
F-THYRO1001702
Mus musculus mRNA for myeloid associated differentiation protein.
1.4e-70:502:82
AJ001616
F-THYRO1001725
F.rubripes GSS sequence, clone 133B16aE1, genomic survey sequence.
3.8e-06:249:65
AL004967
F-THYRO1001770
S.cerevisiae chromosome II reading frame ORF YBR059c.
1.5e-07:320:62
Z35928
F-THYRO1001803
Homo sapiens chromosome 10 clone CRI-JC2019 map 10q22.1-10q22.2, WORKING DRAFT SEQUENCE, 1 ordered pieces.
1.2e-38:234:94
AC006108
F-Y79AA1000030
Homo sapiens chromosome 5, BAC clone 319C17 (LBNL H159), complete sequence. 9. 9e-92:389:98
AC005214
F-Y79AA1000127
Homo sapiens genomic DNA, chromosome 21q11.1, segment 5/28, WORKING DRAFT
SEQUENCE.
9.2e-131:359:100
AP000034
F-Y79AA1000207
Homo sapiens chromosome 17, clone hRPK.271_K_11, complete sequence.
2.2e-151:302:98
AC005562
F-Y79AA1000226
Drosophila melanogaster, chromosome 2L, region 21C5-21D1, P1 clone DS07610, complete sequence.
1.1e-50:549:67
AC004573
F-Y79AA1000270
Bos taurus vacuolar H+ ATPase subunit Ac45 mRNA, complete cds.
6.4e-111:771:83
U10039
F-Y79AA1000426
Mus musculus activin beta E subunit mRNA, complete cds.
2.4e-87:703:76
U96386
F-Y79AA1000521
Homo sapiens LERK-6 (EPLG6) gene, exon 1.
0.0092:148:68
U92893
F-Y79AA1000750
Homo sapiens Chromosome 16 BAC clone CIT987SK-A-761H5, complete sequence. 9.0e-07:143:74
AC002544
F-Y79AA1000776
F-Y79AA1000777
Podospora anserina beta transducin-like protein (het-e1) gene, complete cds. 6.6e-17:760:59
L28125
F-Y79AA1000876
Homo sapiens long form transcription factor C-MAF (c-maf) mRNA, complete cds. 3.3e-10:323:66
AF055377
F-Y79AA1000888
Streptomyces coelicolor cosmid 8A6.
3.1e-06:665:59
AL031013
F-Y79AA1000959
Homo sapiens Hsp70 binding protein HspBP1 mRNA, complete cds.
1. 6e-52:277:96
AF093420
F-Y79AA1000967
Rattus norvegicus vesicla-associate calmodulin-binding protein mRNA, complete cds.
2.9e-131:752:86
L22557
F-Y79AA1001013
F-Y79AA1001056
Mus musculus maternal transcript Maid (Maid) mRNA, complete cds.
1.2e-85:676:79
U50734
F-Y79AA1001062
Human Chromosome 11 Cosmid cSRL34e5, complete sequence.
8. 6e-17: 293: 65
U73643
F-Y79AA1001090
Homo sapiens chromosome 17, clone hCIT.175_E_5, complete sequence.
1.9e-05:223:63
AC004596
F-Y79AA1001212
Homo sapiens SL15 protein mRNA, complete cds.
4.7e-162:763:98
AF038961
F-Y79AA1001264
Drosophila melanogaster DNA sequence (P1s DS00764 (D273) and DS00501 (D274)), complete sequence.
1.2e-32:599:63
AC005269
F-Y79AA1001272
Homo sapiens *** SEQUENCING IN PROGRESS *** from cosmid 5L5, WORKING DRAFT
SEQUENCE.
1.2e-11:356:67
AJ009613
F-Y79AA1001328
Rattus norvegicus Delta 3 mRNA, complete cds.
2.1e-51:443:76
AF084576
F-Y79AA1001426
HS_3146_A1_A10_T7 CIT Approved Human Genomic Sperm Library D Homo sapiens genomic clone Plate=3146 Col=19 Row=A, genomic survey sequence.
9.0e-23:106:91
AQ141090
F-Y79AA1001427
Bovine cytochrome b5 reductase mRNA, partial cds.
1.4e-55:670:70
M83104
F-Y79AA1001430
Homo sapiens mRNA for KIAA0469 protein, complete cds.
8.6e-123:577:99
AB007938
F-Y79AA1001523
Homo sapiens transcription intermediary factor 1 (TIF1) mRNA, complete cds. 3.3e-91:496:93
AF009353
F-Y79AA1001530
Human DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs, an STS and GSSs, complete sequence.
1. 8e-126: 764: 89
AL030996
F-Y79AA1001592
HS_3219_A2_E12_MR CIT Approved Human Genomic Sperm Library D Homo sapiens genomic clone Plate=3219 Col=24 Row=I, genomic survey sequence.
5.2e-36:234:89
AQ180547
F-Y79AA1001727
F-Y79AA1001787
S.pombe chromosome III cosmid c1672.
8.8e-11:409:58
AL031324
F-Y79AA1001793
Fugu rubripes GSS sequence, clone 027L23aG3, genomic survey sequence. 0.12:131:70
AL025355
F-Y79AA1001795
Human DNA sequence from clone 1033B10 on chromosome 6p21.2-21.31. Contains the BING5 gene, exons 11 to 15 of the BING4 gene, the gene for GalT3 (beta3-Galactosyltransferase), the RPS18 (40S ribosomal protein S18) gene, the SACM2L (suppressor of actin mutation 2, yeast, homolog) gene, a pseudogene similar to TAT-SF1, a Pseudogene similar to zinc finger genes, the RING1 gene, the gene for HKE6 (RING2), the gene for HKE4 (RING5), the RXRB (Retinoid X receptor beta) gene, the COL11A2 (collagen, type XI, alpha 2) gene, the HLA-DPB2 pseudogene and part of the HLA-DPA3 pseudogene. Contains predicted CpG islands, ESTs, STSs, and GSSs, complete sequence.
1.2e-140:672:98
AL031228
F-Y79AA1001799
S.pombe chromosome I cosmid c8C9.
0.00031:300:60
Z99168
F-Y79AA1001803
Mus musculus secretogranin III (SgIII) mRNA, complete cds.
4. 6e-101 :516:82
U02982
F-Y79AA1001863
Homo sapiens DNA, anonymous heat-stable fragment RP5-6A.
5.2e-85:410:99
AB012170
F-Y79AA1002022
CIT-HSP-2053H1.TF CIT-HSP Homo sapiens genomic clone 2053H1, genomic survey sequence.
4.3e-20:130:95
B68526
F-Y79AA1002058
Homo sapiens clone 24733 mRNA sequence.
5.3e-153:740:98
AF052149
F-Y79AA1002121
F-Y79AA1002129
Bovine herpesvirus type 1 early-intermediate transcription control protein (BICP4) gene, complete cds.
5.5e-12:565:61
L14320
F-Y79AA1002213
Rattus norvegicus brain specific Na+-dependent inorganic phosphate cotransporter mRNA, complete cds.
4.0e-12:434:60
U07609
F-Y79AA1002334
F.rubripes GSS sequence, clone 174E24aB10, genomic survey sequence.
3.0e-10:171:72
AL019366
F-Y79AA1002373
Rattus norvegicus Smad8 mRNA, complete cds.
0.96:420:61
AF012347
F-Y79AA1002376
Rattus norvegicus cytoplasmic dynein intermediate chain 2B mRNA, complete cds. 1.1e-132:805:88
U39045
F-Y79AA1002378
Mus musculus mRNA for zinc finger protein, complete cds, clone:CTfin51. 1.9e-64:521:78
D10630
F-Y79AA1002381
O.sativa mRNA for cdc2+/CDC28-related protein kinase.
3.3e-21:431:60
X58194

### Homology search result 7

The result of the homology search in the GenBank(http://www.ncbi.nlm.nih.gov/web/GenBank/) using the clone sequences of the 3'-ends. except EST and STS sequences.

Indicated are from the top,
the name of the clone sequence,
definition of the top hit data,
the P-value: the length of the sequence used for comparison (nucleotide):similarity (%),
the Accession No. of the top hit data.

Blank indicates that the 3'-end sequence corresponding to the 5'-end was not determined in the clone. Data were not shown for the clones in which the P-value was higher than 1.
R-HEMBA1000006
R-HEMBA1000121
Human DNA sequence *** SEQUENCING IN PROGRESS *** from clone 796F18, WORKING
DRAFT SEQUENCE.
2.2e-43:355:80
AL031291
R-HEMBA1000128
Homo sapiens chromosome X, PAC 671D9, complete sequence.
0.99:389:60
AF031078
R-HEMBA1000275
Homo sapiens DNA sequence from PAC 958B3 on chromosome Xp22.11-Xp22.22. Contains ESTs STS and CpG island.
3. 4e-10 : 212 : 66
Z93023
R-HEMBA1000300
{Alu RNA transcript, clone NE461} [human, embryonal carcinoma cells, NTera2D1 pluripotential cells, Other RNA, 282 nt].
4.6e-42:246:89
S42653
R-nnnnnnnnnnnn
Homo sapiens chromosome 17, clone hRPK.235_I_10, complete sequence.
1.0e-71:192:95
AC005922
R-HEMBA1000462
Homo sapiens clone 243 unknown mRNA, complete sequence:
8.3e-90:313:94
AF091094
R-HEMBA1000477
Homo sapiens genomic DNA of 8p21.3-p22 anti-oncogene of hepatocellular colorectal and non-small cell lung cancer , segment 8/11.
0.22:377:60
AB020865
R-HEMBA1000590
Homo sapiens mRNA for matrilin-4, partial.
8.0e-101:547:93
AJ007581
R-HEMBA1000634
*** SEQUENCING IN PROGRESS *** Homo sapiens chromosome 4, BAC clone C0539L10; HTGS phase 1, WORKING DRAFT SEQUENCE, 15 unordered pieces.
0. 95:186:62
AC004480
R-HEMBA1000671
RPC111-65E1.TJ RPC111 Homo sapiens genomic clone R-65E1, genomic survey sequence.
2.1e-09:165:73
AQ237194
R-HEMBA1000713
Homo sapiens 10kD protein (BC10) mRNA, complete cds.
1.2e-117:575:97
AF053470
R-HEMBA1000732
Homo sapiens mRNA for latent transforming growth factor-beta binding protein-4.
1.4e-108:581:93
Y13622
R-nnnnnnnnnnnn
R-HEMBA1000875
Homo sapiens chromosome 17, clone hRPK.1090_M_7, complete sequence. 0.044:253:64
AC005274
R-HEMBA1000940
***ALU WARNING: Human Alu-J subfamily consensus sequence.
1.9e-33:222:82
U14567
R-HEMBA1000962
R-HEMBA1001184
Plasmodium falciparum 3D7 chromosome 12 PFYAC69 genomic sequence, WORKING DRAFT SEQUENCE, 4 unordered pieces.
0.00044:466:58
AC004688
R-HEMBA1001221
Sequence 1 from patent US 5633147.
7.1e-11:232:65
I43819
R-HEMBA1001228
Human germline oligomeric matrix protein (COMP) mRNA, complete cds.
7.8e-89:358:96
L32137
R-HEMBA1001272
nbxb0003bD01r CUGI Rice BAC Library Oryza sativa genomic clone nbxb0003G00r, genomic survey sequence.
0.00014:201:64
AQ050116
R-HEMBA1001296
Homo sapiens PAC clone DJ1168D11 from 7p21-p22, complete sequence. 0.13:440:58
AC004614
R-HEMBA1001297
Homo sapiens putative transcription factor CA150 mRNA, complete cds.
5.0e-92:466:96
AF017789
R-HEMBA1001390
CIT-HSP-2314K10.TR CIT-HSP Homo sapiens genomic clone 2314K10, genomic survey sequence.
3.4e-43:196:85
AQ027191
R-HEMBA1001563
H.sapiens villin gene, exon 1.
2.1e-43:342:81
X71058
R-HEMBA1001621
Human G protein-coupled receptor APJ gene, complete cds.
1.2e-41:288:87
U03642
R-HEMBA1001878
Homo sapiens U5 snRNP-specific 40 kDa protein mRNA, complete cds.
2.0e-79:434:93
AF090988
R-HEMBA1001886
Human zinc finger protein (ZNF141) mRNA, complete cds.
1.8e-59:530:80
L15309
R-HEMBA1002048
Homo sapiens chromosome 5, P1 clone 1307e8 (LBNL H60), complete sequence. 0.36:322:61
AC005355
R-HEMBA1002131
Human DNA sequence from clone 301K23 on chromosome 1p35.1-36.21. Contains the 5' part of a novel gene similar to predicted yeast and worm genes. Contains ESTs and GSSs, complete sequence.
0.22:233:61
AL031730
R-HEMBA1002163
Homo sapiens chromosome X, clone 592, WORKING DRAFT SEQUENCE, 8 unordered pieces.
1.1e-16:275:69
AC002489
R-HEMBA1002167
Rattus norvegicus neuroligin I mRNA, complete cds.
8.7e-23:193:84
U22952
R-HEMBA1002178
R-HEMBA1002195
Homo sapiens DHPS gene, exons 8 to 9.
1.4e-19:114:100
AJ001704
R-HEMBA1002227
Homo sapiens mRNA for 80K-L protein, complete cds.
6.1e-115:567:97
D10522
R-HEMBA1002316
Homo sapiens mRNA for putative GTP-binding protein.
1.5e-18:161:85
Y14391
R-HEMBA1002420
Caenorhabditis elegans cosmid C27A7, complete sequence.
0.88:214:62
Z81041
R-HEMBA1002421
Human heparan sulfate proteoglycan (HSPG) core protein, 3' end.
6.0e-90:443:97
J04621
R-HEMBA1002524
Human MHC Class I region proline rich protein mRNA, complete cds.
3.2e-110:551:96
U63336
R-HEMBA1002551
Mouse Bac 276o8, WORKING DRAFT SEQUENCE, 25 unordered pieces.
7.0e-06:397:61
AC003022
R-HEMBA1002767
Homo sapiens clone NH0486I22, WORKING DRAFT SEQUENCE, 5 unordered pieces. 4.2e-110:568:96
AC005038
R-HEMBA1002985
Homo sapiens chromosome 17, clone hRPK.15_K_2, complete sequence.
3.4e-23:184:86
AC005901
R-HEMBA1003047
Homo sapiens intrinsic factor-B12 receptor precursor, mRNA, complete cds. 5.0e-114:571:96
AF034611
R-HEMBA1003072
HS-1014-B1-F12-MR.abi CIT Human Genomic Sperm Library C Homo sapiens genomic clone Plate=CT 789 Col=23 Row=L, genomic survey sequence.
1.5e-62:340:94
B32084
R-HEMBA1003101
Homo sapiens tyrosylprotein sulfotransferase-2 mRNA, complete cds.
3.8e-116:575:97
AF049891
R-HEMBA1003120
HS_3220_A1_F04_T7 CIT Approved Human Genomic Sperm Library D Homo sapiens genomic clone Plate=3220 Col=7 Row=K, genomic survey sequence. 3.6e-61:354:92
AQ184345
R-HEMBA1003230
Homo sapiens UP50 mRNA, complete cds.
1.3e-42:258:93
AF093118
R-HEMBA1003294
HS_3220_A1_D03_T7 CIT Approved Human Genomic Sperm Library D Homo sapiens genomic clone Plate=3220 Col=5 Row=G, genomic survey sequence. 0.0095:204:63
AQ190655
R-HEMBA1003315
Sus scrofa DNA for LH beta, exons 1, 2, 3, complete cds.
6.6e-24:163:79
D00579
R-HEMBA1003392
Homo sapiens LDL receptor-related protein 6 (LRP6) mRNA, complete cds. 2.6e-115:557:98
AF074264
R-HEMBA1003399
Homo sapiens clone DJ1125K23, WORKING DRAFT SEQUENCE, 21 unordered pieces. 1.8e-63:166:100
AC004971
R-HEMBA1003487
R-HEMBA1003497
Human DNA sequence *** SEQUENCING IN PROGRESS *** from clone 2705, WORKING
DRAFT SEQUENCE.
1.4e-119:592:97
AL033529
R-HEMBA1003530
R-HEMBA1003602
Human Chromosome 16 BAC clone CIT987SK-A-17E1, complete sequence.
9.4e-79:468:91
AC002041
R-HEMBA1003732
Homo sapiens clone DJ0935K16, complete sequence.
2.0e-118:586:98
AC006011
R-HEMBA1003945
Human calcineurin B mRNA, complete cds.
8.9e-82:410:97
M30773
R-HEMBA1004007
Homo sapiens clone DJ0665P05, WORKING DRAFT SEQUENCE, 5 unordered pieces. 6.7e-56:404:75
AC004851
R-HEMBA1004085
G.gallus microsatellite DNA (LE10311 (= EC12A05)).
0.66:144:65
Z95196
R-nnnnnnnnnnnn
Homo sapiens intersectin short form mRNA, complete cds.
2.1e-115:569:97
AF064243
R-HEMBA1004250
Homo sapiens chromosome 5, BAC clone 182a8 (LBNL H161), complete sequence. 3.8e-98:478:98
AC005752
R-HEMBA1004391
Plasmodium falciparum MAL3P8, complete sequence.
0.29:126:65
AL034560
R-HEMBA1004444
Homo sapiens clone DJ0971C03, WORKING DRAFT SEQUENCE, 18 unordered pieces. 8.4e-52:308:78
AC004938
R-HEMBA1004454
CIT-HSP-2337122.TF CIT-HSP Homo sapiens genomic clone 2337122, genomic survey sequence.
0.78:59:77
AQ038475
R-HEMBA1004505
R-HEMBA1004785
R-HEMBA1004797
R-HEMBA1004952
Mus musculus diabetic embryopathy (Dep-1) mRNA.
3.4e-39:327:82
AF032130
R-HEMBA1004971
Homo sapiens BAC clone RG351J01 from 7q22-q31, complete sequence. 0.00040:251:66
AC005099
R-HEMBA1004982
Strongyloides fulleborni 18S ribosomal RNA and 5.8S ribosomal RNA genes, partial sequence, and internal transcribed spacer 1, complete sequence. 0.092:191:63
U43581
R-HEMBA1005070
Human mRNA for KIAA0310 gene, complete cds.
1. 2e-94:381 :91
AB002308
R-HEMBA1005084
R-HEMBA1005145
Homo sapiens complete genomic sequence between D16S3070 and D16S3275, containing Familial Mediterranean Fever gene disease.
5.7e-58:283:84
AJ003147
R-HEMBA1005230
CIT-HSP-2333N15.TR CIT-HSP Homo sapiens genomic clone 2333N15, genomic survey sequence.
5.5e-31:363:73
AQ040189
R-HEMBA1005246
Homo sapiens full length insert cDNA clone YX52E07.
1.6e-11:173:72
AF086040
R-HEMBA1005267
Sequence 1 from patent US 5618695.
2.4e-73:536:81
140055
R-HEMBA1005337
Caenorhabditis elegans cosmid K07D4.
0.16:157:63
AF077534
R-HEMBA1005430
R-HEMBA1005449
R-HEMBA1005489
Anopheles rangeli NADH dehydrogenase subunit 2 gene, mitochondrial gene encoding mitochondrial product, partial cds.
0.020:271:61
U35272
R-HEMBA1005522
R-HEMBA1005545
Human m3 muscarinic acetylcholine receptor (CHRM3) gene, complete cds. 1.8e-115:579:96
U29589
R-HEMBA1005698
0.0065:223:65
AG004952
R-HEMBA1005913
Homo sapiens Xp22 BAC GSHB 526D21 (Genome Systems Human BAC library) complete sequence.
3.7e-15:272:68
AC003037
R-HEMBA1005929
Homo sapiens chromosome 19, cosmid R31237, complete sequence.
9.4e-55:502:76
AC005581
R-HEMBA1005945
Human DNA sequence *** SEQUENCING IN PROGRESS *** from clone 774G10, WORKING
DRAFT SEQUENCE.
0.45:245:62
AL034410
R-HEMBA1006016
Homo sapiens chromosome 17, clone hRPK.271_K_11, complete sequence.
3.5e-25:415:66
AC005562
R-HEMBA1006171
Human DNA sequence from PAC 433M19 on chromosome Xq26.3-Xq27.1. Contains ESTs, STSs and polymorphic CA repeat.
1.0:176:64
Z95703
R-HEMBA1006276
Homo sapiens chromosome 19, CIT-HSP-444n24, complete sequence.
2.8e-118:592:96
AC005261
R-HEMBA1006299
R-HEMBA1006311
R-HEMBA1006335
Human DNA sequence from clone 496H19 on chromosome 6q24 Contains ESTs, complete sequence.
6.1e-111:578:96
AL023582
R-HEMBA1006357
Homo sapiens chromosome Xp22-67-68, WORKING DRAFT SEQUENCE, 99 unordered pieces.
4.8e-11:174:74
AC004469
R-HEMBA1006430
Homo sapiens, WORKING DRAFT SEQUENCE, 52 unordered pieces.
8.7e-45:402:79
AC004086
R-HEMBA1006482
Homo sapiens h-sco1 (SC01) mRNA, nuclear gene encoding mitochondrial protein, complete cds.
1.7e-105:537:96
AF026852
R-HEMBA1006517
345A19.TV CIT978SKA1 Homo sapiens genomic clone A-345A19, genomic survey sequence.
1.5e-44:176:88
B15409
R-HEMBA1006544
Homo sapiens PAC clone DJ130H16 from 22q12.1-qter, complete sequence. 2.5e-66:310:83
AC004997
R-HEMBA1006572
Homo sapiens reduced folate carrier (RFC1) gene, exons 1a, 1c and 1b. 0.028:255:64
U92868
R-HEMBA1006658
Homo sapiens mRNA for KIAA0687 protein, partial cds.
7.3e-111:570:94
AB014587
R-HEMBA1006707
Homo sapiens mRNA for matrilin-4, partial.
5.1e-78:389:97
AJ007581
R-HEMBA1006724
HS_2052_B1_C08_MR CIT Approved Human Genomic Sperm Library D Homo sapiens genomic clone Plate=2052 Col=15 Row=F, genomic survey sequence.
2.6e-46:309:88
AQ305998
R-HEMBA1006749
Homo sapiens mRNA for matrilin-4, partial.
3.2e-88:472:94
AJ007581
R-HEMBA1006770
Homo sapiens CAGH4 mRNA, partial cds.
6.5e-25:145:82
U80746
R-HEMBA1006902
Homo sapiens mRNA for matrilin-4, partial.
9.3e-112:540:98
AJ007581
R-HEMBA1006912
***ALU WARNING: Human Alu-Sc subfamily consensus sequence.
6.6e-48:279:92
U14571
R-HEMBA1006916
Homo sapiens Grb14 mRNA, complete cds.
1.8e-114:346:99
L76687
R-HEMBA1006960
Homo sapiens chromosome 19, cosmid F16403, complete sequence.
0.78:203:62
AC005777
R-HEMBA1007013
Human mRNA for DNA-binding protein TAXREB302, complete cds.
6.3e-31:163:100
D28468
R-HEMBA1007057
CIT-HSP-517F5.TP CIT-HSP Homo sapiens genomic clone 517F5, genomic survey sequence.
1.0:128:67
B49904
R-HEMBA1007063
Homo sapiens DNA sequence from PAC 168L15 on chromosome 6q26-27. Contains RSK3 gene, ribosomal protein S6 kinase, EST, GSS, STS. CpG island, complete sequence.
5.0e-43:300:88
AL022069
R-HEMBA1007241
HIV-1 RNA V3 region (patient Y, sample Y1, clone 05).
0.74:148:66
Z47529
R-HEMBA1007291
Homo sapiens chromosome 19, fosmid 37502, complete sequence.
3.6e-36:300:80
AC004755
R-HEMBA1007332
Human HeLa mRNA isolated as a false positive in a two-hybrid-screen.
7.3e-15:148:80
U56430
R-HEMBB1000106
Plasmodium falciparum (strain FCR3) variant-specific surface protein (var-2, var-3) genes, complete cds's.
8.0e-05:313:60
L40609
R-HEMBB1000276
HS_3048_A2_C07_MR CIT Approved Human Genomic Sperm Library D Homo sapiens genomic clone Plate=3048 Col=14 Row=E, genomic survey sequence.
0.91:234:58
AQ099411
R-HEMBB1000309
R-HEMBB1000407
Mus musculus clone OST5976, genomic survey sequence.
6.4e-28:226:81
AF046768
R-HEMBB1000447
Homo sapiens JWA protein mRNA, complete cds.
1.7e-107:533:97
AF070523
R-HEMBB1000542
Mus musculus bromodomain-containing protein BP75 mRNA, complete cds.
4.4e-72:547:80
AF084259
R-HEMBB1000567
Human insulin-like growth factor (IGF-II) gene, exon 1 of 4.
4.3e-60:368:88
M13970
R-HEMBB1000642
Human DNA sequence from PAC 46H23, BRCA2 gene region chromosome 13q12-13 contains Klotho, ESTs.
2.9e-42:431:75
Z84483
R-HEMBB1000668
CITBI-E1-2508D15.TR CITBI-E1 Homo sapiens genomic clone 2508D15, genomic survey sequence.
2.5e-40:249:91
AQ261535
R-HEMBB1000679
HS_3061_A1_C03_T7 CIT Approved Human Genomic Sperm Library D Homo sapiens genomic clone Plate=3061 Col=5 Row=E, genomic survey sequence.
1.8e-48:257:96
AQ127602
R-HEMBB1000881
CIT-HSP-2350020.TR CIT-HSP Homo sapiens genomic clone 2350020, genomic survey sequence.
0.0072:248:61
AQ062620
R-HEMBB1000905
Homo sapiens clone RG315H11, WORKING DRAFT SEQUENCE, 5 unordered pieces. 2.5e-104:547:94
AC005089
R-HEMBB1001026
R-HEMBB1001048
R-HEMBB1001200
P.falciparum complete gene map of plastid-like DNA (IR-A).
1. 5e-11 : 521 : 59
X95275
R-HEMBB1001407
Human DNA sequence *** SEQUENCING IN PROGRESS *** from clone 2705, WORKING
DRAFT SEQUENCE.
3.0e-29:308:77
AL033529
R-HEMBB1001530
Homo sapiens chromosome 19, cosmid R30538, complete sequence.
0.040:373:63
AC005943
R-HEMBB1001547
Rickettsia prowazekii strain Madrid E, complete genome; segment 1/4. 0.027:291:62
AJ235270
R-HEMBB1001573
CIT-HSP-2307C1.TR CIT-HSP Homo sapiens genomic clone 2307C1, genomic survey sequence.
1.3e-13:90:98
AQ020395
R-HEMBB1001847
Homo sapiens chromosome 21q22.3 PAC 21L13, complete sequence.
3.4e-27:147:80
AF064864
R-HEMBB1001959
Homo sapiens clone 24781 mRNA sequence.
4.4e-103:504:97
AF070640
R-HEMBB1001978
CIT-HSP-2328G6.TF CIT-HSP Homo sapiens genomic clone 2328G6, genomic survey sequence.
7.9e-29:220:86
AQ040310
R-HEMBB1002039
Homo sapiens BAC clone GS166A23 from 7p21, complete sequence.
2.7e-37:550:68
AC005014
R-HEMBB1002041
Sequence 1 from patent US 5633147.
2.7e-23:322:70
I43819
R-HEMBB1002051
Homo sapiens clone DJ0292L20, WORKING DRAFT SEQUENCE, 2 unordered pieces. 9.2e-35:302:79
AC004825
R-HEMBB1002120
Homo sapiens Xp22 GS-524I1 (Genome Systems Human BAC library), complete sequence.
6.0e-05:479:59
AC003106
R-HEMBB1002162
Human DNA sequence from clone 739H11 on chromosome 1p33-34.2 Contains KIAA0237 gene, EST, STS, GSS, complete sequence.
3.7e-30:238:84
AL031289
R-HEMBB1002228
Homo sapiens BAC clone NH0436H22 from 2, complete sequence.
6.6e-57:274:86
AC005234
R-HEMBB1002245
Sequence 25 from patent US 5747660.
4.8e-30:361:73
AR005295
R-HEMBB1002302
Methanococcus jannaschii section 84 of 150 of the complete genome. 0.00019:362:59
U67542
R-HEMBB1002427
Genomic sequence from Human 9q34, complete sequence.
3.9e-105:533:96
AC001643
R-HEMBB1002465
Plasmodium falciparum chromosome 2, section 19 of 73 of the complete sequence. 2.9e-05:335:62
AE001382
R-HEMBB1002661
R-HEMBB1002663
***ALU WARNING: Human Alu-Sq subfamily consensus sequence.
8.3e-43:268:89
U14573
R-HEMBB1002693
Human BAC clone RG126M09 from 7q21-q22, complete sequence.
2.4e-24:220:76
AC002067
R-MAMMA1000046
Plasmodium falciparum 3D7 chromosome 12 PFYAC181 genomic sequence, WORKING DRAFT SEQUENCE, 8 unordered pieces.
0.032:402:57
AC005505
R-MAMMA1000102
Human DNA sequence from cosmid B33F2 on chromosome 22 Contains ESTs.
2.0e-84:428:96
Z79996
R-MAMMA1000106
Homo sapiens genomic DNA, chromosome 21q11.1, segment 2/28, WORKING DRAFT
SEQUENCE.
0.095:138:66
AP000031
R-MAMMA1000118
R-MAMMA1000141
Homo sapiens 12q24.2 PAC RPCI1-128M12 (Roswell Park Cancer Institute Human PAC library) complete sequence.
9.0e-91:480:95
AC004024
R-MAMMA1000204
Homo sapiens mRNA for LGMD2B protein.
1.5e-107:544:96
AJ007670
R-MAMMA1000226
H.sapiens VASP gene, exons 4 to 13.
0.99:244:63
X98534
R-MAMMA1000403
CIT-HSP-2372A15.TF CIT-HSP Homo sapiens genomic clone 2372A15, genomic survey sequence.
8.0e-38:187:81
AQ112406
R-MAMMA1000449
Homo sapiens BAC clone RG139P11 from 7q11-q21, complete sequence.
1.2e-41:422:76
AC004491
R-MAMMA1000457
Homo sapiens clone 638 unknown mRNA, complete sequence.
7.4e-116:570:97
AF091084
R-MAMMA1000473
Homo sapiens Chromosome 16 BAC clone CIT987SK-A-69G12, complete sequence. 9.6e-09:136:77
AC004131
R-MAMMA1000496
Homo sapiens PAC clone DJ130H16 from 22q12.1-qter, complete sequence.
2.6e-48:272:93
AC004997
R-MAMMA1000528
Human BAC clone RG114A06 from 7q31, complete sequence.
1.8e-13:109:80
AC002542
R-MAMMA1000591
Human cosmid g1572c264, complete sequence.
1.6e-22:329:71
AC000359
R-MAMMA1000614
R-MAMMA1000652
H.sapiens flow-sorted chromosome 6 HindIII fragment, SC6pA28A10.
0.81:158:65
Z84499
R-MAMMA1000681
Homo sapiens full length insert cDNA clone YY85D04.
1.0e-107:560:94
AF088014
R-MAMMA1000706
Homo sapiens cGMP phosphodiesterase A1 (PDE9A) mRNA, complete cds.
1.1e-46:232:100
AF067223
R-MAMMA1000788
Bos taurus P14 (p14) mRNA, complete cds.
3.8e-72:493:84
AF037349
R-MAMMA1000810
Human DNA from overlapping chromosome 19-specific cosmids R29515 and R28253, genomic sequence, complete sequence.
5.0e-37:318:79
AC003002
R-MAMMA1000814
Homo sapiens BAC clone BK085E05 from 22q12.1-qter, complete sequence. 7.7e-15:140:85
AC003071
R-MAMMA1000881
Human DNA sequence from clone 105D16 on chromosome Xp11.3-11.4 Contains pseudogene similar to laminin-binding protein, CA repeat, STS, complete sequence.
8.8e-46:457:75
AL031311
R-MAMMA1000986
Homo sapiens chromosome 16 BAC clone CIT987SK-334D11 complete sequence. 7.7e-44:343:82
AF001550
R-MAMMA1000994
Homo sapiens mRNA for ISLR, complete cds.
3.6e-108:552:96
AB003184
R-MAMMA1001043
R-MAMMA1001066
Homo sapiens chromosome 17, clone hRPK.346_K_10, complete sequence. 1.3e-42:302:82
AC006120
R-MAMMA1001094
Homo sapiens clone 243 unknown mRNA, complete sequence.
5.4e-115:567:97
AF091094
R-MAMMA1001141
HS_3059_B1_H06_MR CIT Approved Human Genomic Sperm Library D Homo sapiens genomic clone Plate=3059 Col=11 Row=P, genomic survey sequence.
1.3e-68:388:92
AQ214896
R-MAMMA1001150
H.sapiens mRNA for protein kinase C mu.
5.4e-20:340:66
X75756
R-MAMMA1001237
Mouse DNA fragment that hybridizes to HSV-1 SmaI A fragment.
0.15:222:65
M11041
R-MAMMA1001284
Human DNA sequence *** SEQUENCING IN PROGRESS *** from clone 1185N5, WORKING
DRAFT SEQUENCE.
1.2e-33:344:76
AL034423
R-MAMMA1001310
Human Bruton agammaglobulinemia (BTK) gene, exons 10-12.
1.8e-39:332:80
L31565
R-MAMMA1001418
Human proto-oncogene tyrosine-protein kinase (ABL) gene, exon 1a and exons 2-10, complete cds.
4.4e-42:411:76
U07563
R-MAMMA1001532
Homo sapiens PAC clone DJ0728D04, complete sequence.
2.3e-10:196:73
AC004865
R-MAMMA1001609
HS-1054-B2-H01-MF.abi CIT Human Genomic Sperm Library C Homo sapiens genomic clone Plate=CT 776 Col=2 Row=P, genomic survey sequence.
1.6e-34:170:79
B42016
R-MAMMA1001615
R-MAMMA1001623
Homo sapiens 12q24.2 BAC RPCI11-407A16 (Roswell Park Cancer Institute Human BAC Library) complete sequence.
8.8e-21:180:82
AC006065
R-MAMMA1001634
Homo sapiens chromosome 17, clone hRPK.85_B_7, complete sequence.
2.6e-40:283:86
AC005695
R-MAMMA1001893
Homo sapiens clone DJ0782K24, WORKING DRAFT SEQUENCE, 16 unordered pieces. 0.73:132:67
AC006003
R-MAMMA1001901
Homo sapiens DNA sequence from PAC 958B3 on chromosome Xp22.11-Xp22.22. Contains ESTs STS and CpG island.
4.0e-43:288:77
Z93023
R-MAMMA1001957
Homo sapiens chromosome 16, P1 clone 96-4B (LANL), complete sequence. 1.2e-41:298:86
AC005212
R-MAMMA1001978
R-MAMMA1002070
Homo sapiens clone DJ400N23, WORKING DRAFT SEQUENCE, 10 unordered pieces. 2.1e-104:530:97
AC005003
R-MAMMA1002080
rah=ras-related homolog [mice, HT4 neural cell line, mRNA, 993 nt].
5.9e-47:449:76
S72304
R-MAMMA1002087
Human Cosmid g1572c037 from 7q31.3, complete sequence.
1.7e-11:120:83
AC000125
R-MAMMA1002095
Rat alternatively spliced mRNA.
5.3e-30:289:74
M93018
R-MAMMA1002128
R-MAMMA1002142
R-MAMMA1002165
Homo sapiens chromosome 10 clone CIT987SK-1109P11, complete sequence. 1.1e-28:350:72
AC005871
R-MAMMA1002205
Human DNA sequence from PAC 368A4 on chromosome X. Contains ESTs, CELLULAR NUCLEIC ACID BINDING PROTEIN (CNBP) like gene and STSs.
1.2e-42:282:75
Z83843
R-MAMMA1002224
Arabidopsis thaliana DNA chromosome 4, BAC clone F1C12 (ESSAII project). 0.99:210:60
AL022224
R-MAMMA1002234
Canine mRNA for 68kDA subunit of signal recognition particle (SRP68).
1.7e-61:310:81
X53744
R-MAMMA1002586
Streptomyces collinus coenzyme B12-dependent mutase (meaA) gene, complete cds. 0.99:348:60
AF008569
R-MAMMA1002633
Homo sapiens, clone hRPK.1_A_1, complete sequence.
2.6e-13:381:64
AC006196
R-MAMMA1003126
R-NT2RM4000100
Plasmodium falciparum MAL3P2, complete sequence.
0.00047:296:61
AL034558
R-NT2RM4000115
Plasmodium falciparum DNA *** SEQUENCING IN PROGRESS *** from MAL1P3, WORKING
DRAFT SEQUENCE.
0.079:270:64
AL031746
R-NT2RM4000198
Homo sapiens chromosome 10 clone LA10NC01_15_E_11 map 10q26.3, WORKING DRAFT SEQUENCE, 3 unordered pieces.
7.7e-24:244:78
AC006171
R-NT2RM4000284
Human IgG Fc receptor hFcRn mRNA, complete cds.
1.7e-93:440:100
U12255
R-NT2RM4000295
, complete sequence.
0.89:351:58
AC005663
R-NT2RM4000326
Homo sapiens complete genomic sequence between D16S3070 and D16S3275, containing Familial Mediterranean Fever gene disease.
2.3e-112:602:94
AJ003147
R-NT2RM4000417
Human DNA sequence from PAC 326L13 containing brain-4 mRNA ESTs and polymorphic CA repeat.
0.78:229:62
Z82170
R-NT2RM4000444
R-NT2RM4000587
R-NT2RM4000593
R-NT2RM4000648
0. 010:260:61
AG005508
R-NT2RM4000761
H.sapiens mitochondrial genome (consensus sequence).
3.2e-95:476:97
X62996
R-NT2RM4000965
R-NT2RM4000997
R-NT2RM4001321
Human DNA sequence from clone 1177E19 on chromosome 1p36.12-36.31. Contains the 3' part of the DNA-binding Zinc finger protein RIZ gene, ESTs, an STS, GSSs and a CpG island, complete sequence.
6.0e-19:282:73
AL031277
R-NT2RM4001325
R-NT2RM4001377
Homo sapiens mRNA for KIAA0638 protein, partial cds.
2.9e-111:553:96
AB014538
R-NT2RM4001735
Homo sapiens clone 23904 mRNA sequence.
4.6e-106:553:94
AF052129
R-NT2RM4001768
Human HepG2 3' region Mbol cDNA, clone hmd3c03m3.
4.1e-29:187:91
D17194
R-NT2RM4001843
Homo sapiens chromosome 17, clone hRPK.269_G_24, complete sequence. 0.95:366:58
AC005828
R-NT2RM4002352
Homo sapiens hLRp105 mRNA for LDL receptor related protein 105, complete cds. 5.5e-108:557:95
AB009462
R-NT2RP2000092
HS_3070_B1_B04_MR CIT Approved Human Genomic Sperm Library D Homo sapiens genomic clone Plate=3070 Col=7 Row=D, genomic survey sequence.
1.1e-23:247:77
AQ120714
R-NT2RP2000178
E.amylovora Ion gene.
1.1e-15:422:62
X77706
R-NT2RP2000240
*** SEQUENCING IN PROGRESS *** Homo sapiens chromosome 4, BAC clone C0539L10; HTGS phase 1, WORKING DRAFT SEQUENCE, 15 unordered pieces.
0.00010:260:62
AC004480
R-NT2RP2000394
HS_3211_B2_G06_T7 CIT Approved Human Genomic Sperm Library D Homo sapiens genomic clone Plate=3211 Col=12 Row=N, genomic survey sequence.
1. 1e-61:316:97
AQ174850
R-NT2RP2000447
Homo sapiens clone DJ1129D05, complete sequence. 8.7e-67:357:94
AC005630
R-NT2RP2000479
Homo sapiens chromosome 17, clone 193h18, complete sequence.
5.7e-51:551:73
AC002546
R-NT2RP2000514
P.falciparum parasite antigen reactive with the parasite inhibitory mouse monoclonal antibody (mMAb) 43E5, clone #366, partial cds.
2.1e-08:192:68
M21323
R-NT2RP2000533
Mus musculus cornichon mRNA, complete cds.
3.5e-59:243:82
AF022811
R-NT2RP2000616
Human DNA sequence from clone 694E4 on chromosome 22 Contains exon similar to phosphatidylserine decarboxylase, EST, GSS, complete sequence.
0.0064:105:67
AL031255
R-NT2RP2000649
Homo sapiens mRNA for Hs Ste24p, complete cds.
1.4e-65:326:98
AB016068
R-NT2RP2000663
Human DNA sequence from cosmid U61B11, between markers DXS366 and DXS87 on chromosome X contains ESTs.
7.9e-110:555:96
Z73913
R-NT2RP2000712
Homo sapiens clone DJ0756H11, WORKING DRAFT SEQUENCE, 5 unordered pieces. 9.8e-32:308:78
AC006001
R-NT2RP2000739
Bos taurus TATA box binding protein (TBP) gene, partial cds.
0.19:128:68
L47974
R-NT2RP2000818
Caenorhabditis elegans cosmid C48D5, complete sequence.
0. 010:429:58
Z36237
R-NT2RP2000903
H.sapiens 5T4 gene for 5T4 Oncofetal antigen.
4.0e-99:505:96
Z29083
R-NT2RP2001200
Homo sapiens mRNA for KIAA0676 protein, partial cds.
2.0e-57:306:95
AB014576
R-NT2RP2001223
R-NT2RP2001276
Mouse regulatory protein (npdc-1) mRNA, complete cds.
5.8e-14:353:65
L03814
R-NT2RP2001388
Homo sapiens clone DJ1125K23, WORKING DRAFT SEQUENCE, 21 unordered pieces.
1. 7e-31 : 291 : 77
AC004971
R-NT2RP2001469
M.musculus tex292 mRNA (3' region).
3.7e-26:188:89
X80433
R-NT2RP2001480
Homo sapiens thrombospondin 3 (THBS3) gene, complete cds.
6.6e-83:426:95
L38969
R-NT2RP2001495
transcript ch123.Rev [human, RF1,RF48 stomach cancer cell lines, mRNA, 252 nt].
6.3e-43:238:96
S77359
R-NT2RP2001514
Homo sapiens cyclin K (CPR4) mRNA, complete cds.
6.6e-06:57:100
AF060515
R-NT2RP2001538
Mus musculus transcriptional regulatory protein (mSin3) gene, complete cds.
6. 9e-12:179:75
L36831
R-NT2RP2001562
Human PAC clone DJ0800B09 from 7q11.23-q21, complete sequence.
0.074:257:61
AC004028
R-NT2RP2001662
Homo sapiens clone 24615 mRNA sequence.
3.2e-94:485:95
AF055012
R-NT2RP2001755
Homo sapiens mRNA for KIAA0762 protein, partial cds.
1.3e-103:576:92
AB018305
R-NT2RP2001769
CIT-HSP-2376023.TF CIT-HSP Homo sapiens genomic clone 2376023, genomic survey sequence.
1.5e-74:381:96
AQ111163
R-NT2RP2001817
HS_2037_B2_A09_T7 CIT Approved Human Genomic Sperm Library D Homo sapiens genomic clone Plate=2037 Col=18 Row=B, genomic survey sequence.
3.9e-60:430:84
AQ243047
R-NT2RP2001878
Human DNA sequence from PAC 296K21 on chromosome X contains cytokeratin exon, delta-aminolevulinate synthase (erythroid); 5-aminolevulinic acid
synthase. (EC 2.3.1.37). 6-phosphofructo-2-kinase/fructose-2,6-bisphosphatase (EC 2.7.1.105, EC 3.1.3.46), ESTs and STS.
0.018:148:67
Z83821
R-NT2RP2001903
Human Not1 linking clone from chromosome 1q32.
0.99:160:63
U36769
R-NT2RP2001915
R-NT2RP2001921
Homo sapiens clone NH0332L11, complete sequence.
6.5e-86:295:98
AC005538
R-NT2RP2001948
Homo sapiens chromosome 19, cosmid R33590, complete sequence.
2.3e-79:440:91
AC005620
R-NT2RP2001956
R-NT2RP2002015
Human DNA sequence from clone 1177E19 on chromosome 1p36.12-36.31. Contains the 3' part of the DNA-binding Zinc finger protein RIZ gene, ESTs, an STS, GSSs and a CpG island, complete sequence.
1.1e-16:254:72
AL031277
R-NT2RP2002063
Homo sapiens chromosome 4 clone B207D4 map 4q25, complete sequence.
5.8e-105:550:95
AC004050
R-NT2RP2002188
Human DNA sequence *** SEQUENCING IN PROGRESS *** from clone 134019, WORKING
DRAFT SEQUENCE.
0.47:212:62
AL034555
R-NT2RP2002232
R-nnnnnnnnnnnn
Human mRNA for KIAA0383 gene, partial cds.
2.5e-100:511:96
AB002381
R-NT2RP2002409
S.pombe chromosome I cosmid c17H9.
1.0:241:63
Z98597
R-NT2RP2002510
Homo sapiens chromosome 19, cosmid F19847, complete sequence.
1.6e-38:307:81
AC005952
R-NT2RP2002527
Homo sapiens chromosome 11, BAC CIT-HSP-311e8 (BC269730) containing the hFEN1 gene, complete sequence.
1.5e-18:165:83
AC004770
R-NT2RP2002533
Homo sapiens alpha 2 delta calcium channel subunit isoform II mRNA, complete cds.
9.7e-116:580:96
AF042793
R-NT2RP2002564
Homo sapiens clone DJ0800G07, complete sequence.
3.8e-110:580:94
AC004890
R-NT2RP2002674
Plasmodium falciparum chromosome 2, section 11 of 73 of the complete sequence. 1.0:244:60
AE001374
R-NT2RP2002721
Homo sapiens chromosome 17, clone HCIT217L10, complete sequence.
1.2e-10:221:73
AC003962
R-NT2RP2002824
Human HepG2 3' region Mbol cDNA, clone hmd4f06m3.
3.0e-07:108:77
D17237
R-NT2RP2002942
Human DNA sequence from clone 88D7 on chromosome Xq25-26.3 Contains F9 (coagulation factor IX (plasma thromboplastic component, Christmas disease, haemophilia B)), dbl oncogene. EST, STS, GSS, complete sequence.
2.0e-37:491:71
AL033403
R-NT2RP2002974
H.sapiens DMAHP gene.
4.0e-118:585:97
X84813
R-NT2RP2002976
CIT-HSP-2348J20.TF CIT-HSP Homo sapiens genomic clone 2348J20, genomic survey sequence.
8.4e-45:233:98
AQ059444
R-NT2RP2003042
R-NT2RP2003179
R-NT2RP2003210
R-NT2RP2003302
Human DNA sequence from 4PTEL, Huntington's Disease Region, chromosome 4p16.3. 1.5e-24:255:78
Z95704
R-NT2RP2003369
Homo sapiens chromosome 7q22 sequence, complete sequence.
3.1e-95:514:92
AF053356
R-NT2RP2003383
Homo sapiens mRNA for KIAA0458 protein, complete cds.
3.9e-111:549:97
AB007927
R-NT2RP2003390
Human DNA sequence *** SEQUENCING IN PROGRESS *** from clone 191J18, WORKING
DRAFT SEQUENCE.
4. 9e-102:413:99
AL024507
R-NT2RP2003469
Genomic sequence from Human 9q34, complete sequence.
1.4e-35:376:74
AC001644
R-NT2RP2003545
Plasmodium falciparum DNA *** SEQUENCING IN PROGRESS *** from MAL4P1, WORKING
DRAFT SEQUENCE.
1.5e-09:503:61
AL034557
R-NT2RP2003593
Human DNA sequence *** SEQUENCING IN PROGRESS *** from clone 971N18, WORKING
DRAFT SEQUENCE.
7.8e-81:433:93
AL021396
R-NT2RP2003599
HS_3240_A1_C04_T7 CIT Approved Human Genomic Sperm Library D Homo sapiens genomic clone Plate=3240 Col=7 Row=E, genomic survey sequence.
0.091:341:58
AQ206348
R-NT2RP2003655
Homo sapiens PAC clone DJ0015123 from 22, complete sequence.
2.0e-08:249:69
AC004819
R-NT2RP2003664
Homo sapiens mRNA for leptin receptor gene-related protein.
1.7e-110:549:96
Y12670
R-NT2RP2003931
Human mRNA for KIAA0365 gene, partial cds.
5.4e-112:571:96
AB002363
R-NT2RP2003940
Human Chromosome 11 pac pDJ1173a5, complete sequence.
2.4e-20:353:70
AC000378
R-NT2RP2003950
Homo sapiens clone 24778 unknown mRNA.
1.5e-96:494:95
AF070572
R-NT2RP2004069
Human DNA sequence from clone 618F1 on chromosome Xq25 Contains part of gene similar to DOC4, CA repeat, GSS, complete sequence.
2.6e-50:539:75
AL023878
R-NT2RP2004108
RPCI11-91F9.TV RPCI11 Homo sapiens genomic clone R-91F9, genomic survey sequence.
0.00013:281:63
AQ283338
R-NT2RP2004141
cSRL-115f11-u cSRL flow sorted Chromosome 11 specific cosmid Homo sapiens genomic clone cSRL-115f11, genomic survey sequence.
2.3e-05:239:64
B00539
R-NT2RP2004179
Genomic sequence from Human 9q34, complete sequence.
0.43:130:68
AC002322
R-NT2RP2004205
Homo sapiens chromosome 7q22 sequence, complete sequence.
1.4e-42:324:83
AF053356
R-NT2RP2004447
Homo sapiens Chromosome 11q13 BAC Clone 18h3, WORKING DRAFT SEQUENCE, 7 ordered pieces.
5.5e-35:285:84
AC000353
R-NT2RP2004495
transcript ch123.Rev [human, RF1,RF48 stomach cancer cell lines, mRNA, 252 nt].
3.4e-44:238:97
S77359
R-NT2RP2004524
Genomic sequence from Human 9q34, complete sequence.
7.4e-113:572:96
AC001644
R-NT2RP2004556
CIT-HSP-2306F6.TF CIT-HSP Homo sapiens genomic clone 2306F6, genomic survey sequence.
8.1e-99:514:95
AQ019229
R-NT2RP2004606
cDNA encoding NIC(Natural Inhibitor of Collagenase).
8.2e-116:576:96
E00985
R-NT2RP2004648
Homo sapiens chromosome 17, clone hRPK.269_G_24, complete sequence. 0.98:369:57
AC005828
R-NT2RP2004670
Rattus norvegicus vesicla-associate calmodulin-binding protein mRNA, complete cds.
4.5e-43:592:69
L22557
R-NT2RP2004794
R-NT2RP2004837
Human Chromosome 11 pac pDJ148f1, WORKING DRAFT SEQUENCE, 27 unordered pieces. 1.2e-60:366:90
AC001232
R-NT2RP2004847
Homo sapiens full length insert cDNA clone YY87C09.
1.0e-68:333:100
AF086055
R-NT2RP2005027
Human glucose transporter-like protein-III (GLUT3), complete cds.
7.8e-103:508:97
M20681
R-NT2RP2005069
Rat vacuolar protein sorting homolog r-vps33b mRNA, complete cds.
3.8e-42:463:73
U35245
R-NT2RP2005163
CIT-HSP-2348J20.TF CIT-HSP Homo sapiens genomic clone 2348J20, genomic survey sequence.
7.4e-44:233:96
AQ059444
R-NT2RP2005181
Rattus norvegicus mRNA for cationic amino acid transporter 3, complete cds. 7.6e-53:567:73
AB000113
R-NT2RP2005247
Homo sapiens Xp22 Cosmid U151G1 (from Lawrence Livermore X library) and PAC RPC11-93D11 (from Roswell Park Cancer Center) complete sequence.
5.8e-38:341:76
AC002357
R-NT2RP2005378
Homo sapiens full length insert cDNA clone YW25A12.
0.13:152:66
AF086029
R-NT2RP2005391
HS_3056_A1_C03_MR CIT Approved Human Genomic Sperm Library D Homo sapiens genomic clone Plate=3056 Col=5 Row=E, genomic survey sequence.
1.1e-14:140:84
AQ134064
R-NT2RP2005425
Homo sapiens mRNA for KIAA0803 protein, partial cds.
8.2e-100:526:94
AB018346
R-NT2RP2005463
R-NT2RP2005514
R-NT2RP2005535
Homo sapiens DNA from chromosome 19, BAC 33152, complete sequence.
1.9e-11:488:62
AC003973
R-NT2RP2005541
CIT-HSP-2034G23.TF CIT-HSP Homo sapiens genomic clone 2034G23, genomic survey sequence.
2.7e-61:311:98
B74709
R-NT2RP2005597
R-nnnnnnnnnnnn
{FRA16A, folate-sensitive fragile site} [human, Genomic, 160 nt] .
0. 92:104:65
S70397
R-NT2RP2005666
R-NT2RP2005774
Homo sapiens apoptosis-related mRNA, 3'UTR, partial sequence.
2.2e-94:440:96
AF035364
R-NT2RP2005878
Homo sapiens chromosome 19, cosmid F17987, complete sequence.
1.3e-32:340:76
AC004790
R-NT2RP2005883
Human DNA sequence from clone 248E1 on chromosome 6q23.1-23.3 Contains DOPAMINE-BETA-MONOOXYGENASE PRECURSOR, EF-1-ALPHA-2 pseudogene EST GSS and CA repeat, complete sequence.
1. 3e-117:581 :97
AL023578
R-NT2RP2005887
Human Chromosome 11 pac pDJ148f1, WORKING DRAFT SEQUENCE, 27 unordered pieces. 2.5e-61:367:90
AC001232
R-nnnnnnnnnnnn
Human paired box gene (PAX6) homologue, complete cds.
5.0e-115:578:96
M93650
R-NT2RP2005994
Homo sapiens chromosome 4 clone B207D4 map 4q25, complete sequence.
2.4e-116:594:96
AC004050
R-NT2RP2006004
Rattus norvegicus Shal-related potassium channel Kv4.3 mRNA, complete cds. 1.8e-45:264:93
U42975
R-NT2RP2006042
T31H24TF TAMU Arabidopsis thaliana genomic clone T31H24, genomic survey sequence.
0.42:111:70
B78148
R-NT2RP2006092
Homo sapiens chromosome 5, BAC clone 319C17 (LBNL H159), complete sequence. 1.7e-73:385:95
AC005214
R-NT2RP2006099
Homo sapiens PAC clone DJ0903G02, complete sequence.
1.3e-27:335:74
AC004924
R-NT2RP2006134
Homo sapiens chromosome 4 clone B139M23 map 4q25, complete sequence. 1.0:143:63
AC004045
R-NT2RP2006269
Phreatamoeba balamuthi UB13 sequence, putative polyubiquitin gene. 0.82:153:63
AJ000657
R-NT2RP2006512
Homo sapiens clone 23904 mRNA sequence.
4.6e-106:531:96
AF052129
R-NT2RP3000011
HS_2196_A2_E08_MR CIT Approved Human Genomic Sperm Library D Homo sapiens genomic clone Plate=2196 Col=16 Row=I, genomic survey sequence.
1.3e-36:292:83
AQ210450
R-NT2RP3000022
Plasmodium falciparum DNA *** SEQUENCING IN PROGRESS *** from contig 3-15, complete sequence.
0.28:248:60
Z98550
R-NT2RP3000059
Homo sapiens chick ovalbumin upstream promoter transcription factor II (COUP-TFII) mRNA, partial cds.
0.047:393:61
M62760
R-NT2RP3000063
HS_3190_B2_D10_MR CIT Approved Human Genomic Sperm Library D Homo sapiens genomic clone Plate=3190 Col=20 Row=H, genomic survey sequence.
0.88:232:63
AQ172428
R-nnnnnnnnnnnn
RPC111-35A1.TJ RPCI-11 Homo sapiens genomic clone RPCI-11-35A1, genomic survey sequence.
3.8e-29:159:99
AQ045699
R-NT2RP3000148
Homo sapiens full length insert cDNA clone ZE03A07.
2.8e-112:574:95
AF086510
R-NT2RP3000169
Homo sapiens MRS1 mRNA, complete cds.
4.4e-110:551:96
AF093239
R-NT2RP3000171
R-NT2RP3000172
Rattus norvegicus vesicla-associate calmodulin-binding protein mRNA, complete cds.
1.3e-40:554:70
L22557
R-NT2RP3000201
Homo sapiens BAG clone NH0353P23 from 2, complete sequence.
6.4e-96:478:97
AC005035
R-NT2RP3000232
Plasmodium falciparum MAL3P2, complete sequence.
0.93:262:61
AL034558
R-NT2RP3000304
Homo sapiens LDL receptor-related protein 6 (LRP6) mRNA, complete cds. 2.4e-109:546:97
AF074264
R-NT2RP3000378
Mus musculus mSin3A (sin3A) mRNA, complete cds.
3.0e-27:411:72
U22394
R-NT2RP3000436
Plasmodium falciparum chromosome 2, section 35 of 73 of the complete sequence. 1.1e-06:440:57
AE001398
R-NT2RP3000444
Human DNA sequence *** SEQUENCING IN PROGRESS *** from clone 889J22, WORKING
DRAFT SEQUENCE.
5.9e-105:543:95
AL031406
R-NT2RP3000460
Canis familiaris sec61 homologue mRNA, complete cds.
2.8e-12:292:68
M96629
R-NT2RP3000481
WORKING DRAFT SEQUENCE, 8 unordered pieces.
0.99:160:65
AC005992
R-NT2RP3000616
Plasmodium falciparum 3D7 chromosome 12 PFYAC492 genomic sequence, WORKING DRAFT SEQUENCE, 5 unordered pieces.
0.00087:412:57
AC005308
R-NT2RP3000645
R-NT2RP3000652
Homo sapiens DNA from chromosome 19, cosmid R32532, complete sequence.
1.9e-44:539:74
AC004004
R-NT2RP3000676
Homo sapiens mRNA for KIAA0446 protein, complete cds.
3.1e-103:542:94
AB007915
R-NT2RP3000677
Homo sapiens genomic DNA of 9q32 anti-oncogene of flat epitherium cancer , segment 4/10.
0.067:235:61
AB020872
R-NT2RP3000721
CIT-HSP-2348J20.TF CIT-HSP Homo sapiens genomic clone 2348J20, genomic survey sequence.
4.0e-45:233:98
AQ059444
R-NT2RP3000789
R-NT2RP3000818
Human DNA sequence *** SEQUENCING IN PROGRESS *** from clone 94M16, WORKING
DRAFT SEQUENCE.
5.7e-95:510:93
Z97201
R-NT2RP3000820
RPC111-77B13.TJ RPC111 Homo sapiens genomic clone R-77B13, genomic survey sequence.
2.1e-50:266:96
AQ283547
R-NT2RP3000838
Homo sapiens mRNA for KIAA0638 protein, partial cds.
4.6e-99:522:94
AB014538
R-NT2RP3000871
Homo sapiens clone DJ0703P08, WORKING DRAFT SEQUENCE, 23 unordered pieces. 0.68:249:61
AC005481
R-NT2RP3000907
X.laevis oocyte repetitive sequence (XLOREP) mRNA.
2.9e-30:386:69
X65290
R-NT2RP3000921
HS_2026_A1_B06_MR CIT Approved Human Genomic Sperm Library D Homo sapiens genomic clone Plate=2026 Col=11 Row=C, genomic survey sequence.
2.2e-54:311:92
AQ232644
R-NT2RP3001012
Rattus norvegicus mRNA for TIP120, complete cds.
9.2e-63:456:83
D87671
R-NT2RP3001044
Human Chromosome 11 pac pDJ148f1, WORKING DRAFT SEQUENCE, 27 unordered pieces. 1.2e-60:366:90
AC001232
R-NT2RP3001061
Plasmodium falciparum 3D7 chromosome 12 PFYAC357 genomic sequence, WORKING DRAFT SEQUENCE, 7 unordered pieces.
0.17:357:61
AC005506
R-NT2RP3001159
Homo sapiens Chromosome 11q12.2 PAC clone pDJ519o13 containing human gene for ferritin heavy chain (FTH), complete sequence.
8.8e-111:561:96
AC004228
R-NT2RP3001170
Homo sapiens mRNA for KIAA0784 protein, partial cds.
8.8e-117:561:98
AB018327
R-NT2RP3001195
Genomic sequence from Human 9q34, complete sequence.
1.4e-35:376:74
AC001644
R-NT2RP3001240
Canis familiaris sec61 homologue mRNA, complete cds.
2.8e-12:292:68
M96629
R-NT2RP3001271
Homo sapiens chromosome 19, BAC CIT-B-470f8 (BC330812), complete sequence. 7.9e-17:260:71
AC006115
R-NT2RP3001322
Human DNA sequence from PAC 128N22 on chromosome Xq25-Xq26.3. contains STS. 0.035:451:60
Z97629
R-NT2RP3001542
Plasmodium falciparum 3D7 chromosome 12 PFYAC812 genomic sequence, WORKING DRAFT SEQUENCE, 8 unordered pieces.
4.1e-08:500:61
AC004153
R-NT2RP3001560
Mouse mRNA for thymic epithelial cell surface antigen, complete cds.
1.0e-30:523:65
D67067
R-NT2RP3001592
R-NT2RP3001685
Human DNA sequence from clone 91J24 on chromosome 6q24 Contains part of utrophin Gene, part of cytochrome C oxidase gene, EST, CpG island, complete sequence.
2.4e-30:147:85
AL024474
R-NT2RP3001738
Homo sapiens Chromosome 11q12.2 PAC clone pDJ519o13 containing human gene for ferritin heavy chain (FTH), complete sequence.
9.2e-107:553:95
AC004228
R-NT2RP3001754
Human DNA sequence *** SEQUENCING IN PROGRESS *** from clone 50024, WORKING
DRAFT SEQUENCE.
2.0e-67:345:97
AL034380
R-NT2RP3001858
R-NT2RP3001976
Homo sapiens chromosome 9, clone hRPK.467_F_21, complete sequence.
4.4e-14:302:62
AC006239
R-NT2RP3002015
Homo sapiens clone DJ0539M06, WORKING DRAFT SEQUENCE, 10 unordered pieces. 6.2e-65:492:82
AC004832
R-NT2RP3002160
Genomic sequence from Human 9q34, complete sequence.
2.1e-82:431:95
AC001643
R-NT2RP3002281
Homo sapiens mRNA for KIAA0765 protein, partial cds.
1.1e-81:446:93
AB018308
R-NT2RP3002286
Mus musculus EGF repeat transmembrane protein mRNA, complete cds.
1.0e-80:378:90
U57368
R-NT2RP3002311
Homo sapiens chromosome 17, clone hRPK.269_G_24, complete sequence. 0.57:366:58
AC005828
R-NT2RP3002324
H.sapiens gene for nitric oxide synthase (promoter region).
1.6e-30:337:72
Z49251
R-NT2RP3002342
transcript ch123. Rev [human, RF1, RF48 stomach cancer cell lines, mRNA, 252 nt].
6.5e-45:238:98
S77359
R-NT2RP3002353
Human Chromosome 16 BAC clone CIT987SK-A-418G10, complete sequence. 0.00015:164:70
AC002044
NNNNNNNNNNNNNN
Homo sapiens mRNA for KIAA0788 protein, partial cds.
4.5e-98:493:96
AB018331
NNNNNNNNNNNNNN
R-NT2RP3002448
S.cerevisiae DNA for or i 2.
0.52:91:71
X59535
R-NT2RP3002571
R-NT2RP3002664
Homo sapiens full length insert cDNA clone ZC48G09.
9.9e-103:522:96
AF086209
R-NT2RP3002721
R-NT2RP3002737
R-NT2RP3002738
Sequence 4 from patent US 5541109.
2.9e-22:171:74
I24014
R-NT2RP3002790
R-NT2RP3002836
Bos taurus retina specific RGS protein (RET-RGS1) mRNA, complete cds. 2.3e-34:384:75
U89254
R-NT2RP3002887
Mus musculus cathepsin S (CatS) gene, promoter region and exons 1 and 2. 1.6e-05:435:62
AF051726
R-NT2RP3002900
Homo sapiens mRNA from chromosome 5q21-22, clone:A3-B.
1.3e-116:569:97
AB002451
R-NT2RP3002958
Homo sapiens clone 23851 mRNA sequence.
2.0e-117:575:98
AF035313
R-NT2RP3002983
Homo sapiens genomic DNA, chromosome 21q11.1, segment 17/28, WORKING DRAFT
SEQUENCE.
5.1e-59:295:99
AP000046
R-NT2RP3003000
Homo sapiens clone 24597 mRNA sequence.
6.1e-109:562:95
AF070604
R-NT2RP3003076
R-NT2RP3003354
Human protocadherin 42 mRNA, complete cds for abbreviated PC42.
0.87:208:61
L11370
R-NT2RP3003448
High throughput sequencing of human chromosome 12, WORKING DRAFT SEQUENCE, 1 ordered pieces.
1.3e-41:287:80
AC005840
R-NT2RP3003469
Human DNA sequence from clone 1177E19 on chromosome 1p36.12-36.31. Contains the 3' part of the DNA-binding Zinc finger protein RIZ gene, ESTs, an STS, GSSs and a CpG island, complete sequence.
2.1e-18:223:77
AL031277
R-NT2RP3003473
Human DNA sequence *** SEQUENCING IN PROGRESS *** from clone 250D10, WORKING
DRAFT SEQUENCE.
1.5e-54:294:96
Z99716
R-NT2RP3003527
Homo sapiens mRNA for protein kinase Dyrk1B.
5.1e-91:445:97
Y17999
R-NT2RP3003532
Mus musculus cell surface molecule OX-2 mRNA, complete cds.
1.2e-30:529:67
AF004023
R-nnnnnnnnnnnn
Homo sapiens PAC clone DJ0531G15 from 7p21, complete sequence.
0.13:294:61
AC004739
R-NT2RP3003559
CIT-HSP-2307F17.TR CIT-HSP Homo sapiens genomic clone 2307F17, genomic survey sequence.
1.4e-15:342:68
AQ016972
R-NT2RP3003614
Homo sapiens chromosome 19, cosmid F21967, complete sequence.
0.013:174:64
AC005256
R-NT2RP3003729
R-NT2RP3003849
Genomic sequence from Human 9q34, WORKING DRAFT SEQUENCE, 6 unordered pieces. 8.9e-106:557:94
AC002320
R-NT2RP3003874
Homo sapiens incomplete cDNA for a mutated allele of a myosin class I, myh-1c. 1.6e-55:302:94
AJ001381
R-NT2RP3003963
cSRL-66f9-u cSRL flow sorted Chromosome 11 specific cosmid Homo sapiens genomic clone cSRL-66f9, genomic survey sequence.
0.028:78:76
B05608
R-NT2RP3004000
Tapa-1=integral membrane protein TAPA-1 [mice, B cell lymphoma line 38C13, Genomic, 861 nt, segment 7 of 7].
0.87:212:62
S45012
R-NT2RP3004025
Homo sapiens chromosome 19, cosmid F17987, complete sequence.
0.71:197:62
AC004790
R-NT2RP3004075
R-NT2RP3004083
Arabidopsis thaliana DNA chromosome 4, ESSA I contig fragment No. 5. 0.27:375:59
Z97340
R-NT2RP3004090
CIT-HSP-2172H20.TR CIT-HSP Homo sapiens genomic clone 2172H20, genomic survey sequence.
2.2e-40:243:91
B99962
R-NT2RP3004119
Homo sapiens PAC clone DJ1059M17 from 7q21-q31.1, complete sequence.
8.3e-42:475:73
AC004953
R-NT2RP3004130
R-NT2RP3004133
CIT-HSP-2306G15.TR CIT-HSP Homo sapiens genomic clone 2306G15, genomic survey sequence.
0.00037:194:64
AQ022229
R-NT2RP3004202
Homo sapiens BAC clone GS285F21 from 7q21-q22, complete sequence.
0.65:209:62
AC004012
R-NT2RP3004294
R-NT2RP3004309
Homo sapiens Chromosome 11q12.2 PAC clone pDJ519o13 containing human gene for ferritin heavy chain (FTH), complete sequence.
7.4e-99:500:96
AC004228
R-NT2RP3004321
Human chromosome 11 168h3 cosmid, complete sequence.
1.7e-105:540:96
U73637
R-NT2RP3004345
Human BAC clone RG016J04 from 7q21, complete sequence.
0.00033:348:61
AC002064
R-NT2RP3004355
Plasmodium falciparum 3D7 chromosome 12 PFYAC1122 genomic sequence, WORKING DRAFT SEQUENCE, 3 unordered pieces.
0.0029:180:66
AC004709
R-NT2RP3004374
Human DNA sequence from clone 1177E19 on chromosome 1p36.12-36.31. Contains the 3' part of the DNA-binding Zinc finger protein RIZ gene, ESTs, an STS, GSSs and a CpG island, complete sequence.
4.3e-18:223:77
AL031277
R-NT2RP3004406
Plasmodium falciparum DNA *** SEQUENCING IN PROGRESS *** from contig 4-70, complete sequence.
1.0:201:62
AL010239
R-NT2RP3004481
R-NT2RP3004552
Human germline immunoglobulin lambda light chain pseudogene (VII.1). 1.0:165:63
X57825
R-NT2RP3004625
Homo sapiens I-1 receptor candidate protein mRNA, complete cds.
8.2e-49:352:84
AF082516
R-NT2RP3004640
Homo sapiens full length insert cDNA clone ZC45E05.
1.2e-96:471:98
AF086205
R-NT2RP3004647
Homo sapiens mRNA for KIAA0446 protein, complete cds.
1.5e-109:555:96
AB007915
R-NT2RP4000108
Mouse neurofilament protein (NF-L) gene, 3' flank.
1.0e-09:344:63
M55424
R-NT2RP4000634
Homo sapiens Xp22 BAC GSHB 526D21 (Genome Systems Human BAC library) complete sequence.
1.6e-16:267:71
AC003037
R-NT2RP4000962
Human DNA sequence from PAC 970D1 on chromosome 1q24. Contains ESTs, STSs and a BAC end-sequence (GSS).
0.026:176:67
AL021069
R-NT2RP4001001
Drosophila melanogaster Oregon-R mitochondrial A+T region.
0.00026:354:61
U11584
R-NT2RP4001009
Homo sapiens mRNA for Hs Ste24p, complete cds.
1.6e-82:408:98
AB016068
R-NT2RP4001467
Human placental cDNA coding for 5'nucleotidase (EC 3.1.3.5).
1.8e-111:545:97
X55740
R-NT2RP4001877
Yeast (S.cerevisiae) mitochondrial cob gene, intron 4.
0.19:384:59
J01469
R-NT2RP4001879
Homo sapiens full length insert cDNA clone ZD76G10.
4.4e-107:548:94
AF086408
R-NT2RP4002187
RPCI11-69F22.TK RFCI11 Homo sapiens genomic clone R-69F22, genomic survey sequence.
7.1e-37:240:89
AQ238297
R-NT2RP4002451
Human DNA sequence *** SEQUENCING IN PROGRESS *** from clone 111B22, WORKING
DRAFT SEQUENCE.
5.8e-111:575:96
Z98200
R-NT2RP4002715
Human Chromosome 11 pac pDJ148f1, WORKING DRAFT SEQUENCE, 27 unordered pieces. 2.5e-61:367:90
AC001232
R-NT2RP4002750
Rattus norvegicus mRNA for cationic amino acid transporter 3, complete cds. 1.2e-52:527:74
AB000113
R-OVARC1000003
Homo sapiens clone DJ0856024, WORKING DRAFT SEQUENCE, 4 unordered pieces. 2.3e-10:140:77
AC004909
R-OVARC1000090
Homo sapiens genomic DNA, 237 kb segment from 6p21.3 region including HLA genes, WORKING DRAFT SEQUENCE.
2.8e-59:323:78
D84394
R-OVARC1000105
H.sapiens gene for ribosomal protein L38.
2.7e-12:83:100
Z26876
R-OVARC1000137
R-OVARC1000208
Homo sapiens PAC clone DJ0817118 from 7p11.2p13, complete sequence.
2.7e-52:464:79
AC004901
R-OVARC1000255
H.sapiens syk mRNA for protein-tyrosine kinase.
1.9e-105:511:98
Z29630
R-OVARC1000275
R-OVARC1000298
Plasmodium falciparum carbamoyl phosphate synthetase II gene, complete cds. 0.66:364:59
L32150
R-OVARC1000307
R-OVARC1000313
Homo sapiens mRNA for KIAA0573 protein, partial cds.
1.6e-96:534:93
AB011145
R-OVARC1000331
Sequence 2 from patent US 5763589.
8.1e-66:335:97
AR012692
R-OVARC1000410
Homo sapiens clone 23767 and 23782 mRNA sequences.
1.0e-88:462:94
AF007150
R-OVARC1000439
E.coli fanG and fanH genes.
0. 99:424:58
Y00531
R-OVARC1000467
HS_3235_A2_A12_T7 CIT Approved Human Genomic Sperm Library D Homo sapiens genomic clone Plate=3235 Col=24 Row=A, genomic survey sequence.
9.2e-09:125:76
AQ206826
R-OVARC1000529
R-OVARC1000553
Homo sapiens chromosome 19, cosmid R26894, complete sequence.
2.0e-84:437:96
AC005594
R-OVARC1000775
CIT-HSP-2060P5.TF CIT-HSP Homo sapiens genomic clone 2060P5, genomic survey sequence.
0.29:223:63
B70025
R-OVARC1000811
Homo sapiens chromosome 16, cosmid clone 432A1 (LANL), complete sequence. 2.8e-89:438:98
AC004235
R-OVARC1000853
RPC111-16C11.TV RPCI-11 Homo sapiens genomic clone RPCI-11-16C11, genomic survey sequence.
5.3e-53:317:92
B76661
R-OVARC1000873
Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.
1.2e-102:511:97
AL034418
R-OVARC1000916
H. sapiens PISSLRE mRNA.
5.8e-111:588:94
X78342
R-OVARC1000956
Human DNA sequence from cosmid L241B9, Huntington's Disease Region, chromosome 4p16.3 contains polymorphic VNTR pYNZ32.
2.7e-89:478:94
Z69708
R-OVARC1000995
Human DNA sequence from clone 885E17 on chromosome 1p33-34.1. Contains STSs and GSSs and genomic marker D1S1302, complete sequence.
9.5e-46:325:84
AL031294
R-OVARC1001030
Homo sapiens neuroendocrine-specific protein C (NSP) mRNA, complete cds. 1.2e-05:197:63
L10335
R-OVARC1001049
R-OVARC1001086
Homo sapiens cyclin T2a mRNA, complete cds.
4.3e-105:569:94
AF048731
R-OVARC1001132
Homo sapiens genomic DNA, chromosome 21q11.1, segment 9/28, WORKING DRAFT
SEQUENCE.
2.2e-111:569:96
AP000038
R-OVARC1001163
Sus scrofa MHC SLA class III steroid 21-hydroxylase (CYP21) gene, complete cds, ORF human-like gene, last 5 exons.
0.010:349:60
M83939
R-OVARC1001222
Spiroplasma citri orfa and orff genes, partial cds, orfb, orfc, and orfe genes and Spiroplasma virus SpV1-derived ORF1 and ORF3 genes, complete cds, and SpV1-derived ORF14 gene, partial cds.
0.58:184:60
U28972
R-OVARC1001260
Homo sapiens clone DJ0856024, WORKING DRAFT SEQUENCE, 4 unordered pieces. 1.1e-10:140:78
AC004909
R-OVARC1001336
Homo sapiens clone DJ0856024, WORKING DRAFT SEQUENCE, 4 unordered pieces. 2.3e-10:140:77
AC004909
R-OVARC1001338
R-OVARC1001569
R-OVARC1001570
R-OVARC1001596
Homo sapiens chromosome 17, clone hRPK.372_K_20, complete sequence.
5.9e-47:361:83
AC005951
R-OVARC1001607
Human beta-1,2-N-acetylglucosaminyltransferase II (MGAT2) gene, complete cds. 3.3e-112:559:96
U15128
R-OVARC1001725
Homo sapiens patched related protein TRC8 (TRC8) gene, exon 1 and partial cds.
3. 9e-56:318:95
AF064800
R-OVARC1001727
Human DNA sequence from clone 48A11 on chromosome 20p12 Contains EST, STS, GSS, complete sequence.
6. 1e-101:533:94
AL031132
R-OVARC1001807
Human TR3 orphan receptor mRNA, complete cds.
2.8e-87:426:97
L13740
R-OVARC1001833
Mouse fork head related protein (HNF-3beta) mRNA, complete cds.
1.1e-21:263:76
L10409
R-OVARC1001991
H.sapiens chromosome 22 CpG island DNA genomic Mse1 fragment, clone 301e3, reverse read 301e3.r.
0.90:151:59
Z79826
R-OVARC1002058
Homo sapiens full length insert cDNA clone ZD58C02.
1.9e-105:547:95
AF088043
R-OVARC1002178
Human DNA sequence from clone 267M20 on chromosome Xq22.2-22.3. Contains part of the DIAPH2 gene and a pseudogene, ESTs, STSs and GSSs, complete sequence. 0.26:429:58
AL031053
R-PLACE1000033
Plasmodium falciparum 3D7 chromosome 12 PFYAC69 genomic sequence, WORKING DRAFT SEQUENCE, 4 unordered pieces.
0.098:467:59
AC004688
R-PLACE1000231
Homo sapiens BAC clone RG060N22 from 7q21, complete sequence.
0.91:141:64
AC003083
R-PLACE1000258
Human DNA sequence from clone 710L4 on chromosome Xq11.2-12 Contains part of a gene similar to myotubularin-related protein, EST, STS and GSS, complete sequence.
3.8e-53:524:75
AL034408
R-PLACE1000442
Arabidopsis thaliana genomic DNA, chromosome 5, TAC clone: K22F20, complete sequence.
3.0e-07:413:62
AB016873
R-PLACE1000560
Homo sapiens chromosome 5, BAC clone 194j18 (LBNL H158), complete sequence. 6.3e-59:323:94
AC005368
R-PLACE1000740
H.sapiens PEX gene.
0.0065:202:63
Y10196
R-PLACE1000912
R-PLACE1000914
Homo sapiens chromosome 17, clone HCIT145P4, WORKING DRAFT SEQUENCE, 8 unordered pieces.
3.4e-68:452:86
AC002093
R-PLACE1000927
Cowpox virus strain GRI-90 DNA (49 kb fragment).
1.8e-46:432:76
Y15035
R-PLACE1000986
HS_2037_A2_B06_MR CIT Approved Human Genomic Sperm Library D Homo sapiens genomic clone Plate=2037 Col=12 Row=C, genomic survey sequence.
0.087:48:89
AQ232754
R-PLACE1001016
M.fascicularis gene for apolipoprotein A-IV.
0.016:226:61
X68361
R-PLACE1001100
Human DNA sequence from PAC 42616 on chromosome 1p34.1-1p35. Contains NIPP-1-like gene a nuclear inhibitor of protein phosphatase-1, ESTs, and a CA repeat. 3.4e-37:320:80
AL020997
R-PLACE1001114
RPC111-5C23.TV RPCI-11 Homo sapiens genomic clone RPCI-11-5C23, genomic survey sequence.
9.2e-44:173:85
B49180
R-PLACE1001123
R-PLACE1001183
Plasmodium falciparum MAL3P8, complete sequence.
0.47:217:63
AL034560
R-PLACE1001229
Mitochondrion Culex torrentium A+T rich mitochondrial control region.
3.3e-09:356:63
U69573
R-PLACE1001231
Rattus norvegicus sodium-dependent multi-vitamin transporter (SMVT) mRNA, complete cds.
1.2e-09:186:72
AF026554
R-PLACE1001340
Homo sapiens mRNA for KIAA0719 protein, complete cds.
2.0e-51:265:98
AB018262
R-PLACE1001401
Bactrocera dorsalis strain Tahiti mitochondrial D-loop region, complete sequence.
0.0073:203:60
AF033929
R-PLACE1001407
Human DNA sequence from clone 496H19 on chromosome 6q24 Contains ESTs, complete sequence.
5.8e-70:360:96
AL023582
R-PLACE1001464
Human placental cDNA coding for 5'nucleotidase (EC 3.1.3.5).
3.1e-90:457:96
X55740
R-PLACE1001500
Homo sapiens clone DJ0917G04, WORKING DRAFT SEQUENCE, 35 unordered pieces. 1.0:232:62
AC004929
R-PLACE1001516
Homo sapiens Chromosome 16 BAC clone CIT987SK-A-575C2, complete sequence. 1.9e-26:168:88
AC002425
R-PLACE1001536
Human Chromosome X clone bWXD187, complete sequence.
6.5e-61:310:98
AC004383
R-PLACE1001564
Human DNA sequence *** SEQUENCING IN PROGRESS *** from clone 287G14, WORKING
DRAFT SEQUENCE.
2.9e-100:504:97
AL033377
R-PLACE1001655
Homo sapiens Shab-related delayed-rectifier K+ channel alpha subunit (KCNS3) mRNA, complete cds.
3.8e-117:578:97
AF043472
R-PLACE1001788
Sequence 9 from Patent W09722695.
1.9e-05:91:82
A63556
R-PLACE1001795
R-PLACE1001836
, complete sequence.
4.1e-14:269:69
AC005406
R-PLACE1001918
Human HepG2 3' region Mbol cDNA, clone hmd4f06m3.
7.3e-25:151:95
D17237
R-PLACE1001949
Human DNA sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.
0. 54:165:63
Z94056
R-PLACE1002080
Homo sapiens chromosome 17, clone hRPC.971_F_3, WORKING DRAFT SEQUENCE, 1 ordered pieces.
3.7e-60:289:95
AC004150
R-PLACE1002095
Plasmodium falciparum DNA *** SEQUENCING IN PROGRESS *** from contig 3-52, complete sequence.
0.00052:422:59
AL008982
R-PLACE1002153
Homo sapiens TACC2 protein (TACC2) mRNA, partial cds.
4.6e-100:514:95
AF095791
R-PLACE1002329
Homo sapiens chromosome 19, cosmid R31855, complete sequence.
1.3e-46:257:95
AC005782
R-PLACE1002355
HS_2057_B1_D01_T7 CIT Approved Human Genomic Sperm Library D Homo sapiens genomic clone Plate=2057 Col=1 Row=H, genomic survey sequence.
0.089:132:65
AQ245227
R-PLACE1002374
Human mRNA for pro-cathepsin L (major excreted protein MEP).
2.6e-101:501:97
X12451
R-PLACE1002518
HS_2176_A2_D04_MR CIT Approved Human Genomic Sperm Library D Homo sapiens genomic clone Plate=2176 Col=8 Row=G, genomic survey sequence.
1. 7e-43:221:100
AQ014851
R-PLACE1002547
Homo sapiens mRNA for KIAA0719 protein, complete cds.
2.0e-53:276:97
AB018262
R-PLACE1002726
Human DNA-binding protein ABP/ZF mRNA, complete cds.
1.1e-37:212:94
U82613
R-PLACE1002905
Human DNA sequence *** SEQUENCING IN PROGRESS *** from clone 215D11, WORKING
DRAFT SEQUENCE.
1.2e-42:302:86
AL034417
R-PLACE1002911
R-PLACE1002967
Homo sapiens chromosome 16, BAC clone 461A8 , complete sequence.
2.3e-39:310:82
AC006111
R-PLACE1003135
Plasmodium falciparum DNA *** SEQUENCING IN PROGRESS *** from MAL4P1, WORKING
DRAFT SEQUENCE.
2.2e-07:418:60
AL034557
R-PLACE1003163
Homo sapiens DBI-related protein mRNA, complete cds.
4.7e-110:547:96
AF069301
R-PLACE1003407
Homo sapiens putative transmembrane protein (CLN5) mRNA, complete cds.
1.7e-47:287:91
AF068227
R-PLACE1003428
Human DNA sequence from clone 55C23 on chromosome 6q22.3-23.3 contains vanin-like genes VNN1 and VNN2, ESTs, GSSs,, complete sequence.
1.1e-75:268:98
AL032821
R-PLACE1003438
R-PLACE1003460
HS_3026_B1_A08_MR CIT Approved Human Genomic Sperm Library D Homo sapiens genomic clone Plate=3026 Col=15 Row=B, genomic survey sequence.
0.30:100:69
AQ093523
R-nnnnnnnnnnnn
Homo sapiens clone DJ0981007, complete sequence.
3.3e-46:135:98
AC006017
R-PLACE1003573
HS_3079_B2_A02_MR CIT Approved Human Genomic Sperm Library D Homo sapiens genomic clone Plate=3079 Col=4 Row=B, genomic survey sequence.
1. 1e-49:261:96
AQ121751
R-PLACE1003598
R-PLACE1003644
Caenorhabditis elegans cosmid F52H3, complete sequence.
0.38:345:62
Z66512
R-PLACE1003737
Homo sapiens Xp22-83 BAC GSHB-324M7 (Genome Systems Human BAC Library) complete sequence.
1.9e-77:406:96
AC005859
R-PLACE1003772
Human DNA sequence from PAC 42616 on chromosome 1p34.1-1p35. Contains NIPP-1-like gene a nuclear inhibitor of protein phosphatase-1, ESTs, and a CA repeat. 2.2e-29:454:70
AL020997
R-PLACE1003839
Homo sapiens Chromosome 16 BAC clone CIT987SK-A-69G12, complete sequence. 3.0e-52:272:97
AC004131
R-PLACE1003845
HS_3219_A1_A10_MR CIT Approved Human Genomic Sperm Library D Homo sapiens genomic clone Plate=3219 Col=19 Row=A, genomic survey sequence.
1.5e-13:231:70
AQ181482
R-PLACE1003852
Homo sapiens mRNA for KIAA0758 protein, partial cds.
6.8e-86:439:96
AB018301
R-PLACE1004028
R-PLACE1004078
Homo sapiens 12p13.3 PAC RPC15-940J5 (Roswell Park Cancer Institute Human PAC Library) complete sequence.
5.0e-36:310:80
AC006064
R-PLACE1004166
Petromyzon marinus neurofilament subunit NF-180 mRNA, complete cds. 0.95:224:62
U19361
R-nnnnnnnnnnnn
Fugu rubripes GSS sequence, clone 076D01bH10, genomic survey sequence.
3.0e-08:107:77
AL026605
R-PLACE1004199
Human prostaglandin D2 synthase gene, exons 2 through 6 and complete cds. 0.0028:157:67
M98538
R-PLACE1004279
Human DNA sequence from PAC 193B12 on chromosome 6p21.3-22.3. Contains histone H2A/d, H2B/d, H2A.i, H1.5, H3.F, H4.k, H3/j genes, histone pH2b.i and hypothetical protein A4 pseudogenes, histone H2A.1 and H2B.2 duplicate genes, Glycine (GGC) tRNA gene, olfactory receptor OL1 like gene, ESTs STSs and predicted CpG islands.
0.00065:228:58
Z98744
R-PLACE1004282
R-PLACE1004305
Homo sapiens mRNA for KIAA0740 protein, complete cds.
2.0e-77:377:99
AB018283
R-PLACE1004441
RPCI11-76P13. TV RFCI11 Homo sapiens genomic clone R-76P13, genomic survey sequence.
1.8e-73:370:97
AQ281810
R-PLACE1004450
Arabidopsis thaliana genomic DNA, chromosome 5, TAC clone: K24G6, complete sequence.
0.87:269:59
AB012242
R-PLACE1004482
Mus musculus hematopoietic lineage switch 2 (HLS2) mRNA, complete cds. 5.2e-33:356:75
AF009513
R-PLACE1004492
Mus musculus mRNA for Doc2, partial cds.
4.1e-28:268:77
D50000
R-PLACE1004519
Human DNA sequence from clone 24o18 on chromosome 6p21.31-22.2 Contains zinc finger protein pseudogene, VNO-type olfactory receptor pseudogene, nuclear envelope pore membrane protein, EST, STS, GSS, complete sequence.
1.8e-14:330:67
AL021808
R-PLACE1004520
Human pregnancy specific beta-1-glycoprotein 1 (PSG1) gene.
1.4e-73:397:93
M93705
R-PLACE1004630
R-PLACE1004637
Human Chromosome 11 Cosmid cSRL16b6, complete sequence.
5.5e-108:625:91
U73638
R-PLACE1004648
R-PLACE1004816
Homo sapiens mRNA for Hakata antigen, complete cds.
5.6e-103:586:90
D88587
R-PLACE1004887
CIT-HSP-2172H20.TR CIT-HSP Homo sapiens genomic clone 2172H20, genomic survey sequence.
1.2e-31:177:97
B99962
R-PLACE1005003
Mus musculus clone OST13719, genomic survey sequence.
0.0043:159:64
AF046703
R-PLACE1005005
Homo sapiens ubiquitin conjugating enzyme G2 (UBE2G2) mRNA, complete cds. 2.1e-56:299:95
AF032456
R-PLACE1005031
Homo sapiens chromosome 17, clone hRPK.156_L_14, complete sequence. 1.0:155:65
AC005821
R-PLACE1005239
Homo sapiens mRNA for HIRIP3 protein (clone pH4-31, pH4-17).
4.4e-85:450:93
AJ223351
R-PLACE1005250
Mus musculus maternal transcript Maid (Maid) mRNA, complete cds.
7.7e-19:232:73
U50734
R-PLACE1005383
Homo sapiens UP50 mRNA, complete cds.
2.0e-77:471:88
AF093118
R-PLACE1005410
Canis familiaris sec61 homologue mRNA, complete cds.
6.4e-12:132:82
M96629
R-PLACE1005426
Human pregnancy specific beta-1-glycoprotein 4 (PSG4) mRNA, complete cds. 2.3e-109:574:94
M94891
R-PLACE1005519
Plasmodium falciparum DNA *** SEQUENCING IN PROGRESS *** from MAL4P1, WORKING
DRAFT SEQUENCE.
4.1e-08:426:61
AL034557
R-PLACE1005539
R-PLACE1005544
Homo sapiens chromosome 19, cosmid F20887, complete sequence.
1.0e-17:202:73
AC005578
R-PLACE1005569
Human DNA sequence *** SEQUENCING IN PROGRESS *** from clone 159A1, WORKING
DRAFT SEQUENCE.
3.8e-89:470:95
AL034397
R-PLACE1005601
Homo Sapiens angiotensin II receptor gene, complete cds.
8.0e-52:301:90
L48211
R-PLACE1005660
R-PLACE1005669
Mitochondrion Culex torrentium A+T rich mitochondrial control region.
9.5e-09:338:63
U69573
R-PLACE1005682
Caenorhabditis elegans cosmid M70.
0.012:226:62
AF047661
R-PLACE1005725
Caenorhabditis elegans DNA *** SEQUENCING IN PROGRESS *** from clone Y48E1,
WORKING DRAFT SEQUENCE.
0.42:435:59
Z92856
R-PLACE1005736
Rattus norvegicus DD6A4-1 mRNA, partial sequence.
9.0e-21:282:73
AF034237
R-PLACE1005745
RPC111-88L20.TJ RPCI11 Homo sapiens genomic clone R-88L20, genomic survey sequence.
2.4e-62:310:99
AQ281511
R-PLACE1005768
Human DNA sequence from Fosmid 24E5 on chromosome 22q11.2-qter contains parvalbumin, ESTs, STS.
5.6e-94:511:93
Z82185
R-PLACE1005815
Human Chromosome 16 BAC clone CIT987SK-A-635H12, complete sequence.
9.0e-55:586:73
AC002310
R-PLACE1005878
R-PLACE1005927
R-PLACE1006071
CIT-HSP-2308A18.TR CIT-HSP Homo sapiens genomic clone 2308A18, genomic survey sequence.
1.6e-76:410:95
AQ022149
R-PLACE1006073
Homo sapiens mRNA for glucuronyltransferase I, complete cds.
2.2e-97:513:93
AB009598
R-PLACE1006079
Homo sapiens distal-less homeobox protein (DLX3) gene, complete cds.
5.4e-57:333:91
AF028233
R-PLACE1006093
R-nnnnnnnnnnnn
Caenorhabditis elegans mRNA for GAP-2-7, partial cds.
1.9e-08:251:60
AB011283
R-PLACE1006219
HS_3219_A1_A10_MR CIT Approved Human Genomic Sperm Library D Homo sapiens genomic clone Plate=3219 Col=19 Row=A, genomic survey sequence.
3.1e-12:228:69
AQ181482
R-PLACE1006277
Human DNA sequence *** SEQUENCING IN PROGRESS *** from clone 159A1, WORKING
DRAFT SEQUENCE.
7.8e-96:557:91
AL034397
R-PLACE1006290
Bacillus whitei clone pWH/Cug1 satellite DNA.
0. 37:209:62
U90159
R-PLACE1006443
Homo sapiens chromosome 17, clone HCIT145P4, WORKING DRAFT SEQUENCE, 8 unordered pieces.
8.9e-76:451:91
AC002093
R-PLACE1006515
Homo sapiens mRNA for KIAA0576 protein, partial cds.
2.1e-76:413:94
AB011148
R-PLACE1006716
M.musculus gene encoding prostaglandin D synthase, putative.
1.0:199:59
Y10138
R-PLACE1006786
HS_2037_A2_B06_MR CIT Approved Human Genomic Sperm Library D Homo sapiens genomic clone Plate=2037 Col=12 Row=C, genomic survey sequence.
0.33:47:91
AQ232754
R-PLACE1006809
Homo sapiens Xq28 genomic DNA in the region of the L1CAM locus containing the genes for neural cell adhesion molecule L1 (L1CAM), arginine-vasopressin receptor (AVPR2), C1 p115 (C1), ARD1 N-acetyltransferase related protein (TE2), renin-binding protein (RbP), host cell factor 1 (HCF1), and
interleukin-1 receptor-associated kinase (IRAK) genes, complete cds, and Xq28Iu2 gene.
0.67:241:59
U52112
R-PLACE1006959
R-PLACE1007028
R-PLACE1007040
Rabbit angiotensin-converting enzyme (ACE-P) gene, 5' end.
0.0037:208:65
M58579
R-PLACE1007077
CIT-HSP-2308A18.TR CIT-HSP Homo sapiens genomic clone 2308A18, genomic survey sequence.
3.0e-76:411:94
AQ022149
R-PLACE1007081
RPCI11-31D7.TJ RPCI-11 Homo sapiens genomic clone RPCI-11-31D7, genomic survey sequence.
1.9e-06:88:84
AQ016433
R-PLACE1007096
H.sapiens DMD gene microsatellite (147-200bp).
1.0:142:59
X77677
R-PLACE1007296
R-PLACE1007591.
Human DNA sequence from clone 113J7 on chromosome Xp11.22-11.4. Contains part of a putative Homeobox (pseudo?) gene, ESTs and an STS, complete sequence. 1.6e-11:203:66
AL023574
R-PLACE1007626
Homo sapiens unknown mRNA, complete cds.
4. 9e-29:183:91
AF047439
R-PLACE1007702
Homo sapiens chromosome 17, clone 363G12, WORKING DRAFT SEQUENCE, 11 unordered pieces.
2.3e-75:445:90
AC002348
R-PLACE1007845
CIT-HSP-2306G15.TR CIT-HSP Homo sapiens genomic clone 2306G15, genomic survey sequence.
0.00045:194:64
AQ022229
R-PLACE1007881
CITBI-E1-2503C21.TF CITBI-E1 Homo sapiens genomic clone 2503C21, genomic survey sequence.
0.43:104:69
AQ263355
R-PLACE1007971
R-PLACE1008282
Homo sapiens clone DJ0042M02, WORKING DRAFT SEQUENCE, 20 unordered pieces. 7.7e-73:396:94
AC005995
R-PLACE1008297
N.frontalis enolase gene, promotor region.
1.2e-08:457:57
X81451
R-PLACE1008359
Plasmodium falciparum MAL3P1, complete sequence.
0.00044:443:56
Z97348
R-PLACE1008469
Homo sapiens chromosome 17, clone HCIT145P4, WORKING DRAFT SEQUENCE, 8 unordered pieces.
4.4e-78:536:84
AC002093
R-PLACE1008549
Mus musculus E74-like factor 5 (EIf5) mRNA, complete cds.
3.4e-30:256:75
AF049702
R-PLACE1008657
Homo sapiens BAC clone GS067A24 from 7q21.q21.2, complete sequence.
1.9e-40:320:82
AC005009
R-PLACE1008716
Human beta-1,2-N-acetylglucosaminyltransferase II (MGAT2) gene, complete cds. 8.2e-118:591:96
U15128
R-PLACE1008744
Sequence 1 from patent US 5691147.
3.1e-108:559:94
I76197
R-PLACE1008984
Human DNA sequence *** SEQUENCING IN PROGRESS *** from clone 167A19, WORKING
DRAFT SEQUENCE.
1.6e-102:503:96
AL031427
R-PLACE1008985
Mus musculus synaptotagmin VIII mRNA, partial cds.
9.7e-29:255:77
U20107
R-PLACE1009067
R-PLACE1009196
Caenorhabditis elegans DNA *** SEQUENCING IN PROGRESS *** from clone Y48A6,
WORKING DRAFT SEQUENCE.
0.0094:206:65
Z92854
R-PLACE1009279
Homo sapiens serine protease (PRSS11) mRNA, partial cds.
2.4e-26:553:62
AF097709
R-PLACE1009527
Human DNA-binding protein ABP/ZF mRNA, complete cds.
7.9e-91:497:91
U82613
R-PLACE1009546
Human PAC clone DJ218B13 from Xq23, complete sequence.
0.29:147:64
AC002072
R-PLACE1009600
Mouse mRNA for tetracycline transporter-like protein, complete cds.
6.1e-81:466:90
D88315
R-PLACE1009735
Homo sapiens clone NH0523H20, complete sequence.
2.0e-74:268:99
AC005041
R-nnnnnnnnnnnn
Homo sapiens DNA sequence from PAC 833B2 on chromosome Xq26.1-27.2. Contains an EST, complete sequence.
1.9e-05:255:65
AL023800
R-PLACE1010011
, complete sequence.
2.9e-77:174:100
AC005409
R-PLACE1010078
Human DNA sequence *** SEQUENCING IN PROGRESS *** from clone 209H1, WORKING
DRAFT SEQUENCE.
1.0:108:65
Z84465
R-PLACE1010081
Homo sapiens serine/threonine kinase RICK (RICK) mRNA, complete cds.
9.2e-105:560:93
AF027706
R-PLACE1010251
Plasmodium falciparum MAL3P4, complete sequence.
5.0e-07:468:58
AL008970
R-PLACE1010445
Pan troglodytes HS19.8-similar locus and Y Alu element, genomic survey sequence.
1.2e-49:303:90
AF077058
R-PLACE1010713
RPCI11-69F22. TK RFCI11 Homo sapiens genomic clone R-69F22, genomic survey sequence.
7.4e-11:114:80
AQ238297
R-PLACE1010784
Capra hircus strain Saanen, genomic survey sequence.
7.4e-24:182:87
AF083406
R-PLACE1010827
nbxb0026K23f CUGI Rice BAC Library Oryza sativa genomic clone nbxb0026K23f, genomic survey sequence.
1.0:252:61
AQ271546
R-PLACE1010968
Plasmodium falciparum 3D7 chromosome 12 PFYAC492 genomic sequence, WORKING DRAFT SEQUENCE, 5 unordered pieces.
0.0038:295:57
AC005308
R-PLACE1011045
Homo sapiens E1-like protein mRNA, complete cds.
1.6e-90:453:96
AF094516
R-PLACE1011116
Homo sapiens embryonic lung protein (HUEL) mRNA, complete cds.
4.6e-72:385:94
AF006621
R-PLACE1011236
*** SEQUENCING IN PROGRESS *** Homo sapiens chromosome 4, BAG clone C0289H06; HTGS phase 1, WORKING DRAFT SEQUENCE, 4 unordered pieces.
0.98:142:65
AC004580
R-PLACE1011364
R-PLACE1011407
Human DNA sequence from clone 127F18 on chromosome Xp11.4-21.3. Contains part of a novel gene with some similarity to parts of chicken Myosin Light Chain and various species' Interleukin-1 Receptor Type 1 (IL1-R-1). Contains GSSs, complete sequence.
9.1e-27:293:74
AL031575
R-PLACE1011516
Fugu rubripes GSS sequence, clone 190N14aC12, genomic survey sequence. 1.0:96:68
AL030602
R-PLACE1011708
Homo sapiens intrinsic factor-B12 receptor precursor, mRNA, complete cds. 2.4e-91:521:91
AF034611
R-PLACE1011824
Figure 6. DNA sequence of three 3' apoB VNTR alleles.
2.2e-06:264:65
M23046
R-PLACE1011978
Figure 2. Nucleotide and translated protein sequences of HPF1, -2, and -9. 4.8e-50:553:69
M27877
R-PLACE2000118
Homo sapiens DNA sequence from PAC 393P12 on chromosome Xp11.21. Contains a hypothetical protein KIAA0413 (KIAA0412, KIAA0065, KIAA0569) LIKE Zinc Finger protein pseudogene, a hypothetical Proline-rich protein KIAA0269 LIKE gene and a 60S Ribosomal Protein L7 LIKE pseudogene. Contains ESTs, an STS and a GSS (BAC end sequence), complete sequence.
3.9e-107:567:94
AL022578
R-PLACE2000219
Homo sapiens clone RG132J19, complete sequence.
4.7e-39:317:82
AC005163
R-PLACE3000181
Human protocadherin 42 mRNA, 3' end of cds for alternative splicing PC42-8. 3.9e-81:441:94
L11369
R-PLACE3000213
Sequence 1 from patent US 5691147.
1.5e-109:559:95
I76197
R-PLACE4000354
Sequence 1 from patent US 5691147.
2.7e-106:548:95
I76197
R-PLACE4000455
Arabidopsis thaliana genomic DNA, chromosome 3, P1 clone: MEB5, complete sequence.
9.3e-07:357:60
AB019230
R-THYRO1000036
Sequence 11 from patent US 5773248.
4.0e-99:527:93
AR014074
R-THYRO1000061
Homo sapiens chromosome 19, cosmid R34382, complete sequence.
7.3e-90:460:96
AC005329
R-THYRO1000099
R-THYRO1000196
Homo sapiens Ksp-cadherin (CDH16) mRNA, complete cds.
1.1e-104:530:96
AF016272
R-THYRO1000400
Homo sapiens Chromosome 16 BAC clone CIT987SK-A-233A8, complete sequence. 1.0:308:61
AC004685
R-THYRO1000580
Human Kox26 mRNA for zinc finger protein, partial.
0.11:105:67
X52357
R-THYRO1000584
*** SEQUENCING IN PROGRESS *** Homo sapiens chromosome 4, BAC clone C0539L10; HTGS phase 1, WORKING DRAFT SEQUENCE, 15 unordered pieces.
1.4e-14:241:68
AC004480
R-THYRO1000678
Belonogaster petiolata 16S ribosomal RNA gene, mitochondrial gene for mitochondrial rRNA, partial sequence.
0.049:150:64
AF066910
R-THYRO1000776
CITBI-E1-2505N5.TF.1 CITBI-E1 Homo sapiens genomic clone 2505N5, genomic survey sequence.
0.38:179:63
AQ241670
R-THYRO1000795
R-THYRO1000846
Homo sapiens hJAG2.del-E6 (JAG2) mRNA, alternatively spliced isoform of Jagged2, complete cds.
3.6e-06:425:61
AF029779
R-THYRO1000866
Homo sapiens SKB1Hs mRNA, complete cds.
4.0e-42:251:92
AF015913
R-THYRO1000956
R-THYRO1000964
Human Chromosome 11 Cosmid cSRL186g7, complete sequence.
0.18:292:61
U73627
R-THYRO1000999
CIT-HSP-2288E24.TR CIT-HSP Homo sapiens genomic clone 2288E24, genomic survey sequence.
3.6e-18:292:71
AQ002356
R-THYRO1001063
Homo sapiens chromosome 16 BAC clone CIT987SK-381E11 complete sequence. 1.5e-27:292:76
AF001552
R-THYRO1001071
Human DNA sequence *** SEQUENCING IN PROGRESS *** from clone 37E16, WORKING
DRAFT SEQUENCE.
1.7e-105:513:98
Z83844
R-THYRO1001102
Homo sapiens clone DJ0539M06, WORKING DRAFT SEQUENCE, 10 unordered pieces. 3.2e-62:429:86
AC004832
R-THYRO1001113
Caenorhabditis elegans cosmid C25F9, complete sequence.
0.026:338:58
Z81476
R-THYRO1001128
Homo sapiens chromosome 9q34, clone 63G10, complete sequence.
5.3e-12:132:79
AC002096
R-THYRO1001205
Homo sapiens clone DJ1173120, WORKING DRAFT SEQUENCE, 5 unordered pieces. 1.9e-60:251:85
AC004987
R-THYRO1001237
R-THYRO1001242
Mouse mRNA for thymic epithelial cell surface antigen, complete cds.
1.5e-45:525:75
D67067
R-THYRO1001266
H.sapiens DNA containing a polymorphic (CA)n repeat (436bp).
6.0e-05:258:67
X65457
R-THYRO1001327
Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
2.8e-104:541:95
AL021578
R-THYRO1001456
R-THYRO1001457
H.sapiens mRNA for protein kinase C mu.
2.9e-23:391:66
X75756
R-THYRO1001471
Homo sapiens Chromosome 16 BAC clone CIT987SK-A-952F10, complete sequence. 0.39:271:61
AC004787
R-THYRO1001478
R-THYRO1001495
Homo sapiens clone DJ0813F11, WORKING DRAFT SEQUENCE, 5 unordered pieces. 2.8e-88:446:88
AC006006
R-THYRO1001523
CIT-HSP-2333F9. TF CIT-HSP Homo sapiens genomic clone 2333F9, genomic survey sequence.
1.4e-05:126:71
AQ039390
R-THYRO1001529
R-THYRO1001593
Homo sapiens chromosome 19, cosmid R33632, complete sequence.
3.7e-100:514:96
AC005781
R-THYRO1001608
Homo sapiens clone DJ0635005, WORKING DRAFT SEQUENCE, 7 unordered pieces. 2.3e-40:369:79
AC004845
R-THYRO1001641
Homo sapiens clone 24448 unknown mRNA, partial cds.
3.4e-110:562:96
AF070638
R-THYRO1001700
R-THYRO1001702
Mus musculus mRNA for myeloid associated differentiation protein.
1.1e-11:367:66
AJ001616
R-THYRO1001725
Homo sapiens, clone hRPK.1_A_1, complete sequence.
9.1e-12:329:65
AC006196
R-THYRO1001770
Homo sapiens PAC clone DJ0755G17 from 7p21-p22, complete sequence. 0.12:339:59
AC004879
R-THYRO1001803
R-Y79AA1000030
Homo sapiens chromosome 5, BAC clone 319C17 (LBNL H159), complete sequence. 2.0e-98:515:95
AC005214
R-Y79AA1000127
Homo sapiens genomic DNA, chromosome 21q11.1, segment 5/28, WORKING DRAFT
SEQUENCE.
3.2e-115:551:99
AP000034
R-Y79AA1000207
Homo sapiens chromosome 17, clone hRPK.271_K_11, complete sequence.
1.8e-38:282:85
AC005562
R-Y79AA1000226
Homo sapiens full length insert cDNA YN52F10.
4.8e-09:104:85
AF075033
R-Y79AA1000270
Human mRNA for ORF, Xq terminal portion.
1.0e-105:564:93
D16469
R-Y79AA1000426
Rattus norvegicus activin beta E mRNA, complete cds.
6.1e-50:562:72
AF089825
R-Y79AA1000521
Rattus norvegicus steroid sulfatase (Sts) mRNA, complete cds.
0.48:233:62
U37138
R-Y79AA1000750
Human DNA from cosmid f23280 from chromosome 19q13.2, genomic sequence. 6.8e-07:320:60
L47334
R-Y79AA1000776
R-Y79AA1000777
Homo sapiens full length insert cDNA clone ZD93D10.
2.9e-110:574:95
AF088072
R-Y79AA1000876
Mus musculus bone morphogenetic protein-6 (BMP-6) gene, exons 6 and 7 and complete cds.
0.0096:105:71
U73520
R-Y79AA1000959
Homo sapiens Hsp70 binding protein HspBP1 mRNA, complete cds.
1.0e-78:453:92
AF093420
R-Y79AA1000967
Rattus norvegicus vesicla-associate calmodulin-binding protein mRNA, complete cds.
2.3e-43:263:84
L22557
R-Y79AA1001013
R-Y79AA1001056
Mus musculus maternal transcript Maid (Maid) mRNA, complete cds.
1.5e-22:269:73
U50734
R-Y79AA1001062
D.teissieri mitochondrial DNA for tRNA-fmet, tRNA-Ile, tRNA-Gln & tRNA-Val. 1.1e-07:494:57
X54011
R-Y79AA1001090
Homo sapiens Chromosome 16 BAC clone CIT987SK-A-279B10, complete sequence. 1.2e-26:269:77
AC002300
R-Y79AA1001212
Homo sapiens SL15 protein mRNA, complete cds.
5.7e-82:407:97
AF038961
R-Y79AA1001264
HS_2195_A2_A07_MR CIT Approved Human Genomic Sperm Library D Homo sapiens genomic clone Plate=2195 Col=14 Row=A, genomic survey sequence.
3.4e-07:101:82
AQ191092
R-Y79AA1001272
Hansenula wingei mitochondrial DNA, complete sequence.
2.1e-05:435:60
D31785
R-Y79AA1001328
Rattus norvegicus Delta 3 mRNA, complete cds.
1.0e-29:356:72
AF084576
R-Y79AA1001426
R-Y79AA1001430
Homo sapiens mRNA for KIAA0469 protein, complete cds.
6.2e-111:555:96
AB007938
R-Y79AA1001523
Homo sapiens DNA sequence from PAC 418A9 on chromosome 6q21. Contains the first (5') two exons of a CDK8 (Cell Division Protein Kinase 8) LIKE gene, a Neutral Calponin LIKE pseudogene, ESTs and STSs, complete sequence.
3.7e-71:259:90
Z84480
R-Y79AA1001530
Human beta-tubulin gene (5-beta) with ten Alu family members.
2.6e-56:301:96
X00734
R-Y79AA1001592
HS_2175_A2_B11_T7 CIT Approved Human Genomic Sperm Library D Homo sapiens genomic clone Plate=2175 Col=22 Row=C, genomic survey sequence.
1.0:237:59
AQ307634
R-Y79AA1001727
R-Y79AA1001787
Homo sapiens mRNA for putative ATPase, partial.
7.2e-80:405:97
AJ009947
R-Y79AA1001795
Human DNA sequence from clone 1033B10 on chromosome 6p21.2-21.31. Contains the BING5 gene, exons 11 to 15 of the BING4 gene, the gene for GalT3 (beta3-Galactosyltransferase), the RPS18 (40S ribosomal protein S18) gene, the SACM2L (suppressor of actin mutation 2, yeast, homolog) gene, a pseudogene similar to TAT-SF1, a Pseudogene similar to zinc finger genes, the RING1 gene, the gene for HKE6 (RING2), the gene for HKE4 (RING5), the RXRB (Retinoid X receptor beta) gene, the COL11A2 (collagen, type XI, alpha 2) gene, the HLA-DPB2 pseudogene and part of the HLA-DPA3 pseudogene. Contains predicted CpG islands, ESTs, STSs, and GSSs, complete sequence.
4.2e-110:555:97
AL031228
R-Y79AA1001799
R-Y79AA1001803
Rattus norvegicus secretogranin III (SgIII) mRNA, complete cds.
6.2e-60:499:77
U02983
R-Y79AA1001863
Human DNA sequence from PAC 365E2 on chromosome 6p22.3-24.1. Contains EST and
STS.
1.4e-45:261:75
AL009177
R-Y79AA1002022
H.sapiens mRNA for basement membrane heparan sulfate proteoglycan.
1.0:311:61
X62515
R-nnnnnnnnnnnn
Plasmodium falciparum chromosome 2, section 18 of 73 of the complete sequence.
1. 0:208:62
AE001381
R-nnnnnnnnnnnn
Homo sapiens DNA, trinucleotide repeats region, clone CAG83.
0.17:132:67
AB018494
R-Y79AA1002213
Human DNA sequence from PAC 340G1 on chromosome 6 contains STS.
5.6e-46:490:73
Z84719
R-Y79AA1002334
Japanese Quail (C.coturnix) troponin T isoform mRNA, clone cC501.
0.96:210:63
M26599
R-Y79AA1002373
Human BAC clone RG126M09 from 7q21-q22, complete sequence.
9.7e-82:544:85
AC002067
R-Y79AA1002376
Human mitochondrial DNA, fragment M1, encoding transfer RNAs, cytochrome oxidase I, and 2 URFs.
1.9e-111:546:97
M10546
R-Y79AA1002378
Mus musculus mRNA for zinc finger protein, complete cds, clone:CTfin51. 1.5e-33:244:74
D10630
R-Y79AA1002381
Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence. 0.0046:177:68
Z95152

### Homology search result 8.

The result of the homology search in the Human Unigene(http://www.ncbi.nlm.nih.gov/UniGene) using the clone sequences of the 5'-ends.

Indicated are from the top,
the name of the clone sequence,
title of the top hit data,
the P-value: the length of the sequence used for comparison (nucleotide):similarity (%), the Accession No. of the top hit data.

Data were not shown for the clones in which the P-value was higher than 1.
F-BNGH41000020
ESTs
6.6e-72:412:92
Hs.153375:AI287812
F-BNGH41000087
Homo sapiens mRNA for MIFR-1, complete cds
0.027:499:57
Hs.58269:AB010962
F-BNGH41000091
Homo sapiens voltage-gated potassium channel eag (EAG) mRNA, complete cds 5.2e-81:687:76
Hs.158305:AJ001366
F-HEMBA1000006
ESTs, Weakly similar to HYPOTHETICAL 51.2 KD PROTEIN IN LAG1-RPL14B INTERGENIC REGION [S.cerevisiae]
2.0e-25:167:91
Hs.9252:R53360
F-HEMBA1000121
ESTs, Moderately similar to HYPOTHETICAL 68.7 KD PROTEIN ZK757.1 IN CHROMOSOME III [Caenorhabditis elegans]
3.0e-34:180:98
Hs.149509:N24022
F-HEMBA1000128
EST
0.00069:177:62
Hs.158854:AI377837
F-HEMBA1000275
Human modulator recognition factor I (MRF-1) mRNA, 3' end
0. 012:508:58
Hs.920:M62324
F-HEMBA1000300
Human mRNA for KIAA0355 gene, complete cds
1.6e-46:402:78
Hs.153014:AB002353
F-HEMBA1000349
EST
6.7e-08:65:95
Hs.54372:N80032
F-HEMBA1000443
ESTs
6.1e-23:278:76
Hs.69492:AA116026
F-HEMBA1000462
F-HEMBA1000477
ESTs
6.9e-78:414:94
Hs.152861:AA287444
F-HEMBA1000590
Homo sapiens mRNA for matrilin-4, partial
7.3e-95:482:96
Hs.129361:AJ007581
F-HEMBA1000634
ESTs
1.3e-38:246:86
Hs.6145:W26640
F-HEMBA1000671
Zinc finger protein 140 (clone pHZ-39)
2.4e-53:469:68
Hs.154205:U09368
F-HEMBA1000713
Homo sapiens 10kD protein (BC10) mRNA, complete cds
2.1e-127:442:97
Hs.5300:AF053470
F-HEMBA1000732
Homo sapiens latent transforming growth factor-beta binding protein 4S mRNA, complete cds
1.0e-45:258:94
Hs.85087:AF051344
F-HEMBA1000745
Human cardiotrophin-1 (CTF1) mRNA, complete cds
1.1e-07:316:61
Hs.25537:U43030
F-HEMBA1000835
ESTs
4.2e-11:188:72
Hs.116265:AI184988
F-HEMBA1000875
Zinc finger protein 133 (clone pHZ-13)
1.5e-27:169:93
Hs.78434:U09366
F-HEMBA1000907
Homo sapiens transcription factor forkhead-like 7 (FKHL7) gene, complete cds 1.3e-06:545:60
Hs.143551:AF048693
F-HEMBA1000940
Homo sapiens connexin46.6 (Cx46.6) gene, complete cds
4.1e-18:307:66
Hs.100072:AF014643
F-HEMBA1000962
Homo sapiens mRNA for MEGF8, partial cds
0.0018:391:62
Hs.158200:AB011541
F-HEMBA1001184
Homo sapiens SH3 domain binding glutamic acid-rich-like protein (SH3BGRL) mRNA, complete cds
2.7e-24:404:67
Hs.14368:AF042081
F-HEMBA1001221
Human transmembrane protein mRNA, complete cds
7.7e-44:858:63
Hs.78531:U19878
F-HEMBA1001228
Human germline oligomeric matrix protein (COMP) mRNA, complete cds
2.2e-85:463:93
Hs.1584:AC003107
F-HEMBA1001272
Antidiuretic hormone receptor
0. 064:616:57
Hs.2524:L22206
F-HEMBA1001296
Homo sapiens delta-catenin mRNA, complete cds
0.031:410:59
Hs.80220:U96136
F-HEMBA1001297
Homo sapiens putative transcription factor CA150 mRNA, complete cds
3.0e-15:143:81
Hs.13063:AF017789
F-HEMBA1001390
ESTs, Highly similar to polymerase I-transcript release factor [M.musculus] 1.6e-49:297:91
Hs.25581:AI246284
F-HEMBA1001563
ESTs
4. 9e-12:160:74
   Hs.162813:AA524616
F-HEMBA1001621
Human P2U nucleotide receptor mRNA, complete cds
0.00098:314:61
Hs.339:U07225
F-HEMBA1001878
Homo sapiens U5 snRNP-specific 40 kDa protein mRNA, complete cds
3.4e-172:810:98
Hs.10290:AF090988
F-HEMBA1001886
Human repressor transcriptional factor (ZNF85) mRNA, complete cds
1.1e-115:849:80
Hs.37138:U35376
F-HEMBA1002048
Homo sapiens mRNA for APC 2 protein, complete cds
0.96:266:62
Hs.20912:AB012162
F-HEMBA1002131
Homo sapiens mRNA for KIAA0584 protein, partial cds
1.1e-45:709:66
Hs.106794:AB011156
F-HEMBA1002163
ASPARTYL-TRNA SYNTHETASE
0.026:568:58
Hs.80758:J05032
F-HEMBA1002164
Pregnancy-associated plasma protein A
0.0049:274:60
Hs.158229:U28727
F-HEMBA1002167
F-HEMBA1002178
Homo sapiens mRNA for KIAA0584 protein, partial cds
8.3e-48:794:65
Hs.106794:AB011156
F-HEMBA1002195
EST
2.0e-05:177:70
Hs.145935:AI275921
F-HEMBA1002227
Myristoylated alanine-rich C-kinase substrate
1.2e-138:382:95
Hs.75607:D10522
F-HEMBA1002239
Homo sapiens mRNA, chromosome 1 specific transcript KIAA0488
1.2e-47:570:71
Hs. 67619:AB007957
F-HEMBA1002316
EST
1.8e-28:246:79
Hs.136950:AA825638
F-HEMBA1002420
Homo sapiens GABA-B receptor mRNA, complete cds
1.7e-05:303:63
Hs.12307:AF056085
F-HEMBA1002421
Syndecan 2 (heparan sulfate proteoglycan 1, cell surface-associated, fibroglycan)
4.3e-167:778:98
Hs.1501:J04621
F-HEMBA1002524
Human MHC Class I region proline rich protein mRNA, complete cds
8.5e-128:751:89
Hs. 41548:U63336
F-HEMBA1002551
ESTs
2.4e-25:207:84
Hs.158172:N24325
F-HEMBA1002767
Homo sapiens chromosome 1p33-p34 beta-1,4-galactosyltransferase mRNA, complete cds
4.4e-170:798:98
Hs.19154:AF038660
F-HEMBA1002985
ESTs
2.6e-09:124:76
Hs.118620:T60326
F-HEMBA1002992
ESTs
2.4e-21:121:97
Hs.143571:AI089396
F-HEMBA1003047
Homo sapiens intrinsic factor-B12 receptor precursor, mRNA, complete cds 1.5e-188:873:99
Hs.148318:AF034611
F-HEMBA1003072
ESTs
1.2e-33:387:71
Hs.59628:W91959
F-HEMBA1003101
Homo sapiens tyrosylprotein sulfotransferase-2 mRNA, complete cds 1.7e-140:671:98
Hs.26350:AF049891
F-HEMBA1003120
Zinc finger protein 91 (HPF7, HTF10)
1.0e-24:143:76
Hs.8597:L11672
F-HEMBA1003230
Homo sapiens UP50 mRNA, complete cds
1.8e-184:856:98
Hs.11494:AF093118
F-HEMBA1003294
Human antisecretory factor-1 mRNA, complete cds
5.1e-45:324:83
Hs.148495:AF050199
F-HEMBA1003315
Homo sapiens mRNA for TIP49, complete cds
4.2e-19:377:64
Hs.155541:AF070735
F-HEMBA1003392
Homo sapiens LDL receptor-related protein 6 (LRP6) mRNA, complete cds
9.2e-185:851:99
Hs.23672:AF074264
F-HEMBA1003399
H.sapiens BDP1 mRNA for protein-tyrosine-phosphatase
0.00042:297:61
Hs.118929:X79568
F-HEMBA1003487
Homo sapiens receptor for viral semaphorin protein (VESPR) mRNA, complete cds 0.0011:237:63
Hs.88145:AF030339
F-HEMBA1003497
ESTs, Weakly similar to similar to zinc finger 5 protein from Gallus gallus,
U51640 [H. sapiens]
2.5e-09:303:63
Hs.143723:H86048
F-HEMBA1003530
Homo sapiens mRNA for ephrin-A2
0.024:396:60
Hs.158306:AJ007292
F-HEMBA1003602
Homo sapiens DNA from chromosome 19, cosmid R29144
0.0072:663:57
Hs.155647:AC004221
F-HEMBA1003732
ESTs
1.0e-106:494:100
Hs.157568:AI356515
F-HEMBA1003945
Homo sapiens clone 638 unknown mRNA, complete sequence
5.9e-78:310:93
Hs.159515:AF091085
F-HEMBA1004007
PROTO-ONCOGENE TYROSINE-PROTEIN KINASE SRC
0.56:165:64
Hs.115742:AF077754
F-HEMBA1004067
Fatty acid synthase {3' region} [human, breast and HepG2 cells, mRNA Partial, 2237 nt]
0.048:581:58
Hs.83190:U29344
F-HEMBA1004085
ESTs
1. 7e-15:92:98
Hs.98138:AI183561
F-HEMBA1004110
Homo sapiens intersectin short form mRNA, complete cds
1. 2e-159 : 779: 96
Hs.66392:AF064244
F-HEMBA1004250
Human mRNA for KIAA0327 protein, complete cds
2.1e-23:676:59
Hs.149323:AB002325
F-HEMBA1004391
NEURAL CELL ADHESION MOLECULE L1 PRECURSOR
0.43:157:63
Hs.1757:U52112
F-HEMBA1004444
H.sapiens mRNA for gp25L2 protein
1.5e-54:544:73
Hs.159569:X90872
F-HEMBA1004454
Homo sapiens tetraspan NET-4 mRNA, complete cds
1.1e-05:230:62
Hs.20709:AF065389
F-HEMBA1004505
ESTs
9.1e-61:345:93
Hs.4814:AA631254
F-HEMBA1004785
Homo sapiens Polycomb 2 homolog (hPc2) mRNA, complete cds 3.7e-18:294:65
Hs.123085:AF013956
F-HEMBA1004797
ESTs
3.9e-06:107:73
Hs.42302:AI032142
F-HEMBA1004952
Human cardiotrophin-1 (CTF1) mRNA, complete cds 0.00021:175:68
Hs.25537:U43030
F-HEMBA1004971
F-HEMBA1004982
Human metabotropic glutamate receptor 8 mRNA, complete cds 0.31:288:60
Hs.86204:U92459
F-HEMBA1005070
Human mRNA for KIAA0310 gene, complete cds
7.9e-67:370:93
Hs.5716:AB002308
F-HEMBA1005084
Homo sapiens mRNA for KIAA0612 protein, partial cds 0.00022:400:59
Hs.112499:AB014512
F-HEMBA1005145
Lymphocyte-activation gene 3
3.4e-05:480:59
Hs.74011:X51985
F-HEMBA1005230
ESTs
2.3e-103:481:99
Hs.135112:AI090827
F-HEMBA1005246
Homo sapiens ALR mRNA, complete cds
2.0e-05:220:62
Hs.153638:AF010403
F-HEMBA1005267
ESTs
5.6e-16:305:64
Hs.125699:AA868017
F-HEMBA1005337
EST
2.1e-59:304:97
Hs.48956:N64339
F-HEMBA1005430
ESTs
6.9e-19:333:65
Hs.116567:AI332643
F-HEMBA1005449
Human plectin (PLEC1) mRNA, complete cds 0.026:576:56
Hs.79706:U53204
F-HEMBA1005489
Homo sapiens mRNA for KIAA0291 gene, partial cds 0.14:551:59
Hs.104717:AB006629
F-HEMBA1005522
COAGULATION FACTOR VII PRECURSOR
1.8e-12:298:64
Hs.36989:M13232
F-HEMBA1005545
MUSCARINIC ACETYLCHOLINE RECEPTOR M3
2.6e-143:672:98
Hs.7138:U29589
F-HEMBA1005698
ESTs
1.8e-124:611:97
Hs.144441:AI338335
F-HEMBA1005913
F-HEMBA1005929
H.sapiens mRNA for serine/threonine protein kinase EMK
1.5e-86:847:72
Hs.157199:X97630
F-HEMBA1005945
ESTs, Weakly similar to F17E5.2 [C.elegans]
4.2e-26:159:92
Hs.126571:AI038963
F-HEMBA1006016
ESTs
1.3e-22:145:93
Hs.33728:H97503
F-HEMBA1006171
F-HEMBA1006276
Homo sapiens KIAA0412 mRNA, partial cds
5.1e-19:371:65
Hs.6200:AB007872
F-HEMBA1006299
F-HEMBA1006311
F-HEMBA1006335
ESTs
0.00021:327:62
Hs.146044:AI089998
F-HEMBA1006357
Homo sapiens secretory carrier membrane protein (SCAMP2) mRNA, complete cds 7.4e-28:389:67
Hs.10761:AF005038
F-HEMBA1006430
ESTs
9.7e-92:463:95
Hs.143702:AI084062
F-HEMBA1006482
Homo sapiens h-sco1 (SC01) mRNA, nuclear gene encoding mitochondrial protein, complete cds
6.2e-146:575:98
Hs.14511:AF026852
F-HEMBA1006517
ESTs
3.6e-63:381:87
Hs.11611:W21919
F-HEMBA1006544
Homo sapiens suppressor of white apricot homolog 2 (SWAP2) mRNA, complete cds 2.0e-50:503:76
Hs.43543:AF042800
F-HEMBA1006572
Calcium channel, voltage-dependent, P/Q type, alpha 1A subunit
0.031:611:57
Hs.96253:U79666
F-HEMBA1006658
Homo sapiens mRNA for KIAA0687 protein, partial cds
1.2e-128:646:95
Hs.3628:AB014587
F-HEMBA1006707
Homo sapiens mRNA for matrilin-4, partial
1.7e-101:476:98
Hs.129361:AJ007581
F-HEMBA1006724
ESTs
8.3e-86:450:95
Hs.10056:AA210796
F-HEMBA1006749
Homo sapiens mRNA for matrilin-4, partial
6.1e-97:457:98
Hs. 129361:AJ007581
F-HEMBA1006770
ESTs, Highly similar to BRAIN PROTEIN F41 [Mus musculus]
1.6e-31:237:85
Hs.31612:H41366
F-HEMBA1006902
Homo sapiens mRNA for matrilin-4, partial
9.4e-113:541:97
Hs.129361:AJ007581
F-HEMBA1006912
ESTs
1.4e-94:460:97
Hs.88672:AA279956
F-HEMBA1006916
Homo sapiens Grb14 mRNA, complete cds
5.2e-120:651:92
Hs.83070:L76687
F-HEMBA1006960
Human DNA binding protein FKHL15 (FKHL15) mRNA, complete cds
0.011:628:57
Hs.159234:U89995
F-HEMBA1007013
ESTs
2.6e-05:139:69
Hs.113817:AA702497
F-HEMBA1007057
Homo sapiens hLRp105 mRNA for LDL receptor related protein 105, complete cds 7.5e-12:389:64
Hs.143641:AB009462
F-HEMBA1007063
F-HEMBA1007226
ESTs
1.8e-35:202:94
Hs.105140:N32669
F-HEMBA1007241
ESTs, Weakly similar to No definition line found [C.elegans]
4. 1e-27:361:67
   Hs.114062:AI421699
F-HEMBA1007291
ESTs
0.96:114:69
Hs.121411:AA770241
F-HEMBA1007332
ESTs, Weakly similar to hTAF11100 [H. sapiens]
2.5e-81:405:97
Hs.3727:AA205887
F-HEMBB1000106
ESTs
2.2e-76:393:96
Hs.151874:AI023405
F-HEMBB1000276
EST
0.81:239:63
Hs.149811:AI286277
F-HEMBB1000309
Homo sapiens zinc finger protein (MBLL) mRNA, complete cds 2.4e-35:180:100
Hs.44806:AF061261
F-HEMBB1000407
Cyclin-dependent kinase inhibitor 1C (p57, Kip2) 0.026:218:65
Hs.106070:U22398
F-HEMBB1000447
Homo sapiens JWA protein mRNA, complete cds
4.6e-160:750:98
Hs.92384:AF070523
F-HEMBB1000542
ESTs, Weakly similar to C01H6.7 [C.elegans]
6.8e-07:130:77
Hs.18171:AA524327
F-HEMBB1000567
ESTs
8.8e-13:271:71
Hs.19934:AA455673
F-HEMBB1000642
F-HEMBB1000668
EST
0.83:192:58
Hs.126372:AA912193
F-HEMBB1000679
H.sapiens mRNA for TRAMP protein
4.1e-96:727:80
Hs.4147:X63679
F-HEMBB1000881
Homo sapiens chromosome 4p homeobox mRNA sequence 2.2e-06:512:60
Hs.104134:M99587
F-HEMBB1000905
Homo sapiens mRNA for voltage gated potassium channel 0.93:337:58
Hs.4975:Y15065
F-HEMBB1001026
Human p76 mRNA, complete cds
6.1e-08:410:61
Hs.28757:U81006
F-HEMBB1001048
Human Hpast (HPAST) mRNA, complete cds
2.1e-56:524:75
Hs.155119:AF001434
F-HEMBB1001200
EST
0. 10:300:61
Hs.161647:AA133367
F-HEMBB1001407
Homo sapiens PRKY exon 1 and joined CDS
2.6e-40:271:81
Hs.56336:Y15801
F-HEMBB1001530
ESTs
1.2e-98:477:98
Hs.135208:AI093908
F-HEMBB1001547
F-HEMBB1001573
EST
2.2e-06:115:75
Hs.138275:R43976
F-HEMBB1001847
ESTs
5.3e-79:389:98
Hs.16141:W56079
F-HEMBB1001959
Homo sapiens clone 24781 mRNA sequence
1.0e-58:322:93
Hs.108112:AF070640
F-HEMBB1001978
EST
4.7e-23:245:74
Hs.136356:AA493225
F-HEMBB1002039
EST
2.3e-25:345:70
Hs.128248:AA972858
F-HEMBB1002041
Human plectin (PLEC1) mRNA, complete cds
2.2e-08:477:60
Hs.79706:U53204
F-HEMBB1002051
Homo sapiens E74-like factor 5 (ELF5) mRNA, complete cds 9.9e-97:454:99
Hs.159267:AF049703
F-HEMBB1002120
ESTs
7. 6e-10:68:100
Hs.146335:AI262660
F-HEMBB1002162
Homo sapiens genethonin 1 mRNA, complete cds
2.2e-68:328:99
Hs.109590:AF062534
F-HEMBB1002228
Homo sapiens unknown mRNA, complete cds
5.3e-41:208:98
Hs.11441:AF047439
F-HEMBB1002245
Human growth/differentiation factor 1 (GDF-1) mRNA, complete cds
5.6e-05:299:63
Hs.92614:M62302
F-HEMBB1002302
F-HEMBB1002427
Homo sapiens GN6ST mRNA for long form of N-acetylglucosamine-6-0-sulfotransferase (GIcNAc6ST), complete cds
0.84:108:68
Hs.8786:AB014680
F-HEMBB1002465
ESTs, Highly similar to ACYL-COA DEHYDROGENASE [Bacillus subtilis]
3. 2e-18:159:84
Hs.14791:AA741056
F-HEMBB1002661
ESTs
0.023:424:55
Hs.154029:AI380603
F-HEMBB1002663
F-HEMBB1002693
F-MAMMA1000046
Human mRNA for tryptophan hydroxylase (EC 1.14.16.4)
3.2e-43:454:74
Hs.144563:AF057280
F-MAMMA1000102
Homo sapiens mRNA for cathepsin V, complete cds
0.70:222:65
Hs.87417:AB001928
F-MAMMA1000106
Homo sapiens mRNA for KIAA0754 protein, partial cds 0.00076:331:61
Hs.159183:AB018297
F-MAMMA1000118
B94 PROTEIN
1.5e-07:511:61
Hs.75522:M92357
F-MAMMA1000141
ESTs
2.3e-18:268:73
Hs.155334:AA827904
F-MAMMA1000204
Homo sapiens dysferlin mRNA, complete cds
2.5e-167:781:98
Hs.143897:AF075575
F-MAMMA1000226
Human involucrin mRNA
0. 0010:414:61
Hs.157091:M13903
F-MAMMA1000403
ESTs
2.0e-24:163:90
Hs.44281:AI342377
F-MAMMA1000449
ESTs
0.99:211:60
Hs.143715:AI167929
F-MAMMA1000457
NADH-CYTOCHROME B5 REDUCTASE
7.7e-37:551:66
Hs.75666:M28713
F-MAMMA1000473
F-MAMMA1000496
Homo sapiens PAC clone DJ130H16 from 22q12.1-qter 1.1e-107:543:96
Hs.8003:AC004997
F-MAMMA1000528
EST
0.22:227:59
Hs.161400:AI423879
F-MAMMA1000591
H.sapiens mRNA for UDP-GaINAc:polypeptide N-acetylgalactosaminyl transferase 3.3e-23:470:62
Hs.55823:X92689
F-MAMMA1000614
H.sapiens mRNA for CCAAT/enhancer binding protein alpha
1.9e-06:492:61
Hs.76171:Y11525
F-MAMMA1000652
Homo sapiens mRNA, chromosome 1 specific transcript KIAA0487
1.5e-61:449:75
Hs.92381:AB007956
F-MAMMA1000681
Homo sapiens mRNA for putative G-protein coupled receptor, EDG6
4.0e-34:636:65
Hs.159543:AJ000479
F-MAMMA1000706
COAGULATION FACTOR VII PRECURSOR
9.7e-16:378:65
Hs.36989:M13232
F-MAMMA1000788
ESTs, Weakly similar to M01E11.2 [C.elegans]
3.4e-118:571:97
Hs.78389:AI191127
F-MAMMA1000810
EST
0.065:211:61
Hs.116798:AA633813
F-MAMMA1000814
EST
3.1e-08:224:66
Hs.141620:N63316
F-MAMMA1000881
Homo sapiens sgk gene
3.5e-08:165:69
Hs.159640:AJ000512
F-MAMMA1000986
Homo sapiens clone 24796 mRNA sequence
2.3e-115:320:99
Hs.27191:AF070596
F-MAMMA1000994
Human HOX4C mRNA for a homeobox protein
0.050:178:64
Hs.74061:X59372
F-MAMMA1001043
Latent transforming growth factor beta binding protein 2
0.0013:376:60
Hs.83337:Z37976
F-MAMMA1001066
ESTs
1.1e-18:128:77
Hs.114031:AA700958
F-MAMMA1001094
Homo sapiens clone 243 unknown mRNA, complete sequence
2.0e-182:844:99
Hs.20423:AF091094
F-MAMMA1001141
Homo sapiens achaete scute homologous protein (ASH1) mRNA, complete cds 6.1e-07:492:58
Hs.1619:L08424
F-MAMMA1001150
Protein kinase C, mu
8.3e-51:691:67
Hs.2891:X75756
F-MAMMA1001237
Homo sapiens monocarboxylate transporter (MCT3) mRNA, complete cds 8.2e-08:386:60
Hs.85838:U81800
F-MAMMA1001284
ESTs
1.1e-91:452:97
Hs.114756:AI279440
F-MAMMA1001310
Homo sapiens mRNA for KIAA0708 protein, partial cds
0.014:512:57
Hs.117177:AB014608
F-MAMMA1001344
ESTs, Weakly similar to No definition line found [C.elegans]
8.3e-80:406:96
Hs.121619:AI188389
F-MAMMA1001418
Human Na+/nucleoside cotransporter (hCNT1a) mRNA, complete cds
1.9e-36:622:63
Hs.97207:U62966
F-MAMMA1001532
Human kruppel-related zinc finger protein (ZNF184) mRNA, partial cds
2.1e-33:282:68
Hs.158174:U66561
F-MAMMA1001609
Insulin-like growth factor-binding protein 4
0.00026:596:57
Hs.1516:U20982
F-MAMMA1001615
Homo sapiens DNA from chromosome 19, cosmid R29144
1.1e-05:504:59
Hs.155647:AC004221
F-MAMMA1001623
Excision repair protein ERCC6
1.2e-38:274:86
Hs.99924:L04791
F-MAMMA1001634
ESTs
1.5e-26:176:90
Hs.16187:AI139901
F-MAMMA1001893
Cyclin-dependent kinase inhibitor 1C (p57, Kip2)
0.00030:170:68
Hs.106070:U22398
F-MAMMA1001901
ESTs
1.5e-36:201:76
Hs.161660:AA167744
F-MAMMA1001957
Prostaglandin 12 (prostacyclin) receptor (IP)
0.041:277:61
Hs.393:D38128
F-MAMMA1001978
EST
4.0e-43:359:81
Hs.136494:AA587773
F-MAMMA1002070
Human PAC clone DJ515N1 from 22q11.2-q22
5.1e-135:652:97
Hs.26670:AC002073
F-MAMMA1002080
Calcium channel, voltage-dependent, L type, alpha 1C subunit
0. 0019:574:57
Hs.89925:L04569
F-MAMMA1002087
Human mRNA for KIAA0009 gene, complete cds
0.71:228:63
Hs.79972:013634
F-MAMMA1002091
Homo sapiens CD39L2 (CD39L2) mRNA, complete cds
5.2e-158:743:98
Hs.12330:AF039916
F-MAMMA1002095
Homo sapiens mRNA for KIAA0703 protein, complete cds
4.9e-55:657:68
Hs.6168:AB014603
F-MAMMA1002128
Human leucine zipper on the D14S46E locus mRNA, complete cds 0.77:449:59
Hs.89606:M95925
F-MAMMA1002142
F-MAMMA1002165
Homo sapiens connective tissue growth factor-like protein precursor, mRNA, complete cds
1.2e-35:182:98
Hs.139340:AF083500
F-MAMMA1002205
ESTs
4.7e-32:385:71
Hs.46158:AI160121
F-MAMMA1002224
TRANSCRIPTION INITIATION FACTOR IIE, ALPHA SUBUNIT
1.3e-34:248:85
Hs.3006:X63468
F-MAMMA1002234
ESTs
1. 1e-100:501:97
Hs.158161:AA312511
F-MAMMA1002586
Human mRNA for KIAA0183 gene, partial cds
0.00041:388:61
Hs.76666:080005
F-MAMMA1002633
Landsteiner-Wiener blood group glycoprotein
1.1e-37:477:71
Hs.108287:L27670
F-MAMMA1003126
Human Hpast (HPAST) mRNA, complete cds
4.1e-84:801:74
Hs.155119:AF001434
F-NT2RM1000407
ESTs
4.1e-19:132:92
Hs.133484:D80522
F-NT2RM1000462
F-NT2RM1000542
Beta-galactosidase (GLB1)
1.3e-17:436:61
Hs.79222:M34423
F-NT2RM1000580
ESTs, Weakly similar to similar to M. musculus MER5 and other AHPC/TSA proteins [C.elegans]
6.2e-51:254:98
Hs.132096:AA314601
F-NT2RM1000789
Homo sapiens mRNA for hTCF-4
3.5e-96:299:92
Hs.154485:Y11306
F-NT2RM1000855
Hydroxysteroid (11-beta) dehydrogenase 2
0.021:178:67
Hs.1376:U26726
F-NT2RM1000858
F-NT2RM1000899
Homo sapiens BAC clone RG119C02 from 7p15
0.037:222:63
Hs.22900:AC004520
F-NT2RM2000241
ESTs
2.9e-31:166:97
Hs.156175:AI334328
F-NT2RM2000306
F-NT2RM2000410
ESTs
3.2e-12:81:97
Hs.72116:AA151564
F-NT2RM2000423
Beta-galactosidase (GLB1)
0.074:163:63
Hs.79222:M34423
F-NT2RM2000497
ESTs, Weakly similar to CHL1 protein [H.sapiens]
3.7e-21:121:97
Hs.97515:AA435715
F-NT2RM2000514
F-NT2RM2000565
F-NT2RM2000582
EST
1.7e-42:218:98
Hs.160262:AI146610
F-NT2RM2000589
F-NT2RM2000622
Androgen receptor (dihydrotestosterone receptor; testicular feminization; spinal and bulbar muscular atrophy; Kennedy disease)
0.00018:409:62
Hs.99915:M23263
F-NT2RM2000632
Homo sapiens TBP-associated factor 172 (TAF-172) mRNA, complete cds 0.00017:331:59
Hs.14244:AF038362
F-NT2RM2000773
Human zinc finger protein (MAZ) mRNA
7.2e-47:274:91
Hs.7647:M94046
F-NT2RM2001126
Homo sapiens multi PDZ domain protein MUPP1 (MUPP1) mRNA, complete cds 5.1e-163:663:99
Hs.21301:AF093419
F-NT2RM2001558
Homo sapiens protein kinase A binding protein AKAP110 mRNA, complete cds 3.9e-166:770:98
Hs.98397:AF093408
F-NT2RM2001626
Human mRNA for KIAA0231 gene, partial cds
2.8e-40:562:67
Hs.7938:D86984
F-NT2RM2001643
ESTs
7.9e-112:548:97
Hs.12610:W56112
F-NT2RM2001738
FACTOR VIII INTRON 22 PROTEIN
0.32:452:59
Hs.83363:M34677
F-NT2RM2001767
Homo sapiens mRNA for B120, complete cds
5.0e-24:131:100
Hs.123090:AB001895
F-NT2RM2001792
Homo sapiens mRNA for serum lectin P35, complete cds
8.2e-14:244:67
Hs.54517:063160
F-NT2RM2001818
EST
0.051:152:61
Hs.157619:AI357718
F-NT2RM2001902
Human p21-activated protein kinase (Pak1) gene, complete cds 4.4e-39:568:66
Hs.62402:U24152
F-NT2RM2001939
Human G protein-coupled receptor GPR-NGA gene, complete cds 4.2e-141:664:98
Hs.92458:U55312
F-NT2RM2001941
Dopamine receptor D4
1. 3e-14:547:61
Hs.99922:L12398
F-NT2RM4000100
Human involucrin mRNA
1.1e-09:487:62
Hs.157091:M13903
F-NT2RM4000115
F-NT2RM4000198
ESTs
9.3e-101:496:98
Hs.128676:AA464413
F-NT2RM4000284
Human IgG Fc receptor hFcRn mRNA, complete cds
2.4e-38:194:98
Hs.110804:U12255
F-NT2RM4000295
Homo sapiens SOX22 protein (SOX22) mRNA, complete cds
1.7e-06:479:60
Hs.43627:U35612
F-NT2RM4000326
Phosphorylase kinase, gamma 2 (testis)
0.95:204:63
Hs.87452:M31606
F-NT2RM4000417
H.sapiens Syt V gene (genomic and cDNA sequence)
0.97:143:67
Hs.23179:X96783
F-NT2RM4000444
Eukaryotic translation elongation factor 1 delta (guanine nucleotide exchange prote i n)
0.45:194:64
Hs.90319:Z21507
F-NT2RM4000587
Human proto-oncogene (FRAT1) gene, complete cds
3.8e-05:495:60
Hs.143005:U58975
F-NT2RM4000593
F-NT2RM4000648
Homo sapiens glypican-4 (GPC4) mRNA, complete cds
1.0e-50:610:70
Hs.58367:AF030186
F-NT2RM4000761
EST
0.89:53:79
Hs.161967:AA494423
F-NT2RM4000965
H.sapiens mRNA for PHAPI2b protein
0.18:148:68
Hs.84264:U70439
F-NT2RM4000997
F-NT2RM4001321
ESTs
1.8e-94:467:97
Hs.12610:W56112
F-NT2RM4001325
Homo sapiens mRNA for chondroitin 6-sulfotransferase, complete cds
2.1e-13:384:64
Hs.158304:AB012192
F-NT2RM4001377
Homo sapiens mRNA for KIAA0638 protein, partial cds
3.1e-156:719:99
Hs.77864:AB014538
F-NT2RM4001735
F-NT2RM4001768
ESTs
0. 00012:123:68
Hs.128045:AA970231
F-NT2RM4001843
F-NT2RM4002352
Homo sapiens hLRp105 mRNA for LDL receptor related protein 105, complete cds 4.4e-157:761:97
Hs. 143641:AB009462
F-NT2RP1000002
EST
0.00023:170:68
Hs.135504:AI091717
F-NT2RP1000050
Histidine-rich calcium binding protein
0.0047:257:61
Hs.1480:M60052
F-NT2RP1000181
Homo sapiens chromosome 11, BAC CIT-HSP-311e8 (BC269730) containing the hFEN1 gene
6.9e-99:510:94
Hs.132898:AC004770
F-NT2RP1000239
ESTs
1.7e-34:240:67
Hs.33020:N31946
F-NT2RP1000261
ESTs, Weakly similar to HYPOTHETICAL 25.2 KD PROTEIN IN ACB1-KSS1 INTERGENIC REGION [S.cerevisiae]
9.1e-92:484:94
Hs.7870:AI078137
F-NT2RP1000271
Homo sapiens DNA-binding protein mRNA, complete cds
1.4e-140:678:97
Hs.137582:AF038951
F-NT2RP1000300
Human endosome-associated protein (EEA1) mRNA, complete cds
1.0:205:61
Hs.2864:L40157
F-NT2RP1000325
Phosphate carrier, mitochondrial
7.7e-84:444:93
Hs.78713:X60036
F-NT2RP1000448
ESTs
9.5e-73:405:93
Hs.24054:N46499
F-NT2RP1000465
ESTs
8.5e-10:81:87
Hs.18619:AI202769
F-NT2RP1000468
Homo sapiens clone 24781 mRNA sequence
2.1e-20:133:92
Hs.108112:AF070640
F-NT2RP1000551
Homo sapiens putative interferon-related protein (SM15) mRNA, partial cds 2.4e-140:742:93
Hs.75402:U09585
F-NT2RP1000579
SUCCINATE DEHYDROGENASE
1.1e-141:798:91
Hs.469:L21936
F-NT2RP1000613
Homo sapiens carbonic anhydrase precursor (CA 12) mRNA, complete cds 5.5e-11:468:58
Hs.5338:AF037335
F-NT2RP1000679
ESTs
0. 79:127:65
Hs.146093:AA100242
F-NT2RP1000740
Homo sapiens Trio isoform mRNA, complete cds
0.24:160:66
Hs.150625:AF091395
F-NT2RP1000903
F-NT2RP1000981
F-NT2RP1001004
Human mRNA for Doc2 beta, complete cds
0.00072:520:57
Hs.54402:070830
F-NT2RP1001020
ESTs, Weakly similar to SPORULATION-SPECIFIC PROTEIN 1 [Saccharomyces cerevisiae]
2.1e-73:392:94
Hs.4789:AI418298
F-NT2RP1001031
Miller-Dieker syndrome chromosome region
4.5e-07:383:61
Hs.77318:L13385
F-NT2RP1001563
Human eukaryotic translation initiation factor (eIF3) mRNA, complete cds 0.086:398:59
Hs. 57783:U78525
F-NT2RP2000092
Zinc finger protein 136 (clone pHZ-20)
5.5e-56:652:70
Hs.69740:U09367
F-NT2RP2000178
Homo sapiens chromosome 5, BAC clone 203o13 (LBNL H155), complete sequence 0.14:231:62
Hs.159402:AC005609
F-NT2RP2000240
Homo sapiens KIAA0415 mRNA, complete cds
3.0e-61:554:76
Hs.7289:AB007875
F-NT2RP2000394
ESTs
0.0063:210:63
Hs.134272:AI220363
F-NT2RP2000447
Human (clone SY11) golgin-95 mRNA, complete cds
3.8e-22:498:65
Hs.24049:L06147
F-NT2RP2000479
ESTs
1.3e-46:298:90
Hs.15641:W63676
F-NT2RP2000514
Homo sapiens roundabout 1 (robo1) mRNA, complete cds
1.2e-37:543:67
Hs.36702:AF040990
F-NT2RP2000533
ESTs, Highly similar to HYPOTHETICAL 16.3 KD PROTEIN IN DUR1,2-NGR1
INTERGENIC REGION [Saccharomyces cerevisiae]
5.4e-132:647:96
Hs.18120:AA913148
F-NT2RP2000610
Homo sapiens antigen NY-CO-16 mRNA, complete cds
0.00027:182:66
Hs.132206:AF039694
F-NT2RP2000616
ESTs
0.44:235:60
Hs.31714:AA514389
F-NT2RP2000649
Homo sapiens mRNA for Hs Ste24p, complete cds
6.2e-167:802:97
Hs.25846:AB016068
F-NT2RP2000663
Homo sapiens mRNA for KIAA0512 protein, complete cds
4.8e-15:305:64
Hs.48924:AB011084
F-NT2RP2000694
H.sapiens 5T4 gene for 5T4 Oncofetal antigen
1.0e-113:558:96
Hs.82128:AJ012159
F-NT2RP2000712
ESTs, Weakly similar to ZINC FINGER PROTEIN 91 [H.sapiens]
1.5e-83:442:93
Hs.154226:AA468767
F-NT2RP2000739
Human mRNA for KIAA0326 gene, partial cds
2.1e-25:574:62
Hs.6833:AB002324
F-NT2RP2000818
F-NT2RP2000903
H.sapiens 5T4 gene for 5T4 Oncofetal antigen
3.5e-112:539:97
Hs.82128:AJ012159
F-NT2RP2001200
Homo sapiens mRNA for KIAA0676 protein, partial cds
1. 1e-111:540:96
Hs.115763:AB014576
F-NT2RP2001223
ESTs
5.9e-91:461:95
Hs.103733:AA436929
F-NT2RP2001276
Homo sapiens mRNA for KIAA0634 protein, partial cds 2.4e-11:382:62
Hs.30898:AB014534
F-NT2RP2001388
F-NT2RP2001469
ESTs
7.3e-39:213:95
Hs.151001:AA564706
F-NT2RP2001480
Homo sapiens thrombospondin 3 (THBS3) gene, complete cds 2.9e-141:686:96
Hs.82165:L38969
F-NT2RP2001495
Human transporter protein (g17) mRNA, complete cds 6.0e-37:581:64
Hs.76460:U49082
F-NT2RP2001514
F-NT2RP2001529
Homo sapiens mRNA for ZIP-kinase, complete cds
1.5e-153:757:96
Hs.25619:AB007144
F-NT2RP2001538
ESTs, Highly similar to co-repressor protein [M.musculus] 4.4e-63:329:94
Hs.22583:AA188168
F-NT2RP2001562
Homo sapiens GLE1 (GLE1) mRNA, complete cds
7.5e-119:572:97
Hs.81449:AF058922
F-NT2RP2001662
H.sapiens mRNA for TGIF protein
2.6e-29:448:67
Hs.90077:X89750
F-NT2RP2001755
ESTs, Highly similar to F-SPONDIN PRECURSOR [Rattus norvegicus]
1.0e-47:275:92
Hs.153657:H37929
F-NT2RP2001769
Human protein kinase C-L (PRKCL) mRNA, complete cds
1.9e-09:399:59
Hs.89616:M55284
F-NT2RP2001817
EST
0.97:133:63
Hs.145274:AI249468
F-NT2RP2001878
Human DNA binding protein FKHL15 (FKHL15) mRNA, complete cds
3.6e-05:491:60
Hs.159234:U89995
F-NT2RP2001903
Human mRNA for apolipoprotein E receptor 2, complete cds
0.0023:270:60
Hs.54481:086407
F-NT2RP2001915
Homo sapiens Pig3 (PIG3) mRNA, complete cds
3.2e-05:493:60
Hs.50649:AF010309
F-NT2RP2001921
F-NT2RP2001948
ESTs
0.55:213:61
Hs.147805:AI221717
F-NT2RP2001956
ESTs, Weakly similar to HYPOTHETICAL 25.2 KD PROTEIN IN ACB1-KSS1 INTERGENIC REGION [S.cerevisiae]
8.1e-45:510:70
Hs.13144:T67556
F-NT2RP2002015
ESTs
4.3e-20:127:92
Hs.12610:W56112
F-NT2RP2002063
ESTs
1.0e-08:73:91
Hs.19814:T81721
F-NT2RP2002188
F-NT2RP2002232
EST
0. 82:99:67
Hs.148596:AI202232
F-NT2RP2002304
Human monocytic leukaemia zinc finger protein (MOZ) mRNA, complete cds 0.031:107:71
Hs.82210:U47742
F-NT2RP2002409
Homo sapiens lymphocyte secreted C-type lectin precursor, mRNA, complete cds 0.00063:302:65
Hs.105927:AF020044
F-NT2RP2002510
ABO blood group (transferase A, alpha 1-3-N-acetylgalactosaminyltransferase; transferase B, alpha 1-3-galactosyltransferase)
4.4e-09:298:64
Hs.144023:U15197
F-NT2RP2002527
Homo sapiens chromosome 11, BAC CIT-HSP-311e8 (BC269730) containing the hFEN1 gene
5.2e-65:327:96
Hs.132898:AC004770
F-NT2RP2002533
Homo sapiens putative tumor suppressor gene 26 protein alpha 2 delta calcium channel subunit mRNA, complete cds
2.1e-142:726:95
Hs.127436:AF040709
F-NT2RP2002564
Homo sapiens mRNA for repressor protein, partial cds
3.5e-55:594:74
Hs.58167:D30612
F-NT2RP2002674
Epoxide hydrolase 2, cytoplasmic
2.5e-07:332:62
Hs.113:L05779
F-NT2RP2002721
F-NT2RP2002824
ESTs, Weakly similar to ZK858.6 [C.elegans]
5.2e-28:190:90
Hs.120416:AA057428
F-NT2RP2002942
Homo sapiens mRNA for KIAA0806 protein, complete cds
2.0e-146:758:94
Hs.24279:AB018349
F-NT2RP2002974
ESTs
4.9e-51:475:77
Hs.137840:AI123378
F-NT2RP2002976
ESTs, Weakly similar to No definition line found [C.elegans]
7.8e-50:315:89
Hs.159604:AI380827
F-NT2RP2003042
Lecithin-cholesterol acyltransferase
2.4e-25:454:65
Hs.112125:M12625
F-NT2RP2003138
H.sapiens mRNA for TGIF protein
2.0e-05:121:75
Hs.90077:X89750
F-NT2RP2003179
Homo sapiens mRNA for KIAA0537 protein, complete cds
1.0e-43:587:70
Hs.12836:AB011109
F-NT2RP2003210
F-NT2RP2003302
Zinc finger protein 136 (clone pHZ-20)
1.8e-64:691:69
Hs.69740:U09367
F-NT2RP2003369
Homo sapiens chromosome 7q22 sequence
5.1e-109:539:96
Hs.125742:AF053356
F-NT2RP2003383
Homo sapiens mRNA for KIAA0458 protein, complete cds
1.6e-159:801:95
Hs.7414:AB007927
F-NT2RP2003390
Homo sapiens SEC63 (SEC63) mRNA, complete cds
2.2e-116:554:98
Hs.31575:AF100141
F-NT2RP2003469
ESTs
0. 26:127:69
Hs.62649:AA115328
F-NT2RP2003545
ESTs, Highly similar to SERINE/THREONINE-PROTEIN KINASE PAK [Rattus norvegicus]
4.2e-111:550:96
Hs.85768:W16504
F-NT2RP2003593
EST
8.7e-43:213:99
Hs.130657:AI005473
F-NT2RP2003599
ESTs
7.8e-14:84:98
Hs.107171:H53973
F-NT2RP2003655
F-NT2RP2003664
Homo sapiens mRNA for leptin receptor gene-related protein
5.4e-134:630:98
Hs.23581:Y12670
F-NT2RP2003931
Human mRNA for KIAA0365 gene, partial cds
4. 3e-14:101 :92
Hs.84123:AB002363
F-NT2RP2003940
Zinc finger protein 43 (HTF6)
4.6e-99:693:82
Hs.74107:X59244
F-NT2RP2003950
Cell division cycle 25A
0.00041:419:59
Hs.1634:M81933
F-NT2RP2004069
ESTs, Weakly similar to HYPOTHETICAL 26.4 KD PROTEIN EEED8.8 IN CHROMOSOME II
[C.elegans]
1.3e-75:390:94
Hs.13322:AA151730
F-NT2RP2004108
Zinc finger protein 136 (clone pHZ-20)
4.9e-69:548:78
Hs.69740:U09367
F-NT2RP2004141
TRICHOHYALIN
4. 8e-11 :435:63
Hs.82276:L09190
F-NT2RP2004179
ESTs
0.0054:180:66
Hs.134917:AI092952
F-NT2RP2004205
Homo sapiens nuclear dual-specificity phosphatase (SBF1) mRNA, partial cds 0.27:474:56
Hs. 112049:U93181
F-NT2RP2004447
Homo sapiens LDL receptor member LR3 mRNA, complete cds
0. 016:456:57
Hs.6347:AF077820
F-NT2RP2004495
Human transporter protein (g17) mRNA, complete cds
1.2e-26:497:61
Hs.76460:U49082
F-NT2RP2004524
Human bone morphogenetic protein-3b
0.0016:259:64
Hs. 2171:D49493
F-NT2RP2004556
ESTs
1.1e-34:181:97
Hs.27160:AA421991
F-NT2RP2004606
Tissue inhibitor of metalloproteinase 1 (erythroid potentiating activity, collagenase inhibitor)
5.7e-107:587:92
Hs.148726:X03124
F-NT2RP2004648
TUBULIN ALPHA-4 CHAIN
0.59:186:61
Hs.75318:X06956
F-NT2RP2004670
Human mRNA for KIAA0369 gene, complete cds
0.097:309:61
Hs.21355:AB002367
F-NT2RP2004794
ESTs
1.3e-60:310:96
Hs.84926:N50073
F-NT2RP2004837
F-NT2RP2004847
Zinc finger protein 42 (myeloid-specific retinoic acid-responsive)
1.4e-05:396:60
Hs.78247:M58297
F-NT2RP2005027
GLUCOSE TRANSPORTER TYPE 3, BRAIN
7.2e-147:713:96
Hs.7594:M20681
F-NT2RP2005069
Human mRNA for KIAA0355 gene, complete cds
0.14:303:61
Hs.153014:AB002353
F-NT2RP2005163
ESTs, Weakly similar to No definition line found [C.elegans]
1.4e-23:334:70
Hs.159604:AI380827
F-NT2RP2005181
Ecotropic retroviral receptor
8.3e-45:501:70
Hs.2928:X57303
F-NT2RP2005247
Oxysterol binding protein
4.2e-08:356:62
Hs.143065:M86917
F-NT2RP2005378
ESTs
1.7e-100:485:97
Hs.151572:AA588083
F-NT2RP2005391
EST
1.0:264:62
Hs.148259:AA905706
F-NT2RP2005425
Homo sapiens mRNA for KIAA0803 protein, partial cds
3.3e-118:566:97
Hs. 58103:AB018346
F-NT2RP2005463
F-NT2RP2005514
ESTs
3.6e-18:193:77
Hs.153344:R26293
F-NT2RP2005535
Homo sapiens DNA-binding protein mRNA, complete cds
7.5e-127:726:90
Hs.137582:AF038951
F-NT2RP2005541
Glucosamine (N-acetyl)-6-sulfatase (Sanfilippo disease IIID)
1.2e-06:225:64
Hs.2703:Z12173
F-NT2RP2005597
F-NT2RP2005632
ESTs
5.6e-67:344:96
Hs.112011:AA987961
F-NT2RP2005666
ESTs
5.8e-71:453:87
Hs.122698:AI042484
F-NT2RP2005774
Zinc finger protein 136 (clone pHZ-20)
1.3e-45:451:74
Hs.69740:U09367
F-NT2RP2005878
ESTs, Highly similar to ESTRADIOL 17 BETA-DEHYDROGENASE 3 [Homo sapiens]
5. 9e-10:67:100
Hs.104523:AA584520
F-NT2RP2005883
F-NT2RP2005887
F-NT2RP2005941
Human novel homeobox mRNA for a DNA binding protein
6.2e-11:464:61
Hs.37035:U07664
F-NT2RP2005994
F-NT2RP2006004
Homo sapiens KIAA0405 mRNA, complete cds
1.2e-13:273:63
Hs.48998:AB007865
F-NT2RP2006042
Human mRNA for KIAA0144 gene, complete cds
5.6e-12:220:69
Hs.8127:D63478
F-NT2RP2006092
Human FE65-like protein (hFE65L) mRNA, partial cds 2.6e-23:353:65
Hs.24957:U62325
F-NT2RP2006099
EST
2.5e-28:180:90
Hs.160878:AI361890
F-NT2RP2006134
Neogenin (chicken) homolog 1
0.035:219:60
Hs.90408:U61262
F-NT2RP2006269
Homo sapiens mRNA for matrilin-3
1.0:147:65
Hs.119534:AJ224741
F-NT2RP2006512
ESTs
1.6e-09:70:95
Hs.118981:AA282396
F-NT2RP3000011
F-NT2RP3000022
EST
0. 016:293:60
Hs.127706:AA961478
F-NT2RP3000059
Human SH3 domain-containing proline-rich kinase (sprk) mRNA, complete cds 0.0041:608:59
Hs.89449:L32976
F-NT2RP3000063
Excision repair protein ERCC6
1.0:264:59
Hs.99924:L04791
F-NT2RP3000125
Human mRNA for KIAA0314 gene, partial cds
6.9e-08:379:59
Hs.155045:AB002312
F-NT2RP3000148
Human Chromosome 16 BAC clone CIT987SK-A-635H12
4.5e-40:349:73
Hs.108604:AC002310
F-NT2RP3000169
Homo sapiens MRS1 mRNA, complete cds
1.1e-107:501:99
Hs.30985:AF093239
F-NT2RP3000171
Homo sapiens methionine synthase reductase (MTRR) mRNA, complete cds
1. 0:279:64
Hs.153792:AF025794
F-NT2RP3000172
Homo sapiens mRNA for ZIP-kinase, complete cds
7.4e-09:463:59
Hs.25619:AB007144
F-NT2RP3000201
Homo sapiens mRNA for KIAA0687 protein, partial cds
3.0e-171:792:98
Hs.3628:AB014587
F-NT2RP3000232
ESTs, Weakly similar to ZINC FINGER PROTEIN 91 [H.sapiens]
8.6e-24:304:70
Hs.112094:AA447558
F-NT2RP3000304
Homo sapiens LDL receptor-related protein 6 (LRP6) mRNA, complete cds 1.1e-172:797:98
Hs.23672:AF074264
F-NT2RP3000378
Homo sapiens mRNA for KIAA0700 protein, partial cds
4.3e-45:585:66
Hs.13999:AB014600
F-NT2RP3000427
Protein kinase, cAMP-dependent, catalytic, beta
1. 2e-15:97:98
Hs.87773:M34181
F-NT2RP3000436
Human protein disulfide isomerase-related protein P5 mRNA, partial cds 4.1e-06:353:59
Hs.85200:D49489
F-NT2RP3000444
Homo sapiens mRNA for KIAA0445 protein, complete cds
1.2e-08:542:60
Hs.154139:AB007914
F-NT2RP3000460
ESTs, Highly similar to PROTEIN TRANSPORT PROTEIN SEC61 ALPHA SUBUNIT [Can is familiaris]
1. 3e-17:181 :75
Hs.131840:AI016073
F-NT2RP3000481
Homo sapiens RanBP7/importin 7 mRNA, complete cds
5.4e-164:770:98
Hs.5151:AF098799
F-NT2RP3000616
Homo sapiens KIAA0405 mRNA, complete cds
1.5e-32:579:62
Hs.48998:AB007865
F-NT2RP3000645
Human KH type splicing regulatory protein KSRP mRNA, complete cds
4.6e-06:245:64
Hs.91142:U94832
F-NT2RP3000652
Homo sapiens DNA from chromosome 19, BAC 33152
2.6e-135:853:84
Hs.55452:AC003973
F-NT2RP3000676
Homo sapiens mRNA for KIAA0446 protein, complete cds
8.8e-88:420:98
Hs.158286:AB007915
F-NT2RP3000677
ESTs
3.9e-09:67:97
Hs.98819:AA778727
F-NT2RP3000721
ESTs, Weakly similar to No definition line found [C.elegans]
1.2e-57:395:86
Hs.159604:AI380827
F-NT2RP3000789
Homo sapiens putative RNA binding protein KOC (koc) mRNA, complete cds 4.8e-75:833:69
Hs.79440:U97188
F-NT2RP3000818
Homo sapiens chromosome 19, fosmid 39554
5. 9e-08:313:63
Hs.129906:AC004410
F-NT2RP3000820
ESTs, Moderately similar to WSB-1 [M.musculus]
8.8e-127:613:97
Hs.24630:AI365246
F-NT2RP3000838
Homo sapiens mRNA for KIAA0638 protein, partial cds
8.3e-79:682:79
Hs.77864:AB014538
F-NT2RP3000871
Homo sapiens retinoblastoma-interacting protein (RBBP8) mRNA, complete cds 1.9e-08:350:60
Hs.29287:U72066
F-NT2RP3000907
Human Ini1 mRNA, complete cds
0.91:345:59
Hs.155626:U04847
F-NT2RP3000921
Homo sapiens mRNA for KIAA0806 protein, complete cds
2.0e-65:798:68
Hs.24279:AB018349
F-NT2RP3001012
Homo sapiens mRNA for KIAA0667 protein, partial cds
1.3e-21:383:64
Hs.154740:AB014567
F-NT2RP3001044
F-NT2RP3001061
KERATIN, TYPE II CYTOSKELETAL 7
3.4e-05:256:62
Hs.23881:M99063
F-NT2RP3001159
Homo sapiens chromosome 11, BAG CIT-HSP-311e8 (BC269730) containing the hFEN1 gene
1.8e-81:527:70
Hs.132874:AC004770
F-NT2RP3001170
Homo sapiens mRNA for KIAA0784 protein, partial cds
7.3e-183:859:98
Hs.3657:AB018327
F-NT2RP3001195
ESTs
3.5e-08:282:62
Hs.135168:AI394026
F-NT2RP3001240
ESTs, Highly similar to PROTEIN TRANSPORT PROTEIN SEC61 ALPHA SUBUNIT [Canis familiaris]
2.8e-64:344:95
Hs.14038:R06800
F-NT2RP3001271
Centromere protein B (80kD)
7.6e-08:288:64
Hs.85004:X05299
F-NT2RP3001322
ESTs, Weakly similar to W09D10.2 [C.elegans]
1.2e-86:422:98
Hs.26107:R60661
F-NT2RP3001388
F-NT2RP3001542
F-NT2RP3001560
Protein phosphatase 2C alpha [human, teratocarcinoma, mRNA, 2346 nt] 0.016:190:63
Hs.57764:S87759
F-NT2RP3001592
Cyclin-dependent kinase inhibitor 1C (p57, Kip2)
2.3e-13:188:71
Hs.106070:U22398
F-NT2RP3001650
Homo sapiens KIAA0415 mRNA, complete cds
1.6e-17:394:66
Hs.7289:AB007875
F-NT2RP3001685
F-NT2RP3001738
Homo sapiens chromosome 11, BAC CIT-HSP-311e8 (BC269730) containing the hFEN1 gene
1.9e-54:776:65
Hs.132898:AC004770
F-NT2RP3001754
Homo sapiens mRNA for B120, complete cds
2.4e-18:106:100
Hs.123090:AB001895
F-NT2RP3001858
Homo sapiens mRNA for KIAA0584 protein, partial cds
1.9e-40:770:63
Hs.106794:AB011156
F-NT2RP3001976
Zinc finger protein 140 (clone pHZ-39)
7.3e-33:493:68
Hs.154205:U09368
F-NT2RP3002015
Homo sapiens OPA-containing protein mRNA, complete cds
0.018:329:62
Hs.85313:AF071309
F-NT2RP3002160
Homo sapiens protein phosphatase with EF-hands-2 long form (PPEF-2) mRNA, complete cds
0.53:182:64
Hs.113259:AF023456
F-NT2RP3002281
Homo sapiens mRNA for KIAA0765 protein, partial cds
5. 2e-151:713:98
Hs.62318:AB018308
F-NT2RP3002286
ESTs
0.034:48:95
Hs.124692:AA777421
F-NT2RP3002311
Beta-galactosidase (GLB1)
2.3e-28:633:61
Hs.79222:M34423
F-NT2RP3002324
ESTs
2.5e-28:296:75
Hs.22822:H06408
F-NT2RP3002342
Human transporter protein (g17) mRNA, complete cds
3.2e-37:565:65
Hs.76460:U49082
F-NT2RP3002353
Homo sapiens mRNA for KIAA0790 protein, partial cds
0.0055:271:60
Hs.12002:AB018333
F-NT2RP3002409
Homo sapiens mRNA for KIAA0719 protein, complete cds
6. 4e-191:89/:98
Hs.21198:AB018262
F-NT2RP3002411
Hydroxysteroid (17-beta) dehydrogenase 3
2.9e-28:604:62
Hs.477:U05659
F-NT2RP3002448
Human mRNA for KIAA0233 gene, complete cds
1.6e-08:721:57
Hs.79077:087071
F-NT2RP3002571
Homo sapiens mRNA for KIAA0603 protein, complete cds 9.7e-67:707:71
Hs.16909:AB011175
F-NT2RP3002664
Homo sapiens Trio isoform mRNA, complete cds 0.26:160:66
Hs.150625:AF091395
F-NT2RP3002721
Homo sapiens citrate synthase mRNA, complete cds 2.4e-180:873:96
Hs.132991:AF047042
F-NT2RP3002737
Homo sapiens mRNA for voltage gated potassium channel 7.1e-43:409:75
Hs.4975:Y15065
F-NT2RP3002738
Human BMK1 alpha kinase mRNA, complete cds 0.0070:722:57
Hs.3080:U29725
F-NT2RP3002790
Cyclin-dependent kinase inhibitor 1C (p57, Kip2) 7.2e-17:626:62
Hs.106070:U22398
F-NT2RP3002836
Homo sapiens mRNA for KIAA0463 protein, partial cds 2.2e-153:717:99
Hs.77738:AB007932
F-NT2RP3002887
Human plectin (PLEC1) mRNA, complete cds
2.5e-06:605:59
Hs.79706:U53204
F-NT2RP3002900
H.sapiens mRNA for transmembrane protein rnp24
3.1e-09:346:64
Hs.75914:X92098
F-NT2RP3002958
ESTs
8.3e-117:765:86
Hs.107119:AI198794
F-NT2RP3002983
ESTs
1.4e-07:270:67
Hs.160271:AI149075
F-NT2RP3003000
Homo sapiens T-type calcium channel alpha-1 subunit mRNA, complete cds 2.5e-89:555:88
Hs.122359:AF051946
F-NT2RP3003076
Homo sapiens mRNA for APC 2 protein, complete cds
0.00016:522:60
Hs.20912:AB012162
F-NT2RP3003354
Homo sapiens secretory carrier membrane protein (SCAMP2) mRNA, complete cds 4.0e-36:625:64
Hs.10761:AF005038
F-NT2RP3003448
Arginine vasopressin receptor 1B
0.77:149:69
Hs.1372:L37112
F-NT2RP3003469
ESTs
1.4e-42:239:93
Hs.12610:W56112
F-NT2RP3003473
ESTs, Highly similar to transcription factor ARF6 chain B [M.musculus] 8.7e-46:281:89
Hs.136172:W28257
F-NT2RP3003527
Homo sapiens mRNA for protein kinase DyrklB
4.6e-162:769:98
Hs.130988:Y17999
F-NT2RP3003532
OX-2 MEMBRANE GLYCOPROTEIN PRECURSOR
1.5e-146:682:98
Hs.79015:M17229
F-NT2RP3003535
EST
6.7e-10:330:60
Hs.133239:AI052508
F-NT2RP3003559
Breakpoint cluster region protein BCR
1.0:143:66
Hs.2557:Y00661
F-NT2RP3003614
ESTs
3.7e-50:327:88
Hs.148873:T33582
F-NT2RP3003729
ESTs, Weakly similar to unknown [S.cerevisiae]
1.9e-96:449:99
Hs.100843:W28953
F-NT2RP3003849
ESTs, Weakly similar to rhophilin [M.musculus]
1.7e-32:197:92
Hs.118457:AA019161
F-NT2RP3003874
Homo sapiens incomplete cDNA for a myosin class I, myh-1c
8.5e-84:494:90
Hs.109805:AJ001381
F-NT2RP3003939
Peroxisomal biogenesis factor 6
1.5e-05:236:62
Hs.30729:083703
F-NT2RP3003963
F-NT2RP3004000
Homo sapiens mRNA for APC 2 protein, complete cds
4.8e-06:669:59
Hs.20912:AB012162
F-NT2RP3004025
ESTs
0.0015:68:86
Hs.154835:AI289188
F-NT2RP3004067
ESTs, Weakly similar to HYPOTHETICAL 51.2 KD PROTEIN IN LAG1-RPL14B INTERGENIC REGION [S.cerevisiae]
2.1e-76:416:94
Hs.9252:R53360
F-NT2RP3004075
ESTs
1.1e-54:298:94
Hs.124051:T15786
F-NT2RP3004083
F-NT2RP3004090
Homo sapiens Achaete-Scute homologue 2 (ASCL2) gene, complete cds 2.4e-06:486:62
Hs.135639:U77629
F-NT2RP3004119
Human mRNA for KIAA0215 gene, complete cds
4.1e-74:640:75
Hs.82292:D86969
F-NT2RP3004130
F-NT2RP3004133
ESTs, Weakly similar to similar to M. musculus MER5 and other AHPC/TSA proteins [C.elegans]
4.6e-52:259:98
Hs.132096:AA314601
F-NT2RP3004202
ALPHA-2C-1 ADRENERGIC RECEPTOR
1.0:229:62
Hs.123022:J03853
F-NT2RP3004294
Homo sapiens mRNA for KIAA0741 protein, complete cds
2.4e-05:404:59
Hs.3615:AB018284
F-NT2RP3004309
Homo sapiens chromosome 11, BAC CIT-HSP-311e8 (BC269730) containing the hFEN1 gene
3.4e-71:756:71
Hs.132874:AC004770
F-NT2RP3004321
Homo sapiens (clone MG2-5-12) mucin (MG2) mRNA, complete polyA site 0.015:263:60
Hs.103944:L13283
F-NT2RP3004345
Cyclin-dependent kinase inhibitor 1C (p57, Kip2)
2.3e-13:188:71
Hs.106070:U22398
F-NT2RP3004355
EST
0.25:130:59
Hs.149436:AI274484
F-NT2RP3004374
ESTs
1.4e-95:480:96
Hs.12610:W56112
F-NT2RP3004406
Human breast cancer, estrogen regulated LIV-1 protein (LIV-1) mRNA, partial cds
3.4e-45:505:70
Hs.79136:U41060
F-NT2RP3004481
Homo sapiens mRNA for KIAA0476 protein, complete cds
0.00065:594:58
Hs.6684:AB007945
F-NT2RP3004552
Biglycan
0.92:347:57
Hs.821:J04599
F-NT2RP3004557
Human Ki nuclear autoantigen mRNA, complete cds
2.6e-121:626:94
Hs.152978:U11292
F-NT2RP3004625
Homo sapiens I-1 receptor candidate protein mRNA, complete cds
3.1e-152:710:98
Hs.26285:AF082516
F-NT2RP3004640
ESTs, Moderately similar to unknown [H.sapiens]
0. 76:195:64
Hs.6487:165302
F-NT2RP3004647
Homo sapiens mRNA for KIAA0446 protein, complete cds
6.6e-111:524:98
Hs.158286:AB007915
F-NT2RP4000108
NEUROFILAMENT TRIPLET L PROTEIN
5.3e-159:862:93
Hs. 159540:X05608
F-NT2RP4000634
Human MEK kinase 3 mRNA, complete cds
2.3e-54:370:71
Hs.86201:U78876
F-NT2RP4000962
ESTs, Weakly similar to SPORULATION-SPECIFIC PROTEIN 1 [Saccharomyces cerevisiae]
2.3e-95:479:96
Hs.4789:AI418298
F-NT2RP4001001
EST
0.98:93:64
Hs.147598:AI217868
F-NT2RP4001009
Homo sapiens mRNA for Hs Ste24p, complete cds
3.1e-176:828:98
Hs.25846:AB016068
F-NT2RP4001467
5' nucleotidase (CD73)
1.1e-160:742:98
Hs.153952:X55740
F-NT2RP4001877
ESTs, Weakly similar to siah binding protein 1 [H.sapiens]
3.3e-103:495:98
Hs.65648:AA600816
F-NT2RP4001879
EST
0.78:171:61
Hs.112790:AA609949
F-NT2RP4002187
Hydroxysteroid (17-beta) dehydrogenase 3
9.9e-27:534:63
Hs.477:U05659
F-NT2RP4002451
ESTs
1.5e-11:106:86
Hs.163724:AA017689
F-NT2RP4002715
EST
4.2e-07:64:93
Hs.160901:Al366910
F-NT2RP4002750
Ecotropic retroviral receptor
6.6e-51:581:68
Hs.2928:X57303
F-OVARC1000003
Solute carrier family 17 (sodium phosphate), member 2
6.9e-65:587:73
Hs.936:L13258
F-OVARC1000090
ESTs
4.8e-07:214:65
Hs.87456:AA434484
F-OVARC1000105
Human novel homeobox mRNA for a DNA binding protein
0.00095:204:64
Hs. 37035:U07664
F-OVARC1000137
Human SNARE protein Ykt6 (YKT6) mRNA, complete cds
4.0e-35:184:98
Hs.31531:U95735
F-OVARC1000208
Human calcium-dependent group X phospholipase A2 mRNA, complete cds
1.5e-61:365:90
Hs.136004:U95301
F-OVARC1000255
Spleen tyrosine kinase
2.2e-88:615:84
Hs.74101:L28824
F-OVARC1000275
ESTs, Weakly similar to Rap2 interacting protein 8 [M.musculus]
4.7e-85:424:97
Hs.55165:AA573499
F-OVARC1000298
Homo sapiens GABA-B receptor mRNA, complete cds
0.00021:285:61
Hs.12307:AF056085
F-OVARC1000307
ESTs
0.00016:226:63
Hs.162935:AI393970
F-OVARC1000313
Homo sapiens mRNA for KIAA0573 protein, partial cds
5.5e-121:585:97
Hs.154023:AB011145
F-OVARC1000331
Glucose-6-phosphate dehydrogenase
5.3e-18:213:71
Hs.1435:M24470
F-OVARC1000410
Homo sapiens mRNA for angiopoietin-like factor
1.5e-27:538:62
Hs.146559:Y16132
F-OVARC1000439
F-OVARC1000467
ESTs
2.5e-26:173:90
Hs.105040:AA292817
F-OVARC1000529
Homo sapiens mRNA for C8FW phosphoprotein
1.1e-12:391:59
Hs.143513:AJ000480
F-OVARC1000553
Homo sapiens chromosome 19, cosmid R26894 9.0e-111:425:99
Hs.157732:AC005594
F-OVARC1000775
Human chromosome 3p21.1 gene sequence
2.2e-70:380:95
Hs.82837:L13435
F-OVARC1000811
HEPATOCYTE GROWTH FACTOR ACTIVATOR PRECURSOR
1.2e-06:446:61
Hs.104:D14012
F-OVARC1000853
ESTs
7.9e-09:268:63
Hs.92700:W37903
F-OVARC1000873
Homo sapiens mRNA for MIFR-1, complete cds
0.038:343:60
Hs.58269:AB010962
F-OVARC1000916
H.sapiens PISSLRE mRNA
1.3e-56:435:82
Hs.77313:X78342
F-OVARC1000956
Human TBP-associated factor (hTAFII130) mRNA, partial cds
7.7e-05:511:59
Hs.24644:U75308
F-OVARC1000995
ESTs
2.4e-39:205:98
Hs.163662:AA514348
F-OVARC1001030
EST
1.1e-44:232:96
Hs.135504:AI091717
F-OVARC1001049
ESTs
6.1e-78:373:98
Hs.135022:AI417283
F-OVARC1001086
Homo sapiens cyclin T2a mRNA, complete cds
6.0e-166:761:99
Hs.155478:AF048731
F-OVARC1001132
ESTs, Weakly similar to GC-RICH SEQUENCE DNA-BINDING FACTOR [Homo sapiens]
7.9e-121:610:96
Hs.26461:AI341685
F-OVARC1001163
ESTs
5. 9e-39:215:94
Hs.126067:AI344351
F-OVARC1001222
ESTs
2.7e-93:467:95
Hs.10267:W27845
F-OVARC1001260
Pregnancy-zone protein
1.0:251:58
Hs.74094:X54380
F-OVARC1001336
Solute carrier family 17 (sodium phosphate), member 2
1.2e-31:304:74
Hs.936:L13258
F-OVARC1001338
Homo sapiens cam kinase I mRNA, complete cds
3.7e-17:570:60
Hs.118414:L41816
F-OVARC1001569
Human novel homeobox mRNA for a DNA binding protein
0.038:178:63
Hs.37035:U07664
F-OVARC1001570
ESTs
4.5e-10:75:93
Hs.120928:AA703165
F-OVARC1001596
Matrix metalloproteinase 2 (gelatinase A, 72kD gelatinase, 72kD type IV collagenase)
0.0092:287:63
Hs. 111301:M55593
F-OVARC1001607
Human beta-1,2-N-acetylglucosaminyltransferase II (MGAT2) gene, complete cds
5.5e-41:323:80
Hs.154844:U15128
F-OVARC1001725
F-OVARC1001727
EST
3.2e-05:237:61
Hs.119508:AA485732
F-OVARC1001807
Hormone receptor (growth factor-inducible nuclear protein N10)
3.4e-91:564:88
Hs.1119:049728
F-OVARC1001833
ESTs
1.2e-94:444:97
Hs.155256:AA707750
F-OVARC1001952
Myristoylated alanine-rich C-kinase substrate
2.9e-10:364:64
Hs.75607:010522
F-OVARC1001991
Human mRNA for KIAA0176 gene, partial cds
0.0019:224:62
Hs.4935:D79998
F-OVARC1002058
Human mRNA for KIAA0149 gene, complete cds
5.0e-48:674:67
Hs.57735:D86864
F-OVARC1002178
Homo sapiens zinc-finger protein of the cerebellum 3 (ZIC3) mRNA, complete cds
0.010:310:61
Hs.111227:AF028706
F-PLACE1000033
F-PLACE1000231
Guanine nucleotide binding protein (G protein), alpha 11 (Gq class) 0.00021:235:63
Hs.1686:M69013
F-PLACE1000258
KRAB zinc finger protein {alternative products}
1.2e-14:241:70
Hs.22556:U37251
F-PLACE1000442
Zinc finger protein 136 (clone pHZ-20)
7.3e-89:774:76
Hs.69740:U09367
F-PLACE1000560
ESTs
1.5e-36:200:96
Hs.86541:AA214554
F-PLACE1000740
Homo sapiens secreted apoptosis related protein 3 (SARP3) mRNA, complete cds
6.5e-05:283:62
Hs.113285:AF017988
F-PLACE1000907
ESTs, Moderately similar to zinc finger protein [H.sapiens]
8.1e-38:237:89
Hs.139115:AA325104
F-PLACE1000912
ESTs
4.6e-61:331:95
Hs.17558:AA155762
F-PLACE1000914
Homo sapiens PB39 mRNA, complete cds
3.1e-45:500:69
Hs.18910:AF045584
F-PLACE1000927
ESTs, Weakly similar to N-methyl-D-aspartate receptor-associated protein
[D.metanogaster]
1.4e-123:655:94
Hs.8661:AI189791
F-PLACE1000986
ESTs
1.2e-105:494:99
Hs.19207:AA039595
F-PLACE1001016
Calcium channel, voltage-dependent, L type, alpha 1S subunit
0.011:432:59
Hs.1294:L33798
F-PLACE1001100
Human clone 23839 mRNA sequence
0.38:342:60
Hs.78362:U79249
F-PLACE1001114
Human mRNA for KIAA0303 gene, partial cds
0.085:339:59
Hs.54985:AB002301
F-PLACE1001123
ESTs
5.0e-14:505:61
Hs.99272:AI147740
F-PLACE1001183
ESTs, Weakly similar to gene pp21 protein [H.sapiens]
0.66:361:58
Hs.15984:AI085974
F-PLACE1001229
ESTs, Weakly similar to D9481.15 gene product [S.cerevisiae]
9.3e-110:561:96
Hs.125155:W52093
F-PLACE1001231
ESTs, Weakly similar to sodium iodide symporter [H.sapiens]
1.0e-17:120:91
Hs.5167:AA053914
F-PLACE1001340
Homo sapiens mRNA for KIAA0719 protein, complete cds
4.1e-132:636:97
Hs.21198:AB018262
F-PLACE1001401
ESTs, Weakly similar to IgE receptor beta subunit [H.sapiens]
3.1e-100:516:95
Hs.43900:AA418443
F-PLACE1001407
H.sapiens mRNA for B-HLH DNA binding protein
0.00015:244:66
Hs.66744:X99268
F-PLACE1001464
5' nucleotidase (CD73)
1.6e-152:742:96
Hs.153952:X55740
F-PLACE1001500
Bloom syndrome
5.7e-05:450:58
Hs.36820:U39817
F-PLACE1001516
Homo sapiens Rigui (RIGUI) mRNA, complete cds
2.3e-07:663:58
Hs.8114:AF022991
F-PLACE1001536
ESTs
4.6e-60:318:97
Hs.13026:H04491
F-PLACE1001564
H.sapiens mRNA for HE6 Tm7 receptor
8.8e-41:499:70
Hs.155681:X81892
F-PLACE1001655
Homo sapiens Shab-related delayed-rectifier K+ channel alpha subunit (KCNS3) mRNA, complete cds
4.3e-125:585:98
Hs.47584:AF043472
F-PLACE1001788
Homo sapiens mRNA for HYA22, complete cds
3.2e-22:234:75
Hs.147189:D88153
F-PLACE1001795
F-PLACE1001836
ESTs, Moderately similar to !!!! ALU SUBFAMILY SQ WARNING ENTRY !!!!
[H.sapiens]
1.1e-18:162:80
Hs.157223:AA309318
F-PLACE1001918
Human p76 mRNA, complete cds
1.3e-22:693:60
Hs.28757:U81006
F-PLACE1001949
ESTs
0.97:243:63
Hs.151143:AA576926
F-PLACE1002080
Homo sapiens mRNA for KIAA0600 protein, partial cds
2.4e-130:622:98
Hs.9028:AF039691
F-PLACE1002095
F-PLACE1002153
Homo sapiens TACC2 protein (TACC2) mRNA, partial cds
2.7e-162:764:98
Hs.90415:AF095791
F-PLACE1002329
ESTs
1.5e-107:556:95
Hs.28907:AI343292
F-PLACE1002355
Homo sapiens protease-activated receptor 4 mRNA, complete cds
9.0e-19:190:77
Hs.137574:AF055917
F-PLACE1002374
Cathepsin L
2.0e-163:716:94
Hs.78056:X12451
F-PLACE1002518
Homo sapiens multiple membrane spanning receptor TRC8 (TRC8) mRNA, complete cds
7.0e-19:396:64
Hs.28285:AF064801
F-PLACE1002547
Homo sapiens mRNA for KIAA0719 protein, complete cds
8. 3e-173:819:98
Hs.21198:AB018262
F-PLACE1002726
Human mRNA for KIAA0362 gene, partial cds
1.0:310:59
Hs.25515:AB002360
F-PLACE1002905
ESTs
2.4e-74:415:92
Hs.110298:AA621807
F-PLACE1002911
ESTs, Weakly similar to Y53C12A.3 [C.elegans]
0.030:279:58
Hs.107747:AI357868
F-PLACE1002967
ESTs
3.3e-120:574:98
Hs.11090:W37646
F-PLACE1003135
Homo sapiens STE20-like kinase 3 (mst-3) mRNA, complete cds
1.5e-50:450:75
Hs.72292:AF024636
F-PLACE1003163
Homo sapiens DBI-related protein mRNA, complete cds
1.5e-153:722:98
Hs.15250:AF069301
F-PLACE1003407
Homo sapiens putative transmembrane protein (CLN5) mRNA, complete cds
2.0e-142:682:97
Hs. 30213:AF068227
F-PLACE1003428
Biotinidase
8.2e-06:265:62
Hs.78885:AF018631
F-PLACE1003438
ESTs
0.018:470:60
Hs.119482:AI361002
F-PLACE1003460
ESTs
0.019:211:60
Hs.92700:W37903
F-PLACE1003529
130 KD LEUCINE-RICH PROTEIN
0.53:208:63
Hs.87157:M92439
F-PLACE1003573
F-PLACE1003598
Calcium channel, voltage-dependent, P/Q type, alpha 1A subunit
0.00064:302:64
Hs. 96253:U79666
F-PLACE1003644
ESTs
1.3e-06:265:63
Hs.163564:R43678
F-PLACE1003737
F-PLACE1003772
Human p300/CBP-associated factor (P/CAF) mRNA, complete cds
7.0e-09:448:61
Hs.155302:U57317
F-PLACE1003839
Homo sapiens Chromosome 16 BAC clone CIT987SK-A-69G12
7.7e-109:521:97
Hs.154050:AC004131
F-PLACE1003845
ESTs, Moderately similar to similar to thymidine diphosphoglucose 4,6-dehydratase [C.elegans]
1.2e-92:432:100
Hs.153778:AI246000
F-PLACE1003852
Homo sapiens mRNA for KIAA0758 protein, partial cds
2.4e-172:814:98
Hs.22039:AB018301
F-PLACE1004028
F-PLACE1004078
GELSOLIN PRECURSOR, PLASMA
3.1e-49:616:67
Hs.80562:X04412
F-PLACE1004166
ESTs
7.6e-79:415:94
Hs.163741:AA551077
F-PLACE1004168
F-PLACE1004199
EST
6.8e-15:147:80
Hs.128205:AA972308
F-PLACE1004279
Homo sapiens putative renal organic anion transporter 1 (hROAT1) mRNA, complete cds
3.9e-20:456:62
Hs.23965:AF057039
F-PLACE1004282
F-PLACE1004305
Homo sapiens mRNA for KIAA0740 protein, complete cds
8.7e-123:612:96
Hs.15099:AB018283
F-PLACE1004441
Human G protein-coupled receptor (GPR1) gene, complete cds
8.6e-99:501:96
Hs.159248:U13666
F-PLACE1004450
AMINOPEPTIDASE N
1.1e-09:587:57
Hs. 1239:M22324
F-PLACE1004482
F-PLACE1004492
ESTs
2.1e-25:134:100
Hs.154475:AI199037
F-PLACE1004519
ESTs
1.0e-110:518:99
Hs.128505:AA306435
F-PLACE1004520
Pregnancy-specific beta 1-glycoprotein 7
1.3e-110:606:92
Hs.119662:M34715
F-PLACE1004630
Homo sapiens mRNA for osteoblast specific cysteine-rich protein, complete cds
2.0e-139:749:92
Hs.82582:AB008375
F-PLACE1004637
F-PLACE1004648
F-PLACE1004816
Homo sapiens mRNA for Hakata antigen, complete cds
1.2e-99:590:90
Hs.9225:D88587
F-PLACE1004887
Homo sapiens Achaete-Scute homologue 2 (ASCL2) gene, complete cds
5.1e-06:486:62
Hs.135639:U77629
F-PLACE1005003
Human SNC19 mRNA sequence
1.5e-21:472:63
Hs.56937:U20428
F-PLACE1005005
Homo sapiens ubiquitin conjugating enzyme G2 (UBE2G2) mRNA, complete cds
4.7e-42:245:93
Hs.151614:AF032456
F-PLACE1005031
ESTs, Highly similar to CHLORINE CHANNEL PROTEIN P64 [Bos taurus]
2.9e-43:538:70
Hs.118991:AA675919
F-PLACE1005239
ESTs
2.4e-42:209:100
Hs.154475:AI199037
F-PLACE1005250
F-PLACE1005383
Homo sapiens UP50 mRNA, complete cds
8.5e-128:633:96
Hs.11494:AF093118
F-PLACE1005410
ESTs, Highly similar to PROTEIN TRANSPORT PROTEIN SEC61 ALPHA SUBUNIT [Canis familiaris]
1.3e-17:181:75
Hs.131840:AI016073
F-PLACE1005426
Pregnancy-specific beta-1 glycoprotein 4
2.3e-109:596:93
Hs.108936:X17097
F-PLACE1005519
Homo sapiens STE20-like kinase 3 (mst-3) mRNA, complete cds
3.3e-55:521:74
Hs.72292:AF024636
F-PLACE1005539
HETEROGENOUS NUCLEAR RIBONUCLEOPROTEIN U
5.8e-05:277:63
Hs.103804:AF068846
F-PLACE1005544
F-PLACE1005569
EST
0.38:60:75
Hs.137086:AA912486
F-PLACE1005601
Homo Sapiens angiotensin II receptor gene, complete cds
0.016:72:84
Hs.20954:AI054441
F-PLACE1005660
F-PLACE1005669
Homo sapiens N-methyl-D-aspartate receptor 2D subunit precursor (NMDAR2D) mRNA, complete cds
3.5e-08:461:60
Hs.113286:U77783
F-PLACE1005682
F-PLACE1005725
Huntingtin (Huntington disease)
1.1e-06:401:61
Hs. 79391:L12392
F-PLACE1005736
ESTs
3.6e-63:343:94
Hs.17757:AA875839
F-PLACE1005745
ESTs, Weakly similar to HYPOTHETICAL 25.2 KD PROTEIN IN ACB1-KSS1 INTERGENIC REGION [S.cerevisiae]
6.9e-66:351:94
Hs.7870:AI078137
F-PLACE1005768
ESTs
7.9e-60:318:95
Hs.143856:AI186351
F-PLACE1005815
Mutated in colorectal cancers
0.0029:199:62
Hs. 1345:M62397
F-PLACE1005878
ESTs, Highly similar to CHLORINE CHANNEL PROTEIN P64 [Bos taurus]
5.0e-38:464:70
Hs.118991:AA675919
F-PLACE1005927
INSULIN-LIKE GROWTH FACTOR BINDING PROTEIN COMPLEX ACID LABILE CHAIN PRECURSOR
0.010:511:59
Hs.839:M86826
F-PLACE1006071
EST
0.68:224:59
Hs.161788:AA371859
F-PLACE1006073
Homo sapiens mRNA for glucuronyltransferase I, complete cds
5.5e-96:464:98
Hs. 26492:AB009598
F-PLACE1006079
Homo sapiens BAC clone RG300E22 from 7q21-q31.1
1.5e-18:402:65
Hs.99348:AC004774
F-PLACE1006093
Homo sapiens mRNA for protein phosphatase 1 (PPP1R6)
0.0022:306:59
Hs.106471:Y18206
F-PLACE1006208
HOMEOBOX/POU DOMAIN PROTEIN RDC-1
0.022:425:57
Hs.74095:L20433
F-PLACE1006219
ESTs, Moderately similar to similar to thymidine diphosphoglucose 4,6-dehydratase [C.elegans]
1.7e-61:294:100
Hs.153778:AI246000
F-PLACE1006277
EST
0.42:60:75
Hs.137086:AA912486
F-PLACE1006290
ESTs, Weakly similar to similar to M. musculus MER5 and other AHPC/TSA proteins [C.elegans]
1.3e-51:260:98
Hs.132096:AA314601
F-PLACE1006443
Homo sapiens PB39 mRNA, complete cds
1.2e-53:553:70
Hs.18910:AF045584
F-PLACE1006515
Homo sapiens mRNA for KIAA0576 protein, partial cds
1.3e-141:655:99
Hs. 14687:AB011148
F-PLACE1006716
EST
7.2e-12:148:75
Hs.162969:AA677315
F-PLACE1006786
ESTs
0.0050:125:72
Hs.109156:AA193501
F-PLACE1006809
ESTs
4.5e-99:477:98
Hs.135208:AI093908
F-PLACE1006959
ESTs
7.4e-72:381:93
Hs.4963:W29030
F-PLACE1007028
Homo sapiens p17-Beckwith-Wiedemann region 1 C (BWR1C) mRNA, complete cds
1.8e-18:364:65
Hs.154036:AF035444
F-PLACE1007040
H.sapiens NF-H gene, exon 1 (and joined CDS)
1.4e-09:501:61
Hs.75735:X15306
F-PLACE1007077
ESTs, Moderately similar to testis-specific TCP20 [H.sapiens]
0.88:192:62
Hs.85818:AI216525
F-PLACE1007081
Human plectin (PLEC1) mRNA, complete cds
0.079:403:60
Hs.79706:U53204
F-PLACE1007096
YY1 transcription factor
0.64:173:64
Hs.97496:M77698
F-PLACE1007296
ER LUMEN PROTEIN RETAINING RECEPTOR 1
4.2e-73:542:83
Hs. 78040:X55885
F-PLACE1007591
EST
0.026:136:64
Hs. 130897:AI014389
F-PLACE1007626
Homo sapiens unknown mRNA, complete cds
2.6e-105:516:97
Hs.11441:AF047439
F-PLACE1007702
Homo sapiens mRNA for UTF1, complete cds
0.033:297:62
Hs.158307:AB011076
F-PLACE1007845
ESTs
4.8e-22:158:89
Hs.23445:AA489015
F-PLACE1007881
F-PLACE1007971
ESTs, Weakly similar to K07F5. 14 [C.elegans]
1.1e-128:599:99
Hs.157918:AA313781
F-PLACE1008282
ESTs, Highly similar to HEME-REGULATED EUKARYOTIC INITIATION FACTOR EIF-2-ALPHA KINASE [Oryctolagus cuniculus]
2.4e-65:353:94
Hs.130830:W27380
F-PLACE1008297
F-PLACE1008359
Human arginine-rich protein (ARP) gene, complete cds
0.020:197:64
Hs.75412:M83751
F-PLACE1008469
Homo sapiens PB39 mRNA, complete cds
5.3e-20:620:60
Hs.18910:AF045584
F-PLACE1008549
Homo sapiens E74-like factor 5 (ELF5) mRNA, complete cds
1.8e-145:693:98
Hs.159267:AF049703
F-PLACE1008657
VILLIN
2.3e-10:356:61
Hs.3046:X12901
F-PLACE1008716
Human beta-1,2-N-acetylglucosaminyltransferase II (MGAT2) gene, complete cds
1.5e-31:191:92
Hs.154844:U15128
F-PLACE1008744
F-PLACE1008984
Pregnancy-associated plasma protein A
0.0085:268:60
Hs.158229:U28727
F-PLACE1008985
Signal transducer and activator of transcription 5A
0.0047:249:64
Hs.14203:U43185
F-PLACE1009067
Human density enhanced phosphatase-1 mRNA, complete cds
2.0e-06:453:60
Hs.1177:U10886
F-PLACE1009196
F-PLACE1009279
Homo sapiens mRNA for serin protease with IGF-binding motif, complete cds
1.9e-11:327:64
Hs.75111:D87258
F-PLACE1009527
Human DNA-binding protein ABP/ZF mRNA, complete cds
6.8e-21:125:96
Hs.86185:U82613
F-PLACE1009546
TRANSCRIPTION FACTOR RELB
0.051:248:61
Hs. 858:M83221
F-PLACE1009600
ESTs
1.0:124:64
Hs. 52794:W51887
F-PLACE1009735
ESTs
0.022:387:61
Hs.132253:AI027207
F-PLACE1009982
F-PLACE1010011
Homo sapiens transcription factor forkhead-like 7 (FKHL7) gene, complete cds
1.3e-09:330:66
Hs.143551:AF048693
F-PLACE1010078
ESTs, Weakly similar to HYPOTHETICAL 25.2 KD PROTEIN IN ACB1-KSS1 INTERGENIC REGION [S.cerevisiae]
8.3e-47:474:72
Hs.13144:T67556
F-PLACE1010081
Homo sapiens serine/threonine kinase RICK (RICK) mRNA, complete cds
2.2e-151:733:97
Hs.103755:AF027706
F-PLACE1010251
Homo sapiens Na+/H+ exchanger regulatory factor 2 (NHERF-2) mRNA, complete cds
0.0037:405:60
Hs.101813:AB016243
F-PLACE1010445
ESTs
1. 7e-45: 235: 97
Hs.144501:N39767
F-PLACE1010713
Hydroxysteroid (17-beta) dehydrogenase 3
2.8e-20:447:62
Hs.477:U05659
F-PLACE1010784
Human protease-activated receptor 3 (PAR3) mRNA, complete cds
0.56:199:59
Hs.159196:U92971
F-PLACE1010827
H.sapiens mRNA for transmembrane protein rnp24
2.9e-09:346:64
Hs.75914:X92098
F-PLACE1010968
ESTs
0.00062:52:98
Hs.119408:T87544
F-PLACE1011045
Homo sapiens E1-like protein mRNA, complete cds
6.0e-129:595:99
Hs.28190:AF094516
F-PLACE1011116
F-PLACE1011181
ESTs
1.0:301:58
Hs.80285:AI092519
F-PLACE1011236
Homo sapiens putative renal organic anion transporter 1 (hROAT1) mRNA, complete cds
1.1e-41:776:62
Hs.23965:AF057039
F-PLACE1011364
ESTs, Weakly similar to HYPOTHETICAL 141.2 KD PROTEIN EEED8.9 IN CHROMOSOME II [C.elegans]
3.7e-53:276:96
Hs.106499:W28299
F-PLACE1011407
ESTs, Moderately similar to ZINC FINGER PROTEIN 140 [H.sapiens]
3.2e-15:228:70
Hs.152174:AI199619
F-PLACE1011516
ESTs, Weakly similar to HYPOTHETICAL 21.5 KD PROTEIN IN SEC15-SAP4 INTERGENIC REGION [S.cerevisiae]
1.7e-85:444:95
Hs.110978:AA843431
F-PLACE1011708
Homo sapiens intrinsic factor-B12 receptor precursor, mRNA, complete cds
5.9e-145:722:96
Hs.148318:AF034611
F-PLACE1011824
Human Ste20-like kinase (MST2) mRNA, complete cds
1.6e-101:561:92
Hs.92317:U26424
F-PLACE1011978
Homo sapiens DNA from chromosome 19, BAC 33152
3.8e-67:733:72
Hs.55452:AC003973
F-PLACE2000118
Homo sapiens DNA sequence from PAC 393P12 on chromosome Xp11.21. Contains a hypothetical protein KIAA0413 (KIAA0412, KIAA0065, KIAA0569) LIKE Zinc Finger protein pseudogene, a hypothetical Proline-rich protein KIAA0269 LIKE gene and a 60S Ribosomal Protein L7 LIKE pseudogene. Contains ESTs, an STS and a GSS (BAC end sequence)
7.8e-115:568:95
Hs.120856:AL022578
F-PLACE2000219
EST
8.7e-11:137:75
Hs.98191:AA417044
F-PLACE3000181
Human protocadherin 42 mRNA, complete cds for abbreviated PC42
1.5e-128:745:90
Hs.79769:L11370
F-PLACE3000213
EST
1.0:219:63
Hs.98452:AA426058
F-PLACE4000354
ESTs
1.4e-13:190:71
Hs.138841:R94879
F-PLACE4000455
F-SKNMC1000004
Homo sapiens GABA-B receptor mRNA, complete cds
0.00039:275:62
Hs.12307:AF056085
F-SKNMC1000014
ESTs
3.3e-38:196:98
Hs.113307:H16716
F-SKNMC1000082
Complement component 4A
0.98:324:63
Hs. 76682:K02403
F-THYRO1000036
Homo sapiens mRNA for putative ATPase, partial
0.98:199:60
Hs. 91471:AJ006268
F-THYRO1000061
Human kinase Myt1 (Myt1) mRNA, complete cds
1.0:210:62
Hs.77783:AF014118
F-THYRO1000099
ESTs
2.5e-119:605:96
Hs.11782:W07369
F-THYRO1000196
Homo sapiens Ksp-cadherin (CDH16) mRNA, complete cds
1.6e-126:475:98
Hs.115418:AF016272
F-THYRO1000400
Human R kappa B mRNA, complete cds
0.64:223:63
Hs.95262:U08191
F-THYRO1000580
ESTs, Weakly similar to ZINC FINGER PROTEIN 7 [H.sapiens]
5.4e-27:248:76
Hs.25465:AA528105
F-THYRO1000584
Alpha mannosidase II isozyme
2.2e-06:528:60
Hs.155961:L28821
F-THYRO1000678
Gap junction protein, beta 2, 26kD (connexin 26)
1.3e-33:266:80
Hs.81795:M86849
F-THYRO1000776
Human involucrin mRNA
0.0025:497:59
Hs.157091:M13903
F-THYRO1000795
MITOCHONDRIAL 2-OXOGLUTARATE/MALATE CARRIER PROTEIN
4.1e-19:532:62
Hs.3816:AF070548
F-THYRO1000846
Homo sapiens centrosomal Nek2-associated protein 1 (C-NAP1) mRNA, complete cds
0.029:387:60
Hs.27910:AF049105
F-THYRO1000866
Homo sapiens SKB1Hs mRNA, complete cds
1.1e-92:529:89
Hs.12912:AF015913
F-THYRO1000956
Homo sapiens mRNA for G-protein coupled receptor
1.8e-15:474:64
Hs.155235:Y13583
F-THYRO1000964
Human OB binding protein-2 (OB-BP2) mRNA, complete cds
0.22:303:61
Hs.117005:U71383
F-THYRO1000999
EST
2.0e-05:198:63
Hs.146520:AI130948
F-THYRO1001063
Human mRNA for cerebroside sulfotransferase, complete cds
0.51:448:60
Hs.17958:D88667
F-THYRO1001071
ESTs
2.1e-29:237:83
Hs.155582:AI125241
F-THYRO1001102
ESTs, Weakly similar to growth arrest inducible gene product [H.sapiens]
4.7e-32:208:88
Hs.7854:W21970
F-THYRO1001113
Homo sapiens dysferlin mRNA, complete cds
3.2e-53:684:68
Hs.143897:AF075575
F-THYRO1001128
ESTs
2.1e-120:589:97
Hs.62595:AA306052
F-THYRO1001205
F-THYRO1001237
ESTs
0.66:326:60
Hs.148352:U80757
F-THYRO1001242
Protein phosphatase 2C alpha [human, teratocarcinoma, mRNA, 2346 nt]
0. 017:188:63
Hs.57764:S87759
F-THYRO1001266
Human sodium iodide symporter mRNA, complete cds
8.6e-43:806:62
Hs.103983:U66088
F-THYRO1001327
ESTs
2.8e-50:264:96
Hs.154667:AI343524
F-THYRO1001456
EST
0.90:84:72
Hs.130049:AA902650
F-THYRO1001457
Protein kinase C, mu
6.0e-53:705:67
Hs. 2891:X75756
F-THYRO1001471
ESTs
8.0e-52:278:94
Hs.7604:W31115
F-THYRO1001478
Human mRNA for KIAA0150 gene, partial cds
0.79:150:66
Hs.98508:D63484
F-THYRO1001495
Homo sapiens KIAA0415 mRNA, complete cds
9.5e-75:550:82
Hs.7289:AB007875
F-THYRO1001523
ESTs
7. 2e-19:142:86
Hs.140588:H60533
F-THYRO1001529
ESTs
5. 7e-24:141 :95
Hs.114172:AA703201
F-THYRO1001593
H.sapiens mRNA for serine/threonine protein kinase EMK
1.4e-70:643:74
Hs.157199:X97630
F-THYRO1001608
Human mRNA for KIAA0227 gene, partial cds
2.6e-07:533:59
Hs. 79170:D86980
F-THYRO1001641
ESTs
0.87:269:59
Hs.14599:AA522511
F-THYRO1001700
Homo sapiens c-Jun N-terminal kinase kinase 2 (JNKK2) mRNA, complete cds
3.3e-05:441:59
Hs.110299:AF013589
F-THYRO1001702
Human plectin (PLEC1) mRNA, complete cds
0.00017:346:62
Hs.79706:U53204
F-THYRO1001725
Homo sapiens mRNA for procollagen I-N proteinase
1.3e-06:275:64
Hs.120330:AJ003125
F-THYRO1001770
Homo sapiens mRNA for HsGAK, complete cds
0.046:265:58
Hs.153227:D88435
F-THYRO1001803
EST
0.0085:201:63
Hs.158782:AI376601
F-Y79AA1000030
ESTs
0.00051:276:60
Hs.111999:AA465020
F-Y79AA1000127
ESTs
1.3e-85:430:96
Hs.49932:W58552
F-Y79AA1000207
ESTs
4.5e-81:407:96
Hs.125308:AI376737
F-Y79AA1000226
ESTs, Highly similar to HYPOTHETICAL 52.8 KD PROTEIN T05E11.5 IN CHROMOSOME IV [Caenorhabditis elegans]
0.00081:76:84
Hs.11221:AI192291
F-Y79AA1000270
Human mRNA for ORF, Xq terminal portion
9.9e-97:590:88
Hs.6551:D16469
F-Y79AA1000426
CELL SURFACE GLYCOPROTEIN MUC18 PRECURSOR
0.045:507:59
Hs.82914:X68264
F-Y79AA1000521
Homo sapiens mRNA for putative G-protein coupled receptor, EDG6 0.0029:489:58
Hs.159543:AJ000479
F-Y79AA1000750
ESTs
9.9e-12:252:65
Hs.52885:H29851
F-Y79AA1000776
ESTs
1.4e-50:340:87
Hs.144198:AI017555
F-Y79AA1000777
Homo sapiens mRNA for putative transcription factor, partial
3. 9e-10 : 501 : 61
Hs.26782:AJ009770
F-Y79AA1000876
Homo sapiens short form transcription factor C-MAF (c-maf) mRNA, complete cds 1.3e-11:323:66
Hs.30250:AF055376
F-Y79AA1000888
Homo sapiens mRNA for KIAA0469 protein, complete cds
1.5e-05:641:58
Hs.7764:AB007938
F-Y79AA1000959
Homo sapiens Hsp70 binding protein HspBP1 mRNA, complete cds
5.3e-54:277:96
Hs.53066:AF093420
F-Y79AA1000967
Human mRNA for KIAA0369 gene, complete cds
8.1e-10:517:61
Hs.21355:AB002367
F-Y79AA1001013
ESTs
2.4e-44:259:93
Hs.109468:W52074
F-Y79AA1001056
ESTs, Moderately similar to maternal transcript Maid [M.musculus] 4.7e-07:90:87
Hs.36794:AI038407
F-Y79AA1001062
ESTs, Weakly similar to tumor necrosis factor-alpha-induced protein B12 [H. sap i ens]
1.6e-60:320:96
Hs.13982:W27344
F-Y79AA1001090
H.sapiens DAP-kinase mRNA
2.3e-06:465:59
Hs.153924:X76104
F-Y79AA1001212
Homo sapiens SL15 protein mRNA, complete cds
1. 5e-163: 763: 98
Hs.6710:AF038961
F-Y79AA1001264
Homo sapiens mRNA for MSJ-1, complete cds
5.3e-15:367:64
Hs.3845:AB014888
F-Y79AA1001272
Human plectin (PLEC1) mRNA, complete cds
6.3e-05:325:63
Hs.79706:U53204
F-Y79AA1001328
Homo sapiens Delta mRNA, complete cds
1.8e-07:271:61
Hs.144631:AF003522
F-Y79AA1001426
Aldehyde dehydrogenase 7
0.94:485:56
Hs.83155:U10868
F-Y79AA1001427
NADH-CYTOCHROME B5 REDUCTASE
1.7e-56:649:69
Hs.75666:M28713
F-Y79AA1001430
Homo sapiens mRNA for KIAA0469 protein, complete cds
2.8e-124:577:99
Hs.7764:AB007938
F-Y79AA1001523
Homo sapiens transcription intermediary factor 1 (TIF1) mRNA, complete cds
1.1e-92:496:93
Hs.128763:AF009353
F-Y79AA1001530
Human beta-tubulin gene (5-beta) with ten Alu family members
1.0e-131:669:95
Hs.108014:X00734
F-Y79AA1001592
ESTs
1. 2e-88:212:97
Hs.131180:AA594251
F-Y79AA1001727
F-Y79AA1001787
Human mRNA for KIAA0315 gene, partial cds
0.48:248:63
Hs.3989:AB002313
F-Y79AA1001793
ESTs
1.4e-67:192:98
Hs.118559:AA887084
F-Y79AA1001795
Homo sapiens mRNA for GaIT4 protein
5. 3e-89:431 :98
Hs.21495:AL031228
F-Y79AA1001799
NUCLEAR FACTOR RIP140
0.54:182:62
Hs.155017:X84373
F-Y79AA1001803
ESTs, Highly similar to MELANOMA-ASSOCIATED ANTIGEN XP [Homo sapiens] 0.72:169:63
Hs.94011:AA627644
F-Y79AA1001863
EST
1.0:114:63
Hs.152260:AA489703
F-Y79AA1002022
B94 PROTEIN
5.7e-13:469:65
Hs.75522:M92357
F-Y79AA1002058
Homo sapiens clone 24733 mRNA sequence
1.7e-154:740:98
Hs.21970:AF052149
F-Y79AA1002121
EST
0.14:104:66
Hs.100070:M91493
F-Y79AA1002129
ESTs
5.1e-90:431:98
Hs.40719:AI183452
F-Y79AA1002213
F-Y79AA1002334
ESTs
5.0e-20:187:80
Hs.111900:AA397579
F-Y79AA1002373
ESTs
4.5e-37:192:98
Hs.118559:AA887084
F-Y79AA1002376
Homo sapiens cytoplasmic dyne in intermediate chain 1 mRNA, complete cds 1.2e-36:657:64
Hs.65248:AF063228
F-Y79AA1002378
Homo sapiens KIAA0426 mRNA, complete cds
4.9e-38:424:72
Hs.97476:AB007886
F-Y79AA1002381
CELL DIVISION PROTEIN KINASE 3
8.4e-17:580:61
Hs.100009:X66357

### Homology search result 9

The result of the homology search in the Human Unigene(http://www.ncbi.nlm.nih.gov/UniGene) using the clone sequences of the 3'-ends. Indicated are from the top,
the name of the clone sequence,
title of the top hit data,
the P-value: the length of the sequence used for comparison (nucleotide):similarity (%),
the Accession No. of the top hit data.

Blank indicates that the 3'-end sequence corresponding to the 5'-end was not determined in the clone.
Data were not shown for the clones in which the P-value was higher than 1.
R-HEMBA1000006
ESTs
1.0:85:71
Hs.130699:AA621478
R-HEMBA1000121
ESTs
1.3e-111:545:97
Hs.153432:AA098922
R-HEMBA1000128
ESTs, Weakly similar to HYPOTHETICAL 30.6 KD PROTEIN IN SCP160-MRPL8
INTERGENIC REGION PRECURSOR [S.cerevisiae]
3.0e-98:532:93
Hs.7745:H92988
R-HEMBA1000275
ESTs
6.5e-11:81:81
Hs.163492:AI334460
R-HEMBA1000300
Homo sapiens mRNA for putative lipoic acid synthetase, partial
1.2e-39:309:81
Hs.53531:AJ224162
R-nnnnnnnnnnnn
ESTs
4.9e-95:455:98
Hs.154009:AI284184
R-HEMBA1000462
Homo sapiens clone 243 unknown mRNA, complete sequence
3.6e-91:313:94
Hs.20423:AF091094
R-HEMBA1000477
ESTs
4.7e-111:541:97
Hs.84526:AI341541
R-HEMBA1000590
Homo sapiens mRNA for matrilin-4, partial
2.6e-102:547:93
Hs.129361:AJ007581
R-HEMBA1000634
ESTs
0.85:189:62
Hs.131268:AA909162
R-HEMBA1000671
ESTs
6.5e-84:432:96
Hs.31991:T78668
R-HEMBA1000713
Homo sapiens 10kD protein (BC10) mRNA, complete cds
4.0e-119:575:97
Hs.5300:AF053470
R-HEMBA1000732
EST
3.9e-81:435:92
Hs.146718:AI146722
R-nnnnnnnnnnnn
R-HEMBA1000875
EST
0.023:207:62
Hs.148275:AA907849
R-HEMBA1000940
Human macrophage-derived chemokine precursor (MDC) mRNA, complete cds
7.4e-31:211:81
Hs.97203:U83171
R-HEMBA1000962
ESTs
1.1e-104:515:97
Hs.8978:W63573
R-HEMBA1001184
EST
7.1e-07:382:62
Hs.124559:AA847550
R-HEMBA1001221
ESTs, Weakly similar to transmembrane protein [H.sapiens]
1.2e-95:487:95
Hs.22791:AI056665
R-HEMBA1001228
Human germline oligomeric matrix protein (COMP) mRNA, complete cds
4.0e-42:170:92
Hs.1584:AC003107
R-HEMBA1001272
ESTs
5. 7e-71 :514:84
Hs.26966:N74056
R-HEMBA1001296
EST
1.7e-93:494:95
Hs.102465:N27272
R-HEMBA1001297
Homo sapiens putative transcription factor CA150 mRNA, complete cds 1.5e-93:466:96
Hs.13063:AF017789
R-HEMBA1001390
ESTs
1. 6e-42 :181 : 89
Hs.139190:N55515
R-HEMBA1001563
Homo sapiens DEC-205 mRNA, complete cds
8.4e-42:311:83
Hs.153563:AF011333
R-HEMBA1001621
ESTs, Highly similar to PROBABLE G PROTEIN-COUPLED RECEPTOR APJ [Homo sapiens]
4.2e-56:386:86
Hs.9305:W84893
R-HEMBA1001878
Homo sapiens U5 snRNP-specific 40 kDa protein mRNA, complete cds
1.1e-80:433:93
Hs.10290:AF090988
R-HEMBA1001886
Zinc finger protein 141 (clone pHZ-44)
5.9e-61:530:80
Hs.159596:L15309
R-HEMBA1002048
ESTs
0.95:127:63
Hs.98690:AA431162
R-HEMBA1002131
R-HEMBA1002163
ESTs, Weakly similar to K09E9. 2 [C.elegans]
5.9e-36:225:90
Hs.26813:AI339473
R-HEMBA1002167
ESTs
1.5e-35:325:80
Hs.124171:N98933
R-HEMBA1002178
MICROSOMAL DIPEPTIDASE PRECURSOR
0.99:243:61
Hs.109:J05257
R-HEMBA1002195
Deoxyhypusine synthase
1.9e-19:109:100
Hs.79064:U79262
R-HEMBA1002227
Myristoylated alanine-rich C-kinase substrate
2.0e-116:567:97
Hs.75607:010522
R-HEMBA1002316
Homo sapiens mRNA for putative GTP-binding protein
8.2e-20:160:85
Hs.101033:Y14391
R-HEMBA1002420
ESTs, Weakly similar to T03G11.6 gene product [C.elegans]
2.7e-78:402:97
Hs.108354:W19984
R-HEMBA1002421
Syndecan 2 (heparan sulfate proteoglycan 1, cell surface-associated, fibroglycan)
1.9e-91:443:97
Hs. 1501 : J04621
R-HEMBA1002524
Human MHC Class I region proline rich protein mRNA, complete cds
1.0e-111:551:96
Hs.41548:U63336
R-HEMBA1002551
ESTs
3.4e-107:553:96
Hs.92071:W80592
R-HEMBA1002767
Homo sapiens chromosome 1p33-p34 beta-1,4-galactosyltransferase mRNA, complete cds
5.5e-108:568:95
Hs.19154:AF038660
R-HEMBA1002985
ESTs
4.4e-39:211:96
Hs.126894:AA932538
R-HEMBA1003047
Homo sapiens intrinsic factor-B12 receptor precursor, mRNA, complete cds
1.6e-115:571:96
Hs.148318:AF034611
R-HEMBA1003072
EST
0.044:220:61
Hs.136349:AA490873
R-HEMBA1003101
Homo sapiens tyrosylprotein sulfotransferase-2 mRNA, complete cds
1.2e-117:575:97
Hs.26350:AF049891
R-HEMBA1003120
Zinc finger protein 10 (KOX 1)
5.8e-41:412:73
Hs.2479:X78933
R-HEMBA1003230
Homo sapiens UP50 mRNA, complete cds
4.2e-44:258:93
Hs.11494:AF093118
R-HEMBA1003294
ESTs
4.3e-84:410:98
Hs.113517:AA418756
R-HEMBA1003315
ESTs, Weakly similar to TIP49 [R.norvegicus]
7.3e-73:476:87
Hs.6455:AA515838
R-HEMBA1003392
Homo sapiens LDL receptor-related protein 6 (LRP6) mRNA, complete cds
8.3e-117:557:98
Hs.23672:AF074264
R-HEMBA1003399
ESTs, Highly similar to MVP1 PROTEIN [Saccharomyces cerevisiae]
8.0e-94:526:92
Hs.12169:N38744
R-HEMBA1003487
ESTs
4.5e-84:417:96
Hs.21835:AA458524
R-HEMBA1003497
ESTs
1.4e-72:346:99
Hs.129837:AA778570
R-HEMBA1003530
ESTs
8.5e-82:459:91
Hs.22140:R41751
R-HEMBA1003602
ESTs
1.0e-101:592:90
Hs.124342:AA829829
R-HEMBA1003732
ESTs
2.1e-111:530:99
Hs.101660:AA481200
R-HEMBA1003945
Calcineurin B
2.9e-83:410:97
Hs.1335:M30773
R-HEMBA1004007
Homo sapiens PYRIN (MEFV) mRNA, complete cds
3.8e-57:382:77
Hs.113283:AF018080
R-HEMBA1004085
ESTs
3.0e-59:396:87
Hs.102480:AA520980
R-nnnnnnnnnnnn
Homo sapiens intersectin short form mRNA, complete cds
2.0e-116:569:97
Hs.66392:AF064244
R-HEMBA1004250
ESTs
1.6e-97:469:97
Hs.125529:AA883986
R-HEMBA1004391
EST
0.085:113:63
Hs.157582:AI356856
R-HEMBA1004444
ESTs
2.3e-88:430:98
Hs.141680:N98441
R-HEMBA1004454
ESTs
1.7e-71:338:100
Hs.103913:AA740543
R-HEMBA1004505
ESTs
2.2e-63:329:95
Hs.4814:AA631254
R-HEMBA1004785
EST
1.0:77:67
Hs.144066:AA905236
R-HEMBA1004797
ESTs
4.1e-11:71:100
Hs.27206:AA626782
R-HEMBA1004952
ESTs
6.0e-93:435:99
Hs.115120:AA935633
R-HEMBA1004971
ESTs
0.98:152:58
Hs.112621:AA608964
R-HEMBA1004982
ESTs
2. 3e-109:516:98
Hs.14877:AA749081
R-HEMBA1005070
Human mRNA for KIAA0310 gene, complete cds
4.0e-96:381:91
Hs. 5716:AB002308
R-HEMBA1005084
ESTs
1.0:75:80
Hs.62119:AA043299
R-HEMBA1005145
Homo sapiens LIM protein mRNA, complete cds
1.6e-58:278:84
Hs.154103:AF061258
R-HEMBA1005230
ESTs
3.7e-65:336:95
Hs.124946:AI026708
R-HEMBA1005246
R-HEMBA1005267
Human protein immuno-reactive with anti-PTH polyclonal antibodies mRNA, partial cds
7.8e-75:536:81
Hs.44566:U28831
R-HEMBA1005337
EST
8.7e-58:291:97
Hs.48956:N64339
R-HEMBA1005430
ESTs
7.6e-83:388:100
Hs.28968:AA524690
R-HEMBA1005449
ESTs
5.0e-47:317:86
Hs.23650:H21144
R-HEMBA1005489
ESTs
1.8e-96:504:94
Hs.8028:AA053817
R-HEMBA1005522
EST
1.0:98:64
Hs.157385:AI364194
R-HEMBA1005545
MUSCARINIC ACETYLCHOLINE RECEPTOR M3
6.3e-117:579:96
Hs. 7138:U29589
R-HEMBA1005698
ESTs
1.6e-113:562:96
Hs.12942:AI042353
R-HEMBA1005913
ESTs
2.8e-109:564:94
Hs.28827:AI125541
R-HEMBA1005929
Human macrophage-derived chemokine precursor (MDC) mRNA, complete cds
9.6e-63:497:77
Hs.97203:U83171
R-HEMBA1005945
ESTs
1. 1e-74:412:92
Hs.32246:AA464020
R-HEMBA1006016
ESTs
1.4e-18:162:82
Hs.149448:AI082465
R-HEMBA1006171
EST
0.049:94:69
Hs.159919:AA961766
R-HEMBA1006276
ESTs
6.3e-22:257:75
Hs.138847:N64493
R-HEMBA1006299
ESTs, Weakly similar to R06B9.b [C.elegans]
3.9e-107:596:91
Hs.30432:W28988
R-HEMBA1006311
Homo sapiens SALL1 gene, partial
0.99:273:60
Hs.123094:X98833
R-HEMBA1006335
ESTs
2.5e-72:447:89
Hs.23579:W38893
R-HEMBA1006357
ESTs
6. 3e-15:187:74
Hs.161714:AA229078
R-HEMBA1006430
Homo sapiens tumor necrosis factor superfamily member LIGHT mRNA, complete cds
2.9e-47:303:88
Hs.129708:AF064090
R-HEMBA1006482
Homo sapiens h-sco1 (SC01) mRNA, nuclear gene encoding mitochondrial protein, complete cds
5.5e-107:537:96
Hs.14511:AF026852
R-HEMBA1006517
ESTs, Weakly similar to !!!! ALU SUBFAMILY J WARNING ENTRY !!!! [H.sapiens] 2.7e-43:173:86
Hs.141505:N30650
R-HEMBA1006544
Homo sapiens mRNA for small GTP-binding protein, complete cds
5.8e-60:329:80
Hs.115325:D84488
R-HEMBA1006572
ESTs
7.2e-94:450:99
Hs.123933:AA758566
R-HEMBA1006658
Homo sapiens mRNA for KIAA0687 protein, partial cds
2.3e-112:570:94
Hs.3628:AB014587
R-HEMBA1006707
Homo sapiens mRNA for matrilin-4, partial
1.7e-79:389:97
Hs.129361:AJ007581
R-HEMBA1006724
R-HEMBA1006749
Homo sapiens mRNA for matrilin-4, partial
1.0e-89:472:94
Hs.129361:AJ007581
R-HEMBA1006770
ESTs, Moderately similar to CAGH4 [H.sapiens]
2.0e-112:554:96
Hs.41641:AA428519
R-HEMBA1006902
Homo sapiens mRNA for matrilin-4, partial
3.0e-113:540:98
Hs.129361:AJ007581
R-HEMBA1006912
H.sapiens mRNA for phosphoinositide 3-kinase
5. 9e-45:297:86
Hs.101238:Y11312
R-HEMBA1006916
Homo sapiens Grb14 mRNA, complete cds
5.8e-116:346:99
Hs.83070:L76687
R-HEMBA1006960
ESTs
1.7e-110:519:99
Hs.22015:AI359551
R-HEMBA1007013
ESTs
0.53:280:59
Hs.143532:AI087321
R-HEMBA1007057
R-HEMBA1007063
EST
3.2e-41:310:83
Hs.163333:AA879053
R-HEMBA1007241
ESTs
1.8e-103:492:98
Hs.127478:AI188768
R-HEMBA1007291
Human mRNA for KIAA0266 gene, complete cds 8.7e-46:283:89
Hs.78878:D87455
R-HEMBA1007332
ESTs, Weakly similar to hTAFII100 [H.sapiens] 2.8e-17:161:80
Hs.3727:AA205887
R-HEMBB1000106
ESTs
1.3e-100:491:97
Hs.27774:AA576731
R-HEMBB1000276
R-HEMBB1000309
EST
1.0:150:64
Hs.125409:AA879096
R-HEMBB1000407
ESTs, Weakly similar to C47D12.2 [C.elegans]
4.1e-110:535:97
Hs.14328:AA503393
R-HEMBB1000447
Homo sapiens JWA protein mRNA, complete cds 5.6e-109:533:97
Hs.92384:AF070523
R-HEMBB1000542
ESTs, Weakly similar to C01H6.7 [C.elegans]
1.6e-88:497:91
Hs.18171:AA524327
R-HEMBB1000567
Insulin-like growth factor 2 (somatomedin A)
8.9e-61:369:88
Hs.155487:J03242
R-HEMBB1000642
ESTs
2.2e-44:308:84
Hs.141318:N71080
R-HEMBB1000668
ESTs, Weakly similar to hTAFII100 [H. sapiens]
2.5e-102:520:95
Hs.3830:AA167691
R-HEMBB1000679
ESTs
6.7e-36:188:97
Hs.154218:AA169554
R-HEMBB1000881
ESTs
8.4e-105:519:96
Hs.110967:AA570505
R-HEMBB1000905
ESTs
1.1e-94:454:98
Hs.52515:AA464314
R-HEMBB1001026
ESTs
0.22:93:69
Hs.119510:AA630235
R-HEMBB1001048
EST
0.42:127:66
Hs.147466:AI215091
R-HEMBB1001200
ESTs
3.7e-07:330:62
Hs.10109:AI148628
R-HEMBB1001407
MHC class II transactivator
3.8e-35:414:71
Hs.3076:U18259
R-HEMBB1001530
ESTs
2.4e-95:455:98
Hs.8956:AI146421
R-HEMBB1001547
ESTs
1.0e-111:533:98
Hs.33979:AI074147
R-HEMBB1001573
ESTs, Moderately similar to LL5 protein [R.norvegicus]
1.7e-06:64:95
Hs.131327:AI148746
R-HEMBB1001847
ESTs
1.4e-54:280:96
Hs.109755:AA180809
R-HEMBB1001959
Homo sapiens clone 24781 mRNA sequence
1.5e-104:504:97
Hs.108112:AF070640
R-HEMBB1001978
Homo sapiens mRNA for TRAF5, complete cds
7.0e-28:220:84
Hs.29736:AB000509
R-HEMBB1002039
ESTs, Weakly similar to !!!! ALU SUBFAMILY J WARNING ENTRY !!!! [H.sapiens] 1.5e-34:423:72
Hs.154912:N63897
R-HEMBB1002041
ESTs, Weakly similar to transmembrane protein [H.sapiens]
7.0e-122:575:98
Hs.22791:AI056665
R-HEMBB1002051
ESTs, Weakly similar to HYPOTHETICAL 92.1 KD PROTEIN ZK1098.3 IN CHROMOSOME III [Caenorhabditis elegans]
4.2e-43:298:84
Hs.141429:AA631915
R-HEMBB1002120
ESTs
1.4e-91:438:99
Hs.145014:AI218562
R-HEMBB1002162
ESTs
1.0e-34:238:86
Hs.164036:AA845659
R-HEMBB1002228
Homo sapiens neuronal thread protein AD7c-NTP mRNA, complete cds
1.7e-59:583:77
Hs.129735:AF010144
R-HEMBB1002245
ESTs
9.1e-66:383:91
Hs.8989:R71365
R-HEMBB1002302
ESTs
3.6e-54:329:89
Hs.37706:AA005120
R-HEMBB1002427
ESTs
4.2e-83:400:98
Hs.130783:AI263114
R-HEMBB1002465
EST
9.9e-38:231:90
Hs.133443:AI061405
R-HEMBB1002661
ESTs
2.5e-101:472:99
Hs.26878:AI421289
R-HEMBB1002663
Small inducible cytokine A5 (RANTES)
7.1e-43:268:88
Hs.155464:AF088219
R-HEMBB1002693
ESTs
4.6e-84:435:96
Hs.155522:AA829725
R-MAMMA1000046
EST
3.9e-06:196:65
Hs.136664:AA707467
R-MAMMA1000102
Human G protein-coupled receptor (STRL22) mRNA, complete cds 1.1e-31:237:83
Hs.46468:U45984
R-MAMMA1000106
ESTs
1.3e-65:333:95
Hs.130749:AI284219
R-MAMMA1000118
ESTs
7.3e-95:465:97
Hs.119286:AA126730
R-MAMMA1000141
ESTs
4.2e-94:515:93
Hs.8116:H23508
R-MAMMA1000204
Homo sapiens dysferlin mRNA, complete cds
2.3e-108:542:96
Hs.143897:AF075575
R-MAMMA1000226
ESTs
2.1e-112:535:98
Hs.105761:AA903862
R-MAMMA1000403
ESTs
1.5e-36:162:83
Hs.152413:AA780515
R-MAMMA1000449
EST
1.5e-40:347:78
Hs.163333:AA879053
R-MAMMA1000457
Homo sapiens clone 638 unknown mRNA, complete sequence
2.6e-117:570:97
Hs.5825:AF091084
R-MAMMA1000473
ESTs
1.3e-62:308:99
Hs.53565:W02102
R-MAMMA1000496
Phosphodiesterase 4C, CAMP-specific (dunce (Drosophila)-homolog phosphodiesterase E1)
0.051:125:68
Hs.189:AC005759
R-MAMMA1000528
ESTs
2.4e-12:216:71
Hs.134105:AI078038
R-MAMMA1000591
ESTs
5.0e-104:509:98
Hs.151678:AA032243
R-MAMMA1000614
Homo sapiens mRNA for KIAA0665 protein, complete cds
0.57:251:62
Hs.119004:AB014565
R-MAMMA1000652
ESTs
0.93:49:87
Hs.13248:R54144
R-MAMMA1000681
ESTs
1.3e-87:434:97
Hs.46668:N47089
R-MAMMA1000706
Homo sapiens cGMP phosphodiesterase A1 (PDE9A) mRNA, complete cds
3.7e-48:232:100
Hs.18953:AF067223
R-MAMMA1000788
ESTs
3.7e-108:559:94
Hs.38969:AA130220
R-MAMMA1000810
ESTs
1.2e-45:347:80
Hs.146811:AA410788
R-MAMMA1000814
ESTs
1. 1e-18:288:70
Hs.140608:N53448
R-MAMMA1000881
ESTs
1.9e-107:557:96
Hs.141602:N63562
R-MAMMA1000986
ESTs
3.8e-46:342:83
Hs.132722:AA618531
R-MAMMA1000994
Homo sapiens mRNA for ISLR, complete cds
1.2e-109:552:96
Hs.102171:AB003184
R-MAMMA1001043
ESTs
2.3e-88:445:96
Hs.20450:AI094818
R-MAMMA1001066
Homo sapiens KIAA0414 mRNA, partial cds
1.5e-43:282:81
Hs.127649:AB007874
R-MAMMA1001094
Homo sapiens clone 243 unknown mRNA, complete sequence
3.0e-116:566:97
Hs.20423:AF091094
R-MAMMA1001141
ESTs
1.2e-104:496:98
Hs.29669:AI285856
R-MAMMA1001150
ESTs, Highly similar to MYOSIN LIGHT CHAIN KINASE [Dictyostelium discoideum]
1.9e-59:284:100
Hs.9915:AI300083
R-MAMMA1001237
ESTs
0.45:206:62
Hs.121366:AA758653
R-MAMMA1001284
ESTs
6.3e-40:279:85
Hs.109765:AI096738
R-MAMMA1001310
ESTs, Moderately similar to !!!! ALU SUBFAMILY J WARNING ENTRY !!!!
[H. sapiens]
5.1e-98:498:96
Hs.27264:AA159597
R-MAMMA1001418
Human mRNA for platelet-activating factor acetylhydrolase 2, complete cds
1.2e-41:302:85
Hs.86188:D87845
R-MAMMA1001532
ESTs
3.9e-22:331:71
Hs.141840:AA028117
R-MAMMA1001609
Small inducible cytokine A5 (RANTES)
1.5e-31:277:78
Hs.155464:AF088219
R-MAMMA1001615
ESTs
1.1e-72:376:95
Hs.135569:AA923461
R-MAMMA1001623
ESTs
7.9e-106:505:98
Hs.22908:AI224910
R-MAMMA1001634
Homo sapiens PYRIN (MEFV) mRNA, complete cds
1.9e-44:428:76
Hs.113283:AF018080
R-MAMMA1001893
ESTs
8.0e-67:367:92
Hs.19210:W26097
R-MAMMA1001901
Homo sapiens mRNA, chromosome 1 specific transcript KIAA0492
4.7e-35:342:69
Hs.127338:AB007961
R-MAMMA1001957
Cytochrome P450, subfamily I (aromatic compound-inducible), polypeptide 2
5.5e-47:383:79
Hs.1361:M55053
R-MAMMA1001978
ESTs
6.6e-108:560:95
Hs.8859:AA191552
R-MAMMA1002070
R-MAMMA1002080
ESTs, Weakly similar to GTP-BINDING PROTEIN YPTM1 [Zea mays]
9.8e-105:542:94
Hs.10092:AI189282
R-MAMMA1002087
ESTs
4.0e-19:153:84
Hs.136678:AA730474
R-MAMMA1002095
ESTs
6.8e-34:196:93
Hs.48119:AA454227
R-MAMMA1002128
ESTs, Highly similar to ABC1 PROTEIN PRECURSOR [Saccharomyces cerevisiae]
9.0e-96:503:94
Hs.39088:AA194773
R-MAMMA1002142
ESTs
5.6e-21:145:90
Hs.62119:AA043299
R-MAMMA1002165
ESTs
1.6e-35:351:76
Hs.140413:N47721
R-MAMMA1002205
Homo sapiens DNA fragmentation factor 40 kDa subunit (DFF40) mRNA, complete cds
6.4e-42:217:79
Hs.133089:AF064019
R-MAMMA1002224
ESTs
0.50:170:64
Hs.144140:H04293
R-MAMMA1002234
R-MAMMA1002586
ESTs
5.0e-105:529:96
Hs.4814:AA631254
R-MAMMA1002633
ESTs
7.3e-97:470:98
Hs.38039:AI360128
R-MAMMA1003126
ESTs
6.1e-114:567:97
Hs.20733:AI417917
R-NT2RM4000100
ESTs
3.6e-71:343:99
Hs.92186:AI080282
R-NT2RM4000115
ESTs
1.5e-86:405:100
Hs.129151:AA988192
R-NT2RM4000198
ESTs
8.4e-83:462:93
Hs.96772:AI369496
R-NT2RM4000284
Human IgG Fc receptor hFcRn mRNA, complete cds
5.4e-95:440:100
Hs.110804:U12255
R-NT2RM4000295
ESTs
1.1e-112:544:97
Hs.21452:AA581881
R-NT2RM4000326
EST
4.0e-59:301:96
Hs.86264:AA206496
R-NT2RM4000417
ESTs
2.0e-88:489:93
Hs.29098:AA521439
R-NT2RM4000444
ESTs
6.4e-90:497:92
Hs.6129:U66676
R-NT2RM4000587
ESTs
1.0e-97:473:98
Hs.24947:AA039350
R-NT2RM4000593
ESTs
9.8e-109:554:95
Hs.7579:AA775865
R-NT2RM4000648
ESTs, Moderately similar to GLYPICAN-1 PRECURSOR [Homo sapiens]
7.6e-39:262:85
Hs.118407:AA001322
R-NT2RM4000761
ESTs
6.4e-86:433:95
Hs.153428:AI246519
R-NT2RM4000965
ESTs
2.8e-102:523:96
Hs.61790:AA421156
R-NT2RM4000997
R-NT2RM4001321
ESTs
2.4e-66:315:100
Hs.75425:AA149434
R-NT2RM4001325
ESTs
0.99:104:62
Hs.116257:AA628680
R-NT2RM4001377
Homo sapiens mRNA for KIAA0638 protein, partial cds
9.3e-113:553:96
Hs.77864:AB014538
R-NT2RM4001735
Homo sapiens clone 23904 mRNA sequence
1.5e-107:553:94
Hs.67364:AF052129
R-NT2RM4001768
EST
1. 6e-14:82:85
Hs.140922:R51520
R-NT2RM4001843
ESTs
2.1e-123:579:98
Hs.3741:AI057614
R-NT2RM4002352
Homo sapiens hLRp105 mRNA for LDL receptor related protein 105, complete cds
1.8e-109:557:95
Hs.143641:AB009462
R-NT2RP2000092
ESTs
3.3e-08:286:65
Hs.79881:AA401302
R-NT2RP2000178
ESTs, Weakly similar to MITOCHONDRIAL LON PROTEASE HOMOLOG PRECURSOR
[H.sapiens]
2.3e-95:462:98
Hs.47305:AA195153
R-NT2RP2000240
ESTs
1. 3e-55 : 272 : 98
Hs.125522:AI299693
R-NT2RP2000394
ESTs
2.4e-107:528:96
Hs. 28555:W55892
R-NT2RP2000447
ESTs, Moderately similar to dynamin, internal form 2, short C-terminal form [H.sapiens]
1.6e-67:357:94
Hs.128788:AA424076
R-NT2RP2000479
ESTs
2.6e-48:312:86
Hs.146811:AA410788
R-NT2RP2000514
EST
3.2e-63:348:93
Hs.44542:N33966
R-NT2RP2000533
ESTs
0.017:307:57
Hs.97873:AA402799
R-NT2RP2000616
ESTs
1.0e-91:475:95
Hs.50344:AI300539
R-NT2RP2000649
Homo sapiens mRNA for Hs Ste24p, complete cds
1.4e-66:322:98
Hs.25846:AB016068
R-NT2RP2000663
ESTs
8.2e-59:311:96
Hs.9728:T98746
R-NT2RP2000712
EST
1.7e-27:239:76
Hs.161561:W60681
R-NT2RP2000739
ESTs, Weakly similar to zinc finger protein [H. sapiens]
6.3e-86:462:93
Hs.13323:AA897542
R-NT2RP2000818
ESTs
7.3e-99:485:97
Hs.100525:AI310204
R-NT2RP2000903
H.sapiens 5T4 gene for 5T4 Oncofetal antigen
1.2e-100:505:96
Hs.82128:AJ012159
R-NT2RP2001200
Homo sapiens mRNA for KIAA0676 protein, partial cds
6.6e-59:306:95
Hs.115763:AB014576
R-NT2RP2001223
ESTs
1.2e-94:475:95
Hs.27556:AA115361
R-NT2RP2001276
ESTs, Moderately similar to regulatory protein [M.musculus]
4.7e-65:354:92
Hs.105547:AI361036
R-NT2RP2001388
ESTs
5.5e-83:459:93
Hs.15713:AA485755
R-NT2RP2001469
ESTs, Weakly similar to teg292 protein [M.musculus]
2.0e-30:233:83
Hs.68791:AA527270
R-NT2RP2001480
Homo sapiens thrombospondin 3 (THBS3) gene, complete cds
2.1e-84:426:95
Hs.82165:L38969
R-NT2RP2001495
ESTs, Weakly similar to transporter protein [H.sapiens]
1.7e-14:130:84
Hs.18272:N78499
R-NT2RP2001514
ESTs, Weakly similar to PROBABLE CATION-TRANSPORTING ATPASE YEL031W
[Saccharomyces cerevisiae]
3.3e-45:242:95
Hs.9275:AA973284
R-NT2RP2001538
EST
1.4e-05:111:73
Hs.137268:T39311
R-NT2RP2001562
EST
0.50:35:91
Hs.140505:AA804211
R-NT2RP2001662
Homo sapiens clone 24615 mRNA sequence
1.0e-95:485:95
Hs.94785:AF055012
R-NT2RP2001755
Homo sapiens mRNA for KIAA0762 protein, partial cds
4.2e-105:576:92
Hs.5378:AB018305
R-NT2RP2001769
ESTs
4.2e-102:548:93
Hs.14014:AA745592
R-NT2RP2001817
ESTs
6.0e-97:472:97
Hs.31176:AI037953
R-NT2RP2001878
ESTs
3.3e-94:475:95
Hs.144655:AI279798
R-NT2RP2001903
ESTs
1.7e-88:461:95
Hs.112218:AI038601
R-NT2RP2001915
ESTs
7.8e-96:480:96
Hs.100890:AA779892
R-NT2RP2001921
ESTs
2.5e-88:466:94
Hs.104859:AA779101
R-NT2RP2001948
ESTs
1.9e-81:439:91
Hs.105463:AA583017
R-NT2RP2001956
ESTs
8.7e-85:477:91
Hs.12101:AA677423
R-NT2RP2002015
ESTs
3.5e-85:431:95
Hs.75425:AA149434
R-NT2RP2002063
EST
0.0083:199:62
Hs.48699:N63049
R-NT2RP2002188
ESTs
1.5e-108:559:94
Hs.47320:AA057440
R-NT2RP2002232
ESTs
2.5e-113:576:95
Hs.7099:AI089774
R-nnnnnnnnnnnn
Human mRNA for KIAA0383 gene, partial cds
8.0e-102:511:96
Hs.27590:AB002381
R-NT2RP2002409
ESTs
3.2e-84:432:95
Hs.128443:AI281991
R-NT2RP2002510
ESTs
1.3e-42:303:82
Hs.146811:AA410788
R-NT2RP2002527
Thromboxane A2 receptor
2.9e-23:164:88
Hs.89887:038081
R-NT2RP2002533
Homo sapiens putative tumor suppressor gene 26 protein alpha 2 delta calcium channel subunit mRNA, complete cds
4.0e-117:580:96
Hs.127436:AF040709
R-NT2RP2002564
Human zinc-finger protein C2H2-150 mRNA, complete cds
4.0e-111:569:94
Hs.108139:U38864
R-NT2RP2002674
ESTs, Weakly similar to putative p150 [H.sapiens]
0. 010:293:60
Hs.140964:AI214400
R-NT2RP2002721
ESTs
5.6e-10:165:69
Hs.108745:H95644
R-NT2RP2002824
EST
0.0055:209:58
Hs.136259:AA347883
R-NT2RP2002942
ESTs
9.2e-82:422:96
Hs.140952:R59211
R-NT2RP2002974
ESTs
5.6e-99:507:96
Hs.43314:AA160543
R-NT2RP2002976
ESTs
2.9e-78:397:91
Hs.83575:N28730
R-NT2RP2003042
ESTs
2.7e-107:526:97
Hs.6770:AA972732
R-NT2RP2003179
ESTs
2.9e-59:335:92
Hs.87019:AA760977
R-NT2RP2003210
ESTs
2.1e-80:419:94
Hs.25354:N28667
R-NT2RP2003302
ESTs, Moderately similar to ZINC FINGER PROTEIN 7 [Homo sapiens]
2.1e-92:443:98
Hs.112508:AA599140
R-NT2RP2003369
ESTs
9.7e-84:462:92
Hs.155116:C16874
R-NT2RP2003383
Homo sapiens mRNA for KIAA0458 protein, complete cds 1.3e-112:549:97
Hs.7414:AB007927
R-NT2RP2003390
Homo sapiens SEC63 (SEC63) mRNA, complete cds
4.9e-56:286:96
Hs.31575:AF100141
R-NT2RP2003469
Human mRNA for KIAA0355 gene, complete cds
6.6e-40:302:83
Hs.153014:AB002353
R-NT2RP2003545
ESTs
8.0e-121:572:98
Hs.23643:AI299952
R-NT2RP2003593
EST
1.0:124:62
Hs.59890:AA001879
R-NT2RP2003599
EST
5.2e-06:319:60
Hs.147887:AI223203
R-NT2RP2003655
ESTs
9.3e-107:519:97
Hs.5831:AA176450
R-NT2RP2003664
Homo sapiens mRNA for leptin receptor gene-related protein
5.3e-112:549:96
Hs.23581:Y12670
R-NT2RP2003931
Human mRNA for KIAA0365 gene, partial cds
1. 7e-113:571 :96
Hs.84123:AB002363
R-NT2RP2003940
EST
3.0e-71:385:93
Hs.162657:AA603590
R-NT2RP2003950
Homo sapiens clone 24778 unknown mRNA
5.0e-98:494:95
Hs.25306:AF070572
R-NT2RP2004069
Human kpni repeat mrna (cdna clone pcd-kpni-8), 3' end
6.3e-54:556:74
Hs.103948:K00627
R-NT2RP2004108
ESTs, Moderately similar to ZINC FINGER PROTEIN ZFP-36 [Homo sapiens]
6.9e-92:442:98
Hs.14831:AI261191
R-NT2RP2004141
ESTs
8.3e-29:171:93
Hs.25700:AI338437
R-NT2RP2004179
ESTs
3.1e-71:461:88
Hs.6748:R68509
R-NT2RP2004205
ESTs
2.6e-44:397:78
Hs.95115:AA206594
R-NT2RP2004447
ESTs
4.0e-101:494:97
Hs.51655:AA523276
R-NT2RP2004495
ESTs, Weakly similar to transporter protein [H.sapiens]
6.1e-71:417:92.
Hs.18272:N78499
R-NT2RP2004524
ESTs
1.8e-93:482:95
Hs.119285:AI225008
R-NT2RP2004556
Homo sapiens mRNA for KIAA0459 protein, partial cds
8.8e-48:353:82
Hs.28169:AB007928
R-NT2RP2004606
Tissue inhibitor of metalloproteinase 1 (erythroid potentiating activity, collagenase inhibitor)
3.5e-116:576:96
Hs.148726 :X03124
R-NT2RP2004648
ESTs
5.9e-114:600:93
Hs.3741:AI057614
R-NT2RP2004670
ESTs
1.7e-92:488:94
Hs.6262:T89093
R-NT2RP2004794
EST
0.44:205:57
Hs.147759:AI220726
R-NT2RP2004837
ESTs
6.9e-111:576:94
Hs.12305:AA166889
R-NT2RP2004847
ESTs
8.3e-94:445:99
Hs.53996:AI268861
R-NT2RP2005027
GLUCOSE TRANSPORTER TYPE 3, BRAIN
2.5e-104:508:97
Hs.7594:M20681
R-NT2RP2005069
ESTs, Highly similar to vacuolar protein sorting homolog r-vps33b
[R. norveg i cus]
4.7e-111:541:97
Hs.26510:AA700425
R-NT2RP2005163
ESTs
6.8e-64:327:89
Hs.83575:N28730
R-NT2RP2005181
ESTs, Moderately similar to HIGH-AFFINITY CATIONIC AMINO ACID TRANSPORTER-1 [H.sapiens]
1.6e-106:527:97
Hs.86362:AA205485
R-NT2RP2005247
MHC class II transactivator
7.9e-35:465:69
Hs.3076:U18259
R-NT2RP2005378
ESTs
3.4e-110:566:94
Hs.23060:N64748
R-NT2RP2005391
ESTs
5.5e-82:463:92
Hs.118793:AA192438
R-NT2RP2005425
Homo sapiens mRNA for KIAA0803 protein, partial cds
2.6e-101:526:94
Hs.58103:AB018346
R-NT2RP2005463
ESTs, Weakly similar to weakly similar to S. cervisiae PTM1 precursor [C.elegans]
7.6e-111:554:97
Hs.16492:N95400
R-NT2RP2005514
ESTs
1.8e-97:490:95
Hs.109677:AA447864
R-NT2RP2005535
EST
5.1e-40:399:73
Hs.127142:AA937570
R-NT2RP2005541
ESTs
5.2e-114:573:96
Hs.70823:AI378619
R-NT2RP2005597
ESTs, Weakly similar to rotated abdomen protein [D.melanogaster]
3.7e-109:543:96
Hs.99654:AA670164
R-nnnnnnnnnnnn
ESTs
1.1e-100:501:97
Hs.112011:AA987961
R-NT2RP2005666
ESTs
2.7e-106:560:94
Hs.42814:AA205754
R-NT2RP2005774
Homo sapiens apoptosis-related mRNA, 3'UTR, partial sequence
7.0e-96:440:96
Hs.139345:AF035364
R-NT2RP2005878
ESTs
2.8e-89:479:93
Hs.142305:R42591
R-NT2RP2005883
ESTs
1.0e-85:431:96
Hs.6909:AA928115
R-NT2RP2005887
ESTs
5.5e-109:566:94
Hs.12305:AA166889
R-nnnnnnnnnnnn
Paired box homeotic gene 6 (aniridia, keratitis)
1.6e-116:578:96
Hs.89506:M93650
R-NT2RP2005994
EST
0.0061:129:68
Hs.160756:AI310589
R-NT2RP2006004
ESTs, Weakly similar to KIAA0405 [H. sapiens]
4.7e-45:303:86
Hs.14146:W92235
R-NT2RP2006042
EST
0. 64:84:71
Hs.133275:AI053487
R-NT2RP2006092
ESTs, Weakly similar to HYPOTHETICAL PROTEIN KIAA0168 [H.sapiens]
1.1e-75:384:95
Hs.32822:AI194045
R-NT2RP2006099
ESTs
6.9e-35:224:82
Hs.139446:AA461080
R-NT2RP2006134
EST
1.3e-95:445:100
Hs.162033:AA514590
R-NT2RP2006269
Human mRNA for KIAA0315 gene, partial cds
0.96:343:60
Hs.3989:AB002313
R-NT2RP2006512
Homo sapiens clone 23904 mRNA sequence
1.5e-107:531:96
Hs.67364:AF052129
R-NT2RP3000011
ESTs
7.3e-92:508:91
Hs.112041:W26001
R-NT2RP3000022
EST
0.78:175:63
Hs.135650:AA902912
R-NT2RP3000059
ESTs
6.2e-99:475:98
Hs.123136:AA631067
R-NT2RP3000063
ESTs
9.7e-105:554:94
Hs.7542:AA121663
R-nnnnnnnnnnnn
Human mRNA for KIAA0314 gene, partial cds
5.0e-17:307:65
Hs.155045:AB002312
R-NT2RP3000148
ESTs
6.4e-101:527:94
Hs.58461:W80378
R-NT2RP3000169
Homo sapiens MRS1 mRNA, complete cds
1.4e-111:551:96
Hs.30985:AF093239
R-NT2RP3000171
EST
0.45:205:57
Hs.147759:AI220726
R-NT2RP3000172
ESTs
2.0e-89:494:93
Hs.6262:T89093
R-NT2RP3000201
Human mRNA for KIAA0355 gene, complete cds
1.1e-40:305:83
Hs.153014:AB002353
R-NT2RP3000232
ESTs, Weakly similar to kruppel-related zinc finger protein [H.sapiens]
5.7e-65:386:90
Hs.4841:AI279875
R-NT2RP3000304
Homo sapiens LDL receptor-related protein 6 (LRP6) mRNA, complete cds
1.1e-109:541:97
Hs.23672:AF074264
R-NT2RP3000378
EST
2.0e-05:112:74
Hs.137268:T39311
R-NT2RP3000436
EST
1.2e-08:347:62
Hs.158830:AI377454
R-NT2RP3000444
ESTs
3.3e-70:314:99
Hs.57973:AI263207
R-NT2RP3000460
EST
1.9e-50:294:92
Hs.7260:T23737
R-NT2RP3000481
PROBABLE G PROTEIN-COUPLED RECEPTOR GPR6
1.0:183:59
Hs.46332:U18549
R-NT2RP3000616
ESTs
3.0e-71:309:93
Hs.41296:N71923
R-NT2RP3000645
ESTs
1.5e-101:550:92
Hs.21910:AA020743
R-NT2RP3000652
ESTs
6.6e-88:411:100
Hs.43134:AA766138
R-NT2RP3000676
Homo sapiens mRNA for KIAA0446 protein, complete cds
1.0e-104:542:94
Hs.158286:AB007915
R-NT2RP3000677
ESTs
0.33:307:59
Hs.133022:AI374739
R-NT2RP3000721
ESTs
1.6e-75:390:90
Hs.83575:N28730
R-NT2RP3000789
ESTs
1.5e-71:340:99
Hs.37776:H93038
R-NT2RP3000818
ESTs
1.9e-52:330:88
Hs.111052:H80504
R-NT2RP3000820
EST
9.1e-05:82:74
Hs.124352:AA830406
R-NT2RP3000838
Homo sapiens mRNA for KIAA0638 protein, partial cds
1.5e-100:522:94
Hs.77864:AB014538
R-NT2RP3000871
ESTs
3.9e-76:424:91
Hs.121642:AA772262
R-NT2RP3000907
ESTs, Weakly similar to PROBABLE CATION-TRANSPORTING ATPASE YEL031W
[Saccharomyces cerevisiae]
4.5e-95:493:94
Hs.9275:AA973284
R-NT2RP3000921
ESTs
4.1e-52:283:94
Hs.49714:AA442453
R-NT2RP3001012
Homo sapiens mRNA for CMP-sialic acid transporter, complete cds
0.60:250:61
Hs.82921:D87969
R-NT2RP3001044
ESTs
3.5e-106:547:95
Hs.12305:AA166889
R-NT2RP3001061
ESTs
1.3e-96:453:99
Hs.4916:AI149707
R-NT2RP3001159
ESTs, Weakly similar to T13F2. 1 [C.elegans]
3.8e-47:302:90
Hs.6281:AA523081
R-NT2RP3001170
Homo sapiens mRNA for KIAA0784 protein, partial cds
2.8e-118:561:98
Hs.3657:AB018327
R-NT2RP3001195
ESTs
1.5e-40:461:72
Hs.152438:AI334078
R-NT2RP3001240
EST
1.9e-50:294:92
Hs.7260:T23737
R-NT2RP3001271
ESTs
1.1e-77:432:92
Hs.12211:AA908631
R-NT2RP3001322
ESTs
0.25:331:60
Hs.44330:N32264
R-NT2RP3001542
EST
0.0032:432:58
Hs.148107:AA693476
R-NT2RP3001560
EST
3.5e-50:281:93
Hs.101727:H16171
R-NT2RP3001592
ESTs
3.2e-65:344:93
Hs.28964:AA715101
R-NT2RP3001685
EST
3.0e-37:165:81
Hs.160895:AI365871
R-NT2RP3001738
ESTs, Weakly similar to T13F2.1 [C.elegans]
3.8e-47:302:90
Hs.6281:AA523081
R-NT2RP3001754
EST
0.00043:104:69
Hs.148331:AA911426
R-NT2RP3001858
ESTs
7.6e-93:502:93
Hs.153524:AA533582
R-NT2RP3001976
ESTs
5.0e-104:516:96
Hs.35461:AA777644
R-NT2RP3002015
R-NT2RP3002160
ESTs
1.4e-34:178:99
Hs.130783:AI263114
R-NT2RP3002281
Homo sapiens mRNA for KIAA0765 protein, partial cds
3.5e-83:446:93
Hs.62318:AB018308
R-NT2RP3002286
ESTs
2.1e-103:513:97
Hs.58570:AA521423
R-NT2RP3002311
ESTs
1.4e-108:538:96
Hs.3741:AI057614
R-NT2RP3002324
ESTs
3.7e-102:483:99
Hs.99668:AA829521
R-NT2RP3002342
ESTs, Weakly similar to transporter protein [H.sapiens]
2.0e-60:339:95
Hs.18272:N78499
R-NT2RP3002353
ESTs
6.8e-98:484:97
Hs.9732:AA527784
NNNNNNNNNNNNNN
Homo sapiens mRNA for KIAA0788 protein, partial cds
2.7e-29:214:85
Hs.2397:Z70200
NNNNNNNNNNNNNN
ESTs
3.0e-72:389:94
Hs.32246:AA464020
R-NT2RP3002448
ESTs, Weakly similar to Y48E1B.t [C.elegans]
1.0e-19:131:75
Hs.8715:H58021
R-NT2RP3002571
ESTs
1.1e-78:407:95
Hs.27356:AA740928
R-NT2RP3002664
ESTs
1.2e-56:341:90
Hs.23308:AA115020
R-NT2RP3002721
EST
2.8e-41:302:82
Hs.124936:AA825548
R-NT2RP3002737
EST
1.7e-51:267:97
Hs.161348:AI422470
R-NT2RP3002738
ESTs, Weakly similar to enhancer of filmentation 1 [H.sapiens]
1.7e-106:530:96
Hs.4894:AI191323
R-NT2RP3002790
R-NT2RP3002836
ESTs
4.6e-49:282:92
Hs.107979:AA146994
R-NT2RP3002887
ESTs
6.3e-98:516:94
Hs.11900:AA535065
R-NT2RP3002900
ESTs
2.0e-29:155:99
Hs.153329:AA112325
R-NT2RP3002958
Homo sapiens clone 23851 mRNA sequence
6.6e-119:575:98
Hs.10065:AF035313
R-NT2RP3002983
ESTs
1. 1e-61:374:90
Hs.17834:AA128246
R-NT2RP3003000
Homo sapiens T-type calcium channel alpha-1 subunit mRNA, complete cds
4.1e-65:358:94
Hs.122359:AF051946
R-NT2RP3003076
ESTs
2.6e-95:507:93
Hs.21910:AA020743
R-NT2RP3003354
ESTs, Moderately similar to !!!! ALU SUBFAMILY SQ WARNING ENTRY !!!! [H.sapiens]
2.1e-78:385:96
Hs.92177:AI207792
R-NT2RP3003448
ESTs
6.7e-105:521:96
Hs.106833:AA470128
R-NT2RP3003469
ESTs
1. 1e-91:461:96
Hs.75425:AA149434
R-NT2RP3003473
R-NT2RP3003527
Homo sapiens mRNA for protein kinase DyrklB
1.6e-92:445:97
Hs.130988:Y17999
R-NT2RP3003532
ESTs
0.022:193:63
Hs.122593:Z99400
R-nnnnnnnnnnnn
EST
0.036:279:59
Hs.158745:AI375513
R-NT2RP3003559
ESTs
9.8e-106:513:97
Hs.44970:AI061464
R-NT2RP3003614
Homo sapiens mRNA, chromosome 1 specific transcript KIAA0510
0.00016:113:69
Hs.92660:AB007979
R-NT2RP3003729
ESTs
1.2e-43:289:86
Hs.106401:R50967
R-NT2RP3003849
ESTs
5.4e-91:435:98
Hs.144840:AI221746
R-NT2RP3003874
ESTs
0.21:323:59
Hs.42919:AA805764
R-NT2RP3003963
ESTs
1.7e-90:438:97
Hs.105894:AA564110
R-NT2RP3004000
ESTs
2.9e-101:559:91
Hs.21910:AA020743
R-NT2RP3004025
ESTs
2.3e-108:517:98
Hs.15356:AA911109
R-NT2RP3004075
ESTs
7.4e-84:453:93
Hs.22412:AA523036
R-NT2RP3004083
ESTs, Weakly similar to R06B9.b [C.elegans]
4.2e-84:474:91
Hs.30432:W28988
R-NT2RP3004090
ESTs
1.0:207:61
Hs.92832:AA631027
R-NT2RP3004119
EST
1.8e-50:248:99
Hs.162023:AA506128
R-NT2RP3004130
ESTs
1.1e-103:520:96
Hs.10491:W28968
R-NT2RP3004133
ESTs
4.7e-104:545:93
Hs.15727:H98190
R-NT2RP3004202
ESTs
1.1e-98:471:98
Hs.61884:AI335985
R-NT2RP3004294
ESTs, Weakly similar to R06B9.b [C.elegans]
2.4e-96:500:94
Hs.30432:W28988
R-NT2RP3004309
ESTs, Weakly similar to T13F2.1 [C.elegans]
3.5e-48:308:90
Hs.6281:AA523081
R-NT2RP3004321
ESTs
2.6e-99:494:97
Hs.19306:N53491
R-NT2RP3004345
ESTs
5.4e-95:444:99
Hs.107149:AI379497
R-NT2RP3004355
ESTs
3.9e-99:490:97
Hs.43410:N23651
R-NT2RP3004374
ESTs
1.2e-90:462:95
Hs.75425:AA149434
R-NT2RP3004406
ESTs
1.9e-100:502:96
Hs.24936:AA479402
R-NT2RP3004481
ESTs
1.6e-53:370:87
Hs.11953:AA194120
R-NT2RP3004552
ESTs, Weakly similar to gene SEZ-6 [M.musculus]
7.8e-92:488:93
Hs.6314:AA522619
R-NT2RP3004625
Homo sapiens I-1 receptor candidate protein mRNA, complete cds
2.6e-50:352:84
Hs.26285:AF082516
R-NT2RP3004640
ESTs
1.1e-105:551:94
Hs.83348:AA527170
R-NT2RP3004647
Homo sapiens mRNA for KIAA0446 protein, complete cds
4.9e-111:555:96
Hs.158286:AB007915
R-NT2RP4000108
ESTs
2.9e-94:479:96
Hs.6625:AA115182
R-NT2RP4000634
ESTs
3.0e-120:572:98
Hs.28827:AI125541
R-NT2RP4000962
ESTs, Weakly similar to SPORULATION-SPECIFIC PROTEIN 1 [Saccharomyces cerevisiae]
6.0e-17:98:98
Hs.4789:AI418298
R-NT2RP4001001
ESTs
3.1e-117:567:97
Hs.4931:AA523860
R-NT2RP4001009
Homo sapiens mRNA for Hs Ste24p, complete cds
1.6e-83:404:98
Hs.25846:AB016068
R-NT2RP4001467
5' nucleotidase (CD73)
5.9e-113:545:97
Hs.153952:X55740
R-NT2RP4001877
ESTs, Highly similar to GONADOTROPIN-RELEASING HORMONE RECEPTOR [Rattus norvegicus]
2.2e-67:375:93
Hs.16389:AA206356
R-NT2RP4001879
R-NT2RP4002187
EST
0.010:117:70
Hs.160416:AI394161
R-NT2RP4002451
EST
1.3e-62:386:87
Hs.57082:H25761
R-NT2RP4002715
ESTs
6.9e-111:552:96
Hs.12305:AA166889
R-NT2RP4002750
ESTs, Moderately similar to HIGH-AFFINITY CATIONIC AMINO ACID TRANSPORTER-1 [H. sapiens]
7.0e-109:532:97
Hs.86362:AA205485
R-OVARC1000003
ESTs
1.3e-74:391:95
Hs.105039:AA477819
R-OVARC1000090
Homo sapiens neuronal thread protein AD7c-NTP mRNA, complete cds
9.9e-44:471:75
Hs.129735:AF010144
R-OVARC1000105
60S RIBOSOMAL PROTEIN L38
8.8e-14:83:100
Hs.2017:Z26876
R-OVARC1000137
ESTs
3.0e-84:387:95
Hs.22028:AA167715
R-OVARC1000208
Human mRNA for KIAA0392 gene, partial cds
2.8e-51:313:89
Hs.40100:AB002390
R-OVARC1000255
Spleen tyrosine kinase
2. 8e-106:510:98
Hs.74101:L28824
R-OVARC1000275
ESTs, Highly similar to PROBABLE PHOSPHATIDYLINOSITOL-4-PHOSPHATE 5-KINASE FAB1 [Saccharomyces cerevisiae]
6.9e-105:556:94
Hs.5748:AA608559
R-OVARC1000298
ESTs, Weakly similar to T03G11.6 gene product [C.elegans]
2.4e-56:338:90
Hs.108354:W19984
R-OVARC1000307
ESTs
2.4e-101:563:93
Hs.24479:N25972
R-OVARC1000313
Homo sapiens mRNA for KIAA0573 protein, partial cds
5.0e-98:534:93
Hs.154023:AB011145
R-OVARC1000331
Homo sapiens chromosome 9, P1 clone 11659
1.0e-55:281:97
Hs.3439:AC004472
R-OVARC1000410
Homo sapiens clone 23767 and 23782 mRNA sequences
3.3e-90:462:94
Hs.8025:AF007150
R-OVARC1000439
ESTs, Highly similar to HYPOTHETICAL 64.3 KD GTP-BINDING PROTEIN C02F5.3 IN CHROMOSOME III [Caenorhabditis elegans]
1.6e-99:510:95
Hs.7471:AI143226
R-OVARC1000467
R-OVARC1000529
ESTs
5.7e-93:461:96
Hs.21396:AA114834
R-OVARC1000553
ESTs
4.3e-51:351:87
Hs.42979:W31096
R-OVARC1000775
R-OVARC1000811
ESTs
1.3e-82:441:95
Hs.73452:AA581386
R-OVARC1000853
ESTs, Weakly similar to !!!! ALU SUBFAMILY J WARNING ENTRY !!!! [H.sapiens]
3.1e-95:492:95
Hs.6853:AA401703
R-OVARC1000873
ESTs
2.4e-46:281:91
Hs.43857:R91358
R-OVARC1000916
H.sapiens PISSLRE mRNA
1.9e-112:588:94
Hs.77313:X78342
R-OVARC1000956
Homo sapiens mRNA for MDC3, complete cds
0. 18:259:62
Hs.7164:AB009672
R-OVARC1000995
EST
6.6e-43:343:81
Hs.149580:AI281881
R-OVARC1001030
ESTs, Weakly similar to neuroendocrine-specific protein C [H.sapiens]
1. 5e-21:116:100
Hs.65450:AA055913
R-OVARC1001049
ESTs
1.2e-70:369:95
Hs.42949:N21131
R-OVARC1001086
Homo sapiens cyclin T2a mRNA, complete cds
1.3e-106:569:94
Hs.155478:AF048731
R-OVARC1001132
INITIATION FACTOR IF-2, MITOCHONDRIAL PRECURSOR
0.16:170:64
Hs.149894:L34600
R-OVARC1001163
ESTs
1.9e-39:219:94
Hs.126067:AI344351
R-OVARC1001222
ESTs
0.62:177:63
Hs.141162:H66213
R-OVARC1001260
ESTs
2.1e-79:425:94
Hs.105039:AA477819
R-OVARC1001336
ESTs
9.2e-75:439:91
Hs.105039:AA477819
R-OVARC1001338
ESTs
2.3e-19:139:92
Hs.7978:W05059
R-OVARC1001569
ESTs
2.4e-83:412:97
Hs.21396:AA114834
R-OVARC1001570
ESTs
2.6e-49:280:94
Hs.3854:R12478
R-OVARC1001596
EST
8.2e-15:93:97
Hs.136918:AA811543
R-OVARC1001607
ESTs
0.019:413:56
Hs.24684:AA587245
R-OVARC1001725
ESTs
1.4e-96:504:95
Hs.23754:N29716
R-OVARC1001727
R-OVARC1001807
Hormone receptor (growth factor-inducible nuclear protein N10)
8.5e-78:425:94
Hs.1119:D49728
R-OVARC1001833
ESTs
1.0e-63:325:96
Hs.126912:AA469087
R-OVARC1001991
ESTs
1.3e-92:467:95
Hs.26506:AI348000
R-OVARC1002058
ESTs
2.5e-89:512:91
Hs.58093:W63576
R-OVARC1002178
ESTs
3.3e-99:487:96
Hs.136527:AI419398
R-PLACE1000033
ESTs
0. 012:202:59
Hs.157400:AI370528
R-PLACE1000231
ESTs
2.9e-56:285:96
Hs.36688:AA603479
R-PLACE1000258
EST
3.4e-50:353:83
Hs.146794:AI149478
R-PLACE1000442
ESTs, Moderately similar to ZINC FINGER PROTEIN ZFP-36 [Homo sapiens]
5.5e-91:437:98
Hs.14831:AI261191
R-PLACE1000560
ESTs
3.7e-60:317:94
Hs.65713:AI269328
R-PLACE1000740
ESTs
4.2e-67:362:94
Hs.163434:T79849
R-PLACE1000912
ESTs
3.4e-57:329:92
Hs.121907:R66773
R-PLACE1000914
ESTs
2.6e-71:419:89
Hs.90809:AA010979
R-PLACE1000927
ESTs, Weakly similar to N-methyl-D-aspartate receptor-associated protein [D.melanogaster]
7.8e-111:545:97
Hs.8661:AI189791
R-PLACE1000986
ESTs
1.5e-91:431:99
Hs.42458:AA452296
R-PLACE1001016
ESTs
3.4e-45:231:97
Hs.121013:AA324765
R-PLACE1001100
Homo sapiens nephrin (NPHS1) mRNA, complete cds
3.5e-43:321:83
Hs.128834:AF035835
R-PLACE1001114
Human clone 23732 mRNA, partial cds
1.6e-42:305:83
Hs.81281:U79258
R-PLACE1001123
ESTs, Highly similar to UBIQUITIN-CONJUGATING ENZYME E2-28.4 KD
[Saccharomyces cerevisiae]
1. 2e-51 :310:90
Hs.7773:AA127629
R-PLACE1001183
Human mRNA for KIAA0308 gene, partial cds
0.88:182:65
Hs.10351:AB002306
R-PLACE1001229
ESTs
5.2e-90:471:95
Hs.18271:N92774
R-PLACE1001231
R-PLACE1001340
Homo sapiens mRNA for KIAA0719 protein, complete cds
6.6e-53:265:98
Hs.21198:AB018262
R-PLACE1001401
ESTs
1.9e-72:362:96
Hs.20161:AA056410
R-PLACE1001407
ESTs
2.1e-36:249:85
Hs.23579:W38893
R-PLACE1001464
5' nucleotidase (CD73)
1.0e-91:457:96
Hs.153952:X55740
R-PLACE1001500
ESTs, Weakly similar to DNA helicase Q1 [H.sapiens]
2.0e-19:150:87
Hs.154199:AA155882
R-PLACE1001516
EST
1.9e-11:109:82
Hs.137486:AA425225
R-PLACE1001536
Human BRCA2 region, mRNA sequence CG016
0. 28:146:63
Hs.112434:U50529
R-PLACE1001564
ESTs
6.3e-14:109:88
Hs.26519:AA442703
R-PLACE1001655
Homo sapiens Shab-related delayed-rectifier K+ channel alpha subunit (KCNS3) mRNA, complete cds
1. 2e-118:578:97
Hs.47584:AF043472
R-PLACE1001788
ESTs
8.4e-38:205:95
Hs.23800:AA524095
R-PLACE1001795
ESTs, Weakly similar to HYPOTHETICAL 30.6 KD PROTEIN IN SCP160-MRPL8
INTERGENIC REGION PRECURSOR [S.cerevisiae]
2.5e-77:392:96
Hs.7745:H92988
R-PLACE1001836
ESTs
1.5e-49:296:90
Hs.17691:H60366
R-PLACE1001918
ESTs, Weakly similar to multispanning membrane protein [H.sapiens]
2.0e-42:304:85
Hs.110439:N93209
R-PLACE1001949
R-PLACE1002080
Small inducible cytokine A5 (RANTES)
8.5e-41:296:82
Hs.155464:AF088219
R-PLACE1002095
ESTs
8.5e-25:227:81
Hs.110488:AA034235
R-PLACE1002153
Homo sapiens TACC2 protein (TACC2) mRNA, partial cds
1. 5e-101 :514:95
Hs.90415:AF095791
R-PLACE1002329
ESTs
8.7e-48:257:94
Hs.126062:AA411593
R-PLACE1002355
ESTs
7.7e-71:362:95
Hs.120866:AI076780
R-PLACE1002374
Cathepsin L
8.4e-103:501:97
Hs.78056:X12451
R-PLACE1002518
ESTs
6.9e-97:471:97
Hs.104893:AA576941
R-PLACE1002547
Homo sapiens mRNA for KIAA0719 protein, complete cds
6.5e-55:276:97
Hs.21198:AB018262
R-PLACE1002726
Human DNA-binding protein ABP/ZF mRNA, complete cds
3.8e-39:212:94
Hs.86185:U82613
R-PLACE1002905
Homo sapiens mRNA for KIAA0563 protein, complete cds
2.9e-41:330:81
Hs.15731:AB011135
R-PLACE1002911
R-PLACE1002967
ESTs
1.0e-43:384:77
Hs.132722:AA618531
R-PLACE1003135
ESTs
8.2e-94:462:97
Hs.23643:AI299952
R-PLACE1003163
Homo sapiens DBI-related protein mRNA, complete cds
3.5e-110:541:96
Hs.15250:AF069301
R-PLACE1003407
Homo sapiens putative transmembrane protein (CLN5) mRNA, complete cds
5.5e-49:287:91
Hs.30213:AF068227
R-PLACE1003428
ESTs, Moderately similar to BIOTINIDASE PRECURSOR [Homo sapiens]
6.8e-83:406:97
Hs.17586:AA461448
R-PLACE1003438
ESTs
2.9e-83:463:92
Hs.11067:H30385
R-PLACE1003460
ESTs
7.0e-27:187:87
Hs.18763:H56292
R-nnnnnnnnnnnn
ESTs
1. 7e-52: 265: 97
Hs.114049:AI091839
R-PLACE1003573
Human mRNA for KIAA0160 gene, partial cds
0.13:102:69
Hs.79880:063881
R-PLACE1003598
ESTs
8.0e-39:210:95
Hs.26286:AA040823
R-PLACE1003644
EST
0.47:84:73
Hs.105856:AA551478
R-PLACE1003737
ESTs
1.1e-77:366:100
Hs.62699:AA707766
R-PLACE1003772
Human mRNA for KIAA0355 gene, complete cds
6.1e-27:551:65
Hs.153014:AB002353
R-PLACE1003839
ESTs
0.019:244:59
Hs.137825:AA778400
R-PLACE1003845
EST
5.3e-79:416:93
Hs.150153:AI300555
R-PLACE1003852
Homo sapiens mRNA for KIAA0758 protein, partial cds
2.2e-87:439:96
Hs.22039:AB018301
R-PLACE1004028
Sialyltransferase 4A (beta-galactosidase alpha-2,3-sialytransferase)
0. 73:128:71
Hs.60617:L13972
R-PLACE1004078
ESTs
1.7e-69:353:96
Hs.142075:AA654529
R-PLACE1004166
ESTs
1.7e-64:362:92
Hs.10177:AA191619
R-nnnnnnnnnnnn
EST
0.98:59:71
Hs.132255:AI027216
R-PLACE1004199
ESTs
1.3e-55:279:97
Hs.147585:AI217699
R-PLACE1004279
ESTs
3.7e-68:373:93
Hs.145531:H87181
R-PLACE1004282
R-PLACE1004305
Homo sapiens mRNA for KIAA0740 protein, complete cds
6.4e-79:377:99
Hs.15099:AB018283
R-PLACE1004441
ESTs
1.8e-46:244:95
Hs.107082:R63714
R-PLACE1004450
R-PLACE1004482
ESTs
1.2e-92:491:93
Hs.17840:AI269915
R-PLACE1004492
ESTs
6.1e-54:278:95
Hs.55862:AI341676
R-PLACE1004519
ESTs
3.1e-25:133:100
Hs.47378:AI193598
R-PLACE1004520
Pregnancy-specific beta-1 glycoprotein 4
2.8e-66:390:89
Hs.108936:X17097
R-PLACE1004630
ESTs
7.3e-58:338:92
Hs.155506:AI281549
R-PLACE1004637
ESTs
1.1e-37:309:82
Hs.20102:AA150165
R-PLACE1004648
ESTs
2.3e-67:340:96
Hs.69321:AA633240
R-PLACE1004816
Homo sapiens mRNA for Hakata antigen, complete cds
1.8e-104:586:90
Hs.9225:D88587
R-PLACE1004887
ESTs, Weakly similar to GOLIATH PROTEIN [D.melanogaster]
2.6e-30:222:86
Hs.18557:AA203416
R-PLACE1005003
ESTs
0.99:123:68
Hs.146244:AI276718
R-PLACE1005005
Homo sapiens ubiquitin conjugating enzyme G2 (UBE2G2) mRNA, complete cds
6.8e-58:299:95
Hs.151614:AF032456
R-PLACE1005031
ESTs
4.7e-57:325:92
Hs.31196:H13265
R-PLACE1005239
Homo sapiens mRNA for HIRIP3 protein, clone pH4-17
1.4e-86:450:93
Hs.26484:AJ223351
R-PLACE1005250
ESTs, Moderately similar to maternal transcript Maid [M.musculus]
1.7e-106:521:97
Hs.36794:AI038407
R-PLACE1005383
Homo sapiens UP50 mRNA, complete cds
6.3e-79:471:88
Hs.11494:AF093118
R-PLACE1005410
EST
2.3e-49:296:90
Hs.7260:T23737
R-PLACE1005426
Pregnancy-specific beta-1 glycoprotein 4
8.0e-109:576:93
Hs.108936:X17097
R-PLACE1005519
ESTs
5.4e-108:569:93
Hs.23643:AI299952
R-PLACE1005539
ESTs, Weakly similar to p20 protein [R.norvegicus]
4.5e-05:107:77
Hs.56874:W61026
R-PLACE1005544
ESTs
4.2e-57:280:98
Hs.155391:AA451633
R-PLACE1005569
ESTs
2.7e-90:470:94
Hs.8904:AI129815
R-PLACE1005601
R-PLACE1005660
ESTs, Highly similar to HYPOTHETICAL 64.3 KD GTP-BINDING PROTEIN C02F5.3 IN CHROMOSOME III [Caenorhabditis elegans]
1.4e-91:483:93
Hs.7471:AI143226
R-PLACE1005669
ESTs
1.7e-84:438:95
Hs. 18271 :N92774
R-PLACE1005682
ESTs
6.3e-80:482:88
Hs.128679:AI160081
R-PLACE1005725
ESTs
1.5e-98:519:93
Hs.11360:AI147467
R-PLACE1005736
ESTs
3.1e-110:561:95
Hs.24111:AI346026
R-PLACE1005745
ESTs
2.4e-96:473:97
Hs.126935:AA603217
R-PLACE1005768
Cytochrome P450, subfamily I (aromatic compound-inducible), polypeptide 2
4.0e-46:387:77
Hs. 1361:M55053
R-PLACE1005815
Homo sapiens PYRIN (MEFV) mRNA, complete cds
7.1e-56:324:79
Hs.113283:AF018080
R-PLACE1005878
ESTs
3.1e-75:388:94
Hs.153483:AA569128
R-PLACE1005927
ESTs
4.3e-64:403:87
Hs.126899:N50907
R-PLACE1006071
ESTs
5.3e-96:510:93
Hs.24398:AI262946
R-PLACE1006073
Homo sapiens mRNA for glucuronyltransferase I, complete cds
3.0e-97:504:93
Hs.26492:AB009598
R-PLACE1006079
ESTs
3.1e-79:453:90
Hs.134194:AI142137
R-PLACE1006093
ESTs
1.3e-78:378:98
Hs.129327:AI201040
R-nnnnnnnnnnnn
R-PLACE1006219
EST
1.6e-75:412:92
Hs.150153:AI300555
R-PLACE1006277
ESTs
2.8e-92:493:93
Hs.8904:AI129815
R-PLACE1006290
ESTs
2.8e-92:433:99
Hs.23445:AA489015
R-PLACE1006443
ESTs
2.5e-73:419:91
Hs.90809:AA010979
R-PLACE1006515
Homo sapiens mRNA for KIAA0576 protein, partial cds
6.9e-78:413:94
Hs.14687:AB011148
R-PLACE1006716
ESTs
4.8e-44:262:88
Hs.8503:AI393886
R-PLACE1006786
ESTs
6.3e-89:431:98
Hs.42458:AA452296
R-PLACE1006809
ESTs
1. 6e-68: 377: 92
Hs.8956:AI146421
R-PLACE1006959
EST
0.00065:211:63
Hs.136605:AA665784
R-PLACE1007028
ESTs
7.4e-92:475:94
Hs.110222:AA532444
R-PLACE1007040
ESTs
5.1e-103:509:97
Hs.71190:AA524036
R-PLACE1007077
ESTs
1.0e-98:529:93
Hs.24398:AI262946
R-PLACE1007081
Human growth factor independence-1 (Gfi-1) mRNA, complete cds
0.57:238:61
Hs.73172:U67369
R-PLACE1007096
ESTs
1.2e-88:466:94
Hs.8268:N70144
R-PLACE1007296
EST
4.3e-53:338:86
Hs.147274:AI206582
R-PLACE1007591
EST
4.6e-76:384:97
Hs.94445:N90719
R-PLACE1007626
Homo sapiens unknown mRNA, complete cds
5.0e-30:179:91
Hs.11441:AF047439
R-PLACE1007702
ESTs
1.0e-52:341:87
Hs.103382:AA026923
R-PLACE1007845
ESTs
2.2e-102:541:93
Hs.15727:H98190
R-PLACE1007881
ESTs
4.1e-75:398:93
Hs.55560:AI142804
R-PLACE1007971
ESTs
2.8e-43:304:85
Hs.82933:AA058963
R-PLACE1008282
ESTs, Highly similar to HEME-REGULATED EUKARYOTIC INITIATION FACTOR EIF-2-ALPHA KINASE [Oryctolagus cuniculus]
2.2e-74:393:94
Hs.77613:AI367385
R-PLACE1008297
ESTs
6.5e-101:506:96
Hs.44274:AA523749
R-PLACE1008359
ESTs
1.8e-94:469:96
Hs.160551:AI281417
R-PLACE1008469
ESTs
7.0e-74:421:90
Hs.90809:AA010979
R-PLACE1008549
ESTs
2.0e-81:474:90
Hs.11713:T65960
R-PLACE1008657
ESTs
9.5e-89:512:89
Hs.142075:AA654529
R-PLACE1008716
Human beta-1,2-N-acetylglucosaminyltransferase II (MGAT2) gene, complete cds
5.6e-100:504:95
Hs.154844:U15128
R-PLACE1008744
ESTs, Highly similar to COMPLEMENT RECEPTOR TYPE 2 PRECURSOR [Homo sapiens] 2.3e-107:528:96
Hs.8963:AI379350
R-PLACE1008984
ESTs
2.0e-76:464:89
Hs.40094:D12041
R-PLACE1008985
EST, Highly similar to SYNAPTOTAGMIN B [Discopyge ommata]
2.2e-59:343:90
Hs.161031:H72014
R-PLACE1009067
ESTs
7.7e-90:503:92
Hs.55067:AA037664
R-PLACE1009196
EST
0.011:243:60
Hs.149839:AI287601
R-PLACE1009279
Homo sapiens mRNA for serin protease with IGF-binding motif, complete cds
5.4e-27:553:62
Hs.75111:D87258
R-PLACE1009527
Human DNA-binding protein ABP/ZF mRNA, complete cds
2.7e-92:497:91
Hs.86185:U82613
R-PLACE1009546
ESTs
5.9e-80:461:90
Hs.134292:AA603031
R-PLACE1009600
ESTs
5.5e-98:509:93
Hs.21015:AA428288
R-PLACE1009735
ESTs
1.1e-85:462:93
Hs.48563:AA526595
R-nnnnnnnnnnnn
ESTs
6.8e-82:499:87
Hs.43498:AA570507
R-PLACE1010011
ESTs, Moderately similar to synaptonemal complex protein [M.musculus]
2.7e-15:171:78
Hs.31655:AI075991
R-PLACE1010078
ESTs
1.2e-48:267:92
Hs.12101:AA677423
R-PLACE1010081
Homo sapiens serine/threonine kinase RICK (RICK) mRNA, complete cds
3.0e-106:560:93
Hs.103755:AF027706
R-PLACE1010251
ESTs
0.00049:248:60
Hs.154164:AI246893
R-PLACE1010445
ESTs
1.5e-90:496:92
Hs.163999:AA778110
R-PLACE1010713
Interleukin 1 receptor antagonist
4.1e-07:307:59
Hs.81134:U65590
R-PLACE1010784
ESTs, Weakly similar to putative G-protein-coupled receptor [H.sapiens]
1.5e-21:206:78
Hs.29202:R71586
R-PLACE1010827
R-PLACE1010968
ESTs
2.6e-75:385:95
Hs.109884:AA766018
R-PLACE1011045
Homo sapiens E1-like protein mRNA, complete cds
5.3e-92:453:96
Hs.28190:AF094516
R-PLACE1011116
Homo sapiens embryonic lung protein (HUEL) mRNA, complete cds
1.5e-73:385:94
Hs.44053:AF006621
R-PLACE1011236
R-PLACE1011364
ESTs
2.3e-47:289:89
Hs.6163:W26652
R-PLACE1011407
ESTs
1.1e-09:191:64
Hs.118620:T60326
R-PLACE1011516
ESTs, Weakly similar to HYPOTHETICAL 21.5 KD PROTEIN IN SEC15-SAP4 INTERGENIC REGION [S.cerevisiae]
6.3e-75:441:88
Hs.110978:AA843431
R-PLACE1011708
Homo sapiens intrinsic factor-B12 receptor precursor, mRNA, complete cds
7.7e-93:521:91
Hs.148318:AF034611
R-PLACE1011824
ESTs
0.013:199:62
Hs.44343:AA532514
R-PLACE1011978
EST
4.0e-97:462:98
Hs.116391:AA644085
R-PLACE2000118
ESTs
1.2e-83:468:92
Hs.110578:AA115763
R-PLACE2000219
Homo sapiens KIAA0414 mRNA, partial cds
2.0e-44:344:81
Hs. 127649:AB007874
R-PLACE3000181
Human protocadherin 42 mRNA, 3' end of cds for alternative splicing PC42-8
1.3e-82:441:94
Hs.115642:L11369
R-PLACE3000213
ESTs, Highly similar to COMPLEMENT RECEPTOR TYPE 2 PRECURSOR [Homo sapiens]
2.3e-114:557:97
Hs.8963:AI379350
R-PLACE4000354
ESTs, Highly similar to COMPLEMENT RECEPTOR TYPE 2 PRECURSOR [Homo sapiens]
3.4e-105:518:97
Hs.8963:AI379350
R-PLACE4000455
ESTs
9.0e-57:289:96
Hs.42458:AA452296
R-THYRO1000036
Collagen, type IX, alpha 3
1.3e-100:527:93
Hs.53563:L41162
R-THYRO1000061
ESTs
1.8e-87:460:94
Hs.124869:H98977
R-THYRO1000099
ESTs
1.2e-34:193:94
Hs.149488:AI243816
R-THYRO1000196
Homo sapiens Ksp-cadherin (CDH16) mRNA, complete cds
3.7e-106:530:96
Hs.115418:AF016272
R-THYRO1000400
Human HU-K4 mRNA, complete cds
0.99:227:60
Hs.74573:U60644
R-THYRO1000580
Homo sapiens mRNA for KIAA0628 protein, complete cds
0.21:126:67
Hs. 43133:AB014528
R-THYRO1000584
ESTs, Weakly similar to golgi alpha-mannosidasell [H. sapiens]
3.0e-106:529:96
Hs.12183:AA888145
R-THYRO1000678
EST
2.9e-62:304:99
Hs.48956:N64339
R-THYRO1000776
ESTs
1.3e-102:533:94
Hs.4866:AA582196
R-THYRO1000795
ESTs
3.3e-98:529:92
Hs.55263:AI344338
R-THYRO1000846
ESTs
1.6e-105:522:96
Hs.135106:AI335251
R-THYRO1000866
Homo sapiens SKB1Hs mRNA, complete cds
1.3e-43:251:92
Hs.12912:AF015913
R-THYRO1000956
ESTs, Highly similar to PROBABLE G PROTEIN-COUPLED RECEPTOR APJ [Homo sapiens]
5.2e-106:548:94
Hs.9305:W84893
R-THYRO1000964
R-THYRO1000999
ESTs
1.9e-18:150:84
Hs.111583:AA463590
R-THYRO1001063
ESTs
1.5e-95:464:97
Hs.142684:AA902402
R-THYRO1001071
ESTs
2.5e-104:496:98
Hs.6071:AA868544
R-THYRO1001102
R-THYRO1001113
ESTs, Weakly similar to FER-1 [C.elegans]
7.1e-90:446:97
Hs.8076:AA115644
R-THYRO1001128
ESTs
1.9e-16:270:68
Hs.140194:N35720
R-THYRO1001205
Small inducible cytokine A5 (RANTES)
1.9e-58:400:84
Hs.155464:AF088219
R-THYRO1001237
ESTs
1.5e-104:532:96
Hs.6603:AA772122
R-THYRO1001242
EST
1.7e-50:281:93
Hs.101727:H16171
R-THYRO1001266
Homo sapiens mRNA for KIAA0650 protein, partial cds
0.00037:403:60
Hs.8118:AB014550
R-THYRO1001327
ESTs
1.2e-96:530:93
Hs.28786:AA034412
R-THYRO1001456
ESTs, Weakly similar to Similar to phytoene desaturase [C.elegans]
3.3e-43:257:92
Hs.97031:AA773647
R-THYRO1001457
ESTs, Highly similar to MYOSIN LIGHT CHAIN KINASE [Dictyostelium discoideum]
4.8e-59:284:99
Hs.9915:AI300083
R-THYRO1001471
ESTs
1.1e-67:378:93
Hs.52113:R40587
R-THYRO1001478
R-THYRO1001495
H.sapiens mRNA for Zinc-finger protein (ZNFpT17)
1.6e-63:434:84
Hs.32954:X65233
R-THYRO1001523
ESTs
5.8e-75:388:96
Hs.6527:R21517
R-THYRO1001529
ESTs
1.1e-25:184:87
Hs.18441:AA005104
R-THYRO1001593
ESTs
4.7e-34:182:98
Hs.8312:AA813022
R-THYRO1001608
ESTs
2.8e-107:547:95
Hs.23765:AA524283
R-THYRO1001641
Homo sapiens clone 24448 unknown mRNA, partial cds
1.1e-111:562:96
Hs.4973:AF070638
R-THYRO1001700
ESTs
1.3e-78:407:95
Hs.86987:N99896
R-THYRO1001702
ESTs
4.3e-98:566:92
Hs.119447:AA524436
R-THYRO1001725
ESTs
1.3e-84:424:96
Hs.38039:AI360128
R-THYRO1001770
ESTs
1.0e-62:325:97
Hs.20137:R08273
R-THYRO1001803
ESTs
6.8e-90:456:96
Hs.134438:R42585
R-Y79AA1000030
ESTs, Weakly similar to HYPOTHETICAL PROTEIN KIAA0168 [H.sapiens]
2.4e-98:515:94
Hs.32822:AI194045
R-Y79AA1000127
ESTs, Weakly similar to !!!! ALU SUBFAMILY J WARNING ENTRY !!!! [H.sapiens]
1.1e-57:307:95
Hs.83513:W05849
R-Y79AA1000207
EST
1.0e-97:411:96
Hs.141431:N21286
R-Y79AA1000226
ESTs, Highly similar to HYPOTHETICAL 52.8 KD PROTEIN T05E11.5 IN CHROMOSOME IV [Caenorhabditis elegans]
7.2e-102:545:94
Hs.11221:AI192291
R-Y79AA1000270
Human mRNA for ORF, Xq terminal portion
3.3e-107:564:93
Hs.6551:016469
R-Y79AA1000426
H.sapiens mRNA for activin beta-C chain
2.5e-10:217:66
Hs.83267:X82540
R-Y79AA1000521
Homo sapiens N-methyl-D-aspartate receptor 2D subunit precursor (NMDAR2D) mRNA, complete cds
0.73:257:59
Hs.113286:U77783
R-Y79AA1000750
ESTs
4.3e-75:391:95
Hs.157192:W84862
R-Y79AA1000776
ESTs
3.5e-56:303:95
Hs.118559:AA887084
R-Y79AA1000777
ESTs, Weakly similar to LIS-1 protein [H.sapiens]
9.5e-98:515:95
Hs.59461:W93217
R-Y79AA1000876
EST
2.7e-23:173:84
Hs.135872:AI037885
R-Y79AA1000959
Homo sapiens Hsp70 binding protein HspBP1 mRNA, complete cds
3.4e-80:453:92
Hs.53066:AF093420
R-Y79AA1000967
ESTs
7.3e-86:461:93
Hs.6262:T89093
R-Y79AA1001013
ESTs
1.4e-115:566:97
Hs.108408:N31922
R-Y79AA1001056
ESTs, Moderately similar to maternal transcript Maid [M.musculus]
8.7e-111:557:95
Hs.36794:AI038407
R-Y79AA1001062
ESTs
0.0021:365:59
Hs.106129:AA292171
R-Y79AA1001090
ESTs
5.0e-52:255:99
Hs.106214:AI123831
R-Y79AA1001212
Homo sapiens SL15 protein mRNA, complete cds
1.8e-83:407:97
Hs.6710:AF038961
R-Y79AA1001264
ESTs, Highly similar to DNAJ PROTEIN HOMOLOG 2 [Homo sapiens]
2.8e-111:552:96
Hs.62489:AI057091
R-Y79AA1001272
Zinc finger protein, X-linked
0.019:317:59
Hs.2074:X59739
R-Y79AA1001328
ESTs
3.6e-67:385:92
Hs.127792:AI421472
R-Y79AA1001426
ESTs
2.0e-13:92:93
Hs.105607:AA478379
R-Y79AA1001430
Homo sapiens mRNA for KIAA0469 protein, complete cds
2.0e-112:555:96
Hs.7764:AB007938
R-Y79AA1001523
EST
1.7e-07:120:73
Hs. 130984:AI015430
R-Y79AA1001530
Homo sapiens RRM RNA binding protein Gry-rbp (GRY-RBP) mRNA, complete cds
0.030:169:63
Hs.155489:AF037448
R-Y79AA1001592
ESTs
5.0e-60:340:91
Hs.87019:AA760977
R-Y79AA1001727
ESTs
6.1e-101:547:93
Hs.7404:W29012
R-Y79AA1001787
ESTs
8.8e-84:449:95
Hs.128866:AA977749
R-Y79AA1001795
Homo sapiens mRNA for GaIT4 protein
9.9e-110:541:97
Hs.21495:AL031228
R-Y79AA1001799
ESTs, Highly similar to MITOCHONDRIAL RNA SPLICING PROTEIN MSR4 [Saccharomyces cerevisiae]
1.6e-94:567:90
Hs.34401:AA447775
R-Y79AA1001803
ESTs, Highly similar to SECRETOGRANIN III PRECURSOR [Mus musculus]
1.2e-86:509:90
Hs.22215:AI371482
R-Y79AA1001863
ESTs
1.4e-23:268:73
Hs.131613:AI190576
R-Y79AA1002022
ESTs
8.9e-97:462:98
Hs.6140:D52151
R-nnnnnnnnnnnn
R-nnnnnnnnnnnn
Homo sapiens DNA recombination and repair protein (MRE11B) mRNA, complete cds
0.00075:456:59
Hs.153855:AF022778
R-Y79AA1002213
Human mRNA for KIAA0392 gene, partial cds
6.2e-45:304:85
Hs.40100:AB002390
R-Y79AA1002334
ESTs
7.7e-91:495:92
Hs.90804:W28091
R-Y79AA1002373
Human kpni repeat mrna (cdna clone pcd-kpni-8), 3' end
5.2e-98:545:91
Hs.103948:K00627
R-Y79AA1002376
ESTs
2.0e-91:455:97
Hs.153375:AI287812
R-Y79AA1002378
ESTs, Highly similar to ZINC FINGER PROTEIN ZFP-35 [Mus musculus]
9.4e-15:131:83
Hs.20082:W89121
R-Y79AA1002381
ESTs, Highly similar to CELL DIVISION CONTROL PROTEIN 2 HOMOLOG [Plasmodium falciparum (isolate k1 / thailand)]
1.5e-104:531:95
Hs.26322:AA156858

### Homology search result 10

Data obtained by the homology search for full length nucleotide sequences and deduced amino acid sequences. In the result of the search shown below, both units, aa and bp, are used as length units for the sequences to be compared. Each data includes Clone name, Definition in matching data, P value, Length of sequence to be compared, Homology, and Accession number (No.) of matching data. These items are shown in this order, separated by a double-slash mark,//.
C-HEMBA1000006//Homo sapiens mRNA; cDNA DKFZp564G1762 (from clone DKFZp564G1762).//0//1230bp//92%//AB026894
C-nnnnnnnnnnnn//GAMETOGENESIS EXPRESSED PROTEIN GEG-154.//2.30E-71//344aa//50%//P50636
C-HEMBA1000121//HYPOTHETICAL 68.7 KD PROTEIN ZK757.1 IN CHROMOSOME III.//4.80E-05//83aa//27%//P34679
C-HEMBA1000128//PATHOGENESIS-RELATED PROTEIN 1 PRECURSOR (PR-1).//3.20E-07//89aa//34%//P33154
C-HEMBA1000275
C-HEMBA1000300
C-HEMBA1000349//ATP-BINDING CASSETTE TRANSPORTER 1.//5.30E-65//352aa//39%//P41233
C-HEMBA1000443//Homo sapiens CGI-96 protein mRNA, complete cds.//4.70E-129//686bp//91%//AF151854
C-HEMBA1000590//Homo sapiens mRNA for matrilin-4, partial.//2.00E-273//1254bp//99%//AJ007581
C-HEMBA1000634//Homo sapiens T-cell activation protein (PGR1) gene, complete cds.//0//994bp//99%//AF116272
C-HEMBA1000713//Homo sapiens 10kD protein (BC10) mRNA, complete cds.//0//1254bp//99%//AF053470
C-HEMBA1000745//COLLAGEN ALPHA 1(I) CHAIN (FRAGMENTS).//2.00E-07//445aa//27%//P02454
C-HEMBA1000907
C-HEMBA1000940//GAP JUNCTION ALPHA-3 PROTEIN (CONNEXIN 44) (CX44).//2.90E-39//362aa//31%//P41987
C-HEMBA1000962
C-HEMBA1001221//AGRIN PRECURSOR.//2.50E-25//294aa//29%//P31696
C-HEMBA1001228//Human germline oligomeric matrix protein (COMP) mRNA, complete cds.//7.80E-286//1105bp//94%//L32137
C-HEMBA1001297
C-HEMBA1001390//Mus musculus polymerase I-transcript release factor mRNA, complete cds.//2.50E-57//464bp//82%//AF036249
C-HEMBA1001563
C-HEMBA1001621//PROBABLE G PROTEIN-COUPLED RECEPTOR APJ.//3.50E-123//259aa//89%//P35414
C-nnnnnnnnnnnn//ZINC FINGER PROTEIN 84 (ZINC FINGER PROTEIN HPF2).//2.40E-85//293aa//50%//P51523
C-HEMBA1001878//Homo sapiens U5 snRNP-specific 40 kDa protein mRNA, complete cds.//0//1488bp//99%//AF090988
C-HEMBA1002131//PROCOLLAGEN-LYSINE,2-OXOGLUTARATE 5-DIOXYGENASE 1//4.10E-10//140aa//30%//P24802
C-HEMBA1002163//HYPOTHETICAL 40.7 KD PROTEIN IN DAK1-ORC1 INTERGENIC REGION.//9.40E-28//309aa//30%//004651
C-HEMBA1002164
C-HEMBA1002167//Rattus norvegicus neuroligin I mRNA, complete cds.//1.30E-305//1643bp//91%//U22952
C-HEMBA1002178//PROCOLLAGEN-LYSINE,2-OXOGLUTARATE 5-DIOXYGENASE 1 PRECURSOR (EC 1.14.11.4) (LYSYL HYDROXYLASE 1) (LH1).//3.70E-10//140aa//30%//P24802 C-nnnnnnnnnnnn//Human glycyl-tRNA synthetase mRNA, complete cds.//0//2380bp//99%//U09587
C-HEMBA1002195//VEGETATIBLE INCOMPATIBILITY PROTEIN HET-E-1.//8.80E-23//221aa//31%//Q00808
C-HEMBA1002227//Homo sapiens mRNA for 80K-L protein, complete cds.//0//1324bp//98%//D10522
C-HEMBA1002239
C-HEMBA1002316
C-HEMBA1002420
C-HEMBA1002421//Human heparan sulfate proteoglycan (HSPG) core protein, 3' end.//0//2097bp//99%//J04621
C-HEMBA1002524//Human MHC Class I region proline rich protein mRNA, complete cds.//0//1763bp//95%//U63336
C-HEMBA1002551//PUTATIVE SERINE/THREONINE-PROTEIN KINASE PKWA (EC 2.7.1.-).//9.80E-08//110aa//37%//P49695
C-HEMBA1002767//Homo sapiens chromosome 1p33-p34 beta-1,4-galactosyltransferase mRNA, complete cds.//0//1497bp//99%//AF038660
C-HEMBA1002992//UBIQUITIN-LIKE PROTEIN DSK2.//2.00E-21//216aa//35%//P48510
C-HEMBA1003047//Homo sapiens intrinsic factor-B12 receptor precursor, mRNA, complete cds.//0//1768bp//99%//AF034611
C-HEMBA1003072//Gallus gallus single-strand DNA-binding protein csdp mRNA, partial cds.//3.30E-93//927bp//73%//U68380
C-HEMBA1003101//Homo sapiens tyrosylprotein sulfotransferase-2 mRNA, complete cds.//0//1854bp//99%//AF049891
C-HEMBA1003230//Homo sapiens fibulin-5.//5.60E-308//1398bp//99%//AJ133490
C-HEMBA1003294
C-HEMBA1003315//Mus musculus mRNA for DNA helicase, complete cds.//6.30E-250//1426bp//88%//AB013912
C-HEMBA1003392//Homo sapiens LDL receptor-related protein 6 (LRP6) mRNA, complete cds.//0//1721bp//100%//AF074264
C-HEMBA1003399//MVP1 PROTEIN.//2.30E-15//279aa//23%//P40959
C-HEMBA1003487
C-HEMBA1003530//S. scrofa mRNA for BM88 antigen.//1.20E-60//900bp//66%//X82027
C-HEMBA1003602//Homo sapiens CGI-67 protein mRNA, complete cds.//3.50E-70//732bp//66%//AF151825
C-HEMBA1003732//SFT2 PROTEIN.//1.50E-06//162aa//30%//P38166
C-HEMBA1003945//Homo sapiens hypothetical 43.2 Kd protein mRNA, complete cds.//8.90E-287//757bp//97%//AF077030
C-HEMBA1004110//Homo sapiens intersectin short form mRNA, complete cds.//0//2033bp//99%//AF064243
C-HEMBA1004250//CADHERIN-RELATED TUMOR SUPPRESSOR PRECURSOR (FAT PROTEIN).//6.40E-51//277aa//35%//P33450
C-HEMBA1004391//TUMOR SUPPRESSOR PROTEIN DCC PRECURSOR.//5.60E-20//194aa//26%//P70211
C-HEMBA1004444//GLYCOPROTEIN 25L PRECURSOR (GP25L).//4.60E-41//148aa//52%//P27869
C-HEMBA1004454
C-HEMBA1004505//MANNOSYL-OLIGOSACCHARIDE ALPHA-1,2-MANNOSIDASE ISOFORM 1 (EC 3.2.1.113) (MAN(9)-ALPHA-MANNOS)DASE).//2.70E-45//239aa//43%//P53624
C-HEMBA1004797
C-HEMBA1004982//TETRACYCLINE RESISTANCE PROTEIN, CLASS E (TETA(E)).//6.30E-10//149aa//26%//Q07282
C-HEMBA1005070//SKIN SECRETORY PROTEIN XP2 PRECURSOR (APEG PROTEIN).//1.10E-05//187aa//29%//P17437
C-HEMBA1005084//NON-RECEPTOR TYROSINE KINASE SPORE LYSIS A (EC 2.7.1.112) (TYROSINE- PROTEIN KINASE 1).//1.20E-07//102aa//37%//P18160
C-HEMBA1005145
C-HEMBA1005430
C-HEMBA1005449//GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).//5.40E-10//224aa//24%//P13983
C-HEMBA1005489
C-HEMBA1005522//COAGULATION FACTOR VII PRECURSOR (EC 3.4.21.21).//7.70E-15//78aa//51%//P98139
C-HEMBA1005545//MUSCARINIC ACETYLCHOLINE RECEPTOR M3.//0//2121aa//100%//U29589
C-HEMBA1005698//Homo sapiens vesicle trafficking protein (SEC22C) mRNA, complete cds.//6.60E-163//753bp//99%//AF039568
C-HEMBA1005913
C-HEMBA1005929//H.sapiens mRNA for serine/threonine protein kinase EMK.//6.50E-92//1092bp//69%//X97630
C-HEMBA1005945//Oryctolagus cuniculus peroxisomal Ca-dependent solute carrier mRNA, complete cds.//1.90E-44//666bp//65%//AF004161
C-HEMBA1006016
C-HEMBA1006171
C-HEMBA1006299
C-HEMBA1006311
C-HEMBA1006335
C-HEMBA1006430//Human putative transmembrane protein precursor (B5) mRNA, complete cds.//2.40E-70//1108bp//65%//L38961
C-HEMBA1006482//Homo sapiens h-sco1 (SC01) mRNA, nuclear gene encoding mitochondrial protein, complete cds.//0//1101bp//98%//AF026852
C-HEMBA1006572//ODD-SKIPPED PROTEIN.//2.60E-39//85aa//83%//P23803
C-HEMBA1006707//Homo sapiens mRNA for matrilin-4, partial.//0//2003bp//99%//AJ007581
C-HEMBA1006724
C-HEMBA1006902//Homo sapiens mRNA for matrilin-4, partial.//4.80E-275//1799bp//85%//AJ007581
C-HEMBA1006916//Homo sapiens Grb14 mRNA, complete cds.//3.00E-277//1010bp//95%//L76687
C-HEMBA1006960
C-HEMBA1007013
C-HEMBA1007057
C-HEMBA1007241
C-HEMBA1007291
C-HEMBA1007332
C-HEMBB1000276
C-HEMBB1000447//Homo sapiens JWA protein mRNA, complete cds.//0//2059bp//99%//AF070523
C-HEMBB1000642
C-HEMBB1000668//Homo sapiens mRNA for KIAA0893 protein, complete cds.//0//2375bp//99%//AB020700
C-HEMBB1000679//C. familiaris mRNA for TRAM-protein.//4.10E-210//1149bp//80%//X63678
C-HEMBB1000881//Danio rerio mRNA for MINDIN2, complete cds.//1.70E-67//948bp//6696//AB006085
C-HEMBB1000905//TRANSCRIPTIONAL REPRESSOR RCO-1.//1.00E-11//311aa//2796//P78706
C-HEMBB1001026//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//5.30E-11//142aa//30%//P32802
C-HEMBB1001048//Human Hpast (HPAST) mRNA, complete cds.//6.50E-39//448bp//75%//AC000159
C-HEMBB1001200
C-HEMBB1001407
C-HEMBB1001530//SLS1 PROTEIN PRECURSOR.//9.80E-10//273aa//27%//099158
C-HEMBB1001573
C-nnnnnnnnnnnn//Homo sapiens orphan G protein-coupled receptor (GPR34) gene, complete cds.//1.50E-251//1146bp//99%//AF118670
C-HEMBB1001847//NEUROGENIC PROTEIN BIG BRAIN.//4.70E-06//258aa//24%//P23645
C-HEMBB1001978
C-HEMBB1002162//Homo sapiens genethonin 1 mRNA, complete cds.//8.30E-67//328bp//99%//AF062354
C-HEMBB1002228
C-HEMBB1002245//PROSTAGLANDIN F2-ALPHA RECEPTOR REGULATORY PROTEIN PRECURSOR (PROSTAGLANDIN F2-ALPHA RECEPTOR ASSOCIATED PROTEIN).//0//879aa//89%//Q62786
C-HEMBB1002427//FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).//1.80E-70//221aa//50%//P16442
C-HEMBB1002465//ACYL-COA DEHYDROGENASE (EC 1.3.99.-).//2.30E-53//249aa//48%//P45857
C-HEMBB1002663
C-HEMBB1002693
C-MAMMA1000046
C-MAMMA1000118
C-nnnnnnnnnnnn//Homo sapiens SNC73 protein (SNC73) mRNA, complete cds.//1.50E-312//1594bp//93%//AF067420
C-MAMMA1000449
C-MAMMA1000457//Human NADH-cytochrome b5 reductase mRNA, 3' end.//9.50E-79//829bp//71%//M16462
C-MAMMA1000591//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N-ACETYLGALACTOSAMINYLTRANSFERASE) (GALNAC-T1).//1.20E-115//515aa//49%//007537
C-MAMMA1000681//PROBABLE G PROTEIN-COUPLED RECEPTOR EDG-1.//9.40E-82//311aa//52%//008530
C-MAMMA1001043//MRC OX-45 SURFACE ANTIGEN PRECURSOR (BCM1 SURFACE ANTIGEN) (BLAST-1) (CD48).//2.90E-12//239aa//28%//P10252
C-MAMMA1001893
C-NT2RM2000241
C-NT2RM2000306//PUTATIVE GTP-BINDING PROTEIN W08E3.3.//4.50E-130//362aa//68%//P91917
C-NT2RM2000410
C-NT2RM2000423//BETA-GALACTOSIDASE PRECURSOR (EC 3.2.1.23) (LACTASE).//1.80E-38//308aa//35%//P48982
C-NT2RM2000497//CHL1 PROTE)N.//9.90E-24//296aa//29%//P22516
C-NT2RM2000514//Homo sapiens mRNA for KIAA0875 protein, partial cds.//0//2381bp//99%//AB020682
C-NT2RM2000622
C-NT2RM2001126//Homo sapiens mRNA for multi PDZ domain protein.//0//1600bp//99%//AJ0001319
C-NT2RM2001902//Homo sapiens mRNA for PAK4 protein.//5.40E-216//988bp//99%//AJ011855
C-NT2RM2001939//Human G protein-coupled receptor GPR-NGA gene, complete cds.//0//1559bp//98%//U55312
C-NT2RM2001941//MUSCARINIC ACETYLCHOLINE RECEPTOR M1.//7.40E-38//193aa//34%//P08482
C-NT2RM4000198
C-NT2RM4000284//Human IgG Fc receptor hFcRn mRNA, complete cds.//1.30E-257//603bp//96%//U12255
C-NT2RM4000295
C-NT2RM4000326//ZINC FINGER PROTEIN 84 (ZINC FINGER PROTEIN HPF2).//9.00E-100//434aa//43%//P51523
C-NT2RM4000444//ANTIGEN PEPTIDE TRANSPORTER 1 (APT1).//1.70E-112//493aa//44%//P36370
C-NT2RM4000587
C-NT2RM4000648//K-GLYPICAN PRECURSOR.//4.00E-193//531aa//66%//P51655
C-NT2RM4000997//MONO- AND DIACYLGLYCEROL LIPASE PRECURSOR (EC 3.1.1.-) (MDGL).//1.80E-10//189aa//30%//P25234
C-NT2RM4001321
C-NT2RM4001325//CHONDROITIN 6-SULFOTRANSFERASE (EC 2.8.2.17) (C6ST).//2.90E-48//343aa//34%//Q92179
C-NT2RM4001735
C-NT2RM4002352//Homo sapiens hLRp105 mRNA for LDL receptor related protein 105, complete cds.//0//2184bp//99%//AB009462
C-NT2RP1000002
C-NT2RP1000050
C-NT2RP1000181//Homo sapiens delta-6 fatty acid desaturase mRNA, complete cds.//3.30E-121//1394bp//69%//AF126799
C-NT2RP1000261//ORM1 PROTE)N.//2.40E-17//137aa//35%//P53224
C-NT2RP1000300//Human transporter protein (g17) mRNA, complete cds.//3.80E-26//758bp//62%//U49082
C-NT2RP1000325//H.sapiens gene for phosphate carrier.//0//439bp//98%//X77337
C-NT2RP1000448
C-NT2RP1000551//Homo sapiens putative interferon-related protein (SM15) mRNA, partial cds.//0//1761bp//99%//U09585
C-NT2RP1000579//Human succinate dehydrogenase flavoprotein subunit (SDH) mRNA, complete cds.//0//1951bp//94%//L21936
C-NT2RP1000613//CARBONIC ANHYDRASE VI (EC 4.2.1.1) (SALIVARY) (CARBONATE DEHYDRATASE VI).//3.40E-52//304aa//40%//P08060
C-NT2RP1000903
C-NT2RP1000981//CELL SURFACE A33 ANTIGEN PRECURSOR.//3.60E-14//286aa//27%//Q99795
C-NT2RP1001004//F-SPONDIN PRECURSOR.//9.20E-43//322aa//35%//P35446
C-NT2RP1001020//SERINE/THREONINE-PROTEIN KINASE PAK-BETA (EC 2.7.1.-) (BETA-PAK) (P21-ACTIVATED KINASE 3) (CDC42/RAC EFFECTOR KINASE PAK-B).//9.70E-22//227aa//31%//Q61036
C-NT2RP1001563//TESTIS-SPECIFIC PROTEIN TPX-1 PRECURSOR (AUTOANTIGEN 1) (25
KD ACROSOMAL AUTOANTIGEN) (AA1).//9.70E-19//201aa//31%//Q60477
C-NT2RP2000394//Gallus gallus p52 pro-apototic protein mRNA, complete cds.//1.60E-90//956bp//70%//AF029071
C-NT2RP2000479
C-NT2RP2000514//Homo sapiens roundabout 2 (robo2) mRNA, partial cds.//3.00E-185//855bp//99%//AF040991
C-NT2RP2000533//Homo sapiens cornichon protein mRNA, complete cds.//1.30E-290//1324bp//99%//AF070654
C-NT2RP2000649//Homo sapiens mRNA for Hs Ste24p, complete cds.//0//2847bp//9996//AB016068
C-NT2RP2000663
C-NT2RP2000694//Homo sapiens 5T4 oncofetal trophoblast glycoprotein gene.//0//2278bp//99%//AJ012159
C-NT2RP2000903//Homo sapiens 5T4 oncofetal trophoblast glycoprotein gene.//0//2276bp//100%//AJ012159
C-NT2RP2001480//Homo sapiens thrombospondin 3 (THBS3) gene, complete cds.//0//2547bp//99%//L38969
C-NT2RP2001495//Human transporter protein (g17) mRNA, complete cds.//2.20E-65//641bp//65%//U49082
C-NT2RP2001514//PROBABLE CALCIUM-TRANSPORTING ATPASE 6 (EC 3.6.1. 38).//1.20E-133//429aa//41%//P39986
C-NT2RP2001529//Homo sapiens mRNA for ZIP-kinase, complete cds.//0//2079bp//99%//AB007144
C-NT2RP2001769//SERINE/THREONINE-PROTEIN KINASE ORB6 (EC 2.7.1.-).//9.10E-47//185aa//44%//013310
C-NT2RP2001878
C-NT2RP2001903//CALPAIN, LARGE [CATALYTIC] SUBUNIT (EC 3.4.22.17) (CALCIUM-ACTIVATED NEUTRAL PROTEINASE) (CANP) (MU/M-TYPE).//3.80E-58//475aa//34%//P00789
C-NT2RP2001915
C-NT2RP2001956//ORM1 PROTEIN.//3.90E-19//137aa//37%//P53224
C-NT2RP2002063//GNS1 PROTEIN.//3.60E-18//231aa//33%//P25358
C-NT2RP2002188//Rattus norvegicus neuroligin 3 mRNA, complete cds.//2.50E-226//1284bp//89%//U41663
C-NT2RP2002232//HYPOTHETICAL 85.7 KD PROTEIN C13G6.03 IN CHROMOSOME I.//1.90E-93//420aa//43%//Q09782
C-NT2RP2002304//Human mRNA for KIAA0383 gene, partial cds.//0//1640bp//99%//AB002381
C-NT2RP2002409
C-NT2RP2002510
C-NT2RP2002527//CYTOCHROME B5.//1.30E-11//92aa//38%//P40312
C-NT2RP2002533//Homo sapiens alpha 2 delta calcium channel subunit isoform I mRNA, complete cds.//0//2365bp//99%//AF042792
C-NT2RP2002564//Human zinc-finger protein C2H2-150 mRNA, complete cds.//0//2237bp//99%//U38864
C-NT2RP2002674//SOLUBLE EPOXIDE HYDROLASE (SEH) (EC 3.3.2.3) (EPOXIDE HYDRATASE) (CYTOSOLIC EPOXIDE HYDROLASE) (CEH).//5.50E-38//201aa//39%//P34913 C-NT2RP2002721//REGULATORY PROTEIN UHPC.//1.60E-23//153aa//30%//P27669
C-NT2RP2002824//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//3.50E-63//404aa//33%//P32802
C-NT2RP2002942//Homo sapiens mRNA for KIAA0806 protein, complete cds.//0//2090bp//99%//AB018349
C-NT2RP2002974//HOMEOBOX PROTEIN SIX5 (DM LOCUS-ASSOCIATED HOMEODOMAIN PROTEIN HOMOLOG) (FRAGMENT).//8.20E-241//555aa//84%//P70178
C-NT2RP2002976
C-NT2RP2003042//PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).//2.10E-109//385aa//52%//P53760
C-NT2RP2003179//PUTATIVE SERINE/THREONINE-PROTEIN KINASE EMK (EC 2.7.1.).//2.60E-67//256aa//49%//Q05512
C-NT2RP2003210//Homo sapiens fatty acid transport protein (FATP) mRNA, complete cds.//9.80E-272//1265bp//98%//AF055899
C-NT2RP2003369//RAS SUPPRESSOR PROTEIN 1 (RSU-1) (RSP-1 PROTEIN) (RSP-1).//5.90E-20//204aa//34%//015404
C-NT2RP2003383//Homo sapiens mRNA for KIAA0458 protein, complete cds.//0//2565bp//99%//AB007927
C-NT2RP2003469//GALACTOSE-PROTON SYMPORT (GALACTOSE TRANSPORTER).//1.10E-45//324aa//29%//P37021
C-NT2RP2003545//Homo sapiens STE20-like kinase 3 (mst-3) mRNA, complete cds.//5.40E-48//578bp//71%//AF024636
C-NT2RP2003593
C-NT2RP2003599
C-NT2RP2003655//HYPOTHETICAL 26.3 KD PROTEIN IN OYE2-GND1 INTERGENIC//4.80E-15//93aa//47%//P38869
C-NT2RP2003931
C-NT2RP2004141
C-NT2RP2004179
C-NT2RP2004205//BUTYROPHILIN PRECURSOR (BT).//1.60E-21//276aa//32%//Q62556
C-NT2RP2004447
C-NT2RP2004495//Human transporter protein (g17) mRNA, complete cds.//9.80E-64//642bp//64%//U49082
C-NT2RP2004524
C-NT2RP2004556
C-NT2RP2004606//Human fibroblast collagenase inhibitor mRNA, complete cds.//2.10E-166//768bp//99%//M12670
C-NT2RP2004648//Mouse beta-galactosidase (BGAL) gene, complete cds.//1.20E-33//1136bp//59%//M57734
C-NT2RP2004670//Rattus norvegicus vesicla-associate calmodulin-binding protein mRNA, complete cds.//0//1250bp//86%//L22557
C-NT2RP2004794//HYPOTHETICAL 24.5 KD PROTEIN IN SAP185-BCK1 INTERGENIC REGION.//2.70E-09//203aa//26%//P40857
C-NT2RP2004837
C-NT2RP2004847//ZINC FINGER PROTEIN 135.//8.00E-35//193aa//40%//P52742
C-nnnnnnnnnnnn//Homo sapiens SCG10-like-protein (SCLIP) mRNA, complete cds.//2.90E-170//813bp//98%//AF069709
C-NT2RP2005027
C-NT2RP2005163
C-NT2RP2005181//Mus musculus cationic amino acid transporter (CAT3) mRNA, complete cds.//5.30E-315//2126bp//81%//U70859
C-NT2RP2005247//ZINC-FINGER PROTEIN RFP (RET FINGER PROTEIN).//5.00E-53//296aa//37%//Q62158
C-NT2RP2005425//Homo sapiens mRNA for AKAP450 protein.//0//4341bp//99%//AJ131693
C-NT2RP2005463//PROTEIN PTM1 PRECURSOR.//7.40E-15//284aa//28%//P32857
C-NT2RP2005514
C-NT2RP2005541//N-ACETYLGLUCOSAMINE-6-SULFATASE PRECURSOR (EC 3.1.6.14) (G6S) (GLUCOSAMINE-6-SULFATASE).//4.70E-24//78aa//51%//P15586
C-NT2RP2005632
C-NT2RP2005878//PUTATIVE STERO I D DEHYDROGENASE KIK-I (EC 1.1.1.-).//3.60E-55//238aa//50%//057314
C-NT2RP2005883//DOPAMINE-BETA-MONOOXYGENASE PRECURSOR (EC 1.14.17.1)
(DOPAMINE BETA- HYDROXYLASE) (DBH).//6.70E-72//512aa//34%//P15101
C-NT2RP2005887
C-NT2RP2005941//Human paired box gene (PAX6) homologue, complete cds.//1.40E-308//1396bp//99%//M93650
C-NT2RP2005994//HYPOTHETICAL 51.5 KD PROTEIN D2024.3 IN CHROMOSOME IV.//3.50E-35//144aa//47%//P49191
C-NT2RP2006042//GLUCOAMYLASE S1/S2 PRECURSOR (EC 3.2.1.3) (GLUCAN 1, 4-ALPHA-GLUCOSIDASE) (1,4-ALPHA-D-GLUCAN GLUCOHYDROLASE).//9.40E-15//501aa//25%//P08640
C-NT2RP2006269//DOLICHYL-PHOSPHATE-MANNOSE-PROTEIN MANNOSYLTRANSFERASE 2 (EC 2.4.1.109).//2.30E-78//679aa//32%//P31382
C-NT2RP2006512//GNS1 PROTEIN.//2.00E-21//290aa//29%//P25358
C-NT2RP3000059//ANKYRIN, BRAIN VARIANT 2 (ANKYRIN B) (ANKYRIN, NONERYTHROID) (FRAGMENT).//3.70E-12//133aa//32%//Q01485
C-NT2RP3000063//NEUROFILAMENT TRIPLET H PROTEIN (200 KD NEUROFILAMENT PROTEIN) (NF-H).//5.00E-29//596aa//30%//P19246
C-NT2RP3000125//RETINOBLASTOMA BINDING PROTEIN 2 (RBBP-2).//6.30E-08//70aa//41%//P29375
C-NT2RP3000169//Homo sapiens MRS1 mRNA, complete cds.//0//1519bp//97%//AF093239
C-NT2RP3000172//CALCIUM/CALMODULIN-DEPENDENT PROTEIN KINASE TYPE I (EC 2.7.1.123) (CAM KINASE I).//1.30E-80//359aa//44%//Q14012
C-NT2RP3000201//Homo sapiens mRNA for KIAA0687 protein, partial cds.//2.00E-305//1224bp//99%//AB014587
C-NT2RP3000436//PROBABLE PROTEIN DISULFIDE ISOMERASE P5 PRECURSOR (EC 5.3.4.1).//6.20E-27//90aa//42%//P38660
C-NT2RP3000460//Canis familiaris sec61 homologue mRNA, complete cds.//1.80E-198//643bp//89%//M96629
C-NT2RP3000616//FIBROMODULIN PRECURSOR (FM) (COLLAGEN-BINDING 59 KD PROTEIN).//5.20E-26//227aa//36%//Q06828
C-NT2RP3000721//HYPOTHETICAL 149.7 KD PROTEIN IN IRE1-KSP1 INTERGENIC REGION.//1.10E-22//171aa//36%//P38800
C-NT2RP3000820//PUTATIVE SERINE/THREONINE-PROTEIN KINASE PKWA (EC 2.7.1.-).//1.90E-30//269aa//33%//P49695
C-NT2RP3000871
C-NT2RP3000907//PROBABLE CALC I UM-TRANSPORT I NG ATPASE 6 (EC 3.6.1.38).//2.20E-134//296aa//42%//P39986
C-NT2RP3001012//Homo sapiens mRNA for KIAA0829 protein, partial cds.//0//2906bp//98%//AB020636
C-NT2RP3001044
C-NT2RP3001061//Homo sapiens mRNA for KIAA0853 protein, partial cds.//0//3591bp//99%//AB020660
C-NT2RP3001170//Homo sapiens mRNA; cDNA DKFZp586K2120 (from clone DKFZp586K2120).//0//2421bp//99%//AL080163
C-NT2RP3001195//GALACTOSE-PROTON SYMPORT (GALACTOSE TRANSPORTER).//4.70E-48//339aa//29%//P37021
C-NT2RP3001240//Canis familiaris sec61 homologue mRNA, complete cds.//1.20E-301//1141bp//89%//M96629
C-NT2RP3001322//PROBABLE CALCIUM-TRANSPORTING ATPASE 3 (EC 3.6.1.38) (ENDOPLASMIC RETICULUM CA2+-ATPASE).//1.70E-21//220aa//30%//P39524
C-NT2RP3001388//SYNAPTOTAGMIN IV.//2.00E-118//430aa//54%//P50232
C-nnnnnnnnnnnn//Human mRNA for KIAA0315 gene, partial cds.//0//2971bp//99%//AB002313
C-nnnnnnnnnnnn//Homo sapiens mRNA for KIAA0017 protein, complete cds.//0//3243bp//99%//D87686
C-NT2RP3001560//Homo sapiens cleft lip and palate transmembrane protein 1 (CLPTM1) mRNA, complete cds.//0//2468bp//99%//AF037339
C-NT2RP3001592//N2,N2-DIMETHYLGUANOSINE TRNA METHYLTRANSFERASE PRECURSOR (EC 2.1.1.32).//1.30E-18//279aa//27%//P15565
C-NT2RP3001738//CYTOCHROME B5.//1.30E-11//133aa//33%//P00169
C-NT2RP3001754
C-NT2RP3001858
C-NT2RP3002160//Canis familiaris forssman synthetase mRNA, complete cds.//5.00E-152//789bp//84%//U66140
C-NT2RP3002311//BETA-GALACTOSIDASE PRECURSOR (EC 3.2.1.23) (LACTASE).//9.80E-103//547aa//43%//P48982
C-NT2RP3002342//Human transporter protein (g17) mRNA, complete cds.//1.70E-65//641bp//65%//U49082
C-NT2RP3002448
C-NT2RP3002721//Porcine citrate synthase mRNA, complete cds.//9.10E-281//1454bp//93%//M21197
C-NT2RP3002738//Homo sapiens enhancer of filamentation (HEF1) mRNA, complete cds.//2.20E-47//763bp//65%//L43821
C-NT2RP3002790
C-NT2RP3002836//Homo sapiens mRNA for KIAA0463 protein, partial cds.//0//1617bp//99%//AB007932
C-NT2RP3002958//TRANS-GOLGI NETWORK INTEGRAL MEMBRANE PROTEIN TGN38 PRECURSOR.//8.00E-08//197aa//26%//P19814
C-NT2RP3003000//Homo sapiens T-type calcium channel alpha-1 subunit mRNA, complete cds.//0//3160bp//96%//AF051946
C-NT2RP3003076
C-NT2RP3003354//SECRETORY CARRIER-ASSOCIATED MEMBRANE PROTEIN 3.//5.10E-55//208aa//51%//035609
C-NT2RP3003469
C-NT2RP3003527//Homo sapiens mRNA for protein kinase Dyrk1B.//0//2483bp//99%//Y17999
C-NT2RP3003535//UDP-N-ACETYLGLUCOSAMINE―PEPTIDE N-ACETYLGLUCOSAMINYLTRANSFERASE 110 KD SUBUNIT (EC 2.4.1.-) (O-GLCNAC TRANSFERASE P110 SUBUN)T).//8.80E-18//368aa//25%//P56558
C-NT2RP3003559
C-NT2RP3003614
C-NT2RP3003729
C-NT2RP3003849//PROTEIN KINASE C, BRAIN ISOZYME (EC 2.7.1.-) (PKC) (DPKC53E(BR)). PKC1.//1.20E-13//126aa//34%//P05130
C-NT2RP3003874//Homo sapiens incomplete cDNA for a mutated allele of a myosin class I, myh-1c.//0//2160bp//98%//AJ001381
C-NT2RP3003963
C-NT2RP3004000
C-NT2RP3004075
C-NT2RP3004083
C-NT2RP3004090//GOLIATH PROTEIN (G1 PROTE)N).//9.00E-33//179aa//47%//Q06003
C-NT2RP3004130//CELL SURFACE ANTIGEN 114/A10 PRECURSOR.//8.10E-06//71aa//42%//P19467
C-NT2RP3004133//GLYCOSYLTRANSFERASE ALG2 (EC 2.4.1.-).//2.50E-48//198aa//37%//P43636
C-NT2RP3004202
C-NT2RP3004309//Homo sapiens mRNA: cDNA DKFZp586C201 (from clone DKFZp586C201).//0//2584bp//9996//AL050118
C-NT2RP3004321
C-NT2RP3004355
C-NT2RP3004374
C-NT2RP3004406//HYPOTHETICAL 37.4 KD PROTEIN IN IRR1-TIM44 INTERGENIC REG)ON.//3.20E-15//165aa//33%//P40544
C-NT2RP3004552//COMPLEMENT RECEPTOR TYPE 1 PRECURSOR (C3B/C4B RECEPTOR) (CD35 ANT)GEN).//8.50E-24//263aa//33%//P17927
C-NT2RP3004557//Human Ki nuclear autoantigen mRNA, complete cds.//0//2181bp//96%//U11292
C-NT2RP3004625//Homo sapiens mRNA for KIAA0975 protein, partial cds.//0//1339bp//99%//AB023192
C-NT2RP3004640//Bos taurus tuftelin mRNA, complete cds.//0//1204bp//88%//AF105228
C-NT2RP3004647//PUTATIVE MITOCHONDRIAL CARRIER YMR166C.//2.00E-15//220aa//27%//Q03829
C-NT2RP4000108//Human gene for neurofilament subunit NF-L.//0//1998bp//99%//AF176680
C-NT2RP4000962//SPORULATION-SPECIFIC PROTE I N 1 (EC 2.7.1.-).//2.60E-18//225aa//32%//P08458
C-NT2RP4001009//Homo sapiens mRNA for farnesylated-proteins converting enzyme 1.//0//2965bp//99%//Y13834
C-NT2RP4001467//Human placental cDNA coding for 5'nucleotidase (EC 3.1.3.5).//0//2140bp//99%//X55740
C-OVARC1000090
C-OVARC1000105//UBIQUITIN-CONJUGATING ENZYME E2-28.4 KD (EC 6.3.2.19) (UBIQUITIN- PROTEIN LIGASE) (UBIQUITIN CARRIER PROTEIN).//4.20E-47//171aa//56%//P33296
C-OVARC1000137
C-OVARC1000208
C-OVARC1000255//H.sapiens syk mRNA for protein-tyrosine kinase.//0//1525bp//9746//Z29630
C-OVARC1000275//DESMOPLAKIN I AND II (DPI AND DPII) (FRAGMENT).//9.90E-16//352aa//23%//P15924
C-OVARC1000298
C-OVARC1000410//Homo sapiens angiopoietin Y1 mRNA, complete cds.//2.10E-63//744bp//69%//AF107253
C-OVARC1000439//HYPOTHETICAL 64.3 KD GTP-BINDING PROTEIN C02F5.3 IN CHROMOSOME III.//1.40E-33//143aa//53%//P34280
C-OVARC1000467
C-OVARC1000529//PROBABLE SERINE/THREONINE-PROTEIN KINASE YKL101W (EC 2.7.1.-).//1.40E-23//165aa//39%//P34244
C-OVARC1000775
C-nnnnnnnnnnnn//ZINC FINGER PROTEIN 157.//1.00E-35//130aa//46%//P51786
C-OVARC1000811//PLASMINOGEN (EC 3.4.21.7) (FRAGMENT).//6.40E-13//115aa//34%//Q01177
C-OVARC1000853
C-OVARC1000916//H.sapiens PISSLRE mRNA.//7.30E-280//1117bp//95%//X78342
C-OVARC1000956//SKIN SECRETORY PROTEIN XP2 PRECURSOR (APEG PROTEIN).//2.20E-09//250aa//28%//P17437
C-OVARC1001030//Homo sapiens mRNA for KIAA0886 protein, complete cds.//0//907bp//99%//AB020693
C-OVARC1001049//TRANSCRIPTION FACTOR HES-1 (C-HAIRY1).//7.50E-14//96aa//36%//057337
C-OVARC1001086//Homo sapiens cyclin T2a mRNA, complete cds.//0//1593bp//98%//AF048731
C-OVARC1001132//GC-RICH SEQUENCE DNA-BINDING FACTOR (GCF) (TRANSCRIPTION FACTOR 9) (TCF-9).//2.30E-44//268aa//36%//P16383
C-OVARC1001163//HYPOTHETICAL 49.3 KD PROTEIN C30D11.06C IN CHROMOSOME I.//2.30E-20//152aa//30%//Q09906
C-OVARC1001222
C-OVARC1001338//AUTOPHAGY SERINE/THREONINE-PROTEIN KINASE APG1 (EC 2.7.1.-).//8.80E-30//125aa//40%//P53104
C-OVARC1001569//PROBABLE SERINE/THREONINE-PROTEIN KINASE YKL101W (EC 2.7.1.-).//1.50E-22//164aa//39%//P34244
C-OVARC1001596//Homo sapiens Arf-like 2 binding protein BART1 mRNA, complete cds.//0//1766bp//99%//AF126062
C-OVARC1001725
C-OVARC1001727
C-OVARC1001807//Human TR3 orphan receptor mRNA, complete cds.//1.10E-243//1145bp//98%//L13740
C-OVARC1001991//VOLTAGE-GATED POTASSIUM CHANNEL PROTEIN KV3.3 (KSHIIID).//8.30E-06//114aa//35%//Q01956
C-OVARC1002058//Human 18S rRNA gene, complete.//1.50E-164//921bp//9196//M10098
C-OVARC1002178
C-PLACE1000033//VON WILLEBRAND FACTOR PRECURSOR.//3.80E-17//190aa//28%//Q28295
C-PLACE1000231//Rattus norvegicus WAP four-disulfide core domain protein (ps20) mRNA, complete cds. //2.70E-101//947bp//74%//AF037272
C-PLACE1000258//ZINC FINGER PROTEIN 91 (ZINC FINGER PROTEIN HTF10) (HPF7).//1.70E-55//431aa//35%//Q05481
C-PLACE1000442//ZINC FINGER PROTEIN ZFP-36 (FRAGMENT).//1.00E-88//213aa//67%//P16415
C-PLACE1000560
C-PLACE1000740//Mus musculus (Notch2) mRNA, complete cds.//5.60E-122//893bp//81%//M93661
C-PLACE1000912
C-PLACE1000914//Homo sapiens PB39 mRNA, complete cds.//7.50E-88//500bp//69%//AF045584
C-PLACE1000927//HYPOTHETICAL 20.9 KD PROTEIN B0563.3 IN CHROMOSOME X.//6.30E-21//123aa//37%//Q11079
C-PLACE1001016//SODIUM CHANNEL PROTEIN PARA (PARALYTIC PROTEIN).//6.80E-12//133aa//28%//P35500
C-nnnnnnnnnnnn//Homo sapiens T245 protein (T245) mRNA, complete cds.//0//1801bp//99%//AF043906
C-PLACE1001100
C-PLACE1001114//HYPOTHETICAL BHLF1 PROTEIN.//9.20E-06//389aa//31%//P03181
C-PLACE1001123
C-PLACE1001183
C-PLACE1001229
C-PLACE1001231//Rattus norvegicus sodium-dependent multi-vitamin transporter (SMVT) mRNA, complete cds.//2.20E-137//918bp//80%//AF026554
C-nnnnnnnnnnnn//Human pregnancy-specific beta 1-glycoprotein 4 (PSG4) clone FL-64 mRNA, complete cds.//7.60E-293//1631bp//90%//U18469
C-PLACE1001340//Homo sapiens mRNA for KIAA0719 protein, complete cds.//0//2868bp//99%//AB018262
C-PLACE1001401//HIGH AFFINITY IMMUNOGLOBULIN EPSILON RECEPTOR BETA-SUBUNIT
(FCERI) (IGE FC RECEPTOR, BETA-SUBUNIT).//3.70E-18//148aa//39%//P13386
C-PLACE1001407
C-PLACE1001464//Human placental cDNA coding for 5'nucleotidase (EC 3.1.3.5).//0//2756bp//99%//X55740
C-PLACE1001500//Homo sapiens RecQ5 mRNA for DNA helicase, complete cds.//2.30E-271//1230bp//99%//AB006533
C-PLACE1001516//240 KD PROTEIN OF ROD PHOTORECEPTOR CNG-CHANNEL [CONTAINS: GLUTAMIC ACID-RICH PROTEIN (GARP); CYCLIC-NUCLEOTIDE-GATED CATION CHANNEL 4 (CNG CHANNEL 4) (CNG-4) (CYCLIC NUCLEOTIDE-GATED CATION CHANNEL MODULATORY SUBUNIT)].//2.30E-08//274aa//28%//028181
C-PLACE1001536
C-PLACE1001564//H.sapiens mRNA for HE6 Tm7 receptor.//5.10E-36//499bp//70%//X81892
C-PLACE1001655//Homo sapiens Shab-related delayed-rectifier K+ channel alpha subunit (KCNS3) mRNA, complete cds.//0//1708bp//99%//AF043472
C-nnnnnnnnnnnn//Homo sapiens calumein (Calu) mRNA, complete cds.//0//1776bp//99%//AF013759
C-PLACE1001788
C-PLACE1001795//HYPOTHETICAL 30.6 KD PROTEIN IN SCP160-SMC3 INTERGENIC REGION PRECURSOR.//3.40E-20//159aa//40%//P47032
C-PLACE1001836//ENV POLYPROTEIN PRECURSOR (COAT POLYPROTEIN) [CONTAINS: OUTER MEMBRANE PROTEIN GP70; TRANSMEMBRANE PROTEIN P20E].//5.00E-27//134aa//4796//P10269
C-PLACE1001918//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//2.30E-53//339aa//33%//P32802
C-PLACE1001949//PROBABLE CALCIUM-TRANSPORTING ATPASE 9 (EC 3.6.1.38).//3.00E-75//315aa//44%//Q12697
C-PLACE1002080//Homo sapiens antigen NY-CO-9 (NY-CO-9) mRNA, partial cds.//0//1588bp//99%//AF039691
C-PLACE1002095
C-PLACE1002153//Homo sapiens TACC2 protein (TACC2) mRNA, partial cds.//0//1202bp//99%//AF095791
C-PLACE1002329//EPIDERMAL GROWTH FACTOR RECEPTOR KINASE SUBSTRATE EPS8.//6.50E-105//213aa//45%//Q08509
C-PLACE1002355//COMPLEMENT C4 PRECURSOR [CONTAINS: C4A ANAPHYLATOXIN].//4.20E-12//131aa//40%//P01029
C-PLACE1002374//Human mRNA for pro-cathepsin L (major excreted protein MEP).//1.30E-313//1363bp//97%//X12451
C-PLACE1002518
C-PLACE1002547//Homo sapiens mRNA for KIAA0719 protein, complete cds.//0//2985bp//99%//AB018262
C-PLACE1002726//Oryctolagus cuniculus mRNA for epithelial calcium channel (ECaC).//2.80E-202//926bp//82%//AJ133128
C-PLACE1002905//ENDOZEPINE-RELATED PROTEIN PRECURSOR (MEMBRANE-ASSOCIATED DIAZEPAM BINDING INHIBITOR) (MA-DBI).//2.40E-37//188aa//40%//P07106
C-PLACE1002911//POLIOVIRUS RECEPTOR HOMOLOG PRECURSOR.//4.50E-39//345aa//32%//P32507
C-PLACE1002967//HIGH AFFINITY IMMUNOGLOBULIN EPSILON RECEPTOR BETA-SUBUNIT
(FCERI) (IGE FC RECEPTOR, BETA-SUBUN)T).//4.60E-08//156aa//30%//Q01362
C-PLACE1003135//SPORULATION-SPECIFIC PROTEIN 1 (EC 2.7.1.-).//1.30E-47//210aa//49%//P08458
C-PLACE1003163//Homo sapiens DBI-related protein mRNA, complete cds.//1.00E-294//1344bp//99%//AF069301
C-PLACE1003428//Homo sapiens mRNA for VNN1 protein.//1.80E-142//676bp//72%//AJ132099
C-PLACE1003438
C-PLACE1003460
C-PLACE1003529//DNA-DIRECTED RNA POLYMERASE II LARGEST SUBUNIT (EC 2.7.7.6) (RPB1) (FRAGMENT).//1.30E-09//281aa//22%//P11414
C-PLACE1003573//T-CELL SURFACE GLYCOPROTEIN YE1/48 (T LYMPHOCYTE ANTIGEN A1) (LY49-A ANT)GEN).//3.70E-16//226aa//26%//P20937
C-PLACE1003598//TRP-ASP REPEATS CONTAINING PROTEIN RBA-1.//1.80E-07//161aa//27%//P90917
C-PLACE1003644
C-PLACE1003737//TOLL PROTEIN PRECURSOR.//5.40E-07//203aa//27%//P08953
C-PLACE1003772//EXTENSIN PRECURSOR (CELL WALL HYDROXYPROLINE-RICH GLYCOPROTE)N).//2.40E-12//124aa//38%//P13983
C-PLACE1003839//Homo sapiens putative G protein-coupled receptor (RAIG1) mRNA, complete cds.//8.10E-18//771bp//58%//AF095448
C-PLACE1003845//PUTATIVE UDP-GLUCOSE 4-EPIMERASE (EC 5.1.3.2) (GALACTOWALDENASE) (UDP- GALACTOSE 4-EPIMERASE).//3.40E-37//302aa//30%//Q57664
C-PLACE1003852//Homo sapiens mRNA for KIAA0758 protein, partial cds.//0//1667bp//99%//AB018301
C-PLACE1004028
C-PLACE1004166//CREB-BINDING PROTE)N.//1.80E-12//147aa//35%//P45481
C-PLACE1004168//Homo sapiens mRNA for KIAA1007 protein, partial cds.//0//2637bp//99%//AB023224
C-PLACE1004199
C-PLACE1004279//HYPOTHETICAL 59.1 KD PROTEIN ZK637.1 IN CHROMOSOME III.//1.40E-08//166aa//30%//P30638
C-PLACE1004282//MONO- AND DIACYLGLYCEROL LIPASE PRECURSOR (EC 3.1.1.-) (MDGL).//2.10E-11//189aa//30%//P25234
C-PLACE1004305//RAS-RELATED PROTEIN RAC1.//9.60E-29//197aa//41%//P40792
C-PLACE1004441//Human G protein-coupled receptor (GPR1) gene, complete cds.//0//1880bp//98%//AC007383
C-PLACE1004450//AMINOPEPTIDASE N (EC 3.4.11.2) (MICROSOMAL AMINOPEPTIDASE) (LEUKEMIA ANTIGEN CD13).//1.30E-91//562aa//35%//P15541
C-PLACE1004482//Rattus norvegicus hematopoietic lineage switch 2 related protein (Hls2-rp) mRNA, complete cds.//1.90E-246//1643bp//83%//AF097723
C-PLACE1004519
C-PLACE1004520//Human pregnancy-specific beta-glycoprotein d mRNA, complete cds.//9.10E-279//882bp//88%//M20881
C-PLACE1004630
C-PLACE1004637
C-PLACE1004648//GLUCOAMYLASE S1/S2 PRECURSOR (EC 3.2.1.3) (GLUCAN 1,4-ALPHA-GLUCOSIDASE) (1, 4-ALPHA-D-GLUCAN GLUCOHYDROLASE).//1.40E-18//395aa//25%//P08640
C-nnnnnnnnnnnn//Homo sapiens tyrosylprotein sulfotransferase-2 mRNA, complete cds.//0//1825bp//99%//AF049891
C-PLACE1004816//Homo sapiens mRNA for Hakata antigen, complete cds.//1.00E-166//856bp//94%//D88587
C-PLACE1004887//GOLIATH PROTEIN (G1 PROTEIN).//4.80E-33//179aa//47%//006003
C-PLACE1005003//PROSTASIN PRECURSOR (EC 3. 4. 21.-).//2.20E-52//269aa//41%//Q16651
C-PLACE1005005//Homo sapiens ubiquitin conjugating enzyme G2 (UBE2G2) mRNA, complete cds.//4.10E-261//1209bp//98%//AF032456
C-PLACE1005031//CHLORINE CHANNEL PROTEIN P64.//8.00E-92//205aa//87%//P35526
C-PLACE1005239//Homo sapiens mRNA for HIRIP3 protein (clone pH4-31, pH4-17).//1.80E-235//1010bp//84%//AJ223351
C-PLACE1005250//Homo sapiens D-type cyclin-interacting protein 1 (DIP1) mRNA, complete cds.//0//1046bp//96%//AF082569
C-PLACE1005383//Homo sapiens UP50 mRNA, complete cds.//0//2019bp//99%//AF093118
C-PLACE1005410//Canis familiaris sec61 homologue mRNA, complete cds.//2.40E-204//673bp//89%//M96629
C-PLACE1005426//Human pregnancy-specific beta 1-glycoprotein 4 (PSG4) clone FL-64 mRNA, complete cds.//0//1629bp//95%//U18469
C-PLACE1005519//Homo sapiens STE20-like kinase 3 (mst-3) mRNA, complete cds.//4.60E-108//1070bp//73%//AF024636
C-PLACE1005544//Mus musculus junctional adhesion molecule (Jam) mRNA, complete cds.//2.00E-159//1237bp//76%//U89915
C-PLACE1005660//HYPOTHETICAL 64.3 KD GTP-BINDING PROTEIN C02F5.3 IN CHROMOSOME III.//1.90E-33//143aa//53%//P34280
C-PLACE1005669
C-PLACE1005682//B-CELL LYMPHOMA 3-ENCODED PROTEIN (BCL-3 PROTEIN).//4.90E-09//183aa//33%//P20749
C-PLACE1005725//HYPOTHETICAL 55.1 KD PROTEIN B0416.5 IN CHROMOSOME X.//7.60E-17//295aa//27%//Q11073
C-PLACE1005736//Human mRNA for BAS-GRIP protein.//0//2378bp//99%//E16311
C-PLACE1005768
C-PLACE1005878//Bovine chlorine channel protein (p64) mRNA, complete cds.//5.90E-137//889bp//85%//L16547
C-PLACE1006093
C-PLACE1006208//Homo sapiens nGAP mRNA, complete cds.//3.30E-151//694bp//100%//AF047711
C-PLACE1006219//PUTATIVE UDP-GLUCOSE 4-EPIMERASE (EC 5.1.3.2) (GALACTOWALDENASE) (UDP- GALACTOSE 4-EPIMERASE).//3.50E-37//302aa//30%//Q57664
C-PLACE1006277//Homo sapiens mRNA for Z39Ig protein.//0//1768bp//99%//AJ132502
C-PLACE1006290//GLYCOSYLTRANSFERASE ALG2 (EC 2.4.1.-).//8.50E-75//301aa//39%//P43636
C-PLACE1006443//Homo sapiens PB39 mRNA, complete cds.//4.30E-98//553bp//70%//AF045584
C-PLACE1006515//Homo sapiens mRNA for KIAA0576 protein, partial cds.//0//2846bp//99%//AB011148
C-PLACE1006716//30 KD ADIPOCYTE COMPLEMENT-RELATED PROTEIN PRECURSOR (ACRP30)
(ADIPOCYTE SPECIFIC PROTEIN ADIPOQ).//4.60E-25//181aa//35%//Q60994 C-PLACE1006809//SLS1 PROTEIN PRECURSOR.//9.10E-10//273aa//27%//P08124
C-PLACE1006959
C-PLACE1007028
C-PLACE1007040
C-PLACE1007096//PUTATIVE SUGAR TRANSPORT PROTEIN GUTA.//2.70E-17//174aa//27%//034368
C-nnnnnnnnnnnn//Homo sapiens mRNA for putative glucosyltransferase, partial cds.//0//1373bp//99%//AJ224875
C-PLACE1007296//Human mRNA for a presumptive KDEL receptor.//1.10E-185//1038bp//91%//X55885
C-PLACE1007591
C-PLACE1007626//Homo sapiens unknown mRNA, complete cds.//3.00E-246//1122bp//99%//AF047439
C-PLACE1007702//Mus musculus TRA1 mRNA, complete cds.//7.50E-41//662bp//64%//D78335
C-PLACE1007845//GLYCOSYLTRANSFERASE ALG2 (EC 2.4.1.-).//4.80E-14//158aa//40%//P43636
C-PLACE1007881//HYPOTHETICAL 69.4 KD PROTEIN F13H10.3 IN CHROMOSOME IV.//3.10E-99//504aa//42%//Q19425
C-PLACE1008297//MYOSIN HEAVY CHAIN KINASE B (EC 2.7.1.129) (MHCK B).//1.30E-14//187aa//33%//P90648
C-nnnnnnnnnnnn//Homo sapiens mRNA for putative glucosyltransferase, partial cds.//0//1616bp//99%//AJ224875
C-PLACE1008469
C-PLACE1008549//Homo sapiens Ets transcription factor ESE-2b mRNA, complete cds.//0//2274bp//99%//AF115403
C-PLACE1008657//Bovine mRNA for adseverin, complete cds.//7.80E-227//1246bp//90%//D26549
C-PLACE1008716//Human beta-1,2-N-acetylglucosaminyltransferase II (MGAT2) gene, complete cds. //0//1888bp//9996//U15128
C-PLACE1008984
C-PLACE1008985//Mus musculus synaptotagmin VIII mRNA, partial cds.//3.80E-140//650bp//81%//U20107
C-PLACE1009067
C-PLACE1009196
C-PLACE1009279//cDNA encoding novel physiologically active protein which have serine protease activity.//6.60E-86//1414bp//64%//E12965
C-PLACE1009527//Oryctolagus cuniculus mRNA for epithelial calcium channel (ECaC).//1.20E-87//585bp//83%//AJ133128
C-PLACE1009546
C-PLACE1009600//Mouse mRNA for tetracycline transporter-like protein, complete cds.//1.10E-264//924bp//88%//D88315
C-PLACE1009735
C-PLACE1009982//SALIVARY GLUE PROTEIN SGS-3 PRECURSOR.//5.20E-08//166aa//28%//P02840
C-PLACE1010078//ORM1 PROTEIN.//3.70E-19//137aa//37%//P53224
C-PLACE1010081//Homo sapiens serine/threonine kinase RICK (RICK) mRNA, complete cds.//0//2033bp//99%//AF027706
C-PLACE1010251//FIBRILLIN 2 PRECURSOR.//1.70E-31//201aa//35%//Q61555 C-PLACE1010784//Homo sapiens orphan G protein-coupled receptor (GPR34) gene, complete cds.//2.30E-252//1146bp//99%//AF008670
C-PLACE1010827//COP-COATED VESICLE MEMBRANE PROTEIN P24 PRECURSOR (FRAGMENT).//1.90E-19//163aa//34%//P49020
C-PLACE1010968//PROTEIN-TYROSINE PHOSPHATASE DLAR PRECURSOR (EC 3.1.3.48) (PROTEIN- TYROSINE-PHOSPHATE PHOSPHOHYDROLASE).//3.40E-30//690aa//26%//P16621
C-PLACE1011045//Homo sapiens E1-like protein mRNA, complete cds.//0//2376bp//99%//AF094516
C-PLACE1011116//GLUCOAMYLASE S1/S2 PRECURSOR (EC 3.2.1.3) (GLUCAN 1,4-ALPHA-GLUCOSIDASE) (1, 4-ALPHA-D-GLUCAN GLUCOHYDROLASE).//3.30E-09//234aa//27%//P08640
C-PLACE1011236//Mus musculus mRNA for RST, complete cds.//1.70E-90//1398bp//65%//AB005451
C-PLACE1011407//ZINC FINGER PROTEIN 184 (FRAGMENT).//1.80E-133//342aa//59%//Q99676
C-PLACE1011516//HYPOTHETICAL 21.5 KD PROTEIN IN SEC15-SAP4 INTERGENIC REGION.//1.30E-13//139aa//34%//P53073
C-PLACE1011708//Homo sapiens intrinsic factor-B12 receptor precursor, mRNA, complete cds.//0//1840bp//98%//AF034611
C-PLACE1011824//Human Ste20-like kinase (MST2) mRNA, complete cds.//6.40E-202//561bp//92%//U26424
C-PLACE1011978//ZINC FINGER PROTEIN 91 (ZINC FINGER PROTEIN HTF10) (HPF7).//1.10E-194//547aa//57%//Q05481
C-PLACE2000118//WISKOTT-ALDRICH SYNDROME PROTEIN HOMOLOG (WASP).//3.20E-27//205aa//43%//P70315
C-PLACE2000219
C-SKNMC1000004
C-THYRO1000036//Homo sapiens collagen alpha 3 type IX (COL9A3) mRNA, complete cds.//1.20E-258//1376bp//93%//L41162
C-THYRO1000061
C-THYRO1000099
C-THYRO1000196//Homo sapiens Ksp-cadherin (CDH16) mRNA, complete cds.//0//1632bp//91%//AF016272
C-THYRO1000400//Homo sapiens mRNA full length insert cDNA clone EUROIMAGE 25795.//0//1893bp//99%//AL109665
C-THYRO1000580//ZINC FINGER PROTEIN 184 (FRAGMENT).//9.90E-114//279aa//59%//Q99676
C-THYRO1000584//Homo sapiens mRNA for KIAA0935 protein, partial cds.//0//1338bp//99%//AB023152
C-THYRO1000678//Homo sapiens Cx30 gene.//0//1741bp//97%//AJ005585
C-THYRO1000795//C.elegans mRNA for Oxoglutarate/malate carrier protein.//8.80E-42//821bp//63%//X76114
C-THYRO1000846
C-THYRO1000866//SHK1 KINASE-BINDING PROTEIN 1.//4.40E-91//449aa//44%//P78963
C-THYRO1000956//Human G protein-coupled receptor APJ gene, complete cds.//0//1583bp//99%//U03642
C-THYRO1000999
C-THYRO1001063
C-THYRO1001071//GAMMA-ADAPTIN (GOLGI ADAPTOR HA1/AP1 ADAPTIN GAMMA SUBUNIT) (CLATHRIN ASSEMBLY PROTEIN COMPLEX 1 GAMMA LARGE CHAIN).//8.20E-14//157aa//33%//P22892
C-THYRO1001102
C-THYRO1001113//Homo sapiens mRNA; cDNA DKFZp564E1616 (from clone DKFZp564E1616).//0//1361bp//99%//AL096713
C-THYRO1001128
C-THYRO1001205
C-THYRO1001237//PHYTOENE DEHYDROGENASE (EC 1.3.-.-) (PHYTOENE DESATURASE) (ALBINO-1 PROTE)N).//3.10E-13//346aa//22%//P21334
C-THYRO1001266//Human sodium iodide symporter mRNA, complete cds.//7.20E-81//1466bp//62%//U66088
C-THYRO1001327
C-THYRO1001456//HYPOTHETICAL 56.2 KD PROTEIN CY31.25C.//9.40E-32//355aa//3196//Q10555
C-THYRO1001457//H.sapiens mRNA for protein kinase C mu.//2.30E-218//1183bp//73%//X75756
C-THYRO1001471
C-THYRO1001478//CYTOCHROME B-245 HEAVY CHAIN (P22 PHAGOCYTE B-CYTOCHROME) (NEUTROPHIL CYTOCHROME B, 91 KD POLYPEPTIDE) (CGD91-PHOX) (GP91-PHOX) (CYTOCHROME B(558) BETA CHAIN) (SUPEROXIDE-GENERATING NADPH OXIDASE HEAVY CHAIN SUBUNIT).//8.90E-50//296aa//35%//P04839
C-THYRO1001495
C-THYRO1001523//Homo sapiens mRNA for TM7XN1 protein.//0//3663bp//99%//AJ011001
C-THYRO1001529//SERINE PALMITOYLTRANSFERASE 2 (EC 2.3.1.50) (LONG CHAIN BASE BIOSYNTHESIS PROTEIN 2) (SPT 2).//5.50E-25//115aa//53%//Q09925 C-THYRO1001700//SERINE/THREONINE PROTEIN KINASE RIP (EC 2.7.1.-) (CELL DEATH PROTEIN RIP) (RECEPTOR INTERACTING PROTEIN).//9.70E-33//268aa//37%//Q60855
C-THYRO1001702//Mus musculus mRNA for myeloid associated differentiation protein.//1.50E-128//1204bp//73%//AJ001616
C-THYRO1001725
C-THYRO1001803
C-Y79AA1000127
C-Y79AA1000207
C-Y79AA1000226
C-Y79AA1000270//Bos taurus vacuolar H+ ATPase subunit Ac45 mRNA, complete cds.//1.00E-271//1490bp//83%//U10039
C-Y79AA1000426//Mus musculus activin beta E subunit mRNA, complete cds.//7.70E-200//1533bp//78%//U96386
C-Y79AA1000521
C-Y79AA1000776
C-Y79AA1000777//VEGETATIBLE INCOMPATIBILITY PROTEIN HET-E-1.//8.10E-48//283aa//38%//Q00808
C-nnnnnnnnnnnn//Homo sapiens intersectin long form mRNA, complete cds.//0//1519bp//99%//AF064244
C-Y79AA1000876//PROTEIN DISULFIDE ISOMERASE-RELATED PROTEIN PRECURSOR (ERP72).//1.60E-44//210aa//38%//P13667
C-Y79AA1000959//Homo sapiens Hsp70 binding protein HspBP1 mRNA, complete cds.//4.80E-283//1405bp//95%//AF093420
C-Y79AA1000967//CALCIUM/CALMODULIN-DEPENDENT PROTEIN KINASE TYPE I (EC 2.7.1.123) (CAM KINASE I).//1.00E-77//359aa//44%//Q14012
C-Y79AA1001013
C-Y79AA1001056
C-Y79AA1001062//TUMOR NECROSIS FACTOR, ALPHA-INDUCED PROTEIN 1, ENDOTHELIAL (B12 PROTEIN).//8.90E-12//132aa//38%//Q13829
C-Y79AA1001090//NUCLEAR FACTOR NF-KAPPA-B P105 SUBUNIT (DNA-BINDING FACTOR KBF1) (EBP- 1) (NF-KAPPA-B1 P84 / NF-KAPPA-B1 P98) [CONTAINS: NUCLEAR FACTOR NF- KAPPA-B P50 SUBUNIT] (FRAGMENT).//4.50E-09//144aa//31%//Q63369
C-Y79AA1001264//HYPOTHETICAL 39.9 KD PROTEIN T15H9.1 IN CHROMOSOME II PRECURSOR.//5.10E-106//351aa//58%//Q10005
C-Y79AA1001272
C-Y79AA1001328//Mus musculus mRNA for DII3 protein, complete cds.//1.90E-263//1988bp//79%//AB013440
C-Y79AA1001430//Homo sapiens mRNA for KIAA0469 protein, complete cds.//0//2943bp//9996//AB007938
C-Y79AA1002022//POLIOVIRUS RECEPTOR HOMOLOG PRECURSOR.//2.20E-06//140aa//26%//P32507
C-BNGH41000020//NADH-UBIQUINONE OXIDOREDUCTASE CHAIN 2 (EC 1.6.5.3).//9.80E-159//347aa//90%//P03891
C-BNGH41000091//POTASSIUM CHANNEL PROTEIN EAG.//1.20E-249//625aa//65%//Q02280
C-HEMBA1000462//Human potential transcriptional repressor NOT4Hp (NOT4H) mRNA, complete cds.//0//935bp//99%//U71267
C-HEMBA1000477//HYPOTHETICAL 64.8 KD PROTEIN IN GD11-COX15 INTERGENIC REGION.//1.40E-38//344aa//34%//P40085
C-HEMBA1000671//ZINC FINGER PROTEIN 184 (FRAGMENT).//3.90E-104//388aa//46%//Q99676
C-HEMBA1000732//Homo sapiens mRNA for latent transforming growth factor-beta binding protein-4.//0//2153bp//94%//Y13622
C-HEMBA1000835
C-HEMBA1000875
C-HEMBA1001184//SH3BGR PROTEIN (21-GLUTAMIC ACID-RICH PROTEIN) (21-GARP).//2.50E-32//100aa//60%//P55822
C-HEMBA1001272//Homo sapiens mRNA for KIAA1171 protein, partial cds.//0//1490bp//99%//AB032997
C-HEMBA1001296
C-HEMBA1002048//ODD-SKIPPED PROTEIN.//1.60E-55//122aa//75%//P23803
C-HEMBA1002985
C-HEMBA1003120//ZINC FINGER PROTEIN 184 (FRAGMENT).//1.00E-193//547aa//54%//Q99676
C-HEMBA1003497//ZINC FINGER PROTEIN 151 (POLYOMAVIRUS LATE INITIATOR PROMOTER BINDING PROTEIN) (LP-1) (ZINC FINGER PROTEIN Z13).//4.00E-28//358aa//29%//Q60821
C-HEMBA1004007
C-HEMBA1004085
C-HEMBA1004785//MODIFIER 3 PROTEIN (M33).//1.40E-27//221aa//33%//P30658
C-HEMBA1004952
C-HEMBA1004971
C-HEMBA1005230//ZINC FINGER PROTEIN 140.//2.00E-17//83aa//66%//P52738
C-HEMBA1005246
C-HEMBA1005267//ANKYRIN, BRAIN VARIANT 1 (ANKYRIN B) (ANKYRIN, NONERYTHROID).//8.40E-14//187aa//33%//Q01484
C-HEMBA1006276//ZINC FINGER PROTEIN 90 (ZFP-90) (ZINC FINGER PROTEIN NK10).//1.70E-06//56aa//57%//Q61967
C-HEMBA1006357//SECRETORY CARRIER-ASSOCIATED MEMBRANE PROTEIN 2.//3.70E-39//136aa//52%//015127
C-HEMBA1006517
C-HEMBA1006544
C-HEMBA1006749//Homo sapiens mRNA for matrilin-4, partial.//1.40E-275//1942bp//83%//AJ007581
C-HEMBA1006770//FLOWERING TIME CONTROL PROTEIN FCA.//1.20E-33//352aa//34%//004425
C-HEMBA1006912
C-HEMBA1007063
C-HEMBB1000106//CELLULAR NUCLEIC ACID BINDING PROTEIN (CNBP).//1.60E-10//139aa//30%//P53996
C-HEMBB1000407
C-HEMBB1000542//Mus musculus bromodomain-containing protein BP75 mRNA, complete cds.//2.60E-232//1452bp//85%//AF084259
C-HEMBB1001547//Homo sapiens CGI-02 protein mRNA, complete cds.//0//2311bp//99%//AF132937
C-HEMBB1001959//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//7.30E-14//97aa//38%//P25210
C-HEMBB1002039
C-HEMBB1002041//Homo sapiens transmembrane protein TENB2 (TENB2) mRNA, complete cds.//0//1746bp//99%//AF179274
C-HEMBB1002051//Homo sapiens Ets transcription factor ESE-2b mRNA, complete cds.//1.30E-95//454bp//99%//AF115403
C-HEMBB1002120//UDP-N-ACETYLGLUCOSAMINE-PEPTIDE N-ACETYLGLUCOSAMINYLTRANSFERASE 110 KD SUBUNIT (EC 2.4.1.-) (O-GLCNAC TRANSFERASE P110 SUBUNIT).//4.90E-22//337aa//27%//P56558
C-HEMBB1002302
C-HEMBB1002661//Homo sapiens cardiovascular helix-loop-helix factor 2 (CHF2) mRNA, complete cds.//0//2174bp//99%//AF179274
C-MAMMA1000106
C-MAMMA1000141
C-MAMMA1000204//Homo sapiens dysferlin mRNA, complete cds.//0//2028bp//99%//AF075575
C-MAMMA1000226
C-MAMMA1000403//Homo sapiens CDK4-binding protein p34SEI1 (SEI1) mRNA, complete cds.//1.20E-255//1165bp//99%//AF117959
C-MAMMA1000473//HYPOTHETICAL 35.6 KD PROTEIN IN SPC1-ILV3 INTERGENIC REGION.//5.10E-45//299aa//34%//P47088
C-MAMMA1000496//MIC1 PROTEIN.//3.00E-25//202aa//33%//P53258
C-MAMMA1000528
C-MAMMA1000614//Homo sapiens pseudouridine synthase 1 (PUS1) mRNA, partial cds.//2.10E-302//1370bp//99%//AF116238
C-MAMMA1000652
C-MAMMA1000706
C-MAMMA1000788//Bos taurus P14 (p14) mRNA, complete cds.//3.90E-85//502bp//89%//AF037349
C-MAMMA1000810
C-MAMMA1000814
C-MAMMA1000881//Homo sapiens protein kinase (SGK3) mRNA, complete cds.//0//1292bp//100%//AF169035
C-MAMMA1000986
C-MAMMA1000994//Homo sapiens ISLR(immunoglobulin superfamily containing leucine-rich repeat) mRNA, complete cds, alternatively spliced transcript ISLR-2.//0//2211bp//99%//AB024536
C-MAMMA1001141
C-MAMMA1001150//PROTEIN KINASE C, MU TYPE (EC 2.7.1.-) (NPKC-MU).//7.50E-304//587aa//68%//Q15139
C-MAMMA1001237//MONOCARBOXYLATE TRANSPORTER 2 (MCT 2).//7.70E-64//196aa//41%//P53988
C-MAMMA1001284
C-MAMMA1001310//HYPOTHETICAL 57.3 KD TRP-ASP REPEATS CONTAINING PROTEIN IN POM152- REC114 INTERGENIC REGION.//1.50E-67//441aa//37%//Q04225
C-MAMMA1001344
C-MAMMA1001418//HYPOTHETICAL PROTEIN HI0519.//6.90E-27//181aa//38%//P44742
C-MAMMA1001532//ZINC FINGER PROTEIN 84 (ZINC FINGER PROTEIN HPF2).//5.60E-126//319aa//56%//P51523
C-MAMMA1001615//NEUROGENIC DIFFERENTIATION FACTOR 1.//3.80E-11//90aa//42%//Q13562
C-MAMMA1001623//Homo sapiens mRNA: cDNA DKFZp434J1027 (from clone DKFZp434J1027); partial cds.//1.30E-269//1222bp//99%//AL133084
C-MAMMA1001634
C-MAMMA1001957
C-MAMMA1001978//Cimex lectularius apyrase (APY) mRNA, complete cds.//6.70E-19//988bp//56%//AF085499
C-MAMMA1002070//PLASMINOGEN (EC 3.4.21.7) (FRAGMENT).//1.10E-07//103aa//33%//Q01177
C-MAMMA1002080//RAS-RELATED PROTEIN RAB-13.//1.80E-29//208aa//37%//P51153
C-MAMMA1002087
C-MAMMA1002095//CALCIUM-TRANSPORTING ATPASE 1 (EC 3.6.1.38) (P-TYPE CALCIUM ATPASE).//3.70E-222//867aa//52%//043108
C-MAMMA1002128//ABC1 PROTEIN HOMOLOG PRECURSOR.//2.50E-97//464aa//45%//Q92338
C-MAMMA1002142//NON-RECEPTOR TYROSINE KINASE SPORE LYSIS A (EC 2.7.1.112) (TYROSINE- PROTEIN KINASE 1).//9.80E-17//146aa//35%//P18160
C-MAMMA1002165//Homo sapiens connective tissue growth factor related protein WISP-2 (WISP2) mRNA, complete cds.//4.80E-60//382bp//89%//AF100780
C-MAMMA1002205//Homo sapiens mRNA: cDNA DKFZp586C091 (from clone DKFZp586C091).//2.00E-81//308bp//81%//AL050119
C-MAMMA1002234//SIGNAL RECOGNITION PARTICLE 68 KD PROTEIN (SRP68).//0//627aa//96%//000004
C-MAMMA1002586//Homo sapiens alpha 1,2-mannosidase mRNA, complete cds.//0//2228bp//99%//AF148509
C-MAMMA1002633
C-MAMMA1003126//Human Hpast (HPAST) mRNA, complete cds.//3.70E-162//1355bp//75%//AF001434
C-NT2RM1000580//GLYCOSYLTRANSFERASE ALG2 (EC 2.4.1.-).//8.50E-75//301aa//39%//P43636
C-NT2RM1000858//tricarboxylate carrier [rats, liver, mRNA Partial, 2986 nt].//2.10E-98//1035bp//70%//S70011
C-NT2RM2000565//HYPOTHETICAL 85.7 KD PROTEIN C13G6.03 IN CHROMOSOME I.//9.40E-94//394aa//43%//Q09782
C-NT2RM2000582//Homo sapiens mRNA for KIAA1053 protein, partial cds.//0//2666bp//99%//AB028976
C-NT2RM2000589//Bos taurus myosin X, complete cds.//0//4376bp//84%//U55042
C-NT2RM2000632//EXCISION REPAIR PROTEIN ERCC-6 (COCKAYNE SYNDROME PROTEIN CSB).//6.40E-62//183aa//47%//003468
C-NT2RM2000773//Homo sapiens KNSL4 and MAZ genes for kinesin-like DNA binding protein and Myc-associated zinc finger protein, complete cds.//3.00E-289//1092bp//99%//AB017335
C-NT2RM2001558//Homo sapiens protein kinase A binding protein AKAP110 mRNA, complete cds.//0//2398bp//99%//AF093408
C-NT2RM2001626//FLIGHTLESS-I PROTEIN HOMOLOG.//4.30E-19//362aa//26%//P34268
C-NT2RM2001643
C-NT2RM2001738//SOF1 PROTEIN.//3.00E-110//325aa//47%//P33750
C-NT2RM2001792//Homo sapiens angiopoietin-related protein-2 mRNA,complete cds.//7.10E-149//995bp//86%//AF125175
C-NT2RM2001818//SIALIDASE (EC 3.2.1.18) (NEURAMINIDASE) (NA) (MAJOR SURFACE ANTIGEN).//4.30E-11//488aa//26%//P23253
C-NT2RM4000100//Homo sapiens mRNA for KIAA0989 protein, partial cds.//0//2678bp//99%//AB023206
C-NT2RM4000115//HYPOTHETICAL 68.8 KD PROTEIN B0464.6 IN CHROMOSOME III.//1.20E-16//204aa//30%//Q03564
C-NT2RM4000417//SYNAPTOTAGMIN II.//2.70E-23//293aa//30%//P46097
C-NT2RM4000593//HYPOTHETICAL 111.9 KD PROTEIN C22H10.03C IN CHROMOSOME I.//3.90E-27//576aa//24%//Q10297
C-NT2RM4000761//H.sapiens mitochondrial genome (consensus sequence).//0//1931bp//99%//X62996
C-NT2RM4000965//PUTATIVE IMPORTIN BETA-4 SUBUNIT (KARYOPHERIN BETA-4 SUBUNIT).//9.00E-44//520aa//29%//060100
C-NT2RM4001377//Homo sapiens mRNA for KIAA0638 protein, partial cds.//0//1346bp//99%//AB014538
C-NT2RM4001768//Homo sapiens CGI-82 protein mRNA, complete cds.//0//1925bp//99%//AF151840
C-NT2RM4001843//BETA-GALACTOSIDASE PRECURSOR (EC 3.2.1.23) (LACTASE).//6.20E-33//263aa//38%//P48982
C-NT2RP1000239
C-NT2RP1000465//UBIQUITIN-LIKE PROTEIN SMT3.//5.10E-07//81aa//33%//P55857
C-NT2RP1000468//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//2.00E-13//97aa//38%//P25210
C-NT2RP1000679
C-NT2RP1000740//Homo sapiens mRNA; cDNA DKFZp586F1918 (from clone DKFZp586F1918).//4.60E-97//456bp//99%//AL050091
C-NT2RP1001031//VEGETATIBLE INCOMPATIBILITY PROTEIN HET-E-1.//3.40E-42//285aa//35%//Q00808
C-NT2RP2000178//MITOCHONDRIAL LON PROTEASE HOMOLOG 1 PRECURSOR (EC 3.4.21.-).//2.40E-192//778aa//48%//P93647
C-NT2RP2000240
C-NT2RP2000447//GOLGIN-95.//2.80E-33//99aa//66%//Q08379
C-NT2RP2000610//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//1.50E-13//97aa//38%//P25210
C-NT2RP2000616//EXTENSIN PRECURSOR (CELL WALL HYDROXYPROLINE-RICH GLYCOPROTEIN).//4.10E-12//323aa//30%//P13983
C-NT2RP2000712//ZINC FINGER PROTEIN 135.//3.70E-87//296aa//53%//P52742
C-NT2RP2000739//ZINC FINGER PROTEIN 83 (ZINC FINGER PROTEIN HPF1).//7.50E-73//387aa//37%//P51522
C-NT2RP2000818//Homo sapiens xenotropic and polytropic murine leukemia virus receptor (X3) mRNA, complete cds.//0//2724bp//99%//AF089744 C-NT2RP2001200//Homo sapiens mRNA for KIAA0676 protein, partial cds.//0//1539bp//100%//AB014576
C-NT2RP2001223//MYOTUBULARIN-RELATED PROTEIN 3 (FRAGMENT).//3.30E-05//76aa//39%//013615
C-NT2RP2001276//NPDC-1 PROTEIN PRECURSOR.//3.00E-133//331aa//77%//Q64322
C-NT2RP2001388//TRNA-SPLICING ENDONUCLEASE SUBUNIT SEN2 (EG 3.1.27.9) (TRNA-INTRON ENDONUCLEASE).//5.90E-13//157aa//33%//P16658
C-NT2RP2001469//VEGETATIBLE INCOMPATIBILITY PROTEIN HET-E-1.//1.30E-14//242aa//24%//Q00808
C-NT2RP2001562//Homo sapiens GLE1 (GLE1) mRNA, complete cds.//0//1899bp//98%//AF058922
C-NT2RP2001662//HOMEODOMAIN PROTEIN AKR (AVIAN KNOTTED-RELATED PROTEIN).//1.80E-49//94aa//81%//Q90655
C-NT2RP2001755//Rattus norvegicus f-spondin mRNA, complete cds.//0//2974bp//86%//M88469
C-NT2RP2001817//Homo sapiens sirtuin type 1 (SIRT1) mRNA, complete cds.//0//3092bp//99%//AF083106
C-NT2RP2001948//HST2 PROTEIN (HOMOLOGOUS TO SIR2 PROTEIN 2).//1.40E-08//191aa//27%//P53686
C-NT2RP2002015
C-NT2RP2003390//Homo sapiens mRNA for SEC63 protein.//0//2629bp//99%//AJ011779
C-NT2RP2003664//Homo sapiens mRNA for leptin receptor gene-related protein.//1.90E-237//1081bp//99%//Y12670
C-NT2RP2003940//ZINC FINGER PROTEIN 43 (ZINC PROTEIN HTF6).//7.00E-111//401aa//43%//P28160
C-NT2RP2004069//HYPOTHETICAL 26.4 KD PROTEIN EEED8.8 IN CHROMOSOME II.//3.00E-45//188aa//52%//Q09297
C-NT2RP2004108//ZINC FINGER PROTEIN ZFP-36 (FRAGMENT).//3.30E-171//474aa//62%//P16415
C-nnnnnnnnnnnn//Homo sapiens calumein (Calu) mRNA, complete cds.//0//2415bp//99%//AF013759
C-NT2RP2005069//Rat vacuolar protein sorting homolog r-vps33b mRNA, complete cds.//0//1792bp//87%//U35245
C-NT2RP2005378//GLYCINE-RICH CELL WALL STRUCTURAL PROTEIN 1.8 PRECURSOR (GRP 1.8).//6.30E-28//183aa//47%//P10496
C-NT2RP2005391//Homo sapiens partial mRNA for putative p621 protein which interacts with transcription factor Sp1.//0//1544bp//99%//AJ242978
C-NT2RP2005597//Homo sapiens protein 0-mannosyl-transferase 1 (POMT1) mRNA, complete cds.//0//1821bp//97%//AF095136
C-NT2RP2005666
C-NT2RP2006004//FIBROMODULIN PRECURSOR (FM) (COLLAGEN-BINDING 59 KD PROTE)N).//3.00E-26//227aa//36%//Q06828
C-NT2RP2006092//Homo sapiens mRNA for Fe65L2, complete cds.//0//1156bp//99%//AB018247
C-NT2RP2006134
C-NT2RP3000011//VEGETATIBLE INCOMPATIBILITY PROTEIN HET-E-1.//4.00E-14//320aa//24%//Q00808
C-NT2RP3000022//Homo sapiens mRNA for KIAA0936 protein, complete cds.//0//2881bp//99%//AB023153
C-NT2RP3000171//Mus musculus partial mRNA for B-IND1 protein (B-ind1 gene).//4.40E-99//571bp//89%//Z97207
C-NT2RP3000304//Homo sapiens LDL receptor-related protein 6 (LRP6) mRNA, complete cds.//0//2895bp//99%//AF074264
C-NT2RP3000378//PAIRED AMPHIPATHIC HELIX PROTEIN.//4.20E-39//186aa//36%//P22579
C-NT2RP3000444
C-NT2RP3000645
C-NT2RP3000676//PUTATIVE MITOCHONDRIAL CARRIER YMR166C.//2.10E-15//220aa//27%//Q03829
C-NT2RP3000677//DNA BINDING PROTEIN RFX2.//3.60E-56//233aa//41%//P48378 C-NT2RP3000789//Homo sapiens IGF-II mRNA-binding protein 1 (IMP-1) mRNA, complete cds.//0//1458bp//100%//AF117106
C-NT2RP3000818
C-NT2RP3000838//TRICHOHYALIN.//9.80E-11//491aa//26%//Q07283
C-NT2RP3000921//TUMOR SUPPRESSOR PROTEIN DCC PRECURSOR (COLORECTAL CANCER SUPPRESSOR).//4.00E-21//316aa//29%//P43146
C-NT2RP3001159//Homo sapiens mRNA: cDNA DKFZp586C201 (from clone DKFZp586C201).//0//2558bp//99%//AL050118
C-NT2RP3001271//NON-GREEN PLASTID TRIOSE PHOSPHATE TRANSLOCATOR PRECURSOR (CTPT).//2.60E-09//334aa//22%//P52178
C-NT2RP3001542//TUMOR NECROSIS FACTOR, ALPHA-INDUCED PROTEIN 1, ENDOTHELIAL (B12 PROTEIN).//4.70E-11//132aa//37%//Q13829
C-NT2RP3001685//PRPE PROTEIN.//1.00E-68//382aa//41%//P77495
C-NT2RP3001976//ZINC FINGER PROTEIN 84 (ZINC FINGER PROTEIN HPF2).//8.90E-143//379aa//55%//P51523
C-NT2RP3002015//Homo sapiens CGI-71 protein mRNA, complete cds.//0//1991bp//99%//AF151829
C-NT2RP3002281//Homo sapiens mRNA for KIAA0765 protein, partial cds.//0//2286bp//99%//AB018308
C-NT2RP3002286//Homo sapiens candidate tumor suppressor protein DICE1 mRNA, complete cds.//0//2719bp//99%//AF097645
C-NT2RP3002324
C-NT2RP3002353
C-NT2RP3002571//Homo sapiens mRNA for KIAA1108 protein, partial cds.//4.40E-273//1311bp//97%//AB029031
C-NT2RP3002664
C-NT2RP3002737//Homo sapiens voltage-gated potassium channel KCNQ4 (KCNQ4) mRNA, complete cds.//0//1552bp//99%//AF105202
C-NT2RP3002887
C-NT2RP3002900//Homo sapiens CGI-109 protein mRNA, complete cds.//8.70E-298//1321bp//92%//AF151867
C-NT2RP3002983
C-NT2RP3003473//Homo sapiens CGI-55 protein mRNA, complete cds.//5.50E-275//1309bp//98%//AF151813
C-NT2RP3003532//OX-2 MEMBRANE GLYCOPROTEIN PRECURSOR (FRAGMENT).//4.40E-139//263aa//99%//P41217
C-NT2RP3004025
C-NT2RP3004067//Homo sapiens mRNA for NESCA, complete cds.//0//1962bp//99%//AB026894
C-NT2RP3004119//PEREGRIN (BR140 PROTEIN).//7.30E-39//227aa//43%//P55201
C-NT2RP3004294//Xenopus laevis ER1 mRNA, complete cds.//1.20E-71//335bp//79%//AF015454
C-NT2RP3004345//N2,N2-DIMETHYLGUANOSINE TRNA METHYLTRANSFERASE PRECURSOR (EC 2.1.1.32).//3.90E-18//279aa//27%//P15565
C-NT2RP4000634//Homo sapiens mitogen-activated protein kinase kinase kinase MEKK2 mRNA, complete cds.//0//1501bp//98%//AF111105
C-NT2RP4001001//Homo sapiens clone 24856 mRNA sequence, complete cds.//3.90E-301//1374bp//99%//AF131856
C-NT2RP4001877//Homo sapiens ribonucleoprotein RBM8 (RBM8) mRNA, complete cds.//0//2770bp//99%//AF127761
C-NT2RP4001879
C-NT2RP4002187//Homo sapiens steroid dehydrogenase homolog mRNA, complete cds.//0//2373bp//99%//AF078850
C-NT2RP4002451
C-NT2RP4002750//Mus musculus cationic amino acid transporter (CAT3) mRNA, complete cds.//1.00E-310//2084bp//81%//U70859
C-OVARC1000003//Homo sapiens sodium dependent phosphate transporter isoform NaPi-3b mRNA, complete cds.//4.30E-220//1158bp//94%//AF111856
C-OVARC1000313//Homo sapiens mRNA for KIAA0573 protein, partial cds.//0//1833bp//99%//AB011145
C-OVARC1000331//GMP REDUCTASE (EC 1.6.6.8) (GUANOSINE 5'-MONOPHOSPHATE OXIDOREDUCTASE).//9.40E-44//106aa//59%//P36959
C-OVARC1000553//DIPEPTIDYL PEPTIDASE IV (EC 3.4.14.5) (DPP IV) (THYMOCYTE-ACTIVATING MOLECULE) (THAM).//1.30E-23//169aa//40%//P28843
C-OVARC1000873//Homo sapiens mRNA for KIAA1247 protein, partial cds.//0//2178bp//99%//AB033073
C-OVARC1000995
C-OVARC1001260
C-OVARC1001336//Homo sapiens sodium dependent phosphate transporter isoform NaPi-3b mRNA, complete cds.//0//1435bp//99%//AF111856
C-OVARC1001570//Homo sapiens beta-site APP cleaving enzyme (BACE) mRNA, complete cds.//0//1792bp//100%//AF190725
C-OVARC1001607//Human beta-1,2-N-acetylglucosaminyltransferase II (MGAT2) gene, complete cds.//0//1836bp//96%//U15128
C-OVARC1001833//CIS-GOLGI MATRIX PROTEIN GM130.//6.60E-136//363aa//76%//Q62839 C-OVARC1001952//TRICHOHYALIN.//3.30E-16//487aa//27%//Q07283
C-PLACE1000986//Homo sapiens mRNA; cDNA DKFZp586C1817 (from clone DKFZp586C1817).//0//2055bp//9996//AL117450
C-PLACE1003407//Homo sapiens putative transmembrane protein (CLN5) mRNA, complete cds.//0//1965bp//99%//AF068227
C-PLACE1004078//Bovine mRNA for adseverin, complete cds.//0//2218bp//89%//D26549
C-PLACE1004492//VERPROLIN.//3.30E-07//149aa//29%//P37370
C-PLACE1005539//ACTIN POLYMERIZATION INHIBITOR (HEAT SHOCK 25 KD PROTEIN) (25-KD IAP).//3.10E-08//84aa//34%//Q00649
C-PLACE1005569//Homo sapiens mRNA for Z39Ig protein.//0//1768bp//99%//AJ132502
C-PLACE1005601
C-PLACE1005745//ORM1 PROTEIN.//2.40E-17//137aa//35%//P53224
C-PLACE1005815//COLORECTAL MUTANT CANCER PROTEIN (MCC PROTEIN).//1.50E-26//274aa//26%//P23508
C-PLACE1005927//HYPOTHETICAL 35.8 KD PROTEIN C12G12.12 IN CHROMOSOME I.//1.60E-38//333aa//33%//Q09875
C-PLACE1006071//LAMININ BETA-1 CHAIN PRECURSOR (LAMININ B1 CHAIN).//6.00E-08//215aa//26%//P02469
C-PLACE1006073//Homo sapiens mRNA for glucuronyltransferase I, complete cds.//4.10E-316//1020bp//96%//AB009598
C-PLACE1006079//Homo sapiens distal-less homeobox protein (DLX3) gene, complete cds.//0//1379bp//97%//AF028233
C-PLACE1006786
C-PLACE1007077//Homo sapiens mRNA for KIAA0977 protein, complete cds.//0//2578bp//99%//AB023194
C-PLACE1007971
C-PLACE1008282//HEME-REGULATED EUKARYOTIC INITIATION FACTOR EIF-2-ALPHA KINASE (EC 2.7.1.-) (HR)).//7.1OE-274//627aa//82%//P33279
C-PLACE1008359//BEM46 PROTEIN (FRAGMENT).//1.70E-50//289aa//42%//P54069 C-PLACE1008744//Homo sapiens mRNA full I length insert cDNA clone EUROIMAGE 248114.//0//1757bp//99%//AL079279
C-PLACE1010445
C-PLACE1010713//Homo sapiens steroid dehydrogenase homolog mRNA, complete cds.//0//2374bp//99%//AF078850
C-nnnnnnnnnnnn//Homo sapiens angiopoietin-2 mRNA, complete cds.//0//2227bp//99%//AF004327
C-PLACE1011181//MSP1 PROTEIN HOMOLOG.//9.40E-82//353aa//47%//P54815
C-PLACE1011364//MYOTONIN-PROTEIN KINASE (EC 2.7.1.-) (MYOTONIC DISTROPHY PROTEIN KINASE) (MDPK) (DM-KINASE) (DMK) (DMPK) (MT-PK).//2.20E-09//153aa//32%//Q09013
C-PLACE3000181//Human protocadherin 42 mRNA, complete cds for abbreviated PC42.//0//2719bp//95%//L11370
C-SKNMC1000014
C-SKNMC1000082//BRITTLE-1 PROTEIN PRECURSOR.//3.70E-31//307aa//30%//P29518
C-THYRO1000964
C-THYRO1001242//Homo sapiens cleft lip and palate transmembrane protein 1 (CLPTM1) mRNA, complete cds.//0//2468bp//99%//AF037339
C-THYRO1001608
C-THYRO1001641//Homo sapiens CGI-57 protein mRNA, complete cds.//0//1668bp//99%//AF151815
C-THYRO1001770//PROBABLE SERINE/THREONINE-PROTEIN KINASE YNL020C (EC 2.7.1.-).//6.30E-20//169aa//35%//P53974
C-Y79AA1000030//Homo sapiens mRNA for Fe65L2, complete cds.//0//1828bp//100%//AB018247
C-Y79AA1001212//Homo sapiens SL15 protein mRNA, complete cds.//6.30E-306//1388bp//99%//AF038961
C-Y79AA1001426//ANION EXCHANGE PROTEIN 3 (CARDIAC/BRAIN BAND 3-LIKE PROTEIN) (CAE3/BAE3).//6.20E-66//609aa//31%//P48751
C-Y79AA1001427//Homo sapiens cytochrome b5 reductase 1 (B5R.1) mRNA, complete cds.//0//1588bp//99%//AF169481
C-Y79AA1001523//Homo sapiens transcriptional intermediary factor 1 alpha mRNA, complete cds.//0//2263bp//99%//AF119042
C-Y79AA1001530//Human beta-tubulin gene (5-beta) with ten Alu family members.//0//1920bp//98%//X00734
C-Y79AA1001592
C-Y79AA1001727//CELL SURFACE A33 ANTIGEN PRECURSOR.//1.10E-13//286aa//27%//099795
C-Y79AA1001787//PROBABLE CALCIUM-TRANSPORTING ATPASE 9 (EC 3.6.1.38).//1.70E-133//544aa//37%//Q12697
C-Y79AA1001793//Mus musculus mRNA for GSG1, complete cds.//3.70E-126//532bp//78%//D87325
C-Y79AA1001795//Homo sapiens mRNA for GaIT4 protein.//2.30E-250//1137bp//99%//Y15061
C-Y79AA1001799//MITOCHONDRIAL RNA SPLICING PROTEIN MSR4.//3.40E-54//182aa//39%//P23500
C-Y79AA1001803//Homo sapiens secretogranin III mRNA, complete cds.//0//1871bp//99%//AF078851
C-Y79AA1001863
C-Y79AA1002058//Mus musculus Gng3Ig mRNA, complete cds.//4.10E-167//1145bp//83%//AF069954
C-Y79AA1002121//HISTONE H1.//4.90E-12//114aa//35%//P35060
C-Y79AA1002213//HYPOTHETICAL 52.7 KD PROTEIN C38C10.2 IN CHROMOSOME III.//1.20E-98//262aa//41%//Q03567
C-Y79AA1002373//Mus musculus mRNA for GSG1, complete cds.//7.20E-147//680bp//79%//D87325
C-Y79AA1002376//Rattus norvegicus cytoplasmic dynein intermediate chain 2B mRNA, complete cds.//1.50E-304//1667bp//90%//U39045
C-Y79AA1002378//Homo sapiens zinc finger protein NY-REN-21 antigen mRNA, partial cds.//0//963bp//99%//AF155100
C-Y79AA1002381//Homo sapiens cell cycle related kinase mRNA, complete cds.//0//1791bp//98%//AF035013
C-BNGH41000087//Homo sapiens mRNA for KIAA1247 protein, partial cds.//0//2294bp//99%//AB033073
C-HEMBA1001886
C-HEMBA1004067//Homo sapiens mRNA for KIAA0859 protein, complete cds.//8.30E-309//623bp//99%//AB020666
C-HEMBA1007226//Homo sapiens RPA-binding trans-activator (RBT1) mRNA, complete cds.//7.30E-273//1242bp//99%//AF192529
C-HEMBB1000309
C-HEMBB1000567
C-MAMMA1000102//APOLIPOPROTEIN L PRECURSOR (APO-L).//1.40E-21//221aa//35%//014791
C-MAMMA1001066
C-MAMMA1001094//Human potential transcriptional repressor NOT4Hp (NOT4H) mRNA, complete cds.//0//1394bp//93%//U71267
C-MAMMA1001609
C-MAMMA1001901
C-MAMMA1002091//Homo sapiens CD39L2 (CD39L2) mRNA, complete cds.//0//2564bp//99%//AF039916
C-NT2RM1000462
C-NT2RM1000542//BETA-GALACTOSIDASE PRECURSOR (EC 3.2.1.23) (LACTASE).//1.40E-103//566aa//43%//P48982
C-NT2RM1000789//Homo sapiens mRNA for hTCF-4.//2.80E-221//757bp//99%//Y11306
C-NT2RM1000855//Homo sapiens sec61 homolog mRNA, complete cds.//0//3541bp//99%//AF084458
C-NT2RM1000899//MITOCHONDRIAL RNA SPLICING PROTEIN MSR4.//1.10E-54//182aa//39%//P23500
C-NT2RP2000092//ZINC FINGER PROTEIN 136.//1.90E-117//419aa//54%//P52737
C-NT2RP2001538//Homo sapiens mRNA; cDNA DKFZp434K2235 (from clone DKFZp434K2235).//0//2139bp//99%//AL117513
C-NT2RP2001921
C-NT2RP2003138//5'-TG-3' INTERACTING FACTOR (HOMEOBOX PROTEIN TGIF).//2.10E-08//104aa//46%//P70284
C-NT2RP2003302//Homo sapiens GIOT-4 mRNA for gonadotropin inducible transcription repressor-4, complete cds.//0//2891bp//99%//AB021644
C-NT2RP2003950//Homo sapiens clone 24778 unknown mRNA.//0//1545bp//99%//AF070572
C-NT2RP2005535//ZINC FINGER PROTEIN 85 (ZINC FINGER PROTEIN HPF4) (HTF1).//1.90E-172//489aa//62%//Q03923
C-NT2RP2005774//Homo sapiens GIOT-4 mRNA for gonadotropin inducible transcription repressor-4, complete cds.//6.90E-224//1461bp//72%//AB021644
C-NT2RP3000148//ZINC FINGER PROTEIN 84 (ZINC FINGER PROTEIN HPF2).//4.10E-106//350aa//47%//P51523
C-NT2RP3000232//ZINC FINGER PROTEIN 184 (FRAGMENT).//1.40E-130//693aa//41%//Q99676
C-NT2RP3000427
C-NT2RP3000652//ZINC FINGER PROTEIN 43 (ZINC PROTEIN HTF6).//1.90E-153//441aa//62%//P28160
C-NT2RP3001650//BONE MORPHOGENETIC PROTEIN 1 PRECURSOR (EC 3.4.24.-) (BMP-1).//2.20E-22//107aa//42%//P98063
C-NT2RP3002409//Homo sapiens mRNA for KIAA0719 protein, complete cds.//0//2404bp//99%//AB018262
C-NT2RP3002411//Homo sapiens steroid dehydrogenase homolog mRNA, complete cds.//0//2374bp//99%//AF078850
C-NT2RP3003448
C-NT2RP4002715
C-OVARC1000307//HYPOTHETICAL 29.7 KD PROTEIN CY339.02.//3.00E-19//194aa//35%//Q50658
C-PLACE1000907//ZINC FINGER PROTEIN 83 (ZINC FINGER PROTEIN HPF1).//1.30E-204//396aa//85%//P51522
C-PLACE1007081
C-PLACE1010011//Homo sapiens AAPT1-like protein mRNA, partial cds.//1.70E-237//1092bp//99%//AF047431
C-PLACE3000213//COMPLEMENT RECEPTOR TYPE 2 PRECURSOR (CR2) (COMPLEMENT C3D RECEPTOR).//4.60E-68//317aa//34%//P19070
C-PLACE4000354//COMPLEMENT RECEPTOR TYPE 1 PRECURSOR (C3B/C4B RECEPTOR) (CD35 ANTIGEN).//1.00E-129//482aa//29%//P17927
C-PLACE4000455
C-THYRO1000776//PROBABLE SULFATE PERMEASE SPBC3H7.02.//1.30E-68//442aa//36%//074377
C-THYRO1001593//PUTATIVE SERINE/THREONINE-PROTEIN KINASE P78 (EC 2.7.1.-).//3.10E-203//550aa//62%//P27448
C-Y79AA1000750
C-Y79AA1000888//TRNA PSEUDOURIDINE SYNTHASE A (EC 4.2.1.70) (PSEUDOURIDYLATE SYNTHASE I) (PSEUDOURIDINE SYNTHASE I) (URACIL HYDROLYASE).//1.50E-21//267aa//32%//P70973
C-Y79AA1002129
C-Y79AA1002334//GLUCOSE REPRESSION MEDIATOR PROTEIN.//1.70E-10//333aa//23%//P14922
C-BNGH41000020//NADH-UBIQUINONE OXIDOREDUCTASE CHAIN 2 (EC 1.6.5.3).//9.80E-159//347aa//90%//P03891
C-BNGH41000087//N-ACETYLGLUCOSAMINE-6-SULFATASE PRECURSOR (EC 3.1.6.14) (G6S) (GLUCOSAM)NE-6-SULFATASE).//1.20E-17//83aa//40%//P50426
C-BNGH41000091//POTASSIUM CHANNEL PROTEIN EAG.//1.20E-249//625aa//65%//Q02280
C-HEMBA1000006//Homo sapiens mRNA for NESCA, complete cds.//0//1230bp//92%//AB026894
C-HEMBA1000121//HYPOTHETICAL 68.7 KD PROTEIN ZK757.1 IN CHROMOSOME III.//4.80E-05//83aa//27%//P34679
C-HEMBA1000128//PATHOGENESIS-RELATED PROTEIN 1 PRECURSOR (PR-1).//3.20E-07//89aa//34%//P33154
C-HEMBA1000275
C-HEMBA1000300
C-HEMBA1000349//ATP-BINDING CASSETTE TRANSPORTER 1.//5.30E-65//352aa//39%//P41233
C-HEMBA1000443//Homo sapiens CGI-96 protein mRNA, complete cds.//4.70E-129//686bp//91%//AF151854
C-HEMBA1000462//Human potential transcriptional repressor NOT4Hp (NOT4H) mRNA, complete cds.//0//935bp//99%//U71267
C-HEMBA1000477//HYPOTHETICAL 64.8 KD PROTEIN IN GDI1-COX15 INTERGENIC REGION.//1.40E-38//344aa//34%//P40085
C-HEMBA1000590//Homo sapiens mRNA for matrilin-4, partial.//2.00E-273//1254bp//99%//AJ007581
C-HEMBA1000634//Homo sapiens T-cell activation protein (PGR1) gene, complete cds.//0//994bp//99%//AF116272
C-HEMBA1000671//ZINC FINGER PROTEIN 184 (FRAGMENT).//3.90E-104//388aa//46%//Q99676
C-HEMBA1000713//Homo sapiens 10kD protein (BC10) mRNA, complete cds.//0//1254bp//99%//AF053470
C-HEMBA1000732//Homo sapiens mRNA for latent transforming growth factor-beta binding protein-4.//0//2153bp//94%//Y13622
C-HEMBA1000745//COLLAGEN ALPHA 1(I) CHAIN (FRAGMENTS).//2.00E-07//445aa//27%//P02454
C-HEMBA1000835//FIBRILLIN 2 PRECURSOR.//1.30E-42//214aa//45%//P35556
C-HEMBA1000875
C-HEMBA1000907
C-HEMBA1000940//GAP JUNCTION ALPHA-3 PROTEIN (CONNEXIN 44) (CX44).//2.90E-39//362aa//31%//P41987
C-HEMBA1000962
C-HEMBA1001184//SH3BGR PROTEIN (21-GLUTAMIC ACID-RICH PROTEIN) (21-GARP).//2.50E-32//100aa//60%//P55822
C-HEMBA1001221//AGRIN PRECURSOR.//2.50E-25//294aa//29%//P31696
C-HEMBA1001228//Human germline oligomeric matrix protein (COMP) mRNA, complete cds.//7.80E-286//1105bp//94%//L32137
C-HEMBA1001272//Homo sapiens mRNA for KIAA1171 protein, partial cds.//0//1490bp//99%//AB032997
C-HEMBA1001296
C-HEMBA1001297//Homo sapiens putative transcription factor CA150 mRNA, complete cds.//4.60E-276//1081bp//99%//AF017789
C-HEMBA1001390//Mus musculus polymerase I-transcript release factor mRNA, complete cds.//2.50E-57//464bp//82%//AF036249
C-HEMBA1001563
C-HEMBA1001621//PROBABLE G PROTEIN-COUPLED RECEPTOR APJ.//3.50E-123//259aa//89%//P35414
C-HEMBA1001878//Homo sapiens U5 snRNP-specific 40 kDa protein mRNA, complete cds.//0//1488bp//99%//AF090988
C-HEMBA1001886//ZINC FINGER PROTEIN 85 (ZINC FINGER PROTEIN HPF4) (HTF1).//1.40E-148//421aa//60%//Q03923
C-HEMBA1002048//ODD-SKIPPED PROTEIN.//1.60E-55//122aa//75%//P23803 C-HEMBA1002131//PROCOLLAGEN-LYSINE, 2-OXOGLUTARATE 5-DIOXYGENASE 1//4.10E-10//140aa//30%//P24802
C-HEMBA1002163//HYPOTHETICAL 40.7 KD PROTEIN IN DAK1-ORC1 INTERGENIC REGION.//9.40E-28//309aa//30%//Q04651
C-HEMBA1002164
C-HEMBA1002167//Rattus norvegicus neuroligin I mRNA, complete cds.//1.30E-305//1643bp//91%//U22952
C-HEMBA1002178//PROCOLLAGEN-LYSINE,2-OXOGLUTARATE 5-DIOXYGENASE 1 PRECURSOR (EC 1.14.11.4) (LYSYL HYDROXYLASE 1) (LH1).//3.70E-10//140aa//30%//P24802
C-HEMBA1002195//VEGETATIBLE INCOMPATIBILITY PROTEIN HET-E-1.//8.80E-23//221aa//31%//Q00808
C-HEMBA1002227//Homo sapiens mRNA for 80K-L protein, complete cds.//0//1324bp//98%//D10522
C-HEMBA1002239
C-HEMBA1002316//GTP-BINDING PROTEIN HFLX.//5.80E-12//196aa//29%//P25519
C-HEMBA1002420
C-HEMBA1002421//Human heparan sulfate proteoglycan (HSPG) core protein, 3' end.//0//2097bp//99%//J04621
C-HEMBA1002524//Human MHC Class I region proline rich protein mRNA, complete cds.//0//1763bp//95%//U63336
C-HEMBA1002551//PUTATIVE SERINE/THREONINE-PROTEIN KINASE PKWA (EC 2.7.1.-).//9.80E-08//110aa//37%//P49695
C-HEMBA1002767//Homo sapiens chromosome 1p33-p34 beta-1,4-galactosyltransferase mRNA, complete cds.//0//1497bp//99%//AF038660
C-HEMBA1002985
C-HEMBA1002992//UBIQUITIN-LIKE PROTEIN DSK2.//2.00E-21//216aa//35%//P48510 C-HEMBA1003047//Homo sapiens intrinsic factor-B12 receptor precursor, mRNA, complete cds.//0//1768bp//99%//AF034611
C-HEMBA1003072//Gallus gallus single-strand DNA-binding protein csdp mRNA, partial cds.//3.30E-93//927bp//73%//U68380
C-HEMBA1003101//Homo sapiens tyrosylprotein sulfotransferase-2 mRNA, complete cds.//0//1854bp//9996//AF049891
C-HEMBA1003120//ZINC FINGER PROTEIN 184 (FRAGMENT).//1.00E-193//547aa//54%//Q99676
C-HEMBA1003230//Homo sapiens fibulin-5.//5.60E-308//1398bp//99%//AJ133490
C-HEMBA1003294
C-HEMBA1003315//Mus musculus mRNA for DNA helicase, complete cds.//6.30E-250//1426bp//88%//AB013912
C-HEMBA1003392//Homo sapiens LDL receptor-related protein 6 (LRP6) mRNA, complete cds.//0//1721bp//10096//AF074264
C-HEMBA1003399//MVP1 PROTEIN.//2.30E-15//279aa//23%//P40959
C-HEMBA1003487
C-HEMBA1003497//ZINC FINGER PROTEIN 151 (POLYOMAVIRUS LATE INITIATOR PROMOTER BINDING PROTEIN) (LP-1) (ZINC FINGER PROTEIN Z13).//4.00E-28//358aa//29%//Q60821
C-HEMBA1003530//S. scrofa mRNA for BM88 antigen.//1.20E-60//900bp//66%//X82027
C-HEMBA1003602//HYPOTHETICAL 29.7 KD PROTEIN CY339.02.//2.80E-21//200aa//33%//050658
C-HEMBA1003732//SFT2 PROTE)N.//1.50E-06//162aa//30%//P38166
C-HEMBA1003945//Homo sapiens hypothetical 43.2 Kd protein mRNA, complete cds.//8.90E-287//757bp//97%//AF077030
C-HEMBA1004007
C-HEMBA1004067//Homo sapiens mRNA for KIAA0859 protein, complete cds.//0.00E+00//623bp//99%//AB020666
C-HEMBA1004085
C-HEMBA1004110//Homo sapiens intersectin short form mRNA, complete cds.//0//2033bp//99%//AF064243
C-HEMBA1004250//CADHERIN-RELATED TUMOR SUPPRESSOR PRECURSOR (FAT PROTE)N).//6.40E-51//277aa//35%//P33450
C-HEMBA1004391//TUMOR SUPPRESSOR PROTEIN DCC PRECURSOR.//5.60E-20//194aa//26%//P70211
C-HEMBA1004444//GLYCOPROTEIN 25L PRECURSOR (GP25L).//4.60E-41//148aa//52%//P27869
C-HEMBA1004454
C-HEMBA1004505//MANNOSYL-OLIGOSACCHARIDE ALPHA-1, 2-MANNOSIDASE ISOFORM 1 (EC 3.2.1.113) (MAN(9)-ALPHA-MANNOSIDASE).//2.70E-45//239aa//43%//P53624 C-HEMBA1004785//MODIFIER 3 PROTEIN (M33).//1.40E-27//221aa//33%//P30658
C-HEMBA1004797
C-HEMBA1004952
C-HEMBA1004971
C-HEMBA1004982//TETRACYCLINE RESISTANCE PROTEIN, CLASS E (TETA(E)).//6.30E-10//149aa//26%//Q07282
C-HEMBA1005070//SKIN SECRETORY PROTEIN XP2 PRECURSOR (APEG PROTEIN).//1.10E-05//187aa//29%//P17437
C-HEMBA1005084//NON-RECEPTOR TYROSINE KINASE SPORE LYSIS A (EC 2.7.1.112) (TYROSINE- PROTEIN KINASE 1).//1.20E-07//102aa//37%//P18160
C-HEMBA1005145
C-HEMBA1005230//ZINC FINGER PROTEIN 140.//2.00E-17//83aa//66%//P52738
C-HEMBA1005246//HEPATOCYTE NUCLEAR FACTOR 3 FORKHEAD HOMOLOG 4 (HFH-4).//2.10E-15//230aa//28%//092949
C-HEMBA1005267//ANKYRIN, BRAIN VARIANT 1 (ANKYRIN B) (ANKYRIN, NONERYTHROID).//8.40E-14//187aa//33%//Q01484
C-HEMBA1005430
C-HEMBA1005449//EXTENSIN PRECURSOR (CELL WALL HYDROXYPROLINE-RICH GLYCOPROTE)N).//5.40E-10//224aa//24%//P13983
C-HEMBA1005489//Mus musculus semaphorin cytoplasmic domain-associated protein 3A (Semcap3) mRNA, complete cds.//8.40E-255//924bp//80%//AF127084
C-HEMBA1005522//COAGULATION FACTOR VII PRECURSOR (EC 3.4.21.21).//7.70E-15//78aa//51%//P98139
C-HEMBA1005545//MUSCARINIC ACETYLCHOLINE RECEPTOR M3.//0//590aa//100%//P20309 C-HEMBA1005698//Homo sapiens vesicle trafficking protein (SEC22C) mRNA, complete cds.//6.60E-163//753bp//99%//AF039568
C-HEMBA1005913
C-HEMBA1005929//H.sapiens mRNA for serine/threonine protein kinase EMK.//6.50E-92//1092bp//69%//X97630
C-HEMBA1005945//BRITTLE-1 PROTEIN PRECURSOR.//1.70E-29//220aa//35%//P29518
C-HEMBA1006016
C-HEMBA1006171
C-HEMBA1006276//ZINC FINGER PROTEIN 90 (ZFP-90) (ZINC FINGER PROTEIN NK10).//1.70E-06//56aa//57%//Q61967
C-HEMBA1006299
C-HEMBA1006311
C-HEMBA1006335
C-HEMBA1006357//SECRETORY CARRIER-ASSOCIATED MEMBRANE PROTEIN 2.//3.70E-39//136aa//52%//015127
C-HEMBA1006430//Human putative transmembrane protein precursor (B5) mRNA, complete cds.//2.40E-70//1108bp//65%//L38961
C-HEMBA1006482//Homo sapiens h-sco1 (SC01) mRNA, nuclear gene encoding mitochondrial protein, complete cds.//0//1101bp//98%//AF026852
C-HEMBA1006517
C-HEMBA1006544
C-HEMBA1006572//ODD-SKIPPED PROTEIN.//2.60E-39//85aa//83%//P23803
C-HEMBA1006658//Homo sapiens mRNA for NIK, partial cds.//0//1500bp//9896//AB013385
C-HEMBA1006707//Homo sapiens mRNA for matrilin-4, partial.//0//2003bp//99%//AJ007581
C-HEMBA1006724
C-HEMBA1006749//Homo sapiens mRNA for matrilin-4, partial.//1.40E-275//1942bp//83%//AJ007581
C-HEMBA1006770//FLOWERING TIME CONTROL PROTEIN FCA.//1.20E-33//352aa//34%//004425
C-HEMBA1006902//Homo sapiens mRNA for matrilin-4, partial.//4.80E-275//1799bp//85%//AJ007581
C-HEMBA1006912
C-HEMBA1006916//Homo sapiens Grb14 mRNA, complete cds.//3.00E-277//1010bp//95%//L76687
C-HEMBA1006960
C-HEMBA1007013//Mus musculus sphingosine kinase (SPHK1b) mRNA, complete cds.//1.10E-14//412bp//63%//AF068749
C-HEMBA1007057
C-HEMBA1007063
C-HEMBA1007226//Homo sapiens RPA-binding trans-activator (RBT1) mRNA, complete cds.//7.30E-273//1242bp//99%//AF192529
C-HEMBA1007241//HYPOTHETICAL 24.5 KD PROTEIN IN SAP185-BCK1 INTERGENIC REGION.//2.70E-14//106aa//42%//P40857
C-HEMBA1007291
C-HEMBA1007332//Homo sapiens mRNA for unr-interacting protein.//6.40E-83//266bp//98%//AJ010025
C-HEMBB1000106//CELLULAR NUCLEIC ACID BINDING PROTEIN (CNBP).//1.60E-10//139aa//30%//P53996
C-HEMBB1000276
C-HEMBB1000309
C-HEMBB1000407
C-HEMBB1000447//Homo sapiens JWA protein mRNA, complete cds.//0//2059bp//99%//AF070523
C-HEMBB1000542//Mus musculus bromodomain-containing protein BP75 mRNA, complete cds.//2.60E-232//1452bp//85%//AF084259
C-HEMBB1000567
C-HEMBB1000642
C-HEMBB1000668//Homo sapiens mRNA for KIAA0893 protein, complete cds.//0//2375bp//99%//AB020700
C-HEMBB1000679//C.familiaris mRNA for TRAM-protein.//4.10E-210//1149bp//80%//X63678
C-HEMBB1000881//Danio rerio mRNA for MINDIN2, complete cds.//1.70E-67//948bp//66%//AB006085
C-HEMBB1000905//TRANSCRIPTIONAL REPRESSOR RCO-1.//1.00E-11//311aa//27%//P78706
C-HEMBB1001026//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC
INTERMEDIATE COMPONENT).//5.30E-11//142aa//30%//P32802
C-HEMBB1001048//SARCALUMENIN PRECURSOR.//6.50E-18//154aa//33%//P13666
C-HEMBB1001200
C-HEMBB1001407
C-HEMBB1001530//SLS1 PROTEIN PRECURSOR.//9.80E-10//273aa//27%//Q99158
C-HEMBB1001547//Homo sapiens CGI-02 protein mRNA, complete cds.//0//2311bp//99%//AF132937
C-HEMBB1001573
C-HEMBB1001847//NEUROGENIC PROTEIN BIG BRAIN.//4.70E-06//258aa//24%//P23645
C-HEMBB1001959//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//7.30E-14//97aa//38%//P25210
C-HEMBB1001978
C-HEMBB1002039
C-HEMBB1002041//Homo sapiens transmembrane protein TENB2 (TENB2) mRNA, complete cds.//0//1746bp//99%//AF179274
C-HEMBB1002051//Homo sapiens Ets transcription factor ESE-2b mRNA, complete cds.//1.30E-95//454bp//99%//AF115403
C-HEMBB1002120//UDP-N-ACETYLGLUCOSAMINE-PEPTIDE N-ACETYLGLUCOSAMINYLTRANSFERASE 110 KD SUBUNIT (EC 2.4.1.-) (O-GLCNAC
TRANSFERASE P110 SUBUNIT).//4.90E-.22//337aa//27%//P56558
C-HEMBB1002162//Homo sapiens genethonin 1 mRNA, complete cds.//8.30E-67//328bp//99%//AF062534
C-HEMBB1002228
C-HEMBB1002245//PROSTAGLANDIN F2-ALPHA RECEPTOR REGULATORY PROTEIN PRECURSOR (PROSTAGLANDIN F2-ALPHA RECEPTOR ASSOCIATED PROTE)N).//0//879aa//89%//Q62786
C-HEMBB1002302
C-HEMBB1002427//FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).//1.80E-70//221aa//50%//P16442
C-HEMBB1002465//ACYL-COA DEHYDROGENASE (EC 1.3.99.-).//2.30E-53//249aa//48%//P45857
C-HEMBB1002661//Homo sapiens cardiovascular helix-loop-helix factor 2 (CHF2) mRNA, complete cds.//0//2174bp//99%//AF176422
C-HEMBB1002663
C-HEMBB1002693
C-MAMMA1000046
C-MAMMA1000102//APOLIPOPROTEIN L PRECURSOR (APO-L).//1.40E-21//221aa//35%//014791
C-MAMMA1000106
C-MAMMA1000118
C-MAMMA1000141
C-MAMMA1000204//Homo sapiens dysferlin mRNA, complete cds.//0//2028bp//99%//AF075575
C-MAMMA1000226
C-MAMMA1000403//Homo sapiens CDK4-binding protein p34SEI1 (SEI1) mRNA, complete cds.//1.20E-255//1165bp//99%//AF117959
C-MAMMA1000449
C-MAMMA1000457//Human NADH-cytochrome b5 reductase mRNA, 3' end.//9.50E-79//829bp//71%//M16462
C-MAMMA1000473//HYPOTHETICAL 35.6 KD PROTEIN IN SPC1-ILV3 INTERGENIC REGION.//5.10E-45//299aa//34%//P47088
C-MAMMA1000496//MIC1 PROTEIN.//3.00E-25//202aa//33%//P53258
C-MAMMA1000528
C-MAMMA1000591//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N-ACETYLGALACTOSAMINYLTRANSFERASE) (GALNAC-T1).//1.20E-115//515aa//49%//007537 C-MAMMA1000614//Homo sapiens pseudouridine synthase 1 (PUS1) mRNA, partial cds.//2.10E-302//1370bp//99%//AF116238
C-MAMMA1000652
C-MAMMA1000681//PROBABLE G PROTEIN-COUPLED RECEPTOR EDG-1.//9.40E-82//311aa//52%//008530
C-MAMMA1000706
C-MAMMA1000788//Bos taurus P14 (p14) mRNA, complete cds.//3.90E-85//502bp//89%//AF037349
C-MAMMA1000810
C-MAMMA1000814
C-MAMMA1000881//Homo sapiens protein kinase (SGK3) mRNA, complete cds.//0//1292bp//100%//AF169035
C-MAMMA1000986
C-MAMMA1000994//Homo sapiens ISLR(immunoglobulin superfamily containing leucine-rich repeat) mRNA, complete cds, alternatively spliced transcript ISLR-2.//0//2211bp//99%//AB024536
C-MAMMA1001043//MRC OX-45 SURFACE ANTIGEN PRECURSOR (BCM1 SURFACE ANTIGEN) (BLAST-1) (CD48).//2.90E-12//239aa//28%//P10252
C-MAMMA1001066
C-MAMMA1001094//Human potential transcriptional repressor NOT4Hp (NOT4H) mRNA, complete cds.//0//1394bp//93%//U71267
C-MAMMA1001141
C-MAMMA1001150//PROTEIN KINASE C, MU TYPE (EC 2.7.1.-) (NPKC-MU).//7.50E-304//587aa//68%//Q15139
C-MAMMA1001237//MONOCARBOXYLATE TRANSPORTER 2 (MCT 2).//7.70E-64//196aa//41%//P53988
C-MAMMA1001284
C-MAMMA1001310//HYPOTHETICAL 57.3 KD TRP-ASP REPEATS CONTAINING PROTEIN IN
POM152- REC114 INTERGENIC REGION.//1.50E-67//441aa//37%//Q04225
C-MAMMA1001344
C-MAMMA1001418//HYPOTHETICAL PROTEIN H10519.//6.90E-27//181aa//38%//P44742
C-MAMMA1001532//ZINC FINGER PROTEIN 84 (ZINC FINGER PROTEIN HPF2).//5.60E-126//319aa//56%//P51523
C-MAMMA1001609//MYOSIN II HEAVY CHAIN, NON MUSCLE.//1.50E-26//204aa//38%//P05659
C-MAMMA1001615//NEUROGENIC DIFFERENTIATION FACTOR 1.//3.80E-11//90aa//42%//Q13562
C-MAMMA1001623
C-MAMMA1001634
C-MAMMA1001893
C-MAMMA1001901
C-MAMMA1001957
C-MAMMA1001978//Cimex lectularius apyrase (APY) mRNA, complete cds.//6.70E-19//988bp//56%//AF085499
C-MAMMA1002070//PLASMINOGEN (EC 3.4.21.7) (FRAGMENT).//1.10E-07//103aa//33%//Q01177
C-MAMMA1002080//RAS-RELATED PROTEIN RAB-13.//1.80E-29//208aa//37%//P51153
C-MAMMA1002087
C-MAMMA1002091//Homo sapiens CD39L2 (CD39L2) mRNA, complete cds.//0//2564bp//99%//AF039916
C-MAMMA1002095//CALCIUM-TRANSPORTING ATPASE 1 (EC 3.6.1.38) (P-TYPE CALCIUM ATPASE).//3.70E-222//867aa//52%//043108
C-MAMMA1002128//ABC1 PROTEIN HOMOLOG PRECURSOR.//2.50E-97//464aa//45%//Q92338
C-MAMMA1002142//NON-RECEPTOR TYROSINE KINASE SPORE LYSIS A (EC 2.7.1.112) (TYROSINE- PROTEIN KINASE 1).//9.80E-17//146aa//3596//P18160
C-MAMMA1002165//Homo sapiens connective tissue growth factor related protein WISP-2 (WISP2) mRNA, complete cds.//4.80E-60//382bp//89%//AF100780
C-MAMMA1002205//Homo sapiens mRNA; cDNA DKFZp586C091 (from clone DKFZp586C091).//2.00E-81//308bp//81%//AL050119
C-MAMMA1002224
C-MAMMA1002234//SIGNAL RECOGNITION PARTICLE 68 KD PROTEIN (SRP68).//0//627aa//96%//Q00004
C-MAMMA1002586//Homo sapiens alpha 1,2-mannosidase mRNA, complete cds.//0//2228bp//99%//AF148509
C-MAMMA1002633
C-MAMMA1003126//SARCALUMENIN PRECURSOR.//1.10E-51//388aa//32%//P13666
C-NT2RM1000462//ATRIAL GLAND-SPECIFIC ANTIGEN PRECURSOR (AGSA).//8.60E-14//104aa//40%//P15287
C-NT2RM1000542//BETA-GALACTOSIDASE PRECURSOR (EC 3.2.1.23) (LACTASE).//1.40E-103//566aa//43%//P48982
C-NT2RM1000580//GLYCOSYLTRANSFERASE ALG2 (EC 2.4.1.-).//8.50E-75//301aa//39%//P43636
C-NT2RM1000789//Homo sapiens mRNA for hTCF-4.//2.80E-221//757bp//99%//Y11306
C-NT2RM1000855//Homo sapiens sec61 homolog mRNA, complete cds.//0//3541bp//99%//AF084458
C-NT2RM1000858//tricarboxylate carrier [rats, liver, mRNA Partial, 2986 nt].//2.10E-98//1035bp//70%//S70011
C-NT2RM1000899//MITOCHONDRIAL RNA SPLICING PROTEIN MSR4.//1.10E-54//182aa//39%//P23500
C-NT2RM2000241
C-NT2RM2000306//PUTATIVE GTP-BINDING PROTEIN W08E3.3.//4.50E-130//362aa//68%//P91917
C-NT2RM2000410
C-NT2RM2000423//BETA-GALACTOSIDASE PRECURSOR (EC 3.2.1.23) (LACTASE).//1.80E-38//308aa//35%//P48982
C-NT2RM2000497//CHL1 PROTEIN.//9.90E-24//296aa//29%//P22516
C-NT2RM2000514//Homo sapiens F-box protein Fbx21 (FBX21) mRNA, complete cds.//4.40E-304//1374bp//99%//AF174601
C-NT2RM2000565//HYPOTHETICAL 85.7 KD PROTEIN C13G6.03 IN CHROMOSOME I.//4.40E-304//394aa//43%//Q09782
C-NT2RM2000582//Homo sapiens mRNA for KIAA1053 protein, partial cds.//0//2666bp//99%//AB028976
C-NT2RM2000589//Bos taurus myosin X, complete cds.//0//4376bp//84%//U55042
C-NT2RM2000622//Mus musculus F-box protein FBL10 mRNA, partial cds.//3.00E-203//915bp//91%//AF176524
C-NT2RM2000632//EXCISION REPAIR PROTEIN ERCC-6 (COCKAYNE SYNDROME PROTEIN CSB).//6.40E-62//183aa//47%//Q03468
C-NT2RM2000773//Homo sapiens KNSL4 and MAZ genes for kinesin-like DNA binding protein and Myc-associated zinc finger protein, complete cds.//3.00E-289//1092bp//99%//AB017335
C-NT2RM2001126//Homo sapiens mRNA for multi PDZ domain protein.//0//1600bp//99%//AJ001319
C-NT2RM2001558//Homo sapiens protein kinase A binding protein AKAP110 mRNA, complete cds.//0//2398bp//99%//AF093408
C-NT2RM2001626//FLIGHTLESS-I PROTEIN HOMOLOG.//4.30E-19//362aa//26%//P34268
C-NT2RM2001643
C-NT2RM2001738//SOF1 PROTEIN.//3.00E-110//325aa//47%//P33750
C-NT2RM2001792//Homo sapiens angiopoietin-related protein-2 mRNA, complete cds. //7.10E-149//995bp//86%//AF125175
C-NT2RM2001818//SIALIDASE (EC 3.2.1.18) (NEURAMINIDASE) (NA) (MAJOR SURFACE ANTIGEN).//4. 30E-11//488aa//26%//P23253
C-NT2RM2001902//Homo sapiens mRNA for PAK4 protein.//5.40E-216//988bp//99%//AJ011855
C-NT2RM2001939//Human G protein-coupled receptor GPR-NGA gene, complete cds.//0//1559bp//98%//U55312
C-NT2RM2001941//MUSCARINIC ACETYLCHOLINE RECEPTOR M1.//7.40E-38//193aa//34%//P08482
C-NT2RM4000100//Homo sapiens Leman coiled-coil protein (LCCP) mRNA, complete cds.//0//2678bp//99%//AF175966
C-NT2RM4000115
C-NT2RM4000198//BUTYROPHILIN PRECURSOR (BT).//5.10E-12//162aa//33%//Q13410
C-NT2RM4000284//Human IgG Fc receptor hFcRn mRNA, complete cds.//1.30E-257//603bp//96%//U12255
C-NT2RM4000295
C-NT2RM4000326//ZINC FINGER PROTEIN 84 (ZINC FINGER PROTEIN HPF2).//9.00E-100//434aa//43%//P51523
C-NT2RM4000417//SYNAPTOTAGMIN II.//2.70E-23//293aa//30%//P46097
C-NT2RM4000444//ANTIGEN PEPTIDE TRANSPORTER 1 (APT1).//1.70E-112//493aa//44%//P36370
C-NT2RM4000587
C-NT2RM4000593//HYPOTHETICAL 111.9 KD PROTEIN C22H10.03C IN CHROMOSOME I.//3.90E-27//576aa//24%//Q10297
C-NT2RM4000648//K-GLYPICAN PRECURSOR.//4.00E-193//531aa//6696//P51655
C-NT2RM4000761//CYTOCHROME C OXIDASE POLYPEPTIDE I (EC 1.9.3.1).//2.50E-245//306aa//91%//P00395
C-NT2RM4000965//PUTATIVE IMPORTIN BETA-4 SUBUNIT (KARYOPHERIN BETA-4 SUBUNIT). //9. 00E-44//520aa//29%//060100
C-NT2RM4000997//MONO- AND DIACYLGLYCEROL LIPASE PRECURSOR (EC 3.1.1.-) (MDGL).//1.80E-10//189aa//30%//P25234
C-NT2RM4001321
C-NT2RM4001325//CHONDROITIN 6-SULFOTRANSFERASE (EC 2.8.2.17) (C6ST).//2.90E-48//343aa//34%//Q92179
C-NT2RM4001377//R.norvegicus LL5 mRNA.//8.50E-236//990bp//87%//X74226
C-NT2RM4001735
C-NT2RM4001768//Homo sapiens CGI-82 protein mRNA, complete cds.//0//1925bp//99%//AF151840
C-NT2RM4001843//BETA-GALACTOSIDASE PRECURSOR (EC 3.2.1.23) (LACTASE).//6.20E-33//263aa//38%//P48982
C-NT2RM4002352//Homo sapiens hLRp105 mRNA for LDL receptor related protein 105, complete cds.//0//2184bp//99%//AB009462
C-NT2RP1000002
C-NT2RP1000050
C-NT2RP1000181//Homo sapiens delta-6 fatty acid desaturase mRNA, complete cds.//3.30E-121//1394bp//69%//AF126799
C-NT2RP1000239
C-NT2RP1000261//ORM1 PROTEIN.//2.40E-17//137aa//35%//P53224
C-NT2RP1000271//ZINC FINGER PROTEIN 85 (ZINC FINGER PROTEIN HPF4) (HTF1).//4.70E-199//547aa//66%//Q03923
C-NT2RP1000300//Human transporter protein (g17) mRNA, complete cds.//3.80E-26//758bp//62%//U49082
C-NT2RP1000325//H.sapiens gene for phosphate carrier.//0//439bp//98%//X77337
C-NT2RP1000448
C-NT2RP1000465//UBIQUITIN-LIKE PROTEIN SMT3.//5.10E-07//81aa//33%//P55857
C-NT2RP1000468//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//2.00E-13//97aa//38%//P25210
C-NT2RP1000551//Homo sapiens putative interferon-related protein (SM15) mRNA, partial cds.//0//1761bp//99%//U09585
C-NT2RP1000579//Human succinate dehydrogenase flavoprotein subunit (SDH) mRNA, complete cds.//0//1951bp//94%//L21936
C-NT2RP1000613//CARBONIC ANHYDRASE VI (EC 4.2.1.1) (SALIVARY) (CARBONATE DEHYDRATASE VI).//3.40E-52//304aa//40%//P08060
C-NT2RP1000679
C-NT2RP1000740
C-NT2RP1000903
C-NT2RP1000981//CELL SURFACE A33 ANTIGEN PRECURSOR.//3.60E-14//286aa//27%//Q99795
C-NT2RP1001004//F-SPONDIN PRECURSOR.//9.20E-43//322aa//35%//P35446
C-NT2RP1001020//SERINE/THREONINE-PROTEIN KINASE PAK-BETA (EC 2.7.1.-) (BETA-PAK) (P21-ACTIVATED KINASE 3) (CDC42/RAC EFFECTOR KINASE PAK-B).//9.70E-22//227aa//31%//Q61036
C-NT2RP1001031//VEGETATIBLE INCOMPATIBILITY PROTEIN HET-E-1.//3.40E-42//285aa//35%//Q00808
C-NT2RP1001563//TESTIS-SPECIFIC PROTEIN TPX-1 PRECURSOR (AUTOANTIGEN 1) (25 KD ACROSOMAL AUTOANTIGEN) (AA1).//9.70E-19//201aa//31%//Q60477
C-NT2RP2000092//ZINC FINGER PROTEIN 136.//1.90E-117//419aa//54%//P52737
C-NT2RP2000178//MITOCHONDRIAL LON PROTEASE HOMOLOG 1 PRECURSOR (EC 3.4.21.-).//2.40E-192//778aa//48%//P93647
C-NT2RP2000240
C-NT2RP2000394//Gallus gallus p52 pro-apototic protein mRNA, complete cds.//1.60E-90//956bp//70%//AF029071 C-NT2RP2000447//GOLGIN-95.//2.80E-33//99aa//66%//Q08379
C-NT2RP2000479
C-NT2RP2000514//Homo sapiens roundabout 2 (robo2) mRNA, partial cds.//3.00E-185//855bp//99%//AF040991
C-NT2RP2000533//Homo sapiens cornichon protein mRNA, complete cds.//1.30E-290//1324bp//99%//AF070654
C-NT2RP2000610//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//1.50E-13//97aa//38%//P25210
C-NT2RP2000616//EXTENSIN PRECURSOR (CELL WALL HYDROXYPROLINE-RICH GLYCOPROTEIN).//4.10E-12//323aa//30%//P13983
C-NT2RP2000649//Homo sapiens mRNA for Hs Ste24p, complete cds.//0//2847bp//99%//AB016068
C-NT2RP2000663
C-NT2RP2000694//Homo sapiens 5T4 oncofetal trophoblast glycoprotein gene.//0//2278bp//99%//AJ012159
C-NT2RP2000712//ZINC FINGER PROTEIN 135.//3.70E-87//296aa//53%//P52742
C-NT2RP2000739//ZINC FINGER PROTEIN 83 (ZINC FINGER PROTEIN HPF1).//7.50E-73//387aa//37%//P51522
C-NT2RP2000818//Homo sapiens xenotropic and polytropic murine leukemia virus receptor (X3) mRNA, complete cds.//0//2724bp//99%//AF089744
C-NT2RP2000903//Homo sapiens 5T4 oncofetal trophoblast glycoprotein gene.//0//2276bp//100%//AJ012159
C-NT2RP2001200//Homo sapiens mRNA for KIAA0676 protein, partial cds.//0//1539bp//10096//AB014576
C-NT2RP2001223//MYOTUBULARIN-RELATED PROTEIN 3 (FRAGMENT).//3.30E-05//76aa//39%//013615
C-NT2RP2001276//NPDC-1 PROTEIN PRECURSOR.//3.00E-133//331aa//77%//Q64322
C-NT2RP2001388//TRNA-SPLICING ENDONUCLEASE SUBUNIT SEN2 (EC 3.1.27.9) (TRNA-INTRON ENDONUCLEASE).//5.90E-13//157aa//33%//P16658
C-NT2RP2001469//VEGETATIBLE INCOMPATIBILITY PROTEIN HET-E-1.//1.30E-14//242aa//24%//000808
C-NT2RP2001480//Homo sapiens thrombospondin 3 (THBS3) gene, complete cds.//0//2547bp//99%//L38969
C-NT2RP2001495//Human transporter protein (g17) mRNA, complete cds.//2.20E-65//641bp//65%//U49082
C-NT2RP2001514//PROBABLE CALCIUM-TRANSPORTING ATPASE 6 (EC 3.6.1.38).//1.20E-133//429aa//41%//P39986
C-NT2RP2001529//Homo sapiens mRNA for ZIP-kinase, complete cds.//0//2079bp//99%//AB007144
C-NT2RP2001538//Mus musculus mSin3A (sin3A) mRNA, complete cds.//7.60E-272//1480bp//84%//U22394
C-NT2RP2001562//Homo sapiens GLE1 (GLE1) mRNA, complete cds.//0//1899bp//98%//AF058922
C-NT2RP2001662//HOMEODOMAIN PROTEIN AKR (AVIAN KNOTTED-RELATED PROTEIN).//1.80E-49//94aa//81%//Q90655
C-NT2RP2001755//Rattus norvegicus f-spondin mRNA, complete cds.//0//2974bp//86%//M88469
C-NT2RP2001769//SER)NE/THREON)NE-PROTE)N KINASE ORB6 (EC 2.7.1.-).//9.10E-47//185aa//44%//013310
C-NT2RP2001817//Homo sapiens sirtuin type 1 (SIRT1) mRNA, complete cds.//0//3092bp//99%//AF083106
C-NT2RP2001878
C-NT2RP2001903//CALPAIN, LARGE [CATALYTIC] SUBUNIT (EC 3.4.22.17) (CALCIUM-ACTIVATED NEUTRAL PROTEINASE) (CANP) (MU/M-TYPE).//3.80E-58//475aa//34%//P00789
C-NT2RP2001915
C-NT2RP2001921
C-NT2RP2001948//HST2 PROTEIN (HOMOLOGOUS TO SIR2 PROTEIN 2).//1.40E-08//191aa//27%//P53686
C-NT2RP2001956//ORM1 PROTEIN.//3.90E-19//137aa//37%//P53224
C-NT2RP2002015
C-NT2RP2002063//GNS1 PROTEIN.//3.60E-18//231aa//33%//P25358
C-NT2RP2002188//Rattus norvegicus neuroligin 3 mRNA, complete cds.//2.50E-226//1284bp//89%//U41663
C-NT2RP2002232//HYPOTHETICAL 85.7 KD PROTEIN C13G6.03 IN CHROMOSOME I.//1.90E-93//420aa//43%//Q09782
C-NT2RP2002304//Homo sapiens histone acetyltransferase MORF mRNA, complete cds.//0//2737bp//99%//AF113514
C-NT2RP2002409
C-NT2RP2002510
C-NT2RP2002527//CYTOCHROME B5.//1.30E-11//92aa//38%//P40312
C-NT2RP2002533//Homo sapiens alpha 2 delta calcium channel subunit isoform I mRNA, complete cds.//0//2365bp//99%//AF042792
C-NT2RP2002564//Human zinc-finger protein C2H2-150 mRNA, complete cds.//0//2237bp//99%//U38864
C-NT2RP2002674//SOLUBLE EPOXIDE HYDROLASE (SEH) (EC 3.3.2.3) (EPOX I DE HYDRATASE) (CYTOSOLIC EPOXIDE HYDROLASE) (CEH).//5.50E-38//201aa//39%//P34913 C-NT2RP2002721//REGULATORY PROTEIN UHPC.//1.60E-23//153aa//30%//P27669
C-NT2RP2002824//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//3.50E-63//404aa//33%//P32802
C-NT2RP2002942//Homo sapiens mRNA for KIAA0806 protein, complete cds.//0//2090bp//99%//AB018349
C-NT2RP2002974//HOMEOBOX PROTEIN SIX5 (DM LOCUS-ASSOCIATED HOMEODOMAIN PROTEIN HOMOLOG) (FRAGMENT).//8.20E-241//555aa//84%//P70178
C-NT2RP2002976//HYPOTHETICAL 149.7 KD PROTEIN IN IRE1-KSP1 INTERGENIC REGION.//1.30E-20//99aa//47%//P38800
C-NT2RP2003042//PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).//2.10E-109//385aa//52%//P53760
C-NT2RP2003138//5'-TG-3' INTERACTING FACTOR (HOMEOBOX PROTEIN TGIF).//2.10E-08//104aa//46%//P70284
C-NT2RP2003179//PUTATIVE SERINE/THREONINE-PROTEIN KINASE EMK (EC 2.7.1.-).//2.60E-67//256aa//49%//Q05512
C-NT2RP2003210//Homo sapiens fatty acid transport protein (FATP) mRNA, complete cds.//9.80E-272//1265bp//98%//AF055899
C-NT2RP2003302//Homo sapiens GIOT-4 mRNA for gonadotropin inducible transcription repressor-4, complete cds.//0//2891bp//99%//AB021644
C-NT2RP2003369//RAS SUPPRESSOR PROTEIN 1 (RSU-1) (RSP-1 PROTEIN) (RSP-1).//5.90E-20//204aa//34%//Q15404
C-NT2RP2003383//Homo sapiens mRNA for KIAA0458 protein, complete cds.//0//2565bp//99%//AB007927
C-NT2RP2003390//Homo sapiens mRNA for SEC63 protein.//0//2629bp//99%//AJ011779
C-NT2RP2003469//GALACTOSE-PROTON SYMPORT (GALACTOSE TRANSPORTER).//1.10E-45//324aa//29%//P37021
C-NT2RP2003545//Homo sapiens STE20-like kinase 3 (mst-3) mRNA, complete cds.//5.40E-48//578bp//71%//AF024636
C-NT2RP2003593
C-NT2RP2003599
C-NT2RP2003655//HYPOTHETICAL 26.3 KD PROTEIN IN OYE2-GND1 INTERGENIC//4.80E-15//93aa//47%//P38869
C-NT2RP2003664//Homo sapiens mRNA for leptin receptor gene-related protein.//1.90E-237//1081bp//99%//Y12670
C-NT2RP2003931
C-NT2RP2003940//ZINC FINGER PROTEIN 43 (ZINC PROTEIN HTF6).//7.00E-111//401aa//43%//P28160
C-NT2RP2003950//Homo sapiens clone 24778 unknown mRNA.//0//1545bp//99%//AF070572
C-NT2RP2004069
C-NT2RP2004108//ZINC FINGER PROTEIN ZFP-36 (FRAGMENT).//3.30E-171//474aa//62%//P16415
C-NT2RP2004141
C-NT2RP2004179
C-NT2RP2004205//BUTYROPHILIN PRECURSOR (BT).//1.60E-21//276aa//32%//Q62556
C-NT2RP2004447
C-NT2RP2004495//Human transporter protein (g17) mRNA, complete cds.//9.80E-64//642bp//64%//U49082
C-NT2RP2004524
C-NT2RP2004556
C-NT2RP2004606//Human fibroblast collagenase inhibitor mRNA, complete cds.//2.10E-166//768bp//99%//M12670
C-NT2RP2004648//Mouse beta-galactosidase (BGAL) gene, complete cds.//1.20E-33//1136bp//59%//M57734
C-NT2RP2004670//Rattus norvegicus vesicla-associate calmodulin-binding protein mRNA, complete cds.//0//1250bp//86%//L22557
C-NT2RP2004794//HYPOTHETICAL 24.5 KD PROTEIN IN SAP185-BCK1 INTERGENIC REGION.//2.70E-09//203aa//26%//P40857
C-NT2RP2004837
C-NT2RP2004847//ZINC FINGER PROTEIN 135.//8.00E-35//193aa//40%//P52742
C-NT2RP2005027//GLUCOSE TRANSPORTER TYPE 3, BRAIN.//6.20E-67//130aa//100%//P11169
C-NT2RP2005069//Rat vacuolar protein sorting homolog r-vps33b mRNA, complete cds.//0//1792bp//87%//U35245
C-NT2RP2005163
C-NT2RP2005181//Mus musculus cationic amino acid transporter (CAT3) mRNA, complete cds.//5.30E-315//2126bp//81%//U70859
C-NT2RP2005247//ZINC-FINGER PROTEIN RFP (RET FINGER PROTEIN).//5.00E-53//296aa//37%//Q62158
C-NT2RP2005378//GLYCINE-RICH CELL WALL STRUCTURAL PROTEIN 1.8 PRECURSOR (GRP 1.8).//6.30E-28//183aa//47%//P10496
C-NT2RP2005391//Homo sapiens partial mRNA for putative p621 protein which interacts with transcription factor Sp1.//0//1544bp//99%//AJ242978
C-NT2RP2005425//Homo sapiens mRNA for AKAP450 prote i n.//0//4341bp//99%//AJ131693
C-NT2RP2005463//PROTEIN PTM1 PRECURSOR.//7.40E-15//284aa//28%//P32857
C-NT2RP2005514
C-NT2RP2005535//ZINC FINGER PROTEIN 85 (ZINC FINGER PROTEIN HPF4) (HTF1).//1.90E-172//489aa//62%//003923
C-NT2RP2005541//N-ACETYLGLUCOSAMINE-6-SULFATASE PRECURSOR (EC 3.1.6.14) (G6S) (GLUCOSAMINE-6-SULFATASE).//4.70E-24//78aa//51%//P15586
C-NT2RP2005597//Homo sapiens protein O-mannosyl-transferase 1 (POMT1) mRNA, complete cds.//0//1821bp//97%//AF095136
C-NT2RP2005632
C-NT2RP2005666
C-NT2RP2005774//Homo sapiens GIOT-4 mRNA for gonadotropin inducible transcription repressor-4, complete cds.//6.90E-224//1461bp//72%//AB021644
C-NT2RP2005878//PUTATIVE STEROID DEHYDROGENASE KIK-I (EC 1.1.1.-).//3.60E-55//238aa//50%//057314
C-NT2RP2005883//DOPAMINE-BETA-MONOOXYGENASE PRECURSOR (EC 1.14.17.1) (DOPAMINE BETA- HYDROXYLASE) (DBH).//6.70E-72//512aa//34%//P15101
C-NT2RP2005887
C-NT2RP2005941//Human paired box gene (PAX6) homologue, complete cds.//1.40E-308//1396bp//99%//M93650
C-NT2RP2005994//HYPOTHETICAL 51.5 KD PROTEIN D2024.3 IN CHROMOSOME IV.//3.50E-35//144aa//47%//P49191
C-NT2RP2006004//FIBROMODULIN PRECURSOR (FM) (COLLAGEN-BINDING 59 KD PROTEIN).//3.00E-26//227aa//36%//Q06828
C-NT2RP2006042//GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).//9.40E-15//501aa//25%//P08640
C-NT2RP2006092//Homo sapiens mRNA for Fe65L2, complete cds.//0//1156bp//99%//AB018247
C-NT2RP2006099
C-NT2RP2006134
C-NT2RP2006269//DOLICHYL-PHOSPHATE-MANNOSE―PROTEIN MANNOSYLTRANSFERASE 2 (EC 2.4.1.109).//2.30E-78//679aa//32%//P31382
C-NT2RP2006512//GNS1 PROTEIN.//2.00E-21//290aa//29%//P25358
C-NT2RP2006580//Homo sapiens transitional epithelia response protein (TERE1) mRNA, complete cds.//0//1483bp//99%//AF117064
C-NT2RP3000011//VEGETATIBLE INCOMPATIBILITY PROTEIN HET-E-1.//4.00E-14//320aa//24%//Q00808
C-NT2RP3000022//Rat heart mRNA serine/threonine protein kinase, complete cds.//4.80E-203//1496bp//78%//D26178
C-NT2RP3000059//ANKYRIN, BRAIN VARIANT 2 (ANKYRIN B) (ANKYRIN, NONERYTHROID) (FRAGMENT).//3.70E-12//133aa//32%//Q01485
C-NT2RP3000063//NEUROFILAMENT TRIPLET H PROTEIN (200 KD NEUROFILAMENT PROTEIN) (NF-H).//5.00E-29//596aa//30%//P19246
C-NT2RP3000125//RETINOBLASTOMA BINDING PROTEIN 2 (RBBP-2).//6.30E-08//70aa//41%//P29375
C-NT2RP3000148//ZINC FINGER PROTEIN 84 (ZINC FINGER PROTEIN HPF2).//4.10E-106//350aa//47%//P51523
C-NT2RP3000169//Homo sapiens MRS1 mRNA, complete cds.//0//1519bp//97%//AF093239
C-NT2RP3000171//Mus musculus partial mRNA for B-IND1 protein (B-ind1 gene).//4.40E-99//571bp//89%//Z97207
C-NT2RP3000172//CALCIUM/CALMODULIN-DEPENDENT PROTEIN KINASE TYPE I (EC 2.7.1.123) (CAM KINASE )).//1.30E-80//359aa//44%//014012
C-NT2RP3000201//Homo sapiens HPK/GCK-like kinase HGK mRNA, complete cds.//1.30E-270//1231bp//99%//AF096300
C-NT2RP3000232//ZINC FINGER PROTEIN 184 (FRAGMENT).//1.40E-130//693aa//41%//Q99676
C-NT2RP3000304//Homo sapiens LDL receptor-related protein 6 (LRP6) mRNA, complete cds.//0//2895bp//99%//AF074264
C-NT2RP3000378//PAIRED AMPHIPATHIC HELIX PROTEIN.//4.20E-39//186aa//36%//P22579
C-NT2RP3000427
C-NT2RP3000436//PROBABLE PROTEIN DISULFIDE ISOMERASE P5 PRECURSOR (EC 5.3.4.1).//6.20E-27//90aa//42%//P38660
C-NT2RP3000444
C-NT2RP3000460//Canis familiaris sec61 homologue mRNA, complete cds.//1.80E-198//643bp//89%//M96629
C-NT2RP3000481//Homo sapiens RanBP7/importin 7 mRNA, complete cds.//0//2623bp//100%//AF098799
C-NT2RP3000616//FIBROMODULIN PRECURSOR (FM) (COLLAGEN-BINDING 59 KD PROTEIN).//5.20E-26//227aa//36%//Q06828
C-NT2RP3000645
C-NT2RP3000652//ZINC FINGER PROTEIN 43 (ZINC PROTEIN HTF6).//1.90E-153//441aa//62%//P28160
C-NT2RP3000676//PUTATIVE MITOCHONDRIAL CARRIER YMR166C.//2.10E-15//220aa//27%//Q03829
C-NT2RP3000677//DNA BINDING PROTEIN RFX2.//3.60E-56//233aa//41%//P48378
C-NT2RP3000721//HYPOTHETICAL 149.7 KD PROTEIN IN IRE1-KSP1 INTERGENIC REGION.//1.10E-22//171aa//36%//P38800
C-NT2RP3000789//Homo sapiens IGF-II mRNA-binding protein 1 (IMP-1) mRNA, complete cds.//0//1458bp//100%//AF117106
C-NT2RP3000818
C-NT2RP3000820//PUTATIVE SERINE/THREONINE-PROTEIN KINASE PKWA (EC 2.7.1.-).//1.90E-30//269aa//33%//P49695
C-NT2RP3000838//TRICHOHYALIN.//9.80E-11//491aa//26%//Q07283
C-NT2RP3000871
C-NT2RP3000907//PROBABLE CALCIUM-TRANSPORTING ATPASE 6 (EC 3. 6. 1. 38).//2. 20E-134//296aa//42%//P39986
C-NT2RP3000921//TUMOR SUPPRESSOR PROTEIN DCC PRECURSOR (COLORECTAL CANCER SUPPRESSOR).//4.00E-21//316aa//29%//P43146
C-NT2RP3001012//Homo sapiens mRNA for KIAA0829 protein, partial cds.//0//2906bp//98%//AB020636
C-NT2RP3001044
C-NT2RP3001061//Homo sapiens mRNA for KIAA0853 protein, partial cds.//0//3591bp//99%//AB020660
C-NT2RP3001159//Homo sapiens mRNA: cDNA DKFZp586C201 (from clone DKFZp586C201).//0//2558bp//99%//AL050118
C-NT2RP3001170//Mus musculus activity-dependent neuroprotective protein (Adnp) mRNA, complete cds.//4.80E-240//850bp//88%//AF068198
C-NT2RP3001195//GALACTOSE-PROTON SYMPORT (GALACTOSE TRANSPORTER).//4.70E-48//339aa//29%//P37021
C-NT2RP3001240//Canis familiaris sec61 homologue mRNA, complete cds.//1.20E-301//1141bp//89%//M96629
C-NT2RP3001271//NON-GREEN PLASTID TRIOSE PHOSPHATE TRANSLOCATOR PRECURSOR (CTPT).//2.60E-09//334aa//22%//P52178
C-NT2RP3001322//PROBABLE CALCIUM-TRANSPORTING ATPASE 3 (EC 3.6.1.38) (ENDOPLASMIC RETICULUM CA2+-ATPASE).//1.70E-21//220aa//30%//P39524 C-NT2RP3001388//SYNAPTOTAGMIN IV.//2.00E-118//430aa//54%//P50232
C-NT2RP3001542//TUMOR NECROSIS FACTOR, ALPHA-INDUCED PROTEIN 1, ENDOTHELIAL (B12 PROTE)N).//4.70E-11//132aa//37%//Q13829
C-NT2RP3001560//Homo sapiens cleft lip and palate transmembrane protein 1 (CLPTM1) mRNA, complete cds.//0//2468bp//99%//AF037339
C-NT2RP3001592//N2,N2-DIMETHYLGUANOSINE TRNA METHYLTRANSFERASE PRECURSOR (EC 2.1.1.32).//1.30E-18//279aa//27%//P15565.
C-NT2RP3001650//BONE MORPHOGENETIC PROTEIN 1 PRECURSOR (EC 3.4.24.-) (BMP-1).//2.20E-22//107aa//42%//P98063
C-NT2RP3001685//PRPE PROTEIN.//1.00E-68//382aa//41%//P77495
C-NT2RP3001738//CYTOCHROME B5.//1.30E-11//133aa//33%//P00169
C-NT2RP3001754
C-NT2RP3001858
C-NT2RP3001976//ZINC FINGER PROTEIN 84 (ZINC FINGER PROTEIN HPF2).//8.90E-143//379aa//55%//P51523
C-NT2RP3002015//Homo sapiens CGI-71 protein mRNA, complete cds.//0//1991bp//99%//AF151829
C-NT2RP3002160//HISTO-BLOOD GROUP ABO SYSTEM TRANSFERASE (NAGAT) [INCLUDES: GLYCOPROTEIN-FUCOSYLGALACTOSIDE ALPHA-N- ACETYLGALACTOSAMINYLTRANSFERASE (EC 2.4.1.40) (FUCOSYLGLYCOPROTEIN ALPHA-N-ACETYLGALACTOSAMINYLTRANSFERASE) (HISTO-BLOOD GROUP A TRANSFERASE) (A TRANSFERASE): GLYCOPROTEIN-FUCOSYLGALACTOSIDE ALPHA- GALACTOSYLTRANSFERASE (EC 2.4.1.37) (FUCOSYLGLYCOPROTEIN 3-ALPHA- GALACTOSYLTRANSFERASE) (HISTO-BLOOD GROUP B TRANSFERASE) (B TRANSFERASE)].//3.50E-72//231aa//49%//P16442
C-NT2RP3002281//Homo sapiens mRNA for KIAA0765 protein, partial cds.//0//2286bp//99%//AB018308
C-NT2RP3002286//Homo sapiens candidate tumor suppressor protein DICE1 mRNA, complete cds.//0//2719bp//99%//AF097645
C-NT2RP3002311//BETA-GALACTOSIDASE PRECURSOR (EC 3.2.1.23) (LACTASE).//9.80E-103//547aa//43%//P48982
C-NT2RP3002324
C-NT2RP3002342//Human transporter protein (g17) mRNA, complete cds.//1.70E-65//641bp//65%//U49082
C-NT2RP3002353
C-NT2RP3002409//Homo sapiens mRNA for KIAA0719 protein, complete cds.//0//2404bp//99%//AB018262
C-NT2RP3002411//Homo sapiens steroid dehydrogenase homolog mRNA, complete cds.//0//2374bp//99%//AF078850
C-NT2RP3002448
C-NT2RP3002571//Bos taurus mRNA for lyncein.//7.30E-169//1115bp//84%//Y17923
C-NT2RP3002664
C-NT2RP3002721//CITRATE SYNTHASE, MITOCHONDRIAL PRECURSOR (EC 4.1.3.7).//5.80E-249//466aa//98%//075390
C-NT2RP3002737//Homo sapiens voltage-gated potassium channel KCNQ4 (KCNQ4) mRNA, complete cds.//0//1552bp//99%//AF105202
C-NT2RP3002738//Homo sapiens enhancer of filamentation (HEF1) mRNA, complete cds.//2.20E-47//763bp//65%//L43821
C-NT2RP3002790
C-NT2RP3002836//Homo sapiens mRNA for KIAA0463 protein, partial cds.//0//1617bp//9996//AB007932
C-NT2RP3002887
C-NT2RP3002900//Homo sapiens CGI-109 protein mRNA, complete cds.//8.70E-298//1321bp//92%//AF151867
C-NT2RP3002958//TRANS-GOLGI NETWORK INTEGRAL MEMBRANE PROTEIN TGN38 PRECURSOR.//8.00E-08//197aa//26%//P19814
C-NT2RP3002983
C-NT2RP3003000//Homo sapiens T-type calcium channel alpha-1 subunit mRNA, complete cds.//0//3160bp//96%//AF051946
C-NT2RP3003076
C-NT2RP3003354//SECRETORY CARRIER-ASSOCIATED MEMBRANE PROTEIN 3.//5.10E-55//208aa//51%//035609
C-NT2RP3003448
C-NT2RP3003469
C-NT2RP3003473//Homo sapiens CGI-55 protein mRNA, complete cds.//5.50E-275//1309bp//98%//AF151813
C-NT2RP3003527//Homo sapiens mRNA for protein kinase Dyrk1B.//0//2483bp//99%//Y17999
C-NT2RP3003532//OX-2 MEMBRANE GLYCOPROTEIN PRECURSOR (FRAGMENT).//4.40E-139//263aa//99%//P41217
C-NT2RP3003535//UDP-N-ACETYLGLUCOSAMINE-PEPTIDE N-ACETYLGLUCOSAMINYLTRANSFERASE 110 KD SUBUNIT (EC 2.4.1.-) (O-GLCNAC TRANSFERASE P110 SUBUNIT).//8.80E-18//368aa//25%//P56558
C-NT2RP3003559
C-NT2RP3003614
C-NT2RP3003729//HYPOTHETICAL 42.1 KD PROTEIN IN SNZ1-YPK2 INTERGENIC REGION.//5.80E-17//204aa//30%//Q03151
C-NT2RP3003849//PROTEIN KINASE C, BRAIN ISOZYME (EC 2.7.1.-) (PKC) (DPKC53E(BR)). PKC1.//1.20E-13//126aa//34%//P05130
C-NT2RP3003874//Homo sapiens incomplete cDNA for a mutated allele of a myosin class I, myh-1c.//0//2160bp//98%//AJ001381
C-NT2RP3003963
C-NT2RP3004000
C-NT2RP3004025
C-NT2RP3004067//Homo sapiens mRNA for NESCA, complete cds.//0//1962bp//99%//AB026894
C-NT2RP3004075
C-NT2RP3004083
C-NT2RP3004090//GOLIATH PROTEIN (G1 PROTEIN).//9.00E-33//179aa//47%//Q06003 C-NT2RP3004119//PEREGRIN (BR140 PROTEIN).//7.30E-39//227aa//43%//P55201
C-NT2RP3004130//CELL SURFACE ANTIGEN 114/A10 PRECURSOR.//8.10E-06//71aa//42%//P19467
C-NT2RP3004133//GLYCOSYLTRANSFERASE ALG2 (EC 2.4.1.-).//2.50E-48//198aa//37%//P43636
C-NT2RP3004202
C-NT2RP3004294//Xenopus laevis ER1 mRNA, complete cds.//1.20E-71//335bp//79%//AF015454
C-NT2RP3004309//Homo sapiens mRNA; cDNA DKFZp586C201 (from clone DKFZp586C201).//0//2584bp//99%//AL050118
C-NT2RP3004321
C-NT2RP3004345//N2, N2-DIMETHYLGUANOSINE TRNA METHYLTRANSFERASE PRECURSOR (EC 2.1.1.32).//3.90E-18//279aa//27%//P15565
C-NT2RP3004355
C-NT2RP3004374
C-NT2RP3004406//HYPOTHETICAL 37.4 KD PROTEIN IN IRR1-TIM44 INTERGENIC REGION.//3.20E-15//165aa//33%//P40544
C-NT2RP3004481//BUTYROPHILIN PRECURSOR (BT).//8.50E-22//276aa//32%//Q62556
C-NT2RP3004552//COMPLEMENT RECEPTOR TYPE 1 PRECURSOR (C3B/C4B RECEPTOR) (CD35 ANTIGEN).//8.50E-24//263aa//33%//P17927
C-NT2RP3004557//Human Ki nuclear autoantigen mRNA, complete cds.//0//2181bp//96%//U11292
C-NT2RP3004625//Homo sapiens 1-1 receptor candidate protein mRNA, complete cds.//0//1339bp//99%//AF082516
C-NT2RP3004640//Bos taurus tuftelin mRNA, complete cds.//0//1204bp//88%//AF105228
C-NT2RP3004647//PUTATIVE MITOCHONDRIAL CARRIER YMR166C.//2.OOE-15//220aa//27%//Q03829
C-NT2RP4000108//Human gene for neurofilament subunit NF-L.//0//1998bp//99%//X05608
C-NT2RP4000634//Homo sapiens mitogen-activated protein kinase kinase kinase MEKK2 mRNA, complete cds.//0//1501bp//98%//AF111105
C-NT2RP4000962//SPORULATION-SPECIFIC PROTEIN 1 (EC 2.7.1.-).//2.60E-18//225aa//32%//P08458
C-NT2RP4001001//Homo sapiens clone 24856 mRNA sequence, complete cds.//3.90E-301//1374bp//99%//AF131856
C-NT2RP4001009//Homo sapiens mRNA for farnesylated-proteins converting enzyme 1.//0//2965bp//99%//Y13834
C-NT2RP4001467//Human placental cDNA coding for 5'nucleotidase (EC 3.1.3.5).//0//2140bp//99%//X55740
C-NT2RP4001877//Homo sapiens ribonucleoprotein RBM8 (RBM8) mRNA, complete cds.//0//2770bp//99%//AF127761
C-NT2RP4001879
C-NT2RP4002187//Homo sapiens steroid dehydrogenase homolog mRNA, complete cds.//0//2373bp//99%//AF078850
C-NT2RP4002451
C-NT2RP4002715
C-NT2RP4002750//Mus musculus cationic amino acid transporter (CAT3) mRNA, complete cds.//1.00E-310//2084bp//81%//U70859
C-OVARC1000003//Homo sapiens sodium dependent phosphate transporter isoform NaPi-3b mRNA, complete cds.//4.30E-220//1158bp//94%//AF111856
C-OVARC1000090
C-OVARC1000105//UBIQUITIN-CONJUGATING ENZYME E2-28.4 KD (EC 6.3.2.19) (UBIQUITIN- PROTEIN LIGASE) (UBIQUITIN CARRIER PROTEIN).//4.20E-47//171aa//56%//P33296
C-OVARC1000137
C-OVARC1000208//Human calcium-dependent group X phospholipase A2 mRNA, complete cds//1.50E-61//365bp//90%//U95301
C-OVARC1000255//H.sapiens syk mRNA for protein-tyrosine kinase.//0//1525bp//97%//Z29630
C-OVARC1000275//DESMOPLAKIN I AND II (DPI AND DPII) (FRAGMENT).//9.90E-16//352aa//23%//P15924
C-OVARC1000298
C-OVARC1000307//HYPOTHETICAL 29.7 KD PROTEIN CY339.02.//3.00E-19//194aa//35%//Q50658
C-OVARC1000313//PROTEIN DISULFIDE ISOMERASE PRECURSOR (PDI) (EC 5.3.4.1).//3.00E-24//353aa//27%//Q12730
C-OVARC1000331//GMP REDUCTASE (EC 1.6.6.8) (GUANOSINE 5'-MONOPHOSPHATE OXIDOREDUCTASE).//9.40E-44//106aa//59%//P36959
C-OVARC1000410//Homo sapiens angiopoietin Y1 mRNA, complete cds.//2.10E-63//744bp//69%//AF107253
C-OVARC1000439//HYPOTHETICAL 64.3 KD GTP-BINDING PROTEIN C02F5.3 IN CHROMOSOME III.//1.40E-33//143aa//53%//P34280
C-OVARC1000467
C-OVARC1000529//PROBABLE SERINE/THREONINE-PROTEIN KINASE YKL101W (EC 2.7.1.-).//1.40E-23//165aa//39%//P34244
C-OVARC1000553//DIPEPTIDYL PEPT I DASE IV (EC 3.4.14.5) (DPP IV) (THYMOCYTE-ACTIVATING MOLECULE) (THAM).//1.30E-23//169aa//40%//P28843
C-OVARC1000775
C-OVARC1000811//PLASMINOGEN (EC 3.4.21.7) (FRAGMENT).//6.40E-13//115aa//34%//Q01177
C-OVARC1000853
C-OVARC1000873//N-ACETYLGLUCOSAMINE-6-SULFATASE PRECURSOR (EC 3.1.6.14) (G6S) (GLUCOSAMINE-6-SULFATASE).//1.00E-09//83aa//40%//P50426 C-OVARC1000916//H.sapiens PISSLRE mRNA.//7.30E-280//1117bp//95%//X78342
C-OVARC1000956//SKIN SECRETORY PROTEIN XP2 PRECURSOR (APEG PROTEIN).//2.20E-09//250aa//28%//P17437
C-OVARC1000995
C-OVARC1001030//Homo sapiens mRNA for KIAA0886 protein, complete cds.//0//907bp//99%//AB020693
C-OVARC1001049//TRANSCRIPTION FACTOR HES-1 (C-HAIRY1).//7.50E-14//96aa//36%//057337
C-OVARC1001086//Homo sapiens cyclin T2a mRNA, complete cds.//0//1593bp//98%//AF048731
C-OVARC1001132//GC-RICH SEQUENCE DNA-BINDING FACTOR (GCF) (TRANSCRIPTION FACTOR 9)(TCF-9).//2.30E-44//268aa//36%//P16383
C-OVARC1001163//HYPOTHETICAL 49.3 KD PROTEIN C30D11.06C IN CHROMOSOME I.//2.30E-20//152aa//30%//Q09906
C-OVARC1001222
C-OVARC1001260
C-OVARC1001336//Homo sapiens sodium dependent phosphate transporter isoform NaPi-3b mRNA, complete cds.//0//1435bp//99%//AF111856
C-OVARC1001338//AUTOPHAGY SERINE/THREONINE-PROTEIN KINASE APG1 (EC 2.7.1.-).//8.80E-30//125aa//40%//P53104
C-OVARC1001569//PROBABLE SERINE/THREONINE-PROTEIN KINASE YKL101W (EC 2.7.1.-).//1.50E-22//164aa//39%//P34244
C-OVARC1001570//Homo sapiens beta-site APP cleaving enzyme (BACE) mRNA, complete cds.//0//1792bp//100%//AF190725
C-OVARC1001596//Homo sapiens Arf-like 2 binding protein BART1 mRNA, complete cds.//0//1766bp//99%//AF126062
C-OVARC1001607//Human beta-1,2-N-acetylglucosaminyltransferase II (MGAT2) gene, complete cds.//0//1836bp//96%//U15128
C-OVARC1001725//Homo sapiens patched related protein TRC8 (TRC8) gene, exon 1 and partial cds.//0//1624bp//99%//AF064800
C-OVARC1001727
C-OVARC1001807//Human TR3 orphan receptor mRNA, complete cds.//1.10E-243//1145bp//98%//L13740
C-OVARC1001833//CIS-GOLGI MATRIX PROTEIN GM130.//6.60E-136//363aa//76%//Q62839
C-OVARC1001952//TRICHOHYALIN.//3.30E-16//487aa//27%//Q07283
C-OVARC1001991//VOLTAGE-GATED POTASSIUM CHANNEL PROTEIN KV3.3 (KSHIIID).//8.30E-06//114aa//35%//Q01956
C-OVARC1002058//Human 18S rRNA gene, complete.//1.50E-164//921bp//91%//M10098
C-OVARC1002178
C-PLACE1000033//VON WILLEBRAND FACTOR PRECURSOR.//3.80E-17//190aa//28%//Q28295
C-PLACE1000231//Rattus norvegicus WAP four-disulfide core domain protein (ps20) mRNA, complete cds.//2.70E-101//947bp//74%//AF037272
C-PLACE1000258//ZINC FINGER PROTEIN 91 (ZINC FINGER PROTEIN HTF10) (HPF7).//1.70E-55//431aa//35%//Q05481
C-PLACE1000442//ZINC FINGER PROTEIN ZFP-36 (FRAGMENT).//1.00E-88//213aa//67%//P16415
C-PLACE1000560
C-PLACE1000740//Mus musculus (Notch2) mRNA, complete cds.//5.60E-122//893bp//81%//M93661
C-PLACE1000907//ZINC FINGER PROTEIN 83 (ZINC FINGER PROTEIN HPF1).//1.30E-204//396aa//85%//P51522
C-PLACE1000912
C-PLACE1000914//Homo sapiens PB39 mRNA, complete cds.//7.50E-88//500bp//69%//AF045584
C-PLACE1000927//HYPOTHETICAL 20.9 KD PROTEIN B0563.3 IN CHROMOSOME X.//6.30E-21//123aa//37%//Q11079
C-PLACE1000986//Homo sapiens mRNA: cDNA DKFZp586C1817 (from clone DKFZp586C1817).//0//2055bp//99%//AL117450
C-PLACE1001016//SODIUM CHANNEL PROTEIN PARA (PARALYTIC PROTEIN).//6.80E-12//133aa//28%//P35500
C-PLACE1001100//Homo sapiens nephrin (NPHS1) mRNA, complete cds//3.10E-46//323bp//84%//AF035835
C-PLACE1001114//HYPOTHETICAL BHLF1 PROTEIN.//9.20E-06//389aa//31%//P03181
C-PLACE1001123//INTESTINAL MEMBRANE A4 PROTEIN (DIFFERENTIATION-DEPENDENT PROTEIN A4).//5.OOE-08//95aa//31%//Q04941
C-PLACE1001183
C-PLACE1001229
C-PLACE1001231//Rattus norvegicus sodium-dependent multi-vitamin transporter (SMVT) mRNA, complete cds.//2.20E-137//918bp//80%//AF026554
C-PLACE1001340//MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).//1.20E-23//231aa//31%//P23231
C-PLACE1001401//HIGH AFFINITY IMMUNOGLOBULIN EPSILON RECEPTOR BETA-SUBUNIT (FCERI) (IGE FC RECEPTOR, BETA-SUBUNIT).//3.70E-18//148aa//39%//P13386
C-PLACE1001407
C-PLACE1001464//Human placental cDNA coding for 5' nucleotidase (EC 3.1.3.5).//0//2756bp//99%//X55740
C-PLACE1001500//Homo sapiens RecQ5 mRNA for DNA helicase, complete cds.//2.30E-271//1230bp//99%//AB006533
C-PLACE1001516//240 KD PROTEIN OF ROD PHOTORECEPTOR CNG-CHANNEL [CONTAINS: GLUTAMIC ACID-RICH PROTEIN (GARP); CYCLIC-NUCLEOTIDE-GATED CATION CHANNEL 4 (CNG CHANNEL 4) (CNG-4) (CYCLIC NUCLEOTIDE-GATED CATION CHANNEL MODULATORY SUBUNIT)].//2.30E-08//274aa//28%//Q28181
C-PLACE1001536
C-PLACE1001564//H.sapiens mRNA for HE6 Tm7 receptor.//5.10E-36//499bp//70%//X81892
C-PLACE1001655//Homo sapiens Shab-related delayed-rectifier K+ channel alpha subunit (KCNS3) mRNA, complete cds.//0//1708bp//99%//AF043472
C-PLACE1001788
C-PLACE1001795//HYPOTHETICAL 30.6 KD PROTEIN IN SCP160-SMC3 INTERGENIC REGION PRECURSOR.//3.40E-20//159aa//40%//P47032
C-PLACE1001836//ENV POLYPROTEIN PRECURSOR (COAT POLYPROTEIN) [CONTAINS: OUTER MEMBRANE PROTEIN GP70; TRANSMEMBRANE PROTEIN P20E].//5.00E-27//134aa//47%//P10269 C-PLACE1001918//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC
INTERMEDIATE COMPONENT).//2.30E-53//339aa//33%//P32802
C-PLACE1001949//PROBABLE CALCIUM-TRANSPORTING ATPASE 9 (EC 3.6.1.38).//3.00E-75//315aa//44%//Q12697
C-PLACE1002080//Homo sapiens antigen NY-CO-9 (NY-CO-9) mRNA, partial cds.//0//1588bp//99%//AF039691
C-PLACE1002095
C-PLACE1002153//Homo sapiens TACC2 protein (TACC2) mRNA, partial cds.//0//1202bp//99%//AF095791
C-PLACE1002329//EPIDERMAL GROWTH FACTOR RECEPTOR KINASE SUBSTRATE EPSB.//6.50E-105//213aa//45%//Q08509
C-PLACE1002355//COMPLEMENT C4 PRECURSOR [CONTAINS: C4A ANAPHYLATOXIN].//4.20E-12//131aa//40%//P01029
C-PLACE1002374//Human mRNA for pro-cathepsin L (major excreted protein MEP).//1.30E-313//1363bp//97%//X12451
C-PLACE1002518//Homo sapiens multiple membrane spanning receptor TRC8 (TRC8) mRNA, complete cds.//2.50E-14//396bp//64%//AF064801
C-PLACE1002547//MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).//2.30E-28//277aa//31%//P23231
C-PLACE1002726//Oryctolagus cuniculus mRNA for epithelial calcium channel (ECaC).//2.80E-202//926bp//82%//AJ133128
C-PLACE1002905//ENDOZEPINE-RELATED PROTEIN PRECURSOR (MEMBRANE-ASSOCIATED DIAZEPAM BINDING INHIBITOR) (MA-DBI).//2.40E-37//188aa//40%//P07106
C-PLACE1002911//POLIOVIRUS RECEPTOR HOMOLOG PRECURSOR.//4.50E-39//345aa//32%//P32507
C-PLACE1002967//HIGH AFFINITY IMMUNOGLOBULIN EPSILON RECEPTOR BETA-SUBUNIT (FCERI) (IGE FC RECEPTOR, BETA-SUBUNIT).//4.60E-08//156aa//30%//Q01362
C-PLACE1003135//SPORULATION-SPECIFIC PROTEIN 1 (EC 2.7.1.-).//1.30E-47//210aa//49%//P08458
C-PLACE1003163//Homo sapiens DBI-related protein mRNA, complete cds.//1.00E-294//1344bp//99%//AF069301
C-PLACE1003407//Homo sapiens putative transmembrane protein (CLN5) mRNA, complete cds.//0//1965bp//99%//AF068227
C-PLACE1003428//Homo sapiens mRNA for VNN1 protein.//1.80E-142//676bp//72%//AJ132099
C-PLACE1003438
C-PLACE1003460
C-PLACE1003529//DNA-DIRECTED RNA POLYMERASE II LARGEST SUBUNIT (EC 2.7.7.6) (RPB1) (FRAGMENT).//1.30E-09//281aa//22%//P11414
C-PLACE1003573//T-CELL SURFACE GLYCOPROTEIN YE1/48 (T LYMPHOCYTE ANTIGEN A1) (LY49-A ANTIGEN).//3.70E-16//226aa//26%//P20937
C-PLACE1003598//TRP-ASP REPEATS CONTAINING PROTEIN RBA-1.//1.80E-07//161aa//27%//P90917
C-PLACE1003644
C-PLACE1003737//TOLL PROTEIN PRECURSOR.//5.40E-07//203aa//27%//P08953
C-PLACE1003772//EXTENSIN PRECURSOR (CELL WALL HYDROXYPROLINE-RICH GLYCOPROTEIN).//2.40E-12//124aa//38%//P13983
C-PLACE1003839//Homo sapiens putative G protein-coupled receptor (RAIG1) mRNA, complete cds.//8.10E-18//771bp//58%//AF095448
C-PLACE1003845//PUTATIVE UDP-GLUCOSE 4-EPIMERASE (EC 5.1.3.2) (GALACTOWALDENASE) (UDP- GALACTOSE 4-EPIMERASE).//3.40E-37//302aa//30%//Q57664
C-PLACE1003852//Homo sapiens mRNA for KIAA0758 protein, partial cds.//0//1667bp//99%//AB018301
C-PLACE1004028
C-PLACE1004078//Bovine mRNA for adseverin, complete cds.//0//2218bp//89%//D26549
C-PLACE1004166//CREB-BINDING PROTE)N.//1.80E-12//147aa//35%//P45481
C-PLACE1004168//GENERAL NEGATIVE REGULATOR OF TRANSCRIPTION SUBUNIT 1.//9.10E-62//485aa//32%//P25655
C-PLACE1004199
C-PLACE1004279//HYPOTHETICAL 59.1 KD PROTEIN ZK637.1 IN CHROMOSOME III.//1.40E-08//166aa//30%//P30638
C-PLACE1004282//MONO- AND DIACYLGLYCEROL LIPASE PRECURSOR (EC 3.1.1.-) (MDGL).//2.10E-11//189aa//30%//P25234
C-PLACE1004305//RAS-RELATED PROTEIN RAC1.//9.60E-29//197aa//41%//P40792
C-PLACE1004441//Human G protein-coupled receptor (GPR1) gene, complete cds.//0//1880bp//98%//U13666
C-PLACE1004450//AMINOPEPTIDASE N (EC 3.4.11.2) (MICROSOMAL AMINOPEPTIDASE) (LEUKEMIA ANTIGEN CD13).//1.30E-91//562aa//35%//P15541
C-PLACE1004482//Rattus norvegicus hematopoietic lineage switch 2 related protein (Hls2-rp) mRNA, complete cds.//1.90E-246//1643bp//83%//AF097723 C-PLACE1004492//VERPROLIN.//3.30E-07//149aa//29%//P37370
C-PLACE1004519
C-PLACE1004520//Human pregnancy-specific beta-glycoprotein d mRNA, complete cds.//9.10E-279//882bp//88%//M20881
C-PLACE1004630//Homo sapiens ten integrin EGF-like repeat domains protein precursor (ITGBL1) mRNA, complete cds.//1.00E-138//643bp//99%//AF072752
C-PLACE1004637
C-PLACE1004648//GLUCOAMYLASE S1/S2 PRECURSOR (EC 3.2.1.3) (GLUCAN 1, 4-ALPHA-GLUCOSIDASE) (1,4-ALPHA-D-GLUCAN GLUCOHYDROLASE).//1.40E-18//395aa//25%//P08640
C-PLACE1004816//Homo sapiens mRNA for Hakata antigen, complete cds.//1.00E-166//856bp//94%//D88587
C-PLACE1004887//GOLIATH PROTEIN (G1 PROTEIN).//4.80E-33//179aa//47%//Q06003
C-PLACE1005003//PROSTASIN PRECURSOR (EC 3.4.21.-).//2.20E-52//269aa//41%//Q16651
C-PLACE1005005//Homo sapiens ubiquitin conjugating enzyme G2 (UBE2G2) mRNA, complete cds.//4.10E-261//1209bp//98%//AF032456
C-PLACE1005031//CHLORINE CHANNEL PROTEIN P64.//8.00E-92//205aa//87%//P35526 C-PLACE1005239//Homo sapiens mRNA for HIRIP3 protein (clone pH4-31, pH4-17).//1.80E-235//1010bp//84%//AJ223351
C-PLACE1005250//Homo sapiens D-type cyclin-interacting protein 1 (DIP1) mRNA, complete cds.//0//1046bp//96%//AF082569
C-PLACE1005383//Homo sapiens UP50 mRNA, complete cds.//0//2019bp//99%//AF093118
C-PLACE1005410//Canis familiaris sec61 homologue mRNA, complete cds.//2.40E-204//673bp//89%//M96629
C-PLACE1005426//Human pregnancy-specific beta 1-glycoprotein 4 (PSG4) clone FL-64 mRNA, complete cds.//0//1629bp//9596//U18469
C-PLACE1005519//Homo sapiens STE20-like kinase 3 (mst-3) mRNA, complete cds.//4.60E-108//1070bp//73%//AF024636
C-PLACE1005539//ACTIN POLYMERIZATION INHIBITOR (HEAT SHOCK 25 KD PROTEIN) (25-KD IAP).//3.10E-08//84aa//34%//Q00649
C-PLACE1005544//Mus musculus junctional adhesion molecule (Jam) mRNA, complete cds.//2.00E-159//1237bp//7696//U89915
C-PLACE1005569//Homo sapiens mRNA for Z39Ig protein.//0//1768bp//9996//AJ132502
C-PLACE1005601
C-PLACE1005660//HYPOTHETICAL 64.3 KD GTP-BINDING PROTEIN C02F5.3 IN CHROMOSOME III.//1.90E-33//143aa//53%//P34280
C-PLACE1005669
C-PLACE1005682//B-CELL LYMPHOMA 3-ENCODED PROTEIN (BCL-3 PROTEIN).//4.90E-09//183aa//33%//P20749
C-PLACE1005725//HYPOTHETICAL 55.1 KD PROTEIN B0416.5 IN CHROMOSOME X.//7.60E-17//295aa//27%//011073
C-PLACE1005736//Human mRNA for BAS-GRIP protein.//0//2378bp//99%//E16311 C-PLACE1005745//ORM1 PROTE)N.//2.40E-17//137aa//35%//P53224
C-PLACE1005768
C-PLACE1005815//COLORECTAL MUTANT CANCER PROTEIN (MCC PROTEIN).//1.50E-26//274aa//26%//P23508
C-PLACE1005878//Bovine chlorine channel protein (p64) mRNA, complete cds.//5.90E-137//889bp//85%//L16547
C-PLACE1005927//HYPOTHETICAL 35.8 KD PROTEIN C12G12.12 IN CHROMOSOME I.//1.60E-38//333aa//33%//Q09875
C-PLACE1006071//LAMININ BETA-1 CHAIN PRECURSOR (LAMININ B1 CHAIN).//6.00E-08//215aa//26%//P02469
C-PLACE1006073//Homo sapiens mRNA for glucuronyltransferase I, complete cds.//4.10E-316//1020bp//96%//AB009598
C-PLACE1006079//Homo sapiens distal-less homeobox protein (DLX3) gene, complete cds.//0//1379bp//97%//AF028233
C-PLACE1006093
C-PLACE1006208//Homo sapiens nGAP mRNA, complete cds.//3.30E-151//694bp//100%//AF047711
C-PLACE1006219//PUTATIVE UDP-GLUCOSE 4-EPIMERASE (EC 5.1.3.2) (GALACTOWALDENASE) (UDP- GALACTOSE 4-EPIMERASE).//3.50E-37//302aa//30%//Q57664
C-PLACE1006277//Homo sapiens mRNA for Z39Ig protein.//0//1768bp//99%//AJ132502
C-PLACE1006290//GLYCOSYLTRANSFERASE ALG2 (EC 2.4.1.-).//8.50E-75//301aa//39%//P43636
C-PLACE1006443//Homo sapiens PB39 mRNA, complete cds.//4.30E-98//553bp//70%//AF045584
C-PLACE1006515//Homo sapiens mRNA for KIAA0576 protein, partial cds.//0//2846bp//99%//AB011148
C-PLACE1006716//30 KD ADIPOCYTE COMPLEMENT-RELATED PROTEIN PRECURSOR (ACRP30) (ADIPOCYTE SPECIFIC PROTEIN ADIP0Q).//4.60E-25//181aa//35%//Q60994
C-PLACE1006786
C-PLACE1006809//SLS1 PROTEIN PRECURSOR.//9.10E-10//273aa//27%//P08124
C-PLACE1006959
C-PLACE1007028//Homo sapiens TDAG51/Ipl homologue 1 (TIH1) mRNA, complete cds.//1.40E-307//1423bp//99%//AF151100
C-PLACE1007040
C-PLACE1007077//Homo sapiens mRNA for KIAA0977 protein, complete cds.//0//2578bp//99%//AB023194
C-PLACE1007081//COAGULATION FACTOR V PRECURSOR (ACTIVATED PROTEIN C COFACTOR).//5.00E-20//247aa//34%//Q28107
C-PLACE1007096//PUTATIVE SUGAR TRANSPORT PROTEIN GUTA.//2.70E-17//174aa//27%//034368
C-PLACE1007296//Human mRNA for a presumptive KDEL receptor.//1.10E-185//1038bp//91%//X55885
C-PLACE1007591
C-PLACE1007626//Homo sapiens unknown mRNA, complete cds.//3.00E-246//1122bp//99%//AF047439
C-PLACE1007702//Mus musculus TRA1 mRNA, complete cds.//7.50E-41//662bp//64%//D78335
C-PLACE1007845//GLYCOSYLTRANSFERASE ALG2 (EC 2.4.1.-).//4.80E-14//158aa//40%//P43636
C-PLACE1007881//HYPOTHETICAL 69.4 KD PROTEIN F13H10.3 IN CHROMOSOME IV.//3.10E-99//504aa//42%//Q19425
C-PLACE1007971
C-PLACE1008282//HEME-REGULATED EUKARYOTIC INITIATION FACTOR EIF-2-ALPHA
KINASE (EC 2.7.1.-) (HR)).//7.1OE-274//627aa//82%//P33279
C-PLACE1008297//MYOSIN HEAVY CHAIN KINASE B (EC 2.7.1.129) (MHCK B).//1.30E-14//187aa//33%//P90648
C-PLACE1008359//BEM46 PROTEIN (FRAGMENT).//1.70E-50//289aa//42%//P54069
C-PLACE1008469
C-PLACE1008549//Homo sapiens Ets transcription factor ESE-2b mRNA, complete cds.//0//2274bp//99%//AF115403
C-PLACE1008657//Bovine mRNA for adseverin, complete cds.//7.80E-227//1246bp//90%//D26549
C-PLACE1008716//Human beta-1,2-N-acetylglucosaminyltransferase II (MGAT2) gene, complete cds.//0//1888bp//99%//U15128
C-PLACE1008744//P-SELECTIN PRECURSOR (GRANULE MEMBRANE PROTEIN 140) (GMP-140) (PADGEM) (CD62P) (LEUKOCYTE-ENDOTHELIAL CELL ADHESION MOLECULE 3) (LECAM3).//4.80E-32//338aa//30%//Q01102
C-PLACE1008984
C-PLACE1008985//Mus musculus synaptotagmin VIII mRNA, partial cds.//3.80E-140//650bp//81%//U20107
C-PLACE1009067
C-PLACE1009196
C-PLACE1009279//cDNA encoding novel physiologically active protein which have serine protease activity.//6.60E-86//1414bp//64%//E12965
C-PLACE1009527//Oryctolagus cuniculus mRNA for epithelial calcium channel (ECaC).//1.20E-87//585bp//83%//AJ133128
C-PLACE1009546
C-PLACE1009600//Mouse mRNA for tetracycline transporter-like protein, complete cds.//1.10E-264//924bp//88%//D88315
C-PLACE1009735
C-PLACE1009982//SALIVARY GLUE PROTEIN SGS-3 PRECURSOR.//5.20E-08//166aa//28%//P02840
C-PLACE1010011//Homo sapiens AAPT1-like protein mRNA, partial cds.//1.70E-237//1092bp//99%//AF047431
C-PLACE1010078//ORM1 PROTEIN.//3.70E-19//137aa//37%//P53224
C-PLACE1010081//Homo sapiens serine/threonine kinase RICK (RICK) mRNA, complete cds.//0//2033bp//99%//AF027706
C-PLACE1010251//FIBRILLIN 2 PRECURSOR.//1.70E-31//201aa//35%//Q61555
C-PLACE1010445
C-PLACE1010713//Homo sapiens steroid dehydrogenase homolog mRNA, complete cds.//0//2374bp//99%//AF078850
C-PLACE1010784//Homo sapiens orphan G protein-coupled receptor (GPR34) gene, complete cds.//2.30E-252//1146bp//99%//AF039686
C-PLACE1010827//COP-COATED VESICLE MEMBRANE PROTEIN P24 PRECURSOR (FRAGMENT).//1.90E-19//163aa//34%//P49020
C-PLACE1010968//PROTEIN-TYROSINE PHOSPHATASE DLAR PRECURSOR (EC 3.1.3.48) (PROTEIN- TYROSINE-PHOSPHATE PHOSPHOHYDROLASE).//3.40E-30//690aa//26%//P16621 C-PLACE1011045//Homo sapiens E1-like protein mRNA, complete cds.//0//2376bp//99%//AF094516
C-PLACE1011116//GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).//3.30E-09//234aa//27%//P08640
C-PLACE1011181//MSP1 PROTEIN HOMOLOG.//9.40E-82//353aa//47%//P54815
C-PLACE1011236//Mus musculus mRNA for RST, complete cds.//1.70E-90//1398bp//65%//AB005451
C-PLACE1011364//MYOTONIN-PROTEIN KINASE (EC 2.7.1.-) (MYOTONIC DISTROPHY
PROTEIN KINASE) (MDPK) (DM-KINASE) (DMK) (DMPK) (MT-PK).//2.20E-09//153aa//32%//Q09013
C-PLACE1011407//ZINC FINGER PROTEIN 184 (FRAGMENT).//1.80E-133//342aa//59%//Q99676
C-PLACE1011516//HYPOTHETICAL 21.5 KD PROTEIN IN SEC15-SAP4 INTERGENIC REGION.//1.30E-13//139aa//34%//P53073
C-PLACE1011708//Homo sapiens intrinsic factor-B12 receptor precursor, mRNA, complete cds.//0//1840bp//98%//AF034611
C-PLACE1011824//Human Ste20-like kinase (MST2) mRNA, complete cds.//6.40E-202//561bp//92%//U26424
C-PLACE1011978//ZINC FINGER PROTEIN 91 (ZINC FINGER PROTEIN HTF10) (HPF7).//1.10E-194//547aa//57%//Q05481
C-PLACE2000118//WISKOTT-ALDRICH SYNDROME PROTEIN HOMOLOG (WASP).//3.20E-27//205aa//43%//P70315
C-PLACE2000219
C-PLACE3000181//Human protocadherin 42 mRNA, complete cds for abbreviated PC42.//0//2719bp//95%//L11370
C-PLACE3000213//COMPLEMENT RECEPTOR TYPE 2 PRECURSOR (CR2) (COMPLEMENT C3D RECEPTOR).//4.60E-68//317aa//34%//P19070
C-PLACE4000354//COMPLEMENT RECEPTOR TYPE 1 PRECURSOR (C3B/C4B RECEPTOR) (CD35 ANTIGEN).//1.00E-129//482aa//29%//P17927
C-PLACE4000455
C-SKNMC1000004
C-SKNMC1000014
C-SKNMC1000082//BRITTLE-1 PROTEIN PRECURSOR.//3.70E-31//307aa//30%//P29518
C-THYRO1000036//Homo sapiens collagen alpha 3 type IX (COL9A3) mRNA, complete cds.//1.20E-258//1376bp//93%//L41162
C-THYRO1000061//Mus musculus mRNA for UBE-1c1, UBE-1c2, UBE-1c3, complete cds.//1.40E-117//1126bp//74%//AB030505
C-THYRO1000099
C-THYRO1000196//Homo sapiens Ksp-cadherin (CDH16) mRNA, complete cds.//0//1632bp//91%//AF016272
C-THYRO1000400//Homo sapiens mRNA full length insert cDNA clone EUROIMAGE 25795.//0//1893bp//99%//AL109665
C-THYRO1000580//ZINC FINGER PROTEIN 184 (FRAGMENT).//9.90E-114//279aa//59%//Q99676
C-THYRO1000584//EPIDIDYMIS-SPECIFIC ALPHA-MANNOSIDASE (EC 3.2.1.24) (ALPHA-D-MANNOSIDE MANNOHYDROLASE) (135 KD PROTEIN).//5.40E-127//335aa//71%//Q28949
C-THYRO1000678//Homo sapiens Cx30 gene.//0//1741bp//97%//AJ005585
C-THYRO1000776//PROBABLE SULFATE PERMEASE SPBC3H7.02.//1.30E-68//442aa//36%//074377
C-THYRO1000795//C.elegans mRNA for Oxoglutarate/malate carrier protein.//8.80E-42//821bp//63%//X76114
C-THYRO1000846
C-THYRO1000866//SHK1 KINASE-BINDING PROTEIN 1.//4.40E-91//449aa//44%//P78963
C-THYRO1000956//Human G protein-coupled receptor APJ gene, complete cds.//0//1583bp//9996//U03642
C-THYRO1000964//Drosophila melanogaster Pelle associated protein Pellino (Pli) mRNA, complete cds.//1.10E-34//759bp//63%//AF091624
C-THYRO1000999
C-THYRO1001063
C-THYRO1001071//GAMMA-ADAPTIN (GOLGI ADAPTOR HA1/AP1 ADAPTIN GAMMA SUBUNIT) (CLATHRIN ASSEMBLY PROTEIN COMPLEX 1 GAMMA LARGE CHAIN).//8.20E-14//157aa//33%//P22892
C-THYRO1001102
C-THYRO1001113//Homo sapiens mRNA: cDNA DKFZp564E1616 (from clone DKFZp564E1616).//0//1361bp//9996//AL096713
C-THYRO1001128//Homo sapiens mRNA for hypothetical protein (C9orf9 gene).//6.40E-155//648bp//99%//AJ011375
C-THYRO1001205
C-THYRO1001237//PHYTOENE DEHYDROGENASE (EC 1.3.-.-) (PHYTOENE DESATURASE) (ALBINO-1 PROTE)N).//3.10E-13//346aa//22%//P21334
C-THYRO1001242//Homo sapiens cleft lip and palate transmembrane protein 1 (CLPTM1) mRNA, complete cds.//0//2468bp//99%//AF037339
C-THYRO1001266//Human sodium iodide symporter mRNA, complete cds.//7.20E-81//1466bp//62%//U66088
C-THYRO1001327
C-THYRO1001456//HYPOTHETICAL 56.2 KD PROTEIN CY31.25C.//9.40E-32//355aa//31%//Q10555
C-THYRO1001457//H.sapiens mRNA for protein kinase C mu.//2.30E-218//1183bp//73%//X75756
C-THYRO1001471
C-THYRO1001478//CYTOCHROME B-245 HEAVY CHAIN (P22 PHAGOCYTE B-CYTOCHROME) (NEUTROPHIL CYTOCHROME B, 91 KD POLYPEPTIDE) (CGD91-PHOX) (GP91-PHOX) (CYTOCHROME B(558) BETA CHAIN) (SUPEROXIDE-GENERATING NADPH OXIDASE HEAVY CHAIN SUBUN)T).//8.90E-50//296aa//35%//P04839
C-THYRO1001495
C-THYRO1001523//Homo sapiens mRNA for TM7XN1 protein.//0//3663bp//99%//AJ011001
C-THYRO1001529//SERINE PALMITOYLTRANSFERASE 2 (EC 2.3.1.50) (LONG CHAIN BASE BIOSYNTHESIS PROTEIN 2) (SPT 2).//5.50E-25//115aa//53%//Q09925
C-THYRO1001593//PUTATIVE SERINE/THREONINE-PROTEIN KINASE P78 (EC 2.7.1.).//3.10E-203//550aa//62%//P27448
C-THYRO1001608
C-THYRO1001641//Homo sapiens CGI-57 protein mRNA, complete cds.//0//1668bp//99%//AF151815
C-THYRO1001700//SERINE/THREONINE PROTEIN KINASE RIP (EC 2.7.1.-) (CELL DEATH PROTEIN RIP) (RECEPTOR INTERACTING PROTEIN).//9.70E-33//268aa//37%//Q60855
C-THYRO1001702//Mus musculus mRNA for myeloid associated differentiation protein.//1.50E-128//1204bp//73%//AJ001616
C-THYRO1001725
C-THYRO1001770//PROBABLE SERINE/THREONINE-PROTEIN KINASE YNL020C (EC 2.7.1.-).//6.30E-20//169aa//35%//P53974
C-THYRO1001803
C-Y79AA1000030//Homo sapiens mRNA for Fe65L2, complete cds.//0//1828bp//100%//AB018247
C-Y79AA1000127
C-Y79AA1000207
C-Y79AA1000226
C-Y79AA1000270//Bos taurus vacuolar H+ ATPase subunit Ac45 mRNA, complete cds.//1.00E-271//1490bp//83%//U10039
C-Y79AA1000426//Mus musculus activin beta E subunit mRNA, complete cds.//7.70E-200//1533bp//78%//U96386
C-Y79AA1000521
C-Y79AA1000750
C-Y79AA1000776//Mus musculus mRNA for GSG1, complete cds.//2.40E-161//820bp//85%//D87325
C-Y79AA1000777//VEGETATIBLE INCOMPATIBILITY PROTEIN HET-E-1.//8.10E-48//283aa//38%//Q00808
C-Y79AA1000876//PROTEIN DISULFIDE ISOMERASE-RELATED PROTEIN PRECURSOR (ERP72).//1.60E-44//210aa//38%//P13667
C-Y79AA1000888//TRNA PSEUDOURIDINE SYNTHASE A (EC 4.2.1.70) (PSEUDOURIDYLATE SYNTHASE I) (PSEUDOURIDINE SYNTHASE I) (URACIL HYDROLYASE).//1.50E-21//267aa//32%//P70973
C-Y79AA1000959//Homo sapiens Hsp70 binding protein HspBP1 mRNA, complete cds.//4.80E-283//1405bp//95%//AF093420
C-Y79AA1000967//CALCIUM/CALMODULIN-DEPENDENT PROTEIN KINASE TYPE I (EC 2.7.1.123) (CAM KINASE I).//1.00E-77//359aa//44%//Q14012
C-Y79AA1001013
C-Y79AA1001056//Homo sapiens MAID protein mRNA, complete cds.//0//1475bp//99%//AF113535
C-Y79AA1001062//TUMOR NECROSIS FACTOR, ALPHA-INDUCED PROTEIN 1, ENDOTHELIAL (B12 PROTEIN).//8.90E-12//132aa//38%//013829
C-Y79AA1001090//NUCLEAR FACTOR NF-KAPPA-B P105 SUBUNIT (DNA-BINDING FACTOR KBF1) (EBP- 1) (NF-KAPPA-B1 P84 / NF-KAPPA-B1 P98) [CONTAINS: NUCLEAR FACTOR NF- KAPPA-B P50 SUBUNIT] (FRAGMENT).//4.50E-09//144aa//31%//Q63369
C-Y79AA1001212//Homo sapiens SL15 protein mRNA, complete cds.//6.30E-306//1388bp//99%//AF038961
C-Y79AA1001264//HYPOTHETICAL 39.9 KD PROTEIN T15H9.1 IN CHROMOSOME II PRECURSOR.//5.10E-106//351aa//58%//Q10005
C-Y79AA1001272//Homo sapiens retinoic acid repressible protein (RARG-1) mRNA, complete cds.//1.50E-183//867bp//98%//AF172066
C-Y79AA1001328//Mus musculus mRNA for DII3 protein, complete cds.//1.90E-263//1988bp//79%//AB013440
C-Y79AA1001426//ANION EXCHANGE PROTEIN 3 (CARDIAC/BRAIN BAND 3-LIKE PROTEIN) (CAE3/BAE3).//6.20E-66//609aa//31%//P48751
C-Y79AA1001427//Homo sapiens cytochrome b5 reductase 1 (B5R.1) mRNA, complete cds.//0//1588bp//99%//AF169481
C-Y79AA1001430//Homo sapiens mRNA for KIAA0469 protein, complete cds.//0//2943bp//99%//AB007938
C-Y79AA1001523//Homo sapiens transcriptional intermediary factor 1 alpha mRNA, complete cds.//0//2263bp//99%//AF119042
C-Y79AA1001530//Human beta-tubulin gene (5-beta) with ten Alu family members.//0//1920bp//98%//X00734
C-Y79AA1001592
C-Y79AA1001727//CELL SURFACE A33 ANTIGEN PRECURSOR.//1.10E-13//286aa//27%//Q99795
C-Y79AA1001787//PROBABLE CALCIUM-TRANSPORTING ATPASE 9 (EC 3.6.1.38).//1.70E-133//544aa//37%//Q12697
C-Y79AA1001793//Mus musculus mRNA for GSG1, complete cds.//3.70E-126//532bp//78%//D87325
C-Y79AA1001795//Homo sapiens mRNA for GaIT4 protein.//2.30E-250//1137bp//99%//Y15061
C-Y79AA1001799//MITOCHONDRIAL RNA SPLICING PROTEIN MSR4.//3.40E-54//182aa//39%//P23500
C-Y79AA1001803//Homo sapiens secretogranin III mRNA, complete cds.//0//1871bp//99%//AF078851
C-Y79AA1001863
C-Y79AA1002022//POLIOVIRUS RECEPTOR HOMOLOG PRECURSOR.//2.20E-06//140aa//26%//P32507
C-Y79AA1002058//Mus musculus Gng3Ig mRNA, complete cds.//4.10E-167//1145bp//83%//AF069954
C-Y79AA1002121//HISTONE H1.//4.90E-12//114aa//35%//P35060
C-Y79AA1002129
C-Y79AA1002213//HYPOTHETICAL 52.7 KD PROTEIN C38C10.2 IN CHROMOSOME III.//1.20E-98//262aa//41%//Q03567
C-Y79AA1002334//GLUCOSE REPRESSION MEDIATOR PROTEIN.//1.70E-10//333aa//23%//P14922
C-Y79AA1002373//Mus musculus mRNA for GSG1, complete cds.//7.20E-147//680bp//79%//D87325
C-Y79AA1002376//Rattus norvegicus cytoplasmic dynein intermediate chain 2B mRNA, complete cds.//1.50E-304//1667bp//90%//U39045
C-Y79AA1002378//Homo sapiens zinc finger protein NY-REN-21 antigen mRNA, partial cds.//0//963bp//99%//AF155100
C-Y79AA1002381//Homo sapiens cell cycle related kinase mRNA, complete cds.//0//1791bp//98%//AF035013

## Claims

1. Use of an oligonucleotide as a primer for synthesizing the polynucleotide comprising the nucleotide sequence set forth in SEQ ID NO: 189, or the complementary strand thereof, wherein said oligonucleotide is complementary to said polynucleotide or the complementary strand thereof and comprises at least 15 nucleotides.

2. A primer set for synthesizing polynucleotides, the primer set comprising an oligo-dT primer and an oligonucleotide complementary to the complementary strand of the polynucleotide comprising the nucleotide sequence set forth in SEQ ID NO: 189, wherein said oligonucleotide comprises at least 15 nucleotides.

3. A primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence / 3'-end nucleotide sequence is selected from the group consisting of SEQ ID NO:189 and SEQ ID NO:1007.

4. A polynucleotide which can be synthesized with the primer set of claim 2 or 3.

5. A polynucleotide comprising a coding region in the polynucleotide of claim 4.

6. A protein encoded by polynucleotide of claim 4.

7. A partial peptide of the protein of claim 6.

8. A polynucleotide selected from the group consisting of
(a) a polynucleotide comprising a coding region of the nucleotide sequence set forth in SEQ ID NO:3704;
(b) a polynucleotide comprising a nucleotide sequence encoding a protein comprising the amino acid sequence set forth in SEQ ID NO:3705;
(c) a polynucleotide comprising a nucleotide sequence encoding a protein comprising an amino acid sequence selected from the amino acid sequences of (b), in which one or more amino acids are substituted, deleted, inserted, and/or added, wherein said protein is functionally equivalent to the protein comprising said amino acid sequence selected from the amino acid sequences of (b);
(d) a polynucleotide that hybridizes with a polynucleotide comprising a nucleotide sequence selected from the nucleotide sequences of (a), and that comprises a nucleotide sequence encoding a protein functionally equivalent to the protein encoded by the nucleotide sequence selected from the nucleotide sequences of (a);
(e) a polynucleotide comprising a nucleotide sequence encoding a partial amino acid sequence of a protein encoded by the polynucleotide of (a) to (d); and
(f) a polynucleotide comprising a nucleotide sequence with at least 70% identity to the nucleotide sequence of (a).

9. A protein encoded by the polynucleotide of claim 8.

10. An antibody against the protein or peptide of any one of claims 6, 7, and 9.

11. A vector comprising the polynucleotide of claim 5 or 8.

12. A transformant carrying the polynucleotide of claim 5 or 8, or the vector of claim 11.

13. A transformant expressively carrying the polynucleotide of claim 5 or 8, or the vector of claim 11.

14. A method for producing the protein or peptide of any one of claims 6, 7, and 9, comprising culturing the transformant of claim 13 and recovering the expression product.

15. An oligonucleotide comprising the nucleotide sequence of claim 8 (a) or the nucleotide sequence complementary to the complementary strand thereof, wherein said oligonucleotide comprises 15 nucleotides or more.

16. Use of the oligonucleotide of claim 15 as a primer for synthesizing a polynucleotide.

17. Use of the oligonucleotide of claim 15 as a probe for detecting a gene.

18. An antisense polynucleotide against the polynucleotide of claim 8, or the portion thereof.

19. A method for synthesizing a polynucleotide, the method comprising:
a) synthesizing a complementary strand using a cDNA library as a template, and using the primer set of claim 2 or 3, or the primer of claim 16; and
b) recovering the synthesized product.

20. The method of claim 19, wherein the cDNA library is obtainable by oligo-capping method.

21. The method of claim 19, wherein the complementary strand is obtainable by PCR.

22. A method for detecting the polynucleotide of claim 8, the method comprising:
a) incubating a target polynucleotide with the oligonucleotide of claim 15 under the conditions where hybridization occurs, and
b) detecting the hybridization of the target polynucleotide with the oligonucleotide of claim 15.

23. A database of polynucleotides and/or proteins, the database comprising information on at least one sequence selected from the nucleotide sequences of claim 8 (a) and/or the amino acid sequences of claim 8 (b), or a medium on which the database is stored.
